(19) Europäisches Patentamt
European Patent Office
Office européen des brevets

(11) EP 3 438 282 A1

(12) EUROPEAN PATENT APPLICATION
published in accordance with Art. 153(4) EPC

(43) Date of publication:
**06.02.2019 Bulletin 2019/06**

(21) Application number: **17775147.6**

(22) Date of filing: **28.03.2017**

(51) Int Cl.:
***C12Q 1/68*** *(2018.01)* ***C12N 15/09*** *(2006.01)*
***G01N 33/15*** *(2006.01)* ***G01N 33/50*** *(2006.01)*
***G01N 33/53*** *(2006.01)*

(86) International application number:
**PCT/JP2017/012761**

(87) International publication number:
**WO 2017/170610 (05.10.2017 Gazette 2017/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **29.03.2016 JP 2016066696**
**31.01.2017 JP 2017015821**

(71) Applicant: **Advanced Telecommunications Research Institute International**
**Soraku-gun**
**Kyoto 619-0288 (JP)**

(72) Inventor: **SATO, Narutoku**
**Soraku-gun**
**Kyoto 619-0288 (JP)**

(74) Representative: **Vossius & Partner**
**Patentanwälte Rechtsanwälte mbB**
**Siebertstrasse 3**
**81675 München (DE)**

# (54) SCREENING METHOD FOR CANDIDATE SUBSTANCES FOR ACTIVE COMPONENT TO PREVENT OR TREAT AT LEAST ONE DISEASE SELECTED FROM THE GROUP CONSISTING OF RENAL HYPOFUNCTION, CHRONIC KIDNEY DISEASE AND KIDNEY FAILURE

(57) This invention provides a device for screening a candidate substance for an active ingredient to prevent or treat declined kidney function etc.

A screening device 1 for screening a candidate substance for an active ingredient to prevent or treat declined kidney function etc. comprises a first measurement value obtaining unit 11 for obtaining a measurement value of a kidney function prediction marker protein and/or a measurement value of mRNA of the protein in a test-substance-treated specimen, and a candidate substance determination unit 14 for determining that the test substance is a candidate substance for the active ingredient based on the measurement value(s) obtained by the first measurement value obtaining unit 11.

EP 3 438 282 A1

## Description

Technical Field

The present invention relates to a method and device for screening a candidate substance for an active ingredient to prevent or treat at least one disease selected from the group consisting of declined kidney function, chronic kidney disease, and renal failure. The present invention also relates to a program that causes a computer to perform a function for screening a candidate substance for an active ingredient to prevent or treat at least one disease selected from the group consisting of declined kidney function, chronic kidney disease, and renal failure.

Furthermore, the present invention relates to a device and program for predicting the presence of at least one disease selected from the group consisting of declined kidney function, chronic kidney disease, and renal failure, and/or the stage of the disease in a subject. The present invention also relates to a device and program for predicting the presence of a disease and/or the stage of the disease in each of one or more organs other than the kidneys in a subject affected with at least one disease selected from the group consisting of declined kidney function, chronic kidney disease, and renal failure.

Background Art

Diseases include those in a state that can be reversibly treated, and those in a state that cannot, i.e., those in an irreversible state. Early detection and treatment of abnormalities during a reversible state, or preventing such a state from occurring, is essential for health maintenance. Even in a reversible state, early detection of disease directly leads to milder treatment, a shorter treatment period, and better prognostic health. As in heart disease, brain disease, cancer, and diabetes, it is well known that abnormalities in one organ or tissue lead to a disease state in other organs (commonly called a "complication"). In such diseases, it is essential to prevent, at the earliest possible time, abnormalities in one organ or tissue from causing disease in other organs or tissue.

In all animals, including humans, each organ and tissue form a functional network, rather than serving as separate parts, and quality control at the individual level is achieved. Transport of endocrine factors, such as hormones, by the vascular network throughout the entire body and coordinated adjustment of organ functions by the neural network are typical examples of an "inter-organ cross talk system," and systematized as physiology or endocrinology.

Meanwhile, the number of end-stage kidney disease (ESKD) patients in need of dialysis or kidney transplant has been increasing worldwide. The number of ESKD patients increased from 430,000 to 1,065,000 over the decade from 1990 to 2000, and further increased to at least about 1,650,000 in 2008 (Non-patent Literature 1). Chronic kidney disease (CKD) progresses to ESKD. However, in the kidneys, called the "silent organ," even if kidney damage occurs, its condition is less likely to appear in clinical data etc. Thus, early detection of declined kidney function before onset of chronic kidney disease is difficult.

Citation List

Non-patent Literature

NPL 1: Lysaght MJ: J Am Soc Nephrol. 2002 Jan; 13 Suppl 1; S37-40.

Summary of Invention

Technical Problem

CKD develops due to various diseases such as diabetes, hypertension, and like lifestyle-related diseases; and urinary tract infection, urinary tract obstruction, glomerulonephritis, vascular disease in the kidneys (blood flow disorder), drug-induced nephropathy caused by an analgesic, and like urinary system diseases. Thus, after kidney function has declined, treatment of such a primary disease is first performed in order to slow progression of the declined kidney function. In addition, treatment such as blood-pressure control or dietary restriction is conducted to slow progression of CKD. Further, for example, a drug therapy using a phosphate-binding agent etc. is performed for abnormal bone metabolism associated with chronic kidney disease when CKD progresses.

However, there is currently no fundamental therapeutic agent that halts progression of declined kidney function.

An objective of the present invention is to provide a method and device for screening a candidate substance for an active ingredient to prevent or treat at least one disease selected from the group consisting of declined kidney function, chronic kidney disease, and renal failure. Another object of the present invention is to provide a pharmaceutical composition for suppressing progression of declined kidney function or improving declined kidney function, and preventing or treating at least one disease selected from the group consisting of declined kidney function, chronic kidney disease, and renal failure.

A further object of the present invention is to provide a device and program for detecting, from cells or tissue of one organ other than the kidneys, at least one disease selected from the group consisting of declined kidney function, chronic kidney disease, and renal failure at the earliest possible time. More specifically, a further object of the present invention is to predict the presence of at least one disease selected from the group consisting of declined kidney function, chronic kidney disease, and renal failure, and/or the stage of the disease, from an inter-organ cross talk indicator derived from one or more organs other than the kidneys. A still further object of the present invention is to predict the presence of a disease and/or the stage of the disease in each of one or more organs other than the kidneys from the state of at least one disease selected from the group consisting of declined kidney function, chronic kidney disease, and renal failure.

Solution to Problem

The inventor conducted extensive research, and found that the expression of certain kidney function prediction marker proteins increases in an animal model of kidney disease. The inventor found that progression of declined kidney function can be suppressed, or that declined kidney function can be improved, by suppressing function of such a kidney function prediction marker protein. The inventor further found that whether a test substance can be a candidate substance for an active ingredient to prevent or treat at least one disease selected from the group consisting of declined kidney function, chronic kidney disease, and renal failure can be screened by observing changes in expression or function of such a kidney function prediction marker protein in a subject to which the test subject has been administered.

The present inventor also focused on an inter-organ cross talk system to achieve the above objects. The inventor conducted extensive research, and found that it is possible to provide a device and program for diagnosing the state of at least one disease selected from the group consisting of declined kidney function, chronic kidney disease, and renal failure from the state of an organ other than the kidneys; and for predicting a future state by using the inter-organ cross talk system.

The present invention has been accomplished based on these findings, and includes the following embodiments.

Item 1. A device for screening a candidate substance for an active ingredient to prevent or treat at least one disease selected from the group consisting of declined kidney function, chronic kidney disease, and renal failure, the device comprising the following computation means:

first measurement value obtaining means for obtaining a measurement value of a kidney function prediction marker protein and/or a measurement value of mRNA of the protein in a specimen collected from a subject (excluding humans), test tissue, or test cell treated with a test substance (a test-substance-treated specimen); and

means for determining that the test substance is a candidate substance for the active ingredient based on the measurement value(s) obtained by the first measurement value obtaining means.

Item 2. The device according to Item 1, further comprising:

second measurement value obtaining means for obtaining a measurement value of the kidney function prediction marker protein and/or a measurement value of mRNA of the protein in a specimen collected from a subject (excluding humans), test tissue, or test cell that is not treated with the test substance (an untreated specimen); and

means for comparing the measurement value(s) of the test-substance-treated specimen with the measurement value(s) of the untreated specimen,

wherein the determination means determines that the test substance is a candidate substance for the active ingredient based on the comparison result obtained by the measurement value comparison means.

Item 3. The device according to Item 1 or 2, wherein the kidney function prediction marker protein is at least one member selected from the group consisting of Aplnr, Spp1, Dnase1, Slco1a1, Anpep, Slc7a8, Oscar, proline-rich proteins, defensins, fibrinogens, and Hamp/Hepcidin.

Item 4. A screening program that, when executed by a computer, causes the computer to carry out the following processing to screen a candidate substance for an active ingredient to prevent or treat at least one disease selected from the group consisting of declined kidney function, chronic kidney disease, and renal failure:

first measurement value obtaining processing of obtaining a measurement value of a kidney function prediction marker protein and/or a measurement value of mRNA of the protein in a specimen collected from a subject (excluding humans), test tissue, or test cell treated with a test substance (a test-substance-treated specimen);

and

processing of determining that the test substance is a candidate substance for the active ingredient based on the measurement value(s) obtained by the first measurement value obtaining processing.

Item 5. The screening program according to Item 4, wherein the program further causes the computer to carry out second

measurement value obtaining processing of obtaining a measurement value of the kidney function prediction marker protein and/or a measurement value of mRNA of the protein in a specimen collected from a subject (excluding humans), test tissue, or test cell that is not treated with the test substance (an untreated specimen); and processing of comparing the measurement value(s) of the test-substance-treated specimen with the measurement value(s) of the untreated specimen, and

in the determination processing, it is determined that the test substance is a candidate substance for the active ingredient based on the comparison result obtained by the measurement value comparison processing.

Item 6. The screening program according to Item 4 or 5, wherein the kidney function prediction marker protein is at least one member selected from the group consisting of Aplnr, Spp1, Dnase1, Slco1a1, Anpep, Slc7a8, Oscar, proline-rich proteins, defensins, fibrinogens, and Hamp/Hepcidin.

Item 7. A method for screening a candidate substance for an active ingredient to prevent or treat at least one disease selected from the group consisting of declined kidney function, chronic kidney disease, and renal failure, the method comprising the steps of:

(I) obtaining a measurement value of a kidney function prediction marker protein and/or a measurement value of mRNA of the protein in a specimen collected from a subject (excluding humans), test tissue, or test cell treated with a test substance (a test-substance-treated specimen); and

(II) determining that the test substance is a candidate substance for the active ingredient based on the measurement value(s) obtained in step (I).

Item 8. The method according to Item 7, further comprising, between steps (I) and (II), the step of comparing the measurement value(s) of the test-substance-treated specimen obtained in step (I) with a measurement value of a corresponding kidney function prediction marker protein and/or a measurement value of mRNA of the protein in a specimen collected from a subject (excluding humans), test tissue, or test cell that is not treated with the test substance (an untreated specimen).

Item 9. The method according to Item 7 or 8, comprising, before step (I), the steps of:

(i) treating the subject (excluding humans), test tissue, or test cell with the test substance;

(ii) collecting the specimen from the subject, test tissue, or test cell treated with the test substance in step (i); and

(iii) collecting the protein and/or the mRNA from the specimen obtained in step (ii).

Item 10. The method according to any one of Items 7 to 9, wherein the kidney function prediction marker protein is at least one member selected from the group consisting of Aplnr, Spp1, Dnase1, Slco1a1, Anpep, Slc7a8, Oscar, proline-rich proteins, defensins, fibrinogens, and Hamp/Hepcidin.

Item 11. A method for screening a candidate substance for an active ingredient to prevent or treat at least one disease selected from the group consisting of declined kidney function, chronic kidney disease, and renal failure, the method comprising the steps of:

(A) detecting a kidney function prediction marker protein and/or mRNA of the protein in a specimen collected from a subject (excluding humans), test tissue, or test cell treated with a test substance (a test-substance-treated specimen); and

(B) determining that the test substance is a candidate substance for the active ingredient based on the result obtained in step (A).

Item 12. The method according to Item 11, further comprising, between steps (A) and (B), the step of comparing the detection result of the test-substance-treated specimen obtained in step (A) with a detection result of a corresponding kidney function prediction marker protein and/or mRNA of the protein in a specimen collected from a subject (excluding humans), test tissue, or test cell that is not treated with the test substance (an untreated specimen).

Item 13. The method according to Item 11 or 12, wherein the kidney function prediction marker protein is at least one member selected from the group consisting of Aplnr, Spp1, Dnase1, Slco1a1, Anpep, Slc7a8, Oscar, proline-rich proteins, defensins, fibrinogens, and Hamp/Hepcidin.

Item 14. A device for screening a candidate substance for an active ingredient to prevent or treat at least one disease selected from the group consisting of declined kidney function, chronic kidney disease, and renal failure, the device comprising the following computation means:

first evaluation result obtaining means for obtaining an evaluation result of function of a kidney function prediction marker protein in a specimen collected from a subject (excluding humans), test tissue, or test cell treated with a test substance (a test-substance-treated specimen); and
means for determining that the test substance is a candidate substance for the active ingredient based on the evaluation result obtained by the first evaluation result obtaining means.

Item 15. The device according to Item 14, further comprising:

second evaluation result obtaining means for obtaining an evaluation result of function of the kidney function prediction marker protein in a specimen collected from a subject (excluding humans), test tissue, or test cell that is not treated with the test substance (an untreated specimen); and
means for comparing the evaluation result of the test-substance-treated specimen with the evaluation result of the untreated specimen,
wherein the determination means determines that the test substance is a candidate substance for the active ingredient based on the comparison result obtained by the evaluation result comparison means.

Item 16. The device according to Item 14 or 15, wherein the kidney function prediction marker protein is at least one member selected from the group consisting of Aplnr, Spp1, Dnase1, Slco1a1, Anpep, Slc7a8, Oscar, proline-rich proteins, defensins, fibrinogens, and Hamp/Hepcidin.
Item 17. A screening program that, when executed by a computer, causes the computer to carry out the following processing to screen a candidate substance for an active ingredient to prevent or treat at least one disease selected from the group consisting of declined kidney function, chronic kidney disease, and renal failure:

first evaluation result obtaining processing of obtaining an evaluation result of function of a kidney function prediction marker protein in a specimen collected from a subject (excluding humans), test tissue, or test cell treated with a test substance (a test-substance-treated specimen); and
processing of determining that the test substance is a candidate substance for the active ingredient based on the evaluation result obtained by the first evaluation result obtaining processing.

Item 18. The screening program according to Item 17, wherein the program further causes the computer to carry out second evaluation result obtaining processing of obtaining an evaluation result of function of the kidney function prediction marker protein in a specimen collected from a subject (excluding humans), test tissue, or test cell that is not treated with the test substance (an untreated specimen); and processing of comparing the evaluation result of the test-substance-treated specimen with the evaluation result of the untreated specimen, and
in the determination processing, it is determined that the test substance is a candidate substance for the active ingredient based on the comparison result obtained by the evaluation result comparison processing.
Item 19. The screening program according to Item 17 or 18, wherein the kidney function prediction marker protein is at least one member selected from the group consisting of Aplnr, Spp1, Dnase1, Slco1a1, Anpep, Slc7a8, Oscar, proline-rich proteins, defensins, fibrinogens, and Hamp/Hepcidin.
Item 20. A method for screening a candidate substance for an active ingredient to prevent or treat at least one disease selected from the group consisting of declined kidney function, chronic kidney disease, and renal failure, the method comprising the steps of:

(I) evaluating function of a kidney function prediction marker protein in a specimen collected from a subject (excluding humans), test tissue, or test cell treated with a test substance (a test-substance-treated specimen); and
(II) determining that the test substance is a candidate substance for the active ingredient based on the evaluation result obtained in step (I).

Item 21. The method according to Item 20, further comprising, between steps (I) and (II), the step of comparing the evaluation result of the test-substance-treated specimen obtained in step (I) with an evaluation result of function of a corresponding kidney function prediction marker protein in a specimen collected from a subject (excluding humans), test tissue, or test cell that is not treated with the test substance (an untreated specimen).
Item 22. The method according to Item 20 or 21, comprising, before step (I), the steps of:

(i) treating the subject (excluding humans), test tissue, or test cell with the test substance; and
(ii) collecting the specimen from the subject, test tissue, or test cell treated with the test substance in step (i).

Item 23. The method according to any one of Items 20 to 22, wherein the kidney function prediction marker protein is at least one member selected from the group consisting of Aplnr, Spp1, Dnase1, Slco1a1, Anpep, Slc7a8, Oscar, proline-rich proteins, defensins, fibrinogens, and Hamp/Hepcidin.
Item 24. A device for predicting the presence of at least one disease selected from the group consisting of declined kidney function, chronic kidney disease, and renal failure, and/or the stage of the disease in a subject, the device comprising the following computation means:

means for obtaining data of the subject regarding an inter-organ cross talk indicator in each of one or more organs other than the kidneys in the subject, the inter-organ cross talk indicator being derived from cells or tissue originating from each of the one or more organs;
means for calculating, by comparing the data of the subject obtained by the subject data obtaining means with standard data 1 in one or more organs corresponding to the one or more organs from which the data of the subject originates, similarity of patterns of the inter-organ cross talk indicators between the data of the subject and the standard data 1, wherein the standard data 1 is extracted from a group of standard data 1 predetermined for each of the at least one disease selected from the group consisting of declined kidney function, chronic kidney disease, and renal failure, or for each stage of the disease; and
means for predicting the presence of the at least one disease selected from the group consisting of declined kidney function, chronic kidney disease, and renal failure, and/or the stage of the disease, by using, as a measure, the similarity of patterns of the inter-organ cross talk indicators calculated by the pattern similarity calculation means;
wherein the data of the subject is a pattern of the inter-organ cross talk indicator representing a relationship between an amount of the inter-organ cross talk indicator in the organ other than the kidneys of the subject and an amount of the corresponding inter-organ cross talk indicator in the same organ as the organ other than the kidneys in negative control(s) with no disease in the kidneys, and
the standard data 1 includes patterns of the inter-organ cross talk indicators, each of the patterns being derived from a predetermined relationship between an amount of the inter-organ cross talk indicator in the organ other than the kidneys in positive control(s) affected with the at least one disease selected from the group consisting of declined kidney function, chronic kidney disease, and renal failure, and an amount of the corresponding inter-organ cross talk indicator in the same organ as the organ other than the kidneys in negative control(s) without the at least one disease selected from the group consisting of declined kidney function, chronic kidney disease, and renal failure.

Item 24-1. The device according to Item 24, wherein the inter-organ cross talk indicator is at least one member selected from the group consisting of Aplnr, Spp1, Dnase1, Slco1a1, Anpep, Slc7a8, Oscar, proline-rich proteins, defensins, fibrinogens, and Hamp/Hepcidin.
Item 25. A prediction program that, when executed by a computer, causes the computer to carry out the following processing to predict the presence of at least one disease selected from the group consisting of declined kidney function, chronic kidney disease, and renal failure, and/or the stage of the disease in a subject:

processing of obtaining data of the subject regarding an inter-organ cross talk indicator in each of one or more organs other than the kidneys in the subject, the inter-organ cross talk indicator being derived from cells or tissue originating from each of the one or more organs;
processing of calculating, by comparing the data of the subject obtained by the subject data obtaining processing with standard data 1 in one or more organs corresponding to the one or more organs from which the data of the subject originates, similarity of patterns of the inter-organ cross talk indicators between the data of the subject and the standard data 1, wherein the standard data 1 is extracted from a group of standard data 1 predetermined for each of the at least one disease selected from the group consisting of declined kidney function, chronic kidney disease, and renal failure, or for each stage of the disease; and
processing of predicting the presence of the at least one disease selected from the group consisting of declined kidney function, chronic kidney disease, and renal failure, and/or the stage of the disease, by using, as a measure, the similarity of patterns of the inter-organ cross talk indicators calculated by the pattern similarity calculation processing;
wherein the data of the subject is a pattern of the inter-organ cross talk indicator representing a relationship between an amount of the inter-organ cross talk indicator in the organ other than the kidneys of the subject and an amount of the corresponding inter-organ cross talk indicator in the same organ as the organ other than the kidneys in negative control(s) without the at least one disease selected from the group consisting of declined kidney function, chronic kidney disease, and renal failure, and
the standard data 1 includes patterns of the inter-organ cross talk indicators, each of the patterns being derived from a predetermined relationship between an amount of the inter-organ cross talk indicator in the organ other than the kidneys in positive control(s) affected with the at least one disease selected from the group consisting of declined kidney function, chronic kidney disease, and renal failure, and an amount of the corresponding inter-organ cross talk indicator in the same organ as the organ other than the kidneys in negative control(s) with no disease in the kidneys.

Item 25-1. The prediction program according to Item 25, wherein the inter-organ cross talk indicator is at least one member selected from the group consisting of Aplnr, Spp1, Dnase1, Slco1a1, Anpep, Slc7a8, Oscar, proline-rich proteins, defensins, fibrinogens, and Hamp/Hepcidin.
Item 26. A method for predicting the presence of at least one disease selected from the group consisting of declined kidney function, chronic kidney disease, and renal failure and/or the stage of the disease in a subject, the method comprising the steps of:

(1) obtaining data of the subject regarding an inter-organ cross talk indicator in each of one or more organs other than the kidneys in the subject, the inter-organ cross talk indicator being derived from cells or tissue originating from each of the one or more organs;
(2) calculating, by comparing the data of the subject obtained in step (1) with standard data 1 in one or more organs corresponding to the one or more organs from which the data of the subject originates, similarity of patterns of the inter-organ cross talk indicators between the data of the subject and the standard data 1, wherein the standard data 1 is extracted from a group of standard data 1 predetermined for each of the at least one disease selected from the group consisting of declined kidney function, chronic kidney disease, and renal failure, or for each stage of the disease; and
(3) determining that the subject has a disease corresponding to the standard data 1 and/or that the subject is in a stage of a disease corresponding to the standard data 1 when it is determined from the similarity of patterns of the inter-organ cross talk indicators calculated in step (2) that both patterns are similar;

wherein the data of the subject is a pattern of the inter-organ cross talk indicator representing a relationship between an amount of the inter-organ cross talk indicator in the organ other than the kidneys of the subject and an amount of the corresponding inter-organ cross talk indicator in the same organ as the organ other than the kidneys in negative control(s) without the at least one disease selected from the group consisting of declined kidney function, chronic kidney disease, and renal failure, and
the standard data 1 includes patterns of the inter-organ cross talk indicators, each of the patterns being derived from a predetermined relationship between an amount of the inter-organ cross talk indicator in the organ other than the kidneys in positive control(s) affected with the at least one disease selected from the group consisting of declined kidney function, chronic kidney disease, and renal failure, and an amount of the corresponding inter-organ cross talk indicator in the same organ as the organ other than the kidneys in negative control(s) without the at least one disease selected from the group consisting of declined kidney function, chronic kidney disease, and renal failure.
Item 26-1. The method according to Item 26, wherein the inter-organ cross talk indicator is at least one member selected from the group consisting of Aplnr, Spp1, Dnase1, Slco1a1, Anpep, Slc7a8, Oscar, proline-rich proteins, defensins, fibrinogens, and Hamp/Hepcidin.
Item 27. A method for obtaining information regarding similarity of patterns of inter-organ cross talk indicators to predict the presence of at least one disease selected from the group consisting of declined kidney function, chronic kidney disease, and renal failure and/or the stage of the disease in a subject, the method comprising the steps of:

(1) obtaining data of the subject regarding an inter-organ cross talk indicator in each of one or more organs other than the kidneys in the subject, the inter-organ cross talk indicator being derived from cells or tissue originating from each of the one or more organs; and
(2) calculating, by comparing the data of the subject obtained in step (1) with predetermined standard data 1 of the corresponding inter-organ cross talk indicator, similarity of patterns of the inter-organ cross talk indicators between the data of the subject and the standard data 1, and obtaining information regarding the similarity;

wherein the data of the subject is a pattern of the inter-organ cross talk indicator representing a relationship between an amount of the inter-organ cross talk indicator in the organ other than the kidneys of the subject and an amount of the corresponding inter-organ cross talk indicator in the same organ as the organ other than the kidneys in negative control(s) without the at least one disease selected from the group consisting of declined kidney function, chronic kidney disease, and renal failure, and
the standard data 1 includes patterns of the inter-organ cross talk indicators, each of the patterns being derived from a predetermined relationship between an amount of the inter-organ cross talk indicator in the organ other than the kidneys in positive control(s) affected with the at least one disease selected from the group consisting of declined kidney function, chronic kidney disease, and renal failure, and an amount of the corresponding inter-organ cross talk indicator in the same organ as the organ other than the kidneys in negative control(s) without the at least one disease selected from the group consisting of declined kidney function, chronic kidney disease, and renal failure.
Item 27-1. The method according to Item 27, wherein the inter-organ cross talk indicator is at least one member selected from the group consisting of Aplnr, Spp1, Dnase1, Slco1a1, Anpep, Slc7a8, Oscar, proline-rich proteins, defensins, fibrinogens, and Hamp/Hepcidin.
Item 28. A device for predicting the presence of a disease and/or the stage of the disease in each of one or more organs other than the kidneys in a subject affected with at least one disease selected from the group consisting of declined kidney function, chronic kidney disease, and renal failure, the device comprising the following computation means:

means for obtaining information about a stage of the at least one disease selected from the group consisting of declined kidney function, chronic kidney disease, and renal failure in the subject;
means for checking the information about the stage obtained by the stage information obtaining means against standard data 2;
means for extracting a pattern of an inter-organ cross talk indicator in each of one or more organs other than the kidneys in the subject based on the result obtained by the stage information checking means; and
means for predicting the presence of a disease and/or the stage of the disease in each of the one or more organs other than the kidneys by using, as a measure, the pattern of the inter-organ cross talk indicator obtained by the pattern extraction means;
wherein the standard data 2 includes patterns of the inter-organ cross talk indicators predetermined for each stage of the at least one disease selected from the group consisting of declined kidney function, chronic kidney disease, and renal failure, each of the patterns being derived from a predetermined relationship between an amount of the inter-organ cross talk indicator in the organ other than the kidneys in positive control(s) affected with the at least one disease selected from the group consisting of declined kidney function, chronic kidney disease, and renal failure, and an amount of the corresponding inter-organ cross talk indicator in the same organ as the organ other than the kidneys in negative control(s) without the at least one disease selected from the group consisting of declined kidney function, chronic kidney disease, and renal failure.

Item 28-1. The device according to Item 28, wherein the inter-organ cross talk indicator is at least one member selected from the group consisting of Aplnr, Spp1, Dnase1, Slco1a1, Anpep, Slc7a8, Oscar, proline-rich proteins, defensins, fibrinogens, and Hamp/Hepcidin.
Item 29. A prediction program that, when executed by a computer, causes the computer to carry out the following processing to predict the presence of a disease and/or the stage of the disease in each of one or more organs other than the kidneys in a subject affected with at least one disease selected from the group consisting of declined kidney function, chronic kidney disease, and renal failure:

processing of obtaining information about a stage of the at least one disease selected from the group consisting of declined kidney function, chronic kidney disease, and renal failure in the subject;
stage information comparison processing of checking the information about the stage obtained by the stage information obtaining processing against standard data 2;
processing of extracting a pattern of an inter-organ cross talk indicator in each of one or more organs other than the kidneys in the subject based on the result obtained by the stage information comparison processing; and
processing of predicting the presence of a disease and/or the stage of the disease in each of the one or more organs other than the kidneys by using, as a measure, the pattern of the inter-organ cross talk indicator obtained by the pattern extraction processing,
wherein the standard data 2 includes patterns of the inter-organ cross talk indicators predetermined for each stage of the at least one disease selected from the group consisting of declined kidney function, chronic kidney disease, and renal failure, each of the patterns being derived from a predetermined relationship between an amount of the inter-organ cross talk indicator in the organ other than the kidneys in positive control(s) affected with the at least one disease selected from the group consisting of declined kidney function, chronic kidney disease, and renal failure, and an amount of the corresponding inter-organ cross talk indicator in the same organ as the organ other than the kidneys in negative control(s) without the at least one disease selected from the group consisting of declined kidney function, chronic kidney disease, and renal failure.

Item 29-1. The prediction program according to Item 29, wherein the inter-organ cross talk indicator is at least one member selected from the group consisting of Aplnr, Spp1, Dnase1, Slco1a1, Anpep, Slc7a8, Oscar, proline-rich proteins, defensins, fibrinogens, and Hamp/Hepcidin.
Item 30. A method for predicting the presence of a disease and/or the stage of the disease in each of one or more organs other than the kidneys in a subject affected with at least one disease selected from the group consisting of declined kidney function, chronic kidney disease, and renal failure, the method comprising the steps of:

(i) obtaining information about a stage of the at least one disease selected from the group consisting of declined kidney function, chronic kidney disease, and renal failure in the subject from a diagnostic result of the subject;
(ii) checking the information about the stage obtained in step (i) against standard data 2;
(iii) determining, from the standard data 2, standard data $\alpha$ at a stage of the at least one disease selected from the group consisting of declined kidney function, chronic kidney disease, and renal failure corresponding to the information about the stage, based on the checking result obtained in step (ii), and extracting, from the standard data $\alpha$, a pattern of an inter-organ cross talk indicator corresponding to the stage in the subject in each of one or more organs other than the kidneys in the subject;
(iv) checking the pattern of the inter-organ cross talk indicator extracted in step (iii) against known information about inter-organ cross talk indicators in diseases and/or stages of the diseases, and determining the presence of a disease and/or the stage of the disease in each of the one or more organs other than the kidneys corresponding to the pattern of the inter-organ cross talk indicator in each of the one or more organs other than the kidneys in the subject; and
(v) further determining that the disease in each of the one or more organs other than the kidneys determined in step (iv) is a disease from which the subject may be suffering, and/or determining that the stage of the disease in each of the one or more organs other than the kidneys determined in step (iv) is a stage of a disease from which the subject is suffering,

wherein the standard data 2 includes patterns of the inter-organ cross talk indicators predetermined for each stage of the at least one disease selected from the group consisting of declined kidney function, chronic kidney disease, and renal failure, each of the patterns being derived from a predetermined relationship between an amount of the inter-organ cross talk indicator in the organ other than the kidneys in positive control(s) affected with the at least one disease selected from the group consisting of declined kidney function, chronic kidney disease, and renal failure, and an amount of the corresponding inter-organ cross talk indicator in the same organ as the organ other than the kidneys in negative control(s) with no disease in the kidneys.
Item 30-1. The method according to Item 30, wherein the inter-organ cross talk indicator is at least one member selected from the group consisting of Aplnr, Spp1, Dnase1, Slco1a1, Anpep, Slc7a8, Oscar, proline-rich proteins, defensins, fibrinogens, and Hamp/Hepcidin.
Item 31. A method for obtaining information to predict the presence of a disease and/or the stage of the disease in each of one or more organs other than the kidneys in a subject affected with at least one disease selected from the group consisting of declined kidney function, chronic kidney disease, and renal failure, the method comprising the steps of:

(i) obtaining information about a stage of the at least one disease selected from the group consisting of declined kidney function, chronic kidney disease, and renal failure in the subject from a diagnostic result of the subject;
(ii) checking the information about the stage obtained in step (i) against standard data 2
(iii) determining, from the standard data 2, standard data $\alpha$ at a stage of the at least one disease selected from the group consisting of declined kidney function, chronic kidney disease, and renal failure corresponding to the information about the stage, based on the checking result obtained in step (ii), and extracting, from the standard data $\alpha$, a pattern of an inter-organ cross talk indicator corresponding to the stage in the subject in each of one or more organs other than the kidneys in the subject; and
(iv') checking the pattern of the inter-organ cross talk indicator extracted in step (iii) against known information regarding inter-organ cross talk indicators in diseases and/or the stages of the diseases, and obtaining information regarding the presence of a disease and/or the stage of the disease in each of the one or more organs other than the kidneys corresponding to the pattern of the inter-organ cross talk indicator in each of the one or more organs other than the kidneys in the subject,
wherein the standard data 2 includes patterns of the inter-organ cross talk indicators predetermined for each stage of the at least one disease selected from the group consisting of declined kidney function, chronic kidney disease, and renal failure, each of the patterns being derived from a predetermined relationship between an amount of the inter-organ cross talk indicator in the organ other than the kidneys in positive control(s) affected with the at least one disease selected from the group consisting of declined kidney function, chronic kidney disease, and renal failure, and an amount of the corresponding inter-organ cross talk indicator in the same organ as the organ other than the kidneys in negative control(s) with no disease in the kidneys.

Item 31-1. The method according to Item 31, wherein the inter-organ cross talk indicator is at least one member selected from the group consisting of Aplnr, Spp1, Dnase1, Slco1a1, Anpep, Slc7a8, Oscar, proline-rich proteins, defensins, fibrinogens, and Hamp/Hepcidin.
Item 32. A method for generating standard data of patterns of inter-organ cross talk indicators for use in prediction of the presence of at least one disease selected from the group consisting of declined kidney function, chronic kidney disease, and renal failure and/or the stage of the disease in a subject, the method comprising the steps of:

extracting an inter-organ cross talk indicator from cells or tissue collected from each of one or more organs other than the kidneys of positive control(s) of a gold standard for each stage of the at least one disease selected from the group consisting of declined kidney function, chronic kidney disease, and renal failure;
extracting the inter-organ cross talk indicator from cells or tissue collected from each of the one or more organs other than the kidneys of negative control(s) of a gold standard;
identifying and quantifying the inter-organ cross talk indicators;
determining patterns of the inter-organ cross talk indicators, each of the patterns being determined from a relationship between an amount of the inter-organ cross talk indicator in the organ other than the kidneys in the positive control(s) affected with the at least one disease selected from the group consisting of declined kidney function, chronic kidney disease, and renal failure, and an amount of the corresponding inter-organ cross talk indicator in the same organ as the organ other than the kidneys in the negative control(s) without the at least one disease selected from the group consisting of declined kidney function, chronic kidney disease, and renal failure; and
associating the patterns of the inter-organ cross talk indicators with the corresponding stages of the at least one disease selected from the group consisting of declined kidney function, chronic kidney disease, and renal failure.

Item 32-1. The method according to Item 32, wherein the inter-organ cross talk indicator is at least one member selected from the group consisting of Aplnr, Spp1, Dnase1, Slco1a1, Anpep, Slc7a8, Oscar, proline-rich proteins, defensins, fibrinogens, and Hamp/Hepcidin.
Item 33. A method for generating standard data of patterns of inter-organ cross talk indicators for use in prediction of the presence of a disease and/or the stage of the disease in each of one or more organs other than the kidneys in a subject affected with at least one disease selected from the group consisting of declined kidney function, chronic kidney disease, and renal failure, the method comprising the steps of:

extracting an inter-organ cross talk indicator from cells or tissue collected from each of one or more organs other than the kidneys of positive control(s) of a gold standard for each stage of the at least one disease selected from the group consisting of declined kidney function, chronic kidney disease, and renal failure;
extracting the inter-organ cross talk indicator from cells or tissue collected from each of the one or more organs other than the kidneys of negative control(s) of a gold standard;
identifying and quantifying the inter-organ cross talk indicators; and
determining patterns of the inter-organ cross talk indicators for each stage of the at least one disease selected from the group consisting of declined kidney function, chronic kidney disease, and renal failure, each of the patterns being determined from a relationship between an amount of the inter-organ cross talk indicator in the organ other than the kidneys in the positive control(s) affected with the at least one disease selected from the group consisting of declined kidney function, chronic kidney disease, and renal failure, and an amount of the corresponding inter-organ cross talk indicator in the same organ as the organ other than the kidneys in the negative control(s) without the at least one disease selected from the group consisting of declined kidney function, chronic kidney disease, and renal failure.

Item 33-1. The method according to Item 33, wherein the inter-organ cross talk indicator is at least one member selected from the group consisting of Aplnr, Spp1, Dnase1, Slco1a1, Anpep, Slc7a8, Oscar, proline-rich proteins, defensins, fibrinogens, and Hamp/Hepcidin.

## Advantageous Effects of Invention

According to an embodiment of the present invention, a method for screening a candidate substance for an active ingredient to prevent or treat at least one disease selected from the group consisting of declined kidney function, chronic kidney disease, and renal failure can be provided.

Moreover, according to an embodiment of the present invention (Reverse iOrgans), subtle changes in the state of an organ other than the kidneys are correlated with subtle changes in the kidneys to capture subtle changes in the whole kidneys or kidney tissue, and thus the present invention can detect at least one disease selected from the group consisting of declined kidney function, chronic kidney disease, and renal failure earlier than usual diagnostic methods. Furthermore, the use of a device or program for evaluating such a correlation in multiple organs or tissues makes it possible to diagnose the multiple organs or tissues by diagnosing one organ or tissue, dramatically improving diagnostic efficiency. According to an embodiment of the present invention (Forward iOrgans), the state of an organ that cannot yet be diagnosed as having an abnormality by using a usual test is inferred from the state of the kidneys already confirmed to have at least one disease selected from the group consisting of declined kidney function, chronic kidney disease, and renal failure by using a usual diagnostic method; therefore, an abnormality in other organs or tissues caused by at least one disease selected from the group consisting of declined kidney function, chronic kidney disease, and renal failure can be detected early, and secondary and tertiary diseases (such as renal failure, hepatopathy, and cancer metastasis) can be prevented or treated.

Brief Description of Drawings

Fig. 1 is an overview of a system 100 according to a first embodiment of the present invention.

Fig. 2 is a block diagram illustrating a hardware configuration of the system 100 according to the first embodiment of the present invention.

Fig. 3 is a block diagram illustrating functions of a screening device 1 according to the first embodiment of the present invention.

Fig. 4 is a flow chart illustrating a flow of data processing performed by the screening device 1 according to the first embodiment of the present invention to carry out a screening method.

Fig. 5 is an overview of a system 110 according to a second embodiment of the present invention.

Fig. 6 is a block diagram illustrating a hardware configuration of the system 110 according to the second embodiment of the present invention.

Fig. 7 is a block diagram illustrating functions of a screening device 2 according to the second embodiment of the present invention.

Fig. 8 is a flow chart illustrating a flow of data processing performed by the screening device 2 according to the second embodiment of the present invention to carry out a screening method.

Fig. 9 schematically illustrates an outline of Reverse iOrgans according to the present invention.

Fig. 10 schematically illustrates an outline of Reverse iOrgans according to the present invention.

Fig. 11 schematically illustrates an outline of Forward iOrgans according to the present invention.

Fig. 12 schematically illustrates an outline of Forward iOrgans according to the present invention.

Fig. 13 is an overview of a system 120 according to a third embodiment of the present invention.

Fig. 14 is a block diagram illustrating a hardware configuration of the system 120 according to the third embodiment of the present invention.

Fig. 15 is a block diagram illustrating functions of a prediction device 6 according to the third embodiment of the present invention.

Fig. 16 is a flow chart illustrating a flow of data processing performed by the prediction device 6 according to the third embodiment of the present invention to carry out a prediction method.

Fig. 17 is an overview of a system 130 according to a fourth embodiment of the present invention.

Fig. 18 is a block diagram illustrating a hardware configuration of the system 130 according to the fourth embodiment of the present invention.

Fig. 19 is a block diagram illustrating functions of a prediction device 7 according to the fourth embodiment of the present invention.

Fig. 20 is a flow chart illustrating a flow of data processing performed by the prediction device 7 according to the fourth embodiment of the present invention to carry out a prediction method.

Fig. 21 is a list of genes in mice that can be detected by, for example, RNA-Seq. In Fig. 21, "Line No" indicates a line number in the list, "Gene Name" indicates a gene name registered with the U.S. National Center for Biotechnology Information (NCBI), and "Reference Seq. ID" indicates a reference sequence ID number registered with NCBI. "Chromosome Location" indicates a chromosome locus registered in mm10.

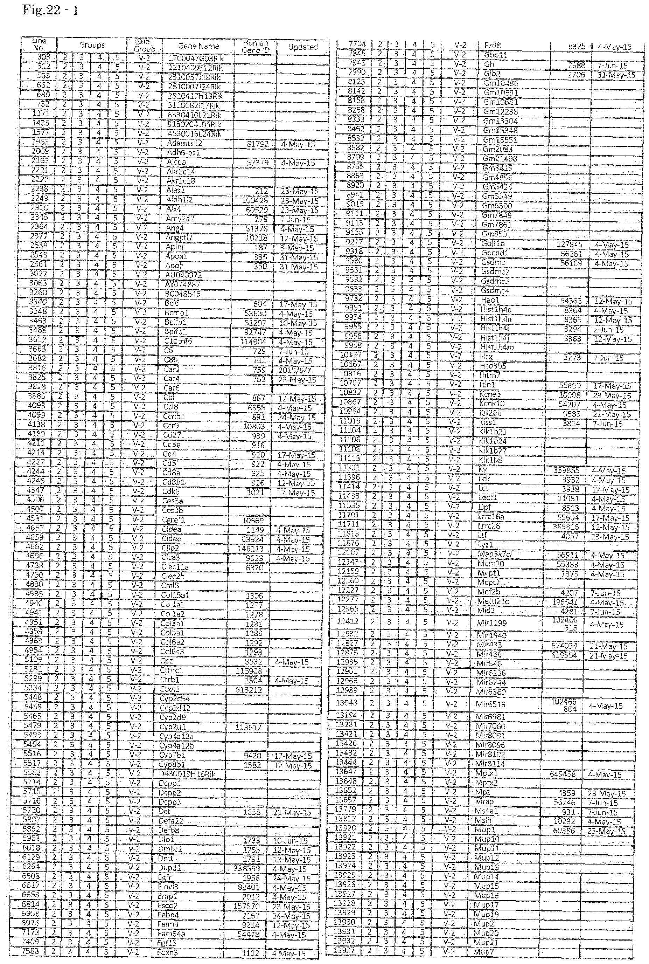

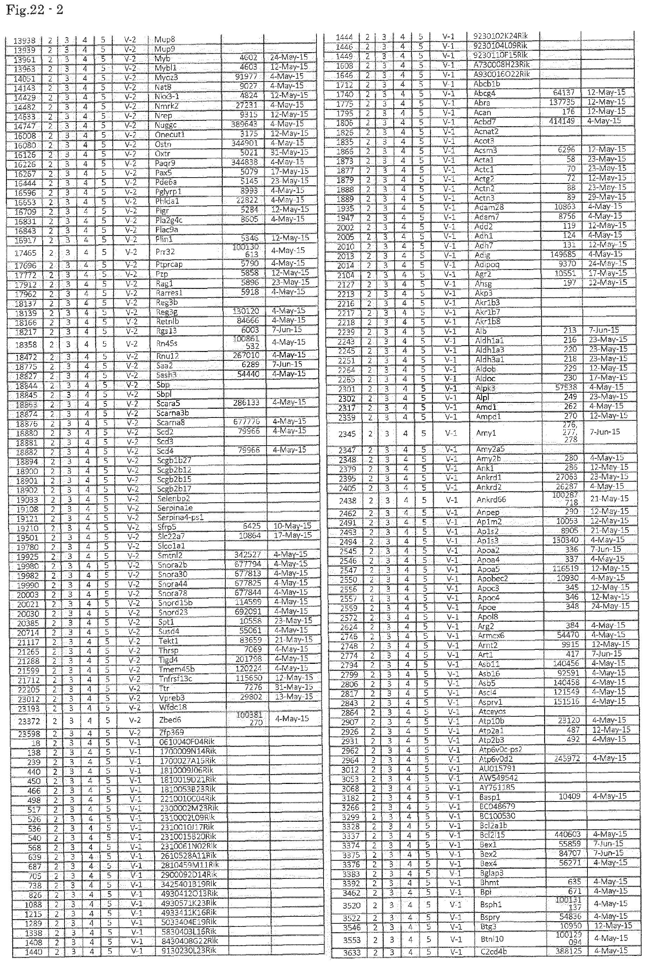

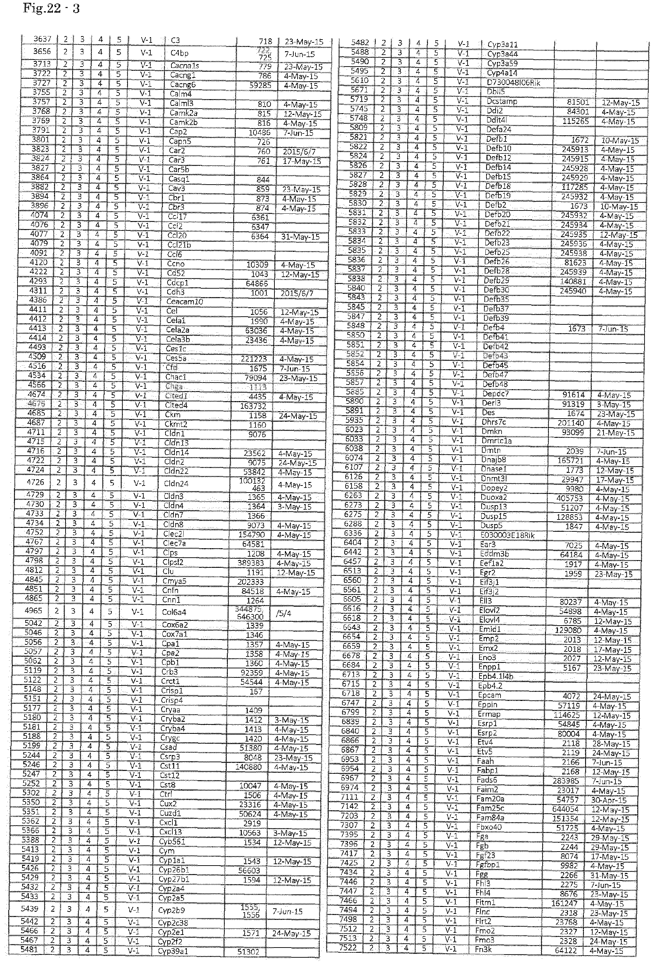

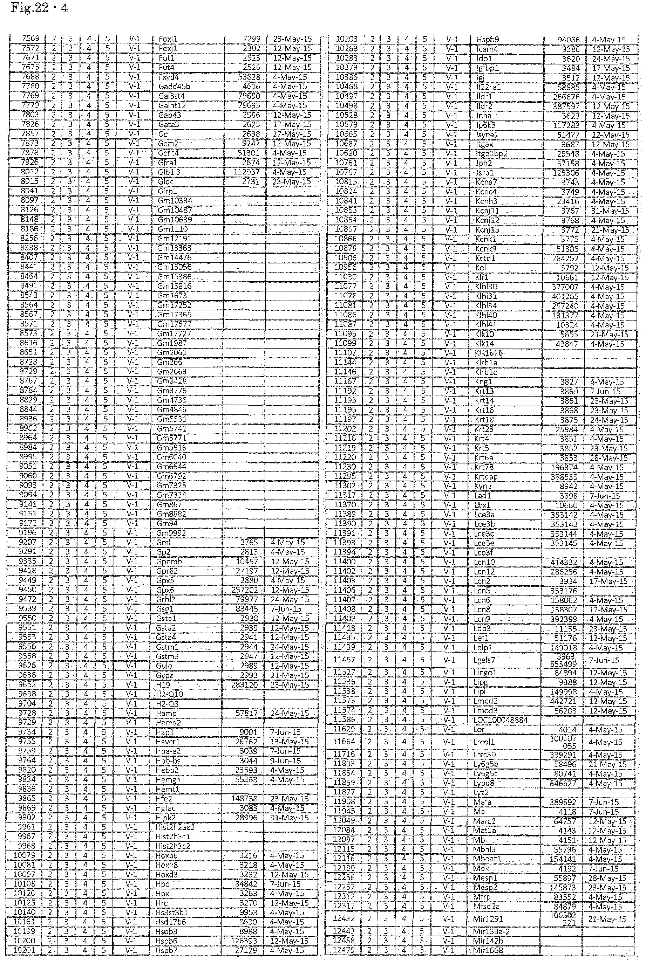

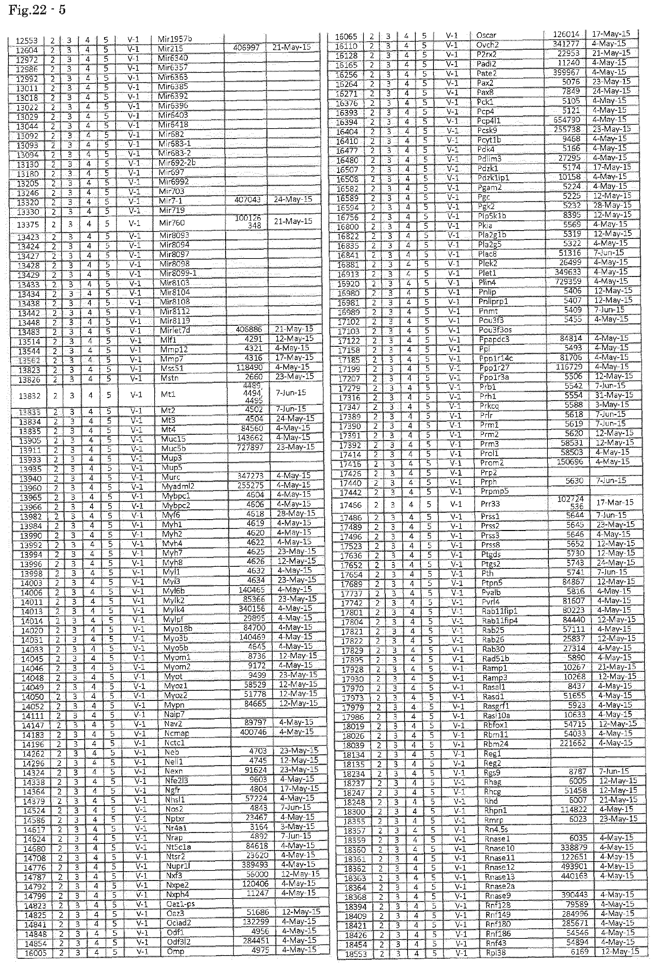

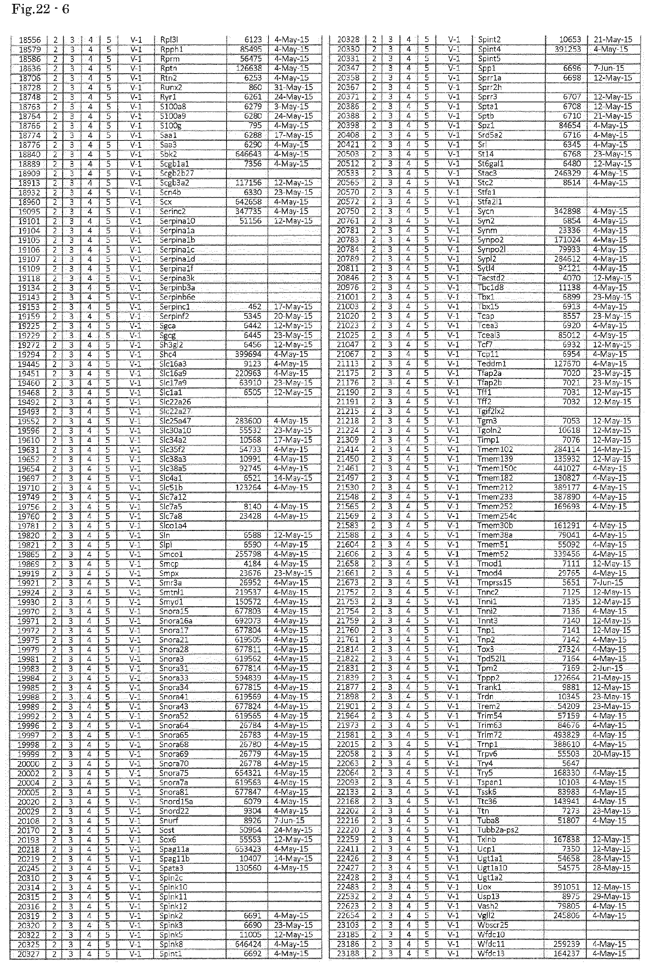

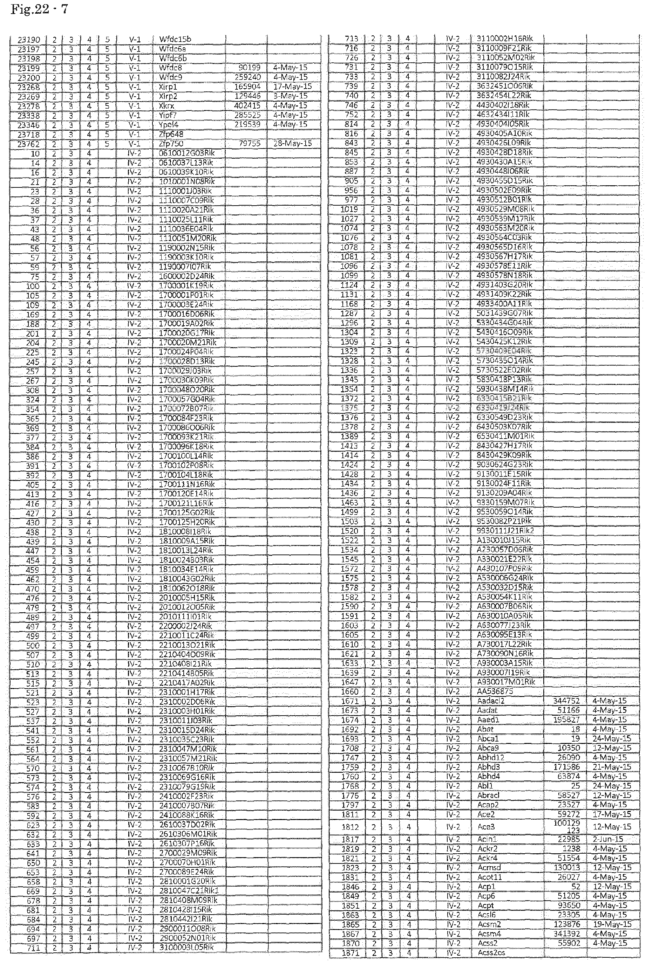

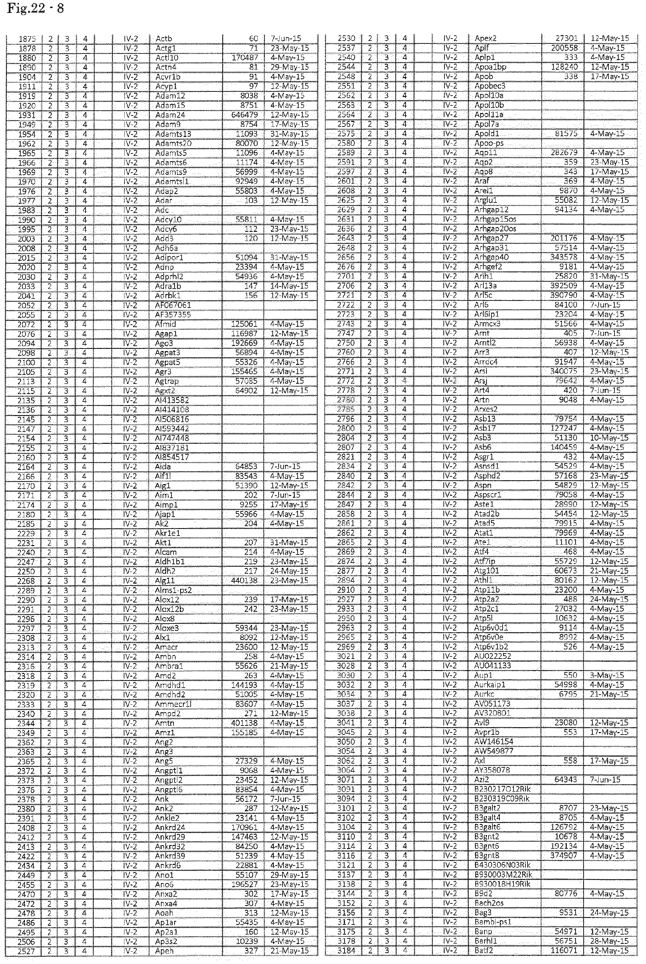

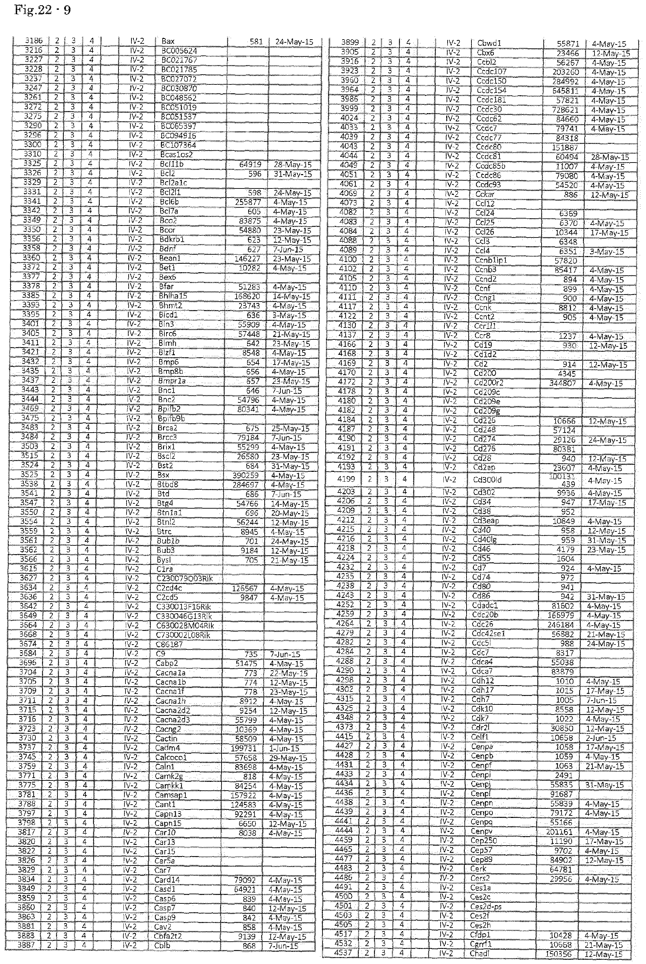

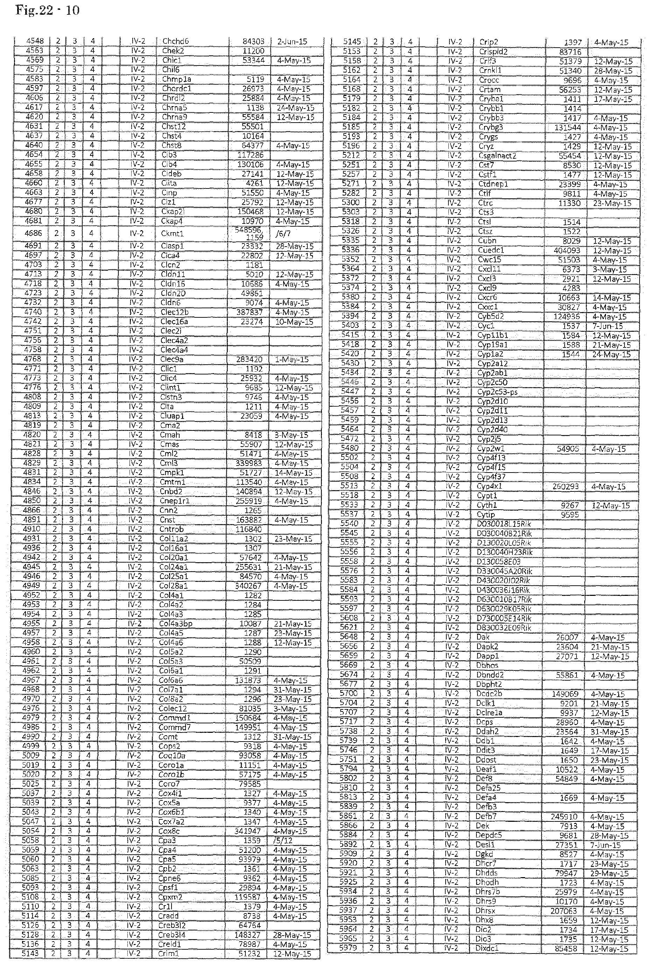

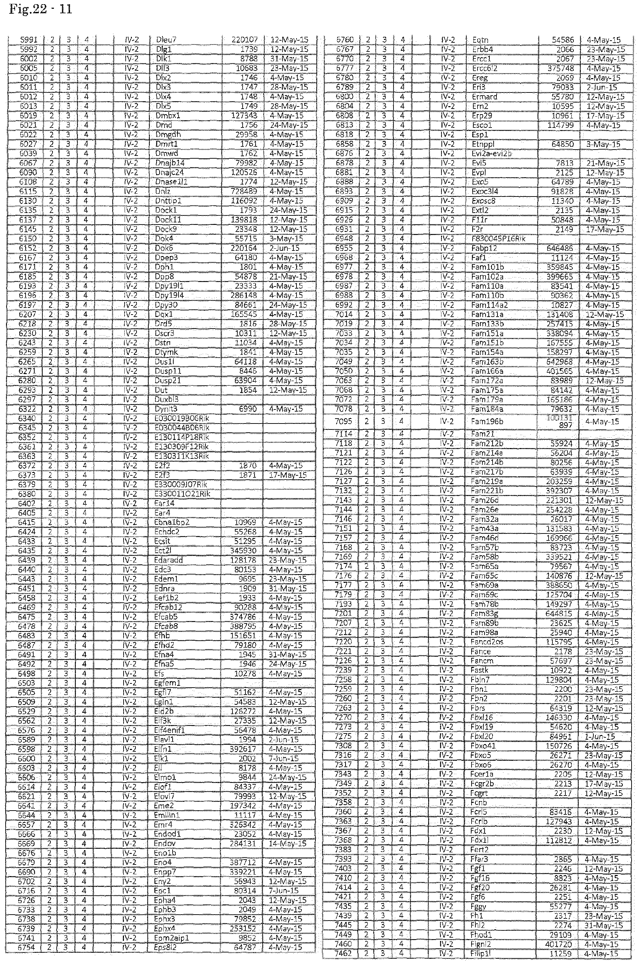

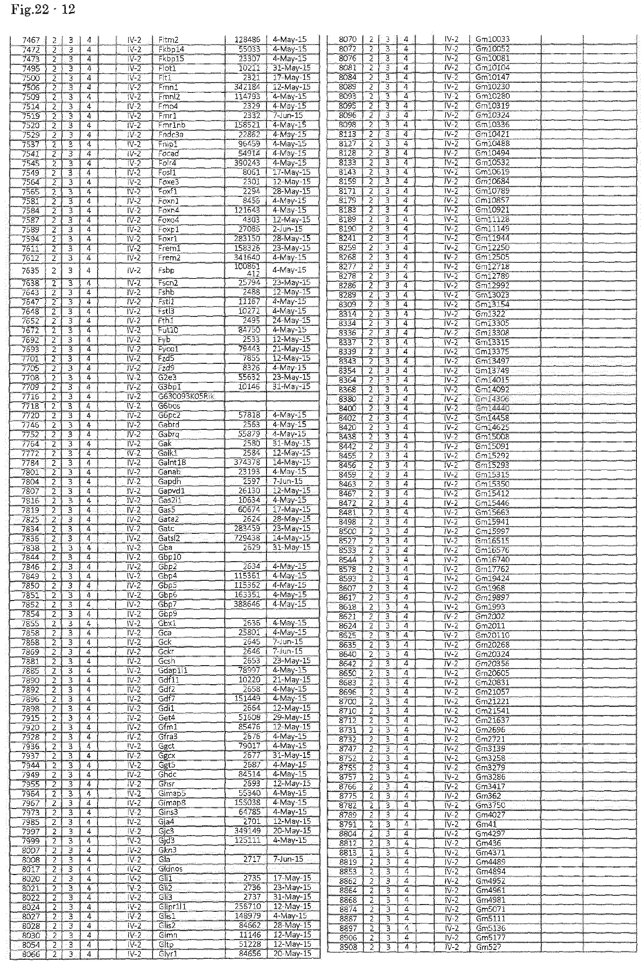

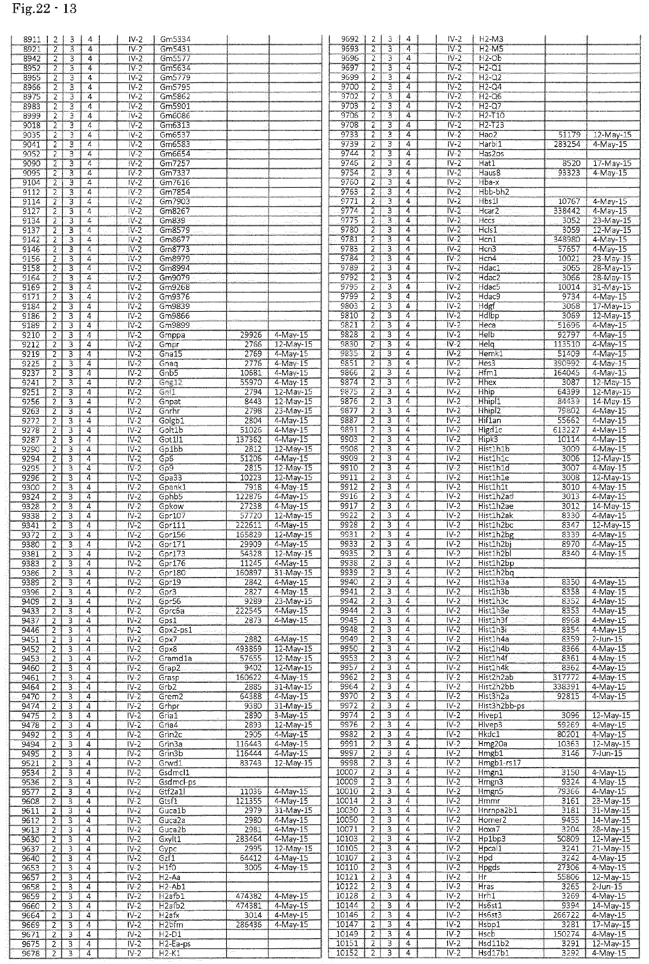

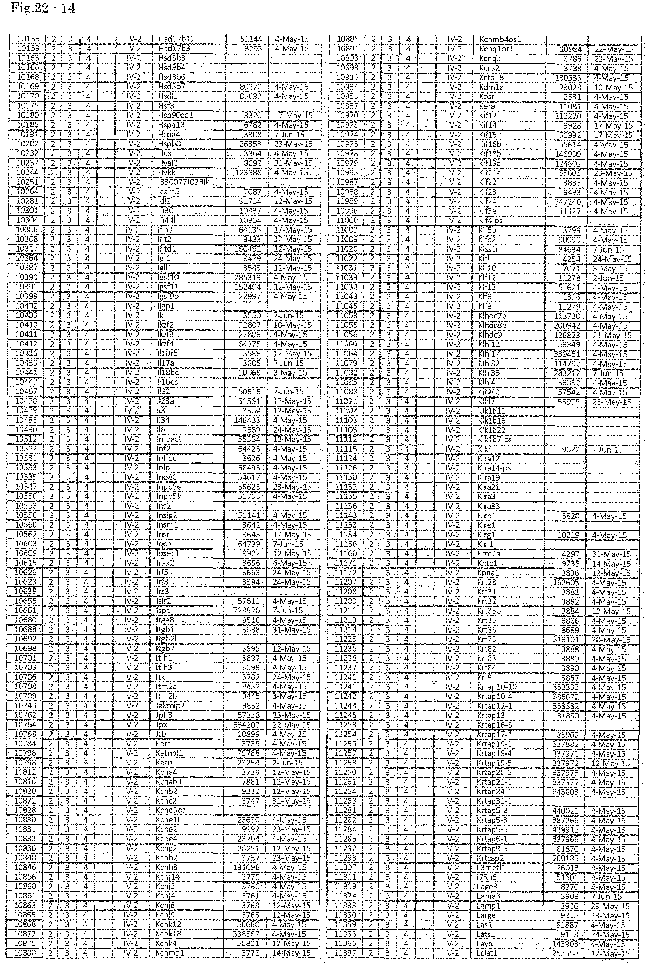

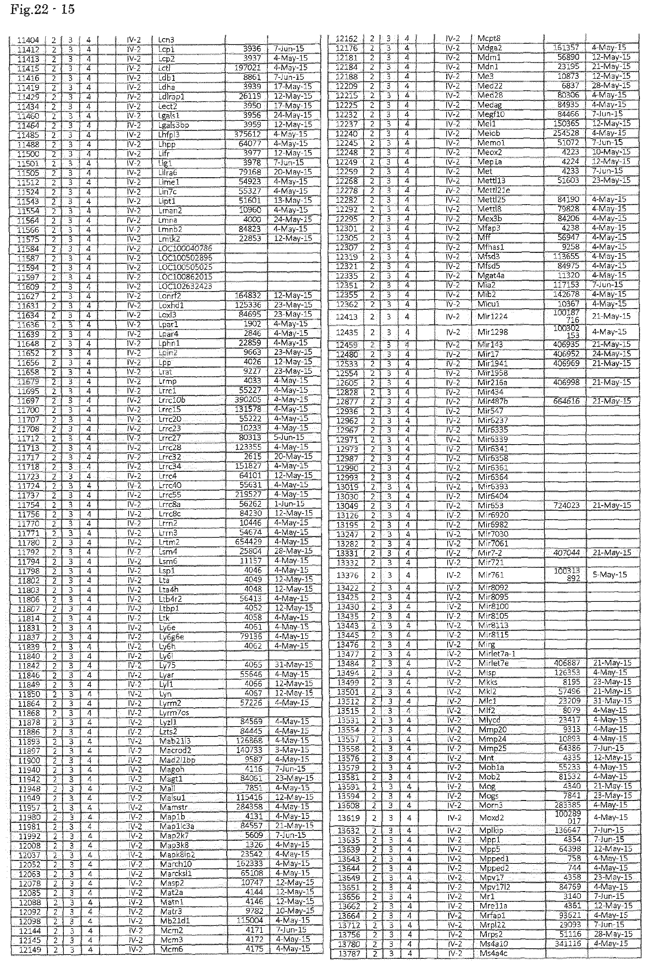

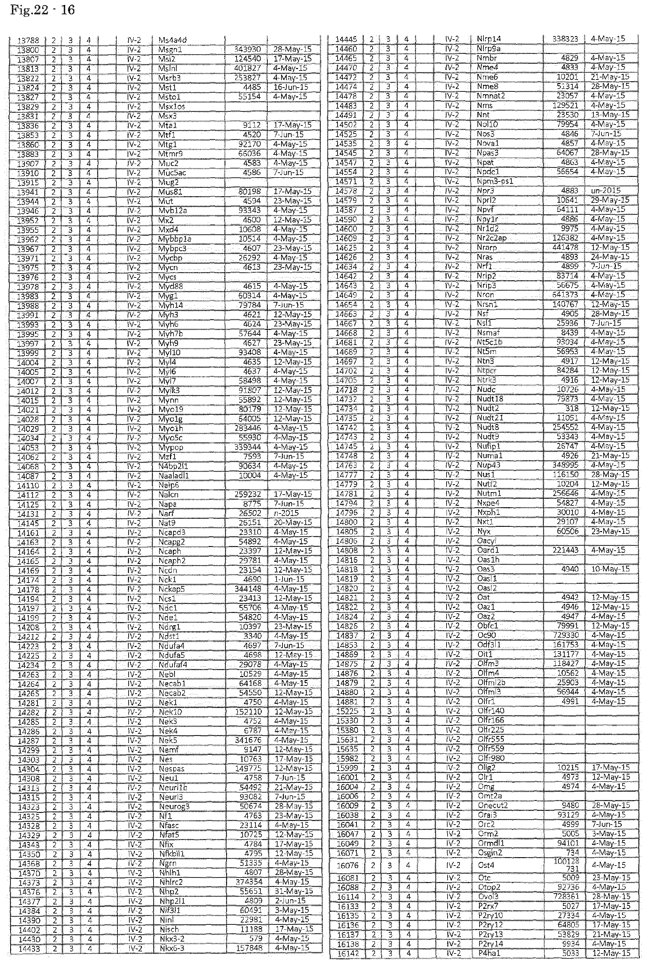

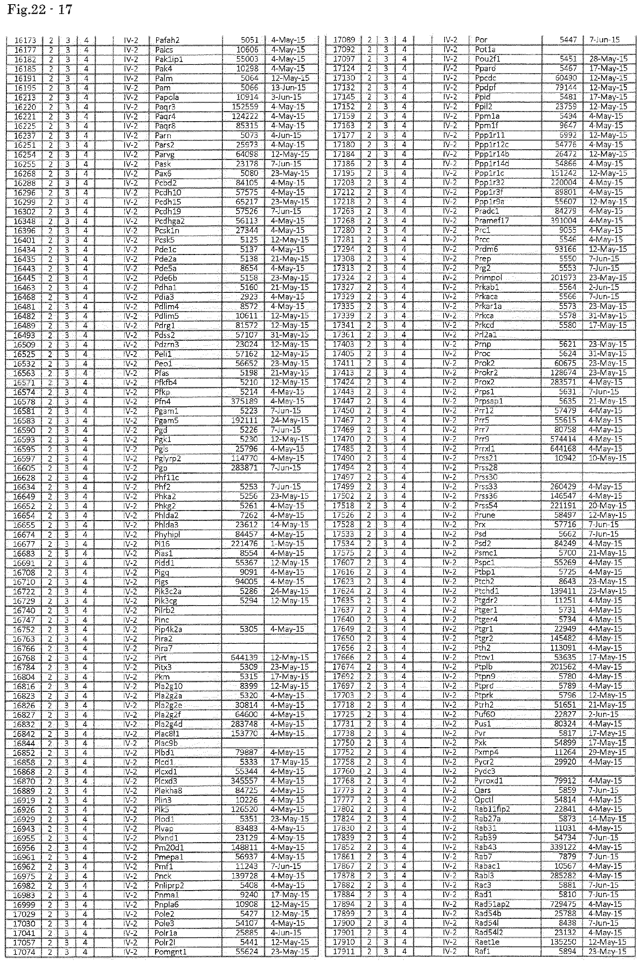

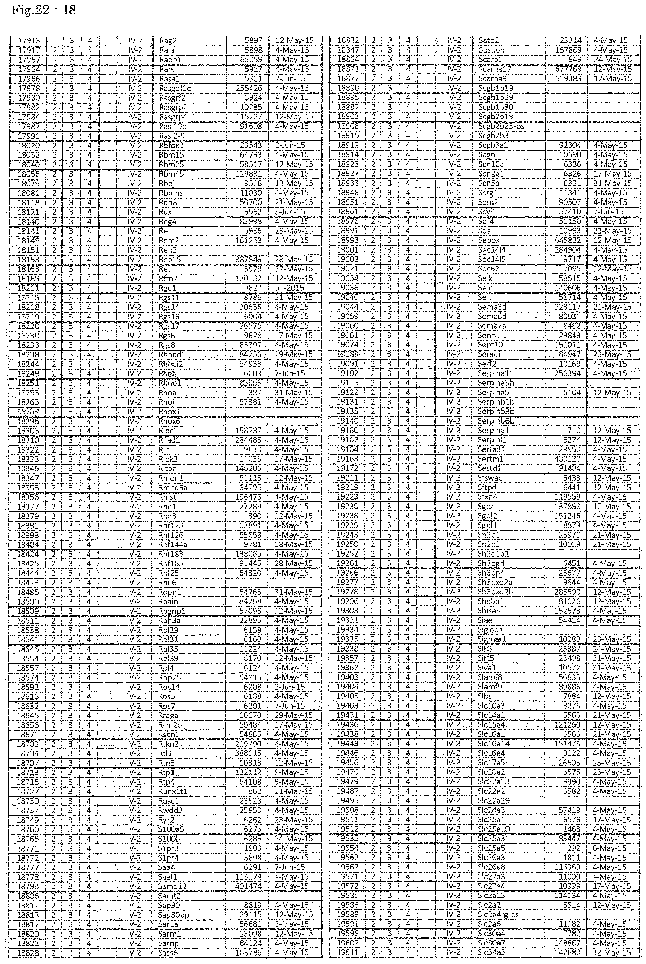

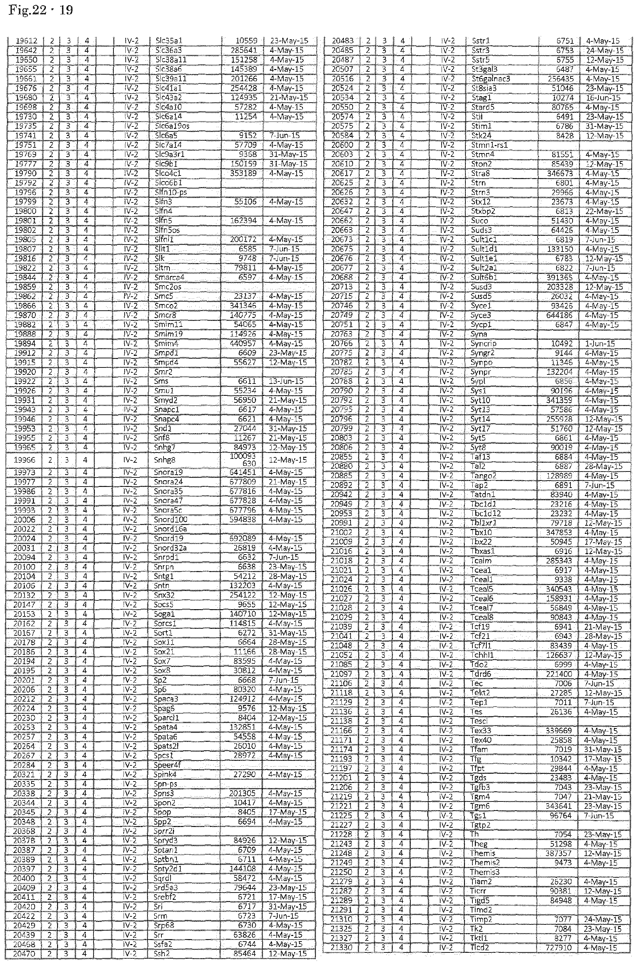

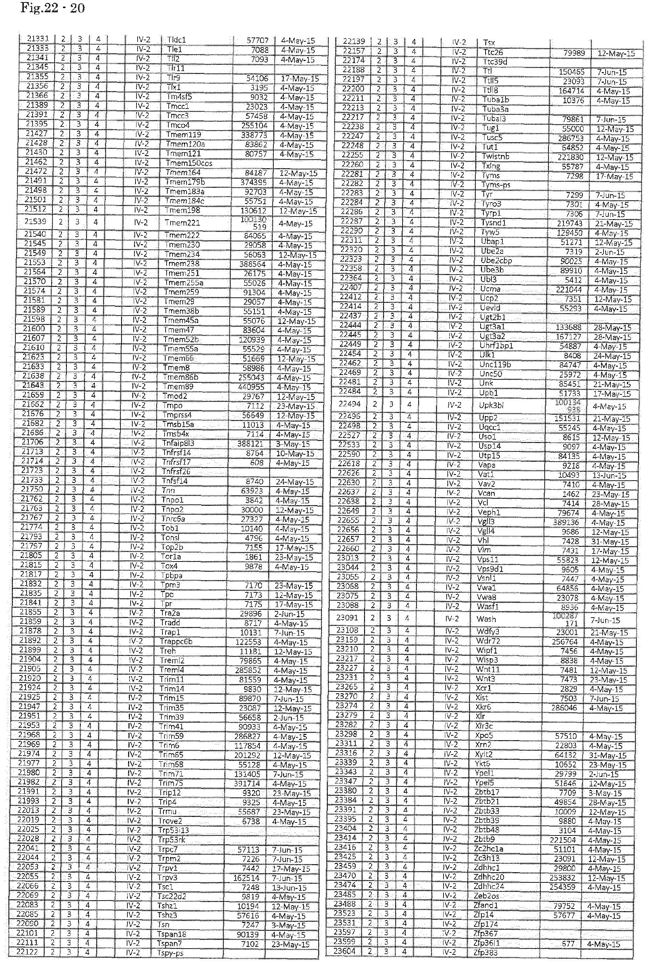

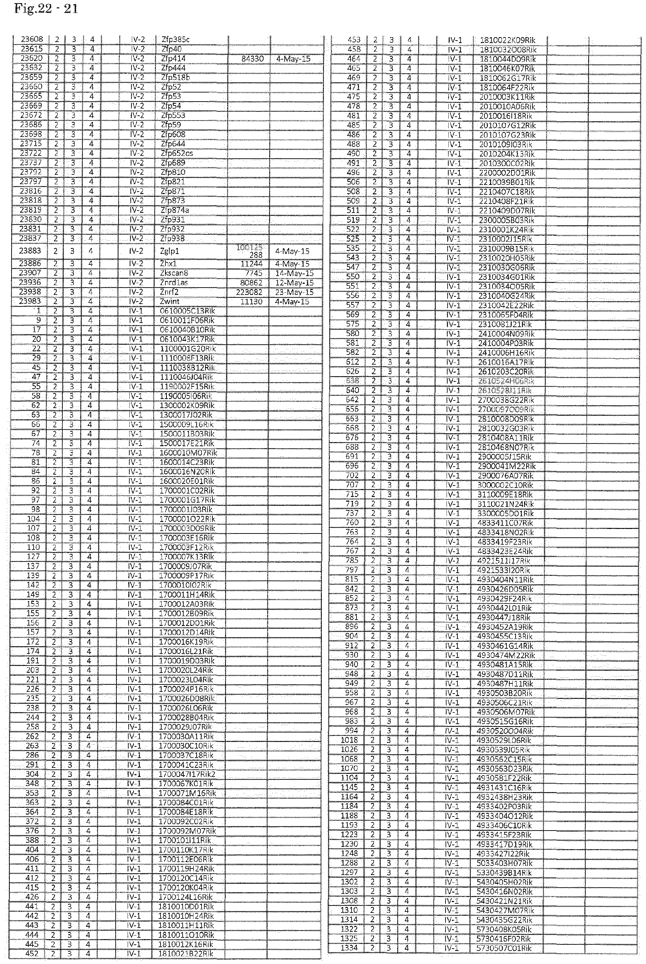

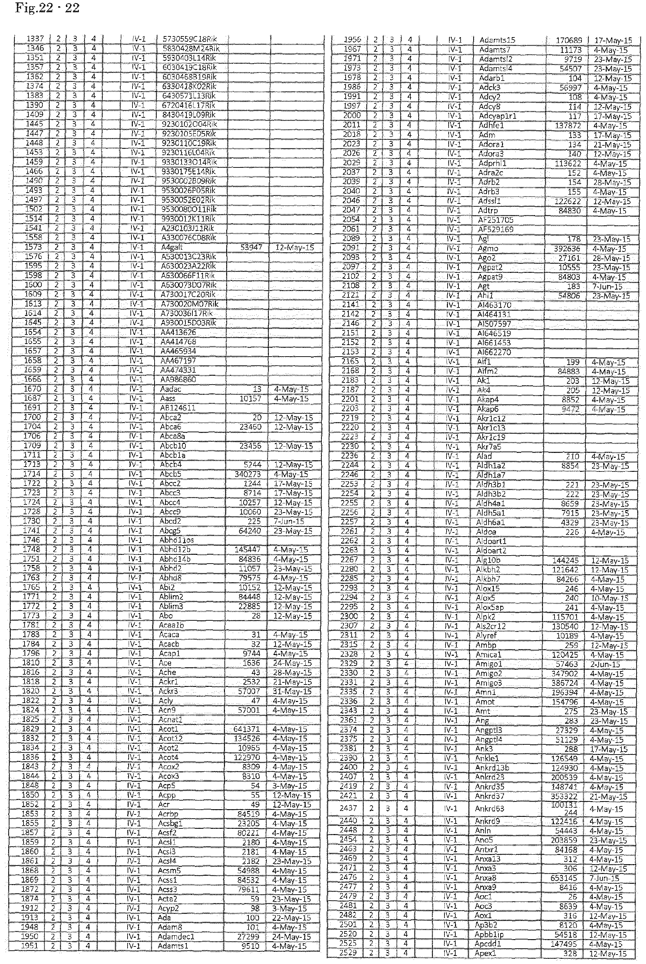

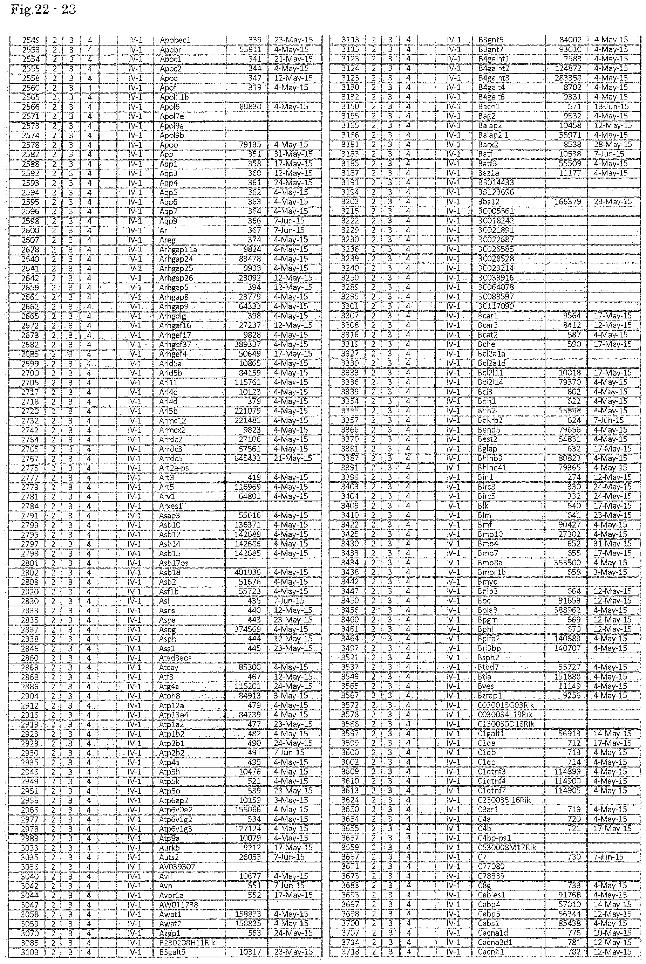

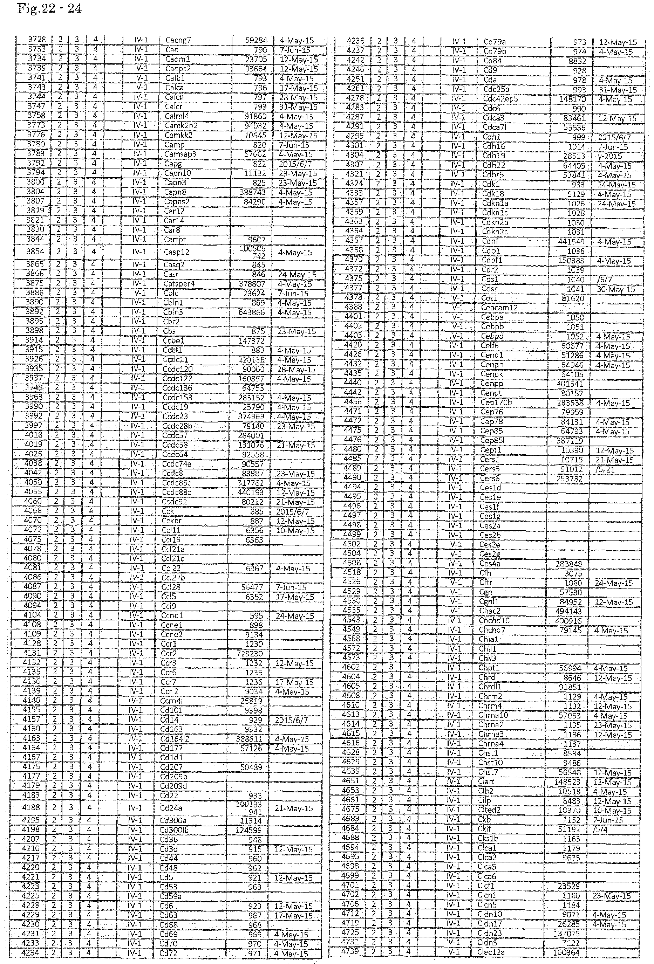

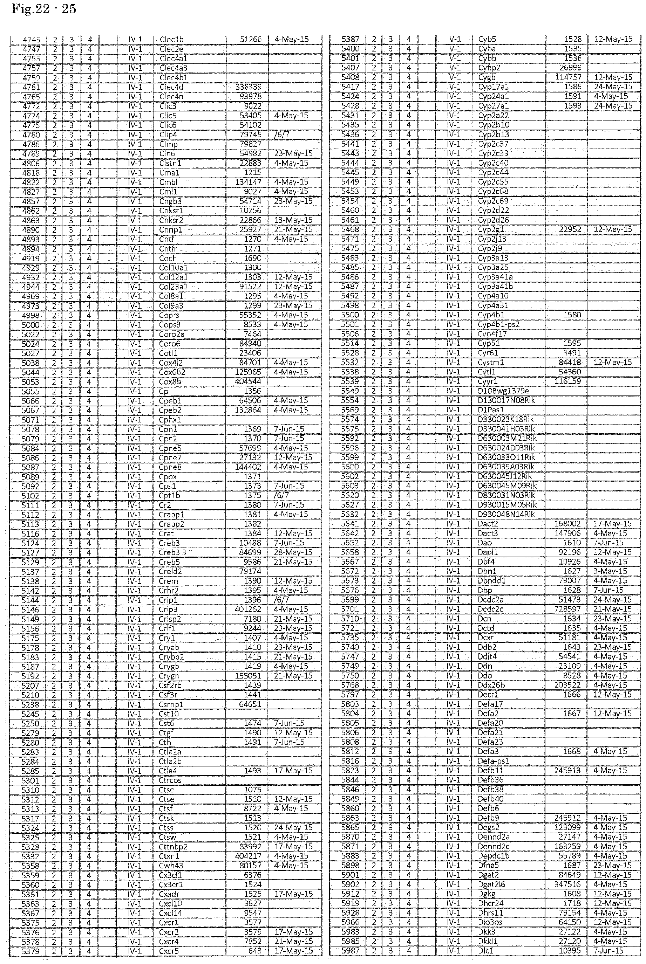

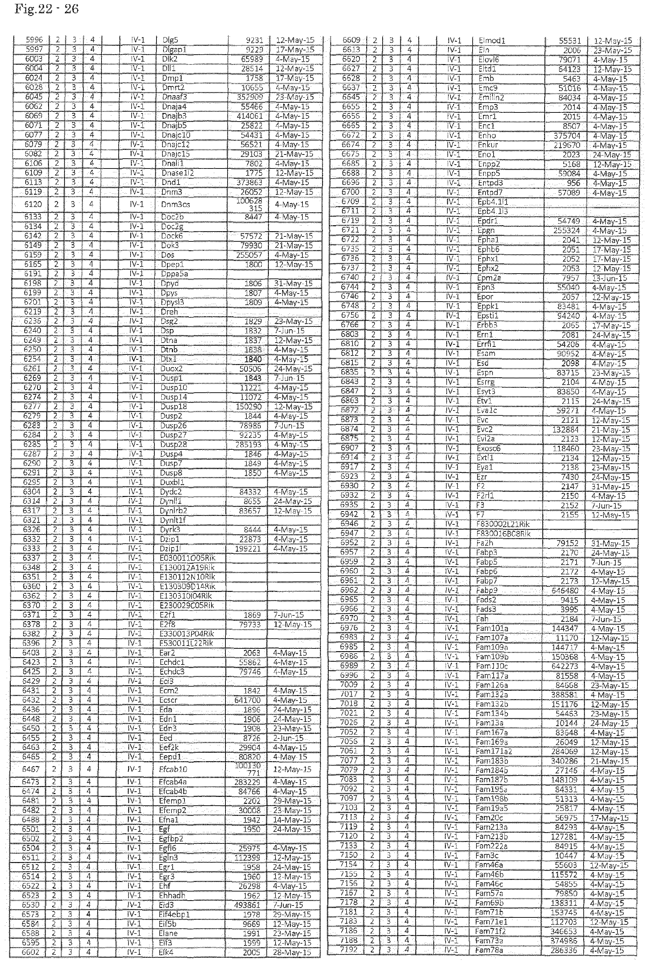

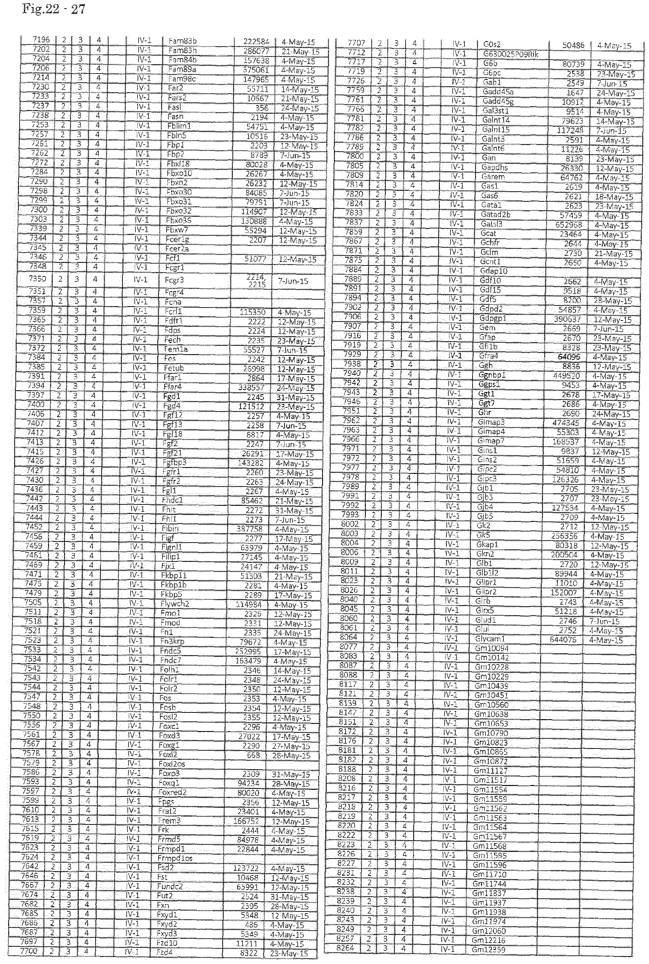

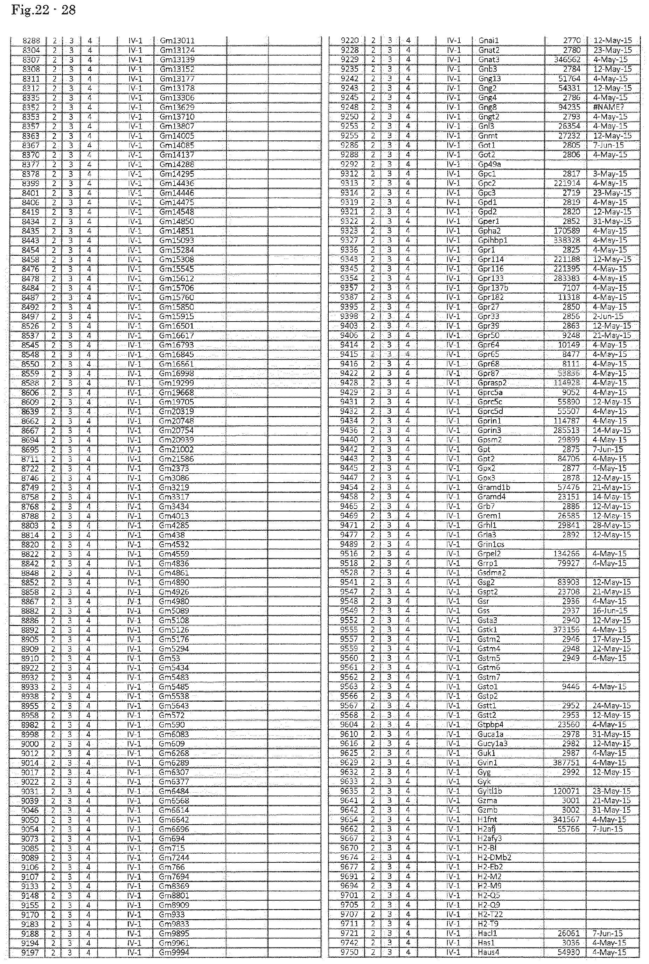

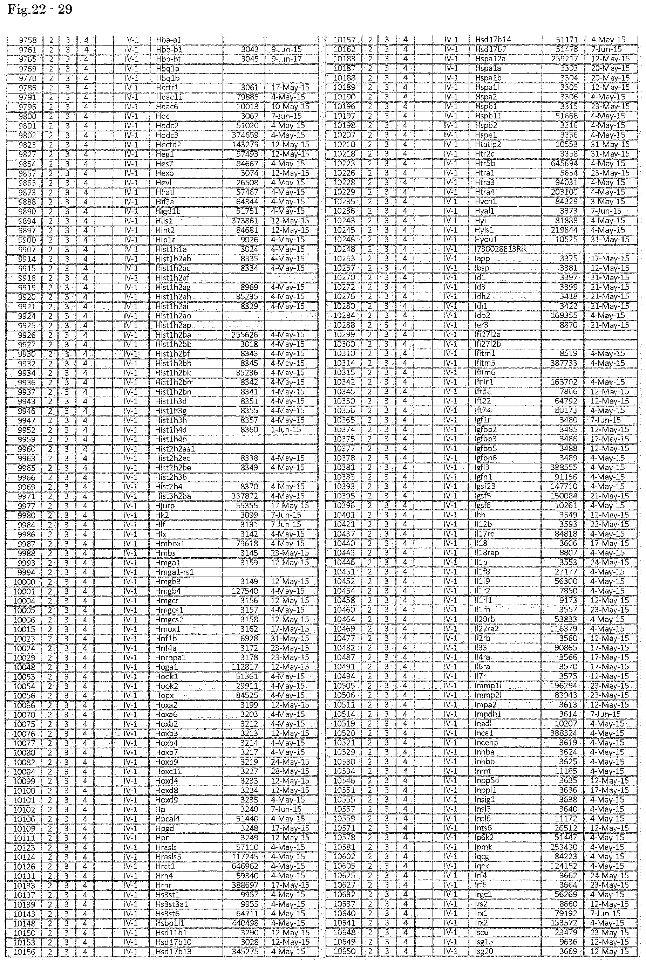

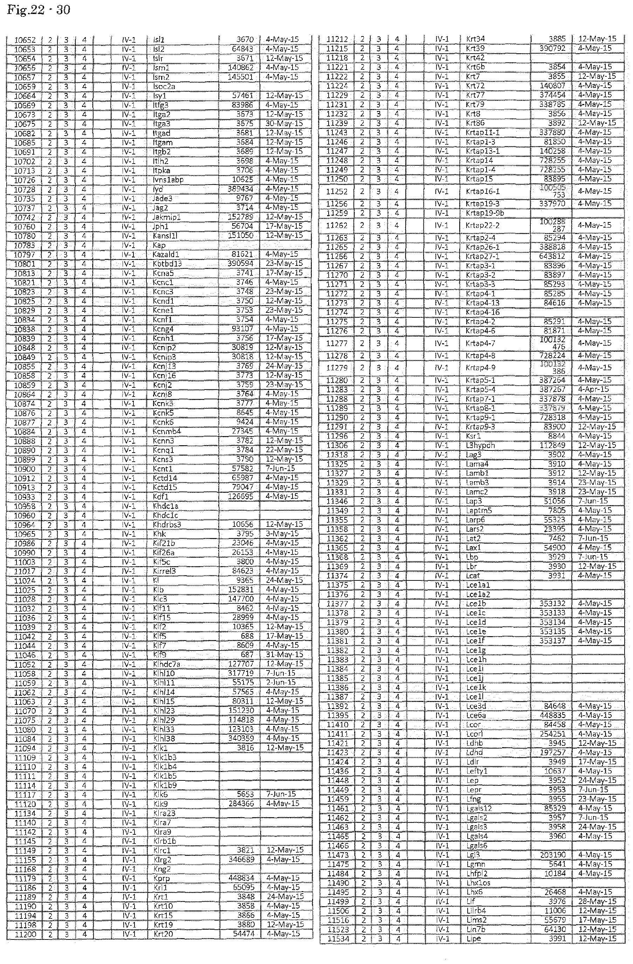

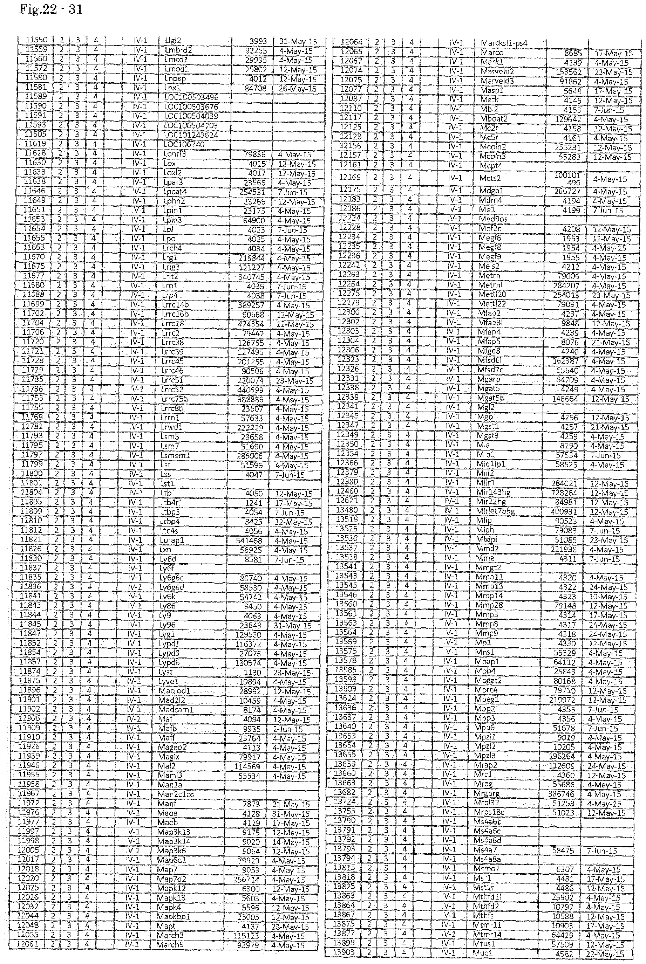

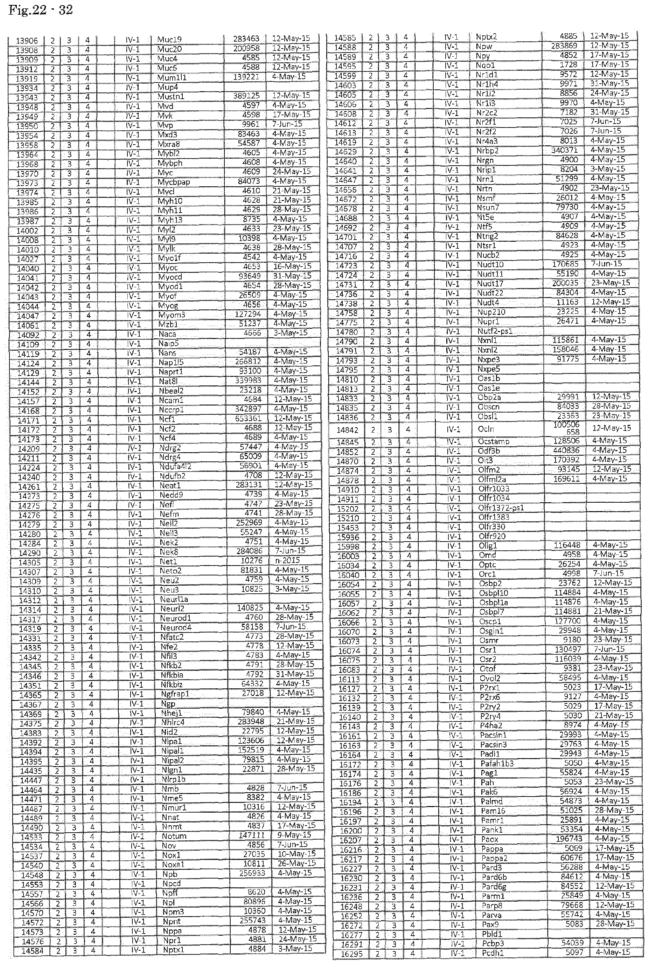

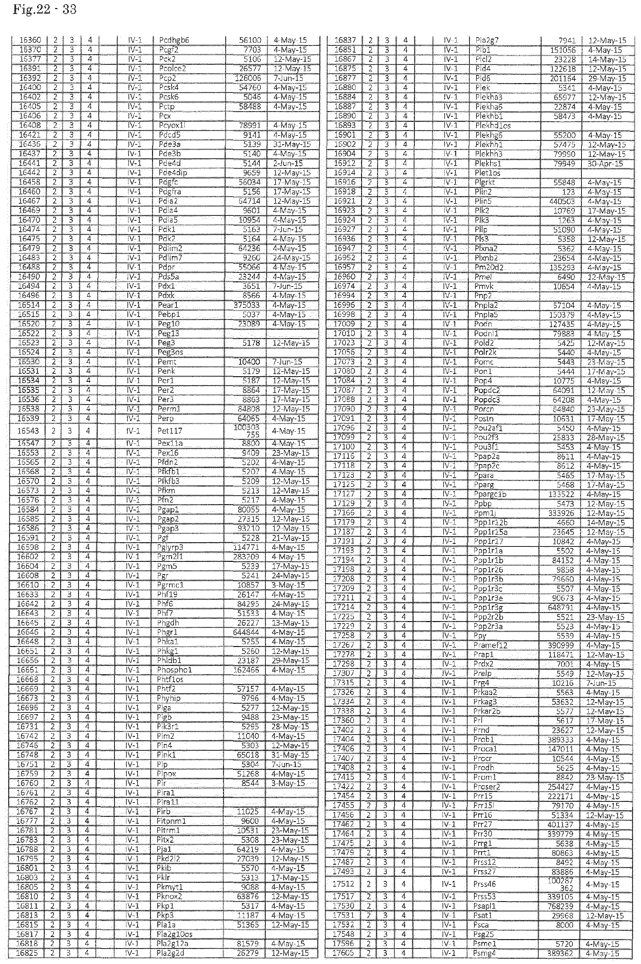

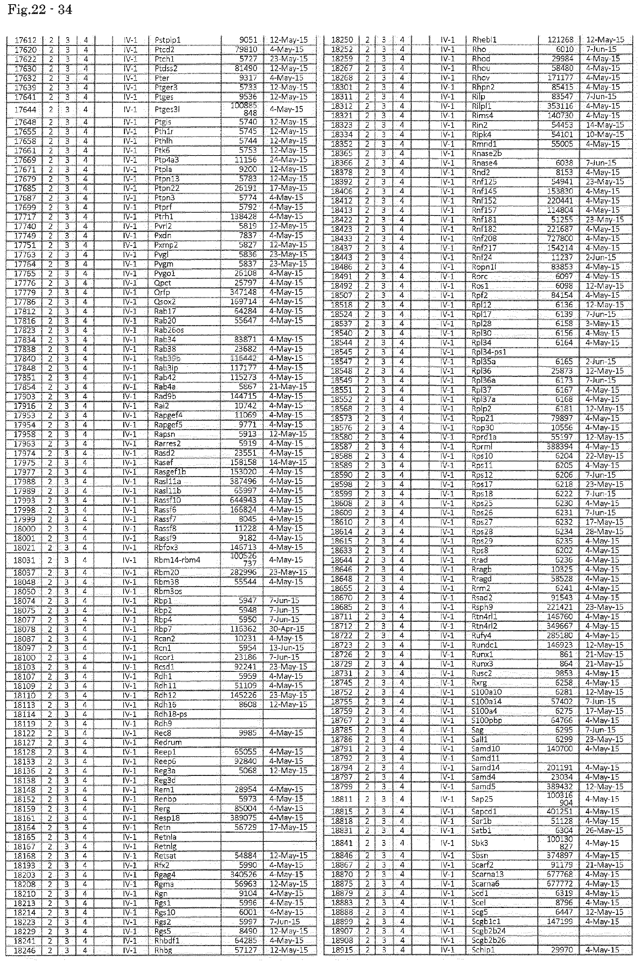

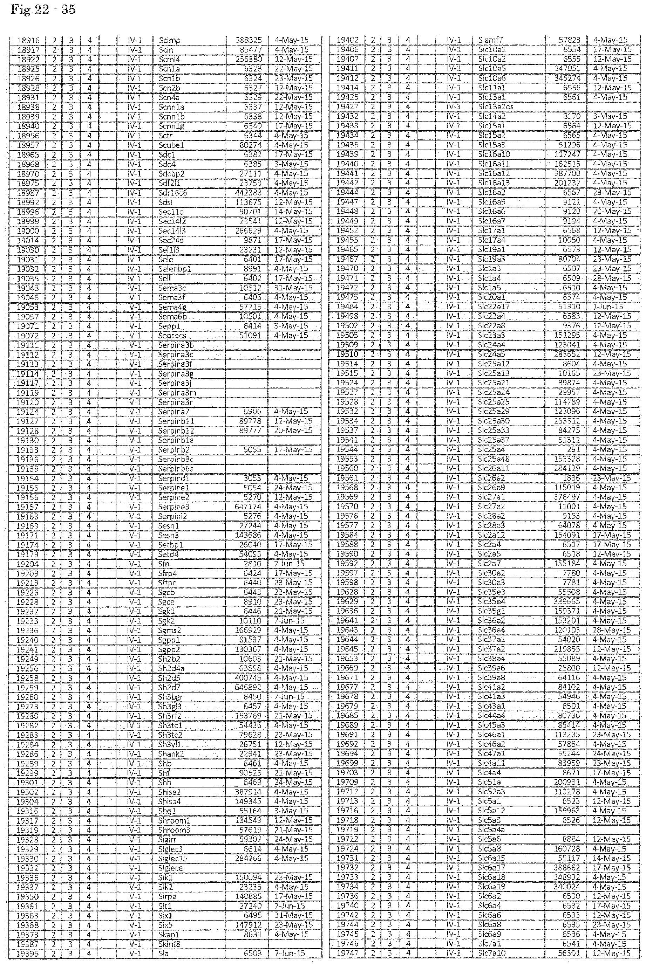

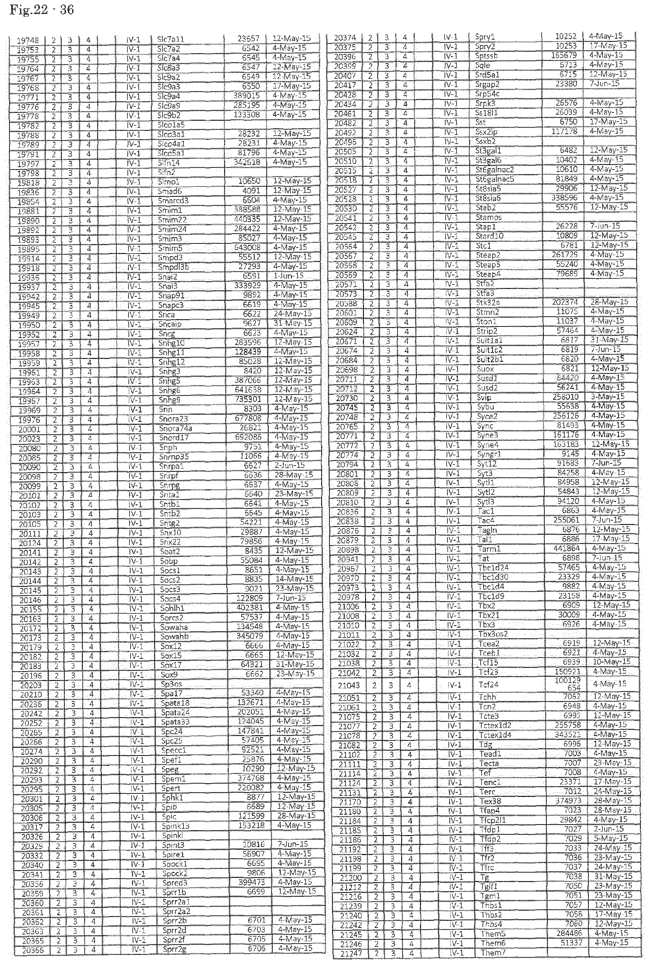

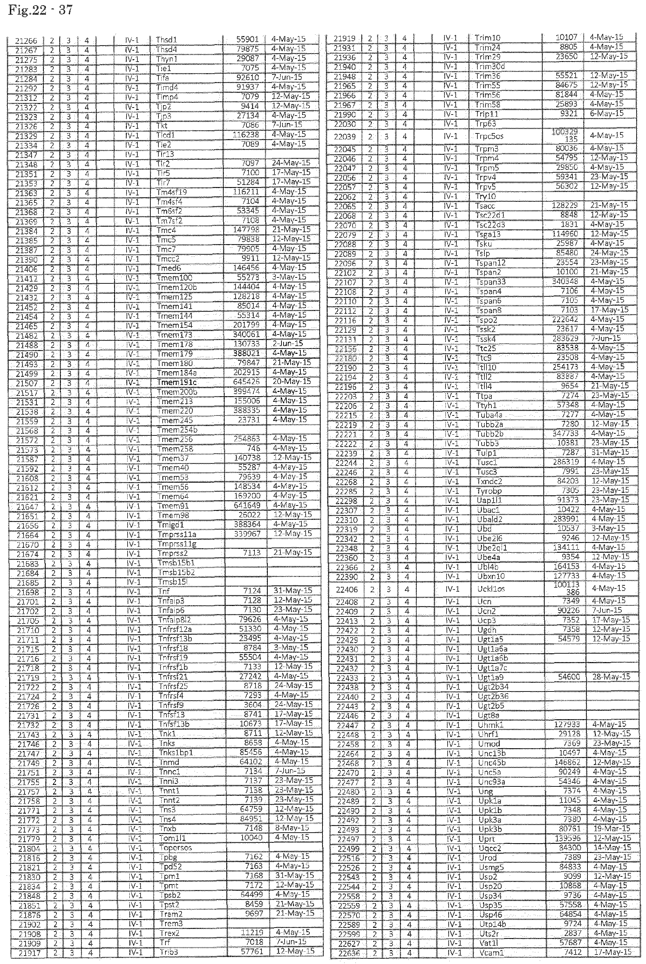

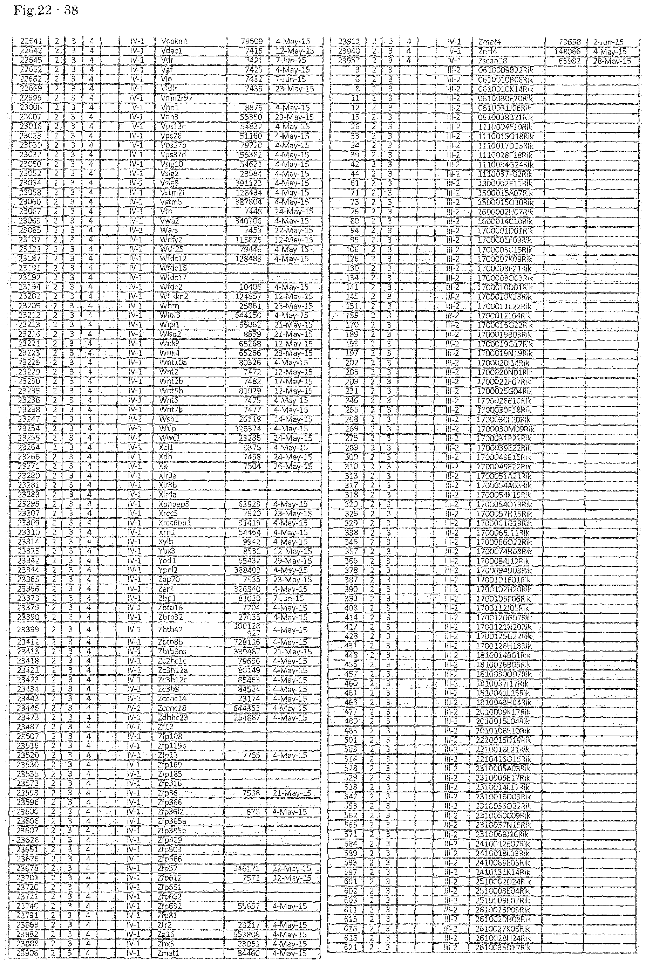

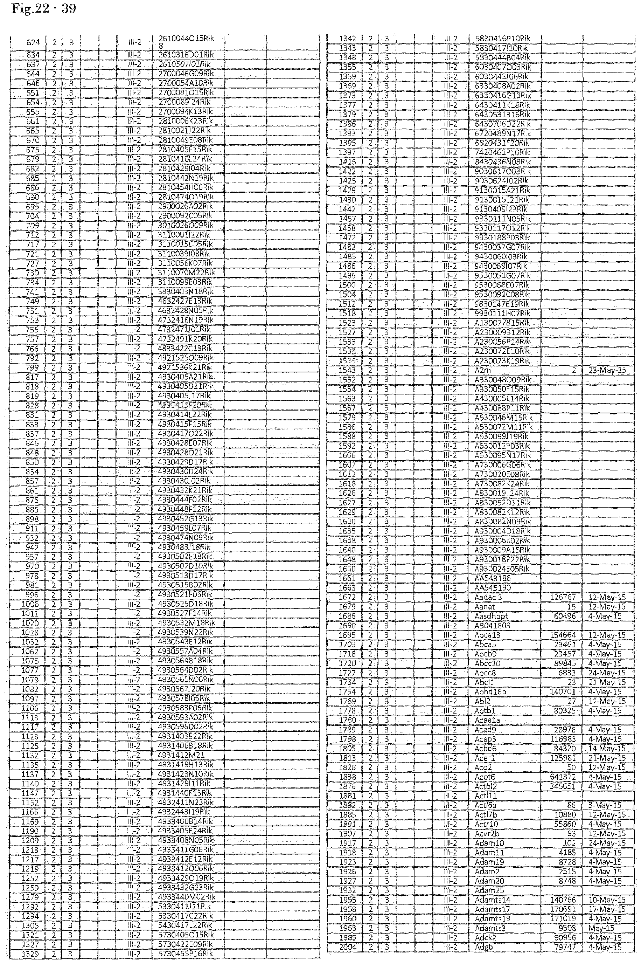

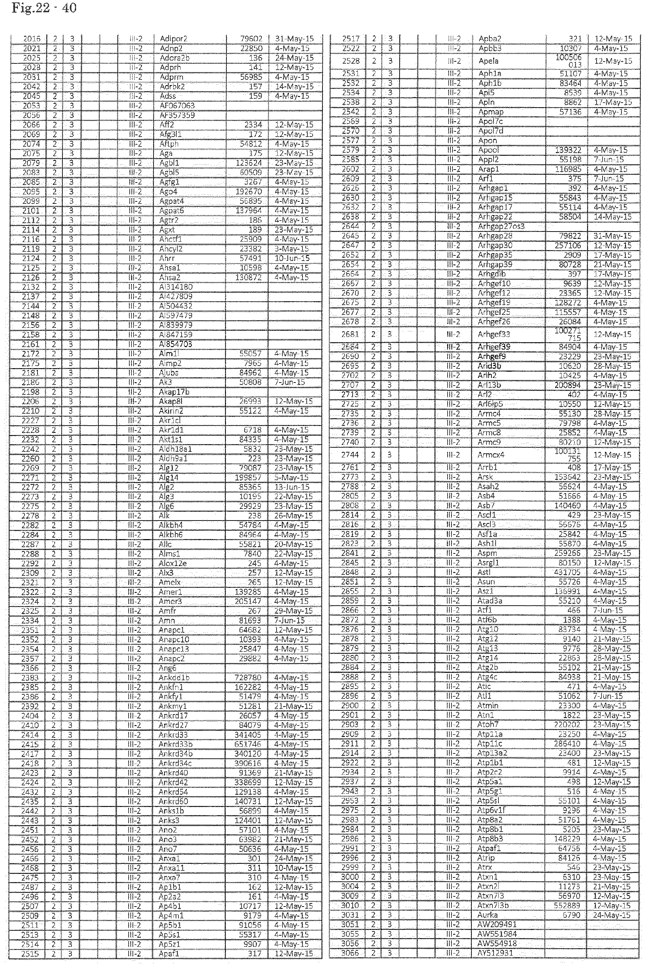

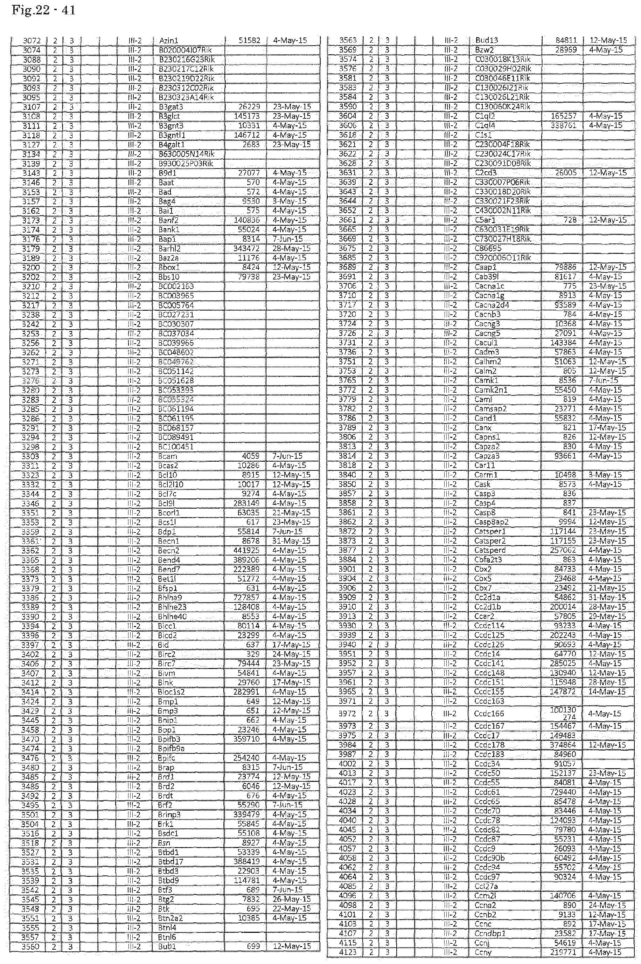

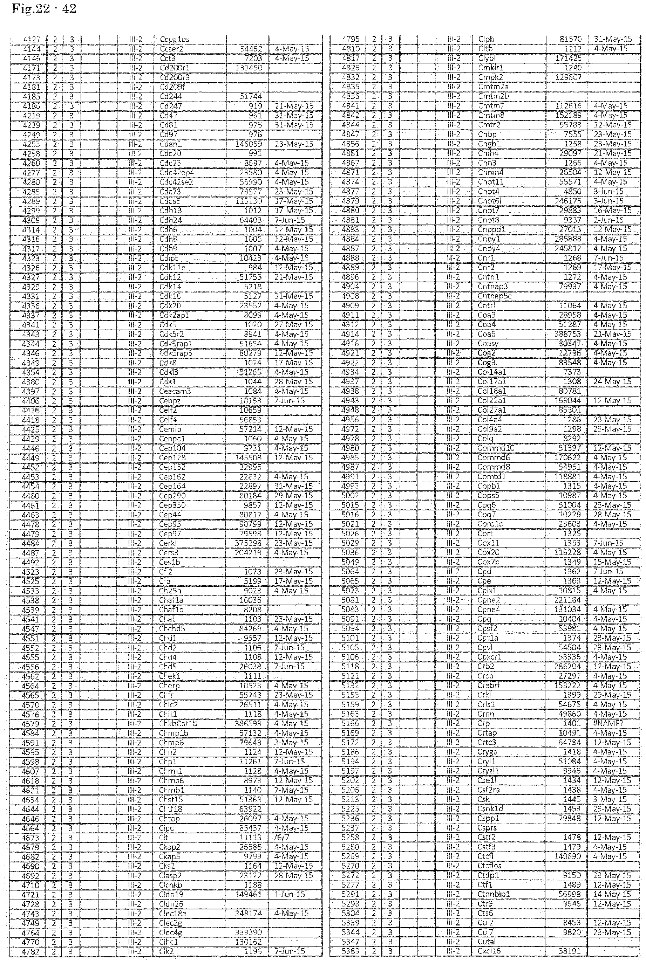

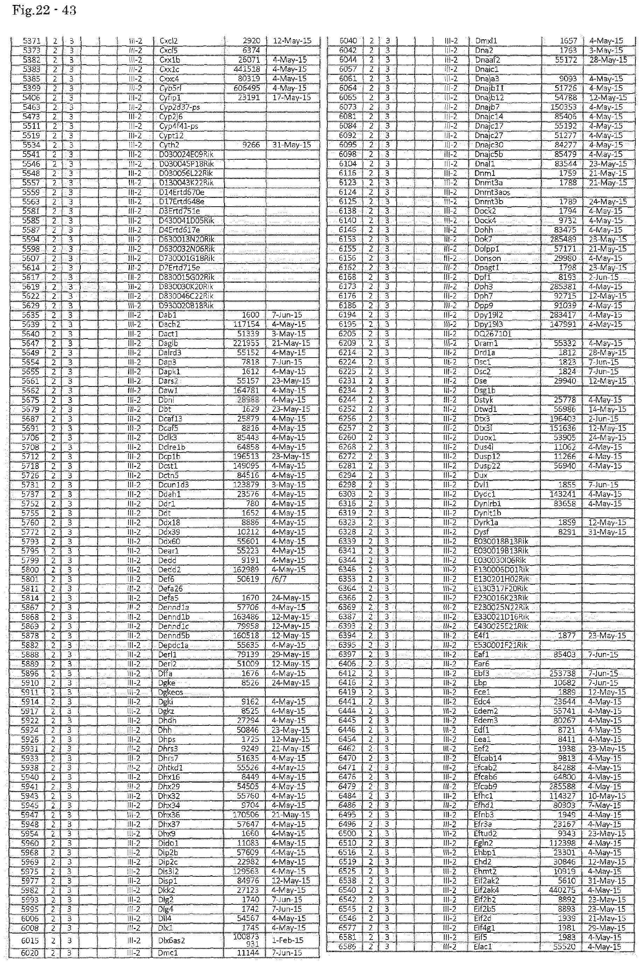

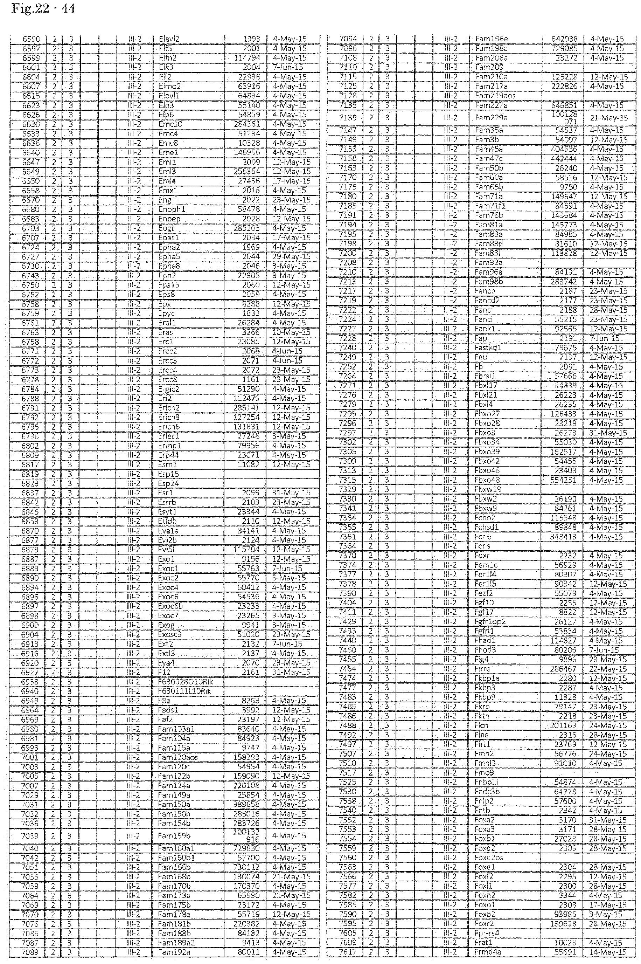

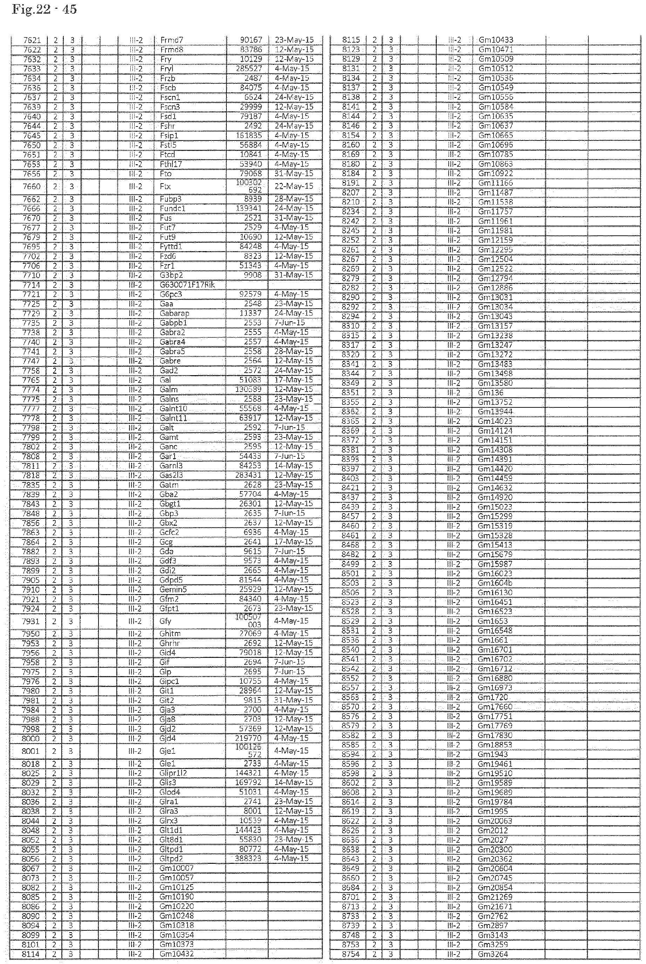

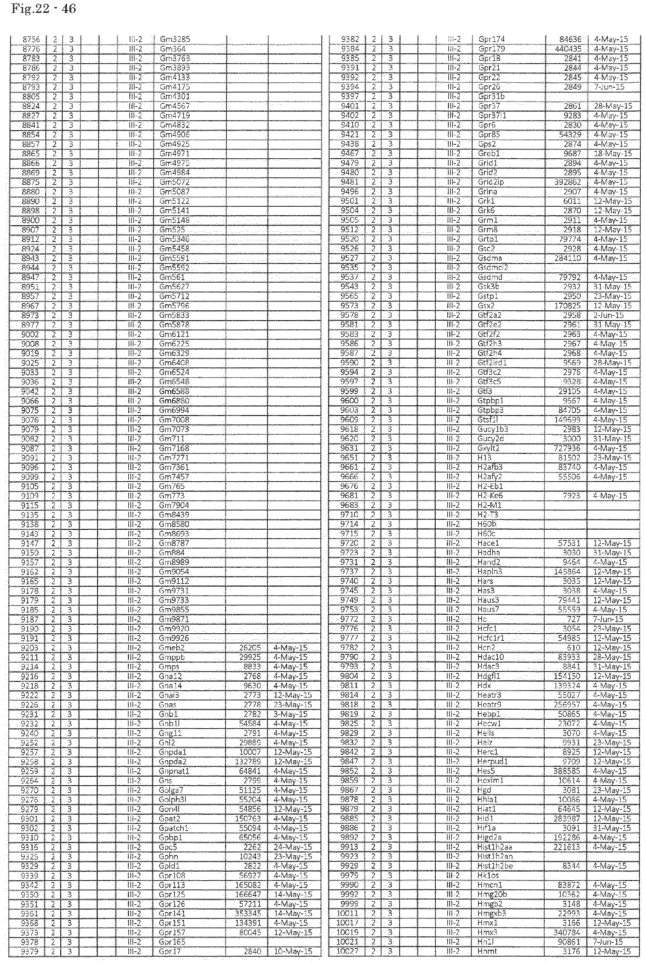

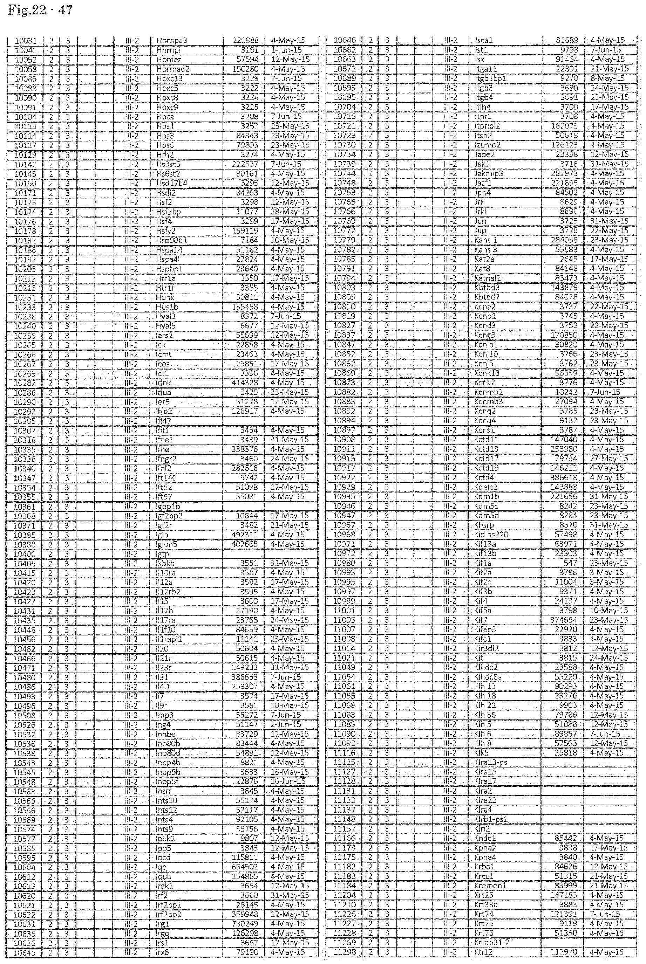

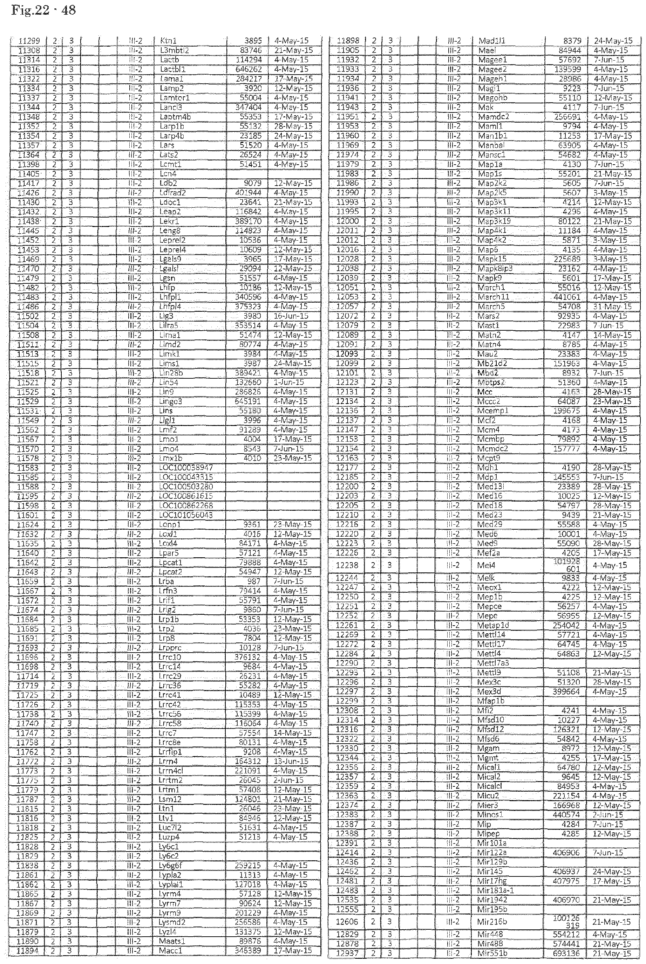

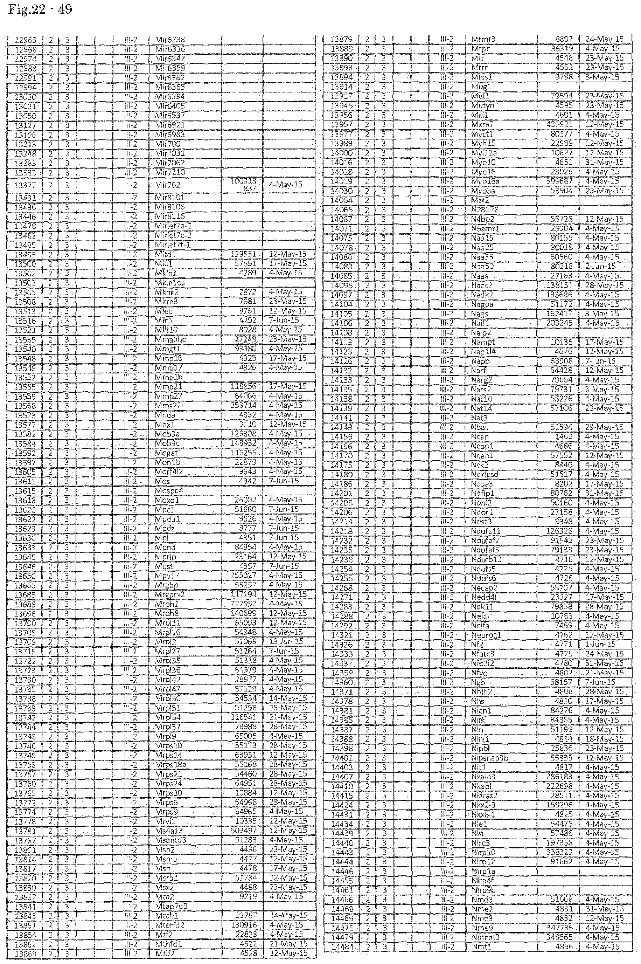

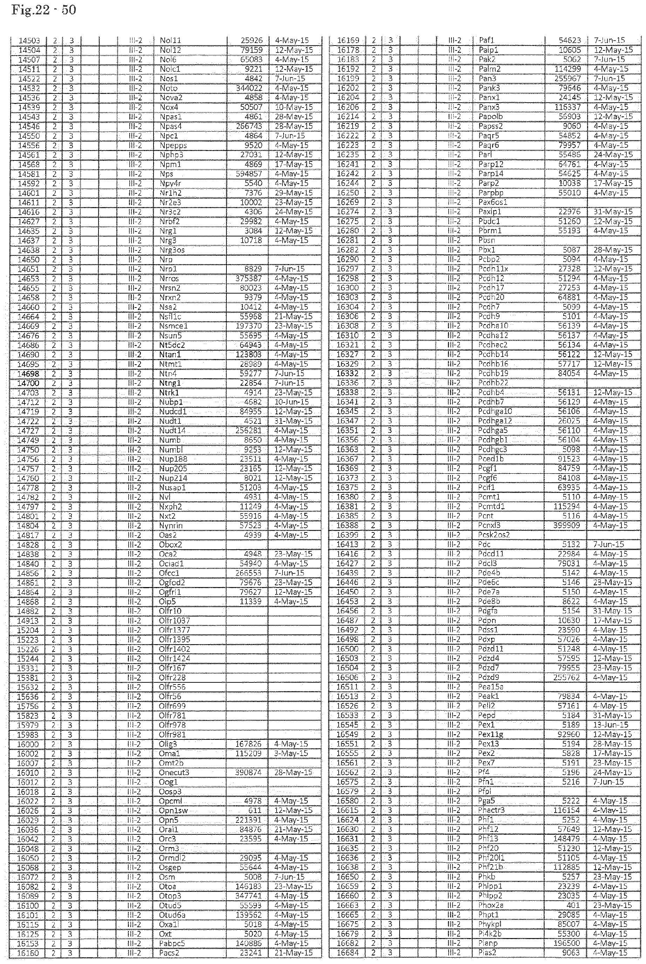

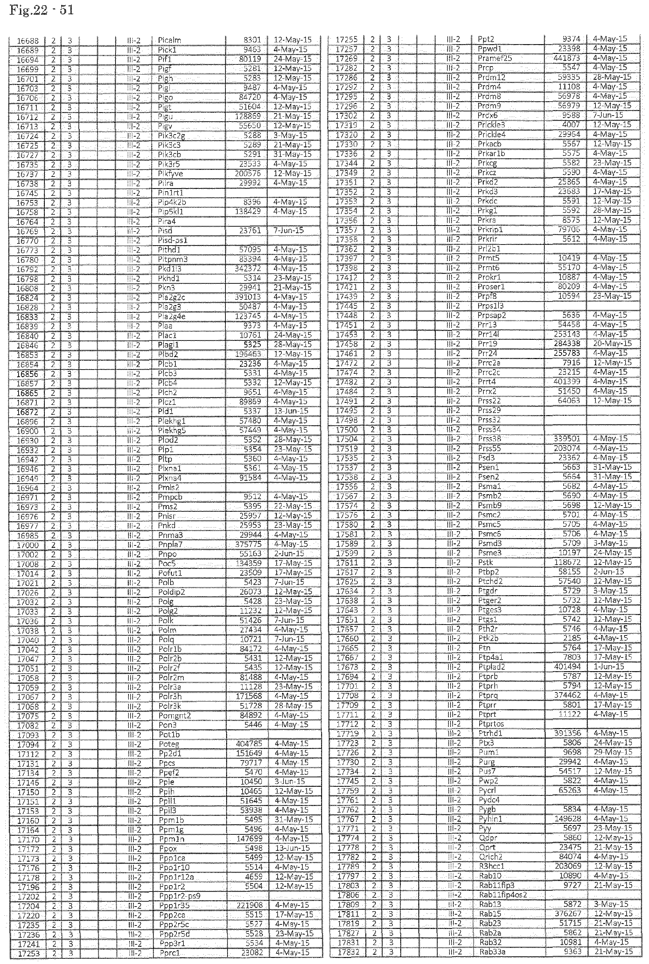

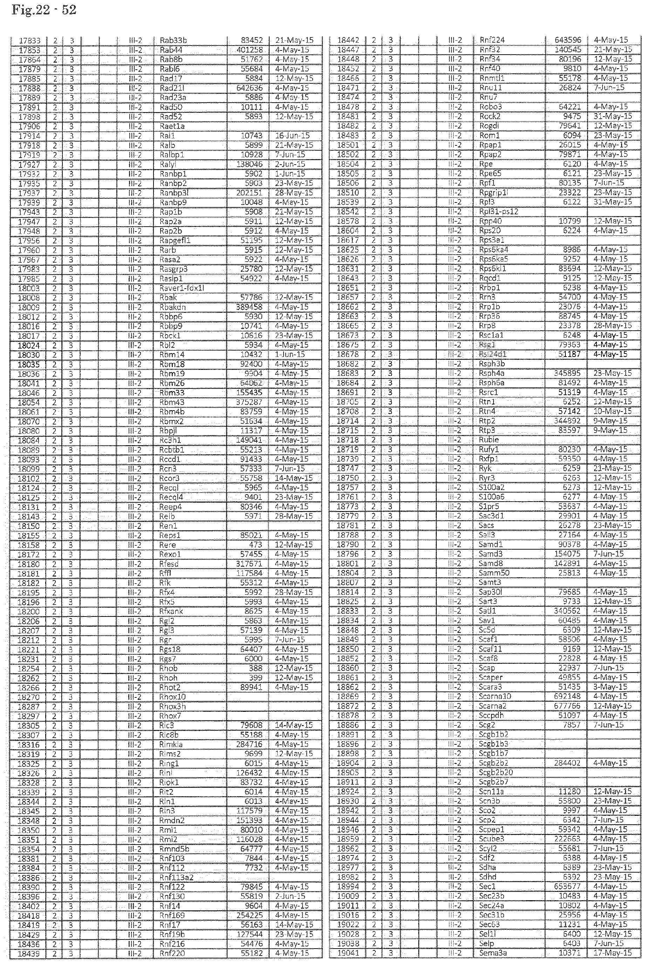

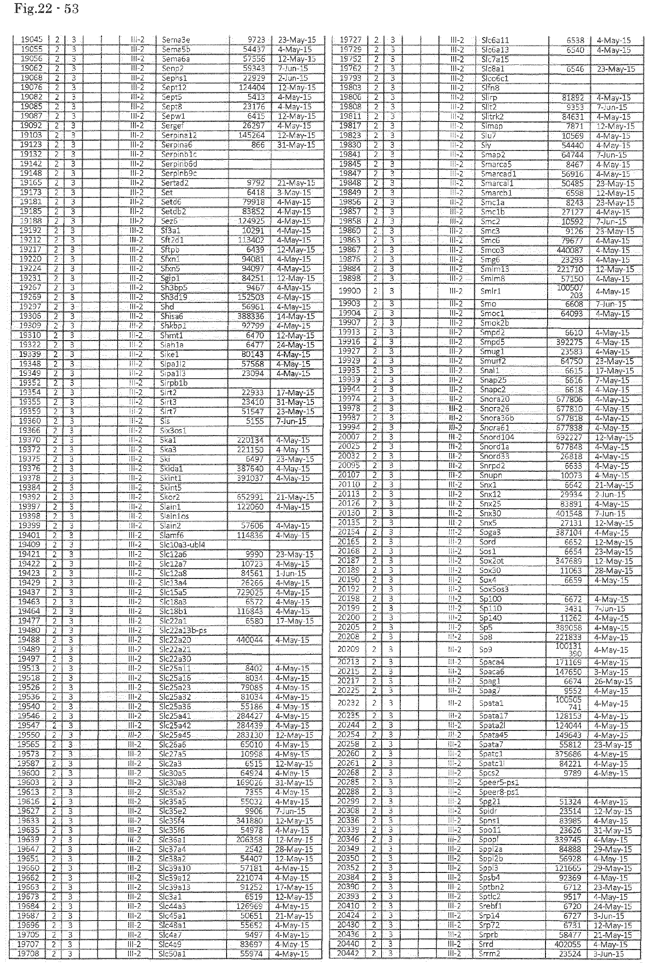

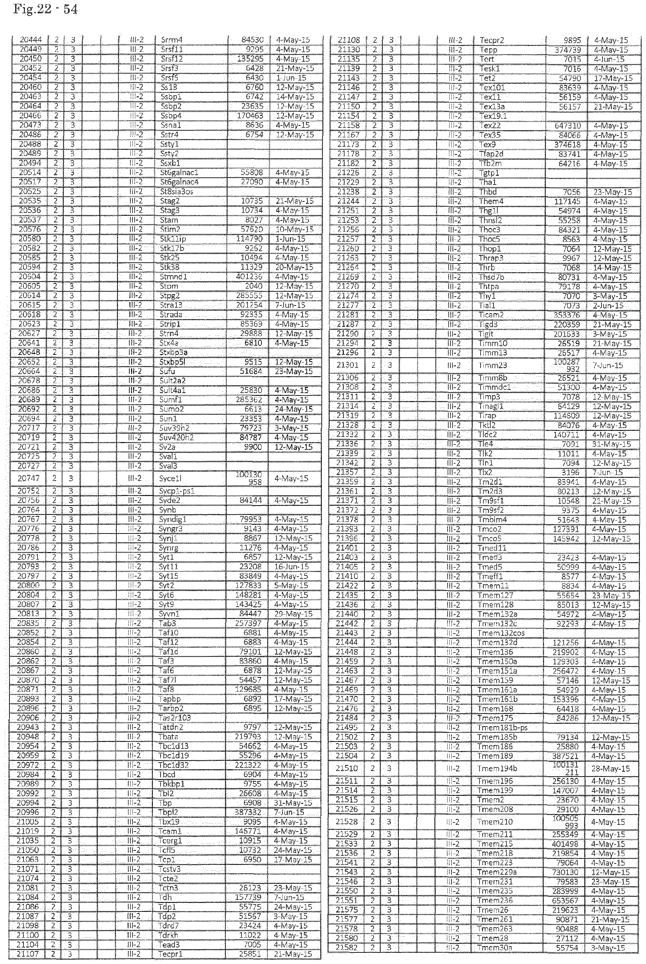

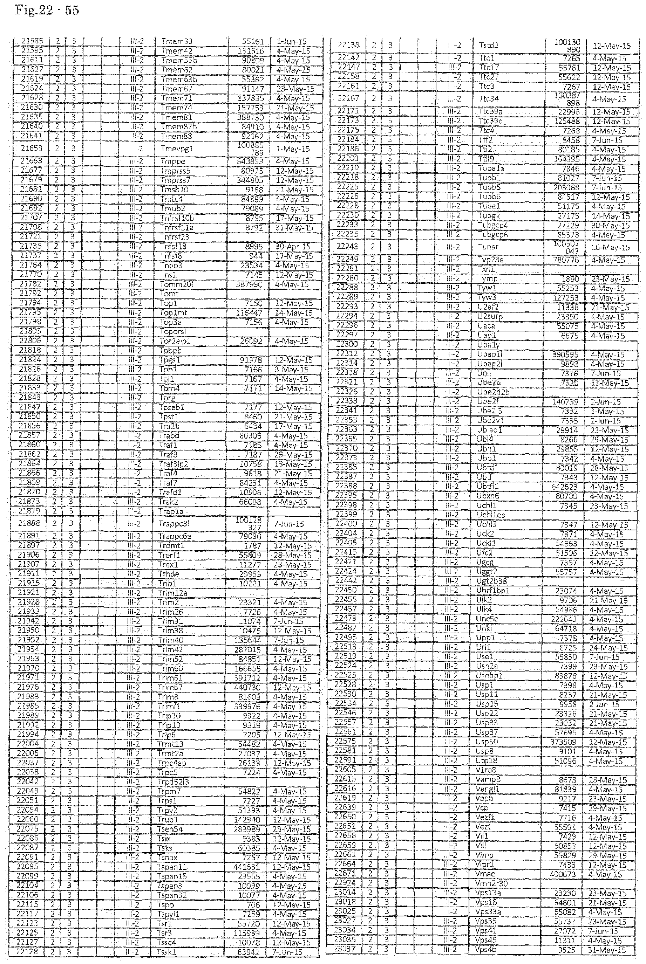

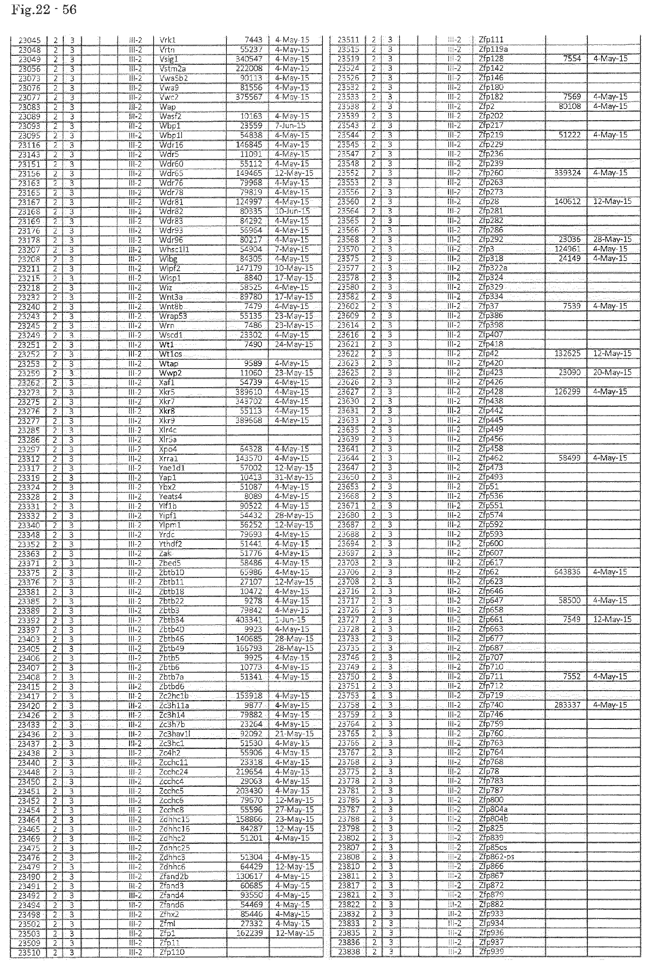

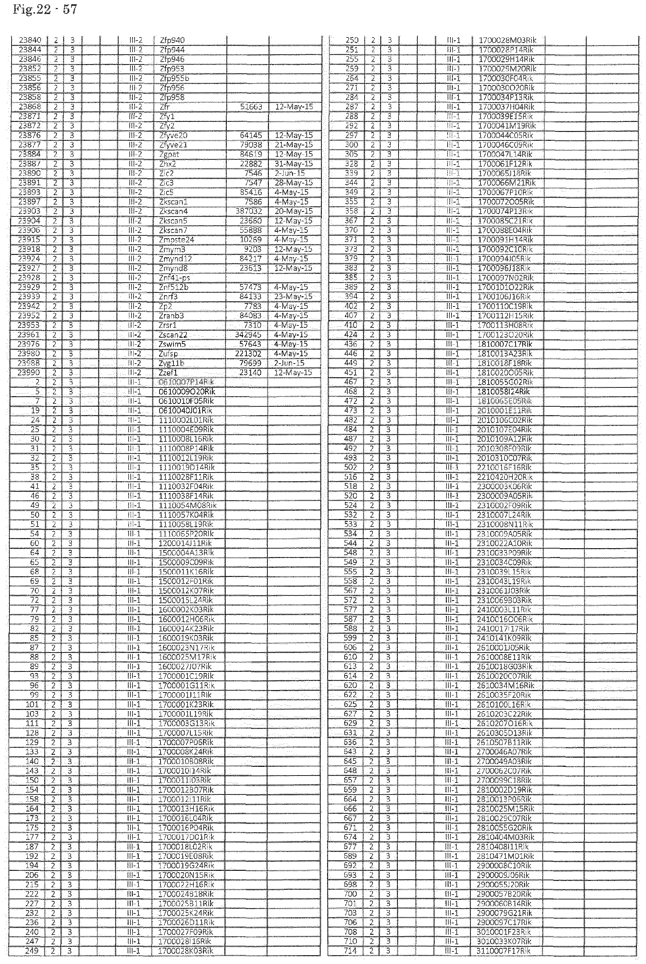

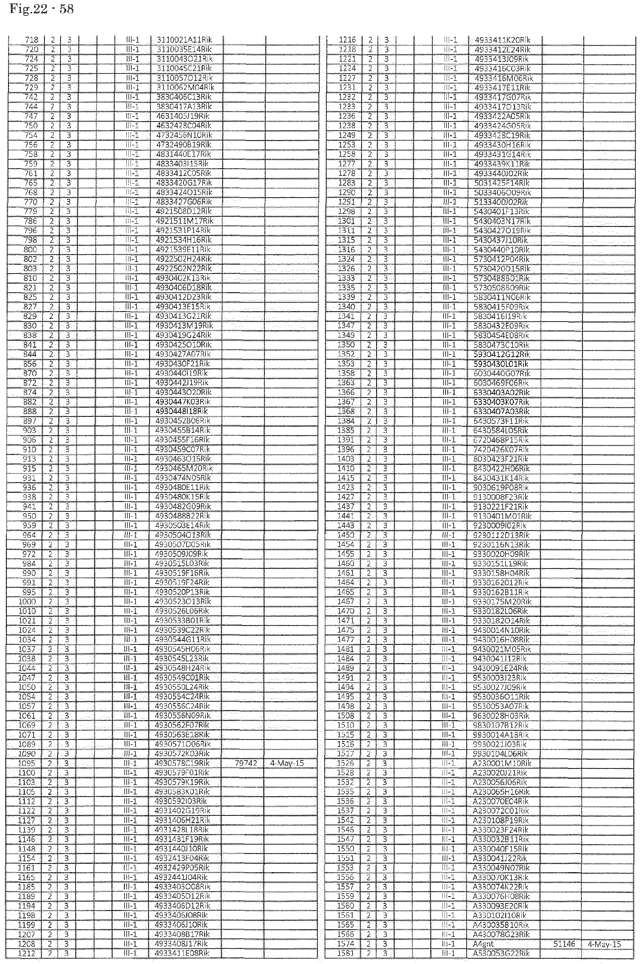

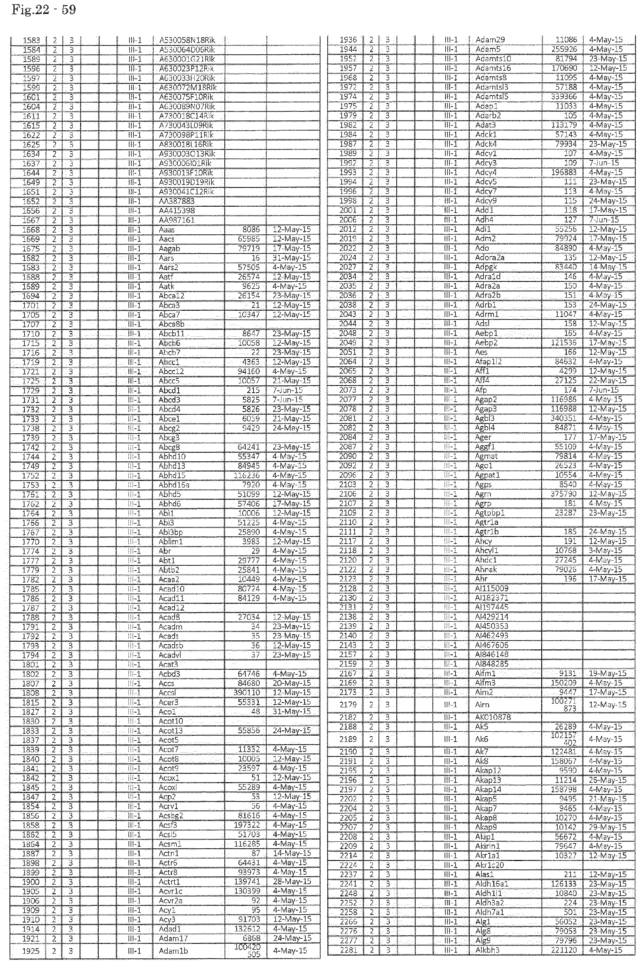

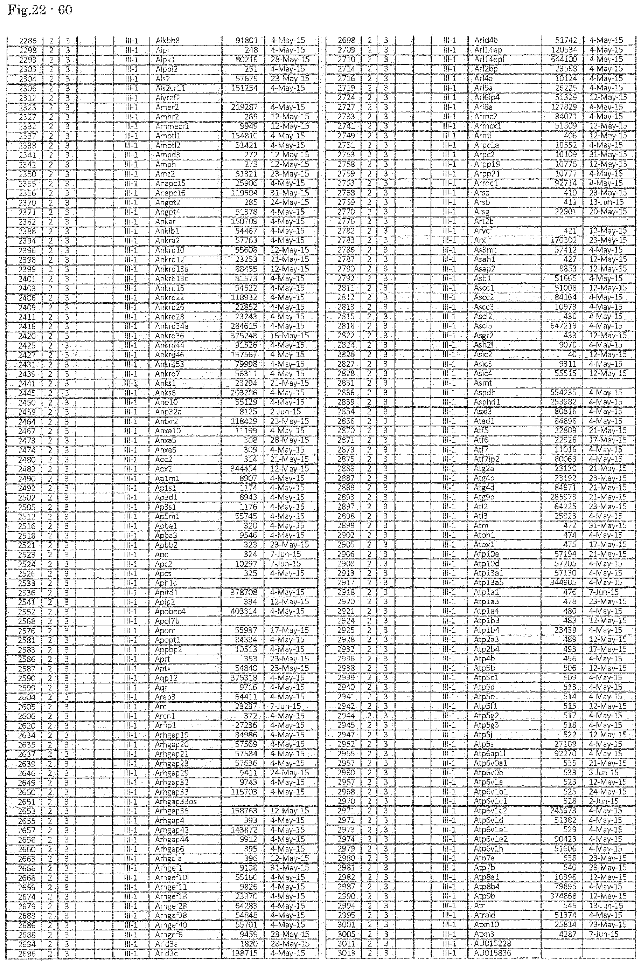

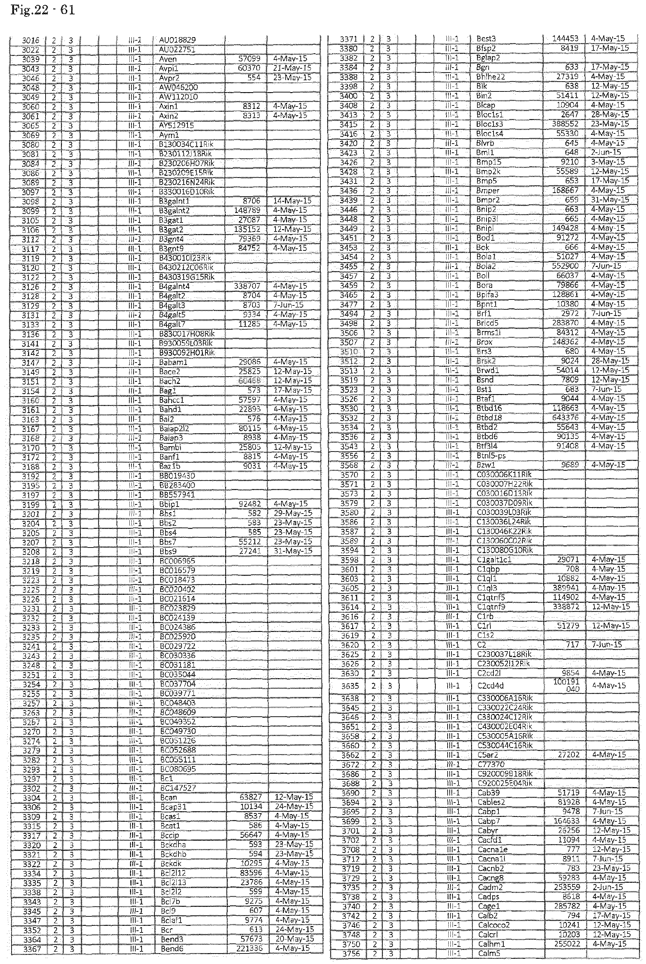

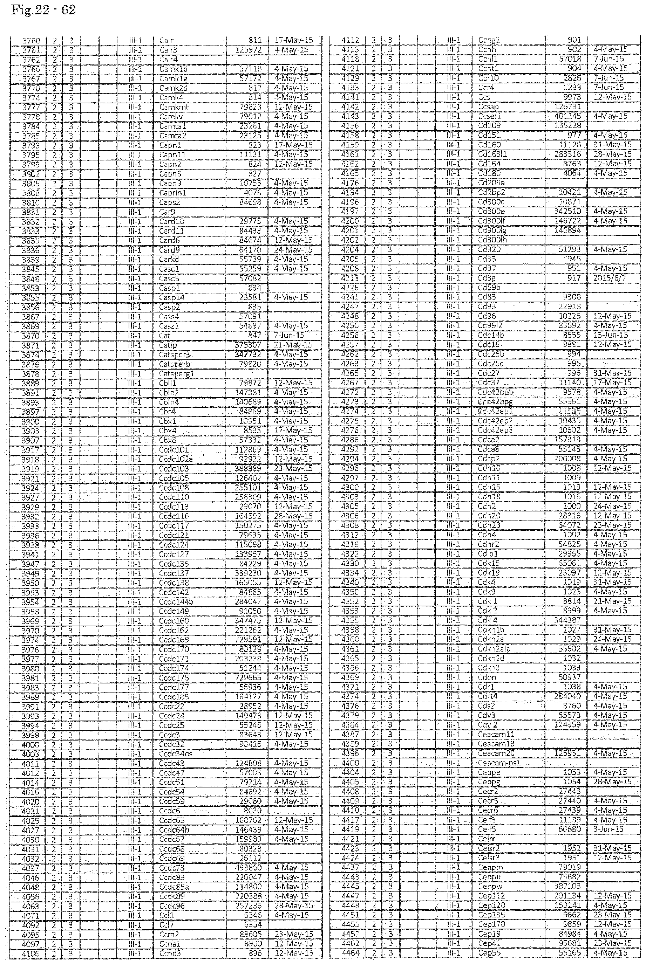

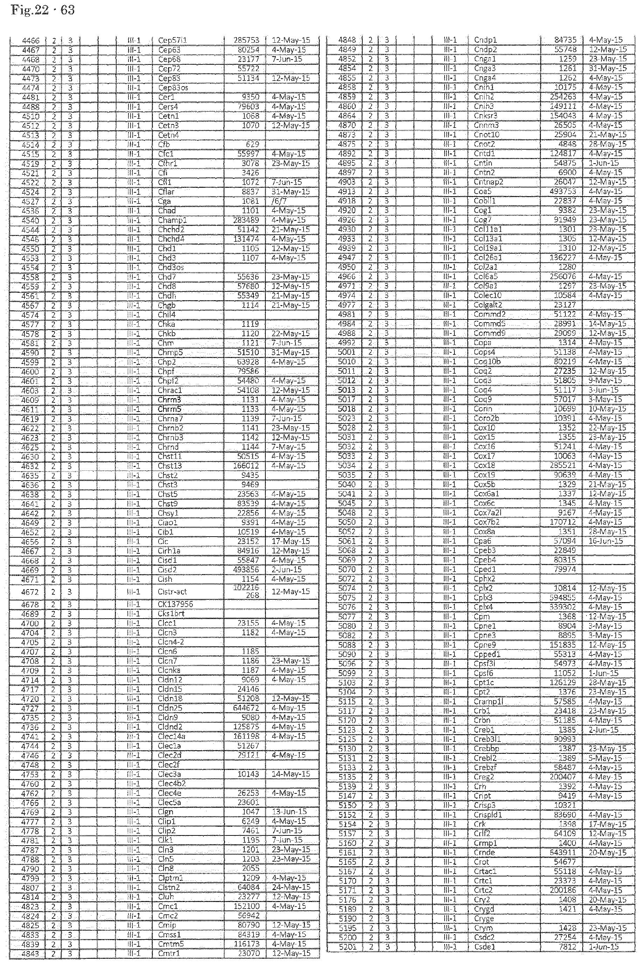

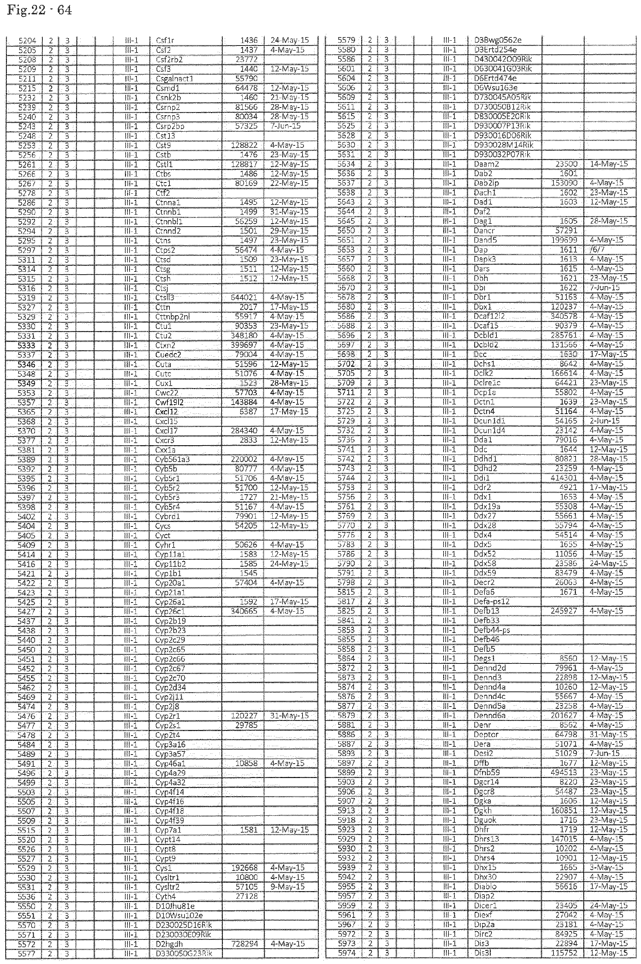

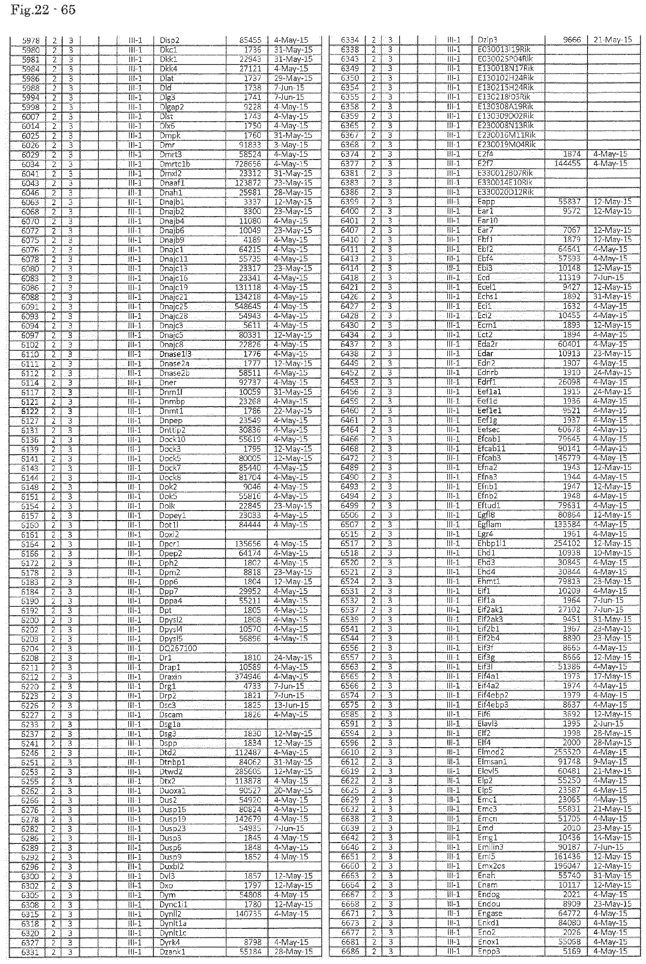

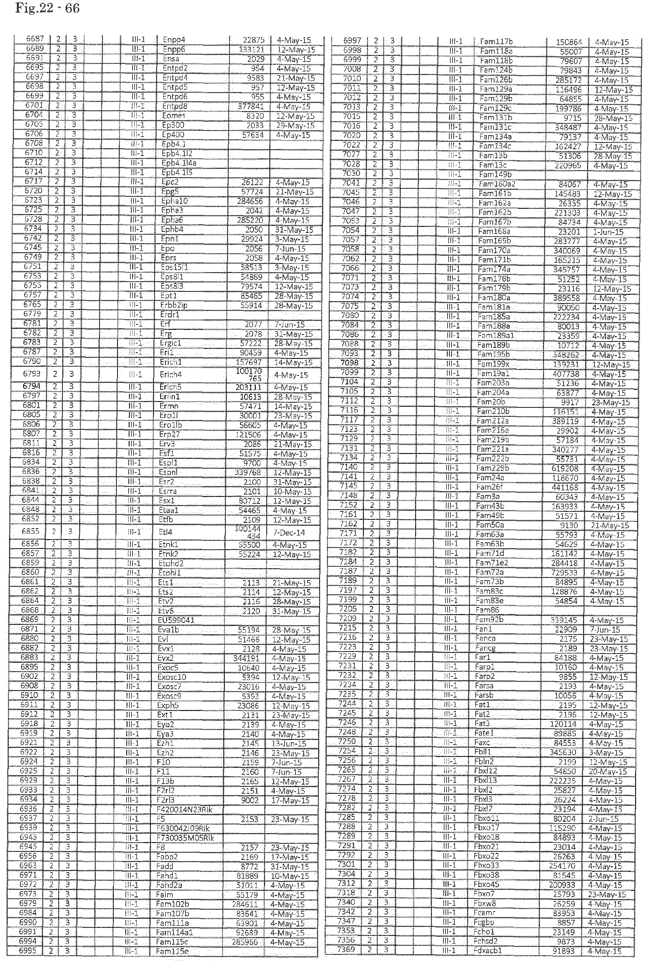

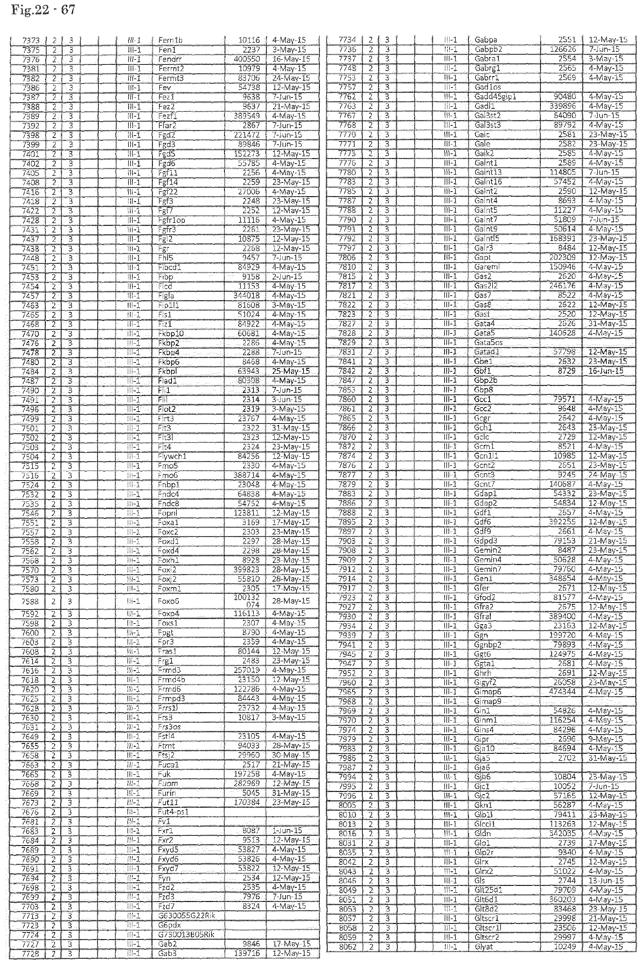

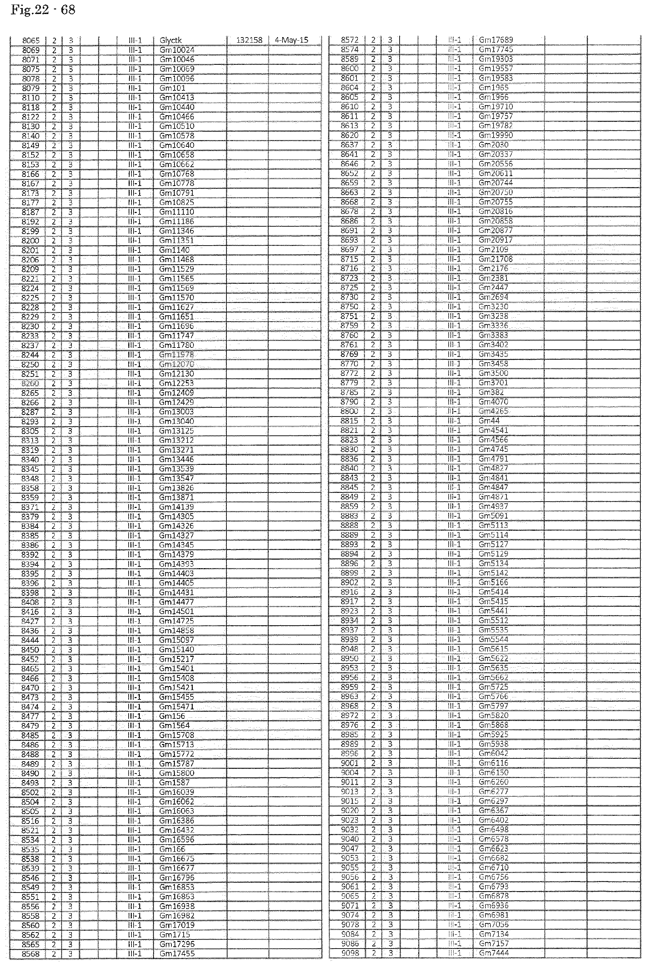

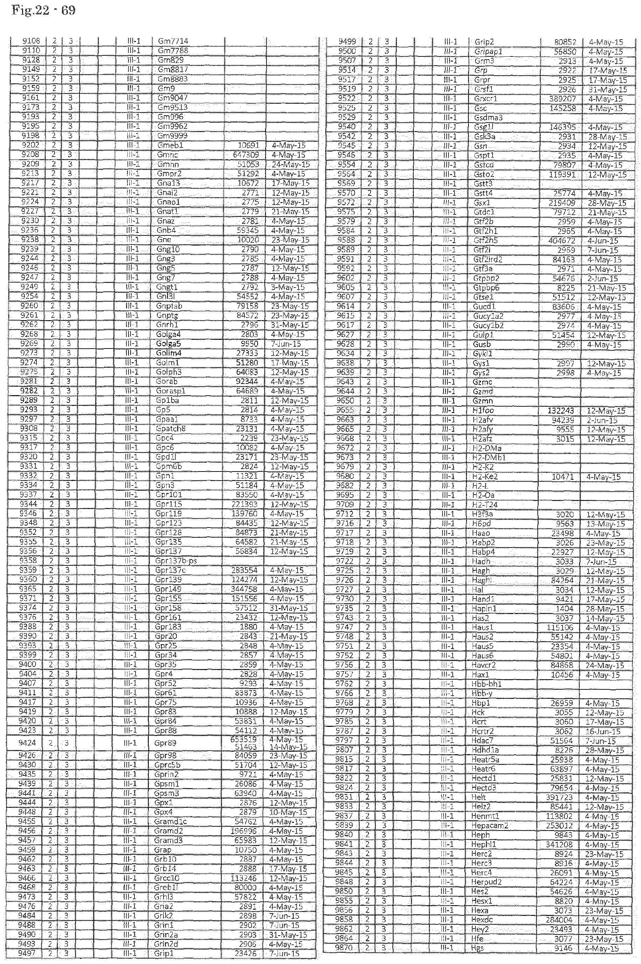

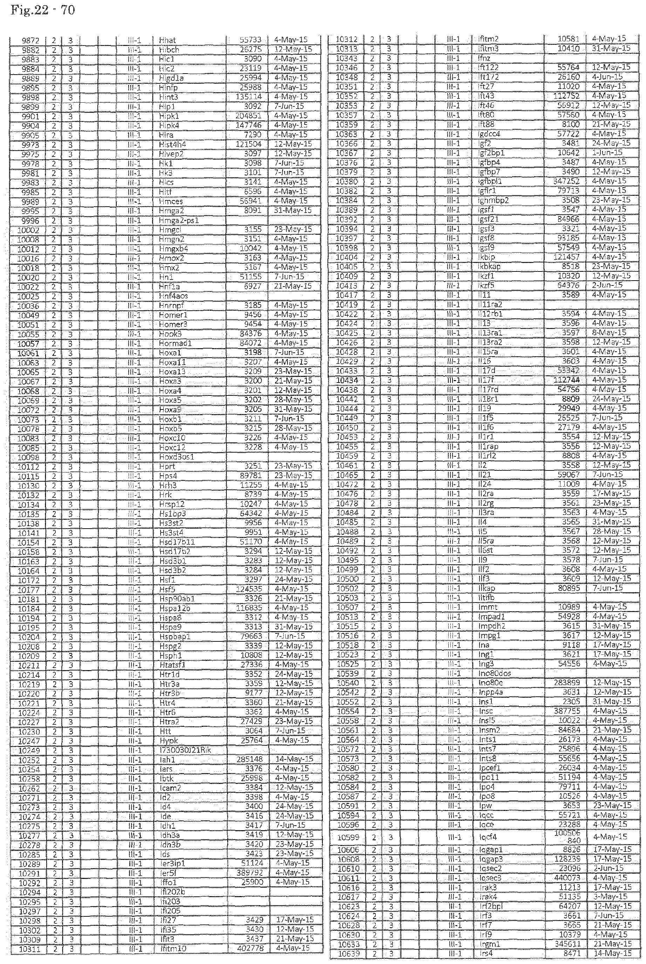

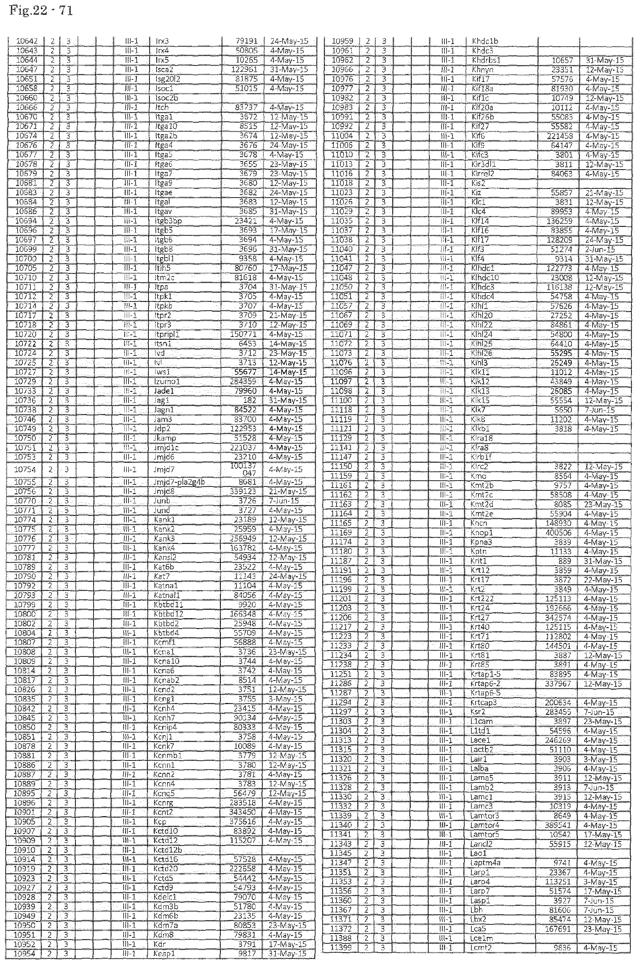

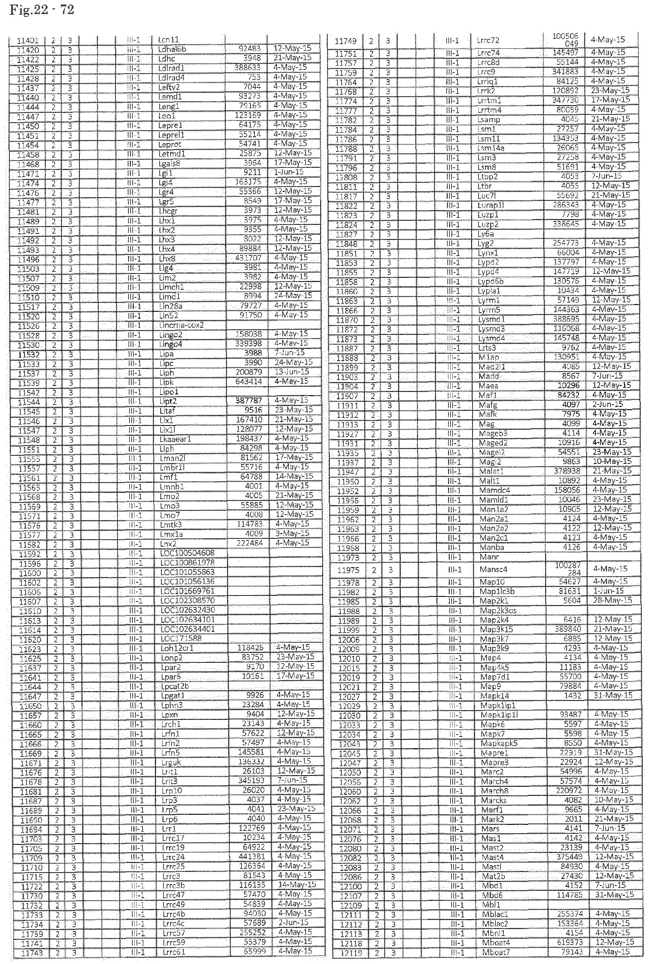

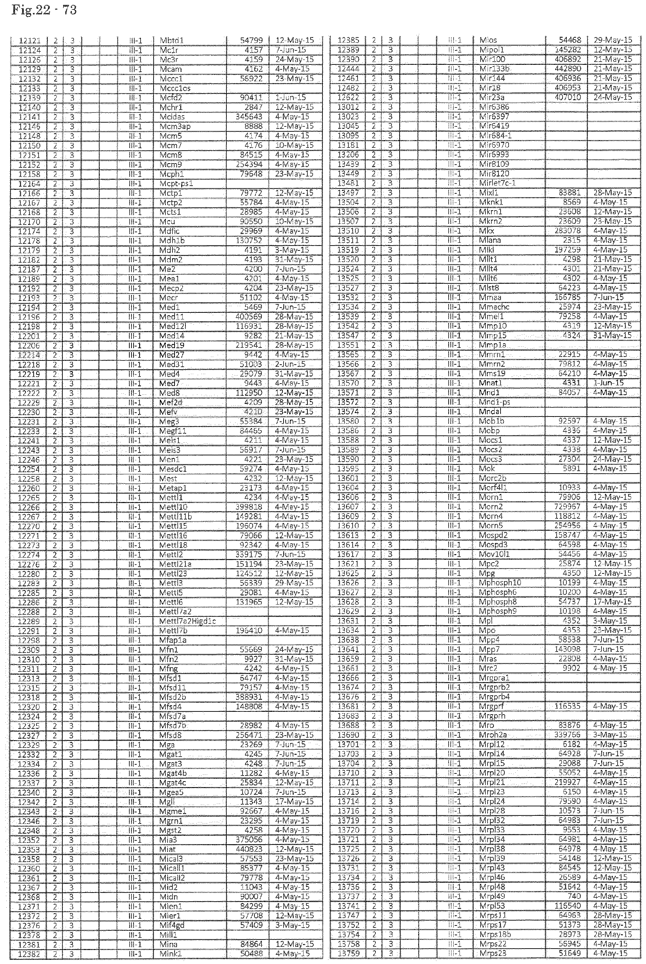

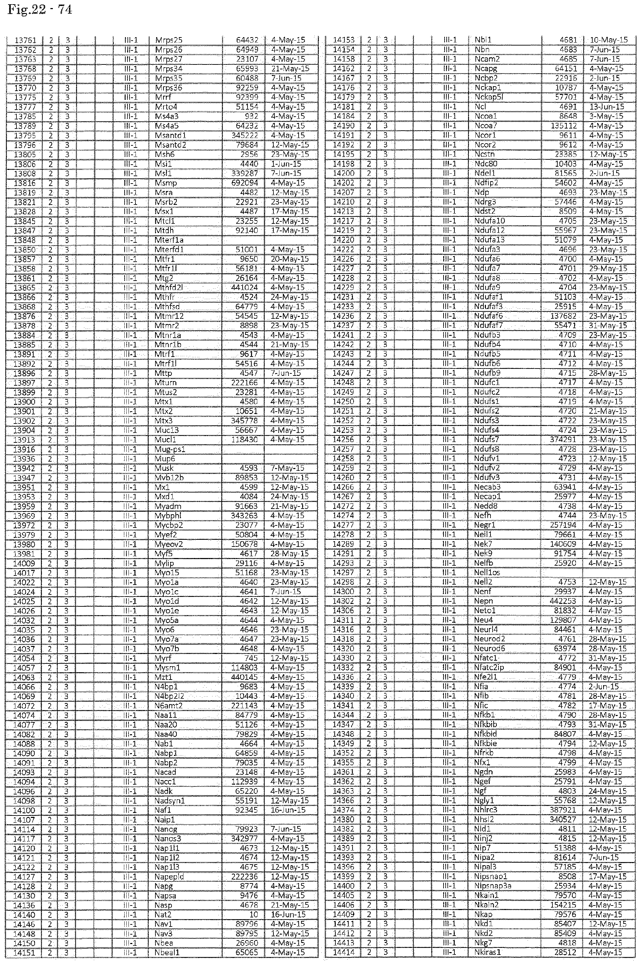

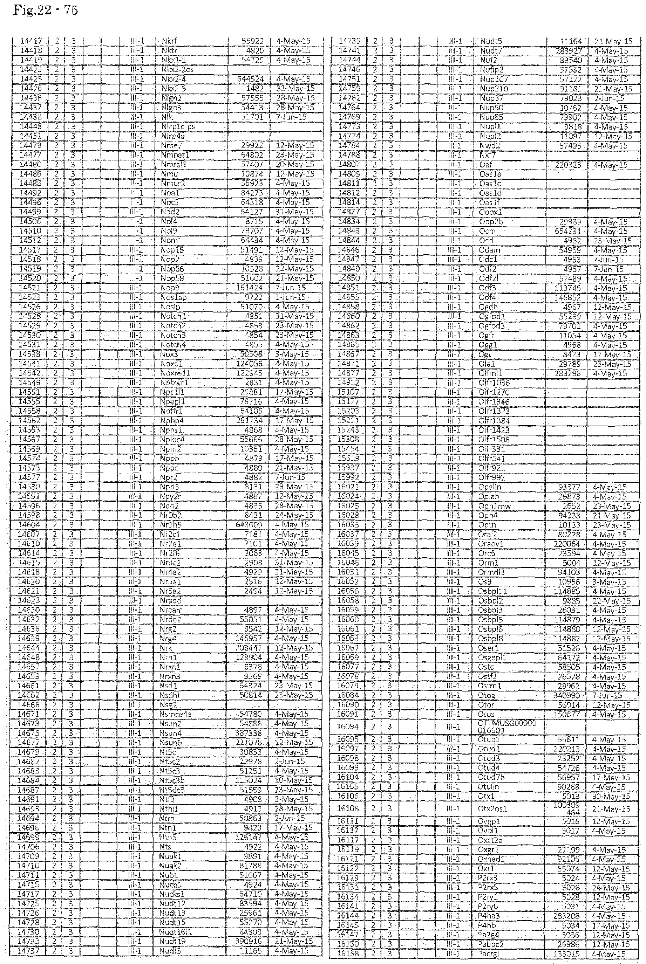

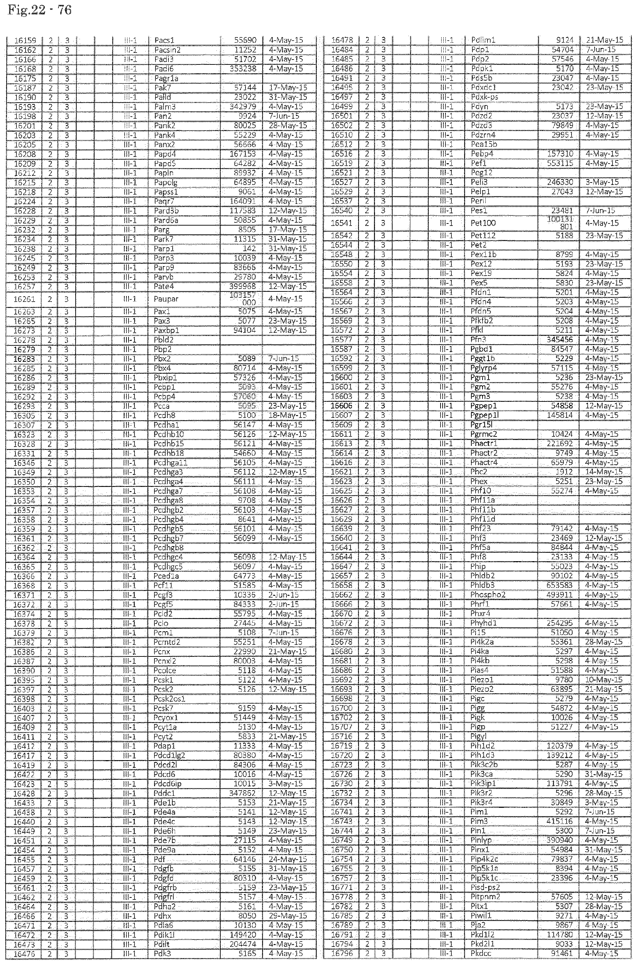

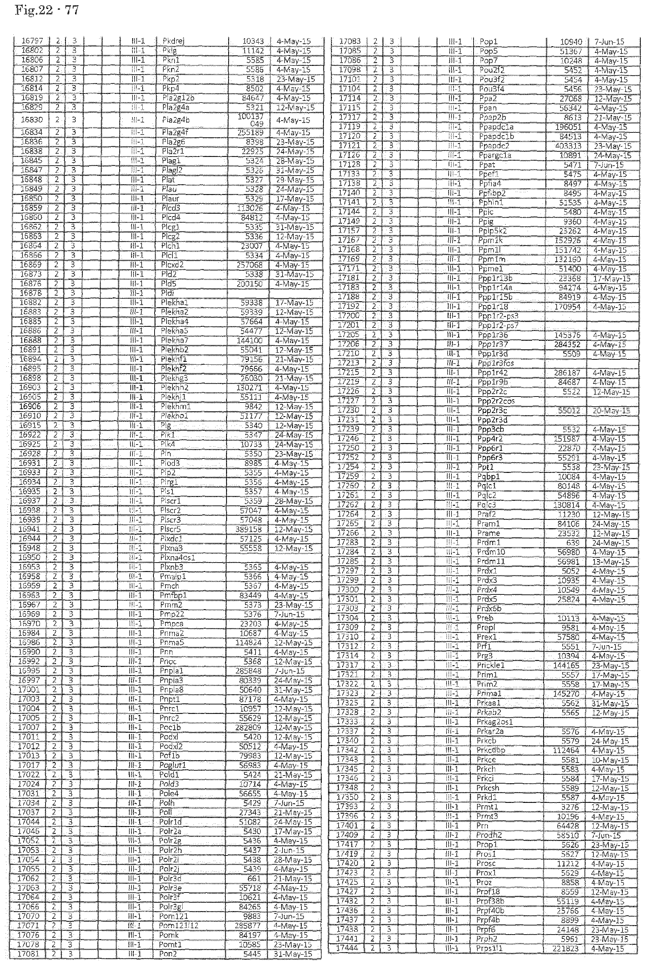

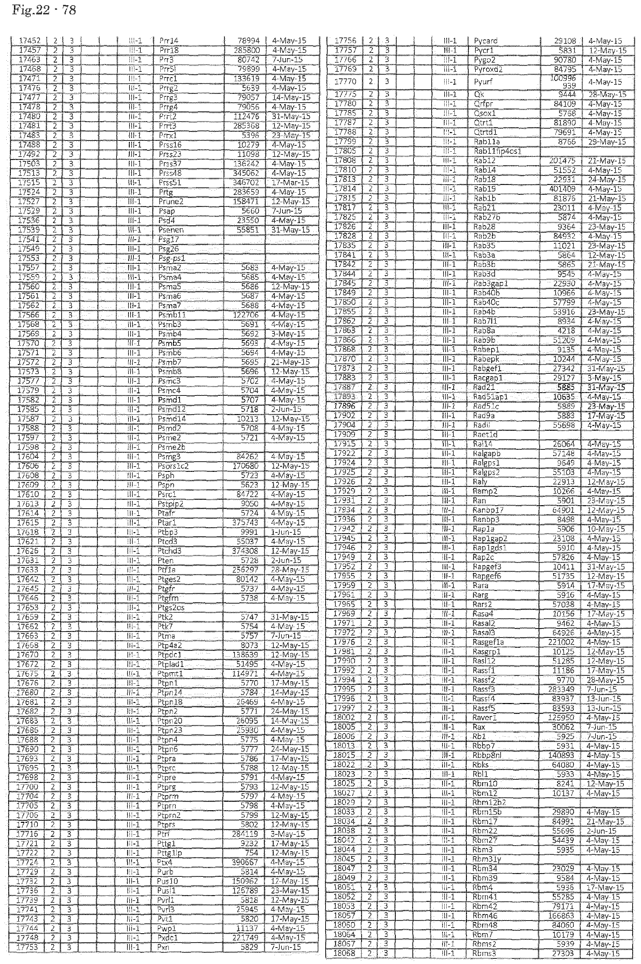

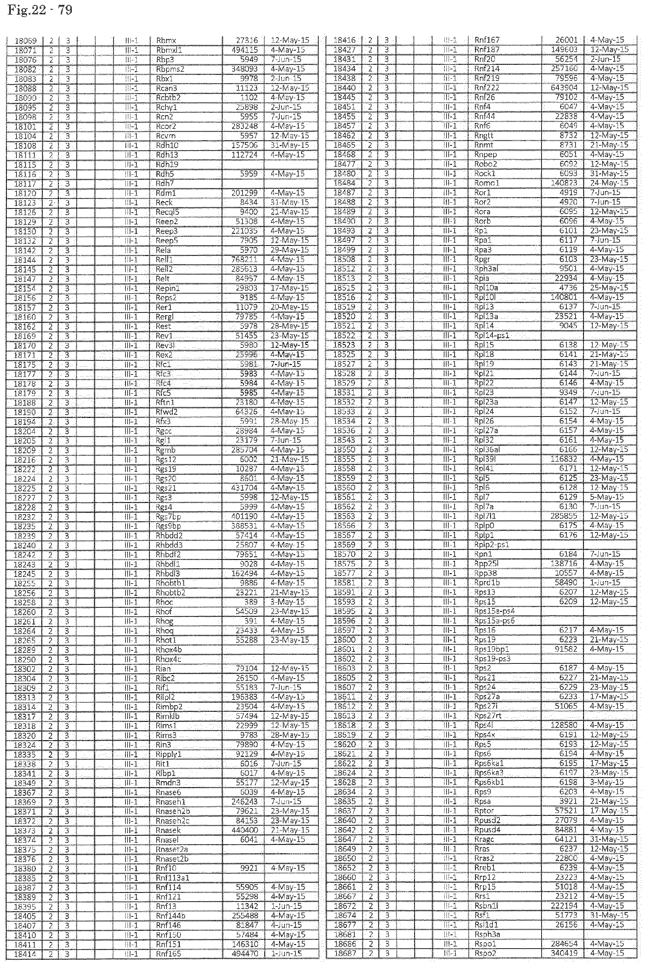

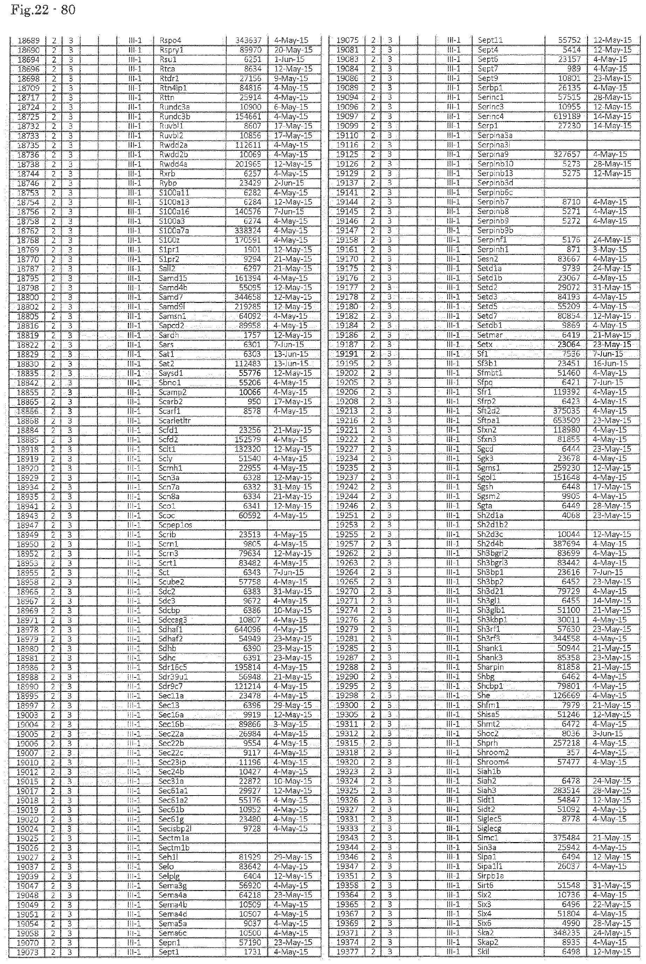

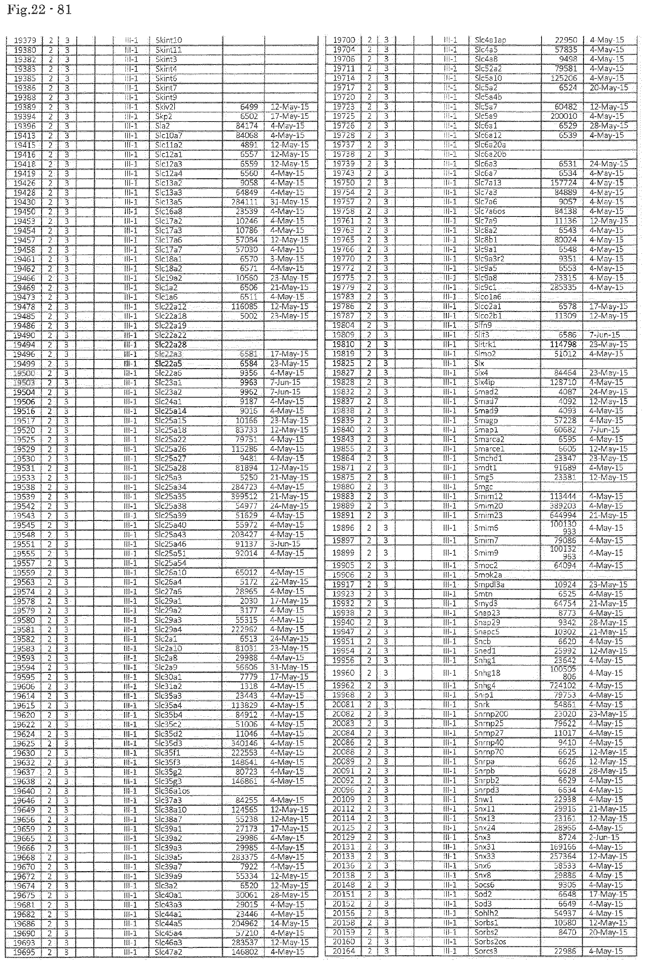

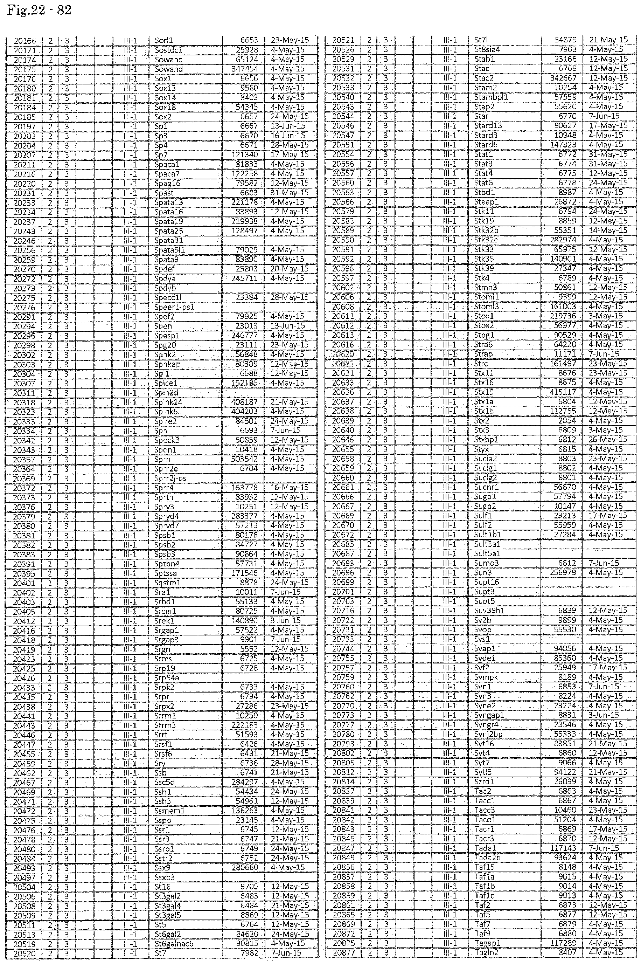

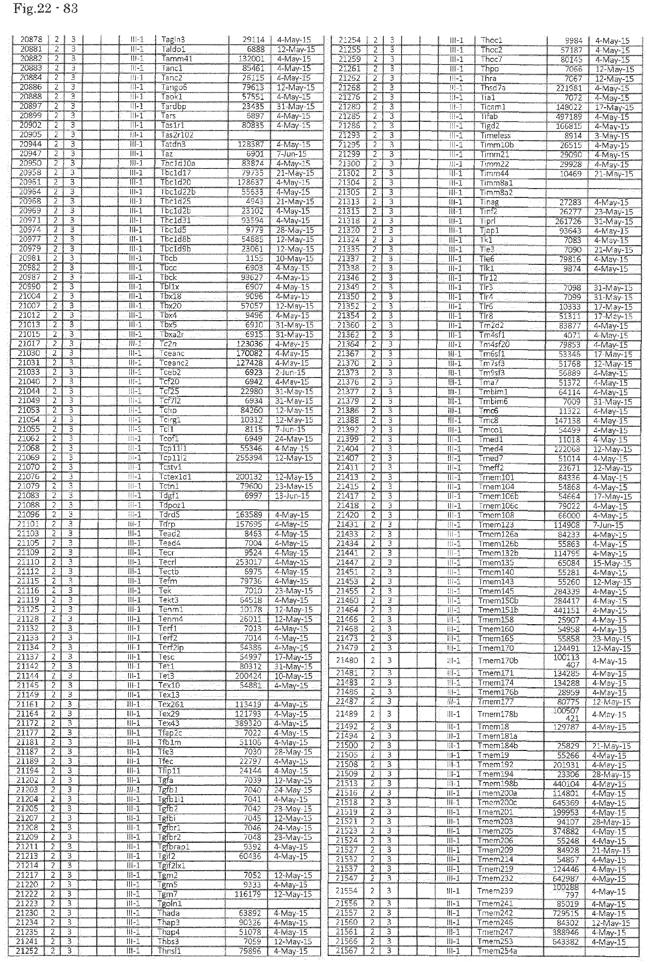

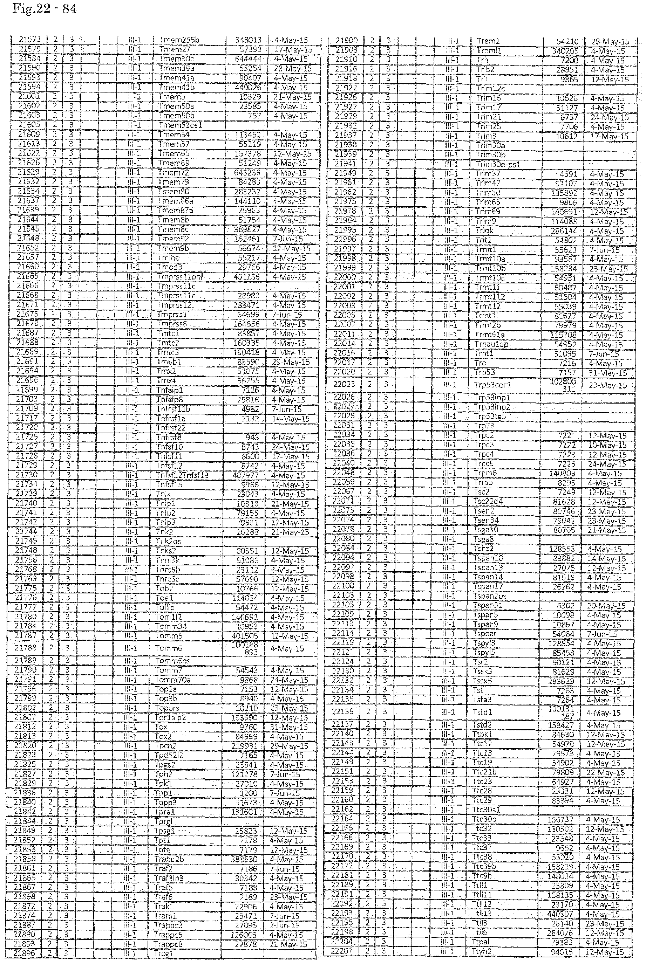

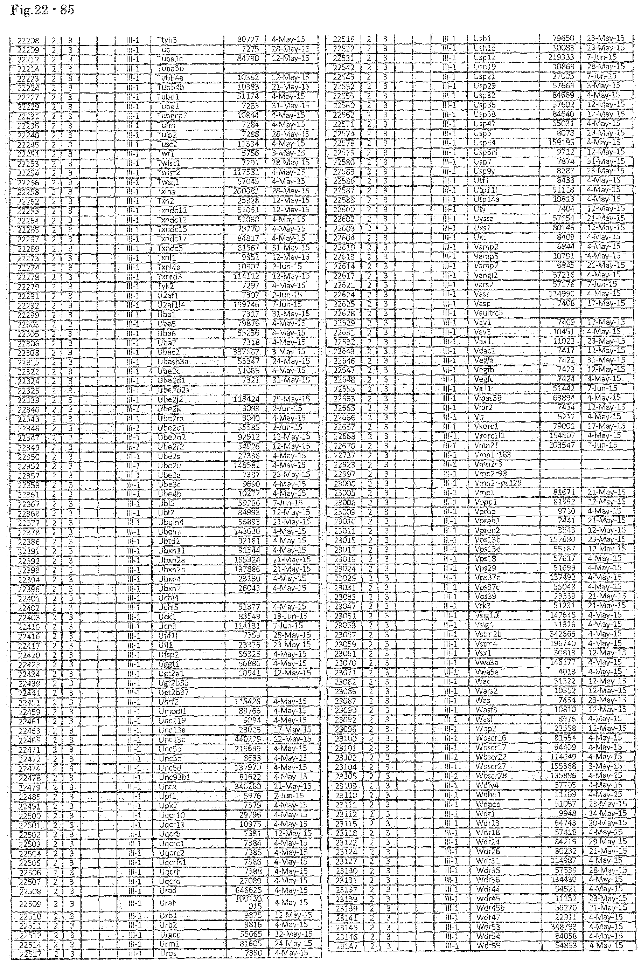

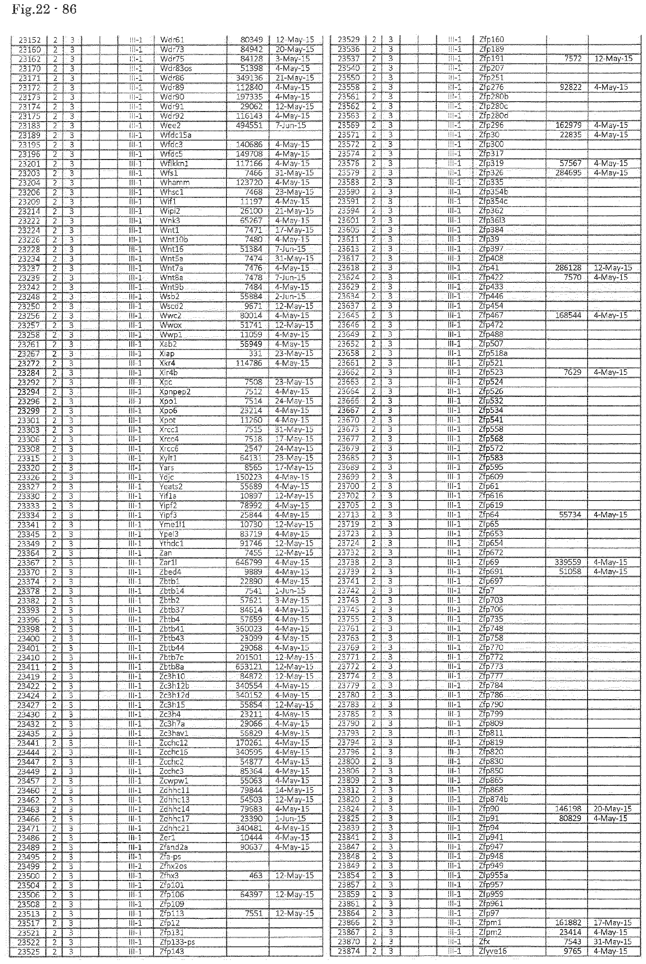

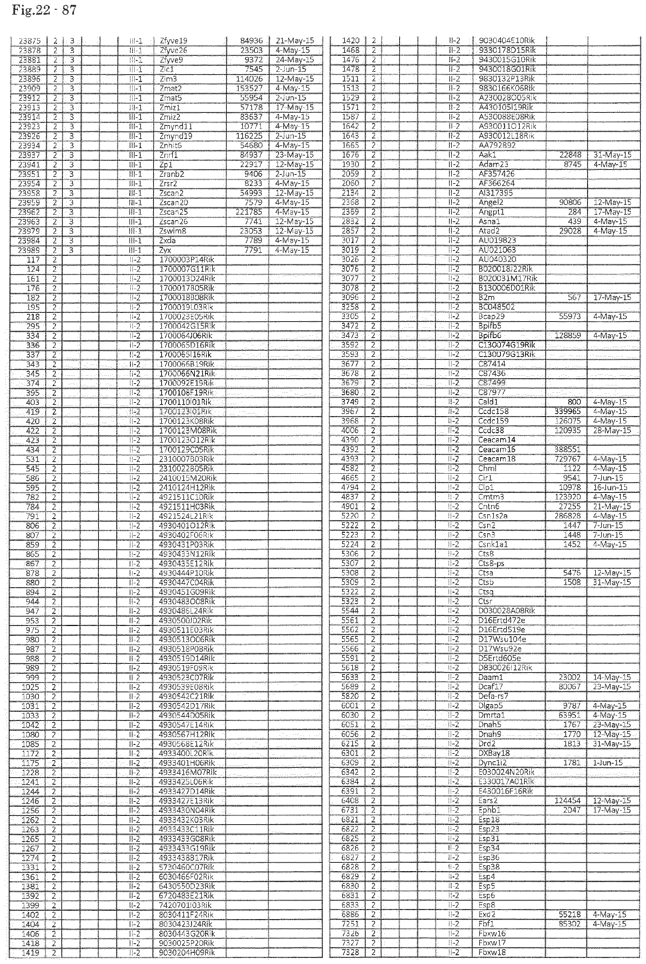

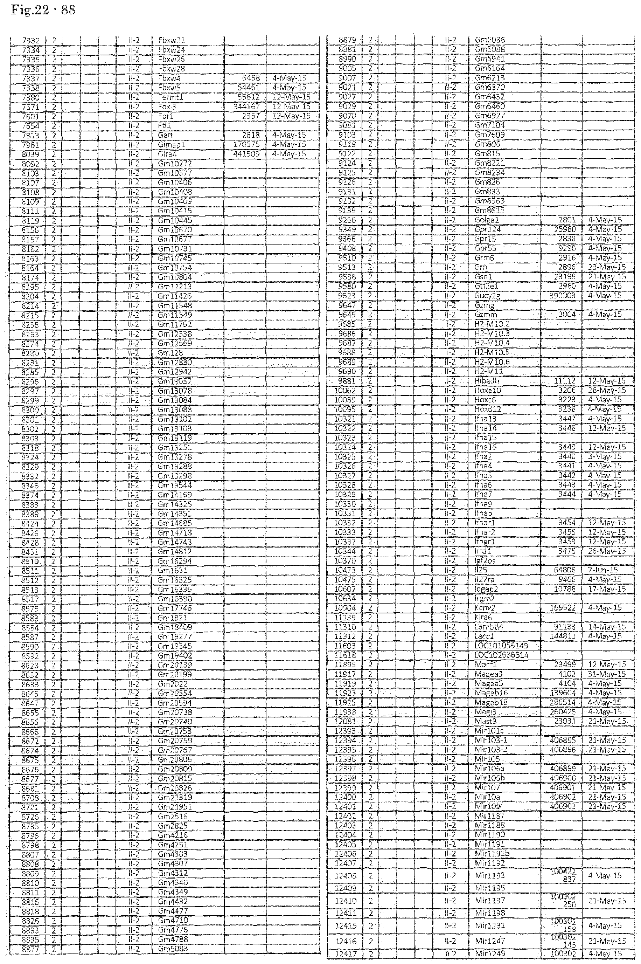

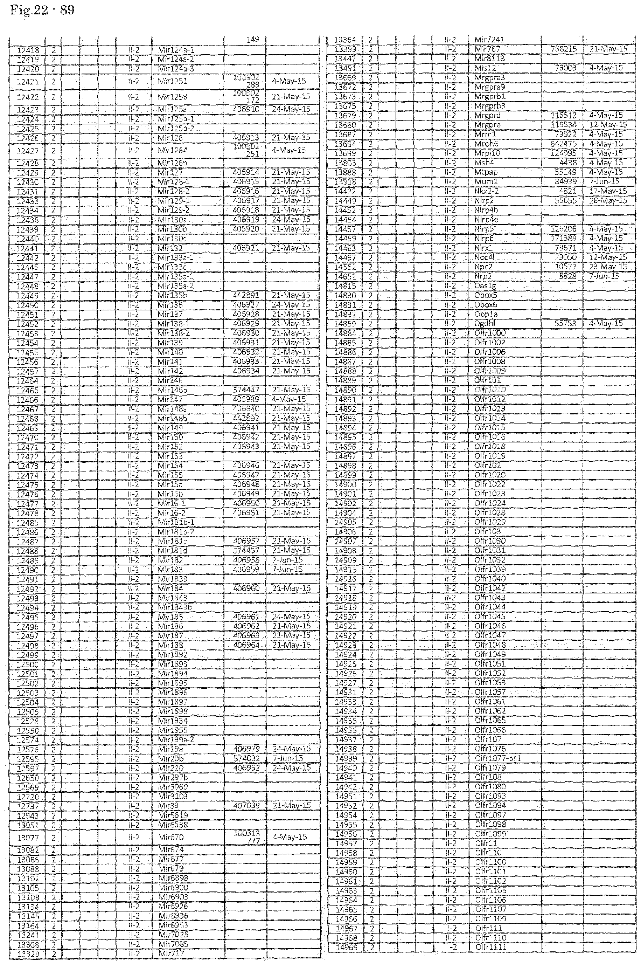

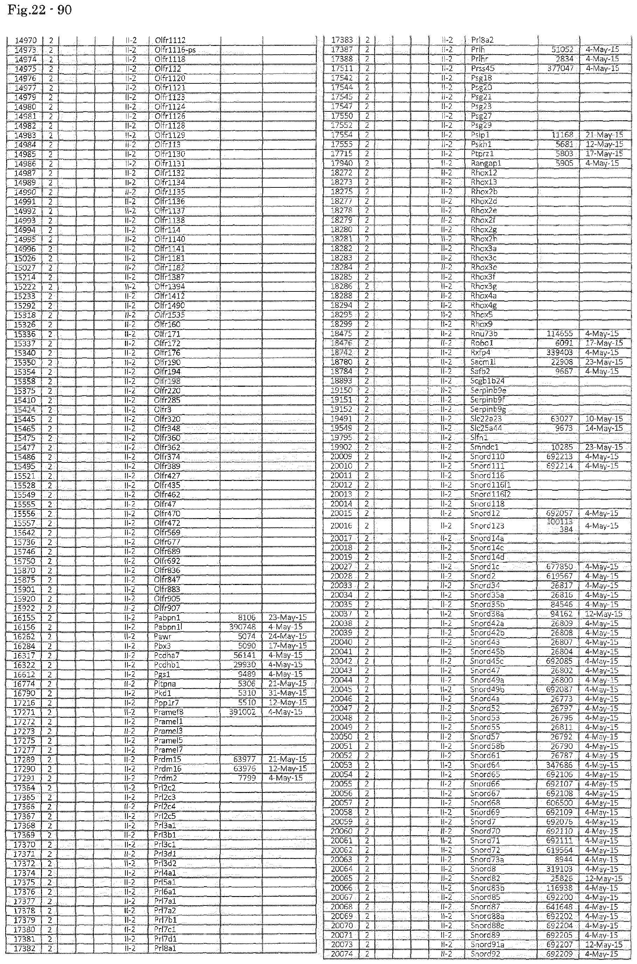

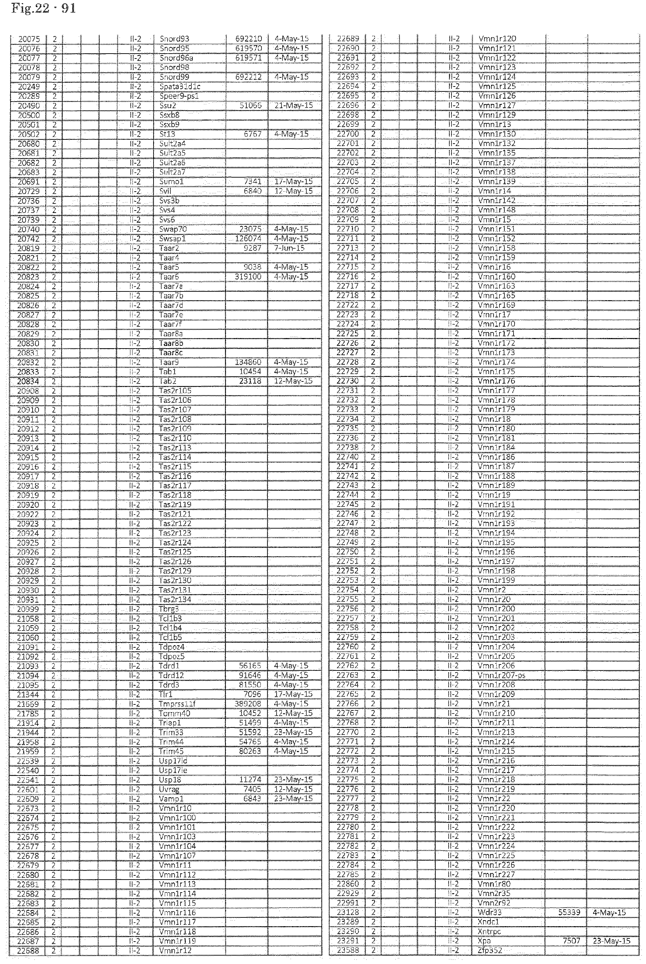

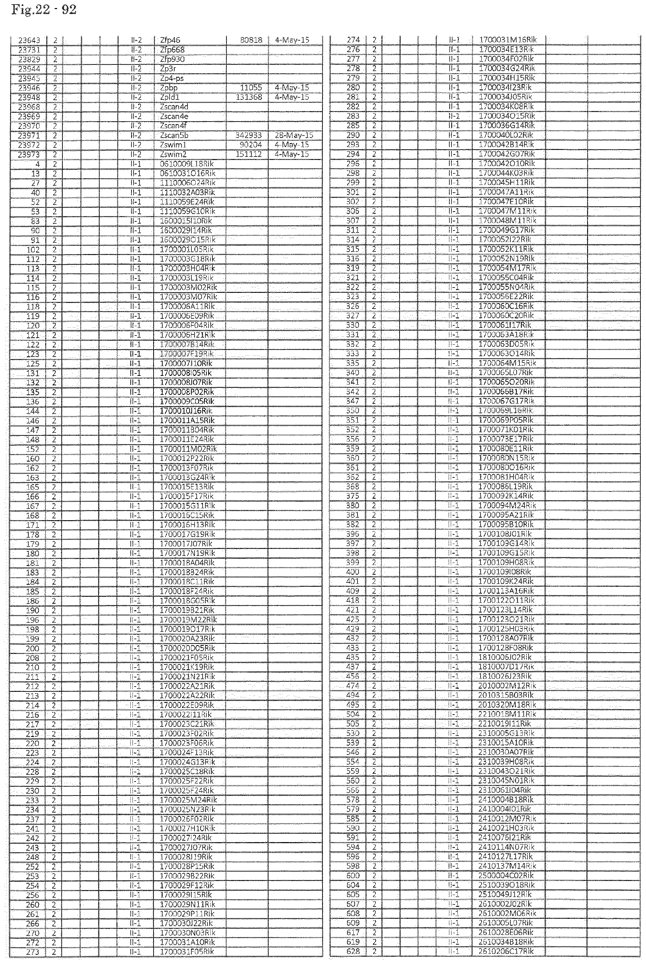

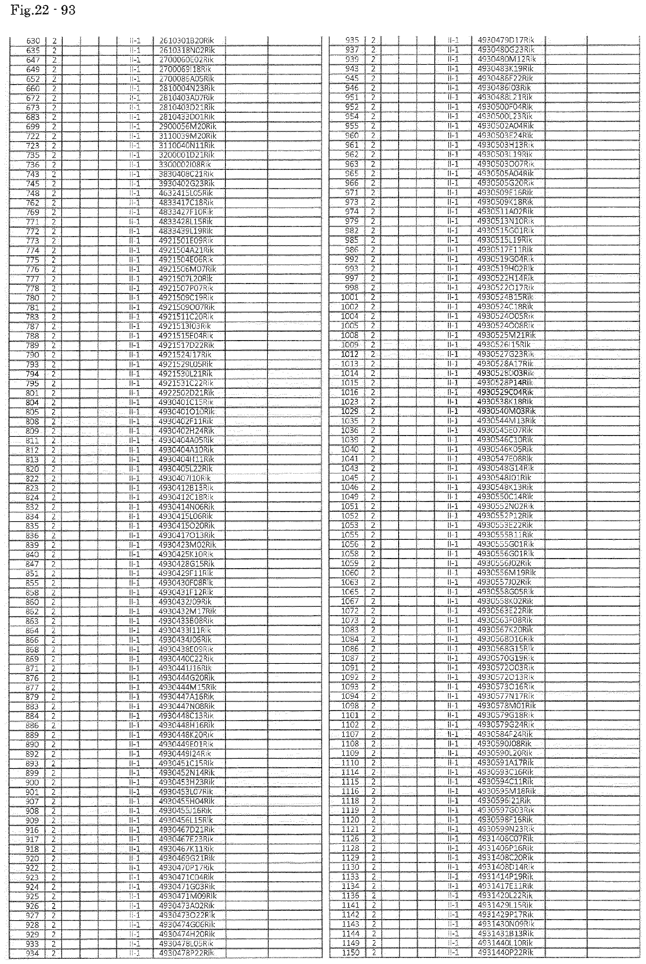

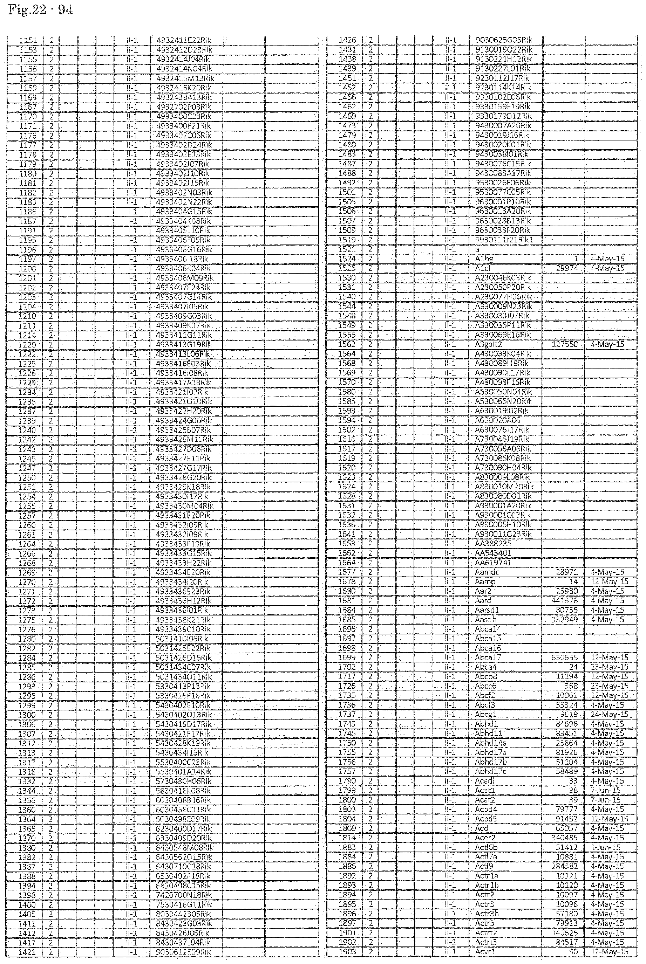

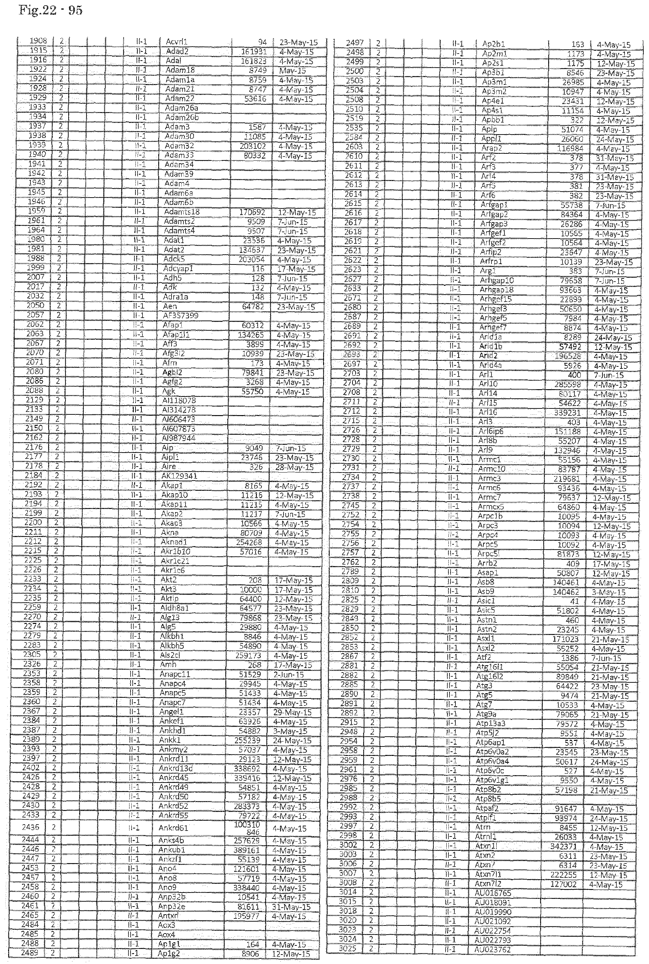

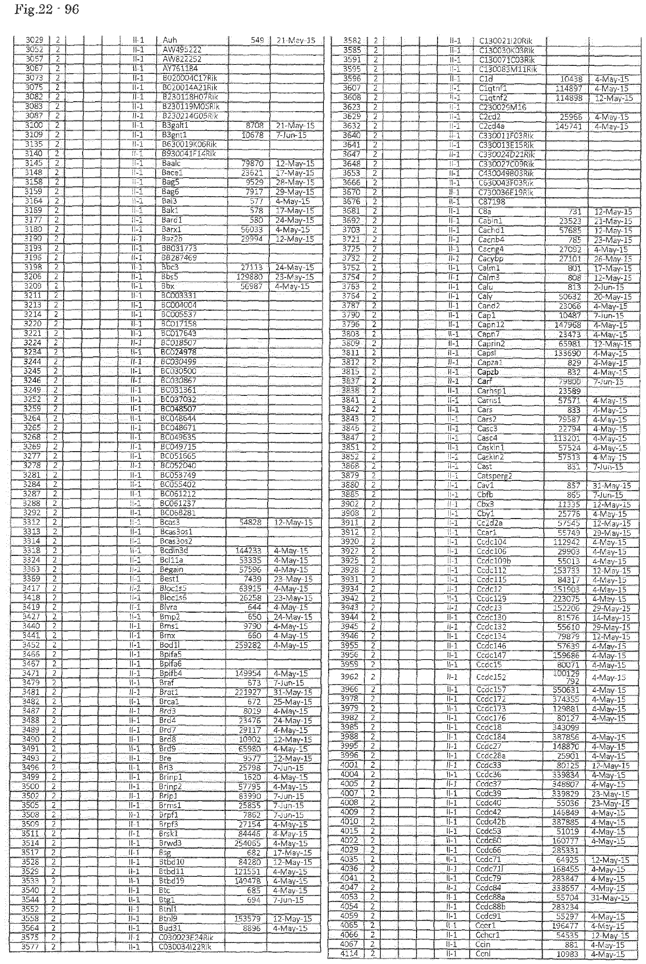

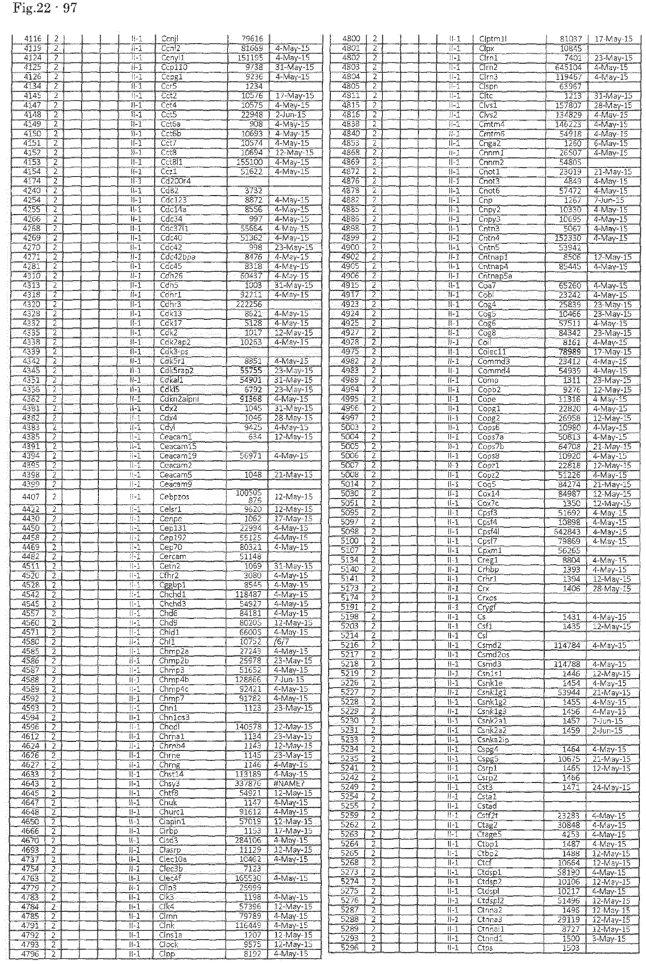

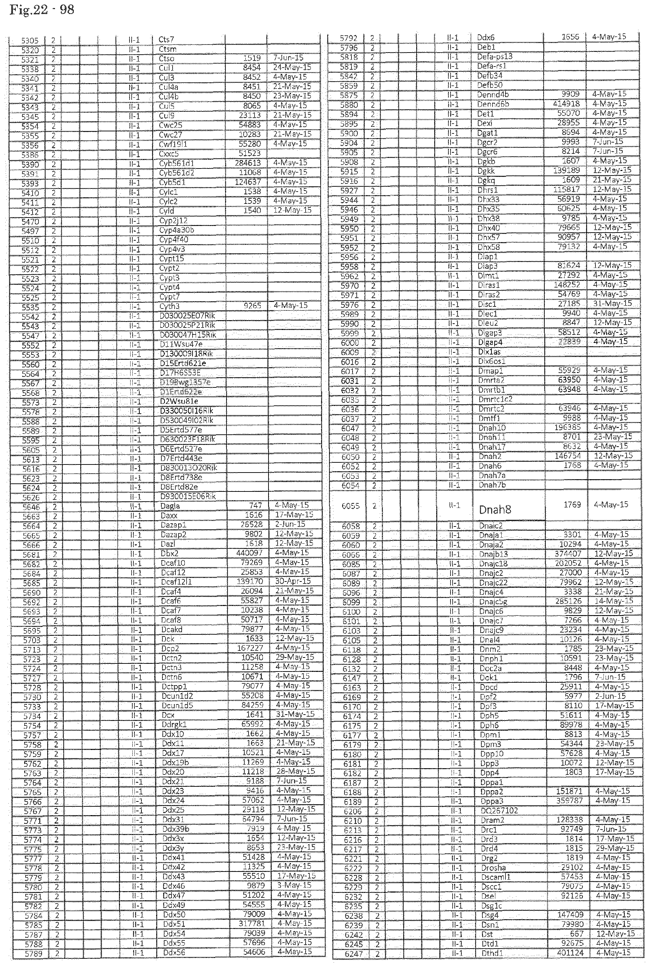

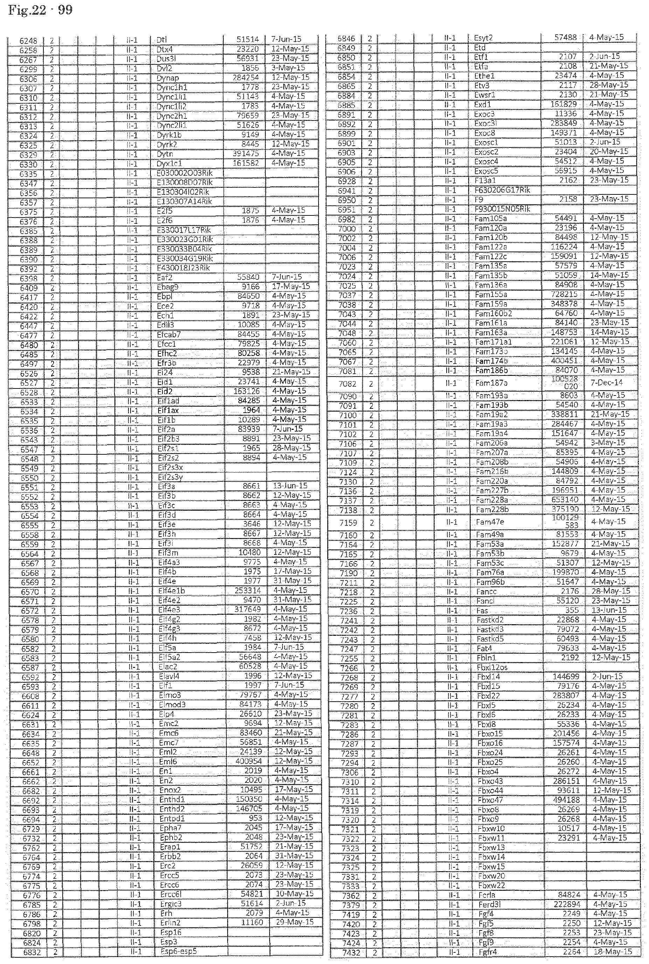

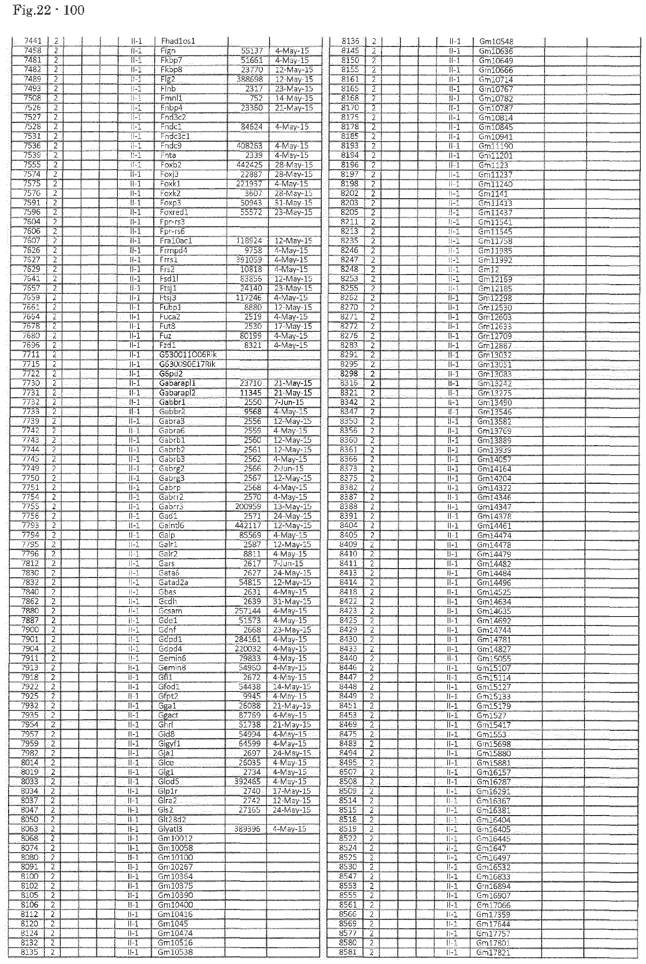

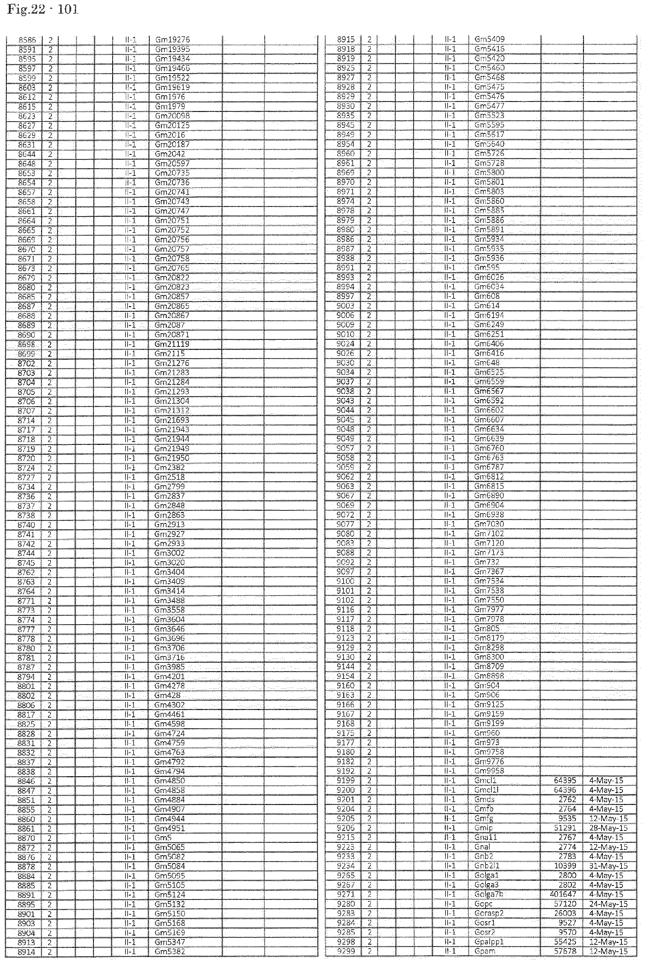

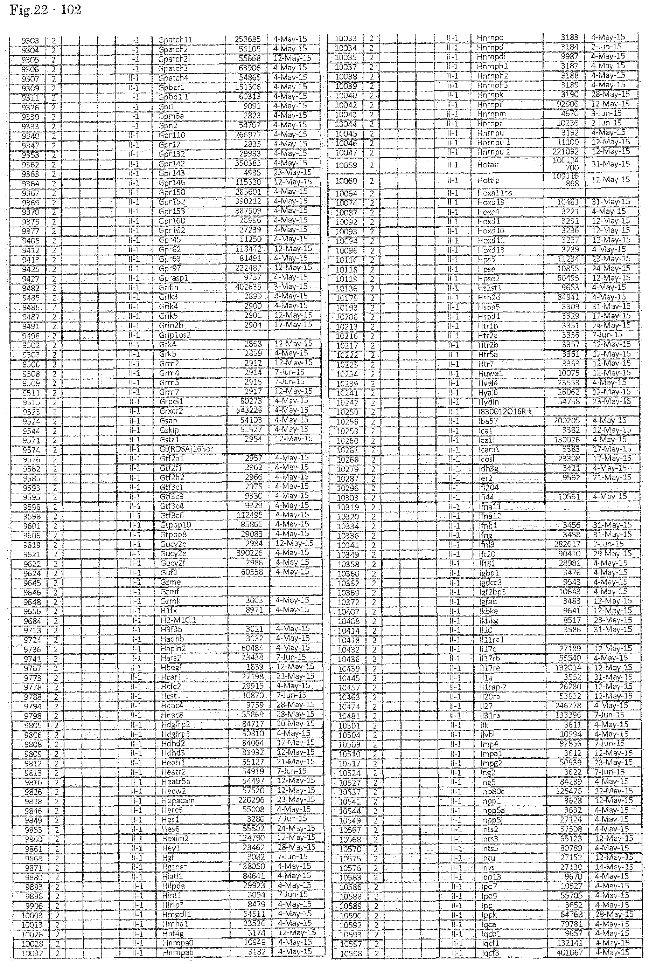

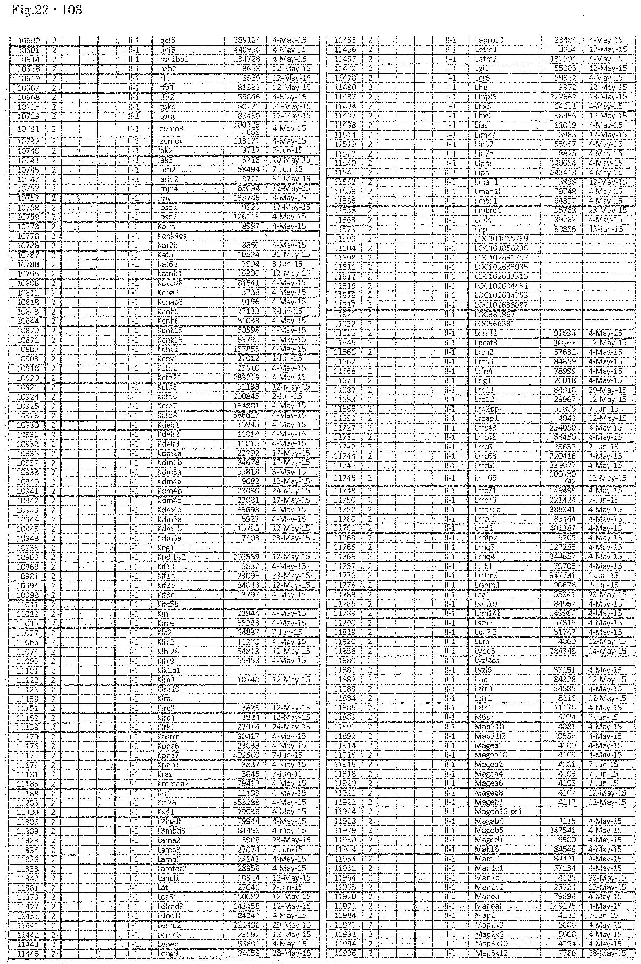

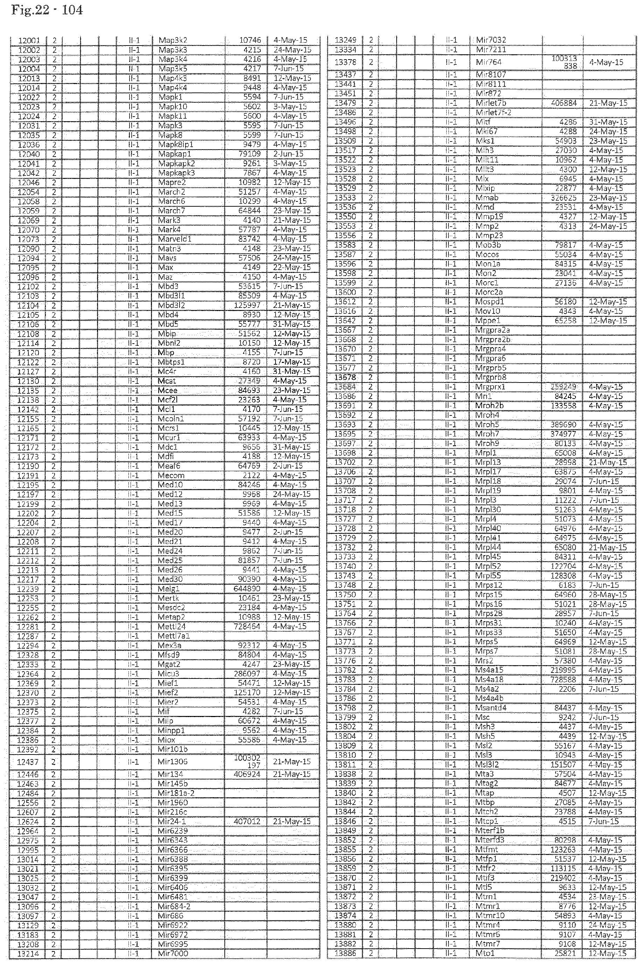

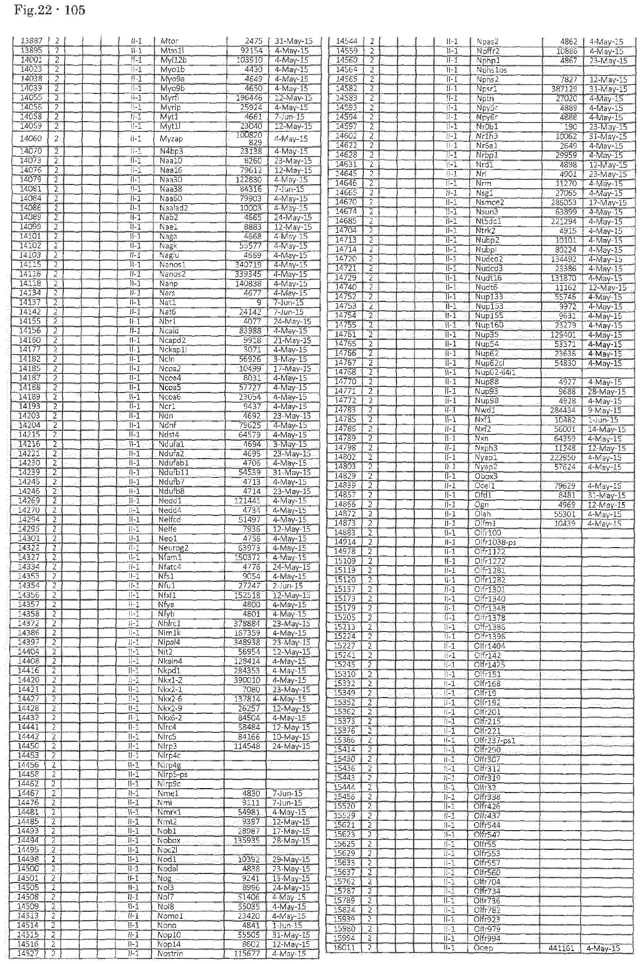

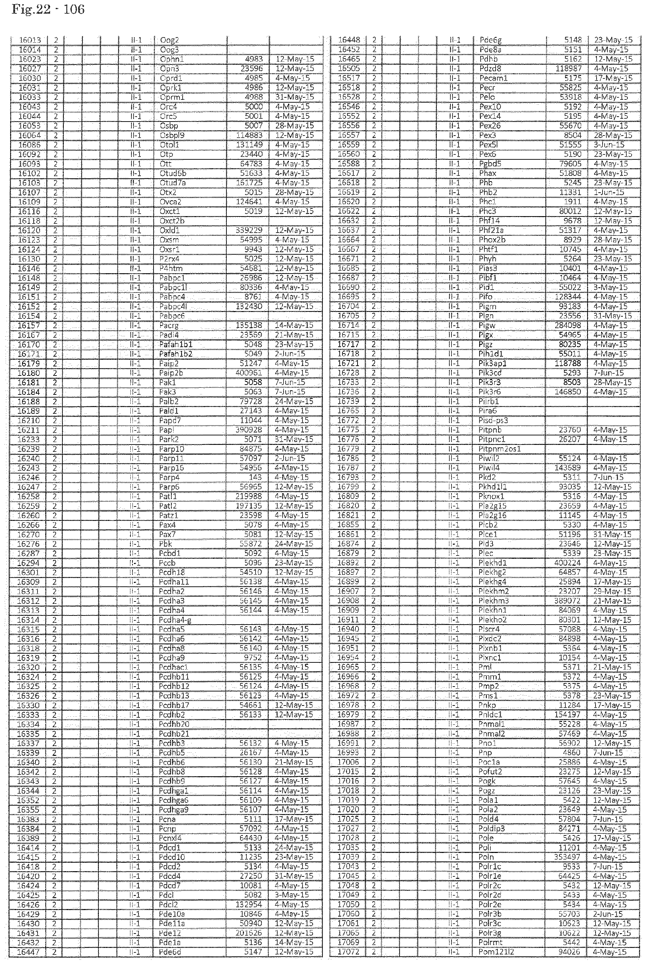

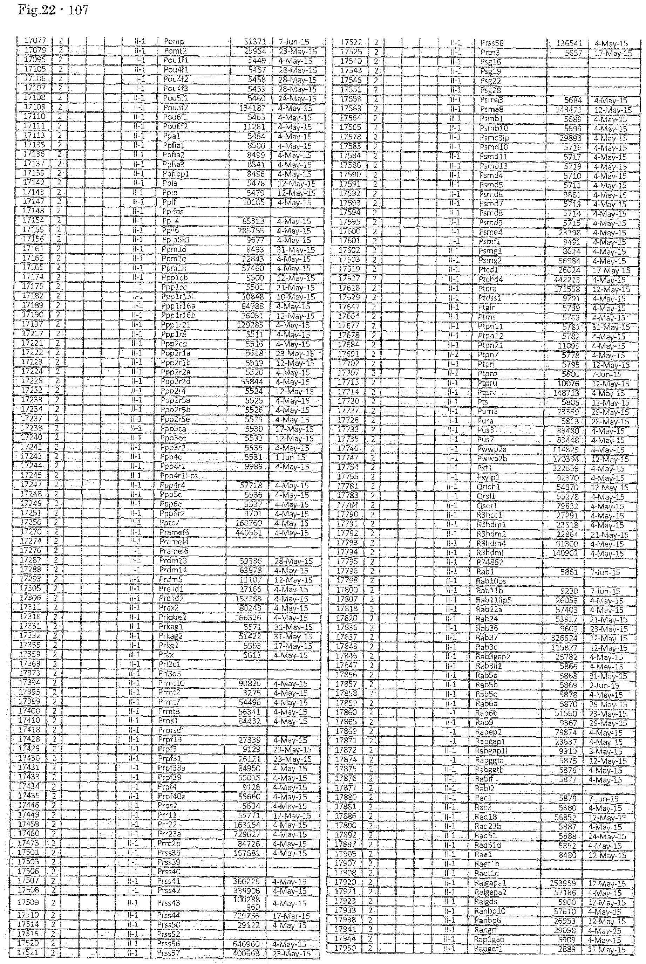

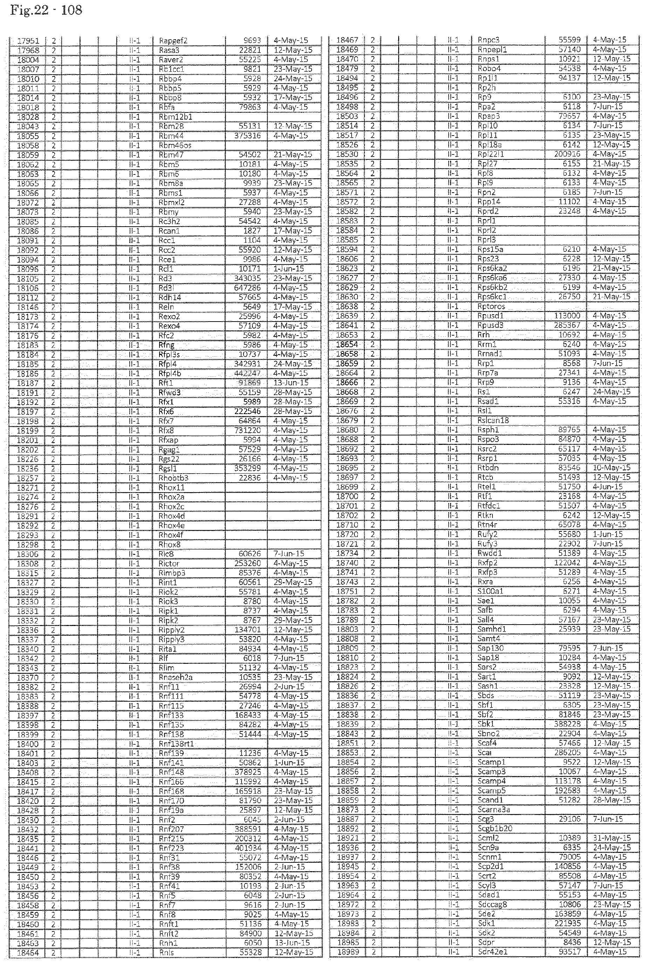

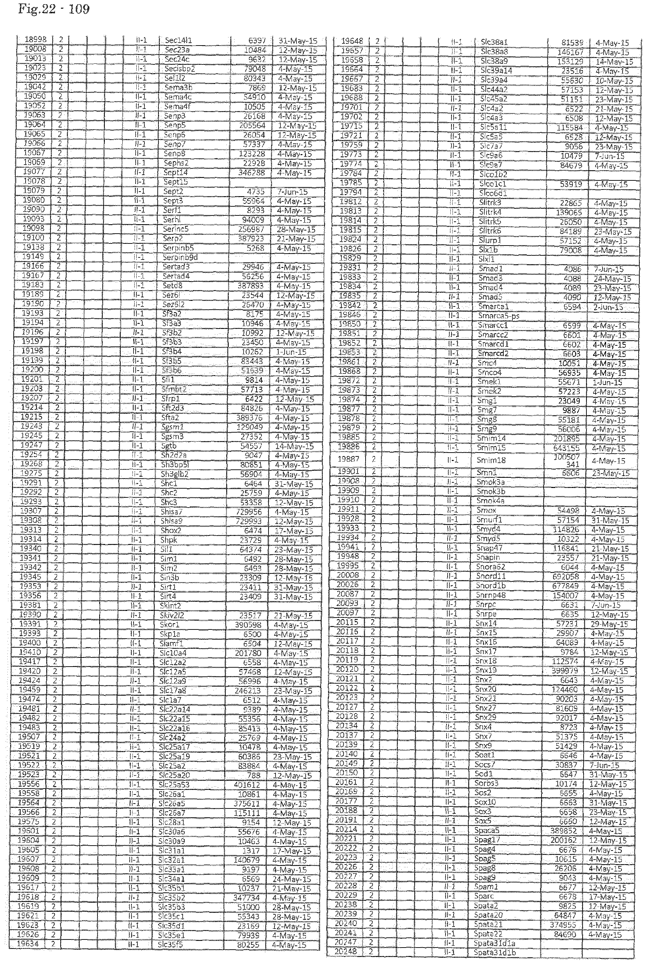

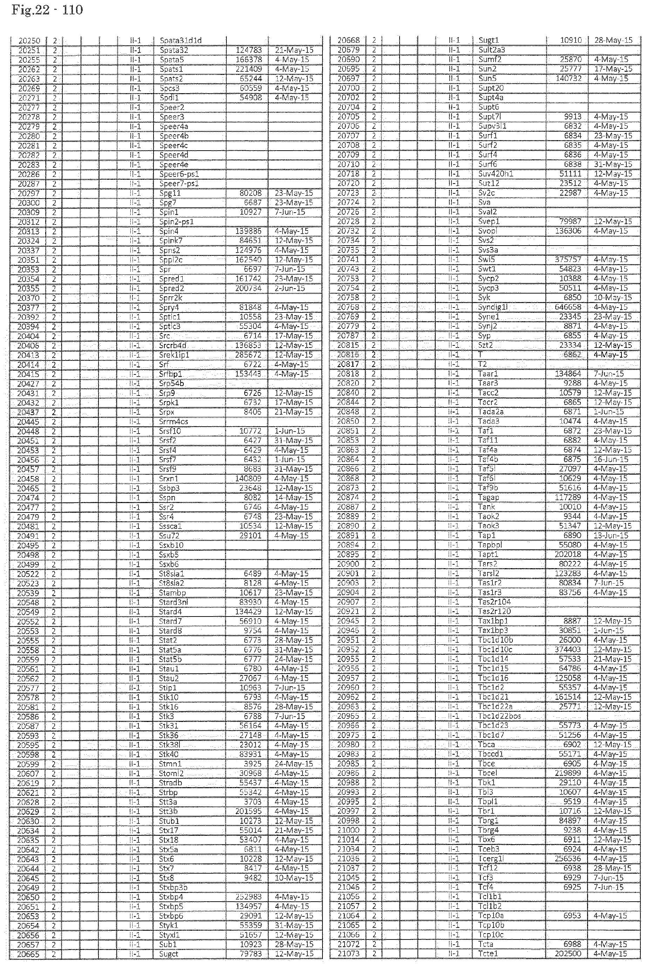

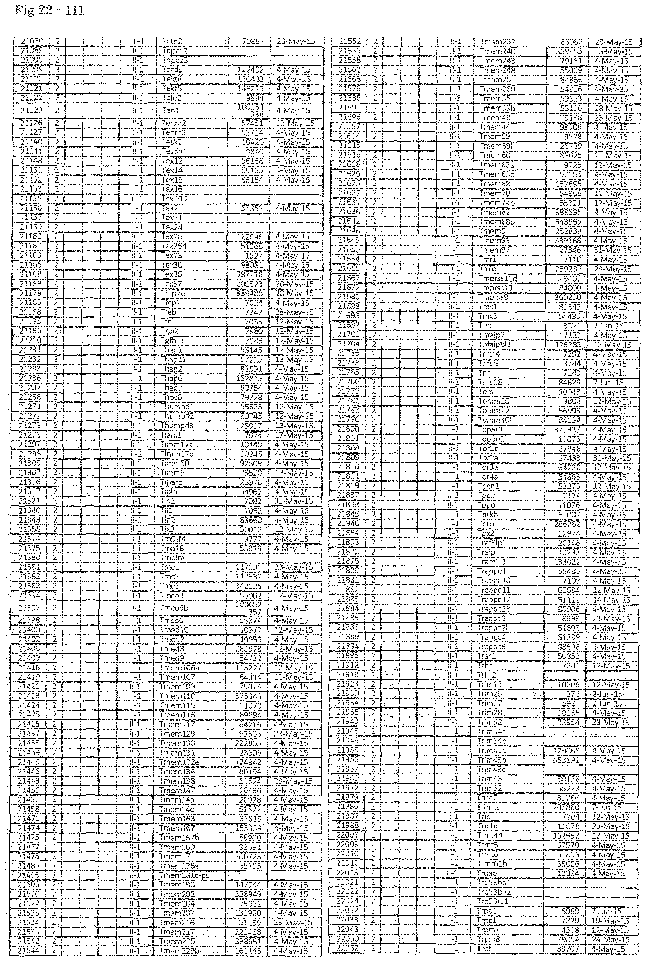

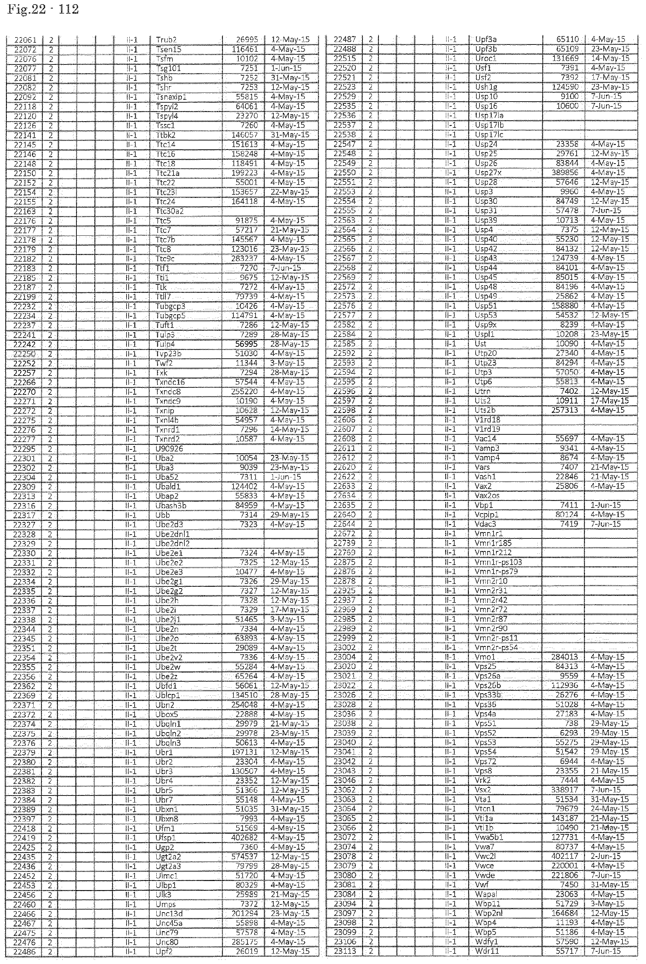

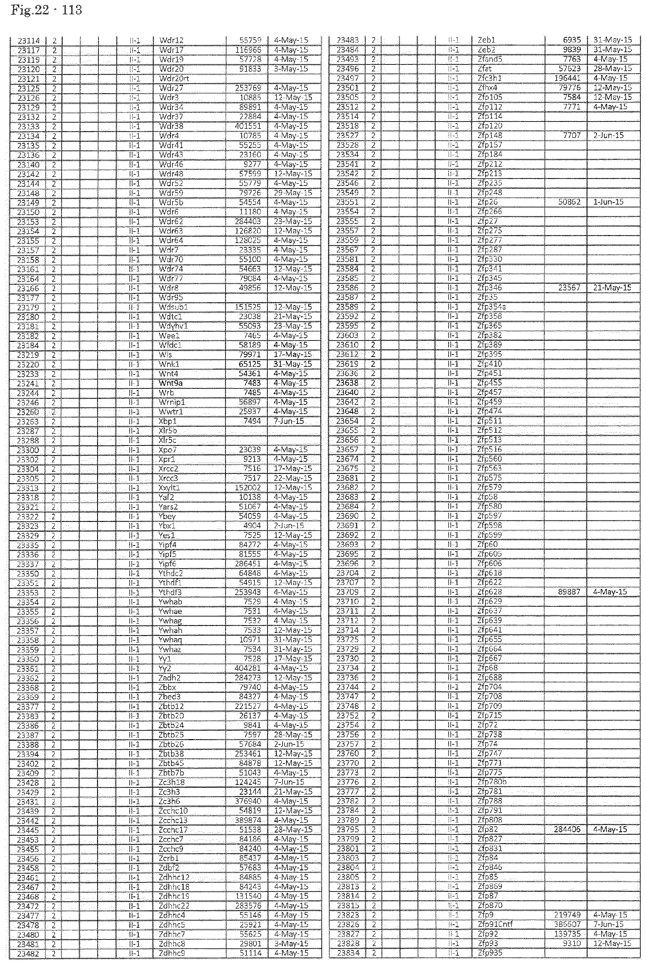

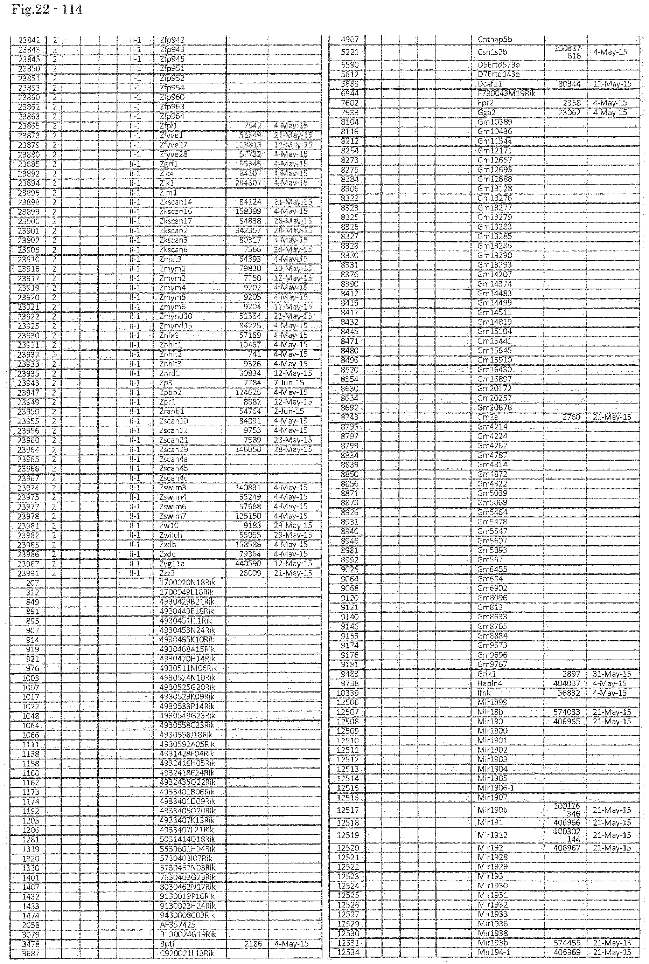

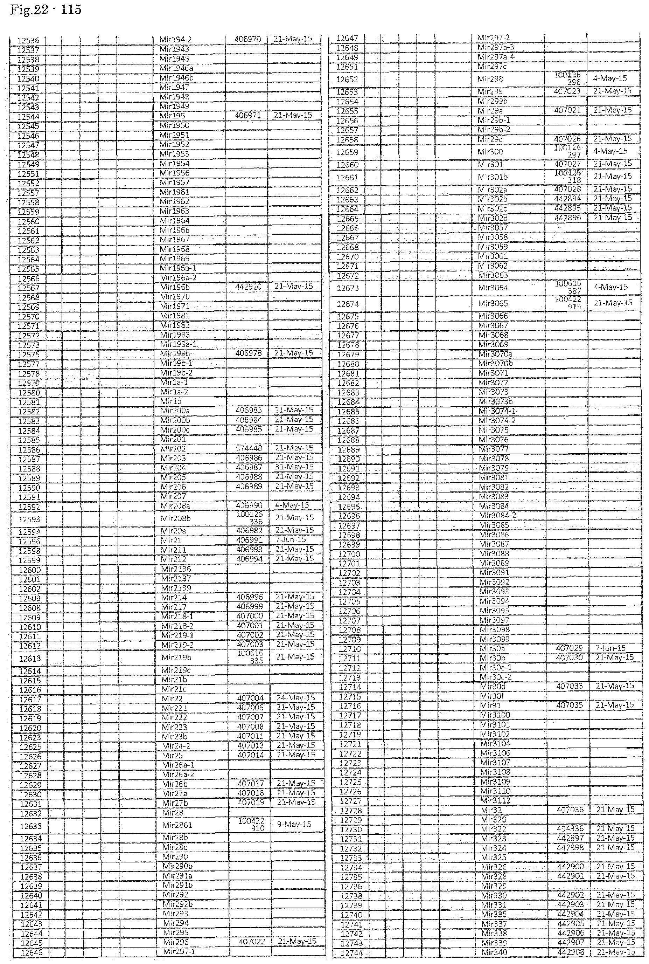

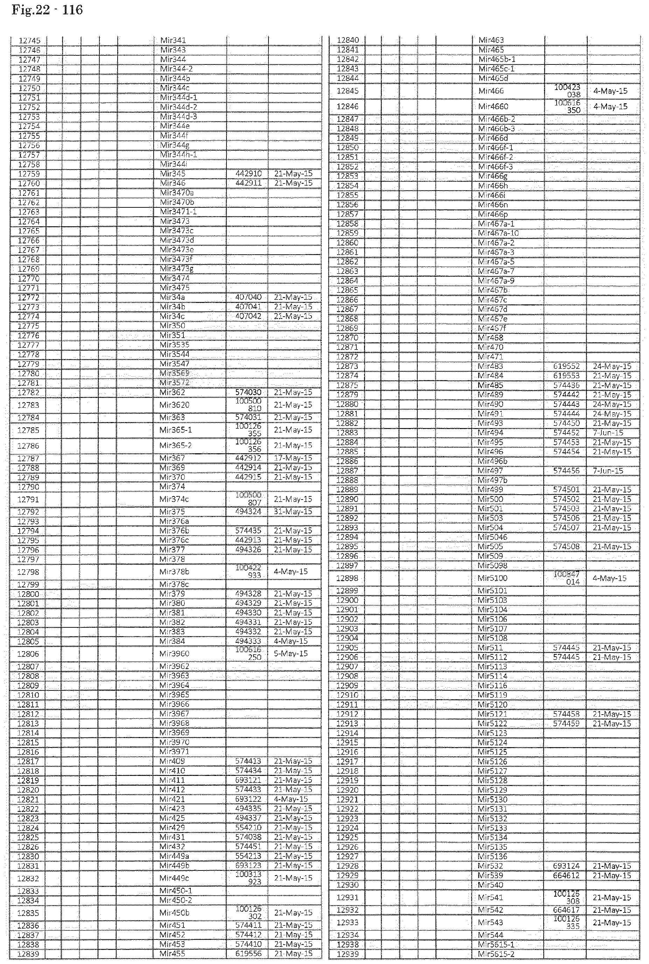

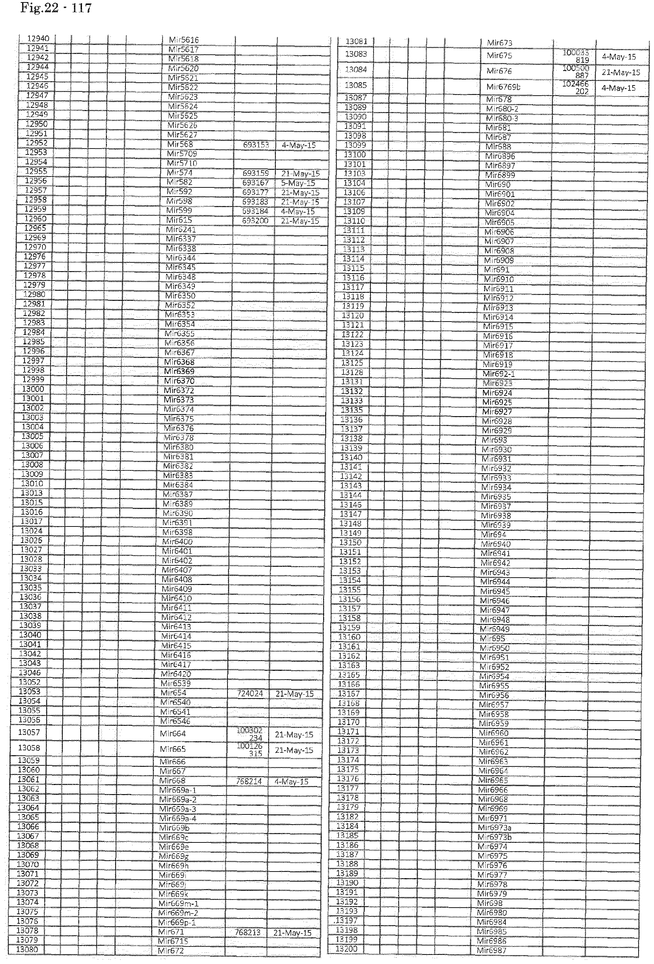

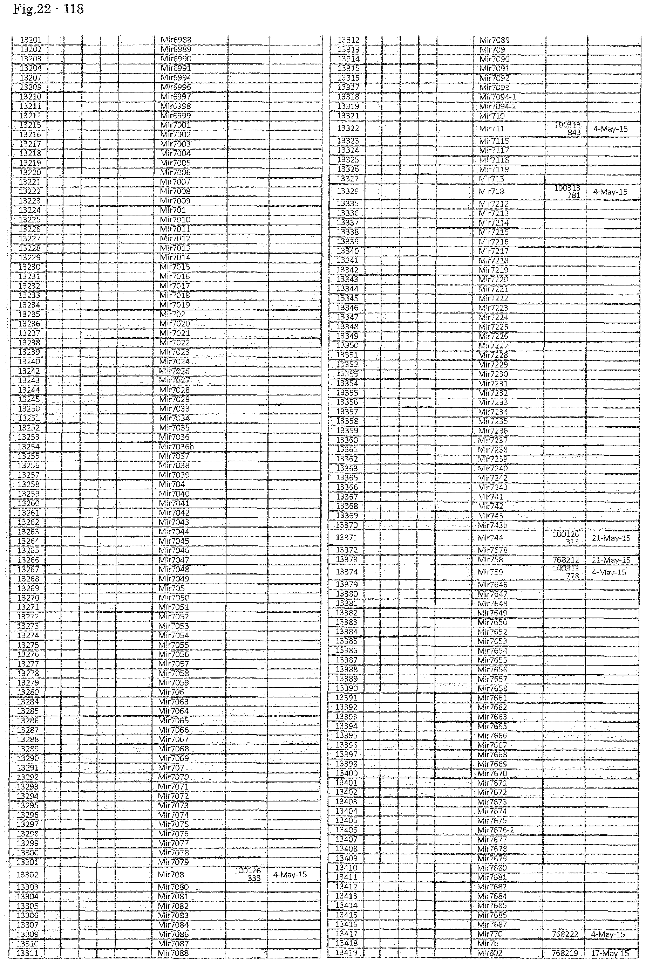

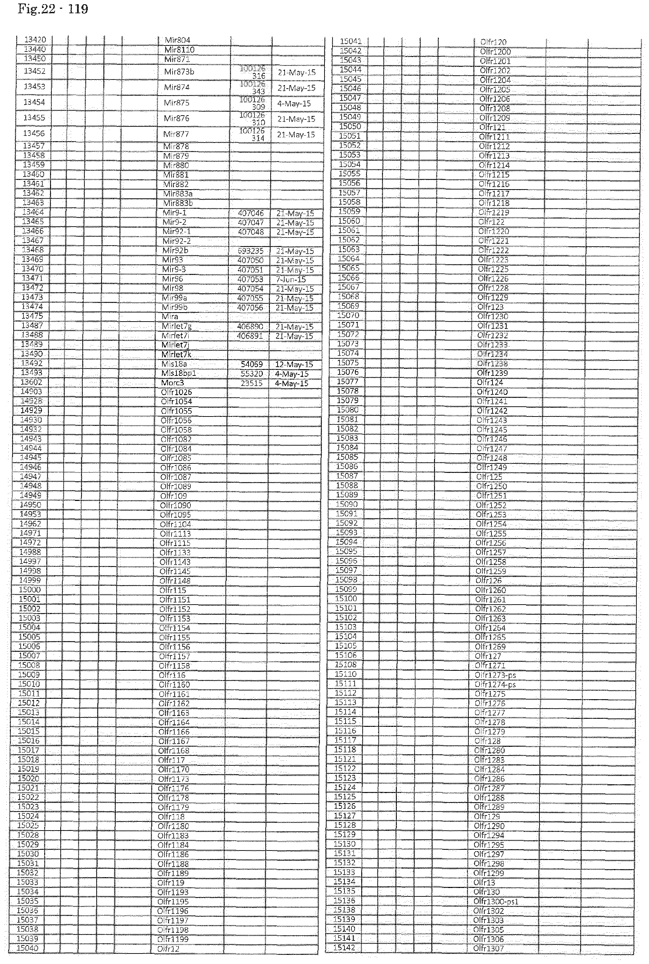

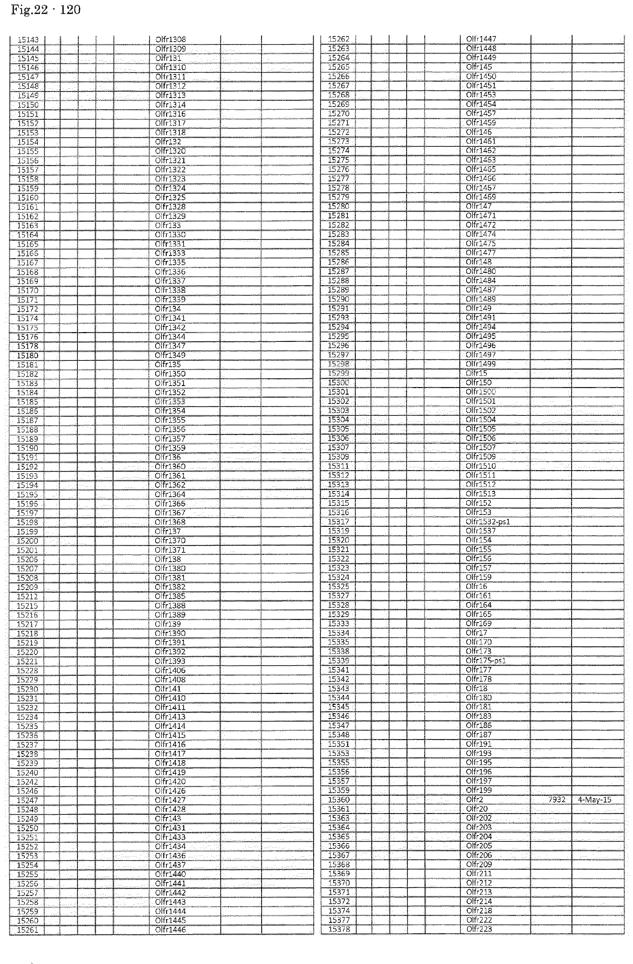

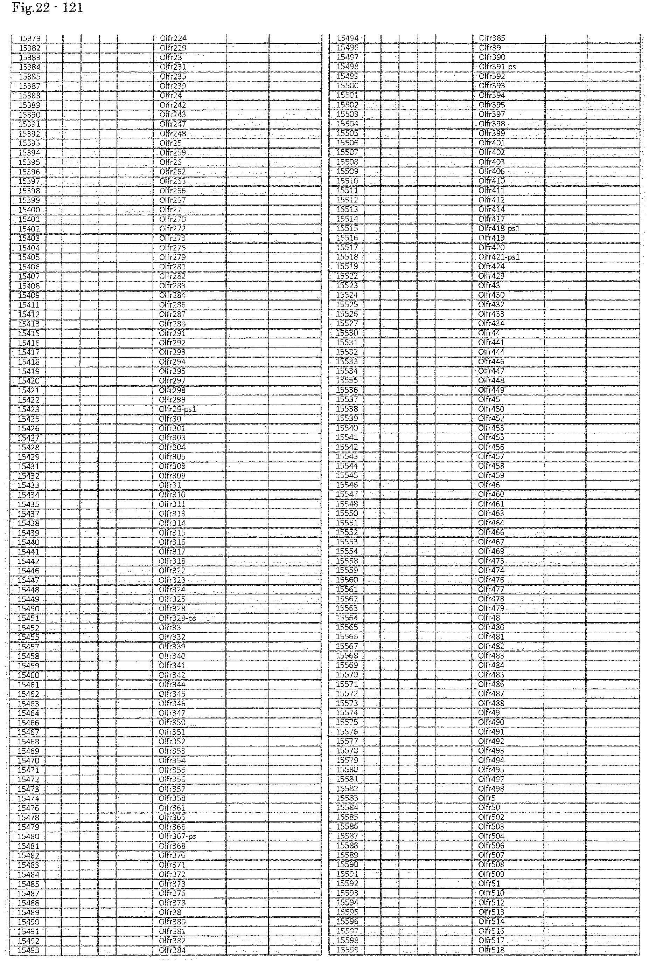

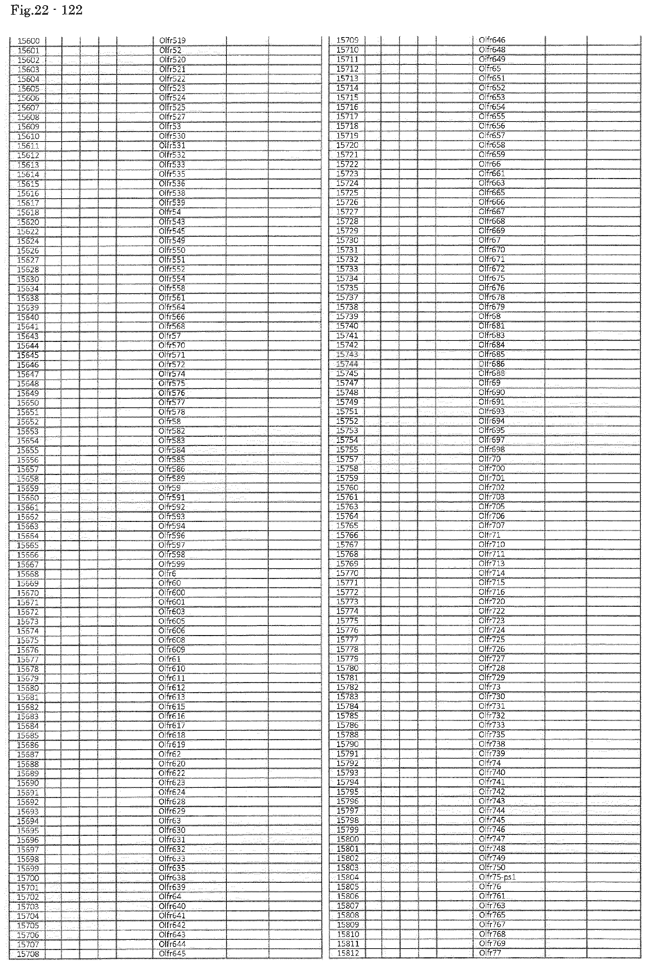

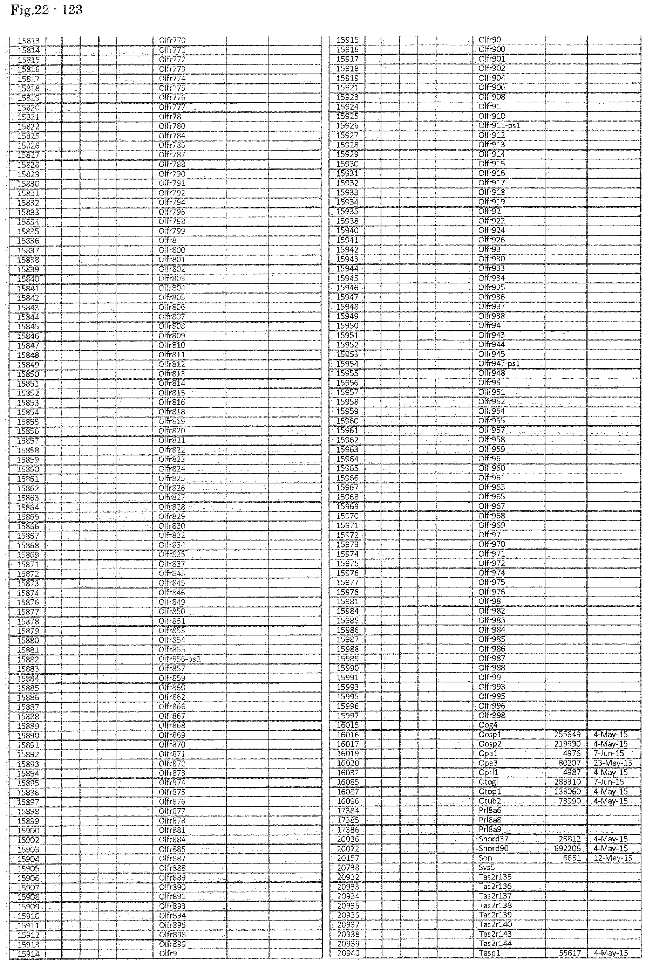

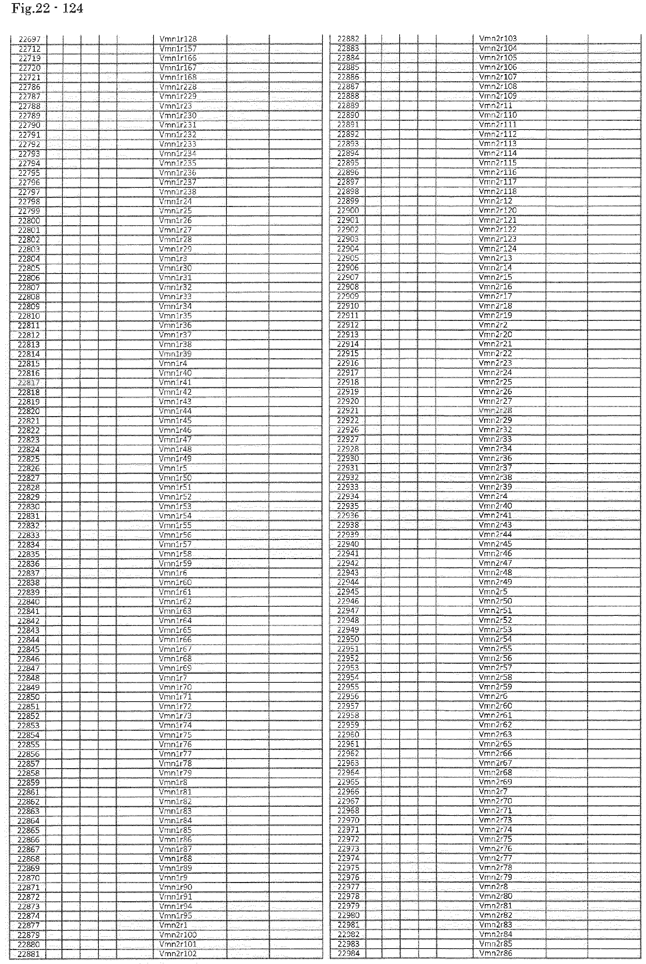

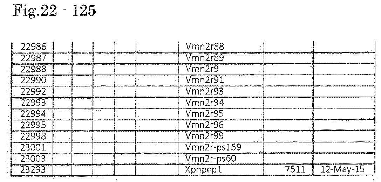

Fig. 22: Genes examined for their expression levels were classified as follows. Genes in which CKD/Sham is more than 1 or less than 1 were classified as group 2, genes in which CKD/Sham is more than 1.5 or less than 0.67 were classified as group 3, genes in which CKD/Sham is more than 2 or less than 0.5 were classified as group 4, and genes in which CKD/Sham is more than 5 or less than 0.2 were classified as group 5. In Fig. 22, "Line No." indicates a line number in the list, "Groups" indicates a group number of each of the groups classified based on the CKD/Sham values, "Gene Name" indicates a gene name registered with NCBI, "Human Gene ID" indicates a human gene number registered with NCBI that corresponds to the gene name, and "Updated" indicates the date of update to the Human Gene ID in NCBI. In "Sub-Group," "V-1" indicates genes, among the genes of group 5, in which CKD/Sham is more than 5; and "V-2" indicates genes, among the genes of group 5, in which CKD/Sham is less than 0.2. "IV-1" indicates genes, among the genes of group 4, in which CKD/Sham is more than 2 and that are not included in group 5; and "IV-2" indicates genes, among the genes of group 4, in which CKD/Sham is less than 0.5 and that are not included in group 5. "III-1" indicates genes, among the genes of group 3, in which CKD/Sham is more than 1.5 and that are not included in group 4 or group 5; and "III-2" indicates genes, among the genes of group 3, in which CKD/Sham is less than 0.67 and that are not included in group 4 or group 5. "II-1" indicates genes, among the genes of group 2, in which CKD/Sham is more than 1 and that are not included in any of groups 3 to 5; and "11-2" indicates genes, among the genes of group 2, in which CKD/Sham is less than 1 and that are not included in any of groups 3 to 5. Genes with no group number are a group in which CKD/Sham is 1.

Fig. 23 shows the expression levels of genes in which Sham>1 and CKD/Sham>5, genes in which Sham<1 and CKD/Sham>10, and genes in which Sham>10 and CKD/Sham<0.3 at the early and middle stages.

Fig. 24 shows mutation sites by CRISPR/Cas9 and sequences of ssODNs for obtaining Oscar gene mutant mice. Fig. 24A shows a mutation site of Oscar-gRNA1 and an ssODN sequence. Fig. 24B shows a mutation site of Oscar-gRNA2 and an ssODN sequence. Fig. 24C indicates phenotypes of Oscar in obtained mice.

Fig. 25 shows volcano plots in the skull 1 week (E), 4 weeks (M), and 8 weeks (L) after the start of a diet with high phosphorus content in UNx/HPi model mice (a diet with low phosphorus content in a Sham group).

Fig. 26A shows DESeq analysis results of expression of Oscar gene in each tissue. Fig. 26B shows results of expression of Oscar mRNA in the UNx/HPi model mice and sham mice at E, M, and L examined by qRT-PCR. Fig. 26C shows results of the expression of Oscar gene in the kidneys confirmed by qRT-PCR. In the graphs, *** indicates $p<0.001$, and n.s. indicates no significant difference.

Fig. 27 shows results of expression of FGF23 gene in the skull confirmed by qRT-PCR. In the graph, ** indicates $p < 0.01$, and *** indicates $p < 0.001$.

Fig. 28A shows the expression of Oscar gene in the skull 1 day, 3 days, 1 week, and 4 weeks after switching from a normal diet to a diet with low phosphorus content or a diet with high phosphorus content. Fig. 28B shows the expression of FGF23 gene in the skull 1 day, 3 days, 1 week, and 4 weeks after switching from a normal diet to a diet with low phosphorus content or a diet with high phosphorus content. In the graphs, * indicates $p < 0.05$, ** indicates $p < 0.01$, *** indicates $p < 0.001$, and n.s. indicates no significant difference.

Fig. 29 shows the expression of FGF23 in the bones of normal mice and Oscar gene mutant mice fed a diet with high phosphorus content. ** indicates $p < 0.01$.

Fig. 30 shows qRT-PCR results of FGF23 in the skull. HP4W indicates mice 4 weeks after the start of a diet with high phosphorus content, and LP4W indicates mice 4 weeks after the start of a diet with low phosphorus content (a control group). sOscar indicates mice to which a soluble human Oscar-Fc fusion protein was administered, and NS indicates mice to which physiological saline was administered in place of the soluble human Oscar-Fc fusion protein.

Fig. 31 shows qRT-PCR results of PRP genes in the parotid glands. HP4W indicates mice 4 weeks after the start of a diet with high phosphorus content, and LP4W indicates mice 4 weeks after the start of a diet with low phosphorus content (a control group). sOscar indicates mice to which a soluble human Oscar-Fc fusion protein was administered, and NS indicates mice to which physiological saline was administered in place of the soluble human Oscar-Fc fusion protein.

Fig. 32 shows ELISA measurement results of FGF23 in plasma. HP4W indicates mice 4 weeks after the start of a diet with high phosphorus content, and LP4W indicates mice 4 weeks after the start of a diet with low phosphorus content (a control group). sOscar indicates mice to which a soluble human Oscar-Fc fusion protein was administered, and NS indicates mice to which physiological saline was administered in place of the soluble human Oscar-Fc fusion protein. WT (12W) indicates 12-week-old male wild-type mice fed a normal diet.

Fig. 33 shows the concentration of creatinine in plasma. HP4W indicates mice 4 weeks after the start of a diet with high phosphorus content, and LP4W indicates mice 4 weeks after the start of a diet with low phosphorus content (a control group). WT (12W) indicates 12-week-old male wild-type mice.

Fig. 34 shows qRT-PCR results of Fgg gene in the kidneys. HP4W indicates mice 4 weeks after the start of a diet with high phosphorus content, and LP4W indicates mice 4 weeks after the start of a diet with low phosphorus content (a control group). sOscar indicates mice to which a soluble human Oscar-Fc fusion protein was administered, and NS indicates mice to which physiological saline was administered in place of the soluble human Oscar-Fc fusion protein.

Description of Embodiments

## I. Screening

### 1. Explanation of terms

First, terms used in the present specification, claims, and abstract regarding inventions relating to a method and device for screening a candidate substance for an active ingredient to prevent or treat at least one disease selected from the group consisting of declined kidney function, chronic kidney disease, and renal failure; and a program that causes a computer to carry out functions for screening a candidate substance for an active ingredient to prevent or treat at least one disease selected from the group consisting of declined kidney function, chronic kidney disease, and renal failure, are explained. The same terms as some of the terms used in this section "I. Screening" are used in the section "II. iOrgans" described later; however, terms relating to the inventions described in "I. Screening" used in the specification, claims, and abstract are in accordance with the definitions in this section, unless otherwise stated. The definitions of the terms used in this section "I. Screening" and the definitions of the terms used in the section "II. iOrgans" described later are independent of each other, unless individual sections are referred to.

"Declined kidney function" as used herein refers to, in the case of humans, a condition in which, for example, at least one kidney disease marker shown in Tables 1-1 to 1-3 below falls outside a threshold range.

Table 1-1

| | Item | Threshold | Unit | Measurement method |
|---|---|---|---|---|
| Serum | Total protein | 6.7 to 7.8 | g/dl | Biuret method |
| | Albumin | 3.8 to 5.3 | mg/dl | BCG method |
| | Urea nitrogen | 8 to 20 | mg/dl | Urease-GLDH method |
| | Creatinine | Male: 0.6 to 1.0 | mg/dl | Enzymatic method |
| | | Female: 0.4 to 0.8 | | |
| | Uric acid | Male: 3 to 7.7 | mg/dl | Uricase-POD method |
| | | Female: 2 to 7.7 | | |
| | Ammonia | 12 to 66 | μg/dl | GLDH method |
| | Sodium | 136 to 145 | mEq/l | ISE |
| | Potassium | 3.4 to 4.5 | mEq/l | ISE |
| | Chlorine | 100 to 108 | mEq/l | ISE |
| | Total calcium | 8.6 to 10.1 | mg/dl | OCPC method |
| | Magnesium | 1.8 to 2.3 | mg/dl | Enzymatic method |
| | Inorganic phosphorus | Adult: 2.2 to 4.1 | mg/dl | Enzymatic method |
| | | Child: 4.0 to 7.0 | | |
| | Copper | 71 to 132 | μg/dl | Chelate colorimetric method |
| | Amylase | 40 to 126 | IU/l | JSCC standardization corresponding method |
| | FGF23 | Full-length assay threshold: 10 to 50 C-terminal assay threshold: 150 | pg/ml RU/ml | ELISA |

(continued)

| | Item | Threshold | Unit | Measurement method |
|---|---|---|---|---|
| Whole blood | Red blood cell count | Male: 414 to 563 | $\times 10^4/\mu l$ | Electrical resistance-type automatic blood cell counter |
| | | Female: 373 to 495 | | |
| | Hemoglobin | Male: 12.9 to 17.4 | g/dl | Oxyhemoglobin method |
| | | Female: 10.7 to 15.3 | | |
| | Pyruvic acid | 0.30 to 0.94 | mg/dl | Enzymatic method |

Table 1-2

| | Item | Threshold | Unit | Measurement method |
|---|---|---|---|---|
| Arterial blood gas analysis/ acid-base equilibrium | $O_2$ saturation $S_aO_2$ | 94 to 99 | % | |
| | $O_2$ partial pressure $P_aO_2$ | 80 to 100 | Torr | |
| | $CO_2$ partial pressure $P_aCO_2$ | 35 to 45 | Torr | |
| | pH | 7.35 to 7.45 | | |
| | $HCO_3^-$ | 22 to 26 | mEq/l | |
| | Base excess (BE) | -2.2 to +2.2 | mEq/l | |
| | Buffer base (BB) | 46 to 52 | mEq/l | |
| | Standard bicarbonate (SB) | 21 to 25 | mEq/l | |
| Urine | Urinary output | 600 to 1,600 | ml/day | |
| | Specific gravity (spot urine) | 1.006 to 1.030 | | |
| | pH | 4.5 to 7.5 | | |
| | Urinary protein | 20 to 120 | mg/day | Pyrogallol red•Mo coloring method |
| | Albumin | $5.7 \pm 2.6$ | mg/day | |
| | Glucose | 2 to 20 | mg/dl | |
| Urinary sediment | Red blood cell count | < 5 | /400x field | |
| | Leukocyte count | <5 | | |
| | Epithelial cell count | Less than 1 (excluding squamous epithelium) | | |
| | Cast count | < 1 | | |

Table 1-3

| | Item | Threshold | Unit | Measurement method |
|---|---|---|---|---|
| | Creatinine clearance (Ccr) | 70 to 130 | ml/min | |

(continued)

| | Item | Threshold | Unit | Measurement method |
|---|---|---|---|---|
| Kidney function | 24-Hour creatinine clearance | Male: 62 to 108 | ml/min | |
| | | Female: 57 to 78 | | |
| | Glomerular filtration rate (GFR) | Male: 129±26 | ml/min | |
| | | Female: 97±13 | | |
| | Urea clearance | Maximum clearance: 62 to 77 | ml/ min | |
| | | Standard clearance: 45 to 55 | | |
| | Inulin clearance (GFR) | Male: 72 to 176 | $ml/min/1.73m^2$ | |
| | | Female: 81 to 137 | $ml/min/1.73m^2$ | |
| | Sodium thiosulfate clearance | Male: 90 to 138 | ml/min | |
| | | Female: 86 to 120 | | |
| | Renal plasma flow (RPF) | 350 to 650 | ml/min | $C_{PAII}$ |
| | Filtration fraction (FF) | 0.18 to 0.22 | | GRF/RPF |
| | Fractional excretion of sodium | 1 ≤ | % | |
| | Fractional excretion of lithium | 20 to 30 | % | |
| | Phenolsulfonphthalein (PSP) test | ≤ 100 | mOsm/kg | |
| | | 15 min value: ≥ 25 | % | |
| | | 120 min value: ≥ 55 | % | |
| | Concentration test | ≥ 1.025 | (Specific gravity) | Fishberg |
| | Dilution test | ≤ 1.006 | (Specific gravity) | Fishberg |
| | Free water clearance | At the time of water diuresis: 13 to 15 | ml/min | |
| | Free water reabsorption | At the time of concentration: 1.5 to 2.0 | ml/min | |
| | Maximal tubular excretory capacity | 81±11 | $mg/min/1.48m^2$ | $T_{mPAII}$ |
| | Maximal tubular reabsorption capacity | 340±18 | $mg/min/1.48m^2$ | $T_{mPAII}$ |
| | Rate of phosphate reabsorption | 80 to 96 | % | %TRP |
| | $\beta_2$-Microglobulin | Serum: 0.8 to 0.2 | mg/l | LPIA |
| | | Urine: 11 to 253 | μg/day | LPIA |
| | | (30 to 340) | (μg/l) | |
| | $\alpha_1$-Microglobulin | Serum: 10 to 30 | mg/l | EIA |
| | | Urine: 1.8±0.9 | mg/l | EIA |

"Chronic kidney disease" as used herein refers to, when the subject is a human, a condition in which kidney damage (for example, urine abnormalities such as proteinuria including microalbuminuria, abnormal urinary sediment, imaging abnormalities such as a single kidney and polycystic kidney disease, declined kidney function such as increased serum creatinine, electrolyte abnormalities such as hypokalemia due to tubular damage, abnormalities in histopathological examination such as renal biopsy), or declined kidney function, i.e., an estimated GFR (glomerular filtration rate) of less than 60 mL/min/1.73 $m^2$, persists for 3 months or more, according to the Clinical Practice Guidebook for Diagnosis and Treatment of Chronic Kidney Disease 2012 (edited by the Japanese Society of Nephrology).

Here, the estimated GFR (eGFR) can be calculated using the estimation formulas (eGFRcreat) from a serum creatinine value shown in Table 2 below. The estimation formulas (eGFRcys) based on serum cystatin C can be applied to those who have extremely low muscle mass, such as lower-extremity amputees.

Table 2

| | |
|---|---|
| Male | eGFRcreat (mL/min/1.73 $m^2$) = $194 \times Cr^{-1.094} \times age^{-0.287}$ |
| | eGFRcys (mL/min/1.73 $m^2$) = $(104 \times Cys\text{-}C^{-1019} \times 0.996^{a9e})-8$ |
| Female | eGFRcreat (mL/min/1.73 $m^2$) = $194 \times Cr^{-1.094} \times age^{-0.287} \times 0.739$ |
| | eGFRcys (mL/min/1.73 $m^2$) = $(104 \times Cys\text{-}C^{-1.019} \times 0.996^{age} \times 0.929)-8$ |
| *This evaluation of kidney function is performed for persons aged 18 or older. | |

For example, in the case in which protein is used as an index, when the results of a urine test 3 months or more prior and a recent urine test show that the subject has a persistent urinary protein level of 0.15 g/gCr or more, such a condition can be diagnosed as chronic kidney disease. When the subject has diabetes and the results of an albuminuria test 3 months or more prior and a recent albuminuria test show that the subject has a persistent urinary albumin level of 30 mg/gCr or more, such a condition can be diagnosed as chronic kidney disease.

For children, a threshold of serum creatinine (Cr) can be determined by using an enzymatic method for Japanese children, and used to evaluate children with kidney function abnormalities. For example, the eGFR in % for children aged 2 or older but 11 or younger can be represented by equation 1 below.

Equation 1

eGFR (%) = (0.3 × body height (m)/serum Cr value in subject) × 100

In the case of non-human mammals, such as cats and dogs, it can be predicted whether a non-human mammal has chronic kidney disease from, for example, average daily water intake or urine specific gravity.

The severity of chronic kidney disease can be determined based on, for example, Table 3 below in the case of humans (Table 3 is Table 2 in the Clinical Practice Guidebook for Diagnosis and Treatment of Chronic Kidney Disease, 2012).

Table 3

| Primary disease | Proteinuria category | | | A1 | A2 | A3 |
|---|---|---|---|---|---|---|
| Diabetes | Urinary albumin quantification (mg/day)<br>Urinary albumin/Cr ratio (mg/gCr) | | | Normal | Microalbuminuria | Macroalbuminuria |
| | | | | Less than 30 | 30~299 | 300 or more |
| High blood pressure<br>Nephritis<br>Polycystic kidney<br>Renal graft<br>Unknown<br>Others | Urinary protein quantification (g/day)<br>Urinary protein/Cr ratio (g/gCr) | | | Normal | Mild proteinuria | High proteinuria |
| | | | | Less than 0.15 | 0.15~0.49 | 0.50 or more |
| GFR category (mL/min/ 1.73 m$^2$) | G1 | Normal or elevated value | ≥90 | | | |
| | G2 | Normal or mild reduction | 60~89 | | | |
| | G3a | Mild to moderate reduction | 45~59 | | | |
| | G3b | Moderate to severe reduction | 30~44 | | | |
| | G4 | Severe reduction | 15~29 | | | |
| | G5 | End-stage kidney disease (ESKD) | <15 | | | |

The severity is evaluated by a stage in which the primary disease, the GFR category, and the proteinuria category are combined.
Regarding the severity of CKD, the risks of mortality, end-stage kidney disease, and cardiovascular mortality increase as the stage increases in the order of , , and with reference to the stage .

"Renal failure" as used herein is included in the "chronic kidney disease" described above. For example, in the case of humans, "renal failure" refers to a condition in which eGFR is less than 45 ml/min/1.73 m$^2$, preferably less than 30 ml/min/1.73 m$^2$, and more preferably less than 15 ml/min/1.73 m$^2$ in chronic kidney disease.

"Individual" as used herein is not particularly limited, and includes humans and non-human mammals. Examples of non-human mammals include bovines, horses, sheep, goats, pigs, dogs, cats, rabbits, monkeys, and the like. Humans, cats, and dogs are preferable. There is no limitation on the age or sex of the individual.

"Subject" is an individual to which a test substance is to be administered, and preferably excludes humans. The subject is preferably an individual with a history of declined kidney function or other kidney disease. Individuals that may be the subject preferably have symptoms, such as polyuria, thirst, increased water intake, excessive gastric juice, vomiting, bloody urine, and general malaise. Further, individuals that may be the subject include individuals suspected of having kidney damage or chronic kidney disease according to a known diagnostic method, such as a medical interview, a urine test, a biochemical test of blood, kidney diagnostic imaging, or a renal biopsy, disease animal models, and the like.

"Test tissue" as used herein refers to tissue to which a test substance is to be administered. For example, the test tissue is living tissue *in vitro,* for example, collected from an individual that may be the subject, and cultured *in vitro.*

The tissue may be an entire organ, or a portion of an organ.

"Test cell" as used herein refers to a cell to which a test substance is to be administered. For example, the test cell is a living cell *in vitro,* for example, collected from an individual that may be the subject, and cultured *in vitro.* The cell may be a cell whose passage capability is limited, such as a primary cultured cell; or may be a so-called cultured cell whose passage capability is maintained. Such cells may be cells prepared by genetic engineering.

"Specimen" as used herein includes cells, tissue (the adrenal glands, aorta, brain, lungs, pancreas, pituitary glands, skin, skull, skeletal muscle, spleen, testes, thyroid gland, kidneys, colon, eyeballs, heart, liver, submandibular glands, thymus, adipose tissue, stomach, jejunum, ileum, and the like), body fluids (sweat, secretions from skin, lacrimal fluid, saliva, spinal fluid, ascites fluid, and pleural effusion), urine, blood samples, and the like, derived from a subject described above. As specimens, adipose tissue, skin, hair roots, salivary glands (the parotid glands, submandibular glands, and sublingual glands, and preferably submandibular glands), sweat, secretions from skin, lacrimal fluid, saliva, urine, and blood samples are preferable; and urine, saliva, the parotid glands, blood samples, adipose tissue, hair roots, skin, secretions from skin, and sweat are more preferable.

Moreover, "specimen" may include test tissue itself, a portion of test tissue, a test cell itself, a portion of a test cell, and a culture supernatant of test tissue or a test cell.

In the section "2. Method for obtaining each measurement value" described later, when the measurement value of a kidney function prediction marker protein is obtained, the specimen is preferably a blood sample or a body fluid. In the same section, when the measurement value of mRNA of a kidney function prediction marker protein is obtained, the specimen is preferably tissue, a blood sample, or a body fluid.

"Blood sample" as used herein includes blood (whole blood) collected from a subject, or serum, plasma, or the like prepared from the blood. When the measurement value of a kidney function prediction marker protein is obtained, the blood sample is more preferably serum or plasma, and even more preferably serum. When the measurement value of mRNA of a kidney function prediction marker protein is obtained, it is preferable to use whole blood. The type of anticoagulant used for collecting plasma is not particularly limited. The type of blood sample of a subject used for measurement and the type of blood sample used for determining a predetermined threshold may be the same or different, and are preferably the same. When plasma is used as a blood sample, it is preferable that plasma for determining a predetermined threshold is prepared from blood collected using the same anticoagulant as used for plasma of the subject.

Further, the specimen may be a fresh specimen, or may be a preserved specimen. When the specimen is preserved, it can be preserved in a room-temperature environment, a refrigerated environment, or a frozen environment; and cryopreservation is preferable.

"Test substance" as used herein refers to a substance to be evaluated as to whether it is a candidate substance for an active ingredient, and is not particularly limited. Examples include compounds, proteins, peptides, nucleic acids, lipids, carbohydrates, glycolipids, glycoproteins, metals, and the like. The method for administering a test substance is not particularly limited. When a test substance is administered to a test cell or test tissue, for example, the test substance can be administered to a culture medium of the cell or tissue in an amount of 1 pg/ml to 1 mg/ml. In the case of a subject, a test substance can be administered to the subject in an amount of 1 ng/kg to 1 g/kg per day. The period from administration of a test substance to collection of a specimen is not particularly limited, as long as an effect of the test substance is obtained.

Further, a specimen collected from a subject, test tissue, or test cell treated with a test substance may be referred to "test-substance-treated specimen" in the present specification. In addition, a specimen collected from a subject, test tissue, or test cell that is not treated with a test substance may be referred to as "untreated specimen" in the present specification.

"Healthy individual" is not particularly limited. Preferably, the healthy individual is a human or non-human mammal that is described in the explanation of the term "individual" and that does not show abnormal data in biochemical tests, blood tests, urine tests, serum tests, physiological tests, etc. The age and sex of the healthy individual are not particularly limited.

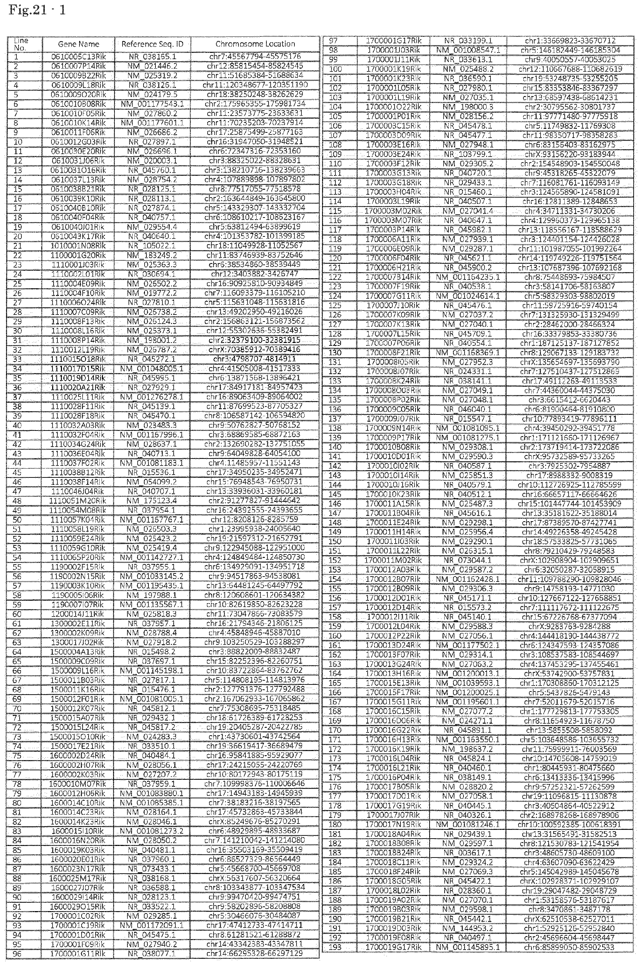

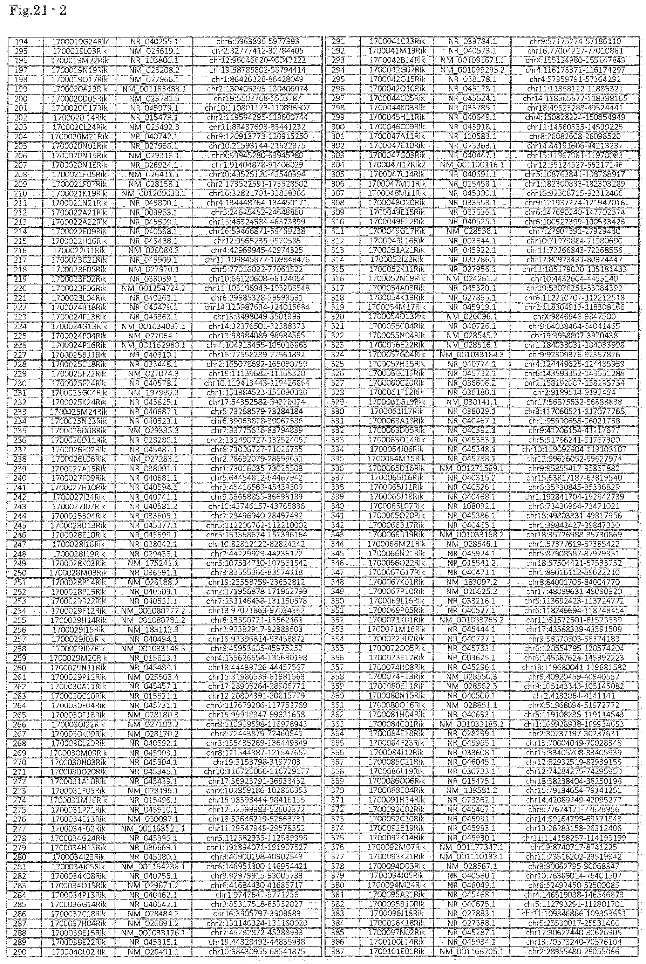

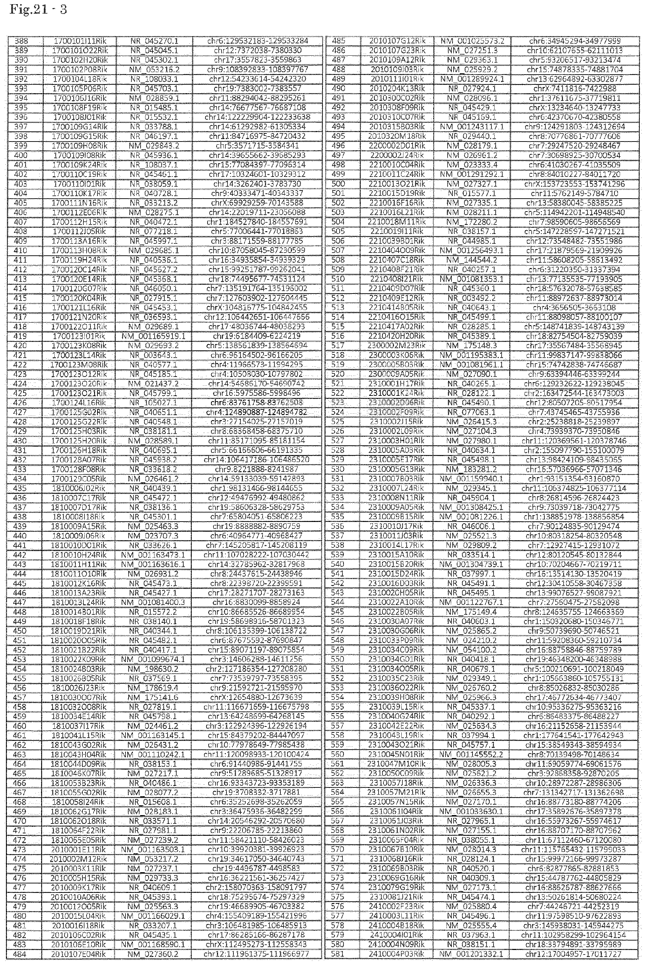

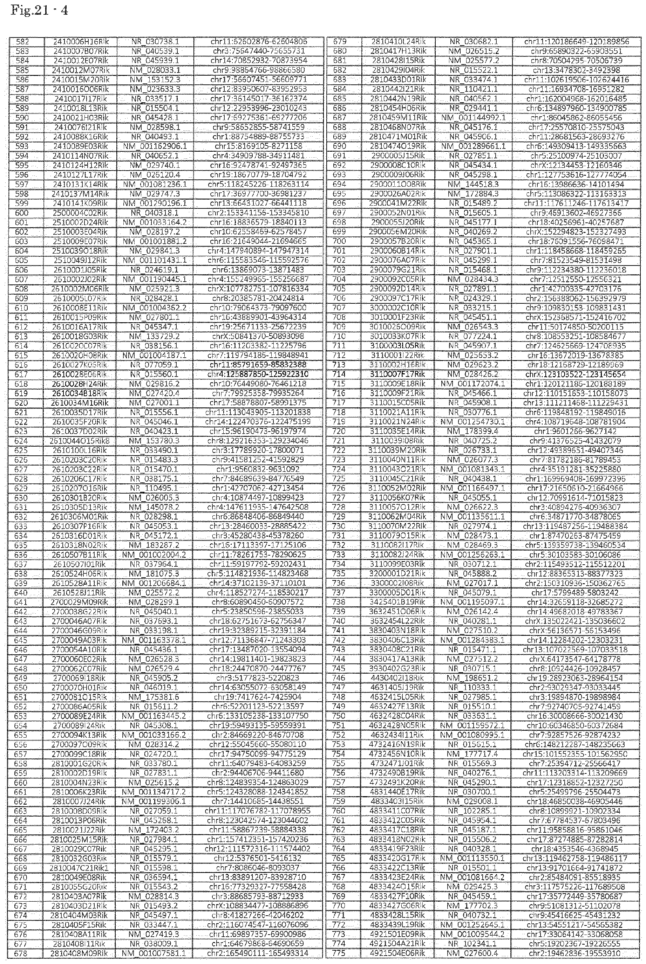

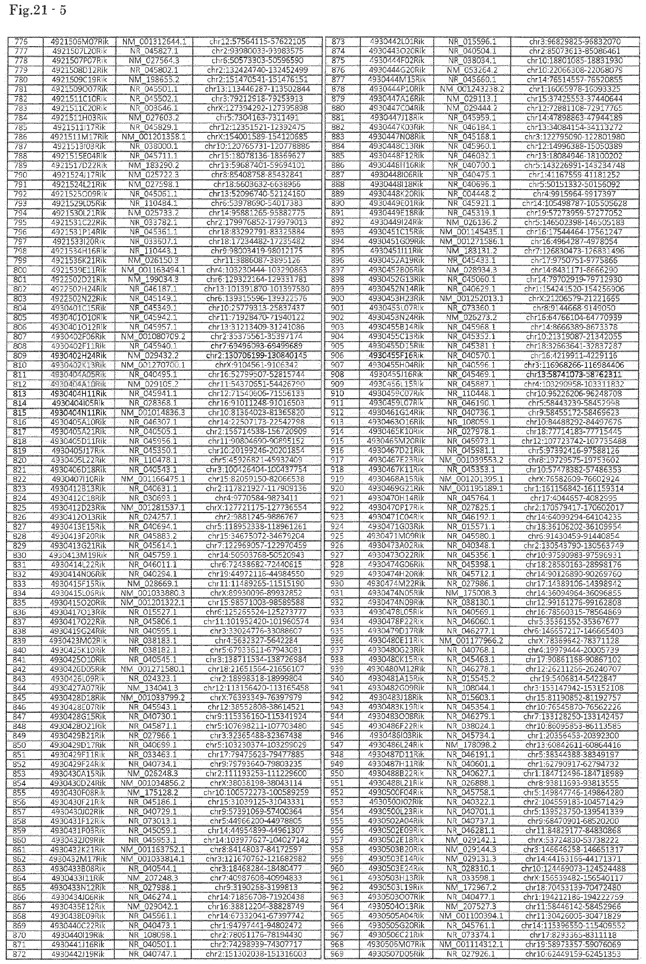

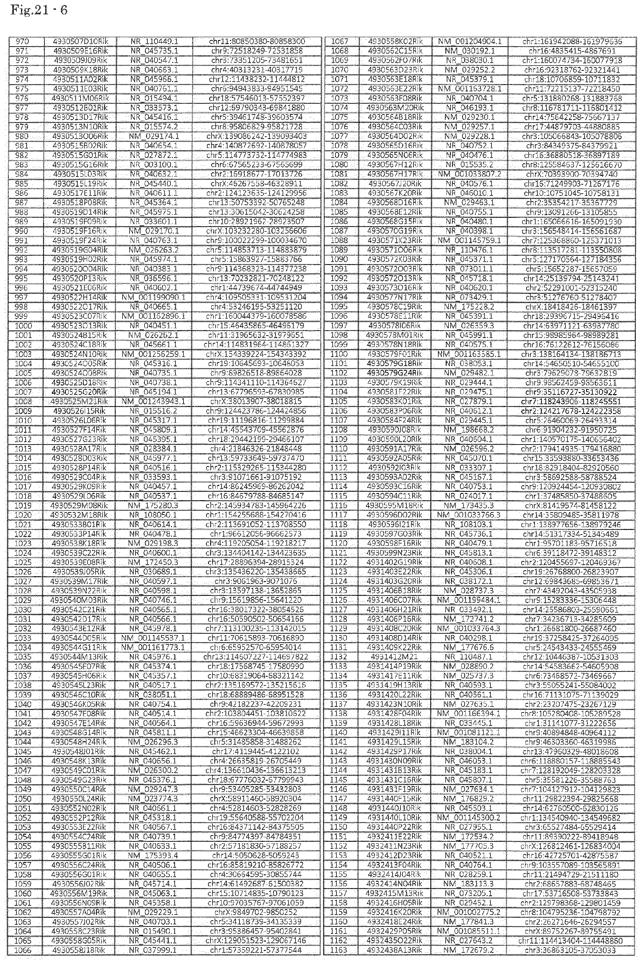

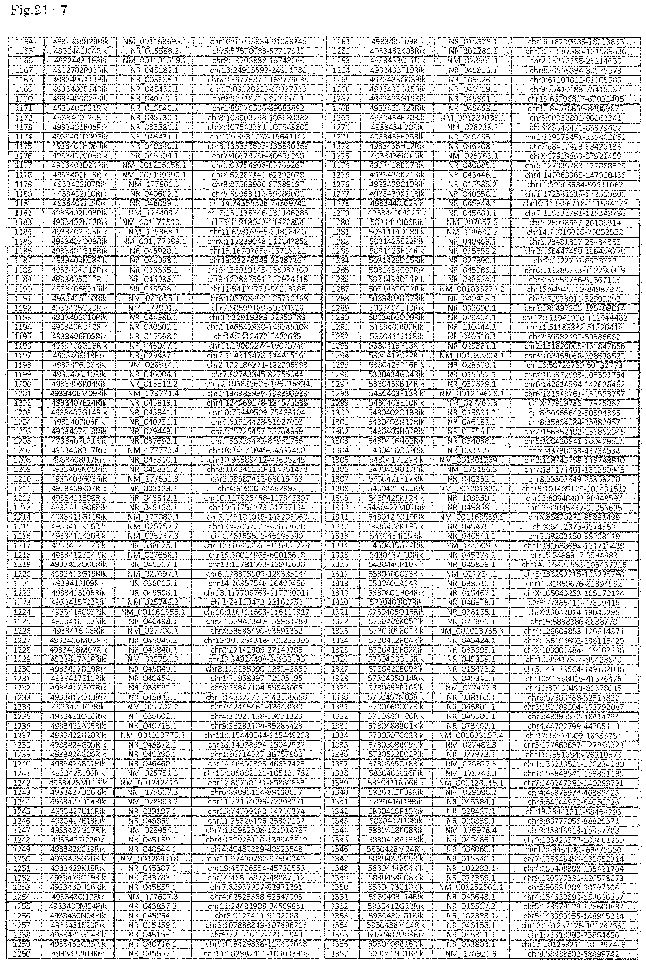

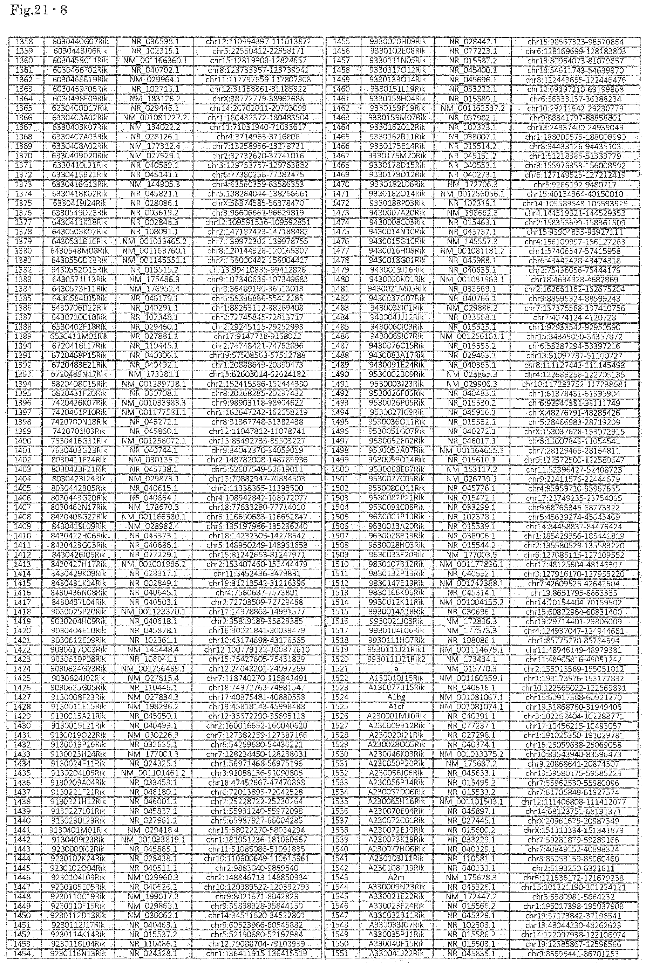

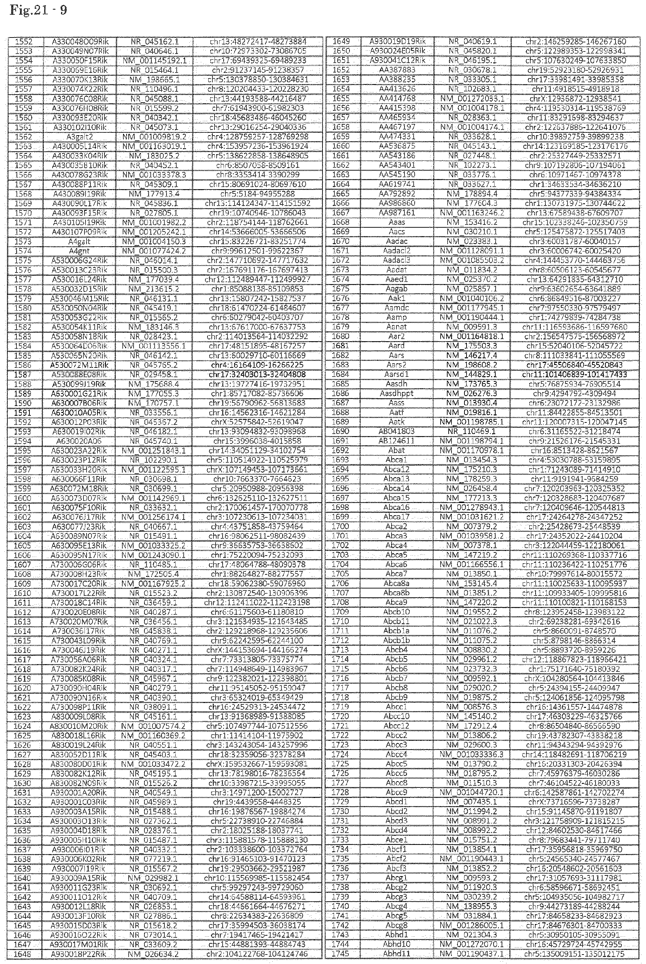

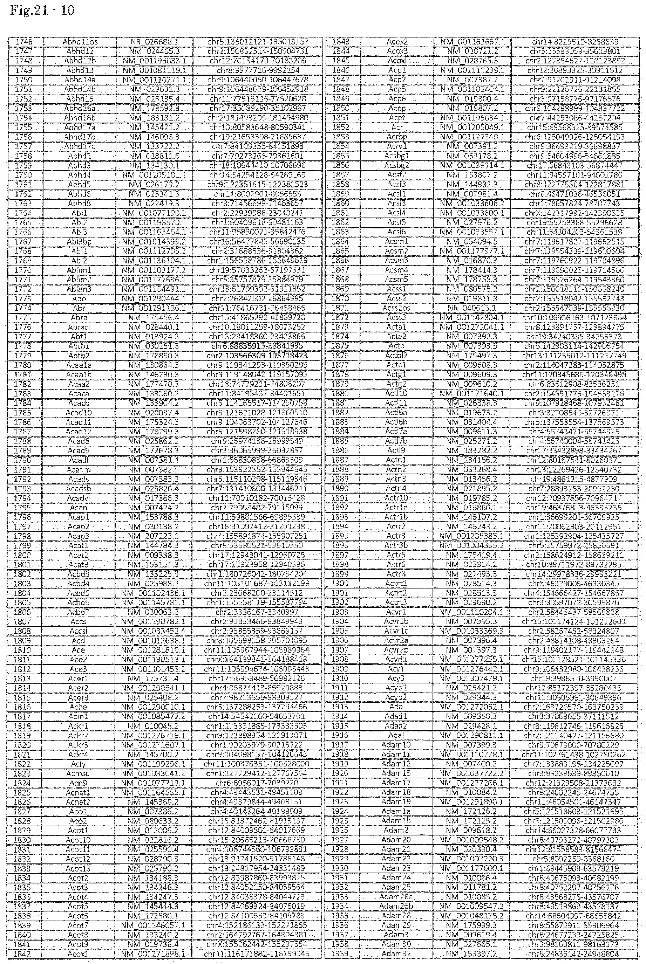

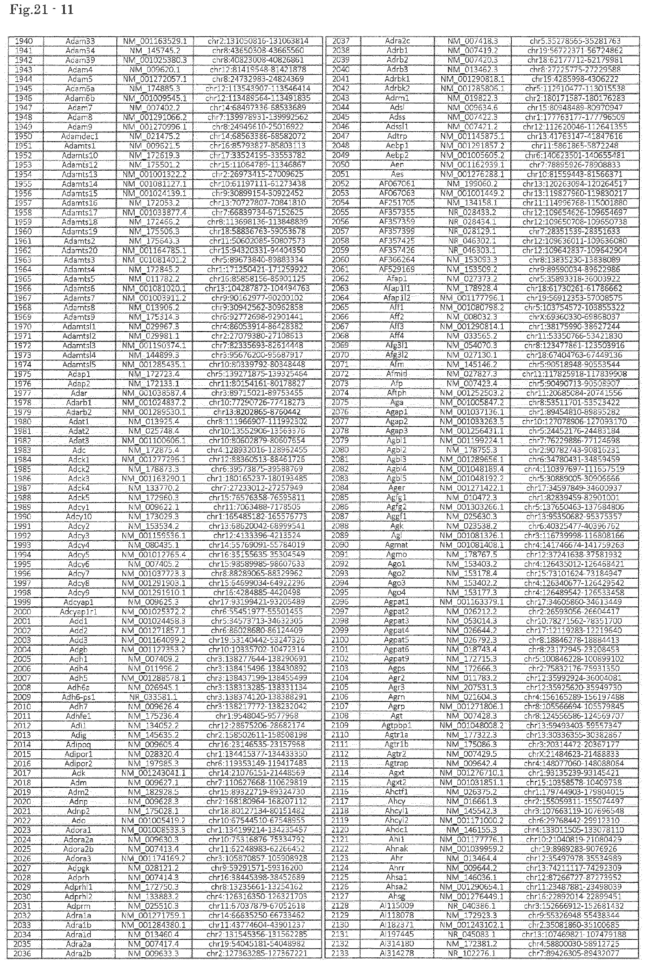

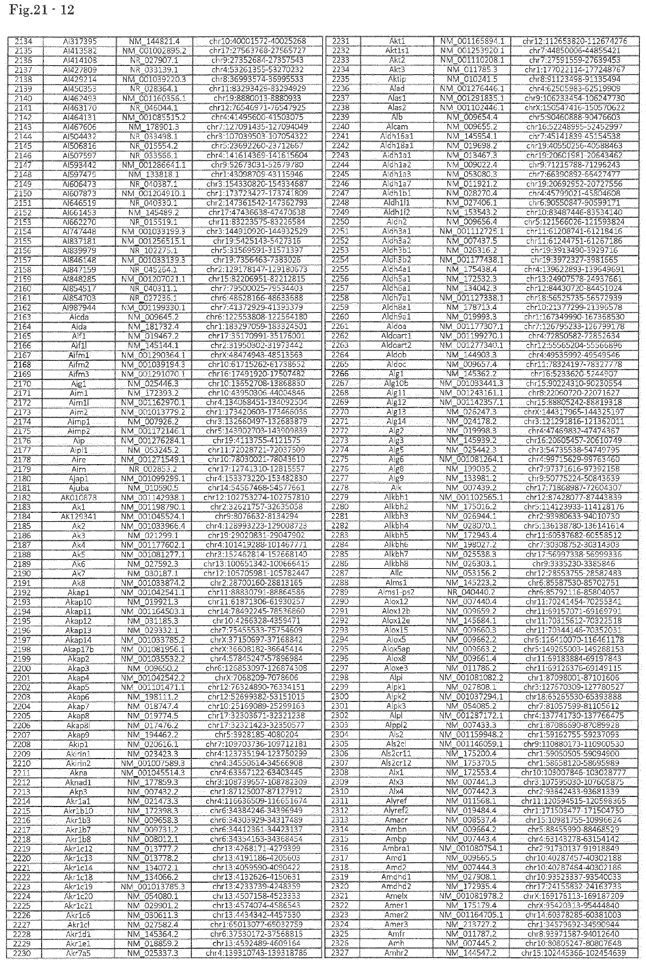

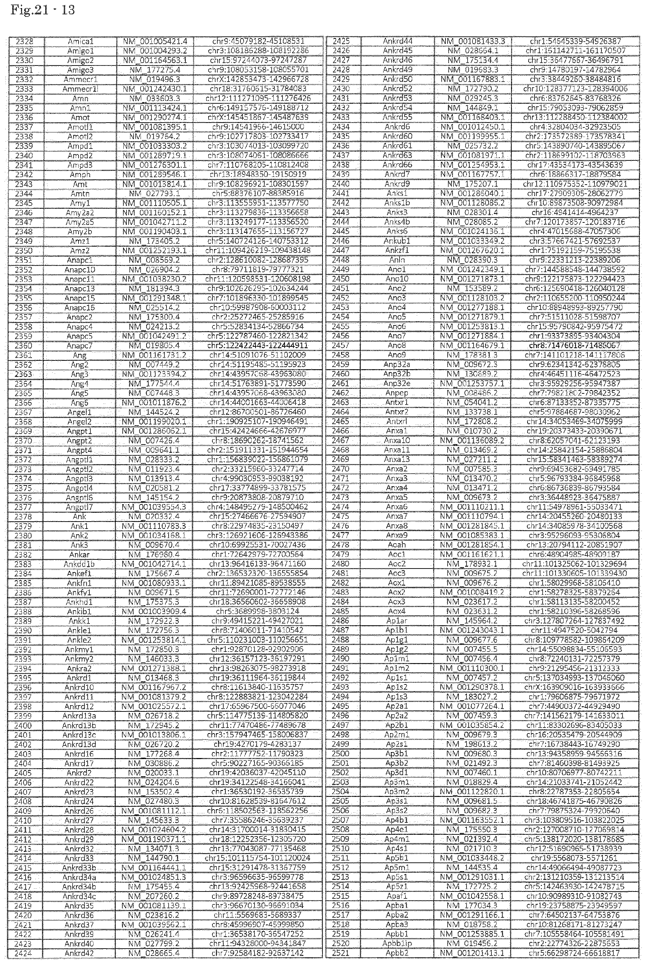

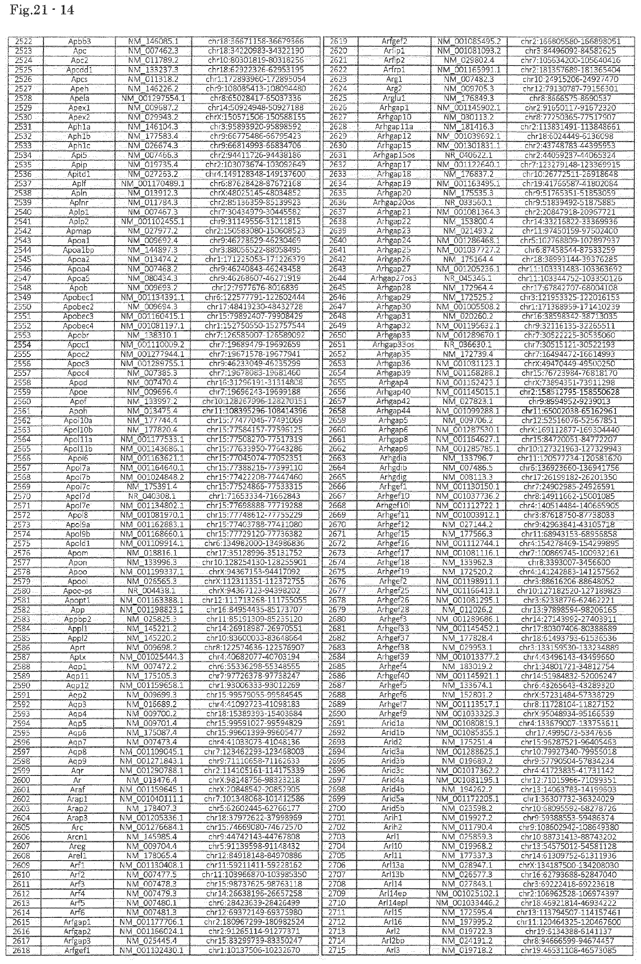

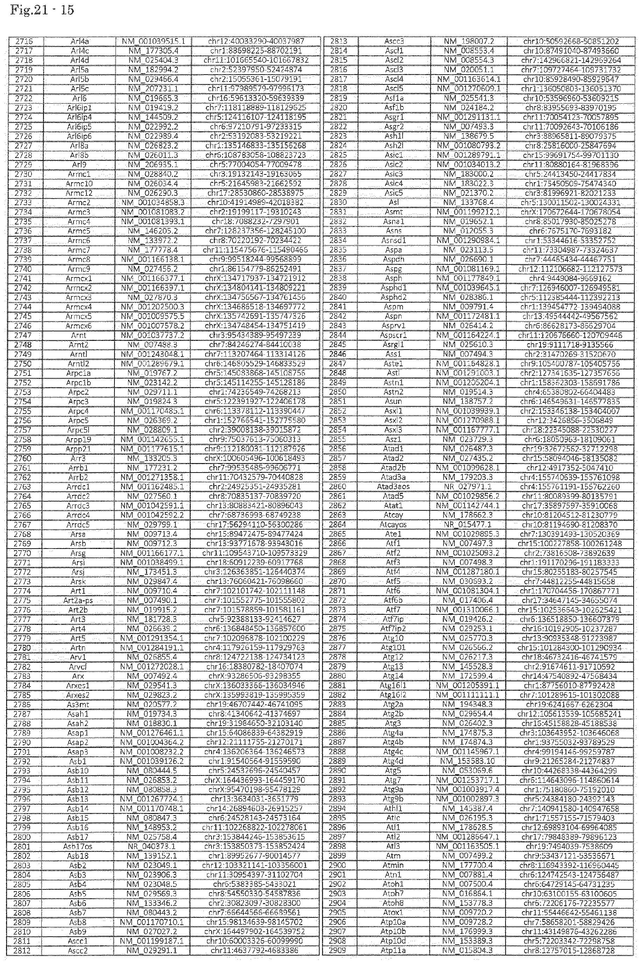

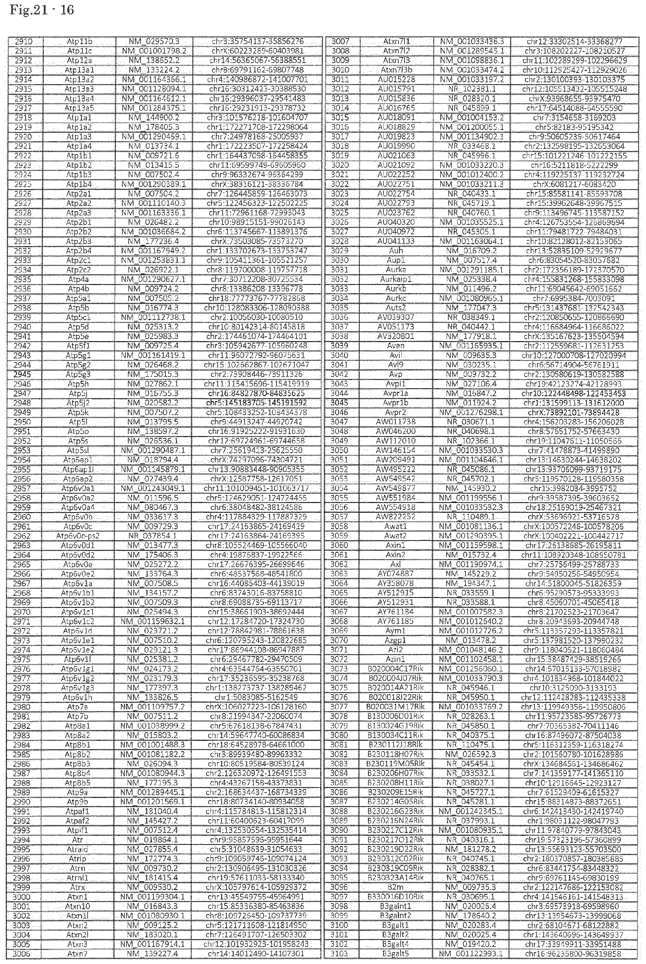

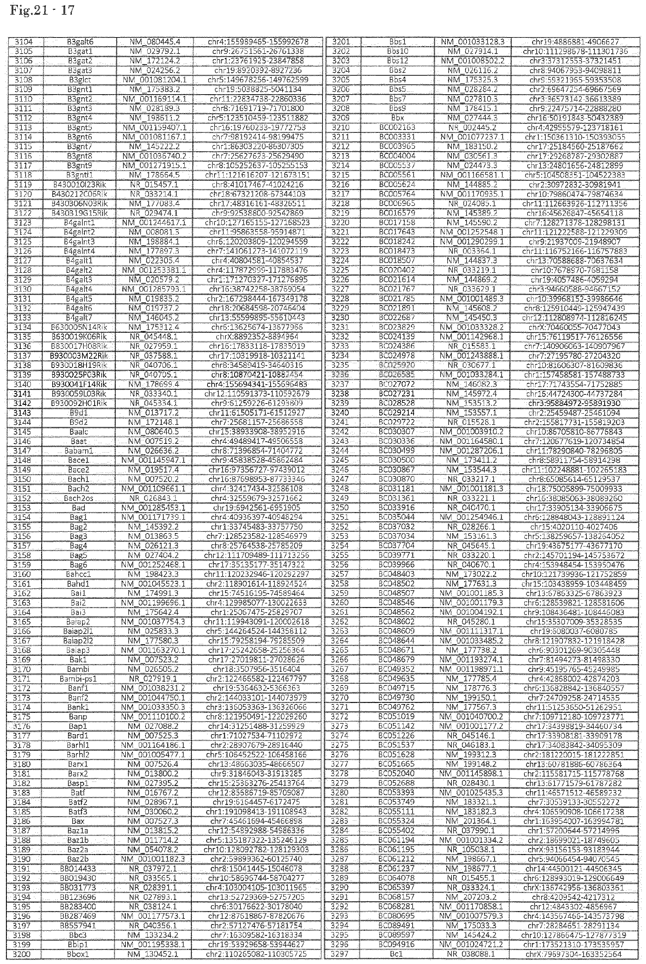

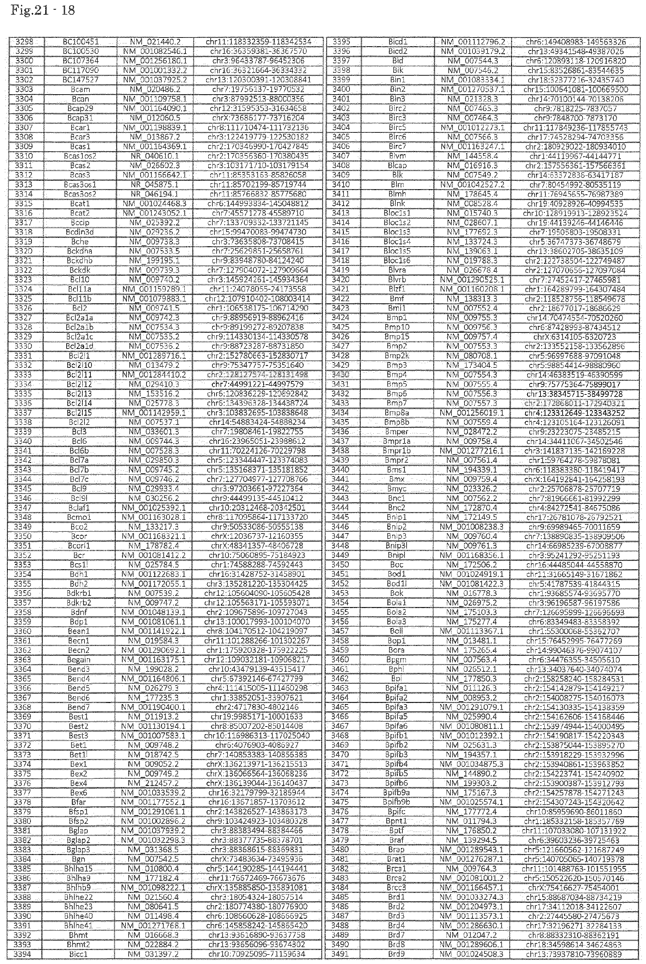

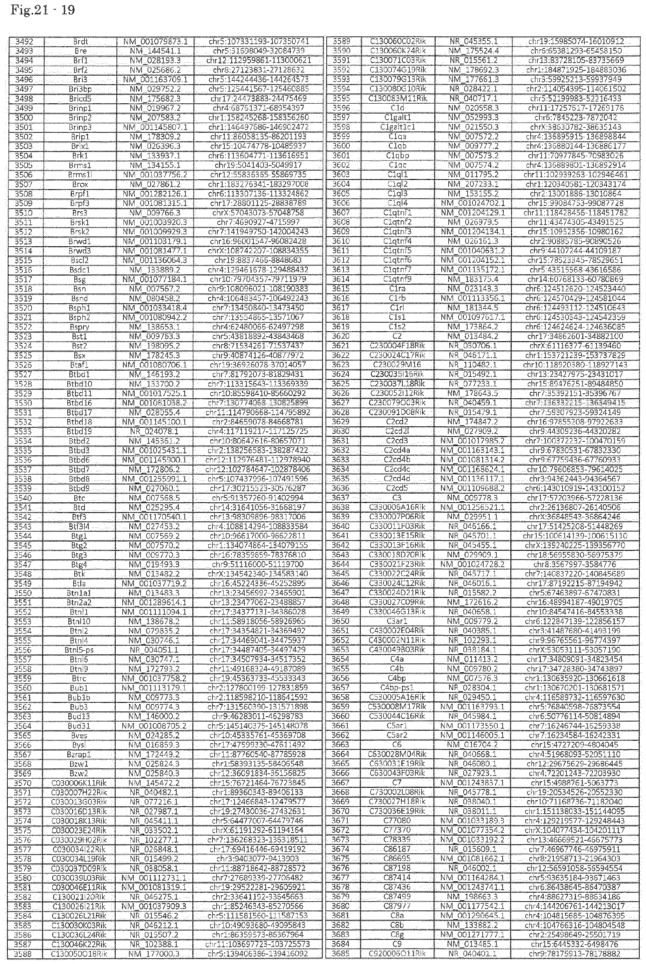

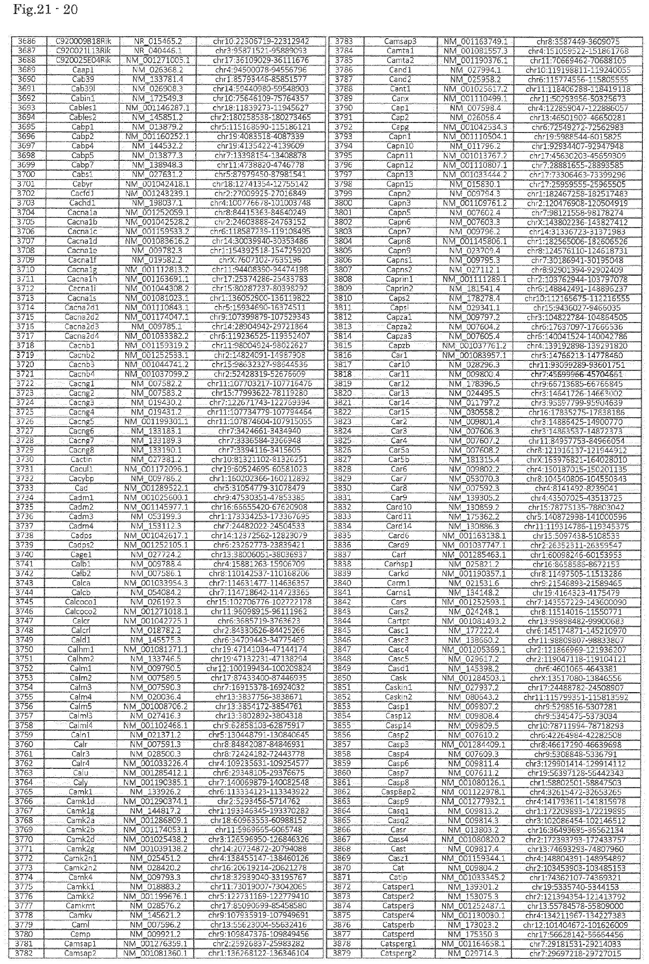

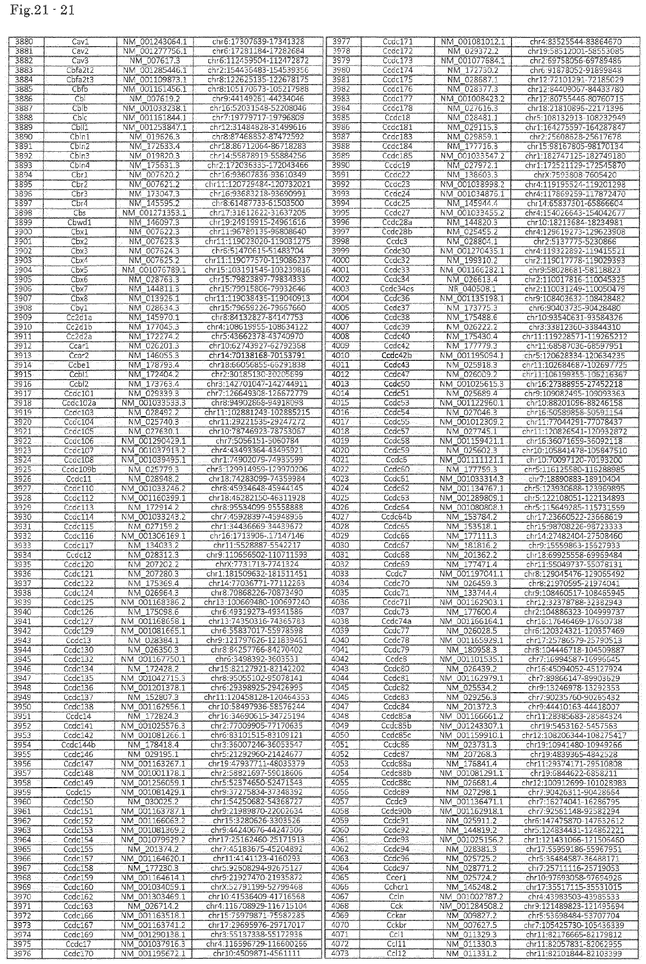

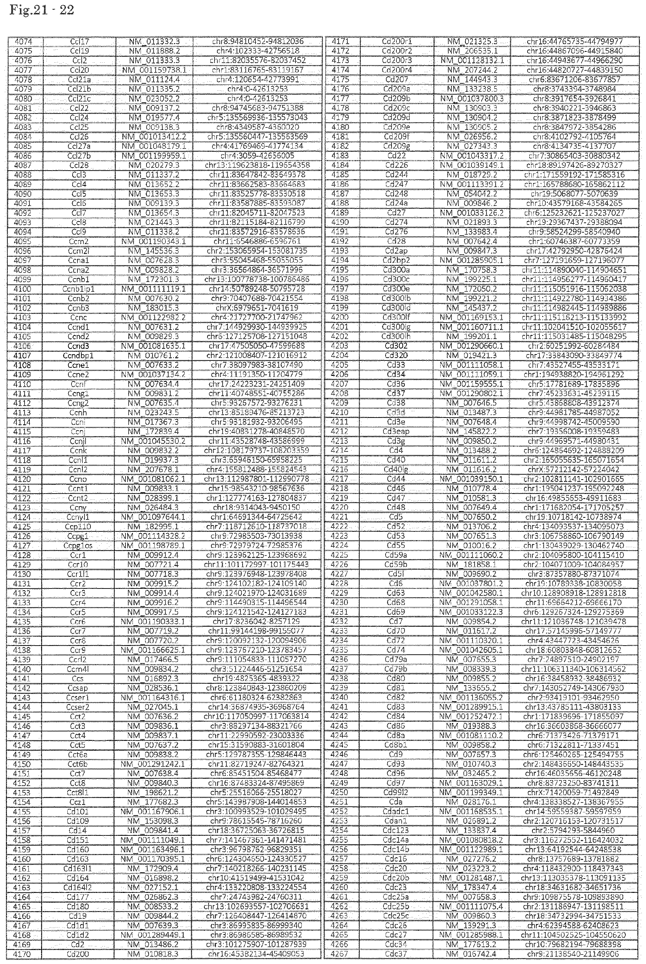

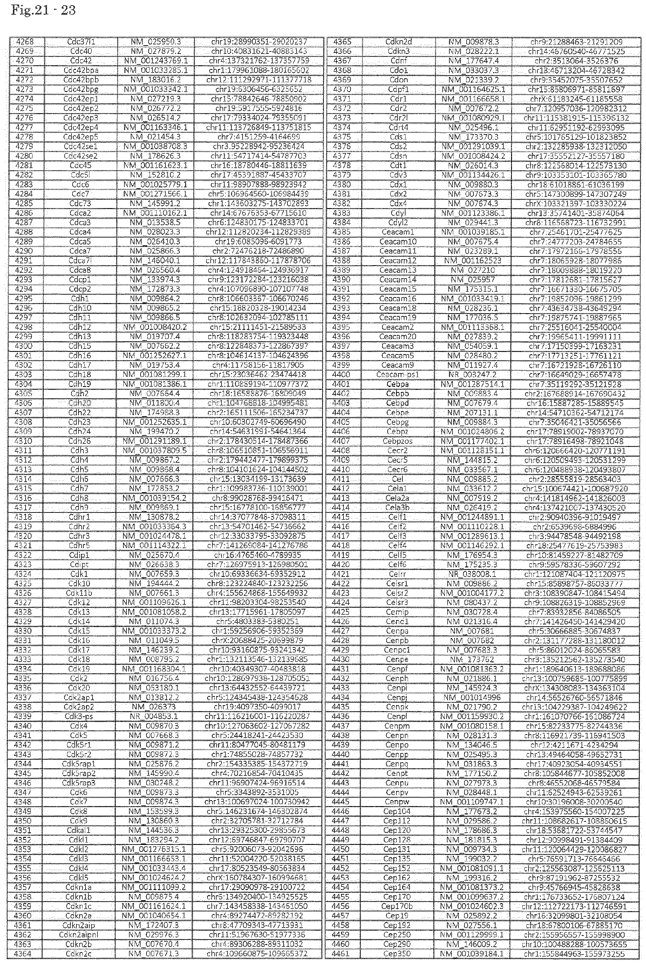

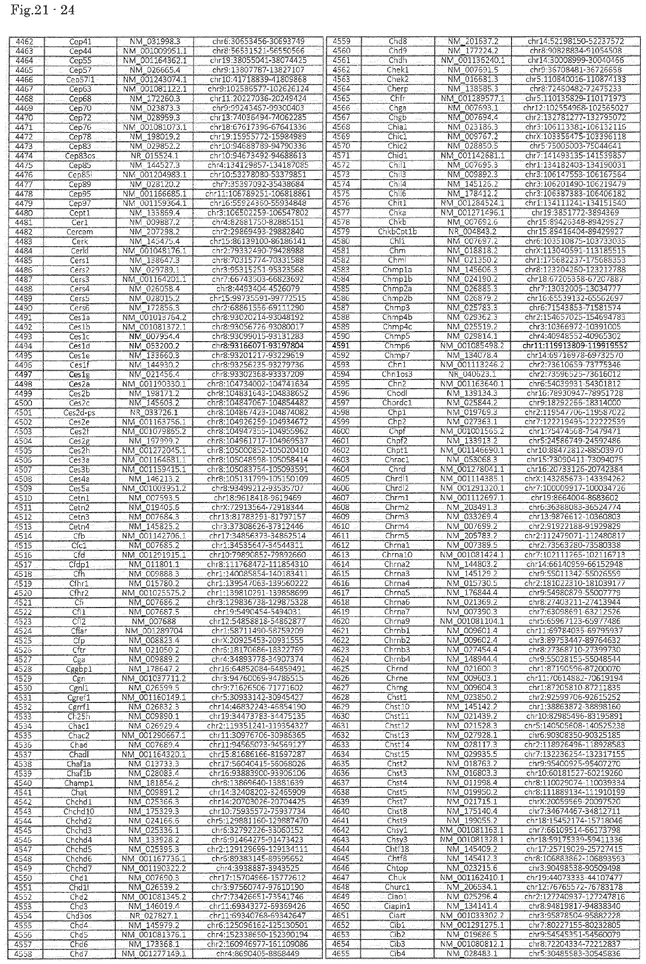

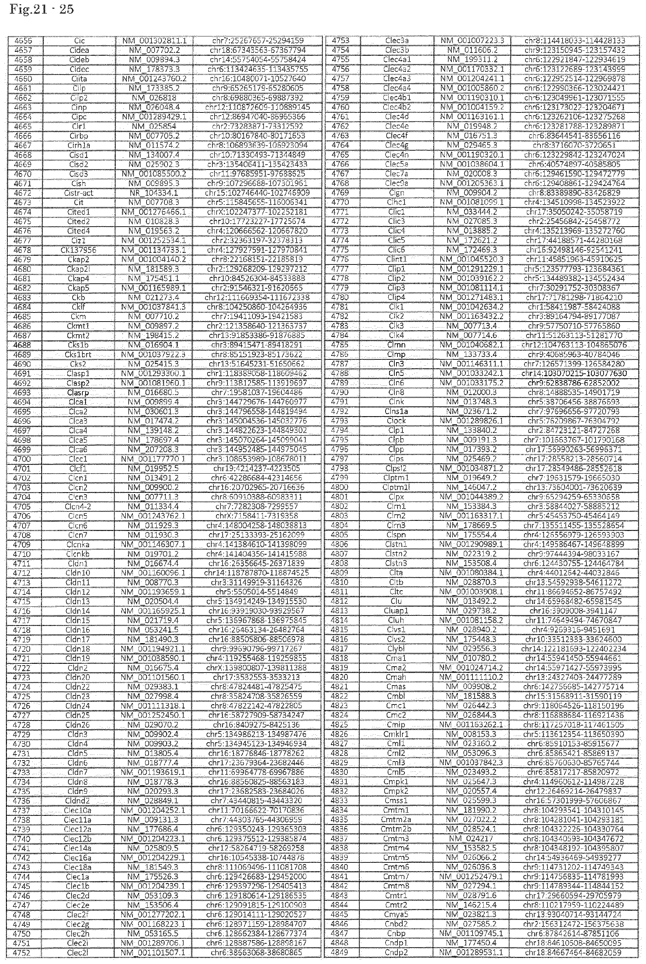

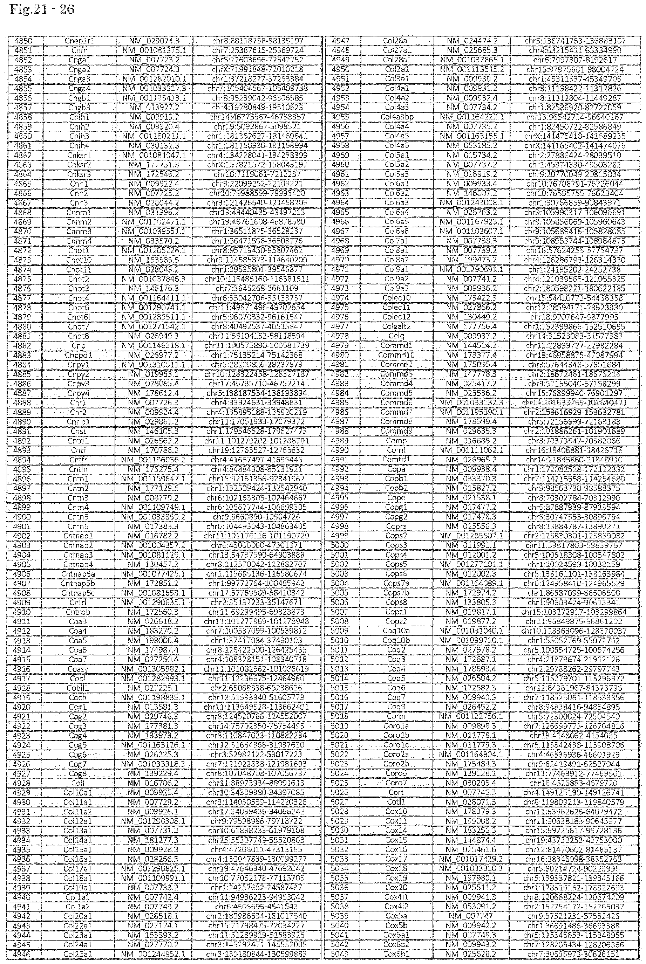

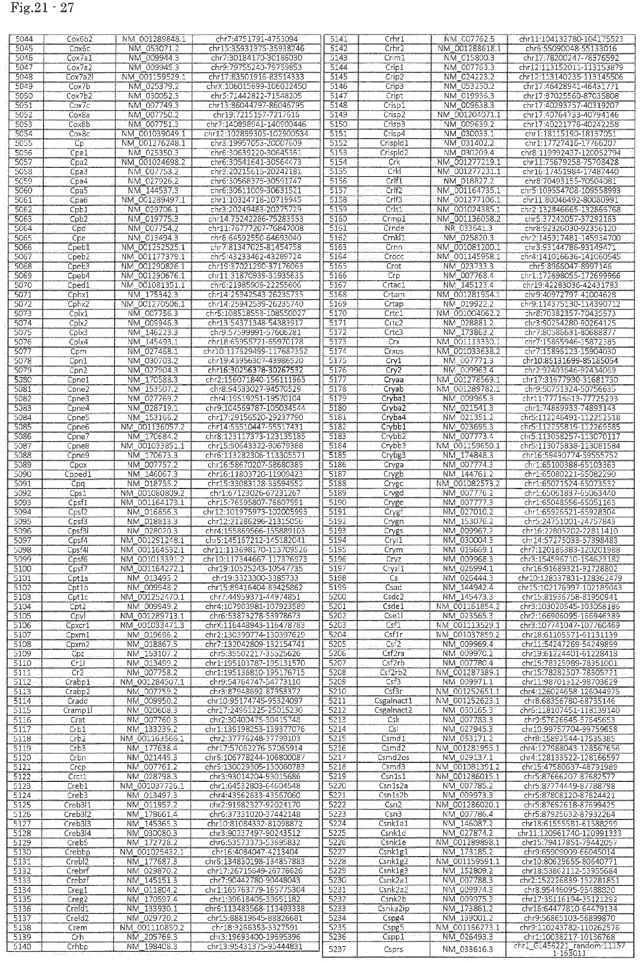

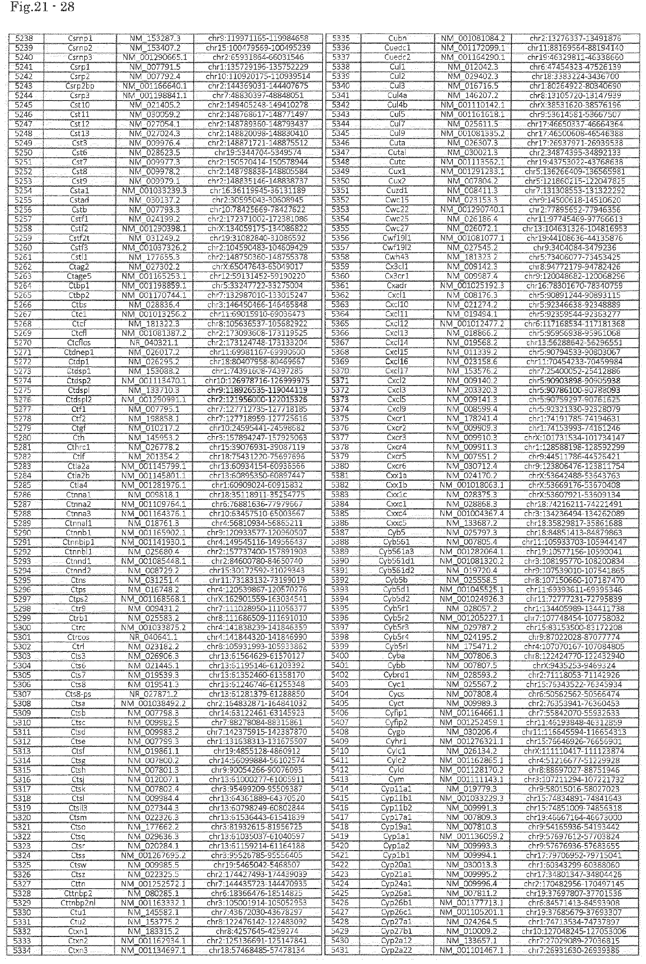

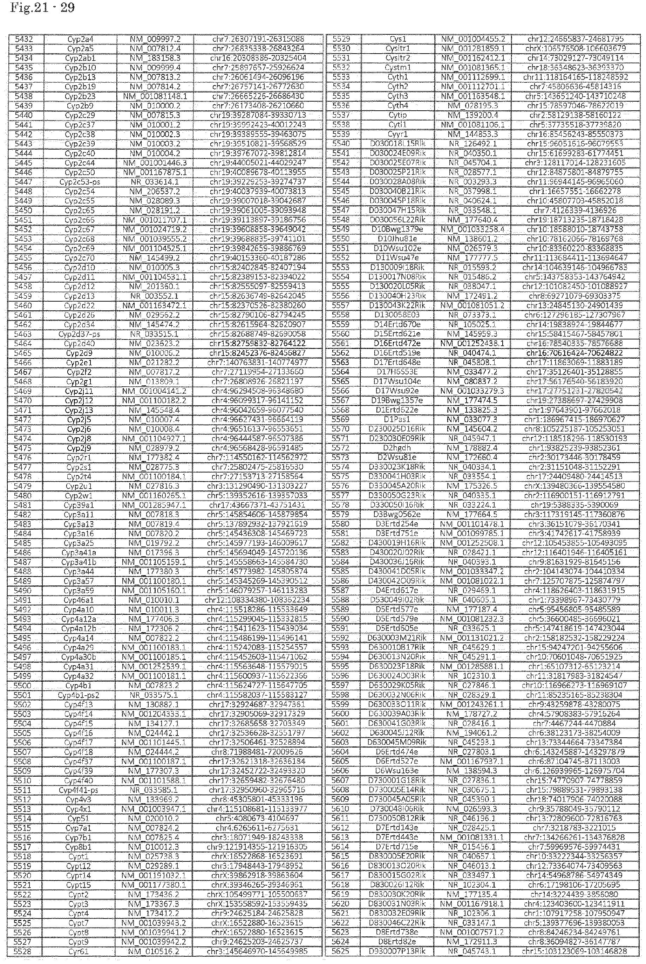

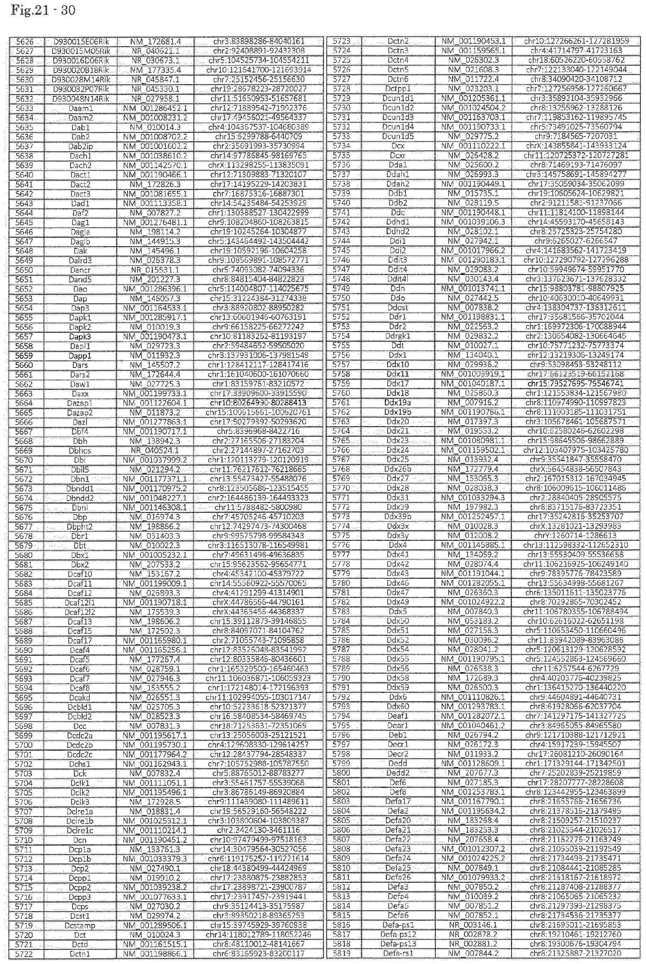

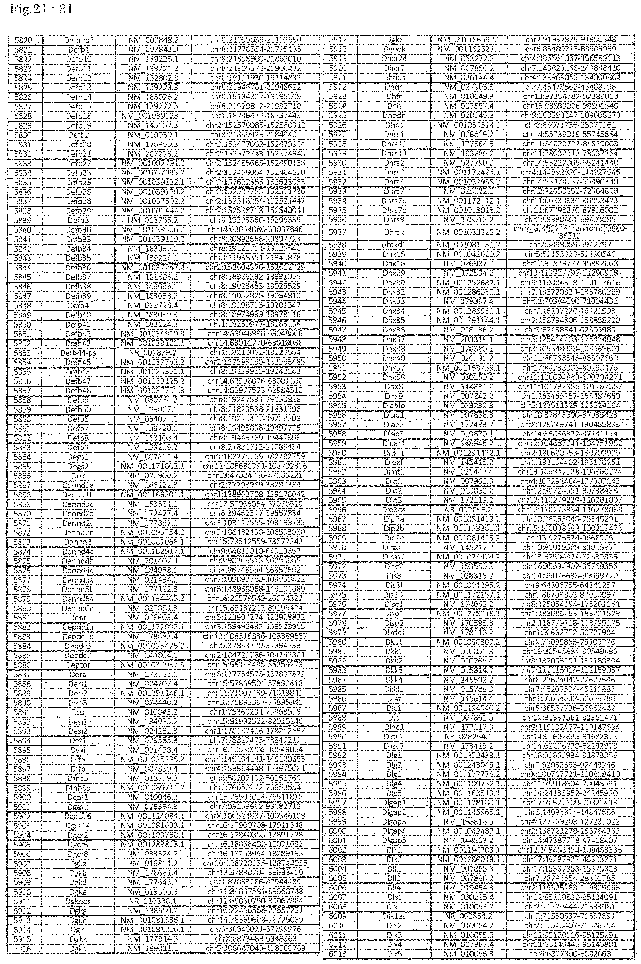

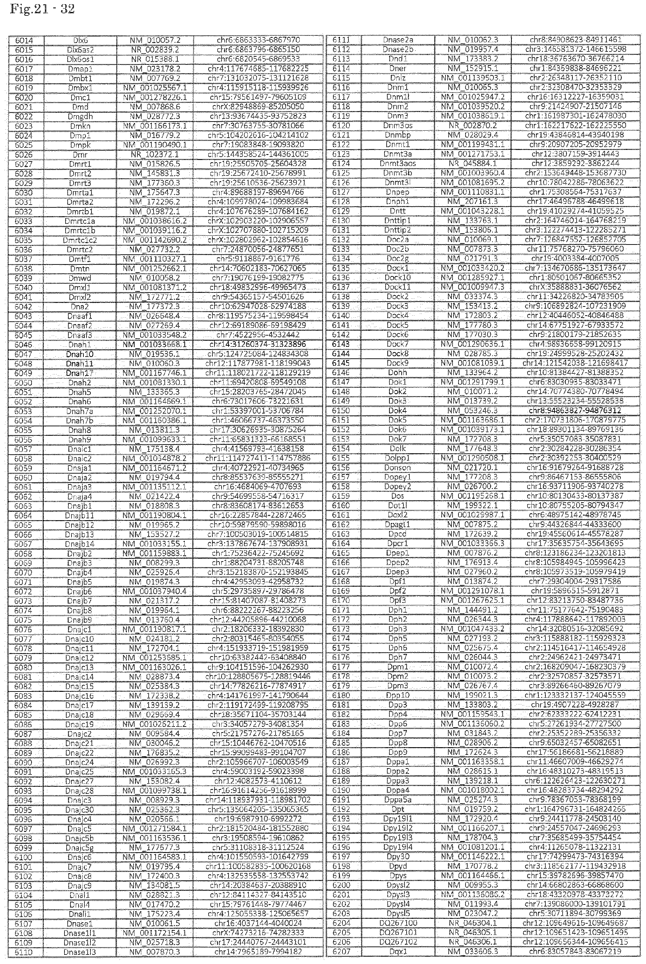

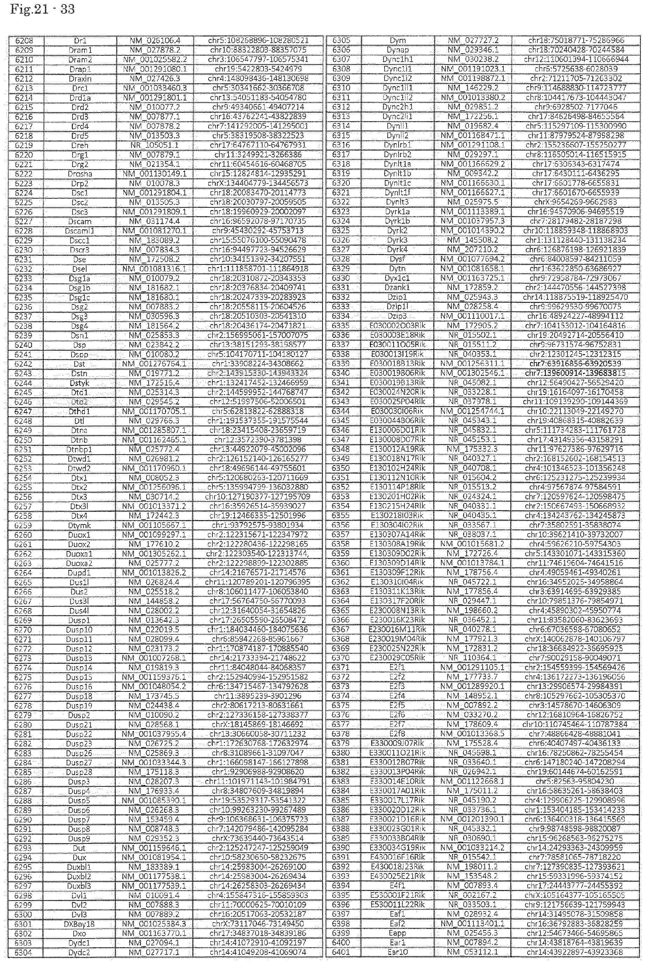

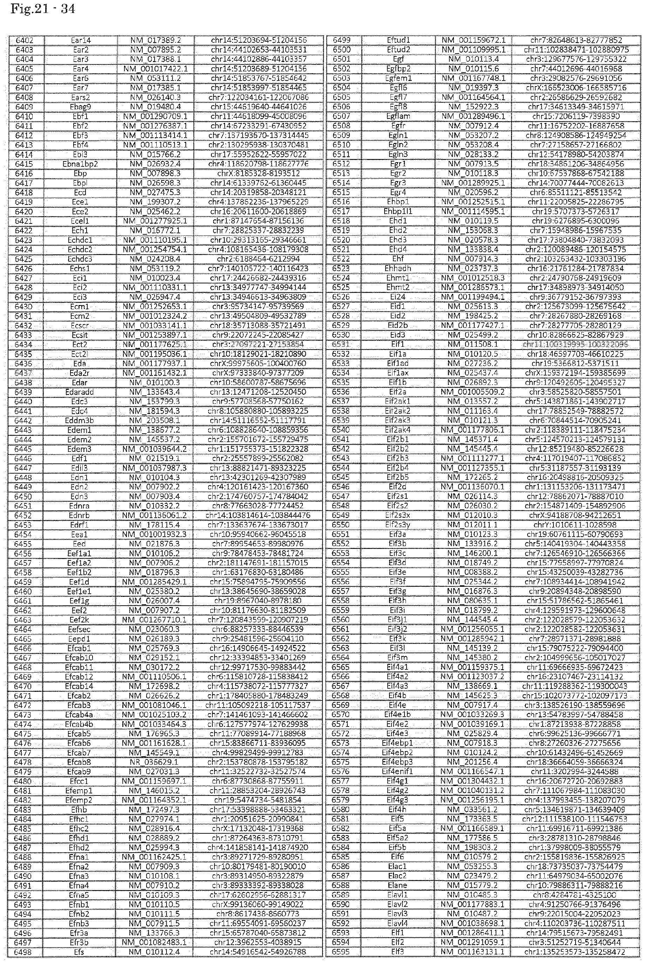

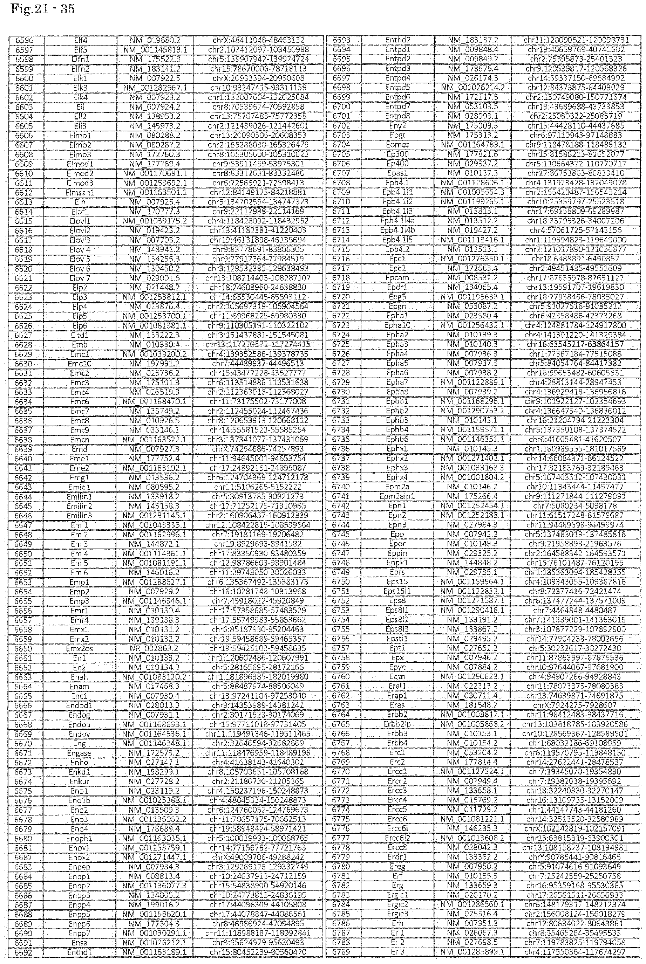

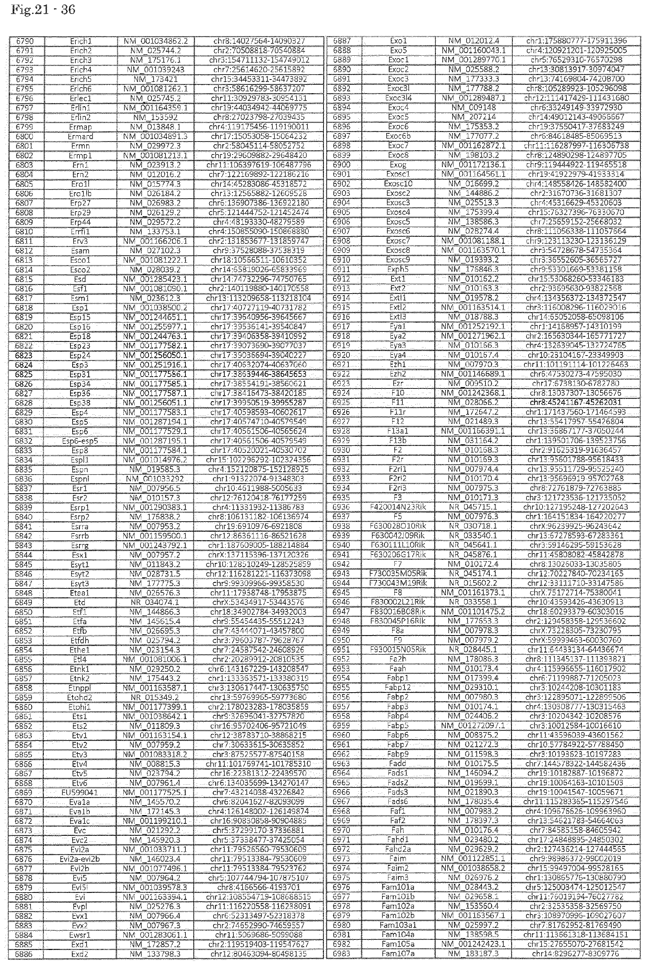

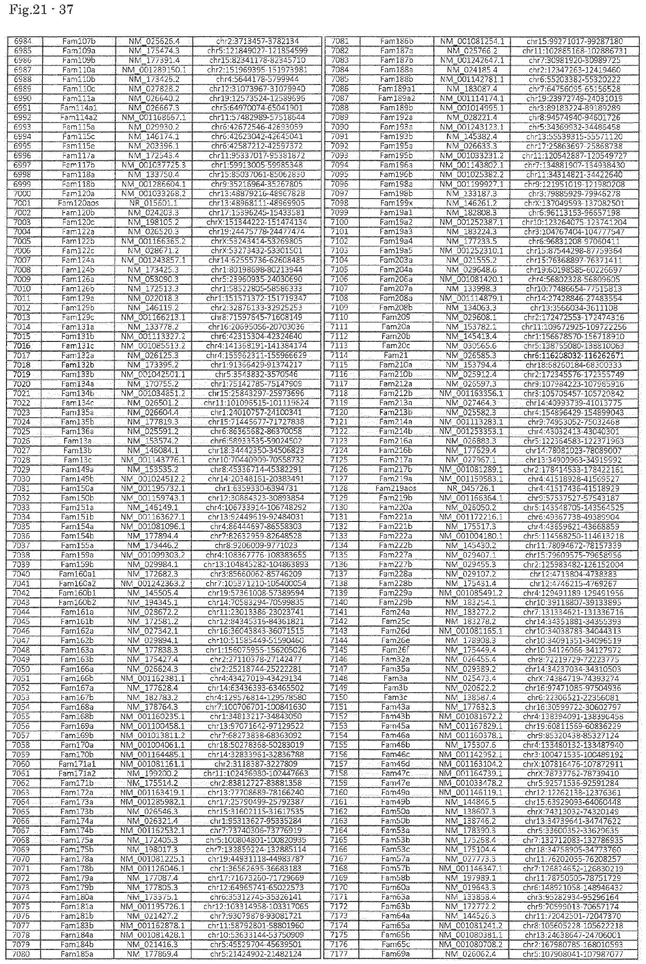

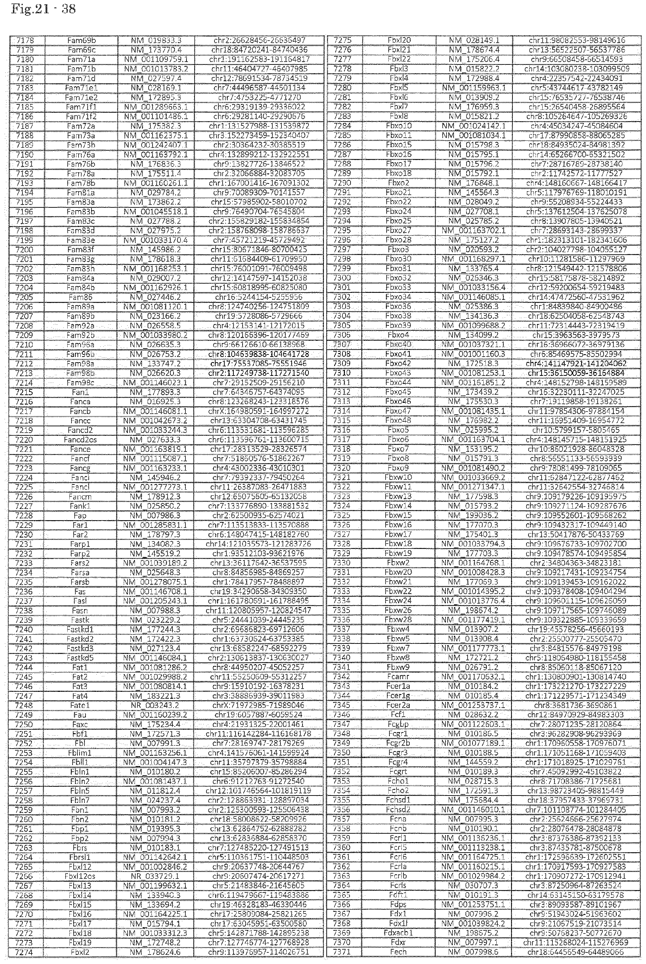

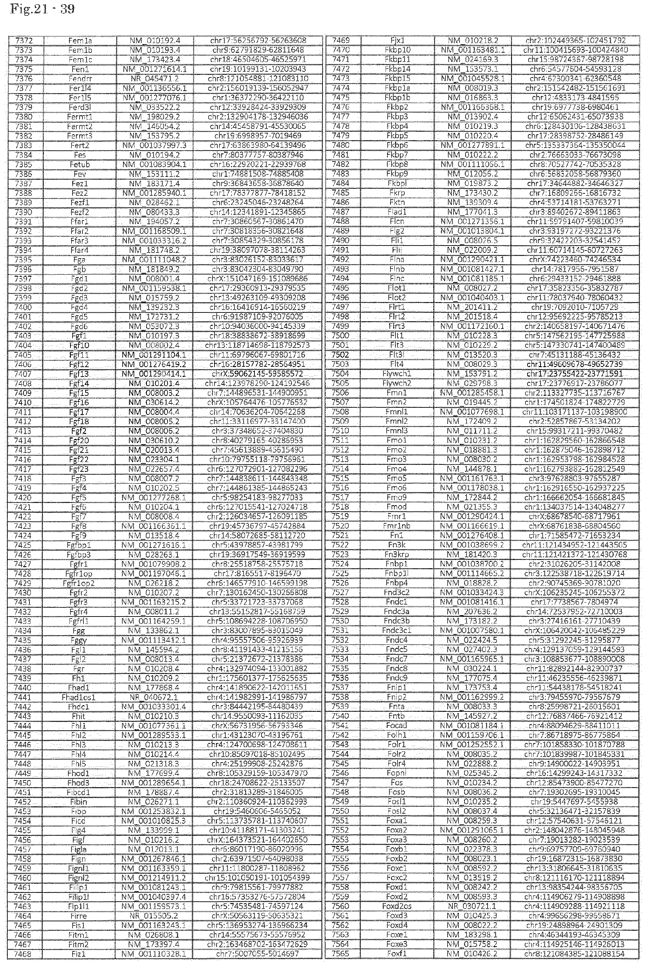

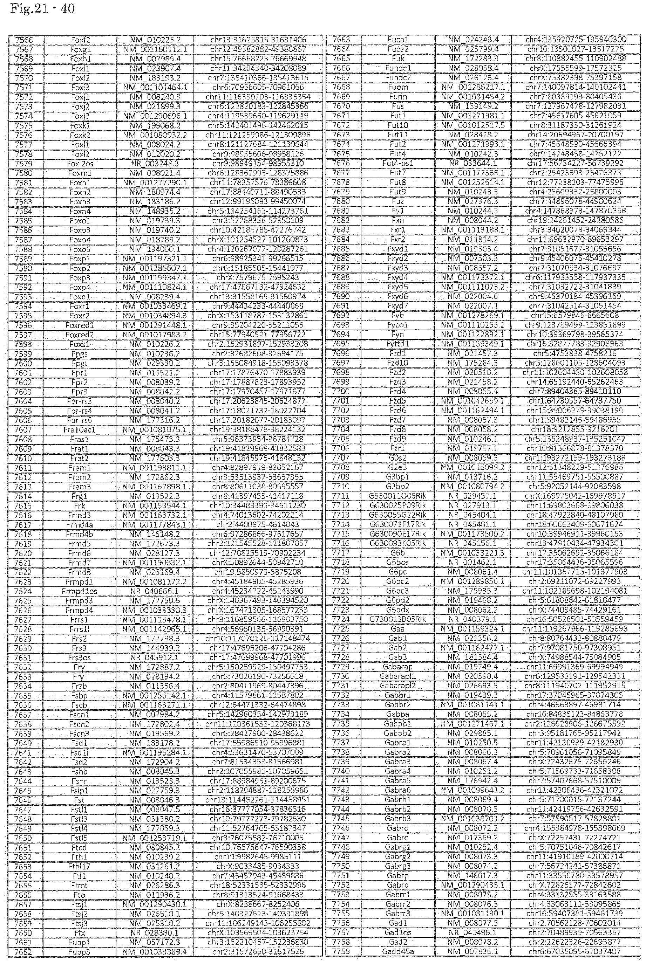

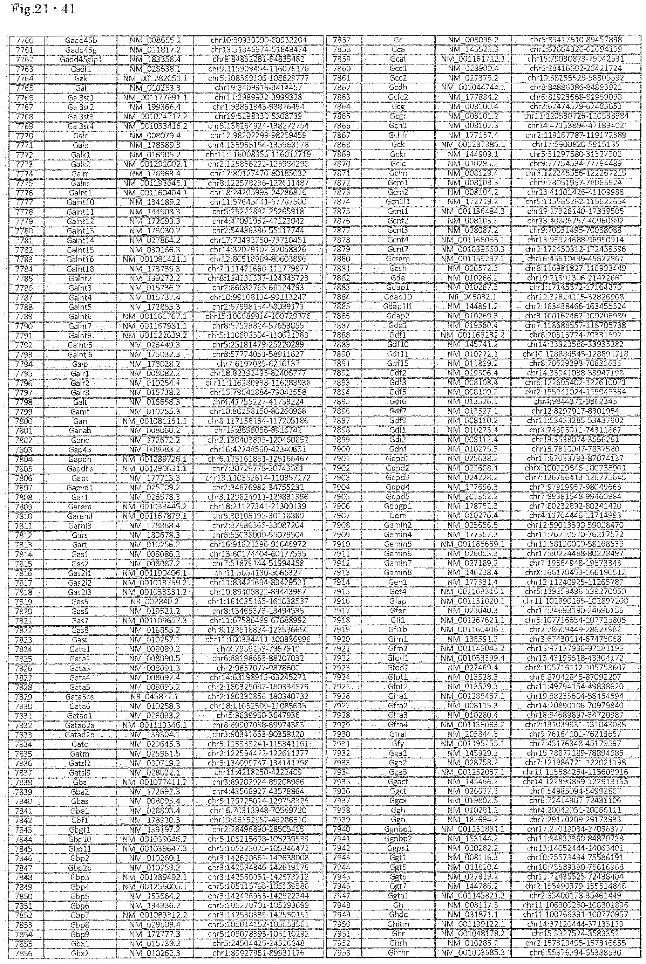

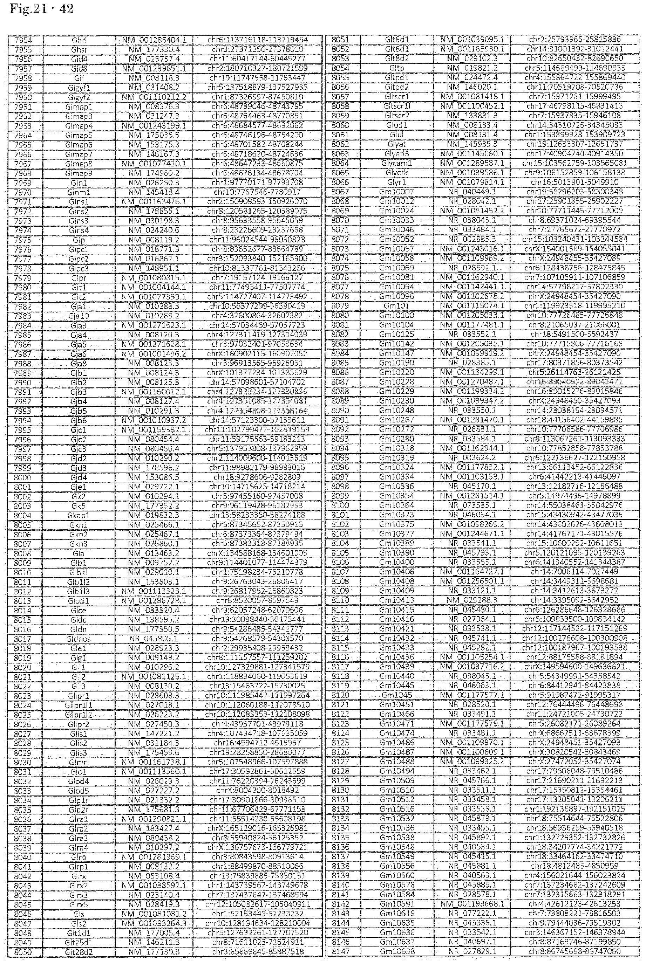

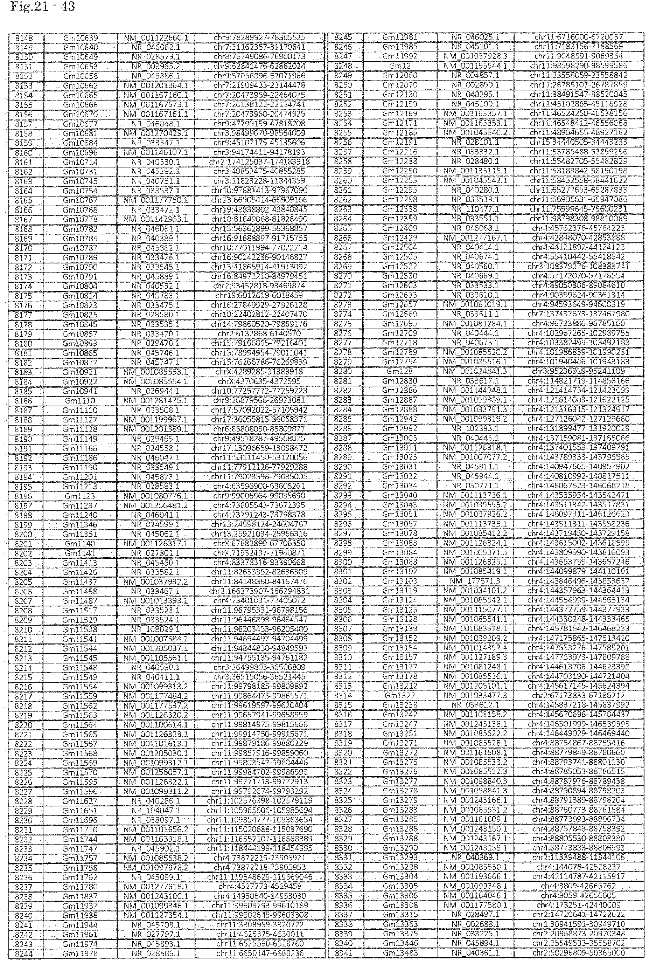

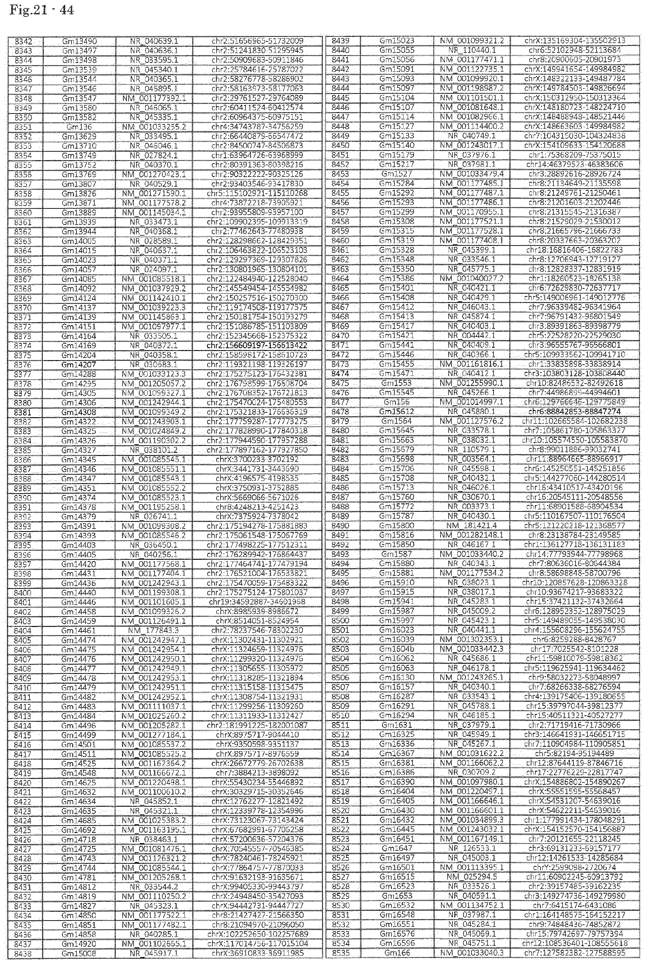

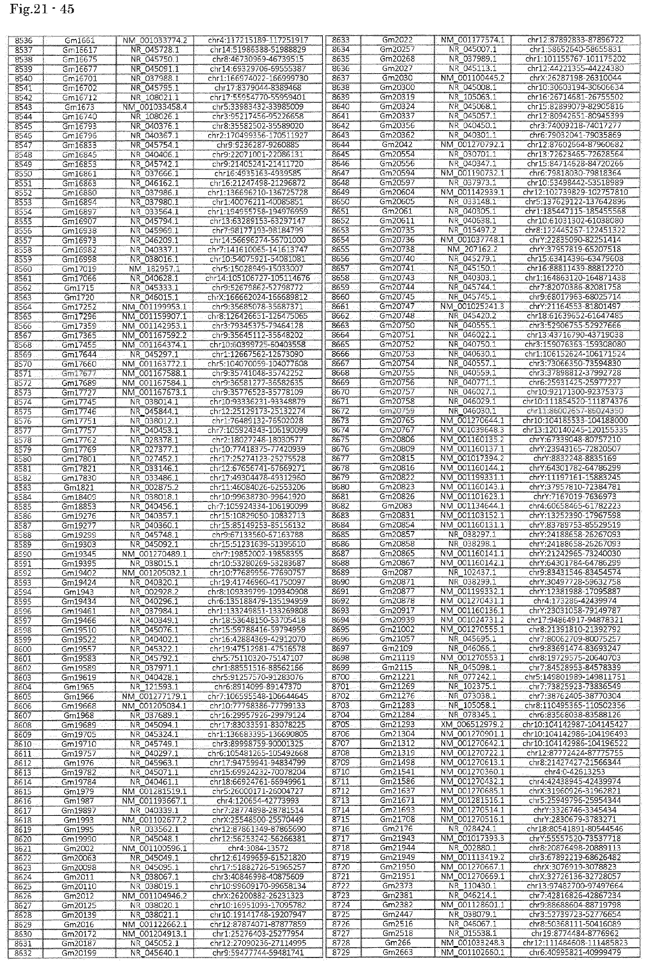

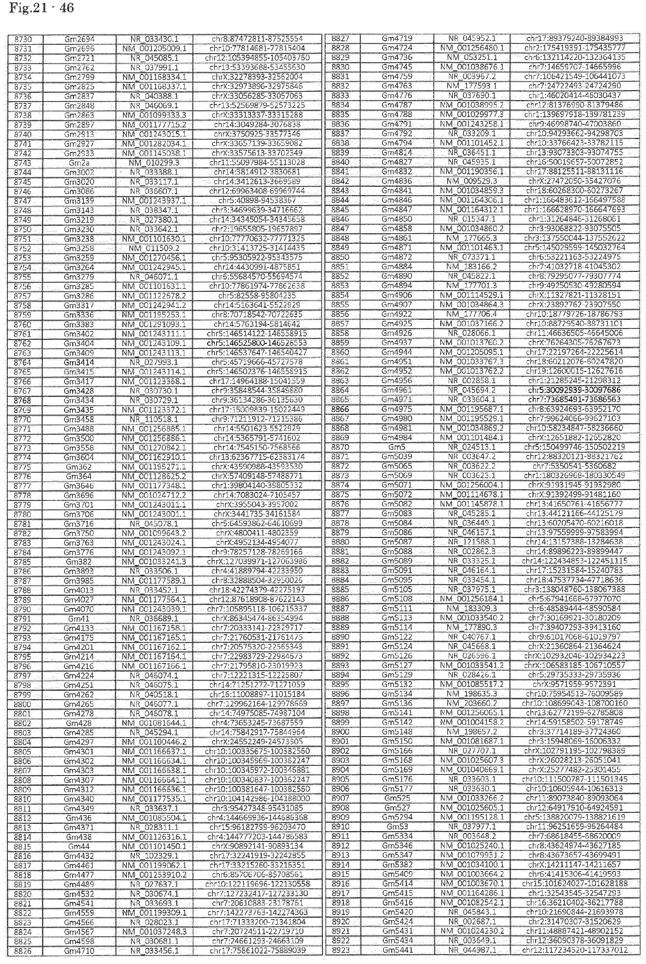

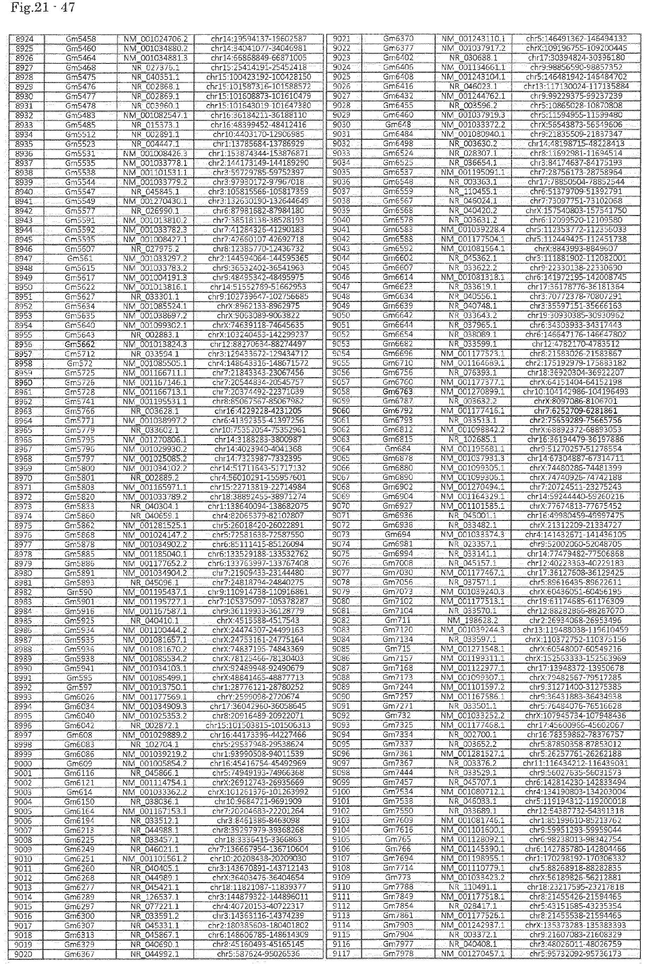

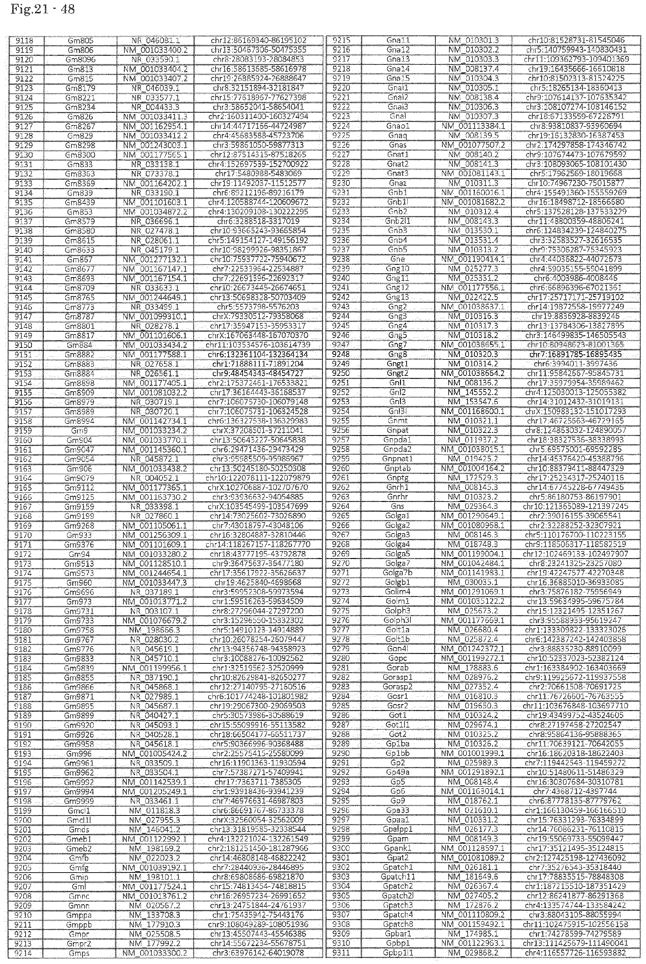

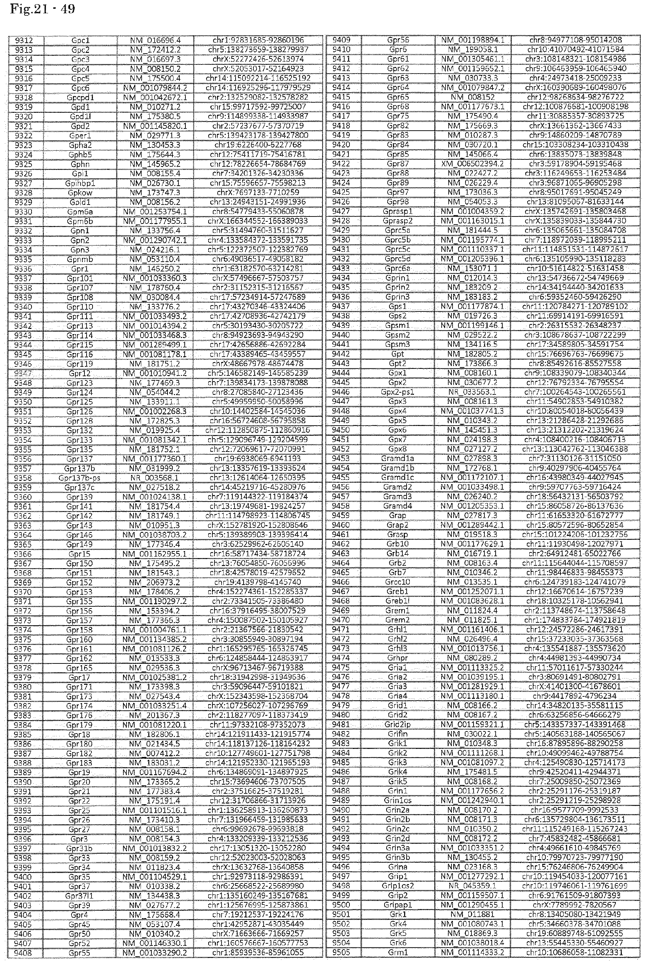

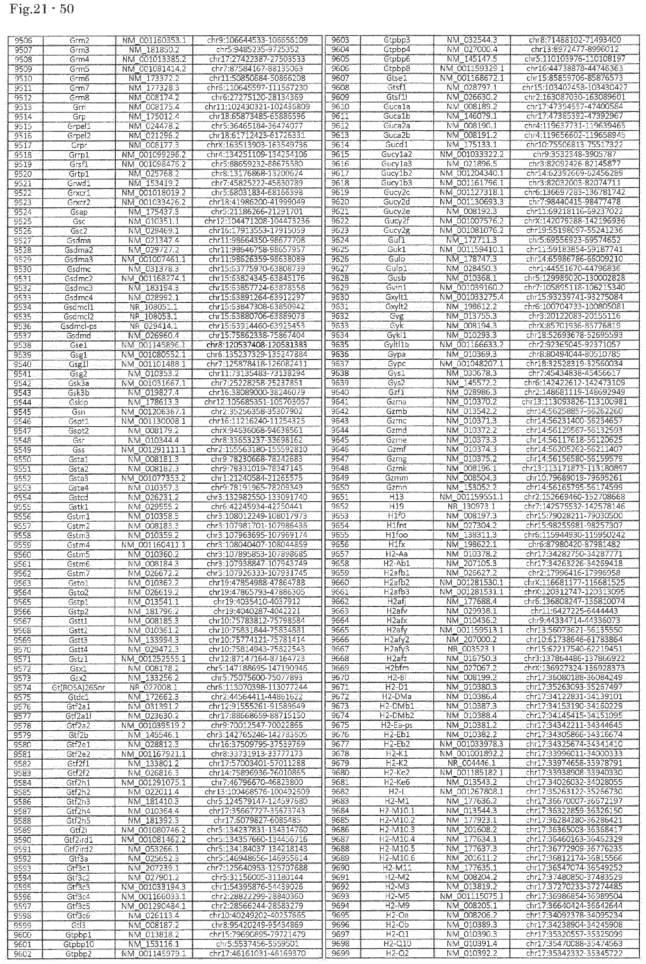

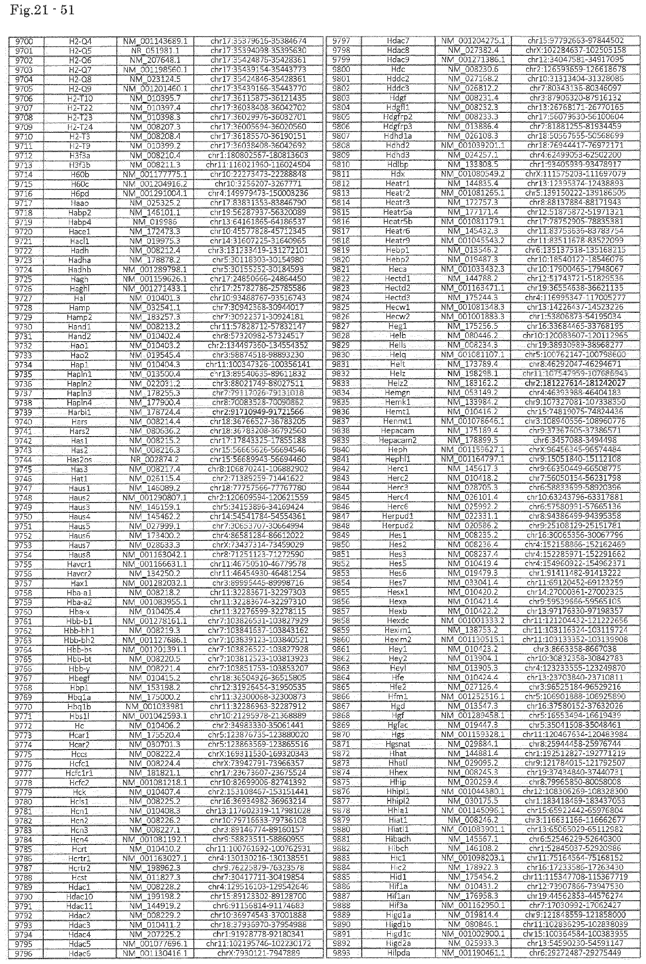

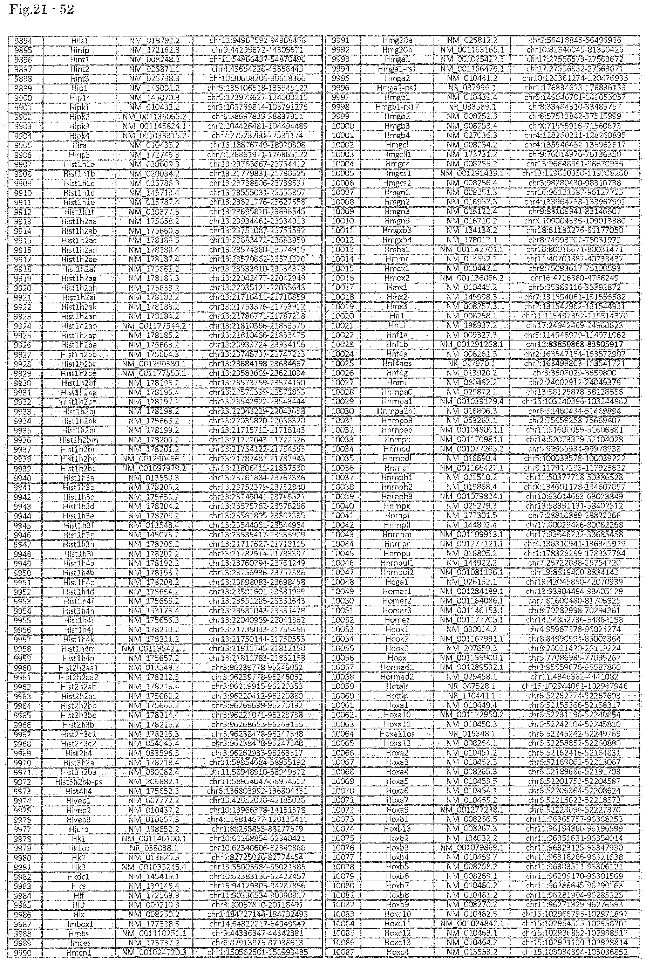

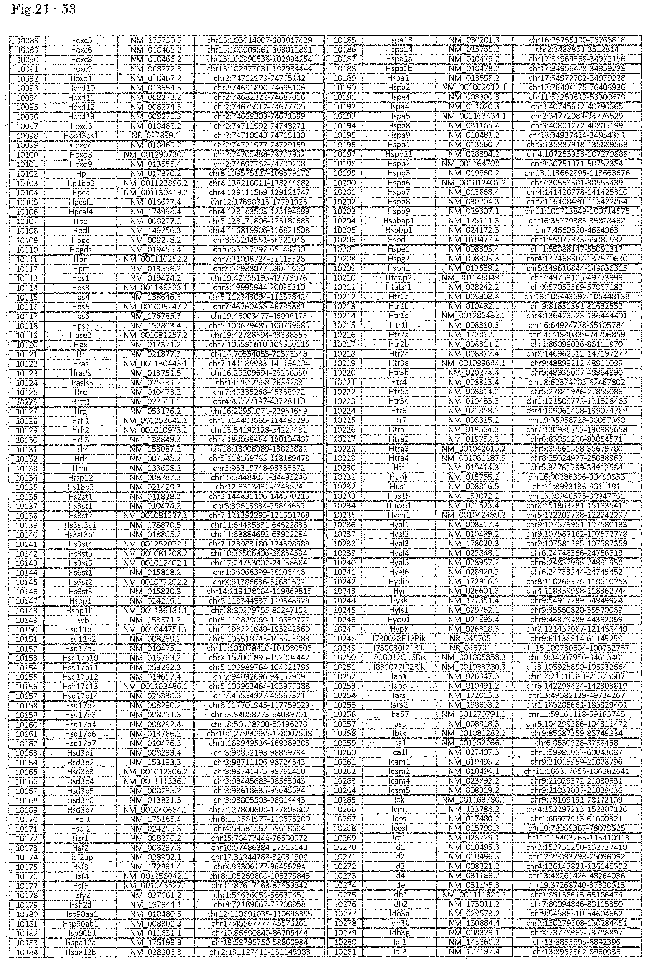

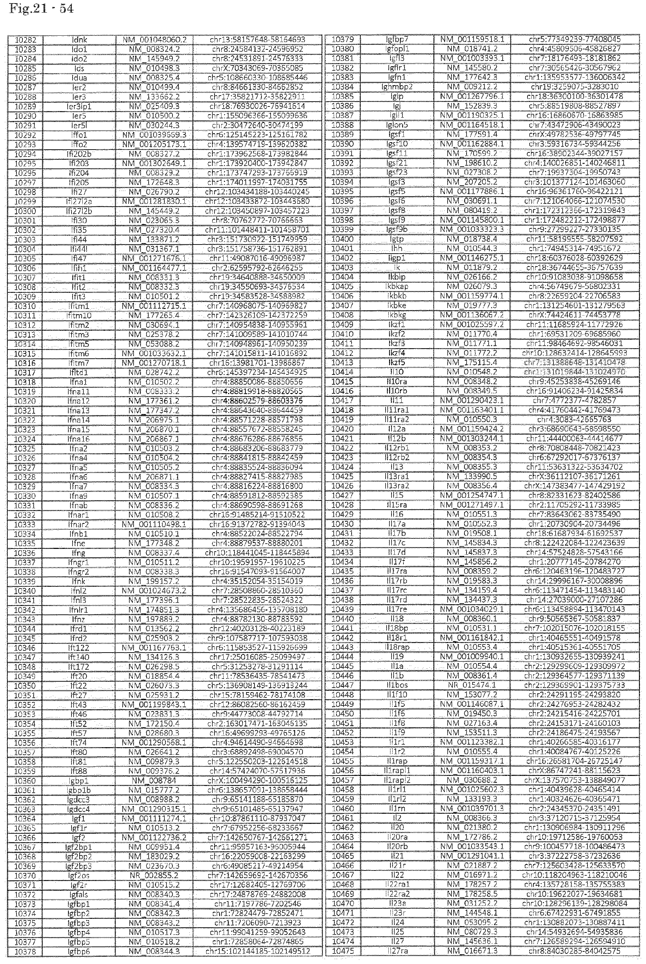

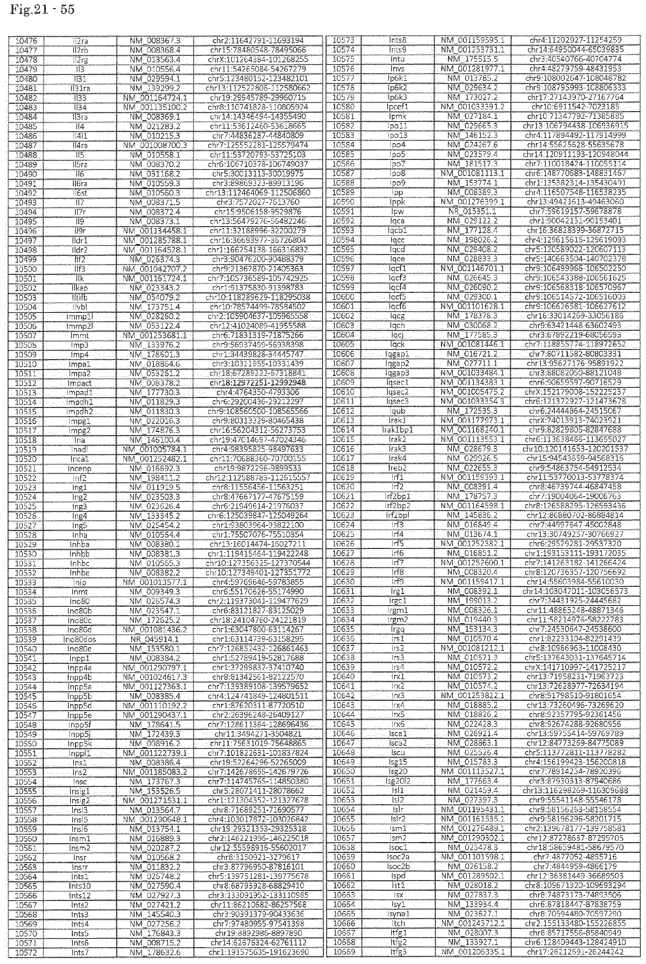

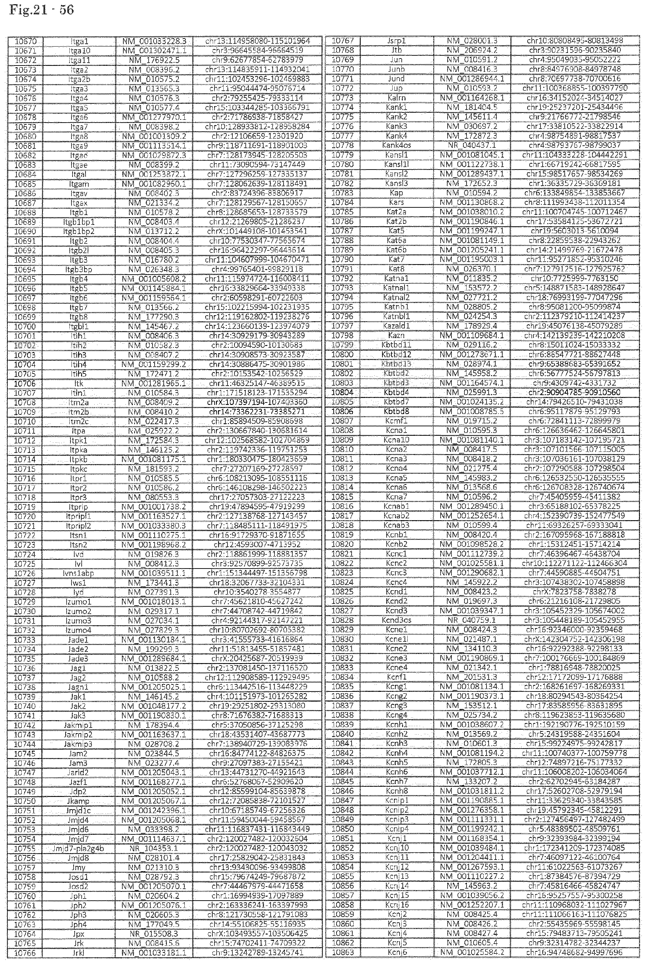

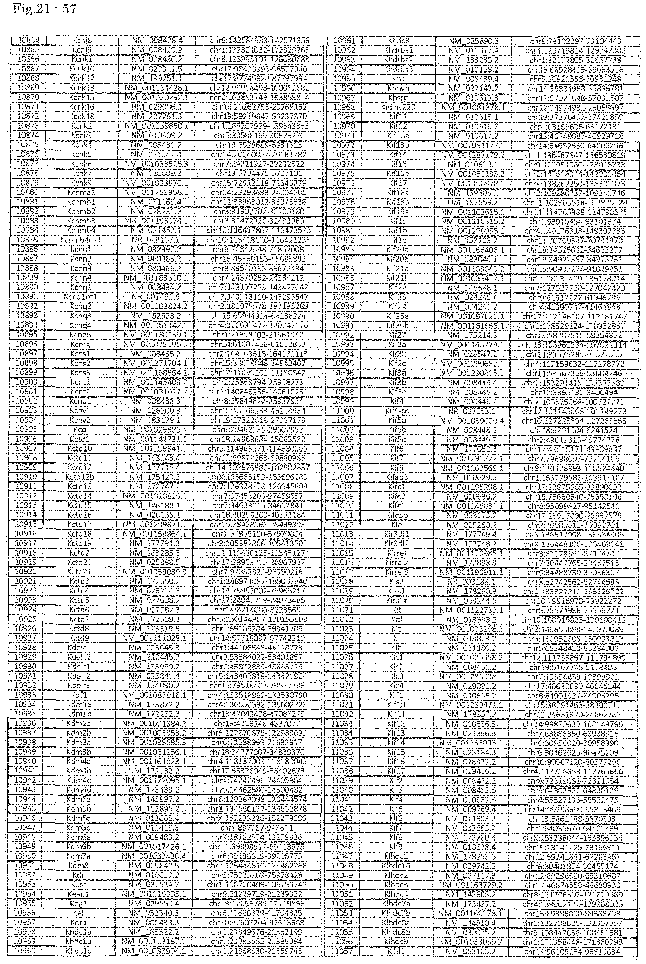

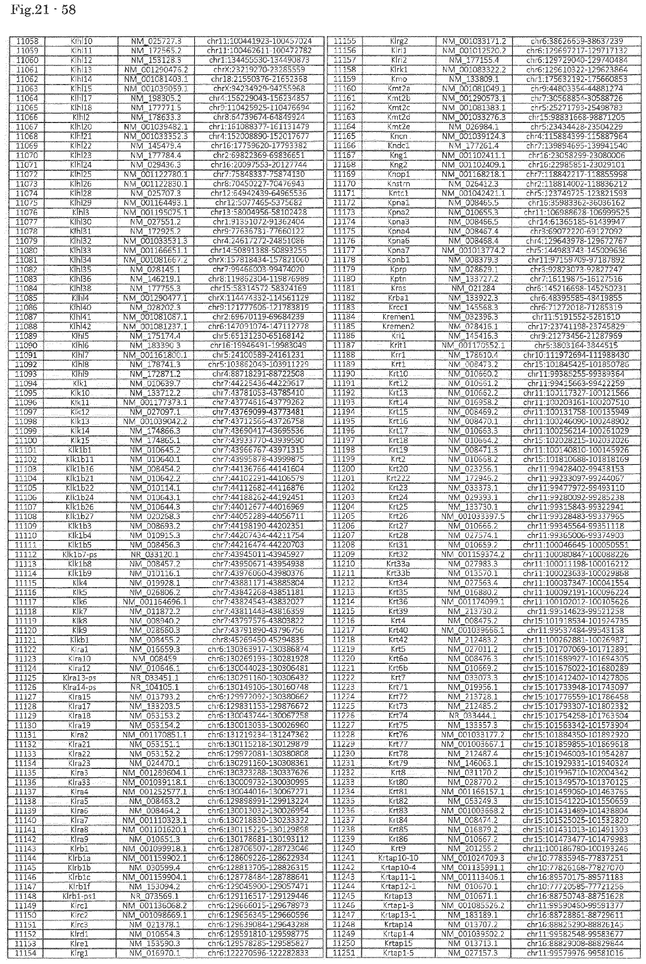

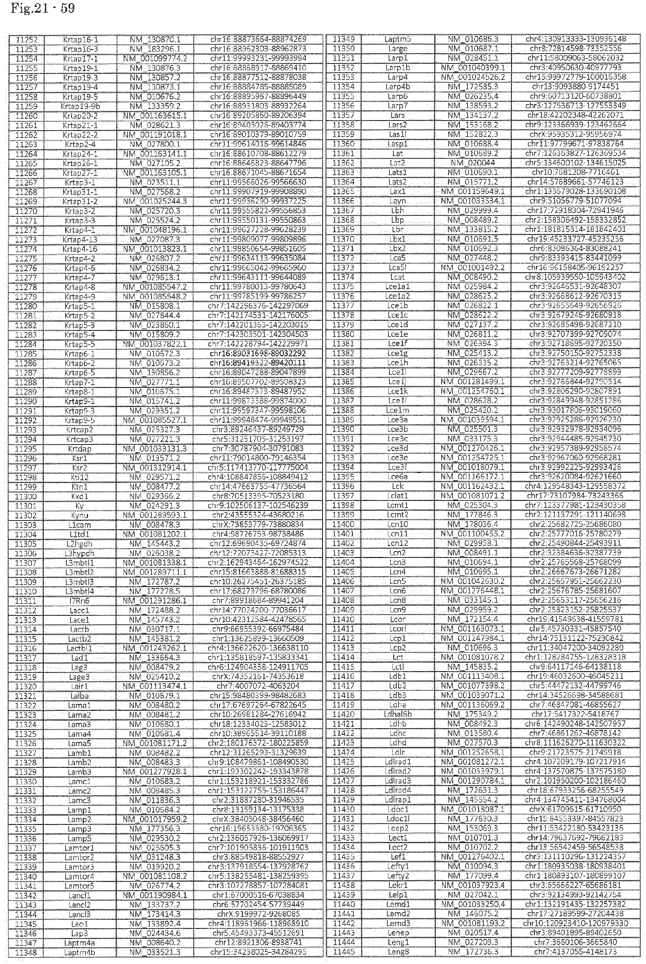

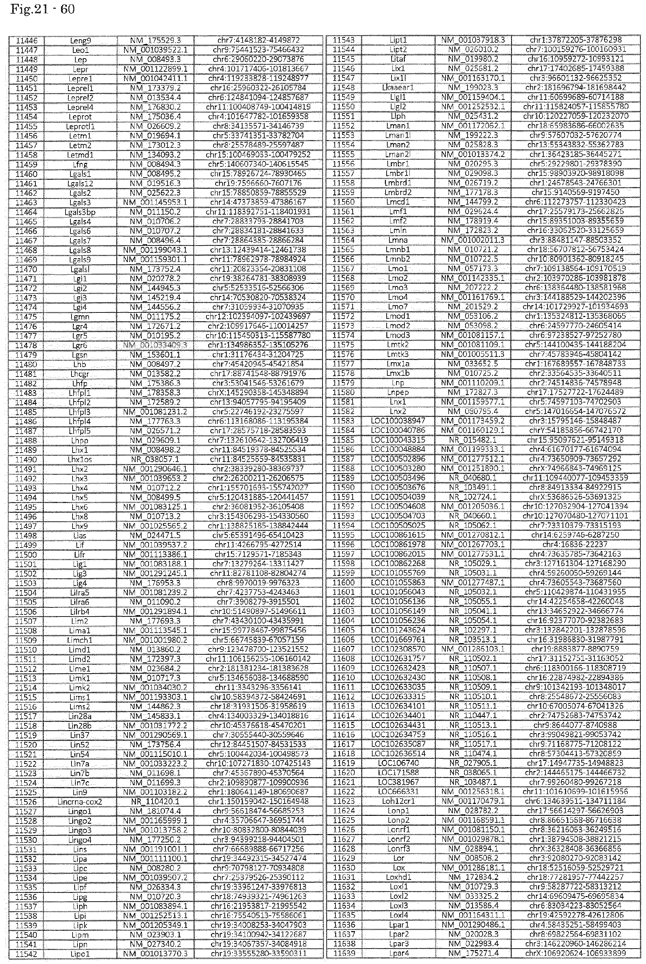

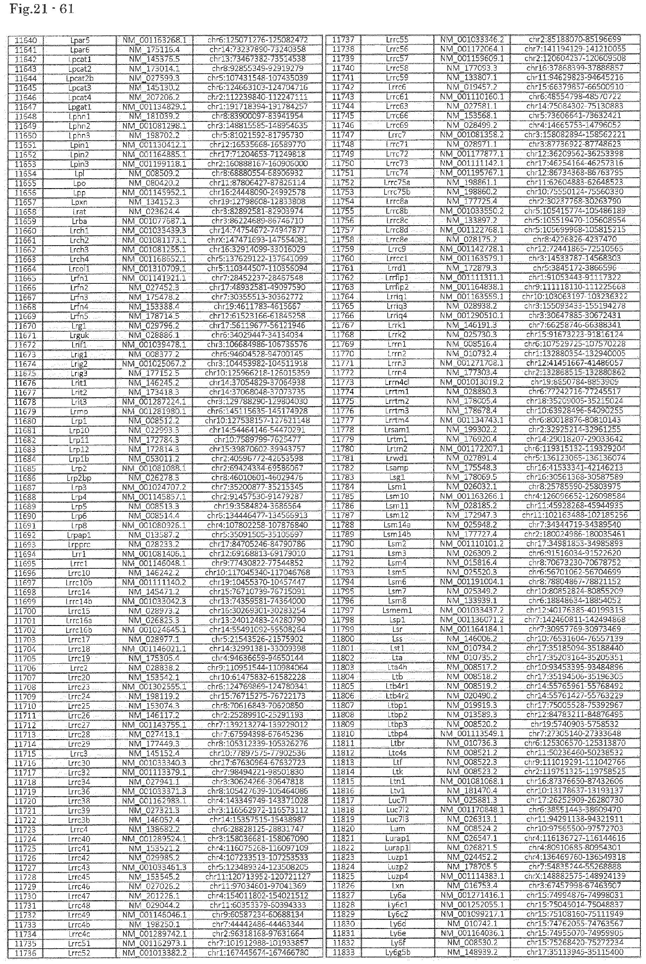

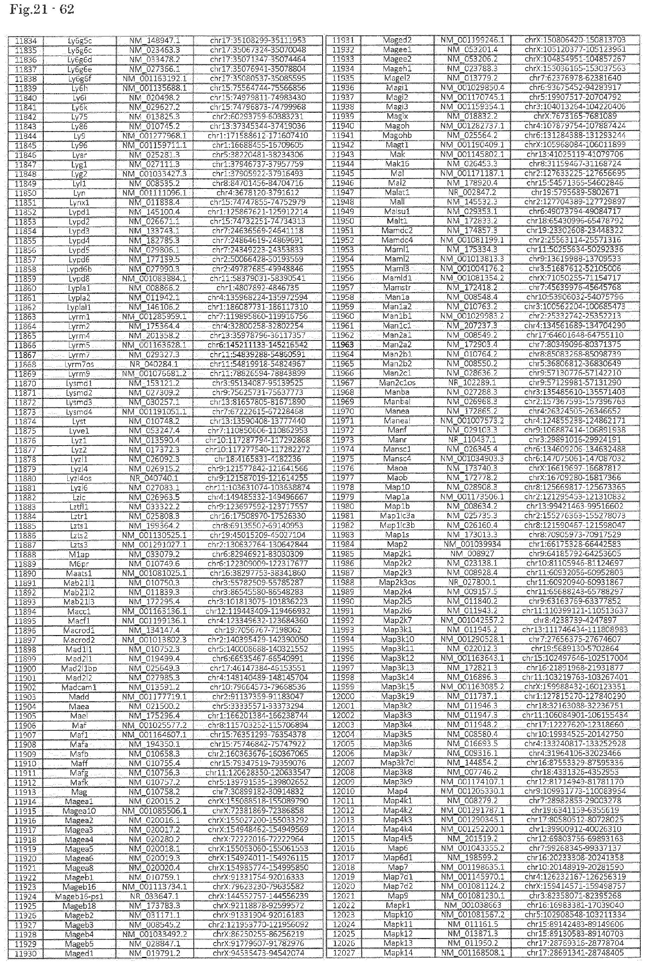

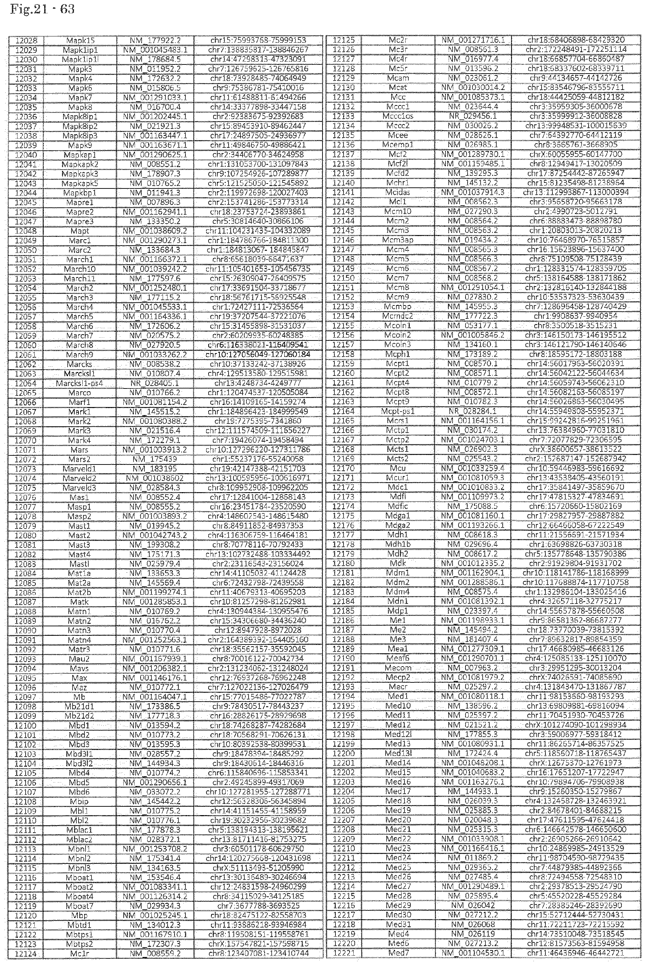

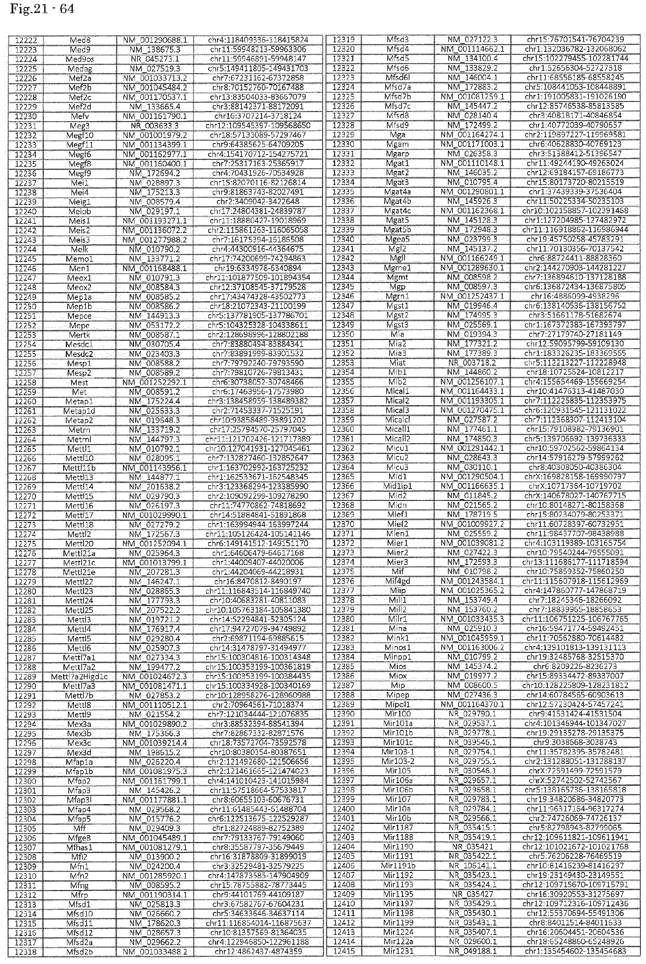

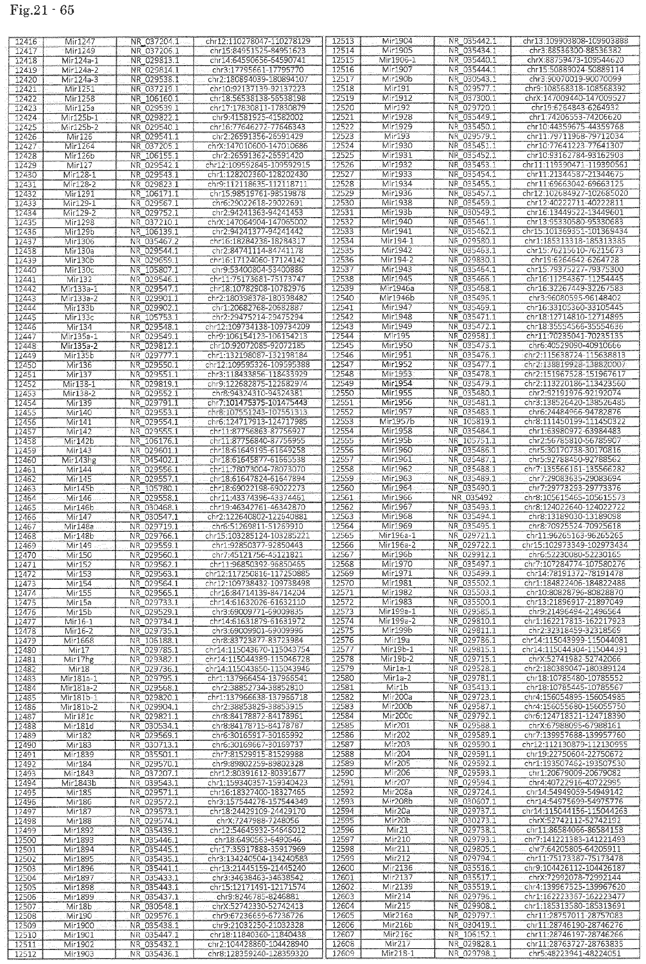

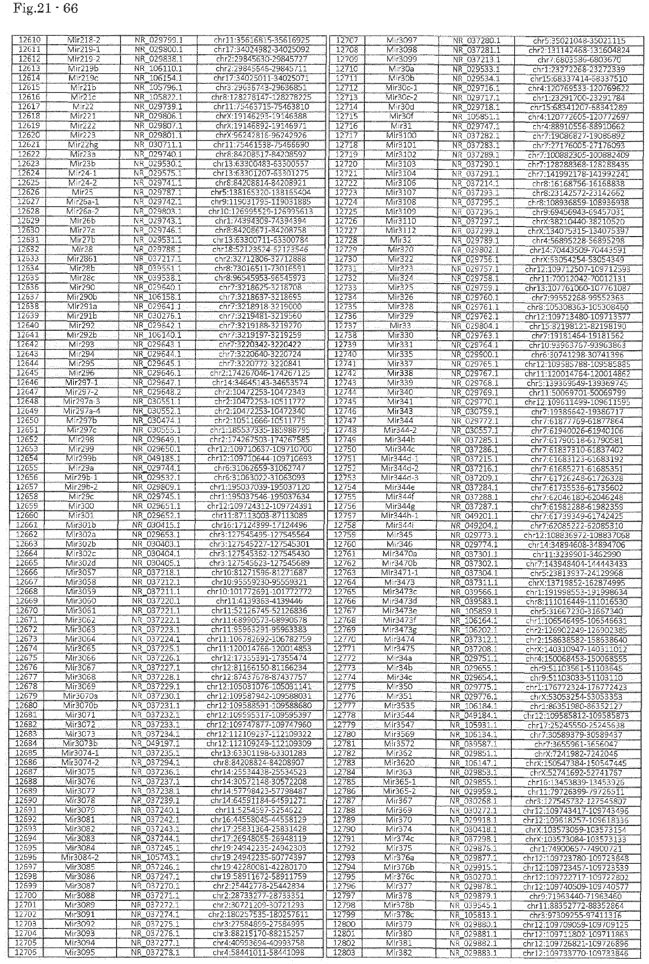

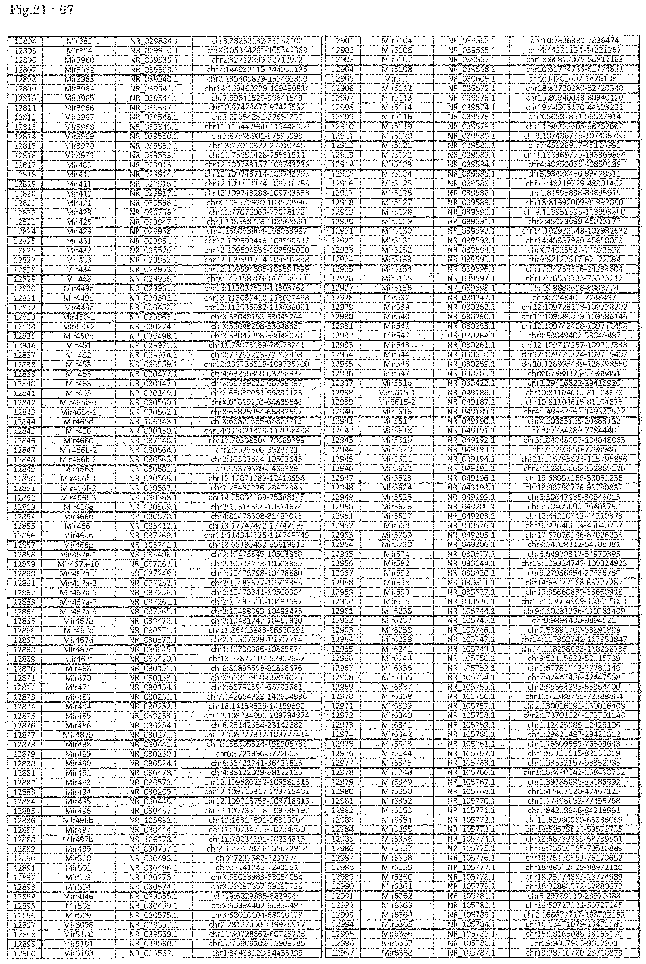

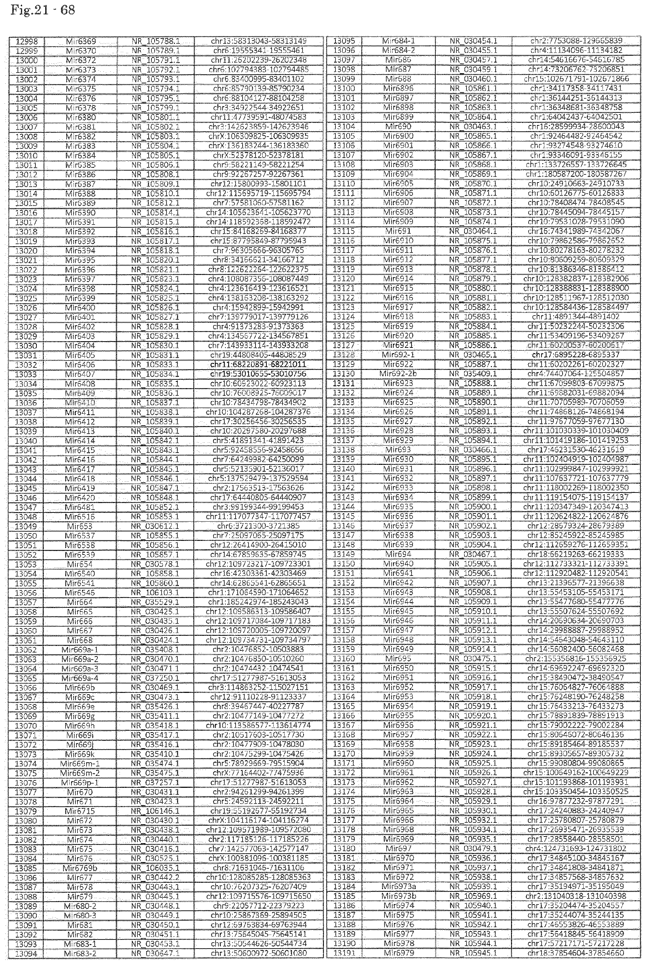

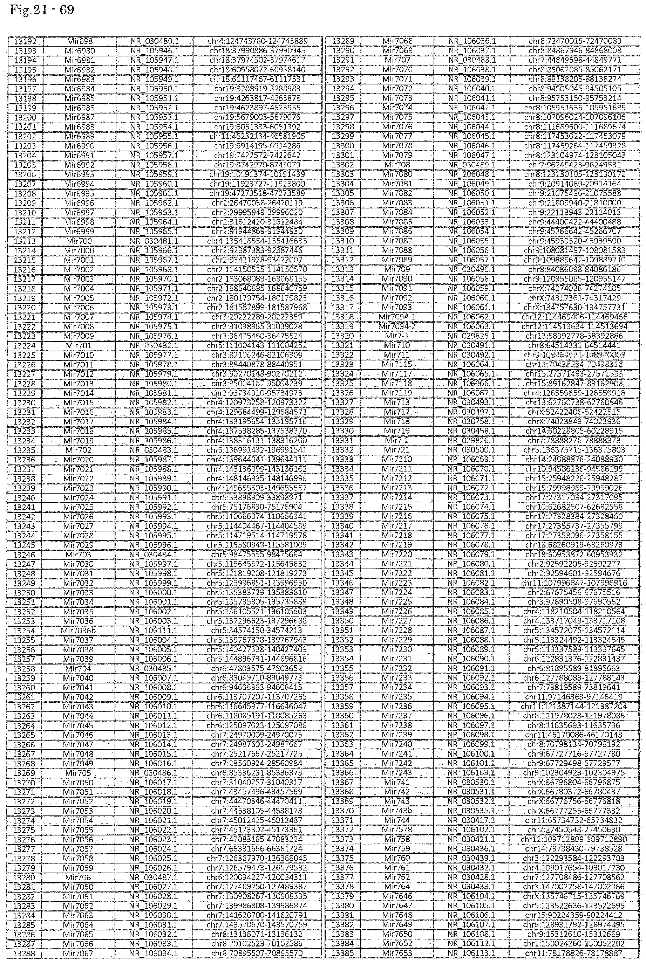

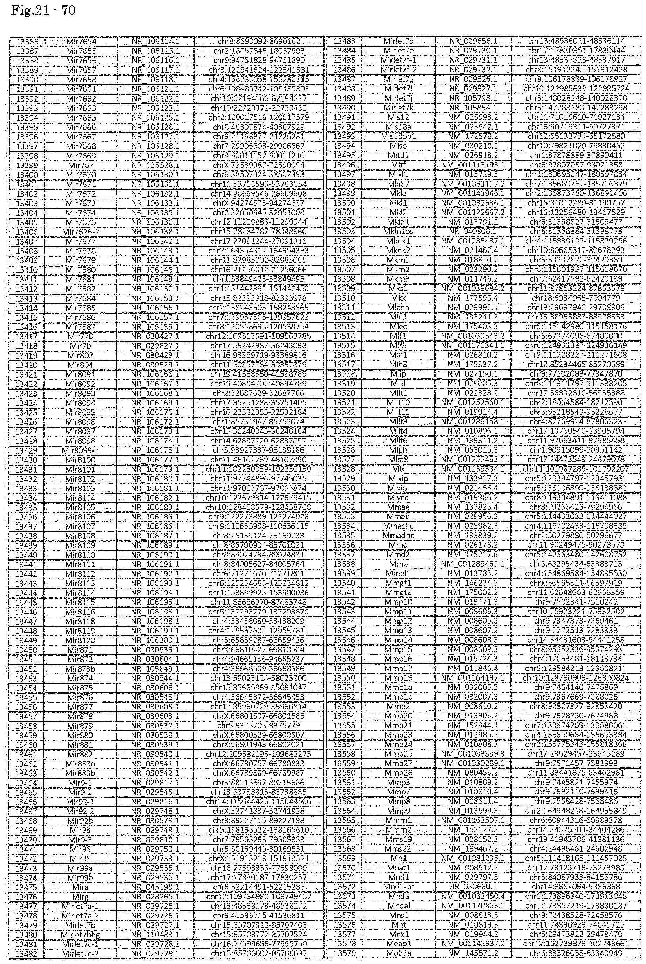

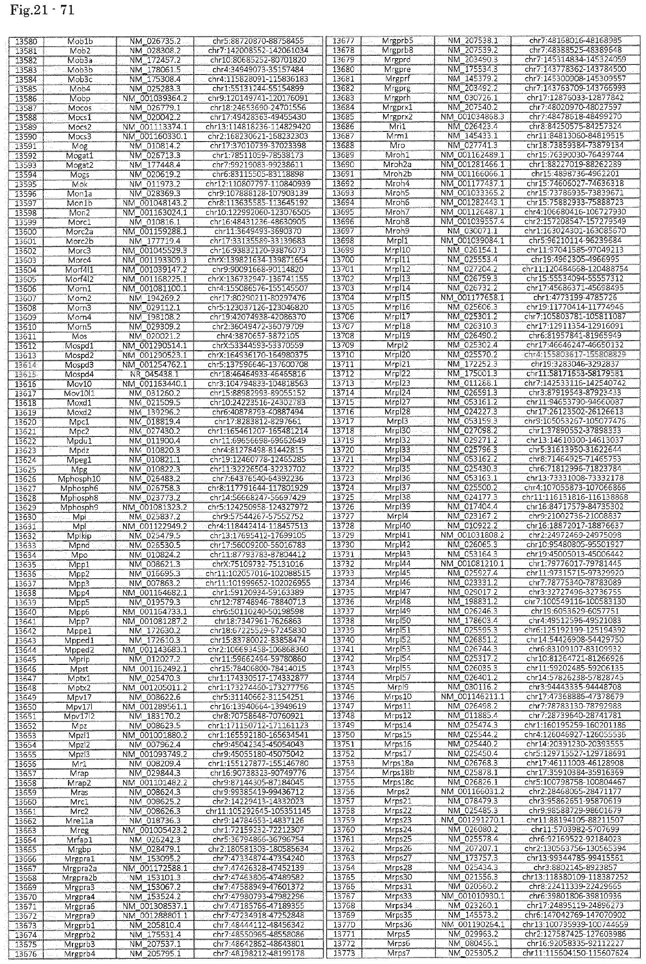

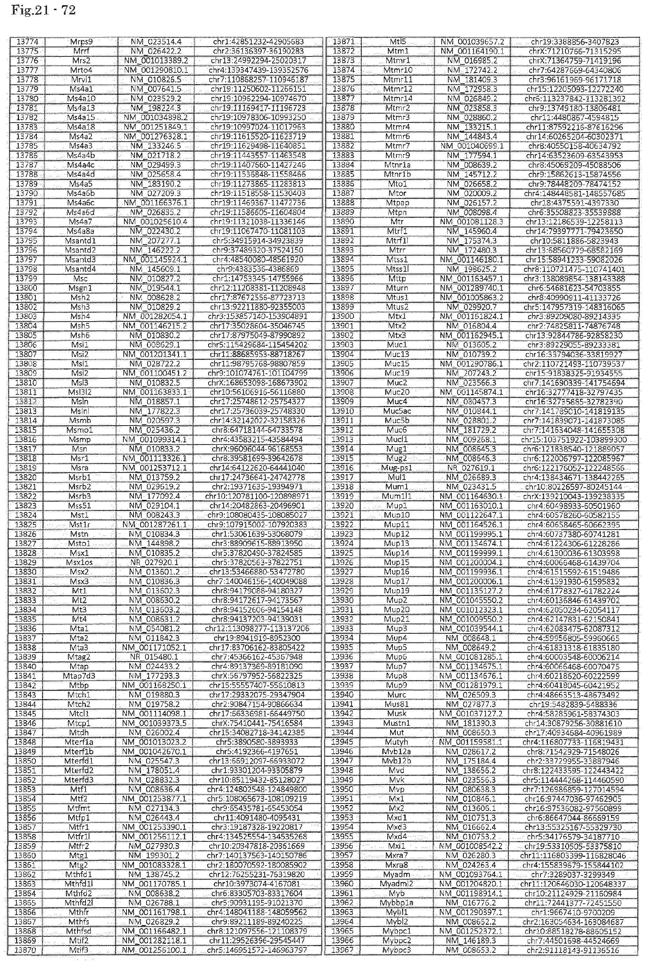

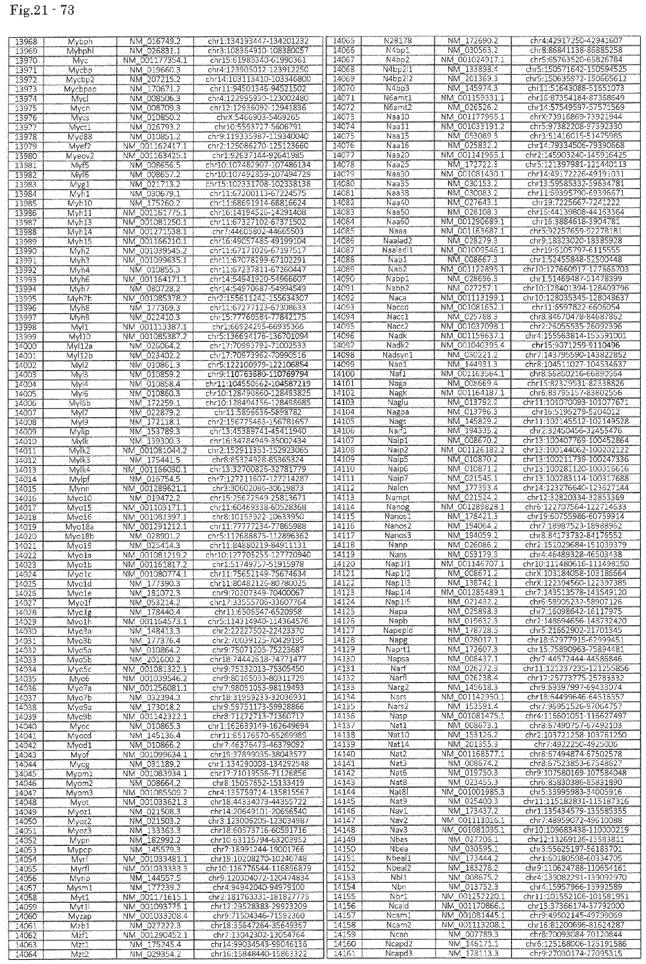

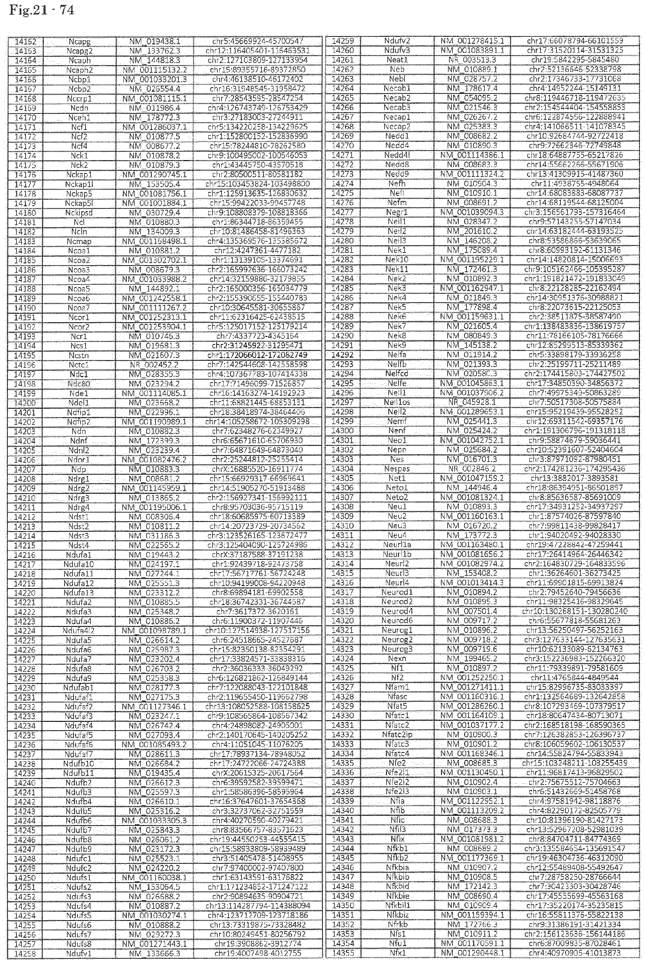

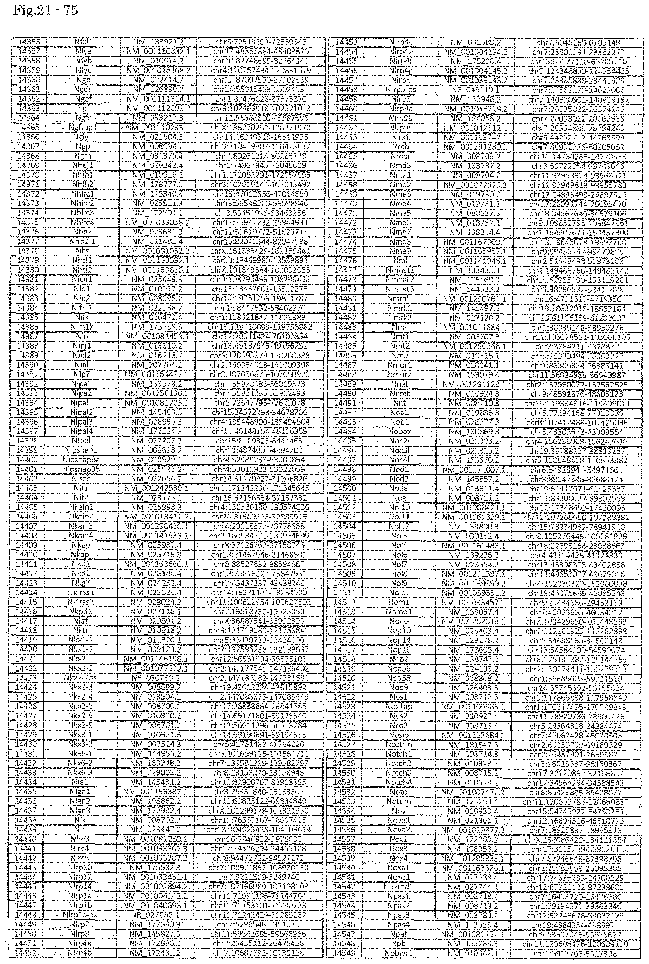

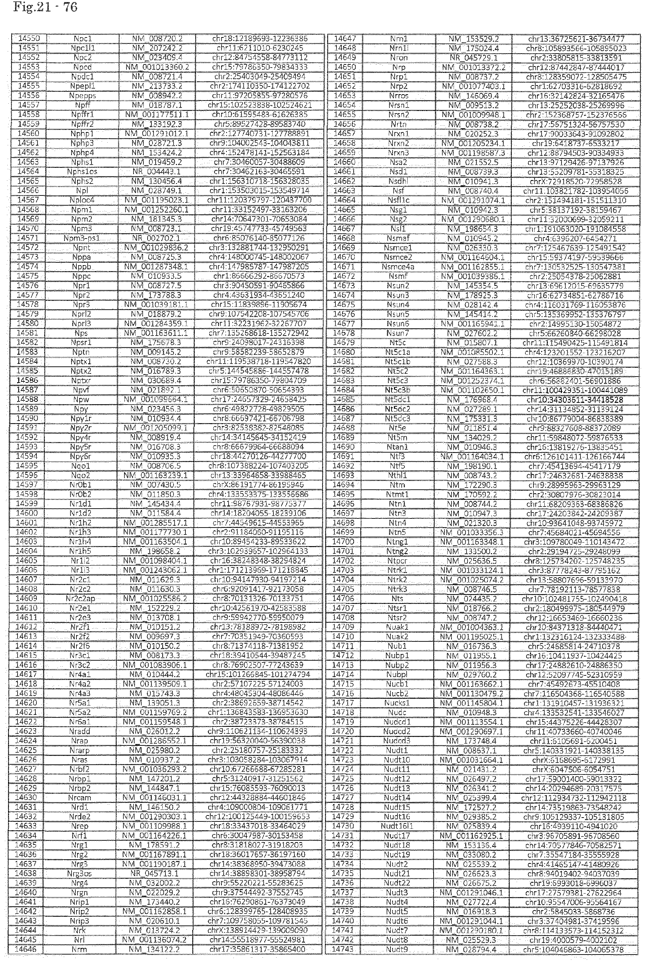

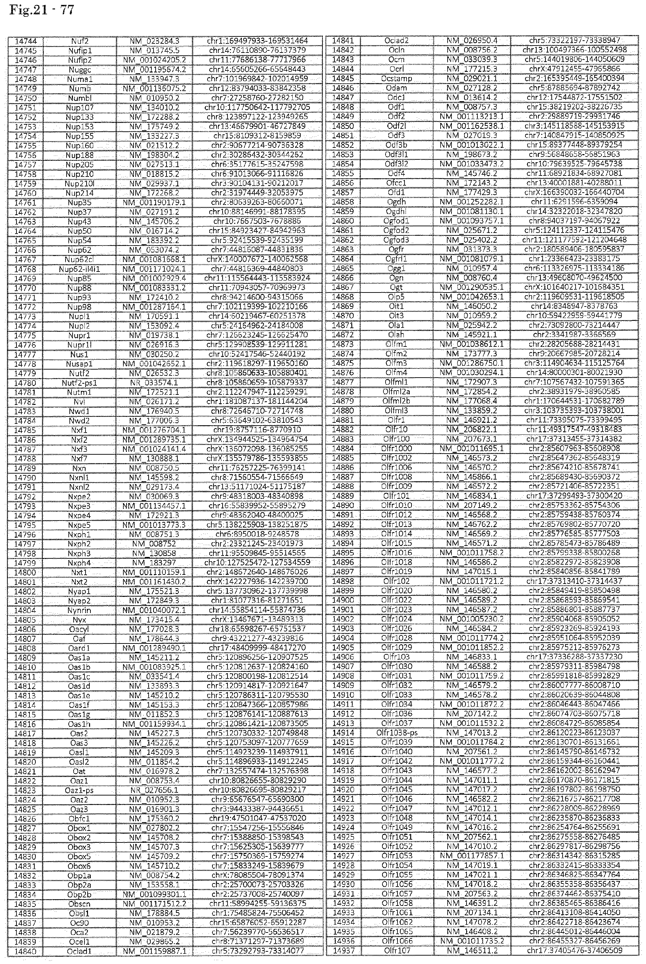

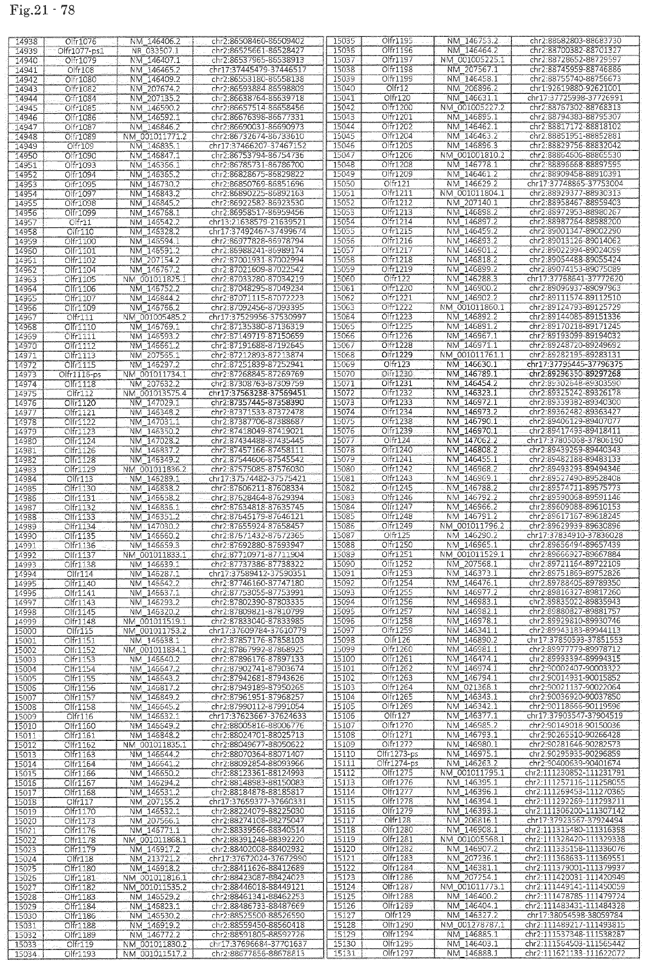

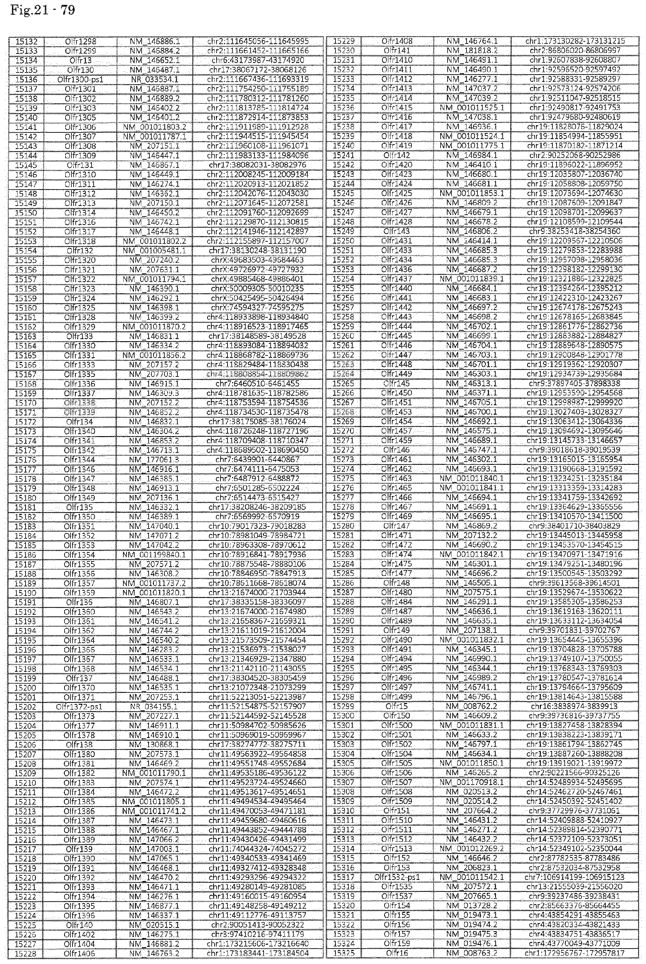

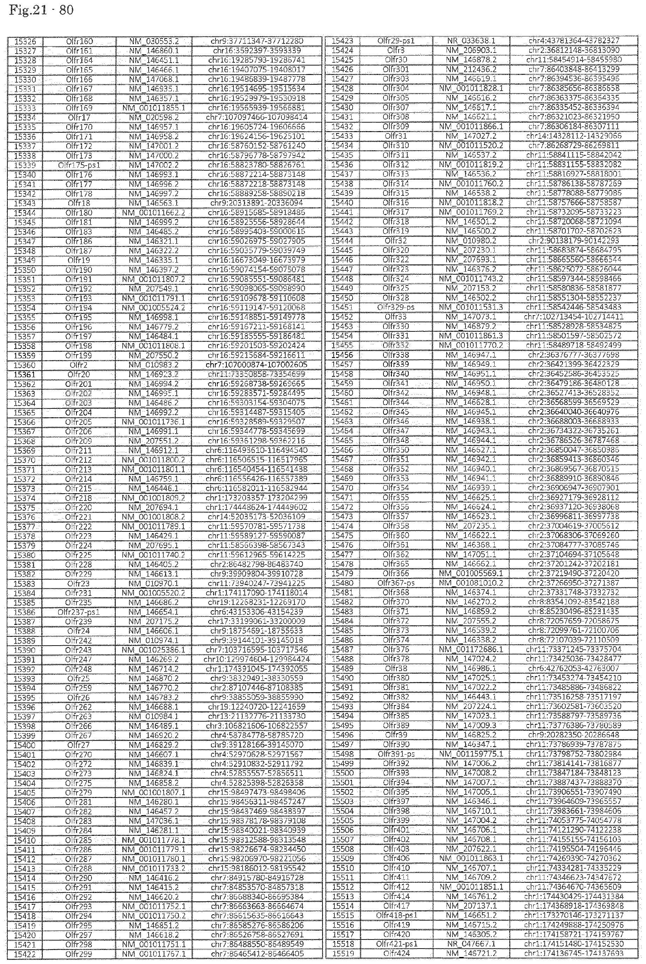

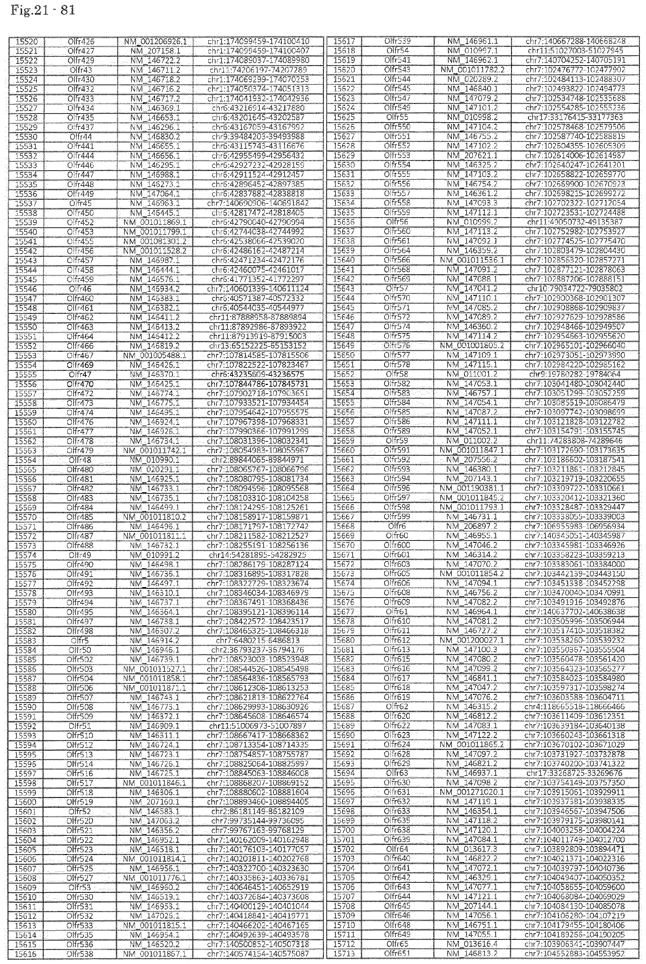

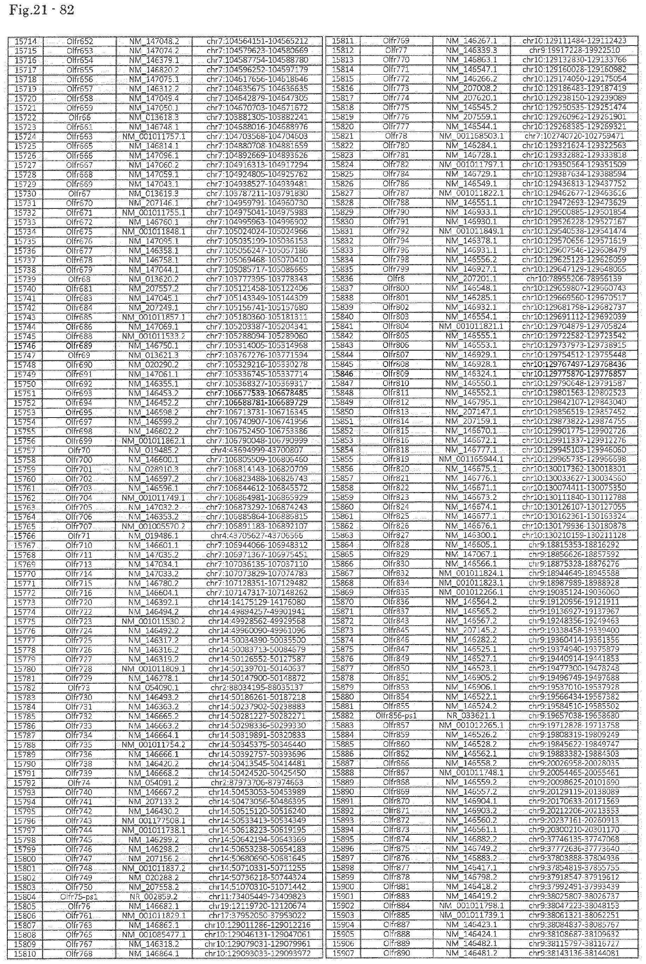

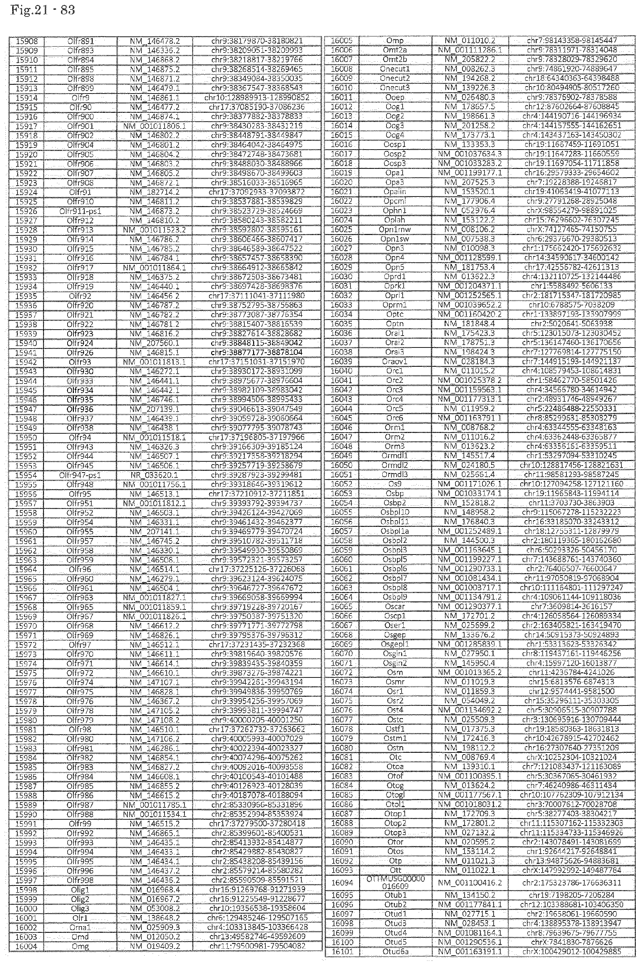

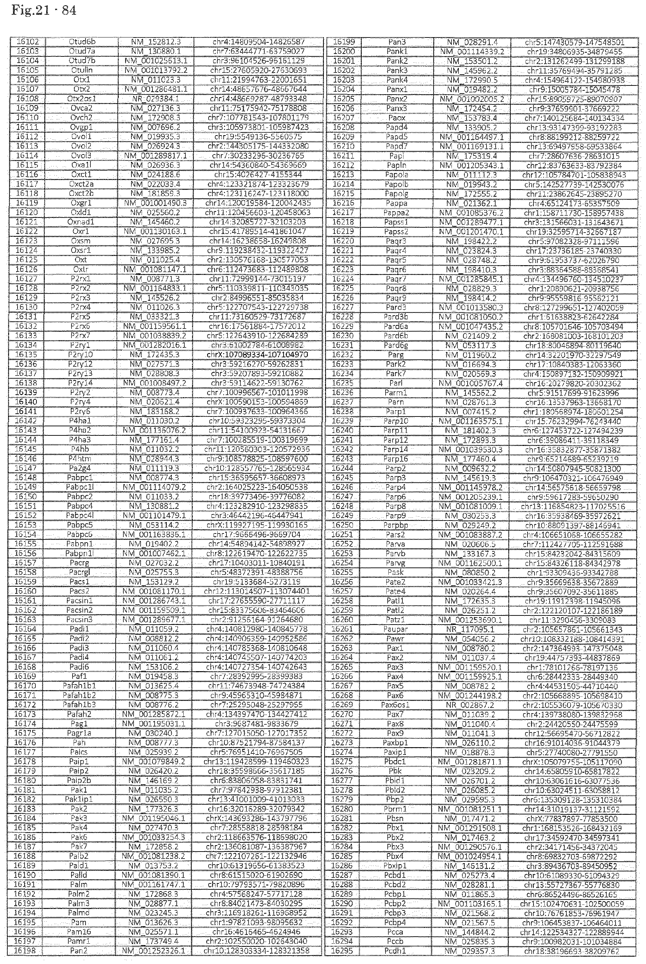

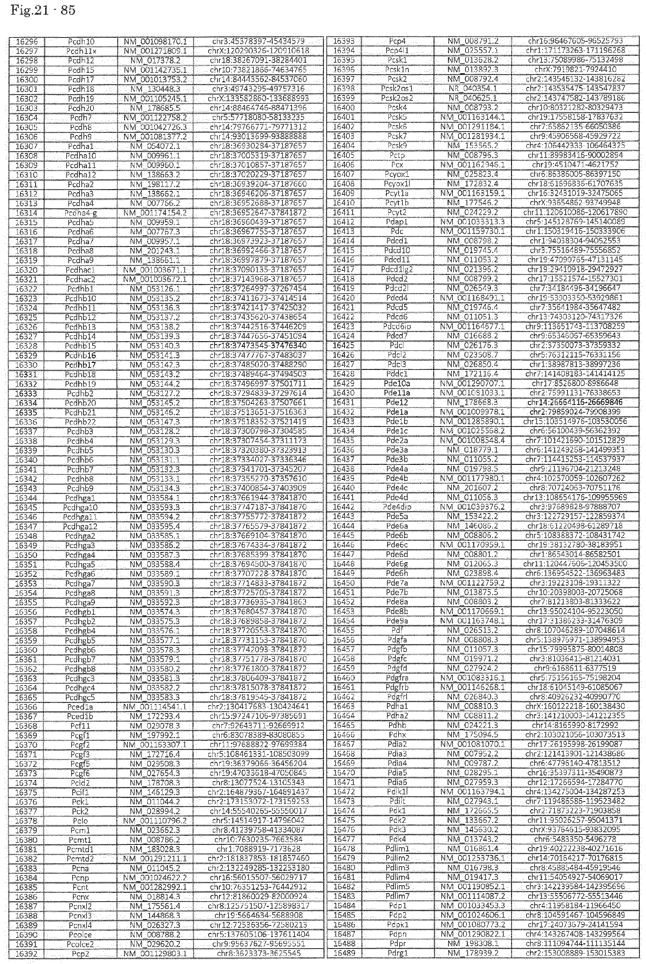

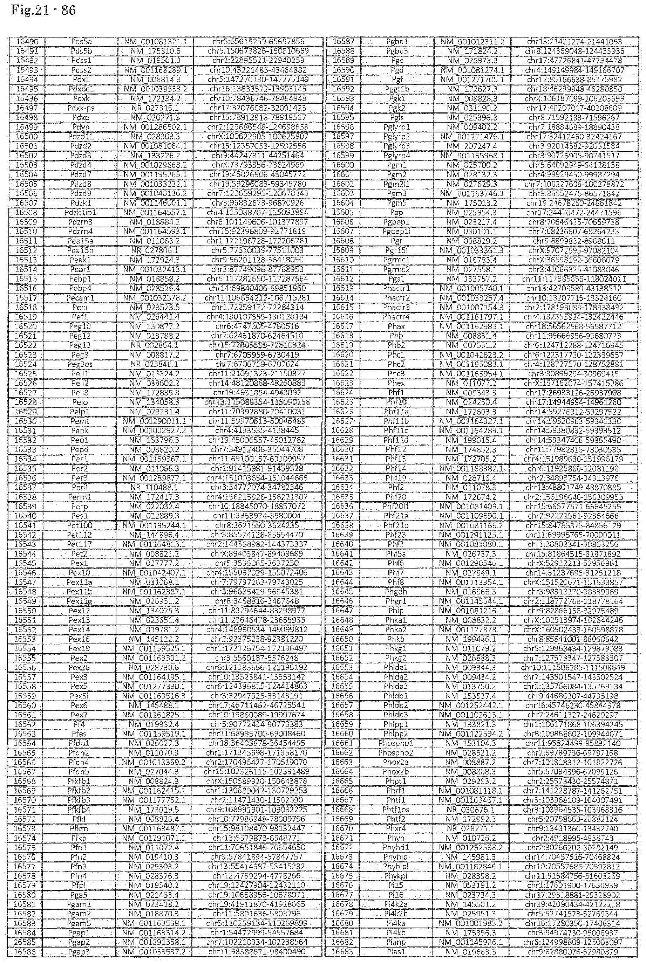

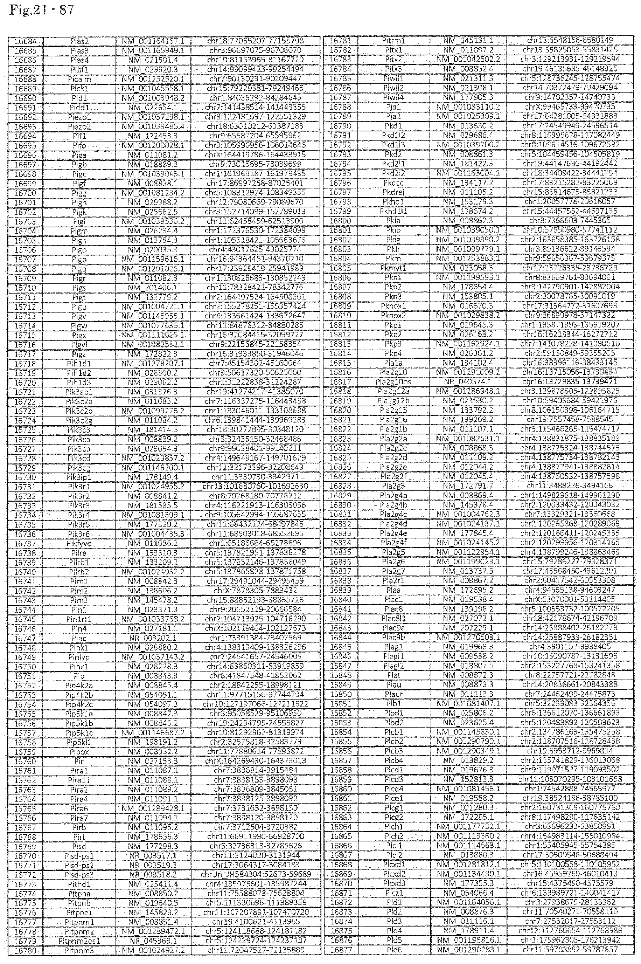

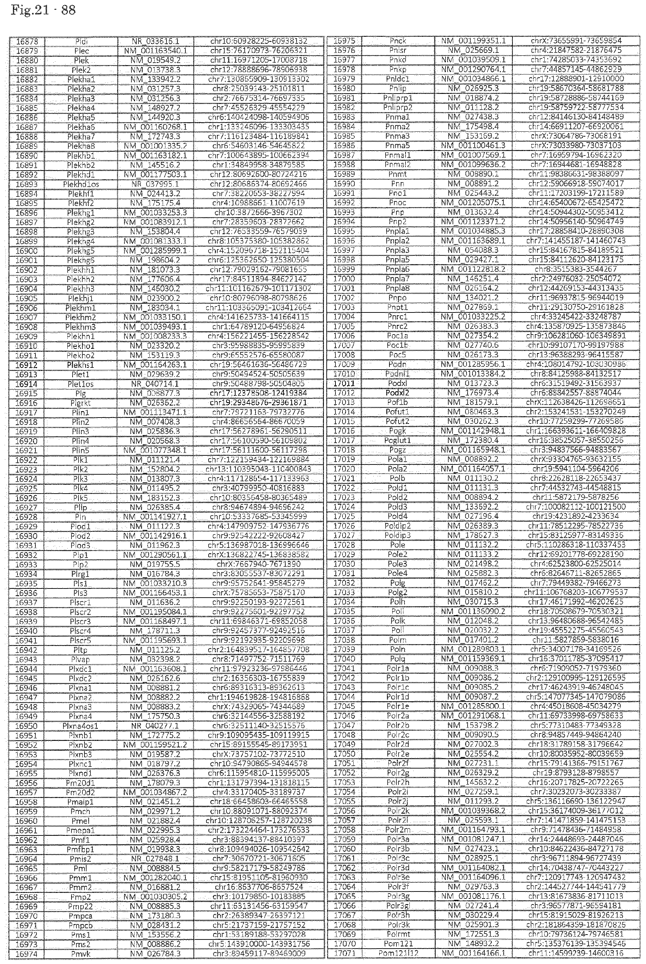

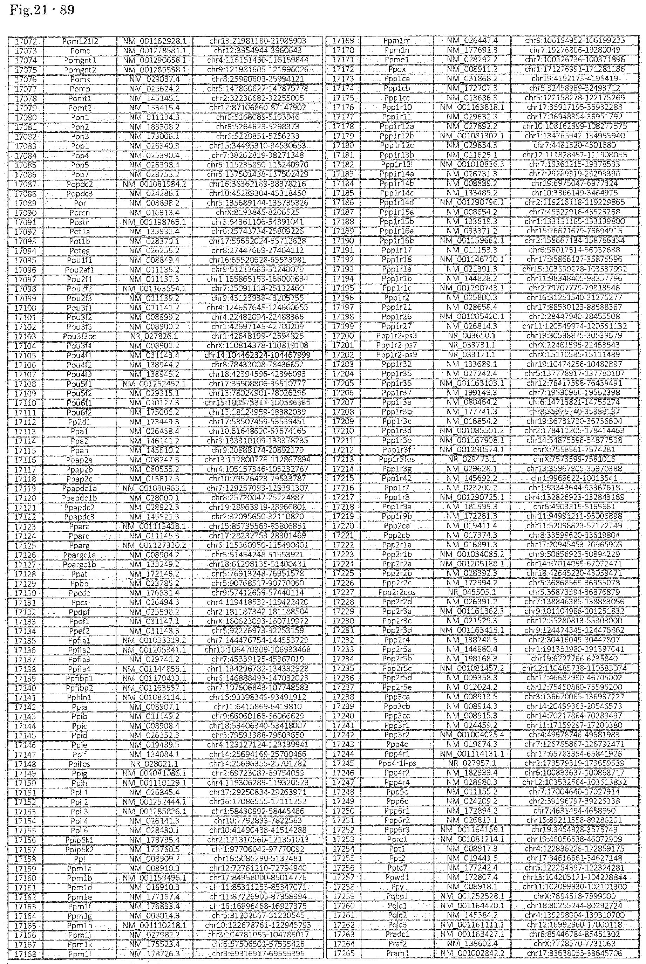

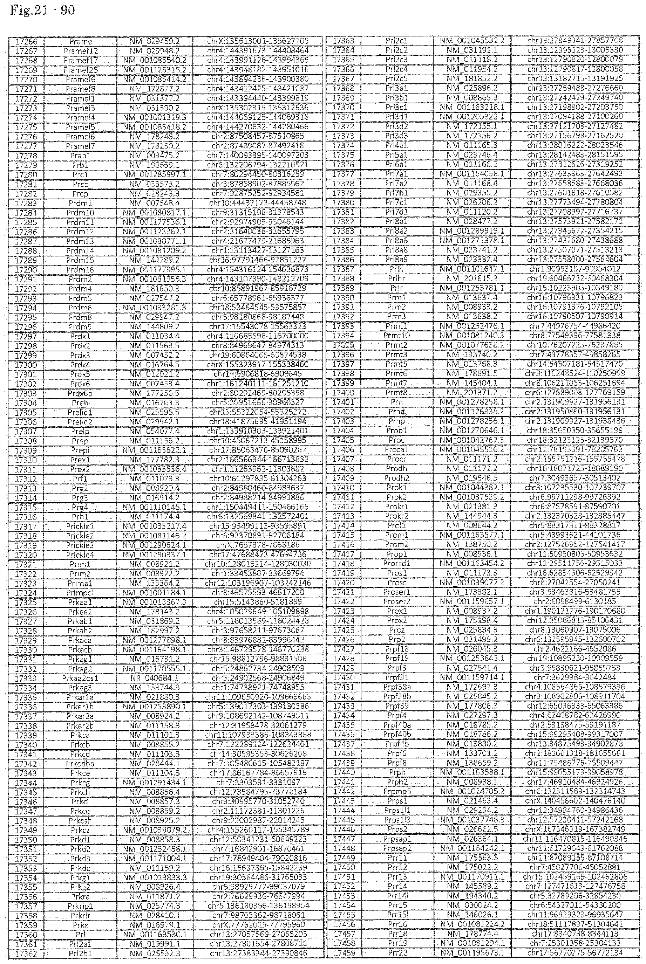

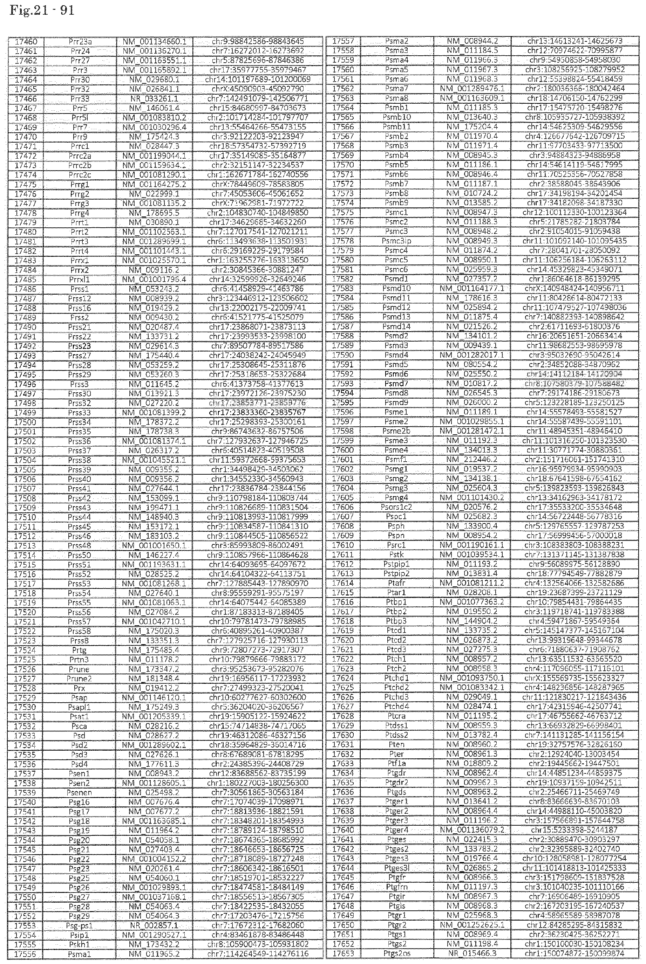

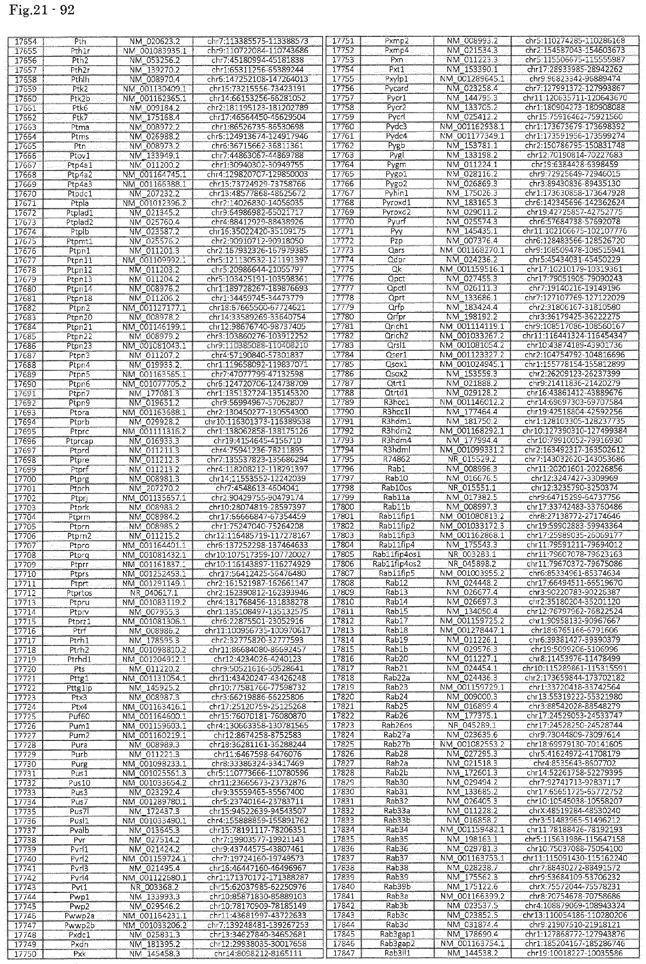

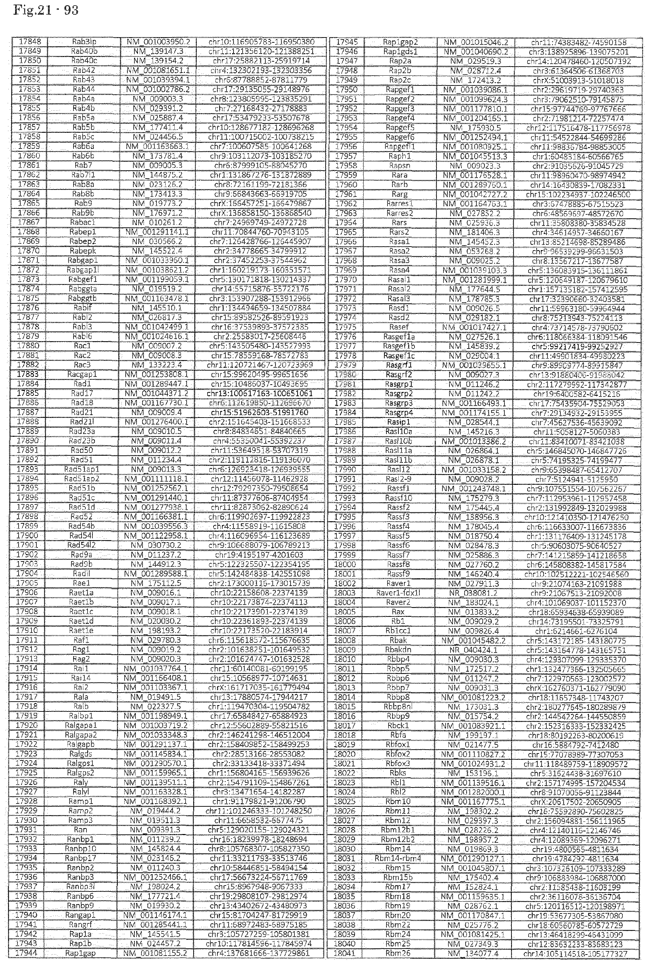

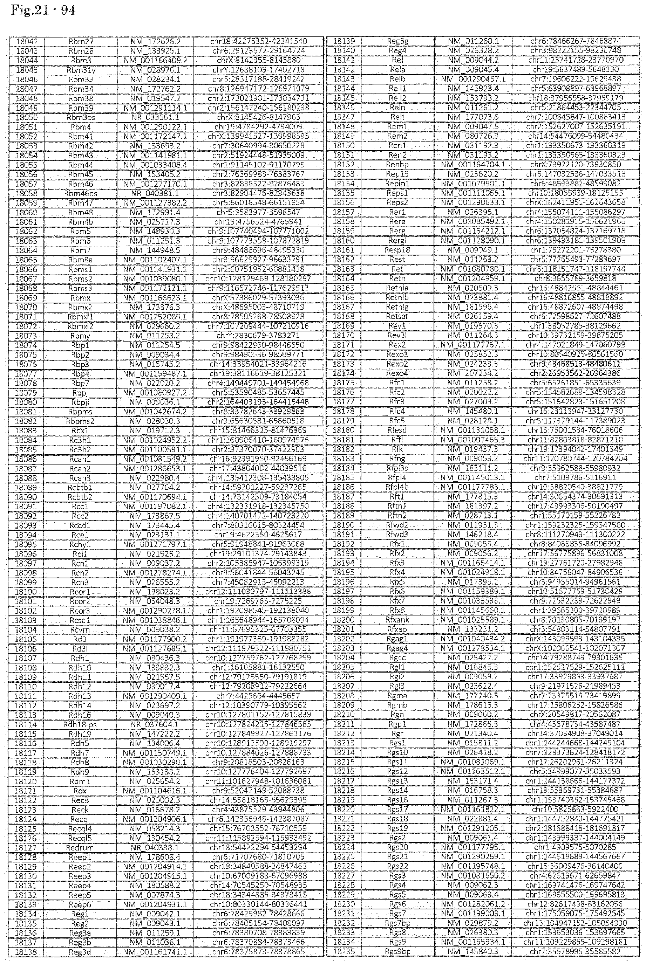

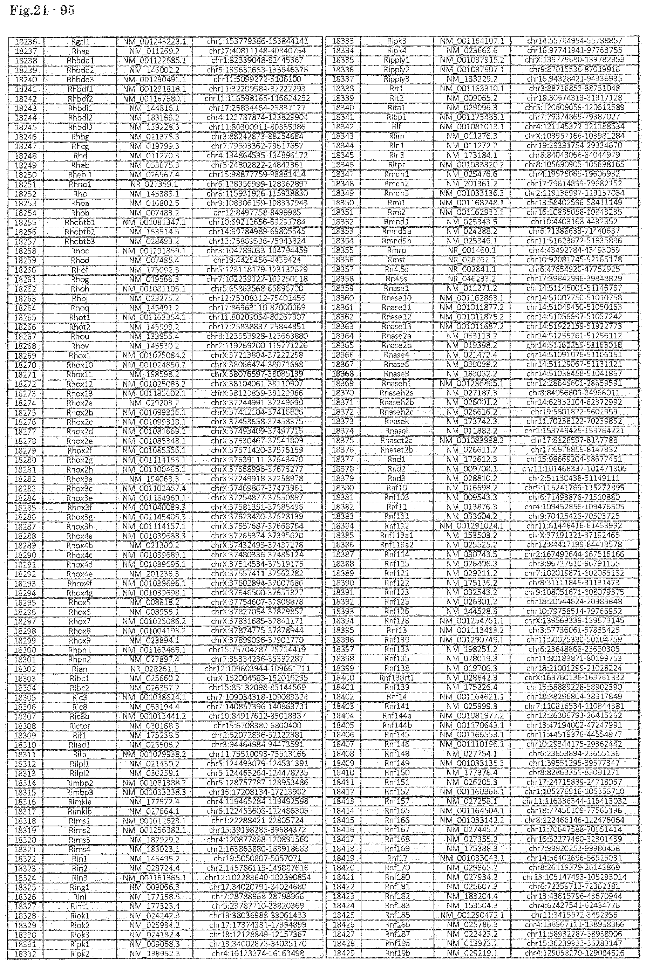

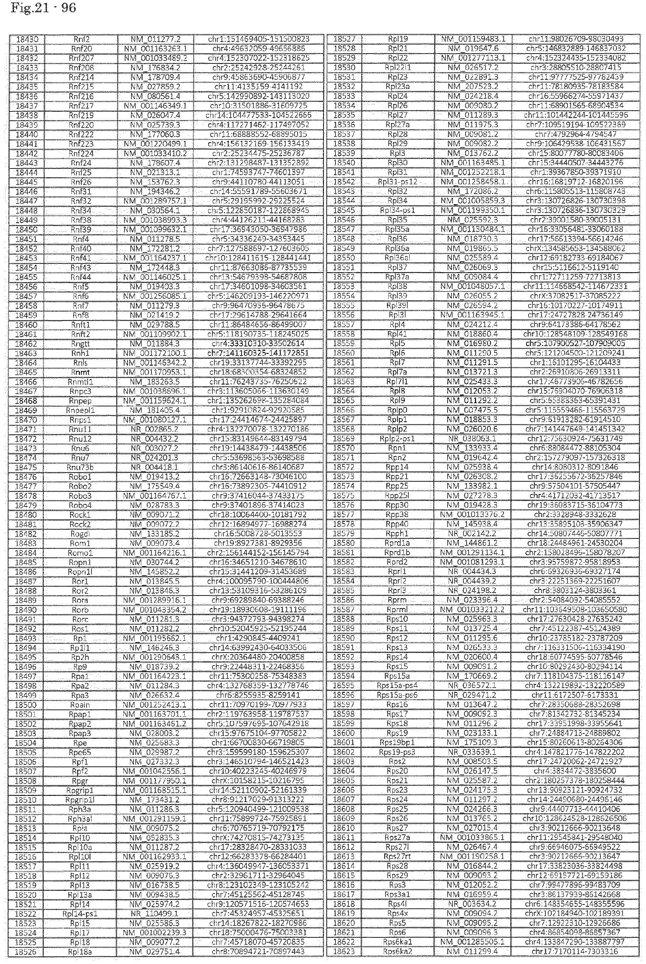

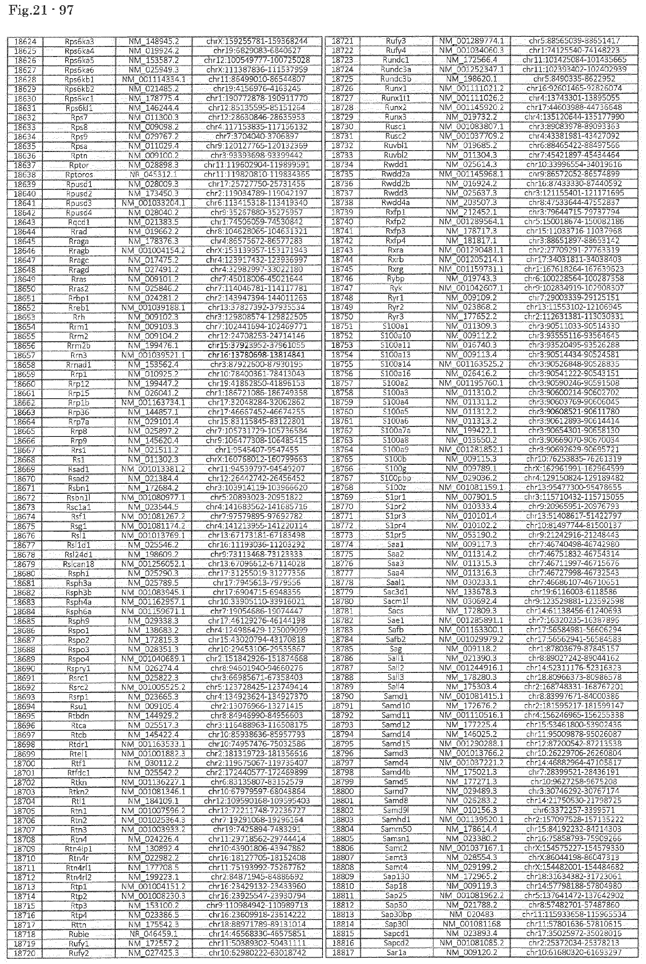

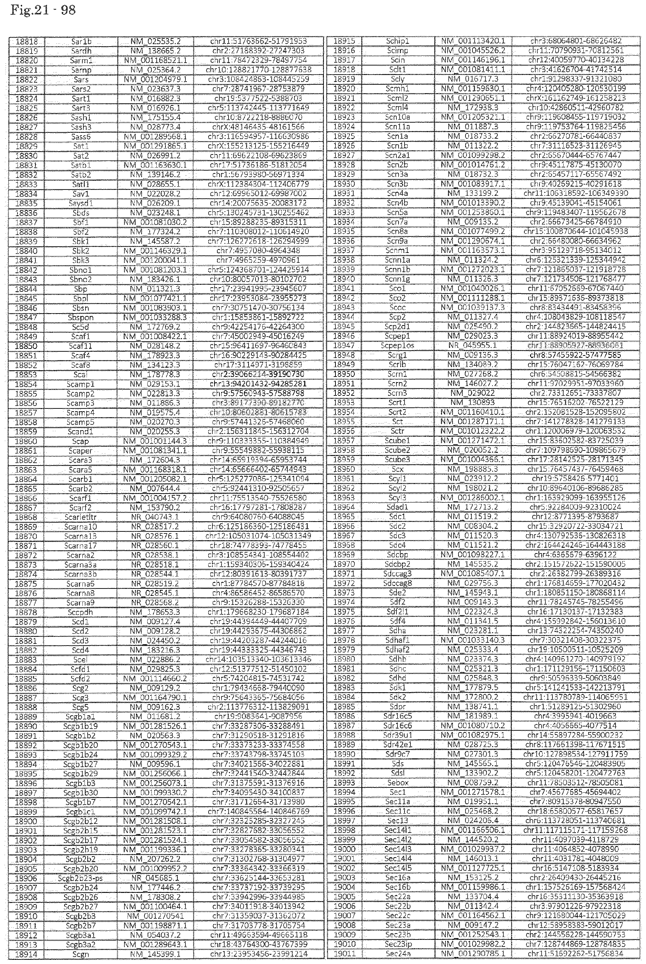

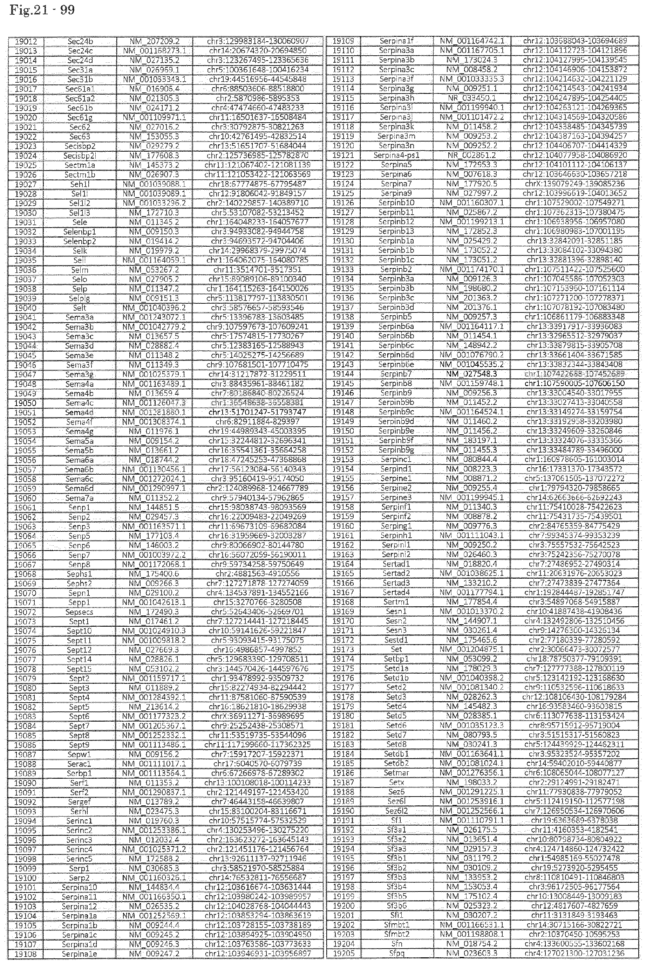

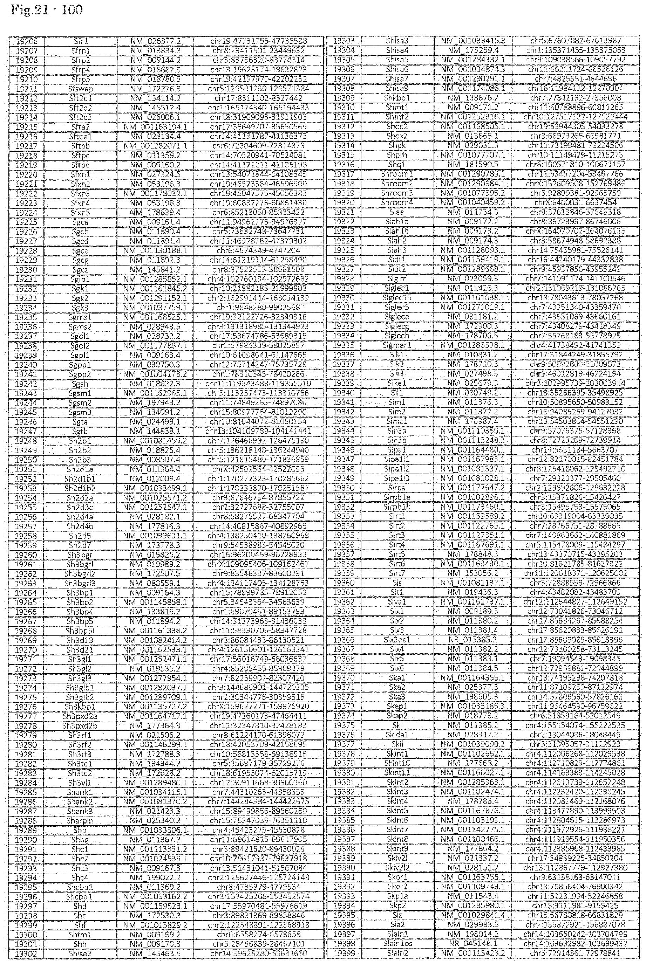

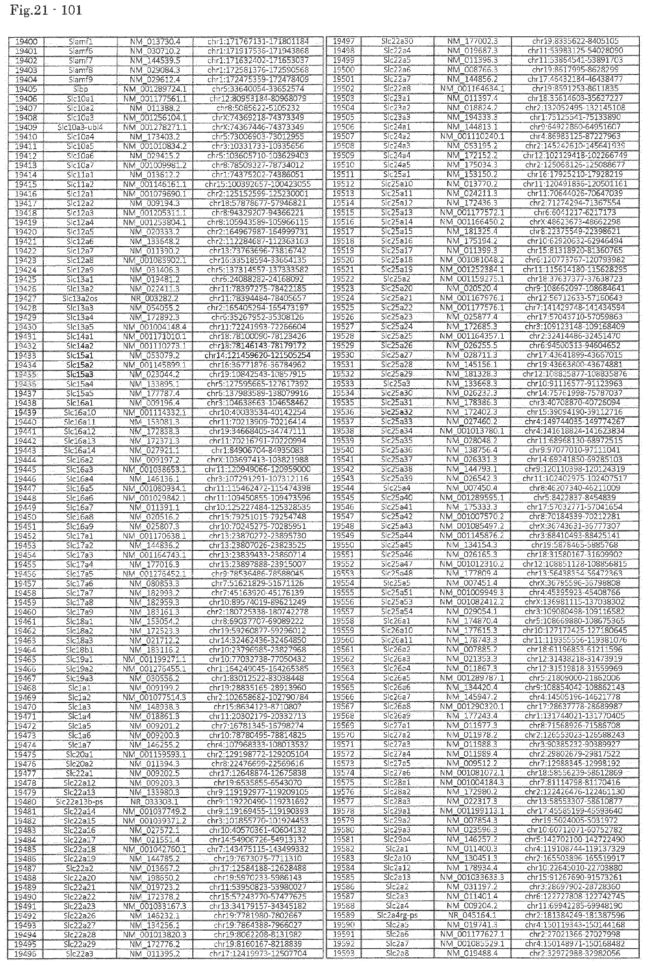

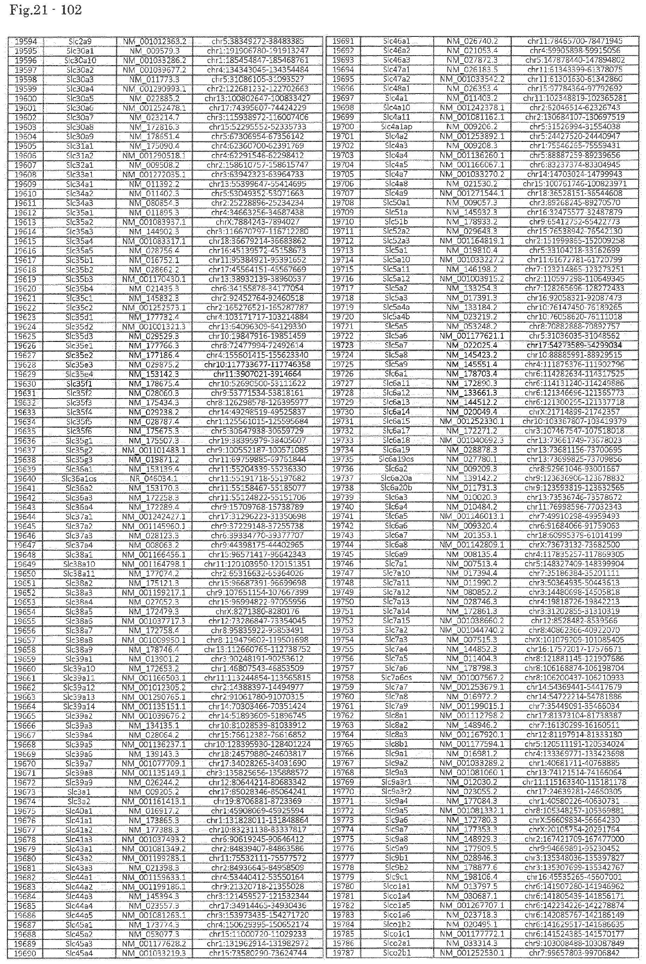

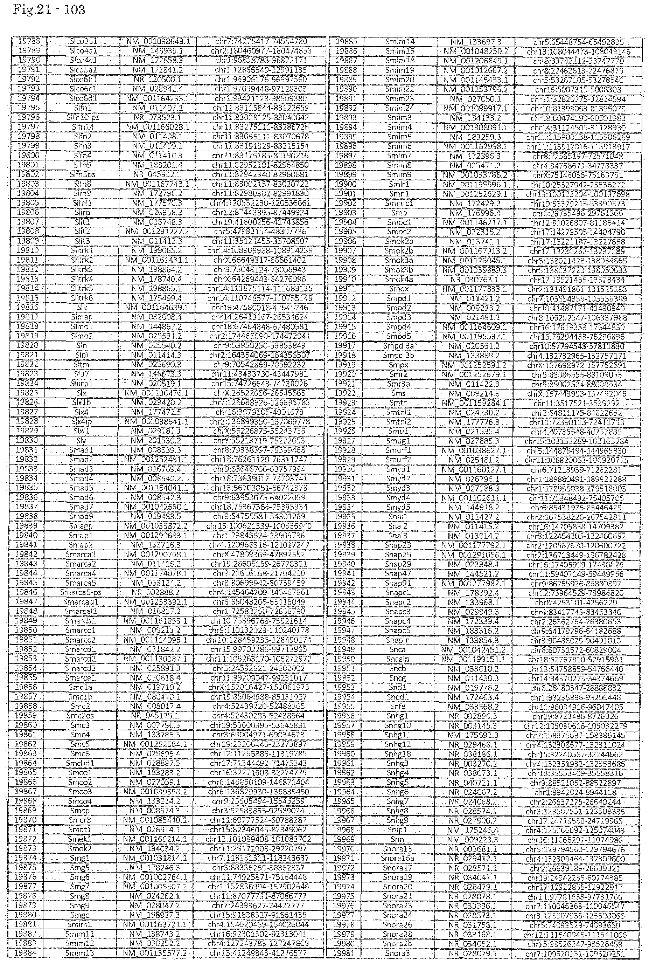

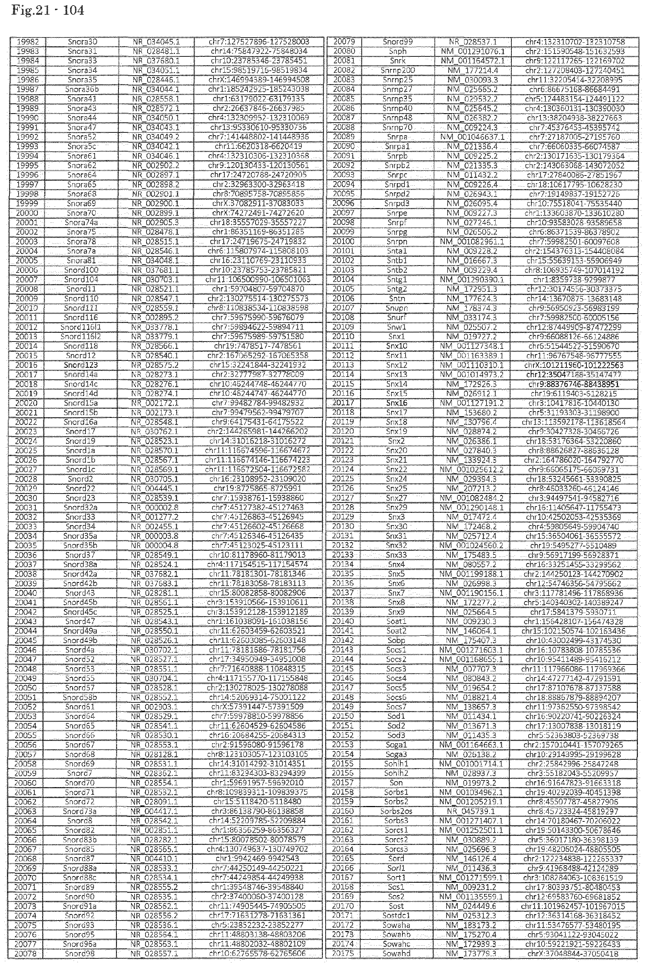

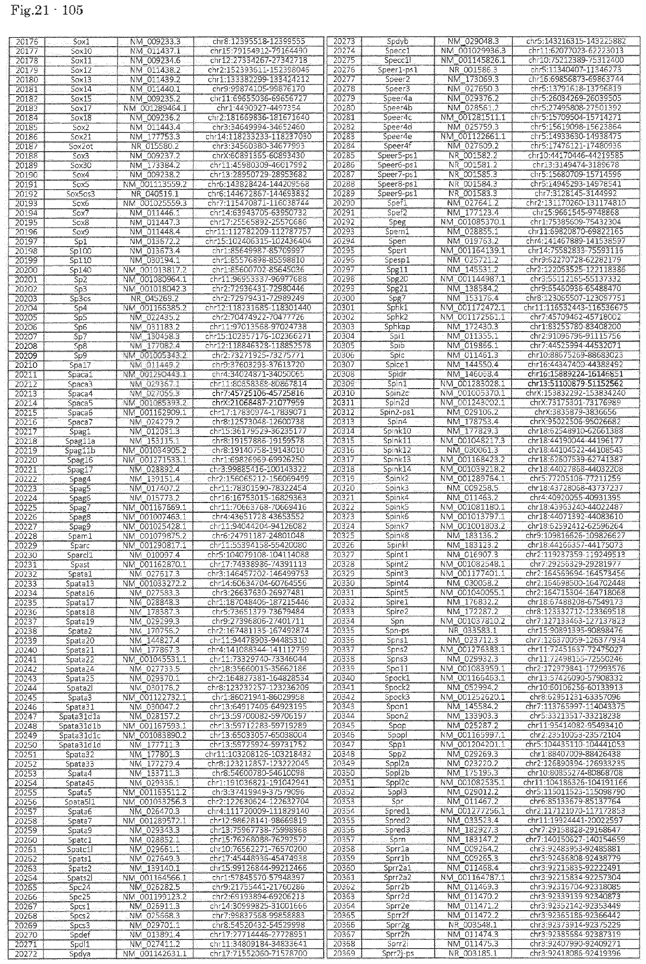

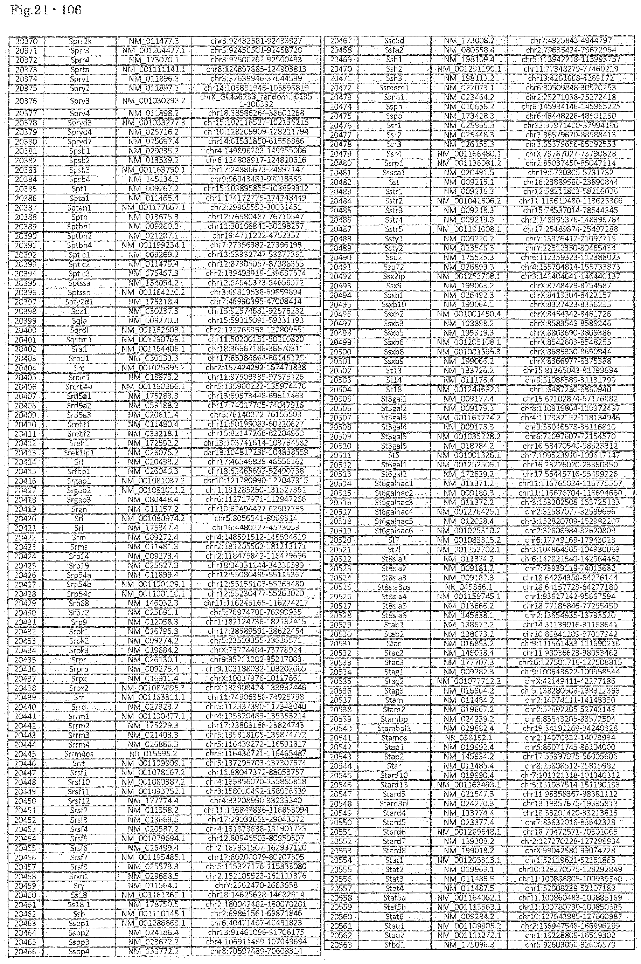

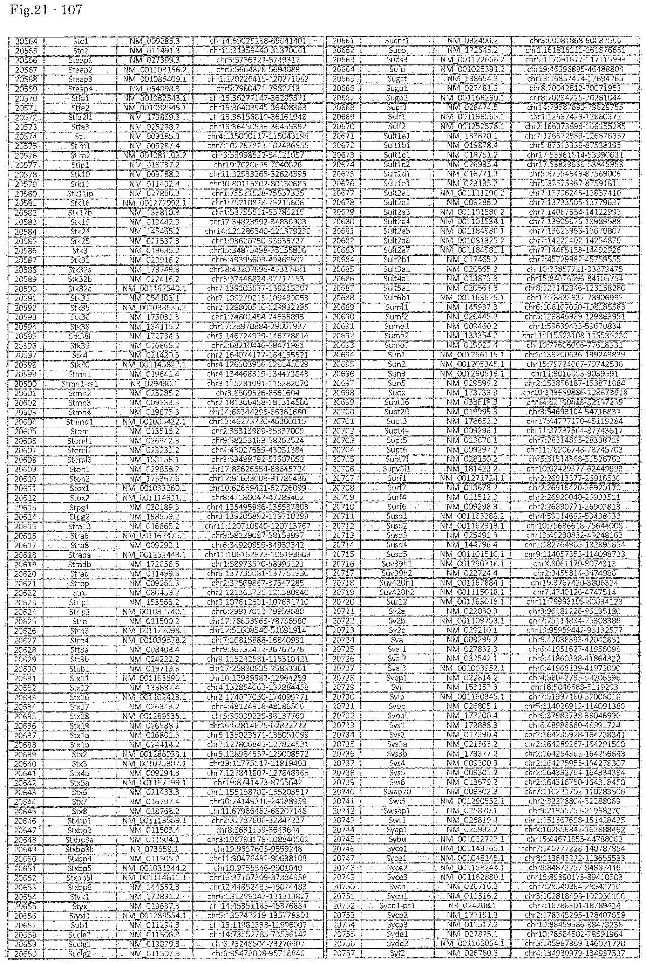

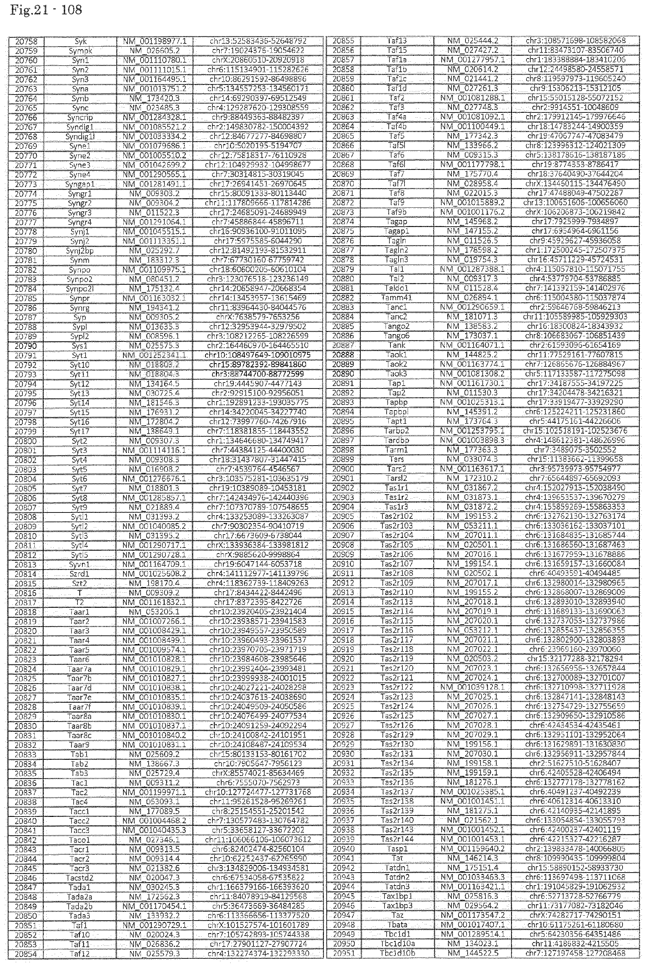

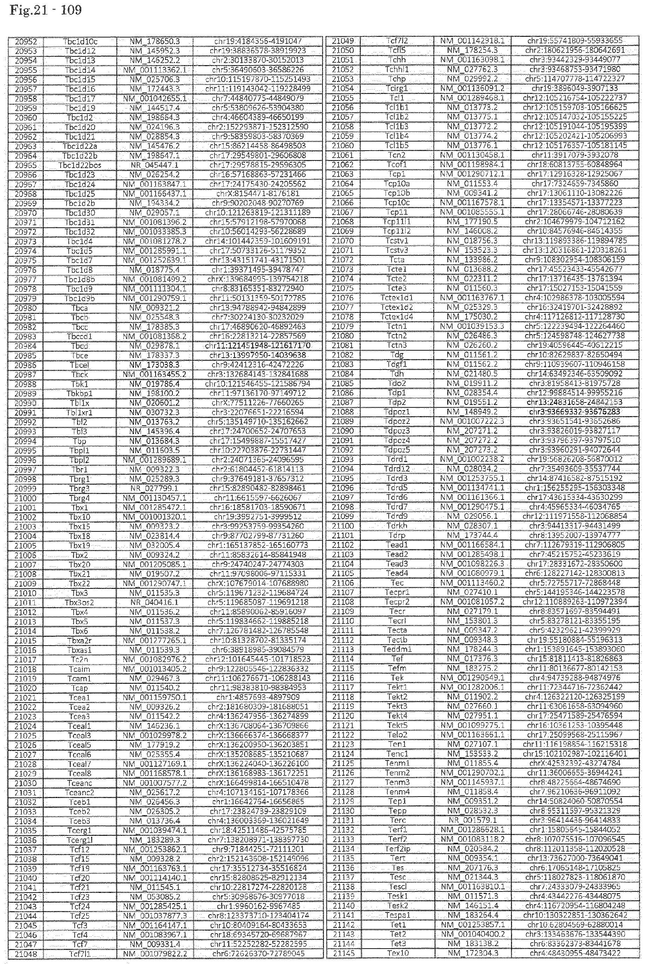

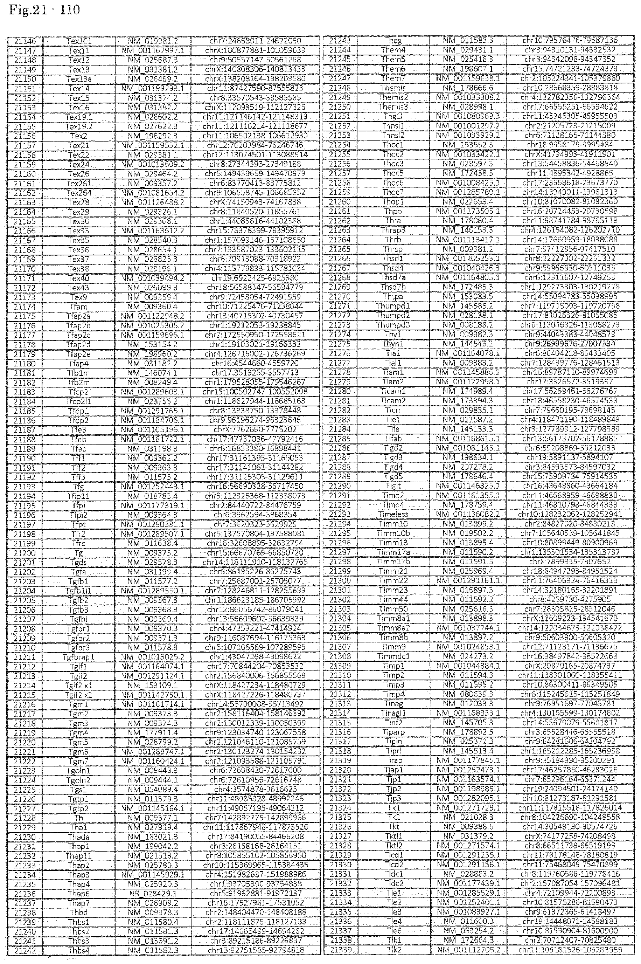

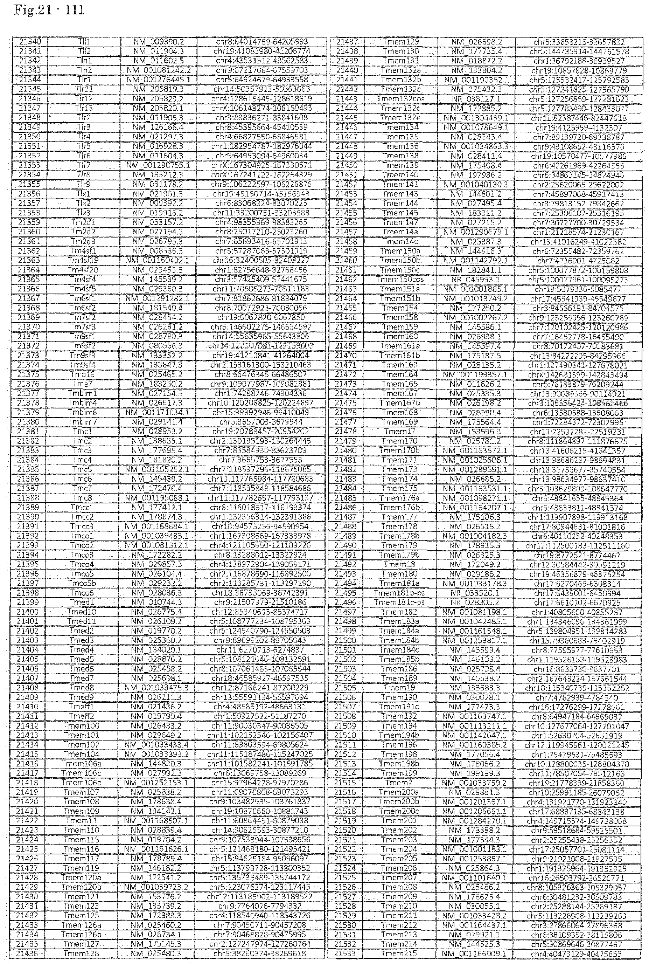

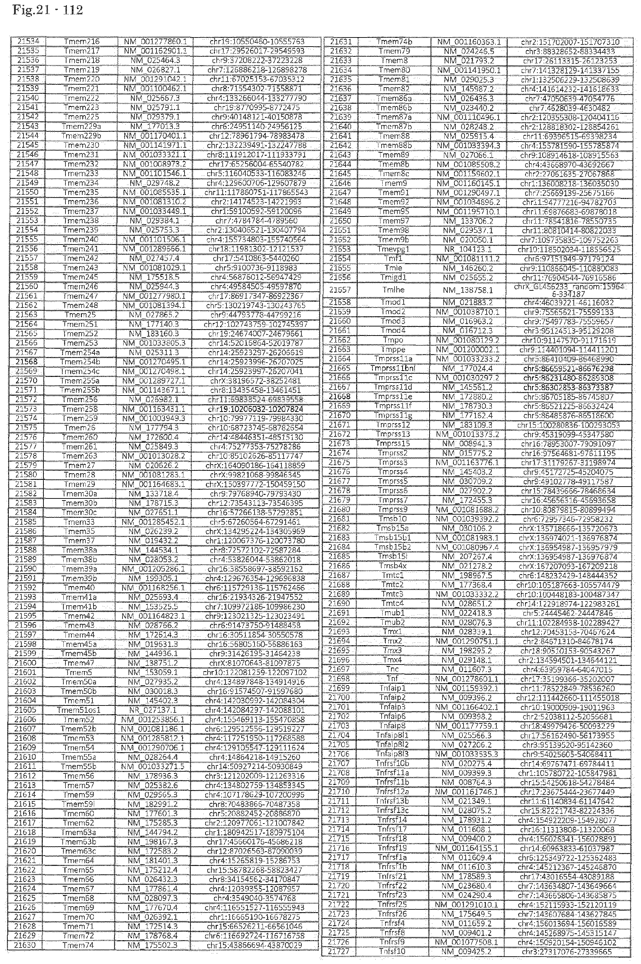

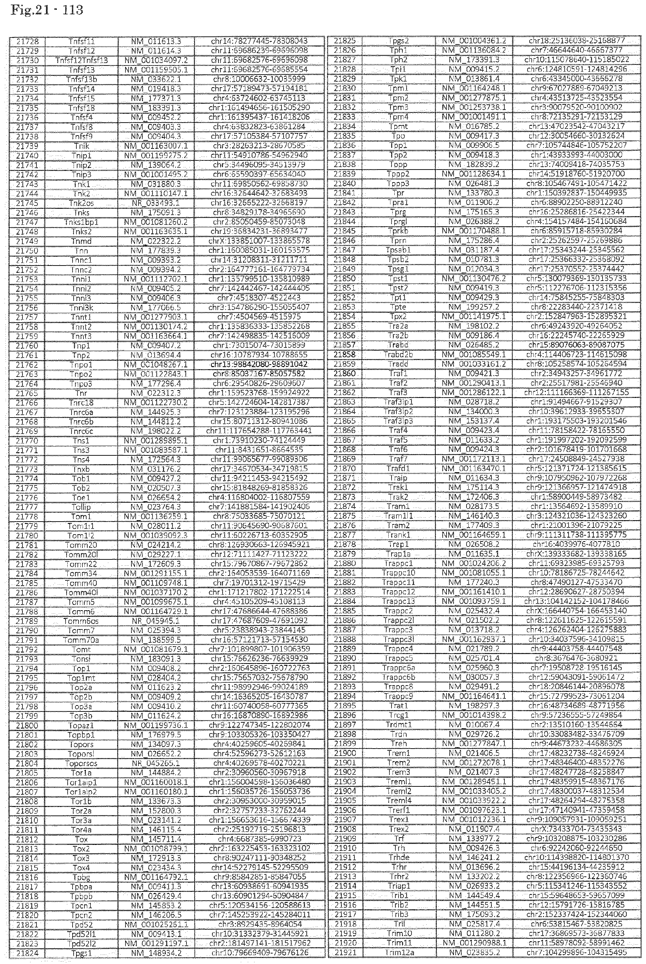

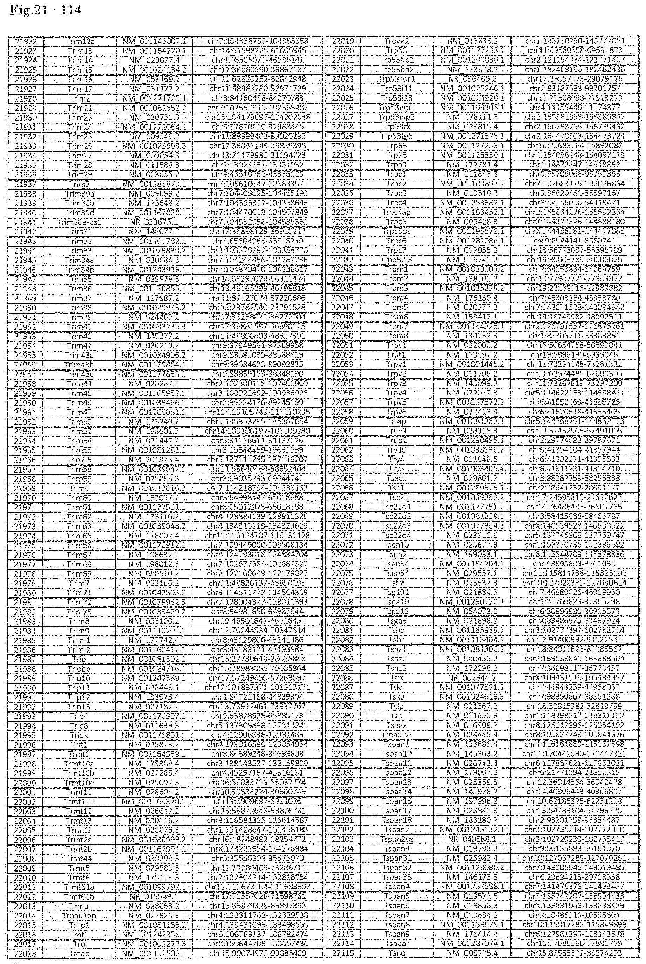

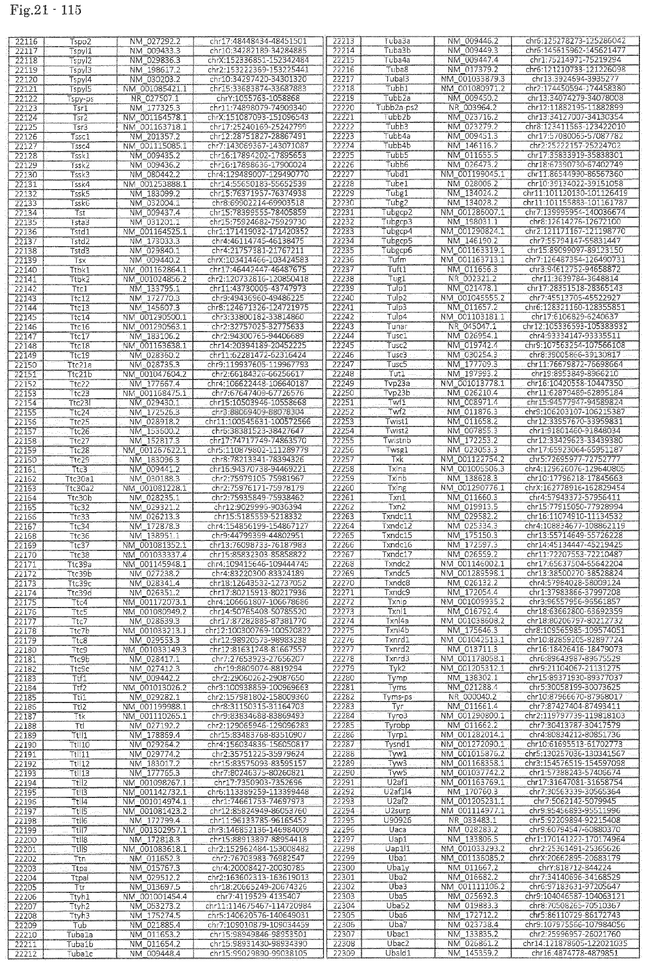

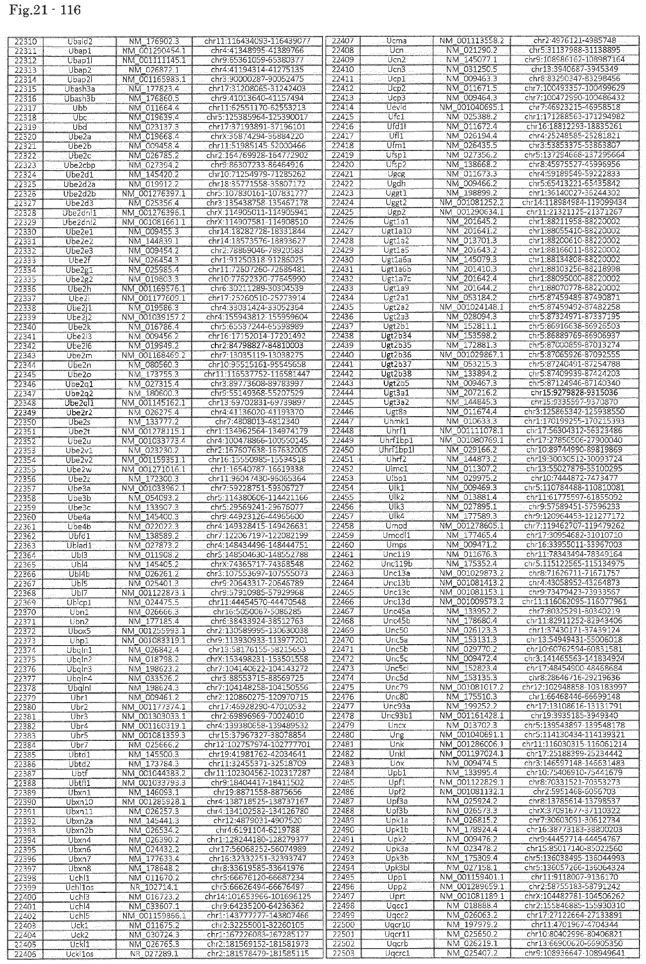

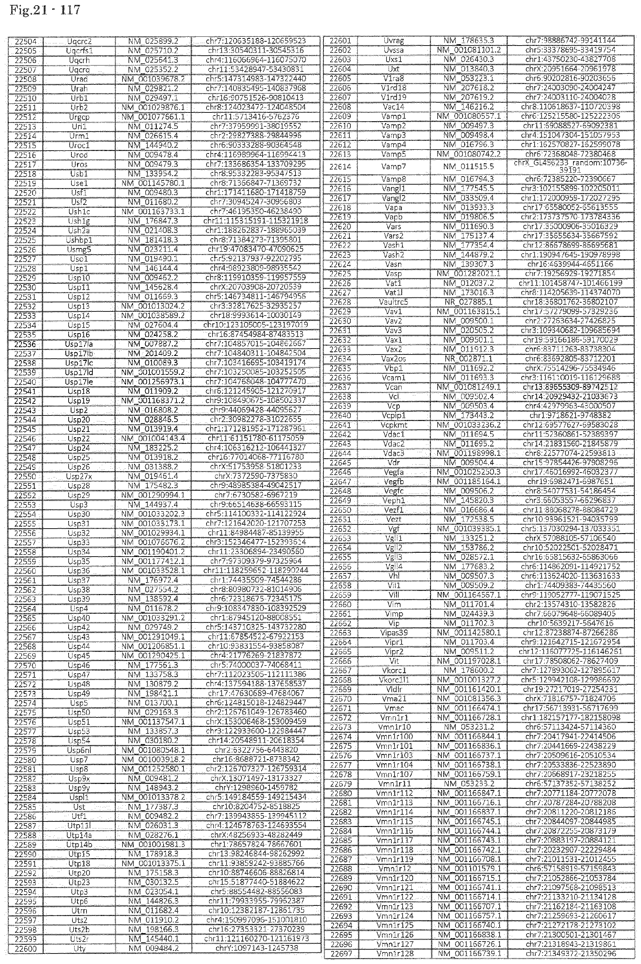

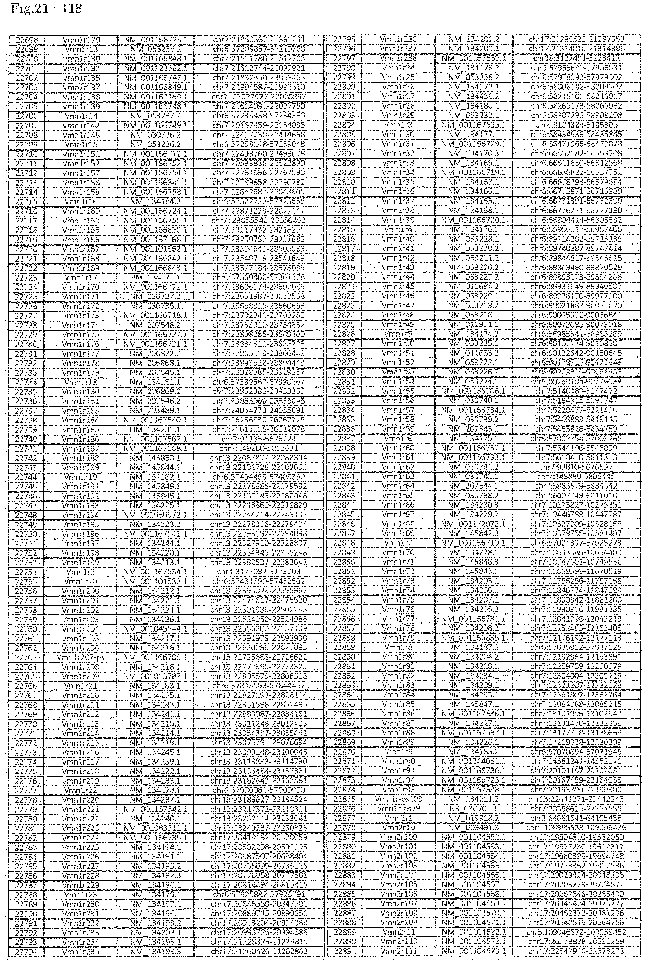

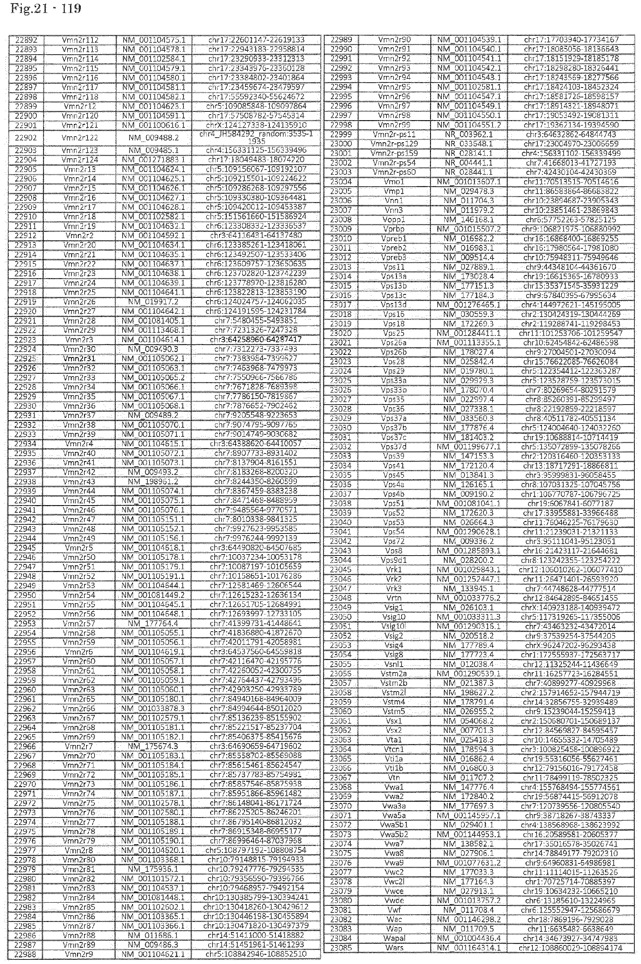

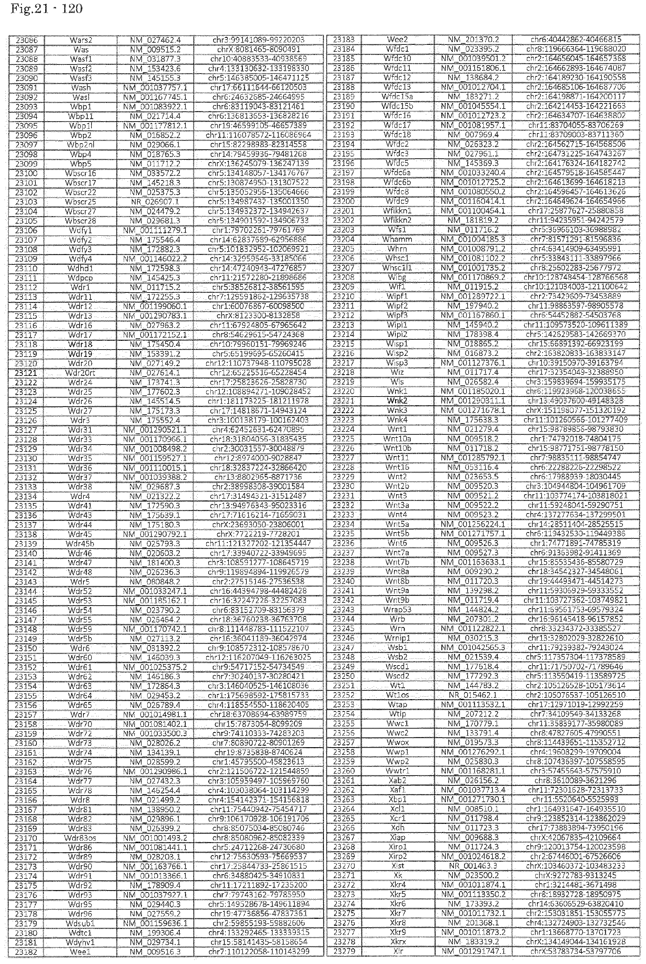

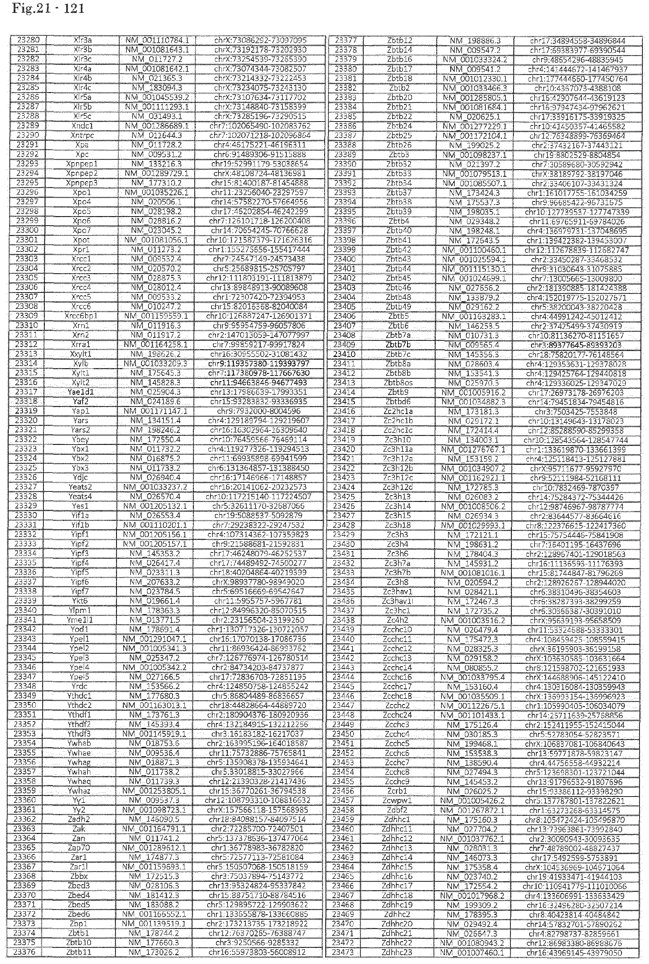

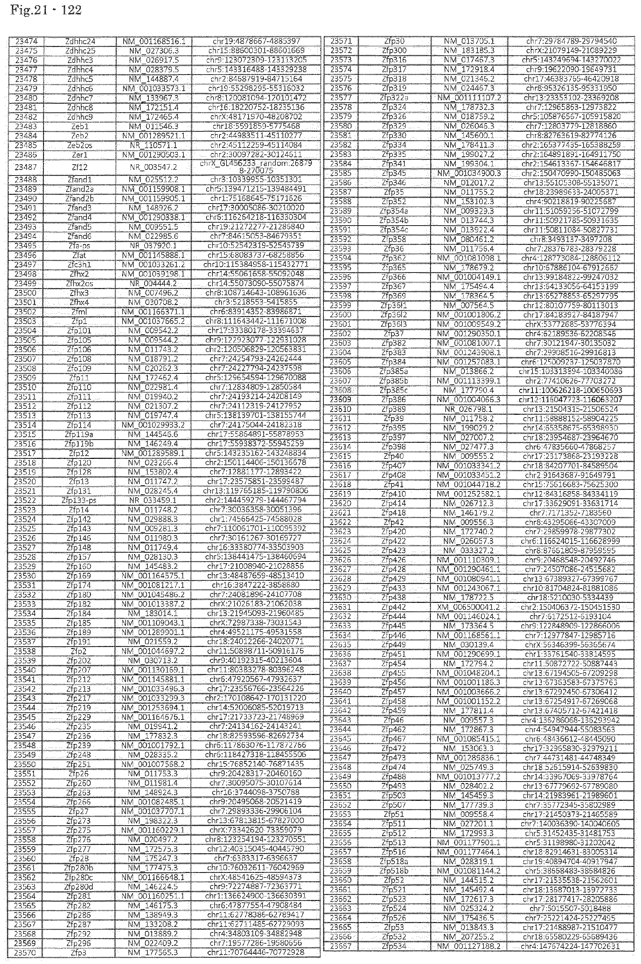

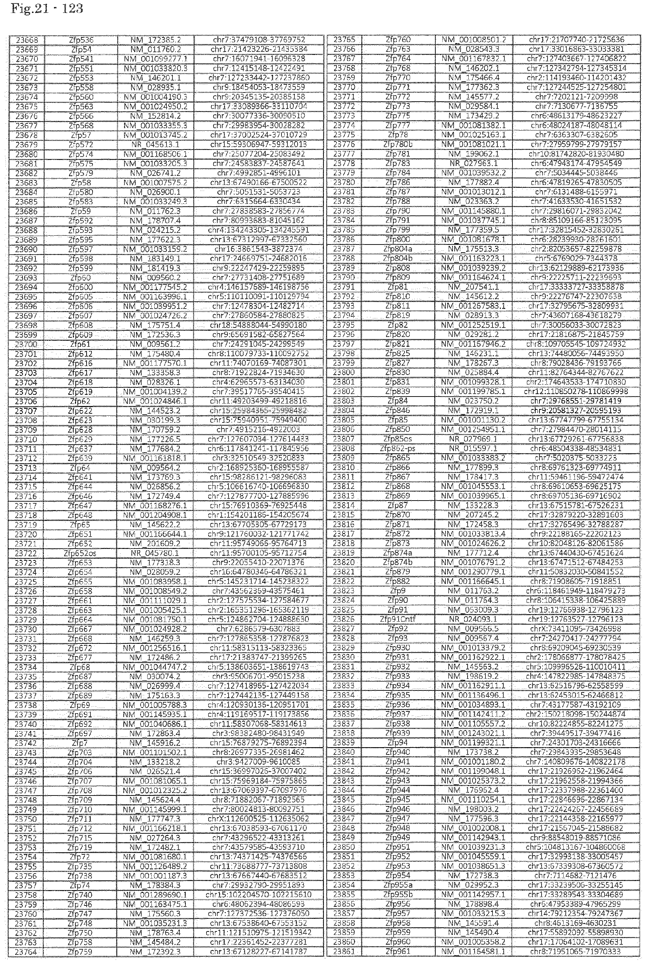

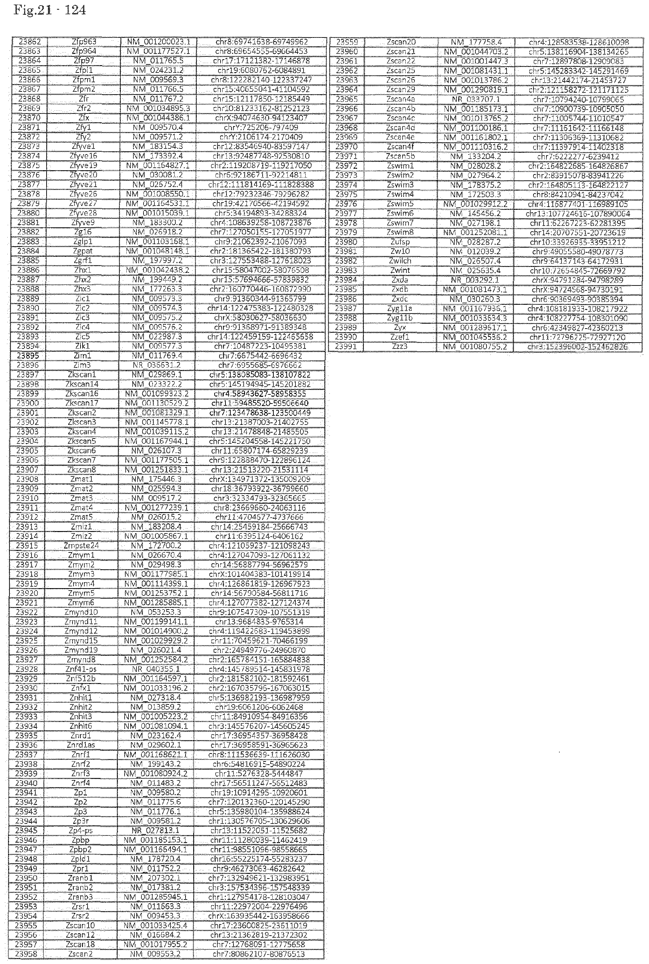

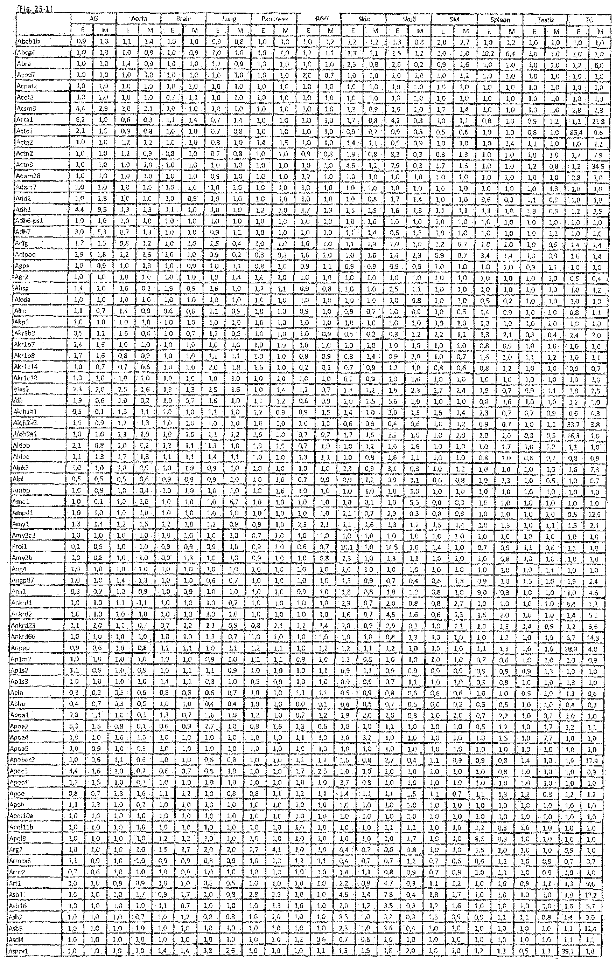

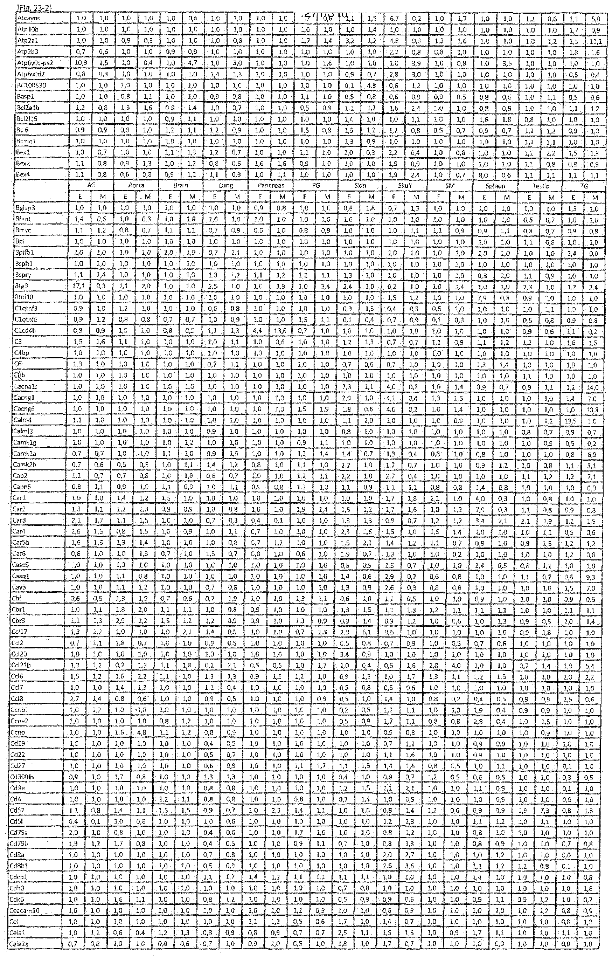

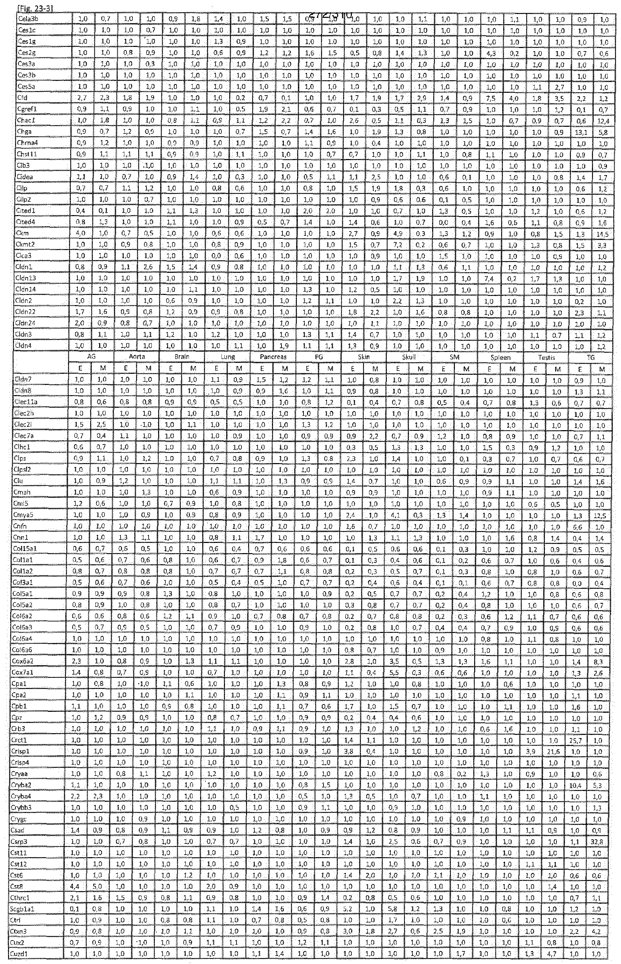

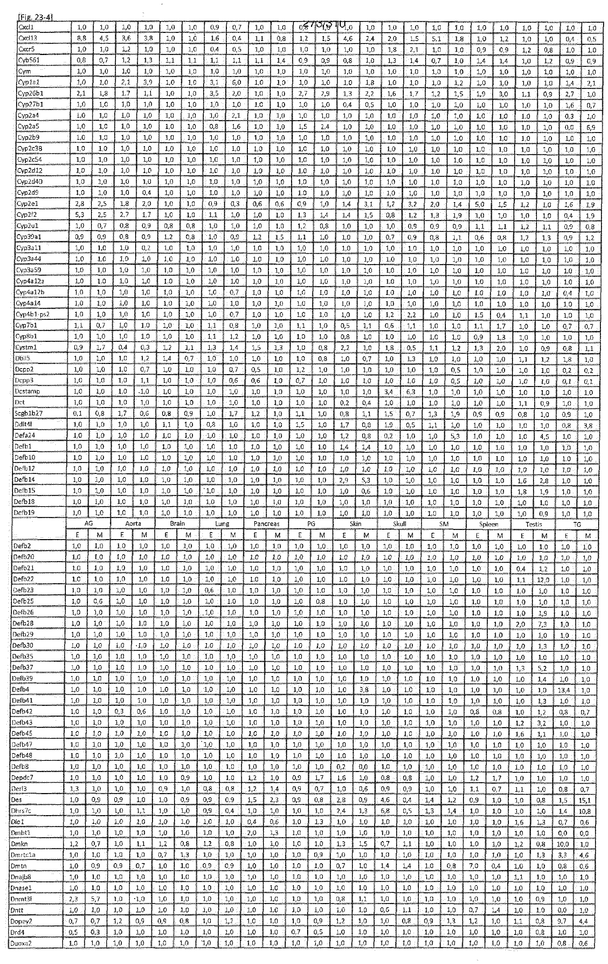

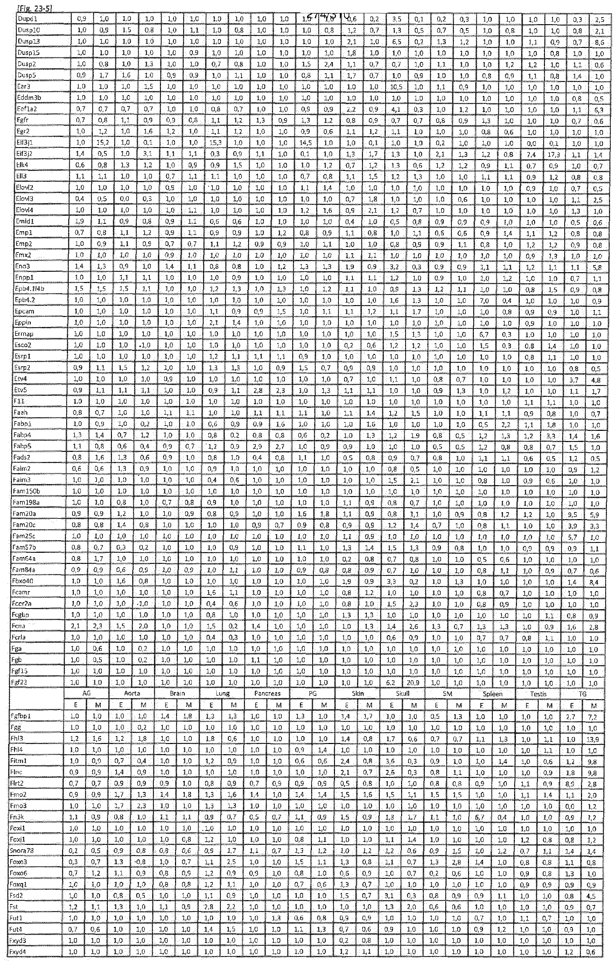

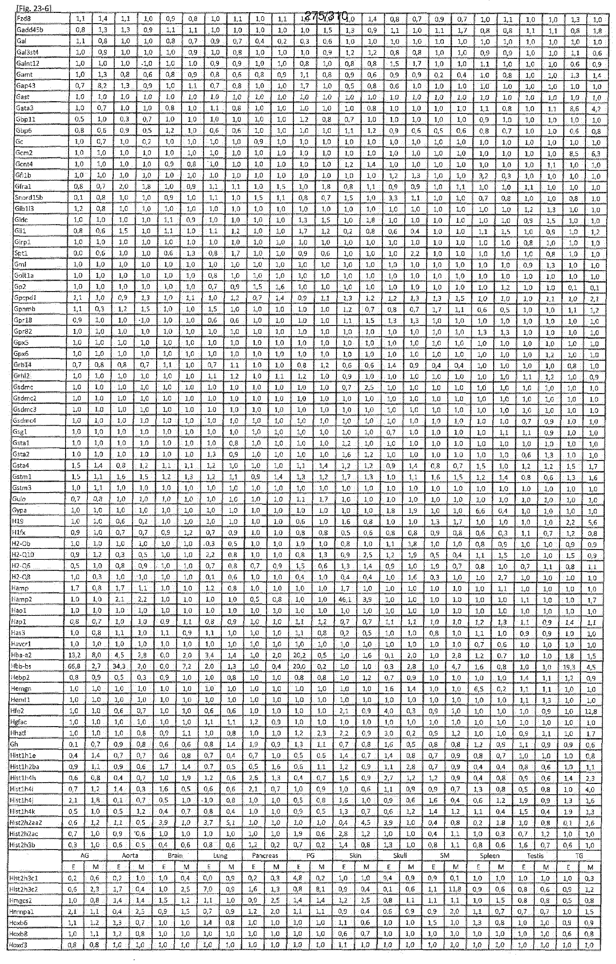

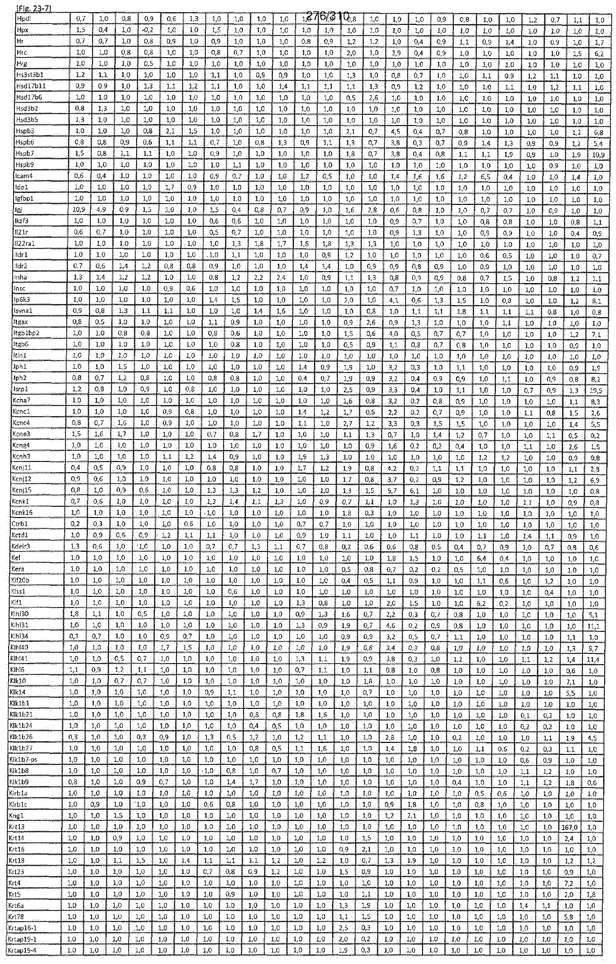

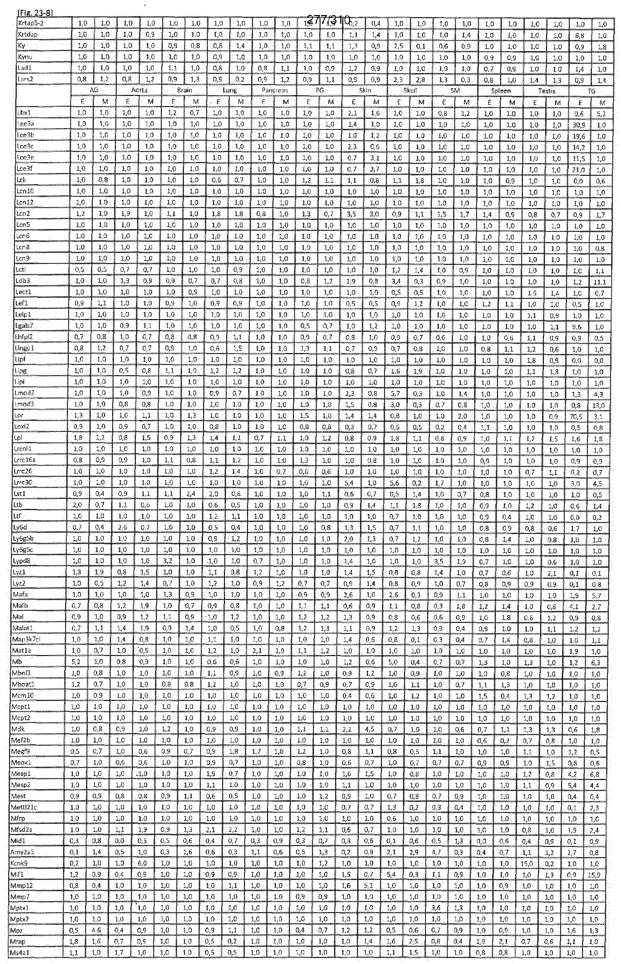

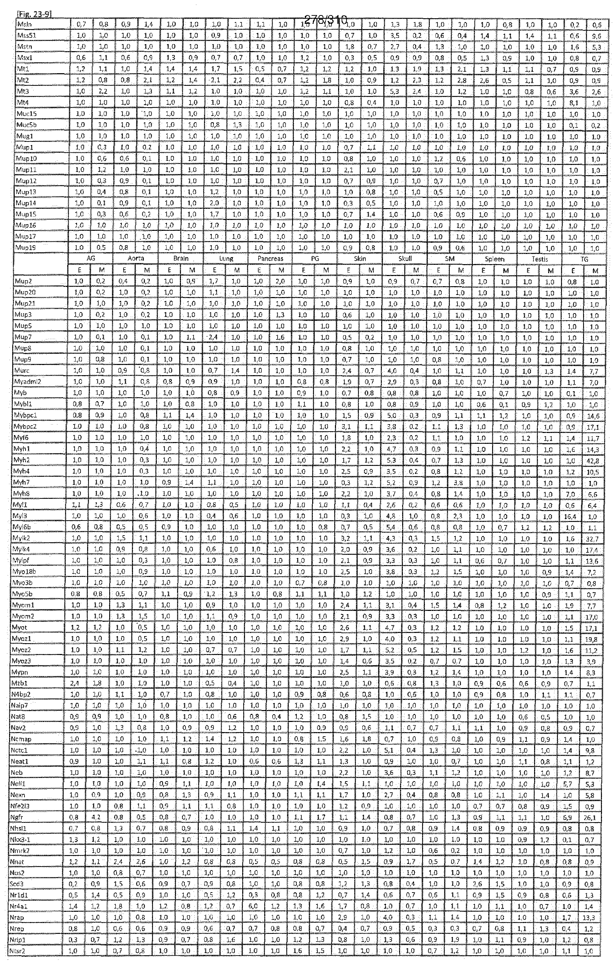

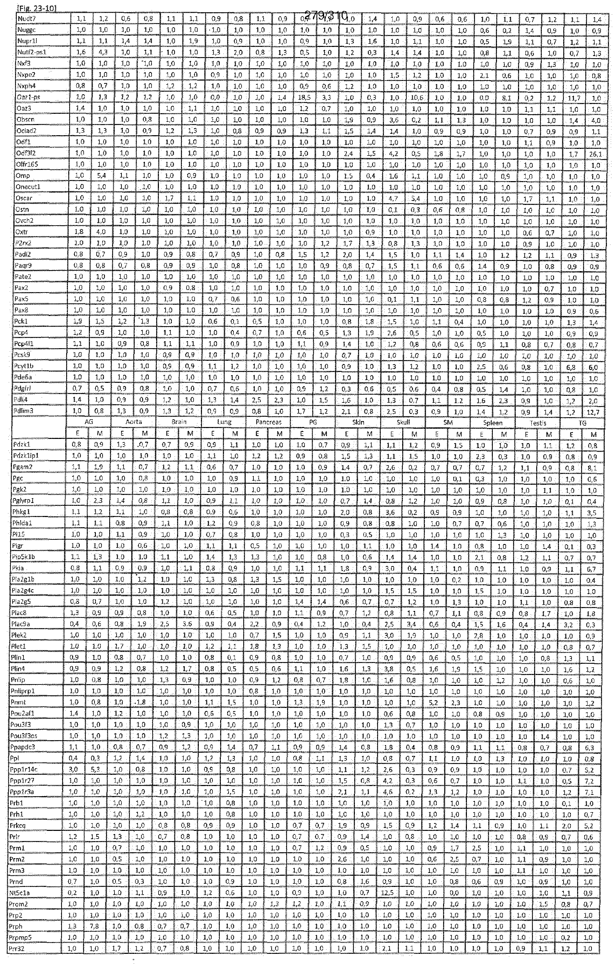

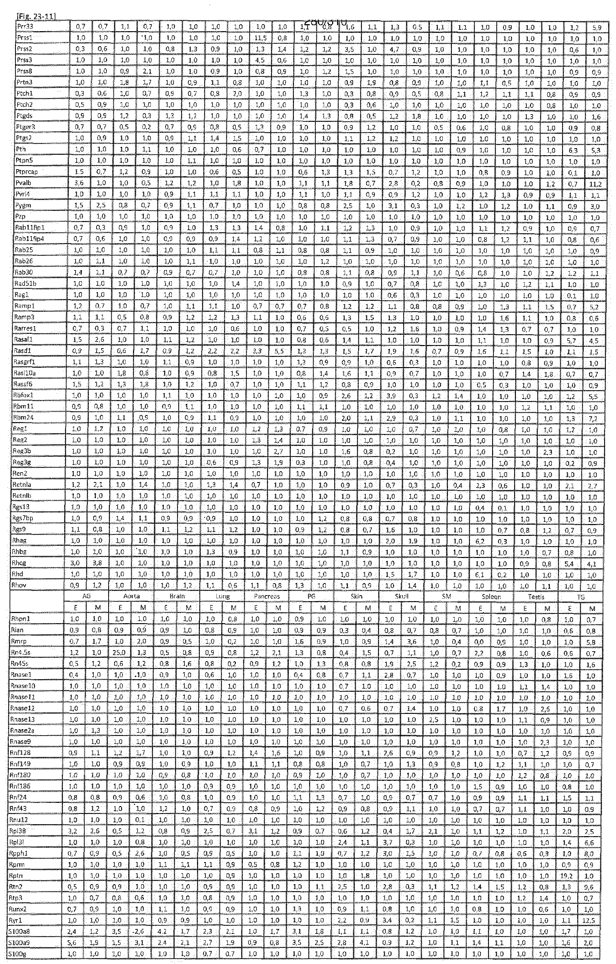

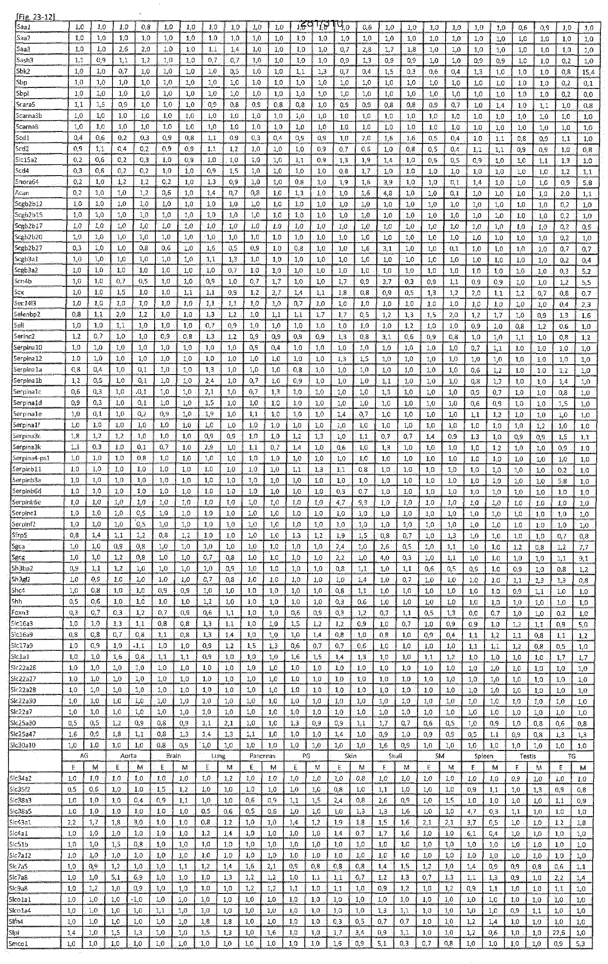

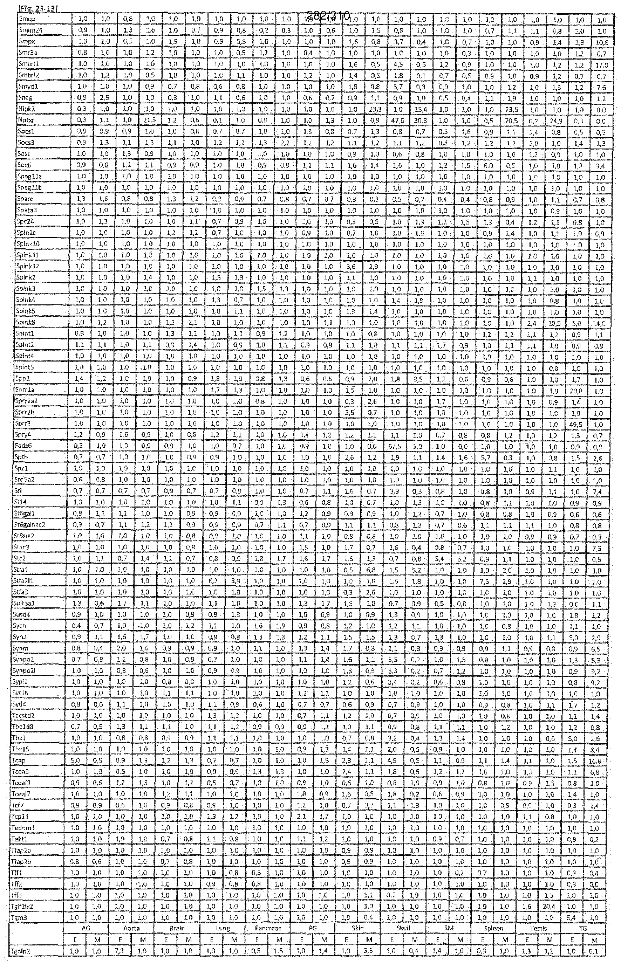

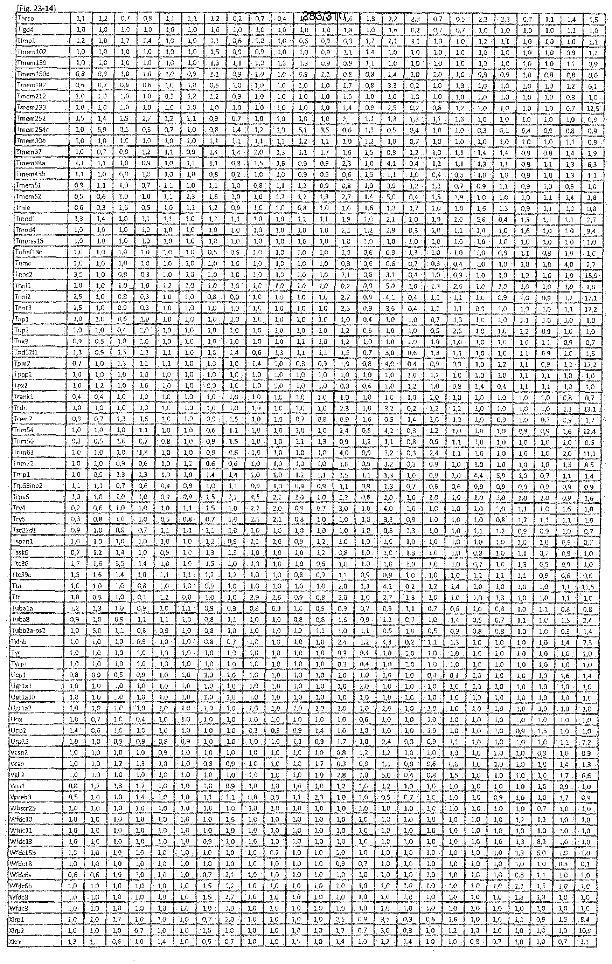

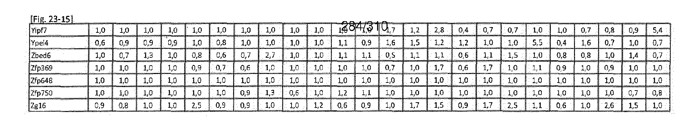

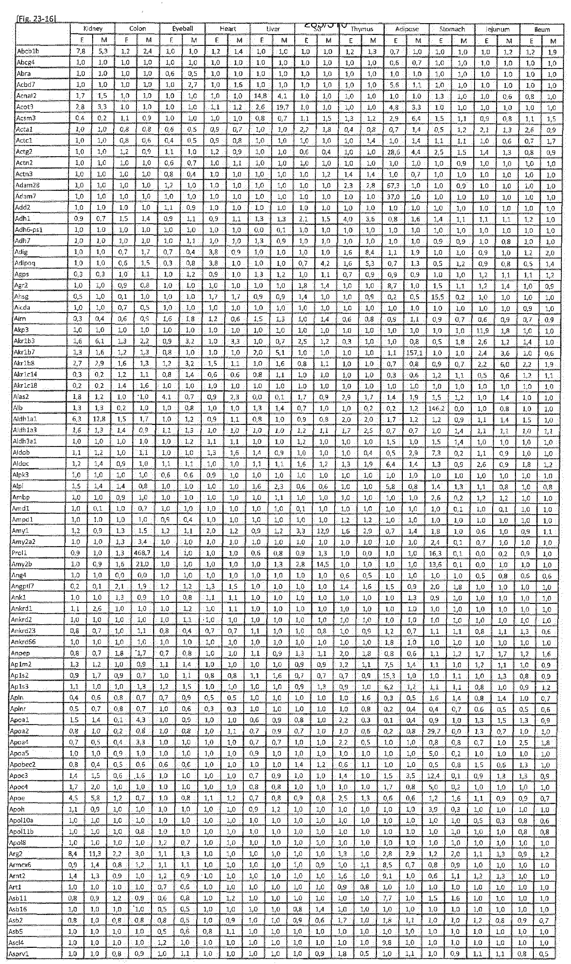

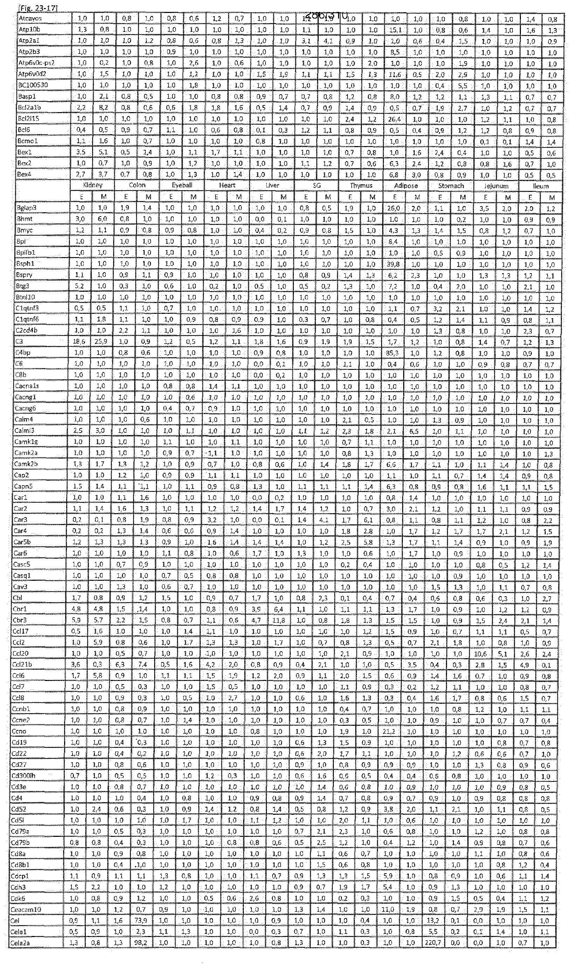

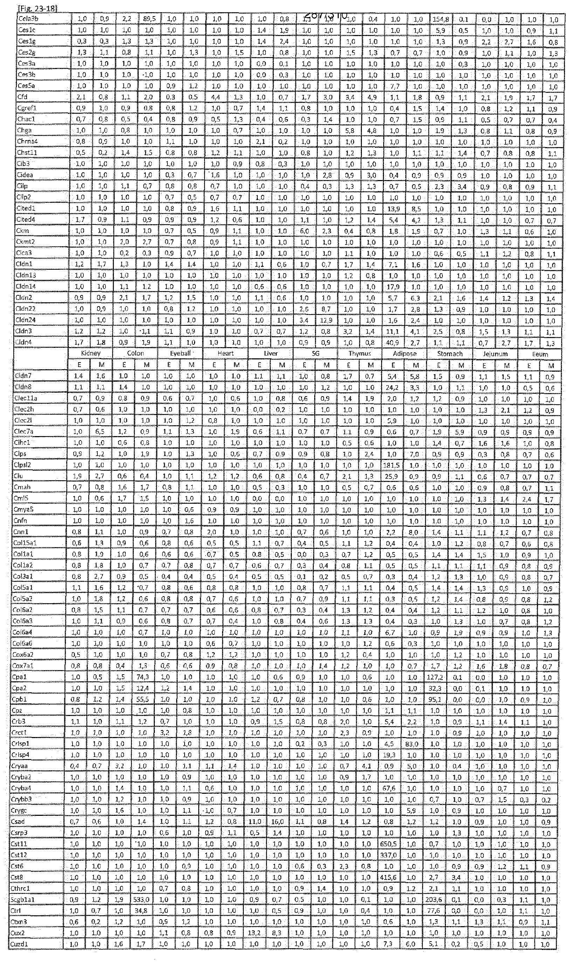

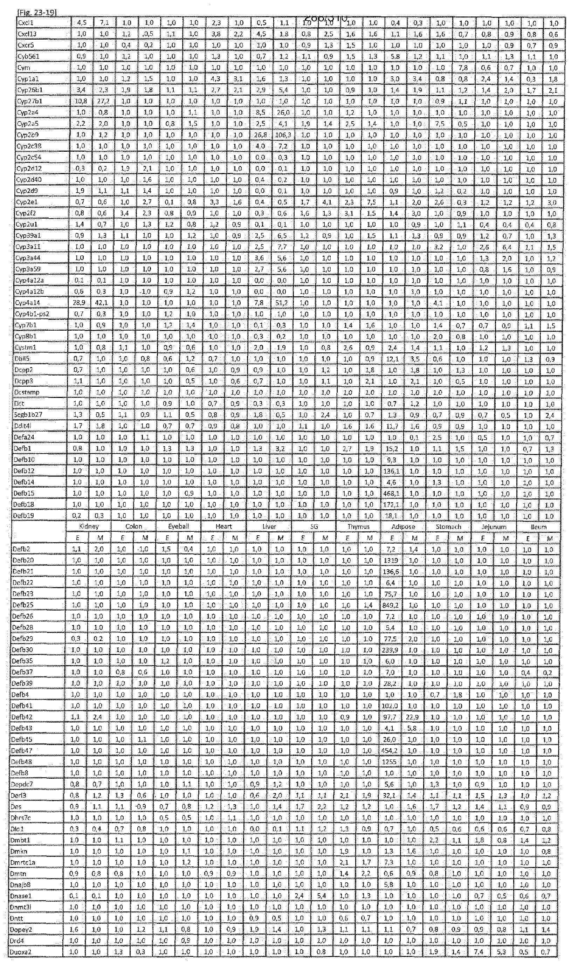

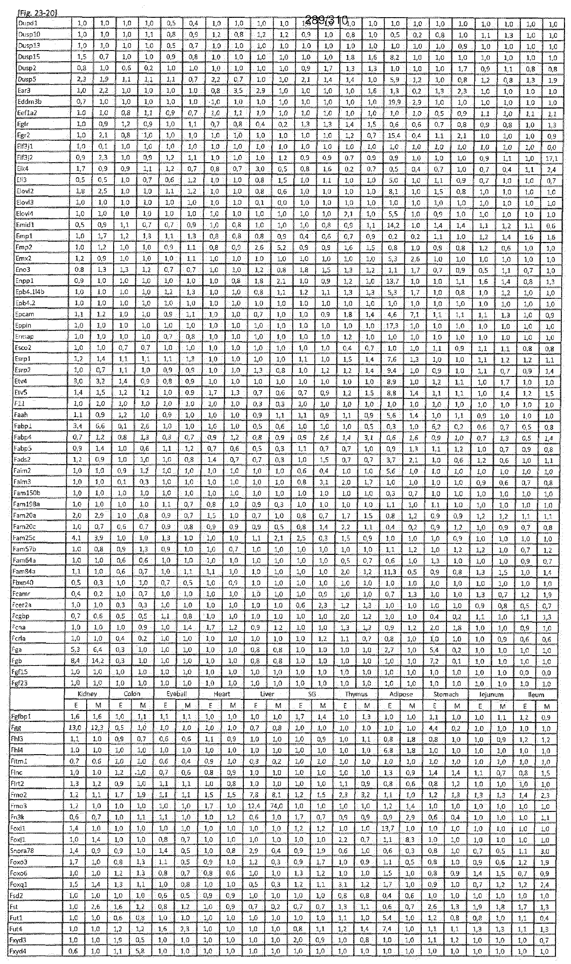

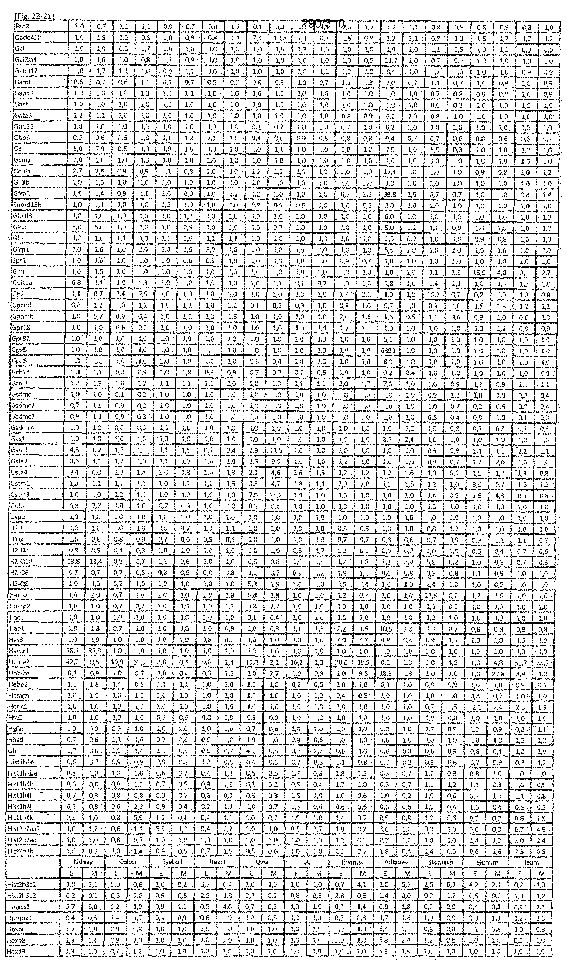

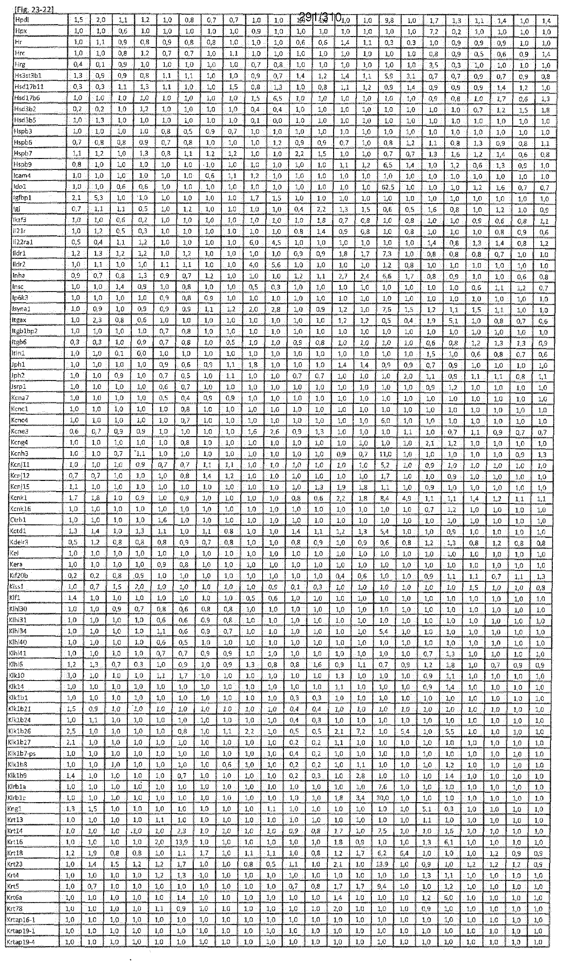

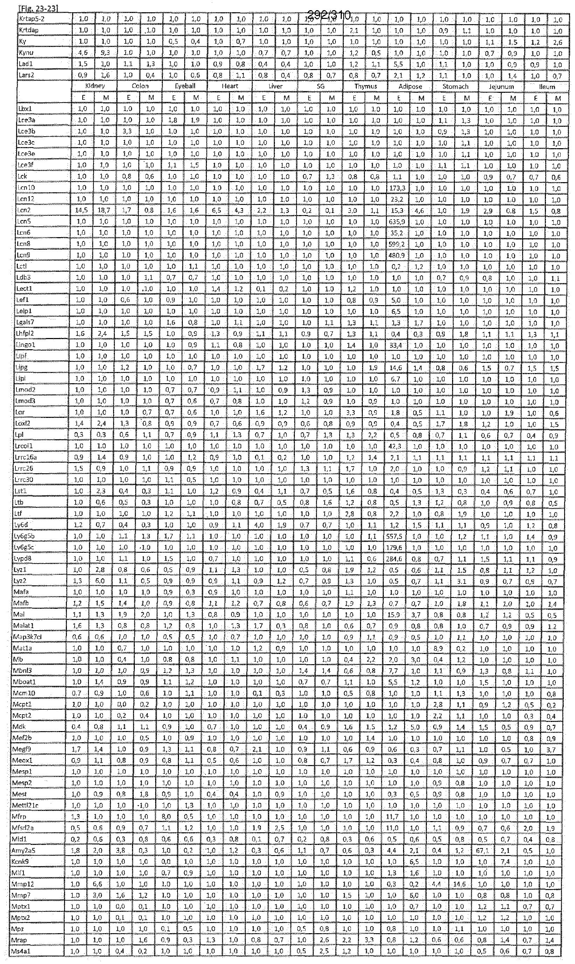

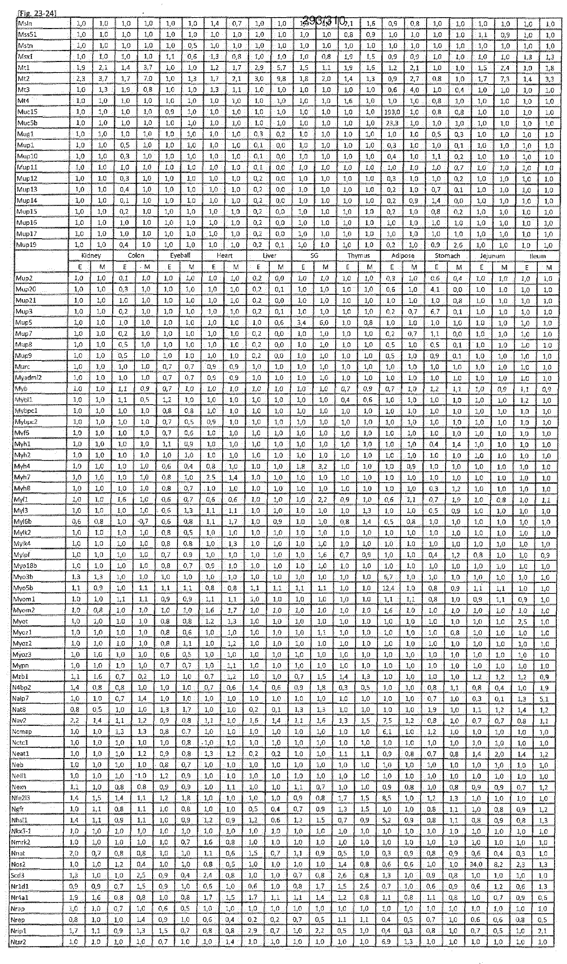

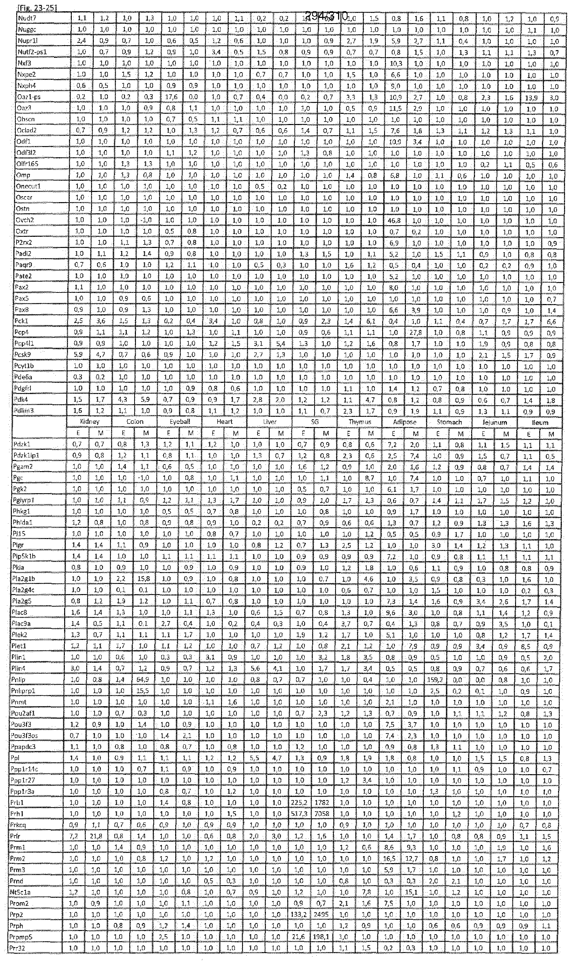

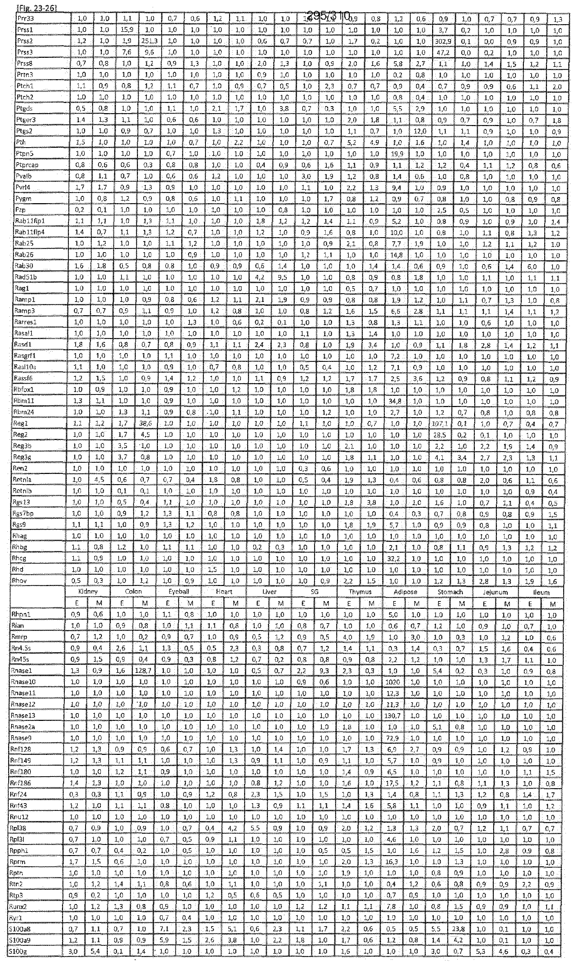

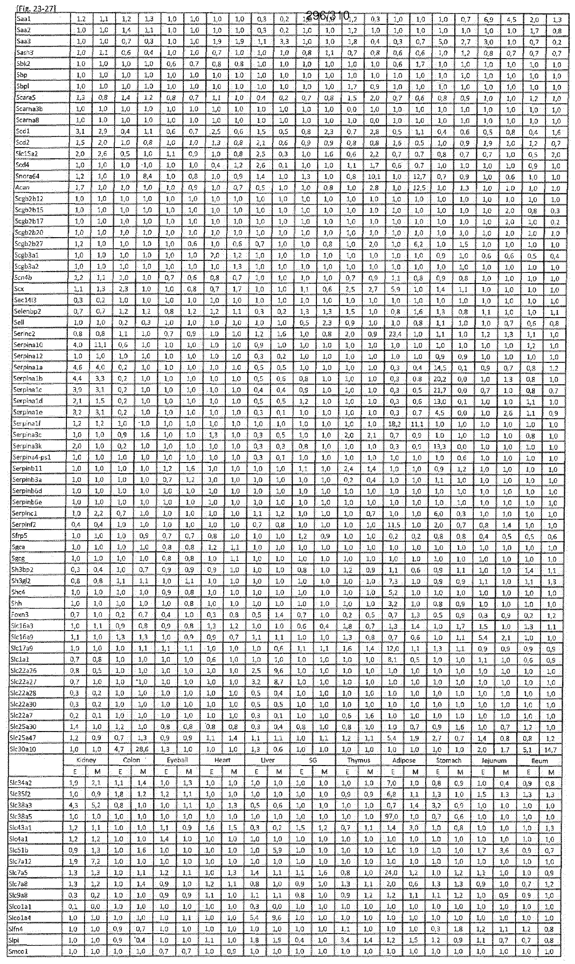

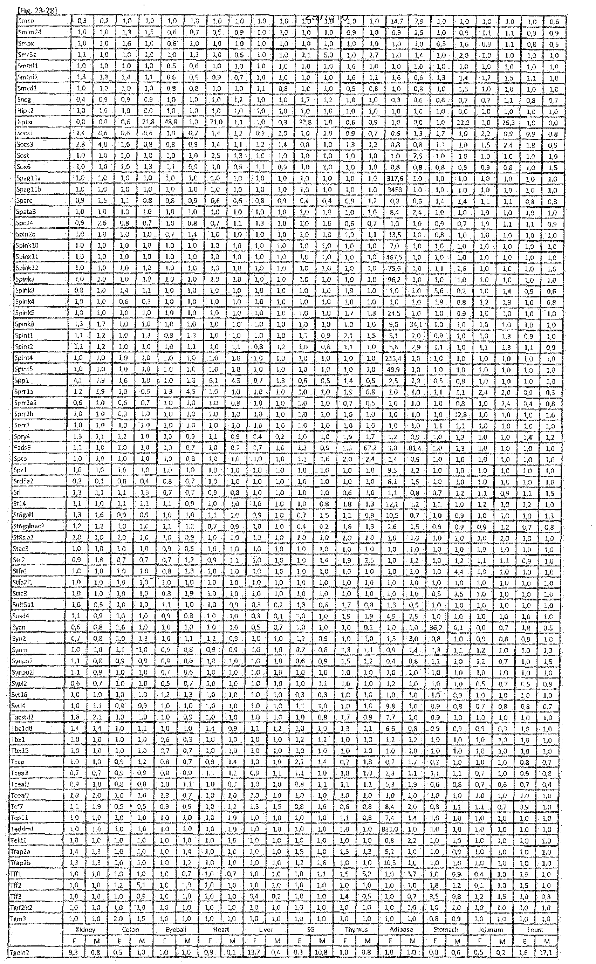

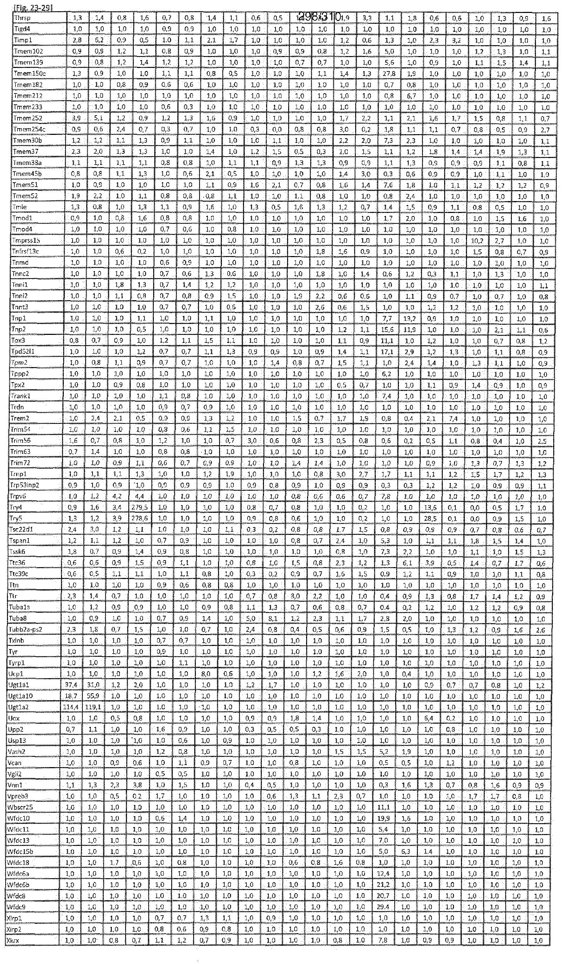

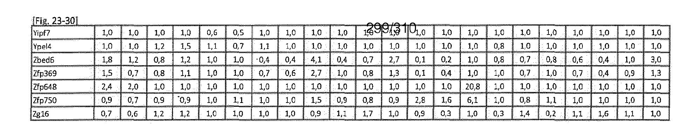

"Kidney function prediction marker" as used herein includes at least one member selected from the group consisting of the genes shown in Fig. 21 ("group 1"). More specifically, the kidney function prediction marker includes at least one member selected from the group consisting of the genes selected from group 2 shown in Fig. 22, preferably at least one member selected from the group consisting of the genes of group 3 shown in Fig. 22, more preferably at least one member selected from the group consisting of the genes of group 4 shown in Fig. 22, and even more preferably at least one member selected from the group consisting of the genes of group 5 shown in Fig. 22. Most preferably, the kidney function prediction marker is at least one member selected from the group consisting of the genes of group 6 shown in Fig. 23, in particular, at least one member selected from the group consisting of proline-rich proteins (Prh1, Prp2, Prb1, Prpmp5), defensins (Defa and Defb), Aplnr, Spp1, Dnase1, Slco1a1, Anpep, Slc7a8, Oscar, fibrinogens (Fga, Fgb, and Fgg), and Hamp/Hepcidin. Moreover, these groups may also include splicing variants of the individual genes.

As specific examples of combinations of a kidney function prediction marker protein and a specimen, it is preferable to use saliva and/or salivary glands (preferably the parotid glands) as a specimen in the case of PRPs, and it is preferable to use adipose tissue, hair roots, skin, secretions from skin, and/or sweat as a specimen when kidney function prediction markers are defensins and/or Hamp2.

In particular, skin is preferable as a specimen for obtaining the measurement value of Defb8; the stomach, skeletal muscle, and/or testes are preferable as a specimen for obtaining the measurement value of Defa24; adipose tissue is preferable as a specimen for obtaining the measurement values of Defb1, Defb10, Defb12, Defb14, Defb15, Defb18, Defb19, Defb2, Defb20, Defb21, Defb22, Defb23, Defb25, Defb26, Defb28, Defb29, Defb30, Defb35, Defb37, Defb39, Defb41, Defb42, Defb43, Defb45, Defb47, and/or Defb48; the skull is preferable as a specimen for obtaining the measurement value of Oscar; saliva, the salivary glands, or parotid glands are preferable as a specimen for obtaining the measurement values of Prb1, Prh1, Prp2, and/or Prpmp5; the skull, kidneys, and/or heart is preferable as a specimen for obtaining the measurement value of Spp1; the kidneys, salivary glands, preferably parotid glands, are preferable as a specimen for obtaining the measurement value of Dnase1; the aorta is preferable as a specimen for obtaining the measurement value of Slc7a8; the thyroid gland is preferable as a specimen for obtaining the measurement value of Anpep; the kidneys and/or liver is preferable as a specimen for obtaining the measurement value of Slco1a1; the adrenal glands, aorta, lungs, pituitary glands, skin, skin, skull, skeletal muscle, spleen, thyroid gland, kidneys, heart, and/or adipose tissue is preferable as a specimen for obtaining the measurement value of Aplnr; and the kidneys, a blood sample, and/or urine is preferable when the measurement values of Fga, Fgb, and Fgg are obtained.

"Measurement value of at least one protein selected from the group consisting of kidney function prediction markers" refers to a value reflecting the amount or concentration of at least one protein selected from the group consisting of kidney function prediction markers. When the measurement value is indicated by "amount," it may be expressed on either a mole basis or a mass basis; however, it is preferable to indicate the amount on a mass basis. When the value is expressed in terms of "concentration," it may be a molar concentration or a ratio of a mass per constant volume of a specimen (mass/volume), preferably a mass/volume ratio. The value reflecting the amount or concentration may be the above or the intensity of a signal such as fluorescence or luminescence.

"Measurement value of at least one mRNA selected from the group consisting of kidney function prediction markers" may be represented by the number of copies (absolute amount) of the kidney function prediction marker mRNA present in a certain amount of a specimen; or may be a value reflecting the relative expression level to that of a housekeeping gene, such as β2-microglobulin mRNA, GAPDH mRNA, Maea mRNA, or β-actin mRNA. The measurement value may also be represented by the intensity of a signal such as fluorescence or luminescence.

"Antibody against a kidney function prediction marker" is not limited, as long as the antibody specifically binds to at least one protein selected from the group consisting of the above-described kidney function prediction markers; and any of polyclonal antibodies, monoclonal antibodies, and fragments thereof (for example, Fab, $F(ab)_2$, etc.) obtained by immunizing a non-human animal with at least one protein selected from the group consisting of the kidney function prediction markers or a part thereof as an antigen can be used. Additionally, immunoglobulin classes and subclasses are not particularly limited. Moreover, the antibody against a kidney function prediction marker may be a chimeric antibody. Further, the antibody against a kidney function prediction marker may be scFv or the like.

Examples of a kidney function prediction marker protein used as an antigen for preparing an antibody against a kidney function prediction marker include the entirety or a part of at least one protein selected from the group consisting of the above-described kidney function prediction markers.

"Nucleic acid for kidney function prediction marker mRNA detection" as used herein is not limited, as long as it contains a sequence that specifically hybridizes to at least one mRNA selected from the group consisting of the above-described kidney function prediction markers, or to a reverse transcription product of the mRNA. The nucleic acid for detection may be DNA or RNA, and the nucleotides contained in the nucleic acid for detection may be naturally occurring nucleotides or artificially synthesized nucleotides.

The length of the nucleic acid for detection is not particularly limited. When the nucleic acid for detection is used as a capture probe in, for example, a microarray, the length of sequence that hybridizes to a target nucleic acid is preferably about 100 mer, more preferably about 60 mer, and even more preferably about 20 to 30 mer. The capture probe can be produced with, for example, a known oligonucleotide synthesizer. The capture probe may contain a sequence that does not hybridize to the target nucleic acid.

When the nucleic acid for detection is a primer used for PCR reactions, the length of sequence that hybridizes to a target nucleic acid is preferably about 50 mer, more preferably about 30 mer, and even more preferably about 15 to 25 mer. The primer can be produced with, for example, a known oligonucleotide synthesizer. The primer may contain a sequence that does not hybridize to the target nucleic acid. The primer may be labeled with a fluorescent dye or the like.

A probe for quantification that is decomposed during a PCR reaction may also be used for real-time quantification of a PCR product in RT-PCR, in addition to primers. The probe for quantification is not limited as long as it hybridizes to a target nucleic acid. The probe for quantification is preferably a nucleic acid with a length of about 5 to 20 mer that contains a sequence that hybridizes to a target nucleic acid. Further, it is preferred that the probe for quantification is labeled at one end with a fluorescent dye, and at the other end with a quencher of the fluorescent dye.

2. Method for obtaining each measurement value

The methods for obtaining the measurement value of at least one protein selected from the group consisting of kidney function prediction markers and the measurement value of at least one mRNA selected from the group consisting of kidney function prediction markers in the present specification are not limited, as long as the measurement values can be obtained. For example, they can be obtained according to the methods described below.

2-1. Method for measuring kidney function prediction marker protein

When the measurement value of at least one protein selected from the group consisting of kidney function prediction markers (hereinafter may be abbreviated as "measurement value of a kidney function prediction marker protein" in the present specification) is measured, a measurement method using an antibody against a kidney function prediction marker described in the section "1. Explanation of terms" can be used in the process in order to obtain the measurement value. The known ELISA method or the like can be used as the measurement method for obtaining the measurement value of a kidney function prediction marker protein.

In this embodiment, an antibody against a kidney function prediction marker for antigen capture can be immobilized on a solid phase such as a microplate, fluorescent beads, or magnetic beads in advance, and a complex between the immobilized antibody against a kidney function prediction marker and a kidney function prediction marker protein in a specimen can be formed. The amount or concentration of the kidney function prediction marker protein contained in the specimen can be measured by detecting the complex immobilized on the solid phase or the complex formed on the solid phase by a method known in the art. In this embodiment, a complex between an antibody against a kidney function prediction marker for antigen capture and a kidney function prediction marker protein in a specimen may be formed in advance, and then immobilized on a solid phase.

The method for immobilizing an antibody against a kidney function prediction marker for antigen capture on a solid phase is not particularly limited. An antibody against a kidney function prediction marker may be directly immobilized or indirectly immobilized with another substance interposed therebetween by using a known method. Examples of direct binding include physical adsorption and the like. Preferably, for example, an immunoplate may be used to directly physically bind an antibody against a kidney function prediction marker to the microplate.

The shape of the solid phase is not particularly limited. Examples include microplates, microtubes, test tubes, beads, and the like. The material of the solid phase is not particularly limited. For example, polystyrene, polypropylene, and the like can be used for microplates, microtubes, test tubes, etc. In the case of beads, Polystyrene xMAP (registered trademark) Beads (Luminex), MagPlex (registered trademark) Microspheres (Luminex), and the like can be used.

This method may comprise, following the formation of the complex, an operation of washing the solid phase. In washing, for example, PBS containing a surfactant or the like may be used.

In this method, the complex can be detected by using an antibody against a kidney function prediction marker for detection labeled with a labeling substance, or using an unlabeled antibody against a kidney function prediction marker, an anti-immunoglobulin antibody labeled with a labeling substance and capable of binding to the unlabeled antibody against a kidney function prediction marker, etc. It is preferable to use a labeled antibody against a kidney function prediction marker for detection. It is also preferable that the epitope in the kidney function prediction marker protein of the antibody against a kidney function prediction marker for detection is different from the epitope in the kidney function prediction marker protein of the antibody against a kidney function prediction marker for antigen capture.

The labeling substance used for the antibody against a kidney function prediction marker for detection or the labeled anti-immunoglobulin antibody is not particularly limited as long as the labeling substance generates a detectable signal. Examples include fluorescent substances, radioactive isotopes, enzymes, and the like. Examples of enzymes include alkaline phosphatase, peroxidase, and the like. Examples of fluorescent substances include fluorescent dyes such as fluorescein isothiocyanate (FITC), rhodamine, and Alexa Fluor (registered trademark), fluorescent proteins such as GFP, and the like. Examples of radioactive isotopes include $^{125}I$, $^{14}C$, $^{32}P$, and the like. Among them, alkaline phosphatase or peroxidase is preferable as the labeling substance.

The antibody against a kidney function prediction marker for detection is obtained by labeling an antibody against a kidney function prediction marker with the above-mentioned labeling substance by a labeling method known in the art. Alternatively, such labeling may be performed using a commercially available labeling kit or the like. For the labeled immunoglobulin antibody, the same method as the labeling of the antibody against a kidney function prediction marker may be used, or a commercially available product may be used.

In this method, the measurement value of the kidney function prediction marker contained in the specimen can be obtained by detecting a signal generated by the labeling substance of the labeled antibody against a kidney function prediction marker contained in the complex. Here, "detecting a signal" includes qualitatively detecting the presence or absence of a signal, quantifying the signal intensity, and semi-quantitatively detecting the signal intensity. Such semi-quantitative detection means to indicate the signal intensity in stages such as "no signal generation," "weak," "medium," and "strong." In this step, it is preferable to detect the signal intensity quantitatively or semi-quantitatively.

As the method for detecting a signal, a known method may be used. In this method, a measurement method according to the type of signal derived from the above-mentioned labeling substance may be appropriately selected. For example, when the labeling substance is an enzyme, detection of a signal may be performed by measuring a signal such as light or color generated by the reaction of the enzyme with a substrate using a known device such as a luminometer or a spectrophotometer.

The substrate of an enzyme can be appropriately selected from known substrates depending on the type of enzyme. For example, when alkaline phosphatase is used as an enzyme, examples of substrates include chemiluminescent substrates such as CDP-Star (registered trademark) (disodium 4-chloro-3-(methoxyspiro[1,2-dioxetane-3,2'-(5'-chloro)tricyclo[3.3.1.1$^{3,7}$]decan]-4-yl)phenyl phosphate), and chromogenic substrates such as 5-bromo-4-chloro-3-indolyl phosphate (BCIP), disodium 5-bromo-6-chloro-3-indolyl phosphate, and p-nitrophenyl phosphate. When the labeling substance is peroxidase, examples of substrates include tetramethylbenzidine (TMB) and the like.

When the labeling substance is a radioactive isotope, a signal, i.e., radiation, can be measured using a known device such as a scintillation counter. When the labeling substance is a fluorescent substance, a signal, i.e., fluorescence, can be measured using a known device such as a fluorescence microplate reader or Luminex (registered trademark) system (Luminex). The excitation wavelength and the fluorescence wavelength can be appropriately determined according to the type of fluorescent substance used.

The detection results of the signal can be used as the measurement value of the kidney function prediction marker protein. For example, when the signal intensity is quantitatively detected, the measurement value itself of the signal intensity or a value calculated from the measurement value of the signal intensity can be used as the measurement value of the kidney function prediction marker protein.

2-2. Method for measuring kidney function prediction marker mRNA

A known measurement method, such as a microarray method, an RNA-Seq analysis method, or a quantitative RT-PCR method, can be used to obtain the measurement value of at least one mRNA selected from the group consisting of kidney function prediction markes (hereinafter may be abbreviated as "measurement value of a kidney function prediction marker mRNA" in the present specification). As probes used for the microarray method, probes of one's choosing, or known probes, may be synthesized and used; or a commercially available microarray chip may be used.

In this method, any of total RNA and mRNA extracted from a specimen may be used. It is preferred that the specimen used for total RNA and mRNA extraction is subjected to RNA extraction immediately after being collected from an individual; or is frozen (preferably under an atmosphere at -196°C or less (rapidly cooled in liquid nitrogen)) immediately after being collected from an individual, and stored at -80°C or less until RNA extraction.

The method for extracting total RNA and mRNA from a specimen is not particularly limited, and a known extraction method may be used.

Quantification by the microarray method may be performed according to a known method. The expression level of a kidney function prediction marker mRNA may be expressed as the relative expression level to that of a housekeeping gene; or expressed as the measurement value of the signal intensity of, for example, a fluorescent dye.

Quantification by RT-PCR may be performed by conducting a reverse transcription reaction using total RNA or mRNA extracted from a specimen as a template, and performing analysis by a real-time PCR method or the like with the obtained cDNA as a template by using specific primers for a kidney function prediction marker mRNA. In this case, the expression level of the kidney function prediction marker mRNA may be expressed as the relative expression level to that of a housekeeping gene or expressed as the measurement value of the signal intensity of, for example, a fluorescent dye.

In the RNA-Seq analysis method, mRNA extracted from a specimen is fragmented, cDNA is synthesized by reverse transcription reaction using these fragments as a temperate, and libraries are prepared. The nucleotide sequence of each fragment contained in each library is determined by using a next-generation sequencer, the obtained information is mapped to a reference gene sequence, and the expression level of mRNA is represented as RPKM (Reads Per Kilobase per Million). RPKM may be represented as the intensity of a signal in, for example, a heat map.

The detection results of the signal can be used as the expression level of the kidney function prediction marker mRNA. For example, when the signal intensity is quantitatively detected, the measurement value itself of the signal intensity or a value calculated from the measurement value of the signal intensity can be used as the expression level of the kidney function prediction marker mRNA.

Examples of the value calculated from the measurement value of the signal intensity include a value obtained by subtracting, from the measurement value of the signal intensity, the measurement value of the signal intensity of a negative control sample; a value obtained by dividing the measurement value of the signal intensity by the measurement value of the signal intensity of a positive control sample; a combination thereof; and the like. Examples of negative control samples include specimens of healthy subjects and the like. Examples of positive control samples include specimens containing the kidney function prediction marker mRNA at a predetermined expression level.

3. Evaluation of function of kidney function prediction marker protein

In the present invention, the method for evaluating function of a kidney function prediction marker protein is not particularly limited, as long as the function of the kidney function prediction marker protein can be evaluated. "Function of a kidney function prediction marker protein" as used herein is the original function of a kidney function prediction marker protein. For example, when the kidney function prediction marker protein is a receptor, the function is a function to activate when its ligand binds to the kidney function prediction marker protein, transmit a signal of the activation to a protein or a chemical mediator downstream in the signaling pathway to which the protein belongs, and act on cells etc. through the signaling pathway under the control of the receptor. When the kidney function prediction marker protein is a ligand, the function is a function to activate a receptor to which the ligand binds.

An example of the method for evaluating function of a kidney function prediction marker protein is a method in which the presence or absence of, for example, phosphorylation or dephosphorylation of a protein downstream in the signaling pathway to which a kidney function prediction marker protein belongs; an increase or decrease in the expression level of a protein located downstream; activation or inactivation of the transcriptional regulatory region of a protein located downstream; or the like, is detected. For example, the presence or absence of phosphorylation of a protein can be detected by a known method, such as the Western blotting method. For example, an increase or decrease in the expression level of a protein can be detected by a known method, such as the ELISA method, Western blotting method, quantitative RT-PCR method, or RNA-Seq method. Further, activation or inactivation of the transcriptional regulatory region can be detected by a reporter assay. Examples of reporters include firefly luciferase, Renilla luciferase, GFP (Green Fluorescent Protein), β-galactosidase, and the like. The reporter assay can be performed according to a known method.

As another method for evaluating function of a kidney function prediction marker protein, the function of a kidney function prediction marker protein can be evaluated by measuring the amount of a chemical mediator (e.g., inositol trisphosphate, cAMP, cGMP, $Ca^{2+}$) downstream in the signaling pathway to which the kidney function prediction marker protein belongs. Such a chemical mediator can be measured according to a known method.

The detection results obtained by using the ELISA method, Western blotting method, quantitative RT-PCR method, RNA-Seq method, and reporter assay, and the measurement results of a chemical mediator, can be used as evaluation results of function of the kidney function prediction marker protein. The evaluation results may be quantitative data, semi-quantitative information such as "high" and "low," or qualitative data such as "present" and "not present."

4. System configuration for screening

In the present invention, "I. 5. Screening 1" described later is performed using a measurement value obtained in the above "I. 2. Method for obtaining each measurement value." Moreover, in the present invention, "I. 6. Screening 2" described later is performed using an evaluation result obtained in the above "I. 3. Evaluation of function of kidney function prediction marker protein." First, system configurations for performing these processes are described.

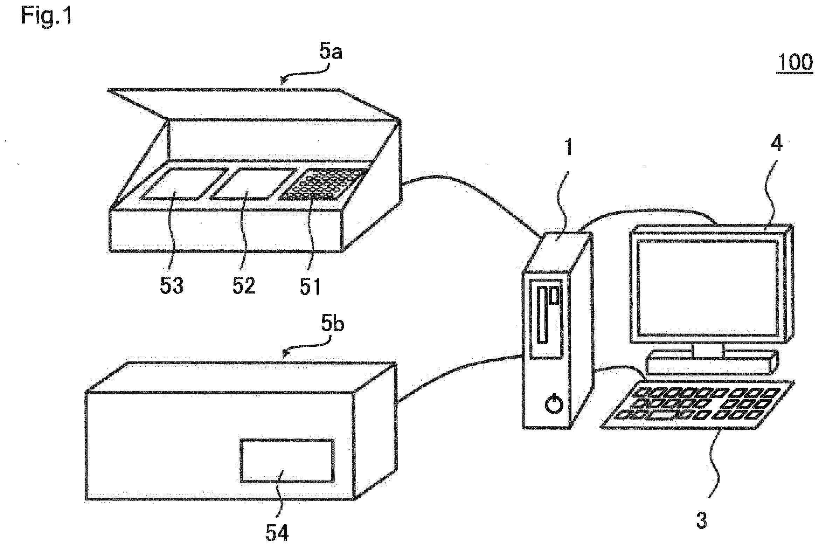

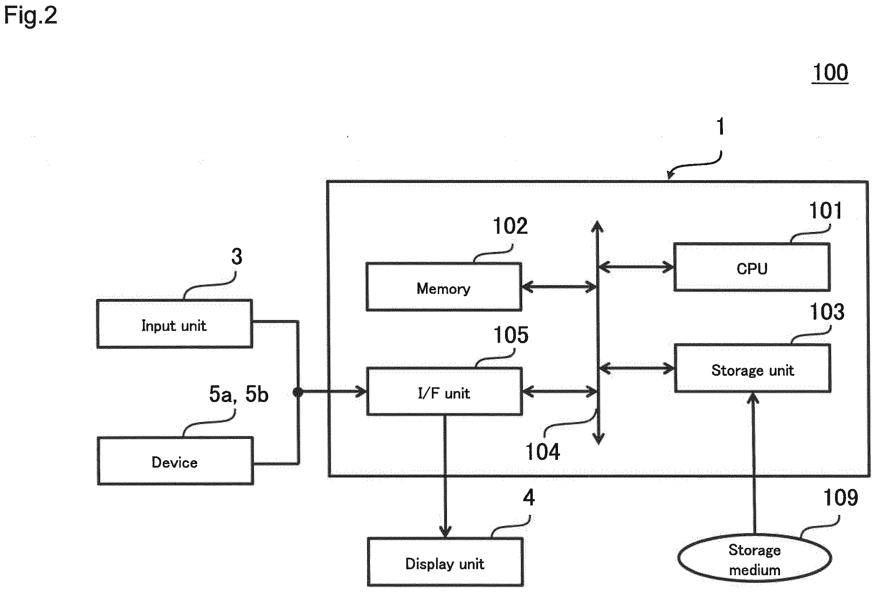

Fig. 1 is an overview of a system 100 for performing screening 1 according to a first embodiment of the present invention. Fig. 2 is a block diagram illustrating a hardware configuration of the system 100. As an embodiment, the system 100 comprises a screening device 1, an input unit 3, a display unit 4, and a device 5a or a device 5b.

The screening device 1 includes, for example, a general-purpose personal computer, and comprises a CPU 101 for performing data processing described later, a memory 102 serving as a work area for data processing, a storage unit 103 for storing processed data, a bus 104 for transmitting data between the units, and an interface unit 105 (hereinafter referred to as "I/F unit") for performing data input and output between the screening device and external devices. The input unit 3 and the display unit 4 are connected to the screening device 1. The input unit 3 includes, for example, a keyboard; and the display unit 4 includes, for example, a liquid crystal display. The input unit 3 and the display unit 4 may be integrated and implemented as a display with a touch panel. The screening device 1 need not be a single device; and the CPU 101, the memory 102, the storage unit 103, and the like may be located in separate places, and connected via a network. The screening device may also be a device that omits the input unit 3 and the display unit 4, and that does not require an operator.

The screening device 1 and the device 5a or the device 5b are also not necessarily located in one place, and may be configured such that the devices located in separate places are communicatively connected to each other via a network.

In the explanation below, a process performed by the screening device 1 means a process performed by the CPU 101 of the screening device 1 based on a screening program stored in the storage unit 103 or the memory 102 shown in Fig. 2, unless otherwise specified. The CPU 101 temporarily stores necessary data (such as intermediate data being processed) in the memory 102 that serves as a work area, and suitably stores data that is stored for a long period of time, such as computation results, in the storage unit 103.

The device 5a is a device for measuring the amount or concentration of a protein, and comprises a sample placement area 51, a reaction unit 52, and a detection unit 53. A specimen, collected from a subject, set in the sample placement area 51 is dispensed into and incubated in a microplate that is placed in the reaction unit 52 and on which an antibody against a kidney function prediction marker for antigen capture is immobilized. The unreacted antigen is removed, if necessary. Thereafter, a detection antibody is dispensed into the microplate, followed by incubation. The unreacted antigen is removed if necessary, and a substrate for detecting the detection antibody is dispensed into the microplate. The microplate is transferred to the detection unit 53, and a signal generated by reaction with the substrate is measured. Another embodiment of the device 5a is a device for measuring the expression level of mRNA by microarray analysis. A reverse transcription reaction product set in the sample placement area 51 is dispensed into a microarray chip set in the reaction unit 52, followed by hybridization. After the microarray chip is washed, it is transferred to the detection unit 53, and a signal is measured.

Further, another embodiment of the device 5a is a device for measuring the expression level of mRNA by RT-PCR. A reverse transcription reaction product set in the sample placement area 51 is dispensed into a microtube set in the reaction unit 52, and a reagent for quantitative PCR is subsequently dispensed into the microtube. A signal in the tube is detected by the detection unit 53 while performing a PCR reaction in the reaction unit 52.

The device 5b is a device for measuring the expression level of mRNA by the RNA-Seq method, and comprises a sequence analysis unit 54. A sample subjected to a reaction for RNA-Seq is set in the sequence analysis unit 54, and analysis of nucleotide sequences is performed in the sequence analysis unit 54.

The devices 5a or 5b are connected to the screening device 1 by a wired or wireless connection. The device 5a A/D converts the measurement value of a protein or the measurement value of mRNA, and transmits it as digital data to the screening device 1. Similarly, the device 5b A/D converts the measurement value of mRNA, and transmits it as digital data to the screening device 1. Therefore, the screening device 1 can obtain, as digital data that can be computed, the measurement value of a protein or the measurement value of mRNA.

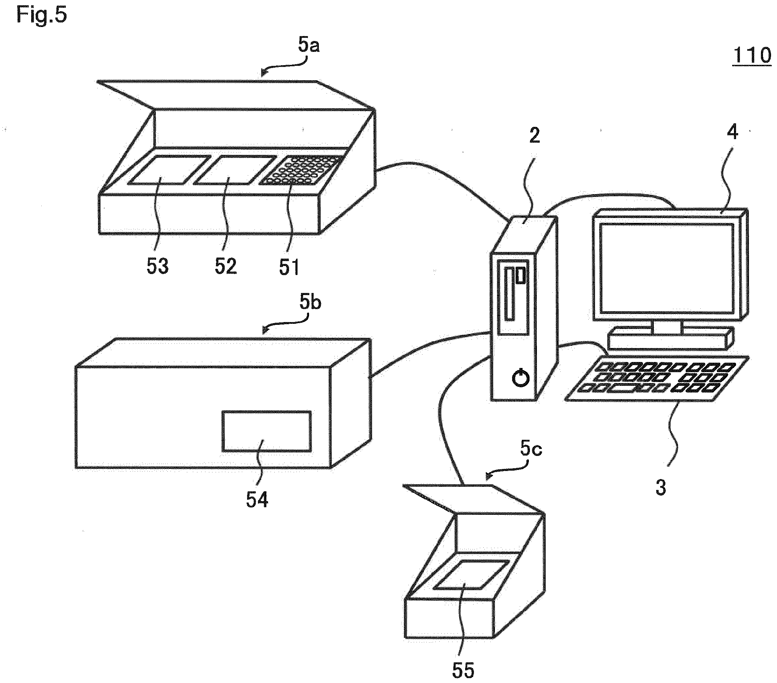

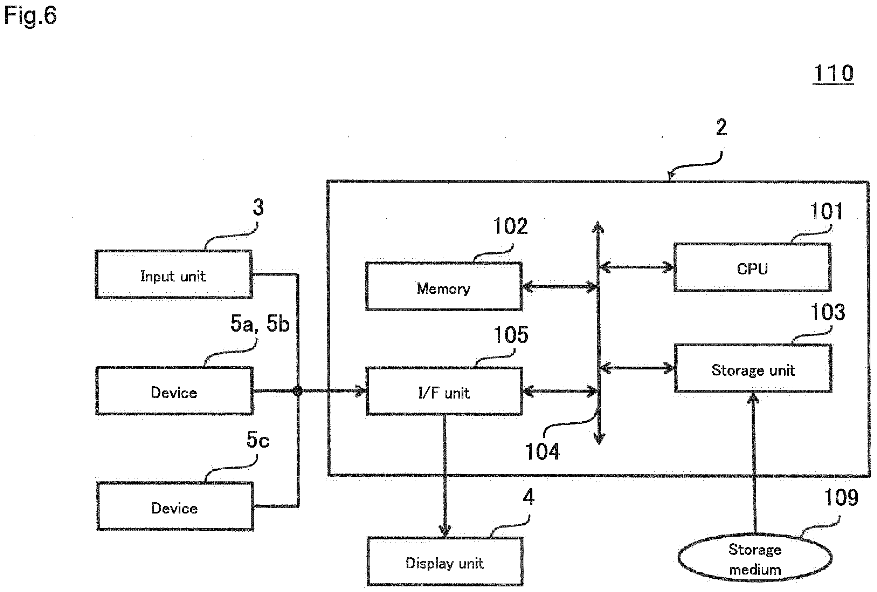

Fig. 5 is an overview of a system 110 for performing screening 2 according to a second embodiment of the present invention. Fig. 6 is a block diagram illustrating a hardware configuration of the system 110. As an embodiment, the system 110 comprises a screening device 2, an input unit 3, a display unit 4, and a device 5a, a device 5b, or a device 5c.

The screening device 2 includes, for example, a general-purpose personal computer, and comprises a CPU 101 for performing data processing described later, a memory 102 serving as a work area for data processing, a storage unit 103 for storing processed data, a bus 104 for transmitting data between the units, and an interface unit 105 (hereinafter referred to as "I/F unit") for performing data input and output between the screening device and external devices. The input unit 3 and the display unit 4 are connected to screening device 2. The input unit 3 includes, for example, a keyboard; and the display unit 4 includes, for example, a liquid crystal display. The input unit 3 and the display unit 4 may be integrated and implemented as a display with a touch panel. The screening device 2 need not be a single device; and the CPU 101, the memory 102, the storage unit 103, and the like may be located in separate places, and connected via a network. The screening device may also be a device that omits the input unit 3 and the display unit 4, and that does not require an operator.

The screening device 2 and the device 5a, the device 5b, or the device 5c are also not necessarily located in one place, and may be configured such that the devices located in separate places are communicatively connected to each other via a network.

In the explanation below, a process performed by the screening device 2 means a process performed by the CPU 101 of the screening device 2 based on a screening program stored in the storage unit 103 or the memory 102 shown in Fig. 6, unless otherwise specified. The CPU 101 temporarily stores necessary data (such as intermediate data being processed) in the memory 102 that serves as a work area, and suitably stores data that is stored for a long period of time, such as computation results, in the storage unit 103.

The device 5a is a device for measuring the amount or concentration of a protein, and comprises a sample placement area 51, a reaction unit 52, and a detection unit 53. A specimen, collected from a subject, set in the sample placement area 51 is dispensed into and incubated in a microplate that is placed in the reaction unit 52, and on which an antibody against a kidney function prediction marker for antigen capture is immobilized. The unreacted antigen is removed, if necessary. Thereafter, a detection antibody is dispensed into the microplate, followed by incubation. The unreacted antigen is removed if necessary, and a substrate for detecting the detection antibody is dispensed into the microplate. The microplate is transferred to the detection unit 53, and a signal generated by reaction with the substrate is measured. Another embodiment of the device 5a is a device for measuring the expression level of mRNA by microarray analysis. A reverse transcription reaction product set in the sample placement area 51 is dispensed into a microarray chip set in the reaction unit 52, followed by hybridization. After the microarray chip is washed, it is transferred to the detection unit 53, and a signal is measured.

Further, another embodiment of the device 5a is a device for measuring the expression level of mRNA by RT-PCR. A reverse transcription reaction product set in the sample placement area 51 is dispensed into a microtube set in the reaction unit 52, and a reagent for quantitative PCR is subsequently dispensed into the microtube. A signal in the tube is detected by the detection unit 53 while performing a PCR reaction in the reaction unit 52. Further, another embodiment of the device 5a is a device for measuring a chemical mediator. A cell lysate set in the sample placement area 51 is dispensed into a microtube set in the reaction unit 52, and a reagent for measuring a chemical mediator is subsequently dispensed into the microtube. A signal in the tube is detected by the detection unit 53.

The device 5b is a device for measuring the expression level of mRNA by the RNA-Seq method, and comprises a sequence analysis unit 54. A sample subjected to a reaction for RNA-Seq is set in the sequence analysis unit 54, and analysis of nucleotide sequences is performed in the sequence analysis unit 54.

An embodiment of the device 5c is a device for detecting a signal in, for example, Western blotting. For example, when the function of a kidney function prediction marker protein is evaluated by using the Western blotting method or the like, a membrane for detection is set in the detection unit 55 of the device 5c, and chemiluminescence intensity or fluorescence intensity is measured. Another embodiment of the device 5c is a device for detecting the expression intensity of a reporter gene in a reporter assay. A cell lysate for a reporter assay is set in the detection unit 55 of the device 5c, and a liquid of a substrate is dispensed thereinto. Thereafter, chemiluminescence intensity is measured.

The devices 5a, 5b, or 5c are connected to the screening device 2 by a wired or wireless connection. The device 5a A/D converts the detection result of a protein, the detection result of mRNA, or the measurement result of a chemical mediator, and transmits it as digital data to the screening device 2. The device 5b A/D converts the detection result of mRNA, and transmits it as digital data to the screening device 2. The device 5c A/D converts the measurement result of a signal in Western blotting or the like or the measurement result of a reporter assay or the like, and transmits it as digital data to the screening device 2. Therefore, the screening device 2 can obtain, as digital data regarding the evaluation result of function of a kidney function prediction marker protein that can be computed, the detection result of a protein, the detection result of mRNA, the measurement result of a chemical mediator, the measurement result of a signal in Western blotting, or the measurement result of a reporter assay.

## 5. Screening 1

### 5-1. Outline

In this embodiment, it is determined that a test substance is a candidate substance for an active ingredient to prevent or treat at least one disease selected from the group consisting of declined kidney function, chronic kidney disease, and renal failure, by using the measurement value of a kidney function prediction marker protein and/or the measurement value of mRNA of the protein in a specimen collected from a subject, test tissue, or test cell treated with the test substance (a test-substance-treated specimen). The measurement value(s) of the test-substance-treated specimen are obtained by performing the above "I. 2. Method for obtaining each measurement value."

More specifically, this embodiment comprises the steps of:

(I) obtaining a measurement value of a kidney function prediction marker protein and/or a measurement value of mRNA of the protein in a specimen collected from a subject, test tissue, or test cell treated with a test substance (a test-substance-treated specimen); and

(II) determining that the test substance is a candidate substance for the active ingredient based on the measurement value(s) obtained in step (I).

In this case, when the measurement value of a kidney function prediction marker protein and/or the measurement value of mRNA of the protein in the test-substance-treated specimen is close to the measurement value of a corresponding kidney function prediction marker protein and/or the measurement value of mRNA of the protein in a healthy individual, it can be determined that the test substance is a candidate substance for the active ingredient.

Here, step (I) may be performed in such a manner that a measurement value is obtained by actually performing the "I. 2. Method for obtaining each measurement value," or in such a manner that the prediction device described later or the like is allowed to obtain a measurement value already obtained. In addition, step (I) and step (II) are not necessarily performed consecutively in the same organization. For example, the measurement value(s) obtained in step (I) may be sent to a third-party organization to perform step (II) and the subsequent process.

Further, in this embodiment, it may be determined that the test substance is a candidate substance for an active ingredient to prevent or treat at least one disease selected from the group consisting of declined kidney function, chronic kidney disease, and renal failure, by comparing the measurement value(s) of the test-substance-treated specimen with the measurement value of the kidney function prediction marker protein and/or the measurement value of mRNA of the protein in a specimen collected from a subject, test tissue, or test cell that is not treated with the test substance (an untreated specimen). The measurement value(s) of the untreated specimen are obtained by performing the above "I. 2. Method for obtaining each measurement value." More specifically, this embodiment may comprise, between steps (I) and (II), the step of comparing the measurement value(s) of the test-substance-treated specimen obtained in step (I) with a measurement value of a corresponding kidney function prediction marker protein and/or a measurement value of mRNA of the protein in a specimen collected from a subject, test tissue, or test cell that is not treated with the test substance (an untreated specimen). In this case, when the measurement value of the kidney function prediction marker protein and/or the measurement value of mRNA of the protein in the test-substance-treated specimen indicates a more improved result than the measurement value of the corresponding kidney function prediction marker protein and/or the measurement value of mRNA of the protein in the untreated specimen, it can be determined that the test substance is a candidate substance for the active ingredient.

Here, for example, in the case where the specimen is collected from a subject with at least one disease selected from the group consisting of declined kidney function, chronic kidney disease, and renal failure, and where the measurement value of the kidney function prediction marker protein and/or the measurement value of mRNA of the protein increases with a decline in kidney function, if the measurement value of the kidney function prediction marker protein and/or the measurement value of mRNA of the protein in the test-substance-treated specimen is lower than the measurement value of the corresponding kidney function prediction marker protein and/or the measurement value of mRNA of the protein in the untreated specimen when compared with each other, it can be determined that the at least one disease selected from the group consisting of declined kidney function, chronic kidney disease, and renal failure has been improved by the test substance. In this case, there is no particular limitation on how much lower the measurement value(s) are. When the measurement value(s) of the test-substance-treated specimen are, for example, 85% or less, preferably 70% or less, more preferably 50% or less of the measurement value(s) of the untreated specimen, it can be determined that the at least one disease selected from the group consisting of declined kidney function, chronic kidney disease, and renal failure has been improved by the test substance.

In the case where the specimen is collected from a subject with at least one disease selected from the group consisting of declined kidney function, chronic kidney disease, and renal failure, and where the measurement value of the kidney function prediction marker protein and/or the measurement value of mRNA of the protein decreases with a decline in kidney function, if the measurement value of the kidney function prediction marker protein and/or the measurement value of mRNA of the protein in the test-substance-treated specimen is higher than the measurement value of the corresponding kidney function prediction marker protein and/or the measurement value of mRNA of the protein in the untreated specimen when compared with each other, it can be determined that the at least one disease selected from the group consisting of declined kidney function, chronic kidney disease, and renal failure has been improved by the test substance. In this case, there is no particular limitation on how much higher the measurement value(s) are. When the measurement value(s) of the test-substance-treated specimen are, for example, 115% or more, preferably 130% or more, more preferably 150% or more of the measurement value(s) of the untreated specimen, it can be determined that the at least one disease selected from the group consisting of declined kidney function, chronic kidney disease, and renal failure has been improved by the test substance.

Further, the screening 1 may comprise, before step (I), the steps of (i) treating the test substance to the subject, test tissue, or test cell, (ii) collecting the specimen from the subject, test tissue, or test cell to which the test substance has been treated in step (i), and (iii) collecting the protein and/or the mRNA from the specimen obtained in step (ii). In this case, step (ii) and step (iii) are not necessarily performed consecutively in the same organization. For example, the specimen collected in step (ii) may be sent to a third-party organization to perform step (iii) and the subsequent steps. In addition, step (iii) and step (I) are also not necessarily performed consecutively in the same organization. For example, the protein and/or the mRNA collected in step (iii) may be sent to a third-party organization to perform the steps after step (iii).

5-2. Screening device

The present invention includes, as the first embodiment, a device for screening a candidate substance for an active ingredient to prevent or treat at least one disease selected from the group consisting of declined kidney function, chronic kidney disease, and renal failure, the device comprising the following computation means:

first measurement value obtaining means for obtaining a measurement value of a kidney function prediction marker protein and/or a measurement value of mRNA of the protein in a specimen collected from a subject, test tissue, or test cell treated with a test substance (a test-substance-treated specimen); and

means for determining that the test substance is a candidate substance for the active ingredient based on the measurement value(s) obtained by the first measurement value obtaining means.

Preferably, the above screening device further comprises:

second measurement value obtaining means for obtaining a measurement value of the kidney function prediction marker protein and/or a measurement value of mRNA of the protein in a specimen collected from a subject, test tissue, or test cell that is not treated with the test substance (an untreated specimen); and

means for comparing the measurement value(s) of the test-substance-treated specimen with the measurement value(s) of the untreated specimen,

wherein the determination means determines that the test substance is a candidate substance for the active ingredient based on the comparison result obtained by the measurement value comparison means. The determination method is in accordance with the description in the section "I. 5-4. Screening method" described later.

In this embodiment, a candidate substance for an active ingredient to prevent or treat at least one disease selected from the group consisting of declined kidney function, chronic kidney disease, and renal failure can be screened by the system 100 (Figs. 1 and 2) comprising the screening device 1 as the screening device described above.

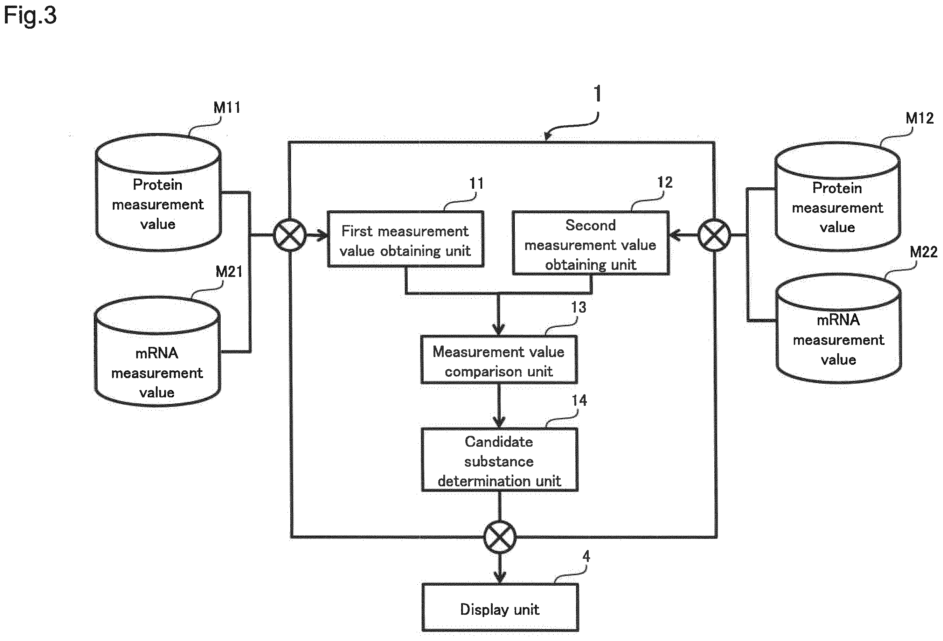

Fig. 3 is a block diagram illustrating functions of the screening device 1 according to this embodiment. The screening device 1 comprises a first measurement value obtaining unit 11, a second measurement value obtaining unit 12, a measurement value comparison unit 13, and a candidate substance determination unit 14. The second measurement value obtaining unit 12 may be optional. These functional blocks are implemented by installing the screening program according to the present invention in the storage unit 103 or the memory 102 of the screening device 1 shown in Fig. 2, and causing the CPU 101 to execute the screening program. Thereby, the screening device 1 carries out the screening method described in the section "I. 5-4. Screening method" described later. The first measurement value obtaining means, second measurement value obtaining means, measurement value comparison means, and determination means recited in the claims correspond to the first measurement value obtaining unit 11, second measurement value obtaining unit 12, measurement value comparison unit 13, and candidate substance determination unit 14 shown in Fig. 3, respectively.

In other words, the screening device 1 is a device for screening a candidate substance for an active ingredient to prevent or treat at least one disease selected from the group consisting of declined kidney function, chronic kidney disease, and renal failure, the device executing the following computation functions by the CPU 101:

a first measurement value obtaining function for obtaining a measurement value of a kidney function prediction marker protein and/or a measurement value of mRNA of the protein in a specimen collected from a subject, test tissue, or test cell treated with a test substance (a test-substance-treated specimen); and

a function for determining that the test substance is a candidate substance for the active ingredient based on the measurement value(s) obtained by the first measurement value obtaining function.

Preferably, the screening device 1 further executes the following functions by the CPU 101:

a second measurement value obtaining function for obtaining a measurement value of the kidney function prediction marker protein and/or a measurement value of mRNA of the protein in a specimen collected from a subject, test tissue, or test cell that is not treated with the test substance (an untreated specimen); and

a function for comparing the measurement value(s) of the test-substance-treated specimen with the measurement value(s) of the untreated specimen,

wherein the determination function determines that the test substance is a candidate substance for the active ingredient based on the comparison result obtained by the measurement value comparison function.

In this embodiment, a measurement value M11 of a kidney function prediction marker protein in a test-substance-treated specimen is put into the screening device 1 from the device 5a, and a measurement value M21 of mRNA of the protein is put into the screening device 1 from the device 5a or 5b. Similarly, a measurement value M12 of the kidney function prediction marker protein in an untreated specimen is also put into the screening device 1 from the device 5a, and a measurement value M22 of mRNA of the protein is also put into the screening device 1 from the device 5a or 5b.

The measurement values M11 and M12 of the kidney function prediction marker protein and the measurement values M21 and M22 of mRNA of the protein in the test-substance-treated specimen and the untreated specimen may also be put into the screening device 1 from a third-party organization (not shown) via a network.

Moreover, the functional blocks, i.e., the first measurement value obtaining unit 11, the second measurement value obtaining unit 12, the measurement value comparison unit 13, and the candidate substance determination unit 14, are not necessarily executed by a single CPU, and may be processed by multiple CPUs in a distributed manner. For example, these functional blocks may be configured such that the functions of the first measurement value obtaining unit 11 and the second measurement value obtaining unit 12 are executed by a CPU of a first computer, and such that the functions of the measurement value comparison unit 13 and the candidate substance determination unit 14 are executed by a CPU of a second computer, i.e., another computer.

5-3. Screening program

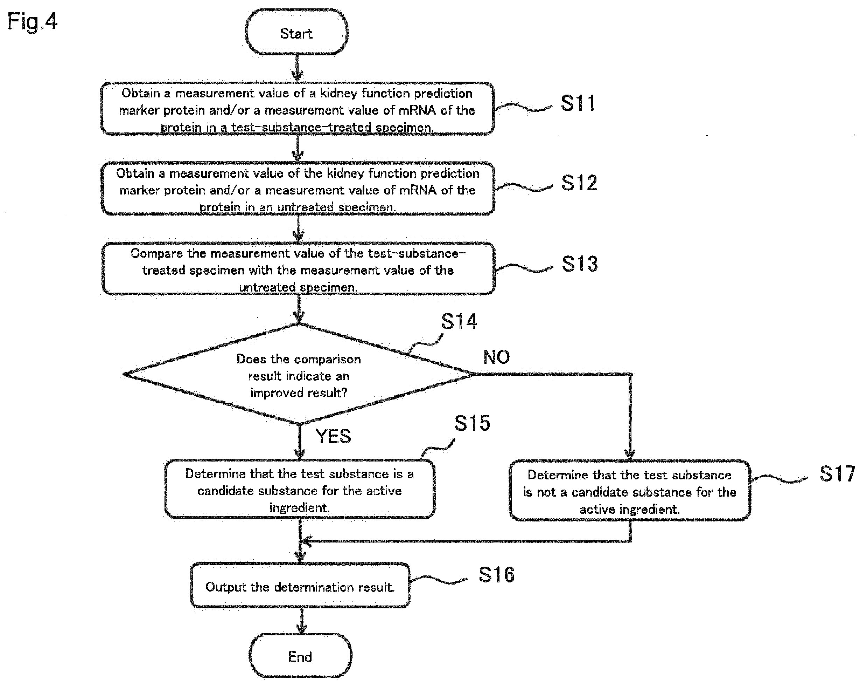

In order to carry out the processing for steps S11 to S17 in Fig. 4 below, the screening device 1 according to the first embodiment of the present invention stores the screening program according to this embodiment in the storage unit 103 beforehand, for example, in an executable format (for example, a form in which the program can be produced by conversion from a programming language using a compiler). The screening device 1 carries out the processing using the screening program stored in the storage unit 103.

Specifically, the screening program according to the first embodiment of the present invention is a screening program that, when executed by a computer, causes the computer to carry out the following processing to screen a candidate substance for an active ingredient to prevent or treat at least one disease selected from the group consisting of declined kidney function, chronic kidney disease, and renal failure:

first measurement value obtaining processing of obtaining a measurement value of a kidney function prediction marker protein and/or a measurement value of mRNA of the protein in a specimen collected from a subject, test tissue, or test cell treated with a test substance (a test-substance-treated specimen); and
processing of determining that the test substance is a candidate substance for the active ingredient based on the measurement value(s) obtained by the first measurement value obtaining processing.

Preferably, the screening program further causes the computer to carry out second measurement value obtaining processing of obtaining a measurement value of the kidney function prediction marker protein and/or a measurement value of mRNA of the protein in a specimen collected from a subject, test tissue, or test cell that is not treated with the test substance (an untreated specimen); and, in the determination processing, it is determined that the test substance is a candidate substance for the active ingredient, by comparing the measurement value(s) obtained by the first measurement value obtaining processing with the measurement value(s) obtained by the second measurement value obtaining processing. The determination method is in accordance with the description in the section "I. 5-4. Screening method" described later.

In this embodiment, as shown in Fig. 2, the screening program is stored in a computer-readable non-transitory tangible storage medium 109, such as a CD-ROM, and is installed in the screening device 1 from the storage medium 109; alternatively, the screening device 1 may be connected to the internet (not shown) to download the program code of the screening program via the internet.

5-4. Screening method

The screening device 1 according to the first embodiment of the present invention carries out the screening method according to the first embodiment of the present invention. The screening method according to the first embodiment of the present invention includes a method for screening a candidate substance for an active ingredient to prevent or treat at least one disease selected from the group consisting of declined kidney function, chronic kidney disease, and renal failure, the method comprising the steps of:

(I) obtaining a measurement value of a kidney function prediction marker protein and/or a measurement value of mRNA of the protein in a specimen collected from a subject, test tissue, or test cell treated with a test substance (a test-substance-treated specimen); and
(II) determining that the test substance is a candidate substance for the active ingredient based on the measurement value(s) obtained in step (I).

Preferably, the above screen method further comprises, between steps (I) and (II), the step of comparing the measurement value(s) of the test-substance-treated specimen obtained in step (I) with a measurement value of a corresponding kidney function prediction marker protein and/or a measurement value of mRNA of the protein in a specimen collected from a subject, test tissue, or test cell that is not treated with the test substance (an untreated specimen).

Fig. 4 is a flow chart illustrating a flow of data processing performed by the screening device 1 according to the first embodiment of the present invention to carry out the screening method. Steps S11, S12, and S13 are performed by the first measurement value obtaining unit 11, second measurement value obtaining unit 12, and measurement value comparison unit 13 shown in Fig. 3, respectively. Steps S14 to S17 are performed by the candidate substance determination unit 14 shown in Fig. 3.

In step S11, the first measurement value obtaining unit 11 obtains a measurement value M11 of a protein and/or a measurement value M21 of mRNA in a test-substance-treated specimen.

In step S12, the second measurement value obtaining unit 12 obtains a measurement value M12 of the protein and/or a measurement value M22 of the mRNA in an untreated specimen.

In step S13, the measurement value comparison unit 13 compares the measurement value(s) of the test-substance-treated specimen obtained in step S11 with the measurement value(s) of the untreated specimen obtained in step S12. The comparison result is output to the candidate substance determination unit 14.

The candidate substance determination unit 14 determines that the test substance is a candidate substance for the active ingredient based on the comparison result obtained by the measurement value comparison unit 13. More specifically, when the comparison result indicates an improved result (YES in step S14), the candidate substance determination unit 14 determines, in step S15, that the test substance is a candidate substance for the active ingredient.

More specifically, in the case where the test-substance-treated specimen is collected from a subject with at least one disease selected from the group consisting of declined kidney function, chronic kidney disease, and renal failure, and where the measurement value of the kidney function prediction marker protein and/or the measurement value of mRNA of the protein increases with a decline in kidney function, when a value obtained by dividing M11 by M12 or a value obtained by dividing M21 by M22 is, for example, 0.85 or less, preferably 0.7, and more preferably 0.5 or less, the measurement value comparison unit 13 determines that the at least one disease selected from the group consisting of declined kidney function, chronic kidney disease, and renal failure has been improved by the test substance, and outputs a comparison result such that the disease has been improved. When the comparison result indicates that the disease has been improved, the candidate substance determination unit 14 determines that the test substance is a candidate substance for the active ingredient. In the case where the test-substance-treated specimen is collected from a subject with at least one disease selected from the group consisting of declined kidney function, chronic kidney disease, and renal failure, and where the measurement value of the kidney function prediction marker protein and/or the measurement value of mRNA of the protein decreases with a decline in kidney function, when a value obtained by dividing M11 by M12 or a value obtained by dividing M21 by M22 is, for example, 1.15 or more, preferably 1.3 or more, and more preferably 1.5 or more, the measurement value comparison unit 13 determines that the at least one disease selected from the group consisting of declined kidney function, chronic kidney disease, and renal failure has been improved by the test substance, and outputs a comparison result such that the disease has been improved. When determining that the at least one disease selected from the group consisting of declined kidney function, chronic kidney disease, and renal failure has not been improved by the test substance, the measurement value comparison unit 13 outputs a comparison result such that the disease has not been improved. When the comparison result indicates that the disease has been improved, the candidate substance determination unit 14 determines that the test substance is a candidate substance for the active ingredient.

In step S16, the candidate substance determination unit 14 outputs the result determined in step S15. In this embodiment, the candidate substance for the active ingredient is displayed on the display unit 4, and the determination result is stored in the storage unit 103 in the screening device 1. The determination result may be displayed on a display unit of an external computer terminal connected to the screening device 1 via the internet, for example, a display unit of a computer terminal in a third-party organization (not shown), instead of displaying the determination result on the display unit 4.

In step S14, when the comparison result indicates that the disease has not been improved, the processing proceeds to step S17, and the candidate substance determination unit 14 determines that the test substance is not the active ingredient. In this case, the result such that the test substance is not the active ingredient may be displayed on the display unit 4.

## 6. Screening 2

### 6-1. Outline

In this embodiment, it is determined that a test substance is a candidate substance for an active ingredient to prevent or treat at least one disease selected from the group consisting of declined kidney function, chronic kidney disease, and renal failure, using an evaluation result of function of a kidney function prediction marker protein in a test-substance-treated specimen obtained by performing a method described in the above section "1.3. Evaluation of function of kidney function prediction marker protein." More specifically, this embodiment comprises the following steps: (I) evaluating function of a kidney function prediction marker protein in a specimen collected from a subject, test tissue, or test cell treated with a test substance (a test-substance-treated specimen); and (II) determining that the test substance is a candidate substance for the active ingredient based on the evaluation result obtained in step (I). In this case, when the evaluation result of function of the kidney function prediction marker protein in the test-substance-treated specimen is closed to an evaluation result of function of a corresponding kidney function prediction marker protein in a healthy individual, it can be determined that the test substance is a candidate substance for the active ingredient.

Here, step (I) may be performed in such a manner that an evaluation result is obtained by actually performing "I. 3. Evaluation of function of kidney function prediction marker protein," or in such a manner that the prediction device described later or the like is allowed to obtain an evaluation result already obtained. In addition, step (I) and determination step (II) are not necessarily performed consecutively in the same organization. For example, the evaluation result obtained in step (I) may be sent to a third-party organization to perform step (II) and the subsequent process.

Further, in this embodiment, the above evaluation result can be compared with an evaluation result of function of the kidney function prediction marker protein in an untreated specimen obtained by the method described in the above section "I. 3. Evaluation of function of kidney function prediction marker protein," and it can be determined that the test substance is a candidate substance for an active ingredient to prevent or treat at least one disease selected from the group consisting of declined kidney function, chronic kidney disease, and renal failure. More specifically, this embodiment further comprises, between steps (I) and (II), the step of comparing the evaluation result of the test-substance-treated specimen obtained in step (I) with an evaluation result of function of a corresponding kidney function prediction marker protein in an untreated specimen. In this case, when the evaluation result of function of the kidney function prediction marker protein of the test-substance-treated specimen indicates a more improved result than the evaluation result of function of the corresponding kidney function prediction marker protein of the untreated specimen, it can be determined that the test substance is a candidate substance for the active ingredient.

For example, in the case where the specimen is collected from a subject with at least one disease selected from the group consisting of declined kidney function, chronic kidney disease, and renal failure, and where the function of the kidney function prediction marker protein is activated as kidney function declines, when the evaluation result of function of the kidney function prediction marker protein in the test-substance-treated specimen shows a lower result than the evaluation result of function of the corresponding kidney function prediction marker protein in the untreated specimen when compared with each other, it can be determined that the at least one disease selected from the group consisting of declined kidney function, chronic kidney disease, and renal failure has been improved by the test substance. In this case, there is no particular limitation on how much lower the evaluation result is. When the measurement value of the test-substance-treated specimen is, for example, 85% or less, preferably 70% or less, more preferably 50% or less of the evaluation result of the untreated specimen, it can be determined that the at least one disease selected from the group consisting of declined kidney function, chronic kidney disease, and renal failure has been improved by the test substance.

In the case where the specimen is collected from a subject with at least one disease selected from the group consisting of declined kidney function, chronic kidney disease, and renal failure, and where the function of the kidney function prediction marker protein is suppressed as kidney function declines, when the evaluation result of function of the kidney function prediction marker protein in the test-substance-treated specimen shows a higher result than the evaluation result of function of the corresponding kidney function prediction marker protein in the untreated specimen when compared with each other, it can be determined that the at least one disease selected from the group consisting of declined kidney function, chronic kidney disease, and renal failure has been improved by the test substance. In this case, there is no particular limitation on how much higher the evaluation result is. When the measurement value of the test-substance-treated specimen is, for example, 115% or more, preferably 130% or more, more preferably 150% or more of the evaluation result of the untreated specimen, it can be determined that the at least one disease selected from the group consisting of declined kidney function, chronic kidney disease, and renal failure has been improved by the test substance.

Further, the screening 2 may comprises, before step (I), the steps of (i) treating the test substance to the subject, test tissue, or test cell; and (ii) collecting the specimen from the subject, test tissue, or test cell after step (i). In this case, step (i) and step (I) are not necessarily performed consecutively in the same organization. For example, the specimen collected in step (ii) may be sent to a third-party organization to perform step (I) and the subsequent process.

## 6-2. Screening device

The present invention includes, as the second embodiment, a device for screening a candidate substance for an active ingredient to prevent or treat at least one disease selected from the group consisting of declined kidney function, chronic kidney disease, and renal failure, the device comprising the following computation means:

first evaluation result obtaining means for obtaining an evaluation result of function of a kidney function prediction marker protein in a specimen collected from a subject, test tissue, or test cell treated with a test substance (a test-substance-treated specimen); and
means for determining that the test substance is a candidate substance for the active ingredient based on the evaluation result obtained by the first evaluation result obtaining means.

Preferably, the screening device further comprising:

second evaluation result obtaining means for obtaining an evaluation result of function of the kidney function prediction marker protein in a specimen collected from a subject, test tissue, or test cell that is not treated with the test substance (an untreated specimen); and

means for comparing the evaluation result of the test-substance-treated specimen with the evaluation result of the untreated specimen,

wherein the determination means determines that the test substance is a candidate substance for the active ingredient based on the comparison result obtained by the evaluation result comparison means. The determination method is in accordance with the description in the section "I. 6-4. Screening method" described later.

In this embodiment, a candidate substance for an active ingredient to prevent or treat at least one disease selected from the group consisting of declined kidney function, chronic kidney disease, and renal failure can be screened by the system 110 (Figs. 5 and 6) comprising the screening device 2 described above.

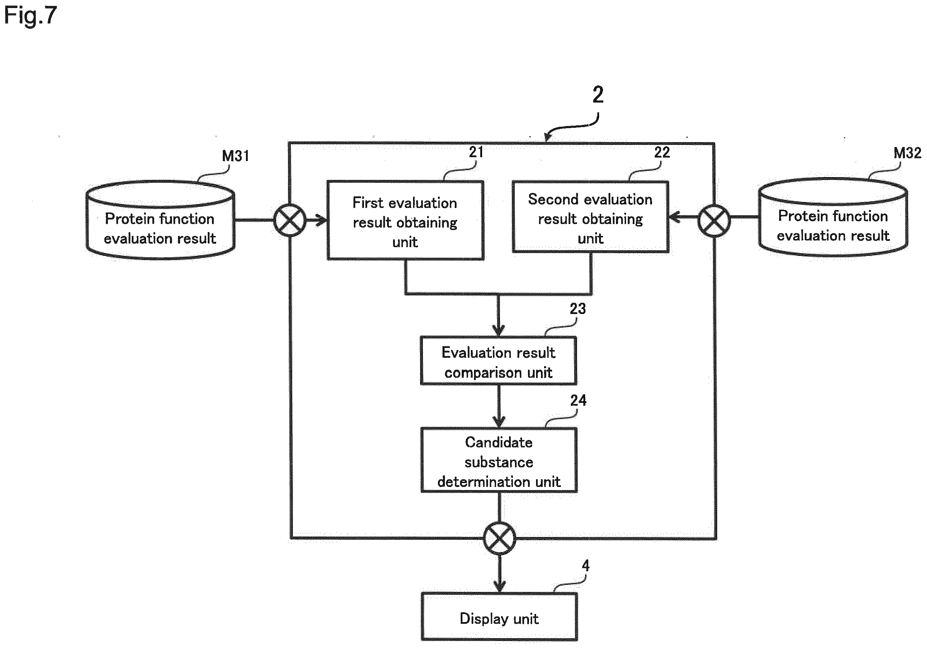

Fig. 7 is a block diagram illustrating functions of the screening device 2 according to the second embodiment of the present invention. The screening device 2 comprises a first evaluation result obtaining unit 21, a second evaluation result obtaining unit 22, an evaluation result comparison unit 23, and a candidate substance determination unit 24. The second evaluation result obtaining unit 22 may be optional. These functional blocks are implemented by installing the screening program according to the present invention in the storage unit 103 or the memory 102 of the screening device 2 shown in Fig. 6, and causing the CPU 101 to execute the screening program. Thereby, the screening device 2 carries out the screening method described in the "I. 6-4. Screening method" described later. The first evaluation result obtaining means, second evaluation result obtaining means, evaluation result comparison means, and determination means recited in the claims correspond to the first evaluation result obtaining unit 21, second evaluation result obtaining unit 22, evaluation result comparison unit 23, and candidate substance determination unit 24 shown in Fig. 7, respectively.

In other words, the screening device 2 is a device for screening a candidate substance for an active ingredient to prevent or treat at least one disease selected from the group consisting of declined kidney function, chronic kidney disease, and renal failure, the device executing the following computation functions by the CPU 101:

a first evaluation result obtaining function for obtaining an evaluation result of function of a kidney function prediction marker protein in a specimen collected from a subject, test tissue, or test cell treated with a test substance (a test-substance-treated specimen); and

a function for determining that the test substance is a candidate substance for the active ingredient based on the evaluation result obtained by the first evaluation result obtaining function.

Preferably, the screening device 2 further executing the following functions by the CPU 101:

a second evaluation result obtaining function for obtaining an evaluation result of function of the kidney function prediction marker protein in a specimen collected from a subject, test tissue, or test cell that is not treated with the test substance (an untreated specimen); and

a function for comparing the evaluation result of the test-substance-treated specimen with the evaluation result of the untreated specimen,

wherein the determination function determines that the test substance is a candidate substance for the active ingredient based on the comparison result obtained by the evaluation result comparison function. The determination method is in accordance with the description in the section "I. 6-4. Screening method" described later.

In this embodiment, an evaluation result M31 of function of a kidney function prediction marker protein in a test-substance-treated specimen is put into the screening device 2 from the device 5a, 5b, or 5c. Similarly, an evaluation result M32 of the kidney function prediction marker protein in an untreated specimen is also put into the screening device 2 from the device 5a, 5b, or 5c.

The evaluation results M31 and M32 of function of the kidney function prediction marker protein in the test-substance-treated specimen and the untreated specimen may also be put into the screening device from a third-party organization (not shown) via a network.

Moreover, the functional blocks, i.e., the first evaluation result obtaining unit 21, the second evaluation result obtaining unit 22, the evaluation result comparison unit 23, and the candidate substance determination unit 24, are not necessarily executed by a single CPU, and may be processed by multiple CPUs in a distributed manner. For example, these functional blocks may be configured such that the functions of the first evaluation result obtaining unit 21 and the second evaluation result obtaining unit 22 are executed by a CPU of a first computer, and such that the functions of the evaluation result comparison unit 23 and the candidate substance determination unit 24 are executed by a CPU of a second computer, i.e., another computer.

6-3. Screening program

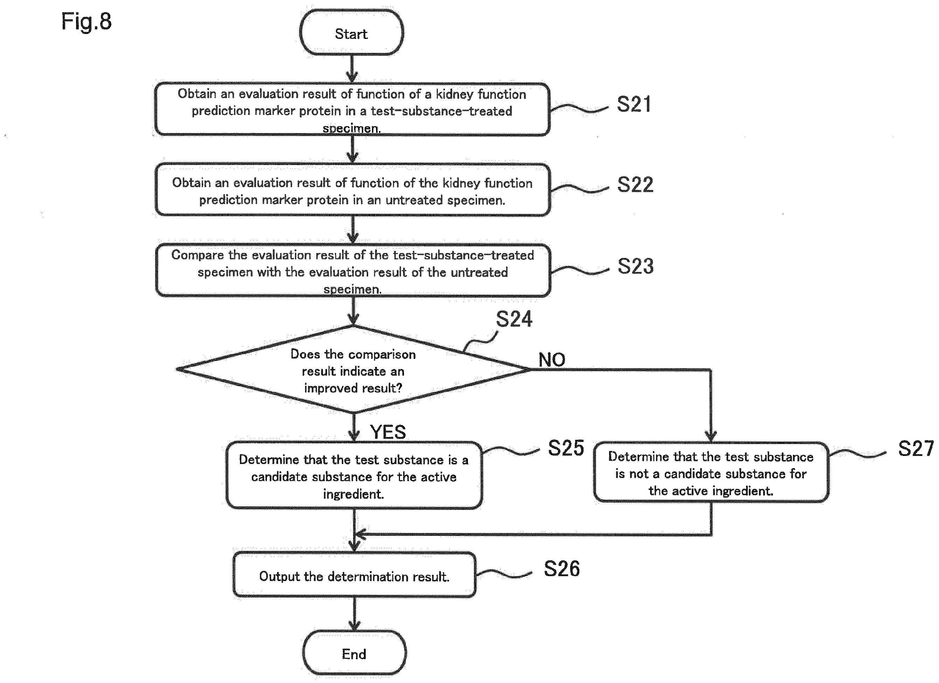

In order to carry out the processing for steps S21 to S27 in Fig. 8 below, the screening device 2 according to the second embodiment of the present invention stores the screening program according to this embodiment in the storage unit 103 beforehand, for example, in an executable format (for example, a form in which the program can be produced by conversion from a programming language using a compiler). The screening device 2 carries out the processing using the screening program stored in the storage unit 103.

Specifically, the screening program according to the second embodiment of the present invention is a screening program that, when executed by a computer, causes the computer to carry out the following processing to screen a candidate substance for an active ingredient to prevent or treat at least one disease selected from the group consisting of declined kidney function, chronic kidney disease, and renal failure:

first evaluation result obtaining processing of obtaining an evaluation result of function of a kidney function prediction marker protein in a specimen collected from a subject, test tissue, or test cell treated with a test substance (a test-substance-treated specimen); and

processing of determining that the test substance is a candidate substance for the active ingredient based on the evaluation result obtained by the first evaluation result obtaining processing.

Preferably, the screening program further causes the computer to carry out second evaluation result obtaining processing of obtaining an evaluation result of function of the kidney function prediction marker protein in a specimen collected from a subject, test tissue, or test cell that is not treated with the test substance (an untreated specimen); and processing of comparing the evaluation result of the test-substance-treated specimen with the evaluation result of the untreated specimen, and

in the determination processing, it is determined that the test substance is a candidate substance for the active ingredient based on the comparison result obtained by the evaluation result comparison processing. The determination method is in accordance with the description in the section "I. 6-4. Screening method" described later.

In this embodiment, as shown in Fig. 6, the screening program is stored in a computer-readable non-transitory tangible storage medium 109, such as a CD-ROM, and is installed in the screening device 2 from the storage medium 109; alternatively, the screening device 2 may be connected to the internet (not shown) to download the program code of the screening program via the internet.

6-4. Screening method

The screening device 2 according to the second embodiment of the present invention carries out the screening method according to the second embodiment of the present invention. The screening method according to the second embodiment of the present invention includes a method for screening a candidate substance for an active ingredient to prevent or treat at least one disease selected from the group consisting of declined kidney function, chronic kidney disease, and renal failure, the method comprising the steps of:

(I) evaluating function of a kidney function prediction marker protein in a specimen collected from a subject, test tissue, or test cell treated with a test substance (a test-substance-treated specimen); and

(II) determining that the test substance is a candidate substance for the active ingredient based on the evaluation result obtained in step (I).

Preferably, the screening method further comprises, between steps (I) and (II), the step of comparing the evaluation result of the test-substance-treated specimen obtained in step (I) with an evaluation result of function of a corresponding kidney function prediction marker protein in a specimen collected from a subject, test tissue, or test cell that is not treated with the test substance (an untreated specimen).

Fig. 8 is a flow chart illustrating a flow of data processing performed by the screening device 2 according to the second embodiment of the present invention to carry out the method described above. Steps S21, S22, and S23 are performed by the first evaluation result obtaining unit 21, second evaluation result obtaining unit 22, and evaluation result comparison unit 23 shown in Fig. 7, respectively. Steps S24 to S27 are performed by the candidate substance determination unit 24 shown in Fig. 7.

In step S21, the first evaluation result obtaining unit 21 obtains an evaluation result M31 of function of a protein in a test-substance-treated specimen.

In step S22, the second evaluation result obtaining unit 22 obtains an evaluation result M32 of function of the protein in an untreated specimen.

In step S23, the evaluation result comparison unit 23 compares the evaluation result of the test-substance-treated specimen obtained in step S21 with the evaluation result of the untreated specimen obtained in step S22. The comparison result is output to the candidate substance determination unit 24.

The candidate substance determination unit 24 determines a candidate substance for the active ingredient based on the comparison result obtained by the evaluation result comparison unit 23. Specifically, when the comparison result indicates an improved result (YES in step S24), the candidate substance determination unit 24 determines, in step S25, that the test substance is a candidate substance for the active ingredient.

More specifically, in the case where the test-substance-treated specimen is collected from a subject with at least one disease selected from the group consisting of declined kidney function, chronic kidney disease, and renal failure, and where the function of the kidney function prediction marker protein is activated as kidney function declines, when a value obtained by dividing M31 by M32 is, for example, 0.85 or less, preferably 0.7 or less, more preferably 0.5 or less, the evaluation result comparison unit 23 determines that the at least one disease selected from the group consisting of declined kidney function, chronic kidney disease, and renal failure has been improved by the test substance, and outputs a comparison result such that the disease has been improved. When the comparison result indicates that the disease has been improved, the candidate substance determination unit 24 determines that the test substance is a candidate substance for the active ingredient. In the case where the test-substance-treated specimen is collected from a subject with at least one disease selected from the group consisting of declined kidney function, chronic kidney disease, and renal failure, and where the function of the kidney function prediction marker protein is suppressed as kidney function declines, when a value obtained by dividing M31 by M32 is, for example, 1.15 or more, preferably 1.3 or more, more preferably 1.5 or more, the evaluation result comparison unit 23 determines that the at least one disease selected from the group consisting of declined kidney function, chronic kidney disease, and renal failure has been improved by the test substance, and outputs a comparison result such that the disease has been improved. When determining that the at least one disease selected from the group consisting of declined kidney function, chronic kidney disease, and renal failure has not been improved by the test substance, the evaluation result comparison unit 23 outputs a comparison result such that the disease has not been improved. When the comparison result indicates an improved result, the candidate substance determination unit 24 determines that the test substance is a candidate substance for the active ingredient.

In step S26, the candidate substance determination unit 24 outputs the result determined in step S25. In this embodiment, the determination result is displayed on the display unit 4, and stored in the storage unit 103 in the screening device 2. The determination result may be displayed on a display unit of an external computer terminal connected to the screening device 2 via the internet, for example, a display unit of a computer terminal in a third-party organization, instead of displaying the determination result on the display unit 4.

In step S24, when the comparison result indicates that the disease has not been improved, the processing proceeds to step S27, and the candidate substance determination unit 24 determines that the test substance is not the active ingredient. In this case, the result such that the test substance is not the active ingredient may be displayed on the display unit 4.

7. Screening 3

The present invention further includes a third screening method. This embodiment is a method for screening a candidate substance for an active ingredient to prevent or treat at least one disease selected from the group consisting of declined kidney function, chronic kidney disease, and renal failure, the method comprising step (A) of detecting a kidney function prediction marker protein and/or mRNA of the protein in a test-substance-treated specimen. The detection of the kidney function prediction marker protein can be performed, for example, by a known detection method of proteins, such as the Western blotting method or ELISA method, using an "antibody against a kidney function prediction marker" described in the above section "I. 1. Explanation of terms." The detection of the kidney function prediction marker mRNA can be performed, for example, by a known method, such as the microarray method or RT-PCR, using a "nucleic acid for kidney function prediction marker mRNA detection" described in the above section "I. 1. Explanation of terms."

The detection result may be obtained through visual observation, or may be obtained as absorbance, fluorescence intensity, or luminescence intensity.

This embodiment may comprise step (B) of determining that the test substance is a candidate substance for the active ingredient based on the result obtained in the detection step. In this step, for example, in the case where the specimen is collected from a subject with at least one disease selected from the group consisting of declined kidney function, chronic kidney disease, and renal failure, and where the kidney function prediction marker protein is a protein whose expression increases with a decline in kidney function, when the kidney function prediction marker protein and/or mRNA of the protein is not detected, or even if detected, is significantly small, in the test-substance-treated specimen, it can be determined that the test substance is a candidate substance for the active ingredient. In the case where the specimen is collected from a subject with at least one disease selected from the group consisting of declined kidney function, chronic kidney disease, and renal failure, and where the kidney function prediction marker protein is a protein whose expression decrease with a decline in kidney function, when the kidney function prediction marker protein and/or mRNA of the protein in the test-substance-treated specimen is detected in a larger amount than a corresponding kidney function prediction marker protein and/or mRNA of the protein in an untreated specimen, it can be determined that the test substance is a candidate substance for the active ingredient.

Further, in this embodiment, the detection result of the kidney function prediction marker protein and/or mRNA of the protein in the test-substance-treated specimen may be compared with a detection result of a corresponding protein and/or mRNA in an untreated specimen, and it may be determined that the test substance is a candidate substance for the active ingredient. More specifically, this embodiment may comprise, between step (A) and step (B), a step of comparing the detection result of the test-substance-treated specimen obtained in step (A) with a detection result of a corresponding kidney function prediction marker protein and/or mRNA of the protein in an untreated specimen. For example, in the case where the specimen is collected from a subject with at least one disease selected from the group consisting of declined kidney function, chronic kidney disease, and renal failure, and where the expression of the kidney function prediction marker protein and/or mRNA of the protein increases with a decline in kidney function, when the protein and/or mRNA is detected in the untreated specimen, and when the corresponding protein and/or mRNA is not detected in the test-substance-treated specimen or the expression thereof is decreased in the test-substance-treated specimen, it can be determined that the test substance is a candidate substance for the active ingredient. In the case where the specimen is collected from a subject with at least one disease selected from the group consisting of declined kidney function, chronic kidney disease, and renal failure, and where the expression of the kidney function prediction marker protein and/or mRNA of the protein decreases with a decline in kidney function, when the protein and/or mRNA in the test-substance-treated specimen is detected in a larger amount than the kidney function prediction marker protein and/or mRNA of the protein in the untreated specimen, it can be determined that the test substance is a candidate substance for the active ingredient. In a comparison between the detection result of the kidney function prediction marker protein and/or mRNA of the protein in the test-substance-treated specimen and the detection result of the corresponding protein and/or mRNA in the untreated specimen, a difference between them may be a difference that can be visually detected.

This embodiment may comprise, before step (A), the following steps: (a) treating the test substance to the subject, test tissue, or test cell; (b) collecting the specimen from the subject, test tissue, or test cell to which the test substance has been administered in step (a); and (c) collecting the protein or mRNA from the specimen obtained in step (b).

## 8. Screening 4

When the functional expression of Oscar, which is a kidney function prediction marker protein, is suppressed, induction of expression of FGF23 is suppressed under ingestion of a diet with high phosphorus content. Thus, in the screening 1 to the screening 3 described above, when the kidney function prediction marker protein is Oscar, "screening a candidate substance for an active ingredient to prevent or treat at least one disease selected from the group consisting of declined kidney function, chronic kidney disease, and renal failure" can also be read as "screening a candidate substance capable of suppressing the functional expression of FGF23."

## 9. Biomarker

The present invention relates to a method for using a kidney function prediction marker protein and/or mRNA of the kidney function prediction marker protein as a biomarker for screening a candidate substance for an active ingredient to prevent or treat at least one disease selected from the group consisting of declined kidney function, chronic kidney disease, and renal failure. Here, the definition of "kidney function prediction marker" is in accordance with the description in the "1. Explanation of terms."

Any one protein selected from proteins encoded by the genes shown in Fig. 22 or any one mRNA selected from mRNAs encoded by the genes shown in Fig. 23 can be used as a biomarker for screening a candidate substance for an active ingredient to prevent or treat at least one disease selected from the group consisting of declined kidney function, chronic kidney disease, and renal failure.

Moreover, as another embodiment, a combination of at least two proteins selected from proteins encoded by the genes shown in Fig. 22, or a combination of at least two mRNAs of proteins selected from mRNAs encoded by the genes shown in Fig. 23 can be used as a biomarker for screening a candidate substance for an active ingredient to prevent or treat at least one disease selected from the group consisting of declined kidney function, chronic kidney disease, and renal failure.

These biomarkers are contained in each specimen.

# II. iOrgans

This embodiment relates to two novel disease determination methods called "Reverse iOrgans" and "Forward iOrgans," based on a new methodology called "iOrgans (Inter-Organ Cross Talks) technology." In the methodology, a comprehensive database of changes in the amounts of gene expression, metabolites, etc., derived from organs other than a specific organ is constructed, and the changes are associated with functional and histological changes of the specific organ in a subject. The disease determination is achieved by using the comprehensive database. "iOrgans" is a technology to diagnose, prevent, and/or treat disease by using the interrelationship between the state of one organ and that of one or more other organs as a measure.

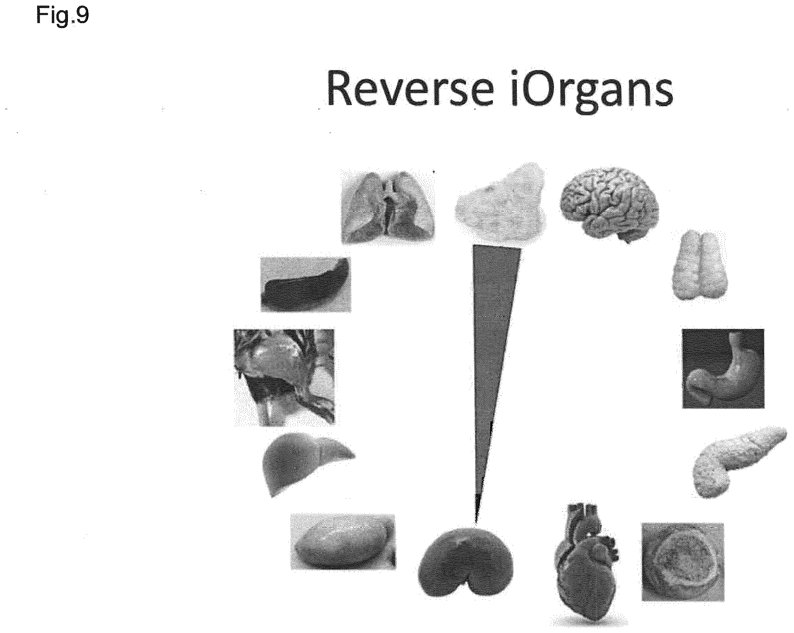

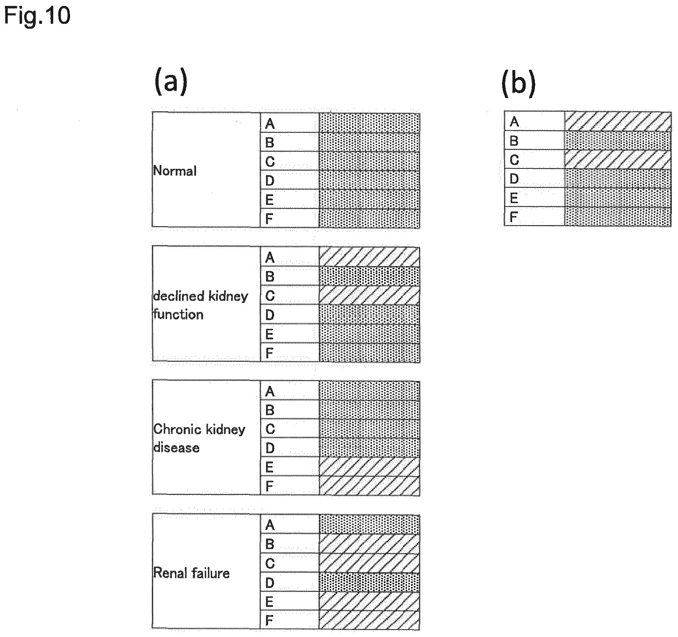

Figs. 9 and 10 schematically illustrate an outline of Reverse iOrgans according to the present invention.

Reverse iOrgans is a method for predicting a specific disease in a subject from information regarding, for example, the pattern of gene expression in each organ other than a specific organ collected from the same subject at the same time point. It is possible to predict the presence of a specific latent disease or the state of a specific organ by this method. In the example shown in Fig. 9, a disease (e.g., at least one disease selected from the group consisting of declined kidney function, chronic kidney disease, and renal failure) in a specific organ (e.g., kidneys) is predicted from information regarding, for example, the pattern of gene expression in another organ (e.g., adipose tissue or cells). An outline of the prediction method of Reverse iOrgans is described with reference to Fig. 10, assuming, as an example, that the other organ is adipose tissue, and that the disease in the specific organ is at least one disease selected from the group consisting of declined kidney function, chronic kidney disease, and renal failure. A to F shown in Fig. 10 represent an inter-organ cross talk indicator.

First, a pattern of gene expression (i.e., a pattern of an inter-organ cross talk indicator) in adipose tissue is collected beforehand for each stage of at least one disease selected from the group consisting of declined kidney function, chronic kidney disease, and renal failure, as standard data for each stage; and a group of standard data is prepared. Fig. 10(a) shows an example of standard data. The standard data of Fig. 10(a) shows a pattern of the inter-organ cross talk indicator, i.e., A to F in adipose tissue at each stage of the state of the kidneys (selected from normal state, declined kidney function, chronic kidney disease, and renal failure). In the pattern of the inter-organ cross talk indicator in declined kidney function, chronic kidney disease, or renal failure, items in the inter-organ cross talk indicator shown in gray represent items in the inter-organ cross talk indicator showing no changes relative to normal, and items in the inter-organ cross talk indicator shown with diagonal hatching represent items in the inter-organ cross talk indicator showing changes relative to normal.

Next, adipose tissue is collected from a subject, and the pattern of the inter-organ cross talk indicator in the adipose tissue is determined and used as data of the subject (e.g., Fig. 10(b)). Subsequently, the standard data and the data of the subject derived from adipose tissue are compared with each other, and similarity between patterns is calculated. When a pattern similar to the data of the subject is present in the standard data, it can be predicted that the state of the kidneys linked with the similar pattern in the standard data is the state of the kidneys (a disease stage selected from declined kidney function, chronic kidney disease, and renal failure) from which the subject is suffering. In the example shown in Fig. 10, the pattern of the data of the subject shown in (b) is similar to the second pattern from the top in the standard data. The second pattern from the top is a pattern obtained when the kidneys are in the state of declined kidney function. It can thus be predicted that the kidneys of the subject are in the state of declined kidney function.

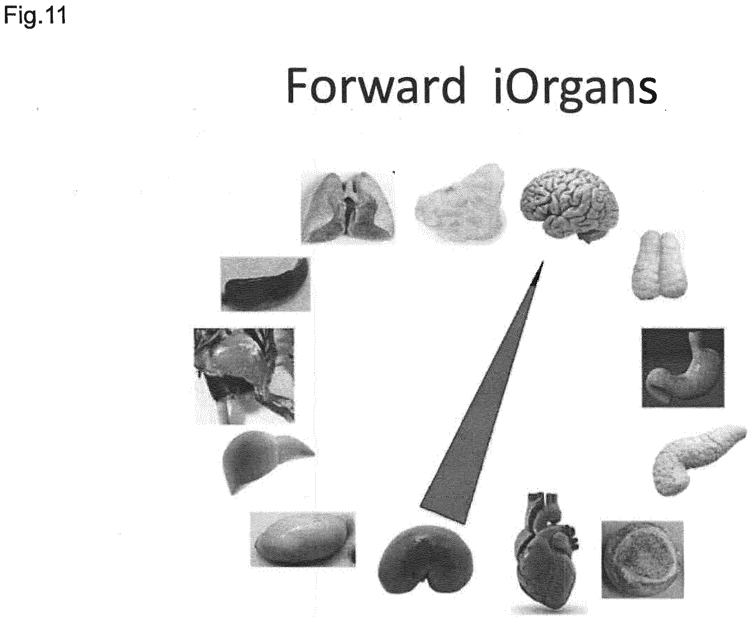

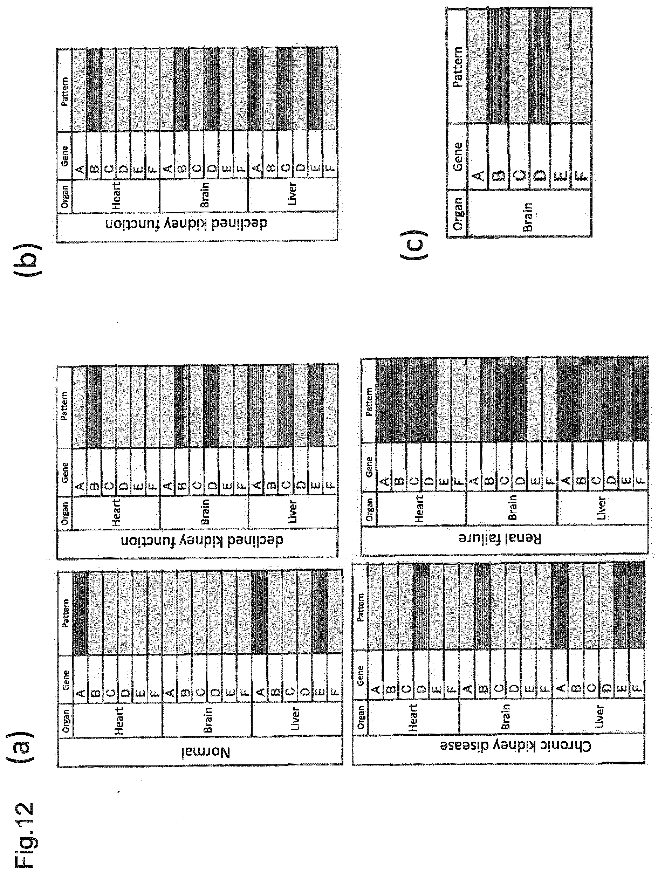

Figs. 11 and 12 schematically illustrate an outline of Forward iOrgans according to the present invention.

Forward iOrgans is a method in which, after one disease stage selected from declined kidney function, chronic kidney disease, and renal failure in the kidneys of a subject is determined by using a usual test etc., the one disease stage selected from declined kidney function, chronic kidney disease, and renal failure is compared with predetermined data regarding inter-organ cross talk indicators in other organs to determine the pattern of gene expression etc. derived from each organ other than the kidneys of the subject; and, on the basis of this, the presence of a disease or the stage of the disease, including complications, in each of the organs other than the kidneys is predicted. The presence of a disease or the stage of the disease, including complications, in each organ other than the kidneys can be predicted by checking the pattern of gene expression etc. derived from each of the organs other than the kidneys of the subject against previously reported information regarding gene expression in the disease in each of the organs other than the kidneys. In the example of Fig. 11, one disease stage selected from declined kidney function, chronic kidney disease, and renal failure is identified beforehand by using a usual test etc., and the state of an organ (e.g., brain) other than the kidneys is predicted from the one disease stage selected from declined kidney function, chronic kidney disease, and renal failure. Taking this case as an example, an outline of the prediction method of Forward iOrgans is described with reference to Fig. 12.

First, information that the state of the kidneys in a subject is one disease stage selected from declined kidney function, chronic kidney disease, and renal failure is determined from the results of, for example, a biochemical test of serum or the like. Next, the stage of the subject is checked against patterns of gene expression etc. in organs including the kidneys stored for the one disease stage selected from declined kidney function, chronic kidney disease, and renal failure (patterns of inter-organ cross talk indicators) (e.g., Fig. 12(a)), thereby extracting patterns of the inter-organ cross talk indicators corresponding to the stage in the subject (e.g., the state of declined kidney function) (Fig. 12(b)) from the data of Fig. 12(a). Furthermore, the pattern of the inter-organ cross talk indicator derived from the brain (Fig. 12(c)) is extracted from the patterns of Fig. 12(b). By this procedure, the pattern of the inter-organ cross talk indicator derived from the brain (Fig. 12(c)) can be inferred to be the pattern of the inter-organ cross talk indicator derived from the brain at the stage in the subject. Based on the inter-organ cross talk indicator shown in the pattern inferred, the state of the brain can be predicted from previously reported information regarding diseases and complications.

1. Explanation of terms

First, terms used in the present specification, claims, and abstract regarding inventions relating to iOrgans are explained. Unless otherwise stated, terms used in the present specification, claims, and abstract are in accordance with the definitions in this section. The same terms as some of the terms used in the above section "I. Screening" are used in this section "II. iOrgans"; however, terms relating to the inventions described in "II. iOrgans" used in the specification, claims, and abstract are in accordance with the definitions in this section, unless otherwise stated. The definitions of the terms used in the above section "I. Screening" and the definitions of the terms used in this section "II. iOrgans" are independent of each other, unless individual sections are referred to.

"Individual" as used herein is not particularly limited. Examples include mammals, such as humans, mice, rats, dogs, cats, rabbits, bovines, horses, goats, sheep, and pigs, birds, such as chickens, and the like. The individual is preferably a mammal such as a human, a mouse, a dog, a cat, a bovine, a horse, or a pig, more preferably a human, a mouse, a dog, a cat, or the like, even more preferably a human or a mouse, and most preferably a human. In addition, the term "individual" includes both individuals having disease and individuals having no disease. There is no limitation on the age or sex (male or female) of the individual; however, the individual is preferably the same species, the same age, and/or the same sex as the subject described later.

Moreover, the term "individual" also includes individuals that gestate.

The ages of the individuals in the present invention may be classified into the following age groups in humans: under 7 years of age, 7 years of age or older but under 15 years of age, 15 years of age or older but under 30 years of age, 30 years of age or older but under 60 years of age, and 60 years of age or older. The age in the present invention is not particularly limited and is preferably 15 years of age or older but under 30 years of age, 30 years of age or older but under 60 years of age, or 60 years of age or older, and more preferably 30 years of age or older but under 60 years of age, or 60 years of age or older. In mice, the ages may be classified into the following age groups: under 6 weeks of age, 6 weeks of age or older but under 24 weeks of age, 24 weeks of age or older but under 48 weeks of age, and 48 weeks of age or older.

Here, an individual with at least one disease selected from the group consisting of declined kidney function, chronic kidney disease, and renal failure described later is referred to as a "positive control," and an individual without at least one disease selected from the group consisting of declined kidney function, chronic kidney disease, and renal failure described later is referred to as a "negative control."

In the present invention, "tissue" refers to a collection of cells that have a similar function and a similar shape.

"Organ" as used herein means a collection of tissue, in a subject, that has a certain independent form and a specific function. Specific examples include organs of the circulatory system (such as the heart, arteries, veins, and lymphatic vessels), organs of the respiratory system (such as the nasal cavity, paranasal sinus, larynx, trachea, bronchus, and lungs), organs of the digestive system (such as the lips, buccal region, palate, teeth, gums, tongue, salivary glands, pharynx, esophagus, stomach, duodenum, jejunum, ileum, cecum, appendix, ascending colon, transverse colon, sigmoid colon, rectum, anus, liver, gallbladder, bile duct, biliary tract, pancreas, and pancreatic duct), organs of the urinary system (such as the urethra, urinary bladder, ureter, and kidneys), organs of the nervous system (such as the cerebrum, cerebellum, midbrain, brainstem, spinal cord, peripheral nerves, and autonomic nerves), organs of the female reproductive system (such as the ovaries, Fallopian tubes, uterus, and vagina), the breasts, organs of the male reproductive system (such as the penis, prostate gland, testes, epididymis, and vas deferens), organs of the endocrine system (such as the hypothalamus, pituitary glands, pineal body, thyroid gland, parathyroid glands, and adrenal glands), organs of the integumentary system (such as the skin, hair, and nails), organs of the hematopoietic system (such as peripheral blood, bone marrow, and spleen), organs of the immune system (such as the lymph nodes, tonsils, and thymus), bones and soft-tissue organs (such as the bones, cartilage, skeletal muscles, connective tissue, ligaments, tendons, diaphragm, peritoneum, pleura, and adipose tissue), and organs of the sensory organ system (such as the eyeballs, eyelids, lacrimal glands, outer ear, middle ear, inner ear, and cochlea). Preferable examples of tissue in the present invention include tissue of the heart, cerebrum, lungs, kidneys, adipose tissue, liver, skeletal muscle, testes, spleen, thymus, bone marrow, pancreas, and the like. More preferred examples of tissue include tissue of the heart, cerebrum, lungs, kidneys, adipose tissue, liver, skeletal muscle, spleen, bone marrow, pancreas, and the like.

Furthermore, in the case of using an individual that gestates (preferably an individual other than humans) as a subject, the term "organ" in the present invention may include the whole body of an embryo or the organs described above of an embryo.

In the present invention, body fluids, such as serum, plasma, urine, spinal fluid, ascites fluid, pleural effusion, saliva, gastric fluid, pancreatic fluid, bile, and milk, particularly preferably plasma, may be used instead of the organs described above.

"Inter-organ cross talk indicator" as used herein is at least one *in vivo* factor (or molecule) that is present in a living organism and acts as a measure representing the states of organs through organ-to-organ communication (i.e., inter-organ cross talk) in a living organism. In other words, the inter-organ cross talk indicator is an *in vivo* substance or substances that can undergo changes in cells or tissue originating from each organ, and/or a body fluid in an individual having at least one disease selected from the group consisting of declined kidney function, chronic kidney disease, and renal failure, depending on whether the disease is present. Examples of *in vivo* substances that can act as an inter-organ cross talk indicator include nucleic acids; carbohydrates; lipids; glycoproteins; glycolipids; lipoproteins; amino acids, peptides; proteins; polyphenols; chemokines; at least one metabolite selected from the group consisting of metabolic end products of the above substances, intermediate metabolites of the above substances, and starting substances for one or more metabolic pathways of the above substance; metal ions; and the like. Preferable examples are nucleic acids.

In the present invention, the nucleic acid is preferably RNA, such as mRNA, non-coding RNA, or microRNA, and more preferably mRNA. The RNA is preferably at least one RNA selected from the group consisting of mRNAs, non-coding RNAs, and microRNAs that can be expressed in cells or tissue originating from organs described above, more preferably RNAs expressed from genes listed in Fig. 21 in which the RNAs can be detected by RNA-Seq etc. (also referred to herein as "group 1"). Among these, the RNAs having polyA sequences are preferable. More specifically, the RNA is at least one RNA selected from the group consisting of RNAs expressed from the genes of group 2 listed in Fig. 22, or at least one RNA selected from the group consisting of RNAs expressed from the orthologs of group 2 that are present in the individual described above. More preferably, the RNA is at least one RNA selected from the group consisting of RNAs expressed from the genes of group 3 listed in Fig. 22, or at least one RNA selected from the group consisting of RNAs expressed from the orthologs of group 3 that are present in the individual described above. More preferably, the RNA is at least one RNA selected from the group consisting of RNAs expressed from the genes of group 4 listed in Fig. 22, or at least one RNA selected from the group consisting of RNAs expressed from the orthologs of group 4 that are present in the individual described above. More preferably, the RNA is at least one RNA selected from the group consisting of RNAs expressed from the genes of group 5 listed in Fig. 22, or at least one RNA selected from the group consisting of RNAs expressed from the orthologs of group 5 that are present in the individual described above. More preferably, the RNA is at least one RNA selected from the group consisting of RNAs expressed from the genes of group 6 listed in Fig. 23, or at least one RNA selected from the group consisting of RNAs expressed from the orthologs of group 6 that are present in the individual described above. Particularly preferably, the RNA is mRNA expressed from at least one gene selected from the group consisting of proline-rich proteins (Prh1, Prp2, Prb1, Prpmp5), defensins (Defa and Defb), Aplnr, Spp1, Dnase1, Slco1a1, Anpep, Slc7a8, Oscar, fibrinogens, and Hamp/Hepcidin; or at least one RNAs expressed from the orthologs of these genes that are present in the individual described above. In an individual in which an ortholog corresponding to a gene described in Fig. 22 or 23 is not present, the ortholog is excluded from the analysis. It is more preferred that non-coding RNAs and microRNAs (their NCBI Reference Seq IDs start with "NR") be excluded from the analysis in individuals other than mice. Human orthologs corresponding to groups 2 to 6 are RNAs expressed from genes indicated by the Human Gene ID listed in Fig. 22.

"An amount of an inter-organ cross talk indicator" or "amounts of inter-organ cross talk indicators" as used herein may be expressed as a quantitative value (or a quantitative level), or expressed semi-quantitatively as follows: for example, "increase," "no change," and "decrease." "An amount of an inter-organ cross talk indicator" or "amounts of inter-organ cross talk indicators" may be the measurement value of the inter-organ cross talk indicator.

In the present invention, the definitions of the terms "declined kidney function," "chronic kidney disease," and "renal failure" are in accordance with the definitions in the above section "I. Screening, 1. Explanation of terms."

"Standard data 1" as used herein is data of inter-organ cross talk indicators in each organ that serve as a measure for predicting the presence of at least one disease selected from the group consisting of declined kidney function, chronic kidney disease, and renal failure, and/or the stage of the disease in a subject. More specifically, standard data 1 is a group of patterns of inter-organ cross talk indicators, each of the patterns being derived from a predetermined relationship between an amount of an inter-organ cross talk indicator in an organ other than the kidneys in positive control(s) affected with the at least one disease selected from the group consisting of declined kidney function, chronic kidney disease, and renal failure, and an amount of the corresponding inter-organ cross talk indicator in the same organ as the organ other than the kidneys in negative control(s) without the at least one disease selected from the group consisting of declined kidney function, chronic kidney disease, and renal failure; and preferably a group of patterns of inter-organ cross talk indicators, each of the patterns being derived from a predetermined ratio between an amount of an inter-organ cross talk indicator in an organ other than the kidneys in positive control(s) affected with the at least one disease selected from the group consisting of declined kidney function, chronic kidney disease, and renal failure, and an amount of the corresponding inter-organ cross talk indicator in the same organ as the organ other than the kidneys in negative control(s) without the at least one disease selected from the group consisting of declined kidney function, chronic kidney disease, and renal failure. More preferably, the amount of the inter-organ cross talk indicator is the expression level of at least one gene, and the patterns of the inter-organ cross talk indicators are a group of patterns of expression of at least one gene. Moreover, instead of a group of standard data 1, correlation maps (standard data 1-Maps) may be used. The correlation maps (standard data 1-Maps) are generated using a group of standard data 1 derived from multiple organs by determining, for each disease or each stage, the correlation of the patterns of inter-organ cross talk indicators between the organs. The method for generating the correlation maps is described below.

"Standard data 2" as used herein is data of inter-organ cross talk indicators in each organ that serve as a measure for predicting the presence of a disease and/or the stage of the disease in each of one or more organs other than the kidneys in a subject affected with at least one disease selected from the group consisting of declined kidney function, chronic kidney disease, and renal failure. More specifically, standard data 2 is a group of patterns of inter-organ cross talk indicators predetermined for each stage of the at least one disease selected from the group consisting of declined kidney function, chronic kidney disease, and renal failure, each of the patterns being derived from a predetermined relationship between an amount of an inter-organ cross talk indicator in an organ other than the kidneys in positive control(s) affected with the at least one disease selected from the group consisting of declined kidney function, chronic kidney disease, and renal failure, and an amount of the corresponding inter-organ cross talk indicator in the same organ as the organ other than the kidneys in negative control(s) without the at least one disease selected from the group consisting of declined kidney function, chronic kidney disease, and renal failure. Preferably, standard data 2 is a group of patterns of inter-organ cross talk indicators predetermined for each stage of at least one disease selected from the group consisting of declined kidney function, chronic kidney disease, and renal failure, each of the patterns being derived from a value obtained by dividing an amount of an inter-organ cross talk indicator in an organ other than the kidneys in the positive control(s) by an amount of the corresponding inter-organ cross talk indicator in the same organ as the organ other than the kidneys in the negative control(s). More preferably, the amount of the inter-organ cross talk indicator is the expression level of at least one gene, and the patterns of the inter-organ cross talk indicators are a group of patterns of expression of at least one gene.

Standard data 1 or 2 is obtained for each stage of at least one disease selected from the group consisting of declined kidney function, chronic kidney disease, and renal failure; each organ or body fluid; and, if necessary, each sex and/or each age group. A database of a group of standard data 1 or a group of standard data 2 may be established. Each of the patterns of the inter-organ cross talk indicators is linked with information regarding the corresponding stage of at least one disease selected from the group consisting of declined kidney function, chronic kidney disease, and renal failure, and the corresponding organ or body fluid. The database may be stored in a storage medium or a storage device.

The term "pattern" includes, for example, the presence or absence of an inter-organ cross talk indicator, the amounts of inter-organ cross talk indicators, or changes in the amounts of inter-organ cross talk indicators over time; and a combination of the amounts of inter-organ cross talk indicators, and changes in the amounts of inter-organ cross talk indicators over time. Preferably, the pattern includes the presence or absence of an inter-organ cross talk indicator, the amounts of inter-organ cross talk indicators, or changes in the amounts of inter-organ cross talk indicators over time; and a combination of the amounts of inter-organ cross talk indicators and changes in the amounts of inter-organ cross talk indicators over time, for each stage. Preferably, the pattern includes the presence or absence of expression of RNA from at least one gene, the expression level of RNA from at least one gene, or changes in the expression level of RNA from at least one gene over time; and a combination of the expression level of RNA from at least one gene, and changes in the expression level of RNA from at least one gene over time.

The amassment of standard data 1, standard data 2, a group of standard data 1, or a group of standard data 2 above is called a "database." The corresponding pattern of an inter-organ cross talk indicator in standard data 1, standard data 2, a group of standard data 1, or a group of standard data 2 can be retrieved and extracted from the database based on, for example, information regarding one disease stage selected from the group consisting of chronic kidney disease and renal failure, and/or the name of each organ or body fluid.

"Subject" is a subject to which the prediction methods according to the present invention are applied, and is preferably of a species corresponding to those of individuals used for obtaining standard data 1 or 2 above. For example, if individuals used for obtaining the standard data are mice, then a mouse, a rat, a human, or the like may be selected as the subject. The age and sex of the subject are not particularly limited, and the subject may be in the same age group and/or of the same sex as individuals used for obtaining standard data 1 or 2.

"Data of a subject" or "subject data" as used herein is data of an inter-organ cross talk indicator derived from all or part of an organ collected from a subject, and the pattern is linked with the corresponding information about the subject and the organ. More specifically, data of a subject or subject data is a pattern of an inter-organ cross talk indicator indicating the relationship between the amount of the inter-organ cross talk indicator in an organ other than the kidneys of the subject, and the amount of the corresponding inter-organ cross talk indicator in the same organ as the organ other than the kidneys in negative control(s) without at least one disease selected from the group consisting of declined kidney function, chronic kidney disease, and renal failure. Data of a subject or subject data is preferably a pattern of an inter-organ cross talk indicator indicating the ratio between the amount of the inter-organ cross talk indicator in an organ other than the kidneys of the subject, and the amount of the corresponding inter-organ cross talk indicator in the same organ as the organ other than the kidneys in negative control(s) without at least one disease selected from the group consisting of declined kidney function, chronic kidney disease, and renal failure. More preferably, the amount of the inter-organ cross talk indicator is the expression level of RNA from at least one gene, and the pattern of the inter-organ cross talk indicator is a pattern of expression of least one gene.

"Gold standard" as used herein is an individual or individuals that have already been determined to have or not have at least one disease selected from the group consisting of declined kidney function, chronic kidney disease, and renal failure by a known test method and/or diagnostic method. The term "gold standard" also includes healthy individuals.

"Similarity" as used herein indicates the degree to which patterns of inter-organ cross talk indicators are similar when data of a subject is compared with standard data or when subject data X is compared with standard data Y. More specifically, the similarity can be determined visually, or by statistical analysis or the like. Examples of statistical analysis for calculating the similarity include Z-score, Spearman's pairwise correlation, Kullback-Leibler divergence, principal component analysis (PCA), and the like.

For example, when a p-value is calculated using Spearman pairwise correlation, it can be determined as follows: when the p-value is 1, it can be determined that the data of the subject is identical to the standard data 1; when the p-value is more than 0.4 and less than 1, preferably more than 0.65 and less than 1, more preferably more than 0.75 and less than 1, and even more preferably more than 0.85 and less than 1, it can be determined that the data of the subject is similar to the standard data 1; on the other hand, when the p-value is 0.8 or less, preferably 0.65 or less, and more preferably 0.40 or less, it can be determined that the data of the subject is not similar to the standard data 1.

For example, when a z-value is calculated using a Z-score, it can be determined as follows: when the z-value is 0, it can be determined that the data of the subject is identical to the standard data 1; when the z-value falls within the range of $0\pm0.5$ (excluding 0), preferably within the range of $0\pm0.4$ (excluding 0), more preferably within the range of $0\pm0.3$ (excluding 0), and even more preferably within the range of $0\pm0.15$ (excluding 0), it can be determined that the data of the subject is similar to the standard data 1; on the other hand, when the z-value falls outside the range of $0\pm0.15$, preferably outside the range of $0\pm0.3$, more preferably outside the range of $0\pm0.4$, and even more preferably outside the range of $0\pm0.5$, it can be determined that the data of the subject is not similar to the standard data 1.

Further, when at least 50%, preferably at least 70%, more preferably at least 80%, and even more preferably at least 90% of items in examined inter-organ cross talk indicators are identical or similar between standard data 1 and data of a subject, it can be determined that the pattern in the standard data 1 is similar to the pattern in the data of the subject. On the other hand, when at least 50%, preferably at least 70%, more preferably at least 80%, and even more preferably at least 90% of items in examined inter-organ cross talk indicators are not identical or similar between standard data 1 and data of a subject, it can be determined that the pattern in the standard data 1 is not similar to the pattern in the data of the subject.

Standard data 1-Maps are determined as follows. When standard data 1-Maps are determined, multiple organs are collected for each stage of at least one disease selected from the group consisting of declined kidney function, chronic kidney disease, and renal failure; and patterns of inter-organ cross talk indicators derived from the organs are determined (for example, when the inter-organ cross talk indicator is RNA, the genes expressing RNAs are listed in a certain order that is consistent among organs). Correlation coefficients are calculated between the patterns of the organs using, for example, Spearman's rank correlation, and maps between the organs are generated.

More specifically, for example, the correlation coefficient of patterns of inter-organ cross talk indicators j between organ m and organ l in disease model i is represented by $r_{ijml}$.

The number of individuals of disease model i is represented by n.

In this case, the correlation coefficient between organ m and organ l of disease model i can be represented by probability model p (the following equation).

Equation 1

$$p(r|i,m,l\ ) = \frac{1}{\sqrt{2\pi}\sigma_{iml}} \exp\left(-\frac{(r-r_{iml})^2}{2\sigma_{iml}^2}\right)$$

wherein $r_{iml}$ is the mean of n correlation coefficients $r_{ijml}$, and $\sigma_{iml}^2$ is the sample variance of the correlation coefficients $r_{ijml}$.

Comparisons between data of a subject and standard data 1-Maps, comparisons between subject data X and standard data Y2-Maps, and comparisons between subject data X and standard data Y3-Maps can be performed using Bayesian inference, machine-learning methods, etc.

For example, patterns of inter-organ cross talk indicators of multiple organs in a subject are obtained, and correlation coefficient(s) of the patterns of inter-organ cross talk indicators are determined between the organs in the subject from which the data of the subject or the subject data X is obtained, in the same manner as described above. The obtained value(s) are represented by the following:

$$\{r'_{ml}\}_{m,l \in (\text{collected organs})}$$

In this case, the likelihood $L_i$ of correlation

$$\{r'_{ml}\}_{m,l \in (\text{collected organs})}$$

with respect to each model i can be calculated using the following equation.

Equation 2

$$L_i = \prod_{m,l} p(r'_{ml} | i, m, l)$$

The likelihood is calculated for each model i, and a model i with the highest likelihood can be inferred to be the state of the subject.

When the number of organs to be compared is three or more, the likelihood between a disease model and a subject is determined between two of each of the organs, and the product of the calculated likelihoods is determined. A model i with the highest product may be inferred to be the state of the subject.

Which inter-organ cross talk indicator is used is not particularly limited when comparisons between data of a subject and standard data 1-Maps are performed. For example, it is preferable to use an inter-organ cross talk indicator in which the difference between positive control(s) and negative control(s) is large. More specifically, for example, when the inter-organ cross talk indicator is RNA, it is RNA in which the ratio between positive control(s) and negative control(s) is more than 1.5 or less than 0.65, preferably more than 2 or less than 0.5, and more preferably more than 5 or less than 0.2.

The statistical analysis described above can be performed, for example, with a computer using a calculation program. In this case, the prediction program according to the present invention described later may comprise program code of a statistical analysis program for performing statistical analysis, or commercially available statistical analysis software may be used as a statistical analysis program. For example, the analysis can be performed using commercially available statistical analysis software, such as StatFlex Ver. 6 (Artech Co., Ltd., Osaka, Japan) or IBM SPSS Statistics (IBM Japan Ltd.).

"One or more" as used herein includes cases of one kind, and cases of multiple kinds. The term "multiple" is not particularly limited as long as it means two or more, and preferably refers to three or more, more preferably five or more, and even more preferably ten or more. Further, in the present specification, the use of a singular noun may include the plural.

## 2. Methods for collecting and storing cells or tissue for extraction of an inter-organ cross talk indicator, and methods for extracting and measuring an inter-organ cross talk indicator

The method for collecting cells or tissue for extraction of an inter-organ cross talk indicator used in the present invention and the method for their storage are not particularly limited, and cells or tissue can be collected and stored according to known methods depending on the type of inter-organ cross talk indicator. The method for extracting an inter-organ cross talk indicator used in the present invention is also not particularly limited, and the inter-organ cross talk indicator can be extracted according to a known method depending on the type of inter-organ cross talk indicator. The method for measuring an inter-organ cross talk indicator in the present invention is not particularly limited as long as the amount of an inter-organ cross talk indicator can be measured.

Cells or tissue used for extraction of an inter-organ cross talk indicator is not particularly limited. Examples include cells, tissue, etc., collected from a subject by, for example, puncture, biopsy, or surgery. (The collected cells or tissue is also called a "specimen.") The cells or tissue may be, for example, fresh material after collection or cryopreserved material.

In this embodiment, an inter-organ cross talk indicator may be obtained from cells or tissue originating from the kidneys suspected of having a disease and from one or more organs other than the kidneys, for each stage of at least one disease selected from the group consisting of declined kidney function, chronic kidney disease, and renal failure. In addition, an inter-organ cross talk indicator may be derived from the corresponding cells or tissue in an individual without the at least one disease selected from the group consisting of declined kidney function, chronic kidney disease, and renal failure.

The time at which cells, tissue, or body fluids are collected can suitably be selected according to the disease stage from, for example, the following: before the onset of at least one disease selected from the group consisting of declined kidney function, chronic kidney disease, and renal failure (in the normal state); at the onset of at least one disease selected from the group consisting of declined kidney function, chronic kidney disease, and renal failure; 1 month, 6 months, 1 year, 2 years, 3 years, 5 years, or 10 years after the onset of at least one disease selected from the group consisting of declined kidney function, chronic kidney disease, and renal failure; and the like.

When RNA is used as an inter-organ cross talk indicator, it is preferable that RNA extraction from cells or tissue is performed immediately after the cells or tissue is collected; or is performed after freezing the cells or tissue with liquid nitrogen or the like immediately after the cells or tissue is collected, and then transporting and storing the cells or tissue.

The method for extracting RNA is not particularly limited, and RNA can be extracted using a known method. RNA may be purified using, for example, an oligo dT probe, as necessary. If necessary, cDNA may be synthesized from extracted or purified RNA by a reverse transcription reaction, and used for measurement. Qualitative or quantitative measurement (including semi-quantitative measurement) of RNA may be performed by a known method, such as a method using a microarray, which can comprehensively analyze gene expression; or a method in which analysis is conducted by RNA-Seq, which determines the absolute amounts of RNAs in cells. As comprehensive and quantitative analysis, RNA-Seq is preferable.

Data obtained by RNA-Seq or the like can be analyzed using a known method. For example, when the data is analyzed with Illumina HiSeq (Illumina, Inc.) or the like, the output data can be processed by the following method: (1) text data of nucleotide sequences are obtained from the output raw data of analysis (image data) (base calling); (2) the data is selected using predetermined filtering such as removing low fluorescence purity clusters caused by overlapping clusters from the data by using a calculation formula, such as chastity (filtering); and (3) the sample data is sorted based on index sequence information provided for each sample (specific nucleotide sequence information).

A data file (Fastq format or the like) obtained from the RNA-Seq sequencer is uploaded on, for example, Galaxy (https://usegalaxy.org/). Thereafter, analysis is carried out using, for example, Bowtie2 (http://bowtie-bio.sourceforge.net/bowtie2/index.shtml) to map each sequence to mouse genome map information mm9 or mm10. A BAM file obtained using Bowtie2 or the like is analyzed using, for example, Cufflinks (http://cole-trapnell-lab.github.io/cufflinks/) to calculate FPKM (RPKM) for each gene. In the obtained FPKM data, all FPKM values less than 1 are regarded as 0; pairwise correlation ($\rho=1-(6\sum D^2)/(n^3-n)$) is calculated using Python, and a heat map is generated using MeV. The FPKM values may also be visually analyzed.

If necessary, expression can also be confirmed by real-time PCR or the like. In addition, the mRNA expression level can be normalized by the expression level of a housekeeping gene, such as GAPDH, β2-microglobulin (β2M), or Maea, as necessary, and expressed as a relative expression level.

The expression level obtained by the method described above can be stored in the storage unit of a device, or a device having a storage unit that is different from the device, as a pattern of the inter-organ cross talk indicator for each stage of at least one disease selected from the group consisting of declined kidney function, chronic kidney disease, and renal failure; each organ or body fluid; each type of individual; each age group of individuals; and/or each sex of individuals.

## 3. Reverse iOrgans

### 3-1. Outline

In this embodiment, the presence of at least one disease selected from the group consisting of declined kidney function, chronic kidney disease, and renal failure, and/or the stage of the disease in a subject is predicted from the pattern of an inter-organ cross talk indicator derived from each of one or more organs other than the kidneys of the subject. Specifically, data of the subject regarding an inter-organ cross talk indicator in each of one or more organs other than the kidneys is obtained by performing a measurement method described in section II. 2. above, wherein the inter-organ cross talk indicator is derived from cells or tissue originating from each of the one or more organs; the data of the subject is compared with standard data 1 in one or more organs corresponding to the one or more organs from which the data of the subject originates, and similarity of patterns of the inter-organ cross talk indicators between the data of the subject and the standard data 1 is calculated, wherein the standard data 1 is extracted from a group of standard data 1 pedetermined for each of the at least one disease selected from the group consisting of declined kidney function, chronic kidney disease, and renal failure, or for each stage of the disease; and the presence of the at least one disease selected from the group consisting of declined kidney function, chronic kidney disease, and renal failure, and/or the stage of the disease is predicted by using, as a measure, the similarity. Specifically, this embodiment comprises the steps of: (1) obtaining data of the subject regarding an inter-organ cross talk indicator in each of one or more organs other than the kidneys in the subject, the inter-organ cross talk indicator being derived from cells or tissue originating from each of the one or more organs; (2) calculating, by comparing the data of the subject obtained in step (1) with standard data 1 in one or more organs corresponding to the one or more organs from which the data of the subject originates, similarity of patterns of the inter-organ cross talk indicators between the data of the subject and the standard data 1, more specifically calculating, by checking the name of the one or more organs from which the data of the subject obtained in step (1) orginates against the name of one or more organs stored in predetermined standard data 1, and comparing the pattern of the inter-organ cross talk indicator in the data of the subject with the pattern of the corresponding inter-organ cross talk indicator in the standard data 1 derived from the same organ as each of the one or more organs from which the data of the subject originates, similarity of the patterns of the inter-organ cross talk indicators, wherein the standard data 1 is extracted from a group of standard data 1 pedetermined for each of the at least one disease selected from the group consisting of declined kidney function, chronic kidney disease, and renal failure, or for each stage of the disease; and (3) determining that the subject has a disease corresponding to the standard data 1 and/or is in a stage of a disease corresponding to the standard data 1 when it is determined from the similarity of the patterns of the inter-organ cross talk indicators calculated in step (2) that both patterns are similar.

Step (1) may be performed in such a manner that the data of the subject is obtained by actually performing a measurement method described in the section II. 2., or in such a manner that the data of the subject already obtained is further put into the prediction device described later or the like. The method for calculating the similarity between the standard data 1 and the data of the subject in step (2), and the method for determining whether the standard data 1 and the data of the subject are similar in step (3), can be performed according to a method described in the above section "II. 1. Explanation of terms." Here, step (1) and step (2) are not necessarily performed consecutively in the same organization. For example, the data of the subject obtained in step (1) may be sent to a third-party organization to perform step (2) and the subsequent step.

Moreover, this embodiment may further comprise, before step (1), the following steps: (i) extracting the inter-organ cross talk indicator from the cells or tissue originating from each of the one or more organs other than the kidneys of the subject; and (ii) measuring the amount of the inter-organ cross talk indicator extracted in step (i). In this case, step (i) and step (ii) are not necessarily performed consecutively. For example, the inter-organ cross talk indicator obtained in step (i) may be sent to a third-party organization to perform step (ii). Step (ii) and step (1) are also not necessarily performed consecutively. The measurement result of the inter-organ cross talk indicator obtained in step (ii) may be sent to a third-party organization to perform step (1) and the subsequent steps.

Here, the method for calculating the similarity between the standard data 1 and the data of the subject, and the method for determining whether the standard data 1 and the data of the subject are similar are as described in the above section "II. iOrgans, 1. Explanation of terms."

As another embodiment, this embodiment also includes a method for obtaining information regarding the similarity of patterns of inter-organ cross talk indicators to predict the presence of at least one disease selected from the group consisting of declined kidney function, chronic kidney disease, and renal failure, and/or the stage of the disease in a subject, the method comprising steps (1) and (2) mentioned above, and a step of obtaining the information from step (2).

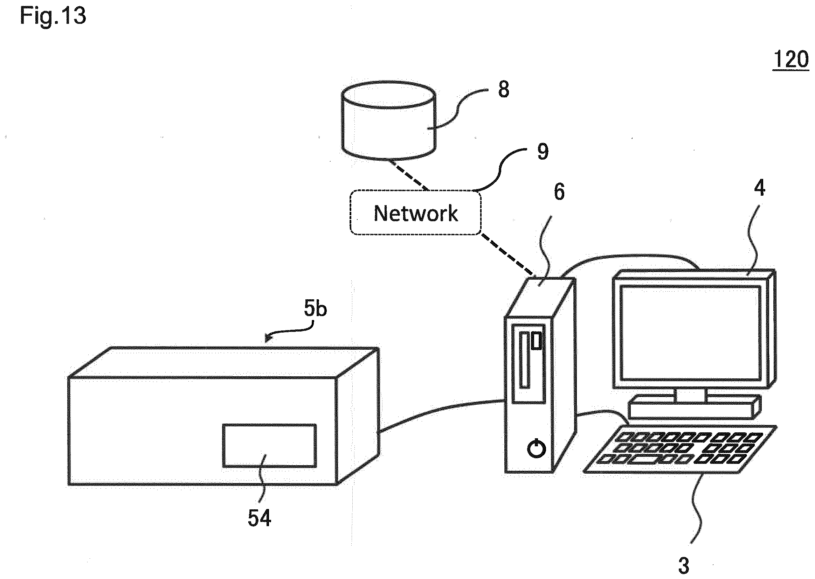

The prediction method may also comprise a step of obtaining a group of standard data 1 from a storage device 8 (Fig. 13) storing the group of standard data 1, or a step of obtaining one or more sets of standard data 1 from the storage device 8 storing the group of standard data 1.

## 3-2. System configuration

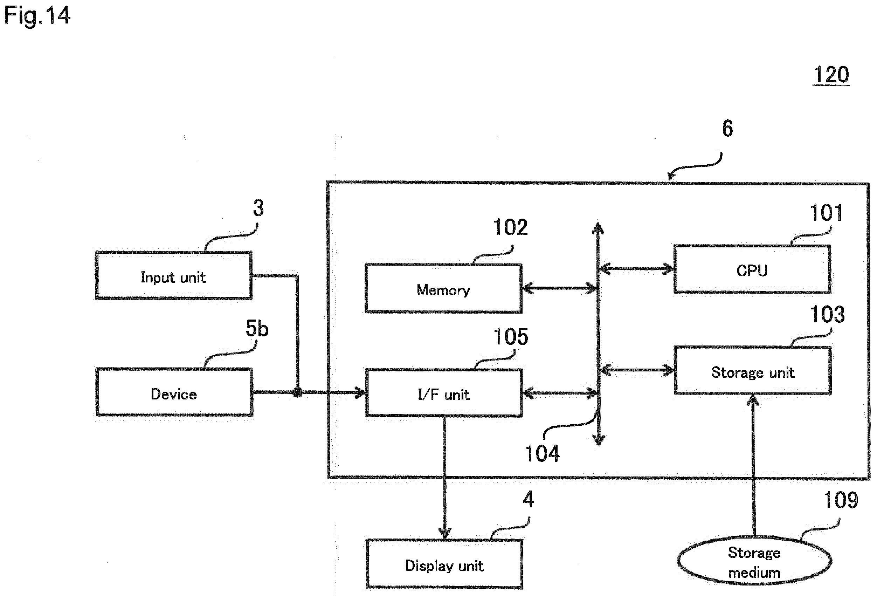

Fig. 13 is an overview of a system 120 according to a third embodiment of the present invention. Fig. 14 is a block diagram illustrating a hardware configuration of the system 120. The system 120 comprises an input unit 3, a display unit 4, a device 5b, and a prediction device 6.

The prediction device 6 includes, for example, a general-purpose personal computer; and comprises a CPU 101 for performing data processing described later, a memory 102 serving as a work area for data processing, a storage unit 103 for storing processed data, a bus 104 for transmitting data between the units, and an interface unit 105 (hereinafter referred to as "I/F unit") for performing data input and output between the prediction device and external devices. The input unit 3 and the display unit 4 are connected to the prediction device 6. The input unit 3 includes, for example, a keyboard; and the display unit 4 includes, for example, a liquid crystal display. The input unit 3 and the display unit 4 may be integrated and implemented as a display with a touch panel. The prediction device 6 need not be a single device; and the CPU 101, the memory 102, the storage unit 103, and the like may be located in separate places, and connected via a network. The prediction device may also be a device that omits the input unit 3 and the display unit 4, and that does not require an operator.

The prediction device 6 and the device 5b are also not necessarily located in one place, and may be configured such that the devices located in separate places are communicatively connected to each other via a network.

In the explanation below, a process performed by the prediction device 6 means a process performed by the CPU 101 of the prediction device 6 based on a prediction program unless otherwise specified. The CPU 101 temporarily stores necessary data (such as intermediate data being processed) in the memory 102 that serves as a work area, and suitably stores data that is stored for a long period of time, such as computation results, in the storage unit 103.

The device 5b is a device for measuring the expression level of mRNA by the RNA-Seq method, and comprises a sequence analysis unit 54. A sample subjected to a reaction for RNA-Seq is set in the sequence analysis unit 54, and analysis of nucleotide sequences is performed in the sequence analysis unit 54.

The device 5b is connected to the prediction device 6 by a wired or wireless connection. The device 5b A/D converts the measurement value of mRNA and transmits it as digital data to the prediction device 6. Therefore, the prediction device 6 can obtain, as digital data that can be computed, the measurement value of mRNA. In this embodiment, digital data from the device 5b is referred to as "data of a subject regarding an inter-organ cross talk indicator," or simply referred to as "data of a subject."

As described above, the hardware configuration of each of the input unit 3, display unit 4, device 5b, and prediction device 6 of the system 120 may be the same as that of each of the input unit 3, display unit 4, device 5b, and screening device 1 of the system 100 shown in Fig. 1; or that of each of the input unit 3, display unit 4, device 5b, and screening device 2 of the system 110 shown in Fig. 5.

The system 120 may also comprise a storage device 8 storing standard data 1 or a group of standard data 1, the storage device 8 being connected to the prediction device via a network. A network 9 is, for example, a communication medium, such as the internet, virtual private network (VPN), wide area network (WAN), or public switched telephone network (PSTN), and is not limited as long as it enables communication between the storage device 8 and the prediction device 6. Specifically, the system 120 may be a prediction system for predicting the presence of a disease and/or the stage of the disease in each of one or more organs other than the kidneys in a subject affected with at least one disease selected from the group consisting of declined kidney function, chronic kidney disease, and renal failure, the system comprising the storage device 8 storing standard data 1 or a group of standard data 1 and the prediction device 6 described later.

## 3-3. Prediction device

The present invention includes, as the third embodiment, a device for predicting the presence of at least one disease selected from the group consisting of declined kidney function, chronic kidney disease, and renal failure, and/or the stage of the disease in a subject, the device comprising the following computation means:

means for obtaining data of the subject regarding an inter-organ cross talk indicator in each of one or more organs other than the kidneys in the subject, the inter-organ cross talk indicator being derived from cells or tissue originating from each of the one or more organs;
means for calculating, by comparing the data of the subject with standard data in one or more organs corresponding to the one or more organs from which the data of the subject originates, similarity of patterns of the inter-organ cross talk indicators between the data of the subject and the standard data 1, wherein the standard data is extracted from a group of standard data 1 predetermined for each of the at least one disease selected from the group consisting of declined kidney function, chronic kidney disease, and renal failure, or for each stage of the disease; and
means for predicting the presence of the at least one disease selected from the group consisting of declined kidney function, chronic kidney disease, and renal failure, and/or the stage of the disease, by using, as a measure, the similarity of patterns of the inter-organ cross talk indicators calculated by the comparison means.

Here, the method for calculating the similarity between the standard data 1 and the data of the subject, and the method for determining whether the standard data 1 and the data of the subject are similar are as described in the above section "II. iOrgans, 1. Explanation of terms."

In this embodiment, the presence of at least one disease selected from the group consisting of declined kidney function, chronic kidney disease, and renal failure, and/or the stage of the disease in a subject, can be predicted by the system 120 (Figs. 13 and 14) comprising the prediction device 6 as the prediction device described above.

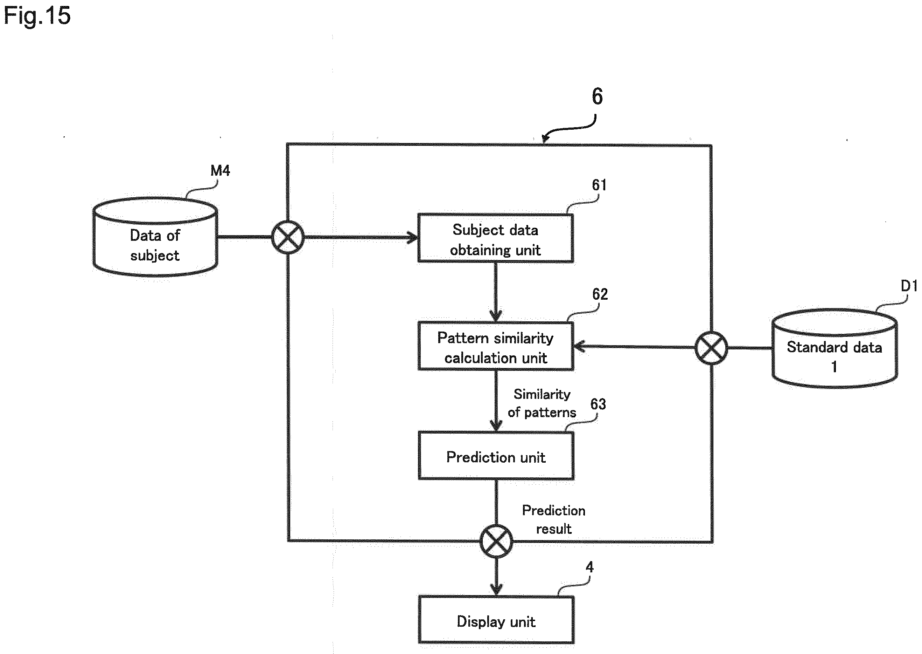

Fig. 15 is a block diagram illustrating functions of the prediction device 6 according to the third embodiment of the present invention. The prediction device 6 comprises a subject data obtaining unit 61, a pattern similarity calculation unit 62, and a prediction unit 63. These functional blocks are implemented by installing the prediction program according to the present invention in the storage unit 103 or the memory 102 of the prediction device 6, and causing the CPU 101 to execute the program. With this structure, the prediction device 6 carries out the prediction method described later in the section "II. iOrgans, 3-5. Prediction method." The subject data obtaining means, pattern similarity calculation means, and prediction means recited in the claims correspond to the subject data obtaining unit 61, pattern similarity calculation unit 62, and prediction unit 63 shown in Fig. 15, respectively.

In other words, the prediction device 6 is a prediction device for predicting the presence of at least one disease selected from the group consisting of declined kidney function, chronic kidney disease, and renal failure, and/or the stage of the disease in a subject, the device executing the following computation functions by the CPU 101:

a function for obtaining data of the subject regarding an inter-organ cross talk indicator in each of one or more organs other than the kidneys, the inter-organ cross talk indicator being derived from cells or tissue originating from each of the one or more organs;
a function for calculating, by comparing the data of the subject obtained by the subject data obtaining function with standard data 1 in one or more organs corresponding to the one or more organs from which the data of the subject originates, similarity of patterns of the inter-organ cross talk indicators between the data of the subject and the standard data 1, wherein the standard data 1 is extracted from a group of standard data 1 predetermined for each of the at least one disease selected from the group consisting of declined kidney function, chronic kidney disease, and renal failure, or for each stage of the disease; and
a function for predicting the presence of the at least one disease selected from the group consisting of declined kidney function, chronic kidney disease, and renal failure, and/or the stage of the disease, by using, as a measure, the similarity of patterns of the inter-organ cross talk indicators calculated by the pattern similarity calculation function.

The prediction device may also comprise a function for obtaining a group of standard data 1 from the storage device 8 storing the group of standard data 1, or a function for obtaining one or more sets of standard data 1 from the storage device 8 storing the group of standard data 1.

In this embodiment, subject data M4 of an inter-organ cross talk indicator measured in device 5b is put into the prediction device 6 from the device 5b. Standard data 1 (D1) is stored outside the prediction device 6, and put into the prediction device 6 via, for example, the internet.

The subject data M4 may be put into the prediction device 6 from a third-party organization (not shown) via a network. The subject data M4 and the standard data D1 may be stored in the storage unit 103 or the memory 102 of the prediction device 6 beforehand.

Moreover, the functional blocks, i.e., the subject data obtaining unit 61, the pattern similarity calculation unit 62, and the prediction unit 63, are not necessarily executed by a single CPU, and may be processed by multiple CPUs in a distributed manner. For example, these functional blocks may be configured such that the function of the subject data obtaining unit 61 is executed by a CPU of a first computer, and such that the functions of the pattern similarity calculation unit 62 and the prediction unit 63 are executed by a CPU of a second computer, i.e., another computer.

3-4. Prediction program

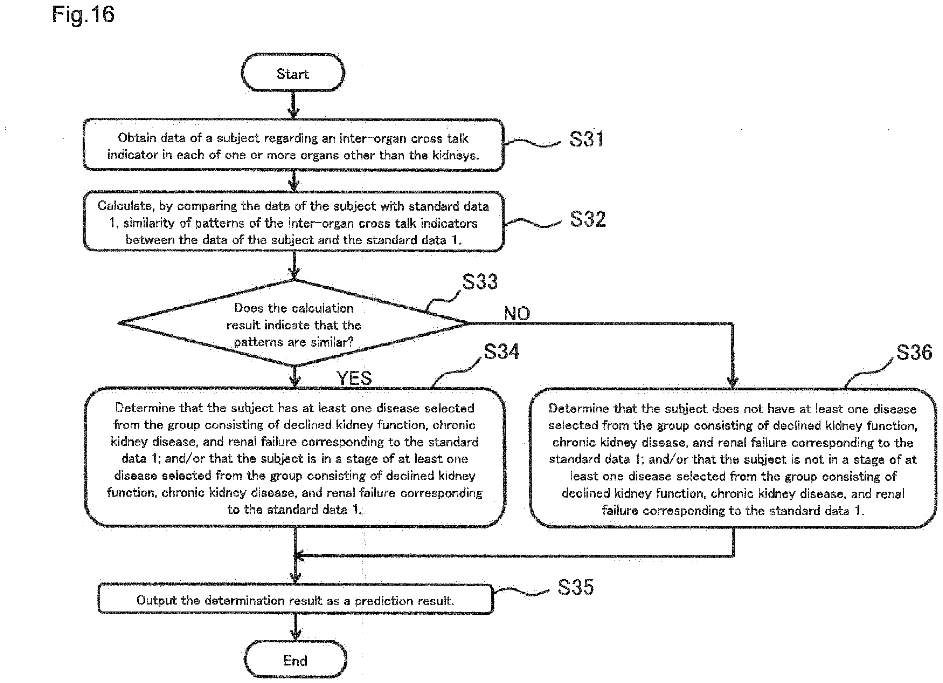

In order to carry out the processing for steps S31 to S36 in Fig. 16 below, the prediction device 6 stores the prediction program according to the present invention in the storage unit 103 beforehand, for example, in an executable format (for example, a form in which the program can be produced by conversion from a programming language using a compiler). The prediction device 6 carries out the processing using the prediction program stored in the storage unit 103.

Specifically, the prediction program according to the third embodiment of the present invention is a prediction program that, when executed by a computer, causes the computer to carry out the following processing to predict the presence of at least one disease selected from the group consisting of declined kidney function, chronic kidney disease, and renal failure, and/or the stage of the disease in a subject:

processing of obtaining data of the subject regarding an inter-organ cross talk indicator in each of one or more organs other than the kidneys in the subject, the inter-organ cross talk indicator being derived from cells or tissue originating from each of the one or more organs;
processing of calculating, by comparing the data of the subject obtained by the subject data obtaining processing with standard data 1 in one or more organs corresponding to the one or more organs from which the data of the subject originates, similarity of patterns of the inter-organ cross talk indicators between the data of the subject and the standard data 1, wherein the standard data 1 is extracted from a group of standard data 1 predetermined for each of the at least one disease selected from the group consisting of declined kidney function, chronic kidney disease, and renal failure, or for each stage of the disease; and
processing of predicting the presence of the at least one disease selected from the group consisting of declined kidney function, chronic kidney disease, and renal failure, and/or the stage of the disease, by using, as a measure, the similarity of patterns of the inter-organ cross talk indicators calculated by the pattern comparison processing.

The prediction program may also comprise processing of obtaining a group of standard data 1 from the storage device 8 storing the group of standard data 1, or processing of obtaining one or more sets of standard data 1 from the storage device 8 storing the group of standard data 1.

In this embodiment, as shown in Fig. 14, the prediction program is stored in a computer-readable non-transitory tangible storage medium 109, such as a CD-ROM, and installed to the prediction device 6 from the storage medium 109; alternatively, the prediction device 6 may be connected to the internet (not shown) to download the program code of the prediction program via the internet. To cause a computer to carry out the computation processing described above, the prediction program according to the present invention may be linked to another program stored in the storage unit 103 or the memory 102. For example, the prediction program may be linked to statistical analysis software mentioned in the above section "II. iOrgans, 1. Explanation of terms," and the pattern similarity calculation processing may be carried out using the statistical analysis software.

3-5. Prediction method

The prediction device 6 according to the third embodiment of the present invention carries out the prediction method according to the third embodiment of the present invention. The prediction method according to the third embodiment of the present invention is a method for predicting the presence of at least one disease selected from the group consisting of declined kidney function, chronic kidney disease, and renal failure, and/or the stage of the disease in a subject, the method comprising the steps of:

calculating, by comparing data of the subject regarding an inter-organ cross talk indicator in each of one or more organs other than the kidneys in the subject, the inter-organ cross talk indicator being derived from cells or tissue originating from each of the one or more organs, with standard data 1 in one or more organs corresponding to the one or more organs from which the data of the subject originates, similarity of patterns of the inter-organ cross talk indicators between the data of the subject and the standard data 1, wherein the standard data 1 is extracted from a group of standard data 1 predetermined for each of the at least one disease selected from the group consisting of declined kidney function, chronic kidney disease, and renal failure, or for each stage of the disease; and
predicting the presence of the at least one disease selected from the group consisting of declined kidney function, chronic kidney disease, and renal failure, and/or the stage of the disease by using, as a measure, the similarity of patterns of the inter-organ cross talk indicators calculated in the similarity calculation step.

The prediction method may also comprise a step of obtaining a group of standard data 1 from the storage device 8 storing the group of standard data 1, or a step of obtaining one or more sets of standard data 1 from the storage device 8 storing the group of standard data 1.

Fig. 16 is a flow chart illustrating a flow of data processing performed by the prediction device 6 according to the third embodiment of the present invention to carry out the prediction method described above. The processing for steps S31 to S36 shown in Fig. 16 is performed by the subject data obtaining unit 61, pattern similarity calculation unit 62, and prediction unit 63 shown in Fig. 15.

In step S31, the subject data obtaining unit 61 obtains subject data M4. The subject data M4 is a pattern of an inter-organ cross talk indicator in each of one or more organs other than the kidneys of a subject, the inter-organ cross talk indicator being derived from cells or tissue originating from each of the one or more organs, and transmitted from the device 5b to the prediction device 6.

In step S32, the pattern similarity calculation unit 62 compares the obtained subject data M4 with standard data 1 (D1), and calculates similarity of patterns of the inter-organ cross talk indicators. Specifically, the pattern similarity calculation unit 62 selects standard data 1 (D1) corresponding to the name of each of the one or more organs from which the subject data M4 originates, compares the pattern of the selected standard data 1 (D1) with the pattern of the subject data M4, and calculates the similarity between them. The method for calculating the similarity and the method for determining whether patterns are similar are as described in the above section "II. iOrgans, 1. Explanation of terms." The prediction program described in the above section "II. iOrgans, 3-4. Prediction program" may comprise program code of a program for causing the CPU 101 of the prediction device 6 to perform computation processing by the pattern similarity calculation unit 62; or, for example, may be linked to statistical analysis software mentioned in the above section "II. iOrgans, 1. Explanation of terms," to cause the CPU 101 to perform computation processing by the pattern similarity calculation unit 62 using the statistical analysis software.

In step S34, the prediction unit 63 predicts the presence of at least one disease selected from the group consisting of declined kidney function, chronic kidney disease, and renal failure, and/or the stage of the disease by using, as a measure, the similarity obtained in step S32. Specifically, when it is determined from the similarity that patterns are similar ("YES" in step 33), the prediction unit 63 determines in step S34 that the subject has at least one disease selected from the group consisting of declined kidney function, chronic kidney disease, and renal failure corresponding to a pattern in the standard data 1 (D1) that is similar to the subject data M4, and/or that the subject is in a stage of at least one disease selected from the group consisting of declined kidney function, chronic kidney disease, and renal failure corresponding to the standard data 1.

When it is determined from the similarity obtained in step S32 that patterns are not similar ("NO" in step 33), the prediction unit 63 determines in step S36 that the subject does not have at least one disease selected from the group consisting of declined kidney function, chronic kidney disease, and renal failure corresponding to the standard data 1, and/or that the subject is not in a stage of at least one disease selected from the group consisting of declined kidney function, chronic kidney disease, and renal failure corresponding to the standard data 1.

In step S35, the prediction unit 63 outputs the result determined in step S34 as prediction result data. In this embodiment, the prediction result is displayed on the display unit 4, and the prediction result data is stored in the storage unit 103 in the prediction device 6. The prediction result may be displayed on a display unit of an external computer terminal connected to the prediction device 6 via the internet, for example, a display unit of a computer terminal in a third-party organization, instead of displaying the prediction result on the display unit 4.

## 4. Forward iOrgans

### 4-1. Outline

In this embodiment, the presence of a disease and/or the stage of the disease in each of one or more organs other than the kidneys in a subject affected with at least one disease selected from the group consisting of declined kidney function, chronic kidney disease, and renal failure is predicted. Specifically, the presence of a disease and/or the stage of the disease in each of one or more organs other than the kidneys in a subject affected with at least one disease selected from the group consisting of declined kidney function, chronic kidney disease, and renal failure is predicted based on information regarding the stage of the at least one disease selected from the group consisting of declined kidney function, chronic kidney disease, and renal failure in the subject obtained from diagnostic result of the subject. This embodiment comprises the steps of (i) obtaining information regarding a stage of at least one disease selected from the group consisting of declined kidney function, chronic kidney disease, and renal failure in a subject from a diagnostic result of the subject; (ii) checking the information about the stage obtained in step (i) against standard data 2; (iii) determining, from the standard data 2, standard data $\alpha$ at a stage of the at least one disease selected from the group consisting of declined kidney function, chronic kidney disease, and renal failure corresponding to the information about the stage, based on the checking result obtained in step (ii), and extracting, from the standard data $\alpha$, a pattern of an inter-organ cross talk indicator corresponding to the stage in the subject in each of one or more organs other than the kidneys in the subject; (iv) checking the pattern of the inter-organ cross talk indicator extracted in step (iii) against known information about inter-organ cross talk indicators in diseases and/or stages of the diseases, and determining the presence of a disease and/or the stage of the disease in each of the one or more organs other than the kidneys corresponding to the pattern of the inter-organ cross talk indicator in each of the one or more organs other than the kidneys in the subject; and (v) further determining that the disease in each of the one or more organs other than the kidneys determined in step (iv) is a disease from which the subject may be suffering, and/or determining that the stage of the disease in each of the one or more organs other than the kidneys determined in step (iv) is a stage of a disease from which the subject is suffering.

In step (i), the diagnostic result of the subject is not limited as long as it is derived by, for example, a physician based on, for example, test results or a medical interview. The diagnostic result may be information obtained from, for example, a paper chart; or may be electronic data extracted from, for example, an electronic chart. In step (i), information about the stage of at least one disease selected from the group consisting of declined kidney function, chronic kidney disease, and renal failure in a subject is obtained as, for example, oral, written, or digital information, based on the diagnostic result of the subject. That is, the information about the stage of at least one disease selected from the group consisting of declined kidney function, chronic kidney disease, and renal failure in a subject is information regarding in what stage of at least one disease selected from the group consisting of declined kidney function, chronic kidney disease, and renal failure the subject is.

In checking the information regarding the stage obtained in step (i) against the standard data 2 in step (ii), for example, it is checked whether the name of the stage matches the name of the stage of the at least one disease selected from the group consisting of declined kidney function, chronic kidney disease, and renal failure assigned to each pattern of an inter-organ cross talk indicator in the standard data 2. The checking may be carried out visually; or may be carried out, for example, on database software, such as Microsoft (registered trademark) Excel (Microsoft Corporation) or Microsoft (registered trademark) Access (Microsoft Corporation), using the search function, the filtering function, or the like of the software.

In step (iii), patterns of inter-organ cross talk indicators linked with the name of the stage of the at least one disease selected from the group consisting of declined kidney function, chronic kidney disease, and renal failure in the subject are extracted based on the result of the checking in step (ii). The group of the extracted patterns of inter-organ cross talk indicators is determined to be standard data $\alpha$. Further, at least one organ other than the kidneys is selected from the names of organs linked with the corresponding patterns of inter-organ cross talk indicators contained in standard data $\alpha$, and the pattern of the inter-organ cross talk indicator in the at least one selected organ is extracted. Selection of at least one organ other than the kidneys, and extraction of the pattern of the inter-organ cross talk indicator in the at least one selected organ may be carried out visually; or may be carried out on the database software described above using the search function, the filtering function, or the like of the software.

In step (iv), the similarity between the extracted pattern of the inter-organ cross talk indicator in the at least one selected organ and the information regarding inter-organ cross talk indicators in diseases and/or stages of the diseases stored in a database of known information regarding diseases (e.g., DPC database (provided by Japanese Ministry of Health, Labour and Welfare), PubMed (provided by National Center for Biotechnology Information), Embase (provided by Elsevier), or Cochrane Library (Cochrane); hereinafter also referred to as "disease information database") is calculated and determined. Subsequently, the name of a disease, or the name of a stage of a disease, whose pattern of the inter-organ cross talk indicator stored in the disease information database is determined to be wholly or partially similar to the pattern of the inter-organ cross talk indicator in the at least one selected organ, is extracted. Whether the pattern of the inter-organ cross talk indicator in the at least one selected organ is similar to the known information can be determined according to a method for determining similarity described in the above section "II. iOrgans, 1. Explanation of terms." It can then be determined that the extracted disease is present in the selected organ other than the kidneys, or that the selected organ other than the kidneys is in the extracted stage of the disease. In this determination process, the pattern of the inter-organ cross talk indicator in the at least one selected organ can be compared with known information regarding the inter-organ cross talk indicator in healthy individuals to determine that the organ is normal.

In step (v), it is further determined that the disease in the selected organ other than the kidneys determined in step (iv) is a disease from which the subject may be suffering, and/or that the stage of the disease determined in step (iv) is a stage of a disease from which the subject is suffering. When multiple diseases are determined in step (iv), it can be determined that a disease showing high similarity to the pattern of the inter-organ cross talk indicator in the selected organ is a disease from which the subject may be suffering. When multiple stages of diseases are determined in step (iv), it can be determined that a stage of a disease showing high similarity to the pattern of the inter-organ cross talk indicator in the selected organ is the stage of the disease from which the subject may be suffering.

Further, this embodiment may also be a method for obtaining information to predict the presence of a disease and/or the stage of the disease in each of one or more organs other than the kidneys in a subject affected with at least one disease selected from the group consisting of declined kidney function, chronic kidney disease, and renal failure, the method comprising steps (i) to (iii) above; and further comprising, instead of step (iv) above, step (iv') of checking the pattern of the inter-organ cross talk indicator extracted in step (iii) against known information regarding inter-organ cross talk indicators in diseases and/or the stages of the diseases, and obtaining information regarding the presence of a disease and/or the stage of the disease in each of the one or more organs other than the kidneys corresponding to the pattern of the inter-organ cross talk indicator in each of the one or more organs other than the kidneys in the subject. Checking the extracted pattern of the inter-organ cross talk indicator against known information regarding inter-organ cross talk indicators in diseases and/or stages of the diseases is in accordance with step (iv) above.

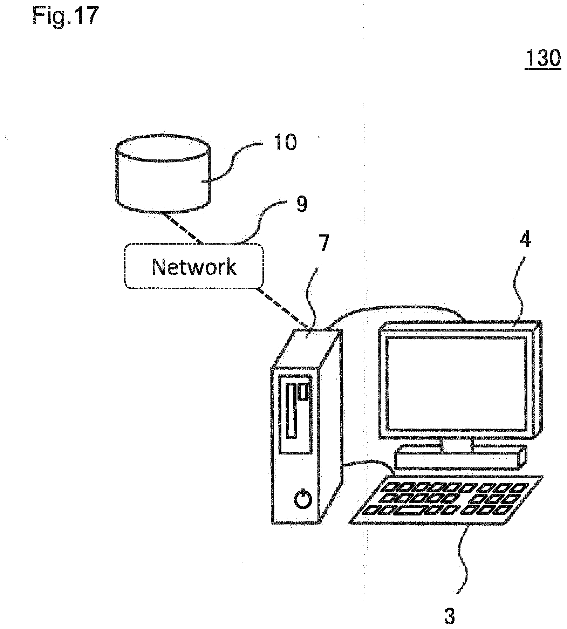

The prediction method above may also comprise a step of obtaining a group of standard data 2 from a storage device 10 (Fig. 17) storing the group of standard data 2, or a step of obtaining one or more sets of standard data 2 from the storage device 10 storing the group of standard data 2.

### 4-2. System configuration

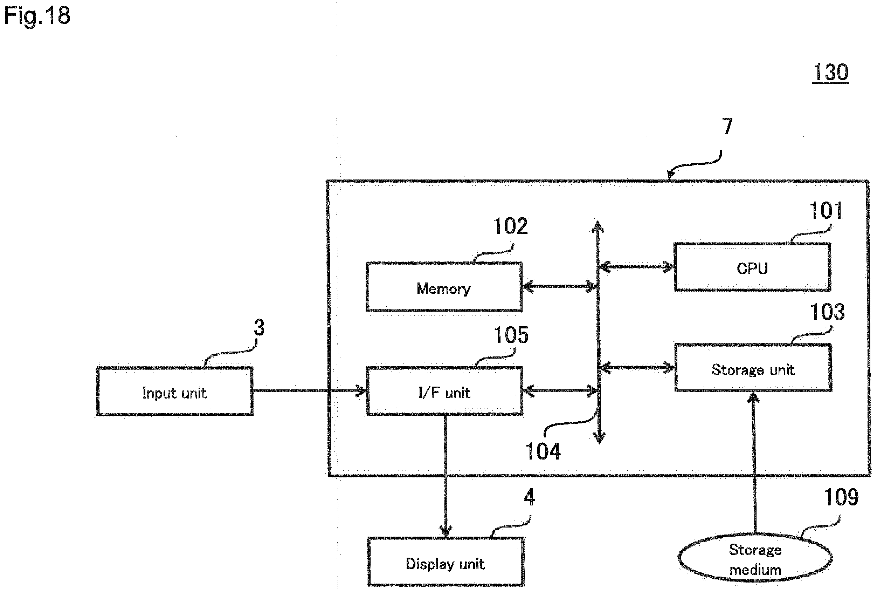

Fig. 17 is an overview of a system 130 according to a fourth embodiment of the present invention, and Fig. 18 is a block diagram illustrating a hardware configuration of the system 130. The system 130 comprises an input unit 3, a display unit 4, and a prediction device 7.

The prediction device 7 includes, for example, a general-purpose personal computer; and comprises a CPU 101 for performing data processing described later, a memory 102 serving as a work area for data processing, a storage unit 103 for storing processed data, a bus 104 for transmitting data between the units, and an interface unit 105 (hereinafter referred to as an "I/F unit") for performing data input and output between the prediction device and external devices. The input unit 3 and the display unit 4 are connected to the prediction device 7. The input unit 3 includes, for example, a keyboard, and the display unit 4 includes, for example, a liquid crystal display. The input unit 3 and the display unit 4 may be integrated and implemented as a display with a touch panel. The prediction device 7 need not be a single apparatus; and the CPU 101, the memory 102, the storage unit 103, and the like may be located in separate places, and connected via a network. The prediction device 7 may also be a device that omits the input unit 3 and the display unit 4 and that does not require an operator.

In the explanation below, a process performed by the prediction device 7 means a process performed by the CPU 101 of the prediction device 7 based on a prediction program unless otherwise specified. The CPU 101 temporarily stores necessary data (such as intermediate data being processed) in the memory 102 that serves as a work area, and suitably stores data that is stored for a long period of time, such as computation results, in the storage unit 103.

As described above, the hardware configuration of each of the input unit 3, display unit 4, and prediction device 7 of the system 130 may be the same as that of each of the input unit 3, display unit 4, and screening device 1 of the system 100 shown in Fig. 1; or that of each of the input unit 3, display unit 4, and screening device 2 of the system 110 shown in Fig. 5.

The system 130 may also comprise a storage device 10 storing standard data 2 or a group of standard data 2, the storage device 10 being connected to the prediction device via a network. A network 9 is, for example, a communication medium, such as the internet, virtual private network (VPN), wide area network (WAN), or public switched telephone network (PSTN), and is not limited as long as it enables communication between the storage device 10 and the prediction device 7. Specifically, the system 130 may be a prediction system for predicting the presence of a disease and/or the stage of the disease in each of one or more organs other than the kidneys in a subject affected with at least one disease selected from the group consisting of declined kidney function, chronic kidney disease, and renal failure, the system comprising the storage device 10 storing standard data 2 or a group of standard data 2 and the prediction device 7 described later.

4-3. Prediction device

The present invention includes, as the fourth embodiment, a device for predicting the presence of a disease and/or the stage of the disease in each of one or more organs other than the kidneys in a subject affected with at least one disease selected from the group consisting of declined kidney function, chronic kidney disease, and renal failure, the device comprising the following computation means:

means for obtaining information about a stage of the at least one disease selected from the group consisting of declined kidney function, chronic kidney disease, and renal failure in the subject;
means for checking the information about the stage obtained by the stage information obtaining means against standard data 2;
means for extracting a pattern of an inter-organ cross talk indicator in each of one or more organs other than the kidneys in the subject based on the result obtained by the stage information checking means; and
means for predicting the presence of a disease and/or the stage of the disease in each of the one or more organs other than the kidneys by using, as a measure, the pattern of the inter-organ cross talk indicator obtained by the pattern extraction means.

The prediction device may also comprise a function for obtaining a group of standard data 2 from the storage device 10 storing the group of standard data 2, or a function for obtaining one or more sets of standard data 2 from the storage device 10 storing the group of standard data 2.

In this embodiment, the presence of at least one disease selected from the group consisting of declined kidney function, chronic kidney disease, and renal failure and/or the stage of the disease in a subject can be predicted by the system 130 (Figs. 17 and 18) comprising the prediction device 7 as the prediction device above.

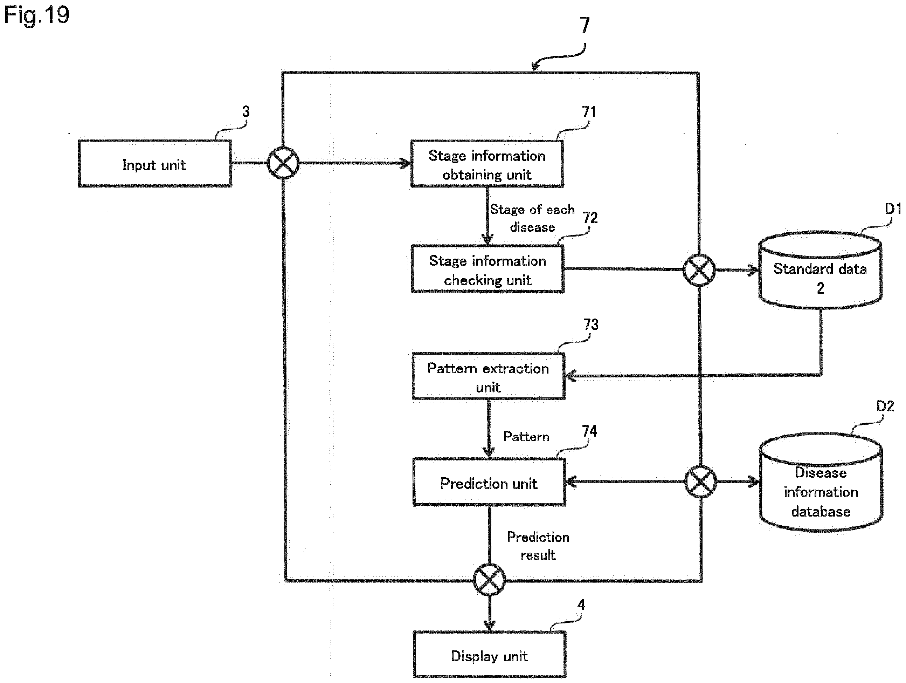

Fig. 19 is a block diagram illustrating functions of the prediction device 7 according to the fourth embodiment of the present invention. The prediction device 7 comprises a stage information obtaining unit 71, a stage information checking unit 72, a pattern extraction unit 73, and a prediction unit 74. These functional blocks are implemented by installing the prediction program according to the present invention in the storage unit 103 or the memory 102 of the prediction device 7, and causing the CPU 101 to execute the program. With this structure, the prediction device 7 carries out the prediction method described later in the section "II. iOrgans, 4-5. Prediction method." The stage information obtaining means, stage information checking means, pattern extraction means, and prediction means recited in the claims correspond to the stage information obtaining unit 71, stage information checking unit 72, pattern extraction unit 73, and prediction unit 74 shown in Fig. 19, respectively.

In other words, the prediction device 7 is a device for predicting the presence of a disease and/or the stage of the disease in each of one or more organs other than the kidneys in a subject affected with at least one disease selected from the group consisting of declined kidney function, chronic kidney disease, and renal failure, the device executing the following computation functions by the CPU 101:

a function for obtaining information about a stage of the at least one disease selected from the group consisting of declined kidney function, chronic kidney disease, and renal failure in the subject;
a function for checking the information about the stage obtained by the stage information obtaining function against standard data 2;

a function for extracting a pattern of an inter-organ cross talk indicator in each of one or more organs other than the kidneys in the subject based on the result obtained by the stage information checking function; and
a function for predicting the presence of a disease and/or the stage of the disease in each of the one or more organs other than the kidneys by using, as a measure, the pattern of the inter-organ cross talk indicator obtained by the pattern extraction function.

In this embodiment, standard data 2 (D1) and disease information database D2 are stored outside the prediction device 7, and put into the prediction device 7 via, for example, the internet.
The standard data 2 (D1) and the disease information database D2 may be stored in the storage unit 103 or the memory 102 of the prediction device 7 beforehand.
Moreover, the functional blocks, i.e., the stage information obtaining unit 71, the stage information checking unit 72, the pattern extraction unit 73, and the prediction unit 74, are not necessarily executed by a single CPU, and may be processed by multiple CPUs in a distributed manner. For example, these functional blocks may be configured such that the function of the stage information obtaining unit 71 is executed by a CPU of a first computer; and such that the functions of the stage information checking unit 72, the pattern extraction unit 73, and the prediction unit 74 are executed by a CPU of a second computer, i.e., another computer.

4-4. Prediction program

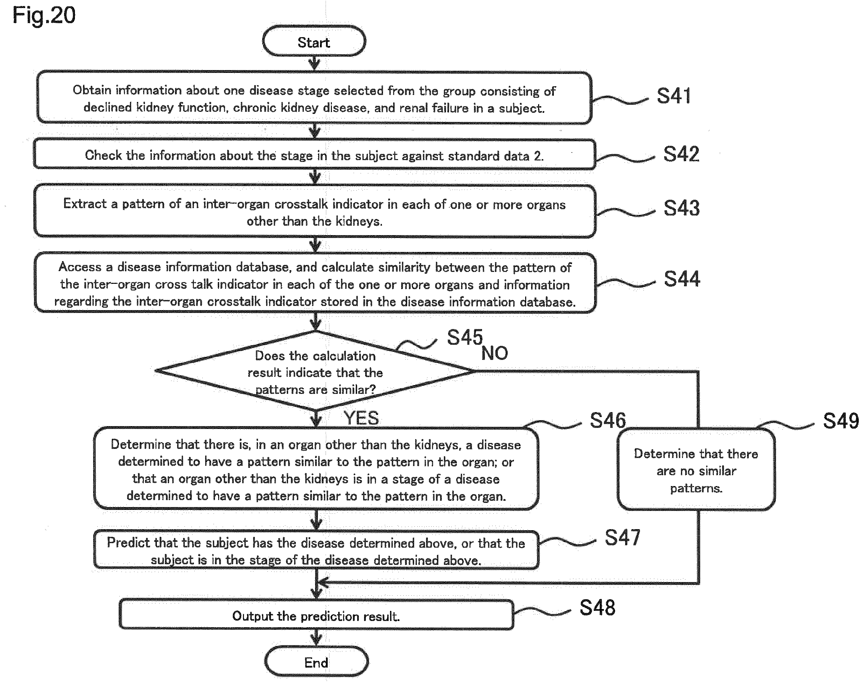

Moreover, in order to carry out steps S41 to S49 in Fig. 20 described below, the prediction device 7 stores the prediction program according to the present invention beforehand in the storage unit 103, for example, in an executable format. The prediction device 7 carries out processing using the prediction program stored in the storage unit 103.
Specifically, the prediction program according to the fourth embodiment of the present invention is a prediction program that, when executed by a computer, causes the computer to carry out the following processing to predict the presence of a disease and/or the stage of the disease in each of one or more organs other than the kidneys in a subject affected with at least one disease selected from the group consisting of declined kidney function, chronic kidney disease, and renal failure:

processing of obtaining information about a stage of the at least one disease selected from the group consisting of declined kidney function, chronic kidney disease, and renal failure in the subject;
processing of checking the information about the stage obtained by the stage information obtaining processing against standard data 2;
processing of extracting a pattern of an inter-organ cross talk indicator in each of one or more organs other than the kidneys in the subject based on the result obtained by the stage information checking processing; and
processing of predicting the presence of a disease and/or the stage of the disease in each of the one or more organs other than the kidneys by using, as a measure, the pattern of the inter-organ cross talk indicator obtained by the pattern extraction processing.

The prediction program may also comprise processing of obtaining a group of standard data 2 from the storage device 10 storing the group of standard data 2, or processing of obtaining two or more sets of standard data 2 from the storage device 10 storing the group of standard data 2.
In this embodiment, as shown in Fig. 18, the prediction program is stored in a computer-readable non-transitory tangible storage medium 109, such as a CD-ROM, and installed to the prediction device 7 from the storage medium 109; alternatively, the prediction device 7 may be connected to the internet (not shown) to download the program code of the prediction program via the internet. To cause a computer to carry out the computation processing described above, the prediction program according to the present invention may be linked to another program stored in the storage unit 103 or the memory 102. For example, the prediction program may be linked to the commercially available database software mentioned in the above section "II. iOrgans, 4-1. Outline," and the stage information checking processing and the pattern extraction processing may be carried out using the database software.

4-5. Prediction method

The prediction device 7 according to the fourth embodiment of the present invention carries out the prediction method according to the fourth embodiment of the present invention. The prediction method according to the fourth embodiment of the present invention is a method for predicting the presence of a disease and/or the stage of the disease in each of one or more organs other than the kidneys in a subject affected with at least one disease selected from the group consisting of declined kidney function, chronic kidney disease, and renal failure, the method comprising the steps of:

obtaining information about a stage of the at least one disease selected from the group consisting of declined kidney function, chronic kidney disease, and renal failure in the subject;
checking the information about the stage obtained in the stage information obtaining processing against standard data 2;
extracting a pattern of an inter-organ cross talk indicator in each of one or more organs other than the kidneys in the subject, based on the checking result obtained in the stage information checking step; and
predicting the presence of a disease and/or the stage of the disease in each of the one or more organs other than the kidneys, by using, as a measure, the pattern of the inter-organ cross talk indicator obtained in the pattern extraction processing.

The prediction method may also comprise a step of obtaining a group of standard data 2 from the storage device 10 storing the group of standard data 2, or a step of obtaining one or more sets of standard data 2 from the storage device 10 storing the group of standard data 2.

Fig. 20 is a flow chart illustrating a flow of data processing performed by the prediction device 7 according to the fourth embodiment of the present invention to carry out the prediction method described above. The processing of steps S41 to S49 shown in Fig. 20 is performed by the stage information obtaining unit 71, stage information checking unit 72, pattern extraction unit 73, and prediction unit 74 shown in Fig. 19.

In step S41, the stage information obtaining unit 71 obtains stage information. The stage information is information regarding in what stage of at least one disease selected from the group consisting of declined kidney function, chronic kidney disease, and renal failure the subject is. The stage information obtaining unit 71 obtains the stage information by, for example, operation of the input unit 3. The manner in which the stage information is obtained is not limited to this, and the stage information may be stored in the storage unit 103 of the prediction device 7 from an electronic chart or by any method, such as external data communication.

In step S42, the stage information checking unit 72 checks the stage information against standard data 2 (D1). Subsequently, in step S43, the pattern extraction unit 73 determines, from the standard data 2, standard data $\alpha$ at a stage of the at least one disease selected from the group consisting of declined kidney function, chronic kidney disease, and renal failure corresponding to the stage information, based on the checking result obtained in step S42; and extracts, from the standard data $\alpha$, a pattern of an inter-organ cross talk indicator corresponding to the stage in the subject in each of the one or more organs other than the kidneys in the subject. The name of one or more organs other than the kidneys to be extracted may be input from the input unit 3. The specific procedure for extraction is in accordance with the description in the above section "II. iOrgans, 4-1. Outline." The prediction program described in the above section "II. iOrgans, 4-4. Prediction program" may comprise program code of a program for causing the CPU 101 of the prediction device 7 to perform computation processing by the stage information checking unit 72 and the pattern extraction unit 73; or, for example, may be linked to commercially available database software mentioned above to cause the CPU 101 to perform the computation processing by the stage information checking unit 72 and the pattern extraction unit 73, using the database software.

In step S44, the prediction unit 74 suitably accesses a disease information database D2 downloaded outside of the prediction device 7 or downloaded in the memory 102 or the storage unit 103; and calculates and determines similarity between the pattern of the inter-organ cross talk indicator in each of the one or more organs extracted in step S43, and information regarding the inter-organ cross talk indicator stored in the disease information database. In step S46, it is determined that there is, in an organ other than the kidneys, a disease determined to have a pattern that is wholly or partially similar to the pattern of the inter-organ cross talk indicator in the organ ("YES" in step S45); or it is determined that an organ other than the kidneys is in a stage of a disease determined to have a pattern that is wholly or partially similar to the pattern of the inter-organ cross talk indicator in the organ ("YES" in step S45). In step S47, it is predicted that the subject is suffering from the disease determined in step S46, or that the subject is in the stage of the disease determined in step S46. The prediction program described in the above section "II. iOrgans, 4-4. Prediction program" may comprise program code of a program for causing the CPU 101 of the prediction device 7 to perform computation processing by the prediction unit 74; or, for example, may be linked to statistical analysis software mentioned in the above section "II. iOrgans, 1. Explanation of terms" to cause the CPU 101 to perform the computation processing by the prediction unit 74, using the statistical analysis software.

In step S48, the prediction unit 74 outputs the result predicted in step S47. In this embodiment, the prediction result is displayed on the display unit 4, and the prediction result is stored in the storage unit 103 in the prediction device 7. The prediction result may be displayed on a display unit of an external computer terminal connected to the prediction device 7 via the internet, for example, a display unit of a computer terminal in a third-party organization, instead of displaying the prediction result on the display unit 4.

When it is determined in step S45 from the result in step S44 that patterns are not similar ("NO" in step S45), the prediction unit 74 determines in step S49 that there are no similar patterns.

The specific procedure of each step is in accordance with the description in the above section "II. iOrgans, 4-1.

Outline."

5. Generation of standard data, and standard data

5-1. Generation of standard data

The present invention relates to a method for generating standard data 1 for use in "3. Reverse iOrgans" above, and a method for generating standard data 2 for use in "4. Forward iOrgans" above.

The method for generating standard data is a method for generating standard data of patterns of inter-organ cross talk indicators for use in prediction of the presence of at least one disease selected from the group consisting of declined kidney function, chronic kidney disease, and renal failure, and/or the stage of the disease in a subject, the method comprising the steps of:

extracting an inter-organ cross talk indicator from cells or tissue collected from each of one or more organs other than the kidneys of positive control(s) of a gold standard for each stage of the at least one disease selected from the group consisting of declined kidney function, chronic kidney disease, and renal failure;
extracting the inter-organ cross talk indicator from cells or tissue collected from each of the one or more organs other than the kidneys of negative control(s) of a gold standard;
identifying and quantifying the inter-organ cross talk indicators;
determining patterns of the inter-organ cross talk indicators, each of the patterns being determined from a relationship between an amount of the inter-organ cross talk indicator in the organ other than the kidneys in the positive control(s) affected with the at least one disease selected from the group consisting of declined kidney function, chronic kidney disease, and renal failure, and an amount of the corresponding inter-organ cross talk indicator in the same organ as the organ other than the kidneys in the negative control(s) without the at least one disease selected from the group consisting of declined kidney function, chronic kidney disease, and renal failure; and
associating the patterns of the inter-organ cross talk indicators with the corresponding stages of the one disease selected from the group consisting of declined kidney function, chronic kidney disease, and renal failure.

Specifically, the procedure for generating standard data 1 is a procedure as described later in the Examples.

First, cells or tissue is collected from one or more organs (e.g., adipose) other than the kidneys of negative control(s) and positive control(s) in each stage of at least one disease selected from the group consisting of declined kidney function, chronic kidney disease, and renal failure; and an inter-organ cross talk indicator is extracted. The extracted inter-organ cross talk indicator is then identified and quantified.

Next, patterns of inter-organ cross talk indicators are determined, each of the patterns being determined from the relationship (ratio) between the amount of an inter-organ cross talk indicator in an organ other than the kidneys of the positive control(s) affected with the at least one disease selected from the group consisting of declined kidney function, chronic kidney disease, and renal failure, and the amount of the corresponding inter-organ cross talk indicator in the organ other than the kidneys of the negative control(s) without the at least one disease selected from the group consisting of declined kidney function, chronic kidney disease, and renal failure. The determined patterns of inter-organ cross talk indicators are linked to the at least one disease selected from the group consisting of declined kidney function, chronic kidney disease, and renal failure; and stored in, for example, a storage device as standard data 1 or a group of standard data 1. Further, the standard data 1 or the group of standard data 1 can be stored in an external server.

Furthermore, the present invention includes a method for generating standard data 2.

The method for generating standard data is a method for generating standard data 2 of patterns of inter-organ cross talk indicators for use in prediction of the presence of a disease and/or the stage of the disease in each of one or more organs other than the kidneys in a subject affected with at least one disease selected from the group consisting of declined kidney function, chronic kidney disease, and renal failure, the method comprising the steps of:

extracting an inter-organ cross talk indicator from cells or tissue collected from each of one or more organs other than the kidneys of positive control(s) of a gold standard for each stage of the at least one disease selected from the group consisting of declined kidney function, chronic kidney disease, and renal failure;
extracting the inter-organ cross talk indicator from cells or tissue collected from each of the one or more organs other than the kidneys of negative control(s) of a gold standard;
identifying and quantifying the inter-organ cross talk indicators; and
determining patterns of the inter-organ cross talk indicators for each stage of the at least one disease selected from the group consisting of declined kidney function, chronic kidney disease, and renal failure, each of the patterns being determined from a relationship between an amount of the inter-organ cross talk indicator in the organ other than the kidneys in the positive control(s) affected with the at least one disease selected from the group consisting of declined kidney function, chronic kidney disease, and renal failure, and an amount of the corresponding inter-organ cross talk indicator in the same organ as the organ other than the kidneys in the negative control(s) without the at least one disease selected from the group consisting of declined kidney function, chronic kidney disease, and renal failure.

Specifically, the procedure for generating standard data 2 is a procedure as described later in the Examples.
First, cells or tissue are collected from one or more organs other than the kidneys collected from negative control(s) and positive control(s) affected with at least one disease selected from the group consisting of declined kidney function, chronic kidney disease, and renal failure, and an inter-organ cross talk indicator is extracted. The extracted inter-organ cross talk indicator is then identified and quantified.
Next, patterns of inter-organ cross talk indicators are determined for each stage of the at least one disease selected from the group consisting of declined kidney function, chronic kidney disease, and renal failure, each of the patterns being determined from the relationship (ratio) between the amount of an inter-organ cross talk indicator in an organ other than the kidneys collected from the positive control(s) affected with the at least one disease selected from the group consisting of declined kidney function, chronic kidney disease, and renal failure, and the amount of the corresponding inter-organ cross talk indicator in the organ other than the kidneys collected from the negative control(s) without the at least one disease selected from the group consisting of declined kidney function, chronic kidney disease, and renal failure. Such patterns of inter-organ cross talk indicators determined for each stage of the at least one disease selected from the group consisting of declined kidney function, chronic kidney disease, and renal failure are stored in, for example, a storage device as standard data 2 or a group of standard data 2. Further, the standard data 2 or the group of standard data 2 can be stored in an external server.

5-2. Standard data

The present invention includes standard data 1 or a group of standard data 1 generated by the method described above.
The standard data 1 is standard data of patterns of inter-organ cross talk indicators for use in prediction of the presence of at least one disease selected from the group consisting of declined kidney function, chronic kidney disease, and renal failure and/or the stage of the disease in a subject, the standard data being generated by the steps of:

extracting an inter-organ cross talk indicator from cells or tissue collected from each of one or more organs other than the kidneys of positive control(s) of a gold standard for each stage of the at least one disease selected from the group consisting of declined kidney function, chronic kidney disease, and renal failure;
extracting the inter-organ cross talk indicator from cells or tissue collected from each of the one or more organs other than the kidneys of negative control(s) of a gold standard;
identifying and quantifying the inter-organ cross talk indicators;
determining patterns of the inter-organ cross talk indicators, each of the patterns being determined from a relationship between an amount of the inter-organ cross talk indicator in the organ other than the kidneys in the positive control(s) affected with the at least one disease selected from the group consisting of declined kidney function, chronic kidney disease, and renal failure, and an amount of the corresponding inter-organ cross talk indicator in the same organ as the organ other than the kidneys in the negative control(s) without the at least one disease selected from the group consisting of declined kidney function, chronic kidney disease, and renal failure; and
associating the patterns of the inter-organ cross talk indicators with the corresponding stages of the at least one disease selected from the group consisting of declined kidney function, chronic kidney disease, and renal failure.

Generated standard data 1 or group of standard data 1 may be stored in the storage unit 103 or the memory 102 of the prediction device 6. Alternatively, generated standard data 1 may be stored in a storage device connected locally to the prediction device 6 or in an external storage device, for example, a storage device of a server, accessible via a network by the prediction device 6.
Furthermore, the present invention includes standard data 2 or a group of standard data 2 generated by the method described above.
The standard data 2 is standard data of patterns of inter-organ cross talk indicators for use in prediction of the presence of a disease and/or the stage of the disease in each of one or more organs other than the kidneys in a subject affected with at least one disease selected from the group consisting of declined kidney function, chronic kidney disease, and renal failure, the standard data being generated by the steps of:

extracting an inter-organ cross talk indicator from cells or tissue collected from each of one or more organs other than the kidneys of positive control(s) of a gold standard for each stage of the at least one disease selected from the group consisting of declined kidney function, chronic kidney disease, and renal failure;
extracting the inter-organ cross talk indicator from cells or tissue collected from each of the one or more organs other than the kidneys of negative control(s) of a gold standard;
identifying and quantifying the inter-organ cross talk indicators; and
determining patterns of the inter-organ cross talk indicators for each stage of the at least one disease selected from the group consisting of declined kidney function, chronic kidney disease, and renal failure, each of the patterns being determined from a relationship between an amount of the inter-organ cross talk indicator in the organ other than the kidneys in the positive control(s) affected with the at least one disease selected from the group consisting of declined kidney function, chronic kidney disease, and renal failure, and an amount of the corresponding inter-organ cross talk indicator in the same organ as the organ other than the kidneys in the negative control(s) without the at least one disease selected from the group consisting of declined kidney function, chronic kidney disease, and renal failure.

Generated standard data 2 or group of standard data 2 may be stored in the storage unit 103 or the memory 102 of the prediction device 7. Alternatively, generated standard data 2 or group of standard data 2 may be stored in a storage device connected locally to the prediction device 7 or in an external storage device, for example, a storage device of a server, accessible via a network by the prediction device 7.

6. Treatment method

R-iOrgans and F-iOrgans may further comprise a process for treating at least one disease selected from the group consisting of declined kidney function, chronic kidney disease, and renal failure. The process for treating the disease can be performed according to a known method. When it is determined that a subject has declined kidney function, first, a clinical state causing declined kidney function may be treated in order to slow progression of declined kidney function. Examples of such clinical states include lifestyle-related diseases, such as diabetes and hypertension; urinary diseases, such as urinary tract infection, urinary tract obstruction, glomerulonephritis, vascular disease in the kidneys (blood flow disorder), and drug-induced nephropathy due to an analgesic; and the like. Moreover, treatment such as blood-pressure control or dietary restriction may be performed in order to slow progression of chronic kidney disease. Further, a drug therapy etc. may be performed for abnormal bone metabolism associated with chronic kidney disease using, for example, a phosphate-binding agent when chronic kidney disease progresses. Further, if necessary, dialysis etc. may be performed.

Moreover, in the treatment of at least one disease selected from the group consisting of declined kidney function, chronic kidney disease, and renal failure, an antagonist of an Oscar protein can be used as an active ingredient in the treatment. Examples of the antagonist include soluble receptors of an Oscar protein. The soluble receptors comprise an amino acid sequence of a ligand-binding region in an Oscar protein. The soluble receptors may also comprise, for example, the Fc portion of an immunoglobulin or synthetic polymer polyethylene glycol (PEG) as a molecule other than an amino acid sequence of a ligand-binding region in the Oscar protein. In the case of humans, an example of such a soluble receptor is the soluble receptor set forth in NP_996554.2 (SEQ ID NO: 1), which is capable of inhibiting activation of the Oscar protein. In the case of humans, the soluble receptor comprises, among the sequence of the 1st to 228th amino acids of the amino acid sequence set forth in SEQ ID NO: 1, at least the sequence of the 1st to 220th amino acids, preferably the sequence of the 1st to 228th amino acids. The soluble receptor may comprise an amino acid sequence with at least 70%, preferably at least 80%, more preferably at least 85%, even more preferably at least 90%, still even more preferably 95% homology to the sequence of the 1st to 228th amino acids of the amino acid sequence set forth in SEQ ID NO: 1, in place of the amino acid sequence set forth in SEQ ID NO: 1. The soluble receptor is preferably, for example, a soluble receptor comprising a peptide comprising the amino acid sequence set forth in SEQ ID NO: 2; or a peptide having the same amino acid sequence as the amino acid sequence of the peptide, except that one to three amino acids are substituted, deleted, or inserted, the amino acid sequence being capable of inhibiting activation of human Oscar. In the case of mice, an example of such a soluble receptor is a soluble receptor set forth in NP_783440.1 (SEQ ID NO: 3), which is capable of inhibiting activation of Oscar. The soluble receptor comprises, among the sequence of the 1st to 235th amino acids of the amino acid sequence set forth in SEQ ID NO: 3, at least the sequence of the 1st to 225th amino acids, and preferably the sequence of the 1st to 235th amino acids. The soluble receptor may comprise an amino acid sequence with at least 70%, preferably at least 80%, more preferably at least 85%, even more preferably at least 90%, still even more preferably 95% homology to the sequence of the 1st to 235th amino acids of the amino acid sequence set forth in SEQ ID NO: 3, in place of the amino acid sequence set forth in SEQ ID NO: 3. The soluble receptor is preferably a soluble receptor comprising a peptide comprising the amino acid sequence set forth in SEQ ID NO: 4; or a peptide having the same amino acid sequence as the amino acid sequence of the peptide, except that one to three amino acids are substituted, deleted, or inserted, the amino acid sequence being capable of inhibiting activation of mouse Oscar.

When systemically administered, the antagonist can be administered in an amount of 0.01 to 1,000 mg/day per kg of body weight of an adult.

When locally administered, the antagonist can be administered in an amount of 0.01 to 100 mg per $cm^2$ of target tissue.

The antagonist can be prepared by combining it with pharmaceutically acceptable carriers or additives.

Examples

The present invention is described in more detail below with reference to examples. The present invention, however, should not be construed as limited to the examples.

Experimental Example 1: Establishment of model mice of chronic kidney disease

Model mice of chronic kidney disease were obtained by feeding mice a diet with high phosphorus content after unilateral nephrectomy. As a control, mice were obtained by feeding them a normal diet after a sham operation.

1. Unilateral nephrectomy

After mice (C57BL/6, 8 weeks old, male) were anesthetized by intraperitoneal administration of Avertin (250 mg/kg), the skin was incised from the back. The right renal artery and vein, and ureter were ligated. After cutting on the distal side of the ligation, the right kidney was removed, and the incision was closed. The control mice were subjected to a sham operation. In the sham operation, the right renal artery and vein, and ureter were exposed, and the incision was closed without ligation. In order to wait for the mice to completely recover from operative stress, the mice were fed a normal diet (containing 0.35% inorganic phosphorus) for 4 weeks.

2. Phosphorus overload

From 4 weeks after the completion of the operation (12 weeks old), the unilaterally nephrectomized mice were given a diet with high phosphorus content (containing 2.0% inorganic phosphorus) (hereinafter also referred to as the "kidney disease group"). The sham-operated mice were given a normal diet (containing 0.35% inorganic phosphorus) (hereinafter also referred to as the "Sham group").

The model mice of chronic kidney disease were obtained by a modification of the method described in Hu M.C. et al. (J Am Soc Nephrol 22, 124-136, 2011). In Hu M.C. et al., the remaining kidney (left kidney) is subjected to ischemia-reperfusion injury at the time of unilateral nephrectomy in Item 1 above. However, in this modification, ischemia-reperfusion was not performed.

Experimental Example 2: CKD evaluation

1. 4 weeks after start of diet with high phosphorus content (16 weeks old)

In histological observation of the kidneys, a decrease in the stainability of proximal tubule cells to an acidophilic dye (Eosin) was observed in Hematoxylin-Eosin (HE) staining, suggesting that some change occurred in mitochondria. There was no finding of evident fibrosis in the renal interstitium; however, a mild degree of inflammatory cell infiltration was observed.

In physiological observation, an increase in urinary proteins was observed; however, an increase in blood creatinine or phosphorus, or a decrease in creatinine clearance, was not observed.

In gene expression observation by the RNA-Seq analysis described later, it was observed that expression of fibrosis markers (Collagen-1, TGF-1) and inflammatory markers (IL-1, MCP-1) was enhanced.

2. 8 weeks after start of diet with high phosphorus content (20 weeks old)

In histological observation of the kidneys, fibrosis and cell infiltration in the renal interstitium, i.e., kidney fibrosis, was observed. In physiological observation, an increase in urinary proteins and an increase in blood creatinine, phosphorus, and FGF23 were observed; however, creatinine clearance rather increased, indicating the state of so-called hyperfiltration.

3. 12 weeks after start of diet with high phosphorus content (24 weeks old)

12 weeks after the start of the diet with high phosphorus content, the above-mentioned findings observed 8 weeks after the start of the diet with high phosphorus content further progressed. In some cases, calcification was observed at and around the cortico-medullary junction of the kidney. However, none of the mice died.

Example 1: Observation of inter-organ cross talk by R-iOrgans

1. Collection of each organ

Organs or tissue (the pancreas, skull, brain, pituitary gland(PG), kidney, adrenal glands(AG), liver, spleen, thymus, heart, lungs, submandibular gland(SG), thyroid gland(TG), aorta, skeletal muscle (muscle, SM), skin, testis, adipose, eyes, stomach, jejunum, ileum, and colon) was collected 1 week (early stage:E) and 4 weeks (middle stage:M) after the start of the diet with high phosphorus content (the normal diet in the Sham group).

The animals from which the organs and tissue were to be collected were fasted overnight from the day before dissection and euthanized by cervical dislocation without anesthesia, and the organs and tissue were collected. After the wet weights of the collected organs and tissue were measured, the organs and tissue were rapidly frozen in liquid nitrogen and stored at - 80°C.

2. Analysis of RNA

(1) Extraction of RNA from each tissue

Each cryopreserved tissue was individually homogenized in TRIzol Reagent (Life Technologies) with a Cell Destroyer PS1000 (Pro Sense, Inc.) or PT 10-35 6T Polytron homogenizer (KINEATICA). After incubation at room temperature for 5 minutes to separate proteins, 0.2 mL of chloroform was added per mL of TRIzol, and the tube was capped. Subsequently, the mixture was vortexed vigorously for 15 seconds. After the vortexing, the mixture was incubated at room temperature for 3 minutes and centrifuged at 12,000 g for 15 minutes at 4°C, and the RNA-containing aqueous layer was collected in a fresh tube. An equal amount of 70% ethanol was added to the collected aqueous layer, and mixed. Then, 700 μL of the mixture was applied to each RNeasy Mini column (Qiagen), and purified RNAs were collected according to the RNeasy Mini kit (Qiagen) standard protocol. The quality and concentration of each of the collected RNAs was evaluated by 1% agarose electrophoresis and NanoDrop.

(2) Obtaining RNA-Seq data

RNA-Seq data was obtained using the samples described above by the following procedure in Macrogen Japan Corp.

i. Quality check

Quality testing of the samples was performed based on the following item.

- Concentration measurement and quality check using an Agilent 2200 TapeStation System

ii. Preparation of sample

A library for sequencing was prepared using 500 to 1000 ng of each total RNA sample that passed the quality testing as a template with Illumina's TruSeq RNA Sample Prep Kit according to the standard protocol in the following manner.

(a) Purification of poly(A)+RNA using Oligo-dT beads
(b) Poly(A)+RNA fragmentation
(c) Reverse transcription 2nd strand cDNA synthesis
(d) Terminus repair and 3'A addition
(e) Adapter ligation

Note: The adapters contain index tags for identification of specimens.

(f) PCR amplification
(g) Purification and removal of low-molecular-weight substances (<200 bp) using AMPure XP beads

iii. Obtaining data using next-generation sequencer

Nucleotide sequence data was obtained using an Illumina HiSeq 4000 next-generation sequencer by reading 100 bases according to the paired-end method.

(3) Analysis of RNA-Seq data

(3-1) Analysis of output data obtained using next-generation sequencer

The following information processing was carried out for the output data.

i. Base calling: text data of nucleotide sequences was obtained from the output raw data of analysis (image data).
ii. Filtering: selection of read data by predetermined filtering was performed.
iii. Sorting based on index sequences: sample data was sorted based on index information.

(3-2) Secondary analysis of output data

The data file (Fastq format) obtained using Illumina HiSeq was uploaded on Galaxy (https://usegalaxy.org/) downloaded to a local server. Thereafter, analysis was carried out using Bowtie2 (http://bowtie-bio.sourceforge.net/bowtie2/index.shtml) to map each sequence to mouse genome map information mm10. The BAM file obtained using Bowtie2 was analyzed using Cufflinks (http://cole-trapnell-lab.github.io/cufflinks/) to calculate FPKM (RPKM) for each gene.

(3-3) Results

Values were calculated by dividing the RNA expression level of each gene (FPKM value) by the expression level of the corresponding RNA (FPKM value) in the mice of the sham group (hereinafter also referred to as "CKD/sham"). Genes in which CKD/sham is more than 1 or less than 1 were classified as group 2, genes in which CKD/sham is more than 1.5 or less than 0.67 were classified as group 3, genes in which CKD/sham is more than 2 or less than 0.5 were classified as group 4, and genes in which CKD/sham is more than 5 or less than 0.2 were classified as group 5 (Fig. 22). All FPKM values of less than 1 were treated as "1." Fig. 23 lists genes in which Sham>1 and CKD/Sham>5, genes in which Sham<1 and CKD/Sham>10, and genes in which Sham>10 and CKD/Sham <0.3. In particular, the genes of Fig. 23 were considered to best reflect the state of kidney function.

From the above results, proteins or mRNAs expressed from the genes listed in Figs. 22 and 23 were considered to be preferably used as markers for predicting kidney function. Moreover, proteins or mRNAs expressed from the genes listed in Figs. 22 and 23 were considered to be preferably used as an inter-organ cross talk indicator in the iOrgans technology.

Example 2: Comparison of Pattern similarities of inter-organ cross talk indicators in each disease

To demonstrate that the presence of chronic kidney disease and a stage can be predicted from the pattern of an inter-organ cross talk indicator obtained from cells or tissue of each organ collected at each stage in the model mice of chronic kidney disease according to the theory of R-iOrgans, correlation coefficients between patterns of expression of RNAs of group 5 shown in Fig. 22 in individual organs were determined for each stage of diseases such as myocardial infarction. The correlation coefficients were determined by modifying Spearman's rank correlation method and Z-score method.

The similarity was calculated based on correlation coefficients of the patterns of inter-organ cross talk indicators between two organs.

1. Spearman's rank correlation

Calculation was performed by using function cor (method="spearman") of analysis software R. Tables 4-1 to 4-8 show the results.

Table 4-1

| | | | Model mice of chronic kidney disease | | | | | | | | | | | | | | | | | |
|---|---|---|---|---|---|---|---|---|---|---|---|---|---|---|---|---|---|---|---|---|
| | | | Brain Early stage | Brain Middle stage | Brain Late stage | Heart Early stage | Heart Middle stage | Heart Late stage | Kidney Early stage | Kidney Middle stage | Kidney Late stage | Liver Early stage | Liver Middle stage | Liver Late stage | Lung Early stage | Lung Middle stage | Lung Late stage | Pancreas Early stage | Pancreas Middle stage | Pancreas Late stage |
| Model mice of chronic kidney disease | Brain | Early stage | 1.00 | 0.27 | 0.07 | 0.08 | 0.15 | 0.06 | -0.06 | 0.05 | -0.04 | -0.09 | 0.10 | 0.00 | 0.12 | -0.05 | 0.07 | 0.01 | 0.10 | 0.03 |
| | Brain | Middle stage | 0.27 | 1.00 | 0.00 | 0.06 | 0.26 | 0.06 | -0.15 | 0.03 | -0.04 | -0.18 | 0.19 | 0.01 | 0.13 | -0.21 | 0.04 | -0.01 | 0.20 | 0.06 |
| | Brain | Late stage | 0.07 | 0.00 | 1.00 | 0.06 | 0.01 | 0.06 | 0.06 | 0.05 | 0.00 | 0.08 | 0.01 | 0.04 | 0.02 | 0.12 | 0.10 | 0.03 | -0.03 | 0.01 |
| | Heart | Early stage | 0.08 | 0.06 | 0.06 | 1.00 | 0.24 | 0.36 | 0.07 | 0.02 | 0.06 | 0.00 | 0.04 | 0.05 | 0.12 | 0.08 | 0.12 | 0.04 | 0.04 | 0.07 |
| | Heart | Middle stage | 0.15 | 0.26 | 0.01 | 0.24 | 1.00 | 0.32 | -0.06 | 0.03 | -0.04 | -0.12 | 0.14 | 0.02 | 0.14 | -0.05 | 0.08 | 0.03 | 0.20 | 0.09 |
| | Heart | Late stage | 0.06 | 0.06 | 0.06 | 0.36 | 0.32 | 1.00 | 0.09 | 0.02 | 0.12 | 0.01 | 0.06 | 0.09 | 0.12 | 0.08 | 0.13 | 0.04 | 0.08 | 0.10 |
| | Kidney | Early stage | -0.06 | -0.15 | 0.06 | 0.07 | -0.06 | 0.09 | 1.00 | 0.42 | 0.49 | 0.21 | -0.01 | 0.13 | 0.04 | 0.17 | 0.05 | 0.05 | -0.08 | 0.03 |
| | Kidney | Middle stage | 0.05 | 0.03 | 0.05 | 0.02 | 0.03 | 0.02 | 0.42 | 1.00 | 0.57 | 0.07 | 0.16 | 0.10 | 0.01 | -0.04 | 0.05 | -0.01 | 0.04 | 0.04 |
| | Kidney | Late stage | -0.04 | -0.04 | 0.00 | 0.06 | -0.04 | 0.12 | 0.49 | 0.57 | 1.00 | 0.11 | 0.07 | 0.17 | -0.03 | 0.00 | -0.04 | -0.01 | -0.01 | 0.04 |
| | Liver | Early stage | -0.09 | -0.18 | 0.08 | 0.00 | -0.12 | 0.01 | 0.21 | 0.07 | 0.11 | 1.00 | 0.33 | 0.45 | 0.02 | 0.24 | 0.07 | 0.08 | -0.11 | 0.02 |
| | Liver | Middle stage | 0.10 | 0.19 | 0.01 | 0.04 | 0.14 | 0.06 | -0.01 | 0.16 | 0.07 | 0.33 | 1.00 | 0.50 | 0.09 | -0.04 | 0.10 | -0.01 | 0.12 | 0.06 |
| | Liver | Late stage | 0.00 | 0.01 | 0.04 | 0.05 | 0.02 | 0.09 | 0.13 | 0.10 | 0.17 | 0.45 | 0.50 | 1.00 | 0.04 | 0.10 | 0.08 | 0.06 | 0.01 | 0.08 |
| | Lung | Early stage | 0.12 | 0.13 | 0.02 | 0.12 | 0.14 | 0.12 | 0.04 | 0.01 | -0.03 | 0.02 | 0.09 | 0.04 | 1.00 | 0.23 | 0.36 | 0.06 | 0.08 | 0.05 |
| | Lung | Middle stage | -0.05 | -0.21 | 0.12 | 0.08 | -0.05 | 0.08 | 0.17 | -0.04 | 0.00 | 0.24 | -0.04 | 0.10 | 0.23 | 1.00 | 0.29 | 0.09 | -0.07 | 0.02 |
| | Lung | Late stage | 0.07 | 0.04 | 0.10 | 0.12 | 0.08 | 0.13 | 0.05 | 0.05 | -0.04 | 0.07 | 0.10 | 0.08 | 0.36 | 0.29 | 1.00 | 0.04 | 0.01 | 0.05 |
| | Pancreas | Early stage | 0.01 | -0.01 | 0.03 | 0.04 | 0.03 | 0.04 | 0.05 | -0.01 | -0.01 | 0.08 | -0.01 | 0.06 | 0.06 | 0.09 | 0.04 | 1.00 | 0.17 | 0.15 |
| | Pancreas | Middle stage | 0.10 | 0.20 | -0.03 | 0.04 | 0.20 | 0.08 | -0.08 | 0.04 | -0.01 | -0.11 | 0.12 | 0.01 | 0.08 | -0.07 | 0.01 | 0.17 | 1.00 | 0.17 |
| | Pancreas | Late stage | 0.03 | 0.06 | 0.01 | 0.07 | 0.09 | 0.10 | 0.03 | 0.04 | 0.04 | 0.02 | 0.06 | 0.08 | 0.05 | 0.02 | 0.05 | 0.15 | 0.17 | 1.00 |
| | Muscle | Early stage | 0.12 | 0.11 | 0.06 | 0.14 | 0.21 | 0.15 | 0.03 | -0.01 | -0.05 | 0.03 | 0.08 | 0.06 | 0.14 | 0.09 | 0.11 | 0.04 | 0.09 | 0.07 |
| | Muscle | Middle stage | 0.02 | -0.06 | 0.09 | 0.16 | 0.18 | 0.16 | 0.11 | -0.06 | -0.02 | 0.15 | 0.02 | 0.07 | 0.08 | 0.30 | 0.08 | 0.08 | 0.04 | 0.06 |
| | Muscle | Late stage | 0.03 | -0.11 | 0.11 | 0.18 | 0.12 | 0.23 | 0.18 | 0.04 | 0.08 | 0.18 | 0.01 | 0.09 | 0.10 | 0.25 | 0.13 | 0.09 | -0.02 | 0.07 |
| | Skin | Early stage | 0.12 | 0.11 | -0.01 | 0.07 | 0.16 | 0.10 | 0.01 | -0.05 | -0.07 | -0.02 | 0.08 | 0.02 | 0.15 | 0.06 | 0.10 | 0.00 | 0.06 | 0.04 |
| | Skin | Middle stage | 0.00 | -0.04 | 0.06 | 0.06 | 0.01 | 0.07 | 0.07 | 0.10 | 0.02 | 0.05 | 0.05 | 0.04 | 0.04 | 0.11 | 0.09 | 0.00 | -0.06 | 0.01 |
| | Skin | Late stage | 0.11 | 0.14 | -0.02 | 0.08 | 0.20 | 0.11 | -0.09 | -0.04 | -0.07 | -0.10 | 0.09 | 0.01 | 0.10 | -0.05 | 0.08 | -0.03 | 0.09 | 0.07 |
| | Spleen | Early stage | 0.05 | 0.01 | 0.04 | 0.06 | 0.08 | 0.07 | 0.07 | 0.01 | 0.00 | 0.06 | 0.04 | 0.04 | 0.16 | 0.10 | 0.11 | 0.05 | -0.01 | 0.01 |
| | Spleen | Middle stage | 0.01 | 0.00 | 0.07 | 0.11 | 0.05 | 0.10 | 0.09 | 0.00 | 0.08 | 0.05 | -0.01 | 0.08 | 0.05 | 0.17 | 0.07 | -0.01 | 0.00 | 0.05 |
| | Spleen | Late stage | 0.01 | 0.04 | 0.03 | 0.13 | 0.04 | 0.11 | 0.07 | 0.02 | 0.11 | 0.00 | -0.01 | 0.07 | 0.08 | 0.10 | 0.10 | 0.02 | 0.04 | 0.07 |
| | Testis | Early stage | 0.05 | 0.11 | -0.06 | 0.05 | 0.09 | 0.03 | -0.04 | -0.05 | -0.01 | -0.08 | 0.03 | -0.03 | 0.01 | -0.09 | -0.02 | -0.02 | 0.07 | 0.04 |
| | Testis | Middle stage | 0.06 | 0.04 | 0.07 | -0.03 | -0.02 | -0.05 | -0.04 | 0.18 | -0.06 | 0.02 | 0.14 | 0.01 | 0.02 | -0.03 | 0.10 | 0.00 | 0.00 | -0.02 |
| | Testis | Late stage | 0.05 | 0.14 | -0.11 | 0.03 | 0.10 | 0.03 | -0.06 | 0.02 | 0.07 | -0.10 | 0.08 | 0.03 | 0.01 | -0.10 | -0.02 | -0.03 | 0.07 | 0.03 |
| | Adipose | Early stage | 0.04 | 0.03 | 0.03 | 0.10 | 0.08 | 0.08 | 0.08 | -0.05 | 0.01 | 0.04 | 0.00 | 0.04 | 0.08 | 0.11 | 0.04 | 0.07 | 0.08 | 0.04 |
| | Adipose | Middle stage | 0.18 | 0.29 | -0.03 | 0.11 | 0.30 | 0.07 | -0.15 | -0.05 | -0.14 | -0.19 | 0.13 | -0.03 | 0.15 | -0.12 | 0.08 | -0.02 | 0.22 | 0.05 |
| | Adipose | Late stage | 0.18 | 0.24 | 0.01 | 0.15 | 0.28 | 0.15 | -0.10 | -0.05 | -0.14 | -0.13 | 0.12 | 0.00 | 0.16 | -0.04 | 0.14 | 0.01 | 0.19 | 0.08 |

Table 4-2

| | | | Model mice of chronic kidney disease | | | | | | | | | | | | | | |
|---|---|---|---|---|---|---|---|---|---|---|---|---|---|---|---|---|---|
| | | | Muscle Early stage | Muscle Middle stage | Muscle Late stage | Skin Early stage | Skin Middle stage | Skin Late stage | Spleen Early stage | Spleen Middle stage | Spleen Late stage | Testis Early stage | Testis Middle stage | Testis Late stage | Adipose Early stage | Adipose Middle stage | Adipose Late stage |
| Model mice of chronic kidney disease | Brain | Early stage | 0.12 | 0.02 | 0.03 | 0.12 | 0.00 | 0.11 | 0.05 | 0.01 | 0.01 | 0.05 | 0.06 | 0.05 | 0.04 | 0.18 | 0.18 |
| | Brain | Middle stage | 0.11 | -0.06 | -0.11 | 0.11 | -0.04 | 0.14 | 0.01 | 0.00 | 0.04 | 0.11 | 0.04 | 0.14 | 0.03 | 0.29 | 0.24 |
| | Brain | Late stage | 0.06 | 0.09 | 0.11 | -0.01 | 0.06 | -0.02 | 0.04 | 0.07 | 0.03 | -0.06 | 0.07 | -0.11 | 0.03 | -0.03 | 0.01 |
| | Heart | Early stage | 0.14 | 0.16 | 0.18 | 0.07 | 0.06 | 0.08 | 0.06 | 0.11 | 0.13 | 0.05 | -0.03 | 0.03 | 0.10 | 0.11 | 0.15 |
| | Heart | Middle stage | 0.21 | 0.18 | 0.12 | 0.16 | 0.01 | 0.20 | 0.08 | 0.05 | 0.04 | 0.09 | -0.02 | 0.10 | 0.08 | 0.30 | 0.28 |
| | Heart | Late stage | 0.15 | 0.16 | 0.23 | 0.10 | 0.07 | 0.11 | 0.07 | 0.10 | 0.11 | 0.03 | -0.05 | 0.03 | 0.08 | 0.07 | 0.15 |
| | Kidney | Early stage | 0.03 | 0.11 | 0.18 | 0.01 | 0.07 | -0.09 | 0.07 | 0.09 | 0.07 | -0.04 | -0.04 | -0.06 | 0.08 | -0.15 | -0.10 |
| | Kidney | Middle stage | -0.01 | -0.06 | 0.04 | -0.05 | 0.10 | -0.04 | 0.01 | 0.00 | 0.02 | -0.05 | 0.18 | 0.02 | -0.05 | -0.05 | -0.05 |
| | Kidney | Late stage | -0.05 | -0.02 | 0.08 | -0.07 | 0.02 | -0.07 | 0.00 | 0.08 | 0.11 | -0.01 | -0.06 | 0.07 | 0.01 | -0.14 | -0.14 |
| | Liver | Early stage | 0.03 | 0.15 | 0.18 | -0.02 | 0.05 | -0.10 | 0.06 | 0.05 | 0.00 | -0.08 | 0.02 | -0.10 | 0.04 | -0.19 | -0.13 |
| | Liver | Middle stage | 0.08 | 0.02 | 0.01 | 0.08 | 0.05 | 0.09 | 0.04 | -0.01 | -0.01 | 0.03 | 0.14 | 0.08 | 0.00 | 0.13 | 0.12 |
| | Liver | Late stage | 0.06 | 0.07 | 0.09 | 0.02 | 0.04 | 0.01 | 0.04 | 0.08 | 0.07 | -0.03 | 0.01 | 0.03 | 0.04 | -0.03 | 0.00 |
| | Lung | Early stage | 0.14 | 0.08 | 0.10 | 0.15 | 0.04 | 0.10 | 0.16 | 0.05 | 0.08 | 0.01 | 0.02 | 0.01 | 0.08 | 0.15 | 0.16 |
| | Lung | Middle stage | 0.09 | 0.30 | 0.25 | 0.06 | 0.11 | -0.05 | 0.10 | 0.17 | 0.10 | -0.09 | -0.03 | -0.10 | 0.11 | -0.12 | -0.04 |
| | Lung | Late stage | 0.11 | 0.08 | 0.13 | 0.10 | 0.09 | 0.08 | 0.11 | 0.07 | 0.10 | -0.02 | 0.10 | -0.02 | 0.04 | 0.08 | 0.14 |
| | Pancreas | Early stage | 0.04 | 0.08 | 0.09 | 0.00 | 0.00 | -0.03 | 0.05 | -0.01 | 0.02 | -0.02 | 0.00 | -0.03 | 0.07 | -0.02 | 0.01 |
| | Pancreas | Middle stage | 0.09 | 0.04 | -0.02 | 0.06 | -0.06 | 0.09 | -0.01 | 0.00 | 0.04 | 0.07 | 0.00 | 0.07 | 0.08 | 0.22 | 0.19 |
| | Pancreas | Late stage | 0.07 | 0.06 | 0.07 | 0.04 | 0.01 | 0.07 | 0.01 | 0.05 | 0.07 | 0.04 | -0.02 | 0.03 | 0.04 | 0.05 | 0.08 |
| | Muscle | Early stage | 1.00 | 0.39 | 0.34 | 0.17 | 0.03 | 0.12 | 0.09 | 0.00 | -0.02 | 0.06 | 0.02 | 0.00 | 0.13 | 0.18 | 0.21 |
| | Muscle | Middle stage | 0.39 | 1.00 | 0.47 | 0.18 | 0.03 | 0.09 | 0.09 | 0.06 | 0.01 | 0.00 | -0.04 | -0.04 | 0.18 | 0.05 | 0.14 |
| | Muscle | Late stage | 0.34 | 0.47 | 1.00 | 0.09 | 0.08 | 0.02 | 0.10 | 0.10 | 0.05 | -0.03 | 0.01 | -0.08 | 0.17 | 0.00 | 0.12 |
| | Skin | Early stage | 0.17 | 0.18 | 0.09 | 1.00 | 0.18 | 0.46 | 0.04 | 0.13 | 0.09 | 0.11 | -0.02 | 0.02 | 0.10 | 0.19 | 0.23 |
| | Skin | Middle stage | 0.03 | 0.03 | 0.08 | 0.18 | 1.00 | 0.21 | -0.02 | 0.19 | 0.11 | -0.03 | 0.05 | -0.02 | -0.09 | 0.00 | 0.03 |
| | Skin | Late stage | 0.12 | 0.09 | 0.02 | 0.46 | 0.21 | 1.00 | 0.04 | 0.12 | 0.11 | 0.10 | -0.04 | 0.08 | -0.01 | 0.22 | 0.25 |
| | Spleen | Early stage | 0.09 | 0.09 | 0.10 | 0.04 | -0.02 | 0.04 | 1.00 | 0.09 | 0.06 | -0.01 | 0.00 | 0.00 | 0.03 | 0.07 | 0.09 |
| | Spleen | Middle stage | 0.00 | 0.06 | 0.10 | 0.13 | 0.19 | 0.12 | 0.09 | 1.00 | 0.45 | -0.02 | -0.10 | -0.01 | 0.01 | 0.03 | 0.06 |
| | Spleen | Late stage | -0.02 | 0.01 | 0.05 | 0.09 | 0.11 | 0.11 | 0.06 | 0.45 | 1.00 | 0.01 | -0.08 | 0.02 | 0.03 | 0.05 | 0.07 |
| | Testis | Early stage | 0.06 | 0.00 | -0.03 | 0.11 | -0.03 | 0.10 | -0.01 | -0.02 | 0.01 | 1.00 | -0.05 | 0.17 | 0.08 | 0.13 | 0.12 |
| | Testis | Middle stage | 0.02 | -0.04 | 0.01 | -0.02 | 0.05 | -0.04 | 0.00 | -0.10 | -0.08 | -0.05 | 1.00 | 0.00 | -0.04 | 0.00 | 0.01 |
| | Testis | Late stage | 0.00 | -0.04 | -0.08 | 0.02 | -0.02 | 0.08 | 0.00 | -0.01 | 0.02 | 0.17 | 0.00 | 1.00 | 0.03 | 0.08 | 0.05 |
| | Adipose | Early stage | 0.13 | 0.18 | 0.17 | 0.10 | -0.09 | -0.01 | 0.03 | 0.01 | 0.03 | 0.08 | -0.04 | 0.03 | 1.00 | 0.30 | 0.30 |
| | Adipose | Middle stage | 0.18 | 0.05 | 0.00 | 0.19 | 0.00 | 0.22 | 0.07 | 0.03 | 0.05 | 0.13 | 0.00 | 0.08 | 0.30 | 1.00 | 0.59 |
| | Adipose | Late stage | 0.21 | 0.14 | 0.12 | 0.23 | 0.03 | 0.25 | 0.09 | 0.06 | 0.07 | 0.12 | 0.01 | 0.05 | 0.30 | 0.59 | 1.00 |

Table 4-3

| | | | Brain Early stage | Brain Middle stage | Brain Late stage | Heart Early stage | Heart Middle stage | Heart Late stage | Kidney Early stage | Kidney Middle stage | Kidney Late stage | Liver Early stage | Liver Middle stage | Liver Late stage | Lung Early stage | Lung Middle stage | Lung Late stage | Pancreas Early stage | Pancreas Middle stage | Pancreas Late stage |
|---|---|---|---|---|---|---|---|---|---|---|---|---|---|---|---|---|---|---|---|---|
| Moded mice of myocardial infarction | Brain | Early stage | 0.10 | -0.01 | 0.18 | 0.08 | 0.02 | 0.07 | 0.10 | 0.08 | 0.06 | 0.11 | 0.03 | 0.05 | 0.07 | 0.14 | 0.08 | 0.06 | -0.03 | 0.03 |
| | Brain | Middle stage | 0.07 | 0.02 | 0.06 | 0.02 | 0.00 | 0.03 | 0.06 | 0.04 | 0.09 | 0.02 | 0.00 | 0.04 | -0.05 | 0.00 | -0.06 | 0.00 | 0.00 | 0.02 |
| | Brain | Late stage | -0.08 | -0.10 | -0.06 | -0.04 | -0.05 | -0.03 | 0.01 | 0.00 | 0.00 | 0.02 | -0.03 | -0.05 | -0.01 | -0.04 | -0.01 | -0.03 | -0.06 | 0.02 |
| | Heart | Early stage | -0.04 | -0.03 | 0.02 | 0.04 | -0.04 | 0.10 | 0.14 | 0.13 | 0.25 | 0.11 | 0.06 | 0.12 | 0.06 | 0.09 | 0.04 | 0.06 | -0.01 | 0.04 |
| | Heart | Middle stage | -0.04 | -0.09 | 0.00 | -0.01 | -0.14 | 0.03 | 0.12 | 0.19 | 0.29 | 0.06 | -0.01 | 0.07 | -0.08 | -0.01 | -0.10 | 0.03 | -0.04 | 0.01 |
| | Heart | Late stage | -0.07 | -0.11 | 0.00 | -0.01 | -0.18 | 0.01 | 0.16 | 0.15 | 0.26 | 0.08 | -0.02 | 0.07 | -0.06 | -0.01 | -0.06 | 0.02 | -0.10 | -0.01 |
| | Kidney | Early stage | 0.03 | -0.07 | 0.11 | 0.02 | -0.01 | 0.02 | 0.21 | 0.11 | 0.07 | 0.16 | 0.02 | 0.08 | 0.06 | 0.18 | 0.07 | 0.05 | -0.06 | 0.03 |
| | Kidney | Middle stage | -0.03 | -0.06 | 0.04 | -0.04 | -0.05 | 0.02 | 0.23 | 0.25 | 0.37 | 0.13 | 0.03 | 0.13 | -0.04 | 0.08 | -0.03 | 0.05 | -0.03 | 0.03 |
| | Kidney | Late stage | 0.01 | 0.00 | -0.05 | -0.01 | 0.00 | 0.00 | -0.13 | -0.07 | -0.10 | -0.05 | -0.01 | -0.05 | 0.01 | -0.06 | -0.02 | -0.01 | 0.03 | -0.03 |
| | Liver | Early stage | 0.01 | -0.03 | 0.05 | 0.04 | 0.03 | 0.06 | 0.10 | 0.02 | 0.03 | 0.26 | 0.20 | 0.23 | 0.07 | 0.12 | 0.08 | 0.03 | -0.03 | 0.03 |
| | Liver | Middle stage | -0.04 | -0.05 | 0.00 | 0.00 | -0.07 | 0.02 | 0.10 | 0.17 | 0.26 | 0.16 | 0.09 | 0.17 | -0.10 | -0.04 | -0.07 | 0.00 | -0.02 | 0.01 |
| | Liver | Late stage | 0.00 | -0.02 | -0.01 | 0.01 | -0.03 | 0.01 | -0.01 | -0.02 | -0.02 | -0.09 | -0.08 | -0.11 | 0.03 | -0.03 | -0.01 | 0.01 | 0.04 | 0.00 |
| | Lung | Early stage | 0.04 | 0.02 | 0.06 | 0.04 | 0.06 | 0.09 | 0.05 | 0.04 | 0.04 | 0.08 | 0.10 | 0.10 | 0.26 | 0.16 | 0.21 | 0.08 | 0.07 | 0.04 |
| | Lung | Middle stage | 0.06 | 0.12 | -0.01 | 0.02 | 0.06 | 0.03 | -0.06 | 0.08 | 0.07 | -0.07 | 0.09 | 0.02 | -0.04 | -0.22 | -0.04 | 0.01 | 0.11 | 0.01 |
| | Lung | Late stage | -0.02 | -0.11 | 0.03 | 0.00 | -0.11 | 0.01 | 0.10 | 0.08 | 0.06 | 0.08 | 0.00 | -0.01 | 0.00 | 0.04 | 0.03 | 0.03 | -0.06 | -0.03 |
| | Pancreas | Early stage | 0.01 | 0.08 | 0.01 | 0.01 | 0.08 | 0.05 | -0.05 | -0.06 | -0.04 | -0.02 | 0.03 | 0.00 | 0.05 | 0.00 | 0.01 | 0.04 | 0.04 | -0.02 |
| | Pancreas | Middle stage | 0.02 | -0.03 | 0.05 | 0.02 | 0.03 | 0.04 | 0.01 | -0.03 | -0.05 | 0.06 | 0.01 | 0.04 | 0.03 | 0.07 | 0.05 | 0.03 | 0.03 | 0.00 |
| | Pancreas | Late stage | 0.02 | 0.01 | 0.01 | 0.04 | 0.07 | 0.05 | 0.01 | -0.10 | -0.06 | 0.01 | 0.00 | 0.01 | 0.10 | 0.07 | 0.05 | 0.06 | 0.05 | 0.02 |
| | Muscle | Early stage | 0.03 | -0.04 | 0.11 | 0.04 | 0.09 | 0.12 | 0.13 | 0.03 | 0.01 | 0.16 | 0.07 | 0.08 | 0.13 | 0.22 | 0.12 | 0.07 | 0.00 | 0.03 |
| | Muscle | Middle stage | 0.01 | -0.02 | 0.05 | 0.02 | 0.03 | 0.01 | 0.06 | 0.02 | 0.00 | 0.03 | -0.03 | -0.03 | 0.01 | 0.04 | 0.02 | 0.00 | -0.03 | 0.00 |
| | Muscle | Late stage | -0.04 | -0.04 | -0.03 | -0.04 | -0.07 | -0.04 | -0.01 | -0.02 | 0.01 | 0.00 | 0.01 | -0.01 | -0.02 | -0.05 | -0.04 | 0.01 | -0.01 | -0.01 |
| | Skin | Early stage | -0.03 | -0.17 | 0.07 | 0.02 | -0.04 | 0.07 | 0.10 | 0.00 | 0.00 | 0.17 | -0.03 | 0.06 | 0.01 | 0.26 | 0.09 | 0.04 | -0.11 | -0.01 |
| | Skin | Middle stage | 0.00 | -0.01 | -0.03 | -0.04 | -0.08 | -0.06 | -0.04 | 0.12 | 0.00 | 0.02 | 0.08 | -0.01 | -0.06 | -0.10 | 0.01 | -0.04 | -0.02 | -0.04 |
| | Skin | Late stage | -0.03 | -0.03 | 0.00 | -0.02 | -0.06 | -0.01 | 0.01 | 0.04 | 0.02 | 0.04 | 0.01 | -0.02 | 0.01 | 0.06 | 0.07 | 0.02 | 0.00 | -0.01 |
| | Spleen | Early stage | -0.03 | -0.11 | 0.09 | 0.02 | -0.04 | 0.06 | 0.17 | 0.04 | 0.04 | 0.14 | -0.05 | 0.07 | 0.04 | 0.18 | 0.03 | 0.04 | -0.08 | 0.04 |
| | Spleen | Middle stage | 0.08 | 0.09 | -0.03 | 0.00 | 0.10 | -0.01 | -0.09 | -0.01 | -0.07 | -0.01 | 0.11 | 0.01 | 0.07 | -0.07 | 0.03 | 0.03 | 0.09 | 0.00 |
| | Spleen | Late stage | 0.05 | 0.09 | 0.00 | 0.01 | 0.07 | -0.01 | -0.10 | -0.09 | -0.17 | -0.03 | 0.05 | -0.04 | 0.10 | -0.03 | 0.07 | 0.00 | 0.04 | -0.03 |
| | Testis | Early stage | 0.04 | 0.06 | -0.02 | -0.01 | 0.06 | 0.00 | -0.03 | -0.07 | -0.03 | -0.03 | 0.02 | 0.00 | 0.06 | 0.00 | -0.01 | 0.02 | 0.04 | -0.02 |
| | Testis | Middle stage | 0.00 | 0.01 | -0.03 | 0.00 | -0.02 | -0.01 | -0.04 | -0.05 | -0.04 | -0.01 | 0.00 | 0.00 | 0.00 | -0.01 | -0.01 | 0.00 | -0.01 | 0.03 |
| | Testis | Late stage | 0.03 | 0.06 | -0.02 | 0.01 | 0.05 | 0.02 | -0.04 | -0.04 | -0.05 | -0.06 | 0.00 | -0.02 | 0.04 | -0.04 | -0.02 | -0.03 | 0.06 | 0.02 |
| | Adipose | Early stage | 0.02 | -0.02 | 0.07 | 0.04 | 0.06 | 0.10 | 0.09 | 0.11 | 0.09 | 0.12 | 0.11 | 0.13 | 0.13 | 0.10 | 0.10 | 0.05 | 0.04 | 0.03 |
| | Adipose | Middle stage | 0.02 | 0.06 | 0.00 | 0.03 | 0.07 | 0.03 | 0.03 | 0.05 | 0.05 | -0.02 | 0.04 | 0.03 | 0.04 | -0.01 | 0.01 | 0.07 | 0.13 | 0.04 |
| | Adipose | Late stage | 0.02 | 0.06 | -0.01 | -0.01 | 0.05 | -0.01 | -0.11 | -0.16 | -0.21 | -0.10 | -0.03 | -0.10 | 0.07 | -0.06 | 0.06 | 0.01 | 0.08 | -0.01 |

Table 4-4

| | | | Muscle Early stage | Muscle Middle stage | Muscle Late stage | Skin Early stage | Skin Middle stage | Skin Late stage | Spleen Early stage | Spleen Middle stage | Spleen Late stage | Testis Early stage | Testis Middle stage | Testis Late stage | Adipose Early stage | Adipose Middle stage | Adipose Late stage |
|---|---|---|---|---|---|---|---|---|---|---|---|---|---|---|---|---|---|
| Model mice of myocardial infarction | Brain | Early stage | 0.06 | 0.10 | 0.14 | 0.05 | 0.09 | -0.01 | 0.06 | 0.04 | 0.02 | -0.04 | 0.05 | -0.04 | -0.02 | -0.07 | -0.01 |
| | Brain | Middle stage | -0.01 | 0.01 | 0.03 | -0.01 | 0.04 | -0.04 | -0.02 | 0.04 | 0.02 | 0.01 | -0.03 | 0.01 | -0.03 | -0.03 | -0.04 |
| | Brain | Late stage | -0.04 | -0.06 | -0.03 | -0.03 | -0.04 | -0.05 | -0.02 | -0.10 | -0.06 | -0.02 | 0.03 | -0.04 | -0.02 | -0.04 | -0.05 |
| | Heart | Early stage | -0.02 | 0.01 | 0.13 | -0.14 | -0.02 | -0.19 | -0.02 | -0.02 | 0.01 | -0.05 | 0.00 | -0.02 | -0.04 | -0.17 | -0.17 |
| | Heart | Middle stage | -0.09 | -0.08 | 0.06 | -0.25 | 0.02 | -0.27 | -0.08 | -0.01 | 0.00 | -0.08 | 0.01 | 0.00 | -0.09 | -0.24 | -0.25 |
| | Heart | Late stage | -0.14 | -0.15 | 0.02 | -0.20 | 0.05 | -0.24 | -0.06 | -0.02 | 0.04 | -0.07 | -0.02 | 0.00 | -0.10 | -0.26 | -0.27 |
| | Kidney | Early stage | 0.08 | 0.15 | 0.15 | 0.05 | 0.10 | 0.00 | 0.09 | 0.09 | 0.06 | -0.06 | 0.03 | -0.08 | 0.02 | -0.01 | 0.00 |
| | Kidney | Middle stage | -0.01 | 0.06 | 0.07 | -0.03 | 0.06 | -0.05 | -0.02 | 0.03 | 0.02 | -0.05 | -0.01 | 0.00 | -0.01 | -0.16 | -0.12 |
| | Kidney | Late stage | -0.02 | -0.04 | -0.02 | -0.05 | -0.06 | -0.07 | -0.01 | -0.08 | -0.03 | 0.00 | 0.03 | 0.03 | -0.02 | -0.02 | -0.05 |
| | Liver | Early stage | 0.08 | 0.10 | 0.10 | 0.08 | 0.05 | 0.02 | 0.06 | 0.09 | 0.05 | -0.01 | 0.01 | -0.02 | 0.03 | -0.02 | 0.03 |
| | Liver | Middle stage | -0.03 | -0.03 | 0.05 | -0.12 | 0.01 | -0.10 | -0.05 | -0.08 | -0.06 | -0.05 | 0.01 | -0.01 | -0.08 | -0.18 | -0.16 |
| | Liver | Late stage | -0.01 | -0.02 | -0.01 | -0.06 | -0.11 | -0.08 | -0.04 | -0.07 | -0.05 | 0.01 | 0.02 | -0.01 | 0.01 | -0.05 | -0.04 |
| | Lung | Early stage | 0.06 | 0.06 | 0.08 | -0.02 | -0.09 | -0.07 | 0.09 | -0.06 | -0.03 | -0.03 | 0.02 | -0.01 | 0.02 | -0.02 | 0.01 |
| | Lung | Middle stage | 0.05 | -0.06 | -0.03 | -0.13 | -0.13 | -0.06 | -0.02 | -0.21 | -0.20 | 0.01 | 0.05 | 0.04 | -0.05 | 0.03 | 0.04 |
| | Lung | Late stage | -0.04 | 0.00 | 0.05 | -0.08 | -0.07 | -0.14 | 0.00 | -0.09 | -0.04 | -0.09 | 0.08 | -0.05 | -0.04 | -0.19 | -0.15 |
| | Pancreas | Early stage | 0.07 | 0.01 | 0.02 | 0.04 | -0.06 | 0.02 | 0.04 | 0.02 | -0.01 | 0.04 | -0.04 | 0.02 | 0.03 | 0.07 | 0.08 |
| | Pancreas | Middle stage | 0.10 | 0.11 | 0.08 | 0.01 | 0.00 | -0.04 | 0.03 | 0.01 | -0.03 | -0.01 | 0.04 | -0.02 | 0.07 | 0.02 | 0.04 |
| | Pancreas | Late stage | 0.07 | 0.12 | 0.07 | 0.06 | -0.02 | 0.05 | 0.07 | 0.00 | 0.01 | 0.02 | -0.08 | -0.02 | 0.09 | 0.07 | 0.08 |
| | Muscle | Early stage | 0.22 | 0.27 | 0.29 | 0.10 | 0.08 | 0.00 | 0.09 | 0.09 | 0.03 | -0.07 | 0.00 | -0.06 | 0.02 | -0.04 | 0.03 |
| | Muscle | Middle stage | 0.17 | 0.09 | 0.12 | 0.04 | 0.10 | 0.02 | 0.02 | 0.11 | 0.03 | -0.03 | 0.00 | -0.03 | 0.01 | 0.04 | 0.05 |
| | Muscle | Late stage | -0.10 | -0.18 | -0.09 | -0.12 | -0.04 | -0.09 | -0.04 | 0.00 | -0.03 | -0.02 | 0.00 | 0.02 | -0.09 | -0.09 | -0.11 |
| | Skin | Early stage | 0.04 | 0.18 | 0.15 | 0.10 | 0.18 | 0.10 | 0.02 | 0.10 | 0.04 | -0.06 | 0.01 | -0.09 | -0.05 | -0.14 | -0.12 |
| | Skin | Middle stage | -0.06 | -0.09 | -0.06 | -0.15 | 0.04 | -0.04 | 0.01 | -0.12 | -0.09 | -0.08 | 0.19 | 0.00 | -0.11 | -0.07 | -0.07 |
| | Skin | Late stage | -0.05 | 0.02 | -0.01 | -0.03 | -0.01 | -0.07 | 0.00 | -0.07 | -0.03 | -0.04 | 0.08 | -0.03 | -0.01 | -0.07 | -0.02 |
| | Spleen | Early stage | 0.03 | 0.11 | 0.16 | 0.10 | 0.12 | 0.00 | 0.13 | 0.21 | 0.13 | -0.06 | -0.01 | -0.07 | 0.03 | -0.07 | -0.02 |
| | Spleen | Middle stage | 0.10 | 0.04 | 0.02 | -0.05 | -0.15 | -0.01 | 0.16 | -0.30 | -0.32 | 0.02 | 0.03 | 0.04 | 0.03 | 0.10 | 0.11 |
| | Spleen | Late stage | 0.05 | 0.01 | -0.02 | -0.04 | -0.19 | -0.01 | 0.26 | -0.24 | -0.21 | 0.02 | 0.04 | 0.01 | 0.03 | 0.10 | 0.09 |
| | Testis | Early stage | 0.06 | 0.06 | 0.00 | 0.04 | -0.07 | 0.02 | 0.04 | -0.02 | -0.04 | 0.10 | -0.07 | -0.01 | 0.08 | 0.11 | 0.06 |
| | Testis | Middle stage | 0.03 | 0.03 | 0.00 | -0.01 | -0.04 | 0.00 | -0.01 | -0.04 | -0.05 | 0.07 | -0.04 | 0.00 | 0.03 | 0.04 | 0.02 |
| | Testis | Late stage | 0.03 | 0.00 | -0.02 | 0.00 | -0.05 | 0.03 | 0.00 | 0.00 | -0.02 | 0.09 | -0.12 | -0.01 | 0.04 | 0.10 | 0.08 |
| | Adipose | Early stage | 0.10 | 0.09 | 0.09 | 0.04 | 0.08 | 0.02 | 0.05 | 0.05 | 0.03 | -0.02 | 0.04 | -0.03 | -0.13 | -0.11 | -0.04 |
| | Adipose | Middle stage | 0.07 | 0.07 | 0.06 | 0.00 | -0.07 | -0.04 | -0.03 | -0.05 | -0.05 | 0.03 | 0.05 | 0.02 | 0.44 | 0.08 | 0.12 |
| | Adipose | Late stage | 0.02 | -0.04 | -0.06 | 0.01 | -0.08 | 0.02 | 0.04 | -0.04 | 0.00 | 0.05 | -0.02 | 0.02 | 0.04 | 0.14 | 0.17 |

Table 4-5

| | | | Brain | Brain | Brain | Heart | Heart | Heart | Kidney | Kidney | Kidney | Liver | Liver | Liver | Lung | Lung | Lung | Pancreas | Pancreas | Pancreas |
|---|---|---|---|---|---|---|---|---|---|---|---|---|---|---|---|---|---|---|---|---|
| | | | Early stage | Middle stage | Late stage | Early stage | Middle stage | Late stage | Early stage | Middle stage | Late stage | Early stage | Middle stage | Late stage | Early stage | Middle stage | Late stage | Early stage | Middle stage | Late stage |
| Model mice of glioma | Left brain | Early stage | 0.06 | 0.02 | -0.08 | 0.00 | 0.00 | 0.02 | 0.01 | 0.10 | 0.03 | 0.02 | 0.05 | 0.03 | 0.05 | -0.04 | 0.01 | 0.00 | -0.02 | -0.01 |
| | Left brain | Middle stage | -0.24 | -0.52 | 0.15 | -0.05 | -0.27 | -0.04 | 0.23 | -0.02 | 0.06 | 0.27 | -0.23 | 0.00 | -0.10 | 0.33 | -0.01 | 0.06 | -0.26 | -0.05 |
| | Right brain | Early stage | -0.12 | -0.26 | 0.04 | -0.01 | -0.14 | -0.02 | 0.12 | 0.05 | 0.05 | 0.12 | -0.09 | 0.04 | -0.07 | 0.12 | -0.03 | 0.02 | -0.12 | -0.01 |
| | Right brain | Middle stage | 0.04 | 0.06 | -0.07 | -0.02 | 0.02 | -0.02 | -0.05 | 0.05 | 0.08 | -0.10 | 0.06 | 0.03 | -0.01 | -0.13 | -0.05 | -0.04 | 0.05 | -0.02 |
| | Heart | Early stage | -0.11 | -0.17 | 0.05 | -0.05 | -0.21 | -0.09 | 0.11 | -0.03 | 0.00 | 0.14 | -0.11 | -0.03 | -0.02 | 0.14 | 0.02 | 0.03 | -0.15 | -0.03 |
| | Heart | Middle stage | -0.10 | -0.28 | 0.04 | -0.04 | -0.30 | -0.06 | 0.15 | -0.01 | 0.07 | 0.14 | -0.16 | -0.01 | -0.09 | 0.14 | -0.03 | 0.03 | -0.18 | -0.03 |
| | Kidney | Early stage | 0.14 | 0.29 | -0.10 | 0.00 | 0.14 | 0.00 | -0.23 | -0.04 | -0.09 | -0.28 | 0.12 | -0.05 | 0.08 | -0.29 | -0.02 | -0.07 | 0.17 | 0.02 |
| | Kidney | Middle stage | -0.07 | -0.08 | -0.01 | 0.00 | -0.08 | 0.00 | -0.03 | -0.16 | 0.01 | 0.01 | -0.13 | 0.00 | -0.04 | 0.01 | -0.04 | 0.04 | -0.05 | -0.01 |
| | Liver | Early stage | -0.10 | -0.24 | 0.10 | -0.01 | -0.17 | -0.02 | 0.17 | 0.02 | 0.05 | 0.23 | -0.13 | 0.02 | -0.04 | 0.20 | 0.02 | 0.03 | -0.16 | -0.01 |
| | Liver | Middle stage | 0.12 | 0.28 | -0.08 | 0.04 | 0.16 | 0.02 | -0.20 | -0.05 | -0.04 | -0.35 | 0.03 | -0.08 | 0.05 | -0.28 | -0.04 | -0.05 | 0.19 | 0.00 |
| | Lung | Early stage | -0.11 | -0.27 | 0.06 | -0.06 | -0.22 | -0.04 | 0.10 | 0.02 | 0.04 | 0.18 | -0.09 | 0.01 | -0.04 | 0.17 | 0.02 | 0.05 | -0.18 | -0.04 |
| | Lung | Middle stage | -0.03 | -0.01 | -0.03 | 0.00 | -0.02 | 0.00 | -0.02 | -0.09 | 0.03 | -0.05 | -0.09 | -0.03 | -0.12 | -0.14 | -0.08 | 0.01 | -0.02 | -0.01 |
| | Pancreas | Early stage | -0.06 | -0.17 | 0.02 | -0.02 | -0.12 | -0.02 | 0.08 | -0.04 | -0.01 | 0.15 | -0.06 | 0.02 | -0.02 | 0.13 | 0.01 | 0.09 | -0.14 | -0.06 |
| | Pancreas | Middle stage | 0.09 | 0.18 | -0.07 | -0.01 | 0.09 | 0.01 | -0.13 | 0.04 | 0.02 | -0.18 | 0.10 | 0.00 | 0.01 | -0.22 | -0.02 | -0.07 | 0.14 | 0.01 |
| | Muscle | Early stage | 0.07 | 0.18 | -0.07 | 0.01 | 0.08 | 0.01 | -0.10 | -0.03 | -0.01 | -0.17 | 0.05 | -0.03 | 0.07 | -0.22 | -0.03 | -0.04 | 0.10 | 0.03 |
| | Muscle | Middle stage | 0.00 | -0.04 | -0.03 | -0.04 | -0.15 | -0.10 | -0.04 | 0.09 | 0.02 | 0.00 | 0.00 | -0.02 | -0.06 | -0.04 | -0.02 | 0.00 | -0.05 | -0.04 |
| | Skin | Early stage | 0.09 | 0.21 | -0.05 | 0.03 | 0.10 | 0.02 | -0.13 | 0.02 | -0.01 | -0.15 | 0.12 | 0.00 | 0.04 | -0.20 | -0.04 | -0.05 | 0.11 | 0.03 |
| | Skin | Middle stage | -0.13 | -0.21 | 0.04 | -0.05 | -0.20 | -0.06 | 0.07 | 0.04 | 0.04 | 0.13 | -0.10 | 0.00 | -0.15 | 0.05 | -0.03 | 0.00 | -0.16 | -0.05 |
| | Spleen | Early stage | 0.02 | 0.05 | -0.01 | 0.01 | 0.00 | 0.05 | -0.02 | -0.02 | -0.01 | -0.05 | 0.00 | 0.00 | 0.03 | -0.04 | 0.02 | -0.02 | 0.01 | 0.01 |
| | Spleen | Middle stage | -0.01 | 0.00 | -0.01 | 0.01 | -0.02 | 0.01 | -0.03 | -0.06 | -0.03 | 0.03 | 0.01 | 0.01 | -0.01 | -0.03 | 0.03 | 0.04 | -0.02 | -0.02 |
| | Testis | Early stage | 0.12 | 0.34 | -0.13 | 0.04 | 0.23 | 0.02 | -0.20 | -0.07 | -0.06 | -0.23 | 0.12 | 0.00 | 0.08 | -0.26 | -0.04 | -0.03 | 0.20 | 0.05 |
| | Testis | Middle stage | -0.05 | -0.15 | 0.08 | -0.03 | -0.12 | -0.05 | 0.07 | 0.11 | 0.00 | 0.13 | 0.01 | 0.02 | -0.03 | 0.09 | 0.06 | 0.03 | -0.09 | -0.03 |
| | Adipose | Early stage | 0.02 | 0.10 | -0.01 | 0.03 | 0.07 | -0.02 | -0.01 | -0.07 | 0.02 | -0.03 | -0.01 | 0.01 | 0.03 | -0.02 | -0.06 | 0.01 | 0.07 | -0.02 |
| | Adipose | Middle stage | -0.05 | -0.01 | -0.03 | -0.01 | -0.06 | -0.04 | 0.05 | -0.03 | 0.06 | -0.04 | -0.11 | -0.02 | -0.03 | -0.01 | -0.07 | -0.02 | 0.00 | -0.02 |

Table 4-6

| | | | Muscle | Muscle | Muscle | Skin | Skin | Skin | Spleen | Spleen | Spleen | Testis | Testis | Testis | Adipose | Adipose | Adipose |
|---|---|---|---|---|---|---|---|---|---|---|---|---|---|---|---|---|---|
| | | | Early stage | Middle stage | Late stage | Early stage | Middle stage | Late stage | Early stage | Middle stage | Late stage | Early stage | Middle stage | Late stage | Early stage | Middle stage | Late stage |
| Model mice of glioma | Left brain | Early stage | 0.01 | -0.05 | 0.02 | -0.02 | 0.04 | -0.02 | 0.03 | -0.02 | 0.01 | -0.01 | 0.13 | 0.00 | -0.04 | -0.04 | 0.00 |
| | Left brain | Middle stage | -0.08 | 0.14 | 0.20 | -0.10 | 0.11 | -0.20 | 0.01 | 0.06 | 0.00 | -0.14 | -0.05 | -0.19 | 0.00 | -0.36 | -0.29 |
| | Right brain | Early stage | -0.03 | 0.05 | 0.07 | -0.08 | 0.05 | -0.09 | 0.02 | 0.00 | -0.01 | -0.09 | -0.01 | -0.08 | -0.08 | -0.22 | -0.17 |
| | Right brain | Middle stage | -0.05 | -0.12 | -0.09 | -0.02 | -0.01 | 0.02 | -0.03 | -0.01 | 0.01 | 0.03 | -0.01 | 0.07 | -0.05 | 0.04 | 0.01 |
| | Heart | Early stage | -0.04 | 0.00 | 0.08 | -0.05 | 0.03 | -0.11 | 0.00 | 0.00 | -0.03 | -0.03 | 0.00 | -0.08 | 0.00 | -0.18 | -0.16 |
| | Heart | Middle stage | -0.16 | -0.06 | 0.07 | -0.11 | 0.06 | -0.16 | -0.02 | 0.01 | 0.01 | -0.11 | -0.01 | -0.11 | 0.02 | -0.25 | -0.21 |
| | Kidney | Early stage | 0.03 | -0.16 | -0.19 | 0.05 | -0.09 | 0.12 | -0.03 | -0.08 | -0.02 | 0.10 | 0.04 | 0.14 | -0.02 | 0.25 | 0.19 |
| | Kidney | Middle stage | -0.08 | -0.02 | 0.01 | -0.06 | -0.06 | -0.06 | -0.03 | 0.00 | -0.01 | 0.00 | -0.09 | -0.03 | 0.02 | -0.11 | -0.08 |
| | Liver | Early stage | -0.02 | 0.11 | 0.14 | -0.05 | 0.03 | -0.16 | 0.00 | 0.04 | -0.02 | -0.10 | 0.04 | -0.10 | -0.02 | -0.27 | -0.19 |
| | Liver | Middle stage | 0.03 | -0.15 | -0.19 | 0.05 | -0.08 | 0.13 | -0.01 | -0.05 | 0.02 | 0.11 | -0.06 | 0.12 | -0.01 | 0.25 | 0.17 |
| | Lung | Early stage | -0.06 | 0.04 | 0.11 | -0.12 | 0.05 | -0.20 | 0.00 | -0.08 | -0.05 | -0.11 | 0.06 | -0.12 | -0.07 | -0.30 | -0.22 |
| | Lung | Middle stage | -0.04 | -0.06 | -0.05 | -0.05 | -0.06 | -0.03 | -0.04 | -0.02 | 0.03 | 0.04 | -0.10 | 0.00 | 0.00 | -0.04 | -0.05 |
| | Pancreas | Early stage | -0.04 | 0.06 | 0.09 | -0.06 | 0.02 | -0.09 | -0.01 | -0.02 | -0.06 | -0.09 | 0.03 | -0.07 | 0.01 | -0.16 | -0.11 |
| | Pancreas | Middle stage | -0.03 | -0.15 | -0.15 | 0.02 | -0.06 | 0.06 | -0.05 | -0.09 | 0.01 | 0.10 | 0.01 | 0.09 | 0.01 | 0.15 | 0.11 |
| | Muscle | Early stage | 0.00 | -0.15 | -0.13 | 0.06 | -0.05 | 0.13 | 0.02 | 0.00 | 0.05 | 0.08 | -0.03 | 0.11 | 0.01 | 0.16 | 0.14 |
| | Muscle | Middle stage | -0.16 | -0.17 | -0.12 | -0.12 | -0.03 | -0.09 | -0.06 | -0.07 | -0.04 | -0.07 | 0.11 | -0.02 | -0.02 | -0.09 | -0.09 |
| | Skin | Early stage | 0.01 | -0.11 | -0.12 | 0.02 | -0.04 | 0.09 | 0.01 | -0.03 | 0.02 | 0.09 | 0.02 | 0.09 | 0.02 | 0.18 | 0.14 |
| | Skin | Middle stage | -0.09 | -0.04 | 0.02 | -0.18 | 0.04 | -0.15 | -0.07 | -0.05 | -0.06 | -0.10 | 0.07 | -0.09 | -0.13 | -0.25 | -0.23 |
| | Spleen | Early stage | 0.03 | 0.00 | 0.01 | 0.00 | 0.00 | 0.01 | -0.08 | -0.03 | 0.06 | -0.01 | 0.01 | -0.01 | 0.04 | 0.03 | 0.06 |
| | Spleen | Middle stage | 0.03 | 0.00 | 0.02 | -0.08 | -0.11 | -0.08 | 0.02 | -0.19 | -0.14 | 0.01 | 0.02 | -0.02 | 0.05 | -0.01 | 0.02 |
| | Testis | Early stage | 0.08 | -0.08 | -0.18 | 0.09 | -0.13 | 0.16 | -0.01 | -0.03 | 0.00 | 0.22 | -0.10 | 0.21 | 0.08 | 0.34 | 0.24 |
| | Testis | Middle stage | -0.04 | 0.00 | 0.05 | -0.10 | 0.06 | -0.13 | -0.01 | -0.04 | -0.06 | -0.19 | 0.22 | -0.15 | -0.09 | -0.18 | -0.12 |
| | Adipose | Early stage | 0.03 | 0.04 | 0.00 | 0.02 | -0.13 | -0.05 | 0.01 | 0.00 | 0.02 | 0.11 | -0.09 | 0.06 | 0.44 | 0.26 | 0.09 |
| | Adipose | Middle stage | -0.08 | -0.05 | -0.02 | -0.07 | -0.09 | -0.09 | -0.06 | 0.00 | 0.01 | 0.03 | -0.08 | 0.01 | 0.23 | 0.05 | -0.09 |

Table 4-7

| | | | Brain Early stage | Brain Middle stage | Brain Late stage | Heart Early stage | Heart Middle stage | Heart Late stage | Kidney Early stage | Kidney Middle stage | Kidney Late stage | Liver Early stage | Liver Middle stage | Liver Late stage | Lung Early stage | Lung Middle stage | Lung Late stage | Pancreas Early stage | Pancreas Middle stage | Pancreas Late stage |
|---|---|---|---|---|---|---|---|---|---|---|---|---|---|---|---|---|---|---|---|---|
| Model mice of early onset dementia | Brain | Early stage | 0.15 | 0.29 | -0.06 | 0.05 | 0.16 | 0.05 | -0.12 | -0.04 | -0.03 | -0.13 | 0.08 | 0.00 | 0.07 | -0.11 | 0.02 | 0.00 | 0.12 | 0.03 |
| | Brain | Middle stage | 0.02 | 0.03 | 0.02 | 0.00 | 0.02 | 0.01 | 0.02 | 0.03 | 0.02 | 0.01 | -0.01 | -0.02 | 0.00 | 0.00 | 0.00 | 0.01 | 0.02 | 0.01 |
| | Brain | Late stage | 0.11 | 0.20 | -0.03 | 0.03 | 0.12 | 0.06 | -0.04 | -0.01 | 0.03 | -0.11 | 0.05 | -0.02 | 0.06 | -0.09 | 0.03 | 0.01 | 0.10 | 0.02 |
| | Heart | Early stage | -0.01 | -0.09 | 0.05 | 0.02 | 0.01 | 0.05 | 0.05 | -0.04 | 0.00 | 0.03 | -0.07 | -0.01 | 0.07 | 0.14 | 0.06 | 0.04 | -0.05 | 0.01 |
| | Heart | Middle stage | -0.06 | -0.22 | 0.07 | 0.01 | -0.08 | 0.02 | 0.13 | 0.02 | 0.01 | 0.19 | -0.05 | 0.02 | 0.05 | 0.26 | 0.10 | 0.06 | -0.13 | -0.01 |
| | Heart | Late stage | -0.08 | -0.22 | 0.06 | 0.07 | -0.11 | 0.06 | 0.18 | 0.05 | 0.13 | 0.15 | -0.10 | 0.04 | 0.00 | 0.19 | 0.04 | 0.06 | -0.13 | 0.00 |
| | Kidney | Early stage | -0.03 | -0.13 | 0.05 | -0.01 | -0.06 | 0.00 | 0.09 | -0.03 | -0.03 | 0.13 | -0.07 | 0.02 | 0.02 | 0.19 | 0.07 | 0.06 | -0.08 | -0.02 |
| | Kidney | Middle stage | -0.02 | -0.15 | 0.07 | -0.03 | -0.09 | 0.00 | 0.08 | 0.05 | -0.02 | 0.12 | -0.04 | -0.02 | 0.00 | 0.16 | 0.07 | 0.01 | -0.11 | -0.01 |
| | Kidney | Late stage | -0.06 | -0.15 | 0.05 | -0.01 | -0.04 | 0.03 | 0.09 | -0.08 | -0.02 | 0.10 | -0.10 | 0.01 | -0.01 | 0.15 | 0.03 | 0.04 | -0.07 | 0.00 |
| | Liver | Early stage | 0.03 | 0.02 | -0.01 | 0.02 | 0.03 | 0.04 | 0.01 | 0.05 | 0.05 | -0.03 | -0.03 | -0.05 | 0.06 | 0.02 | 0.05 | 0.04 | 0.05 | 0.03 |
| | Liver | Middle stage | 0.03 | 0.13 | -0.01 | 0.06 | 0.14 | 0.06 | -0.06 | -0.05 | 0.02 | -0.08 | 0.05 | 0.02 | 0.07 | -0.05 | 0.05 | 0.02 | 0.09 | 0.03 |
| | Liver | Late stage | -0.02 | -0.06 | 0.00 | 0.00 | 0.00 | 0.06 | 0.09 | 0.04 | 0.10 | 0.07 | -0.07 | -0.01 | 0.03 | 0.09 | 0.01 | 0.05 | -0.01 | 0.03 |
| | Lung | Early stage | 0.05 | 0.22 | -0.06 | 0.08 | 0.19 | 0.06 | -0.11 | -0.08 | -0.04 | -0.14 | 0.08 | 0.03 | 0.10 | -0.11 | 0.07 | 0.01 | 0.14 | 0.03 |
| | Lung | Middle stage | 0.10 | 0.31 | -0.07 | 0.08 | 0.23 | 0.06 | -0.14 | -0.06 | -0.04 | -0.22 | 0.12 | 0.01 | 0.13 | -0.19 | 0.07 | -0.02 | 0.19 | 0.04 |
| | Lung | Late stage | 0.01 | -0.06 | 0.03 | 0.05 | 0.02 | 0.04 | 0.09 | -0.01 | -0.01 | 0.06 | -0.02 | 0.01 | 0.20 | 0.20 | 0.18 | 0.08 | -0.01 | 0.01 |
| | Pancreas | Early stage | -0.04 | -0.05 | 0.02 | 0.00 | 0.00 | 0.01 | 0.05 | -0.03 | 0.01 | 0.11 | 0.02 | 0.06 | 0.05 | 0.12 | 0.08 | 0.11 | 0.03 | 0.04 |
| | Pancreas | Middle stage | -0.01 | -0.02 | 0.03 | 0.04 | 0.01 | 0.05 | 0.06 | 0.04 | 0.07 | 0.10 | 0.02 | 0.06 | 0.04 | 0.08 | 0.06 | 0.09 | 0.03 | 0.05 |
| | Pancreas | Late stage | -0.02 | -0.09 | 0.07 | 0.02 | -0.01 | 0.04 | 0.09 | 0.04 | 0.06 | 0.15 | 0.03 | 0.09 | 0.01 | 0.14 | 0.06 | 0.08 | -0.03 | 0.05 |
| | Muscle | Early stage | 0.10 | 0.15 | -0.08 | 0.06 | 0.17 | 0.11 | -0.05 | 0.01 | 0.02 | -0.12 | 0.11 | 0.01 | 0.12 | -0.10 | 0.05 | -0.01 | 0.13 | 0.03 |
| | Muscle | Middle stage | -0.03 | -0.21 | 0.06 | 0.01 | -0.11 | 0.03 | 0.16 | 0.10 | 0.04 | 0.16 | -0.07 | -0.02 | 0.05 | 0.21 | 0.11 | 0.05 | -0.13 | -0.01 |
| | Muscle | Late stage | -0.02 | -0.09 | 0.03 | 0.07 | -0.01 | 0.11 | 0.12 | 0.04 | 0.09 | 0.08 | -0.02 | 0.05 | 0.04 | 0.11 | 0.06 | 0.04 | -0.05 | 0.01 |
| | Skin | Early stage | 0.07 | 0.20 | -0.08 | 0.05 | 0.21 | 0.09 | -0.07 | -0.05 | -0.03 | -0.14 | 0.04 | -0.02 | 0.09 | -0.10 | 0.00 | -0.02 | 0.11 | 0.04 |
| | Skin | Middle stage | 0.09 | 0.32 | -0.08 | 0.04 | 0.22 | 0.04 | -0.18 | -0.06 | -0.05 | -0.25 | 0.11 | 0.00 | 0.04 | -0.26 | 0.00 | -0.04 | 0.18 | 0.02 |
| | Skin | Late stage | -0.01 | -0.06 | 0.04 | 0.03 | 0.01 | 0.06 | 0.04 | 0.02 | -0.02 | 0.07 | -0.02 | 0.01 | -0.02 | 0.10 | 0.03 | 0.01 | -0.06 | 0.00 |
| | Spleen | Early stage | 0.10 | 0.13 | 0.03 | 0.07 | 0.10 | 0.04 | -0.04 | 0.04 | -0.05 | -0.06 | 0.11 | 0.01 | 0.10 | -0.02 | 0.09 | 0.03 | 0.09 | 0.02 |
| | Spleen | Middle stage | 0.09 | 0.26 | -0.07 | 0.07 | 0.16 | 0.04 | -0.09 | -0.02 | -0.03 | -0.17 | 0.10 | -0.02 | 0.08 | -0.13 | 0.03 | -0.02 | 0.15 | 0.04 |
| | Spleen | Late stage | 0.01 | -0.08 | 0.05 | 0.04 | -0.01 | 0.02 | 0.07 | -0.01 | -0.03 | 0.10 | -0.01 | 0.02 | 0.03 | 0.13 | 0.04 | 0.05 | -0.04 | 0.02 |
| | Testis | Early stage | -0.06 | -0.13 | 0.05 | -0.02 | -0.09 | -0.02 | 0.10 | 0.00 | 0.03 | 0.13 | -0.06 | -0.01 | -0.04 | 0.14 | 0.01 | 0.03 | -0.10 | -0.03 |
| | Testis | Middle stage | 0.02 | 0.14 | -0.05 | 0.05 | 0.12 | 0.02 | -0.08 | -0.10 | -0.03 | -0.12 | 0.03 | 0.02 | 0.03 | -0.09 | 0.00 | -0.01 | 0.09 | 0.02 |
| | Testis | Late stage | -0.03 | -0.07 | -0.01 | 0.00 | -0.04 | -0.03 | 0.06 | -0.06 | 0.04 | 0.06 | -0.06 | 0.02 | -0.04 | 0.09 | -0.03 | 0.03 | -0.06 | 0.01 |
| | Adipose | Early stage | 0.02 | -0.04 | -0.01 | 0.04 | 0.03 | 0.05 | 0.07 | 0.00 | 0.02 | 0.10 | 0.00 | 0.03 | 0.10 | 0.15 | 0.09 | 0.08 | 0.02 | 0.02 |
| | Adipose | Middle stage | 0.14 | 0.32 | -0.09 | 0.05 | 0.24 | 0.05 | -0.17 | -0.03 | -0.04 | -0.23 | 0.15 | 0.00 | 0.08 | -0.21 | 0.02 | -0.02 | 0.21 | 0.02 |
| | Adipose | Late stage | 0.09 | 0.11 | -0.04 | 0.08 | 0.13 | 0.07 | 0.00 | -0.01 | 0.01 | -0.03 | 0.06 | 0.02 | 0.11 | 0.03 | 0.06 | 0.04 | 0.08 | 0.04 |

1.00

0.50 or more, less than 1.00

Table 4-8

| | | | Muscle Early stage | Muscle Middle stage | Muscle Late stage | Skin Early stage | Skin Middle stage | Skin Late stage | Spleen Early stage | Spleen Middle stage | Spleen Late stage | Testis Early stage | Testis Middle stage | Testis Late stage | Adipose Early stage | Adipose Middle stage | Adipose Late stage |
|---|---|---|---|---|---|---|---|---|---|---|---|---|---|---|---|---|---|
| Model mice of early onset dementia | Brain | Early stage | 0.05 | -0.04 | -0.09 | 0.11 | -0.04 | 0.13 | 0.01 | -0.01 | 0.04 | 0.08 | -0.06 | 0.11 | 0.03 | 0.16 | 0.13 |
| | Brain | Middle stage | 0.01 | 0.01 | 0.03 | 0.01 | -0.01 | 0.00 | 0.01 | 0.00 | -0.01 | 0.00 | 0.02 | 0.01 | 0.02 | 0.02 | 0.00 |
| | Brain | Late stage | 0.05 | -0.04 | -0.04 | 0.08 | -0.02 | 0.12 | 0.00 | 0.03 | 0.02 | 0.05 | -0.04 | 0.12 | 0.00 | 0.10 | 0.08 |
| | Heart | Early stage | 0.09 | 0.10 | 0.12 | 0.06 | 0.02 | 0.01 | 0.03 | 0.05 | 0.02 | 0.00 | -0.11 | -0.03 | -0.01 | -0.05 | -0.05 |
| | Heart | Middle stage | 0.06 | 0.19 | 0.19 | 0.00 | 0.07 | -0.10 | 0.04 | 0.01 | -0.02 | -0.10 | 0.01 | -0.10 | 0.00 | -0.18 | -0.15 |
| | Heart | Late stage | -0.01 | 0.08 | 0.17 | -0.06 | 0.08 | -0.12 | 0.00 | 0.05 | 0.02 | -0.10 | -0.04 | -0.09 | -0.05 | -0.25 | -0.20 |
| | Kidney | Early stage | 0.04 | 0.12 | 0.12 | 0.04 | 0.08 | -0.06 | 0.00 | 0.07 | 0.01 | -0.07 | -0.02 | -0.08 | -0.04 | -0.16 | -0.13 |
| | Kidney | Middle stage | -0.03 | 0.07 | 0.13 | -0.01 | 0.08 | -0.07 | 0.00 | 0.03 | 0.00 | -0.11 | 0.07 | -0.07 | -0.05 | -0.17 | -0.16 |
| | Kidney | Late stage | 0.01 | 0.11 | 0.12 | 0.05 | 0.06 | -0.03 | 0.01 | 0.09 | 0.01 | -0.03 | -0.09 | -0.08 | -0.02 | -0.12 | -0.12 |
| | Liver | Early stage | 0.03 | 0.01 | 0.03 | 0.01 | 0.00 | -0.01 | -0.01 | 0.00 | -0.02 | -0.02 | -0.01 | -0.01 | -0.03 | -0.04 | -0.03 |
| | Liver | Middle stage | 0.08 | 0.04 | -0.03 | 0.06 | -0.05 | 0.08 | 0.00 | 0.01 | 0.03 | 0.10 | -0.11 | 0.07 | 0.04 | 0.13 | 0.14 |
| | Liver | Late stage | 0.03 | 0.06 | 0.10 | 0.00 | 0.03 | -0.03 | -0.02 | 0.03 | -0.02 | -0.03 | -0.03 | -0.02 | -0.02 | -0.12 | -0.11 |
| | Lung | Early stage | 0.11 | 0.01 | -0.08 | 0.09 | -0.07 | 0.13 | 0.04 | -0.02 | 0.00 | 0.15 | -0.10 | 0.10 | 0.10 | 0.27 | 0.21 |
| | Lung | Middle stage | 0.10 | -0.05 | -0.13 | 0.09 | -0.08 | 0.17 | 0.02 | -0.03 | 0.00 | 0.16 | -0.09 | 0.14 | 0.06 | 0.32 | 0.25 |
| | Lung | Late stage | 0.07 | 0.11 | 0.09 | 0.08 | 0.04 | 0.01 | 0.06 | 0.01 | 0.02 | -0.01 | -0.04 | -0.03 | 0.04 | -0.01 | 0.01 |
| | Pancreas | Early stage | 0.02 | 0.05 | 0.07 | 0.02 | 0.01 | -0.06 | 0.01 | 0.02 | 0.02 | -0.03 | 0.00 | -0.02 | 0.01 | -0.10 | -0.07 |
| | Pancreas | Middle stage | 0.01 | 0.04 | 0.04 | 0.00 | 0.00 | -0.06 | -0.01 | 0.02 | 0.03 | -0.04 | 0.01 | -0.01 | -0.04 | -0.10 | -0.09 |
| | Pancreas | Late stage | 0.03 | 0.11 | 0.10 | 0.01 | 0.05 | -0.04 | 0.02 | 0.04 | 0.02 | -0.06 | 0.02 | -0.04 | -0.01 | -0.14 | -0.10 |
| | Muscle | Early stage | 0.13 | -0.01 | 0.01 | 0.11 | 0.00 | 0.13 | 0.01 | -0.02 | 0.00 | 0.08 | -0.03 | 0.10 | -0.02 | 0.13 | 0.10 |
| | Muscle | Middle stage | 0.00 | 0.10 | 0.21 | 0.00 | 0.12 | -0.08 | 0.04 | 0.02 | 0.01 | -0.13 | 0.09 | -0.10 | -0.06 | -0.23 | -0.16 |
| | Muscle | Late stage | 0.05 | 0.05 | 0.17 | 0.01 | 0.12 | -0.04 | 0.02 | 0.06 | 0.04 | -0.04 | -0.02 | -0.03 | -0.05 | -0.12 | -0.09 |
| | Skin | Early stage | 0.07 | 0.01 | -0.03 | 0.24 | 0.03 | 0.26 | 0.01 | 0.11 | 0.08 | 0.15 | -0.12 | 0.11 | 0.04 | 0.26 | 0.18 |
| | Skin | Middle stage | 0.08 | -0.09 | -0.17 | 0.12 | -0.07 | 0.21 | -0.01 | -0.01 | 0.02 | 0.17 | -0.07 | 0.14 | 0.03 | 0.32 | 0.24 |
| | Skin | Late stage | 0.03 | 0.08 | 0.09 | 0.11 | 0.16 | 0.20 | 0.03 | 0.13 | 0.07 | -0.03 | -0.01 | -0.04 | -0.11 | -0.06 | -0.02 |
| | Spleen | Early stage | 0.10 | 0.04 | 0.03 | 0.10 | 0.06 | 0.11 | -0.04 | 0.09 | 0.13 | 0.01 | 0.07 | 0.02 | 0.00 | 0.14 | 0.16 |
| | Spleen | Middle stage | 0.07 | -0.06 | -0.08 | 0.12 | -0.03 | 0.18 | 0.00 | 0.10 | 0.18 | 0.10 | -0.08 | 0.08 | 0.04 | 0.27 | 0.22 |
| | Spleen | Late stage | 0.07 | 0.12 | 0.14 | 0.06 | 0.08 | 0.04 | -0.02 | 0.16 | 0.15 | -0.03 | 0.01 | -0.06 | 0.03 | -0.04 | 0.02 |
| | Testis | Early stage | -0.01 | 0.09 | 0.10 | -0.04 | 0.03 | -0.10 | 0.03 | -0.02 | -0.06 | -0.09 | 0.05 | -0.11 | -0.02 | -0.19 | -0.12 |
| | Testis | Middle stage | 0.07 | -0.01 | -0.06 | 0.08 | -0.05 | 0.09 | -0.02 | 0.02 | 0.03 | 0.13 | -0.11 | 0.09 | 0.05 | 0.16 | 0.15 |
| | Testis | Late stage | 0.02 | 0.07 | 0.06 | -0.01 | -0.03 | -0.05 | -0.02 | -0.01 | -0.04 | 0.00 | -0.06 | 0.03 | 0.02 | -0.09 | -0.06 |
| | Adipose | Early stage | 0.09 | 0.15 | 0.16 | 0.08 | -0.01 | -0.02 | 0.04 | 0.01 | 0.00 | 0.01 | -0.03 | 0.00 | 0.16 | 0.04 | 0.01 |
| | Adipose | Middle stage | 0.11 | -0.04 | -0.10 | 0.12 | -0.09 | 0.16 | 0.03 | -0.04 | -0.02 | 0.18 | -0.04 | 0.13 | 0.12 | 0.37 | 0.29 |
| | Adipose | Late stage | 0.13 | 0.13 | 0.11 | 0.14 | 0.00 | 0.09 | 0.05 | 0.02 | 0.01 | 0.03 | -0.03 | 0.06 | 0.17 | 0.25 | 0.25 |

| | |
|---|---|
| | 1.00 |
| | 0.50 or more, less than 1.00 |

Tables 4-1 to 4-8 show that, in the same organ, when diseases are different, the p-value is less than 0.55. In the case of the same organ and the same disease, when the stages are different, the p-value is less than 0.75. In other words, it was believed that when the p-value obtained between standard data 1 and the data of a subject is 0.55 or more, it can be determined that the data of the subject indicates the same disease as the disease corresponding to the standard data 1; when the p-value obtained between standard data 1 and the data of a subject is 0.75 or more, it can be determined that the data of the subject indicates the same stage as the stage corresponding to the standard data 1.

2. Application of Z-score method

The amount of expression of each gene in data of a subject was divided by the amount of expression of the corresponding gene in standard data, and the obtained value was scaled by log2. The scaled value was represented by $x_i$ (i=1, ..., the number of genes). Regarding the value $x_i$, the mean $\mu$ and variance $\sigma$ of all of the analyzed genes were determined.

Here, Z-score $z_i$ for a gene i is represented by the following equation.

Equation 3

$$z_i = \frac{x_i - \mu}{\sigma}$$

This is a quantification value indicating how far the scaled value $x_i$ of the gene i is from the mean of all of the analyzed genes. Here, this value indicates how much the gene i exhibits specific changes in expression compared to all of the analyzed genes. The closer this value is to 0, the less the gene exhibits specific changes in expression. The farther this value is from 0, the more the gene exhibits specific changes in expression. How much a gene exhibits specific changes in expression can be quantified by taking the median (Z') of the scaled value $z_i$.

For the analysis, a script for calculating Equation 1 was described using R. Tables 5-1 to 5-8 show the results.

Table 5-1

| | | | Model mice of chronic kidney disease | | | | | | | | | | | | | | | | | |
|---|---|---|---|---|---|---|---|---|---|---|---|---|---|---|---|---|---|---|---|---|
| | | | Brain Early stage | Brain Middle stage | Brain Late stage | Heart Early stage | Heart Middle stage | Heart Late stage | Kidney Early stage | Kidney Middle stage | Kidney Late stage | Liver Early stage | Liver Middle stage | Liver Late stage | Lung Early stage | Lung Middle stage | Lung Late stage | Pancreas Early stage | Pancreas Middle stage | Pancreas Late stage |
| Model mice of chronic kidney disease | Brain | Early stage | 0.00 | -0.92 | -0.17 | -0.64 | 1.10 | -0.13 | 0.74 | 1.32 | 6.51 | -0.85 | -1.36 | -1.83 | -2.98 | -5.19 | -0.93 | 3.35 | 10.44 | 1.80 |
| | Brain | Middle stage | 0.92 | 0.00 | 0.67 | 0.16 | 1.89 | 0.59 | 1.33 | 1.83 | 6.77 | -0.30 | -0.86 | -1.33 | -2.07 | -4.06 | -0.11 | 3.99 | 10.98 | 2.46 |
| | Brain | Late stage | 0.17 | -0.67 | 0.00 | -0.49 | 1.14 | 0.01 | 0.85 | 1.38 | 6.68 | -0.77 | -1.26 | -1.73 | -2.62 | -5.42 | -0.77 | 3.32 | 9.86 | 1.75 |
| | Heart | Early stage | 0.64 | -0.16 | 0.49 | 0.00 | 1.62 | 0.50 | 1.15 | 1.64 | 6.80 | -0.41 | -0.94 | -1.41 | -1.99 | -4.49 | -0.27 | 3.50 | 9.52 | 2.08 |
| | Heart | Middle stage | -1.10 | -1.89 | -1.14 | -1.62 | 0.00 | -1.19 | -0.07 | 0.52 | 5.58 | -1.47 | -1.96 | -2.35 | -3.81 | -5.46 | -1.80 | 2.09 | 8.55 | 0.59 |
| | Heart | Late stage | 0.13 | -0.59 | -0.01 | -0.50 | 1.19 | 0.00 | 0.79 | 1.33 | 6.55 | -0.72 | -1.21 | -1.70 | -2.43 | -4.74 | -0.71 | 3.04 | 9.06 | 1.70 |
| | Kidney | Early stage | -0.74 | -1.33 | -0.85 | -1.15 | 0.07 | -0.79 | 0.00 | 0.77 | 7.46 | -1.41 | -1.70 | -2.20 | -2.89 | -4.80 | -1.36 | 1.88 | 6.56 | 0.54 |
| | Kidney | Middle stage | -1.32 | -1.83 | -1.38 | -1.64 | -0.52 | -1.33 | -0.77 | 0.00 | 7.90 | -1.75 | -2.19 | -2.56 | -3.16 | -4.74 | -1.86 | 1.09 | 5.59 | -0.09 |
| | Kidney | Late stage | -6.51 | -6.77 | -6.68 | -6.80 | -5.58 | -6.55 | -7.46 | -7.90 | 0.00 | -6.34 | -6.44 | -6.84 | -7.70 | -9.12 | -6.71 | -4.31 | -0.87 | -5.33 |
| | Liver | Early stage | 0.85 | 0.30 | 0.77 | 0.41 | 1.47 | 0.72 | 1.41 | 1.75 | 6.34 | 0.00 | -0.68 | -1.30 | -1.06 | -3.10 | 0.23 | 3.15 | 7.28 | 1.97 |
| | Liver | Middle stage | 1.36 | 0.86 | 1.26 | 0.94 | 1.96 | 1.21 | 1.70 | 2.19 | 6.44 | 0.68 | 0.00 | -0.75 | -0.39 | -2.09 | 0.76 | 3.25 | 7.31 | 2.26 |
| | Liver | Late stage | 1.83 | 1.33 | 1.73 | 1.41 | 2.35 | 1.70 | 2.20 | 2.56 | 6.84 | 1.30 | 0.75 | 0.00 | 0.11 | -1.60 | 1.26 | 3.77 | 7.55 | 2.81 |
| | Lung | Early stage | 2.98 | 2.07 | 2.62 | 1.99 | 3.81 | 2.43 | 2.89 | 3.16 | 7.70 | 1.06 | 0.39 | -0.11 | 0.00 | -2.78 | 2.14 | 5.79 | 12.19 | 4.18 |
| | Lung | Middle stage | 5.19 | 4.06 | 5.42 | 4.49 | 5.46 | 4.74 | 4.80 | 4.74 | 9.12 | 3.10 | 2.09 | 1.60 | 2.78 | 0.00 | 4.78 | 7.46 | 12.80 | 6.19 |
| | Lung | Late stage | 0.93 | 0.11 | 0.77 | 0.27 | 1.80 | 0.71 | 1.36 | 1.86 | 6.71 | -0.23 | -0.76 | -1.26 | -2.14 | -4.78 | 0.00 | 3.83 | 9.95 | 2.43 |
| | Pancreas | Early stage | -3.35 | -3.99 | -3.32 | -3.50 | -2.09 | -3.04 | -1.88 | -1.09 | 4.31 | -3.15 | -3.25 | -3.77 | -5.79 | -7.46 | -3.83 | 0.00 | 6.80 | -1.79 |
| | Pancreas | Middle stage | -10.44 | -10.98 | -9.86 | -9.52 | -8.55 | -9.06 | -6.56 | -5.59 | 0.87 | -7.28 | -7.31 | -7.55 | -12.19 | -12.80 | -9.95 | -6.80 | 0.00 | -8.85 |
| | Pancreas | Late stage | -1.80 | -2.46 | -1.75 | -2.08 | -0.59 | -1.70 | -0.54 | 0.09 | 5.33 | -1.97 | -2.26 | -2.81 | -4.18 | -6.19 | -2.43 | 1.79 | 8.85 | 0.00 |
| | Muscle | Early stage | 3.44 | 2.55 | 3.30 | 2.80 | 4.30 | 3.06 | 3.25 | 3.50 | 7.87 | 1.54 | 0.87 | 0.35 | 0.70 | -1.80 | 2.37 | 5.90 | 11.82 | 4.44 |
| | Muscle | Middle stage | 4.19 | 3.33 | 3.88 | 3.32 | 5.11 | 3.84 | 4.04 | 4.06 | 8.11 | 2.35 | 1.49 | 1.04 | 1.70 | -0.72 | 3.20 | 6.66 | 11.90 | 5.53 |
| | Muscle | Late stage | 0.20 | -0.44 | 0.08 | -0.31 | 1.08 | 0.09 | 0.84 | 1.30 | 6.11 | -0.64 | -1.07 | -1.56 | -2.15 | -4.42 | -0.54 | 2.95 | 8.00 | 1.56 |
| | Skin | Early stage | 0.40 | -0.21 | 0.27 | -0.07 | 1.16 | 0.27 | 0.91 | 1.32 | 5.66 | -0.41 | -0.89 | -1.33 | -1.72 | -3.57 | -0.28 | 2.76 | 7.49 | 1.59 |
| | Skin | Middle stage | 0.16 | -0.47 | 0.04 | -0.31 | 0.95 | 0.05 | 0.77 | 1.28 | 5.89 | -0.63 | -1.06 | -1.54 | -2.02 | -3.88 | -0.54 | 2.71 | 7.53 | 1.49 |
| | Skin | Late stage | -4.21 | -4.63 | -4.20 | -4.41 | -3.47 | -4.13 | -3.00 | -2.43 | 2.25 | -3.90 | -4.22 | -4.51 | -5.87 | -7.19 | -4.59 | -1.82 | 2.17 | -3.00 |
| | Spleen | Early stage | -8.31 | -8.56 | -7.98 | -7.96 | -6.93 | -7.39 | -5.71 | -4.56 | 1.31 | -6.53 | -6.37 | -6.66 | -10.39 | -11.40 | -8.31 | -4.53 | 0.76 | -6.20 |
| | Spleen | Middle stage | 2.54 | 1.69 | 2.34 | 1.81 | 3.25 | 2.18 | 2.62 | 2.91 | 7.75 | 0.94 | 0.29 | -0.19 | -0.13 | -2.56 | 1.55 | 4.98 | 10.90 | 3.85 |
| | Spleen | Late stage | -5.32 | -5.74 | -5.27 | -5.47 | -4.13 | -5.05 | -3.68 | -2.86 | 2.61 | -4.56 | -4.63 | -5.16 | -7.25 | -8.89 | -5.93 | -2.32 | 2.63 | -3.84 |
| | Testis | Early stage | -2.52 | -3.27 | -2.54 | -2.81 | -1.21 | -2.27 | -1.06 | -0.36 | 5.08 | -2.43 | -2.71 | -3.17 | -4.83 | -6.92 | -3.01 | 1.01 | 7.44 | -0.67 |
| | Testis | Middle stage | -0.95 | -1.60 | -1.06 | -1.34 | 0.07 | -0.92 | -0.02 | 0.57 | 5.56 | -1.44 | -1.87 | -2.26 | -3.25 | -5.29 | -1.65 | 2.01 | 7.81 | 0.62 |
| | Testis | Late stage | -0.55 | -1.34 | -0.67 | -1.04 | 0.52 | -0.60 | 0.33 | 0.91 | 6.20 | -1.17 | -1.62 | -2.11 | -3.20 | -5.20 | -1.32 | 2.69 | 9.29 | 1.16 |
| | Adipose | Early stage | -13.87 | -14.04 | -13.85 | -13.95 | -13.18 | -13.69 | -12.58 | -11.59 | -7.26 | -13.00 | -12.83 | -13.26 | -14.77 | -16.00 | -13.98 | -12.07 | -9.40 | -13.06 |
| | Adipose | Middle stage | -1.87 | -2.47 | -1.87 | -2.24 | -1.13 | -1.80 | -0.97 | -0.47 | 3.96 | -2.00 | -2.49 | -2.82 | -3.67 | -4.93 | -2.40 | 0.48 | 4.97 | -0.66 |
| | Adipose | Late stage | -2.52 | -3.06 | -2.55 | -2.96 | -1.80 | -2.47 | -1.51 | -0.96 | 3.51 | -2.52 | -2.98 | -3.24 | -4.35 | -5.80 | -3.00 | -0.13 | 4.22 | -1.25 |

Table 5-2

| | | | Model mice of chronic kidney disease | | | | | | | | | | | | | | |
|---|---|---|---|---|---|---|---|---|---|---|---|---|---|---|---|---|---|
| | | | Muscle Early stage | Muscle Middle stage | Muscle Late stage | Skin Early stage | Skin Middle stage | Skin Late stage | Spleen Early stage | Spleen Middle stage | Spleen Late stage | Testis Early stage | Testis Middle stage | Testis Late stage | Adipose Early stage | Adipose Middle stage | Adipose Late stage |
| Model mice of chronic kidney disease | Brain | Early stage | -3.44 | -4.19 | -0.20 | -0.40 | -0.16 | 4.21 | 8.31 | -2.54 | 5.32 | 2.52 | 0.95 | 0.55 | 13.87 | 1.87 | 2.52 |
| | Brain | Middle stage | -2.55 | -3.33 | 0.44 | 0.21 | 0.47 | 4.63 | 8.56 | -1.69 | 5.74 | 3.27 | 1.60 | 1.34 | 14.04 | 2.47 | 3.06 |
| | Brain | Late stage | -3.30 | -3.88 | -0.08 | -0.27 | -0.04 | 4.20 | 7.98 | -2.34 | 5.27 | 2.54 | 1.06 | 0.67 | 13.85 | 1.87 | 2.55 |
| | Heart | Early stage | -2.80 | -3.32 | 0.31 | 0.07 | 0.31 | 4.41 | 7.96 | -1.81 | 5.47 | 2.81 | 1.34 | 1.04 | 13.95 | 2.24 | 2.96 |
| | Heart | Middle stage | -4.30 | -5.11 | -1.08 | -1.16 | -0.95 | 3.47 | 6.93 | -3.25 | 4.13 | 1.21 | -0.07 | -0.52 | 13.18 | 1.13 | 1.80 |
| | Heart | Late stage | -3.06 | -3.84 | -0.09 | -0.27 | -0.05 | 4.13 | 7.39 | -2.18 | 5.05 | 2.27 | 0.92 | 0.60 | 13.69 | 1.80 | 2.47 |
| | Kidney | Early stage | -3.25 | -4.04 | -0.84 | -0.91 | -0.77 | 3.00 | 5.71 | -2.62 | 3.68 | 1.06 | 0.02 | -0.33 | 12.58 | 0.97 | 1.51 |
| | Kidney | Middle stage | -3.50 | -4.06 | -1.30 | -1.32 | -1.28 | 2.43 | 4.56 | -2.91 | 2.86 | 0.36 | -0.57 | -0.91 | 11.59 | 0.47 | 0.96 |
| | Kidney | Late stage | -7.87 | -8.11 | -6.11 | -5.66 | -5.89 | -2.25 | -1.31 | -7.75 | -2.61 | -5.08 | -5.56 | -6.20 | 7.26 | -3.96 | -3.51 |
| | Liver | Early stage | -1.54 | -2.35 | 0.64 | 0.41 | 0.63 | 3.90 | 6.53 | -0.94 | 4.56 | 2.43 | 1.44 | 1.17 | 13.00 | 2.00 | 2.52 |
| | Liver | Middle stage | -0.87 | -1.49 | 1.07 | 0.89 | 1.06 | 4.22 | 6.37 | -0.29 | 4.63 | 2.71 | 1.87 | 1.62 | 12.83 | 2.49 | 2.98 |
| | Liver | Late stage | -0.35 | -1.04 | 1.56 | 1.33 | 1.54 | 4.51 | 6.66 | 0.19 | 5.16 | 3.17 | 2.26 | 2.11 | 13.26 | 2.82 | 3.24 |
| | Lung | Early stage | -0.70 | -1.70 | 2.15 | 1.72 | 2.02 | 5.87 | 10.39 | 0.13 | 7.25 | 4.83 | 3.25 | 3.20 | 14.77 | 3.67 | 4.35 |
| | Lung | Middle stage | 1.80 | 0.72 | 4.42 | 3.57 | 3.88 | 7.19 | 11.40 | 2.56 | 8.89 | 6.92 | 5.29 | 5.20 | 16.00 | 4.93 | 5.80 |
| | Lung | Late stage | -2.37 | -3.20 | 0.54 | 0.28 | 0.54 | 4.59 | 8.31 | -1.55 | 5.93 | 3.01 | 1.65 | 1.32 | 13.98 | 2.40 | 3.00 |
| | Pancreas | Early stage | -5.90 | -6.66 | -2.95 | -2.76 | -2.71 | 1.82 | 4.53 | -4.98 | 2.32 | -1.01 | -2.01 | -2.69 | 12.07 | -0.48 | 0.13 |
| | Pancreas | Middle stage | -11.82 | -11.90 | -8.00 | -7.49 | -7.53 | -2.17 | -0.76 | -10.90 | -2.63 | -7.44 | -7.81 | -9.29 | 9.40 | -4.97 | -4.22 |
| | Pancreas | Late stage | -4.44 | -5.53 | -1.56 | -1.59 | -1.49 | 3.00 | 6.20 | -3.85 | 3.84 | 0.67 | -0.62 | -1.16 | 13.06 | 0.66 | 1.25 |
| | Muscle | Early stage | 0.00 | -1.16 | 3.34 | 2.32 | 2.46 | 6.27 | 10.05 | 0.75 | 7.06 | 5.33 | 3.77 | 3.58 | 15.22 | 4.20 | 5.05 |
| | Muscle | Middle stage | 1.16 | 0.00 | 4.56 | 3.11 | 3.32 | 6.91 | 10.46 | 1.70 | 7.66 | 5.90 | 4.42 | 4.40 | 15.55 | 4.51 | 5.27 |
| | Muscle | Late stage | -3.34 | -4.56 | 0.00 | -0.19 | 0.03 | 4.02 | 6.96 | -1.88 | 4.56 | 2.13 | 0.92 | 0.61 | 13.58 | 1.78 | 2.47 |
| | Skin | Early stage | -2.32 | -3.11 | 0.19 | 0.00 | 0.22 | 5.31 | 6.41 | -1.59 | 4.60 | 2.13 | 1.01 | 0.75 | 13.22 | 1.97 | 2.54 |
| | Skin | Middle stage | -2.46 | -3.32 | -0.03 | -0.22 | 0.00 | 4.06 | 6.43 | -1.92 | 4.59 | 2.00 | 0.86 | 0.56 | 12.70 | 1.71 | 2.23 |
| | Skin | Late stage | -6.27 | -6.91 | -4.02 | -5.31 | -4.06 | 0.00 | 1.49 | -5.77 | 0.03 | -2.58 | -3.27 | -3.76 | 9.54 | -2.07 | -1.59 |
| | Spleen | Early stage | -10.05 | -10.46 | -6.96 | -6.41 | -6.43 | -1.49 | 0.00 | -8.73 | -1.61 | -5.85 | -6.39 | -7.32 | 9.50 | -3.91 | -3.39 |
| | Spleen | Middle stage | -0.75 | -1.70 | 1.88 | 1.59 | 1.92 | 5.77 | 8.73 | 0.00 | 8.77 | 4.46 | 2.93 | 2.86 | 14.42 | 3.36 | 3.97 |
| | Spleen | Late stage | -7.06 | -7.66 | -4.56 | -4.60 | -4.59 | -0.03 | 1.61 | -8.77 | 0.00 | -3.23 | -3.96 | -4.71 | 10.20 | -2.23 | -1.67 |
| | Testis | Early stage | -5.33 | -5.90 | -2.13 | -2.13 | -2.00 | 2.58 | 5.85 | -4.46 | 3.23 | 0.00 | -1.25 | -2.00 | 12.78 | 0.23 | 0.85 |
| | Testis | Middle stage | -3.77 | -4.42 | -0.92 | -1.01 | -0.86 | 3.27 | 6.39 | -2.93 | 3.96 | 1.25 | 0.00 | -0.44 | 13.01 | 1.06 | 1.65 |
| | Testis | Late stage | -3.58 | -4.40 | -0.61 | -0.75 | -0.56 | 3.76 | 7.32 | -2.86 | 4.71 | 2.00 | 0.44 | 0.00 | 13.36 | 1.45 | 2.03 |
| | Adipose | Early stage | -15.22 | -15.55 | -13.58 | -13.22 | -12.70 | -9.54 | -9.50 | -14.42 | -10.20 | -12.78 | -13.01 | -13.36 | 0.00 | -12.68 | -11.87 |
| | Adipose | Middle stage | -4.20 | -4.51 | -1.78 | -1.97 | -1.71 | 2.07 | 3.91 | -3.36 | 2.23 | -0.23 | -1.06 | -1.45 | 12.68 | 0.00 | 0.74 |
| | Adipose | Late stage | -5.05 | -5.27 | -2.47 | -2.54 | -2.23 | 1.59 | 3.39 | -3.97 | 1.67 | -0.85 | -1.65 | -2.03 | 11.87 | -0.74 | 0.00 |

Table 5-3

| | | | Brain | Brain | Brain | Heart | Heart | Heart | Kidney | Kidney | Kidney | Liver | Liver | Liver | Lung | Lung | Lung | Pancreas | Pancreas | Pancreas |
|---|---|---|---|---|---|---|---|---|---|---|---|---|---|---|---|---|---|---|---|---|
| | | | Early stage | Middle stage | Late stage | Early stage | Middle stage | Late stage | Early stage | Middle stage | Late stage | Early stage | Middle stage | Late stage | Early stage | Middle stage | Late stage | Early stage | Middle stage | Late stage |
| Model mice of myocardial infarction | Brain | Early stage | -2.34 | -2.91 | -2.41 | -2.60 | -1.04 | -2.14 | -0.95 | -0.27 | 5.19 | -2.36 | -2.60 | -3.06 | -4.68 | -6.93 | -2.90 | 1.13 | 7.24 | -0.49 |
| | Brain | Middle stage | -7.91 | -8.12 | -7.68 | -7.48 | -6.08 | -7.07 | -5.25 | -4.23 | 1.95 | -6.08 | -5.94 | -6.36 | -9.39 | -10.98 | -7.65 | -3.97 | 1.71 | -5.79 |
| | Brain | Late stage | -0.83 | -1.58 | -0.93 | -1.31 | 0.28 | -0.82 | 0.15 | 0.74 | 5.96 | -1.39 | -1.78 | -2.24 | -3.51 | -5.69 | -1.60 | 2.54 | 9.07 | 0.94 |
| | Heart | Early stage | -8.21 | -8.57 | -8.14 | -8.31 | -7.04 | -8.12 | -6.66 | -5.87 | -0.22 | -7.34 | -7.20 | -7.64 | -9.95 | -11.09 | -8.67 | -5.62 | -1.34 | -6.85 |
| | Heart | Middle stage | -8.85 | -9.12 | -8.76 | -8.80 | -7.54 | -8.52 | -7.44 | -6.98 | -1.44 | -7.96 | -7.76 | -8.26 | -9.87 | -11.14 | -8.72 | -6.43 | -2.58 | -7.57 |
| | Heart | Late stage | -9.80 | -10.27 | -9.91 | -10.00 | -8.45 | -9.66 | -8.33 | -7.61 | -1.99 | -8.76 | -8.45 | -9.00 | -11.04 | -12.09 | -9.84 | -7.32 | -3.30 | -8.64 |
| | Kidney | Early stage | -3.39 | -3.95 | -3.44 | -3.54 | -2.15 | -3.05 | -1.98 | -1.18 | 4.28 | -3.18 | -3.32 | -3.76 | -5.66 | -7.83 | -3.87 | -0.11 | 5.56 | -1.67 |
| | Kidney | Middle stage | -0.59 | -1.24 | -0.70 | -1.01 | 0.33 | -0.63 | 0.22 | 0.81 | 6.43 | -1.23 | -1.62 | -2.11 | -2.88 | -4.99 | -1.26 | 2.28 | 7.81 | 0.87 |
| | Kidney | Late stage | 0.63 | -0.14 | 0.47 | 0.02 | 1.47 | 0.45 | 1.11 | 1.59 | 6.40 | -0.39 | -0.90 | -1.36 | -1.94 | -4.08 | -0.23 | 3.52 | 9.55 | 2.12 |
| | Liver | Early stage | -0.81 | -1.47 | -0.90 | -1.23 | 0.13 | -0.82 | 0.04 | 0.59 | 5.58 | -1.52 | -1.91 | -2.37 | -3.08 | -5.15 | -1.46 | 2.04 | 7.32 | 0.66 |
| | Liver | Middle stage | -12.34 | -12.39 | -12.17 | -11.99 | -10.77 | -11.83 | -10.14 | -9.44 | -3.17 | -10.96 | -10.42 | -10.97 | -13.16 | -14.16 | -12.04 | -9.27 | -5.12 | -10.74 |
| | Liver | Late stage | 2.24 | 1.47 | 2.07 | 1.52 | 2.90 | 1.94 | 2.35 | 2.71 | 7.31 | 0.73 | 0.15 | -0.31 | -0.33 | -2.55 | 1.28 | 4.89 | 10.69 | 3.57 |
| | Lung | Early stage | 1.50 | 0.76 | 1.30 | 0.86 | 2.21 | 1.25 | 1.82 | 2.24 | 6.97 | 0.28 | -0.28 | -0.75 | -1.03 | -3.28 | 0.69 | 4.16 | 9.70 | 2.79 |
| | Lung | Middle stage | -16.95 | -17.11 | -16.31 | -16.20 | -15.33 | -15.78 | -14.03 | -13.40 | -7.49 | -14.22 | -14.07 | -14.13 | -16.94 | -17.03 | -16.10 | -13.92 | -10.56 | -15.20 |
| | Lung | Late stage | -0.04 | -0.73 | -0.17 | -0.56 | 0.85 | -0.15 | 0.66 | 1.20 | 6.29 | -0.85 | -1.29 | -1.75 | -2.52 | -4.64 | -0.82 | 2.91 | 8.66 | 1.45 |
| | Pancreas | Early stage | -4.08 | -4.62 | -3.96 | -4.11 | -2.51 | -3.46 | -2.00 | -1.18 | 4.44 | -3.32 | -3.50 | -3.93 | -6.50 | -8.30 | -4.35 | -0.08 | 6.81 | -1.97 |
| | Pancreas | Middle stage | -3.93 | -4.31 | -3.84 | -3.90 | -2.40 | -3.38 | -2.04 | -1.25 | 4.20 | -3.34 | -3.50 | -3.93 | -6.13 | -7.92 | -4.25 | -0.22 | 5.95 | -1.92 |
| | Pancreas | Late stage | -5.66 | -6.12 | -5.72 | -5.55 | -3.93 | -4.98 | -3.32 | -2.34 | 3.48 | -4.46 | -4.45 | -4.90 | -7.90 | -9.83 | -5.90 | -1.68 | 4.68 | -3.53 |
| | Muscle | Early stage | -3.93 | -4.29 | -3.95 | -4.11 | -2.84 | -3.75 | -2.47 | -1.69 | 3.62 | -3.72 | -3.95 | -4.38 | -6.07 | -8.10 | -4.42 | -0.86 | 4.39 | -2.31 |
| | Muscle | Middle stage | -2.13 | -2.72 | -2.18 | -2.43 | -1.02 | -1.98 | -0.96 | -0.31 | 4.82 | -2.27 | -2.50 | -2.92 | -4.34 | -6.18 | -2.61 | 0.95 | 6.48 | -0.53 |
| | Muscle | Late stage | -1.67 | -2.34 | -1.74 | -2.01 | -0.59 | -1.56 | -0.58 | 0.04 | 5.24 | -1.96 | -2.27 | -2.69 | -4.05 | -5.88 | -2.23 | 1.44 | 7.25 | -0.06 |
| | Skin | Early stage | 1.53 | 0.68 | 1.42 | 0.87 | 2.32 | 1.24 | 1.83 | 2.24 | 7.18 | 0.20 | -0.38 | -0.86 | -1.17 | -3.82 | 0.58 | 4.35 | 10.18 | 2.83 |
| | Skin | Middle stage | -0.54 | -1.32 | -0.64 | -1.03 | 0.51 | -0.55 | 0.32 | 0.91 | 6.03 | -1.20 | -1.69 | -2.11 | -3.12 | -5.13 | -1.30 | 2.62 | 9.01 | 1.14 |
| | Skin | Late stage | -0.90 | -1.58 | -0.93 | -1.30 | 0.25 | -0.86 | 0.13 | 0.73 | 5.86 | -1.40 | -1.76 | -2.27 | -3.45 | -5.62 | -1.69 | 2.53 | 8.98 | 0.96 |
| | Spleen | Early stage | -1.59 | -2.10 | -1.68 | -1.92 | -0.55 | -1.52 | -0.56 | 0.05 | 5.15 | -1.91 | -2.19 | -2.66 | -3.78 | -5.91 | -2.12 | 1.38 | 6.70 | -0.04 |
| | Spleen | Middle stage | -1.04 | -1.60 | -1.11 | -1.40 | -0.19 | -1.02 | -0.22 | 0.30 | 5.02 | -1.51 | -1.89 | -2.28 | -3.09 | -4.89 | -1.65 | 1.54 | 6.43 | 0.28 |
| | Spleen | Late stage | -6.76 | -7.11 | -6.65 | -6.79 | -5.90 | -6.43 | -5.41 | -4.72 | -0.06 | -6.28 | -6.32 | -6.52 | -8.22 | -9.30 | -7.01 | -4.48 | -0.97 | -5.56 |
| | Testis | Early stage | 4.23 | 3.30 | 3.95 | 3.16 | 4.68 | 3.50 | 3.79 | 4.05 | 8.50 | 2.00 | 1.22 | 0.71 | 1.26 | -1.29 | 3.00 | 6.76 | 12.89 | 5.45 |
| | Testis | Middle stage | 1.85 | 1.04 | 1.67 | 1.12 | 2.58 | 1.51 | 2.10 | 2.48 | 7.22 | 0.43 | -0.16 | -0.65 | -0.84 | -3.17 | 0.88 | 4.66 | 10.63 | 3.32 |
| | Testis | Late stage | 0.62 | -0.18 | 0.45 | -0.01 | 1.52 | 0.42 | 1.16 | 1.65 | 6.57 | -0.42 | -0.92 | -1.41 | -2.07 | -4.23 | -0.27 | 3.61 | 9.93 | 2.18 |
| | Adipose | Early stage | 1.10 | 0.43 | 0.97 | 0.55 | 1.83 | 0.93 | 1.54 | 1.98 | 6.68 | 0.06 | -0.46 | -0.92 | -1.17 | -3.27 | 0.35 | 3.59 | 8.67 | 2.33 |
| | Adipose | Middle stage | -11.96 | -12.18 | -11.88 | -11.87 | -11.23 | -11.66 | -10.57 | -9.95 | -5.43 | -11.05 | -10.90 | -11.20 | -12.94 | -13.87 | -11.99 | -10.09 | -7.26 | -10.98 |
| | Adipose | Late stage | 8.40 | 7.37 | 7.97 | 7.16 | 8.44 | 7.25 | 7.06 | 6.88 | 10.31 | 5.15 | 4.19 | 3.62 | 5.69 | 2.93 | 7.08 | 10.32 | 15.89 | 9.28 |

Table 5-4

| | | | Muscle Early stage | Muscle Middle stage | Muscle Late stage | Skin Early stage | Skin Middle stage | Skin Late stage | Spleen Early stage | Spleen Middle stage | Spleen Late stage | Testis Early stage | Testis Middle stage | Testis Late stage | Adipose Early stage | Adipose Middle stage | Adipose Late stage |
|---|---|---|---|---|---|---|---|---|---|---|---|---|---|---|---|---|---|
| Model mice of myocardial infarction | Brain | Early stage | -5.08 | -5.85 | -2.01 | -1.96 | -1.86 | 2.67 | 5.71 | -4.31 | 3.31 | 0.15 | -1.06 | -1.64 | 12.59 | 0.31 | 0.93 |
| | Brain | Middle stage | -9.44 | -9.86 | -6.35 | -5.84 | -6.13 | -0.94 | 0.79 | -8.82 | -1.12 | -5.27 | -5.91 | -6.88 | 9.93 | -3.40 | -2.71 |
| | Brain | Late stage | -3.90 | -4.72 | -0.82 | -0.94 | -0.76 | 3.60 | 7.20 | -3.11 | 4.54 | 1.65 | 0.20 | -0.26 | 13.42 | 1.28 | 1.89 |
| | Heart | Early stage | -9.68 | -10.08 | -7.58 | -6.72 | -7.08 | -2.65 | -1.82 | -9.16 | -3.28 | -6.38 | -6.87 | -7.73 | 7.60 | -4.68 | -4.11 |
| | Heart | Middle stage | -9.83 | -10.16 | -8.03 | -6.98 | -8.03 | -3.39 | -2.92 | -9.74 | -4.24 | -7.19 | -7.69 | -8.50 | 6.35 | -5.29 | -4.75 |
| | Heart | Late stage | -10.77 | -10.92 | -8.84 | -8.08 | -9.05 | -4.07 | -3.71 | -10.87 | -5.10 | -8.30 | -8.63 | -9.70 | 6.13 | -6.11 | -5.47 |
| | Kidney | Early stage | -5.94 | -6.66 | -2.97 | -2.85 | -2.78 | 1.76 | 4.42 | -5.21 | 2.15 | -1.09 | -2.15 | -2.73 | 11.96 | -0.55 | 0.05 |
| | Kidney | Middle stage | -3.30 | -4.21 | -0.66 | -0.77 | -0.60 | 3.38 | 6.39 | -2.63 | 4.15 | 1.45 | 0.26 | -0.12 | 12.90 | 1.19 | 1.76 |
| | Kidney | Late stage | -2.43 | -3.27 | 0.31 | 0.09 | 0.32 | 4.23 | 7.72 | -1.69 | 5.27 | 2.78 | 1.38 | 1.08 | 13.65 | 2.09 | 2.64 |
| | Liver | Early stage | -3.56 | -4.39 | -0.85 | -0.95 | -0.78 | 3.22 | 6.14 | -2.81 | 3.91 | 1.22 | 0.06 | -0.33 | 12.86 | 1.08 | 1.68 |
| | Liver | Middle stage | -13.33 | -13.51 | -10.96 | -9.84 | -10.68 | -5.58 | -5.30 | -12.66 | -6.48 | -10.42 | -10.76 | -11.76 | 5.45 | -7.56 | -6.97 |
| | Liver | Late stage | -0.94 | -1.83 | 1.64 | 1.29 | 1.55 | 5.15 | 8.71 | -0.19 | 6.38 | 4.23 | 2.76 | 2.59 | 14.36 | 3.16 | 3.65 |
| | Lung | Early stage | -1.49 | -2.34 | 1.06 | 0.75 | 1.01 | 4.60 | 8.11 | -0.74 | 5.56 | 3.33 | 2.04 | 1.84 | 13.79 | 2.62 | 3.18 |
| | Lung | Middle stage | -17.49 | -17.37 | -14.92 | -13.22 | -14.17 | -9.53 | -10.62 | -15.45 | -10.49 | -14.88 | -15.28 | -15.85 | 1.29 | -12.20 | -11.50 |
| | Lung | Late stage | -2.93 | -3.77 | -0.21 | -0.37 | -0.17 | 3.77 | 7.05 | -2.17 | 4.74 | 2.09 | 0.79 | 0.43 | 13.26 | 1.60 | 2.15 |
| | Pancreas | Early stage | -6.61 | -7.24 | -3.18 | -3.05 | -2.94 | 1.90 | 4.92 | -5.85 | 2.44 | -1.26 | -2.38 | -3.16 | 12.26 | -0.58 | 0.08 |
| | Pancreas | Middle stage | -6.41 | -7.07 | -3.15 | -3.00 | -2.93 | 1.72 | 4.39 | -5.60 | 2.20 | -1.31 | -2.43 | -3.06 | 12.13 | -0.65 | -0.02 |
| | Pancreas | Late stage | -7.94 | -8.57 | -4.58 | -4.26 | -4.23 | 0.80 | 3.28 | -7.05 | 1.06 | -2.93 | -3.73 | -4.82 | 11.52 | -1.70 | -1.04 |
| | Muscle | Early stage | -6.94 | -7.55 | -3.83 | -3.40 | -3.34 | 1.14 | 3.40 | -5.50 | 1.39 | -1.76 | -2.67 | -3.27 | 11.26 | -1.10 | -0.52 |
| | Muscle | Middle stage | -5.04 | -5.65 | -1.99 | -1.93 | -1.83 | 2.46 | 5.32 | -4.13 | 2.98 | 0.05 | -1.05 | -1.56 | 12.34 | 0.25 | 0.84 |
| | Muscle | Late stage | -4.26 | -4.85 | -1.47 | -1.53 | -1.44 | 2.75 | 5.77 | -3.70 | 3.49 | 0.55 | -0.65 | -1.16 | 12.47 | 0.58 | 1.14 |
| | Skin | Early stage | -1.84 | -2.62 | 1.05 | 0.73 | 1.05 | 5.14 | 8.51 | -1.02 | 6.03 | 3.59 | 2.10 | 1.88 | 13.93 | 2.63 | 3.29 |
| | Skin | Middle stage | -3.52 | -4.28 | -0.58 | -0.69 | -0.55 | 3.67 | 7.29 | -2.71 | 4.46 | 1.81 | 0.45 | 0.01 | 13.30 | 1.43 | 2.01 |
| | Skin | Late stage | -3.75 | -4.87 | -0.83 | -0.96 | -0.80 | 3.56 | 7.07 | -3.03 | 4.64 | 1.59 | 0.18 | -0.29 | 13.36 | 1.26 | 1.82 |
| | Spleen | Early stage | -4.22 | -4.98 | -1.50 | -1.55 | -1.39 | 2.81 | 5.82 | -3.79 | 3.51 | 0.52 | -0.59 | -1.03 | 12.53 | 0.57 | 1.17 |
| | Spleen | Middle stage | -3.52 | -4.19 | -1.04 | -1.09 | -0.95 | 2.79 | 5.56 | -2.59 | 2.89 | 0.79 | -0.24 | -0.61 | 12.30 | 0.80 | 1.34 |
| | Spleen | Late stage | -8.25 | -8.82 | -6.22 | -5.81 | -5.82 | -2.37 | -1.61 | -7.14 | -2.23 | -5.23 | -5.80 | -6.32 | 7.29 | -4.35 | -3.82 |
| | Testis | Early stage | 0.53 | -0.52 | 3.16 | 2.64 | 3.01 | 6.46 | 10.85 | 1.31 | 7.82 | 6.43 | 4.44 | 4.53 | 15.42 | 4.52 | 4.97 |
| | Testis | Middle stage | -1.45 | -2.36 | 1.29 | 0.97 | 1.27 | 5.05 | 8.83 | -0.66 | 6.15 | 4.11 | 2.48 | 2.27 | 14.26 | 2.97 | 3.50 |
| | Testis | Late stage | -2.59 | -3.41 | 0.29 | 0.07 | 0.31 | 4.36 | 7.88 | -1.81 | 5.39 | 2.91 | 1.39 | 1.10 | 13.87 | 2.18 | 2.74 |
| | Adipose | Early stage | -1.71 | -2.52 | 0.78 | 0.53 | 0.78 | 4.44 | 7.28 | -0.99 | 5.29 | 2.84 | 1.71 | 1.43 | 13.00 | 2.22 | 2.85 |
| | Adipose | Middle stage | -13.11 | -13.37 | -11.35 | -10.97 | -11.01 | -7.55 | -7.34 | -12.52 | -8.17 | -10.71 | -11.16 | -11.48 | 3.20 | -9.39 | -9.10 |
| | Adipose | Late stage | 4.63 | 3.48 | 6.71 | 6.03 | 6.33 | 9.26 | 13.83 | 5.28 | 10.96 | 10.02 | 8.20 | 8.46 | 17.19 | 7.85 | 8.45 |

Table 5-5

| | | | Brain Early stage | Brain Middle stage | Brain Late stage | Heart Early stage | Heart Middle stage | Heart Late stage | Kidney Early stage | Kidney Middle stage | Kidney Late stage | Liver Early stage | Liver Middle stage | Liver Late stage | Lung Early stage | Lung Middle stage | Lung Late stage | Pancreas Early stage | Pancreas Middle stage | Pancreas Late stage |
|---|---|---|---|---|---|---|---|---|---|---|---|---|---|---|---|---|---|---|---|---|
| Model mice of glioma | Left brain | Early stage | -1.00 | -1.78 | -1.07 | -1.43 | 0.19 | -0.94 | 0.07 | 0.69 | 6.05 | -1.45 | -1.87 | -2.32 | -3.74 | -5.89 | -1.72 | 2.40 | 8.97 | 0.84 |
| | Left brain | Middle stage | -3.10 | -3.31 | -3.60 | -3.55 | -2.22 | -3.13 | -2.40 | -1.54 | 3.38 | -3.69 | -3.32 | -3.88 | -4.91 | -7.86 | -3.69 | -0.81 | 3.43 | -1.97 |
| | Right brain | Early stage | -4.28 | -4.59 | -4.47 | -4.46 | -2.69 | -3.85 | -2.46 | -1.57 | 4.26 | -3.73 | -3.68 | -4.19 | -6.39 | -9.09 | -4.68 | -0.57 | 5.61 | -2.36 |
| | Right brain | Middle stage | -8.37 | -8.38 | -7.61 | -7.52 | -6.12 | -7.10 | -4.91 | -4.05 | 2.33 | -5.85 | -5.90 | -6.25 | -9.73 | -11.10 | -7.82 | -3.67 | 2.36 | -5.73 |
| | Heart | Early stage | -1.09 | -1.76 | -1.26 | -1.53 | 0.04 | -1.06 | -0.04 | 0.55 | 5.84 | -1.61 | -1.90 | -2.37 | -3.72 | -6.20 | -1.85 | 2.28 | 8.60 | 0.69 |
| | Heart | Middle stage | 0.66 | -0.18 | 0.48 | -0.01 | 1.45 | 0.43 | 1.20 | 1.65 | 6.77 | -0.45 | -0.94 | -1.43 | -2.12 | -4.63 | -0.28 | 3.74 | 9.81 | 2.28 |
| | Kidney | Early stage | 0.64 | -0.25 | 0.42 | -0.05 | 1.60 | 0.40 | 1.10 | 1.66 | 6.55 | -0.43 | -1.00 | -1.46 | -2.23 | -4.13 | -0.32 | 3.68 | 10.75 | 2.26 |
| | Kidney | Middle stage | -0.58 | -1.37 | -0.79 | -1.19 | 0.49 | -0.63 | 0.33 | 0.87 | 6.25 | -1.23 | -1.64 | -2.12 | -3.28 | -5.83 | -1.37 | 2.85 | 9.40 | 1.15 |
| | Liver | Early stage | -3.87 | -4.29 | -4.08 | -4.04 | -2.35 | -3.51 | -2.20 | -1.29 | 4.30 | -3.70 | -3.41 | -3.97 | -6.05 | -8.77 | -4.34 | -0.27 | 5.75 | -2.04 |
| | Liver | Middle stage | -0.16 | -0.92 | -0.28 | -0.70 | 0.77 | -0.24 | 0.53 | 1.07 | 6.18 | -0.80 | -1.33 | -1.74 | -2.52 | -4.40 | -0.87 | 2.67 | 8.86 | 1.29 |
| | Lung | Early stage | -0.53 | -1.20 | -0.68 | -1.03 | 0.49 | -0.57 | 0.35 | 0.89 | 6.19 | -1.25 | -1.58 | -2.06 | -3.18 | -5.66 | -1.35 | 2.71 | 8.69 | 1.14 |
| | Lung | Middle stage | -4.76 | -5.30 | -4.92 | -5.03 | -3.00 | -4.08 | -2.55 | -1.64 | 4.21 | -3.74 | -3.87 | -4.33 | -6.81 | -8.84 | -4.96 | -0.75 | 5.74 | -2.49 |
| | Pancreas | Early stage | -1.53 | -2.19 | -1.68 | -1.94 | -0.37 | -1.45 | -0.39 | 0.24 | 5.45 | -1.90 | -2.14 | -2.66 | -4.02 | -6.49 | -2.24 | 1.86 | 7.79 | 0.22 |
| | Pancreas | Middle stage | -4.47 | -5.10 | -4.43 | -4.73 | -3.06 | -3.94 | -2.51 | -1.84 | 3.81 | -3.54 | -4.06 | -4.28 | -6.27 | -7.84 | -4.65 | -0.95 | 4.86 | -2.46 |
| | Muscle | Early stage | -2.97 | -3.63 | -3.01 | -3.28 | -1.65 | -2.73 | -1.41 | -0.71 | 4.74 | -2.65 | -2.98 | -3.39 | -5.33 | -6.96 | -3.40 | 0.48 | 6.72 | -1.17 |
| | Muscle | Middle stage | -0.42 | -1.13 | -0.50 | -0.86 | 0.56 | -0.46 | 0.41 | 0.98 | 5.99 | -1.09 | -1.49 | -1.97 | -2.98 | -4.94 | -1.15 | 2.74 | 8.86 | 1.27 |
| | Skin | Early stage | -1.02 | -1.80 | -1.11 | -1.58 | 0.13 | -0.95 | 0.02 | 0.62 | 5.90 | -1.42 | -1.96 | -2.33 | -3.67 | -5.71 | -1.68 | 2.29 | 8.83 | 0.70 |
| | Skin | Middle stage | -4.24 | -4.59 | -4.37 | -4.23 | -2.89 | -3.77 | -2.60 | -1.86 | 3.71 | -3.90 | -3.98 | -4.40 | -6.01 | -7.94 | -4.53 | -1.00 | 4.43 | -2.50 |
| | Spleen | Early stage | -4.46 | -4.92 | -4.48 | -4.50 | -3.26 | -4.14 | -2.90 | -2.13 | 3.21 | -3.90 | -4.07 | -4.49 | -6.36 | -7.89 | -4.78 | -1.42 | 3.71 | -2.91 |
| | Spleen | Middle stage | 2.21 | 1.46 | 2.11 | 1.61 | 2.80 | 1.91 | 2.37 | 2.74 | 7.35 | 0.82 | 0.21 | -0.26 | -0.23 | -2.52 | 1.34 | 4.73 | 9.90 | 3.31 |
| | Testis | Early stage | -1.53 | -2.27 | -1.45 | -1.76 | -0.60 | -1.43 | -0.53 | 0.01 | 4.83 | -1.70 | -2.18 | -2.59 | -3.62 | -4.89 | -2.01 | 1.22 | 6.69 | -0.09 |
| | Testis | Middle stage | -0.12 | -0.86 | -0.27 | -0.67 | 0.86 | -0.22 | 0.63 | 1.18 | 6.24 | -0.94 | -1.37 | -1.83 | -2.76 | -5.11 | -0.95 | 2.96 | 9.29 | 1.51 |
| | Adipose | Early stage | -11.86 | -12.17 | -11.85 | -11.77 | -10.91 | -11.46 | -10.12 | -9.20 | -4.16 | -10.52 | -10.18 | -10.59 | -12.96 | -14.00 | -11.86 | -9.54 | -6.18 | -10.66 |
| | Adipose | Middle stage | -13.65 | -13.59 | -13.35 | -13.19 | -11.89 | -12.72 | -11.11 | -9.99 | -4.14 | -11.14 | -10.46 | -10.75 | -14.67 | -15.81 | -13.19 | -10.48 | -6.52 | -11.92 |

Table 5-6

| | | | Muscle Early stage | Muscle Middle stage | Muscle Late stage | Skin Early stage | Skin Middle stage | Skin Late stage | Spleen Early stage | Spleen Middle stage | Spleen Late stage | Testis Early stage | Testis Middle stage | Testis Late stage | Adipose Early stage | Adipose Middle stage | Adipose Late stage |
|---|---|---|---|---|---|---|---|---|---|---|---|---|---|---|---|---|---|
| Model mice of glioma | Left brain | Early stage | -4.00 | -4.80 | -0.90 | -1.01 | -0.83 | 3.57 | 7.17 | -3.26 | 4.54 | 1.55 | 0.12 | -0.37 | 13.38 | 1.19 | 1.83 |
| | Left brain | Middle stage | -5.47 | -6.20 | -3.31 | -2.88 | -2.98 | 0.92 | 2.86 | -4.97 | 1.16 | -1.58 | -2.35 | -2.77 | 10.69 | -0.95 | -0.49 |
| | Right brain | Early stage | -6.67 | -7.39 | -3.51 | -3.25 | -3.33 | 1.51 | 4.27 | -6.09 | 1.93 | -1.76 | -2.76 | -3.44 | 11.82 | -0.86 | -0.26 |
| | Right brain | Middle stage | -9.54 | -9.64 | -6.09 | -5.71 | -6.13 | -0.65 | 1.27 | -9.19 | -0.80 | -5.28 | -6.03 | -7.17 | 10.27 | -3.18 | -2.50 |
| | Heart | Early stage | -4.11 | -4.92 | -1.06 | -1.11 | -0.95 | 3.38 | 7.13 | -3.38 | 4.33 | 1.40 | -0.03 | -0.51 | 13.34 | 1.07 | 1.69 |
| | Heart | Middle stage | -2.54 | -3.44 | 0.29 | 0.06 | 0.31 | 4.38 | 8.20 | -1.88 | 5.62 | 2.90 | 1.43 | 1.08 | 13.94 | 2.06 | 2.68 |
| | Kidney | Early stage | -2.73 | -3.39 | 0.26 | 0.04 | 0.29 | 4.50 | 8.42 | -1.84 | 5.52 | 3.09 | 1.42 | 1.16 | 13.83 | 2.32 | 2.87 |
| | Kidney | Middle stage | -3.88 | -4.37 | -0.66 | -0.76 | -0.55 | 3.72 | 7.52 | -2.95 | 4.73 | 2.00 | 0.41 | -0.02 | 13.64 | 1.43 | 2.09 |
| | Liver | Early stage | -6.47 | -7.39 | -3.38 | -3.05 | -3.06 | 1.66 | 4.52 | -5.85 | 2.15 | -1.40 | -2.44 | -3.11 | 12.02 | -0.65 | -0.05 |
| | Liver | Middle stage | -3.12 | -3.49 | -0.28 | -0.46 | -0.25 | 3.84 | 6.87 | -2.23 | 4.74 | 2.00 | 0.66 | 0.32 | 13.27 | 1.71 | 2.30 |
| | Lung | Early stage | -3.57 | -4.49 | -0.61 | -0.71 | -0.55 | 3.51 | 7.37 | -2.76 | 4.71 | 1.82 | 0.44 | 0.01 | 13.20 | 1.33 | 1.95 |
| | Lung | Middle stage | -7.24 | -7.19 | -3.65 | -3.42 | -3.40 | 1.40 | 4.22 | -6.36 | 1.88 | -1.99 | -2.92 | -3.92 | 11.92 | -1.02 | -0.38 |
| | Pancreas | Early stage | -4.47 | -5.28 | -1.44 | -1.43 | -1.26 | 3.07 | 6.38 | -3.65 | 3.86 | 0.87 | -0.44 | -0.94 | 13.19 | 0.77 | 1.39 |
| | Pancreas | Middle stage | -6.73 | -6.54 | -3.47 | -3.48 | -3.30 | 1.13 | 3.46 | -5.77 | 1.40 | -2.09 | -2.98 | -3.76 | 11.44 | -1.28 | -0.63 |
| | Muscle | Early stage | -5.44 | -5.85 | -2.41 | -2.41 | -2.31 | 2.26 | 5.21 | -4.90 | 2.83 | -0.55 | -1.72 | -2.40 | 12.33 | -0.14 | 0.50 |
| | Muscle | Middle stage | -3.07 | -4.06 | -0.47 | -0.62 | -0.46 | 3.71 | 7.04 | -2.61 | 4.63 | 1.82 | 0.52 | 0.11 | 13.29 | 1.46 | 2.01 |
| | Skin | Early stage | -4.13 | -4.51 | -0.90 | -1.05 | -0.81 | 3.52 | 6.77 | -3.17 | 4.31 | 1.43 | 0.05 | -0.42 | 13.18 | 1.20 | 1.87 |
| | Skin | Middle stage | -6.39 | -7.11 | -3.62 | -3.27 | -3.56 | 1.06 | 3.38 | -5.67 | 1.33 | -2.02 | -3.04 | -3.68 | 11.18 | -1.14 | -0.58 |
| | Spleen | Early stage | -6.60 | -7.07 | -3.82 | -3.74 | -3.65 | 0.68 | 2.65 | -5.86 | 1.02 | -2.38 | -3.25 | -3.76 | 10.89 | -1.54 | -0.96 |
| | Spleen | Middle stage | -0.84 | -1.63 | 1.71 | 1.30 | 1.58 | 5.02 | 8.46 | -0.10 | 5.61 | 4.13 | 2.70 | 2.62 | 14.23 | 3.17 | 3.73 |
| | Testis | Early stage | -3.84 | -4.56 | -1.32 | -1.51 | -1.31 | 2.75 | 5.09 | -3.27 | 3.16 | 0.46 | -0.59 | -1.07 | 12.37 | 0.58 | 1.11 |
| | Testis | Middle stage | -3.20 | -4.02 | -0.28 | -0.44 | -0.24 | 3.89 | 7.54 | -2.46 | 4.84 | 2.06 | 0.81 | 0.37 | 13.41 | 1.63 | 2.29 |
| | Adipose | Early stage | -13.10 | -13.49 | -11.03 | -10.59 | -10.60 | -6.54 | -6.35 | -12.58 | -7.47 | -10.58 | -10.66 | -11.45 | 5.96 | -9.72 | -8.23 |
| | Adipose | Middle stage | -14.17 | -14.66 | -11.88 | -11.20 | -11.59 | -6.74 | -6.71 | -13.85 | -7.95 | -11.55 | -11.78 | -12.66 | 5.86 | -9.72 | -8.41 |

Table 5-7

| | | | Brain Early stage | Brain Middle stage | Brain Late stage | Heart Early stage | Heart Middle stage | Heart Late stage | Kidney Early stage | Kidney Middle stage | Kidney Late stage | Liver Early stage | Liver Middle stage | Liver Late stage | Lung Early stage | Lung Middle stage | Lung Late stage | Pancreas Early stage | Pancreas Middle stage | Pancreas Late stage |
|---|---|---|---|---|---|---|---|---|---|---|---|---|---|---|---|---|---|---|---|---|
| Model mice of early onset dementia | Brain | Early stage | -0.03 | -0.86 | -0.16 | -0.59 | 0.96 | -0.15 | 0.65 | 1.20 | 6.21 | -0.81 | -1.34 | -1.79 | -2.65 | -4.65 | -0.84 | 2.99 | 9.41 | 1.57 |
| | Brain | Middle stage | -5.21 | -5.55 | -5.26 | -5.40 | -4.59 | -5.08 | -4.35 | -3.87 | 0.09 | -5.06 | -5.31 | -5.48 | -6.35 | -7.53 | -5.44 | -3.50 | -0.61 | -4.29 |
| | Brain | Late stage | 1.22 | 0.32 | 0.96 | 0.46 | 2.06 | 0.89 | 1.52 | 2.00 | 6.92 | -0.09 | -0.64 | -1.12 | -1.64 | -3.85 | 0.18 | 4.11 | 10.56 | 2.72 |
| | Heart | Early stage | -6.67 | -6.82 | -6.58 | -6.77 | -5.50 | -6.33 | -5.07 | -4.12 | 1.17 | -5.81 | -5.52 | -6.06 | -8.34 | -9.98 | -6.91 | -3.93 | 0.55 | -5.21 |
| | Heart | Middle stage | -2.45 | -2.78 | -2.56 | -2.74 | -1.61 | -2.43 | -1.57 | -0.95 | 3.71 | -2.81 | -2.84 | -3.32 | -4.23 | -6.48 | -2.99 | -0.09 | 4.06 | -1.26 |
| | Heart | Late stage | -1.77 | -2.20 | -1.86 | -2.18 | -0.86 | -1.80 | -0.88 | -0.27 | 4.87 | -2.15 | -2.30 | -2.80 | -3.74 | -5.80 | -2.34 | 0.87 | 5.50 | -0.43 |
| | Kidney | Early stage | -4.61 | -4.86 | -4.62 | -4.69 | -3.38 | -4.24 | -3.12 | -2.30 | 2.77 | -4.29 | -4.16 | -4.64 | -6.48 | -8.66 | -4.98 | -1.74 | 2.96 | -3.12 |
| | Kidney | Middle stage | -2.45 | -2.80 | -2.51 | -2.68 | -1.70 | -2.36 | -1.60 | -1.10 | 3.24 | -2.66 | -2.90 | -3.24 | -4.00 | -5.65 | -2.90 | -0.33 | 3.43 | -1.36 |
| | Kidney | Late stage | -1.58 | -2.09 | -1.70 | -1.95 | -0.62 | -1.55 | -0.62 | -0.03 | 4.88 | -1.97 | -2.19 | -2.66 | -3.69 | -5.90 | -2.19 | 1.20 | 6.28 | -0.15 |
| | Liver | Early stage | -1.11 | -1.61 | -1.22 | -1.45 | -0.41 | -1.15 | -0.43 | 0.06 | 4.65 | -1.55 | -1.82 | -2.21 | -2.88 | -4.58 | -1.64 | 1.08 | 5.25 | -0.02 |
| | Liver | Middle stage | -1.73 | -2.08 | -1.76 | -1.97 | -1.21 | -1.76 | -1.18 | -0.76 | 3.08 | -2.05 | -2.25 | -2.61 | -3.03 | -4.25 | -2.11 | -0.09 | 2.96 | -0.93 |
| | Liver | Late stage | -0.96 | -1.49 | -1.06 | -1.33 | -0.17 | -1.00 | -0.22 | 0.29 | 5.16 | -1.47 | -1.72 | -2.14 | -2.91 | -4.77 | -1.53 | 1.48 | 6.02 | 0.27 |
| | Lung | Early stage | -1.41 | -1.77 | -1.46 | -1.65 | -0.95 | -1.45 | -0.91 | -0.55 | 2.94 | -1.74 | -2.08 | -2.38 | -2.65 | -3.77 | -1.80 | 0.08 | 2.90 | -0.67 |
| | Lung | Middle stage | -6.07 | -6.55 | -6.02 | -6.22 | -5.74 | -6.05 | -5.22 | -4.85 | -1.35 | -5.73 | -6.21 | -6.32 | -7.25 | -7.79 | -6.42 | -4.60 | -2.25 | -5.32 |
| | Lung | Late stage | -3.64 | -4.04 | -3.65 | -3.89 | -2.62 | -3.51 | -2.29 | -1.57 | 3.65 | -3.44 | -3.55 | -3.95 | -6.10 | -7.79 | -4.43 | -0.71 | 4.44 | -2.13 |
| | Pancreas | Early stage | -4.78 | -5.23 | -4.87 | -4.85 | -3.54 | -4.46 | -3.16 | -2.32 | 3.14 | -4.38 | -4.34 | -4.75 | -6.87 | -8.81 | -5.27 | -1.77 | 3.72 | -3.26 |
| | Pancreas | Middle stage | -3.06 | -3.54 | -3.23 | -3.42 | -2.09 | -2.97 | -1.93 | -1.28 | 3.96 | -3.14 | -3.35 | -3.72 | -5.02 | -7.04 | -3.55 | -0.39 | 4.64 | -1.69 |
| | Pancreas | Late stage | 1.86 | 1.09 | 1.79 | 1.25 | 2.60 | 1.60 | 2.16 | 2.52 | 7.38 | 0.56 | -0.06 | -0.54 | -0.64 | -3.08 | 0.98 | 4.66 | 10.10 | 3.14 |
| | Muscle | Early stage | -7.71 | -8.10 | -7.45 | -7.74 | -6.87 | -7.48 | -5.82 | -5.18 | 0.22 | -6.42 | -6.61 | -6.84 | -9.30 | -10.13 | -7.86 | -4.93 | -0.77 | -6.24 |
| | Muscle | Middle stage | -5.11 | -5.27 | -5.12 | -5.28 | -4.29 | -5.01 | -4.11 | -3.47 | 1.21 | -4.97 | -4.86 | -5.24 | -6.58 | -8.16 | -5.54 | -2.98 | 0.65 | -3.97 |
| | Muscle | Late stage | -4.47 | -4.81 | -4.39 | -4.60 | -3.47 | -4.46 | -3.20 | -2.53 | 2.72 | -4.20 | -4.18 | -4.72 | -6.24 | -7.78 | -4.90 | -1.83 | 2.63 | -3.22 |
| | Skin | Early stage | -8.74 | -9.28 | -8.26 | -8.53 | -7.91 | -8.33 | -6.95 | -6.14 | -1.11 | -7.49 | -7.49 | -7.91 | -10.13 | -10.86 | -8.74 | -6.17 | -2.49 | -7.66 |
| | Skin | Middle stage | -2.67 | -2.91 | -2.67 | -2.79 | -2.44 | -2.68 | -2.33 | -2.15 | 0.01 | -2.74 | -3.07 | -3.19 | -3.31 | -3.83 | -2.84 | -1.83 | -0.37 | -2.26 |
| | Skin | Late stage | -2.41 | -2.87 | -2.51 | -2.69 | -1.58 | -2.38 | -1.42 | -0.86 | 4.00 | -2.58 | -2.82 | -3.22 | -4.30 | -6.14 | -2.92 | 0.10 | 4.64 | -1.14 |
| | Spleen | Early stage | 0.20 | -0.35 | 0.10 | -0.23 | 0.91 | 0.10 | 0.72 | 1.17 | 5.71 | -0.52 | -0.99 | -1.36 | -1.76 | -3.63 | -0.43 | 2.41 | 7.03 | 1.29 |
| | Spleen | Middle stage | -13.45 | -14.09 | -13.17 | -13.35 | -12.72 | -13.03 | -11.71 | -11.13 | -6.55 | -11.85 | -12.05 | -12.11 | -14.49 | -14.94 | -13.47 | -11.35 | -8.76 | -12.42 |
| | Spleen | Late stage | 2.86 | 2.14 | 2.79 | 2.33 | 3.35 | 2.55 | 2.91 | 3.21 | 7.49 | 1.53 | 0.90 | 0.44 | 0.72 | -1.34 | 2.08 | 4.86 | 9.39 | 3.79 |
| | Testis | Early stage | 1.79 | 1.06 | 1.77 | 1.21 | 2.45 | 1.56 | 2.11 | 2.46 | 7.13 | 0.57 | -0.02 | -0.48 | -0.55 | -2.87 | 0.97 | 4.31 | 9.37 | 2.92 |
| | Testis | Middle stage | -5.39 | -5.72 | -5.44 | -5.60 | -4.87 | -5.37 | -4.67 | -4.16 | -0.47 | -5.32 | -5.43 | -5.73 | -6.48 | -7.52 | -5.64 | -3.86 | -1.21 | -4.64 |
| | Testis | Late stage | 4.60 | 3.71 | 4.29 | 3.67 | 5.06 | 4.01 | 4.31 | 4.47 | 8.74 | 2.56 | 1.70 | 1.21 | 1.93 | -0.46 | 3.46 | 6.96 | 12.18 | 5.74 |
| | Adipose | Early stage | -3.29 | -3.65 | -3.34 | -3.48 | -2.62 | -3.28 | -2.43 | -1.86 | 2.57 | -3.44 | -3.57 | -3.93 | -4.90 | -6.53 | -3.73 | -1.24 | 2.44 | -2.25 |
| | Adipose | Middle stage | -9.44 | -9.95 | -9.16 | -9.32 | -9.12 | -9.35 | -8.32 | -8.12 | -4.67 | -8.60 | -9.33 | -9.28 | -10.31 | -10.52 | -9.46 | -7.94 | -6.08 | -8.74 |
| | Adipose | Late stage | 2.69 | 2.20 | 2.48 | 2.21 | 3.24 | 2.50 | 2.83 | 3.10 | 6.79 | 1.57 | 1.08 | 0.68 | 0.98 | -0.69 | 2.05 | 4.46 | 8.30 | 3.62 |

0.00

More than 0.00, 0.15 or less

Table 5-8

| | | | Muscle Early stage | Muscle Middle stage | Muscle Late stage | Skin Early stage | Skin Middle stage | Skin Late stage | Spleen Early stage | Spleen Middle stage | Spleen Late stage | Testis Early stage | Testis Middle stage | Testis Late stage | Adipose Early stage | Adipose Middle stage | Adipose Late stage |
|---|---|---|---|---|---|---|---|---|---|---|---|---|---|---|---|---|---|
| Model mice of early onset dementia | Brain | Early stage | -3.11 | -3.87 | -0.20 | -0.40 | -0.17 | 4.04 | 7.42 | -2.30 | 4.99 | 2.24 | 0.82 | 0.47 | 13.61 | 1.79 | 2.35 |
| | Brain | Middle stage | -6.65 | -6.91 | -4.96 | -4.94 | -4.82 | -1.90 | -0.99 | -6.14 | -2.00 | -4.04 | -4.56 | -4.89 | 6.74 | -3.52 | -3.14 |
| | Brain | Late stage | -2.20 | -3.05 | 0.68 | 0.42 | 0.70 | 4.76 | 8.60 | -1.42 | 5.86 | 3.45 | 1.85 | 1.63 | 14.01 | 2.54 | 3.11 |
| | Heart | Early stage | -8.77 | -9.20 | -6.22 | -5.83 | -5.86 | -1.40 | -0.07 | -8.05 | -1.58 | -4.84 | -5.23 | -6.06 | 8.87 | -3.53 | -2.94 |
| | Heart | Middle stage | -4.76 | -5.57 | -2.50 | -2.34 | -2.27 | 1.47 | 3.37 | -4.01 | 1.72 | -0.82 | -1.66 | -1.98 | 10.96 | -0.45 | 0.03 |
| | Heart | Late stage | -4.16 | -4.92 | -1.75 | -1.68 | -1.64 | 2.26 | 4.57 | -3.60 | 2.82 | 0.07 | -0.89 | -1.31 | 11.71 | 0.23 | 0.74 |
| | Kidney | Early stage | -6.64 | -7.31 | -4.12 | -3.89 | -3.94 | 0.34 | 2.14 | -6.12 | 0.44 | -2.62 | -3.41 | -3.90 | 10.34 | -1.73 | -1.21 |
| | Kidney | Middle stage | -4.34 | -4.96 | -2.35 | -2.37 | -2.26 | 1.17 | 2.79 | -3.81 | 1.34 | -0.98 | -1.74 | -2.07 | 10.25 | -0.64 | -0.18 |
| | Kidney | Late stage | -4.11 | -4.91 | -1.52 | -1.55 | -1.41 | 2.63 | 5.21 | -3.57 | 3.23 | 0.38 | -0.67 | -1.10 | 12.34 | 0.47 | 1.01 |
| | Liver | Early stage | -3.25 | -3.89 | -1.16 | -1.21 | -1.08 | 2.34 | 4.28 | -2.68 | 2.69 | 0.40 | -0.45 | -0.77 | 11.37 | 0.49 | 0.97 |
| | Liver | Middle stage | -3.30 | -3.93 | -1.74 | -1.78 | -1.68 | 1.14 | 2.42 | -2.91 | 1.28 | -0.61 | -1.19 | -1.47 | 9.47 | -0.40 | 0.00 |
| | Liver | Late stage | -3.29 | -4.08 | -1.02 | -1.07 | -0.94 | 2.70 | 5.02 | -2.72 | 3.22 | 0.74 | -0.22 | -0.58 | 12.01 | 0.73 | 1.23 |
| | Lung | Early stage | -2.95 | -3.42 | -1.43 | -1.53 | -1.38 | 1.25 | 2.41 | -2.48 | 1.32 | -0.39 | -0.93 | -1.19 | 9.41 | -0.23 | 0.16 |
| | Lung | Middle stage | -7.41 | -7.56 | -5.77 | -5.96 | -5.72 | -3.27 | -2.48 | -6.81 | -3.32 | -5.15 | -5.40 | -5.88 | 4.90 | -4.94 | -4.48 |
| | Lung | Late stage | -5.98 | -6.67 | -3.29 | -3.17 | -3.07 | 1.21 | 3.37 | -5.29 | 1.49 | -1.61 | -2.48 | -3.08 | 11.25 | -1.00 | -0.42 |
| | Pancreas | Early stage | -7.00 | -7.77 | -4.24 | -3.98 | -3.92 | 0.52 | 2.55 | -6.34 | 0.67 | -2.67 | -3.52 | -4.22 | 10.83 | -1.69 | -1.13 |
| | Pancreas | Middle stage | -5.43 | -5.82 | -2.82 | -2.74 | -2.59 | 1.36 | 3.46 | -4.69 | 1.69 | -1.19 | -2.10 | -2.59 | 11.12 | -0.69 | -0.16 |
| | Pancreas | Late stage | -1.26 | -2.10 | 1.40 | 1.04 | 1.30 | 4.96 | 8.44 | -0.50 | 6.08 | 3.80 | 2.43 | 2.24 | 13.99 | 2.82 | 3.46 |
| | Muscle | Early stage | -9.56 | -9.61 | -6.94 | -6.71 | -6.63 | -2.43 | -1.29 | -8.53 | -2.66 | -5.85 | -6.32 | -7.18 | 7.93 | -4.56 | -3.96 |
| | Muscle | Middle stage | -6.87 | -7.49 | -5.06 | -4.73 | -4.71 | -1.05 | 0.14 | -6.21 | -1.15 | -3.58 | -4.33 | -4.61 | 8.29 | -2.77 | -2.35 |
| | Muscle | Late stage | -6.48 | -7.14 | -4.31 | -3.94 | -4.12 | 0.19 | 1.89 | -5.93 | 0.26 | -2.63 | -3.35 | -3.94 | 10.05 | -1.85 | -1.31 |
| | Skin | Early stage | -10.00 | -10.86 | -7.87 | -8.45 | -7.86 | -4.02 | -2.86 | -9.98 | -4.16 | -7.22 | -7.30 | -8.14 | 6.79 | -6.26 | -5.28 |
| | Skin | Middle stage | -3.50 | -3.68 | -2.61 | -2.77 | -2.63 | -1.16 | -0.57 | -3.25 | -1.13 | -2.13 | -2.38 | -2.56 | 4.21 | -2.08 | -1.82 |
| | Skin | Late stage | -4.64 | -5.41 | -2.32 | -2.41 | -2.25 | 1.95 | 3.78 | -4.22 | 2.04 | -0.67 | -1.55 | -1.97 | 11.00 | -0.33 | 0.19 |
| | Spleen | Early stage | -2.27 | -2.82 | 0.03 | -0.14 | 0.05 | 3.63 | 5.58 | -1.65 | 4.52 | 1.80 | 0.85 | 0.54 | 12.48 | 1.64 | 2.23 |
| | Spleen | Middle stage | -14.43 | -14.48 | -12.42 | -12.77 | -12.52 | -9.23 | -8.64 | -14.51 | -10.44 | -12.20 | -12.24 | -13.04 | 0.95 | -11.34 | -10.65 |
| | Spleen | Late stage | 0.19 | -0.63 | 2.35 | 2.01 | 2.20 | 5.51 | 7.75 | 0.89 | 7.07 | 4.37 | 3.26 | 3.00 | 14.06 | 3.56 | 4.23 |
| | Testis | Early stage | -1.16 | -1.95 | 1.39 | 1.03 | 1.27 | 4.81 | 8.19 | -0.41 | 5.73 | 3.57 | 2.42 | 2.14 | 13.85 | 2.73 | 3.42 |
| | Testis | Middle stage | -6.77 | -7.11 | -5.23 | -5.26 | -5.13 | -2.38 | -1.55 | -6.40 | -2.53 | -4.45 | -4.75 | -5.20 | 5.99 | -3.89 | -3.50 |
| | Testis | Late stage | 1.22 | 0.24 | 3.66 | 3.08 | 3.53 | 6.66 | 10.63 | 1.94 | 8.08 | 6.33 | 4.80 | 4.84 | 15.21 | 4.65 | 5.22 |
| | Adipose | Early stage | -5.22 | -5.90 | -3.27 | -3.16 | -2.97 | 0.38 | 1.83 | -4.59 | 0.46 | -1.88 | -2.54 | -2.97 | 9.86 | -1.43 | -0.97 |
| | Adipose | Middle stage | -10.45 | -10.66 | -8.93 | -9.22 | -8.96 | -6.69 | -6.09 | -9.93 | -6.74 | -8.61 | -8.79 | -9.22 | 1.36 | -8.60 | -8.03 |
| | Adipose | Late stage | 0.50 | -0.21 | 2.34 | 2.13 | 2.28 | 5.28 | 7.42 | 1.01 | 5.75 | 3.94 | 2.93 | 2.92 | 13.66 | 3.85 | 4.45 |

(black) 0.00
(grey) More than 0.00, 0.15 or less

Tables 5-1 to 5-8 show that, in the same organ, when diseases are different, the absolute value of the zi value is large.

Reference Example 1

Experimental Example I. Establishment of Oscar gene mutant mice

1. Construction of gRNA and Cas9 expression vector

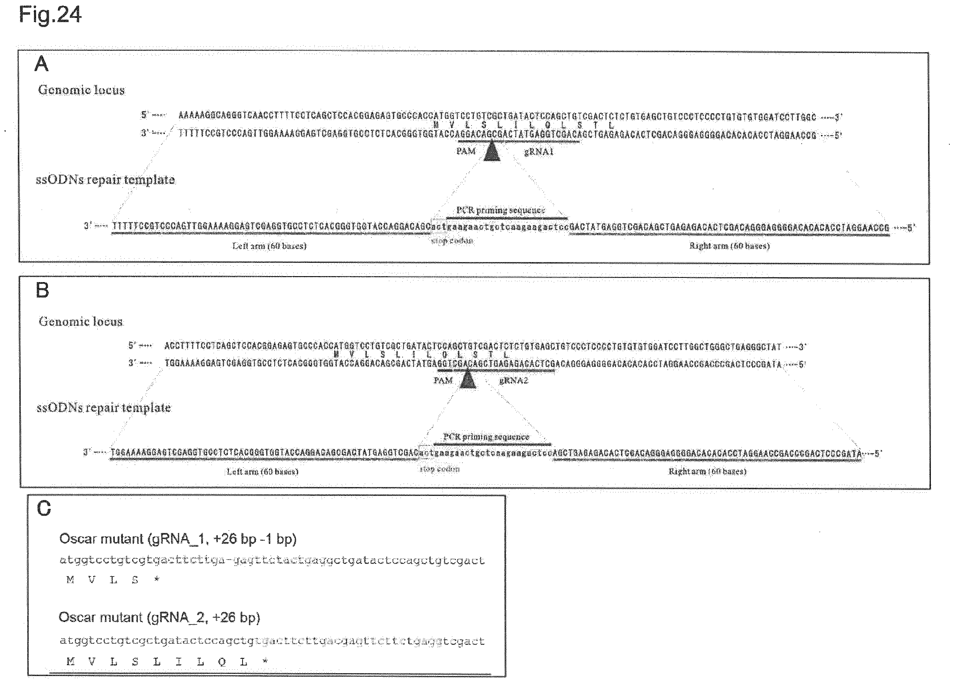

gRNA sequences (Oscar-gRNAl and Oscar-gRNA2) were designed, and oligo DNA encoding the gRNA sequences was synthesized using an Optimized CRISPR design tool (publicly available on the website of Massachusetts Institute of Technology, Zhang Lab (http://crispr.mit.edu/)). The Oscar gene target sequences contained in these sequences are the following sequences: ACAGCTGGAGTATCAGCGAC (SEQ ID NO: 5) in Oscar-gRNAl and GCTCACAGAGAGTCGACAGC (SEQ ID NO: 6) in Oscar-gRNA2, which are present in exon 1 of the Oscar gene. These were individually inserted into pX330-U6-Chimeric_BB-CBh-hSpCas9, which is a Cas9 expression vector (pX330-Oscar-gRNA1 and pX330-Oscar-gRNA2). The nucleotide sequences of the gRNA insertion sites in the obtained vectors were determined to confirm that gRNA was inserted as designed. In addition, single-stranded oligodeoxynucleotides (ssODNs) were individually synthesized so as to include the respective Oscar gene target sequences. The donor oligo DNA region was designed so that a stop codon is placed immediately downstream of the Serine coding sequence of the predetermined Oscar-gRNAl cleavage site, and immediately downstream of the Leucine coding sequence of the predetermined Oscar-gRNA2 cleavage site (Fig. 24A and Fig. 24B).

ssODN for Oscar-gRNAl:

GCCAAGGATCCACACACAGGGGAGGGACAGCTCACAGAGAGTCGACAGCTGGAGTATCAGcctca
gaagaactcgtcaagaagtcaCGACAGGACCATGGTGGGCACTCTCCGTGGAGCTGAGGAAAAGG
TTGACCCTGCCTTTTT (SEQ ID NO: 7)

ssODN for Oscar-gRNA2:

ATAGCCCTCAGCCCAGCCAAGGATCCACACACAGGGGAGGGACAGCTCACAGAGAGTCGAcctca
gaagaactcgtcaagaagtcaCAGCTGGAGTATCAGCGACAGGACCATGGTGGGCACTCTCCGTG
GAGCTGAGGAAAAGGT (SEQ ID NO: 8)

2. Establishment of Oscar gene mutant mice

pX330-Oscar-gRNA1 and pX330-Oscar-gRNA2 were individually injected into C57BL/6N Slc fertilized eggs together with the corresponding ssODN. The fertilized eggs after the injection were injected into the oviducts of pseudopregnant ICR female mice. The genotype of the F0 mice was confirmed by PCR (for the primers, see Table 6) and direct sequencing. F1 mice were obtained by mating the F0 mice.

The genotype of each mouse was determined by direct sequencing and high-resolution melting (HRM) analyses using the primers shown in Table 6.

Fig. 24C shows the sequences of the genotypes of the obtained mutant mice.

Experimental Example II. Establishment of disease model mice

1. Establishment of UNx/HPi model mice

UNx/HPi mice (unilateral nephrectomy- a diet with high phosphorus content-ingested mice) were obtained by feeding mice a diet with high phosphorus content after unilateral nephrectomy. As a control, mice were obtained by feeding them a diet with low phosphorus content after a sham operation.

1-1. Unilateral nephrectomy

After mice (C57BL/6J, 8 weeks old, male) were anesthetized by intraperitoneal administration of Avertin (250 mg/kg), the skin was incised from the back. The right renal artery and vein, and ureter were ligated. After cutting on the distal side of the ligation, the right kidney was removed, and the incision was closed. The control mice were subjected to a sham operation. In the sham operation, the right renal artery and vein, and ureter were exposed, and the incision was closed without ligation. In order to wait for the mice to completely recover from operative stress, the mice were fed a 0.54% inorganic phosphorus-containing normal diet (CE-2, CLEA Japan, Inc.) for 4 weeks.

1-2. Phosphorus overload and collection of tissue

From 4 weeks after the completion of the operation (12 weeks old), the unilaterally nephrectomized mice were given a diet with high phosphorus content in which contains (TD.10662, OrientalBioService, Inc.) (hereinafter also referred to as the "kidney disease group"). The sham-operated mice were given a diet with low phosphorus content in which contains (TD.10662 modified type, OrientalBioService, Inc.) (hereinafter also referred to as the "Sham group").

The model mice of chronic kidney disease were obtained by a modification of the method described in Hu M.C. et al. (J Am Soc Nephrol 22, 124-136, 2011). In Hu M.C. et al., the remaining kidney (left kidney) is subjected to ischemia-reperfusion injury at the time of unilateral nephrectomy in Item 1 above. However, in this modification, ischemia-reperfusion was not performed. Tissue was collected 1 week (E), 4 weeks (M), and 8 weeks (L) after the start of the diet with high phosphorus content (the diet with low phosphorus content in which contains in the Sham group).

The animals from which the tissue was to be collected were euthanized by cervical dislocation after blood was collected from the orbit under anesthesia, and the organs and tissue (bone marrow, brain, skin, heart, kidney, liver, lung, pancreas, skeletal muscle, spleen, testis, thymus, adipose, colon, stomach, adrenal glands, aorta, eyes, ileum, jejunum, pituitary gland, skull, salivary glands, and thyroid gland) were collected. After the wet weights of the collected organs and tissue were measured, the organs and tissue were rapidly frozen in liquid nitrogen and stored at -80°C.

2. Establishment of model of phosphorus-overloaded mice

2-1. Phosphorus overload

WT mice (C57BL/6N, 7 weeks old, male) or Oscar gene mutant mice (7-16 weeks old, male/female) were fed a 0.54% inorganic phosphorus-containing normal diet (CE-2, CLEA Japan, Inc.) for 1 week. Thereafter, the mice were given a diet with high phosphorus content in which contains 2% inorganic phosphorus (TD.10662, OrientalBioService, Inc.) or a diet with high phosphorus content in which contains 0.35% inorganic phosphorus (TD.10662 modified type, OrientalBioService, Inc.) as a special phosphorus diet.

2-2. Collection of tissue

The skull was collected at the time of the start of the special phosphorus diet fed to the WT mice (8 weeks old), 1 day, 3 days, 1 week (9 weeks old), and 4 weeks (12 weeks old) after the start. In the Oscar gene mutant mice, the skull was collected 1 week after the start of the special phosphorus diet. The animals from which the tissue was to be collected were euthanized by cervical dislocation after being anesthetized by intraperitoneal administration of Avertin (250 mg/kg), and the tissue was collected. After the weight of the collected tissue was measured, the tissue was rapidly frozen in liquid nitrogen, and stored at -80°C.

Experimental Example III. Analysis of gene expression in each tissue

1. Extraction of RNA from each tissue

Each cryopreserved tissue was individually homogenized in TRIzol Reagent (Thermo Fisher Scientific, MA, USA) with a PT10-35 GT Polytron homogenizer (KINEMATICA, Luzern, Switzerland) at 15,000 r.p.m. for 10 minutes; or ground with a mortar and pestle in liquid nitrogen, dried, and then homogenized in TRIzol Reagent (Thermo Fisher Scientific, MA, USA) with a PT10-35 GT Polytron homogenizer (KINEMATICA, Luzern, Switzerland) at 15,000 r.p.m. for 10 minutes; or homogenized using zirconia beads of different sizes (1.5 mm diameter × 50, 3 mm diameter × 5, 5 mm diameter × 2) with Cell Destroyer PS1000 or PS2000 (Bio Medical Science Inc., Tokyo, Japan) at 4,260 r.p.m. for 45 seconds at 4°C. After incubation at room temperature for 5 minutes to separate proteins, 0.2 mL of chloroform was added per mL of TRIzol, and the tube was capped. Subsequently, the mixture was vortexed vigorously for 15 seconds. After the vortexing, the mixture was incubated at room temperature for 3 minutes and centrifuged at 12,000 g for 15 minutes at 4°C, and the RNA-containing aqueous layer was collected in a fresh tube. An equal amount of 70% ethanol was added to the collected aqueous layer, and mixed. Then, 700 μL of the mixture was applied to each RNeasy Mini column (Qiagen), and purified RNAs were collected according to the RNeasy Mini kit (Qiagen) standard protocol. The quality and concentration of each of the collected RNAs was evaluated by using NanoDrop (Thermo Fisher Scientific, MA, USA).

2. Analysis of RNA expression (RNA-Seq)

(1) Obtaining RNA-Seq data

RNA-Seq data was obtained using the samples described above by the following procedure.

a. Quality check

Quality testing of the samples was performed based on the following items.

- Concentration measurement using NanoDrop (spectrophotometer)
- Concentration measurement and quality check using Agilent 2100 Bioanalyzer

b. Preparation of sample

A library for a HiSeq next-generation sequencer was prepared with SureSelect Strand-Specific RNA library preparation kit in the following manner.

i. Collection of poly (A)+RNA (mRNA) from total RNA using Oligo (dT) magnetic beads
ii. Fragmentation of RNA
iii. cDNA synthesis
iv. Double-stranded cDNA synthesis
v. Terminus repair, phosphorylation, A tail addition
vi. Ligation of adapters with indices
vii. 13-cycle PCR
viii. Purification with magnetic beads

c. Obtaining data using next-generation sequencer

Sequencing was performed using a HiSeq 2500 or 4000 next-generation sequencer (Illumina) in the following manner.

i. Addition of sequencing reagent

Reagent: TruSeq PE Cluster Kit v3 - cBot - HS (1 flowcell) <PE-401-3001> (Illumina)
Reagent: TruSeq SBS Kit v3 - HS (200 cycle) <FC-401-3001> (Illumina)

ii. Single-base extension reaction
iii. Removal of unreacted bases
iv. Incorporation of fluorescent signal
v. Removal of protecting groups and fluorescence The cycle was repeated (e.g., cycle 2, cycle 3 ...) and these steps were carried out to 100 cycles.
vi. For the opposite strand (Read 2), i to v were carried out to 100 cycles.

(2) Analysis of RNA-Seq data

(2)-1. Analysis of output data obtained using next-generation sequencer

The following information processing was carried out for the output data.

i. Base calling: text data of nucleotide sequences was obtained from the output raw data of analysis (image data).
ii. Filtering: selection of read data by predetermined filtering was performed.

iii. Sorting based on index sequences: sample data was sorted based on index information.

(2)-2. Secondary analysis of output data

The data file (Fastq format) obtained using Illumina HiSeq 2500 or 4000 was uploaded on Galaxy (https://usegalaxy.org/) downloaded to a local server. Thereafter, analysis was carried out using Bowtie2 (http://bowtie-bio.sourceforge.net/bowtie2/index.shtml) to map each sequence to mouse genome map information mm10. The BAM file obtained using Bowtie2 was analyzed using Cufflinks (http://cole-trapnell-lab.github.io/cufflinks/) to calculate FPKM (RPKM) of genes. That is, in this analysis, all RNAs expressed in each tissue were analyzed.

3. qRT-PCR

1 μg of total RNA obtained from each tissue was used as a template for cDNA synthesis, and cDNA was synthesized using Oligo dT20 primer according to the standard protocol of SuperScript III First-Strand Synthesis SuperMix (Life Technologies). After the synthesized cDNA was diluted 20-fold with TE buffer (10 mM Tris-HCl, pH 8.0, 0.1 mM EDTA), real-time PCR was performed with a LightCycler 480 II (Roche) according to the standard protocol of LightCycler 480 SYBR Green I Master (Roche, Basel, Switzerland), and Cp values were measured. The relative expression level of each gene to a reference gene was quantified by comparing the Cp value obtained for each gene with the Cp value for β2-microglobulin (B2m) as the reference gene. The primer pairs used in the real-time PCR are as shown in Table 6. All of the primers were designed by using Primer-BLAST (NCBI).

Table 6

| Target | Sequence | SEQ ID NO. |
|---|---|---|
| Forward primer for direct-sequence analysis | CTACTTAGCGACAACGTCCT | 9 |
| Reverse primer for direct-sequence analysis | GCCTTGGGGTTTGAAGGTTT | 10 |
| Sequence primer for direct-sequence analysis | CAGAGGCTATGACTGTTCCA | 11 |
| Forward primer for HRM analysis | GGCAGGGTCAACCTTTTCCT | 12 |
| Reverse primer for HRM analysis | AGGGACAGCTCACAGAGAGT | 13 |
| Forward primer: B2m | GCTCGGTGACCCTGGTCTTT | 14 |
| Reverse primer: B2m | AATGTGAGGCGGGTGGAACT | 15 |
| Forward primer: Oscar | CGGGCATGAGTTTTGCACTG | 16 |
| Reverse primer: Oscar | TGGGTATAGTCCAAGGAGCCA | 17 |
| Forward primer: Fgf23 | AGGAGCCATGACTCGAAGGT | 18 |
| Reverse primer: Fgf23 | GCTCACCAGGTAGTGATGCTT | 19 |

4. Analysis of differentially expressed genes

To extract differentially expressed genes, the number of annotation reads of each transcript in sequence data mapped by Bowtie2 was counted by using HTSeq-count (parameter: -r was pos, and -s was no). The obtained results were analyzed by DESeq2 (Love, M.I., Huber, W. & Anders, S.; Genome biology 15, 550, doi:10.1186/s13059-014-0550-8 (2014)) with default settings. Expression differences were compared in E-UNx/HPi vs. E-/M-/L-Sham (n=3), M-UNx/HPi vs. E-/M-/L-Sham (n=3), and L-UNx/HPi vs. E-/M-/L-Sham (n=3). Gene ontology (GO) enrichment analysis was performed using R package "topGO." In the gene ontology (GO) enrichment analysis, the case in which the result of the DESeq2 analysis is 1 or more and the p-value is less than 0.05 was defined as "$\log_2$ (fold-change)."

5. Statistical analysis

In statistical analysis, Student's t-test or one-way analysis of variance was performed, and then significant differences were determined by the Tukey-Kramer test. The case in which the p-value is less than 0.05 was defined as being significant (* $p < 0.05$, ** $p < 0.01$, and *** $p < 0.001$). In the scatter plots, the average is indicated by a horizontal line.

6. Results

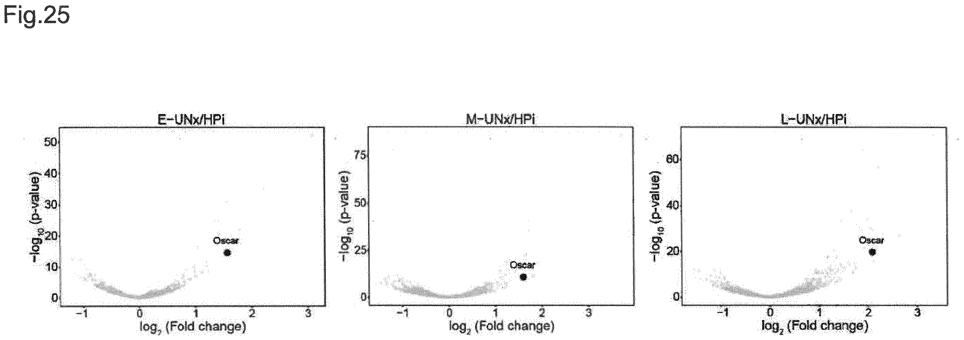

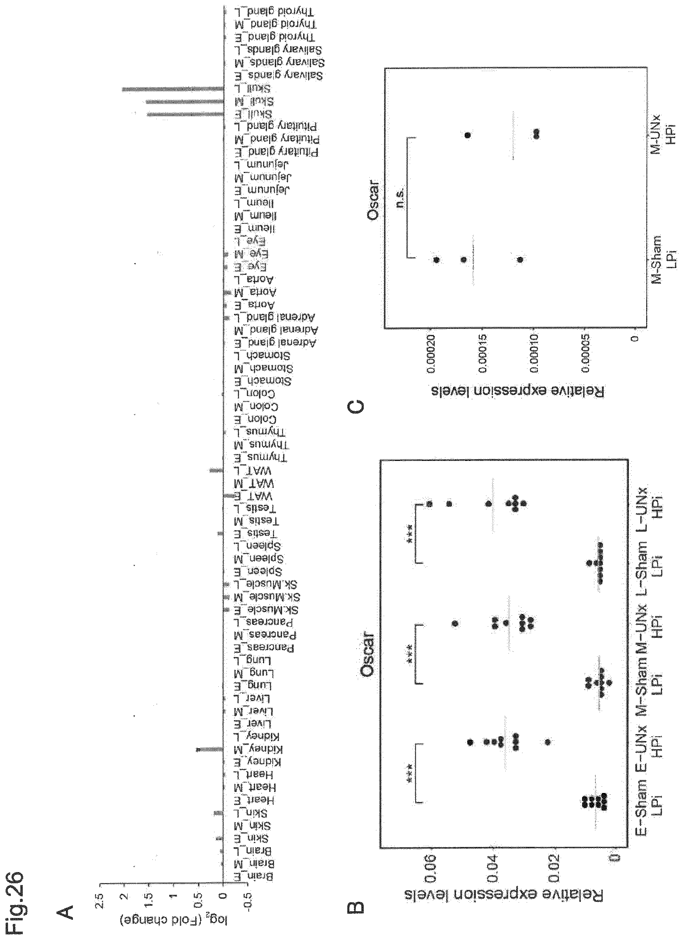

Fig. 25 shows volcano plots of the skull 1 week (E-UNx/HPi), 4 weeks (M-UNx/HPi), and 8 weeks (L-UNx/HPi) after the start of the diet with high phosphorus content. Each mRNA after DESeq analysis was plotted as a small dot, and Oscar was indicated by a large dot. The expression of the Oscar gene increased in the UNx/HPi model mice (HPi) at E-UNx/HPi, M-UNx/HPi, L-UNx/HPi compared with that in the sham-operated mice (LPi). Fig. 26A shows DESeq analysis results of expression of the Oscar gene in each tissue. The Oscar gene showed high expression in the skull of the UNx/HPi model at E-, M-, L-. This result was confirmed by qRT-PCR (n = 8 to 9). As a result, it was revealed that the expression of the Oscar gene increased in the skull in the UNx/HPi model mice (HPi) at E-UNx/HPi, M-UNx/HPi, and L-UNx/HPi compared with that in the sham-operated mice (LPi) (Fig. 26B). The expression of the Oscar gene slightly increased in the kidney in the DESeq analysis; however, no significant difference was observed in confirmation by qRT-PCR (Fig. 26C).

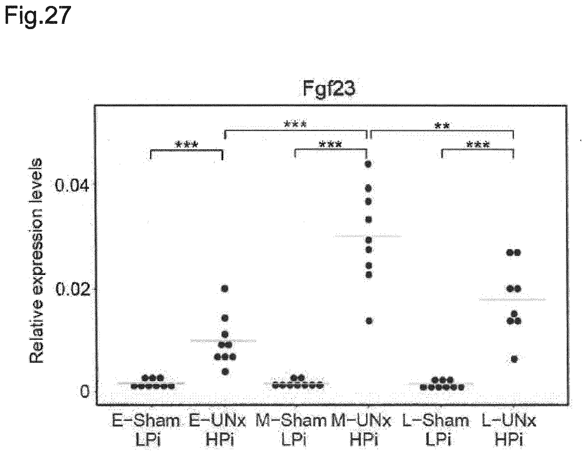

FGF23 is a master regulator of inter-organ cross talk between the parathyroid glands, bones, and kidneys in kidney disease. The expression of the FGF23 gene in the skull increased in the UNx/HPi model mice (HPi) at E-UNx/HPi, M-UNx/HPi, L-UNx/HPi compared with that in the sham-operated mice (LPi) (Fig. 27) .

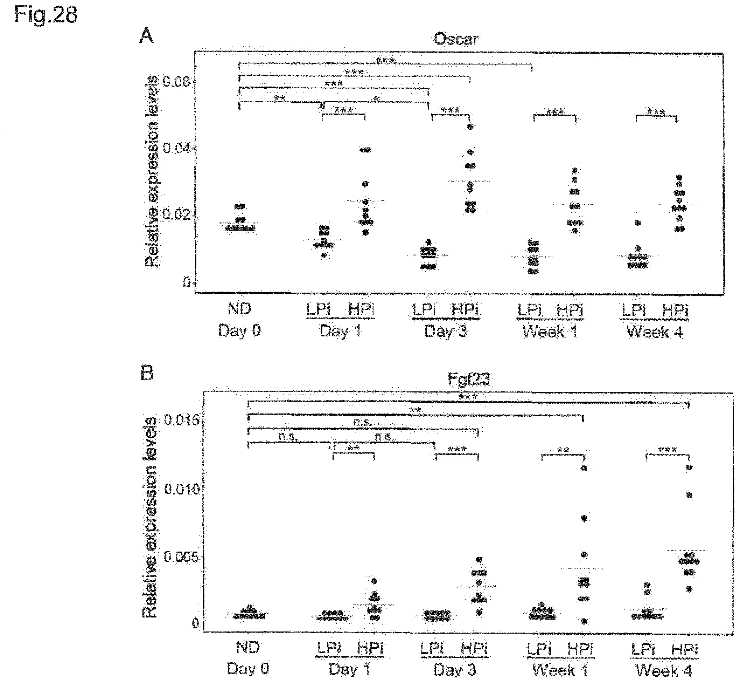

Next, the expression of the Oscar gene and the FGF23 gene over time after the feed of the mice was switched from the normal diet to the diet with low phosphorus content or the diet with high phosphorus content was examined in the skull. Fig. 28A shows the expression of the Oscar gene in the skull 1 day, 3 days, 1 week, and 4 weeks after switching to the diet with low phosphorus content or the diet with high phosphorus content. Fig. 28B shows the expression of the FGF23 gene in the skull 1 day, 3 days, 1 week, and 4 weeks after switching to the diet with low phosphorus content or the diet with high phosphorus content. The expression of the Oscar gene and the FGF23 gene already increased in the skull on day 1 after switching to the diet with high phosphorus content. Because the p-value was lower in Student's t-test, the expression of the Oscar gene increased more robustly than the expression of the FGF23 gene. After the feed was switched from the normal diet to the diet with low phosphorus content, the expression level of the Oscar gene significantly decreased, whereas the expression level of the FGF23 gene was unchanged. After peaking 3 days following the switch to the diet with high phosphorus content, the expression of the Oscar gene showed a tendency to gradually cease to increase. In contrast, the expression of the FGF23 gene gradually increased as the period of the diet with high phosphorus content lengthened. These results showed that the expression of the Oscar gene reflects phosphorus intake more strongly and more sensitively than the expression of FGF23.

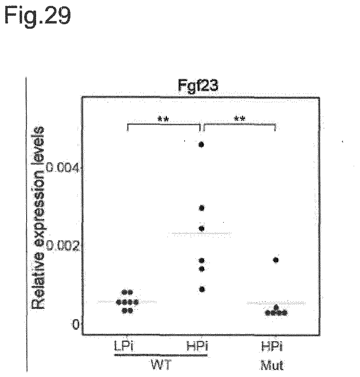

Further, what effect an increase in the expression of Oscar in the bones has on the expression of FGF23 in the bones was examined. The Oscar gene mutant mice obtained in Item I above were fed a diet with high phosphorus content, and the expression of FGF23 in the bones was examined by qRT-PCR. The results revealed that the expression of FGF23 did not increase in the bones of the Oscar gene mutant mice even if they were fed the diet with high phosphorus content, as shown in Fig. 29.

The above results showed that Oscar up-regulates the expression pathway of FGF23. This indicated that suppressing the functional expression of Oscar enables suppression of the functional expression of FGF23, and further that suppressing the functional expression of Oscar enables control of disease involving FGF23.

Reference Example 2: Preparation of soluble human Oscar-Fc fusion protein

An expression plasmid containing a nucleotide sequence encoding a soluble human Oscar-Fc fusion protein was expressed in animal cells, thereby preparing the soluble human Oscar-Fc fusion protein shown in SEQ ID NO: 2. The preparation of the protein was outsourced to ProteinExpress Co., Ltd. (Chiba, Japan).

<Amino acid sequence of humanOscar-humanIgG1>

MALVLILQLLTLWPLCHTDITPSVAIIVPPASYHPKPWLGAQPATVVTPGVNVTLRCRAPQPAWR
FGLFKPGEIAPLLFRDVSSELAEFFLEEVTPAQGGSYRCCYRRPDWGPGVWSQPSDVLELLVTEE
LPRPSLVALPGPVVGPGANVSLRCAGRLRNMSFVLYREGVAAPLQYRHSAQPWADFTLLGARAPG
TYSCYYHTPSAPYVLSQRSEVLVISWEGEGPEARPASDKTHTCPPCPAPELLGGPSVFLFPPKPK
DTLMISRTPEVTCVVVDVSHEDPEVKFNWYVDGVEVHNAKTKPREEQYNSTYRVVSVLTVLHQDW
LNGKEYKCKVSNKALPAPIEKTISKAKGQPREPQVYTLPPSREEMTKNQVSLTCLVKGFYPSDIA
VEWESNGQPENNYKTTPPVLDSDGSFFLYSKLTVDKSRWQQGNVFSCSVMHEALHNHYTQKSLSL
SPGK*(SEQ ID NO: 2)

(The underline indicates a hinge region. The upstream from the hinge region indicates the sequence of human Oscar, and the downstream from the hinge region indicates the human IgG1 sequence.)

1. Preparation of cells

FreeStyle 293-F cells were subcultured in FreeStyle 293 Expression Medium (Invitrogen). The subculture was performed using a 500 ml-flask at the following conditions: a shaking speed of 120 r.p.m., a carbon dioxide concentration of 8%, and a temperature of 37°C (170 mL of medium). The cells were diluted to 6 x $10^5$ cells/mL to a total amount of 1 L the day before transfection and cultured for 24 hours. After the culture, the number of cells was adjusted to 1 x $10^6$ cells/mL, and the cells were used for transfection.

2. Expression test

2.1 mL of 293fectin Transfection Reagent (Invitrogen) was added to 30 mL of OptiPRO SFM (Invitrogen) pre-warmed to 37°C, thereby preparing a reagent solution. Similarly, 1 mg of pcDNA3 (Invitrogen) containing kozak-hOscar-hIgG1-intronXbaI having the following sequence was added to 30 mL of OptiPRO SFM, thereby preparing a DNA solution. The prepared reagent solution and DNA solution were incubated at room temperature for 5 minutes. The reagent solution and DNA solution after the 5 minutes were mixed to prepare a transfection solution. The transfection solution was incubated at room temperature for 20 minutes and added to a culture medium adjusted for transfection, followed by sampling on day 1, day 2, and day 3 after the start of the transfection. The culture was then terminated on day 4. The obtained samples were centrifuged to separate into a supernatant and a precipitate. The protein expression was confirmed by SDS-PAGE and Western blotting.

<kozak-hOscar-hIgG1-intronXbaI>

aagcttgccaccATGGCCCTCGTGCTTATCCTCCAACTTCTCACGCTTTGGCCTCTGTGCCACAC
CGACATTACTCCGTCTGTTGCGATAATTGTCCCTCCCGCCTCTTATCACCCTAAACCTTGGCTGG
GCGCACAGCCAGCTACTGTGGTTACTCCTGGGGTGAACGTAACACTGCGCTGCCGTGCTCCTCAG
CCCGCCTGGAGATTTGGGTTGTTTAAGCCCGGAGAGATAGCACCACTGCTGTTTCGGGATGTGTC
CTCAGAGCTGGCTGAGTTCTTCCTGGAAGAGGTCACTCCTGCCCAAGGAGGCAGCTATCGGTGCT
GTTATAGGCGGCCGGATTGGGGACCCGGCGTTTGGTCCCAACCATCTGATGTGCTCGAACTGCTT
GTGACAGAAGAGCTGCCCAGACCTAGCTTGGTAGCCTTGCCCGGTCCTGTCGTCGGACCTGGTGC
CAATGTTTCTCTTCGATGTGCCGGAAGGCTGCGCAATATGTCCTTTGTACTGTATAGGGAGGGAG
TAGCCGCACCTCTGCAGTATAGGCATAGCGCTCAGCCCTGGGCGGATTTTACTCTGCTTGGTGCC
AGAGCACCCGGGACCTATTCCTGCTACTACCACACTCCTTCCGCACCCTACGTCCTGTCACAGAG
ATCAGAAGTGCTCGTGATCTCCTGGGAGGGAGAAGGCCCAGAAGCCGACAAAACTCACACATGCC
CACCGTGCCCAGgtaagccagcccaggcctcgccctccagctcaaggcgggacaggtgccctaga
gtagcctgcatccagggacaggccccagccgggtgctgacacgtccacctccatctcttcctcag
CACCTGAACTCCTGGGGGGACCGTCAGTCTTCCTCTTCCCCCCAAAACCCAAGGACACCCTCATG
ATCTCCCGGACCCCTGAGGTCACATGCGTGGTGGTGGACGTGAGCCACGAAGACCCTGAGGTCAA
GTTCAACTGGTACGTGGACGGCGTGGAGGTGCATAATGCCAAGACAAAGCCGCGGGAGGAGCAGT
ACAACAGCACGTACCGTGTGGTCAGCGTCCTCACCGTCCTGCACCAGGACTGGCTGAATGGCAAG
GAGTACAAGTGCAAGGTCTCCAACAAAGCCCTCCCAGCCCCCATCGAGAAAACCATCTCCAAAGC
CAAAGgtgggacccgtggggtgcgagggccacatggacagaggccggctcggcccaccctctgcc
ctgagagtgactgctgtaccaacctctgtccctacagGGCAGCCCCGAGAACCACAGGTGTACAC
CCTGCCCCCATCCCGGGATGAGCTGACCAAGAACCAGGTCAGCCTGACCTGCCTGGTCAAAGGCT

TCTATCCCAGCGACATCGCCGTGGAGTGGGAGAGCAATGGGCAGCCGGAGAACAACTACAAGACC
ACGCCTCCCGTGCTGGACTCCGACGGCTCCTTCTTCCTCTACAGCAAGCTCACCGTGGACAAGAG
CAGGTGGCAGCAGGGGAACGTCTTCTCATGCTCCGTGATGCATGAGGCTCTGCACAACCACTACA
CGCAGAAGAGCCTCTCCCTGTCTCCGGGTAAATGAtctaga (SEQ ID NO: 20)

3. Purification using Bipo Resin Protein A (10mL)

The culture supernatant (1 L) was loaded on Bipo Resin Protein A (10 mL) to purify a target protein. After washing with PBS, elution of the target protein was performed using 100 mM citric acid buffer (pH 2.5) (5 mL × 5). The eluate was neutralized with 1M Tris-HCl (pH 9.0). After the purification, purity of protein was confirmed in each fraction by SDS-PAGE and Western blotting. The results confirmed a band of the target protein in the elution fraction. The fraction containing the target protein was collected, followed by a buffer exchange into PBS (total amount: 50 mL). Thereby, l mg/mL of a soluble human Oscar-Fc fusion protein was obtained.

Example 2: Experiment of administration of soluble human Oscar-Fc fusion protein to HP model mice

HP mice (mice of diet with high phosphorus content intake) were obtained by feeding mice a diet with high phosphorus content. The soluble human Oscar-Fc fusion protein was administered to this model every week.

1. Phosphorus overload

Mice ingested a diet with high phosphorus content (HP) were obtained by feeding mice (C57BL/6N, 8 weeks old, male) a diet containing 2% inorganic phosphorus (TD.10662, OrientalBioService, Inc.) as a special phosphorus diet. Mice ingested a diet with low phosphorus content (LP) were fed a diet containing 0.35% inorganic phosphorus (TD.10662 modified type, OrientalBioService, Inc.).

2. Administration of soluble human Oscar-Fc fusion protein and collection of tissue

The soluble human Oscar-Fc fusion protein (10 mg/kg) was intraperitoneally administered weekly from the start date of the diet with high phosphorus content (week 0) to week 4, i.e., a total of five times. In this experiment, physiological saline was administered to a control group. Tissue was collected the day after completion of the intraperitoneal administration on week 4, i.e., the fifth intraperitoneal administration. The animals from which the tissue was to be collected were euthanized by cervical dislocation after blood was collected in an EDTA-supplemented tube from the orbit under tribromoethanol anesthesia (250 mg/kg), and the organs and tissue (the skull, brain, pituitary gland, parotid glands, thyroid gland, heart, lung, pancreas, kidney, adrenal glands, liver, spleen, thymus, aorta, femoral muscle, skin, testis, adipose tissue, stomach, jejunum, ileum, colon, and bone marrow cells) were collected. After the wet weights of the collected organs and tissue were measured, the organs and tissue were rapidly frozen in liquid nitrogen and stored at -80°C. The collected blood was centrifuged at 1200 g for 10 minutes at room temperature. After the centrifugation, the supernatant plasma was collected and stored at -80°C.

3. Analysis of gene expression in each tissue and measurement of blood components

3-1. RNA extraction from each tissue

Each cryopreserved tissue was individually homogenized in TRIzol Reagent (Thermo Fisher Scientific, MA, USA) with a PT10-35 GT Polytron homogenizer (KINEMATICA, Luzern, Switzerland) at 15,000 r.p.m. for 10 minutes, or homogenized using zirconia beads of different sizes (1.5 mm diameter × 50, 3 mm diameter × 5, 5 mm diameter × 2) with a Cell Destroyer PS1000 or PS2000 (Bio Medical Science Inc., Tokyo, Japan) at 4,260 r.p.m. for 45 seconds at 4°C. After incubation at room temperature for 5 minutes to separate proteins, 0.2 ml of chloroform was added per mL of TRIzol, and the tube was capped. Subsequently, the mixture was vortexed vigorously for 15 seconds. After the vortexing, the mixture was incubated at room temperature for 3 minutes and centrifuged at 12,000 g for 15 minutes at 4°C, and the RNA-containing aqueous layer was collected in a fresh tube. An equal amount of 70% ethanol was added to the collected aqueous layer, and mixed. Then, 700 μL of the mixture was applied to each RNeasy Mini column (Qiagen), and purified RNAs were collected according to the RNeasy Mini kit (Qiagen) standard protocol. The quality and concentration of each of the collected RNAs was evaluated by using NanoDrop (Thermo Fisher Scientific, MA, USA).

3-2. qRT-PCR

0.5 to 1 μg of total RNA obtained from each tissue was used as a template for cDNA synthesis, and cDNA was synthesized using Oligo dT20 primer according to the standard protocol of SuperScript III First-Strand Synthesis SuperMix (Life Technologies). After the synthesized cDNA was diluted 20-fold with TE buffer (10 mM Tris-HCl, pH 8.0, 0.1 mM EDTA), real-time PCR was performed with a LightCycler 480 II (Roche) according to the standard protocol of LightCycler 480 SYBR Green I Master (Roche, Basel, Switzerland), and Cp values were measured. The relative expression level of each gene to a reference gene was quantified by comparing the Cp value obtained for each gene with the Cp value for Maea as the reference gene. The primer pairs used in the real-time PCR are as shown in Table 7. All of the primers were designed by using Primer-BLAST (NCBI).

Table 7

| | Gene | Forward | SEQ ID NO. | Reverse | SEQ ID NO. |
|---|---|---|---|---|---|
| 1 | Maea | AAGACCTTGAGTAGTTGCCCA | (21) | TGCTCGATCCTACGTTTGCAG | (22) |
| 2 | Fgf23 | AGGAGCCATGACTCGAAGGT | (23) | GCTCACCAGGTAGTGATGCTT | (24) |
| 3 | Prb1 | ACCCCAGCATGGAAACAAAG | (25) | AAGAATGGTATTGAAGTCATCTGTC | (26) |

(continued)

| | Gene | Forward | SEQ ID NO. | Reverse | SEQ ID NO. |
|---|---|---|---|---|---|
| 4 | Prh1 | ACCCCGTGAAGAAAATCAGAA | (27) | TAACAGGCGGTCTTGGTTGG | (28) |
| 5 | Prp2 | TGGTGGTCCTGTTTACAGTGG | (29) | TTCTGAAGTTCTTCACGGGGT | (30) |
| 6 | Prpmp5 | CCTACGAAGACTCAAATTCTCAGC | (31) | GAGGACCATGGTGGTGTCC | (32) |
| 7 | B2m | GCTCGGTGACCCTGGTCTTT | (33) | AATGTGAGGCGGGTGGAACT | (34) |
| 8 | Fgg | CTCCATCGGAGAAGGACAGC | (35) | AGGTCCTGAAAGTCCATTGTCC | (36) |

4. Measurement of concentration of FGF23 in plasma by ELISA method

The concentration of FGF23 in plasma was measured using an ELISA kit sold by KAINOS Laboratories, Inc. (TCY4000). A plasma sample cryopreserved in a freezer was thawed on ice, and the sample undiluted or the sample diluted 5-fold with standard solution 1 (FGF-23 concentration, 0 pg/ml) supplied with the kit was used for measurement.

50 μl of the diluted sample or a sample for a calibration curve was added to each well of an ELISA plate supplied with the kit. The plate was sealed, followed by stirring and incubating at room temperature for 2 hours. After the two hours, the sample in each well was aspirated and discarded. 300 μl of a wash solution supplied with the kit was then added to each well, and the wash solution was removed. This operation was performed four times. After the wash solution was thoroughly removed, 100 μl of an enzyme-labeled antibody solution (FGF-23 Conjugate) supplied with the kit was added to each well, and the plate was sealed, followed by stirring and incubating at room temperature for 1 hour. After the 1 hour, the sample in each well was aspirated and discarded. 300 μl of the wash solution supplied with the kit was then added to each well, and the wash solution was removed. This operation was performed four times. After the wash solution was thoroughly removed, 100 μl of a substrate solution (Substrate) supplied with the kit was added to each well, and the plate was allowed to stand at room temperature for 30 minutes in the shade. Thereafter, 100 μl of a reaction stop solution (Stop Solution) supplied with the kit was added to each well, followed by gentle shaking. Absorbance at 450 nm was then measured with an absorbance microplate reader (Multiskan GO, Thermo Fisher Scientific Inc.). The concentration of FGF23 in plasma was calculated by making a calibration curve from the measurement result of recombinant FGF23 supplied with the kit.

5. Statistical analysis

In statistical analysis, Student's t-test or one-way analysis of variance was performed, and then significant differences were determined by the Tukey-Kramer test. The case in which the p-value is less than 0.05 was defined as being significant.

6. Results

Hereinafter, in the drawings, HP4W indicates mice 4 weeks after the start of the diet with high phosphorus content, and LP4W indicates mice 4 weeks after the start of the diet with low phosphorus content (control group). sOscar indicates mice to which the soluble human Oscar-Fc fusion protein was administered, and NS indicates mice to which physiological saline was administered in place of the soluble human Oscar-Fc fusion protein. WT (12W) indicates 12-week-old male wild-type mice fed a normal diet.

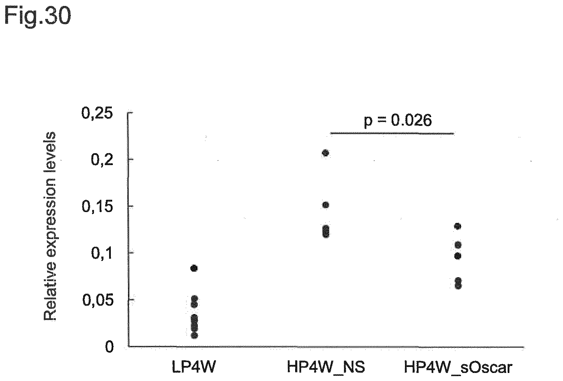

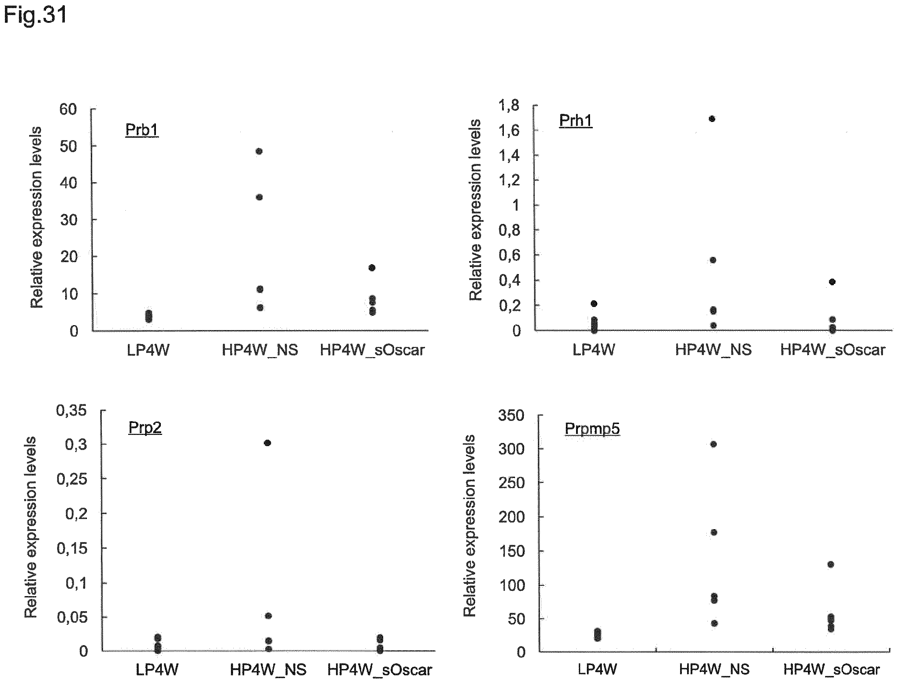

Fig. 30 shows qRT-PCR results of FGF23 in the skull. Fig. 31 shows qRT-PCR results of PRP genes in the parotid glands. FGF23 is a master regulator of inter-organ cross talk between the parathyroid glands, bones, and kidneys in kidney disease. The expression of FGF23 was induced by the diet with high phosphorus content in the skulls. However, the expression of FGF23 was suppressed by administration of the soluble human Oscar-Fc fusion protein (Fig. 30) (n=5 to 6). The expression of the Maea gene was normalized, and a significance test was performed. The results showed a significant difference (p=0.026) between HP to which sOscar was administered, and HP to which sOscar was not administered. The expression of PRP genes (Prb1, Prh1, Prp2, and Prpmp5) whose expression is induced by the diet with high phosphorus content was suppressed by administration of the soluble human Oscar-Fc fusion protein in the parotid glands (Fig. 31) (n=5 to 6). From the above, it was indicated that the expression of renal failure early markers FGF23 gene (the skull) and PRP genes (the parotid glands) were suppressed by administration of the soluble human Oscar-Fc fusion protein to the model mouse of phosphorus overload for 4 weeks.

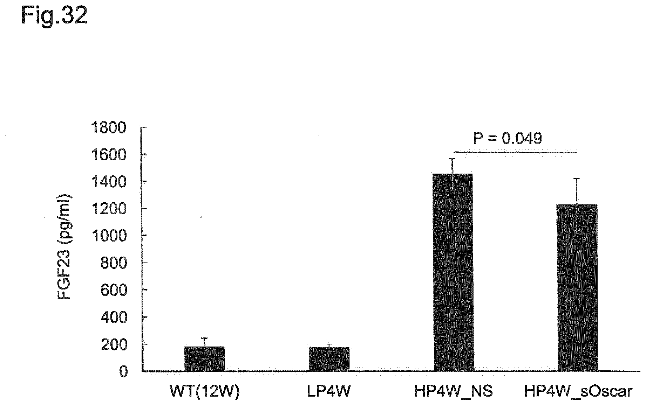

Fig. 32 shows ELISA measurement results of FGF23 in plasma. In HP, the concentration of FGF23 in plasma was higher than that in LP4W and WT (12W). The increase in the concentration of FGF23, which is a renal failure early marker in blood, was statistically significantly suppressed by administration of the soluble human Oscar-Fc fusion protein to HP (p=0.049).

As described later, qRT-PCR analysis in the bones of the 1-week model of Oscar gene mutant mice fed the diet with high phosphorus content showed that the increase in the expression of renal failure early marker FGF23 gene was significantly suppressed. In this experiment, administering the soluble human Oscar-Fc fusion protein, which has an effect of inhibiting ligand binding to Oscar, from the early stage of phosphorus overload suppressed the expression of the FGF23 gene, which is deeply involved in clinical states such as chronic kidney disease, in the bones; and further suppressed the increase in the concentration of FGF23 in plasma. This showed the potential of administering the soluble human Oscar-Fc fusion protein as a novel treatment method for improving a clinical state caused by an excess phosphorus state, such as kidney damage.

Reference Example 3: Measurement of creatinine in plasma

Mice (C57BL/6N, 8 weeks old, male) were fed a diet with high phosphorus content in which contains 2% inorganic phosphorus (TD.10662, OrientalBioService, Inc.) or a diet with low phosphorus content in which contains 0.35% inorganic phosphorus (TD.10662 modified type, OrientalBioService, Inc.) as a special phosphorus diet for 4 weeks. Thereafter, plasma samples (LP4W, HP4W) were collected and cryopreserved. A plasma sample (WT (12W)) was collected from 12-week-old male mice (C57BL/6N) and cryopreserved. 8-week-old male mice (C57BL/6N) were subjected to unilateral nephrectomy, and after being adapted for 4 weeks, the mice were fed a diet with high phosphorus content for 4 weeks. Thereafter, a plasma sample (UNx/HP4W) was collected and cryopreserved. Creatinine values in plasma were measured using 100 μl of each sample by an enzymatic method.

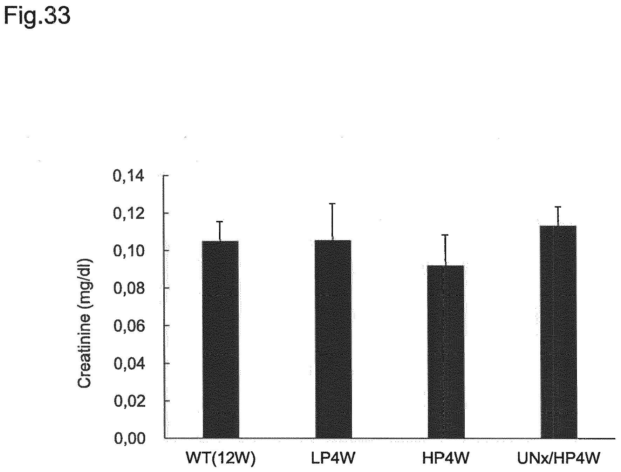

Fig. 33 shows a creatinine value in plasma in each model of phosphorus overload. In the HP4W model and the UNx/HP4W model, the amount of creatinine, which is a blood marker of renal failure, did not increase. Thus, it was believed that the soluble human Oscar-Fc fusion protein can be used for suppressing a decline in kidney function. It was also believed that the kidney function prediction markers shown in Figs. 22 and 23 can detect a decline in kidney function earlier than the amount of creatinine in blood increases. Therefore, it was believed that a decline in kidney function can be suppressed in an early stage by administering the soluble human Oscar-Fc fusion protein at a stage when the amount of creatinine has not yet increased, and when the kidney function prediction markers shown in Figs. 22 and 23 are increased or decreased.

Reference Example 4: Expression of fibrinogen in kidneys

To evaluate the effect of the soluble Oscar-Fc fusion protein on kidney function, the expression of a kidney function marker was confirmed in the kidneys of HP4W_NS, HP4W_sOscar, and LP4W of Reference Example 2.

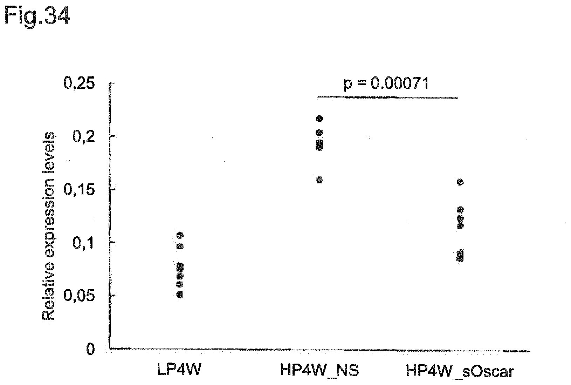

In the kidneys, the expression of the Fgg gene, which is a fibrinogen gene whose expression is induced by a diet with high phosphorus content, was significantly suppressed by administration of the soluble Oscar-Fc fusion protein (Fig. 34) (p=0.00071; n=5 to 6). Fibrinogen genes have been reported as kidney function markers and therapeutic targets. This suggested that at least one disease selected from the group consisting of declined kidney function, chronic kidney disease, and renal failure, which are associated with a high-phosphorus state, can be prevented or treated by administering the soluble Oscar-Fc fusion protein.

Description of Reference Numerals

1,2 Screening device
3 Input unit
4 Display unit
5a Device
5b Device
5c Device
6,7 Prediction device
11 First measurement value obtaining unit
12 Second measurement value obtaining unit
13 Measurement value comparison unit
14 Candidate substance determination unit

21 First evaluation result obtaining unit
22 Second evaluation result obtaining unit
23 Evaluation result comparison unit
24 Candidate substance determination unit
52 Reaction/electrophoresis unit
53 Detection unit
61 Subject data obtaining unit
62 Pattern similarity calculation unit
63 Prediction unit
71 Stage information obtaining unit
72 Stage information checking unit
73 Pattern extraction unit
74 Prediction unit
100,110,120,130 System
101 CPU
102 Memory
103 Storage unit
104 Bus
105 Interface unit
109 Storage medium

**Claims**

**1.** A device for screening a candidate substance for an active ingredient to prevent or treat at least one disease selected from the group consisting of declined kidney function, chronic kidney disease, and renal failure, the device comprising the following computation means:

first measurement value obtaining means for obtaining a measurement value of a kidney function prediction marker protein and/or a measurement value of mRNA of the protein in a specimen collected from a subject (excluding humans), test tissue, or test cell treated with a test substance (a test-substance-treated specimen); and
means for determining that the test substance is a candidate substance for the active ingredient based on the measurement value(s) obtained by the first measurement value obtaining means.

**2.** The device according to claim 1, further comprising:

second measurement value obtaining means for obtaining a measurement value of the kidney function prediction marker protein and/or a measurement value of mRNA of the protein in a specimen collected from a subject (excluding humans), test tissue, or test cell that is not treated with the test substance (an untreated specimen); and means for comparing the measurement value(s) of the test-substance-treated specimen with the measurement value(s) of the untreated specimen,
wherein the determination means determines that the test substance is a candidate substance for the active ingredient based on the comparison result obtained by the measurement value comparison means.

**3.** The device according to claim 1 or 2, wherein the kidney function prediction marker protein is at least one member selected from the group consisting of Aplnr, Spp1, Dnase1, Slco1a1, Anpep, Slc7a8, Oscar, proline-rich proteins, defensins, fibrinogens, and Hamp/Hepcidin.

**4.** A screening program that, when executed by a computer, causes the computer to carry out the following processing to screen a candidate substance for an active ingredient to prevent or treat at least one disease selected from the group consisting of declined kidney function, chronic kidney disease, and renal failure:

first measurement value obtaining processing of obtaining a measurement value of a kidney function prediction marker protein and/or a measurement value of mRNA of the protein in a specimen collected from a subject (excluding humans), test tissue, or test cell treated with a test substance (a test-substance-treated specimen); and
processing of determining that the test substance is a candidate substance for the active ingredient based on the measurement value(s) obtained by the first measurement value obtaining processing.

**5.** The screening program according to claim 4, wherein the program further causes the computer to carry out second measurement value obtaining processing of obtaining a measurement value of the kidney function prediction marker protein and/or a measurement value of mRNA of the protein in a specimen collected from a subject (excluding humans), test tissue, or test cell that is not treated with the test substance (an untreated specimen); and processing of comparing the measurement value(s) of the test-substance-treated specimen with the measurement value(s) of the untreated specimen, and
in the determination processing, it is determined that the test substance is a candidate substance for the active ingredient based on the comparison result obtained by the measurement value comparison processing.

**6.** The screening program according to claim 4 or 5,
wherein the kidney function prediction marker protein is at least one member selected from the group consisting of Aplnr, Spp1, Dnase1, Slco1a1, Anpep, Slc7a8, Oscar, proline-rich proteins, defensins, fibrinogens, and Hamp/Hepcidin.

**7.** A method for screening a candidate substance for an active ingredient to prevent or treat at least one disease selected from the group consisting of declined kidney function, chronic kidney disease, and renal failure, the method comprising the steps of:

(I) obtaining a measurement value of a kidney function prediction marker protein and/or a measurement value of mRNA of the protein in a specimen collected from a subject (excluding humans), test tissue, or test cell treated with a test substance (a test-substance-treated specimen); and
(II) determining that the test substance is a candidate substance for the active ingredient based on the measurement value(s) obtained in step (I).

**8.** The method according to claim 7, further comprising, between steps (I) and (II), the step of comparing the measurement value(s) of the test-substance-treated specimen obtained in step (I) with a measurement value of a corresponding kidney function prediction marker protein and/or a measurement value of mRNA of the protein in a specimen collected from a subject (excluding humans), test tissue, or test cell that is not treated with the test substance (an untreated specimen).

**9.** The method according to claim 7 or 8, comprising, before step (I), the steps of:

(i) treating the subject (excluding humans), test tissue, or test cell with the test substance;
(ii) collecting the specimen from the subject, test tissue, or test cell treated with the test substance in step (i); and
(iii) collecting the protein and/or the mRNA from the specimen obtained in step (ii).

**10.** The method according to any one of claims 7 to 9, wherein the kidney function prediction marker protein is at least one member selected from the group consisting of Aplnr, Spp1, Dnase1, Slco1a1, Anpep, Slc7a8, Oscar, proline-rich proteins, defensins, fibrinogens, and Hamp/Hepcidin.

**11.** A method for screening a candidate substance for an active ingredient to prevent or treat at least one disease selected from the group consisting of declined kidney function, chronic kidney disease, and renal failure, the method comprising the steps of:

(A) detecting a kidney function prediction marker protein and/or mRNA of the protein in a specimen collected from a subject (excluding humans), test tissue, or test cell treated with a test substance (a test-substance-treated specimen); and
(B) determining that the test substance is a candidate substance for the active ingredient based on the result obtained in step (A).

**12.** The method according to claim 11, further comprising, between steps (A) and (B), the step of comparing the detection result of the test-substance-treated specimen obtained in step (A) with a detection result of a corresponding kidney function prediction marker protein and/or mRNA of the protein in a specimen collected from a subject (excluding humans), test tissue, or test cell that is not treated with the test substance (an untreated specimen).

**13.** The method according to claim 11 or 12, wherein the kidney function prediction marker protein is at least one member selected from the group consisting of Aplnr, Spp1, Dnase1, Slco1a1, Anpep, Slc7a8, Oscar, proline-rich proteins, defensins, fibrinogens, and Hamp/Hepcidin.

**14.** A device for screening a candidate substance for an active ingredient to prevent or treat at least one disease selected from the group consisting of declined kidney function, chronic kidney disease, and renal failure, the device comprising the following computation means:

first evaluation result obtaining means for obtaining an evaluation result of function of a kidney function prediction marker protein in a specimen collected from a subject (excluding humans), test tissue, or test cell treated with a test substance (a test-substance-treated specimen); and
means for determining that the test substance is a candidate substance for the active ingredient based on the evaluation result obtained by the first evaluation result obtaining means.

**15.** The device according to claim 14, further comprising:

second evaluation result obtaining means for obtaining an evaluation result of function of the kidney function prediction marker protein in a specimen collected from a subject (excluding humans), test tissue, or test cell that is not treated with the test substance (an untreated specimen); and
means for comparing the evaluation result of the test-substance-treated specimen with the evaluation result of the untreated specimen,
wherein the determination means determines that the test substance is a candidate substance for the active ingredient based on the comparison result obtained by the evaluation result comparison means.

**16.** The device according to claim 14 or 15, wherein the kidney function prediction marker protein is at least one member selected from the group consisting of Aplnr, Spp1, Dnase1, Slco1a1, Anpep, Slc7a8, Oscar, proline-rich proteins, defensins, fibrinogens, and Hamp/Hepcidin.

**17.** A screening program that, when executed by a computer, causes the computer to carry out the following processing to screen a candidate substance for an active ingredient to prevent or treat at least one disease selected from the group consisting of declined kidney function, chronic kidney disease, and renal failure:

first evaluation result obtaining processing of obtaining an evaluation result of function of a kidney function prediction marker protein in a specimen collected from a subject (excluding humans), test tissue, or test cell treated with a test substance (a test-substance-treated specimen); and
processing of determining that the test substance is a candidate substance for the active ingredient based on the evaluation result obtained by the first evaluation result obtaining processing.

**18.** The screening program according to claim 17, wherein the program further causes the computer to carry out second evaluation result obtaining processing of obtaining an evaluation result of function of the kidney function prediction marker protein in a specimen collected from a subject (excluding humans), test tissue, or test cell that is not treated with the test substance (an untreated specimen); and processing of comparing the evaluation result of the test-substance-treated specimen with the evaluation result of the untreated specimen, and
in the determination processing, it is determined that the test substance is a candidate substance for the active ingredient based on the comparison result obtained by the evaluation result comparison processing.

**19.** The screening program according to claim 17 or 18, wherein the kidney function prediction marker protein is at least one member selected from the group consisting of Aplnr, Spp1, Dnase1, Slco1a1, Anpep, Slc7a8, Oscar, proline-rich proteins, defensins, fibrinogens, and Hamp/Hepcidin.

**20.** A method for screening a candidate substance for an active ingredient to prevent or treat at least one disease selected from the group consisting of declined kidney function, chronic kidney disease, and renal failure, the method comprising the steps of:

(I) evaluating function of a kidney function prediction marker protein in a specimen collected from a subject (excluding humans), test tissue, or test cell treated with a test substance (a test-substance-treated specimen); and
(II) determining that the test substance is a candidate substance for the active ingredient based on the evaluation result obtained in step (I).

**21.** The method according to claim 20, further comprising, between steps (I) and (II), the step of comparing the evaluation result of the test-substance-treated specimen obtained in step (I) with an evaluation result of function of a corresponding kidney function prediction marker protein in a specimen collected from a subject (excluding humans), test tissue, or test cell that is not treated with the test substance (an untreated specimen).

**22.** The method according to claim 20 or 21, comprising, before step (I), the steps of:

(i) treating the subject (excluding humans), test tissue, or test cell with the test substance; and
(ii) collecting the specimen from the subject, test tissue, or test cell treated with the test substance in step (i).

**23.** The method according to any one of claims 20 to 22, wherein the kidney function prediction marker protein is at least one member selected from the group consisting of Aplnr, Spp1, Dnase1, Slco1a1, Anpep, Slc7a8, Oscar, proline-rich proteins, defensins, fibrinogens, and Hamp/Hepcidin.

**24.** A device for predicting the presence of at least one disease selected from the group consisting of declined kidney function, chronic kidney disease, and renal failure, and/or the stage of the disease in a subject, the device comprising the following computation means:

means for obtaining data of the subject regarding an inter-organ cross talk indicator in each of one or more organs other than the kidneys in the subject, the inter-organ cross talk indicator being derived from cells or tissue originating from each of the one or more organs;
means for calculating, by comparing the data of the subject obtained by the subject data obtaining means with standard data 1 in one or more organs corresponding to the one or more organs from which the data of the subject originates, similarity of patterns of the inter-organ cross talk indicators between the data of the subject and the standard data 1, wherein the standard data 1 is extracted from a group of standard data 1 predetermined for each of the at least one disease selected from the group consisting of declined kidney function, chronic kidney disease, and renal failure, or for each stage of the disease; and
means for predicting the presence of the at least one disease selected from the group consisting of declined kidney function, chronic kidney disease, and renal failure, and/or the stage of the disease by using, as a measure, the similarity of patterns of the inter-organ cross talk indicators calculated by the pattern similarity calculation means;
wherein the data of the subject is a pattern of the inter-organ cross talk indicator representing a relationship between an amount of the inter-organ cross talk indicator in the organ other than the kidneys of the subject and an amount of the corresponding inter-organ cross talk indicator in the same organ as the organ other than the kidneys in negative control(s) with no disease in the kidneys, and
the standard data 1 includes patterns of the inter-organ cross talk indicators, each of the patterns being derived from a predetermined relationship between an amount of the inter-organ cross talk indicator in the organ other than the kidneys in positive control(s) affected with the at least one disease selected from the group consisting of declined kidney function, chronic kidney disease, and renal failure, and an amount of the corresponding inter-organ cross talk indicator in the same organ as the organ other than the kidneys in negative control(s) without the at least one disease selected from the group consisting of declined kidney function, chronic kidney disease, and renal failure.

**25.** A prediction program that, when executed by a computer, causes the computer to carry out the following processing to predict the presence of at least one disease selected from the group consisting of declined kidney function, chronic kidney disease, and renal failure, and/or the stage of the disease in a subject:

processing of obtaining data of the subject regarding an inter-organ cross talk indicator in each of one or more organs other than the kidneys in the subject, the inter-organ cross talk indicator being derived from cells or tissue originating from each of the one or more organs;
processing of calculating, by comparing the data of the subject obtained by the subject data obtaining processing with standard data 1 in one or more organs corresponding to the one or more organs from which the data of the subject originates, similarity of patterns of the inter-organ cross talk indicators between the data of the subject and the standard data 1, wherein the standard data 1 is extracted from a group of standard data 1 predetermined for each of the at least one disease selected from the group consisting of declined kidney function, chronic kidney disease, and renal failure, or for each stage of the disease; and
processing of predicting the presence of the at least one disease selected from the group consisting of declined kidney function, chronic kidney disease, and renal failure and/or the stage of the disease by using, as a measure, the similarity of patterns of the inter-organ cross talk indicators calculated by the pattern similarity calculation processing;
wherein the data of the subject is a pattern of the inter-organ cross talk indicator representing a relationship between an amount of the inter-organ cross talk indicator in the organ other than the kidneys of the subject and an amount of the corresponding inter-organ cross talk indicator in the same organ as the organ other than the kidneys in negative control(s) without the at least one disease selected from the group consisting of declined kidney function, chronic kidney disease, and renal failure, and
the standard data 1 includes patterns of the inter-organ cross talk indicators, each of the patterns being derived from a predetermined relationship between an amount of the inter-organ cross talk indicator in the organ other than the kidneys in positive control(s) affected with the at least one disease selected from the group consisting of declined kidney function, chronic kidney disease, and renal failure, and an amount of the corresponding inter-organ cross talk indicator in the same organ as the organ other than the kidneys in negative control(s) with no disease in the kidneys.

**26.** A method for predicting the presence of at least one disease selected from the group consisting of declined kidney function, chronic kidney disease, and renal failure, and/or the stage of the disease in a subject, the method comprising the steps of:

(1) obtaining data of the subject regarding an inter-organ cross talk indicator in each of one or more organs other than the kidneys in the subject, the inter-organ cross talk indicator being derived from cells or tissue originating from each of the one or more organs;
(2) calculating, by comparing the data of the subject obtained in step (1) with standard data 1 in one or more organs corresponding to the one or more organs from which the data of the subject originates, similarity of patterns of the inter-organ cross talk indicators between the data of the subject and the standard data 1, wherein the standard data 1 is extracted from a group of standard data 1 predetermined for each of the at least one disease selected from the group consisting of declined kidney function, chronic kidney disease, and renal failure, or for each stage of the disease; and
(3) determining that the subject has a disease corresponding to the standard data 1 and/or is in a stage of a disease corresponding to the standard data 1 when it is determined from the similarity of patterns of the inter-organ cross talk indicators calculated in step (2) that both patterns are similar;

wherein the data of the subject is a pattern of the inter-organ cross talk indicator representing a relationship between an amount of the inter-organ cross talk indicator in the organ other than the kidneys of the subject and an amount of the corresponding inter-organ cross talk indicator in the same organ as the organ other than the kidneys in negative control(s) without the at least one disease selected from the group consisting of declined kidney function, chronic kidney disease, and renal failure, and
the standard data 1 includes patterns of the inter-organ cross talk indicators, each of the patterns being derived from a predetermined relationship between an amount of the inter-organ cross talk indicator in the organ other than the kidneys in positive control(s) affected with the at least one disease selected from the group consisting of declined kidney function, chronic kidney disease, and renal failure, and an amount of the corresponding inter-organ cross talk indicator in the same organ as the organ other than the kidneys in negative control(s) without the at least one disease selected from the group consisting of declined kidney function, chronic kidney disease, and renal failure.

**27.** A device for predicting the presence of a disease and/or the stage of the disease in each of one or more organs other than the kidneys in a subject affected with at least one disease selected from the group consisting of declined kidney function, chronic kidney disease, and renal failure, the device comprising the following computation means:

means for obtaining information about a stage of the at least one disease selected from the group consisting of declined kidney function, chronic kidney disease, and renal failure in the subject;
means for checking the information about the stage obtained by the stage information obtaining means against standard data 2;
means for extracting a pattern of an inter-organ cross talk indicator in each of one or more organs other than the kidneys in the subject based on the result obtained by the stage information checking means; and
means for predicting the presence of a disease and/or the stage of the disease in each of the one or more organs other than the kidneys by using, as a measure, the pattern of the inter-organ cross talk indicator obtained by the pattern extraction means;
wherein the standard data 2 includes patterns of the inter-organ cross talk indicators predetermined for each stage of the at least one disease selected from the group consisting of declined kidney function, chronic kidney disease, and renal failure, each of the patterns being derived from a predetermined relationship between an amount of the inter-organ cross talk indicator in the organ other than the kidneys in positive control(s) affected with the at least one disease selected from the group consisting of declined kidney function, chronic kidney disease, and renal failure, and an amount of the corresponding inter-organ cross talk indicator in the same organ as the organ other than the kidneys in negative control(s) without the at least one disease selected from the group consisting of declined kidney function, chronic kidney disease, and renal failure.

**28.** A prediction program that, when executed by a computer, causes the computer to carry out the following processing to predict the presence of a disease and/or the stage of the disease in each of one or more organs other than the kidneys in a subject affected with at least one disease selected from the group consisting of declined kidney function, chronic kidney disease, and renal failure:

processing of obtaining information about a stage of the at least one disease selected from the group consisting of declined kidney function, chronic kidney disease, and renal failure in the subject;
stage information comparison processing of checking the information about the stage obtained by the stage information obtaining processing against standard data 2;
processing of extracting a pattern of an inter-organ cross talk indicator in each of one or more organs other than the kidneys in the subject based on the result obtained by the stage information comparison processing; and
processing of predicting the presence of a disease and/or the stage of the disease in each of the one or more organs other than the kidneys by using, as a measure, the pattern of the inter-organ cross talk indicator obtained by the pattern extraction processing,
wherein the standard data 2 includes patterns of the inter-organ cross talk indicators predetermined for each stage of the at least one disease selected from the group consisting of declined kidney function, chronic kidney disease, and renal failure, each of the patterns being derived from a predetermined relationship between an amount of the inter-organ cross talk indicator in the organ other than the kidneys in positive control(s) affected with the at least one disease selected from the group consisting of declined kidney function, chronic kidney disease, and renal failure, and an amount of the corresponding inter-organ cross talk indicator in the same organ as the organ other than the kidneys in negative control(s) without the at least one disease selected from the group consisting of declined kidney function, chronic kidney disease, and renal failure.

**29.** A method for predicting the presence of a disease and/or the stage of the disease in each of one or more organs other than the kidneys in a subject affected with at least one disease selected from the group consisting of declined kidney function, chronic kidney disease, and renal failure, the method comprising the steps of:

(i) obtaining information about a stage of the at least one disease selected from the group consisting of declined kidney function, chronic kidney disease, and renal failure in the subject from a diagnostic result of the subject;
(ii) checking the information about the stage obtained in step (i) against standard data 2;
(iii) determining, from the standard data 2, standard data $\alpha$ at a stage of the at least one disease selected from the group consisting of declined kidney function, chronic kidney disease, and renal failure corresponding to the information about the stage, based on the checking result obtained in step (ii), and extracting, from the standard data $\alpha$, a pattern of an inter-organ cross talk indicator corresponding to the stage in the subject in each of one or more organs other than the kidneys in the subject;

(iv) checking the pattern of the inter-organ cross talk indicator extracted in step (iii) against known information about inter-organ cross talk indicators in diseases and/or stages of the diseases, and determining the presence of a disease and/or the stage of the disease in each of the one or more organs other than the kidneys corresponding to the pattern of the inter-organ cross talk indicator in each of the one or more organs other than the kidneys in the subject; and

(v) further determining that the disease in each of the one or more organs other than the kidneys determined in step (iv) is a disease from which the subject may be suffering, and/or determining that the stage of the disease in each of the one or more organs other than the kidneys determined in step (iv) is a stage of a disease from which the subject is suffering,

wherein the standard data 2 includes patterns of the inter-organ cross talk indicators predetermined for each stage of the at least one disease selected from the group consisting of declined kidney function, chronic kidney disease, and renal failure, each of the patterns being derived from a predetermined relationship between an amount of the inter-organ cross talk indicator in the organ other than the kidneys in positive control(s) affected with the at least one disease selected from the group consisting of declined kidney function, chronic kidney disease, and renal failure, and an amount of the corresponding inter-organ cross talk indicator in the same organ as the organ other than the kidneys in negative control(s) with no disease in the kidneys.

**30.** A method for generating standard data of patterns of inter-organ cross talk indicators for use in prediction of the presence of at least one disease selected from the group consisting of declined kidney function, chronic kidney disease, and renal failure, and/or the stage of the disease in a subject, the method comprising the steps of:

extracting an inter-organ cross talk indicator from cells or tissue collected from each of one or more organs other than the kidneys of positive control(s) of a gold standard for each stage of the at least one disease selected from the group consisting of declined kidney function, chronic kidney disease, and renal failure;

extracting the inter-organ cross talk indicator from cells or tissue collected from each of the one or more organs other than the kidneys of negative control(s) of a gold standard;

identifying and quantifying the inter-organ cross talk indicators;

determining patterns of the inter-organ cross talk indicators, each of the patterns being determined from a relationship between an amount of the inter-organ cross talk indicator in the organ other than the kidneys in the positive control(s) affected with the at least one disease selected from the group consisting of declined kidney function, chronic kidney disease, and renal failure, and an amount of the corresponding inter-organ cross talk indicator in the same organ as the organ other than the kidneys in the negative control(s) without the at least one disease selected from the group consisting of declined kidney function, chronic kidney disease, and renal failure; and

associating the patterns of the inter-organ cross talk indicators with the corresponding stages of the at least one disease selected from the group consisting of declined kidney function, chronic kidney disease, and renal failure.

**31.** A method for generating standard data of patterns of inter-organ cross talk indicators for use in prediction of the presence of a disease and/or the stage of the disease in each of one or more organs other than the kidneys in a subject affected with at least one disease selected from the group consisting of declined kidney function, chronic kidney disease, and renal failure, the method comprising the steps of:

extracting an inter-organ cross talk indicator from cells or tissue collected from each of one or more organs other than the kidneys of positive control(s) of a gold standard for each stage of the at least one disease selected from the group consisting of declined kidney function, chronic kidney disease, and renal failure;

extracting the inter-organ cross talk indicator from cells or tissue collected from each of the one or more organs other than the kidneys of negative control(s) of a gold standard;

identifying and quantifying the inter-organ cross talk indicators; and

determining patterns of the inter-organ cross talk indicators for each stage of the at least one disease selected from the group consisting of declined kidney function, chronic kidney disease, and renal failure, each of the patterns being determined from a relationship between an amount of the inter-organ cross talk indicator in the organ other than the kidneys in the positive control(s) affected with the at least one disease selected from the group consisting of declined kidney function, chronic kidney disease, and renal failure, and an amount of the corresponding inter-organ cross talk indicator in the same organ as the organ other than the kidneys in the negative control(s) without the at least one disease selected from the group consisting of declined kidney function, chronic kidney disease, and renal failure.

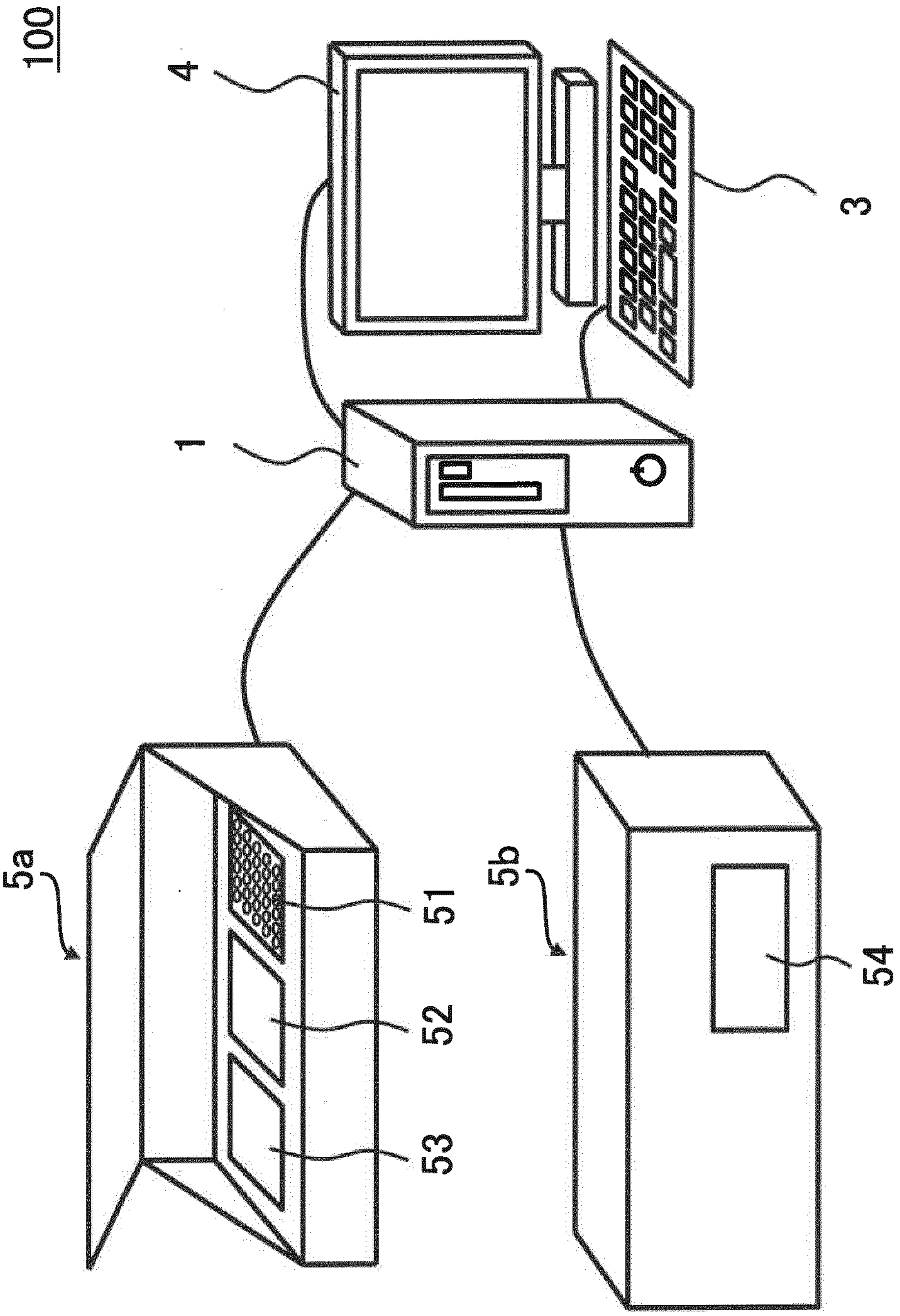


Fig.1

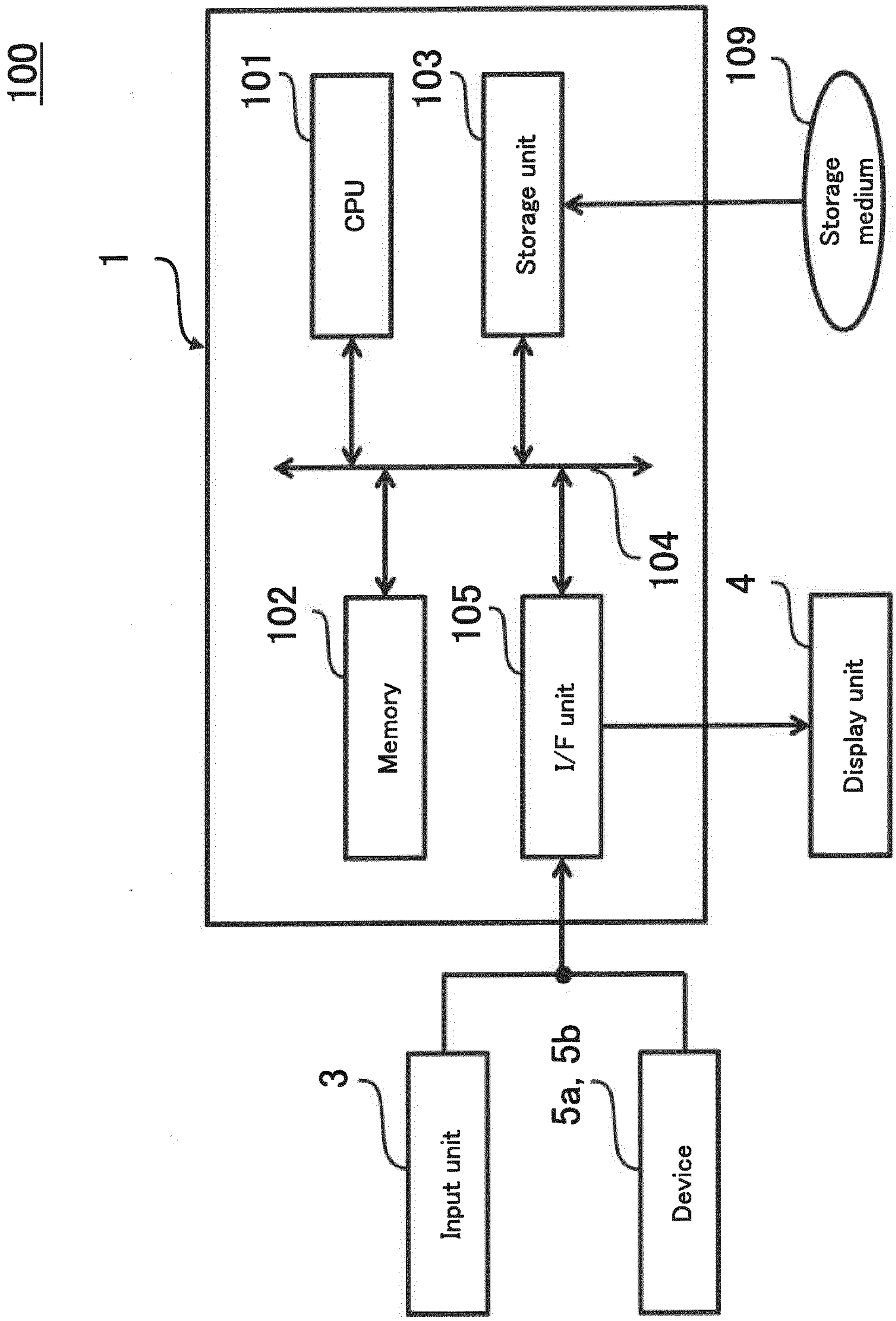
100
1
101
CPU
103
Storage unit
109
Storage medium
102
Memory
105
I/F unit
104
4
Display unit
3
Input unit
5a, 5b
Device

Fig.2

Fig.3

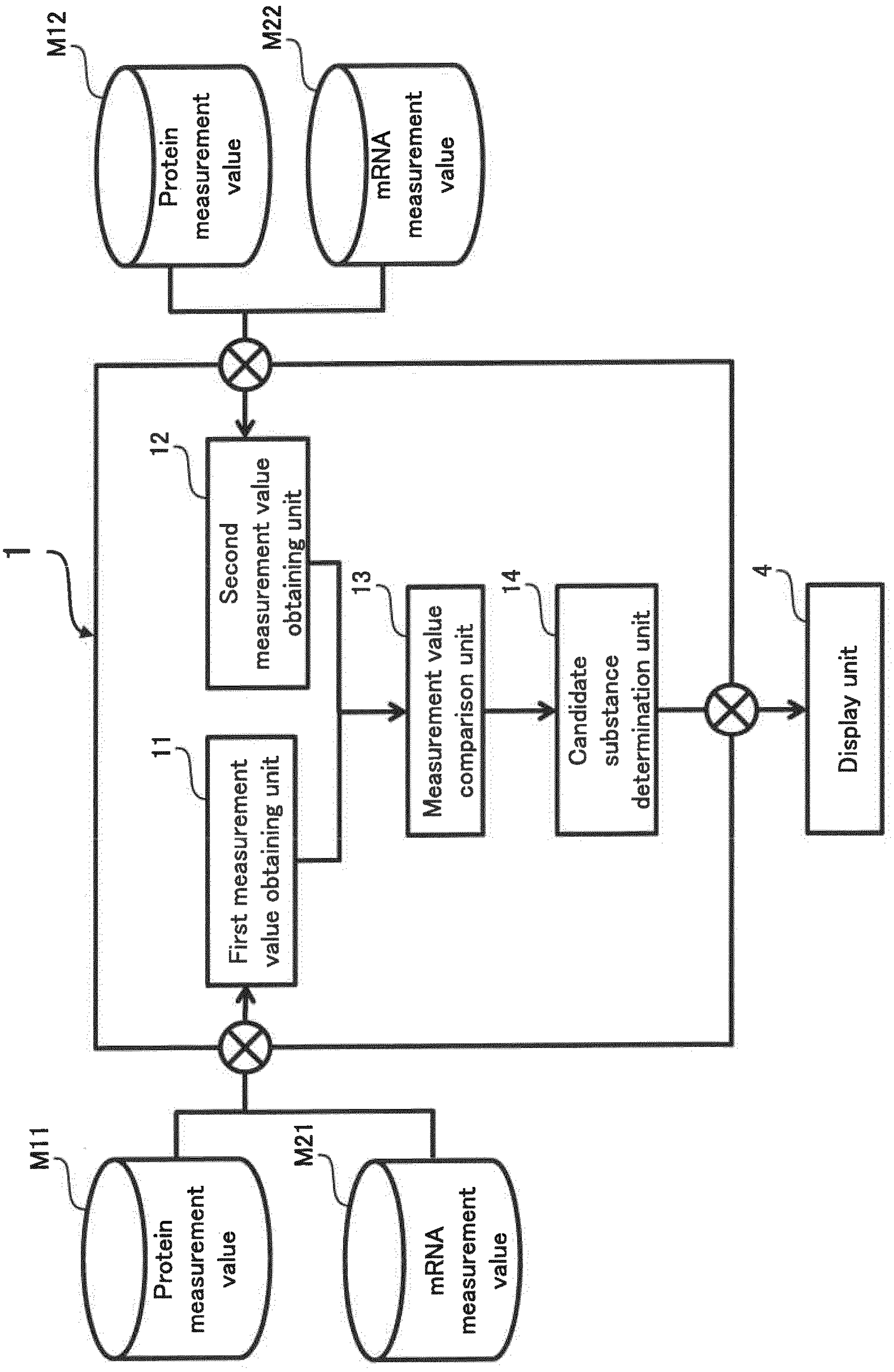
M12
Protein measurement value
M22
mRNA measurement value
1
12
Second measurement value obtaining unit
11
First measurement value obtaining unit
13
Measurement value comparison unit
14
Candidate substance determination unit
4
Display unit
M11
Protein measurement value
M21
mRNA measurement value

Fig.4

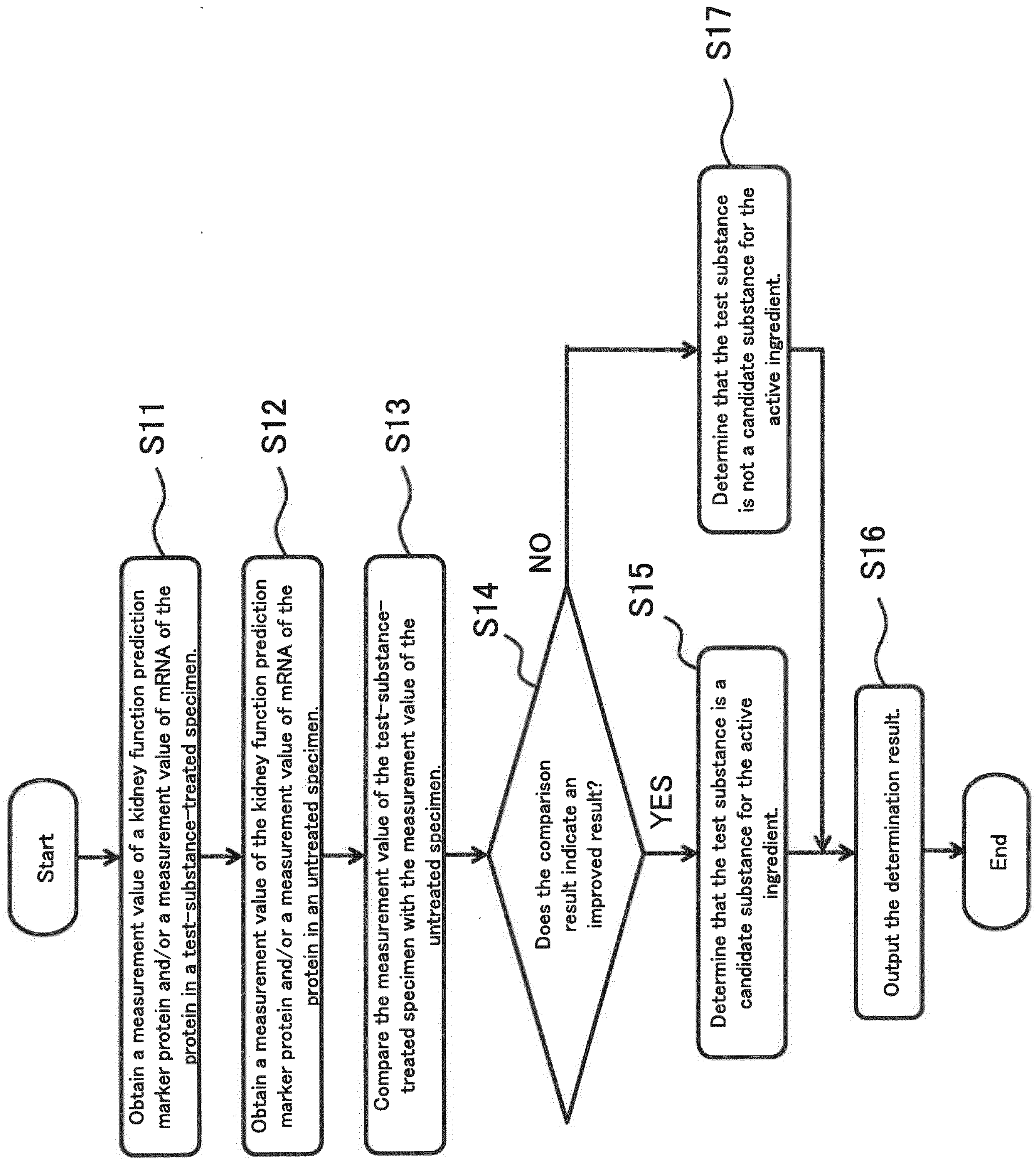
Start
S11
Obtain a measurement value of a kidney function prediction marker protein and/or a measurement value of mRNA of the protein in a test-substance-treated specimen.
S12
Obtain a measurement value of the kidney function prediction marker protein and/or a measurement value of mRNA of the protein in an untreated specimen.
S13
Compare the measurement value of the test-substance-treated specimen with the measurement value of the untreated specimen.
S14
Does the comparison result indicate an improved result?
NO
YES
S15
Determine that the test substance is a candidate substance for the active ingredient.
S17
Determine that the test substance is not a candidate substance for the active ingredient.
S16
Output the determination result.
End

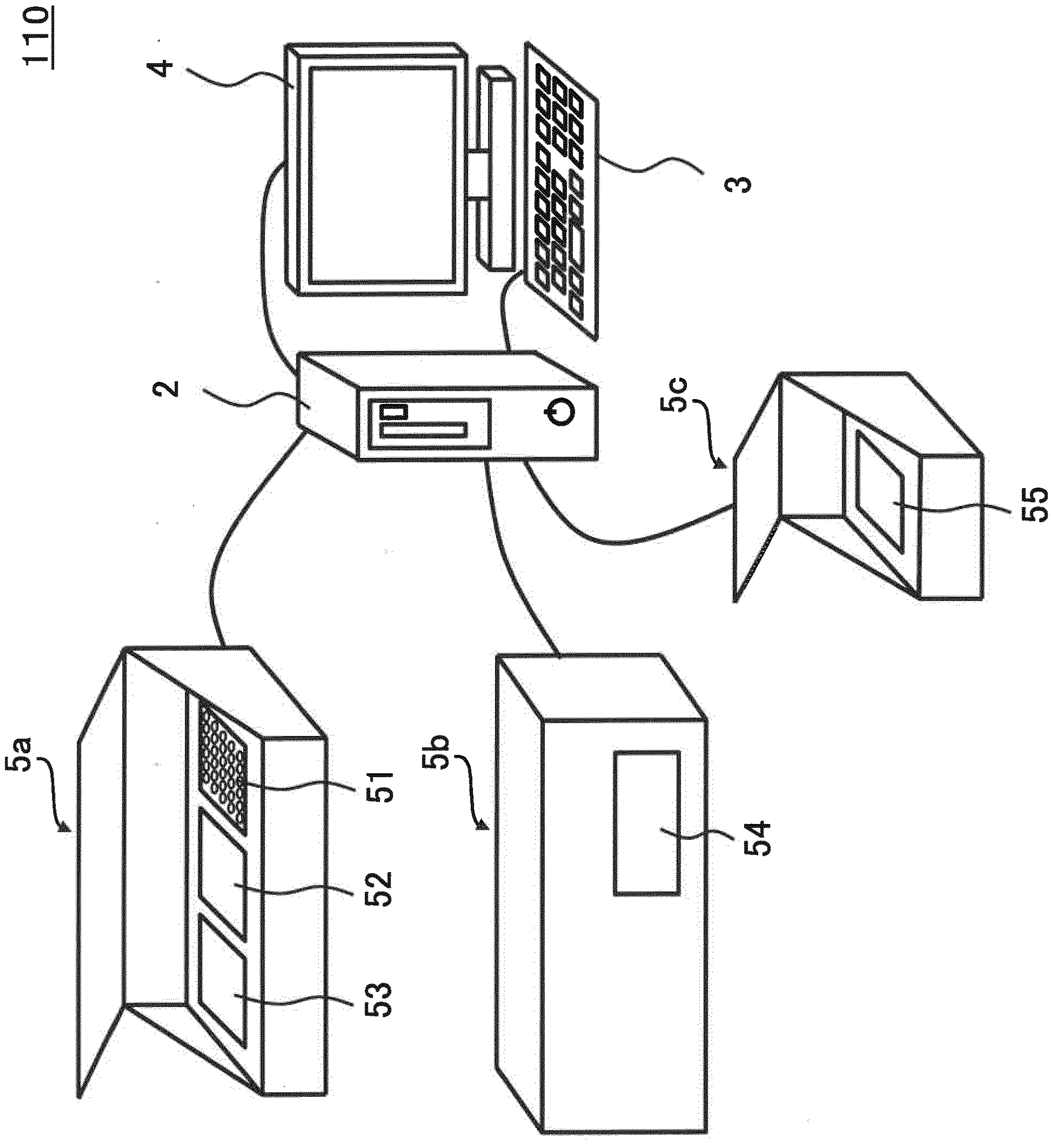


Fig.5

Fig.6

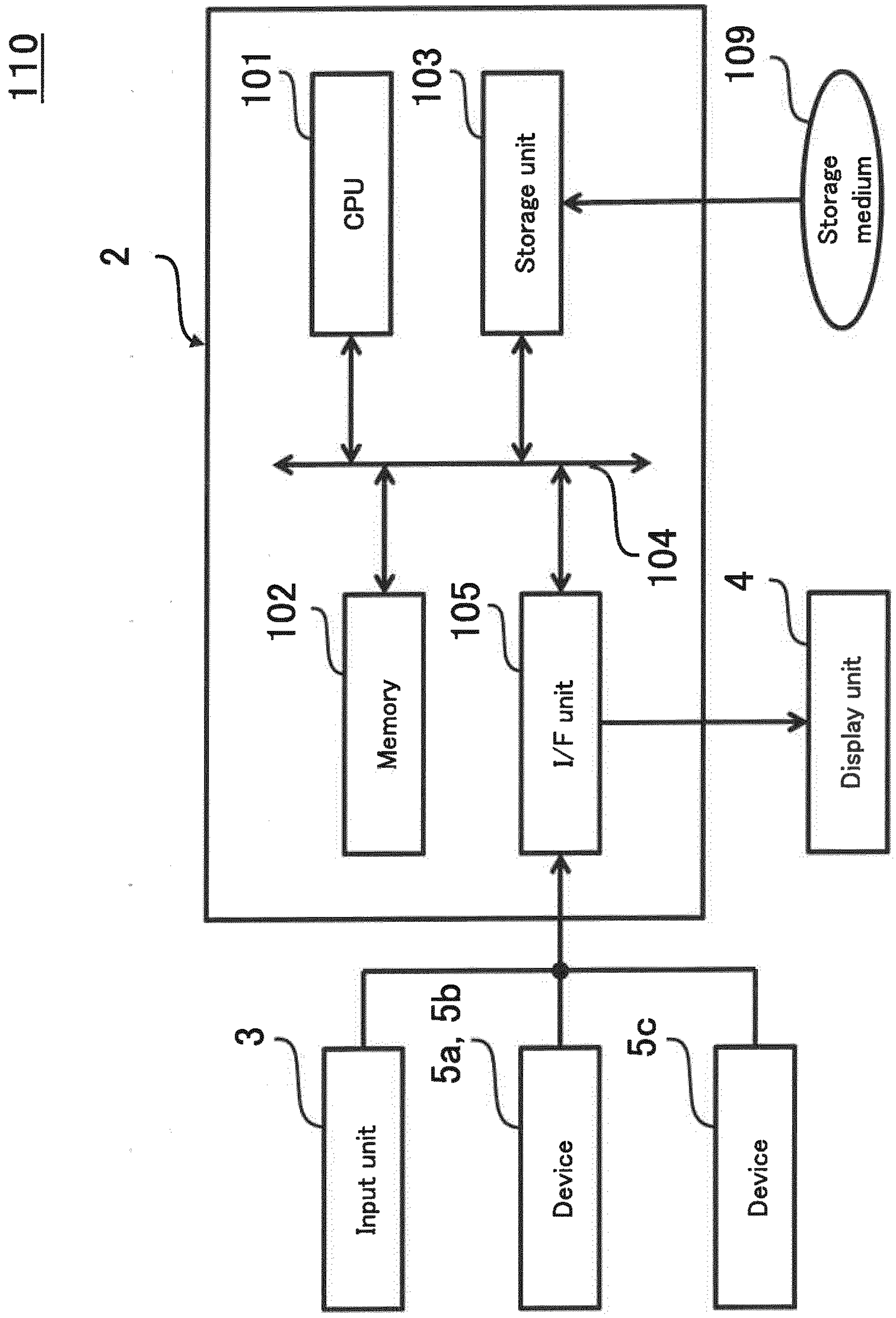
110
2
101
CPU
103
Storage unit
109
Storage medium
104
102
Memory
105
I/F unit
4
Display unit
3
Input unit
5a, 5b
Device
5c
Device

Fig.7

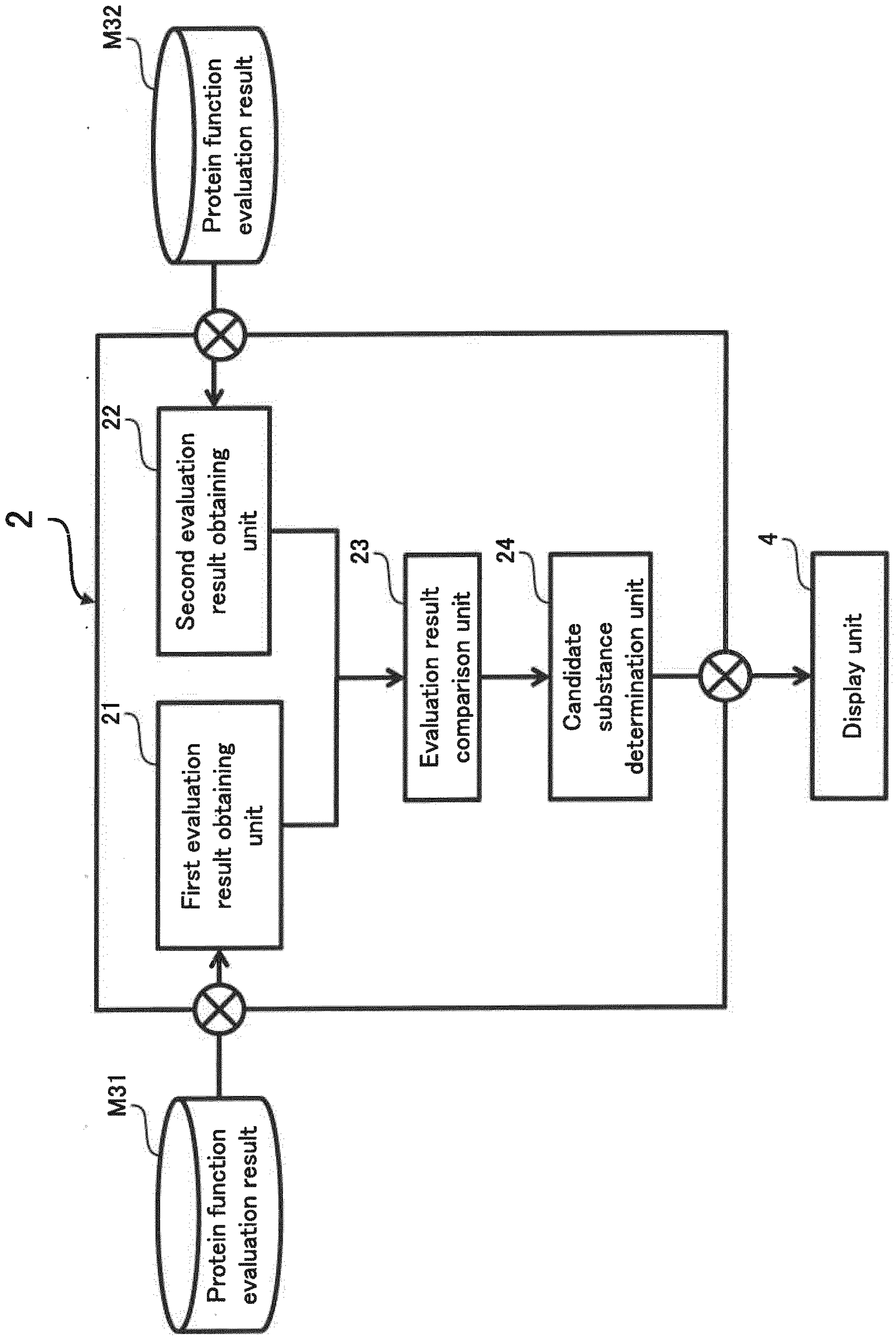
M32
Protein function evaluation result
2
22
Second evaluation result obtaining unit
21
First evaluation result obtaining unit
23
Evaluation result comparison unit
24
Candidate substance determination unit
4
Display unit
M31
Protein function evaluation result

Fig.8

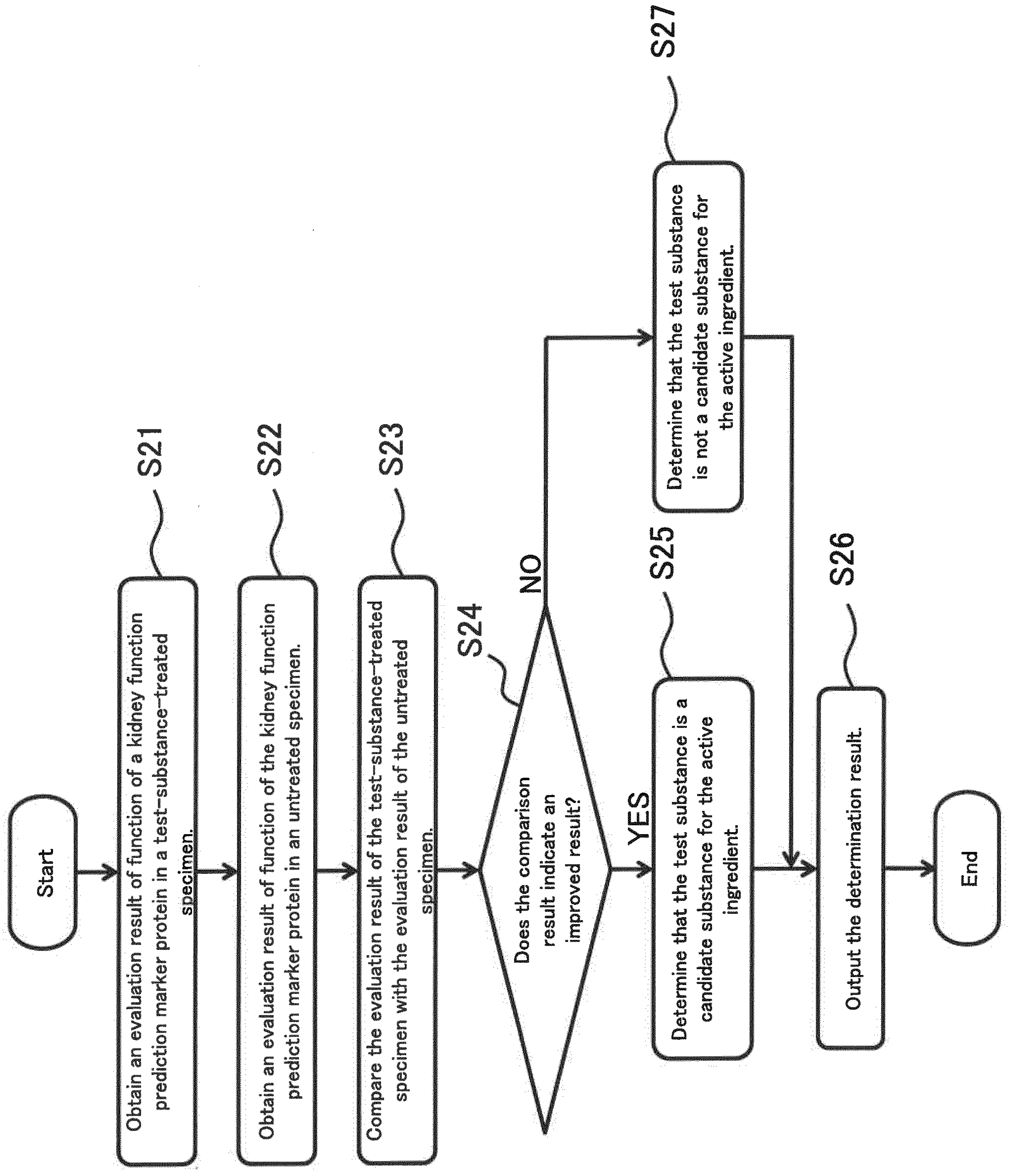
Start
S21
Obtain an evaluation result of function of a kidney function prediction marker protein in a test-substance-treated specimen.
S22
Obtain an evaluation result of function of the kidney function prediction marker protein in an untreated specimen.
S23
Compare the evaluation result of the test-substance-treated specimen with the evaluation result of the untreated specimen.
S24
Does the comparison result indicate an improved result?
YES
NO
S25
Determine that the test substance is a candidate substance for the active ingredient.
S27
Determine that the test substance is not a candidate substance for the active ingredient.
S26
Output the determination result.
End

Fig.9

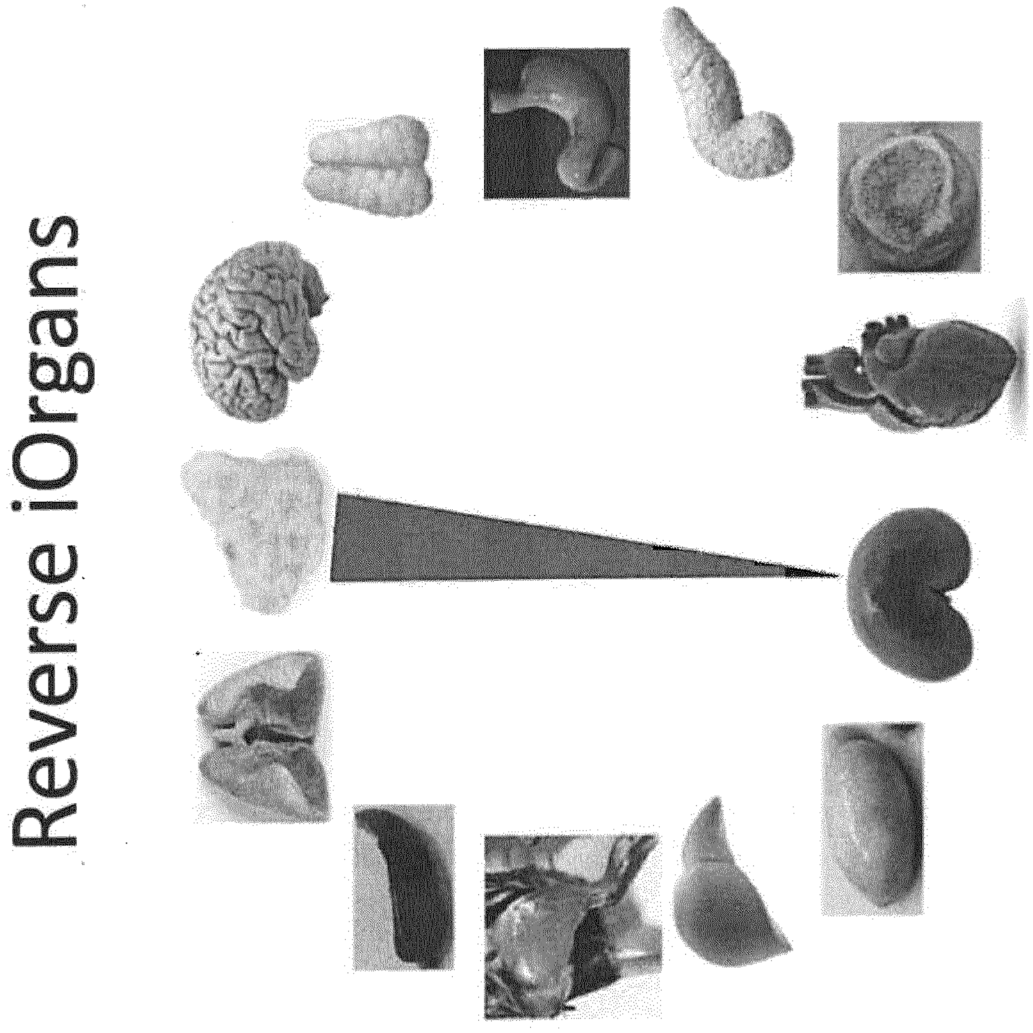

Fig.10

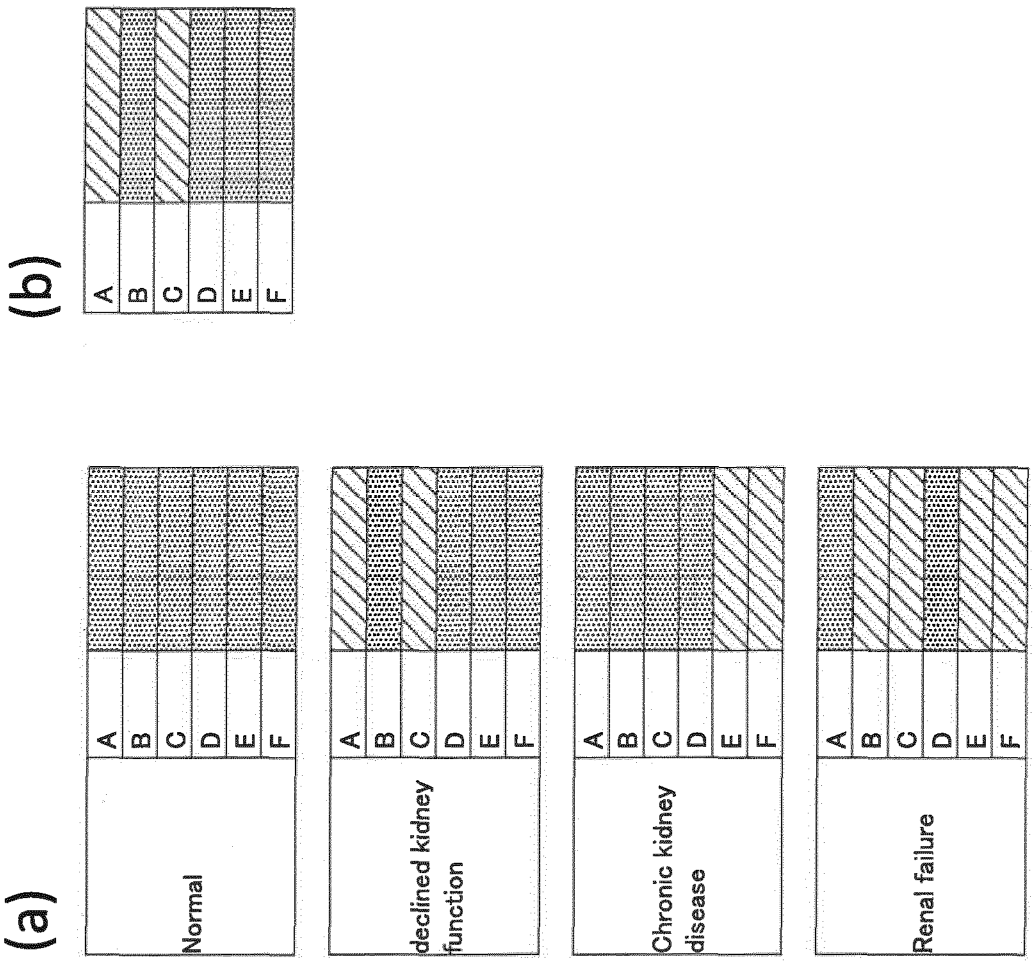
(a)
Normal
A
B
C
D
E
F
declined kidney function
A
B
C
D
E
F
Chronic kidney disease
A
B
C
D
E
F
Renal failure
A
B
C
D
E
F
(b)
A
B
C
D
E
F

Fig.11

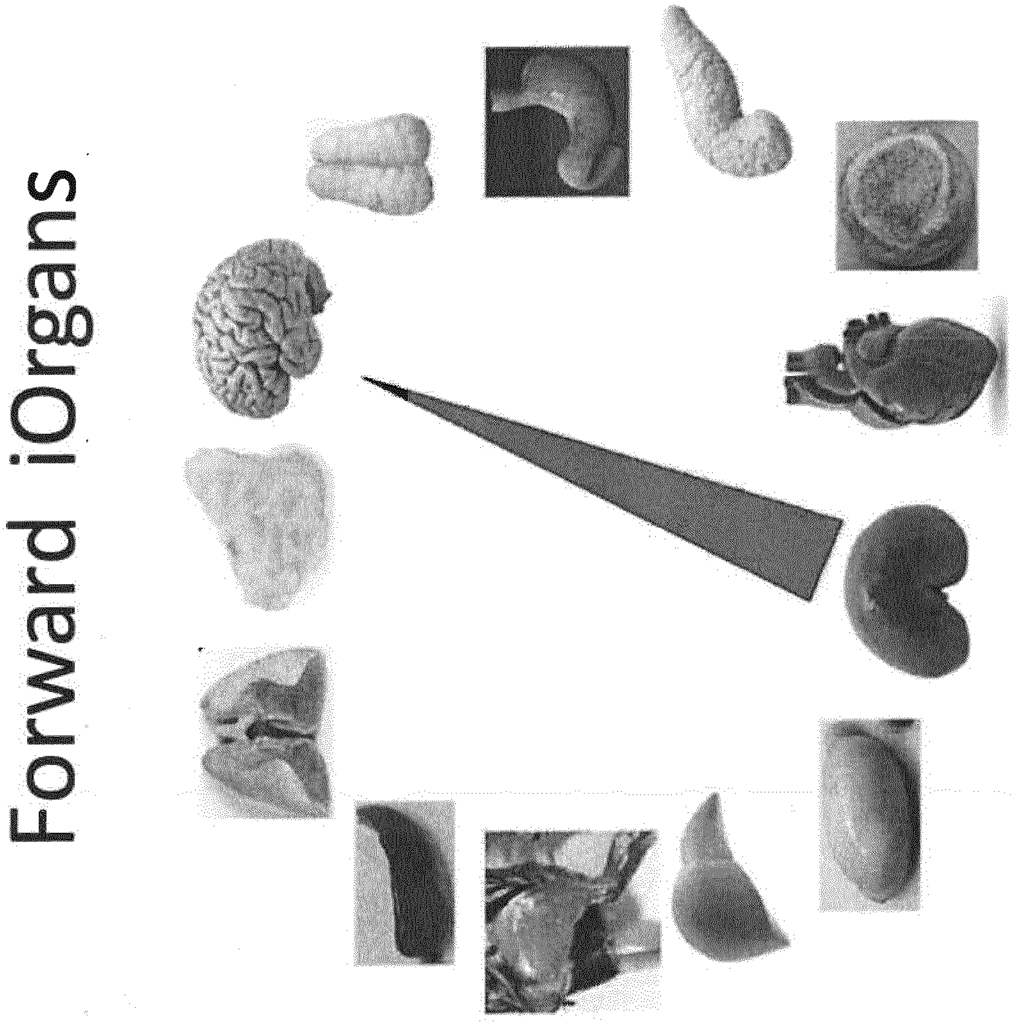

Fig.12

(a) (b)

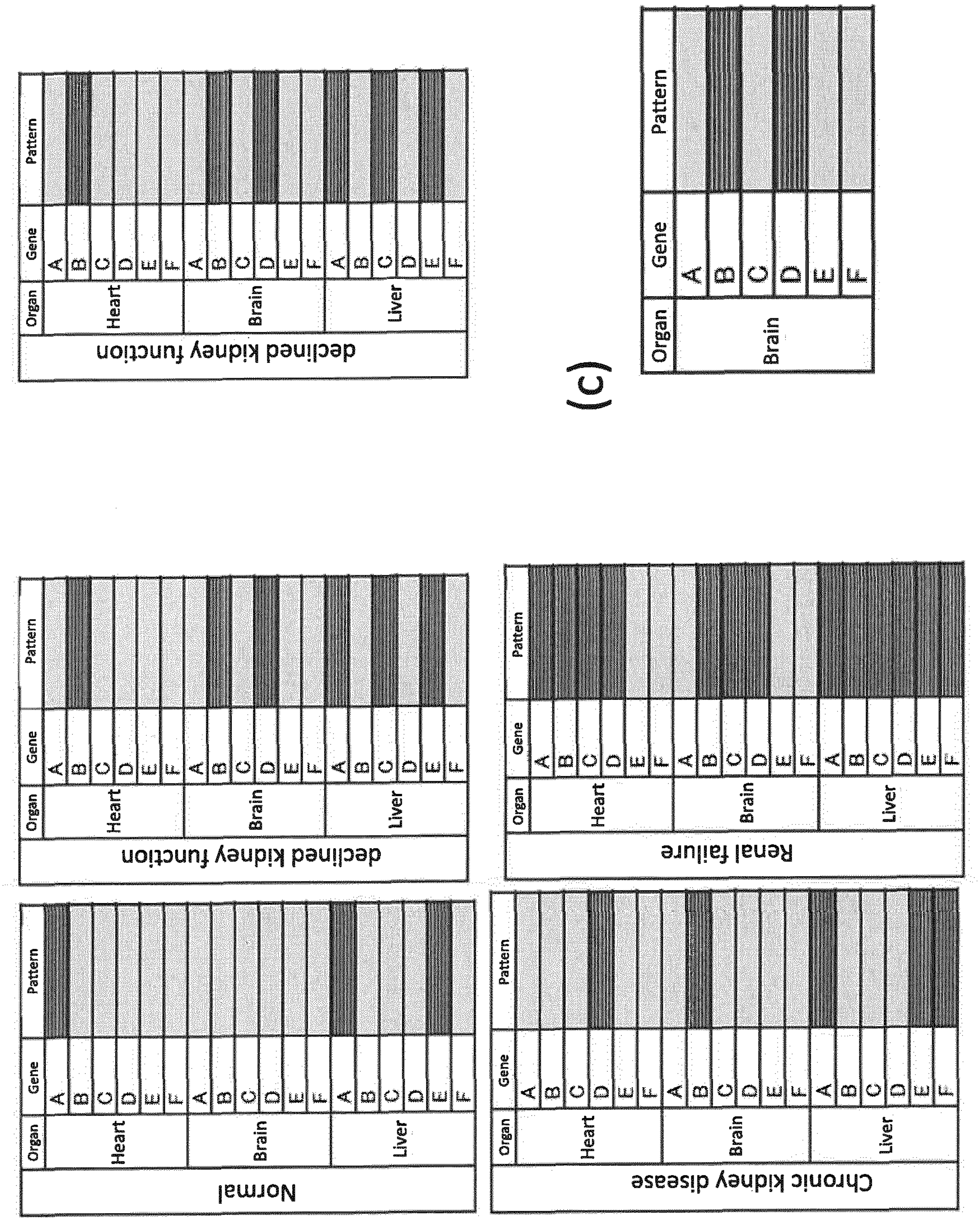
Normal
Organ
Gene
Pattern
Heart
Brain
Liver
A
B
C
D
E
F
declined kidney function
Chronic kidney disease
Renal failure
(c)

Fig.13

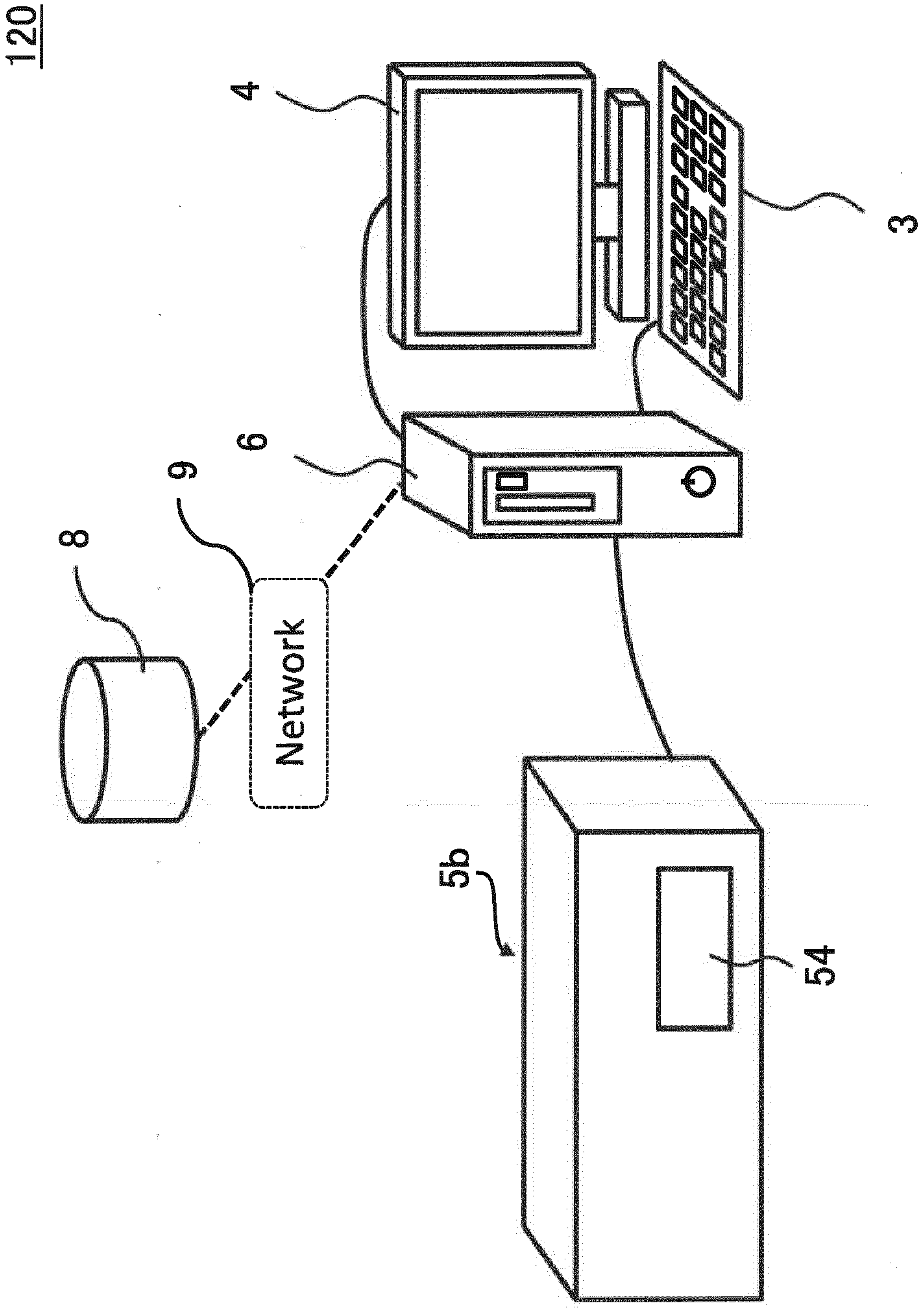

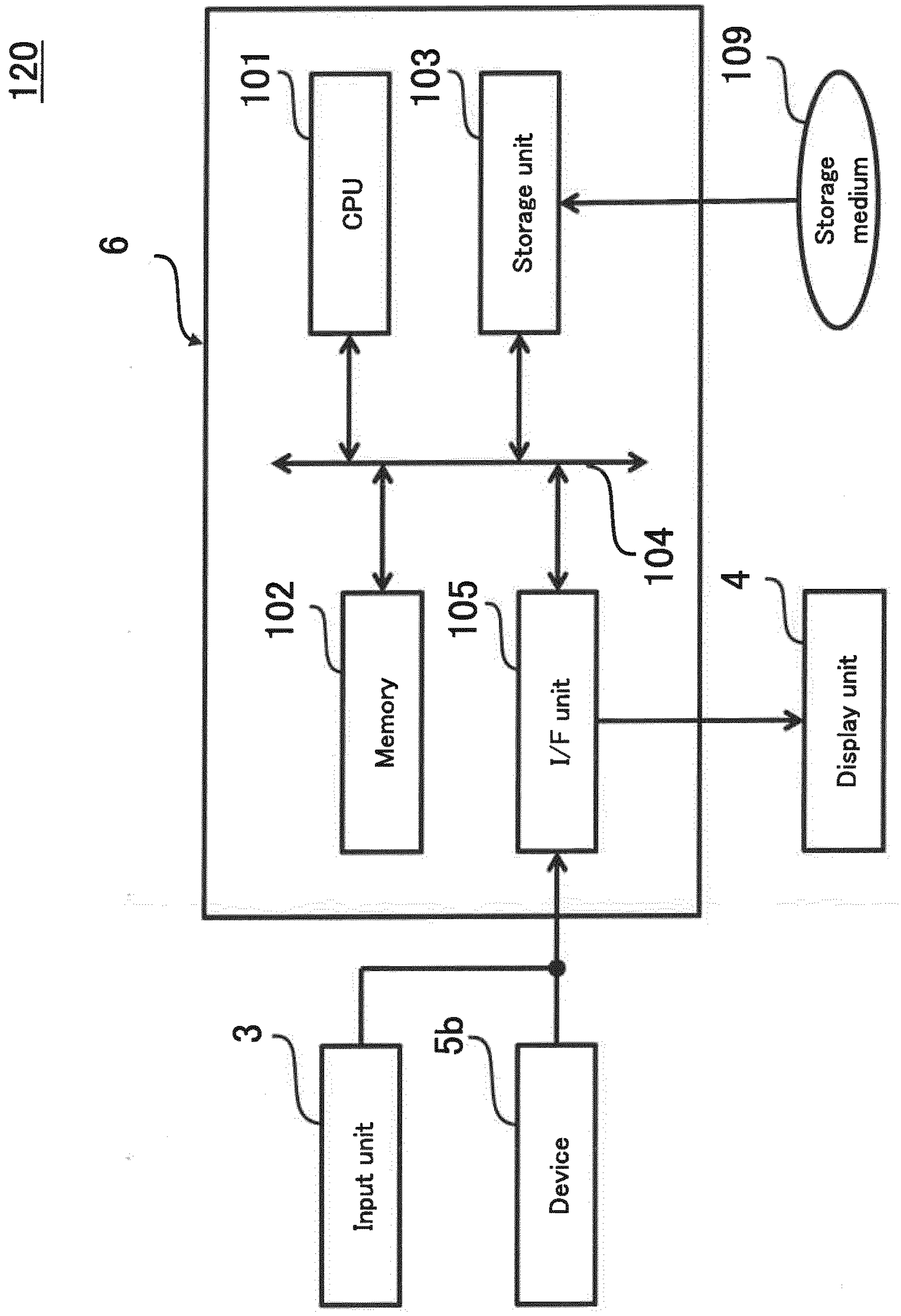
120
6
101
CPU
103
Storage unit
109
Storage medium
104
102
Memory
105
I/F unit
4
Display unit
3
Input unit
5b
Device

Fig.14

Fig.15

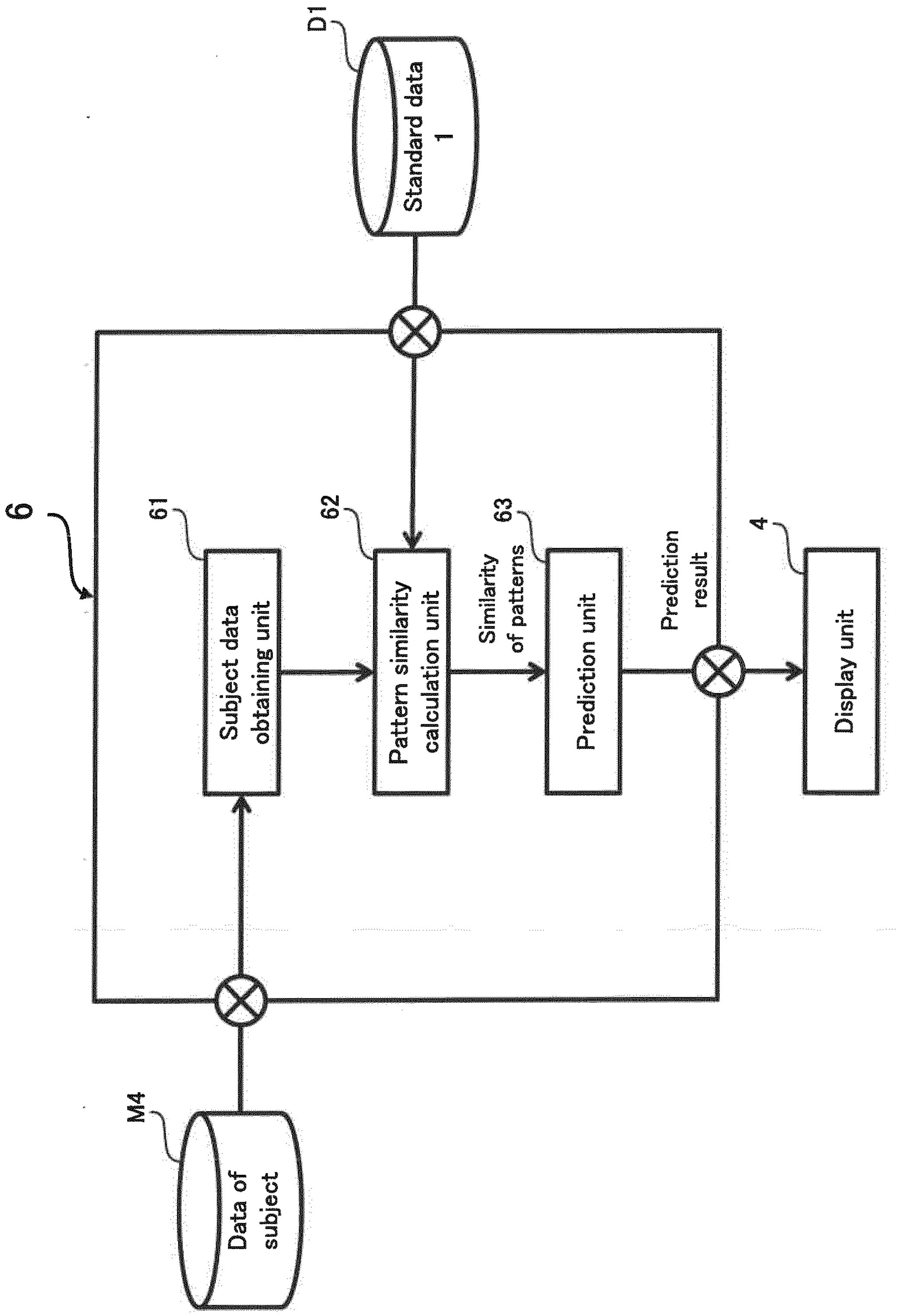
6
D1
Standard data 1
61
Subject data obtaining unit
62
Pattern similarity calculation unit
Similarity of patterns
63
Prediction unit
Prediction result
4
Display unit
M4
Data of subject

Fig.16

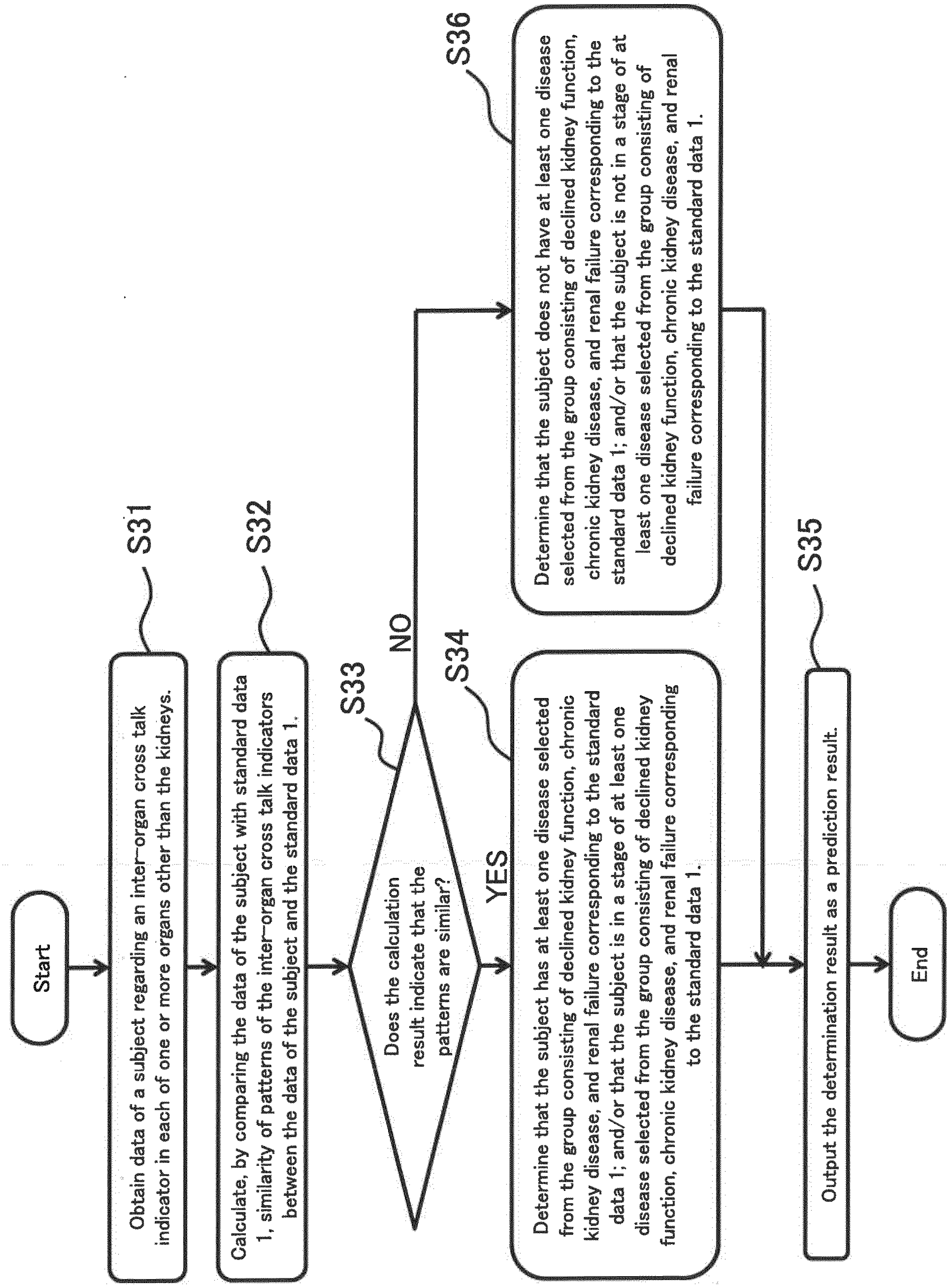
Start
Obtain data of a subject regarding an inter-organ cross talk indicator in each of one or more organs other than the kidneys.
S31
Calculate, by comparing the data of the subject with standard data 1, similarity of patterns of the inter-organ cross talk indicators between the data of the subject and the standard data 1.
S32
Does the calculation result indicate that the patterns are similar?
S33
YES
NO
Determine that the subject has at least one disease selected from the group consisting of declined kidney function, chronic kidney disease, and renal failure corresponding to the standard data 1; and/or that the subject is in a stage of at least one disease selected from the group consisting of declined kidney function, chronic kidney disease, and renal failure corresponding to the standard data 1.
S34
Determine that the subject does not have at least one disease selected from the group consisting of declined kidney function, chronic kidney disease, and renal failure corresponding to the standard data 1; and/or that the subject is not in a stage of at least one disease selected from the group consisting of declined kidney function, chronic kidney disease, and renal failure corresponding to the standard data 1.
S36
Output the determination result as a prediction result.
S35
End

Fig.17

130

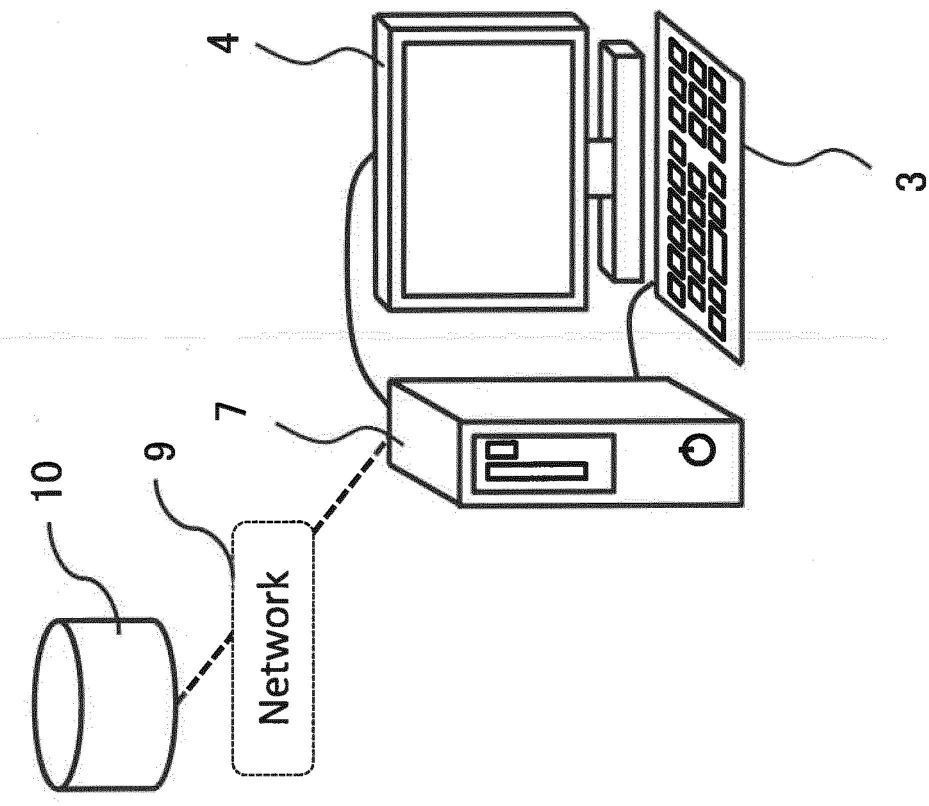

Fig.18

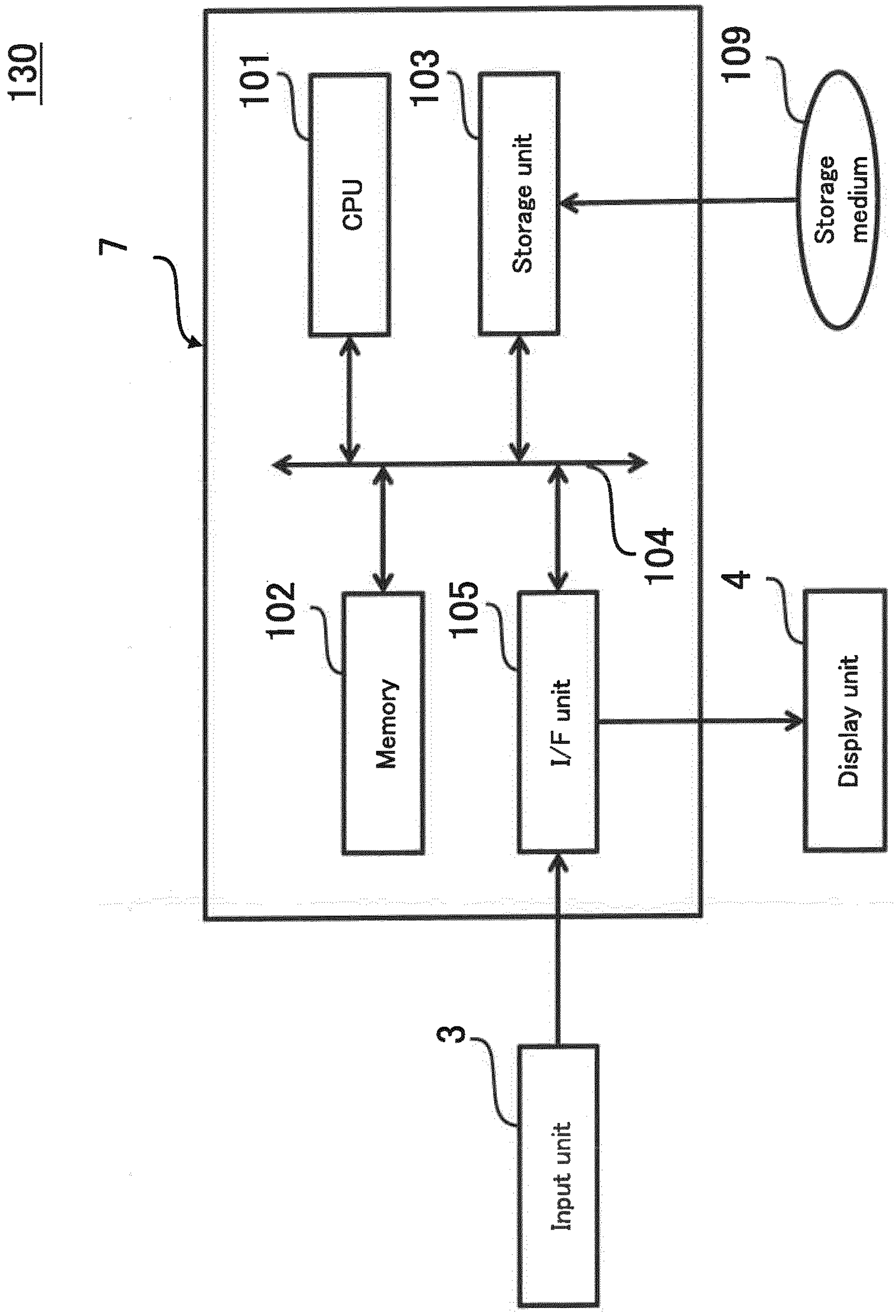
130
7
CPU
101
Storage unit
103
109
Storage medium
104
Memory
102
I/F unit
105
4
Display unit
3
Input unit

Fig.19

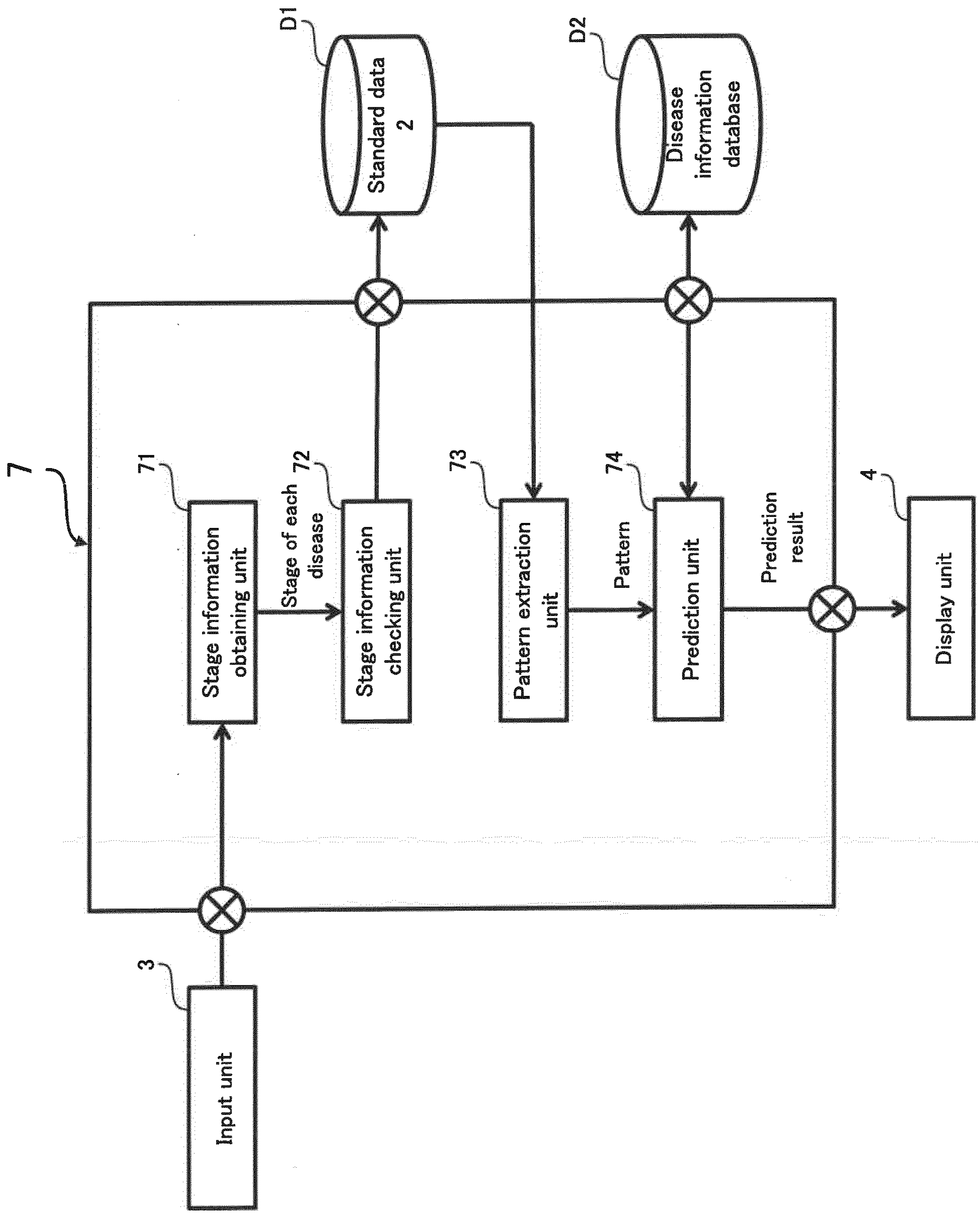
7
Input unit
3
Stage information obtaining unit
71
Stage of each disease
Stage information checking unit
72
Pattern extraction unit
73
Pattern
Prediction unit
74
Prediction result
Display unit
4
Standard data 2
D1
Disease information database
D2

Fig.20

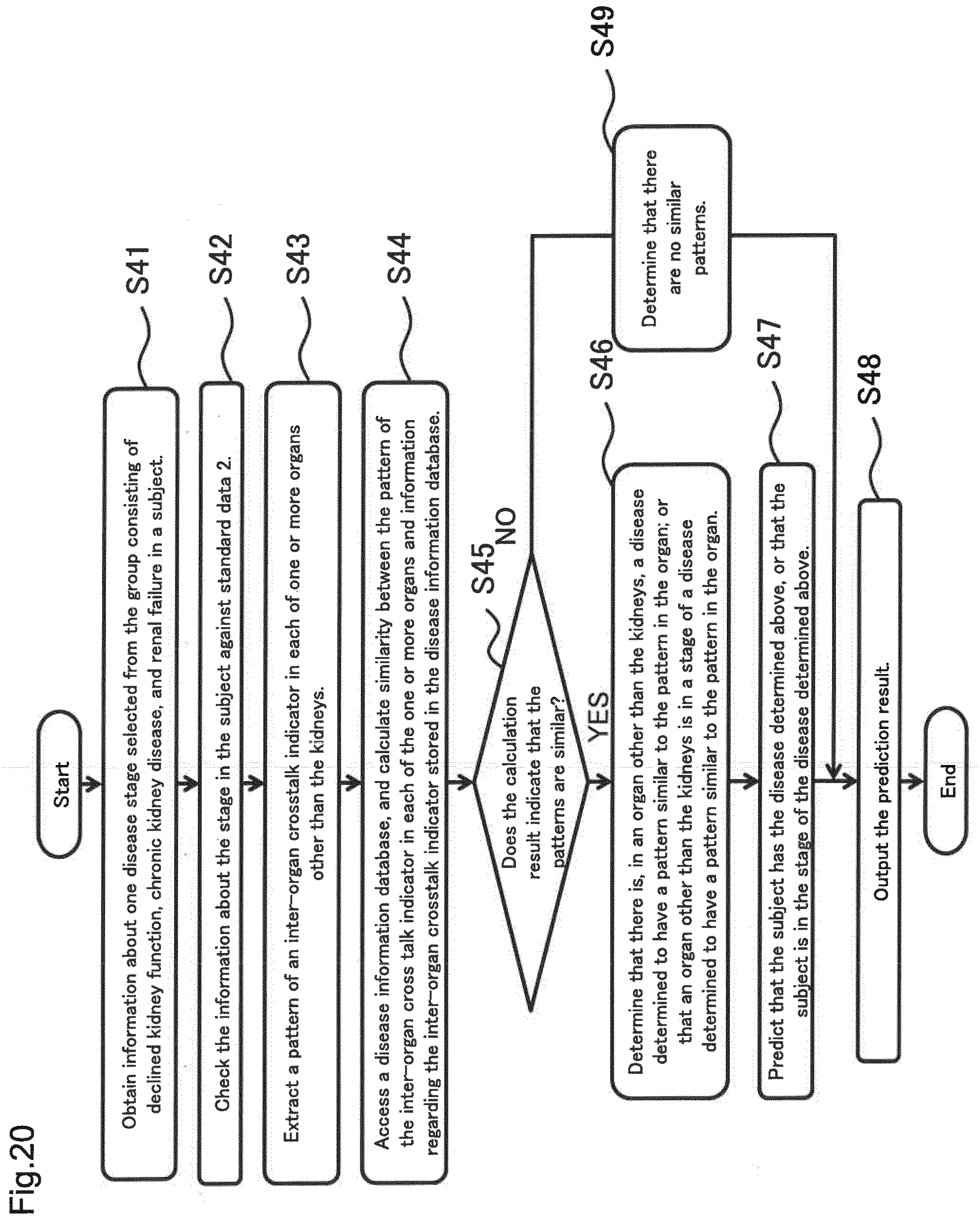
Start
S41
Obtain information about one disease stage selected from the group consisting of declined kidney function, chronic kidney disease, and renal failure in a subject.
S42
Check the information about the stage in the subject against standard data 2.
S43
Extract a pattern of an inter-organ crosstalk indicator in each of one or more organs other than the kidneys.
S44
Access a disease information database, and calculate similarity between the pattern of the inter-organ cross talk indicator in each of the one or more organs and information regarding the inter-organ crosstalk indicator stored in the disease information database.
S45
Does the calculation result indicate that the patterns are similar?
YES
NO
S46
Determine that there is, in an organ other than the kidneys, a disease determined to have a pattern similar to the pattern in the organ; or that an organ other than the kidneys is in a stage of a disease determined to have a pattern similar to the pattern in the organ.
S49
Determine that there are no similar patterns.
S47
Predict that the subject has the disease determined above, or that the subject is in the stage of the disease determined above.
S48
Output the prediction result.
End

Fig.21 - 1

| Line No. | Gene Name | Reference Seq. ID | Chromosome Location |
|---|---|---|---|
| 1 | 0610005C13Rik | NR_038165.1 | chr7:45567794-45575176 |
| 2 | 0610007P14Rik | NM_021446.2 | chr12:85815454-85824545 |
| 3 | 0610009B22Rik | NM_025319.2 | chr11:51685384-51688634 |
| 4 | 0610009L18Rik | NR_038126.1 | chr11:120348677-120351190 |
| 5 | 0610009O20Rik | NM_024179.5 | chr18:38250248-38262629 |
| 6 | 0610010B08Rik | NM_001177543.1 | chr2:175965355-175981734 |
| 7 | 0610010F05Rik | NM_027860.2 | chr11:23573775-23633631 |
| 8 | 0610010K14Rik | NM_001177601.1 | chr11:70235203-70237914 |
| 9 | 0610011F06Rik | NM_026686.2 | chr17:25875499-25877163 |
| 10 | 0610012G03Rik | NR_027897.1 | chr16:31947050-31948521 |
| 11 | 0610030E20Rik | NM_026696.1 | chr6:72347316-72353160 |
| 12 | 0610031J06Rik | NM_020003.1 | chr3:88325022-88328631 |
| 13 | 0610031O16Rik | NR_045760.1 | chr3:138210716-138239663 |
| 14 | 0610037L13Rik | NM_028754.2 | chr4:107889898-107897802 |
| 15 | 0610038B21Rik | NR_028125.1 | chr8:77517055-77518578 |
| 16 | 0610039K10Rik | NR_028113.1 | chr2:163644849-163645800 |
| 17 | 0610040B10Rik | NR_027874.1 | chr5:143329307-143332704 |
| 18 | 0610040F04Rik | NR_040757.1 | chr6:108610217-108623167 |
| 19 | 0610040J01Rik | NM_029554.4 | chr5:63812494-63899619 |
| 20 | 0610043K17Rik | NR_040640.1 | chr4:101353782-101399185 |
| 21 | 1010001N08Rik | NR_105022.1 | chr18:11049928-11052567 |
| 22 | 1100001G20Rik | NM_183249.2 | chr11:83746939-83752646 |
| 23 | 1110001J03Rik | NM_025363.3 | chr6:38534860-38539449 |
| 24 | 1110002L01Rik | NR_030694.1 | chr12:3403882-3426747 |
| 25 | 1110004E09Rik | NM_026502.2 | chr16:90925810-90934849 |
| 26 | 1110004F10Rik | NM_019772.2 | chr7:116093379-116105210 |
| 27 | 1110006O24Rik | NR_027810.1 | chr5:115631048-115631816 |
| 28 | 1110007C09Rik | NM_026738.2 | chr13:49202950-49216026 |
| 29 | 1110008F13Rik | NM_026124.3 | chr2:156863121-156873562 |
| 30 | 1110008L16Rik | NM_025373.1 | chr12:55302636-55382491 |
| 31 | 1110008P14Rik | NM_198001.2 | chr2:32379100-32381915 |
| 32 | 1110012L19Rik | NM_026787.2 | chrX:70385912-70389416 |
| 33 | 1110015O18Rik | NR_045272.1 | chr3:4798707-4814911 |
| 34 | 1110017D15Rik | NM_001048005.1 | chr4:41505008-41517333 |
| 35 | 1110019D14Rik | NR_045995.1 | chr6:13871568-13896421 |
| 36 | 1110020A21Rik | NR_027929.1 | chr17:84917181-84957423 |
| 37 | 1110025L11Rik | NM_001276278.1 | chr16:89063409-89064002 |
| 38 | 1110028F11Rik | NR_045139.1 | chr11:87699523-87705327 |
| 39 | 1110028F18Rik | NR_045470.1 | chr8:106587142-106594820 |
| 40 | 1110032A03Rik | NM_023483.3 | chr9:50762827-50768152 |
| 41 | 1110032F04Rik | NM_001167996.1 | chr3:68869585-68872163 |
| 42 | 1110034G24Rik | NM_028637.1 | chr2:132690282-132751055 |
| 43 | 1110036E04Rik | NR_040713.1 | chr9:64049828-64054100 |
| 44 | 1110037F02Rik | NM_001081183.1 | chr4:11485957-11551143 |
| 45 | 1110038B12Rik | NR_015536.1 | chr17:34950235-34952471 |
| 46 | 1110038F14Rik | NM_054099.2 | chr15:76948543-76950731 |
| 47 | 1110046J04Rik | NR_040707.1 | chr13:33936031-33960181 |
| 48 | 1110051M20Rik | NM_175123.4 | chr2:91277827-91444642 |
| 49 | 1110054M08Rik | NR_037954.1 | chr16:24392555-24393655 |
| 50 | 1110057K04Rik | NM_001167767.1 | chr12:8208126-8285759 |
| 51 | 1110058L19Rik | NM_026503.3 | chr1:23995938-24005640 |
| 52 | 1110059E24Rik | NM_025423.2 | chr19:21597312-21652791 |
| 53 | 1110059G10Rik | NM_025419.4 | chr9:122945088-122951000 |
| 54 | 1110065P20Rik | NM_001142727.1 | chr4:124849484-124850730 |
| 55 | 1190002F15Rik | NR_037955.1 | chr6:134929091-134951718 |
| 56 | 1190002N15Rik | NM_001033145.2 | chr9:94517863-94538081 |
| 57 | 1190003K10Rik | NM_001195435.1 | chr13:64481245-64497792 |
| 58 | 1190005I06Rik | NM_197988.1 | chr8:120608601-120634382 |
| 59 | 1190007I07Rik | NM_001135567.1 | chr10:82619850-82623228 |
| 60 | 1200014J11Rik | NM_025818.3 | chr11:73047866-73083579 |
| 61 | 1300002E11Rik | NR_037957.1 | chr16:21794346-21806125 |
| 62 | 1300002K09Rik | NM_028788.4 | chr4:45848946-45887010 |
| 63 | 1300017J02Rik | NM_027918.2 | chr9:103250529-103288297 |
| 64 | 1500004A13Rik | NR_015498.2 | chr3:88822009-88832487 |
| 65 | 1500009C09Rik | NR_037697.1 | chr15:82252396-82260751 |
| 66 | 1500009L16Rik | NM_001145198.1 | chr10:83722864-83762762 |
| 67 | 1500011B03Rik | NR_027817.1 | chr5:114808195-114813976 |
| 68 | 1500011K16Rik | NR_015476.1 | chr2:127791376-127792488 |
| 69 | 1500012F01Rik | NM_001081005.1 | chr2:167062933-167065862 |
| 70 | 1500012K07Rik | NR_045812.1 | chr7:75308695-75318485 |
| 71 | 1500015A07Rik | NR_029432.1 | chr18:61726389-61728253 |
| 72 | 1500015L24Rik | NR_045817.2 | chr19:20405287-20422785 |
| 73 | 1500015O10Rik | NM_024283.3 | chr1:43730601-43742564 |
| 74 | 1500017E21Rik | NR_033510.1 | chr19:36619417-36689479 |
| 75 | 1600002D24Rik | NR_040484.1 | chr16:95841885-95929077 |
| 76 | 1600002H07Rik | NM_028056.1 | chr17:24215055-24220769 |
| 77 | 1600002K03Rik | NM_027207.2 | chr10:80172943-80175119 |
| 78 | 1600010M07Rik | NR_037959.1 | chr7:109998376-110006646 |
| 79 | 1600012H06Rik | NM_001083880.1 | chr17:14943183-14945939 |
| 80 | 1600014C10Rik | NM_001085385.1 | chr7:38183216-38197565 |
| 81 | 1600014C23Rik | NM_028164.1 | chr17:45732863-45733844 |
| 82 | 1600014K23Rik | NM_028046.1 | chrX:85249676-85270291 |
| 83 | 1600015I10Rik | NM_001081273.2 | chr6:48929895-48933687 |
| 84 | 1600016N20Rik | NM_028050.2 | chr7:141210042-141214080 |
| 85 | 1600019K03Rik | NR_040481.1 | chr16:35503169-35509419 |
| 86 | 1600020E01Rik | NR_037960.1 | chr6:86527329-86564449 |
| 87 | 1600023N17Rik | NR_073433.1 | chr5:45668700-45669708 |
| 88 | 1600025M17Rik | NR_038168.1 | chrX:56317607-56320664 |
| 89 | 1600027J07Rik | NR_036588.1 | chr8:103343877-103347534 |
| 90 | 1600029I14Rik | NR_028123.1 | chr9:99470420-99474751 |
| 91 | 1600029O15Rik | NR_033522.1 | chr9:58202896-58208808 |
| 92 | 1700001C02Rik | NM_029285.1 | chr5:30466076-30484087 |
| 93 | 1700001C19Rik | NM_001172091.1 | chr17:47412733-47414711 |
| 94 | 1700001D01Rik | NR_045475.1 | chr8:61281521-61288872 |
| 95 | 1700001F09Rik | NM_027940.2 | chr14:43342383-43347811 |
| 96 | 1700001G11Rik | NR_038077.1 | chr14:66295328-66297129 |
| 97 | 1700001G17Rik | NR_033199.1 | chr1:33669823-33670712 |
| 98 | 1700001J03Rik | NM_001008547.1 | chr5:146182449-146185304 |
| 99 | 1700001J11Rik | NR_033613.1 | chr9:40050557-40053025 |
| 100 | 1700001K19Rik | NM_025488.2 | chr12:110667688-110682619 |
| 101 | 1700001K23Rik | NR_036590.1 | chr19:53248735-53255205 |
| 102 | 1700001L05Rik | NR_027980.1 | chr15:83353846-83367297 |
| 103 | 1700001L19Rik | NM_027035.1 | chr13:68597438-68614231 |
| 104 | 1700001O22Rik | NM_198000.3 | chr2:30795562-30801737 |
| 105 | 1700001P01Rik | NM_028156.2 | chr11:97771480-97775918 |
| 106 | 1700003C15Rik | NR_045478.1 | chr5:11749832-11769308 |
| 107 | 1700003D09Rik | NR_045477.1 | chr11:98350717-98358283 |
| 108 | 1700003E16Rik | NM_027948.1 | chr6:83156403-83162975 |
| 109 | 1700003E24Rik | NR_103799.1 | chrX:93156220-93183944 |
| 110 | 1700003F12Rik | NM_029305.2 | chr2:154548903-154550048 |
| 111 | 1700003G13Rik | NR_040720.1 | chr9:45318265-45322079 |
| 112 | 1700003G18Rik | NR_029433.1 | chr7:116081761-116093149 |
| 113 | 1700003H04Rik | NR_015460.1 | chr3:124565890-124581091 |
| 114 | 1700003L19Rik | NR_040507.1 | chr16:12811389-12848653 |
| 115 | 1700003M02Rik | NM_027041.4 | chr4:34711331-34730206 |
| 116 | 1700003M07Rik | NR_040647.1 | chr4:129960373-129965138 |
| 117 | 1700003P14Rik | NR_045982.1 | chr13:118556167-118588629 |
| 118 | 1700006A11Rik | NM_027939.1 | chr3:124401154-124426028 |
| 119 | 1700006E09Rik | NM_029287.1 | chr11:101987055-101992264 |
| 120 | 1700006F04Rik | NR_045621.1 | chr14:119749226-119751564 |
| 121 | 1700006H21Rik | NR_045900.2 | chr13:107687396-107692168 |
| 122 | 1700007B14Rik | NM_001164235.1 | chr8:75448693-75984507 |
| 123 | 1700007F19Rik | NR_040538.1 | chr3:58141706-58163807 |
| 124 | 1700007G11Rik | NM_001024614.1 | chr5:98329303-98802019 |
| 125 | 1700007J10Rik | NR_045476.1 | chr11:59725916-59740154 |
| 126 | 1700007K09Rik | NM_027037.2 | chr7:131325930-131329499 |
| 127 | 1700007K13Rik | NM_027040.1 | chr2:28462000-28466324 |
| 128 | 1700007L15Rik | NR_045709.1 | chr16:33379853-33380736 |
| 129 | 1700007P06Rik | NR_040554.1 | chr1:187125137-187127852 |
| 130 | 1700008F21Rik | NM_001168369.1 | chr8:129067133-129183732 |
| 131 | 1700008I05Rik | NM_027952.3 | chrX:135654697-135693790 |
| 132 | 1700008J07Rik | NR_024331.1 | chr7:127510437-127512869 |
| 133 | 1700008K24Rik | NR_038141.1 | chr17:49112263-49113533 |
| 134 | 1700008O03Rik | NM_027049.1 | chr7:44360044-44375030 |
| 135 | 1700008P02Rik | NM_027048.1 | chr3:6615412-6620443 |
| 136 | 1700009C05Rik | NR_046040.1 | chr6:81900464-81910800 |
| 137 | 1700009J07Rik | NR_015547.1 | chr10:77893419-77896111 |
| 138 | 1700009N14Rik | NM_001081095.1 | chr4:39450292-39451778 |
| 139 | 1700009P17Rik | NM_001081275.1 | chr1:171121660-171126967 |
| 140 | 1700010B08Rik | NM_029308.1 | chr2:173719414-173722086 |
| 141 | 1700010D01Rik | NM_029590.3 | chrX:95732589-95733265 |
| 142 | 1700010I02Rik | NR_040587.1 | chr3:7925302-7954887 |
| 143 | 1700010I14Rik | NM_025851.3 | chr17:8988332-9008319 |
| 144 | 1700010J16Rik | NR_040579.1 | chr10:112726925-112785599 |
| 145 | 1700010K23Rik | NR_040512.1 | chr16:66657117-66664626 |
| 146 | 1700011A15Rik | NM_025487.3 | chr15:101447744-101453909 |
| 147 | 1700011B04Rik | NR_045616.1 | chr13:35181622-35188014 |
| 148 | 1700011E24Rik | NM_029298.1 | chr17:87389570-87427741 |
| 149 | 1700011H14Rik | NM_025956.4 | chr14:49226358-49245428 |
| 150 | 1700011I03Rik | NM_029290.1 | chr18:57533825-57731065 |
| 151 | 1700011L22Rik | NM_026315.1 | chr8:79210429-79248583 |
| 152 | 1700011M02Rik | NR_073044.1 | chrX:102908904-102909651 |
| 153 | 1700012A03Rik | NM_029587.2 | chr6:32050287-32058915 |
| 154 | 1700012B07Rik | NM_001162428.1 | chr11:109788290-109828046 |
| 155 | 1700012B09Rik | NM_029306.3 | chr9:14758193-14771030 |
| 156 | 1700012D01Rik | NR_045171.1 | chr10:127667122-127668851 |
| 157 | 1700012D14Rik | NR_015573.2 | chr7:111117672-111122675 |
| 158 | 1700012I11Rik | NR_045140.1 | chr15:67226768-67377094 |
| 159 | 1700012L04Rik | NM_029588.3 | chrX:9283763-9284288 |
| 160 | 1700012P22Rik | NM_027056.1 | chr4:144418190-144438772 |
| 161 | 1700013D24Rik | NM_001177502.1 | chr6:124347593-124357086 |
| 162 | 1700013F07Rik | NM_029314.1 | chr3:108537583-108544697 |
| 163 | 1700013G24Rik | NM_027063.2 | chr4:137453295-137455461 |
| 164 | 1700013H16Rik | NM_001200013.1 | chrX:53742900-53757831 |
| 165 | 1700015E13Rik | NM_001039593.1 | chr1:170308860-170312125 |
| 166 | 1700015F17Rik | NM_001200025.1 | chr5:5437826-5479143 |
| 167 | 1700015G11Rik | NM_001195601.1 | chr7:52011679-52015716 |
| 168 | 1700016C15Rik | NM_027077.2 | chr1:177729813-177753305 |
| 169 | 1700016D06Rik | NM_024271.1 | chr8:11654923-11678750 |
| 170 | 1700016G22Rik | NR_045891.1 | chr13:5855508-5858092 |
| 171 | 1700016H13Rik | NM_001163550.1 | chr5:103648586-103655732 |
| 172 | 1700016K19Rik | NM_198637.2 | chr11:75999911-76003569 |
| 173 | 1700016L04Rik | NR_045824.1 | chr10:14705608-14759019 |
| 174 | 1700016L21Rik | NR_040460.1 | chr1:80445931-80475660 |
| 175 | 1700016P04Rik | NR_038149.1 | chr6:13413336-13415996 |
| 176 | 1700017B05Rik | NM_028820.2 | chr9:57252321-57262599 |
| 177 | 1700017D01Rik | NM_027058.1 | chr19:11096815-11130878 |
| 178 | 1700017G19Rik | NR_040445.1 | chr3:40504864-40522912 |
| 179 | 1700017J07Rik | NR_040326.1 | chr2:168978268-168978906 |
| 180 | 1700017N19Rik | NM_001081246.1 | chr10:100592385-100618391 |
| 181 | 1700018A04Rik | NR_029439.1 | chr13:31565491-31582513 |
| 182 | 1700018B08Rik | NM_029597.1 | chr8:121530783-121541954 |
| 183 | 1700018B24Rik | NR_003617.1 | chr3:48605730-48609100 |
| 184 | 1700018C11Rik | NM_029324.2 | chr4:63607090-63622429 |
| 185 | 1700018F24Rik | NM_027069.3 | chr5:145042989-145045678 |
| 186 | 1700018G05Rik | NR_045422.1 | chrX:102928371-102929107 |
| 187 | 1700018L02Rik | NR_028360.1 | chr19:29047482-29048729 |
| 188 | 1700019A02Rik | NM_027070.1 | chr1:53158576-53187617 |
| 189 | 1700019B03Rik | NM_029598.1 | chr8:3470861-3487178 |
| 190 | 1700019B21Rik | NR_045442.1 | chrX:62510538-62527011 |
| 191 | 1700019D03Rik | NM_144953.2 | chr1:52925126-52952840 |
| 192 | 1700019E08Rik | NR_040497.1 | chr2:45696604-45698447 |
| 193 | 1700019G17Rik | NM_001145895.1 | chr6:85899050-85902533 |

Fig.21 - 2

| | | | |
|---|---|---|---|
| 194 | 1700019G24Rik | NR_040255.1 | chr6:5963896-5977393 |
| 195 | 1700019L03Rik | NM_025619.1 | chr2:32777412-32784405 |
| 196 | 1700019M22Rik | NR_103800.1 | chr12:96046620-96047222 |
| 197 | 1700019N19Rik | NM_026208.2 | chr19:58785802-58794414 |
| 198 | 1700019O17Rik | NM_027966.1 | chr1:86426328-86428049 |
| 199 | 1700020A23Rik | NM_001163483.1 | chr2:130405295-130406074 |
| 200 | 1700020D05Rik | NM_023781.5 | chr19:5502768-5503787 |
| 201 | 1700020G17Rik | NR_045979.1 | chr10:110801173-110896507 |
| 202 | 1700020I14Rik | NR_015473.1 | chr2:119594295-119600744 |
| 203 | 1700020L24Rik | NM_025492.3 | chr11:83437693-83441232 |
| 204 | 1700020M21Rik | NR_040742.1 | chr9:120913773-120915250 |
| 205 | 1700020N01Rik | NR_027968.1 | chr10:21593144-21622375 |
| 206 | 1700020N15Rik | NM_029316.1 | chrX:69945280-69945980 |
| 207 | 1700020N18Rik | NR_026924.1 | chr1:91404878-91406029 |
| 208 | 1700021F05Rik | NM_026411.1 | chr10:43525120-43540994 |
| 209 | 1700021F07Rik | NM_028158.1 | chr2:173522591-173528502 |
| 210 | 1700021K19Rik | NM_001200038.1 | chr16:32821701-32868366 |
| 211 | 1700021N21Rik | NR_045800.1 | chr4:134448764-134450171 |
| 212 | 1700022A21Rik | NR_003953.1 | chr5:24645452-24648860 |
| 213 | 1700022A22Rik | NR_045509.1 | chr15:46324584-46373899 |
| 214 | 1700022E09Rik | NR_040568.1 | chr16:59466871-59469238 |
| 215 | 1700022H16Rik | NR_045488.1 | chr12:9565235-9570585 |
| 216 | 1700022I11Rik | NM_026088.3 | chr4:42969945-42974325 |
| 217 | 1700023C21Rik | NR_045909.1 | chr11:109845877-109848476 |
| 218 | 1700023E05Rik | NM_027970.1 | chr5:77016022-77061522 |
| 219 | 1700023F02Rik | NR_038039.1 | chr10:66120608-66124064 |
| 220 | 1700023F06Rik | NM_001254724.2 | chr11:103198943-103208548 |
| 221 | 1700023L04Rik | NR_040263.1 | chr6:29985328-29993531 |
| 222 | 1700024B18Rik | NR_045479.1 | chr14:123987634-124015684 |
| 223 | 1700024F13Rik | NR_045363.1 | chr13:3498049-3501393 |
| 224 | 1700024G13Rik | NM_001034037.1 | chr14:32376501-32388373 |
| 225 | 1700024P04Rik | NM_027064.1 | chr13:98984089-98984565 |
| 226 | 1700024P16Rik | NM_001162980.1 | chr4:104913455-105016863 |
| 227 | 1700025B11Rik | NR_040310.1 | chr15:77558239-77561892 |
| 228 | 1700025C18Rik | NR_033448.1 | chr2:165078692-165090750 |
| 229 | 1700025F22Rik | NM_027074.3 | chr19:11139682-11165320 |
| 230 | 1700025F24Rik | NR_040578.1 | chr10:119413443-119426864 |
| 231 | 1700025G04Rik | NM_197990.3 | chr1:151884523-152090320 |
| 232 | 1700025K24Rik | NR_045825.1 | chr17:54352582-54370074 |
| 233 | 1700025M24Rik | NR_040687.1 | chr5:73268579-73284184 |
| 234 | 1700025N23Rik | NR_040523.1 | chr6:39063878-39067586 |
| 235 | 1700026D08Rik | NM_029335.3 | chr7:83775616-83794839 |
| 236 | 1700026D11Rik | NR_028286.1 | chr2:132490727-132524057 |
| 237 | 1700026F02Rik | NR_045487.1 | chr8:71006727-71026755 |
| 238 | 1700026L06Rik | NM_027283.1 | chr2:28692079-28699651 |
| 239 | 1700027A15Rik | NR_038001.1 | chr1:73016035-73025508 |
| 240 | 1700027F09Rik | NR_040681.1 | chr5:64454812-64467942 |
| 241 | 1700027H10Rik | NR_040594.1 | chr3:45416583-45439309 |
| 242 | 1700027I24Rik | NR_040741.1 | chr9:36668855-36693189 |
| 243 | 1700027J07Rik | NR_040581.2 | chr10:43746157-43765836 |
| 244 | 1700028B04Rik | NR_033605.1 | chr7:28496940-28497492 |
| 245 | 1700028D13Rik | NR_045377.1 | chr5:112206762-112210002 |
| 246 | 1700028E10Rik | NR_045699.1 | chr5:151368674-151396164 |
| 247 | 1700028I16Rik | NR_038042.1 | chr10:82812122-82824242 |
| 248 | 1700028J19Rik | NR_029436.1 | chr7:44229929-44236122 |
| 249 | 1700028K03Rik | NM_175241.1 | chr5:107534710-107551542 |
| 250 | 1700028M03Rik | NR_036591.1 | chr3:83555366-83574118 |
| 251 | 1700028P14Rik | NM_026188.2 | chr19:23558759-23652812 |
| 252 | 1700028P15Rik | NR_040509.1 | chr2:171956878-171962799 |
| 253 | 1700029B22Rik | NR_040531.1 | chr7:131146438-131150578 |
| 254 | 1700029F12Rik | NM_001080777.2 | chr13:97021863-97034362 |
| 255 | 1700029H14Rik | NM_001080781.2 | chr8:13550721-13562461 |
| 256 | 1700029I15Rik | NM_183112.3 | chr2:92382917-92383603 |
| 257 | 1700029J03Rik | NR_040494.1 | chr16:93396814-93458872 |
| 258 | 1700029J07Rik | NM_001033148.3 | chr8:45953605-45975252 |
| 259 | 1700029M20Rik | NR_015613.1 | chr4:135626654-135630198 |
| 260 | 1700029N11Rik | NR_045489.1 | chr13:44439726-44457567 |
| 261 | 1700029P11Rik | NM_025503.4 | chr15:81980539-81981563 |
| 262 | 1700030A11Rik | NR_045457.1 | chr17:28905264-28906771 |
| 263 | 1700030C10Rik | NR_015521.1 | chr12:20804391-20815779 |
| 264 | 1700030F04Rik | NR_045731.1 | chr6:117679206-117751769 |
| 265 | 1700030F18Rik | NM_028180.3 | chr15:99918347-99931658 |
| 266 | 1700030J22Rik | NM_027103.2 | chr8:116969598-116978943 |
| 267 | 1700030K09Rik | NM_028170.2 | chr8:72443879-72460541 |
| 268 | 1700030L20Rik | NR_040592.1 | chr3:136435269-136449349 |
| 269 | 1700030M09Rik | NR_045903.1 | chr8:121544387-121547652 |
| 270 | 1700030N03Rik | NR_045304.1 | chr19:3153798-3197703 |
| 271 | 1700030O20Rik | NR_045345.1 | chr10:116723066-116729177 |
| 272 | 1700031A10Rik | NR_045439.1 | chr17:36923791-36933432 |
| 273 | 1700031F05Rik | NM_028496.1 | chrX:102859186-102866353 |
| 274 | 1700031M16Rik | NR_015496.1 | chr15:98398444-98416135 |
| 275 | 1700031P21Rik | NR_045910.1 | chr12:52599983-52602322 |
| 276 | 1700034E13Rik | NM_030097.1 | chr18:52646219-52663731 |
| 277 | 1700034F02Rik | NM_001163521.1 | chr11:29547949-29578352 |
| 278 | 1700034G24Rik | NR_045396.1 | chr5:112582935-112589996 |
| 279 | 1700034H15Rik | NR_030669.1 | chr1:191894071-191907527 |
| 280 | 1700034I23Rik | NR_045380.1 | chr3:40900198-40902543 |
| 281 | 1700034J05Rik | NM_001164236.1 | chr6:146951300-146954421 |
| 282 | 1700034K08Rik | NR_040756.1 | chr9:92979915-93005733 |
| 283 | 1700034O15Rik | NM_029671.2 | chr6:41684430-41685717 |
| 284 | 1700034P13Rik | NR_040462.1 | chr1:9747647-9771256 |
| 285 | 1700036G14Rik | NR_040542.1 | chr3:85317518-85332027 |
| 286 | 1700037C18Rik | NM_028484.2 | chr16:3905797-3908689 |
| 287 | 1700037H04Rik | NM_026091.2 | chr2:131146324-131160020 |
| 288 | 1700039E15Rik | NM_001033176.1 | chr7:45282872-45288993 |
| 289 | 1700039E22Rik | NR_045315.1 | chr19:44828492-44835938 |
| 290 | 1700040L02Rik | NM_028491.1 | chr10:68430955-68541875 |
| 291 | 1700041C23Rik | NR_033784.1 | chr9:57175274-57186110 |
| 292 | 1700041M19Rik | NR_040573.1 | chr16:77004227-77010881 |
| 293 | 1700042B14Rik | NM_001081671.1 | chrX:155124980-155147849 |
| 294 | 1700042G07Rik | NM_001099295.2 | chr4:116173371-116174297 |
| 295 | 1700042G15Rik | NR_038178.1 | chr4:57359791-57364292 |
| 296 | 1700042O10Rik | NR_045178.1 | chr11:11868122-11885321 |
| 297 | 1700044C05Rik | NR_045624.1 | chr14:118365877-118398165 |
| 298 | 1700044K03Rik | NR_033785.1 | chr18:49523288-49524441 |
| 299 | 1700045H11Rik | NR_040649.1 | chr4:150828224-150854949 |
| 300 | 1700046C09Rik | NR_045918.1 | chr11:14560335-14599225 |
| 301 | 1700047A11Rik | NR_110583.1 | chr8:26082608-26096520 |
| 302 | 1700047E10Rik | NR_073363.1 | chr14:44191606-44213237 |
| 303 | 1700047G03Rik | NR_040447.1 | chr15:11967061-11970083 |
| 304 | 1700047I17Rik2 | NM_001100116.1 | chr12:55124527-55217146 |
| 305 | 1700047L14Rik | NR_040691.1 | chr5:108763841-108768917 |
| 306 | 1700047M11Rik | NR_015458.1 | chr1:182300833-182303289 |
| 307 | 1700048M11Rik | NR_045300.1 | chr16:92308715-92312466 |
| 308 | 1700048O20Rik | NR_033553.1 | chr9:121937274-121947016 |
| 309 | 1700049E15Rik | NR_033636.1 | chr6:147690240-147702374 |
| 310 | 1700049E22Rik | NR_040525.1 | chr6:100527399-100533426 |
| 311 | 1700049G17Rik | NM_028538.1 | chr7:27907391-27929430 |
| 312 | 1700049L16Rik | NR_003644.1 | chr10:71979884-71980690 |
| 313 | 1700051A21Rik | NR_045922.1 | chr11:72266843-72268556 |
| 314 | 1700052I22Rik | NR_033786.1 | chr12:80923431-80924447 |
| 315 | 1700052K11Rik | NR_027956.1 | chr11:105179020-105181433 |
| 316 | 1700052N19Rik | NM_024261.2 | chr10:4432604-4455140 |
| 317 | 1700054A03Rik | NR_045320.1 | chr19:53076251-53084392 |
| 318 | 1700054K19Rik | NR_027865.1 | chr6:112210707-112212518 |
| 319 | 1700054M17Rik | NR_045919.1 | chr2:118304913-118308166 |
| 320 | 1700054O13Rik | NM_026096.1 | chrX:9846946-9847500 |
| 321 | 1700055C04Rik | NR_040726.1 | chr9:64038464-64041465 |
| 322 | 1700055N04Rik | NM_028545.2 | chr19:3958807-3970438 |
| 323 | 1700056E22Rik | NM_028516.1 | chr1:184033031-184033998 |
| 324 | 1700057G04Rik | NM_001033184.3 | chr9:92309376-92357876 |
| 325 | 1700057H15Rik | NR_040774.1 | chr4:124449625-124485959 |
| 326 | 1700060C16Rik | NR_045732.1 | chr6:143593352-143651288 |
| 327 | 1700060C20Rik | NR_036606.2 | chr2:158192007-158195734 |
| 328 | 1700061F12Rik | NR_038180.1 | chr2:9189514-9197484 |
| 329 | 1700061G19Rik | NM_030141.1 | chr17:56875632-56888838 |
| 330 | 1700061I17Rik | NR_038029.1 | chr3:117060521-117077765 |
| 331 | 1700063A18Rik | NR_040467.1 | chr1:95990658-96021758 |
| 332 | 1700063D05Rik | NR_040392.1 | chr9:41206154-41217627 |
| 333 | 1700063O14Rik | NR_045383.1 | chr5:91766241-91767300 |
| 334 | 1700064J06Rik | NR_045348.1 | chr10:119092904-119103107 |
| 335 | 1700064M15Rik | NR_045288.1 | chr12:99626052-99627974 |
| 336 | 1700065D16Rik | NM_001271569.1 | chr9:95855417-95857882 |
| 337 | 1700065I16Rik | NR_040315.2 | chr15:63817187-63819540 |
| 338 | 1700065J11Rik | NR_040526.1 | chr6:35330845-35336829 |
| 339 | 1700065J18Rik | NR_040468.1 | chr1:192841704-192842739 |
| 340 | 1700065L07Rik | NR_108032.1 | chr6:73436964-73471021 |
| 341 | 1700065O20Rik | NR_045386.1 | chr18:49803331-49817956 |
| 342 | 1700066B17Rik | NR_040465.1 | chr1:39842427-39847330 |
| 343 | 1700066B19Rik | NM_001033168.2 | chr18:35726988-35730869 |
| 344 | 1700066M21Rik | NM_028546.1 | chr1:57377619-57385422 |
| 345 | 1700066N21Rik | NR_045924.1 | chr5:87908587-87979351 |
| 346 | 1700066O22Rik | NR_015541.2 | chr18:57504421-57533752 |
| 347 | 1700067G17Rik | NR_040471.1 | chr1:89016112-89022210 |
| 348 | 1700067K01Rik | NM_183097.2 | chr8:84001705-84004770 |
| 349 | 1700067P10Rik | NM_026625.2 | chr17:48089631-48090920 |
| 350 | 1700069L16Rik | NR_033216.1 | chr5:113692423-113724772 |
| 351 | 1700069P05Rik | NR_040527.1 | chr6:118246694-118248454 |
| 352 | 1700071K01Rik | NM_001033765.2 | chr11:81572501-81573539 |
| 353 | 1700071M16Rik | NR_045444.1 | chr17:43588339-43591509 |
| 354 | 1700072B07Rik | NR_040727.1 | chr9:58370503-58374183 |
| 355 | 1700072O05Rik | NR_045733.1 | chr6:120554795-120574204 |
| 356 | 1700073E17Rik | NR_003625.1 | chr6:145387624-145392223 |
| 357 | 1700074H08Rik | NR_045296.1 | chr13:119680041-119681582 |
| 358 | 1700074P13Rik | NM_028550.3 | chr6:40920459-40940557 |
| 359 | 1700080E11Rik | NM_028562.3 | chr9:105143343-105145082 |
| 360 | 1700080N15Rik | NR_040500.1 | chr2:4132064-4141141 |
| 361 | 1700080O16Rik | NM_028851.1 | chrX:51968694-51972772 |
| 362 | 1700081H04Rik | NR_040693.1 | chr5:119108235-119114543 |
| 363 | 1700084C01Rik | NM_001033185.2 | chr1:169928938-169934653 |
| 364 | 1700084E18Rik | NR_028299.1 | chr2:30237197-30237631 |
| 365 | 1700084F23Rik | NR_045965.1 | chr13:70004049-70028348 |
| 366 | 1700084J12Rik | NR_033608.1 | chr15:33405208-33405939 |
| 367 | 1700085C21Rik | NR_046045.1 | chr12:82932519-82939155 |
| 368 | 1700086L19Rik | NR_030733.1 | chr12:74284275-74295950 |
| 369 | 1700086O06Rik | NR_015475.1 | chr18:38238404-38250198 |
| 370 | 1700088E04Rik | NM_138581.2 | chr15:79134654-79141251 |
| 371 | 1700091H14Rik | NR_073362.1 | chr14:42089749-42095277 |
| 372 | 1700092C02Rik | NR_045467.1 | chr8:77624171-77628956 |
| 373 | 1700092C10Rik | NR_045931.1 | chr14:69164798-69171843 |
| 374 | 1700092E19Rik | NR_045933.1 | chr13:26283158-26312406 |
| 375 | 1700092K14Rik | NR_045930.1 | chr11:114198257-114199199 |
| 376 | 1700092M07Rik | NM_001177347.1 | chr19:8740717-8741225 |
| 377 | 1700093K21Rik | NM_001110133.1 | chr11:23516202-23519942 |
| 378 | 1700094D03Rik | NM_028567.1 | chr3:90062795-90068347 |
| 379 | 1700094J05Rik | NR_040580.1 | chr10:76389014-76401507 |
| 380 | 1700094M24Rik | NR_046049.1 | chr6:52492450-52500085 |
| 381 | 1700095A21Rik | NR_045468.1 | chr4:146519038-146546873 |
| 382 | 1700095B10Rik | NR_040675.1 | chr5:112793291-112801701 |
| 383 | 1700096J18Rik | NR_027883.1 | chr11:109346866-109353651 |
| 384 | 1700096K18Rik | NR_027388.1 | chr5:25530017-25531466 |
| 385 | 1700097N02Rik | NR_045287.1 | chr17:30622440-30626905 |
| 386 | 1700100L14Rik | NR_045934.1 | chr13:70573240-70576104 |
| 387 | 1700101E01Rik | NM_001166705.1 | chr2:28955480-29055066 |

Fig.21 - 3

| | | | |
|---|---|---|---|
| 388 | 1700101I11Rik | NR_045270.1 | chr6:129532183-129533284 |
| 389 | 1700101O22Rik | NR_045045.1 | chr12:7372038-7380330 |
| 390 | 1700102H20Rik | NR_045302.1 | chr17:3557823-3559863 |
| 391 | 1700102P08Rik | NM_053216.2 | chr9:108392833-108397767 |
| 392 | 1700104L18Rik | NR_108033.1 | chr12:54233614-54242320 |
| 393 | 1700105P06Rik | NR_045703.1 | chr19:7383002-7383557 |
| 394 | 1700106J16Rik | NM_028859.1 | chr11:88294042-88295261 |
| 395 | 1700108F19Rik | NR_015485.1 | chr14:76677567-76687108 |
| 396 | 1700108J01Rik | NR_015532.1 | chr14:122229904-122233638 |
| 397 | 1700109G14Rik | NR_033788.1 | chr14:61292982-61305334 |
| 398 | 1700109G15Rik | NR_046197.1 | chr11:84716975-84720432 |
| 399 | 1700109H08Rik | NM_029843.2 | chr5:3571715-3584341 |
| 400 | 1700109I08Rik | NR_045936.1 | chr14:39655662-39685293 |
| 401 | 1700109K24Rik | NR_108037.1 | chr15:77084397-77096314 |
| 402 | 1700110C19Rik | NR_045461.1 | chr17:10324601-10329312 |
| 403 | 1700110I01Rik | NR_038059.1 | chr14:3262401-3783730 |
| 404 | 1700110K17Rik | NR_040728.1 | chr9:40333471-40343337 |
| 405 | 1700111N16Rik | NR_033213.2 | chrX:69929259-70143588 |
| 406 | 1700112E06Rik | NM_028275.1 | chr14:22019711-23056088 |
| 407 | 1700112H15Rik | NR_040472.1 | chr1:184527840-184557691 |
| 408 | 1700112J05Rik | NR_077218.1 | chr5:77006441-77018863 |
| 409 | 1700113A16Rik | NR_045997.1 | chr3:88171559-88177785 |
| 410 | 1700113H08Rik | NM_029685.1 | chr10:87058045-87230599 |
| 411 | 1700119H24Rik | NR_040536.1 | chr16:34935854-34939329 |
| 412 | 1700120C14Rik | NR_045627.2 | chr15:99251787-99262041 |
| 413 | 1700120E14Rik | NR_045368.1 | chr18:74495677-74531124 |
| 414 | 1700120G07Rik | NR_046050.1 | chr7:135191764-135196002 |
| 415 | 1700120K04Rik | NR_027915.1 | chr7:127603902-127604445 |
| 416 | 1700121L16Rik | NR_045453.1 | chrX:104816775-104842455 |
| 417 | 1700121N20Rik | NR_036593.1 | chr12:106442651-106447666 |
| 418 | 1700122O11Rik | NM_029689.1 | chr17:48036744-48038293 |
| 419 | 1700123I01Rik | NM_001165919.1 | chr19:6184409-6224219 |
| 420 | 1700123K08Rik | NM_029693.2 | chr5:138561839-138564694 |
| 421 | 1700123L14Rik | NR_003643.1 | chr6:96164502-96166205 |
| 422 | 1700123M08Rik | NR_040577.1 | chr4:11966573-11994295 |
| 423 | 1700123O12Rik | NR_045185.1 | chr4:10508030-10797802 |
| 424 | 1700123O20Rik | NM_021437.2 | chr14:54686170-54690742 |
| 425 | 1700123O21Rik | NR_045799.1 | chr16:5975586-5998496 |
| 426 | 1700124L16Rik | NR_105027.1 | chr6:83761758-83762508 |
| 427 | 1700125G02Rik | NR_040651.1 | chr4:124890887-124894782 |
| 428 | 1700125G22Rik | NR_040548.1 | chr3:27154025-27157019 |
| 429 | 1700125H03Rik | NR_038181.1 | chr8:68368458-68375710 |
| 430 | 1700125H20Rik | NM_028589.1 | chr11:85171095-85181154 |
| 431 | 1700126H18Rik | NR_040695.1 | chr5:66166606-66191335 |
| 432 | 1700128A07Rik | NR_045938.2 | chr14:106417186-106486520 |
| 433 | 1700128F08Rik | NR_033618.2 | chr9:8221888-8241987 |
| 434 | 1700129C05Rik | NM_026461.2 | chr14:59133039-59142893 |
| 435 | 1810006J02Rik | NR_040439.1 | chr1:98131466-98144655 |
| 436 | 1810007C17Rik | NR_045472.1 | chr12:49476992-49480862 |
| 437 | 1810007D17Rik | NR_038136.1 | chr19:58606328-58629753 |
| 438 | 1810008I18Rik | NR_045301.1 | chr7:65804051-65806223 |
| 439 | 1810009A15Rik | NM_025463.3 | chr19:8888882-8890759 |
| 440 | 1810009J06Rik | NM_023707.3 | chr6:40964771-40968427 |
| 441 | 1810010D01Rik | NR_033626.1 | chr7:145205817-145208119 |
| 442 | 1810010H24Rik | NM_001163473.1 | chr11:107028222-107030442 |
| 443 | 1810011H11Rik | NM_001163616.1 | chr14:32785962-32817968 |
| 444 | 1810011O10Rik | NM_026931.2 | chr8:24437615-24438946 |
| 445 | 1810012K16Rik | NR_045473.1 | chr8:22398720-22399591 |
| 446 | 1810013A23Rik | NR_045427.1 | chr17:28271707-28273163 |
| 447 | 1810013L24Rik | NM_001081400.3 | chr16:8830099-8858924 |
| 448 | 1810014B01Rik | NR_015572.2 | chr10:86685526-86689954 |
| 449 | 1810018F18Rik | NR_038140.1 | chr19:58698916-58701323 |
| 450 | 1810019D21Rik | NR_040344.1 | chr8:106135399-106138722 |
| 451 | 1810020O05Rik | NR_045482.1 | chr6:87675592-87690847 |
| 452 | 1810021B22Rik | NR_040417.1 | chr15:89071197-89075854 |
| 453 | 1810022K09Rik | NM_001099674.1 | chr3:14606288-14611256 |
| 454 | 1810024B03Rik | NM_198630.2 | chr2:127186354-127208280 |
| 455 | 1810026B05Rik | NR_037569.1 | chr7:73539797-73558395 |
| 456 | 1810026J23Rik | NM_178619.4 | chr9:21592721-21595970 |
| 457 | 1810030O07Rik | NM_175141.6 | chrX:12654880-12673639 |
| 458 | 1810032O08Rik | NR_027819.1 | chr11:116671659-116675798 |
| 459 | 1810034E14Rik | NR_045798.1 | chr13:64248699-64268145 |
| 460 | 1810037I17Rik | NM_024461.2 | chr3:122924396-122926194 |
| 461 | 1810041L15Rik | NM_001163145.1 | chr15:84379202-84447097 |
| 462 | 1810043G02Rik | NM_026431.2 | chr10:77978649-77985438 |
| 463 | 1810043H04Rik | NM_001110242.1 | chr11:120098933-120100424 |
| 464 | 1810044D09Rik | NR_038153.1 | chr6:91440986-91441755 |
| 465 | 1810046K07Rik | NM_027217.1 | chr9:51289685-51328917 |
| 466 | 1810053B23Rik | NR_040486.1 | chr16:93343723-93353189 |
| 467 | 1810055G02Rik | NM_028077.2 | chr19:3708332-3717881 |
| 468 | 1810058I24Rik | NR_015608.1 | chr6:35252698-35262059 |
| 469 | 1810062G17Rik | NM_028183.1 | chr3:36475936-36482299 |
| 470 | 1810062O18Rik | NR_033571.1 | chr14:20546292-20570680 |
| 471 | 1810064F22Rik | NR_027981.1 | chr9:22206785-22213860 |
| 472 | 1810065E05Rik | NM_027239.2 | chr11:58421110-58426023 |
| 473 | 2010001E11Rik | NM_001163503.1 | chr10:39920381-39926923 |
| 474 | 2010002M12Rik | NM_053217.2 | chr19:34617050-34640743 |
| 475 | 2010003K11Rik | NM_027237.1 | chr19:4496787-4498583 |
| 476 | 2010005H15Rik | NM_029733.3 | chr16:36221561-36257427 |
| 477 | 2010009K17Rik | NR_040609.1 | chr2:158070363-158091797 |
| 478 | 2010010A06Rik | NR_045393.1 | chr18:75295674-75297329 |
| 479 | 2010012O05Rik | NM_025563.3 | chr19:46689905-46703382 |
| 480 | 2010015L04Rik | NM_001166029.1 | chr4:155409189-155421996 |
| 481 | 2010016I18Rik | NR_033207.1 | chr3:106481985-106485913 |
| 482 | 2010106C02Rik | NR_045435.1 | chr17:86285166-86287178 |
| 483 | 2010106E10Rik | NM_001168590.1 | chrX:112495273-112558343 |
| 484 | 2010107E04Rik | NM_027360.2 | chr12:111961375-111966977 |
| 485 | 2010107G12Rik | NM_001025573.2 | chr6:34945294-34977999 |
| 486 | 2010107G23Rik | NM_027251.3 | chr10:62107655-62111013 |
| 487 | 2010109A12Rik | NM_029363.1 | chr5:93206517-93213474 |
| 488 | 2010109I03Rik | NM_025929.2 | chr15:74878335-74881704 |
| 489 | 2010111I01Rik | NM_001289924.1 | chr13:62964892-63302877 |
| 490 | 2010204K13Rik | NR_027924.1 | chrX:7411816-7422988 |
| 491 | 2010300C02Rik | NM_028096.1 | chr1:37611675-37719811 |
| 492 | 2010308F09Rik | NR_045429.1 | chrX:13234640-13247733 |
| 493 | 2010310C07Rik | NR_045169.1 | chr6:42370670-42380558 |
| 494 | 2010315B03Rik | NM_001243117.1 | chr9:124291803-124312694 |
| 495 | 2010320M18Rik | NR_029440.1 | chr8:70776861-70777606 |
| 496 | 2200002D01Rik | NM_028179.1 | chr7:29247520-29248467 |
| 497 | 2200002J24Rik | NM_026961.2 | chr7:30698925-30700534 |
| 498 | 2210010C04Rik | NM_023333.4 | chr6:41030267-41035509 |
| 499 | 2210011C24Rik | NM_001291292.1 | chr8:84010227-84011720 |
| 500 | 2210013O21Rik | NM_027327.1 | chrX:153723553-153741296 |
| 501 | 2210015D19Rik | NR_015577.1 | chr11:5762149-5784710 |
| 502 | 2210016F16Rik | NM_027335.1 | chr13:58380045-58385225 |
| 503 | 2210016L21Rik | NM_028211.1 | chr5:114942201-114948540 |
| 504 | 2210018M11Rik | NM_172280.2 | chr7:98590605-98656569 |
| 505 | 2210019I11Rik | NR_038157.1 | chr5:147228597-147271521 |
| 506 | 2210039B01Rik | NR_044985.1 | chr12:73548482-73551986 |
| 507 | 2210404O09Rik | NM_001256493.1 | chr17:21879569-21909926 |
| 508 | 2210407C18Rik | NM_144544.2 | chr11:58608205-58613492 |
| 509 | 2210408F21Rik | NR_040257.1 | chr6:31220350-31337394 |
| 510 | 2210408I21Rik | NM_001081353.1 | chr13:77135535-77193905 |
| 511 | 2210409D07Rik | NR_045360.1 | chr18:57632078-57638585 |
| 512 | 2210409E12Rik | NR_003492.2 | chr11:88972637-88973014 |
| 513 | 2210414B05Rik | NR_040643.1 | chr4:3656505-3663108 |
| 514 | 2210416O15Rik | NR_045499.1 | chr11:88098057-88100107 |
| 515 | 2210417A02Rik | NR_028285.1 | chr5:148741839-148743139 |
| 516 | 2210420H20Rik | NR_045389.1 | chr18:82754504-82759039 |
| 517 | 2300002M23Rik | NM_175148.3 | chr17:35567484-35568945 |
| 518 | 2300003K06Rik | NM_001195383.1 | chr11:99837147-99838066 |
| 519 | 2300005B03Rik | NM_001081961.1 | chr15:74742838-74746687 |
| 520 | 2300009A05Rik | NM_027090.1 | chr9:63394446-63399244 |
| 521 | 2310001H17Rik | NR_040265.1 | chr6:129232622-129238045 |
| 522 | 2310001K24Rik | NR_028122.1 | chr2:163472544-163473003 |
| 523 | 2310002D06Rik | NR_045490.1 | chr12:80507205-80517954 |
| 524 | 2310002F09Rik | NR_077063.1 | chr7:43745465-43755936 |
| 525 | 2310002J15Rik | NM_026415.3 | chr2:25238818-25239897 |
| 526 | 2310002L09Rik | NM_027104.3 | chr4:73939370-73950846 |
| 527 | 2310003H01Rik | NM_027980.1 | chr11:120369561-120378746 |
| 528 | 2310005A03Rik | NR_040634.1 | chr2:155097790-155100079 |
| 529 | 2310005E17Rik | NR_045498.1 | chr13:98424109-98435065 |
| 530 | 2310005G13Rik | NM_183281.2 | chr16:57036966-57071346 |
| 531 | 2310007B03Rik | NM_001159940.1 | chr1:93151354-93160870 |
| 532 | 2310007L24Rik | NM_029345.1 | chr11:106374825-106377114 |
| 533 | 2310008N11Rik | NR_045904.1 | chr8:26814596-26824423 |
| 534 | 2310009A05Rik | NM_001308425.1 | chr9:73039718-73042775 |
| 535 | 2310009B15Rik | NM_001081226.1 | chr1:138851978-138856854 |
| 536 | 2310010J17Rik | NR_046006.1 | chr7:90124835-90129474 |
| 537 | 2310011J03Rik | NM_025521.3 | chr10:80318254-80320548 |
| 538 | 2310014L17Rik | NM_029809.2 | chr7:12927415-12931072 |
| 539 | 2310015A10Rik | NR_033514.1 | chr12:80120545-80132844 |
| 540 | 2310015B20Rik | NM_001304739.1 | chr10:70204667-70219711 |
| 541 | 2310015D24Rik | NR_037997.1 | chr16:13514130-13520419 |
| 542 | 2310016D03Rik | NR_045491.1 | chr12:30410558-30467358 |
| 543 | 2310020H05Rik | NR_045495.1 | chr13:99076527-99087921 |
| 544 | 2310022A10Rik | NM_001122767.1 | chr7:27560475-27582098 |
| 545 | 2310022B05Rik | NM_175149.4 | chr8:124635755-124663369 |
| 546 | 2310030A07Rik | NR_040603.1 | chr1:150320680-150346771 |
| 547 | 2310030G06Rik | NM_025865.2 | chr9:50739690-50746521 |
| 548 | 2310033P09Rik | NM_024210.2 | chr11:59208360-59210734 |
| 549 | 2310034C09Rik | NM_054100.2 | chr16:88758846-88759789 |
| 550 | 2310034G01Rik | NR_040418.1 | chr19:46348200-46348988 |
| 551 | 2310034O05Rik | NR_040679.1 | chr5:100210691-100218049 |
| 552 | 2310035C23Rik | NM_029349.1 | chr1:105663860-105755131 |
| 553 | 2310036O22Rik | NM_026760.2 | chr8:85026832-85030286 |
| 554 | 2310039H08Rik | NM_025966.3 | chr17:46772634-46773407 |
| 555 | 2310039L15Rik | NR_045337.1 | chr10:95336275-95363216 |
| 556 | 2310040G24Rik | NR_040292.1 | chr6:86483375-86488227 |
| 557 | 2310042E22Rik | NM_025634.3 | chr16:21152658-21153944 |
| 558 | 2310043L19Rik | NR_037994.1 | chr1:177641541-177642943 |
| 559 | 2310043O21Rik | NR_045757.1 | chr15:38549343-38594934 |
| 560 | 2310045N01Rik | NM_001145552.2 | chr8:70139498-70148634 |
| 561 | 2310047M10Rik | NM_028005.3 | chr11:69059774-69061576 |
| 562 | 2310050C09Rik | NM_025621.2 | chr3:92868358-92870205 |
| 563 | 2310057J18Rik | NM_026336.3 | chr10:28972287-28986306 |
| 564 | 2310057M21Rik | NM_026655.3 | chr7:131342717-131362698 |
| 565 | 2310057N15Rik | NM_027170.1 | chr16:88773180-88774206 |
| 566 | 2310061I04Rik | NM_001033630.1 | chr17:35892676-35897378 |
| 567 | 2310061J03Rik | NR_027965.1 | chr16:55973267-55974617 |
| 568 | 2310061N02Rik | NM_027155.1 | chr16:88707170-88707962 |
| 569 | 2310065F04Rik | NR_038055.1 | chr11:67112460-67120080 |
| 570 | 2310067B10Rik | NM_028014.3 | chr11:115765432-115799033 |
| 571 | 2310068J16Rik | NR_028124.1 | chr15:99972166-99973287 |
| 572 | 2310069B03Rik | NR_040520.1 | chr6:82877865-82881853 |
| 573 | 2310069G16Rik | NR_040309.1 | chr15:44787762-44805829 |
| 574 | 2310079G19Rik | NM_027173.1 | chr16:88626787-88627666 |
| 575 | 2310081J21Rik | NR_045474.1 | chr13:50261814-50680224 |
| 576 | 2410002F23Rik | NM_025880.4 | chr7:44246721-44252319 |
| 577 | 2410003L11Rik | NR_045496.1 | chr11:97598510-97622893 |
| 578 | 2410004B18Rik | NM_025555.4 | chr3:145938031-145944275 |
| 579 | 2410004I01Rik | NR_037963.1 | chr11:102958299-102964154 |
| 580 | 2410004N09Rik | NR_038151.1 | chr18:33794891-33795989 |
| 581 | 2410004P03Rik | NM_001201332.1 | chr12:17004957-17011727 |

Fig.21 - 4

| | | | | | | | |
|---|---|---|---|---|---|---|---|
| 582 | 2410006H16Rik | NR_030738.1 | chr11:62602876-62604806 | 679 | 2810410L24Rik | NR_030682.1 | chr11:120186649-120189856 |
| 583 | 2410007B07Rik | NR_040539.1 | chr3:75647440-75655731 | 680 | 2810417H13Rik | NM_026515.2 | chr9:65890322-65903551 |
| 584 | 2410012E07Rik | NR_045939.1 | chr14:70852932-70873954 | 681 | 2810428I15Rik | NM_025577.2 | chr8:70504295-70506739 |
| 585 | 2410012M07Rik | NM_028033.1 | chr9:98864766-98866580 | 682 | 2810429I04Rik | NR_015522.1 | chr13:3478302-3492398 |
| 586 | 2410015M20Rik | NM_153152.3 | chr17:56607451-56609771 | 683 | 2810433D01Rik | NR_033474.1 | chr11:102619506-102624416 |
| 587 | 2410016O06Rik | NM_023633.3 | chr12:83950607-83952953 | 684 | 2810442I21Rik | NR_110421.1 | chr11:16934708-16951282 |
| 588 | 2410017I17Rik | NR_033517.1 | chr17:36145017-36162374 | 685 | 2810442N19Rik | NR_040562.1 | chr1:162004968-162016485 |
| 589 | 2410018L13Rik | NR_015504.1 | chr12:22953996-23010243 | 686 | 2810454H06Rik | NR_029441.1 | chr6:134897960-134900785 |
| 590 | 2410021H03Rik | NR_045428.1 | chr17:69275361-69277206 | 687 | 2810459M11Rik | NM_001144992.1 | chr1:86045862-86055456 |
| 591 | 2410076I21Rik | NM_028598.1 | chr9:58652855-58741559 | 688 | 2810468N07Rik | NR_045176.1 | chr17:25570810-25575043 |
| 592 | 2410088K16Rik | NR_040493.1 | chr1:88754889-88755733 | 689 | 2810471M01Rik | NR_045906.1 | chr11:28681563-28693276 |
| 593 | 2410089E03Rik | NM_001162906.1 | chr15:8169105-8271158 | 690 | 2810474O19Rik | NM_001289661.1 | chr6:149309413-149335663 |
| 594 | 2410114N07Rik | NR_040652.1 | chr4:34909788-34911481 | 691 | 2900005J15Rik | NR_027851.1 | chr5:25100974-25103007 |
| 595 | 2410124H12Rik | NM_029740.1 | chr16:92478741-92497365 | 692 | 2900008C10Rik | NR_045434.1 | chrX:12134453-12160346 |
| 596 | 2410127L17Rik | NM_026120.4 | chr19:18670779-18704792 | 693 | 2900009J06Rik | NR_045298.1 | chr1:127753616-127774054 |
| 597 | 2410131K14Rik | NM_001081236.1 | chr5:118245226-118263114 | 694 | 2900011O08Rik | NM_144518.3 | chr16:13986636-14101494 |
| 598 | 2410137M14Rik | NM_029747.3 | chr17:36977700-36981237 | 695 | 2900026A02Rik | NM_172884.3 | chr5:113086322-113163313 |
| 599 | 2410141K09Rik | NM_001290196.1 | chr13:66431027-66441118 | 696 | 2900041M22Rik | NR_015489.2 | chr11:117611246-117613417 |
| 600 | 2500004C02Rik | NR_040318.1 | chr2:153341156-153345810 | 697 | 2900052N01Rik | NR_015605.1 | chr9:46913602-46927366 |
| 601 | 2510002D24Rik | NM_001033164.2 | chr16:18836579-18840113 | 698 | 2900055J20Rik | NR_045177.1 | chr18:40256961-40257687 |
| 602 | 2510003E04Rik | NM_028197.2 | chr10:62558469-62578457 | 699 | 2900056M20Rik | NR_040269.2 | chrX:152294823-152327493 |
| 603 | 2510009E07Rik | NM_001001881.2 | chr16:21649044-21694665 | 700 | 2900057B20Rik | NR_045365.1 | chr18:76091556-76098471 |
| 604 | 2510039O18Rik | NM_029841.3 | chr4:147940894-147947314 | 701 | 2900060B14Rik | NR_027901.1 | chr1:118458668-118459265 |
| 605 | 2510049J12Rik | NM_001101431.1 | chr6:115583546-115592576 | 702 | 2900076A07Rik | NR_045299.1 | chr7:81523549-81531498 |
| 606 | 2610001J05Rik | NR_024619.1 | chr6:13869073-13871483 | 703 | 2900079G21Rik | NR_015468.1 | chr9:112234380-112236018 |
| 607 | 2610002J02Rik | NM_001190445.1 | chr4:155249965-155256687 | 704 | 2900092C05Rik | NM_028434.3 | chr7:12512550-12556321 |
| 608 | 2610002M06Rik | NM_025921.3 | chrX:107782751-107816334 | 705 | 2900092D14Rik | NR_027891.1 | chr1:42700335-42703176 |
| 609 | 2610005L07Rik | NR_028428.1 | chr8:20385781-20424814 | 706 | 2900097C17Rik | NR_024329.1 | chr2:156388062-156392979 |
| 610 | 2610008E11Rik | NM_001004362.2 | chr10:79064373-79097600 | 707 | 3000002C10Rik | NR_033215.1 | chr9:109830153-109831431 |
| 611 | 2610015P09Rik | NM_027801.1 | chr16:43889901-43964314 | 708 | 3010001F23Rik | NR_045451.1 | chrX:152368571-152416702 |
| 612 | 2610016A17Rik | NR_045347.1 | chr19:25671133-25672239 | 709 | 3010026O09Rik | NM_026543.3 | chr11:50174850-50200115 |
| 613 | 2610018G03Rik | NM_133729.2 | chrX:50841370-50893098 | 710 | 3010033K07Rik | NR_077224.1 | chr8:108553251-108584677 |
| 614 | 2610020C07Rik | NR_038156.1 | chr16:11203382-11225796 | 711 | 3100003L05Rik | NR_045907.1 | chr7:124625669-124708935 |
| 615 | 2610020H08Rik | NM_001004187.1 | chr7:119794186-119848941 | 712 | 3110001I22Rik | NM_025653.2 | chr16:13672019-13678385 |
| 616 | 2610027K06Rik | NR_077059.1 | chr11:85791659-85832388 | 713 | 3110002H16Rik | NM_029623.2 | chr18:12168729-12189969 |
| 617 | 2610028E06Rik | NR_015560.1 | chr4:125887850-125922310 | 714 | 3110007F17Rik | NM_028426.2 | chrX:123103522-123145654 |
| 618 | 2610028H24Rik | NM_029816.2 | chr10:76449080-76461218 | 715 | 3110009E18Rik | NM_001172074.1 | chr1:120121186-120188189 |
| 619 | 2610034B18Rik | NM_027420.4 | chr7:79925358-79935264 | 716 | 3110009F21Rik | NR_045466.1 | chr12:110151653-110158073 |
| 620 | 2610034M16Rik | NM_027001.1 | chr17:58878807-58991375 | 717 | 3110015C05Rik | NR_045908.1 | chr13:111211468-111229431 |
| 621 | 2610035D17Rik | NR_015556.1 | chr11:113043905-113201838 | 718 | 3110021A11Rik | NR_030776.1 | chr6:119848192-119849016 |
| 622 | 2610035F20Rik | NR_045046.1 | chr14:122470376-122475199 | 719 | 3110021N24Rik | NM_001254730.1 | chr4:108719648-108781904 |
| 623 | 2610037D02Rik | NR_040423.1 | chr15:96190473-96197974 | 720 | 3110035E14Rik | NM_178399.4 | chr1:9601266-9627142 |
| 624 | 2610044O15Rik8 | NM_153780.3 | chr8:129216353-129234046 | 721 | 3110039I08Rik | NR_040725.2 | chr9:41376525-41432079 |
| 625 | 2610100L16Rik | NR_033490.1 | chr3:17789920-17800071 | 722 | 3110039M20Rik | NR_026733.1 | chr12:49389651-49407346 |
| 626 | 2610203C20Rik | NR_015483.3 | chr9:41581252-41592829 | 723 | 3110040N11Rik | NM_026077.3 | chr7:81782186-81789453 |
| 627 | 2610203C22Rik | NR_015470.1 | chr1:9560832-9631092 | 724 | 3110043O21Rik | NM_001081343.1 | chr4:35191281-35225880 |
| 628 | 2610206C17Rik | NR_038175.1 | chr7:84689639-84776549 | 725 | 3110045C21Rik | NR_040438.1 | chr1:169969408-169972396 |
| 629 | 2610207O16Rik | NR_110495.1 | chr1:42707062-42713454 | 726 | 3110052M02Rik | NM_001166497.1 | chr17:21650610-21664966 |
| 630 | 2610301B20Rik | NM_026005.3 | chr4:10874497-10899423 | 727 | 3110056K07Rik | NR_045055.1 | chr12:70991614-71015823 |
| 631 | 2610305D13Rik | NM_145078.2 | chr4:147611935-147642508 | 728 | 3110057O12Rik | NM_026622.3 | chr3:40894276-40936307 |
| 632 | 2610306M01Rik | NR_028298.1 | chr6:86848406-86849440 | 729 | 3110062M04Rik | NM_001135611.1 | chr6:34871770-34878065 |
| 633 | 2610307P16Rik | NR_045053.1 | chr13:28460033-28885422 | 730 | 3110070M22Rik | NR_027974.1 | chr13:119487256-119488384 |
| 634 | 2610316D01Rik | NR_045172.1 | chr3:45280438-45378260 | 731 | 3110079O15Rik | NM_028473.1 | chr1:87470263-87475459 |
| 635 | 2610318N02Rik | NM_183287.2 | chr16:17113397-17125106 | 732 | 3110082I17Rik | NM_028469.3 | chr5:139359738-139460534 |
| 636 | 2610507B11Rik | NM_001002004.2 | chr11:78261753-78290625 | 733 | 3110082J24Rik | NM_001256263.1 | chr5:30103583-30106086 |
| 637 | 2610507I01Rik | NR_037964.1 | chr11:59197792-59202431 | 734 | 3110099E03Rik | NR_030712.1 | chr2:115493512-115512201 |
| 638 | 2610524H06Rik | NM_181075.3 | chr5:114821936-114823468 | 735 | 3200001D21Rik | NR_045888.2 | chr12:88365313-88377323 |
| 639 | 2610528A11Rik | NM_001206684.1 | chr14:37102139-37110101 | 736 | 3300002I08Rik | NM_027017.1 | chr2:150310936-150362765 |
| 640 | 2610528J11Rik | NM_025572.2 | chr4:118527274-118530217 | 737 | 3300005D01Rik | NR_045079.1 | chr17:5799489-5803242 |
| 641 | 2700029M09Rik | NM_028299.1 | chr8:60890450-60907572 | 738 | 3425401B19Rik | NM_001195097.1 | chr14:32659118-32685272 |
| 642 | 2700038G22Rik | NR_045040.1 | chr5:23850596-23855033 | 739 | 3632451O06Rik | NM_026142.4 | chr14:49682018-49783367 |
| 643 | 2700046A07Rik | NR_037693.1 | chr18:62751673-62756347 | 740 | 3632454L22Rik | NR_040281.1 | chrX:135022421-135036602 |
| 644 | 2700046G09Rik | NR_033198.1 | chr19:32389215-32391184 | 741 | 3830403N18Rik | NM_027510.2 | chrX:56136571-56153496 |
| 645 | 2700049A03Rik | NM_001163378.1 | chr12:71136847-71243303 | 742 | 3830406C13Rik | NM_001284383.1 | chr14:12284202-12303231 |
| 646 | 2700054A10Rik | NR_045436.1 | chr17:13487020-13554094 | 743 | 3830408C21Rik | NR_015471.1 | chr13:107022569-107033518 |
| 647 | 2700060E02Rik | NM_026528.3 | chr14:19811401-19823823 | 744 | 3830417A13Rik | NM_027512.2 | chrX:64173547-64178778 |
| 648 | 2700062C07Rik | NM_026529.4 | chr18:24470870-24477767 | 745 | 3930402G23Rik | NR_030715.1 | chr8:10924426-10928457 |
| 649 | 2700069I18Rik | NR_045905.2 | chr3:5177823-5220823 | 746 | 4430402I18Rik | NM_198651.2 | chr19:28923063-28964154 |
| 650 | 2700070H01Rik | NR_046019.1 | chr14:63055072-63058149 | 747 | 4631405J19Rik | NR_110333.1 | chr2:93029347-93033445 |
| 651 | 2700081O15Rik | NM_175381.6 | chr19:7417624-7425904 | 748 | 4632415L05Rik | NR_027985.1 | chr3:19894870-19898984 |
| 652 | 2700086A05Rik | NR_015611.2 | chr6:52201123-52213597 | 749 | 4632427E13Rik | NR_015510.1 | chr7:92740705-92741459 |
| 653 | 2700089E24Rik | NM_001163445.2 | chr6:133105238-133107750 | 750 | 4632428C04Rik | NR_033631.1 | chr16:30008666-30021430 |
| 654 | 2700089I24Rik | NR_045308.1 | chr19:59493135-59559391 | 751 | 4632428N05Rik | NM_001159572.1 | chr10:60346850-60372684 |
| 655 | 2700094K13Rik | NM_001033166.2 | chr2:84669220-84670708 | 752 | 4632434I11Rik | NM_001080995.1 | chr7:92857526-92874232 |
| 656 | 2700097O09Rik | NM_028314.2 | chr12:55045660-55080110 | 753 | 4732416N19Rik | NR_015615.1 | chr6:148212287-148235663 |
| 657 | 2700099C18Rik | NR_024720.1 | chr17:94750099-94775129 | 754 | 4732456N10Rik | NM_177717.4 | chr15:101552355-101562950 |
| 658 | 2810001G20Rik | NR_033780.1 | chr11:64079483-64083259 | 755 | 4732471J01Rik | NR_015569.3 | chr7:25394712-25566417 |
| 659 | 2810002D19Rik | NR_027831.1 | chr2:94406706-94411680 | 756 | 4732490B19Rik | NR_040276.1 | chr11:113203314-113209669 |
| 660 | 2810004N23Rik | NM_025615.2 | chr8:124839354-124863029 | 757 | 4732491K20Rik | NR_045290.1 | chr17:12318852-12327250 |
| 661 | 2810006K23Rik | NM_001134717.2 | chr5:124328088-124341852 | 758 | 4831440E17Rik | NR_030700.1 | chr5:25499796-25504473 |
| 662 | 2810007J24Rik | NM_001199306.1 | chr7:14410685-14438551 | 759 | 4833403I15Rik | NM_029008.1 | chr18:46850038-46905446 |
| 663 | 2810008D09Rik | NR_027059.1 | chr11:117076782-117078955 | 760 | 4833411C07Rik | NR_102285.1 | chr8:10899921-10902334 |
| 664 | 2810013P06Rik | NR_045268.1 | chr8:123042574-123044602 | 761 | 4833412C05Rik | NR_045954.1 | chr7:67784537-67803496 |
| 665 | 2810021J22Rik | NM_172403.2 | chr1:58867239-58884338 | 762 | 4833417C18Rik | NR_045187.1 | chr11:95858816-95861046 |
| 666 | 2810025M15Rik | NR_027984.1 | chr1:157412351-157420236 | 763 | 4833418N02Rik | NR_015506.2 | chr17:87274885-87282814 |
| 667 | 2810029C07Rik | NR_045295.1 | chr12:111572316-111574402 | 764 | 4833419F23Rik | NR_040328.1 | chr18:4353546-4368945 |
| 668 | 2810032G03Rik | NR_015579.1 | chr12:5376501-5416132 | 765 | 4833420G17Rik | NM_001113550.1 | chr13:119462758-119486117 |
| 669 | 2810047C21Rik1 | NR_015598.1 | chr7:8086046-8093037 | 766 | 4833422C13Rik | NR_015501.1 | chr13:91701664-91741872 |
| 670 | 2810049E08Rik | NR_036594.1 | chr13:83891207-83928710 | 767 | 4833423E24Rik | NM_001081664.2 | chr2:85484091-85518935 |
| 671 | 2810055G20Rik | NR_015543.2 | chr16:77329327-77558428 | 768 | 4833424O15Rik | NM_029425.3 | chr3:117575226-117689508 |
| 672 | 2810403A07Rik | NM_028814.3 | chr3:88685793-88712933 | 769 | 4833427F10Rik | NR_045459.1 | chr17:35772449-35780687 |
| 673 | 2810403D21Rik | NR_015493.2 | chrX:108834477-108886896 | 770 | 4833427G06Rik | NM_177702.3 | chr9:51081312-51102078 |
| 674 | 2810404M03Rik | NR_045497.1 | chr8:41827266-42046202 | 771 | 4833428L15Rik | NR_040732.1 | chr9:45416625-45431232 |
| 675 | 2810405F15Rik | NR_033447.1 | chr2:116074547-116076096 | 772 | 4833439L19Rik | NM_001252645.1 | chr13:54551217-54565382 |
| 676 | 2810408A11Rik | NM_027419.3 | chr11:69897357-69900986 | 773 | 4921501E09Rik | NM_001009544.2 | chr17:33064142-33068058 |
| 677 | 2810408I11Rik | NR_038009.1 | chr1:64679868-64690659 | 774 | 4921504A21Rik | NR_102341.1 | chr5:19202367-19226555 |
| 678 | 2810408M09Rik | NM_001007581.1 | chr2:165490111-165493314 | 775 | 4921504E06Rik | NM_027600.4 | chr2:19462836-19553910 |

Fig.21 - 5

| | | | |
|---|---|---|---|
| 776 | 4921506M07Rik | NM_001312644.1 | chr12:57564115-57622105 |
| 777 | 4921507L20Rik | NR_045827.1 | chr2:93980033-93983575 |
| 778 | 4921507P07Rik | NM_027564.3 | chr6:50573303-50596590 |
| 779 | 4921508D12Rik | NR_045802.1 | chr2:132424740-132452499 |
| 780 | 4921509C19Rik | NM_198655.2 | chr2:151470541-151476151 |
| 781 | 4921509O07Rik | NR_045501.1 | chr13:113446287-113502844 |
| 782 | 4921511C10Rik | NR_045502.1 | chr3:79212918-79253913 |
| 783 | 4921511C20Rik | NR_003646.1 | chrX:127394292-127395898 |
| 784 | 4921511H03Rik | NM_027603.2 | chr5:7304163-7311491 |
| 785 | 4921511I17Rik | NR_045829.1 | chr12:12351521-12392475 |
| 786 | 4921511M17Rik | NM_001201358.1 | chrX:154001589-154120685 |
| 787 | 4921513I03Rik | NR_038000.1 | chr10:120765731-120778886 |
| 788 | 4921515E04Rik | NR_045711.1 | chr15:18078136-18369627 |
| 789 | 4921517D22Rik | NM_183290.2 | chr13:59687401-59694101 |
| 790 | 4921524J17Rik | NM_025722.3 | chr8:85408758-85432841 |
| 791 | 4921524L21Rik | NM_027598.1 | chr18:6603632-6638966 |
| 792 | 4921525O09Rik | NR_045061.1 | chr13:52096740-52124160 |
| 793 | 4921529L05Rik | NR_110484.1 | chr6:53978690-54017383 |
| 794 | 4921530L21Rik | NM_025733.2 | chr14:95881265-95882775 |
| 795 | 4921531C22Rik | NR_033782.1 | chr2:179976852-179979013 |
| 796 | 4921531P14Rik | NR_045361.1 | chr18:83292791-83325884 |
| 797 | 4921533I20Rik | NR_033607.1 | chr18:17234482-17235482 |
| 798 | 4921534H16Rik | NR_110443.1 | chr9:98003419-98012175 |
| 799 | 4921536K21Rik | NM_026150.3 | chr11:3886087-3895126 |
| 800 | 4921539E11Rik | NM_001163494.1 | chr4:103230444-103290863 |
| 801 | 4922502D21Rik | NM_199034.3 | chr6:129322164-129331781 |
| 802 | 4922502H24Rik | NR_046187.1 | chr13:101391870-101397580 |
| 803 | 4922502N22Rik | NR_045149.1 | chr6:139315596-139322576 |
| 804 | 4930401C15Rik | NR_045349.1 | chr10:25779313-25837437 |
| 805 | 4930401O10Rik | NR_045942.1 | chr11:71928470-71940122 |
| 806 | 4930401O12Rik | NR_045957.1 | chr13:31213409-31241086 |
| 807 | 4930402F06Rik | NM_001080709.2 | chr2:35375561-35397174 |
| 808 | 4930402F11Rik | NR_045940.1 | chr7:69496093-69499689 |
| 809 | 4930402H24Rik | NM_029432.2 | chr2:130706199-130840145 |
| 810 | 4930402K13Rik | NM_001270700.1 | chrX:9104561-9106342 |
| 811 | 4930404A05Rik | NR_040495.1 | chr16:52799507-52815744 |
| 812 | 4930404A10Rik | NM_029105.2 | chr11:54370651-54426790 |
| 813 | 4930404H11Rik | NR_045941.1 | chr12:71540606-71556133 |
| 814 | 4930404I05Rik | NR_028368.1 | chr16:91011248-91016503 |
| 815 | 4930404N11Rik | NM_001014836.3 | chr10:81364023-81365820 |
| 816 | 4930405A10Rik | NR_046307.1 | chr14:22507173-22542798 |
| 817 | 4930405A21Rik | NR_040505.1 | chr2:156714538-156720909 |
| 818 | 4930405D11Rik | NR_045956.1 | chr11:90804690-90895152 |
| 819 | 4930405J17Rik | NR_045350.1 | chr10:20199246-20201854 |
| 820 | 4930405L22Rik | NR_110478.1 | chr5:45926821-45932409 |
| 821 | 4930406D18Rik | NR_040543.1 | chr3:100426404-100437754 |
| 822 | 4930407I10Rik | NM_001166475.1 | chr15:82059150-82066538 |
| 823 | 4930412B13Rik | NR_040631.1 | chr2:117821927-117909136 |
| 824 | 4930412C18Rik | NR_030693.1 | chr4:9770584-9823411 |
| 825 | 4930412D23Rik | NM_001281537.1 | chrX:127721175-127736554 |
| 826 | 4930412O13Rik | NR_024257.1 | chr2:9881245-9886767 |
| 827 | 4930413E15Rik | NR_040694.1 | chr5:118952338-118961261 |
| 828 | 4930413F20Rik | NR_045883.2 | chr15:34675072-34679204 |
| 829 | 4930413G21Rik | NR_045614.1 | chr7:122969057-122970459 |
| 830 | 4930413M19Rik | NR_045759.1 | chr14:50503768-50520943 |
| 831 | 4930414L22Rik | NR_046011.1 | chr6:72438682-72440615 |
| 832 | 4930414N06Rik | NR_040294.1 | chr19:44972116-44984550 |
| 833 | 4930415F15Rik | NM_028669.1 | chr11:11489265-11515190 |
| 834 | 4930415L06Rik | NM_001033880.3 | chrX:89930096-89932852 |
| 835 | 4930415O20Rik | NM_001201322.1 | chr15:98571003-98589588 |
| 836 | 4930417O13Rik | NR_015527.1 | chr6:125265524-125273777 |
| 837 | 4930417O22Rik | NR_045806.1 | chr11:101952420-101960574 |
| 838 | 4930419G24Rik | NR_040595.1 | chr3:33024776-33088607 |
| 839 | 4930423M02Rik | NR_038183.1 | chr4:5632327-5642284 |
| 840 | 4930425K10Rik | NR_038182.1 | chr5:67933611-67943081 |
| 841 | 4930425O10Rik | NR_040545.1 | chr3:138711534-138726984 |
| 842 | 4930426D05Rik | NM_001271580.1 | chr18:21651564-21656107 |
| 843 | 4930426L09Rik | NR_024323.1 | chr2:18998318-18999804 |
| 844 | 4930427A07Rik | NM_134041.3 | chr12:113156420-113165458 |
| 845 | 4930428D18Rik | NM_001033799.2 | chrX:76393349-76397979 |
| 846 | 4930428E07Rik | NR_045943.1 | chr12:38552808-38614521 |
| 847 | 4930428G15Rik | NR_040730.1 | chr9:115336160-115341924 |
| 848 | 4930428O21Rik | NR_045871.1 | chr5:107698211-107703480 |
| 849 | 4930429B21Rik | NR_027966.1 | chr3:32365488-32367438 |
| 850 | 4930429D17Rik | NR_040699.1 | chr5:103230374-103299029 |
| 851 | 4930429F11Rik | NR_033463.1 | chr17:79475623-79477885 |
| 852 | 4930429F24Rik | NR_040734.1 | chr9:79793640-79803235 |
| 853 | 4930430A15Rik | NM_026248.3 | chr2:111193253-111229600 |
| 854 | 4930430D24Rik | NM_001034856.2 | chrX:38038198-38043114 |
| 855 | 4930430F08Rik | NM_175128.2 | chr10:100572273-100589259 |
| 856 | 4930430F21Rik | NR_045186.1 | chr15:31039125-31043331 |
| 857 | 4930430J02Rik | NR_040729.1 | chr9:57391069-57400364 |
| 858 | 4930431F12Rik | NR_073013.1 | chr5:44966200-44978805 |
| 859 | 4930431P03Rik | NR_045059.1 | chr14:44954899-44961307 |
| 860 | 4930432J09Rik | NR_045953.1 | chr14:103977627-104027142 |
| 861 | 4930432K21Rik | NM_001163752.1 | chr8:84148037-84172597 |
| 862 | 4930432M17Rik | NM_001033814.1 | chr3:121670762-121682982 |
| 863 | 4930433B08Rik | NR_040544.1 | chr3:18468284-18480477 |
| 864 | 4930433I11Rik | NM_207248.3 | chr7:40987608-40994833 |
| 865 | 4930433N12Rik | NR_027988.1 | chr9:3190268-3199813 |
| 866 | 4930434J06Rik | NR_046274.1 | chr14:71856708-71920438 |
| 867 | 4930435E12Rik | NM_029042.1 | chr16:38812204-38828749 |
| 868 | 4930438E09Rik | NR_045961.1 | chr14:67332041-67397742 |
| 869 | 4930440C22Rik | NR_040473.1 | chr1:94797441-94802472 |
| 870 | 4930440I19Rik | NR_108098.1 | chr2:78051176-78194430 |
| 871 | 4930441J16Rik | NR_040501.1 | chr2:74298939-74307717 |
| 872 | 4930442J19Rik | NR_040747.1 | chr2:151302038-151316003 |
| 873 | 4930442L01Rik | NR_015596.1 | chr3:96829825-96832070 |
| 874 | 4930443O20Rik | NR_040504.1 | chr2:85073613-85086461 |
| 875 | 4930444F02Rik | NR_038034.1 | chr10:18801085-18831930 |
| 876 | 4930444G20Rik | NM_053264.2 | chr10:22066308-22068079 |
| 877 | 4930444M15Rik | NR_045660.1 | chr14:76514557-76520855 |
| 878 | 4930444P10Rik | NM_001243238.2 | chr1:16065978-16093325 |
| 879 | 4930447A16Rik | NM_029113.1 | chr15:37425553-37440644 |
| 880 | 4930447C04Rik | NM_029444.2 | chr12:72881108-72917765 |
| 881 | 4930447J18Rik | NR_045959.1 | chr14:47898863-47944189 |
| 882 | 4930447K03Rik | NR_046184.1 | chr13:34084154-34113272 |
| 883 | 4930447N08Rik | NR_045168.1 | chr3:122795090-122801980 |
| 884 | 4930448C13Rik | NR_045960.1 | chr12:14996388-15050389 |
| 885 | 4930448F12Rik | NR_046032.1 | chr13:18084946-18100202 |
| 886 | 4930448H16Rik | NR_040700.1 | chr5:143226991-143234748 |
| 887 | 4930448I06Rik | NR_040475.1 | chr1:41167559-41181252 |
| 888 | 4930448I18Rik | NR_040696.1 | chr5:50151332-50156092 |
| 889 | 4930448K20Rik | NR_004448.2 | chr4:9915964-9917397 |
| 890 | 4930449E01Rik | NR_045921.1 | chr14:105498787-105505628 |
| 891 | 4930449E18Rik | NR_045319.1 | chr19:57273959-57277052 |
| 892 | 4930449I24Rik | NM_026136.2 | chr5:146502398-146505183 |
| 893 | 4930451C15Rik | NM_001145435.1 | chr16:17544464-17561247 |
| 894 | 4930451G09Rik | NM_001271586.1 | chr16:4964287-4978054 |
| 895 | 4930451I11Rik | NM_183131.2 | chr7:126830473-126831496 |
| 896 | 4930452A19Rik | NR_045433.1 | chr17:9750751-9775866 |
| 897 | 4930452B06Rik | NM_028934.3 | chr14:8431171-8666290 |
| 898 | 4930452G13Rik | NR_045060.1 | chr14:79702919-79712930 |
| 899 | 4930452N14Rik | NR_040629.1 | chr1:154241520-154256906 |
| 900 | 4930453H23Rik | NM_001252013.1 | chrX:21206579-21221665 |
| 901 | 4930453L07Rik | NR_073360.1 | chr8:9144668-9149050 |
| 902 | 4930453N24Rik | NM_026273.2 | chr16:64766104-64770939 |
| 903 | 4930455B14Rik | NR_045968.1 | chr14:8666389-8673378 |
| 904 | 4930455C13Rik | NR_045352.1 | chr10:21319087-21342055 |
| 905 | 4930455D15Rik | NR_045381.1 | chr18:32663641-32837287 |
| 906 | 4930455F16Rik | NR_040570.1 | chr16:4219911-4229116 |
| 907 | 4930455H04Rik | NR_040596.1 | chr3:116968266-116984406 |
| 908 | 4930455J16Rik | NR_045469.1 | chr13:58741073-58762311 |
| 909 | 4930456L15Rik | NR_045887.1 | chr4:103290958-103311832 |
| 910 | 4930459C07Rik | NR_110448.1 | chr10:96226206-96248708 |
| 911 | 4930459L07Rik | NR_046190.1 | chr5:58443239-58452998 |
| 912 | 4930461G14Rik | NR_040736.1 | chr9:58455172-58469623 |
| 913 | 4930463O16Rik | NR_108059.1 | chr10:84488292-84497676 |
| 914 | 4930465K10Rik | NR_027978.1 | chr18:77714183-77715445 |
| 915 | 4930465M20Rik | NR_045973.1 | chr12:107723742-107735488 |
| 916 | 4930467D21Rik | NR_045981.1 | chr5:97392416-97588126 |
| 917 | 4930467E23Rik | NM_001039553.2 | chr8:19729575-19753602 |
| 918 | 4930467K11Rik | NR_045353.1 | chr10:57478382-57486353 |
| 919 | 4930468A15Rik | NM_001201395.1 | chrX:76582609-76602924 |
| 920 | 4930469G21Rik | NM_001195189.1 | chr1:161156842-161159314 |
| 921 | 4930470H14Rik | NR_045764.1 | chr17:4044657-4082995 |
| 922 | 4930470P17Rik | NR_027825.1 | chr2:170579417-170602017 |
| 923 | 4930471C04Rik | NR_046192.1 | chr14:64099294-64104235 |
| 924 | 4930471G03Rik | NR_015571.1 | chr18:36106202-36109954 |
| 925 | 4930471M09Rik | NR_045980.1 | chr6:91430459-91440854 |
| 926 | 4930473A02Rik | NR_040348.1 | chr2:130543790-130563749 |
| 927 | 4930473O22Rik | NR_045356.1 | chr10:97590983-97596931 |
| 928 | 4930474G06Rik | NR_045398.1 | chr18:28560163-28998176 |
| 929 | 4930474H20Rik | NR_045712.1 | chr14:90126890-90269760 |
| 930 | 4930474M22Rik | NR_027986.1 | chr17:14389106-14398942 |
| 931 | 4930474N05Rik | NM_175008.3 | chr14:36094964-36096855 |
| 932 | 4930474N09Rik | NR_038130.1 | chr12:99161276-99162808 |
| 933 | 4930478L05Rik | NR_040569.1 | chr16:78560315-78564869 |
| 934 | 4930478P22Rik | NR_046060.1 | chr5:35361552-35367677 |
| 935 | 4930479D17Rik | NR_046277.1 | chr6:146657217-146665403 |
| 936 | 4930480E11Rik | NM_001177966.2 | chrX:78369642-78371128 |
| 937 | 4930480G23Rik | NR_040768.1 | chr4:19979444-20005739 |
| 938 | 4930480K15Rik | NR_045463.1 | chr17:90861168-90867102 |
| 939 | 4930480M12Rik | NR_046278.1 | chr12:26211266-26240707 |
| 940 | 4930481A15Rik | NR_015545.2 | chr19:5406814-5422847 |
| 941 | 4930482G09Rik | NR_108044.1 | chr3:153147942-153152108 |
| 942 | 4930483J18Rik | NR_015603.1 | chr15:81190852-81192757 |
| 943 | 4930483K19Rik | NR_045354.1 | chr10:76545870-76562226 |
| 944 | 4930483O08Rik | NR_046279.1 | chr7:133128250-133142457 |
| 945 | 4930486F22Rik | NR_038024.1 | chr10:86095853-86113585 |
| 946 | 4930486I03Rik | NR_045734.1 | chr1:20356453-20392300 |
| 947 | 4930486L24Rik | NM_178098.2 | chr13:60842611-60864416 |
| 948 | 4930487D11Rik | NR_046191.1 | chr5:38344388-38349197 |
| 949 | 4930487H11Rik | NR_040601.1 | chr1:62790917-62794732 |
| 950 | 4930488B22Rik | NR_040627.1 | chr1:184712496-184718989 |
| 951 | 4930488L21Rik | NR_026888.1 | chr8:93811693-93813555 |
| 952 | 4930500F04Rik | NR_045758.1 | chr5:149847746-149864280 |
| 953 | 4930500J02Rik | NR_040322.1 | chr2:104559183-104571429 |
| 954 | 4930500L23Rik | NR_040701.1 | chr5:139523750-139541339 |
| 955 | 4930502A04Rik | NR_040737.1 | chr9:68470901-68520200 |
| 956 | 4930502E09Rik | NR_046281.1 | chr11:84829177-84830868 |
| 957 | 4930502E18Rik | NM_029142.1 | chrX:53724830-53738222 |
| 958 | 4930503B20Rik | NM_029144.3 | chr3:146646258-146651317 |
| 959 | 4930503E14Rik | NM_029131.3 | chr14:44163166-44171371 |
| 960 | 4930503E24Rik | NR_028310.1 | chr10:124469073-124524488 |
| 961 | 4930503H13Rik | NR_033598.1 | chrX:156539482-156540117 |
| 962 | 4930503L19Rik | NM_172967.2 | chr18:70453139-70472480 |
| 963 | 4930503O07Rik | NR_040477.1 | chr1:194212186-194222759 |
| 964 | 4930504O13Rik | NM_207527.3 | chr11:58446142-58452966 |
| 965 | 4930505A04Rik | NM_001100394.1 | chr11:30426005-30471829 |
| 966 | 4930505G20Rik | NR_045761.1 | chr14:115396550-115409552 |
| 967 | 4930506C21Rik | NR_073374.1 | chr17:8293365-8311118 |
| 968 | 4930506M07Rik | NM_001114312.1 | chr19:58973357-59076069 |
| 969 | 4930507D05Rik | NR_027926.1 | chr10:62449159-62451353 |

Fig.21 - 6

| | | | |
|---|---|---|---|
| 970 | 4930507D10Rik | NR_110449.1 | chr11:80850380-80858300 |
| 971 | 4930509E16Rik | NR_045735.1 | chr9:72518249-72531858 |
| 972 | 4930509J09Rik | NR_040547.1 | chr3:73351205-73481651 |
| 973 | 4930509K18Rik | NR_040663.1 | chr4:40313231-40317719 |
| 974 | 4930511A02Rik | NR_045966.1 | chr12:11438232-11444812 |
| 975 | 4930511E03Rik | NR_040761.1 | chr6:94943833-94951545 |
| 976 | 4930511M06Rik | NR_015494.1 | chr18:57546013-57552397 |
| 977 | 4930512B01Rik | NR_033573.1 | chr12:69790343-69841880 |
| 978 | 4930513D17Rik | NR_045416.1 | chr5:39461748-39603574 |
| 979 | 4930513N10Rik | NR_015574.2 | chr8:95806829-95821728 |
| 980 | 4930513O06Rik | NM_029174.1 | chrX:139086242-139093403 |
| 981 | 4930515B02Rik | NR_040654.1 | chr4:140872692-140878057 |
| 982 | 4930515G01Rik | NR_027872.1 | chr5:114773732-114774983 |
| 983 | 4930515G16Rik | NR_003100.1 | chr6:67565233-67565699 |
| 984 | 4930515L03Rik | NR_040632.1 | chr2:16918677-17013726 |
| 985 | 4930515L19Rik | NR_045440.1 | chrX:46267558-46328911 |
| 986 | 4930517E11Rik | NR_040611.1 | chr2:124123635-124129956 |
| 987 | 4930518P08Rik | NR_045364.1 | chr13:50753392-50765248 |
| 988 | 4930519D14Rik | NR_045975.1 | chr13:30615042-30624258 |
| 989 | 4930519F09Rik | NR_033601.1 | chr10:28921962-28923507 |
| 990 | 4930519F16Rik | NM_029170.1 | chrX:103232280-103256606 |
| 991 | 4930519F24Rik | NR_040763.1 | chr9:100022299-100034670 |
| 992 | 4930519G04Rik | NM_026263.2 | chr5:114853713-114883879 |
| 993 | 4930519H02Rik | NR_045974.1 | chr5:15863927-15883766 |
| 994 | 4930520O04Rik | NR_040383.1 | chr9:114368323-114377238 |
| 995 | 4930520P13Rik | NR_036596.1 | chr13:70232821-70248122 |
| 996 | 4930521E06Rik | NR_040602.1 | chr1:44739674-44744949 |
| 997 | 4930522H14Rik | NM_001199090.1 | chr4:109505331-109531204 |
| 998 | 4930522O17Rik | NR_040665.1 | chr4:53246195-53251120 |
| 999 | 4930523C07Rik | NM_001162896.1 | chr1:160044379-160078586 |
| 1000 | 4930523O13Rik | NR_040451.1 | chr15:46435865-46496179 |
| 1001 | 4930524B15Rik | NM_026262.1 | chr11:31965632-31979651 |
| 1002 | 4930524C18Rik | NR_045661.1 | chr14:114831964-114861327 |
| 1003 | 4930524N10Rik | NM_001256259.1 | chrX:154339224-154343392 |
| 1004 | 4930524O05Rik | NR_045316.1 | chr19:10645693-10648053 |
| 1005 | 4930524O08Rik | NR_040735.1 | chr9:89826518-89864028 |
| 1006 | 4930525D18Rik | NR_040738.1 | chr9:114341110-114364627 |
| 1007 | 4930525G20Rik | NR_045194.1 | chr13:67796593-67830985 |
| 1008 | 4930525M21Rik | NM_001243943.1 | chrX:38013907-38018815 |
| 1009 | 4930526I15Rik | NR_015516.2 | chr9:124423786-124424856 |
| 1010 | 4930526L06Rik | NR_045317.1 | chr19:11196816-11299884 |
| 1011 | 4930527F14Rik | NR_045809.1 | chr14:45543709-45562876 |
| 1012 | 4930527G23Rik | NR_045395.1 | chr18:29442199-29466107 |
| 1013 | 4930528A17Rik | NR_028384.1 | chr4:21846326-21848448 |
| 1014 | 4930528D03Rik | NR_045977.1 | chr13:59733649-59737470 |
| 1015 | 4930528P14Rik | NR_040516.1 | chr2:115329265-115344280 |
| 1016 | 4930529C04Rik | NR_033593.1 | chr3:91071661-91075192 |
| 1017 | 4930529K09Rik | NR_040457.1 | chr14:86245969-86262042 |
| 1018 | 4930529L06Rik | NR_040537.1 | chr16:84679788-84685147 |
| 1019 | 4930529M08Rik | NM_175280.3 | chr2:145934783-145964226 |
| 1020 | 4930532M18Rik | NR_108050.1 | chr1:154255688-154270416 |
| 1021 | 4930533B01Rik | NR_040614.1 | chr2:113691052-113708550 |
| 1022 | 4930533P14Rik | NR_040478.1 | chr1:96612056-96662573 |
| 1023 | 4930538K18Rik | NM_029198.3 | chr4:119205054-119218217 |
| 1024 | 4930539C22Rik | NR_040600.1 | chr3:134404142-134423635 |
| 1025 | 4930539E08Rik | NM_172450.3 | chr17:28896394-28915324 |
| 1026 | 4930539J05Rik | NR_030689.1 | chr3:135436220-135438665 |
| 1027 | 4930539M17Rik | NR_040597.1 | chr3:9061963-9071076 |
| 1028 | 4930539N22Rik | NR_040598.1 | chr3:13597138-13652865 |
| 1029 | 4930540M03Rik | NR_040746.1 | chr9:15619856-15641220 |
| 1030 | 4930542C21Rik | NR_040565.1 | chr16:38017322-38054526 |
| 1031 | 4930542D17Rik | NR_040566.1 | chr16:50590502-50654166 |
| 1032 | 4930543E12Rik | NR_045978.1 | chr7:113100235-113142015 |
| 1033 | 4930544D05Rik | NM_001145537.1 | chr11:70615893-70616890 |
| 1034 | 4930544G11Rik | NM_001161773.1 | chr6:65952570-65954014 |
| 1035 | 4930544M13Rik | NR_045976.1 | chr13:114607227-114697822 |
| 1036 | 4930545E07Rik | NR_045374.1 | chr18:17568745-17580990 |
| 1037 | 4930545H06Rik | NR_045357.1 | chr10:68319064-68321142 |
| 1038 | 4930545L23Rik | NR_040517.1 | chr2:135169572-135215616 |
| 1039 | 4930546C10Rik | NR_038051.1 | chr18:68889486-68951528 |
| 1040 | 4930546K05Rik | NR_040754.1 | chr9:42182237-42209231 |
| 1041 | 4930547E08Rik | NR_040514.1 | chr2:103804451-103810622 |
| 1042 | 4930547E14Rik | NR_040564.1 | chr16:59636944-59672993 |
| 1043 | 4930548G14Rik | NR_045811.1 | chr15:46623304-46639858 |
| 1044 | 4930548H24Rik | NM_026296.3 | chr5:31485858-31488262 |
| 1045 | 4930548J01Rik | NR_045462.1 | chr17:4119445-4122102 |
| 1046 | 4930548K13Rik | NR_040656.1 | chr4:26635819-26705449 |
| 1047 | 4930549C01Rik | NM_026300.2 | chr4:136610436-136613213 |
| 1048 | 4930549G23Rik | NR_045376.1 | chr18:67776032-67799943 |
| 1049 | 4930550C14Rik | NM_029247.3 | chr9:53405285-53432803 |
| 1050 | 4930550L24Rik | NM_023774.3 | chrX:58911460-58920304 |
| 1051 | 4930552N02Rik | NR_040661.1 | chr4:52814603-52828269 |
| 1052 | 4930552P12Rik | NR_045318.1 | chr19:55640588-55702204 |
| 1053 | 4930553E22Rik | NR_040567.1 | chr16:84371142-84375505 |
| 1054 | 4930554C24Rik | NR_040739.1 | chr9:84774397-84784351 |
| 1055 | 4930555B11Rik | NR_040633.1 | chr2:57181830-57188257 |
| 1056 | 4930555G01Rik | NM_175393.4 | chr14:5050628-5059243 |
| 1057 | 4930556C24Rik | NR_040506.1 | chr16:85819210-85826772 |
| 1058 | 4930556G01Rik | NR_040655.1 | chr4:30664595-30855744 |
| 1059 | 4930556J02Rik | NR_045714.1 | chr14:61492687-61500382 |
| 1060 | 4930556M19Rik | NR_045063.1 | chr15:10714835-10790123 |
| 1061 | 4930556N09Rik | NR_045358.1 | chr10:97035767-97061059 |
| 1062 | 4930557A04Rik | NM_029229.1 | chrX:9849702-9850252 |
| 1063 | 4930557J02Rik | NR_040703.1 | chr5:34118739-34135339 |
| 1064 | 4930558C23Rik | NR_015490.1 | chr3:95386457-95402841 |
| 1065 | 4930558G05Rik | NR_045441.1 | chrX:129051523-129067146 |
| 1066 | 4930558J18Rik | NR_037999.1 | chr1:57359221-57377544 |
| 1067 | 4930558K02Rik | NM_001204904.1 | chr1:161942088-161979636 |
| 1068 | 4930562C15Rik | NM_030192.1 | chr16:4835415-4867691 |
| 1069 | 4930562F07Rik | NR_038030.1 | chr1:160074734-160077918 |
| 1070 | 4930563D23Rik | NM_029252.2 | chr16:92318762-92321441 |
| 1071 | 4930563E18Rik | NR_045379.1 | chr18:10706859-10711832 |
| 1072 | 4930563E22Rik | NM_001163728.1 | chr11:72215137-72218450 |
| 1073 | 4930563F08Rik | NR_040704.1 | chr5:131880268-131883768 |
| 1074 | 4930563M20Rik | NR_046193.1 | chr8:116781711-116801412 |
| 1075 | 4930564B18Rik | NM_029230.1 | chr14:75642258-75667137 |
| 1076 | 4930564C03Rik | NM_029257.1 | chr17:44879703-44880885 |
| 1077 | 4930564D02Rik | NM_029228.1 | chr3:105066843-105078806 |
| 1078 | 4930565D16Rik | NR_040752.1 | chr3:84349375-84379921 |
| 1079 | 4930565N06Rik | NR_040476.1 | chr16:36880518-36897189 |
| 1080 | 4930567H12Rik | NR_015535.2 | chr8:125584637-125616670 |
| 1081 | 4930567H17Rik | NM_001033807.2 | chrX:70393900-70394740 |
| 1082 | 4930567J20Rik | NR_040576.1 | chr16:71249903-71267176 |
| 1083 | 4930567K20Rik | NR_046010.1 | chr10:10751045-10758131 |
| 1084 | 4930568D16Rik | NM_029463.1 | chr2:35354217-35367729 |
| 1085 | 4930568E12Rik | NR_040755.1 | chr9:13091266-13105855 |
| 1086 | 4930568G15Rik | NR_040480.1 | chr1:165066616-165091930 |
| 1087 | 4930570G19Rik | NR_040398.1 | chr3:156548414-156561687 |
| 1088 | 4930571K23Rik | NM_001145759.1 | chr7:125368860-125371013 |
| 1089 | 4930571O06Rik | NR_110476.1 | chr8:113517281-113550808 |
| 1090 | 4930572K03Rik | NR_045371.1 | chr5:127170564-127184356 |
| 1091 | 4930572O03Rik | NR_073011.1 | chr5:15652287-15657059 |
| 1092 | 4930572O13Rik | NR_045718.1 | chr14:25139794-25143241 |
| 1093 | 4930573O16Rik | NR_040620.1 | chr2:52291001-52315240 |
| 1094 | 4930577N17Rik | NR_073429.1 | chr3:51276760-51278407 |
| 1095 | 4930578C19Rik | NM_175228.2 | chrX:18418426-18461397 |
| 1096 | 4930578E11Rik | NR_045391.1 | chr18:29396715-29496416 |
| 1097 | 4930578I06Rik | NM_026359.3 | chr14:63971121-63987780 |
| 1098 | 4930578M01Rik | NR_045991.1 | chr15:98985964-98989281 |
| 1099 | 4930578N18Rik | NR_040575.1 | chr16:76122612-76156086 |
| 1100 | 4930579F01Rik | NM_001163385.1 | chr3:138164134-138186713 |
| 1101 | 4930579G18Rik | NR_038053.1 | chr14:54650510-54655100 |
| 1102 | 4930579G24Rik | NM_029482.1 | chr3:79629078-79632819 |
| 1103 | 4930579K19Rik | NR_029444.1 | chr9:98562459-98563611 |
| 1104 | 4930581F22Rik | NR_029475.1 | chr9:35116727-35130922 |
| 1105 | 4930583K01Rik | NR_027879.1 | chr7:118243906-118245551 |
| 1106 | 4930583P06Rik | NR_040612.1 | chr2:124217678-124222358 |
| 1107 | 4930584F24Rik | NR_029445.1 | chr5:26460069-26493314 |
| 1108 | 4930590J08Rik | NM_198668.2 | chr6:91904232-91950725 |
| 1109 | 4930590L20Rik | NR_040604.1 | chr1:140570175-140656402 |
| 1110 | 4930591A17Rik | NM_026596.2 | chr2:179414935-179416880 |
| 1111 | 4930592A05Rik | NR_045070.1 | chr15:33593880-33653436 |
| 1112 | 4930592I03Rik | NR_033307.1 | chr18:82918404-82920560 |
| 1113 | 4930593A02Rik | NR_045167.1 | chr3:58692588-58788524 |
| 1114 | 4930593C16Rik | NR_040753.1 | chr9:120924454-120930802 |
| 1115 | 4930594C11Rik | NR_024017.1 | chr1:37485850-37488605 |
| 1116 | 4930595M18Rik | NM_173435.3 | chrX:81419574-81458122 |
| 1117 | 4930596D02Rik | NM_001033766.3 | chr14:35809485-35811978 |
| 1118 | 4930596I21Rik | NR_108103.1 | chr1:138977656-138979246 |
| 1119 | 4930597G03Rik | NR_045736.1 | chr14:51317334-51345489 |
| 1120 | 4930598F16Rik | NR_040479.1 | chr1:95701183-95716518 |
| 1121 | 4930599N23Rik | NR_045813.1 | chr6:39118472-39148312 |
| 1122 | 4931402G19Rik | NR_040608.1 | chr2:120455697-120469367 |
| 1123 | 4931403E22Rik | NR_045306.1 | chr19:26768800-26823907 |
| 1124 | 4931403G20Rik | NR_038172.1 | chr12:69843685-69853671 |
| 1125 | 4931406B18Rik | NM_028737.3 | chr7:43492043-43505938 |
| 1126 | 4931406C07Rik | NM_001199484.1 | chr9:15283336-15306448 |
| 1127 | 4931406H21Rik | NR_033492.1 | chr14:25586803-25590661 |
| 1128 | 4931406P16Rik | NM_172741.2 | chr7:34236713-34285609 |
| 1129 | 4931408C20Rik | NM_001033764.3 | chr1:26681800-26687460 |
| 1130 | 4931408D14Rik | NR_040298.1 | chr19:37258425-37264095 |
| 1131 | 4931409K22Rik | NM_177676.6 | chr5:24543433-24555469 |
| 1132 | 4931412M21 | NR_110487.1 | chr12:10446387-10531303 |
| 1133 | 4931414P19Rik | NM_028890.2 | chr14:54583662-54605908 |
| 1134 | 4931417E11Rik | NM_025737.3 | chr6:73468572-73469667 |
| 1135 | 4931419H13Rik | NR_040593.1 | chr3:55055241-55084002 |
| 1136 | 4931420L22Rik | NR_040561.1 | chr16:71131075-71139029 |
| 1137 | 4931423N10Rik | NM_027635.1 | chr2:23207475-23267129 |
| 1138 | 4931428F04Rik | NM_001166394.1 | chr8:105280408-105289528 |
| 1139 | 4931428L18Rik | NR_033445.1 | chr1:31141077-31222656 |
| 1140 | 4931429I11Rik | NM_001081121.1 | chr9:40894848-40964112 |
| 1141 | 4931429L15Rik | NM_183104.2 | chr9:46303360-46319986 |
| 1142 | 4931429P17Rik | NR_038004.1 | chr13:47960329-48018608 |
| 1143 | 4931430N09Rik | NR_046053.1 | chr6:118880157-118885543 |
| 1144 | 4931431B13Rik | NR_045183.1 | chr7:128192049-128203328 |
| 1145 | 4931431C16Rik | NR_045807.1 | chr5:35581226-35588763 |
| 1146 | 4931431F19Rik | NM_027634.1 | chr7:104127912-104129823 |
| 1147 | 4931440F15Rik | NM_176829.2 | chr11:29822394-29825668 |
| 1148 | 4931440J10Rik | NR_045503.1 | chr14:62760500-62830126 |
| 1149 | 4931440L10Rik | NM_001145300.2 | chr1:134540940-134549682 |
| 1150 | 4931440P22Rik | NR_027955.1 | chr3:65527484-65529414 |
| 1151 | 4932411E22Rik | NM_172534.2 | chr11:89390222-89418948 |
| 1152 | 4932411N23Rik | NM_177705.3 | chrX:126812461-126834004 |
| 1153 | 4932412D23Rik | NR_040521.1 | chr16:42725701-42875587 |
| 1154 | 4932413F04Rik | NR_040764.1 | chr9:103557089-103565891 |
| 1155 | 4932414J04Rik | NR_028259.1 | chr11:21494729-21511180 |
| 1156 | 4932414N04Rik | NM_183113.3 | chr2:68657883-68748465 |
| 1157 | 4932415M13Rik | NR_073205.1 | chr17:53716508-53733843 |
| 1158 | 4932416H05Rik | NR_029452.1 | chr2:129798368-129801459 |
| 1159 | 4932416K20Rik | NM_001002775.2 | chr8:104795236-104798792 |
| 1160 | 4932418E24Rik | NM_177841.3 | chr2:26271646-26294557 |
| 1161 | 4932429P05Rik | NM_001085511.1 | chrX:89752267-89755491 |
| 1162 | 4932435O22Rik | NR_027643.2 | chr11:114413404-114448860 |
| 1163 | 4932438A13Rik | NM_172679.2 | chr3:36863105-37053033 |

Fig.21 - 7

| | | | | | | | |
|---|---|---|---|---|---|---|---|
| 1164 | 4932438H23Rik | NM_001163695.1 | chr16:91053934-91069145 | 1261 | 4933432I09Rik | NR_015575.1 | chr16:18209685-18213863 |
| 1165 | 4932441J04Rik | NR_015588.2 | chr5:57570083-57717919 | 1262 | 4933432K03Rik | NR_102286.1 | chr7:121587385-121589836 |
| 1166 | 4932443I19Rik | NM_001101519.1 | chr8:13705888-13743066 | 1263 | 4933433C11Rik | NM_028961.1 | chr2:25212558-25214630 |
| 1167 | 4932702P03Rik | NR_045182.1 | chr13:24905599-24911780 | 1264 | 4933433F19Rik | NR_045856.1 | chr8:30568394-30575573 |
| 1168 | 4933400A11Rik | NR_003635.1 | chrX:169776377-169779635 | 1265 | 4933433G08Rik | NR_105026.1 | chr9:61103011-61105386 |
| 1169 | 4933400B14Rik | NR_045432.1 | chr17:89320226-89327333 | 1266 | 4933433G15Rik | NR_040719.1 | chr9:75410183-75415537 |
| 1170 | 4933400C23Rik | NR_040770.1 | chr9:92718715-92795711 | 1267 | 4933433G19Rik | NR_045851.1 | chr13:66996817-67032405 |
| 1171 | 4933400F21Rik | NR_015540.1 | chr1:89676506-89683892 | 1268 | 4933433H22Rik | NR_045458.1 | chr17:84078659-84089875 |
| 1172 | 4933400L20Rik | NR_045730.1 | chr8:103603798-103680382 | 1269 | 4933434E20Rik | NM_001287086.1 | chr3:90052801-90063341 |
| 1173 | 4933401B06Rik | NR_033580.1 | chrX:107542581-107543800 | 1270 | 4933434I20Rik | NM_026233.2 | chr8:83348471-83379402 |
| 1174 | 4933401D09Rik | NR_045431.1 | chr17:15631787-15641102 | 1271 | 4933436E23Rik | NR_040455.1 | chr1:139379451-139402852 |
| 1175 | 4933401H06Rik | NR_040540.1 | chr3:135833693-135840269 | 1272 | 4933436H12Rik | NR_046208.1 | chr7:68417423-68426133 |
| 1176 | 4933402C06Rik | NR_045504.1 | chr7:40674736-40691260 | 1273 | 4933436I01Rik | NM_025763.1 | chrX:67919863-67921450 |
| 1177 | 4933402D24Rik | NM_001256158.1 | chr1:63754908-63769267 | 1274 | 4933438B17Rik | NR_040685.1 | chr5:127030788-127088529 |
| 1178 | 4933402E13Rik | NM_001199996.1 | chrX:62287141-62292078 | 1275 | 4933438K21Rik | NR_045446.1 | chr4:147063365-147068436 |
| 1179 | 4933402J07Rik | NM_177901.3 | chr8:87563906-87589197 | 1276 | 4933439C10Rik | NR_015585.2 | chr11:59505684-59511067 |
| 1180 | 4933402J10Rik | NR_040682.1 | chr5:59963118-59986002 | 1277 | 4933439K11Rik | NR_040558.1 | chr1:172541619-172550806 |
| 1181 | 4933402J15Rik | NR_046059.1 | chr14:74355526-74369741 | 1278 | 4933440J02Rik | NR_045344.1 | chr10:111586718-111594273 |
| 1182 | 4933402N03Rik | NM_173409.4 | chr7:131138346-131146283 | 1279 | 4933440M02Rik | NR_045803.1 | chr7:125331781-125349786 |
| 1183 | 4933402N22Rik | NM_001177510.1 | chr5:11918042-11922804 | 1280 | 5031410I06Rik | NM_207657.3 | chr5:26098667-26105314 |
| 1184 | 4933402P03Rik | NM_175368.1 | chr11:69816565-69818440 | 1281 | 5031414D18Rik | NM_198642.2 | chr14:75016026-75052532 |
| 1185 | 4933403O08Rik | NM_001177389.1 | chrX:112239048-112243852 | 1282 | 5031425E22Rik | NR_040469.1 | chr5:23431807-23434353 |
| 1186 | 4933404G15Rik | NR_045920.1 | chr16:16707686-16718121 | 1283 | 5031425F14Rik | NR_015558.2 | chr2:166447450-166458770 |
| 1187 | 4933404K08Rik | NR_046038.1 | chr13:23278349-23282267 | 1284 | 5031426D15Rik | NR_027890.1 | chr2:6922701-6928722 |
| 1188 | 4933404O12Rik | NR_015555.1 | chr5:136919145-136937109 | 1285 | 5031434C07Rik | NR_045986.1 | chr6:112286793-112290319 |
| 1189 | 4933405D12Rik | NR_046036.1 | chr3:122882591-122924116 | 1286 | 5031434O11Rik | NR_033624.1 | chr3:51559756-51567116 |
| 1190 | 4933405E24Rik | NR_045506.1 | chr11:54177771-54213288 | 1287 | 5031439G07Rik | NM_001033273.2 | chr15:84945719-84987971 |
| 1191 | 4933405L10Rik | NM_027655.1 | chr8:105708302-105710168 | 1288 | 5033403H07Rik | NR_040413.1 | chr5:52973011-52992292 |
| 1192 | 4933405O20Rik | NM_172901.2 | chr7:50599189-50600528 | 1289 | 5033404E19Rik | NR_033600.1 | chr1:185497305-185498014 |
| 1193 | 4933406C10Rik | NR_044986.1 | chr12:32919383-32953789 | 1290 | 5033406O09Rik | NR_029464.1 | chr12:111941990-111944482 |
| 1194 | 4933406D12Rik | NR_040502.1 | chr2:146542930-146546108 | 1291 | 5133400J02Rik | NR_110444.1 | chr11:51189832-51220418 |
| 1195 | 4933406F09Rik | NR_015568.2 | chr14:7412472-7422685 | 1292 | 5330411J11Rik | NR_040510.1 | chr2:59382492-59386682 |
| 1196 | 4933406G16Rik | NR_046037.1 | chr11:19065274-19075740 | 1293 | 5330413P13Rik | NR_029381.1 | chr2:131820005-131847656 |
| 1197 | 4933406I18Rik | NR_029437.1 | chr7:114315478-114415161 | 1294 | 5330417C22Rik | NM_001033304.1 | chr3:108458068-108536522 |
| 1198 | 4933406J08Rik | NM_028914.1 | chr2:122186271-122206393 | 1295 | 5330426P16Rik | NR_028300.1 | chr16:50726750-50732773 |
| 1199 | 4933406J10Rik | NR_046004.1 | chr7:82743345-82755644 | 1296 | 5330434G04Rik | NR_015552.1 | chrX:105372993-105391754 |
| 1200 | 4933406K04Rik | NR_015512.2 | chr12:106685606-106716324 | 1297 | 5330439B14Rik | NR_037679.1 | chr6:142614594-142626462 |
| 1201 | 4933406M09Rik | NM_173771.4 | chr1:134385939-134390983 | 1298 | 5430401F13Rik | NM_001244628.1 | chr6:131543761-131553757 |
| 1202 | 4933407E24Rik | NR_045819.1 | chr4:124569178-124575538 | 1299 | 5430402E10Rik | NM_027768.3 | chrX:77919785-77925062 |
| 1203 | 4933407G14Rik | NR_045841.1 | chr10:75449509-75463104 | 1300 | 5430402O13Rik | NR_015581.1 | chr6:50566642-50594865 |
| 1204 | 4933407I05Rik | NR_040731.1 | chr9:51914428-51927003 | 1301 | 5430403N17Rik | NR_046181.1 | chr8:35864084-35882957 |
| 1205 | 4933407K13Rik | NR_029443.1 | chrX:75725457-75764699 | 1302 | 5430405H02Rik | NR_015591.1 | chr2:156852402-156862945 |
| 1206 | 4933407L21Rik | NR_037692.1 | chr1:85928482-85931756 | 1303 | 5430416N02Rik | NR_034038.1 | chr5:100420841-100429535 |
| 1207 | 4933408B17Rik | NM_177773.4 | chr18:34579845-34597468 | 1304 | 5430416O09Rik | NR_033355.1 | chr4:43730033-43734534 |
| 1208 | 4933408J17Rik | NR_045810.1 | chr10:93589412-93605245 | 1305 | 5430417L22Rik | NM_001301269.1 | chr2:118745758-118748810 |
| 1209 | 4933408N05Rik | NR_045831.2 | chr8:114341160-114351478 | 1306 | 5430419D17Rik | NM_175166.3 | chr7:131174401-131250945 |
| 1210 | 4933409G03Rik | NM_177651.3 | chr2:68582412-68616463 | 1307 | 5430421F17Rik | NR_040352.1 | chr8:25302649-25306270 |
| 1211 | 4933409K07Rik | NR_033123.1 | chr4:60800-42462993 | 1308 | 5430421N21Rik | NM_001201323.1 | chr15:101485129-101491512 |
| 1212 | 4933411E08Rik | NR_045342.1 | chr10:117925458-117948307 | 1309 | 5430425K12Rik | NR_103550.1 | chr13:80940402-80948597 |
| 1213 | 4933411G06Rik | NR_045158.1 | chr10:51756173-51757194 | 1310 | 5430427M07Rik | NR_045858.1 | chr12:91045847-91056635 |
| 1214 | 4933411G11Rik | NM_177880.4 | chr5:143181016-143205068 | 1311 | 5430427O19Rik | NM_001163539.1 | chrX:85870272-85891499 |
| 1215 | 4933411K16Rik | NM_025752.2 | chr19:42052227-42053628 | 1312 | 5430428K19Rik | NR_045426.1 | chrX:6452375-6574663 |
| 1216 | 4933411K20Rik | NM_025747.3 | chr8:46169555-46195590 | 1313 | 5430434I15Rik | NR_040541.1 | chr3:38203150-38208119 |
| 1217 | 4933412E12Rik | NR_038025.1 | chr10:116950561-116963279 | 1314 | 5430435G22Rik | NM_145509.3 | chr1:131688694-131715439 |
| 1218 | 4933412E24Rik | NM_027668.1 | chr15:60014865-60016618 | 1315 | 5430437J10Rik | NR_045274.1 | chr15:5496317-5594983 |
| 1219 | 4933412O06Rik | NR_045507.1 | chr13:15781663-15802630 | 1316 | 5430440P10Rik | NR_045859.1 | chr14:105427558-105437716 |
| 1220 | 4933413G19Rik | NM_027697.1 | chr6:128375509-128385144 | 1317 | 5530400C23Rik | NM_027784.1 | chr6:133292215-133295790 |
| 1221 | 4933413J09Rik | NR_038005.1 | chr14:26357546-26400456 | 1318 | 5530401A14Rik | NR_038010.1 | chr11:81860676-81894582 |
| 1222 | 4933413L06Rik | NR_045508.1 | chr13:117706763-117720011 | 1319 | 5530601H04Rik | NR_015467.1 | chrX:105040853-105070124 |
| 1223 | 4933415F23Rik | NM_025746.2 | chr1:23100473-23102253 | 1320 | 5730403I07Rik | NR_040378.1 | chr9:77366411-77399416 |
| 1224 | 4933416C03Rik | NM_001161855.1 | chr10:116111663-116113917 | 1321 | 5730405O15Rik | NR_038158.1 | chrX:13042014-13045295 |
| 1225 | 4933416E03Rik | NR_040498.1 | chr2:159947340-159981289 | 1322 | 5730408K05Rik | NR_027866.1 | chr19:8888386-8888770 |
| 1226 | 4933416I08Rik | NM_027700.1 | chrX:53686490-53691332 | 1323 | 5730409E04Rik | NM_001013755.3 | chr4:126609853-126614371 |
| 1227 | 4933416M06Rik | NR_045846.2 | chr13:101254318-101293396 | 1324 | 5730412P04Rik | NR_045424.1 | chrX:136104602-136115420 |
| 1228 | 4933416M07Rik | NR_045840.1 | chr8:27142909-27149706 | 1325 | 5730416F02Rik | NR_033596.1 | chrX:109001484-109002296 |
| 1229 | 4933417A18Rik | NM_025750.3 | chr13:34924408-34953196 | 1326 | 5730420D15Rik | NR_045338.1 | chr10:95417374-95428640 |
| 1230 | 4933417D19Rik | NR_045849.1 | chr8:123235090-123242359 | 1327 | 5730422E09Rik | NR_015478.2 | chr5:149119564-149182036 |
| 1231 | 4933417E11Rik | NR_040454.1 | chr1:71958997-72005195 | 1328 | 5730435O14Rik | NR_045341.1 | chr10:41568015-41576476 |
| 1232 | 4933417G07Rik | NR_033592.1 | chr3:55847104-55848065 | 1329 | 5730455P16Rik | NM_027472.3 | chr11:80360491-80378015 |
| 1233 | 4933417O13Rik | NR_045842.1 | chr7:143322771-143330650 | 1330 | 5730457N03Rik | NR_038163.1 | chr6:52308388-52314832 |
| 1234 | 4933421I07Rik | NM_027702.2 | chr7:42445461-42448080 | 1331 | 5730460C07Rik | NR_045801.1 | chr3:153789304-153792087 |
| 1235 | 4933421O10Rik | NR_036602.1 | chr4:33027138-33031323 | 1332 | 5730480H06Rik | NR_045500.1 | chr5:48395572-48414294 |
| 1236 | 4933422A05Rik | NR_040715.1 | chr9:35281104-35285423 | 1333 | 5730488B01Rik | NR_073462.1 | chr4:44702799-44705110 |
| 1237 | 4933422H20Rik | NM_001033775.3 | chr11:115440544-115448268 | 1334 | 5730507C01Rik | NM_001033157.4 | chr12:18514509-18535254 |
| 1238 | 4933424G05Rik | NR_045372.1 | chr18:14988994-15047987 | 1335 | 5730508B09Rik | NM_027482.3 | chr3:127869687-127896323 |
| 1239 | 4933424G06Rik | NR_040290.1 | chr1:36714537-36757960 | 1336 | 5730522E02Rik | NR_027973.1 | chr11:25616845-26210576 |
| 1240 | 4933425B07Rik | NR_046460.1 | chr14:46602805-46637423 | 1337 | 5730559C18Rik | NM_028872.3 | chr1:136213521-136234280 |
| 1241 | 4933425L06Rik | NM_025751.3 | chr13:105082121-105121782 | 1338 | 5830403L16Rik | NM_178243.3 | chr1:153849541-153851195 |
| 1242 | 4933426M11Rik | NM_001242419.1 | chr12:80790531-80880833 | 1339 | 5830411N06Rik | NM_001128145.1 | chr7:140247380-140299791 |
| 1243 | 4933427D06Rik | NM_175017.3 | chr6:89096114-89110037 | 1340 | 5830415F09Rik | NM_029086.2 | chr4:46376974-46389423 |
| 1244 | 4933427D14Rik | NM_028963.2 | chr11:72154096-72203371 | 1341 | 5830416I19Rik | NR_045384.1 | chr5:64044972-64050226 |
| 1245 | 4933427E11Rik | NR_033197.1 | chr15:74709160-74710374 | 1342 | 5830416P10Rik | NR_028427.1 | chr19:53441211-53464796 |
| 1246 | 4933427E13Rik | NR_045853.1 | chr11:25326106-25367137 | 1343 | 5830417I10Rik | NR_028359.1 | chr3:88777056-88829371 |
| 1247 | 4933427G17Rik | NM_028955.1 | chr7:120982508-121014787 | 1344 | 5830418K08Rik | NM_176976.4 | chr9:15316913-15357788 |
| 1248 | 4933427I22Rik | NR_045159.1 | chr4:139926110-139943519 | 1345 | 5830418P13Rik | NR_040466.1 | chr9:103423577-103461260 |
| 1249 | 4933428C19Rik | NR_040644.1 | chr4:40482839-40525548 | 1346 | 5830428M24Rik | NR_038060.1 | chr12:69464786-69475550 |
| 1250 | 4933428G20Rik | NM_001289118.1 | chr11:97490782-97500340 | 1347 | 5830432E09Rik | NR_015548.1 | chr7:135648456-135652314 |
| 1251 | 4933429K18Rik | NR_045307.1 | chr19:45726554-45730558 | 1348 | 5830444B04Rik | NR_102283.1 | chr4:155408308-155421704 |
| 1252 | 4933429O19Rik | NR_033783.1 | chr14:48878872-48887112 | 1349 | 5830454E08Rik | NR_073359.1 | chr9:120577330-120578073 |
| 1253 | 4933430H16Rik | NR_045855.1 | chr7:82937937-82971391 | 1350 | 5830473C10Rik | NM_001252661.1 | chr5:90561208-90597906 |
| 1254 | 4933430I17Rik | NM_177607.3 | chr4:62525368-62547993 | 1351 | 5930403L14Rik | NR_045643.1 | chr4:154630690-154636367 |
| 1255 | 4933430M04Rik | NR_045857.2 | chr11:24481908-24569951 | 1352 | 5930412G12Rik | NR_015517.2 | chr5:128579129-128600687 |
| 1256 | 4933430N04Rik | NR_045854.1 | chr8:9125411-9132288 | 1353 | 5930430L01Rik | NR_102383.1 | chr5:148990055-148995214 |
| 1257 | 4933431E20Rik | NR_015459.1 | chr3:107888849-107896213 | 1354 | 5930438M14Rik | NR_046158.1 | chr13:101232126-101247551 |
| 1258 | 4933431G14Rik | NR_045163.1 | chr6:72120212-72122940 | 1355 | 6030407O03Rik | NR_045311.1 | chr1:73618380-73864466 |
| 1259 | 4933432G23Rik | NR_040716.1 | chr9:118429838-118437048 | 1356 | 6030408B16Rik | NR_033803.1 | chr15:101293211-101297426 |
| 1260 | 4933432I03Rik | NR_045657.1 | chr14:102987411-103033803 | 1357 | 6030419C18Rik | NM_176921.3 | chr9:58488602-58499742 |

Fig.21 - 8

| | | | |
|---|---|---|---|
| 1358 | 6030440G07Rik | NR_036598.1 | chr12:110994397-111013872 |
| 1359 | 6030443J06Rik | NR_102315.1 | chr5:22550412-22558171 |
| 1360 | 6030458C11Rik | NM_001166360.1 | chr15:12819903-12824657 |
| 1361 | 6030466F02Rik | NR_040702.1 | chr8:123733957-123739941 |
| 1362 | 6030468B19Rik | NM_029964.1 | chr11:117797659-117807308 |
| 1363 | 6030469F06Rik | NR_102715.1 | chr12:31168861-31185922 |
| 1364 | 6030498E09Rik | NM_183126.2 | chrX:38772779-38962688 |
| 1365 | 6230400D17Rik | NR_029446.1 | chr14:20702011-20703099 |
| 1366 | 6330403A02Rik | NM_001081227.2 | chr1:180432372-180483504 |
| 1367 | 6330403K07Rik | NM_134022.2 | chr11:71031940-71033617 |
| 1368 | 6330407A03Rik | NR_028126.1 | chr4:3714963-3716806 |
| 1369 | 6330408A02Rik | NM_177312.4 | chr7:13258966-13278721 |
| 1370 | 6330409D20Rik | NM_027529.1 | chr2:32732620-32741016 |
| 1371 | 6330410L21Rik | NR_040589.1 | chr3:129753757-129763882 |
| 1372 | 6330415B21Rik | NR_045141.1 | chr6:77380256-77382475 |
| 1373 | 6330416G13Rik | NM_144905.3 | chr4:63560359-63586353 |
| 1374 | 6330418K02Rik | NR_045821.1 | chr5:138264044-138266661 |
| 1375 | 6330419J24Rik | NR_028086.1 | chrX:56374585-56378470 |
| 1376 | 6330549D23Rik | NR_003619.2 | chr3:96606661-96629819 |
| 1377 | 6430411K18Rik | NR_002848.3 | chr12:109591536-109592851 |
| 1378 | 6430503K07Rik | NR_108091.1 | chr2:147187423-147188482 |
| 1379 | 6430531B16Rik | NM_001033465.2 | chr7:139972302-139978755 |
| 1380 | 6430548M08Rik | NM_001163760.1 | chr8:120144928-120165307 |
| 1381 | 6430550D23Rik | NM_001145351.1 | chr2:156000442-156004427 |
| 1382 | 6430562O15Rik | NR_015515.2 | chr13:99410835-99412826 |
| 1383 | 6430571L13Rik | NM_175486.3 | chr9:107340639-107349683 |
| 1384 | 6430573F11Rik | NM_176952.4 | chr8:36489190-36513013 |
| 1385 | 6430584L05Rik | NR_046179.1 | chr6:55396886-55412285 |
| 1386 | 6430706D22Rik | NR_040291.1 | chr1:88263112-88269408 |
| 1387 | 6430710C18Rik | NR_102348.1 | chr2:72745845-72813717 |
| 1388 | 6530402F18Rik | NR_029460.1 | chr2:29245115-29252993 |
| 1389 | 6530411M01Rik | NR_027881.1 | chr17:9147718-9168022 |
| 1390 | 6720416L17Rik | NR_110445.1 | chr2:74748421-74762896 |
| 1391 | 6720468P15Rik | NR_040306.1 | chr19:57508563-57512788 |
| 1392 | 6720483E21Rik | NR_040492.1 | chr1:20888649-20890473 |
| 1393 | 6720489N17Rik | NM_173381.1 | chr13:62603014-62624182 |
| 1394 | 6820408C15Rik | NM_001289738.1 | chr2:152415586-152444330 |
| 1395 | 6820431F20Rik | NR_030708.1 | chr8:20268285-20297432 |
| 1396 | 7420426K07Rik | NM_001033983.3 | chr9:98903118-98904622 |
| 1397 | 7420461P10Rik | NM_001177581.1 | chr1:162647242-162658219 |
| 1398 | 7420700N18Rik | NR_046272.1 | chr8:31367748-31382438 |
| 1399 | 7420701I03Rik | NR_045860.1 | chr12:11047812-11078741 |
| 1400 | 7530416G11Rik | NM_001256072.1 | chr15:85492735-85503227 |
| 1401 | 7630403G23Rik | NR_040744.1 | chr9:34042370-34059019 |
| 1402 | 8030411F24Rik | NM_030135.2 | chr2:148782008-148785936 |
| 1403 | 8030423F21Rik | NR_045738.1 | chr5:52607549-52619011 |
| 1404 | 8030423J24Rik | NM_029873.1 | chr13:70882947-70884503 |
| 1405 | 8030442B05Rik | NR_040615.1 | chr2:11338365-11398500 |
| 1406 | 8030443G20Rik | NR_040664.1 | chr4:108942842-108972077 |
| 1407 | 8030462N17Rik | NM_178670.3 | chr18:77633280-77714010 |
| 1408 | 8430408G22Rik | NM_001166580.1 | chr6:116650683-116652847 |
| 1409 | 8430419L09Rik | NM_028982.4 | chr6:135197986-135236240 |
| 1410 | 8430422H06Rik | NR_045373.1 | chr18:14232305-14278542 |
| 1411 | 8430423G03Rik | NR_040686.1 | chr5:148950249-148951658 |
| 1412 | 8430426J06Rik | NR_077229.1 | chr15:81242653-81247971 |
| 1413 | 8430427H17Rik | NM_001001986.2 | chr2:153407460-153444479 |
| 1414 | 8430429K09Rik | NR_028317.1 | chr11:3452436-3479831 |
| 1415 | 8430431K14Rik | NR_002849.1 | chr19:31213542-31216396 |
| 1416 | 8430436N08Rik | NR_040645.1 | chr4:7560687-7573801 |
| 1417 | 8430437L04Rik | NR_040503.1 | chr2:72703509-72729468 |
| 1418 | 9030025P20Rik | NM_001123370.1 | chr17:14978863-14991577 |
| 1419 | 9030204H09Rik | NR_040618.1 | chr2:35819189-35823385 |
| 1420 | 9030404E10Rik | NR_045878.1 | chr16:30021841-30039479 |
| 1421 | 9030612E09Rik | NR_102361.1 | chr10:43174698-43176565 |
| 1422 | 9030617O03Rik | NM_145448.4 | chr12:100779122-100872610 |
| 1423 | 9030619P08Rik | NR_108041.1 | chr15:75427605-75431829 |
| 1424 | 9030624G23Rik | NM_001256489.1 | chr12:24043201-24097269 |
| 1425 | 9030624J02Rik | NM_027815.4 | chr7:118740270-118841491 |
| 1426 | 9030625G05Rik | NR_110446.1 | chr18:74972763-74981547 |
| 1427 | 9130008F23Rik | NM_027834.3 | chr17:40875481-40880558 |
| 1428 | 9130011E15Rik | NM_198296.2 | chr19:45818143-45998488 |
| 1429 | 9130015A21Rik | NR_045050.1 | chr12:35672290-35695118 |
| 1430 | 9130015L21Rik | NR_040499.1 | chr2:160016652-160040620 |
| 1431 | 9130019O22Rik | NM_030226.3 | chr7:127382259-127387166 |
| 1432 | 9130019P16Rik | NR_033635.1 | chr6:54269680-54430221 |
| 1433 | 9130023H24Rik | NM_177001.3 | chr7:128234450-128238031 |
| 1434 | 9130024F11Rik | NR_024325.1 | chr1:56971468-56975196 |
| 1435 | 9130204L05Rik | NM_001101461.2 | chr3:91088136-91090805 |
| 1436 | 9130209A04Rik | NR_033453.1 | chr18:47452667-47470868 |
| 1437 | 9130221F21Rik | NR_046180.1 | chr6:72013895-72042528 |
| 1438 | 9130221H12Rik | NR_046001.1 | chr7:25228722-25230264 |
| 1439 | 9130227L01Rik | NR_045837.1 | chr1:55931240-55972098 |
| 1440 | 9130230L23Rik | NR_027961.1 | chr5:65987927-66004285 |
| 1441 | 9130401M01Rik | NM_029418.4 | chr15:58022270-58034294 |
| 1442 | 9130409I23Rik | NM_001033819.1 | chr1:181051236-181060667 |
| 1443 | 9230009I02Rik | NR_045865.1 | chr11:51085086-51091835 |
| 1444 | 9230102K24Rik | NR_028438.1 | chr10:110600649-110615961 |
| 1445 | 9230102O04Rik | NR_040511.1 | chr2:9883040-9889540 |
| 1446 | 9230104L09Rik | NM_029960.3 | chr2:148846713-148850934 |
| 1447 | 9230105E05Rik | NR_040626.1 | chr10:120389522-120392793 |
| 1448 | 9230110C19Rik | NM_199017.2 | chr9:8021671-8042823 |
| 1449 | 9230110F15Rik | NM_029863.1 | chr9:35838328-35844150 |
| 1450 | 9230112D13Rik | NM_030062.1 | chr14:34511620-34522801 |
| 1451 | 9230112J17Rik | NR_040463.1 | chr9:60523966-60545882 |
| 1452 | 9230114K14Rik | NR_015537.2 | chr5:52190680-52197984 |
| 1453 | 9230116L04Rik | NR_110486.1 | chr12:79088704-79103939 |
| 1454 | 9230116N13Rik | NR_024328.1 | chr1:136411915-136415519 |
| 1455 | 9330020H09Rik | NR_028442.1 | chr15:98567323-98570864 |
| 1456 | 9330102E08Rik | NR_077223.1 | chr6:128169699-128183803 |
| 1457 | 9330111N05Rik | NR_015587.2 | chr13:80964073-81079857 |
| 1458 | 9330117O12Rik | NR_045400.1 | chr18:54611743-54639870 |
| 1459 | 9330133O14Rik | NR_045696.1 | chr8:122443655-122446476 |
| 1460 | 9330151L19Rik | NR_033222.1 | chr12:69197210-69199868 |
| 1461 | 9330158H04Rik | NR_015589.1 | chr6:36333137-36388234 |
| 1462 | 9330159F19Rik | NM_001162537.2 | chr10:29211642-29230779 |
| 1463 | 9330159M07Rik | NR_037982.1 | chr9:88841797-88858801 |
| 1464 | 9330162012Rik | NR_102323.1 | chr13:24937400-24939049 |
| 1465 | 9330162B11Rik | NR_038007.1 | chr1:188006575-188008990 |
| 1466 | 9330175E14Rik | NR_015514.2 | chr8:94433126-94435103 |
| 1467 | 9330175M20Rik | NR_045151.2 | chr1:51218385-51333779 |
| 1468 | 9330178D15Rik | NR_040553.1 | chr3:155976353-156008592 |
| 1469 | 9330179D12Rik | NR_040273.1 | chr6:127149625-127212419 |
| 1470 | 9330182L06Rik | NM_172706.3 | chr5:9266192-9480717 |
| 1471 | 9330182O14Rik | NM_001256056.1 | chr15:40134364-40150010 |
| 1472 | 9330188P03Rik | NR_102319.1 | chr14:105589548-105593929 |
| 1473 | 9430007A20Rik | NM_198662.3 | chr4:144519821-144529353 |
| 1474 | 9430008C03Rik | NR_015463.1 | chr2:158353699-158361509 |
| 1475 | 9430014N10Rik | NR_045737.1 | chr15:93904855-93927111 |
| 1476 | 9430015G10Rik | NM_145557.3 | chr4:156109997-156127263 |
| 1477 | 9430016H08Rik | NM_001081181.2 | chr1:57406547-57415958 |
| 1478 | 9430018G01Rik | NR_045988.1 | chr6:43442428-43474318 |
| 1479 | 9430019J16Rik | NR_040635.1 | chr2:75436056-75444179 |
| 1480 | 9430020K01Rik | NM_001081963.1 | chr18:4634928-4682869 |
| 1481 | 9430021M05Rik | NR_033569.1 | chr2:162661162-162675204 |
| 1482 | 9430037G07Rik | NR_040766.1 | chr9:88595324-88599243 |
| 1483 | 9430038I01Rik | NM_029886.2 | chr7:137375568-137410756 |
| 1484 | 9430041J12Rik | NR_033568.1 | chr7:4074124-4120728 |
| 1485 | 9430060I03Rik | NR_015525.1 | chr1:92933542-92950590 |
| 1486 | 9430069I07Rik | NM_001256161.1 | chr15:34349050-34357872 |
| 1487 | 9430076C15Rik | NR_015553.2 | chr6:53287294-53397216 |
| 1488 | 9430083A17Rik | NR_029463.1 | chr13:51097737-51100727 |
| 1489 | 9430091E24Rik | NR_040363.1 | chr8:111127443-111145498 |
| 1490 | 9530002B09Rik | NM_023865.3 | chr4:122689258-122705135 |
| 1491 | 9530003J23Rik | NM_029906.3 | chr10:117233752-117238681 |
| 1492 | 9530026F06Rik | NR_040483.1 | chr1:61378431-61395904 |
| 1493 | 9530026P05Rik | NR_015530.2 | chr6:92940581-93111749 |
| 1494 | 9530027J09Rik | NR_045916.1 | chrX:48276791-48285426 |
| 1495 | 9530036O11Rik | NR_015562.1 | chr5:28466983-28719209 |
| 1496 | 9530051G07Rik | NR_040272.1 | chrX:153037628-153072915 |
| 1497 | 9530052E02Rik | NR_046017.1 | chr8:11007849-11054541 |
| 1498 | 9530053A07Rik | NM_001164655.1 | chr7:28129465-28164811 |
| 1499 | 9530059O14Rik | NR_015610.1 | chr9:122572500-122580647 |
| 1500 | 9530068E07Rik | NM_153117.2 | chr11:52396427-52408723 |
| 1501 | 9530077C05Rik | NM_026739.1 | chr9:22411576-22444679 |
| 1502 | 9530080O11Rik | NR_045776.1 | chr4:95959710-95967655 |
| 1503 | 9530082P21Rik | NR_015472.1 | chr17:23749235-23754065 |
| 1504 | 9530091C08Rik | NR_033299.1 | chr9:68765345-68773322 |
| 1505 | 9630001P10Rik | NR_102378.1 | chr5:45639274-45645469 |
| 1506 | 9630013A20Rik | NR_015539.1 | chr14:84458837-84476424 |
| 1507 | 9630028B13Rik | NR_038006.1 | chr1:185429356-185441819 |
| 1508 | 9630028H03Rik | NR_015544.2 | chr2:135580529-135583220 |
| 1509 | 9630033F20Rik | NM_177003.5 | chr6:127085115-127109552 |
| 1510 | 9830107B12Rik | NM_001177896.1 | chr17:48125604-48146307 |
| 1511 | 9830132P13Rik | NR_040552.1 | chr3:127916170-127955220 |
| 1512 | 9830147E19Rik | NM_001242388.1 | chr7:42609525-42642604 |
| 1513 | 9830166K06Rik | NR_045314.1 | chr19:8651795-8663335 |
| 1514 | 9930012K11Rik | NM_001004155.2 | chr14:70154404-70159502 |
| 1515 | 9930014A18Rik | NR_030696.1 | chr15:60822964-60831400 |
| 1516 | 9930021J03Rik | NM_172836.3 | chr19:29714401-29806009 |
| 1517 | 9930104L06Rik | NM_177573.3 | chr4:124937047-124944661 |
| 1518 | 9930111H07Rik | NR_108086.1 | chr1:85775270-85784694 |
| 1519 | 9930111J21Rik1 | NM_001114679.1 | chr11:48946149-48979381 |
| 1520 | 9930111J21Rik2 | NM_173434.1 | chr11:48965816-49051242 |
| 1521 | a | NM_015770.3 | chr2:155013569-155051012 |
| 1522 | A130010J15Rik | NM_001160359.1 | chr1:193173576-193177832 |
| 1523 | A130077B15Rik | NR_040616.1 | chr10:122565022-122569891 |
| 1524 | A1bg | NM_001081067.1 | chr15:60917588-60921270 |
| 1525 | A1cf | NM_001081074.1 | chr19:31868760-31949406 |
| 1526 | A230001M10Rik | NR_040391.1 | chr3:102262404-102288771 |
| 1527 | A230009B12Rik | NR_077237.1 | chr17:10456215-10493057 |
| 1528 | A230020J21Rik | NR_027298.1 | chr1:191025350-191029781 |
| 1529 | A230028O05Rik | NR_040374.1 | chr16:25059638-25069058 |
| 1530 | A230046K03Rik | NM_001033375.2 | chr10:83543940-83596473 |
| 1531 | A230050P20Rik | NM_175687.2 | chr9:20868641-20874307 |
| 1532 | A230056J06Rik | NR_045633.1 | chr13:59580175-59585223 |
| 1533 | A230056P14Rik | NR_015495.2 | chr7:55962530-55980096 |
| 1534 | A230057D06Rik | NR_015533.2 | chr7:61705849-61927574 |
| 1535 | A230065H16Rik | NM_001101503.1 | chr12:111406808-111412077 |
| 1536 | A230070E04Rik | NR_045897.1 | chr14:68123751-68131371 |
| 1537 | A230072C01Rik | NR_027445.1 | chrX:20961675-20987349 |
| 1538 | A230072E10Rik | NR_015600.2 | chrX:151313334-151341879 |
| 1539 | A230073K19Rik | NR_033229.1 | chr7:59281879-59289166 |
| 1540 | A230077H06Rik | NR_040329.1 | chr7:40849152-40898324 |
| 1541 | A230103J11Rik | NR_110581.1 | chr8:85053159-85060460 |
| 1542 | A230108P19Rik | NR_040333.1 | chr2:6193250-6321611 |
| 1543 | A2m | NM_175628.3 | chr6:121636172-121679238 |
| 1544 | A330009N23Rik | NR_045326.1 | chr15:101221190-101224121 |
| 1545 | A330021E22Rik | NM_172447.2 | chr5:5580981-5664232 |
| 1546 | A330023F24Rik | NR_015566.2 | chr1:195017398-195037908 |
| 1547 | A330032B11Rik | NR_045329.1 | chr19:37173842-37196541 |
| 1548 | A330033J07Rik | NR_102303.1 | chr13:48044230-48262623 |
| 1549 | A330035P11Rik | NR_015586.2 | chr14:122097938-122106974 |
| 1550 | A330040F15Rik | NR_015503.1 | chr19:12585867-12596566 |
| 1551 | A330041J22Rik | NR_045835.1 | chr9:86695441-86701253 |

Fig.21 - 9

| | | | |
|---|---|---|---|
| 1552 | A330048O09Rik | NR_045162.1 | chr13:48272417-48273884 |
| 1553 | A330049N07Rik | NR_040646.1 | chr10:72973302-73086705 |
| 1554 | A330050F15Rik | NM_001145192.1 | chr17:69439325-69489233 |
| 1555 | A330069E16Rik | NR_015464.1 | chr2:91237145-91238357 |
| 1556 | A330070K13Rik | NM_198665.1 | chr5:130378850-130384631 |
| 1557 | A330074K22Rik | NR_110496.1 | chr8:120204433-120228230 |
| 1558 | A330076C08Rik | NR_045088.1 | chr13:44193588-44216487 |
| 1559 | A330076H08Rik | NR_015599.2 | chr7:61943900-61982303 |
| 1560 | A330093E20Rik | NR_040342.1 | chr18:45683486-46045260 |
| 1561 | A330102I10Rik | NR_045073.1 | chr13:29016254-29040336 |
| 1562 | A3galt2 | NM_001009819.2 | chr4:128759257-128769298 |
| 1563 | A430005L14Rik | NM_001163019.1 | chr4:153957236-153961924 |
| 1564 | A430033K04Rik | NM_183025.2 | chr5:138622858-138648905 |
| 1565 | A430035B10Rik | NR_040452.1 | chr6:8507058-8509161 |
| 1566 | A430078G23Rik | NM_001033378.3 | chr8:3353414-3390299 |
| 1567 | A430088P11Rik | NR_045309.1 | chr15:80691024-80697610 |
| 1568 | A430089I19Rik | NM_177913.4 | chr5:5184-94955288 |
| 1569 | A430090L17Rik | NR_045836.1 | chr13:114124347-114151592 |
| 1570 | A430093F15Rik | NR_027805.1 | chr19:10740946-10786043 |
| 1571 | A430105I19Rik | NM_001001982.2 | chr2:118754144-118762661 |
| 1572 | A430107P09Rik | NM_001205242.1 | chr14:53666005-53666506 |
| 1573 | A4galt | NM_001004150.3 | chr15:83226721-83251774 |
| 1574 | A4gnt | NM_001077424.2 | chr9:99612501-99622367 |
| 1575 | A530006G24Rik | NR_046014.1 | chr2:147710692-147717632 |
| 1576 | A530013C23Rik | NR_015500.3 | chr2:167691176-167697413 |
| 1577 | A530016L24Rik | NM_177039.4 | chr12:112489447-112499927 |
| 1578 | A530032D15Rik | NM_213615.2 | chr1:85088138-85109853 |
| 1579 | A530046M15Rik | NR_046131.1 | chr13:15807242-15827537 |
| 1580 | A530050N04Rik | NR_045419.1 | chr18:61470224-61484607 |
| 1581 | A530053G22Rik | NR_015565.2 | chr6:60279042-60403707 |
| 1582 | A530054K11Rik | NM_183146.3 | chr13:67617000-67637753 |
| 1583 | A530058N18Rik | NR_028423.1 | chr2:114013564-114032292 |
| 1584 | A530064D06Rik | NM_001113556.1 | chr17:48151895-48167257 |
| 1585 | A530065N20Rik | NR_046142.1 | chr13:60029710-60116669 |
| 1586 | A530072M11Rik | NR_045765.2 | chr4:16164109-16266225 |
| 1587 | A530088E08Rik | NR_029458.1 | chr17:32403013-32404808 |
| 1588 | A530099J19Rik | NM_175688.4 | chr13:19727416-19732951 |
| 1589 | A630001G21Rik | NM_177055.3 | chr1:85717082-85736606 |
| 1590 | A630007B06Rik | NM_170757.1 | chr19:56790962-56813683 |
| 1591 | A630010A05Rik | NR_033556.1 | chr16:14562316-14621284 |
| 1592 | A630012P03Rik | NR_045367.2 | chrX:52575842-52619047 |
| 1593 | A630019I02Rik | NR_046182.1 | chr13:93094832-93098968 |
| 1594 | A630020A06 | NR_045740.1 | chr15:3996038-4015858 |
| 1595 | A630023A22Rik | NM_001251843.1 | chr14:34051129-34102754 |
| 1596 | A630023P12Rik | NR_102290.1 | chr5:110514922-110525979 |
| 1597 | A630033H20Rik | NM_001122595.1 | chrX:107149453-107173661 |
| 1598 | A630066F11Rik | NR_030698.1 | chr10:7663370-7664623 |
| 1599 | A630072M18Rik | NR_030699.1 | chr5:20950988-20956398 |
| 1600 | A630073D07Rik | NM_001142969.1 | chr6:132625110-132627511 |
| 1601 | A630075F10Rik | NR_033632.1 | chr2:170061457-170070778 |
| 1602 | A630076J17Rik | NM_001256174.1 | chr3:107230613-107234031 |
| 1603 | A630077J23Rik | NR_040667.1 | chr4:43751858-43759464 |
| 1604 | A630089N07Rik | NR_015491.1 | chr16:98062511-98082439 |
| 1605 | A630095E13Rik | NM_001033325.2 | chr9:36635753-36638602 |
| 1606 | A630095N17Rik | NM_001243090.1 | chr1:75220094-75232093 |
| 1607 | A730006G06Rik | NR_110485.1 | chr17:48064788-48090378 |
| 1608 | A730008H23Rik | NM_172505.4 | chr1:88264827-88277557 |
| 1609 | A730017C20Rik | NM_001167925.2 | chr18:59062380-59076960 |
| 1610 | A730017L22Rik | NR_015523.2 | chr2:130872540-130906396 |
| 1611 | A730018C14Rik | NR_036459.1 | chr12:112411022-112423198 |
| 1612 | A730020E08Rik | NR_040287.1 | chr6:61175603-61180810 |
| 1613 | A730020M07Rik | NR_036456.1 | chr3:121634935-121643485 |
| 1614 | A730036I17Rik | NR_045838.1 | chr2:129218968-129235606 |
| 1615 | A730043L09Rik | NR_040769.1 | chr9:62242595-62244100 |
| 1616 | A730046J19Rik | NR_040271.1 | chrX:144153694-144166274 |
| 1617 | A730056A06Rik | NR_040324.1 | chr7:73313805-73375774 |
| 1618 | A730082K24Rik | NR_040317.1 | chr7:114948649-114983967 |
| 1619 | A730085K08Rik | NR_045967.1 | chr9:122382021-122398801 |
| 1620 | A730090H04Rik | NR_040279.1 | chr11:95145052-95159047 |
| 1621 | A730090N16Rik | NR_040390.1 | chr3:65324019-65349429 |
| 1622 | A730098P11Rik | NR_038091.1 | chr16:24529313-24534472 |
| 1623 | A830009L08Rik | NR_045161.1 | chr13:91368989-91388085 |
| 1624 | A830010M20Rik | NM_001007574.2 | chr5:107497744-107512556 |
| 1625 | A830018L16Rik | NM_001160369.2 | chr1:11414104-11975902 |
| 1626 | A830019L24Rik | NR_040551.1 | chr3:143243054-143257996 |
| 1627 | A830052D11Rik | NR_045403.1 | chr18:32359056-32378284 |
| 1628 | A830080D01Rik | NM_001033472.2 | chrX:159532667-159593081 |
| 1629 | A830082K12Rik | NR_045195.1 | chr13:78198016-78236564 |
| 1630 | A830082N09Rik | NR_015526.2 | chr10:33987215-33995055 |
| 1631 | A930001A20Rik | NR_040549.1 | chr3:14971200-15002727 |
| 1632 | A930001C03Rik | NR_045989.1 | chr19:4439558-4448325 |
| 1633 | A930003A15Rik | NR_015488.1 | chr16:19876567-19884274 |
| 1634 | A930003O13Rik | NR_027362.1 | chr5:22738910-22746884 |
| 1635 | A930004D18Rik | NR_028376.1 | chr2:18025188-18037741 |
| 1636 | A930005H10Rik | NR_015487.1 | chr3:115881578-115888130 |
| 1637 | A930006I01Rik | NR_040332.1 | chr2:103338600-103372764 |
| 1638 | A930006K02Rik | NR_077219.1 | chr16:91465103-91470123 |
| 1639 | A930007I19Rik | NR_015567.2 | chr19:29503662-29521987 |
| 1640 | A930009A15Rik | NM_029982.1 | chr10:115569985-115582454 |
| 1641 | A930011G23Rik | NR_030692.1 | chr5:99297243-99729060 |
| 1642 | A930011O12Rik | NR_040709.1 | chr14:64588114-64593961 |
| 1643 | A930012L18Rik | NR_026853.1 | chr18:44661664-44676271 |
| 1644 | A930013F10Rik | NR_027886.1 | chr8:22634383-22636809 |
| 1645 | A930015D03Rik | NR_015618.2 | chr17:35994503-36038174 |
| 1646 | A930016O22Rik | NR_073014.1 | chr7:19417465-19421417 |
| 1647 | A930017M01Rik | NR_033609.2 | chr15:44881393-44884743 |
| 1648 | A930018P22Rik | NM_026634.2 | chr2:104122768-104124746 |

| | | | |
|---|---|---|---|
| 1649 | A930019D19Rik | NR_040619.1 | chr2:146259285-146267160 |
| 1650 | A930024E05Rik | NR_045820.1 | chr5:122989353-122998341 |
| 1651 | A930041C12Rik | NR_046195.1 | chr5:107630249-107633850 |
| 1652 | AA387883 | NR_030678.1 | chr19:52923180-52926931 |
| 1653 | AA388235 | NR_033305.1 | chr17:33981491-33985358 |
| 1654 | AA413626 | NR_102683.1 | chr11:4918515-4918918 |
| 1655 | AA414768 | NM_001272033.1 | chrX:12936872-12938541 |
| 1656 | AA415398 | NM_001004178.1 | chr4:119530314-119538769 |
| 1657 | AA465934 | NR_028363.1 | chr11:83291698-83294637 |
| 1658 | AA467197 | NM_001004174.1 | chr2:122637886-122641076 |
| 1659 | AA474331 | NR_033628.1 | chr10:39892759-39899238 |
| 1660 | AA536875 | NR_045143.1 | chr14:123169185-123176176 |
| 1661 | AA543186 | NR_027448.1 | chr2:25327449-25332571 |
| 1662 | AA543401 | NR_102273.1 | chr9:107192806-107194061 |
| 1663 | AA545190 | NR_033776.1 | chr6:10971467-10974378 |
| 1664 | AA619741 | NR_033627.1 | chr1:34633534-34636210 |
| 1665 | AA792892 | NM_178894.4 | chr5:94377339-94384334 |
| 1666 | AA986860 | NM_177604.3 | chr1:130731975-130744622 |
| 1667 | AA987161 | NM_001163246.2 | chr13:67589438-67609707 |
| 1668 | Aaas | NM_153416.2 | chr15:102338246-102350759 |
| 1669 | Aacs | NM_030210.1 | chr5:125475872-125517403 |
| 1670 | Aadac | NM_023383.1 | chr3:60031787-60040157 |
| 1671 | Aadacl2 | NM_001128091.1 | chr3:60006742-60025420 |
| 1672 | Aadacl3 | NM_001085503.2 | chr4:144453770-144463756 |
| 1673 | Aadat | NM_011834.2 | chr8:60506123-60545677 |
| 1674 | Aaed1 | NM_025370.2 | chr13:64291835-64312710 |
| 1675 | Aagab | NM_025857.1 | chr9:63602654-63641889 |
| 1676 | Aak1 | NM_001040106.2 | chr6:86849516-87003227 |
| 1677 | Aamdc | NM_001177945.1 | chr7:97550330-97579497 |
| 1678 | Aamp | NM_001190444.1 | chr1:74279839-74284738 |
| 1679 | Aanat | NM_009591.3 | chr11:116593686-116597680 |
| 1680 | Aar2 | NM_001164818.1 | chr2:156547575-156568972 |
| 1681 | Aard | NM_175503.3 | chr15:52040106-52045722 |
| 1682 | Aars | NM_146217.4 | chr8:111033841-111055569 |
| 1683 | Aars2 | NM_198608.2 | chr17:45506840-45520843 |
| 1684 | Aarsd1 | NM_144829.1 | chr11:101406839-101417433 |
| 1685 | Aasdh | NM_173765.3 | chr5:76875934-76905514 |
| 1686 | Aasdhppt | NM_026276.3 | chr9:4294792-4309494 |
| 1687 | Aass | NM_013930.4 | chr6:23072172-23132986 |
| 1688 | Aatf | NM_019816.1 | chr11:84422855-84513501 |
| 1689 | Aatk | NM_001198785.1 | chr11:120007315-120047145 |
| 1690 | AB041803 | NR_110469.1 | chr6:31165522-31218474 |
| 1691 | AB124611 | NM_001198794.1 | chr9:21526176-21545331 |
| 1692 | Abat | NM_001170978.1 | chr16:8513428-8621567 |
| 1693 | Abca1 | NM_013454.3 | chr4:53030788-53159895 |
| 1694 | Abca12 | NM_175210.3 | chr1:71243089-71414910 |
| 1695 | Abca13 | NM_178259.3 | chr11:9191941-9684259 |
| 1696 | Abca14 | NM_026458.4 | chr7:120203963-120325352 |
| 1697 | Abca15 | NM_177213.3 | chr7:120328683-120407687 |
| 1698 | Abca16 | NM_001278943.1 | chr7:120409646-120544813 |
| 1699 | Abca17 | NM_001031621.2 | chr17:24264278-24347252 |
| 1700 | Abca2 | NM_007379.2 | chr2:25428673-25448539 |
| 1701 | Abca3 | NM_001039581.2 | chr17:24352022-24410204 |
| 1702 | Abca4 | NM_007378.1 | chr3:122044459-122180061 |
| 1703 | Abca5 | NM_147219.2 | chr11:110269368-110337716 |
| 1704 | Abca6 | NM_001166556.1 | chr11:110236422-110251776 |
| 1705 | Abca7 | NM_013850.1 | chr10:79997614-80015572 |
| 1706 | Abca8a | NM_153145.4 | chr11:110025633-110095937 |
| 1707 | Abca8b | NM_013851.2 | chr11:109933405-109995816 |
| 1708 | Abca9 | NM_147220.2 | chr11:110100821-110168153 |
| 1709 | Abcb10 | NM_019552.2 | chr8:123952458-123983122 |
| 1710 | Abcb11 | NM_021022.3 | chr2:69238281-69342616 |
| 1711 | Abcb1a | NM_011076.2 | chr5:8660091-8748570 |
| 1712 | Abcb1b | NM_011075.2 | chr5:8798146-8866314 |
| 1713 | Abcb4 | NM_008830.2 | chr5:8893720-8959226 |
| 1714 | Abcb5 | NM_029961.2 | chr12:118867823-118966421 |
| 1715 | Abcb6 | NM_023732.3 | chr1:75171640-75180392 |
| 1716 | Abcb7 | NM_009592.1 | chrX:104280564-104413846 |
| 1717 | Abcb8 | NM_029020.2 | chr5:24394155-24409947 |
| 1718 | Abcb9 | NM_019875.2 | chr5:124061856-124095798 |
| 1719 | Abcc1 | NM_008576.3 | chr16:14361557-14474878 |
| 1720 | Abcc10 | NM_145140.2 | chr17:46303229-46325766 |
| 1721 | Abcc12 | NM_172912.4 | chr8:86504840-86566590 |
| 1722 | Abcc2 | NM_013806.2 | chr19:43782307-43838218 |
| 1723 | Abcc3 | NM_029600.3 | chr11:94343294-94392976 |
| 1724 | Abcc4 | NM_001033336.3 | chr14:118482691-118706219 |
| 1725 | Abcc5 | NM_013790.2 | chr16:20331303-20426394 |
| 1726 | Abcc6 | NM_018795.2 | chr7:45976379-46030286 |
| 1727 | Abcc8 | NM_011510.3 | chr7:46104522-46180033 |
| 1728 | Abcc9 | NM_001044720.1 | chr6:142587861-142702274 |
| 1729 | Abcd1 | NM_007435.1 | chrX:73716596-73738287 |
| 1730 | Abcd2 | NM_011994.2 | chr15:91145870-91191807 |
| 1731 | Abcd3 | NM_008991.2 | chr3:121758909-121815215 |
| 1732 | Abcd4 | NM_008992.2 | chr12:84602530-84617466 |
| 1733 | Abce1 | NM_015751.2 | chr8:79683441-79711740 |
| 1734 | Abcf1 | NM_013854.1 | chr17:35956818-35969750 |
| 1735 | Abcf2 | NM_001190443.1 | chr5:24565340-24577467 |
| 1736 | Abcf3 | NM_013852.2 | chr16:20548602-20561603 |
| 1737 | Abcg1 | NM_009593.2 | chr17:31057693-31117981 |
| 1738 | Abcg2 | NM_011920.3 | chr6:58596671-58692451 |
| 1739 | Abcg3 | NM_030239.2 | chr5:104935056-104982717 |
| 1740 | Abcg4 | NM_138955.3 | chr9:44273189-44288244 |
| 1741 | Abcg5 | NM_031884.1 | chr17:84658233-84682923 |
| 1742 | Abcg8 | NM_001286005.1 | chr17:84676301-84700333 |
| 1743 | Abhd1 | NM_021304.3 | chr5:30950105-30955091 |
| 1744 | Abhd10 | NM_001272070.1 | chr16:45729724-45742955 |
| 1745 | Abhd11 | NM_001190437.1 | chr5:135009151-135012175 |

Fig.21 - 10

| | | | |
|---|---|---|---|
| 1746 | Abhd11os | NR_026688.1 | chr5:135012121-135013157 |
| 1747 | Abhd12 | NM_024465.3 | chr2:150832514-150904731 |
| 1748 | Abhd12b | NM_001195033.1 | chr12:70154170-70183206 |
| 1749 | Abhd13 | NM_001081119.1 | chr8:9977716-9992154 |
| 1750 | Abhd14a | NM_001110271.1 | chr9:106440050-106447678 |
| 1751 | Abhd14b | NM_029631.3 | chr9:106448639-106452918 |
| 1752 | Abhd15 | NM_026185.4 | chr11:77515116-77520628 |
| 1753 | Abhd16a | NM_178592.3 | chr17:35089290-35102987 |
| 1754 | Abhd16b | NM_183181.2 | chr2:181493205-181494980 |
| 1755 | Abhd17a | NM_145421.2 | chr10:80583648-80590341 |
| 1756 | Abhd17b | NM_146096.3 | chr19:21653308-21685637 |
| 1757 | Abhd17c | NM_133722.2 | chr7:84109355-84151893 |
| 1758 | Abhd2 | NM_018811.6 | chr7:79273265-79361601 |
| 1759 | Abhd3 | NM_134130.1 | chr18:10644410-10706696 |
| 1760 | Abhd4 | NM_001205181.1 | chr14:54254128-54269169 |
| 1761 | Abhd5 | NM_026179.2 | chr9:122351615-122381523 |
| 1762 | Abhd6 | NM_025341.3 | chr14:8002901-8056555 |
| 1763 | Abhd8 | NM_022419.3 | chr8:71456699-71463657 |
| 1764 | Abi1 | NM_001077190.2 | chr2:22939988-23040241 |
| 1765 | Abi2 | NM_001198570.1 | chr1:60409618-60481163 |
| 1766 | Abi3 | NM_001163464.1 | chr11:95830071-95842476 |
| 1767 | Abi3bp | NM_001014399.2 | chr16:56477845-56690135 |
| 1768 | Abl1 | NM_001112703.2 | chr2:31688536-31804362 |
| 1769 | Abl2 | NM_001136104.1 | chr1:156558786-156649619 |
| 1770 | Ablim1 | NM_001103177.2 | chr19:57033263-57197631 |
| 1771 | Ablim2 | NM_001177696.1 | chr5:35757879-35884979 |
| 1772 | Ablim3 | NM_001164491.1 | chr18:61799392-61911852 |
| 1773 | Abo | NM_001290444.1 | chr2:26842502-26864995 |
| 1774 | Abr | NM_001291186.1 | chr11:76416731-76468465 |
| 1775 | Abra | NM_175456.4 | chr15:41865292-41869720 |
| 1776 | Abracl | NM_028440.1 | chr10:18011259-18023252 |
| 1777 | Abt1 | NM_013924.3 | chr13:23418360-23423866 |
| 1778 | Abtb1 | NM_030251.3 | chr6:88835913-88841935 |
| 1779 | Abtb2 | NM_178890.3 | chr2:103566309-103718423 |
| 1780 | Acaa1a | NM_130864.3 | chr9:119341293-119350295 |
| 1781 | Acaa1b | NM_146230.3 | chr9:119148042-119157093 |
| 1782 | Acaa2 | NM_177470.3 | chr18:74779211-74806207 |
| 1783 | Acaca | NM_133360.2 | chr11:84195437-84401651 |
| 1784 | Acacb | NM_133904.2 | chr5:114165517-114250758 |
| 1785 | Acad10 | NM_028037.4 | chr5:121621028-121660510 |
| 1786 | Acad11 | NM_175324.3 | chr9:104063702-104127646 |
| 1787 | Acad12 | NM_178799.3 | chr5:121598280-121618938 |
| 1788 | Acad8 | NM_025862.2 | chr9:26974138-26999549 |
| 1789 | Acad9 | NM_172678.3 | chr3:36065999-36092857 |
| 1790 | Acadl | NM_007381.4 | chr1:66830838-66863309 |
| 1791 | Acadm | NM_007382.5 | chr3:153922352-153944643 |
| 1792 | Acads | NM_007383.3 | chr5:115110298-115119346 |
| 1793 | Acadsb | NM_025826.4 | chr7:131410600-131446211 |
| 1794 | Acadvl | NM_017366.3 | chr11:70010182-70015428 |
| 1795 | Acan | NM_007424.2 | chr7:79053482-79115099 |
| 1796 | Acap1 | NM_153788.3 | chr11:69881566-69895539 |
| 1797 | Acap2 | NM_030138.2 | chr16:31092412-31201238 |
| 1798 | Acap3 | NM_207223.1 | chr4:155891874-155907251 |
| 1799 | Acat1 | NM_144784.3 | chr9:53580521-53610350 |
| 1800 | Acat2 | NM_009338.3 | chr17:12943041-12960725 |
| 1801 | Acat3 | NM_153151.3 | chr17:12923958-12940396 |
| 1802 | Acbd3 | NM_133225.3 | chr1:180726042-180754204 |
| 1803 | Acbd4 | NM_025988.2 | chr11:103101687-103112199 |
| 1804 | Acbd5 | NM_001102436.1 | chr2:23068200-23114512 |
| 1805 | Acbd6 | NM_001145781.1 | chr1:155558119-155587794 |
| 1806 | Acbd7 | NM_030063.2 | chr2:3336167-3340997 |
| 1807 | Accs | NM_001290782.1 | chr2:93833466-93849943 |
| 1808 | Accsl | NM_001033452.4 | chr2:93855359-93869157 |
| 1809 | Acd | NM_001012638.1 | chr8:105698158-105701095 |
| 1810 | Ace | NM_001281819.1 | chr11:105967944-105989964 |
| 1811 | Ace2 | NM_001130513.1 | chrX:164139341-164188418 |
| 1812 | Ace3 | NM_001101453.2 | chr11:105994674-106005443 |
| 1813 | Acer1 | NM_175731.4 | chr17:56953489-56982126 |
| 1814 | Acer2 | NM_001290541.1 | chr4:86874413-86920883 |
| 1815 | Acer3 | NM_025408.2 | chr7:98213659-98309527 |
| 1816 | Ache | NM_001290010.1 | chr5:137288253-137294466 |
| 1817 | Acin1 | NM_001085472.2 | chr14:54642160-54653701 |
| 1818 | Ackr1 | NM_010045.2 | chr1:173331885-173333503 |
| 1819 | Ackr2 | NM_001276719.1 | chr9:121898354-121911071 |
| 1820 | Ackr3 | NM_001271607.1 | chr1:90203979-90215722 |
| 1821 | Ackr4 | NM_145700.2 | chr9:104098137-104126643 |
| 1822 | Acly | NM_001199296.1 | chr11:100476351-100528000 |
| 1823 | Acmsd | NM_001033041.2 | chr1:127729412-127767564 |
| 1824 | Acn9 | NM_001077713.1 | chr6:6956017-7039220 |
| 1825 | Acnat1 | NM_001164565.1 | chr4:49443531-49451109 |
| 1826 | Acnat2 | NM_145368.2 | chr4:49379844-49408151 |
| 1827 | Aco1 | NM_007386.2 | chr4:40143264-40199009 |
| 1828 | Aco2 | NM_080633.2 | chr15:81872462-81915137 |
| 1829 | Acot1 | NM_012006.2 | chr12:84009501-84017669 |
| 1830 | Acot10 | NM_022816.2 | chr15:20665213-20666750 |
| 1831 | Acot11 | NM_025590.4 | chr4:106744560-106799831 |
| 1832 | Acot12 | NM_028790.3 | chr13:91741520-91786148 |
| 1833 | Acot13 | NM_025790.2 | chr13:24817954-24831489 |
| 1834 | Acot2 | NM_134188.3 | chr12:83987860-83993875 |
| 1835 | Acot3 | NM_134246.3 | chr12:84052150-84059564 |
| 1836 | Acot4 | NM_134247.3 | chr12:84038378-84044723 |
| 1837 | Acot5 | NM_145444.3 | chr12:84069324-84076019 |
| 1838 | Acot6 | NM_172580.1 | chr12:84100653-84109783 |
| 1839 | Acot7 | NM_001146057.1 | chr4:152186133-152271855 |
| 1840 | Acot8 | NM_133240.2 | chr2:164792767-164804881 |
| 1841 | Acot9 | NM_019736.4 | chrX:155262442-155297654 |
| 1842 | Acox1 | NM_001271898.1 | chr11:116171882-116199045 |
| 1843 | Acox2 | NM_001161667.1 | chr14:8225510-8258839 |
| 1844 | Acox3 | NM_030721.2 | chr5:35583059-35613801 |
| 1845 | Acoxl | NM_028765.3 | chr2:127854627-128123892 |
| 1846 | Acp1 | NM_001110239.1 | chr12:30893325-30911612 |
| 1847 | Acp2 | NM_007387.2 | chr2:91202911-91214098 |
| 1848 | Acp5 | NM_001102404.1 | chr9:22126726-22131865 |
| 1849 | Acp6 | NM_019800.4 | chr3:97158776-97176576 |
| 1850 | Acpp | NM_019807.2 | chr9:104298999-104337722 |
| 1851 | Acpt | NM_001195034.1 | chr7:44253086-44257204 |
| 1852 | Acr | NM_001205049.1 | chr15:89568325-89574585 |
| 1853 | Acrbp | NM_001127340.1 | chr6:125049926-125054193 |
| 1854 | Acrv1 | NM_007391.2 | chr9:36693219-36698837 |
| 1855 | Acsbg1 | NM_053178.2 | chr9:54604996-54661885 |
| 1856 | Acsbg2 | NM_001039114.1 | chr17:56843103-56874447 |
| 1857 | Acsf2 | NM_153807.2 | chr11:94557101-94601786 |
| 1858 | Acsf3 | NM_144932.3 | chr8:122775504-122817881 |
| 1859 | Acsl1 | NM_007981.4 | chr8:46471036-46536051 |
| 1860 | Acsl3 | NM_001033606.2 | chr1:78657824-78707743 |
| 1861 | Acsl4 | NM_001033600.1 | chrX:142317992-142390535 |
| 1862 | Acsl5 | NM_027976.2 | chr19:55253368-55296628 |
| 1863 | Acsl6 | NM_001033597.1 | chr11:54304203-54361539 |
| 1864 | Acsm1 | NM_054094.5 | chr7:119617827-119662515 |
| 1865 | Acsm2 | NM_001177977.1 | chr7:119554339-119600694 |
| 1866 | Acsm3 | NM_016870.3 | chr7:119760922-119784896 |
| 1867 | Acsm4 | NM_178414.3 | chr7:119690025-119714566 |
| 1868 | Acsm5 | NM_178758.3 | chr7:119526264-119543360 |
| 1869 | Acss1 | NM_080575.2 | chr2:150618110-150668240 |
| 1870 | Acss2 | NM_019811.3 | chr2:155518042-155562743 |
| 1871 | Acss2os | NR_040613.1 | chr2:155547039-155556930 |
| 1872 | Acss3 | NM_001142804.1 | chr10:106936163-107123664 |
| 1873 | Acta1 | NM_001272041.1 | chr8:123891757-123894775 |
| 1874 | Acta2 | NM_007392.3 | chr19:34240335-34255373 |
| 1875 | Actb | NM_007393.5 | chr5:142903114-142906754 |
| 1876 | Actbl2 | NM_175497.3 | chr13:111255012-111257749 |
| 1877 | Actc1 | NM_009608.3 | chr2:114047283-114052875 |
| 1878 | Actg1 | NM_009609.3 | chr11:120345686-120348495 |
| 1879 | Actg2 | NM_009610.2 | chr6:83512908-83536251 |
| 1880 | Actl10 | NM_001171640.1 | chr2:154551775-154553276 |
| 1881 | Actl11 | NM_026338.3 | chr9:107928468-107932461 |
| 1882 | Actl6a | NM_019673.2 | chr3:32708545-32726971 |
| 1883 | Actl6b | NM_031404.4 | chr5:137553554-137569573 |
| 1884 | Actl7a | NM_009611.3 | chr4:56743421-56744925 |
| 1885 | Actl7b | NM_025271.2 | chr4:56740004-56741425 |
| 1886 | Actl9 | NM_183282.2 | chr17:33432898-33434267 |
| 1887 | Actn1 | NM_134156.2 | chr12:80167541-80260371 |
| 1888 | Actn2 | NM_033268.4 | chr13:12269426-12340732 |
| 1889 | Actn3 | NM_013456.2 | chr19:4861215-4877909 |
| 1890 | Actn4 | NM_021895.2 | chr7:28893253-28962280 |
| 1891 | Actr10 | NM_019785.2 | chr12:70937856-70964717 |
| 1892 | Actr1a | NM_016860.1 | chr19:46376813-46395735 |
| 1893 | Actr1b | NM_146107.2 | chr1:36699201-36709925 |
| 1894 | Actr2 | NM_146243.2 | chr11:20062303-20112951 |
| 1895 | Actr3 | NM_001205385.1 | chr1:125392904-125435727 |
| 1896 | Actr3b | NM_001004365.2 | chr5:25759972-25850691 |
| 1897 | Actr5 | NM_175419.4 | chr2:158624912-158639211 |
| 1898 | Actr6 | NM_025914.2 | chr10:89711972-89732295 |
| 1899 | Actr8 | NM_027493.3 | chr14:29978336-29993221 |
| 1900 | Actrt1 | NM_028514.3 | chrX:46329006-46330345 |
| 1901 | Actrt2 | NM_028513.3 | chr4:154666427-154667867 |
| 1902 | Actrt3 | NM_029690.2 | chr3:30597072-30599870 |
| 1903 | Acvr1 | NM_001110204.1 | chr2:58446437-58566828 |
| 1904 | Acvr1b | NM_007395.3 | chr15:101174124-101212601 |
| 1905 | Acvr1c | NM_001033369.3 | chr2:58267452-58324807 |
| 1906 | Acvr2a | NM_007396.4 | chr2:48814108-48903264 |
| 1907 | Acvr2b | NM_007397.3 | chr9:119402177-119442148 |
| 1908 | Acvrl1 | NM_001277255.1 | chr15:101128521-101145336 |
| 1909 | Acy1 | NM_001276442.1 | chr9:106432980-106438236 |
| 1910 | Acy3 | NM_001302479.1 | chr19:3986570-3990007 |
| 1911 | Acyp1 | NM_025421.2 | chr12:85272397-85280435 |
| 1912 | Acyp2 | NM_029344.3 | chr11:30505991-30649396 |
| 1913 | Ada | NM_001272052.1 | chr2:163726570-163750239 |
| 1914 | Adad1 | NM_009350.3 | chr3:37063655-37111512 |
| 1915 | Adad2 | NM_029428.1 | chr8:119612746-119616926 |
| 1916 | Adal | NM_001290811.1 | chr2:121140427-121156680 |
| 1917 | Adam10 | NM_007399.3 | chr9:70679000-70780229 |
| 1918 | Adam11 | NM_001110778.1 | chr11:102761438-102780262 |
| 1919 | Adam12 | NM_007400.2 | chr7:133883198-134225097 |
| 1920 | Adam15 | NM_001037722.2 | chr3:89339639-89350010 |
| 1921 | Adam17 | NM_001277266.1 | chr12:21323508-21373632 |
| 1922 | Adam18 | NM_010084.2 | chr8:24602245-24674755 |
| 1923 | Adam19 | NM_001291890.1 | chr11:46054501-46147347 |
| 1924 | Adam1a | NM_172126.2 | chr5:121518603-121521695 |
| 1925 | Adam1b | NM_172125.2 | chr5:121500096-121502980 |
| 1926 | Adam2 | NM_009618.2 | chr14:66027328-66077733 |
| 1927 | Adam20 | NM_001009548.2 | chr8:40793272-40797303 |
| 1928 | Adam21 | NM_020330.4 | chr12:81558583-81568474 |
| 1929 | Adam22 | NM_001007220.3 | chr5:8092259-8368160 |
| 1930 | Adam23 | NM_001177600.1 | chr1:63445903-63573219 |
| 1931 | Adam24 | NM_010086.4 | chr8:40675093-40682199 |
| 1932 | Adam25 | NM_011781.2 | chr8:40752207-40756176 |
| 1933 | Adam26a | NM_010085.2 | chr8:43568275-43576707 |
| 1934 | Adam26b | NM_001009547.2 | chr8:43519863-43528137 |
| 1935 | Adam28 | NM_001048175.2 | chr14:68604997-68655842 |
| 1936 | Adam29 | NM_175939.3 | chr8:55870911-55906964 |
| 1937 | Adam3 | NM_009619.4 | chr8:24677233-24725825 |
| 1938 | Adam30 | NM_027665.1 | chr3:98160811-98163173 |
| 1939 | Adam32 | NM_153397.2 | chr8:24836142-24948804 |

Fig.21 - 11

| | | | | | | | |
|---|---|---|---|---|---|---|---|
| 1940 | Adam33 | NM_001163529.1 | chr2:131050816-131063814 | 2037 | Adra2c | NM_007418.3 | chr5:35278565-35281763 |
| 1941 | Adam34 | NM_145745.2 | chr8:43650308-43665560 | 2038 | Adrb1 | NM_007419.2 | chr19:56722371-56724862 |
| 1942 | Adam39 | NM_001025380.3 | chr8:40823008-40826861 | 2039 | Adrb2 | NM_007420.3 | chr18:62177712-62179981 |
| 1943 | Adam4 | NM_009620.1 | chr12:81419548-81421878 | 2040 | Adrb3 | NM_013462.3 | chr8:27225775-27229588 |
| 1944 | Adam5 | NM_001272057.1 | chr8:24732983-24824369 | 2041 | Adrbk1 | NM_001290818.1 | chr19:4285998-4306222 |
| 1945 | Adam6a | NM_174885.3 | chr12:113543907-113546414 | 2042 | Adrbk2 | NM_001285806.1 | chr5:112910477-113015538 |
| 1946 | Adam6b | NM_001009545.1 | chr12:113489564-113491835 | 2043 | Adrm1 | NM_019822.3 | chr2:180171587-180176283 |
| 1947 | Adam7 | NM_007402.2 | chr14:68497336-68533689 | 2044 | Adsl | NM_009634.6 | chr15:80948489-80970947 |
| 1948 | Adam8 | NM_001291066.2 | chr7:139978931-139992562 | 2045 | Adss | NM_007422.3 | chr1:177763177-177796509 |
| 1949 | Adam9 | NM_001270996.1 | chr8:24949610-25016922 | 2046 | Adssl1 | NM_007421.2 | chr12:112620046-112641355 |
| 1950 | Adamdec1 | NM_021475.2 | chr14:68563386-68582072 | 2047 | Adtrp | NM_001145875.1 | chr13:41763147-41847616 |
| 1951 | Adamts1 | NM_009621.5 | chr16:85793827-85803113 | 2048 | Aebp1 | NM_001291857.2 | chr11:5861865-5872248 |
| 1952 | Adamts10 | NM_172619.3 | chr17:33524195-33553782 | 2049 | Aebp2 | NM_001005605.2 | chr6:140623501-140655481 |
| 1953 | Adamts12 | NM_175501.2 | chr15:11064789-11346867 | 2050 | Aen | NM_001162939.1 | chr7:78895926-78908833 |
| 1954 | Adamts13 | NM_001001322.2 | chr2:26973415-27009625 | 2051 | Aes | NM_001276288.1 | chr10:81559443-81566371 |
| 1955 | Adamts14 | NM_001081127.1 | chr10:61197111-61273438 | 2052 | AF067061 | NM_199060.2 | chr13:120263094-120264517 |
| 1956 | Adamts15 | NM_001024139.1 | chr9:30899154-30922452 | 2053 | AF067063 | NM_001001449.2 | chr13:119827960-119830217 |
| 1957 | Adamts16 | NM_172053.2 | chr13:70727807-70841810 | 2054 | AF251705 | NM_134158.1 | chr11:114996768-115001880 |
| 1958 | Adamts17 | NM_001033877.4 | chr7:66839734-67152625 | 2055 | AF357355 | NR_028433.2 | chr12:109654626-109654697 |
| 1959 | Adamts18 | NM_172466.2 | chr8:113698136-113848839 | 2056 | AF357359 | NR_028434.1 | chr12:109650708-109650738 |
| 1960 | Adamts19 | NM_175506.3 | chr18:58836763-59053678 | 2057 | AF357399 | NR_028129.1 | chr7:28351539-28351633 |
| 1961 | Adamts2 | NM_175643.3 | chr11:50602085-50807573 | 2058 | AF357425 | NR_046302.1 | chr12:109636011-109636080 |
| 1962 | Adamts20 | NM_001164785.1 | chr15:94320331-94404350 | 2059 | AF357426 | NR_046303.1 | chr12:109642837-109642904 |
| 1963 | Adamts3 | NM_001081401.2 | chr5:89673840-89883334 | 2060 | AF366264 | NM_153093.3 | chr8:13835230-13838089 |
| 1964 | Adamts4 | NM_172845.2 | chr1:171250421-171259922 | 2061 | AF529169 | NM_153509.2 | chr9:89590034-89622986 |
| 1965 | Adamts5 | NM_011782.2 | chr16:85858156-85901125 | 2062 | Afap1 | NM_027373.2 | chr5:35893318-36003922 |
| 1966 | Adamts6 | NM_001081020.1 | chr13:104287872-104494763 | 2063 | Afap1l1 | NM_178928.4 | chr18:61730261-61786662 |
| 1967 | Adamts7 | NM_001003911.2 | chr9:90162977-90200102 | 2064 | Afap1l2 | NM_001177796.1 | chr19:56912353-57008575 |
| 1968 | Adamts8 | NM_013906.2 | chr9:30942562-30962858 | 2065 | Aff1 | NM_001080798.2 | chr5:103754572-103855322 |
| 1969 | Adamts9 | NM_175314.3 | chr6:92772698-92901441 | 2066 | Aff2 | NM_008032.3 | chrX:69360330-69868037 |
| 1970 | Adamtsl1 | NM_029967.3 | chr4:86053914-86428382 | 2067 | Aff3 | NM_001290814.1 | chr1:38175990-38627244 |
| 1971 | Adamtsl2 | NM_029981.1 | chr2:27079380-27108613 | 2068 | Aff4 | NM_033565.2 | chr11:53350766-53421830 |
| 1972 | Adamtsl3 | NM_001190374.1 | chr7:82335693-82614448 | 2069 | Afg3l1 | NM_054070.3 | chr8:123477861-123503916 |
| 1973 | Adamtsl4 | NM_144899.3 | chr3:95676200-95687917 | 2070 | Afg3l2 | NM_027130.1 | chr18:67404763-67449136 |
| 1974 | Adamtsl5 | NM_001285435.1 | chr10:80339792-80348448 | 2071 | Afm | NM_145146.2 | chr5:90518948-90553544 |
| 1975 | Adap1 | NM_172723.4 | chr5:139271875-139325464 | 2072 | Afmid | NM_027827.3 | chr11:117825918-117839908 |
| 1976 | Adap2 | NM_172133.1 | chr11:80154161-80178827 | 2073 | Afp | NM_007423.4 | chr5:90490713-90508907 |
| 1977 | Adar | NM_001038587.4 | chr3:89715021-89753455 | 2074 | Aftph | NM_001252503.2 | chr11:20685084-20741556 |
| 1978 | Adarb1 | NM_001024837.2 | chr10:77290726-77418273 | 2075 | Aga | NM_001005847.2 | chr8:53511701-53523422 |
| 1979 | Adarb2 | NM_001289530.1 | chr13:8202865-8760442 | 2076 | Agap1 | NM_001037136.1 | chr1:89454810-89895282 |
| 1980 | Adat1 | NM_013925.4 | chr8:111966907-111992302 | 2077 | Agap2 | NM_001033263.5 | chr10:127078906-127093170 |
| 1981 | Adat2 | NM_025748.4 | chr10:13552906-13563376 | 2078 | Agap3 | NM_001256431.1 | chr5:24452176-24483184 |
| 1982 | Adat3 | NM_001100606.1 | chr10:80602879-80607654 | 2079 | Agbl1 | NM_001199224.1 | chr7:76229886-77124698 |
| 1983 | Adc | NM_172875.4 | chr4:128932016-128962455 | 2080 | Agbl2 | NM_178755.3 | chr2:90782743-90816231 |
| 1984 | Adck1 | NM_001277296.1 | chr12:88360513-88461726 | 2081 | Agbl3 | NM_001289656.1 | chr6:34780431-34859459 |
| 1985 | Adck2 | NM_178873.3 | chr6:39573875-39588769 | 2082 | Agbl4 | NM_001048189.4 | chr4:110397697-111657519 |
| 1986 | Adck3 | NM_001163290.1 | chr1:180165237-180193485 | 2083 | Agbl5 | NM_001048192.2 | chr5:30889005-30906666 |
| 1987 | Adck4 | NM_133770.2 | chr7:27233012-27257949 | 2084 | Ager | NM_001271422.1 | chr17:34597849-34600937 |
| 1988 | Adck5 | NM_172960.3 | chr15:76576358-76595811 | 2085 | Agfg1 | NM_010472.3 | chr1:82839459-82901001 |
| 1989 | Adcy1 | NM_009622.1 | chr11:7063488-7178505 | 2086 | Agfg2 | NM_001303266.1 | chr5:137650463-137684806 |
| 1990 | Adcy10 | NM_173029.3 | chr1:165485182-165576773 | 2087 | Aggf1 | NM_025630.3 | chr13:95350682-95375357 |
| 1991 | Adcy2 | NM_153534.2 | chr13:68620042-68999541 | 2088 | Agk | NM_023538.2 | chr6:40325477-40396762 |
| 1992 | Adcy3 | NM_001159536.1 | chr12:4133396-4213524 | 2089 | Agl | NM_001081326.1 | chr3:116739998-116808166 |
| 1993 | Adcy4 | NM_080435.1 | chr14:55769091-55784019 | 2090 | Agmat | NM_001081408.1 | chr4:141746674-141759263 |
| 1994 | Adcy5 | NM_001012765.4 | chr16:35155635-35304549 | 2091 | Agmo | NM_178767.5 | chr12:37241638-37581932 |
| 1995 | Adcy6 | NM_007405.2 | chr15:98589985-98607633 | 2092 | Ago1 | NM_153403.2 | chr4:126435012-126468421 |
| 1996 | Adcy7 | NM_001037723.3 | chr8:88289065-88329962 | 2093 | Ago2 | NM_153178.4 | chr15:73101624-73184947 |
| 1997 | Adcy8 | NM_001291903.1 | chr15:64699034-64922296 | 2094 | Ago3 | NM_153402.2 | chr4:126340677-126429542 |
| 1998 | Adcy9 | NM_001291910.1 | chr16:4284885-4420498 | 2095 | Ago4 | NM_153177.3 | chr4:126489542-126533458 |
| 1999 | Adcyap1 | NM_009625.3 | chr17:93199421-93205489 | 2096 | Agpat1 | NM_001163379.1 | chr17:34605860-34613449 |
| 2000 | Adcyap1r1 | NM_001025372.2 | chr6:55451977-55501455 | 2097 | Agpat2 | NM_026212.2 | chr2:26593056-26604417 |
| 2001 | Add1 | NM_001024458.3 | chr5:34573713-34632305 | 2098 | Agpat3 | NM_053014.3 | chr10:78271562-78351700 |
| 2002 | Add2 | NM_001271857.1 | chr6:86028680-86124409 | 2099 | Agpat4 | NM_026644.2 | chr17:12119283-12219640 |
| 2003 | Add3 | NM_001164099.2 | chr19:53140442-53247326 | 2100 | Agpat5 | NM_026792.3 | chr8:18846278-18884413 |
| 2004 | Adgb | NM_001127353.2 | chr10:10335702-10472314 | 2101 | Agpat6 | NM_018743.4 | chr8:23172945-23208453 |
| 2005 | Adh1 | NM_007409.2 | chr3:138277644-138290691 | 2102 | Agpat9 | NM_172715.3 | chr5:100846228-100899102 |
| 2006 | Adh4 | NM_011996.2 | chr3:138415496-138430892 | 2103 | Agps | NM_172666.3 | chr2:75832176-75931350 |
| 2007 | Adh5 | NM_001288578.1 | chr3:138437199-138455499 | 2104 | Agr2 | NM_011783.2 | chr12:35992924-36004081 |
| 2008 | Adh6a | NM_026945.1 | chr3:138313285-138331134 | 2105 | Agr3 | NM_207531.3 | chr12:35925620-35949730 |
| 2009 | Adh6-ps1 | NR_033581.1 | chr3:138374120-138388291 | 2106 | Agrn | NM_021604.3 | chr4:156165289-156197488 |
| 2010 | Adh7 | NM_009626.4 | chr3:138217772-138232042 | 2107 | Agrp | NM_001271806.1 | chr8:105566694-105579845 |
| 2011 | Adhfe1 | NM_175236.4 | chr1:9548045-9577968 | 2108 | Agt | NM_007428.3 | chr8:124556586-124569707 |
| 2012 | Adi1 | NM_134052.2 | chr12:28675206-28682174 | 2109 | Agtpbp1 | NM_001048008.2 | chr13:59493403-59557347 |
| 2013 | Adig | NM_145635.2 | chr2:158502611-158508198 | 2110 | Agtr1a | NM_177322.3 | chr13:30336355-30382867 |
| 2014 | Adipoq | NM_009605.4 | chr16:23146535-23157968 | 2111 | Agtr1b | NM_175086.3 | chr3:20314472-20367177 |
| 2015 | Adipor1 | NM_028320.4 | chr1:134415377-134433350 | 2112 | Agtr2 | NM_007429.5 | chrX:21484623-21488833 |
| 2016 | Adipor2 | NM_197985.3 | chr6:119353149-119417483 | 2113 | Agtrap | NM_009642.4 | chr4:148077060-148088064 |
| 2017 | Adk | NM_001243041.1 | chr14:21076151-21448569 | 2114 | Agxt | NM_001276710.1 | chr1:93135239-93145421 |
| 2018 | Adm | NM_009627.1 | chr7:110627668-110629819 | 2115 | Agxt2 | NM_001031851.1 | chr15:10358578-10409738 |
| 2019 | Adm2 | NM_182928.5 | chr15:89322719-89324730 | 2116 | Ahctf1 | NM_026375.2 | chr1:179744903-179804015 |
| 2020 | Adnp | NM_009628.3 | chr2:168180964-168207112 | 2117 | Ahcy | NM_016661.3 | chr2:155059311-155074497 |
| 2021 | Adnp2 | NM_175028.1 | chr18:80127134-80151482 | 2118 | Ahcyl1 | NM_145542.3 | chr3:107663119-107696548 |
| 2022 | Ado | NM_001005419.2 | chr10:67544510-67548955 | 2119 | Ahcyl2 | NM_001171000.2 | chr6:29768442-29912310 |
| 2023 | Adora1 | NM_001008533.3 | chr1:134199214-134235457 | 2120 | Ahdc1 | NM_146155.3 | chr4:133011505-133078110 |
| 2024 | Adora2a | NM_009630.3 | chr10:75316876-75334792 | 2121 | Ahi1 | NM_001177776.1 | chr10:21040819-21080429 |
| 2025 | Adora2b | NM_007413.4 | chr11:62248983-62266452 | 2122 | Ahnak | NM_001039959.2 | chr19:8989283-9076926 |
| 2026 | Adora3 | NM_001174169.2 | chr3:105870857-105908928 | 2123 | Ahr | NM_013464.4 | chr12:35497978-35534989 |
| 2027 | Adpgk | NM_028121.2 | chr9:59291571-59316200 | 2124 | Ahrr | NM_009644.2 | chr13:74211117-74292309 |
| 2028 | Adprh | NM_007414.3 | chr16:38445398-38452689 | 2125 | Ahsa1 | NM_146036.1 | chr12:87266727-87273952 |
| 2029 | Adprhl1 | NM_172750.3 | chr8:13235661-13254162 | 2126 | Ahsa2 | NM_001290654.1 | chr11:23487881-23498039 |
| 2030 | Adprhl2 | NM_133883.2 | chr4:126316350-126321703 | 2127 | Ahsg | NM_001276449.1 | chr16:22892014-22899451 |
| 2031 | Adprm | NM_025510.3 | chr11:67037879-67052618 | 2128 | AI115009 | NR_040386.1 | chr3:152666912-152681432 |
| 2032 | Adra1a | NM_001271759.1 | chr14:66635250-66733462 | 2129 | AI118078 | NM_172923.3 | chr9:55326948-55438344 |
| 2033 | Adra1b | NM_001284380.1 | chr11:43774604-43901237 | 2130 | AI182371 | NM_001243102.1 | chr2:35081860-35100685 |
| 2034 | Adra1d | NM_013460.4 | chr2:131545356-131562285 | 2131 | AI197445 | NR_045083.1 | chr13:107469821-107479188 |
| 2035 | Adra2a | NM_007417.4 | chr19:54045181-54048982 | 2132 | AI314180 | NM_172381.2 | chr4:58800030-58912725 |
| 2036 | Adra2b | NM_009633.3 | chr2:127363285-127367221 | 2133 | AI314278 | NR_102276.1 | chr7:89426305-89432077 |

Fig.21 - 12

| | | | | | | | |
|---|---|---|---|---|---|---|---|
| 2134 | AI317395 | NM_144821.4 | chr10:40001572-40025268 | 2231 | Akt1 | NM_001165894.1 | chr12:112653820-112674276 |
| 2135 | AI413582 | NM_001002895.2 | chr17:27563768-27565727 | 2232 | Akt1s1 | NM_001253920.1 | chr7:44850006-44855421 |
| 2136 | AI414108 | NR_027907.1 | chr9:27352684-27357543 | 2233 | Akt2 | NM_001110208.1 | chr7:27591559-27639453 |
| 2137 | AI427809 | NR_033139.1 | chr4:53261355-53270232 | 2234 | Akt3 | NM_011785.3 | chr1:177022114-177248767 |
| 2138 | AI429214 | NM_001039220.3 | chr8:36993574-36995533 | 2235 | Aktip | NM_010241.5 | chr8:91123498-91135494 |
| 2139 | AI450353 | NR_028364.1 | chr11:83293429-83294929 | 2236 | Alad | NM_001276446.1 | chr4:62505983-62519909 |
| 2140 | AI462493 | NM_001160356.1 | chr19:8880013-8880933 | 2237 | Alas1 | NM_001291835.1 | chr9:106233454-106247730 |
| 2141 | AI463170 | NR_046044.1 | chr12:76546971-76547925 | 2238 | Alas2 | NM_001102446.1 | chrX:150547416-150570622 |
| 2142 | AI464131 | NM_001085515.2 | chr4:41495600-41503075 | 2239 | Alb | NM_009654.4 | chr5:90460888-90476603 |
| 2143 | AI467606 | NM_178901.3 | chr7:127091435-127094049 | 2240 | Alcam | NM_009655.2 | chr16:52248995-52452997 |
| 2144 | AI504432 | NR_033498.1 | chr3:107039503-107054322 | 2241 | Aldh16a1 | NM_145954.1 | chr7:45141839-45154538 |
| 2145 | AI506816 | NR_015554.2 | chr5:23692260-23712667 | 2242 | Aldh18a1 | NM_019698.2 | chr19:40550256-40588463 |
| 2146 | AI507597 | NR_033566.1 | chr4:141614369-141615604 | 2243 | Aldh1a1 | NM_013467.3 | chr19:20601981-20643462 |
| 2147 | AI593442 | NM_001286641.1 | chr9:52673031-52679780 | 2244 | Aldh1a2 | NM_009022.4 | chr9:71215788-71296243 |
| 2148 | AI597479 | NM_133818.1 | chr1:43098709-43115946 | 2245 | Aldh1a3 | NM_053080.3 | chr7:66390892-66427477 |
| 2149 | AI606473 | NR_040387.1 | chr3:154330820-154334687 | 2246 | Aldh1a7 | NM_011921.2 | chr19:20692952-20727556 |
| 2150 | AI607873 | NM_001204910.1 | chr1:173723427-173741809 | 2247 | Aldh1b1 | NM_028270.4 | chr4:45799021-45804608 |
| 2151 | AI646519 | NR_040330.1 | chr2:147361542-147362793 | 2248 | Aldh1l1 | NM_027406.1 | chr6:90550847-90599171 |
| 2152 | AI661453 | NM_145489.2 | chr17:47436638-47470638 | 2249 | Aldh1l2 | NM_153543.2 | chr10:83487446-83534140 |
| 2153 | AI662270 | NR_015519.1 | chr11:83223575-83226584 | 2250 | Aldh2 | NM_009656.4 | chr5:121566026-121593824 |
| 2154 | AI747448 | NM_001033199.3 | chr3:144910920-144932529 | 2251 | Aldh3a1 | NM_001112725.1 | chr11:61208741-61218416 |
| 2155 | AI837181 | NM_001256515.1 | chr19:5425143-5427316 | 2252 | Aldh3a2 | NM_007437.5 | chr11:61244751-61267186 |
| 2156 | AI839979 | NR_102275.1 | chr5:31569591-31571397 | 2253 | Aldh3b1 | NM_026316.2 | chr19:3913490-3929716 |
| 2157 | AI846148 | NM_001033139.3 | chr19:7356463-7383026 | 2254 | Aldh3b2 | NM_001177438.1 | chr19:3972327-3981665 |
| 2158 | AI847159 | NR_045264.1 | chr2:129178147-129180673 | 2255 | Aldh4a1 | NM_175438.4 | chr4:139622893-139649691 |
| 2159 | AI848285 | NM_001207021.1 | chr15:82206951-82212815 | 2256 | Aldh5a1 | NM_172532.3 | chr13:24907578-24937661 |
| 2160 | AI854517 | NR_040311.1 | chr7:79500025-79534403 | 2257 | Aldh6a1 | NM_134042.3 | chr12:84430720-84451024 |
| 2161 | AI854703 | NR_027236.1 | chr6:48628166-48633688 | 2258 | Aldh7a1 | NM_001127338.1 | chr18:56525735-56572939 |
| 2162 | AI987944 | NM_001199330.1 | chr7:41372929-41393379 | 2259 | Aldh8a1 | NM_178713.4 | chr10:21377299-21396578 |
| 2163 | Aicda | NM_009645.2 | chr6:122553808-122564180 | 2260 | Aldh9a1 | NM_019993.3 | chr1:167349990-167368530 |
| 2164 | Aida | NM_181732.4 | chr1:183297059-183324501 | 2261 | Aldoa | NM_001177307.1 | chr7:126795233-126799178 |
| 2165 | Aif1 | NM_019467.2 | chr17:35170991-35176001 | 2262 | Aldoart1 | NM_001199270.1 | chr4:72850582-72852634 |
| 2166 | Aif1l | NM_145144.1 | chr2:31950302-31973442 | 2263 | Aldoart2 | NM_001277340.1 | chr12:55565204-55566896 |
| 2167 | Aifm1 | NM_001290364.1 | chrX:48474943-48513563 | 2264 | Aldob | NM_144903.3 | chr4:49535992-49549546 |
| 2168 | Aifm2 | NM_001039194.3 | chr10:61715262-61738652 | 2265 | Aldoc | NM_009657.4 | chr11:78324197-78327778 |
| 2169 | Aifm3 | NM_001291070.1 | chr16:17491920-17507482 | 2266 | Alg1 | NM_145362.2 | chr16:5233620-5244907 |
| 2170 | Aig1 | NM_025446.3 | chr10:13652708-13868830 | 2267 | Alg10b | NM_001033441.3 | chr15:90224310-90230554 |
| 2171 | Aim1 | NM_172393.2 | chr10:43950306-44004846 | 2268 | Alg11 | NM_001243161.1 | chr8:22060720-22071627 |
| 2172 | Aim1l | NM_001162970.1 | chr4:134068451-134092504 | 2269 | Alg12 | NM_001142357.1 | chr15:88805242-88819318 |
| 2173 | Aim2 | NM_001013779.2 | chr1:173420603-173466036 | 2270 | Alg13 | NM_026247.3 | chrX:144317965-144325197 |
| 2174 | Aimp1 | NM_007926.2 | chr3:132660497-132683879 | 2271 | Alg14 | NM_024178.2 | chr3:121291816-121362011 |
| 2175 | Aimp2 | NM_001172146.1 | chr5:143902703-143909839 | 2272 | Alg2 | NM_019998.3 | chr4:47469832-47474367 |
| 2176 | Aip | NM_001276284.1 | chr19:4113755-4121575 | 2273 | Alg3 | NM_145939.2 | chr16:20605457-20610749 |
| 2177 | Aipl1 | NM_053245.2 | chr11:72028721-72037509 | 2274 | Alg5 | NM_025442.3 | chr3:54735538-54749795 |
| 2178 | Aire | NM_001271549.1 | chr10:78030021-78043610 | 2275 | Alg6 | NM_001081264.1 | chr4:99715629-99763460 |
| 2179 | Airn | NR_002853.2 | chr17:12741310-12815557 | 2276 | Alg8 | NM_199035.2 | chr7:97371616-97392158 |
| 2180 | Ajap1 | NM_001099299.1 | chr4:153373220-153482830 | 2277 | Alg9 | NM_133981.2 | chr9:50775224-50843639 |
| 2181 | Ajuba | NM_010590.5 | chr14:54567468-54577661 | 2278 | Alk | NM_007439.2 | chr17:71868987-72604307 |
| 2182 | AK010878 | NM_001142938.1 | chr12:102753274-102757810 | 2279 | Alkbh1 | NM_001102565.1 | chr12:87428077-87443839 |
| 2183 | Ak1 | NM_001198790.1 | chr2:32621757-32635058 | 2280 | Alkbh2 | NM_175016.2 | chr5:114123933-114128176 |
| 2184 | AK129341 | NM_001045524.1 | chr9:8076632-8134294 | 2281 | Alkbh3 | NM_026944.1 | chr2:93980633-94010730 |
| 2185 | Ak2 | NM_001033966.4 | chr4:128993223-129008723 | 2282 | Alkbh4 | NM_028070.1 | chr5:136138780-136141614 |
| 2186 | Ak3 | NM_021299.1 | chr19:29020831-29047902 | 2283 | Alkbh5 | NM_172943.4 | chr11:60537682-60558512 |
| 2187 | Ak4 | NM_001177602.1 | chr4:101419288-101467771 | 2284 | Alkbh6 | NM_198027.2 | chr7:30308752-30314303 |
| 2188 | Ak5 | NM_001081277.1 | chr3:152462814-152668140 | 2285 | Alkbh7 | NM_025538.3 | chr17:56997338-56999336 |
| 2189 | Ak6 | NM_027592.3 | chr13:100651342-100666415 | 2286 | Alkbh8 | NM_026303.1 | chr9:3335230-3385846 |
| 2190 | Ak7 | NM_030187.1 | chr12:105705981-105782447 | 2287 | Allc | NM_053156.2 | chr12:28553755-28582483 |
| 2191 | Ak8 | NM_001033874.2 | chr2:28700160-28813165 | 2288 | Alms1 | NM_145223.2 | chr6:85587530-85702751 |
| 2192 | Akap1 | NM_001042541.1 | chr11:88830791-88864586 | 2289 | Alms1-ps2 | NR_040440.2 | chr6:85792116-85804057 |
| 2193 | Akap10 | NM_019921.3 | chr11:61871306-61930257 | 2290 | Alox12 | NM_007440.4 | chr11:70241454-70255341 |
| 2194 | Akap11 | NM_001164503.1 | chr14:78492245-78536860 | 2291 | Alox12b | NM_009659.2 | chr11:69157071-69169791 |
| 2195 | Akap12 | NM_031185.3 | chr10:4266328-4359471 | 2292 | Alox12e | NM_145684.1 | chr11:70315612-70322518 |
| 2196 | Akap13 | NM_029332.1 | chr7:75455533-75754609 | 2293 | Alox15 | NM_009660.3 | chr11:70344146-70352031 |
| 2197 | Akap14 | NM_001033785.2 | chrX:37150697-37168842 | 2294 | Alox5 | NM_009662.2 | chr6:116410070-116461178 |
| 2198 | Akap17b | NM_001081956.1 | chrX:36608182-36645414 | 2295 | Alox5ap | NM_009663.2 | chr5:149265003-149288153 |
| 2199 | Akap2 | NM_001035532.2 | chr4:57845247-57896984 | 2296 | Alox8 | NM_009661.4 | chr11:69183884-69197843 |
| 2200 | Akap3 | NM_009650.2 | chr6:126853097-126874308 | 2297 | Aloxe3 | NM_011786.2 | chr11:69126376-69149115 |
| 2201 | Akap4 | NM_001042542.2 | chrX:7068209-7078606 | 2298 | Alpi | NM_001081082.2 | chr1:87098001-87101606 |
| 2202 | Akap5 | NM_001101471.1 | chr12:76324890-76334151 | 2299 | Alpk1 | NM_027808.1 | chr3:127670309-127780527 |
| 2203 | Akap6 | NM_198111.2 | chr12:52699382-53151015 | 2300 | Alpk2 | NM_001037294.1 | chr18:65265530-65393888 |
| 2204 | Akap7 | NM_018747.4 | chr10:25169089-25299163 | 2301 | Alpk3 | NM_054085.2 | chr7:81057599-81105612 |
| 2205 | Akap8 | NM_019774.5 | chr17:32303671-32321238 | 2302 | Alpl | NM_001287172.1 | chr4:137741730-137766475 |
| 2206 | Akap8l | NM_017476.2 | chr17:32321423-32350577 | 2303 | Alppl2 | NM_007433.3 | chr1:87086690-87089928 |
| 2207 | Akap9 | NM_194462.2 | chr5:3928185-4080204 | 2304 | Als2 | NM_001159948.2 | chr1:59162755-59237093 |
| 2208 | Akip1 | NM_020616.1 | chr7:109703736-109712181 | 2305 | Als2cl | NM_001146059.1 | chr9:110880173-110900530 |
| 2209 | Akirin1 | NM_023423.3 | chr4:123735194-123750299 | 2306 | Als2cr11 | NM_175200.4 | chr1:59050505-59094900 |
| 2210 | Akirin2 | NM_001007589.3 | chr4:34550614-34566908 | 2307 | Als2cr12 | NM_175370.5 | chr1:58658120-58695989 |
| 2211 | Akna | NM_001045514.3 | chr4:63367122-63403445 | 2308 | Alx1 | NM_172553.4 | chr10:103007846-103028777 |
| 2212 | Aknad1 | NM_177859.3 | chr3:108739657-108782309 | 2309 | Alx3 | NM_007441.3 | chr3:107595030-107605875 |
| 2213 | Akp3 | NM_007432.2 | chr1:87125007-87127912 | 2310 | Alx4 | NM_007442.3 | chr2:93642433-93681339 |
| 2214 | Akr1a1 | NM_021473.3 | chr4:116636509-116651674 | 2311 | Alyref | NM_011568.1 | chr11:120594515-120598365 |
| 2215 | Akr1b10 | NM_172398.3 | chr6:34384246-34396949 | 2312 | Alyref2 | NM_019484.4 | chr1:171503477-171504750 |
| 2216 | Akr1b3 | NM_009658.3 | chr6:34303929-34317489 | 2313 | Amacr | NM_008537.4 | chr15:10981755-10996624 |
| 2217 | Akr1b7 | NM_009731.2 | chr6:34412361-34423137 | 2314 | Ambn | NM_009664.2 | chr5:88455990-88468529 |
| 2218 | Akr1b8 | NM_008012.1 | chr6:34354163-34368454 | 2315 | Ambp | NM_007443.4 | chr4:63143278-63154142 |
| 2219 | Akr1c12 | NM_013777.2 | chr13:4268171-4279399 | 2316 | Ambra1 | NM_001080754.1 | chr2:91730137-91918849 |
| 2220 | Akr1c13 | NM_013778.2 | chr13:4191186-4205603 | 2317 | Amd1 | NM_009665.5 | chr10:40287457-40302188 |
| 2221 | Akr1c14 | NM_134072.1 | chr13:4059590-4090422 | 2318 | Amd2 | NM_007444.3 | chr10:40287464-40302186 |
| 2222 | Akr1c18 | NM_134066.2 | chr13:4132626-4150631 | 2319 | Amdhd1 | NM_027908.1 | chr10:93523337-93540033 |
| 2223 | Akr1c19 | NM_001013785.3 | chr13:4233739-4248359 | 2320 | Amdhd2 | NM_172935.4 | chr17:24155832-24163733 |
| 2224 | Akr1c20 | NM_054080.1 | chr13:4507158-4523333 | 2321 | Amelx | NM_001081978.2 | chrX:169176113-169187209 |
| 2225 | Akr1c21 | NM_029901.2 | chr13:4574074-4586543 | 2322 | Amer1 | NM_175179.4 | chrX:95420313-95444840 |
| 2226 | Akr1c6 | NM_030611.3 | chr13:4434342-4457530 | 2323 | Amer2 | NM_001164705.1 | chr14:60378285-60381003 |
| 2227 | Akr1cl | NM_027582.4 | chr1:65013077-65032759 | 2324 | Amer3 | NM_213727.2 | chr1:34579692-34590944 |
| 2228 | Akr1d1 | NM_145364.2 | chr6:37530172-37568815 | 2325 | Amfr | NM_011787.2 | chr8:93971587-94012640 |
| 2229 | Akr1e1 | NM_018859.2 | chr13:4592489-4609164 | 2326 | Amh | NM_007445.2 | chr10:80805247-80807648 |
| 2230 | Akr7a5 | NM_025337.3 | chr4:139310743-139318786 | 2327 | Amhr2 | NM_144547.2 | chr15:102445366-102454639 |

Fig.21・13

| | | | | | | | |
|---|---|---|---|---|---|---|---|
| 2328 | Amica1 | NM_001005421.4 | chr9:45079182-45108531 | 2425 | Ankrd44 | NM_001081433.3 | chr1:54645339-54926387 |
| 2329 | Amigo1 | NM_001004293.2 | chr3:108186288-108192286 | 2426 | Ankrd45 | NM_028664.1 | chr1:161142711-161170507 |
| 2330 | Amigo2 | NM_001164563.1 | chr15:97244073-97247287 | 2427 | Ankrd46 | NM_175134.4 | chr15:36477667-36496791 |
| 2331 | Amigo3 | NM_177275.4 | chr9:108053158-108055701 | 2428 | Ankrd49 | NM_019683.3 | chr9:14780197-14782964 |
| 2332 | Ammecr1 | NM_019496.3 | chrX:142853473-142966728 | 2429 | Ankrd50 | NM_001167883.1 | chr3:38449260-38484816 |
| 2333 | Ammecr1l | NM_001242430.1 | chr18:31760615-31784083 | 2430 | Ankrd52 | NM_172790.2 | chr10:128377123-128394006 |
| 2334 | Amn | NM_033603.3 | chr12:111271095-111276426 | 2431 | Ankrd53 | NM_029245.3 | chr6:83762645-83768326 |
| 2335 | Amn1 | NM_001113424.1 | chr6:149157576-149188712 | 2432 | Ankrd54 | NM_144849.1 | chr15:79053093-79062859 |
| 2336 | Amot | NM_001290274.1 | chrX:145451867-145487639 | 2433 | Ankrd55 | NM_001168403.1 | chr13:112288450-112384002 |
| 2337 | Amotl1 | NM_001081395.1 | chr9:14541966-14615000 | 2434 | Ankrd6 | NM_001012450.1 | chr4:32804034-32923505 |
| 2338 | Amotl2 | NM_019764.2 | chr9:102717803-102733417 | 2435 | Ankrd60 | NM_001199955.1 | chr2:173572389-173578341 |
| 2339 | Ampd1 | NM_001033303.2 | chr3:103074013-103099720 | 2436 | Ankrd61 | NM_025732.2 | chr5:143890740-143895067 |
| 2340 | Ampd2 | NM_001289719.1 | chr3:108074061-108086666 | 2437 | Ankrd63 | NM_001081971.1 | chr2:118699102-118703963 |
| 2341 | Ampd3 | NM_001276301.1 | chr7:110768205-110812408 | 2438 | Ankrd66 | NM_001254953.1 | chr17:43534173-43543639 |
| 2342 | Amph | NM_001289546.1 | chr13:18948350-19150919 | 2439 | Ankrd7 | NM_001167757.1 | chr6:18866317-18879584 |
| 2343 | Amt | NM_001013814.1 | chr9:108296921-108301597 | 2440 | Ankrd9 | NM_175207.1 | chr12:110975352-110979021 |
| 2344 | Amtn | NM_027793.1 | chr5:88376107-88385916 | 2441 | Anks1 | NM_001286040.1 | chr17:27909305-28062779 |
| 2345 | Amy1 | NM_001110505.1 | chr3:113555951-113577750 | 2442 | Anks1b | NM_001128086.2 | chr10:89873508-90972984 |
| 2346 | Amy2a2 | NM_001160152.1 | chr3:113279836-113356658 | 2443 | Anks3 | NM_028301.4 | chr16:4941414-4964237 |
| 2347 | Amy2a5 | NM_001042711.2 | chr3:113249177-113356520 | 2444 | Anks4b | NM_028085.2 | chr7:120173857-120183716 |
| 2348 | Amy2b | NM_001190403.1 | chr3:113147655-113156727 | 2445 | Anks6 | NM_001024136.1 | chr4:47015688-47057306 |
| 2349 | Amz1 | NM_173405.2 | chr5:140724126-140753312 | 2446 | Ankub1 | NM_001033349.2 | chr3:57667421-57692537 |
| 2350 | Amz2 | NM_001252193.1 | chr11:109426219-109438148 | 2447 | Ankzf1 | NM_001267620.1 | chr1:75192159-75195538 |
| 2351 | Anapc1 | NM_008569.2 | chr2:128610082-128687395 | 2448 | Anln | NM_028390.3 | chr9:22331213-22389206 |
| 2352 | Anapc10 | NM_026904.2 | chr8:79711819-79777321 | 2449 | Ano1 | NM_001242349.1 | chr7:144588548-144738592 |
| 2353 | Anapc11 | NM_001038230.2 | chr11:120598531-120608198 | 2450 | Ano10 | NM_001271873.1 | chr9:122175873-122294423 |
| 2354 | Anapc13 | NM_181394.3 | chr9:102626295-102634244 | 2451 | Ano2 | NM_153589.2 | chr6:125690418-126040128 |
| 2355 | Anapc15 | NM_001291348.1 | chr7:101896330-101899545 | 2452 | Ano3 | NM_001128103.2 | chr2:110655200-110950244 |
| 2356 | Anapc16 | NM_025514.2 | chr10:59987908-60003112 | 2453 | Ano4 | NM_001277188.1 | chr10:88948993-89257790 |
| 2357 | Anapc2 | NM_175300.4 | chr2:25272465-25285916 | 2454 | Ano5 | NM_001271879.1 | chr7:51511028-51598707 |
| 2358 | Anapc4 | NM_024213.2 | chr5:52834134-52866734 | 2455 | Ano6 | NM_001253813.1 | chr15:95790842-95975472 |
| 2359 | Anapc5 | NM_001042491.2 | chr5:122787460-122821342 | 2456 | Ano7 | NM_001271884.1 | chr1:93373895-93404304 |
| 2360 | Anapc7 | NM_019805.4 | chr5:122422443-122444911 | 2457 | Ano8 | NM_001164679.1 | chr8:71476018-71486067 |
| 2361 | Ang | NM_001161731.2 | chr14:51091076-51102009 | 2458 | Ano9 | NM_178381.3 | chr7:141101218-141117806 |
| 2362 | Ang2 | NM_007449.2 | chr14:51195485-51195923 | 2459 | Anp32a | NM_009672.3 | chr9:62341342-62378805 |
| 2363 | Ang3 | NM_001123394.2 | chr14:43957068-43963080 | 2460 | Anp32b | NM_130889.2 | chr4:46451116-46472523 |
| 2364 | Ang4 | NM_177544.4 | chr14:51763891-51773590 | 2461 | Anp32e | NM_001253757.1 | chr3:95929256-95947387 |
| 2365 | Ang5 | NM_007448.3 | chr14:43957068-43963080 | 2462 | Anpep | NM_008486.2 | chr7:79821802-79842352 |
| 2366 | Ang6 | NM_001011876.2 | chr14:44001663-44006418 | 2463 | Antxr1 | NM_054041.2 | chr6:87133852-87335775 |
| 2367 | Angel1 | NM_144524.2 | chr12:86700501-86726460 | 2464 | Antxr2 | NM_133738.1 | chr5:97884687-98030962 |
| 2368 | Angel2 | NM_001199020.1 | chr1:190925107-190946491 | 2465 | Antxrl | NM_172808.2 | chr14:34053469-34075999 |
| 2369 | Angpt1 | NM_001286062.1 | chr15:42424666-42676977 | 2466 | Anxa1 | NM_010730.2 | chr19:20373433-20390671 |
| 2370 | Angpt2 | NM_007426.4 | chr8:18690262-18741562 | 2467 | Anxa10 | NM_001136089.2 | chr8:62057041-62123193 |
| 2371 | Angpt4 | NM_009641.1 | chr2:151911331-151944654 | 2468 | Anxa11 | NM_013469.2 | chr14:25842154-25886804 |
| 2372 | Angptl1 | NM_028333.2 | chr1:156839022-156861079 | 2469 | Anxa13 | NM_027211.2 | chr15:58341463-58389274 |
| 2373 | Angptl2 | NM_011923.4 | chr2:33215960-33247714 | 2470 | Anxa2 | NM_007585.3 | chr9:69453682-69491785 |
| 2374 | Angptl3 | NM_013913.4 | chr4:99030953-99038192 | 2471 | Anxa3 | NM_013470.2 | chr5:96793384-96845968 |
| 2375 | Angptl4 | NM_020581.2 | chr17:33774899-33781575 | 2472 | Anxa4 | NM_013471.2 | chr6:86736839-86793584 |
| 2376 | Angptl6 | NM_145154.2 | chr9:20873808-20879710 | 2473 | Anxa5 | NM_009673.2 | chr3:36448923-36475887 |
| 2377 | Angptl7 | NM_001039554.3 | chr4:148495279-148500462 | 2474 | Anxa6 | NM_001110211.1 | chr11:54978961-55033471 |
| 2378 | Ank | NM_020332.4 | chr15:27466676-27594907 | 2475 | Anxa7 | NM_001110741.1 | chr14:20455260-20480133 |
| 2379 | Ank1 | NM_001110783.3 | chr8:22974835-23150497 | 2476 | Anxa8 | NM_001281845.1 | chr14:34085978-34100568 |
| 2380 | Ank2 | NM_001034168.1 | chr3:126921606-126943386 | 2477 | Anxa9 | NM_001085383.1 | chr3:95296093-95306804 |
| 2381 | Ank3 | NM_009670.4 | chr10:69925531-70027436 | 2478 | Aoah | NM_001281854.1 | chr13:20794112-20851907 |
| 2382 | Ankar | NM_176980.4 | chr1:72642979-72700564 | 2479 | Aoc1 | NM_001161621.1 | chr6:48904985-48909187 |
| 2383 | Ankdd1b | NM_001042714.1 | chr13:96416133-96471160 | 2480 | Aoc2 | NM_178932.1 | chr11:101325062-101329694 |
| 2384 | Ankef1 | NM_175667.4 | chr2:136532320-136555854 | 2481 | Aoc3 | NM_009675.2 | chr11:101330605-101339430 |
| 2385 | Ankfn1 | NM_001080933.1 | chr11:89421085-89538555 | 2482 | Aox1 | NM_009676.2 | chr1:58029968-58106410 |
| 2386 | Ankfy1 | NM_009671.5 | chr11:72690001-72772146 | 2483 | Aox2 | NM_001008419.2 | chr1:58278325-58379264 |
| 2387 | Ankhd1 | NM_175375.3 | chr18:36560602-36658908 | 2484 | Aox3 | NM_023617.2 | chr1:58113135-58200452 |
| 2388 | Ankib1 | NM_001003909.4 | chr5:3689998-3803124 | 2485 | Aox4 | NM_023631.2 | chr1:58210396-58268596 |
| 2389 | Ankk1 | NM_172922.3 | chr9:49415221-49427021 | 2486 | Ap1ar | NM_145964.2 | chr3:127807264-127837492 |
| 2390 | Ankle1 | NM_172756.3 | chr8:71406011-71410542 | 2487 | Ap1b1 | NM_001243043.1 | chr11:4947520-5042794 |
| 2391 | Ankle2 | NM_001253814.1 | chr5:110231003-110256651 | 2488 | Ap1g1 | NM_009677.6 | chr8:109778582-109864209 |
| 2392 | Ankmy1 | NM_172850.3 | chr1:92870128-92902906 | 2489 | Ap1g2 | NM_007455.5 | chr14:55098834-55106593 |
| 2393 | Ankmy2 | NM_146033.3 | chr12:36157123-36197291 | 2490 | Ap1m1 | NM_007456.4 | chr8:72240131-72257379 |
| 2394 | Ankra2 | NM_001271388.1 | chr13:98263075-98273918 | 2491 | Ap1m2 | NM_001110300.1 | chr9:21295456-21312333 |
| 2395 | Ankrd1 | NM_013468.3 | chr19:36111964-36119844 | 2492 | Ap1s1 | NM_007457.2 | chr5:137034993-137046060 |
| 2396 | Ankrd10 | NM_001167967.2 | chr8:11613840-11635757 | 2493 | Ap1s2 | NM_001290378.1 | chrX:163909016-163933666 |
| 2397 | Ankrd11 | NM_001081379.2 | chr8:122883821-123042284 | 2494 | Ap1s3 | NM_183027.2 | chr1:79606875-79671972 |
| 2398 | Ankrd12 | NM_001025572.1 | chr17:65967500-66077046 | 2495 | Ap2a1 | NM_001077264.1 | chr7:44900372-44929490 |
| 2399 | Ankrd13a | NM_026718.2 | chr5:114775139-114805820 | 2496 | Ap2a2 | NM_007459.3 | chr7:141562179-141633011 |
| 2400 | Ankrd13b | NM_172945.2 | chr11:77470486-77489678 | 2497 | Ap2b1 | NM_001035854.2 | chr11:83302696-83405033 |
| 2401 | Ankrd13c | NM_001013806.1 | chr3:157947465-158006837 | 2498 | Ap2m1 | NM_009679.3 | chr16:20535479-20544909 |
| 2402 | Ankrd13d | NM_026720.2 | chr19:4270179-4283137 | 2499 | Ap2s1 | NM_198613.2 | chr7:16738443-16749290 |
| 2403 | Ankrd16 | NM_177268.4 | chr2:11777752-11790323 | 2500 | Ap3b1 | NM_009680.3 | chr13:94358959-94566316 |
| 2404 | Ankrd17 | NM_030886.2 | chr5:90227165-90366185 | 2501 | Ap3b2 | NM_021492.3 | chr7:81460398-81493925 |
| 2405 | Ankrd2 | NM_020033.1 | chr19:42036037-42045110 | 2502 | Ap3d1 | NM_007460.1 | chr10:80706977-80742211 |
| 2406 | Ankrd22 | NM_024204.6 | chr19:34122548-34166041 | 2503 | Ap3m1 | NM_018829.4 | chr14:21033741-21052442 |
| 2407 | Ankrd23 | NM_153502.4 | chr1:36530192-36535739 | 2504 | Ap3m2 | NM_001122820.1 | chr8:22787353-22805654 |
| 2408 | Ankrd24 | NM_027480.3 | chr10:81628539-81647612 | 2505 | Ap3s1 | NM_009681.5 | chr18:46741875-46790826 |
| 2409 | Ankrd26 | NM_001081112.1 | chr6:118502563-118562256 | 2506 | Ap3s2 | NM_009682.3 | chr7:79875324-79920640 |
| 2410 | Ankrd27 | NM_145633.3 | chr7:35586246-35639237 | 2507 | Ap4b1 | NM_001163552.1 | chr3:103809516-103822025 |
| 2411 | Ankrd28 | NM_001024604.2 | chr14:31700014-31830415 | 2508 | Ap4e1 | NM_175550.3 | chr2:127008710-127069814 |
| 2412 | Ankrd29 | NM_001190371.1 | chr18:12252356-12305720 | 2509 | Ap4m1 | NM_021392.4 | chr5:138172020-138178685 |
| 2413 | Ankrd32 | NM_134071.3 | chr13:77043087-77135468 | 2510 | Ap4s1 | NM_021710.3 | chr12:51690965-51738939 |
| 2414 | Ankrd33 | NM_144790.1 | chr15:101115754-101120024 | 2511 | Ap5b1 | NM_001033448.2 | chr19:5568073-5571261 |
| 2415 | Ankrd33b | NM_001164441.1 | chr15:31291478-31367759 | 2512 | Ap5m1 | NM_144535.4 | chr14:49066494-49087723 |
| 2416 | Ankrd34a | NM_001024851.3 | chr3:96596635-96599778 | 2513 | Ap5s1 | NM_001291031.1 | chr2:131210359-131213514 |
| 2417 | Ankrd34b | NM_175455.4 | chr13:92425968-92441658 | 2514 | Ap5z1 | NM_172725.2 | chr5:142463930-142478715 |
| 2418 | Ankrd34c | NM_207260.2 | chr9:89728248-89738475 | 2515 | Apaf1 | NM_001042558.1 | chr10:90989310-91082743 |
| 2419 | Ankrd35 | NM_001081139.1 | chr3:96670130-96691034 | 2516 | Apba1 | NM_177034.3 | chr19:23758875-23949597 |
| 2420 | Ankrd36 | NM_023816.2 | chr11:5569683-5689337 | 2517 | Apba2 | NM_001291166.1 | chr7:64502137-64753876 |
| 2421 | Ankrd37 | NM_001039562.1 | chr8:45996907-45999850 | 2518 | Apba3 | NM_018758.2 | chr10:81268171-81273247 |
| 2422 | Ankrd39 | NM_026241.4 | chr1:36538170-36547252 | 2519 | Apbb1 | NM_001253885.1 | chr7:105558464-105581491 |
| 2423 | Ankrd40 | NM_027799.2 | chr11:94328000-94341847 | 2520 | Apbb1ip | NM_019456.2 | chr2:22774326-22875653 |
| 2424 | Ankrd42 | NM_028665.4 | chr7:92584182-92637142 | 2521 | Apbb2 | NM_001201413.1 | chr5:66298724-66618817 |

Fig.21 - 14

| | | | | | | | |
|---|---|---|---|---|---|---|---|
| 2522 | Apbb3 | NM_146085.1 | chr18:36671158-36679366 | 2619 | Arfgef2 | NM_001085495.2 | chr2:166805580-166898051 |
| 2523 | Apc | NM_007462.3 | chr18:34220983-34322190 | 2620 | Arfip1 | NM_001081093.2 | chr3:84496092-84582625 |
| 2524 | Apc2 | NM_011789.2 | chr10:80301819-80318256 | 2621 | Arfip2 | NM_029802.4 | chr7:105634200-105640416 |
| 2525 | Apcdd1 | NM_133237.3 | chr18:62922326-62953195 | 2622 | Arfrp1 | NM_001165991.1 | chr2:181357689-181365404 |
| 2526 | Apcs | NM_011318.2 | chr1:172893960-172895054 | 2623 | Arg1 | NM_007482.3 | chr10:24915206-24927470 |
| 2527 | Apeh | NM_146226.2 | chr9:108085413-108094480 | 2624 | Arg2 | NM_009705.3 | chr12:79130787-79156301 |
| 2528 | Apela | NM_001297554.1 | chr8:65028417-65037336 | 2625 | Arglu1 | NM_176849.3 | chr8:8666575-8690537 |
| 2529 | Apex1 | NM_009687.2 | chr14:50924948-50927188 | 2626 | Arhgap1 | NM_001145902.1 | chr2:91650117-91672320 |
| 2530 | Apex2 | NM_029943.2 | chrX:150571506-150588155 | 2627 | Arhgap10 | NM_030113.2 | chr8:77250365-77517907 |
| 2531 | Aph1a | NM_146104.3 | chr3:95893920-95898592 | 2628 | Arhgap11a | NM_181416.3 | chr2:113831491-113848661 |
| 2532 | Aph1b | NM_177583.4 | chr9:66775486-66795423 | 2629 | Arhgap12 | NM_001039692.1 | chr18:6024449-6136098 |
| 2533 | Aph1c | NM_026674.3 | chr9:66814993-66834706 | 2630 | Arhgap15 | NM_001301831.1 | chr2:43748783-44395953 |
| 2534 | Api5 | NM_007466.3 | chr2:94411726-94438186 | 2631 | Arhgap15os | NR_040622.1 | chr2:44059237-44065324 |
| 2535 | Apip | NM_019735.4 | chr2:103073674-103092649 | 2632 | Arhgap17 | NM_001122640.1 | chr7:123279148-123369915 |
| 2536 | Apitd1 | NM_027263.2 | chr4:149128348-149137600 | 2633 | Arhgap18 | NM_176837.2 | chr10:26772511-26918648 |
| 2537 | Aplf | NM_001170489.1 | chr6:87628428-87672168 | 2634 | Arhgap19 | NM_001163495.1 | chr19:41766587-41802084 |
| 2538 | Apln | NM_013912.3 | chrX:48025145-48034852 | 2635 | Arhgap20 | NM_175535.3 | chr9:51765351-51853059 |
| 2539 | Aplnr | NM_011784.3 | chr2:85136359-85139923 | 2636 | Arhgap20os | NR_033560.1 | chr9:51839492-51875885 |
| 2540 | Aplp1 | NM_007467.3 | chr7:30434979-30445582 | 2637 | Arhgap21 | NM_001081364.3 | chr2:20847918-20967721 |
| 2541 | Aplp2 | NM_001102455.1 | chr9:31149556-31211815 | 2638 | Arhgap22 | NM_153800.4 | chr14:33216822-33369936 |
| 2542 | Apmap | NM_027977.2 | chr2:150583080-150608523 | 2639 | Arhgap23 | NM_021493.2 | chr11:97450159-97502400 |
| 2543 | Apoa1 | NM_009692.4 | chr9:46228629-46230469 | 2640 | Arhgap24 | NM_001286468.1 | chr5:102768809-102897937 |
| 2544 | Apoa1bp | NM_144897.3 | chr3:88056522-88058495 | 2641 | Arhgap25 | NM_001037727.2 | chr6:87458544-87533259 |
| 2545 | Apoa2 | NM_013474.2 | chr1:171225053-171226379 | 2642 | Arhgap26 | NM_175164.4 | chr18:38993144-39376285 |
| 2546 | Apoa4 | NM_007468.2 | chr9:46240843-46243458 | 2643 | Arhgap27 | NM_001205236.1 | chr11:103331483-103363692 |
| 2547 | Apoa5 | NM_080434.3 | chr9:46268607-46271919 | 2644 | Arhgap27os3 | NR_045346.1 | chr11:103344752-103350126 |
| 2548 | Apob | NM_009693.2 | chr12:7977676-8016839 | 2645 | Arhgap28 | NM_172964.4 | chr17:67842707-68004108 |
| 2549 | Apobec1 | NM_001134391.1 | chr6:122577791-122602444 | 2646 | Arhgap29 | NM_172525.2 | chr3:121953325-122016153 |
| 2550 | Apobec2 | NM_009694.3 | chr17:48419230-48432728 | 2647 | Arhgap30 | NM_001005508.2 | chr1:171388959-171410239 |
| 2551 | Apobec3 | NM_001160415.1 | chr15:79892407-79908429 | 2648 | Arhgap31 | NM_020260.2 | chr16:38598342-38713035 |
| 2552 | Apobec4 | NM_001081197.1 | chr1:152750550-152757544 | 2649 | Arhgap32 | NM_001195632.1 | chr9:32116135-32265511 |
| 2553 | Apobr | NM_138310.1 | chr7:126585007-126589092 | 2650 | Arhgap33 | NM_001289670.1 | chr7:30522225-30535060 |
| 2554 | Apoc1 | NM_001110009.2 | chr7:19689479-19692659 | 2651 | Arhgap33os | NR_036630.1 | chr7:30515121-30522193 |
| 2555 | Apoc2 | NM_001277944.1 | chr7:19671578-19677941 | 2652 | Arhgap35 | NM_172739.4 | chr7:16494472-16614993 |
| 2556 | Apoc3 | NM_001289755.1 | chr9:46233049-46235299 | 2653 | Arhgap36 | NM_001081123.1 | chrX:49470449-49500250 |
| 2557 | Apoc4 | NM_007385.3 | chr7:19678083-19681460 | 2654 | Arhgap39 | NM_001168288.1 | chr15:76723984-76818170 |
| 2558 | Apod | NM_007470.4 | chr16:31296191-31314808 | 2655 | Arhgap4 | NM_001162423.1 | chrX:73894351-73911298 |
| 2559 | Apoe | NM_009696.4 | chr7:19696243-19699188 | 2656 | Arhgap40 | NM_001145015.1 | chr2:158512795-158550628 |
| 2560 | Apof | NM_133997.2 | chr10:128267996-128270151 | 2657 | Arhgap42 | NM_027823.1 | chr9:8994952-9239013 |
| 2561 | Apoh | NM_013475.4 | chr11:108395296-108414396 | 2658 | Arhgap44 | NM_001099288.1 | chr11:65002038-65162961 |
| 2562 | Apol10a | NM_177744.4 | chr15:77477046-77491069 | 2659 | Arhgap5 | NM_009706.2 | chr12:52516076-52567851 |
| 2563 | Apol10b | NM_177820.4 | chr15:77584157-77596125 | 2660 | Arhgap6 | NM_001287530.1 | chrX:169112877-169304440 |
| 2564 | Apol11a | NM_001177533.1 | chr15:77508270-77517319 | 2661 | Arhgap8 | NM_001164627.1 | chr15:84720051-84772207 |
| 2565 | Apol11b | NM_001143686.1 | chr15:77633950-77643286 | 2662 | Arhgap9 | NM_001285785.1 | chr10:127321963-127329943 |
| 2566 | Apol6 | NM_001163621.1 | chr15:77045074-77052351 | 2663 | Arhgdia | NM_133796.7 | chr11:120577234-120581620 |
| 2567 | Apol7a | NM_001164640.1 | chr15:77388216-77399110 | 2664 | Arhgdib | NM_007486.5 | chr6:136923660-136941756 |
| 2568 | Apol7b | NM_001024848.2 | chr15:77422208-77447460 | 2665 | Arhgdig | NM_008113.3 | chr17:26199182-26201350 |
| 2569 | Apol7c | NM_175391.4 | chr15:77524866-77533315 | 2666 | Arhgef1 | NM_001130150.1 | chr7:24902985-24926591 |
| 2570 | Apol7d | NR_040308.1 | chr1:71653334-71662843 | 2667 | Arhgef10 | NM_001037736.2 | chr8:14911662-15001085 |
| 2571 | Apol7e | NM_001134802.1 | chr15:77698888-77719288 | 2668 | Arhgef10l | NM_001112722.1 | chr4:140514484-140665905 |
| 2572 | Apol8 | NM_001081970.1 | chr15:77748612-77755229 | 2669 | Arhgef11 | NM_001003912.1 | chr3:87618750-87738033 |
| 2573 | Apol9a | NM_001162883.1 | chr15:77403788-77411080 | 2670 | Arhgef12 | NM_027144.2 | chr9:42963841-43105718 |
| 2574 | Apol9b | NM_001168660.1 | chr15:77729120-77736382 | 2671 | Arhgef15 | NM_177566.3 | chr11:68943153-68956858 |
| 2575 | Apold1 | NM_001109914.1 | chr6:134982000-134986836 | 2672 | Arhgef16 | NM_001112744.1 | chr4:154278469-154299895 |
| 2576 | Apom | NM_018816.1 | chr17:35128996-35131752 | 2673 | Arhgef17 | NM_001081116.1 | chr7:100869745-100932161 |
| 2577 | Apon | NM_133996.3 | chr10:128254130-128255901 | 2674 | Arhgef18 | NM_133962.3 | chr8:3393007-3456600 |
| 2578 | Apoo | NM_001199337.1 | chrX:94367153-94417092 | 2675 | Arhgef19 | NM_172520.2 | chr4:141242883-141257562 |
| 2579 | Apool | NM_026565.3 | chrX:112311351-112372755 | 2676 | Arhgef2 | NM_001198911.1 | chr3:88616206-88648052 |
| 2580 | Apoo-ps | NR_004438.1 | chrX:94367123-94398202 | 2677 | Arhgef25 | NM_001166413.1 | chr10:127182520-127189823 |
| 2581 | Apopt1 | NM_001163388.1 | chr12:111713268-111755055 | 2678 | Arhgef26 | NM_001081295.1 | chr3:62338776-62462221 |
| 2582 | App | NM_001198823.1 | chr16:84954435-85173707 | 2679 | Arhgef28 | NM_012026.2 | chr13:97898594-98206165 |
| 2583 | Appbp2 | NM_025825.3 | chr11:85191309-85235120 | 2680 | Arhgef3 | NM_001289686.1 | chr14:27143992-27403911 |
| 2584 | Appl1 | NM_145221.2 | chr14:26918987-26970551 | 2681 | Arhgef33 | NM_001145452.1 | chr17:80307406-80388689 |
| 2585 | Appl2 | NM_145220.2 | chr10:83600033-83648664 | 2682 | Arhgef37 | NM_177828.4 | chr18:61493793-61536536 |
| 2586 | Aprt | NM_009698.2 | chr8:122574636-122576907 | 2683 | Arhgef38 | NM_029953.1 | chr3:133159530-133234889 |
| 2587 | Aptx | NM_001025444.3 | chr4:40682077-40703194 | 2684 | Arhgef39 | NM_001013377.2 | chr4:43496143-43499660 |
| 2588 | Aqp1 | NM_007472.2 | chr6:55336298-55348555 | 2685 | Arhgef4 | NM_183019.2 | chr1:34801721-34812754 |
| 2589 | Aqp11 | NM_175105.3 | chr7:97726378-97738247 | 2686 | Arhgef40 | NM_001145921.1 | chr14:51984832-52006247 |
| 2590 | Aqp12 | NM_001159658.1 | chr1:93006333-93012269 | 2687 | Arhgef5 | NM_133674.1 | chr6:43265643-43289320 |
| 2591 | Aqp2 | NM_009699.3 | chr15:99579055-99584545 | 2688 | Arhgef6 | NM_152801.2 | chrX:57231484-57338729 |
| 2592 | Aqp3 | NM_016689.2 | chr4:41092723-41098183 | 2689 | Arhgef7 | NM_001113517.1 | chr8:11728104-11827152 |
| 2593 | Aqp4 | NM_009700.2 | chr18:15389393-15403684 | 2690 | Arhgef9 | NM_001033329.3 | chrX:95048934-95166539 |
| 2594 | Aqp5 | NM_009701.4 | chr15:99591027-99594829 | 2691 | Arid1a | NM_001080819.1 | chr4:133679007-133753611 |
| 2595 | Aqp6 | NM_175087.4 | chr15:99601399-99605477 | 2692 | Arid1b | NM_001085355.1 | chr17:4995073-5347656 |
| 2596 | Aqp7 | NM_007473.4 | chr4:41033073-41048136 | 2693 | Arid2 | NM_175251.4 | chr15:96287521-96405463 |
| 2597 | Aqp8 | NM_001109045.1 | chr7:123462293-123468003 | 2694 | Arid3a | NM_001288625.1 | chr10:79927340-79955018 |
| 2598 | Aqp9 | NM_001271843.1 | chr9:71110658-71162633 | 2695 | Arid3b | NM_019689.2 | chr9:57790504-57834234 |
| 2599 | Aqr | NM_001290788.1 | chr2:114105161-114175339 | 2696 | Arid3c | NM_001017362.2 | chr4:41723835-41731142 |
| 2600 | Ar | NM_013476.4 | chrX:98148756-98323218 | 2697 | Arid4a | NM_001081195.1 | chr12:71015966-71099351 |
| 2601 | Araf | NM_001159645.1 | chrX:20848542-20852905 | 2698 | Arid4b | NM_194262.2 | chr13:14063783-14199603 |
| 2602 | Arap1 | NM_001040111.1 | chr7:101348068-101412586 | 2699 | Arid5a | NM_001172205.1 | chr1:36307732-36324029 |
| 2603 | Arap2 | NM_178407.3 | chr5:62602445-62766177 | 2700 | Arid5b | NM_023598.2 | chr10:68095592-68278726 |
| 2604 | Arap3 | NM_001205336.1 | chr18:37972622-37998969 | 2701 | Arih1 | NM_019927.2 | chr9:59388553-59486374 |
| 2605 | Arc | NM_001276684.1 | chr15:74669080-74672570 | 2702 | Arih2 | NM_011790.4 | chr9:108602942-108649380 |
| 2606 | Arcn1 | NM_145985.4 | chr9:44742143-44767808 | 2703 | Arl1 | NM_025859.3 | chr10:88731413-88743202 |
| 2607 | Areg | NM_009704.4 | chr5:91139598-91148432 | 2704 | Arl10 | NM_019968.2 | chr13:54575012-54581128 |
| 2608 | Arel1 | NM_178065.4 | chr12:84918148-84970886 | 2705 | Arl11 | NM_177337.3 | chr14:61309752-61311936 |
| 2609 | Arf1 | NM_001130408.1 | chr11:59211411-59228162 | 2706 | Arl13a | NM_028947.1 | chrX:134187500-134208030 |
| 2610 | Arf2 | NM_007477.5 | chr11:103966870-103985350 | 2707 | Arl13b | NM_026577.3 | chr16:62793688-62847040 |
| 2611 | Arf3 | NM_007478.3 | chr15:98737625-98763118 | 2708 | Arl14 | NM_027843.1 | chr3:69222418-69223618 |
| 2612 | Arf4 | NM_007479.3 | chr14:26638196-26657258 | 2709 | Arl14ep | NM_001025102.1 | chr2:106962528-106974397 |
| 2613 | Arf5 | NM_007480.1 | chr6:28423639-28426499 | 2710 | Arl14epl | NM_001033446.2 | chr18:46921814-46934222 |
| 2614 | Arf6 | NM_007481.3 | chr12:69372149-69375980 | 2711 | Arl15 | NM_172595.4 | chr13:113794507-114157461 |
| 2615 | Arfgap1 | NM_001177706.1 | chr2:180967299-180982524 | 2712 | Arl16 | NM_197995.2 | chr11:120464325-120467600 |
| 2616 | Arfgap2 | NM_001166024.1 | chr2:91265114-91277371 | 2713 | Arl2 | NM_019722.3 | chr19:6134388-6141137 |
| 2617 | Arfgap3 | NM_025445.4 | chr15:83299739-83350247 | 2714 | Arl2bp | NM_024191.2 | chr8:94666599-94674457 |
| 2618 | Arfgef1 | NM_001102430.1 | chr1:10137506-10232670 | 2715 | Arl3 | NM_019718.2 | chr19:46531108-46573085 |

Fig.21 - 15

| | | | | | | | |
|---|---|---|---|---|---|---|---|
| 2716 | Arl4a | NM_001039515.1 | chr12:40033290-40037987 | 2813 | Ascc3 | NM_198007.2 | chr10:50592668-50851202 |
| 2717 | Arl4c | NM_177305.4 | chr1:88698225-88702191 | 2814 | Ascl1 | NM_008553.4 | chr10:87491040-87493660 |
| 2718 | Arl4d | NM_025404.3 | chr11:101665540-101667832 | 2815 | Ascl2 | NM_008554.3 | chr7:142966821-142969264 |
| 2719 | Arl5a | NM_182994.2 | chr2:52397950-52424874 | 2816 | Ascl3 | NM_020051.1 | chr7:109727464-109731732 |
| 2720 | Arl5b | NM_029466.4 | chr2:15055361-15079191 | 2817 | Ascl4 | NM_001163614.1 | chr10:85928490-85929647 |
| 2721 | Arl5c | NM_207231.1 | chr11:97989579-97996173 | 2818 | Ascl5 | NM_001270609.1 | chr1:136050803-136051370 |
| 2722 | Arl6 | NM_019665.3 | chr16:59613320-59639339 | 2819 | Asf1a | NM_025541.3 | chr10:53596960-53609215 |
| 2723 | Arl6ip1 | NM_019419.2 | chr7:118118889-118129625 | 2820 | Asf1b | NM_024184.2 | chr8:83955693-83970195 |
| 2724 | Arl6ip4 | NM_144509.2 | chr5:124116107-124118195 | 2821 | Asgr1 | NM_001291131.1 | chr11:70054123-70057895 |
| 2725 | Arl6ip5 | NM_022992.2 | chr6:97210791-97233315 | 2822 | Asgr2 | NM_007493.3 | chr11:70092643-70106186 |
| 2726 | Arl6ip6 | NM_022989.4 | chr2:53192083-53219221 | 2823 | Ash1l | NM_138679.5 | chr3:88965811-89079375 |
| 2727 | Arl8a | NM_026823.2 | chr1:135146833-135156268 | 2824 | Ash2l | NM_001080793.2 | chr8:25816000-25847694 |
| 2728 | Arl8b | NM_026011.3 | chr6:108783058-108823723 | 2825 | Asic1 | NM_001289791.1 | chr15:99691754-99701130 |
| 2729 | Arl9 | NM_206935.1 | chr5:77004054-77009478 | 2826 | Asic2 | NM_001034013.2 | chr11:80880164-81968396 |
| 2730 | Armc1 | NM_028840.2 | chr3:19132143-19163065 | 2827 | Asic3 | NM_183000.2 | chr5:24413450-24417834 |
| 2731 | Armc10 | NM_026034.4 | chr5:21645983-21662592 | 2828 | Asic4 | NM_183022.3 | chr1:75450509-75474340 |
| 2732 | Armc12 | NM_026290.3 | chr17:28530860-28538975 | 2829 | Asic5 | NM_021370.2 | chr3:81996921-82021233 |
| 2733 | Armc2 | NM_001034858.3 | chr10:41914989-42018382 | 2830 | Asl | NM_133768.4 | chr5:130011502-130024331 |
| 2734 | Armc3 | NM_001081083.2 | chr2:19199117-19310243 | 2831 | Asmt | NM_001199212.1 | chrX:170672644-170678054 |
| 2735 | Armc4 | NM_001081393.1 | chr18:7088232-7297901 | 2832 | Asna1 | NM_019652.1 | chr8:85017930-85025278 |
| 2736 | Armc5 | NM_146205.2 | chr7:128237356-128245100 | 2833 | Asns | NM_012055.3 | chr6:7675170-7693182 |
| 2737 | Armc6 | NM_133972.2 | chr8:70220192-70234422 | 2834 | Asnsd1 | NM_001290984.1 | chr1:53344616-53352752 |
| 2738 | Armc7 | NM_177778.4 | chr11:115475676-115490466 | 2835 | Aspa | NM_023113.5 | chr11:73304987-73324637 |
| 2739 | Armc8 | NM_001166138.1 | chr9:99518244-99568899 | 2836 | Aspdh | NM_026690.1 | chr7:44465434-44467751 |
| 2740 | Armc9 | NM_027456.2 | chr1:86154779-86252491 | 2837 | Aspg | NM_001081169.1 | chr12:112106682-112127573 |
| 2741 | Armcx1 | NM_001166377.1 | chrX:134717937-134721912 | 2838 | Asph | NM_001177849.1 | chr4:9449084-9669162 |
| 2742 | Armcx2 | NM_001166397.1 | chrX:134804141-134809221 | 2839 | Asphd1 | NM_001039645.1 | chr7:126946007-126949581 |
| 2743 | Armcx3 | NM_027870.3 | chrX:134756567-134761456 | 2840 | Asphd2 | NM_028386.1 | chr5:112385444-112392213 |
| 2744 | Armcx4 | NM_001202500.3 | chrX:134686518-134697772 | 2841 | Aspm | NM_009791.4 | chr1:139454772-139494088 |
| 2745 | Armcx5 | NM_001009575.5 | chrX:135742691-135747326 | 2842 | Aspn | NM_001172481.1 | chr13:49544442-49567562 |
| 2746 | Armcx6 | NM_001007578.2 | chrX:134748454-134751419 | 2843 | Asprv1 | NM_026414.2 | chr6:86628173-86629704 |
| 2747 | Arnt | NM_001037737.2 | chr3:95434389-95497239 | 2844 | Aspscr1 | NM_001164224.1 | chr11:120676660-120709446 |
| 2748 | Arnt2 | NM_007488.3 | chr7:84246274-84410038 | 2845 | Asrgl1 | NM_025610.3 | chr19:9111718-9135566 |
| 2749 | Arntl | NM_001243048.1 | chr7:113207464-113314126 | 2846 | Ass1 | NM_007494.3 | chr2:31470269-31520670 |
| 2750 | Arntl2 | NM_001289679.1 | chr6:146805529-146833529 | 2847 | Aste1 | NM_001164828.1 | chr9:105400787-105405756 |
| 2751 | Arpc1a | NM_019767.2 | chr5:145083868-145108756 | 2848 | Astl | NM_001291003.1 | chr2:127341635-127357656 |
| 2752 | Arpc1b | NM_023142.2 | chr5:145114255-145128186 | 2849 | Astn1 | NM_001205204.1 | chr1:158362303-158691786 |
| 2753 | Arpc2 | NM_029711.1 | chr1:74236549-74268213 | 2850 | Astn2 | NM_019514.3 | chr4:65380802-66404483 |
| 2754 | Arpc3 | NM_019824.3 | chr5:122391927-122406178 | 2851 | Asun | NM_138757.2 | chr6:146549631-146577835 |
| 2755 | Arpc4 | NM_001170485.1 | chr6:113378112-113390447 | 2852 | Asxl1 | NM_001039939.1 | chr2:153346138-153404007 |
| 2756 | Arpc5 | NM_026369.2 | chr1:152766541-152775580 | 2853 | Asxl2 | NM_001270988.1 | chr12:3426856-3506849 |
| 2757 | Arpc5l | NM_028809.1 | chr2:39008138-39015872 | 2854 | Asxl3 | NM_001167777.1 | chr18:22345088-22530227 |
| 2758 | Arpp19 | NM_001142655.1 | chr9:75037613-75060313 | 2855 | Asz1 | NM_023729.3 | chr6:18050963-18109061 |
| 2759 | Arpp21 | NM_001177615.1 | chr9:112180031-112187926 | 2856 | Atad1 | NM_026487.3 | chr19:32672562-32712298 |
| 2760 | Arr3 | NM_133205.3 | chrX:100605496-100618493 | 2857 | Atad2 | NM_027435.2 | chr15:58094046-58135082 |
| 2761 | Arrb1 | NM_177231.2 | chr7:99535485-99606771 | 2858 | Atad2b | NM_001099628.1 | chr12:4917352-5047410 |
| 2762 | Arrb2 | NM_001271358.1 | chr11:70432579-70440828 | 2859 | Atad3a | NM_179203.3 | chr4:155740639-155761098 |
| 2763 | Arrdc1 | NM_001162485.1 | chr2:24925351-24935281 | 2860 | Atad3aos | NR_027971.1 | chr4:155761191-155762260 |
| 2764 | Arrdc2 | NM_027560.1 | chr8:70835137-70839720 | 2861 | Atad5 | NM_001029856.2 | chr11:80089399-80135791 |
| 2765 | Arrdc3 | NM_001042591.1 | chr13:80883421-80896043 | 2862 | Atat1 | NM_001142744.1 | chr17:35897597-35910068 |
| 2766 | Arrdc4 | NM_001042592.2 | chr7:68736993-68749238 | 2863 | Atcay | NM_178662.3 | chr10:81204512-81230779 |
| 2767 | Arrdc5 | NM_029799.1 | chr17:56294110-56300286 | 2864 | Atcayos | NR_015477.1 | chr10:81194690-81208370 |
| 2768 | Arsa | NM_009713.4 | chr15:89472475-89477424 | 2865 | Ate1 | NM_001029895.3 | chr7:130391493-130520369 |
| 2769 | Arsb | NM_009712.3 | chr13:93771678-93943016 | 2866 | Atf1 | NM_007497.3 | chr15:100227858-100261248 |
| 2770 | Arsg | NM_001166177.1 | chr11:109543710-109573329 | 2867 | Atf2 | NM_001025093.2 | chr2:73816508-73892639 |
| 2771 | Arsi | NM_001038499.1 | chr18:60912239-60917768 | 2868 | Atf3 | NM_007498.3 | chr1:191170296-191183333 |
| 2772 | Arsj | NM_173451.3 | chr3:126363851-126440374 | 2869 | Atf4 | NM_001287180.1 | chr15:80255183-80257545 |
| 2773 | Arsk | NM_029847.4 | chr13:76060421-76098660 | 2870 | Atf5 | NM_030693.2 | chr7:44812255-44815658 |
| 2774 | Art1 | NM_009710.4 | chr7:102101742-102111148 | 2871 | Atf6 | NM_001081304.1 | chr1:170704456-170867771 |
| 2775 | Art2a-ps | NM_007490.1 | chr7:101552775-101555802 | 2872 | Atf6b | NM_017406.4 | chr17:34647145-34655074 |
| 2776 | Art2b | NM_019915.2 | chr7:101578859-101581161 | 2873 | Atf7 | NM_001310066.1 | chr15:102536643-102625421 |
| 2777 | Art3 | NM_181728.3 | chr5:92388133-92414627 | 2874 | Atf7ip | NM_019426.2 | chr6:136518850-136607379 |
| 2778 | Art4 | NM_026639.2 | chr6:136848450-136857600 | 2875 | Atf7ip2 | NM_029253.1 | chr16:10192905-10237287 |
| 2779 | Art5 | NM_001291354.1 | chr7:102096878-102100229 | 2876 | Atg10 | NM_025770.3 | chr13:90935348-91223987 |
| 2780 | Artn | NM_001284191.1 | chr4:117926159-117929763 | 2877 | Atg101 | NM_026566.2 | chr15:101284300-101290934 |
| 2781 | Arv1 | NM_026855.4 | chr8:124722138-124734123 | 2878 | Atg12 | NM_026217.3 | chr18:46732416-46741579 |
| 2782 | Arvcf | NM_001272028.1 | chr16:18380782-18407074 | 2879 | Atg13 | NM_145528.3 | chr2:91674611-91710592 |
| 2783 | Arx | NM_007492.4 | chrX:93286506-93298355 | 2880 | Atg14 | NM_172599.4 | chr14:47540892-47568434 |
| 2784 | Arxes1 | NM_029541.3 | chrX:136033366-136034946 | 2881 | Atg16l1 | NM_001205391.1 | chr1:87756010-87792428 |
| 2785 | Arxes2 | NM_029823.2 | chrX:135993819-135995359 | 2882 | Atg16l2 | NM_001111111.1 | chr7:101289615-101302088 |
| 2786 | As3mt | NM_020577.2 | chr19:46707442-46741095 | 2883 | Atg2a | NM_194348.3 | chr19:6241667-6262304 |
| 2787 | Asah1 | NM_019734.3 | chr8:41340642-41374697 | 2884 | Atg2b | NM_029654.4 | chr12:105613539-105685241 |
| 2788 | Asah2 | NM_018830.1 | chr19:31984650-32103140 | 2885 | Atg3 | NM_026402.3 | chr16:45158828-45188538 |
| 2789 | Asap1 | NM_001276461.1 | chr15:64086839-64382919 | 2886 | Atg4a | NM_174875.3 | chr3:103643952-103646068 |
| 2790 | Asap2 | NM_001004364.2 | chr12:21111755-21270171 | 2887 | Atg4b | NM_174874.3 | chr1:93755032-93789529 |
| 2791 | Asap3 | NM_001008232.2 | chr4:136206364-136246573 | 2888 | Atg4c | NM_001145967.1 | chr4:99194146-99259787 |
| 2792 | Asb1 | NM_001039126.2 | chr1:91540564-91559590 | 2889 | Atg4d | NM_153583.10 | chr9:21265284-21274837 |
| 2793 | Asb10 | NM_080444.5 | chr5:24532696-24540457 | 2890 | Atg5 | NM_053069.6 | chr10:44268338-44364299 |
| 2794 | Asb11 | NM_026853.2 | chrX:164436993-164459170 | 2891 | Atg7 | NM_001253717.1 | chr6:114643096-114860614 |
| 2795 | Asb12 | NM_080858.3 | chrX:95470198-95478129 | 2892 | Atg9a | NM_001003917.4 | chr1:75180860-75192010 |
| 2796 | Asb13 | NM_001267724.1 | chr13:3634031-3651779 | 2893 | Atg9b | NM_001002897.3 | chr5:24384180-24392143 |
| 2797 | Asb14 | NM_001170748.1 | chr14:26894603-26915257 | 2894 | Athl1 | NM_145387.4 | chr7:140941580-140947658 |
| 2798 | Asb15 | NM_080847.3 | chr6:24528143-24573164 | 2895 | Atic | NM_026195.3 | chr1:71557155-71579403 |
| 2799 | Asb16 | NM_148953.2 | chr11:102268822-102278061 | 2896 | Atl1 | NM_178628.5 | chr12:69893104-69964085 |
| 2800 | Asb17 | NM_025758.4 | chr3:153844246-153853615 | 2897 | Atl2 | NM_001286647.1 | chr17:79848389-79896123 |
| 2801 | Asb17os | NR_040373.1 | chr3:153850373-153852424 | 2898 | Atl3 | NM_001163505.1 | chr19:7494039-7538609 |
| 2802 | Asb18 | NM_139152.1 | chr1:89952677-90014577 | 2899 | Atm | NM_007499.2 | chr9:53437121-53536671 |
| 2803 | Asb2 | NM_023049.1 | chr12:103321141-103356001 | 2900 | Atmin | NM_177700.4 | chr8:116943392-116960445 |
| 2804 | Asb3 | NM_023906.3 | chr11:30954397-31102704 | 2901 | Atn1 | NM_007881.4 | chr6:124742543-124756487 |
| 2805 | Asb4 | NM_023048.5 | chr6:5383385-5433021 | 2902 | Atoh1 | NM_007500.4 | chr6:64729145-64731235 |
| 2806 | Asb5 | NM_029569.3 | chr8:54550330-54587836 | 2903 | Atoh7 | NM_016864.1 | chr10:63100155-63100605 |
| 2807 | Asb6 | NM_133346.2 | chr2:30823097-30828300 | 2904 | Atoh8 | NM_153778.3 | chr6:72206176-72235577 |
| 2808 | Asb7 | NM_080443.2 | chr7:66644566-66689561 | 2905 | Atox1 | NM_009720.2 | chr11:55446642-55461138 |
| 2809 | Asb8 | NM_001170710.1 | chr15:98134639-98145702 | 2906 | Atp10a | NM_009728.2 | chr7:58658201-58829426 |
| 2810 | Asb9 | NM_027027.2 | chrX:164497902-164539752 | 2907 | Atp10b | NM_176999.3 | chr11:43149876-43262286 |
| 2811 | Ascc1 | NM_001199187.1 | chr10:60003326-60099990 | 2908 | Atp10d | NM_153389.3 | chr5:72203342-72298758 |
| 2812 | Ascc2 | NM_029291.1 | chr11:4637792-4683386 | 2909 | Atp11a | NM_015804.3 | chr8:12757015-12868728 |

Fig.21 - 16

| | | | | | | | |
|---|---|---|---|---|---|---|---|
| 2910 | Atp11b | NM_029570.3 | chr3:35754137-35856276 | 3007 | Atxn7l1 | NM_001033436.3 | chr12:33302514-33368277 |
| 2911 | Atp11c | NM_001001798.2 | chrX:60223289-60403981 | 3008 | Atxn7l2 | NM_001289545.1 | chr3:108202227-108210527 |
| 2912 | Atp12a | NM_138652.2 | chr14:56365067-56388551 | 3009 | Atxn7l3 | NM_001098836.1 | chr11:102289299-102296629 |
| 2913 | Atp13a1 | NM_133224.2 | chr8:69791162-69807748 | 3010 | Atxn7l3b | NM_001033474.2 | chr10:112925427-112929026 |
| 2914 | Atp13a2 | NM_001164366.1 | chr4:140986872-141007701 | 3011 | AU015228 | NM_001033197.2 | chr2:130100393-130103375 |
| 2915 | Atp13a3 | NM_001128094.1 | chr16:30312423-30388530 | 3012 | AU015791 | NR_102381.1 | chr12:105513432-105515248 |
| 2916 | Atp13a4 | NM_001164612.1 | chr16:29396037-29541483 | 3013 | AU015836 | NR_028320.1 | chrX:93968655-93975470 |
| 2917 | Atp13a5 | NM_001284375.1 | chr16:29231913-29378732 | 3014 | AU016765 | NR_045899.1 | chr17:64514088-64555590 |
| 2918 | Atp1a1 | NM_144900.2 | chr3:101576218-101604707 | 3015 | AU018091 | NM_001004153.2 | chr7:3154658-3169203 |
| 2919 | Atp1a2 | NM_178405.3 | chr1:172271708-172298064 | 3016 | AU018829 | NM_001200055.1 | chr5:82183-95195342 |
| 2920 | Atp1a3 | NM_001290469.1 | chr7:24978168-25005937 | 3017 | AU019823 | NM_001134902.1 | chr9:50605239-50617464 |
| 2921 | Atp1a4 | NM_013734.1 | chr1:172223507-172258424 | 3018 | AU019990 | NR_033468.1 | chr2:132598195-132653064 |
| 2922 | Atp1b1 | NM_009721.6 | chr1:164437098-164458355 | 3019 | AU021063 | NR_045996.1 | chr15:101221246-101222155 |
| 2923 | Atp1b2 | NM_013415.5 | chr11:69599749-69605960 | 3020 | AU021092 | NM_001033220.3 | chr16:5211818-5222299 |
| 2924 | Atp1b3 | NM_007502.4 | chr9:96332674-96364299 | 3021 | AU022252 | NM_001012400.2 | chr4:119225137-119232724 |
| 2925 | Atp1b4 | NM_001290389.1 | chrX:38316121-38336784 | 3022 | AU022751 | NM_001033211.3 | chrX:6081217-6083420 |
| 2926 | Atp2a1 | NM_007504.2 | chr7:126445859-126463073 | 3023 | AU022754 | NR_040433.1 | chr15:85581141-85593708 |
| 2927 | Atp2a2 | NM_001110140.3 | chr5:122456323-122502225 | 3024 | AU022793 | NR_045719.1 | chr15:39962648-39967515 |
| 2928 | Atp2a3 | NM_001163336.1 | chr11:72961168-72993043 | 3025 | AU023762 | NR_040760.1 | chr9:113496745-113587152 |
| 2929 | Atp2b1 | NM_026482.2 | chr10:98915151-99026143 | 3026 | AU040320 | NM_001035525.1 | chr4:126753554-126869694 |
| 2930 | Atp2b2 | NM_001036684.2 | chr6:113745667-113891376 | 3027 | AU040972 | NR_045305.1 | chr11:79481722-79484031 |
| 2931 | Atp2b3 | NM_177236.4 | chrX:73503085-73573270 | 3028 | AU041133 | NM_001163064.1 | chr10:82128012-82153065 |
| 2932 | Atp2b4 | NM_001167949.2 | chr1:133702673-133753747 | 3029 | Auh | NM_016709.2 | chr13:52835109-52929677 |
| 2933 | Atp2c1 | NM_001253831.1 | chr9:105411361-105521257 | 3030 | Aup1 | NM_007517.4 | chr6:83054520-83057682 |
| 2934 | Atp2c2 | NM_026922.1 | chr8:119700008-119757718 | 3031 | Aurka | NM_001291185.1 | chr2:172356189-172370570 |
| 2935 | Atp4a | NM_001290627.1 | chr7:30712208-30725534 | 3032 | Aurkaip1 | NM_025338.4 | chr4:155831268-155833098 |
| 2936 | Atp4b | NM_009724.2 | chr8:13386208-13396778 | 3033 | Aurkb | NM_011496.2 | chr11:69045642-69051662 |
| 2937 | Atp5a1 | NM_007505.2 | chr18:77773767-77782868 | 3034 | Aurkc | NM_001080965.1 | chr7:6995384-7003091 |
| 2938 | Atp5b | NM_016774.3 | chr10:128083306-128090388 | 3035 | Auts2 | NM_177047.3 | chr5:131437681-132542343 |
| 2939 | Atp5c1 | NM_001112738.1 | chr2:10056030-10080510 | 3036 | AV039307 | NR_038349.1 | chr2:120850655-120866690 |
| 2940 | Atp5d | NM_025313.2 | chr10:80142314-80145818 | 3037 | AV051173 | NR_040442.1 | chr4:116684964-116686022 |
| 2941 | Atp5e | NM_025983.3 | chr2:174461074-174464101 | 3038 | AV320801 | NM_177918.1 | chrX:135167623-135504594 |
| 2942 | Atp5f1 | NM_009725.4 | chr3:105942677-105960248 | 3039 | Aven | NM_001165935.1 | chr2:112559681-112631253 |
| 2943 | Atp5g1 | NM_001161419.1 | chr11:96072792-96075631 | 3040 | Avil | NM_009635.3 | chr10:127000708-127020994 |
| 2944 | Atp5g2 | NM_026468.2 | chr15:102662867-102671047 | 3041 | Avl9 | NM_030235.1 | chr6:56714904-56761911 |
| 2945 | Atp5g3 | NM_175015.3 | chr2:73908446-73911326 | 3042 | Avp | NM_009732.2 | chr2:130580619-130582588 |
| 2946 | Atp5h | NM_027862.1 | chr11:115415696-115419919 | 3043 | Avpi1 | NM_027106.4 | chr19:42123274-42128993 |
| 2947 | Atp5j | NM_016755.3 | chr16:84827870-84835625 | 3044 | Avpr1a | NM_016847.2 | chr10:122448498-122453453 |
| 2948 | Atp5j2 | NM_020582.2 | chr5:145183705-145191592 | 3045 | Avpr1b | NM_011924.2 | chr1:131599113-131612000 |
| 2949 | Atp5k | NM_007507.2 | chr5:108433252-108434378 | 3046 | Avpr2 | NM_001276298.1 | chrX:73892101-73894428 |
| 2950 | Atp5l | NM_013795.5 | chr9:44913247-44920742 | 3047 | AW011738 | NR_030671.1 | chr4:156203283-156206028 |
| 2951 | Atp5o | NM_138597.2 | chr16:91925222-91931630 | 3048 | AW046200 | NR_040698.1 | chr8:57651752-57663430 |
| 2952 | Atp5s | NM_026536.1 | chr12:69724961-69744658 | 3049 | AW112010 | NR_102366.1 | chr19:11047611-11050566 |
| 2953 | Atp5sl | NM_001290487.1 | chr7:25619413-25625550 | 3050 | AW146154 | NM_001033530.3 | chr7:41478873-41499890 |
| 2954 | Atp6ap1 | NM_018794.4 | chrX:74297096-74304721 | 3051 | AW209491 | NM_001104646.1 | chr13:14630244-14638202 |
| 2955 | Atp6ap1l | NM_001145879.1 | chr13:90883448-90905355 | 3052 | AW495222 | NR_045086.1 | chr13:93706099-93719175 |
| 2956 | Atp6ap2 | NM_027439.4 | chrX:12587758-12617051 | 3053 | AW549542 | NR_045702.1 | chr5:119570128-119580358 |
| 2957 | Atp6v0a1 | NM_001243049.1 | chr11:101009451-101063717 | 3054 | AW549877 | NM_145930.2 | chr15:3982034-3995752 |
| 2958 | Atp6v0a2 | NM_011596.5 | chr5:124629051-124724455 | 3055 | AW551984 | NM_001199556.1 | chr9:39587395-39603652 |
| 2959 | Atp6v0a4 | NM_080467.3 | chr6:38048482-38124586 | 3056 | AW554918 | NM_001033532.3 | chr18:25169019-25467321 |
| 2960 | Atp6v0b | NM_033617.3 | chr4:117884329-117887329 | 3057 | AW822252 | NR_110489.1 | chrX:53696921-53716578 |
| 2961 | Atp6v0c | NM_009729.3 | chr17:24163865-24169429 | 3058 | Awat1 | NM_001081136.1 | chrX:100572246-100578206 |
| 2962 | Atp6v0c-ps2 | NR_037854.1 | chr17:24163864-24169395 | 3059 | Awat2 | NM_001290395.1 | chrX:100402221-100442717 |
| 2963 | Atp6v0d1 | NM_013477.3 | chr8:105524469-105566040 | 3060 | Axin1 | NM_001159598.1 | chr17:26138685-26195811 |
| 2964 | Atp6v0d2 | NM_175406.3 | chr4:19876837-19922566 | 3061 | Axin2 | NM_015732.4 | chr11:108920348-108950781 |
| 2965 | Atp6v0e | NM_025272.2 | chr17:26676395-26699646 | 3062 | Axl | NM_001190974.1 | chr7:25756499-25788733 |
| 2966 | Atp6v0e2 | NM_133764.3 | chr6:48537568-48541800 | 3063 | AY074887 | NM_145229.2 | chr9:54950256-54950954 |
| 2967 | Atp6v1a | NM_007508.5 | chr16:44085403-44139019 | 3064 | AY358078 | NM_194347.1 | chr14:51800045-51826359 |
| 2968 | Atp6v1b1 | NM_134157.2 | chr6:83743016-83758810 | 3065 | AY512915 | NR_033559.1 | chr6:95290573-95333993 |
| 2969 | Atp6v1b2 | NM_007509.3 | chr8:69088735-69113717 | 3066 | AY512931 | NR_033588.1 | chr8:45060701-45065418 |
| 2970 | Atp6v1c1 | NM_025494.3 | chr15:38661903-38692444 | 3067 | AY761184 | NM_001007582.3 | chr8:21702523-21703647 |
| 2971 | Atp6v1c2 | NM_001159632.1 | chr12:17284720-17324730 | 3068 | AY761185 | NM_001012640.2 | chr8:20943693-20944748 |
| 2972 | Atp6v1d | NM_023721.2 | chr12:78842981-78861638 | 3069 | Aym1 | NM_001012726.2 | chr5:113357293-113357821 |
| 2973 | Atp6v1e1 | NM_007510.2 | chr6:120795243-120822685 | 3070 | Azgp1 | NM_013478.2 | chr5:137981520-137990232 |
| 2974 | Atp6v1e2 | NM_029121.3 | chr17:86944108-86947887 | 3071 | Azi2 | NM_001048146.2 | chr9:118040521-118060484 |
| 2975 | Atp6v1f | NM_025381.2 | chr6:29467782-29470509 | 3072 | Azin1 | NM_001102458.1 | chr15:38487429-38519266 |
| 2976 | Atp6v1g1 | NM_024173.2 | chr4:63544764-63550701 | 3073 | B020004C17Rik | NM_001256060.1 | chr14:57015133-57018982 |
| 2977 | Atp6v1g2 | NM_023179.3 | chr17:35236595-35238768 | 3074 | B020004J07Rik | NM_001033790.3 | chr4:101834968-101844022 |
| 2978 | Atp6v1g3 | NM_177397.3 | chr1:138273737-138289462 | 3075 | B020014A21Rik | NR_045946.1 | chr10:3125090-3133193 |
| 2979 | Atp6v1h | NM_133826.5 | chr1:5083085-5162549 | 3076 | B020018J22Rik | NR_045950.1 | chr12:112428283-112435338 |
| 2980 | Atp7a | NM_001109757.2 | chrX:106027223-106128160 | 3077 | B020031M17Rik | NM_001033769.2 | chr13:119949356-119950806 |
| 2981 | Atp7b | NM_007511.2 | chr8:21994347-22060074 | 3078 | B130006D01Rik | NR_028263.1 | chr11:95723585-95726773 |
| 2982 | Atp8a1 | NM_001038999.2 | chr5:67618138-67847431 | 3079 | B130024G19Rik | NR_045850.1 | chr7:70365382-70411146 |
| 2983 | Atp8a2 | NM_015803.2 | chr14:59647740-60086834 | 3080 | B130034C11Rik | NR_040375.1 | chr16:87496072-87504038 |
| 2984 | Atp8b1 | NM_001001488.3 | chr18:64528978-64661000 | 3081 | B230112J18Rik | NR_110475.1 | chr5:116312359-116318274 |
| 2985 | Atp8b2 | NM_001081182.2 | chr3:89939480-89963332 | 3082 | B230118H07Rik | NM_026592.3 | chr2:101560780-101628986 |
| 2986 | Atp8b3 | NM_026094.3 | chr10:80519584-80539124 | 3083 | B230119M05Rik | NR_045454.1 | chrX:134684561-134686462 |
| 2987 | Atp8b4 | NM_001080944.3 | chr2:126320972-126491553 | 3084 | B230206H07Rik | NR_033532.1 | chr7:141359177-141365110 |
| 2988 | Atp8b5 | NM_177195.3 | chr4:43267158-43373831 | 3085 | B230208H11Rik | NR_038027.1 | chr10:12916645-12923127 |
| 2989 | Atp9a | NM_001289445.1 | chr2:168634437-168734339 | 3086 | B230209E15Rik | NR_045727.1 | chr7:61529409-61615327 |
| 2990 | Atp9b | NM_001201569.1 | chr18:80734140-80934058 | 3087 | B230214G05Rik | NR_045281.1 | chr15:88314873-88372651 |
| 2991 | Atpaf1 | NM_181040.4 | chr4:115784813-115812314 | 3088 | B230216G23Rik | NM_001242345.1 | chr6:142413430-142419740 |
| 2992 | Atpaf2 | NM_145427.2 | chr11:60400623-60417099 | 3089 | B230216N24Rik | NR_037993.1 | chr1:98031122-98047793 |
| 2993 | Atpif1 | NM_007512.4 | chr4:132530554-132535414 | 3090 | B230217C12Rik | NM_001080935.1 | chr11:97840779-97843043 |
| 2994 | Atr | NM_019864.1 | chr9:95857596-95951644 | 3091 | B230217O12Rik | NR_040316.1 | chr19:57323196-57360899 |
| 2995 | Atraid | NM_027855.4 | chr5:31048639-31054633 | 3092 | B230219D22Rik | NM_181278.2 | chr13:55693123-55703500 |
| 2996 | Atrip | NM_172774.3 | chr9:109059746-109074124 | 3093 | B230312C02Rik | NR_040745.1 | chr2:180370857-180385885 |
| 2997 | Atrn | NM_009730.2 | chr2:130906495-131030326 | 3094 | B230319C09Rik | NR_028382.1 | chr6:83441754-83448322 |
| 2998 | Atrnl1 | NM_181415.4 | chr19:57611033-58133340 | 3095 | B230323A14Rik | NR_040765.1 | chr9:69761145-69830199 |
| 2999 | Atrx | NM_009530.2 | chrX:105797614-105929372 | 3096 | B2m | NM_009735.3 | chr2:122147686-122153082 |
| 3000 | Atxn1 | NM_001199304.1 | chr13:45549755-45964991 | 3097 | B330016D10Rik | NR_030695.1 | chr4:141546161-141548313 |
| 3001 | Atxn10 | NM_016843.3 | chr15:85336380-85463836 | 3098 | B3galnt1 | NM_020026.4 | chr3:69573918-69598960 |
| 3002 | Atxn1l | NM_001080930.1 | chr8:109726450-109737739 | 3099 | B3galnt2 | NM_178640.2 | chr13:13954673-13999068 |
| 3003 | Atxn2 | NM_009125.2 | chr5:121711608-121814950 | 3100 | B3galt1 | NM_020283.4 | chr2:68104671-68122882 |
| 3004 | Atxn2l | NM_183020.1 | chr7:126491707-126503302 | 3101 | B3galt2 | NM_020025.4 | chr1:143640696-143649937 |
| 3005 | Atxn3 | NM_001167914.1 | chr12:101932923-101958243 | 3102 | B3galt4 | NM_019420.2 | chr17:33949911-33951488 |
| 3006 | Atxn7 | NM_139227.4 | chr14:14012490-14107301 | 3103 | B3galt5 | NM_001122993.1 | chr16:96235800-96319858 |

Fig.21 - 17

| | | | | | | | |
|---|---|---|---|---|---|---|---|
| 3104 | B3galt6 | NM_080445.4 | chr4:155989465-155992678 | 3201 | Bbs1 | NM_001033128.3 | chr19:4886881-4906627 |
| 3105 | B3gat1 | NM_029792.1 | chr9:26751561-26761338 | 3202 | Bbs10 | NM_027914.1 | chr10:111298678-111301736 |
| 3106 | B3gat2 | NM_172124.2 | chr1:23761925-23847858 | 3203 | Bbs12 | NM_001008502.2 | chr3:37312553-37321451 |
| 3107 | B3gat3 | NM_024256.2 | chr19:8920392-8927236 | 3204 | Bbs2 | NM_026116.2 | chr8:94067953-94098811 |
| 3108 | B3glct | NM_001081204.1 | chr5:149678256-149762599 | 3205 | Bbs4 | NM_175325.3 | chr9:59321965-59353508 |
| 3109 | B3gnt1 | NM_175383.2 | chr19:5038825-5041134 | 3206 | Bbs5 | NM_028284.2 | chr2:69647254-69667569 |
| 3110 | B3gnt2 | NM_001169114.1 | chr11:22834738-22860336 | 3207 | Bbs7 | NM_027810.3 | chr3:36573142-36613389 |
| 3111 | B3gnt3 | NM_028189.3 | chr8:71691719-71701800 | 3208 | Bbs9 | NM_178415.1 | chr9:22475714-22888280 |
| 3112 | B3gnt4 | NM_198611.2 | chr5:123510459-123511882 | 3209 | Bbx | NM_027444.3 | chr16:50191843-50432389 |
| 3113 | B3gnt5 | NM_001159407.1 | chr16:19760233-19772753 | 3210 | BC002163 | NR_002445.2 | chr4:42955579-123718161 |
| 3114 | B3gnt6 | NM_001081167.1 | chr7:98192414-98199475 | 3211 | BC003331 | NM_001077237.1 | chr1:150361310-150393055 |
| 3115 | B3gnt7 | NM_145222.2 | chr1:86303220-86307305 | 3212 | BC003965 | NM_183150.2 | chr17:25184560-25187662 |
| 3116 | B3gnt8 | NM_001036740.2 | chr7:25627623-25629490 | 3213 | BC004004 | NM_030561.3 | chr17:29268787-29302887 |
| 3117 | B3gnt9 | NM_001271915.1 | chr8:105252637-105255153 | 3214 | BC005537 | NM_024473.3 | chr13:24801656-24812899 |
| 3118 | B3gntl1 | NM_178664.5 | chr11:121616207-121673151 | 3215 | BC005561 | NM_001166581.1 | chr5:104508351-104522383 |
| 3119 | B430010I23Rik | NR_015457.1 | chr8:41017467-41024216 | 3216 | BC005624 | NM_144885.2 | chr2:30972832-30981941 |
| 3120 | B430212C06Rik | NR_033214.1 | chr18:67321208-67344103 | 3217 | BC005764 | NM_001170935.1 | chr10:79860474-79874634 |
| 3121 | B430306N03Rik | NM_177083.4 | chr17:48316161-48326511 | 3218 | BC006965 | NR_024085.1 | chr11:112663926-112711356 |
| 3122 | B430319G15Rik | NR_029474.1 | chr9:92538800-92542869 | 3219 | BC016579 | NM_145389.2 | chr16:45626847-45654118 |
| 3123 | B4galnt1 | NM_001244617.1 | chr10:127165155-127168523 | 3220 | BC017158 | NM_145590.2 | chr7:128271378-128298131 |
| 3124 | B4galnt2 | NM_008081.3 | chr11:95863558-95914871 | 3221 | BC017643 | NM_001252548.1 | chr11:121222588-121229309 |
| 3125 | B4galnt3 | NM_198884.1 | chr6:120203809-120294559 | 3222 | BC018242 | NM_001290299.1 | chr9:21937009-21948907 |
| 3126 | B4galnt4 | NM_177897.3 | chr7:141061273-141072119 | 3223 | BC018473 | NR_003364.1 | chr11:116752166-116757883 |
| 3127 | B4galt1 | NM_022305.4 | chr4:40804581-40854537 | 3224 | BC018507 | NM_144837.3 | chr13:70588688-70637634 |
| 3128 | B4galt2 | NM_001253381.1 | chr4:117872999-117883476 | 3225 | BC020402 | NR_033219.1 | chr10:7678970-7681158 |
| 3129 | B4galt3 | NM_020579.2 | chr1:171270327-171276895 | 3226 | BC021614 | NM_144869.2 | chr19:4057486-4059294 |
| 3130 | B4galt4 | NM_001285793.1 | chr16:38742258-38769054 | 3227 | BC021767 | NR_033629.1 | chr3:94660588-94667152 |
| 3131 | B4galt5 | NM_019835.2 | chr2:167298444-167349178 | 3228 | BC021785 | NM_001001489.3 | chr10:39968152-39986646 |
| 3132 | B4galt6 | NM_019737.2 | chr18:20684598-20746404 | 3229 | BC021891 | NM_145608.2 | chr8:125910449-125947439 |
| 3133 | B4galt7 | NM_146045.2 | chr13:55599895-55610443 | 3230 | BC022687 | NM_145450.3 | chr12:112808974-112816245 |
| 3134 | B630005N14Rik | NM_175312.4 | chr6:13625674-13677966 | 3231 | BC023829 | NM_001033328.2 | chrX:70460055-70477043 |
| 3135 | B630019K06Rik | NR_045448.1 | chrX:8892352-8894964 | 3232 | BC024139 | NM_001142968.1 | chr15:76119517-76126556 |
| 3136 | B830017H08Rik | NR_027959.1 | chr16:17833118-17835019 | 3233 | BC024386 | NR_015583.1 | chr7:140906063-140907967 |
| 3137 | B930003M22Rik | NR_037588.1 | chr17:10319918-10321141 | 3234 | BC024978 | NM_001243888.1 | chr7:27195780-27204320 |
| 3138 | B930018H19Rik | NR_040706.1 | chr8:34589419-34640316 | 3235 | BC025920 | NR_030677.1 | chr10:81606307-81609836 |
| 3139 | B930025P03Rik | NR_040705.1 | chr8:10870421-10882454 | 3236 | BC026585 | NM_001033284.1 | chr1:157458581-157488733 |
| 3140 | B930041F14Rik | NM_178699.4 | chr4:155694341-155696483 | 3237 | BC027072 | NM_146082.3 | chr17:71743554-71752885 |
| 3141 | B930059L03Rik | NR_033340.1 | chr12:110591373-110592679 | 3238 | BC027231 | NM_145972.4 | chr16:44724300-44737284 |
| 3142 | B930092H01Rik | NR_045334.1 | chr9:61259226-61293809 | 3239 | BC028528 | NM_153513.2 | chr3:95884972-95891930 |
| 3143 | B9d1 | NM_013717.2 | chr11:61505171-61512927 | 3240 | BC029214 | NM_153557.1 | chr2:25459487-25461094 |
| 3144 | B9d2 | NM_172148.1 | chr7:25681157-25686558 | 3241 | BC029722 | NR_015528.1 | chr2:155817731-155819203 |
| 3145 | Baalc | NM_080640.5 | chr15:38933908-38952916 | 3242 | BC030307 | NM_001003910.2 | chr10:86705810-86776843 |
| 3146 | Baat | NM_007519.2 | chr4:49489417-49506558 | 3243 | BC030336 | NM_001164580.1 | chr7:120677619-120734854 |
| 3147 | Babam1 | NM_026636.2 | chr8:71396854-71404772 | 3244 | BC030499 | NM_001287206.1 | chr11:78290840-78296805 |
| 3148 | Bace1 | NM_001145947.1 | chr9:45838528-45862484 | 3245 | BC030500 | NM_173411.2 | chr8:58911754-58914298 |
| 3149 | Bace2 | NM_019517.4 | chr16:97356727-97439012 | 3246 | BC030867 | NM_153544.3 | chr11:102248881-102265183 |
| 3150 | Bach1 | NM_007520.2 | chr16:87698953-87733346 | 3247 | BC030870 | NR_033217.1 | chr8:65085614-65129537 |
| 3151 | Bach2 | NM_001109661.1 | chr4:32417434-32586108 | 3248 | BC031181 | NM_001001181.3 | chr18:75005899-75009933 |
| 3152 | Bach2os | NR_026843.1 | chr4:32559679-32571662 | 3249 | BC031361 | NR_033221.1 | chr16:38085063-38089260 |
| 3153 | Bad | NM_001285453.1 | chr19:6942561-6951905 | 3250 | BC033916 | NR_040470.1 | chr17:33905134-33906675 |
| 3154 | Bag1 | NM_001171739.1 | chr4:40936397-40948294 | 3251 | BC035044 | NM_001254946.1 | chr6:128848043-128891124 |
| 3155 | Bag2 | NM_145392.2 | chr1:33745483-33757750 | 3252 | BC037032 | NR_028266.1 | chr15:4020110-4027406 |
| 3156 | Bag3 | NM_013863.5 | chr7:128523582-128546979 | 3253 | BC037034 | NM_153161.3 | chr5:138259657-138264052 |
| 3157 | Bag4 | NM_026121.3 | chr8:25764538-25785209 | 3254 | BC037704 | NR_045645.1 | chr19:43675177-43677170 |
| 3158 | Bag5 | NM_027404.2 | chr12:111709489-111713256 | 3255 | BC039771 | NR_033220.1 | chr2:145701194-145753672 |
| 3159 | Bag6 | NM_001252468.1 | chr17:35135177-35147322 | 3256 | BC039966 | NR_040670.1 | chr4:153948454-153950476 |
| 3160 | Bahcc1 | NM_198423.3 | chr11:120232946-120292297 | 3257 | BC048403 | NM_173022.2 | chr10:121739936-121752859 |
| 3161 | Bahd1 | NM_001045523.1 | chr2:118901614-118924524 | 3258 | BC048502 | NM_177631.3 | chr15:103438959-103448459 |
| 3162 | Bai1 | NM_174991.3 | chr15:74516195-74589464 | 3259 | BC048507 | NM_001001185.3 | chr13:67863325-67863923 |
| 3163 | Bai2 | NM_001199696.1 | chr4:129985077-130022633 | 3260 | BC048546 | NM_001001179.3 | chr6:128539821-128581606 |
| 3164 | Bai3 | NM_175642.4 | chr1:25067475-25829707 | 3261 | BC048562 | NM_001004192.1 | chr9:108436481-108446083 |
| 3165 | Baiap2 | NM_001037754.3 | chr11:119943091-120002618 | 3262 | BC048602 | NR_045280.1 | chr15:35307009-35328535 |
| 3166 | Baiap2l1 | NM_025833.3 | chr5:144264524-144358112 | 3263 | BC048609 | NM_001111317.1 | chr19:6080037-6080785 |
| 3167 | Baiap2l2 | NM_177580.3 | chr15:79258194-79285509 | 3264 | BC048644 | NM_001033485.2 | chr8:121907832-121918428 |
| 3168 | Baiap3 | NM_001163270.1 | chr17:25242658-25256364 | 3265 | BC048671 | NM_177738.2 | chr6:90301269-90305448 |
| 3169 | Bak1 | NM_007523.2 | chr17:27019811-27028626 | 3266 | BC048679 | NM_001193274.1 | chr7:81494273-81498330 |
| 3170 | Bambi | NM_026505.2 | chr18:3507956-3516404 | 3267 | BC049352 | NM_001198971.1 | chr9:45195765-45249985 |
| 3171 | Bambi-ps1 | NR_027919.1 | chr2:122466582-122467797 | 3268 | BC049635 | NM_177785.4 | chr4:42868002-42874203 |
| 3172 | Banf1 | NM_001038231.2 | chr19:5364632-5366363 | 3269 | BC049715 | NM_178776.3 | chr6:136828842-136840557 |
| 3173 | Banf2 | NM_001044750.1 | chr2:144033101-144073979 | 3270 | BC049730 | NM_199150.1 | chr7:24709258-24714535 |
| 3174 | Bank1 | NM_001033350.3 | chr3:136053363-136326066 | 3271 | BC049762 | NM_177567.3 | chr11:51253650-51262951 |
| 3175 | Banp | NM_001110100.2 | chr8:121950491-122029260 | 3272 | BC051019 | NM_001040700.2 | chr7:109712180-109723771 |
| 3176 | Bap1 | NM_027088.2 | chr14:31251488-31259929 | 3273 | BC051142 | NM_001001177.2 | chr17:34398819-34460734 |
| 3177 | Bard1 | NM_007525.3 | chr1:71027534-71102972 | 3274 | BC051226 | NR_045146.1 | chr17:33908181-33909178 |
| 3178 | Barhl1 | NM_001164186.1 | chr2:28907679-28916440 | 3275 | BC051537 | NR_046183.1 | chr17:34083842-34095309 |
| 3179 | Barhl2 | NM_001005477.1 | chr5:106452522-106458166 | 3276 | BC051628 | NM_199312.3 | chr2:181220015-181222851 |
| 3180 | Barx1 | NM_007526.4 | chr13:48663035-48666507 | 3277 | BC051665 | NM_199148.2 | chr13:60781886-60786364 |
| 3181 | Barx2 | NM_013800.2 | chr9:31846043-31913285 | 3278 | BC052040 | NM_001145898.1 | chr2:115581715-115778768 |
| 3182 | Basp1 | NM_027395.2 | chr15:25363276-25413764 | 3279 | BC052688 | NR_028430.1 | chr13:61771579-61787282 |
| 3183 | Batf | NM_016767.2 | chr12:85686719-85709087 | 3280 | BC053393 | NM_001025435.3 | chr11:46571512-46589232 |
| 3184 | Batf2 | NM_028967.1 | chr19:6164457-6172475 | 3281 | BC053749 | NM_183321.1 | chr7:30539133-30552272 |
| 3185 | Batf3 | NM_030060.2 | chr1:191098413-191108943 | 3282 | BC055111 | NM_183182.3 | chr4:106590908-106617238 |
| 3186 | Bax | NM_007527.3 | chr7:45461694-45466898 | 3283 | BC055324 | NM_201364.1 | chr1:163954007-163994781 |
| 3187 | Baz1a | NM_013815.2 | chr12:54892988-54986336 | 3284 | BC055402 | NR_037990.1 | chr1:57200644-57214996 |
| 3188 | Baz1b | NM_011714.2 | chr5:135187322-135246129 | 3285 | BC061194 | NM_001001334.2 | chr2:18699021-18749605 |
| 3189 | Baz2a | NM_054078.2 | chr10:128092782-128129303 | 3286 | BC061195 | NR_105038.1 | chrX:93156153-93183944 |
| 3190 | Baz2b | NM_001001182.3 | chr2:59899362-60125740 | 3287 | BC061212 | NM_198667.1 | chr5:94066454-94070545 |
| 3191 | BB014433 | NR_037972.1 | chr8:15041445-15046078 | 3288 | BC061237 | NM_198677.1 | chr14:44500121-44506345 |
| 3192 | BB019430 | NR_033565.1 | chr10:58696744-58704277 | 3289 | BC064078 | NR_015455.1 | chr6:128993019-129006649 |
| 3193 | BB031773 | NR_028391.1 | chr4:103004105-103011965 | 3290 | BC065397 | NR_033324.1 | chrX:136742956-136803361 |
| 3194 | BB123696 | NR_027893.1 | chr13:52729369-52757205 | 3291 | BC068157 | NM_207203.2 | chr8:4209542-4217312 |
| 3195 | BB283400 | NR_038124.1 | chr6:30176622-30178040 | 3292 | BC068281 | NM_001170858.1 | chr12:4843302-4856967 |
| 3196 | BB287469 | NM_001177573.1 | chr12:87618867-87820676 | 3293 | BC080695 | NM_001007579.3 | chr4:143567466-143573798 |
| 3197 | BB557941 | NR_040356.1 | chr2:57127476-57181754 | 3294 | BC089491 | NM_175033.3 | chr7:28284651-28291134 |
| 3198 | Bbc3 | NM_133234.2 | chr7:16309582-16318334 | 3295 | BC089597 | NM_145424.2 | chr10:127866475-127877319 |
| 3199 | Bbip1 | NM_001195338.1 | chr19:53929658-53944627 | 3296 | BC094916 | NM_001024721.2 | chr1:173521310-173535957 |
| 3200 | Bbox1 | NM_130452.1 | chr2:110265082-110305725 | 3297 | Bc1 | NR_038088.1 | chrX:79697304-163352564 |

Fig.21 - 18

| | | | | | | | |
|---|---|---|---|---|---|---|---|
| 3298 | BC100451 | NM_021440.2 | chr11:118332359-118342534 | 3395 | Bicd1 | NM_001112796.2 | chr6:149408983-149563326 |
| 3299 | BC100530 | NM_001082546.1 | chr16:36359381-36367570 | 3396 | Bicd2 | NM_001039179.2 | chr13:49341548-49387026 |
| 3300 | BC107364 | NM_001256180.1 | chr3:96433787-96452306 | 3397 | Bid | NM_007544.3 | chr6:120893118-120916820 |
| 3301 | BC117090 | NM_001001332.2 | chr16:36321664-36334332 | 3398 | Bik | NM_007546.2 | chr15:83526861-83544635 |
| 3302 | BC147527 | NM_001037925.2 | chr13:120300391-120308841 | 3399 | Bin1 | NM_001083334.1 | chr18:32377216-32435740 |
| 3303 | Bcam | NM_020486.2 | chr7:19756137-19770532 | 3400 | Bin2 | NM_001270537.1 | chr15:100641081-100669500 |
| 3304 | Bcan | NM_001109758.1 | chr3:87992513-88000356 | 3401 | Bin3 | NM_021328.3 | chr14:70100144-70138206 |
| 3305 | Bcap29 | NM_001164090.1 | chr12:31595353-31634658 | 3402 | Birc2 | NM_007465.3 | chr9:7818225-7837057 |
| 3306 | Bcap31 | NM_012060.5 | chrX:73686177-73716204 | 3403 | Birc3 | NM_007464.3 | chr9:7848700-7873170 |
| 3307 | Bcar1 | NM_001198839.1 | chr8:111710474-111732136 | 3404 | Birc5 | NM_001012273.1 | chr11:117849236-117855743 |
| 3308 | Bcar3 | NM_013867.2 | chr3:122419779-122530182 | 3405 | Birc6 | NM_007566.3 | chr17:74528294-74703356 |
| 3309 | Bcas1 | NM_001164369.1 | chr2:170346990-170427845 | 3406 | Birc7 | NM_001163247.1 | chr2:180929022-180934010 |
| 3310 | Bcas1os2 | NR_040610.1 | chr2:170356360-170380435 | 3407 | Bivm | NM_144558.4 | chr1:44119967-44144771 |
| 3311 | Bcas2 | NM_026602.3 | chr3:103171710-103179154 | 3408 | Blcap | NM_016916.3 | chr2:157556361-157566361 |
| 3312 | Bcas3 | NM_001166642.1 | chr11:85353163-85826058 | 3409 | Blk | NM_007549.2 | chr14:63372836-63417187 |
| 3313 | Bcas3os1 | NR_045875.1 | chr11:85702199-85719744 | 3410 | Blm | NM_001042527.2 | chr7:80454992-80535119 |
| 3314 | Bcas3os2 | NR_046194.1 | chr11:85766832-85775680 | 3411 | Blmh | NM_178645.4 | chr11:76945655-76987389 |
| 3315 | Bcat1 | NM_001024468.3 | chr6:144993834-145048812 | 3412 | Blnk | NM_008528.4 | chr19:40928926-40994535 |
| 3316 | Bcat2 | NM_001243052.1 | chr7:45571278-45589710 | 3413 | Bloc1s1 | NM_015740.3 | chr10:128919913-128923524 |
| 3317 | Bccip | NM_025392.2 | chr7:133709332-133721145 | 3414 | Bloc1s2 | NM_028607.1 | chr19:44139246-44146446 |
| 3318 | Bcdin3d | NM_029236.2 | chr15:99470083-99474730 | 3415 | Bloc1s3 | NM_177692.3 | chr7:19505803-19508331 |
| 3319 | Bche | NM_009738.3 | chr3:73635808-73708415 | 3416 | Bloc1s4 | NM_133724.3 | chr5:36747373-36748679 |
| 3320 | Bckdha | NM_007533.5 | chr7:25629851-25658761 | 3417 | Bloc1s5 | NM_139063.1 | chr13:38602705-38635109 |
| 3321 | Bckdhb | NM_199195.1 | chr9:83948780-84124240 | 3418 | Bloc1s6 | NM_019788.3 | chr2:122738504-122749487 |
| 3322 | Bckdk | NM_009739.3 | chr7:127904072-127909664 | 3419 | Blvra | NM_026678.4 | chr2:127070656-127097084 |
| 3323 | Bcl10 | NM_009740.2 | chr3:145924261-145934364 | 3420 | Blvrb | NM_001290525.1 | chr7:27452417-27465981 |
| 3324 | Bcl11a | NM_001159289.1 | chr11:24078055-24173558 | 3421 | Blzf1 | NM_001160208.1 | chr1:164289799-164307484 |
| 3325 | Bcl11b | NM_001079883.1 | chr12:107910402-108003414 | 3422 | Bmf | NM_138313.3 | chr2:118528756-118549678 |
| 3326 | Bcl2 | NM_009741.5 | chr1:106538175-106714290 | 3423 | Bmi1 | NM_007552.4 | chr2:18677017-18686629 |
| 3327 | Bcl2a1a | NM_009742.3 | chr9:88956919-88962416 | 3424 | Bmp1 | NM_009755.3 | chr14:70474554-70520260 |
| 3328 | Bcl2a1b | NM_007534.3 | chr9:89199272-89207838 | 3425 | Bmp10 | NM_009756.3 | chr6:87428993-87434512 |
| 3329 | Bcl2a1c | NM_007535.2 | chr9:114330134-114330578 | 3426 | Bmp15 | NM_009757.4 | chrX:6314105-6320723 |
| 3330 | Bcl2a1d | NM_007536.2 | chr9:88723287-88731850 | 3427 | Bmp2 | NM_007553.3 | chr2:133552158-133562896 |
| 3331 | Bcl2l1 | NM_001289716.1 | chr2:152780663-152830717 | 3428 | Bmp2k | NM_080708.1 | chr5:96997688-97091048 |
| 3332 | Bcl2l10 | NM_013479.2 | chr9:75347757-75351640 | 3429 | Bmp3 | NM_173404.5 | chr5:98854414-98880960 |
| 3333 | Bcl2l11 | NM_001284410.2 | chr2:128127574-128131498 | 3430 | Bmp4 | NM_007554.3 | chr14:46383519-46390599 |
| 3334 | Bcl2l12 | NM_029410.3 | chr7:44991221-44997579 | 3431 | Bmp5 | NM_007555.4 | chr9:75775364-75899017 |
| 3335 | Bcl2l13 | NM_153516.2 | chr6:120836229-120892842 | 3432 | Bmp6 | NM_007556.3 | chr13:38345715-38499728 |
| 3336 | Bcl2l14 | NM_025778.3 | chr6:134396328-134438724 | 3433 | Bmp7 | NM_007557.3 | chr2:172868011-172940321 |
| 3337 | Bcl2l15 | NM_001142959.1 | chr3:103832695-103838648 | 3434 | Bmp8a | NM_001256019.1 | chr4:123312649-123343252 |
| 3338 | Bcl2l2 | NM_007537.1 | chr14:54883424-54888234 | 3435 | Bmp8b | NM_007559.4 | chr4:123105164-123126091 |
| 3339 | Bcl3 | NM_033601.3 | chr7:19808461-19822755 | 3436 | Bmper | NM_028472.2 | chr9:23223075-23485215 |
| 3340 | Bcl6 | NM_009744.3 | chr16:23965051-23988612 | 3437 | Bmpr1a | NM_009758.4 | chr14:34411067-34502546 |
| 3341 | Bcl6b | NM_007528.3 | chr11:70224126-70229798 | 3438 | Bmpr1b | NM_001277216.1 | chr3:141837135-142169228 |
| 3342 | Bcl7a | NM_029850.3 | chr5:123344447-123374083 | 3439 | Bmpr2 | NM_007561.4 | chr1:59764278-59878081 |
| 3343 | Bcl7b | NM_009745.2 | chr5:135168371-135181852 | 3440 | Bms1 | NM_194339.1 | chr6:118383380-118419417 |
| 3344 | Bcl7c | NM_009746.2 | chr7:127704977-127708766 | 3441 | Bmx | NM_009759.4 | chrX:164192841-164258193 |
| 3345 | Bcl9 | NM_029933.4 | chr3:97203661-97227364 | 3442 | Bmyc | NM_023326.2 | chr2:25706878-25707719 |
| 3346 | Bcl9l | NM_030256.2 | chr9:44499135-44510412 | 3443 | Bnc1 | NM_007562.2 | chr7:81966661-81992299 |
| 3347 | Bclaf1 | NM_001025392.1 | chr10:20312468-20342501 | 3444 | Bnc2 | NM_172870.4 | chr4:84272541-84675086 |
| 3348 | Bcmo1 | NM_001163028.1 | chr8:117095864-117133720 | 3445 | Bnip1 | NM_172149.5 | chr17:26781078-26792521 |
| 3349 | Bco2 | NM_133217.3 | chr9:50533086-50555138 | 3446 | Bnip2 | NM_001008238.3 | chr9:69989465-70011659 |
| 3350 | Bcor | NM_001168321.1 | chrX:12036737-12160355 | 3447 | Bnip3 | NM_009760.4 | chr7:138890835-138909506 |
| 3351 | Bcorl1 | NM_178782.4 | chrX:48341357-48406728 | 3448 | Bnip3l | NM_009761.3 | chr14:66985239-67008877 |
| 3352 | Bcr | NM_001081412.2 | chr10:75060895-75184923 | 3449 | Bnipl | NM_001168356.1 | chr3:95241292-95251193 |
| 3353 | Bcs1l | NM_025784.5 | chr1:74588288-74592443 | 3450 | Boc | NM_172506.2 | chr16:44485044-44558870 |
| 3354 | Bdh1 | NM_001122683.1 | chr16:31428752-31458901 | 3451 | Bod1 | NM_001024919.1 | chr11:31665149-31671862 |
| 3355 | Bdh2 | NM_001172055.1 | chr3:135281220-135304425 | 3452 | Bod1l | NM_001081422.3 | chr5:41787539-41844315 |
| 3356 | Bdkrb1 | NM_007539.2 | chr12:105604090-105605428 | 3453 | Bok | NM_016778.3 | chr1:93685574-93695770 |
| 3357 | Bdkrb2 | NM_009747.2 | chr12:105563171-105593071 | 3454 | Bola1 | NM_026975.2 | chr3:96196587-96197586 |
| 3358 | Bdnf | NM_001048139.1 | chr2:109675896-109727043 | 3455 | Bola2 | NM_175103.3 | chr7:126695999-126696693 |
| 3359 | Bdp1 | NM_001081061.1 | chr13:100017993-100104070 | 3456 | Bola3 | NM_175277.4 | chr6:83349483-83358392 |
| 3360 | Bean1 | NM_001141922.1 | chr8:104170512-104219097 | 3457 | Boll | NM_001113367.1 | chr1:55300068-55362707 |
| 3361 | Becn1 | NM_019584.3 | chr11:101288266-101302267 | 3458 | Bop1 | NM_013481.1 | chr15:76452995-76477269 |
| 3362 | Becn2 | NM_001290692.1 | chr1:175920328-175922225 | 3459 | Bora | NM_175265.4 | chr14:99046376-99074107 |
| 3363 | Begain | NM_001163175.1 | chr12:109032181-109068217 | 3460 | Bpgm | NM_007563.4 | chr6:34476355-34505610 |
| 3364 | Bend3 | NM_199028.2 | chr10:43479139-43515417 | 3461 | Bphl | NM_026512.1 | chr13:34037640-34074074 |
| 3365 | Bend4 | NM_001164806.1 | chr5:67392146-67427799 | 3462 | Bpi | NM_177850.3 | chr2:158258240-158284531 |
| 3366 | Bend5 | NM_026279.3 | chr4:111415005-111460298 | 3463 | Bpifa1 | NM_011126.3 | chr2:154142879-154149217 |
| 3367 | Bend6 | NM_177235.3 | chr1:33852051-33907621 | 3464 | Bpifa2 | NM_008953.2 | chr2:154008275-154016073 |
| 3368 | Bend7 | NM_001190400.1 | chr2:4717830-4802146 | 3465 | Bpifa3 | NM_001291079.1 | chr2:154130335-154138359 |
| 3369 | Best1 | NM_011913.2 | chr19:9985171-10001633 | 3466 | Bpifa5 | NM_025990.4 | chr2:154162606-154168446 |
| 3370 | Best2 | NM_001130194.1 | chr8:85007202-85014408 | 3467 | Bpifa6 | NM_001080811.1 | chr2:153974944-154000495 |
| 3371 | Best3 | NM_001007583.1 | chr10:116986313-117025040 | 3468 | Bpifb1 | NM_001012392.1 | chr2:154190817-154220343 |
| 3372 | Bet1 | NM_009748.2 | chr6:4076903-4086927 | 3469 | Bpifb2 | NM_025631.3 | chr2:153875044-153895270 |
| 3373 | Bet1l | NM_018742.5 | chr7:140853383-140856383 | 3470 | Bpifb3 | NM_194357.1 | chr2:153918229-153932996 |
| 3374 | Bex1 | NM_009052.2 | chrX:136213971-136215513 | 3471 | Bpifb4 | NM_001034875.3 | chr2:153940861-153963852 |
| 3375 | Bex2 | NM_009749.2 | chrX:136066564-136068236 | 3472 | Bpifb5 | NM_144890.2 | chr2:154223741-154240902 |
| 3376 | Bex4 | NM_212457.2 | chrX:136139044-136140437 | 3473 | Bpifb6 | NM_199303.2 | chr2:153900387-153912793 |
| 3377 | Bex6 | NM_001033539.2 | chr16:32179799-32186944 | 3474 | Bpifb9a | NM_175167.3 | chr2:154257878-154271243 |
| 3378 | Bfar | NM_001177552.1 | chr16:13671857-13703612 | 3475 | Bpifb9b | NM_001025574.1 | chr2:154307243-154320642 |
| 3379 | Bfsp1 | NM_001291061.1 | chr2:143826527-143863173 | 3476 | Bpifc | NM_177772.4 | chr10:85959690-86011860 |
| 3380 | Bfsp2 | NM_001002896.2 | chr9:103424923-103480328 | 3477 | Bpnt1 | NM_011794.3 | chr1:185332158-185357769 |
| 3381 | Bglap | NM_001037939.2 | chr3:88383494-88384466 | 3478 | Bptf | NM_176850.2 | chr11:107033080-107131922 |
| 3382 | Bglap2 | NM_001032298.3 | chr3:88377735-88378701 | 3479 | Braf | NM_139294.5 | chr6:39603236-39725463 |
| 3383 | Bglap3 | NM_031368.5 | chr3:88368615-88369831 | 3480 | Brap | NM_001289543.1 | chr5:121660562-121687249 |
| 3384 | Bgn | NM_007542.5 | chrX:73483634-73495936 | 3481 | Brat1 | NM_001276287.1 | chr5:140705065-140719378 |
| 3385 | Bhlha15 | NM_010800.4 | chr5:144190285-144194441 | 3482 | Brca1 | NM_009764.3 | chr11:101488763-101551955 |
| 3386 | Bhlha9 | NM_177182.4 | chr11:76672469-76673676 | 3483 | Brca2 | NM_001081001.2 | chr5:150522620-150570146 |
| 3387 | Bhlhb9 | NM_001098222.1 | chrX:135885850-135891081 | 3484 | Brcc3 | NM_001166457.1 | chrX:75416627-75454001 |
| 3388 | Bhlhe22 | NM_021560.4 | chr3:18054324-18057514 | 3485 | Brd1 | NM_001033274.3 | chr15:88687034-88734219 |
| 3389 | Bhlhe23 | NM_080641.5 | chr2:180774380-180776900 | 3486 | Brd2 | NM_001204973.1 | chr17:34112018-34122607 |
| 3390 | Bhlhe40 | NM_011498.4 | chr6:108660628-108666925 | 3487 | Brd3 | NM_001113573.1 | chr2:27445580-27475673 |
| 3391 | Bhlhe41 | NM_001271768.1 | chr6:145858242-145865420 | 3488 | Brd4 | NM_001286630.1 | chr17:32196271-32284133 |
| 3392 | Bhmt | NM_016668.3 | chr13:93616890-93637758 | 3489 | Brd7 | NM_012047.2 | chr8:88332310-88362191 |
| 3393 | Bhmt2 | NM_022884.2 | chr13:93656096-93674302 | 3490 | Brd8 | NM_001289606.1 | chr18:34598614-34624863 |
| 3394 | Bicc1 | NM_031397.2 | chr10:70925095-71159634 | 3491 | Brd9 | NM_001024508.3 | chr13:73937810-73960889 |

Fig.21 - 19

| | | | | | | | |
|---|---|---|---|---|---|---|---|
| 3492 | Brdt | NM_001079873.1 | chr5:107331193-107350741 | 3589 | C130060C02Rik | NR_045355.1 | chr19:15985074-16010912 |
| 3493 | Bre | NM_144541.1 | chr5:31698049-32084739 | 3590 | C130060K24Rik | NM_175524.4 | chr6:65381293-65458150 |
| 3494 | Brf1 | NM_028193.3 | chr12:112959861-113000621 | 3591 | C130071C03Rik | NR_015561.2 | chr13:83728105-83735669 |
| 3495 | Brf2 | NM_025686.2 | chr8:27123831-27128632 | 3592 | C130074G19Rik | NM_178692.3 | chr1:184871925-184883036 |
| 3496 | Bri3 | NM_001163709.1 | chr5:144244436-144264573 | 3593 | C130079G13Rik | NM_177661.3 | chr3:59925213-59937949 |
| 3497 | Bri3bp | NM_029752.2 | chr5:125441567-125460885 | 3594 | C130080G10Rik | NR_028422.1 | chr2:114054395-114061502 |
| 3498 | Bricd5 | NM_175682.3 | chr17:24473883-24475469 | 3595 | C130083M11Rik | NR_040717.1 | chr5:52199983-52216433 |
| 3499 | Brinp1 | NM_019967.2 | chr4:68761371-68954397 | 3596 | C1d | NM_020558.3 | chr11:17257617-17269176 |
| 3500 | Brinp2 | NM_207583.2 | chr1:158245268-158356260 | 3597 | C1galt1 | NM_052993.3 | chr6:7845223-7872042 |
| 3501 | Brinp3 | NM_001145807.1 | chr1:146497686-146902472 | 3598 | C1galt1c1 | NM_021550.3 | chrX:38630782-38635143 |
| 3502 | Brip1 | NM_178309.2 | chr11:86058135-86201193 | 3599 | C1qa | NM_007572.2 | chr4:136895915-136898844 |
| 3503 | Brix1 | NM_026396.3 | chr15:10474778-10485937 | 3600 | C1qb | NM_009777.2 | chr4:136880144-136886177 |
| 3504 | Brk1 | NM_133937.1 | chr6:113604771-113616951 | 3601 | C1qbp | NM_007573.2 | chr11:70977845-70983026 |
| 3505 | Brms1 | NM_134155.1 | chr19:5041403-5049917 | 3602 | C1qc | NM_007574.2 | chr4:136889801-136892914 |
| 3506 | Brms1l | NM_001037756.2 | chr12:55836365-55869735 | 3603 | C1ql1 | NM_011795.2 | chr11:102939263-102946461 |
| 3507 | Brox | NM_027861.2 | chr1:183276341-183297008 | 3604 | C1ql2 | NM_207233.1 | chr1:120340581-120343174 |
| 3508 | Brpf1 | NM_001282126.1 | chr6:113307136-113324862 | 3605 | C1ql3 | NM_153155.2 | chr2:13001886-13010864 |
| 3509 | Brpf3 | NM_001081315.1 | chr17:28801125-28838789 | 3606 | C1ql4 | NM_001024702.1 | chr15:99084753-99087728 |
| 3510 | Brs3 | NM_009766.3 | chrX:57043073-57048758 | 3607 | C1qtnf1 | NM_001204129.1 | chr11:118428456-118451782 |
| 3511 | Brsk1 | NM_001003920.3 | chr7:4690927-4715997 | 3608 | C1qtnf2 | NM_026979.5 | chr11:43474305-43491525 |
| 3512 | Brsk2 | NM_001009929.3 | chr7:141949750-142004243 | 3609 | C1qtnf3 | NM_001204134.1 | chr15:10952356-10980162 |
| 3513 | Brwd1 | NM_001103179.1 | chr16:96001547-96082428 | 3610 | C1qtnf4 | NM_026161.3 | chr2:90885785-90890526 |
| 3514 | Brwd3 | NM_001081477.1 | chrX:108742207-108834355 | 3611 | C1qtnf5 | NM_001040631.2 | chr9:44107244-44109187 |
| 3515 | Bscl2 | NM_001136064.3 | chr19:8837466-8848683 | 3612 | C1qtnf6 | NM_001204152.1 | chr15:78523345-78529651 |
| 3516 | Bsdc1 | NM_133889.2 | chr4:129461678-129488432 | 3613 | C1qtnf7 | NM_001135172.1 | chr5:43515568-43616586 |
| 3517 | Bsg | NM_001077184.1 | chr10:79704357-79711979 | 3614 | C1qtnf9 | NM_183175.4 | chr14:60768133-60780869 |
| 3518 | Bsn | NM_007567.2 | chr9:108096021-108190383 | 3615 | C1ra | NM_023143.3 | chr6:124512620-124523440 |
| 3519 | Bsnd | NM_080458.2 | chr4:106483457-106492243 | 3616 | C1rb | NM_001113356.1 | chr6:124570429-124581044 |
| 3520 | Bsph1 | NM_001033418.4 | chr7:13450840-13473450 | 3617 | C1rl | NM_181344.5 | chr6:124493112-124510643 |
| 3521 | Bsph2 | NM_001080942.2 | chr7:13554865-13571067 | 3618 | C1s1 | NM_001097617.1 | chr6:124530343-124542359 |
| 3522 | Bspry | NM_138653.1 | chr4:62480066-62497298 | 3619 | C1s2 | NM_173864.2 | chr6:124624624-124636085 |
| 3523 | Bst1 | NM_009763.3 | chr5:43818892-43843468 | 3620 | C2 | NM_013484.2 | chr17:34862601-34882100 |
| 3524 | Bst2 | NM_198095.2 | chr8:71534261-71537437 | 3621 | C230004F18Rik | NR_030706.1 | chrX:61116377-61139460 |
| 3525 | Bsx | NM_178245.3 | chr9:40874126-40877972 | 3622 | C230024C17Rik | NR_046171.1 | chr1:153721239-153737829 |
| 3526 | Btaf1 | NM_001080706.1 | chr19:36926078-37014057 | 3623 | C230029M16 | NR_110482.1 | chr10:118920380-118927143 |
| 3527 | Btbd1 | NM_146193.2 | chr7:81792073-81829431 | 3624 | C230035I16Rik | NR_015492.1 | chr13:23427975-23431017 |
| 3528 | Btbd10 | NM_133700.2 | chr7:113315643-113369339 | 3625 | C230037L18Rik | NR_077233.1 | chr15:89476251-89484850 |
| 3529 | Btbd11 | NM_001017525.1 | chr10:85598410-85660292 | 3626 | C230052I12Rik | NM_178643.5 | chr7:35392151-35396767 |
| 3530 | Btbd16 | NM_001081038.2 | chr7:130774068-130825899 | 3627 | C230079O03Rik | NR_040459.1 | chr7:136332215-136349415 |
| 3531 | Btbd17 | NM_028055.4 | chr11:114790668-114795892 | 3628 | C230091D08Rik | NR_015479.1 | chr7:59307923-59324149 |
| 3532 | Btbd18 | NM_001145100.1 | chr2:84659078-84668781 | 3629 | C2cd2 | NM_174847.2 | chr16:97855208-97922633 |
| 3533 | Btbd19 | NR_024078.1 | chr4:117119217-117125725 | 3630 | C2cd2l | NM_027909.2 | chr9:44309236-44320282 |
| 3534 | Btbd2 | NM_145361.2 | chr10:80642616-80657071 | 3631 | C2cd3 | NM_001017985.2 | chr7:100372232-100470159 |
| 3535 | Btbd3 | NM_001025431.1 | chr2:138256583-138287422 | 3632 | C2cd4a | NM_001163143.1 | chr9:67830531-67832330 |
| 3536 | Btbd6 | NM_001145900.1 | chr12:112976481-112978940 | 3633 | C2cd4b | NM_001081314.2 | chr9:67759436-67760933 |
| 3537 | Btbd7 | NM_172806.2 | chr12:102784647-102878406 | 3634 | C2cd4c | NM_001168624.1 | chr10:79606853-79614025 |
| 3538 | Btbd8 | NM_001255991.1 | chr5:107437996-107491596 | 3635 | C2cd4d | NM_001136117.1 | chr3:94362443-94364567 |
| 3539 | Btbd9 | NM_027060.1 | chr17:30215523-30576287 | 3636 | C2cd5 | NM_001109688.2 | chr6:143010919-143100152 |
| 3540 | Btc | NM_007568.5 | chr5:91357260-91402994 | 3637 | C3 | NM_009778.3 | chr17:57203966-57228136 |
| 3541 | Btd | NM_025295.4 | chr14:31641056-31668197 | 3638 | C330006A16Rik | NM_001256521.1 | chr2:26136807-26140506 |
| 3542 | Btf3 | NM_001170540.1 | chr13:98309896-98317006 | 3639 | C330007P06Rik | NM_029951.1 | chrX:36848543-36864246 |
| 3543 | Btf3l4 | NM_027453.2 | chr4:108814294-108833584 | 3640 | C330011F03Rik | NR_046166.1 | chr17:51425208-51448269 |
| 3544 | Btg1 | NM_007569.2 | chr10:96617000-96622811 | 3641 | C330013E15Rik | NR_045701.1 | chr15:100614139-100615110 |
| 3545 | Btg2 | NM_007570.2 | chr1:134074864-134079155 | 3642 | C330013F16Rik | NR_045455.1 | chrX:139240225-139356770 |
| 3546 | Btg3 | NM_009770.3 | chr16:78359859-78376810 | 3643 | C330018D20Rik | NM_029909.1 | chr18:56955830-56975379 |
| 3547 | Btg4 | NM_019493.3 | chr9:51116000-51119700 | 3644 | C330021F23Rik | NM_001024728.2 | chr8:3567997-3584776 |
| 3548 | Btk | NM_013482.2 | chrX:134542340-134583140 | 3645 | C330022C24Rik | NR_045717.1 | chr7:140837220-140845689 |
| 3549 | Btla | NM_001037719.2 | chr16:45224336-45252895 | 3646 | C330024C12Rik | NR_046016.1 | chr17:87192215-87194942 |
| 3550 | Btn1a1 | NM_013483.3 | chr13:23456992-23465901 | 3647 | C330024D21Rik | NR_015582.2 | chr5:67463897-67470831 |
| 3551 | Btn2a2 | NM_001289614.1 | chr13:23477062-23488857 | 3648 | C330027C09Rik | NM_172616.2 | chr16:48994187-49019705 |
| 3552 | Btnl1 | NM_001111094.1 | chr17:34377131-34386028 | 3649 | C330046G13Rik | NR_040658.1 | chr10:84547416-84553338 |
| 3553 | Btnl10 | NM_138678.2 | chr11:58918056-58926965 | 3650 | C3ar1 | NM_009779.2 | chr6:122847139-122856157 |
| 3554 | Btnl2 | NM_079835.2 | chr17:34354821-34369492 | 3651 | C430002E04Rik | NR_040385.1 | chr3:41487680-41493199 |
| 3555 | Btnl4 | NM_030746.1 | chr17:34469041-34475937 | 3652 | C430002N11Rik | NR_102293.1 | chr9:96765561-96774397 |
| 3556 | Btnl5-ps | NR_004051.1 | chr17:34487405-34497429 | 3653 | C430049B03Rik | NR_038184.1 | chrX:53053111-53057190 |
| 3557 | Btnl6 | NM_030747.1 | chr17:34507934-34517352 | 3654 | C4a | NM_011413.2 | chr17:34809091-34823454 |
| 3558 | Btnl9 | NM_172793.2 | chr11:49168324-49187089 | 3655 | C4b | NM_009780.2 | chr17:34728380-34743897 |
| 3559 | Btrc | NM_001037758.2 | chr19:45363733-45533343 | 3656 | C4bp | NM_007576.3 | chr1:130635920-130661618 |
| 3560 | Bub1 | NM_001113179.1 | chr2:127800199-127831859 | 3657 | C4bp-ps1 | NR_028304.1 | chr1:130670201-130681571 |
| 3561 | Bub1b | NM_009773.3 | chr2:118598210-118641592 | 3658 | C530005A16Rik | NR_029450.1 | chr4:116589732-116597630 |
| 3562 | Bub3 | NM_009774.3 | chr7:131560390-131571898 | 3659 | C530008M17Rik | NM_001163793.1 | chr5:76840598-76873554 |
| 3563 | Bud13 | NM_146000.2 | chr9:46283011-46298783 | 3660 | C530044C16Rik | NR_045984.1 | chr6:50776114-50814894 |
| 3564 | Bud31 | NM_001008705.2 | chr5:145140375-145148078 | 3661 | C5ar1 | NM_001173550.1 | chr7:16246744-16259338 |
| 3565 | Bves | NM_024285.2 | chr10:45335761-45369708 | 3662 | C5ar2 | NM_001146005.1 | chr7:16234584-16242331 |
| 3566 | Bysl | NM_016859.3 | chr17:47599330-47611492 | 3663 | C6 | NM_016704.2 | chr15:4727209-4804045 |
| 3567 | Bzrap1 | NM_172449.2 | chr11:87760540-87785928 | 3664 | C630028M04Rik | NR_040668.1 | chr4:51968093-52051110 |
| 3568 | Bzw1 | NM_025824.3 | chr1:58393135-58406548 | 3665 | C630031E19Rik | NR_046080.1 | chr12:29675629-29686445 |
| 3569 | Bzw2 | NM_025840.3 | chr12:36091834-36156825 | 3666 | C630043F03Rik | NR_027923.1 | chr4:72201243-72203930 |
| 3570 | C030006K11Rik | NM_145472.2 | chr15:76721464-76723845 | 3667 | C7 | NM_001243837.1 | chr15:4988761-5063773 |
| 3571 | C030007H22Rik | NR_040482.1 | chr1:89360343-89406133 | 3668 | C730002L08Rik | NR_045778.1 | chr19:20534526-20552330 |
| 3572 | C030013G03Rik | NR_077216.1 | chr17:12466843-12479577 | 3669 | C730027H18Rik | NR_038040.1 | chr10:71168736-71182040 |
| 3573 | C030016D13Rik | NR_027987.1 | chr19:27430036-27432631 | 3670 | C730036E19Rik | NR_038011.1 | chr1:151138033-151144095 |
| 3574 | C030018K13Rik | NR_045411.1 | chr5:64477007-64479746 | 3671 | C77080 | NM_001033189.3 | chr4:129219577-129248443 |
| 3575 | C030023E24Rik | NR_033502.1 | chrX:61191292-61194164 | 3672 | C77370 | NM_001077354.2 | chrX:104077434-104201117 |
| 3576 | C030029H02Rik | NR_102277.1 | chr7:136268323-136318511 | 3673 | C78339 | NM_001033192.2 | chr13:46669521-46675773 |
| 3577 | C030034I22Rik | NR_026848.1 | chr17:69416446-69419192 | 3674 | C86187 | NR_015609.1 | chr7:46967746-46975911 |
| 3578 | C030034L19Rik | NR_015499.2 | chr3:9403077-9413903 | 3675 | C86695 | NM_001081662.1 | chr8:21958713-21964303 |
| 3579 | C030037D09Rik | NR_038058.1 | chr11:88718642-88728572 | 3676 | C87198 | NR_046002.1 | chr12:56591058-56594554 |
| 3580 | C030039L03Rik | NM_001112731.1 | chr7:27689339-27706482 | 3677 | C87414 | NM_001164284.1 | chr5:93635184-93671463 |
| 3581 | C030046E11Rik | NM_001081319.1 | chr19:29522281-29605921 | 3678 | C87436 | NM_001243741.1 | chr6:86438645-86470387 |
| 3582 | C130021I20Rik | NR_046275.1 | chr2:33641192-33645663 | 3679 | C87499 | NM_198663.3 | chr4:88627319-88634186 |
| 3583 | C130026I21Rik | NM_001037909.3 | chr1:85246343-85270566 | 3680 | C87977 | NM_001177542.1 | chr4:144206761-144213017 |
| 3584 | C130026L21Rik | NR_015546.2 | chr5:111581560-111587153 | 3681 | C8a | NM_001290645.1 | chr4:104815685-104876395 |
| 3585 | C130030K03Rik | NR_046212.1 | chr10:49093680-49095843 | 3682 | C8b | NM_133882.2 | chr4:104766316-104804548 |
| 3586 | C130036L24Rik | NR_015507.2 | chr1:86359573-86367964 | 3683 | C8g | NM_001271777.1 | chr2:25498649-25501719 |
| 3587 | C130046K22Rik | NR_102388.1 | chr11:103697723-103725573 | 3684 | C9 | NM_013485.1 | chr15:6445332-6498476 |
| 3588 | C130050O18Rik | NM_177000.3 | chr5:139406386-139416092 | 3685 | C920006O11Rik | NR_040401.1 | chr9:78175913-78178882 |

Fig.21 - 20

| | | | |
|---|---|---|---|
| 3686 | C920009B18Rik | NR_015465.2 | chr10:22306719-22312942 |
| 3687 | C920021L13Rik | NR_040446.1 | chr3:95871521-95889093 |
| 3688 | C920025E04Rik | NM_001271005.1 | chr17:36109029-36111676 |
| 3689 | Caap1 | NM_026368.2 | chr4:94500078-94556796 |
| 3690 | Cab39 | NM_133781.4 | chr1:85793446-85851577 |
| 3691 | Cab39l | NM_026908.3 | chr14:59440980-59548903 |
| 3692 | Cabin1 | NM_172549.3 | chr10:75646109-75764357 |
| 3693 | Cables1 | NM_001146287.1 | chr18:11839273-11945627 |
| 3694 | Cables2 | NM_145851.2 | chr2:180258538-180273465 |
| 3695 | Cabp1 | NM_013879.2 | chr5:115168690-115186121 |
| 3696 | Cabp2 | NM_001160252.1 | chr19:4083518-4087339 |
| 3697 | Cabp4 | NM_144532.2 | chr19:4135422-4139609 |
| 3698 | Cabp5 | NM_013877.3 | chr7:13398154-13408878 |
| 3699 | Cabp7 | NM_138948.3 | chr11:4738820-4746778 |
| 3700 | Cabs1 | NM_027631.2 | chr5:87979450-87981541 |
| 3701 | Cabyr | NM_001042418.1 | chr18:12741354-12755142 |
| 3702 | Cacfd1 | NM_001243239.1 | chr2:27009925-27016849 |
| 3703 | Cachd1 | NM_198037.1 | chr4:100776678-101003748 |
| 3704 | Cacna1a | NM_001252059.1 | chr8:84415363-84640249 |
| 3705 | Cacna1b | NM_001042528.2 | chr2:24603888-24763152 |
| 3706 | Cacna1c | NM_001159533.2 | chr6:118587239-119108495 |
| 3707 | Cacna1d | NM_001083616.2 | chr14:30039940-30353486 |
| 3708 | Cacna1e | NM_009782.3 | chr1:154392518-154725920 |
| 3709 | Cacna1f | NM_019582.2 | chrX:7607102-7635196 |
| 3710 | Cacna1g | NM_001112813.2 | chr11:94408390-94474198 |
| 3711 | Cacna1h | NM_001163691.1 | chr17:25374286-25433783 |
| 3712 | Cacna1i | NM_001044308.2 | chr15:80287237-80398292 |
| 3713 | Cacna1s | NM_001081023.1 | chr1:136052900-136119822 |
| 3714 | Cacna2d1 | NM_001110843.1 | chr5:15934690-16374511 |
| 3715 | Cacna2d2 | NM_001174047.1 | chr9:107399879-107529343 |
| 3716 | Cacna2d3 | NM_009785.1 | chr14:28904942-29721864 |
| 3717 | Cacna2d4 | NM_001033382.2 | chr6:119236525-119352407 |
| 3718 | Cacnb1 | NM_001159319.2 | chr11:98004924-98022627 |
| 3719 | Cacnb2 | NM_001252533.1 | chr2:14824091-14987908 |
| 3720 | Cacnb3 | NM_001044741.2 | chr15:98632327-98644536 |
| 3721 | Cacnb4 | NM_001037099.2 | chr2:52428319-52676609 |
| 3722 | Cacng1 | NM_007582.2 | chr11:107703217-107716476 |
| 3723 | Cacng2 | NM_007583.2 | chr15:77993622-78119280 |
| 3724 | Cacng3 | NM_019430.2 | chr7:122671743-122769394 |
| 3725 | Cacng4 | NM_019431.2 | chr11:107734779-107794464 |
| 3726 | Cacng5 | NM_001199301.1 | chr11:107874604-107915055 |
| 3727 | Cacng6 | NM_133183.1 | chr7:3424661-3434940 |
| 3728 | Cacng7 | NM_133189.3 | chr7:3336584-3366948 |
| 3729 | Cacng8 | NM_133190.1 | chr7:3394116-3415605 |
| 3730 | Cactin | NM_027381.2 | chr10:81321102-81326251 |
| 3731 | Cacul1 | NM_001172096.1 | chr19:60524695-60581023 |
| 3732 | Cacybp | NM_009786.2 | chr1:160202366-160212892 |
| 3733 | Cad | NM_001289522.1 | chr5:31054779-31078479 |
| 3734 | Cadm1 | NM_001025600.1 | chr9:47530351-47853385 |
| 3735 | Cadm2 | NM_001145977.1 | chr16:66655420-67620908 |
| 3736 | Cadm3 | NM_053199.3 | chr1:173334253-173367695 |
| 3737 | Cadm4 | NM_153112.3 | chr7:24482022-24504533 |
| 3738 | Cadps | NM_001042617.1 | chr14:12372562-12823079 |
| 3739 | Cadps2 | NM_001252105.1 | chr6:23262773-23839421 |
| 3740 | Cage1 | NM_027724.2 | chr13:38006051-38036937 |
| 3741 | Calb1 | NM_009788.4 | chr4:15881263-15906709 |
| 3742 | Calb2 | NM_007586.1 | chr8:110142537-110168206 |
| 3743 | Calca | NM_001033954.3 | chr7:114631477-114636357 |
| 3744 | Calcb | NM_054084.2 | chr7:114718642-114723365 |
| 3745 | Calcoco1 | NM_026192.3 | chr15:102706776-102722178 |
| 3746 | Calcoco2 | NM_001271018.1 | chr11:96098915-96111962 |
| 3747 | Calcr | NM_001042725.1 | chr6:3685719-3763623 |
| 3748 | Calcrl | NM_018782.2 | chr2:84330626-84425266 |
| 3749 | Cald1 | NM_145575.3 | chr6:34709443-34775469 |
| 3750 | Calhm1 | NM_001081271.1 | chr19:47141034-47144174 |
| 3751 | Calhm2 | NM_133746.5 | chr19:47132231-47138294 |
| 3752 | Calm1 | NM_009790.5 | chr12:100199434-100209824 |
| 3753 | Calm2 | NM_007589.5 | chr17:87433400-87446935 |
| 3754 | Calm3 | NM_007590.3 | chr7:16915378-16924032 |
| 3755 | Calm4 | NM_020036.4 | chr13:3837756-3838671 |
| 3756 | Calm5 | NM_001008706.2 | chr13:3854172-3854761 |
| 3757 | Calml3 | NM_027416.3 | chr13:3802892-3804318 |
| 3758 | Calml4 | NM_001102468.1 | chr9:62858103-62875917 |
| 3759 | Caln1 | NM_021371.2 | chr5:130448791-130840645 |
| 3760 | Calr | NM_007591.3 | chr8:84842087-84846931 |
| 3761 | Calr3 | NM_028500.3 | chr8:72424182-72443778 |
| 3762 | Calr4 | NM_001033226.4 | chr4:109235631-109254577 |
| 3763 | Calu | NM_001285412.1 | chr6:29348105-29376675 |
| 3764 | Caly | NM_001190385.1 | chr7:140069879-140082548 |
| 3765 | Camk1 | NM_133926.2 | chr6:113334123-113343922 |
| 3766 | Camk1d | NM_001290374.1 | chr2:5293456-5714762 |
| 3767 | Camk1g | NM_144817.2 | chr1:193346345-193370282 |
| 3768 | Camk2a | NM_001286809.1 | chr18:60963553-60988152 |
| 3769 | Camk2b | NM_001174053.1 | chr11:5969665-6065748 |
| 3770 | Camk2d | NM_001025438.2 | chr3:126596950-126846326 |
| 3771 | Camk2g | NM_001039138.2 | chr14:20734872-20794088 |
| 3772 | Camk2n1 | NM_025451.2 | chr4:138455147-138460126 |
| 3773 | Camk2n2 | NM_028420.2 | chr16:20619214-20621278 |
| 3774 | Camk4 | NM_009793.3 | chr18:32939040-33195767 |
| 3775 | Camkk1 | NM_018883.2 | chr11:73019007-73042065 |
| 3776 | Camkk2 | NM_001199676.1 | chr5:122731169-122779410 |
| 3777 | Camkmt | NM_028576.2 | chr17:85090699-85458580 |
| 3778 | Camkv | NM_145621.2 | chr9:107935919-107949691 |
| 3779 | Caml | NM_007596.2 | chr13:55623004-55632416 |
| 3780 | Camp | NM_009921.2 | chr9:109847376-109849456 |
| 3781 | Camsap1 | NM_001276359.1 | chr2:25926837-25983282 |
| 3782 | Camsap2 | NM_001081360.1 | chr1:136268122-136346104 |
| 3783 | Camsap3 | NM_001163749.1 | chr8:3587449-3609075 |
| 3784 | Camta1 | NM_001081557.3 | chr4:151059522-151861768 |
| 3785 | Camta2 | NM_001190376.1 | chr11:70669462-70688105 |
| 3786 | Cand1 | NM_027994.1 | chr10:119198811-119240055 |
| 3787 | Cand2 | NM_025958.2 | chr6:115774556-115805555 |
| 3788 | Cant1 | NM_001025617.2 | chr11:118406288-118419118 |
| 3789 | Canx | NM_001110499.1 | chr11:50293956-50325673 |
| 3790 | Cap1 | NM_007598.4 | chr4:122859047-122886057 |
| 3791 | Cap2 | NM_026056.4 | chr13:46501902-46650281 |
| 3792 | Capg | NM_001042534.3 | chr6:72549272-72562983 |
| 3793 | Capn1 | NM_001110504.1 | chr19:5988544-6015825 |
| 3794 | Capn10 | NM_011796.2 | chr1:92934407-92947948 |
| 3795 | Capn11 | NM_001013767.2 | chr17:45630203-45659309 |
| 3796 | Capn12 | NM_001110807.1 | chr7:28881655-28893585 |
| 3797 | Capn13 | NM_001033444.2 | chr17:73306463-73399296 |
| 3798 | Capn15 | NM_015830.1 | chr17:25959555-25965505 |
| 3799 | Capn2 | NM_009794.3 | chr1:182467258-182517483 |
| 3800 | Capn3 | NM_001109761.2 | chr2:120476908-120504919 |
| 3801 | Capn5 | NM_007602.4 | chr7:98121558-98178274 |
| 3802 | Capn6 | NM_007603.3 | chrX:143802236-143827412 |
| 3803 | Capn7 | NM_009796.2 | chr14:31336723-31371983 |
| 3804 | Capn8 | NM_001145806.1 | chr1:182565006-182606526 |
| 3805 | Capn9 | NM_023709.4 | chr8:124576110-124618731 |
| 3806 | Capns1 | NM_009795.3 | chr7:30186941-30195048 |
| 3807 | Capns2 | NM_027112.1 | chr8:92901394-92902409 |
| 3808 | Caprin1 | NM_001111289.1 | chr2:103762944-103797078 |
| 3809 | Caprin2 | NM_181541.4 | chr6:148842491-148896237 |
| 3810 | Caps2 | NM_178278.4 | chr10:112165675-112216555 |
| 3811 | Capsl | NM_029341.1 | chr15:9436027-9466035 |
| 3812 | Capza1 | NM_009797.2 | chr3:104822784-104864505 |
| 3813 | Capza2 | NM_007604.2 | chr6:17637097-17666536 |
| 3814 | Capza3 | NM_007605.4 | chr6:140041524-140042786 |
| 3815 | Capzb | NM_001037761.2 | chr4:139192898-139291820 |
| 3816 | Car1 | NM_001083957.1 | chr3:14766213-14778460 |
| 3817 | Car10 | NM_028296.3 | chr11:93099289-93601751 |
| 3818 | Car11 | NM_009800.4 | chr7:45699966-45704661 |
| 3819 | Car12 | NM_178396.5 | chr9:66713685-66766845 |
| 3820 | Car13 | NM_024495.5 | chr3:14641726-14663002 |
| 3821 | Car14 | NM_011797.2 | chr3:95897799-95904639 |
| 3822 | Car15 | NM_030558.2 | chr16:17835275-17838186 |
| 3823 | Car2 | NM_009801.4 | chr3:14886425-14900770 |
| 3824 | Car3 | NM_007606.3 | chr3:14863537-14872373 |
| 3825 | Car4 | NM_007607.2 | chr11:84957753-84966054 |
| 3826 | Car5a | NM_007608.2 | chr8:121916137-121944912 |
| 3827 | Car5b | NM_181315.4 | chrX:163976821-164028010 |
| 3828 | Car6 | NM_009802.2 | chr4:150187015-150201135 |
| 3829 | Car7 | NM_053070.3 | chr8:104540806-104550343 |
| 3830 | Car8 | NM_007592.3 | chr4:8141492-8239041 |
| 3831 | Car9 | NM_139305.2 | chr4:43507025-43513725 |
| 3832 | Card10 | NM_130859.2 | chr15:78775135-78803042 |
| 3833 | Card11 | NM_175362.2 | chr5:140872998-141000596 |
| 3834 | Card14 | NM_130886.3 | chr11:119314786-119345375 |
| 3835 | Card6 | NM_001163138.1 | chr15:5097438-5108533 |
| 3836 | Card9 | NM_001037747.1 | chr2:26352311-26359547 |
| 3837 | Carf | NM_001285463.1 | chr1:60098246-60153953 |
| 3838 | Carhsp1 | NM_025821.2 | chr16:8658586-8672153 |
| 3839 | Carkd | NM_001190357.1 | chr8:11497505-11513286 |
| 3840 | Carm1 | NM_021531.6 | chr9:21546893-21589465 |
| 3841 | Carns1 | NM_134148.2 | chr19:4164323-4175479 |
| 3842 | Cars | NM_001252593.1 | chr7:143557229-143600090 |
| 3843 | Cars2 | NM_024248.1 | chr8:11514016-11550771 |
| 3844 | Cartpt | NM_001081493.2 | chr13:99898482-99900683 |
| 3845 | Casc1 | NM_177222.4 | chr6:145174871-145210970 |
| 3846 | Casc3 | NM_138660.2 | chr11:98809807-98833807 |
| 3847 | Casc4 | NM_001205369.1 | chr2:121866969-121936207 |
| 3848 | Casc5 | NM_029617.2 | chr2:119047118-119104121 |
| 3849 | Casd1 | NM_145398.2 | chr6:4601065-4643381 |
| 3850 | Cask | NM_001284503.1 | chrX:13517080-13846556 |
| 3851 | Caskin1 | NM_027937.2 | chr17:24488782-24508907 |
| 3852 | Caskin2 | NM_080643.2 | chr11:115799351-115813592 |
| 3853 | Casp1 | NM_009807.2 | chr9:5298516-5307281 |
| 3854 | Casp12 | NM_009808.4 | chr9:5345475-5373034 |
| 3855 | Casp14 | NM_009809.5 | chr10:78711994-78718293 |
| 3856 | Casp2 | NM_007610.2 | chr6:42264984-42282508 |
| 3857 | Casp3 | NM_001284409.1 | chr8:46617290-46639698 |
| 3858 | Casp4 | NM_007609.3 | chr9:5308848-5336791 |
| 3859 | Casp6 | NM_009811.4 | chr3:129901414-129914112 |
| 3860 | Casp7 | NM_007611.2 | chr19:56397128-56442343 |
| 3861 | Casp8 | NM_001080126.1 | chr1:58802501-58847503 |
| 3862 | Casp8ap2 | NM_001122978.1 | chr4:32615472-32653265 |
| 3863 | Casp9 | NM_001277932.1 | chr4:141793611-141815978 |
| 3864 | Casq1 | NM_009813.2 | chr1:172209893-172219895 |
| 3865 | Casq2 | NM_009814.3 | chr3:102086454-102146512 |
| 3866 | Casr | NM_013803.2 | chr16:36493695-36562134 |
| 3867 | Cass4 | NM_001080820.2 | chr2:172393793-172433757 |
| 3868 | Cast | NM_009817.4 | chr13:74693293-74807960 |
| 3869 | Casz1 | NM_001159344.1 | chr4:148804391-148954892 |
| 3870 | Cat | NM_009804.2 | chr2:103453903-103485153 |
| 3871 | Catip | NM_001033345.2 | chr1:74362107-74369321 |
| 3872 | Catsper1 | NM_139301.2 | chr19:5335740-5344153 |
| 3873 | Catsper2 | NM_153075.3 | chr2:121394354-121413792 |
| 3874 | Catsper3 | NM_001252487.1 | chr13:55784578-55809000 |
| 3875 | Catsper4 | NM_001130030.1 | chr4:134211967-134227383 |
| 3876 | Catsperb | NM_173023.2 | chr12:101404672-101626009 |
| 3877 | Catsperd | NM_175350.3 | chr17:56628142-56664456 |
| 3878 | Catsperg1 | NM_001164658.1 | chr7:29181531-29214033 |
| 3879 | Catsperg2 | NM_029714.3 | chr7:29697218-29727015 |

Fig.21 - 21

| | | | | | | | |
|---|---|---|---|---|---|---|---|
| 3880 | Cav1 | NM_001243064.1 | chr6:17307639-17341328 | 3977 | Ccdc171 | NM_001081012.1 | chr4:83525544-83864670 |
| 3881 | Cav2 | NM_001277756.1 | chr6:17281184-17282684 | 3978 | Ccdc172 | NM_029372.2 | chr19:58512001-58553085 |
| 3882 | Cav3 | NM_007617.3 | chr6:112459504-112472872 | 3979 | Ccdc173 | NM_001077684.1 | chr2:69758056-69789486 |
| 3883 | Cbfa2t2 | NM_001285446.1 | chr2:154436483-154539356 | 3980 | Ccdc174 | NM_172730.2 | chr6:91878052-91899848 |
| 3884 | Cbfa2t3 | NM_001109873.1 | chr8:122625135-122678175 | 3981 | Ccdc175 | NM_028687.1 | chr12:72101291-72185029 |
| 3885 | Cbfb | NM_001161456.1 | chr8:105170673-105217988 | 3982 | Ccdc176 | NM_028377.3 | chr12:84409067-84433780 |
| 3886 | Cbl | NM_007619.2 | chr9:44149261-44234046 | 3983 | Ccdc177 | NM_001008423.2 | chr12:80755446-80760715 |
| 3887 | Cblb | NM_001033238.1 | chr16:52031548-52208046 | 3984 | Ccdc178 | NM_027616.3 | chr18:21810896-22171396 |
| 3888 | Cblc | NM_001161844.1 | chr7:19779717-19796809 | 3985 | Ccdc18 | NM_028481.1 | chr5:108132913-108232949 |
| 3889 | Cbll1 | NM_001253847.1 | chr12:31484828-31499616 | 3986 | Ccdc181 | NM_029115.3 | chr1:164275597-164287847 |
| 3890 | Cbln1 | NM_019626.3 | chr8:87468852-87472592 | 3987 | Ccdc183 | NM_029859.1 | chr2:25608628-25617678 |
| 3891 | Cbln2 | NM_172633.4 | chr18:86712064-86718283 | 3988 | Ccdc184 | NM_177716.3 | chr15:98167805-98170134 |
| 3892 | Cbln3 | NM_019820.3 | chr14:55878919-55884256 | 3989 | Ccdc185 | NM_001033547.2 | chr1:182747125-182749180 |
| 3893 | Cbln4 | NM_175631.3 | chr2:172036335-172043466 | 3990 | Ccdc19 | NM_027972.1 | chr1:172521129-172545870 |
| 3894 | Cbr1 | NM_007620.2 | chr16:93607836-93610349 | 3991 | Ccdc22 | NM_138603.3 | chrX:7593808-7605420 |
| 3895 | Cbr2 | NM_007621.2 | chr11:120729484-120732021 | 3992 | Ccdc23 | NM_001038998.2 | chr4:119195524-119201298 |
| 3896 | Cbr3 | NM_173047.3 | chr16:93683218-93690991 | 3993 | Ccdc24 | NM_001034876.1 | chr4:117869259-117872470 |
| 3897 | Cbr4 | NM_145595.2 | chr8:61487733-61503500 | 3994 | Ccdc25 | NM_145944.4 | chr14:65837301-65866604 |
| 3898 | Cbs | NM_001271353.1 | chr17:31612622-31637205 | 3995 | Ccdc27 | NM_001033455.2 | chr4:154026643-154042677 |
| 3899 | Cbwd1 | NM_146097.3 | chr19:24919915-24961616 | 3996 | Ccdc28a | NM_144820.3 | chr10:18213684-18234981 |
| 3900 | Cbx1 | NM_007622.3 | chr11:96789135-96808640 | 3997 | Ccdc28b | NM_025455.2 | chr4:129619273-129623908 |
| 3901 | Cbx2 | NM_007623.3 | chr11:119023020-119031275 | 3998 | Ccdc3 | NM_028804.1 | chr2:5137775-5230866 |
| 3902 | Cbx3 | NM_007624.3 | chr6:51470615-51483704 | 3999 | Ccdc30 | NM_001270435.1 | chr4:119322892-119415521 |
| 3903 | Cbx4 | NM_007625.2 | chr11:119077570-119086237 | 4000 | Ccdc32 | NM_199310.2 | chr2:119017778-119029393 |
| 3904 | Cbx5 | NM_001076789.1 | chr15:103191545-103239816 | 4001 | Ccdc33 | NM_001166282.1 | chr9:58028681-58118823 |
| 3905 | Cbx6 | NM_028763.3 | chr15:79823897-79834333 | 4002 | Ccdc34 | NM_026613.4 | chr2:110017816-110045325 |
| 3906 | Cbx7 | NM_144811.3 | chr15:79915806-79932646 | 4003 | Ccdc34os | NR_040508.1 | chr2:110031249-110050479 |
| 3907 | Cbx8 | NM_013926.1 | chr11:119038435-119040913 | 4004 | Ccdc36 | NM_001135198.1 | chr9:108403632-108428482 |
| 3908 | Cby1 | NM_028634.3 | chr15:79659226-79667660 | 4005 | Ccdc37 | NM_173775.3 | chr6:90403735-90428480 |
| 3909 | Cc2d1a | NM_145970.1 | chr8:84132827-84147753 | 4006 | Ccdc38 | NM_175488.6 | chr10:93540631-93584326 |
| 3910 | Cc2d1b | NM_177045.3 | chr4:108619955-108634122 | 4007 | Ccdc39 | NM_026222.2 | chr3:33812360-33844310 |
| 3911 | Cc2d2a | NM_172274.2 | chr5:43662378-43740970 | 4008 | Ccdc40 | NM_175430.4 | chr11:119228571-119265212 |
| 3912 | Ccar1 | NM_026201.3 | chr10:62743927-62792368 | 4009 | Ccdc42 | NM_177779.3 | chr11:68587036-68597951 |
| 3913 | Ccar2 | NM_146055.3 | chr14:70138168-70153791 | 4010 | Ccdc42b | NM_001195094.1 | chr5:120628334-120634235 |
| 3914 | Ccbe1 | NM_178793.4 | chr18:66056855-66291838 | 4011 | Ccdc43 | NM_025918.3 | chr11:102684687-102697725 |
| 3915 | Ccbl1 | NM_172404.2 | chr2:30185130-30205699 | 4012 | Ccdc47 | NM_026009.2 | chr11:106199355-106216367 |
| 3916 | Ccbl2 | NM_173763.4 | chr3:142701047-142744911 | 4013 | Ccdc50 | NM_001025615.3 | chr16:27388955-27452218 |
| 3917 | Ccdc101 | NM_029339.3 | chr7:126649308-126672779 | 4014 | Ccdc51 | NM_025689.4 | chr9:109082495-109093363 |
| 3918 | Ccdc102a | NM_001033533.3 | chr8:94902868-94918098 | 4015 | Ccdc53 | NM_001122960.1 | chr10:88201096-88246158 |
| 3919 | Ccdc103 | NM_028492.2 | chr11:102881243-102885215 | 4016 | Ccdc54 | NM_027046.3 | chr16:50589858-50591154 |
| 3920 | Ccdc104 | NM_025740.3 | chr11:29221535-29247272 | 4017 | Ccdc55 | NM_001012309.2 | chr11:77044291-77078437 |
| 3921 | Ccdc105 | NM_027630.1 | chr10:78746923-78753067 | 4018 | Ccdc57 | NM_027745.1 | chr11:120826541-120932872 |
| 3922 | Ccdc106 | NM_001290429.1 | chr7:5056151-5060784 | 4019 | Ccdc58 | NM_001159421.1 | chr16:36071659-36092118 |
| 3923 | Ccdc107 | NM_001037913.2 | chr4:43493364-43495921 | 4020 | Ccdc59 | NM_025602.3 | chr10:105841478-105847510 |
| 3924 | Ccdc108 | NM_001039495.1 | chr1:74902079-74935599 | 4021 | Ccdc6 | NM_001111121.1 | chr10:70097120-70193200 |
| 3925 | Ccdc109b | NM_025779.3 | chr3:129914959-129970206 | 4022 | Ccdc60 | NM_177759.3 | chr5:116125580-116288985 |
| 3926 | Ccdc11 | NM_028948.2 | chr18:74283099-74359984 | 4023 | Ccdc61 | NM_001033314.3 | chr7:18890883-18910404 |
| 3927 | Ccdc110 | NM_001033246.2 | chr8:45934648-45944145 | 4024 | Ccdc62 | NM_001134767.1 | chr5:123930688-123969895 |
| 3928 | Ccdc112 | NM_001160399.1 | chr18:46282150-46311928 | 4025 | Ccdc63 | NM_001289809.1 | chr5:122108051-122134893 |
| 3929 | Ccdc113 | NM_172914.2 | chr8:95534099-95558888 | 4026 | Ccdc64 | NM_001080808.1 | chr5:115649285-115731559 |
| 3930 | Ccdc114 | NM_001033243.2 | chr7:45928397-45948956 | 4027 | Ccdc64b | NM_153784.2 | chr17:23660522-23668619 |
| 3931 | Ccdc115 | NM_027159.2 | chr1:34436669-34439672 | 4028 | Ccdc65 | NM_153518.1 | chr15:98708226-98723333 |
| 3932 | Ccdc116 | NM_001306169.1 | chr16:1713906-.17147146 | 4029 | Ccdc66 | NM_177111.3 | chr14:27482404-27508460 |
| 3933 | Ccdc117 | NM_134033.2 | chr11:5528887-5542217 | 4030 | Ccdc67 | NM_181816.2 | chr9:15559863-15627933 |
| 3934 | Ccdc12 | NM_028312.3 | chr9:110656502-110711593 | 4031 | Ccdc68 | NM_201362.2 | chr18:69925558-69969484 |
| 3935 | Ccdc120 | NM_207202.2 | chrX:7731713-7741324 | 4032 | Ccdc69 | NM_177471.4 | chr11:55049737-55078131 |
| 3936 | Ccdc121 | NM_207280.3 | chr1:181509632-181511451 | 4033 | Ccdc7 | NM_001197041.1 | chr8:129045476-129065492 |
| 3937 | Ccdc122 | NM_175369.4 | chr14:77036771-77112263 | 4034 | Ccdc70 | NM_026459.3 | chr8:21970595-21974041 |
| 3938 | Ccdc124 | NM_026964.3 | chr8:70868226-70873490 | 4035 | Ccdc71 | NM_133744.4 | chr9:108460517-108465945 |
| 3939 | Ccdc125 | NM_001168386.2 | chr13:100669480-100697240 | 4036 | Ccdc71l | NM_001162903.1 | chr12:32378788-32382943 |
| 3940 | Ccdc126 | NM_175098.6 | chr6:49319273-49341586 | 4037 | Ccdc73 | NM_177600.4 | chr2:104886323-104999737 |
| 3941 | Ccdc127 | NM_001168658.1 | chr13:74350316-74365783 | 4038 | Ccdc74a | NM_001166164.1 | chr16:17646469-17650738 |
| 3942 | Ccdc129 | NM_001081665.1 | chr6:55837017-55978598 | 4039 | Ccdc77 | NM_026028.5 | chr6:120324321-120357469 |
| 3943 | Ccdc13 | NM_028384.1 | chr9:121797626-121839461 | 4040 | Ccdc78 | NM_001165929.1 | chr17:25786579-25790513 |
| 3944 | Ccdc130 | NM_026350.3 | chr8:84257766-84270402 | 4041 | Ccdc79 | NM_180958.3 | chr8:104446718-104509887 |
| 3945 | Ccdc132 | NM_001167750.1 | chr6:3498392-3603531 | 4042 | Ccdc8 | NM_001101535.1 | chr7:16994587-16996645 |
| 3946 | Ccdc134 | NM_172428.2 | chr15:82127921-82142202 | 4043 | Ccdc80 | NM_026439.2 | chr16:45094052-45127924 |
| 3947 | Ccdc135 | NM_001042715.3 | chr8:95055102-95078141 | 4044 | Ccdc81 | NM_001162979.1 | chr7:89866147-89903629 |
| 3948 | Ccdc136 | NM_001201378.1 | chr6:29398925-29426995 | 4045 | Ccdc82 | NM_025534.2 | chr9:13246978-13292353 |
| 3949 | Ccdc137 | NM_152807.3 | chr11:120458128-120464353 | 4046 | Ccdc83 | NM_029256.3 | chr7:90235760-90265432 |
| 3950 | Ccdc138 | NM_001162956.1 | chr10:58497936-58576244 | 4047 | Ccdc84 | NM_201372.3 | chr9:44410163-44418007 |
| 3951 | Ccdc14 | NM_172824.3 | chr16:34690615-34725194 | 4048 | Ccdc85a | NM_001166661.2 | chr11:28385683-28584324 |
| 3952 | Ccdc141 | NM_001025576.3 | chr2:77009905-77170635 | 4049 | Ccdc85b | NM_001243307.1 | chr19:5453162-5457563 |
| 3953 | Ccdc142 | NM_001081266.1 | chr6:83101515-83109121 | 4050 | Ccdc85c | NM_001159910.1 | chr12:108206344-108275417 |
| 3954 | Ccdc144b | NM_178418.4 | chr3:36007246-36053547 | 4051 | Ccdc86 | NM_023731.3 | chr19:10941480-10949266 |
| 3955 | Ccdc146 | NM_029195.1 | chr5:21292960-21424677 | 4052 | Ccdc87 | NM_207268.3 | chr19:4839365-4842528 |
| 3956 | Ccdc147 | NM_001163267.1 | chr19:47937711-48035379 | 4053 | Ccdc88a | NM_176841.4 | chr11:29374171-29510808 |
| 3957 | Ccdc148 | NM_001001178.1 | chr2:58821697-59018606 | 4054 | Ccdc88b | NM_001081291.1 | chr19:6844622-6858211 |
| 3958 | Ccdc149 | NM_001256059.1 | chr5:52374650-52471543 | 4055 | Ccdc88c | NM_026681.4 | chr12:100912699-101028983 |
| 3959 | Ccdc15 | NM_001081429.1 | chr9:37275834-37348392 | 4056 | Ccdc89 | NM_027298.1 | chr7:90426311-90428664 |
| 3960 | Ccdc150 | NM_030025.2 | chr1:54250682-54368727 | 4057 | Ccdc9 | NM_001136471.1 | chr7:16274041-16286795 |
| 3961 | Ccdc151 | NM_001163787.1 | chr9:21989870-22002634 | 4058 | Ccdc90b | NM_001162918.1 | chr7:92561148-92582294 |
| 3962 | Ccdc152 | NM_001166063.2 | chr15:3280626-3303526 | 4059 | Ccdc91 | NM_025911.2 | chr6:147475870-147632612 |
| 3963 | Ccdc153 | NM_001081369.2 | chr9:44240676-44247306 | 4060 | Ccdc92 | NM_144819.2 | chr5:124834431-124862221 |
| 3964 | Ccdc154 | NM_001079929.2 | chr17:25162460-25171913 | 4061 | Ccdc93 | NM_001025156.2 | chr1:121431066-121506460 |
| 3965 | Ccdc155 | NM_201374.2 | chr7:45183675-45204892 | 4062 | Ccdc94 | NM_028381.3 | chr17:55959186-55967951 |
| 3966 | Ccdc157 | NM_001164620.1 | chr11:4141123-4160293 | 4063 | Ccdc96 | NM_025725.2 | chr5:36484587-36488171 |
| 3967 | Ccdc158 | NM_177230.3 | chr5:92608294-92675127 | 4064 | Ccdc97 | NM_028771.2 | chr7:25711116-25719053 |
| 3968 | Ccdc159 | NM_001164614.1 | chr9:21927470-21935872 | 4065 | Ccer1 | NM_025724.2 | chr10:97693058-97694926 |
| 3969 | Ccdc160 | NM_001034059.1 | chrX:52791199-52799468 | 4066 | Cchcr1 | NM_146248.2 | chr17:35517115-35531015 |
| 3970 | Ccdc162 | NM_001303469.1 | chr10:41536409-41716568 | 4067 | Ccin | NM_001002787.2 | chr4:43983503-43985533 |
| 3971 | Ccdc163 | NM_026714.2 | chr4:116708929-116715104 | 4068 | Cck | NM_001284508.2 | chr9:121489823-121495694 |
| 3972 | Ccdc166 | NM_001163518.1 | chr15:75979871-75982285 | 4069 | Cckar | NM_009827.2 | chr5:53698484-53707704 |
| 3973 | Ccdc167 | NM_001163741.2 | chr17:29695976-29717017 | 4070 | Cckbr | NM_007627.5 | chr7:105425730-105436339 |
| 3974 | Ccdc169 | NM_001290138.1 | chr3:55137338-55172936 | 4071 | Ccl1 | NM_011329.3 | chr11:82176665-82179812 |
| 3975 | Ccdc17 | NM_001037916.3 | chr4:116596729-116600266 | 4072 | Ccl11 | NM_011330.3 | chr11:82057831-82062955 |
| 3976 | Ccdc170 | NM_001195672.1 | chr10:4509871-4561111 | 4073 | Ccl12 | NM_011331.2 | chr11:82101844-82103399 |

Fig.21 - 22

| | | | | | | | |
|---|---|---|---|---|---|---|---|
| 4074 | Ccl17 | NM_011332.3 | chr8:94810452-94812036 | 4171 | Cd200r1 | NM_021325.3 | chr16:44765735-44794977 |
| 4075 | Ccl19 | NM_011888.2 | chr4:102333-42756518 | 4172 | Cd200r2 | NM_206535.1 | chr16:44867096-44915840 |
| 4076 | Ccl2 | NM_011333.3 | chr11:82035576-82037452 | 4173 | Cd200r3 | NM_001128132.1 | chr16:44943677-44966290 |
| 4077 | Ccl20 | NM_001159738.1 | chr1:83116765-83119167 | 4174 | Cd200r4 | NM_207244.2 | chr16:44820727-44839150 |
| 4078 | Ccl21a | NM_011124.4 | chr4:120654-42773991 | 4175 | Cd207 | NM_144943.3 | chr6:83671206-83677857 |
| 4079 | Ccl21b | NM_011335.2 | chr4:0-42613253 | 4176 | Cd209a | NM_133238.5 | chr8:3743394-3748984 |
| 4080 | Ccl21c | NM_023052.2 | chr4:0-42613253 | 4177 | Cd209b | NM_001037800.3 | chr8:3917654-3926841 |
| 4081 | Ccl22 | NM_009137.2 | chr8:94745683-94751388 | 4178 | Cd209c | NM_130903.3 | chr8:3940221-3946863 |
| 4082 | Ccl24 | NM_019577.4 | chr5:135569936-135573043 | 4179 | Cd209d | NM_130904.2 | chr8:3871823-3878499 |
| 4083 | Ccl25 | NM_009138.3 | chr8:4349587-4360020 | 4180 | Cd209e | NM_130905.2 | chr8:3847972-3854286 |
| 4084 | Ccl26 | NM_001013412.2 | chr5:135560447-135563569 | 4181 | Cd209f | NM_026956.2 | chr8:4102792-4105764 |
| 4085 | Ccl27a | NM_001048179.1 | chr4:41769469-41774134 | 4182 | Cd209g | NM_027343.3 | chr8:4134735-4137707 |
| 4086 | Ccl27b | NM_001199959.1 | chr4:3059-42656005 | 4183 | Cd22 | NM_001043317.2 | chr7:30865403-30880342 |
| 4087 | Ccl28 | NM_020279.3 | chr13:119623818-119654358 | 4184 | Cd226 | NM_001039149.1 | chr18:89197426-89270327 |
| 4088 | Ccl3 | NM_011337.2 | chr11:83647842-83649378 | 4185 | Cd244 | NM_018729.2 | chr1:171559192-171585316 |
| 4089 | Ccl4 | NM_013652.2 | chr11:83662583-83664683 | 4186 | Cd247 | NM_001113391.2 | chr1:165788680-165862112 |
| 4090 | Ccl5 | NM_013653.3 | chr11:83525778-83530518 | 4187 | Cd248 | NM_054042.2 | chr19:5068077-5070639 |
| 4091 | Ccl6 | NM_009139.3 | chr11:83587885-83593087 | 4188 | Cd24a | NM_009846.2 | chr10:43579168-43584265 |
| 4092 | Ccl7 | NM_013654.3 | chr11:82045711-82047523 | 4189 | Cd27 | NM_001033126.2 | chr6:125232621-125237027 |
| 4093 | Ccl8 | NM_021443.3 | chr11:82115184-82116799 | 4190 | Cd274 | NM_021893.3 | chr19:29367437-29388094 |
| 4094 | Ccl9 | NM_011338.2 | chr11:83572916-83578636 | 4191 | Cd276 | NM_133983.4 | chr9:58524299-58540940 |
| 4095 | Ccm2 | NM_001190343.1 | chr11:6546886-6596761 | 4192 | Cd28 | NM_007642.4 | chr1:60746387-60773359 |
| 4096 | Ccm2l | NM_145536.3 | chr2:153065954-153081735 | 4193 | Cd2ap | NM_009847.3 | chr17:42792950-42876424 |
| 4097 | Ccna1 | NM_007628.3 | chr3:55045468-55055055 | 4194 | Cd2bp2 | NM_001285905.1 | chr7:127191659-127196077 |
| 4098 | Ccna2 | NM_009828.2 | chr3:36564864-36571996 | 4195 | Cd300a | NM_170758.3 | chr11:114890040-114904651 |
| 4099 | Ccnb1 | NM_172301.3 | chr13:100778738-100786486 | 4196 | Cd300c | NM_199225.1 | chr11:114956277-114960417 |
| 4100 | Ccnb1ip1 | NM_001111119.1 | chr14:50789248-50795728 | 4197 | Cd300e | NM_172050.2 | chr11:115051916-115062038 |
| 4101 | Ccnb2 | NM_007630.2 | chr9:70407688-70421554 | 4198 | Cd300lb | NM_199221.2 | chr11:114922780-114934386 |
| 4102 | Ccnb3 | NM_183015.3 | chrX:6979651-7041619 | 4199 | Cd300ld | NM_145437.2 | chr11:114982445-114989886 |
| 4103 | Ccnc | NM_001122982.2 | chr4:21727700-21747962 | 4200 | Cd300lf | NM_001169153.1 | chr11:115116213-115133992 |
| 4104 | Ccnd1 | NM_007631.2 | chr7:144929930-144939925 | 4201 | Cd300lg | NM_001160711.1 | chr11:102041510-102055617 |
| 4105 | Ccnd2 | NM_009829.3 | chr6:127125708-127151048 | 4202 | Cd300lh | NM_199201.1 | chr11:115031485-115048295 |
| 4106 | Ccnd3 | NM_001081635.1 | chr17:47505050-47599688 | 4203 | Cd302 | NM_001290660.1 | chr2:60251992-60284484 |
| 4107 | Ccndbp1 | NM_010761.2 | chr2:121008407-121016912 | 4204 | Cd320 | NM_019421.3 | chr17:33843090-33849774 |
| 4108 | Ccne1 | NM_007633.2 | chr7:38097983-38107490 | 4205 | Cd33 | NM_001111058.1 | chr7:43527455-43533171 |
| 4109 | Ccne2 | NM_001037134.2 | chr4:11191350-11204779 | 4206 | Cd34 | NM_001111059.1 | chr1:194938820-194961292 |
| 4110 | Ccnf | NM_007634.4 | chr17:24223231-24251409 | 4207 | Cd36 | NM_001159555.1 | chr5:17781689-17835896 |
| 4111 | Ccng1 | NM_009831.2 | chr11:40748551-40755286 | 4208 | Cd37 | NM_001290802.1 | chr7:45233631-45239115 |
| 4112 | Ccng2 | NM_007635.4 | chr5:93267572-93276231 | 4209 | Cd38 | NM_007646.5 | chr5:43868808-43912374 |
| 4113 | Ccnh | NM_023243.5 | chr13:85189476-85213723 | 4210 | Cd3d | NM_013487.3 | chr9:44981785-44987052 |
| 4114 | Ccni | NM_017367.3 | chr5:93181932-93206495 | 4211 | Cd3e | NM_007648.4 | chr9:44998742-45009590 |
| 4115 | Ccnj | NM_172839.4 | chr19:40831278-40848570 | 4212 | Cd3eap | NM_145822.2 | chr7:19356008-19359483 |
| 4116 | Ccnjl | NM_001045530.2 | chr11:43528748-43586999 | 4213 | Cd3g | NM_009850.2 | chr9:44969571-44980431 |
| 4117 | Ccnk | NM_009832.2 | chr12:108179737-108203359 | 4214 | Cd4 | NM_013488.2 | chr6:124864692-124888209 |
| 4118 | Ccnl1 | NM_019937.3 | chr3:65946150-65958225 | 4215 | Cd40 | NM_011611.2 | chr2:165055635-165071654 |
| 4119 | Ccnl2 | NM_207678.1 | chr4:155812488-155824543 | 4216 | Cd40lg | NM_011616.2 | chrX:57212142-57224042 |
| 4120 | Ccno | NM_001081062.1 | chr13:112987801-112990778 | 4217 | Cd44 | NM_001039150.1 | chr2:102811141-102901665 |
| 4121 | Ccnt1 | NM_009833.1 | chr15:98543210-98567636 | 4218 | Cd46 | NM_010778.4 | chr1:195041237-195092248 |
| 4122 | Ccnt2 | NM_028399.1 | chr1:127774163-127804837 | 4219 | Cd47 | NM_010581.3 | chr16:49855653-49911683 |
| 4123 | Ccny | NM_026484.3 | chr18:9314043-9450150 | 4220 | Cd48 | NM_007649.4 | chr1:171682054-171705257 |
| 4124 | Ccnyl1 | NM_001097644.1 | chr1:64691344-64725642 | 4221 | Cd5 | NM_007650.2 | chr19:10718142-10738974 |
| 4125 | Ccp110 | NM_182995.1 | chr7:118712610-118737018 | 4222 | Cd52 | NM_013706.2 | chr4:134093537-134095073 |
| 4126 | Ccpg1 | NM_001114328.2 | chr9:72985503-73013938 | 4223 | Cd53 | NM_007651.3 | chr3:106758860-106790149 |
| 4127 | Ccpg1os | NM_001198789.1 | chr9:72979724-72985376 | 4224 | Cd55 | NM_010016.2 | chr1:130439029-130462740 |
| 4128 | Ccr1 | NM_009912.4 | chr9:123962125-123968692 | 4225 | Cd59a | NM_001111060.2 | chr2:104095800-104115410 |
| 4129 | Ccr10 | NM_007721.4 | chr11:101172997-101175443 | 4226 | Cd59b | NM_181858.1 | chr2:104071009-104084957 |
| 4130 | Ccr1l1 | NM_007718.3 | chr9:123976948-123978408 | 4227 | Cd5l | NM_009690.2 | chr3:87357880-87371074 |
| 4131 | Ccr2 | NM_009915.2 | chr9:124102182-124109140 | 4228 | Cd6 | NM_001037801.2 | chr19:10789338-10830058 |
| 4132 | Ccr3 | NM_009914.4 | chr9:124021970-124031689 | 4229 | Cd63 | NM_001042580.1 | chr10:128908918-128912818 |
| 4133 | Ccr4 | NM_009916.2 | chr9:114490315-114496544 | 4230 | Cd68 | NM_001291058.1 | chr11:69664212-69666170 |
| 4134 | Ccr5 | NM_009917.5 | chr9:124121542-124127183 | 4231 | Cd69 | NM_001033122.3 | chr6:129267324-129275369 |
| 4135 | Ccr6 | NM_001190333.1 | chr17:8236042-8257129 | 4232 | Cd7 | NM_009854.2 | chr11:121036748-121039478 |
| 4136 | Ccr7 | NM_007719.2 | chr11:99144198-99155077 | 4233 | Cd70 | NM_011617.2 | chr17:57145996-57149777 |
| 4137 | Ccr8 | NM_007720.2 | chr9:120092132-120094906 | 4234 | Cd72 | NM_001110320.1 | chr4:43447723-43454626 |
| 4138 | Ccr9 | NM_001166625.1 | chr9:123767210-123783457 | 4235 | Cd74 | NM_001042605.1 | chr18:60803848-60812652 |
| 4139 | Ccrl2 | NM_017466.5 | chr9:111054833-111057270 | 4236 | Cd79a | NM_007655.3 | chr7:24897510-24902197 |
| 4140 | Ccrn4l | NM_009834.2 | chr3:51224446-51251654 | 4237 | Cd79b | NM_008339.3 | chr11:106311340-106314562 |
| 4141 | Ccs | NM_016892.3 | chr19:4825365-4839322 | 4238 | Cd80 | NM_009855.2 | chr16:38458932-38486932 |
| 4142 | Ccsap | NM_028536.1 | chr8:123840843-123860209 | 4239 | Cd81 | NM_133655.2 | chr7:143052749-143067930 |
| 4143 | Ccser1 | NM_001164316.1 | chr6:61180324-62382863 | 4240 | Cd82 | NM_001136055.2 | chr2:93419101-93462950 |
| 4144 | Ccser2 | NM_027045.1 | chr14:36874935-36968764 | 4241 | Cd83 | NM_001289915.1 | chr13:43785111-43803133 |
| 4145 | Cct2 | NM_007636.2 | chr10:117050997-117063814 | 4242 | Cd84 | NM_001252472.1 | chr1:171839696-171855097 |
| 4146 | Cct3 | NM_009836.1 | chr3:88297134-88321766 | 4243 | Cd86 | NM_019388.3 | chr16:36603868-36666077 |
| 4147 | Cct4 | NM_009837.1 | chr11:22990592-23003336 | 4244 | Cd8a | NM_001081110.2 | chr6:71373426-71379171 |
| 4148 | Cct5 | NM_007637.2 | chr15:31590883-31601804 | 4245 | Cd8b1 | NM_009858.2 | chr6:71322811-71337451 |
| 4149 | Cct6a | NM_009838.2 | chr5:129787355-129846443 | 4246 | Cd9 | NM_007657.3 | chr6:125460265-125494755 |
| 4150 | Cct6b | NM_001291242.1 | chr11:82719247-82764321 | 4247 | Cd93 | NM_010740.3 | chr2:148436650-148443535 |
| 4151 | Cct7 | NM_007638.4 | chr6:85451504-85468477 | 4248 | Cd96 | NM_032465.2 | chr16:46035656-46120248 |
| 4152 | Cct8 | NM_009840.3 | chr16:87483324-87495869 | 4249 | Cd97 | NM_001163029.1 | chr8:83723250-83741311 |
| 4153 | Cct8l1 | NM_198621.2 | chr5:25516066-25518027 | 4250 | Cd99l2 | NM_001199349.1 | chrX:71420059-71492849 |
| 4154 | Ccz1 | NM_177682.3 | chr5:143987908-144014853 | 4251 | Cda | NM_028176.1 | chr4:138338527-138367955 |
| 4155 | Cd101 | NM_001190333.1 | chr3:100993529-101029495 | 4252 | Cdadc1 | NM_001168535.1 | chr14:59559387-59597959 |
| 4156 | Cd109 | NM_153098.3 | chr9:78615545-78716260 | 4253 | Cdan1 | NM_026891.2 | chr2:120716153-120731517 |
| 4157 | Cd14 | NM_009841.4 | chr18:36725063-36726815 | 4254 | Cdc123 | NM_133837.4 | chr2:5794293-5844960 |
| 4158 | Cd151 | NM_001111049.1 | chr7:141467361-141471481 | 4255 | Cdc14a | NM_001080818.2 | chr3:116272552-116424032 |
| 4159 | Cd160 | NM_001163496.1 | chr3:96798762-96829351 | 4256 | Cdc14b | NM_001122989.1 | chr13:64192544-64248538 |
| 4160 | Cd163 | NM_001170395.1 | chr6:124304650-124330527 | 4257 | Cdc16 | NM_027276.2 | chr8:13757689-13781882 |
| 4161 | Cd163l1 | NM_172909.4 | chr7:140218266-140231145 | 4258 | Cdc20 | NM_023223.2 | chr4:118432900-118437343 |
| 4162 | Cd164 | NM_016898.2 | chr10:41519499-41531042 | 4259 | Cdc20b | NM_001281487.1 | chr13:113035378-113091135 |
| 4163 | Cd164l2 | NM_027152.1 | chr4:133220808-133224554 | 4260 | Cdc23 | NM_178347.4 | chr18:34631682-34651736 |
| 4164 | Cd177 | NM_026862.3 | chr7:24743982-24760311 | 4261 | Cdc25a | NM_007658.3 | chr9:109875578-109893890 |
| 4165 | Cd180 | NM_008533.2 | chr13:102693557-102706631 | 4262 | Cdc25b | NM_001111075.4 | chr2:131186947-131198511 |
| 4166 | Cd19 | NM_009844.2 | chr7:126408447-126414870 | 4263 | Cdc25c | NM_009860.3 | chr18:34732994-34751533 |
| 4167 | Cd1d1 | NM_007639.3 | chr3:86995835-86999340 | 4264 | Cdc26 | NM_139291.3 | chr4:62394588-62408623 |
| 4168 | Cd1d2 | NM_001289449.1 | chr3:86986585-86989532 | 4265 | Cdc27 | NM_001285988.1 | chr11:104502525-104550620 |
| 4169 | Cd2 | NM_013486.2 | chr3:101275907-101287939 | 4266 | Cdc34 | NM_177613.2 | chr10:79682194-79688398 |
| 4170 | Cd200 | NM_010818.3 | chr16:45382134-45409053 | 4267 | Cdc37 | NM_016742.4 | chr9:21138540-21149906 |

Fig.21 - 23

| | | | | | | | |
|---|---|---|---|---|---|---|---|
| 4268 | Cdc37l1 | NM_025950.3 | chr19:28990351-29020237 | 4365 | Cdkn2d | NM_009878.3 | chr9:21288463-21291209 |
| 4269 | Cdc40 | NM_027879.2 | chr10:40831621-40883143 | 4366 | Cdkn3 | NM_028222.1 | chr14:46760540-46771525 |
| 4270 | Cdc42 | NM_001243769.1 | chr4:137321762-137357759 | 4367 | Cdnf | NM_177647.4 | chr2:3513064-3526376 |
| 4271 | Cdc42bpa | NM_001033285.1 | chr1:179961088-180165602 | 4368 | Cdo1 | NM_033037.3 | chr18:46713204-46728342 |
| 4272 | Cdc42bpb | NM_183016.2 | chr12:111292971-111377718 | 4369 | Cdon | NM_021339.2 | chr9:35452075-35507652 |
| 4273 | Cdc42bpg | NM_001033342.1 | chr19:6306456-6325652 | 4370 | Cdpf1 | NM_001164625.1 | chr15:85806971-85811697 |
| 4274 | Cdc42ep1 | NM_027219.3 | chr15:78842646-78850902 | 4371 | Cdr1 | NM_001166658.1 | chrX:61183245-61185558 |
| 4275 | Cdc42ep2 | NM_026772.2 | chr19:5917555-5924816 | 4372 | Cdr2 | NM_007672.2 | chr7:120957036-120982312 |
| 4276 | Cdc42ep3 | NM_026514.2 | chr17:79334024-79355091 | 4373 | Cdr2l | NM_001080929.1 | chr11:115381915-115396132 |
| 4277 | Cdc42ep4 | NM_001163346.1 | chr11:113726849-113751815 | 4374 | Cdrt4 | NM_025496.1 | chr11:62951192-62993095 |
| 4278 | Cdc42ep5 | NM_021454.3 | chr7:4151259-4164699 | 4375 | Cds1 | NM_173370.3 | chr5:101765129-101823852 |
| 4279 | Cdc42se1 | NM_001038708.3 | chr3:95228942-95236424 | 4376 | Cds2 | NM_001291039.1 | chr2:132285938-132312050 |
| 4280 | Cdc42se2 | NM_178626.3 | chr11:54717414-54787703 | 4377 | Cdsn | NM_001008424.2 | chr17:35552127-35557180 |
| 4281 | Cdc45 | NM_001161623.1 | chr16:18780446-18811639 | 4378 | Cdt1 | NM_026014.3 | chr8:122568014-122573130 |
| 4282 | Cdc5l | NM_152810.2 | chr17:45391887-45433707 | 4379 | Cdv3 | NM_001134426.1 | chr9:103353101-103365780 |
| 4283 | Cdc6 | NM_001025779.1 | chr11:98907888-98923942 | 4380 | Cdx1 | NM_009880.3 | chr18:61018861-61036199 |
| 4284 | Cdc7 | NM_001271566.1 | chr5:106964560-106984439 | 4381 | Cdx2 | NM_007673.3 | chr5:147300899-147307249 |
| 4285 | Cdc73 | NM_145991.2 | chr1:143603275-143702893 | 4382 | Cdx4 | NM_007674.3 | chrX:103321397-103330224 |
| 4286 | Cdca2 | NM_001110162.1 | chr14:67676353-67715610 | 4383 | Cdyl | NM_001123386.1 | chr13:35741401-35874064 |
| 4287 | Cdca3 | NM_013538.5 | chr6:124830175-124833701 | 4384 | Cdyl2 | NM_029441.3 | chr8:116568723-116732991 |
| 4288 | Cdca4 | NM_028023.3 | chr12:112820234-112829389 | 4385 | Ceacam1 | NM_001039185.1 | chr7:25461701-25477625 |
| 4289 | Cdca5 | NM_026410.3 | chr19:6085096-6091773 | 4386 | Ceacam10 | NM_007675.4 | chr7:24777203-24784655 |
| 4290 | Cdca7 | NM_025866.3 | chr2:72476218-72486890 | 4387 | Ceacam11 | NM_023289.1 | chr7:17972166-17978556 |
| 4291 | Cdca7l | NM_146040.1 | chr12:117843860-117878706 | 4388 | Ceacam12 | NM_001162523 | chr7:18065928-18077986 |
| 4292 | Cdca8 | NM_026560.4 | chr4:124918464-124936917 | 4389 | Ceacam13 | NM_027210 | chr7:18009888-18019220 |
| 4293 | Cdcp1 | NM_133974.3 | chr9:123172284-123216038 | 4390 | Ceacam14 | NM_025957 | chr7:17812681-17815627 |
| 4294 | Cdcp2 | NM_172873.3 | chr4:107096890-107107748 | 4391 | Ceacam15 | NM_175315.1 | chr7:16671330-16675705 |
| 4295 | Cdh1 | NM_009864.2 | chr8:106603367-106670246 | 4392 | Ceacam16 | NM_001033419.1 | chr7:19852096-19861299 |
| 4296 | Cdh10 | NM_009865.2 | chr15:18820328-19014234 | 4393 | Ceacam18 | NM_028236.1 | chr7:43634738-43649294 |
| 4297 | Cdh11 | NM_009866.5 | chr8:102632094-102785111 | 4394 | Ceacam19 | NM_177036.5 | chr7:19875741-19887965 |
| 4298 | Cdh12 | NM_001008420.2 | chr15:21111451-21589533 | 4395 | Ceacam2 | NM_001113368.1 | chr7:25516041-25540004 |
| 4299 | Cdh13 | NM_019707.4 | chr8:118283754-119323448 | 4396 | Ceacam20 | NM_027839.2 | chr7:19965411-19991111 |
| 4300 | Cdh15 | NM_007662.2 | chr8:122848373-122867397 | 4397 | Ceacam3 | NM_054059.1 | chr7:17150399-17163231 |
| 4301 | Cdh16 | NM_001252627.1 | chr8:104614137-104624396 | 4398 | Ceacam5 | NM_028480.2 | chr7:17713251-17761121 |
| 4302 | Cdh17 | NM_019753.4 | chr4:11758156-11817905 | 4399 | Ceacam9 | NM_011927.4 | chr7:16721928-16726110 |
| 4303 | Cdh18 | NM_001081299.1 | chr15:23036462-23474418 | 4400 | Ceacam-ps1 | NR_003247.2 | chr7:16649029-16657478 |
| 4304 | Cdh19 | NM_001081386.1 | chr1:110889194-110977372 | 4401 | Cebpa | NM_001287514.1 | chr7:35119292-35121928 |
| 4305 | Cdh2 | NM_007664.4 | chr18:16588876-16809049 | 4402 | Cebpb | NM_009883.4 | chr2:167688914-167690432 |
| 4306 | Cdh20 | NM_011800.4 | chr1:104768818-104995481 | 4403 | Cebpd | NM_007679.4 | chr16:15887285-15889545 |
| 4307 | Cdh22 | NM_174988.3 | chr2:165111506-165234737 | 4404 | Cebpe | NM_207131.1 | chr14:54710362-54712174 |
| 4308 | Cdh23 | NM_001252635.1 | chr10:60302749-60696490 | 4405 | Cebpg | NM_009884.3 | chr7:35046421-35056566 |
| 4309 | Cdh24 | NM_199470.2 | chr14:54631991-54641364 | 4406 | Cebpz | NM_001024806.2 | chr17:78919002-78937070 |
| 4310 | Cdh26 | NM_001291189.1 | chr2:178430514-178487366 | 4407 | Cebpzos | NM_001177402.1 | chr17:78916498-78921048 |
| 4311 | Cdh3 | NM_001037809.5 | chr8:106510851-106556911 | 4408 | Cecr2 | NM_001128151.1 | chr6:120666420-120771191 |
| 4312 | Cdh4 | NM_009867.2 | chr2:179442477-179899375 | 4409 | Cecr5 | NM_144815.2 | chr6:120509493-120531299 |
| 4313 | Cdh5 | NM_009868.4 | chr8:104101624-104144502 | 4410 | Cecr6 | NM_033567.1 | chr6:120488938-120493807 |
| 4314 | Cdh6 | NM_007666.3 | chr15:13034199-13173639 | 4411 | Cel | NM_009885.2 | chr2:28555819-28563403 |
| 4315 | Cdh7 | NM_172853.2 | chr1:109983736-110139001 | 4412 | Cela1 | NM_033612.2 | chr15:100674421-100687920 |
| 4316 | Cdh8 | NM_001039154.2 | chr8:99028768-99416471 | 4413 | Cela2a | NM_007919.2 | chr4:141814962-141826003 |
| 4317 | Cdh9 | NM_009869.1 | chr15:16778100-16856777 | 4414 | Cela3b | NM_026419.2 | chr4:137421007-137430520 |
| 4318 | Cdhr1 | NM_130878.2 | chr14:37077848-37098311 | 4415 | Celf1 | NM_001244891.1 | chr2:90940396-91019497 |
| 4319 | Cdhr2 | NM_001033364.3 | chr13:54701462-54736662 | 4416 | Celf2 | NM_001110228.1 | chr2:6539698-6884996 |
| 4320 | Cdhr3 | NM_001024478.1 | chr12:33033795-33092875 | 4417 | Celf3 | NM_001289613.1 | chr3:94478548-94492198 |
| 4321 | Cdhr5 | NM_001114322.1 | chr7:141269084-141276786 | 4418 | Celf4 | NM_001146292.1 | chr18:25477619-25753983 |
| 4322 | Cdip1 | NM_025670.4 | chr16:4765460-4789935 | 4419 | Celf5 | NM_176954.3 | chr10:81459227-81482709 |
| 4323 | Cdipt | NM_026638.3 | chr7:126975913-126980501 | 4420 | Celf6 | NM_175235.3 | chr9:59578336-59607292 |
| 4324 | Cdk1 | NM_007659.3 | chr10:69336634-69352912 | 4421 | Celrr | NR_038008.1 | chr1:121087404-121120975 |
| 4325 | Cdk10 | NM_194444.2 | chr8:123224840-123232256 | 4422 | Celsr1 | NM_009886.2 | chr15:85898757-86033777 |
| 4326 | Cdk11b | NM_007661.3 | chr4:155624868-155649932 | 4423 | Celsr2 | NM_001004177.2 | chr3:108390847-108415494 |
| 4327 | Cdk12 | NM_001109626.1 | chr11:98203304-98253540 | 4424 | Celsr3 | NM_080437.2 | chr9:108826319-108852969 |
| 4328 | Cdk13 | NM_001081058.2 | chr13:17715961-17805097 | 4425 | Cemip | NM_030728.4 | chr7:83932856-84086505 |
| 4329 | Cdk14 | NM_011074.3 | chr5:4803383-5380251 | 4426 | Cend1 | NM_021316.4 | chr7:141426450-141429420 |
| 4330 | Cdk15 | NM_001033373.2 | chr1:59256906-59352369 | 4427 | Cenpa | NM_007681 | chr5:30666885-30674837 |
| 4331 | Cdk16 | NM_011049.5 | chrX:20688425-20699879 | 4428 | Cenpb | NM_007682 | chr2:131177288-131180012 |
| 4332 | Cdk17 | NM_146239.2 | chr10:93160875-93241342 | 4429 | Cenpc1 | NM_007683.3 | chr5:86012024-86065583 |
| 4333 | Cdk18 | NM_008795.2 | chr1:132113546-132139685 | 4430 | Cenpe | NM_173762 | chr3:135212562-135273540 |
| 4334 | Cdk19 | NM_001168304.1 | chr10:40349307-40483818 | 4431 | Cenpf | NM_001081363.2 | chr1:189640613-189688086 |
| 4335 | Cdk2 | NM_016756.4 | chr10:128697938-128705051 | 4432 | Cenph | NM_021886.1 | chr13:100759685-100775899 |
| 4336 | Cdk20 | NM_053180.1 | chr13:64432552-64439721 | 4433 | Cenpi | NM_145924.3 | chrX:134308083-134363104 |
| 4337 | Cdk2ap1 | NM_013812.2 | chr5:124345438-124354628 | 4434 | Cenpj | NM_001014996 | chr14:56526760-56571846 |
| 4338 | Cdk2ap2 | NM_026373 | chr19:4097350-4099017 | 4435 | Cenpk | NM_021790.2 | chr13:104229387-104249622 |
| 4339 | Cdk3-ps | NR_004853.1 | chr11:116216001-116220287 | 4436 | Cenpl | NM_001159930.2 | chr1:161070766-161086724 |
| 4340 | Cdk4 | NM_009870.3 | chr10:127063602-127067282 | 4437 | Cenpm | NM_001080158.1 | chr15:82233775-82244336 |
| 4341 | Cdk5 | NM_007668.3 | chr5:24418241-24423530 | 4438 | Cenpn | NM_028131.3 | chr8:116921739-116941503 |
| 4342 | Cdk5r1 | NM_009871.2 | chr11:80477045-80481179 | 4439 | Cenpo | NM_134046.5 | chr12:4211671-4234294 |
| 4343 | Cdk5r2 | NM_009872.3 | chr1:74855028-74857732 | 4440 | Cenpp | NM_025495.3 | chr13:49464058-49652731 |
| 4344 | Cdk5rap1 | NM_025876.2 | chr2:154335385-154372719 | 4441 | Cenpq | NM_031863.3 | chr17:40923054-40934551 |
| 4345 | Cdk5rap2 | NM_145990.4 | chr4:70216854-70410435 | 4442 | Cenpt | NM_177150.2 | chr8:105844677-105852008 |
| 4346 | Cdk5rap3 | NM_030248.2 | chr11:96907424-96916514 | 4443 | Cenpu | NM_027973.3 | chr8:46552068-46579584 |
| 4347 | Cdk6 | NM_009873.3 | chr5:3343892-3531005 | 4444 | Cenpv | NM_028448.1 | chr11:62524943-62539261 |
| 4348 | Cdk7 | NM_009874.3 | chr13:100697024-100730942 | 4445 | Cenpw | NM_001109747.1 | chr10:30196008-30200540 |
| 4349 | Cdk8 | NM_153599.3 | chr5:146231674-146302874 | 4446 | Cep104 | NM_177673.2 | chr4:153975560-154007225 |
| 4350 | Cdk9 | NM_130860.3 | chr2:32705781-32712784 | 4447 | Cep112 | NM_029586.2 | chr11:108682617-108860615 |
| 4351 | Cdkal1 | NM_144536.3 | chr13:29325300-29855673 | 4448 | Cep120 | NM_178686.3 | chr18:53681722-53744547 |
| 4352 | Cdkl1 | NM_183294.2 | chr12:69746847-69790707 | 4449 | Cep128 | NM_181815.3 | chr12:90998491-91384409 |
| 4353 | Cdkl2 | NM_001276315.1 | chr5:92006073-92042696 | 4450 | Cep131 | NM_009734.3 | chr11:120064429-120086827 |
| 4354 | Cdkl3 | NM_001166653.1 | chr11:52004220-52038165 | 4451 | Cep135 | NM_199032.2 | chr5:76591713-76646466 |
| 4355 | Cdkl4 | NM_001033443.4 | chr17:80523549-80563834 | 4452 | Cep152 | NM_001081091.1 | chr2:125563087-125625113 |
| 4356 | Cdkl5 | NM_001024624.2 | chrX:160784307-160994681 | 4453 | Cep162 | NM_199316.2 | chr9:87191962-87255532 |
| 4357 | Cdkn1a | NM_001111099.2 | chr17:29090978-29100722 | 4454 | Cep164 | NM_001081373.2 | chr9:45766945-45828638 |
| 4358 | Cdkn1b | NM_009875.4 | chr6:134920400-134925525 | 4455 | Cep170 | NM_001099637.2 | chr1:176733652-176807124 |
| 4359 | Cdkn1c | NM_001161624.1 | chr7:143458338-143461050 | 4456 | Cep170b | NM_001024602.3 | chr12:112722173-112746591 |
| 4360 | Cdkn2a | NM_001040654.1 | chr4:89274472-89282192 | 4457 | Cep19 | NM_025892.2 | chr16:32099801-32108054 |
| 4361 | Cdkn2aip | NM_172407.3 | chr8:47709343-47713931 | 4458 | Cep192 | NM_027556.1 | chr18:67800106-67885170 |
| 4362 | Cdkn2aipnl | NM_029976.3 | chr11:51967630-51977336 | 4459 | Cep250 | NM_001129999.1 | chr2:155956557-155998900 |
| 4363 | Cdkn2b | NM_007670.4 | chr4:89306288-89311032 | 4460 | Cep290 | NM_146009.2 | chr10:100488288-100573655 |
| 4364 | Cdkn2c | NM_007671.3 | chr4:109660875-109665372 | 4461 | Cep350 | NM_001039184.1 | chr1:155844963-155973255 |

Fig.21 - 24

| | | | | | | | |
|---|---|---|---|---|---|---|---|
| 4462 | Cep41 | NM_031998.3 | chr6:30653456-30693749 | 4559 | Chd8 | NM_201637.2 | chr14:52198150-52237572 |
| 4463 | Cep44 | NM_001009951.1 | chr8:56531521-56550566 | 4560 | Chd9 | NM_177224.2 | chr8:90828834-91054508 |
| 4464 | Cep55 | NM_001164362.1 | chr19:38055041-38074425 | 4561 | Chdh | NM_001136240.1 | chr14:30008999-30040466 |
| 4465 | Cep57 | NM_026665.4 | chr9:13807787-13827107 | 4562 | Chek1 | NM_007691.5 | chr9:36708481-36726658 |
| 4466 | Cep57l1 | NM_001243074.1 | chr10:41718839-41809868 | 4563 | Chek2 | NM_016681.3 | chr5:110840016-110874133 |
| 4467 | Cep63 | NM_001081122.1 | chr9:102586577-102626124 | 4564 | Cherp | NM_138585.3 | chr8:72460482-72475233 |
| 4468 | Cep68 | NM_172260.3 | chr11:20227036-20249424 | 4565 | Chfr | NM_001289577.1 | chr5:110135829-110171973 |
| 4469 | Cep70 | NM_023873.3 | chr9:99243467-99300403 | 4566 | Chga | NM_007693.1 | chr12:102554968-102565027 |
| 4470 | Cep72 | NM_028959.3 | chr13:74036494-74062285 | 4567 | Chgb | NM_007694.4 | chr2:132781277-132795072 |
| 4471 | Cep76 | NM_001081073.1 | chr18:67617396-67641336 | 4568 | Chia1 | NM_023186.3 | chr3:106113381-106132116 |
| 4472 | Cep78 | NM_198019.2 | chr19:15955772-15984989 | 4569 | Chic1 | NM_009767.2 | chrX:103356475-103396118 |
| 4473 | Cep83 | NM_029852.2 | chr10:94688789-94790336 | 4570 | Chic2 | NM_028850.5 | chr5:75005003-75044641 |
| 4474 | Cep83os | NR_015524.1 | chr10:94673492-94688613 | 4571 | Chid1 | NM_001142681.1 | chr7:141493135-141539857 |
| 4475 | Cep85 | NM_144527.3 | chr4:134129857-134187085 | 4572 | Chil1 | NM_007695.3 | chr1:134182403-134190031 |
| 4476 | Cep85l | NM_001204983.1 | chr10:53278080-53379851 | 4573 | Chil3 | NM_009892.3 | chr3:106147553-106167564 |
| 4477 | Cep89 | NM_028120.2 | chr7:35397092-35438684 | 4574 | Chil4 | NM_145126.2 | chr3:106201490-106219479 |
| 4478 | Cep95 | NM_001166685.1 | chr11:106789251-106818861 | 4575 | Chil6 | NM_178412.2 | chr3:106387383-106406182 |
| 4479 | Cep97 | NM_001159364.1 | chr16:55924360-55934848 | 4576 | Chit1 | NM_001284524.1 | chr1:134111241-134151540 |
| 4480 | Cept1 | NM_133869.4 | chr3:106502259-106547802 | 4577 | Chka | NM_001271496.1 | chr19:3851772-3894369 |
| 4481 | Cer1 | NM_009887.2 | chr4:82881750-82885151 | 4578 | Chkb | NM_007692.6 | chr15:89426348-89429927 |
| 4482 | Cercam | NM_207298.2 | chr2:29869493-29882840 | 4579 | ChkbCpt1b | NR_004843.2 | chr15:89416404-89429927 |
| 4483 | Cerk | NM_145475.4 | chr15:86139100-86186141 | 4580 | Chl1 | NM_007697.2 | chr6:103510875-103733035 |
| 4484 | Cerkl | NM_001048176.1 | chr2:79332490-79428988 | 4581 | Chm | NM_018818.2 | chrX:113040591-113185515 |
| 4485 | Cers1 | NM_138647.3 | chr8:70315774-70331588 | 4582 | Chml | NM_021350.2 | chr1:175682237-175688353 |
| 4486 | Cers2 | NM_029789.1 | chr3:95315251-95323568 | 4583 | Chmp1a | NM_145606.3 | chr8:123204260-123212788 |
| 4487 | Cers3 | NM_001164201.1 | chr7:66743503-66823692 | 4584 | Chmp1b | NM_024190.2 | chr18:67205358-67207887 |
| 4488 | Cers4 | NM_026058.4 | chr8:4493404-4526079 | 4585 | Chmp2a | NM_026885.3 | chr7:13032005-13034777 |
| 4489 | Cers5 | NM_028015.2 | chr15:99735591-99772515 | 4586 | Chmp2b | NM_026879.2 | chr16:65539132-65562697 |
| 4490 | Cers6 | NM_172856.3 | chr2:68861556-69111290 | 4587 | Chmp3 | NM_025783.3 | chr6:71543853-71581574 |
| 4491 | Ces1a | NM_001013764.2 | chr8:93020214-93048192 | 4588 | Chmp4b | NM_029362.3 | chr2:154657025-154694783 |
| 4492 | Ces1b | NM_001081372.1 | chr8:93056726-93080017 | 4589 | Chmp4c | NM_025519.2 | chr3:10366972-10391005 |
| 4493 | Ces1c | NM_007954.4 | chr8:93099015-93131283 | 4590 | Chmp5 | NM_029814.1 | chr4:40948552-40965302 |
| 4494 | Ces1d | NM_053200.2 | chr8:93166071-93197804 | 4591 | Chmp6 | NM_001085498.2 | chr11:119913809-119919552 |
| 4495 | Ces1e | NM_133660.3 | chr8:93201217-93229619 | 4592 | Chmp7 | NM_134078.4 | chr14:69716978-69732570 |
| 4496 | Ces1f | NM_144930.2 | chr8:93256235-93279736 | 4593 | Chn1 | NM_001113246.2 | chr2:73610659-73775346 |
| 4497 | Ces1g | NM_021456.4 | chr8:93302368-93337209 | 4594 | Chn1os3 | NR_040623.1 | chr2:73596525-73616012 |
| 4498 | Ces2a | NM_001190330.1 | chr8:104734002-104741634 | 4595 | Chn2 | NM_001163640.1 | chr6:54039931-54301812 |
| 4499 | Ces2b | NM_198171.2 | chr8:104831643-104838652 | 4596 | Chodl | NM_139134.3 | chr16:78930947-78951728 |
| 4500 | Ces2c | NM_145603.2 | chr8:104847067-104854482 | 4597 | Chordc1 | NM_025844.2 | chr9:18292266-18314000 |
| 4501 | Ces2d-ps | NR_033726.1 | chr8:104867423-104874082 | 4598 | Chp1 | NM_019769.3 | chr2:119547706-119587022 |
| 4502 | Ces2e | NM_001163756.1 | chr8:104926259-104934672 | 4599 | Chp2 | NM_027363.1 | chr7:122219495-122222539 |
| 4503 | Ces2f | NM_001079865.2 | chr8:104947355-104955962 | 4600 | Chpf | NM_001001565.2 | chr1:75474568-75479471 |
| 4504 | Ces2g | NM_197999.2 | chr8:104961717-104969537 | 4601 | Chpf2 | NM_133913.2 | chr5:24586749-24592486 |
| 4505 | Ces2h | NM_001272045.1 | chr8:105000852-105020410 | 4602 | Chpt1 | NM_001146690.1 | chr10:88472812-88503970 |
| 4506 | Ces3a | NM_001164681.1 | chr8:105048598-105058414 | 4603 | Chrac1 | NM_053068.3 | chr15:73090411-73094075 |
| 4507 | Ces3b | NM_001159415.1 | chr8:105083754-105093591 | 4604 | Chrd | NM_001278041.1 | chr16:20733126-20742384 |
| 4508 | Ces4a | NM_146213.2 | chr8:105131799-105150109 | 4605 | Chrdl1 | NM_001114385.1 | chrX:143285673-143394262 |
| 4509 | Ces5a | NM_001003951.2 | chr8:93499212-93535707 | 4606 | Chrdl2 | NM_001291320.1 | chr7:100009917-100034726 |
| 4510 | Cetn1 | NM_007593.5 | chr18:9618418-9619469 | 4607 | Chrm1 | NM_001112697.1 | chr19:8664004-8683602 |
| 4511 | Cetn2 | NM_019405.6 | chrX:72913564-72918344 | 4608 | Chrm2 | NM_203491.3 | chr6:36388083-36524774 |
| 4512 | Cetn3 | NM_007684.3 | chr13:81783291-81797157 | 4609 | Chrm3 | NM_033269.4 | chr13:9876612-10360803 |
| 4513 | Cetn4 | NM_145825.2 | chr3:37308626-37312446 | 4610 | Chrm4 | NM_007699.2 | chr2:91922188-91929829 |
| 4514 | Cfb | NM_001142706.1 | chr17:34856373-34862514 | 4611 | Chrm5 | NM_205783.2 | chr2:112479071-112480817 |
| 4515 | Cfc1 | NM_007685.2 | chr1:34535647-34544311 | 4612 | Chrna1 | NM_007389.5 | chr2:73563280-73580338 |
| 4516 | Cfd | NM_001291915.1 | chr10:79890852-79892660 | 4613 | Chrna10 | NM_001081424.1 | chr7:102111265-102116713 |
| 4517 | Cfdp1 | NM_011801.1 | chr8:111768472-111854310 | 4614 | Chrna2 | NM_144803.2 | chr14:66140959-66152948 |
| 4518 | Cfh | NM_009888.3 | chr1:140085854-140183411 | 4615 | Chrna3 | NM_145129.2 | chr9:55011342-55026559 |
| 4519 | Cfhr1 | NM_015780.2 | chr1:139547063-139560222 | 4616 | Chrna4 | NM_015730.5 | chr2:181022310-181039177 |
| 4520 | Cfhr2 | NM_001025575.2 | chr1:139810291-139858699 | 4617 | Chrna5 | NM_176844.4 | chr9:54980879-55007779 |
| 4521 | Cfi | NM_007686.2 | chr3:129836738-129875328 | 4618 | Chrna6 | NM_021369.2 | chr8:27403211-27413944 |
| 4522 | Cfl1 | NM_007687.5 | chr19:5490454-5494031 | 4619 | Chrna7 | NM_007390.3 | chr7:63098691-63212526 |
| 4523 | Cfl2 | NM_007688 | chr12:54858818-54862877 | 4620 | Chrna9 | NM_001081104.1 | chr5:65967123-65977486 |
| 4524 | Cflar | NM_001289704 | chr1:58711490-58759209 | 4621 | Chrnb1 | NM_009601.4 | chr11:69784035-69795937 |
| 4525 | Cfp | NM_008823.4 | chrX:20925453-20931555 | 4622 | Chrnb2 | NM_009602.4 | chr3:89753447-89764632 |
| 4526 | Cftr | NM_021050.2 | chr6:18170686-18322769 | 4623 | Chrnb3 | NM_027454.4 | chr8:27368710-27399730 |
| 4527 | Cga | NM_009889.2 | chr4:34893778-34907374 | 4624 | Chrnb4 | NM_148944.4 | chr9:55028155-55048544 |
| 4528 | Cggbp1 | NM_178647.2 | chr16:64852084-64859491 | 4625 | Chrnd | NM_021600.3 | chr1:87190596-87200070 |
| 4529 | Cgn | NM_001037711.2 | chr3:94760069-94786515 | 4626 | Chrne | NM_009603.1 | chr11:70614882-70619194 |
| 4530 | Cgnl1 | NM_026599.5 | chr9:71626506-71771602 | 4627 | Chrng | NM_009604.3 | chr1:87205810-87211835 |
| 4531 | Cgref1 | NM_001160149.1 | chr5:30933142-30945427 | 4628 | Chst1 | NM_023850.2 | chr2:92599706-92615252 |
| 4532 | Cgrrf1 | NM_026832.3 | chr14:46832243-46854190 | 4629 | Chst10 | NM_145142.2 | chr1:38863872-38898160 |
| 4533 | Ch25h | NM_009890.1 | chr19:34473783-34475135 | 4630 | Chst11 | NM_021439.2 | chr10:82985496-83195891 |
| 4534 | Chac1 | NM_026929.4 | chr2:119351241-119354327 | 4631 | Chst12 | NM_021528.3 | chr5:140505608-140525238 |
| 4535 | Chac2 | NM_001290667.1 | chr11:30976706-30986365 | 4632 | Chst13 | NM_027928.1 | chr6:90308350-90325185 |
| 4536 | Chad | NM_007689.4 | chr11:94565073-94569127 | 4633 | Chst14 | NM_028117.3 | chr2:118926496-118928583 |
| 4537 | Chadl | NM_001164320.1 | chr15:81686166-81697287 | 4634 | Chst15 | NM_029935.5 | chr7:132236254-132317155 |
| 4538 | Chaf1a | NM_013733.3 | chr17:56040415-56068026 | 4635 | Chst2 | NM_018763.2 | chr9:95400925-95407270 |
| 4539 | Chaf1b | NM_028083.4 | chr16:93883900-93906106 | 4636 | Chst3 | NM_016803.3 | chr10:60181527-60219260 |
| 4540 | Champ1 | NM_181854.2 | chr8:13869640-13881639 | 4637 | Chst4 | NM_011998.4 | chr8:110029074-110039334 |
| 4541 | Chat | NM_009891.2 | chr14:32408202-32465909 | 4638 | Chst5 | NM_019950.2 | chr8:111889134-111910199 |
| 4542 | Chchd1 | NM_025366.3 | chr14:20703026-20704425 | 4639 | Chst7 | NM_021715.1 | chrX:20059569-20097520 |
| 4543 | Chchd10 | NM_175329.3 | chr10:75935572-75937734 | 4640 | Chst8 | NM_175140.4 | chr7:34674467-34812711 |
| 4544 | Chchd2 | NM_024166.6 | chr5:129881160-129887470 | 4641 | Chst9 | NM_199055.2 | chr18:15452174-15718046 |
| 4545 | Chchd3 | NM_025336.1 | chr6:32792226-33060152 | 4642 | Chsy1 | NM_001081163.1 | chr7:66109514-66173798 |
| 4546 | Chchd4 | NM_133928.2 | chr6:91464275-91473423 | 4643 | Chsy3 | NM_001081328.1 | chr18:59175339-59411336 |
| 4547 | Chchd5 | NM_025395.3 | chr2:129129699-129134111 | 4644 | Chtf18 | NM_145409.2 | chr17:25719029-25727415 |
| 4548 | Chchd6 | NM_001167736.1 | chr6:89383145-89595652 | 4645 | Chtf8 | NM_145412.3 | chr8:106883862-106893593 |
| 4549 | Chchd7 | NM_001190322.2 | chr4:3938887-3943525 | 4646 | Chtop | NM_023215.6 | chr3:90498538-90509498 |
| 4550 | Chd1 | NM_007690.3 | chr17:15704966-15772612 | 4647 | Chuk | NM_001162410.1 | chr19:44073333-44107477 |
| 4551 | Chd1l | NM_026539.2 | chr3:97560747-97610190 | 4648 | Churc1 | NM_206534.1 | chr12:76765572-76783178 |
| 4552 | Chd2 | NM_001081345.2 | chr7:73426651-73541746 | 4649 | Ciao1 | NM_025296.4 | chr2:127240937-127247816 |
| 4553 | Chd3 | NM_146019.4 | chr11:69343272-69369426 | 4650 | Ciapin1 | NM_134141.4 | chr8:94819817-94838340 |
| 4554 | Chd3os | NR_027827.1 | chr11:69340768-69342647 | 4651 | Ciart | NM_001033302.2 | chr3:95878504-95882228 |
| 4555 | Chd4 | NM_145979.2 | chr6:125096162-125130501 | 4652 | Cib1 | NM_001291275.1 | chr7:80227155-80232805 |
| 4556 | Chd5 | NM_001081376.1 | chr4:152338650-152390194 | 4653 | Cib2 | NM_019686.5 | chr9:54545351-54560079 |
| 4557 | Chd6 | NM_173368.1 | chr2:160946977-161109086 | 4654 | Cib3 | NM_001080812.1 | chr8:72204334-72212837 |
| 4558 | Chd7 | NM_001277149.1 | chr4:8690405-8868449 | 4655 | Cib4 | NM_028483.1 | chr5:30485583-30545836 |

Fig.21 - 25

| | | | | | | | |
|---|---|---|---|---|---|---|---|
| 4656 | Cic | NM_001302811.1 | chr7:25267657-25294159 | 4753 | Clec3a | NM_001007223.3 | chr8:114418033-114428133 |
| 4657 | Cidea | NM_007702.2 | chr18:67343563-67367794 | 4754 | Clec3b | NM_011606.2 | chr9:123150945-123157432 |
| 4658 | Cideb | NM_009894.3 | chr14:55754054-55758424 | 4755 | Clec4a1 | NM_199311.2 | chr6:122921847-122934619 |
| 4659 | Cidec | NM_178373.3 | chr6:113424635-113435755 | 4756 | Clec4a2 | NM_001170332.1 | chr6:123122689-123143999 |
| 4660 | Ciita | NM_001243760.2 | chr16:10480071-10527640 | 4757 | Clec4a3 | NM_001204241.1 | chr6:122952514-122969878 |
| 4661 | Cilp | NM_173385.2 | chr9:65265179-65280605 | 4758 | Clec4a4 | NM_001005860.2 | chr6:122990366-123024421 |
| 4662 | Cilp2 | NM_026818 | chr8:69880365-69887392 | 4759 | Clec4b1 | NM_001190310.1 | chr6:123049961-123071555 |
| 4663 | Cinp | NM_026048.4 | chr12:110872609-110889145 | 4760 | Clec4b2 | NM_001004159.2 | chr6:123173022-123204671 |
| 4664 | Cipc | NM_001289429.1 | chr12:86947040-86965366 | 4761 | Clec4d | NM_001163161.1 | chr6:123262106-123275268 |
| 4665 | Cir1 | NM_025854 | chr2:73283871-73312592 | 4762 | Clec4e | NM_019948.2 | chr6:123281788-123289871 |
| 4666 | Cirbp | NM_007705.2 | chr10:80167840-80171653 | 4763 | Clec4f | NM_016751.3 | chr6:83644541-83656116 |
| 4667 | Cirh1a | NM_011574.2 | chr8:106893639-106923094 | 4764 | Clec4g | NM_029465.3 | chr8:3716070-3720651 |
| 4668 | Cisd1 | NM_134007.4 | chr10:71330493-71344849 | 4765 | Clec4n | NM_001190320.1 | chr6:123229842-123247024 |
| 4669 | Cisd2 | NM_025902.3 | chr3:135406411-135423433 | 4766 | Clec5a | NM_001038604.1 | chr6:40574897-40585805 |
| 4670 | Cisd3 | NM_001085500.2 | chr11:97685951-97688625 | 4767 | Clec7a | NM_020008.3 | chr6:129461590-129472779 |
| 4671 | Cish | NM_009895.3 | chr9:107296688-107301961 | 4768 | Clec9a | NM_001205363.1 | chr6:129408861-129424764 |
| 4672 | Cistr-act | NR_104334.1 | chr15:102746440-102746909 | 4769 | Clgn | NM_009904.2 | chr8:83389890-83426829 |
| 4673 | Cit | NM_007708.3 | chr5:115845655-116006341 | 4770 | Clhc1 | NM_001081099.1 | chr4:134510998-134523922 |
| 4674 | Cited1 | NM_001276466.1 | chrX:102247377-102252181 | 4771 | Clic1 | NM_033444.2 | chr17:35050242-35058719 |
| 4675 | Cited2 | NM_010828.3 | chr10:17723227-17725674 | 4772 | Clic3 | NM_027085.3 | chr2:25456842-25458772 |
| 4676 | Cited4 | NM_019563.2 | chr4:120666562-120667820 | 4773 | Clic4 | NM_013885.2 | chr4:135213969-135272760 |
| 4677 | Ciz1 | NM_001252534.1 | chr2:32363197-32378313 | 4774 | Clic5 | NM_172621.2 | chr17:44188571-44280168 |
| 4678 | CK137956 | NM_001134733.1 | chr4:127927591-127970841 | 4775 | Clic6 | NM_172469.3 | chr16:92498146-92541241 |
| 4679 | Ckap2 | NM_001004140.2 | chr8:22168151-22185819 | 4776 | Clint1 | NM_001045520.3 | chr11:45851963-45910625 |
| 4680 | Ckap2l | NM_181589.3 | chr2:129268209-129297212 | 4777 | Clip1 | NM_001291229.1 | chr5:123577793-123684361 |
| 4681 | Ckap4 | NM_175451.1 | chr10:84526304-84533888 | 4778 | Clip2 | NM_001039162.2 | chr5:134489382-134552434 |
| 4682 | Ckap5 | NM_001165989.1 | chr2:91546321-91620665 | 4779 | Clip3 | NM_001081114.1 | chr7:30291752-30308367 |
| 4683 | Ckb | NM_021273.4 | chr12:111669354-111672338 | 4780 | Clip4 | NM_001271483.1 | chr17:71781298-71864210 |
| 4684 | Cklf | NM_001037841.3 | chr8:104250860-104264936 | 4781 | Clk1 | NM_001042634.2 | chr1:58411987-58424088 |
| 4685 | Ckm | NM_007710.2 | chr7:19411093-19421583 | 4782 | Clk2 | NM_001163432.2 | chr3:89164794-89177087 |
| 4686 | Ckmt1 | NM_009897.2 | chr2:121358640-121363737 | 4783 | Clk3 | NM_007713.4 | chr9:57750710-57765860 |
| 4687 | Ckmt2 | NM_198415.2 | chr13:91853386-91876885 | 4784 | Clk4 | NM_007714.6 | chr11:51263113-51281770 |
| 4688 | Cks1b | NM_016904.1 | chr3:89415471-89418291 | 4785 | Clmn | NM_001040682.1 | chr12:104763113-104865076 |
| 4689 | Cks1brt | NM_001037922.3 | chr8:85151923-85173622 | 4786 | Clmp | NM_133733.4 | chr9:40685963-40784046 |
| 4690 | Cks2 | NM_025415.3 | chr13:51645231-51650662 | 4787 | Cln3 | NM_001146311.1 | chr7:126571399-126584280 |
| 4691 | Clasp1 | NM_001293300.1 | chr1:118389058-118609462 | 4788 | Cln5 | NM_001033242.1 | chr14:103070215-103077630 |
| 4692 | Clasp2 | NM_001081960.1 | chr9:113812585-113919697 | 4789 | Cln6 | NM_001033175.2 | chr9:62838786-62852002 |
| 4693 | Clasrp | NM_016680.5 | chr7:19581037-19604486 | 4790 | Cln8 | NM_012000.3 | chr8:14888535-14901719 |
| 4694 | Clca1 | NM_009899.4 | chr3:144729676-144760977 | 4791 | Clnk | NM_013748.3 | chr5:38706456-38876693 |
| 4695 | Clca2 | NM_030601.3 | chr3:144796558-144819494 | 4792 | Clns1a | NM_023671.2 | chr7:97696656-97720793 |
| 4696 | Clca3 | NM_017474.2 | chr3:145004536-145032776 | 4793 | Clock | NM_001289826.1 | chr5:76209867-76304792 |
| 4697 | Clca4 | NM_139148.2 | chr3:144822623-144849302 | 4794 | Clp1 | NM_133840.2 | chr2:84723121-84727268 |
| 4698 | Clca5 | NM_178697.4 | chr3:145070264-145099041 | 4795 | Clpb | NM_009191.3 | chr7:101663767-101790168 |
| 4699 | Clca6 | NM_207208.3 | chr3:144952485-144975045 | 4796 | Clpp | NM_017393.2 | chr17:56990263-56996371 |
| 4700 | Clcc1 | NM_001177770.1 | chr3:108653989-108678011 | 4797 | Clps | NM_025469.2 | chr17:28558213-28560714 |
| 4701 | Clcf1 | NM_019952.5 | chr19:4214237-4223505 | 4798 | Clpsl2 | NM_001034871.2 | chr17:28549486-28552618 |
| 4702 | Clcn1 | NM_013491.2 | chr6:42286684-42314656 | 4799 | Clptm1 | NM_019649.2 | chr7:19631579-19665030 |
| 4703 | Clcn2 | NM_009900.2 | chr16:20702965-20716636 | 4800 | Clptm1l | NM_146047.2 | chr13:73604001-73620639 |
| 4704 | Clcn3 | NM_007711.3 | chr8:60910388-60983311 | 4801 | Clpx | NM_001044389.2 | chr9:65294259-65330658 |
| 4705 | Clcn4-2 | NM_011334.4 | chr7:7282308-7299557 | 4802 | Clrn1 | NM_153384.3 | chr3:58844027-58885212 |
| 4706 | Clcn5 | NM_001243762.1 | chrX:7158411-7319358 | 4803 | Clrn2 | NM_001163317.1 | chr5:45453750-45464149 |
| 4707 | Clcn6 | NM_011929.3 | chr4:148004258-148038813 | 4804 | Clrn3 | NM_178669.5 | chr7:135511455-135528654 |
| 4708 | Clcn7 | NM_011930.3 | chr17:25133393-25162099 | 4805 | Clspn | NM_175554.4 | chr4:126556979-126593903 |
| 4709 | Clcnka | NM_001146307.1 | chr4:141384610-141398099 | 4806 | Clstn1 | NM_001290989.1 | chr4:149586467-149648899 |
| 4710 | Clcnkb | NM_019701.2 | chr4:141404356-141415988 | 4807 | Clstn2 | NM_022319.2 | chr9:97444394-98033167 |
| 4711 | Cldn1 | NM_016674.4 | chr16:26356645-26371839 | 4808 | Clstn3 | NM_153508.4 | chr6:124430755-124464784 |
| 4712 | Cldn10 | NM_001160096.1 | chr14:118787870-118874525 | 4809 | Clta | NM_001080384.1 | chr4:44012642-44032846 |
| 4713 | Cldn11 | NM_008770.3 | chr3:31149919-31164326 | 4810 | Cltb | NM_028870.3 | chr13:54592938-54611272 |
| 4714 | Cldn12 | NM_001193659.1 | chr5:5505014-5514849 | 4811 | Cltc | NM_001003908.1 | chr11:86694652-86757492 |
| 4715 | Cldn13 | NM_020504.4 | chr5:134914249-134915530 | 4812 | Clu | NM_013492.2 | chr14:65968482-65981545 |
| 4716 | Cldn14 | NM_001165925.1 | chr16:93919030-93929567 | 4813 | Cluap1 | NM_029738.2 | chr16:3909008-3941147 |
| 4717 | Cldn15 | NM_021719.4 | chr5:136967868-136975845 | 4814 | Cluh | NM_001081158.2 | chr11:74649494-74670847 |
| 4718 | Cldn16 | NM_053241.5 | chr16:26463134-26482764 | 4815 | Clvs1 | NM_028940.2 | chr4:9269316-9451691 |
| 4719 | Cldn17 | NM_181490.3 | chr16:88505806-88506978 | 4816 | Clvs2 | NM_175448.3 | chr10:33512333-33624600 |
| 4720 | Cldn18 | NM_001194921.1 | chr9:99690796-99717267 | 4817 | Clybl | NM_029556.3 | chr14:122181693-122402234 |
| 4721 | Cldn19 | NM_001038590.1 | chr4:119255468-119259855 | 4818 | Cma1 | NM_010780.2 | chr14:55941450-55944661 |
| 4722 | Cldn2 | NM_016675.4 | chrX:139800807-139811388 | 4819 | Cma2 | NM_001024714.2 | chr14:55971427-55973995 |
| 4723 | Cldn20 | NM_001101560.1 | chr17:3532553-3533213 | 4820 | Cmah | NM_001111110.2 | chr13:24327403-24477289 |
| 4724 | Cldn22 | NM_029383.1 | chr8:47824481-47825475 | 4821 | Cmas | NM_009908.2 | chr6:142756685-142775714 |
| 4725 | Cldn23 | NM_027998.4 | chr8:35824708-35826559 | 4822 | Cmbl | NM_181588.3 | chr15:31568911-31590119 |
| 4726 | Cldn24 | NM_001111318.1 | chr8:47822142-47822805 | 4823 | Cmc1 | NM_026442.3 | chr9:118064526-118150196 |
| 4727 | Cldn25 | NM_001252450.1 | chr16:58727909-58734247 | 4824 | Cmc2 | NM_026844.3 | chr8:116888684-116921436 |
| 4728 | Cldn26 | NM_029070.2 | chr16:8409275-8425136 | 4825 | Cmip | NM_001163262.1 | chr8:117257018-117461505 |
| 4729 | Cldn3 | NM_009902.4 | chr5:134986213-134987476 | 4826 | Cmklr1 | NM_008153.3 | chr5:113612354-113650390 |
| 4730 | Cldn4 | NM_009903.2 | chr5:134945123-134946934 | 4827 | Cml1 | NM_023160.2 | chr6:85910153-85915677 |
| 4731 | Cldn5 | NM_013805.4 | chr16:18776846-18778262 | 4828 | Cml2 | NM_053096.3 | chr6:85865421-85869137 |
| 4732 | Cldn6 | NM_018777.4 | chr17:23679364-23682446 | 4829 | Cml3 | NM_001037842.3 | chr6:85760630-85765744 |
| 4733 | Cldn7 | NM_001193619.1 | chr11:69964778-69967886 | 4830 | Cml5 | NM_023493.2 | chr6:85817217-85820972 |
| 4734 | Cldn8 | NM_018778.3 | chr16:88560825-88563183 | 4831 | Cmpk1 | NM_025647.3 | chr4:114960612-114987228 |
| 4735 | Cldn9 | NM_020293.3 | chr17:23682583-23684026 | 4832 | Cmpk2 | NM_020557.4 | chr12:26469214-26479837 |
| 4736 | Cldnd2 | NM_028849.1 | chr7:43440815-43443320 | 4833 | Cmss1 | NM_025599.3 | chr16:57301999-57606867 |
| 4737 | Clec10a | NM_001204252.1 | chr11:70166622-70170836 | 4834 | Cmtm1 | NM_181990.2 | chr8:104293541-104310145 |
| 4738 | Clec11a | NM_009131.3 | chr7:44303765-44306959 | 4835 | Cmtm2a | NM_027022.2 | chr8:104281041-104293181 |
| 4739 | Clec12a | NM_177686.4 | chr6:129350243-129365303 | 4836 | Cmtm2b | NM_028524.1 | chr8:104322226-104330764 |
| 4740 | Clec12b | NM_001204223.1 | chr6:129375512-129385874 | 4837 | Cmtm3 | NM_024217 | chr8:104340593-104347672 |
| 4741 | Clec14a | NM_025809.5 | chr12:58264719-58269258 | 4838 | Cmtm4 | NM_153582.5 | chr8:104348192-104395807 |
| 4742 | Clec16a | NM_001204229.1 | chr16:10545338-10744878 | 4839 | Cmtm5 | NM_026066.2 | chr14:54936469-54939277 |
| 4743 | Clec18a | NM_181549.3 | chr8:111069496-111081708 | 4840 | Cmtm6 | NM_026036.3 | chr9:114731202-114749343 |
| 4744 | Clec1a | NM_175526.3 | chr6:129426683-129452000 | 4841 | Cmtm7 | NM_001252479.1 | chr9:114756835-114781993 |
| 4745 | Clec1b | NM_001204239.1 | chr6:129397296-129405413 | 4842 | Cmtm8 | NM_027294.1 | chr9:114789344-114844152 |
| 4746 | Clec2d | NM_053109.3 | chr6:129180614-129186535 | 4843 | Cmtr1 | NM_028791.6 | chr17:29660594-29705979 |
| 4747 | Clec2e | NM_153506.4 | chr6:129091815-129100903 | 4844 | Cmtr2 | NM_146215.4 | chr8:110217959-110224489 |
| 4748 | Clec2f | NM_001277202.1 | chr6:129014111-129020527 | 4845 | Cmya5 | NM_023821.3 | chr13:93040714-93144724 |
| 4749 | Clec2g | NM_001168223.1 | chr6:128971159-128984707 | 4846 | Cnbd2 | NM_027585.2 | chr2:156312472-156375638 |
| 4750 | Clec2h | NM_053165.5 | chr6:128662384-128677374 | 4847 | Cnbp | NM_001109745.1 | chr6:87842614-87851106 |
| 4751 | Clec2i | NM_001289706.1 | chr6:128887586-128898167 | 4848 | Cndp1 | NM_177450.4 | chr18:84610508-84650095 |
| 4752 | Clec2l | NM_001101507.1 | chr6:38663068-38680865 | 4849 | Cndp2 | NM_001289531.1 | chr18:84667464-84682059 |

Fig.21 - 26

| | | | | | | | |
|---|---|---|---|---|---|---|---|
| 4850 | Cnep1r1 | NM_029074.3 | chr8:88118758-88135197 | 4947 | Col26a1 | NM_024474.2 | chr5:136741763-136883107 |
| 4851 | Cnfn | NM_001081375.1 | chr7:25367615-25369724 | 4948 | Col27a1 | NM_025685.3 | chr4:63215411-63334990 |
| 4852 | Cnga1 | NM_007723.2 | chr5:72603696-72642752 | 4949 | Col28a1 | NM_001037865.1 | chr6:7997807-8192617 |
| 4853 | Cnga2 | NM_007724.3 | chrX:71991848-72010218 | 4950 | Col2a1 | NM_001113515.2 | chr15:97975601-98004724 |
| 4854 | Cnga3 | NM_001282010.1 | chr1:37218277-37263384 | 4951 | Col3a1 | NM_009930.2 | chr1:45311537-45349706 |
| 4855 | Cnga4 | NM_001033317.3 | chr7:105404567-105408738 | 4952 | Col4a1 | NM_009931.2 | chr8:11198422-11312826 |
| 4856 | Cngb1 | NM_001195413.1 | chr8:95239042-95306585 | 4953 | Col4a2 | NM_009932.4 | chr8:11312804-11449287 |
| 4857 | Cngb3 | NM_013927.2 | chr4:19280849-19510623 | 4954 | Col4a3 | NM_007734.2 | chr1:82586920-82722059 |
| 4858 | Cnih1 | NM_009919.2 | chr14:46775567-46788357 | 4955 | Col4a3bp | NM_001164222.1 | chr13:96542734-96640167 |
| 4859 | Cnih2 | NM_009920.4 | chr19:5092867-5098521 | 4956 | Col4a4 | NM_007735.2 | chr1:82450722-82586849 |
| 4860 | Cnih3 | NM_001160211.1 | chr1:181352627-181460641 | 4957 | Col4a5 | NM_001163155.1 | chrX:141475418-141689235 |
| 4861 | Cnih4 | NM_030131.3 | chr1:181150930-181168994 | 4958 | Col4a6 | NM_053185.2 | chrX:141165402-141474076 |
| 4862 | Cnksr1 | NM_001081047.1 | chr4:134228041-134238399 | 4959 | Col5a1 | NM_015734.2 | chr2:27886424-28039510 |
| 4863 | Cnksr2 | NM_177751.3 | chrX:157821572-158043197 | 4960 | Col5a2 | NM_007737.2 | chr1:45374330-45503282 |
| 4864 | Cnksr3 | NM_172546.2 | chr10:7119061-7212237 | 4961 | Col5a3 | NM_016919.2 | chr9:20770049-20815034 |
| 4865 | Cnn1 | NM_009922.4 | chr9:22099252-22109221 | 4962 | Col6a1 | NM_009933.4 | chr10:76708791-76726044 |
| 4866 | Cnn2 | NM_007725.2 | chr10:79988599-79995400 | 4963 | Col6a2 | NM_146007.2 | chr10:76595755-76623404 |
| 4867 | Cnn3 | NM_028044.2 | chr3:121426540-121458205 | 4964 | Col6a3 | NM_001243008.1 | chr1:90766859-90843971 |
| 4868 | Cnnm1 | NM_031396.2 | chr19:43440435-43497213 | 4965 | Col6a4 | NM_026763.2 | chr9:105990317-106096691 |
| 4869 | Cnnm2 | NM_001102471.1 | chr19:46761608-46878580 | 4966 | Col6a5 | NM_001167923.1 | chr9:105856069-105960643 |
| 4870 | Cnnm3 | NM_001039551.1 | chr1:36511875-36528237 | 4967 | Col6a6 | NM_001102607.1 | chr9:105689416-105828085 |
| 4871 | Cnnm4 | NM_033570.2 | chr1:36471596-36508776 | 4968 | Col7a1 | NM_007738.3 | chr9:108953744-108984875 |
| 4872 | Cnot1 | NM_001205226.1 | chr8:95719450-95807462 | 4969 | Col8a1 | NM_007739.2 | chr16:57624255-57754737 |
| 4873 | Cnot10 | NM_153585.5 | chr9:114585873-114640200 | 4970 | Col8a2 | NM_199473.2 | chr4:126286793-126314330 |
| 4874 | Cnot11 | NM_028043.2 | chr1:39535801-39546877 | 4971 | Col9a1 | NM_001290691.1 | chr1:24195202-24252738 |
| 4875 | Cnot2 | NM_001037846.3 | chr10:116485160-116581511 | 4972 | Col9a2 | NM_007741.2 | chr4:121039565-121055325 |
| 4876 | Cnot3 | NM_146176.3 | chr7:3645268-3661109 | 4973 | Col9a3 | NM_009936.2 | chr2:180598221-180622185 |
| 4877 | Cnot4 | NM_001164411.1 | chr6:35042706-35133737 | 4974 | Colec10 | NM_173422.3 | chr15:54410773-54466358 |
| 4878 | Cnot6 | NM_001290741.1 | chr11:49671496-49702654 | 4975 | Colec11 | NM_027866.2 | chr12:28594171-28623330 |
| 4879 | Cnot6l | NM_001285511.1 | chr5:96070332-96161547 | 4976 | Colec12 | NM_130449.2 | chr18:9707647-9877995 |
| 4880 | Cnot7 | NM_001271542.1 | chr8:40492537-40515847 | 4977 | Colgalt2 | NM_177756.4 | chr1:152399866-152510695 |
| 4881 | Cnot8 | NM_026949.3 | chr11:58104152-58118594 | 4978 | Colq | NM_009937.2 | chr14:31523083-31577383 |
| 4882 | Cnp | NM_001146318.1 | chr11:100575890-100581739 | 4979 | Commd1 | NM_144514.2 | chr11:22899727-22982284 |
| 4883 | Cnppd1 | NM_026977.2 | chr1:75135214-75142368 | 4980 | Commd10 | NM_178377.4 | chr18:46958875-47087994 |
| 4884 | Cnpy1 | NM_001310511.1 | chr5:28200826-28237873 | 4981 | Commd2 | NM_175095.4 | chr3:57644348-57651684 |
| 4885 | Cnpy2 | NM_019953.1 | chr10:128322458-128327187 | 4982 | Commd3 | NM_147778.3 | chr2:18672461-18676216 |
| 4886 | Cnpy3 | NM_028065.4 | chr17:46735710-46752214 | 4983 | Commd4 | NM_025417.2 | chr9:57155040-57158299 |
| 4887 | Cnpy4 | NM_178612.4 | chr5:138187534-138193894 | 4984 | Commd5 | NM_025536.2 | chr15:76899940-76901297 |
| 4888 | Cnr1 | NM_007726.3 | chr4:33924631-33948831 | 4985 | Commd6 | NM_001033132.3 | chr14:101633765-101640471 |
| 4889 | Cnr2 | NM_009924.4 | chr4:135895188-135920219 | 4986 | Commd7 | NM_001195390.1 | chr2:153616929-153632781 |
| 4890 | Cnrip1 | NM_029861.2 | chr11:17051933-17079372 | 4987 | Commd8 | NM_178599.4 | chr5:72156999-72168183 |
| 4891 | Cnst | NM_146105.3 | chr1:179546528-179627473 | 4988 | Commd9 | NM_029635.3 | chr2:101886261-101901639 |
| 4892 | Cntd1 | NM_026562.2 | chr11:101279202-101288701 | 4989 | Comp | NM_016685.2 | chr8:70373547-70382066 |
| 4893 | Cntf | NM_170786.2 | chr19:12763527-12765632 | 4990 | Comt | NM_001111062.1 | chr16:18406881-18426716 |
| 4894 | Cntfr | NM_001136056.2 | chr4:41657497-41695445 | 4991 | Comtd1 | NM_026965.2 | chr14:21845860-21848910 |
| 4895 | Cntln | NM_175275.4 | chr4:84884308-85131921 | 4992 | Copa | NM_009938.4 | chr1:172082528-172122332 |
| 4896 | Cntn1 | NM_001159647.1 | chr15:92161356-92341967 | 4993 | Copb1 | NM_033370.3 | chr7:114215558-114254680 |
| 4897 | Cntn2 | NM_177129.5 | chr1:132509424-132542940 | 4994 | Copb2 | NM_015827.2 | chr9:98563730-98588375 |
| 4898 | Cntn3 | NM_008779.2 | chr6:102163305-102464667 | 4995 | Cope | NM_021538.1 | chr8:70302784-70312990 |
| 4899 | Cntn4 | NM_001109749.1 | chr6:105677744-106699305 | 4996 | Copg1 | NM_017477.2 | chr6:87887939-87913594 |
| 4900 | Cntn5 | NM_001033359.2 | chr9:9660890-10904726 | 4997 | Copg2 | NM_017478.3 | chr6:30747553-30896794 |
| 4901 | Cntn6 | NM_017383.3 | chr6:104493043-104863405 | 4998 | Coprs | NM_025556.3 | chr8:13884787-13890271 |
| 4902 | Cntnap1 | NM_016782.2 | chr11:101176116-101190720 | 4999 | Cops2 | NM_001285507.1 | chr2:125830301-125859082 |
| 4903 | Cntnap2 | NM_001004357.2 | chr6:45060060-47301371 | 5000 | Cops3 | NM_011991.1 | chr11:59817803-59839767 |
| 4904 | Cntnap3 | NM_001081129.1 | chr13:64737590-64903888 | 5001 | Cops4 | NM_012001.2 | chr5:100518308-100547802 |
| 4905 | Cntnap4 | NM_130457.2 | chr8:112570042-112882707 | 5002 | Cops5 | NM_001277101.1 | chr1:10024599-10038159 |
| 4906 | Cntnap5a | NM_001077425.1 | chr1:115685136-116580674 | 5003 | Cops6 | NM_012002.3 | chr5:138161101-138163984 |
| 4907 | Cntnap5b | NM_172851.2 | chr1:99772764-100485942 | 5004 | Cops7a | NM_001164089.1 | chr6:124958410-124965529 |
| 4908 | Cntnap5c | NM_001081653.1 | chr17:57769569-58410342 | 5005 | Cops7b | NM_172974.2 | chr1:86587099-86606500 |
| 4909 | Cntrl | NM_001290635.1 | chr2:35132233-35147671 | 5006 | Cops8 | NM_133805.3 | chr1:90603424-90613341 |
| 4910 | Cntrob | NM_172560.3 | chr11:69299495-69323873 | 5007 | Copz1 | NM_019817.1 | chr15:103272917-103299864 |
| 4911 | Coa3 | NM_026618.2 | chr11:101277969-101278948 | 5008 | Copz2 | NM_019877.2 | chr11:96849875-96861202 |
| 4912 | Coa4 | NM_183270.2 | chr7:100537099-100539812 | 5009 | Coq10a | NM_001081040.1 | chr10:128363096-128370037 |
| 4913 | Coa5 | NM_198006.4 | chr1:37417084-37430103 | 5010 | Coq10b | NM_001039710.1 | chr1:55052769-55072702 |
| 4914 | Coa6 | NM_174987.4 | chr8:126422500-126425435 | 5011 | Coq2 | NM_027978.2 | chr5:100654725-100674256 |
| 4915 | Coa7 | NM_027250.4 | chr4:108328151-108340718 | 5012 | Coq3 | NM_172687.1 | chr4:21879674-21912126 |
| 4916 | Coasy | NM_001305982.1 | chr11:101082562-101086619 | 5013 | Coq4 | NM_178693.4 | chr2:29788262-29797743 |
| 4917 | Cobl | NM_001282993.1 | chr11:12236675-12464960 | 5014 | Coq5 | NM_026504.2 | chr5:115279701-115296972 |
| 4918 | Cobll1 | NM_027225.1 | chr2:65088338-65238626 | 5015 | Coq6 | NM_172582.3 | chr12:84361967-84373796 |
| 4919 | Coch | NM_001198835.1 | chr12:51593340-51605773 | 5016 | Coq7 | NM_009940.3 | chr7:118525061-118533356 |
| 4920 | Cog1 | NM_013581.3 | chr11:113649528-113662401 | 5017 | Coq9 | NM_026452.2 | chr8:94838416-94854895 |
| 4921 | Cog2 | NM_029746.3 | chr8:124520766-124552007 | 5018 | Corin | NM_001122756.1 | chr5:72300024-72504540 |
| 4922 | Cog3 | NM_177381.3 | chr14:75702350-75754493 | 5019 | Coro1a | NM_009898.3 | chr7:126699773-126704816 |
| 4923 | Cog4 | NM_133973.2 | chr8:110847023-110882234 | 5020 | Coro1b | NM_011778.1 | chr19:4148662-4154035 |
| 4924 | Cog5 | NM_001163126.1 | chr12:31654868-31937630 | 5021 | Coro1c | NM_011779.3 | chr5:113842438-113908706 |
| 4925 | Cog6 | NM_026225.3 | chr3:52982122-53017223 | 5022 | Coro2a | NM_001164804.1 | chr4:46536936-46601929 |
| 4926 | Cog7 | NM_001033318.3 | chr7:121922838-121981693 | 5023 | Coro2b | NM_175484.3 | chr9:62419491-62537044 |
| 4927 | Cog8 | NM_139229.4 | chr8:107048708-107056737 | 5024 | Coro6 | NM_139128.1 | chr11:77463912-77469501 |
| 4928 | Coil | NM_016706.2 | chr11:88973934-88991613 | 5025 | Coro7 | NM_030205.4 | chr16:4626883-4679720 |
| 4929 | Col10a1 | NM_009925.4 | chr10:34389980-34397085 | 5026 | Cort | NM_007745.3 | chr4:149125190-149126741 |
| 4930 | Col11a1 | NM_007729.2 | chr3:114030539-114220326 | 5027 | Cotl1 | NM_028071.3 | chr8:119809213-119840579 |
| 4931 | Col11a2 | NM_009926.1 | chr17:34039436-34066242 | 5028 | Cox10 | NM_178379.3 | chr11:63962626-64079472 |
| 4932 | Col12a1 | NM_001290308.1 | chr9:79598986-79718722 | 5029 | Cox11 | NM_199008.2 | chr11:90638183-90645977 |
| 4933 | Col13a1 | NM_007731.3 | chr10:61838233-61979108 | 5030 | Cox14 | NM_183256.3 | chr15:99725617-99728136 |
| 4934 | Col14a1 | NM_181277.3 | chr15:55307749-55520803 | 5031 | Cox15 | NM_144874.4 | chr19:43733253-43753000 |
| 4935 | Col15a1 | NM_009928.3 | chr4:47208011-47313165 | 5032 | Cox16 | NM_025461.6 | chr12:81470602-81485137 |
| 4936 | Col16a1 | NM_028266.5 | chr4:130047839-130099277 | 5033 | Cox17 | NM_001017429.2 | chr16:38346998-38352763 |
| 4937 | Col17a1 | NM_001290825.1 | chr19:47646340-47692042 | 5034 | Cox18 | NM_001033310.3 | chr5:90214724-90223996 |
| 4938 | Col18a1 | NM_001109991.1 | chr10:77052178-77113705 | 5035 | Cox19 | NM_197980.1 | chr5:139337821-139345166 |
| 4939 | Col19a1 | NM_007733.2 | chr1:24257682-24587437 | 5036 | Cox20 | NM_025511.2 | chr1:178319152-178322693 |
| 4940 | Col1a1 | NM_007742.4 | chr11:94936223-94953042 | 5037 | Cox4i1 | NM_009941.3 | chr8:120668224-120674209 |
| 4941 | Col1a2 | NM_007743.2 | chr6:4505696-4541543 | 5038 | Cox4i2 | NM_053091.2 | chr2:152754172-152765037 |
| 4942 | Col20a1 | NM_028518.1 | chr2:180986534-181017540 | 5039 | Cox5a | NM_007747 | chr9:57521231-57532426 |
| 4943 | Col22a1 | NM_027174.1 | chr15:71798475-72034227 | 5040 | Cox5b | NM_009942.2 | chr1:36691486-36693388 |
| 4944 | Col23a1 | NM_153393.2 | chr11:51289919-51583925 | 5041 | Cox6a1 | NM_007748.3 | chr5:115345653-115348955 |
| 4945 | Col24a1 | NM_027770.2 | chr3:145292471-145552005 | 5042 | Cox6a2 | NM_009943.2 | chr7:128205434-128206366 |
| 4946 | Col25a1 | NM_001244952.1 | chr3:130180844-130599883 | 5043 | Cox6b1 | NM_025628.2 | chr7:30616973-30626151 |

Fig.21 - 27

| | | | |
|---|---|---|---|
| 5044 | Cox6b2 | NM_001289848.1 | chr7:4751791-4753094 |
| 5045 | Cox6c | NM_053071.2 | chr15:35931975-35938246 |
| 5046 | Cox7a1 | NM_009944.3 | chr7:30184170-30186030 |
| 5047 | Cox7a2 | NM_009945.3 | chr9:79755240-79759853 |
| 5048 | Cox7a2l | NM_001159529.1 | chr17:83501916-83514333 |
| 5049 | Cox7b | NM_025379.2 | chrX:106015699-106022450 |
| 5050 | Cox7b2 | NM_030052.3 | chr5:71442822-71548205 |
| 5051 | Cox7c | NM_007749.3 | chr13:86044797-86046795 |
| 5052 | Cox8a | NM_007750.2 | chr19:7215157-7217616 |
| 5053 | Cox8b | NM_007751.3 | chr7:140898941-140900446 |
| 5054 | Cox8c | NM_001039049.1 | chr12:102899305-102900534 |
| 5055 | Cp | NM_001276248.1 | chr3:19957053-20007609 |
| 5056 | Cpa1 | NM_025350.3 | chr6:30639220-30645361 |
| 5057 | Cpa2 | NM_001024698.2 | chr6:30541641-30564473 |
| 5058 | Cpa3 | NM_007753.2 | chr3:20215615-20242181 |
| 5059 | Cpa4 | NM_027926.2 | chr6:30568375-30591747 |
| 5060 | Cpa5 | NM_144537.3 | chr6:30611009-30631521 |
| 5061 | Cpa6 | NM_001289497.1 | chr1:10324718-10719945 |
| 5062 | Cpb1 | NM_029706.1 | chr3:20249483-20275729 |
| 5063 | Cpb2 | NM_019775.3 | chr14:75242286-75283553 |
| 5064 | Cpd | NM_007754.2 | chr11:76777207-76847008 |
| 5065 | Cpe | NM_013494.3 | chr8:64592550-64693040 |
| 5066 | Cpeb1 | NM_001252525.1 | chr7:81347025-81454758 |
| 5067 | Cpeb2 | NM_001177379.1 | chr5:43233462-43289724 |
| 5068 | Cpeb3 | NM_001290826.1 | chr19:37021290-37176063 |
| 5069 | Cpeb4 | NM_001290676.1 | chr11:31870939-31935635 |
| 5070 | Cped1 | NM_001081351.1 | chr6:21985909-22255606 |
| 5071 | Cphx1 | NM_175342.3 | chr14:25942543-26235735 |
| 5072 | Cphx2 | NM_001270506.1 | chr14:25942539-26235740 |
| 5073 | Cplx1 | NM_007756.3 | chr5:108518553-108550027 |
| 5074 | Cplx2 | NM_009946.3 | chr13:54371348-54383917 |
| 5075 | Cplx3 | NM_146223.3 | chr9:57599991-57606281 |
| 5076 | Cplx4 | NM_145493.1 | chr18:65955721-65970178 |
| 5077 | Cpm | NM_027468.1 | chr10:117629499-117687352 |
| 5078 | Cpn1 | NM_030703.2 | chr19:43956307-43986520 |
| 5079 | Cpn2 | NM_027904.3 | chr16:30256378-30267532 |
| 5080 | Cpne1 | NM_170588.3 | chr2:156071840-156111965 |
| 5081 | Cpne2 | NM_153507.2 | chr8:94533027-94570529 |
| 5082 | Cpne3 | NM_027769.2 | chr4:19519251-19570104 |
| 5083 | Cpne4 | NM_028719.1 | chr9:104569787-105034544 |
| 5084 | Cpne5 | NM_153166.2 | chr17:29156520-29237790 |
| 5085 | Cpne6 | NM_001136057.2 | chr14:55510447-55517431 |
| 5086 | Cpne7 | NM_170684.2 | chr8:123117373-123135185 |
| 5087 | Cpne8 | NM_001033851.1 | chr15:90643322-90679388 |
| 5088 | Cpne9 | NM_170673.3 | chr6:113282306-113305571 |
| 5089 | Cpox | NM_007757.2 | chr16:58670207-58680389 |
| 5090 | Cpped1 | NM_146067.3 | chr16:11803720-11909423 |
| 5091 | Cpq | NM_018755.2 | chr15:33083128-33594552 |
| 5092 | Cps1 | NM_001080809.2 | chr1:67123026-67231267 |
| 5093 | Cpsf1 | NM_001164173.1 | chr15:76595807-76607591 |
| 5094 | Cpsf2 | NM_016856.3 | chr12:101975973-102005993 |
| 5095 | Cpsf3 | NM_018813.3 | chr12:21286296-21315056 |
| 5096 | Cpsf3l | NM_028020.3 | chr4:155869566-155889103 |
| 5097 | Cpsf4 | NM_001291248.1 | chr5:145167212-145182041 |
| 5098 | Cpsf4l | NM_001164532.1 | chr11:113698170-113709526 |
| 5099 | Cpsf6 | NM_001013391.2 | chr10:117344667-117376973 |
| 5100 | Cpsf7 | NM_001164272.1 | chr19:10525243-10547735 |
| 5101 | Cpt1a | NM_013495.2 | chr19:3323300-3385733 |
| 5102 | Cpt1b | NM_009948.2 | chr15:89416404-89425862 |
| 5103 | Cpt1c | NM_001252470.1 | chr7:44959371-44974851 |
| 5104 | Cpt2 | NM_009949.2 | chr4:107903981-107923589 |
| 5105 | Cpvl | NM_001289713.1 | chr6:53873278-53978673 |
| 5106 | Cpxcr1 | NM_001033471.3 | chrX:116448943-116478783 |
| 5107 | Cpxm1 | NM_019696.2 | chr2:130390774-130397629 |
| 5108 | Cpxm2 | NM_018867.5 | chr7:132042809-132154741 |
| 5109 | Cpz | NM_153107.2 | chr5:35502217-35525626 |
| 5110 | Cr1l | NM_013499.2 | chr1:195103787-195131570 |
| 5111 | Cr2 | NM_007758.2 | chr1:195136810-195176715 |
| 5112 | Crabp1 | NM_001284507.1 | chr9:54764747-54773110 |
| 5113 | Crabp2 | NM_007759.2 | chr3:87948692-87953372 |
| 5114 | Cradd | NM_009950.2 | chr10:95174745-95324097 |
| 5115 | Cramp1l | NM_020608.3 | chr17:24961225-25015230 |
| 5116 | Crat | NM_007760.3 | chr2:30400475-30415748 |
| 5117 | Crb1 | NM_133239.2 | chr1:139198253-139377076 |
| 5118 | Crb2 | NM_001163566.1 | chr2:37776248-37799103 |
| 5119 | Crb3 | NM_177638.4 | chr17:57062276-57065914 |
| 5120 | Crbn | NM_021449.3 | chr6:106778244-106800087 |
| 5121 | Crcp | NM_007761.2 | chr5:130029305-130060783 |
| 5122 | Crct1 | NM_028798.3 | chr3:93014204-93015686 |
| 5123 | Creb1 | NM_001037726.1 | chr1:64532803-64604548 |
| 5124 | Creb3 | NM_013497.3 | chr4:43562633-43567060 |
| 5125 | Creb3l1 | NM_011957.2 | chr2:91982327-92024170 |
| 5126 | Creb3l2 | NM_178661.4 | chr6:37331020-37442148 |
| 5127 | Creb3l3 | NM_145365.3 | chr10:81084332-81098872 |
| 5128 | Creb3l4 | NM_030080.3 | chr3:90237497-90243512 |
| 5129 | Creb5 | NM_172728.2 | chr6:53573373-53695832 |
| 5130 | Crebbp | NM_001025432.1 | chr16:4084047-4213404 |
| 5131 | Crebl2 | NM_177687.3 | chr6:134830198-134857883 |
| 5132 | Crebrf | NM_029870.2 | chr17:26715649-26776626 |
| 5133 | Crebzf | NM_145151.3 | chr7:90442780-90448043 |
| 5134 | Creg1 | NM_011804.2 | chr1:165763779-165775304 |
| 5135 | Creg2 | NM_170597.4 | chr1:39618405-39651182 |
| 5136 | Creld1 | NM_133930.1 | chr6:113483568-113493338 |
| 5137 | Creld2 | NM_029720.2 | chr15:88819645-88826681 |
| 5138 | Crem | NM_001110850.2 | chr18:3266353-3327591 |
| 5139 | Crh | NM_205769.3 | chr3:19693400-19695396 |
| 5140 | Crhbp | NM_198408.3 | chr13:95431375-95444831 |
| 5141 | Crhr1 | NM_007762.5 | chr11:104132780-104175523 |
| 5142 | Crhr2 | NM_001288618.1 | chr6:55090048-55133016 |
| 5143 | Crim1 | NM_015800.3 | chr17:78200247-78376592 |
| 5144 | Crip1 | NM_007763.3 | chr12:113152011-113153879 |
| 5145 | Crip2 | NM_024223.2 | chr12:113140235-113145506 |
| 5146 | Crip3 | NM_053250.2 | chr17:46428941-46431771 |
| 5147 | Cript | NM_019936.3 | chr17:87025560-87035808 |
| 5148 | Crisp1 | NM_009638.3 | chr17:40293757-40319207 |
| 5149 | Crisp2 | NM_001204071.1 | chr17:40764733-40794146 |
| 5150 | Crisp3 | NM_009639.2 | chr17:40221776-40242258 |
| 5151 | Crisp4 | NM_030033.1 | chr1:18115190-18137051 |
| 5152 | Crispld1 | NM_031402.2 | chr1:17727416-17766207 |
| 5153 | Crispld2 | NM_030209.4 | chr8:119992437-120052794 |
| 5154 | Crk | NM_001277219.1 | chr11:75679258-75708428 |
| 5155 | Crkl | NM_001277231.1 | chr16:17451984-17487440 |
| 5156 | Crlf1 | NM_018827.2 | chr8:70493155-70504081 |
| 5157 | Crlf2 | NM_001164735.1 | chr5:109554708-109558993 |
| 5158 | Crlf3 | NM_001277106.1 | chr11:80046492-80080991 |
| 5159 | Crls1 | NM_001024385.1 | chr2:132846665-132866768 |
| 5160 | Crmp1 | NM_001136058.2 | chr5:37242057-37292163 |
| 5161 | Crnde | NR_033641.3 | chr8:92326030-92356120 |
| 5162 | Crnkl1 | NM_025820.3 | chr2:145917481-145934700 |
| 5163 | Crnn | NM_001081200.1 | chr3:93144786-93149471 |
| 5164 | Crocc | NM_001145958.1 | chr4:141016636-141060545 |
| 5165 | Crot | NM_023733.3 | chr5:8966047-8997146 |
| 5166 | Crp | NM_007768.4 | chr1:172698055-172699966 |
| 5167 | Crtac1 | NM_145123.4 | chr19:42283036-42431783 |
| 5168 | Crtam | NM_001281954.1 | chr9:40972797-41004628 |
| 5169 | Crtap | NM_019922.2 | chr9:114375130-114390712 |
| 5170 | Crtc1 | NM_001004062.2 | chr8:70382357-70439573 |
| 5171 | Crtc2 | NM_028881.2 | chr3:90254280-90264125 |
| 5172 | Crtc3 | NM_173863.2 | chr7:80586631-80688877 |
| 5173 | Crx | NM_001113330.1 | chr7:15865946-15872385 |
| 5174 | Crxos | NM_001033638.2 | chr7:15896123-15904030 |
| 5175 | Cry1 | NM_007771.3 | chr10:85131699-85185054 |
| 5176 | Cry2 | NM_009963.4 | chr2:92403646-92434069 |
| 5177 | Cryaa | NM_001278569.1 | chr17:31677930-31681730 |
| 5178 | Cryab | NM_001289782.1 | chr9:50751324-50756635 |
| 5179 | Cryba1 | NM_009965.3 | chr11:77718613-77725293 |
| 5180 | Cryba2 | NM_021541.3 | chr1:74889933-74893143 |
| 5181 | Cryba4 | NM_021351.2 | chr5:112246491-112252518 |
| 5182 | Crybb1 | NM_023695.3 | chr5:112255819-112269585 |
| 5183 | Crybb2 | NM_007773.4 | chr5:113058257-113070117 |
| 5184 | Crybb3 | NM_001159650.1 | chr5:113075838-113081584 |
| 5185 | Crybg3 | NM_174848.3 | chr16:59490774-59555752 |
| 5186 | Cryga | NM_007774.3 | chr1:65100388-65103363 |
| 5187 | Crygb | NM_144761.2 | chr1:65080221-65082290 |
| 5188 | Crygc | NM_001082573.2 | chr1:65071524-65073532 |
| 5189 | Crygd | NM_007776.2 | chr1:65061837-65063440 |
| 5190 | Cryge | NM_007777.3 | chr1:65048556-65051163 |
| 5191 | Crygf | NM_027010.2 | chr1:65926521-65928304 |
| 5192 | Crygn | NM_153076.2 | chr5:24751001-24757843 |
| 5193 | Crygs | NM_009967.2 | chr16:22805202-22811410 |
| 5194 | Cryl1 | NM_030004.3 | chr14:57275033-57398483 |
| 5195 | Crym | NM_016669.1 | chr7:120186383-120201988 |
| 5196 | Cryz | NM_009968.3 | chr3:154596710-154623182 |
| 5197 | Cryzl1 | NM_026994.1 | chr16:91689321-91728802 |
| 5198 | Cs | NM_026444.3 | chr10:128337831-128362479 |
| 5199 | Csad | NM_144942.4 | chr15:102176997-102189043 |
| 5200 | Csdc2 | NM_145473.3 | chr15:81936758-81950941 |
| 5201 | Csde1 | NM_001161854.2 | chr3:103020545-103058186 |
| 5202 | Cse1l | NM_023565.3 | chr2:166906095-166946389 |
| 5203 | Csf1 | NM_001113529.1 | chr3:107741047-107760469 |
| 5204 | Csf1r | NM_001037859.2 | chr18:61105571-61131139 |
| 5205 | Csf2 | NM_009969.4 | chr11:54247269-54249899 |
| 5206 | Csf2ra | NM_009970.2 | chr19:61224401-61228418 |
| 5207 | Csf2rb | NM_007780.4 | chr15:78325989-78351001 |
| 5208 | Csf2rb2 | NM_001287389.1 | chr15:78282507-78305721 |
| 5209 | Csf3 | NM_009971.1 | chr11:98701312-98703629 |
| 5210 | Csf3r | NM_001252651.1 | chr4:126024658-126044975 |
| 5211 | Csgalnact1 | NM_001252623.1 | chr8:68356780-68735146 |
| 5212 | Csgalnact2 | NM_030165.3 | chr6:118107451-118139140 |
| 5213 | Csk | NM_007783.3 | chr9:57626645-57645653 |
| 5214 | Csl | NM_027945.3 | chr10:99757704-99759658 |
| 5215 | Csmd1 | NM_053171.2 | chr8:15892544-17535385 |
| 5216 | Csmd2 | NM_001281955.1 | chr4:127988043-128567656 |
| 5217 | Csmd2os | NM_029137.1 | chr4:128133522-128166597 |
| 5218 | Csmd3 | NM_001081391.2 | chr15:47580637-48791989 |
| 5219 | Csn1s1 | NM_001286015.1 | chr5:87666207-87682577 |
| 5220 | Csn1s2a | NM_007785.2 | chr5:87774449-87788798 |
| 5221 | Csn1s2b | NM_009973.3 | chr5:87808120-87824421 |
| 5222 | Csn2 | NM_001286020.1 | chr5:87692618-87699425 |
| 5223 | Csn3 | NM_007786.4 | chr5:87925632-87932264 |
| 5224 | Csnk1a1 | NM_146087.2 | chr18:61555581-61588299 |
| 5225 | Csnk1d | NM_027874.2 | chr11:120961740-120991333 |
| 5226 | Csnk1e | NM_001289898.1 | chr15:79417851-79442057 |
| 5227 | Csnk1g1 | NM_173185.2 | chr9:65909009-66045014 |
| 5228 | Csnk1g2 | NM_001159591.1 | chr10:80629655-80640771 |
| 5229 | Csnk1g3 | NM_152809.2 | chr18:53862112-53955684 |
| 5230 | Csnk2a1 | NM_007788.3 | chr2:152226839-152281851 |
| 5231 | Csnk2a2 | NM_009974.3 | chr8:95446095-95488820 |
| 5232 | Csnk2b | NM_009975.3 | chr17:35116194-35121292 |
| 5233 | Csnka2ip | NM_173861.2 | chr16:64477810-64479134 |
| 5234 | Cspg4 | NM_139001.2 | chr9:56865103-56899870 |
| 5235 | Cspg5 | NM_001166273.1 | chr9:110243782-110262576 |
| 5236 | Cspp1 | NM_026493.3 | chr1:10038217-10136768 |
| 5237 | Csprs | NM_033616.3 | chr1_GL456221_random:111571-163011 |

Fig.21 - 28

| | | | | | | | |
|---|---|---|---|---|---|---|---|
| 5238 | Csrnp1 | NM_153287.3 | chr9:119971165-119984658 | 5335 | Cubn | NM_001081084.2 | chr2:13276337-13491876 |
| 5239 | Csrnp2 | NM_153407.2 | chr15:100479569-100495239 | 5336 | Cuedc1 | NM_001172099.1 | chr11:88169564-88194140 |
| 5240 | Csrnp3 | NM_001290665.1 | chr2:65931864-66031546 | 5337 | Cuedc2 | NM_001164290.1 | chr19:46329811-46338660 |
| 5241 | Csrp1 | NM_007791.5 | chr1:135729196-135752229 | 5338 | Cul1 | NM_012042.3 | chr6:47454323-47526139 |
| 5242 | Csrp2 | NM_007792.4 | chr10:110920175-110939514 | 5339 | Cul2 | NM_029402.3 | chr18:3383224-3436700 |
| 5243 | Csrp2bp | NM_001166640.1 | chr2:144369031-144407675 | 5340 | Cul3 | NM_016716.5 | chr1:80264922-80340690 |
| 5244 | Csrp3 | NM_001198841.1 | chr7:48830397-48848051 | 5341 | Cul4a | NM_146207.2 | chr8:13105720-13147939 |
| 5245 | Cst10 | NM_021405.2 | chr2:149405248-149410278 | 5342 | Cul4b | NM_001110142.1 | chrX:38531620-38576196 |
| 5246 | Cst11 | NM_030059.2 | chr2:148768617-148771497 | 5343 | Cul5 | NM_001161618.1 | chr9:53614581-53667507 |
| 5247 | Cst12 | NM_027054.1 | chr2:148789360-148793437 | 5344 | Cul7 | NM_025611.5 | chr17:46650337-46664364 |
| 5248 | Cst13 | NM_027024.3 | chr2:148820098-148830410 | 5345 | Cul9 | NM_001081335.2 | chr17:46500608-46546388 |
| 5249 | Cst3 | NM_009976.4 | chr2:148871721-148875512 | 5346 | Cuta | NM_026307.3 | chr17:26937971-26939538 |
| 5250 | Cst6 | NM_028623.5 | chr19:5344704-5349574 | 5347 | Cutal | NM_030021.3 | chr2:34874395-34892133 |
| 5251 | Cst7 | NM_009977.3 | chr2:150570414-150578944 | 5348 | Cutc | NM_001113562.1 | chr19:43753022-43768638 |
| 5252 | Cst8 | NM_009978.2 | chr2:148798838-148805584 | 5349 | Cux1 | NM_001291233.1 | chr5:136266409-136565981 |
| 5253 | Cst9 | NM_009979.1 | chr2:148835146-148838737 | 5350 | Cux2 | NM_007804.2 | chr5:121860215-122047825 |
| 5254 | Csta1 | NM_001033239.3 | chr16:36119945-36131189 | 5351 | Cuzd1 | NM_008411.3 | chr7:131308553-131322292 |
| 5255 | Cstad | NM_030137.2 | chr2:30595043-30608945 | 5352 | Cwc15 | NM_023153.3 | chr9:14500618-14510620 |
| 5256 | Cstb | NM_007793.3 | chr10:78425669-78427622 | 5353 | Cwc22 | NM_001290740.1 | chr2:77895652-77946356 |
| 5257 | Cstf1 | NM_024199.2 | chr2:172371002-172381086 | 5354 | Cwc25 | NM_026186.4 | chr11:97745469-97766613 |
| 5258 | Cstf2 | NM_001290398.1 | chrX:134059175-134086822 | 5355 | Cwc27 | NM_026072.1 | chr13:104631326-104816953 |
| 5259 | Cstf2t | NM_031249.2 | chr19:31082840-31086592 | 5356 | Cwf19l1 | NM_001081077.1 | chr19:44108636-44135876 |
| 5260 | Cstf3 | NM_001037326.2 | chr2:104590483-104609429 | 5357 | Cwf19l2 | NM_027545.2 | chr9:3404084-3479236 |
| 5261 | Cstl1 | NM_177655.3 | chr2:148750360-148755378 | 5358 | Cwh43 | NM_181323.2 | chr5:73406077-73453425 |
| 5262 | Ctag2 | NM_027302.2 | chrX:65047643-65049017 | 5359 | Cx3cl1 | NM_009142.3 | chr8:94772179-94782426 |
| 5263 | Ctage5 | NM_001165253.1 | chr12:59131452-59190220 | 5360 | Cx3cr1 | NM_009987.4 | chr9:120048682-120068296 |
| 5264 | Ctbp1 | NM_001198859.1 | chr5:33247722-33275004 | 5361 | Cxadr | NM_001025192.3 | chr16:78301670-78340759 |
| 5265 | Ctbp2 | NM_001170744.1 | chr7:132987010-133015247 | 5362 | Cxcl1 | NM_008176.3 | chr5:90891244-90893115 |
| 5266 | Ctbs | NM_028836.4 | chr3:146450466-146465848 | 5363 | Cxcl10 | NM_021274.2 | chr5:92346638-92348889 |
| 5267 | Ctc1 | NM_001013256.2 | chr11:69015910-69036473 | 5364 | Cxcl11 | NM_019494.1 | chr5:92359544-92363277 |
| 5268 | Ctcf | NM_181322.3 | chr8:105636537-105682922 | 5365 | Cxcl12 | NM_001012477.2 | chr6:117168534-117181368 |
| 5269 | Ctcfl | NM_001081387.2 | chr2:173093608-173119525 | 5366 | Cxcl13 | NM_018866.2 | chr5:95956938-95961068 |
| 5270 | Ctcflos | NR_040321.1 | chr2:173124748-173133204 | 5367 | Cxcl14 | NM_019568.2 | chr13:56288642-56296551 |
| 5271 | Ctdnep1 | NM_026017.2 | chr11:69981167-69990600 | 5368 | Cxcl15 | NM_011339.2 | chr5:90794533-90803067 |
| 5272 | Ctdp1 | NM_026295.2 | chr18:80407958-80469667 | 5369 | Cxcl16 | NM_023158.6 | chr11:70454233-70459984 |
| 5273 | Ctdsp1 | NM_153088.2 | chr1:74391608-74397285 | 5370 | Cxcl17 | NM_153576.2 | chr7:25400052-25412886 |
| 5274 | Ctdsp2 | NM_001113470.1 | chr10:126978716-126999975 | 5371 | Cxcl2 | NM_009140.2 | chr5:90903898-90905938 |
| 5275 | Ctdspl | NM_133710.3 | chr9:118926535-119044119 | 5372 | Cxcl3 | NM_203320.3 | chr5:90786100-90788093 |
| 5276 | Ctdspl2 | NM_001290991.1 | chr2:121956000-122015326 | 5373 | Cxcl5 | NM_009141.3 | chr5:90759297-90761625 |
| 5277 | Ctf1 | NM_007795.1 | chr7:127712735-127718185 | 5374 | Cxcl9 | NM_008599.4 | chr5:92321330-92328079 |
| 5278 | Ctf2 | NM_198858.1 | chr7:127718959-127725616 | 5375 | Cxcr1 | NM_178241.4 | chr1:74191785-74194631 |
| 5279 | Ctgf | NM_010217.2 | chr10:24595441-24598682 | 5376 | Cxcr2 | NM_009909.3 | chr1:74153993-74161246 |
| 5280 | Cth | NM_145953.2 | chr3:157894247-157925063 | 5377 | Cxcr3 | NM_009910.3 | chrX:101731534-101734147 |
| 5281 | Cthrc1 | NM_026778.2 | chr15:39076931-39087119 | 5378 | Cxcr4 | NM_009911.3 | chr1:128588198-128592299 |
| 5282 | Ctif | NM_201354.2 | chr18:75431220-75697696 | 5379 | Cxcr5 | NM_007551.2 | chr9:44511786-44526421 |
| 5283 | Ctla2a | NM_001145799.1 | chr13:60934154-60936566 | 5380 | Cxcr6 | NM_030712.4 | chr9:123806476-123811754 |
| 5284 | Ctla2b | NM_001145801.1 | chr13:60895350-60897447 | 5381 | Cxx1a | NM_024170.2 | chrX:53642488-53643763 |
| 5285 | Ctla4 | NM_001281976.1 | chr1:60909024-60915832 | 5382 | Cxx1b | NM_001018063.1 | chrX:53669176-53670408 |
| 5286 | Ctnna1 | NM_009818.1 | chr18:35118911-35254775 | 5383 | Cxx1c | NM_028375.3 | chrX:53607921-53609134 |
| 5287 | Ctnna2 | NM_001109764.1 | chr6:76881636-77979667 | 5384 | Cxxc1 | NM_028868.3 | chr18:74216211-74221491 |
| 5288 | Ctnna3 | NM_001164376.1 | chr10:63457510-65003667 | 5385 | Cxxc4 | NM_001004367.4 | chr3:134236494-134262089 |
| 5289 | Ctnnal1 | NM_018761.3 | chr4:56810934-56865211 | 5386 | Cxxc5 | NM_133687.2 | chr18:35829817-35861688 |
| 5290 | Ctnnb1 | NM_001165902.1 | chr9:120933577-120960507 | 5387 | Cyb5 | NM_025797.3 | chr18:84851413-84879863 |
| 5291 | Ctnnbip1 | NM_001141930.1 | chr4:149545116-149566437 | 5388 | Cyb561 | NM_007805.4 | chr11:105933703-105944147 |
| 5292 | Ctnnbl1 | NM_025680.4 | chr2:157737400-157891903 | 5389 | Cyb561a3 | NM_001282064.1 | chr19:10577156-10590041 |
| 5293 | Ctnnd1 | NM_001085448.1 | chr2:84600780-84650740 | 5390 | Cyb561d1 | NM_001081320.2 | chr3:108195770-108200834 |
| 5294 | Ctnnd2 | NM_008729.2 | chr15:30172592-31029343 | 5391 | Cyb561d2 | NM_019720.4 | chr9:107539010-107541865 |
| 5295 | Ctns | NM_031251.4 | chr11:73183132-73199019 | 5392 | Cyb5b | NM_025558.5 | chr8:107150660-107187470 |
| 5296 | Ctps | NM_016748.2 | chr4:120539867-120570276 | 5393 | Cyb5d1 | NM_001045525.1 | chr11:69393611-69395346 |
| 5297 | Ctps2 | NM_001168568.1 | chrX:162901559-163034541 | 5394 | Cyb5d2 | NM_001024926.3 | chr11:72777231-72795839 |
| 5298 | Ctr9 | NM_009431.2 | chr7:111028950-111056377 | 5395 | Cyb5r1 | NM_028057.2 | chr1:134405989-134411738 |
| 5299 | Ctrb1 | NM_025583.2 | chr8:111686509-111691010 | 5396 | Cyb5r2 | NM_001205227.1 | chr7:107748454-107758032 |
| 5300 | Ctrc | NM_001033875.2 | chr4:141838239-141846359 | 5397 | Cyb5r3 | NM_029787.2 | chr15:83153500-83172208 |
| 5301 | Ctrcos | NR_040641.1 | chr4:141844320-141846990 | 5398 | Cyb5r4 | NM_024195.2 | chr9:87022028-87077774 |
| 5302 | Ctrl | NM_023182.2 | chr8:105931993-105933862 | 5399 | Cyb5rl | NM_175471.2 | chr4:107070167-107084805 |
| 5303 | Cts3 | NM_026906.3 | chr13:61564629-61570127 | 5400 | Cyba | NM_007806.3 | chr8:122424770-122432940 |
| 5304 | Cts6 | NM_021445.1 | chr13:61195146-61203392 | 5401 | Cybb | NM_007807.5 | chrX:9435253-9469324 |
| 5305 | Cts7 | NM_019539.3 | chr13:61352460-61358170 | 5402 | Cybrd1 | NM_028593.2 | chr2:71118053-71142926 |
| 5306 | Cts8 | NM_019541.3 | chr13:61246746-61255348 | 5403 | Cyc1 | NM_025567.2 | chr15:76343522-76345934 |
| 5307 | Cts8-ps | NR_027871.2 | chr13:61281379-61288850 | 5404 | Cycs | NM_007808.4 | chr6:50562562-50566474 |
| 5308 | Ctsa | NM_001038492.2 | chr2:164832871-164841032 | 5405 | Cyct | NM_009989.3 | chr2:76353941-76360453 |
| 5309 | Ctsb | NM_007798.3 | chr14:63122461-63145923 | 5406 | Cyfip1 | NM_001164661.1 | chr7:55842070-55932633 |
| 5310 | Ctsc | NM_009982.5 | chr7:88278084-88315861 | 5407 | Cyfip2 | NM_001252459.1 | chr11:46193848-46312859 |
| 5311 | Ctsd | NM_009983.2 | chr7:142375915-142387870 | 5408 | Cygb | NM_030206.4 | chr11:116645594-116654313 |
| 5312 | Ctse | NM_007799.3 | chr1:131638313-131675507 | 5409 | Cyhr1 | NM_001276321.1 | chr15:76646926-76656901 |
| 5313 | Ctsf | NM_019861.1 | chr19:4855128-4860912 | 5410 | Cylc1 | NM_026134.2 | chrX:111110417-111123874 |
| 5314 | Ctsg | NM_007800.2 | chr14:56099884-56102574 | 5411 | Cylc2 | NM_001162865.1 | chr4:51216677-51229928 |
| 5315 | Ctsh | NM_007801.3 | chr9:90054266-90076095 | 5412 | Cyld | NM_001128170.2 | chr8:88697027-88751946 |
| 5316 | Ctsj | NM_012007.1 | chr13:61000277-61005911 | 5413 | Cym | NM_001111143.1 | chr3:107211294-107221732 |
| 5317 | Ctsk | NM_007802.4 | chr3:95499209-95509387 | 5414 | Cyp11a1 | NM_019779.3 | chr9:58015016-58027023 |
| 5318 | Ctsl | NM_009984.4 | chr13:64361889-64370520 | 5415 | Cyp11b1 | NM_001033229.3 | chr15:74834891-74841643 |
| 5319 | Ctsll3 | NM_027344.3 | chr13:60798249-60802844 | 5416 | Cyp11b2 | NM_009991.3 | chr15:74851009-74856318 |
| 5320 | Ctsm | NM_022326.3 | chr13:61536443-61541839 | 5417 | Cyp17a1 | NM_007809.3 | chr19:46667164-46673000 |
| 5321 | Ctso | NM_177662.2 | chr3:81932615-81956725 | 5418 | Cyp19a1 | NM_007810.3 | chr9:54165936-54193442 |
| 5322 | Ctsq | NM_029636.3 | chr13:61035037-61040597 | 5419 | Cyp1a1 | NM_001136059.2 | chr9:57697612-57703824 |
| 5323 | Ctsr | NM_020284.1 | chr13:61159214-61164188 | 5420 | Cyp1a2 | NM_009993.3 | chr9:57676936-57683655 |
| 5324 | Ctss | NM_001267695.2 | chr3:95526785-95556405 | 5421 | Cyp1b1 | NM_009994.1 | chr17:79706952-79715041 |
| 5325 | Ctsw | NM_009985.5 | chr19:5465042-5468507 | 5422 | Cyp20a1 | NM_030013.3 | chr1:60343299-60388060 |
| 5326 | Ctsz | NM_022325.5 | chr2:174427493-174439039 | 5423 | Cyp21a1 | NM_009995.2 | chr17:34801347-34804426 |
| 5327 | Cttn | NM_001252572.1 | chr7:144435723-144470935 | 5424 | Cyp24a1 | NM_009996.4 | chr2:170482956-170497145 |
| 5328 | Cttnbp2 | NM_080285.1 | chr6:18366476-18514825 | 5425 | Cyp26a1 | NM_007811.2 | chr19:37697807-37701536 |
| 5329 | Cttnbp2nl | NM_001163332.1 | chr3:105001914-105052953 | 5426 | Cyp26b1 | NM_001177713.1 | chr6:84571413-84593908 |
| 5330 | Ctu1 | NM_145582.1 | chr7:43672030-43678297 | 5427 | Cyp26c1 | NM_001105201.1 | chr19:37685679-37693307 |
| 5331 | Ctu2 | NM_153775.2 | chr8:122476142-122483092 | 5428 | Cyp27a1 | NM_024264.5 | chr1:74713534-74737897 |
| 5332 | Ctxn1 | NM_183315.2 | chr8:4257645-4259274 | 5429 | Cyp27b1 | NM_010009.2 | chr10:127048245-127053006 |
| 5333 | Ctxn2 | NM_001162934.1 | chr2:125136691-125147841 | 5430 | Cyp2a12 | NM_133657.1 | chr7:27029089-27036815 |
| 5334 | Ctxn3 | NM_001134697.1 | chr18:57468485-57478134 | 5431 | Cyp2a22 | NM_001101467.1 | chr7:26931630-26939386 |

Fig.21 - 29

| | | | |
|---|---|---|---|
| 5432 | Cyp2a4 | NM_009997.2 | chr7:26307191-26315088 |
| 5433 | Cyp2a5 | NM_007812.4 | chr7:26835338-26843264 |
| 5434 | Cyp2ab1 | NM_183158.3 | chr16:20308386-20325404 |
| 5435 | Cyp2b10 | NM_009999.4 | chr7:25897657-25926624 |
| 5436 | Cyp2b13 | NM_007813.2 | chr7:26061494-26096196 |
| 5437 | Cyp2b19 | NM_007814.2 | chr7:26757141-26772630 |
| 5438 | Cyp2b23 | NM_001081148.1 | chr7:26665226-26686430 |
| 5439 | Cyp2b9 | NM_010000.2 | chr7:26173408-26210660 |
| 5440 | Cyp2c29 | NM_007815.3 | chr19:39287084-39330713 |
| 5441 | Cyp2c37 | NM_010001.2 | chr19:39992423-40012243 |
| 5442 | Cyp2c38 | NM_010002.3 | chr19:39389555-39463075 |
| 5443 | Cyp2c39 | NM_010003.2 | chr19:39510821-39568529 |
| 5444 | Cyp2c40 | NM_010004.2 | chr19:39767072-39812814 |
| 5445 | Cyp2c44 | NM_001001446.3 | chr19:44005021-44029247 |
| 5446 | Cyp2c50 | NM_001167875.1 | chr19:40089678-40113955 |
| 5447 | Cyp2c53-ps | NR_033614.1 | chr19:39229253-39274737 |
| 5448 | Cyp2c54 | NM_206537.2 | chr19:40037939-40073813 |
| 5449 | Cyp2c55 | NM_028089.3 | chr19:39007018-39042687 |
| 5450 | Cyp2c65 | NM_028191.2 | chr19:39061005-39093948 |
| 5451 | Cyp2c66 | NM_001011707.1 | chr19:39113897-39186756 |
| 5452 | Cyp2c67 | NM_001024719.2 | chr19:39608858-39649042 |
| 5453 | Cyp2c68 | NM_001039555.2 | chr19:39688835-39741101 |
| 5454 | Cyp2c69 | NM_001104525.1 | chr19:39842659-39886769 |
| 5455 | Cyp2c70 | NM_145499.2 | chr19:40153360-40187286 |
| 5456 | Cyp2d10 | NM_010005.3 | chr15:82402845-82407194 |
| 5457 | Cyp2d11 | NM_001104531.1 | chr15:82389153-82394022 |
| 5458 | Cyp2d12 | NM_201360.1 | chr15:82555097-82559413 |
| 5459 | Cyp2d13 | NR_003552.1 | chr15:82636749-82642045 |
| 5460 | Cyp2d22 | NM_001163472.1 | chr15:82370526-82380260 |
| 5461 | Cyp2d26 | NM_029562.2 | chr15:82790106-82794245 |
| 5462 | Cyp2d34 | NM_145474.2 | chr15:82615964-82620907 |
| 5463 | Cyp2d37-ps | NR_033515.1 | chr15:82688749-82690058 |
| 5464 | Cyp2d40 | NM_023623.2 | chr15:82759832-82764122 |
| 5465 | Cyp2d9 | NM_010006.2 | chr15:82452376-82456827 |
| 5466 | Cyp2e1 | NM_021282.2 | chr7:140763831-140774977 |
| 5467 | Cyp2f2 | NM_007817.2 | chr7:27119954-27133660 |
| 5468 | Cyp2g1 | NM_013809.1 | chr7:26808926-26821197 |
| 5469 | Cyp2j11 | NM_001004141.2 | chr4:96294508-96348680 |
| 5470 | Cyp2j12 | NM_001100182.2 | chr4:96099317-96141152 |
| 5471 | Cyp2j13 | NM_145548.4 | chr4:96042659-96077540 |
| 5472 | Cyp2j5 | NM_010007.4 | chr4:96627431-96664119 |
| 5473 | Cyp2j6 | NM_010008.4 | chr4:96516137-96553651 |
| 5474 | Cyp2j8 | NM_001104927.1 | chr4:96444587-96507386 |
| 5475 | Cyp2j9 | NM_028979.2 | chr4:96568428-96591485 |
| 5476 | Cyp2r1 | NM_177382.4 | chr7:114550162-114562972 |
| 5477 | Cyp2s1 | NM_028775.3 | chr7:25802475-25816530 |
| 5478 | Cyp2t4 | NM_001100184.1 | chr7:27153713-27158564 |
| 5479 | Cyp2u1 | NM_027816.3 | chr3:131290490-131303227 |
| 5480 | Cyp2w1 | NM_001160265.1 | chr5:139352616-139357033 |
| 5481 | Cyp39a1 | NM_001285947.1 | chr17:43667371-43751431 |
| 5482 | Cyp3a11 | NM_007818.3 | chr5:145854606-145879854 |
| 5483 | Cyp3a13 | NM_007819.4 | chr5:137892932-137921619 |
| 5484 | Cyp3a16 | NM_007820.2 | chr5:145436308-145469723 |
| 5485 | Cyp3a25 | NM_019792.2 | chr5:145977193-146009617 |
| 5486 | Cyp3a41a | NM_017396.3 | chr5:145694049-145720136 |
| 5487 | Cyp3a41b | NM_001105159.1 | chr5:145558663-145584730 |
| 5488 | Cyp3a44 | NM_177380.3 | chr5:145773982-145805874 |
| 5489 | Cyp3a57 | NM_001100180.1 | chr5:145345269-145390512 |
| 5490 | Cyp3a59 | NM_001105160.1 | chr5:146079257-146113283 |
| 5491 | Cyp46a1 | NM_010010.1 | chr12:108334380-108362234 |
| 5492 | Cyp4a10 | NM_010011.3 | chr4:115518286-115533649 |
| 5493 | Cyp4a12a | NM_177406.3 | chr4:115299045-115332815 |
| 5494 | Cyp4a12b | NM_172306.2 | chr4:115411623-115439034 |
| 5495 | Cyp4a14 | NM_007822.2 | chr4:115486199-115496141 |
| 5496 | Cyp4a29 | NM_001100183.1 | chr4:115242083-115254557 |
| 5497 | Cyp4a30b | NM_001100185.1 | chr4:115452603-115471062 |
| 5498 | Cyp4a31 | NM_001252539.1 | chr4:115563648-115579015 |
| 5499 | Cyp4a32 | NM_001100181.1 | chr4:115600937-115622366 |
| 5500 | Cyp4b1 | NM_007823.2 | chr4:115624727-115647705 |
| 5501 | Cyp4b1-ps2 | NR_033575.1 | chr4:115582037-115583127 |
| 5502 | Cyp4f13 | NM_130882.1 | chr17:32924687-32947361 |
| 5503 | Cyp4f14 | NM_001204333.1 | chr17:32905069-32917329 |
| 5504 | Cyp4f15 | NM_134127.1 | chr17:32685658-32703349 |
| 5505 | Cyp4f16 | NM_024442.1 | chr17:32536628-32551797 |
| 5506 | Cyp4f17 | NM_001101445.1 | chr17:32506461-32528894 |
| 5507 | Cyp4f18 | NM_024444.2 | chr8:71988481-72009626 |
| 5508 | Cyp4f37 | NM_001100187.1 | chr17:32621318-32636184 |
| 5509 | Cyp4f39 | NM_177307.3 | chr17:32452722-32493320 |
| 5510 | Cyp4f40 | NM_001101588.1 | chr17:32659482-32676480 |
| 5511 | Cyp4f41-ps | NR_033585.1 | chr17:32950960-32965716 |
| 5512 | Cyp4v3 | NM_133969.2 | chr8:45305801-45333196 |
| 5513 | Cyp4x1 | NM_001003947.1 | chr4:115108681-115133977 |
| 5514 | Cyp51 | NM_020010.2 | chr5:4080673-4104697 |
| 5515 | Cyp7a1 | NM_007824.2 | chr4:6265611-6275631 |
| 5516 | Cyp7b1 | NM_007825.4 | chr3:18071949-18243338 |
| 5517 | Cyp8b1 | NM_010012.3 | chr9:121914355-121916305 |
| 5518 | Cypt1 | NM_025738.3 | chrX:16522868-16523691 |
| 5519 | Cypt12 | NM_029289.1 | chr3:17948443-17948952 |
| 5520 | Cypt14 | NM_001191032.1 | chrX:39862918-39863604 |
| 5521 | Cypt15 | NM_001177380.1 | chrX:39346265-39346961 |
| 5522 | Cypt2 | NM_173436.2 | chrX:105499771-105500637 |
| 5523 | Cypt3 | NM_173367.3 | chrX:153558592-153559435 |
| 5524 | Cypt4 | NM_173412.2 | chr9:24625184-24625828 |
| 5525 | Cypt7 | NM_001039943.2 | chrX:16522880-16523615 |
| 5526 | Cypt8 | NM_001039941.2 | chrX:16522880-16523615 |
| 5527 | Cypt9 | NM_001039942.2 | chr9:24625203-24625737 |
| 5528 | Cyr61 | NM_010516.2 | chr3:145646970-145649985 |
| 5529 | Cys1 | NM_001004455.2 | chr12:24665837-24681795 |
| 5530 | Cysltr1 | NM_001281859.1 | chrX:106576508-106603679 |
| 5531 | Cysltr2 | NM_001162412.1 | chr14:73029127-73049114 |
| 5532 | Cystm1 | NM_001081365.1 | chr18:36348623-36393370 |
| 5533 | Cyth1 | NM_001112699.1 | chr11:118164165-118248592 |
| 5534 | Cyth2 | NM_001112701.1 | chr7:45806636-45814316 |
| 5535 | Cyth3 | NM_001163548.1 | chr5:143651240-143710248 |
| 5536 | Cyth4 | NM_028195.3 | chr15:78597046-78622019 |
| 5537 | Cytip | NM_139200.4 | chr2:58129138-58160122 |
| 5538 | Cytl1 | NM_001081106.1 | chr5:37735518-37739820 |
| 5539 | Cyyr1 | NM_144853.3 | chr16:85456243-85550373 |
| 5540 | D030018L15Rik | NR_126492.1 | chr15:96051616-96079553 |
| 5541 | D030024E09Rik | NR_040350.1 | chr15:61699283-61774451 |
| 5542 | D030025E07Rik | NR_045704.1 | chr3:128117014-128231605 |
| 5543 | D030025P21Rik | NR_028577.1 | chr12:84875801-84879755 |
| 5544 | D030028A08Rik | NR_003293.3 | chr11:96944145-96965060 |
| 5545 | D030040B21Rik | NR_037998.1 | chr1:16657551-16662278 |
| 5546 | D030045P18Rik | NR_040624.1 | chr10:45807703-45852018 |
| 5547 | D030047H15Rik | NR_033548.1 | chr7:4126339-4136926 |
| 5548 | D030056L22Rik | NM_177640.4 | chr19:18713235-18718428 |
| 5549 | D10Bwg1379e | NM_001033258.4 | chr10:18588010-18743758 |
| 5550 | D10Jhu81e | NM_138601.2 | chr10:78162066-78169768 |
| 5551 | D10Wsu102e | NM_026579.3 | chr10:83360220-83368835 |
| 5552 | D11Wsu47e | NM_177777.5 | chr11:113684411-113694647 |
| 5553 | D130009I18Rik | NR_015593.2 | chr14:104639146-104966783 |
| 5554 | D130017N08Rik | NR_015486.2 | chr5:143758353-143764942 |
| 5555 | D130020L05Rik | NR_038047.1 | chr12:101082450-101088927 |
| 5556 | D130040H23Rik | NM_172491.2 | chr8:69271079-69303375 |
| 5557 | D130043K22Rik | NM_001081051.2 | chr13:24845130-24901439 |
| 5558 | D130058E03 | NR_073373.1 | chr6:127296185-127307967 |
| 5559 | D14Ertd670e | NR_105025.1 | chr14:19838924-19844677 |
| 5560 | D15Ertd621e | NM_145959.3 | chr15:58415467-58457801 |
| 5561 | D16Ertd472e | NM_001252438.1 | chr16:78540335-78576688 |
| 5562 | D16Ertd519e | NR_040474.1 | chr16:70616424-70624822 |
| 5563 | D17Ertd648e | NR_045808.1 | chr17:11863069-11883189 |
| 5564 | D17H6S53E | NM_033477.2 | chr17:35126401-35128855 |
| 5565 | D17Wsu104e | NM_080837.2 | chr17:56176540-56183920 |
| 5566 | D17Wsu92e | NM_001033279.3 | chr17:27751231-27820542 |
| 5567 | D19Bwg1357e | NM_177474.5 | chr19:27388697-27429908 |
| 5568 | D1Ertd622e | NM_133825.3 | chr1:97643901-97662018 |
| 5569 | D1Pas1 | NM_033077.3 | chr1:186967415-186970627 |
| 5570 | D230025D16Rik | NM_145604.2 | chr8:105225187-105253051 |
| 5571 | D230030E09Rik | NR_045947.1 | chr12:118518296-118530193 |
| 5572 | D2hgdh | NM_178882.4 | chr1:93825239-93852361 |
| 5573 | D2Wsu81e | NM_172660.4 | chr2:30173446-30178459 |
| 5574 | D330023K18Rik | NR_040334.1 | chr2:31151048-31152291 |
| 5575 | D330041H03Rik | NR_033554.1 | chr17:24409480-24414513 |
| 5576 | D330045A20Rik | NM_175326.5 | chrX:139480366-139554580 |
| 5577 | D330050G23Rik | NR_040335.1 | chr2:116900151-116912791 |
| 5578 | D330050I16Rik | NR_033224.1 | chr19:5388335-5390069 |
| 5579 | D3Bwg0562e | NM_177664.5 | chr3:117319145-117360876 |
| 5580 | D3Ertd254e | NM_001101478.1 | chr3:36151079-36170341 |
| 5581 | D3Ertd751e | NM_001099785.1 | chr3:41742617-41758939 |
| 5582 | D430019H16Rik | NM_001252508.1 | chr12:105453855-105493095 |
| 5583 | D430020J02Rik | NR_028421.1 | chr12:116401946-116405161 |
| 5584 | D430036J16Rik | NR_040393.1 | chr9:81631929-81645156 |
| 5585 | D430041D05Rik | NM_001033347.2 | chr2:104143074-104410334 |
| 5586 | D430042O09Rik | NM_001081022.1 | chr7:125707875-125874797 |
| 5587 | D4Ertd617e | NR_029469.1 | chr4:118626403-118631915 |
| 5588 | D530049I02Rik | NR_040605.1 | chr1:73398967-73430779 |
| 5589 | D5Ertd577e | NM_177187.4 | chr5:95456805-95485589 |
| 5590 | D5Ertd579e | NM_001081232.3 | chr5:36600485-36696021 |
| 5591 | D5Ertd605e | NR_033625.1 | chr5:147418619-147423044 |
| 5592 | D630003M21Rik | NM_001131021.2 | chr2:158182532-158229224 |
| 5593 | D630010B17Rik | NR_045629.1 | chr15:94247201-94255606 |
| 5594 | D630013N20Rik | NR_045291.1 | chr10:70601048-70651925 |
| 5595 | D630023F18Rik | NM_001285881.1 | chr1:65107312-65123214 |
| 5596 | D630024D03Rik | NR_102310.1 | chr11:31817983-31824547 |
| 5597 | D630029K05Rik | NR_027846.1 | chr10:116966273-116969107 |
| 5598 | D630032N06Rik | NR_028329.1 | chr11:85235165-85238304 |
| 5599 | D630033O11Rik | NM_001243261.1 | chr9:43259878-43280075 |
| 5600 | D630039A03Rik | NM_178727.2 | chr4:57908383-57916264 |
| 5601 | D630041G03Rik | NR_028416.1 | chr7:4467244-4470884 |
| 5602 | D630045J12Rik | NM_194061.2 | chr6:38123173-38254009 |
| 5603 | D630045M09Rik | NR_045293.1 | chr13:73344664-73347384 |
| 5604 | D6Ertd474e | NR_027803.1 | chr6:143245887-143297879 |
| 5605 | D6Ertd527e | NM_001167937.1 | chr6:87104745-87113003 |
| 5606 | D6Wsu163e | NM_138594.3 | chr6:126939965-126975704 |
| 5607 | D730001G18Rik | NR_027836.1 | chr15:74770907-74778859 |
| 5608 | D730005E14Rik | NR_030675.1 | chr15:79889531-79893138 |
| 5609 | D730045A05Rik | NR_045390.1 | chr18:74017906-74020088 |
| 5610 | D730048I06Rik | NM_026593.3 | chr9:35788049-35790112 |
| 5611 | D730050B12Rik | NR_046196.1 | chr13:72809600-72816763 |
| 5612 | D7Ertd143e | NR_028425.1 | chr7:3218783-3221015 |
| 5613 | D7Ertd443e | NM_001081331.1 | chr7:134266261-134376828 |
| 5614 | D7Ertd715e | NR_015456.1 | chr7:59969576-59974431 |
| 5615 | D830005E20Rik | NR_040657.1 | chr10:33222344-33256357 |
| 5616 | D830013O20Rik | NR_046013.1 | chr12:73364074-73409563 |
| 5617 | D830015G02Rik | NR_033497.1 | chr14:54968786-54974349 |
| 5618 | D830026I12Rik | NR_102304.1 | chr6:17198106-17205695 |
| 5619 | D830030K20Rik | NM_177135.4 | chr14:3224439-3856080 |
| 5620 | D830031N03Rik | NM_001167918.1 | chr4:123403600-123411911 |
| 5621 | D830032E09Rik | NR_102306.1 | chr1:107917258-107950947 |
| 5622 | D830046C22Rik | NR_033147.1 | chr5:139377696-139380053 |
| 5623 | D8Ertd738e | NM_001007571.2 | chr8:84246234-84249761 |
| 5624 | D8Ertd82e | NM_172911.3 | chr8:36094827-36147787 |
| 5625 | D930007P13Rik | NR_045743.1 | chr15:103123069-103146828 |

Fig.21 - 30

| | | | |
|---|---|---|---|
| 5626 | D930015E06Rik | NM_172681.4 | chr3:83898286-84040161 |
| 5627 | D930015M05Rik | NR_040621.1 | chr2:92408891-92432308 |
| 5628 | D930016D06Rik | NR_030673.1 | chr5:104525734-104554211 |
| 5629 | D930020B18Rik | NM_177335.4 | chr10:121641700-121693914 |
| 5630 | D930028M14Rik | NR_045847.1 | chr7:25152456-25156630 |
| 5631 | D930032P07Rik | NR_045330.1 | chr19:28678223-28720027 |
| 5632 | D930048N14Rik | NR_027958.1 | chr11:51650953-51657681 |
| 5633 | Daam1 | NM_001286452.1 | chr12:71889542-71992376 |
| 5634 | Daam2 | NM_001008231.2 | chr17:49456021-49564337 |
| 5635 | Dab1 | NM_010014.3 | chr4:104367537-104680389 |
| 5636 | Dab2 | NM_001008702.2 | chr15:6299788-6440709 |
| 5637 | Dab2ip | NM_001001602.2 | chr2:35691993-35730994 |
| 5638 | Dach1 | NM_001038610.2 | chr14:97786845-98169765 |
| 5639 | Dach2 | NM_001142570.1 | chrX:113298255-113835091 |
| 5640 | Dact1 | NM_001190466.1 | chr12:71309883-71320107 |
| 5641 | Dact2 | NM_172826.3 | chr17:14195229-14203831 |
| 5642 | Dact3 | NM_001081655.1 | chr7:16875316-16887301 |
| 5643 | Dad1 | NM_001113358.1 | chr14:54235484-54253929 |
| 5644 | Daf2 | NM_007827.2 | chr1:130388527-130422999 |
| 5645 | Dag1 | NM_001276481.1 | chr9:108204860-108263815 |
| 5646 | Dagla | NM_198114.2 | chr19:10245264-10304877 |
| 5647 | Daglb | NM_144915.3 | chr5:143464492-143504442 |
| 5648 | Dak | NM_145496.1 | chr19:10592196-10604258 |
| 5649 | Dalrd3 | NM_026378.3 | chr9:108569891-108572771 |
| 5650 | Dancr | NR_015531.1 | chr5:74093082-74094336 |
| 5651 | Dand5 | NM_201227.3 | chr8:84815404-84822823 |
| 5652 | Dao | NM_001286396.1 | chr5:114004807-114025675 |
| 5653 | Dap | NM_146057.3 | chr15:31224384-31274338 |
| 5654 | Dap3 | NM_001164533.1 | chr3:88920802-88950282 |
| 5655 | Dapk1 | NM_001285917.1 | chr13:60601946-60763191 |
| 5656 | Dapk2 | NM_010019.3 | chr9:66158225-66272242 |
| 5657 | Dapk3 | NM_001190473.1 | chr10:81183262-81193197 |
| 5658 | Dapl1 | NM_029723.3 | chr2:59484652-59505020 |
| 5659 | Dapp1 | NM_011932.3 | chr3:137931006-137981549 |
| 5660 | Dars | NM_145507.2 | chr1:128412117-128417416 |
| 5661 | Dars2 | NM_172644.4 | chr1:161040600-161070660 |
| 5662 | Daw1 | NM_027725.3 | chr1:83159761-83210572 |
| 5663 | Daxx | NM_001199733.1 | chr17:33909600-33915590 |
| 5664 | Dazap1 | NM_001122604.1 | chr10:80264990-80288413 |
| 5665 | Dazap2 | NM_011873.2 | chr15:100615661-100620761 |
| 5666 | Dazl | NM_001277863.1 | chr17:50279392-50293620 |
| 5667 | Dbf4 | NM_001190717.1 | chr5:8396968-8422716 |
| 5668 | Dbh | NM_138942.3 | chr2:27165506-27183204 |
| 5669 | Dbhos | NR_040524.1 | chr2:27144897-27162703 |
| 5670 | Dbi | NM_001037999.2 | chr1:120113279-120120919 |
| 5671 | Dbil5 | NM_021294.2 | chr11:76217612-76218665 |
| 5672 | Dbn1 | NM_001177371.1 | chr13:55473427-55488076 |
| 5673 | Dbndd1 | NM_001170975.2 | chr8:123505686-123515455 |
| 5674 | Dbndd2 | NM_001048227.1 | chr2:164486139-164493323 |
| 5675 | Dbnl | NM_001146308.1 | chr11:5788482-5800980 |
| 5676 | Dbp | NM_016974.3 | chr7:45705246-45710203 |
| 5677 | Dbpht2 | NM_198866.2 | chr12:74297473-74300468 |
| 5678 | Dbr1 | NM_031403.3 | chr9:99575798-99584343 |
| 5679 | Dbt | NM_010022.3 | chr3:116513078-116549981 |
| 5680 | Dbx1 | NM_001005232.1 | chr7:49631498-49636835 |
| 5681 | Dbx2 | NM_207533.2 | chr15:95623562-95654771 |
| 5682 | Dcaf10 | NM_153167.2 | chr4:45342100-45379722 |
| 5683 | Dcaf11 | NM_001199009.1 | chr14:55560922-55570065 |
| 5684 | Dcaf12 | NM_026893.3 | chr4:41291299-41314901 |
| 5685 | Dcaf12l1 | NM_001190718.1 | chrX:44786566-44790161 |
| 5686 | Dcaf12l2 | NM_175539.3 | chrX:44365456-44368337 |
| 5687 | Dcaf13 | NM_198606.2 | chr15:39112873-39146855 |
| 5688 | Dcaf15 | NM_172502.3 | chr8:84097071-84104762 |
| 5689 | Dcaf17 | NM_001165980.1 | chr2:71055743-71095858 |
| 5690 | Dcaf4 | NM_001165256.1 | chr12:83526048-83541992 |
| 5691 | Dcaf5 | NM_177267.4 | chr12:80335846-80436601 |
| 5692 | Dcaf6 | NM_028759.1 | chr1:165329500-165460463 |
| 5693 | Dcaf7 | NM_027946.3 | chr11:106036871-106059323 |
| 5694 | Dcaf8 | NM_153555.2 | chr1:172148014-172196393 |
| 5695 | Dcakd | NM_026551.3 | chr11:102994055-103017147 |
| 5696 | Dcbld1 | NM_025705.3 | chr10:52233618-52321377 |
| 5697 | Dcbld2 | NM_028523.3 | chr16:58408534-58469745 |
| 5698 | Dcc | NM_007831.3 | chr18:71253631-72351069 |
| 5699 | Dcdc2a | NM_001195617.1 | chr13:25056003-25121521 |
| 5700 | Dcdc2b | NM_001195730.1 | chr4:129608330-129614257 |
| 5701 | Dcdc2c | NM_001177964.2 | chr12:28437794-28548337 |
| 5702 | Dchs1 | NM_001162943.1 | chr7:105752988-105787550 |
| 5703 | Dck | NM_007832.4 | chr5:88765012-88783277 |
| 5704 | Dclk1 | NM_001111051.1 | chr3:55461757-55539068 |
| 5705 | Dclk2 | NM_001195496.1 | chr3:86786149-86920884 |
| 5706 | Dclk3 | NM_172928.5 | chr9:111439080-111489611 |
| 5707 | Dclre1a | NM_018831.4 | chr19:56529160-56548222 |
| 5708 | Dclre1b | NM_001025312.1 | chr3:103800604-103809387 |
| 5709 | Dclre1c | NM_001110214.1 | chr2:3424130-3461116 |
| 5710 | Dcn | NM_001190451.2 | chr10:97479499-97518163 |
| 5711 | Dcp1a | NM_133761.3 | chr14:30479564-30527056 |
| 5712 | Dcp1b | NM_001033379.3 | chr6:119175252-119221614 |
| 5713 | Dcp2 | NM_027490.1 | chr18:44380499-44424969 |
| 5714 | Dcpp1 | NM_019910.2 | chr17:23880875-23882853 |
| 5715 | Dcpp2 | NM_001039238.2 | chr17:23898721-23900787 |
| 5716 | Dcpp3 | NM_001077633.1 | chr17:23917457-23919441 |
| 5717 | Dcps | NM_027030.2 | chr9:35124413-35175987 |
| 5718 | Dcst1 | NM_029974.2 | chr3:89350218-89365253 |
| 5719 | Dcstamp | NM_001289506.1 | chr15:39745929-39760938 |
| 5720 | Dct | NM_010024.3 | chr14:118012789-118052246 |
| 5721 | Dctd | NM_001161515.1 | chr8:48110012-48141667 |
| 5722 | Dctn1 | NM_001198866.1 | chr6:83165923-83200117 |

| | | | |
|---|---|---|---|
| 5723 | Dctn2 | NM_001190453.1 | chr10:127266261-127281959 |
| 5724 | Dctn3 | NM_001159565.1 | chr4:41714797-41723163 |
| 5725 | Dctn4 | NM_026302.3 | chr18:60526220-60558762 |
| 5726 | Dctn5 | NM_021608.3 | chr7:122133040-122149044 |
| 5727 | Dctn6 | NM_011722.4 | chr8:34090420-34108712 |
| 5728 | Dctpp1 | NM_023203.1 | chr7:127256958-127260667 |
| 5729 | Dcun1d1 | NM_001205361.1 | chr3:35892104-35932966 |
| 5730 | Dcun1d2 | NM_001024504.2 | chr8:13255962-13288126 |
| 5731 | Dcun1d3 | NM_001163703.1 | chr7:119853162-119895745 |
| 5732 | Dcun1d4 | NM_001190733.1 | chr5:73491025-73560794 |
| 5733 | Dcun1d5 | NM_029775.2 | chr9:7184565-7207031 |
| 5734 | Dcx | NM_001110222.1 | chrX:143855841-143933124 |
| 5735 | Dcxr | NM_026428.2 | chr11:120725372-120727281 |
| 5736 | Dda1 | NM_025600.2 | chr8:71469193-71476097 |
| 5737 | Ddah1 | NM_026993.3 | chr3:145758691-145894277 |
| 5738 | Ddah2 | NM_001190449.1 | chr17:35059034-35062099 |
| 5739 | Ddb1 | NM_015735.1 | chr19:10605624-10629821 |
| 5740 | Ddb2 | NM_028119.5 | chr2:91211581-91237066 |
| 5741 | Ddc | NM_001190448.1 | chr11:11814100-11898144 |
| 5742 | Ddhd1 | NM_001039106.3 | chr14:45593170-45658143 |
| 5743 | Ddhd2 | NM_028102.1 | chr8:25725323-25754280 |
| 5744 | Ddi1 | NM_027942.1 | chr9:6265027-6266547 |
| 5745 | Ddi2 | NM_001017966.2 | chr4:141683562-141723419 |
| 5746 | Ddit3 | NM_001290183.1 | chr10:127290792-127296288 |
| 5747 | Ddit4 | NM_029083.2 | chr10:59949674-59951770 |
| 5748 | Ddit4l | NM_030143.4 | chr3:137623671-137628332 |
| 5749 | Ddn | NM_001013741.1 | chr15:98803781-98807925 |
| 5750 | Ddo | NM_027442.5 | chr10:40630010-40649931 |
| 5751 | Ddost | NM_007838.2 | chr4:138304737-138312611 |
| 5752 | Ddr1 | NM_001198831.1 | chr17:35681566-35702044 |
| 5753 | Ddr2 | NM_022563.2 | chr1:169972306-170088944 |
| 5754 | Ddrgk1 | NM_029832.2 | chr2:130654082-130664645 |
| 5755 | Ddt | NM_010027.1 | chr10:75771232-75773374 |
| 5756 | Ddx1 | NM_134040.1 | chr12:13219306-13249174 |
| 5757 | Ddx10 | NM_029936.2 | chr9:53098453-53248112 |
| 5758 | Ddx11 | NM_001003919.1 | chr17:66123519-66152168 |
| 5759 | Ddx17 | NM_001040187.1 | chr15:79527695-79546741 |
| 5760 | Ddx18 | NM_025860.3 | chr1:121553834-121567980 |
| 5761 | Ddx19a | NM_007916.2 | chr8:110974990-110997823 |
| 5762 | Ddx19b | NM_001190786.1 | chr8:111003185-111031751 |
| 5763 | Ddx20 | NM_017397.3 | chr3:105678461-105687571 |
| 5764 | Ddx21 | NM_019553.2 | chr10:62580246-62602298 |
| 5765 | Ddx23 | NM_001080981.1 | chr15:98645506-98662889 |
| 5766 | Ddx24 | NM_001159502.1 | chr12:103407975-103425780 |
| 5767 | Ddx25 | NM_013932.4 | chr9:35541847-35558470 |
| 5768 | Ddx26b | NM_172779.4 | chrX:56454838-56507843 |
| 5769 | Ddx27 | NM_153065.3 | chr2:167015312-167034945 |
| 5770 | Ddx28 | NM_028038.3 | chr8:106009615-106011486 |
| 5771 | Ddx31 | NM_001033294.3 | chr2:28840405-28905575 |
| 5772 | Ddx39 | NM_197982.3 | chr8:83715176-83723351 |
| 5773 | Ddx39b | NM_001252457.1 | chr17:35242816-35253707 |
| 5774 | Ddx3x | NM_010028.3 | chrX:13281021-13293983 |
| 5775 | Ddx3y | NM_012008.2 | chrY:1260714-1286613 |
| 5776 | Ddx4 | NM_001145885.1 | chr13:112598332-112652310 |
| 5777 | Ddx41 | NM_134059.2 | chr13:55530409-55536658 |
| 5778 | Ddx42 | NM_028074.4 | chr11:106216925-106249140 |
| 5779 | Ddx43 | NM_001191044.1 | chr9:78395776-78423589 |
| 5780 | Ddx46 | NM_001282055.1 | chr13:55634998-55681267 |
| 5781 | Ddx47 | NM_026360.3 | chr6:135011611-135023776 |
| 5782 | Ddx49 | NM_001024922.2 | chr8:70292865-70302452 |
| 5783 | Ddx5 | NM_007840.3 | chr11:106780355-106788494 |
| 5784 | Ddx50 | NM_053183.2 | chr10:62616022-62651198 |
| 5785 | Ddx51 | NM_027156.3 | chr5:110653450-110660496 |
| 5786 | Ddx52 | NM_030096.2 | chr11:83942089-83963086 |
| 5787 | Ddx54 | NM_028041.2 | chr5:120613129-120628592 |
| 5788 | Ddx55 | NM_001190795.1 | chr5:124552863-124569660 |
| 5789 | Ddx56 | NM_026538.3 | chr11:6257544-6267729 |
| 5790 | Ddx58 | NM_172689.3 | chr4:40203776-40239825 |
| 5791 | Ddx59 | NM_026500.3 | chr1:136415270-136440220 |
| 5792 | Ddx6 | NM_001110826.1 | chr9:44604891-44640731 |
| 5793 | Ddx60 | NM_001293783.1 | chr8:61928066-62037704 |
| 5794 | Deaf1 | NM_001282072.1 | chr7:141297175-141327725 |
| 5795 | Dear1 | NM_001040461.2 | chr3:84965055-84965580 |
| 5796 | Deb1 | NM_026794.2 | chr9:121710388-121712921 |
| 5797 | Decr1 | NM_026172.3 | chr4:15917239-15945507 |
| 5798 | Decr2 | NM_011933.2 | chr17:26081210-26090164 |
| 5799 | Dedd | NM_001128609.1 | chr1:171329144-171342501 |
| 5800 | Dedd2 | NM_207677.3 | chr7:25202839-25219859 |
| 5801 | Def6 | NM_027185.3 | chr17:28207777-28228608 |
| 5802 | Def8 | NM_001253783.1 | chr8:123442955-123463899 |
| 5803 | Defa17 | NM_001167790.1 | chr8:21655766-21656736 |
| 5804 | Defa2 | NM_001195634.2 | chr8:21378516-21379495 |
| 5805 | Defa20 | NM_183268.4 | chr8:21509257-21510237 |
| 5806 | Defa21 | NM_183253.3 | chr8:21025544-21026517 |
| 5807 | Defa22 | NM_207658.4 | chr8:21162276-21163249 |
| 5808 | Defa23 | NM_001012307.2 | chr8:21055039-21192549 |
| 5809 | Defa24 | NM_001024225.2 | chr8:21734493-21735471 |
| 5810 | Defa25 | NM_007849.1 | chr8:21084441-21085285 |
| 5811 | Defa26 | NM_001079933.2 | chr8:21618167-21618972 |
| 5812 | Defa3 | NM_007850.2 | chr8:21287408-21288377 |
| 5813 | Defa4 | NM_010039.2 | chr8:21065065-21065232 |
| 5814 | Defa5 | NM_007851.2 | chr8:21297393-21298375 |
| 5815 | Defa6 | NM_007852.1 | chr8:21734536-21735377 |
| 5816 | Defa-ps1 | NR_003146.1 | chr8:21695011-21695853 |
| 5817 | Defa-ps12 | NR_002878.2 | chr8:19210461-19212760 |
| 5818 | Defa-ps13 | NR_002881.2 | chr8:19300676-19304794 |
| 5819 | Defa-rs1 | NM_007844.2 | chr8:21325887-21327020 |

Fig.21 - 31

| | | | |
|---|---|---|---|
| 5820 | Defa-rs7 | NM_007848.2 | chr8:21055039-21192550 |
| 5821 | Defb1 | NM_007843.3 | chr8:21776554-21795185 |
| 5822 | Defb10 | NM_139225.1 | chr8:21858900-21862010 |
| 5823 | Defb11 | NM_139221.2 | chr8:21905373-21906432 |
| 5824 | Defb12 | NM_152802.3 | chr8:19111930-19114833 |
| 5825 | Defb13 | NM_139223.3 | chr8:21946761-21948622 |
| 5826 | Defb14 | NM_183026.2 | chr8:19194327-19195309 |
| 5827 | Defb15 | NM_139222.3 | chr8:21929812-21932710 |
| 5828 | Defb18 | NM_001039123.1 | chr1:18236472-18237443 |
| 5829 | Defb19 | NM_145157.3 | chr2:152576085-152580312 |
| 5830 | Defb2 | NM_010030.1 | chr8:21839925-21843481 |
| 5831 | Defb20 | NM_176950.3 | chr2:152477062-152479934 |
| 5832 | Defb21 | NM_207276.2 | chr2:152572743-152574943 |
| 5833 | Defb22 | NM_001002791.2 | chr2:152485665-152490138 |
| 5834 | Defb23 | NM_001037933.2 | chr2:152459054-152464620 |
| 5835 | Defb25 | NM_001039122.1 | chr2:152622355-152623053 |
| 5836 | Defb26 | NM_001039120.2 | chr2:152507755-152511736 |
| 5837 | Defb28 | NM_001037502.2 | chr2:152518254-152521447 |
| 5838 | Defb29 | NM_001001444.2 | chr2:152538713-152540041 |
| 5839 | Defb3 | NM_013756.2 | chr8:19293360-19295339 |
| 5840 | Defb30 | NM_001039566.2 | chr14:63034086-63037846 |
| 5841 | Defb33 | NM_001039119.2 | chr8:20892666-20897723 |
| 5842 | Defb34 | NM_183035.1 | chr8:19123751-19126540 |
| 5843 | Defb35 | NM_139224.1 | chr8:21938351-21940878 |
| 5844 | Defb36 | NM_001037247.4 | chr2:152604326-152612729 |
| 5845 | Defb37 | NM_181683.2 | chr8:18986232-18991055 |
| 5846 | Defb38 | NM_183036.1 | chr8:19023463-19026529 |
| 5847 | Defb39 | NM_183038.2 | chr8:19052825-19064810 |
| 5848 | Defb4 | NM_019728.4 | chr8:19198703-19201547 |
| 5849 | Defb40 | NM_183039.3 | chr8:18974939-18978116 |
| 5850 | Defb41 | NM_183124.3 | chr1:18250977-18265138 |
| 5851 | Defb42 | NM_001034910.3 | chr14:63046990-63048606 |
| 5852 | Defb43 | NM_001039121.1 | chr14:63011770-63018088 |
| 5853 | Defb44-ps | NR_002879.2 | chr1:18210052-18223564 |
| 5854 | Defb45 | NM_001037752.2 | chr2:152593190-152596485 |
| 5855 | Defb46 | NM_001025351.1 | chr8:19239915-19242143 |
| 5856 | Defb47 | NM_001039125.2 | chr14:62998076-63001160 |
| 5857 | Defb48 | NM_001037751.3 | chr14:62977523-62984510 |
| 5858 | Defb5 | NM_030734.2 | chr8:19247591-19250828 |
| 5859 | Defb50 | NM_199067.1 | chr8:21823538-21831296 |
| 5860 | Defb6 | NM_054074.1 | chr8:19225477-19228209 |
| 5861 | Defb7 | NM_139220.1 | chr8:19495096-19497775 |
| 5862 | Defb8 | NM_153108.4 | chr8:19445769-19447606 |
| 5863 | Defb9 | NM_139219.2 | chr8:21881712-21885434 |
| 5864 | Degs1 | NM_007853.4 | chr1:182275769-182282759 |
| 5865 | Degs2 | NM_001171002.1 | chr12:108686791-108702306 |
| 5866 | Dek | NM_025900.2 | chr13:47084766-47106221 |
| 5867 | Dennd1a | NM_146122.3 | chr2:37798989-38287384 |
| 5868 | Dennd1b | NM_001166501.1 | chr1:138963708-139176042 |
| 5869 | Dennd1c | NM_153551.1 | chr17:57066054-57078510 |
| 5870 | Dennd2a | NM_172477.4 | chr6:39462377-39557834 |
| 5871 | Dennd2c | NM_177857.1 | chr3:103127555-103169733 |
| 5872 | Dennd2d | NM_001093754.2 | chr3:106482430-106503030 |
| 5873 | Dennd3 | NM_001081066.1 | chr15:73512559-73572242 |
| 5874 | Dennd4a | NM_001162917.1 | chr9:64811010-64919667 |
| 5875 | Dennd4b | NM_201407.4 | chr3:90266513-90280665 |
| 5876 | Dennd4c | NM_184088.1 | chr4:86748554-86850602 |
| 5877 | Dennd5a | NM_021494.1 | chr7:109893780-109960422 |
| 5878 | Dennd5b | NM_177192.3 | chr6:148988068-149101680 |
| 5879 | Dennd6a | NM_001134465.2 | chr14:26579549-26634322 |
| 5880 | Dennd6b | NM_027081.3 | chr15:89182212-89196474 |
| 5881 | Denr | NM_026603.4 | chr5:123907274-123928832 |
| 5882 | Depdc1a | NM_001172092.1 | chr3:159495432-159529955 |
| 5883 | Depdc1b | NM_178683.4 | chr13:108316336-108389557 |
| 5884 | Depdc5 | NM_001025426.2 | chr5:32863720-32994233 |
| 5885 | Depdc7 | NM_144804.1 | chr2:104721786-104742801 |
| 5886 | Deptor | NM_001037937.3 | chr15:55133435-55259273 |
| 5887 | Dera | NM_172733.1 | chr6:137754576-137837872 |
| 5888 | Derl1 | NM_024207.4 | chr15:57869501-57892418 |
| 5889 | Derl2 | NM_001291146.1 | chr11:71007439-71019841 |
| 5890 | Derl3 | NM_024440.2 | chr10:75893397-75895941 |
| 5891 | Des | NM_010043.2 | chr1:75360291-75368579 |
| 5892 | Desi1 | NM_134095.2 | chr15:81992522-82016140 |
| 5893 | Desi2 | NM_024282.3 | chr1:178187416-178252597 |
| 5894 | Det1 | NM_029585.3 | chr7:78827473-78847211 |
| 5895 | Dexi | NM_021428.4 | chr16:10530206-10543054 |
| 5896 | Dffa | NM_001025296.2 | chr4:149104141-149120653 |
| 5897 | Dffb | NM_007859.4 | chr4:153964448-153975081 |
| 5898 | Dfna5 | NM_018769.3 | chr6:50207402-50261769 |
| 5899 | Dfnb59 | NM_001080711.2 | chr2:76650272-76658554 |
| 5900 | Dgat1 | NM_010046.2 | chr15:76502014-76511818 |
| 5901 | Dgat2 | NM_026384.3 | chr7:99153662-99182713 |
| 5902 | Dgat2l6 | NM_001114084.1 | chrX:100524837-100546108 |
| 5903 | Dgcr14 | NM_001081633.1 | chr16:17900708-17911348 |
| 5904 | Dgcr2 | NM_001109750.1 | chr16:17840355-17891728 |
| 5905 | Dgcr6 | NM_001289813.1 | chr16:18066402-18071632 |
| 5906 | Dgcr8 | NM_033324.2 | chr16:18253964-18289168 |
| 5907 | Dgka | NM_016811.2 | chr10:128720135-128744056 |
| 5908 | Dgkb | NM_178681.4 | chr12:37880704-38633410 |
| 5909 | Dgkd | NM_177646.3 | chr1:87853286-87944489 |
| 5910 | Dgke | NM_019505.3 | chr11:89037581-89060748 |
| 5911 | Dgkeos | NR_110336.1 | chr11:89060750-89067884 |
| 5912 | Dgkg | NM_138650.2 | chr16:22466568-22657231 |
| 5913 | Dgkh | NM_001081336.1 | chr14:78569608-78725089 |
| 5914 | Dgki | NM_001081206.1 | chr6:36846021-37299976 |
| 5915 | Dgkk | NM_177914.3 | chrX:6873483-6948363 |
| 5916 | Dgkq | NM_199011.1 | chr5:108647043-108660769 |
| 5917 | Dgkz | NM_001166597.1 | chr2:91932826-91950348 |
| 5918 | Dguok | NM_001162521.1 | chr6:83480213-83506969 |
| 5919 | Dhcr24 | NM_053272.2 | chr4:106561037-106589113 |
| 5920 | Dhcr7 | NM_007856.2 | chr7:143823166-143848410 |
| 5921 | Dhdds | NM_026144.4 | chr4:133969056-134000864 |
| 5922 | Dhdh | NM_027903.3 | chr7:45473562-45488796 |
| 5923 | Dhfr | NM_010049.3 | chr13:92354782-92389053 |
| 5924 | Dhh | NM_007857.4 | chr15:98893026-98898540 |
| 5925 | Dhodh | NM_020046.3 | chr8:109593247-109608673 |
| 5926 | Dhps | NM_001039514.1 | chr8:85071756-85075161 |
| 5927 | Dhrs1 | NM_026819.2 | chr14:55739019-55745684 |
| 5928 | Dhrs11 | NM_177564.5 | chr11:84820727-84829003 |
| 5929 | Dhrs13 | NM_183286.2 | chr11:78032312-78037864 |
| 5930 | Dhrs2 | NM_027790.2 | chr14:55222006-55241440 |
| 5931 | Dhrs3 | NM_001172424.1 | chr4:144892826-144927645 |
| 5932 | Dhrs4 | NM_001037938.2 | chr14:55478757-55490340 |
| 5933 | Dhrs7 | NM_025522.5 | chr12:72650352-72664828 |
| 5934 | Dhrs7b | NM_001172112.1 | chr11:60830630-60858423 |
| 5935 | Dhrs7c | NM_001013013.2 | chr11:67798270-67816002 |
| 5936 | Dhrs9 | NM_175512.2 | chr2:69380461-69403086 |
| 5937 | Dhrsx | NM_001033326.2 | chr4_GL456216_random:15880-36213 |
| 5938 | Dhtkd1 | NM_001081131.2 | chr2:5898059-5942792 |
| 5939 | Dhx15 | NM_001042620.2 | chr5:52153323-52190546 |
| 5940 | Dhx16 | NM_026987.2 | chr17:35879777-35892668 |
| 5941 | Dhx29 | NM_172594.2 | chr13:112927792-112969187 |
| 5942 | Dhx30 | NM_001252682.1 | chr9:110084318-110117616 |
| 5943 | Dhx32 | NM_001286030.1 | chr7:133720934-133760269 |
| 5944 | Dhx33 | NM_178367.4 | chr11:70984090-71004432 |
| 5945 | Dhx34 | NM_001285931.1 | chr7:16197220-16221993 |
| 5946 | Dhx35 | NM_001291144.1 | chr2:158794806-158858220 |
| 5947 | Dhx36 | NM_028136.2 | chr3:62468641-62506988 |
| 5948 | Dhx37 | NM_203319.1 | chr5:125414403-125434048 |
| 5949 | Dhx38 | NM_178380.1 | chr8:109548023-109565601 |
| 5950 | Dhx40 | NM_026191.2 | chr11:86768848-86807660 |
| 5951 | Dhx57 | NM_001163759.1 | chr17:80238303-80290476 |
| 5952 | Dhx58 | NM_030150.2 | chr11:100694883-100704271 |
| 5953 | Dhx8 | NM_144831.2 | chr11:101732955-101767357 |
| 5954 | Dhx9 | NM_007842.2 | chr1:153455757-153487660 |
| 5955 | Diablo | NM_023232.3 | chr5:123511329-123524164 |
| 5956 | Diap1 | NM_007858.3 | chr18:37843600-37935423 |
| 5957 | Diap2 | NM_172493.2 | chrX:129749741-130465833 |
| 5958 | Diap3 | NM_019670.1 | chr14:86656322-87141114 |
| 5959 | Dicer1 | NM_148948.2 | chr12:104687741-104751952 |
| 5960 | Dido1 | NM_001291432.1 | chr2:180680953-180709999 |
| 5961 | Diexf | NM_145415.2 | chr1:193104402-193130251 |
| 5962 | Dimt1 | NM_025447.4 | chr13:106947128-106960224 |
| 5963 | Dio1 | NM_007860.3 | chr4:107291464-107307143 |
| 5964 | Dio2 | NM_010050.2 | chr12:90724551-90738438 |
| 5965 | Dio3 | NM_172119.2 | chr12:110279229-110281097 |
| 5966 | Dio3os | NR_002866.2 | chr12:110275384-110278068 |
| 5967 | Dip2a | NM_001081419.2 | chr10:76263048-76345291 |
| 5968 | Dip2b | NM_001159361.1 | chr15:100038663-100219473 |
| 5969 | Dip2c | NM_001081426.2 | chr13:9276524-9668926 |
| 5970 | Diras1 | NM_145217.2 | chr10:81019589-81025377 |
| 5971 | Diras2 | NM_001024474.2 | chr13:52504374-52530836 |
| 5972 | Dirc2 | NM_153550.3 | chr16:35694902-35769356 |
| 5973 | Dis3 | NM_028315.2 | chr14:99076633-99099770 |
| 5974 | Dis3l | NM_001001295.2 | chr9:64306755-64341257 |
| 5975 | Dis3l2 | NM_001172157.1 | chr1:86703803-87050097 |
| 5976 | Disc1 | NM_174853.2 | chr8:125054194-125261151 |
| 5977 | Disp1 | NM_001278218.1 | chr1:183086263-183221529 |
| 5978 | Disp2 | NM_170593.3 | chr2:118779718-118795175 |
| 5979 | Dixdc1 | NM_178118.2 | chr9:50662752-50727984 |
| 5980 | Dkc1 | NM_001030307.2 | chrX:75095853-75109776 |
| 5981 | Dkk1 | NM_010051.3 | chr19:30545884-30549496 |
| 5982 | Dkk2 | NM_020265.4 | chr3:132085291-132180304 |
| 5983 | Dkk3 | NM_015814.2 | chr7:112116018-112159057 |
| 5984 | Dkk4 | NM_145592.2 | chr8:22624042-22627546 |
| 5985 | Dkkl1 | NM_015789.3 | chr7:45207524-45211883 |
| 5986 | Dlat | NM_145614.4 | chr9:50634632-50659780 |
| 5987 | Dlc1 | NM_001194940.2 | chr8:36567738-36952442 |
| 5988 | Dld | NM_007861.5 | chr12:31331561-31351471 |
| 5989 | Dlec1 | NM_177117.3 | chr9:119102477-119147694 |
| 5990 | Dleu2 | NR_028264.1 | chr14:61602835-61682373 |
| 5991 | Dleu7 | NM_173419.2 | chr14:62276228-62292979 |
| 5992 | Dlg1 | NM_001252433.1 | chr16:31663934-31873356 |
| 5993 | Dlg2 | NM_001243046.1 | chr7:92062393-92449246 |
| 5994 | Dlg3 | NM_001177778.2 | chrX:100767721-100818410 |
| 5995 | Dlg4 | NM_001109752.1 | chr11:70018604-70045531 |
| 5996 | Dlg5 | NM_001163513.1 | chr14:24133952-24245920 |
| 5997 | Dlgap1 | NM_001128180.1 | chr17:70522109-70821413 |
| 5998 | Dlgap2 | NM_001145965.1 | chr8:14095874-14847686 |
| 5999 | Dlgap3 | NM_198618.5 | chr4:127169203-127237022 |
| 6000 | Dlgap4 | NM_001042487.1 | chr2:156721278-156764363 |
| 6001 | Dlgap5 | NM_144553.2 | chr14:47387778-47418407 |
| 6002 | Dlk1 | NM_001190703.1 | chr12:109453454-109463336 |
| 6003 | Dlk2 | NM_001286013.1 | chr17:46297927-46303271 |
| 6004 | Dll1 | NM_007865.3 | chr17:15367353-15375823 |
| 6005 | Dll3 | NM_007866.2 | chr7:28293554-28301785 |
| 6006 | Dll4 | NM_019454.3 | chr2:119325783-119335666 |
| 6007 | Dlst | NM_030225.4 | chr12:85110832-85134091 |
| 6008 | Dlx1 | NM_010053.2 | chr2:71529444-71533981 |
| 6009 | Dlx1as | NR_002854.2 | chr2:71530637-71537891 |
| 6010 | Dlx2 | NM_010054.2 | chr2:71543407-71546754 |
| 6011 | Dlx3 | NM_010055.3 | chr11:95120116-95125291 |
| 6012 | Dlx4 | NM_007867.4 | chr11:95140446-95145801 |
| 6013 | Dlx5 | NM_010056.3 | chr6:6877800-6882068 |

Fig.21 - 32

| | | | |
|---|---|---|---|
| 6014 | Dlx6 | NM_010057.2 | chr6:6863333-6867970 |
| 6015 | Dlx6as2 | NR_002839.2 | chr6:6863796-6865150 |
| 6016 | Dlx6os1 | NR_015388.1 | chr6:6820545-6869533 |
| 6017 | Dmap1 | NM_023178.2 | chr4:117674685-117682225 |
| 6018 | Dmbt1 | NM_007769.2 | chr7:131032075-131121628 |
| 6019 | Dmbx1 | NM_001025567.1 | chr4:115915118-115939926 |
| 6020 | Dmc1 | NM_001278226.1 | chr15:79561497-79605109 |
| 6021 | Dmd | NM_007868.6 | chrX:82948869-85205050 |
| 6022 | Dmgdh | NM_028772.3 | chr13:93674435-93752823 |
| 6023 | Dmkn | NM_001166173.1 | chr7:30763755-30781066 |
| 6024 | Dmp1 | NM_016779.2 | chr5:104202616-104214102 |
| 6025 | Dmpk | NM_001190490.1 | chr7:19083848-19093820 |
| 6026 | Dmr | NR_102372.1 | chr5:144358524-144361005 |
| 6027 | Dmrt1 | NM_015826.5 | chr19:25505705-25604328 |
| 6028 | Dmrt2 | NM_145831.3 | chr19:25672410-25678991 |
| 6029 | Dmrt3 | NM_177360.3 | chr19:25610536-25623921 |
| 6030 | Dmrta1 | NM_175647.3 | chr4:89688197-89694766 |
| 6031 | Dmrta2 | NM_172296.2 | chr4:109978024-109983684 |
| 6032 | Dmrtb1 | NM_019872.1 | chr4:107676289-107684162 |
| 6033 | Dmrtc1a | NM_001038616.2 | chrX:102903220-102906557 |
| 6034 | Dmrtc1b | NM_001039116.2 | chrX:102707880-102715209 |
| 6035 | Dmrtc1c2 | NM_001142690.2 | chrX:102802962-102854616 |
| 6036 | Dmrtc2 | NM_027732.2 | chr7:24870056-24877651 |
| 6037 | Dmtf1 | NM_001110327.1 | chr5:9118867-9161776 |
| 6038 | Dmtn | NM_001252662.1 | chr14:70602183-70627065 |
| 6039 | Dmwd | NM_010058.2 | chr7:19076199-19082775 |
| 6040 | Dmxl1 | NM_001081371.2 | chr18:49832996-49965473 |
| 6041 | Dmxl2 | NM_172771.2 | chr9:54365157-54501626 |
| 6042 | Dna2 | NM_177372.3 | chr10:62947028-62974188 |
| 6043 | Dnaaf1 | NM_026648.4 | chr8:119575234-119598454 |
| 6044 | Dnaaf2 | NM_027269.4 | chr12:69189086-69198429 |
| 6045 | Dnaaf3 | NM_001033548.2 | chr7:4522956-4532442 |
| 6046 | Dnah1 | NM_001033668.1 | chr14:31260374-31323896 |
| 6047 | Dnah10 | NM_019536.1 | chr5:124725084-124834308 |
| 6048 | Dnah11 | NM_010060.3 | chr12:117877981-118199043 |
| 6049 | Dnah17 | NM_001167746.1 | chr11:118021722-118129219 |
| 6050 | Dnah2 | NM_001081330.1 | chr11:69420808-69549108 |
| 6051 | Dnah5 | NM_133365.3 | chr15:28203765-28472045 |
| 6052 | Dnah6 | NM_001164669.1 | chr6:73017606-73221631 |
| 6053 | Dnah7a | NM_001252070.1 | chr1:53397001-53706784 |
| 6054 | Dnah7b | NM_001160386.1 | chr1:46066737-46373550 |
| 6055 | Dnah8 | NM_013811.3 | chr17:30626935-30875264 |
| 6056 | Dnah9 | NM_001099633.1 | chr11:65831323-66168551 |
| 6057 | Dnaic1 | NM_175138.4 | chr4:41569793-41638158 |
| 6058 | Dnaic2 | NM_001034878.2 | chr11:114727411-114757886 |
| 6059 | Dnaja1 | NM_001164671.2 | chr4:40722921-40734965 |
| 6060 | Dnaja2 | NM_019794.4 | chr8:85537639-85555271 |
| 6061 | Dnaja3 | NM_001135112.1 | chr16:4684069-4707693 |
| 6062 | Dnaja4 | NM_021422.4 | chr9:54699558-54716317 |
| 6063 | Dnajb1 | NM_018808.3 | chr8:83608174-83612653 |
| 6064 | Dnajb11 | NM_001190804.1 | chr16:22857844-22872465 |
| 6065 | Dnajb12 | NM_019965.2 | chr10:59879590-59898016 |
| 6066 | Dnajb13 | NM_153527.2 | chr7:100503019-100514815 |
| 6067 | Dnajb14 | NM_001033155.1 | chr3:137867674-137908931 |
| 6068 | Dnajb2 | NM_001159883.1 | chr1:75236422-75245692 |
| 6069 | Dnajb3 | NM_008299.3 | chr1:88204731-88205748 |
| 6070 | Dnajb4 | NM_025926.4 | chr3:152183870-152193845 |
| 6071 | Dnajb5 | NM_019874.3 | chr4:42953093-42958732 |
| 6072 | Dnajb6 | NM_001037940.4 | chr5:29735897-29786478 |
| 6073 | Dnajb7 | NM_021317.2 | chr15:81407087-81408273 |
| 6074 | Dnajb8 | NM_019964.1 | chr6:88222267-88223256 |
| 6075 | Dnajb9 | NM_013760.4 | chr12:44205896-44210068 |
| 6076 | Dnajc1 | NM_001190817.1 | chr2:18206332-18392830 |
| 6077 | Dnajc10 | NM_024181.2 | chr2:80315465-80354055 |
| 6078 | Dnajc11 | NM_172704.1 | chr4:151933719-151981959 |
| 6079 | Dnajc12 | NM_001253685.1 | chr10:63382442-63408840 |
| 6080 | Dnajc13 | NM_001163026.1 | chr9:104151596-104262930 |
| 6081 | Dnajc14 | NM_028873.4 | chr10:128805675-128819446 |
| 6082 | Dnajc15 | NM_025384.3 | chr14:77826216-77874917 |
| 6083 | Dnajc16 | NM_172338.2 | chr4:141761997-141790644 |
| 6084 | Dnajc17 | NM_139139.2 | chr2:119172499-119208795 |
| 6085 | Dnajc18 | NM_029669.4 | chr18:35671104-35703144 |
| 6086 | Dnajc19 | NM_001026211.2 | chr3:34057279-34081354 |
| 6087 | Dnajc2 | NM_009584.4 | chr5:21757276-21785165 |
| 6088 | Dnajc21 | NM_030046.2 | chr15:10446762-10470516 |
| 6089 | Dnajc22 | NM_176835.2 | chr15:99099483-99104707 |
| 6090 | Dnajc24 | NM_026992.3 | chr2:105966707-106003549 |
| 6091 | Dnajc25 | NM_001033165.3 | chr4:59003192-59023398 |
| 6092 | Dnajc27 | NM_153082.4 | chr12:4082573-4110612 |
| 6093 | Dnajc28 | NM_001099738.1 | chr16:91614256-91618999 |
| 6094 | Dnajc3 | NM_008929.3 | chr14:118937931-118981702 |
| 6095 | Dnajc30 | NM_025362.3 | chr5:135064205-135065365 |
| 6096 | Dnajc4 | NM_020566.1 | chr19:6987910-6992272 |
| 6097 | Dnajc5 | NM_001271584.1 | chr2:181520484-181552880 |
| 6098 | Dnajc5b | NM_001163536.1 | chr3:19508594-19610862 |
| 6099 | Dnajc5g | NM_177677.3 | chr5:31108318-31112524 |
| 6100 | Dnajc6 | NM_001164583.1 | chr4:101550593-101642799 |
| 6101 | Dnajc7 | NM_019795.4 | chr11:100582835-100620168 |
| 6102 | Dnajc8 | NM_172400.3 | chr4:132535558-132553742 |
| 6103 | Dnajc9 | NM_134081.5 | chr14:20384637-20388910 |
| 6104 | Dnal1 | NM_028821.3 | chr12:84114327-84143510 |
| 6105 | Dnal4 | NM_017470.2 | chr15:79761448-79774467 |
| 6106 | Dnali1 | NM_175223.4 | chr4:125055338-125065657 |
| 6107 | Dnase1 | NM_010061.5 | chr16:4037144-4040024 |
| 6108 | Dnase1l1 | NM_001172154.1 | chrX:74273216-74282333 |
| 6109 | Dnase1l2 | NM_025718.3 | chr17:24440767-24443101 |
| 6110 | Dnase1l3 | NM_007870.3 | chr14:7965189-7994182 |
| 6111 | Dnase2a | NM_010062.3 | chr8:84908623-84911461 |
| 6112 | Dnase2b | NM_019957.4 | chr3:146581372-146615598 |
| 6113 | Dnd1 | NM_173383.2 | chr18:36763670-36766214 |
| 6114 | Dner | NM_152915.1 | chr1:84369838-84696221 |
| 6115 | Dnlz | NM_001139503.1 | chr2:26348117-26352110 |
| 6116 | Dnm1 | NM_010065.3 | chr2:32308470-32353329 |
| 6117 | Dnm1l | NM_001025947.2 | chr16:16312227-16359031 |
| 6118 | Dnm2 | NM_001039520.2 | chr9:21424907-21507146 |
| 6119 | Dnm3 | NM_001038619.1 | chr1:161987301-162478030 |
| 6120 | Dnm3os | NR_002870.2 | chr1:162217622-162225550 |
| 6121 | Dnmbp | NM_028029.4 | chr19:43846814-43940198 |
| 6122 | Dnmt1 | NM_001199431.1 | chr9:20907205-20952979 |
| 6123 | Dnmt3a | NM_001271753.1 | chr12:3807159-3914443 |
| 6124 | Dnmt3aos | NR_045884.1 | chr12:3859292-3862244 |
| 6125 | Dnmt3b | NM_001003960.4 | chr2:153649448-153687730 |
| 6126 | Dnmt3l | NM_001081695.2 | chr10:78042286-78063622 |
| 6127 | Dnpep | NM_001110831.1 | chr1:75308564-75317637 |
| 6128 | Dnph1 | NM_207161.3 | chr17:46496788-46499618 |
| 6129 | Dntt | NM_001043228.1 | chr19:41029274-41059525 |
| 6130 | Dnttip1 | NM_133763.1 | chr2:164746014-164768219 |
| 6131 | Dnttip2 | NM_153806.1 | chr3:122274413-122285271 |
| 6132 | Doc2a | NM_010069.1 | chr7:126847552-126852705 |
| 6133 | Doc2b | NM_007873.3 | chr11:75768270-75796060 |
| 6134 | Doc2g | NM_021791.3 | chr19:4003384-4007005 |
| 6135 | Dock1 | NM_001033420.2 | chr7:134670686-135173647 |
| 6136 | Dock10 | NM_001285927.1 | chr1:80501067-80665352 |
| 6137 | Dock11 | NM_001009947.3 | chrX:35888831-36076562 |
| 6138 | Dock2 | NM_033374.3 | chr11:34226820-34783905 |
| 6139 | Dock3 | NM_153413.2 | chr9:106892824-107231909 |
| 6140 | Dock4 | NM_172803.2 | chr12:40446052-40846488 |
| 6141 | Dock5 | NM_177780.3 | chr14:67751927-67933572 |
| 6142 | Dock6 | NM_177030.3 | chr9:21800179-21852635 |
| 6143 | Dock7 | NM_001290636.1 | chr4:98936658-99120915 |
| 6144 | Dock8 | NM_028785.3 | chr19:24999528-25202432 |
| 6145 | Dock9 | NM_001081039.1 | chr14:121542038-121698417 |
| 6146 | Dohh | NM_133964.2 | chr10:81384427-81388352 |
| 6147 | Dok1 | NM_001291799.1 | chr6:83030935-83033471 |
| 6148 | Dok2 | NM_010071.2 | chr14:70774380-70778494 |
| 6149 | Dok3 | NM_013739.2 | chr13:55523234-55528538 |
| 6150 | Dok4 | NM_053246.3 | chr8:94863827-94876312 |
| 6151 | Dok5 | NM_001163686.1 | chr2:170731806-170879775 |
| 6152 | Dok6 | NM_001039173.1 | chr18:89301134-89769136 |
| 6153 | Dok7 | NM_172708.3 | chr5:35057083-35087831 |
| 6154 | Dolk | NM_177648.3 | chr2:30284228-30286354 |
| 6155 | Dolpp1 | NM_001290508.1 | chr2:30392253-30400529 |
| 6156 | Donson | NM_021720.1 | chr16:91679264-91688728 |
| 6157 | Dopey1 | NM_177208.3 | chr9:86467153-86555806 |
| 6158 | Dopey2 | NM_026700.2 | chr16:93711906-93740278 |
| 6159 | Dos | NM_001195268.1 | chr10:80130433-80137387 |
| 6160 | Dot1l | NM_199322.1 | chr10:80755205-80794347 |
| 6161 | Doxl2 | NM_001029987.1 | chr6:48975142-48978745 |
| 6162 | Dpagt1 | NM_007875.2 | chr9:44326844-44333600 |
| 6163 | Dpcd | NM_172639.2 | chr19:45560614-45578287 |
| 6164 | Dpcr1 | NM_001033366.3 | chr17:35635754-35643695 |
| 6165 | Dpep1 | NM_007876.2 | chr8:123186234-123201813 |
| 6166 | Dpep2 | NM_176913.4 | chr8:105984945-105996423 |
| 6167 | Dpep3 | NM_027960.2 | chr8:105973519-105979419 |
| 6168 | Dpf1 | NM_013874.2 | chr7:29304004-29317586 |
| 6169 | Dpf2 | NM_001291078.1 | chr19:5896515-5912871 |
| 6170 | Dpf3 | NM_001267625.1 | chr12:83213750-83487736 |
| 6171 | Dph1 | NM_144491.2 | chr11:75177642-75190483 |
| 6172 | Dph2 | NM_026344.3 | chr4:117888642-117892003 |
| 6173 | Dph3 | NM_001047433.2 | chr14:32080516-32085692 |
| 6174 | Dph5 | NM_027193.2 | chr3:115888182-115929323 |
| 6175 | Dph6 | NM_025675.4 | chr2:114516417-114654928 |
| 6176 | Dph7 | NM_026044.3 | chr2:24962421-24973471 |
| 6177 | Dpm1 | NM_010072.4 | chr2:168209047-168230379 |
| 6178 | Dpm2 | NM_010073.2 | chr2:32570857-32573571 |
| 6179 | Dpm3 | NM_026767.4 | chr3:89266460-89267079 |
| 6180 | Dpp10 | NM_199021.3 | chr1:123332137-124045559 |
| 6181 | Dpp3 | NM_133803.2 | chr19:4907228-4928287 |
| 6182 | Dpp4 | NM_001159543.1 | chr2:62333222-62412231 |
| 6183 | Dpp6 | NM_001136060.2 | chr5:27261934-27727500 |
| 6184 | Dpp7 | NM_031843.2 | chr2:25352289-25356332 |
| 6185 | Dpp8 | NM_028906.2 | chr9:65032457-65082651 |
| 6186 | Dpp9 | NM_172624.3 | chr17:56186681-56218889 |
| 6187 | Dppa1 | NM_001163358.1 | chr11:46607009-46629274 |
| 6188 | Dppa2 | NM_028615.1 | chr16:48310273-48319513 |
| 6189 | Dppa3 | NM_139218.1 | chr6:122626423-122630271 |
| 6190 | Dppa4 | NM_001018002.1 | chr16:48283734-48294292 |
| 6191 | Dppa5a | NM_025274.3 | chr9:78367053-78368199 |
| 6192 | Dpt | NM_019759.2 | chr1:164796731-164824266 |
| 6193 | Dpy19l1 | NM_172920.4 | chr9:24411778-24503140 |
| 6194 | Dpy19l2 | NM_001166207.1 | chr9:24557047-24696293 |
| 6195 | Dpy19l3 | NM_178704.3 | chr7:35685499-35754454 |
| 6196 | Dpy19l4 | NM_001081201.1 | chr4:11265078-11322131 |
| 6197 | Dpy30 | NM_001146222.1 | chr17:74299473-74316394 |
| 6198 | Dpyd | NM_170778.2 | chr3:118562177-119432918 |
| 6199 | Dpys | NM_001164466.1 | chr15:39782696-39857470 |
| 6200 | Dpysl2 | NM_009955.3 | chr14:66802863-66868600 |
| 6201 | Dpysl3 | NM_001136086.2 | chr18:43320978-43373272 |
| 6202 | Dpysl4 | NM_011993.4 | chr7:139086000-139101791 |
| 6203 | Dpysl5 | NM_023047.2 | chr5:30711894-30799369 |
| 6204 | DQ267100 | NR_046304.1 | chr12:109649616-109649687 |
| 6205 | DQ267101 | NR_046305.1 | chr12:109651423-109651495 |
| 6206 | DQ267102 | NR_046306.1 | chr12:109656344-109656415 |
| 6207 | Dqx1 | NM_033606.3 | chr6:83057843-83067219 |

Fig.21 - 33

| | | | | | | | |
|---|---|---|---|---|---|---|---|
| 6208 | Dr1 | NM_026106.4 | chr5:108268896-108280521 | 6305 | Dym | NM_027727.2 | chr18:75018771-75286966 |
| 6209 | Dram1 | NM_027878.2 | chr10:88322803-88357075 | 6306 | Dynap | NM_029346.1 | chr18:70240428-70244584 |
| 6210 | Dram2 | NM_001025582.2 | chr3:106547797-106575341 | 6307 | Dync1h1 | NM_030238.2 | chr12:110601394-110666944 |
| 6211 | Drap1 | NM_001291080.1 | chr19:5422803-5424979 | 6308 | Dync1i1 | NM_001191023.1 | chr6:5725638-6028039 |
| 6212 | Draxin | NM_027426.3 | chr4:148098436-148130698 | 6309 | Dync1i2 | NM_001198872.1 | chr2:71211705-71263302 |
| 6213 | Drc1 | NM_001033460.3 | chr5:30341662-30366708 | 6310 | Dync1li1 | NM_146229.2 | chr9:114688830-114723777 |
| 6214 | Drd1a | NM_001291801.1 | chr13:54051183-54054780 | 6311 | Dync1li2 | NM_001013380.2 | chr8:104417673-104443047 |
| 6215 | Drd2 | NM_010077.2 | chr9:49340661-49407214 | 6312 | Dync2h1 | NM_029851.2 | chr9:6928502-7177046 |
| 6216 | Drd3 | NM_007877.1 | chr16:43762241-43822839 | 6313 | Dync2li1 | NM_172256.1 | chr17:84626498-84655564 |
| 6217 | Drd4 | NM_007878.2 | chr7:141292005-141295001 | 6314 | Dynll1 | NM_019682.4 | chr5:115297109-115300990 |
| 6218 | Drd5 | NM_013503.3 | chr5:38319508-38322523 | 6315 | Dynll2 | NM_001168471.1 | chr11:87979524-87998298 |
| 6219 | Dreh | NR_105051.1 | chr17:64767110-64767931 | 6316 | Dynlrb1 | NM_001291108.1 | chr2:155236607-155250277 |
| 6220 | Drg1 | NM_007879.1 | chr11:3249921-3266386 | 6317 | Dynlrb2 | NM_029297.1 | chr8:116505014-116515915 |
| 6221 | Drg2 | NM_021354.1 | chr11:60454616-60468705 | 6318 | Dynlt1a | NM_001166629.2 | chr17:6306343-6317474 |
| 6222 | Drosha | NM_001130149.1 | chr15:12824814-12935291 | 6319 | Dynlt1b | NM_009342.2 | chr17:6430111-6436295 |
| 6223 | Drp2 | NM_010078.3 | chrX:134404779-134456573 | 6320 | Dynlt1c | NM_001166630.1 | chr17:6601778-6655831 |
| 6224 | Dsc1 | NM_001291804.1 | chr18:20083470-20114773 | 6321 | Dynlt1f | NM_001166627.1 | chr17:6601670-6655939 |
| 6225 | Dsc2 | NM_013505.3 | chr18:20030797-20059505 | 6322 | Dynlt3 | NM_025975.5 | chrX:9654269-9662983 |
| 6226 | Dsc3 | NM_001291809.1 | chr18:19960929-20002097 | 6323 | Dyrk1a | NM_001113389.1 | chr16:94570906-94695519 |
| 6227 | Dscam | NM_031174.4 | chr16:96592078-97170735 | 6324 | Dyrk1b | NM_001037957.3 | chr7:28179482-28187298 |
| 6228 | Dscaml1 | NM_001081270.1 | chr9:45430292-45753713 | 6325 | Dyrk2 | NM_001014390.2 | chr10:118859348-118868903 |
| 6229 | Dscc1 | NM_183089.2 | chr15:55076100-55090478 | 6326 | Dyrk3 | NM_145508.2 | chr1:131128440-131138234 |
| 6230 | Dscr3 | NM_007834.3 | chr16:94497723-94526629 | 6327 | Dyrk4 | NM_207210.2 | chr6:126876198-126921839 |
| 6231 | Dse | NM_172508.2 | chr10:34151392-34207551 | 6328 | Dysf | NM_001077694.2 | chr6:84008597-84211059 |
| 6232 | Dsel | NM_001081316.1 | chr1:111858701-111864918 | 6329 | Dytn | NM_001081658.1 | chr1:63622850-63686927 |
| 6233 | Dsg1a | NM_010079.2 | chr18:20310872-20343353 | 6330 | Dyx1c1 | NM_001163725.1 | chr9:72958784-72973067 |
| 6234 | Dsg1b | NM_181682.1 | chr18:20376834-20409741 | 6331 | Dzank1 | NM_172859.2 | chr2:144470556-144527398 |
| 6235 | Dsg1c | NM_181680.1 | chr18:20247339-20283923 | 6332 | Dzip1 | NM_025943.3 | chr14:118875519-118925470 |
| 6236 | Dsg2 | NM_007883.2 | chr18:20558115-20604526 | 6333 | Dzip1l | NM_028258.4 | chr9:99629530-99670075 |
| 6237 | Dsg3 | NM_030596.3 | chr18:20510303-20541310 | 6334 | Dzip3 | NM_001110017.1 | chr16:48924227-48994112 |
| 6238 | Dsg4 | NM_181564.2 | chr18:20436174-20471821 | 6335 | E030002O03Rik | NM_172905.2 | chr7:104153012-104164816 |
| 6239 | Dsn1 | NM_025853.3 | chr2:156995061-157007075 | 6336 | E030003E18Rik | NR_015502.1 | chr19:20492714-20556410 |
| 6240 | Dsp | NM_023842.2 | chr13:38151293-38198577 | 6337 | E030011O05Rik | NR_015511.2 | chr9:96731574-96752831 |
| 6241 | Dspp | NM_010080.2 | chr5:104170711-104180127 | 6338 | E030013I19Rik | NR_040353.1 | chr2:12301245-12312315 |
| 6242 | Dst | NM_001276764.1 | chr1:33908224-34308662 | 6339 | E030018B13Rik | NM_001256311.1 | chr7:63916856-63920539 |
| 6243 | Dstn | NM_019771.2 | chr2:143915330-143943324 | 6340 | E030019B06Rik | NM_001302546.1 | chr7:139600914-139683815 |
| 6244 | Dstyk | NM_172516.4 | chr1:132417452-132466959 | 6341 | E030019B13Rik | NR_045082.1 | chr12:56490427-56529420 |
| 6245 | Dtd1 | NM_025314.3 | chr2:144599952-144768747 | 6342 | E030024N20Rik | NR_033228.1 | chr19:16164097-16170458 |
| 6246 | Dtd2 | NM_029545.2 | chr12:51997506-52006501 | 6343 | E030025P04Rik | NR_037978.1 | chr11:109139290-109144369 |
| 6247 | Dthd1 | NM_001170705.1 | chr5:62813822-62888318 | 6344 | E030030I06Rik | NM_001254744.1 | chr10:22113049-22149270 |
| 6248 | Dtl | NM_029766.3 | chr1:191537355-191575544 | 6345 | E030044B06Rik | NR_045343.1 | chr19:40868315-40882639 |
| 6249 | Dtna | NM_001285807.1 | chr18:23415408-23659719 | 6346 | E130006D01Rik | NR_045832.1 | chr5:111734283-111761728 |
| 6250 | Dtnb | NM_001162465.1 | chr12:3572390-3781398 | 6347 | E130008D07Rik | NR_045153.1 | chr17:43149356-43158291 |
| 6251 | Dtnbp1 | NM_025772.4 | chr13:44922079-45002096 | 6348 | E130012A19Rik | NM_175332.3 | chr11:97627386-97629716 |
| 6252 | Dtwd1 | NM_026981.2 | chr2:126152140-126165277 | 6349 | E130018N17Rik | NR_040327.1 | chr2:168152602-168154513 |
| 6253 | Dtwd2 | NM_001170960.1 | chr18:49696144-49755601 | 6350 | E130102H24Rik | NR_040708.1 | chr4:101346523-101356248 |
| 6254 | Dtx1 | NM_008052.3 | chr5:120680263-120711669 | 6351 | E130112N10Rik | NR_015604.2 | chr6:125231275-125239934 |
| 6255 | Dtx2 | NM_001256096.1 | chr5:135994799-136032880 | 6352 | E130114P18Rik | NR_015513.2 | chr4:97567874-97584591 |
| 6256 | Dtx3 | NM_030714.2 | chr10:127190377-127195709 | 6353 | E130201H02Rik | NR_024324.1 | chr7:120597624-120598475 |
| 6257 | Dtx3l | NM_001013371.2 | chr16:35926514-35939027 | 6354 | E130215H24Rik | NR_040331.1 | chr2:150667493-150668932 |
| 6258 | Dtx4 | NM_172442.3 | chr19:12466335-12501996 | 6355 | E130218I03Rik | NR_040435.1 | chr4:134243762-134245873 |
| 6259 | Dtymk | NM_001105667.1 | chr1:93792575-93801934 | 6356 | E130304I02Rik | NR_033567.1 | chr7:35802591-35838074 |
| 6260 | Duox1 | NM_001099297.1 | chr2:122315671-122347972 | 6357 | E130307A14Rik | NR_038037.1 | chr10:39621410-39732007 |
| 6261 | Duox2 | NM_177610.2 | chr2:122280436-122298165 | 6358 | E130308A19Rik | NM_001015681.2 | chr4:59626210-59754303 |
| 6262 | Duoxa1 | NM_001305262.1 | chr2:122303540-122313744, | 6359 | E130309D02Rik | NM_172726.4 | chr5:143301071-143315360 |
| 6263 | Duoxa2 | NM_025777.2 | chr2:122298899-122302885 | 6360 | E130309D14Rik | NM_001013784.1 | chr11:74619604-74641516 |
| 6264 | Dupd1 | NM_001013826.2 | chr14:21676571-21714576 | 6361 | E130309F12Rik | NM_178756.4 | chr4:49059461-49340261 |
| 6265 | Dus1l | NM_026824.4 | chr11:120789201-120796395 | 6362 | E130310I04Rik | NR_045722.1 | chr16:34952025-34958864 |
| 6266 | Dus2 | NM_025518.2 | chr8:106011477-106053840 | 6363 | E130311K13Rik | NM_177856.4 | chr3:63914695-63929385 |
| 6267 | Dus3l | NM_144858.2 | chr17:56764750-56770093 | 6364 | E130317F20Rik | NR_029447.1 | chr10:79851376-79854971 |
| 6268 | Dus4l | NM_028002.2 | chr12:31640054-31654826 | 6365 | E230008N13Rik | NM_198660.2 | chr4:45890302-45950774 |
| 6269 | Dusp1 | NM_013642.3 | chr17:26505590-26508472 | 6366 | E230016K23Rik | NR_036452.1 | chr11:83582060-83623693 |
| 6270 | Dusp10 | NM_022019.5 | chr1:184034460-184075636 | 6367 | E230016M11Rik | NR_040278.1 | chr6:67036598-67080652 |
| 6271 | Dusp11 | NM_028099.4 | chr6:85942268-85961667 | 6368 | E230019M04Rik | NM_177921.3 | chrX:140062878-140106797 |
| 6272 | Dusp12 | NM_023173.2 | chr1:170874187-170885540 | 6369 | E230025N22Rik | NM_172831.2 | chr18:36684922-36695925 |
| 6273 | Dusp13 | NM_001007268.1 | chr14:21733394-21748622 | 6370 | E230029C05Rik | NR_110364.1 | chr7:90029158-90049071 |
| 6274 | Dusp14 | NM_019819.3 | chr11:84048044-84068357 | 6371 | E2f1 | NM_001291105.1 | chr2:154559399-154569426 |
| 6275 | Dusp15 | NM_001159376.1 | chr2:152940994-152951582 | 6372 | E2f2 | NM_177733.7 | chr4:136172273-136196056 |
| 6276 | Dusp16 | NM_001048054.2 | chr6:134715467-134792628 | 6373 | E2f3 | NM_001289920.1 | chr13:29906574-29984391 |
| 6277 | Dusp18 | NM_173745.5 | chr11:3895239-3901296 | 6374 | E2f4 | NM_148952.1 | chr8:105297662-105305370 |
| 6278 | Dusp19 | NM_024438.4 | chr2:80617213-80631661 | 6375 | E2f5 | NM_007892.2 | chr3:14578670-14606309 |
| 6279 | Dusp2 | NM_010090.2 | chr2:127336158-127338377 | 6376 | E2f6 | NM_033270.2 | chr12:16810964-16826752 |
| 6280 | Dusp21 | NM_028568.1 | chrX:18145869-18146692 | 6377 | E2f7 | NM_178609.4 | chr10:110745464-110787384 |
| 6281 | Dusp22 | NM_001037955.4 | chr13:30660058-30711232 | 6378 | E2f8 | NM_001013368.5 | chr7:48866428-48881041 |
| 6282 | Dusp23 | NM_026725.2 | chr1:172630768-172632974 | 6379 | E330009J07Rik | NM_175528.4 | chr6:40407497-40436133 |
| 6283 | Dusp26 | NM_025869.3 | chr8:31089661-31097047 | 6380 | E330011O21Rik | NR_045698.1 | chr16:78250862-78255454 |
| 6284 | Dusp27 | NM_001033344.3 | chr1:166098147-166127898 | 6381 | E330012B07Rik | NR_033640.1 | chr6:147180240-147208294 |
| 6285 | Dusp28 | NM_175118.3 | chr1:92906988-92908620 | 6382 | E330013P04Rik | NR_026942.1 | chr19:60144674-60162591 |
| 6286 | Dusp3 | NM_028207.3 | chr11:101971143-101984791 | 6383 | E330014E10Rik | NM_001122668.1 | chr5:82563-95804230 |
| 6287 | Dusp4 | NM_176933.4 | chr8:34807609-34819894 | 6384 | E330017A01Rik | NM_175011.2 | chr16:58635261-58638403 |
| 6288 | Dusp5 | NM_001085390.1 | chr19:53529317-53541322 | 6385 | E330017L17Rik | NR_045190.2 | chr4:129906225-129908996 |
| 6289 | Dusp6 | NM_026268.3 | chr10:99263230-99267489 | 6386 | E330020D12Rik | NR_033736.1 | chr1:153404185-153414233 |
| 6290 | Dusp7 | NM_153459.4 | chr9:106368631-106375723 | 6387 | E330021D16Rik | NM_001201390.1 | chr6:136400318-136415569 |
| 6291 | Dusp8 | NM_008748.3 | chr7:142079486-142095284 | 6388 | E330023G01Rik | NR_045332.1 | chr9:98748598-98820087 |
| 6292 | Dusp9 | NM_029352.3 | chrX:73639440-73643514 | 6389 | E330033B04Rik | NR_030690.1 | chr15:96268563-96275275 |
| 6293 | Dut | NM_001159646.1 | chr2:125247247-125259049 | 6390 | E330034G19Rik | NM_001033214.2 | chr14:24293363-24309959 |
| 6294 | Dux | NM_001081954.1 | chr10:58230650-58232675 | 6391 | E430016F16Rik | NR_015542.1 | chr7:78581065-78718220 |
| 6295 | Duxbl1 | NM_183389.1 | chr14:25983004-26269100 | 6392 | E430018J23Rik | NM_198011.2 | chr7:127390835-127393621 |
| 6296 | Duxbl2 | NM_001177538.1 | chr14:25983004-26269434 | 6393 | E430025E21Rik | NM_153548.2 | chr15:59331996-59374152 |
| 6297 | Duxbl3 | NM_001177539.1 | chr14:26258303-26269434 | 6394 | E4f1 | NM_007893.4 | chr17:24443777-24455392 |
| 6298 | Dvl1 | NM_010091.4 | chr4:155847316-155859303 | 6395 | E530001F21Rik | NR_002167.2 | chrX:105164377-105165505 |
| 6299 | Dvl2 | NM_007888.3 | chr11:70000625-70010109 | 6396 | E530011L22Rik | NR_033503.1 | chr9:121756639-121759943 |
| 6300 | Dvl3 | NM_007889.2 | chr16:20517063-20532187 | 6397 | Eaf1 | NM_028932.4 | chr14:31495078-31509858 |
| 6301 | DXBay18 | NM_001025384.3 | chrX:73117046-73149450 | 6398 | Eaf2 | NM_001113401.1 | chr16:36792883-36828259 |
| 6302 | Dxo | NM_001163770.1 | chr17:34837018-34839186 | 6399 | Eapp | NM_025456.3 | chr12:54673466-54695865 |
| 6303 | Dydc1 | NM_027094.1 | chr14:41072910-41092197 | 6400 | Ear1 | NM_007894.2 | chr14:43818764-43819639 |
| 6304 | Dydc2 | NM_027717.1 | chr14:41049208-41069074 | 6401 | Ear10 | NM_053112.1 | chr14:43922897-43923368 |

Fig.21 - 34

| | | | | | | | |
|---|---|---|---|---|---|---|---|
| 6402 | Ear14 | NM_017389.2 | chr14:51203694-51204156 | 6499 | Eftud1 | NM_001159672.1 | chr7:82648613-82777852 |
| 6403 | Ear2 | NM_007895.2 | chr14:44102653-44103531 | 6500 | Eftud2 | NM_001109995.1 | chr11:102838471-102880975 |
| 6404 | Ear3 | NM_017388.1 | chr14:44102886-44103357 | 6501 | Egf | NM_010113.4 | chr3:129677576-129755322 |
| 6405 | Ear4 | NM_001017422.1 | chr14:51203689-51204156 | 6502 | Egfbp2 | NM_010115.6 | chr7:44012696-44016968 |
| 6406 | Ear6 | NM_053111.2 | chr14:51853767-51854642 | 6503 | Egfem1 | NM_001167748.1 | chr3:29082576-29691056 |
| 6407 | Ear7 | NM_017385.1 | chr14:51853997-51854465 | 6504 | Egfl6 | NM_019397.3 | chrX:166523006-166585716 |
| 6408 | Ears2 | NM_026140.3 | chr7:122034161-122067086 | 6505 | Egfl7 | NM_001164564.1 | chr2:26586629-26592682 |
| 6409 | Ebag9 | NM_019480.4 | chr15:44619640-44641026 | 6506 | Egfl8 | NM_152922.3 | chr17:34613349-34615971 |
| 6410 | Ebf1 | NM_001290709.1 | chr11:44618099-45008096 | 6507 | Egflam | NM_001289496.1 | chr15:7206119-7398390 |
| 6411 | Ebf2 | NM_001276387.1 | chr14:67233291-67430952 | 6508 | Egfr | NM_007912.4 | chr11:16752202-16887658 |
| 6412 | Ebf3 | NM_001113414.1 | chr7:137193670-137314445 | 6509 | Egln1 | NM_053207.2 | chr8:124908586-124949254 |
| 6413 | Ebf4 | NM_001110513.1 | chr2:130295938-130370481 | 6510 | Egln2 | NM_053208.4 | chr7:27158657-27166802 |
| 6414 | Ebi3 | NM_015766.2 | chr17:55952622-55957022 | 6511 | Egln3 | NM_028133.2 | chr12:54178980-54203874 |
| 6415 | Ebna1bp2 | NM_026932.4 | chr4:118620798-118627776 | 6512 | Egr1 | NM_007913.5 | chr18:34861206-34864956 |
| 6416 | Ebp | NM_007898.3 | chrX:8185328-8193512 | 6513 | Egr2 | NM_010118.3 | chr10:67537868-67542188 |
| 6417 | Ebpl | NM_026598.3 | chr14:61339762-61360445 | 6514 | Egr3 | NM_001289925.1 | chr14:70077444-70082613 |
| 6418 | Ecd | NM_027475.3 | chr14:20319858-20348121 | 6515 | Egr4 | NM_020596.2 | chr6:85511121-85513542 |
| 6419 | Ece1 | NM_199307.2 | chr4:137862236-137965229 | 6516 | Ehbp1 | NM_001252515.1 | chr11:22005825-22286795 |
| 6420 | Ece2 | NM_025462.2 | chr16:20611600-20618869 | 6517 | Ehbp1l1 | NM_001114595.1 | chr19:5707373-5726317 |
| 6421 | Ecel1 | NM_001277925.1 | chr1:87147654-87156136 | 6518 | Ehd1 | NM_010119.5 | chr19:6276895-6300096 |
| 6422 | Ech1 | NM_016772.1 | chr7:28825337-28832239 | 6519 | Ehd2 | NM_153068.3 | chr7:15948986-15967535 |
| 6423 | Echdc1 | NM_001110195.1 | chr10:29313165-29346661 | 6520 | Ehd3 | NM_020578.3 | chr17:73804840-73832093 |
| 6424 | Echdc2 | NM_001254754.1 | chr4:108165436-108179308 | 6521 | Ehd4 | NM_133838.4 | chr2:120089486-120154575 |
| 6425 | Echdc3 | NM_024208.4 | chr2:6188464-6212994 | 6522 | Ehf | NM_007914.3 | chr2:103263432-103303196 |
| 6426 | Echs1 | NM_053119.2 | chr7:140105722-140116423 | 6523 | Ehhadh | NM_023737.3 | chr16:21761284-21787834 |
| 6427 | Eci1 | NM_010023.4 | chr17:24426682-24439316 | 6524 | Ehmt1 | NM_001012518.3 | chr2:24790768-24919609 |
| 6428 | Eci2 | NM_001110331.1 | chr13:34977747-34994144 | 6525 | Ehmt2 | NM_001286573.1 | chr17:34898973-34914050 |
| 6429 | Eci3 | NM_026947.4 | chr13:34946613-34963809 | 6526 | Ei24 | NM_001199494.1 | chr9:36779152-36797393 |
| 6430 | Ecm1 | NM_001252653.1 | chr3:95734147-95739569 | 6527 | Eid1 | NM_025613.3 | chr2:125673099-125675642 |
| 6431 | Ecm2 | NM_001012324.2 | chr13:49504809-49532789 | 6528 | Eid2 | NM_198425.2 | chr7:28267880-28269168 |
| 6432 | Ecscr | NM_001033141.1 | chr18:35713088-35721491 | 6529 | Eid2b | NM_001177427.1 | chr7:28277705-28280129 |
| 6433 | Ecsit | NM_001253897.1 | chr9:22072245-22085427 | 6530 | Eid3 | NM_025499.2 | chr10:82866625-82867929 |
| 6434 | Ect2 | NM_001177625.1 | chr3:27097221-27153854 | 6531 | Eif1 | NM_011508.1 | chr11:100319995-100322096 |
| 6435 | Ect2l | NM_001195036.1 | chr10:18129021-18210890 | 6532 | Eif1a | NM_010120.5 | chr18:46597703-46610225 |
| 6436 | Eda | NM_001177937.1 | chrX:99975605-100400760 | 6533 | Eif1ad | NM_027236.2 | chr19:5366812-5371511 |
| 6437 | Eda2r | NM_001161432.1 | chrX:97333840-97377209 | 6534 | Eif1ax | NM_025437.4 | chrX:159372194-159385699 |
| 6438 | Edar | NM_010100.3 | chr10:58600787-58675696 | 6535 | Eif1b | NM_026892.3 | chr9:120492605-120495327 |
| 6439 | Edaradd | NM_133643.4 | chr13:12471208-12520450 | 6536 | Eif2a | NM_001005509.2 | chr3:58525820-58557501 |
| 6440 | Edc3 | NM_153799.3 | chr9:57708568-57750162 | 6537 | Eif2ak1 | NM_013557.2 | chr5:143871861-143902717 |
| 6441 | Edc4 | NM_181594.3 | chr8:105880880-105893225 | 6538 | Eif2ak2 | NM_011163.4 | chr17:78852549-78882572 |
| 6442 | Eddm3b | NM_203508.1 | chr14:51116552-51117791 | 6539 | Eif2ak3 | NM_010121.3 | chr6:70844514-70905241 |
| 6443 | Edem1 | NM_138677.2 | chr6:108828640-108859356 | 6540 | Eif2ak4 | NM_001177806.1 | chr2:118389111-118475234 |
| 6444 | Edem2 | NM_145537.2 | chr2:155701672-155729475 | 6541 | Eif2b1 | NM_145371.4 | chr5:124570213-124579131 |
| 6445 | Edem3 | NM_001039644.2 | chr1:151755373-151822328 | 6542 | Eif2b2 | NM_145445.4 | chr12:85219480-85226628 |
| 6446 | Edf1 | NM_021519.1 | chr2:25557899-25562082 | 6543 | Eif2b3 | NM_001111277.1 | chr4:117019407-117086852 |
| 6447 | Edil3 | NM_001037987.3 | chr13:88821471-89323225 | 6544 | Eif2b4 | NM_001127355.1 | chr5:31187557-31193139 |
| 6448 | Edn1 | NM_010104.3 | chr13:42301269-42307989 | 6545 | Eif2b5 | NM_172265.2 | chr16:20498816-20509325 |
| 6449 | Edn2 | NM_007902.2 | chr4:120161423-120167360 | 6546 | Eif2d | NM_001136070.1 | chr1:131153206-131173471 |
| 6450 | Edn3 | NM_007903.4 | chr2:174760757-174784042 | 6547 | Eif2s1 | NM_026114.3 | chr12:78862071-78887010 |
| 6451 | Ednra | NM_010332.2 | chr8:77663028-77724452 | 6548 | Eif2s2 | NM_026030.2 | chr2:154871409-154892906 |
| 6452 | Ednrb | NM_001136061.2 | chr14:103814614-103844476 | 6549 | Eif2s3x | NM_012010.3 | chrX:94188708-94212651 |
| 6453 | Edrf1 | NM_178115.4 | chr7:133637674-133673017 | 6550 | Eif2s3y | NM_012011.1 | chrY:1010611-1028598 |
| 6454 | Eea1 | NM_001001932.3 | chr10:95940662-96045518 | 6551 | Eif3a | NM_010123.3 | chr19:60761115-60790693 |
| 6455 | Eed | NM_021876.3 | chr7:89954653-89980976 | 6552 | Eif3b | NM_133916.2 | chr5:140419304-140443358 |
| 6456 | Eef1a1 | NM_010106.2 | chr9:78478453-78481724 | 6553 | Eif3c | NM_146200.1 | chr7:126546910-126566366 |
| 6457 | Eef1a2 | NM_007906.2 | chr2:181147691-181157015 | 6554 | Eif3d | NM_018749.2 | chr15:77958997-77970824 |
| 6458 | Eef1b2 | NM_018796.3 | chr1:63176830-63180486 | 6555 | Eif3e | NM_008388.2 | chr15:43250039-43282736 |
| 6459 | Eef1d | NM_001285429.1 | chr15:75894795-75909556 | 6556 | Eif3f | NM_025344.2 | chr7:108934414-108941942 |
| 6460 | Eef1e1 | NM_025380.2 | chr13:38645690-38659028 | 6557 | Eif3g | NM_016876.3 | chr9:20894348-20898590 |
| 6461 | Eef1g | NM_026007.4 | chr19:8967040-8978180 | 6558 | Eif3h | NM_080635.1 | chr15:51786562-51865461 |
| 6462 | Eef2 | NM_007907.2 | chr10:81176630-81182509 | 6559 | Eif3i | NM_018799.2 | chr4:129591973-129600648 |
| 6463 | Eef2k | NM_001267710.1 | chr7:120843599-120907219 | 6560 | Eif3j1 | NM_144545.4 | chr2:122028579-122053632 |
| 6464 | Eefsec | NM_023060.3 | chr6:88257333-88446539 | 6561 | Eif3j2 | NM_001256055.1 | chr2:122028582-122053631 |
| 6465 | Eepd1 | NM_026189.3 | chr9:25481596-25604110 | 6562 | Eif3k | NM_001285942.1 | chr7:28971371-28981888 |
| 6466 | Efcab1 | NM_025769.3 | chr16:14906645-14924522 | 6563 | Eif3l | NM_145139.2 | chr15:79075222-79094400 |
| 6467 | Efcab10 | NM_029152.1 | chr12:33394853-33401269 | 6564 | Eif3m | NM_145380.2 | chr2:104999656-105017027 |
| 6468 | Efcab11 | NM_030172.2 | chr12:99717530-99883442 | 6565 | Eif4a1 | NM_001159375.1 | chr11:69666935-69672423 |
| 6469 | Efcab12 | NM_001110506.1 | chr6:115810728-115838412 | 6566 | Eif4a2 | NM_001123037.2 | chr16:23107467-23114132 |
| 6470 | Efcab14 | NM_172698.2 | chr4:115738072-115777327 | 6567 | Eif4a3 | NM_138669.1 | chr11:119288362-119300043 |
| 6471 | Efcab2 | NM_026626.2 | chr1:178405880-178483249 | 6568 | Eif4b | NM_145625.3 | chr15:102073772-102097173 |
| 6472 | Efcab3 | NM_001081046.1 | chr11:105092218-105117537 | 6569 | Eif4e | NM_007917.4 | chr3:138526190-138559696 |
| 6473 | Efcab4a | NM_001025103.2 | chr7:141461093-141466602 | 6570 | Eif4e1b | NM_001033269.3 | chr13:54783997-54788458 |
| 6474 | Efcab4b | NM_001033464.3 | chr6:127577974-127629938 | 6571 | Eif4e2 | NM_001039169.1 | chr1:87213938-87228858 |
| 6475 | Efcab5 | NM_176965.3 | chr11:77089914-77188968 | 6572 | Eif4e3 | NM_025829.4 | chr6:99625136-99666771 |
| 6476 | Efcab6 | NM_001161628.1 | chr15:83866711-83936095 | 6573 | Eif4ebp1 | NM_007918.3 | chr8:27260326-27275656 |
| 6477 | Efcab7 | NM_145549.1 | chr4:99829499-99912783 | 6574 | Eif4ebp2 | NM_010124.2 | chr10:61432496-61452669 |
| 6478 | Efcab8 | NR_036629.1 | chr2:153780878-153795182 | 6575 | Eif4ebp3 | NM_201256.4 | chr18:36664059-36666324 |
| 6479 | Efcab9 | NM_027031.3 | chr11:32522732-32527574 | 6576 | Eif4enif1 | NM_001166547.1 | chr11:3202994-3244588 |
| 6480 | Efcc1 | NM_001159697.1 | chr6:87730868-87755911 | 6577 | Eif4g1 | NM_001304432.1 | chr16:20672720-20692883 |
| 6481 | Efemp1 | NM_146015.2 | chr11:28853204-28926743 | 6578 | Eif4g2 | NM_001040131.2 | chr7:111067984-111083030 |
| 6482 | Efemp2 | NM_001164352.1 | chr19:5474734-5481854 | 6579 | Eif4g3 | NM_001256195.1 | chr4:137993455-138207079 |
| 6483 | Efhb | NM_172497.3 | chr17:53398888-53463321 | 6580 | Eif4h | NM_033561.2 | chr5:134619871-134639409 |
| 6484 | Efhc1 | NM_027974.1 | chr1:20951625-20990841 | 6581 | Eif5 | NM_173363.5 | chr12:111538100-111546753 |
| 6485 | Efhc2 | NM_028916.4 | chrX:17132048-17319368 | 6582 | Eif5a | NM_001166589.1 | chr11:69916711-69921386 |
| 6486 | Efhd1 | NM_028889.2 | chr1:87264363-87310791 | 6583 | Eif5a2 | NM_177586.5 | chr3:28781310-28798846 |
| 6487 | Efhd2 | NM_025994.3 | chr4:141858141-141874920 | 6584 | Eif5b | NM_198303.2 | chr1:37998009-38055579 |
| 6488 | Efna1 | NM_001162425.1 | chr3:89271729-89280951 | 6585 | Eif6 | NM_010579.2 | chr2:155819836-155826925 |
| 6489 | Efna2 | NM_007909.3 | chr10:80179481-80190010 | 6586 | Elac1 | NM_053255.3 | chr18:73735037-73754479 |
| 6490 | Efna3 | NM_010108.1 | chr3:89314950-89322879 | 6587 | Elac2 | NM_023479.2 | chr11:64979034-65002076 |
| 6491 | Efna4 | NM_007910.2 | chr3:89333392-89338028 | 6588 | Elane | NM_015779.2 | chr10:79886311-79888216 |
| 6492 | Efna5 | NM_010109.3 | chr17:62602956-62881317 | 6589 | Elavl1 | NM_010485.3 | chr8:4284781-4325100 |
| 6493 | Efnb1 | NM_010110.5 | chrX:99136060-99149022 | 6590 | Elavl2 | NM_001177883.1 | chr4:91250766-91376496 |
| 6494 | Efnb2 | NM_010111.5 | chr8:8617438-8660773 | 6591 | Elavl3 | NM_010487.2 | chr9:22015004-22052023 |
| 6495 | Efnb3 | NM_007911.5 | chr11:69554091-69560237 | 6592 | Elavl4 | NM_001038698.1 | chr4:110203736-110287511 |
| 6496 | Efr3a | NM_133766.3 | chr15:65787040-65873812 | 6593 | Elf1 | NM_001286411.1 | chr14:79515673-79582491 |
| 6497 | Efr3b | NM_001082483.1 | chr12:3962553-4038915 | 6594 | Elf2 | NM_001291059.1 | chr3:51252719-51340644 |
| 6498 | Efs | NM_010112.4 | chr14:54916542-54926788 | 6595 | Elf3 | NM_001163131.1 | chr1:135253573-135258472 |

Fig.21 - 35

| | | | |
|---|---|---|---|
| 6596 | Elf4 | NM_019680.2 | chrX:48411048-48463132 |
| 6597 | Elf5 | NM_001145813.1 | chr2:103412097-103450988 |
| 6598 | Elfn1 | NM_175522.3 | chr5:139907942-139974724 |
| 6599 | Elfn2 | NM_183141.2 | chr15:78670006-78718113 |
| 6600 | Elk1 | NM_007922.5 | chrX:20933394-20950608 |
| 6601 | Elk3 | NM_001282967.1 | chr10:93247415-93311159 |
| 6602 | Elk4 | NM_007923.2 | chr1:132007604-132025684 |
| 6603 | Ell | NM_007924.2 | chr8:70539674-70592858 |
| 6604 | Ell2 | NM_138953.2 | chr13:75707483-75772358 |
| 6605 | Ell3 | NM_145973.2 | chr2:121439026-121442601 |
| 6606 | Elmo1 | NM_080288.2 | chr13:20090506-20608353 |
| 6607 | Elmo2 | NM_080287.2 | chr2:165288030-165326479 |
| 6608 | Elmo3 | NM_172760.3 | chr8:105305600-105310623 |
| 6609 | Elmod1 | NM_177769.4 | chr9:53911459-53975301 |
| 6610 | Elmod2 | NM_001170691.1 | chr8:83312631-83332486 |
| 6611 | Elmod3 | NM_001253692.1 | chr6:72565921-72598413 |
| 6612 | Elmsan1 | NM_001163501.1 | chr12:84149173-84218881 |
| 6613 | Eln | NM_007925.4 | chr5:134702594-134747323 |
| 6614 | Elof1 | NM_170777.3 | chr9:22112988-22114169 |
| 6615 | Elovl1 | NM_001039175.2 | chr4:118428092-118432952 |
| 6616 | Elovl2 | NM_019423.2 | chr13:41182381-41220403 |
| 6617 | Elovl3 | NM_007703.2 | chr19:46131898-46135694 |
| 6618 | Elovl4 | NM_148941.2 | chr9:83778691-83806305 |
| 6619 | Elovl5 | NM_134255.3 | chr9:77917364-77984519 |
| 6620 | Elovl6 | NM_130450.2 | chr3:129532385-129638493 |
| 6621 | Elovl7 | NM_029001.5 | chr13:108214403-108287107 |
| 6622 | Elp2 | NM_021448.2 | chr18:24603960-24638830 |
| 6623 | Elp3 | NM_001253812.1 | chr14:65530445-65593112 |
| 6624 | Elp4 | NM_023876.4 | chr2:105697319-105904564 |
| 6625 | Elp5 | NM_001253700.1 | chr11:69968225-69980330 |
| 6626 | Elp6 | NM_001081381.1 | chr9:110305191-110322102 |
| 6627 | Eltd1 | NM_133222.3 | chr3:151437881-151545081 |
| 6628 | Emb | NM_010330.4 | chr13:117220572-117274415 |
| 6629 | Emc1 | NM_001039200.2 | chr4:139352586-139378735 |
| 6630 | Emc10 | NM_197991.2 | chr7:44489937-44496513 |
| 6631 | Emc2 | NM_025736.2 | chr15:43477228-43527777 |
| 6632 | Emc3 | NM_175101.3 | chr6:113514886-113531638 |
| 6633 | Emc4 | NM_026519.3 | chr2:112363018-112368027 |
| 6634 | Emc6 | NM_001168470.1 | chr11:73175502-73177008 |
| 6635 | Emc7 | NM_133749.2 | chr2:112455024-112467436 |
| 6636 | Emc8 | NM_010926.5 | chr8:120653913-120668112 |
| 6637 | Emc9 | NM_033146.1 | chr14:55581523-55585254 |
| 6638 | Emcn | NM_001163522.1 | chr3:137341077-137431069 |
| 6639 | Emd | NM_007927.3 | chrX:74254686-74257893 |
| 6640 | Eme1 | NM_177752.4 | chr11:94645001-94653754 |
| 6641 | Eme2 | NM_001163102.1 | chr17:24872151-24895087 |
| 6642 | Emg1 | NM_013536.2 | chr6:124704369-124712178 |
| 6643 | Emid1 | NM_080595.2 | chr11:5106265-5152222 |
| 6644 | Emilin1 | NM_133918.2 | chr5:30913785-30921273 |
| 6645 | Emilin2 | NM_145158.3 | chr17:71252175-71310965 |
| 6646 | Emilin3 | NM_001291145.1 | chr2:160906437-160912339 |
| 6647 | Eml1 | NM_001043335.1 | chr12:108422815-108539564 |
| 6648 | Eml2 | NM_001162996.1 | chr7:19181169-19206482 |
| 6649 | Eml3 | NM_144872.1 | chr19:8929693-8941582 |
| 6650 | Eml4 | NM_001114361.1 | chr17:83350930-83480359 |
| 6651 | Eml5 | NM_001081191.1 | chr12:98786603-98901484 |
| 6652 | Eml6 | NM_146016.2 | chr11:29743050-30026033 |
| 6653 | Emp1 | NM_001288627.1 | chr6:135367492-135383173 |
| 6654 | Emp2 | NM_007929.2 | chr16:10281748-10313968 |
| 6655 | Emp3 | NM_001146346.1 | chr7:45918022-45920849 |
| 6656 | Emr1 | NM_010130.4 | chr17:57358685-57483529 |
| 6657 | Emr4 | NM_139138.3 | chr17:55749983-55853662 |
| 6658 | Emx1 | NM_010131.2 | chr6:85187930-85204463 |
| 6659 | Emx2 | NM_010132.2 | chr19:59458689-59465357 |
| 6660 | Emx2os | NR_002863.2 | chr19:59425103-59458635 |
| 6661 | En1 | NM_010133.2 | chr1:120602486-120607991 |
| 6662 | En2 | NM_010134.3 | chr5:28165695-28172166 |
| 6663 | Enah | NM_001083120.2 | chr1:181896385-182019980 |
| 6664 | Enam | NM_017468.3 | chr5:88487974-88506049 |
| 6665 | Enc1 | NM_007930.4 | chr13:97241104-97253040 |
| 6666 | Endod1 | NM_028013.3 | chr9:14353989-14381242 |
| 6667 | Endog | NM_007931.1 | chr2:30171523-30174069 |
| 6668 | Endou | NM_001168693.1 | chr15:97711018-97731405 |
| 6669 | Endov | NM_001164636.1 | chr11:119491346-119511465 |
| 6670 | Eng | NM_001146348.1 | chr2:32646594-32682669 |
| 6671 | Engase | NM_172573.2 | chr11:118476959-118489198 |
| 6672 | Enho | NM_027147.1 | chr4:41638143-41640302 |
| 6673 | Enkd1 | NM_198299.1 | chr8:105703651-105708168 |
| 6674 | Enkur | NM_027728.2 | chr2:21180730-21205365 |
| 6675 | Eno1 | NM_023119.2 | chr4:150237196-150248873 |
| 6676 | Eno1b | NM_001025388.1 | chr18:48045334-150248873 |
| 6677 | Eno2 | NM_013509.3 | chr6:124760052-124769673 |
| 6678 | Eno3 | NM_001136062.2 | chr11:70657175-70662513 |
| 6679 | Eno4 | NM_178689.4 | chr19:58943424-58971421 |
| 6680 | Enoph1 | NM_001163035.1 | chr5:100039993-100068765 |
| 6681 | Enox1 | NM_001253759.1 | chr14:77156762-77721763 |
| 6682 | Enox2 | NM_001271447.1 | chrX:49009706-49288242 |
| 6683 | Enpep | NM_007934.3 | chr3:129269176-129332749 |
| 6684 | Enpp1 | NM_008813.4 | chr10:24637913-24712159 |
| 6685 | Enpp2 | NM_001136077.3 | chr15:54838900-54920146 |
| 6686 | Enpp3 | NM_134005.2 | chr10:24773813-24836195 |
| 6687 | Enpp4 | NM_199016.2 | chr17:44096309-44105808 |
| 6688 | Enpp5 | NM_001168620.1 | chr17:44078847-44086561 |
| 6689 | Enpp6 | NM_177304.3 | chr8:46986924-47094895 |
| 6690 | Enpp7 | NM_001030291.1 | chr11:118988187-118992841 |
| 6691 | Ensa | NM_001026212.1 | chr3:95624979-95630493 |
| 6692 | Enthd1 | NM_001163189.1 | chr15:80452239-80560470 |
| 6693 | Enthd2 | NM_183137.2 | chr11:120090521-120098731 |
| 6694 | Entpd1 | NM_009848.4 | chr19:40659769-40741602 |
| 6695 | Entpd2 | NM_009849.2 | chr2:25395873-25401323 |
| 6696 | Entpd3 | NM_178676.4 | chr9:120539817-120568326 |
| 6697 | Entpd4 | NM_026174.3 | chr14:69337150-69584992 |
| 6698 | Entpd5 | NM_001026214.2 | chr12:84373875-84409029 |
| 6699 | Entpd6 | NM_172117.5 | chr2:150749080-150771674 |
| 6700 | Entpd7 | NM_053103.5 | chr19:43689688-43733853 |
| 6701 | Entpd8 | NM_028093.1 | chr2:25080322-25085719 |
| 6702 | Eny2 | NM_175009.3 | chr15:44428110-44437685 |
| 6703 | Eogt | NM_175313.2 | chr6:97110943-97148883 |
| 6704 | Eomes | NM_001164789.1 | chr9:118478188-118486132 |
| 6705 | Ep300 | NM_177821.6 | chr15:81586213-81652077 |
| 6706 | Ep400 | NM_029337.2 | chr5:110664372-110770717 |
| 6707 | Epas1 | NM_010137.3 | chr17:86753863-86833410 |
| 6708 | Epb4.1 | NM_001128606.1 | chr4:131923428-132049078 |
| 6709 | Epb4.1l1 | NM_001006664.3 | chr2:156420487-156543214 |
| 6710 | Epb4.1l2 | NM_001199265.1 | chr10:25359797-25523518 |
| 6711 | Epb4.1l3 | NM_013813.1 | chr17:69156809-69289987 |
| 6712 | Epb4.1l4a | NM_013512.2 | chr18:33796326-34007206 |
| 6713 | Epb4.1l4b | NM_019427.2 | chr4:57061725-57143156 |
| 6714 | Epb4.1l5 | NM_001113416.1 | chr1:119594823-119649000 |
| 6715 | Epb4.2 | NM_013513.3 | chr2:121017890-121036877 |
| 6716 | Epc1 | NM_001276350.1 | chr18:6488891-6490857 |
| 6717 | Epc2 | NM_172663.4 | chr2:49451485-49551609 |
| 6718 | Epcam | NM_008532.2 | chr17:87635978-87651127 |
| 6719 | Epdr1 | NM_134065.4 | chr13:19591707-19619830 |
| 6720 | Epg5 | NM_001195633.1 | chr18:77938466-78035027 |
| 6721 | Epgn | NM_053087.2 | chr5:91027516-91035212 |
| 6722 | Epha1 | NM_023580.4 | chr6:42358486-42373268 |
| 6723 | Epha10 | NM_001256432.1 | chr4:124881784-124917800 |
| 6724 | Epha2 | NM_010139.3 | chr4:141301220-141329384 |
| 6725 | Epha3 | NM_010140.3 | chr16:63545217-63864157 |
| 6726 | Epha4 | NM_007936.3 | chr1:77367184-77515088 |
| 6727 | Epha5 | NM_007937.3 | chr5:84054764-84417382 |
| 6728 | Epha6 | NM_007938.2 | chr16:59653482-60605531 |
| 6729 | Epha7 | NM_001122889.1 | chr4:28813144-28947453 |
| 6730 | Epha8 | NM_007939.2 | chr4:136929418-136956816 |
| 6731 | Ephb1 | NM_001168296.1 | chr9:101922127-102354693 |
| 6732 | Ephb2 | NM_001290753.2 | chr4:136647540-136836012 |
| 6733 | Ephb3 | NM_010143.1 | chr16:21204794-21223304 |
| 6734 | Ephb4 | NM_001159571.1 | chr5:137350108-137374522 |
| 6735 | Ephb6 | NM_001146351.1 | chr6:41605481-41620507 |
| 6736 | Ephx1 | NM_010145.3 | chr1:180989555-181017569 |
| 6737 | Ephx2 | NM_001271402.1 | chr14:66084371-66124522 |
| 6738 | Ephx3 | NM_001033163.3 | chr17:32183769-32189463 |
| 6739 | Ephx4 | NM_001001804.2 | chr5:107403512-107430031 |
| 6740 | Epm2a | NM_010146.2 | chr10:11343444-11457477 |
| 6741 | Epm2aip1 | NM_175266.4 | chr9:111271844-111279091 |
| 6742 | Epn1 | NM_001252454.1 | chr7:5080234-5098178 |
| 6743 | Epn2 | NM_001252188.1 | chr11:61517248-61579687 |
| 6744 | Epn3 | NM_027984.3 | chr11:94489598-94499974 |
| 6745 | Epo | NM_007942.2 | chr5:137483019-137485816 |
| 6746 | Epor | NM_010149.3 | chr9:21958898-21963576 |
| 6747 | Eppin | NM_029325.2 | chr2:164588342-164593571 |
| 6748 | Eppk1 | NM_144848.2 | chr15:76101487-76120195 |
| 6749 | Eprs | NM_029735.1 | chr1:185363094-185428355 |
| 6750 | Eps15 | NM_001159964.1 | chr4:109343055-109387816 |
| 6751 | Eps15l1 | NM_001122832.1 | chr8:72377416-72421474 |
| 6752 | Eps8 | NM_001271587.1 | chr6:137477244-137571009 |
| 6753 | Eps8l1 | NM_001290416.1 | chr7:4464848-4480487 |
| 6754 | Eps8l2 | NM_133191.2 | chr7:141339001-141363016 |
| 6755 | Eps8l3 | NM_133867.2 | chr3:107877229-107892900 |
| 6756 | Epsti1 | NM_029495.2 | chr14:77904238-78002656 |
| 6757 | Ept1 | NM_027652.2 | chr5:30232617-30272430 |
| 6758 | Epx | NM_007946.2 | chr11:87863997-87875536 |
| 6759 | Epyc | NM_007884.2 | chr10:97644067-97681900 |
| 6760 | Eqtn | NM_001290623.1 | chr4:94907266-94928843 |
| 6761 | Eral1 | NM_022313.2 | chr11:78073375-78080383 |
| 6762 | Erap1 | NM_030711.4 | chr13:74639871-74691875 |
| 6763 | Eras | NM_181548.2 | chrX:7924275-7928607 |
| 6764 | Erbb2 | NM_001003817.1 | chr11:98412483-98437716 |
| 6765 | Erbb2ip | NM_001005868.2 | chr13:103818785-103920586 |
| 6766 | Erbb3 | NM_010153.1 | chr10:128569367-128589501 |
| 6767 | Erbb4 | NM_010154.2 | chr1:68032186-69108059 |
| 6768 | Erc1 | NM_053204.2 | chr6:119570795-119848150 |
| 6769 | Erc2 | NM_177814.4 | chr14:27622441-28478537 |
| 6770 | Ercc1 | NM_001127324.1 | chr7:19345070-19354830 |
| 6771 | Ercc2 | NM_007949.4 | chr7:19382038-19395692 |
| 6772 | Ercc3 | NM_133658.1 | chr18:32240330-32270147 |
| 6773 | Ercc4 | NM_015769.2 | chr16:13109735-13152009 |
| 6774 | Ercc5 | NM_011729.2 | chr1:44147743-44181260 |
| 6775 | Ercc6 | NM_001081221.1 | chr14:32513520-32580989 |
| 6776 | Ercc6l | NM_146235.3 | chrX:102142819-102157091 |
| 6777 | Ercc6l2 | NM_001013608.2 | chr13:63815319-63900301 |
| 6778 | Ercc8 | NM_028042.3 | chr13:108158737-108194981 |
| 6779 | Erdr1 | NM_133362.2 | chrY:90785441-90816465 |
| 6780 | Ereg | NM_007950.2 | chr5:91074616-91093649 |
| 6781 | Erf | NM_010155.3 | chr7:25242559-25250758 |
| 6782 | Erg | NM_133659.3 | chr16:95359168-95530365 |
| 6783 | Ergic1 | NM_026170.2 | chr17:26561511-26656933 |
| 6784 | Ergic2 | NM_001286560.1 | chr6:148179317-148212374 |
| 6785 | Ergic3 | NM_025516.4 | chr2:156008124-156018279 |
| 6786 | Erh | NM_007951.3 | chr12:80634022-80643861 |
| 6787 | Eri1 | NM_026067.3 | chr8:35465264-35495533 |
| 6788 | Eri2 | NM_027698.5 | chr7:119783825-119794058 |
| 6789 | Eri3 | NM_001285899.1 | chr4:117550364-117674297 |

Fig.21 - 36

| | | | | | | | |
|---|---|---|---|---|---|---|---|
| 6790 | Erich1 | NM_001034862.2 | chr8:14027564-14090327 | 6887 | Exo1 | NM_012012.4 | chr1:175880777-175911396 |
| 6791 | Erich2 | NM_025744.2 | chr2:70508818-70540884 | 6888 | Exo5 | NM_001160043.1 | chr4:120921201-120925005 |
| 6792 | Erich3 | NM_175176.1 | chr3:154711132-154749012 | 6889 | Exoc1 | NM_001289770.1 | chr5:76529310-76570298 |
| 6793 | Erich4 | NM_001039243 | chr7:25614620-25615892 | 6890 | Exoc2 | NM_025588.2 | chr13:30813917-30974047 |
| 6794 | Erich5 | NM_173421 | chr15:34453311-34473892 | 6891 | Exoc3 | NM_177333.3 | chr13:74169804-74208700 |
| 6795 | Erich6 | NM_001081262.1 | chr3:58616299-58637207 | 6892 | Exoc3l | NM_177788.2 | chr8:105289923-105296098 |
| 6796 | Erlec1 | NM_025745.2 | chr11:30929783-30954131 | 6893 | Exoc3l4 | NM_001289487.1 | chr12:111417429-111431680 |
| 6797 | Erlin1 | NM_001164359.1 | chr19:44034942-44069775 | 6894 | Exoc4 | NM_009148 | chr6:33249149-33972930 |
| 6798 | Erlin2 | NM_153592 | chr8:27023798-27039435 | 6895 | Exoc5 | NM_207214 | chr14:49012143-49066667 |
| 6799 | Ermap | NM_013848.1 | chr4:119175456-119190011 | 6896 | Exoc6 | NM_175353.2 | chr19:37550417-37683249 |
| 6800 | Ermard | NM_001034891.3 | chr17:15053058-15064232 | 6897 | Exoc6b | NM_177077.2 | chr6:84618485-85069513 |
| 6801 | Ermn | NM_029972.3 | chr2:58045114-58052752 | 6898 | Exoc7 | NM_001162872.1 | chr11:116287997-116306738 |
| 6802 | Ermp1 | NM_001081213.1 | chr19:29609882-29648420 | 6899 | Exoc8 | NM_198103.2 | chr8:124890298-124897705 |
| 6803 | Ern1 | NM_023913.2 | chr11:106397619-106487796 | 6900 | Exog | NM_001172136.1 | chr9:119444922-119465518 |
| 6804 | Ern2 | NM_012016.2 | chr7:122169892-122186216 | 6901 | Exosc1 | NM_001164561.1 | chr19:41922979-41933314 |
| 6805 | Ero1l | NM_015774.3 | chr14:45283086-45318572 | 6902 | Exosc10 | NM_016699.2 | chr4:148558426-148582400 |
| 6806 | Ero1lb | NM_026184.2 | chr13:12565882-12609528 | 6903 | Exosc2 | NM_144886.2 | chr2:31670736-31681307 |
| 6807 | Erp27 | NM_026983.2 | chr6:136907386-136922180 | 6904 | Exosc3 | NM_025513.3 | chr4:45316629-45320603 |
| 6808 | Erp29 | NM_026129.2 | chr5:121444752-121452474 | 6905 | Exosc4 | NM_175399.4 | chr15:76327396-76330670 |
| 6809 | Erp44 | NM_029572.2 | chr4:48193330-48279589 | 6906 | Exosc5 | NM_138586.3 | chr7:25659152-25668032 |
| 6810 | Errfi1 | NM_133753.1 | chr4:150855090-150868880 | 6907 | Exosc6 | NM_028274.4 | chr8:111056338-111057664 |
| 6811 | Erv3 | NM_001166206.1 | chr2:131853677-131859747 | 6908 | Exosc7 | NM_001081188.1 | chr9:123113230-123136129 |
| 6812 | Esam | NM_027102.3 | chr9:37528088-37538319 | 6909 | Exosc8 | NM_001163570.1 | chr3:54728678-54735364 |
| 6813 | Esco1 | NM_001081222.1 | chr18:10566511-10610352 | 6910 | Exosc9 | NM_019393.2 | chr3:36552605-36565727 |
| 6814 | Esco2 | NM_028039.2 | chr14:65819026-65833969 | 6911 | Exph5 | NM_176846.3 | chr9:53301669-53381158 |
| 6815 | Esd | NM_001285423.1 | chr14:74732296-74750765 | 6912 | Ext1 | NM_010162.2 | chr15:53068260-53346183 |
| 6816 | Esf1 | NM_001081090.1 | chr2:140119880-140170558 | 6913 | Ext2 | NM_010163.3 | chr2:93695630-93822568 |
| 6817 | Esm1 | NM_023612.3 | chr13:113209658-113218104 | 6914 | Extl1 | NM_019578.2 | chr4:134356372-134372547 |
| 6818 | Esp1 | NM_001038500.2 | chr17:40727119-40731782 | 6915 | Extl2 | NM_001163514.1 | chr3:116008296-116029016 |
| 6819 | Esp15 | NM_001244651.1 | chr17:39640956-39645667 | 6916 | Extl3 | NM_018788.3 | chr14:65052058-65098106 |
| 6820 | Esp16 | NM_001255977.1 | chr17:39536141-39540847 | 6917 | Eya1 | NM_001252192.1 | chr1:14168957-14310199 |
| 6821 | Esp18 | NM_001244763.1 | chr17:39406358-39410992 | 6918 | Eya2 | NM_001271962.1 | chr2:165630344-165771727 |
| 6822 | Esp23 | NM_001177582.1 | chr17:39073690-39077037 | 6919 | Eya3 | NM_010166.3 | chr4:132639045-132724765 |
| 6823 | Esp24 | NM_001256050.1 | chr17:39036694-39040227 | 6920 | Eya4 | NM_010167.4 | chr10:23104167-23349903 |
| 6824 | Esp3 | NM_001251916.1 | chr17:40632074-40637060 | 6921 | Ezh1 | NM_007970.3 | chr11:101191114-101226463 |
| 6825 | Esp31 | NM_001177586.1 | chr17:38639446-38645653 | 6922 | Ezh2 | NM_001146689.1 | chr6:47530273-47595030 |
| 6826 | Esp34 | NM_001177585.1 | chr17:38554191-38560621 | 6923 | Ezr | NM_009510.2 | chr17:6738130-6782780 |
| 6827 | Esp36 | NM_001177587.1 | chr17:38416473-38420185 | 6924 | F10 | NM_001242368.1 | chr8:13037307-13056676 |
| 6828 | Esp38 | NM_001256051.1 | chr17:39950519-39955287 | 6925 | F11 | NM_028066.2 | chr8:45241167-45262031 |
| 6829 | Esp4 | NM_001177583.1 | chr17:40598593-40602617 | 6926 | F11r | NM_172647.2 | chr1:171437560-171464593 |
| 6830 | Esp5 | NM_001287194.1 | chr17:40574710-40579549 | 6927 | F12 | NM_021489.3 | chr13:55417957-55426804 |
| 6831 | Esp6 | NM_001177529.1 | chr17:40561506-40565624 | 6928 | F13a1 | NM_001166391.1 | chr13:36867177-37050244 |
| 6832 | Esp6-esp5 | NM_001287195.1 | chr17:40561506-40579549 | 6929 | F13b | NM_031164.2 | chr1:139501706-139523756 |
| 6833 | Esp8 | NM_001177584.1 | chr17:40520021-40530702 | 6930 | F2 | NM_010168.3 | chr2:91625319-91636457 |
| 6834 | Espl1 | NM_001014976.2 | chr15:102296292-102324356 | 6931 | F2r | NM_010169.3 | chr13:95601788-95618433 |
| 6835 | Espn | NM_019585.3 | chr4:152120875-152128925 | 6932 | F2rl1 | NM_007974.4 | chr13:95511729-95525240 |
| 6836 | Espnl | NM_001033292 | chr1:91322074-91348303 | 6933 | F2rl2 | NM_010170.4 | chr13:95696919-95702768 |
| 6837 | Esr1 | NM_007956.5 | chr10:4611988-5005633 | 6934 | F2rl3 | NM_007975.3 | chr8:72761879-72763885 |
| 6838 | Esr2 | NM_010157.3 | chr12:76120418-76177259 | 6935 | F3 | NM_010171.3 | chr3:121723536-121735052 |
| 6839 | Esrp1 | NM_001290383.1 | chr4:11331932-11386783 | 6936 | F420014N23Rik | NR_045715.1 | chr10:127195248-127202643 |
| 6840 | Esrp2 | NM_176838.2 | chr8:106131182-106136974 | 6937 | F5 | NM_007976.3 | chr1:164151834-164220277 |
| 6841 | Esrra | NM_007953.2 | chr19:6910976-6921808 | 6938 | F630028O10Rik | NR_030718.1 | chrX:96239925-96243642 |
| 6842 | Esrrb | NM_001159500.1 | chr12:86361116-86521628 | 6939 | F630042J09Rik | NR_033540.1 | chr13:67278593-67283361 |
| 6843 | Esrrg | NM_001243792.1 | chr1:187609005-188214884 | 6940 | F630111L10Rik | NR_045641.1 | chr3:59146295-59153628 |
| 6844 | Esx1 | NM_007957.2 | chrX:137115396-137120326 | 6941 | F630206G17Rik | NR_045876.1 | chr11:45808082-45842878 |
| 6845 | Esyt1 | NM_011843.2 | chr10:128510249-128525859 | 6942 | F7 | NM_010172.4 | chr8:13026033-13035805 |
| 6846 | Esyt2 | NM_028731.5 | chr12:116281221-116373098 | 6943 | F730035M05Rik | NR_045174.1 | chr12:70227840-70234165 |
| 6847 | Esyt3 | NM_177775.3 | chr9:99309966-99358530 | 6944 | F730043M19Rik | NR_015602.2 | chr12:33111710-33147586 |
| 6848 | Etaa1 | NM_026576.3 | chr11:17938748-17953875 | 6945 | F8 | NM_001161373.1 | chrX:75172714-75380041 |
| 6849 | Etd | NR_034074.1 | chrX:53434917-53443576 | 6946 | F830002L21Rik | NR_033558.1 | chr10:43593426-43630913 |
| 6850 | Etf1 | NM_144866.3 | chr18:34902784-34932003 | 6947 | F830016B08Rik | NM_001101475.2 | chr18:60293379-60303016 |
| 6851 | Etfa | NM_145615.4 | chr9:55454435-55512243 | 6948 | F830045P16Rik | NM_177653.3 | chr2:129458358-129536602 |
| 6852 | Etfb | NM_026695.3 | chr7:43444071-43457800 | 6949 | F8a | NM_007978.3 | chrX:73228305-73230795 |
| 6853 | Etfdh | NM_025794.2 | chr3:79603787-79628767 | 6950 | F9 | NM_007979.2 | chrX:59999463-60030760 |
| 6854 | Ethe1 | NM_023154.3 | chr7:24587542-24608926 | 6951 | F930015N05Rik | NR_028445.1 | chr11:64433134-64436674 |
| 6855 | Etl4 | NM_001081006.1 | chr2:20289912-20810535 | 6952 | Fa2h | NM_178086.3 | chr8:111345137-111393821 |
| 6856 | Etnk1 | NM_029250.2 | chr6:143167229-143208547 | 6953 | Faah | NM_010173.4 | chr4:115996655-116017902 |
| 6857 | Etnk2 | NM_175443.2 | chr1:133363571-133380319 | 6954 | Fabp1 | NM_017399.4 | chr6:71199887-71205023 |
| 6858 | Etnppl | NM_001163587.1 | chr3:130617447-130635750 | 6955 | Fabp12 | NM_029310.1 | chr3:10244208-10301183 |
| 6859 | Etohd2 | NR_015349.2 | chr13:59769965-59773680 | 6956 | Fabp2 | NM_007980.3 | chr3:122895071-122899506 |
| 6860 | Etohi1 | NM_001177399.1 | chr2:178023283-178035859 | 6957 | Fabp3 | NM_010174.1 | chr4:130308777-130315463 |
| 6861 | Ets1 | NM_001038642.1 | chr9:32696041-32757820 | 6958 | Fabp4 | NM_024406.2 | chr3:10204342-10208576 |
| 6862 | Ets2 | NM_011809.3 | chr16:95702406-95721049 | 6959 | Fabp5 | NM_001272097.1 | chr3:10012584-10016610 |
| 6863 | Etv1 | NM_001163154.1 | chr12:38783710-38868215 | 6960 | Fabp6 | NM_008375.2 | chr11:43596039-43601562 |
| 6864 | Etv2 | NM_007959.2 | chr7:30633615-30635852 | 6961 | Fabp7 | NM_021272.3 | chr10:57784922-57788450 |
| 6865 | Etv3 | NM_001083318.2 | chr3:87525577-87540158 | 6962 | Fabp9 | NM_011598.3 | chr3:10193623-10197283 |
| 6866 | Etv4 | NM_008815.3 | chr11:101769741-101785310 | 6963 | Fadd | NM_010175.5 | chr7:144578322-144582436 |
| 6867 | Etv5 | NM_023794.2 | chr16:22381312-22439570 | 6964 | Fads1 | NM_146094.2 | chr19:10182887-10196872 |
| 6868 | Etv6 | NM_007961.4 | chr6:134035699-134270147 | 6965 | Fads2 | NM_019699.1 | chr19:10064163-10101503 |
| 6869 | EU599041 | NM_001177525.1 | chr7:43214038-43226842 | 6966 | Fads3 | NM_021890.3 | chr19:10041547-10059671 |
| 6870 | Eva1a | NM_145570.2 | chr6:82041627-82093099 | 6967 | Fads6 | NM_178035.4 | chr11:115283365-115297546 |
| 6871 | Eva1b | NM_172145.3 | chr4:126148002-126149874 | 6968 | Faf1 | NM_007983.2 | chr4:109676626-109963960 |
| 6872 | Eva1c | NM_001199210.1 | chr16:90830858-90904885 | 6969 | Faf2 | NM_178397.3 | chr13:54621783-54664063 |
| 6873 | Evc | NM_021292.2 | chr5:37299170-37336881 | 6970 | Fah | NM_010176.4 | chr7:84585158-84605942 |
| 6874 | Evc2 | NM_145920.3 | chr5:37338477-37425054 | 6971 | Fahd1 | NM_023480.2 | chr17:24848895-24850302 |
| 6875 | Evi2a | NM_001033711.1 | chr11:79526560-79530609 | 6972 | Fahd2a | NM_029629.2 | chr2:127436214-127444565 |
| 6876 | Evi2a-evi2b | NM_146023.4 | chr11:79513384-79530609 | 6973 | Faim | NM_001122851.1 | chr9:98986372-99002019 |
| 6877 | Evi2b | NM_001077496.1 | chr11:79513384-79523762 | 6974 | Faim2 | NM_001038658.2 | chr15:99497004-99528165 |
| 6878 | Evi5 | NM_007964.2 | chr5:107744794-107875107 | 6975 | Faim3 | NM_026976.2 | chr1:130865776-130880790 |
| 6879 | Evi5l | NM_001039578.3 | chr8:4166566-4193701 | 6976 | Fam101a | NM_028443.2 | chr5:125003474-125012547 |
| 6880 | Evl | NM_001163394.1 | chr12:108554719-108688515 | 6977 | Fam101b | NM_029658.1 | chr11:76019194-76027782 |
| 6881 | Evpl | NM_025276.3 | chr11:116220558-116238091 | 6978 | Fam102a | NM_153560.4 | chr2:32535358-32569750 |
| 6882 | Evx1 | NM_007966.4 | chr6:52313497-52318378 | 6979 | Fam102b | NM_001163567.1 | chr3:108970996-109027607 |
| 6883 | Evx2 | NM_007967.3 | chr2:74652990-74659557 | 6980 | Fam103a1 | NM_025997.2 | chr7:81762952-81769490 |
| 6884 | Ewsr1 | NM_001283061.1 | chr11:5069686-5099088 | 6981 | Fam104a | NM_138598.5 | chr11:113661318-113684151 |
| 6885 | Exd1 | NM_172857.2 | chr2:119519403-119547627 | 6982 | Fam105a | NM_001242423.1 | chr15:27655070-27681542 |
| 6886 | Exd2 | NM_133798.3 | chr12:80463094-80498135 | 6983 | Fam107a | NM_183187.3 | chr14:8296277-8309776 |

Fig.21 - 37

| | | | |
|---|---|---|---|
| 6984 | Fam107b | NM_025626.4 | chr2:3713457-3782134 |
| 6985 | Fam109a | NM_175474.3 | chr5:121849027-121854599 |
| 6986 | Fam109b | NM_177391.4 | chr15:82341178-82345710 |
| 6987 | Fam110a | NM_001289150.1 | chr2:151969395-151973981 |
| 6988 | Fam110b | NM_173426.2 | chr4:5644178-5799944 |
| 6989 | Fam110c | NM_027828.2 | chr12:31073967-31079940 |
| 6990 | Fam111a | NM_026640.2 | chr19:12573524-12589696 |
| 6991 | Fam114a1 | NM_026667.3 | chr5:64970074-65041901 |
| 6992 | Fam114a2 | NM_001168667.1 | chr11:57482989-57518644 |
| 6993 | Fam115a | NM_029930.2 | chr6:42672546-42693059 |
| 6994 | Fam115c | NM_146174.1 | chr6:42623042-42645041 |
| 6995 | Fam115e | NM_203396.1 | chr6:42587212-42597372 |
| 6996 | Fam117a | NM_172543.4 | chr11:95337017-95381872 |
| 6997 | Fam117b | NM_001037725.3 | chr1:59913005-59985348 |
| 6998 | Fam118a | NM_133750.4 | chr15:85037061-85062830 |
| 6999 | Fam118b | NM_001286604.1 | chr9:35216964-35267805 |
| 7000 | Fam120a | NM_001033268.2 | chr13:48879216-48967828 |
| 7001 | Fam120aos | NR_015601.1 | chr13:48968111-48969905 |
| 7002 | Fam120b | NM_024203.3 | chr17:15396245-15433581 |
| 7003 | Fam120c | NM_198105.2 | chrX:151344222-151474134 |
| 7004 | Fam122a | NM_026520.3 | chr19:24475778-24477474 |
| 7005 | Fam122b | NM_001166365.2 | chrX:53243414-53269805 |
| 7006 | Fam122c | NM_028671.2 | chrX:53273432-53301501 |
| 7007 | Fam124a | NM_001243857.1 | chr14:62555736-62608485 |
| 7008 | Fam124b | NM_173425.3 | chr1:80198698-80213944 |
| 7009 | Fam126a | NM_053090.3 | chr5:23960935-24030690 |
| 7010 | Fam126b | NM_172513.3 | chr1:58522805-58586333 |
| 7011 | Fam129a | NM_022018.3 | chr1:151571372-151719347 |
| 7012 | Fam129b | NM_146119.2 | chr2:32876133-32925253 |
| 7013 | Fam129c | NM_001166213.1 | chr8:71597645-71608149 |
| 7014 | Fam131a | NM_133778.2 | chr16:20695056-20703036 |
| 7015 | Fam131b | NM_001113327.2 | chr6:42315304-42324640 |
| 7016 | Fam131c | NM_001085513.2 | chr4:141368191-141384174 |
| 7017 | Fam132a | NM_026125.3 | chr4:155962311-155966629 |
| 7018 | Fam132b | NM_173395.2 | chr1:91366429-91374217 |
| 7019 | Fam133b | NM_001042501.1 | chr5:3543832-3570546 |
| 7020 | Fam134a | NM_170755.2 | chr1:75142785-75147909 |
| 7021 | Fam134b | NM_001034851.2 | chr15:25843297-25973696 |
| 7022 | Fam134c | NM_026501.2 | chr11:101096515-101119824 |
| 7023 | Fam135a | NM_026604.4 | chr1:24010757-24100341 |
| 7024 | Fam135b | NM_177819.3 | chr15:71445677-71727838 |
| 7025 | Fam136a | NM_025591.2 | chr6:86365682-86370058 |
| 7026 | Fam13a | NM_153574.2 | chr6:58933535-59024502 |
| 7027 | Fam13b | NM_146084.1 | chr18:34442350-34506823 |
| 7028 | Fam13c | NM_001143776.1 | chr10:70440909-70558732 |
| 7029 | Fam149a | NM_153535.2 | chr8:45336714-45382291 |
| 7030 | Fam149b | NM_001024512.2 | chr14:20348161-20383491 |
| 7031 | Fam150a | NM_001195732.1 | chr1:6359330-6394731 |
| 7032 | Fam150b | NM_001159743.1 | chr12:30884323-30893854 |
| 7033 | Fam151a | NM_146149.1 | chr4:106733914-106748292 |
| 7034 | Fam151b | NM_001163627.1 | chr13:92449619-92484031 |
| 7035 | Fam154a | NM_001081096.1 | chr4:86444697-86558303 |
| 7036 | Fam154b | NM_177894.4 | chr7:82632959-82648528 |
| 7037 | Fam155a | NM_173446.2 | chr8:9206009-9771023 |
| 7038 | Fam159a | NM_001099303.2 | chr4:108367776-108383655 |
| 7039 | Fam159b | NM_029984.1 | chr13:104845282-104863893 |
| 7040 | Fam160a1 | NM_172682.3 | chr3:85660062-85746209 |
| 7041 | Fam160a2 | NM_001242363.2 | chr7:105371210-105400054 |
| 7042 | Fam160b1 | NM_145505.4 | chr19:57361008-57389594 |
| 7043 | Fam160b2 | NM_194345.1 | chr14:70583294-70599835 |
| 7044 | Fam161a | NM_028672.2 | chr11:23013386-23023741 |
| 7045 | Fam161b | NM_172581.2 | chr12:84345316-84361821 |
| 7046 | Fam162a | NM_027342.1 | chr16:36043843-36071515 |
| 7047 | Fam162b | NM_029894.1 | chr10:51585449-51590460 |
| 7048 | Fam163a | NM_177838.3 | chr1:156075955-156205026 |
| 7049 | Fam163b | NM_175427.4 | chr2:27110378-27142477 |
| 7050 | Fam166a | NM_026624.3 | chr2:25218744-25222281 |
| 7051 | Fam166b | NM_001162381.1 | chr4:43427019-43429134 |
| 7052 | Fam167a | NM_177628.4 | chr14:63436393-63465502 |
| 7053 | Fam167b | NM_182783.2 | chr4:129576814-129578580 |
| 7054 | Fam168a | NM_178764.3 | chr7:100706701-100841630 |
| 7055 | Fam168b | NM_001160235.1 | chr1:34813217-34843050 |
| 7056 | Fam169a | NM_001100458.1 | chr13:97071642-97129522 |
| 7057 | Fam169b | NM_001013811.2 | chr7:68273838-68363092 |
| 7058 | Fam170a | NM_001004061.1 | chr18:50278368-50283019 |
| 7059 | Fam170b | NM_001164485.1 | chr14:32833961-32836788 |
| 7060 | Fam171a1 | NM_001081161.1 | chr2:3118387-3227809 |
| 7061 | Fam171a2 | NM_199200.2 | chr11:102436980-102447663 |
| 7062 | Fam171b | NM_175514.2 | chr2:83812727-83881358 |
| 7063 | Fam172a | NM_001163419.1 | chr13:77708689-78166240 |
| 7064 | Fam173a | NM_001285982.1 | chr17:25790499-25792387 |
| 7065 | Fam173b | NM_026546.3 | chr15:31602115-31617535 |
| 7066 | Fam174a | NM_026321.4 | chr1:95313627-95335284 |
| 7067 | Fam174b | NM_001162532.1 | chr7:73740306-73776919 |
| 7068 | Fam175a | NM_172405.3 | chr5:100804801-100820935 |
| 7069 | Fam175b | NM_198017.3 | chr7:132859224-132885114 |
| 7070 | Fam178a | NM_001081225.1 | chr19:44931118-44983787 |
| 7071 | Fam178b | NM_001126046.1 | chr1:36562695-36683183 |
| 7072 | Fam179a | NM_177087.4 | chr17:71673260-71729669 |
| 7073 | Fam179b | NM_177805.3 | chr12:64965741-65022573 |
| 7074 | Fam180a | NM_173375.1 | chr6:35312745-35326141 |
| 7075 | Fam181a | NM_001195726.1 | chr12:103314958-103317065 |
| 7076 | Fam181b | NM_021427.2 | chr7:93079878-93081721 |
| 7077 | Fam183b | NM_001162878.1 | chr11:58792801-58801960 |
| 7078 | Fam184a | NM_001081428.1 | chr10:53633144-53750909 |
| 7079 | Fam184b | NM_021416.3 | chr5:45529704-45639501 |
| 7080 | Fam185a | NM_177869.4 | chr5:21424902-21482124 |
| 7081 | Fam186b | NM_001081254.1 | chr15:99271017-99287180 |
| 7082 | Fam187a | NM_025766.2 | chr11:102885168-102886731 |
| 7083 | Fam187b | NM_001242647.1 | chr7:30981920-30989725 |
| 7084 | Fam188a | NM_024185.4 | chr2:12347263-12419460 |
| 7085 | Fam188b | NM_001142781.1 | chr6:55203382-55320222 |
| 7086 | Fam189a1 | NM_183087.4 | chr7:64756095-65156528 |
| 7087 | Fam189a2 | NM_001114174.1 | chr19:23972749-24031019 |
| 7088 | Fam189b | NM_001014995.1 | chr3:89183224-89189289 |
| 7089 | Fam192a | NM_028221.4 | chr8:94574940-94601726 |
| 7090 | Fam193a | NM_001243123.1 | chr5:34369932-34486458 |
| 7091 | Fam193b | NM_145382.4 | chr13:55539315-55571120 |
| 7092 | Fam195a | NM_026633.3 | chr17:25863697-25868738 |
| 7093 | Fam195b | NM_001033231.2 | chr11:120542887-120549727 |
| 7094 | Fam196a | NM_001143802.1 | chr7:134881907-134938430 |
| 7095 | Fam196b | NM_001025382.2 | chr11:34314821-34422640 |
| 7096 | Fam198a | NM_001199927.1 | chr9:121951019-121980208 |
| 7097 | Fam198b | NM_133187.3 | chr3:79885929-79946278 |
| 7098 | Fam199x | NM_146261.2 | chrX:137049593-137082501 |
| 7099 | Fam19a1 | NM_182808.3 | chr6:96113153-96657198 |
| 7100 | Fam19a2 | NM_001252387.1 | chr10:123264075-123741204 |
| 7101 | Fam19a3 | NM_183224.3 | chr3:104767404-104777547 |
| 7102 | Fam19a4 | NM_177233.5 | chr6:96831208-97060411 |
| 7103 | Fam19a5 | NM_001252310.1 | chr15:87544298-87759364 |
| 7104 | Fam203a | NM_021555.2 | chr15:76368897-76371411 |
| 7105 | Fam204a | NM_029648.6 | chr19:60198585-60226697 |
| 7106 | Fam206a | NM_001081420.1 | chr4:56802328-56809605 |
| 7107 | Fam207a | NM_133998.3 | chr10:77486654-77515813 |
| 7108 | Fam208a | NM_001114879.1 | chr14:27428846-27483554 |
| 7109 | Fam208b | NM_134063.3 | chr13:3566034-3611108 |
| 7110 | Fam209 | NM_029608.1 | chr2:172472553-172474316 |
| 7111 | Fam20a | NM_153782.1 | chr11:109672925-109722256 |
| 7112 | Fam20b | NM_145413.4 | chr1:156678570-156718910 |
| 7113 | Fam20c | NM_030565.6 | chr5:138755080-138810063 |
| 7114 | Fam21 | NM_026585.3 | chr6:116208032-116262671 |
| 7115 | Fam210a | NM_153794.4 | chr18:68260184-68300333 |
| 7116 | Fam210b | NM_025912.4 | chr2:172345576-172355749 |
| 7117 | Fam212a | NM_026597.3 | chr9:107984223-107985916 |
| 7118 | Fam212b | NM_001163356.1 | chr3:105705457-105720842 |
| 7119 | Fam213a | NM_027464.3 | chr14:40993739-41013775 |
| 7120 | Fam213b | NM_025582.3 | chr4:154896429-154899043 |
| 7121 | Fam214a | NM_001113283.1 | chr9:74953052-75032468 |
| 7122 | Fam214b | NM_001253353.1 | chr4:43032413-43040301 |
| 7123 | Fam216a | NM_026883.3 | chr5:122364583-122371963 |
| 7124 | Fam216b | NM_177629.4 | chr14:78081023-78089007 |
| 7125 | Fam217a | NM_027967.1 | chr13:34909963-34919992 |
| 7126 | Fam217b | NM_001081289.1 | chr2:178414533-178422161 |
| 7127 | Fam219a | NM_001159583.1 | chr4:41518928-41569527 |
| 7128 | Fam219aos | NR_045726.1 | chr4:41517436-41518929 |
| 7129 | Fam219b | NM_001166364.1 | chr9:57537527-57543187 |
| 7130 | Fam220a | NM_026050.2 | chr5:143548705-143564525 |
| 7131 | Fam221a | NM_001172216.1 | chr6:49367738-49389904 |
| 7132 | Fam221b | NM_175517.3 | chr4:43659621-43668859 |
| 7133 | Fam222a | NM_001004180.1 | chr5:114568250-114613218 |
| 7134 | Fam222b | NM_145430.2 | chr11:78094672-78157339 |
| 7135 | Fam227a | NM_029407.1 | chr15:79609575-79658956 |
| 7136 | Fam227b | NM_029455.3 | chr2:125983482-126152004 |
| 7137 | Fam228a | NM_029107.2 | chr12:4713804-4738383 |
| 7138 | Fam228b | NM_175431.4 | chr12:4746215-4769267 |
| 7139 | Fam229a | NM_001085491.2 | chr4:129491189-129491956 |
| 7140 | Fam229b | NM_183254.1 | chr10:39118807-39133895 |
| 7141 | Fam24a | NM_183272.2 | chr7:131334621-131336716 |
| 7142 | Fam25c | NM_183278.2 | chr14:34351881-34355393 |
| 7143 | Fam26d | NM_001081165.1 | chr10:34038783-34044313 |
| 7144 | Fam26e | NM_178908.3 | chr10:34091351-34096519 |
| 7145 | Fam26f | NM_175449.4 | chr10:34126066-34127972 |
| 7146 | Fam32a | NM_026455.4 | chr8:72219729-72223775 |
| 7147 | Fam35a | NM_029389.2 | chr14:34237034-34310503 |
| 7148 | Fam3a | NM_025473.4 | chrX:74384719-74393274 |
| 7149 | Fam3b | NM_020622.2 | chr16:97471085-97504936 |
| 7150 | Fam3c | NM_138587.4 | chr6:22306521-22356081 |
| 7151 | Fam43a | NM_177632.3 | chr16:30599722-30602797 |
| 7152 | Fam43b | NM_001081672.2 | chr4:138394091-138396458 |
| 7153 | Fam45a | NM_001167829.1 | chr19:60811569-60836229 |
| 7154 | Fam46a | NM_001160378.1 | chr9:85320438-85327124 |
| 7155 | Fam46b | NM_175307.6 | chr4:133480132-133487940 |
| 7156 | Fam46c | NM_001142952.1 | chr3:100471535-100489192 |
| 7157 | Fam46d | NM_001163104.2 | chrX:107816476-107872911 |
| 7158 | Fam47c | NM_001164739.1 | chrX:78737762-78739410 |
| 7159 | Fam47e | NM_001033478.2 | chr5:92571536-92591284 |
| 7160 | Fam49a | NM_001146119.1 | chr12:12262138-12376361 |
| 7161 | Fam49b | NM_144846.5 | chr15:63929093-64060448 |
| 7162 | Fam50a | NM_138607.3 | chrX:74313032-74320149 |
| 7163 | Fam50b | NM_138746.2 | chr13:34739641-34747622 |
| 7164 | Fam53a | NM_178390.3 | chr5:33600352-33629635 |
| 7165 | Fam53b | NM_175268.4 | chr7:132712083-132786935 |
| 7166 | Fam53c | NM_175104.4 | chr18:34758905-34773760 |
| 7167 | Fam57a | NM_027773.3 | chr11:76202055-76208257 |
| 7168 | Fam57b | NM_001146347.1 | chr7:126824652-126830219 |
| 7169 | Fam58b | NM_197989.1 | chr11:78750505-78751729 |
| 7170 | Fam60a | NM_019643.3 | chr6:148921058-148946432 |
| 7171 | Fam63a | NM_133858.4 | chr3:95282934-95296164 |
| 7172 | Fam63b | NM_172772.2 | chr9:70599013-70657174 |
| 7173 | Fam64a | NM_144526.3 | chr11:72042501-72047370 |
| 7174 | Fam65a | NM_001081241.2 | chr8:105605228-105622218 |
| 7175 | Fam65b | NM_001080381.1 | chr13:24638647-24706001 |
| 7176 | Fam65c | NM_001080708.2 | chr2:167980785-168010593 |
| 7177 | Fam69a | NM_026062.4 | chr5:107908041-107987077 |

Fig.21 - 38

| | | | |
|---|---|---|---|
| 7178 | Fam69b | NM_019833.3 | chr2:26628456-26636497 |
| 7179 | Fam69c | NM_173770.4 | chr18:84720241-84740436 |
| 7180 | Fam71a | NM_001109759.1 | chr1:191162583-191164817 |
| 7181 | Fam71b | NM_001013783.2 | chr11:46404727-46407985 |
| 7182 | Fam71d | NM_027597.4 | chr12:78691534-78734519 |
| 7183 | Fam71e1 | NM_028169.1 | chr7:44496587-44501134 |
| 7184 | Fam71e2 | NM_172895.3 | chr7:4753225-4771270 |
| 7185 | Fam71f1 | NM_001289663.1 | chr6:29319139-29336022 |
| 7186 | Fam71f2 | NM_001101486.1 | chr6:29281140-29290676 |
| 7187 | Fam72a | NM_175382.3 | chr1:131527988-131539872 |
| 7188 | Fam73a | NM_001162375.1 | chr3:152273459-152340407 |
| 7189 | Fam73b | NM_001242407.1 | chr2:30364232-30385519 |
| 7190 | Fam76a | NM_001163792.1 | chr4:132899212-132922551 |
| 7191 | Fam76b | NM_176836.3 | chr9:13827726-13846522 |
| 7192 | Fam78a | NM_175511.4 | chr2:32066884-32083705 |
| 7193 | Fam78b | NM_001160261.1 | chr1:167001416-167091302 |
| 7194 | Fam81a | NM_029784.2 | chr9:70089309-70141557 |
| 7195 | Fam83a | NM_173862.2 | chr15:57985902-58010702 |
| 7196 | Fam83b | NM_001045518.1 | chr9:76490704-76545804 |
| 7197 | Fam83c | NM_027788.2 | chr2:155829182-155834854 |
| 7198 | Fam83d | NM_027975.2 | chr2:158768098-158786637 |
| 7199 | Fam83e | NM_001033170.4 | chr7:45721219-45729492 |
| 7200 | Fam83f | NM_145986.2 | chr15:80671846-80700425 |
| 7201 | Fam83g | NM_178618.3 | chr11:61684409-61709950 |
| 7202 | Fam83h | NM_001168253.1 | chr15:76001091-76009498 |
| 7203 | Fam84a | NM_029007.2 | chr12:14147597-14152038 |
| 7204 | Fam84b | NM_001162926.1 | chr15:60818995-60825080 |
| 7205 | Fam86 | NM_027446.2 | chr16:5244154-5255956 |
| 7206 | Fam89a | NM_001081120.1 | chr8:124740256-124751809 |
| 7207 | Fam89b | NM_023166.2 | chr19:5728086-5729666 |
| 7208 | Fam92a | NM_026558.5 | chr4:12153141-12172015 |
| 7209 | Fam92b | NM_001033980.2 | chr8:120166396-120177469 |
| 7210 | Fam96a | NM_026635.3 | chr9:66126610-66138968 |
| 7211 | Fam96b | NM_026753.2 | chr8:104639838-104641728 |
| 7212 | Fam98a | NM_133747.2 | chr17:75537085-75551946 |
| 7213 | Fam98b | NM_026620.3 | chr2:117249738-117271540 |
| 7214 | Fam98c | NM_001146023.1 | chr7:29152509-29156210 |
| 7215 | Fan1 | NM_177893.3 | chr7:64346757-64374095 |
| 7216 | Fanca | NM_016925.3 | chr8:123268243-123318576 |
| 7217 | Fancb | NM_001146081.1 | chrX:164980591-164997272 |
| 7218 | Fancc | NM_001042673.2 | chr13:63304708-63431745 |
| 7219 | Fancd2 | NM_001033244.3 | chr6:113531681-113596285 |
| 7220 | Fancd2os | NM_027633.3 | chr6:113596761-113600715 |
| 7221 | Fance | NM_001163819.1 | chr17:28313529-28326574 |
| 7222 | Fancf | NM_001115087.1 | chr7:51860576-51862267 |
| 7223 | Fancg | NM_001163233.1 | chr4:43002336-43010301 |
| 7224 | Fanci | NM_145946.2 | chr7:79392337-79450264 |
| 7225 | Fancl | NM_001277273.1 | chr11:26387083-26471883 |
| 7226 | Fancm | NM_178912.3 | chr12:65075605-65132058 |
| 7227 | Fank1 | NM_025850.2 | chr7:133776890-133881532 |
| 7228 | Fap | NM_007986.3 | chr2:62500935-62574021 |
| 7229 | Far1 | NM_001285831.1 | chr7:113513833-113570888 |
| 7230 | Far2 | NM_178797.3 | chr6:148047415-148182760 |
| 7231 | Farp1 | NM_134082.3 | chr14:121035573-121283726 |
| 7232 | Farp2 | NM_145519.2 | chr1:93512103-93621976 |
| 7233 | Fars2 | NM_001039189.2 | chr13:36117642-36537595 |
| 7234 | Farsa | NM_025648.3 | chr8:84856985-84869257 |
| 7235 | Farsb | NM_001278075.1 | chr1:78417957-78488897 |
| 7236 | Fas | NM_001146708.1 | chr19:34290658-34309350 |
| 7237 | Fasl | NM_001205243.1 | chr1:161780691-161788495 |
| 7238 | Fasn | NM_007988.3 | chr11:120805957-120824547 |
| 7239 | Fastk | NM_023229.2 | chr5:24441039-24445235 |
| 7240 | Fastkd1 | NM_177244.3 | chr2:69686823-69712606 |
| 7241 | Fastkd2 | NM_172422.3 | chr1:63730624-63753385 |
| 7242 | Fastkd3 | NM_027123.4 | chr13:68582247-68592279 |
| 7243 | Fastkd5 | NM_001146084.1 | chr2:130613837-130630027 |
| 7244 | Fat1 | NM_001081286.2 | chr8:44950207-45052257 |
| 7245 | Fat2 | NM_001029988.2 | chr11:55250609-55312257 |
| 7246 | Fat3 | NM_001080814.1 | chr9:15910192-16378231 |
| 7247 | Fat4 | NM_183221.3 | chr3:38886939-39011983 |
| 7248 | Fate1 | NR_003243.2 | chrX:71972985-71989046 |
| 7249 | Fau | NM_001160239.2 | chr19:6057887-6059524 |
| 7250 | Faxc | NM_175234.4 | chr4:21931325-22001461 |
| 7251 | Fbf1 | NM_172571.3 | chr11:116142284-116168178 |
| 7252 | Fbl | NM_007991.3 | chr7:28169747-28179269 |
| 7253 | Fblim1 | NM_001163256.1 | chr4:141576061-141599924 |
| 7254 | Fbll1 | NM_001004147.3 | chr11:35797379-35798884 |
| 7255 | Fbln1 | NM_010180.2 | chr15:85206007-85286294 |
| 7256 | Fbln2 | NM_001081437.1 | chr6:91212763-91272540 |
| 7257 | Fbln5 | NM_011812.4 | chr12:101746564-101819119 |
| 7258 | Fbln7 | NM_024237.4 | chr2:128863931-128897034 |
| 7259 | Fbn1 | NM_007993.2 | chr2:125300593-125506438 |
| 7260 | Fbn2 | NM_010181.2 | chr18:58008622-58209926 |
| 7261 | Fbp1 | NM_019395.3 | chr13:62864752-62888282 |
| 7262 | Fbp2 | NM_007994.3 | chr13:62836884-62858370 |
| 7263 | Fbrs | NM_010183.1 | chr7:127485220-127491513 |
| 7264 | Fbrsl1 | NM_001142642.1 | chr5:110361751-110448503 |
| 7265 | Fbxl12 | NM_001002846.2 | chr9:20637748-20644767 |
| 7266 | Fbxl12os | NR_033729.1 | chr9:20607474-20617271 |
| 7267 | Fbxl13 | NM_001199632.1 | chr5:21483846-21645605 |
| 7268 | Fbxl14 | NM_133940.3 | chr6:119479667-119483886 |
| 7269 | Fbxl15 | NM_133694.2 | chr19:46328183-46330446 |
| 7270 | Fbxl16 | NM_001164225.1 | chr17:25809084-25821265 |
| 7271 | Fbxl17 | NM_015794.1 | chr17:63045951-63500580 |
| 7272 | Fbxl18 | NM_001033312.3 | chr5:142871788-142895238 |
| 7273 | Fbxl19 | NM_172748.2 | chr7:127746774-127768928 |
| 7274 | Fbxl2 | NM_178624.6 | chr9:113976957-114026751 |

| | | | |
|---|---|---|---|
| 7275 | Fbxl20 | NM_028149.1 | chr11:98082553-98149616 |
| 7276 | Fbxl21 | NM_178674.4 | chr13:56522507-56537786 |
| 7277 | Fbxl22 | NM_175206.4 | chr9:66508458-66514593 |
| 7278 | Fbxl3 | NM_015822.2 | chr14:103080238-103099509 |
| 7279 | Fbxl4 | NM_172988.4 | chr4:22357542-22434091 |
| 7280 | Fbxl5 | NM_001159963.1 | chr5:43744617-43782149 |
| 7281 | Fbxl6 | NM_013909.2 | chr15:76535727-76538746 |
| 7282 | Fbxl7 | NM_176959.3 | chr15:26540458-26895564 |
| 7283 | Fbxl8 | NM_015821.2 | chr8:105264647-105269326 |
| 7284 | Fbxo10 | NM_001024142.1 | chr4:45034247-45084604 |
| 7285 | Fbxo11 | NM_001081034.1 | chr17:87990858-88065285 |
| 7286 | Fbxo15 | NM_015798.3 | chr18:84935024-84981392 |
| 7287 | Fbxo16 | NM_015795.1 | chr14:65266700-65321502 |
| 7288 | Fbxo17 | NM_015796.2 | chr7:28716789-28738140 |
| 7289 | Fbxo18 | NM_015792.1 | chr2:11742572-11777527 |
| 7290 | Fbxo2 | NM_176848.1 | chr4:148160667-148166417 |
| 7291 | Fbxo21 | NM_145564.3 | chr5:117976769-118010191 |
| 7292 | Fbxo22 | NM_028049.2 | chr9:55208934-55224433 |
| 7293 | Fbxo24 | NM_027708.1 | chr5:137612504-137625078 |
| 7294 | Fbxo25 | NM_025785.2 | chr8:13907805-13940521 |
| 7295 | Fbxo27 | NM_001163702.1 | chr7:28693143-28699337 |
| 7296 | Fbxo28 | NM_175127.2 | chr1:182313101-182341606 |
| 7297 | Fbxo3 | NM_020593.2 | chr2:104027798-104055127 |
| 7298 | Fbxo30 | NM_001168297.1 | chr10:11281586-11297969 |
| 7299 | Fbxo31 | NM_133765.4 | chr8:121549442-121578806 |
| 7300 | Fbxo32 | NM_026346.3 | chr15:58175878-58214892 |
| 7301 | Fbxo33 | NM_001033156.4 | chr12:59200654-59219483 |
| 7302 | Fbxo34 | NM_001146085.1 | chr14:47472560-47531962 |
| 7303 | Fbxo36 | NM_025386.3 | chr1:84839840-84900486 |
| 7304 | Fbxo38 | NM_134136.3 | chr18:62504058-62548743 |
| 7305 | Fbxo39 | NM_001099688.2 | chr11:72314443-72319419 |
| 7306 | Fbxo4 | NM_134099.2 | chr15:3963563-3979573 |
| 7307 | Fbxo40 | NM_001037321.1 | chr16:36966072-36979136 |
| 7308 | Fbxo41 | NM_001001160.3 | chr6:85469575-85502994 |
| 7309 | Fbxo42 | NM_172518.3 | chr4:141147921-141204062 |
| 7310 | Fbxo43 | NM_001081253.1 | chr15:36150059-36164884 |
| 7311 | Fbxo44 | NM_001161851.2 | chr4:148152798-148159589 |
| 7312 | Fbxo45 | NM_173439.2 | chr16:32230111-32247025 |
| 7313 | Fbxo46 | NM_175530.3 | chr7:19119858-19138261 |
| 7314 | Fbxo47 | NM_001081435.1 | chr11:97854306-97884154 |
| 7315 | Fbxo48 | NM_176982.2 | chr11:16951409-16954772 |
| 7316 | Fbxo5 | NM_025995.2 | chr10:5799157-5805465 |
| 7317 | Fbxo6 | NM_001163704.1 | chr4:148145715-148151925 |
| 7318 | Fbxo7 | NM_153195.2 | chr10:86021928-86048328 |
| 7319 | Fbxo8 | NM_015791.3 | chr8:56551133-56593939 |
| 7320 | Fbxo9 | NM_001081490.2 | chr9:78081499-78109065 |
| 7321 | Fbxw10 | NM_001033669.2 | chr11:62847122-62877462 |
| 7322 | Fbxw11 | NM_001271347.1 | chr11:32642554-32746814 |
| 7323 | Fbxw13 | NM_177598.3 | chr9:109179226-109195975 |
| 7324 | Fbxw14 | NM_015793.2 | chr9:109271124-109287676 |
| 7325 | Fbxw15 | NM_199036.2 | chr9:109552601-109568262 |
| 7326 | Fbxw16 | NM_177070.3 | chr9:109432317-109449140 |
| 7327 | Fbxw17 | NM_175401.3 | chr13:50417876-50433769 |
| 7328 | Fbxw18 | NM_001033794.3 | chr9:109676733-109702700 |
| 7329 | Fbxw19 | NM_177703.3 | chr9:109478574-109495854 |
| 7330 | Fbxw2 | NM_001164768.1 | chr2:34804363-34823181 |
| 7331 | Fbxw20 | NM_001008428.3 | chr9:109217431-109234754 |
| 7332 | Fbxw21 | NM_177069.3 | chr9:109139453-109162022 |
| 7333 | Fbxw22 | NM_001014395.2 | chr9:109378408-109404294 |
| 7334 | Fbxw24 | NM_001013776.4 | chr9:109601115-109626059 |
| 7335 | Fbxw26 | NM_198674.2 | chr9:109717565-109746089 |
| 7336 | Fbxw28 | NM_001177419.1 | chr9:109322885-109339659 |
| 7337 | Fbxw4 | NM_013907.2 | chr19:45578256-45660193 |
| 7338 | Fbxw5 | NM_013908.4 | chr2:25500777-25505470 |
| 7339 | Fbxw7 | NM_001177773.1 | chr3:84815576-84979198 |
| 7340 | Fbxw8 | NM_172721.2 | chr5:118064980-118155458 |
| 7341 | Fbxw9 | NM_026791.2 | chr8:85060118-85067120 |
| 7342 | Fcamr | NM_001170632.1 | chr1:130800901-130814740 |
| 7343 | Fcer1a | NM_010184.2 | chr1:173221270-173227229 |
| 7344 | Fcer1g | NM_010185.4 | chr1:171229571-171234349 |
| 7345 | Fcer2a | NM_001253737.1 | chr8:3681736-3690861 |
| 7346 | Fcf1 | NM_028632.2 | chr12:84970929-84983303 |
| 7347 | Fcgbp | NM_001122603.1 | chr7:28071235-28120864 |
| 7348 | Fcgr1 | NM_010186.5 | chr3:96282908-96293969 |
| 7349 | Fcgr2b | NM_001077189.1 | chr1:170960558-170976071 |
| 7350 | Fcgr3 | NM_010188.5 | chr1:171051168-171059403 |
| 7351 | Fcgr4 | NM_144559.2 | chr1:171018925-171029761 |
| 7352 | Fcgrt | NM_010189.3 | chr7:45092992-45103822 |
| 7353 | Fcho1 | NM_028715.3 | chr8:71708386-71725681 |
| 7354 | Fcho2 | NM_172591.3 | chr13:98723405-98815449 |
| 7355 | Fchsd1 | NM_175684.4 | chr18:37957433-37969731 |
| 7356 | Fchsd2 | NM_001146010.1 | chr7:101108774-101284405 |
| 7357 | Fcna | NM_007995.3 | chr2:25624666-25627974 |
| 7358 | Fcnb | NM_010190.1 | chr2:28076478-28084878 |
| 7359 | Fcrl1 | NM_001136236.1 | chr3:87376386-87392133 |
| 7360 | Fcrl5 | NM_001113238.1 | chr3:87435781-87500678 |
| 7361 | Fcrl6 | NM_001164725.1 | chr1:172596639-172602551 |
| 7362 | Fcrla | NM_001160215.1 | chr1:170917593-170927583 |
| 7363 | Fcrlb | NM_001029984.2 | chr1:170907272-170912941 |
| 7364 | Fcrls | NM_030707.3 | chr3:87250964-87263524 |
| 7365 | Fdft1 | NM_010191.3 | chr14:63145150-63179578 |
| 7366 | Fdps | NM_001253751.1 | chr3:89093587-89101967 |
| 7367 | Fdx1 | NM_007996.2 | chr9:51943024-51963602 |
| 7368 | Fdx1l | NM_001039824.2 | chr9:21067519-21073514 |
| 7369 | Fdxacb1 | NM_198675.2 | chr9:50768237-50772670 |
| 7370 | Fdxr | NM_007997.1 | chr11:115268024-115276969 |
| 7371 | Fech | NM_007998.6 | chr18:64456549-64489066 |

Fig.21 - 39

| | | | | | | | |
|---|---|---|---|---|---|---|---|
| 7372 | Fem1a | NM_010192.4 | chr17:56256792-56263608 | 7469 | Fjx1 | NM_010218.2 | chr2:102449365-102451792 |
| 7373 | Fem1b | NM_010193.4 | chr9:62791829-62811648 | 7470 | Fkbp10 | NM_001163481.1 | chr11:100415693-100424840 |
| 7374 | Fem1c | NM_173423.4 | chr18:46504605-46525971 | 7471 | Fkbp11 | NM_024169.3 | chr15:98724367-98728198 |
| 7375 | Fen1 | NM_001271614.1 | chr19:10199131-10203943 | 7472 | Fkbp14 | NM_153573.1 | chr6:54577604-54593128 |
| 7376 | Fendrr | NR_045471.2 | chr8:121054881-121083110 | 7473 | Fkbp15 | NM_001045528.1 | chr4:62300341-62360548 |
| 7377 | Fer1l4 | NM_001136556.1 | chr2:156019139-156052947 | 7474 | Fkbp1a | NM_008019.3 | chr2:151542482-151561691 |
| 7378 | Fer1l5 | NM_001277076.1 | chr1:36372290-36422110 | 7475 | Fkbp1b | NM_016863.3 | chr12:4833173-4841595 |
| 7379 | Ferd3l | NM_033522.2 | chr12:33928424-33929309 | 7476 | Fkbp2 | NM_001166368.1 | chr19:6977738-6980461 |
| 7380 | Fermt1 | NM_198029.2 | chr2:132904178-132946036 | 7477 | Fkbp3 | NM_013902.4 | chr12:65062431-65073938 |
| 7381 | Fermt2 | NM_146054.2 | chr14:45458791-45530065 | 7478 | Fkbp4 | NM_010219.3 | chr6:128430106-128438631 |
| 7382 | Fermt3 | NM_153795.2 | chr19:6998957-7019469 | 7479 | Fkbp5 | NM_010220.4 | chr17:28398752-28486149 |
| 7383 | Fert2 | NM_001037997.3 | chr17:63863980-64139496 | 7480 | Fkbp6 | NM_001277891.1 | chr5:135337364-135350044 |
| 7384 | Fes | NM_010194.2 | chr7:80377757-80387946 | 7481 | Fkbp7 | NM_010222.2 | chr2:76663033-76673098 |
| 7385 | Fetub | NM_001083904.1 | chr16:22920221-22939768 | 7482 | Fkbp8 | NM_001111066.1 | chr8:70527742-70535328 |
| 7386 | Fev | NM_153111.2 | chr1:74881508-74885408 | 7483 | Fkbp9 | NM_012056.2 | chr6:56832058-56879360 |
| 7387 | Fez1 | NM_183171.4 | chr9:36843658-36878640 | 7484 | Fkbpl | NM_019873.2 | chr17:34644882-34646327 |
| 7388 | Fez2 | NM_001285940.1 | chr17:78377877-78418152 | 7485 | Fkrp | NM_173430.2 | chr7:16809266-16816732 |
| 7389 | Fezf1 | NM_028462.1 | chr6:23245046-23248264 | 7486 | Fktn | NM_139309.4 | chr4:53714181-53763271 |
| 7390 | Fezf2 | NM_080433.3 | chr14:12341891-12345865 | 7487 | Flad1 | NM_177041.3 | chr3:89402672-89411863 |
| 7391 | Ffar1 | NM_194057.2 | chr7:30860567-30861470 | 7488 | Flcn | NM_001271356.1 | chr11:59791407-59810039 |
| 7392 | Ffar2 | NM_001168509.1 | chr7:30818356-30821648 | 7489 | Flg2 | NM_001013804.1 | chr3:93197272-93221376 |
| 7393 | Ffar3 | NM_001033316.2 | chr7:30854329-30856178 | 7490 | Fli1 | NM_008026.5 | chr9:32422203-32541452 |
| 7394 | Ffar4 | NM_181748.2 | chr19:38097078-38114263 | 7491 | Flii | NM_022009.2 | chr11:60714145-60727263 |
| 7395 | Fga | NM_001111048.2 | chr3:83026152-83033617 | 7492 | Flna | NM_001290421.1 | chrX:74223460-74246534 |
| 7396 | Fgb | NM_181849.2 | chr3:83042304-83049790 | 7493 | Flnb | NM_001081427.1 | chr14:7817956-7951587 |
| 7397 | Fgd1 | NM_008001.4 | chrX:151047169-151089686 | 7494 | Flnc | NM_001081185.1 | chr6:29433152-29461888 |
| 7398 | Fgd2 | NM_001159538.1 | chr17:29360913-29379535 | 7495 | Flot1 | NM_008027.2 | chr17:35823356-35832787 |
| 7399 | Fgd3 | NM_015759.2 | chr13:49263109-49309208 | 7496 | Flot2 | NM_001040403.1 | chr11:78037940-78060432 |
| 7400 | Fgd4 | NM_139232.3 | chr16:16416914-16560219 | 7497 | Flrt1 | NM_201411.2 | chr19:7092010-7105729 |
| 7401 | Fgd5 | NM_172731.2 | chr6:91987109-92076005 | 7498 | Flrt2 | NM_201518.4 | chr12:95692225-95785213 |
| 7402 | Fgd6 | NM_053072.3 | chr10:94036000-94145339 | 7499 | Flrt3 | NM_001172160.1 | chr2:140658197-140671476 |
| 7403 | Fgf1 | NM_010197.3 | chr18:38838672-38918699 | 7500 | Flt1 | NM_010228.3 | chr5:147562195-147725988 |
| 7404 | Fgf10 | NM_008002.4 | chr13:118714698-118792573 | 7501 | Flt3 | NM_010229.2 | chr5:147330741-147400489 |
| 7405 | Fgf11 | NM_001291104.1 | chr11:69796067-69801716 | 7502 | Flt3l | NM_013520.3 | chr7:45131188-45136432 |
| 7406 | Fgf12 | NM_001276419.2 | chr16:28157782-28564951 | 7503 | Flt4 | NM_008029.3 | chr11:49609678-49652739 |
| 7407 | Fgf13 | NM_001290414.1 | chrX:59062145-59585572 | 7504 | Flywch1 | NM_153791.2 | chr17:23755422-23771591 |
| 7408 | Fgf14 | NM_010201.4 | chr14:123978290-124192546 | 7505 | Flywch2 | NM_029798.3 | chr17:23776917-23786077 |
| 7409 | Fgf15 | NM_008003.2 | chr7:144896531-144900951 | 7506 | Fmn1 | NM_001285458.1 | chr2:113327735-113716767 |
| 7410 | Fgf16 | NM_030614.2 | chrX:105764476-105776532 | 7507 | Fmn2 | NM_019445.2 | chr1:174501824-174822729 |
| 7411 | Fgf17 | NM_008004.4 | chr14:70636204-70642268 | 7508 | Fmnl1 | NM_001077698.1 | chr11:103171137-103198900 |
| 7412 | Fgf18 | NM_008005.2 | chr11:33116977-33147400 | 7509 | Fmnl2 | NM_172409.2 | chr2:52857867-53134202 |
| 7413 | Fgf2 | NM_008006.2 | chr3:37348652-37404830 | 7510 | Fmnl3 | NM_011711.2 | chr15:99317211-99370482 |
| 7414 | Fgf20 | NM_030610.2 | chr8:40279165-40286953 | 7511 | Fmo1 | NM_010231.2 | chr1:162829560-162866548 |
| 7415 | Fgf21 | NM_020013.4 | chr7:45613889-45615490 | 7512 | Fmo2 | NM_018881.3 | chr1:162875046-162898712 |
| 7416 | Fgf22 | NM_023304.1 | chr10:79755118-79756961 | 7513 | Fmo3 | NM_008030.2 | chr1:162953798-162984528 |
| 7417 | Fgf23 | NM_022657.4 | chr6:127072901-127082296 | 7514 | Fmo4 | NM_144878.1 | chr1:162793882-162812549 |
| 7418 | Fgf3 | NM_008007.2 | chr7:144838611-144843348 | 7515 | Fmo5 | NM_001161763.1 | chr3:97628803-97655287 |
| 7419 | Fgf4 | NM_010202.5 | chr7:144861385-144865243 | 7516 | Fmo6 | NM_001178038.1 | chr1:162916550-162937225 |
| 7420 | Fgf5 | NM_001277268.1 | chr5:98254183-98277033 | 7517 | Fmo9 | NM_172844.2 | chr1:166662054-166681845 |
| 7421 | Fgf6 | NM_010204.1 | chr6:127015541-127024718 | 7518 | Fmod | NM_021355.3 | chr1:134037514-134048277 |
| 7422 | Fgf7 | NM_008008.4 | chr2:126034657-126091185 | 7519 | Fmr1 | NM_001290424.1 | chrX:68678540-68717961 |
| 7423 | Fgf8 | NM_001166361.1 | chr19:45736797-45742884 | 7520 | Fmr1nb | NM_001166619.1 | chrX:68761838-68804560 |
| 7424 | Fgf9 | NM_013518.4 | chr14:58072685-58112720 | 7521 | Fn1 | NM_001276408.1 | chr1:71585472-71653234 |
| 7425 | Fgfbp1 | NM_001271616.1 | chr5:43978857-43981799 | 7522 | Fn3k | NM_001038699.2 | chr11:121434952-121443505 |
| 7426 | Fgfbp3 | NM_028263.1 | chr19:36917549-36919599 | 7523 | Fn3krp | NM_181420.3 | chr11:121421372-121430768 |
| 7427 | Fgfr1 | NM_001079908.2 | chr8:25518758-25575718 | 7524 | Fnbp1 | NM_001038700.2 | chr2:31026205-31142008 |
| 7428 | Fgfr1op | NM_001197046.1 | chr17:8165517-8196470 | 7525 | Fnbp1l | NM_001114665.2 | chr3:122538718-122619714 |
| 7429 | Fgfr1op2 | NM_026218.2 | chr6:146577910-146599198 | 7526 | Fnbp4 | NM_018828.2 | chr2:90745369-90781020 |
| 7430 | Fgfr2 | NM_010207.2 | chr7:130162450-130266808 | 7527 | Fnd3c2 | NM_001033424.3 | chrX:106235245-106255372 |
| 7431 | Fgfr3 | NM_001163215.2 | chr5:33721723-33737068 | 7528 | Fndc1 | NM_001081416.1 | chr17:7738567-7804974 |
| 7432 | Fgfr4 | NM_008011.2 | chr13:55152817-55168759 | 7529 | Fndc3a | NM_207636.2 | chr14:72537952-72710003 |
| 7433 | Fgfrl1 | NM_001164259.1 | chr5:108694228-108706950 | 7530 | Fndc3b | NM_173182.2 | chr3:27416161-27710439 |
| 7434 | Fgg | NM_133862.1 | chr3:83007895-83015049 | 7531 | Fndc3c1 | NM_001007580.1 | chrX:106420042-106485229 |
| 7435 | Fggy | NM_001113412.1 | chr4:95557506-95926939 | 7532 | Fndc4 | NM_022424.5 | chr5:31292245-31295877 |
| 7436 | Fgl1 | NM_145594.2 | chr8:41191433-41215156 | 7533 | Fndc5 | NM_027402.3 | chr4:129137059-129144593 |
| 7437 | Fgl2 | NM_008013.4 | chr5:21372672-21378386 | 7534 | Fndc7 | NM_001165965.1 | chr3:108853677-108890008 |
| 7438 | Fgr | NM_010208.4 | chr4:132974094-133001882 | 7535 | Fndc8 | NM_030224.1 | chr11:82892144-82900737 |
| 7439 | Fh1 | NM_010209.2 | chr1:175601377-175625635 | 7536 | Fndc9 | NM_177075.4 | chr11:46235556-46239871 |
| 7440 | Fhad1 | NM_177868.4 | chr4:141890622-142011651 | 7537 | Fnip1 | NM_173753.4 | chr11:54438178-54518241 |
| 7441 | Fhad1os1 | NR_040672.1 | chr4:141982991-141986797 | 7538 | Fnip2 | NM_001162999.2 | chr3:79455970-79567679 |
| 7442 | Fhdc1 | NM_001033301.4 | chr3:84442195-84480439 | 7539 | Fnta | NM_008033.3 | chr8:25998721-26015601 |
| 7443 | Fhit | NM_010210.3 | chr14:9550093-11162035 | 7540 | Fntb | NM_145927.2 | chr12:76837466-76921412 |
| 7444 | Fhl1 | NM_001077361.1 | chrX:56731956-56793346 | 7541 | Focad | NM_001081184.1 | chr4:88094629-88411011 |
| 7445 | Fhl2 | NM_001289533.1 | chr1:43123070-43196761 | 7542 | Folh1 | NM_001159706.1 | chr7:86718975-86775864 |
| 7446 | Fhl3 | NM_010213.3 | chr4:124700698-124708611 | 7543 | Folr1 | NM_001252552.1 | chr7:101858330-101870788 |
| 7447 | Fhl4 | NM_010214.4 | chr10:85097018-85102495 | 7544 | Folr2 | NM_008035.2 | chr7:101839987-101845331 |
| 7448 | Fhl5 | NM_021318.3 | chr4:25199908-25242876 | 7545 | Folr4 | NM_022888.2 | chr9:14900022-14903951 |
| 7449 | Fhod1 | NM_177699.4 | chr8:105329159-105347970 | 7546 | Fopnl | NM_025345.2 | chr16:14299243-14317332 |
| 7450 | Fhod3 | NM_001289654.1 | chr18:24708622-25133507 | 7547 | Fos | NM_010234.2 | chr12:85473900-85477270 |
| 7451 | Fibcd1 | NM_178887.4 | chr2:31813289-31846005 | 7548 | Fosb | NM_008036.2 | chr7:19302695-19310045 |
| 7452 | Fibin | NM_026271.1 | chr2:110360924-110362993 | 7549 | Fosl1 | NM_010235.2 | chr19:5447697-5455938 |
| 7453 | Fibp | NM_001253832.1 | chr19:5460606-5465052 | 7550 | Fosl2 | NM_008037.4 | chr5:32136471-32157839 |
| 7454 | Ficd | NM_001010825.3 | chr5:113735781-113740607 | 7551 | Foxa1 | NM_008259.3 | chr12:57540631-57546121 |
| 7455 | Fig4 | NM_133999.1 | chr10:41188171-41303241 | 7552 | Foxa2 | NM_001291065.1 | chr2:148042876-148045948 |
| 7456 | Figf | NM_010216.2 | chrX:164373521-164402650 | 7553 | Foxa3 | NM_008260.2 | chr7:19013282-19023539 |
| 7457 | Figla | NM_012013.1 | chr6:86017190-86020996 | 7554 | Foxb1 | NM_022378.3 | chr9:69757709-69760940 |
| 7458 | Fign | NM_001267846.1 | chr2:63971507-64098038 | 7555 | Foxb2 | NM_008023.1 | chr19:16872315-16873830 |
| 7459 | Fignl1 | NM_001163359.1 | chr11:11800287-11808962 | 7556 | Foxc1 | NM_008592.2 | chr13:31806645-31810635 |
| 7460 | Fignl2 | NM_001214911.2 | chr15:101050191-101054399 | 7557 | Foxc2 | NM_013519.2 | chr8:121116170-121118894 |
| 7461 | Filip1 | NM_001081243.1 | chr9:79815561-79977882 | 7558 | Foxd1 | NM_008242.2 | chr13:98354244-98356705 |
| 7462 | Filip1l | NM_001040397.4 | chr16:57353276-57572804 | 7559 | Foxd2 | NM_008593.3 | chr4:114906279-114908898 |
| 7463 | Fip1l1 | NM_001159573.1 | chr5:74535481-74597124 | 7560 | Foxd2os | NR_030721.1 | chr4:114909288-114921118 |
| 7464 | Firre | NR_015505.2 | chrX:50563119-50635321 | 7561 | Foxd3 | NM_010425.3 | chr4:99656298-99658671 |
| 7465 | Fis1 | NM_001163243.1 | chr5:136953274-136966234 | 7562 | Foxd4 | NM_008022.2 | chr19:24898964-24901309 |
| 7466 | Fitm1 | NM_026808.1 | chr14:55575673-55576952 | 7563 | Foxe1 | NM_183298.1 | chr4:46344193-46345309 |
| 7467 | Fitm2 | NM_173397.4 | chr2:163468702-163472629 | 7564 | Foxe3 | NM_015758.2 | chr4:114925146-114926013 |
| 7468 | Fiz1 | NM_001110328.1 | chr7:5007055-5014697 | 7565 | Foxf1 | NM_010426.2 | chr8:121084385-121088154 |

Fig.21 - 40

| | | | | | | | |
|---|---|---|---|---|---|---|---|
| 7566 | Foxf2 | NM_010225.2 | chr13:31625815-31631406 | 7663 | Fuca1 | NM_024243.4 | chr4:135920725-135940300 |
| 7567 | Foxg1 | NM_001160112.1 | chr12:49382882-49386867 | 7664 | Fuca2 | NM_025799.4 | chr10:13501027-13517275 |
| 7568 | Foxh1 | NM_007989.4 | chr15:76668223-76669948 | 7665 | Fuk | NM_172283.3 | chr8:110882455-110902488 |
| 7569 | Foxi1 | NM_023907.4 | chr11:34204340-34208089 | 7666 | Fundc1 | NM_028058.4 | chrX:17555599-17572325 |
| 7570 | Foxi2 | NM_183193.2 | chr7:135410366-135413615 | 7667 | Fundc2 | NM_026126.4 | chrX:75382398-75397158 |
| 7571 | Foxi3 | NM_001101464.1 | chr6:70956605-70961066 | 7668 | Fuom | NM_001286217.1 | chr7:140097814-140102441 |
| 7572 | Foxj1 | NM_008240.3 | chr11:116330703-116335354 | 7669 | Furin | NM_001081454.2 | chr7:80389193-80405436 |
| 7573 | Foxj2 | NM_021899.3 | chr6:122820183-122845366 | 7670 | Fus | NM_139149.2 | chr7:127967478-127982031 |
| 7574 | Foxj3 | NM_001290696.1 | chr4:119539660-119629119 | 7671 | Fut1 | NM_001271981.1 | chr7:45617605-45621059 |
| 7575 | Foxk1 | NM_199068.2 | chr5:142401496-142462015 | 7672 | Fut10 | NM_001012517.5 | chr8:31187330-31261924 |
| 7576 | Foxk2 | NM_001080932.2 | chr11:121259986-121309896 | 7673 | Fut11 | NM_028428.2 | chr14:20694967-20700197 |
| 7577 | Foxl1 | NM_008024.2 | chr8:121127684-121130644 | 7674 | Fut2 | NM_001271993.1 | chr7:45648590-45666394 |
| 7578 | Foxl2 | NM_012020.2 | chr9:98955606-98958126 | 7675 | Fut4 | NM_010242.3 | chr9:14748458-14752122 |
| 7579 | Foxl2os | NR_003248.3 | chr9:98949154-98955310 | 7676 | Fut4-ps1 | NR_033644.1 | chr17:56734227-56739292 |
| 7580 | Foxm1 | NM_008021.4 | chr6:128362993-128375886 | 7677 | Fut7 | NM_001177366.1 | chr2:25423693-25426373 |
| 7581 | Foxn1 | NM_001277290.1 | chr11:78357576-78386608 | 7678 | Fut8 | NM_001252614.1 | chr12:77238103-77475996 |
| 7582 | Foxn2 | NM_180974.4 | chr17:88440711-88490533 | 7679 | Fut9 | NM_010243.3 | chr4:25609332-25800003 |
| 7583 | Foxn3 | NM_183186.2 | chr12:99195093-99450074 | 7680 | Fuz | NM_027376.3 | chr7:44896078-44900624 |
| 7584 | Foxn4 | NM_148935.2 | chr5:114254163-114273761 | 7681 | Fv1 | NM_010244.3 | chr4:147868978-147870358 |
| 7585 | Foxo1 | NM_019739.3 | chr3:52268336-52350109 | 7682 | Fxn | NM_008044.2 | chr19:24261452-24280586 |
| 7586 | Foxo3 | NM_019740.2 | chr10:42185785-42276742 | 7683 | Fxr1 | NM_001113188.1 | chr3:34020078-34069344 |
| 7587 | Foxo4 | NM_018789.2 | chrX:101254527-101260873 | 7684 | Fxr2 | NM_011814.2 | chr11:69632970-69653297 |
| 7588 | Foxo6 | NM_194060.1 | chr4:120267077-120287261 | 7685 | Fxyd1 | NM_019503.4 | chr7:31051677-31055656 |
| 7589 | Foxp1 | NM_001197321.1 | chr6:98925341-99266515 | 7686 | Fxyd2 | NM_007503.3 | chr9:45406076-45410278 |
| 7590 | Foxp2 | NM_001286607.1 | chr6:15185505-15441977 | 7687 | Fxyd3 | NM_008557.2 | chr7:31070534-31076697 |
| 7591 | Foxp3 | NM_001199347.1 | chrX:7579675-7595243 | 7688 | Fxyd4 | NM_001173372.1 | chr6:117933558-117937335 |
| 7592 | Foxp4 | NM_001110824.1 | chr17:47867132-47924632 | 7689 | Fxyd5 | NM_001111073.2 | chr7:31032722-31041839 |
| 7593 | Foxq1 | NM_008239.4 | chr13:31558169-31560974 | 7690 | Fxyd6 | NM_022004.6 | chr9:45370184-45396159 |
| 7594 | Foxr1 | NM_001033469.2 | chr9:44434233-44440868 | 7691 | Fxyd7 | NM_022007.1 | chr7:31042514-31051454 |
| 7595 | Foxr2 | NM_001034894.3 | chrX:153118787-153132861 | 7692 | Fyb | NM_001278269.1 | chr15:6579846-6665608 |
| 7596 | Foxred1 | NM_001291448.1 | chr9:35204220-35211055 | 7693 | Fyco1 | NM_001110253.2 | chr9:123789499-123851899 |
| 7597 | Foxred2 | NM_001017983.2 | chr15:77940521-77956722 | 7694 | Fyn | NM_001122892.1 | chr10:39369798-39565374 |
| 7598 | Foxs1 | NM_010226.2 | chr2:152931897-152933208 | 7695 | Fyttd1 | NM_001159349.1 | chr16:32877783-32908963 |
| 7599 | Fpgs | NM_010236.2 | chr2:32682608-32694175 | 7696 | Fzd1 | NM_021457.3 | chr5:4753838-4758216 |
| 7600 | Fpgt | NM_029330.2 | chr3:155084918-155093378 | 7697 | Fzd10 | NM_175284.3 | chr5:128601105-128604093 |
| 7601 | Fpr1 | NM_013521.2 | chr17:17876470-17883939 | 7698 | Fzd2 | NM_020510.2 | chr11:102604430-102608058 |
| 7602 | Fpr2 | NM_008039.2 | chr17:17887823-17893952 | 7699 | Fzd3 | NM_021458.2 | chr14:65192440-65262463 |
| 7603 | Fpr3 | NM_008042.2 | chr17:17970457-17971677 | 7700 | Fzd4 | NM_008055.4 | chr7:89404365-89410110 |
| 7604 | Fpr-rs3 | NM_008040.2 | chr17:20623845-20624877 | 7701 | Fzd5 | NM_001042659.1 | chr1:64730557-64737750 |
| 7605 | Fpr-rs4 | NM_008041.2 | chr17:18021732-18022704 | 7702 | Fzd6 | NM_001162494.1 | chr15:39006279-39038190 |
| 7606 | Fpr-rs6 | NM_177316.2 | chr17:20182077-20183097 | 7703 | Fzd7 | NM_008057.3 | chr1:59482146-59486955 |
| 7607 | Fra10ac1 | NM_001081075.1 | chr19:38188478-38224132 | 7704 | Fzd8 | NM_008058.2 | chr18:9212855-9216201 |
| 7608 | Fras1 | NM_175473.3 | chr5:96373954-96784728 | 7705 | Fzd9 | NM_010246.1 | chr5:135248937-135251047 |
| 7609 | Frat1 | NM_008043.3 | chr19:41829969-41832583 | 7706 | Fzr1 | NM_019757.1 | chr10:81366878-81378370 |
| 7610 | Frat2 | NM_177603.3 | chr19:41845975-41848132 | 7707 | G0s2 | NM_008059.3 | chr1:193272159-193273188 |
| 7611 | Frem1 | NM_001198811.1 | chr4:82897919-83052167 | 7708 | G2e3 | NM_001015099.2 | chr12:51348229-51376986 |
| 7612 | Frem2 | NM_172862.3 | chr3:53513937-53657355 | 7709 | G3bp1 | NM_013716.2 | chr11:55469751-55500887 |
| 7613 | Frem3 | NM_001167898.1 | chr8:80611038-80695557 | 7710 | G3bp2 | NM_001080794.2 | chr5:92052144-92083598 |
| 7614 | Frg1 | NM_013522.3 | chr8:41397453-41417118 | 7711 | G530011O06Rik | NR_029457.1 | chrX:169975042-169978917 |
| 7615 | Frk | NM_001159544.1 | chr10:34483399-34611230 | 7712 | G630025P09Rik | NR_027913.1 | chr11:69803668-69806038 |
| 7616 | Frmd3 | NM_001163732.1 | chr4:74013602-74202214 | 7713 | G630055G22Rik | NR_045404.1 | chr18:47922840-48107980 |
| 7617 | Frmd4a | NM_001177843.1 | chr2:4400975-4614043 | 7714 | G630071F17Rik | NR_045401.1 | chr18:60663409-60671624 |
| 7618 | Frmd4b | NM_145148.2 | chr6:97286866-97617657 | 7715 | G630090E17Rik | NM_001173500.2 | chr10:39946911-39960153 |
| 7619 | Frmd5 | NM_172673.3 | chr2:121545528-121807057 | 7716 | G630093K05Rik | NR_045156.1 | chr13:47910434-47934301 |
| 7620 | Frmd6 | NM_028127.3 | chr12:70825513-70902234 | 7717 | G6b | NM_001033221.3 | chr17:35062692-35066184 |
| 7621 | Frmd7 | NM_001190332.1 | chrX:50892644-50942710 | 7718 | G6bos | NR_001462.1 | chr17:35064436-35065596 |
| 7622 | Frmd8 | NM_026169.4 | chr19:5850973-5875208 | 7719 | G6pc | NM_008061.4 | chr11:101367715-101377903 |
| 7623 | Frmpd1 | NM_001081172.2 | chr4:45184905-45285936 | 7720 | G6pc2 | NM_001289856.1 | chr2:69211072-69227993 |
| 7624 | Frmpd1os | NR_040666.1 | chr4:45234722-45243990 | 7721 | G6pc3 | NM_175935.3 | chr11:102189698-102194081 |
| 7625 | Frmpd3 | NM_177750.6 | chrX:140367493-140394520 | 7722 | G6pd2 | NM_019468.2 | chr5:61808842-61810477 |
| 7626 | Frmpd4 | NM_001033330.3 | chrX:167471305-168577233 | 7723 | G6pdx | NM_008062.2 | chrX:74409485-74429161 |
| 7627 | Frrs1 | NM_001113478.1 | chr3:116859566-116903750 | 7724 | G730013B05Rik | NR_040379.1 | chr16:50528501-50559459 |
| 7628 | Frrs1l | NM_001142965.1 | chr4:56960135-56990391 | 7725 | Gaa | NM_001159324.1 | chr11:119267966-119285698 |
| 7629 | Frs2 | NM_177798.3 | chr10:117070126-117148474 | 7726 | Gab1 | NM_021356.2 | chr8:80764433-80880479 |
| 7630 | Frs3 | NM_144939.2 | chr17:47695206-47704286 | 7727 | Gab2 | NM_001162477.1 | chr7:97081750-97308951 |
| 7631 | Frs3os | NR_045912.1 | chr17:47699968-47701996 | 7728 | Gab3 | NM_181584.4 | chrX:74988544-75084905 |
| 7632 | Fry | NM_172887.2 | chr5:150259929-150497753 | 7729 | Gabarap | NM_019749.4 | chr11:69991369-69994949 |
| 7633 | Fryl | NM_028194.2 | chr5:73020190-73256618 | 7730 | Gabarapl1 | NM_020590.4 | chr6:129533191-129542331 |
| 7634 | Frzb | NM_011356.4 | chr2:80411969-80447396 | 7731 | Gabarapl2 | NM_026693.5 | chr8:111940702-111952915 |
| 7635 | Fsbp | NM_001256142.1 | chr4:11579661-11587802 | 7732 | Gabbr1 | NM_019439.3 | chr17:37045965-37074305 |
| 7636 | Fscb | NM_001163271.1 | chr12:64471332-64474898 | 7733 | Gabbr2 | NM_001081141.1 | chr4:46663897-46991714 |
| 7637 | Fscn1 | NM_007984.2 | chr5:142960354-142973189 | 7734 | Gabpa | NM_008065.2 | chr16:84835123-84863778 |
| 7638 | Fscn2 | NM_172802.4 | chr11:120361533-120368173 | 7735 | Gabpb1 | NM_001271467.1 | chr2:126628906-126675592 |
| 7639 | Fscn3 | NM_019569.2 | chr6:28427900-28438622 | 7736 | Gabpb2 | NM_029885.1 | chr3:95181765-95217942 |
| 7640 | Fsd1 | NM_183178.2 | chr17:55986510-55996881 | 7737 | Gabra1 | NM_010250.5 | chr11:42130939-42182930 |
| 7641 | Fsd1l | NM_001195284.1 | chr4:53631470-53707009 | 7738 | Gabra2 | NM_008066.3 | chr5:70961056-71095849 |
| 7642 | Fsd2 | NM_172904.2 | chr7:81534353-81566981 | 7739 | Gabra3 | NM_008067.4 | chrX:72432675-72656246 |
| 7643 | Fshb | NM_008045.3 | chr2:107055985-107059651 | 7740 | Gabra4 | NM_010251.2 | chr5:71569733-71658308 |
| 7644 | Fshr | NM_013523.3 | chr17:88984951-89200675 | 7741 | Gabra5 | NM_176942.4 | chr7:57407668-57510009 |
| 7645 | Fsip1 | NM_027759.3 | chr2:118204887-118256966 | 7742 | Gabra6 | NM_001099641.2 | chr11:42306436-42321072 |
| 7646 | Fst | NM_008046.3 | chr13:114452261-114458951 | 7743 | Gabrb1 | NM_008069.4 | chr5:71700015-72137244 |
| 7647 | Fstl1 | NM_008047.5 | chr16:37777054-37836516 | 7744 | Gabrb2 | NM_008070.3 | chr11:42419756-42632591 |
| 7648 | Fstl3 | NM_031380.2 | chr10:79777273-79782630 | 7745 | Gabrb3 | NM_001038701.2 | chr7:57590517-57828801 |
| 7649 | Fstl4 | NM_177059.3 | chr11:52764705-53187347 | 7746 | Gabrd | NM_008072.2 | chr4:155384978-155398069 |
| 7650 | Fstl5 | NM_001253719.1 | chr3:76075582-76710005 | 7747 | Gabre | NM_017369.2 | chrX:72257431-72274721 |
| 7651 | Ftcd | NM_080845.2 | chr10:76575647-76590338 | 7748 | Gabrg1 | NM_010252.4 | chr5:70751046-70842617 |
| 7652 | Fth1 | NM_010239.2 | chr19:9982645-9985111 | 7749 | Gabrg2 | NM_008073.3 | chr11:41910189-42000714 |
| 7653 | Fthl17 | NM_031261.2 | chrX:9033485-9034333 | 7750 | Gabrg3 | NM_008074.2 | chr7:56724241-57386871 |
| 7654 | Ftl1 | NM_010240.2 | chr7:45457943-45459886 | 7751 | Gabrp | NM_146017.3 | chr11:33550780-33578957 |
| 7655 | Ftmt | NM_026286.3 | chr18:52331535-52332996 | 7752 | Gabrq | NM_001290435.1 | chrX:72825177-72842602 |
| 7656 | Fto | NM_011936.2 | chr8:91313524-91668433 | 7753 | Gabrr1 | NM_008075.2 | chr4:33132555-33163588 |
| 7657 | Ftsj1 | NM_001290430.1 | chrX:8238667-8252406 | 7754 | Gabrr2 | NM_008076.3 | chr4:33063111-33095865 |
| 7658 | Ftsj2 | NM_026510.1 | chr5:140327673-140331898 | 7755 | Gabrr3 | NM_001081190.1 | chr16:59407381-59461739 |
| 7659 | Ftsj3 | NM_025310.2 | chr11:106249143-106255802 | 7756 | Gad1 | NM_008077.5 | chr2:70562128-70602014 |
| 7660 | Ftx | NR_028380.1 | chrX:103569504-103623754 | 7757 | Gad1os | NR_040496.1 | chr2:70489939-70563357 |
| 7661 | Fubp1 | NM_057172.3 | chr3:152210457-152236830 | 7758 | Gad2 | NM_008078.2 | chr2:22622326-22693877 |
| 7662 | Fubp3 | NM_001033389.4 | chr2:31572650-31617526 | 7759 | Gadd45a | NM_007836.1 | chr6:67035095-67037407 |

Fig.21 - 41

| | | | | | | | |
|---|---|---|---|---|---|---|---|
| 7760 | Gadd45b | NM_008655.1 | chr10:80930090-80932204 | 7857 | Gc | NM_008096.2 | chr5:89417510-89457898 |
| 7761 | Gadd45g | NM_011817.2 | chr13:51846674-51848474 | 7858 | Gca | NM_145523.3 | chr2:62664326-62694109 |
| 7762 | Gadd45gip1 | NM_183358.4 | chr8:84832281-84835482 | 7859 | Gcat | NM_001161712.1 | chr15:79030873-79042531 |
| 7763 | Gadl1 | NM_028638.1 | chr9:115909454-116076176 | 7860 | Gcc1 | NM_028900.4 | chr6:28416602-28421724 |
| 7764 | Gak | NM_001282051.1 | chr5:108569106-108629777 | 7861 | Gcc2 | NM_027375.2 | chr10:58255525-58305592 |
| 7765 | Gal | NM_010253.3 | chr19:3409916-3414457 | 7862 | Gcdh | NM_001044744.1 | chr8:84886386-84893921 |
| 7766 | Gal3st1 | NM_001177691.1 | chr11:3989932-3999328 | 7863 | Gcfc2 | NM_177884.2 | chr6:81923668-81959098 |
| 7767 | Gal3st2 | NM_199366.4 | chr1:93861343-93876494 | 7864 | Gcg | NM_008100.4 | chr2:62474529-62483653 |
| 7768 | Gal3st3 | NM_001024717.2 | chr19:5298330-5308739 | 7865 | Gcgr | NM_008101.2 | chr11:120530726-120538984 |
| 7769 | Gal3st4 | NM_001033416.2 | chr5:138264924-138272754 | 7866 | Gch1 | NM_008102.3 | chr14:47153894-47189402 |
| 7770 | Galc | NM_008079.4 | chr12:98202299-98259459 | 7867 | Gchfr | NM_177157.4 | chr2:119167787-119172389 |
| 7771 | Gale | NM_178389.3 | chr4:135965164-135968178 | 7868 | Gck | NM_001287386.1 | chr11:5900820-5915135 |
| 7772 | Galk1 | NM_016905.2 | chr11:116008356-116012719 | 7869 | Gckr | NM_144909.1 | chr5:31297580-31327302 |
| 7773 | Galk2 | NM_001291002.1 | chr2:125866222-125984298 | 7870 | Gclc | NM_010295.2 | chr9:77754534-77794489 |
| 7774 | Galm | NM_176963.4 | chr17:80127470-80185032 | 7871 | Gclm | NM_008129.4 | chr3:122245556-122267215 |
| 7775 | Galns | NM_001193645.1 | chr8:122578236-122611487 | 7872 | Gcm1 | NM_008103.3 | chr9:78051957-78065624 |
| 7776 | Galnt1 | NM_001160404.1 | chr18:24205993-24286816 | 7873 | Gcm2 | NM_008104.2 | chr13:41101426-41109988 |
| 7777 | Galnt10 | NM_134189.2 | chr11:57645441-57787500 | 7874 | Gcnt1l1 | NM_172719.2 | chr5:115565262-115622654 |
| 7778 | Galnt11 | NM_144908.3 | chr5:25222892-25265918 | 7875 | Gcnt1 | NM_001136484.3 | chr19:17326140-17339505 |
| 7779 | Galnt12 | NM_172693.3 | chr4:47091952-47123042 | 7876 | Gcnt2 | NM_008105.3 | chr13:40886757-40960892 |
| 7780 | Galnt13 | NM_173030.2 | chr2:54436386-55117744 | 7877 | Gcnt3 | NM_028087.2 | chr9:70031495-70038088 |
| 7781 | Galnt14 | NM_027864.2 | chr17:73493750-73710451 | 7878 | Gcnt4 | NM_001166065.1 | chr13:96924688-96950914 |
| 7782 | Galnt15 | NM_030166.3 | chr14:32029102-32058326 | 7879 | Gcnt7 | NM_001039560.3 | chr2:172450312-172458596 |
| 7783 | Galnt16 | NM_001081421.1 | chr12:80518989-80603896 | 7880 | Gcsam | NM_001159297.1 | chr16:45610439-45622867 |
| 7784 | Galnt18 | NM_173739.3 | chr7:111471660-111779977 | 7881 | Gcsh | NM_026572.3 | chr8:116981827-116993449 |
| 7785 | Galnt2 | NM_139272.2 | chr8:124231393-124345723 | 7882 | Gda | NM_010266.2 | chr19:21391306-21472661 |
| 7786 | Galnt3 | NM_015736.2 | chr2:66082765-66124793 | 7883 | Gdap1 | NM_010267.3 | chr1:17145372-17164270 |
| 7787 | Galnt4 | NM_015737.4 | chr10:99108134-99113247 | 7884 | Gdap10 | NR_045032.1 | chr12:32824115-32826908 |
| 7788 | Galnt5 | NM_172855.3 | chr2:57998154-58039171 | 7885 | Gdap1l1 | NM_144891.2 | chr2:163438466-163455324 |
| 7789 | Galnt6 | NM_001161767.1 | chr15:100689914-100729376 | 7886 | Gdap2 | NM_010269.3 | chr3:100162462-100206989 |
| 7790 | Galnt7 | NM_001167981.1 | chr8:57523824-57653055 | 7887 | Gde1 | NM_019580.4 | chr7:118688557-118705738 |
| 7791 | Galnt9 | NM_001122639.2 | chr5:110603504-110621383 | 7888 | Gdf1 | NM_001163282.2 | chr8:70315774-70331592 |
| 7792 | Galntl5 | NM_026449.3 | chr5:25181479-25220289 | 7889 | Gdf10 | NM_145741.2 | chr14:33923586-33935282 |
| 7793 | Galntl6 | NM_175032.3 | chr8:57774051-58911627 | 7890 | Gdf11 | NM_010272.1 | chr10:128884545-128891718 |
| 7794 | Galp | NM_178028.2 | chr7:6197089-6216137 | 7891 | Gdf15 | NM_011819.2 | chr8:70629393-70631635 |
| 7795 | Galr1 | NM_008082.2 | chr18:82392495-82406777 | 7892 | Gdf2 | NM_019506.4 | chr14:33941038-33947198 |
| 7796 | Galr2 | NM_010254.4 | chr11:116280938-116283938 | 7893 | Gdf3 | NM_008108.4 | chr6:122605402-122610071 |
| 7797 | Galr3 | NM_015738.2 | chr15:79041884-79043558 | 7894 | Gdf5 | NM_008109.2 | chr2:155941024-155945364 |
| 7798 | Galt | NM_016658.3 | chr4:41755227-41759224 | 7895 | Gdf6 | NM_013526.1 | chr4:9844371-9862345 |
| 7799 | Gamt | NM_010255.3 | chr10:80258150-80260968 | 7896 | Gdf7 | NM_013527.1 | chr12:8297917-8301954 |
| 7800 | Gan | NM_001081151.1 | chr8:117158134-117205186 | 7897 | Gdf9 | NM_008110.2 | chr11:53433285-53437902 |
| 7801 | Ganab | NM_008060.2 | chr19:8898056-8916742 | 7898 | Gdi1 | NM_010273.4 | chrX:74305011-74311867 |
| 7802 | Ganc | NM_172672.2 | chr2:120403895-120460852 | 7899 | Gdi2 | NM_008112.4 | chr13:3538074-3566261 |
| 7803 | Gap43 | NM_008083.2 | chr16:42248560-42340651 | 7900 | Gdnf | NM_010275.3 | chr15:7810047-7837580 |
| 7804 | Gapdh | NM_001289726.1 | chr6:125161851-125166467 | 7901 | Gdpd1 | NM_025638.2 | chr11:87033793-87074137 |
| 7805 | Gapdhs | NM_001290631.1 | chr7:30729778-30743681 | 7902 | Gdpd2 | NM_023608.4 | chrX:100729846-100738901 |
| 7806 | Gapt | NM_177713.3 | chr13:110352614-110357172 | 7903 | Gdpd3 | NM_024228.2 | chr7:126766413-126775645 |
| 7807 | Gapvd1 | NM_025709.2 | chr2:34676982-34755232 | 7904 | Gdpd4 | NM_177696.3 | chr7:97919957-98049663 |
| 7808 | Gar1 | NM_026578.3 | chr3:129824911-129831396 | 7905 | Gdpd5 | NM_201352.2 | chr7:99381548-99460984 |
| 7809 | Garem | NM_001033445.2 | chr18:21127341-21300139 | 7906 | Gdpgp1 | NM_178752.3 | chr7:80232892-80241420 |
| 7810 | Gareml | NM_001167879.1 | chr5:30105195-30118380 | 7907 | Gem | NM_010276.4 | chr4:11704446-11714993 |
| 7811 | Garnl3 | NM_178888.4 | chr2:32986365-33087204 | 7908 | Gemin2 | NM_025656.5 | chr12:59013390-59028470 |
| 7812 | Gars | NM_180678.3 | chr6:55038000-55079504 | 7909 | Gemin4 | NM_177367.3 | chr11:76210570-76217572 |
| 7813 | Gart | NM_010256.2 | chr16:91621396-91646972 | 7910 | Gemin5 | NM_001166669.1 | chr11:58120000-58168539 |
| 7814 | Gas1 | NM_008086.2 | chr13:60174404-60177535 | 7911 | Gemin6 | NM_026053.3 | chr17:80224488-80228497 |
| 7815 | Gas2 | NM_008087.2 | chr7:51879144-51994458 | 7912 | Gemin7 | NM_027189.2 | chr7:19564948-19573343 |
| 7816 | Gas2l1 | NM_001190406.1 | chr11:5054130-5065327 | 7913 | Gemin8 | NM_146238.4 | chrX:166170453-166190512 |
| 7817 | Gas2l2 | NM_001013759.2 | chr11:83421634-83429521 | 7914 | Gen1 | NM_177331.4 | chr12:11240925-11265787 |
| 7818 | Gas2l3 | NM_001033331.2 | chr10:89408822-89443967 | 7915 | Get4 | NM_001163316.1 | chr5:139253496-139270050 |
| 7819 | Gas5 | NR_002840.2 | chr1:161035165-161038537 | 7916 | Gfap | NM_001131020.1 | chr11:102890165-102897200 |
| 7820 | Gas6 | NM_019521.2 | chr8:13465373-13494535 | 7917 | Gfer | NM_023040.3 | chr17:24693190-24696156 |
| 7821 | Gas7 | NM_001109657.3 | chr11:67586499-67688992 | 7918 | Gfi1 | NM_001267621.1 | chr5:107716654-107725805 |
| 7822 | Gas8 | NM_018855.2 | chr8:123518834-123536650 | 7919 | Gfi1b | NM_001160406.1 | chr2:28609449-28621982 |
| 7823 | Gast | NM_010257.1 | chr11:100334411-100336996 | 7920 | Gfm1 | NM_138591.2 | chr3:67430114-67475068 |
| 7824 | Gata1 | NM_008089.2 | chrX:7959259-7967910 | 7921 | Gfm2 | NM_001146043.2 | chr13:97137936-97181196 |
| 7825 | Gata2 | NM_008090.5 | chr6:88198663-88207032 | 7922 | Gfod1 | NM_001033399.4 | chr13:43195518-43304172 |
| 7826 | Gata3 | NM_008091.3 | chr2:9857077-9878600 | 7923 | Gfod2 | NM_027469.4 | chr8:105716112-105758607 |
| 7827 | Gata4 | NM_008092.4 | chr14:63198913-63245271 | 7924 | Gfpt1 | NM_013528.3 | chr6:87042845-87092207 |
| 7828 | Gata5 | NM_008093.2 | chr2:180325087-180334679 | 7925 | Gfpt2 | NM_013529.3 | chr11:49794154-49838620 |
| 7829 | Gata5os | NR_045877.1 | chr2:180332856-180340732 | 7926 | Gfra1 | NM_001285457.1 | chr19:58235604-58454594 |
| 7830 | Gata6 | NM_010258.3 | chr18:11052509-11085635 | 7927 | Gfra2 | NM_008115.3 | chr14:70890106-70979840 |
| 7831 | Gatad1 | NM_026033.2 | chr5:3639960-3647936 | 7928 | Gfra3 | NM_010280.4 | chr18:34689897-34720387 |
| 7832 | Gatad2a | NM_001113346.1 | chr8:69907068-69974383 | 7929 | Gfra4 | NM_001136063.2 | chr2:131039631-131043088 |
| 7833 | Gatad2b | NM_139304.1 | chr3:90341653-90358120 | 7930 | Gfral | NM_205844.3 | chr9:76164101-76213657 |
| 7834 | Gatc | NM_029645.3 | chr5:115333241-115341161 | 7931 | Gfy | NM_001195255.1 | chr7:45176348-45179597 |
| 7835 | Gatm | NM_025961.5 | chr2:122594472-122611277 | 7932 | Gga1 | NM_145929.2 | chr15:78877189-78894585 |
| 7836 | Gatsl2 | NM_030719.2 | chr5:134099747-134141758 | 7933 | Gga2 | NM_028758.2 | chr7:121986721-122021198 |
| 7837 | Gatsl3 | NM_028022.1 | chr11:4218250-4222409 | 7934 | Gga3 | NM_001252067.1 | chr11:115584254-115603916 |
| 7838 | Gba | NM_001077411.2 | chr3:89202924-89208966 | 7935 | Ggact | NM_145466.2 | chr14:122890859-122913165 |
| 7839 | Gba2 | NM_172692.3 | chr4:43566927-43578864 | 7936 | Ggct | NM_026637.3 | chr6:54985094-54992867 |
| 7840 | Gbas | NM_008095.4 | chr5:129725074-129758325 | 7937 | Ggcx | NM_019802.5 | chr6:72414307-72431106 |
| 7841 | Gbe1 | NM_028803.4 | chr16:70313948-70569720 | 7938 | Ggh | NM_010281.2 | chr4:20042051-20066111 |
| 7842 | Gbf1 | NM_178930.3 | chr19:46152557-46286510 | 7939 | Ggn | NM_182694.2 | chr7:29170209-29173933 |
| 7843 | Gbgt1 | NM_139197.2 | chr2:28496890-28505415 | 7940 | Ggnbp1 | NM_001251881.1 | chr17:27018034-27036377 |
| 7844 | Gbp10 | NM_001039646.2 | chr5:105215698-105239533 | 7941 | Ggnbp2 | NM_153144.2 | chr11:84832360-84870738 |
| 7845 | Gbp11 | NM_001039647.3 | chr5:105323025-105346472 | 7942 | Ggps1 | NM_010282.2 | chr13:14052444-14063401 |
| 7846 | Gbp2 | NM_010260.1 | chr3:142620662-142638008 | 7943 | Ggt1 | NM_008116.3 | chr10:75573494-75586191 |
| 7847 | Gbp2b | NM_010259.2 | chr3:142594846-142619176 | 7944 | Ggt5 | NM_011820.4 | chr10:75589380-75616968 |
| 7848 | Gbp3 | NM_001289492.1 | chr3:142560051-142573212 | 7945 | Ggt6 | NM_027819.2 | chr11:72435525-72438404 |
| 7849 | Gbp4 | NM_001256005.1 | chr5:105115766-105139586 | 7946 | Ggt7 | NM_144786.2 | chr2:155490379-155514846 |
| 7850 | Gbp5 | NM_153564.2 | chr3:142496933-142522344 | 7947 | Ggta1 | NM_001145821.2 | chr2:35400178-35461449 |
| 7851 | Gbp6 | NM_194336.2 | chr5:105270701-105293699 | 7948 | Gh | NM_008117.3 | chr11:106300260-106301896 |
| 7852 | Gbp7 | NM_001083312.2 | chr3:142530335-142550151 | 7949 | Ghdc | NM_031871.1 | chr11:100766331-100770957 |
| 7853 | Gbp8 | NM_029509.4 | chr5:105014152-105053561 | 7950 | Ghitm | NM_001199122.1 | chr14:37120444-37135139 |
| 7854 | Gbp9 | NM_172777.3 | chr5:105078393-105110292 | 7951 | Ghr | NM_001048178.2 | chr15:3327524-3583352 |
| 7855 | Gbx1 | NM_015739.2 | chr5:24504425-24526848 | 7952 | Ghrh | NM_010285.2 | chr2:157329495-157346655 |
| 7856 | Gbx2 | NM_010262.3 | chr1:89927961-89931176 | 7953 | Ghrhr | NM_001003685.3 | chr6:55376294-55388530 |

Fig.21 - 42

| | | | |
|---|---|---|---|
| 7954 | Ghrl | NM_001286404.1 | chr6:113716118-113719454 |
| 7955 | Ghsr | NM_177330.4 | chr3:27371350-27378010 |
| 7956 | Gid4 | NM_025757.4 | chr11:60417144-60445277 |
| 7957 | Gid8 | NM_001289651.1 | chr2:180710327-180721599 |
| 7958 | Gif | NM_008118.3 | chr19:11747558-11763447 |
| 7959 | Gigyf1 | NM_031408.2 | chr5:137518879-137527935 |
| 7960 | Gigyf2 | NM_001110212.2 | chr1:87326997-87450810 |
| 7961 | Gimap1 | NM_008376.3 | chr6:48739046-48743795 |
| 7962 | Gimap3 | NM_031247.3 | chr6:48764463-48770851 |
| 7963 | Gimap4 | NM_001243199.1 | chr6:48684577-48692062 |
| 7964 | Gimap5 | NM_175035.5 | chr6:48746196-48754200 |
| 7965 | Gimap6 | NM_153175.3 | chr6:48701582-48708244 |
| 7966 | Gimap7 | NM_146167.3 | chr6:48718620-48724636 |
| 7967 | Gimap8 | NM_001077410.1 | chr6:48647233-48660875 |
| 7968 | Gimap9 | NM_174960.2 | chr6:48676134-48678704 |
| 7969 | Gin1 | NM_026250.3 | chr1:97770171-97793708 |
| 7970 | Ginm1 | NM_145418.4 | chr10:7767946-7780917 |
| 7971 | Gins1 | NM_001163476.1 | chr2:150909593-150926070 |
| 7972 | Gins2 | NM_178856.1 | chr8:120581265-120589075 |
| 7973 | Gins3 | NM_030198.3 | chr8:95633558-95645059 |
| 7974 | Gins4 | NM_024240.6 | chr8:23226609-23237668 |
| 7975 | Gip | NM_008119.2 | chr11:96024544-96030828 |
| 7976 | Gipc1 | NM_018771.3 | chr8:83652677-83664789 |
| 7977 | Gipc2 | NM_016867.1 | chr3:152093840-152165900 |
| 7978 | Gipc3 | NM_148951.1 | chr10:81337761-81343266 |
| 7979 | Gipr | NM_001080815.1 | chr7:19157124-19166127 |
| 7980 | Git1 | NM_001004144.1 | chr11:77493411-77507774 |
| 7981 | Git2 | NM_001077359.1 | chr5:114727407-114773492 |
| 7982 | Gja1 | NM_010288.3 | chr10:56377299-56390419 |
| 7983 | Gja10 | NM_010289.2 | chr4:32600864-32602382 |
| 7984 | Gja3 | NM_001271623.1 | chr14:57034459-57057723 |
| 7985 | Gja4 | NM_008120.3 | chr4:127311419-127314039 |
| 7986 | Gja5 | NM_001271628.1 | chr3:97032401-97053634 |
| 7987 | Gja6 | NM_001001496.2 | chrX:160902115-160907052 |
| 7988 | Gja8 | NM_008123.3 | chr3:96913565-96926051 |
| 7989 | Gjb1 | NM_008124.3 | chrX:101377234-101385629 |
| 7990 | Gjb2 | NM_008125.3 | chr14:57098601-57104702 |
| 7991 | Gjb3 | NM_001160012.1 | chr4:127325234-127330836 |
| 7992 | Gjb4 | NM_008127.4 | chr4:127351085-127354081 |
| 7993 | Gjb5 | NM_010291.3 | chr4:127354808-127358164 |
| 7994 | Gjb6 | NM_001010937.2 | chr14:57123300-57133611 |
| 7995 | Gjc1 | NM_001159382.1 | chr11:102799477-102819159 |
| 7996 | Gjc2 | NM_080454.4 | chr11:59175563-59183213 |
| 7997 | Gjc3 | NM_080450.4 | chr5:137953808-137962959 |
| 7998 | Gjd2 | NM_010290.2 | chr2:114009600-114013619 |
| 7999 | Gjd3 | NM_178596.2 | chr11:98982179-98983016 |
| 8000 | Gjd4 | NM_153086.5 | chr18:9278606-9282809 |
| 8001 | Gje1 | NM_029722.1 | chr10:14715625-14718214 |
| 8002 | Gk2 | NM_010294.1 | chr5:97455160-97457008 |
| 8003 | Gk5 | NM_177352.2 | chr9:96119428-96182953 |
| 8004 | Gkap1 | NM_019832.3 | chr13:58233350-58274188 |
| 8005 | Gkn1 | NM_025466.1 | chr6:87345652-87350915 |
| 8006 | Gkn2 | NM_025467.1 | chr6:87373364-87379494 |
| 8007 | Gkn3 | NM_026860.1 | chr6:87383318-87388935 |
| 8008 | Gla | NM_013463.2 | chrX:134588168-134601005 |
| 8009 | Glb1 | NM_009752.2 | chr9:114401077-114474379 |
| 8010 | Glb1l | NM_029010.1 | chr1:75198234-75210778 |
| 8011 | Glb1l2 | NM_153803.1 | chr9:26763043-26806417 |
| 8012 | Glb1l3 | NM_001113323.1 | chr9:26817952-26860823 |
| 8013 | Glcci1 | NM_001286728.1 | chr6:8520057-8597549 |
| 8014 | Glce | NM_033320.4 | chr9:62057248-62070606 |
| 8015 | Gldc | NM_138595.2 | chr19:30098440-30175441 |
| 8016 | Gldn | NM_177350.5 | chr9:54286485-54341777 |
| 8017 | Gldnos | NR_045805.1 | chr9:54268579-54301570 |
| 8018 | Gle1 | NM_028923.3 | chr2:29935408-29959432 |
| 8019 | Glg1 | NM_009149.2 | chr8:111157557-111259202 |
| 8020 | Gli1 | NM_010296.2 | chr10:127329881-127341579 |
| 8021 | Gli2 | NM_001081125.1 | chr1:118834060-119053619 |
| 8022 | Gli3 | NM_008130.2 | chr13:15463722-15730025 |
| 8023 | Glipr1 | NM_028608.3 | chr10:111985447-111997264 |
| 8024 | Glipr1l1 | NM_027018.1 | chr10:112060188-112078510 |
| 8025 | Glipr1l2 | NM_026223.2 | chr10:112083353-112108098 |
| 8026 | Glipr2 | NM_027450.3 | chr4:43957701-43979118 |
| 8027 | Glis1 | NM_147221.2 | chr4:107434718-107635059 |
| 8028 | Glis2 | NM_031184.3 | chr16:4594712-4615957 |
| 8029 | Glis3 | NM_175459.6 | chr19:28258850-28680077 |
| 8030 | Glmn | NM_001161738.1 | chr5:107548966-107597888 |
| 8031 | Glo1 | NM_001113560.1 | chr17:30592861-30612659 |
| 8032 | Glod4 | NM_026029.3 | chr11:76220394-76243699 |
| 8033 | Glod5 | NM_027227.2 | chrX:8004200-8018492 |
| 8034 | Glp1r | NM_021332.2 | chr17:30901866-30936510 |
| 8035 | Glp2r | NM_175681.3 | chr11:67706429-67771153 |
| 8036 | Glra1 | NM_001290821.1 | chr11:55514238-55608198 |
| 8037 | Glra2 | NM_183427.4 | chrX:165129016-165326981 |
| 8038 | Glra3 | NM_080438.2 | chr8:55940824-56125352 |
| 8039 | Glra4 | NM_010297.2 | chrX:136757673-136779721 |
| 8040 | Glrb | NM_001281969.1 | chr3:80843598-80913614 |
| 8041 | Glrp1 | NM_008132.2 | chr1:88499870-88510066 |
| 8042 | Glrx | NM_053108.4 | chr13:75839885-75850151 |
| 8043 | Glrx2 | NM_001038592.1 | chr1:143739567-143749678 |
| 8044 | Glrx3 | NM_023140.4 | chr7:137437647-137468594 |
| 8045 | Glrx5 | NM_028419.3 | chr12:105032617-105040911 |
| 8046 | Gls | NM_001081081.2 | chr1:52163449-52233232 |
| 8047 | Gls2 | NM_001033264.3 | chr10:128194634-128210004 |
| 8048 | Glt1d1 | NM_177005.4 | chr5:127632261-127707520 |
| 8049 | Glt25d1 | NM_146211.3 | chr8:71611023-71624911 |
| 8050 | Glt28d2 | NM_177130.3 | chr3:85869845-85887518 |
| 8051 | Glt6d1 | NM_001039095.1 | chr2:25793966-25815836 |
| 8052 | Glt8d1 | NM_001165930.1 | chr14:31001392-31012441 |
| 8053 | Glt8d2 | NM_029102.3 | chr10:82650432-82690650 |
| 8054 | Gltp | NM_019821.2 | chr5:114669499-114690935 |
| 8055 | Gltpd1 | NM_024472.4 | chr4:155864722-155869440 |
| 8056 | Gltpd2 | NM_146020.1 | chr11:70519208-70520736 |
| 8057 | Gltscr1 | NM_001081418.1 | chr7:15971261-15999495 |
| 8058 | Gltscr1l | NM_001100452.1 | chr17:46798115-46831413 |
| 8059 | Gltscr2 | NM_133831.3 | chr7:15937835-15946108 |
| 8060 | Glud1 | NM_008133.4 | chr14:34310726-34345033 |
| 8061 | Glul | NM_008131.4 | chr1:153899928-153909723 |
| 8062 | Glyat | NM_145935.3 | chr19:12633307-12651737 |
| 8063 | Glyatl3 | NM_001145060.1 | chr17:40904740-40914350 |
| 8064 | Glycam1 | NM_001289587.1 | chr15:103562759-103565081 |
| 8065 | Glyctk | NM_001039586.1 | chr9:106152859-106158138 |
| 8066 | Glyr1 | NM_001079814.1 | chr16:5013901-5049910 |
| 8067 | Gm10007 | NR_040449.1 | chr19:58296203-58300348 |
| 8068 | Gm10012 | NR_028042.1 | chr17:25901855-25902227 |
| 8069 | Gm10024 | NM_001081452.2 | chr10:77711445-77712009 |
| 8070 | Gm10033 | NR_038043.1 | chr8:69371024-69395544 |
| 8071 | Gm10046 | NR_033484.1 | chr7:27765672-27770972 |
| 8072 | Gm10052 | NR_002885.3 | chr15:103240431-103244584 |
| 8073 | Gm10057 | NM_001243016.1 | chrX:154001589-154055041 |
| 8074 | Gm10058 | NM_001109969.2 | chrX:24948455-35427089 |
| 8075 | Gm10069 | NR_028592.1 | chr6:128438756-128475845 |
| 8076 | Gm10081 | NM_001162940.1 | chr7:107105911-107106859 |
| 8077 | Gm10094 | NM_001142441.1 | chr14:57798217-57802330 |
| 8078 | Gm10096 | NM_001102678.2 | chrX:24948454-35427090 |
| 8079 | Gm101 | NM_001115074.1 | chr1:119923518-119995210 |
| 8080 | Gm10100 | NM_001205033.1 | chr10:77726485-77726848 |
| 8081 | Gm10104 | NM_001177481.1 | chr8:21065037-21066001 |
| 8082 | Gm10125 | NR_033552.1 | chr18:5491500-5592437 |
| 8083 | Gm10142 | NM_001205035.1 | chr10:77715806-77716169 |
| 8084 | Gm10147 | NM_001099919.2 | chrX:24948454-35427090 |
| 8085 | Gm10190 | NR_028385.1 | chr17:80371856-80373542 |
| 8086 | Gm10220 | NM_001134299.1 | chr5:26114763-26121425 |
| 8087 | Gm10228 | NM_001270487.1 | chr16:89040922-89041472 |
| 8088 | Gm10229 | NM_001199334.2 | chr16:89015276-89015846 |
| 8089 | Gm10230 | NM_001099347.2 | chrX:24948450-35427093 |
| 8090 | Gm10248 | NR_033550.1 | chr14:23038194-23094571 |
| 8091 | Gm10267 | NM_001281470.1 | chr18:44156402-44159885 |
| 8092 | Gm10272 | NR_026831.1 | chr10:77706586-77706986 |
| 8093 | Gm10280 | NR_033584.1 | chr8:113067261-113093333 |
| 8094 | Gm10318 | NM_001162944.1 | chr10:77852858-77853788 |
| 8095 | Gm10319 | NR_003624.2 | chr6:122136627-122150958 |
| 8096 | Gm10324 | NM_001177832.1 | chr13:66113452-66122836 |
| 8097 | Gm10334 | NM_001103153.1 | chr6:41442213-41446097 |
| 8098 | Gm10336 | NR_045170.1 | chr13:12182716-12186488 |
| 8099 | Gm10354 | NM_001281514.1 | chr5:14974496-14978899 |
| 8100 | Gm10364 | NR_073535.1 | chr14:55038461-55042976 |
| 8101 | Gm10373 | NR_046064.1 | chr15:43430942-43477036 |
| 8102 | Gm10375 | NM_001098269.2 | chr14:43602626-43608013 |
| 8103 | Gm10377 | NM_001244671.1 | chr14:41767171-43015576 |
| 8104 | Gm10389 | NR_033541.1 | chr15:10600292-10611651 |
| 8105 | Gm10390 | NR_045793.1 | chr5:120121095-120139263 |
| 8106 | Gm10400 | NR_033555.1 | chr6:141340552-141344387 |
| 8107 | Gm10406 | NM_001164727.1 | chr14:7006114-7027449 |
| 8108 | Gm10408 | NM_001256501.1 | chr14:3449311-3698681 |
| 8109 | Gm10409 | NR_033121.1 | chr14:3412613-3673272 |
| 8110 | Gm10413 | NM_029288.3 | chr14:3395092-3642952 |
| 8111 | Gm10415 | NR_045480.1 | chr6:126286648-126328686 |
| 8112 | Gm10416 | NR_027964.1 | chr5:109833500-109834142 |
| 8113 | Gm10421 | NR_033538.1 | chr12:117144522-117151269 |
| 8114 | Gm10432 | NR_045741.1 | chr12:100276608-100300908 |
| 8115 | Gm10433 | NR_045282.1 | chr12:100187967-100193538 |
| 8116 | Gm10436 | NM_001105254.1 | chr12:88175588-88181894 |
| 8117 | Gm10439 | NM_001037716.2 | chrX:149594600-149636621 |
| 8118 | Gm10440 | NR_038045.1 | chr5:54349991-54358542 |
| 8119 | Gm10445 | NR_046063.1 | chr6:84412941-84423838 |
| 8120 | Gm1045 | NM_001177577.1 | chr5:91987472-91995317 |
| 8121 | Gm10451 | NR_028520.1 | chr12:76444496-76448698 |
| 8122 | Gm10466 | NR_033491.1 | chr11:24721005-24730722 |
| 8123 | Gm10471 | NM_001177579.1 | chr5:26082171-26089264 |
| 8124 | Gm10474 | NR_033481.1 | chrX:68667513-68678399 |
| 8125 | Gm10486 | NM_001109970.1 | chrX:24948451-35427093 |
| 8126 | Gm10487 | NM_001100609.1 | chrX:30820542-30843469 |
| 8127 | Gm10488 | NM_001099325.2 | chrX:27472052-35427074 |
| 8128 | Gm10494 | NR_033462.1 | chr17:79506048-79510486 |
| 8129 | Gm10509 | NR_045766.1 | chr17:21690211-21692213 |
| 8130 | Gm10510 | NR_033511.1 | chr17:15350812-15354461 |
| 8131 | Gm10512 | NR_033458.1 | chr17:13205041-13206211 |
| 8132 | Gm10516 | NR_033536.1 | chr1:192136897-192151025 |
| 8133 | Gm10532 | NR_045879.1 | chr18:75514644-75522806 |
| 8134 | Gm10536 | NR_033455.1 | chr18:56936259-56940518 |
| 8135 | Gm10538 | NR_045892.1 | chr1:132729352-132732826 |
| 8136 | Gm10548 | NR_040534.1 | chr18:34207774-34221772 |
| 8137 | Gm10549 | NR_045415.1 | chr18:33464162-33474710 |
| 8138 | Gm10556 | NR_045881.1 | chr18:4812485-4850959 |
| 8139 | Gm10560 | NR_040563.1 | chr4:156021644-156023824 |
| 8140 | Gm10578 | NR_045885.1 | chr7:137234682-137242609 |
| 8141 | Gm10584 | NR_028578.1 | chr7:132315663-132318291 |
| 8142 | Gm10591 | NM_001193668.1 | chr4:42612123-42613253 |
| 8143 | Gm10619 | NR_077222.1 | chr7:73808221-73816503 |
| 8144 | Gm10635 | NR_045336.1 | chr9:79444036-79519302 |
| 8145 | Gm10636 | NR_033542.1 | chr3:146367152-146378944 |
| 8146 | Gm10637 | NR_040697.1 | chr8:87169746-87199850 |
| 8147 | Gm10638 | NR_027829.1 | chr8:86745698-86747060 |

Fig.21 - 43

| | | | |
|---|---|---|---|
| 8148 | Gm10639 | NM_001122660.1 | chr9:78289927-78305525 |
| 8149 | Gm10640 | NR_046062.1 | chr7:31162357-31170641 |
| 8150 | Gm10649 | NR_028579.1 | chr8:76749086-76900173 |
| 8151 | Gm10653 | NR_003965.2 | chr9:62841476-62862024 |
| 8152 | Gm10658 | NR_045886.1 | chr9:57056896-57071966 |
| 8153 | Gm10662 | NM_001201364.1 | chr7:21909433-23144478 |
| 8154 | Gm10665 | NM_001167160.1 | chr7:20473959-22464075 |
| 8155 | Gm10666 | NM_001167573.1 | chr7:20138122-22134741 |
| 8156 | Gm10670 | NM_001167161.1 | chr7:20473960-20474925 |
| 8157 | Gm10677 | NR_046048.1 | chr9:47799159-47818208 |
| 8158 | Gm10681 | NM_001270429.1 | chr3:98499070-98564009 |
| 8159 | Gm10684 | NR_033547.1 | chr9:45107175-45135606 |
| 8160 | Gm10696 | NM_001146107.1 | chr3:94174411-94178193 |
| 8161 | Gm10714 | NR_040530.1 | chr2:174125037-174183918 |
| 8162 | Gm10731 | NR_045392.1 | chr3:40853475-40855285 |
| 8163 | Gm10745 | NR_040751.1 | chr3:11823228-11844359 |
| 8164 | Gm10754 | NR_033537.1 | chr10:97681413-97967090 |
| 8165 | Gm10767 | NM_001177750.1 | chr13:66905414-66909166 |
| 8166 | Gm10768 | NR_033472.1 | chr19:43838802-43840845 |
| 8167 | Gm10778 | NM_001142963.1 | chr10:81649068-81826490 |
| 8168 | Gm10782 | NR_046061.1 | chr13:56362899-56368857 |
| 8169 | Gm10785 | NR_040389.1 | chr16:91688897-91715755 |
| 8170 | Gm10787 | NR_045882.1 | chr10:77011994-77022214 |
| 8171 | Gm10789 | NR_033476.1 | chr16:90142236-90146827 |
| 8172 | Gm10790 | NR_033545.1 | chr13:41865914-41913092 |
| 8173 | Gm10791 | NR_045889.1 | chr16:84972210-84979451 |
| 8174 | Gm10804 | NR_040532.1 | chr2:93452818-93469874 |
| 8175 | Gm10814 | NR_045783.1 | chr19:6012619-6018459 |
| 8176 | Gm10823 | NR_033475.1 | chr16:27849929-27926128 |
| 8177 | Gm10825 | NR_028580.1 | chr10:22402812-22407470 |
| 8178 | Gm10845 | NR_033535.1 | chr14:79860520-79869176 |
| 8179 | Gm10857 | NR_033470.1 | chr2:6132868-6140570 |
| 8180 | Gm10863 | NR_029470.1 | chr15:79166065-79216401 |
| 8181 | Gm10865 | NR_045746.1 | chr15:78994954-79011041 |
| 8182 | Gm10872 | NR_045747.1 | chr15:76266786-76269839 |
| 8183 | Gm10921 | NM_001085553.1 | chrX:4289285-31383918 |
| 8184 | Gm10922 | NM_001085554.1 | chrX:4370635-4372595 |
| 8185 | Gm10941 | NR_026944.1 | chr10:77257772-77259223 |
| 8186 | Gm1110 | NM_001281475.1 | chr9:26879566-26923081 |
| 8187 | Gm11110 | NR_033508.1 | chr17:57092022-57105942 |
| 8188 | Gm11127 | NM_001199967.1 | chr17:36055815-36058371 |
| 8189 | Gm11128 | NM_001201389.1 | chr6:85808050-85809877 |
| 8190 | Gm11149 | NR_029465.1 | chr9:49518287-49568025 |
| 8191 | Gm11166 | NR_024558.1 | chr17:13096659-13098472 |
| 8192 | Gm11186 | NR_046047.1 | chr11:53111450-53120056 |
| 8193 | Gm11190 | NR_033549.1 | chr11:77912126-77929288 |
| 8194 | Gm11201 | NR_045873.1 | chr11:79023596-79035005 |
| 8195 | Gm11213 | NR_028583.1 | chr4:63596900-63605261 |
| 8196 | Gm1123 | NM_001080776.1 | chr9:99006964-99035690 |
| 8197 | Gm11237 | NM_001256481.2 | chr4:73605543-73672395 |
| 8198 | Gm11240 | NR_046041.1 | chr4:73791243-73798378 |
| 8199 | Gm11346 | NR_024599.1 | chr13:24598124-24604767 |
| 8200 | Gm11351 | NR_045062.1 | chr13:25921034-25966316 |
| 8201 | Gm1140 | NM_001126317.1 | chrX:67682899-67706350 |
| 8202 | Gm1141 | NR_027801.1 | chrX:71932437-71940871 |
| 8203 | Gm11413 | NR_045450.1 | chr4:83378316-83390668 |
| 8204 | Gm11426 | NR_033582.1 | chr11:82633352-82636309 |
| 8205 | Gm11437 | NM_001037932.2 | chr11:84148360-84167476 |
| 8206 | Gm11468 | NR_033467.1 | chr2:166273907-166294831 |
| 8207 | Gm11487 | NM_001013393.1 | chr4:73401031-73405072 |
| 8208 | Gm11517 | NR_033523.1 | chr11:96795331-96798156 |
| 8209 | Gm11529 | NR_033524.1 | chr11:96446898-96464547 |
| 8210 | Gm11538 | NR_108029.1 | chr11:96203453-96205480 |
| 8211 | Gm11541 | NM_001007584.2 | chr11:94694497-94704499 |
| 8212 | Gm11544 | NM_001205037.1 | chr11:94844830-94849593 |
| 8213 | Gm11545 | NM_001105561.1 | chr11:94755135-94761182 |
| 8214 | Gm11548 | NR_040590.1 | chr3:36499803-36506809 |
| 8215 | Gm11549 | NR_040411.1 | chr3:36515056-36521445 |
| 8216 | Gm11554 | NM_001099313.2 | chr11:99798185-99809892 |
| 8217 | Gm11559 | NM_001177484.2 | chr11:99864475-99865571 |
| 8218 | Gm11562 | NM_001177537.2 | chr11:99619597-99620404 |
| 8219 | Gm11563 | NM_001126320.2 | chr11:99657941-99658959 |
| 8220 | Gm11564 | NM_001100614.1 | chr11:99814975-99815666 |
| 8221 | Gm11565 | NM_001126323.1 | chr11:99914750-99915671 |
| 8222 | Gm11567 | NM_001101613.1 | chr11:99879186-99880229 |
| 8223 | Gm11568 | NM_001205030.1 | chr11:99857916-99859060 |
| 8224 | Gm11569 | NM_001099312.1 | chr11:99803547-99804446 |
| 8225 | Gm11570 | NM_001256057.1 | chr11:99984702-99986593 |
| 8226 | Gm11595 | NM_001126322.1 | chr11:99771713-99772913 |
| 8227 | Gm11596 | NM_001099311.2 | chr11:99792674-99793292 |
| 8228 | Gm11627 | NR_040286.1 | chr11:102576398-102579119 |
| 8229 | Gm11651 | NR_104047.1 | chr11:105965606-105985694 |
| 8230 | Gm11696 | NR_038097.1 | chr11:109354777-109363654 |
| 8231 | Gm11710 | NM_001101656.2 | chr11:115020688-115037690 |
| 8232 | Gm11744 | NM_001163318.1 | chr11:116657107-116668389 |
| 8233 | Gm11747 | NR_045902.1 | chr11:118444199-118454995 |
| 8234 | Gm11757 | NM_001085538.2 | chr4:73872219-73905921 |
| 8235 | Gm11758 | NM_001097978.2 | chr4:73872218-73905953 |
| 8236 | Gm11762 | NR_045099.1 | chr11:119548629-119569046 |
| 8237 | Gm11780 | NM_001277919.1 | chr4:4527773-4529458 |
| 8238 | Gm11837 | NM_001243100.1 | chr4:14930640-14953030 |
| 8239 | Gm11937 | NM_001099346.1 | chr11:99609793-99610189 |
| 8240 | Gm11938 | NM_001127354.1 | chr11:99602645-99603308 |
| 8241 | Gm11944 | NR_045708.1 | chr11:3308999-3320722 |
| 8242 | Gm11961 | NR_027797.1 | chr11:4625375-4630011 |
| 8243 | Gm11974 | NR_045893.1 | chr11:6525590-6528760 |
| 8244 | Gm11978 | NR_028586.1 | chr11:6650147-6660236 |
| 8245 | Gm11981 | NR_046025.1 | chr11:6716000-6720037 |
| 8246 | Gm11985 | NR_045101.1 | chr11:7183156-7188569 |
| 8247 | Gm11992 | NM_001037928.3 | chr11:9048591-9069354 |
| 8248 | Gm12 | NM_001195544.1 | chr11:98598290-98599586 |
| 8249 | Gm12060 | NR_004857.1 | chr11:23558059-23558842 |
| 8250 | Gm12070 | NR_002890.1 | chr11:26785107-26787859 |
| 8251 | Gm12130 | NR_040295.1 | chr11:38491547-38520045 |
| 8252 | Gm12159 | NR_045100.1 | chr11:45102865-45116928 |
| 8253 | Gm12169 | NM_001163357.1 | chr11:46524250-46538156 |
| 8254 | Gm12171 | NM_001163353.1 | chr11:46548412-46556068 |
| 8255 | Gm12185 | NM_001045540.2 | chr11:48904655-48927182 |
| 8256 | Gm12191 | NR_028101.1 | chr15:34440505-34443233 |
| 8257 | Gm12216 | NR_033332.1 | chr11:53785488-53859256 |
| 8258 | Gm12238 | NR_028480.1 | chr11:55482705-55482829 |
| 8259 | Gm12250 | NM_001135115.1 | chr11:58183842-58190198 |
| 8260 | Gm12253 | NM_001045542.1 | chr11:58432558-58441622 |
| 8261 | Gm12295 | NR_040280.1 | chr11:65277653-65287833 |
| 8262 | Gm12298 | NR_033539.1 | chr11:66905631-66947086 |
| 8263 | Gm12338 | NR_110477.1 | chr11:75599645-75600231 |
| 8264 | Gm12359 | NR_033551.1 | chr11:98798308-98810089 |
| 8265 | Gm12409 | NR_046068.1 | chr4:45762376-45764223 |
| 8266 | Gm12429 | NM_001277167.1 | chr4:42848070-42853888 |
| 8267 | Gm12504 | NR_040414.1 | chr4:44121892-44124123 |
| 8268 | Gm12505 | NR_040674.1 | chr4:55410442-55418842 |
| 8269 | Gm12522 | NR_040560.1 | chr3:108379276-108383741 |
| 8270 | Gm12530 | NR_040669.1 | chr4:57172070-57176554 |
| 8271 | Gm12603 | NR_033533.1 | chr4:89050306-89084610 |
| 8272 | Gm12633 | NR_033610.1 | chr4:90359624-90361314 |
| 8273 | Gm12657 | NM_001081019.1 | chr4:94593649-94600319 |
| 8274 | Gm12669 | NR_033611.1 | chr7:137437673-137467980 |
| 8275 | Gm12695 | NM_001081284.1 | chr4:96723886-96785160 |
| 8276 | Gm12709 | NR_040444.1 | chr4:102967265-102989755 |
| 8277 | Gm12718 | NR_040673.1 | chr4:103382499-103492188 |
| 8278 | Gm12789 | NM_001085520.2 | chr4:101986839-101990231 |
| 8279 | Gm12794 | NM_001085516.1 | chr4:101940406-101943183 |
| 8280 | Gm128 | NM_001024841.3 | chr3:95236919-95241109 |
| 8281 | Gm12830 | NR_033617.1 | chr4:114821719-114856166 |
| 8282 | Gm12886 | NM_001144948.1 | chr4:121414734-121423099 |
| 8283 | Gm12887 | NM_001099309.1 | chr4:121614003-121622125 |
| 8284 | Gm12888 | NM_001033791.3 | chr4:121316315-121324917 |
| 8285 | Gm12942 | NM_001099319.2 | chr4:127126042-127129660 |
| 8286 | Gm12992 | NR_102393.1 | chr4:131899477-131920029 |
| 8287 | Gm13003 | NR_040443.1 | chr4:137159081-137165066 |
| 8288 | Gm13011 | NM_001126318.1 | chr4:137401553-137409791 |
| 8289 | Gm13023 | NM_001007077.2 | chr4:143789333-143795585 |
| 8290 | Gm13031 | NR_045911.1 | chr4:140947665-140957902 |
| 8291 | Gm13032 | NR_045944.1 | chr4:140810992-140817511 |
| 8292 | Gm13034 | NR_030771.1 | chr4:146067523-146068718 |
| 8293 | Gm13040 | NM_001113736.1 | chr4:143535954-143542471 |
| 8294 | Gm13043 | NM_001039595.2 | chr4:143511342-143517831 |
| 8295 | Gm13051 | NM_001037926.2 | chr4:146097311-146126623 |
| 8296 | Gm13057 | NM_001113735.1 | chr4:143511311-143558236 |
| 8297 | Gm13078 | NM_001085412.2 | chr4:143719450-143729158 |
| 8298 | Gm13083 | NM_001126324.1 | chr4:143615002-143618595 |
| 8299 | Gm13084 | NM_001005371.3 | chr4:143809990-143816093 |
| 8300 | Gm13088 | NM_001126325.1 | chr4:143653759-143657246 |
| 8301 | Gm13102 | NM_001085419.1 | chr4:144099879-144110101 |
| 8302 | Gm13103 | NM_177571.3 | chr4:143846496-143853637 |
| 8303 | Gm13119 | NM_001034101.2 | chr4:144357963-144364419 |
| 8304 | Gm13124 | NM_001085542.1 | chr4:144554999-144565134 |
| 8305 | Gm13125 | NM_001115077.1 | chr4:144372759-144377933 |
| 8306 | Gm13128 | NM_001085541.1 | chr4:144330248-144333465 |
| 8307 | Gm13139 | NM_001083918.1 | chr4:145781542-146468233 |
| 8308 | Gm13152 | NM_001039209.2 | chr4:147175865-147513420 |
| 8309 | Gm13154 | NM_001014397.4 | chr4:147553276-147585201 |
| 8310 | Gm13157 | NM_001127189.3 | chr4:147753973-147809788 |
| 8311 | Gm13177 | NM_001081248.1 | chr4:144613706-144623398 |
| 8312 | Gm13178 | NM_001085536.1 | chr4:144703190-144721404 |
| 8313 | Gm13212 | NM_001205101.1 | chr4:145617145-145624394 |
| 8314 | Gm1322 | NM_001033477.3 | chr2:67173833-67186212 |
| 8315 | Gm13238 | NR_033612.1 | chr4:145837218-145837992 |
| 8316 | Gm13242 | NM_001103158.2 | chr4:145670696-145704437 |
| 8317 | Gm13247 | NM_001243138.1 | chr4:146501999-146539395 |
| 8318 | Gm13251 | NM_001085522.2 | chr4:146449029-146469440 |
| 8319 | Gm13271 | NM_001085528.1 | chr4:88754867-88755416 |
| 8320 | Gm13272 | NM_001161608.1 | chr4:88779849-88780660 |
| 8321 | Gm13275 | NM_001085533.2 | chr4:88793741-88801130 |
| 8322 | Gm13276 | NM_001085532.3 | chr4:88785053-88786515 |
| 8323 | Gm13277 | NM_001098840.3 | chr4:88787976-88789438 |
| 8324 | Gm13278 | NM_001098841.3 | chr4:88790894-88798203 |
| 8325 | Gm13279 | NM_001243166.1 | chr4:88791389-88798204 |
| 8326 | Gm13283 | NM_001085531.2 | chr4:88760773-88761584 |
| 8327 | Gm13285 | NM_001161609.1 | chr4:88773993-88806734 |
| 8328 | Gm13286 | NM_001243150.1 | chr4:88757843-88758392 |
| 8329 | Gm13288 | NM_001243167.1 | chr4:88805530-88808380 |
| 8330 | Gm13290 | NM_001243155.1 | chr4:88773833-88806993 |
| 8331 | Gm13293 | NR_040369.1 | chr2:11339488-11344106 |
| 8332 | Gm13298 | NM_001085530.1 | chr4:144078-42528237 |
| 8333 | Gm13304 | NM_001193666.1 | chr4:42114787-42115917 |
| 8334 | Gm13305 | NM_001099348.1 | chr4:3809-42665762 |
| 8335 | Gm13306 | NM_001164046.1 | chr4:3059-42656005 |
| 8336 | Gm13308 | NM_001177580.1 | chr4:173251-42440009 |
| 8337 | Gm13315 | NR_028497.1 | chr2:14720641-14722622 |
| 8338 | Gm13363 | NR_002688.1 | chr1:30941591-30949710 |
| 8339 | Gm13375 | NR_033225.1 | chr2:20968873-20970348 |
| 8340 | Gm13446 | NR_045894.1 | chr2:35549533-35558702 |
| 8341 | Gm13483 | NR_040361.1 | chr2:50296809-50365000 |

Fig.21 - 44

| | | | | | | | |
|---|---|---|---|---|---|---|---|
| 8342 | Gm13490 | NR_040639.1 | chr2:51656965-51732009 | 8439 | Gm15023 | NM_001099321.2 | chrX:135169304-135502913 |
| 8343 | Gm13497 | NR_040636.1 | chr2:51241830-51295945 | 8440 | Gm15055 | NR_110440.1 | chr6:52102948-52113684 |
| 8344 | Gm13498 | NR_033595.1 | chr2:50909683-50911846 | 8441 | Gm15056 | NM_001177471.1 | chr8:20900605-20901973 |
| 8345 | Gm13539 | NR_045340.1 | chr2:25784616-25787022 | 8442 | Gm15091 | NM_001122735.1 | chrX:149941654-149984982 |
| 8346 | Gm13544 | NR_040365.1 | chr2:58276778-58286902 | 8443 | Gm15093 | NM_001099920.1 | chrX:148322133-149487784 |
| 8347 | Gm13546 | NR_045895.1 | chr2:58163973-58177063 | 8444 | Gm15097 | NM_001198987.2 | chrX:149784503-149826694 |
| 8348 | Gm13547 | NM_001177392.1 | chr2:29761527-29764089 | 8445 | Gm15104 | NM_001101501.1 | chrX:150312950-150313364 |
| 8349 | Gm13580 | NR_046065.1 | chr2:60411524-60412574 | 8446 | Gm15107 | NM_001081648.1 | chrX:148180723-148224710 |
| 8350 | Gm13582 | NR_045335.1 | chr2:60964375-60975151 | 8447 | Gm15114 | NM_001082966.1 | chrX:148488948-148521446 |
| 8351 | Gm136 | NM_001033255.2 | chr4:34743787-34756259 | 8448 | Gm15127 | NM_001114400.2 | chrX:148663603-149984982 |
| 8352 | Gm13629 | NR_033495.1 | chr2:66440879-66547472 | 8449 | Gm15133 | NR_040749.1 | chr7:104315030-104324838 |
| 8353 | Gm13710 | NR_046046.1 | chr2:84500747-84506873 | 8450 | Gm15140 | NM_001243017.1 | chrX:154109633-154120688 |
| 8354 | Gm13749 | NR_027824.1 | chr1:63964726-63968999 | 8451 | Gm15179 | NR_037976.1 | chr1:75368209-75375015 |
| 8355 | Gm13752 | NR_040370.1 | chr2:80391363-80398216 | 8452 | Gm15217 | NR_037981.1 | chr14:46379523-46383606 |
| 8356 | Gm13769 | NM_001270423.1 | chr2:90322222-90325126 | 8453 | Gm1527 | NM_001033479.4 | chr3:28892616-28926724 |
| 8357 | Gm13807 | NR_040529.1 | chr2:93403546-93417830 | 8454 | Gm15284 | NM_001177485.1 | chr8:21134649-21135598 |
| 8358 | Gm13826 | NM_001271590.1 | chr5:115102921-115110268 | 8455 | Gm15292 | NM_001177487.1 | chr8:21249761-21250461 |
| 8359 | Gm13871 | NM_001177578.2 | chr4:73872218-73905921 | 8456 | Gm15293 | NM_001177486.1 | chr8:21201603-21202446 |
| 8360 | Gm13889 | NM_001145034.1 | chr2:93955809-93957100 | 8457 | Gm15299 | NM_001170955.1 | chr8:21315545-21316387 |
| 8361 | Gm13939 | NR_033473.1 | chr2:109902395-109913319 | 8458 | Gm15308 | NM_001177521.1 | chr8:21529029-21530012 |
| 8362 | Gm13944 | NR_040368.1 | chr2:77462643-77480938 | 8459 | Gm15315 | NM_001177528.1 | chr8:21665796-21666733 |
| 8363 | Gm14005 | NR_028589.1 | chr2:128298662-128429351 | 8460 | Gm15319 | NM_001177408.1 | chr8:20337663-20363202 |
| 8364 | Gm14015 | NR_040637.1 | chr2:106463822-106523103 | 8461 | Gm15328 | NR_045399.1 | chr18:16816406-16822783 |
| 8365 | Gm14023 | NR_040371.1 | chr2:129297369-129307826 | 8462 | Gm15348 | NR_033546.1 | chr8:12706943-12719127 |
| 8366 | Gm14057 | NR_024097.1 | chr2:130801965-130804101 | 8463 | Gm15350 | NR_045775.1 | chr8:12828337-12831919 |
| 8367 | Gm14085 | NM_001085518.1 | chr2:122484940-122528040 | 8464 | Gm15386 | NM_001040027.2 | chr1:18260523-18265138 |
| 8368 | Gm14092 | NM_001037929.2 | chr2:145549454-145554982 | 8465 | Gm15401 | NR_040421.1 | chr6:72629830-72637717 |
| 8369 | Gm14124 | NM_001142410.1 | chr2:150257516-150270300 | 8466 | Gm15408 | NR_040429.1 | chr5:149006961-149012776 |
| 8370 | Gm14137 | NM_001039223.3 | chr2:119174508-119177575 | 8467 | Gm15412 | NR_046043.1 | chr7:96339482-96341964 |
| 8371 | Gm14139 | NM_001145863.1 | chr2:150181754-150193279 | 8468 | Gm15413 | NR_045874.1 | chr7:96791432-96801549 |
| 8372 | Gm14151 | NM_001097977.1 | chr2:151086785-151103809 | 8469 | Gm15417 | NR_040403.1 | chr3:89391863-89398779 |
| 8373 | Gm14164 | NR_033505.1 | chr2:152345668-152375322 | 8470 | Gm15421 | NR_004442.1 | chr5:22528220-22529030 |
| 8374 | Gm14169 | NR_040372.1 | chr2:156609197-156613422 | 8471 | Gm15441 | NR_040409.1 | chr3:96555767-96566801 |
| 8375 | Gm14204 | NR_040358.1 | chr2:158598172-158610723 | 8472 | Gm15446 | NR_040366.1 | chr5:109933562-109941710 |
| 8376 | Gm14207 | NR_030683.1 | chr2:119321198-119326197 | 8473 | Gm15455 | NM_001161816.1 | chr1:33835898-33838914 |
| 8377 | Gm14288 | NM_001033123.3 | chr2:175275123-176432381 | 8474 | Gm15471 | NR_040412.1 | chr3:103803128-103808440 |
| 8378 | Gm14295 | NM_001205057.2 | chr2:176798599-176808704 | 8475 | Gm1553 | NM_001255990.1 | chr10:82486532-82492618 |
| 8379 | Gm14305 | NM_001099327.1 | chr2:176708352-176721813 | 8476 | Gm15545 | NR_045266.1 | chr7:44986899-44994601 |
| 8380 | Gm14306 | NM_001242944.1 | chr2:175470024-175480553 | 8477 | Gm156 | NM_001014997.1 | chr6:129766646-129775849 |
| 8381 | Gm14308 | NM_001099349.2 | chr2:175321833-176636319 | 8478 | Gm15612 | NR_045880.1 | chr6:88842853-88847274 |
| 8382 | Gm14322 | NM_001243903.1 | chr2:177759287-177773275 | 8479 | Gm1564 | NM_001127576.2 | chr11:102665584-102682238 |
| 8383 | Gm14325 | NM_001024849.2 | chr2:177828990-177840318 | 8480 | Gm15645 | NR_033578.1 | chr7:105861780-105863327 |
| 8384 | Gm14326 | NM_001190302.2 | chr2:177944590-177957288 | 8481 | Gm15663 | NR_038032.1 | chr10:105574550-105583870 |
| 8385 | Gm14327 | NR_038101.2 | chr2:177897162-177927850 | 8482 | Gm15679 | NR_110579.1 | chr8:99011886-99032741 |
| 8386 | Gm14345 | NM_001085545.1 | chrX:3700233-3702192 | 8483 | Gm15698 | NR_003564.1 | chr11:88964665-88966917 |
| 8387 | Gm14346 | NM_001085551.1 | chrX:3441731-3443690 | 8484 | Gm15706 | NR_045598.1 | chr6:145250551-145251856 |
| 8388 | Gm14347 | NM_001085543.1 | chrX:4196575-4198535 | 8485 | Gm15708 | NR_040432.1 | chr5:144277060-144280514 |
| 8389 | Gm14351 | NM_001085552.2 | chrX:3750931-3752885 | 8486 | Gm15713 | NR_046026.1 | chr16:43410517-43420196 |
| 8390 | Gm14374 | NM_001085523.1 | chrX:5669066-5671026 | 8487 | Gm15760 | NR_030670.1 | chr16:20545111-20548556 |
| 8391 | Gm14378 | NM_001195258.1 | chr8:4248213-4251423 | 8488 | Gm15772 | NR_003373.1 | chr11:68901588-68904534 |
| 8392 | Gm14379 | NR_026741.1 | chrX:7375924-7378042 | 8489 | Gm15787 | NR_040430.1 | chr5:110167507-110176504 |
| 8393 | Gm14391 | NM_001099308.2 | chr2:175194278-175881883 | 8490 | Gm15800 | NM_181421.4 | chr5:121220218-121368577 |
| 8394 | Gm14393 | NM_001085546.2 | chr2:175061548-175067769 | 8491 | Gm15816 | NM_001282148.1 | chr8:23138784-23149585 |
| 8395 | Gm14403 | NR_036450.1 | chr2:177498225-177512311 | 8492 | Gm15850 | NR_046167.1 | chr1:136127718-136131183 |
| 8396 | Gm14405 | NR_040256.1 | chr2:176289942-176864437 | 8493 | Gm1587 | NM_001033440.2 | chr14:77793944-77798968 |
| 8397 | Gm14420 | NM_001177568.1 | chr2:177464741-177479194 | 8494 | Gm15880 | NR_040343.1 | chr7:80636016-80644384 |
| 8398 | Gm14431 | NM_001177404.1 | chr2:176521004-176533821 | 8495 | Gm15881 | NM_001177534.2 | chr8:58698848-58700796 |
| 8399 | Gm14436 | NM_001242943.1 | chr2:175470059-175483322 | 8496 | Gm15910 | NR_038023.1 | chr10:120857628-120863328 |
| 8400 | Gm14440 | NM_001199308.1 | chr2:175275124-175801037 | 8497 | Gm15915 | NR_038017.1 | chr10:93674217-93683322 |
| 8401 | Gm14446 | NM_001101605.1 | chr19:34592887-34601968 | 8498 | Gm15941 | NR_045283.1 | chr15:37421132-37432664 |
| 8402 | Gm14458 | NM_001099326.2 | chrX:8985939-8986672 | 8499 | Gm15987 | NR_045009.2 | chr6:128952352-128975029 |
| 8403 | Gm14459 | NM_001126491.1 | chrX:8514051-8524954 | 8500 | Gm15997 | NR_045423.1 | chr5:149489055-149538030 |
| 8404 | Gm14461 | NM_177843.3 | chr2:78237546-78302230 | 8501 | Gm16023 | NR_040441.1 | chr4:155608296-155624755 |
| 8405 | Gm14474 | NM_001242947.1 | chrX:11302431-11302921 | 8502 | Gm16039 | NM_001302353.1 | chr6:8259288-8428767 |
| 8406 | Gm14475 | NM_001242954.1 | chrX:11324659-11324976 | 8503 | Gm1604b | NM_001033442.3 | chr17:7025542-8101228 |
| 8407 | Gm14476 | NM_001242950.1 | chrX:11299320-11324976 | 8504 | Gm16062 | NR_045686.1 | chr11:59810079-59818362 |
| 8408 | Gm14477 | NM_001242949.1 | chrX:11305655-11305972 | 8505 | Gm16063 | NR_046178.1 | chr5:119625941-119634462 |
| 8409 | Gm14478 | NM_001242953.1 | chrX:11318285-11321894 | 8506 | Gm16130 | NM_001243265.1 | chr9:58032273-58048997 |
| 8410 | Gm14479 | NM_001242951.1 | chrX:11315158-11315475 | 8507 | Gm16157 | NR_040340.1 | chr7:68266338-68276594 |
| 8411 | Gm14482 | NM_001242952.1 | chrX:11308754-11321931 | 8508 | Gm16287 | NR_033543.1 | chr4:139175406-139180655 |
| 8412 | Gm14483 | NM_001111037.1 | chrX:11299256-11309260 | 8509 | Gm16291 | NR_045788.1 | chr15:39797044-39812377 |
| 8413 | Gm14484 | NM_001025260.2 | chrX:11311933-11312427 | 8510 | Gm16294 | NR_046185.1 | chr15:40511321-40527277 |
| 8414 | Gm14496 | NM_001205282.1 | chr2:181991225-182001087 | 8511 | Gm1631 | NR_037979.1 | chr2:71719416-71730966 |
| 8415 | Gm14499 | NM_001277184.1 | chrX:8975717-9044410 | 8512 | Gm16325 | NR_045949.1 | chr3:146641931-146651715 |
| 8416 | Gm14501 | NM_001085537.2 | chrX:9350598-9351137 | 8513 | Gm16336 | NR_045267.1 | chr7:110904984-110905851 |
| 8417 | Gm14511 | NM_001085525.2 | chrX:8975717-8976559 | 8514 | Gm16367 | NM_001031622.2 | chr5:82194-95194489 |
| 8418 | Gm14525 | NM_001162364.2 | chrX:26672779-26702638 | 8515 | Gm16381 | NM_001166062.2 | chr12:87644119-87846716 |
| 8419 | Gm14548 | NM_001166672.1 | chr7:3884213-3898092 | 8516 | Gm16386 | NR_030709.2 | chr17:22776229-22817747 |
| 8420 | Gm14625 | NM_001220498.1 | chrX:55430234-55446892 | 8517 | Gm16390 | NM_001097980.1 | chrX:154886802-154890267 |
| 8421 | Gm14632 | NM_001100610.2 | chrX:30329715-30352646 | 8518 | Gm16404 | NM_001220497.1 | chrX:55551595-55568457 |
| 8422 | Gm14634 | NR_045852.1 | chrX:12762277-12821492 | 8519 | Gm16405 | NM_001166646.1 | chrX:54531207-54639016 |
| 8423 | Gm14635 | NR_045321.1 | chrX:12339778-12354996 | 8520 | Gm16430 | NM_001166601.1 | chrX:54622211-54639016 |
| 8424 | Gm14685 | NM_001025383.2 | chrX:73123067-73143424 | 8521 | Gm16432 | NM_001034899.3 | chr1:177991434-178048291 |
| 8425 | Gm14692 | NM_001163195.1 | chrX:67682991-67706258 | 8522 | Gm16445 | NM_001243032.1 | chrX:154152570-154156887 |
| 8426 | Gm14718 | NR_038463.1 | chrX:57200636-57204376 | 8523 | Gm16451 | NM_001167149.1 | chr7:20121655-22118245 |
| 8427 | Gm14725 | NM_001081476.1 | chrX:70545557-70546385 | 8524 | Gm1647 | NR_126533.1 | chr3:69131233-69157177 |
| 8428 | Gm14743 | NM_001126321.2 | chrX:78240461-78245921 | 8525 | Gm16497 | NR_045003.1 | chr12:14261533-14285684 |
| 8429 | Gm14744 | NM_001085544.1 | chrX:77864757-77870033 | 8526 | Gm16501 | NM_001113395.1 | chrY:2599098-2720674 |
| 8430 | Gm14781 | NM_001205268.1 | chrX:91632193-91635671 | 8527 | Gm16515 | NM_025294.5 | chr11:60902245-60913792 |
| 8431 | Gm14812 | NR_033544.2 | chrX:99405330-99443797 | 8528 | Gm16523 | NR_033526.1 | chr2:39157485-39162235 |
| 8432 | Gm14819 | NM_001110250.2 | chrX:24948450-35427093 | 8529 | Gm1653 | NR_040591.1 | chr3:149274736-149279980 |
| 8433 | Gm14827 | NR_045323.1 | chrX:94442731-94447727 | 8530 | Gm16532 | NM_001134752.1 | chr7:6415174-6431086 |
| 8434 | Gm14850 | NM_001177522.1 | chr8:21427427-21566350 | 8531 | Gm16548 | NR_037987.1 | chr1:164148575-164152217 |
| 8435 | Gm14851 | NM_001177482.1 | chr8:21094970-21096050 | 8532 | Gm16551 | NR_045284.1 | chr9:74848436-74852872 |
| 8436 | Gm14858 | NR_040285.1 | chrX:102252650-102257689 | 8533 | Gm16576 | NR_045069.1 | chr15:79742697-79757394 |
| 8437 | Gm14920 | NM_001102665.1 | chrX:117014756-117015104 | 8534 | Gm16596 | NR_045751.1 | chr12:108536401-108555618 |
| 8438 | Gm15008 | NR_045917.1 | chrX:36910833-36911985 | 8535 | Gm166 | NM_001033040.3 | chr7:127582382-127588595 |

Fig.21 - 45

| | | | |
|---|---|---|---|
| 8536 | Gm1661 | NM_001033774.2 | chr4:117215189-117251917 |
| 8537 | Gm16617 | NR_045728.1 | chr14:51986388-51988829 |
| 8538 | Gm16675 | NR_045750.1 | chr8:46730969-46739515 |
| 8539 | Gm16677 | NR_045091.1 | chr14:69329706-69555387 |
| 8540 | Gm16701 | NR_037988.1 | chr1:166974022-166999730 |
| 8541 | Gm16702 | NR_045795.1 | chr17:8379044-8389468 |
| 8542 | Gm16712 | NR_108021.1 | chr17:55954770-55959401 |
| 8543 | Gm1673 | NM_001033458.4 | chr5:33983432-33985009 |
| 8544 | Gm16740 | NR_108026.1 | chr3:95217456-95226658 |
| 8545 | Gm16793 | NR_040376.1 | chr8:35582502-35589020 |
| 8546 | Gm16796 | NR_040367.1 | chr2:170499356-170511927 |
| 8547 | Gm16833 | NR_045754.1 | chr9:9236287-9260885 |
| 8548 | Gm16845 | NR_040406.1 | chr9:22071001-22086131 |
| 8549 | Gm16853 | NR_045742.1 | chr9:21405241-21411720 |
| 8550 | Gm16861 | NR_037666.1 | chr16:4935163-4939585 |
| 8551 | Gm16863 | NR_046162.1 | chr16:21247498-21296872 |
| 8552 | Gm16880 | NR_037986.1 | chr1:136696210-136725728 |
| 8553 | Gm16894 | NR_037980.1 | chr1:40076211-40085851 |
| 8554 | Gm16897 | NR_033564.1 | chr1:194955758-194976959 |
| 8555 | Gm16907 | NR_045794.1 | chr13:63289153-63297147 |
| 8556 | Gm16938 | NR_045969.1 | chr7:98177193-98184799 |
| 8557 | Gm16973 | NR_046209.1 | chr14:56696274-56701000 |
| 8558 | Gm16982 | NR_040337.1 | chr7:141610065-141613747 |
| 8559 | Gm16998 | NR_038016.1 | chr10:54075921-54081081 |
| 8560 | Gm17019 | NM_182957.1 | chr5:15028949-15033007 |
| 8561 | Gm17066 | NR_040628.1 | chr14:105106727-105114676 |
| 8562 | Gm1715 | NR_045333.1 | chr9:52795862-52798772 |
| 8563 | Gm1720 | NR_046015.1 | chrX:166662024-166689812 |
| 8564 | Gm17252 | NM_001199953.1 | chr9:35685078-35687371 |
| 8565 | Gm17296 | NM_001159907.1 | chr8:126426651-126475065 |
| 8566 | Gm17359 | NM_001142953.1 | chr3:79345375-79464128 |
| 8567 | Gm17365 | NM_001167592.2 | chr9:35645112-35648202 |
| 8568 | Gm17455 | NM_001164374.1 | chr10:60399725-60403558 |
| 8569 | Gm17644 | NR_045297.1 | chr1:12667562-12673090 |
| 8570 | Gm17660 | NM_001163772.1 | chr5:104070059-104077608 |
| 8571 | Gm17677 | NM_001167588.1 | chr9:35741048-35742252 |
| 8572 | Gm17689 | NM_001167584.1 | chr9:36581277-36582635 |
| 8573 | Gm17727 | NM_001167673.1 | chr9:35776523-35778109 |
| 8574 | Gm17745 | NR_038014.1 | chr10:93336231-93348879 |
| 8575 | Gm17746 | NR_045844.1 | chr12:25129173-25132274 |
| 8576 | Gm17751 | NR_038012.1 | chr1:76489132-76502028 |
| 8577 | Gm17757 | NR_040453.1 | chr7:105924343-106190099 |
| 8578 | Gm17762 | NR_028378.1 | chr2:18027248-18030577 |
| 8579 | Gm17769 | NR_027377.1 | chr10:77418375-77420939 |
| 8580 | Gm17801 | NR_027452.1 | chr17:25274123-25275528 |
| 8581 | Gm17821 | NR_033146.1 | chr12:67656741-67669271 |
| 8582 | Gm17830 | NR_033486.1 | chr17:49304478-49312960 |
| 8583 | Gm1821 | NR_002875.2 | chr11:46084026-62553206 |
| 8584 | Gm18409 | NR_038018.1 | chr10:99638730-99641920 |
| 8585 | Gm18853 | NR_040456.1 | chr7:105924334-106190099 |
| 8586 | Gm19276 | NR_040357.1 | chr15:10829050-10832713 |
| 8587 | Gm19277 | NR_040360.1 | chr15:85149253-85156132 |
| 8588 | Gm19299 | NR_045748.1 | chr9:67133560-67163788 |
| 8589 | Gm19303 | NR_045092.1 | chr15:51231639-51395610 |
| 8590 | Gm19345 | NM_001270489.1 | chr7:19852002-19858355 |
| 8591 | Gm19395 | NR_038015.1 | chr10:53280269-53283687 |
| 8592 | Gm19402 | NM_001205032.1 | chr10:77689956-77690757 |
| 8593 | Gm19424 | NR_040320.1 | chr19:41746960-41750097 |
| 8594 | Gm1943 | NR_002928.2 | chr8:109339799-109340908 |
| 8595 | Gm19434 | NR_040296.1 | chr6:135188479-135194959 |
| 8596 | Gm19461 | NR_037984.1 | chr1:133249851-133269808 |
| 8597 | Gm19466 | NR_040349.1 | chr18:53648150-53705418 |
| 8598 | Gm19510 | NR_045076.1 | chr15:59788416-59794959 |
| 8599 | Gm19522 | NR_040402.1 | chr16:42884369-42912070 |
| 8600 | Gm19557 | NR_045322.1 | chr19:47512981-47516578 |
| 8601 | Gm19583 | NR_045792.1 | chr5:75110320-75147107 |
| 8602 | Gm19589 | NR_037971.1 | chr1:88551516-88562166 |
| 8603 | Gm19619 | NR_040428.1 | chr5:91257570-91283076 |
| 8604 | Gm1965 | NR_121593.1 | chr6:8914099-89147370 |
| 8605 | Gm1966 | NM_001277179.1 | chr7:106595548-106644645 |
| 8606 | Gm19668 | NM_001205034.1 | chr10:77798386-77799133 |
| 8607 | Gm1968 | NR_037689.1 | chr16:29957926-29979124 |
| 8608 | Gm19689 | NR_045094.1 | chr17:83033591-83078225 |
| 8609 | Gm19705 | NR_045324.1 | chr1:136683395-136690805 |
| 8610 | Gm19710 | NR_045749.1 | chr3:89998759-90001325 |
| 8611 | Gm19757 | NR_040297.1 | chr6:105481265-105492668 |
| 8612 | Gm1976 | NR_045963.1 | chr17:94759941-94834799 |
| 8613 | Gm19782 | NR_045071.1 | chr15:69924232-70078204 |
| 8614 | Gm19784 | NR_040461.1 | chr18:66924761-66949961 |
| 8615 | Gm1979 | NM_001281519.1 | chr5:26000171-26004727 |
| 8616 | Gm1987 | NM_001193667.1 | chr4:120654-42773993 |
| 8617 | Gm19897 | NR_040339.1 | chr7:28774898-28781514 |
| 8618 | Gm1993 | NM_001102677.2 | chrX:25548500-25570449 |
| 8619 | Gm1995 | NR_033562.1 | chr12:87861349-87865690 |
| 8620 | Gm19990 | NR_045048.1 | chr12:56253242-56266381 |
| 8621 | Gm2002 | NM_001100596.1 | chr4:3084-13572 |
| 8622 | Gm20063 | NR_045049.1 | chr12:61499659-61521820 |
| 8623 | Gm20098 | NR_045095.1 | chr17:51882726-51965257 |
| 8624 | Gm2011 | NR_038067.1 | chr3:40846998-40875609 |
| 8625 | Gm20110 | NR_038019.1 | chr10:99609170-99658134 |
| 8626 | Gm2012 | NM_001104946.2 | chrX:26200882-26231323 |
| 8627 | Gm20125 | NR_038020.1 | chr10:16951093-17095782 |
| 8628 | Gm20139 | NR_038021.1 | chr10:19141748-19207947 |
| 8629 | Gm2016 | NM_001122662.1 | chr12:87874071-87877859 |
| 8630 | Gm20172 | NM_001204913.1 | chr1:25276403-25277954 |
| 8631 | Gm20187 | NR_045052.1 | chr12:27090236-27114995 |
| 8632 | Gm20199 | NR_045640.1 | chr9:59477744-59481741 |
| 8633 | Gm2022 | NM_001177574.1 | chr12:87892833-87896722 |
| 8634 | Gm20257 | NR_045007.1 | chr1:58652640-58655831 |
| 8635 | Gm20268 | NR_037989.1 | chr1:101155767-101175202 |
| 8636 | Gm2027 | NR_045113.1 | chr12:44221355-44224380 |
| 8637 | Gm2030 | NM_001100445.2 | chrX:26287198-26310044 |
| 8638 | Gm20300 | NR_045008.1 | chr10:30603194-30606634 |
| 8639 | Gm20319 | NR_105063.1 | chr16:26714681-26755502 |
| 8640 | Gm20324 | NR_045068.1 | chr15:82899079-82905816 |
| 8641 | Gm20337 | NR_045057.1 | chr12:80942651-80945399 |
| 8642 | Gm20356 | NR_040450.1 | chr3:74009218-74017277 |
| 8643 | Gm20362 | NR_040301.1 | chr6:79032041-79035869 |
| 8644 | Gm2042 | NM_001270792.1 | chr12:87602664-87960682 |
| 8645 | Gm20554 | NR_030701.1 | chr13:72623465-72628564 |
| 8646 | Gm20556 | NR_040347.1 | chr15:84714628-84720266 |
| 8647 | Gm20594 | NM_001190732.1 | chr6:79818030-79818364 |
| 8648 | Gm20597 | NR_037973.1 | chr10:53498442-53518989 |
| 8649 | Gm20604 | NM_001142939.1 | chr12:102739829-102757810 |
| 8650 | Gm20605 | NR_033148.1 | chr5:137629122-137642896 |
| 8651 | Gm2061 | NR_040305.1 | chr1:185447115-185455568 |
| 8652 | Gm20611 | NR_040638.1 | chr10:61031302-61038080 |
| 8653 | Gm20735 | NR_015497.2 | chr8:122445267-122451322 |
| 8654 | Gm20736 | NM_001037748.1 | chrY:22835090-82251414 |
| 8655 | Gm20738 | NM_207162.2 | chrY:37957819-65207518 |
| 8656 | Gm20740 | NR_045279.1 | chr15:63414396-63479608 |
| 8657 | Gm20741 | NR_045150.1 | chr16:88811439-88812220 |
| 8658 | Gm20743 | NR_040303.1 | chr1:164863120-164871438 |
| 8659 | Gm20744 | NR_045744.1 | chr7:82070386-82081758 |
| 8660 | Gm20745 | NR_045745.1 | chr9:68017963-68025714 |
| 8661 | Gm20747 | NM_001025241.3 | chrY:21164553-81801497 |
| 8662 | Gm20748 | NR_045420.2 | chr18:61639652-61647485 |
| 8663 | Gm20750 | NR_040555.1 | chr3:52906755-52927666 |
| 8664 | Gm20751 | NR_046022.1 | chr13:43716790-43719038 |
| 8665 | Gm20752 | NR_040750.1 | chr3:159076363-159308080 |
| 8666 | Gm20753 | NR_040630.1 | chr1:106152624-106171524 |
| 8667 | Gm20754 | NR_040557.1 | chr3:73066350-73594830 |
| 8668 | Gm20755 | NR_040559.1 | chr3:37898812-37992728 |
| 8669 | Gm20756 | NR_040771.1 | chr6:25931425-25977227 |
| 8670 | Gm20757 | NR_046027.1 | chr10:92171300-92375373 |
| 8671 | Gm20758 | NR_046029.1 | chr10:111854520-111874376 |
| 8672 | Gm20759 | NR_046030.1 | chr11:86002657-86024350 |
| 8673 | Gm20765 | NM_001270644.1 | chr10:104185533-104188000 |
| 8674 | Gm20767 | NM_001039648.3 | chr13:120140245-120155335 |
| 8675 | Gm20806 | NM_001160135.2 | chrY:67339048-80757210 |
| 8676 | Gm20809 | NM_001160137.1 | chrY:23943165-72820507 |
| 8677 | Gm20815 | NM_001017394.2 | chrY:8832248-8835169 |
| 8678 | Gm20816 | NM_001160144.1 | chrY:64301782-64786299 |
| 8679 | Gm20822 | NM_001199331.1 | chrY:11197161-15883245 |
| 8680 | Gm20823 | NM_001160143.1 | chrY:37957810-72384781 |
| 8681 | Gm20826 | NM_001101623.1 | chrY:7167019-7636973 |
| 8682 | Gm2083 | NM_001134644.1 | chr4:60658465-61782223 |
| 8683 | Gm20831 | NM_001103152.1 | chrY:13252390-17967598 |
| 8684 | Gm20854 | NM_001160131.1 | chrY:83789753-85529519 |
| 8685 | Gm20857 | NR_038297.1 | chrY:24188658-26267093 |
| 8686 | Gm20858 | NR_038298.1 | chrY:24188658-26267093 |
| 8687 | Gm20865 | NM_001160141.1 | chrY:21242965-73240030 |
| 8688 | Gm20867 | NM_001160142.1 | chrY:64301784-64786299 |
| 8689 | Gm2087 | NR_102437.1 | chr9:83431546-83454574 |
| 8690 | Gm20871 | NR_038299.1 | chrY:30497728-59632758 |
| 8691 | Gm20877 | NM_001199332.1 | chrY:12381988-17095887 |
| 8692 | Gm20878 | NM_001270431.1 | chr4:173286-42439974 |
| 8693 | Gm20917 | NM_001160136.1 | chrY:23031058-79149787 |
| 8694 | Gm20939 | NM_001024731.2 | chr17:94864917-94878321 |
| 8695 | Gm21002 | NM_001270555.1 | chr8:21391810-21392792 |
| 8696 | Gm21057 | NR_045695.1 | chr7:80062709-80075257 |
| 8697 | Gm2109 | NR_046066.1 | chr9:83691474-83693247 |
| 8698 | Gm21119 | NM_001270553.1 | chr8:19729575-20640703 |
| 8699 | Gm2115 | NR_045098.1 | chr7:84528953-84578339 |
| 8700 | Gm21221 | NR_077242.1 | chr5:149801989-149811751 |
| 8701 | Gm21269 | NR_102375.1 | chr7:73825923-73836549 |
| 8702 | Gm21276 | NR_073038.1 | chr7:38762405-38770304 |
| 8703 | Gm21283 | NR_105058.1 | chr8:110495365-110502356 |
| 8704 | Gm21284 | NR_078345.1 | chr6:83568038-83588126 |
| 8705 | Gm21293 | XM_006512979.2 | chr10:104142987-104145427 |
| 8706 | Gm21304 | NM_001270901.1 | chr10:104142986-104196493 |
| 8707 | Gm21312 | NM_001270642.1 | chr10:104142986-104196522 |
| 8708 | Gm21319 | NM_001270722.1 | chr12:87772424-87775755 |
| 8709 | Gm21498 | NM_001270613.1 | chr8:21427427-21566344 |
| 8710 | Gm21541 | NM_001270360.1 | chr4:0-42613253 |
| 8711 | Gm21586 | NM_001270432.1 | chr4:42438945-42439974 |
| 8712 | Gm21637 | NM_001270685.1 | chrX:31960926-31962821 |
| 8713 | Gm21671 | NM_001281516.1 | chr5:25949796-25954344 |
| 8714 | Gm21693 | NM_001270514.1 | chrY:3326746-3345434 |
| 8715 | Gm21708 | NM_001270516.1 | chrY:2830679-3783271 |
| 8716 | Gm2176 | NR_028424.1 | chr18:80541891-80544546 |
| 8717 | Gm21943 | NM_001017393.3 | chrY:55557520-73537718 |
| 8718 | Gm21944 | NR_002880.1 | chr8:20876498-20889113 |
| 8719 | Gm21949 | NM_001113419.2 | chr3:67892219-68626482 |
| 8720 | Gm21950 | NM_001270667.1 | chrX:3076919-3078823 |
| 8721 | Gm21951 | NM_001270669.1 | chrX:32726136-32728057 |
| 8722 | Gm2373 | NR_110430.1 | chr13:97482700-97497664 |
| 8723 | Gm2381 | NR_046214.1 | chr7:42816826-42867234 |
| 8724 | Gm2382 | NM_001128601.1 | chr9:88688604-88719798 |
| 8725 | Gm2447 | NR_038079.1 | chr3:52739723-52776654 |
| 8726 | Gm2516 | NR_046067.1 | chr8:50368111-50416089 |
| 8727 | Gm2518 | NR_015538.1 | chr19:8774484-8776962 |
| 8728 | Gm266 | NM_001033248.3 | chr12:111484608-111485823 |
| 8729 | Gm2663 | NM_001102660.1 | chr6:40995821-40999479 |

Fig.21 - 46

| | | | |
|---|---|---|---|
| 8730 | Gm2694 | NR_033430.1 | chr8:87472811-87525554 |
| 8731 | Gm2696 | NM_001205009.1 | chr10:77814681-77815404 |
| 8732 | Gm2721 | NR_045085.1 | chr12:105394855-105403760 |
| 8733 | Gm2762 | NR_037991.1 | chr13:53393688-53455630 |
| 8734 | Gm2799 | NM_001168334.1 | chrX:32278393-32562004 |
| 8735 | Gm2825 | NM_001168337.1 | chrX:32973896-32975846 |
| 8736 | Gm2837 | NR_040388.1 | chrX:33056285-33057063 |
| 8737 | Gm2848 | NR_046069.1 | chr13:52569879-52573225 |
| 8738 | Gm2863 | NM_001099333.3 | chrX:33313337-33315288 |
| 8739 | Gm2897 | NM_001177715.2 | chr14:3049284-3076838 |
| 8740 | Gm2913 | NM_001243015.1 | chrX:3750925-33577346 |
| 8741 | Gm2927 | NM_001282034.1 | chrX:33657139-33659082 |
| 8742 | Gm2933 | NM_001145038.1 | chrX:33575613-33702349 |
| 8743 | Gm2a | NM_010299.3 | chr11:55097984-55113028 |
| 8744 | Gm3002 | NR_033388.1 | chr14:3814912-3830681 |
| 8745 | Gm3020 | NR_033117.1 | chr14:3412613-3669589 |
| 8746 | Gm3086 | NR_036607.1 | chr12:69963408-69969744 |
| 8747 | Gm3139 | NM_001243937.1 | chr5:40898-94538367 |
| 8748 | Gm3143 | NR_038347.1 | chr3:34699639-34716662 |
| 8749 | Gm3219 | NR_027380.1 | chr14:34345054-34345658 |
| 8750 | Gm3230 | NR_033642.1 | chr2:19655805-19657897 |
| 8751 | Gm3238 | NM_001101630.1 | chr10:77770632-77771325 |
| 8752 | Gm3258 | NM_011509.2 | chr10:31413725-31414435 |
| 8753 | Gm3259 | NM_001270456.1 | chr5:95305922-95343575 |
| 8754 | Gm3264 | NM_001242945.1 | chr14:4430991-4875851 |
| 8755 | Gm3279 | NR_046071.1 | chr6:55684570-55694574 |
| 8756 | Gm3285 | NM_001101631.1 | chr10:77861974-77862638 |
| 8757 | Gm3286 | NM_001122678.2 | chr5:82558-95804235 |
| 8758 | Gm3317 | NM_001242941.2 | chr14:5163641-5522929 |
| 8759 | Gm3336 | NM_001195253.1 | chr8:70718542-70722635 |
| 8760 | Gm3383 | NM_001291093.1 | chr14:5763194-5814642 |
| 8761 | Gm3402 | NM_001243111.1 | chr5:146514122-146558915 |
| 8762 | Gm3404 | NM_001243109.1 | chr5:146525800-146528553 |
| 8763 | Gm3409 | NM_001243113.1 | chr5:146537647-146540427 |
| 8764 | Gm3414 | NR_027993.1 | chr5:45719666-45727578 |
| 8765 | Gm3415 | NM_001243114.1 | chr5:146502376-146558915 |
| 8766 | Gm3417 | NM_001123368.1 | chr17:14964188-15041559 |
| 8767 | Gm3428 | NR_030730.1 | chr9:35848544-35849880 |
| 8768 | Gm3434 | NR_030729.1 | chr9:36134286-36135630 |
| 8769 | Gm3435 | NM_001123372.1 | chr17:15009839-15022449 |
| 8770 | Gm3458 | NR_110518.1 | chr9:71211912-71215386 |
| 8771 | Gm3488 | NM_001256885.1 | chr14:5501623-5522929 |
| 8772 | Gm3500 | NM_001256886.1 | chr14:5365791-5741602 |
| 8773 | Gm3558 | NM_001270842.1 | chr14:7545150-7568566 |
| 8774 | Gm3604 | NM_001162910.1 | chr13:62367715-62383174 |
| 8775 | Gm362 | NM_001195271.1 | chrX:43590988-43593530 |
| 8776 | Gm364 | NM_001128625.2 | chrX:57409148-57488771 |
| 8777 | Gm3646 | NM_001177348.1 | chr1:39804140-39805332 |
| 8778 | Gm3696 | NM_001024712.2 | chr14:7083024-7105457 |
| 8779 | Gm3701 | NM_001243011.1 | chrX:3955043-3957002 |
| 8780 | Gm3706 | NM_001243001.1 | chrX:3441735-34161584 |
| 8781 | Gm3716 | NR_045078.1 | chr5:64593862-64610699 |
| 8782 | Gm3750 | NM_001099643.2 | chrX:4800411-4802359 |
| 8783 | Gm3763 | NM_001243024.1 | chrX:4952134-4954077 |
| 8784 | Gm3776 | NM_001243092.1 | chr9:78257128-78269166 |
| 8785 | Gm382 | NM_001033241.3 | chrX:127039971-127063986 |
| 8786 | Gm3893 | NR_033506.1 | chr4:41889794-42233950 |
| 8787 | Gm3985 | NM_001177589.1 | chr8:32888504-32950026 |
| 8788 | Gm4013 | NR_033452.1 | chr18:42274379-42275197 |
| 8789 | Gm4027 | NM_001177564.1 | chr12:87618908-87622143 |
| 8790 | Gm4070 | NM_001243039.1 | chr7:105895118-106215337 |
| 8791 | Gm41 | NR_036689.1 | chrX:86345474-86354994 |
| 8792 | Gm4133 | NM_001167158.1 | chr7:20333141-22329717 |
| 8793 | Gm4175 | NM_001167165.1 | chr7:21760531-21761475 |
| 8794 | Gm4201 | NM_001167162.1 | chr7:20575320-22565343 |
| 8795 | Gm4214 | NM_001167164.1 | chr7:22983729-22984673 |
| 8796 | Gm4216 | NM_001167166.1 | chr7:21795810-23019923 |
| 8797 | Gm4224 | NR_046074.1 | chr7:12221315-12225807 |
| 8798 | Gm4251 | NR_046075.1 | chr14:71251272-71271059 |
| 8799 | Gm4262 | NR_040518.1 | chr16:11008897-11015184 |
| 8800 | Gm4265 | NR_046077.1 | chr7:129962164-129978669 |
| 8801 | Gm4278 | NR_046078.1 | chr14:74975085-74987104 |
| 8802 | Gm428 | NM_001081644.1 | chr4:73653245-73687559 |
| 8803 | Gm4285 | NR_045294.1 | chr14:75842917-75844964 |
| 8804 | Gm4297 | NM_001100446.2 | chrX:24552249-24573305 |
| 8805 | Gm4301 | NM_001166637.1 | chr10:100335675-100382560 |
| 8806 | Gm4302 | NM_001166634.1 | chr10:100345969-100362247 |
| 8807 | Gm4303 | NM_001166638.1 | chr10:100345972-100346881 |
| 8808 | Gm4307 | NM_001166641.1 | chr10:100340837-100362247 |
| 8809 | Gm4312 | NM_001166636.1 | chr10:100381647-100382560 |
| 8810 | Gm4340 | NM_001177535.1 | chr10:104142986-104188000 |
| 8811 | Gm4349 | NR_033637.1 | chr3:95427348-95431085 |
| 8812 | Gm436 | NM_001085504.1 | chr4:144669936-144686368 |
| 8813 | Gm4371 | NR_028311.1 | chr15:96182799-96203470 |
| 8814 | Gm438 | NM_001126316.1 | chr4:144777203-144786583 |
| 8815 | Gm44 | NM_001101450.1 | chrX:90892141-90893134 |
| 8816 | Gm4432 | NR_102329.1 | chr17:32241919-32242855 |
| 8817 | Gm4461 | NM_001199062.1 | chr17:33215260-33216351 |
| 8818 | Gm4477 | NM_001253910.2 | chr6:85706706-85708561 |
| 8819 | Gm4489 | NR_027637.1 | chr10:122119696-122130558 |
| 8820 | Gm4532 | NR_030674.1 | chr7:127232417-127233130 |
| 8821 | Gm4541 | NR_033693.1 | chr7:20610883-23178761 |
| 8822 | Gm4559 | NM_001199309.1 | chr7:142273763-142274363 |
| 8823 | Gm4566 | NR_028023.1 | chr17:71333200-71341804 |
| 8824 | Gm4567 | NM_001037248.3 | chr7:20724511-22719710 |
| 8825 | Gm4598 | NR_030681.1 | chr7:24661293-24663109 |
| 8826 | Gm4710 | NR_033456.1 | chr17:75861022-75889039 |
| 8827 | Gm4719 | NR_045952.1 | chr17:89379240-89384993 |
| 8828 | Gm4724 | NM_001256480.1 | chr2:175419391-175435777 |
| 8829 | Gm4736 | NM_053251.1 | chr6:132114220-132364135 |
| 8830 | Gm4745 | NM_001038676.1 | chr7:14659707-14665996 |
| 8831 | Gm4759 | NR_003967.2 | chr7:106421549-106441073 |
| 8832 | Gm4763 | NM_177593.1 | chr7:24722493-24724290 |
| 8833 | Gm4776 | NR_037690.1 | chr1:46020414-46030437 |
| 8834 | Gm4787 | NM_001038995.2 | chr12:81376990-81379486 |
| 8835 | Gm4788 | NM_001029977.3 | chr1:139697918-139781239 |
| 8836 | Gm4791 | NM_001243258.1 | chr9:46998740-47003860 |
| 8837 | Gm4792 | NR_033209.1 | chr10:94293662-94298703 |
| 8838 | Gm4794 | NM_001101452.1 | chr10:33766423-33782115 |
| 8839 | Gm4814 | NR_036451.1 | chr13:93073303-93074755 |
| 8840 | Gm4827 | NR_045935.1 | chr16:50019657-50072852 |
| 8841 | Gm4832 | NM_001190356.1 | chr17:88125511-88131116 |
| 8842 | Gm4836 | NM_009529.3 | chrX:27472050-35427076 |
| 8843 | Gm4841 | NM_001034859.3 | chr18:60268300-60273267 |
| 8844 | Gm4846 | NM_001164306.1 | chr1:166483612-166497588 |
| 8845 | Gm4847 | NM_001164312.1 | chr1:166628970-166647693 |
| 8846 | Gm4850 | NR_015347.1 | chr1:31264846-31268061 |
| 8847 | Gm4858 | NM_001034860.2 | chr3:93068822-93075505 |
| 8848 | Gm4861 | NM_177665.3 | chr3:137550044-137552622 |
| 8849 | Gm4871 | NM_001101463.1 | chr5:145029599-145032764 |
| 8850 | Gm4872 | NR_073371.1 | chr6:53221163-53224975 |
| 8851 | Gm4884 | NM_183166.2 | chr7:41032718-41045302 |
| 8852 | Gm4890 | NR_045822.1 | chr8:79295077-79307774 |
| 8853 | Gm4894 | NM_177701.3 | chr9:49250530-49280594 |
| 8854 | Gm4906 | NM_001114529.1 | chrX:11327821-11328151 |
| 8855 | Gm4907 | NM_001034864.3 | chrX:23892762-23907550 |
| 8856 | Gm4922 | NM_177706.4 | chr10:18779726-18786793 |
| 8857 | Gm4925 | NM_001037166.2 | chr10:88729540-88731101 |
| 8858 | Gm4926 | NR_028066.1 | chr11:46636505-46645006 |
| 8859 | Gm4937 | NM_001013760.2 | chrX:76264305-76267673 |
| 8860 | Gm4944 | NM_001205095.1 | chr17:22197264-22225614 |
| 8861 | Gm4951 | NM_001033767.3 | chr18:60212076-60247820 |
| 8862 | Gm4952 | NM_001013762.2 | chr19:12600015-12627616 |
| 8863 | Gm4956 | NR_002858.1 | chr1:21285245-21298312 |
| 8864 | Gm4961 | NR_045694.2 | chr5:30092939-30097686 |
| 8865 | Gm4971 | NR_033604.1 | chr7:73685491-73686563 |
| 8866 | Gm4975 | NM_001195687.1 | chr8:63924693-63952170 |
| 8867 | Gm4980 | NM_001195529.1 | chr7:99624066-99627103 |
| 8868 | Gm4981 | NM_001034869.2 | chr10:58234847-58236660 |
| 8869 | Gm4984 | NM_001101484.1 | chrX:12651882-12652820 |
| 8870 | Gm5 | NR_024513.1 | chr5:150499746-150502219 |
| 8871 | Gm5039 | NR_003647.2 | chr12:88320121-88321762 |
| 8872 | Gm5065 | NR_003622.2 | chr7:5350541-5360682 |
| 8873 | Gm5069 | NR_003623.1 | chr1:180326968-180330549 |
| 8874 | Gm5071 | NM_001256004.1 | chrX:91931945-91932980 |
| 8875 | Gm5072 | NM_001114678.1 | chrX:91392499-91481160 |
| 8876 | Gm5082 | NM_001145878.1 | chr13:41650761-41656777 |
| 8877 | Gm5083 | NR_045285.1 | chr13:44121166-44125179 |
| 8878 | Gm5084 | NR_036449.1 | chr13:60205470-60216018 |
| 8879 | Gm5086 | NR_046157.1 | chr13:97559999-97583994 |
| 8880 | Gm5087 | NR_121588.1 | chr14:13157388-13284638 |
| 8881 | Gm5088 | NR_002862.3 | chr14:89896223-89899447 |
| 8882 | Gm5089 | NR_033325.1 | chr14:122434853-122451115 |
| 8883 | Gm5091 | NR_046164.1 | chr17:15231584-15240783 |
| 8884 | Gm5095 | NR_033454.1 | chr18:47537734-47718636 |
| 8885 | Gm5105 | NR_037975.1 | chr3:138048760-138067388 |
| 8886 | Gm5108 | NM_001256184.1 | chr5:67941668-67977070 |
| 8887 | Gm5111 | NM_183309.3 | chr6:48589444-48590584 |
| 8888 | Gm5113 | NM_001033540.2 | chr7:30169921-30180209 |
| 8889 | Gm5114 | NM_177890.3 | chr7:39407293-39413160 |
| 8890 | Gm5122 | NR_040767.1 | chr9:61017068-61019797 |
| 8891 | Gm5124 | NR_045668.1 | chrX:21360864-21364624 |
| 8892 | Gm5126 | NR_026596.1 | chrX:102932046-102934223 |
| 8893 | Gm5127 | NM_001033541.2 | chrX:106583185-106710557 |
| 8894 | Gm5129 | NR_028426.1 | chr5:29735333-29735936 |
| 8895 | Gm5132 | NM_001085517.2 | chrX:9571959-9572391 |
| 8896 | Gm5134 | NM_198635.3 | chr10:75954513-76009589 |
| 8897 | Gm5136 | NM_203660.2 | chr10:108699043-108700160 |
| 8898 | Gm5141 | NM_001256065.1 | chr13:62772199-62785808 |
| 8899 | Gm5142 | NM_001004158.2 | chr14:59158502-59178749 |
| 8900 | Gm5148 | NM_198657.2 | chr3:37714189-37724360 |
| 8901 | Gm5150 | NM_001081687.1 | chr3:15948069-16006332 |
| 8902 | Gm5166 | NR_027707.1 | chrX:102791195-102798389 |
| 8903 | Gm5168 | NM_001025607.3 | chrX:26028213-26051041 |
| 8904 | Gm5169 | NM_001040669.1 | chrX:25277482-25301455 |
| 8905 | Gm5176 | NR_033603.1 | chr10:111500787-111501345 |
| 8906 | Gm5177 | NR_033630.1 | chr10:10605944-10616313 |
| 8907 | Gm525 | NM_001033266.2 | chr11:89073840-89093064 |
| 8908 | Gm527 | NM_001025605.1 | chr12:64917910-64924591 |
| 8909 | Gm5294 | NM_001195128.1 | chr5:138820079-138821619 |
| 8910 | Gm53 | NR_037977.1 | chr11:96251659-96264484 |
| 8911 | Gm5334 | NR_003648.2 | chr7:68618455-68620009 |
| 8912 | Gm5346 | NM_001025240.1 | chr8:43624974-43627185 |
| 8913 | Gm5347 | NM_001079931.2 | chr8:43673657-43699491 |
| 8914 | Gm5382 | NM_001034100.1 | chrX:14211147-14211657 |
| 8915 | Gm5409 | NM_001003664.2 | chr6:41415306-41419593 |
| 8916 | Gm5414 | NM_001003670.1 | chr15:101624027-101628188 |
| 8917 | Gm5415 | NM_001164286.1 | chr1:32543545-32547293 |
| 8918 | Gm5416 | NM_001082542.1 | chr16:36210402-36217788 |
| 8919 | Gm5420 | NR_045843.1 | chr10:21690844-21693978 |
| 8920 | Gm5424 | NR_002687.1 | chr2:31470307-31520629 |
| 8921 | Gm5431 | NM_001024230.2 | chr11:48887421-48902152 |
| 8922 | Gm5434 | NR_003649.1 | chr12:36090378-36091829 |
| 8923 | Gm5441 | NR_044987.1 | chr12:117234520-117337012 |

Fig.21 - 47

| | | | |
|---|---|---|---|
| 8924 | Gm5458 | NM_001024706.2 | chr14:19594137-19602587 |
| 8925 | Gm5460 | NM_001034880.2 | chr14:34041077-34046981 |
| 8926 | Gm5464 | NM_001034881.3 | chr14:66868849-66871005 |
| 8927 | Gm5468 | NR_027376.1 | chr15:25414191-25452418 |
| 8928 | Gm5475 | NR_040351.1 | chr15:100423192-100428150 |
| 8929 | Gm5476 | NR_002868.1 | chr15:101587316-101588572 |
| 8930 | Gm5477 | NR_002869.1 | chr15:101608873-101610479 |
| 8931 | Gm5478 | NR_003960.1 | chr15:101643019-101647380 |
| 8932 | Gm5483 | NM_001082547.1 | chr16:36184211-36188110 |
| 8933 | Gm5485 | NR_015373.1 | chr16:48399452-48412416 |
| 8934 | Gm5512 | NR_002891.1 | chr10:4403170-12906985 |
| 8935 | Gm5523 | NR_004447.1 | chr1:13785684-13786929 |
| 8936 | Gm5531 | NM_001008426.3 | chr1:153874344-153876871 |
| 8937 | Gm5535 | NM_001033778.1 | chr2:144173149-144189290 |
| 8938 | Gm5538 | NM_001101531.1 | chr3:59729785-59752397 |
| 8939 | Gm5544 | NM_001033779.2 | chr3:97930172-97967018 |
| 8940 | Gm5547 | NR_045845.1 | chr3:105815566-105817359 |
| 8941 | Gm5549 | NM_001270430.1 | chr3:132630190-132644649 |
| 8942 | Gm5577 | NR_026990.1 | chr6:87981682-87984180 |
| 8943 | Gm5591 | NM_001013810.2 | chr7:38518138-38528193 |
| 8944 | Gm5592 | NM_001033782.3 | chr7:41284326-41290183 |
| 8945 | Gm5595 | NM_001008427.1 | chr7:42660107-42692718 |
| 8946 | Gm5607 | NR_027975.2 | chr8:12385770-12436732 |
| 8947 | Gm561 | NM_001033297.2 | chr2:144594064-144595365 |
| 8948 | Gm5615 | NM_001033783.2 | chr9:36532402-36541963 |
| 8949 | Gm5617 | NM_001004191.3 | chr9:48495342-48495975 |
| 8950 | Gm5622 | NM_001013816.1 | chr14:51552789-51662953 |
| 8951 | Gm5627 | NR_033301.1 | chr9:102739647-102756685 |
| 8952 | Gm5634 | NM_001085524.1 | chrX:8962133-8962975 |
| 8953 | Gm5635 | NM_001038697.2 | chrX:9063089-9063822 |
| 8954 | Gm5640 | NM_001099302.1 | chrX:74639118-74645635 |
| 8955 | Gm5643 | NR_002883.1 | chrX:103240453-142299237 |
| 8956 | Gm5662 | NM_001013824.3 | chr12:88270634-88274497 |
| 8957 | Gm5712 | NR_033594.1 | chr3:129433672-129434712 |
| 8958 | Gm572 | NM_001085505.1 | chr4:148643316-148671572 |
| 8959 | Gm5725 | NM_001166711.1 | chr7:21843343-23067456 |
| 8960 | Gm5726 | NM_001167146.1 | chr7:20544834-20545757 |
| 8961 | Gm5728 | NM_001166713.1 | chr7:20374492-22371039 |
| 8962 | Gm5741 | NM_001195531.1 | chr8:85067567-85067982 |
| 8963 | Gm5766 | NR_003628.1 | chr16:4229228-4231205 |
| 8964 | Gm5771 | NM_001038997.2 | chr6:41392355-41397256 |
| 8965 | Gm5779 | NR_033602.1 | chr10:75352054-75352961 |
| 8966 | Gm5795 | NM_001270806.1 | chr14:3188283-3800987 |
| 8967 | Gm5796 | NM_001029930.2 | chr14:4023940-4041368 |
| 8968 | Gm5797 | NM_001025085.2 | chr14:7323987-7332395 |
| 8969 | Gm5800 | NM_001034102.2 | chr14:51711643-51717132 |
| 8970 | Gm5801 | NR_002889.2 | chr4:56010291-155957601 |
| 8971 | Gm5803 | NM_001165971.1 | chr15:22713819-22714984 |
| 8972 | Gm5820 | NM_001033789.2 | chr18:38892455-38971274 |
| 8973 | Gm5833 | NR_040304.1 | chr1:138640094-138682075 |
| 8974 | Gm5860 | NR_040659.1 | chr4:82065379-82102807 |
| 8975 | Gm5862 | NM_001281525.1 | chr5:26018420-26022891 |
| 8976 | Gm5868 | NM_001024147.2 | chr5:72581638-72587550 |
| 8977 | Gm5878 | NM_001034902.2 | chr6:85111415-85126094 |
| 8978 | Gm5885 | NM_001185040.1 | chr6:133529188-133532762 |
| 8979 | Gm5886 | NM_001177652.2 | chr6:133763997-133767408 |
| 8980 | Gm5891 | NM_001034904.2 | chr7:21909433-23144480 |
| 8981 | Gm5893 | NR_045096.1 | chr7:24818794-24840275 |
| 8982 | Gm590 | NM_001195437.1 | chr9:110914738-110916861 |
| 8983 | Gm5901 | NM_001195727.1 | chr7:105375097-105378287 |
| 8984 | Gm5916 | NM_001167587.1 | chr9:36119933-36128779 |
| 8985 | Gm5925 | NR_040410.1 | chrX:4515588-4517543 |
| 8986 | Gm5934 | NM_001100444.2 | chrX:24474307-24499163 |
| 8987 | Gm5935 | NM_001081657.1 | chrX:24753161-24775164 |
| 8988 | Gm5936 | NM_001081670.2 | chrX:74837195-74843369 |
| 8989 | Gm5938 | NM_001085534.2 | chrX:78125466-78130403 |
| 8990 | Gm5941 | NM_001034103.1 | chrX:92489948-92490679 |
| 8991 | Gm595 | NM_001085499.1 | chrX:48841465-48877713 |
| 8992 | Gm597 | NM_001013750.1 | chr1:28776121-28780252 |
| 8993 | Gm6026 | NM_001177569.1 | chrY:2599098-2720674 |
| 8994 | Gm6034 | NM_001034909.3 | chr17:36042960-36058645 |
| 8995 | Gm6040 | NM_001025353.2 | chr8:20916489-20922071 |
| 8996 | Gm6042 | NR_002872.1 | chr15:101503815-101506313 |
| 8997 | Gm608 | NM_001029889.2 | chr16:44173396-44227466 |
| 8998 | Gm6083 | NR_102704.1 | chr5:29537948-29538624 |
| 8999 | Gm6086 | NM_001039219.2 | chr1:93990508-94011539 |
| 9000 | Gm609 | NM_001005854.2 | chr16:45416754-45492969 |
| 9001 | Gm6116 | NR_045866.1 | chr5:74949193-74966368 |
| 9002 | Gm6121 | NM_001114754.1 | chrX:26912743-26935669 |
| 9003 | Gm614 | NM_001033362.2 | chrX:101261376-101263992 |
| 9004 | Gm6150 | NR_038036.1 | chr10:9684721-9691909 |
| 9005 | Gm6164 | NM_001167153.1 | chr7:20204683-22201264 |
| 9006 | Gm6194 | NR_033512.1 | chr3:8461386-8463098 |
| 9007 | Gm6213 | NR_044988.1 | chr8:39297979-39368268 |
| 9008 | Gm6225 | NR_033457.1 | chr18:3336415-3366863 |
| 9009 | Gm6249 | NR_046021.1 | chr7:136667954-136710604 |
| 9010 | Gm6251 | NM_001101561.2 | chr10:20208438-20209030 |
| 9011 | Gm6260 | NR_040405.1 | chr3:143670891-143712143 |
| 9012 | Gm6268 | NR_044989.1 | chrX:36403476-36404654 |
| 9013 | Gm6277 | NR_045421.1 | chr18:11821087-11839377 |
| 9014 | Gm6289 | NR_126537.1 | chr3:144879322-144896011 |
| 9015 | Gm6297 | NR_077221.1 | chr4:40720153-40722317 |
| 9016 | Gm6300 | NR_033591.2 | chr3:14363116-14374239 |
| 9017 | Gm6307 | NR_045331.1 | chr2:180385603-180401802 |
| 9018 | Gm6313 | NR_045867.1 | chr6:148606785-148614309 |
| 9019 | Gm6329 | NR_040690.1 | chr8:45160493-45165145 |
| 9020 | Gm6367 | NR_044992.1 | chr5:587624-95026536 |
| 9021 | Gm6370 | NM_001243110.1 | chr5:146491362-146494132 |
| 9022 | Gm6377 | NM_001037917.2 | chrX:109196755-109200445 |
| 9023 | Gm6402 | NR_030688.1 | chr17:30394824-30396180 |
| 9024 | Gm6406 | NM_001134661.1 | chr9:98856590-98857352 |
| 9025 | Gm6408 | NM_001243104.1 | chr5:146481942-146484702 |
| 9026 | Gm6416 | NR_046023.1 | chr13:117130024-117135884 |
| 9027 | Gm6432 | NM_001244762.1 | chr9:99229375-99237239 |
| 9028 | Gm6455 | NR_003596.2 | chr5:10865028-10870808 |
| 9029 | Gm6460 | NM_001037919.3 | chr5:11594955-11599480 |
| 9030 | Gm648 | NM_001033372.2 | chrX:56543873-56549606 |
| 9031 | Gm6484 | NM_001080940.1 | chr9:21835509-21837347 |
| 9032 | Gm6498 | NR_003630.2 | chr14:48198715-48228413 |
| 9033 | Gm6524 | NR_028307.1 | chr8:11692981-11694514 |
| 9034 | Gm6525 | NR_036654.1 | chr3:84174637-84175193 |
| 9035 | Gm6537 | NM_001195091.1 | chr7:28756173-28758964 |
| 9036 | Gm6548 | NR_003363.1 | chr17:78850504-78852544 |
| 9037 | Gm6559 | NR_110455.1 | chr6:51379709-51392791 |
| 9038 | Gm6567 | NR_046024.1 | chr7:73097751-73102068 |
| 9039 | Gm6568 | NR_040420.2 | chrX:157540803-157541750 |
| 9040 | Gm6578 | NR_003631.2 | chr6:12099520-12109580 |
| 9041 | Gm6583 | NM_001039228.4 | chr5:112353772-112356033 |
| 9042 | Gm6588 | NM_001177504.1 | chr5:112449425-112451738 |
| 9043 | Gm6592 | NM_001081564.1 | chrX:8843993-8849607 |
| 9044 | Gm6602 | NR_045362.1 | chr3:111881902-112082001 |
| 9045 | Gm6607 | NR_033622.2 | chr9:22330138-22330690 |
| 9046 | Gm6614 | NM_001081318.1 | chr6:141972195-142008745 |
| 9047 | Gm6623 | NR_033619.1 | chr17:36178776-36181364 |
| 9048 | Gm6634 | NR_040556.1 | chr3:70772378-70807291 |
| 9049 | Gm6639 | NR_040748.1 | chr3:35597151-35666163 |
| 9050 | Gm6642 | NR_033643.2 | chr19:30930385-30930962 |
| 9051 | Gm6644 | NR_037965.1 | chr6:34303933-34317443 |
| 9052 | Gm6654 | NR_038089.1 | chr6:146647176-146647802 |
| 9053 | Gm6682 | NR_033599.1 | chr12:4782170-4783512 |
| 9054 | Gm6696 | NM_001177523.1 | chr8:21583026-21583867 |
| 9055 | Gm6710 | NM_001164689.1 | chr2:175192979-175883182 |
| 9056 | Gm6756 | NR_076393.1 | chr18:36920304-36922207 |
| 9057 | Gm6760 | NM_001177377.1 | chrX:64151404-64152198 |
| 9058 | Gm6763 | NM_001270899.1 | chr10:104142986-104196493 |
| 9059 | Gm6787 | NR_003632.2 | chrX:8097086-8106701 |
| 9060 | Gm6792 | NM_001177416.1 | chr7:6252709-6281861 |
| 9061 | Gm6793 | NR_033513.1 | chr2:75659289-75665756 |
| 9062 | Gm6812 | NM_001098842.2 | chrX:68892372-68893053 |
| 9063 | Gm6815 | NR_102685.1 | chr16:36194479-36197886 |
| 9064 | Gm684 | NM_001195681.1 | chr9:51270257-51278554 |
| 9065 | Gm6878 | NM_001037931.3 | chr14:67304887-67314711 |
| 9066 | Gm6880 | NM_001099305.1 | chrX:74480286-74481399 |
| 9067 | Gm6890 | NM_001099306.1 | chrX:74740926-74742188 |
| 9068 | Gm6902 | NM_001270494.1 | chr7:20724511-23275243 |
| 9069 | Gm6904 | NM_001164329.1 | chr14:59244440-59260216 |
| 9070 | Gm6927 | NM_001101585.1 | chrX:77674813-77675452 |
| 9071 | Gm6936 | NR_045001.1 | chr16:49980459-49997475 |
| 9072 | Gm6938 | NR_033482.1 | chrX:21312209-21334727 |
| 9073 | Gm694 | NM_001033374.3 | chr4:141432671-141436105 |
| 9074 | Gm6981 | NR_023357.1 | chr9:52002060-52048705 |
| 9075 | Gm6994 | NR_033141.1 | chr14:77479482-77506868 |
| 9076 | Gm7008 | NR_045157.1 | chr12:40223363-40229183 |
| 9077 | Gm7030 | NM_001177467.1 | chr17:36127608-36129425 |
| 9078 | Gm7056 | NR_037571.1 | chr5:89616435-89622611 |
| 9079 | Gm7073 | NM_001039240.3 | chrX:60436051-60456195 |
| 9080 | Gm7102 | NM_001177513.1 | chr19:61174685-61176309 |
| 9081 | Gm7104 | NR_033570.1 | chr12:88282866-88287070 |
| 9082 | Gm711 | NM_198628.2 | chr2:26934068-26953496 |
| 9083 | Gm7120 | NM_001039244.3 | chr13:119488038-119610459 |
| 9084 | Gm7134 | NR_033597.1 | chrX:110372752-110375156 |
| 9085 | Gm715 | NM_001271548.1 | chrX:60548007-60549216 |
| 9086 | Gm7157 | NM_001199311.1 | chrX:152563333-152563969 |
| 9087 | Gm7168 | NM_001122977.1 | chr17:13948372-13950678 |
| 9088 | Gm7173 | NM_001099307.1 | chrX:79482567-79517285 |
| 9089 | Gm7244 | NM_001101597.2 | chr9:31271400-31275385 |
| 9090 | Gm7257 | NM_001167586.1 | chr9:36431883-36434938 |
| 9091 | Gm7271 | NR_033501.1 | chr5:76484076-76516628 |
| 9092 | Gm732 | NM_001033252.2 | chrX:107945734-107948436 |
| 9093 | Gm7325 | NM_001177468.1 | chr17:45600966-45602067 |
| 9094 | Gm7334 | NR_002700.1 | chr16:78359862-78376757 |
| 9095 | Gm7337 | NR_003652.2 | chr5:87850358-87853012 |
| 9096 | Gm7361 | NM_001281527.1 | chr5:26257761-26262188 |
| 9097 | Gm7367 | NR_003376.2 | chr11:116434212-116439031 |
| 9098 | Gm7444 | NR_033529.1 | chr9:56027635-56031573 |
| 9099 | Gm7457 | NR_045707.1 | chr6:142814230-142833494 |
| 9100 | Gm7534 | NM_001080712.1 | chr4:134190803-134203004 |
| 9101 | Gm7538 | NR_046033.1 | chr5:119194312-119200018 |
| 9102 | Gm7550 | NR_033689.1 | chr12:54387732-54391318 |
| 9103 | Gm7609 | NM_001081746.1 | chr1:85199610-85213762 |
| 9104 | Gm7616 | NM_001101600.1 | chr9:59951293-59959044 |
| 9105 | Gm765 | NM_001128092.1 | chr6:98238013-98342754 |
| 9106 | Gm766 | NM_001145390.1 | chr6:142785780-142804466 |
| 9107 | Gm7694 | NM_001198955.1 | chr1:170298192-170306332 |
| 9108 | Gm7714 | NM_001110779.1 | chr5:88268918-88282835 |
| 9109 | Gm773 | NM_001033423.2 | chrX:56189826-56212881 |
| 9110 | Gm7788 | NR_110491.1 | chr18:23217595-23217818 |
| 9111 | Gm7849 | NM_001177518.1 | chr8:21455426-21594465 |
| 9112 | Gm7854 | NR_028417.1 | chr5:43151685-43235354 |
| 9113 | Gm7861 | NM_001177526.1 | chr8:21455538-21594465 |
| 9114 | Gm7903 | NM_001242937.1 | chrX:135373283-135383393 |
| 9115 | Gm7904 | NR_003372.1 | chr9:21607083-21608329 |
| 9116 | Gm7977 | NR_040408.1 | chr3:48026011-48026759 |
| 9117 | Gm7978 | NM_001270457.1 | chr5:95732092-95736173 |

Fig.21 - 48

| | | | | | | | |
|---|---|---|---|---|---|---|---|
| 9118 | Gm805 | NR_046081.1 | chr12:86169340-86195102 | 9215 | Gna11 | NM_010301.3 | chr10:81528731-81545046 |
| 9119 | Gm806 | NM_001033400.2 | chr13:50467306-50475355 | 9216 | Gna12 | NM_010302.2 | chr5:140759943-140830431 |
| 9120 | Gm8096 | NR_033590.1 | chr8:28083193-28084853 | 9217 | Gna13 | NM_010303.3 | chr11:109362793-109401369 |
| 9121 | Gm813 | NM_001033404.2 | chr16:58613685-58616978 | 9218 | Gna14 | NM_008137.4 | chr19:16435666-16610818 |
| 9122 | Gm815 | NM_001033407.2 | chr19:26885924-26888647 | 9219 | Gna15 | NM_010304.3 | chr10:81502313-81524225 |
| 9123 | Gm8179 | NR_046039.1 | chr8:32151894-32181847 | 9220 | Gnai1 | NM_010305.1 | chr5:18265134-18360413 |
| 9124 | Gm8221 | NR_033577.1 | chr15:77618967-77627398 | 9221 | Gnai2 | NM_008138.4 | chr9:107614137-107635342 |
| 9125 | Gm8234 | NR_004433.3 | chr3:58652041-58654041 | 9222 | Gnai3 | NM_010306.3 | chr3:108107274-108146152 |
| 9126 | Gm826 | NM_001033411.3 | chr2:160311400-160327494 | 9223 | Gnal | NM_010307.3 | chr18:67133559-67226791 |
| 9127 | Gm8267 | NM_001162954.1 | chr14:44717166-44724987 | 9224 | Gnao1 | NM_001113384.1 | chr8:93810837-93960694 |
| 9128 | Gm829 | NM_001033412.2 | chr4:45683588-45723706 | 9225 | Gnaq | NM_008139.5 | chr19:16132830-16387453 |
| 9129 | Gm8298 | NM_001243003.1 | chr3:59861050-59877313 | 9226 | Gnas | NM_001077507.2 | chr2:174297858-174346742 |
| 9130 | Gm8300 | NM_001177565.1 | chr12:87514315-87518265 | 9227 | Gnat1 | NM_008140.2 | chr9:107674473-107679592 |
| 9131 | Gm833 | NR_033138.1 | chr4:152697539-152700922 | 9228 | Gnat2 | NM_008141.3 | chr3:108093065-108101430 |
| 9132 | Gm8363 | NR_073378.1 | chr17:5480988-5483069 | 9229 | Gnat3 | NM_001081143.1 | chr5:17962569-18019668 |
| 9133 | Gm8369 | NM_001164202.1 | chr19:11492037-11512577 | 9230 | Gnaz | NM_010311.3 | chr10:74967230-75015877 |
| 9134 | Gm839 | NR_033190.1 | chr6:89212196-89216179 | 9231 | Gnb1 | NM_001160016.1 | chr4:155491360-155559269 |
| 9135 | Gm8439 | NM_001101603.1 | chr4:120588744-120609672 | 9232 | Gnb1l | NM_001081682.2 | chr16:18498712-18566680 |
| 9136 | Gm853 | NM_001034872.2 | chr4:130209108-130222295 | 9233 | Gnb2 | NM_010312.4 | chr5:137528128-137533229 |
| 9137 | Gm8579 | NR_036696.1 | chr6:3288518-3317019 | 9234 | Gnb2l1 | NM_008143.3 | chr11:48800359-48806241 |
| 9138 | Gm8580 | NR_027478.1 | chr10:93665243-93665854 | 9235 | Gnb3 | NM_013530.1 | chr6:124834239-124840275 |
| 9139 | Gm8615 | NR_028061.1 | chr5:149154127-149156192 | 9236 | Gnb4 | NM_013531.4 | chr3:32583527-32616535 |
| 9140 | Gm8633 | NR_045179.1 | chr10:98299926-98351867 | 9237 | Gnb5 | NM_010313.2 | chr9:75306287-75345923 |
| 9141 | Gm867 | NM_001277132.1 | chr10:75937722-75940672 | 9238 | Gne | NM_001190414.1 | chr4:44036822-44072673 |
| 9142 | Gm8677 | NM_001167147.1 | chr7:22533964-22534887 | 9239 | Gng10 | NM_025277.3 | chr4:59035155-59041899 |
| 9143 | Gm8693 | NM_001167154.1 | chr7:22691396-22692317 | 9240 | Gng11 | NM_025331.2 | chr6:4003986-4008446 |
| 9144 | Gm8709 | NR_033633.1 | chr10:26673445-26674651 | 9241 | Gng12 | NM_001177556.1 | chr6:66896396-67021361 |
| 9145 | Gm8765 | NM_001244649.1 | chr13:50698328-50703409 | 9242 | Gng13 | NM_022422.5 | chr17:25717171-25719102 |
| 9146 | Gm8773 | NR_033499.1 | chr5:5573798-5576203 | 9243 | Gng2 | NM_001038637.1 | chr14:19872558-19977249 |
| 9147 | Gm8787 | NM_001099310.1 | chrX:79330512-79358068 | 9244 | Gng3 | NM_010316.3 | chr19:8836928-8839246 |
| 9148 | Gm8801 | NR_028278.1 | chr17:35947153-35953317 | 9245 | Gng4 | NM_010317.3 | chr13:13784306-13827895 |
| 9149 | Gm8817 | NM_001101606.1 | chrX:167063448-167070370 | 9246 | Gng5 | NM_010318.2 | chr3:146499835-146505543 |
| 9150 | Gm884 | NM_001033434.2 | chr11:103534576-103614739 | 9247 | Gng7 | NM_001038655.1 | chr10:80948623-81001365 |
| 9151 | Gm8882 | NM_001177588.1 | chr6:132361104-132364134 | 9248 | Gng8 | NM_010320.3 | chr7:16891785-16895435 |
| 9152 | Gm8883 | NR_027658.1 | chr1:71888111-71891204 | 9249 | Gngt1 | NM_010314.2 | chr6:3994011-3997436 |
| 9153 | Gm8884 | NR_026561.1 | chr9:48454343-48454727 | 9250 | Gngt2 | NM_001038664.2 | chr11:95842667-95845731 |
| 9154 | Gm8898 | NM_001177405.1 | chr2:175372461-176533821 | 9251 | Gnl1 | NM_008136.2 | chr17:35979954-35989462 |
| 9155 | Gm8909 | NM_001081032.2 | chr17:36164443-36168537 | 9252 | Gnl2 | NM_145552.2 | chr4:125030013-125055382 |
| 9156 | Gm8979 | NR_030719.1 | chr7:106075730-106079148 | 9253 | Gnl3 | NM_153547.6 | chr14:31012432-31019131 |
| 9157 | Gm8989 | NR_030720.1 | chr7:106075731-106324528 | 9254 | Gnl3l | NM_001168600.1 | chrX:150983132-151017293 |
| 9158 | Gm8994 | NM_001142734.1 | chr6:136327538-136329983 | 9255 | Gnmt | NM_010321.1 | chr17:46725663-46729165 |
| 9159 | Gm9 | NM_001033234.2 | chrX:37208501-37211041 | 9256 | Gnpat | NM_010322.3 | chr8:124863032-124890057 |
| 9160 | Gm904 | NM_001033770.1 | chr13:50643227-50645838 | 9257 | Gnpda1 | NM_011937.2 | chr18:38327536-38338993 |
| 9161 | Gm9047 | NM_001145360.1 | chr6:29471436-29473429 | 9258 | Gnpda2 | NM_001038015.1 | chr5:69575001-69592285 |
| 9162 | Gm9054 | NR_045872.1 | chr3:95985509-95986967 | 9259 | Gnpnat1 | NM_019425.2 | chr14:45376420-45388796 |
| 9163 | Gm906 | NM_001033438.2 | chr13:50245180-50250308 | 9260 | Gnptab | NM_001004164.2 | chr10:88379411-88447329 |
| 9164 | Gm9079 | NR_004052.1 | chr10:122078111-122079879 | 9261 | Gnptg | NM_172529.3 | chr17:25234317-25240116 |
| 9165 | Gm9112 | NM_001177365.1 | chrX:102706887-102707670 | 9262 | Gnrh1 | NM_008145.3 | chr14:67745228-67749436 |
| 9166 | Gm9125 | NM_001163730.2 | chr3:93936632-94054885 | 9263 | Gnrhr | NM_010323.2 | chr5:86180753-86197901 |
| 9167 | Gm9159 | NR_033398.1 | chrX:103545499-103547699 | 9264 | Gns | NM_029364.3 | chr10:121365089-121397245 |
| 9168 | Gm9199 | NR_027860.1 | chr14:73025602-73026890 | 9265 | Golga1 | NM_001290649.1 | chr2:39016155-39065541 |
| 9169 | Gm9268 | NM_001105061.1 | chr7:43018797-43048106 | 9266 | Golga2 | NM_001080968.1 | chr2:32288252-32307921 |
| 9170 | Gm933 | NM_001256309.1 | chr16:32804887-32810446 | 9267 | Golga3 | NM_008146.3 | chr5:110176700-110223155 |
| 9171 | Gm9376 | NM_001101609.1 | chr14:118267157-118267770 | 9268 | Golga4 | NM_018748.3 | chr9:118506317-118582519 |
| 9172 | Gm94 | NM_001033280.2 | chr18:43777195-43792878 | 9269 | Golga5 | NM_001199004.1 | chr12:102469133-102497907 |
| 9173 | Gm9513 | NM_001128510.1 | chr9:36475637-36477180 | 9270 | Golga7 | NM_001042484.1 | chr8:23241325-23257080 |
| 9174 | Gm9573 | NM_001244654.1 | chr17:35617922-35626637 | 9271 | Golga7b | NM_001141983.1 | chr19:42247577-42270348 |
| 9175 | Gm960 | NM_001033447.3 | chr19:4625840-4698668 | 9272 | Golgb1 | NM_030035.1 | chr16:36885010-36933085 |
| 9176 | Gm9696 | NR_037189.1 | chr3:59952308-59973594 | 9273 | Golim4 | NM_001291069.1 | chr3:75876182-75956949 |
| 9177 | Gm973 | NM_001013771.2 | chr1:59516263-59634509 | 9274 | Golm1 | NM_001035122.2 | chr13:59634995-59675784 |
| 9178 | Gm9731 | NR_003107.1 | chr8:27296044-27297200 | 9275 | Golph3 | NM_025673.2 | chr15:12321495-12351267 |
| 9179 | Gm9733 | NM_001076679.2 | chr3:15296550-15332302 | 9276 | Golph3l | NM_001177669.1 | chr3:95588933-95619247 |
| 9180 | Gm9758 | NM_198666.3 | chr5:14910123-14914889 | 9277 | Golt1a | NM_026680.4 | chr1:133309822-133323026 |
| 9181 | Gm9767 | NR_028030.2 | chr10:26078254-26079447 | 9278 | Golt1b | NM_025872.4 | chr6:142387242-142403858 |
| 9182 | Gm9776 | NR_045619.1 | chr13:94356748-94358923 | 9279 | Gon4l | NM_001242372.1 | chr3:88835230-88910099 |
| 9183 | Gm9833 | NR_045710.1 | chr3:10088276-10092562 | 9280 | Gopc | NM_001199272.1 | chr10:52337023-52382124 |
| 9184 | Gm9839 | NM_001199956.1 | chr1:32519562-32520999 | 9281 | Gorab | NM_178883.6 | chr1:163384902-163403669 |
| 9185 | Gm9855 | NR_037190.1 | chr10:82629841-82650277 | 9282 | Gorasp1 | NM_028976.2 | chr9:119925672-119937558 |
| 9186 | Gm9866 | NR_045868.1 | chr12:27140795-27160516 | 9283 | Gorasp2 | NM_027352.4 | chr2:70661508-70691725 |
| 9187 | Gm9871 | NR_027989.1 | chr6:101774248-101801982 | 9284 | Gosr1 | NM_016810.3 | chr11:76726601-76763555 |
| 9188 | Gm9895 | NR_045687.1 | chr19:29067300-29069503 | 9285 | Gosr2 | NM_019650.3 | chr11:103676848-103697710 |
| 9189 | Gm9899 | NR_040427.1 | chr5:30573986-30588619 | 9286 | Got1 | NM_010324.2 | chr19:43499752-43524605 |
| 9190 | Gm9920 | NR_045093.1 | chr15:55099916-55113582 | 9287 | Got1l1 | NM_029674.1 | chr8:27197458-27202547 |
| 9191 | Gm9926 | NR_040528.1 | chr18:66504177-66511737 | 9288 | Got2 | NM_010325.2 | chr8:95864136-95888365 |
| 9192 | Gm9958 | NR_045618.1 | chr5:90366996-90368488 | 9289 | Gp1ba | NM_010326.2 | chr11:70639121-70642055 |
| 9193 | Gm996 | NM_001005424.2 | chr2:25575415-25580099 | 9290 | Gp1bb | NM_001001999.1 | chr16:18620318-18622403 |
| 9194 | Gm9961 | NR_033509.1 | chr16:11901363-11930594 | 9291 | Gp2 | NM_025989.3 | chr7:119442543-119459272 |
| 9195 | Gm9962 | NR_033504.1 | chr7:57387271-57409941 | 9292 | Gp49a | NM_001291892.1 | chr10:51480611-51486329 |
| 9196 | Gm9992 | NM_001142539.1 | chr17:7363711-7385305 | 9293 | Gp5 | NM_008148.4 | chr16:30307684-30310781 |
| 9197 | Gm9994 | NM_001205249.1 | chr1:93918436-93941239 | 9294 | Gp6 | NM_001163014.1 | chr7:4368712-4397744 |
| 9198 | Gm9999 | NR_033461.1 | chr7:46976631-46987803 | 9295 | Gp9 | NM_018762.1 | chr6:87778135-87779762 |
| 9199 | Gmcl1 | NM_011818.3 | chr6:86691767-86733378 | 9296 | Gpa33 | NM_021610.1 | chr1:166130459-166166510 |
| 9200 | Gmcl1l | NM_027955.3 | chrX:32560054-32562009 | 9297 | Gpaa1 | NM_010331.2 | chr15:76331293-76334899 |
| 9201 | Gmds | NM_146041.2 | chr13:31819585-32338544 | 9298 | Gpalpp1 | NM_026177.3 | chr14:76086231-76110815 |
| 9202 | Gmeb1 | NM_001122992.1 | chr4:132221024-132261549 | 9299 | Gpam | NM_008149.3 | chr19:55069733-55099447 |
| 9203 | Gmeb2 | NM_198169.2 | chr2:181251450-181287966 | 9300 | Gpank1 | NM_001128597.1 | chr17:35121495-35124815 |
| 9204 | Gmfb | NM_022023.2 | chr14:46808148-46822242 | 9301 | Gpat2 | NM_001081089.2 | chr2:127425198-127436092 |
| 9205 | Gmfg | NM_001039192.1 | chr7:28440936-28446895 | 9302 | Gpatch1 | NM_026181.1 | chr7:35276543-35318440 |
| 9206 | Gmip | NM_198101.1 | chr8:69808686-69821870 | 9303 | Gpatch11 | NM_181649.6 | chr17:78835515-78848308 |
| 9207 | Gml | NM_001177524.1 | chr15:74813454-74818815 | 9304 | Gpatch2 | NM_026367.4 | chr1:187215510-187351429 |
| 9208 | Gmnc | NM_001013761.2 | chr16:26957234-26991652 | 9305 | Gpatch2l | NM_027405.2 | chr12:86241877-86291368 |
| 9209 | Gmnn | NM_020567.2 | chr13:24751844-24761937 | 9306 | Gpatch3 | NM_172876.2 | chr4:133574744-133584242 |
| 9210 | Gmppa | NM_133708.3 | chr1:75435942-75443176 | 9307 | Gpatch4 | NM_001110809.2 | chr3:88043105-88055994 |
| 9211 | Gmppb | NM_177910.3 | chr9:108049289-108051936 | 9308 | Gpatch8 | NM_001159492.1 | chr11:102475915-102556158 |
| 9212 | Gmpr | NM_025508.5 | chr13:45507443-45546386 | 9309 | Gpbar1 | NM_174985.1 | chr1:74278599-74279589 |
| 9213 | Gmpr2 | NM_177992.2 | chr14:55672234-55678751 | 9310 | Gpbp1 | NM_001122963.1 | chr13:111425679-111490041 |
| 9214 | Gmps | NM_001033300.2 | chr3:63976142-64019078 | 9311 | Gpbp1l1 | NM_029868.2 | chr4:116557726-116593882 |

Fig.21 - 49

| | | | |
|---|---|---|---|
| 9312 | Gpc1 | NM_016696.4 | chr1:92831685-92860196 |
| 9313 | Gpc2 | NM_172412.2 | chr5:138273659-138279937 |
| 9314 | Gpc3 | NM_016697.3 | chrX:52272426-52613974 |
| 9315 | Gpc4 | NM_008150.2 | chrX:52053017-52164923 |
| 9316 | Gpc5 | NM_175500.4 | chr14:115092214-116525192 |
| 9317 | Gpc6 | NM_001079844.2 | chr14:116925296-117979529 |
| 9318 | Gpcpd1 | NM_001042672.1 | chr2:132529082-132578282 |
| 9319 | Gpd1 | NM_010271.2 | chr15:99717592-99725007 |
| 9320 | Gpd1l | NM_175380.5 | chr9:114899338-114933987 |
| 9321 | Gpd2 | NM_001145820.1 | chr2:57237677-57370719 |
| 9322 | Gper1 | NM_029771.3 | chr5:139423178-139427800 |
| 9323 | Gpha2 | NM_130453.3 | chr19:6226400-6227768 |
| 9324 | Gphb5 | NM_175644.3 | chr12:75411719-75416781 |
| 9325 | Gphn | NM_145965.2 | chr12:78226654-78684769 |
| 9326 | Gpi1 | NM_008155.4 | chr7:34201326-34230336 |
| 9327 | Gpihbp1 | NM_026730.1 | chr15:75596657-75598213 |
| 9328 | Gpkow | NM_173747.3 | chrX:7697133-7710259 |
| 9329 | Gpld1 | NM_008156.2 | chr13:24943151-24991936 |
| 9330 | Gpm6a | NM_001253754.1 | chr8:54779433-55060878 |
| 9331 | Gpm6b | NM_001177955.1 | chrX:166344552-166389033 |
| 9332 | Gpn1 | NM_133756.4 | chr5:31494760-31511627 |
| 9333 | Gpn2 | NM_001290742.1 | chr4:133584372-133591735 |
| 9334 | Gpn3 | NM_024216.1 | chr5:122372507-122382769 |
| 9335 | Gpnmb | NM_053110.4 | chr6:49036517-49058182 |
| 9336 | Gpr1 | NM_146250.2 | chr1:63182570-63214281 |
| 9337 | Gpr101 | NM_001033360.3 | chrX:57496667-57503757 |
| 9338 | Gpr107 | NM_178760.4 | chr2:31152315-31216567 |
| 9339 | Gpr108 | NM_030084.4 | chr17:57234914-57247689 |
| 9340 | Gpr110 | NM_133776.2 | chr17:43270346-43324406 |
| 9341 | Gpr111 | NM_001033493.2 | chr17:42708936-42742179 |
| 9342 | Gpr113 | NM_001014394.2 | chr5:30193430-30205722 |
| 9343 | Gpr114 | NM_001033468.3 | chr8:94923693-94943290 |
| 9344 | Gpr115 | NM_001289499.1 | chr17:42656886-42692284 |
| 9345 | Gpr116 | NM_001081178.1 | chr17:43389465-43459557 |
| 9346 | Gpr119 | NM_181751.2 | chrX:48667978-48674478 |
| 9347 | Gpr12 | NM_001010941.2 | chr5:146582149-146585239 |
| 9348 | Gpr123 | NM_177469.3 | chr7:139834173-139878088 |
| 9349 | Gpr124 | NM_054044.2 | chr8:27085840-27123436 |
| 9350 | Gpr125 | NM_133911.1 | chr5:49959950-50058996 |
| 9351 | Gpr126 | NM_001002268.3 | chr10:14402584-14545036 |
| 9352 | Gpr128 | NM_172825.3 | chr16:56724608-56795858 |
| 9353 | Gpr132 | NM_019925.4 | chr12:112850875-112860916 |
| 9354 | Gpr133 | NM_001081342.1 | chr5:129096749-129204599 |
| 9355 | Gpr135 | NM_181752.1 | chr12:72069617-72070991 |
| 9356 | Gpr137 | NM_001177360.1 | chr19:6938069-6941193 |
| 9357 | Gpr137b | NM_031999.2 | chr13:13357619-13393624 |
| 9358 | Gpr137b-ps | NR_003568.1 | chr13:12614064-12650395 |
| 9359 | Gpr137c | NM_027518.2 | chr14:45219716-45280976 |
| 9360 | Gpr139 | NM_001024138.1 | chr7:119144322-119184374 |
| 9361 | Gpr141 | NM_181754.4 | chr13:19749681-19824257 |
| 9362 | Gpr142 | NM_181749.1 | chr11:114798923-114806745 |
| 9363 | Gpr143 | NM_010951.3 | chrX:152781920-152808646 |
| 9364 | Gpr146 | NM_001038703.2 | chr5:139389903-139396414 |
| 9365 | Gpr149 | NM_177346.4 | chr3:62529962-62605140 |
| 9366 | Gpr15 | NM_001162955.1 | chr16:58717434-58718724 |
| 9367 | Gpr150 | NM_175495.2 | chr13:76054850-76056996 |
| 9368 | Gpr151 | NM_181543.1 | chr18:42578019-42579652 |
| 9369 | Gpr152 | NM_206973.2 | chr19:4139798-4145740 |
| 9370 | Gpr153 | NM_178406.2 | chr4:152274361-152285337 |
| 9371 | Gpr155 | NM_001190297.2 | chr2:73341505-73386480 |
| 9372 | Gpr156 | NM_153394.2 | chr16:37916495-38007529 |
| 9373 | Gpr157 | NM_177366.3 | chr4:150087502-150105927 |
| 9374 | Gpr158 | NM_001004761.1 | chr2:21367566-21830542 |
| 9375 | Gpr160 | NM_001134385.2 | chr3:30855949-30897194 |
| 9376 | Gpr161 | NM_001081126.2 | chr1:165295765-165326745 |
| 9377 | Gpr162 | NM_013533.3 | chr6:124858444-124863917 |
| 9378 | Gpr165 | NM_029536.3 | chrX:96713467-96719388 |
| 9379 | Gpr17 | NM_001025381.2 | chr18:31942998-31949636 |
| 9380 | Gpr171 | NM_173398.3 | chr3:59096447-59101821 |
| 9381 | Gpr173 | NM_027543.4 | chrX:152343598-152368704 |
| 9382 | Gpr174 | NM_001033251.4 | chrX:107256027-107296769 |
| 9383 | Gpr176 | NM_201367.3 | chr2:118277097-118373419 |
| 9384 | Gpr179 | NM_001081220.1 | chr11:97332108-97352073 |
| 9385 | Gpr18 | NM_182806.1 | chr14:121911433-121915774 |
| 9386 | Gpr180 | NM_021434.5 | chr14:118137126-118164232 |
| 9387 | Gpr182 | NM_007412.2 | chr10:127749601-127751798 |
| 9388 | Gpr183 | NM_183031.2 | chr14:121952330-121965193 |
| 9389 | Gpr19 | NM_001167694.2 | chr6:134869091-134897925 |
| 9390 | Gpr20 | NM_173365.2 | chr15:73694606-73707505 |
| 9391 | Gpr21 | NM_177383.4 | chr2:37516625-37519281 |
| 9392 | Gpr22 | NM_175191.4 | chr12:31706866-31713926 |
| 9393 | Gpr25 | NM_001101516.1 | chr1:136258913-136260873 |
| 9394 | Gpr26 | NM_173410.3 | chr7:131966459-131985633 |
| 9395 | Gpr27 | NM_008158.1 | chr6:99692678-99693818 |
| 9396 | Gpr3 | NM_008154.3 | chr4:133209339-133212536 |
| 9397 | Gpr31b | NM_001013832.2 | chr17:13051320-13052280 |
| 9398 | Gpr33 | NM_008159.2 | chr12:52023003-52028063 |
| 9399 | Gpr34 | NM_011823.4 | chrX:13632768-13640858 |
| 9400 | Gpr35 | NM_001104529.1 | chr1:92973118-92986391 |
| 9401 | Gpr37 | NM_010338.2 | chr6:25668522-25689980 |
| 9402 | Gpr37l1 | NM_134438.3 | chr1:135160249-135167681 |
| 9403 | Gpr39 | NM_027677.2 | chr1:125676995-125873861 |
| 9404 | Gpr4 | NM_175668.4 | chr7:19212537-19224176 |
| 9405 | Gpr45 | NM_053107.4 | chr1:42952871-43035449 |
| 9406 | Gpr50 | NM_010340.2 | chrX:71663666-71669257 |
| 9407 | Gpr52 | NM_001146330.1 | chr1:160576667-160577753 |
| 9408 | Gpr55 | NM_001033290.2 | chr1:85939536-85961055 |

| | | | |
|---|---|---|---|
| 9409 | Gpr56 | NM_001198894.1 | chr8:94977108-95014208 |
| 9410 | Gpr6 | NM_199058.1 | chr10:41070492-41071584 |
| 9411 | Gpr61 | NM_001305461.1 | chr3:108148321-108154986 |
| 9412 | Gpr62 | NM_001159652.1 | chr9:106463959-106465940 |
| 9413 | Gpr63 | NM_030733.3 | chr4:24973418-25009233 |
| 9414 | Gpr64 | NM_001079847.2 | chrX:160390689-160498076 |
| 9415 | Gpr65 | NM_008152 | chr12:98268634-98276722 |
| 9416 | Gpr68 | NM_001177673.1 | chr12:100876681-100908198 |
| 9417 | Gpr75 | NM_175490.4 | chr11:30885357-30893725 |
| 9418 | Gpr82 | NM_175669.3 | chrX:13661362-13667433 |
| 9419 | Gpr83 | NM_010287.3 | chr9:14860209-14870789 |
| 9420 | Gpr84 | NM_030720.1 | chr15:103308234-103310438 |
| 9421 | Gpr85 | NM_145066.4 | chr6:13835073-13839848 |
| 9422 | Gpr87 | XM_006502394.2 | chr3:59178904-59195468 |
| 9423 | Gpr88 | NM_022427.2 | chr3:116249653-116253484 |
| 9424 | Gpr89 | NM_026229.4 | chr3:96871065-96905298 |
| 9425 | Gpr97 | NM_173036.3 | chr8:95017691-95045249 |
| 9426 | Gpr98 | NM_054053.3 | chr13:81095067-81633144 |
| 9427 | Gprasp1 | NM_001004359.2 | chrX:135742691-135803468 |
| 9428 | Gprasp2 | NM_001163015.1 | chrX:135839033-135844730 |
| 9429 | Gprc5a | NM_181444.5 | chr6:135065661-135084708 |
| 9430 | Gprc5b | NM_001195774.1 | chr7:118972039-118995211 |
| 9431 | Gprc5c | NM_001110337.1 | chr11:114851531-114872617 |
| 9432 | Gprc5d | NM_001205396.1 | chr6:135105990-135118283 |
| 9433 | Gprc6a | NM_153071.1 | chr10:51614822-51631458 |
| 9434 | Gprin1 | NM_012014.3 | chr13:54736672-54749669 |
| 9435 | Gprin2 | NM_183209.2 | chr14:34194440-34201633 |
| 9436 | Gprin3 | NM_183183.2 | chr6:59352460-59426290 |
| 9437 | Gps1 | NM_001177874.1 | chr11:120784271-120789102 |
| 9438 | Gps2 | NM_019726.3 | chr11:69914191-69916591 |
| 9439 | Gpsm1 | NM_001199146.1 | chr2:26315532-26348237 |
| 9440 | Gpsm2 | NM_029522.2 | chr3:108678637-108722299 |
| 9441 | Gpsm3 | NM_134116.5 | chr17:34589805-34591754 |
| 9442 | Gpt | NM_182805.2 | chr15:76696763-76699675 |
| 9443 | Gpt2 | NM_173866.3 | chr8:85492616-85527558 |
| 9444 | Gpx1 | NM_008160.1 | chr9:108339079-108340344 |
| 9445 | Gpx2 | NM_030677.2 | chr12:76792334-76795554 |
| 9446 | Gpx2-ps1 | NR_033563.1 | chr7:100264543-100265561 |
| 9447 | Gpx3 | NM_008161.3 | chr11:54902853-54910382 |
| 9448 | Gpx4 | NM_001037741.3 | chr10:80054018-80056439 |
| 9449 | Gpx5 | NM_010343.2 | chr13:21286428-21292686 |
| 9450 | Gpx6 | NM_145451.3 | chr13:21312202-21319624 |
| 9451 | Gpx7 | NM_024198.3 | chr4:108400216-108406713 |
| 9452 | Gpx8 | NM_027127.2 | chr13:113042762-113046388 |
| 9453 | Gramd1a | NM_027898.3 | chr7:31130126-31151050 |
| 9454 | Gramd1b | NM_172768.1 | chr9:40297906-40455764 |
| 9455 | Gramd1c | NM_001172107.1 | chr16:43980349-44027945 |
| 9456 | Gramd2 | NM_001033498.1 | chr9:59707763-59716424 |
| 9457 | Gramd3 | NM_026240.2 | chr18:56432131-56503792 |
| 9458 | Gramd4 | NM_001205353.1 | chr15:86058726-86137636 |
| 9459 | Grap | NM_027817.3 | chr11:61653320-61672777 |
| 9460 | Grap2 | NM_001289442.1 | chr15:80572596-80652854 |
| 9461 | Grasp | NM_019518.3 | chr15:101224206-101232756 |
| 9462 | Grb10 | NM_001177629.1 | chr11:11930498-12027971 |
| 9463 | Grb14 | NM_016719.1 | chr2:64912481-65022766 |
| 9464 | Grb2 | NM_008163.4 | chr11:115644044-115708597 |
| 9465 | Grb7 | NM_010346.2 | chr11:98446833-98455373 |
| 9466 | Grcc10 | NM_013535.1 | chr6:124739183-124741079 |
| 9467 | Greb1 | NM_001252071.1 | chr12:16670614-16757239 |
| 9468 | Greb1l | NM_001083628.1 | chr18:10325178-10562941 |
| 9469 | Grem1 | NM_011824.4 | chr2:113748674-113758648 |
| 9470 | Grem2 | NM_011825.1 | chr1:174833784-174921819 |
| 9471 | Grhl1 | NM_001161406.1 | chr12:24572286-24617391 |
| 9472 | Grhl2 | NM_026496.4 | chr15:37233035-37363568 |
| 9473 | Grhl3 | NM_001013756.1 | chr4:135541887-135573620 |
| 9474 | Grhpr | NM_080289.2 | chr4:44981393-44990734 |
| 9475 | Gria1 | NM_001113325.2 | chr11:57011617-57330244 |
| 9476 | Gria2 | NM_001039195.1 | chr3:80691491-80802791 |
| 9477 | Gria3 | NM_001281929.1 | chrX:41401300-41678601 |
| 9478 | Gria4 | NM_001113180.1 | chr9:4417892-4796234 |
| 9479 | Grid1 | NM_008166.2 | chr14:34820135-35581115 |
| 9480 | Grid2 | NM_008167.2 | chr6:63256856-64666279 |
| 9481 | Grid2ip | NM_001159321.1 | chr5:143357337-143391468 |
| 9482 | Grifin | NM_030022.1 | chr5:140563188-140565067 |
| 9483 | Grik1 | NM_010348.3 | chr16:87895896-88290258 |
| 9484 | Grik2 | NM_001111268.1 | chr10:49099462-49788754 |
| 9485 | Grik3 | NM_001081097.2 | chr4:125490830-125714173 |
| 9486 | Grik4 | NM_175481.5 | chr9:42520411-42944371 |
| 9487 | Grik5 | NM_008168.2 | chr7:25009850-25072369 |
| 9488 | Grin1 | NM_001177656.2 | chr2:25291176-25319187 |
| 9489 | Grin1os | NM_001242940.1 | chr2:25291219-25298928 |
| 9490 | Grin2a | NM_008170.2 | chr16:9577709-9992533 |
| 9491 | Grin2b | NM_008171.3 | chr6:135729804-136173511 |
| 9492 | Grin2c | NM_010350.2 | chr11:115249168-115267243 |
| 9493 | Grin2d | NM_008172.2 | chr7:45832482-45866681 |
| 9494 | Grin3a | NM_001033351.2 | chr4:49661610-49845769 |
| 9495 | Grin3b | NM_130455.2 | chr10:79970723-79977190 |
| 9496 | Grina | NM_023168.3 | chr15:76246806-76249904 |
| 9497 | Grip1 | NM_001277292.1 | chr10:119454033-120077161 |
| 9498 | Grip1os2 | NR_045359.1 | chr10:119746061-119761699 |
| 9499 | Grip2 | NM_001159507.1 | chr6:91761509-91807393 |
| 9500 | Gripap1 | NM_001290455.1 | chrX:7789992-7820567 |
| 9501 | Grk1 | NM_011881 | chr8:13405080-13421949 |
| 9502 | Grk4 | NM_001080743.1 | chr5:34660378-34701088 |
| 9503 | Grk5 | NM_018869.3 | chr19:60889748-61092555 |
| 9504 | Grk6 | NM_001038018.4 | chr13:55445330-55460927 |
| 9505 | Grm1 | NM_001114333.2 | chr10:10686058-11082331 |

Fig.21 - 50

| | | | |
|---|---|---|---|
| 9506 | Grm2 | NM_001160353.1 | chr9:106644533-106656109 |
| 9507 | Grm3 | NM_181850.2 | chr5:9485235-9725352 |
| 9508 | Grm4 | NM_001013385.2 | chr17:27422387-27503533 |
| 9509 | Grm5 | NM_001081414.2 | chr7:87584167-88135063 |
| 9510 | Grm6 | NM_173372.2 | chr11:50850684-50866208 |
| 9511 | Grm7 | NM_177328.3 | chr6:110645597-111567230 |
| 9512 | Grm8 | NM_008174.2 | chr6:27275120-28134369 |
| 9513 | Grn | NM_008175.4 | chr11:102430321-102436809 |
| 9514 | Grp | NM_175012.4 | chr18:65873485-65886596 |
| 9515 | Grpel1 | NM_024478.2 | chr5:36465184-36474077 |
| 9516 | Grpel2 | NM_021296.2 | chr18:61712423-61726331 |
| 9517 | Grpr | NM_008177.3 | chrX:163513903-163549736 |
| 9518 | Grrp1 | NM_001099296.2 | chr4:134251109-134254106 |
| 9519 | Grsf1 | NM_001098476.2 | chr5:88659232-88675580 |
| 9520 | Grtp1 | NM_025768.2 | chr8:13176868-13200624 |
| 9521 | Grwd1 | NM_153419.2 | chr7:45825222-45830789 |
| 9522 | Grxcr1 | NM_001018019.2 | chr5:68031834-68166398 |
| 9523 | Grxcr2 | NM_001033426.2 | chr18:41986200-41999049 |
| 9524 | Gsap | NM_175437.3 | chr5:21186266-21291701 |
| 9525 | Gsc | NM_010351.1 | chr12:104471208-104473236 |
| 9526 | Gsc2 | NM_029469.1 | chr16:17913553-17915059 |
| 9527 | Gsdma | NM_021347.4 | chr11:98664350-98677708 |
| 9528 | Gsdma2 | NM_029727.2 | chr11:98646758-98657957 |
| 9529 | Gsdma3 | NM_001007461.1 | chr11:98626359-98638089 |
| 9530 | Gsdmc | NM_031378.3 | chr15:63775970-63808739 |
| 9531 | Gsdmc2 | NM_001168274.1 | chr15:63824345-63845176 |
| 9532 | Gsdmc3 | NM_183194.3 | chr15:63857724-63878558 |
| 9533 | Gsdmc4 | NM_028992.1 | chr15:63891264-63912297 |
| 9534 | Gsdmcl1 | NR_108051.1 | chr15:63847308-63850942 |
| 9535 | Gsdmcl2 | NR_108053.1 | chr15:63880706-63889073 |
| 9536 | Gsdmcl-ps | NR_029414.1 | chr15:63914460-63925453 |
| 9537 | Gsdmd | NM_026960.4 | chr15:75862338-75867404 |
| 9538 | Gse1 | NM_001145896.1 | chr8:120537408-120581383 |
| 9539 | Gsg1 | NM_001080552.1 | chr6:135237329-135247884 |
| 9540 | Gsg1l | NM_001101488.1 | chr7:125878418-126082411 |
| 9541 | Gsg2 | NM_010353.2 | chr11:73135483-73138294 |
| 9542 | Gsk3a | NM_001031667.1 | chr7:25228258-25237851 |
| 9543 | Gsk3b | NM_019827.4 | chr16:38089000-38246079 |
| 9544 | Gskip | NM_178613.3 | chr12:105685351-105703057 |
| 9545 | Gsn | NM_001206367.1 | chr2:35256358-35307902 |
| 9546 | Gspt1 | NM_001130008.1 | chr16:11216240-11254325 |
| 9547 | Gspt2 | NM_008179.2 | chrX:94636068-94638561 |
| 9548 | Gsr | NM_010344.4 | chr8:33653237-33698162 |
| 9549 | Gss | NM_001291111.1 | chr2:155563180-155592810 |
| 9550 | Gsta1 | NM_008181.3 | chr9:78230668-78242683 |
| 9551 | Gsta2 | NM_008182.3 | chr9:78331019-78347145 |
| 9552 | Gsta3 | NM_001077353.2 | chr1:21240584-21265575 |
| 9553 | Gsta4 | NM_010357.3 | chr9:78191965-78209349 |
| 9554 | Gstcd | NM_026231.2 | chr3:132982550-133091740 |
| 9555 | Gstk1 | NM_029555.2 | chr6:42245934-42250441 |
| 9556 | Gstm1 | NM_010358.5 | chr3:108012249-108017973 |
| 9557 | Gstm2 | NM_008183.3 | chr3:107981701-107986436 |
| 9558 | Gstm3 | NM_010359.2 | chr3:107963695-107969174 |
| 9559 | Gstm4 | NM_001160411.1 | chr3:108040407-108044859 |
| 9560 | Gstm5 | NM_010360.2 | chr3:107895853-107898685 |
| 9561 | Gstm6 | NM_008184.3 | chr3:107938847-107943749 |
| 9562 | Gstm7 | NM_026672.2 | chr3:107926333-107931745 |
| 9563 | Gsto1 | NM_010362.2 | chr19:47854988-47864788 |
| 9564 | Gsto2 | NM_026619.2 | chr19:47865793-47886305 |
| 9565 | Gstp1 | NM_013541.1 | chr19:4035410-4037912 |
| 9566 | Gstp2 | NM_181796.2 | chr19:4040287-4042221 |
| 9567 | Gstt1 | NM_008185.3 | chr10:75783812-75798584 |
| 9568 | Gstt2 | NM_010361.2 | chr10:75831844-75834881 |
| 9569 | Gstt3 | NM_133994.3 | chr10:75774121-75781414 |
| 9570 | Gstt4 | NM_029472.3 | chr10:75814943-75822543 |
| 9571 | Gstz1 | NM_001252555.1 | chr12:87147164-87164723 |
| 9572 | Gsx1 | NM_008178.2 | chr5:147188695-147190946 |
| 9573 | Gsx2 | NM_133256.2 | chr5:75075600-75077893 |
| 9574 | Gt(ROSA)26Sor | NR_027008.1 | chr6:113070398-113077244 |
| 9575 | Gtdc1 | NM_172662.3 | chr2:44564411-44861622 |
| 9576 | Gtf2a1 | NM_031391.2 | chr12:91555261-91589649 |
| 9577 | Gtf2a1l | NM_023630.2 | chr17:88668659-88715150 |
| 9578 | Gtf2a2 | NM_001039519.2 | chr9:70012547-70022866 |
| 9579 | Gtf2b | NM_145546.1 | chr3:142765246-142783605 |
| 9580 | Gtf2e1 | NM_028812.3 | chr16:37509795-37539769 |
| 9581 | Gtf2e2 | NM_001167921.1 | chr8:33731913-33777173 |
| 9582 | Gtf2f1 | NM_133801.2 | chr17:57003401-57011288 |
| 9583 | Gtf2f2 | NM_026816.3 | chr14:75896936-76010865 |
| 9584 | Gtf2h1 | NM_001291075.1 | chr7:46796670-46823800 |
| 9585 | Gtf2h2 | NM_022011.4 | chr13:100468576-100492609 |
| 9586 | Gtf2h3 | NM_181410.3 | chr5:124579147-124597680 |
| 9587 | Gtf2h4 | NM_010364.4 | chr17:35667727-35673743 |
| 9588 | Gtf2h5 | NM_181392.3 | chr17:6079827-6085485 |
| 9589 | Gtf2i | NM_001080746.2 | chr5:134237831-134314760 |
| 9590 | Gtf2ird1 | NM_001081462.2 | chr5:134357660-134456716 |
| 9591 | Gtf2ird2 | NM_053266.1 | chr5:134184037-134218143 |
| 9592 | Gtf3a | NM_025652.3 | chr5:146948656-146955614 |
| 9593 | Gtf3c1 | NM_207239.1 | chr7:125640953-125707688 |
| 9594 | Gtf3c2 | NM_027901.2 | chr5:31156005-31180144 |
| 9595 | Gtf3c3 | NM_001033194.3 | chr1:54395876-54439026 |
| 9596 | Gtf3c4 | NM_001166033.1 | chr2:28822299-28840360 |
| 9597 | Gtf3c5 | NM_001290484.1 | chr2:28566244-28583279 |
| 9598 | Gtf3c6 | NM_026113.4 | chr10:40249202-40257665 |
| 9599 | Gtl3 | NM_008187.2 | chr8:95420249-95434869 |
| 9600 | Gtpbp1 | NM_013818.2 | chr15:79690895-79721479 |
| 9601 | Gtpbp10 | NM_153116.1 | chr5:5537456-5559501 |
| 9602 | Gtpbp2 | NM_001145979.1 | chr17:46161031-46169370 |

| | | | |
|---|---|---|---|
| 9603 | Gtpbp3 | NM_032544.3 | chr8:71488102-71493400 |
| 9604 | Gtpbp4 | NM_027000.4 | chr13:8972477-8996012 |
| 9605 | Gtpbp6 | NM_145147.5 | chr5:110103976-110108197 |
| 9606 | Gtpbp8 | NM_001159329.1 | chr16:44738878-44746363 |
| 9607 | Gtse1 | NM_001168672.1 | chr15:85859706-85876573 |
| 9608 | Gtsf1 | NM_028797.1 | chr15:103402458-103430427 |
| 9609 | Gtsf1l | NM_026630.2 | chr2:163087030-163089601 |
| 9610 | Guca1a | NM_008189.2 | chr17:47394557-47400584 |
| 9611 | Guca1b | NM_146079.1 | chr17:47385392-47392967 |
| 9612 | Guca2a | NM_008190.1 | chr4:119637731-119639465 |
| 9613 | Guca2b | NM_008191.2 | chr4:119656602-119658945 |
| 9614 | Gucd1 | NM_175133.1 | chr10:75506813-75517322 |
| 9615 | Gucy1a2 | NM_001033322.2 | chr9:3532348-3905787 |
| 9616 | Gucy1a3 | NM_021896.5 | chr3:82092426-82145877 |
| 9617 | Gucy1b2 | NM_001204340.1 | chr14:62392669-62456289 |
| 9618 | Gucy1b3 | NM_001161796.1 | chr3:82032003-82074711 |
| 9619 | Gucy2c | NM_001127318.1 | chr6:136697283-136781742 |
| 9620 | Gucy2d | NM_001130693.3 | chr7:98440415-98477478 |
| 9621 | Gucy2e | NM_008192.3 | chr11:69218116-69237022 |
| 9622 | Gucy2f | NM_001007576.2 | chrX:142079288-142196936 |
| 9623 | Gucy2g | NM_001081076.2 | chr19:55198097-55241236 |
| 9624 | Guf1 | NM_172711.3 | chr5:69556923-69574652 |
| 9625 | Guk1 | NM_001159410.1 | chr11:59183854-59187741 |
| 9626 | Gulo | NM_178747.3 | chr14:65986786-66009210 |
| 9627 | Gulp1 | NM_028450.3 | chr1:44551670-44796836 |
| 9628 | Gusb | NM_010368.1 | chr5:129989020-130002828 |
| 9629 | Gvin1 | NM_001039160.2 | chr7:105895118-106215340 |
| 9630 | Gxylt1 | NM_001033275.4 | chr15:93239741-93275084 |
| 9631 | Gxylt2 | NM_198612.2 | chr6:100704733-100805081 |
| 9632 | Gyg | NM_013755.3 | chr3:20122083-20155116 |
| 9633 | Gyk | NM_008194.3 | chrX:85701936-85776819 |
| 9634 | Gykl1 | NM_010293.3 | chr18:52693678-52695593 |
| 9635 | Gyltl1b | NM_001166633.2 | chr2:92365045-92371057 |
| 9636 | Gypa | NM_010369.3 | chr8:80494044-80510785 |
| 9637 | Gypc | NM_001048207.1 | chr18:32528319-32560034 |
| 9638 | Gys1 | NM_030678.3 | chr7:45434838-45456617 |
| 9639 | Gys2 | NM_145572.2 | chr6:142422612-142473109 |
| 9640 | Gzf1 | NM_028986.3 | chr2:148681119-148692949 |
| 9641 | Gzma | NM_010370.2 | chr13:113093826-113100981 |
| 9642 | Gzmb | NM_013542.2 | chr14:56258857-56262260 |
| 9643 | Gzmc | NM_010371.3 | chr14:56231400-56234657 |
| 9644 | Gzmd | NM_010372.2 | chr14:56129567-56132593 |
| 9645 | Gzme | NM_010373.3 | chr14:56117618-56120625 |
| 9646 | Gzmf | NM_010374.3 | chr14:56205262-56211407 |
| 9647 | Gzmg | NM_010375.2 | chr14:56156580-56159579 |
| 9648 | Gzmk | NM_008196.1 | chr13:113171873-113180897 |
| 9649 | Gzmm | NM_008504.3 | chr10:79689019-79695261 |
| 9650 | Gzmn | NM_153052.2 | chr14:56165795-56174599 |
| 9651 | H13 | NM_001159551.1 | chr2:152669460-152708668 |
| 9652 | H19 | NR_130973.1 | chr7:142575532-142578146 |
| 9653 | H1f0 | NM_008197.3 | chr15:79028211-79030500 |
| 9654 | H1fnt | NM_027304.2 | chr15:98255981-98257307 |
| 9655 | H1foo | NM_138311.3 | chr6:115944930-115950242 |
| 9656 | H1fx | NM_198622.1 | chr6:87980420-87981482 |
| 9657 | H2-Aa | NM_010378.2 | chr17:34282750-34287771 |
| 9658 | H2-Ab1 | NM_207105.3 | chr17:34263226-34269418 |
| 9659 | H2afb1 | NM_026627.2 | chr2:17996416-17996958 |
| 9660 | H2afb2 | NM_001281530.1 | chrX:116681177-116681525 |
| 9661 | H2afb3 | NM_001281531.1 | chrX:120312747-120313095 |
| 9662 | H2afj | NM_177688.4 | chr6:136808247-136810074 |
| 9663 | H2afv | NM_029938.1 | chr11:6427225-6444443 |
| 9664 | H2afx | NM_010436.2 | chr9:44334714-44336073 |
| 9665 | H2afy | NM_001159513.1 | chr13:56073621-56135550 |
| 9666 | H2afy2 | NM_207000.2 | chr10:61738646-61783864 |
| 9667 | H2afy3 | NR_003523.1 | chr15:62217540-62219451 |
| 9668 | H2afz | NM_016750.3 | chr3:137864486-137866922 |
| 9669 | H2bfm | NM_027067.2 | chrX:136927324-136928373 |
| 9670 | H2-Bl | NM_008199.2 | chr17:36080188-36084249 |
| 9671 | H2-D1 | NM_010380.3 | chr17:35263093-35267497 |
| 9672 | H2-DMa | NM_010386.4 | chr17:34122831-34139101 |
| 9673 | H2-DMb1 | NM_010387.3 | chr17:34153190-34160229 |
| 9674 | H2-DMb2 | NM_010388.4 | chr17:34145415-34151095 |
| 9675 | H2-Ea-ps | NM_010381.2 | chr17:34342211-34344645 |
| 9676 | H2-Eb1 | NM_010382.2 | chr17:34305866-34316674 |
| 9677 | H2-Eb2 | NM_001033978.3 | chr17:34325674-34341410 |
| 9678 | H2-K1 | NM_001001892.2 | chr17:33996011-34000333 |
| 9679 | H2-K2 | NR_004446.1 | chr17:33974658-33978791 |
| 9680 | H2-Ke2 | NM_001185182.1 | chr17:33938908-33940330 |
| 9681 | H2-Ke6 | NM_013543.2 | chr17:34026032-34028055 |
| 9682 | H2-L | NM_001267808.1 | chr17:35263122-35266730 |
| 9683 | H2-M1 | NM_177636.2 | chr17:36670007-36672197 |
| 9684 | H2-M10.1 | NM_013544.3 | chr17:36322859-36326150 |
| 9685 | H2-M10.2 | NM_177923.1 | chr17:36284280-36286421 |
| 9686 | H2-M10.3 | NM_201608.2 | chr17:36365003-36368417 |
| 9687 | H2-M10.4 | NM_177634.1 | chr17:36460163-36462329 |
| 9688 | H2-M10.5 | NM_177637.3 | chr17:36772909-36776235 |
| 9689 | H2-M10.6 | NM_201611.2 | chr17:36812174-36815566 |
| 9690 | H2-M11 | NM_177635.1 | chr17:36547074-36549252 |
| 9691 | H2-M2 | NM_008204.2 | chr17:37480850-37483529 |
| 9692 | H2-M3 | NM_013819.2 | chr17:37270233-37274485 |
| 9693 | H2-M5 | NM_001115075.1 | chr17:36986854-36989504 |
| 9694 | H2-M9 | NM_008205.1 | chr17:36640424-36642644 |
| 9695 | H2-Oa | NM_008206.2 | chr17:34092378-34095234 |
| 9696 | H2-Ob | NM_010389.3 | chr17:34238904-34245908 |
| 9697 | H2-Q1 | NM_010390.3 | chr17:35320557-35325099 |
| 9698 | H2-Q10 | NM_010391.4 | chr17:35470088-35474563 |
| 9699 | H2-Q2 | NM_010392.2 | chr17:35342332-35345722 |

Fig.21 - 51

| | | | |
|---|---|---|---|
| 9700 | H2-Q4 | NM_001143689.1 | chr17:35379616-35384674 |
| 9701 | H2-Q5 | NR_051981.1 | chr17:35394098-35395630 |
| 9702 | H2-Q6 | NM_207648.1 | chr17:35424876-35428361 |
| 9703 | H2-Q7 | NM_001198560.1 | chr17:35439154-35443773 |
| 9704 | H2-Q8 | NM_023124.5 | chr17:35424846-35428361 |
| 9705 | H2-Q9 | NM_001201460.1 | chr17:35439166-35443770 |
| 9706 | H2-T10 | NM_010395.7 | chr17:36115875-36121435 |
| 9707 | H2-T22 | NM_010397.4 | chr17:36038408-36042702 |
| 9708 | H2-T23 | NM_010398.3 | chr17:36029976-36032701 |
| 9709 | H2-T24 | NM_008207.3 | chr17:36005694-36020560 |
| 9710 | H2-T3 | NM_008208.4 | chr17:36185570-36190151 |
| 9711 | H2-T9 | NM_010399.2 | chr17:36038408-36042692 |
| 9712 | H3f3a | NM_008210.4 | chr1:180802567-180813603 |
| 9713 | H3f3b | NM_008211.3 | chr11:116021960-116024504 |
| 9714 | H60b | NM_001177775.1 | chr10:22273473-22288848 |
| 9715 | H60c | NM_001204916.2 | chr10:3256207-3267771 |
| 9716 | H6pd | NM_001291004.1 | chr4:149979473-150003236 |
| 9717 | Haao | NM_025325.2 | chr17:83831353-83846790 |
| 9718 | Habp2 | NM_146101.1 | chr19:56287937-56320089 |
| 9719 | Habp4 | NM_019986 | chr13:64161865-64186537 |
| 9720 | Hace1 | NM_172473.3 | chr10:45577828-45712345 |
| 9721 | Hacl1 | NM_019975.3 | chr14:31607225-31640965 |
| 9722 | Hadh | NM_008212.4 | chr3:131233419-131272101 |
| 9723 | Hadha | NM_178878.2 | chr5:30118303-30154980 |
| 9724 | Hadhb | NM_001289798.1 | chr5:30155252-30184593 |
| 9725 | Hagh | NM_001159626.1 | chr17:24850666-24864450 |
| 9726 | Haghl | NM_001271433.1 | chr17:25782786-25785586 |
| 9727 | Hal | NM_010401.3 | chr10:93488767-93516743 |
| 9728 | Hamp | NM_032541.1 | chr7:30942368-30944017 |
| 9729 | Hamp2 | NM_183257.3 | chr7:30922371-30924181 |
| 9730 | Hand1 | NM_008213.2 | chr11:57828712-57832147 |
| 9731 | Hand2 | NM_010402.4 | chr8:57320982-57324517 |
| 9732 | Hao1 | NM_010403.2 | chr2:134497360-134554352 |
| 9733 | Hao2 | NM_019545.4 | chr3:98874518-98893230 |
| 9734 | Hap1 | NM_010404.3 | chr11:100347326-100356141 |
| 9735 | Hapln1 | NM_013500.4 | chr13:89540635-89611832 |
| 9736 | Hapln2 | NM_022031.2 | chr3:88021749-88027511 |
| 9737 | Hapln3 | NM_178255.3 | chr7:79117026-79131018 |
| 9738 | Hapln4 | NM_177900.4 | chr8:70083528-70090862 |
| 9739 | Harbi1 | NM_178724.4 | chr2:91710949-91721566 |
| 9740 | Hars | NM_008214.4 | chr18:36766527-36783205 |
| 9741 | Hars2 | NM_080636.2 | chr18:36783208-36792560 |
| 9742 | Has1 | NM_008215.2 | chr17:17843325-17855188 |
| 9743 | Has2 | NM_008216.3 | chr15:56665626-56694546 |
| 9744 | Has2os | NR_002874.2 | chr15:56689943-56694460 |
| 9745 | Has3 | NM_008217.4 | chr8:106870241-106882902 |
| 9746 | Hat1 | NM_026115.4 | chr2:71389259-71441622 |
| 9747 | Haus1 | NM_146089.2 | chr18:77757566-77767780 |
| 9748 | Haus2 | NM_001290807.1 | chr2:120609594-120621559 |
| 9749 | Haus3 | NM_146159.1 | chr5:34153896-34169424 |
| 9750 | Haus4 | NM_145462.2 | chr14:54541784-54554361 |
| 9751 | Haus5 | NM_027999.1 | chr7:30653707-30664994 |
| 9752 | Haus6 | NM_173400.2 | chr4:86581284-86612022 |
| 9753 | Haus7 | NM_028633.3 | chrX:73437314-73459029 |
| 9754 | Haus8 | NM_001163042.1 | chr8:71251123-71272590 |
| 9755 | Havcr1 | NM_001166631.1 | chr11:46750510-46779578 |
| 9756 | Havcr2 | NM_134250.2 | chr11:46454930-46481254 |
| 9757 | Hax1 | NM_001282032.1 | chr3:89995445-89998716 |
| 9758 | Hba-a1 | NM_008218.2 | chr11:32283671-32297303 |
| 9759 | Hba-a2 | NM_001083955.1 | chr11:32283674-32297310 |
| 9760 | Hba-x | NM_010405.4 | chr11:32276599-32278115 |
| 9761 | Hbb-b1 | NM_001278161.1 | chr7:103826531-103827929 |
| 9762 | Hbb-bh1 | NM_008219.3 | chr7:103841637-103843162 |
| 9763 | Hbb-bh2 | NM_001127686.1 | chr7:103839123-103840521 |
| 9764 | Hbb-bs | NM_001201391.1 | chr7:103826522-103827928 |
| 9765 | Hbb-bt | NM_008220.5 | chr7:103812523-103813923 |
| 9766 | Hbb-y | NM_008221.4 | chr7:103851753-103853207 |
| 9767 | Hbegf | NM_010415.2 | chr18:36504926-36515805 |
| 9768 | Hbp1 | NM_153198.2 | chr12:31926454-31950535 |
| 9769 | Hbq1a | NM_175000.2 | chr11:32300068-32300873 |
| 9770 | Hbq1b | NM_001033981 | chr11:32286963-32287912 |
| 9771 | Hbs1l | NM_001042593.1 | chr10:21295978-21368889 |
| 9772 | Hc | NM_010406.2 | chr2:34983330-35061441 |
| 9773 | Hcar1 | NM_175520.4 | chr5:123876735-123880020 |
| 9774 | Hcar2 | NM_030701.3 | chr5:123863569-123865516 |
| 9775 | Hccs | NM_008222.4 | chrX:169311530-169320343 |
| 9776 | Hcfc1 | NM_008224.4 | chrX:73942791-73966357 |
| 9777 | Hcfc1r1 | NM_181821.1 | chr17:23673607-23675524 |
| 9778 | Hcfc2 | NM_001081218.1 | chr10:82699006-82741392 |
| 9779 | Hck | NM_010407.4 | chr2:153108467-153151441 |
| 9780 | Hcls1 | NM_008225.2 | chr16:36934982-36963214 |
| 9781 | Hcn1 | NM_010408.3 | chr13:117602319-117981028 |
| 9782 | Hcn2 | NM_008226.2 | chr10:79716633-79736108 |
| 9783 | Hcn3 | NM_008227.1 | chr3:89146774-89160157 |
| 9784 | Hcn4 | NM_001081192.1 | chr9:58823511-58860955 |
| 9785 | Hcrt | NM_010410.2 | chr11:100761692-100762931 |
| 9786 | Hcrtr1 | NM_001163027.1 | chr4:130130216-130138551 |
| 9787 | Hcrtr2 | NM_198962.3 | chr9:76225879-76323578 |
| 9788 | Hcst | NM_011827.3 | chr7:30417711-30419854 |
| 9789 | Hdac1 | NM_008228.2 | chr4:129516103-129542646 |
| 9790 | Hdac10 | NM_199198.2 | chr15:89123302-89128700 |
| 9791 | Hdac11 | NM_144919.2 | chr6:91156814-91174683 |
| 9792 | Hdac2 | NM_008229.2 | chr10:36974543-37001888 |
| 9793 | Hdac3 | NM_010411.2 | chr18:37936970-37954988 |
| 9794 | Hdac4 | NM_207225.2 | chr1:91928778-92180341 |
| 9795 | Hdac5 | NM_001077696.1 | chr11:102195746-102230172 |
| 9796 | Hdac6 | NM_001130416.1 | chrX:7930121-7947889 |
| 9797 | Hdac7 | NM_001204275.1 | chr15:97792663-97844502 |
| 9798 | Hdac8 | NM_027382.4 | chrX:102284637-102505158 |
| 9799 | Hdac9 | NM_001271386.1 | chr12:34047581-34917095 |
| 9800 | Hdc | NM_008230.6 | chr2:126593659-126618678 |
| 9801 | Hddc2 | NM_027168.2 | chr10:31313404-31328086 |
| 9802 | Hddc3 | NM_026812.2 | chr7:80343136-80346097 |
| 9803 | Hdgf | NM_008231.4 | chr3:87906320-87916132 |
| 9804 | Hdgfl1 | NM_008232.3 | chr13:26768171-26770165 |
| 9805 | Hdgfrp2 | NM_008233.3 | chr17:56079630-56100604 |
| 9806 | Hdgfrp3 | NM_013886.4 | chr7:81881255-81934459 |
| 9807 | Hdhd1a | NM_026108.3 | chr18:50567655-50568699 |
| 9808 | Hdhd2 | NM_001039201.1 | chr18:76944417-76972171 |
| 9809 | Hdhd3 | NM_024257.1 | chr4:62499053-62502200 |
| 9810 | Hdlbp | NM_133808.5 | chr1:93405939-93478917 |
| 9811 | Hdx | NM_001080549.2 | chrX:111575203-111697079 |
| 9812 | Heatr1 | NM_144835.4 | chr13:12395374-12438893 |
| 9813 | Heatr2 | NM_001081265.1 | chr5:139150222-139186505 |
| 9814 | Heatr3 | NM_172757.3 | chr8:88137884-88171943 |
| 9815 | Heatr5a | NM_177171.4 | chr12:51875872-51971321 |
| 9816 | Heatr5b | NM_001081179.1 | chr17:78752905-78835381 |
| 9817 | Heatr6 | NM_145432.3 | chr11:83753636-83783754 |
| 9818 | Heatr9 | NM_001045543.2 | chr11:83511678-83522099 |
| 9819 | Hebp1 | NM_013546.2 | chr6:135137518-135168215 |
| 9820 | Hebp2 | NM_019487.3 | chr10:18540122-18546076 |
| 9821 | Heca | NM_001033432.3 | chr10:17900465-17948067 |
| 9822 | Hectd1 | NM_144788.2 | chr12:51743721-51829536 |
| 9823 | Hectd2 | NM_001163471.1 | chr19:36554638-36621135 |
| 9824 | Hectd3 | NM_175244.3 | chr4:116995347-117005277 |
| 9825 | Hecw1 | NM_001081348.3 | chr13:14226437-14523226 |
| 9826 | Hecw2 | NM_001001883.3 | chr1:53806873-54195034 |
| 9827 | Heg1 | NM_175256.5 | chr16:33684465-33768195 |
| 9828 | Helb | NM_080446.2 | chr10:120083607-120112965 |
| 9829 | Hells | NM_008234.3 | chr19:38930989-38968277 |
| 9830 | Helq | NM_001081107.1 | chr5:100762147-100798600 |
| 9831 | Helt | NM_173789.4 | chr8:46292047-46294671 |
| 9832 | Helz | NM_198298.1 | chr11:107547959-107686943 |
| 9833 | Helz2 | NM_183162.2 | chr2:181227614-181242027 |
| 9834 | Hemgn | NM_053149.2 | chr4:46393988-46404183 |
| 9835 | Hemk1 | NM_133984.2 | chr9:107327081-107338350 |
| 9836 | Hemt1 | NM_010416.2 | chr15:74819075-74824436 |
| 9837 | Henmt1 | NM_001078646.1 | chr3:108940556-108960776 |
| 9838 | Hepacam | NM_175189.4 | chr9:37367605-37386571 |
| 9839 | Hepacam2 | NM_178899.5 | chr6:3457088-3494498 |
| 9840 | Heph | NM_001159627.1 | chrX:96456345-96574484 |
| 9841 | Hephl1 | NM_001164797.1 | chr9:15051840-15112108 |
| 9842 | Herc1 | NM_145617.3 | chr9:66350449-66508775 |
| 9843 | Herc2 | NM_010418.2 | chr7:56050154-56231798 |
| 9844 | Herc3 | NM_028705.3 | chr6:58833699-58920396 |
| 9845 | Herc4 | NM_026101.4 | chr10:63243796-63317881 |
| 9846 | Herc6 | NM_025992.2 | chr6:57580991-57665136 |
| 9847 | Herpud1 | NM_022331.1 | chr8:94386499-94395358 |
| 9848 | Herpud2 | NM_020586.2 | chr9:25108129-25151781 |
| 9849 | Hes1 | NM_008235.2 | chr16:30065356-30067796 |
| 9850 | Hes2 | NM_008236.4 | chr4:152158866-152162469 |
| 9851 | Hes3 | NM_008237.4 | chr4:152285971-152291662 |
| 9852 | Hes5 | NM_010419.4 | chr4:154960922-154962371 |
| 9853 | Hes6 | NM_019479.3 | chr1:91411482-91413222 |
| 9854 | Hes7 | NM_033041.4 | chr11:69120452-69123259 |
| 9855 | Hesx1 | NM_010420.2 | chr14:27000361-27002325 |
| 9856 | Hexa | NM_010421.4 | chr9:59539666-59565105 |
| 9857 | Hexb | NM_010422.2 | chr13:97176330-97198357 |
| 9858 | Hexdc | NM_001001333.2 | chr11:121204432-121222656 |
| 9859 | Hexim1 | NM_138753.2 | chr11:103116324-103119724 |
| 9860 | Hexim2 | NM_001130515.1 | chr11:103133352-103139908 |
| 9861 | Hey1 | NM_010423.2 | chr3:8663358-8667038 |
| 9862 | Hey2 | NM_013904.1 | chr10:30832358-30842783 |
| 9863 | Heyl | NM_013905.3 | chr4:123233555-123249870 |
| 9864 | Hfe | NM_010424.4 | chr13:23703840-23710811 |
| 9865 | Hfe2 | NM_027126.4 | chr3:96525184-96529216 |
| 9866 | Hfm1 | NM_001252516.1 | chr5:106901888-106925890 |
| 9867 | Hgd | NM_013547.3 | chr16:37580152-37632026 |
| 9868 | Hgf | NM_001289458.1 | chr5:16553494-16619439 |
| 9869 | Hgfac | NM_019447.3 | chr5:35041508-35048461 |
| 9870 | Hgs | NM_001159328.1 | chr11:120467634-120483984 |
| 9871 | Hgsnat | NM_029884.1 | chr8:25944458-25976744 |
| 9872 | Hhat | NM_144881.4 | chr1:192512827-192771219 |
| 9873 | Hhatl | NM_029095.2 | chr9:121784015-121792507 |
| 9874 | Hhex | NM_008245.3 | chr19:37434840-37440731 |
| 9875 | Hhip | NM_020259.4 | chr8:79965850-80058008 |
| 9876 | Hhipl1 | NM_001044380.1 | chr12:108306269-108328300 |
| 9877 | Hhipl2 | NM_030175.5 | chr1:183418469-183437053 |
| 9878 | Hhla1 | NM_001145096.1 | chr15:65922442-65976804 |
| 9879 | Hiat1 | NM_008246.2 | chr3:116631166-116662677 |
| 9880 | Hiatl1 | NM_001083901.1 | chr13:65065029-65112982 |
| 9881 | Hibadh | NM_145567.1 | chr6:52546229-52640300 |
| 9882 | Hibch | NM_146108.2 | chr1:52845037-52920986 |
| 9883 | Hic1 | NM_001098203.1 | chr11:75164564-75168152 |
| 9884 | Hic2 | NM_178922.3 | chr16:17233586-17263430 |
| 9885 | Hid1 | NM_175454.2 | chr11:115347708-115367719 |
| 9886 | Hif1a | NM_010431.2 | chr12:73907866-73947530 |
| 9887 | Hif1an | NM_176958.3 | chr19:44562853-44576274 |
| 9888 | Hif3a | NM_001162950.1 | chr7:17030992-17062427 |
| 9889 | Higd1a | NM_019814.4 | chr9:121848559-121858000 |
| 9890 | Higd1b | NM_080846.1 | chr11:102836295-102838039 |
| 9891 | Higd1c | NM_001002900.1 | chr15:100364584-100383955 |
| 9892 | Higd2a | NM_025933.3 | chr13:54590230-54591147 |
| 9893 | Hilpda | NM_001190461.1 | chr6:29272487-29275449 |

Fig.21 - 52

| | | | | | | | |
|---|---|---|---|---|---|---|---|
| 9894 | Hils1 | NM_018792.2 | chr11:94967592-94968456 | 9991 | Hmg20a | NM_025812.2 | chr9:56418845-56496936 |
| 9895 | Hinfp | NM_172162.3 | chr9:44295672-44305671 | 9992 | Hmg20b | NM_001163165.1 | chr10:81346045-81350426 |
| 9896 | Hint1 | NM_008248.2 | chr11:54866437-54870496 | 9993 | Hmga1 | NM_001025427.3 | chr17:27556573-27563672 |
| 9897 | Hint2 | NM_026871.1 | chr4:43654226-43656445 | 9994 | Hmga1-rs1 | NM_001166476.1 | chr17:27556652-27563671 |
| 9898 | Hint3 | NM_025798.3 | chr10:30608206-30618366 | 9995 | Hmga2 | NM_010441.2 | chr10:120361274-120476935 |
| 9899 | Hip1 | NM_146001.2 | chr5:135406518-135545122 | 9996 | Hmga2-ps1 | NR_037996.1 | chr1:176834623-176836133 |
| 9900 | Hip1r | NM_145070.3 | chr5:123973627-124003215 | 9997 | Hmgb1 | NM_010439.4 | chr5:149046701-149053057 |
| 9901 | Hipk1 | NM_010432.2 | chr3:103739814-103791275 | 9998 | Hmgb1-rs17 | NR_033589.1 | chr8:33484310-33485757 |
| 9902 | Hipk2 | NM_001136065.2 | chr6:38697839-38837311 | 9999 | Hmgb2 | NM_008252.3 | chr8:57511842-57515999 |
| 9903 | Hipk3 | NM_001145824.1 | chr2:104426481-104494489 | 10000 | Hmgb3 | NM_008253.4 | chrX:71555916-71560673 |
| 9904 | Hipk4 | NM_001033315.2 | chr7:27523260-27531174 | 10001 | Hmgb4 | NM_027036.3 | chr4:128260211-128260895 |
| 9905 | Hira | NM_010435.2 | chr16:18876749-18970308 | 10002 | Hmgcl | NM_008254.2 | chr4:135946452-135962617 |
| 9906 | Hirip3 | NM_172746.3 | chr7:126861971-126865122 | 10003 | Hmgcll1 | NM_173731.2 | chr9:76014976-76136350 |
| 9907 | Hist1h1a | NM_030609.3 | chr13:23763667-23764412 | 10004 | Hmgcr | NM_008255.2 | chr13:96648961-96670936 |
| 9908 | Hist1h1b | NM_020034.2 | chr13:21779831-21780625 | 10005 | Hmgcs1 | NM_001291439.1 | chr13:119690350-119708260 |
| 9909 | Hist1h1c | NM_015786.3 | chr13:23738806-23739531 | 10006 | Hmgcs2 | NM_008256.4 | chr3:98280430-98310738 |
| 9910 | Hist1h1d | NM_145713.4 | chr13:23555031-23555807 | 10007 | Hmgn1 | NM_008251.3 | chr16:96121587-96127725 |
| 9911 | Hist1h1e | NM_015787.4 | chr13:23621776-23622558 | 10008 | Hmgn2 | NM_016957.3 | chr4:133964738-133967991 |
| 9912 | Hist1h1t | NM_010377.3 | chr13:23695810-23696545 | 10009 | Hmgn3 | NM_026122.4 | chr9:83109941-83146607 |
| 9913 | Hist1h2aa | NM_175658.2 | chr13:23934461-23934913 | 10010 | Hmgn5 | NM_016710.2 | chrX:109004536-109013380 |
| 9914 | Hist1h2ab | NM_175660.3 | chr13:23751087-23751592 | 10011 | Hmgxb3 | NM_134134.2 | chr18:61131276-61177050 |
| 9915 | Hist1h2ac | NM_178189.5 | chr13:23683472-23683959 | 10012 | Hmgxb4 | NM_178017.1 | chr8:74993702-75031972 |
| 9916 | Hist1h2ad | NM_178188.4 | chr13:23574380-23574915 | 10013 | Hmha1 | NM_001142701.1 | chr10:80016671-80031471 |
| 9917 | Hist1h2ae | NM_178187.4 | chr13:23570662-23571220 | 10014 | Hmmr | NM_013552.2 | chr11:40701387-40733437 |
| 9918 | Hist1h2af | NM_175661.2 | chr13:23533910-23534378 | 10015 | Hmox1 | NM_010442.2 | chr8:75093617-75100593 |
| 9919 | Hist1h2ag | NM_178186.3 | chr13:22042477-22042949 | 10016 | Hmox2 | NM_001136066.2 | chr16:4726360-4766249 |
| 9920 | Hist1h2ah | NM_175659.2 | chr13:22035121-22035643 | 10017 | Hmx1 | NM_010445.2 | chr5:35389116-35392872 |
| 9921 | Hist1h2ai | NM_178182.2 | chr13:21716411-21716859 | 10018 | Hmx2 | NM_145998.3 | chr7:131554061-131556582 |
| 9922 | Hist1h2ak | NM_178183.2 | chr13:21753376-21753912 | 10019 | Hmx3 | NM_008257.3 | chr7:131542962-131544931 |
| 9923 | Hist1h2an | NM_178184.2 | chr13:21786771-21787218 | 10020 | Hn1 | NM_008258.1 | chr11:115497352-115514370 |
| 9924 | Hist1h2ao | NM_001177544.2 | chr13:21810366-21833575 | 10021 | Hn1l | NM_198937.2 | chr17:24942469-24960623 |
| 9925 | Hist1h2ap | NM_178185.2 | chr13:21810466-21833475 | 10022 | Hnf1a | NM_009327.3 | chr5:114948979-114971062 |
| 9926 | Hist1h2ba | NM_175663.2 | chr13:23933724-23934156 | 10023 | Hnf1b | NM_001291268.1 | chr11:83850868-83905917 |
| 9927 | Hist1h2bb | NM_175664.3 | chr13:23746733-23747223 | 10024 | Hnf4a | NM_008261.3 | chr2:163547154-163572907 |
| 9928 | Hist1h2bc | NM_001290380.1 | chr13:23684198-23684667 | 10025 | Hnf4aos | NR_027970.1 | chr2:163493803-163541721 |
| 9929 | Hist1h2be | NM_001177653.1 | chr13:23583669-23621094 | 10026 | Hnf4g | NM_013920.2 | chr3:3508029-3659800 |
| 9930 | Hist1h2bf | NM_178195.2 | chr13:23573759-23574190 | 10027 | Hnmt | NM_080462.2 | chr2:24002912-24049379 |
| 9931 | Hist1h2bg | NM_178196.4 | chr13:23571399-23571863 | 10028 | Hnrnpa0 | NM_029872.1 | chr13:58125878-58128556 |
| 9932 | Hist1h2bh | NM_178197.2 | chr13:23542922-23543444 | 10029 | Hnrnpa1 | NM_001039129.4 | chr15:103240396-103244962 |
| 9933 | Hist1h2bj | NM_178198.2 | chr13:22043229-22043658 | 10030 | Hnrnpa2b1 | NM_016806.3 | chr6:51460434-51469894 |
| 9934 | Hist1h2bk | NM_175665.2 | chr13:22035820-22036320 | 10031 | Hnrnpa3 | NM_053263.1 | chr2:75659258-75669407 |
| 9935 | Hist1h2bl | NM_178199.2 | chr13:21715712-21716143 | 10032 | Hnrnpab | NM_001048061.1 | chr11:51600099-51606881 |
| 9936 | Hist1h2bm | NM_178200.2 | chr13:21722043-21722526 | 10033 | Hnrnpc | NM_001170981.1 | chr14:52073379-52104028 |
| 9937 | Hist1h2bn | NM_178201.2 | chr13:21754122-21754553 | 10034 | Hnrnpd | NM_001077265.2 | chr5:99955934-99978938 |
| 9938 | Hist1h2bp | NM_001290466.1 | chr13:21787487-21787943 | 10035 | Hnrnpdl | NM_016690.4 | chr5:100033578-100039222 |
| 9939 | Hist1h2bq | NM_001097979.2 | chr13:21806411-21837530 | 10036 | Hnrnpf | NM_001166427.1 | chr6:117917293-117925622 |
| 9940 | Hist1h3a | NM_013550.3 | chr13:23761884-23762386 | 10037 | Hnrnph1 | NM_021510.2 | chr11:50377718-50386528 |
| 9941 | Hist1h3b | NM_178203.2 | chr13:23752379-23752840 | 10038 | Hnrnph2 | NM_019868.4 | chrX:134601178-134607057 |
| 9942 | Hist1h3c | NM_175653.2 | chr13:23745041-23745521 | 10039 | Hnrnph3 | NM_001079824.1 | chr10:63014663-63023849 |
| 9943 | Hist1h3d | NM_178204.2 | chr13:23575762-23576266 | 10040 | Hnrnpk | NM_025279.3 | chr13:58391131-58402512 |
| 9944 | Hist1h3e | NM_178205.2 | chr13:23561895-23562365 | 10041 | Hnrnpl | NM_177301.5 | chr7:28810889-28822266 |
| 9945 | Hist1h3f | NM_013548.4 | chr13:23544051-23544954 | 10042 | Hnrnpll | NM_144802.4 | chr17:80029486-80062268 |
| 9946 | Hist1h3g | NM_145073.2 | chr13:23535417-23535909 | 10043 | Hnrnpm | NM_001109913.1 | chr17:33646232-33685458 |
| 9947 | Hist1h3h | NM_178206.2 | chr13:21717627-21718115 | 10044 | Hnrnpr | NM_001277121.1 | chr4:136310941-136345979 |
| 9948 | Hist1h3i | NM_178207.2 | chr13:21782914-21783397 | 10045 | Hnrnpu | NM_016805.2 | chr1:178328299-178337784 |
| 9949 | Hist1h4a | NM_178192.2 | chr13:23760794-23761249 | 10046 | Hnrnpul1 | NM_144922.2 | chr7:25722038-25754720 |
| 9950 | Hist1h4b | NM_178193.2 | chr13:23756936-23757386 | 10047 | Hnrnpul2 | NM_001081196.1 | chr19:8819400-8834142 |
| 9951 | Hist1h4c | NM_178208.2 | chr13:23698083-23698458 | 10048 | Hoga1 | NM_026152.1 | chr19:42045850-42070939 |
| 9952 | Hist1h4d | NM_175654.2 | chr13:23581601-23581969 | 10049 | Homer1 | NM_001284189.1 | chr13:93304494-93405129 |
| 9953 | Hist1h4f | NM_175655.2 | chr13:23551285-23551643 | 10050 | Homer2 | NM_001164086.1 | chr7:81600480-81706925 |
| 9954 | Hist1h4h | NM_153173.4 | chr13:23531043-23531478 | 10051 | Homer3 | NM_001146153.1 | chr8:70282998-70294361 |
| 9955 | Hist1h4i | NM_175656.3 | chr13:22040959-22041362 | 10052 | Homez | NM_001177705.1 | chr14:54852736-54864158 |
| 9956 | Hist1h4j | NM_178210.2 | chr13:21735033-21735456 | 10053 | Hook1 | NM_030014.2 | chr4:95967378-96024274 |
| 9957 | Hist1h4k | NM_178211.2 | chr13:21750144-21750553 | 10054 | Hook2 | NM_001167991.1 | chr8:84990594-85003364 |
| 9958 | Hist1h4m | NM_001195421.1 | chr13:21811745-21812150 | 10055 | Hook3 | NM_207659.3 | chr8:26021420-26119224 |
| 9959 | Hist1h4n | NM_175657.2 | chr13:21811783-21832158 | 10056 | Hopx | NM_001159900.1 | chr5:77086985-77095267 |
| 9960 | Hist2h2aa1 | NM_013549.2 | chr3:96239778-96246052 | 10057 | Hormad1 | NM_001289532.1 | chr3:95559676-95587860 |
| 9961 | Hist2h2aa2 | NM_178212.3 | chr3:96239778-96246052 | 10058 | Hormad2 | NM_029458.1 | chr11:4346382-4441082 |
| 9962 | Hist2h2ab | NM_178213.4 | chr3:96219915-96220353 | 10059 | Hotair | NR_047528.1 | chr15:102944061-102947946 |
| 9963 | Hist2h2ac | NM_175662.2 | chr3:96220412-96220880 | 10060 | Hottip | NR_110441.1 | chr6:52262774-52267603 |
| 9964 | Hist2h2bb | NM_175666.2 | chr3:96269699-96270192 | 10061 | Hoxa1 | NM_010449.4 | chr6:52155366-52158317 |
| 9965 | Hist2h2be | NM_178214.4 | chr3:96221071-96223738 | 10062 | Hoxa10 | NM_001122950.2 | chr6:52231196-52240854 |
| 9966 | Hist2h3b | NM_178215.2 | chr3:96268653-96269155 | 10063 | Hoxa11 | NM_010450.3 | chr6:52242104-52245810 |
| 9967 | Hist2h3c1 | NM_178216.3 | chr3:96238478-96247348 | 10064 | Hoxa11os | NR_015348.1 | chr6:52245242-52249769 |
| 9968 | Hist2h3c2 | NM_054045.4 | chr3:96238478-96247348 | 10065 | Hoxa13 | NM_008264.1 | chr6:52258852-52260880 |
| 9969 | Hist2h4 | NM_033596.3 | chr3:96262933-96263317 | 10066 | Hoxa2 | NM_010451.2 | chr6:52162416-52164831 |
| 9970 | Hist3h2a | NM_178218.4 | chr11:58954684-58955192 | 10067 | Hoxa3 | NM_010452.3 | chr6:52169061-52213067 |
| 9971 | Hist3h2ba | NM_030082.4 | chr11:58948910-58949372 | 10068 | Hoxa4 | NM_008265.3 | chr6:52189686-52191703 |
| 9972 | Hist3h2bb-ps | NM_206882.1 | chr11:58954047-58954512 | 10069 | Hoxa5 | NM_010453.5 | chr6:52201753-52204587 |
| 9973 | Hist4h4 | NM_175652.3 | chr6:136803992-136804431 | 10070 | Hoxa6 | NM_010454.1 | chr6:52206364-52208624 |
| 9974 | Hivep1 | NM_007772.2 | chr13:42052020-42185026 | 10071 | Hoxa7 | NM_010455.2 | chr6:52215623-52218573 |
| 9975 | Hivep2 | NM_010437.2 | chr10:13966378-14151378 | 10072 | Hoxa9 | NM_001277238.1 | chr6:52223096-52227370 |
| 9976 | Hivep3 | NM_010657.3 | chr4:119814677-120135411 | 10073 | Hoxb1 | NM_008266.5 | chr11:96365757-96368253 |
| 9977 | Hjurp | NM_198652.2 | chr1:88258855-88277579 | 10074 | Hoxb13 | NM_008267.3 | chr11:96194360-96196599 |
| 9978 | Hk1 | NM_001146100.1 | chr10:62268854-62340421 | 10075 | Hoxb2 | NM_134032.2 | chr11:96351631-96354014 |
| 9979 | Hk1os | NR_038038.1 | chr10:62340606-62349866 | 10076 | Hoxb3 | NM_001079869.1 | chr11:96323125-96347930 |
| 9980 | Hk2 | NM_013820.3 | chr6:82725026-82774454 | 10077 | Hoxb4 | NM_010459.7 | chr11:96318266-96321638 |
| 9981 | Hk3 | NM_001033245.4 | chr13:55005984-55021385 | 10078 | Hoxb5 | NM_008268.2 | chr11:96303511-96306121 |
| 9982 | Hkdc1 | NM_145419.1 | chr10:62383136-62422457 | 10079 | Hoxb6 | NM_008269.1 | chr11:96299170-96301569 |
| 9983 | Hlcs | NM_139145.4 | chr16:94129305-94287856 | 10080 | Hoxb7 | NM_010460.2 | chr11:96286645-96290163 |
| 9984 | Hlf | NM_172563.3 | chr11:90336534-90390917 | 10081 | Hoxb8 | NM_010461.2 | chr11:96281904-96285325 |
| 9985 | Hltf | NM_009210.3 | chr3:20057810-20118491 | 10082 | Hoxb9 | NM_008270.2 | chr11:96271329-96276593 |
| 9986 | Hlx | NM_008250.2 | chr1:184727144-184732493 | 10083 | Hoxc10 | NM_010462.5 | chr15:102966795-102971897 |
| 9987 | Hmbox1 | NM_177338.5 | chr14:64822217-64949847 | 10084 | Hoxc11 | NM_001024842.1 | chr15:102954525-102956701 |
| 9988 | Hmbs | NM_001110251.1 | chr9:44336347-44342381 | 10085 | Hoxc12 | NM_010463.1 | chr15:102936852-102938517 |
| 9989 | Hmces | NM_173737.2 | chr6:87913975-87936613 | 10086 | Hoxc13 | NM_010464.2 | chr15:102921130-102928814 |
| 9990 | Hmcn1 | NM_001024720.3 | chr1:150562501-150993435 | 10087 | Hoxc4 | NM_013553.2 | chr15:103034394-103036852 |

Fig.21 - 53

| | | | |
|---|---|---|---|
| 10088 | Hoxc5 | NM_175730.5 | chr15:103014007-103017429 |
| 10089 | Hoxc6 | NM_010465.2 | chr15:103009561-103011881 |
| 10090 | Hoxc8 | NM_010466.2 | chr15:102990538-102994254 |
| 10091 | Hoxc9 | NM_008272.3 | chr15:102977031-102984444 |
| 10092 | Hoxd1 | NM_010467.2 | chr2:74762979-74765142 |
| 10093 | Hoxd10 | NM_013554.5 | chr2:74691890-74695106 |
| 10094 | Hoxd11 | NM_008273.2 | chr2:74682322-74687016 |
| 10095 | Hoxd12 | NM_008274.3 | chr2:74675012-74677705 |
| 10096 | Hoxd13 | NM_008275.3 | chr2:74668309-74671599 |
| 10097 | Hoxd3 | NM_010468.2 | chr2:74711992-74748271 |
| 10098 | Hoxd3os1 | NR_027899.1 | chr2:74710043-74716130 |
| 10099 | Hoxd4 | NM_010469.2 | chr2:74721977-74729159 |
| 10100 | Hoxd8 | NM_001290730.1 | chr2:74705488-74707932 |
| 10101 | Hoxd9 | NM_013555.4 | chr2:74697762-74700208 |
| 10102 | Hp | NM_017370.2 | chr8:109575127-109579172 |
| 10103 | Hp1bp3 | NM_001122896.2 | chr4:138216611-138244682 |
| 10104 | Hpca | NM_001130419.2 | chr4:129111569-129121747 |
| 10105 | Hpcal1 | NM_016677.4 | chr12:17690813-17791926 |
| 10106 | Hpcal4 | NM_174998.4 | chr4:123183503-123194699 |
| 10107 | Hpd | NM_008277.2 | chr5:123171806-123182686 |
| 10108 | Hpdl | NM_146256.3 | chr4:116819906-116821508 |
| 10109 | Hpgd | NM_008278.2 | chr8:56294551-56321046 |
| 10110 | Hpgds | NM_019455.4 | chr6:65117292-65144730 |
| 10111 | Hpn | NM_001110252.2 | chr7:31098724-31115326 |
| 10112 | Hprt | NM_013556.2 | chrX:52988077-53021660 |
| 10113 | Hps1 | NM_019424.2 | chr19:42755195-42779976 |
| 10114 | Hps3 | NM_001146323.1 | chr3:19995944-20035310 |
| 10115 | Hps4 | NM_138646.3 | chr5:112343094-112378424 |
| 10116 | Hps5 | NM_001005247.2 | chr7:46760465-46795881 |
| 10117 | Hps6 | NM_176785.3 | chr19:46003477-46006173 |
| 10118 | Hpse | NM_152803.4 | chr5:100679485-100719683 |
| 10119 | Hpse2 | NM_001081257.2 | chr19:42788594-43388355 |
| 10120 | Hpx | NM_017371.2 | chr7:105591610-105600116 |
| 10121 | Hr | NM_021877.3 | chr14:70554055-70573548 |
| 10122 | Hras | NM_001130443.1 | chr7:141189933-141194004 |
| 10123 | Hrasls | NM_013751.5 | chr16:29209694-29230530 |
| 10124 | Hrasls5 | NM_025731.2 | chr19:7612568-7639238 |
| 10125 | Hrc | NM_010473.2 | chr7:45335268-45338972 |
| 10126 | Hrct1 | NM_027511.1 | chr4:43727197-43728110 |
| 10127 | Hrg | NM_053176.2 | chr16:22951071-22961659 |
| 10128 | Hrh1 | NM_001252642.1 | chr6:114403665-114483296 |
| 10129 | Hrh2 | NM_001010973.2 | chr13:54192128-54222432 |
| 10130 | Hrh3 | NM_133849.3 | chr2:180099464-180104407 |
| 10131 | Hrh4 | NM_153087.2 | chr18:13006989-13022882 |
| 10132 | Hrk | NM_007545.2 | chr5:118169763-118189478 |
| 10133 | Hrnr | NM_133698.2 | chr3:93319748-93333572 |
| 10134 | Hrsp12 | NM_008287.3 | chr15:34484021-34495246 |
| 10135 | Hs1bp3 | NM_021429.3 | chr12:8313432-8343824 |
| 10136 | Hs2st1 | NM_011828.3 | chr3:144431106-144570216 |
| 10137 | Hs3st1 | NM_010474.2 | chr5:39613934-39644631 |
| 10138 | Hs3st2 | NM_001081327.1 | chr7:121392295-121501768 |
| 10139 | Hs3st3a1 | NM_178870.5 | chr11:64435331-64522835 |
| 10140 | Hs3st3b1 | NM_018805.2 | chr11:63884692-63922284 |
| 10141 | Hs3st4 | NM_001252072.1 | chr7:123983180-124398989 |
| 10142 | Hs3st5 | NM_001081208.2 | chr10:36506806-36834394 |
| 10143 | Hs3st6 | NM_001012402.1 | chr17:24753002-24758684 |
| 10144 | Hs6st1 | NM_015818.2 | chr1:36068399-36106446 |
| 10145 | Hs6st2 | NM_001077202.2 | chrX:51386636-51681602 |
| 10146 | Hs6st3 | NM_015820.3 | chr14:119138264-119869815 |
| 10147 | Hsbp1 | NM_024219.1 | chr8:119344537-119348929 |
| 10148 | Hsbp1l1 | NM_001136181.1 | chr18:80229755-80247102 |
| 10149 | Hscb | NM_153571.2 | chr5:110829069-110839777 |
| 10150 | Hsd11b1 | NM_001044751.1 | chr1:193221640-193242360 |
| 10151 | Hsd11b2 | NM_008289.2 | chr8:105518745-105523988 |
| 10152 | Hsd17b1 | NM_010475.1 | chr11:101078410-101080505 |
| 10153 | Hsd17b10 | NM_016763.2 | chrX:152001895-152004442 |
| 10154 | Hsd17b11 | NM_053262.3 | chr5:103989764-104021796 |
| 10155 | Hsd17b12 | NM_019657.4 | chr2:94032696-94157909 |
| 10156 | Hsd17b13 | NM_001163486.1 | chr5:103963464-103977388 |
| 10157 | Hsd17b14 | NM_025330.3 | chr7:45554927-45567321 |
| 10158 | Hsd17b2 | NM_008290.2 | chr8:117701945-117759029 |
| 10159 | Hsd17b3 | NM_008291.3 | chr13:64058273-64089201 |
| 10160 | Hsd17b4 | NM_008292.4 | chr18:50128200-50196270 |
| 10161 | Hsd17b6 | NM_013786.2 | chr10:127990935-128007508 |
| 10162 | Hsd17b7 | NM_010476.3 | chr1:169949536-169969205 |
| 10163 | Hsd3b1 | NM_008293.4 | chr3:98852193-98859794 |
| 10164 | Hsd3b2 | NM_153193.3 | chr3:98711106-98724543 |
| 10165 | Hsd3b3 | NM_001012306.2 | chr3:98741475-98762410 |
| 10166 | Hsd3b4 | NM_001111336.1 | chr3:98445683-98563943 |
| 10167 | Hsd3b5 | NM_008295.2 | chr3:98618635-98645534 |
| 10168 | Hsd3b6 | NM_013821.3 | chr3:98805503-98814443 |
| 10169 | Hsd3b7 | NM_001040684.1 | chr7:127800608-127803802 |
| 10170 | Hsdl1 | NM_175185.4 | chr8:119561977-119575200 |
| 10171 | Hsdl2 | NM_024255.3 | chr4:59581562-59618694 |
| 10172 | Hsf1 | NM_008296.2 | chr15:76477444-76500972 |
| 10173 | Hsf2 | NM_008297.3 | chr10:57486384-57513143 |
| 10174 | Hsf2bp | NM_028902.1 | chr17:31944768-32034508 |
| 10175 | Hsf3 | NM_172931.4 | chrX:96306177-96456294 |
| 10176 | Hsf4 | NM_001256042.1 | chr8:105269800-105275845 |
| 10177 | Hsf5 | NM_001045527.1 | chr11:87617163-87659542 |
| 10178 | Hsfy2 | NM_027661.2 | chr1:56636050-56637451 |
| 10179 | Hsh2d | NM_197944.1 | chr8:72189667-72200958 |
| 10180 | Hsp90aa1 | NM_010480.5 | chr12:110691035-110696395 |
| 10181 | Hsp90ab1 | NM_008302.3 | chr17:45567777-45573261 |
| 10182 | Hsp90b1 | NM_011631.1 | chr10:86690840-86705444 |
| 10183 | Hspa12a | NM_175199.3 | chr19:58795750-58860984 |
| 10184 | Hspa12b | NM_028306.3 | chr2:131127411-131145983 |
| 10185 | Hspa13 | NM_030201.3 | chr16:75755190-75766818 |
| 10186 | Hspa14 | NM_015765.2 | chr2:3488853-3512814 |
| 10187 | Hspa1a | NM_010479.2 | chr17:34969358-34972156 |
| 10188 | Hspa1b | NM_010478.2 | chr17:34956428-34959238 |
| 10189 | Hspa1l | NM_013558.2 | chr17:34972702-34979228 |
| 10190 | Hspa2 | NM_001002012.1 | chr12:76404175-76406936 |
| 10191 | Hspa4 | NM_008300.3 | chr11:53259813-53300479 |
| 10192 | Hspa4l | NM_011020.3 | chr3:40745612-40790365 |
| 10193 | Hspa5 | NM_001163434.1 | chr2:34772089-34776529 |
| 10194 | Hspa8 | NM_031165.4 | chr9:40801272-40805199 |
| 10195 | Hspa9 | NM_010481.2 | chr18:34937414-34954351 |
| 10196 | Hspb1 | NM_013560.2 | chr5:135887918-135889563 |
| 10197 | Hspb11 | NM_028394.2 | chr4:107253933-107279888 |
| 10198 | Hspb2 | NM_001164708.1 | chr9:50751071-50752354 |
| 10199 | Hspb3 | NM_019960.2 | chr13:113662895-113663676 |
| 10200 | Hspb6 | NM_001012401.2 | chr7:30553301-30555439 |
| 10201 | Hspb7 | NM_013868.4 | chr4:141420778-141425310 |
| 10202 | Hspb8 | NM_030704.3 | chr5:116408490-116422864 |
| 10203 | Hspb9 | NM_029307.1 | chr11:100713849-100714575 |
| 10204 | Hspbap1 | NM_175111.3 | chr16:35770385-35828462 |
| 10205 | Hspbp1 | NM_024172.3 | chr7:4660520-4684963 |
| 10206 | Hspd1 | NM_010477.4 | chr1:55077833-55087932 |
| 10207 | Hspe1 | NM_008303.4 | chr1:55088147-55091317 |
| 10208 | Hspg2 | NM_008305.3 | chr4:137468802-137570630 |
| 10209 | Hsph1 | NM_013559.2 | chr5:149616844-149636315 |
| 10210 | Htatip2 | NM_001146049.1 | chr7:49759105-49773999 |
| 10211 | Htatsf1 | NM_028242.2 | chrX:57053569-57067182 |
| 10212 | Htr1a | NM_008308.4 | chr13:105443692-105448133 |
| 10213 | Htr1b | NM_010482.1 | chr9:81631391-81632552 |
| 10214 | Htr1d | NM_001285482.1 | chr4:136423523-136444401 |
| 10215 | Htr1f | NM_008310.3 | chr16:64924728-65105784 |
| 10216 | Htr2a | NM_172812.2 | chr14:74640839-74706859 |
| 10217 | Htr2b | NM_008311.2 | chr1:86099036-86111970 |
| 10218 | Htr2c | NM_008312.4 | chrX:146962512-147197277 |
| 10219 | Htr3a | NM_001099644.1 | chr9:48899212-48911099 |
| 10220 | Htr3b | NM_020274.4 | chr9:48935007-48964990 |
| 10221 | Htr4 | NM_008313.4 | chr18:62324203-62467802 |
| 10222 | Htr5a | NM_008314.2 | chr5:27841946-27855086 |
| 10223 | Htr5b | NM_010483.3 | chr1:121509772-121528465 |
| 10224 | Htr6 | NM_021358.2 | chr4:139061408-139074789 |
| 10225 | Htr7 | NM_008315.2 | chr19:35958728-36057360 |
| 10226 | Htra1 | NM_019564.3 | chr7:130936202-130985658 |
| 10227 | Htra2 | NM_019752.3 | chr6:83051266-83054571 |
| 10228 | Htra3 | NM_001042615.2 | chr5:35661558-35679780 |
| 10229 | Htra4 | NM_001081187.3 | chr8:25024927-25038962 |
| 10230 | Htt | NM_010414.3 | chr5:34761739-34912534 |
| 10231 | Hunk | NM_015755.2 | chr16:90386396-90499553 |
| 10232 | Hus1 | NM_008316.5 | chr11:8993136-9011191 |
| 10233 | Hus1b | NM_153072.2 | chr13:30946575-30947761 |
| 10234 | Huwe1 | NM_021523.4 | chrX:151803281-151935417 |
| 10235 | Hvcn1 | NM_001042489.2 | chr5:122209728-122242297 |
| 10236 | Hyal1 | NM_008317.4 | chr9:107576951-107580133 |
| 10237 | Hyal2 | NM_010489.2 | chr9:107569162-107572778 |
| 10238 | Hyal3 | NM_178020.3 | chr9:107581295-107587359 |
| 10239 | Hyal4 | NM_029848.1 | chr6:24748366-24766519 |
| 10240 | Hyal5 | NM_028957.2 | chr6:24857996-24891958 |
| 10241 | Hyal6 | NM_028920.2 | chr6:24733244-24745452 |
| 10242 | Hydin | NM_172916.2 | chr8:110266976-110610253 |
| 10243 | Hyi | NM_026601.3 | chr4:118359998-118362744 |
| 10244 | Hykk | NM_177351.4 | chr9:54917289-54949924 |
| 10245 | Hyls1 | NM_029762.1 | chr9:35560820-35570069 |
| 10246 | Hyou1 | NM_021395.4 | chr9:44379489-44392369 |
| 10247 | Hypk | NM_026318.3 | chr2:121457087-121458440 |
| 10248 | I730028E13Rik | NR_045705.1 | chr9:61138514-61145259 |
| 10249 | I730030J21Rik | NR_045781.1 | chr15:100730504-100732737 |
| 10250 | I830012O16Rik | NM_001005858.3 | chr19:34607956-34613401 |
| 10251 | I830077J02Rik | NM_001033780.3 | chr3:105925890-105932664 |
| 10252 | Iah1 | NM_026347.3 | chr12:21316391-21323607 |
| 10253 | Iapp | NM_010491.2 | chr6:142298424-142303819 |
| 10254 | Iars | NM_172015.3 | chr13:49682129-49734267 |
| 10255 | Iars2 | NM_198653.2 | chr1:185286661-185329401 |
| 10256 | Iba57 | NM_001270791.1 | chr11:59161118-59163745 |
| 10257 | Ibsp | NM_008318.3 | chr5:104299286-104311472 |
| 10258 | Ibtk | NM_001081282.2 | chr9:85687359-85749334 |
| 10259 | Ica1 | NM_001252266.1 | chr6:8630526-8758458 |
| 10260 | Ica1l | NM_027407.3 | chr1:59989067-60043087 |
| 10261 | Icam1 | NM_010493.2 | chr9:21015959-21028796 |
| 10262 | Icam2 | NM_010494.1 | chr11:106377655-106382641 |
| 10263 | Icam4 | NM_023892.2 | chr9:21029372-21030531 |
| 10264 | Icam5 | NM_008319.2 | chr9:21032037-21039036 |
| 10265 | Ick | NM_001163780.1 | chr9:78109191-78172109 |
| 10266 | Icmt | NM_133788.2 | chr4:152297213-152307126 |
| 10267 | Icos | NM_017480.2 | chr1:60977913-61000321 |
| 10268 | Icosl | NM_015790.3 | chr10:78069367-78079525 |
| 10269 | Ict1 | NM_026729.1 | chr11:115403765-115410913 |
| 10270 | Id1 | NM_010495.3 | chr2:152736250-152737410 |
| 10271 | Id2 | NM_010496.3 | chr12:25093798-25096092 |
| 10272 | Id3 | NM_008321.2 | chr4:136143821-136145392 |
| 10273 | Id4 | NM_031166.2 | chr13:48261426-48264036 |
| 10274 | Ide | NM_031156.3 | chr19:37268740-37330613 |
| 10275 | Idh1 | NM_001111320.1 | chr1:65158615-65186479 |
| 10276 | Idh2 | NM_173011.2 | chr7:80094846-80115350 |
| 10277 | Idh3a | NM_029573.2 | chr9:54586510-54604662 |
| 10278 | Idh3b | NM_130884.4 | chr2:130279308-130284451 |
| 10279 | Idh3g | NM_008323.1 | chrX:73778962-73786897 |
| 10280 | Idi1 | NM_145360.2 | chr13:8885605-8892396 |
| 10281 | Idi2 | NM_177197.4 | chr13:8952862-8960935 |

Fig.21 - 54

| | | | | | | | |
|---|---|---|---|---|---|---|---|
| 10282 | Idnk | NM_001048060.2 | chr13:58157648-58164693 | 10379 | Igfbp7 | NM_001159518.1 | chr5:77349239-77408045 |
| 10283 | Ido1 | NM_008324.2 | chr8:24584132-24596952 | 10380 | Igfbpl1 | NM_018741.2 | chr4:45809506-45826827 |
| 10284 | Ido2 | NM_145949.2 | chr8:24531891-24576333 | 10381 | Igfl3 | NM_001003393.1 | chr7:18176493-18181862 |
| 10285 | Ids | NM_010498.3 | chrX:70343069-70365085 | 10382 | Igflr1 | NM_145580.2 | chr7:30565426-30567962 |
| 10286 | Idua | NM_008325.4 | chr5:108660330-108685446 | 10383 | Igfn1 | NM_177642.3 | chr1:135953577-136006342 |
| 10287 | Ier2 | NM_010499.4 | chr8:84661330-84662852 | 10384 | Ighmbp2 | NM_009212.2 | chr19:3259075-3283010 |
| 10288 | Ier3 | NM_133662.2 | chr17:35821712-35822911 | 10385 | Igip | NM_001267796.1 | chr18:36300100-36301478 |
| 10289 | Ier3ip1 | NM_025409.3 | chr18:76930026-76941614 | 10386 | Igj | NM_152839.3 | chr5:88519808-88527897 |
| 10290 | Ier5 | NM_010500.2 | chr1:155096366-155099636 | 10387 | Igll1 | NM_001190325.1 | chr16:16860670-16863985 |
| 10291 | Ier5l | NM_030244.3 | chr2:30472640-30474199 | 10388 | Iglon5 | NM_001164518.1 | chr7:43472906-43490023 |
| 10292 | Iffo1 | NM_001039669.3 | chr6:125145223-125161782 | 10389 | Igsf1 | NM_177591.4 | chrX:49782536-49797745 |
| 10293 | Iffo2 | NM_001205173.1 | chr4:139574719-139620382 | 10390 | Igsf10 | NM_001162884.1 | chr3:59316734-59344256 |
| 10294 | Ifi202b | NM_008327.2 | chr1:173962568-173982844 | 10391 | Igsf11 | NM_170599.2 | chr16:38902344-39027157 |
| 10295 | Ifi203 | NM_001302649.1 | chr1:173920400-173942847 | 10392 | Igsf21 | NM_198610.2 | chr4:140026851-140246811 |
| 10296 | Ifi204 | NM_008329.2 | chr1:173747293-173766919 | 10393 | Igsf23 | NM_027308.2 | chr7:19937304-19950743 |
| 10297 | Ifi205 | NM_172648.3 | chr1:174011997-174031755 | 10394 | Igsf3 | NM_207205.2 | chr3:101377124-101463060 |
| 10298 | Ifi27 | NM_026790.2 | chr12:103434188-103440245 | 10395 | Igsf5 | NM_001177886.1 | chr16:96361760-96422121 |
| 10299 | Ifi27l2a | NM_001281830.1 | chr12:103433872-103443680 | 10396 | Igsf6 | NM_030691.1 | chr7:121064066-121074530 |
| 10300 | Ifi27l2b | NM_145449.2 | chr12:103450897-103457223 | 10397 | Igsf8 | NM_080419.2 | chr1:172312366-172319843 |
| 10301 | Ifi30 | NM_023065.3 | chr8:70762772-70766663 | 10398 | Igsf9 | NM_001145800.1 | chr1:172482212-172498877 |
| 10302 | Ifi35 | NM_027320.4 | chr11:101448411-101458701 | 10399 | Igsf9b | NM_001033323.3 | chr9:27299227-27330135 |
| 10303 | Ifi44 | NM_133871.2 | chr3:151730922-151749959 | 10400 | Igtp | NM_018738.4 | chr11:58199555-58207592 |
| 10304 | Ifi44l | NM_031367.1 | chr3:151758736-151762891 | 10401 | Ihh | NM_010544.3 | chr1:74945314-74951672 |
| 10305 | Ifi47 | NM_001271676.1 | chr11:49087016-49096987 | 10402 | Iigp1 | NM_001146275.1 | chr18:60376028-60392629 |
| 10306 | Ifih1 | NM_001164477.1 | chr2:62595792-62646255 | 10403 | Ik | NM_011879.2 | chr18:36744655-36757639 |
| 10307 | Ifit1 | NM_008331.3 | chr19:34640888-34650009 | 10404 | Ikbip | NM_026166.2 | chr10:91083038-91098658 |
| 10308 | Ifit2 | NM_008332.3 | chr19:34550693-34576534 | 10405 | Ikbkap | NM_026079.3 | chr4:56749679-56802331 |
| 10309 | Ifit3 | NM_010501.2 | chr19:34583528-34588982 | 10406 | Ikbkb | NM_001159774.1 | chr8:22659204-22706583 |
| 10310 | Ifitm1 | NM_001112715.1 | chr7:140968075-140969827 | 10407 | Ikbke | NM_019777.3 | chr1:131254601-131279563 |
| 10311 | Ifitm10 | NM_177265.4 | chr7:142326109-142372259 | 10408 | Ikbkg | NM_001136067.2 | chrX:74424611-74453778 |
| 10312 | Ifitm2 | NM_030694.1 | chr7:140954838-140955961 | 10409 | Ikzf1 | NM_001025597.2 | chr11:11685924-11772926 |
| 10313 | Ifitm3 | NM_025378.2 | chr7:141009589-141010744 | 10410 | Ikzf2 | NM_011770.4 | chr1:69531209-69685960 |
| 10314 | Ifitm5 | NM_053088.2 | chr7:140948961-140950239 | 10411 | Ikzf3 | NM_011771.1 | chr11:98464692-98546031 |
| 10315 | Ifitm6 | NM_001033632.1 | chr7:141015811-141016892 | 10412 | Ikzf4 | NM_011772.2 | chr10:128632414-128645993 |
| 10316 | Ifitm7 | NM_001270718.1 | chr16:13981701-13986867 | 10413 | Ikzf5 | NM_175115.4 | chr7:131388648-131410478 |
| 10317 | Ifltd1 | NM_028742.2 | chr6:145397234-145434925 | 10414 | Il10 | NM_010548.2 | chr1:131019844-131024970 |
| 10318 | Ifna1 | NM_010502.2 | chr4:88850086-88850656 | 10415 | Il10ra | NM_008348.2 | chr9:45253838-45269146 |
| 10319 | Ifna11 | NM_008333.2 | chr4:88819918-88820565 | 10416 | Il10rb | NM_008349.5 | chr16:91406234-91425834 |
| 10320 | Ifna12 | NM_177361.2 | chr4:88602579-88603376 | 10417 | Il11 | NM_001290423.1 | chr7:4772377-4782857 |
| 10321 | Ifna13 | NM_177347.2 | chr4:88643640-88644459 | 10418 | Il11ra1 | NM_001163401.1 | chr4:41760442-41769473 |
| 10322 | Ifna14 | NM_206975.1 | chr4:88571228-88571798 | 10419 | Il11ra2 | NM_010550.3 | chr4:3083-42665763 |
| 10323 | Ifna15 | NM_206870.1 | chr4:88557672-88558245 | 10420 | Il12a | NM_001159424.2 | chr3:68690643-68698550 |
| 10324 | Ifna16 | NM_206867.1 | chr4:88676286-88676856 | 10421 | Il12b | NM_001303244.1 | chr11:44400063-44414677 |
| 10325 | Ifna2 | NM_010503.2 | chr4:88683206-88683779 | 10422 | Il12rb1 | NM_008353.2 | chr8:70808448-70821423 |
| 10326 | Ifna4 | NM_010504.2 | chr4:88841815-88842459 | 10423 | Il12rb2 | NM_008354.3 | chr6:67292017-67376137 |
| 10327 | Ifna5 | NM_010505.2 | chr4:88835524-88836094 | 10424 | Il13 | NM_008355.3 | chr11:53631322-53634702 |
| 10328 | Ifna6 | NM_206871.1 | chr4:88827415-88827985 | 10425 | Il13ra1 | NM_133990.5 | chrX:36112107-36171261 |
| 10329 | Ifna7 | NM_008334.3 | chr4:88816224-88816800 | 10426 | Il13ra2 | NM_008356.4 | chrX:147383477-147429192 |
| 10330 | Ifna9 | NM_010507.1 | chr4:88591812-88592385 | 10427 | Il15 | NM_001254747.1 | chr8:82331623-82402586 |
| 10331 | Ifnab | NM_008336.2 | chr4:88690598-88691268 | 10428 | Il15ra | NM_001271497.1 | chr2:11705292-11733985 |
| 10332 | Ifnar1 | NM_010508.2 | chr16:91485214-91510522 | 10429 | Il16 | NM_010551.3 | chr7:83643062-83735490 |
| 10333 | Ifnar2 | NM_001110498.1 | chr16:91372782-91394043 | 10430 | Il17a | NM_010552.3 | chr1:20730904-20734496 |
| 10334 | Ifnb1 | NM_010510.1 | chr4:88522024-88522794 | 10431 | Il17b | NM_019508.1 | chr18:61687934-61692537 |
| 10335 | Ifne | NM_177348.2 | chr4:88879537-88880201 | 10432 | Il17c | NM_145834.3 | chr8:122422084-122423639 |
| 10336 | Ifng | NM_008337.4 | chr10:118441045-118445894 | 10433 | Il17d | NM_145837.3 | chr14:57524828-57543166 |
| 10337 | Ifngr1 | NM_010511.2 | chr10:19591957-19610225 | 10434 | Il17f | NM_145856.2 | chr1:20777145-20784270 |
| 10338 | Ifngr2 | NM_008338.3 | chr16:91547093-91564007 | 10435 | Il17ra | NM_008359.2 | chr6:120463196-120483727 |
| 10339 | Ifnk | NM_199157.2 | chr4:35152054-35154019 | 10436 | Il17rb | NM_019583.3 | chr14:29996167-30008896 |
| 10340 | Ifnl2 | NM_001024673.2 | chr7:28508860-28510360 | 10437 | Il17rc | NM_134159.4 | chr6:113471454-113483140 |
| 10341 | Ifnl3 | NM_177396.1 | chr7:28522835-28524322 | 10438 | Il17rd | NM_134437.3 | chr14:27039000-27107286 |
| 10342 | Ifnlr1 | NM_174851.3 | chr4:135686456-135708180 | 10439 | Il17re | NM_001034029.1 | chr6:113458894-113470143 |
| 10343 | Ifnz | NM_197889.2 | chr4:88782130-88783592 | 10440 | Il18 | NM_008360.1 | chr9:50565367-50581837 |
| 10344 | Ifrd1 | NM_013562.2 | chr12:40203128-40223189 | 10441 | Il18bp | NM_010531.1 | chr7:102015076-102018155 |
| 10345 | Ifrd2 | NM_025903.2 | chr9:107587717-107593038 | 10442 | Il18r1 | NM_001161842.1 | chr1:40465551-40491578 |
| 10346 | Ift122 | NM_001167763.1 | chr6:115853527-115926699 | 10443 | Il18rap | NM_010553.4 | chr1:40515361-40551705 |
| 10347 | Ift140 | NM_134126.3 | chr17:25016085-25099497 | 10444 | Il19 | NM_001009940.1 | chr1:130932655-130939241 |
| 10348 | Ift172 | NM_026298.5 | chr5:31253278-31291114 | 10445 | Il1a | NM_010554.4 | chr2:129299609-129309972 |
| 10349 | Ift20 | NM_018854.4 | chr11:78536435-78541473 | 10446 | Il1b | NM_008361.4 | chr2:129364577-129371139 |
| 10350 | Ift22 | NM_026073.3 | chr5:136908149-136913244 | 10447 | Il1bos | NR_015474.1 | chr2:129369901-129375733 |
| 10351 | Ift27 | NM_025931.2 | chr15:78159462-78174108 | 10448 | Il1f10 | NM_153077.2 | chr2:24291195-24293820 |
| 10352 | Ift43 | NM_001199843.1 | chr12:86082560-86162459 | 10449 | Il1f5 | NM_001146087.1 | chr2:24276953-24282432 |
| 10353 | Ift46 | NM_023831.3 | chr9:44773008-44792714 | 10450 | Il1f6 | NM_019450.3 | chr2:24215416-24225701 |
| 10354 | Ift52 | NM_172150.4 | chr2:163017471-163046135 | 10451 | Il1f8 | NM_027163.4 | chr2:24153171-24160103 |
| 10355 | Ift57 | NM_028680.3 | chr16:49699293-49765126 | 10452 | Il1f9 | NM_153511.3 | chr2:24186475-24193567 |
| 10356 | Ift74 | NM_001290568.1 | chr4:94614490-94664698 | 10453 | Il1r1 | NM_001123382.1 | chr1:40266585-40316177 |
| 10357 | Ift80 | NM_026641.2 | chr3:68892498-69004570 | 10454 | Il1r2 | NM_010555.4 | chr1:40084767-40125226 |
| 10358 | Ift81 | NM_009879.3 | chr5:122550203-122614518 | 10455 | Il1rap | NM_001159317.1 | chr16:26581704-26725147 |
| 10359 | Ift88 | NM_009376.2 | chr14:57424070-57517936 | 10456 | Il1rapl1 | NM_001160403.1 | chrX:86747241-88115623 |
| 10360 | Igbp1 | NM_008784 | chrX:100494290-100516125 | 10457 | Il1rapl2 | NM_030688.2 | chrX:137570753-138849077 |
| 10361 | Igbp1b | NM_015777.2 | chr6:138657091-138658444 | 10458 | Il1rl1 | NM_001025602.3 | chr1:40439628-40465414 |
| 10362 | Igdcc3 | NM_008988.2 | chr9:65141188-65185870 | 10459 | Il1rl2 | NM_133193.3 | chr1:40324626-40365471 |
| 10363 | Igdcc4 | NM_001290315.1 | chr9:65101485-65137947 | 10460 | Il1rn | NM_001039701.3 | chr2:24345370-24351491 |
| 10364 | Igf1 | NM_001111274.1 | chr10:87861110-87937047 | 10461 | Il2 | NM_008366.3 | chr3:37120715-37125954 |
| 10365 | Igf1r | NM_010513.2 | chr7:67952256-68233667 | 10462 | Il20 | NM_021380.2 | chr1:130906984-130911296 |
| 10366 | Igf2 | NM_001122736.2 | chr7:142650767-142661271 | 10463 | Il20ra | NM_172786.2 | chr10:19712586-19760053 |
| 10367 | Igf2bp1 | NM_009951.4 | chr11:95957163-96005944 | 10464 | Il20rb | NM_001033543.1 | chr9:100457718-100486473 |
| 10368 | Igf2bp2 | NM_183029.2 | chr16:22059008-22163299 | 10465 | Il21 | NM_001291041.1 | chr3:37222758-37232636 |
| 10369 | Igf2bp3 | NM_023670.3 | chr6:49085217-49214954 | 10466 | Il21r | NM_021887.2 | chr7:125603428-125633570 |
| 10370 | Igf2os | NR_002855.2 | chr7:142659692-142670356 | 10467 | Il22 | NM_016971.2 | chr10:118204963-118210046 |
| 10371 | Igf2r | NM_010515.2 | chr17:12682405-12769706 | 10468 | Il22ra1 | NM_178257.2 | chr4:135728158-135755383 |
| 10372 | Igfals | NM_008340.3 | chr17:24878769-24882008 | 10469 | Il22ra2 | NM_178258.5 | chr10:19622027-19634681 |
| 10373 | Igfbp1 | NM_008341.4 | chr11:7197786-7202546 | 10470 | Il23a | NM_031252.2 | chr10:128296139-128298084 |
| 10374 | Igfbp2 | NM_008342.3 | chr1:72824479-72852471 | 10471 | Il23r | NM_144548.1 | chr6:67422931-67491855 |
| 10375 | Igfbp3 | NM_008343.2 | chr11:7206090-7213923 | 10472 | Il24 | NM_053095.2 | chr1:130882073-130887411 |
| 10376 | Igfbp4 | NM_010517.3 | chr11:99041259-99052643 | 10473 | Il25 | NM_080729.3 | chr14:54932694-54935836 |
| 10377 | Igfbp5 | NM_010518.2 | chr1:72858064-72874865 | 10474 | Il27 | NM_145636.1 | chr7:126589294-126594910 |
| 10378 | Igfbp6 | NM_008344.3 | chr15:102144185-102149512 | 10475 | Il27ra | NM_016671.3 | chr8:84030285-84042575 |

Fig.21 - 55

| | | | | | | | |
|---|---|---|---|---|---|---|---|
| 10476 | Il2ra | NM_008367.3 | chr2:11642791-11693194 | 10573 | Ints8 | NM_001159595.1 | chr4:11202927-11254259 |
| 10477 | Il2rb | NM_008368.4 | chr15:78480548-78495066 | 10574 | Ints9 | NM_001253731.1 | chr14:64950044-65039835 |
| 10478 | Il2rg | NM_013563.4 | chrX:101264384-101268255 | 10575 | Intu | NM_175515.5 | chr3:40540766-40704774 |
| 10479 | Il3 | NM_010556.4 | chr11:54265084-54267279 | 10576 | Invs | NM_001281977.1 | chr4:48279759-48431953 |
| 10480 | Il31 | NM_029594.1 | chr5:123480152-123482101 | 10577 | Ip6k1 | NM_013785.2 | chr9:108002647-108048782 |
| 10481 | Il31ra | NM_139299.2 | chr13:112522806-112580662 | 10578 | Ip6k2 | NM_029634.2 | chr9:108795993-108806333 |
| 10482 | Il33 | NM_001164724.1 | chr19:29945789-29960715 | 10579 | Ip6k3 | NM_173027.2 | chr17:27143970-27167764 |
| 10483 | Il34 | NM_001135100.2 | chr8:110741828-110805924 | 10580 | Ipcef1 | NM_001033391.2 | chr10:6911542-7023185 |
| 10484 | Il3ra | NM_008369.1 | chr14:14346494-14355490 | 10581 | Ipmk | NM_027184.1 | chr10:71347792-71385885 |
| 10485 | Il4 | NM_021283.2 | chr11:53612460-53618665 | 10582 | Ipo11 | NM_029665.3 | chr13:106794438-106936915 |
| 10486 | Il4i1 | NM_010215.3 | chr7:44836287-44840809 | 10583 | Ipo13 | NM_146152.3 | chr4:117894492-117914999 |
| 10487 | Il4ra | NM_001008700.3 | chr7:125552281-125579474 | 10584 | Ipo4 | NM_024267.6 | chr14:55625628-55635678 |
| 10488 | Il5 | NM_010558.1 | chr11:53720793-53725103 | 10585 | Ipo5 | NM_023579.4 | chr14:120911193-120948044 |
| 10489 | Il5ra | NM_008370.2 | chr6:106710378-106749037 | 10586 | Ipo7 | NM_181517.3 | chr7:110018424-110055114 |
| 10490 | Il6 | NM_031168.2 | chr5:30013113-30019975 | 10587 | Ipo8 | NM_001081113.1 | chr6:148770683-148831467 |
| 10491 | Il6ra | NM_010559.3 | chr3:89869323-89913196 | 10588 | Ipo9 | NM_153774.1 | chr1:135382314-135430491 |
| 10492 | Il6st | NM_010560.3 | chr13:112464069-112506860 | 10589 | Ipp | NM_008389.3 | chr4:116507548-116538235 |
| 10493 | Il7 | NM_008371.5 | chr3:7572027-7613760 | 10590 | Ippk | NM_001276399.1 | chr13:49421613-49463060 |
| 10494 | Il7r | NM_008372.4 | chr15:9506158-9529876 | 10591 | Ipw | NR_015351.1 | chr7:59619157-59678878 |
| 10495 | Il9 | NM_008373.1 | chr13:56479276-56482246 | 10592 | Iqca | NM_029122.2 | chr1:90042131-90153401 |
| 10496 | Il9r | NM_001134458.1 | chr11:32188996-32200279 | 10593 | Iqcb1 | NM_177128.4 | chr16:36828399-36872715 |
| 10497 | Ildr1 | NM_001285788.1 | chr16:36693977-36726804 | 10594 | Iqcc | NM_198026.2 | chr4:129615616-129619093 |
| 10498 | Ildr2 | NM_001164528.1 | chr1:166254138-166316832 | 10595 | Iqcd | NM_029408.2 | chr5:120589022-120607113 |
| 10499 | Ilf2 | NM_026374.3 | chr3:90476200-90488379 | 10596 | Iqce | NM_028833.3 | chr5:140663504-140702378 |
| 10500 | Ilf3 | NM_001042707.2 | chr9:21367870-21405363 | 10597 | Iqcf1 | NM_001146701.1 | chr9:106499966-106502250 |
| 10501 | Ilk | NM_001161724.1 | chr7:105736589-105742925 | 10598 | Iqcf3 | NM_026645.3 | chr9:106543388-106561625 |
| 10502 | Ilkap | NM_023343.2 | chr1:91375830-91398783 | 10599 | Iqcf4 | NM_026090.2 | chr9:106568318-106570967 |
| 10503 | Iltifb | NM_054079.2 | chr10:118289629-118295038 | 10600 | Iqcf5 | NM_029300.1 | chr9:106514572-106516003 |
| 10504 | Ilvbl | NM_173751.4 | chr10:78574499-78584502 | 10601 | Iqcf6 | NM_001101628.1 | chr9:106626581-106627612 |
| 10505 | Immp1l | NM_028260.2 | chr2:105904637-105965558 | 10602 | Iqcg | NM_178378.3 | chr16:33014269-33056186 |
| 10506 | Immp2l | NM_053122.4 | chr12:41024089-41955588 | 10603 | Iqch | NM_030068.2 | chr9:63421448-63602493 |
| 10507 | Immt | NM_001253681.1 | chr6:71831319-71875266 | 10604 | Iqcj | NM_177585.3 | chr3:67892219-68056593 |
| 10508 | Imp3 | NM_133976.2 | chr9:56937499-56938398 | 10605 | Iqck | NM_001081446.1 | chr7:118855774-118972652 |
| 10509 | Imp4 | NM_178601.3 | chr1:34439828-34445747 | 10606 | Iqgap1 | NM_016721.2 | chr7:80711582-80803331 |
| 10510 | Impa1 | NM_018864.6 | chr3:10311955-10331439 | 10607 | Iqgap2 | NM_027711.1 | chr13:95627176-95891922 |
| 10511 | Impa2 | NM_053261.2 | chr18:67289222-67318841 | 10608 | Iqgap3 | NM_001033484.1 | chr3:88082050-88121048 |
| 10512 | Impact | NM_008378.2 | chr18:12972251-12992948 | 10609 | Iqsec1 | NM_001134383.1 | chr6:90659597-90716529 |
| 10513 | Impad1 | NM_177730.3 | chr4:4764350-4793306 | 10610 | Iqsec2 | NM_001005475.2 | chrX:152179008-152225237 |
| 10514 | Impdh1 | NM_011829.3 | chr6:29200436-29212297 | 10611 | Iqsec3 | NM_001033354.3 | chr6:121372927-121473678 |
| 10515 | Impdh2 | NM_011830.3 | chr9:108560500-108565566 | 10612 | Iqub | NM_172535.3 | chr6:24444864-24515067 |
| 10516 | Impg1 | NM_022016.3 | chr9:80313329-80465438 | 10613 | Irak1 | NM_001177973.1 | chrX:74013913-74023921 |
| 10517 | Impg2 | NM_174876.3 | chr16:56204312-56273753 | 10614 | Irak1bp1 | NM_001168240.1 | chr9:82829805-82847688 |
| 10518 | Ina | NM_146100.4 | chr19:47014697-47024346 | 10615 | Irak2 | NM_001113553.1 | chr6:113638466-113695027 |
| 10519 | Inadl | NM_001005784.1 | chr4:98395825-98497633 | 10616 | Irak3 | NM_028679.3 | chr10:120141653-120201537 |
| 10520 | Inca1 | NM_001252482.1 | chr11:70688360-70700155 | 10617 | Irak4 | NM_029926.5 | chr15:94543659-94568316 |
| 10521 | Incenp | NM_016692.3 | chr19:9872296-9899533 | 10618 | Ireb2 | NM_022655.3 | chr9:54863754-54912534 |
| 10522 | Inf2 | NM_198411.2 | chr12:112588783-112615557 | 10619 | Irf1 | NM_001159393.1 | chr11:53770013-53778374 |
| 10523 | Ing1 | NM_011919.5 | chr8:11556456-11563251 | 10620 | Irf2 | NM_008391.4 | chr8:46739744-46847458 |
| 10524 | Ing2 | NM_023503.3 | chr8:47667177-47675159 | 10621 | Irf2bp1 | NM_178757.3 | chr7:19004064-19006763 |
| 10525 | Ing3 | NM_023626.4 | chr6:21949614-21976037 | 10622 | Irf2bp2 | NM_001164598.1 | chr8:126588295-126593436 |
| 10526 | Ing4 | NM_133345.2 | chr6:125039847-125049264 | 10623 | Irf2bpl | NM_145836.2 | chr12:86880702-86884814 |
| 10527 | Ing5 | NM_025454.2 | chr1:93803964-93822100 | 10624 | Irf3 | NM_016849.4 | chr7:44997647-45002848 |
| 10528 | Inha | NM_010564.4 | chr1:75507076-75510354 | 10625 | Irf4 | NM_013674.1 | chr13:30749257-30766927 |
| 10529 | Inhba | NM_008380.1 | chr13:16014474-16027211 | 10626 | Irf5 | NM_001252382.1 | chr6:29529281-29537320 |
| 10530 | Inhbb | NM_008381.3 | chr1:119415464-119422248 | 10627 | Irf6 | NM_016851.2 | chr1:193153111-193172035 |
| 10531 | Inhbc | NM_010565.3 | chr10:127356325-127370544 | 10628 | Irf7 | NM_001252600.1 | chr7:141263182-141266424 |
| 10532 | Inhbe | NM_008382.2 | chr10:127349401-127351772 | 10629 | Irf8 | NM_008320.4 | chr8:120736357-120756692 |
| 10533 | Inip | NM_001013577.1 | chr4:59769646-59783855 | 10630 | Irf9 | NM_001159417.1 | chr14:55603984-55610030 |
| 10534 | Inmt | NM_009349.3 | chr6:55170626-55174990 | 10631 | Irg1 | NM_008392.1 | chr14:103047011-103056573 |
| 10535 | Ino80 | NM_026574.3 | chr2:119373041-119477629 | 10632 | Irgc1 | NM_199013.2 | chr7:24431925-24445682 |
| 10536 | Ino80b | NM_023547.1 | chr6:83121827-83125029 | 10633 | Irgm1 | NM_008326.1 | chr11:48865248-48871346 |
| 10537 | Ino80c | NM_172625.2 | chr18:24104760-24121819 | 10634 | Irgm2 | NM_019440.3 | chr11:58214976-58222783 |
| 10538 | Ino80d | NM_001081436.2 | chr1:63047800-63114267 | 10635 | Irgq | NM_153134.3 | chr7:24530647-24538600 |
| 10539 | Ino80dos | NR_045914.1 | chr1:63114739-63158295 | 10636 | Irs1 | NM_010570.4 | chr1:82233104-82291439 |
| 10540 | Ino80e | NM_153580.1 | chr7:126852432-126861463 | 10637 | Irs2 | NM_001081212.1 | chr8:10986963-11008430 |
| 10541 | Inpp1 | NM_008384.2 | chr1:52789419-52817688 | 10638 | Irs3 | NM_010571.3 | chr5:137643031-137645714 |
| 10542 | Inpp4a | NM_001290797.1 | chr1:37299837-37410740 | 10639 | Irs4 | NM_010572.2 | chrX:141710997-141725217 |
| 10543 | Inpp4b | NM_001024617.3 | chr8:81342561-82122570 | 10640 | Irx1 | NM_010573.2 | chr13:71958231-71963723 |
| 10544 | Inpp5a | NM_001127363.1 | chr7:139389108-139579652 | 10641 | Irx2 | NM_010574.2 | chr13:72628977-72634194 |
| 10545 | Inpp5b | NM_008385.4 | chr4:124741849-124801511 | 10642 | Irx3 | NM_001253822.1 | chr8:91798510-91801654 |
| 10546 | Inpp5d | NM_001110192.2 | chr1:87620311-87720510 | 10643 | Irx4 | NM_018885.2 | chr13:73260496-73269620 |
| 10547 | Inpp5e | NM_001290437.1 | chr2:26396248-26409127 | 10644 | Irx5 | NM_018826.2 | chr8:92357795-92361456 |
| 10548 | Inpp5f | NM_178641.5 | chr7:128611364-128696436 | 10645 | Irx6 | NM_022428.3 | chr8:92674288-92680956 |
| 10549 | Inpp5j | NM_172439.3 | chr11:3494271-3504821 | 10646 | Isca1 | NM_026921.4 | chr13:59755414-59769789 |
| 10550 | Inpp5k | NM_008916.2 | chr11:75631019-75648865 | 10647 | Isca2 | NM_028863.1 | chr12:84773269-84775089 |
| 10551 | Inppl1 | NM_001122739.1 | chr7:101822631-101837824 | 10648 | Iscu | NM_025526.4 | chr5:113772811-113778282 |
| 10552 | Ins1 | NM_008386.4 | chr19:52264296-52265009 | 10649 | Isg15 | NM_015783.3 | chr4:156199423-156200818 |
| 10553 | Ins2 | NM_001185083.2 | chr7:142678655-142679726 | 10650 | Isg20 | NM_001113527.1 | chr7:78914234-78920396 |
| 10554 | Insc | NM_173767.3 | chr7:114745765-114850380 | 10651 | Isg20l2 | NM_177663.4 | chr3:87930313-87940686 |
| 10555 | Insig1 | NM_153526.5 | chr5:28071411-28078662 | 10652 | Isl1 | NM_021459.4 | chr13:116298269-116309688 |
| 10556 | Insig2 | NM_001271531.1 | chr1:121304352-121327678 | 10653 | Isl2 | NM_027397.3 | chr9:55541148-55546178 |
| 10557 | Insl3 | NM_013564.7 | chr8:71689251-71690577 | 10654 | Islr | NM_001195431.1 | chr9:58156263-58158554 |
| 10558 | Insl5 | NM_001290648.1 | chr4:103017872-103026842 | 10655 | Islr2 | NM_001161535.1 | chr9:58196296-58201715 |
| 10559 | Insl6 | NM_013754.1 | chr19:29321353-29325318 | 10656 | Ism1 | NM_001276489.1 | chr2:139678177-139758581 |
| 10560 | Insm1 | NM_016889.3 | chr2:146221996-146225018 | 10657 | Ism2 | NM_001290302.1 | chr12:87278637-87299705 |
| 10561 | Insm2 | NM_020287.2 | chr12:55598916-55602017 | 10658 | Isoc1 | NM_025478.3 | chr18:58659481-58679570 |
| 10562 | Insr | NM_010568.2 | chr8:3150921-3279617 | 10659 | Isoc2a | NM_001101598.1 | chr7:4877052-4895716 |
| 10563 | Insrr | NM_011832.2 | chr3:87796950-87816101 | 10660 | Isoc2b | NM_026158.2 | chr7:4844959-4866179 |
| 10564 | Ints1 | NM_026748.2 | chr5:139751281-139775678 | 10661 | Ispd | NM_001289502.1 | chr12:36381449-36689503 |
| 10565 | Ints10 | NM_027590.4 | chr8:68793928-68829410 | 10662 | Ist1 | NM_028018.2 | chr8:109671320-109693294 |
| 10566 | Ints12 | NM_027927.3 | chr3:133091952-133110985 | 10663 | Isx | NM_027837.3 | chr8:74873173-74893506 |
| 10567 | Ints2 | NM_027421.2 | chr11:86210682-86257568 | 10664 | Isy1 | NM_133934.4 | chr6:87818447-87838759 |
| 10568 | Ints3 | NM_145540.3 | chr3:90391379-90433636 | 10665 | Isyna1 | NM_023627.1 | chr8:70594480-70597290 |
| 10569 | Ints4 | NM_027256.2 | chr7:97480955-97541398 | 10666 | Itch | NM_001243712.1 | chr2:155133480-155226855 |
| 10570 | Ints5 | NM_176843.3 | chr19:8892986-8897890 | 10667 | Itfg1 | NM_028007.3 | chr8:85717556-85840949 |
| 10571 | Ints6 | NM_008715.2 | chr14:62676324-62761112 | 10668 | Itfg2 | NM_133927.1 | chr6:128409443-128424910 |
| 10572 | Ints7 | NM_178632.6 | chr1:191575635-191623690 | 10669 | Itfg3 | NM_001206335.1 | chr17:26212691-26244242 |

Fig.21 - 56

| | | | | | | | |
|---|---|---|---|---|---|---|---|
| 10670 | Itga1 | NM_001033228.3 | chr13:114958080-115101964 | 10767 | Jsrp1 | NM_028001.3 | chr10:80808495-80813498 |
| 10671 | Itga10 | NM_001302471.1 | chr3:96645584-96664519 | 10768 | Jtb | NM_206924.2 | chr3:90231596-90235840 |
| 10672 | Itga11 | NM_176922.5 | chr9:62677854-62783979 | 10769 | Jun | NM_010591.2 | chr4:95049035-95052222 |
| 10673 | Itga2 | NM_008396.2 | chr13:114835911-114932041 | 10770 | Junb | NM_008416.3 | chr8:84976908-84978748 |
| 10674 | Itga2b | NM_010575.2 | chr11:102453296-102469883 | 10771 | Jund | NM_001286944.1 | chr8:70697738-70700616 |
| 10675 | Itga3 | NM_013565.3 | chr11:95044474-95076714 | 10772 | Jup | NM_010593.2 | chr11:100368855-100397790 |
| 10676 | Itga4 | NM_010576.3 | chr2:79255425-79333114 | 10773 | Kalrn | NM_001164268.1 | chr16:34152024-34514027 |
| 10677 | Itga5 | NM_010577.4 | chr15:103344285-103366791 | 10774 | Kank1 | NM_181404.5 | chr19:25237201-25434496 |
| 10678 | Itga6 | NM_001277970.1 | chr2:71786938-71858427 | 10775 | Kank2 | NM_145611.4 | chr9:21766772-21798546 |
| 10679 | Itga7 | NM_008398.2 | chr10:128933812-128958284 | 10776 | Kank3 | NM_030697.2 | chr17:33810522-33822914 |
| 10680 | Itga8 | NM_001001309.2 | chr2:12106659-12301920 | 10777 | Kank4 | NM_172872.3 | chr4:98754891-98817537 |
| 10681 | Itga9 | NM_001113514.1 | chr9:118711691-118901003 | 10778 | Kank4os | NR_040437.1 | chr4:98793767-98799037 |
| 10682 | Itgad | NM_001029872.3 | chr7:128173945-128205503 | 10779 | Kansl1 | NM_001081045.1 | chr11:104333228-104442291 |
| 10683 | Itgae | NM_008399.2 | chr11:73090594-73147449 | 10780 | Kansl1l | NM_001122738.1 | chr1:66719242-66817595 |
| 10684 | Itgal | NM_001253872.1 | chr7:127296259-127335137 | 10781 | Kansl2 | NM_001289437.1 | chr15:98517657-98534269 |
| 10685 | Itgam | NM_001082960.1 | chr7:128062639-128118491 | 10782 | Kansl3 | NM_172652.3 | chr1:36335729-36369181 |
| 10686 | Itgav | NM_008402.3 | chr2:83724396-83806917 | 10783 | Kap | NM_010594.2 | chr6:133849854-133853667 |
| 10687 | Itgax | NM_021334.2 | chr7:128129567-128150657 | 10784 | Kars | NM_001130868.2 | chr8:111993438-112011354 |
| 10688 | Itgb1 | NM_010578.2 | chr8:128685653-128733579 | 10785 | Kat2a | NM_001038010.2 | chr11:100704745-100712467 |
| 10689 | Itgb1bp1 | NM_008403.4 | chr12:21269805-21286237 | 10786 | Kat2b | NM_001190846.1 | chr17:53584125-53672721 |
| 10690 | Itgb1bp2 | NM_013712.2 | chrX:101449108-101453541 | 10787 | Kat5 | NM_001199247.1 | chr19:5603013-5610094 |
| 10691 | Itgb2 | NM_008404.4 | chr10:77530347-77565674 | 10788 | Kat6a | NM_001081149.1 | chr8:22859538-22943262 |
| 10692 | Itgb2l | NM_008405.3 | chr16:96422297-96443614 | 10789 | Kat6b | NM_001205241.1 | chr14:21499769-21672478 |
| 10693 | Itgb3 | NM_016780.2 | chr11:104607999-104670471 | 10790 | Kat7 | NM_001195003.1 | chr11:95271852-95310246 |
| 10694 | Itgb3bp | NM_026348.3 | chr4:99765401-99829118 | 10791 | Kat8 | NM_026370.1 | chr7:127912516-127925762 |
| 10695 | Itgb4 | NM_001005608.2 | chr11:115974724-116008411 | 10792 | Katna1 | NM_011835.2 | chr10:7725999-7763150 |
| 10696 | Itgb5 | NM_001145884.1 | chr16:33829664-33949338 | 10793 | Katnal1 | NM_153572.2 | chr5:148871583-148928647 |
| 10697 | Itgb6 | NM_001159564.1 | chr2:60598291-60722603 | 10794 | Katnal2 | NM_027721.2 | chr18:76993199-77047296 |
| 10698 | Itgb7 | NM_013566.2 | chr15:102215994-102231935 | 10795 | Katnb1 | NM_028805.2 | chr8:95081200-95099874 |
| 10699 | Itgb8 | NM_177290.3 | chr12:119162802-119238276 | 10796 | Katnbl1 | NM_024254.3 | chr2:112379210-112414237 |
| 10700 | Itgbl1 | NM_145467.2 | chr14:123660139-123974079 | 10797 | Kazald1 | NM_178929.4 | chr19:45076138-45079289 |
| 10701 | Itih1 | NM_008406.3 | chr14:30929179-30943289 | 10798 | Kazn | NM_001109684.1 | chr4:142139239-142210208 |
| 10702 | Itih2 | NM_010582.3 | chr2:10094590-10130683 | 10799 | Kbtbd11 | NM_029116.2 | chr8:15011024-15033332 |
| 10703 | Itih3 | NM_008407.2 | chr14:30908573-30923587 | 10800 | Kbtbd12 | NM_001278671.1 | chr6:88547721-88627448 |
| 10704 | Itih4 | NM_001159299.2 | chr14:30886475-30901986 | 10801 | Kbtbd13 | NM_028974.1 | chr9:65388683-65391652 |
| 10705 | Itih5 | NM_172471.2 | chr2:10153542-10256529 | 10802 | Kbtbd2 | NM_145958.2 | chr6:56777524-56797813 |
| 10706 | Itk | NM_001281965.1 | chr11:46325147-46389515 | 10803 | Kbtbd3 | NM_001164574.1 | chr9:4309742-4331732 |
| 10707 | Itln1 | NM_010584.3 | chr1:171518123-171535294 | 10804 | Kbtbd4 | NM_025991.3 | chr2:90904785-90910560 |
| 10708 | Itm2a | NM_008409.2 | chrX:107397194-107403360 | 10805 | Kbtbd7 | NM_001024135.2 | chr14:79426510-79431038 |
| 10709 | Itm2b | NM_008410.2 | chr14:73362231-73385271 | 10806 | Kbtbd8 | NM_001008785.5 | chr6:95117879-95129793 |
| 10710 | Itm2c | NM_022417.3 | chr1:85894509-85908698 | 10807 | Kcmf1 | NM_019715.2 | chr6:72841113-72899979 |
| 10711 | Itpa | NM_025922.2 | chr2:130667840-130681614 | 10808 | Kcna1 | NM_010595.3 | chr6:126636462-126645801 |
| 10712 | Itpk1 | NM_172584.3 | chr12:102568582-102704869 | 10809 | Kcna10 | NM_001081140.1 | chr3:107183142-107195721 |
| 10713 | Itpka | NM_146125.2 | chr2:119742336-119751253 | 10810 | Kcna2 | NM_008417.5 | chr3:107101566-107115005 |
| 10714 | Itpkb | NM_001081175.1 | chr1:180330475-180423659 | 10811 | Kcna3 | NM_008418.2 | chr3:107036161-107038129 |
| 10715 | Itpkc | NM_181593.2 | chr7:27207169-27228597 | 10812 | Kcna4 | NM_021275.4 | chr2:107290588-107298504 |
| 10716 | Itpr1 | NM_010585.5 | chr6:108213095-108551116 | 10813 | Kcna5 | NM_145983.2 | chr6:126532550-126535555 |
| 10717 | Itpr2 | NM_010586.2 | chr6:146108298-146502223 | 10814 | Kcna6 | NM_013568.6 | chr6:126708328-126740674 |
| 10718 | Itpr3 | NM_080553.3 | chr17:27057303-27122223 | 10815 | Kcna7 | NM_010596.2 | chr7:45405959-45411382 |
| 10719 | Itprip | NM_001001738.2 | chr19:47894595-47919299 | 10816 | Kcnab1 | NM_001289450.1 | chr3:65188102-65378225 |
| 10720 | Itpripl1 | NM_001163527.1 | chr2:127138768-127143457 | 10817 | Kcnab2 | NM_001252654.1 | chr4:152390739-152477549 |
| 10721 | Itpripl2 | NM_001033380.3 | chr7:118485111-118491975 | 10818 | Kcnab3 | NM_010599.4 | chr11:69326257-69333041 |
| 10722 | Itsn1 | NM_001110275.1 | chr16:91729370-91871655 | 10819 | Kcnb1 | NM_008420.4 | chr2:167095968-167188818 |
| 10723 | Itsn2 | NM_001198968.2 | chr12:4593007-4713952 | 10820 | Kcnb2 | NM_001098528.2 | chr1:15312451-15714214 |
| 10724 | Ivd | NM_019826.3 | chr2:118861999-118881357 | 10821 | Kcnc1 | NM_001112739.2 | chr7:46396467-46438704 |
| 10725 | Ivl | NM_008412.3 | chr3:92570899-92573735 | 10822 | Kcnc2 | NM_001025581.1 | chr10:112271122-112466304 |
| 10726 | Ivns1abp | NM_001039511.1 | chr1:151344497-151356798 | 10823 | Kcnc3 | NM_001290682.1 | chr7:44590885-44604751 |
| 10727 | Iws1 | NM_173441.3 | chr18:32067733-32104331 | 10824 | Kcnc4 | NM_145922.2 | chr3:107438302-107458898 |
| 10728 | Iyd | NM_027391.3 | chr10:3540278-3554877 | 10825 | Kcnd1 | NM_008423.2 | chrX:7823758-7838278 |
| 10729 | Izumo1 | NM_001018013.1 | chr7:45621810-45627242 | 10826 | Kcnd2 | NM_019697.3 | chr6:21216108-21729805 |
| 10730 | Izumo2 | NM_029317.1 | chr7:44708742-44719842 | 10827 | Kcnd3 | NM_001039347.1 | chr3:105452329-105674002 |
| 10731 | Izumo3 | NM_027034.1 | chr4:92144317-92147221 | 10828 | Kcnd3os | NR_040759.1 | chr3:105448189-105452955 |
| 10732 | Izumo4 | NM_027829.3 | chr10:80702692-80705382 | 10829 | Kcne1 | NM_008424.3 | chr16:92346000-92359468 |
| 10733 | Jade1 | NM_001130184.1 | chr3:41555733-41616864 | 10830 | Kcne1l | NM_021487.1 | chrX:142304752-142306198 |
| 10734 | Jade2 | NM_199299.3 | chr11:51813455-51857481 | 10831 | Kcne2 | NM_134110.3 | chr16:92292388-92298133 |
| 10735 | Jade3 | NM_001289684.1 | chrX:20425687-20519939 | 10832 | Kcne3 | NM_001190869.1 | chr7:100176669-100184869 |
| 10736 | Jag1 | NM_013822.5 | chr2:137081450-137116520 | 10833 | Kcne4 | NM_021342.1 | chr1:78816948-78820025 |
| 10737 | Jag2 | NM_010588.2 | chr12:112908589-112929495 | 10834 | Kcnf1 | NM_201531.3 | chr12:17172099-17176888 |
| 10738 | Jagn1 | NM_001205025.1 | chr6:113442516-113448229 | 10835 | Kcng1 | NM_001081134.1 | chr2:168261697-168269331 |
| 10739 | Jak1 | NM_146145.2 | chr4:101151973-101265282 | 10836 | Kcng2 | NM_001190373.1 | chr18:80294543-80364254 |
| 10740 | Jak2 | NM_001048177.2 | chr19:29251802-29313080 | 10837 | Kcng3 | NM_153512.1 | chr17:83585956-83631895 |
| 10741 | Jak3 | NM_001190830.1 | chr8:71676382-71688313 | 10838 | Kcng4 | NM_025734.2 | chr8:119623853-119635680 |
| 10742 | Jakmip1 | NM_178394.4 | chr5:37050856-37125298 | 10839 | Kcnh1 | NM_001038607.2 | chr1:192190776-192510159 |
| 10743 | Jakmip2 | NM_001163637.1 | chr18:43531407-43687773 | 10840 | Kcnh2 | NM_013569.2 | chr5:24319588-24351604 |
| 10744 | Jakmip3 | NM_028708.2 | chr7:138940729-139083976 | 10841 | Kcnh3 | NM_010601.3 | chr15:99224975-99242817 |
| 10745 | Jam2 | NM_023844.5 | chr16:84774122-84826375 | 10842 | Kcnh4 | NM_001081194.2 | chr11:100740377-100759778 |
| 10746 | Jam3 | NM_023277.4 | chr9:27097383-27155421 | 10843 | Kcnh5 | NM_172805.3 | chr12:74897216-75177332 |
| 10747 | Jarid2 | NM_001205043.1 | chr13:44731270-44921643 | 10844 | Kcnh6 | NM_001037712.1 | chr11:106008202-106034064 |
| 10748 | Jazf1 | NM_001168277.1 | chr6:52768067-52909620 | 10845 | Kcnh7 | NM_133207.2 | chr2:62702945-63184287 |
| 10749 | Jdp2 | NM_001205052.1 | chr12:85599104-85639878 | 10846 | Kcnh8 | NM_001031811.2 | chr17:52602708-52979194 |
| 10750 | Jkamp | NM_001205067.1 | chr12:72085838-72101527 | 10847 | Kcnip1 | NM_001190885.1 | chr11:33629340-33843585 |
| 10751 | Jmjd1c | NM_001242396.1 | chr10:67185749-67256326 | 10848 | Kcnip2 | NM_001276358.1 | chr19:45792345-45812291 |
| 10752 | Jmjd4 | NM_001205068.1 | chr11:59450044-59458567 | 10849 | Kcnip3 | NM_001111331.1 | chr2:127456497-127482499 |
| 10753 | Jmjd6 | NM_033398.2 | chr11:116837431-116843449 | 10850 | Kcnip4 | NM_001199242.1 | chr5:48389502-48509761 |
| 10754 | Jmjd7 | NM_001114637.1 | chr2:120027482-120032604 | 10851 | Kcnj1 | NM_001168354.1 | chr9:32393984-32399194 |
| 10755 | Jmjd7-pla2g4b | NR_104353.1 | chr2:120027482-120043032 | 10852 | Kcnj10 | NM_001039484.1 | chr1:172341209-172374085 |
| 10756 | Jmjd8 | NM_028101.4 | chr17:25829042-25831843 | 10853 | Kcnj11 | NM_001204411.1 | chr7:46097122-46100764 |
| 10757 | Jmy | NM_021310.3 | chr13:93430096-93499808 | 10854 | Kcnj12 | NM_001267593.1 | chr11:61022563-61073267 |
| 10758 | Josd1 | NM_028792.3 | chr15:79674249-79687872 | 10855 | Kcnj13 | NM_001110227.2 | chr1:87384576-87394729 |
| 10759 | Josd2 | NM_001205070.1 | chr7:44467979-44471658 | 10856 | Kcnj14 | NM_145963.2 | chr7:45816466-45824747 |
| 10760 | Jph1 | NM_020604.2 | chr1:16994939-17097889 | 10857 | Kcnj15 | NM_001039056.2 | chr16:95257557-95300258 |
| 10761 | Jph2 | NM_001205076.1 | chr2:163336241-163397993 | 10858 | Kcnj16 | NM_001252207.1 | chr11:110968032-111027967 |
| 10762 | Jph3 | NM_020605.3 | chr8:121730558-121791083 | 10859 | Kcnj2 | NM_008425.4 | chr11:111066163-111076825 |
| 10763 | Jph4 | NM_177049.5 | chr14:55106825-55116935 | 10860 | Kcnj3 | NM_008426.2 | chr2:55435969-55598145 |
| 10764 | Jpx | NR_015508.3 | chrX:103493557-103506425 | 10861 | Kcnj4 | NM_008427.4 | chr15:79483713-79505241 |
| 10765 | Jrk | NM_008415.6 | chr15:74702411-74709322 | 10862 | Kcnj5 | NM_010605.4 | chr9:32314782-32344237 |
| 10766 | Jrkl | NM_001033181.1 | chr9:13242789-13245741 | 10863 | Kcnj6 | NM_001025584.2 | chr16:94748682-94997696 |

Fig.21 - 57

| | | | |
|---|---|---|---|
| 10864 | Kcnj8 | NM_008428.4 | chr6:142564938-142571356 |
| 10865 | Kcnj9 | NM_008429.2 | chr1:172321032-172329263 |
| 10866 | Kcnk1 | NM_008430.2 | chr8:125995101-126030688 |
| 10867 | Kcnk10 | NM_029911.5 | chr12:98433993-98577940 |
| 10868 | Kcnk12 | NM_199251.1 | chr17:87745820-87797994 |
| 10869 | Kcnk13 | NM_001164426.1 | chr12:99964498-100062682 |
| 10870 | Kcnk15 | NM_001030292.1 | chr2:163853749-163858874 |
| 10871 | Kcnk16 | NM_029006.1 | chr14:20262755-20269162 |
| 10872 | Kcnk18 | NM_207261.3 | chr19:59219647-59237370 |
| 10873 | Kcnk2 | NM_001159850.1 | chr1:189207929-189343353 |
| 10874 | Kcnk3 | NM_010608.2 | chr5:30588169-30625270 |
| 10875 | Kcnk4 | NM_008431.2 | chr19:6925689-6934515 |
| 10876 | Kcnk5 | NM_021542.4 | chr14:20140057-20181782 |
| 10877 | Kcnk6 | NM_001033525.3 | chr7:29221927-29232522 |
| 10878 | Kcnk7 | NM_010609.2 | chr19:5704475-5707101 |
| 10879 | Kcnk9 | NM_001033876.1 | chr15:72512118-72546279 |
| 10880 | Kcnma1 | NM_001253358.1 | chr14:23298693-24004205 |
| 10881 | Kcnmb1 | NM_031169.4 | chr11:33963012-33973638 |
| 10882 | Kcnmb2 | NM_028231.2 | chr3:31902702-32200180 |
| 10883 | Kcnmb3 | NM_001195074.1 | chr3:32472320-32491969 |
| 10884 | Kcnmb4 | NM_021452.1 | chr10:116417867-116473523 |
| 10885 | Kcnmb4os1 | NR_028107.1 | chr10:116418120-116421235 |
| 10886 | Kcnn1 | NM_032397.2 | chr8:70842048-70857008 |
| 10887 | Kcnn2 | NM_080465.2 | chr18:45560153-45685883 |
| 10888 | Kcnn3 | NM_080466.2 | chr3:89520163-89672494 |
| 10889 | Kcnn4 | NM_001163510.1 | chr7:24370262-24385212 |
| 10890 | Kcnq1 | NM_008434.2 | chr7:143107253-143427042 |
| 10891 | Kcnq1ot1 | NR_001461.5 | chr7:143213110-143296547 |
| 10892 | Kcnq2 | NM_001003824.2 | chr2:181075578-181135289 |
| 10893 | Kcnq3 | NM_152923.2 | chr15:65994914-66286224 |
| 10894 | Kcnq4 | NM_001081142.1 | chr4:120697472-120747176 |
| 10895 | Kcnq5 | NM_001160139.1 | chr1:21398402-21961942 |
| 10896 | Kcnrg | NM_001039105.3 | chr14:61607456-61612833 |
| 10897 | Kcns1 | NM_008435.2 | chr2:164163618-164171113 |
| 10898 | Kcns2 | NM_001271704.1 | chr15:34838048-34843407 |
| 10899 | Kcns3 | NM_001168564.1 | chr12:11090201-11150842 |
| 10900 | Kcnt1 | NM_001145403.2 | chr2:25863794-25918273 |
| 10901 | Kcnt2 | NM_001081027.2 | chr1:140246256-140610261 |
| 10902 | Kcnu1 | NM_008432.3 | chr8:25849622-25937934 |
| 10903 | Kcnv1 | NM_026200.3 | chr15:45106283-45114934 |
| 10904 | Kcnv2 | NM_183179.1 | chr19:27322618-27337179 |
| 10905 | Kcp | NM_001029985.4 | chr6:29482035-29507952 |
| 10906 | Kctd1 | NM_001142731.1 | chr18:14968684-15063582 |
| 10907 | Kctd10 | NM_001159941.1 | chr5:114363571-114380505 |
| 10908 | Kctd11 | NM_153143.4 | chr11:69878263-69880985 |
| 10909 | Kctd12 | NM_177715.4 | chr14:102976580-102982637 |
| 10910 | Kctd12b | NM_175429.3 | chrX:153685153-153696280 |
| 10911 | Kctd13 | NM_172747.2 | chr7:126928878-126945609 |
| 10912 | Kctd14 | NM_001010826.3 | chr7:97453203-97459557 |
| 10913 | Kctd15 | NM_146188.1 | chr7:34639015-34652841 |
| 10914 | Kctd16 | NM_026135.1 | chr18:40258360-40531184 |
| 10915 | Kctd17 | NM_001289671.1 | chr15:78428563-78439303 |
| 10916 | Kctd18 | NM_001159864.1 | chr1:57955100-57970084 |
| 10917 | Kctd19 | NM_177791.3 | chr8:105382806-105413502 |
| 10918 | Kctd2 | NM_183285.3 | chr11:115420125-115431274 |
| 10919 | Kctd20 | NM_025888.5 | chr17:28953215-28967937 |
| 10920 | Kctd21 | NM_001039039.3 | chr7:97332322-97350216 |
| 10921 | Kctd3 | NM_172650.2 | chr1:188971097-189007840 |
| 10922 | Kctd4 | NM_026214.3 | chr14:75955002-75965217 |
| 10923 | Kctd5 | NM_027008.2 | chr17:24047719-24073485 |
| 10924 | Kctd6 | NM_027782.3 | chr14:8214080-8223569 |
| 10925 | Kctd7 | NM_172509.3 | chr5:130144887-130155808 |
| 10926 | Kctd8 | NM_175519.5 | chr5:69109284-69341709 |
| 10927 | Kctd9 | NM_001111028.1 | chr14:67716097-67742310 |
| 10928 | Kdelc1 | NM_023645.3 | chr1:44106545-44118773 |
| 10929 | Kdelc2 | NM_212445.2 | chr9:53384022-53401867 |
| 10930 | Kdelr1 | NM_133950.2 | chr7:45872839-45883726 |
| 10931 | Kdelr2 | NM_025841.4 | chr5:143403819-143421904 |
| 10932 | Kdelr3 | NM_134090.2 | chr15:79516407-79527739 |
| 10933 | Kdf1 | NM_001083916.1 | chr4:133518962-133530790 |
| 10934 | Kdm1a | NM_133872.2 | chr4:136550532-136602723 |
| 10935 | Kdm1b | NM_172262.3 | chr13:47043498-47085279 |
| 10936 | Kdm2a | NM_001001984.2 | chr19:4316146-4397077 |
| 10937 | Kdm2b | NM_001003953.2 | chr5:122870675-122989099 |
| 10938 | Kdm3a | NM_001038695.3 | chr6:71588969-71632917 |
| 10939 | Kdm3b | NM_001081256.1 | chr18:34777007-34839370 |
| 10940 | Kdm4a | NM_001161823.1 | chr4:118137003-118180043 |
| 10941 | Kdm4b | NM_172132.2 | chr17:56326049-56402873 |
| 10942 | Kdm4c | NM_001172095.1 | chr4:74242496-74405864 |
| 10943 | Kdm4d | NM_173433.2 | chr9:14462580-14500482 |
| 10944 | Kdm5a | NM_145997.2 | chr6:120364098-120444574 |
| 10945 | Kdm5b | NM_152895.2 | chr1:134560177-134632878 |
| 10946 | Kdm5c | NM_013668.4 | chrX:152233226-152279099 |
| 10947 | Kdm5d | NM_011419.3 | chrY:897787-943811 |
| 10948 | Kdm6a | NM_009483.2 | chrX:18162574-18279936 |
| 10949 | Kdm6b | NM_001017426.1 | chr11:69398517-69413675 |
| 10950 | Kdm7a | NM_001033430.4 | chr6:39136619-39206773 |
| 10951 | Kdm8 | NM_029842.5 | chr7:125444619-125462268 |
| 10952 | Kdr | NM_010612.2 | chr5:75933269-75978428 |
| 10953 | Kdsr | NM_027534.2 | chr1:106720409-106759742 |
| 10954 | Keap1 | NM_001110305.1 | chr9:21229729-21239332 |
| 10955 | Keg1 | NM_029550.4 | chr19:12695789-12719896 |
| 10956 | Kel | NM_032540.3 | chr6:41686329-41704325 |
| 10957 | Kera | NM_008438.3 | chr10:97607204-97613688 |
| 10958 | Khdc1a | NM_183322.2 | chr1:21349676-21352199 |
| 10959 | Khdc1b | NM_001113187.1 | chr1:21383555-21386384 |
| 10960 | Khdc1c | NM_001033904.1 | chr1:21368330-21369743 |

| | | | |
|---|---|---|---|
| 10961 | Khdc3 | NM_025890.3 | chr9:73102397-73104443 |
| 10962 | Khdrbs1 | NM_011317.4 | chr4:129713814-129742303 |
| 10963 | Khdrbs2 | NM_133235.2 | chr1:32172805-32657738 |
| 10964 | Khdrbs3 | NM_010158.2 | chr15:68928419-69093518 |
| 10965 | Khk | NM_008439.4 | chr5:30921558-30931248 |
| 10966 | Khnyn | NM_027143.2 | chr14:55884968-55896781 |
| 10967 | Khsrp | NM_010613.3 | chr17:57021048-57031507 |
| 10968 | Kidins220 | NM_001081378.1 | chr12:24974931-25059697 |
| 10969 | Kif11 | NM_010615.1 | chr19:37376402-37421859 |
| 10970 | Kif12 | NM_010616.2 | chr4:63165636-63172131 |
| 10971 | Kif13a | NM_010617.2 | chr13:46749087-46929718 |
| 10972 | Kif13b | NM_001081177.1 | chr14:64652530-64806296 |
| 10973 | Kif14 | NM_001287179.2 | chr1:136467847-136530819 |
| 10974 | Kif15 | NM_010620.1 | chr9:122951080-123018733 |
| 10975 | Kif16b | NM_001081133.2 | chr2:142618344-142901464 |
| 10976 | Kif17 | NM_001190978.1 | chr4:138262250-138301973 |
| 10977 | Kif18a | NM_139303.1 | chr2:109280737-109341746 |
| 10978 | Kif18b | NM_197959.2 | chr11:102905518-102925124 |
| 10979 | Kif19a | NM_001102615.1 | chr11:114765388-114790575 |
| 10980 | Kif1a | NM_001110315.2 | chr1:93015454-93101874 |
| 10981 | Kif1b | NM_001290995.1 | chr4:149176318-149307733 |
| 10982 | Kif1c | NM_153103.2 | chr11:70700547-70731970 |
| 10983 | Kif20a | NM_001166406.1 | chr18:34625032-34633277 |
| 10984 | Kif20b | NM_183046.1 | chr19:34922357-34975731 |
| 10985 | Kif21a | NM_001109040.2 | chr15:90933274-91049951 |
| 10986 | Kif21b | NM_001039472.1 | chr1:136131400-136178014 |
| 10987 | Kif22 | NM_145588.1 | chr7:127027730-127042420 |
| 10988 | Kif23 | NM_024245.4 | chr9:61917277-61946799 |
| 10989 | Kif24 | NM_024241.2 | chr4:41390747-41464848 |
| 10990 | Kif26a | NM_001097621.1 | chr12:112146207-112181747 |
| 10991 | Kif26b | NM_001161665.1 | chr1:178529124-178932857 |
| 10992 | Kif27 | NM_175214.3 | chr13:58287515-58354862 |
| 10993 | Kif2a | NM_001145779.1 | chr13:106960584-107022114 |
| 10994 | Kif2b | NM_028547.2 | chr11:91575285-91577555 |
| 10995 | Kif2c | NM_001290662.1 | chr4:117159632-117178772 |
| 10996 | Kif3a | NM_001290805.1 | chr11:53567368-53604246 |
| 10997 | Kif3b | NM_008444.4 | chr2:153291415-153333389 |
| 10998 | Kif3c | NM_008445.2 | chr12:3365131-3406494 |
| 10999 | Kif4 | NM_008446.2 | chrX:100626064-100727271 |
| 11000 | Kif4-ps | NR_033653.1 | chr12:101145608-101149273 |
| 11001 | Kif5a | NM_001039000.4 | chr10:127225694-127263363 |
| 11002 | Kif5b | NM_008448.3 | chr18:6201004-6241524 |
| 11003 | Kif5c | NM_008449.2 | chr2:49619313-49774778 |
| 11004 | Kif6 | NM_177052.3 | chr17:49615171-49909847 |
| 11005 | Kif7 | NM_001291222.1 | chr7:79698097-79714186 |
| 11006 | Kif9 | NM_001163569.1 | chr9:110476993-110524440 |
| 11007 | Kifap3 | NM_010629.3 | chr1:163779582-163917107 |
| 11008 | Kifc1 | NM_001195298.1 | chr17:33875665-33890633 |
| 11009 | Kifc2 | NM_010630.2 | chr15:76660640-76668196 |
| 11010 | Kifc3 | NM_001145831.1 | chr8:95099827-95142540 |
| 11011 | Kifc5b | NM_053173.2 | chr17:26917090-26932579 |
| 11012 | Kin | NM_025280.2 | chr2:10080611-10092701 |
| 11013 | Kir3dl1 | NM_177749.4 | chrX:136517998-136534306 |
| 11014 | Kir3dl2 | NM_177748.2 | chrX:136448106-136469041 |
| 11015 | Kirrel | NM_001170985.1 | chr3:87078591-87174747 |
| 11016 | Kirrel2 | NM_172898.3 | chr7:30447765-30457515 |
| 11017 | Kirrel3 | NM_001190911.1 | chr9:34488730-35036307 |
| 11018 | Kis2 | NR_003188.1 | chrX:52742562-52744593 |
| 11019 | Kiss1 | NM_178260.3 | chr1:133327211-133329722 |
| 11020 | Kiss1r | NM_053244.5 | chr10:79916970-79922272 |
| 11021 | Kit | NM_001122733.1 | chr5:75574986-75656721 |
| 11022 | Kitl | NM_013598.2 | chr10:100015823-100100412 |
| 11023 | Kiz | NM_001033298.3 | chr2:146855888-146970089 |
| 11024 | Kl | NM_013823.2 | chr5:150952606-150993817 |
| 11025 | Klb | NM_031180.2 | chr5:65348410-65384003 |
| 11026 | Klc1 | NM_001025358.2 | chr12:111758867-111794899 |
| 11027 | Klc2 | NM_008451.2 | chr19:5107745-5118408 |
| 11028 | Klc3 | NM_001286038.1 | chr7:19394439-19399921 |
| 11029 | Klc4 | NM_029091.2 | chr17:46630630-46645144 |
| 11030 | Klf1 | NM_010635.2 | chr8:84901927-84905295 |
| 11031 | Klf10 | NM_001289471.1 | chr15:38291463-38300711 |
| 11032 | Klf11 | NM_178357.3 | chr12:24651370-24662782 |
| 11033 | Klf12 | NM_010636.3 | chr14:99870639-100149796 |
| 11034 | Klf13 | NM_021366.3 | chr7:63886350-63938915 |
| 11035 | Klf14 | NM_001135093.1 | chr6:30956020-30958990 |
| 11036 | Klf15 | NM_023184.3 | chr6:90462625-90475209 |
| 11037 | Klf16 | NM_078477.2 | chr10:80567120-80577296 |
| 11038 | Klf17 | NM_029416.2 | chr4:117756658-117765666 |
| 11039 | Klf2 | NM_008452.2 | chr8:72319061-72321654 |
| 11040 | Klf3 | NM_008453.5 | chr5:64803522-64830129 |
| 11041 | Klf4 | NM_010637.3 | chr4:55527136-55532475 |
| 11042 | Klf5 | NM_009769.4 | chr14:99298690-99313409 |
| 11043 | Klf6 | NM_011803.2 | chr13:5861488-5870393 |
| 11044 | Klf7 | NM_033563.2 | chr1:64035670-64121389 |
| 11045 | Klf8 | NM_173780.4 | chrX:153238044-153396134 |
| 11046 | Klf9 | NM_010638.4 | chr19:23141225-23166911 |
| 11047 | Klhdc1 | NM_178253.5 | chr12:69241831-69283961 |
| 11048 | Klhdc10 | NM_029742.3 | chr6:30401854-30455174 |
| 11049 | Klhdc2 | NM_027117.3 | chr12:69296680-69310687 |
| 11050 | Klhdc3 | NM_001163729.2 | chr17:46674550-46680930 |
| 11051 | Klhdc4 | NM_145605.2 | chr8:121796307-121829569 |
| 11052 | Klhdc7a | NM_173427.2 | chr4:139962172-139968026 |
| 11053 | Klhdc7b | NM_001160178.1 | chr15:89386890-89388708 |
| 11054 | Klhdc8a | NM_144810.4 | chr1:132298625-132307357 |
| 11055 | Klhdc8b | NM_030075.2 | chr9:108447638-108461581 |
| 11056 | Klhdc9 | NM_001033039.2 | chr1:171358448-171360798 |
| 11057 | Klhl1 | NM_053105.2 | chr14:96105264-96519034 |

Fig.21 - 58

| | | | | | | | |
|---|---|---|---|---|---|---|---|
| 11058 | Klhl10 | NM_025727.3 | chr11:100441923-100457024 | 11155 | Klrg2 | NM_001033171.2 | chr6:38626659-38637239 |
| 11059 | Klhl11 | NM_172565.2 | chr11:100462611-100472782 | 11156 | Klri1 | NM_001012520.2 | chr6:129697217-129717132 |
| 11060 | Klhl12 | NM_153128.3 | chr1:134455530-134490873 | 11157 | Klri2 | NM_177155.4 | chr6:129729040-129740484 |
| 11061 | Klhl13 | NM_001290476.2 | chrX:23219270-23285559 | 11158 | Klrk1 | NM_001083322.2 | chr6:129610322-129623864 |
| 11062 | Klhl14 | NM_001081403.1 | chr18:21550376-21652368 | 11159 | Kmo | NM_133809.1 | chr1:175632192-175660853 |
| 11063 | Klhl15 | NM_001039059.1 | chrX:94234929-94255968 | 11160 | Kmt2a | NM_001081049.1 | chr9:44803354-44881274 |
| 11064 | Klhl17 | NM_198305.2 | chr4:156229043-156234857 | 11161 | Kmt2b | NM_001290573.1 | chr7:30568854-30588726 |
| 11065 | Klhl18 | NM_177771.5 | chr9:110425925-110476694 | 11162 | Kmt2c | NM_001081383.1 | chr5:25271793-25498783 |
| 11066 | Klhl2 | NM_178633.3 | chr8:64739674-64849924 | 11163 | Kmt2d | NM_001033276.3 | chr15:98831668-98871205 |
| 11067 | Klhl20 | NM_001039482.1 | chr1:161088377-161131479 | 11164 | Kmt2e | NM_026984.1 | chr5:23434428-23504229 |
| 11068 | Klhl21 | NM_001033352.3 | chr4:152008890-152017677 | 11165 | Kncn | NM_001039124.3 | chr4:115884399-115887964 |
| 11069 | Klhl22 | NM_145479.4 | chr16:17759620-17793382 | 11166 | Kndc1 | NM_177261.4 | chr7:139894695-139941540 |
| 11070 | Klhl23 | NM_177784.4 | chr2:69822369-69836651 | 11167 | Kng1 | NM_001102411.1 | chr16:23058299-23080006 |
| 11071 | Klhl24 | NM_029436.3 | chr16:20097553-20127744 | 11168 | Kng2 | NM_001102409.1 | chr16:22985851-23029101 |
| 11072 | Klhl25 | NM_001122780.1 | chr7:75848337-75874130 | 11169 | Knop1 | NM_001168218.1 | chr7:118842217-118855998 |
| 11073 | Klhl26 | NM_001122830.1 | chr8:70450227-70476943 | 11170 | Knstrn | NM_026412.3 | chr2:118814002-118836212 |
| 11074 | Klhl28 | NM_025707.3 | chr12:64942439-64965536 | 11171 | Kntc1 | NM_001042421.1 | chr5:123749725-123821593 |
| 11075 | Klhl29 | NM_001164493.1 | chr12:5077465-5375682 | 11172 | Kpna1 | NM_008465.5 | chr16:35983362-36036162 |
| 11076 | Klhl3 | NM_001195075.1 | chr13:58004956-58102428 | 11173 | Kpna2 | NM_010655.3 | chr11:106988628-106999525 |
| 11077 | Klhl30 | NM_027551.2 | chr1:91351072-91362404 | 11174 | Kpna3 | NM_008466.5 | chr14:61365185-61439947 |
| 11078 | Klhl31 | NM_172925.2 | chr9:77636731-77660122 | 11175 | Kpna4 | NM_008467.4 | chr3:69072220-69127092 |
| 11079 | Klhl32 | NM_001033531.3 | chr4:24617272-24851086 | 11176 | Kpna6 | NM_008468.4 | chr4:129643978-129672767 |
| 11080 | Klhl33 | NM_001166651.1 | chr14:50891388-50893255 | 11177 | Kpna7 | NM_001013774.2 | chr5:144983743-145009636 |
| 11081 | Klhl34 | NM_001081667.2 | chrX:157818434-157821060 | 11178 | Kpnb1 | NM_008379.3 | chr11:97159709-97187892 |
| 11082 | Klhl35 | NM_028145.1 | chr7:99466003-99474020 | 11179 | Kprp | NM_028629.1 | chr3:92823073-92827247 |
| 11083 | Klhl36 | NM_146219.1 | chr8:119862304-119876989 | 11180 | Kptn | NM_133727.2 | chr7:16119875-16127516 |
| 11084 | Klhl38 | NM_177755.3 | chr15:58314572-58324169 | 11181 | Kras | NM_021284 | chr6:145216698-145250231 |
| 11085 | Klhl4 | NM_001290477.1 | chrX:114474332-114561129 | 11182 | Krba1 | NM_133922.3 | chr6:48395585-48419855 |
| 11086 | Klhl40 | NM_028202.3 | chr9:121777606-121783819 | 11183 | Krcc1 | NM_145568.3 | chr6:71272018-71285319 |
| 11087 | Klhl41 | NM_001081087.1 | chr2:69670119-69684239 | 11184 | Kremen1 | NM_032396.3 | chr11:5191552-5261610 |
| 11088 | Klhl42 | NM_001081237.1 | chr6:147091074-147112778 | 11185 | Kremen2 | NM_028416.1 | chr17:23741198-23745829 |
| 11089 | Klhl5 | NM_175174.4 | chr5:65131230-65168142 | 11186 | Kri1 | NM_145416.3 | chr9:21273456-21287969 |
| 11090 | Klhl6 | NM_183390.3 | chr16:19946491-19983049 | 11187 | Krit1 | NM_001170552.1 | chr5:3803164-3844515 |
| 11091 | Klhl7 | NM_001161800.1 | chr5:24100589-24161231 | 11188 | Krr1 | NM_178610.4 | chr10:111972694-111988430 |
| 11092 | Klhl8 | NM_178741.3 | chr5:103862049-103911229 | 11189 | Krt1 | NM_008473.2 | chr15:101845425-101850786 |
| 11093 | Klhl9 | NM_172871.2 | chr4:88718291-88722508 | 11190 | Krt10 | NM_010660.2 | chr11:99385255-99389364 |
| 11094 | Klk1 | NM_010639.7 | chr7:44225436-44229617 | 11191 | Krt12 | NM_010661.2 | chr11:99415663-99422259 |
| 11095 | Klk10 | NM_133712.2 | chr7:43781053-43785410 | 11192 | Krt13 | NM_010662.2 | chr11:100117327-100121566 |
| 11096 | Klk11 | NM_001177373.1 | chr7:43774616-43779262 | 11193 | Krt14 | NM_016958.2 | chr11:100203161-100207510 |
| 11097 | Klk12 | NM_027097.1 | chr7:43769099-43773481 | 11194 | Krt15 | NM_008469.2 | chr11:100131758-100135949 |
| 11098 | Klk13 | NM_001039042.2 | chr7:43712566-43726758 | 11195 | Krt16 | NM_008470.1 | chr11:100246090-100248902 |
| 11099 | Klk14 | NM_174866.3 | chr7:43690417-43695536 | 11196 | Krt17 | NM_010663.3 | chr11:100256214-100261029 |
| 11100 | Klk15 | NM_174865.1 | chr7:43933770-43939590 | 11197 | Krt18 | NM_010664.2 | chr15:102028215-102032026 |
| 11101 | Klk1b1 | NM_010645.2 | chr7:43966767-43971315 | 11198 | Krt19 | NM_008471.3 | chr11:100140810-100145926 |
| 11102 | Klk1b11 | NM_010640.1 | chr7:43995878-43999875 | 11199 | Krt2 | NM_010668.2 | chr15:101810688-101818169 |
| 11103 | Klk1b16 | NM_008454.2 | chr7:44136766-44141604 | 11200 | Krt20 | NM_023256.1 | chr11:99428402-99438153 |
| 11104 | Klk1b21 | NM_010642.2 | chr7:44102291-44106579 | 11201 | Krt222 | NM_172946.2 | chr11:99233097-99244067 |
| 11105 | Klk1b22 | NM_010114.1 | chr7:44112682-44116876 | 11202 | Krt23 | NM_033373.1 | chr11:99477972-99493110 |
| 11106 | Klk1b24 | NM_010643.1 | chr7:44188262-44192451 | 11203 | Krt24 | NM_029393.1 | chr11:99280092-99285238 |
| 11107 | Klk1b26 | NM_010644.3 | chr7:44012677-44016969 | 11204 | Krt25 | NM_133730.1 | chr11:99315843-99322941 |
| 11108 | Klk1b27 | NM_020268.3 | chr7:44052289-44056711 | 11205 | Krt26 | NM_001033397.5 | chr11:99328483-99337965 |
| 11109 | Klk1b3 | NM_008693.2 | chr7:44198190-44202351 | 11206 | Krt27 | NM_010666.2 | chr11:99345564-99351118 |
| 11110 | Klk1b4 | NM_010915.3 | chr7:44207434-44211754 | 11207 | Krt28 | NM_027574.1 | chr11:99365006-99374903 |
| 11111 | Klk1b5 | NM_008456.3 | chr7:44216474-44220703 | 11208 | Krt31 | NM_010659.2 | chr11:100046645-100050551 |
| 11112 | Klk1b7-ps | NR_033120.1 | chr7:43945011-43945927 | 11209 | Krt32 | NM_001159374.2 | chr11:100080847-100088226 |
| 11113 | Klk1b8 | NM_008457.2 | chr7:43950671-43954938 | 11210 | Krt33a | NM_027983.3 | chr11:100011198-100016212 |
| 11114 | Klk1b9 | NM_010116.1 | chr7:43976060-43980376 | 11211 | Krt33b | NM_013570.1 | chr11:100023633-100029868 |
| 11115 | Klk4 | NM_019928.1 | chr7:43881171-43885804 | 11212 | Krt34 | NM_027563.4 | chr11:100037347-100041554 |
| 11116 | Klk5 | NM_026806.2 | chr7:43842268-43851181 | 11213 | Krt35 | NM_016880.2 | chr11:100092191-100096224 |
| 11117 | Klk6 | NM_001164696.1 | chr7:43824543-43832027 | 11214 | Krt36 | NM_001174099.1 | chr11:100102012-100105626 |
| 11118 | Klk7 | NM_011872.2 | chr7:43811443-43816359 | 11215 | Krt39 | NM_213730.2 | chr11:99514623-99521258 |
| 11119 | Klk8 | NM_008940.2 | chr7:43797576-43803822 | 11216 | Krt4 | NM_008475.2 | chr15:101918534-101924735 |
| 11120 | Klk9 | NM_028660.3 | chr7:43791890-43796756 | 11217 | Krt40 | NM_001039666.1 | chr11:99537484-99543158 |
| 11121 | Klkb1 | NM_008455.2 | chr8:45269450-45294835 | 11218 | Krt42 | NM_212483.2 | chr11:100262881-100269871 |
| 11122 | Klra1 | NM_016659.3 | chr6:130363917-130386874 | 11219 | Krt5 | NM_027011.2 | chr15:101707069-101712891 |
| 11123 | Klra10 | NM_008459 | chr6:130269193-130281928 | 11220 | Krt6a | NM_008476.3 | chr15:101689927-101694305 |
| 11124 | Klra12 | NM_010646.1 | chr6:130044023-130306481 | 11221 | Krt6b | NM_010669.2 | chr15:101676022-101680289 |
| 11125 | Klra13-ps | NR_033451.1 | chr6:130291160-130306432 | 11222 | Krt7 | NM_033073.3 | chr15:101412402-101427806 |
| 11126 | Klra14-ps | NR_104105.1 | chr6:130149105-130160748 | 11223 | Krt71 | NM_019956.1 | chr15:101733948-101743097 |
| 11127 | Klra15 | NM_013793.2 | chr6:129972092-130380662 | 11224 | Krt72 | NM_213728.1 | chr15:101776559-101786458 |
| 11128 | Klra17 | NM_133203.5 | chr6:129831153-129876672 | 11225 | Krt73 | NM_212485.2 | chr15:101793307-101802332 |
| 11129 | Klra18 | NM_053153.2 | chr6:130043744-130067258 | 11226 | Krt74 | NR_033444.1 | chr15:101754258-101763504 |
| 11130 | Klra19 | NM_053154.2 | chr6:130013033-130026960 | 11227 | Krt75 | NM_133357.3 | chr15:101563342-101573904 |
| 11131 | Klra2 | NM_001170851.1 | chr6:131219234-131247362 | 11228 | Krt76 | NM_001033177.2 | chr15:101884350-101892920 |
| 11132 | Klra21 | NM_053151.1 | chr6:130115218-130129879 | 11229 | Krt77 | NM_001003667.1 | chr15:101859855-101869618 |
| 11133 | Klra22 | NM_053152.2 | chr6:129972081-130380808 | 11230 | Krt78 | NM_212487.4 | chr15:101946003-101954287 |
| 11134 | Klra23 | NM_024470.1 | chr6:130291160-130308361 | 11231 | Krt79 | NM_146063.1 | chr15:101929331-101940324 |
| 11135 | Klra3 | NM_001289604.1 | chr6:130323288-130337626 | 11232 | Krt8 | NM_031170.2 | chr15:101996710-102004342 |
| 11136 | Klra33 | NM_001039118.1 | chr6:130009732-130030995 | 11233 | Krt80 | NM_028770.2 | chr15:101349570-101370125 |
| 11137 | Klra4 | NM_001252577.1 | chr6:130044016-130067271 | 11234 | Krt81 | NM_001166157.1 | chr15:101459060-101463765 |
| 11138 | Klra5 | NM_008463.2 | chr6:129898991-129913224 | 11235 | Krt82 | NM_053249.3 | chr15:101541220-101550659 |
| 11139 | Klra6 | NM_008464.2 | chr6:130013032-130026954 | 11236 | Krt83 | NM_001003668.2 | chr15:101431489-101438804 |
| 11140 | Klra7 | NM_001110323.1 | chr6:130218830-130233322 | 11237 | Krt84 | NM_008474.2 | chr15:101525025-101532820 |
| 11141 | Klra8 | NM_001101620.1 | chr6:130115225-130129898 | 11238 | Krt85 | NM_016879.2 | chr15:101431013-101491303 |
| 11142 | Klra9 | NM_010651.3 | chr6:130178681-130193112 | 11239 | Krt86 | NM_010667.2 | chr15:101473477-101479983 |
| 11143 | Klrb1 | NM_001099918.1 | chr6:128706507-128723046 | 11240 | Krt9 | NM_201255.2 | chr11:100186780-100193246 |
| 11144 | Klrb1a | NM_001159902.1 | chr6:128609226-128622934 | 11241 | Krtap10-10 | NM_001024709.3 | chr10:77835946-77837251 |
| 11145 | Klrb1b | NM_030599.4 | chr6:128813705-128826315 | 11242 | Krtap10-4 | NM_001135991.1 | chr10:77826168-77827070 |
| 11146 | Klrb1c | NM_001159904.1 | chr6:128778484-128788641 | 11243 | Krtap11-1 | NM_001113406.1 | chr16:89570175-89571183 |
| 11147 | Klrb1f | NM_153094.2 | chr6:129045900-129057471 | 11244 | Krtap12-1 | NM_010670.1 | chr10:77720585-77721256 |
| 11148 | Klrb1-ps1 | NR_073569.1 | chr6:129116517-129129446 | 11245 | Krtap13 | NM_010671.1 | chr16:88750743-88751628 |
| 11149 | Klrc1 | NM_001136068.2 | chr6:129666015-129678973 | 11246 | Krtap1-3 | NM_001085526.2 | chr11:99590460-99591377 |
| 11150 | Klrc2 | NM_001098669.1 | chr6:129656345-129660596 | 11247 | Krtap13-1 | NM_183189.1 | chr16:88728861-88729611 |
| 11151 | Klrc3 | NM_021378.1 | chr6:129639084-129643288 | 11248 | Krtap14 | NM_013707.2 | chr16:88825290-88826145 |
| 11152 | Klrd1 | NM_010654.3 | chr6:129591810-129598775 | 11249 | Krtap1-4 | NM_001039502.2 | chr11:99582548-99583677 |
| 11153 | Klre1 | NM_153590.3 | chr6:129578285-129585827 | 11250 | Krtap15 | NM_013713.1 | chr16:88829008-88829844 |
| 11154 | Klrg1 | NM_016970.1 | chr6:122270596-122282833 | 11251 | Krtap1-5 | NM_027157.3 | chr11:99579976-99581016 |

Fig.21 - 59

| | | | | | | | |
|---|---|---|---|---|---|---|---|
| 11252 | Krtap16-1 | NM_130870.1 | chr16:88873664-88874269 | 11349 | Laptm5 | NM_010686.3 | chr4:130913333-130936148 |
| 11253 | Krtap16-3 | NM_183296.1 | chr16:88962303-88962873 | 11350 | Large | NM_010687.1 | chr8:72814598-73352556 |
| 11254 | Krtap17-1 | NM_001099774.2 | chr11:99993231-99993994 | 11351 | Larp1 | NM_028451.1 | chr11:58009063-58062032 |
| 11255 | Krtap19-1 | NM_130876.3 | chr16:88868917-88869410 | 11352 | Larp1b | NM_001040399.1 | chr3:40950630-40977793 |
| 11256 | Krtap19-3 | NM_130857.2 | chr16:88877512-88878038 | 11353 | Larp4 | NM_001024526.2 | chr15:99972779-100016358 |
| 11257 | Krtap19-4 | NM_130873.1 | chr16:88884785-88885089 | 11354 | Larp4b | NM_172585.3 | chr13:9093880-9174451 |
| 11258 | Krtap19-5 | NM_010676.2 | chr16:88895967-88896449 | 11355 | Larp6 | NM_026235.4 | chr9:60713120-60738801 |
| 11259 | Krtap19-9b | NM_133359.2 | chr16:88931803-88932264 | 11356 | Larp7 | NM_138593.2 | chr3:127536713-127553349 |
| 11260 | Krtap20-2 | NM_001163615.1 | chr16:89205860-89206394 | 11357 | Lars | NM_134137.2 | chr18:42202348-42262071 |
| 11261 | Krtap21-1 | NM_028621.3 | chr16:89403026-89403774 | 11358 | Lars2 | NM_153168.2 | chr9:123366939-123462664 |
| 11262 | Krtap22-2 | NM_001191018.1 | chr16:89010379-89010759 | 11359 | Las1l | NM_152822.3 | chrX:95935312-95956974 |
| 11263 | Krtap2-4 | NM_027800.1 | chr11:99614016-99614846 | 11360 | Lasp1 | NM_010688.4 | chr11:97799671-97838764 |
| 11264 | Krtap24-1 | NM_001163141.1 | chr16:88610708-88612279 | 11361 | Lat | NM_010689.2 | chr7:126363827-126369534 |
| 11265 | Krtap26-1 | NM_027105.2 | chr16:88646823-88647796 | 11362 | Lat2 | NM_020044 | chr5:134600102-134615025 |
| 11266 | Krtap27-1 | NM_001163105.1 | chr16:88671045-88671654 | 11363 | Lats1 | NM_010690.1 | chr10:7681208-7716461 |
| 11267 | Krtap3-1 | NM_023511.1 | chr11:99566026-99566630 | 11364 | Lats2 | NM_015771.2 | chr14:57689661-57746123 |
| 11268 | Krtap31-1 | NM_027568.2 | chr11:99907919-99908890 | 11365 | Lax1 | NM_001159649.1 | chr1:133679028-133690108 |
| 11269 | Krtap31-2 | NM_001025244.3 | chr11:99936290-99937225 | 11366 | Layn | NM_001033534.1 | chr9:51056779-51077094 |
| 11270 | Krtap3-2 | NM_025720.3 | chr11:99555822-99556853 | 11367 | Lbh | NM_029999.4 | chr17:72918304-72941946 |
| 11271 | Krtap3-3 | NM_025524.2 | chr11:99550131-99550863 | 11368 | Lbp | NM_008489.2 | chr2:158306492-158332852 |
| 11272 | Krtap4-1 | NM_001048196.1 | chr11:99627228-99628239 | 11369 | Lbr | NM_133815.2 | chr1:181815314-181842401 |
| 11273 | Krtap4-13 | NM_027087.3 | chr11:99809077-99809896 | 11370 | Lbx1 | NM_010691.5 | chr19:45233727-45235236 |
| 11274 | Krtap4-16 | NM_001013823.1 | chr11:99850654-99851605 | 11371 | Lbx2 | NM_010692.3 | chr6:83086364-83088241 |
| 11275 | Krtap4-2 | NM_026807.2 | chr11:99634113-99635084 | 11372 | Lca5 | NM_027448.2 | chr9:83393415-83441099 |
| 11276 | Krtap4-6 | NM_026834.2 | chr11:99665042-99665960 | 11373 | Lca5l | NM_001001492.2 | chr16:96158405-96192257 |
| 11277 | Krtap4-7 | NM_029613.1 | chr11:99643111-99644089 | 11374 | Lcat | NM_008490.2 | chr8:105939550-105943402 |
| 11278 | Krtap4-8 | NM_001085547.2 | chr11:99780013-99780643 | 11375 | Lce1a1 | NM_025984.2 | chr3:92646531-92648307 |
| 11279 | Krtap4-9 | NM_001085548.2 | chr11:99785199-99786257 | 11376 | Lce1a2 | NM_028625.2 | chr3:92668612-92670315 |
| 11280 | Krtap5-1 | NM_015808.1 | chr7:142296376-142297069 | 11377 | Lce1b | NM_026822.1 | chr3:92655649-92656926 |
| 11281 | Krtap5-2 | NM_027844.4 | chr7:142174531-142176005 | 11378 | Lce1c | NM_028622.2 | chr3:92679246-92680918 |
| 11282 | Krtap5-3 | NM_023860.1 | chr7:142201363-142203015 | 11379 | Lce1d | NM_027137.2 | chr3:92685498-92687210 |
| 11283 | Krtap5-4 | NM_015809.2 | chr7:142303501-142304503 | 11380 | Lce1e | NM_026811.2 | chr3:92707399-92709074 |
| 11284 | Krtap5-5 | NM_001037822.1 | chr7:142228794-142229971 | 11381 | Lce1f | NM_026394.3 | chr3:92718695-92720350 |
| 11285 | Krtap6-1 | NM_010672.3 | chr16:89031698-89032292 | 11382 | Lce1g | NM_025413.2 | chr3:92750150-92752338 |
| 11286 | Krtap6-2 | NM_010673.2 | chr16:89419322-89420111 | 11383 | Lce1h | NM_026335.2 | chr3:92763214-92765065 |
| 11287 | Krtap6-5 | NM_130856.2 | chr16:89047288-89047899 | 11384 | Lce1i | NM_029667.2 | chr3:92777209-92778899 |
| 11288 | Krtap7-1 | NM_027771.1 | chr16:89507702-89508323 | 11385 | Lce1j | NM_001281499.1 | chr3:92788844-92790514 |
| 11289 | Krtap8-1 | NM_010675.1 | chr16:89487373-89487952 | 11386 | Lce1k | NM_001254760.1 | chr3:92806290-92807891 |
| 11290 | Krtap9-1 | NM_015741.2 | chr11:99873388-99874000 | 11387 | Lce1l | NM_028628.2 | chr3:92849948-92851286 |
| 11291 | Krtap9-3 | NM_029351.2 | chr11:99597347-99598106 | 11388 | Lce1m | NM_025420.2 | chr3:93017806-93019060 |
| 11292 | Krtap9-5 | NM_001085527.1 | chr11:99948474-99949551 | 11389 | Lce3a | NM_001039594.1 | chr3:92925286-92926230 |
| 11293 | Krtcap2 | NM_025327.3 | chr3:89246437-89249729 | 11390 | Lce3b | NM_025501.3 | chr3:92932978-92934096 |
| 11294 | Krtcap3 | NM_027221.3 | chr5:31251705-31253197 | 11391 | Lce3c | NM_033175.3 | chr3:92944485-92945730 |
| 11295 | Krtdap | NM_001033131.3 | chr7:30787904-30791083 | 11392 | Lce3d | NM_001270426.1 | chr3:92957389-92958574 |
| 11296 | Ksr1 | NM_013571.2 | chr11:79014800-79146354 | 11393 | Lce3e | NM_001254725.1 | chr3:92967060-92968281 |
| 11297 | Ksr2 | NM_001312914.1 | chr5:117413770-117775004 | 11394 | Lce3f | NM_001018079.1 | chr3:92992225-92993426 |
| 11298 | Kti12 | NM_029571.2 | chr4:108847856-108849412 | 11395 | Lce6a | NM_001166172.1 | chr3:92620084-92621660 |
| 11299 | Ktn1 | NM_008477.2 | chr14:47663755-47736564 | 11396 | Lck | NM_001162432.1 | chr4:129548343-129558372 |
| 11300 | Kxd1 | NM_029366.2 | chr8:70513395-70523180 | 11397 | Lclat1 | NM_001081071.2 | chr17:73107984-73243366 |
| 11301 | Ky | NM_024291.3 | chr9:102506137-102546239 | 11398 | Lcmt1 | NM_025304.3 | chr7:123377981-123430358 |
| 11302 | Kynu | NM_001289593.1 | chr2:43555324-43680216 | 11399 | Lcmt2 | NM_177846.3 | chr2:121137291-121140698 |
| 11303 | L1cam | NM_008478.3 | chrX:73853779-73880834 | 11400 | Lcn10 | NM_178036.4 | chr2:25682725-25686080 |
| 11304 | L1td1 | NM_001081202.1 | chr4:98726753-98738486 | 11401 | Lcn11 | NM_001100455.2 | chr2:25777016-25780279 |
| 11305 | L2hgdh | NM_145443.2 | chr12:69690435-69724874 | 11402 | Lcn12 | NM_029958.1 | chr2:25490844-25493911 |
| 11306 | L3hypdh | NM_026038.2 | chr12:72073427-72085313 | 11403 | Lcn2 | NM_008491.1 | chr2:32384636-32387739 |
| 11307 | L3mbtl1 | NM_001081338.1 | chr2:162943464-162974522 | 11404 | Lcn3 | NM_010694.1 | chr2:25765568-25768099 |
| 11308 | L3mbtl2 | NM_001289711.1 | chr15:81663888-81688315 | 11405 | Lcn4 | NM_010695.1 | chr2:26667673-26671282 |
| 11309 | L3mbtl3 | NM_172787.2 | chr10:26275451-26375185 | 11406 | Lcn5 | NM_001042630.2 | chr2:25657951-25662230 |
| 11310 | L3mbtl4 | NM_177278.5 | chr17:68273796-68780086 | 11407 | Lcn6 | NM_001276448.1 | chr2:25676785-25681607 |
| 11311 | l7Rn6 | NM_001291286.1 | chr7:89918684-89941204 | 11408 | Lcn8 | NM_033145.1 | chr2:25653117-25656216 |
| 11312 | Lacc1 | NM_172488.2 | chr14:77024200-77036617 | 11409 | Lcn9 | NM_029959.2 | chr2:25823152-25825537 |
| 11313 | Lace1 | NM_145743.2 | chr10:42312584-42478565 | 11410 | Lcor | NM_172154.4 | chr19:41549638-41559781 |
| 11314 | Lactb | NM_030717.1 | chr9:66955392-66975484 | 11411 | Lcorl | NM_001163073.1 | chr5:45730331-45857540 |
| 11315 | Lactb2 | NM_145381.2 | chr1:13625899-13660509 | 11412 | Lcp1 | NM_001247984.1 | chr14:75131122-75230842 |
| 11316 | Lactbl1 | NM_001243262.1 | chr4:136622620-136638110 | 11413 | Lcp2 | NM_010696.3 | chr11:34047200-34092280 |
| 11317 | Lad1 | NM_133664.3 | chr1:135818597-135833341 | 11414 | Lct | NM_001081078.2 | chr1:128284755-128328318 |
| 11318 | Lag3 | NM_008479.2 | chr6:124904358-124911705 | 11415 | Lctl | NM_145835.2 | chr9:64117146-64138118 |
| 11319 | Lage3 | NM_025410.2 | chrX:74352161-74353618 | 11416 | Ldb1 | NM_001113408.1 | chr19:46032600-46045211 |
| 11320 | Lair1 | NM_001113474.1 | chr7:4007072-4063204 | 11417 | Ldb2 | NM_001077398.2 | chr5:44472132-44799746 |
| 11321 | Lalba | NM_010679.1 | chr15:98480399-98482683 | 11418 | Ldb3 | NM_001039071.2 | chr14:34526698-34588681 |
| 11322 | Lama1 | NM_008480.2 | chr17:67697264-67822645 | 11419 | Ldha | NM_001136069.2 | chr7:46847081-46855627 |
| 11323 | Lama2 | NM_008481.2 | chr10:26981284-27616942 | 11420 | Ldhal6b | NM_175349.2 | chr17:5417322-5418767 |
| 11324 | Lama3 | NM_010680.1 | chr18:12334023-12583012 | 11421 | Ldhb | NM_008492.3 | chr6:142490248-142507957 |
| 11325 | Lama4 | NM_010681.4 | chr10:38965514-39110188 | 11422 | Ldhc | NM_013580.4 | chr7:46861262-46878142 |
| 11326 | Lama5 | NM_001081171.2 | chr2:180176372-180225859 | 11423 | Ldhd | NM_027570.3 | chr8:111626270-111630322 |
| 11327 | Lamb1 | NM_008482.2 | chr12:31265293-31329639 | 11424 | Ldlr | NM_001252658.1 | chr9:21723575-21749918 |
| 11328 | Lamb2 | NM_008483.3 | chr9:108479861-108490530 | 11425 | Ldlrad1 | NM_001081272.1 | chr4:107209179-107217914 |
| 11329 | Lamb3 | NM_001277928.1 | chr1:193302242-193343878 | 11426 | Ldlrad2 | NM_001033979.1 | chr4:137570875-137575180 |
| 11330 | Lamc1 | NM_010683.2 | chr1:153218921-153332786 | 11427 | Ldlrad3 | NM_001290784.1 | chr2:101950200-102186460 |
| 11331 | Lamc2 | NM_008485.3 | chr1:153122755-153186447 | 11428 | Ldlrad4 | NM_172631.3 | chr18:67933256-68255549 |
| 11332 | Lamc3 | NM_011836.3 | chr2:31887280-31946535 | 11429 | Ldlrap1 | NM_145554.2 | chr4:134745411-134768004 |
| 11333 | Lamp1 | NM_010684.2 | chr8:13159134-13175338 | 11430 | Ldoc1 | NM_001018087.1 | chrX:61709615-61710950 |
| 11334 | Lamp2 | NM_001017959.2 | chrX:38405048-38456460 | 11431 | Ldoc1l | NM_177630.3 | chr15:84553397-84557823 |
| 11335 | Lamp3 | NM_177356.3 | chr16:19653380-19706365 | 11432 | Leap2 | NM_153069.3 | chr11:53422180-53423136 |
| 11336 | Lamp5 | NM_029530.2 | chr2:136057926-136069917 | 11433 | Lect1 | NM_010701.3 | chr14:79637692-79662183 |
| 11337 | Lamtor1 | NM_025605.3 | chr7:101905836-101911903 | 11434 | Lect2 | NM_010702.2 | chr13:56542459-56548538 |
| 11338 | Lamtor2 | NM_031248.3 | chr3:88549818-88552927 | 11435 | Lef1 | NM_001276402.1 | chr3:131110296-131224357 |
| 11339 | Lamtor3 | NM_019920.2 | chr3:137918554-137928762 | 11436 | Lefty1 | NM_010094.3 | chr1:180935038-180938401 |
| 11340 | Lamtor4 | NM_001081108.2 | chr5:138255481-138259395 | 11437 | Lefty2 | NM_177099.4 | chr1:180893107-180899107 |
| 11341 | Lamtor5 | NM_026774.2 | chr3:107278857-107284081 | 11438 | Lekr1 | NM_001037923.4 | chr3:65666227-65686181 |
| 11342 | Lancl1 | NM_001190984.1 | chr1:67000516-67038834 | 11439 | Lelp1 | NM_027042.1 | chr3:92134993-92142754 |
| 11343 | Lancl2 | NM_133737.2 | chr6:57702454-57739449 | 11440 | Lemd1 | NM_001033250.4 | chr1:132191435-132257382 |
| 11344 | Lancl3 | NM_173414.3 | chrX:9199972-9268085 | 11441 | Lemd2 | NM_146075.2 | chr17:27189599-27204438 |
| 11345 | Lao1 | NM_133892.4 | chr4:118961966-118968910 | 11442 | Lemd3 | NM_001081193.2 | chr10:120923410-120979330 |
| 11346 | Lap3 | NM_024434.6 | chr5:45493373-45512691 | 11443 | Lenep | NM_020517.4 | chr3:89401895-89402650 |
| 11347 | Laptm4a | NM_008640.2 | chr12:8921306-8938741 | 11444 | Leng1 | NM_027203.3 | chr7:3660106-3665840 |
| 11348 | Laptm4b | NM_033521.3 | chr15:34238025-34284295 | 11445 | Leng8 | NM_172736.3 | chr7:4137055-4148173 |

Fig.21 - 60

| | | | | | | | |
|---|---|---|---|---|---|---|---|
| 11446 | Leng9 | NM_175529.3 | chr7:4148182-4149872 | 11543 | Lipt1 | NM_001037918.3 | chr1:37872205-37876298 |
| 11447 | Leo1 | NM_001039522.1 | chr9:75441523-75466432 | 11544 | Lipt2 | NM_026010.2 | chr7:100159276-100160931 |
| 11448 | Lep | NM_008493.3 | chr6:29060220-29073876 | 11545 | Litaf | NM_019980.2 | chr16:10959272-10993121 |
| 11449 | Lepr | NM_001122899.1 | chr4:101717406-101813667 | 11546 | Lix1 | NM_025681.2 | chr17:17402685-17459388 |
| 11450 | Lepre1 | NM_001042411.1 | chr4:119233828-119248977 | 11547 | Lix1l | NM_001163170.1 | chr3:96601132-96625352 |
| 11451 | Leprel1 | NM_173379.2 | chr16:25960322-26105784 | 11548 | Lkaaear1 | NM_199023.3 | chr2:181696794-181698442 |
| 11452 | Leprel2 | NM_013534.4 | chr6:124841094-124857687 | 11549 | Llgl1 | NM_001159404.1 | chr11:60699689-60714188 |
| 11453 | Leprel4 | NM_176830.2 | chr11:100408749-100414819 | 11550 | Llgl2 | NM_001252532.1 | chr11:115824057-115855780 |
| 11454 | Leprot | NM_175036.4 | chr4:101647782-101659358 | 11551 | Llph | NM_025431.2 | chr10:120227059-120232070 |
| 11455 | Leprotl1 | NM_026609.2 | chr8:34135571-34146739 | 11552 | Lman1 | NM_001172062.1 | chr18:65983686-66002635 |
| 11456 | Letm1 | NM_019694.1 | chr5:33741351-33782704 | 11553 | Lman1l | NM_199222.3 | chr9:57607032-57620774 |
| 11457 | Letm2 | NM_173012.3 | chr8:25578489-25597487 | 11554 | Lman2 | NM_025828.3 | chr13:55343832-55362783 |
| 11458 | Letmd1 | NM_134093.2 | chr15:100469033-100479252 | 11555 | Lman2l | NM_001013374.2 | chr1:36423185-36445271 |
| 11459 | Lfng | NM_008494.3 | chr5:140607340-140615545 | 11556 | Lmbr1 | NM_020295.3 | chr5:29229801-29378390 |
| 11460 | Lgals1 | NM_008495.2 | chr15:78926724-78930465 | 11557 | Lmbr1l | NM_029098.3 | chr15:98903920-98918098 |
| 11461 | Lgals12 | NM_019516.3 | chr19:7596660-7607176 | 11558 | Lmbrd1 | NM_026719.2 | chr1:24678543-24766301 |
| 11462 | Lgals2 | NM_025622.3 | chr15:78850859-78855529 | 11559 | Lmbrd2 | NM_177178.3 | chr15:9140569-9197450 |
| 11463 | Lgals3 | NM_001145953.1 | chr14:47373859-47386167 | 11560 | Lmcd1 | NM_144799.2 | chr6:112273757-112330423 |
| 11464 | Lgals3bp | NM_011150.2 | chr11:118392751-118401931 | 11561 | Lmf1 | NM_029624.4 | chr17:25579173-25662826 |
| 11465 | Lgals4 | NM_010706.2 | chr7:28833793-28841703 | 11562 | Lmf2 | NM_178919.4 | chr15:89351003-89355659 |
| 11466 | Lgals6 | NM_010707.2 | chr7:28834181-28841633 | 11563 | Lmln | NM_172823.2 | chr16:33062520-33125659 |
| 11467 | Lgals7 | NM_008496.4 | chr7:28864385-28866284 | 11564 | Lmna | NM_001002011.3 | chr3:88481147-88503352 |
| 11468 | Lgals8 | NM_001199043.1 | chr13:12439414-12461738 | 11565 | Lmnb1 | NM_010721.2 | chr18:56707812-56753424 |
| 11469 | Lgals9 | NM_001159301.1 | chr11:78962978-78984924 | 11566 | Lmnb2 | NM_010722.5 | chr10:80901362-80918245 |
| 11470 | Lgalsl | NM_173752.4 | chr11:20823354-20831108 | 11567 | Lmo1 | NM_057173.3 | chr7:109138564-109170519 |
| 11471 | Lgi1 | NM_020278.2 | chr19:38264781-38308939 | 11568 | Lmo2 | NM_001142335.1 | chr2:103970286-103981878 |
| 11472 | Lgi2 | NM_144945.3 | chr5:52533516-52566306 | 11569 | Lmo3 | NM_207222.2 | chr6:138364480-138581968 |
| 11473 | Lgi3 | NM_145219.4 | chr14:70530820-70538324 | 11570 | Lmo4 | NM_001161769.1 | chr3:144188529-144202396 |
| 11474 | Lgi4 | NM_144556.2 | chr7:31059934-31070935 | 11571 | Lmo7 | NM_201529.2 | chr14:101729927-101934693 |
| 11475 | Lgmn | NM_011175.2 | chr12:102394097-102439697 | 11572 | Lmod1 | NM_053106.2 | chr1:135324812-135368065 |
| 11476 | Lgr4 | NM_172671.2 | chr2:109917646-110014257 | 11573 | Lmod2 | NM_053098.2 | chr6:24597770-24605414 |
| 11477 | Lgr5 | NM_010195.2 | chr10:115450313-115587780 | 11574 | Lmod3 | NM_001081157.1 | chr6:97238527-97252780 |
| 11478 | Lgr6 | NM_001033409.3 | chr1:134986352-135105276 | 11575 | Lmtk2 | NM_001081109.1 | chr5:144100435-144188204 |
| 11479 | Lgsn | NM_153601.1 | chr1:31176434-31204725 | 11576 | Lmtk3 | NM_001005511.3 | chr7:45783946-45804142 |
| 11480 | Lhb | NM_008497.2 | chr7:45420945-45421854 | 11577 | Lmx1a | NM_033652.5 | chr1:167689557-167848733 |
| 11481 | Lhcgr | NM_013582.2 | chr17:88741548-88791976 | 11578 | Lmx1b | NM_010725.2 | chr2:33564535-33640511 |
| 11482 | Lhfp | NM_175386.3 | chr3:53041546-53261679 | 11579 | Lnp | NM_001110209.1 | chr2:74514836-74578948 |
| 11483 | Lhfpl1 | NM_178358.3 | chrX:145290358-145348894 | 11580 | Lnpep | NM_172827.3 | chr17:17527722-17624489 |
| 11484 | Lhfpl2 | NM_172589.2 | chr13:94057795-94195409 | 11581 | Lnx1 | NM_001159577.1 | chr5:74597103-74702903 |
| 11485 | Lhfpl3 | NM_001081231.2 | chr5:22746192-23275597 | 11582 | Lnx2 | NM_080795.4 | chr5:147016654-147076572 |
| 11486 | Lhfpl4 | NM_177763.3 | chr6:113168086-113195384 | 11583 | LOC100038947 | NM_001173459.2 | chr3:15795146-15848487 |
| 11487 | Lhfpl5 | NM_026571.2 | chr17:28575718-28583593 | 11584 | LOC100040786 | NM_001160129.1 | chrY:54185856-66742170 |
| 11488 | Lhpp | NM_029609.1 | chr7:132610642-132706419 | 11585 | LOC100043315 | NR_015482.1 | chr15:95097621-95149318 |
| 11489 | Lhx1 | NM_008498.2 | chr11:84519378-84525534 | 11586 | LOC100048884 | NM_001199333.1 | chr4:61670177-61674094 |
| 11490 | Lhx1os | NR_038057.1 | chr11:84525659-84535831 | 11587 | LOC100502896 | NM_001277512.1 | chr4:73650909-73657292 |
| 11491 | Lhx2 | NM_001290646.1 | chr2:38339280-38369737 | 11588 | LOC100503280 | NM_001251890.1 | chrX:74966843-74969125 |
| 11492 | Lhx3 | NM_001039653.2 | chr2:26200211-26206575 | 11589 | LOC100503496 | NR_040680.1 | chr11:109440077-109453359 |
| 11493 | Lhx4 | NM_010712.2 | chr1:155701693-155742027 | 11590 | LOC100503676 | NR_103491.1 | chr8:84913334-84922915 |
| 11494 | Lhx5 | NM_008499.5 | chr5:120431885-120441457 | 11591 | LOC100504039 | NR_102724.1 | chrX:53686526-53691325 |
| 11495 | Lhx6 | NM_001083125.1 | chr2:36081952-36105408 | 11592 | LOC100504608 | NM_001205036.1 | chr10:127032904-127041394 |
| 11496 | Lhx8 | NM_010713.2 | chr3:154306293-154330560 | 11593 | LOC100504703 | NR_040660.1 | chr10:127070480-127071101 |
| 11497 | Lhx9 | NM_001025565.2 | chr1:138825185-138842444 | 11594 | LOC100505025 | NR_105062.1 | chr7:73310379-73315193 |
| 11498 | Lias | NM_024471.5 | chr5:65391496-65410423 | 11595 | LOC100861615 | NM_001270812.1 | chr14:6259746-6287250 |
| 11499 | Lif | NM_001039537.2 | chr11:4266795-4272514 | 11596 | LOC100861978 | NM_001267703.1 | chr4:16836-22237 |
| 11500 | Lifr | NM_001113386.1 | chr15:7129571-7185343 | 11597 | LOC100862015 | NM_001277531.1 | chr4:73635785-73642163 |
| 11501 | Lig1 | NM_001083188.1 | chr7:13279264-13311427 | 11598 | LOC100862268 | NR_105029.1 | chr3:127161304-127168290 |
| 11502 | Lig3 | NM_001291245.1 | chr11:82781108-82804274 | 11599 | LOC101055769 | NR_105031.1 | chr4:59260050-59269144 |
| 11503 | Lig4 | NM_176953.3 | chr8:9970019-9976323 | 11600 | LOC101055863 | NM_001277487.1 | chr4:73605543-73687560 |
| 11504 | Lilra5 | NM_001081239.2 | chr7:4237753-4243463 | 11601 | LOC101056043 | NR_105032.1 | chr5:110429874-110431955 |
| 11505 | Lilra6 | NM_011090.2 | chr7:3908279-3915501 | 11602 | LOC101056136 | NR_105055.1 | chr14:42254658-42260048 |
| 11506 | Lilrb4 | NM_001291894.1 | chr10:51490897-51496611 | 11603 | LOC101056149 | NR_105041.1 | chr13:34652922-34666774 |
| 11507 | Lim2 | NM_177693.3 | chr7:43430100-43435991 | 11604 | LOC101056236 | NR_105054.1 | chr16:92377070-92382683 |
| 11508 | Lima1 | NM_001113545.1 | chr15:99778467-99875456 | 11605 | LOC101243624 | NR_102297.1 | chr3:132842201-132878596 |
| 11509 | Limch1 | NM_001001980.2 | chr5:66745839-67057159 | 11606 | LOC101669761 | NR_103513.1 | chr16:31986830-31987791 |
| 11510 | Limd1 | NM_013860.2 | chr9:123478700-123521552 | 11607 | LOC102308570 | NM_001286103.1 | chr19:8883877-8890759 |
| 11511 | Limd2 | NM_172397.3 | chr11:106156255-106160142 | 11608 | LOC102631757 | NR_110502.1 | chr17:31152751-31163052 |
| 11512 | Lime1 | NM_023684.2 | chr2:181381234-181383628 | 11609 | LOC102632423 | NR_110507.1 | chr6:118300166-118308719 |
| 11513 | Limk1 | NM_010717.3 | chr5:134656038-134688590 | 11610 | LOC102632430 | NR_110508.1 | chr16:22874982-22894386 |
| 11514 | Limk2 | NM_001034030.2 | chr11:3343296-3356141 | 11611 | LOC102633035 | NR_110509.1 | chr9:101342193-101348017 |
| 11515 | Lims1 | NM_001193303.1 | chr10:58394372-58424691 | 11612 | LOC102633315 | NR_110510.1 | chr8:25548672-25556083 |
| 11516 | Lims2 | NM_144862.3 | chr18:31931506-31958619 | 11613 | LOC102634101 | NR_110511.1 | chr10:67005074-67041326 |
| 11517 | Lin28a | NM_145833.1 | chr4:134003329-134018816 | 11614 | LOC102634401 | NR_110447.1 | chr2:74752683-74753742 |
| 11518 | Lin28b | NM_001031772.2 | chr10:45376618-45470201 | 11615 | LOC102634431 | NR_110513.1 | chr9:8644077-8740988 |
| 11519 | Lin37 | NM_001290569.1 | chr7:30555440-30559646 | 11616 | LOC102634753 | NR_110516.1 | chr3:99049821-99053742 |
| 11520 | Lin52 | NM_173756.4 | chr12:84451507-84531533 | 11617 | LOC102635087 | NR_110517.1 | chr9:71168775-71208122 |
| 11521 | Lin54 | NM_001115010.1 | chr5:100442034-100498573 | 11618 | LOC102636514 | NR_110474.1 | chr8:57304413-57320859 |
| 11522 | Lin7a | NM_001033223.2 | chr10:107271830-107425143 | 11619 | LOC106740 | NR_027905.1 | chr17:14947735-14948823 |
| 11523 | Lin7b | NM_011698.1 | chr7:45367890-45370564 | 11620 | LOC171588 | NR_038065.1 | chr2:144465175-144466732 |
| 11524 | Lin7c | NM_011699.3 | chr2:109890877-109900936 | 11621 | LOC381967 | NR_103487.1 | chr7:99260480-99267218 |
| 11525 | Lin9 | NM_001103182.2 | chr1:180641149-180690687 | 11622 | LOC666331 | NM_001256318.1 | chr11:101610699-101615956 |
| 11526 | Lincrna-cox2 | NR_110420.1 | chr1:150159042-150164948 | 11623 | Loh12cr1 | NM_001170479.1 | chr6:134639511-134711184 |
| 11527 | Lingo1 | NM_181074.4 | chr9:56618474-56685253 | 11624 | Lonp1 | NM_028782.2 | chr17:56614297-56626903 |
| 11528 | Lingo2 | NM_001165999.1 | chr4:35706647-36951744 | 11625 | Lonp2 | NM_001168591.1 | chr8:86651568-86716638 |
| 11529 | Lingo3 | NM_001013758.2 | chr10:80832800-80844039 | 11626 | Lonrf1 | NM_001081150.1 | chr8:36216063-36249516 |
| 11530 | Lingo4 | NM_177250.2 | chr3:94399218-94404501 | 11627 | Lonrf2 | NM_001029878.1 | chr1:38794508-38821215 |
| 11531 | Lins | NM_001191001.1 | chr7:66689888-66717256 | 11628 | Lonrf3 | NM_028894.1 | chrX:36328408-36366856 |
| 11532 | Lipa | NM_001111100.1 | chr19:34492315-34527474 | 11629 | Lor | NM_008508.2 | chr3:92080270-92083142 |
| 11533 | Lipc | NM_008280.2 | chr9:70798127-70934808 | 11630 | Lox | NM_001286181.1 | chr18:52516059-52529721 |
| 11534 | Lipe | NM_001039507.2 | chr7:25379526-25390112 | 11631 | Loxhd1 | NM_172834.2 | chr18:77281957-77442257 |
| 11535 | Lipf | NM_026334.3 | chr19:33961247-33976813 | 11632 | Loxl1 | NM_010729.3 | chr9:58287722-58313212 |
| 11536 | Lipg | NM_010720.3 | chr18:74939321-74961263 | 11633 | Loxl2 | NM_033325.2 | chr14:69609475-69695834 |
| 11537 | Liph | NM_001083894.1 | chr16:21953817-21995542 | 11634 | Loxl3 | NM_013586.4 | chr6:83034223-83052564 |
| 11538 | Lipi | NM_001252513.1 | chr16:75540513-75586061 | 11635 | Loxl4 | NM_001164311.1 | chr19:42592278-42612806 |
| 11539 | Lipk | NM_001205349.1 | chr19:34008253-34047903 | 11636 | Lpar1 | NM_001290486.1 | chr4:58435251-58499403 |
| 11540 | Lipm | NM_023903.1 | chr19:34100942-34122687 | 11637 | Lpar2 | NM_020028.3 | chr8:69822564-69831102 |
| 11541 | Lipn | NM_027340.2 | chr19:34067357-34084918 | 11638 | Lpar3 | NM_022983.4 | chr3:146220960-146286214 |
| 11542 | Lipo1 | NM_001013770.3 | chr19:33555280-33590311 | 11639 | Lpar4 | NM_175271.4 | chrX:106920624-106933899 |

Fig.21 - 61

| | | | | | | | |
|---|---|---|---|---|---|---|---|
| 11640 | Lpar5 | NM_001163268.1 | chr6:125071276-125082472 | 11737 | Lrrc55 | NM_001033346.2 | chr2:85188070-85196699 |
| 11641 | Lpar6 | NM_175116.4 | chr14:73237890-73240358 | 11738 | Lrrc56 | NM_001172064.1 | chr7:141194129-141210055 |
| 11642 | Lpcat1 | NM_145376.5 | chr13:73467382-73514538 | 11739 | Lrrc57 | NM_001159609.1 | chr2:120604237-120609508 |
| 11643 | Lpcat2 | NM_173014.1 | chr8:92855349-92919279 | 11740 | Lrrc58 | NM_177093.3 | chr16:37868399-37888857 |
| 11644 | Lpcat2b | NM_027599.3 | chr5:107431548-107435039 | 11741 | Lrrc59 | NM_133807.1 | chr11:94629823-94645216 |
| 11645 | Lpcat3 | NM_145130.2 | chr6:124663103-124704716 | 11742 | Lrrc6 | NM_019457.2 | chr15:66379857-66500910 |
| 11646 | Lpcat4 | NM_207206.2 | chr2:112239840-112247111 | 11743 | Lrrc61 | NM_001110160.1 | chr6:48554798-48570722 |
| 11647 | Lpgat1 | NM_001134829.1 | chr1:191718394-191784257 | 11744 | Lrrc63 | NM_027581.1 | chr14:75084302-75130883 |
| 11648 | Lphn1 | NM_181039.2 | chr8:83900097-83941954 | 11745 | Lrrc66 | NM_153568.1 | chr5:73606641-73632421 |
| 11649 | Lphn2 | NM_001081298.1 | chr3:148815585-148954635 | 11746 | Lrrc69 | NM_028499.2 | chr4:14665753-14796052 |
| 11650 | Lphn3 | NM_198702.2 | chr5:81021592-81795730 | 11747 | Lrrc7 | NM_001081358.2 | chr3:158082894-158562221 |
| 11651 | Lpin1 | NM_001130412.1 | chr12:16535668-16589770 | 11748 | Lrrc71 | NM_028971.1 | chr3:87736922-87748623 |
| 11652 | Lpin2 | NM_001164885.1 | chr17:71204653-71249818 | 11749 | Lrrc72 | NM_001177877.1 | chr12:36209562-36253398 |
| 11653 | Lpin3 | NM_001199118.1 | chr2:160888167-160906000 | 11750 | Lrrc73 | NM_001111142.1 | chr17:46254164-46257316 |
| 11654 | Lpl | NM_008509.2 | chr8:68880554-68906932 | 11751 | Lrrc74 | NM_001195767.1 | chr12:86734368-86763795 |
| 11655 | Lpo | NM_080420.2 | chr11:87806427-87826114 | 11752 | Lrrc75a | NM_198861.1 | chr11:62604883-62648523 |
| 11656 | Lpp | NM_001145952.1 | chr16:24448090-24992578 | 11753 | Lrrc75b | NM_198860.2 | chr10:75550124-75560330 |
| 11657 | Lpxn | NM_134152.3 | chr19:12798608-12833808 | 11754 | Lrrc8a | NM_177725.4 | chr2:30237768-30263790 |
| 11658 | Lrat | NM_023624.4 | chr3:82892581-82903974 | 11755 | Lrrc8b | NM_001033550.2 | chr5:105415774-105486189 |
| 11659 | Lrba | NM_001077687.1 | chr3:86224689-86746710 | 11756 | Lrrc8c | NM_133897.2 | chr5:105519470-105608954 |
| 11660 | Lrch1 | NM_001033439.3 | chr14:74754672-74947877 | 11757 | Lrrc8d | NM_001122768.1 | chr5:105699968-105815215 |
| 11661 | Lrch2 | NM_001081173.1 | chrX:147471693-147554081 | 11758 | Lrrc8e | NM_028175.2 | chr8:4226826-4237470 |
| 11662 | Lrch3 | NM_001081255.1 | chr16:32914099-33016029 | 11759 | Lrrc9 | NM_001142728.1 | chr12:72441865-72510565 |
| 11663 | Lrch4 | NM_001168652.1 | chr5:137629122-137641099 | 11760 | Lrrcc1 | NM_001163579.1 | chr3:14533787-14568303 |
| 11664 | Lrcol1 | NM_001310709.1 | chr5:110344507-110356094 | 11761 | Lrrd1 | NM_172879.3 | chr5:3845172-3866596 |
| 11665 | Lrfn1 | NM_001141921.1 | chr7:28452237-28467548 | 11762 | Lrrfip1 | NM_001111311.1 | chr1:91053443-91117322 |
| 11666 | Lrfn2 | NM_027452.3 | chr17:48932581-49097590 | 11763 | Lrrfip2 | NM_001164838.1 | chr9:111118110-111225668 |
| 11667 | Lrfn3 | NM_175478.2 | chr7:30355513-30362772 | 11764 | Lrriq1 | NM_001163559.1 | chr10:103063197-103236322 |
| 11668 | Lrfn4 | NM_153388.4 | chr19:4611783-4615667 | 11765 | Lrriq3 | NM_028938.2 | chr3:155093433-155194278 |
| 11669 | Lrfn5 | NM_178714.5 | chr12:61523166-61845258 | 11766 | Lrriq4 | NM_001290510.1 | chr3:30647885-30672431 |
| 11670 | Lrg1 | NM_029796.2 | chr17:56119677-56121946 | 11767 | Lrrk1 | NM_146191.3 | chr7:66258746-66388341 |
| 11671 | Lrguk | NM_028886.1 | chr6:34029447-34134034 | 11768 | Lrrk2 | NM_025730.3 | chr15:91673223-91816124 |
| 11672 | Lrif1 | NM_001039478.1 | chr3:106684986-106736576 | 11769 | Lrrn1 | NM_008516.4 | chr6:107529725-107570228 |
| 11673 | Lrig1 | NM_008377.2 | chr6:94604528-94700145 | 11770 | Lrrn2 | NM_010732.4 | chr1:132880354-132940005 |
| 11674 | Lrig2 | NM_001025067.2 | chr3:104453982-104511918 | 11771 | Lrrn3 | NM_001271708.1 | chr12:41451667-41486057 |
| 11675 | Lrig3 | NM_177152.5 | chr10:125966218-126015359 | 11772 | Lrrn4 | NM_177303.4 | chr2:132868515-132880862 |
| 11676 | Lrit1 | NM_146245.2 | chr14:37054829-37064938 | 11773 | Lrrn4cl | NM_001013019.2 | chr19:8850784-8853909 |
| 11677 | Lrit2 | NM_173418.3 | chr14:37068048-37073735 | 11774 | Lrrtm1 | NM_028880.3 | chr6:77242716-77245517 |
| 11678 | Lrit3 | NM_001287224.1 | chr3:129788290-129804030 | 11775 | Lrrtm2 | NM_178005.4 | chr18:35209005-35215024 |
| 11679 | Lrmp | NM_001281980.1 | chr6:145115635-145174928 | 11776 | Lrrtm3 | NM_178678.4 | chr10:63928496-64090255 |
| 11680 | Lrp1 | NM_008512.2 | chr10:127538157-127621148 | 11777 | Lrrtm4 | NM_001134743.1 | chr6:80018876-80810143 |
| 11681 | Lrp10 | NM_022993.3 | chr14:54464146-54470291 | 11778 | Lrsam1 | NM_199302.2 | chr2:32925214-32961255 |
| 11682 | Lrp11 | NM_172784.3 | chr10:7589799-7625477 | 11779 | Lrtm1 | NM_176920.4 | chr14:29018207-29033642 |
| 11683 | Lrp12 | NM_172814.3 | chr15:39870602-39943757 | 11780 | Lrtm2 | NM_001172207.1 | chr6:119315132-119329204 |
| 11684 | Lrp1b | NM_053011.2 | chr2:40596772-42653598 | 11781 | Lrwd1 | NM_027891.4 | chr5:136123065-136136074 |
| 11685 | Lrp2 | NM_001081088.1 | chr2:69424334-69586067 | 11782 | Lsamp | NM_175548.3 | chr16:41533341-42146213 |
| 11686 | Lrp2bp | NM_026278.3 | chr8:46010601-46029476 | 11783 | Lsg1 | NM_178069.5 | chr16:30561368-30587589 |
| 11687 | Lrp3 | NM_001024707.2 | chr7:35200877-35215345 | 11784 | Lsm1 | NM_026032.1 | chr8:25785590-25803975 |
| 11688 | Lrp4 | NM_001145857.1 | chr2:91457530-91479287 | 11785 | Lsm10 | NM_001163266.1 | chr4:126096652-126098584 |
| 11689 | Lrp5 | NM_008513.3 | chr19:3584824-3686564 | 11786 | Lsm11 | NM_028185.2 | chr11:45928268-45944935 |
| 11690 | Lrp6 | NM_008514.4 | chr6:134446477-134566913 | 11787 | Lsm12 | NM_172947.3 | chr11:102163488-102185256 |
| 11691 | Lrp8 | NM_001080926.1 | chr4:107802258-107876840 | 11788 | Lsm14a | NM_025948.2 | chr7:34344719-34389540 |
| 11692 | Lrpap1 | NM_013587.2 | chr5:35091505-35105697 | 11789 | Lsm14b | NM_177727.4 | chr2:180024986-180035461 |
| 11693 | Lrpprc | NM_028233.2 | chr17:84705246-84790786 | 11790 | Lsm2 | NM_001110101.2 | chr17:34981853-34985893 |
| 11694 | Lrr1 | NM_001081406.1 | chr12:69168813-69179010 | 11791 | Lsm3 | NM_026309.2 | chr6:91516034-91522620 |
| 11695 | Lrrc1 | NM_001146048.1 | chr9:77430822-77544852 | 11792 | Lsm4 | NM_015816.4 | chr8:70673230-70678752 |
| 11696 | Lrrc10 | NM_146242.2 | chr10:117045340-117046768 | 11793 | Lsm5 | NM_025520.3 | chr6:56701062-56704699 |
| 11697 | Lrrc10b | NM_001111140.2 | chr19:10455370-10457447 | 11794 | Lsm6 | NM_001191004.1 | chr8:78804867-78821152 |
| 11698 | Lrrc14 | NM_145471.2 | chr15:76710739-76715091 | 11795 | Lsm7 | NM_025349.2 | chr10:80852824-80855209 |
| 11699 | Lrrc14b | NM_001033042.3 | chr13:74359581-74364000 | 11796 | Lsm8 | NM_133939.1 | chr6:18848634-18854052 |
| 11700 | Lrrc15 | NM_028973.2 | chr16:30269301-30283254 | 11797 | Lsmem1 | NM_001033437.2 | chr12:40176385-40199315 |
| 11701 | Lrrc16a | NM_026825.3 | chr13:24012483-24280790 | 11798 | Lsp1 | NM_001136071.2 | chr7:142460811-142494868 |
| 11702 | Lrrc16b | NM_001024645.1 | chr14:55491092-55508264 | 11799 | Lsr | NM_001164184.1 | chr7:30957769-30973469 |
| 11703 | Lrrc17 | NM_028977.1 | chr5:21543526-21575902 | 11800 | Lss | NM_146006.2 | chr10:76531604-76557139 |
| 11704 | Lrrc18 | NM_001146021.1 | chr14:32991381-33009398 | 11801 | Lst1 | NM_010734.2 | chr17:35185094-35188440 |
| 11705 | Lrrc19 | NM_175305.4 | chr4:94636659-94650144 | 11802 | Lta | NM_010735.2 | chr17:35203164-35205351 |
| 11706 | Lrrc2 | NM_028838.2 | chr9:110951544-110984064 | 11803 | Lta4h | NM_008517.2 | chr10:93453395-93484896 |
| 11707 | Lrrc20 | NM_153542.1 | chr10:61475832-61582228 | 11804 | Ltb | NM_008518.2 | chr17:35194506-35196305 |
| 11708 | Lrrc23 | NM_001302555.1 | chr6:124769869-124780341 | 11805 | Ltb4r1 | NM_008519.2 | chr14:55765961-55768492 |
| 11709 | Lrrc24 | NM_198119.2 | chr15:76715275-76722173 | 11806 | Ltb4r2 | NM_020490.2 | chr14:55761427-55763229 |
| 11710 | Lrrc25 | NM_153074.3 | chr8:70616843-70620850 | 11807 | Ltbp1 | NM_019919.3 | chr17:75005528-75392967 |
| 11711 | Lrrc26 | NM_146117.2 | chr2:25289910-25291193 | 11808 | Ltbp2 | NM_013589.3 | chr12:84783211-84876495 |
| 11712 | Lrrc27 | NM_001143755.1 | chr7:139213274-139229012 | 11809 | Ltbp3 | NM_008520.2 | chr19:5740903-5758532 |
| 11713 | Lrrc28 | NM_027413.1 | chr7:67594398-67645236 | 11810 | Ltbp4 | NM_001113549.1 | chr7:27305140-27333648 |
| 11714 | Lrrc29 | NM_177449.3 | chr8:105312339-105326276 | 11811 | Ltbr | NM_010736.3 | chr6:125306570-125313870 |
| 11715 | Lrrc3 | NM_145152.4 | chr10:77897575-77902536 | 11812 | Ltc4s | NM_008521.2 | chr11:50236460-50238532 |
| 11716 | Lrrc30 | NM_001033340.3 | chr17:67630964-67632723 | 11813 | Ltf | NM_008522.3 | chr9:111019291-111042766 |
| 11717 | Lrrc32 | NM_001113379.1 | chr7:98494221-98501830 | 11814 | Ltk | NM_008523.2 | chr2:119751325-119758525 |
| 11718 | Lrrc34 | NM_027941.1 | chr3:30624266-30647818 | 11815 | Ltn1 | NM_001081068.1 | chr16:87376650-87432606 |
| 11719 | Lrrc36 | NM_001033371.3 | chr8:105427639-105464086 | 11816 | Ltv1 | NM_181470.4 | chr10:13178637-13193137 |
| 11720 | Lrrc38 | NM_001162983.1 | chr4:143349749-143371028 | 11817 | Luc7l | NM_025881.3 | chr17:26252909-26280730 |
| 11721 | Lrrc39 | NM_027321.3 | chr3:116562972-116573112 | 11818 | Luc7l2 | NM_001170848.1 | chr6:38551443-38609470 |
| 11722 | Lrrc3b | NM_146052.4 | chr14:15357515-15438987 | 11819 | Luc7l3 | NM_026313.1 | chr11:94291138-94321911 |
| 11723 | Lrrc4 | NM_138682.2 | chr6:28828125-28831747 | 11820 | Lum | NM_008524.2 | chr10:97565500-97572703 |
| 11724 | Lrrc40 | NM_001289524.1 | chr3:158036681-158067090 | 11821 | Lurap1 | NM_026547.1 | chr4:116136727-116144616 |
| 11725 | Lrrc41 | NM_153521.2 | chr4:116075268-116097109 | 11822 | Lurap1l | NM_026821.5 | chr4:80910685-80954301 |
| 11726 | Lrrc42 | NM_029985.2 | chr4:107233513-107253533 | 11823 | Luzp1 | NM_024452.2 | chr4:136469760-136549318 |
| 11727 | Lrrc43 | NM_001033461.3 | chr5:123489324-123508205 | 11824 | Luzp2 | NM_178705.5 | chr7:54835244-55268888 |
| 11728 | Lrrc45 | NM_153545.2 | chr11:120713952-120721127 | 11825 | Luzp4 | NM_001114383.1 | chrX:148882575-148924139 |
| 11729 | Lrrc46 | NM_027026.2 | chr11:97034601-97041369 | 11826 | Lxn | NM_016753.4 | chr3:67457998-67463907 |
| 11730 | Lrrc47 | NM_201226.1 | chr4:154011802-154021512 | 11827 | Ly6a | NM_001271416.1 | chr15:74994876-74998031 |
| 11731 | Lrrc48 | NM_029044.2 | chr11:60353379-60394333 | 11828 | Ly6c1 | NM_001252055.1 | chr15:75045014-75048837 |
| 11732 | Lrrc49 | NM_001146046.1 | chr9:60587234-60688134 | 11829 | Ly6c2 | NM_001099217.1 | chr15:75108160-75111949 |
| 11733 | Lrrc4b | NM_198250.1 | chr7:44442486-44463344 | 11830 | Ly6d | NM_010742.1 | chr15:74762055-74763567 |
| 11734 | Lrrc4c | NM_001289742.1 | chr2:96318168-97631664 | 11831 | Ly6e | NM_001164036.1 | chr15:74955070-74959905 |
| 11735 | Lrrc51 | NM_001162973.1 | chr7:101912988-101933857 | 11832 | Ly6f | NM_008530.2 | chr15:75268420-75272234 |
| 11736 | Lrrc52 | NM_001013382.2 | chr1:167445674-167466780 | 11833 | Ly6g5b | NM_148939.2 | chr17:35113945-35115400 |

Fig.21 - 62

| | | | |
|---|---|---|---|
| 11834 | Ly6g5c | NM_148947.1 | chr17:35108299-35111953 |
| 11835 | Ly6g6c | NM_023463.3 | chr17:35067324-35070048 |
| 11836 | Ly6g6d | NM_033478.2 | chr17:35071347-35074464 |
| 11837 | Ly6g6e | NM_027366.1 | chr17:35076941-35078804 |
| 11838 | Ly6g6f | NM_001163192.1 | chr17:35080537-35085595 |
| 11839 | Ly6h | NM_001135688.1 | chr15:75564744-75566856 |
| 11840 | Ly6i | NM_020498.2 | chr15:74979811-74983430 |
| 11841 | Ly6k | NM_029627.2 | chr15:74796873-74799968 |
| 11842 | Ly75 | NM_013825.3 | chr2:60293759-60383231 |
| 11843 | Ly86 | NM_010745.2 | chr13:37345344-37419036 |
| 11844 | Ly9 | NM_001277968.1 | chr1:171588612-171607410 |
| 11845 | Ly96 | NM_001159711.1 | chr1:16688455-16709605 |
| 11846 | Lyar | NM_025281.3 | chr5:38220481-38234306 |
| 11847 | Lyg1 | NM_027111.3 | chr1:37946737-37957759 |
| 11848 | Lyg2 | NM_001033427.3 | chr1:37905922-37916493 |
| 11849 | Lyl1 | NM_008535.2 | chr8:84701456-84704716 |
| 11850 | Lyn | NM_001111096.1 | chr4:3678120-3791612 |
| 11851 | Lynx1 | NM_011838.4 | chr15:74747855-74752979 |
| 11852 | Lypd1 | NM_145100.4 | chr1:125867621-125912214 |
| 11853 | Lypd2 | NM_026671.1 | chr15:74732251-74734313 |
| 11854 | Lypd3 | NM_133743.1 | chr7:24636569-24641118 |
| 11855 | Lypd4 | NM_182785.3 | chr7:24864619-24869691 |
| 11856 | Lypd5 | NM_029806.1 | chr7:24349223-24353833 |
| 11857 | Lypd6 | NM_177139.5 | chr2:50066428-50193569 |
| 11858 | Lypd6b | NM_027990.3 | chr2:49787685-49948846 |
| 11859 | Lypd8 | NM_001083884.1 | chr11:58379031-58390541 |
| 11860 | Lypla1 | NM_008866.2 | chr1:4807892-4846735 |
| 11861 | Lypla2 | NM_011942.1 | chr4:135968224-135972594 |
| 11862 | Lyplal1 | NM_146106.2 | chr1:186087731-186117310 |
| 11863 | Lyrm1 | NM_001285959.1 | chr7:119895860-119916756 |
| 11864 | Lyrm2 | NM_175364.4 | chr4:32800258-32802254 |
| 11865 | Lyrm4 | NM_201358.2 | chr13:35978796-36117357 |
| 11866 | Lyrm5 | NM_001163628.1 | chr6:145211133-145216542 |
| 11867 | Lyrm7 | NM_029327.3 | chr11:54839288-54860591 |
| 11868 | Lyrm7os | NR_040284.1 | chr11:54819918-54824967 |
| 11869 | Lyrm9 | NM_001076681.2 | chr11:78826594-78843899 |
| 11870 | Lysmd1 | NM_153121.2 | chr3:95134087-95139525 |
| 11871 | Lysmd2 | NM_027309.2 | chr9:75625731-75637773 |
| 11872 | Lysmd3 | NM_030257.1 | chr13:81657805-81671890 |
| 11873 | Lysmd4 | NM_001191051.1 | chr7:67222615-67228468 |
| 11874 | Lyst | NM_010748.2 | chr13:13590408-13777440 |
| 11875 | Lyve1 | NM_053247.4 | chr7:110850606-110862953 |
| 11876 | Lyz1 | NM_013590.4 | chr10:117287794-117292868 |
| 11877 | Lyz2 | NM_017372.3 | chr10:117277540-117282272 |
| 11878 | Lyzl1 | NM_026092.3 | chr18:4165831-4182236 |
| 11879 | Lyzl4 | NM_026915.2 | chr9:121577842-121641566 |
| 11880 | Lyzl4os | NR_040740.1 | chr9:121587019-121614255 |
| 11881 | Lyzl6 | NM_027083.1 | chr11:103631074-103638874 |
| 11882 | Lzic | NM_026963.5 | chr4:149485332-149496667 |
| 11883 | Lztfl1 | NM_033322.2 | chr9:123697592-123717557 |
| 11884 | Lztr1 | NM_025808.3 | chr16:17508970-17526330 |
| 11885 | Lzts1 | NM_199364.2 | chr8:69135502-69140953 |
| 11886 | Lzts2 | NM_001130525.1 | chr19:45015209-45027104 |
| 11887 | Lzts3 | NM_001291027.1 | chr2:130632764-130642844 |
| 11888 | M1ap | NM_033079.2 | chr6:82946921-83030309 |
| 11889 | M6pr | NM_010749.6 | chr6:122309009-122317677 |
| 11890 | Maats1 | NM_001081025.1 | chr16:38297753-38341860 |
| 11891 | Mab21l1 | NM_010750.3 | chr3:55782509-55785287 |
| 11892 | Mab21l2 | NM_011839.3 | chr3:86545580-86548283 |
| 11893 | Mab21l3 | NM_172295.4 | chr3:101813075-101836223 |
| 11894 | Macc1 | NM_001163136.1 | chr12:119443409-119466932 |
| 11895 | Macf1 | NM_001199136.1 | chr4:123349632-123684360 |
| 11896 | Macrod1 | NM_134147.4 | chr19:7056767-7198062 |
| 11897 | Macrod2 | NM_001013802.3 | chr2:140395429-142390050 |
| 11898 | Mad1l1 | NM_010752.3 | chr5:140008688-140321552 |
| 11899 | Mad2l1 | NM_019499.4 | chr6:66535467-66540991 |
| 11900 | Mad2l1bp | NM_025649.3 | chr17:46147384-46153551 |
| 11901 | Mad2l2 | NM_027985.3 | chr4:148140489-148145704 |
| 11902 | Madcam1 | NM_013591.2 | chr10:79664573-79668536 |
| 11903 | Madd | NM_001177719.1 | chr2:91137359-91183047 |
| 11904 | Maea | NM_021500.2 | chr5:33335571-33373294 |
| 11905 | Mael | NM_175296.4 | chr1:166201384-166238744 |
| 11906 | Maf | NM_001025577.2 | chr8:115703252-115706894 |
| 11907 | Maf1 | NM_001164607.1 | chr15:76351293-76354378 |
| 11908 | Mafa | NM_194350.1 | chr15:75746842-75747922 |
| 11909 | Mafb | NM_010658.3 | chr2:160363676-160367065 |
| 11910 | Maff | NM_010755.4 | chr15:79347519-79359076 |
| 11911 | Mafg | NM_010756.3 | chr11:120628350-120633547 |
| 11912 | Mafk | NM_010757.2 | chr5:139791535-139802652 |
| 11913 | Mag | NM_010758.2 | chr7:30899182-30914832 |
| 11914 | Magea1 | NM_020015.2 | chrX:155088518-155089790 |
| 11915 | Magea10 | NM_001085506.1 | chrX:72381869-72386858 |
| 11916 | Magea2 | NM_020016.1 | chrX:155027200-155033292 |
| 11917 | Magea3 | NM_020017.2 | chrX:154948462-154949569 |
| 11918 | Magea4 | NM_020280.2 | chrX:72222016-72222964 |
| 11919 | Magea5 | NM_020018.1 | chrX:155053060-155061553 |
| 11920 | Magea6 | NM_020019.3 | chrX:154924011-154926115 |
| 11921 | Magea8 | NM_020020.4 | chrX:154985774-154995850 |
| 11922 | Mageb1 | NM_010759.1 | chrX:91331754-92016333 |
| 11923 | Mageb16 | NM_001113734.1 | chrX:79623230-79635582 |
| 11924 | Mageb16-ps1 | NR_033647.1 | chrX:144552757-144556239 |
| 11925 | Mageb18 | NM_173783.3 | chrX:92118878-92599572 |
| 11926 | Mageb2 | NM_031171.1 | chrX:91331904-92016183 |
| 11927 | Mageb3 | NM_008545.2 | chr2:121953770-121956092 |
| 11928 | Mageb4 | NM_001033492.2 | chrX:86250255-86256219 |
| 11929 | Mageb5 | NM_028847.1 | chrX:91779607-91782976 |
| 11930 | Maged1 | NM_019791.2 | chrX:94535473-94542074 |
| 11931 | Maged2 | NM_001199246.1 | chrX:150806420-150813703 |
| 11932 | Magee1 | NM_053201.4 | chrX:105120377-105123961 |
| 11933 | Magee2 | NM_053206.2 | chrX:104854951-104857267 |
| 11934 | Mageh1 | NM_023788.3 | chrX:153036165-153037563 |
| 11935 | Magel2 | NM_013779.2 | chr7:62376978-62381640 |
| 11936 | Magi1 | NM_001029850.4 | chr6:93675452-94283917 |
| 11937 | Magi2 | NM_001170745.1 | chr5:19907517-20704792 |
| 11938 | Magi3 | NM_001159354.1 | chr3:104013264-104220406 |
| 11939 | Magix | NM_018832.2 | chrX:7673165-7681089 |
| 11940 | Magoh | NM_001282737.1 | chr4:107879754-107887424 |
| 11941 | Magohb | NM_025564.2 | chr6:131284388-131293244 |
| 11942 | Magt1 | NM_001190409.1 | chrX:105968084-106011899 |
| 11943 | Mak | NM_001145802.1 | chr13:41025119-41079706 |
| 11944 | Mak16 | NM_026453.3 | chr8:31159467-31168724 |
| 11945 | Mal | NM_001171187.1 | chr2:127633225-127656695 |
| 11946 | Mal2 | NM_178920.4 | chr15:54571365-54602846 |
| 11947 | Malat1 | NR_002847.2 | chr19:5795689-5802671 |
| 11948 | Mall | NM_145532.3 | chr2:127704389-127729897 |
| 11949 | Malsu1 | NM_029353.1 | chr6:49073794-49084717 |
| 11950 | Malt1 | NM_172833.2 | chr18:65430996-65478792 |
| 11951 | Mamdc2 | NM_174857.3 | chr19:23302608-23448322 |
| 11952 | Mamdc4 | NM_001081199.1 | chr2:25563114-25571316 |
| 11953 | Maml1 | NM_175334.3 | chr11:50255634-50292336 |
| 11954 | Maml2 | NM_001013813.3 | chr9:13619988-13709533 |
| 11955 | Maml3 | NM_001004176.2 | chr3:51687612-52105006 |
| 11956 | Mamld1 | NM_001081354.2 | chrX:71050255-71154717 |
| 11957 | Mamstr | NM_172418.2 | chr7:45639976-45645768 |
| 11958 | Man1a | NM_008548.4 | chr10:53906032-54075796 |
| 11959 | Man1a2 | NM_010763.2 | chr3:100562204-100685473 |
| 11960 | Man1b1 | NM_001029983.2 | chr2:25332742-25352213 |
| 11961 | Man1c1 | NM_207237.3 | chr4:134561689-134704290 |
| 11962 | Man2a1 | NM_008549.2 | chr17:64601648-64755110 |
| 11963 | Man2a2 | NM_172903.4 | chr7:80349096-80371375 |
| 11964 | Man2b1 | NM_010764.2 | chr8:85083268-85098739 |
| 11965 | Man2b2 | NM_008550.2 | chr5:36806812-36830649 |
| 11966 | Man2c1 | NM_028636.2 | chr9:57130776-57142210 |
| 11967 | Man2c1os | NR_102289.1 | chr9:57129981-57131290 |
| 11968 | Manba | NM_027288.3 | chr3:135485610-135571403 |
| 11969 | Manbal | NM_026968.3 | chr2:157367593-157396763 |
| 11970 | Manea | NM_172865.2 | chr4:26324505-26346652 |
| 11971 | Maneal | NM_001007573.2 | chr4:124855238-124862171 |
| 11972 | Manf | NM_029103.3 | chr9:106887414-106891938 |
| 11973 | Manr | NR_110437.1 | chr3:29891016-29924191 |
| 11974 | Mansc1 | NM_026345.4 | chr6:134609206-134632488 |
| 11975 | Mansc4 | NM_001034903.3 | chr6:147075061-147087032 |
| 11976 | Maoa | NM_173740.3 | chrX:16619697-16687812 |
| 11977 | Maob | NM_172778.2 | chrX:16709280-16817366 |
| 11978 | Map10 | NM_028908.3 | chr8:125669817-125673365 |
| 11979 | Map1a | NM_001173506.1 | chr2:121295453-121310832 |
| 11980 | Map1b | NM_008634.2 | chr13:99421463-99516602 |
| 11981 | Map1lc3a | NM_025735.3 | chr2:155276363-155278073 |
| 11982 | Map1lc3b | NM_026160.4 | chr8:121590467-121598047 |
| 11983 | Map1s | NM_173013.3 | chr8:70905973-70917529 |
| 11984 | Map2 | NM_001039934 | chr1:66175328-66442583 |
| 11985 | Map2k1 | NM_008927 | chr9:64185792-64253605 |
| 11986 | Map2k2 | NM_023138.1 | chr10:81105946-81124697 |
| 11987 | Map2k3 | NM_008928.4 | chr11:60932056-60952803 |
| 11988 | Map2k3os | NR_027800.1 | chr11:60920940-60931867 |
| 11989 | Map2k4 | NM_009157.5 | chr11:65688243-65788297 |
| 11990 | Map2k5 | NM_011840.2 | chr9:63163769-63377852 |
| 11991 | Map2k6 | NM_011943.2 | chr11:110399121-110513637 |
| 11992 | Map2k7 | NM_001042557.2 | chr8:4238739-4247897 |
| 11993 | Map3k1 | NM_011945.2 | chr13:111746434-111808983 |
| 11994 | Map3k10 | NM_001290528.1 | chr7:27656375-27674607 |
| 11995 | Map3k11 | NM_022012.3 | chr19:5689130-5702864 |
| 11996 | Map3k12 | NM_001163643.1 | chr15:102497646-102517004 |
| 11997 | Map3k13 | NM_172821.3 | chr16:21891968-21931877 |
| 11998 | Map3k14 | NM_016896.3 | chr11:103219763-103267401 |
| 11999 | Map3k15 | NM_001163085.2 | chrX:159988432-160123351 |
| 12000 | Map3k19 | NM_011737.1 | chr1:127815270-127840290 |
| 12001 | Map3k2 | NM_011946.3 | chr18:32163088-32236751 |
| 12002 | Map3k3 | NM_011947.3 | chr11:106084901-106155434 |
| 12003 | Map3k4 | NM_011948.2 | chr17:12227620-12318660 |
| 12004 | Map3k5 | NM_008580.4 | chr10:19934525-20142750 |
| 12005 | Map3k6 | NM_016693.5 | chr4:133240817-133252928 |
| 12006 | Map3k7 | NM_009316.1 | chr4:31964106-32023466 |
| 12007 | Map3k7cl | NM_144854.2 | chr16:87553329-87595336 |
| 12008 | Map3k8 | NM_007746.2 | chr18:4331326-4352953 |
| 12009 | Map3k9 | NM_001174107.1 | chr12:81714949-81781170 |
| 12010 | Map4 | NM_001205330.1 | chr9:109931773-110083954 |
| 12011 | Map4k1 | NM_008279.2 | chr7:28982853-29003278 |
| 12012 | Map4k2 | NM_001291787.1 | chr19:6341159-6355619 |
| 12013 | Map4k3 | NM_001290345.1 | chr17:80580512-80728025 |
| 12014 | Map4k4 | NM_001252200.1 | chr1:39900912-40026310 |
| 12015 | Map4k5 | NM_201519.2 | chr12:69803756-69893163 |
| 12016 | Map6 | NM_001043355.2 | chr7:99268345-99337137 |
| 12017 | Map6d1 | NM_198599.2 | chr16:20233308-20241358 |
| 12018 | Map7 | NM_001198635.1 | chr10:20148919-20281590 |
| 12019 | Map7d1 | NM_001145970.1 | chr4:126232167-126256319 |
| 12020 | Map7d2 | NM_001081124.2 | chrX:159414571-159498757 |
| 12021 | Map9 | NM_001081230.1 | chr3:82358071-82395268 |
| 12022 | Mapk1 | NM_001038663 | chr16:16983381-17039040 |
| 12023 | Mapk10 | NM_001081567.2 | chr5:102908548-103211334 |
| 12024 | Mapk11 | NM_011161.5 | chr15:89142483-89149606 |
| 12025 | Mapk12 | NM_013871.3 | chr15:89130583-89140703 |
| 12026 | Mapk13 | NM_011950.2 | chr17:28769316-28778704 |
| 12027 | Mapk14 | NM_001168508.1 | chr17:28691341-28748405 |

Fig.21 - 63

| | | | |
|---|---|---|---|
| 12028 | Mapk15 | NM_177922.2 | chr15:75993768-75999153 |
| 12029 | Mapk1ip1 | NM_001045483.1 | chr7:138835817-138846267 |
| 12030 | Mapk1ip1l | NM_178684.5 | chr14:47298313-47323091 |
| 12031 | Mapk3 | NM_011952.2 | chr7:126759625-126765816 |
| 12032 | Mapk4 | NM_172632.2 | chr18:73928485-74064949 |
| 12033 | Mapk6 | NM_015806.5 | chr9:75386781-75410016 |
| 12034 | Mapk7 | NM_001291033.1 | chr11:61488811-61494266 |
| 12035 | Mapk8 | NM_016700.4 | chr14:33377898-33447158 |
| 12036 | Mapk8ip1 | NM_001202445.1 | chr2:92383675-92392683 |
| 12037 | Mapk8ip2 | NM_021921.3 | chr15:89453910-89462447 |
| 12038 | Mapk8ip3 | NM_001163447.1 | chr17:24897505-24936977 |
| 12039 | Mapk9 | NM_001163671.1 | chr11:49846750-49886421 |
| 12040 | Mapkap1 | NM_001290625.1 | chr2:34406770-34624958 |
| 12041 | Mapkapk2 | NM_008551.2 | chr1:131053700-131097843 |
| 12042 | Mapkapk3 | NM_178907.3 | chr9:107254926-107289877 |
| 12043 | Mapkapk5 | NM_010765.2 | chr5:121525050-121545892 |
| 12044 | Mapkbp1 | NM_011941.3 | chr2:119972698-120027403 |
| 12045 | Mapre1 | NM_007896.3 | chr2:153741286-153773314 |
| 12046 | Mapre2 | NM_001162941.1 | chr18:23753724-23893861 |
| 12047 | Mapre3 | NM_133350.2 | chr5:30814640-30866106 |
| 12048 | Mapt | NM_001038609.2 | chr11:104231435-104332089 |
| 12049 | Marc1 | NM_001290273.1 | chr1:184786766-184811300 |
| 12050 | Marc2 | NM_133684.3 | chr1:184813067- 184845847 |
| 12051 | March1 | NM_001166372.1 | chr8:65618039-66471637 |
| 12052 | March10 | NM_001039242.2 | chr11:105401653-105456735 |
| 12053 | March11 | NM_177597.6 | chr15:26309047-26409575 |
| 12054 | March2 | NM_001252480.1 | chr17:33691504-33718677 |
| 12055 | March3 | NM_177115.2 | chr18:56761715-56925548 |
| 12056 | March4 | NM_001045533.1 | chr1:72427111-72536564 |
| 12057 | March5 | NM_001164336.1 | chr19:37207544-37221076 |
| 12058 | March6 | NM_172606.2 | chr15:31455898-31531037 |
| 12059 | March7 | NM_020575.2 | chr2:60209935-60248385 |
| 12060 | March8 | NM_027920.5 | chr6:116338021-116409541 |
| 12061 | March9 | NM_001033262.2 | chr10:127056049-127060184 |
| 12062 | Marcks | NM_008538.2 | chr10:37133242-37138926 |
| 12063 | Marcksl1 | NM_010807.4 | chr4:129513580-129515981 |
| 12064 | Marcksl1-ps4 | NR_028405.1 | chr13:4248734-4249777 |
| 12065 | Marco | NM_010766.2 | chr1:120474537-120505084 |
| 12066 | Marf1 | NM_001081154.2 | chr16:14109165-14159274 |
| 12067 | Mark1 | NM_145515.2 | chr1:184896423-184999549 |
| 12068 | Mark2 | NM_001080388.2 | chr19:7275395-7341860 |
| 12069 | Mark3 | NM_021516.4 | chr12:111574509-111656227 |
| 12070 | Mark4 | NM_172279.1 | chr7:19426074-19458494 |
| 12071 | Mars | NM_001003913.2 | chr10:127296220-127311786 |
| 12072 | Mars2 | NM_175439 | chr1:55237176-55240058 |
| 12073 | Marveld1 | NM_183195 | chr19:42147388-42151703 |
| 12074 | Marveld2 | NM_001038602 | chr13:100595956-100616971 |
| 12075 | Marveld3 | NM_028584.3 | chr8:109952908-109962205 |
| 12076 | Mas1 | NM_008552.4 | chr17:12841004-12868143 |
| 12077 | Masp1 | NM_008555.2 | chr16:23451784-23520590 |
| 12078 | Masp2 | NM_001003893.2 | chr4:148602543-148615480 |
| 12079 | Mast1 | NM_019945.2 | chr8:84911852-84937353 |
| 12080 | Mast2 | NM_001042743.2 | chr4:116306759-116464181 |
| 12081 | Mast3 | NM_199308.2 | chr8:70778116-70792433 |
| 12082 | Mast4 | NM_175171.3 | chr13:102732488-103334492 |
| 12083 | Mastl | NM_025979.4 | chr2:23116543-23156024 |
| 12084 | Mat1a | NM_133653.3 | chr14:41105032-41124428 |
| 12085 | Mat2a | NM_145569.4 | chr6:72432798-72439558 |
| 12086 | Mat2b | NM_001199274.1 | chr11:40679313-40695203 |
| 12087 | Matk | NM_001285853.1 | chr10:81257298-81262981 |
| 12088 | Matn1 | NM_010769.2 | chr4:130944384-130955476 |
| 12089 | Matn2 | NM_016762.2 | chr15:34306680-34436240 |
| 12090 | Matn3 | NM_010770.4 | chr12:8947928-8972028 |
| 12091 | Matn4 | NM_001252563.1 | chr2:164389392-164405160 |
| 12092 | Matr3 | NM_010771.6 | chr18:35562157-35592045 |
| 12093 | Mau2 | NM_001167939.1 | chr8:70016122-70042734 |
| 12094 | Mavs | NM_001206382.1 | chr2:131234062-131248024 |
| 12095 | Max | NM_001146176.1 | chr12:76937268-76962248 |
| 12096 | Maz | NM_010772.1 | chr7:127022136-127026479 |
| 12097 | Mb | NM_001164047.1 | chr15:77015486-77022787 |
| 12098 | Mb21d1 | NM_173386.5 | chr9:78430517-78443237 |
| 12099 | Mb21d2 | NM_177718.3 | chr16:28826175-28929698 |
| 12100 | Mbd1 | NM_013594.2 | chr18:74268287-74282684 |
| 12101 | Mbd2 | NM_010773.2 | chr18:70568291-70626131 |
| 12102 | Mbd3 | NM_013595.3 | chr10:80392538-80399531 |
| 12103 | Mbd3l1 | NM_028557.2 | chr9:18478394-18485292 |
| 12104 | Mbd3l2 | NM_144934.3 | chr9:18430614-18446316 |
| 12105 | Mbd4 | NM_010774.2 | chr6:115840696-115853341 |
| 12106 | Mbd5 | NM_001290656.1 | chr2:49245899-49317069 |
| 12107 | Mbd6 | NM_033072.2 | chr10:127281955-127288771 |
| 12108 | Mbip | NM_145442.2 | chr12:56328306-56345894 |
| 12109 | Mbl1 | NM_010775.2 | chr14:41151455-41158959 |
| 12110 | Mbl2 | NM_010776.1 | chr19:30232956-30239682 |
| 12111 | Mblac1 | NM_177878.3 | chr5:138194313-138195621 |
| 12112 | Mblac2 | NM_028372.1 | chr13:81711416-81753275 |
| 12113 | Mbnl1 | NM_001253708.2 | chr3:60501178-60629750 |
| 12114 | Mbnl2 | NM_175341.4 | chr14:120275668-120431698 |
| 12115 | Mbnl3 | NM_134163.5 | chrX:51113493-51205990 |
| 12116 | Mboat1 | NM_153546.4 | chr13:30136489-30246694 |
| 12117 | Mboat2 | NM_001083341.1 | chr12:24831598-24960299 |
| 12118 | Mboat4 | NM_001126314.2 | chr8:34115029-34125185 |
| 12119 | Mboat7 | NM_029934.3 | chr7:3677788-3693525 |
| 12120 | Mbp | NM_001025245.1 | chr18:82475122-82558703 |
| 12121 | Mbtd1 | NM_134012.3 | chr11:93886218-93946984 |
| 12122 | Mbtps1 | NM_001167910.1 | chr8:119508151-119558761 |
| 12123 | Mbtps2 | NM_172307.3 | chrX:157547821-157598715 |
| 12124 | Mc1r | NM_008559.2 | chr8:123407081-123410744 |
| 12125 | Mc2r | NM_001271716.1 | chr18:68406898-68429320 |
| 12126 | Mc3r | NM_008561.3 | chr2:172248491-172251114 |
| 12127 | Mc4r | NM_016977.4 | chr18:66857704-66860487 |
| 12128 | Mc5r | NM_013596.2 | chr18:68337602-68339711 |
| 12129 | Mcam | NM_023061.2 | chr9:44134657-44142726 |
| 12130 | Mcat | NM_001030014.2 | chr15:83546796-83555711 |
| 12131 | Mcc | NM_001085373.1 | chr18:44425059-44812182 |
| 12132 | Mccc1 | NM_023644.4 | chr3:35959305-36000678 |
| 12133 | Mccc1os | NR_029456.1 | chr3:35999912-36008828 |
| 12134 | Mccc2 | NM_030026.2 | chr13:99948531-100015639 |
| 12135 | Mcee | NM_028626.1 | chr7:64392770-64412119 |
| 12136 | Mcemp1 | NM_026985.1 | chr8:3665761-3668905 |
| 12137 | Mcf2 | NM_001289730.1 | chrX:60055955-60147700 |
| 12138 | Mcf2l | NM_001159485.1 | chr8:12949417-13020509 |
| 12139 | Mcfd2 | NM_139295.3 | chr17:87254442-87265947 |
| 12140 | Mchr1 | NM_145132.2 | chr15:81235498-81238964 |
| 12141 | Mcidas | NM_001037914.3 | chr13:112993867-113000394 |
| 12142 | Mcl1 | NM_008562.3 | chr3:95658720-95663178 |
| 12143 | Mcm10 | NM_027290.3 | chr2:4990723-5012791 |
| 12144 | Mcm2 | NM_008564.2 | chr6:88883473-88898780 |
| 12145 | Mcm3 | NM_008563.2 | chr1:20803013-20820213 |
| 12146 | Mcm3ap | NM_019434.2 | chr10:76468970-76515857 |
| 12147 | Mcm4 | NM_008565.3 | chr16:15623896-15637400 |
| 12148 | Mcm5 | NM_008566.3 | chr8:75109508-75128439 |
| 12149 | Mcm6 | NM_008567.2 | chr1:128331574-128359705 |
| 12150 | Mcm7 | NM_008568.2 | chr5:138164588-138171862 |
| 12151 | Mcm8 | NM_001291054.1 | chr2:132816140-132844188 |
| 12152 | Mcm9 | NM_027830.2 | chr10:53537323-53630439 |
| 12153 | Mcmbp | NM_145955.3 | chr7:128696458-128740429 |
| 12154 | Mcmdc2 | NM_177722.3 | chr1:9908637-9940954 |
| 12155 | Mcoln1 | NM_053177.1 | chr8:3500518-3515231 |
| 12156 | Mcoln2 | NM_001005846.2 | chr3:146150173-146195512 |
| 12157 | Mcoln3 | NM_134160.1 | chr3:146121790-146140646 |
| 12158 | Mcph1 | NM_173189.2 | chr8:18595172-18803188 |
| 12159 | Mcpt1 | NM_008570.1 | chr14:56017963-56020391 |
| 12160 | Mcpt2 | NM_008571.1 | chr14:56042122-56044634 |
| 12161 | Mcpt4 | NM_010779.2 | chr14:56059743-56062310 |
| 12162 | Mcpt8 | NM_008572.1 | chr14:56082163-56085197 |
| 12163 | Mcpt9 | NM_010782.3 | chr14:56026863-56030495 |
| 12164 | Mcpt-ps1 | NR_028284.1 | chr14:55949808-55952371 |
| 12165 | Mcrs1 | NM_001164156.1 | chr15:99242816-99251961 |
| 12166 | Mctp1 | NM_030174.2 | chr13:76384960-77031810 |
| 12167 | Mctp2 | NM_001024703.1 | chr7:72077829-72306595 |
| 12168 | Mcts1 | NM_026902.3 | chrX:38600657-38613522 |
| 12169 | Mcts2 | NM_025543.2 | chr2:152687147-152687942 |
| 12170 | Mcu | NM_001033259.4 | chr10:59446983-59616692 |
| 12171 | Mcur1 | NM_001081059.3 | chr13:43538405-43560191 |
| 12172 | Mdc1 | NM_001010833.2 | chr17:35841497-35859670 |
| 12173 | Mdfi | NM_001109973.2 | chr17:47815327-47834691 |
| 12174 | Mdfic | NM_175088.5 | chr6:15720660-15802169 |
| 12175 | Mdga1 | NM_001081160.1 | chr17:29827957-29887882 |
| 12176 | Mdga2 | NM_001193266.1 | chr12:66466058-67222549 |
| 12177 | Mdh1 | NM_008618.3 | chr11:21556691-21571934 |
| 12178 | Mdh1b | NM_029696.4 | chr1:63698826-63730318 |
| 12179 | Mdh2 | NM_008617.2 | chr5:135778648-135790386 |
| 12180 | Mdk | NM_001012335.2 | chr2:91929804-91931702 |
| 12181 | Mdm1 | NM_001162904.1 | chr10:118141786-118168999 |
| 12182 | Mdm2 | NM_001288586.1 | chr10:117688874-117710758 |
| 12183 | Mdm4 | NM_008575.4 | chr1:132986104-133025416 |
| 12184 | Mdn1 | NM_001081392.1 | chr4:32657118-32775217 |
| 12185 | Mdp1 | NM_023397.4 | chr14:55657878-55660508 |
| 12186 | Me1 | NM_001198933.1 | chr9:86581362-86687277 |
| 12187 | Me2 | NM_145494.2 | chr18:73770039-73815392 |
| 12188 | Me3 | NM_181407.4 | chr7:89632817-89854359 |
| 12189 | Mea1 | NM_001277309.1 | chr17:46680985-46683126 |
| 12190 | Meaf6 | NM_001290701.1 | chr4:125085133-125110070 |
| 12191 | Mecom | NM_007963.2 | chr3:29951295-30013204 |
| 12192 | Mecp2 | NM_001081979.2 | chrX:74026591-74085690 |
| 12193 | Mecr | NM_025297.2 | chr4:131843470-131867787 |
| 12194 | Med1 | NM_001080118.1 | chr11:98153660-98193293 |
| 12195 | Med10 | NM_138596.2 | chr13:69809881-69816094 |
| 12196 | Med11 | NM_025397.2 | chr11:70451930-70453726 |
| 12197 | Med12 | NM_021521.2 | chrX:101274090-101298934 |
| 12198 | Med12l | NM_177855.3 | chr3:59006977-59318412 |
| 12199 | Med13 | NM_001080931.1 | chr11:86265714-86357525 |
| 12200 | Med13l | NM_172424.4 | chr5:118560718-118765437 |
| 12201 | Med14 | NM_001048208.1 | chrX:12675370-12761973 |
| 12202 | Med15 | NM_001040683.2 | chr16:17651207-17722947 |
| 12203 | Med16 | NM_001163276.1 | chr10:79894706-79908938 |
| 12204 | Med17 | NM_144933.1 | chr9:15260350-15279867 |
| 12205 | Med18 | NM_026039.3 | chr4:132458728-132463921 |
| 12206 | Med19 | NM_025885.3 | chr2:84678401-84688215 |
| 12207 | Med20 | NM_020048.3 | chr17:47611595-47624418 |
| 12208 | Med21 | NM_025315.3 | chr6:146642578-146650600 |
| 12209 | Med22 | NM_001033908.1 | chr2:26905266-26910642 |
| 12210 | Med23 | NM_001166416.1 | chr10:24869985-24913529 |
| 12211 | Med24 | NM_011869.2 | chr11:98704590-98729435 |
| 12212 | Med25 | NM_029365.2 | chr7:44879385-44892366 |
| 12213 | Med26 | NM_027485.4 | chr8:72494558-72548310 |
| 12214 | Med27 | NM_001290489.1 | chr2:29378513-29524790 |
| 12215 | Med28 | NM_025895.4 | chr5:45520228-45529284 |
| 12216 | Med29 | NM_026042 | chr7:28385246-28392690 |
| 12217 | Med30 | NM_027212.2 | chr15:52712444-52730431 |
| 12218 | Med31 | NM_026068 | chr11:72211723-72215592 |
| 12219 | Med4 | NM_026119 | chr14:73510048-73518545 |
| 12220 | Med6 | NM_027213.2 | chr12:81573563-81594958 |
| 12221 | Med7 | NM_001104530.1 | chr11:46436946-46442721 |

Fig.21 - 64

| | | | |
|---|---|---|---|
| 12222 | Med8 | NM_001290688.1 | chr4:118409336-118415824 |
| 12223 | Med9 | NM_138675.3 | chr11:59948213-59963306 |
| 12224 | Med9os | NR_045273.1 | chr11:59946891-59948147 |
| 12225 | Medag | NM_027519.3 | chr5:149411805-149431703 |
| 12226 | Mef2a | NM_001033713.2 | chr7:67231162-67372858 |
| 12227 | Mef2b | NM_001045484.2 | chr8:70152760-70167488 |
| 12228 | Mef2c | NM_001170537.1 | chr13:83504033-83667079 |
| 12229 | Mef2d | NM_133665.4 | chr3:88142371-88172091 |
| 12230 | Mefv | NM_001161790.1 | chr16:3707214-3718124 |
| 12231 | Meg3 | NR_003633.3 | chr12:109545397-109568650 |
| 12232 | Megf10 | NM_001001979.2 | chr18:57133089-57297467 |
| 12233 | Megf11 | NM_001134399.1 | chr9:64385625-64709205 |
| 12234 | Megf6 | NM_001162977.1 | chr4:154170712-154275721 |
| 12235 | Megf8 | NM_001160400.1 | chr7:25317163-25365917 |
| 12236 | Megf9 | NM_172694.2 | chr4:70431926-70534928 |
| 12237 | Mei1 | NM_028897.3 | chr15:82070116-82126814 |
| 12238 | Mei4 | NM_175213.3 | chr9:81863743-82027491 |
| 12239 | Meig1 | NM_008579.4 | chr2:3409042-3422648 |
| 12240 | Meiob | NM_029197.1 | chr17:24804381-24839787 |
| 12241 | Meis1 | NM_001193271.1 | chr11:18880427-19018969 |
| 12242 | Meis2 | NM_001136072.2 | chr2:115861263-116065058 |
| 12243 | Meis3 | NM_001277988.2 | chr7:16175394-16186508 |
| 12244 | Melk | NM_010790.2 | chr4:44300916-44364675 |
| 12245 | Memo1 | NM_133771.2 | chr17:74200699-74294863 |
| 12246 | Men1 | NM_001168488.1 | chr19:6334978-6340894 |
| 12247 | Meox1 | NM_010791.3 | chr11:101877509-101894354 |
| 12248 | Meox2 | NM_008584.3 | chr12:37108545-37179528 |
| 12249 | Mep1a | NM_008585.2 | chr17:43474328-43502773 |
| 12250 | Mep1b | NM_008586.2 | chr18:21072343-21100199 |
| 12251 | Mepce | NM_144913.3 | chr5:137781905-137786701 |
| 12252 | Mepe | NM_053172.2 | chr5:104325328-104338611 |
| 12253 | Mertk | NM_008587.1 | chr2:128698996-128802188 |
| 12254 | Mesdc1 | NM_030705.4 | chr7:83880494-83884341 |
| 12255 | Mesdc2 | NM_023403.3 | chr7:83891999-83901532 |
| 12256 | Mesp1 | NM_008588.2 | chr7:79792240-79793590 |
| 12257 | Mesp2 | NM_008589.2 | chr7:79810726-79813431 |
| 12258 | Mest | NM_001252292.1 | chr6:30738052-30748466 |
| 12259 | Met | NM_008591.2 | chr6:17463956-17573980 |
| 12260 | Metap1 | NM_175224.4 | chr3:138458959-138489382 |
| 12261 | Metap1d | NM_025633.3 | chr2:71453337-71525191 |
| 12262 | Metap2 | NM_019648.3 | chr10:93858489-93891202 |
| 12263 | Metrn | NM_133719.2 | chr17:25794570-25797045 |
| 12264 | Metrnl | NM_144797.3 | chr11:121702426-121717389 |
| 12265 | Mettl1 | NM_010792.1 | chr10:127041931-127045461 |
| 12266 | Mettl10 | NM_028095.1 | chr7:132827460-132852647 |
| 12267 | Mettl11b | NM_001143956.1 | chr1:163702992-163725232 |
| 12268 | Mettl13 | NM_144877.1 | chr1:162533671-162548345 |
| 12269 | Mettl14 | NM_201638.2 | chr3:123368294-123385990 |
| 12270 | Mettl15 | NM_029790.3 | chr2:109092299-109278290 |
| 12271 | Mettl16 | NM_026197.3 | chr11:74770862-74818692 |
| 12272 | Mettl17 | NM_001029990.1 | chr14:51884841-51891868 |
| 12273 | Mettl18 | NM_027279.2 | chr1:163994944-163997244 |
| 12274 | Mettl2 | NM_172567.3 | chr11:105126424-105141146 |
| 12275 | Mettl20 | NM_001252094.1 | chr6:149141512-149151170 |
| 12276 | Mettl21a | NM_025964.3 | chr1:64606479-64617168 |
| 12277 | Mettl21c | NM_001013799.1 | chr1:44009407-44020006 |
| 12278 | Mettl21e | NM_207281.3 | chr1:44204069-44218931 |
| 12279 | Mettl22 | NM_146247.1 | chr16:8470812-8490197 |
| 12280 | Mettl23 | NM_028865.3 | chr11:116843514-116849740 |
| 12281 | Mettl24 | NM_177793.3 | chr10:40683281-40811083 |
| 12282 | Mettl25 | NM_207522.2 | chr10:105763184-105841380 |
| 12283 | Mettl3 | NM_019721.2 | chr14:52294841-52305124 |
| 12284 | Mettl4 | NM_176917.4 | chr17:94727079-94749892 |
| 12285 | Mettl5 | NM_029280.4 | chr2:69871194-69885615 |
| 12286 | Mettl6 | NM_025907.3 | chr14:31478797-31494977 |
| 12287 | Mettl7a1 | NM_027334.3 | chr15:100304816-100314348 |
| 12288 | Mettl7a2 | NM_199477.2 | chr15:100353199-100361819 |
| 12289 | Mettl7a2Higd1c | NM_001024672.3 | chr15:100353199-100384435 |
| 12290 | Mettl7a3 | NM_001081471.1 | chr15:100334928-100340169 |
| 12291 | Mettl7b | NM_027853.2 | chr10:128958276-128960988 |
| 12292 | Mettl8 | NM_001110512.1 | chr2:70964561-71018374 |
| 12293 | Mettl9 | NM_021554.2 | chr7:121034444-121076835 |
| 12294 | Mex3a | NM_001029890.2 | chr3:88532394-88541394 |
| 12295 | Mex3b | NM_175366.3 | chr7:82867332-82871576 |
| 12296 | Mex3c | NM_001039214.4 | chr18:73572704-73592578 |
| 12297 | Mex3d | NM_198615.2 | chr10:80380354-80387651 |
| 12298 | Mfap1a | NM_026220.4 | chr2:121492680-121506656 |
| 12299 | Mfap1b | NM_001081975.3 | chr2:121461665-121474023 |
| 12300 | Mfap2 | NM_001161799.1 | chr4:141010423-141015984 |
| 12301 | Mfap3 | NM_145426.2 | chr11:57518664-57533817 |
| 12302 | Mfap3l | NM_001177881.1 | chr8:60655103-60676731 |
| 12303 | Mfap4 | NM_029568.2 | chr11:61485443-61488704 |
| 12304 | Mfap5 | NM_015776.2 | chr6:122513675-122529287 |
| 12305 | Mff | NM_029409.3 | chr1:82724889-82752389 |
| 12306 | Mfge8 | NM_001045489.1 | chr7:79133767-79149060 |
| 12307 | Mfhas1 | NM_001081279.1 | chr8:35587797-35679449 |
| 12308 | Mfi2 | NM_013900.2 | chr16:31878809-31899019 |
| 12309 | Mfn1 | NM_024200.4 | chr3:32529481-32579225 |
| 12310 | Mfn2 | NM_001285920.1 | chr4:147873585-147904909 |
| 12311 | Mfng | NM_008595.2 | chr15:78755882-78773445 |
| 12312 | Mfrp | NM_001190314.1 | chr9:44101769-44109187 |
| 12313 | Mfsd1 | NM_025813.3 | chr3:67582767-67604231 |
| 12314 | Mfsd10 | NM_026660.2 | chr5:34633646-34637114 |
| 12315 | Mfsd11 | NM_178620.3 | chr11:116854014-116875637 |
| 12316 | Mfsd12 | NM_028657.3 | chr10:81357569-81364035 |
| 12317 | Mfsd2a | NM_029662.2 | chr4:122946850-122961188 |
| 12318 | Mfsd2b | NM_001033488.2 | chr12:4862437-4874359 |
| 12319 | Mfsd3 | NM_027122.3 | chr15:76701541-76704239 |
| 12320 | Mfsd4 | NM_001114662.1 | chr1:132036782-132068062 |
| 12321 | Mfsd5 | NM_134100.4 | chr15:102279455-102281744 |
| 12322 | Mfsd6 | NM_133829.2 | chr1:52656304-52727318 |
| 12323 | Mfsd6l | NM_146004.1 | chr11:68556185-68558245 |
| 12324 | Mfsd7a | NM_172883.2 | chr5:108441053-108448891 |
| 12325 | Mfsd7b | NM_001081259.1 | chr1:191005831-191026190 |
| 12326 | Mfsd7c | NM_145447.2 | chr12:85746538-85813585 |
| 12327 | Mfsd8 | NM_028140.4 | chr3:40818171-40846854 |
| 12328 | Mfsd9 | NM_172499.2 | chr1:40772039-40790657 |
| 12329 | Mga | NM_001164274.1 | chr2:119897227-119969581 |
| 12330 | Mgam | NM_001171003.1 | chr6:40628830-40769123 |
| 12331 | Mgarp | NM_026358.3 | chr3:51388412-51396547 |
| 12332 | Mgat1 | NM_001110148.1 | chr11:49244190-49263024 |
| 12333 | Mgat2 | NM_146035.2 | chr12:69184157-69186773 |
| 12334 | Mgat3 | NM_010795.4 | chr15:80173720-80215519 |
| 12335 | Mgat4a | NM_001290801.1 | chr1:37439339-37536404 |
| 12336 | Mgat4b | NM_145926.3 | chr11:50225334-50235103 |
| 12337 | Mgat4c | NM_001162368.1 | chr10:102158857-102391468 |
| 12338 | Mgat5 | NM_145128.3 | chr1:127204985-127482972 |
| 12339 | Mgat5b | NM_172948.3 | chr11:116918862-116986944 |
| 12340 | Mgea5 | NM_023799.3 | chr19:45750258-45783291 |
| 12341 | Mgl2 | NM_145137.2 | chr11:70130356-70137542 |
| 12342 | Mgll | NM_001166249.1 | chr6:88724411-88828360 |
| 12343 | Mgme1 | NM_001289630.1 | chr2:144270903-144281227 |
| 12344 | Mgmt | NM_008598.2 | chr7:136894610-137128188 |
| 12345 | Mgp | NM_008597.3 | chr6:136872434-136875805 |
| 12346 | Mgrn1 | NM_001252437.1 | chr16:4886099-4938296 |
| 12347 | Mgst1 | NM_019946.4 | chr6:138140536-138156752 |
| 12348 | Mgst2 | NM_174995.3 | chr3:51661178-51682674 |
| 12349 | Mgst3 | NM_025569.1 | chr1:167372383-167393797 |
| 12350 | Mia | NM_019394.3 | chr7:27179740-27181149 |
| 12351 | Mia2 | NM_177321.2 | chr12:59095799-59109130 |
| 12352 | Mia3 | NM_177389.3 | chr1:183326235-183369565 |
| 12353 | Miat | NR_003718.2 | chr5:112213227-112228948 |
| 12354 | Mib1 | NM_144860.2 | chr18:10725624-10812217 |
| 12355 | Mib2 | NM_001256107.1 | chr4:155654469-155669254 |
| 12356 | Mical1 | NM_001164433.1 | chr10:41476313-41487030 |
| 12357 | Mical2 | NM_001193305.1 | chr7:112225835-112353975 |
| 12358 | Mical3 | NM_001270475.1 | chr6:120931545-121131022 |
| 12359 | Micalcl | NM_027587.2 | chr7:112368307-112413104 |
| 12360 | Micall1 | NM_177461.1 | chr15:79108982-79136901 |
| 12361 | Micall2 | NM_174850.3 | chr5:139706692-139736333 |
| 12362 | Micu1 | NM_001291442.1 | chr10:59702562-59864134 |
| 12363 | Micu2 | NM_028643.3 | chr14:57916279-57999262 |
| 12364 | Micu3 | NM_030110.1 | chr8:40308050-40386304 |
| 12365 | Mid1 | NM_001290504.1 | chrX:169828158-169990797 |
| 12366 | Mid1ip1 | NM_001166635.1 | chrX:10717364-10719702 |
| 12367 | Mid2 | NM_011845.2 | chrX:140678027-140767715 |
| 12368 | Midn | NM_021565.2 | chr10:80148271-80158368 |
| 12369 | Mief1 | NM_178719.5 | chr15:80234079-80253371 |
| 12370 | Mief2 | NM_001009927.2 | chr11:60728397-60732951 |
| 12371 | Mien1 | NM_025559.2 | chr11:98437707-98438988 |
| 12372 | Mier1 | NM_001039081.2 | chr4:103119389-103165754 |
| 12373 | Mier2 | NM_027422.3 | chr10:79540244-79555091 |
| 12374 | Mier3 | NM_172593.3 | chr13:111686177-111718594 |
| 12375 | Mif | NM_010798.2 | chr10:75859352-75860250 |
| 12376 | Mif4gd | NM_001243584.1 | chr11:115607918-115612969 |
| 12377 | Miip | NM_001025365.2 | chr4:147860777-147868719 |
| 12378 | Mill1 | NM_153749.4 | chr7:18245346-18266092 |
| 12379 | Mill2 | NM_153760.2 | chr7:18839965-18858653 |
| 12380 | Milr1 | NM_001033435.3 | chr11:106751225-106767765 |
| 12381 | Mina | NM_025910.3 | chr16:59471774-59492451 |
| 12382 | Mink1 | NM_001045959.1 | chr11:70562880-70614482 |
| 12383 | Minos1 | NM_001163006.2 | chr4:139101813-139131113 |
| 12384 | Minpp1 | NM_010799.2 | chr19:32485768-32515370 |
| 12385 | Mios | NM_145374.2 | chr6:8209226-8236273 |
| 12386 | Miox | NM_019977.2 | chr15:89334472-89337007 |
| 12387 | Mip | NM_008600.5 | chr10:128225809-128231812 |
| 12388 | Mipep | NM_027436.3 | chr14:60784565-60903613 |
| 12389 | Mipol1 | NM_001164370.1 | chr12:57230424-57457241 |
| 12390 | Mir100 | NR_029790.1 | chr9:41531424-41531504 |
| 12391 | Mir101a | NR_029537.1 | chr4:101346944-101347027 |
| 12392 | Mir101b | NR_029778.1 | chr19:29135278-29135375 |
| 12393 | Mir101c | NR_039546.1 | chr9:3038668-3038743 |
| 12394 | Mir103-1 | NR_029754.1 | chr11:35782395-35782481 |
| 12395 | Mir103-2 | NR_029755.1 | chr2:131288051-131288137 |
| 12396 | Mir105 | NR_030546.1 | chrX:72591499-72591579 |
| 12397 | Mir106a | NR_029657.1 | chrX:52742502-52742567 |
| 12398 | Mir106b | NR_029658.1 | chr5:138165736-138165818 |
| 12399 | Mir107 | NR_029783.1 | chr19:34820686-34820773 |
| 12400 | Mir10a | NR_029784.1 | chr11:96317164-96317274 |
| 12401 | Mir10b | NR_029566.1 | chr2:74726069-74726137 |
| 12402 | Mir1187 | NR_035415.1 | chr5:82798943-82799065 |
| 12403 | Mir1188 | NR_035419.1 | chr12:109611821-109611941 |
| 12404 | Mir1190 | NR_035421 | chr12:101021672-101021768 |
| 12405 | Mir1191 | NR_035422.1 | chr5:76206228-76469519 |
| 12406 | Mir1191b | NR_106141.1 | chr10:81416239-81416297 |
| 12407 | Mir1192 | NR_035423.1 | chr19:23149430-23149551 |
| 12408 | Mir1193 | NR_035424.1 | chr12:109715670-109715791 |
| 12409 | Mir1195 | NR_035427 | chr16:30920553-31275697 |
| 12410 | Mir1197 | NR_035429.1 | chr12:109712316-109712436 |
| 12411 | Mir1198 | NR_035430.1 | chr12:55370694-55491306 |
| 12412 | Mir1199 | NR_035431.1 | chr8:84011514-84011633 |
| 12413 | Mir1224 | NR_035407.1 | chr16:20604451-20604536 |
| 12414 | Mir122a | NR_029600.1 | chr18:65248860-65248926 |
| 12415 | Mir1231 | NR_049188.1 | chr1:135454602-135454683 |

Fig.21 - 65

| | | | |
|---|---|---|---|
| 12416 | Mir1247 | NR_037204.1 | chr12:110278047-110278129 |
| 12417 | Mir1249 | NR_037206.1 | chr15:84951525-84951623 |
| 12418 | Mir124a-1 | NR_029813.1 | chr14:64590656-64590741 |
| 12419 | Mir124a-2 | NR_029814.1 | chr3:17795661-17795770 |
| 12420 | Mir124a-3 | NR_029538.1 | chr2:180894039-180894107 |
| 12421 | Mir1251 | NR_037219.1 | chr10:92137139-92137223 |
| 12422 | Mir1258 | NR_106160.1 | chr18:56538138-56538198 |
| 12423 | Mir125a | NR_029539.1 | chr17:17830811-17830879 |
| 12424 | Mir125b-1 | NR_029822.1 | chr9:41581925-41582002 |
| 12425 | Mir125b-2 | NR_029540.1 | chr16:77646272-77646343 |
| 12426 | Mir126 | NR_029541.1 | chr2:26591356-26591429 |
| 12427 | Mir1264 | NR_037205.1 | chrX:147010600-147010686 |
| 12428 | Mir126b | NR_106155.1 | chr2:26591362-26591420 |
| 12429 | Mir127 | NR_029542.1 | chr12:109592845-109592915 |
| 12430 | Mir128-1 | NR_029543.1 | chr1:128202360-128202430 |
| 12431 | Mir128-2 | NR_029823.1 | chr9:112118635-112118711 |
| 12432 | Mir1291 | NR_106171.1 | chr15:98519761-98519878 |
| 12433 | Mir129-1 | NR_029567.1 | chr6:29022618-29022691 |
| 12434 | Mir129-2 | NR_029752.1 | chr2:94241363-94241453 |
| 12435 | Mir1298 | NR_037210.1 | chrX:147064904-147065002 |
| 12436 | Mir129b | NR_106139.1 | chr2:94241377-94241442 |
| 12437 | Mir1306 | NR_035467.2 | chr16:18284238-18284317 |
| 12438 | Mir130a | NR_029544.1 | chr2:84741114-84741178 |
| 12439 | Mir130b | NR_029659.1 | chr16:17124060-17124142 |
| 12440 | Mir130c | NR_105807.1 | chr9:53400804-53400886 |
| 12441 | Mir132 | NR_029546.1 | chr11:75173681-75173747 |
| 12442 | Mir133a-1 | NR_029547.1 | chr18:10782908-10782976 |
| 12443 | Mir133a-2 | NR_029901.1 | chr2:180398378-180398482 |
| 12444 | Mir133b | NR_029902.1 | chr1:20682768-20682887 |
| 12445 | Mir133c | NR_105753.1 | chr2:29475214-29475294 |
| 12446 | Mir134 | NR_029548.1 | chr12:109734138-109734209 |
| 12447 | Mir135a-1 | NR_029549.1 | chr9:106154123-106154213 |
| 12448 | Mir135a-2 | NR_029812.1 | chr10:92072085-92072185 |
| 12449 | Mir135b | NR_029777.1 | chr1:132198087-132198184 |
| 12450 | Mir136 | NR_029550.1 | chr12:109595326-109595388 |
| 12451 | Mir137 | NR_029551.1 | chr3:118433856-118433929 |
| 12452 | Mir138-1 | NR_029819.1 | chr9:122682875-122682974 |
| 12453 | Mir138-2 | NR_029552.1 | chr8:94324310-94324381 |
| 12454 | Mir139 | NR_029791.1 | chr7:101475375-101475443 |
| 12455 | Mir140 | NR_029553.1 | chr8:107551243-107551313 |
| 12456 | Mir141 | NR_029554.1 | chr6:124717913-124717985 |
| 12457 | Mir142 | NR_029555.1 | chr11:87756863-87756927 |
| 12458 | Mir142b | NR_106176.1 | chr11:87756840-87756955 |
| 12459 | Mir143 | NR_029601.1 | chr18:61649195-61649258 |
| 12460 | Mir143hg | NR_045402.1 | chr18:61645877-61665538 |
| 12461 | Mir144 | NR_029556.1 | chr11:78073004-78073070 |
| 12462 | Mir145 | NR_029557.1 | chr18:61647824-61647894 |
| 12463 | Mir145b | NR_105780.1 | chr18:69022198-69022273 |
| 12464 | Mir146 | NR_029558.1 | chr11:43374396-43374461 |
| 12465 | Mir146b | NR_030468.1 | chr19:46342761-46342870 |
| 12466 | Mir147 | NR_030547.1 | chr2:122640802-122640881 |
| 12467 | Mir148a | NR_029719.1 | chr6:51269811-51269910 |
| 12468 | Mir148b | NR_029766.1 | chr15:103285124-103285221 |
| 12469 | Mir149 | NR_029559.1 | chr1:92850377-92850443 |
| 12470 | Mir150 | NR_029560.1 | chr7:45121756-45121821 |
| 12471 | Mir152 | NR_029562.1 | chr11:96850392-96850465 |
| 12472 | Mir153 | NR_029563.1 | chr12:117250816-117250885 |
| 12473 | Mir154 | NR_029564.1 | chr12:109738432-109738498 |
| 12474 | Mir155 | NR_029565.1 | chr16:84714139-84714204 |
| 12475 | Mir15a | NR_029733.1 | chr14:61632026-61632110 |
| 12476 | Mir15b | NR_029529.1 | chr3:69009771-69009835 |
| 12477 | Mir16-1 | NR_029734.1 | chr14:61631879-61631972 |
| 12478 | Mir16-2 | NR_029735.1 | chr3:69009901-69009996 |
| 12479 | Mir1668 | NR_106188.1 | chr8:83723877-83723984 |
| 12480 | Mir17 | NR_029785.1 | chr14:115043670-115043754 |
| 12481 | Mir17hg | NR_029382.1 | chr14:115044389-115046728 |
| 12482 | Mir18 | NR_029736.1 | chr14:115043850-115043946 |
| 12483 | Mir181a-1 | NR_029795.1 | chr1:137966454-137966541 |
| 12484 | Mir181a-2 | NR_029568.1 | chr2:38852734-38852810 |
| 12485 | Mir181b-1 | NR_029820.1 | chr1:137966638-137966718 |
| 12486 | Mir181b-2 | NR_029904.1 | chr2:38853829-38853915 |
| 12487 | Mir181c | NR_029821.1 | chr8:84178872-84178961 |
| 12488 | Mir181d | NR_030534.1 | chr8:84178715-84178787 |
| 12489 | Mir182 | NR_029569.1 | chr6:30165917-30165992 |
| 12490 | Mir183 | NR_030713.1 | chr6:30169667-30169737 |
| 12491 | Mir1839 | NR_035501.1 | chr7:81529915-81529988 |
| 12492 | Mir184 | NR_029570.1 | chr9:89802259-89802328 |
| 12493 | Mir1843 | NR_037207.1 | chr12:80391612-80391677 |
| 12494 | Mir1843b | NR_039543.1 | chr1:159340357-159340423 |
| 12495 | Mir185 | NR_029571.1 | chr16:18327400-18327465 |
| 12496 | Mir186 | NR_029572.1 | chr3:157544278-157544349 |
| 12497 | Mir187 | NR_029573.1 | chr18:24429109-24429170 |
| 12498 | Mir188 | NR_029574.1 | chrX:7247988-7248056 |
| 12499 | Mir1892 | NR_035439.1 | chr12:54645932-54646012 |
| 12500 | Mir1893 | NR_035446.1 | chr18:6490563-6490646 |
| 12501 | Mir1894 | NR_035445.1 | chr17:35917888-35917969 |
| 12502 | Mir1895 | NR_035435.1 | chr3:134240504-134240583 |
| 12503 | Mir1896 | NR_035441.1 | chr13:21445159-21445240 |
| 12504 | Mir1897 | NR_035433.1 | chr3:34638463-34638542 |
| 12505 | Mir1898 | NR_035443.1 | chr15:12171491-12171574 |
| 12506 | Mir1899 | NR_035437.1 | chr9:8246785-8246881 |
| 12507 | Mir18b | NR_030548.1 | chrX:52742330-52742413 |
| 12508 | Mir190 | NR_029576.1 | chr9:67236659-67236726 |
| 12509 | Mir1900 | NR_035438.1 | chr9:21032250-21032328 |
| 12510 | Mir1901 | NR_035447.1 | chr18:11840360-11840438 |
| 12511 | Mir1902 | NR_035432.1 | chr2:104428860-104428940 |
| 12512 | Mir1903 | NR_035436.1 | chr8:128359240-128359320 |
| 12513 | Mir1904 | NR_035442.1 | chr13:109903808-109903888 |
| 12514 | Mir1905 | NR_035434.1 | chr3:88536300-88536382 |
| 12515 | Mir1906-1 | NR_035440.1 | chrX:88759473-109544620 |
| 12516 | Mir1907 | NR_035444.1 | chr15:50889024-50889114 |
| 12517 | Mir190b | NR_030543.1 | chr3:90070019-90070099 |
| 12518 | Mir191 | NR_029577.1 | chr9:108568318-108568392 |
| 12519 | Mir1912 | NR_037300.1 | chrX:147009440-147009527 |
| 12520 | Mir192 | NR_029720.1 | chr19:6264843-6264932 |
| 12521 | Mir1928 | NR_035449.1 | chr1:74206553-74206620 |
| 12522 | Mir1929 | NR_035450.1 | chr10:44359675-44359768 |
| 12523 | Mir193 | NR_029579.1 | chr11:79711968-79712034 |
| 12524 | Mir1930 | NR_035451.1 | chr10:77641223-77641307 |
| 12525 | Mir1931 | NR_035452.1 | chr10:93162784-93162903 |
| 12526 | Mir1932 | NR_035453.1 | chr11:119390471-119390561 |
| 12527 | Mir1933 | NR_035454.1 | chr11:21344587-21344675 |
| 12528 | Mir1934 | NR_035455.1 | chr11:69663042-69663125 |
| 12529 | Mir1936 | NR_035457.1 | chr12:102684927-102685020 |
| 12530 | Mir1938 | NR_035459.1 | chr12:40222711-40222811 |
| 12531 | Mir193b | NR_030549.1 | chr16:13449522-13449601 |
| 12532 | Mir1940 | NR_035461.1 | chr13:95330580-95330683 |
| 12533 | Mir1941 | NR_035462.1 | chr15:101369351-101369434 |
| 12534 | Mir194-1 | NR_029580.1 | chr1:185313318-185313385 |
| 12535 | Mir1942 | NR_035463.1 | chr15:76215610-76215673 |
| 12536 | Mir194-2 | NR_029830.1 | chr19:6264642-6264728 |
| 12537 | Mir1943 | NR_035464.1 | chr15:79375227-79375300 |
| 12538 | Mir1945 | NR_035466.1 | chr16:11254367-11254445 |
| 12539 | Mir1946a | NR_035468.1 | chr16:32267449-32267583 |
| 12540 | Mir1946b | NR_035496.1 | chr3:96080595-96148402 |
| 12541 | Mir1947 | NR_035469.1 | chr16:33105360-33105445 |
| 12542 | Mir1948 | NR_035471.1 | chr18:12714810-12714895 |
| 12543 | Mir1949 | NR_035472.1 | chr18:35554566-35554636 |
| 12544 | Mir195 | NR_029581.1 | chr11:70235041-70235135 |
| 12545 | Mir1950 | NR_035473.1 | chr6:40529090-40910666 |
| 12546 | Mir1951 | NR_035476.1 | chr2:115638724-115638813 |
| 12547 | Mir1952 | NR_035477.1 | chr2:138819928-138820007 |
| 12548 | Mir1953 | NR_035478.1 | chr2:151967528-151967617 |
| 12549 | Mir1954 | NR_035479.1 | chr2:113220186-113423560 |
| 12550 | Mir1955 | NR_035480.1 | chr2:92191976-92192074 |
| 12551 | Mir1956 | NR_035481.1 | chr3:138526420-138526485 |
| 12552 | Mir1957 | NR_035483.1 | chr6:24484966-94782876 |
| 12553 | Mir1957b | NR_105819.1 | chr8:111450199-111450322 |
| 12554 | Mir1958 | NR_035484.1 | chr1:63980972-63984483 |
| 12555 | Mir195b | NR_105751.1 | chr2:56785810-56785907 |
| 12556 | Mir1960 | NR_035486.1 | chr5:30170738-30170816 |
| 12557 | Mir1961 | NR_035487.1 | chr5:92788450-92788562 |
| 12558 | Mir1962 | NR_035488.1 | chr7:135566161-135566282 |
| 12559 | Mir1963 | NR_035489.1 | chr7:29083635-29083694 |
| 12560 | Mir1964 | NR_035490.1 | chr7:29773293-29773376 |
| 12561 | Mir1966 | NR_035492 | chr8:105615465-105615573 |
| 12562 | Mir1967 | NR_035493.1 | chr8:124022640-124022722 |
| 12563 | Mir1968 | NR_035494.1 | chr8:13189030-13189098 |
| 12564 | Mir1969 | NR_035495.1 | chr8:70925524-70925618 |
| 12565 | Mir196a-1 | NR_029721.1 | chr11:96265163-96265265 |
| 12566 | Mir196a-2 | NR_029722.1 | chr15:102973349-102973434 |
| 12567 | Mir196b | NR_029912.1 | chr6:52230080-52230165 |
| 12568 | Mir1970 | NR_035497.1 | chr7:107284774-107580276 |
| 12569 | Mir1971 | NR_035499.1 | chr14:78191372-78191478 |
| 12570 | Mir1981 | NR_035502.1 | chr1:184822406-184822488 |
| 12571 | Mir1982 | NR_035503.1 | chr10:80828796-80828870 |
| 12572 | Mir1983 | NR_035500.1 | chr13:21896917-21897049 |
| 12573 | Mir199a-1 | NR_029585.1 | chr9:21496494-21496564 |
| 12574 | Mir199a-2 | NR_029810.1 | chr1:162217813-162217923 |
| 12575 | Mir199b | NR_029811.1 | chr2:32318459-32318569 |
| 12576 | Mir19a | NR_029786.1 | chr14:115043999-115044081 |
| 12577 | Mir19b-1 | NR_029815.1 | chr14:115044304-115044391 |
| 12578 | Mir19b-2 | NR_029715.1 | chrX:52741982-52742066 |
| 12579 | Mir1a-1 | NR_029528.1 | chr2:180389047-180389124 |
| 12580 | Mir1a-2 | NR_029781.1 | chr18:10785480-10785552 |
| 12581 | Mir1b | NR_035413.1 | chr18:10785445-10785567 |
| 12582 | Mir200a | NR_029723.1 | chr4:156054895-156054985 |
| 12583 | Mir200b | NR_029587.1 | chr4:156055680-156055750 |
| 12584 | Mir200c | NR_029792.1 | chr6:124718321-124718390 |
| 12585 | Mir201 | NR_029588.1 | chrX:67988095-67988161 |
| 12586 | Mir202 | NR_029589.1 | chr7:139957688-139957760 |
| 12587 | Mir203 | NR_029590.1 | chr12:112130879-112130955 |
| 12588 | Mir204 | NR_029591.1 | chr19:22750604-22750672 |
| 12589 | Mir205 | NR_029592.1 | chr1:193507462-193507530 |
| 12590 | Mir206 | NR_029593.1 | chr1:20679009-20679082 |
| 12591 | Mir207 | NR_029594.1 | chr4:40722916-40722995 |
| 12592 | Mir208a | NR_029724.1 | chr14:54949059-54949142 |
| 12593 | Mir208b | NR_030607.1 | chr14:54975699-54975776 |
| 12594 | Mir20a | NR_029737.1 | chr14:115044156-115044263 |
| 12595 | Mir20b | NR_030273.1 | chrX:52742112-52742192 |
| 12596 | Mir21 | NR_029738.1 | chr11:86584066-86584158 |
| 12597 | Mir210 | NR_029793.1 | chr7:141221383-141221493 |
| 12598 | Mir211 | NR_029805.1 | chr7:64205805-64205911 |
| 12599 | Mir212 | NR_029794.1 | chr11:75173387-75173478 |
| 12600 | Mir2136 | NR_035516.1 | chr9:104426112-104426187 |
| 12601 | Mir2137 | NR_035517.1 | chrX:72992078-72992144 |
| 12602 | Mir2139 | NR_035519.1 | chr4:139967525-139967620 |
| 12603 | Mir214 | NR_029796.1 | chr1:162223367-162223477 |
| 12604 | Mir215 | NR_029908.1 | chr1:185313580-185313691 |
| 12605 | Mir216a | NR_029797.1 | chr11:28757011-28757083 |
| 12606 | Mir216b | NR_030419.1 | chr11:28746190-28746276 |
| 12607 | Mir216c | NR_106152.1 | chr11:28746197-28746266 |
| 12608 | Mir217 | NR_029828.1 | chr11:28763727-28763835 |
| 12609 | Mir218-1 | NR_029798.1 | chr5:48223941-48224051 |

Fig.21 - 66

| | | | | | | | |
|---|---|---|---|---|---|---|---|
| 12610 | Mir218-2 | NR_029799.1 | chr11:35616815-35616925 | 12707 | Mir3097 | NR_037280.1 | chr5:35021048-35021115 |
| 12611 | Mir219-1 | NR_029800.1 | chr17:34024982-34025092 | 12708 | Mir3098 | NR_037281.1 | chr2:131142468-131604824 |
| 12612 | Mir219-2 | NR_029838.1 | chr2:29845630-29845727 | 12709 | Mir3099 | NR_037213.1 | chr7:6803586-6803670 |
| 12613 | Mir219b | NR_106110.1 | chr2:29845646-29845711 | 12710 | Mir30a | NR_029533.1 | chr1:23272268-23272339 |
| 12614 | Mir219c | NR_106154.1 | chr17:34025011-34025071 | 12711 | Mir30b | NR_029534.1 | chr15:68337414-68337510 |
| 12615 | Mir21b | NR_105796.1 | chr3:29636743-29636851 | 12712 | Mir30c-1 | NR_029716.1 | chr4:120769533-120769622 |
| 12616 | Mir21c | NR_105822.1 | chr8:128278147-128278225 | 12713 | Mir30c-2 | NR_029717.1 | chr1:23291700-23291784 |
| 12617 | Mir22 | NR_029739.1 | chr11:75463715-75463810 | 12714 | Mir30d | NR_029718.1 | chr15:68341207-68341289 |
| 12618 | Mir221 | NR_029806.1 | chrX:19146293-19146388 | 12715 | Mir30f | NR_105851.1 | chr4:120772605-120772697 |
| 12619 | Mir222 | NR_029807.1 | chrX:19146892-19146971 | 12716 | Mir31 | NR_029747.1 | chr4:88910556-88910662 |
| 12620 | Mir223 | NR_029801.1 | chrX:96242816-96242926 | 12717 | Mir3100 | NR_037282.1 | chr7:19086827-19086892 |
| 12621 | Mir22hg | NR_030711.1 | chr11:75461538-75466690 | 12718 | Mir3101 | NR_037283.1 | chr7:27176005-27176093 |
| 12622 | Mir23a | NR_029740.1 | chr8:84208517-84208592 | 12719 | Mir3102 | NR_037289.1 | chr7:100882305-100882409 |
| 12623 | Mir23b | NR_029530.1 | chr13:63300483-63300557 | 12720 | Mir3103 | NR_037290.1 | chr7:128288368-128288435 |
| 12624 | Mir24-1 | NR_029575.1 | chr13:63301207-63301275 | 12721 | Mir3104 | NR_037291.1 | chr7:141992178-141992241 |
| 12625 | Mir24-2 | NR_029741.1 | chr8:84208814-84208921 | 12722 | Mir3106 | NR_037214.1 | chr8:16168756-16168838 |
| 12626 | Mir25 | NR_029787.1 | chr5:138165320-138165404 | 12723 | Mir3107 | NR_037293.1 | chr8:23142572-23142662 |
| 12627 | Mir26a-1 | NR_029742.1 | chr9:119031795-119031885 | 12724 | Mir3108 | NR_037295.1 | chr8:108936859-108936938 |
| 12628 | Mir26a-2 | NR_029803.1 | chr10:126995529-126995613 | 12725 | Mir3109 | NR_037296.1 | chr9:69456943-69457031 |
| 12629 | Mir26b | NR_029743.1 | chr1:74394309-74394394 | 12726 | Mir3110 | NR_037297.1 | chrX:38210440-38210520 |
| 12630 | Mir27a | NR_029746.1 | chr8:84208671-84208758 | 12727 | Mir3112 | NR_037299.1 | chrX:134075315-134075397 |
| 12631 | Mir27b | NR_029531.1 | chr13:63300711-63300784 | 12728 | Mir32 | NR_029789.1 | chr4:56895228-56895298 |
| 12632 | Mir28 | NR_029788.1 | chr18:52123524-52123546 | 12729 | Mir320 | NR_029802.1 | chr14:70443509-70443591 |
| 12633 | Mir2861 | NR_037217.1 | chr2:32712806-32712888 | 12730 | Mir322 | NR_029756.1 | chrX:53054254-53054349 |
| 12634 | Mir28b | NR_039551.1 | chr8:73016511-73016591 | 12731 | Mir323 | NR_029757.1 | chr12:109712507-109712593 |
| 12635 | Mir28c | NR_039538.1 | chr8:96545953-96545973 | 12732 | Mir324 | NR_029758.1 | chr11:70012042-70012131 |
| 12636 | Mir290 | NR_029640.1 | chr7:3218625-3218708 | 12733 | Mir325 | NR_029759.1 | chr13:107761060-107761087 |
| 12637 | Mir290b | NR_106158.1 | chr7:3218637-3218695 | 12734 | Mir326 | NR_029760.1 | chr7:99552268-99552363 |
| 12638 | Mir291a | NR_029641.1 | chr7:3218918-3219000 | 12735 | Mir328 | NR_029761.1 | chr8:105308363-105308460 |
| 12639 | Mir291b | NR_030276.1 | chr7:3219481-3219560 | 12736 | Mir329 | NR_029762.1 | chr12:109713480-109713577 |
| 12640 | Mir292 | NR_029642.1 | chr7:3219188-3219270 | 12737 | Mir33 | NR_029804.1 | chr15:82198121-82198190 |
| 12641 | Mir292b | NR_106140.1 | chr7:3219197-3219259 | 12738 | Mir330 | NR_029763.1 | chr7:19181464-19181562 |
| 12642 | Mir293 | NR_029643.1 | chr7:3220342-3220422 | 12739 | Mir331 | NR_029764.1 | chr10:93963767-93963863 |
| 12643 | Mir294 | NR_029644.1 | chr7:3220640-3220724 | 12740 | Mir335 | NR_029900.1 | chr6:30741298-30741396 |
| 12644 | Mir295 | NR_029645.1 | chr7:3220772-3220841 | 12741 | Mir337 | NR_029765.1 | chr12:109585788-109585885 |
| 12645 | Mir296 | NR_029646.1 | chr2:174267046-174267125 | 12742 | Mir338 | NR_029767.1 | chr11:120014764-120014862 |
| 12646 | Mir297-1 | NR_029647.1 | chr14:34645143-34653574 | 12743 | Mir339 | NR_029768.1 | chr5:139369649-139369745 |
| 12647 | Mir297-2 | NR_029648.2 | chr2:10472253-10472343 | 12744 | Mir340 | NR_029769.1 | chr11:50069701-50069799 |
| 12648 | Mir297a-3 | NR_030551.1 | chr2:10472253-10511772 | 12745 | Mir341 | NR_029770.1 | chr12:109611499-109611595 |
| 12649 | Mir297a-4 | NR_030552.1 | chr2:10472253-10472340 | 12746 | Mir343 | NR_030759.1 | chr7:19386642-19386717 |
| 12650 | Mir297b | NR_030474.1 | chr2:10511666-10511775 | 12747 | Mir344 | NR_029772.1 | chr7:61877769-61877864 |
| 12651 | Mir297c | NR_030555.1 | chr1:185537335-185988795 | 12748 | Mir344-2 | NR_030557.1 | chr7:61940026-61940106 |
| 12652 | Mir298 | NR_029649.1 | chr2:174267503-174267585 | 12749 | Mir344b | NR_037285.1 | chr7:61790518-61790581 |
| 12653 | Mir299 | NR_029650.1 | chr12:109710637-109710700 | 12750 | Mir344c | NR_037286.1 | chr7:61837310-61837402 |
| 12654 | Mir299b | NR_049185.1 | chr12:109710644-109710693 | 12751 | Mir344d-1 | NR_037215.1 | chr7:61683123-61683192 |
| 12655 | Mir29a | NR_029744.1 | chr6:31062659-31062747 | 12752 | Mir344d-2 | NR_037216.1 | chr7:61685271-61685351 |
| 12656 | Mir29b-1 | NR_029532.1 | chr6:31063022-31063093 | 12753 | Mir344d-3 | NR_037209.1 | chr7:61726248-61726328 |
| 12657 | Mir29b-2 | NR_029809.1 | chr1:195037039-195037120 | 12754 | Mir344e | NR_037284.1 | chr7:61735536-61735602 |
| 12658 | Mir29c | NR_029745.1 | chr1:195037546-195037634 | 12755 | Mir344f | NR_037288.1 | chr7:62046180-62046248 |
| 12659 | Mir300 | NR_029651.1 | chr12:109724312-109724391 | 12756 | Mir344g | NR_037287.1 | chr7:61982288-61982359 |
| 12660 | Mir301 | NR_029652.1 | chr11:87113003-87113089 | 12757 | Mir344h-1 | NR_049201.1 | chr7:61739349-61742425 |
| 12661 | Mir301b | NR_030415.1 | chr16:17124399-17124496 | 12758 | Mir344i | NR_049204.1 | chr7:62085222-62085310 |
| 12662 | Mir302a | NR_029653.1 | chr3:127545495-127545564 | 12759 | Mir345 | NR_029773.1 | chr12:108836972-108837068 |
| 12663 | Mir302b | NR_030403.1 | chr3:127545227-127545301 | 12760 | Mir346 | NR_029774.1 | chr14:34894608-34894706 |
| 12664 | Mir302c | NR_030404.1 | chr3:127545362-127545430 | 12761 | Mir3470a | NR_037301.1 | chr11:3239901-3462990 |
| 12665 | Mir302d | NR_030405.1 | chr3:127545623-127545689 | 12762 | Mir3470b | NR_037302.1 | chr7:143948404-144443433 |
| 12666 | Mir3057 | NR_037218.1 | chr10:81271596-81271687 | 12763 | Mir3471-1 | NR_037304.1 | chr5:23813937-24129968 |
| 12667 | Mir3058 | NR_037212.1 | chr10:95559230-95559321 | 12764 | Mir3473 | NR_037311.1 | chrX:13719852-162874995 |
| 12668 | Mir3059 | NR_037211.1 | chr10:101772691-101772772 | 12765 | Mir3473c | NR_039566.1 | chr1:191998553-191998634 |
| 12669 | Mir3060 | NR_037220.1 | chr11:4139363-4139446 | 12766 | Mir3473d | NR_039583.1 | chr8:111016449-111016530 |
| 12670 | Mir3061 | NR_037221.1 | chr11:52126745-52126836 | 12767 | Mir3473e | NR_105859.1 | chr5:31667230-31667340 |
| 12671 | Mir3062 | NR_037222.1 | chr11:68990573-68990678 | 12768 | Mir3473f | NR_106164.1 | chr1:106546495-106546631 |
| 12672 | Mir3063 | NR_037223.1 | chr11:95963291-95963383 | 12769 | Mir3473g | NR_106202.1 | chr2:126902249-126902385 |
| 12673 | Mir3064 | NR_037224.1 | chr11:106782692-106782759 | 12770 | Mir3474 | NR_037312.1 | chr2:158638582-158638640 |
| 12674 | Mir3065 | NR_037225.1 | chr11:120014766-120014853 | 12771 | Mir3475 | NR_037208.1 | chrX:140310947-140311012 |
| 12675 | Mir3066 | NR_037226.1 | chr12:17355391-17355474 | 12772 | Mir34a | NR_029751.1 | chr4:150068453-150068555 |
| 12676 | Mir3067 | NR_037227.1 | chr12:81166150-81166234 | 12773 | Mir34b | NR_029655.1 | chr9:51103561-51103645 |
| 12677 | Mir3068 | NR_037228.1 | chr12:87437678-87437757 | 12774 | Mir34c | NR_029654.1 | chr9:51103033-51103110 |
| 12678 | Mir3069 | NR_037229.1 | chr12:105031076-105031141 | 12775 | Mir350 | NR_029775.1 | chr1:176772324-176772423 |
| 12679 | Mir3070a | NR_037230.1 | chr12:109587942-109588031 | 12776 | Mir351 | NR_029776.1 | chrX:53053254-53053353 |
| 12680 | Mir3070b | NR_037231.1 | chr12:109588591-109588680 | 12777 | Mir3535 | NR_106184.1 | chr1:86351980-86352127 |
| 12681 | Mir3071 | NR_037232.1 | chr12:109595317-109595397 | 12778 | Mir3544 | NR_049184.1 | chr12:109585812-109585873 |
| 12682 | Mir3072 | NR_037233.1 | chr12:109747877-109747960 | 12779 | Mir3547 | NR_105931.1 | chr17:25245550-25245638 |
| 12683 | Mir3073 | NR_037234.1 | chr12:112109237-112109322 | 12780 | Mir3569 | NR_106134.1 | chr7:30589379-30589437 |
| 12684 | Mir3073b | NR_049197.1 | chr12:112109249-112109309 | 12781 | Mir3572 | NR_039587.1 | chr7:3655961-3656047 |
| 12685 | Mir3074-1 | NR_037235.1 | chr13:63301198-63301283 | 12782 | Mir362 | NR_029851.1 | chrX:7241982-7242046 |
| 12686 | Mir3074-2 | NR_037294.1 | chr8:84208824-84208907 | 12783 | Mir3620 | NR_106147.1 | chrX:150547384-150547445 |
| 12687 | Mir3075 | NR_037236.1 | chr14:25534438-25534523 | 12784 | Mir363 | NR_029853.1 | chrX:52741692-52741767 |
| 12688 | Mir3076 | NR_037237.1 | chr14:30572148-30572208 | 12785 | Mir365-1 | NR_029855.1 | chr16:13453839-13453926 |
| 12689 | Mir3077 | NR_037238.1 | chr14:57798423-57798487 | 12786 | Mir365-2 | NR_029959.1 | chr11:79726399-79726511 |
| 12690 | Mir3078 | NR_037239.1 | chr14:64591184-64591271 | 12787 | Mir367 | NR_030268.1 | chr3:127545732-127545807 |
| 12691 | Mir3079 | NR_037240.1 | chr11:5254597-5254622 | 12788 | Mir369 | NR_030272.1 | chr12:109743417-109743496 |
| 12692 | Mir3081 | NR_037242.1 | chr16:44558045-44558129 | 12789 | Mir370 | NR_029918.1 | chr12:109618257-109618336 |
| 12693 | Mir3082 | NR_037243.1 | chr17:25831364-25831428 | 12790 | Mir374 | NR_030418.1 | chrX:103573059-103573154 |
| 12694 | Mir3083 | NR_037244.1 | chr17:26948055-26948119 | 12791 | Mir374c | NR_037298.1 | chrX:103573084-103573133 |
| 12695 | Mir3084 | NR_037245.1 | chr19:24942235-24942303 | 12792 | Mir375 | NR_029876.1 | chr1:74900657-74900721 |
| 12696 | Mir3084-2 | NR_105743.1 | chr19:24942235-60774397 | 12793 | Mir376a | NR_029877.1 | chr12:109723780-109723848 |
| 12697 | Mir3085 | NR_037246.1 | chr19:42280081-42280170 | 12794 | Mir376b | NR_029915.1 | chr12:109723457-109723539 |
| 12698 | Mir3086 | NR_037247.1 | chr19:58911672-58911759 | 12795 | Mir376c | NR_030270.1 | chr12:109722717-109722802 |
| 12699 | Mir3087 | NR_037270.1 | chr2:25442778-25442834 | 12796 | Mir377 | NR_029878.1 | chr12:109740509-109740577 |
| 12700 | Mir3088 | NR_037271.1 | chr2:28733277-28733351 | 12797 | Mir378 | NR_029879.1 | chr9:71963440-71963460 |
| 12701 | Mir3089 | NR_037272.1 | chr2:30721209-30721293 | 12798 | Mir378b | NR_039545.1 | chr11:88352772-88352864 |
| 12702 | Mir3091 | NR_037274.1 | chr2:180257535-180257611 | 12799 | Mir378c | NR_105813.1 | chr3:97309255-97411316 |
| 12703 | Mir3092 | NR_037275.1 | chr3:27584899-27584995 | 12800 | Mir379 | NR_029880.1 | chr12:109709059-109709125 |
| 12704 | Mir3093 | NR_037276.1 | chr3:88215170-88215257 | 12801 | Mir380 | NR_029881.1 | chr12:109711802-109711863 |
| 12705 | Mir3094 | NR_037277.1 | chr4:40993694-40993758 | 12802 | Mir381 | NR_029882.1 | chr12:109726821-109726896 |
| 12706 | Mir3095 | NR_037278.1 | chr4:58441011-58441098 | 12803 | Mir382 | NR_029883.1 | chr12:109733770-109733846 |

Fig.21 - 67

| | | | | | | | |
|---|---|---|---|---|---|---|---|
| 12804 | Mir383 | NR_029884.1 | chr8:38252132-38252202 | 12901 | Mir5104 | NR_039563.1 | chr10:7836380-7836474 |
| 12805 | Mir384 | NR_029910.1 | chrX:105344281-105344369 | 12902 | Mir5106 | NR_039565.1 | chr4:44221194-44221267 |
| 12806 | Mir3960 | NR_039536.1 | chr2:32712899-32712972 | 12903 | Mir5107 | NR_039567.1 | chr18:60812075-60812163 |
| 12807 | Mir3962 | NR_039539.1 | chr7:144932115-144932135 | 12904 | Mir5108 | NR_039568.1 | chr10:61774736-61774821 |
| 12808 | Mir3963 | NR_039540.1 | chr2:135405829-135405850 | 12905 | Mir511 | NR_030609.1 | chr2:14261002-14261081 |
| 12809 | Mir3964 | NR_039542.1 | chr14:109460229-109490814 | 12906 | Mir5112 | NR_039572.1 | chr18:82720280-82720340 |
| 12810 | Mir3965 | NR_039544.1 | chr7:99641529-99641549 | 12907 | Mir5113 | NR_039573.1 | chr15:80940038-80940120 |
| 12811 | Mir3966 | NR_039547.1 | chr10:97423477-97423562 | 12908 | Mir5114 | NR_039574.1 | chr19:44303170-44303231 |
| 12812 | Mir3967 | NR_039548.1 | chr2:22654282-22654350 | 12909 | Mir5116 | NR_039576.1 | chrX:56587851-56587914 |
| 12813 | Mir3968 | NR_039549.1 | chr11:115447960-115448060 | 12910 | Mir5119 | NR_039579.1 | chr11:98262605-98262662 |
| 12814 | Mir3969 | NR_039550.1 | chr5:87595901-87595993 | 12911 | Mir5120 | NR_039580.1 | chr9:107436735-107436755 |
| 12815 | Mir3970 | NR_039552.1 | chr13:27010322-27010345 | 12912 | Mir5121 | NR_039581.1 | chr7:45126917-45126991 |
| 12816 | Mir3971 | NR_039553.1 | chr11:75551428-75551511 | 12913 | Mir5122 | NR_039582.1 | chr4:133369775-133369864 |
| 12817 | Mir409 | NR_029913.1 | chr12:109743157-109743236 | 12914 | Mir5123 | NR_039584.1 | chr4:40850055-40850138 |
| 12818 | Mir410 | NR_029914.1 | chr12:109743714-109743795 | 12915 | Mir5124 | NR_039585.1 | chr3:93428490-93428511 |
| 12819 | Mir411 | NR_029916.1 | chr12:109710174-109710256 | 12916 | Mir5125 | NR_039586.1 | chr12:48219729-48301462 |
| 12820 | Mir412 | NR_029917.1 | chr12:109743288-109743368 | 12917 | Mir5126 | NR_039588.1 | chr1:84695838-84695915 |
| 12821 | Mir421 | NR_030558.1 | chrX:103572920-103572996 | 12918 | Mir5127 | NR_039589.1 | chr18:81992009-81992080 |
| 12822 | Mir423 | NR_030756.1 | chr11:77078063-77078172 | 12919 | Mir5128 | NR_039590.1 | chr9:113951595-113993800 |
| 12823 | Mir425 | NR_029947.1 | chr9:108568776-108568861 | 12920 | Mir5129 | NR_039591.1 | chr2:45023099-45023177 |
| 12824 | Mir429 | NR_029958.1 | chr4:156053904-156053987 | 12921 | Mir5130 | NR_039592.1 | chr14:102982548-102982632 |
| 12825 | Mir431 | NR_029951.1 | chr12:109590446-109590537 | 12922 | Mir5131 | NR_039593.1 | chr14:45657960-45658053 |
| 12826 | Mir432 | NR_035526.1 | chr12:109594955-109595030 | 12923 | Mir5132 | NR_039594.1 | chrX:74023527-74023598 |
| 12827 | Mir433 | NR_029952.1 | chr12:109591714-109591838 | 12924 | Mir5133 | NR_039595.1 | chr9:62122517-62122594 |
| 12828 | Mir434 | NR_029953.1 | chr12:109594505-109594599 | 12925 | Mir5134 | NR_039596.1 | chr17:24234526-24234604 |
| 12829 | Mir448 | NR_029956.1 | chrX:147158209-147158321 | 12926 | Mir5135 | NR_039597.1 | chr12:76533133-76533212 |
| 12830 | Mir449a | NR_029961.1 | chr13:113037533-113037624 | 12927 | Mir5136 | NR_039598.1 | chr19:8888698-8888774 |
| 12831 | Mir449b | NR_030602.1 | chr13:113037418-113037498 | 12928 | Mir532 | NR_030242.1 | chrX:7248401-7248497 |
| 12832 | Mir449c | NR_030452.1 | chr13:113035982-113036091 | 12929 | Mir539 | NR_030262.1 | chr12:109728128-109728202 |
| 12833 | Mir450-1 | NR_029963.1 | chrX:53048153-53048244 | 12930 | Mir540 | NR_030260.1 | chr12:109586079-109586146 |
| 12834 | Mir450-2 | NR_030274.1 | chrX:53048298-53048367 | 12931 | Mir541 | NR_030263.1 | chr12:109742408-109742498 |
| 12835 | Mir450b | NR_030498.1 | chrX:53047996-53048078 | 12932 | Mir542 | NR_030264.1 | chrX:53049402-53049487 |
| 12836 | Mir451 | NR_029971.1 | chr11:78073169-78073241 | 12933 | Mir543 | NR_030261.1 | chr12:109717257-109717333 |
| 12837 | Mir452 | NR_029974.1 | chrX:72262223-72262308 | 12934 | Mir544 | NR_030610.1 | chr12:109729324-109729402 |
| 12838 | Mir453 | NR_030559.1 | chr12:109735618-109735700 | 12935 | Mir546 | NR_030259.1 | chr10:126998439-126998560 |
| 12839 | Mir455 | NR_030477.1 | chr4:63256850-63256932 | 12936 | Mir547 | NR_030265.1 | chrX:67988373-67988451 |
| 12840 | Mir463 | NR_030147.1 | chrX:66799222-66799297 | 12937 | Mir551b | NR_030422.1 | chr3:29416822-29416920 |
| 12841 | Mir465 | NR_030149.1 | chrX:66839051-66839125 | 12938 | Mir5615-1 | NR_049186.1 | chr10:81104613-81104673 |
| 12842 | Mir465b-1 | NR_030560.1 | chrX:66829201-66835842 | 12939 | Mir5615-2 | NR_049187.1 | chr10:81104615-81104675 |
| 12843 | Mir465c-1 | NR_030562.1 | chrX:66825954-66832597 | 12940 | Mir5616 | NR_049189.1 | chr4:149537862-149537922 |
| 12844 | Mir465d | NR_106148.1 | chrX:66822655-66822713 | 12941 | Mir5617 | NR_049190.1 | chrX:20863125-20863182 |
| 12845 | Mir466 | NR_030150.1 | chr14:112021429-112058438 | 12942 | Mir5618 | NR_049191.1 | chr9:7784389-7784440 |
| 12846 | Mir4660 | NR_037248.1 | chr12:70308504-70669399 | 12943 | Mir5619 | NR_049192.1 | chr5:104048002-104048063 |
| 12847 | Mir466b-2 | NR_030564.1 | chr2:3523300-3523321 | 12944 | Mir5620 | NR_049193.1 | chr7:7298890-7298946 |
| 12848 | Mir466b-3 | NR_030565.1 | chr2:10503564-10503645 | 12945 | Mir5621 | NR_049194.1 | chr11:115795823-115795886 |
| 12849 | Mir466d | NR_030601.1 | chr2:5379389-5483389 | 12946 | Mir5622 | NR_049195.1 | chr2:152865066-152865126 |
| 12850 | Mir466f-1 | NR_030566.1 | chr19:12071789-12413554 | 12947 | Mir5623 | NR_049196.1 | chr19:58051166-58051236 |
| 12851 | Mir466f-2 | NR_030567.1 | chr7:28452226-28482345 | 12948 | Mir5624 | NR_049198.1 | chr13:93790776-93790837 |
| 12852 | Mir466f-3 | NR_030568.1 | chr14:75004109-75388146 | 12949 | Mir5625 | NR_049199.1 | chr5:30647935-30648015 |
| 12853 | Mir466g | NR_030569.1 | chr2:10514594-10514674 | 12950 | Mir5626 | NR_049200.1 | chr9:70405693-70405753 |
| 12854 | Mir466h | NR_030570.1 | chr4:81476308-81487013 | 12951 | Mir5627 | NR_049203.1 | chr12:44210312-44210373 |
| 12855 | Mir466i | NR_035412.1 | chr13:17747472-17747593 | 12952 | Mir568 | NR_030576.1 | chr16:43640654-43640737 |
| 12856 | Mir466n | NR_037269.1 | chr11:114344525-114749749 | 12953 | Mir5709 | NR_049205.1 | chr17:67026146-67026235 |
| 12857 | Mir466p | NR_105742.1 | chr18:65195452-65619615 | 12954 | Mir5710 | NR_049206.1 | chr9:54708312-54708381 |
| 12858 | Mir467a-1 | NR_035406.1 | chr2:10476345-10503350 | 12955 | Mir574 | NR_030577.1 | chr5:64970317-64970395 |
| 12859 | Mir467a-10 | NR_037267.1 | chr2:10503273-10503355 | 12956 | Mir582 | NR_030644.1 | chr13:109324743-109324823 |
| 12860 | Mir467a-2 | NR_037249.1 | chr2:10478798-10478880 | 12957 | Mir592 | NR_030420.1 | chr6:27936654-27936750 |
| 12861 | Mir467a-3 | NR_037252.1 | chr2:10483677-10503355 | 12958 | Mir598 | NR_030611.1 | chr14:63727188-63727267 |
| 12862 | Mir467a-5 | NR_037256.1 | chr2:10476341-10500904 | 12959 | Mir599 | NR_035527.1 | chr15:35660830-35660918 |
| 12863 | Mir467a-7 | NR_037261.1 | chr2:10493510-10493592 | 12960 | Mir615 | NR_030526.1 | chr15:103014909-103015001 |
| 12864 | Mir467a-9 | NR_037265.1 | chr2:10498393-10498475 | 12961 | Mir6236 | NR_105744.1 | chr9:110281286-110281409 |
| 12865 | Mir467b | NR_030472.1 | chr2:10481247-10481320 | 12962 | Mir6237 | NR_105745.1 | chr9:9894430-9894521 |
| 12866 | Mir467c | NR_030571.1 | chr11:86415843-86520291 | 12963 | Mir6238 | NR_105746.1 | chr7:53891760-53891889 |
| 12867 | Mir467d | NR_030572.1 | chr2:10507629-10507714 | 12964 | Mir6239 | NR_105747.1 | chr14:117953742-117953847 |
| 12868 | Mir467e | NR_030645.1 | chr1:10708386-10865874 | 12965 | Mir6241 | NR_105749.1 | chr14:118258633-118258736 |
| 12869 | Mir467f | NR_035420.1 | chr18:52822107-52902647 | 12966 | Mir6244 | NR_105750.1 | chr9:52115622-52115739 |
| 12870 | Mir468 | NR_030151.1 | chr6:81896598-81896676 | 12967 | Mir6335 | NR_105752.1 | chr2:67781042-67781140 |
| 12871 | Mir470 | NR_030153.1 | chrX:66813950-66814025 | 12968 | Mir6336 | NR_105754.1 | chr2:42447438-42447568 |
| 12872 | Mir471 | NR_030154.1 | chrX:66792594-66792661 | 12969 | Mir6337 | NR_105755.1 | chr2:65364295-65364400 |
| 12873 | Mir483 | NR_030251.1 | chr7:142654923-142654996 | 12970 | Mir6338 | NR_105756.1 | chr11:72388755-72388864 |
| 12874 | Mir484 | NR_030252.1 | chr16:14159625-14159692 | 12971 | Mir6339 | NR_105757.1 | chr2:130016291-130016408 |
| 12875 | Mir485 | NR_030253.1 | chr12:109734901-109734974 | 12972 | Mir6340 | NR_105758.1 | chr2:173701029-173701148 |
| 12876 | Mir486 | NR_030254.1 | chr8:23142554-23142682 | 12973 | Mir6341 | NR_105759.1 | chr1:12425985-12426106 |
| 12877 | Mir487b | NR_030271.1 | chr12:109727332-109727414 | 12974 | Mir6342 | NR_105760.1 | chr1:29421487-29421612 |
| 12878 | Mir488 | NR_030441.1 | chr1:158505624-158505733 | 12975 | Mir6343 | NR_105761.1 | chr1:76509559-76509643 |
| 12879 | Mir489 | NR_030250.1 | chr6:3721896-3722003 | 12976 | Mir6344 | NR_105762.1 | chr1:82131915-82132019 |
| 12880 | Mir490 | NR_030524.1 | chr6:36421741-36421825 | 12977 | Mir6345 | NR_105763.1 | chr1:93352157-93352285 |
| 12881 | Mir491 | NR_030478.1 | chr4:88122039-88122125 | 12978 | Mir6348 | NR_105766.1 | chr1:168490642-168490762 |
| 12882 | Mir493 | NR_030573.1 | chr12:109580232-109580315 | 12979 | Mir6349 | NR_105767.1 | chr1:39186895-39186992 |
| 12883 | Mir494 | NR_030269.1 | chr12:109715317-109715402 | 12980 | Mir6350 | NR_105768.1 | chr1:47467020-47467125 |
| 12884 | Mir495 | NR_030446.1 | chr12:109718753-109718816 | 12981 | Mir6352 | NR_105770.1 | chr1:77496652-77496768 |
| 12885 | Mir496 | NR_030437.1 | chr12:109739118-109739197 | 12982 | Mir6353 | NR_105771.1 | chr1:84218848-84218961 |
| 12886 | Mir496b | NR_105832.1 | chr19:16314891-16315004 | 12983 | Mir6354 | NR_105772.1 | chr11:62960060-63386069 |
| 12887 | Mir497 | NR_030444.1 | chr11:70234716-70234800 | 12984 | Mir6355 | NR_105773.1 | chr18:59579629-59579735 |
| 12888 | Mir497b | NR_106178.1 | chr11:70234691-70234816 | 12985 | Mir6356 | NR_105774.1 | chr18:68739399-68739501 |
| 12889 | Mir499 | NR_030757.1 | chr2:155622879-155622958 | 12986 | Mir6357 | NR_105775.1 | chr18:70516785-70516889 |
| 12890 | Mir500 | NR_030495.1 | chrX:7237682-7237774 | 12987 | Mir6358 | NR_105776.1 | chr18:76170551-76170652 |
| 12891 | Mir501 | NR_030496.1 | chrX:7241242-7241351 | 12988 | Mir6359 | NR_105777.1 | chr18:88972029-88972110 |
| 12892 | Mir503 | NR_030275.1 | chrX:53053983-53054054 | 12989 | Mir6360 | NR_105778.1 | chr18:23774863-23774989 |
| 12893 | Mir504 | NR_030574.1 | chrX:59097657-59097736 | 12990 | Mir6361 | NR_105779.1 | chr18:32880572-32880673 |
| 12894 | Mir5046 | NR_039555.1 | chr19:6829885-6829944 | 12991 | Mir6362 | NR_105781.1 | chr5:29789010-29970488 |
| 12895 | Mir505 | NR_030499.1 | chrX:60394402-60394492 | 12992 | Mir6363 | NR_105782.1 | chr16:50727131-50727245 |
| 12896 | Mir509 | NR_030575.1 | chrX:68010104-68010179 | 12993 | Mir6364 | NR_105783.1 | chr2:166672717-166722152 |
| 12897 | Mir5098 | NR_039557.1 | chr2:28127350-119928917 | 12994 | Mir6365 | NR_105784.1 | chr16:13471079-13471180 |
| 12898 | Mir5100 | NR_039559.1 | chr11:60728662-60728726 | 12995 | Mir6366 | NR_105785.1 | chr16:18165088-18165170 |
| 12899 | Mir5101 | NR_039560.1 | chr12:75909102-75909185 | 12996 | Mir6367 | NR_105786.1 | chr19:9017903-9017931 |
| 12900 | Mir5103 | NR_039562.1 | chr1:34433120-34433199 | 12997 | Mir6368 | NR_105787.1 | chr13:28710780-28710873 |

Fig.21 - 68

| | | | | | | | |
|---|---|---|---|---|---|---|---|
| 12998 | Mir6369 | NR_105788.1 | chr13:58313043-58313149 | 13095 | Mir684-1 | NR_030454.1 | chr2:7753088-129665839 |
| 12999 | Mir6370 | NR_105789.1 | chr6:19555341-19555461 | 13096 | Mir684-2 | NR_030455.1 | chr4:11134096-11134182 |
| 13000 | Mir6372 | NR_105791.1 | chr11:26202239-26202348 | 13097 | Mir686 | NR_030457.1 | chr14:54616676-54616785 |
| 13001 | Mir6373 | NR_105792.1 | chr6:102794383-102794485 | 13098 | Mir687 | NR_030459.1 | chr14:73206762-73206851 |
| 13002 | Mir6374 | NR_105793.1 | chr6:83400995-83401102 | 13099 | Mir688 | NR_030460.1 | chr15:102671791-102671866 |
| 13003 | Mir6375 | NR_105794.1 | chr6:85790139-85790234 | 13100 | Mir6896 | NR_105861.1 | chr1:34117358-34117431 |
| 13004 | Mir6376 | NR_105795.1 | chr6:88104127-88104258 | 13101 | Mir6897 | NR_105862.1 | chr1:36144251-36144313 |
| 13005 | Mir6378 | NR_105799.1 | chr3:34922544-34922651 | 13102 | Mir6898 | NR_105863.1 | chr1:36348681-36348758 |
| 13006 | Mir6380 | NR_105801.1 | chr11:47739591-48074583 | 13103 | Mir6899 | NR_105864.1 | chr1:64042437-64042501 |
| 13007 | Mir6381 | NR_105802.1 | chr3:142623859-142623946 | 13104 | Mir690 | NR_030463.1 | chr16:28599934-28600043 |
| 13008 | Mir6382 | NR_105803.1 | chrX:106309825-106309935 | 13105 | Mir6900 | NR_105865.1 | chr1:92464482-92464542 |
| 13009 | Mir6383 | NR_105804.1 | chrX:136183244-136183360 | 13106 | Mir6901 | NR_105866.1 | chr1:93274548-93274610 |
| 13010 | Mir6384 | NR_105805.1 | chrX:52378120-52378181 | 13107 | Mir6902 | NR_105867.1 | chr1:93346091-93346155 |
| 13011 | Mir6385 | NR_105806.1 | chr9:58221149-58221254 | 13108 | Mir6903 | NR_105868.1 | chr1:133726557-133726645 |
| 13012 | Mir6386 | NR_105808.1 | chr9:92267257-92267361 | 13109 | Mir6904 | NR_105869.1 | chr1:180587200-180587267 |
| 13013 | Mir6387 | NR_105809.1 | chr12:15800993-15801101 | 13110 | Mir6905 | NR_105870.1 | chr10:24910663-24910733 |
| 13014 | Mir6388 | NR_105810.1 | chr12:115695719-115695794 | 13111 | Mir6906 | NR_105871.1 | chr10:60126775-60126833 |
| 13015 | Mir6389 | NR_105812.1 | chr7:57581060-57581162 | 13112 | Mir6907 | NR_105872.1 | chr10:78408474-78408545 |
| 13016 | Mir6390 | NR_105814.1 | chr14:105623641-105623770 | 13113 | Mir6908 | NR_105873.1 | chr10:78445094-78445157 |
| 13017 | Mir6391 | NR_105815.1 | chr14:118592368-118592472 | 13114 | Mir6909 | NR_105874.1 | chr10:79531028-79531090 |
| 13018 | Mir6392 | NR_105816.1 | chr15:84168269-84168377 | 13115 | Mir691 | NR_030464.1 | chr16:74341989-74342067 |
| 13019 | Mir6393 | NR_105817.1 | chr15:87795849-87795943 | 13116 | Mir6910 | NR_105875.1 | chr10:79862586-79862652 |
| 13020 | Mir6394 | NR_105818.1 | chr7:96305666-96305765 | 13117 | Mir6911 | NR_105876.1 | chr10:80278163-80278232 |
| 13021 | Mir6395 | NR_105820.1 | chr8:34166621-34166712 | 13118 | Mir6912 | NR_105877.1 | chr10:80609259-80609329 |
| 13022 | Mir6396 | NR_105821.1 | chr8:122622264-122622375 | 13119 | Mir6913 | NR_105878.1 | chr10:81386346-81386412 |
| 13023 | Mir6397 | NR_105823.1 | chr4:108087356-108087449 | 13120 | Mir6914 | NR_105879.1 | chr10:128382837-128382906 |
| 13024 | Mir6398 | NR_105824.1 | chr4:123616419-123616521 | 13121 | Mir6915 | NR_105880.1 | chr10:128388831-128388900 |
| 13025 | Mir6399 | NR_105825.1 | chr4:138163208-138163292 | 13122 | Mir6916 | NR_105881.1 | chr10:128511967-128512030 |
| 13026 | Mir6400 | NR_105826.1 | chr4:15942899-15942991 | 13123 | Mir6917 | NR_105882.1 | chr10:128584436-128584497 |
| 13027 | Mir6401 | NR_105827.1 | chr7:139779017-139779126 | 13124 | Mir6918 | NR_105883.1 | chr11:4891344-4891402 |
| 13028 | Mir6402 | NR_105828.1 | chr4:91373283-91373363 | 13125 | Mir6919 | NR_105884.1 | chr11:50232244-50232306 |
| 13029 | Mir6403 | NR_105829.1 | chr4:134567722-134567851 | 13126 | Mir6920 | NR_105885.1 | chr11:53409196-53409267 |
| 13030 | Mir6404 | NR_105830.1 | chr7:143933114-143933208 | 13127 | Mir6921 | NR_105886.1 | chr11:60200537-60200617 |
| 13031 | Mir6405 | NR_105831.1 | chr19:44808405-44808529 | 13128 | Mir692-1 | NR_030465.1 | chr17:6895228-6895337 |
| 13032 | Mir6406 | NR_105833.1 | chr11:68220891-68221011 | 13129 | Mir6922 | NR_105887.1 | chr11:60202261-60202327 |
| 13033 | Mir6407 | NR_105834.1 | chr19:53010655-53010756 | 13130 | Mir692-2b | NR_035409.1 | chr4:74407064-125504857 |
| 13034 | Mir6408 | NR_105835.1 | chr10:60923022-60923113 | 13131 | Mir6923 | NR_105888.1 | chr11:67099803-67099875 |
| 13035 | Mir6409 | NR_105836.1 | chr10:76008925-76009017 | 13132 | Mir6924 | NR_105889.1 | chr11:69882031-69882094 |
| 13036 | Mir6410 | NR_105837.1 | chr10:78434798-78434902 | 13133 | Mir6925 | NR_105890.1 | chr11:70705989-70706059 |
| 13037 | Mir6411 | NR_105838.1 | chr10:104287268-104287376 | 13134 | Mir6926 | NR_105891.1 | chr11:74868126-74868194 |
| 13038 | Mir6412 | NR_105839.1 | chr17:30256456-30256535 | 13135 | Mir6927 | NR_105892.1 | chr11:97677059-97677130 |
| 13039 | Mir6413 | NR_105840.1 | chr10:20297580-20297688 | 13136 | Mir6928 | NR_105893.1 | chr11:101030339-101030409 |
| 13040 | Mir6414 | NR_105842.1 | chr5:41891341-41891423 | 13137 | Mir6929 | NR_105894.1 | chr11:101419186-101419253 |
| 13041 | Mir6415 | NR_105843.1 | chr5:92458556-92458656 | 13138 | Mir693 | NR_030466.1 | chr17:46231530-46231619 |
| 13042 | Mir6416 | NR_105844.1 | chr7:64249982-64250099 | 13139 | Mir6930 | NR_105895.1 | chr11:102404919-102404987 |
| 13043 | Mir6417 | NR_105845.1 | chr5:52135901-52136017 | 13140 | Mir6931 | NR_105896.1 | chr11:102999847-102999921 |
| 13044 | Mir6418 | NR_105846.1 | chr5:137529479-137529594 | 13141 | Mir6932 | NR_105897.1 | chr11:107637721-107637779 |
| 13045 | Mir6419 | NR_105847.1 | chr2:17563513-17563626 | 13142 | Mir6933 | NR_105898.1 | chr11:118002269-118002350 |
| 13046 | Mir6420 | NR_105848.1 | chr17:64440805-64440907 | 13143 | Mir6934 | NR_105899.1 | chr11:119154075-119154137 |
| 13047 | Mir6481 | NR_105852.1 | chr3:99199344-99199453 | 13144 | Mir6935 | NR_105900.1 | chr11:120347349-120347413 |
| 13048 | Mir6516 | NR_105853.1 | chr11:117077347-117077457 | 13145 | Mir6936 | NR_105901.1 | chr11:120624822-120624876 |
| 13049 | Mir653 | NR_030612.1 | chr6:3721300-3721385 | 13146 | Mir6937 | NR_105902.1 | chr12:28679324-28679389 |
| 13050 | Mir6537 | NR_105855.1 | chr7:25097065-25097175 | 13147 | Mir6938 | NR_105903.1 | chr12:85245922-85245985 |
| 13051 | Mir6538 | NR_105856.1 | chr12:26414900-26415010 | 13148 | Mir6939 | NR_105904.1 | chr12:112659276-112659351 |
| 13052 | Mir6539 | NR_105857.1 | chr14:67859635-67859745 | 13149 | Mir694 | NR_030467.1 | chr18:66219263-66219333 |
| 13053 | Mir654 | NR_030578.1 | chr12:109723217-109723301 | 13150 | Mir6940 | NR_105905.1 | chr12:112733321-112733391 |
| 13054 | Mir6540 | NR_105858.1 | chr16:42303361-42303469 | 13151 | Mir6941 | NR_105906.1 | chr12:112920482-112920541 |
| 13055 | Mir6541 | NR_105860.1 | chr14:62865541-62865651 | 13152 | Mir6942 | NR_105907.1 | chr13:21396577-21396638 |
| 13056 | Mir6546 | NR_106103.1 | chr1:171064590-171064652 | 13153 | Mir6943 | NR_105908.1 | chr13:55453105-55453171 |
| 13057 | Mir664 | NR_035529.1 | chr1:185242974-185243043 | 13154 | Mir6944 | NR_105909.1 | chr13:55477680-55477776 |
| 13058 | Mir665 | NR_030425.1 | chr12:109586313-109586407 | 13155 | Mir6945 | NR_105910.1 | chr13:55507624-55507692 |
| 13059 | Mir666 | NR_030435.1 | chr12:109717084-109717183 | 13156 | Mir6946 | NR_105911.1 | chr14:20690634-20690703 |
| 13060 | Mir667 | NR_030426.1 | chr12:109720005-109720097 | 13157 | Mir6947 | NR_105912.1 | chr14:29988887-29988952 |
| 13061 | Mir668 | NR_030424.1 | chr12:109734731-109734797 | 13158 | Mir6948 | NR_105913.1 | chr14:54643048-54643110 |
| 13062 | Mir669a-1 | NR_035408.1 | chr2:10476852-10503883 | 13159 | Mir6949 | NR_105914.1 | chr14:56082400-56082468 |
| 13063 | Mir669a-2 | NR_030470.1 | chr2:10476850-10510260 | 13160 | Mir695 | NR_030475.1 | chr2:155356816-155356925 |
| 13064 | Mir669a-3 | NR_030471.1 | chr2:10474432-10474541 | 13161 | Mir6950 | NR_105915.1 | chr14:69692247-69692320 |
| 13065 | Mir669a-4 | NR_037250.1 | chr17:51277987-51613053 | 13162 | Mir6951 | NR_105916.1 | chr15:38490472-38490547 |
| 13066 | Mir669b | NR_030469.1 | chr3:114863252-115027151 | 13163 | Mir6952 | NR_105917.1 | chr15:76064827-76064888 |
| 13067 | Mir669c | NR_030473.1 | chr12:91110228-91123337 | 13164 | Mir6953 | NR_105918.1 | chr15:76248190-76248258 |
| 13068 | Mir669e | NR_035426.1 | chr8:39467447-40227787 | 13165 | Mir6954 | NR_105919.1 | chr15:76433213-76433273 |
| 13069 | Mir669g | NR_035411.1 | chr2:10477149-10477272 | 13166 | Mir6955 | NR_105920.1 | chr15:78891839-78891913 |
| 13070 | Mir669h | NR_035418.1 | chr10:113586577-113614774 | 13167 | Mir6956 | NR_105921.1 | chr15:79002222-79002284 |
| 13071 | Mir669i | NR_035417.1 | chr2:10517603-10517730 | 13168 | Mir6957 | NR_105922.1 | chr15:80646072-80646136 |
| 13072 | Mir669j | NR_035416.1 | chr2:10477909-10478030 | 13169 | Mir6958 | NR_105923.1 | chr15:89185464-89185537 |
| 13073 | Mir669k | NR_035410.1 | chr2:10475299-10475426 | 13170 | Mir6959 | NR_105924.1 | chr15:89305657-89305732 |
| 13074 | Mir669m-1 | NR_035474.1 | chr5:78929669-79515904 | 13171 | Mir6960 | NR_105925.1 | chr15:99080804-99080865 |
| 13075 | Mir669m-2 | NR_035475.1 | chrX:77164402-77475936 | 13172 | Mir6961 | NR_105926.1 | chr15:100649162-100649229 |
| 13076 | Mir669p-1 | NR_037257.1 | chr17:51277987-51613053 | 13173 | Mir6962 | NR_105927.1 | chr15:101193868-101193931 |
| 13077 | Mir670 | NR_030431.1 | chr2:94261299-94261399 | 13174 | Mir6963 | NR_105928.1 | chr15:103350454-103350525 |
| 13078 | Mir671 | NR_030423.1 | chr5:24592113-24592211 | 13175 | Mir6964 | NR_105929.1 | chr16:97877232-97877291 |
| 13079 | Mir6715 | NR_106146.1 | chr19:55192677-55192734 | 13176 | Mir6965 | NR_105930.1 | chr17:24240883-24240947 |
| 13080 | Mir672 | NR_030430.1 | chrX:104116174-104116274 | 13177 | Mir6966 | NR_105932.1 | chr17:25780807-25780879 |
| 13081 | Mir673 | NR_030438.1 | chr12:109571989-109572080 | 13178 | Mir6968 | NR_105934.1 | chr17:26935471-26935539 |
| 13082 | Mir674 | NR_030440.1 | chr2:117185126-117185226 | 13179 | Mir6969 | NR_105935.1 | chr17:28558440-28558501 |
| 13083 | Mir675 | NR_030416.1 | chr7:142577063-142577147 | 13180 | Mir697 | NR_030479.1 | chr4:124731693-124731802 |
| 13084 | Mir676 | NR_030525.1 | chrX:100381096-100381185 | 13181 | Mir6970 | NR_105936.1 | chr17:34845100-34845167 |
| 13085 | Mir6769b | NR_106035.1 | chr8:71631046-71631106 | 13182 | Mir6971 | NR_105937.1 | chr17:34841808-34841871 |
| 13086 | Mir677 | NR_030442.2 | chr10:128085285-128085363 | 13183 | Mir6972 | NR_105938.1 | chr17:34857568-34857632 |
| 13087 | Mir678 | NR_030443.1 | chr10:76207325-76207409 | 13184 | Mir6973a | NR_105939.1 | chr17:35194971-35195049 |
| 13088 | Mir679 | NR_030445.1 | chr12:109715576-109715650 | 13185 | Mir6973b | NR_105969.1 | chr2:131040318-131040398 |
| 13089 | Mir680-2 | NR_030448.1 | chr9:22057712-22379223 | 13186 | Mir6974 | NR_105940.1 | chr17:35204474-35204557 |
| 13090 | Mir680-3 | NR_030449.1 | chr10:25867369-25894505 | 13187 | Mir6975 | NR_105941.1 | chr17:35244074-35244135 |
| 13091 | Mir681 | NR_030450.1 | chr12:69763834-69763944 | 13188 | Mir6976 | NR_105942.1 | chr17:46553826-46553889 |
| 13092 | Mir682 | NR_030451.1 | chr13:75645045-75645141 | 13189 | Mir6977 | NR_105943.1 | chr17:56418845-56418909 |
| 13093 | Mir683-1 | NR_030453.1 | chr13:50544626-50544734 | 13190 | Mir6978 | NR_105944.1 | chr17:57217171-57217228 |
| 13094 | Mir683-2 | NR_030647.1 | chr13:50600972-50601080 | 13191 | Mir6979 | NR_105945.1 | chr18:37854604-37854660 |

Fig.21 - 69

| | | | |
|---|---|---|---|
| 13192 | Mir698 | NR_030480.1 | chr4:124743780-124743889 |
| 13193 | Mir6980 | NR_105946.1 | chr18:37990886-37990945 |
| 13194 | Mir6981 | NR_105947.1 | chr18:37974502-37974617 |
| 13195 | Mir6982 | NR_105948.1 | chr18:60958072-60958140 |
| 13196 | Mir6983 | NR_105949.1 | chr18:61117467-61117531 |
| 13197 | Mir6984 | NR_105950.1 | chr19:3288919-3288983 |
| 13198 | Mir6985 | NR_105951.1 | chr19:4263817-4263878 |
| 13199 | Mir6986 | NR_105952.1 | chr19:4623897-4623955 |
| 13200 | Mir6987 | NR_105953.1 | chr19:5679003-5679076 |
| 13201 | Mir6988 | NR_105954.1 | chr19:6051333-6051392 |
| 13202 | Mir6989 | NR_105955.1 | chr11:46232134-46581905 |
| 13203 | Mir6990 | NR_105956.1 | chr19:6914195-6914286 |
| 13204 | Mir6991 | NR_105957.1 | chr19:7422572-7422642 |
| 13205 | Mir6992 | NR_105958.1 | chr19:8742970-8743079 |
| 13206 | Mir6993 | NR_105959.1 | chr19:10191374-10191439 |
| 13207 | Mir6994 | NR_105960.1 | chr19:11923727-11923800 |
| 13208 | Mir6995 | NR_105961.1 | chr19:47273518-47273589 |
| 13209 | Mir6996 | NR_105962.1 | chr2:26470058-26470119 |
| 13210 | Mir6997 | NR_105963.1 | chr2:29995949-29996020 |
| 13211 | Mir6998 | NR_105964.1 | chr2:31612420-31612484 |
| 13212 | Mir6999 | NR_105965.1 | chr2:91944869-91944930 |
| 13213 | Mir700 | NR_030481.1 | chr4:135416554-135416633 |
| 13214 | Mir7000 | NR_105966.1 | chr2:92387383-92387446 |
| 13215 | Mir7001 | NR_105967.1 | chr2:93421928-93422007 |
| 13216 | Mir7002 | NR_105968.1 | chr2:114150515-114150570 |
| 13217 | Mir7003 | NR_105970.1 | chr2:163068089-163068155 |
| 13218 | Mir7004 | NR_105971.1 | chr2:168640695-168640759 |
| 13219 | Mir7005 | NR_105972.1 | chr2:180179754-180179823 |
| 13220 | Mir7006 | NR_105973.1 | chr2:181587899-181587968 |
| 13221 | Mir7007 | NR_105974.1 | chr3:20222289-20222359 |
| 13222 | Mir7008 | NR_105975.1 | chr3:31038965-31039028 |
| 13223 | Mir7009 | NR_105976.1 | chr3:36475460-36475524 |
| 13224 | Mir701 | NR_030482.1 | chr5:111004143-111004252 |
| 13225 | Mir7010 | NR_105977.1 | chr3:82106246-82106309 |
| 13226 | Mir7011 | NR_105978.1 | chr3:88440878-88440951 |
| 13227 | Mir7012 | NR_105979.1 | chr3:90270148-90270212 |
| 13228 | Mir7013 | NR_105980.1 | chr3:95004167-95004239 |
| 13229 | Mir7014 | NR_105981.1 | chr3:95734910-95734973 |
| 13230 | Mir7015 | NR_105982.1 | chr4:120973258-120973322 |
| 13231 | Mir7016 | NR_105983.1 | chr4:129684499-129684571 |
| 13232 | Mir7017 | NR_105984.1 | chr4:133195654-133195716 |
| 13233 | Mir7018 | NR_105985.1 | chr4:137538285-137538370 |
| 13234 | Mir7019 | NR_105986.1 | chr4:138316131-138316200 |
| 13235 | Mir702 | NR_030483.1 | chr5:136991432-136991541 |
| 13236 | Mir7020 | NR_105987.1 | chr4:139644041-139644111 |
| 13237 | Mir7021 | NR_105988.1 | chr4:143136099-143136162 |
| 13238 | Mir7022 | NR_105989.1 | chr4:148146935-148146996 |
| 13239 | Mir7023 | NR_105990.1 | chr4:149655503-149655567 |
| 13240 | Mir7024 | NR_105991.1 | chr5:33898909-33898971 |
| 13241 | Mir7025 | NR_105992.1 | chr5:75176830-75176904 |
| 13242 | Mir7026 | NR_105993.1 | chr5:110666074-110666141 |
| 13243 | Mir7027 | NR_105994.1 | chr5:114404467-114404539 |
| 13244 | Mir7028 | NR_105995.1 | chr5:114719514-114719578 |
| 13245 | Mir7029 | NR_105996.1 | chr5:115580948-115581009 |
| 13246 | Mir703 | NR_030484.1 | chr5:98475555-98475664 |
| 13247 | Mir7030 | NR_105997.1 | chr5:115645572-115645632 |
| 13248 | Mir7031 | NR_105998.1 | chr5:121819208-121819273 |
| 13249 | Mir7032 | NR_105999.1 | chr5:123996851-123996930 |
| 13250 | Mir7033 | NR_106000.1 | chr5:135383729-135383810 |
| 13251 | Mir7034 | NR_106001.1 | chr5:135735805-135735889 |
| 13252 | Mir7035 | NR_106002.1 | chr5:136105521-136105603 |
| 13253 | Mir7036 | NR_106003.1 | chr5:137296623-137296688 |
| 13254 | Mir7036b | NR_106111.1 | chr5:34574150-34574213 |
| 13255 | Mir7037 | NR_106004.1 | chr5:139767878-139767943 |
| 13256 | Mir7038 | NR_106005.1 | chr5:140427338-140427409 |
| 13257 | Mir7039 | NR_106006.1 | chr5:144896731-144896816 |
| 13258 | Mir704 | NR_030485.1 | chr6:47803575-47803652 |
| 13259 | Mir7040 | NR_106007.1 | chr6:83049710-83049773 |
| 13260 | Mir7041 | NR_106008.1 | chr6:94606363-94606415 |
| 13261 | Mir7042 | NR_106009.1 | chr6:113707207-113707265 |
| 13262 | Mir7043 | NR_106010.1 | chr6:116645977-116646047 |
| 13263 | Mir7044 | NR_106011.1 | chr6:118085191-118085263 |
| 13264 | Mir7045 | NR_106012.1 | chr6:125097023-125097086 |
| 13265 | Mir7046 | NR_106013.1 | chr7:24970009-24970075 |
| 13266 | Mir7047 | NR_106014.1 | chr7:24987603-24987667 |
| 13267 | Mir7048 | NR_106015.1 | chr7:25217667-25217725 |
| 13268 | Mir7049 | NR_106016.1 | chr7:28560924-28560984 |
| 13269 | Mir705 | NR_030486.1 | chr6:85336291-85336373 |
| 13270 | Mir7050 | NR_106017.1 | chr7:31040257-31040317 |
| 13271 | Mir7051 | NR_106018.1 | chr7:43457496-43457569 |
| 13272 | Mir7052 | NR_106019.1 | chr7:44470346-44470411 |
| 13273 | Mir7053 | NR_106020.1 | chr7:44538105-44538178 |
| 13274 | Mir7054 | NR_106021.1 | chr7:45012425-45012487 |
| 13275 | Mir7055 | NR_106022.1 | chr7:45173302-45173361 |
| 13276 | Mir7056 | NR_106023.1 | chr7:47083165-47083224 |
| 13277 | Mir7057 | NR_106024.1 | chr7:66381666-66381724 |
| 13278 | Mir7058 | NR_106025.1 | chr7:126367970-126368045 |
| 13279 | Mir7059 | NR_106026.1 | chr7:126579473-126579532 |
| 13280 | Mir706 | NR_030487.1 | chr6:120034227-120034311 |
| 13281 | Mir7060 | NR_106027.1 | chr7:127489250-127489387 |
| 13282 | Mir7061 | NR_106028.1 | chr7:130908267-130908335 |
| 13283 | Mir7062 | NR_106029.1 | chr7:139986808-139986874 |
| 13284 | Mir7063 | NR_106030.1 | chr7:141620700-141620791 |
| 13285 | Mir7064 | NR_106031.1 | chr7:143570670-143570759 |
| 13286 | Mir7065 | NR_106032.1 | chr8:13136071-13136132 |
| 13287 | Mir7066 | NR_106033.1 | chr8:70102523-70102586 |
| 13288 | Mir7067 | NR_106034.1 | chr8:70895507-70895570 |
| 13289 | Mir7068 | NR_106036.1 | chr8:72470015-72470089 |
| 13290 | Mir7069 | NR_106037.1 | chr8:84867946-84868008 |
| 13291 | Mir707 | NR_030488.1 | chr7:44849698-44849771 |
| 13292 | Mir7070 | NR_106038.1 | chr8:85062085-85062171 |
| 13293 | Mir7071 | NR_106039.1 | chr8:88138205-88138274 |
| 13294 | Mir7072 | NR_106040.1 | chr8:94505045-94505105 |
| 13295 | Mir7073 | NR_106041.1 | chr8:95753150-95753214 |
| 13296 | Mir7074 | NR_106042.1 | chr8:105951636-105951699 |
| 13297 | Mir7075 | NR_106043.1 | chr8:107096024-107096106 |
| 13298 | Mir7076 | NR_106044.1 | chr8:111689600-111689674 |
| 13299 | Mir7077 | NR_106045.1 | chr8:117453022-117453079 |
| 13300 | Mir7078 | NR_106046.1 | chr8:117459264-117459328 |
| 13301 | Mir7079 | NR_106047.1 | chr8:123104974-123105043 |
| 13302 | Mir708 | NR_030489.1 | chr7:96249423-96249532 |
| 13303 | Mir7080 | NR_106048.1 | chr8:123130105-123130172 |
| 13304 | Mir7081 | NR_106049.1 | chr9:20914089-20914164 |
| 13305 | Mir7082 | NR_106050.1 | chr9:21075496-21075588 |
| 13306 | Mir7083 | NR_106051.1 | chr9:21809940-21810000 |
| 13307 | Mir7084 | NR_106052.1 | chr9:22113943-22114013 |
| 13308 | Mir7085 | NR_106053.1 | chr9:44400422-44400488 |
| 13309 | Mir7086 | NR_106054.1 | chr9:45266642-45266707 |
| 13310 | Mir7087 | NR_106055.1 | chr9:45939520-45939590 |
| 13311 | Mir7088 | NR_106056.1 | chr9:108081497-108081583 |
| 13312 | Mir7089 | NR_106057.1 | chr9:109889642-109889710 |
| 13313 | Mir709 | NR_030490.1 | chr8:84086098-84086186 |
| 13314 | Mir7090 | NR_106058.1 | chr9:120955085-120955147 |
| 13315 | Mir7091 | NR_106059.1 | chrX:74274026-74274105 |
| 13316 | Mir7092 | NR_106060.1 | chrX:74317361-74317429 |
| 13317 | Mir7093 | NR_106061.1 | chrX:134757630-134757731 |
| 13318 | Mir7094-1 | NR_106062.1 | chr12:114469406-114469466 |
| 13319 | Mir7094-2 | NR_106063.1 | chr12:114513634-114513694 |
| 13320 | Mir7-1 | NR_029825.1 | chr13:58392778-58392886 |
| 13321 | Mir710 | NR_030491.1 | chr8:64514331-64514441 |
| 13322 | Mir711 | NR_030492.1 | chr9:108969921-108970003 |
| 13323 | Mir7115 | NR_106064.1 | chr11:70438254-70438318 |
| 13324 | Mir7117 | NR_106065.1 | chr15:27571493-27571558 |
| 13325 | Mir7118 | NR_106066.1 | chr15:89162847-89162908 |
| 13326 | Mir7119 | NR_106067.1 | chr4:126559859-126559918 |
| 13327 | Mir713 | NR_030493.1 | chr13:62760738-62760846 |
| 13328 | Mir717 | NR_030497.1 | chrX:52422406-52422515 |
| 13329 | Mir718 | NR_030758.1 | chrX:74023848-74023936 |
| 13330 | Mir719 | NR_030458.1 | chr14:60228805-60228915 |
| 13331 | Mir7-2 | NR_029826.1 | chr7:78888276-78888373 |
| 13332 | Mir721 | NR_030500.1 | chr5:136375715-136375803 |
| 13333 | Mir7210 | NR_106069.1 | chr14:24088876-24088930 |
| 13334 | Mir7211 | NR_106070.1 | chr10:94586136-94586199 |
| 13335 | Mir7212 | NR_106071.1 | chr15:25948226-25948287 |
| 13336 | Mir7213 | NR_106072.1 | chr15:79998969-79999026 |
| 13337 | Mir7214 | NR_106073.1 | chr17:27317034-27317095 |
| 13338 | Mir7215 | NR_106074.1 | chr10:62682507-62682558 |
| 13339 | Mir7216 | NR_106075.1 | chr17:27328384-27328460 |
| 13340 | Mir7217 | NR_106076.1 | chr17:27355737-27355799 |
| 13341 | Mir7218 | NR_106077.1 | chr17:27358096-27358155 |
| 13342 | Mir7219 | NR_106078.1 | chr18:68260919-68260973 |
| 13343 | Mir7220 | NR_106079.1 | chr18:60953872-60953932 |
| 13344 | Mir7221 | NR_106080.1 | chr2:92592205-92592277 |
| 13345 | Mir7222 | NR_106081.1 | chr2:92594601-92594676 |
| 13346 | Mir7223 | NR_106082.1 | chr11:107996847-107996916 |
| 13347 | Mir7224 | NR_106083.1 | chr2:67675456-67675516 |
| 13348 | Mir7225 | NR_106084.1 | chr3:97690508-97690562 |
| 13349 | Mir7226 | NR_106085.1 | chr4:118210504-118210564 |
| 13350 | Mir7227 | NR_106086.1 | chr4:133717049-133717108 |
| 13351 | Mir7228 | NR_106087.1 | chr5:134572075-134572114 |
| 13352 | Mir7229 | NR_106088.1 | chr5:113324492-113324545 |
| 13353 | Mir7230 | NR_106089.1 | chr5:113337589-113337645 |
| 13354 | Mir7231 | NR_106090.1 | chr6:122831376-122831437 |
| 13355 | Mir7232 | NR_106091.1 | chr6:81895589-81895663 |
| 13356 | Mir7233 | NR_106092.1 | chr6:127788083-127788143 |
| 13357 | Mir7234 | NR_106093.1 | chr7:73819589-73819641 |
| 13358 | Mir7235 | NR_106094.1 | chr11:97146363-97146419 |
| 13359 | Mir7236 | NR_106095.1 | chr11:121387144-121387204 |
| 13360 | Mir7237 | NR_106096.1 | chr8:121978023-121978086 |
| 13361 | Mir7238 | NR_106097.1 | chr8:11635693-11635736 |
| 13362 | Mir7239 | NR_106098.1 | chr11:46170086-46170143 |
| 13363 | Mir7240 | NR_106099.1 | chr8:70798134-70798192 |
| 13364 | Mir7241 | NR_106100.1 | chr9:67727716-67727780 |
| 13365 | Mir7242 | NR_106101.1 | chr9:67729498-67729577 |
| 13366 | Mir7243 | NR_106163.1 | chr9:102304923-102304975 |
| 13367 | Mir741 | NR_030530.1 | chrX:66796804-66796875 |
| 13368 | Mir742 | NR_030531.1 | chrX:66780372-66780437 |
| 13369 | Mir743 | NR_030532.1 | chrX:66776756-66776818 |
| 13370 | Mir743b | NR_030535.1 | chrX:66777255-66777332 |
| 13371 | Mir744 | NR_030417.1 | chr11:65734732-65734832 |
| 13372 | Mir7578 | NR_106102.1 | chr2:27450548-27450630 |
| 13373 | Mir758 | NR_030421.1 | chr12:109712809-109712890 |
| 13374 | Mir759 | NR_030436.1 | chr14:79738430-79738528 |
| 13375 | Mir760 | NR_030439.1 | chr3:122293584-122293703 |
| 13376 | Mir761 | NR_030432.1 | chr4:109017654-109017730 |
| 13377 | Mir762 | NR_030428.1 | chr7:127708486-127708562 |
| 13378 | Mir764 | NR_030433.1 | chrX:147002258-147002366 |
| 13379 | Mir7646 | NR_106104.1 | chrX:135746715-135746769 |
| 13380 | Mir7647 | NR_106105.1 | chr5:123522636-123522695 |
| 13381 | Mir7648 | NR_106106.1 | chr15:90224359-90224412 |
| 13382 | Mir7649 | NR_106107.1 | chr6:128931792-128974895 |
| 13383 | Mir7650 | NR_106108.1 | chr9:15312610-15312669 |
| 13384 | Mir7652 | NR_106112.1 | chr1:150024260-150052202 |
| 13385 | Mir7653 | NR_106113.1 | chr11:78178826-78178887 |

Fig.21 - 70

| | | | | | | | |
|---|---|---|---|---|---|---|---|
| 13386 | Mir7654 | NR_106114.1 | chr8:8690092-8690162 | 13483 | Mirlet7d | NR_029656.1 | chr13:48536011-48536114 |
| 13387 | Mir7655 | NR_106115.1 | chr2:18057845-18057903 | 13484 | Mirlet7e | NR_029730.1 | chr17:17830351-17830444 |
| 13388 | Mir7656 | NR_106116.1 | chr9:94751828-94751890 | 13485 | Mirlet7f-1 | NR_029731.1 | chr13:48537828-48537917 |
| 13389 | Mir7657 | NR_106117.1 | chr3:122541624-122541681 | 13486 | Mirlet7f-2 | NR_029732.1 | chrX:151912345-151912428 |
| 13390 | Mir7658 | NR_106118.1 | chr4:156230058-156230115 | 13487 | Mirlet7g | NR_029526.1 | chr9:106178839-106178927 |
| 13391 | Mir7661 | NR_106121.1 | chr6:108489742-108489803 | 13488 | Mirlet7i | NR_029527.1 | chr10:122985639-122985724 |
| 13392 | Mir7662 | NR_106122.1 | chr10:62194166-62194227 | 13489 | Mirlet7j | NR_105798.1 | chr3:140028248-140028370 |
| 13393 | Mir7663 | NR_106123.1 | chr10:22729371-22729432 | 13490 | Mirlet7k | NR_105854.1 | chr5:147283188-147283298 |
| 13394 | Mir7665 | NR_106125.1 | chr2:120017516-120017579 | 13491 | Mis12 | NM_025993.2 | chr11:71019610-71027134 |
| 13395 | Mir7666 | NR_106126.1 | chr8:40307874-40307929 | 13492 | Mis18a | NM_025642.1 | chr16:90719311-90727371 |
| 13396 | Mir7667 | NR_106127.1 | chr9:21168377-21226281 | 13493 | Mis18bp1 | NM_172578.2 | chr12:65132734-65172580 |
| 13397 | Mir7668 | NR_106128.1 | chr7:29906508-29906567 | 13494 | Misp | NM_030218.2 | chr10:79821020-79830452 |
| 13398 | Mir7669 | NR_106129.1 | chr3:90011152-90011210 | 13495 | Mitd1 | NM_026913.2 | chr1:37878889-37890411 |
| 13399 | Mir767 | NR_035528.1 | chrX:72589987-72590094 | 13496 | Mitf | NM_001113198.1 | chr6:97807057-98021358 |
| 13400 | Mir7670 | NR_106130.1 | chr6:38507324-38507393 | 13497 | Mixl1 | NM_013729.3 | chr1:180693047-180697034 |
| 13401 | Mir7671 | NR_106131.1 | chr11:53763596-53763654 | 13498 | Mki67 | NM_001081117.2 | chr7:135689787-135716379 |
| 13402 | Mir7672 | NR_106132.1 | chr14:26669546-26669608 | 13499 | Mkks | NM_001141946.1 | chr2:136873780-136891406 |
| 13403 | Mir7673 | NR_106133.1 | chrX:94274573-94274637 | 13500 | Mkl1 | NM_001082536.1 | chr15:81012280-81190757 |
| 13404 | Mir7674 | NR_106135.1 | chr2:32050945-32051008 | 13501 | Mkl2 | NM_001122667.2 | chr16:13256480-13417529 |
| 13405 | Mir7675 | NR_106136.1 | chr12:11299886-11299944 | 13502 | Mkln1 | NM_013791.2 | chr6:31398827-31509477 |
| 13406 | Mir7676-2 | NR_106138.1 | chr15:78284787-78348660 | 13503 | Mkln1os | NR_040300.1 | chr6:31366884-31398773 |
| 13407 | Mir7677 | NR_106142.1 | chr17:27091244-27091311 | 13504 | Mknk1 | NM_001285487.1 | chr4:115839197-115879256 |
| 13408 | Mir7678 | NR_106143.1 | chr2:164354312-164354383 | 13505 | Mknk2 | NM_021462.4 | chr10:80665317-80676293 |
| 13409 | Mir7679 | NR_106144.1 | chr11:82985002-82985065 | 13506 | Mkrn1 | NM_018810.2 | chr6:39397820-39420369 |
| 13410 | Mir7680 | NR_106145.1 | chr16:21256012-21256066 | 13507 | Mkrn2 | NM_023290.2 | chr6:115601937-115618670 |
| 13411 | Mir7681 | NR_106149.1 | chr1:53849423-53849495 | 13508 | Mkrn3 | NM_011746.2 | chr7:62417592-62420139 |
| 13412 | Mir7682 | NR_106150.1 | chr1:151442392-151442450 | 13509 | Mks1 | NM_001039684.2 | chr11:87853224-87863679 |
| 13413 | Mir7684 | NR_106153.1 | chr15:82393918-82393978 | 13510 | Mkx | NM_177595.4 | chr18:6934965-7004779 |
| 13414 | Mir7685 | NR_106156.1 | chr2:158243503-158243565 | 13511 | Mlana | NM_029993.1 | chr19:29697940-29708306 |
| 13415 | Mir7686 | NR_106157.1 | chr7:139957565-139957622 | 13512 | Mlc1 | NM_133241.2 | chr15:88955883-88978553 |
| 13416 | Mir7687 | NR_106159.1 | chr8:120538695-120538754 | 13513 | Mlec | NM_175403.3 | chr5:115142980-115158176 |
| 13417 | Mir770 | NR_030427.1 | chr12:109563691-109563785 | 13514 | Mlf1 | NM_001039543.2 | chr3:67374096-67400000 |
| 13418 | Mir7b | NR_029827.1 | chr17:56242987-56243098 | 13515 | Mlf2 | NM_001170341.1 | chr6:124931387-124936149 |
| 13419 | Mir802 | NR_030429.1 | chr16:93369719-93369816 | 13516 | Mlh1 | NM_026810.2 | chr9:111228227-111271608 |
| 13420 | Mir804 | NR_030529.1 | chr11:50357784-50357879 | 13517 | Mlh3 | NM_175337.2 | chr12:85234465-85270599 |
| 13421 | Mir8091 | NR_106166.1 | chr19:41588650-41588789 | 13518 | Mlip | NM_027150.1 | chr9:77102083-77347870 |
| 13422 | Mir8092 | NR_106167.1 | chr19:40894702-40894789 | 13519 | Mlkl | NM_029005.3 | chr8:111311797-111338205 |
| 13423 | Mir8093 | NR_106168.1 | chr2:32687629-32687766 | 13520 | Mllt1 | NM_022328.2 | chr17:56892610-56935388 |
| 13424 | Mir8094 | NR_106169.1 | chr17:35251288-35251405 | 13521 | Mllt10 | NM_001252560.1 | chr2:18064584-18212390 |
| 13425 | Mir8095 | NR_106170.1 | chr16:22532055-22532184 | 13522 | Mllt11 | NM_019914.4 | chr3:95218543-95228677 |
| 13426 | Mir8096 | NR_106172.1 | chr1:85751947-85752074 | 13523 | Mllt3 | NM_001286158.1 | chr4:87769924-87806323 |
| 13427 | Mir8097 | NR_106173.1 | chr15:36240045-36240164 | 13524 | Mllt4 | NM_010806.1 | chr17:13760540-13905794 |
| 13428 | Mir8098 | NR_106174.1 | chr14:62837720-62837857 | 13525 | Mllt6 | NM_139311.2 | chr11:97663411-97685458 |
| 13429 | Mir8099-1 | NR_106175.1 | chr3:93927337-95139186 | 13526 | Mlph | NM_053015.3 | chr1:90915099-90951142 |
| 13430 | Mir8100 | NR_106177.1 | chr11:46102269-46102390 | 13527 | Mlst8 | NM_001252463.1 | chr17:24473549-24479078 |
| 13431 | Mir8101 | NR_106179.1 | chr11:102230039-102230150 | 13528 | Mlx | NM_001159384.1 | chr11:101087289-101092207 |
| 13432 | Mir8102 | NR_106180.1 | chr11:97744896-97745035 | 13529 | Mlxip | NM_133917.3 | chr5:123394797-123457931 |
| 13433 | Mir8103 | NR_106181.1 | chr11:97063767-97063874 | 13530 | Mlxipl | NM_021455.4 | chr5:135106890-135138382 |
| 13434 | Mir8104 | NR_106182.1 | chr10:122679314-122679415 | 13531 | Mlycd | NM_019966.2 | chr8:119394891-119411088 |
| 13435 | Mir8105 | NR_106183.1 | chr10:128458679-128458768 | 13532 | Mmaa | NM_133823.4 | chr8:79266423-79294956 |
| 13436 | Mir8106 | NR_106185.1 | chr9:122273889-122274028 | 13533 | Mmab | NM_029956.3 | chr5:114431033-114444027 |
| 13437 | Mir8107 | NR_106186.1 | chr9:110635998-110636115 | 13534 | Mmachc | NM_025962.3 | chr4:116702433-116708385 |
| 13438 | Mir8108 | NR_106187.1 | chr8:25159124-25159233 | 13535 | Mmadhc | NM_133839.2 | chr2:50279880-50296677 |
| 13439 | Mir8109 | NR_106189.1 | chr8:85700904-85701021 | 13536 | Mmd | NM_026178.2 | chr11:90249475-90278573 |
| 13440 | Mir8110 | NR_106190.1 | chr8:89024734-89024831 | 13537 | Mmd2 | NM_175217.6 | chr5:142563480-142608752 |
| 13441 | Mir8111 | NR_106191.1 | chr8:84005627-84005764 | 13538 | Mme | NM_001289462.1 | chr3:63295434-63383713 |
| 13442 | Mir8112 | NR_106192.1 | chr6:71271670-71271801 | 13539 | Mmel1 | NM_013783.2 | chr4:154869584-154895530 |
| 13443 | Mir8113 | NR_106193.1 | chr6:125234683-125234812 | 13540 | Mmgt1 | NM_146234.3 | chrX:56585511-56597919 |
| 13444 | Mir8114 | NR_106194.1 | chr1:153899925-153900036 | 13541 | Mmgt2 | NM_175002.2 | chr11:62648663-62666359 |
| 13445 | Mir8115 | NR_106195.1 | chr11:86656070-87483748 | 13542 | Mmp10 | NM_019471.3 | chr9:7502341-7510242 |
| 13446 | Mir8116 | NR_106196.1 | chr5:137293779-137293876 | 13543 | Mmp11 | NM_008606.3 | chr10:75923221-75932502 |
| 13447 | Mir8118 | NR_106198.1 | chr4:33438080-33438209 | 13544 | Mmp12 | NM_008605.3 | chr9:7347373-7360461 |
| 13448 | Mir8119 | NR_106199.1 | chr4:129557682-129557811 | 13545 | Mmp13 | NM_008607.2 | chr9:7272513-7283333 |
| 13449 | Mir8120 | NR_106200.1 | chr3:65659287-65659426 | 13546 | Mmp14 | NM_008608.3 | chr14:54431603-54441258 |
| 13450 | Mir871 | NR_030536.1 | chrX:66810427-66810504 | 13547 | Mmp15 | NM_008609.3 | chr8:95352336-95374293 |
| 13451 | Mir872 | NR_030604.1 | chr4:94665156-94665237 | 13548 | Mmp16 | NM_019724.3 | chr4:17853481-18118734 |
| 13452 | Mir873b | NR_105849.1 | chr4:36668509-36668586 | 13549 | Mmp17 | NM_011846.4 | chr5:129584213-129608211 |
| 13453 | Mir874 | NR_030544.1 | chr13:58023124-58023200 | 13550 | Mmp19 | NM_001164197.1 | chr10:128790909-128800824 |
| 13454 | Mir875 | NR_030606.1 | chr15:35660969-35661047 | 13551 | Mmp1a | NM_032006.3 | chr9:7464140-7476869 |
| 13455 | Mir876 | NR_030545.1 | chr4:36645372-36645453 | 13552 | Mmp1b | NM_032007.3 | chr9:7367669-7388026 |
| 13456 | Mir877 | NR_030608.1 | chr17:35960729-35960814 | 13553 | Mmp2 | NM_008610.2 | chr8:92827327-92853420 |
| 13457 | Mir878 | NR_030603.1 | chrX:66801507-66801585 | 13554 | Mmp20 | NM_013903.2 | chr9:7628230-7674968 |
| 13458 | Mir879 | NR_030537.1 | chr5:9375703-9375779 | 13555 | Mmp21 | NM_152944.1 | chr7:133674269-133680061 |
| 13459 | Mir880 | NR_030538.1 | chrX:66800529-66800607 | 13556 | Mmp23 | NM_011985.2 | chr4:155650654-155653384 |
| 13460 | Mir881 | NR_030539.1 | chrX:66801943-66802021 | 13557 | Mmp24 | NM_010808.3 | chr2:155775343-155818366 |
| 13461 | Mir882 | NR_030540.1 | chr12:109682196-109682273 | 13558 | Mmp25 | NM_001033339.3 | chr17:23629457-23645269 |
| 13462 | Mir883a | NR_030541.1 | chrX:66780757-66780833 | 13559 | Mmp27 | NM_001030289.1 | chr9:7571457-7581393 |
| 13463 | Mir883b | NR_030542.1 | chrX:66789889-66789967 | 13560 | Mmp28 | NM_080453.2 | chr11:83441875-83462961 |
| 13464 | Mir9-1 | NR_029817.1 | chr3:88215597-88215686 | 13561 | Mmp3 | NM_010809.2 | chr9:7445821-7455974 |
| 13465 | Mir9-2 | NR_029545.1 | chr13:83738813-83738885 | 13562 | Mmp7 | NM_010810.4 | chr9:7692110-7699416 |
| 13466 | Mir92-1 | NR_029816.1 | chr14:115044426-115044506 | 13563 | Mmp8 | NM_008611.4 | chr9:7558428-7568486 |
| 13467 | Mir92-2 | NR_029748.1 | chrX:52741837-52741928 | 13564 | Mmp9 | NM_013599.3 | chr2:164948218-164955849 |
| 13468 | Mir92b | NR_030579.1 | chr3:89227115-89227198 | 13565 | Mmrn1 | NM_001163507.1 | chr6:60944316-60989378 |
| 13469 | Mir93 | NR_029749.1 | chr5:138165522-138165610 | 13566 | Mmrn2 | NM_153127.3 | chr14:34375503-34404286 |
| 13470 | Mir9-3 | NR_029818.1 | chr7:79505263-79505353 | 13567 | Mms19 | NM_028152.3 | chr19:41943706-41981136 |
| 13471 | Mir96 | NR_029750.1 | chr6:30169445-30169551 | 13568 | Mms22l | NM_199467.2 | chr4:24496461-24602948 |
| 13472 | Mir98 | NR_029753.1 | chrX:151913213-151913321 | 13569 | Mn1 | NM_001081235.1 | chr5:111418165-111457025 |
| 13473 | Mir99a | NR_029535.1 | chr16:77598935-77599000 | 13570 | Mnat1 | NM_008612.2 | chr12:73123716-73273988 |
| 13474 | Mir99b | NR_029536.1 | chr17:17830187-17830257 | 13571 | Mnd1 | NM_029797.3 | chr3:84087933-84155786 |
| 13475 | Mira | NR_045199.1 | chr6:52214491-52215288 | 13572 | Mnd1-ps | NR_030680.1 | chr14:9884094-9886868 |
| 13476 | Mirg | NR_028265.1 | chr12:109734980-109749457 | 13573 | Mnda | NM_001033450.4 | chr1:173896340-173913046 |
| 13477 | Mirlet7a-1 | NR_029725.1 | chr13:48538178-48538272 | 13574 | Mndal | NM_001170853.1 | chr1:173857219-173880187 |
| 13478 | Mirlet7a-2 | NR_029726.1 | chr9:41536715-41536811 | 13575 | Mns1 | NM_008613.3 | chr9:72438528-72458576 |
| 13479 | Mirlet7b | NR_029727.1 | chr15:85707318-85707403 | 13576 | Mnt | NM_010813.3 | chr11:74830923-74845725 |
| 13480 | Mirlet7bhg | NR_110483.1 | chr15:85703772-85707524 | 13577 | Mnx1 | NM_019944.2 | chr5:29473822-29478470 |
| 13481 | Mirlet7c-1 | NR_029728.1 | chr16:77599656-77599750 | 13578 | Moap1 | NM_001142937.2 | chr12:102739829-102743661 |
| 13482 | Mirlet7c-2 | NR_029729.1 | chr15:85706602-85706697 | 13579 | Mob1a | NM_145571.2 | chr6:83326038-83340949 |

Fig.21 - 71

| | | | |
|---|---|---|---|
| 13580 | Mob1b | NM_026735.2 | chr5:88720870-88758455 |
| 13581 | Mob2 | NM_028308.2 | chr7:142008552-142061034 |
| 13582 | Mob3a | NM_172457.2 | chr10:80685252-80701820 |
| 13583 | Mob3b | NM_178061.5 | chr4:34949073-35157484 |
| 13584 | Mob3c | NM_175308.4 | chr4:115828091-115836183 |
| 13585 | Mob4 | NM_025283.3 | chr1:55131244-55154899 |
| 13586 | Mobp | NM_001039364.2 | chr9:120149741-120176091 |
| 13587 | Mocos | NM_026779.1 | chr18:24653690-24701556 |
| 13588 | Mocs1 | NM_020042.2 | chr17:49428363-49455430 |
| 13589 | Mocs2 | NM_001113374.1 | chr13:114818236-114829420 |
| 13590 | Mocs3 | NM_001160330.1 | chr2:168230621-168232303 |
| 13591 | Mog | NM_010814.2 | chr17:37010739-37023398 |
| 13592 | Mogat1 | NM_026713.3 | chr1:78511059-78538173 |
| 13593 | Mogat2 | NM_177448.4 | chr7:99219083-99238611 |
| 13594 | Mogs | NM_020619.2 | chr6:83115505-83118898 |
| 13595 | Mok | NM_011973.2 | chr12:110807797-110840939 |
| 13596 | Mon1a | NM_028369.3 | chr9:107888128-107903139 |
| 13597 | Mon1b | NM_001048143.2 | chr8:113635585-113645192 |
| 13598 | Mon2 | NM_001163024.1 | chr10:122992060-123076505 |
| 13599 | Morc1 | NM_010816.1 | chr16:48431236-48630905 |
| 13600 | Morc2a | NM_001159288.1 | chr11:3649493-3690370 |
| 13601 | Morc2b | NM_177719.4 | chr17:33135589-33139683 |
| 13602 | Morc3 | NM_001045529.3 | chr16:93832120-93876073 |
| 13603 | Morc4 | NM_001193309.1 | chrX:139821634-139871654 |
| 13604 | Morf4l1 | NM_001039147.2 | chr9:90091668-90114820 |
| 13605 | Morf4l2 | NM_001168225.1 | chrX:136732947-136741155 |
| 13606 | Morn1 | NM_001081100.1 | chr4:155086576-155145507 |
| 13607 | Morn2 | NM_194269.2 | chr17:80290211-80297476 |
| 13608 | Morn3 | NM_029112.1 | chr5:123037126-123046820 |
| 13609 | Morn4 | NM_198108.2 | chr19:42074938-42086370 |
| 13610 | Morn5 | NM_029309.2 | chr2:36049472-36079709 |
| 13611 | Mos | NM_020021.2 | chr4:3870657-3872105 |
| 13612 | Mospd1 | NM_001290514.1 | chrX:53344593-53370559 |
| 13613 | Mospd2 | NM_001290523.1 | chrX:164936170-164980375 |
| 13614 | Mospd3 | NM_001254762.1 | chr5:137596646-137600708 |
| 13615 | Mospd4 | NR_045438.1 | chr18:46464933-46465816 |
| 13616 | Mov10 | NM_001163440.1 | chr3:104794833-104818563 |
| 13617 | Mov10l1 | NM_031260.2 | chr15:88982993-89055152 |
| 13618 | Moxd1 | NM_021509.5 | chr10:24223516-24302783 |
| 13619 | Moxd2 | NM_139296.2 | chr6:40878793-40887494 |
| 13620 | Mpc1 | NM_018819.4 | chr17:8283812-8297661 |
| 13621 | Mpc2 | NM_027430.2 | chr1:165461207-165481214 |
| 13622 | Mpdu1 | NM_011900.4 | chr11:69656698-69662649 |
| 13623 | Mpdz | NM_010820.3 | chr4:81278498-81442815 |
| 13624 | Mpeg1 | NM_010821.1 | chr19:12460778-12465285 |
| 13625 | Mpg | NM_010822.3 | chr11:32226504-32232702 |
| 13626 | Mphosph10 | NM_026483.2 | chr7:64376540-64392236 |
| 13627 | Mphosph6 | NM_026758.3 | chr8:117791644-117801929 |
| 13628 | Mphosph8 | NM_023773.2 | chr14:56668247-56697429 |
| 13629 | Mphosph9 | NM_001081323.2 | chr5:124250958-124327972 |
| 13630 | Mpi | NM_025837.2 | chr9:57544267-57552752 |
| 13631 | Mpl | NM_001122949.2 | chr4:118442414-118457513 |
| 13632 | Mplkip | NM_025479.5 | chr13:17695412-17699105 |
| 13633 | Mpnd | NM_026530.5 | chr17:56009200-56016783 |
| 13634 | Mpo | NM_010824.2 | chr11:87793783-87804412 |
| 13635 | Mpp1 | NM_008621.3 | chrX:75109732-75131016 |
| 13636 | Mpp2 | NM_016695.3 | chr11:102057016-102088515 |
| 13637 | Mpp3 | NM_007863.2 | chr11:101999652-102026955 |
| 13638 | Mpp4 | NM_001164682.1 | chr1:59120934-59163389 |
| 13639 | Mpp5 | NM_019579.3 | chr12:78748946-78840713 |
| 13640 | Mpp6 | NM_001164733.1 | chr6:50110240-50198598 |
| 13641 | Mpp7 | NM_001081287.2 | chr18:7347961-7626863 |
| 13642 | Mppe1 | NM_172630.2 | chr18:67225529-67245830 |
| 13643 | Mpped1 | NM_172610.3 | chr15:83780022-83858474 |
| 13644 | Mpped2 | NM_001143683.1 | chr2:106693458-106868360 |
| 13645 | Mprip | NM_012027.2 | chr11:59662494-59780860 |
| 13646 | Mpst | NM_001162492.1 | chr15:78406800-78414015 |
| 13647 | Mptx1 | NM_025470.3 | chr1:174330517-174332877 |
| 13648 | Mptx2 | NM_001205011.2 | chr1:173274460-173277756 |
| 13649 | Mpv17 | NM_008622.6 | chr5:31140662-31154251 |
| 13650 | Mpv17l | NM_001289561.1 | chr16:13940664-13949619 |
| 13651 | Mpv17l2 | NM_183170.2 | chr8:70758648-70760921 |
| 13652 | Mpz | NM_008623.5 | chr1:171150712-171161123 |
| 13653 | Mpzl1 | NM_001001880.2 | chr1:165592180-165634541 |
| 13654 | Mpzl2 | NM_007962.4 | chr9:45042343-45054043 |
| 13655 | Mpzl3 | NM_001093749.2 | chr9:45055180-45075042 |
| 13656 | Mr1 | NM_008209.4 | chr1:155127877-155146780 |
| 13657 | Mrap | NM_029844.3 | chr16:90738323-90749776 |
| 13658 | Mrap2 | NM_001101482.2 | chr9:87144305-87184045 |
| 13659 | Mras | NM_008624.3 | chr9:99385419-99436712 |
| 13660 | Mrc1 | NM_008625.2 | chr2:14229413-14332023 |
| 13661 | Mrc2 | NM_008626.3 | chr11:105292645-105351145 |
| 13662 | Mre11a | NM_018736.3 | chr9:14784653-14837126 |
| 13663 | Mreg | NM_001005423.2 | chr1:72159232-72212307 |
| 13664 | Mrfap1 | NM_026242.3 | chr5:36794866-36796754 |
| 13665 | Mrgbp | NM_028479.1 | chr2:180581303-180585634 |
| 13666 | Mrgpra1 | NM_153095.2 | chr7:47334874-47354240 |
| 13667 | Mrgpra2a | NM_001172588.1 | chr7:47426328-47452139 |
| 13668 | Mrgpra2b | NM_153101.3 | chr7:47463806-47489582 |
| 13669 | Mrgpra3 | NM_153067.2 | chr7:47588949-47601372 |
| 13670 | Mrgpra4 | NM_153524.2 | chr7:47980793-47982296 |
| 13671 | Mrgpra6 | NM_001308537.1 | chr7:47185766-47189355 |
| 13672 | Mrgpra9 | NM_001288801.1 | chr7:47234918-47252848 |
| 13673 | Mrgprb1 | NM_205810.4 | chr7:48444112-48456342 |
| 13674 | Mrgprb2 | NM_175531.4 | chr7:48550965-48558086 |
| 13675 | Mrgprb3 | NM_207537.1 | chr7:48642862-48643801 |
| 13676 | Mrgprb4 | NM_205795.1 | chr7:48198212-48199178 |
| 13677 | Mrgprb5 | NM_207538.1 | chr7:48168016-48168985 |
| 13678 | Mrgprb8 | NM_207539.2 | chr7:48388525-48389648 |
| 13679 | Mrgprd | NM_203490.3 | chr7:145314834-145324059 |
| 13680 | Mrgpre | NM_175534.3 | chr7:143778362-143784500 |
| 13681 | Mrgprf | NM_145379.2 | chr7:145300908-145309557 |
| 13682 | Mrgprg | NM_203492.2 | chr7:143763709-143766993 |
| 13683 | Mrgprh | NM_030726.1 | chr17:12876033-12877842 |
| 13684 | Mrgprx1 | NM_207540.2 | chr7:48020970-48027597 |
| 13685 | Mrgprx2 | NM_001034868.3 | chr7:48478618-48499270 |
| 13686 | Mri1 | NM_026423.4 | chr8:84250575-84257324 |
| 13687 | Mrm1 | NM_145433.1 | chr11:84813060-84819515 |
| 13688 | Mro | NM_027741.3 | chr18:73859384-73879134 |
| 13689 | Mroh1 | NM_001162489.1 | chr15:76390030-76439744 |
| 13690 | Mroh2a | NM_001281466.1 | chr1:88227019-88262289 |
| 13691 | Mroh2b | NM_001166066.1 | chr15:4898736-4962201 |
| 13692 | Mroh4 | NM_001177437.1 | chr15:74606027-74636318 |
| 13693 | Mroh5 | NM_001033365.2 | chr15:73786935-73839671 |
| 13694 | Mroh6 | NM_001282443.1 | chr15:75882933-75888723 |
| 13695 | Mroh7 | NM_001126487.1 | chr4:106680416-106727930 |
| 13696 | Mroh8 | NM_001039557.4 | chr2:157208547-157279549 |
| 13697 | Mroh9 | NM_030071.1 | chr1:163024301-163085670 |
| 13698 | Mrpl1 | NM_001039084.1 | chr5:96210114-96239684 |
| 13699 | Mrpl10 | NM_026154.1 | chr11:97041585-97049213 |
| 13700 | Mrpl11 | NM_025553.4 | chr19:4962305-4966995 |
| 13701 | Mrpl12 | NM_027204.2 | chr11:120484668-120488754 |
| 13702 | Mrpl13 | NM_026759.3 | chr15:55534094-55557312 |
| 13703 | Mrpl14 | NM_026732.2 | chr17:45686371-45698495 |
| 13704 | Mrpl15 | NM_001177658.1 | chr1:4773199-4785726 |
| 13705 | Mrpl16 | NM_025606.3 | chr19:11770414-11774946 |
| 13706 | Mrpl17 | NM_025301.2 | chr7:105803781-105811087 |
| 13707 | Mrpl18 | NM_026310.3 | chr17:12911354-12916091 |
| 13708 | Mrpl19 | NM_026490.2 | chr6:81957841-81965949 |
| 13709 | Mrpl2 | NM_025302.4 | chr17:46646247-46650132 |
| 13710 | Mrpl20 | NM_025570.2 | chr4:155803617-155808829 |
| 13711 | Mrpl21 | NM_172252.3 | chr19:3283046-3292837 |
| 13712 | Mrpl22 | NM_175001.3 | chr11:58171653-58179581 |
| 13713 | Mrpl23 | NM_011288.1 | chr7:142533116-142540742 |
| 13714 | Mrpl24 | NM_026591.3 | chr3:87919543-87923433 |
| 13715 | Mrpl27 | NM_053161.2 | chr11:94653790-94660087 |
| 13716 | Mrpl28 | NM_024227.3 | chr17:26123502-26126613 |
| 13717 | Mrpl3 | NM_053159.3 | chr9:105053267-105077476 |
| 13718 | Mrpl30 | NM_027098.2 | chr1:37890552-37898333 |
| 13719 | Mrpl32 | NM_029271.2 | chr13:14610300-14613037 |
| 13720 | Mrpl33 | NM_025796.3 | chr5:31613950-31622644 |
| 13721 | Mrpl34 | NM_053162.2 | chr8:71464925-71465753 |
| 13722 | Mrpl35 | NM_025430.3 | chr6:71812996-71823784 |
| 13723 | Mrpl36 | NM_053163.1 | chr13:73331008-73332178 |
| 13724 | Mrpl37 | NM_025500.2 | chr4:107055873-107066866 |
| 13725 | Mrpl38 | NM_024177.3 | chr11:116131816-116138868 |
| 13726 | Mrpl39 | NM_017404.4 | chr16:84717579-84735302 |
| 13727 | Mrpl4 | NM_023167.2 | chr9:21002736-21008837 |
| 13728 | Mrpl40 | NM_010922.2 | chr16:18872017-18876637 |
| 13729 | Mrpl41 | NM_001031808.2 | chr2:24972469-24975098 |
| 13730 | Mrpl42 | NM_026065.3 | chr10:95480805-95501927 |
| 13731 | Mrpl43 | NM_053164.3 | chr19:45005013-45006442 |
| 13732 | Mrpl44 | NM_001081210.1 | chr1:79776017-79781445 |
| 13733 | Mrpl45 | NM_025927.4 | chr11:97315715-97329920 |
| 13734 | Mrpl46 | NM_023331.2 | chr7:78775340-78783089 |
| 13735 | Mrpl47 | NM_029017.2 | chr3:32727496-32736755 |
| 13736 | Mrpl48 | NM_198831.2 | chr7:100549116-100583130 |
| 13737 | Mrpl49 | NM_026246.3 | chr19:6053629-6057751 |
| 13738 | Mrpl50 | NM_178603.4 | chr4:49512596-49521083 |
| 13739 | Mrpl51 | NM_025595.3 | chr6:125192199-125194392 |
| 13740 | Mrpl52 | NM_026851.2 | chr14:54426908-54429750 |
| 13741 | Mrpl53 | NM_026744.3 | chr6:83109107-83109932 |
| 13742 | Mrpl54 | NM_025317.2 | chr10:81264721-81266926 |
| 13743 | Mrpl55 | NM_026035.3 | chr11:59202485-59206135 |
| 13744 | Mrpl57 | NM_026401.2 | chr14:57826238-57828745 |
| 13745 | Mrpl9 | NM_030116.2 | chr3:94443335-94448708 |
| 13746 | Mrps10 | NM_001146211.1 | chr17:47368886-47378679 |
| 13747 | Mrps11 | NM_026498.2 | chr7:78783130-78792988 |
| 13748 | Mrps12 | NM_011885.4 | chr7:28739640-28741781 |
| 13749 | Mrps14 | NM_025474.3 | chr1:160195259-160201186 |
| 13750 | Mrps15 | NM_025544.2 | chr4:126046927-126055536 |
| 13751 | Mrps16 | NM_025440.2 | chr14:20391230-20393555 |
| 13752 | Mrps17 | NM_025450.4 | chr5:129715527-129718691 |
| 13753 | Mrps18a | NM_026768.3 | chr17:46111003-46128908 |
| 13754 | Mrps18b | NM_025878.1 | chr17:35910384-35916369 |
| 13755 | Mrps18c | NM_026826.1 | chr5:100798758-100804467 |
| 13756 | Mrps2 | NM_001166031.2 | chr2:28468065-28471177 |
| 13757 | Mrps21 | NM_078479.3 | chr3:95862651-95870619 |
| 13758 | Mrps22 | NM_025485.3 | chr9:98588729-98601679 |
| 13759 | Mrps23 | NM_001291270.1 | chr11:88194105-88211507 |
| 13760 | Mrps24 | NM_026080.2 | chr11:5703982-5707699 |
| 13761 | Mrps25 | NM_025578.4 | chr6:92169522-92184023 |
| 13762 | Mrps26 | NM_207207.1 | chr2:130563756-130565394 |
| 13763 | Mrps27 | NM_173757.3 | chr13:99344785-99415561 |
| 13764 | Mrps28 | NM_025434.3 | chr3:8802145-8923857 |
| 13765 | Mrps30 | NM_021556.3 | chr13:118380109-118387252 |
| 13766 | Mrps31 | NM_020560.2 | chr8:22411339-22429665 |
| 13767 | Mrps33 | NM_001010930.1 | chr6:39801806-39810936 |
| 13768 | Mrps34 | NM_023260.1 | chr17:24895119-24896273 |
| 13769 | Mrps35 | NM_145573.2 | chr6:147042769-147070902 |
| 13770 | Mrps36 | NM_001190264.1 | chr13:100735939-100744659 |
| 13771 | Mrps5 | NM_029963.2 | chr2:127587425-127603986 |
| 13772 | Mrps6 | NM_080456.1 | chr16:92058335-92112227 |
| 13773 | Mrps7 | NM_025305.2 | chr11:115604150-115607624 |

Fig.21 - 72

| | | | | | | | |
|---|---|---|---|---|---|---|---|
| 13774 | Mrps9 | NM_023514.4 | chr1:42851232-42905683 | 13871 | Mtl5 | NM_001039657.2 | chr19:3388856-3407823 |
| 13775 | Mrrf | NM_026422.2 | chr2:36136397-36190283 | 13872 | Mtm1 | NM_001164190.1 | chrX:71210766-71315295 |
| 13776 | Mrs2 | NM_001013389.2 | chr13:24992294-25020317 | 13873 | Mtmr1 | NM_016985.2 | chrX:71364759-71419196 |
| 13777 | Mrto4 | NM_001290810.1 | chr4:139347439-139352576 | 13874 | Mtmr10 | NM_172742.2 | chr7:64287669-64340806 |
| 13778 | Mrvi1 | NM_010826.5 | chr7:110868257-110946187 | 13875 | Mtmr11 | NM_181409.3 | chr3:96161969-96171718 |
| 13779 | Ms4a1 | NM_007641.5 | chr19:11250602-11266151 | 13876 | Mtmr12 | NM_172958.3 | chr15:12205093-12272240 |
| 13780 | Ms4a10 | NM_023529.2 | chr19:10962294-10974670 | 13877 | Mtmr14 | NM_026849.2 | chr6:113237842-113281392 |
| 13781 | Ms4a13 | NM_198224.3 | chr19:11169417-11196723 | 13878 | Mtmr2 | NM_023858.3 | chr9:13749180-13806481 |
| 13782 | Ms4a15 | NM_001034898.2 | chr19:10978306-10993250 | 13879 | Mtmr3 | NM_028860.2 | chr11:4480867-4594815 |
| 13783 | Ms4a18 | NM_001251849.1 | chr19:10997024-11017963 | 13880 | Mtmr4 | NM_133215.1 | chr11:87592216-87616296 |
| 13784 | Ms4a2 | NM_001276328.1 | chr19:11615520-11623719 | 13881 | Mtmr6 | NM_144843.4 | chr14:60265204-60302371 |
| 13785 | Ms4a3 | NM_133246.5 | chr19:11629498-11640851 | 13882 | Mtmr7 | NM_001040699.1 | chr8:40550158-40634792 |
| 13786 | Ms4a4b | NM_021718.2 | chr19:11443557-11463548 | 13883 | Mtmr9 | NM_177594.1 | chr14:63523609-63543953 |
| 13787 | Ms4a4c | NM_029499.3 | chr19:11407660-11427246 | 13884 | Mtnr1a | NM_008639.2 | chr8:45069209-45088506 |
| 13788 | Ms4a4d | NM_025658.4 | chr19:11536848-11558466 | 13885 | Mtnr1b | NM_145712.2 | chr9:15862613-15874556 |
| 13789 | Ms4a5 | NM_183190.2 | chr19:11273865-11283813 | 13886 | Mto1 | NM_026658.2 | chr9:78448209-78474152 |
| 13790 | Ms4a6b | NM_027209.3 | chr19:11518558-11530403 | 13887 | Mtor | NM_020009.2 | chr4:148448581-148557685 |
| 13791 | Ms4a6c | NM_001166376.1 | chr19:11469367-11472736 | 13888 | Mtpap | NM_026157.2 | chr18:4375591-4397330 |
| 13792 | Ms4a6d | NM_026835.2 | chr19:11586605-11604804 | 13889 | Mtpn | NM_008098.4 | chr6:35508823-35539888 |
| 13793 | Ms4a7 | NM_001025610.4 | chr19:11321038-11336146 | 13890 | Mtr | NM_001081128.3 | chr13:12186539-12258113 |
| 13794 | Ms4a8a | NM_022430.2 | chr19:11067470-11081103 | 13891 | Mtrf1 | NM_145960.4 | chr14:79397771-79423650 |
| 13795 | Msantd1 | NM_207277.1 | chr5:34915914-34923839 | 13892 | Mtrf1l | NM_175374.3 | chr10:5811886-5823943 |
| 13796 | Msantd2 | NM_146222.2 | chr9:37489320-37524150 | 13893 | Mtrr | NM_172480.3 | chr13:68560779-68582169 |
| 13797 | Msantd3 | NM_001145924.1 | chr4:48540080-48561920 | 13894 | Mtss1 | NM_001146180.1 | chr15:58941233-59082026 |
| 13798 | Msantd4 | NM_145609.1 | chr9:4383536-4386869 | 13895 | Mtss1l | NM_198625.2 | chr8:110721475-110741401 |
| 13799 | Msc | NM_010827.2 | chr1:14753345-14755966 | 13896 | Mttp | NM_001163457.1 | chr3:138089854-138143388 |
| 13800 | Msgn1 | NM_019544.1 | chr12:11208381-11208948 | 13897 | Mturn | NM_001289740.1 | chr6:54681623-54703855 |
| 13801 | Msh2 | NM_008628.2 | chr17:87672556-87723713 | 13898 | Mtus1 | NM_001005863.2 | chr8:40990911-41133726 |
| 13802 | Msh3 | NM_010829.2 | chr13:92211880-92355003 | 13899 | Mtus2 | NM_029920.7 | chr5:147957319-148316065 |
| 13803 | Msh4 | NM_001282054.1 | chr3:153857140-153904891 | 13900 | Mtx1 | NM_001161824.1 | chr3:89209080-89214335 |
| 13804 | Msh5 | NM_001146215.2 | chr17:35028604-35046745 | 13901 | Mtx2 | NM_016804.4 | chr2:74825811-74876748 |
| 13805 | Msh6 | NM_010830.2 | chr17:87975049-87990892 | 13902 | Mtx3 | NM_001162945.1 | chr13:92844786-92858230 |
| 13806 | Msi1 | NM_008629.1 | chr5:115429684-115454202 | 13903 | Muc1 | NM_013605.2 | chr3:89229055-89233381 |
| 13807 | Msi2 | NM_001201341.1 | chr11:88685953-88718267 | 13904 | Muc13 | NM_010739.2 | chr16:33794036-33819927 |
| 13808 | Msl1 | NM_028722.2 | chr11:98795768-98807859 | 13905 | Muc15 | NM_001162786.1 | chr2:110721493-110739537 |
| 13809 | Msl2 | NM_001100451.2 | chr9:101074761-101104799 | 13906 | Muc19 | NM_207243.2 | chr15:91838325-91934555 |
| 13810 | Msl3 | NM_010832.5 | chrX:168653098-168673902 | 13907 | Muc2 | NM_023566.3 | chr7:141690339-141754694 |
| 13811 | Msl3l2 | NM_001163833.1 | chr10:56106916-56116880 | 13908 | Muc20 | NM_001145874.1 | chr16:32777418-32797435 |
| 13812 | Msln | NM_018857.1 | chr17:25748612-25754327 | 13909 | Muc4 | NM_080457.3 | chr16:32735685-32782390 |
| 13813 | Mslnl | NM_177822.3 | chr17:25736039-25748330 | 13910 | Muc5ac | NM_010844.1 | chr7:141789010-141819135 |
| 13814 | Msmb | NM_020597.3 | chr14:32142022-32158326 | 13911 | Muc5b | NM_028801.2 | chr7:141839071-141873085 |
| 13815 | Msmo1 | NM_025436.2 | chr8:64718144-64733578 | 13912 | Muc6 | NM_181729.2 | chr7:141634048-141655308 |
| 13816 | Msmp | NM_001099314.1 | chr4:43583215-43584494 | 13913 | Mucl1 | NM_009268.1 | chr15:103751922-103899300 |
| 13817 | Msn | NM_010833.2 | chrX:96096044-96168553 | 13914 | Mug1 | NM_008645.3 | chr6:121838540-121889057 |
| 13818 | Msr1 | NM_001113326.1 | chr8:39581699-39642678 | 13915 | Mug2 | NM_008646.3 | chr6:122006797-122085967 |
| 13819 | Msra | NM_001253712.1 | chr14:64122620-64441040 | 13916 | Mug-ps1 | NR_027619.1 | chr6:122176052-122248566 |
| 13820 | Msrb1 | NM_013759.2 | chr17:24736641-24742778 | 13917 | Mul1 | NM_026689.3 | chr4:138434671-138442265 |
| 13821 | Msrb2 | NM_029619.2 | chr2:19371635-19394971 | 13918 | Mum1 | NM_023431.5 | chr10:80226597-80245144 |
| 13822 | Msrb3 | NM_177092.4 | chr10:120781100-120898971 | 13919 | Mum1l1 | NM_001164630.1 | chrX:139210043-139238335 |
| 13823 | Mss51 | NM_029104.1 | chr14:20482863-20496901 | 13920 | Mup1 | NM_001163010.1 | chr4:60498933-60501960 |
| 13824 | Mst1 | NM_008243.3 | chr9:108080435-108085027 | 13921 | Mup10 | NM_001122647.1 | chr4:60578260-60582155 |
| 13825 | Mst1r | NM_001287261.1 | chr9:107915002-107920383 | 13922 | Mup11 | NM_001164526.1 | chr4:60658465-60662395 |
| 13826 | Mstn | NM_010834.3 | chr1:53061639-53068079 | 13923 | Mup12 | NM_001199995.1 | chr4:60737380-60741281 |
| 13827 | Msto1 | NM_144898.2 | chr3:88909615-88913950 | 13924 | Mup13 | NM_001134674.1 | chr4:61224306-61228286 |
| 13828 | Msx1 | NM_010835.2 | chr5:37820490-37824585 | 13925 | Mup14 | NM_001199999.1 | chr4:61300036-61303998 |
| 13829 | Msx1os | NR_027920.1 | chr5:37820563-37822751 | 13926 | Mup15 | NM_001200004.1 | chr4:60066468-61439704 |
| 13830 | Msx2 | NM_013601.2 | chr13:53466880-53472780 | 13927 | Mup16 | NM_001199936.1 | chr4:61515592-61519486 |
| 13831 | Msx3 | NM_010836.3 | chr7:140046156-140049088 | 13928 | Mup17 | NM_001200006.1 | chr4:61591930-61595832 |
| 13832 | Mt1 | NM_013602.3 | chr8:94179088-94180327 | 13929 | Mup19 | NM_001135127.2 | chr4:61778327-61782224 |
| 13833 | Mt2 | NM_008630.2 | chr8:94172617-94173567 | 13930 | Mup2 | NM_001045550.2 | chr4:60136846-61439702 |
| 13834 | Mt3 | NM_013603.2 | chr8:94152606-94154148 | 13931 | Mup20 | NM_001012323.1 | chr4:62050234-62054117 |
| 13835 | Mt4 | NM_008631.2 | chr8:94137203-94139031 | 13932 | Mup21 | NM_001009550.2 | chr4:62147831-62150841 |
| 13836 | Mta1 | NM_054081.2 | chr12:113098277-113137206 | 13933 | Mup3 | NM_001039544.1 | chr4:62083475-62087312 |
| 13837 | Mta2 | NM_011842.3 | chr19:8941919-8952300 | 13934 | Mup4 | NM_008648.1 | chr4:59956805-59960665 |
| 13838 | Mta3 | NM_001171052.1 | chr17:83706162-83805422 | 13935 | Mup5 | NM_008649.2 | chr4:61831318-61835180 |
| 13839 | Mtag2 | NR_015480.1 | chr7:45366162-45367948 | 13936 | Mup6 | NM_001081285.1 | chr4:60003548-60006214 |
| 13840 | Mtap | NM_024433.2 | chr4:89137369-89181090 | 13937 | Mup7 | NM_001134675.1 | chr4:60066468-60070475 |
| 13841 | Mtap7d3 | NM_177293.3 | chrX:56797952-56822325 | 13938 | Mup8 | NM_001134676.1 | chr4:60218620-60222599 |
| 13842 | Mtbp | NM_001168250.1 | chr15:55557407-55610813 | 13939 | Mup9 | NM_001281979.1 | chr4:60418045-60421952 |
| 13843 | Mtch1 | NM_019880.3 | chr17:29332075-29347904 | 13940 | Murc | NM_026509.3 | chr4:48663513-48673492 |
| 13844 | Mtch2 | NM_019758.2 | chr2:90847154-90866634 | 13941 | Mus81 | NM_027877.3 | chr19:5482839-5488336 |
| 13845 | Mtcl1 | NM_001114098.1 | chr17:66336981-66449750 | 13942 | Musk | NM_001037127.2 | chr4:58285961-58374303 |
| 13846 | Mtcp1 | NM_001039373.5 | chrX:75410441-75416584 | 13943 | Mustn1 | NM_181390.3 | chr14:30879256-30881610 |
| 13847 | Mtdh | NM_026002.4 | chr15:34082718-34142385 | 13944 | Mut | NM_008650.3 | chr17:40934684-40961989 |
| 13848 | Mterf1a | NM_001013023.2 | chr5:3890580-3893933 | 13945 | Mutyh | NM_001159581.1 | chr4:116807733-116819431 |
| 13849 | Mterf1b | NM_001042670.1 | chr5:4192366-4197651 | 13946 | Mvb12a | NM_028617.2 | chr8:71542929-71548026 |
| 13850 | Mterfd1 | NM_025547.3 | chr13:66912097-66933072 | 13947 | Mvb12b | NM_175184.4 | chr2:33729955-33887946 |
| 13851 | Mterfd2 | NM_178051.4 | chr1:93301204-93305879 | 13948 | Mvd | NM_138656.2 | chr8:122433595-122443422 |
| 13852 | Mterfd3 | NM_028832.3 | chr10:85119432-85128027 | 13949 | Mvk | NM_023556.3 | chr5:114444268-114460590 |
| 13853 | Mtf1 | NM_008636.4 | chr4:124802548-124849800 | 13950 | Mvp | NM_080638.3 | chr7:126986859-127014594 |
| 13854 | Mtf2 | NM_001253877.1 | chr5:108065673-108109219 | 13951 | Mx1 | NM_010846.1 | chr16:97447036-97462905 |
| 13855 | Mtfmt | NM_027134.3 | chr9:65435781-65453054 | 13952 | Mx2 | NM_013606.1 | chr16:97536082-97560899 |
| 13856 | Mtfp1 | NM_026443.4 | chr11:4091480-4095431 | 13953 | Mxd1 | NM_010751.3 | chr6:86647044-86669159 |
| 13857 | Mtfr1 | NM_001253390.1 | chr3:19187328-19220817 | 13954 | Mxd3 | NM_016662.4 | chr13:55325167-55329730 |
| 13858 | Mtfr1l | NM_001256112.1 | chr4:134525554-134535268 | 13955 | Mxd4 | NM_010753.2 | chr5:34176579-34187710 |
| 13859 | Mtfr2 | NM_027930.3 | chr10:20347818-20361669 | 13956 | Mxi1 | NM_001008542.2 | chr19:53310505-53375810 |
| 13860 | Mtg1 | NM_199301.2 | chr7:140137563-140150786 | 13957 | Mxra7 | NM_026280.3 | chr11:116803399-116828046 |
| 13861 | Mtg2 | NM_001083328.1 | chr2:180070592-180085902 | 13958 | Mxra8 | NM_024263.4 | chr4:155839679-155844102 |
| 13862 | Mthfd1 | NM_138745.2 | chr12:76255231-76319820 | 13959 | Myadm | NM_001093764.1 | chr7:3289037-3299349 |
| 13863 | Mthfd1l | NM_001170785.1 | chr10:3973074-4167081 | 13960 | Myadml2 | NM_001204820.1 | chr11:120646030-120648337 |
| 13864 | Mthfd2 | NM_008638.2 | chr6:83305703-83317604 | 13961 | Myb | NM_001198914.1 | chr10:21124929-21160984 |
| 13865 | Mthfd2l | NM_026788.1 | chr5:90931195-91021370 | 13962 | Mybbp1a | NM_016776.2 | chr11:72441377-72451550 |
| 13866 | Mthfr | NM_001161798.1 | chr4:148041188-148059562 | 13963 | Mybl1 | NM_001290397.1 | chr1:9667410-9700209 |
| 13867 | Mthfs | NM_026829.2 | chr9:89211189-89240225 | 13964 | Mybl2 | NM_008652.2 | chr2:163054634-163084687 |
| 13868 | Mthfsd | NM_001166482.1 | chr8:121097556-121108379 | 13965 | Mybpc1 | NM_001252372.1 | chr10:88518278-88605152 |
| 13869 | Mtif2 | NM_001282118.1 | chr11:29526396-29545447 | 13966 | Mybpc2 | NM_146189.3 | chr7:44501698-44524669 |
| 13870 | Mtif3 | NM_001256100.1 | chr5:146951572-146963797 | 13967 | Mybpc3 | NM_008653.2 | chr2:91118143-91136516 |

Fig.21 - 73

| | | | | | | | |
|---|---|---|---|---|---|---|---|
| 13968 | Mybph | NM_016749.2 | chr1:134193447-134201232 | 14065 | N28178 | NM_172690.2 | chr4:42917250-42941607 |
| 13969 | Mybphl | NM_026831.1 | chr3:108364910-108380057 | 14066 | N4bp1 | NM_030563.2 | chr8:86841138-86885258 |
| 13970 | Myc | NM_001177354.1 | chr15:61985340-61990361 | 14067 | N4bp2 | NM_001024917.1 | chr5:65763520-65826784 |
| 13971 | Mycbp | NM_019660.3 | chr4:123905012-123912250 | 14068 | N4bp2l1 | NM_133898.4 | chr5:150571642-150594525 |
| 13972 | Mycbp2 | NM_207215.2 | chr14:103113410-103346800 | 14069 | N4bp2l2 | NM_201369.3 | chr5:150635972-150665612 |
| 13973 | Mycbpap | NM_170671.2 | chr11:94501346-94521502 | 14070 | N4bp3 | NM_145974.3 | chr11:51643088-51651073 |
| 13974 | Mycl | NM_008506.3 | chr4:122995930-123002480 | 14071 | N6amt1 | NM_001159331.1 | chr16:87354184-87368649 |
| 13975 | Mycn | NM_008709.3 | chr12:12936092-12941836 | 14072 | N6amt2 | NM_026526.2 | chr14:57549597-57571569 |
| 13976 | Mycs | NM_010850.2 | chrX:5466903-5469265 | 14073 | Naa10 | NM_001177965.1 | chrX:73916869-73921944 |
| 13977 | Myct1 | NM_026793.2 | chr10:5593727-5606791 | 14074 | Naa11 | NM_001033191.2 | chr5:97382208-97392330 |
| 13978 | Myd88 | NM_010851.2 | chr9:119335987-119340040 | 14075 | Naa15 | NM_053089.3 | chr3:51416015-51475985 |
| 13979 | Myef2 | NM_001162417.1 | chr2:125086270-125123660 | 14076 | Naa16 | NM_025832.2 | chr14:79334506-79390668 |
| 13980 | Myeov2 | NM_001163425.1 | chr1:92637144-92641985 | 14077 | Naa20 | NM_001141965.1 | chr2:145903240-145916425 |
| 13981 | Myf5 | NM_008656.5 | chr10:107482907-107486134 | 14078 | Naa25 | NM_172722.3 | chr5:121397981-121440113 |
| 13982 | Myf6 | NM_008657.2 | chr10:107492859-107494729 | 14079 | Naa30 | NM_001081430.1 | chr14:49172226-49191031 |
| 13983 | Myg1 | NM_021713.2 | chr15:102331708-102338138 | 14080 | Naa35 | NM_030153.2 | chr13:59585332-59634781 |
| 13984 | Myh1 | NM_030679.1 | chr11:67200113-67224575 | 14081 | Naa38 | NM_030083.2 | chr11:69395790-69396671 |
| 13985 | Myh10 | NM_175260.2 | chr11:68691914-68816624 | 14082 | Naa40 | NM_027643.1 | chr19:7225667-7241222 |
| 13986 | Myh11 | NM_001161775.1 | chr16:14194526-14291408 | 14083 | Naa50 | NM_028108.3 | chr16:44139808-44163364 |
| 13987 | Myh13 | NM_001081250.1 | chr11:67327102-67371502 | 14084 | Naa60 | NM_001290689.1 | chr16:3884618-3904781 |
| 13988 | Myh14 | NM_001271538.1 | chr7:44605802-44665503 | 14085 | Naaa | NM_001163687.1 | chr5:92257659-92278181 |
| 13989 | Myh15 | NM_001166210.1 | chr16:49057485-49199104 | 14086 | Naalad2 | NM_028279.3 | chr9:18323020-18385928 |
| 13990 | Myh2 | NM_001039545.2 | chr11:67171026-67197517 | 14087 | Naaladl1 | NM_001009546.1 | chr19:6105797-6115555 |
| 13991 | Myh3 | NM_001099635.1 | chr11:67078299-67102291 | 14088 | Nab1 | NM_008667.3 | chr1:52455848-52500448 |
| 13992 | Myh4 | NM_010855.3 | chr11:67237811-67260447 | 14089 | Nab2 | NM_001122895.1 | chr10:127660917-127666703 |
| 13993 | Myh6 | NM_001164171.1 | chr14:54941920-54966607 | 14090 | Nabp1 | NM_028696.3 | chr1:51469487-51478399 |
| 13994 | Myh7 | NM_080728.2 | chr14:54970687-54994549 | 14091 | Nabp2 | NM_027257.1 | chr10:128401394-128409796 |
| 13995 | Myh7b | NM_001085378.2 | chr2:155611242-155634307 | 14092 | Naca | NM_001113199.1 | chr10:128035345-128048637 |
| 13996 | Myh8 | NM_177369.3 | chr11:67277123-67308633 | 14093 | Nacad | NM_001081652.1 | chr11:6597822-6606054 |
| 13997 | Myh9 | NM_022410.3 | chr15:77760584-77842175 | 14094 | Nacc1 | NM_025788.3 | chr8:84670478-84687862 |
| 13998 | Myl1 | NM_001113387.1 | chr1:66924295-66935366 | 14095 | Nacc2 | NM_001037098.1 | chr2:26055535-26092396 |
| 13999 | Myl10 | NM_001085387.2 | chr5:136694176-136701094 | 14096 | Nadk | NM_001159637.1 | chr4:155563814-155591001 |
| 14000 | Myl12a | NM_026064.2 | chr17:70993792-71002533 | 14097 | Nadk2 | NM_001040395.4 | chr15:9071259-9110496 |
| 14001 | Myl12b | NM_023402.2 | chr17:70973962-70990516 | 14098 | Nadsyn1 | NM_030221.2 | chr7:143795590-143822852 |
| 14002 | Myl2 | NM_010861.3 | chr5:122100979-122106854 | 14099 | Nae1 | NM_144931.3 | chr8:104511027-104534637 |
| 14003 | Myl3 | NM_010859.2 | chr9:110763680-110769794 | 14100 | Naf1 | NM_001163564.1 | chr8:66860216-66890564 |
| 14004 | Myl4 | NM_010858.4 | chr11:104550662-104587219 | 14101 | Naga | NM_008669.4 | chr15:82329531-82338826 |
| 14005 | Myl6 | NM_010860.3 | chr10:128490860-128493825 | 14102 | Nagk | NM_001164187.1 | chr6:83795157-83802556 |
| 14006 | Myl6b | NM_172259.1 | chr10:128494156-128498685 | 14103 | Naglu | NM_013792.2 | chr11:101070093-101077671 |
| 14007 | Myl7 | NM_022879.2 | chr11:5896636-5898782 | 14104 | Nagpa | NM_013796.3 | chr16:5195279-5204012 |
| 14008 | Myl9 | NM_172118.1 | chr2:156775463-156781657 | 14105 | Nags | NM_145829.2 | chr11:102145512-102149528 |
| 14009 | Mylip | NM_153789.3 | chr13:45389741-45411940 | 14106 | Naif1 | NM_194335.2 | chr2:32450456-32455476 |
| 14010 | Mylk | NM_139300.3 | chr16:34784949-35002434 | 14107 | Naip1 | NM_008670.2 | chr13:100407769-100452864 |
| 14011 | Mylk2 | NM_001081044.2 | chr2:152911351-152923065 | 14108 | Naip2 | NM_001126182.2 | chr13:100144062-100202122 |
| 14012 | Mylk3 | NM_175441.5 | chr8:85324928-85365324 | 14109 | Naip5 | NM_010870.2 | chr13:100211739-100247336 |
| 14013 | Mylk4 | NM_001166030.1 | chr13:32700826-32781779 | 14110 | Naip6 | NM_010871.2 | chr13:100281120-100316616 |
| 14014 | Mylpf | NM_016754.5 | chr7:127211607-127214287 | 14111 | Naip7 | NM_021545.1 | chr13:100283114-100317688 |
| 14015 | Mynn | NM_001289621.1 | chr3:30602086-30619873 | 14112 | Nalcn | NM_177393.4 | chr14:123276640-123627144 |
| 14016 | Myo10 | NM_019472.2 | chr15:25622549-25813671 | 14113 | Nampt | NM_021524.2 | chr12:32820334-32853369 |
| 14017 | Myo15 | NM_001103171.1 | chr11:60469338-60528368 | 14114 | Nanog | NM_001289828.1 | chr6:122707564-122714633 |
| 14018 | Myo16 | NM_001081397.1 | chr8:10153922-10633950 | 14115 | Nanos1 | NM_178421.3 | chr19:60755986-60759914 |
| 14019 | Myo18a | NM_001291212.1 | chr11:77777234-77865988 | 14116 | Nanos2 | NM_194064.2 | chr7:18987523-18988962 |
| 14020 | Myo18b | NM_028901.2 | chr5:112688875-112896362 | 14117 | Nanos3 | NM_194059.2 | chr8:84173732-84176552 |
| 14021 | Myo19 | NM_025414.3 | chr11:84880219-84911131 | 14118 | Nanp | NM_026086.2 | chr2:151029684-151039379 |
| 14022 | Myo1a | NM_001081219.2 | chr10:127705255-127720940 | 14119 | Nans | NM_053179.3 | chr4:46489328-46503438 |
| 14023 | Myo1b | NM_001161817.2 | chr1:51749757-51915978 | 14120 | Nap1l1 | NM_001146707.1 | chr10:111480616-111498150 |
| 14024 | Myo1c | NM_001080774.1 | chr11:75652149-75674634 | 14121 | Nap1l2 | NM_008671.2 | chrX:103184058-103186664 |
| 14025 | Myo1d | NM_177390.3 | chr11:80482126-80780025 | 14122 | Nap1l3 | NM_138742.1 | chrX:122394560-122397385 |
| 14026 | Myo1e | NM_181072.3 | chr9:70207349-70400067 | 14123 | Nap1l4 | NM_001285489.1 | chr7:143513578-143549120 |
| 14027 | Myo1f | NM_053214.2 | chr17:33555706-33607764 | 14124 | Nap1l5 | NM_021432.2 | chr6:58905232-58907126 |
| 14028 | Myo1g | NM_178440.4 | chr11:6506547-6520958 | 14125 | Napa | NM_025898.3 | chr7:16098642-16117975 |
| 14029 | Myo1h | NM_001164573.1 | chr5:114314940-114364576 | 14126 | Napb | NM_019632.3 | chr2:148694656-148732420 |
| 14030 | Myo3a | NM_148413.3 | chr2:22227502-22423370 | 14127 | Napepld | NM_178728.5 | chr5:21662902-21701345 |
| 14031 | Myo3b | NM_177376.4 | chr2:70039125-70429195 | 14128 | Napg | NM_028017.1 | chr18:62977915-62999451 |
| 14032 | Myo5a | NM_010864.2 | chr9:75071205-75223687 | 14129 | Naprt1 | NM_172607.3 | chr15:75890963-75894481 |
| 14033 | Myo5b | NM_201600.2 | chr18:74442618-74771477 | 14130 | Napsa | NM_008437.1 | chr7:44572444-44586846 |
| 14034 | Myo5c | NM_001081322.1 | chr9:75232013-75305450 | 14131 | Narf | NM_026272.3 | chr11:121237235-121255856 |
| 14035 | Myo6 | NM_001039546.2 | chr9:80165033-80311729 | 14132 | Narfl | NM_026238.4 | chr17:25773775-25783332 |
| 14036 | Myo7a | NM_001256081.1 | chr7:98051053-98119493 | 14133 | Narg2 | NM_145618.3 | chr9:69397997-69433074 |
| 14037 | Myo7b | NM_032394.3 | chr18:31959233-32036931 | 14134 | Nars | NM_001142950.1 | chr18:64499646-64516557 |
| 14038 | Myo9a | NM_173018.2 | chr9:59751173-59928866 | 14135 | Nars2 | NM_153591.4 | chr7:96951526-97064757 |
| 14039 | Myo9b | NM_001142322.1 | chr8:71272713-71360712 | 14136 | Nasp | NM_001081475.1 | chr4:116601051-116627497 |
| 14040 | Myoc | NM_010865.3 | chr1:162639149-162649694 | 14137 | Nat1 | NM_008673.1 | chr8:67490757-67492103 |
| 14041 | Myocd | NM_145136.4 | chr11:65176570-65269989 | 14138 | Nat10 | NM_153126.2 | chr2:103721258-103761250 |
| 14042 | Myod1 | NM_010866.2 | chr7:46376473-46379092 | 14139 | Nat14 | NM_201355.3 | chr7:4922250-4925006 |
| 14043 | Myof | NM_001099634.1 | chr19:37899035-38043577 | 14140 | Nat2 | NM_001168577.1 | chr8:67494874-67502578 |
| 14044 | Myog | NM_031189.2 | chr1:134290003-134292548 | 14141 | Nat3 | NM_008674.2 | chr8:67523853-67548627 |
| 14045 | Myom1 | NM_001083934.1 | chr17:71019556-71126856 | 14142 | Nat6 | NM_019750.3 | chr9:107580169-107584048 |
| 14046 | Myom2 | NM_008664.2 | chr8:15057652-15133419 | 14143 | Nat8 | NM_023455.3 | chr6:85830386-85831890 |
| 14047 | Myom3 | NM_001085509.2 | chr4:135759714-135815567 | 14144 | Nat8l | NM_001001985.3 | chr5:33995983-34005916 |
| 14048 | Myot | NM_001033621.3 | chr18:44334073-44355722 | 14145 | Nat9 | NM_025400.3 | chr11:115182831-115187316 |
| 14049 | Myoz1 | NM_021508.3 | chr14:20649101-20656540 | 14146 | Nav1 | NM_173437.2 | chr1:135434579-135585355 |
| 14050 | Myoz2 | NM_021503.2 | chr3:123006205-123034987 | 14147 | Nav2 | NM_001111016.1 | chr7:48959072-49610088 |
| 14051 | Myoz3 | NM_133363.3 | chr18:60573716-60591716 | 14148 | Nav3 | NM_001081035.1 | chr10:109683438-110000219 |
| 14052 | Mypn | NM_182992.2 | chr10:63115794-63203952 | 14149 | Nbas | NM_027706.1 | chr12:13269126-13583811 |
| 14053 | Mypop | NM_145579.3 | chr7:18991244-19001766 | 14150 | Nbea | NM_030595.1 | chr3:55625197-56183701 |
| 14054 | Myrf | NM_001033481.1 | chr19:10208270-10240748 | 14151 | Nbeal1 | NM_173444.2 | chr1:60180598-60334705 |
| 14055 | Myrfl | NM_001033333.3 | chr10:116776544-116896879 | 14152 | Nbeal2 | NM_183276.2 | chr9:110624788-110654161 |
| 14056 | Myrip | NM_144557.5 | chr9:120304072-120474834 | 14153 | Nbl1 | NM_008675.2 | chr4:139082291-139092970 |
| 14057 | Mysm1 | NM_177239.2 | chr4:94942040-94979100 | 14154 | Nbn | NM_013752.3 | chr4:15957966-15992589 |
| 14058 | Myt1 | NM_001171615.1 | chr2:181763331-181827775 | 14155 | Nbr1 | NM_001252220.1 | chr11:101552106-101581951 |
| 14059 | Myt1l | NM_001093775.1 | chr12:29528383-29923209 | 14156 | Ncald | NM_001170866.1 | chr15:37366174-37792000 |
| 14060 | Myzap | NM_001033208.4 | chr9:71504346-71592360 | 14157 | Ncam1 | NM_001081445.1 | chr9:49502145-49799069 |
| 14061 | Mzb1 | NM_027222.3 | chr18:35647264-35649367 | 14158 | Ncam2 | NM_001113208.1 | chr16:81200696-81624287 |
| 14062 | Mzf1 | NM_001290452.1 | chr7:13042302-13054764 | 14159 | Ncan | NM_007789.3 | chr8:70093084-70120844 |
| 14063 | Mzt1 | NM_175245.4 | chr14:99034543-99046136 | 14160 | Ncapd2 | NM_146171.1 | chr6:125168006-125191586 |
| 14064 | Mzt2 | NM_029354.2 | chr16:15848440-15863322 | 14161 | Ncapd3 | NM_178113.3 | chr9:27030174-27095315 |

Fig.21 - 74

| | | | | | | | |
|---|---|---|---|---|---|---|---|
| 14162 | Ncapg | NM_019438.1 | chr5:45669924-45700547 | 14259 | Ndufv2 | NM_001278415.1 | chr17:66078794-66101559 |
| 14163 | Ncapg2 | NM_133762.3 | chr12:116405401-116463531 | 14260 | Ndufv3 | NM_001083891.1 | chr17:31520114-31531325 |
| 14164 | Ncaph | NM_144818.3 | chr2:127103809-127133954 | 14261 | Neat1 | NR_003513.3 | chr19:5842295-5845480 |
| 14165 | Ncaph2 | NM_001115132.2 | chr15:89355718-89372850 | 14262 | Neb | NM_010889.1 | chr2:52136646-52338798 |
| 14166 | Ncbp1 | NM_001033201.3 | chr4:46138510-46172402 | 14263 | Nebl | NM_028757.2 | chr2:17346733-17731068 |
| 14167 | Ncbp2 | NM_026554.4 | chr16:31948545-31958472 | 14264 | Necab1 | NM_178617.4 | chr4:14952244-15149131 |
| 14168 | Nccrp1 | NM_001081115.1 | chr7:28543595-28547254 | 14265 | Necab2 | NM_054095.2 | chr8:119446718-119472635 |
| 14169 | Ncdn | NM_011986.4 | chr4:126743749-126753429 | 14266 | Necab3 | NM_021546.3 | chr2:154544404-154558853 |
| 14170 | Nceh1 | NM_178772.3 | chr3:27183003-27244911 | 14267 | Necap1 | NM_026267.2 | chr6:122874556-122888941 |
| 14171 | Ncf1 | NM_001286037.1 | chr5:134220258-134229625 | 14268 | Necap2 | NM_025383.3 | chr4:141066511-141078345 |
| 14172 | Ncf2 | NM_010877.5 | chr1:152800152-152836990 | 14269 | Nedd1 | NM_008682.2 | chr10:92684744-92722418 |
| 14173 | Ncf4 | NM_008677.2 | chr15:78244810-78262580 | 14270 | Nedd4 | NM_010890.3 | chr9:72662346-72749848 |
| 14174 | Nck1 | NM_010878.2 | chr9:100495002-100546053 | 14271 | Nedd4l | NM_001114386.1 | chr18:64887755-65217826 |
| 14175 | Nck2 | NM_010879.3 | chr1:43445750-43570518 | 14272 | Nedd8 | NM_008683.3 | chr14:55662266-55671906 |
| 14176 | Nckap1 | NM_001290745.1 | chr2:80500511-80581182 | 14273 | Nedd9 | NM_001111324.2 | chr13:41309915-41487360 |
| 14177 | Nckap1l | NM_153505.4 | chr15:103453824-103498800 | 14274 | Nefh | NM_010904.3 | chr11:4938755-4948064 |
| 14178 | Nckap5 | NM_001081756.1 | chr1:125913635-126830632 | 14275 | Nefl | NM_010910.1 | chr14:68083883-68087737 |
| 14179 | Nckap5l | NM_001001884.1 | chr15:99422033-99457748 | 14276 | Nefm | NM_008691.2 | chr14:68119544-68125004 |
| 14180 | Nckipsd | NM_030729.4 | chr9:108808379-108818366 | 14277 | Negr1 | NM_001039094.3 | chr3:156561793-157316464 |
| 14181 | Ncl | NM_010880.3 | chr1:86344718-86359455 | 14278 | Neil1 | NM_028347.2 | chr9:57143255-57147034 |
| 14182 | Ncln | NM_134009.3 | chr10:81486458-81496363 | 14279 | Neil2 | NM_201610.2 | chr14:63182444-63193525 |
| 14183 | Ncmap | NM_001168498.1 | chr4:135369576-135385672 | 14280 | Neil3 | NM_146208.2 | chr8:53586866-53639065 |
| 14184 | Ncoa1 | NM_010881.2 | chr12:4247361-4477182 | 14281 | Nek1 | NM_175089.4 | chr8:60993192-61131346 |
| 14185 | Ncoa2 | NM_001302702.1 | chr1:13139105-13374691 | 14282 | Nek10 | NM_001195229.1 | chr14:14820814-15006693 |
| 14186 | Ncoa3 | NM_008679.3 | chr2:165992636-166073242 | 14283 | Nek11 | NM_172461.3 | chr9:105162466-105395287 |
| 14187 | Ncoa4 | NM_001033988.2 | chr14:32159886-32179855 | 14284 | Nek2 | NM_010892.3 | chr1:191821472-191833049 |
| 14188 | Ncoa5 | NM_144892.1 | chr2:165000356-165034779 | 14285 | Nek3 | NM_001162947.1 | chr8:22128285-22162494 |
| 14189 | Ncoa6 | NM_001242558.1 | chr2:155390655-155440783 | 14286 | Nek4 | NM_011849.3 | chr14:30951376-30988821 |
| 14190 | Ncoa7 | NM_001111267.2 | chr10:30645581-30655867 | 14287 | Nek5 | NM_177898.4 | chr8:22073615-22125053 |
| 14191 | Ncor1 | NM_001252313.1 | chr11:62316425-62438515 | 14288 | Nek6 | NM_001159631.1 | chr2:38511875-38587490 |
| 14192 | Ncor2 | NM_001253904.1 | chr5:125017152-125179214 | 14289 | Nek7 | NM_021605.4 | chr1:138483836-138619757 |
| 14193 | Ncr1 | NM_010746.3 | chr7:4337723-4345164 | 14290 | Nek8 | NM_080849.3 | chr11:78166105-78176666 |
| 14194 | Ncs1 | NM_019681.3 | chr2:31245922-31295471 | 14291 | Nek9 | NM_145138.2 | chr12:85299513-85339362 |
| 14195 | Ncstn | NM_021607.3 | chr1:172066012-172082749 | 14292 | Nelfa | NM_011914.2 | chr5:33898179-33936258 |
| 14196 | Nctc1 | NR_002452.2 | chr7:142544608-142558598 | 14293 | Nelfb | NM_021393.3 | chr2:25199711-25211489 |
| 14197 | Ndc1 | NM_028355.3 | chr4:107367783-107414338 | 14294 | Nelfcd | NM_020580.3 | chr2:174415803-174427502 |
| 14198 | Ndc80 | NM_023294.2 | chr17:71496099-71526857 | 14295 | Nelfe | NM_001045863.1 | chr17:34850390-34856372 |
| 14199 | Nde1 | NM_001114085.1 | chr16:14163274-14192923 | 14296 | Nell1 | NM_001037906.2 | chr7:49975349-50863289 |
| 14200 | Ndel1 | NM_023668.2 | chr11:68821445-68853131 | 14297 | Nell1os | NR_045928.1 | chr7:50517308-50575884 |
| 14201 | Ndfip1 | NM_022996.1 | chr18:38418974-38464406 | 14298 | Nell2 | NM_001289653.1 | chr15:95219439-95528252 |
| 14202 | Ndfip2 | NM_001190989.1 | chr14:105258672-105309298 | 14299 | Nemf | NM_025441.3 | chr12:69311542-69357176 |
| 14203 | Ndn | NM_010882.3 | chr7:62348276-62349927 | 14300 | Nenf | NM_025424.2 | chr1:191306796-191318118 |
| 14204 | Ndnf | NM_172399.3 | chr6:65671610-65706930 | 14301 | Neo1 | NM_001042752.1 | chr9:58874679-59036441 |
| 14205 | Ndnl2 | NM_023239.4 | chr7:64871649-64873040 | 14302 | Nepn | NM_025684.2 | chr10:52391607-52404604 |
| 14206 | Ndor1 | NM_001082476.2 | chr2:25244812-25255414 | 14303 | Nes | NM_016701.3 | chr3:87971092-87980451 |
| 14207 | Ndp | NM_010883.3 | chrX:16885520-16911774 | 14304 | Nespas | NR_002846.2 | chr2:174281236-174295436 |
| 14208 | Ndrg1 | NM_008681.2 | chr15:66929317-66969641 | 14305 | Net1 | NM_001047159.2 | chr13:3882017-3893581 |
| 14209 | Ndrg2 | NM_001145959.1 | chr14:51905270-51913488 | 14306 | Neto1 | NM_144946.4 | chr18:86394951-86501897 |
| 14210 | Ndrg3 | NM_013865.2 | chr2:156927341-156992111 | 14307 | Neto2 | NM_001081324.1 | chr8:85636587-85691009 |
| 14211 | Ndrg4 | NM_001195006.1 | chr8:95703036-95715119 | 14308 | Neu1 | NM_010893.3 | chr17:34931252-34937297 |
| 14212 | Ndst1 | NM_008306.4 | chr18:60685975-60713389 | 14309 | Neu2 | NM_001160163.1 | chr1:87574026-87597840 |
| 14213 | Ndst2 | NM_010811.2 | chr14:20723729-20734562 | 14310 | Neu3 | NM_016720.2 | chr7:99811438-99828417 |
| 14214 | Ndst3 | NM_031186.3 | chr3:123526165-123672477 | 14311 | Neu4 | NM_173772.3 | chr1:94020492-94028330 |
| 14215 | Ndst4 | NM_022565.2 | chr3:125404090-125724986 | 14312 | Neurl1a | NM_001163480.1 | chr19:47228842-47259441 |
| 14216 | Ndufa1 | NM_019443.2 | chrX:37187588-37191238 | 14313 | Neurl1b | NM_001081656.2 | chr17:26414964-26446342 |
| 14217 | Ndufa10 | NM_024197.1 | chr1:92439718-92473758 | 14314 | Neurl2 | NM_001082974.2 | chr2:164830729-164833596 |
| 14218 | Ndufa11 | NM_027244.1 | chr17:56717761-56724248 | 14315 | Neurl3 | NM_153408.2 | chr1:36264601-36273425 |
| 14219 | Ndufa12 | NM_025551.3 | chr10:94199008-94220948 | 14316 | Neurl4 | NM_001013414.3 | chr11:69901815-69913824 |
| 14220 | Ndufa13 | NM_023312.2 | chr8:69894181-69902558 | 14317 | Neurod1 | NM_010894.2 | chr2:79452640-79456636 |
| 14221 | Ndufa2 | NM_010885.5 | chr18:36742331-36744587 | 14318 | Neurod2 | NM_010895.3 | chr11:98325416-98329645 |
| 14222 | Ndufa3 | NM_025348.2 | chr7:3617372-3620161 | 14319 | Neurod4 | NM_007501.4 | chr10:130268151-130280240 |
| 14223 | Ndufa4 | NM_010886.2 | chr6:11900372-11907446 | 14320 | Neurod6 | NM_009717.2 | chr6:55677818-55681263 |
| 14224 | Ndufa4l2 | NM_001098789.1 | chr10:127514938-127517156 | 14321 | Neurog1 | NM_010896.2 | chr13:56250497-56252163 |
| 14225 | Ndufa5 | NM_026614.2 | chr6:24518665-24527687 | 14322 | Neurog2 | NM_009718.2 | chr3:127633144-127635631 |
| 14226 | Ndufa6 | NM_025987.3 | chr15:82350138-82354291 | 14323 | Neurog3 | NM_009719.6 | chr10:62133089-62134763 |
| 14227 | Ndufa7 | NM_023202.4 | chr17:33824571-33838316 | 14324 | Nexn | NM_199465.2 | chr3:152236983-152266320 |
| 14228 | Ndufa8 | NM_026703.2 | chr2:36036333-36049292 | 14325 | Nf1 | NM_010897.2 | chr11:79339891-79581609 |
| 14229 | Ndufa9 | NM_025358.3 | chr6:126821862-126849144 | 14326 | Nf2 | NM_001252250.1 | chr11:4765844-4849544 |
| 14230 | Ndufab1 | NM_028177.3 | chr7:122088043-122101848 | 14327 | Nfam1 | NM_001271411.1 | chr15:82996735-83033397 |
| 14231 | Ndufaf1 | NM_027175.3 | chr2:119655450-119662798 | 14328 | Nfasc | NM_001160316.1 | chr1:132564689-132642858 |
| 14232 | Ndufaf2 | NM_001127346.1 | chr13:108052588-108158625 | 14329 | Nfat5 | NM_001286260.1 | chr8:107293469-107379517 |
| 14233 | Ndufaf3 | NM_023247.1 | chr9:108565864-108567342 | 14330 | Nfatc1 | NM_001164109.1 | chr18:80647434-80713071 |
| 14234 | Ndufaf4 | NM_026742.4 | chr4:24898082-24905001 | 14331 | Nfatc2 | NM_001037177.2 | chr2:168518198-168590365 |
| 14235 | Ndufaf5 | NM_027093.4 | chr2:140170645-140205252 | 14332 | Nfatc2ip | NM_010900.3 | chr7:126382853-126396737 |
| 14236 | Ndufaf6 | NM_001085493.2 | chr4:11051045-11076205 | 14333 | Nfatc3 | NM_010901.2 | chr8:106059602-106130537 |
| 14237 | Ndufaf7 | NM_028611.3 | chr17:78937134-78948052 | 14334 | Nfatc4 | NM_001168346.1 | chr14:55824794-55833943 |
| 14238 | Ndufb10 | NM_026684.2 | chr17:24722066-24724388 | 14335 | Nfe2 | NM_008685.3 | chr15:103248211-103255439 |
| 14239 | Ndufb11 | NM_019435.4 | chrX:20615325-20617564 | 14336 | Nfe2l1 | NM_001130450.1 | chr11:96817413-96829502 |
| 14240 | Ndufb2 | NM_026612.3 | chr6:39592582-39599471 | 14337 | Nfe2l2 | NM_010902.4 | chr2:75675512-75704663 |
| 14241 | Ndufb3 | NM_025597.3 | chr1:58586396-58595964 | 14338 | Nfe2l3 | NM_010903.1 | chr6:51432669-51458768 |
| 14242 | Ndufb4 | NM_026610.1 | chr16:37647601-37654368 | 14339 | Nfia | NM_001122952.1 | chr4:97581942-98118876 |
| 14243 | Ndufb5 | NM_025316.2 | chr3:32737062-32751559 | 14340 | Nfib | NM_001113209.2 | chr4:82290172-82505779 |
| 14244 | Ndufb6 | NM_001033305.3 | chr4:40270590-40279421 | 14341 | Nfic | NM_008688.3 | chr10:81396190-81427173 |
| 14245 | Ndufb7 | NM_025843.3 | chr8:83566757-83571623 | 14342 | Nfil3 | NM_017373.3 | chr13:52967208-52981039 |
| 14246 | Ndufb8 | NM_026061.2 | chr19:44550253-44555415 | 14343 | Nfix | NM_001081981.2 | chr8:84704711-84774369 |
| 14247 | Ndufb9 | NM_023172.3 | chr15:58933809-58939489 | 14344 | Nfkb1 | NM_008689.2 | chr3:135584654-135691547 |
| 14248 | Ndufc1 | NM_025523.1 | chr3:51405478-51408955 | 14345 | Nfkb2 | NM_001177369.1 | chr19:46304736-46312090 |
| 14249 | Ndufc2 | NM_024220.2 | chr7:97400002-97407800 | 14346 | Nfkbia | NM_010907.2 | chr12:55489408-55492647 |
| 14250 | Ndufs1 | NM_001160038.1 | chr1:63143591-63176822 | 14347 | Nfkbib | NM_010908.5 | chr7:28758250-28766644 |
| 14251 | Ndufs2 | NM_153064.5 | chr1:171234852-171247122 | 14348 | Nfkbid | NM_172142.3 | chr7:30423303-30428746 |
| 14252 | Ndufs3 | NM_026688.2 | chr2:90894635-90904721 | 14349 | Nfkbie | NM_008690.4 | chr17:45555699-45563168 |
| 14253 | Ndufs4 | NM_010887.2 | chr13:114287794-114388094 | 14350 | Nfkbil1 | NM_010909.4 | chr17:35220174-35235815 |
| 14254 | Ndufs5 | NM_001030274.1 | chr4:123712709-123718186 | 14351 | Nfkbiz | NM_001159394.1 | chr16:55811376-55822138 |
| 14255 | Ndufs6 | NM_010888.2 | chr13:73319875-73328482 | 14352 | Nfrkb | NM_172766.3 | chr9:31386191-31421334 |
| 14256 | Ndufs7 | NM_029272.3 | chr10:80249451-80256792 | 14353 | Nfs1 | NM_010911.2 | chr2:156123636-156144186 |
| 14257 | Ndufs8 | NM_001271443.1 | chr19:3908862-3912774 | 14354 | Nfu1 | NM_001170591.1 | chr6:87009835-87028461 |
| 14258 | Ndufv1 | NM_133666.3 | chr19:4007498-4012755 | 14355 | Nfx1 | NM_001290448.1 | chr4:40970905-41013873 |

Fig.21 - 75

| | | | | | | | |
|---|---|---|---|---|---|---|---|
| 14356 | Nfxl1 | NM_133921.2 | chr5:72513303-72559645 | 14453 | Nlrp4c | NM_031389.2 | chr7:6045160-6105149 |
| 14357 | Nfya | NM_001110832.1 | chr17:48386884-48409820 | 14454 | Nlrp4e | NM_001004194.2 | chr7:23301191-23362277 |
| 14358 | Nfyb | NM_010914.2 | chr10:82748699-82764141 | 14455 | Nlrp4f | NM_175290.4 | chr13:65177110-65205716 |
| 14359 | Nfyc | NM_001048168.2 | chr4:120757434-120831579 | 14456 | Nlrp4g | NM_001004145.2 | chr9:124348830-124354483 |
| 14360 | Ngb | NM_022414.2 | chr12:87097530-87102539 | 14457 | Nlrp5 | NM_001039143.2 | chr7:23385888-23441923 |
| 14361 | Ngdn | NM_026890.2 | chr14:55015453-55024137 | 14458 | Nlrp5-ps | NR_045119.1 | chr7:14561170-14623066 |
| 14362 | Ngef | NM_001111314.1 | chr1:87476828-87573870 | 14459 | Nlrp6 | NM_133946.2 | chr7:140920901-140929192 |
| 14363 | Ngf | NM_001112698.2 | chr3:102469918-102521013 | 14460 | Nlrp9a | NM_001048219.2 | chr7:26535022-26574146 |
| 14364 | Ngfr | NM_033217.3 | chr11:95568820-95587698 | 14461 | Nlrp9b | NM_194058.2 | chr7:20008022-20062938 |
| 14365 | Ngfrap1 | NM_001110233.1 | chrX:136270252-136271978 | 14462 | Nlrp9c | NM_001042612.1 | chr7:26364886-26394243 |
| 14366 | Ngly1 | NM_021504.3 | chr14:16249313-16311926 | 14463 | Nlrx1 | NM_001163742.1 | chr9:44252712-44268599 |
| 14367 | Ngp | NM_008694.2 | chr9:110419807-110423012 | 14464 | Nmb | NM_001291280.1 | chr7:80902226-80905062 |
| 14368 | Ngrn | NM_031375.4 | chr7:80261214-80265378 | 14465 | Nmbr | NM_008703.2 | chr10:14760288-14770556 |
| 14369 | Nhej1 | NM_029342.4 | chr1:74967345-75046639 | 14466 | Nmd3 | NM_133787.2 | chr3:69722054-69749046 |
| 14370 | Nhlh1 | NM_010916.2 | chr1:172052291-172057596 | 14467 | Nme1 | NM_008704.2 | chr11:93958924-93968521 |
| 14371 | Nhlh2 | NM_178777.3 | chr3:102010144-102015492 | 14468 | Nme2 | NM_001077529.2 | chr11:93949813-93955783 |
| 14372 | Nhlrc1 | NM_175340.4 | chr13:47012556-47014850 | 14469 | Nme3 | NM_019730.2 | chr17:24896499-24897529 |
| 14373 | Nhlrc2 | NM_025811.3 | chr19:56548260-56598846 | 14470 | Nme4 | NM_019731.1 | chr17:26091744-26095470 |
| 14374 | Nhlrc3 | NM_172501.2 | chr3:53451995-53463258 | 14471 | Nme5 | NM_080637.3 | chr18:34562640-34579106 |
| 14375 | Nhlrc4 | NM_001039038.2 | chr17:25942232-25944931 | 14472 | Nme6 | NM_018757.1 | chr9:109832793-109842961 |
| 14376 | Nhp2 | NM_026631.3 | chr11:51619772-51623714 | 14473 | Nme7 | NM_138314.4 | chr1:164307671-164437300 |
| 14377 | Nhp2l1 | NM_011482.4 | chr15:82041344-82047598 | 14474 | Nme8 | NM_001167909.1 | chr13:19645078-19697760 |
| 14378 | Nhs | NM_001081052.2 | chrX:161836429-162159441 | 14475 | Nme9 | NM_001165957.1 | chr9:99456242-99479899 |
| 14379 | Nhsl1 | NM_001163592.1 | chr10:18469980-18533891 | 14476 | Nmi | NM_001141948.1 | chr2:51948498-51973208 |
| 14380 | Nhsl2 | NM_001163610.1 | chrX:101849384-102092055 | 14477 | Nmnat1 | NM_133435.1 | chr4:149468786-149485142 |
| 14381 | Nicn1 | NM_025449.3 | chr9:108290456-108296496 | 14478 | Nmnat2 | NM_175460.3 | chr1:152955100-153119261 |
| 14382 | Nid1 | NM_010917.2 | chr13:13437601-13512275 | 14479 | Nmnat3 | NM_144533.2 | chr9:98296582-98411428 |
| 14383 | Nid2 | NM_008695.2 | chr14:19751256-19811787 | 14480 | Nmral1 | NM_001290761.1 | chr16:4711317-4719356 |
| 14384 | Nif3l1 | NM_022988.2 | chr1:58447632-58462276 | 14481 | Nmrk1 | NM_145497.2 | chr19:18632015-18652184 |
| 14385 | Nifk | NM_026472.4 | chr1:118321842-118333831 | 14482 | Nmrk2 | NM_027120.2 | chr10:81198169-81202037 |
| 14386 | Nim1k | NM_175538.3 | chr13:119710093-119755882 | 14483 | Nms | NM_001011684.2 | chr1:38939148-38950276 |
| 14387 | Nin | NM_001081453.1 | chr12:70011434-70102854 | 14484 | Nmt1 | NM_008707.3 | chr11:103028561-103066105 |
| 14388 | Ninj1 | NM_013610.2 | chr13:49187546-49196251 | 14485 | Nmt2 | NM_001290368.1 | chr2:3284211-3328877 |
| 14389 | Ninj2 | NM_016718.2 | chr6:120093379-120200338 | 14486 | Nmu | NM_019515.1 | chr5:76333494-76363777 |
| 14390 | Ninl | NM_207204.2 | chr2:150934518-151009398 | 14487 | Nmur1 | NM_010341.1 | chr1:86386324-86388141 |
| 14391 | Nip7 | NM_001164472.1 | chr8:107056876-107060928 | 14488 | Nmur2 | NM_153079.4 | chr11:56024989-56040987 |
| 14392 | Nipa1 | NM_153578.2 | chr7:55978483-56019573 | 14489 | Nnat | NM_001291128.1 | chr2:157560077-157562525 |
| 14393 | Nipa2 | NM_001256130.1 | chr7:55931265-55962493 | 14490 | Nnmt | NM_010924.3 | chr9:48591876-48605123 |
| 14394 | Nipal1 | NM_001081205.1 | chr5:72647795-72671078 | 14491 | Nnt | NM_008710.3 | chr13:119334316-119409011 |
| 14395 | Nipal2 | NM_145469.5 | chr15:34572798-34678706 | 14492 | Noa1 | NM_019836.3 | chr5:77294168-77310086 |
| 14396 | Nipal3 | NM_028995.3 | chr4:135448900-135494504 | 14493 | Nob1 | NM_026277.3 | chr8:107412488-107425038 |
| 14397 | Nipal4 | NM_172524.3 | chr11:46148154-46166359 | 14494 | Nobox | NM_130869.3 | chr6:43303673-43309554 |
| 14398 | Nipbl | NM_027707.3 | chr15:8289823-8444463 | 14495 | Noc2l | NM_021303.2 | chr4:156236009-156247616 |
| 14399 | Nipsnap1 | NM_008698.2 | chr11:4874002-4894200 | 14496 | Noc3l | NM_021315.2 | chr19:38788127-38819237 |
| 14400 | Nipsnap3a | NM_028529.1 | chr4:52989283-53000854 | 14497 | Noc4l | NM_153570.2 | chr5:110648418-110653382 |
| 14401 | Nipsnap3b | NM_025623.2 | chr4:53011923-53022059 | 14498 | Nod1 | NM_001171007.1 | chr6:54923941-54971661 |
| 14402 | Nisch | NM_022656.2 | chr14:31170927-31206826 | 14499 | Nod2 | NM_145857.2 | chr8:88647346-88688474 |
| 14403 | Nit1 | NM_001242580.1 | chr1:171342236-171345645 | 14500 | Nodal | NM_013611.4 | chr10:61417971-61425337 |
| 14404 | Nit2 | NM_023175.1 | chr16:57156664-57167332 | 14501 | Nog | NM_008711.2 | chr11:89300637-89302559 |
| 14405 | Nkain1 | NM_025998.3 | chr4:130530130-130574036 | 14502 | Nol10 | NM_001008421.1 | chr12:17348492-17430095 |
| 14406 | Nkain2 | NM_001013411.2 | chr10:31689318-32889915 | 14503 | Nol11 | NM_001161329.1 | chr11:107166660-107189381 |
| 14407 | Nkain3 | NM_001290410.1 | chr4:20118873-20778668 | 14504 | Nol12 | NM_133800.3 | chr15:78934932-78941910 |
| 14408 | Nkain4 | NM_001141933.1 | chr2:180934771-180954699 | 14505 | Nol3 | NM_030152.4 | chr8:105276446-105281939 |
| 14409 | Nkap | NM_025937.4 | chrX:37126762-37150746 | 14506 | Nol4 | NM_001161483.1 | chr18:22693154-23038663 |
| 14410 | Nkapl | NM_025719.3 | chr13:21467046-21468501 | 14507 | Nol6 | NM_139236.3 | chr4:41114426-41124339 |
| 14411 | Nkd1 | NM_001163660.1 | chr8:88527632-88594887 | 14508 | Nol7 | NM_023554.2 | chr13:43398375-43402858 |
| 14412 | Nkd2 | NM_028186.4 | chr13:73819327-73847631 | 14509 | Nol8 | NM_001271397.1 | chr13:49653077-49679016 |
| 14413 | Nkg7 | NM_024253.4 | chr7:43437137-43438246 | 14510 | Nol9 | NM_001159599.2 | chr4:152039320-152060038 |
| 14414 | Nkiras1 | NM_023526.4 | chr14:18271141-18284000 | 14511 | Nolc1 | NM_001039351.2 | chr19:46075846-46085543 |
| 14415 | Nkiras2 | NM_028024.2 | chr11:100622954-100627602 | 14512 | Nom1 | NM_001033457.2 | chr5:29434666-29452169 |
| 14416 | Nkpd1 | NM_027116.1 | chr7:19518730-19525050 | 14513 | Nomo1 | NM_153057.4 | chr7:46033695-46084212 |
| 14417 | Nkrf | NM_029891.2 | chrX:36887541-36902899 | 14514 | Nono | NM_001252518.1 | chrX:101429650-101448593 |
| 14418 | Nktr | NM_010918.2 | chr9:121719180-121756841 | 14515 | Nop10 | NM_025403.4 | chr2:112261925-112262898 |
| 14419 | Nkx1-1 | NM_011320.1 | chr5:33430733-33434090 | 14516 | Nop14 | NM_029278.2 | chr5:34638535-34660148 |
| 14420 | Nkx1-2 | NM_009123.2 | chr7:132596238-132599637 | 14517 | Nop16 | NM_178605.4 | chr13:54584190-54590074 |
| 14421 | Nkx2-1 | NM_001146198.1 | chr12:56531934-56535106 | 14518 | Nop2 | NM_138747.2 | chr6:125131882-125144753 |
| 14422 | Nkx2-2 | NM_001077632.1 | chr2:147177545-147186402 | 14519 | Nop56 | NM_024193.2 | chr2:130274411-130279313 |
| 14423 | Nkx2-2os | NR_030769.2 | chr2:147184082-147331681 | 14520 | Nop58 | NM_018868.2 | chr1:59685005-59711510 |
| 14424 | Nkx2-3 | NM_008699.2 | chr19:43612324-43615892 | 14521 | Nop9 | NM_026403.3 | chr14:55745692-55755634 |
| 14425 | Nkx2-4 | NM_023504.1 | chr2:147083875-147085345 | 14522 | Nos1 | NM_008712.3 | chr5:117866838-117958840 |
| 14426 | Nkx2-5 | NM_008700.1 | chr17:26838664-26841565 | 14523 | Nos1ap | NM_001109985.1 | chr1:170317495-170589849 |
| 14427 | Nkx2-6 | NM_010920.2 | chr14:69171801-69175540 | 14524 | Nos2 | NM_010927.4 | chr11:78920786-78960226 |
| 14428 | Nkx2-9 | NM_008701.2 | chr12:56611396-56613284 | 14525 | Nos3 | NM_008713.4 | chr5:24364818-24384474 |
| 14429 | Nkx3-1 | NM_010921.3 | chr14:69190691-69194658 | 14526 | Nosip | NM_001163684.1 | chr7:45062428-45078503 |
| 14430 | Nkx3-2 | NM_007524.3 | chr5:41761482-41764220 | 14527 | Nostrin | NM_181547.3 | chr2:69135799-69189329 |
| 14431 | Nkx6-1 | NM_144955.2 | chr5:101659196-101664711 | 14528 | Notch1 | NM_008714.3 | chr2:26457901-26503822 |
| 14432 | Nkx6-2 | NM_183248.3 | chr7:139581219-139582797 | 14529 | Notch2 | NM_010928.2 | chr3:98013537-98150367 |
| 14433 | Nkx6-3 | NM_029002.2 | chr8:23153270-23158948 | 14530 | Notch3 | NM_008716.2 | chr17:32120892-32166852 |
| 14434 | Nle1 | NM_145431.2 | chr11:82900767-82908395 | 14531 | Notch4 | NM_010929.2 | chr17:34564294-34588543 |
| 14435 | Nlgn1 | NM_001163387.1 | chr3:25431840-26153307 | 14532 | Noto | NM_001007472.2 | chr6:85423885-85428877 |
| 14436 | Nlgn2 | NM_198862.2 | chr11:69823122-69834849 | 14533 | Notum | NM_175263.4 | chr11:120653788-120660837 |
| 14437 | Nlgn3 | NM_172932.4 | chrX:101299178-101321350 | 14534 | Nov | NM_010930.4 | chr15:54745927-54753761 |
| 14438 | Nlk | NM_008702.3 | chr11:78567167-78697425 | 14535 | Nova1 | NM_021361.1 | chr12:46694516-46818775 |
| 14439 | Nln | NM_029447.2 | chr13:104023438-104109614 | 14536 | Nova2 | NM_001029877.3 | chr7:18925887-18965319 |
| 14440 | Nlrc3 | NM_001081280.1 | chr16:3946932-3976632 | 14537 | Nox1 | NM_172203.2 | chrX:134086420-134111854 |
| 14441 | Nlrc4 | NM_001033367.3 | chr17:74426294-74459108 | 14538 | Nox3 | NM_198958.2 | chr17:3635239-3696261 |
| 14442 | Nlrc5 | NM_001033207.3 | chr8:94472762-94527272 | 14539 | Nox4 | NM_001285833.1 | chr7:87246648-87398708 |
| 14443 | Nlrp10 | NM_175532.3 | chr7:108921852-108930158 | 14540 | Noxa1 | NM_001163626.1 | chr2:25085669-25095205 |
| 14444 | Nlrp12 | NM_001033431.1 | chr7:3221509-3249740 | 14541 | Noxo1 | NM_027988.4 | chr17:24696233-24700529 |
| 14445 | Nlrp14 | NM_001002894.2 | chr7:107166989-107198103 | 14542 | Noxred1 | NM_027744.1 | chr12:87221122-87238601 |
| 14446 | Nlrp1a | NM_001004142.2 | chr11:71091196-71144704 | 14543 | Npas1 | NM_008718.2 | chr7:16455720-16476780 |
| 14447 | Nlrp1b | NM_001040696.1 | chr11:71153101-71230733 | 14544 | Npas2 | NM_008719.2 | chr1:39194271-39363240 |
| 14448 | Nlrp1c-ps | NR_027858.1 | chr11:71242429-71285232 | 14545 | Npas3 | NM_013780.2 | chr12:53248676-54072175 |
| 14449 | Nlrp2 | NM_177690.3 | chr7:5298546-5351035 | 14546 | Npas4 | NM_153553.4 | chr19:4984354-4989971 |
| 14450 | Nlrp3 | NM_145827.3 | chr11:59542685-59566956 | 14547 | Npat | NM_001081152.1 | chr9:53537046-53575627 |
| 14451 | Nlrp4a | NM_172896.2 | chr7:26435112-26475458 | 14548 | Npb | NM_153288.3 | chr11:120608476-120609100 |
| 14452 | Nlrp4b | NM_172481.2 | chr7:10687792-10730158 | 14549 | Npbwr1 | NM_010342.1 | chr1:5913706-5917398 |

Fig.21 - 76

| | | | | | | | |
|---|---|---|---|---|---|---|---|
| 14550 | Npc1 | NM_008720.2 | chr18:12189693-12236386 | 14647 | Nrn1 | NM_153529.2 | chr13:36725621-36734477 |
| 14551 | Npc1l1 | NM_207242.2 | chr11:6211010-6230245 | 14648 | Nrn1l | NM_175024.4 | chr8:105893566-105895023 |
| 14552 | Npc2 | NM_023409.4 | chr12:84754558-84773112 | 14649 | Nron | NR_045729.1 | chr2:33805815-33813591 |
| 14553 | Npcd | NM_001013360.2 | chr15:79786350-79834333 | 14650 | Nrp | NM_001013372.2 | chr12:87442847-87444017 |
| 14554 | Npdc1 | NM_008721.4 | chr2:25403049-25409494 | 14651 | Nrp1 | NM_008737.2 | chr8:128359072-128505475 |
| 14555 | Npepl1 | NM_213733.2 | chr2:174110350-174122702 | 14652 | Nrp2 | NM_001077403.1 | chr1:62703316-62818692 |
| 14556 | Npepps | NM_008942.2 | chr11:97205855-97280576 | 14653 | Nrros | NM_146069.4 | chr16:32142824-32165476 |
| 14557 | Npff | NM_018787.1 | chr15:102523838-102524621 | 14654 | Nrsn1 | NM_009513.2 | chr13:25252038-25269996 |
| 14558 | Npffr1 | NM_001177511.1 | chr10:61595483-61626385 | 14655 | Nrsn2 | NM_001009948.1 | chr2:152368757-152376566 |
| 14559 | Npffr2 | NM_133192.3 | chr5:89527428-89583740 | 14656 | Nrtn | NM_008738.2 | chr17:56751324-56757530 |
| 14560 | Nphp1 | NM_001291012.1 | chr2:127740731-127788891 | 14657 | Nrxn1 | NM_020252.3 | chr17:90033643-91092802 |
| 14561 | Nphp3 | NM_028721.3 | chr9:104002543-104043811 | 14658 | Nrxn2 | NM_001205234.1 | chr19:6418737-6533217 |
| 14562 | Nphp4 | NM_153424.2 | chr4:152478141-152563184 | 14659 | Nrxn3 | NM_001198587.3 | chr12:88794503-90334933 |
| 14563 | Nphs1 | NM_019459.2 | chr7:30460057-30488609 | 14660 | Nsa2 | NM_021552.5 | chr13:97129426-97137926 |
| 14564 | Nphs1os | NR_004443.1 | chr7:30462163-30465591 | 14661 | Nsd1 | NM_008739.3 | chr13:55209781-55318325 |
| 14565 | Nphs2 | NM_130456.4 | chr1:156310718-156328035 | 14662 | Nsdhl | NM_010941.3 | chrX:72918520-72958528 |
| 14566 | Npl | NM_028749.1 | chr1:153503015-153549714 | 14663 | Nsf | NM_008740.4 | chr11:103821782-103954056 |
| 14567 | Nploc4 | NM_001195023.1 | chr11:120379797-120437700 | 14664 | Nsfl1c | NM_001291074.1 | chr2:151494181-151511310 |
| 14568 | Npm1 | NM_001252260.1 | chr11:33152497-33163206 | 14665 | Nsg1 | NM_010942.3 | chr5:38137192-38159467 |
| 14569 | Npm2 | NM_181345.3 | chr14:70647301-70653084 | 14666 | Nsg2 | NM_001290680.1 | chr11:32000699-32059211 |
| 14570 | Npm3 | NM_008723.1 | chr19:45747733-45749563 | 14667 | Nsl1 | NM_198654.3 | chr1:191063020-191084558 |
| 14571 | Npm3-ps1 | NR_002702.1 | chr6:85076140-85077126 | 14668 | Nsmaf | NM_010945.2 | chr4:6396207-6454271 |
| 14572 | Npnt | NM_001029836.2 | chr3:132881744-132950291 | 14669 | Nsmce1 | NM_026330.3 | chr7:125467639-125491542 |
| 14573 | Nppa | NM_008725.3 | chr4:148000745-148002067 | 14670 | Nsmce2 | NM_001164604.1 | chr15:59374197-59539666 |
| 14574 | Nppb | NM_001287348.1 | chr4:147985787-147987205 | 14671 | Nsmce4a | NM_001162855.1 | chr7:130532525-130547381 |
| 14575 | Nppc | NM_010933.5 | chr1:86666292-86670573 | 14672 | Nsmf | NM_001039386.1 | chr2:25054378-25062881 |
| 14576 | Npr1 | NM_008727.5 | chr3:90450591-90465866 | 14673 | Nsun2 | NM_145354.5 | chr13:69612015-69635779 |
| 14577 | Npr2 | NM_173788.3 | chr4:43631934-43651240 | 14674 | Nsun3 | NM_178925.3 | chr16:62734851-62786716 |
| 14578 | Npr3 | NM_001039181.1 | chr15:11839896-11905674 | 14675 | Nsun4 | NM_028142.4 | chr4:116031769-116053876 |
| 14579 | Nprl2 | NM_018879.2 | chr9:107542208-107545706 | 14676 | Nsun5 | NM_145414.2 | chr5:135369952-135376797 |
| 14580 | Nprl3 | NM_001284359.1 | chr11:32231962-32267707 | 14677 | Nsun6 | NM_001165941.1 | chr2:14995130-15054872 |
| 14581 | Nps | NM_001163611.1 | chr7:135268618-135272942 | 14678 | Nsun7 | NM_027602.2 | chr5:66260840-66298028 |
| 14582 | Npsr1 | NM_175678.3 | chr9:24098017-24316398 | 14679 | Nt5c | NM_015807.1 | chr11:115490425-115491814 |
| 14583 | Nptn | NM_009145.2 | chr9:58582239-58652879 | 14680 | Nt5c1a | NM_001085502.1 | chr4:123201552-123216207 |
| 14584 | Nptx1 | NM_008730.2 | chr11:119538718-119547820 | 14681 | Nt5c1b | NM_027588.3 | chr12:10369970-10390174 |
| 14585 | Nptx2 | NM_016789.3 | chr5:144545886-144557478 | 14682 | Nt5c2 | NM_001164363.1 | chr19:46886830-47015189 |
| 14586 | Nptxr | NM_030689.4 | chr15:79786350-79804709 | 14683 | Nt5c3 | NM_001252374.1 | chr6:56882401-56901886 |
| 14587 | Npvf | NM_021892.1 | chr6:50650870-50654393 | 14684 | Nt5c3b | NM_001102650.1 | chr11:100429351-100441089 |
| 14588 | Npw | NM_001099664.1 | chr17:24657329-24658425 | 14685 | Nt5dc1 | NM_176968.4 | chr10:34303611-34418528 |
| 14589 | Npy | NM_023456.3 | chr6:49822728-49829505 | 14686 | Nt5dc2 | NM_027289.1 | chr14:31134852-31139124 |
| 14590 | Npy1r | NM_010934.4 | chr8:66697421-66706798 | 14687 | Nt5dc3 | NM_175331.3 | chr10:86779004-86838389 |
| 14591 | Npy2r | NM_001205099.1 | chr3:82538382-82548085 | 14688 | Nt5e | NM_011851.4 | chr9:88327608-88372089 |
| 14592 | Npy4r | NM_008919.4 | chr14:34145645-34152419 | 14689 | Nt5m | NM_134029.2 | chr11:59848072-59876533 |
| 14593 | Npy5r | NM_016708.3 | chr8:66679964-66688094 | 14690 | Ntan1 | NM_010946.3 | chr16:13819276-13835451 |
| 14594 | Npy6r | NM_010935.3 | chr18:44270126-44277700 | 14691 | Ntf3 | NM_001164034.1 | chr6:126101411-126166744 |
| 14595 | Nqo1 | NM_008706.5 | chr8:107388224-107403205 | 14692 | Ntf5 | NM_198190.1 | chr7:45413694-45417179 |
| 14596 | Nqo2 | NM_001163239.1 | chr13:33964658-33988465 | 14693 | Nthl1 | NM_008743.2 | chr17:24632681-24638838 |
| 14597 | Nr0b1 | NM_007430.5 | chrX:86191774-86195946 | 14694 | Ntm | NM_172290.3 | chr9:28995963-29963129 |
| 14598 | Nr0b2 | NM_011850.3 | chr4:133553375-133556686 | 14695 | Ntmt1 | NM_170592.2 | chr2:30807976-30823014 |
| 14599 | Nr1d1 | NM_145434.4 | chr11:98767931-98775377 | 14696 | Ntn1 | NM_008744.2 | chr11:68209363-68386826 |
| 14600 | Nr1d2 | NM_011584.4 | chr14:18204055-18239106 | 14697 | Ntn3 | NM_010947.3 | chr17:24203842-24209387 |
| 14601 | Nr1h2 | NM_001285517.1 | chr7:44549615-44553965 | 14698 | Ntn4 | NM_021320.3 | chr10:93641048-93745972 |
| 14602 | Nr1h3 | NM_001177730.1 | chr2:91184060-91195116 | 14699 | Ntn5 | NM_001033356.3 | chr7:45684021-45694556 |
| 14603 | Nr1h4 | NM_001163504.1 | chr10:89454233-89533622 | 14700 | Ntng1 | NM_001163348.1 | chr3:109780049-110143472 |
| 14604 | Nr1h5 | NM_198658.2 | chr3:102939657-102964133 | 14701 | Ntng2 | NM_133500.2 | chr2:29194725-29248099 |
| 14605 | Nr1i2 | NM_001098404.1 | chr16:38248348-38294824 | 14702 | Ntpcr | NM_025636.5 | chr8:125734202-125748235 |
| 14606 | Nr1i3 | NM_001243062.1 | chr1:171213969-171218845 | 14703 | Ntrk1 | NM_001033124.1 | chr3:87778243-87795162 |
| 14607 | Nr2c1 | NM_011629.3 | chr10:94147930-94197214 | 14704 | Ntrk2 | NM_001025074.2 | chr13:58807696-59133970 |
| 14608 | Nr2c2 | NM_011630.3 | chr6:92091417-92173058 | 14705 | Ntrk3 | NM_008746.5 | chr7:78192113-78577838 |
| 14609 | Nr2c2ap | NM_001025586.2 | chr8:70131326-70133751 | 14706 | Nts | NM_024435.2 | chr10:102481755-102490418 |
| 14610 | Nr2e1 | NM_152229.2 | chr10:42561970-42583588 | 14707 | Ntsr1 | NM_018766.2 | chr2:180499975-180544979 |
| 14611 | Nr2e3 | NM_013708.1 | chr9:59942770-59950079 | 14708 | Ntsr2 | NM_008747.2 | chr12:16653469-16660236 |
| 14612 | Nr2f1 | NM_010151.2 | chr13:78188972-78198982 | 14709 | Nuak1 | NM_001004363.1 | chr10:84371318-84440471 |
| 14613 | Nr2f2 | NM_009697.3 | chr7:70351949-70360593 | 14710 | Nuak2 | NM_001195025.1 | chr1:132316124-132333488 |
| 14614 | Nr2f6 | NM_010150.2 | chr8:71374118-71381952 | 14711 | Nub1 | NM_016736.3 | chr5:24685814-24710378 |
| 14615 | Nr3c1 | NM_008173.3 | chr18:39410544-39487245 | 14712 | Nubp1 | NM_011955.1 | chr16:10411937-10424425 |
| 14616 | Nr3c2 | NM_001083906.1 | chr8:76902507-77243639 | 14713 | Nubp2 | NM_011956.3 | chr17:24882610-24886350 |
| 14617 | Nr4a1 | NM_010444.2 | chr15:101266845-101274794 | 14714 | Nubpl | NM_029760.2 | chr12:52097745-52310959 |
| 14618 | Nr4a2 | NM_001139509.1 | chr2:57107225-57124003 | 14715 | Nucb1 | NM_001163662.1 | chr7:45492673-45510408 |
| 14619 | Nr4a3 | NM_015743.3 | chr4:48045304-48086446 | 14716 | Nucb2 | NM_001130479.2 | chr7:116504368-116540588 |
| 14620 | Nr5a1 | NM_139051.3 | chr2:38692659-38714542 | 14717 | Nucks1 | NM_001145804.1 | chr1:131910457-131936321 |
| 14621 | Nr5a2 | NM_001159769.2 | chr1:136843583-136953630 | 14718 | Nudc | NM_010948.3 | chr4:133532541-133546027 |
| 14622 | Nr6a1 | NM_001159548.1 | chr2:38723373-38784515 | 14719 | Nudcd1 | NM_001113554.1 | chr15:44375226-44428307 |
| 14623 | Nradd | NM_026012.2 | chr9:110621134-110624393 | 14720 | Nudcd2 | NM_001290697.1 | chr11:40733660-40740046 |
| 14624 | Nrap | NM_001286552.1 | chr19:56320040-56390038 | 14721 | Nudcd3 | NM_173748.4 | chr11:6105691-6200451 |
| 14625 | Nrarp | NM_025980.2 | chr2:25180757-25183332 | 14722 | Nudt1 | NM_008637.1 | chr5:140331921-140338135 |
| 14626 | Nras | NM_010937.2 | chr3:103058284-103067914 | 14723 | Nudt10 | NM_001031664.1 | chrX:6168695-6172991 |
| 14627 | Nrbf2 | NM_001036293.2 | chr10:67266688-67285281 | 14724 | Nudt11 | NM_021431.2 | chrX:6047506-6054751 |
| 14628 | Nrbp1 | NM_147201.2 | chr5:31240917-31251562 | 14725 | Nudt12 | NM_026497.2 | chr17:59001400-59013322 |
| 14629 | Nrbp2 | NM_144847.1 | chr15:76085593-76090013 | 14726 | Nudt13 | NM_026341.2 | chr14:20294689-20317575 |
| 14630 | Nrcam | NM_001146031.1 | chr12:44328884-44601846 | 14727 | Nudt14 | NM_025399.4 | chr12:112934732-112942118 |
| 14631 | Nrd1 | NM_146150.2 | chr4:109000804-109061771 | 14728 | Nudt15 | NM_172527.2 | chr14:73519863-73548242 |
| 14632 | Nrde2 | NM_001290303.1 | chr12:100125449-100159653 | 14729 | Nudt16 | NM_029385.2 | chr9:105129337-105131805 |
| 14633 | Nrep | NM_001109988.1 | chr18:33437018-33464029 | 14730 | Nudt16l1 | NM_025839.4 | chr16:4939110-4941020 |
| 14634 | Nrf1 | NM_001164226.1 | chr6:30047987-30153458 | 14731 | Nudt17 | NM_001162925.1 | chr3:96705891-96708560 |
| 14635 | Nrg1 | NM_178591.2 | chr8:31818027-31918203 | 14732 | Nudt18 | NM_153136.4 | chr14:70577846-70582571 |
| 14636 | Nrg2 | NM_001167891.1 | chr18:36017657-36197160 | 14733 | Nudt19 | NM_033080.2 | chr7:35547184-35555928 |
| 14637 | Nrg3 | NM_001190187.1 | chr14:38368950-39473088 | 14734 | Nudt2 | NM_025539.2 | chr4:41465147-41480926 |
| 14638 | Nrg3os | NR_045713.1 | chr14:38898301-38958794 | 14735 | Nudt21 | NM_026623.3 | chr8:94019402-94037039 |
| 14639 | Nrg4 | NM_032002.2 | chr9:55220221-55283625 | 14736 | Nudt22 | NM_026675.2 | chr19:6993018-6996037 |
| 14640 | Nrgn | NM_022029.2 | chr9:37544492-37552745 | 14737 | Nudt3 | NM_001291046.1 | chr17:27579381-27622964 |
| 14641 | Nrip1 | NM_173440.2 | chr16:76290861-76373049 | 14738 | Nudt4 | NM_027722.4 | chr10:95547006-95564167 |
| 14642 | Nrip2 | NM_001162858.1 | chr6:128399765-128408935 | 14739 | Nudt5 | NM_016918.3 | chr2:5845033-5868736 |
| 14643 | Nrip3 | NM_020610.1 | chr7:109758055-109781545 | 14740 | Nudt6 | NM_001291044.1 | chr3:37404981-37419596 |
| 14644 | Nrk | NM_013724.2 | chrX:138914429-139009090 | 14741 | Nudt7 | NM_001290180.1 | chr8:114133573-114152312 |
| 14645 | Nrl | NM_001136074.2 | chr14:55518977-55524981 | 14742 | Nudt8 | NM_025529.3 | chr19:4000579-4002102 |
| 14646 | Nrm | NM_134122.2 | chr17:35861317-35865400 | 14743 | Nudt9 | NM_028794.4 | chr5:104046863-104065378 |

Fig.21 - 77

| | | | | | | | |
|---|---|---|---|---|---|---|---|
| 14744 | Nuf2 | NM_023284.3 | chr1:169497933-169531464 | 14841 | Ociad2 | NM_026950.4 | chr5:73322197-73338947 |
| 14745 | Nufip1 | NM_013745.5 | chr14:76110890-76137379 | 14842 | Ocln | NM_008756.2 | chr13:100497366-100552498 |
| 14746 | Nufip2 | NM_001024205.2 | chr11:77686138-77717966 | 14843 | Ocm | NM_033039.3 | chr5:144019806-144050609 |
| 14747 | Nuggc | NM_001195674.2 | chr14:65605266-65648443 | 14844 | Ocrl | NM_177215.3 | chrX:47912455-47965866 |
| 14748 | Numa1 | NM_133947.3 | chr7:101969842-102014959 | 14845 | Ocstamp | NM_029021.1 | chr2:165395449-165400394 |
| 14749 | Numb | NM_001136075.2 | chr12:83794033-83842358 | 14846 | Odam | NM_027128.2 | chr5:87885694-87892742 |
| 14750 | Numbl | NM_010950.2 | chr7:27258760-27282150 | 14847 | Odc1 | NM_013614.2 | chr12:17544872-17551502 |
| 14751 | Nup107 | NM_134010.2 | chr10:117750642-117792705 | 14848 | Odf1 | NM_008757.3 | chr15:38219202-38226735 |
| 14752 | Nup133 | NM_172288.2 | chr8:123897122-123949265 | 14849 | Odf2 | NM_001113213.1 | chr2:29889719-29931746 |
| 14753 | Nup153 | NM_175749.2 | chr13:46679901-46727849 | 14850 | Odf2l | NM_001162538.1 | chr3:145118588-145153915 |
| 14754 | Nup155 | NM_133227.3 | chr15:8109312-8159859 | 14851 | Odf3 | NM_027019.3 | chr7:140847915-140850925 |
| 14755 | Nup160 | NM_021512.2 | chr2:90677214-90736328 | 14852 | Odf3b | NM_001013022.1 | chr15:89377448-89379254 |
| 14756 | Nup188 | NM_198304.2 | chr2:30286432-30344262 | 14853 | Odf3l1 | NM_198673.2 | chr9:56848658-56851963 |
| 14757 | Nup205 | NM_027513.1 | chr6:35177615-35247598 | 14854 | Odf3l2 | NM_001033473.2 | chr10:79639525-79645738 |
| 14758 | Nup210 | NM_018815.2 | chr6:91013066-91116826 | 14855 | Odf4 | NM_145746.2 | chr11:68921834-68927081 |
| 14759 | Nup210l | NM_029937.1 | chr3:90104131-90212017 | 14856 | Ofcc1 | NM_172143.2 | chr13:40001881-40288011 |
| 14760 | Nup214 | NM_172268.2 | chr2:31974449-32053975 | 14857 | Ofd1 | NM_177429.3 | chrX:166390032-166440704 |
| 14761 | Nup35 | NM_001190179.1 | chr2:80639263-80660071 | 14858 | Ogdh | NM_001252282.1 | chr11:6291596-6359094 |
| 14762 | Nup37 | NM_027191.2 | chr10:88146991-88178395 | 14859 | Ogdhl | NM_001081130.1 | chr14:32322018-32347820 |
| 14763 | Nup43 | NM_145706.2 | chr10:7667503-7678886 | 14860 | Ogfod1 | NM_001093757.1 | chr8:94037197-94067922 |
| 14764 | Nup50 | NM_016714.2 | chr15:84923427-84942963 | 14861 | Ogfod2 | NM_025671.2 | chr5:124112337-124115476 |
| 14765 | Nup54 | NM_183392.2 | chr5:92415539-92435199 | 14862 | Ogfod3 | NM_025402.2 | chr11:121177592-121204648 |
| 14766 | Nup62 | NM_053074.2 | chr7:44816087-44831836 | 14863 | Ogfr | NM_031373.3 | chr2:180589406-180595837 |
| 14767 | Nup62cl | NM_001081668.1 | chrX:140007672-140062568 | 14864 | Ogfrl1 | NM_001081079.1 | chr1:23366423-23383175 |
| 14768 | Nup62-il4i1 | NM_001171024.1 | chr7:44816369-44840803 | 14865 | Ogg1 | NM_010957.4 | chr6:113326975-113334186 |
| 14769 | Nup85 | NM_001002929.4 | chr11:115564443-115583924 | 14866 | Ogn | NM_008760.4 | chr13:49608070-49624500 |
| 14770 | Nup88 | NM_001083331.2 | chr11:70943057-70969973 | 14867 | Ogt | NM_001290535.1 | chrX:101640217-101684351 |
| 14771 | Nup93 | NM_172410.2 | chr8:94214600-94315066 | 14868 | Oip5 | NM_001042653.1 | chr2:119609531-119618505 |
| 14772 | Nup98 | NM_001287164.1 | chr7:102119399-102210166 | 14869 | Oit1 | NM_146050.2 | chr14:8348947-8378763 |
| 14773 | Nupl1 | NM_170591.1 | chr14:60219467-60251378 | 14870 | Oit3 | NM_010959.2 | chr10:59422959-59441779 |
| 14774 | Nupl2 | NM_153092.4 | chr5:24164962-24184008 | 14871 | Ola1 | NM_025942.2 | chr2:73092800-73214447 |
| 14775 | Nupr1 | NM_019738.1 | chr7:126623245-126625470 | 14872 | Olah | NM_145921.1 | chr2:3341987-3366569 |
| 14776 | Nupr1l | NM_026916.3 | chr5:129908539-129911281 | 14873 | Olfm1 | NM_001038612.1 | chr2:28205688-28214431 |
| 14777 | Nus1 | NM_030250.2 | chr10:52417546-52440192 | 14874 | Olfm2 | NM_173777.3 | chr9:20667985-20728214 |
| 14778 | Nusap1 | NM_001042652.1 | chr2:119618297-119650160 | 14875 | Olfm3 | NM_001286750.1 | chr3:114904634-115125764 |
| 14779 | Nutf2 | NM_026532.3 | chr8:105860633-105880401 | 14876 | Olfm4 | NM_001030294.1 | chr14:80000301-80021930 |
| 14780 | Nutf2-ps1 | NR_033574.1 | chr8:105860659-105879337 | 14877 | Olfml1 | NM_172907.3 | chr7:107567432-107591365 |
| 14781 | Nutm1 | NM_172521.1 | chr2:112247947-112259291 | 14878 | Olfml2a | NM_172854.2 | chr2:38931979-38960585 |
| 14782 | Nvl | NM_026171.2 | chr1:181087137-181144204 | 14879 | Olfml2b | NM_177068.4 | chr1:170644531-170682789 |
| 14783 | Nwd1 | NM_176940.5 | chr8:72646710-72714748 | 14880 | Olfml3 | NM_133859.2 | chr3:103735393-103738001 |
| 14784 | Nwd2 | NM_177006.3 | chr5:63649102-63810543 | 14881 | Olfr1 | NM_146921.2 | chr11:73395075-73399495 |
| 14785 | Nxf1 | NM_001276704.1 | chr19:8757116-8770910 | 14882 | Olfr10 | NM_206822.1 | chr11:49317547-49318483 |
| 14786 | Nxf2 | NM_001289735.1 | chrX:134944525-134964754 | 14883 | Olfr100 | NM_207673.1 | chr17:37313455-37314382 |
| 14787 | Nxf3 | NM_001024141.4 | chrX:136072098-136085255 | 14884 | Olfr1000 | NM_001011695.1 | chr2:85607963-85608908 |
| 14788 | Nxf7 | NM_130888.1 | chrX:135579786-135593855 | 14885 | Olfr1002 | NM_146573.2 | chr2:85647362-85648319 |
| 14789 | Nxn | NM_008750.5 | chr11:76257225-76399141 | 14886 | Olfr1006 | NM_146570.2 | chr2:85674210-85678741 |
| 14790 | Nxnl1 | NM_145598.2 | chr8:71560554-71566649 | 14887 | Olfr1008 | NM_146866.1 | chr2:85689430-85690372 |
| 14791 | Nxnl2 | NM_029173.4 | chr13:51171024-51175187 | 14888 | Olfr1009 | NM_146572.2 | chr2:85721406-85722351 |
| 14792 | Nxpe2 | NM_030069.3 | chr9:48318003-48340898 | 14889 | Olfr101 | NM_146834.1 | chr17:37299493-37300420 |
| 14793 | Nxpe3 | NM_001134457.1 | chr16:55839952-55895279 | 14890 | Olfr1010 | NM_207149.2 | chr2:85753362-85754306 |
| 14794 | Nxpe4 | NM_172921.3 | chr9:48362040-48400025 | 14891 | Olfr1012 | NM_146568.2 | chr2:85759438-85760374 |
| 14795 | Nxpe5 | NM_001013773.3 | chr5:138225903-138251875 | 14892 | Olfr1013 | NM_146762.2 | chr2:85769802-85770720 |
| 14796 | Nxph1 | NM_008751.3 | chr6:8950018-9248578 | 14893 | Olfr1014 | NM_146569.2 | chr2:85776585-85777503 |
| 14797 | Nxph2 | NM_008752 | chr2:23321245-23401973 | 14894 | Olfr1015 | NM_146571.2 | chr2:85785473-85786489 |
| 14798 | Nxph3 | NM_130858 | chr11:95509845-95514565 | 14895 | Olfr1016 | NM_001011758.2 | chr2:85799338-85800268 |
| 14799 | Nxph4 | NM_183297 | chr10:127525472-127534559 | 14896 | Olfr1018 | NM_146586.2 | chr2:85822972-85823908 |
| 14800 | Nxt1 | NM_001110159.1 | chr2:148672640-148676026 | 14897 | Olfr1019 | NM_147015.1 | chr2:85840856-85841789 |
| 14801 | Nxt2 | NM_001161430.2 | chrX:142227936-142239700 | 14898 | Olfr102 | NM_001011721.2 | chr17:37313410-37314437 |
| 14802 | Nyap1 | NM_175521.3 | chr5:137730962-137739998 | 14899 | Olfr1020 | NM_146580.2 | chr2:85849419-85850498 |
| 14803 | Nyap2 | NM_172849.3 | chr1:81077316-81271651 | 14900 | Olfr1022 | NM_146589.2 | chr2:85868593-85869541 |
| 14804 | Nynrin | NM_001040072.1 | chr14:55854114-55874736 | 14901 | Olfr1023 | NM_146587.2 | chr2:85886801-85887737 |
| 14805 | Nyx | NM_173415.4 | chrX:13467671-13489313 | 14902 | Olfr1024 | NM_001005230.2 | chr2:85904068-85905052 |
| 14806 | Oacyl | NM_177028.3 | chr18:65698267-65751537 | 14903 | Olfr1026 | NM_146584.2 | chr2:85923269-85924193 |
| 14807 | Oaf | NM_178644.3 | chr9:43221277-43239816 | 14904 | Olfr1028 | NM_001011774.2 | chr2:85951064-85952039 |
| 14808 | Oard1 | NM_001289490.1 | chr17:48409999-48417270 | 14905 | Olfr1029 | NM_001011852.2 | chr2:85975212-85976273 |
| 14809 | Oas1a | NM_145211.2 | chr5:120896256-120907525 | 14906 | Olfr103 | NM_146833.1 | chr17:37336288-37337230 |
| 14810 | Oas1b | NM_001083925.1 | chr5:120812637-120824160 | 14907 | Olfr1030 | NM_146588.2 | chr2:85979311-85984798 |
| 14811 | Oas1c | NM_033541.4 | chr5:120800198-120812514 | 14908 | Olfr1031 | NM_001011759.2 | chr2:85991818-85992829 |
| 14812 | Oas1d | NM_133893.3 | chr5:120914817-120921647 | 14909 | Olfr1032 | NM_146579.2 | chr2:86007777-86008710 |
| 14813 | Oas1e | NM_145210.2 | chr5:120786311-120795530 | 14910 | Olfr1033 | NM_146578.2 | chr2:86020639-86044808 |
| 14814 | Oas1f | NM_145153.3 | chr5:120847366-120857986 | 14911 | Olfr1034 | NM_001011872.2 | chr2:86046443-86047466 |
| 14815 | Oas1g | NM_011852.3 | chr5:120876141-120887613 | 14912 | Olfr1036 | NM_207142.2 | chr2:86074703-86075718 |
| 14816 | Oas1h | NM_001159934.1 | chr5:120861421-120873505 | 14913 | Olfr1037 | NM_001011532.2 | chr2:86084729-86085854 |
| 14817 | Oas2 | NM_145227.3 | chr5:120730332-120749848 | 14914 | Olfr1038-ps | NM_147013.2 | chr2:86120223-86123037 |
| 14818 | Oas3 | NM_145226.2 | chr5:120753097-120777659 | 14915 | Olfr1039 | NM_001011784.2 | chr2:86130701-86131661 |
| 14819 | Oasl1 | NM_145209.3 | chr5:114923239-114937911 | 14916 | Olfr1040 | NM_207561.2 | chr2:86145790-86146732 |
| 14820 | Oasl2 | NM_011854.2 | chr5:114896933-114912245 | 14917 | Olfr1042 | NM_001011777.2 | chr2:86159344-86160441 |
| 14821 | Oat | NM_016978.2 | chr7:132557474-132576398 | 14918 | Olfr1043 | NM_146577.2 | chr2:86162002-86162947 |
| 14822 | Oaz1 | NM_008753.4 | chr10:80826655-80829290 | 14919 | Olfr1044 | NM_147011.1 | chr2:86170870-86171815 |
| 14823 | Oaz1-ps | NR_027656.1 | chr10:80826695-80829217 | 14920 | Olfr1045 | NM_147017.2 | chr2:86197802-86198750 |
| 14824 | Oaz2 | NM_010952.3 | chr9:65676547-65690300 | 14921 | Olfr1046 | NM_146582.2 | chr2:86216757-86217708 |
| 14825 | Oaz3 | NM_016901.3 | chr3:94433387-94436651 | 14922 | Olfr1047 | NM_147012.1 | chr2:86228009-86228969 |
| 14826 | Obfc1 | NM_175360.2 | chr19:47501047-47537020 | 14923 | Olfr1048 | NM_147014.1 | chr2:86235870-86236833 |
| 14827 | Obox1 | NM_027802.2 | chr7:15547256-15556846 | 14924 | Olfr1049 | NM_147016.2 | chr2:86254764-86255691 |
| 14828 | Obox2 | NM_145708.2 | chr7:15388850-15398543 | 14925 | Olfr1051 | NM_207562.1 | chr2:86275558-86276485 |
| 14829 | Obox3 | NM_145707.3 | chr7:15625305-15639777 | 14926 | Olfr1052 | NM_147010.2 | chr2:86297817-86298756 |
| 14830 | Obox5 | NM_145709.2 | chr7:15750369-15759274 | 14927 | Olfr1053 | NM_001177857.1 | chr2:86314342-86315285 |
| 14831 | Obox6 | NM_145710.2 | chr7:15833249-15839679 | 14928 | Olfr1054 | NM_147019.1 | chr2:86332415-86333354 |
| 14832 | Obp1a | NM_008754.2 | chrX:78085504-78091374 | 14929 | Olfr1055 | NM_147021.1 | chr2:86346825-86347764 |
| 14833 | Obp2a | NM_153558.1 | chr2:25700073-25703326 | 14930 | Olfr1056 | NM_147018.2 | chr2:86355358-86356437 |
| 14834 | Obp2b | NM_001099301.1 | chr2:25737008-25740097 | 14931 | Olfr1057 | NM_207563.2 | chr2:86374462-86375410 |
| 14835 | Obscn | NM_001171512.2 | chr11:58994255-59136375 | 14932 | Olfr1058 | NM_146391.2 | chr2:86385465-86386416 |
| 14836 | Obsl1 | NM_178884.5 | chr1:75485824-75506452 | 14933 | Olfr1061 | NM_207134.1 | chr2:86413108-86414050 |
| 14837 | Oc90 | NM_010953.2 | chr15:65876052-65912287 | 14934 | Olfr1062 | NM_147078.2 | chr2:86422718-86423674 |
| 14838 | Oca2 | NM_021879.2 | chr7:56239770-56536517 | 14935 | Olfr1065 | NM_146408.2 | chr2:86445012-86446004 |
| 14839 | Ocel1 | NM_029865.2 | chr8:71371297-71373689 | 14936 | Olfr1066 | NM_001011735.2 | chr2:86455327-86456269 |
| 14840 | Ociad1 | NM_001159887.1 | chr5:73292793-73314077 | 14937 | Olfr107 | NM_146511.2 | chr17:37405476-37406509 |

Fig.21 - 78

| | | | | | | | |
|---|---|---|---|---|---|---|---|
| 14938 | Olfr1076 | NM_146406.2 | chr2:86508460-86509402 | 15035 | Olfr1195 | NM_146753.2 | chr2:88682803-88683730 |
| 14939 | Olfr1077-ps1 | NR_033507.1 | chr2:86525661-86528427 | 15036 | Olfr1196 | NM_146464.2 | chr2:88700382-88701327 |
| 14940 | Olfr1079 | NM_146407.1 | chr2:86537965-86538913 | 15037 | Olfr1197 | NM_001005225.1 | chr2:88728652-88729597 |
| 14941 | Olfr108 | NM_146465.2 | chr17:37445479-37446517 | 15038 | Olfr1198 | NM_207567.1 | chr2:88745959-88746886 |
| 14942 | Olfr1080 | NM_146409.2 | chr2:86553180-86558138 | 15039 | Olfr1199 | NM_146458.1 | chr2:88755740-88756673 |
| 14943 | Olfr1082 | NM_207674.2 | chr2:86593884-86598809 | 15040 | Olfr12 | NM_206896.2 | chr1:92619880-92621001 |
| 14944 | Olfr1084 | NM_207135.2 | chr2:86638764-86639718 | 15041 | Olfr120 | NM_146631.1 | chr17:37725998-37726991 |
| 14945 | Olfr1085 | NM_146590.2 | chr2:86657514-86658456 | 15042 | Olfr1200 | NM_001005227.2 | chr2:88767302-88768313 |
| 14946 | Olfr1086 | NM_146592.1 | chr2:86676398-86677331 | 15043 | Olfr1201 | NM_146895.1 | chr2:88794383-88795307 |
| 14947 | Olfr1087 | NM_146846.2 | chr2:86690031-86690973 | 15044 | Olfr1202 | NM_146462.1 | chr2:88817172-88818102 |
| 14948 | Olfr1089 | NM_001011771.2 | chr2:86732674-86733610 | 15045 | Olfr1204 | NM_146463.2 | chr2:88851951-88852881 |
| 14949 | Olfr109 | NM_146835.1 | chr17:37466207-37467152 | 15046 | Olfr1205 | NM_146896.3 | chr2:88829756-88832042 |
| 14950 | Olfr1090 | NM_146847.1 | chr2:86753794-86754736 | 15047 | Olfr1206 | NM_001001810.2 | chr2:88864606-88865530 |
| 14951 | Olfr1093 | NM_146366.1 | chr2:86785731-86786700 | 15048 | Olfr1208 | NM_146778.1 | chr2:88896668-88897595 |
| 14952 | Olfr1094 | NM_146365.2 | chr2:86828675-86829822 | 15049 | Olfr1209 | NM_146461.2 | chr2:88909458-88910391 |
| 14953 | Olfr1095 | NM_146730.2 | chr2:86850769-86851696 | 15050 | Olfr121 | NM_146629.2 | chr17:37748865-37753004 |
| 14954 | Olfr1097 | NM_146843.2 | chr2:86890225-86892163 | 15051 | Olfr1211 | NM_001011804.1 | chr2:88929377-88930313 |
| 14955 | Olfr1098 | NM_146845.2 | chr2:86922582-86923530 | 15052 | Olfr1212 | NM_207140.1 | chr2:88958467-88959403 |
| 14956 | Olfr1099 | NM_146768.1 | chr2:86958517-86959456 | 15053 | Olfr1213 | NM_146898.2 | chr2:88972953-88980267 |
| 14957 | Olfr11 | NM_146542.2 | chr13:21638579-21639521 | 15054 | Olfr1214 | NM_146897.2 | chr2:88987264-88988200 |
| 14958 | Olfr110 | NM_146328.2 | chr17:37492467-37499674 | 15055 | Olfr1215 | NM_146459.2 | chr2:89001347-89002290 |
| 14959 | Olfr1100 | NM_146594.1 | chr2:86977828-86978794 | 15056 | Olfr1216 | NM_146893.2 | chr2:89013126-89014062 |
| 14960 | Olfr1101 | NM_146591.2 | chr2:86988241-86989174 | 15057 | Olfr1217 | NM_146901.2 | chr2:89022994-89024099 |
| 14961 | Olfr1102 | NM_207154.2 | chr2:87001931-87002994 | 15058 | Olfr1218 | NM_146818.2 | chr2:89054488-89055424 |
| 14962 | Olfr1104 | NM_146767.2 | chr2:87021609-87022542 | 15059 | Olfr1219 | NM_146899.2 | chr2:89074153-89075089 |
| 14963 | Olfr1105 | NM_001011825.1 | chr2:87033280-87034219 | 15060 | Olfr122 | NM_146288.3 | chr17:37768641-37772620 |
| 14964 | Olfr1106 | NM_146752.2 | chr2:87048295-87049234 | 15061 | Olfr1220 | NM_146900.2 | chr2:89096937-89097963 |
| 14965 | Olfr1107 | NM_146844.2 | chr2:87071115-87072223 | 15062 | Olfr1221 | NM_146902.2 | chr2:89111574-89112510 |
| 14966 | Olfr1109 | NM_146766.2 | chr2:87092456-87093395 | 15063 | Olfr1222 | NM_001011860.1 | chr2:89124793-89125729 |
| 14967 | Olfr111 | NM_001005485.2 | chr17:37529956-37530997 | 15064 | Olfr1223 | NM_146892.2 | chr2:89144085-89151336 |
| 14968 | Olfr1110 | NM_146769.1 | chr2:87135380-87136319 | 15065 | Olfr1225 | NM_146891.2 | chr2:89170218-89171245 |
| 14969 | Olfr1111 | NM_146593.2 | chr2:87149719-87150659 | 15066 | Olfr1226 | NM_146967.1 | chr2:89193099-89194032 |
| 14970 | Olfr1112 | NM_146661.2 | chr2:87191688-87192645 | 15067 | Olfr1228 | NM_146971.1 | chr2:89248720-89249692 |
| 14971 | Olfr1113 | NM_207565.1 | chr2:87212893-87213874 | 15068 | Olfr1229 | NM_001011761.1 | chr2:89282195-89283131 |
| 14972 | Olfr1115 | NM_146297.2 | chr2:87251839-87252941 | 15069 | Olfr123 | NM_146630.1 | chr17:37795445-37796375 |
| 14973 | Olfr1116-ps | NM_001011734.1 | chr2:87268845-87269769 | 15070 | Olfr1230 | NM_146789.1 | chr2:89296350-89297268 |
| 14974 | Olfr1118 | NM_207632.2 | chr2:87308763-87309759 | 15071 | Olfr1231 | NM_146454.2 | chr2:89302648-89303590 |
| 14975 | Olfr112 | NM_001013575.4 | chr17:37563238-37569451 | 15072 | Olfr1232 | NM_146323.1 | chr2:89325242-89326178 |
| 14976 | Olfr1120 | NM_147029.1 | chr2:87357445-87358390 | 15073 | Olfr1233 | NM_146972.1 | chr2:89339382-89340300 |
| 14977 | Olfr1121 | NM_146348.2 | chr2:87371533-87372478 | 15074 | Olfr1234 | NM_146973.2 | chr2:89362482-89363427 |
| 14978 | Olfr1122 | NM_147031.1 | chr2:87387706-87388687 | 15075 | Olfr1238 | NM_146790.1 | chr2:89406129-89407077 |
| 14979 | Olfr1123 | NM_146350.2 | chr2:87418049-87419021 | 15076 | Olfr1239 | NM_146970.1 | chr2:89417493-89418411 |
| 14980 | Olfr1124 | NM_147028.2 | chr2:87434488-87435445 | 15077 | Olfr124 | NM_147062.2 | chr17:37805068-37806190 |
| 14981 | Olfr1126 | NM_146837.2 | chr2:87457166-87458111 | 15078 | Olfr1240 | NM_146808.2 | chr2:89439269-89440343 |
| 14982 | Olfr1128 | NM_146349.2 | chr2:87544606-87545542 | 15079 | Olfr1241 | NM_146455.1 | chr2:89482188-89483133 |
| 14983 | Olfr1129 | NM_001011836.2 | chr2:87575085-87576030 | 15080 | Olfr1242 | NM_146968.2 | chr2:89493293-89494346 |
| 14984 | Olfr113 | NM_146289.1 | chr17:37574482-37575421 | 15081 | Olfr1243 | NM_146969.1 | chr2:89527490-89528408 |
| 14985 | Olfr1130 | NM_146838.2 | chr2:87606211-87608334 | 15082 | Olfr1245 | NM_146788.2 | chr2:89574711-89575773 |
| 14986 | Olfr1131 | NM_146658.2 | chr2:87628464-87629394 | 15083 | Olfr1246 | NM_146792.2 | chr2:89590068-89591146 |
| 14987 | Olfr1132 | NM_146836.1 | chr2:87634818-87635745 | 15084 | Olfr1247 | NM_146966.2 | chr2:89609088-89610153 |
| 14988 | Olfr1133 | NM_146351.2 | chr2:87645179-87646121 | 15085 | Olfr1248 | NM_146791.2 | chr2:89617167-89618245 |
| 14989 | Olfr1134 | NM_147030.2 | chr2:87655924-87658457 | 15086 | Olfr1249 | NM_001011796.2 | chr2:89629939-89630896 |
| 14990 | Olfr1135 | NM_146660.2 | chr2:87671432-87672365 | 15087 | Olfr125 | NM_146290.2 | chr17:37834910-37836028 |
| 14991 | Olfr1136 | NM_146659.3 | chr2:87692880-87693947 | 15088 | Olfr1250 | NM_146965.1 | chr2:89656494-89657439 |
| 14992 | Olfr1137 | NM_001011833.1 | chr2:87710971-87711904 | 15089 | Olfr1251 | NM_001011529.1 | chr2:89666927-89667884 |
| 14993 | Olfr1138 | NM_146639.1 | chr2:87737386-87738322 | 15090 | Olfr1252 | NM_207568.1 | chr2:89721164-89722109 |
| 14994 | Olfr114 | NM_146287.1 | chr17:37589412-37590351 | 15091 | Olfr1253 | NM_146373.1 | chr2:89751869-89752826 |
| 14995 | Olfr1140 | NM_146642.2 | chr2:87746160-87747180 | 15092 | Olfr1254 | NM_146476.1 | chr2:89788405-89789350 |
| 14996 | Olfr1141 | NM_146637.1 | chr2:87753055-87753991 | 15093 | Olfr1255 | NM_146977.2 | chr2:89816327-89817260 |
| 14997 | Olfr1143 | NM_146293.2 | chr2:87802390-87803335 | 15094 | Olfr1256 | NM_146983.1 | chr2:89835022-89835943 |
| 14998 | Olfr1145 | NM_146320.2 | chr2:87809821-87810799 | 15095 | Olfr1257 | NM_146982.1 | chr2:89880827-89881757 |
| 14999 | Olfr1148 | NM_001011519.1 | chr2:87833040-87833985 | 15096 | Olfr1258 | NM_146978.1 | chr2:89929810-89930746 |
| 15000 | Olfr115 | NM_001011753.2 | chr17:37609784-37610779 | 15097 | Olfr1259 | NM_146341.1 | chr2:89943183-89944113 |
| 15001 | Olfr1151 | NM_146638.1 | chr2:87857176-87858103 | 15098 | Olfr126 | NM_146890.2 | chr17:37850593-37851553 |
| 15002 | Olfr1152 | NM_001011834.1 | chr2:87867992-87868925 | 15099 | Olfr1260 | NM_146981.1 | chr2:89977779-89978712 |
| 15003 | Olfr1153 | NM_146640.2 | chr2:87896176-87897133 | 15100 | Olfr1261 | NM_146474.1 | chr2:89993394-89994315 |
| 15004 | Olfr1154 | NM_146647.2 | chr2:87902741-87903674 | 15101 | Olfr1262 | NM_146974.1 | chr2:90002407-90003322 |
| 15005 | Olfr1155 | NM_146643.2 | chr2:87942681-87943626 | 15102 | Olfr1263 | NM_146794.1 | chr2:90014931-90015852 |
| 15006 | Olfr1156 | NM_146817.2 | chr2:87949189-87950265 | 15103 | Olfr1264 | NM_021368.1 | chr2:90021137-90022064 |
| 15007 | Olfr1157 | NM_146849.2 | chr2:87961951-87968257 | 15104 | Olfr1265 | NM_146343.1 | chr2:90036920-90037850 |
| 15008 | Olfr1158 | NM_146645.2 | chr2:87990112-87991054 | 15105 | Olfr1269 | NM_146342.1 | chr2:90118666-90119596 |
| 15009 | Olfr116 | NM_146632.1 | chr17:37623667-37624633 | 15106 | Olfr127 | NM_146377.1 | chr17:37903547-37904519 |
| 15010 | Olfr1160 | NM_146649.2 | chr2:88005816-88006776 | 15107 | Olfr1270 | NM_146985.2 | chr2:90149018-90150036 |
| 15011 | Olfr1161 | NM_146848.2 | chr2:88024701-88025713 | 15108 | Olfr1271 | NM_146793.1 | chr2:90265510-90266428 |
| 15012 | Olfr1162 | NM_001011835.1 | chr2:88049677-88050622 | 15109 | Olfr1272 | NM_146980.1 | chr2:90281646-90282573 |
| 15013 | Olfr1163 | NM_146644.2 | chr2:88070364-88071407 | 15110 | Olfr1273-ps | NM_146975.1 | chr2:90295935-90296859 |
| 15014 | Olfr1164 | NM_146641.2 | chr2:88092854-88093966 | 15111 | Olfr1274-ps | NM_146263.2 | chr2:90400639-90401674 |
| 15015 | Olfr1166 | NM_146650.2 | chr2:88123361-88124993 | 15112 | Olfr1275 | NM_001011795.1 | chr2:111230852-111231791 |
| 15016 | Olfr1167 | NM_146294.2 | chr2:88148983-88150083 | 15113 | Olfr1276 | NM_146395.1 | chr2:111257116-111258055 |
| 15017 | Olfr1168 | NM_146531.2 | chr2:88184878-88185817 | 15114 | Olfr1277 | NM_146396.1 | chr2:111269453-111270365 |
| 15018 | Olfr117 | NM_207155.2 | chr17:37659377-37660331 | 15115 | Olfr1278 | NM_146394.1 | chr2:111292269-111293211 |
| 15019 | Olfr1170 | NM_146532.1 | chr2:88224079-88225030 | 15116 | Olfr1279 | NM_146393.1 | chr2:111306200-111307142 |
| 15020 | Olfr1173 | NM_207566.1 | chr2:88274108-88275047 | 15117 | Olfr128 | NM_206816.1 | chr17:37923567-37924494 |
| 15021 | Olfr1176 | NM_146771.1 | chr2:88339566-88340514 | 15118 | Olfr1280 | NM_146908.1 | chr2:111315480-111316398 |
| 15022 | Olfr1178 | NM_001011868.1 | chr2:88391248-88392220 | 15119 | Olfr1281 | NM_001005568.1 | chr2:111328420-111329338 |
| 15023 | Olfr1179 | NM_146917.2 | chr2:88402008-88402932 | 15120 | Olfr1282 | NM_146907.2 | chr2:111335158-111336076 |
| 15024 | Olfr118 | NM_213721.2 | chr17:37672024-37672990 | 15121 | Olfr1283 | NM_207236.1 | chr2:111368633-111369551 |
| 15025 | Olfr1180 | NM_146918.2 | chr2:88411626-88412689 | 15122 | Olfr1284 | NM_146381.1 | chr2:111379001-111379937 |
| 15026 | Olfr1181 | NM_001011816.1 | chr2:88423087-88424023 | 15123 | Olfr1286 | NM_207254.1 | chr2:111420031-111420949 |
| 15027 | Olfr1182 | NM_001011535.2 | chr2:88446018-88449121 | 15124 | Olfr1287 | NM_001011773.1 | chr2:111449141-111450059 |
| 15028 | Olfr1183 | NM_146529.2 | chr2:88461341-88462253 | 15125 | Olfr1288 | NM_146400.2 | chr2:111478785-111479724 |
| 15029 | Olfr1184 | NM_146823.1 | chr2:88486733-88487669 | 15126 | Olfr1289 | NM_146404.1 | chr2:111483431-111484328 |
| 15030 | Olfr1186 | NM_146530.2 | chr2:88525500-88526590 | 15127 | Olfr129 | NM_146327.2 | chr17:38054598-38059784 |
| 15031 | Olfr1188 | NM_146919.2 | chr2:88559450-88560418 | 15128 | Olfr1290 | NM_001278787.1 | chr2:111489217-111493815 |
| 15032 | Olfr1189 | NM_146772.2 | chr2:88591805-88592726 | 15129 | Olfr1294 | NM_146885.1 | chr2:111537348-111538287 |
| 15033 | Olfr119 | NM_001011830.2 | chr17:37696684-37701637 | 15130 | Olfr1295 | NM_146403.1 | chr2:111564503-111565442 |
| 15034 | Olfr1193 | NM_001011517.2 | chr2:88677856-88678813 | 15131 | Olfr1297 | NM_146888.1 | chr2:111621133-111622072 |

Fig.21 - 79

| | | | | | | | |
|---|---|---|---|---|---|---|---|
| 15132 | Olfr1298 | NM_146886.1 | chr2:111645056-111645995 | 15229 | Olfr1408 | NM_146764.1 | chr1:173130282-173131215 |
| 15133 | Olfr1299 | NM_146884.2 | chr2:111661452-111665166 | 15230 | Olfr141 | NM_181818.2 | chr2:86806020-86806997 |
| 15134 | Olfr13 | NM_146652.1 | chr6:43173987-43174920 | 15231 | Olfr1410 | NM_146491.1 | chr1:92607838-92608807 |
| 15135 | Olfr130 | NM_146487.1 | chr17:38067172-38068126 | 15232 | Olfr1411 | NM_146490.1 | chr1:92596520-92597492 |
| 15136 | Olfr1300-ps1 | NR_033534.1 | chr2:111667436-111693319 | 15233 | Olfr1412 | NM_146277.1 | chr1:92588331-92589297 |
| 15137 | Olfr1301 | NM_146887.1 | chr2:111754250-111755189 | 15234 | Olfr1413 | NM_147037.2 | chr1:92573124-92574206 |
| 15138 | Olfr1302 | NM_146889.2 | chr2:111780312-111781260 | 15235 | Olfr1414 | NM_147039.2 | chr1:92511047-92518515 |
| 15139 | Olfr1303 | NM_146402.2 | chr2:111813785-111814724 | 15236 | Olfr1415 | NM_001011525.1 | chr1:92490817-92491753 |
| 15140 | Olfr1305 | NM_146401.2 | chr2:111872914-111873853 | 15237 | Olfr1416 | NM_147038.1 | chr1:92479680-92480619 |
| 15141 | Olfr1306 | NM_001011803.2 | chr2:111911989-111912928 | 15238 | Olfr1417 | NM_146936.1 | chr19:11828076-11829024 |
| 15142 | Olfr1307 | NM_001011787.1 | chr2:111944515-111945454 | 15239 | Olfr1418 | NM_001011524.1 | chr19:11854994-11855951 |
| 15143 | Olfr1308 | NM_207151.1 | chr2:111960108-111961071 | 15240 | Olfr1419 | NM_001011775.1 | chr19:11870182-11871214 |
| 15144 | Olfr1309 | NM_146447.1 | chr2:111983133-111984096 | 15241 | Olfr142 | NM_146984.1 | chr2:90252068-90252986 |
| 15145 | Olfr131 | NM_146867.1 | chr17:38082031-38082976 | 15242 | Olfr1420 | NM_146410.1 | chr19:11896022-11896952 |
| 15146 | Olfr1310 | NM_146449.1 | chr2:112008245-112009184 | 15243 | Olfr1423 | NM_146680.1 | chr19:12035807-12036740 |
| 15147 | Olfr1311 | NM_146274.1 | chr2:112020913-112021852 | 15244 | Olfr1424 | NM_146681.1 | chr19:12058808-12059750 |
| 15148 | Olfr1312 | NM_146362.1 | chr2:112042076-112043030 | 15245 | Olfr1425 | NM_001011853.1 | chr19:12073694-12074630 |
| 15149 | Olfr1313 | NM_207150.1 | chr2:112071645-112072581 | 15246 | Olfr1426 | NM_146809.2 | chr19:12087609-12091847 |
| 15150 | Olfr1314 | NM_146450.2 | chr2:112091760-112092699 | 15247 | Olfr1427 | NM_146679.1 | chr19:12098701-12099637 |
| 15151 | Olfr1316 | NM_146742.1 | chr2:112129870-112130815 | 15248 | Olfr1428 | NM_146678.2 | chr19:12108599-12109544 |
| 15152 | Olfr1317 | NM_146448.1 | chr2:112141946-112142897 | 15249 | Olfr143 | NM_146806.2 | chr9:38253418-38254360 |
| 15153 | Olfr1318 | NM_001011802.2 | chr2:112155897-112157007 | 15250 | Olfr1431 | NM_146414.1 | chr19:12209567-12210506 |
| 15154 | Olfr132 | NM_001005481.1 | chr17:38130248-38131190 | 15251 | Olfr1433 | NM_146685.3 | chr19:12279853-12283988 |
| 15155 | Olfr1320 | NM_207240.2 | chrX:49683503-49684463 | 15252 | Olfr1434 | NM_146685.3 | chr19:12957098-12958036 |
| 15156 | Olfr1321 | NM_207631.1 | chrX:49726972-49727932 | 15253 | Olfr1436 | NM_146687.2 | chr19:12298182-12299130 |
| 15157 | Olfr1322 | NM_001011794.1 | chrX:49885468-49886401 | 15254 | Olfr1437 | NM_001011839.1 | chr19:12321886-12322825 |
| 15158 | Olfr1323 | NM_146390.1 | chrX:50009305-50010235 | 15255 | Olfr1440 | NM_146684.1 | chr19:12394264-12395212 |
| 15159 | Olfr1324 | NM_146292.1 | chrX:50425495-50426494 | 15256 | Olfr1441 | NM_146683.1 | chr19:12422310-12423267 |
| 15160 | Olfr1325 | NM_146398.1 | chrX:74594327-74595275 | 15257 | Olfr1442 | NM_146697.2 | chr19:12674178-12675243 |
| 15161 | Olfr1328 | NM_146399.2 | chr4:118933898-118934840 | 15258 | Olfr1443 | NM_146698.2 | chr19:12678165-12683845 |
| 15162 | Olfr1329 | NM_001011870.2 | chr4:118916523-118917465 | 15259 | Olfr1444 | NM_146702.1 | chr19:12861776-12862736 |
| 15163 | Olfr133 | NM_146831.1 | chr17:38148589-38149528 | 15260 | Olfr1445 | NM_146699.1 | chr19:12883882-12884827 |
| 15164 | Olfr1330 | NM_146334.2 | chr4:118893084-118894032 | 15261 | Olfr1446 | NM_146704.1 | chr19:12889648-12890575 |
| 15165 | Olfr1331 | NM_001011856.2 | chr4:118868782-118869736 | 15262 | Olfr1447 | NM_146703.1 | chr19:12900848-12901778 |
| 15166 | Olfr1333 | NM_207157.2 | chr4:118829484-118830438 | 15263 | Olfr1448 | NM_146701.1 | chr19:12919362-12920307 |
| 15167 | Olfr1335 | NM_207703.1 | chr4:118808854-118809862 | 15264 | Olfr1449 | NM_146303.1 | chr19:12934739-12935684 |
| 15168 | Olfr1336 | NM_146915.1 | chr7:6460510-6461455 | 15265 | Olfr145 | NM_146313.1 | chr9:37897405-37898338 |
| 15169 | Olfr1337 | NM_146309.3 | chr4:118781635-118782586 | 15266 | Olfr1450 | NM_146371.1 | chr19:12953590-12954568 |
| 15170 | Olfr1338 | NM_207152.2 | chr4:118753594-118754536 | 15267 | Olfr1451 | NM_146705.1 | chr19:12998987-12999920 |
| 15171 | Olfr1339 | NM_146852.2 | chr4:118734530-118735478 | 15268 | Olfr1453 | NM_146700.1 | chr19:13027403-13028327 |
| 15172 | Olfr134 | NM_146832.1 | chr17:38175085-38176024 | 15269 | Olfr1454 | NM_146692.1 | chr19:13063412-13064336 |
| 15173 | Olfr1340 | NM_146304.2 | chr4:118726248-118727196 | 15270 | Olfr1457 | NM_146575.1 | chr19:13094692-13095646 |
| 15174 | Olfr1341 | NM_146853.2 | chr4:118709408-118710347 | 15271 | Olfr1459 | NM_146689.1 | chr19:13145733-13146657 |
| 15175 | Olfr1342 | NM_146713.1 | chr4:118689502-118690450 | 15272 | Olfr146 | NM_146747.1 | chr9:39018618-39019539 |
| 15176 | Olfr1344 | NM_177061.3 | chr7:6439901-6440867 | 15273 | Olfr1461 | NM_146302.1 | chr19:13165015-13165954 |
| 15177 | Olfr1346 | NM_146916.1 | chr7:6474111-6475053 | 15274 | Olfr1462 | NM_146693.1 | chr19:13190668-13191592 |
| 15178 | Olfr1347 | NM_146385.1 | chr7:6487912-6488872 | 15275 | Olfr1463 | NM_001011840.1 | chr19:13234251-13235184 |
| 15179 | Olfr1348 | NM_146913.1 | chr7:6501285-6502224 | 15276 | Olfr1465 | NM_001011841.1 | chr19:13313359-13314283 |
| 15180 | Olfr1349 | NM_207136.1 | chr7:6514473-6515427 | 15277 | Olfr1466 | NM_146694.1 | chr19:13341759-13342692 |
| 15181 | Olfr135 | NM_146332.1 | chr17:38208246-38209185 | 15278 | Olfr1467 | NM_146691.1 | chr19:13364629-13365556 |
| 15182 | Olfr1350 | NM_146389.1 | chr7:6569992-6570919 | 15279 | Olfr1469 | NM_146695.1 | chr19:13410570-13411500 |
| 15183 | Olfr1351 | NM_147040.1 | chr10:79017323-79018283 | 15280 | Olfr147 | NM_146869.2 | chr9:38401710-38403829 |
| 15184 | Olfr1352 | NM_147071.2 | chr10:78981049-78984721 | 15281 | Olfr1471 | NM_207132.2 | chr19:13445013-13445958 |
| 15185 | Olfr1353 | NM_147042.2 | chr10:78963308-78970612 | 15282 | Olfr1472 | NM_146690.2 | chr19:13453570-13454515 |
| 15186 | Olfr1354 | NM_001199840.1 | chr10:78916841-78917936 | 15283 | Olfr1474 | NM_001011842.1 | chr19:13470971-13471916 |
| 15187 | Olfr1355 | NM_207571.2 | chr10:78875548-78880106 | 15284 | Olfr1475 | NM_146301.1 | chr19:13479251-13480196 |
| 15188 | Olfr1356 | NM_146308.2 | chr10:78846950-78847913 | 15285 | Olfr1477 | NM_146696.2 | chr19:13500545-13503292 |
| 15189 | Olfr1357 | NM_001011737.2 | chr10:78611668-78618074 | 15286 | Olfr148 | NM_146505.1 | chr9:39613568-39614501 |
| 15190 | Olfr1359 | NM_001011820.1 | chr13:21674000-21703944 | 15287 | Olfr1480 | NM_207575.1 | chr19:13529674-13530622 |
| 15191 | Olfr136 | NM_146807.1 | chr17:38335158-38336097 | 15288 | Olfr1484 | NM_146291.1 | chr19:13585305-13586253 |
| 15192 | Olfr1360 | NM_146543.2 | chr13:21674000-21674980 | 15289 | Olfr1487 | NM_146636.1 | chr19:13619163-13620111 |
| 15193 | Olfr1361 | NM_146541.2 | chr13:21658367-21659321 | 15290 | Olfr1489 | NM_146635.1 | chr19:13633112-13634054 |
| 15194 | Olfr1362 | NM_146744.2 | chr13:21611019-21612004 | 15291 | Olfr149 | NM_207138.1 | chr9:39701831-39702767 |
| 15195 | Olfr1364 | NM_146540.2 | chr13:21573509-21574454 | 15292 | Olfr1490 | NM_001011832.1 | chr19:13654445-13655396 |
| 15196 | Olfr1366 | NM_146283.2 | chr13:21536973-21538027 | 15293 | Olfr1491 | NM_146345.1 | chr19:13704828-13705788 |
| 15197 | Olfr1367 | NM_146533.1 | chr13:21346929-21347880 | 15294 | Olfr1494 | NM_146990.1 | chr19:13749107-13750055 |
| 15198 | Olfr1368 | NM_146534.1 | chr13:21142110-21143055 | 15295 | Olfr1495 | NM_146344.1 | chr19:13768343-13769303 |
| 15199 | Olfr137 | NM_146488.1 | chr17:38304520-38305459 | 15296 | Olfr1496 | NM_146989.2 | chr19:13780547-13781614 |
| 15200 | Olfr1370 | NM_146535.1 | chr13:21072348-21073299 | 15297 | Olfr1497 | NM_146741.1 | chr19:13794664-13795609 |
| 15201 | Olfr1371 | NM_207253.1 | chr11:52213051-52213987 | 15298 | Olfr1499 | NM_146796.1 | chr19:13814643-13815588 |
| 15202 | Olfr1372-ps1 | NR_034155.1 | chr11:52154875-52157907 | 15299 | Olfr15 | NM_008762.2 | chr16:3838974-3839913 |
| 15203 | Olfr1373 | NM_207227.1 | chr11:52144592-52145528 | 15300 | Olfr150 | NM_146609.2 | chr9:39736816-39737755 |
| 15204 | Olfr1377 | NM_146911.1 | chr11:50984702-50985626 | 15301 | Olfr1500 | NM_001011831.1 | chr19:13827458-13828394 |
| 15205 | Olfr1378 | NM_146910.1 | chr11:50969019-50969967 | 15302 | Olfr1501 | NM_146633.2 | chr19:13838223-13839171 |
| 15206 | Olfr138 | NM_130868.1 | chr17:38274772-38275711 | 15303 | Olfr1502 | NM_146797.1 | chr19:13861794-13862745 |
| 15207 | Olfr1380 | NM_207573.1 | chr11:49563922-49564858 | 15304 | Olfr1504 | NM_146634.1 | chr19:13887260-13888208 |
| 15208 | Olfr1381 | NM_146469.2 | chr11:49551748-49552684 | 15305 | Olfr1505 | NM_001011850.1 | chr19:13919021-13919972 |
| 15209 | Olfr1382 | NM_001011790.1 | chr11:49535186-49536122 | 15306 | Olfr1506 | NM_146265.2 | chr2:90221566-90325126 |
| 15210 | Olfr1383 | NM_207574.1 | chr11:49523724-49524660 | 15307 | Olfr1507 | NM_001170918.1 | chr14:52489934-52495695 |
| 15211 | Olfr1384 | NM_146472.2 | chr11:49513617-49514651 | 15308 | Olfr1508 | NM_020513.2 | chr14:52462720-52467461 |
| 15212 | Olfr1385 | NM_001011805.1 | chr11:49494534-49495464 | 15309 | Olfr1509 | NM_020514.2 | chr14:52450392-52451402 |
| 15213 | Olfr1386 | NM_001011741.2 | chr11:49470053-49471181 | 15310 | Olfr151 | NM_207664.2 | chr9:37729976-37731061 |
| 15214 | Olfr1387 | NM_146473.1 | chr11:49459680-49460616 | 15311 | Olfr1510 | NM_146431.2 | chr14:52409888-52410927 |
| 15215 | Olfr1388 | NM_146467.1 | chr11:49443852-49444788 | 15312 | Olfr1511 | NM_146271.2 | chr14:52389814-52390771 |
| 15216 | Olfr1389 | NM_147066.2 | chr11:49430426-49431499 | 15313 | Olfr1512 | NM_146432.2 | chr14:52372109-52373051 |
| 15217 | Olfr139 | NM_147003.1 | chr11:74044324-74045272 | 15314 | Olfr1513 | NM_001012269.2 | chr14:52349102-52350044 |
| 15218 | Olfr1390 | NM_147065.1 | chr11:49340533-49341469 | 15315 | Olfr152 | NM_146646.2 | chr2:87782535-87783486 |
| 15219 | Olfr1391 | NM_146468.1 | chr11:49327412-49328348 | 15316 | Olfr153 | NM_206823.1 | chr2:87532034-87532958 |
| 15220 | Olfr1392 | NM_146470.2 | chr11:49293296-49294322 | 15317 | Olfr1532-ps1 | NM_001011542.1 | chr7:106914199-106915123 |
| 15221 | Olfr1393 | NM_146471.1 | chr11:49280149-49281085 | 15318 | Olfr1535 | NM_207572.1 | chr13:21555039-21556020 |
| 15222 | Olfr1394 | NM_146276.1 | chr11:49160015-49160954 | 15319 | Olfr1537 | NM_207665.1 | chr9:39237486-39238431 |
| 15223 | Olfr1395 | NM_146877.1 | chr11:49148258-49149212 | 15320 | Olfr154 | NM_013728.2 | chr2:85663376-85664455 |
| 15224 | Olfr1396 | NM_146337.1 | chr11:49112776-49113757 | 15321 | Olfr155 | NM_019473.1 | chr4:43854291-43855463 |
| 15225 | Olfr140 | NM_020515.1 | chr2:90051413-90052322 | 15322 | Olfr156 | NM_019474.2 | chr4:43820334-43821433 |
| 15226 | Olfr1402 | NM_146275.1 | chr3:97410216-97411179 | 15323 | Olfr157 | NM_019475.3 | chr4:43834751-43836517 |
| 15227 | Olfr1404 | NM_146881.2 | chr1:173215606-173216640 | 15324 | Olfr159 | NM_019476.1 | chr4:43770049-43771009 |
| 15228 | Olfr1406 | NM_146763.2 | chr1:173183441-173184504 | 15325 | Olfr16 | NM_008763.2 | chr1:172956767-172957817 |

Fig.21 - 80

| | | | | | | | |
|---|---|---|---|---|---|---|---|
| 15326 | Olfr160 | NM_030553.2 | chr9:37711347-37712280 | 15423 | Olfr29-ps1 | NR_033638.1 | chr4:43781364-43782327 |
| 15327 | Olfr161 | NM_146860.1 | chr16:3592397-3593339 | 15424 | Olfr3 | NM_206903.1 | chr2:36812148-36813090 |
| 15328 | Olfr164 | NM_146451.1 | chr16:19285793-19286741 | 15425 | Olfr30 | NM_146878.2 | chr11:58454914-58455980 |
| 15329 | Olfr165 | NM_146466.1 | chr16:19407075-19408017 | 15426 | Olfr301 | NM_212436.2 | chr7:86403848-86413299 |
| 15330 | Olfr166 | NM_147068.1 | chr16:19486839-19487778 | 15427 | Olfr303 | NM_146619.1 | chr7:86394536-86395496 |
| 15331 | Olfr167 | NM_146935.1 | chr16:19514695-19515634 | 15428 | Olfr304 | NM_001011828.1 | chr7:86385656-86386658 |
| 15332 | Olfr168 | NM_146357.1 | chr16:19529979-19530918 | 15429 | Olfr305 | NM_146616.2 | chr7:86363375-86364335 |
| 15333 | Olfr169 | NM_001011855.1 | chr16:19565939-19566881 | 15430 | Olfr307 | NM_146617.1 | chr7:86335452-86336394 |
| 15334 | Olfr17 | NM_020598.2 | chr7:107097466-107098414 | 15431 | Olfr308 | NM_146621.1 | chr7:86321023-86321950 |
| 15335 | Olfr170 | NM_146957.1 | chr16:19605724-19606666 | 15432 | Olfr309 | NM_001011866.1 | chr7:86306184-86307111 |
| 15336 | Olfr171 | NM_146958.2 | chr16:19624156-19625101 | 15433 | Olfr31 | NM_147027.2 | chr14:14328112-14329066 |
| 15337 | Olfr172 | NM_147001.2 | chr16:58760152-58761240 | 15434 | Olfr310 | NM_001011520.2 | chr7:86268729-86269811 |
| 15338 | Olfr173 | NM_147000.2 | chr16:58796778-58797942 | 15435 | Olfr311 | NM_146537.2 | chr11:58841115-58842042 |
| 15339 | Olfr175-ps1 | NM_147002.2 | chr16:58823780-58826761 | 15436 | Olfr312 | NM_001011819.2 | chr11:58831155-58832082 |
| 15340 | Olfr176 | NM_146993.1 | chr16:58872214-58873148 | 15437 | Olfr313 | NM_146536.2 | chr11:58816927-58818001 |
| 15341 | Olfr177 | NM_146996.2 | chr16:58872218-58873148 | 15438 | Olfr314 | NM_001011760.2 | chr11:58786138-58787269 |
| 15342 | Olfr178 | NM_146997.2 | chr16:58889258-58890218 | 15439 | Olfr315 | NM_146538.2 | chr11:58778088-58779086 |
| 15343 | Olfr18 | NM_146563.1 | chr9:20313891-20336094 | 15440 | Olfr316 | NM_001011818.2 | chr11:58757666-58758587 |
| 15344 | Olfr180 | NM_001011662.2 | chr16:58915685-58918486 | 15441 | Olfr317 | NM_001011769.2 | chr11:58732095-58733223 |
| 15345 | Olfr181 | NM_146999.2 | chr16:58925556-58928644 | 15442 | Olfr318 | NM_146501.2 | chr11:58720068-58721094 |
| 15346 | Olfr183 | NM_146485.2 | chr16:58995403-59000616 | 15443 | Olfr319 | NM_146500.2 | chr11:58701702-58702623 |
| 15347 | Olfr186 | NM_146321.1 | chr16:59026975-59027905 | 15444 | Olfr32 | NM_010980.2 | chr2:90138179-90142293 |
| 15348 | Olfr187 | NM_146322.2 | chr16:59035779-59039749 | 15445 | Olfr320 | NM_207230.1 | chr11:58683874-58684795 |
| 15349 | Olfr19 | NM_146335.1 | chr16:16673049-16673979 | 15446 | Olfr322 | NM_207693.1 | chr11:58665560-58666544 |
| 15350 | Olfr190 | NM_146397.2 | chr16:59074154-59075078 | 15447 | Olfr323 | NM_146376.2 | chr11:58625072-58626044 |
| 15351 | Olfr191 | NM_001011807.2 | chr16:59085551-59086481 | 15448 | Olfr324 | NM_001011743.2 | chr11:58597344-58598466 |
| 15352 | Olfr192 | NM_207549.1 | chr16:59098065-59098990 | 15449 | Olfr325 | NM_207153.2 | chr11:58580836-58581877 |
| 15353 | Olfr193 | NM_001011791.1 | chr16:59109678-59110608 | 15450 | Olfr328 | NM_146502.2 | chr11:58551304-58552237 |
| 15354 | Olfr194 | NM_001005524.2 | chr16:59119147-59120068 | 15451 | Olfr329-ps | NM_001011531.3 | chr11:58542446-58543483 |
| 15355 | Olfr195 | NM_146998.1 | chr16:59148851-59149778 | 15452 | Olfr33 | NM_147073.1 | chr7:102713454-102714411 |
| 15356 | Olfr196 | NM_146779.2 | chr16:59167211-59168141 | 15453 | Olfr330 | NM_146879.2 | chr11:58528928-58534825 |
| 15357 | Olfr197 | NM_146484.1 | chr16:59185555-59186481 | 15454 | Olfr331 | NM_001011861.3 | chr11:58501597-58502572 |
| 15358 | Olfr198 | NM_001011808.1 | chr16:59201503-59202424 | 15455 | Olfr332 | NM_001011770.2 | chr11:58489718-58492499 |
| 15359 | Olfr199 | NM_207550.2 | chr16:59215684-59216611 | 15456 | Olfr338 | NM_146947.1 | chr2:36376777-36377698 |
| 15360 | Olfr2 | NM_010983.2 | chr7:107000874-107002605 | 15457 | Olfr339 | NM_146949.1 | chr2:36421399-36422329 |
| 15361 | Olfr20 | NM_146923.2 | chr11:73350858-73354699 | 15458 | Olfr340 | NM_146951.1 | chr2:36452586-36453525 |
| 15362 | Olfr201 | NM_146994.2 | chr16:59268738-59269665 | 15459 | Olfr341 | NM_146950.1 | chr2:36479186-36480128 |
| 15363 | Olfr202 | NM_146995.1 | chr16:59283571-59284495 | 15460 | Olfr342 | NM_146948.1 | chr2:36527413-36528352 |
| 15364 | Olfr203 | NM_146486.2 | chr16:59303154-59304075 | 15461 | Olfr344 | NM_146628.1 | chr2:36568599-36569529 |
| 15365 | Olfr204 | NM_146992.2 | chr16:59314487-59315405 | 15462 | Olfr345 | NM_146945.1 | chr2:36640040-36640976 |
| 15366 | Olfr205 | NM_001011736.1 | chr16:59328589-59329507 | 15463 | Olfr346 | NM_146938.1 | chr2:36688003-36688933 |
| 15367 | Olfr206 | NM_146991.1 | chr16:59344778-59345699 | 15464 | Olfr347 | NM_146943.1 | chr2:36734322-36735261 |
| 15368 | Olfr209 | NM_207551.2 | chr16:59361298-59362216 | 15465 | Olfr348 | NM_146944.1 | chr2:36786526-36787468 |
| 15369 | Olfr211 | NM_146912.1 | chr6:116493610-116494540 | 15466 | Olfr350 | NM_146627.1 | chr2:36850047-36850986 |
| 15370 | Olfr212 | NM_001011800.2 | chr6:116506515-116517965 | 15467 | Olfr351 | NM_146942.1 | chr2:36859413-36860346 |
| 15371 | Olfr213 | NM_001011801.1 | chr6:116540454-116541438 | 15468 | Olfr352 | NM_146940.1 | chr2:36869567-36870515 |
| 15372 | Olfr214 | NM_146759.1 | chr6:116556426-116557389 | 15469 | Olfr353 | NM_146941.1 | chr2:36889910-36890846 |
| 15373 | Olfr215 | NM_146446.1 | chr6:116582011-116582944 | 15470 | Olfr354 | NM_146939.1 | chr2:36906947-36907901 |
| 15374 | Olfr218 | NM_001001809.2 | chr1:173203357-173204299 | 15471 | Olfr355 | NM_146625.1 | chr2:36927179-36928112 |
| 15375 | Olfr220 | NM_207694.1 | chr1:174448624-174449602 | 15472 | Olfr356 | NM_146624.1 | chr2:36937120-36938068 |
| 15376 | Olfr221 | NM_001001808.2 | chr14:52035173-52036109 | 15473 | Olfr357 | NM_146623.1 | chr2:36996811-36997738 |
| 15377 | Olfr222 | NM_001011789.1 | chr11:59570781-59571738 | 15474 | Olfr358 | NM_207235.1 | chr2:37004619-37005612 |
| 15378 | Olfr223 | NM_146429.1 | chr11:59589127-59590087 | 15475 | Olfr360 | NM_146622.1 | chr2:37068306-37069260 |
| 15379 | Olfr224 | NM_207695.1 | chr11:58566398-58567343 | 15476 | Olfr361 | NM_146368.1 | chr2:37084777-37085746 |
| 15380 | Olfr225 | NM_001011740.2 | chr11:59612965-59614225 | 15477 | Olfr362 | NM_147051.1 | chr2:37104694-37105648 |
| 15381 | Olfr228 | NM_146405.2 | chr2:86482798-86483740 | 15478 | Olfr365 | NM_146662.1 | chr2:37201242-37202181 |
| 15382 | Olfr229 | NM_146613.1 | chr9:39909804-39910728 | 15479 | Olfr366 | NM_001005569.1 | chr2:37219490-37220420 |
| 15383 | Olfr23 | NM_010970.1 | chr11:73940247-73941225 | 15480 | Olfr367-ps | NM_001081010.2 | chr2:37266950-37271387 |
| 15384 | Olfr231 | NM_001005520.2 | chr1:174117090-174118014 | 15481 | Olfr368 | NM_146374.1 | chr2:37331748-37332732 |
| 15385 | Olfr235 | NM_146686.2 | chr19:12268231-12269170 | 15482 | Olfr370 | NM_146270.2 | chr8:83541092-83542188 |
| 15386 | Olfr237-ps1 | NM_146654.1 | chr6:43153306-43154239 | 15483 | Olfr371 | NM_146859.2 | chr8:85230496-85231435 |
| 15387 | Olfr239 | NM_207175.2 | chr17:33199061-33200009 | 15484 | Olfr372 | NM_207555.2 | chr8:72057659-72058675 |
| 15388 | Olfr24 | NM_146606.1 | chr9:18754691-18755633 | 15485 | Olfr373 | NM_146539.2 | chr8:72099761-72100706 |
| 15389 | Olfr242 | NM_010974.1 | chr9:39144101-39145018 | 15486 | Olfr374 | NM_146338.2 | chr8:72107039-72110509 |
| 15390 | Olfr243 | NM_001025386.1 | chr7:103716595-103717546 | 15487 | Olfr376 | NM_001172686.1 | chr11:73371245-73375704 |
| 15391 | Olfr247 | NM_146269.2 | chr10:129974604-129984424 | 15488 | Olfr378 | NM_147024.2 | chr11:73425036-73428477 |
| 15392 | Olfr248 | NM_146714.2 | chr1:174391045-174392055 | 15489 | Olfr38 | NM_146986.1 | chr6:42762053-42763007 |
| 15393 | Olfr25 | NM_146870.2 | chr9:38329491-38330559 | 15490 | Olfr380 | NM_147025.1 | chr11:73453274-73454210 |
| 15394 | Olfr259 | NM_146770.2 | chr2:87107446-87108385 | 15491 | Olfr381 | NM_147022.2 | chr11:73485886-73486822 |
| 15395 | Olfr26 | NM_146783.2 | chr9:38855059-38855990 | 15492 | Olfr382 | NM_146443.1 | chr11:73516258-73517197 |
| 15396 | Olfr262 | NM_146688.1 | chr19:12240720-12241659 | 15493 | Olfr384 | NM_207224.1 | chr11:73602581-73603520 |
| 15397 | Olfr263 | NM_010984.1 | chr13:21132776-21133730 | 15494 | Olfr385 | NM_147023.1 | chr11:73588797-73589736 |
| 15398 | Olfr266 | NM_146489.1 | chr3:106821606-106822557 | 15495 | Olfr389 | NM_147009.3 | chr11:73776386-73780589 |
| 15399 | Olfr267 | NM_146920.2 | chr4:58784778-58785720 | 15496 | Olfr39 | NM_146825.2 | chr9:20282350-20286648 |
| 15400 | Olfr27 | NM_146829.2 | chr9:39128166-39145070 | 15497 | Olfr390 | NM_146347.1 | chr11:73786939-73787875 |
| 15401 | Olfr270 | NM_146607.1 | chr4:52970628-52971567 | 15498 | Olfr391-ps | NM_001159775.1 | chr11:73798752-73802984 |
| 15402 | Olfr272 | NM_146839.1 | chr4:52910832-52911792 | 15499 | Olfr392 | NM_147006.2 | chr11:73814141-73816877 |
| 15403 | Olfr273 | NM_146824.1 | chr4:52855557-52856511 | 15500 | Olfr393 | NM_147008.2 | chr11:73847184-73848123 |
| 15404 | Olfr275 | NM_146858.2 | chr4:52825398-52826358 | 15501 | Olfr394 | NM_147007.1 | chr11:73887437-73888370 |
| 15405 | Olfr279 | NM_001001807.1 | chr15:98497473-98498406 | 15502 | Olfr395 | NM_147005.1 | chr11:73906551-73907490 |
| 15406 | Olfr281 | NM_146280.1 | chr15:98456311-98457247 | 15503 | Olfr397 | NM_146346.1 | chr11:73964609-73965557 |
| 15407 | Olfr282 | NM_146457.2 | chr15:98437469-98438397 | 15504 | Olfr398 | NM_146710.1 | chr11:73983661-73984606 |
| 15408 | Olfr283 | NM_147036.1 | chr15:98378178-98379108 | 15505 | Olfr399 | NM_147004.2 | chr11:74053775-74054778 |
| 15409 | Olfr284 | NM_146281.1 | chr15:98340021-98340939 | 15506 | Olfr401 | NM_146706.1 | chr11:74121290-74122238 |
| 15410 | Olfr285 | NM_001011778.1 | chr15:98312588-98313548 | 15507 | Olfr402 | NM_146708.1 | chr11:74155155-74156103 |
| 15411 | Olfr286 | NM_001011779.1 | chr15:98226674-98234450 | 15508 | Olfr403 | NM_207622.1 | chr11:74195504-74196446 |
| 15412 | Olfr287 | NM_001011780.1 | chr15:98206970-98221056 | 15509 | Olfr406 | NM_001011863.1 | chr11:74269390-74270362 |
| 15413 | Olfr288 | NM_001011733.2 | chr15:98186012-98195542 | 15510 | Olfr410 | NM_146707.1 | chr11:74334281-74335229 |
| 15414 | Olfr290 | NM_146416.2 | chr7:84915780-84916728 | 15511 | Olfr411 | NM_146709.2 | chr11:74346623-74347672 |
| 15415 | Olfr291 | NM_146415.2 | chr7:84853570-84857318 | 15512 | Olfr412 | NM_001011851.1 | chr11:74364670-74365609 |
| 15416 | Olfr292 | NM_146620.2 | chr7:86688340-86695384 | 15513 | Olfr414 | NM_146761.2 | chr1:174430429-174431384 |
| 15417 | Olfr293 | NM_001011752.1 | chr7:86663663-86664674 | 15514 | Olfr417 | NM_207137.1 | chr1:174368918-174369848 |
| 15418 | Olfr294 | NM_001011750.2 | chr7:86615635-86616643 | 15515 | Olfr418-ps1 | NM_146651.2 | chr1:173270146-173271137 |
| 15419 | Olfr295 | NM_146851.2 | chr7:86585276-86586206 | 15516 | Olfr419 | NM_146715.2 | chr1:174249888-174250976 |
| 15420 | Olfr297 | NM_146618.2 | chr7:86526758-86527691 | 15517 | Olfr420 | NM_146305.2 | chr1:174158721-174159767 |
| 15421 | Olfr298 | NM_001011751.1 | chr7:86488550-86489549 | 15518 | Olfr421-ps1 | NR_047667.1 | chr1:174151480-174152530 |
| 15422 | Olfr299 | NM_001011767.1 | chr7:86465412-86466405 | 15519 | Olfr424 | NM_146721.2 | chr1:174136745-174137693 |

Fig.21 - 81

| | | | |
|---|---|---|---|
| 15520 | Olfr426 | NM_001206926.1 | chr1:174099459-174100410 |
| 15521 | Olfr427 | NM_207158.1 | chr1:174099459-174100407 |
| 15522 | Olfr429 | NM_146722.2 | chr1:174089037-174089980 |
| 15523 | Olfr43 | NM_146711.2 | chr11:74206197-74207289 |
| 15524 | Olfr430 | NM_146718.2 | chr1:174069299-174070253 |
| 15525 | Olfr432 | NM_146716.2 | chr1:174050374-174051313 |
| 15526 | Olfr433 | NM_146717.2 | chr1:174041932-174042936 |
| 15527 | Olfr434 | NM_146369.1 | chr6:43216914-43217880 |
| 15528 | Olfr435 | NM_146653.1 | chr6:43201645-43202587 |
| 15529 | Olfr437 | NM_146296.1 | chr6:43167059-43167992 |
| 15530 | Olfr44 | NM_146830.2 | chr9:39484203-39493988 |
| 15531 | Olfr441 | NM_146655.1 | chr6:43115743-43116676 |
| 15532 | Olfr444 | NM_146656.1 | chr6:42955499-42956432 |
| 15533 | Olfr446 | NM_146295.1 | chr6:42927232-42928159 |
| 15534 | Olfr447 | NM_146988.1 | chr6:42911524-42912457 |
| 15535 | Olfr448 | NM_146273.1 | chr6:42896452-42897385 |
| 15536 | Olfr449 | NM_147064.1 | chr6:42837882-42838818 |
| 15537 | Olfr45 | NM_146963.1 | chr7:140690906-140691842 |
| 15538 | Olfr450 | NM_146445.1 | chr6:42817472-42818405 |
| 15539 | Olfr452 | NM_001011869.1 | chr6:42790040-42790994 |
| 15540 | Olfr453 | NM_001011799.1 | chr6:42744038-42744992 |
| 15541 | Olfr455 | NM_001081301.2 | chr6:42538066-42539020 |
| 15542 | Olfr456 | NM_001011528.2 | chr6:42486162-42487214 |
| 15543 | Olfr457 | NM_146987.1 | chr6:42471234-42472176 |
| 15544 | Olfr458 | NM_146444.1 | chr6:42460075-42461017 |
| 15545 | Olfr459 | NM_146576.1 | chr6:41771352-41772297 |
| 15546 | Olfr46 | NM_146934.2 | chr7:140601339-140611124 |
| 15547 | Olfr460 | NM_146383.1 | chr6:40571387-40572332 |
| 15548 | Olfr461 | NM_146382.1 | chr6:40544035-40544977 |
| 15549 | Olfr462 | NM_146411.2 | chr11:87888958-87889894 |
| 15550 | Olfr463 | NM_146413.2 | chr11:87892986-87893922 |
| 15551 | Olfr464 | NM_146412.2 | chr11:87913919-87915003 |
| 15552 | Olfr466 | NM_146819.2 | chr13:65152225-65153152 |
| 15553 | Olfr467 | NM_001005488.1 | chr7:107814585-107815506 |
| 15554 | Olfr469 | NM_146426.1 | chr7:107822522-107823467 |
| 15555 | Olfr47 | NM_146370.1 | chr6:43235609-43236575 |
| 15556 | Olfr470 | NM_146425.1 | chr7:107844786-107845731 |
| 15557 | Olfr472 | NM_146774.1 | chr7:107902718-107903651 |
| 15558 | Olfr473 | NM_146775.1 | chr7:107933521-107934454 |
| 15559 | Olfr474 | NM_146495.1 | chr7:107954642-107955575 |
| 15560 | Olfr476 | NM_146924.1 | chr7:107967398-107968331 |
| 15561 | Olfr477 | NM_146926.1 | chr7:107990366-107991299 |
| 15562 | Olfr478 | NM_146734.1 | chr7:108031396-108032341 |
| 15563 | Olfr479 | NM_001011742.1 | chr7:108054983-108055967 |
| 15564 | Olfr48 | NM_010990.1 | chr2:89844065-89844971 |
| 15565 | Olfr480 | NM_020291.1 | chr7:108065767-108066796 |
| 15566 | Olfr481 | NM_146925.1 | chr7:108080795-108081734 |
| 15567 | Olfr482 | NM_146733.1 | chr7:108094596-108095568 |
| 15568 | Olfr483 | NM_146735.1 | chr7:108103310-108104258 |
| 15569 | Olfr484 | NM_146499.1 | chr7:108124295-108125261 |
| 15570 | Olfr485 | NM_001011810.2 | chr7:108158917-108159871 |
| 15571 | Olfr486 | NM_146496.1 | chr7:108171797-108172742 |
| 15572 | Olfr487 | NM_001011811.1 | chr7:108211582-108212527 |
| 15573 | Olfr488 | NM_146732.1 | chr7:108255191-108256136 |
| 15574 | Olfr49 | NM_010991.2 | chr14:54281895-54282925 |
| 15575 | Olfr490 | NM_146498.1 | chr7:108286179-108287124 |
| 15576 | Olfr491 | NM_146736.1 | chr7:108316895-108317828 |
| 15577 | Olfr492 | NM_146497.1 | chr7:108322729-108323674 |
| 15578 | Olfr493 | NM_146310.1 | chr7:108346034-108346979 |
| 15579 | Olfr494 | NM_146737.1 | chr7:108367491-108368436 |
| 15580 | Olfr495 | NM_146364.1 | chr7:108395121-108396114 |
| 15581 | Olfr497 | NM_146738.1 | chr7:108422572-108423517 |
| 15582 | Olfr498 | NM_146307.2 | chr7:108465325-108466318 |
| 15583 | Olfr5 | NM_146914.2 | chr7:6480215-6486813 |
| 15584 | Olfr50 | NM_146946.1 | chr2:36793237-36794176 |
| 15585 | Olfr502 | NM_146739.1 | chr7:108523003-108523948 |
| 15586 | Olfr503 | NM_001011527.1 | chr7:108544526-108545498 |
| 15587 | Olfr504 | NM_001011858.1 | chr7:108564836-108565793 |
| 15588 | Olfr506 | NM_001011871.1 | chr7:108612308-108613253 |
| 15589 | Olfr507 | NM_146743.1 | chr7:108621813-108622764 |
| 15590 | Olfr508 | NM_146773.1 | chr7:108629993-108630926 |
| 15591 | Olfr509 | NM_146372.1 | chr7:108645608-108646574 |
| 15592 | Olfr51 | NM_146909.1 | chr11:51006973-51007897 |
| 15593 | Olfr510 | NM_146311.1 | chr7:108667417-108668362 |
| 15594 | Olfr512 | NM_146724.1 | chr7:108713354-108714335 |
| 15595 | Olfr513 | NM_146723.1 | chr7:108754857-108755787 |
| 15596 | Olfr514 | NM_146726.1 | chr7:108825064-108825997 |
| 15597 | Olfr516 | NM_146725.1 | chr7:108845063-108846008 |
| 15598 | Olfr517 | NM_001011846.1 | chr7:108868207-108869152 |
| 15599 | Olfr518 | NM_146306.1 | chr7:108880602-108881604 |
| 15600 | Olfr519 | NM_207160.1 | chr7:108893460-108894405 |
| 15601 | Olfr52 | NM_146583.1 | chr2:86181149-86182109 |
| 15602 | Olfr520 | NM_147063.2 | chr7:99735144-99736095 |
| 15603 | Olfr521 | NM_146356.2 | chr7:99767163-99768129 |
| 15604 | Olfr522 | NM_146952.1 | chr7:140162009-140162948 |
| 15605 | Olfr523 | NM_146518.1 | chr7:140176103-140177057 |
| 15606 | Olfr524 | NM_001011814.1 | chr7:140201811-140202768 |
| 15607 | Olfr525 | NM_146956.1 | chr7:140322700-140323630 |
| 15608 | Olfr527 | NM_001011776.1 | chr7:140335863-140336781 |
| 15609 | Olfr53 | NM_146960.2 | chr7:140646451-140652919 |
| 15610 | Olfr530 | NM_146519.1 | chr7:140372684-140373608 |
| 15611 | Olfr531 | NM_146953.1 | chr7:140400129-140401044 |
| 15612 | Olfr532 | NM_147026.1 | chr7:140418841-140419771 |
| 15613 | Olfr533 | NM_001011815.1 | chr7:140466202-140467165 |
| 15614 | Olfr535 | NM_146954.1 | chr7:140492639-140493578 |
| 15615 | Olfr536 | NM_146520.2 | chr7:140500852-140507318 |
| 15616 | Olfr538 | NM_001011867.1 | chr7:140574154-140575087 |
| 15617 | Olfr539 | NM_146961.1 | chr7:140667288-140668248 |
| 15618 | Olfr54 | NM_010997.1 | chr11:51027003-51027945 |
| 15619 | Olfr541 | NM_146962.1 | chr7:140704252-140705191 |
| 15620 | Olfr543 | NM_001011782.2 | chr7:102476772-102477902 |
| 15621 | Olfr544 | NM_020289.2 | chr7:102484113-102488307 |
| 15622 | Olfr545 | NM_146840.1 | chr7:102493822-102494773 |
| 15623 | Olfr547 | NM_147079.2 | chr7:102534748-102535688 |
| 15624 | Olfr549 | NM_147101.2 | chr7:102554285-102555236 |
| 15625 | Olfr55 | NM_010998.2 | chr17:33176415-33177363 |
| 15626 | Olfr550 | NM_147104.2 | chr7:102578468-102579506 |
| 15627 | Olfr551 | NM_146755.2 | chr7:102587740-102588819 |
| 15628 | Olfr552 | NM_147102.2 | chr7:102604355-102605309 |
| 15629 | Olfr553 | NM_207621.1 | chr7:102614006-102614987 |
| 15630 | Olfr554 | NM_146325.2 | chr7:102640247-102641201 |
| 15631 | Olfr555 | NM_147103.2 | chr7:102658822-102659770 |
| 15632 | Olfr556 | NM_146754.2 | chr7:102669900-102670923 |
| 15633 | Olfr557 | NM_146361.2 | chr7:102698215-102699272 |
| 15634 | Olfr558 | NM_147093.3 | chr7:102702322-102712054 |
| 15635 | Olfr559 | NM_147112.1 | chr7:102723531-102724488 |
| 15636 | Olfr56 | NM_010999.2 | chr11:49050732-49135387 |
| 15637 | Olfr560 | NM_147113.2 | chr7:102752982-102753927 |
| 15638 | Olfr561 | NM_147092.1 | chr7:102774525-102775470 |
| 15639 | Olfr564 | NM_146359.2 | chr7:102803479-102804430 |
| 15640 | Olfr566 | NM_001011536.1 | chr7:102856320-102857271 |
| 15641 | Olfr568 | NM_147091.2 | chr7:102877121-102878063 |
| 15642 | Olfr569 | NM_147088.1 | chr7:102887206-102888151 |
| 15643 | Olfr57 | NM_147041.2 | chr10:79034722-79035802 |
| 15644 | Olfr570 | NM_147110.1 | chr7:102900368-102901307 |
| 15645 | Olfr571 | NM_147085.2 | chr7:102908868-102909837 |
| 15646 | Olfr572 | NM_147089.2 | chr7:102927629-102928586 |
| 15647 | Olfr574 | NM_146360.2 | chr7:102948466-102949507 |
| 15648 | Olfr575 | NM_147114.2 | chr7:102954663-102955620 |
| 15649 | Olfr576 | NM_001001805.2 | chr7:102965101-102966040 |
| 15650 | Olfr577 | NM_147109.1 | chr7:102973051-102973990 |
| 15651 | Olfr578 | NM_147115.1 | chr7:102984220-102985162 |
| 15652 | Olfr58 | NM_011001.2 | chr9:19780282-19784064 |
| 15653 | Olfr582 | NM_147053.1 | chr7:103041480-103042440 |
| 15654 | Olfr583 | NM_146757.1 | chr7:103051299-103052259 |
| 15655 | Olfr584 | NM_147054.1 | chr7:103085519-103086479 |
| 15656 | Olfr585 | NM_147087.2 | chr7:103097742-103098699 |
| 15657 | Olfr586 | NM_147111.1 | chr7:103121828-103122782 |
| 15658 | Olfr589 | NM_147052.1 | chr7:103154791-103155745 |
| 15659 | Olfr59 | NM_011002.2 | chr11:74283808-74289646 |
| 15660 | Olfr591 | NM_001011847.1 | chr7:103172690-103173635 |
| 15661 | Olfr592 | NM_207556.2 | chr7:103186602-103187541 |
| 15662 | Olfr593 | NM_146380.1 | chr7:103211861-103212845 |
| 15663 | Olfr594 | NM_207143.1 | chr7:103219719-103220655 |
| 15664 | Olfr596 | NM_001190381.1 | chr7:103309722-103310661 |
| 15665 | Olfr597 | NM_001011845.2 | chr7:103320412-103321360 |
| 15666 | Olfr598 | NM_001011793.1 | chr7:103328487-103329447 |
| 15667 | Olfr599 | NM_146731.1 | chr7:103338055-103339003 |
| 15668 | Olfr6 | NM_206897.2 | chr7:106955983-106956934 |
| 15669 | Olfr60 | NM_146955.1 | chr7:140345051-140345987 |
| 15670 | Olfr600 | NM_147046.2 | chr7:103345981-103346926 |
| 15671 | Olfr601 | NM_146314.2 | chr7:103358223-103359213 |
| 15672 | Olfr603 | NM_147070.2 | chr7:103383061-103384000 |
| 15673 | Olfr605 | NM_001011854.2 | chr7:103442139-103443150 |
| 15674 | Olfr606 | NM_147094.1 | chr7:103451338-103452298 |
| 15675 | Olfr608 | NM_146756.2 | chr7:103470040-103470991 |
| 15676 | Olfr609 | NM_147082.2 | chr7:103491916-103492876 |
| 15677 | Olfr61 | NM_146964.1 | chr7:140637702-140638638 |
| 15678 | Olfr610 | NM_147081.2 | chr7:103505996-103506944 |
| 15679 | Olfr611 | NM_146727.2 | chr7:103517410-103518382 |
| 15680 | Olfr612 | NM_001200027.1 | chr7:103538260-103539232 |
| 15681 | Olfr613 | NM_147100.3 | chr7:103550367-103555504 |
| 15682 | Olfr615 | NM_147080.2 | chr7:103560478-103561420 |
| 15683 | Olfr616 | NM_147099.2 | chr7:103564323-103565277 |
| 15684 | Olfr617 | NM_146841.1 | chr7:103584023-103584980 |
| 15685 | Olfr618 | NM_147047.2 | chr7:103597317-103598274 |
| 15686 | Olfr619 | NM_147076.2 | chr7:103603588-103604711 |
| 15687 | Olfr62 | NM_146315.2 | chr4:118665518-118666466 |
| 15688 | Olfr620 | NM_146812.2 | chr7:103611409-103612351 |
| 15689 | Olfr622 | NM_147083.1 | chr7:103639184-103640138 |
| 15690 | Olfr623 | NM_147122.2 | chr7:103660243-103661318 |
| 15691 | Olfr624 | NM_001011865.2 | chr7:103670102-103671029 |
| 15692 | Olfr628 | NM_147097.2 | chr7:103731927-103732878 |
| 15693 | Olfr629 | NM_146821.2 | chr7:103740200-103741322 |
| 15694 | Olfr63 | NM_146937.1 | chr17:33268725-33269676 |
| 15695 | Olfr630 | NM_147098.2 | chr7:103754149-103757350 |
| 15696 | Olfr631 | NM_001271020.1 | chr7:103915061-103929911 |
| 15697 | Olfr632 | NM_147119.1 | chr7:103937381-103938335 |
| 15698 | Olfr633 | NM_146354.1 | chr7:103946567-103947506 |
| 15699 | Olfr635 | NM_147118.2 | chr7:103979175-103980141 |
| 15700 | Olfr638 | NM_147120.1 | chr7:104003258-104004224 |
| 15701 | Olfr639 | NM_147084.1 | chr7:104011749-104012700 |
| 15702 | Olfr64 | NM_013617.3 | chr7:103892809-103894471 |
| 15703 | Olfr640 | NM_146822.2 | chr7:104021371-104022316 |
| 15704 | Olfr641 | NM_147072.1 | chr7:104039797-104040736 |
| 15705 | Olfr642 | NM_146329.1 | chr7:104049407-104050352 |
| 15706 | Olfr643 | NM_147077.1 | chr7:104058655-104059600 |
| 15707 | Olfr644 | NM_147121.1 | chr7:104068084-104069029 |
| 15708 | Olfr645 | NM_207144.1 | chr7:104084130-104085078 |
| 15709 | Olfr646 | NM_147056.1 | chr7:104106280-104107219 |
| 15710 | Olfr648 | NM_146751.1 | chr7:104179455-104180406 |
| 15711 | Olfr649 | NM_147055.1 | chr7:104189266-104190205 |
| 15712 | Olfr65 | NM_013616.4 | chr7:103906341-103907447 |
| 15713 | Olfr651 | NM_146813.2 | chr7:104552883-104553952 |

Fig.21 - 82

| | | | |
|---|---|---|---|
| 15714 | Olfr652 | NM_147048.2 | chr7:104564151-104565212 |
| 15715 | Olfr653 | NM_147074.2 | chr7:104579623-104580669 |
| 15716 | Olfr654 | NM_146379.1 | chr7:104587754-104588780 |
| 15717 | Olfr655 | NM_146820.2 | chr7:104596252-104597179 |
| 15718 | Olfr656 | NM_147075.1 | chr7:104617656-104618646 |
| 15719 | Olfr657 | NM_146312.2 | chr7:104635675-104636635 |
| 15720 | Olfr658 | NM_147049.4 | chr7:104642879-104647305 |
| 15721 | Olfr659 | NM_147050.1 | chr7:104670703-104671672 |
| 15722 | Olfr66 | NM_013618.3 | chr7:103881305-103882241 |
| 15723 | Olfr661 | NM_146748.1 | chr7:104688016-104688976 |
| 15724 | Olfr663 | NM_001011757.1 | chr7:104703568-104704603 |
| 15725 | Olfr665 | NM_146814.1 | chr7:104880708-104881659 |
| 15726 | Olfr666 | NM_147096.1 | chr7:104892669-104893626 |
| 15727 | Olfr667 | NM_147060.2 | chr7:104916313-104917294 |
| 15728 | Olfr668 | NM_147059.1 | chr7:104924805-104925762 |
| 15729 | Olfr669 | NM_147043.1 | chr7:104938527-104939481 |
| 15730 | Olfr67 | NM_013619.3 | chr7:103787211-103791830 |
| 15731 | Olfr670 | NM_207146.1 | chr7:104959791-104960730 |
| 15732 | Olfr671 | NM_001011755.1 | chr7:104975041-104975983 |
| 15733 | Olfr672 | NM_146760.1 | chr7:104995963-104996902 |
| 15734 | Olfr675 | NM_001011848.1 | chr7:105024024-105024966 |
| 15735 | Olfr676 | NM_147095.1 | chr7:105035199-105036153 |
| 15736 | Olfr677 | NM_146358.1 | chr7:105056247-105057186 |
| 15737 | Olfr678 | NM_146758.1 | chr7:105069468-105070410 |
| 15738 | Olfr679 | NM_147044.1 | chr7:105085717-105086665 |
| 15739 | Olfr68 | NM_013620.2 | chr7:103777395-103778343 |
| 15740 | Olfr681 | NM_207557.2 | chr7:105121458-105122406 |
| 15741 | Olfr683 | NM_147045.1 | chr7:105143349-105144309 |
| 15742 | Olfr684 | NM_207249.1 | chr7:105156741-105157680 |
| 15743 | Olfr685 | NM_001011857.1 | chr7:105180360-105181311 |
| 15744 | Olfr686 | NM_147069.1 | chr7:105203387-105204341 |
| 15745 | Olfr688 | NM_001011533.2 | chr7:105288094-105289060 |
| 15746 | Olfr689 | NM_146750.1 | chr7:105314005-105314968 |
| 15747 | Olfr69 | NM_013621.3 | chr7:103767276-103771594 |
| 15748 | Olfr690 | NM_020290.2 | chr7:105329216-105330278 |
| 15749 | Olfr691 | NM_147061.1 | chr7:105336745-105337714 |
| 15750 | Olfr692 | NM_146355.1 | chr7:105368327-105369317 |
| 15751 | Olfr693 | NM_146453.2 | chr7:106677533-106678485 |
| 15752 | Olfr694 | NM_146452.2 | chr7:106688781-106689729 |
| 15753 | Olfr695 | NM_146598.2 | chr7:106713731-106716345 |
| 15754 | Olfr697 | NM_146599.2 | chr7:106740907-106741956 |
| 15755 | Olfr698 | NM_146602.2 | chr7:106752450-106753386 |
| 15756 | Olfr699 | NM_001011862.1 | chr7:106790048-106790999 |
| 15757 | Olfr70 | NM_019485.2 | chr4:43694999-43700807 |
| 15758 | Olfr700 | NM_146600.1 | chr7:106805509-106806460 |
| 15759 | Olfr701 | NM_028910.3 | chr7:106814143-106820709 |
| 15760 | Olfr702 | NM_146597.2 | chr7:106823488-106826743 |
| 15761 | Olfr703 | NM_146596.1 | chr7:106844612-106845572 |
| 15762 | Olfr704 | NM_001011749.1 | chr7:106864981-106865929 |
| 15763 | Olfr705 | NM_147032.2 | chr7:106873292-106874243 |
| 15764 | Olfr706 | NM_146353.2 | chr7:106885864-106886815 |
| 15765 | Olfr707 | NM_001005570.2 | chr7:106891183-106892107 |
| 15766 | Olfr71 | NM_019486.1 | chr4:43705627-43706566 |
| 15767 | Olfr710 | NM_146601.1 | chr7:106944066-106948312 |
| 15768 | Olfr711 | NM_147035.2 | chr7:106971367-106975451 |
| 15769 | Olfr713 | NM_147034.1 | chr7:107036135-107037110 |
| 15770 | Olfr714 | NM_147033.2 | chr7:107073829-107074783 |
| 15771 | Olfr715 | NM_146780.2 | chr7:107128351-107129482 |
| 15772 | Olfr716 | NM_146604.1 | chr7:107147317-107148262 |
| 15773 | Olfr720 | NM_146392.1 | chr14:14175129-14176080 |
| 15774 | Olfr722 | NM_146494.2 | chr14:49894257-49901941 |
| 15775 | Olfr723 | NM_001011530.2 | chr14:49928562-49929568 |
| 15776 | Olfr724 | NM_146492.2 | chr14:49960090-49961096 |
| 15777 | Olfr725 | NM_146317.2 | chr14:50034390-50035500 |
| 15778 | Olfr726 | NM_146316.2 | chr14:50083713-50084679 |
| 15779 | Olfr727 | NM_146319.2 | chr14:50126552-50127587 |
| 15780 | Olfr728 | NM_001011809.1 | chr14:50139701-50140637 |
| 15781 | Olfr729 | NM_146278.1 | chr14:50147900-50148872 |
| 15782 | Olfr73 | NM_054090.1 | chr2:88034195-88035137 |
| 15783 | Olfr730 | NM_146493.2 | chr14:50186261-50187218 |
| 15784 | Olfr731 | NM_146363.2 | chr14:50237902-50238883 |
| 15785 | Olfr732 | NM_146665.2 | chr14:50281227-50282271 |
| 15786 | Olfr733 | NM_146663.2 | chr14:50298336-50299330 |
| 15787 | Olfr734 | NM_146664.1 | chr14:50319891-50320833 |
| 15788 | Olfr735 | NM_001011754.2 | chr14:50345375-50346440 |
| 15789 | Olfr736 | NM_146666.1 | chr14:50392757-50393696 |
| 15790 | Olfr738 | NM_146420.2 | chr14:50413545-50414481 |
| 15791 | Olfr739 | NM_146668.2 | chr14:50424520-50425450 |
| 15792 | Olfr74 | NM_054091.2 | chr2:87973706-87974663 |
| 15793 | Olfr740 | NM_146667.2 | chr14:50453053-50453989 |
| 15794 | Olfr741 | NM_207133.2 | chr14:50473056-50486395 |
| 15795 | Olfr742 | NM_146430.2 | chr14:50515120-50516240 |
| 15796 | Olfr743 | NM_001177508.1 | chr14:50533413-50534349 |
| 15797 | Olfr744 | NM_001011738.1 | chr14:50618223-50619195 |
| 15798 | Olfr745 | NM_146299.2 | chr14:50642194-50643369 |
| 15799 | Olfr746 | NM_146298.2 | chr14:50653238-50654183 |
| 15800 | Olfr747 | NM_207156.2 | chr14:50680690-50681645 |
| 15801 | Olfr748 | NM_001011837.2 | chr14:50710331-50711255 |
| 15802 | Olfr749 | NM_020288.2 | chr14:50736218-50744324 |
| 15803 | Olfr750 | NM_207558.2 | chr14:51070310-51071442 |
| 15804 | Olfr75-ps1 | NR_002859.2 | chr11:73405449-73409823 |
| 15805 | Olfr76 | NM_146682.1 | chr19:12119720-12120674 |
| 15806 | Olfr761 | NM_001011829.1 | chr17:37952050-37953022 |
| 15807 | Olfr763 | NM_146862.1 | chr10:129011286-129012216 |
| 15808 | Olfr765 | NM_001085477.1 | chr10:129046131-129047061 |
| 15809 | Olfr767 | NM_146318.2 | chr10:129079031-129079961 |
| 15810 | Olfr768 | NM_146864.1 | chr10:129093033-129093972 |
| 15811 | Olfr769 | NM_146267.1 | chr10:129111484-129112423 |
| 15812 | Olfr77 | NM_146339.3 | chr9:19917228-19922510 |
| 15813 | Olfr770 | NM_146863.1 | chr10:129132830-129133766 |
| 15814 | Olfr771 | NM_146547.1 | chr10:129160028-129160982 |
| 15815 | Olfr772 | NM_146266.2 | chr10:129174050-129175054 |
| 15816 | Olfr773 | NM_207008.2 | chr10:129186483-129187419 |
| 15817 | Olfr774 | NM_207620.1 | chr10:129238150-129239089 |
| 15818 | Olfr775 | NM_146545.2 | chr10:129250535-129251474 |
| 15819 | Olfr776 | NM_207559.1 | chr10:129260962-129261901 |
| 15820 | Olfr777 | NM_146544.1 | chr10:129268385-129269321 |
| 15821 | Olfr78 | NM_001168503.1 | chr7:102740720-102759471 |
| 15822 | Olfr780 | NM_146284.1 | chr10:129321624-129322563 |
| 15823 | Olfr781 | NM_146728.1 | chr10:129332882-129333818 |
| 15824 | Olfr782 | NM_001011797.1 | chr10:129350564-129351509 |
| 15825 | Olfr784 | NM_146729.1 | chr10:129387634-129388594 |
| 15826 | Olfr786 | NM_146549.1 | chr10:129436813-129437752 |
| 15827 | Olfr787 | NM_001011822.1 | chr10:129462677-129463616 |
| 15828 | Olfr788 | NM_146551.1 | chr10:129472693-129473629 |
| 15829 | Olfr790 | NM_146933.1 | chr10:129500885-129501854 |
| 15830 | Olfr791 | NM_146930.1 | chr10:129526228-129527167 |
| 15831 | Olfr792 | NM_001011849.1 | chr10:129540538-129541474 |
| 15832 | Olfr794 | NM_146378.1 | chr10:129570656-129571619 |
| 15833 | Olfr796 | NM_146931.1 | chr10:129607546-129608479 |
| 15834 | Olfr798 | NM_146556.2 | chr10:129625123-129626059 |
| 15835 | Olfr799 | NM_146927.1 | chr10:129647129-129648065 |
| 15836 | Olfr8 | NM_207201.1 | chr10:78955206-78956139 |
| 15837 | Olfr800 | NM_146548.1 | chr10:129659807-129660743 |
| 15838 | Olfr801 | NM_146285.1 | chr10:129669560-129670517 |
| 15839 | Olfr802 | NM_146932.1 | chr10:129681798-129682737 |
| 15840 | Olfr803 | NM_146554.1 | chr10:129691112-129692039 |
| 15841 | Olfr804 | NM_001011821.1 | chr10:129704879-129705824 |
| 15842 | Olfr805 | NM_146555.1 | chr10:129722582-129723542 |
| 15843 | Olfr806 | NM_146553.1 | chr10:129737973-129738915 |
| 15844 | Olfr807 | NM_146929.1 | chr10:129754512-129755448 |
| 15845 | Olfr808 | NM_146928.1 | chr10:129767497-129768436 |
| 15846 | Olfr809 | NM_146324.1 | chr10:129775870-129776857 |
| 15847 | Olfr810 | NM_146550.1 | chr10:129790648-129791587 |
| 15848 | Olfr811 | NM_146552.1 | chr10:129801563-129802523 |
| 15849 | Olfr812 | NM_146795.1 | chr10:129842107-129843040 |
| 15850 | Olfr813 | NM_207147.1 | chr10:129856519-129857452 |
| 15851 | Olfr814 | NM_207159.1 | chr10:129873822-129874755 |
| 15852 | Olfr815 | NM_146670.1 | chr10:129901775-129902726 |
| 15853 | Olfr816 | NM_146672.1 | chr10:129911337-129912276 |
| 15854 | Olfr818 | NM_146777.1 | chr10:129945103-129946060 |
| 15855 | Olfr819 | NM_001165944.1 | chr10:129965735-129966698 |
| 15856 | Olfr820 | NM_146675.1 | chr10:130017362-130018301 |
| 15857 | Olfr821 | NM_146776.1 | chr10:130033627-130034560 |
| 15858 | Olfr822 | NM_146671.1 | chr10:130074411-130075350 |
| 15859 | Olfr823 | NM_146673.2 | chr10:130111840-130112788 |
| 15860 | Olfr824 | NM_146674.1 | chr10:130126107-130127055 |
| 15861 | Olfr825 | NM_146677.1 | chr10:130162361-130163324 |
| 15862 | Olfr826 | NM_146676.1 | chr10:130179936-130180878 |
| 15863 | Olfr827 | NM_146300.1 | chr10:130210159-130211128 |
| 15864 | Olfr828 | NM_146605.1 | chr9:18815353-18816292 |
| 15865 | Olfr829 | NM_147067.1 | chr9:18856626-18857592 |
| 15866 | Olfr830 | NM_146566.1 | chr9:18875328-18876276 |
| 15867 | Olfr832 | NM_001011824.1 | chr9:18944649-18945588 |
| 15868 | Olfr834 | NM_001011823.1 | chr9:18987989-18988928 |
| 15869 | Olfr835 | NM_001012266.1 | chr9:19035124-19036060 |
| 15870 | Olfr836 | NM_146564.2 | chr9:19120956-19121911 |
| 15871 | Olfr837 | NM_146565.2 | chr9:19136927-19137967 |
| 15872 | Olfr843 | NM_146567.2 | chr9:19248356-19249463 |
| 15873 | Olfr845 | NM_207145.2 | chr9:19338458-19339400 |
| 15874 | Olfr846 | NM_146282.2 | chr9:19360414-19361356 |
| 15875 | Olfr847 | NM_146525.1 | chr9:19374940-19375879 |
| 15876 | Olfr849 | NM_146527.1 | chr9:19440914-19441853 |
| 15877 | Olfr850 | NM_146523.1 | chr9:19477300-19478248 |
| 15878 | Olfr851 | NM_146905.2 | chr9:19496749-19497688 |
| 15879 | Olfr853 | NM_146906.1 | chr9:19537010-19537928 |
| 15880 | Olfr854 | NM_146522.1 | chr9:19566434-19567382 |
| 15881 | Olfr855 | NM_146524.2 | chr9:19584510-19585502 |
| 15882 | Olfr856-ps1 | NR_033621.1 | chr9:19657038-19658680 |
| 15883 | Olfr857 | NM_001012265.1 | chr9:19712828-19713758 |
| 15884 | Olfr859 | NM_146526.2 | chr9:19808319-19809249 |
| 15885 | Olfr860 | NM_146528.2 | chr9:19845622-19849747 |
| 15886 | Olfr862 | NM_146562.1 | chr9:19883382-19884303 |
| 15887 | Olfr866 | NM_146558.2 | chr9:20026958-20028035 |
| 15888 | Olfr867 | NM_001011748.1 | chr9:20054465-20055461 |
| 15889 | Olfr868 | NM_146559.2 | chr9:20098625-20101690 |
| 15890 | Olfr869 | NM_146557.2 | chr9:20129119-20138089 |
| 15891 | Olfr870 | NM_146904.1 | chr9:20170633-20171569 |
| 15892 | Olfr871 | NM_146903.2 | chr9:20212206-20213353 |
| 15893 | Olfr872 | NM_146560.2 | chr9:20237161-20260913 |
| 15894 | Olfr873 | NM_146561.1 | chr9:20300210-20301170 |
| 15895 | Olfr874 | NM_146882.2 | chr9:37746135-37747068 |
| 15896 | Olfr875 | NM_146749.2 | chr9:37772636-37773640 |
| 15897 | Olfr876 | NM_146883.2 | chr9:37803888-37804936 |
| 15898 | Olfr877 | NM_146417.1 | chr9:37854819-37855755 |
| 15899 | Olfr878 | NM_146798.2 | chr9:37918547-37919612 |
| 15900 | Olfr881 | NM_146418.2 | chr9:37992491-37993439 |
| 15901 | Olfr883 | NM_146419.2 | chr9:38025807-38026737 |
| 15902 | Olfr884 | NM_001011798.1 | chr9:38047223-38048153 |
| 15903 | Olfr885 | NM_001011739.1 | chr9:38061321-38062251 |
| 15904 | Olfr887 | NM_146423.1 | chr9:38084837-38085767 |
| 15905 | Olfr888 | NM_146424.1 | chr9:38108687-38109632 |
| 15906 | Olfr889 | NM_146482.1 | chr9:38115797-38116727 |
| 15907 | Olfr890 | NM_146481.2 | chr9:38143136-38144081 |

Fig.21 - 83

| | | | |
|---|---|---|---|
| 15908 | Olfr891 | NM_146478.2 | chr9:38179870-38180821 |
| 15909 | Olfr893 | NM_146336.2 | chr9:38209051-38209993 |
| 15910 | Olfr894 | NM_146868.2 | chr9:38218817-38219766 |
| 15911 | Olfr895 | NM_146875.2 | chr9:38268514-38269465 |
| 15912 | Olfr898 | NM_146871.2 | chr9:38349084-38350035 |
| 15913 | Olfr899 | NM_146479.1 | chr9:38367547-38368543 |
| 15914 | Olfr9 | NM_146861.1 | chr10:128989913-128990852 |
| 15915 | Olfr90 | NM_146477.2 | chr17:37085190-37086236 |
| 15916 | Olfr900 | NM_146874.1 | chr9:38377882-38378833 |
| 15917 | Olfr901 | NM_001011806.1 | chr9:38430283-38431219 |
| 15918 | Olfr902 | NM_146802.2 | chr9:38448791-38449847 |
| 15919 | Olfr904 | NM_146801.2 | chr9:38464042-38464975 |
| 15920 | Olfr905 | NM_146804.2 | chr9:38472748-38473681 |
| 15921 | Olfr906 | NM_146803.2 | chr9:38488030-38488966 |
| 15922 | Olfr907 | NM_146805.2 | chr9:38498670-38499603 |
| 15923 | Olfr908 | NM_146872.1 | chr9:38516033-38516965 |
| 15924 | Olfr91 | NM_182714.2 | chr17:37092933-37093872 |
| 15925 | Olfr910 | NM_146811.2 | chr9:38537881-38539829 |
| 15926 | Olfr911-ps1 | NM_146873.2 | chr9:38523729-38524669 |
| 15927 | Olfr912 | NM_146810.2 | chr9:38580243-38582211 |
| 15928 | Olfr913 | NM_001011523.2 | chr9:38592802-38595161 |
| 15929 | Olfr914 | NM_146786.2 | chr9:38606466-38607417 |
| 15930 | Olfr915 | NM_146785.2 | chr9:38646589-38647522 |
| 15931 | Olfr916 | NM_146784.1 | chr9:38657457-38658390 |
| 15932 | Olfr917 | NM_001011864.1 | chr9:38664912-38665842 |
| 15933 | Olfr918 | NM_146375.2 | chr9:38672503-38673481 |
| 15934 | Olfr919 | NM_146440.1 | chr9:38697428-38698376 |
| 15935 | Olfr92 | NM_146456.2 | chr17:37111041-37111980 |
| 15936 | Olfr920 | NM_146787.2 | chr9:38752795-38756863 |
| 15937 | Olfr921 | NM_146782.2 | chr9:38773087-38776354 |
| 15938 | Olfr922 | NM_146781.2 | chr9:38815407-38816539 |
| 15939 | Olfr923 | NM_146816.2 | chr9:38827614-38828682 |
| 15940 | Olfr924 | NM_207560.1 | chr9:38848115-38849042 |
| 15941 | Olfr926 | NM_146815.1 | chr9:38877177-38878104 |
| 15942 | Olfr93 | NM_001011813.1 | chr17:37151031-37151970 |
| 15943 | Olfr930 | NM_146272.1 | chr9:38930172-38931099 |
| 15944 | Olfr933 | NM_146441.1 | chr9:38975677-38976604 |
| 15945 | Olfr934 | NM_146442.1 | chr9:38982109-38983042 |
| 15946 | Olfr935 | NM_146746.1 | chr9:38994506-38995433 |
| 15947 | Olfr936 | NM_207139.1 | chr9:39046613-39047549 |
| 15948 | Olfr937 | NM_146439.1 | chr9:39059728-39060664 |
| 15949 | Olfr938 | NM_146438.1 | chr9:39077795-39078743 |
| 15950 | Olfr94 | NM_001011518.1 | chr17:37196805-37197966 |
| 15951 | Olfr943 | NM_146326.3 | chr9:39166309-39185124 |
| 15952 | Olfr944 | NM_146507.1 | chr9:39217358-39218294 |
| 15953 | Olfr945 | NM_146506.1 | chr9:39257719-39258679 |
| 15954 | Olfr947-ps1 | NR_033620.1 | chr9:39287923-39299481 |
| 15955 | Olfr948 | NM_001011756.1 | chr9:39318646-39319612 |
| 15956 | Olfr95 | NM_146513.1 | chr17:37210912-37211851 |
| 15957 | Olfr951 | NM_001011812.1 | chr9:39393792-39394737 |
| 15958 | Olfr952 | NM_146503.1 | chr9:39426124-39427069 |
| 15959 | Olfr954 | NM_146331.1 | chr9:39461432-39462377 |
| 15960 | Olfr955 | NM_207141.1 | chr9:39469779-39470724 |
| 15961 | Olfr957 | NM_146745.2 | chr9:39510782-39511718 |
| 15962 | Olfr958 | NM_146330.1 | chr9:39549930-39550869 |
| 15963 | Olfr959 | NM_146508.1 | chr9:39572321-39573257 |
| 15964 | Olfr96 | NM_146514.1 | chr17:37225126-37226068 |
| 15965 | Olfr960 | NM_146279.1 | chr9:39623124-39624075 |
| 15966 | Olfr961 | NM_146504.1 | chr9:39646727-39647672 |
| 15967 | Olfr963 | NM_001011827.1 | chr9:39669058-39669994 |
| 15968 | Olfr965 | NM_001011859.1 | chr9:39719228-39720167 |
| 15969 | Olfr967 | NM_001011826.1 | chr9:39750387-39751320 |
| 15970 | Olfr968 | NM_146612.2 | chr9:39771771-39772798 |
| 15971 | Olfr969 | NM_146826.1 | chr9:39795376-39796312 |
| 15972 | Olfr97 | NM_146512.1 | chr17:37231435-37232368 |
| 15973 | Olfr970 | NM_146611.1 | chr9:39819640-39820576 |
| 15974 | Olfr971 | NM_146614.1 | chr9:39839435-39840359 |
| 15975 | Olfr972 | NM_146610.1 | chr9:39873276-39874221 |
| 15976 | Olfr974 | NM_147107.1 | chr9:39942261-39943194 |
| 15977 | Olfr975 | NM_146828.1 | chr9:39949836-39950769 |
| 15978 | Olfr976 | NM_146367.2 | chr9:39954256-39957069 |
| 15979 | Olfr978 | NM_147105.2 | chr9:39993811-39994747 |
| 15980 | Olfr979 | NM_147108.2 | chr9:40000205-40001250 |
| 15981 | Olfr98 | NM_146510.1 | chr17:37262732-37263662 |
| 15982 | Olfr980 | NM_147106.2 | chr9:40005993-40007029 |
| 15983 | Olfr981 | NM_146286.1 | chr9:40022394-40023327 |
| 15984 | Olfr982 | NM_146854.1 | chr9:40074296-40075262 |
| 15985 | Olfr983 | NM_146827.2 | chr9:40092016-40093558 |
| 15986 | Olfr984 | NM_146608.1 | chr9:40100543-40101488 |
| 15987 | Olfr985 | NM_146855.2 | chr9:40126923-40128039 |
| 15988 | Olfr986 | NM_146615.2 | chr9:40187078-40188094 |
| 15989 | Olfr987 | NM_001011785.1 | chr2:85330966-85331896 |
| 15990 | Olfr988 | NM_001011534.1 | chr2:85352994-85353924 |
| 15991 | Olfr99 | NM_146515.2 | chr17:37279500-37280418 |
| 15992 | Olfr992 | NM_146865.1 | chr2:85399601-85400531 |
| 15993 | Olfr993 | NM_146435.1 | chr2:85413932-85414877 |
| 15994 | Olfr994 | NM_146433.1 | chr2:85429882-85430827 |
| 15995 | Olfr995 | NM_146434.1 | chr2:85438208-85439156 |
| 15996 | Olfr996 | NM_146437.2 | chr2:85579214-85580282 |
| 15997 | Olfr998 | NM_146436.2 | chr2:85590509-85591521 |
| 15998 | Olig1 | NM_016968.4 | chr16:91269768-91271939 |
| 15999 | Olig2 | NM_016967.2 | chr16:91225549-91228677 |
| 16000 | Olig3 | NM_053008.2 | chr10:19356538-19358604 |
| 16001 | Olr1 | NM_138648.2 | chr6:129485246-129507165 |
| 16002 | Omal | NM_025909.3 | chr4:103313845-103366428 |
| 16003 | Omd | NM_012050.2 | chr13:49582746-49592609 |
| 16004 | Omg | NM_019409.2 | chr11:79500981-79504082 |
| 16005 | Omp | NM_011010.2 | chr7:98143358-98145447 |
| 16006 | Omt2a | NM_001111286.1 | chr9:78311971-78314048 |
| 16007 | Omt2b | NM_205822.2 | chr9:78328029-78329620 |
| 16008 | Onecut1 | NM_008262.3 | chr9:74861920-74889647 |
| 16009 | Onecut2 | NM_194268.2 | chr18:64340363-64398488 |
| 16010 | Onecut3 | NM_139226.3 | chr10:80494905-80517260 |
| 16011 | Ooep | NM_026480.3 | chr9:78376902-78378588 |
| 16012 | Oog1 | NM_178657.5 | chr12:87602664-87608845 |
| 16013 | Oog2 | NM_198661.3 | chr4:144190716-144196934 |
| 16014 | Oog3 | NM_201258.2 | chr4:144157555-144162651 |
| 16015 | Oog4 | NM_173773.1 | chr4:143437163-143450302 |
| 16016 | Oosp1 | NM_133353.3 | chr19:11667459-11691051 |
| 16017 | Oosp2 | NM_001037634.3 | chr19:11647283-11660559 |
| 16018 | Oosp3 | NM_001033283.2 | chr19:11697054-11711858 |
| 16019 | Opa1 | NM_001199177.1 | chr16:29579333-29654602 |
| 16020 | Opa3 | NM_207525.3 | chr7:19228388-19246817 |
| 16021 | Opalin | NM_153520.1 | chr19:41063419-41077113 |
| 16022 | Opcml | NM_177906.4 | chr9:27791268-28925048 |
| 16023 | Ophn1 | NM_052976.4 | chrX:98554279-98891025 |
| 16024 | Oplah | NM_153122.2 | chr15:76296602-76307245 |
| 16025 | Opn1mw | NM_008106.2 | chrX:74127465-74150755 |
| 16026 | Opn1sw | NM_007538.3 | chr6:29376670-29380513 |
| 16027 | Opn3 | NM_010098.3 | chr1:175662420-175692632 |
| 16028 | Opn4 | NM_001128599.1 | chr14:34590617-34600142 |
| 16029 | Opn5 | NM_181753.4 | chr17:42556782-42611313 |
| 16030 | Oprd1 | NM_013622.3 | chr4:132110725-132144486 |
| 16031 | Oprk1 | NM_001204371.1 | chr1:5588492-5606133 |
| 16032 | Oprl1 | NM_001252565.1 | chr2:181715347-181720985 |
| 16033 | Oprm1 | NM_001039652.2 | chr10:6788575-7038209 |
| 16034 | Optc | NM_001160420.2 | chr1:133897193-133907999 |
| 16035 | Optn | NM_181848.4 | chr2:5020641-5063938 |
| 16036 | Orai1 | NM_175423.3 | chr5:123015073-123030452 |
| 16037 | Orai2 | NM_178751.3 | chr5:136147460-136170656 |
| 16038 | Orai3 | NM_198424.3 | chr7:127769814-127775150 |
| 16039 | Oraov1 | NM_028184.3 | chr7:144915193-144921137 |
| 16040 | Orc1 | NM_011015.2 | chr4:108579453-108614831 |
| 16041 | Orc2 | NM_001025378.2 | chr1:58462770-58501426 |
| 16042 | Orc3 | NM_001159563.1 | chr4:34566780-34614942 |
| 16043 | Orc4 | NM_001177313.1 | chr2:48931746-48949267 |
| 16044 | Orc5 | NM_011959.2 | chr5:22486488-22550331 |
| 16045 | Orc6 | NM_001163791.1 | chr8:85299631-85308279 |
| 16046 | Orm1 | NM_008768.2 | chr4:63344555-63348163 |
| 16047 | Orm2 | NM_011016.2 | chr4:63362448-63365877 |
| 16048 | Orm3 | NM_013623.2 | chr4:63356161-63359511 |
| 16049 | Ormdl1 | NM_145517.4 | chr1:53297094-53310245 |
| 16050 | Ormdl2 | NM_024180.5 | chr10:128817456-128821631 |
| 16051 | Ormdl3 | NM_025661.4 | chr11:98581293-98587245 |
| 16052 | Os9 | NM_001171026.1 | chr10:127094258-127121160 |
| 16053 | Osbp | NM_001033174.1 | chr19:11965843-11994114 |
| 16054 | Osbp2 | NM_152818.2 | chr11:3703730-3863903 |
| 16055 | Osbpl10 | NM_148958.2 | chr9:115067278-115232223 |
| 16056 | Osbpl11 | NM_176840.3 | chr16:33185070-33243312 |
| 16057 | Osbpl1a | NM_001252489.1 | chr18:12755311-12879979 |
| 16058 | Osbpl2 | NM_144500.3 | chr2:180119365-180162680 |
| 16059 | Osbpl3 | NM_001163645.1 | chr6:50293326-50456170 |
| 16060 | Osbpl5 | NM_001199227.1 | chr7:143688761-143740360 |
| 16061 | Osbpl6 | NM_001290733.1 | chr2:76406507-76600647 |
| 16062 | Osbpl7 | NM_001081434.1 | chr11:97050819-97068904 |
| 16063 | Osbpl8 | NM_001003717.1 | chr10:111164801-111297247 |
| 16064 | Osbpl9 | NM_001134791.2 | chr4:109061144-109118036 |
| 16065 | Oscar | NM_001290377.1 | chr7:3609814-3616157 |
| 16066 | Oscp1 | NM_172701.2 | chr4:126058564-126089334 |
| 16067 | Oser1 | NM_025699.2 | chr2:163405821-163419470 |
| 16068 | Osgep | NM_133676.2 | chr14:50915373-50924893 |
| 16069 | Osgepl1 | NM_001285839.1 | chr1:53313623-53326342 |
| 16070 | Osgin1 | NM_027950.1 | chr8:119437161-119446256 |
| 16071 | Osgin2 | NM_172709.3 | chr4:15997120-16013877 |
| 16072 | Osm | NM_001013365.2 | chr11:4236784-4241026 |
| 16073 | Osmr | NM_011019.3 | chr15:6813576-6874313 |
| 16074 | Osr1 | NM_011859.3 | chr12:9574441-9581500 |
| 16075 | Osr2 | NM_054049.2 | chr15:35296111-35303305 |
| 16076 | Ost4 | NM_001134692.2 | chr5:30906515-30907788 |
| 16077 | Ostc | NM_025509.3 | chr3:130695916-130709444 |
| 16078 | Ostf1 | NM_017375.3 | chr19:18580363-18631813 |
| 16079 | Ostm1 | NM_172416.3 | chr10:42678915-42702462 |
| 16080 | Ostn | NM_198112.2 | chr16:27307640-27351209 |
| 16081 | Otc | NM_008769.4 | chrX:10252304-10321024 |
| 16082 | Otoa | NM_139310.1 | chr7:121083437-121163089 |
| 16083 | Otof | NM_001100395.1 | chr5:30367065-30461932 |
| 16084 | Otog | NM_013624.2 | chr7:46240986-46311434 |
| 16085 | Otogl | NM_001177567.1 | chr10:107762309-107912134 |
| 16086 | Otol1 | NM_001018031.2 | chr3:70007612-70028708 |
| 16087 | Otop1 | NM_172709.3 | chr5:38277403-38304217 |
| 16088 | Otop2 | NM_172801.2 | chr11:115307162-115332303 |
| 16089 | Otop3 | NM_027132.2 | chr11:115334733-115346926 |
| 16090 | Otor | NM_020595.2 | chr2:143078491-143081699 |
| 16091 | Otos | NM_153114.2 | chr1:92644217-92648841 |
| 16092 | Otp | NM_011021.3 | chr13:94875626-94883681 |
| 16093 | Ott | NM_011022.1 | chrX:147992992-149487784 |
| 16094 | OTTMUSG00000016609 | NM_001100416.2 | chr2:175323786-176636311 |
| 16095 | Otub1 | NM_134150.2 | chr19:7198205-7206284 |
| 16096 | Otub2 | NM_001177841.1 | chr12:103388681-103406350 |
| 16097 | Otud1 | NM_027715.1 | chr2:19658061-19660590 |
| 16098 | Otud3 | NM_028453.1 | chr4:138895378-138913947 |
| 16099 | Otud4 | NM_001081164.1 | chr8:79639675-79677755 |
| 16100 | Otud5 | NM_001290536.1 | chrX:7841830-7876626 |
| 16101 | Otud6a | NM_001163191.1 | chrX:100429012-100429885 |

Fig.21 - 84

| | | | | | | | |
|---|---|---|---|---|---|---|---|
| 16102 | Otud6b | NM_152812.3 | chr4:14809504-14826587 | 16199 | Pan3 | NM_028291.4 | chr5:147430579-147548501 |
| 16103 | Otud7a | NM_130880.1 | chr7:63444771-63759027 | 16200 | Pank1 | NM_001114339.2 | chr19:34806935-34879455 |
| 16104 | Otud7b | NM_001025613.1 | chr3:96104526-96161129 | 16201 | Pank2 | NM_153501.2 | chr2:131262499-131299188 |
| 16105 | Otulin | NM_001013792.2 | chr15:27605920-27630693 | 16202 | Pank3 | NM_145962.2 | chr11:35769494-35791285 |
| 16106 | Otx1 | NM_011023.3 | chr11:21994763-22001651 | 16203 | Pank4 | NM_172990.5 | chr4:154964122-154980938 |
| 16107 | Otx2 | NM_001286481.1 | chr14:48657676-48667644 | 16204 | Panx1 | NM_019482.2 | chr9:15005784-15045478 |
| 16108 | Otx2os1 | NR_029384.1 | chr14:48669287-48793348 | 16205 | Panx2 | NM_001002005.2 | chr15:89059725-89070907 |
| 16109 | Ovca2 | NM_027136.3 | chr11:75175942-75178808 | 16206 | Panx3 | NM_172454.2 | chr9:37659901-37669222 |
| 16110 | Ovch2 | NM_172908.3 | chr7:107781543-107801179 | 16207 | Paox | NM_153783.4 | chr7:140125684-140134334 |
| 16111 | Ovgp1 | NM_007696.2 | chr3:105973801-105987423 | 16208 | Papd4 | NM_133905.2 | chr13:93147399-93192283 |
| 16112 | Ovol1 | NM_019935.3 | chr19:5549136-5560575 | 16209 | Papd5 | NM_001164497.1 | chr8:88199212-88259722 |
| 16113 | Ovol2 | NM_026924.3 | chr2:144305175-144332080 | 16210 | Papd7 | NM_001169131.1 | chr13:69497958-69533864 |
| 16114 | Ovol3 | NM_001289817.1 | chr7:30233296-30236765 | 16211 | Papl | NM_175319.4 | chr7:28607636-28631015 |
| 16115 | Oxa1l | NM_026936.3 | chr14:54360840-54369669 | 16212 | Papln | NM_001205343.1 | chr12:83763633-83792384 |
| 16116 | Oxct1 | NM_024188.6 | chr15:4026427-4155344 | 16213 | Papola | NM_011112.3 | chr12:105784701-105838943 |
| 16117 | Oxct2a | NM_022033.4 | chr4:123321874-123323679 | 16214 | Papolb | NM_019943.2 | chr5:142527739-142530076 |
| 16118 | Oxct2b | NM_181859.3 | chr4:123116247-123118000 | 16215 | Papolg | NM_172555.2 | chr11:23862645-23895270 |
| 16119 | Oxgr1 | NM_001001490.3 | chr14:120019584-120042435 | 16216 | Pappa | NM_021362.1 | chr4:65124173-65357509 |
| 16120 | Oxld1 | NM_025560.2 | chr11:120456603-120458063 | 16217 | Pappa2 | NM_001085376.2 | chr1:158711730-158957438 |
| 16121 | Oxnad1 | NM_145460.2 | chr14:32085727-32103203 | 16218 | Papss1 | NM_001289477.1 | chr3:131566031-131643671 |
| 16122 | Oxr1 | NM_001130163.1 | chr15:41789514-41861047 | 16219 | Papss2 | NM_001201470.1 | chr19:32595714-32667187 |
| 16123 | Oxsm | NM_027695.3 | chr14:16238658-16249808 | 16220 | Paqr3 | NM_198422.2 | chr5:97082328-97111596 |
| 16124 | Oxsr1 | NM_133985.2 | chr9:119238432-119322427 | 16221 | Paqr4 | NM_023824.3 | chr17:23736185-23740330 |
| 16125 | Oxt | NM_011025.4 | chr2:130576168-130577053 | 16222 | Paqr5 | NM_028748.2 | chr9:61953737-62026790 |
| 16126 | Oxtr | NM_001081147.1 | chr6:112473683-112489808 | 16223 | Paqr6 | NM_198410.3 | chr3:88364588-88368541 |
| 16127 | P2rx1 | NM_008771.3 | chr11:72999144-73015197 | 16224 | Paqr7 | NM_001285845.1 | chr4:134496760-134510237 |
| 16128 | P2rx2 | NM_001164833.1 | chr5:110339811-110343035 | 16225 | Paqr8 | NM_028829.3 | chr1:20890621-20938756 |
| 16129 | P2rx3 | NM_145526.2 | chr2:84996551-85035834 | 16226 | Paqr9 | NM_198414.2 | chr9:95559816-95562121 |
| 16130 | P2rx4 | NM_011026.3 | chr5:122707543-122729738 | 16227 | Pard3 | NM_001013580.3 | chr8:127299651-127402059 |
| 16131 | P2rx5 | NM_033321.3 | chr11:73160529-73172687 | 16228 | Pard3b | NM_001081050.2 | chr1:61638823-62642284 |
| 16132 | P2rx6 | NM_001159561.1 | chr16:17561884-17572012 | 16229 | Pard6a | NM_001047435.2 | chr8:105701646-105703494 |
| 16133 | P2rx7 | NM_001038839.2 | chr5:122643910-122684289 | 16230 | Pard6b | NM_021409.2 | chr2:168081003-168101203 |
| 16134 | P2ry1 | NM_001282016.1 | chr3:61002784-61008982 | 16231 | Pard6g | NM_053117.3 | chr18:80046894-80119640 |
| 16135 | P2ry10 | NM_172435.3 | chrX:107089334-107104970 | 16232 | Parg | NM_011960.2 | chr14:32201970-32297549 |
| 16136 | P2ry12 | NM_027571.3 | chr3:59216270-59262831 | 16233 | Park2 | NM_016694.3 | chr17:10840383-12063360 |
| 16137 | P2ry13 | NM_028808.3 | chr3:59207893-59210882 | 16234 | Park7 | NM_020569.3 | chr4:150897132-150909921 |
| 16138 | P2ry14 | NM_001008497.2 | chr3:59114622-59130762 | 16235 | Parl | NM_001005767.4 | chr16:20279820-20302362 |
| 16139 | P2ry2 | NM_008773.4 | chr7:100996567-101011998 | 16236 | Parm1 | NM_145562.2 | chr5:91517699-91623996 |
| 16140 | P2ry4 | NM_020621.4 | chrX:100590153-100594869 | 16237 | Parn | NM_028761.3 | chr16:13537963-13668170 |
| 16141 | P2ry6 | NM_183168.2 | chr7:100937633-100964366 | 16238 | Parp1 | NM_007415.2 | chr1:180568974-180601254 |
| 16142 | P4ha1 | NM_011030.2 | chr10:59323295-59373304 | 16239 | Parp10 | NM_001163575.1 | chr15:76232994-76243440 |
| 16143 | P4ha2 | NM_001136076.2 | chr11:54100923-54131667 | 16240 | Parp11 | NM_181402.3 | chr6:127453722-127494239 |
| 16144 | P4ha3 | NM_177161.4 | chr7:100285519-100319699 | 16241 | Parp12 | NM_172893.3 | chr6:39086411-39118349 |
| 16145 | P4hb | NM_011032.2 | chr11:120560303-120572936 | 16242 | Parp14 | NM_001039530.3 | chr16:35832877-35871382 |
| 16146 | P4htm | NM_028944.3 | chr9:108578825-108597600 | 16243 | Parp16 | NM_177460.4 | chr9:65214689-65239219 |
| 16147 | Pa2g4 | NM_011119.3 | chr10:128557765-128565934 | 16244 | Parp2 | NM_009632.2 | chr14:50807945-50821300 |
| 16148 | Pabpc1 | NM_008774.3 | chr15:36595657-36608973 | 16245 | Parp3 | NM_145619.3 | chr9:106470321-106476949 |
| 16149 | Pabpc1l | NM_001114079.2 | chr2:164025223-164050538 | 16246 | Parp4 | NM_001145978.2 | chr14:56575618-56659798 |
| 16150 | Pabpc2 | NM_011033.2 | chr18:39773496-39776082 | 16247 | Parp6 | NM_001205239.1 | chr9:59617283-59650290 |
| 16151 | Pabpc4 | NM_130881.2 | chr4:123282910-123298835 | 16248 | Parp8 | NM_001081009.1 | chr13:116854823-117025516 |
| 16152 | Pabpc4l | NM_001101479.1 | chr3:46442196-46447941 | 16249 | Parp9 | NM_030253.3 | chr16:35938469-35972621 |
| 16153 | Pabpc5 | NM_053114.2 | chrX:119927195-119930165 | 16250 | Parpbp | NM_029249.2 | chr10:88091397-88146941 |
| 16154 | Pabpc6 | NM_001163836.1 | chr17:9666496-9669704 | 16251 | Pars2 | NM_001083887.2 | chr4:106651068-106655282 |
| 16155 | Pabpn1 | NM_019402.2 | chr14:54894142-54898927 | 16252 | Parva | NM_020606.5 | chr7:112427705-112591688 |
| 16156 | Pabpn1l | NM_001007462.1 | chr8:122619470-122622735 | 16253 | Parvb | NM_133167.3 | chr15:84232042-84315609 |
| 16157 | Pacrg | NM_027032.2 | chr17:10403011-10840191 | 16254 | Parvg | NM_001162500.1 | chr15:84326118-84342978 |
| 16158 | Pacrgl | NM_025755.3 | chr5:48372391-48388756 | 16255 | Pask | NM_080850.2 | chr1:93309436-93342788 |
| 16159 | Pacs1 | NM_153129.2 | chr19:5133684-5273119 | 16256 | Pate2 | NM_001033421.3 | chr9:35669638-35672889 |
| 16160 | Pacs2 | NM_001081170.1 | chr12:113014507-113074401 | 16257 | Pate4 | NM_020264.4 | chr9:35607092-35611885 |
| 16161 | Pacsin1 | NM_001286743.1 | chr17:27655590-27711117 | 16258 | Patl1 | NM_172635.3 | chr19:11912398-11945096 |
| 16162 | Pacsin2 | NM_001159509.1 | chr15:83375606-83464606 | 16259 | Patl2 | NM_026251.2 | chr2:122120107-122186189 |
| 16163 | Pacsin3 | NM_001289677.1 | chr2:91256164-91264680 | 16260 | Patz1 | NM_001253690.1 | chr11:3290456-3309083 |
| 16164 | Padi1 | NM_011059.2 | chr4:140812980-140845778 | 16261 | Paupar | NR_117095.1 | chr2:105657861-105661343 |
| 16165 | Padi2 | NM_008812.2 | chr4:140906359-140952586 | 16262 | Pawr | NM_054056.2 | chr10:108332188-108414391 |
| 16166 | Padi3 | NM_011060.4 | chr4:140785368-140810648 | 16263 | Pax1 | NM_008780.2 | chr2:147364993-147375048 |
| 16167 | Padi4 | NM_011061.2 | chr4:140745507-140774203 | 16264 | Pax2 | NM_011037.4 | chr19:44757393-44837869 |
| 16168 | Padi6 | NM_153106.2 | chr4:140727354-140742643 | 16265 | Pax3 | NM_001159520.1 | chr1:78101266-78197136 |
| 16169 | Paf1 | NM_019458.3 | chr7:28392995-28399383 | 16266 | Pax4 | NM_001159925.1 | chr6:28442333-28449340 |
| 16170 | Pafah1b1 | NM_013625.4 | chr11:74673948-74724384 | 16267 | Pax5 | NM_008782.2 | chr4:44531505-44710440 |
| 16171 | Pafah1b2 | NM_008775.3 | chr9:45965310-45984871 | 16268 | Pax6 | NM_001244198.2 | chr2:105668895-105698410 |
| 16172 | Pafah1b3 | NM_008776.2 | chr7:25295048-25297955 | 16269 | Pax6os1 | NR_002867.2 | chr2:105536079-105670330 |
| 16173 | Pafah2 | NM_001285872.1 | chr4:134397470-134427412 | 16270 | Pax7 | NM_011039.2 | chr4:139738080-139832968 |
| 16174 | Pag1 | NM_001195031.1 | chr3:9687481-9833679 | 16271 | Pax8 | NM_011040.4 | chr2:24420550-24475599 |
| 16175 | Pagr1a | NM_030240.1 | chr7:127015050-127017352 | 16272 | Pax9 | NM_011041.3 | chr12:56695470-56712822 |
| 16176 | Pah | NM_008777.3 | chr10:87521794-87584137 | 16273 | Paxbp1 | NM_026110.2 | chr16:91014036-91044379 |
| 16177 | Paics | NM_025939.2 | chr5:76951410-76967505 | 16274 | Paxip1 | NM_018878.3 | chr5:27740080-27791550 |
| 16178 | Paip1 | NM_001079849.2 | chr13:119428599-119460323 | 16275 | Pbdc1 | NM_001281871.1 | chrX:105079755-105117090 |
| 16179 | Paip2 | NM_026420.2 | chr18:35598666-35617185 | 16276 | Pbk | NM_023209.2 | chr14:65805910-65817822 |
| 16180 | Paip2b | NM_146169.2 | chr6:83806058-83831741 | 16277 | Pbld1 | NM_026701.2 | chr10:63061616-63077536 |
| 16181 | Pak1 | NM_011035.2 | chr7:97842938-97912381 | 16278 | Pbld2 | NM_026085.2 | chr10:63024511-63058812 |
| 16182 | Pak1ip1 | NM_026550.3 | chr13:41001009-41013033 | 16279 | Pbp2 | NM_029595.3 | chr6:135309128-135310384 |
| 16183 | Pak2 | NM_177326.3 | chr16:32016289-32079342 | 16280 | Pbrm1 | NM_001081251.1 | chr14:31019137-31121592 |
| 16184 | Pak3 | NM_001195046.1 | chrX:143693286-143797796 | 16281 | Pbsn | NM_017471.2 | chrX:77837897-77853500 |
| 16185 | Pak4 | NM_027470.3 | chr7:28558818-28598184 | 16282 | Pbx1 | NM_001291508.1 | chr1:168153526-168432169 |
| 16186 | Pak6 | NM_001033254.3 | chr2:118663576-118698020 | 16283 | Pbx2 | NM_017463.2 | chr17:34592470-34597341 |
| 16187 | Pak7 | NM_172858.2 | chr2:136081087-136387967 | 16284 | Pbx3 | NM_001290576.1 | chr2:34171456-34372045 |
| 16188 | Palb2 | NM_001081238.2 | chr7:122107261-122132946 | 16285 | Pbx4 | NM_001024954.1 | chr8:69832703-69872292 |
| 16189 | Pald1 | NM_013753.2 | chr10:61319656-61383523 | 16286 | Pbxip1 | NM_146131.2 | chr3:89436703-89450952 |
| 16190 | Palld | NM_001081390.1 | chr8:61515020-61902690 | 16287 | Pcbd1 | NM_025273.4 | chr10:61089330-61094329 |
| 16191 | Palm | NM_001161747.1 | chr10:79793571-79820896 | 16288 | Pcbd2 | NM_028281.1 | chr13:55727367-55776830 |
| 16192 | Palm2 | NM_172868.3 | chr4:57568247-57717128 | 16289 | Pcbp1 | NM_011865.3 | chr6:86524496-86526165 |
| 16193 | Palm3 | NM_028877.1 | chr8:84021473-84030295 | 16290 | Pcbp2 | NM_001103165.1 | chr15:102470631-102500059 |
| 16194 | Palmd | NM_023245.3 | chr3:116918261-116968952 | 16291 | Pcbp3 | NM_021568.2 | chr10:76761853-76961947 |
| 16195 | Pam | NM_013626.3 | chr1:97821093-98095632 | 16292 | Pcbp4 | NM_021567.5 | chr9:106453837-106464011 |
| 16196 | Pam16 | NM_025571.1 | chr16:4616465-4624946 | 16293 | Pcca | NM_144844.2 | chr14:122534327-122889944 |
| 16197 | Pamr1 | NM_173749.4 | chr2:102550020-102643040 | 16294 | Pccb | NM_025835.3 | chr9:100982031-101034884 |
| 16198 | Pan2 | NM_001252326.1 | chr10:128303334-128321358 | 16295 | Pcdh1 | NM_029357.3 | chr18:38196693-38209762 |

Fig.21 - 85

| | | | | | | | |
|---|---|---|---|---|---|---|---|
| 16296 | Pcdh10 | NM_001098170.1 | chr3:45378397-45434579 | 16393 | Pcp4 | NM_008791.2 | chr16:96467605-96525793 |
| 16297 | Pcdh11x | NM_001271809.1 | chrX:120290326-120910618 | 16394 | Pcp4l1 | NM_025557.1 | chr1:171173263-171196268 |
| 16298 | Pcdh12 | NM_017378.2 | chr18:38267091-38284401 | 16395 | Pcsk1 | NM_013628.2 | chr13:75089986-75132498 |
| 16299 | Pcdh15 | NM_001142735.1 | chr10:73821866-74634765 | 16396 | Pcsk1n | NM_013892.3 | chrX:7919821-7924410 |
| 16300 | Pcdh17 | NM_001013753.2 | chr14:84443562-84537060 | 16397 | Pcsk2 | NM_008792.4 | chr2:143546132-143816282 |
| 16301 | Pcdh18 | NM_130448.3 | chr3:49743295-49757316 | 16398 | Pcsk2os1 | NR_040354.1 | chr2:143535475-143547837 |
| 16302 | Pcdh19 | NM_001105245.1 | chrX:133582860-133688993 | 16399 | Pcsk2os2 | NR_040625.1 | chr2:143747582-143789186 |
| 16303 | Pcdh20 | NM_178685.5 | chr14:88464746-88471396 | 16400 | Pcsk4 | NM_008793.2 | chr10:80321282-80329473 |
| 16304 | Pcdh7 | NM_001122758.2 | chr5:57718080-58133235 | 16401 | Pcsk5 | NM_001163144.1 | chr19:17558158-17837632 |
| 16305 | Pcdh8 | NM_001042726.3 | chr14:79766771-79771312 | 16402 | Pcsk6 | NM_001291184.1 | chr7:65862135-66050386 |
| 16306 | Pcdh9 | NM_001081377.2 | chr14:93013699-93888888 | 16403 | Pcsk7 | NM_001281934.1 | chr9:45906568-45929722 |
| 16307 | Pcdha1 | NM_054072.1 | chr18:36930284-37187657 | 16404 | Pcsk9 | NM_153565.2 | chr4:106442333-106464325 |
| 16308 | Pcdha10 | NM_009961.1 | chr18:37005319-37187657 | 16405 | Pctp | NM_008796.3 | chr11:89983416-90002894 |
| 16309 | Pcdha11 | NM_009960.1 | chr18:37010857-37187657 | 16406 | Pcx | NM_001162946.1 | chr19:4510471-4621752 |
| 16310 | Pcdha12 | NM_138663.2 | chr18:37020229-37187657 | 16407 | Pcyox1 | NM_025823.4 | chr6:86386005-86397150 |
| 16311 | Pcdha2 | NM_198117.2 | chr18:36939204-37187660 | 16408 | Pcyox1l | NM_172832.4 | chr18:61696836-61707635 |
| 16312 | Pcdha3 | NM_138662.1 | chr18:36946206-37187657 | 16409 | Pcyt1a | NM_001163159.1 | chr16:32431019-32475065 |
| 16313 | Pcdha4 | NM_007766.2 | chr18:36952688-37187657 | 16410 | Pcyt1b | NM_177546.2 | chrX:93654862-93749948 |
| 16314 | Pcdha4-g | NM_001174154.2 | chr18:36952647-37841872 | 16411 | Pcyt2 | NM_024229.2 | chr11:120610086-120617890 |
| 16315 | Pcdha5 | NM_009959.1 | chr18:36960439-37187657 | 16412 | Pdap1 | NM_001033313.3 | chr5:145128769-145140089 |
| 16316 | Pcdha6 | NM_007767.3 | chr18:36967755-37187657 | 16413 | Pdc | NM_001159730.1 | chr1:150319416-150333906 |
| 16317 | Pcdha7 | NM_009957.1 | chr18:36973923-37187657 | 16414 | Pdcd1 | NM_008798.2 | chr1:94038304-94052553 |
| 16318 | Pcdha8 | NM_201243.1 | chr18:36992466-37187657 | 16415 | Pdcd10 | NM_019745.4 | chr3:75516489-75556852 |
| 16319 | Pcdha9 | NM_138661.1 | chr18:36997879-37187657 | 16416 | Pdcd11 | NM_011053.2 | chr19:47090765-47131145 |
| 16320 | Pcdhac1 | NM_001003671.1 | chr18:37090135-37187657 | 16417 | Pdcd1lg2 | NM_021396.2 | chr19:29410918-29472927 |
| 16321 | Pcdhac2 | NM_001003672.1 | chr18:37143968-37187657 | 16418 | Pdcd2 | NM_008799.2 | chr17:15521574-15527301 |
| 16322 | Pcdhb1 | NM_053126.1 | chr18:37264997-37267454 | 16419 | Pdcd2l | NM_026549.3 | chr7:34184496-34196647 |
| 16323 | Pcdhb10 | NM_053135.2 | chr18:37411673-37414514 | 16420 | Pdcd4 | NM_001168491.1 | chr19:53903350-53929861 |
| 16324 | Pcdhb11 | NM_053136.3 | chr18:37421417-37425032 | 16421 | Pdcd5 | NM_019746.4 | chr7:35641984-35647482 |
| 16325 | Pcdhb12 | NM_053137.2 | chr18:37435620-37438654 | 16422 | Pdcd6 | NM_011051.3 | chr13:74303120-74317326 |
| 16326 | Pcdhb13 | NM_053138.2 | chr18:37442516-37446209 | 16423 | Pdcd6ip | NM_001164677.1 | chr9:113651743-113708259 |
| 16327 | Pcdhb14 | NM_053139.3 | chr18:37447656-37451094 | 16424 | Pdcd7 | NM_016688.2 | chr9:65346067-65359643 |
| 16328 | Pcdhb15 | NM_053140.3 | chr18:37473545-37476340 | 16425 | Pdcl | NM_026176.3 | chr2:37350073-37359332 |
| 16329 | Pcdhb16 | NM_053141.3 | chr18:37477767-37483037 | 16426 | Pdcl2 | NM_023508.7 | chr5:76312115-76331156 |
| 16330 | Pcdhb17 | NM_053142.3 | chr18:37485020-37488290 | 16427 | Pdcl3 | NM_026850.4 | chr1:38987813-38997236 |
| 16331 | Pcdhb18 | NM_053143.2 | chr18:37489464-37494503 | 16428 | Pddc1 | NM_172116.4 | chr7:141408183-141414125 |
| 16332 | Pcdhb19 | NM_053144.2 | chr18:37496997-37501711 | 16429 | Pde10a | NM_001290707.1 | chr17:8526800-8986648 |
| 16333 | Pcdhb2 | NM_053127.2 | chr18:37294839-37297614 | 16430 | Pde11a | NM_001081033.1 | chr2:75991131-76338653 |
| 16334 | Pcdhb20 | NM_053145.2 | chr18:37504263-37507661 | 16431 | Pde12 | NM_178668.3 | chr14:26664116-26669846 |
| 16335 | Pcdhb21 | NM_053146.2 | chr18:37513651-37516363 | 16432 | Pde1a | NM_001009978.1 | chr2:79859024-79908399 |
| 16336 | Pcdhb22 | NM_053147.3 | chr18:37518352-37521419 | 16433 | Pde1b | NM_001285890.1 | chr15:103514976-103530056 |
| 16337 | Pcdhb3 | NM_053128.2 | chr18:37300798-37304585 | 16434 | Pde1c | NM_001025568.2 | chr6:56100439-56362392 |
| 16338 | Pcdhb4 | NM_053129.3 | chr18:37307454-37311173 | 16435 | Pde2a | NM_001008548.4 | chr7:101421690-101512829 |
| 16339 | Pcdhb5 | NM_053130.3 | chr18:37320380-37323913 | 16436 | Pde3a | NM_018779.1 | chr6:141249268-141499351 |
| 16340 | Pcdhb6 | NM_053131.1 | chr18:37334027-37336346 | 16437 | Pde3b | NM_011055.2 | chr7:114415253-114537937 |
| 16341 | Pcdhb7 | NM_053132.3 | chr18:37341701-37345207 | 16438 | Pde4a | NM_019798.5 | chr9:21196704-21213248 |
| 16342 | Pcdhb8 | NM_053133.1 | chr18:37355270-37357610 | 16439 | Pde4b | NM_001177980.1 | chr4:102570059-102607262 |
| 16343 | Pcdhb9 | NM_053134.3 | chr18:37400854-37403909 | 16440 | Pde4c | NM_201607.2 | chr8:70724063-70751176 |
| 16344 | Pcdhga1 | NM_033584.1 | chr18:37661944-37841870 | 16441 | Pde4d | NM_011056.3 | chr13:108654176-109955969 |
| 16345 | Pcdhga10 | NM_033593.3 | chr18:37747187-37841870 | 16442 | Pde4dip | NM_001039376.2 | chr3:97689828-97888707 |
| 16346 | Pcdhga11 | NM_033594.2 | chr18:37755772-37841872 | 16443 | Pde5a | NM_153422.2 | chr3:122729157-122859374 |
| 16347 | Pcdhga12 | NM_033595.4 | chr18:37765579-37841872 | 16444 | Pde6a | NM_146086.2 | chr18:61220498-61289718 |
| 16348 | Pcdhga2 | NM_033585.1 | chr18:37669104-37841870 | 16445 | Pde6b | NM_008806.2 | chr5:108388372-108431742 |
| 16349 | Pcdhga3 | NM_033586.2 | chr18:37674334-37841872 | 16446 | Pde6c | NM_001170959.1 | chr19:38132780-38183951 |
| 16350 | Pcdhga4 | NM_033587.3 | chr18:37685399-37841870 | 16447 | Pde6d | NM_008801.2 | chr1:86543014-86582501 |
| 16351 | Pcdhga5 | NM_033588.4 | chr18:37694500-37841870 | 16448 | Pde6g | NM_012065.3 | chr11:120447606-120453500 |
| 16352 | Pcdhga6 | NM_033589.1 | chr18:37707228-37841870 | 16449 | Pde6h | NM_023898.4 | chr6:136954522-136963483 |
| 16353 | Pcdhga7 | NM_033590.3 | chr18:37714833-37841872 | 16450 | Pde7a | NM_001122759.2 | chr3:19223108-19311322 |
| 16354 | Pcdhga8 | NM_033591.3 | chr18:37725705-37841872 | 16451 | Pde7b | NM_013875.5 | chr10:20398003-20725068 |
| 16355 | Pcdhga9 | NM_033592.3 | chr18:37736935-37841863 | 16452 | Pde8a | NM_008803.2 | chr7:81213803-81333622 |
| 16356 | Pcdhgb1 | NM_033574.3 | chr18:37680457-37841870 | 16453 | Pde8b | NM_001170669.1 | chr13:95024104-95223050 |
| 16357 | Pcdhgb2 | NM_033575.3 | chr18:37689858-37841872 | 16454 | Pde9a | NM_001163748.1 | chr17:31386233-31476309 |
| 16358 | Pcdhgb4 | NM_033576.1 | chr18:37720553-37841870 | 16455 | Pdf | NM_026513.2 | chr8:107046289-107048614 |
| 16359 | Pcdhgb5 | NM_033577.1 | chr18:37731153-37841870 | 16456 | Pdgfa | NM_008808.3 | chr5:138976971-138994953 |
| 16360 | Pcdhgb6 | NM_033578.3 | chr18:37742093-37841872 | 16457 | Pdgfb | NM_011057.3 | chr15:79995875-80014808 |
| 16361 | Pcdhgb7 | NM_033579.1 | chr18:37751778-37841870 | 16458 | Pdgfc | NM_019971.2 | chr3:81036415-81214031 |
| 16362 | Pcdhgb8 | NM_033580.2 | chr18:37761800-37841872 | 16459 | Pdgfd | NM_027924.2 | chr9:6168611-6377519 |
| 16363 | Pcdhgc3 | NM_033581.3 | chr18:37806409-37841872 | 16460 | Pdgfra | NM_001083316.1 | chr5:75156165-75198204 |
| 16364 | Pcdhgc4 | NM_033582.2 | chr18:37815078-37841872 | 16461 | Pdgfrb | NM_001146268.1 | chr18:61045149-61085067 |
| 16365 | Pcdhgc5 | NM_033583.3 | chr18:37819545-37841872 | 16462 | Pdgfrl | NM_026840.3 | chr8:40926232-40990770 |
| 16366 | Pced1a | NM_001114541.1 | chr2:130417683-130424641 | 16463 | Pdha1 | NM_008810.3 | chrX:160122218-160138430 |
| 16367 | Pced1b | NM_172293.4 | chr15:97247106-97385691 | 16464 | Pdha2 | NM_008811.2 | chr3:141210003-141212355 |
| 16368 | Pcf11 | NM_029078.3 | chr7:92643711-92669912 | 16465 | Pdhb | NM_024221.3 | chr14:8165990-8172992 |
| 16369 | Pcgf1 | NM_197992.1 | chr6:83078389-83080855 | 16466 | Pdhx | NM_175094.5 | chr2:103021056-103073513 |
| 16370 | Pcgf2 | NM_001163307.1 | chr11:97688822-97699384 | 16467 | Pdia2 | NM_001081070.1 | chr17:26195998-26199087 |
| 16371 | Pcgf3 | NM_172716.4 | chr5:108461331-108503099 | 16468 | Pdia3 | NM_007952.2 | chr2:121413901-121438686 |
| 16372 | Pcgf5 | NM_029508.3 | chr19:36379066-36456204 | 16469 | Pdia4 | NM_009787.2 | chr6:47796140-47813512 |
| 16373 | Pcgf6 | NM_027654.3 | chr19:47033618-47050845 | 16470 | Pdia5 | NM_028295.1 | chr16:35397311-35490873 |
| 16374 | Pcid2 | NM_178708.3 | chr8:13077524-13105343 | 16471 | Pdia6 | NM_027959.3 | chr12:17266594-17284770 |
| 16375 | Pcif1 | NM_146129.3 | chr2:164879367-164891437 | 16472 | Pdik1l | NM_001163794.1 | chr4:134275004-134287253 |
| 16376 | Pck1 | NM_011044.2 | chr2:173153072-173159253 | 16473 | Pdilt | NM_027943.1 | chr7:119486586-119523482 |
| 16377 | Pck2 | NM_028994.2 | chr14:55540265-55550017 | 16474 | Pdk1 | NM_172665.5 | chr2:71873223-71903858 |
| 16378 | Pclo | NM_001110796.2 | chr5:14514917-14796042 | 16475 | Pdk2 | NM_133667.2 | chr11:95026257-95041371 |
| 16379 | Pcm1 | NM_023662.3 | chr8:41239758-41334087 | 16476 | Pdk3 | NM_145630.2 | chrX:93764615-93832095 |
| 16380 | Pcmt1 | NM_008786.2 | chr10:7630235-7663584 | 16477 | Pdk4 | NM_013743.2 | chr6:5483350-5496278 |
| 16381 | Pcmtd1 | NM_183028.3 | chr1:7088919-7173628 | 16478 | Pdlim1 | NM_016861.4 | chr19:40222238-40271616 |
| 16382 | Pcmtd2 | NM_001291211.1 | chr2:181837853-181857460 | 16479 | Pdlim2 | NM_001253736.1 | chr14:70164217-70176815 |
| 16383 | Pcna | NM_011045.2 | chr2:132249285-132253180 | 16480 | Pdlim3 | NM_016798.3 | chr8:45885484-45919546 |
| 16384 | Pcnp | NM_001024622.2 | chr16:56015507-56029717 | 16481 | Pdlim4 | NM_019417.3 | chr11:54054927-54069017 |
| 16385 | Pcnt | NM_001282992.1 | chr10:76351253-76442912 | 16482 | Pdlim5 | NM_001190852.1 | chr3:142239584-142395696 |
| 16386 | Pcnx | NM_018814.3 | chr12:81860029-82000924 | 16483 | Pdlim7 | NM_001114087.2 | chr13:55506772-55513446 |
| 16387 | Pcnxl2 | NM_175561.4 | chr8:125751507-125898317 | 16484 | Pdp1 | NM_001033453.3 | chr4:11958184-11966450 |
| 16388 | Pcnxl3 | NM_144868.3 | chr19:5664634-5688908 | 16485 | Pdp2 | NM_001024606.1 | chr8:104591467-104596849 |
| 16389 | Pcnxl4 | NM_026327.3 | chr12:72536356-72580213 | 16486 | Pdpk1 | NM_001080773.2 | chr17:24073679-24141594 |
| 16390 | Pcolce | NM_008788.2 | chr5:137605106-137611404 | 16487 | Pdpn | NM_001290822.1 | chr4:143267408-143299564 |
| 16391 | Pcolce2 | NM_029620.2 | chr9:95637627-95695551 | 16488 | Pdpr | NM_198308.1 | chr8:111094744-111135144 |
| 16392 | Pcp2 | NM_001129803.1 | chr8:3623373-3625545 | 16489 | Pdrg1 | NM_178939.2 | chr2:153008889-153015383 |

Fig.21 - 86

| | | | | | | | |
|---|---|---|---|---|---|---|---|
| 16490 | Pds5a | NM_001081321.1 | chr5:65615259-65697856 | 16587 | Pgbd1 | NM_001012311.2 | chr13:21421274-21441053 |
| 16491 | Pds5b | NM_175310.6 | chr5:150673826-150810669 | 16588 | Pgbd5 | NM_171824.2 | chr8:124369048-124433936 |
| 16492 | Pdss1 | NM_019501.3 | chr2:22895521-22940259 | 16589 | Pgc | NM_025973.3 | chr17:47726841-47734478 |
| 16493 | Pdss2 | NM_001168289.1 | chr10:43221485-43464882 | 16590 | Pgd | NM_001081274.1 | chr4:149149984-149166707 |
| 16494 | Pdx1 | NM_008814.3 | chr5:147270130-147275149 | 16591 | Pgf | NM_001271705.1 | chr12:85166638-85175982 |
| 16495 | Pdxdc1 | NM_001039533.2 | chr16:13833572-13903145 | 16592 | Pggt1b | NM_172627.3 | chr18:46239948-46280850 |
| 16496 | Pdxk | NM_172134.2 | chr10:78436746-78464948 | 16593 | Pgk1 | NM_008828.3 | chrX:106187099-106203699 |
| 16497 | Pdxk-ps | NR_027316.1 | chr17:32076082-32091423 | 16594 | Pgk2 | NM_031190.2 | chr17:40207017-40208609 |
| 16498 | Pdxp | NM_020271.3 | chr15:78913918-78919517 | 16595 | Pgls | NM_025396.3 | chr8:71592183-71596267 |
| 16499 | Pdyn | NM_001286502.1 | chr2:129686548-129698658 | 16596 | Pglyrp1 | NM_009402.2 | chr7:18884689-18890438 |
| 16500 | Pdzd11 | NM_028303.3 | chrX:100622905-100625907 | 16597 | Pglyrp2 | NM_001271476.1 | chr17:32412460-32424167 |
| 16501 | Pdzd2 | NM_001081064.1 | chr15:12357053-12592556 | 16598 | Pglyrp3 | NM_207247.4 | chr3:92014582-92031584 |
| 16502 | Pdzd3 | NM_133226.2 | chr9:44247311-44251464 | 16599 | Pglyrp4 | NM_001165968.1 | chr3:90726905-90741517 |
| 16503 | Pdzd4 | NM_001029868.2 | chrX:73793356-73824969 | 16600 | Pgm1 | NM_025700.2 | chr5:64092949-64128158 |
| 16504 | Pdzd7 | NM_001195265.1 | chr19:45026906-45045772 | 16601 | Pgm2 | NM_028132.3 | chr4:99929450-99987294 |
| 16505 | Pdzd8 | NM_001033222.1 | chr19:59296083-59345780 | 16602 | Pgm2l1 | NM_027629.3 | chr7:100227606-100278872 |
| 16506 | Pdzd9 | NM_001040136.2 | chr7:120659295-120670343 | 16603 | Pgm3 | NM_001163746.1 | chr9:86552475-86571842 |
| 16507 | Pdzk1 | NM_001146001.1 | chr3:96832673-96870926 | 16604 | Pgm5 | NM_175013.2 | chr19:24678260-24861842 |
| 16508 | Pdzk1ip1 | NM_001164557.1 | chr4:115088707-115093894 | 16605 | Pgp | NM_025954.3 | chr17:24470472-24471596 |
| 16509 | Pdzrn3 | NM_018884.2 | chr6:101149606-101377897 | 16606 | Pgpep1 | NM_023217.4 | chr8:70646435-70659738 |
| 16510 | Pdzrn4 | NM_001164593.1 | chr15:92396809-92771819 | 16607 | Pgpep1l | NM_030101.1 | chr7:68236607-68264233 |
| 16511 | Pea15a | NM_011063.2 | chr1:172196728-172206781 | 16608 | Pgr | NM_008829.2 | chr9:8899832-8968611 |
| 16512 | Pea15b | NR_027806.1 | chr5:77510039-77511003 | 16609 | Pgr15l | NM_001033361.3 | chrX:97072595-97082104 |
| 16513 | Peak1 | NM_172924.3 | chr9:56201128-56418050 | 16610 | Pgrmc1 | NM_016783.4 | chrX:36598192-36606079 |
| 16514 | Pear1 | NM_001032413.1 | chr3:87749096-87768953 | 16611 | Pgrmc2 | NM_027558.1 | chr3:41066325-41083046 |
| 16515 | Pebp1 | NM_018858.2 | chr5:117282650-117287564 | 16612 | Pgs1 | NM_133757.2 | chr11:117986856-118024011 |
| 16516 | Pebp4 | NM_028526.4 | chr14:69840406-69851960 | 16613 | Phactr1 | NM_001005740.1 | chr13:42709580-43138512 |
| 16517 | Pecam1 | NM_001032378.2 | chr11:106654212-106715281 | 16614 | Phactr2 | NM_001033257.4 | chr10:13207716-13324160 |
| 16518 | Pecr | NM_023523.5 | chr1:72259172-72284314 | 16615 | Phactr3 | NM_001007154.3 | chr2:178193083-178338492 |
| 16519 | Pef1 | NM_026441.4 | chr4:130107555-130128134 | 16616 | Phactr4 | NM_001161797.1 | chr4:132355924-132422446 |
| 16520 | Peg10 | NM_130877.2 | chr6:4747305-4760516 | 16617 | Phax | NM_001162989.1 | chr18:56562568-56587712 |
| 16521 | Peg12 | NM_013788.2 | chr7:62461870-62464510 | 16618 | Phb | NM_008831.4 | chr11:95666956-95680773 |
| 16522 | Peg13 | NR_002864.1 | chr15:72805599-72810324 | 16619 | Phb2 | NM_007531.2 | chr6:124712288-124716945 |
| 16523 | Peg3 | NM_008817.2 | chr7:6705959-6730419 | 16620 | Phc1 | NM_001042623.2 | chr6:122317730-122339657 |
| 16524 | Peg3os | NR_023846.1 | chr7:6706759-6707624 | 16621 | Phc2 | NM_001195083.1 | chr4:128727570-128752881 |
| 16525 | Peli1 | NM_023324.2 | chr11:21091323-21150327 | 16622 | Phc3 | NM_001165954.1 | chr3:30899294-30969415 |
| 16526 | Peli2 | NM_033602.2 | chr14:48120868-48260883 | 16623 | Phex | NM_011077.2 | chrX:157162074-157415286 |
| 16527 | Peli3 | NM_172835.3 | chr19:4931854-4943092 | 16624 | Phf1 | NM_009343.3 | chr17:26933126-26937908 |
| 16528 | Pelo | NM_134058.3 | chr13:115088354-115090158 | 16625 | Phf10 | NM_024250.4 | chr17:14944994-14961260 |
| 16529 | Pelp1 | NM_029231.4 | chr11:70392880-70410031 | 16626 | Phf11a | NM_172603.3 | chr14:59276912-59297522 |
| 16530 | Pemt | NM_001290011.1 | chr11:59970613-60046489 | 16627 | Phf11b | NM_001164327.1 | chr14:59320963-59341330 |
| 16531 | Penk | NM_001002927.2 | chr4:4133535-4138445 | 16628 | Phf11c | NM_001164289.1 | chr14:59380832-59393512 |
| 16532 | Peo1 | NM_153796.3 | chr19:45006557-45012762 | 16629 | Phf11d | NM_199015.4 | chr14:59347406-59365490 |
| 16533 | Pepd | NM_008820.2 | chr7:34912406-35044708 | 16630 | Phf12 | NM_174852.3 | chr11:77982815-78030535 |
| 16534 | Per1 | NM_001159367.1 | chr11:69100157-69109957 | 16631 | Phf13 | NM_172705.2 | chr4:151989630-151996179 |
| 16535 | Per2 | NM_011066.3 | chr1:91415981-91459328 | 16632 | Phf14 | NM_001168382.1 | chr6:11925880-12081198 |
| 16536 | Per3 | NM_001289877.1 | chr4:151003654-151044665 | 16633 | Phf19 | NM_028716.4 | chr2:34893754-34913976 |
| 16537 | Peril | NR_110488.1 | chr3:34772074-34782346 | 16634 | Phf2 | NM_011078.3 | chr13:48801749-48870885 |
| 16538 | Perm1 | NM_172417.3 | chr4:156215926-156221307 | 16635 | Phf20 | NM_172674.2 | chr2:156196646-156309953 |
| 16539 | Perp | NM_022032.4 | chr10:18845070-18857072 | 16636 | Phf20l1 | NM_001081409.1 | chr15:66577571-66645255 |
| 16540 | Pes1 | NM_022889.3 | chr11:3963974-3980004 | 16637 | Phf21a | NM_001109690.1 | chr2:92221561-92364666 |
| 16541 | Pet100 | NM_001195244.1 | chr8:3621550-3624235 | 16638 | Phf21b | NM_001081166.2 | chr15:84785375-84856129 |
| 16542 | Pet112 | NM_144896.4 | chr3:85574128-85654470 | 16639 | Phf23 | NM_001291125.1 | chr11:69995765-70000011 |
| 16543 | Pet117 | NM_001164813.1 | chr2:144368982-144373337 | 16640 | Phf3 | NM_001081080.1 | chr1:30802341-30863256 |
| 16544 | Pet2 | NM_008821.2 | chrX:89403847-89409689 | 16641 | Phf5a | NM_026737.3 | chr15:81864515-81871892 |
| 16545 | Pex1 | NM_027777.2 | chr5:3596065-3637230 | 16642 | Phf6 | NM_001290546.1 | chrX:52912213-52956961 |
| 16546 | Pex10 | NM_001042407.1 | chr4:155067029-155072406 | 16643 | Phf7 | NM_027949.1 | chr14:31237695-31251218 |
| 16547 | Pex11a | NM_011068.1 | chr7:79737263-79743025 | 16644 | Phf8 | NM_001113354.1 | chrX:151520671-151633857 |
| 16548 | Pex11b | NM_001162387.1 | chr3:96635429-96645381 | 16645 | Phgdh | NM_016966.3 | chr3:98313170-98339969 |
| 16549 | Pex11g | NM_026951.2 | chr8:3458816-3467648 | 16646 | Phgr1 | NM_001145644.1 | chr2:118772768-118778164 |
| 16550 | Pex12 | NM_134025.3 | chr11:83294644-83298977 | 16647 | Phip | NM_001081216.1 | chr9:82866158-82975489 |
| 16551 | Pex13 | NM_023651.4 | chr11:23646478-23665935 | 16648 | Phka1 | NM_008832.2 | chrX:102513974-102644246 |
| 16552 | Pex14 | NM_019781.2 | chr4:148960534-149099812 | 16649 | Phka2 | NM_001177878.1 | chrX:160502433-160598878 |
| 16553 | Pex16 | NM_145122.2 | chr2:92375238-92381220 | 16650 | Phkb | NM_199446.1 | chr8:85841001-86060642 |
| 16554 | Pex19 | NM_001159525.1 | chr1:172126754-172136497 | 16651 | Phkg1 | NM_011079.2 | chr5:129863434-129879083 |
| 16555 | Pex2 | NM_001163301.2 | chr3:5560187-5576248 | 16652 | Phkg2 | NM_026888.3 | chr7:127573347-127583307 |
| 16556 | Pex26 | NM_028730.6 | chr6:121183666-121196192 | 16653 | Phlda1 | NM_009344.3 | chr10:111506285-111508649 |
| 16557 | Pex3 | NM_001164195.1 | chr10:13523841-13553142 | 16654 | Phlda2 | NM_009434.2 | chr7:143501547-143502524 |
| 16558 | Pex5 | NM_001277330.1 | chr6:124396815-124414863 | 16655 | Phlda3 | NM_013750.2 | chr1:135766084-135769134 |
| 16559 | Pex5l | NM_001163516.3 | chr3:32947925-33143191 | 16656 | Phldb1 | NM_153537.4 | chr9:44686307-44735198 |
| 16560 | Pex6 | NM_145488.1 | chr17:46711462-46725541 | 16657 | Phldb2 | NM_001252442.1 | chr16:45746230-45844378 |
| 16561 | Pex7 | NM_001161825.1 | chr10:19860089-19907674 | 16658 | Phldb3 | NM_001102613.1 | chr7:24611327-24629297 |
| 16562 | Pf4 | NM_019932.4 | chr5:90772434-90773383 | 16659 | Phlpp1 | NM_133821.3 | chr1:106171868-106394245 |
| 16563 | Pfas | NM_001159519.1 | chr11:68985700-69008460 | 16660 | Phlpp2 | NM_001122594.2 | chr8:109868602-109944671 |
| 16564 | Pfdn1 | NM_026027.3 | chr18:36403678-36454495 | 16661 | Phospho1 | NM_153104.3 | chr11:95824499-95832140 |
| 16565 | Pfdn2 | NM_011070.3 | chr1:171345698-171358170 | 16662 | Phospho2 | NM_028521.2 | chr2:69789736-69797168 |
| 16566 | Pfdn4 | NM_001013369.2 | chr2:170496427-170519070 | 16663 | Phox2a | NM_008887.2 | chr7:101818312-101822726 |
| 16567 | Pfdn5 | NM_027044.3 | chr15:102326115-102331489 | 16664 | Phox2b | NM_008888.3 | chr5:67094396-67099126 |
| 16568 | Pfkfb1 | NM_008824.3 | chrX:150589920-150643878 | 16665 | Phpt1 | NM_029293.2 | chr2:25573430-25574871 |
| 16569 | Pfkfb2 | NM_001162415.1 | chr1:130689042-130729253 | 16666 | Phrf1 | NM_001081118.1 | chr7:141228787-141262751 |
| 16570 | Pfkfb3 | NM_001177752.1 | chr2:11471430-11502090 | 16667 | Phtf1 | NM_001163467.1 | chr3:103968109-104007491 |
| 16571 | Pfkfb4 | NM_173019.5 | chr9:108991901-109032225 | 16668 | Phtf1os | NR_030676.1 | chr3:103964535-103968316 |
| 16572 | Pfkl | NM_008826.4 | chr10:77986948-78009796 | 16669 | Phtf2 | NM_172992.3 | chr5:20758663-20882124 |
| 16573 | Pfkm | NM_001163487.1 | chr15:98108470-98132447 | 16670 | Phxr4 | NR_028271.1 | chr9:13431360-13432740 |
| 16574 | Pfkp | NM_001291071.1 | chr13:6579873-6648771 | 16671 | Phyh | NM_010726.2 | chr2:4918995-4938743 |
| 16575 | Pfn1 | NM_011072.4 | chr11:70651846-70654650 | 16672 | Phyhd1 | NM_001252568.2 | chr2:30266202-30282149 |
| 16576 | Pfn2 | NM_019410.3 | chr3:57841894-57847757 | 16673 | Phyhip | NM_145981.3 | chr14:70457516-70468824 |
| 16577 | Pfn3 | NM_029303.2 | chr13:55414687-55415232 | 16674 | Phyhipl | NM_001162846.1 | chr10:70557685-70592812 |
| 16578 | Pfn4 | NM_028376.3 | chr12:4769294-4778266 | 16675 | Phykpl | NM_028398.2 | chr11:51584756-51603269 |
| 16579 | Pfpl | NM_019540.2 | chr19:12427904-12432110 | 16676 | Pi15 | NM_053191.2 | chr1:17601900-17630939 |
| 16580 | Pga5 | NM_021453.4 | chr19:10668956-10678071 | 16677 | Pi16 | NM_023734.3 | chr17:29318881-29328902 |
| 16581 | Pgam1 | NM_023418.2 | chr19:41911870-41918665 | 16678 | Pi4k2a | NM_145501.2 | chr19:42090434-42122218 |
| 16582 | Pgam2 | NM_018870.3 | chr11:5801636-5803796 | 16679 | Pi4k2b | NM_025951.3 | chr5:52741573-52769344 |
| 16583 | Pgam5 | NM_001163538.1 | chr5:110259134-110269899 | 16680 | Pi4ka | NM_001001983.2 | chr16:17280350-17406314 |
| 16584 | Pgap1 | NM_001163314.2 | chr1:54472999-54557684 | 16681 | Pi4kb | NM_175356.3 | chr3:94974730-95006937 |
| 16585 | Pgap2 | NM_001291358.1 | chr7:102210334-102238564 | 16682 | Pianp | NM_001145926.1 | chr6:124998609-125003097 |
| 16586 | Pgap3 | NM_001033537.2 | chr11:98388671-98400490 | 16683 | Pias1 | NM_019663.3 | chr9:62880076-62980879 |

Fig.21 - 87

| | | | | | | | |
|---|---|---|---|---|---|---|---|
| 16684 | Pias2 | NM_001164167.1 | chr18:77065207-77155708 | 16781 | Pitrm1 | NM_145131.1 | chr13:6548156-6580149 |
| 16685 | Pias3 | NM_001165949.1 | chr3:96697075-96706070 | 16782 | Pitx1 | NM_011097.2 | chr13:55825053-55831425 |
| 16686 | Pias4 | NM_021501.4 | chr10:81153965-81167720 | 16783 | Pitx2 | NM_001042502.2 | chr3:129213931-129219594 |
| 16687 | Pibf1 | NM_029320.3 | chr14:99099423-99254494 | 16784 | Pitx3 | NM_008852.4 | chr19:46135685-46148325 |
| 16688 | Picalm | NM_001252520.1 | chr7:90130231-90209447 | 16785 | Piwil1 | NM_021311.3 | chr5:128736245-128755474 |
| 16689 | Pick1 | NM_001045558.1 | chr15:79229381-79249466 | 16786 | Piwil2 | NM_021308.1 | chr14:70372479-70429094 |
| 16690 | Pid1 | NM_001003948.2 | chr1:84036292-84284645 | 16787 | Piwil4 | NM_177905.3 | chr9:14702357-14740733 |
| 16691 | Pidd1 | NM_022654.1 | chr7:141438514-141443355 | 16788 | Pja1 | NM_001083110.2 | chrX:99465733-99470735 |
| 16692 | Piezo1 | NM_001037298.1 | chr8:122481697-122551329 | 16789 | Pja2 | NM_001025309.1 | chr17:64281005-64331883 |
| 16693 | Piezo2 | NM_001039485.4 | chr18:63010212-63387183 | 16790 | Pkd1 | NM_013630.2 | chr17:24549949-24596514 |
| 16694 | Pif1 | NM_172453.3 | chr9:65587204-65595962 | 16791 | Pkd1l2 | NM_029686.4 | chr8:116995678-117082449 |
| 16695 | Pifo | NM_001200028.1 | chr3:105996956-106014646 | 16792 | Pkd1l3 | NM_001039700.2 | chr8:109614516-109672592 |
| 16696 | Piga | NM_011081.2 | chrX:164419786-164433915 | 16793 | Pkd2 | NM_008861.3 | chr5:104459456-104505819 |
| 16697 | Pigb | NM_018889.3 | chr9:73015695-73039699 | 16794 | Pkd2l1 | NM_181422.3 | chr19:44147636-44192442 |
| 16698 | Pigc | NM_001039045.1 | chr1:161969187-161973435 | 16795 | Pkd2l2 | NM_001163004.1 | chr18:34409422-34441794 |
| 16699 | Pigf | NM_008838.1 | chr17:86997258-87025401 | 16796 | Pkdcc | NM_134117.2 | chr17:83215282-83225069 |
| 16700 | Pigg | NM_001081234.2 | chr5:108312924-108349355 | 16797 | Pkdrej | NM_011105.2 | chr15:85814675-85821733 |
| 16701 | Pigh | NM_029988.2 | chr12:79080669-79089670 | 16798 | Pkhd1 | NM_153179.3 | chr1:20057778-20618057 |
| 16702 | Pigk | NM_025662.5 | chr3:152714099-152789013 | 16799 | Pkhd1l1 | NM_138674.2 | chr15:44457552-44597135 |
| 16703 | Pigl | NM_001039536.2 | chr11:62458459-62513900 | 16800 | Pkia | NM_008862.3 | chr3:7366603-7445365 |
| 16704 | Pigm | NM_026234.4 | chr1:172376530-172384099 | 16801 | Pkib | NM_001039050.1 | chr10:57650980-57741112 |
| 16705 | Pign | NM_013784.3 | chr1:105518421-105663676 | 16802 | Pkig | NM_001039390.2 | chr2:163658385-163726158 |
| 16706 | Pigo | NM_020035.3 | chr4:43017625-43025774 | 16803 | Pklr | NM_001099779.1 | chr3:89136622-89146594 |
| 16707 | Pigp | NM_001159616.1 | chr16:94364451-94370710 | 16804 | Pkm | NM_001253883.1 | chr9:59656367-59679375 |
| 16708 | Pigq | NM_001291025.1 | chr17:25926419-25941989 | 16805 | Pkmyt1 | NM_023058.3 | chr17:23726335-23736729 |
| 16709 | Pigr | NM_011082.3 | chr1:130826683-130852249 | 16806 | Pkn1 | NM_001199593.1 | chr8:83669761-83694061 |
| 16710 | Pigs | NM_201406.1 | chr11:78328421-78342776 | 16807 | Pkn2 | NM_178654.4 | chr3:142790901-142882004 |
| 16711 | Pigt | NM_133779.2 | chr2:164497524-164508301 | 16808 | Pkn3 | NM_153805.1 | chr2:30078765-30091019 |
| 16712 | Pigu | NM_001004721.1 | chr2:155278251-155357424 | 16809 | Pknox1 | NM_016670.3 | chr17:31564772-31607693 |
| 16713 | Pigv | NM_001145955.1 | chr4:133661424-133672647 | 16810 | Pknox2 | NM_001029838.2 | chr9:36890978-37147322 |
| 16714 | Pigw | NM_001077636.1 | chr11:84876312-84880285 | 16811 | Pkp1 | NM_019645.3 | chr1:135871393-135919207 |
| 16715 | Pigx | NM_001111025.1 | chr16:32084415-32099727 | 16812 | Pkp2 | NM_026163.2 | chr16:16213344-16272712 |
| 16716 | Pigyl | NM_001082532.1 | chr9:22156845-22158354 | 16813 | Pkp3 | NM_001162924.1 | chr7:141078228-141090510 |
| 16717 | Pigz | NM_172822.3 | chr16:31933850-31946046 | 16814 | Pkp4 | NM_026361.2 | chr2:59160849-59355205 |
| 16718 | Pih1d1 | NM_001278207.1 | chr7:45154302-45160064 | 16815 | Pla1a | NM_134102.4 | chr16:38396116-38433145 |
| 16719 | Pih1d2 | NM_028300.2 | chr9:50617320-50625000 | 16816 | Pla2g10 | NM_001291009.2 | chr16:13715056-13730484 |
| 16720 | Pih1d3 | NM_029062.2 | chr1:31222838-31224287 | 16817 | Pla2g10os | NR_040574.1 | chr16:13729835-13739471 |
| 16721 | Pik3ap1 | NM_031376.3 | chr19:41274217-41385070 | 16818 | Pla2g12a | NM_001286948.1 | chr3:129878605-129895825 |
| 16722 | Pik3c2a | NM_011083.2 | chr7:116337275-116443458 | 16819 | Pla2g12b | NM_023530.2 | chr10:59403684-59421976 |
| 16723 | Pik3c2b | NM_001099276.2 | chr1:133046011-133108688 | 16820 | Pla2g15 | NM_133792.2 | chr8:106150398-106164715 |
| 16724 | Pik3c2g | NM_011084.2 | chr6:139841444-139969283 | 16821 | Pla2g16 | NM_139269.2 | chr19:7557458-7588545 |
| 16725 | Pik3c3 | NM_181414.5 | chr18:30272895-30348120 | 16822 | Pla2g1b | NM_011107.1 | chr5:115466265-115474717 |
| 16726 | Pik3ca | NM_008839.2 | chr3:32436150-32468486 | 16823 | Pla2g2a | NM_001082531.1 | chr4:138831875-138835189 |
| 16727 | Pik3cb | NM_029094.3 | chr9:99038401-99140211 | 16824 | Pla2g2c | NM_008868.3 | chr4:138725324-138744575 |
| 16728 | Pik3cd | NM_001029837.2 | chr4:149649167-149701629 | 16825 | Pla2g2d | NM_011109.2 | chr4:138775734-138782143 |
| 16729 | Pik3cg | NM_001146200.1 | chr12:32173396-32208649 | 16826 | Pla2g2e | NM_012044.2 | chr4:138877941-138882814 |
| 16730 | Pik3ip1 | NM_178149.4 | chr11:3330730-3342971 | 16827 | Pla2g2f | NM_012045.4 | chr4:138750532-138757598 |
| 16731 | Pik3r1 | NM_001024955.2 | chr13:101680760-101692630 | 16828 | Pla2g3 | NM_172791.2 | chr11:3488226-3494166 |
| 16732 | Pik3r2 | NM_008841.2 | chr8:70768180-70776712 | 16829 | Pla2g4a | NM_008869.4 | chr1:149829618-149961290 |
| 16733 | Pik3r3 | NM_181585.5 | chr4:116221913-116303056 | 16830 | Pla2g4b | NM_145378.4 | chr2:120033432-120043032 |
| 16734 | Pik3r4 | NM_001081309.1 | chr9:105642994-105687655 | 16831 | Pla2g4c | NM_001004762.3 | chr7:13329321-13360668 |
| 16735 | Pik3r5 | NM_177320.2 | chr11:68432124-68497846 | 16832 | Pla2g4d | NM_001024137.1 | chr2:120265868-120289069 |
| 16736 | Pik3r6 | NM_001004435.3 | chr11:68503018-68552695 | 16833 | Pla2g4e | NM_177845.4 | chr2:120166411-120245335 |
| 16737 | Pikfyve | NM_011086.2 | chr1:65186684-65278696 | 16834 | Pla2g4f | NM_001024145.2 | chr2:120299956-120314165 |
| 16738 | Pilra | NM_153510.3 | chr5:137821951-137836278 | 16835 | Pla2g5 | NM_001122954.1 | chr4:138799246-138863469 |
| 16739 | Pilrb1 | NM_133209.2 | chr5:137852146-137858049 | 16836 | Pla2g6 | NM_001199023.1 | chr15:79286227-79328371 |
| 16740 | Pilrb2 | NM_001024932.2 | chr5:137865828-137871758 | 16837 | Pla2g7 | NM_013737.5 | chr17:43568450-43612201 |
| 16741 | Pim1 | NM_008842.3 | chr17:29491044-29495459 | 16838 | Pla2r1 | NM_008867.2 | chr2:60417542-60553308 |
| 16742 | Pim2 | NM_138606.2 | chrX:7878305-7883432 | 16839 | Plaa | NM_172695.2 | chr4:94565138-94603247 |
| 16743 | Pim3 | NM_145478.2 | chr15:88862193-88865726 | 16840 | Plac1 | NM_019538.4 | chrX:53070001-53114405 |
| 16744 | Pin1 | NM_023371.3 | chr9:20652129-20666584 | 16841 | Plac8 | NM_139198.2 | chr5:100553732-100572205 |
| 16745 | Pin1rt1 | NM_001033768.2 | chr2:104713925-104716290 | 16842 | Plac8l1 | NM_027072.1 | chr18:42178674-42196709 |
| 16746 | Pin4 | NM_027181.1 | chrX:102119464-102127673 | 16843 | Plac9a | NM_207229.1 | chr14:25888402-26182273 |
| 16747 | Pinc | NR_003202.1 | chr1:73391384-73407569 | 16844 | Plac9b | NM_001270503.1 | chr14:25887933-26182351 |
| 16748 | Pink1 | NM_026880.2 | chr4:138313409-138326296 | 16845 | Plag1 | NM_019969.3 | chr4:3901157-3938405 |
| 16749 | Pinlyp | NM_001037143.2 | chr7:24541657-24546005 | 16846 | Plagl1 | NM_009538.2 | chr10:13090787-13131695 |
| 16750 | Pinx1 | NM_028228.3 | chr14:63860311-63919859 | 16847 | Plagl2 | NM_018807.5 | chr2:153227768-153241358 |
| 16751 | Pip | NM_008843.3 | chr6:41847548-41852062 | 16848 | Plat | NM_008872.3 | chr8:22757721-22782848 |
| 16752 | Pip4k2a | NM_008845.4 | chr2:18842255-18998121 | 16849 | Plau | NM_008873.3 | chr14:20836661-20843388 |
| 16753 | Pip4k2b | NM_054051.1 | chr11:97715156-97744704 | 16850 | Plaur | NM_011113.3 | chr7:24462499-24475873 |
| 16754 | Pip4k2c | NM_054097.3 | chr10:127197066-127211622 | 16851 | Plb1 | NM_001081407.1 | chr5:32239083-32364356 |
| 16755 | Pip5k1a | NM_008847.3 | chr3:95058529-95106930 | 16852 | Plbd1 | NM_025806.2 | chr6:136612070-136661893 |
| 16756 | Pip5k1b | NM_008846.2 | chr19:24294795-24555827 | 16853 | Plbd2 | NM_023625.4 | chr5:120483892-120503623 |
| 16757 | Pip5k1c | NM_001146687.2 | chr10:81292962-81319974 | 16854 | Plcb1 | NM_001145830.1 | chr2:134786163-135475258 |
| 16758 | Pip5kl1 | NM_198191.2 | chr2:32575818-32583779 | 16855 | Plcb2 | NM_001290790.1 | chr2:118707516-118728438 |
| 16759 | Pipox | NM_008952.2 | chr11:77880614-77893872 | 16856 | Plcb3 | NM_001290349.1 | chr19:6953712-6969814 |
| 16760 | Pir | NM_027153.3 | chrX:164269430-164373013 | 16857 | Plcb4 | NM_013829.2 | chr2:135741829-136013068 |
| 16761 | Pira1 | NM_011087.1 | chr7:3836814-3915484 | 16858 | Plcd1 | NM_019676.3 | chr9:119071527-119093502 |
| 16762 | Pira11 | NM_011088.1 | chr7:3838153-3898093 | 16859 | Plcd3 | NM_152813.3 | chr11:103070295-103101658 |
| 16763 | Pira2 | NM_011089.2 | chr7:3836809-3845051 | 16860 | Plcd4 | NM_001081456.1 | chr1:74542888-74565977 |
| 16764 | Pira4 | NM_011091.1 | chr7:3838125-3898092 | 16861 | Plce1 | NM_019588.2 | chr19:38524196-38785100 |
| 16765 | Pira6 | NM_001289428.1 | chr7:3731632-3898150 | 16862 | Plcg1 | NM_021280.3 | chr2:160731309-160775760 |
| 16766 | Pira7 | NM_011094.1 | chr7:3838120-3898120 | 16863 | Plcg2 | NM_172285.1 | chr8:117498290-117635142 |
| 16767 | Pirb | NM_011095.2 | chr7:3712504-3720382 | 16864 | Plch1 | NM_001177732.1 | chr3:63696233-63850991 |
| 16768 | Pirt | NM_178656.3 | chr11:66911990-66928700 | 16865 | Plch2 | NM_001113360.2 | chr4:154983114-155010984 |
| 16769 | Pisd | NM_177298.3 | chr5:32736313-32785626 | 16866 | Plcl1 | NM_001114663.1 | chr1:55405945-55754285 |
| 16770 | Pisd-ps1 | NR_003517.1 | chr11:3124020-3131944 | 16867 | Plcl2 | NM_013880.3 | chr17:50509546-50688494 |
| 16771 | Pisd-ps2 | NR_003519.3 | chr17:3064317-3084183 | 16868 | Plcxd1 | NM_001281812.1 | chr5:110100558-110105952 |
| 16772 | Pisd-ps3 | NR_003518.2 | chrUn_JH584304:52673-59689 | 16869 | Plcxd2 | NM_001134480.1 | chr16:45959260-46010413 |
| 16773 | Pithd1 | NM_025411.4 | chr4:135975601-135987244 | 16870 | Plcxd3 | NM_177355.3 | chr15:4375490-4575579 |
| 16774 | Pitpna | NM_008850.2 | chr11:75588078-75628804 | 16871 | Plcz1 | NM_054066.4 | chr6:139989721-140041417 |
| 16775 | Pitpnb | NM_019640.5 | chr5:111330696-111388359 | 16872 | Pld1 | NM_001164056.1 | chr3:27938679-28133362 |
| 16776 | Pitpnc1 | NM_145823.2 | chr11:107207891-107470720 | 16873 | Pld2 | NM_008876.3 | chr11:70540271-70558110 |
| 16777 | Pitpnm1 | NM_008851.4 | chr19:4100621-4113965 | 16874 | Pld3 | NM_011116.1 | chr7:27532017-27553112 |
| 16778 | Pitpnm2 | NM_001289472.1 | chr5:124118688-124187182 | 16875 | Pld4 | NM_178911.4 | chr12:112760654-112768986 |
| 16779 | Pitpnm2os1 | NR_045369.1 | chr5:124229724-124237137 | 16876 | Pld5 | NM_001195816.1 | chr1:175962305-176213942 |
| 16780 | Pitpnm3 | NM_001024927.2 | chr11:72047527-72135889 | 16877 | Pld6 | NM_001290283.1 | chr11:59783892-59787657 |

Fig.21 - 88

| | | | | | | | |
|---|---|---|---|---|---|---|---|
| 16878 | Pldi | NR_033616.1 | chr10:60928225-60938132 | 16975 | Pnck | NM_001199351.1 | chrX:73655991-73659854 |
| 16879 | Plec | NM_001163540.1 | chr15:76170973-76206321 | 16976 | Pnisr | NM_025669.1 | chr4:21847582-21876475 |
| 16880 | Plek | NM_019549.2 | chr11:16971205-17008718 | 16977 | Pnkd | NM_001039509.1 | chr1:74285033-74353692 |
| 16881 | Plek2 | NM_013738.3 | chr12:78888696-78906938 | 16978 | Pnkp | NM_001290764.1 | chr7:44857145-44862929 |
| 16882 | Plekha1 | NM_133942.2 | chr7:130865909-130913302 | 16979 | Pnldc1 | NM_001034866.1 | chr17:12888901-12910000 |
| 16883 | Plekha2 | NM_031257.3 | chr8:25039143-25101811 | 16980 | Pnlip | NM_026925.3 | chr19:58670364-58681788 |
| 16884 | Plekha3 | NM_031256.3 | chr2:76675314-76697335 | 16981 | Pnliprp1 | NM_018874.2 | chr19:58728886-58744169 |
| 16885 | Plekha4 | NM_148927.2 | chr7:45526329-45554229 | 16982 | Pnliprp2 | NM_011128.2 | chr19:58759722-58777534 |
| 16886 | Plekha5 | NM_144920.3 | chr6:140424098-140594906 | 16983 | Pnma1 | NM_027438.3 | chr12:84146130-84148489 |
| 16887 | Plekha6 | NM_001160268.1 | chr1:133246096-133303435 | 16984 | Pnma2 | NM_175498.4 | chr14:66911207-66920061 |
| 16888 | Plekha7 | NM_172743.3 | chr7:116123484-116189841 | 16985 | Pnma3 | NM_153169.2 | chrX:73064786-73068191 |
| 16889 | Plekha8 | NM_001001335.2 | chr6:54603146-54645822 | 16986 | Pnma5 | NM_001100461.3 | chrX:73033980-73037103 |
| 16890 | Plekhb1 | NM_001163182.1 | chr7:100643895-100662394 | 16987 | Pnmal1 | NM_001007569.1 | chr7:16959794-16962320 |
| 16891 | Plekhb2 | NM_145516.2 | chr1:34849958-34879585 | 16988 | Pnmal2 | NM_001099636.2 | chr7:16944681-16948828 |
| 16892 | Plekhd1 | NM_001177503.1 | chr12:80692600-80724216 | 16989 | Pnmt | NM_008890.1 | chr11:98386631-98388097 |
| 16893 | Plekhd1os | NR_037995.1 | chr12:80686374-80692466 | 16990 | Pnn | NM_008891.2 | chr12:59066918-59074017 |
| 16894 | Plekhf1 | NM_024413.2 | chr7:38220653-38227994 | 16991 | Pno1 | NM_025443.2 | chr11:17203199-17211589 |
| 16895 | Plekhf2 | NM_175175.4 | chr4:10988661-11007619 | 16992 | Pnoc | NM_001205075.1 | chr14:65400672-65425472 |
| 16896 | Plekhg1 | NM_001033253.3 | chr10:3872666-3967302 | 16993 | Pnp | NM_013632.4 | chr14:50944302-50953412 |
| 16897 | Plekhg2 | NM_001083912.1 | chr7:28359603-28372662 | 16994 | Pnp2 | NM_001123371.2 | chr14:50956140-50964749 |
| 16898 | Plekhg3 | NM_153804.4 | chr12:76533559-76579039 | 16995 | Pnpla1 | NM_001034885.3 | chr17:28858410-28890308 |
| 16899 | Plekhg4 | NM_001081333.1 | chr8:105375380-105382862 | 16996 | Pnpla2 | NM_001163689.1 | chr7:141455187-141460743 |
| 16900 | Plekhg5 | NM_001285999.1 | chr4:152096718-152115404 | 16997 | Pnpla3 | NM_054088.3 | chr15:84167815-84189521 |
| 16901 | Plekhg6 | NM_198604.2 | chr6:125362650-125380504 | 16998 | Pnpla5 | NM_029427.1 | chr15:84112620-84123175 |
| 16902 | Plekhh1 | NM_181073.3 | chr12:79029162-79081655 | 16999 | Pnpla6 | NM_001122818.2 | chr8:3515383-3544267 |
| 16903 | Plekhh2 | NM_177606.4 | chr17:84511894-84622142 | 17000 | Pnpla7 | NM_146251.4 | chr2:24976032-25054072 |
| 16904 | Plekhh3 | NM_146030.2 | chr11:101162679-101171302 | 17001 | Pnpla8 | NM_026164.2 | chr12:44269153-44313435 |
| 16905 | Plekhj1 | NM_023900.2 | chr10:80796098-80798626 | 17002 | Pnpo | NM_134021.2 | chr11:96937815-96944019 |
| 16906 | Plekhm1 | NM_183034.1 | chr11:103365091-103412664 | 17003 | Pnpt1 | NM_027869.1 | chr11:29130750-29161828 |
| 16907 | Plekhm2 | NM_001033150.1 | chr4:141625733-141664115 | 17004 | Pnrc1 | NM_001033225.2 | chr4:33245422-33248787 |
| 16908 | Plekhm3 | NM_001039493.1 | chr1:64789120-64956824 | 17005 | Pnrc2 | NM_026383.3 | chr4:135870925-135873846 |
| 16909 | Plekhn1 | NM_001008233.3 | chr4:156221455-156228542 | 17006 | Poc1a | NM_027354.2 | chr9:106281060-106349891 |
| 16910 | Plekho1 | NM_023320.2 | chr3:95988835-95995839 | 17007 | Poc1b | NM_027740.6 | chr10:99107170-99197988 |
| 16911 | Plekho2 | NM_153119.3 | chr9:65552576-65580087 | 17008 | Poc5 | NM_026173.3 | chr13:96388293-96415587 |
| 16912 | Plekhs1 | NM_001164263.1 | chr19:56461636-56486729 | 17009 | Podn | NM_001285956.1 | chr4:108014792-108030986 |
| 16913 | Plet1 | NM_029639.2 | chr9:50494524-50505639 | 17010 | Podnl1 | NM_001013384.2 | chr8:84125988-84132517 |
| 16914 | Plet1os | NR_040714.1 | chr9:50488798-50504805 | 17011 | Podxl | NM_013723.3 | chr6:31519492-31563937 |
| 16915 | Plg | NM_008877.3 | chr17:12378508-12419384 | 17012 | Podxl2 | NM_176974.3 | chr6:88842557-88874044 |
| 16916 | Plgrkt | NM_026362.2 | chr19:29348676-29361871 | 17013 | Pof1b | NM_181579.1 | chrX:112638426-112698651 |
| 16917 | Plin1 | NM_001113471.1 | chr7:79721163-79732776 | 17014 | Pofut1 | NM_080463.3 | chr2:153241531-153270249 |
| 16918 | Plin2 | NM_007408.3 | chr4:86656564-86670059 | 17015 | Pofut2 | NM_030262.3 | chr10:77259299-77269586 |
| 16919 | Plin3 | NM_025836.3 | chr17:56278961-56290511 | 17016 | Pogk | NM_001142948.1 | chr1:166393611-166409828 |
| 16920 | Plin4 | NM_020568.3 | chr17:56100590-56109802 | 17017 | Poglut1 | NM_172380.4 | chr16:38525057-38550256 |
| 16921 | Plin5 | NM_001077348.1 | chr17:56111600-56117298 | 17018 | Pogz | NM_001165948.1 | chr3:94837566-94883567 |
| 16922 | Plk1 | NM_011121.4 | chr7:122159434-122169884 | 17019 | Pola1 | NM_008892.2 | chrX:93304765-93632155 |
| 16923 | Plk2 | NM_152804.2 | chr13:110395043-110400843 | 17020 | Pola2 | NM_001164057.1 | chr19:5941104-5964206 |
| 16924 | Plk3 | NM_013807.3 | chr4:117128654-117133963 | 17021 | Polb | NM_011130.2 | chr8:22628118-22653437 |
| 16925 | Plk4 | NM_011495.2 | chr3:40799950-40816883 | 17022 | Pold1 | NM_011131.3 | chr7:44532743-44548815 |
| 16926 | Plk5 | NM_183152.3 | chr10:80356458-80365489 | 17023 | Pold2 | NM_008894.2 | chr11:5872179-5878256 |
| 16927 | Pllp | NM_026385.4 | chr8:94674894-94696242 | 17024 | Pold3 | NM_133692.2 | chr7:100082112-100121500 |
| 16928 | Pln | NM_001141927.1 | chr10:53337685-53345999 | 17025 | Pold4 | NM_027196.4 | chr19:4231892-4233634 |
| 16929 | Plod1 | NM_011122.3 | chr4:147909752-147936776 | 17026 | Poldip2 | NM_026389.3 | chr11:78512295-78522736 |
| 16930 | Plod2 | NM_001142916.1 | chr9:92542222-92608427 | 17027 | Poldip3 | NM_178627.3 | chr15:83125977-83149336 |
| 16931 | Plod3 | NM_011962.3 | chr5:136987018-136996646 | 17028 | Pole | NM_011132.2 | chr5:110286318-110337453 |
| 16932 | Plp1 | NM_001290561.1 | chrX:136822745-136838582 | 17029 | Pole2 | NM_011133.2 | chr12:69201778-69228190 |
| 16933 | Plp2 | NM_019755.5 | chrX:7667940-7671390 | 17030 | Pole3 | NM_021498.2 | chr4:62523800-62525014 |
| 16934 | Plrg1 | NM_016784.3 | chr3:83055537-83072291 | 17031 | Pole4 | NM_025882.3 | chr6:82646711-82652865 |
| 16935 | Pls1 | NM_001033210.3 | chr9:95752641-95845279 | 17032 | Polg | NM_017462.2 | chr7:79449382-79466273 |
| 16936 | Pls3 | NM_001166453.1 | chrX:75785653-75875170 | 17033 | Polg2 | NM_015810.2 | chr11:106768203-106779537 |
| 16937 | Plscr1 | NM_011636.2 | chr9:92250193-92272561 | 17034 | Polh | NM_030715.3 | chr17:46171992-46202625 |
| 16938 | Plscr2 | NM_001195084.1 | chr9:92275601-92297752 | 17035 | Poli | NM_001136090.2 | chr18:70508679-70530321 |
| 16939 | Plscr3 | NM_001168497.1 | chr11:69846371-69852058 | 17036 | Polk | NM_012048.2 | chr13:96480688-96542485 |
| 16940 | Plscr4 | NM_178711.3 | chr9:92457377-92492516 | 17037 | Poll | NM_020032.2 | chr19:45552275-45560543 |
| 16941 | Plscr5 | NM_001195693.1 | chr9:92192935-92209698 | 17038 | Polm | NM_017401.2 | chr11:5827859-5838016 |
| 16942 | Pltp | NM_011125.2 | chr2:164849517-164857708 | 17039 | Poln | NM_001289803.1 | chr5:34007178-34169526 |
| 16943 | Plvap | NM_032398.2 | chr8:71497752-71511769 | 17040 | Polq | NM_001159369.1 | chr16:37011785-37095417 |
| 16944 | Plxdc1 | NM_001163608.1 | chr11:97923236-97986446 | 17041 | Polr1a | NM_009088.3 | chr6:71909052-71979360 |
| 16945 | Plxdc2 | NM_026162.6 | chr2:16356303-16755839 | 17042 | Polr1b | NM_009086.2 | chr2:129100995-129126595 |
| 16946 | Plxna1 | NM_008881.2 | chr6:89316313-89362613 | 17043 | Polr1c | NM_009085.2 | chr17:46243919-46248045 |
| 16947 | Plxna2 | NM_008882.2 | chr1:194619828-194816868 | 17044 | Polr1d | NM_009087.2 | chr5:147077345-147079086 |
| 16948 | Plxna3 | NM_008883.2 | chrX:74329065-74344689 | 17045 | Polr1e | NM_001285800.1 | chr4:45018608-45034279 |
| 16949 | Plxna4 | NM_175750.3 | chr6:32144556-32588192 | 17046 | Polr2a | NM_001291068.1 | chr11:69733998-69758633 |
| 16950 | Plxna4os1 | NR_040277.1 | chr6:32511140-32515576 | 17047 | Polr2b | NM_153798.2 | chr5:77310483-77349328 |
| 16951 | Plxnb1 | NM_172775.2 | chr9:109095435-109119915 | 17048 | Polr2c | NM_009090.5 | chr8:94857449-94864240 |
| 16952 | Plxnb2 | NM_001159521.2 | chr15:89155545-89173951 | 17049 | Polr2d | NM_027002.3 | chr18:31789158-31796642 |
| 16953 | Plxnb3 | NM_019587.2 | chrX:73757102-73772510 | 17050 | Polr2e | NM_025554.2 | chr10:80035952-80039659 |
| 16954 | Plxnc1 | NM_018797.2 | chr10:94790865-94944578 | 17051 | Polr2f | NM_027231.1 | chr15:79141366-79151767 |
| 16955 | Plxnd1 | NM_026376.3 | chr6:115954810-115995005 | 17052 | Polr2g | NM_026329.2 | chr19:8793128-8798557 |
| 16956 | Pm20d1 | NM_178079.3 | chr1:131797394-131818115 | 17053 | Polr2h | NM_145632.2 | chr16:20717825-20722265 |
| 16957 | Pm20d2 | NM_001034867.2 | chr4:33170405-33189737 | 17054 | Polr2i | NM_027259.1 | chr7:30232073-30233387 |
| 16958 | Pmaip1 | NM_021451.2 | chr18:66458603-66465558 | 17055 | Polr2j | NM_011293.2 | chr5:136116690-136122947 |
| 16959 | Pmch | NM_029971.2 | chr10:88091071-88092374 | 17056 | Polr2k | NM_001039368.2 | chr15:36174009-36177012 |
| 16960 | Pmel | NM_021882.4 | chr10:128706257-128720238 | 17057 | Polr2l | NM_025593.1 | chr7:141471859-141475153 |
| 16961 | Pmepa1 | NM_022995.3 | chr2:173224464-173276533 | 17058 | Polr2m | NM_001164793.1 | chr9:71478436-71484958 |
| 16962 | Pmf1 | NM_025928.4 | chr3:88394137-88410397 | 17059 | Polr3a | NM_001081247.1 | chr14:24448693-24487046 |
| 16963 | Pmfbp1 | NM_019938.3 | chr8:109494026-109542642 | 17060 | Polr3b | NM_027423.1 | chr10:84622436-84727178 |
| 16964 | Pmis2 | NR_027848.1 | chr7:30670721-30671605 | 17061 | Polr3c | NM_028925.1 | chr3:96711894-96727439 |
| 16965 | Pml | NM_008884.5 | chr9:58217179-58249786 | 17062 | Polr3d | NM_001164082.1 | chr14:70438747-70443227 |
| 16966 | Pmm1 | NM_001282040.1 | chr15:81951105-81960930 | 17063 | Polr3e | NM_001164096.1 | chr7:120917743-120947432 |
| 16967 | Pmm2 | NM_016881.2 | chr16:8637706-8657524 | 17064 | Polr3f | NM_029763.3 | chr2:144527744-144541779 |
| 16968 | Pmp2 | NM_001030305.2 | chr3:10179850-10183885 | 17065 | Polr3g | NM_001081176.1 | chr13:81673836-81711013 |
| 16969 | Pmp22 | NM_008885.3 | chr11:63131456-63159547 | 17066 | Polr3gl | NM_027241.4 | chr3:96577871-96594181 |
| 16970 | Pmpca | NM_173180.3 | chr2:26389347-26397121 | 17067 | Polr3h | NM_030229.4 | chr15:81915029-81926213 |
| 16971 | Pmpcb | NM_028431.2 | chr5:21737159-21757152 | 17068 | Polr3k | NM_025901.3 | chr2:181864359-181870826 |
| 16972 | Pms1 | NM_153556.2 | chr1:53189188-53297028 | 17069 | Polrmt | NM_172551.3 | chr10:79736124-79746581 |
| 16973 | Pms2 | NM_008886.2 | chr5:143910000-143931756 | 17070 | Pom121 | NM_148932.2 | chr5:135376139-135394546 |
| 16974 | Pmvk | NM_026784.3 | chr3:89459117-89469009 | 17071 | Pom121l12 | NM_001164166.1 | chr11:14599239-14600316 |

Fig.21 - 89

| | | | | | | | |
|---|---|---|---|---|---|---|---|
| 17072 | Pom121l2 | NM_001162928.1 | chr13:21981180-21985903 | 17169 | Ppm1m | NM_026447.4 | chr9:106194952-106199233 |
| 17073 | Pomc | NM_001278581.1 | chr12:3954944-3960643 | 17170 | Ppm1n | NM_177691.3 | chr7:19276806-19280049 |
| 17074 | Pomgnt1 | NM_001290658.1 | chr4:116151430-116159844 | 17171 | Ppme1 | NM_028292.2 | chr7:100326736-100371896 |
| 17075 | Pomgnt2 | NM_001289558.1 | chr9:121981605-121996026 | 17172 | Ppox | NM_008911.2 | chr1:171276991-171281186 |
| 17076 | Pomk | NM_029037.4 | chr8:25980603-25994121 | 17173 | Ppp1ca | NM_031868.2 | chr19:4192173-4195419 |
| 17077 | Pomp | NM_025624.2 | chr5:147860627-147875778 | 17174 | Ppp1cb | NM_172707.3 | chr5:32458969-32493712 |
| 17078 | Pomt1 | NM_145145.1 | chr2:32236682-32255005 | 17175 | Ppp1cc | NM_013636.3 | chr5:122158278-122175269 |
| 17079 | Pomt2 | NM_153415.4 | chr12:87106860-87147902 | 17176 | Ppp1r10 | NM_001163818.1 | chr17:35917195-35932283 |
| 17080 | Pon1 | NM_011134.3 | chr6:5168089-5193946 | 17177 | Ppp1r11 | NM_029632.3 | chr17:36948354-36951792 |
| 17081 | Pon2 | NM_183308.2 | chr6:5264623-5298373 | 17178 | Ppp1r12a | NM_027892.2 | chr10:108162399-108277575 |
| 17082 | Pon3 | NM_173006.1 | chr6:5220851-5256233 | 17179 | Ppp1r12b | NM_001081307.1 | chr1:134765942-134955940 |
| 17083 | Pop1 | NM_026340.3 | chr15:34495310-34530653 | 17180 | Ppp1r12c | NM_029834.3 | chr7:4481520-4501680 |
| 17084 | Pop4 | NM_025390.4 | chr7:38262819-38271348 | 17181 | Ppp1r13b | NM_011625.1 | chr12:111828457-111908055 |
| 17085 | Pop5 | NM_026398.4 | chr5:115235850-115240970 | 17182 | Ppp1r13l | NM_001010836.3 | chr7:19361215-19378533 |
| 17086 | Pop7 | NM_028753.2 | chr5:137501438-137502429 | 17183 | Ppp1r14a | NM_026731.3 | chr7:29289319-29293390 |
| 17087 | Popdc2 | NM_001081984.2 | chr16:38362189-38378216 | 17184 | Ppp1r14b | NM_008889.2 | chr19:6975047-6977324 |
| 17088 | Popdc3 | NM_024286.1 | chr10:45289304-45318450 | 17185 | Ppp1r14c | NM_133485.2 | chr10:3366149-3464975 |
| 17089 | Por | NM_008898.2 | chr5:135689144-135735326 | 17186 | Ppp1r14d | NM_001290796.1 | chr2:119218118-119229865 |
| 17090 | Porcn | NM_016913.4 | chrX:8193845-8206525 | 17187 | Ppp1r15a | NM_008654.2 | chr7:45522916-45526268 |
| 17091 | Postn | NM_001198765.1 | chr3:54361106-54391041 | 17188 | Ppp1r15b | NM_133819.3 | chr1:133131165-133139800 |
| 17092 | Pot1a | NM_133931.4 | chr6:25743734-25809226 | 17189 | Ppp1r16a | NM_033371.2 | chr15:76671679-76694915 |
| 17093 | Pot1b | NM_028370.1 | chr17:55652024-55712628 | 17190 | Ppp1r16b | NM_001159662.1 | chr2:158667134-158766334 |
| 17094 | Poteg | NM_026256.2 | chr8:27447669-27464112 | 17191 | Ppp1r17 | NM_011153.3 | chr6:56017514-56032688 |
| 17095 | Pou1f1 | NM_008849.4 | chr16:65520628-65533981 | 17192 | Ppp1r18 | NM_001146710.1 | chr17:35866127-35875596 |
| 17096 | Pou2af1 | NM_011136.2 | chr9:51213689-51240079 | 17193 | Ppp1r1a | NM_021391.3 | chr15:103530278-103537992 |
| 17097 | Pou2f1 | NM_011137.3 | chr1:165865153-166002634 | 17194 | Ppp1r1b | NM_144828.2 | chr11:98348405-98357796 |
| 17098 | Pou2f2 | NM_001163554.1 | chr7:25091114-25132460 | 17195 | Ppp1r1c | NM_001290743.1 | chr2:79707779-79818546 |
| 17099 | Pou2f3 | NM_011139.2 | chr9:43123938-43205755 | 17196 | Ppp1r2 | NM_025800.3 | chr16:31251540-31275277 |
| 17100 | Pou3f1 | NM_011141.2 | chr4:124657645-124660655 | 17197 | Ppp1r21 | NM_028658.4 | chr17:88530123-88588367 |
| 17101 | Pou3f2 | NM_008899.2 | chr4:22482094-22488366 | 17198 | Ppp1r26 | NM_001005420.1 | chr2:28447940-28455508 |
| 17102 | Pou3f3 | NM_008900.2 | chr1:42697145-42700209 | 17199 | Ppp1r27 | NM_026814.3 | chr11:120549974-120551132 |
| 17103 | Pou3f3os | NR_027826.1 | chr1:42648199-42694825 | 17200 | Ppp1r2-ps3 | NR_003650.1 | chr19:30538875-30539679 |
| 17104 | Pou3f4 | NM_008901.2 | chrX:110814378-110819108 | 17201 | Ppp1r2-ps7 | NR_033731.1 | chrX:22461595-22463543 |
| 17105 | Pou4f1 | NM_011143.4 | chr14:104462324-104467999 | 17202 | Ppp1r2-ps9 | NR_033171.1 | chrX:15110585-15111489 |
| 17106 | Pou4f2 | NM_138944.2 | chr8:78433008-78436652 | 17203 | Ppp1r32 | NM_133689.1 | chr19:10474256-10482897 |
| 17107 | Pou4f3 | NM_138945.2 | chr18:42394596-42396093 | 17204 | Ppp1r35 | NM_027242.4 | chr5:137778917-137780107 |
| 17108 | Pou5f1 | NM_001252452.1 | chr17:35508806-35510777 | 17205 | Ppp1r36 | NM_001163103.1 | chr12:76417598-76439491 |
| 17109 | Pou5f2 | NM_029315.1 | chr13:78024901-78026296 | 17206 | Ppp1r37 | NM_199149.3 | chr7:19530966-19562398 |
| 17110 | Pou6f1 | NM_010127.3 | chr15:100575317-100586365 | 17207 | Ppp1r3a | NM_080464.2 | chr6:14713821-14755274 |
| 17111 | Pou6f2 | NM_175006.2 | chr13:18124959-18382039 | 17208 | Ppp1r3b | NM_177741.3 | chr8:35375740-35388137 |
| 17112 | Pp2d1 | NM_173449.3 | chr17:53507459-53539451 | 17209 | Ppp1r3c | NM_016854.2 | chr19:36731730-36736604 |
| 17113 | Ppa1 | NM_026438.4 | chr10:61648620-61674165 | 17210 | Ppp1r3d | NM_001085501.2 | chr2:178411205-178414463 |
| 17114 | Ppa2 | NM_146141.2 | chr3:133310109-133378235 | 17211 | Ppp1r3e | NM_001167908.1 | chr14:54875596-54877538 |
| 17115 | Ppan | NM_145610.2 | chr9:20888174-20892179 | 17212 | Ppp1r3f | NM_001290574.1 | chrX:7558561-7574281 |
| 17116 | Ppap2a | NM_008247.3 | chr13:112800776-112867894 | 17213 | Ppp1r3fos | NR_029473.1 | chrX:7573599-7581016 |
| 17117 | Ppap2b | NM_080555.2 | chr4:105157346-105232767 | 17214 | Ppp1r3g | NM_029628.1 | chr13:35967905-35970388 |
| 17118 | Ppap2c | NM_015817.3 | chr10:79526423-79533787 | 17215 | Ppp1r42 | NM_145692.2 | chr1:9968622-10013541 |
| 17119 | Ppapdc1a | NM_001080963.1 | chr7:129257093-129391307 | 17216 | Ppp1r7 | NM_023200.2 | chr1:93343644-93367618 |
| 17120 | Ppapdc1b | NM_028000.1 | chr8:25720047-25724887 | 17217 | Ppp1r8 | NM_001290725.1 | chr4:132826923-132843169 |
| 17121 | Ppapdc2 | NM_028922.3 | chr19:28963919-28966801 | 17218 | Ppp1r9a | NM_181595.3 | chr6:4903319-5165661 |
| 17122 | Ppapdc3 | NM_145521.3 | chr2:32095650-32110820 | 17219 | Ppp1r9b | NM_172261.3 | chr11:94991211-95006898 |
| 17123 | Ppara | NM_001113418.1 | chr15:85735563-85806851 | 17220 | Ppp2ca | NM_019411.4 | chr11:52098823-52122749 |
| 17124 | Ppard | NM_011145.3 | chr17:28232753-28301469 | 17221 | Ppp2cb | NM_017374.3 | chr8:33599620-33619804 |
| 17125 | Pparg | NM_001127330.2 | chr6:115360950-115490401 | 17222 | Ppp2r1a | NM_016891.3 | chr17:20945453-20965905 |
| 17126 | Ppargc1a | NM_008904.2 | chr5:51454248-51553921 | 17223 | Ppp2r1b | NM_001034085.2 | chr9:50856923-50894229 |
| 17127 | Ppargc1b | NM_133249.2 | chr18:61298135-61400431 | 17224 | Ppp2r2a | NM_001205188.1 | chr14:67014055-67072471 |
| 17128 | Ppat | NM_172146.2 | chr5:76913248-76951578 | 17225 | Ppp2r2b | NM_028392.3 | chr18:42645220-43059471 |
| 17129 | Ppbp | NM_023785.2 | chr5:90768517-90770060 | 17226 | Ppp2r2c | NM_172994.2 | chr5:36868569-36955078 |
| 17130 | Ppcdc | NM_176831.4 | chr9:57412659-57440114 | 17227 | Ppp2r2cos | NR_045505.1 | chr5:36873594-36876879 |
| 17131 | Ppcs | NM_026494.3 | chr4:119418532-119422420 | 17228 | Ppp2r2d | NM_026391.2 | chr7:138846385-138883056 |
| 17132 | Ppdpf | NM_025598.2 | chr2:181187342-181188504 | 17229 | Ppp2r3a | NM_001161362.3 | chr9:101104988-101251832 |
| 17133 | Ppef1 | NM_011147.1 | chrX:160623093-160719972 | 17230 | Ppp2r3c | NM_021529.3 | chr12:55280813-55303000 |
| 17134 | Ppef2 | NM_011148.3 | chr5:92226973-92253159 | 17231 | Ppp2r3d | NM_001163415.1 | chr9:124474345-124476862 |
| 17135 | Ppfia1 | NM_001033319.2 | chr7:144476754-144553729 | 17232 | Ppp2r4 | NM_138748.5 | chr2:30416049-30447807 |
| 17136 | Ppfia2 | NM_001205341.1 | chr10:106470309-106933468 | 17233 | Ppp2r5a | NM_144880.4 | chr1:191351980-191397041 |
| 17137 | Ppfia3 | NM_029741.2 | chr7:45339125-45367019 | 17234 | Ppp2r5b | NM_198168.3 | chr19:6227766-6235840 |
| 17138 | Ppfia4 | NM_001144855.1 | chr1:134296782-134332928 | 17235 | Ppp2r5c | NM_001081457.2 | chr12:110485738-110583074 |
| 17139 | Ppfibp1 | NM_001170433.1 | chr6:146888493-147032023 | 17236 | Ppp2r5d | NM_009358.3 | chr17:46682990-46705002 |
| 17140 | Ppfibp2 | NM_001163557.1 | chr7:107606843-107748583 | 17237 | Ppp2r5e | NM_012024.2 | chr12:75450880-75596200 |
| 17141 | Pphln1 | NM_001083114.1 | chr15:93398349-93491912 | 17238 | Ppp3ca | NM_008913.5 | chr3:136670065-136937727 |
| 17142 | Ppia | NM_008907.1 | chr11:6415869-6419810 | 17239 | Ppp3cb | NM_008914.3 | chr14:20499363-20546573 |
| 17143 | Ppib | NM_011149.2 | chr9:66060168-66066629 | 17240 | Ppp3cc | NM_008915.3 | chr14:70217864-70289497 |
| 17144 | Ppic | NM_008908.4 | chr18:53406340-53418007 | 17241 | Ppp3r1 | NM_024459.2 | chr11:17159297-17200380 |
| 17145 | Ppid | NM_026352.3 | chr3:79591388-79603650 | 17242 | Ppp3r2 | NM_001004025.4 | chr4:49678746-49681983 |
| 17146 | Ppie | NM_019489.5 | chr4:123127124-123139941 | 17243 | Ppp4c | NM_019674.3 | chr7:126785867-126792471 |
| 17147 | Ppif | NM_134084.1 | chr14:25694169-25700466 | 17244 | Ppp4r1 | NM_001114131.1 | chr17:65783354-65841926 |
| 17148 | Ppifos | NR_028021.1 | chr14:25696355-25701282 | 17245 | Ppp4r1l-ps | NR_027957.1 | chr2:173579319-173659539 |
| 17149 | Ppig | NM_001081086.1 | chr2:69723087-69754059 | 17246 | Ppp4r2 | NM_182939.4 | chr6:100833637-100868717 |
| 17150 | Ppih | NM_001110129.1 | chr4:119306289-119320523 | 17247 | Ppp4r4 | NM_028980.3 | chr12:103532564-103613832 |
| 17151 | Ppil1 | NM_026845.4 | chr17:29250834-29263971 | 17248 | Ppp5c | NM_011155.2 | chr7:17004640-17027914 |
| 17152 | Ppil2 | NM_001252444.1 | chr16:17086555-17111252 | 17249 | Ppp6c | NM_024209.2 | chr2:39196797-39226338 |
| 17153 | Ppil3 | NM_001285826.1 | chr1:58430992-58445486 | 17250 | Ppp6r1 | NM_172894.2 | chr7:4631494-4658950 |
| 17154 | Ppil4 | NM_026141.3 | chr10:7792893-7822563 | 17251 | Ppp6r2 | NM_026813.1 | chr15:89211558-89286261 |
| 17155 | Ppil6 | NM_028430.1 | chr10:41490438-41514288 | 17252 | Ppp6r3 | NM_001164159.1 | chr19:3454928-3575749 |
| 17156 | Ppip5k1 | NM_178795.4 | chr2:121310560-121351013 | 17253 | Pprc1 | NM_001081214.1 | chr19:46056538-46072909 |
| 17157 | Ppip5k2 | NM_173760.5 | chr1:97706042-97770092 | 17254 | Ppt1 | NM_008917.3 | chr4:122836226-122859175 |
| 17158 | Ppl | NM_008909.2 | chr16:5086290-5132481 | 17255 | Ppt2 | NM_019441.5 | chr17:34616661-34627148 |
| 17159 | Ppm1a | NM_008910.3 | chr12:72761210-72794940 | 17256 | Pptc7 | NM_177242.4 | chr5:122284397-122324281 |
| 17160 | Ppm1b | NM_001159496.1 | chr17:84958000-85014776 | 17257 | Ppwd1 | NM_172807.4 | chr13:104205121-104228844 |
| 17161 | Ppm1d | NM_016910.3 | chr11:85311253-85347071 | 17258 | Ppy | NM_008918.1 | chr11:102099930-102101300 |
| 17162 | Ppm1e | NM_177167.4 | chr11:87226905-87358994 | 17259 | Pqbp1 | NM_001252528.1 | chrX:7894518-7899000 |
| 17163 | Ppm1f | NM_176833.4 | chr16:16896468-16927375 | 17260 | Pqlc1 | NM_001164420.1 | chr18:80255244-80292724 |
| 17164 | Ppm1g | NM_008014.3 | chr5:31202667-31220545 | 17261 | Pqlc2 | NM_145384.2 | chr4:139298004-139310700 |
| 17165 | Ppm1h | NM_001110218.1 | chr10:122678761-122945793 | 17262 | Pqlc3 | NM_001161111.1 | chr12:16992960-17000118 |
| 17166 | Ppm1j | NM_027982.2 | chr3:104781055-104786017 | 17263 | Pradc1 | NM_001163427.1 | chr6:85446784-85451302 |
| 17167 | Ppm1k | NM_175523.4 | chr6:57506501-57535426 | 17264 | Praf2 | NM_138602.4 | chrX:7728570-7731063 |
| 17168 | Ppm1l | NM_178726.3 | chr3:69316917-69555396 | 17265 | Pram1 | NM_001002842.2 | chr17:33638055-33645706 |

Fig.21 - 90

| | | | |
|---|---|---|---|
| 17266 | Prame | NM_029459.2 | chrX:135613001-135627705 |
| 17267 | Pramef12 | NM_029948.2 | chr4:144391673-144408464 |
| 17268 | Pramef17 | NM_001085540.2 | chr4:143991126-143994369 |
| 17269 | Pramef25 | NM_001126315.2 | chr4:143948182-143951016 |
| 17270 | Pramef6 | NM_001085414.2 | chr4:143894236-143900380 |
| 17271 | Pramef8 | NM_172877.2 | chr4:143412425-143421087 |
| 17272 | Pramel1 | NM_031377.2 | chr4:143394440-143399819 |
| 17273 | Pramel3 | NM_031390.2 | chrX:135302315-135312636 |
| 17274 | Pramel4 | NM_001001319.3 | chr4:144059125-144069318 |
| 17275 | Pramel5 | NM_001085418.2 | chr4:144270632-144280466 |
| 17276 | Pramel6 | NM_178249.2 | chr2:87508457-87510865 |
| 17277 | Pramel7 | NM_178250.2 | chr2:87489087-87492418 |
| 17278 | Prap1 | NM_009475.2 | chr7:140093395-140097203 |
| 17279 | Prb1 | NM_198669.1 | chr6:132206794-132210521 |
| 17280 | Prc1 | NM_001285997.1 | chr7:80294450-80316259 |
| 17281 | Prcc | NM_033573.2 | chr3:87858902-87885562 |
| 17282 | Prcp | NM_028243.3 | chr7:92875252-92934581 |
| 17283 | Prdm1 | NM_007548.4 | chr10:44437173-44458748 |
| 17284 | Prdm10 | NM_001080817.1 | chr9:31315106-31378543 |
| 17285 | Prdm11 | NM_001177536.1 | chr2:92974905-93046144 |
| 17286 | Prdm12 | NM_001123362.1 | chr2:31640036-31655795 |
| 17287 | Prdm13 | NM_001080771.1 | chr4:21677479-21685963 |
| 17288 | Prdm14 | NM_001081209.2 | chr1:13113427-13127163 |
| 17289 | Prdm15 | NM_144789.2 | chr16:97791466-97851227 |
| 17290 | Prdm16 | NM_001177995.1 | chr4:154316124-154636873 |
| 17291 | Prdm2 | NM_001081355.3 | chr4:143107390-143212709 |
| 17292 | Prdm4 | NM_181650.3 | chr10:85891967-85916729 |
| 17293 | Prdm5 | NM_027547.2 | chr6:65778961-65936377 |
| 17294 | Prdm6 | NM_001033281.3 | chr18:53464545-53575857 |
| 17295 | Prdm8 | NM_029947.2 | chr5:98180868-98187448 |
| 17296 | Prdm9 | NM_144809.2 | chr17:15543078-15563323 |
| 17297 | Prdx1 | NM_011034.4 | chr4:116685598-116700000 |
| 17298 | Prdx2 | NM_011563.5 | chr8:84969647-84974313 |
| 17299 | Prdx3 | NM_007452.2 | chr19:60864065-60874538 |
| 17300 | Prdx4 | NM_016764.5 | chrX:155323917-155338460 |
| 17301 | Prdx5 | NM_012021.2 | chr19:6906818-6909645 |
| 17302 | Prdx6 | NM_007453.4 | chr1:161240111-161251210 |
| 17303 | Prdx6b | NM_177256.5 | chr2:80292469-80295358 |
| 17304 | Preb | NM_016703.3 | chr5:30951666-30960327 |
| 17305 | Prelid1 | NM_025596.5 | chr13:55322054-55325272 |
| 17306 | Prelid2 | NM_029942.1 | chr18:41875695-41951194 |
| 17307 | Prelp | NM_054077.4 | chr1:133910303-133921401 |
| 17308 | Prep | NM_011156.2 | chr10:45067213-45158995 |
| 17309 | Prepl | NM_001163622.1 | chr17:85063476-85090267 |
| 17310 | Prex1 | NM_177782.3 | chr2:166566344-166713832 |
| 17311 | Prex2 | NM_001033636.4 | chr1:11263962-11303682 |
| 17312 | Prf1 | NM_011073.3 | chr10:61297835-61304263 |
| 17313 | Prg2 | NM_008920.4 | chr2:84980460-84983632 |
| 17314 | Prg3 | NM_016914.2 | chr2:84988214-84993886 |
| 17315 | Prg4 | NM_001110146.1 | chr1:150449411-150466165 |
| 17316 | Prh1 | NM_011174.4 | chr6:132569841-132572401 |
| 17317 | Prickle1 | NM_001033217.4 | chr15:93499113-93595891 |
| 17318 | Prickle2 | NM_001081146.2 | chr6:92370891-92706184 |
| 17319 | Prickle3 | NM_001290624.1 | chrX:7657378-7668186 |
| 17320 | Prickle4 | NM_001290337.1 | chr17:47688473-47694736 |
| 17321 | Prim1 | NM_008921.2 | chr10:128015214-128030030 |
| 17322 | Prim2 | NM_008922.2 | chr1:33453807-33669794 |
| 17323 | Prima1 | NM_133364.2 | chr12:103196907-103242146 |
| 17324 | Primpol | NM_001001184.1 | chr8:46575593-46617200 |
| 17325 | Prkaa1 | NM_001013367.3 | chr15:5143860-5181899 |
| 17326 | Prkaa2 | NM_178143.2 | chr4:105029649-105109898 |
| 17327 | Prkab1 | NM_031869.2 | chr5:116013589-116024428 |
| 17328 | Prkab2 | NM_182997.2 | chr3:97658211-97673067 |
| 17329 | Prkaca | NM_001277898.1 | chr8:83976882-83996442 |
| 17330 | Prkacb | NM_001164198.1 | chr3:146729578-146770238 |
| 17331 | Prkag1 | NM_016781.2 | chr15:98812796-98831508 |
| 17332 | Prkag2 | NM_001170555.1 | chr5:24862734-24908509 |
| 17333 | Prkag2os1 | NR_040684.1 | chr5:24902568-24906849 |
| 17334 | Prkag3 | NM_153744.3 | chr1:74738921-74748955 |
| 17335 | Prkar1a | NM_021880.3 | chr11:109650920-109669663 |
| 17336 | Prkar1b | NM_001253890.1 | chr5:139017303-139130386 |
| 17337 | Prkar2a | NM_008924.2 | chr9:108692142-108749511 |
| 17338 | Prkar2b | NM_011158.3 | chr12:31958478-32061279 |
| 17339 | Prkca | NM_011101.3 | chr11:107933386-108343888 |
| 17340 | Prkcb | NM_008855.2 | chr7:122289124-122634401 |
| 17341 | Prkcd | NM_011103.3 | chr14:30595353-30626208 |
| 17342 | Prkcdbp | NM_028444.1 | chr7:105480615-105482197 |
| 17343 | Prkce | NM_011104.3 | chr17:86167784-86657919 |
| 17344 | Prkcg | NM_001291434.1 | chr7:3303531-3331097 |
| 17345 | Prkch | NM_008856.4 | chr12:73584795-73778184 |
| 17346 | Prkci | NM_008857.3 | chr3:30995770-31052740 |
| 17347 | Prkcq | NM_008859.2 | chr2:11172381-11301226 |
| 17348 | Prkcsh | NM_008925.2 | chr9:22002987-22014245 |
| 17349 | Prkcz | NM_001039079.2 | chr4:155260117-155345789 |
| 17350 | Prkd1 | NM_008858.3 | chr12:50341231-50649223 |
| 17351 | Prkd2 | NM_001252458.1 | chr7:16842901-16870461 |
| 17352 | Prkd3 | NM_001171004.1 | chr17:78949404-79020816 |
| 17353 | Prkdc | NM_011159.2 | chr16:15637865-15842239 |
| 17354 | Prkg1 | NM_001013833.3 | chr19:30564486-31765033 |
| 17355 | Prkg2 | NM_008926.4 | chr5:98929772-99037079 |
| 17356 | Prkra | NM_011871.2 | chr2:76629936-76647994 |
| 17357 | Prkrip1 | NM_025774.3 | chr5:136180356-136198954 |
| 17358 | Prkrir | NM_028410.1 | chr7:98703362-98718061 |
| 17359 | Prkx | NM_016979.1 | chrX:77762029-77795960 |
| 17360 | Prl | NM_001163530.1 | chr13:27057569-27065203 |
| 17361 | Prl2a1 | NM_019991.1 | chr13:27801654-27808716 |
| 17362 | Prl2b1 | NM_025532.3 | chr13:27383344-27390846 |
| 17363 | Prl2c1 | NM_001045532.2 | chr13:27849341-27857708 |
| 17364 | Prl2c2 | NM_031191.1 | chr13:12996123-13005330 |
| 17365 | Prl2c3 | NM_011118.2 | chr13:12790820-12800079 |
| 17366 | Prl2c4 | NM_011954.2 | chr13:12790817-12800058 |
| 17367 | Prl2c5 | NM_181852.2 | chr13:13182715-13191925 |
| 17368 | Prl3a1 | NM_025896.2 | chr13:27259488-27276660 |
| 17369 | Prl3b1 | NM_008865.3 | chr13:27242429-27249740 |
| 17370 | Prl3c1 | NM_001163218.1 | chr13:27198902-27203750 |
| 17371 | Prl3d1 | NM_001205322.1 | chr13:27094188-27100260 |
| 17372 | Prl3d2 | NM_172155.1 | chr13:27121703-27127482 |
| 17373 | Prl3d3 | NM_172156.2 | chr13:27156798-27162520 |
| 17374 | Prl4a1 | NM_011165.3 | chr13:28016222-28023546 |
| 17375 | Prl5a1 | NM_023746.4 | chr13:28142483-28151595 |
| 17376 | Prl6a1 | NM_011166.2 | chr13:27312626-27319252 |
| 17377 | Prl7a1 | NM_001164058.1 | chr13:27633363-27642493 |
| 17378 | Prl7a2 | NM_011168.4 | chr13:27658583-27668036 |
| 17379 | Prl7b1 | NM_029355.2 | chr13:27601818-27610582 |
| 17380 | Prl7c1 | NM_026206.2 | chr13:27773494-27780804 |
| 17381 | Prl7d1 | NM_011120.2 | chr13:27708997-27716737 |
| 17382 | Prl8a1 | NM_028477.2 | chr13:27573921-27582171 |
| 17383 | Prl8a2 | NM_001289919.1 | chr13:27345672-27354215 |
| 17384 | Prl8a6 | NM_001271378.1 | chr13:27432680-27438688 |
| 17385 | Prl8a8 | NM_023741.2 | chr13:27507071-27513213 |
| 17386 | Prl8a9 | NM_023332.4 | chr13:27558000-27564604 |
| 17387 | Prlh | NM_001101647.1 | chr1:90953107-90954012 |
| 17388 | Prlhr | NM_201615.2 | chr19:60466732-60468304 |
| 17389 | Prlr | NM_001253781.1 | chr15:10223905-10349180 |
| 17390 | Prm1 | NM_013637.4 | chr16:10796331-10796823 |
| 17391 | Prm2 | NM_008933.2 | chr16:10791376-10792105 |
| 17392 | Prm3 | NM_013638.2 | chr16:10790507-10790914 |
| 17393 | Prmt1 | NM_001252476.1 | chr7:44976754-44986420 |
| 17394 | Prmt10 | NM_001081240.3 | chr8:77549396-77581338 |
| 17395 | Prmt2 | NM_001077638.2 | chr10:76207225-76237865 |
| 17396 | Prmt3 | NM_133740.2 | chr7:49778357-49858265 |
| 17397 | Prmt5 | NM_013768.3 | chr14:54507181-54517470 |
| 17398 | Prmt6 | NM_178891.5 | chr3:110248524-110250999 |
| 17399 | Prmt7 | NM_145404.1 | chr8:106211053-106251694 |
| 17400 | Prmt8 | NM_201371.2 | chr6:127689008-127769159 |
| 17401 | Prn | NM_001278258.1 | chr2:131909927-131956131 |
| 17402 | Prnd | NM_001126338.2 | chr2:131950860-131956131 |
| 17403 | Prnp | NM_001278256.1 | chr2:131909927-131938436 |
| 17404 | Prob1 | NM_001270646.1 | chr18:35650350-35655199 |
| 17405 | Proc | NM_001042767.3 | chr18:32123125-32139570 |
| 17406 | Proca1 | NM_001045516.2 | chr11:78193391-78205763 |
| 17407 | Procr | NM_011171.2 | chr2:155751216-155755478 |
| 17408 | Prodh | NM_011172.2 | chr16:18071725-18089190 |
| 17409 | Prodh2 | NM_019546.5 | chr7:30493657-30513402 |
| 17410 | Prok1 | NM_001044382.1 | chr3:107235530-107239707 |
| 17411 | Prok2 | NM_001037539.2 | chr6:99711298-99726392 |
| 17412 | Prokr1 | NM_021381.3 | chr6:87578591-87590701 |
| 17413 | Prokr2 | NM_144944.3 | chr2:132370328-132385447 |
| 17414 | Prol1 | NM_008644.2 | chr5:88317311-88328817 |
| 17415 | Prom1 | NM_001163577.1 | chr5:43993621-44101736 |
| 17416 | Prom2 | NM_138750.2 | chr2:127526952-127541417 |
| 17417 | Prop1 | NM_008936.1 | chr11:50950805-50953632 |
| 17418 | Prorsd1 | NM_001163454.2 | chr11:29511756-29515033 |
| 17419 | Pros1 | NM_011173.3 | chr16:62854306-62929342 |
| 17420 | Prosc | NM_001039077.2 | chr8:27042554-27050241 |
| 17421 | Proser1 | NM_173382.1 | chr3:53463816-53481755 |
| 17422 | Proser2 | NM_001159657.1 | chr2:6098499-6130185 |
| 17423 | Prox1 | NM_008937.2 | chr1:190121776-190170680 |
| 17424 | Prox2 | NM_175198.4 | chr12:85086813-85106431 |
| 17425 | Proz | NM_025834.3 | chr8:13060907-13075006 |
| 17426 | Prp2 | NM_031499.2 | chr6:132595945-132600702 |
| 17427 | Prpf18 | NM_026045.3 | chr2:4622166-4652086 |
| 17428 | Prpf19 | NM_001253843.1 | chr19:10895230-10909559 |
| 17429 | Prpf3 | NM_027541.4 | chr3:95830621-95855753 |
| 17430 | Prpf31 | NM_001159714.1 | chr7:3629984-3642484 |
| 17431 | Prpf38a | NM_172697.3 | chr4:108564866-108579336 |
| 17432 | Prpf38b | NM_025845.2 | chr3:108902806-108911704 |
| 17433 | Prpf39 | NM_177806.3 | chr12:65036333-65063386 |
| 17434 | Prpf4 | NM_027297.3 | chr4:62408782-62426990 |
| 17435 | Prpf40a | NM_018785.2 | chr2:53138475-53191187 |
| 17436 | Prpf40b | NM_018786.2 | chr15:99295408-99317007 |
| 17437 | Prpf4b | NM_013830.2 | chr13:34875493-34902878 |
| 17438 | Prpf6 | NM_133701.2 | chr2:181601318-181655661 |
| 17439 | Prpf8 | NM_138659.2 | chr11:75486776-75509447 |
| 17440 | Prph | NM_001163588.1 | chr15:99055173-99058978 |
| 17441 | Prph2 | NM_008938.1 | chr17:46910484-46924926 |
| 17442 | Prpmp5 | NM_001024705.2 | chr6:132311589-132314743 |
| 17443 | Prps1 | NM_021463.4 | chrX:140456602-140476140 |
| 17444 | Prps1l1 | NM_029294.2 | chr12:34984760-34986436 |
| 17445 | Prps1l3 | NM_001037746.3 | chr12:57230411-57242168 |
| 17446 | Prps2 | NM_026662.5 | chrX:167346319-167382749 |
| 17447 | Prpsap1 | NM_026364.1 | chr11:116470815-116490346 |
| 17448 | Prpsap2 | NM_001164242.1 | chr11:61729649-61762088 |
| 17449 | Prr11 | NM_175563.5 | chr11:87089155-87108714 |
| 17450 | Prr12 | NM_175022.2 | chr7:45027706-45052881 |
| 17451 | Prr13 | NM_001170911.1 | chr15:102459169-102462806 |
| 17452 | Prr14 | NM_145589.2 | chr7:127471613-127476758 |
| 17453 | Prr14l | NM_194340.2 | chr5:32789206-32854230 |
| 17454 | Prr15 | NM_030024.2 | chr6:54327011-54330200 |
| 17455 | Prr15l | NM_146026.1 | chr11:96929323-96935647 |
| 17456 | Prr16 | NM_001081224.2 | chr18:51117897-51304641 |
| 17457 | Prr18 | NM_178774.4 | chr17:8340738-8344113 |
| 17458 | Prr19 | NM_001081294.1 | chr7:25301358-25304133 |
| 17459 | Prr22 | NM_001195673.1 | chr17:56770275-56772134 |

Fig.21 - 91

| | | | | | | | |
|---|---|---|---|---|---|---|---|
| 17460 | Prr23a | NM_001134660.1 | chr9:98842586-98843645 | 17557 | Psma2 | NM_008944.2 | chr13:14613241-14625673 |
| 17461 | Prr24 | NM_001136270.1 | chr7:16272012-16273692 | 17558 | Psma3 | NM_011184.5 | chr12:70974622-70995877 |
| 17462 | Prr27 | NM_001163551.1 | chr5:87825696-87846386 | 17559 | Psma4 | NM_011966.3 | chr9:54950858-54958030 |
| 17463 | Prr3 | NM_001165892.1 | chr17:35977755-35979467 | 17560 | Psma5 | NM_011967.3 | chr3:108256925-108279952 |
| 17464 | Prr30 | NM_029680.1 | chr14:101197689-101200069 | 17561 | Psma6 | NM_011968.3 | chr12:55398824-55418459 |
| 17465 | Prr32 | NM_026841.1 | chrX:45090903-45092790 | 17562 | Psma7 | NM_001289476.1 | chr2:180036366-180042464 |
| 17466 | Prr33 | NR_033261.1 | chr7:142491079-142506771 | 17563 | Psma8 | NM_001163609.1 | chr18:14706150-14762299 |
| 17467 | Prr5 | NM_146061.4 | chr15:84680997-84703673 | 17564 | Psmb1 | NM_011185.3 | chr17:15475720-15498276 |
| 17468 | Prr5l | NM_001083810.2 | chr2:101714284-101797707 | 17565 | Psmb10 | NM_013640.3 | chr8:105935727-105938392 |
| 17469 | Prr7 | NM_001030296.4 | chr13:55464266-55473155 | 17566 | Psmb11 | NM_175204.4 | chr14:54625309-54629556 |
| 17470 | Prr9 | NM_175424.3 | chr3:92122203-92123947 | 17567 | Psmb2 | NM_011970.4 | chr4:126677642-126709715 |
| 17471 | Prrc1 | NM_028447.3 | chr18:57354732-57392719 | 17568 | Psmb3 | NM_011971.4 | chr11:97703433-97713500 |
| 17472 | Prrc2a | NM_001199044.1 | chr17:35149085-35164877 | 17569 | Psmb4 | NM_008945.3 | chr3:94884323-94886958 |
| 17473 | Prrc2b | NM_001159634.1 | chr2:32151147-32234537 | 17570 | Psmb5 | NM_011186.1 | chr14:54614119-54617995 |
| 17474 | Prrc2c | NM_001081290.1 | chr1:162671784-162740556 | 17571 | Psmb6 | NM_008946.4 | chr11:70525356-70527858 |
| 17475 | Prrg1 | NM_001164275.2 | chrX:78449609-78583805 | 17572 | Psmb7 | NM_011187.1 | chr2:38588045-38643906 |
| 17476 | Prrg2 | NM_022999.1 | chr7:45053606-45061652 | 17573 | Psmb8 | NM_010724.2 | chr17:34198194-34201454 |
| 17477 | Prrg3 | NM_001081135.2 | chrX:71962981-71972722 | 17574 | Psmb9 | NM_013585.2 | chr17:34182098-34187330 |
| 17478 | Prrg4 | NM_178695.5 | chr2:104830740-104849850 | 17575 | Psmc1 | NM_008947.3 | chr12:100112330-100123364 |
| 17479 | Prrt1 | NM_030890.1 | chr17:34629685-34632260 | 17576 | Psmc2 | NM_011188.3 | chr5:21785282-21803784 |
| 17480 | Prrt2 | NM_001102563.1 | chr7:127017541-127021211 | 17577 | Psmc3 | NM_008948.2 | chr2:91054015-91059438 |
| 17481 | Prrt3 | NM_001289699.1 | chr6:113493638-113501931 | 17578 | Psmc3ip | NM_008949.3 | chr11:101092140-101095435 |
| 17482 | Prrt4 | NM_001101443.1 | chr6:29169229-29179584 | 17579 | Psmc4 | NM_011874.2 | chr7:28041701-28050092 |
| 17483 | Prrx1 | NM_001025570.1 | chr1:163255276-163313650 | 17580 | Psmc5 | NM_008950.1 | chr11:106256184-106263112 |
| 17484 | Prrx2 | NM_009116.2 | chr2:30845366-30881247 | 17581 | Psmc6 | NM_025959.3 | chr14:45329823-45349071 |
| 17485 | Prrxl1 | NM_001001796.4 | chr14:32599926-32649246 | 17582 | Psmd1 | NM_027357.2 | chr1:86064618-86139295 |
| 17486 | Prss1 | NM_053243.2 | chr6:41458929-41463786 | 17583 | Psmd10 | NM_001164177.1 | chrX:140948424-140956711 |
| 17487 | Prss12 | NM_008939.2 | chr3:123446912-123506602 | 17584 | Psmd11 | NM_178616.3 | chr11:80428614-80472133 |
| 17488 | Prss16 | NM_019429.2 | chr13:22002175-22009741 | 17585 | Psmd12 | NM_025894.2 | chr11:107479527-107498036 |
| 17489 | Prss2 | NM_009430.2 | chr6:41521775-41525079 | 17586 | Psmd13 | NM_011875.4 | chr7:140882393-140898642 |
| 17490 | Prss21 | NM_020487.4 | chr17:23868071-23873113 | 17587 | Psmd14 | NM_021526.2 | chr2:61711693-61800376 |
| 17491 | Prss22 | NM_133731.2 | chr17:23993533-23998100 | 17588 | Psmd2 | NM_134101.2 | chr16:20651651-20663414 |
| 17492 | Prss23 | NM_029614.3 | chr7:89507784-89517586 | 17589 | Psmd3 | NM_009439.1 | chr11:98682553-98695978 |
| 17493 | Prss27 | NM_175440.4 | chr17:24038242-24045949 | 17590 | Psmd4 | NM_001282017.1 | chr3:95032690-95042614 |
| 17494 | Prss28 | NM_053259.2 | chr17:25308645-25311876 | 17591 | Psmd5 | NM_080554.2 | chr2:34852088-34870962 |
| 17495 | Prss29 | NM_053260.3 | chr17:25318653-25322684 | 17592 | Psmd6 | NM_025550.2 | chr14:14112184-14120904 |
| 17496 | Prss3 | NM_011645.2 | chr6:41373758-41377613 | 17593 | Psmd7 | NM_010817.2 | chr8:107580379-107588482 |
| 17497 | Prss30 | NM_013921.3 | chr17:23972126-23975230 | 17594 | Psmd8 | NM_026545.3 | chr7:29174186-29180673 |
| 17498 | Prss32 | NM_027220.2 | chr17:23853771-23859776 | 17595 | Psmd9 | NM_026000.2 | chr5:123228189-123250125 |
| 17499 | Prss33 | NM_001081399.2 | chr17:23833360-23835767 | 17596 | Psme1 | NM_011189.1 | chr14:55578493-55581527 |
| 17500 | Prss34 | NM_178372.2 | chr17:25298393-25300161 | 17597 | Psme2 | NM_001029855.1 | chr14:55587439-55591101 |
| 17501 | Prss35 | NM_178738.3 | chr9:86743632-86757506 | 17598 | Psme2b | NM_001281472.1 | chr11:48945351-48946410 |
| 17502 | Prss36 | NM_001081374.1 | chr7:127932637-127946725 | 17599 | Psme3 | NM_011192.3 | chr11:101316250-101323530 |
| 17503 | Prss37 | NM_026317.2 | chr6:40514823-40519508 | 17600 | Psme4 | NM_134013.3 | chr11:30771774-30880361 |
| 17504 | Prss38 | NM_001045521.1 | chr11:59377668-59375653 | 17601 | Psmf1 | NM_212446.2 | chr2:151716061-151741310 |
| 17505 | Prss39 | NM_009355.2 | chr1:34498429-34503062 | 17602 | Psmg1 | NM_019537.2 | chr16:95979934-95990903 |
| 17506 | Prss40 | NM_009356.2 | chr1:34552330-34560943 | 17603 | Psmg2 | NM_134138.1 | chr18:67641598-67654162 |
| 17507 | Prss41 | NM_027644.1 | chr17:23836784-23844156 | 17604 | Psmg3 | NM_025604.3 | chr5:139823593-139826843 |
| 17508 | Prss42 | NM_153099.1 | chr9:110798184-110803744 | 17605 | Psmg4 | NM_001101430.2 | chr13:34162963-34178172 |
| 17509 | Prss43 | NM_199471.1 | chr9:110826689-110831504 | 17606 | Psors1c2 | NM_020576.2 | chr17:35533200-35534648 |
| 17510 | Prss44 | NM_148940.3 | chr9:110813993-110817999 | 17607 | Pspc1 | NM_025682.3 | chr14:56722448-56768316 |
| 17511 | Prss45 | NM_153172.1 | chr9:110834587-110841310 | 17608 | Psph | NM_133900.4 | chr5:129765557-129787253 |
| 17512 | Prss46 | NM_183103.2 | chr9:110844505-110856522 | 17609 | Pspn | NM_008954.2 | chr17:56999456-57000018 |
| 17513 | Prss48 | NM_001001650.1 | chr3:85993809-86002491 | 17610 | Psrc1 | NM_001190161.1 | chr3:108383803-108388231 |
| 17514 | Prss50 | NM_146227.4 | chr9:110857966-110864628 | 17611 | Pstk | NM_001039534.1 | chr7:131371145-131387838 |
| 17515 | Prss51 | NM_001193631.1 | chr14:64093695-64097672 | 17612 | Pstpip1 | NM_011193.2 | chr9:56089975-56128890 |
| 17516 | Prss52 | NM_028525.2 | chr14:64104322-64113751 | 17613 | Pstpip2 | NM_013831.4 | chr18:77794549-77882879 |
| 17517 | Prss53 | NM_001081268.1 | chr7:127885443-127890970 | 17614 | Ptafr | NM_001081211.2 | chr4:132564066-132582686 |
| 17518 | Prss54 | NM_027640.1 | chr8:95559291-95575197 | 17615 | Ptar1 | NM_028208.1 | chr19:23687399-23721129 |
| 17519 | Prss55 | NM_001081063.1 | chr14:64075442-64085389 | 17616 | Ptbp1 | NM_001077363.2 | chr10:79854431-79864435 |
| 17520 | Prss56 | NM_027084.2 | chr1:87183313-87188405 | 17617 | Ptbp2 | NM_019550.2 | chr3:119718741-119783388 |
| 17521 | Prss57 | NM_001042710.1 | chr10:79781473-79788985 | 17618 | Ptbp3 | NM_144904.2 | chr4:59471867-59549364 |
| 17522 | Prss58 | NM_175020.3 | chr6:40895261-40900387 | 17619 | Ptcd1 | NM_133735.2 | chr5:145147377-145167104 |
| 17523 | Prss8 | NM_133351.3 | chr7:127925716-127930113 | 17620 | Ptcd2 | NM_026873.2 | chr13:99319648-99344678 |
| 17524 | Prtg | NM_175485.4 | chr9:72807273-72917307 | 17621 | Ptcd3 | NM_027275.3 | chr6:71880637-71908762 |
| 17525 | Prtn3 | NM_011178.2 | chr10:79879666-79883172 | 17622 | Ptch1 | NM_008957.2 | chr13:63511532-63565520 |
| 17526 | Prune | NM_173347.2 | chr3:95253673-95282076 | 17623 | Ptch2 | NM_008958.3 | chr4:117096055-117116101 |
| 17527 | Prune2 | NM_181348.4 | chr19:16956117-17223932 | 17624 | Ptchd1 | NM_001093750.1 | chrX:155569735-155623327 |
| 17528 | Prx | NM_019412.2 | chr7:27499323-27520041 | 17625 | Ptchd2 | NM_001083342.1 | chr4:148236856-148287965 |
| 17529 | Psap | NM_001146120.1 | chr10:60277627-60302600 | 17626 | Ptchd3 | NM_029049.1 | chr11:121830217-121843436 |
| 17530 | Psapl1 | NM_175249.3 | chr5:36204020-36206567 | 17627 | Ptchd4 | NM_028474.1 | chr17:42315946-42507741 |
| 17531 | Psat1 | NM_001205339.1 | chr19:15905122-15924622 | 17628 | Ptcra | NM_011195.2 | chr17:46755662-46763712 |
| 17532 | Psca | NM_028216.2 | chr15:74714838-74717065 | 17629 | Ptdss1 | NM_008959.3 | chr13:66932829-66998401 |
| 17533 | Psd | NM_028627.2 | chr19:46312086-46327156 | 17630 | Ptdss2 | NM_013782.4 | chr7:141131285-141156154 |
| 17534 | Psd2 | NM_001289602.1 | chr18:35964829-36014716 | 17631 | Pten | NM_008960.2 | chr19:32757576-32826160 |
| 17535 | Psd3 | NM_027626.1 | chr8:67689081-67818295 | 17632 | Pter | NM_008961.3 | chr2:12924040-13003454 |
| 17536 | Psd4 | NM_177611.3 | chr2:24385396-24408729 | 17633 | Ptf1a | NM_018809.2 | chr2:19445662-19447501 |
| 17537 | Psen1 | NM_008943.2 | chr12:83688562-83735199 | 17634 | Ptgdr | NM_008962.4 | chr14:44851234-44859375 |
| 17538 | Psen2 | NM_001128605.1 | chr1:180227003-180256300 | 17635 | Ptgdr2 | NM_009962.3 | chr19:10937159-10942511 |
| 17539 | Psenen | NM_025498.2 | chr7:30561865-30563184 | 17636 | Ptgds | NM_008963.2 | chr2:25466711-25469749 |
| 17540 | Psg16 | NM_007676.4 | chr7:17074039-17098971 | 17637 | Ptger1 | NM_013641.2 | chr8:83666639-83670103 |
| 17541 | Psg17 | NM_007677.2 | chr7:18813936-18821591 | 17638 | Ptger2 | NM_008964.4 | chr14:44988110-45003820 |
| 17542 | Psg18 | NM_001163685.1 | chr7:18348201-18354993 | 17639 | Ptger3 | NM_011196.2 | chr3:157566891-157644758 |
| 17543 | Psg19 | NM_011964.2 | chr7:18789124-18798510 | 17640 | Ptger4 | NM_001136079.2 | chr15:5233398-5244187 |
| 17544 | Psg20 | NM_054058.1 | chr7:18674365-18685992 | 17641 | Ptges | NM_022415.3 | chr2:30889470-30903297 |
| 17545 | Psg21 | NM_027403.4 | chr7:18646653-18656725 | 17642 | Ptges2 | NM_133783.2 | chr2:32395889-32402740 |
| 17546 | Psg22 | NM_001004152.2 | chr7:18718089-18727248 | 17643 | Ptges3 | NM_019766.4 | chr10:128058981-128077254 |
| 17547 | Psg23 | NM_020261.4 | chr7:18606342-18616501 | 17644 | Ptges3l | NM_026865.2 | chr11:101418813-101425333 |
| 17548 | Psg25 | NM_054060.1 | chr7:18519701-18532227 | 17645 | Ptgfr | NM_008966.3 | chr3:151798609-151837528 |
| 17549 | Psg26 | NM_001029893.1 | chr7:18474581-18484149 | 17646 | Ptgfrn | NM_011197.3 | chr3:101040235-101110166 |
| 17550 | Psg27 | NM_001037168.1 | chr7:18556513-18567305 | 17647 | Ptgir | NM_008967.3 | chr7:16906489-16910905 |
| 17551 | Psg28 | NM_054063.4 | chr7:18422535-18432055 | 17648 | Ptgis | NM_008968.3 | chr2:167203195-167240537 |
| 17552 | Psg29 | NM_054064.3 | chr7:17203476-17215756 | 17649 | Ptgr1 | NM_025968.3 | chr4:58965589-58987078 |
| 17553 | Psg-ps1 | NR_002857.1 | chr7:17672312-17682060 | 17650 | Ptgr2 | NM_001252625.1 | chr12:84285295-84315832 |
| 17554 | Psip1 | NM_001290527.1 | chr4:83461878-83486448 | 17651 | Ptgs1 | NM_008969.4 | chr2:36230425-36252271 |
| 17555 | Pskh1 | NM_173432.2 | chr8:105900473-105931802 | 17652 | Ptgs2 | NM_011198.4 | chr1:150100030-150108234 |
| 17556 | Psma1 | NM_011965.2 | chr7:114264549-114276116 | 17653 | Ptgs2os | NR_015466.3 | chr1:150074872-150099874 |

Fig.21 - 92

| | | | | | | | |
|---|---|---|---|---|---|---|---|
| 17654 | Pth | NM_020623.2 | chr7:113385575-113388573 | 17751 | Pxmp2 | NM_008993.2 | chr5:110274285-110286168 |
| 17655 | Pth1r | NM_001083935.1 | chr9:110722084-110743686 | 17752 | Pxmp4 | NM_021534.3 | chr2:154587043-154603673 |
| 17656 | Pth2 | NM_053256.2 | chr7:45180994-45181838 | 17753 | Pxn | NM_011223.3 | chr5:115506675-115555987 |
| 17657 | Pth2r | NM_139270.2 | chr1:65311256-65389244 | 17754 | Pxt1 | NM_153390.1 | chr17:28933985-28942262 |
| 17658 | Pthlh | NM_008970.4 | chr6:147252108-147264013 | 17755 | Pxylp1 | NM_001289645.1 | chr9:96823342-96889474 |
| 17659 | Ptk2 | NM_001130409.1 | chr15:73215556-73423191 | 17756 | Pycard | NM_023258.4 | chr7:127991372-127993867 |
| 17660 | Ptk2b | NM_001162365.1 | chr14:66153256-66281052 | 17757 | Pycr1 | NM_144795.3 | chr11:120635711-120643670 |
| 17661 | Ptk6 | NM_009184.2 | chr2:181195123-181202789 | 17758 | Pycr2 | NM_133705.2 | chr1:180904273-180908088 |
| 17662 | Ptk7 | NM_175168.4 | chr17:46564450-46629504 | 17759 | Pycrl | NM_025412.2 | chr15:75916462-75921560 |
| 17663 | Ptma | NM_008972.2 | chr1:86526735-86530698 | 17760 | Pydc3 | NM_001162938.1 | chr1:173673679-173698392 |
| 17664 | Ptms | NM_026988.2 | chr6:124913674-124917946 | 17761 | Pydc4 | NM_001177349.1 | chr1:173591956-173599274 |
| 17665 | Ptn | NM_008973.2 | chr6:36715662-36811361 | 17762 | Pygb | NM_153781.1 | chr2:150786795-150831748 |
| 17666 | Ptov1 | NM_133949.1 | chr7:44863067-44869788 | 17763 | Pygl | NM_133198.2 | chr12:70190814-70227683 |
| 17667 | Ptp4a1 | NM_011200.2 | chr1:30940302-30949755 | 17764 | Pygm | NM_011224.1 | chr19:6384428-6398459 |
| 17668 | Ptp4a2 | NM_001164745.1 | chr4:129820707-129850003 | 17765 | Pygo1 | NM_028116.2 | chr9:72925649-72946015 |
| 17669 | Ptp4a3 | NM_001166388.1 | chr15:73724929-73758766 | 17766 | Pygo2 | NM_026869.3 | chr3:89430836-89435130 |
| 17670 | Ptpdc1 | NM_207232.2 | chr13:48577868-48625672 | 17767 | Pyhin1 | NM_175026.3 | chr1:173630858-173647928 |
| 17671 | Ptpla | NM_001012396.2 | chr2:14026830-14056035 | 17768 | Pyroxd1 | NM_183165.3 | chr6:142345696-142362624 |
| 17672 | Ptplad1 | NM_021345.2 | chr9:64986982-65021717 | 17769 | Pyroxd2 | NM_029011.2 | chr19:42725857-42752775 |
| 17673 | Ptplad2 | NM_025760.4 | chr4:88412929-88438926 | 17770 | Pyurf | NM_025574.3 | chr6:57684738-57692078 |
| 17674 | Ptplb | NM_023587.2 | chr16:35022420-35109175 | 17771 | Pyy | NM_145435.1 | chr11:102106675-102107776 |
| 17675 | Ptpmt1 | NM_025576.2 | chr2:90910712-90918050 | 17772 | Pzp | NM_007376.4 | chr6:128483566-128526720 |
| 17676 | Ptpn1 | NM_011201.3 | chr2:167932326-167979385 | 17773 | Qars | NM_001168270.1 | chr9:108509478-108515941 |
| 17677 | Ptpn11 | NM_001109992.1 | chr5:121130532-121191397 | 17774 | Qdpr | NM_024236.2 | chr5:45434031-45450229 |
| 17678 | Ptpn12 | NM_011203.2 | chr5:20986644-21055797 | 17775 | Qk | NM_001159516.1 | chr17:10210179-10319361 |
| 17679 | Ptpn13 | NM_011204.2 | chr5:103425191-103598361 | 17776 | Qpct | NM_027455.3 | chr17:79051905-79090243 |
| 17680 | Ptpn14 | NM_008976.2 | chr1:189728267-189876693 | 17777 | Qpctl | NM_026111.3 | chr7:19140216-19149196 |
| 17681 | Ptpn18 | NM_011206.2 | chr1:34459745-34473779 | 17778 | Qprt | NM_133686.1 | chr7:127107769-127122029 |
| 17682 | Ptpn2 | NM_001127177.1 | chr18:67665500-67724621 | 17779 | Qrfp | NM_183424.4 | chr2:31806167-31810580 |
| 17683 | Ptpn20 | NM_008978.2 | chr14:33589269-33640754 | 17780 | Qrfpr | NM_198192.2 | chr3:36179425-36222275 |
| 17684 | Ptpn21 | NM_001146199.1 | chr12:98676740-98737405 | 17781 | Qrich1 | NM_001114119.1 | chr9:108517086-108560167 |
| 17685 | Ptpn22 | NM_008979.2 | chr3:103860276-103912252 | 17782 | Qrich2 | NM_001033267.2 | chr11:116441324-116454347 |
| 17686 | Ptpn23 | NM_001081043.1 | chr9:110385088-110408210 | 17783 | Qrsl1 | NM_001081054.2 | chr10:43874189-43901736 |
| 17687 | Ptpn3 | NM_011207.2 | chr4:57190840-57301837 | 17784 | Qser1 | NM_001123327.2 | chr2:104754792-104816696 |
| 17688 | Ptpn4 | NM_019933.2 | chr1:119658092-119837071 | 17785 | Qsox1 | NM_001024945.1 | chr1:155778154-155812899 |
| 17689 | Ptpn5 | NM_001163565.1 | chr7:47077799-47132598 | 17786 | Qsox2 | NM_153559.3 | chr2:26209123-26237399 |
| 17690 | Ptpn6 | NM_001077705.2 | chr6:124720706-124738709 | 17787 | Qtrt1 | NM_021888.2 | chr9:21411836-21420279 |
| 17691 | Ptpn7 | NM_177081.3 | chr1:135132724-135145320 | 17788 | Qtrtd1 | NM_029128.2 | chr16:43861412-43889676 |
| 17692 | Ptpn9 | NM_019651.2 | chr9:56994967-57062807 | 17789 | R3hcc1 | NM_001146012.2 | chr14:69697303-69707584 |
| 17693 | Ptpra | NM_001163688.1 | chr2:130450277-130554300 | 17790 | R3hcc1l | NM_177464.4 | chr19:42518804-42592256 |
| 17694 | Ptprb | NM_029928.2 | chr10:116301373-116389538 | 17791 | R3hdm1 | NM_181750.2 | chr1:128103305-128237735 |
| 17695 | Ptprc | NM_001111316.2 | chr1:138062858-138175126 | 17792 | R3hdm2 | NM_001168292.1 | chr10:127390310-127499384 |
| 17696 | Ptprcap | NM_016933.3 | chr19:4154645-4156710 | 17793 | R3hdm4 | NM_177994.4 | chr10:79910052-79916930 |
| 17697 | Ptprd | NM_011211.3 | chr4:75941236-78211895 | 17794 | R3hdml | NM_001099331.2 | chr2:163492317-163502612 |
| 17698 | Ptpre | NM_011212.3 | chr7:135537823-135686294 | 17795 | R74862 | NR_015529.2 | chr7:143032620-143053686 |
| 17699 | Ptprf | NM_011213.2 | chr4:118208212-118291397 | 17796 | Rab1 | NM_008996.3 | chr11:20201601-20226856 |
| 17700 | Ptprg | NM_008981.3 | chr14:11553552-12242039 | 17797 | Rab10 | NM_016676.5 | chr12:3247427-3309969 |
| 17701 | Ptprh | NM_207270.2 | chr7:4548613-4604041 | 17798 | Rab10os | NR_015551.1 | chr12:3235790-3250374 |
| 17702 | Ptprj | NM_001135657.1 | chr2:90429755-90479174 | 17799 | Rab11a | NM_017382.5 | chr9:64715299-64737756 |
| 17703 | Ptprk | NM_008983.2 | chr10:28074819-28597397 | 17800 | Rab11b | NM_008997.3 | chr17:33742483-33760486 |
| 17704 | Ptprm | NM_008984.2 | chr17:66666847-67354459 | 17801 | Rab11fip1 | NM_001080813.2 | chr8:27138772-27174646 |
| 17705 | Ptprn | NM_008985.2 | chr1:75247040-75264208 | 17802 | Rab11fip2 | NM_001033172.3 | chr19:59902883-59943364 |
| 17706 | Ptprn2 | NM_011215.2 | chr12:116485719-117278167 | 17803 | Rab11fip3 | NM_001162868.1 | chr17:25989035-26069177 |
| 17707 | Ptpro | NM_001164401.1 | chr6:137252298-137464633 | 17804 | Rab11fip4 | NM_175543.3 | chr11:79591211-79694012 |
| 17708 | Ptprq | NM_001081432.1 | chr10:107517359-107720027 | 17805 | Rab11fip4os1 | NR_003283.1 | chr11:79607078-79623163 |
| 17709 | Ptprr | NM_001161837.1 | chr10:116143897-116274929 | 17806 | Rab11fip4os2 | NR_045898.2 | chr11:79670372-79675086 |
| 17710 | Ptprs | NM_001252453.1 | chr17:56412425-56476480 | 17807 | Rab11fip5 | NM_001003955.2 | chr6:85334961-85374634 |
| 17711 | Ptprt | NM_001291149.1 | chr2:161521987-162661147 | 17808 | Rab12 | NM_024448.2 | chr17:66494511-66519670 |
| 17712 | Ptprtos | NR_040617.1 | chr2:162390812-162393946 | 17809 | Rab13 | NM_026677.4 | chr3:90220783-90226387 |
| 17713 | Ptpru | NM_001083119.2 | chr4:131768456-131838278 | 17810 | Rab14 | NM_026697.3 | chr2:35180204-35201120 |
| 17714 | Ptprv | NM_007955.3 | chr1:135108497-135132575 | 17811 | Rab15 | NM_134050.4 | chr12:76797962-76822524 |
| 17715 | Ptprz1 | NM_001081306.1 | chr6:22875501-23052916 | 17812 | Rab17 | NM_001159725.2 | chr1:90958132-90967667 |
| 17716 | Ptrf | NM_008986.2 | chr11:100956735-100970617 | 17813 | Rab18 | NM_001278447.1 | chr18:6765166-6791606 |
| 17717 | Ptrh1 | NM_178595.3 | chr2:32775820-32777593 | 17814 | Rab19 | NM_011226.1 | chr6:39381427-39390379 |
| 17718 | Ptrh2 | NM_001098810.2 | chr11:86684080-86692457 | 17815 | Rab1b | NM_029576.3 | chr19:5099206-5106996 |
| 17719 | Ptrhd1 | NM_001204912.1 | chr12:4234026-4240123 | 17816 | Rab20 | NM_011227.1 | chr8:11453976-11478499 |
| 17720 | Pts | NM_011220.2 | chr9:50521616-50528641 | 17817 | Rab21 | NM_024454.1 | chr10:115289861-115315591 |
| 17721 | Pttg1 | NM_001131054.1 | chr11:43420247-43426248 | 17818 | Rab22a | NM_024436.3 | chr2:173659844-173702182 |
| 17722 | Pttg1ip | NM_145925.2 | chr10:77581766-77598732 | 17819 | Rab23 | NM_001159729.1 | chr1:33720418-33742564 |
| 17723 | Ptx3 | NM_008987.3 | chr3:66219886-66225806 | 17820 | Rab24 | NM_009000.3 | chr13:55319222-55321980 |
| 17724 | Ptx4 | NM_001163416.1 | chr17:25120759-25125268 | 17821 | Rab25 | NM_016899.4 | chr3:88542028-88548279 |
| 17725 | Puf60 | NM_001164600.1 | chr15:76070181-76080870 | 17822 | Rab26 | NM_177375.1 | chr17:24529053-24533747 |
| 17726 | Pum1 | NM_001159603.1 | chr4:130663358-130781565 | 17823 | Rab26os | NR_045289.1 | chr17:24528250-24528744 |
| 17727 | Pum2 | NM_001160219.1 | chr12:8674258-8752583 | 17824 | Rab27a | NM_023635.6 | chr9:73044809-73097614 |
| 17728 | Pura | NM_008989.3 | chr18:36281161-36288244 | 17825 | Rab27b | NM_001082553.2 | chr18:69979130-70141605 |
| 17729 | Purb | NM_011221.3 | chr11:6467598-6476076 | 17826 | Rab28 | NM_027295.3 | chr5:41624972-41708179 |
| 17730 | Purg | NM_001098233.1 | chr8:33386324-33417469 | 17827 | Rab2a | NM_021518.3 | chr4:8535643-8607702 |
| 17731 | Pus1 | NM_001025561.3 | chr5:110773666-110780596 | 17828 | Rab2b | NM_172601.3 | chr14:52261758-52279395 |
| 17732 | Pus10 | NM_001033654.2 | chr11:23665673-23732876 | 17829 | Rab30 | NM_029494.2 | chr7:92741713-92837117 |
| 17733 | Pus3 | NM_023292.4 | chr9:35559465-35567400 | 17830 | Rab31 | NM_133685.2 | chr17:65651725-65772752 |
| 17734 | Pus7 | NM_001289780.1 | chr5:23740164-23783711 | 17831 | Rab32 | NM_026405.3 | chr10:10545038-10558207 |
| 17735 | Pus7l | NM_172437.3 | chr15:94522639-94543507 | 17832 | Rab33a | NM_011228.2 | chrX:48519284-48530240 |
| 17736 | Pusl1 | NM_001033490.1 | chr4:155888859-155891762 | 17833 | Rab33b | NM_016858.2 | chr3:51483965-51496212 |
| 17737 | Pvalb | NM_013645.3 | chr15:78191117-78206351 | 17834 | Rab34 | NM_001159482.1 | chr11:78188426-78192193 |
| 17738 | Pvr | NM_027514.2 | chr7:19903577-19921143 | 17835 | Rab35 | NM_198163.1 | chr5:115631986-115647158 |
| 17739 | Pvrl1 | NM_021424.2 | chr9:43744575-43807461 | 17836 | Rab36 | NM_029781.3 | chr10:75037088-75054100 |
| 17740 | Pvrl2 | NM_001159724.1 | chr7:19724160-19749573 | 17837 | Rab37 | NM_001163753.1 | chr11:115091430-115162240 |
| 17741 | Pvrl3 | NM_021495.4 | chr16:46447160-46496967 | 17838 | Rab38 | NM_028238.7 | chr7:88430272-88491572 |
| 17742 | Pvrl4 | NM_001122680.1 | chr1:171370172-171388287 | 17839 | Rab39 | NM_175562.3 | chr9:53684109-53706232 |
| 17743 | Pvt1 | NR_003368.2 | chr15:62037985-62250976 | 17840 | Rab39b | NM_175122.6 | chrX:75572044-75578231 |
| 17744 | Pwp1 | NM_133993.3 | chr10:85871830-85889103 | 17841 | Rab3a | NM_001166399.2 | chr8:70754678-70758686 |
| 17745 | Pwp2 | NM_029546.2 | chr10:78170909-78185149 | 17842 | Rab3b | NM_023537.5 | chr4:108879069-108943324 |
| 17746 | Pwwp2a | NM_001164231.1 | chr11:43681997-43722633 | 17843 | Rab3c | NM_023852.5 | chr13:110054186-110280206 |
| 17747 | Pwwp2b | NM_001033206.2 | chr7:139248481-139267253 | 17844 | Rab3d | NM_031874.4 | chr9:21907510-21918121 |
| 17748 | Pxdc1 | NM_025831.3 | chr13:34627840-34652681 | 17845 | Rab3gap1 | NM_178690.4 | chr1:127868772-127943876 |
| 17749 | Pxdn | NM_181395.2 | chr12:29938035-30017658 | 17846 | Rab3gap2 | NM_001163754.1 | chr1:185204167-185286746 |
| 17750 | Pxk | NM_145458.3 | chr14:8098212-8165111 | 17847 | Rab3il1 | NM_144538.2 | chr19:10018227-10035586 |

Fig.21 - 93

| | | | | | | | |
|---|---|---|---|---|---|---|---|
| 17848 | Rab3ip | NM_001003950.2 | chr10:116905783-116950380 | 17945 | Rap1gap2 | NM_001015046.2 | chr11:74383482-74590158 |
| 17849 | Rab40b | NM_139147.3 | chr11:121356120-121388251 | 17946 | Rap1gds1 | NM_001040690.2 | chr3:138925896-139075201 |
| 17850 | Rab40c | NM_139154.2 | chr17:25882113-25919714 | 17947 | Rap2a | NM_029519.3 | chr14:120478460-120507192 |
| 17851 | Rab42 | NM_001081651.1 | chr4:132302193-132303356 | 17948 | Rap2b | NM_028712.4 | chr3:61364506-61368703 |
| 17852 | Rab43 | NM_001039394.1 | chr6:87788852-87811779 | 17949 | Rap2c | NM_172413.2 | chrX:51003913-51018018 |
| 17853 | Rab44 | NM_001002786.2 | chr17:29135055-29148976 | 17950 | Rapgef1 | NM_001039086.1 | chr2:29619719-29740363 |
| 17854 | Rab4a | NM_009003.3 | chr8:123805995-123835291 | 17951 | Rapgef2 | NM_001099624.3 | chr3:79062510-79145875 |
| 17855 | Rab4b | NM_029391.2 | chr7:27168432-27178883 | 17952 | Rapgef3 | NM_001177810.1 | chr15:97744769-97767666 |
| 17856 | Rab5a | NM_025887.4 | chr17:53479233-53507678 | 17953 | Rapgef4 | NM_001204165.1 | chr2:71981214-72257474 |
| 17857 | Rab5b | NM_177411.4 | chr10:128677182-128696268 | 17954 | Rapgef5 | NM_175930.5 | chr12:117516478-117756978 |
| 17858 | Rab5c | NM_024456.5 | chr11:100715002-100738215 | 17955 | Rapgef6 | NM_001252494.1 | chr11:54522844-54699286 |
| 17859 | Rab6a | NM_001163663.1 | chr7:100607585-100641268 | 17956 | Rapgefl1 | NM_001080925.1 | chr11:98836784-98853005 |
| 17860 | Rab6b | NM_173781.4 | chr9:103112073-103185270 | 17957 | Raph1 | NM_001045513.3 | chr1:60483184-60566765 |
| 17861 | Rab7 | NM_009005.3 | chr6:87999105-88045270 | 17958 | Rapsn | NM_009023.3 | chr2:91035626-91045729 |
| 17862 | Rab7l1 | NM_144875.2 | chr1:131867276-131872889 | 17959 | Rara | NM_001176528.1 | chr11:98960470-98974942 |
| 17863 | Rab8a | NM_023126.2 | chr8:72161199-72181366 | 17960 | Rarb | NM_001289760.1 | chr14:16430839-17082331 |
| 17864 | Rab8b | NM_173413.3 | chr9:66843663-66919705 | 17961 | Rarg | NM_001042727.2 | chr15:102234937-102246500 |
| 17865 | Rab9 | NM_019773.2 | chrX:166457251-166479867 | 17962 | Rarres1 | NM_001164763.1 | chr3:67478885-67515523 |
| 17866 | Rab9b | NM_176971.2 | chrX:136858150-136868540 | 17963 | Rarres2 | NM_027852.2 | chr6:48569697-48572670 |
| 17867 | Rabac1 | NM_010261.2 | chr7:24969749-24972728 | 17964 | Rars | NM_025936.3 | chr11:35808380-35834528 |
| 17868 | Rabep1 | NM_001291141.1 | chr11:70844760-70943105 | 17965 | Rars2 | NM_181406.3 | chr4:34614957-34660167 |
| 17869 | Rabep2 | NM_030566.2 | chr7:126428766-126445907 | 17966 | Rasa1 | NM_145452.3 | chr13:85214698-85289486 |
| 17870 | Rabepk | NM_145522.4 | chr2:34778665-34799912 | 17967 | Rasa2 | NM_053268.2 | chr9:96539299-96631503 |
| 17871 | Rabgap1 | NM_001033960.1 | chr2:37452253-37544962 | 17968 | Rasa3 | NM_009025.2 | chr8:13567217-13677587 |
| 17872 | Rabgap1l | NM_001038621.2 | chr1:160219173-160351571 | 17969 | Rasa4 | NM_001039103.3 | chr5:136083915-136111861 |
| 17873 | Rabgef1 | NM_001199059.1 | chr5:130171818-130214337 | 17970 | Rasal1 | NM_001281999.1 | chr5:120649187-120679610 |
| 17874 | Rabggta | NM_019519.2 | chr14:55715876-55722176 | 17971 | Rasal2 | NM_177644.5 | chr1:157135182-157412595 |
| 17875 | Rabggtb | NM_001163478.1 | chr3:153907288-153912966 | 17972 | Rasal3 | NM_178785.3 | chr17:32390660-32403581 |
| 17876 | Rabif | NM_145510.1 | chr1:134494659-134507884 | 17973 | Rasd1 | NM_009026.5 | chr11:59963180-59964944 |
| 17877 | Rabl2 | NM_026817.3 | chr15:89582526-89591923 | 17974 | Rasd2 | NM_029182.1 | chr8:75213943-75224113 |
| 17878 | Rabl3 | NM_001042499.1 | chr16:37539893-37572385 | 17975 | Rasef | NM_001017427.1 | chr4:73714578-73790602 |
| 17879 | Rabl6 | NM_001024616.1 | chr2:25583017-25608446 | 17976 | Rasgef1a | NM_027526.1 | chr6:118066384-118091546 |
| 17880 | Rac1 | NM_009007.2 | chr5:143505480-143527993 | 17977 | Rasgef1b | NM_145839.2 | chr5:99217419-99252927 |
| 17881 | Rac2 | NM_009008.3 | chr15:78559168-78572783 | 17978 | Rasgef1c | NM_029004.1 | chr11:49901834-49980223 |
| 17882 | Rac3 | NM_133223.4 | chr11:120721467-120723969 | 17979 | Rasgrf1 | NM_001039655.1 | chr9:89909774-89915847 |
| 17883 | Racgap1 | NM_001253808.1 | chr15:99620495-99651656 | 17980 | Rasgrf2 | NM_009027.3 | chr13:91880406-91988042 |
| 17884 | Rad1 | NM_001289447.1 | chr15:10486037-10493695 | 17981 | Rasgrp1 | NM_011246.2 | chr2:117279992-117342877 |
| 17885 | Rad17 | NM_001044371.2 | chr13:100617163-100651061 | 17982 | Rasgrp2 | NM_011242.2 | chr19:6400582-6415216 |
| 17886 | Rad18 | NM_001167730.1 | chr6:112619850-112696670 | 17983 | Rasgrp3 | NM_001166493.1 | chr17:75435904-75529053 |
| 17887 | Rad21 | NM_009009.4 | chr15:51962603-51991760 | 17984 | Rasgrp4 | NM_001174155.1 | chr7:29134932-29153955 |
| 17888 | Rad21l | NM_001276400.1 | chr2:151645403-151668533 | 17985 | Rasip1 | NM_028544.1 | chr7:45627536-45639092 |
| 17889 | Rad23a | NM_009010.5 | chr8:84834651-84840665 | 17986 | Rasl10a | NM_145216.3 | chr11:5058127-5060383 |
| 17890 | Rad23b | NM_009011.4 | chr4:55350041-55392237 | 17987 | Rasl10b | NM_001013386.2 | chr11:83410071-83421038 |
| 17891 | Rad50 | NM_009012.2 | chr11:53649518-53707319 | 17988 | Rasl11a | NM_026864.1 | chr5:146845070-146847726 |
| 17892 | Rad51 | NM_011234.4 | chr2:119112816-119136070 | 17989 | Rasl11b | NM_026878.1 | chr5:74195325-74199477 |
| 17893 | Rad51ap1 | NM_009013.3 | chr6:126923418-126939555 | 17990 | Rasl12 | NM_001033158.2 | chr9:65398487-65412707 |
| 17894 | Rad51ap2 | NM_001111118.1 | chr12:11456078-11462928 | 17991 | Rasl2-9 | NM_009028.2 | chr7:5124941-5125950 |
| 17895 | Rad51b | NM_001252562.1 | chr12:79297350-79508654 | 17992 | Rassf1 | NM_001243748.1 | chr9:107551554-107562267 |
| 17896 | Rad51c | NM_001291440.1 | chr11:87377606-87404954 | 17993 | Rassf10 | NM_175279.3 | chr7:112953961-112957458 |
| 17897 | Rad51d | NM_001277938.1 | chr11:82873062-82890624 | 17994 | Rassf2 | NM_175445.4 | chr2:131992849-132029988 |
| 17898 | Rad52 | NM_001166381.1 | chr6:119902697-119922823 | 17995 | Rassf3 | NM_138956.3 | chr10:121410350-121476250 |
| 17899 | Rad54b | NM_001039556.3 | chr4:11558919-11615808 | 17996 | Rassf4 | NM_178045.4 | chr6:116633007-116673836 |
| 17900 | Rad54l | NM_001122958.1 | chr4:116096954-116123689 | 17997 | Rassf5 | NM_018750.4 | chr1:131176409-131245178 |
| 17901 | Rad54l2 | NM_030730.2 | chr9:106688079-106789213 | 17998 | Rassf6 | NM_028478.3 | chr5:90603075-90640527 |
| 17902 | Rad9a | NM_011237.2 | chr19:4195197-4201603 | 17999 | Rassf7 | NM_025886.3 | chr7:141215859-141218658 |
| 17903 | Rad9b | NM_144912.3 | chr5:122325507-122354195 | 18000 | Rassf8 | NM_027760.2 | chr6:145808382-145817584 |
| 17904 | Radil | NM_001289588.1 | chr5:142484838-142551098 | 18001 | Rassf9 | NM_146240.4 | chr10:102512221-102546560 |
| 17905 | Rae1 | NM_175112.5 | chr2:173000116-173015739 | 18002 | Raver1 | NM_027911.3 | chr9:21074163-21091988 |
| 17906 | Raet1a | NM_009016.1 | chr10:22158608-22374139 | 18003 | Raver1-fdx1l | NR_038081.2 | chr9:21067513-21092008 |
| 17907 | Raet1b | NM_009017.1 | chr10:22173874-22374113 | 18004 | Raver2 | NM_183024.1 | chr4:101069037-101152370 |
| 17908 | Raet1c | NM_009018.1 | chr10:22173901-22374139 | 18005 | Rax | NM_013833.2 | chr18:65934638-65939089 |
| 17909 | Raet1d | NM_020030.2 | chr10:22361893-22374139 | 18006 | Rb1 | NM_009029.2 | chr14:73195501-73325791 |
| 17910 | Raet1e | NM_198193.2 | chr10:22173520-22183914 | 18007 | Rb1cc1 | NM_009826.4 | chr1:6214661-6276104 |
| 17911 | Raf1 | NM_029780.3 | chr6:115618572-115676635 | 18008 | Rbak | NM_001045482.2 | chr5:143172185-143180775 |
| 17912 | Rag1 | NM_009019.2 | chr2:101638251-101649532 | 18009 | Rbakdn | NR_040424.1 | chr5:143164778-143165751 |
| 17913 | Rag2 | NM_009020.3 | chr2:101624747-101632528 | 18010 | Rbbp4 | NM_009030.3 | chr4:129307099-129335370 |
| 17914 | Rai1 | NM_001037764.1 | chr11:60140081-60199195 | 18011 | Rbbp5 | NM_172517.2 | chr1:132477366-132505665 |
| 17915 | Rai14 | NM_001166408.1 | chr15:10568977-10714631 | 18012 | Rbbp6 | NM_011247.2 | chr7:122970563-123002572 |
| 17916 | Rai2 | NM_001103367.1 | chrX:161717035-161779494 | 18013 | Rbbp7 | NM_009031.3 | chrX:162760371-162779090 |
| 17917 | Rala | NM_019491.5 | chr13:17880574-17944217 | 18014 | Rbbp8 | NM_001081223.2 | chr18:11657348-11743207 |
| 17918 | Ralb | NM_022327.5 | chr1:119470304-119504782 | 18015 | Rbbp8nl | NM_173031.3 | chr2:180277645-180289879 |
| 17919 | Ralbp1 | NM_001198949.1 | chr17:65848427-65884923 | 18016 | Rbbp9 | NM_015754.2 | chr2:144542264-144550859 |
| 17920 | Ralgapa1 | NM_001003719.2 | chr12:55602889-55821516 | 18017 | Rbck1 | NM_001083921.1 | chr2:152316333-152332425 |
| 17921 | Ralgapa2 | NM_001033348.3 | chr2:146241298-146512004 | 18018 | Rbfa | NM_199197.1 | chr18:80192263-80200619 |
| 17922 | Ralgapb | NM_001291137.1 | chr2:158409852-158499253 | 18019 | Rbfox1 | NM_021477.5 | chr16:5884792-7412480 |
| 17923 | Ralgds | NM_001145834.1 | chr2:28513166-28553082 | 18020 | Rbfox2 | NM_001110827.2 | chr15:77078989-77307053 |
| 17924 | Ralgps1 | NM_001290570.1 | chr2:33133418-33371494 | 18021 | Rbfox3 | NM_001024931.2 | chr11:118489759-118909572 |
| 17925 | Ralgps2 | NM_001159965.1 | chr1:156804165-156939626 | 18022 | Rbks | NM_153196.1 | chr5:31624438-31697610 |
| 17926 | Raly | NM_001139511.1 | chr2:154791109-154867261 | 18023 | Rbl1 | NM_001139516.1 | chr2:157174995-157204534 |
| 17927 | Ralyl | NM_001163328.1 | chr3:13471654-14182287 | 18024 | Rbl2 | NM_001282000.1 | chr8:91070056-91123844 |
| 17928 | Ramp1 | NM_001168392.1 | chr1:91179821-91206790 | 18025 | Rbm10 | NM_001167775.1 | chrX:20617502-20650905 |
| 17929 | Ramp2 | NM_019444.2 | chr11:101246333-101248250 | 18026 | Rbm11 | NM_198302.2 | chr16:75592890-75602825 |
| 17930 | Ramp3 | NM_019511.3 | chr11:6658532-6677475 | 18027 | Rbm12 | NM_029397.3 | chr2:156094881-156111965 |
| 17931 | Ran | NM_009391.3 | chr5:129020155-129024321 | 18028 | Rbm12b1 | NM_028226.2 | chr4:12140116-12146746 |
| 17932 | Ranbp1 | NM_011239.2 | chr16:18239978-18248694 | 18029 | Rbm12b2 | NM_198957.2 | chr4:12089369-12096271 |
| 17933 | Ranbp10 | NM_145824.4 | chr8:105768307-105827350 | 18030 | Rbm14 | NM_019869.3 | chr19:4800565-4811634 |
| 17934 | Ranbp17 | NM_023146.2 | chr11:33211793-33513746 | 18031 | Rbm14-rbm4 | NM_001290127.1 | chr19:4784292-4811634 |
| 17935 | Ranbp2 | NM_011240.3 | chr10:58446851-58494154 | 18032 | Rbm15 | NM_001045807.1 | chr3:107326109-107333289 |
| 17936 | Ranbp3 | NM_001252466.1 | chr17:56673224-56711769 | 18033 | Rbm15b | NM_175402.4 | chr9:106883984-106887000 |
| 17937 | Ranbp3l | NM_198024.2 | chr15:8967948-9067333 | 18034 | Rbm17 | NM_152824.1 | chr2:11585438-11603199 |
| 17938 | Ranbp6 | NM_177721.4 | chr19:29808107-29812974 | 18035 | Rbm18 | NM_001159635.1 | chr2:36116078-36136704 |
| 17939 | Ranbp9 | NM_019930.2 | chr13:43402672-43480973 | 18036 | Rbm19 | NM_028762.1 | chr5:120116512-120198971 |
| 17940 | Rangap1 | NM_001146174.1 | chr15:81704247-81729919 | 18037 | Rbm20 | NM_001170847.1 | chr19:53677305-53867080 |
| 17941 | Rangrf | NM_001285441.1 | chr11:68972483-68975185 | 18038 | Rbm22 | NM_025776.2 | chr18:60560785-60572729 |
| 17942 | Rap1a | NM_145541.5 | chr3:105727259-105801381 | 18039 | Rbm24 | NM_001081425.1 | chr13:46418299-46431099 |
| 17943 | Rap1b | NM_024457.2 | chr10:117814596-117845974 | 18040 | Rbm25 | NM_027349.3 | chr12:83632233-83683123 |
| 17944 | Rap1gap | NM_001081155.2 | chr4:137681666-137729861 | 18041 | Rbm26 | NM_134077.4 | chr14:105114518-105177327 |

Fig.21 - 94

| | | | | | | | |
|---|---|---|---|---|---|---|---|
| 18042 | Rbm27 | NM_172626.2 | chr18:42275352-42341540 | 18139 | Reg3g | NM_011260.1 | chr6:78466267-78468874 |
| 18043 | Rbm28 | NM_133925.1 | chr6:29123572-29164724 | 18140 | Reg4 | NM_026328.2 | chr3:98222155-98236748 |
| 18044 | Rbm3 | NM_001166409.2 | chrX:8142355-8145880 | 18141 | Rel | NM_009044.2 | chr11:23741728-23770970 |
| 18045 | Rbm31y | NM_028970.1 | chrY:12688109-17402718 | 18142 | Rela | NM_009045.4 | chr19:5637489-5648130 |
| 18046 | Rbm33 | NM_028234.1 | chr5:28317188-28419242 | 18143 | Relb | NM_001290457.1 | chr7:19606222-19629438 |
| 18047 | Rbm34 | NM_172762.2 | chr8:126947172-126971079 | 18144 | Rell1 | NM_145923.4 | chr5:63908897-63968897 |
| 18048 | Rbm38 | NM_019547.2 | chr2:173021901-173034731 | 18145 | Rell2 | NM_153793.2 | chr18:37955558-37959179 |
| 18049 | Rbm39 | NM_001291114.1 | chr2:156147240-156180238 | 18146 | Reln | NM_011261.2 | chr5:21884453-22344705 |
| 18050 | Rbm3os | NR_033561.1 | chrX:8145426-8147963 | 18147 | Relt | NM_177073.6 | chr7:100845847-100863413 |
| 18051 | Rbm4 | NM_001290122.1 | chr19:4784292-4794009 | 18148 | Rem1 | NM_009047.5 | chr2:152627007-152635191 |
| 18052 | Rbm41 | NM_001172147.1 | chrX:139941527-139998595 | 18149 | Rem2 | NM_080726.3 | chr14:54476099-54480434 |
| 18053 | Rbm42 | NM_133693.2 | chr7:30640994-30650228 | 18150 | Ren1 | NM_031192.3 | chr1:133350673-133360319 |
| 18054 | Rbm43 | NM_001141981.1 | chr2:51924448-51935009 | 18151 | Ren2 | NM_031193.2 | chr1:133350565-133360323 |
| 18055 | Rbm44 | NM_001033408.4 | chr1:91145102-91170795 | 18152 | Renbp | NM_001164704.1 | chrX:73922120-73930850 |
| 18056 | Rbm45 | NM_153405.2 | chr2:76369983-76383767 | 18153 | Rep15 | NM_025620.2 | chr6:147032536-147033518 |
| 18057 | Rbm46 | NM_001277170.1 | chr3:82836522-82876483 | 18154 | Repin1 | NM_001079901.1 | chr6:48593882-48599082 |
| 18058 | Rbm46os | NR_040381.1 | chr3:82904476-82943638 | 18155 | Reps1 | NM_001111065.1 | chr10:18055939-18125155 |
| 18059 | Rbm47 | NM_001127382.2 | chr5:66016548-66151954 | 18156 | Reps2 | NM_001290633.1 | chrX:162411951-162643658 |
| 18060 | Rbm48 | NM_172991.4 | chr5:3583977-3596547 | 18157 | Rer1 | NM_026395.1 | chr4:155074111-155086297 |
| 18061 | Rbm4b | NM_025717.3 | chr19:4756524-4765941 | 18158 | Rere | NM_001085492.1 | chr4:150281915-150621966 |
| 18062 | Rbm5 | NM_148930.3 | chr9:107740494-107771002 | 18159 | Rerg | NM_001164212.1 | chr6:137054824-137169718 |
| 18063 | Rbm6 | NM_011251.3 | chr9:107773558-107872819 | 18160 | Rergl | NM_001128090.1 | chr6:139493181-139501909 |
| 18064 | Rbm7 | NM_144948.5 | chr9:48488696-48495330 | 18161 | Resp18 | NM_009049.1 | chr1:75272201-75278380 |
| 18065 | Rbm8a | NM_001102407.1 | chr3:96629927-96633791 | 18162 | Rest | NM_011263.2 | chr5:77265493-77283697 |
| 18066 | Rbms1 | NM_001141931.1 | chr2:60751952-60881438 | 18163 | Ret | NM_001080780.1 | chr6:118151747-118197744 |
| 18067 | Rbms2 | NM_001039080.1 | chr10:128129469-128180297 | 18164 | Retn | NM_001204959.1 | chr8:3655769-3659818 |
| 18068 | Rbms3 | NM_001172121.1 | chr9:116572746-117629913 | 18165 | Retnla | NM_020509.3 | chr16:48842551-48844461 |
| 18069 | Rbmx | NM_001166623.1 | chrX:57386029-57393036 | 18166 | Retnlb | NM_023881.4 | chr16:48816855-48818892 |
| 18070 | Rbmx2 | NM_173376.3 | chrX:48695003-48710719 | 18167 | Retnlg | NM_181596.4 | chr16:48872607-48874498 |
| 18071 | Rbmxl1 | NM_001252089.1 | chr8:78505268-78508928 | 18168 | Retsat | NM_026159.4 | chr6:72598627-72607488 |
| 18072 | Rbmxl2 | NM_029660.2 | chr7:107209444-107210916 | 18169 | Rev1 | NM_019570.3 | chr1:38052785-38129662 |
| 18073 | Rbmy | NM_011253.2 | chrY:2830679-3783271 | 18170 | Rev3l | NM_011264.3 | chr10:39732159-39875205 |
| 18074 | Rbp1 | NM_011254.5 | chr9:98422960-98446550 | 18171 | Rex2 | NM_001177767.1 | chr4:147021849-147060799 |
| 18075 | Rbp2 | NM_009034.4 | chr9:98490536-98509771 | 18172 | Rexo1 | NM_025852.3 | chr10:80540925-80561560 |
| 18076 | Rbp3 | NM_015745.2 | chr14:33954021-33964216 | 18173 | Rexo2 | NM_024233.3 | chr9:48468513-48480611 |
| 18077 | Rbp4 | NM_001159487.1 | chr19:38116619-38125321 | 18174 | Rexo4 | NM_207234.2 | chr2:26953562-26964386 |
| 18078 | Rbp7 | NM_022020.2 | chr4:149449701-149454968 | 18175 | Rfc1 | NM_011258.2 | chr5:65261851-65335639 |
| 18079 | Rbpj | NM_001080927.2 | chr5:53590485-53657445 | 18176 | Rfc2 | NM_020022.2 | chr5:134582689-134598328 |
| 18080 | Rbpjl | NM_009036.1 | chr2:164403193-164415448 | 18177 | Rfc3 | NM_027009.2 | chr5:151642823-151651208 |
| 18081 | Rbpms | NM_001042674.2 | chr8:33782643-33929863 | 18178 | Rfc4 | NM_145480.1 | chr16:23113947-23127730 |
| 18082 | Rbpms2 | NM_028030.3 | chr9:65630581-65660518 | 18179 | Rfc5 | NM_028128.1 | chr5:117379144-117389023 |
| 18083 | Rbx1 | NM_019712.3 | chr15:81466315-81476369 | 18180 | Rfesd | NM_001131068.1 | chr13:76001534-76018606 |
| 18084 | Rc3h1 | NM_001024952.2 | chr1:160906410-160974976 | 18181 | Rffl | NM_001007465.3 | chr11:82803818-82871210 |
| 18085 | Rc3h2 | NM_001100591.1 | chr2:37370070-37422903 | 18182 | Rfk | NM_019437.3 | chr19:17394042-17401349 |
| 18086 | Rcan1 | NM_001081549.2 | chr16:92391950-92466169 | 18183 | Rfng | NM_009053.2 | chr11:120780744-120784204 |
| 18087 | Rcan2 | NM_001286653.1 | chr17:43804002-44039516 | 18184 | Rfpl3s | NM_183111.2 | chr9:55962588-55980932 |
| 18088 | Rcan3 | NM_022980.4 | chr4:135412308-135433805 | 18185 | Rfpl4 | NM_001145013.1 | chr7:5109786-5116911 |
| 18089 | Rcbtb1 | NM_027764.2 | chr14:59201227-59237265 | 18186 | Rfpl4b | NM_001177783.1 | chr10:38820540-38821779 |
| 18090 | Rcbtb2 | NM_001170694.1 | chr14:73142509-73184054 | 18187 | Rft1 | NM_177815.3 | chr14:30654374-30691313 |
| 18091 | Rcc1 | NM_001197082.1 | chr4:132331918-132345750 | 18188 | Rftn1 | NM_181397.2 | chr17:49993306-50190497 |
| 18092 | Rcc2 | NM_173867.5 | chr4:140701472-140723220 | 18189 | Rftn2 | NM_028713.1 | chr1:55170159-55226782 |
| 18093 | Rccd1 | NM_173445.4 | chr7:80316615-80324454 | 18190 | Rfwd2 | NM_011931.3 | chr1:159232325-159347580 |
| 18094 | Rce1 | NM_023131.1 | chr19:4622550-4625617 | 18191 | Rfwd3 | NM_146218.4 | chr8:111270943-111300222 |
| 18095 | Rchy1 | NM_001271797.1 | chr5:91948841-91963068 | 18192 | Rfx1 | NM_009055.4 | chr8:84066835-84096992 |
| 18096 | Rcl1 | NM_021525.2 | chr19:29101374-29143843 | 18193 | Rfx2 | NM_009056.2 | chr17:56775896-56831008 |
| 18097 | Rcn1 | NM_009037.2 | chr2:105385947-105399319 | 18194 | Rfx3 | NM_001166414.1 | chr19:27761720-27982948 |
| 18098 | Rcn2 | NM_001278274.1 | chr9:56041844-56043245 | 18195 | Rfx4 | NM_001024918.1 | chr10:84756047-84906536 |
| 18099 | Rcn3 | NM_026555.2 | chr7:45082913-45092213 | 18196 | Rfx5 | NM_017395.2 | chr3:94955014-94961561 |
| 18100 | Rcor1 | NM_198023.2 | chr12:111039797-111113386 | 18197 | Rfx6 | NM_001159389.1 | chr10:51677759-51730429 |
| 18101 | Rcor2 | NM_054048.3 | chr19:7269763-7275225 | 18198 | Rfx7 | NM_001033536.1 | chr9:72532239-72622949 |
| 18102 | Rcor3 | NM_001290278.1 | chr1:192098545-192138040 | 18199 | Rfx8 | NM_001145660.1 | chr1:39665300-39720989 |
| 18103 | Rcsd1 | NM_001038846.1 | chr1:165648944-165708094 | 18200 | Rfxank | NM_001025589.1 | chr8:70130805-70139197 |
| 18104 | Rcvrn | NM_009038.2 | chr11:67695325-67703355 | 18201 | Rfxap | NM_133231.2 | chr3:54803114-54807791 |
| 18105 | Rd3 | NM_001177900.2 | chr1:191977369-191988282 | 18202 | Rgag1 | NM_001040434.2 | chrX:143099593-143104335 |
| 18106 | Rd3l | NM_001127685.1 | chr12:111979322-111980751 | 18203 | Rgag4 | NM_001278534.1 | chrX:102066541-102071307 |
| 18107 | Rdh1 | NM_080436.3 | chr10:127759762-127768299 | 18204 | Rgcc | NM_025427.2 | chr14:79288749-79301635 |
| 18108 | Rdh10 | NM_133832.3 | chr1:16105881-16132550 | 18205 | Rgl1 | NM_016846.3 | chr1:152517529-152625111 |
| 18109 | Rdh11 | NM_021557.5 | chr12:79175550-79191819 | 18206 | Rgl2 | NM_009059.2 | chr17:33929893-33937687 |
| 18110 | Rdh12 | NM_030017.4 | chr12:79208912-79222664 | 18207 | Rgl3 | NM_023622.4 | chr9:21971526-21989453 |
| 18111 | Rdh13 | NM_001290409.1 | chr7:4425664-4445657 | 18208 | Rgma | NM_177740.5 | chr7:73375519-73419899 |
| 18112 | Rdh14 | NM_023697.2 | chr12:10390779-10395562 | 18209 | Rgmb | NM_178615.3 | chr17:15806252-15826586 |
| 18113 | Rdh16 | NM_009040.3 | chr10:127801152-127815839 | 18210 | Rgn | NM_009060.2 | chrX:20549817-20562087 |
| 18114 | Rdh18-ps | NR_037604.1 | chr10:127824215-127846565 | 18211 | Rgp1 | NM_172866.3 | chr4:43578734-43587487 |
| 18115 | Rdh19 | NM_147222.2 | chr10:127849927-127861176 | 18212 | Rgr | NM_021340.4 | chr14:37034908-37049014 |
| 18116 | Rdh5 | NM_134006.4 | chr10:128913590-128919297 | 18213 | Rgs1 | NM_015811.2 | chr1:144244668-144249104 |
| 18117 | Rdh7 | NM_001150749.1 | chr10:127884026-127888733 | 18214 | Rgs10 | NM_026418.2 | chr7:128373624-128418172 |
| 18118 | Rdh8 | NM_001030290.1 | chr9:20818503-20826163 | 18215 | Rgs11 | NM_001081069.1 | chr17:26202961-26211324 |
| 18119 | Rdh9 | NM_153133.2 | chr10:127776404-127792697 | 18216 | Rgs12 | NM_001163512.1 | chr5:34999077-35033593 |
| 18120 | Rdm1 | NM_025654.2 | chr11:101627948-101636081 | 18217 | Rgs13 | NM_153171.4 | chr1:144138666-144177372 |
| 18121 | Rdx | NM_001104616.1 | chr9:52047149-52088738 | 18218 | Rgs14 | NM_016758.3 | chr13:55369731-55384687 |
| 18122 | Rec8 | NM_020002.3 | chr14:55618165-55625395 | 18219 | Rgs16 | NM_011267.3 | chr1:153740352-153745468 |
| 18123 | Reck | NM_016678.2 | chr4:43875529-43944806 | 18220 | Rgs17 | NM_001161822.1 | chr10:5825663-5922400 |
| 18124 | Recql | NM_001204906.1 | chr6:142356946-142387087 | 18221 | Rgs18 | NM_022881.4 | chr1:144752840-144775421 |
| 18125 | Recql4 | NM_058214.3 | chr15:76703552-76710559 | 18222 | Rgs19 | NM_001291205.1 | chr2:181688418-181691817 |
| 18126 | Recql5 | NM_130454.2 | chr11:115892594-115933492 | 18223 | Rgs2 | NM_009061.4 | chr1:143999337-144004149 |
| 18127 | Redrum | NR_040338.1 | chr18:54422294-54453294 | 18224 | Rgs20 | NM_001177795.1 | chr1:4909575-5070285 |
| 18128 | Reep1 | NM_178608.4 | chr6:71707680-71810705 | 18225 | Rgs21 | NM_001290269.1 | chr1:144519689-144567667 |
| 18129 | Reep2 | NM_001204914.1 | chr18:34840588-34847463 | 18226 | Rgs22 | NM_001195748.1 | chr15:36009476-36140400 |
| 18130 | Reep3 | NM_001204915.1 | chr10:67009188-67096988 | 18227 | Rgs3 | NM_001081650.2 | chr4:62619671-62659847 |
| 18131 | Reep4 | NM_180588.2 | chr14:70545250-70548935 | 18228 | Rgs4 | NM_009062.3 | chr1:169741476-169747642 |
| 18132 | Reep5 | NM_007874.3 | chr18:34344885-34373415 | 18229 | Rgs5 | NM_009063.4 | chr1:169655500-169695813 |
| 18133 | Reep6 | NM_001204931.1 | chr10:80330144-80336441 | 18230 | Rgs6 | NM_001282061.2 | chr12:82617498-83162056 |
| 18134 | Reg1 | NM_009042.1 | chr6:78425982-78428666 | 18231 | Rgs7 | NM_001199003.1 | chr1:175059075-175492545 |
| 18135 | Reg2 | NM_009043.1 | chr6:78405154-78408097 | 18232 | Rgs7bp | NM_029879.2 | chr13:104947152-105054930 |
| 18136 | Reg3a | NM_011259.1 | chr6:78380708-78383839 | 18233 | Rgs8 | NM_026380.3 | chr1:153653036-153697665 |
| 18137 | Reg3b | NM_011036.1 | chr6:78370884-78373466 | 18234 | Rgs9 | NM_001165934.1 | chr11:109229855-109298181 |
| 18138 | Reg3d | NM_001161741.1 | chr6:78375873-78378865 | 18235 | Rgs9bp | NM_145840.3 | chr7:35578995-35585582 |

Fig.21 - 95

| | | | |
|---|---|---|---|
| 18236 | Rgsl1 | NM_001243223.1 | chr1:153779386-153844141 |
| 18237 | Rhag | NM_011269.2 | chr17:40811148-40840754 |
| 18238 | Rhbdd1 | NM_001122685.1 | chr1:82339048-82445367 |
| 18239 | Rhbdd2 | NM_146002.2 | chr5:135632653-135646376 |
| 18240 | Rhbdd3 | NM_001290491.1 | chr11:5099272-5106100 |
| 18241 | Rhbdf1 | NM_001291818.1 | chr11:32209584-32222293 |
| 18242 | Rhbdf2 | NM_001167680.1 | chr11:116598165-116624252 |
| 18243 | Rhbdl1 | NM_144816.1 | chr17:25834464-25837127 |
| 18244 | Rhbdl2 | NM_183163.2 | chr4:123787874-123829904 |
| 18245 | Rhbdl3 | NM_139228.3 | chr11:80300911-80355986 |
| 18246 | Rhbg | NM_021375.3 | chr3:88242873-88254684 |
| 18247 | Rhcg | NM_019799.3 | chr7:79593362-79617657 |
| 18248 | Rhd | NM_011270.3 | chr4:134864535-134896172 |
| 18249 | Rheb | NM_053075.3 | chr5:24802822-24842361 |
| 18250 | Rhebl1 | NM_026967.4 | chr15:98877759-98881414 |
| 18251 | Rhno1 | NR_027359.1 | chr6:128356999-128362897 |
| 18252 | Rho | NM_145383.1 | chr6:115931926-115938830 |
| 18253 | Rhoa | NM_016802.5 | chr9:108306159-108337943 |
| 18254 | Rhob | NM_007483.2 | chr12:8497758-8499985 |
| 18255 | Rhobtb1 | NM_001081347.1 | chr10:69212656-69291784 |
| 18256 | Rhobtb2 | NM_153514.5 | chr14:69784989-69805545 |
| 18257 | Rhobtb3 | NM_028493.2 | chr13:75869536-75943824 |
| 18258 | Rhoc | NM_001291859.1 | chr3:104789033-104794459 |
| 18259 | Rhod | NM_007485.4 | chr19:4425456-4439424 |
| 18260 | Rhof | NM_175092.3 | chr5:123118179-123132629 |
| 18261 | Rhog | NM_019566.3 | chr7:102239122-102250118 |
| 18262 | Rhoh | NM_001081105.1 | chr5:65863568-65896700 |
| 18263 | Rhoj | NM_023275.2 | chr12:75308312-75401455 |
| 18264 | Rhoq | NM_145491.2 | chr17:86963110-87000069 |
| 18265 | Rhot1 | NM_001163354.1 | chr11:80209054-80267907 |
| 18266 | Rhot2 | NM_145999.2 | chr17:25838837-25844851 |
| 18267 | Rhou | NM_133955.4 | chr8:123653928-123663880 |
| 18268 | Rhov | NM_145530.2 | chr2:119269200-119271226 |
| 18269 | Rhox1 | NM_001025084.2 | chrX:37213804-37222258 |
| 18270 | Rhox10 | NM_001024850.2 | chrX:38066474-38071688 |
| 18271 | Rhox11 | NM_198598.2 | chrX:38076597-38085139 |
| 18272 | Rhox12 | NM_001025083.2 | chrX:38104061-38110907 |
| 18273 | Rhox13 | NM_001185002.1 | chrX:38120839-38129966 |
| 18274 | Rhox2a | NM_029203.2 | chrX:37244991-37249690 |
| 18275 | Rhox2b | NM_001099316.1 | chrX:37412104-37416806 |
| 18276 | Rhox2c | NM_001099318.1 | chrX:37453658-37458375 |
| 18277 | Rhox2d | NM_001081669.2 | chrX:37493409-37497715 |
| 18278 | Rhox2e | NM_001085348.1 | chrX:37530467-37541809 |
| 18279 | Rhox2f | NM_001085356.1 | chrX:37571420-37576159 |
| 18280 | Rhox2g | NM_001114153.1 | chrX:37639111-37643470 |
| 18281 | Rhox2h | NM_001100465.1 | chrX:37668996-37673277 |
| 18282 | Rhox3a | NM_194063.3 | chrX:37249918-37258978 |
| 18283 | Rhox3c | NM_001102457.4 | chrX:37469867-37473961 |
| 18284 | Rhox3e | NM_001184969.1 | chrX:37254877-37550897 |
| 18285 | Rhox3f | NM_001040089.3 | chrX:37581351-37585496 |
| 18286 | Rhox3g | NM_001145406.3 | chrX:37623430-37628139 |
| 18287 | Rhox3h | NM_001114157.1 | chrX:37657687-37668764 |
| 18288 | Rhox4a | NM_001039688.3 | chrX:37265374-37395620 |
| 18289 | Rhox4b | NM_021300.2 | chrX:37432493-37437278 |
| 18290 | Rhox4c | NM_001039689.1 | chrX:37480336-37485124 |
| 18291 | Rhox4d | NM_001039695.1 | chrX:37514534-37519175 |
| 18292 | Rhox4e | NM_201236.3 | chrX:37557411-37562282 |
| 18293 | Rhox4f | NM_001039696.1 | chrX:37602894-37607686 |
| 18294 | Rhox4g | NM_001039698.1 | chrX:37646500-37651327 |
| 18295 | Rhox5 | NM_008818.2 | chrX:37754607-37808878 |
| 18296 | Rhox6 | NM_008955.1 | chrX:37827054-37829857 |
| 18297 | Rhox7 | NM_001025086.2 | chrX:37831685-37841171 |
| 18298 | Rhox8 | NM_001004193.2 | chrX:37874775-37878944 |
| 18299 | Rhox9 | NM_023894.1 | chrX:37899096-37901770 |
| 18300 | Rhpn1 | NM_001163465.1 | chr15:75704287-75714419 |
| 18301 | Rhpn2 | NM_027897.4 | chr7:35334236-35392287 |
| 18302 | Rian | NR_028261.1 | chr12:109603944-109661711 |
| 18303 | Ribc1 | NM_025660.2 | chrX:152004583-152016295 |
| 18304 | Ribc2 | NM_026357.2 | chr15:85132098-85144569 |
| 18305 | Ric3 | NM_001038624.1 | chr7:109034318-109083324 |
| 18306 | Ric8 | NM_053194.4 | chr7:140857396-140863731 |
| 18307 | Ric8b | NM_001013441.2 | chr10:84917612-85018337 |
| 18308 | Rictor | NM_030168.3 | chr15:6708380-6800400 |
| 18309 | Rif1 | NM_175238.5 | chr2:52072836-52122381 |
| 18310 | Riiad1 | NM_025506.2 | chr3:94464984-94473591 |
| 18311 | Rilp | NM_001029938.2 | chr11:75510093-75513166 |
| 18312 | Rilpl1 | NM_021430.2 | chr5:124493079-124531391 |
| 18313 | Rilpl2 | NM_030259.1 | chr5:124463264-124478235 |
| 18314 | Rimbp2 | NM_001081388.2 | chr5:128757787-128953486 |
| 18315 | Rimbp3 | NM_001033338.3 | chr16:17208134-17213982 |
| 18316 | Rimkla | NM_177572.4 | chr4:119465284-119492598 |
| 18317 | Rimklb | NM_027664.1 | chr6:122453608-122486305 |
| 18318 | Rims1 | NM_001012623.1 | chr1:22288421-22805724 |
| 18319 | Rims2 | NM_001256382.1 | chr15:39198285-39684372 |
| 18320 | Rims3 | NM_182929.2 | chr4:120877868-120891560 |
| 18321 | Rims4 | NM_183023.1 | chr2:163863880-163918683 |
| 18322 | Rin1 | NM_145495.2 | chr19:5050807-5057071 |
| 18323 | Rin2 | NM_028724.4 | chr2:145786115-145887616 |
| 18324 | Rin3 | NM_001161365.1 | chr12:102283640-102390854 |
| 18325 | Ring1 | NM_009066.3 | chr17:34020791-34024680 |
| 18326 | Rinl | NM_177158.5 | chr7:28788968-28798966 |
| 18327 | Rint1 | NM_177323.4 | chr5:23787710-23820369 |
| 18328 | Riok1 | NM_024242.3 | chr13:38036988-38061433 |
| 18329 | Riok2 | NM_025934.2 | chr17:17374331-17394899 |
| 18330 | Riok3 | NM_024182.4 | chr18:12128849-12157367 |
| 18331 | Ripk1 | NM_009068.3 | chr13:34002873-34035170 |
| 18332 | Ripk2 | NM_138952.3 | chr4:16123374-16163498 |
| 18333 | Ripk3 | NM_001164107.1 | chr14:55784994-55788857 |
| 18334 | Ripk4 | NM_023663.6 | chr16:97741941-97763755 |
| 18335 | Ripply1 | NM_001037915.2 | chrX:139779680-139782353 |
| 18336 | Ripply2 | NM_001037907.1 | chr9:87015536-87019916 |
| 18337 | Ripply3 | NM_133229.2 | chr16:94328421-94336935 |
| 18338 | Rit1 | NM_001163310.1 | chr3:88716853-88731048 |
| 18339 | Rit2 | NM_009065.2 | chr18:30974313-31317128 |
| 18340 | Rita1 | NM_029096.3 | chr5:120609059-120612589 |
| 18341 | Rlbp1 | NM_001173483.1 | chr7:79374869-79387027 |
| 18342 | Rlf | NM_001081013.1 | chr4:121145372-121188534 |
| 18343 | Rlim | NM_011276.3 | chrX:103957166-103981284 |
| 18344 | Rln1 | NM_011272.2 | chr19:29331754-29334670 |
| 18345 | Rln3 | NM_173184.1 | chr8:84043066-84044979 |
| 18346 | Rltpr | NM_001033320.2 | chr8:105690905-105698165 |
| 18347 | Rmdn1 | NM_025476.6 | chr4:19575065-19606932 |
| 18348 | Rmdn2 | NM_201361.2 | chr17:79614899-79682152 |
| 18349 | Rmdn3 | NM_001033136.3 | chr2:119136997-119157034 |
| 18350 | Rmi1 | NM_001168248.1 | chr13:58402596-58411149 |
| 18351 | Rmi2 | NM_001162932.1 | chr16:10835058-10843235 |
| 18352 | Rmnd1 | NM_025343.5 | chr10:4403168-4432352 |
| 18353 | Rmnd5a | NM_024288.2 | chr6:71388633-71440637 |
| 18354 | Rmnd5b | NM_025346.1 | chr11:51623672-51635896 |
| 18355 | Rmrp | NR_001460.1 | chr4:43492784-43493059 |
| 18356 | Rmst | NR_028262.1 | chr10:92081745-92165178 |
| 18357 | Rn4.5s | NR_002841.1 | chr6:47654920-47752925 |
| 18358 | Rn45s | NR_046233.2 | chr17:39842996-39848829 |
| 18359 | Rnase1 | NM_011271.2 | chr14:51145001-51146767 |
| 18360 | Rnase10 | NM_001162863.1 | chr14:51007750-51010758 |
| 18361 | Rnase11 | NM_001011877.2 | chr14:51049450-51050163 |
| 18362 | Rnase12 | NM_001011875.2 | chr14:51056697-51057242 |
| 18363 | Rnase13 | NM_001011687.2 | chr14:51922159-51922773 |
| 18364 | Rnase2a | NM_053113.2 | chr14:51255261-51256112 |
| 18365 | Rnase2b | NM_019398.2 | chr14:51162259-51163018 |
| 18366 | Rnase4 | NM_021472.4 | chr14:51091076-51106151 |
| 18367 | Rnase6 | NM_030098.2 | chr14:51129067-51131121 |
| 18368 | Rnase9 | NM_183032.2 | chr14:51038458-51041867 |
| 18369 | Rnaseh1 | NM_001286865.1 | chr12:28649601-28659591 |
| 18370 | Rnaseh2a | NM_027187.3 | chr8:84956609-84966011 |
| 18371 | Rnaseh2b | NM_026001.2 | chr14:62332104-62372992 |
| 18372 | Rnaseh2c | NM_026616.2 | chr19:5601872-5602959 |
| 18373 | Rnasek | NM_173742.3 | chr11:70238122-70239852 |
| 18374 | Rnasel | NM_011882.2 | chr1:153749425-153764221 |
| 18375 | Rnaset2a | NM_001083938.2 | chr17:8128597-8147788 |
| 18376 | Rnaset2b | NM_026611.2 | chr17:6978859-8147832 |
| 18377 | Rnd1 | NM_172612.3 | chr15:98669204-98677461 |
| 18378 | Rnd2 | NM_009708.1 | chr11:101468337-101471306 |
| 18379 | Rnd3 | NM_028810.2 | chr2:51130438-51149111 |
| 18380 | Rnf10 | NM_016698.2 | chr5:115241769-115272895 |
| 18381 | Rnf103 | NM_009543.3 | chr6:71493876-71510880 |
| 18382 | Rnf11 | NM_013876.3 | chr4:109452856-109476505 |
| 18383 | Rnf111 | NM_033604.2 | chr9:70425428-70503725 |
| 18384 | Rnf112 | NM_001291024.1 | chr11:61448416-61453992 |
| 18385 | Rnf113a1 | NM_153503.2 | chrX:37191221-37192465 |
| 18386 | Rnf113a2 | NM_025525.2 | chr12:84417199-84418578 |
| 18387 | Rnf114 | NM_030743.5 | chr2:167492644-167516166 |
| 18388 | Rnf115 | NM_026406.3 | chr3:96727610-96791155 |
| 18389 | Rnf121 | NM_029211.2 | chr7:102019871-102065132 |
| 18390 | Rnf122 | NM_175136.2 | chr8:31111845-31131473 |
| 18391 | Rnf123 | NM_032543.2 | chr9:108051671-108079375 |
| 18392 | Rnf125 | NM_026301.2 | chr18:20944624-20983848 |
| 18393 | Rnf126 | NM_144528.3 | chr10:79758514-79766952 |
| 18394 | Rnf128 | NM_001254761.1 | chrX:139563339-139673145 |
| 18395 | Rnf13 | NM_001113413.2 | chr3:57736061-57835425 |
| 18396 | Rnf130 | NM_001290749.1 | chr11:50025330-50104759 |
| 18397 | Rnf133 | NM_198251.2 | chr6:23648868-23650305 |
| 18398 | Rnf135 | NM_028019.3 | chr11:80183871-80199753 |
| 18399 | Rnf138 | NM_019706.3 | chr18:21001299-21028224 |
| 18400 | Rnf138rt1 | NM_028842.3 | chrX:163760138-163761332 |
| 18401 | Rnf139 | NM_175226.4 | chr15:58889228-58902390 |
| 18402 | Rnf14 | NM_001164621.1 | chr18:38296804-38317849 |
| 18403 | Rnf141 | NM_025999.3 | chr7:110816534-110844381 |
| 18404 | Rnf144a | NM_001081977.2 | chr12:26306793-26415262 |
| 18405 | Rnf144b | NM_001170643.1 | chr13:47194002-47247991 |
| 18406 | Rnf145 | NM_001166553.1 | chr11:44519376-44554977 |
| 18407 | Rnf146 | NM_001110196.1 | chr10:29344175-29362442 |
| 18408 | Rnf148 | NM_027754.1 | chr6:23653894-23655136 |
| 18409 | Rnf149 | NM_001033135.3 | chr1:39551295-39577347 |
| 18410 | Rnf150 | NM_177378.4 | chr8:82863355-83091271 |
| 18411 | Rnf151 | NM_026205.3 | chr17:24715839-24718057 |
| 18412 | Rnf152 | NM_001160368.1 | chr1:105276916-105356710 |
| 18413 | Rnf157 | NM_027258.1 | chr11:116336344-116413032 |
| 18414 | Rnf165 | NM_001164504.1 | chr18:77456109-77565136 |
| 18415 | Rnf166 | NM_001033142.2 | chr8:122466146-122476064 |
| 18416 | Rnf167 | NM_027445.2 | chr11:70647588-70651414 |
| 18417 | Rnf168 | NM_027355.2 | chr16:32277460-32301439 |
| 18418 | Rnf169 | NM_175388.3 | chr7:99920253-99980458 |
| 18419 | Rnf17 | NM_001033043.1 | chr14:56402696-56525031 |
| 18420 | Rnf170 | NM_029965.2 | chr8:26119379-26143869 |
| 18421 | Rnf180 | NM_027934.2 | chr13:105147493-105293014 |
| 18422 | Rnf181 | NM_025607.3 | chr6:72359713-72362381 |
| 18423 | Rnf182 | NM_183204.4 | chr13:43615796-43670944 |
| 18424 | Rnf183 | NM_153504.3 | chr4:62427541-62434726 |
| 18425 | Rnf185 | NM_001290472.1 | chr11:3415972-3452956 |
| 18426 | Rnf186 | NM_025786.3 | chr4:138967111-138968366 |
| 18427 | Rnf187 | NM_022423.2 | chr11:58932287-58938906 |
| 18428 | Rnf19a | NM_013923.2 | chr15:36239933-36283147 |
| 18429 | Rnf19b | NM_029219.1 | chr4:129058270-129084526 |

Fig.21 - 96

| | | | |
|---|---|---|---|
| 18430 | Rnf2 | NM_011277.2 | chr1:151469405-151500823 |
| 18431 | Rnf20 | NM_001163263.1 | chr4:49632059-49656886 |
| 18432 | Rnf207 | NM_001033489.2 | chr4:152307022-152318625 |
| 18433 | Rnf208 | NM_176834.2 | chr2:25242928-25244261 |
| 18434 | Rnf214 | NM_178709.4 | chr9:45863690-45906877 |
| 18435 | Rnf215 | NM_027859.2 | chr11:4135159-4141192 |
| 18436 | Rnf216 | NM_080561.4 | chr5:142990892-143113020 |
| 18437 | Rnf217 | NM_001146349.1 | chr10:31501886-31609725 |
| 18438 | Rnf219 | NM_026047.4 | chr14:104477533-104522666 |
| 18439 | Rnf220 | NM_025739.3 | chr4:117271462-117497052 |
| 18440 | Rnf222 | NM_177060.3 | chr11:68888552-68895015 |
| 18441 | Rnf223 | NM_001220499.1 | chr4:156132169-156133419 |
| 18442 | Rnf224 | NM_001033410.2 | chr2:25234475-25236787 |
| 18443 | Rnf24 | NM_178607.4 | chr2:131298487-131352892 |
| 18444 | Rnf25 | NM_021313.1 | chr1:74593747-74601397 |
| 18445 | Rnf26 | NM_153762.3 | chr9:44110780-44113051 |
| 18446 | Rnf31 | NM_194346.2 | chr14:55591789-55603671 |
| 18447 | Rnf32 | NM_001289757.1 | chr5:29195992-29225524 |
| 18448 | Rnf34 | NM_030564.1 | chr5:122850187-122868945 |
| 18449 | Rnf38 | NM_001038993.3 | chr4:44126211-44168283 |
| 18450 | Rnf39 | NM_001099632.1 | chr17:36943050-36947986 |
| 18451 | Rnf4 | NM_011278.5 | chr5:34336249-34353445 |
| 18452 | Rnf40 | NM_172281.2 | chr7:127588697-127603605 |
| 18453 | Rnf41 | NM_001164237.1 | chr10:128411615-128441441 |
| 18454 | Rnf43 | NM_172448.3 | chr11:87663086-87735539 |
| 18455 | Rnf44 | NM_001146025.1 | chr13:54679398-54687808 |
| 18456 | Rnf5 | NM_019403.3 | chr17:34601098-34603561 |
| 18457 | Rnf6 | NM_001256085.1 | chr5:146209193-146220971 |
| 18458 | Rnf7 | NM_011279.3 | chr9:96470936-96478675 |
| 18459 | Rnf8 | NM_021419.2 | chr17:29614788-29641664 |
| 18460 | Rnft1 | NM_029788.5 | chr11:86484656-86499007 |
| 18461 | Rnft2 | NM_001109902.1 | chr5:118190735-118245025 |
| 18462 | Rngtt | NM_011884.3 | chr4:33310310-33502614 |
| 18463 | Rnh1 | NM_001172100.1 | chr7:141160325-141172851 |
| 18464 | Rnls | NM_001146342.2 | chr19:33137744-33392295 |
| 18465 | Rnmt | NM_001170953.1 | chr18:68300354-68324852 |
| 18466 | Rnmtl1 | NM_183263.5 | chr11:76243735-76250622 |
| 18467 | Rnpc3 | NM_001038696.1 | chr3:113605066-113630149 |
| 18468 | Rnpep | NM_001159624.1 | chr1:135262698-135284084 |
| 18469 | Rnpepl1 | NM_181405.4 | chr1:92910824-92920585 |
| 18470 | Rnps1 | NM_001080127.1 | chr17:24414674-24425897 |
| 18471 | Rnu11 | NR_002865.2 | chr4:132270078-132270186 |
| 18472 | Rnu12 | NR_004432.2 | chr15:83149644-83149794 |
| 18473 | Rnu6 | NR_003027.2 | chr19:14438479-14438506 |
| 18474 | Rnu7 | NR_024201.3 | chr5:53698563-53698588 |
| 18475 | Rnu73b | NR_004418.1 | chr3:86140616-86140687 |
| 18476 | Robo1 | NM_019413.2 | chr16:72663148-73046100 |
| 18477 | Robo2 | NM_175549.4 | chr16:73892305-74410912 |
| 18478 | Robo3 | NM_001164767.1 | chr9:37416044-37433175 |
| 18479 | Robo4 | NM_028783.3 | chr9:37401896-37414023 |
| 18480 | Rock1 | NM_009071.2 | chr18:10064400-10181792 |
| 18481 | Rock2 | NM_009072.2 | chr12:16894977-16988274 |
| 18482 | Rogdi | NM_133185.2 | chr16:5008728-5013553 |
| 18483 | Rom1 | NM_009073.4 | chr19:8927381-8929356 |
| 18484 | Romo1 | NM_001164216.1 | chr2:156144152-156145794 |
| 18485 | Ropn1 | NM_030744.2 | chr16:34651210-34678610 |
| 18486 | Ropn1l | NM_145852.2 | chr15:31441209-31453689 |
| 18487 | Ror1 | NM_013845.5 | chr4:100095790-100444806 |
| 18488 | Ror2 | NM_013846.3 | chr13:53109316-53286109 |
| 18489 | Rora | NM_001289916.1 | chr9:69289840-69388246 |
| 18490 | Rorb | NM_001043354.2 | chr19:18930608-19111196 |
| 18491 | Rorc | NM_011281.3 | chr3:94372793-94398274 |
| 18492 | Ros1 | NM_011282.2 | chr10:52045925-52195244 |
| 18493 | Rp1 | NM_001195662.1 | chr1:4290845-4409241 |
| 18494 | Rp1l1 | NM_146246.3 | chr14:63992430-64033506 |
| 18495 | Rp2h | NM_001290643.1 | chrX:20364480-20400858 |
| 18496 | Rp9 | NM_018739.2 | chr9:22448311-22468356 |
| 18497 | Rpa1 | NM_001164223.1 | chr11:75300258-75348383 |
| 18498 | Rpa2 | NM_011284.3 | chr4:132768359-132778746 |
| 18499 | Rpa3 | NM_026632.4 | chr6:8255935-8259141 |
| 18500 | Rpain | NM_001252413.1 | chr11:70970199-70977933 |
| 18501 | Rpap1 | NM_001163701.1 | chr2:119763958-119787537 |
| 18502 | Rpap2 | NM_001163461.2 | chr5:107597695-107642918 |
| 18503 | Rpap3 | NM_028003.2 | chr15:97675104-97705822 |
| 18504 | Rpe | NM_025683.3 | chr1:66700830-66719805 |
| 18505 | Rpe65 | NM_029987.2 | chr3:159599180-159625307 |
| 18506 | Rpf1 | NM_027332.3 | chr3:146510794-146521423 |
| 18507 | Rpf2 | NM_001042556.1 | chr10:40223245-40246979 |
| 18508 | Rpgr | NM_001177950.1 | chrX:10158215-10216795 |
| 18509 | Rpgrip1 | NM_001168515.1 | chr14:52110902-52161339 |
| 18510 | Rpgrip1l | NM_173431.2 | chr8:91217029-91313222 |
| 18511 | Rph3a | NM_011286.3 | chr5:120940499-121009538 |
| 18512 | Rph3al | NM_001291159.1 | chr11:75899724-75925891 |
| 18513 | Rpia | NM_009075.2 | chr6:70765719-70792175 |
| 18514 | Rpl10 | NM_052835.3 | chrX:74270815-74273135 |
| 18515 | Rpl10a | NM_011287.2 | chr17:28328470-28331033 |
| 18516 | Rpl10l | NM_001162933.1 | chr12:66283378-66284401 |
| 18517 | Rpl11 | NM_025919.2 | chr4:136049947-136053371 |
| 18518 | Rpl12 | NM_009076.3 | chr2:32961711-32964045 |
| 18519 | Rpl13 | NM_016738.5 | chr8:123102349-123105242 |
| 18520 | Rpl13a | NM_009438.5 | chr7:45125562-45128745 |
| 18521 | Rpl14 | NM_025974.2 | chr9:120571516-120574653 |
| 18522 | Rpl14-ps1 | NR_110499.1 | chr7:45324957-45325651 |
| 18523 | Rpl15 | NM_025586.3 | chr14:18267822-18270986 |
| 18524 | Rpl17 | NM_001002239.3 | chr18:75000476-75003381 |
| 18525 | Rpl18 | NM_009077.2 | chr7:45718070-45720835 |
| 18526 | Rpl18a | NM_029751.4 | chr8:70894721-70897443 |
| 18527 | Rpl19 | NM_001159483.1 | chr11:98026709-98030493 |
| 18528 | Rpl21 | NM_019647.6 | chr5:146832889-146837032 |
| 18529 | Rpl22 | NM_001277113.1 | chr4:152324435-152334082 |
| 18530 | Rpl22l1 | NM_026517.2 | chr3:28805510-28807415 |
| 18531 | Rpl23 | NM_022891.3 | chr11:97777525-97782439 |
| 18532 | Rpl23a | NM_207523.2 | chr11:78180935-78183584 |
| 18533 | Rpl24 | NM_024218.4 | chr16:55966274-55971437 |
| 18534 | Rpl26 | NM_009080.2 | chr11:68901565-68904534 |
| 18535 | Rpl27 | NM_011289.3 | chr11:101442244-101445596 |
| 18536 | Rpl27a | NM_011975.3 | chr7:109519194-109522369 |
| 18537 | Rpl28 | NM_009081.2 | chr7:4792964-4794547 |
| 18538 | Rpl29 | NM_009082.2 | chr9:106429538-106431567 |
| 18539 | Rpl3 | NM_013762.2 | chr15:80077780-80083406 |
| 18540 | Rpl30 | NM_001163485.1 | chr15:34440507-34443276 |
| 18541 | Rpl31 | NM_001252218.1 | chr1:39367850-39371910 |
| 18542 | Rpl31-ps12 | NM_001258458.1 | chr16:16819712-16820196 |
| 18543 | Rpl32 | NM_172086.2 | chr6:115805513-115808743 |
| 18544 | Rpl34 | NM_001005859.3 | chr3:130726826-130730398 |
| 18545 | Rpl34-ps1 | NM_001199350.1 | chr3:130726836-130730329 |
| 18546 | Rpl35 | NM_025592.3 | chr2:39001580-39005131 |
| 18547 | Rpl35a | NM_001130484.1 | chr16:33056481-33060188 |
| 18548 | Rpl36 | NM_018730.3 | chr17:56613394-56614246 |
| 18549 | Rpl36a | NM_019865.5 | chrX:134585653-134588062 |
| 18550 | Rpl36al | NM_025589.4 | chr12:69182733-69184067 |
| 18551 | Rpl37 | NM_026069.3 | chr15:5116612-5119140 |
| 18552 | Rpl37a | NM_009084.4 | chr1:72711259-72713813 |
| 18553 | Rpl38 | NM_001048057.1 | chr11:114668542-114672331 |
| 18554 | Rpl39 | NM_026055.2 | chrX:37082517-37085222 |
| 18555 | Rpl39l | NM_026594.2 | chr16:10170227-10174911 |
| 18556 | Rpl3l | NM_001163945.1 | chr17:24727828-24736149 |
| 18557 | Rpl4 | NM_024212.4 | chr9:64173386-64178562 |
| 18558 | Rpl41 | NM_018860.4 | chr10:128548109-128549168 |
| 18559 | Rpl5 | NM_016980.2 | chr5:107900527-107909005 |
| 18560 | Rpl6 | NM_011290.5 | chr5:121204500-121209241 |
| 18561 | Rpl7 | NM_011291.5 | chr1:16101295-16104433 |
| 18562 | Rpl7a | NM_013721.3 | chr2:26910806-26913311 |
| 18563 | Rpl7l1 | NM_025433.3 | chr17:46773906-46782656 |
| 18564 | Rpl8 | NM_012053.2 | chr15:76904070-76906318 |
| 18565 | Rpl9 | NM_011292.2 | chr5:65388363-65391431 |
| 18566 | Rplp0 | NM_007475.5 | chr5:115559466-115563729 |
| 18567 | Rplp1 | NM_018853.3 | chr9:61913282-61914510 |
| 18568 | Rplp2 | NM_026020.6 | chr7:141447649-141451342 |
| 18569 | Rplp2-ps1 | NR_038063.1 | chr12:75630924-75631749 |
| 18570 | Rpn1 | NM_133933.4 | chr6:88084472-88105304 |
| 18571 | Rpn2 | NM_019642.4 | chr2:157279097-157326318 |
| 18572 | Rpp14 | NM_025938.4 | chr14:8080312-8091846 |
| 18573 | Rpp21 | NM_026308.2 | chr17:36255672-36257846 |
| 18574 | Rpp25 | NM_133982.1 | chr9:57504101-57505447 |
| 18575 | Rpp25l | NM_027278.3 | chr4:41712032-41713517 |
| 18576 | Rpp30 | NM_019428.3 | chr19:36083715-36104773 |
| 18577 | Rpp38 | NM_001013376.2 | chr2:3328948-3332628 |
| 18578 | Rpp40 | NM_145938.4 | chr13:35895103-35906347 |
| 18579 | Rpph1 | NR_002142.2 | chr14:50807446-50807771 |
| 18580 | Rprd1a | NM_144861.2 | chr18:24484961-24530204 |
| 18581 | Rprd1b | NM_001291134.1 | chr2:158028496-158078207 |
| 18582 | Rprd2 | NM_001081293.1 | chr3:95759872-95818953 |
| 18583 | Rprl1 | NR_004434.3 | chr6:69326936-69327174 |
| 18584 | Rprl2 | NR_004439.2 | chr3:22251369-22251607 |
| 18585 | Rprl3 | NR_024198.2 | chr8:3803124-3803361 |
| 18586 | Rprm | NM_023396.4 | chr2:54084092-54085552 |
| 18587 | Rprml | NM_001033212.2 | chr11:103649508-103650580 |
| 18588 | Rps10 | NM_025963.3 | chr17:27630428-27635242 |
| 18589 | Rps11 | NM_013725.4 | chr7:45122387-45124389 |
| 18590 | Rps12 | NM_011295.6 | chr10:23785182-23787209 |
| 18591 | Rps13 | NM_026533.3 | chr7:116331506-116334190 |
| 18592 | Rps14 | NM_020600.4 | chr18:60774595-60778546 |
| 18593 | Rps15 | NM_009091.2 | chr10:80292430-80294114 |
| 18594 | Rps15a | NM_170669.2 | chr7:118104375-118116147 |
| 18595 | Rps15a-ps4 | NR_036572.1 | chr4:132219892-132220589 |
| 18596 | Rps15a-ps6 | NR_029471.2 | chr11:6172507-6173331 |
| 18597 | Rps16 | NM_013647.2 | chr7:28350688-28352698 |
| 18598 | Rps17 | NM_009092.3 | chr7:81342732-81345234 |
| 18599 | Rps18 | NM_011296.2 | chr17:33951998-33955641 |
| 18600 | Rps19 | NM_023133.1 | chr7:24884713-24889802 |
| 18601 | Rps19bp1 | NM_175109.3 | chr15:80260613-80264306 |
| 18602 | Rps19-ps3 | NR_033639.1 | chr4:147821776-147822202 |
| 18603 | Rps2 | NM_008503.5 | chr17:24720062-24721927 |
| 18604 | Rps20 | NM_026147.5 | chr4:3834472-3835600 |
| 18605 | Rps21 | NM_025587.2 | chr2:180257378-180258444 |
| 18606 | Rps23 | NM_024175.3 | chr13:90923121-90924732 |
| 18607 | Rps24 | NM_011297.2 | chr14:24490680-24496146 |
| 18608 | Rps25 | NM_024266.3 | chr9:44407713-44410406 |
| 18609 | Rps26 | NM_013765.2 | chr10:128624528-128626506 |
| 18610 | Rps27 | NM_027015.4 | chr3:90212666-90213648 |
| 18611 | Rps27a | NM_001033865.1 | chr11:29545841-29548040 |
| 18612 | Rps27l | NM_026467.4 | chr9:66946075-66949522 |
| 18613 | Rps27rt | NM_001190258.1 | chr3:90212666-90213647 |
| 18614 | Rps28 | NM_016844.2 | chr17:33823036-33824498 |
| 18615 | Rps29 | NM_009093.2 | chr12:69157721-69159186 |
| 18616 | Rps3 | NM_012052.2 | chr7:99477896-99483709 |
| 18617 | Rps3a1 | NM_016959.4 | chr3:86137939-86142668 |
| 18618 | Rps4l | NR_003634.2 | chr6:148354655-148355596 |
| 18619 | Rps4x | NM_009094.2 | chrX:102184940-102189391 |
| 18620 | Rps5 | NM_009095.2 | chr7:12922310-12926686 |
| 18621 | Rps6 | NM_009096.3 | chr4:86854098-86857367 |
| 18622 | Rps6ka1 | NM_001285505.1 | chr4:133847290-133887797 |
| 18623 | Rps6ka2 | NM_011299.4 | chr17:7170114-7303316 |

Fig.21 - 97

| | | | | | | | |
|---|---|---|---|---|---|---|---|
| 18624 | Rps6ka3 | NM_148945.2 | chrX:159255781-159368244 | 18721 | Rufy3 | NM_001289774.1 | chr5:88565039-88651417 |
| 18625 | Rps6ka4 | NM_019924.2 | chr19:6829083-6840627 | 18722 | Rufy4 | NM_001034060.3 | chr1:74125540-74148223 |
| 18626 | Rps6ka5 | NM_153587.2 | chr12:100549777-100725028 | 18723 | Rundc1 | NM_172566.4 | chr11:101425084-101435665 |
| 18627 | Rps6ka6 | NM_025949.3 | chrX:111387836-111537959 | 18724 | Rundc3a | NM_001252347.1 | chr11:102393402-102402939 |
| 18628 | Rps6kb1 | NM_001114334.1 | chr11:86499010-86544807 | 18725 | Rundc3b | NM_198620.1 | chr5:8490335-8622952 |
| 18629 | Rps6kb2 | NM_021485.2 | chr19:4156976-4163245 | 18726 | Runx1 | NM_001111021.2 | chr16:92601465-92826074 |
| 18630 | Rps6kc1 | NM_178775.4 | chr1:190772878-190911770 | 18727 | Runx1t1 | NM_001111026.2 | chr4:13743301-13895055 |
| 18631 | Rps6kl1 | NM_146244.4 | chr12:85135595-85151264 | 18728 | Runx2 | NM_001145920.2 | chr17:44603988-44736648 |
| 18632 | Rps7 | NM_011300.3 | chr12:28630846-28635953 | 18729 | Runx3 | NM_019732.2 | chr4:135120644-135177990 |
| 18633 | Rps8 | NM_009098.2 | chr4:117153835-117156132 | 18730 | Rusc1 | NM_001083807.1 | chr3:89083978-89093363 |
| 18634 | Rps9 | NM_029767.2 | chr7:3704040-3706897 | 18731 | Rusc2 | NM_001037709.2 | chr4:43381981-43427092 |
| 18635 | Rpsa | NM_011029.4 | chr9:120127765-120132369 | 18732 | Ruvbl1 | NM_019685.2 | chr6:88465422-88497566 |
| 18636 | Rptn | NM_009100.2 | chr3:93393698-93399442 | 18733 | Ruvbl2 | NM_011304.3 | chr7:45421897-45434464 |
| 18637 | Rptor | NM_028898.3 | chr11:119602904-119899591 | 18734 | Rwdd1 | NM_025614.3 | chr10:33996554-34019616 |
| 18638 | Rptoros | NR_045312.1 | chr11:119820810-119834365 | 18735 | Rwdd2a | NM_001145968.1 | chr9:86572052-86574899 |
| 18639 | Rpusd1 | NM_028009.3 | chr17:25727750-25731456 | 18736 | Rwdd2b | NM_016924.2 | chr16:87433330-87440592 |
| 18640 | Rpusd2 | NM_173450.3 | chr2:119034789-119042197 | 18737 | Rwdd3 | NM_025637.3 | chr3:121155401-121171695 |
| 18641 | Rpusd3 | NM_001033204.1 | chr6:113415318-113419340 | 18738 | Rwdd4a | NM_203507.3 | chr8:47533644-47552837 |
| 18642 | Rpusd4 | NM_028040.2 | chr9:35267880-35275957 | 18739 | Rxfp1 | NM_212452.1 | chr3:79644715-79737794 |
| 18643 | Rqcd1 | NM_021383.5 | chr1:74506059-74530842 | 18740 | Rxfp2 | NM_001289564.1 | chr5:150018674-150082186 |
| 18644 | Rrad | NM_019662.2 | chr8:104628065-104631321 | 18741 | Rxfp3 | NM_178717.3 | chr15:11033716-11037968 |
| 18645 | Rraga | NM_178376.3 | chr4:86575672-86577283 | 18742 | Rxfp4 | NM_181817.1 | chr3:88651897-88653142 |
| 18646 | Rragb | NM_001004154.2 | chrX:153139957-153171943 | 18743 | Rxra | NM_001290481.1 | chr2:27709291-27763319 |
| 18647 | Rragc | NM_017475.2 | chr4:123917432-123936997 | 18744 | Rxrb | NM_001205214.1 | chr17:34031811-34038403 |
| 18648 | Rragd | NM_027491.2 | chr4:32982997-33022180 | 18745 | Rxrg | NM_001159731.1 | chr1:167618264-167639623 |
| 18649 | Rras | NM_009101.2 | chr7:45018006-45021644 | 18746 | Rybp | NM_019743.3 | chr6:100228564-100287358 |
| 18650 | Rras2 | NM_025846.2 | chr7:114046781-114117781 | 18747 | Ryk | NM_001042607.1 | chr9:102834919-102908307 |
| 18651 | Rrbp1 | NM_024281.2 | chr2:143947394-144011263 | 18748 | Ryr1 | NM_009109.2 | chr7:29003339-29125151 |
| 18652 | Rreb1 | NM_001039188.1 | chr13:37827392-37935534 | 18749 | Ryr2 | NM_023868.2 | chr13:11553102-12106945 |
| 18653 | Rrh | NM_009102.3 | chr3:129808574-129822505 | 18750 | Ryr3 | NM_177652.2 | chr2:112631381-113030331 |
| 18654 | Rrm1 | NM_009103.3 | chr7:102441694-102469771 | 18751 | S100a1 | NM_011309.3 | chr3:90511033-90514330 |
| 18655 | Rrm2 | NM_009104.2 | chr12:24708253-24714146 | 18752 | S100a10 | NM_009112.2 | chr3:93555116-93564645 |
| 18656 | Rrm2b | NM_199476.1 | chr15:37923952-37961055 | 18753 | S100a11 | NM_016740.3 | chr3:93520495-93526288 |
| 18657 | Rrn3 | NM_001039521.1 | chr16:13780698-13814841 | 18754 | S100a13 | NM_009113.4 | chr3:90514434-90524581 |
| 18658 | Rrnad1 | NM_153562.4 | chr3:87922600-87930195 | 18755 | S100a14 | NM_001163525.2 | chr3:90526848-90528835 |
| 18659 | Rrp1 | NM_010925.2 | chr10:78400361-78413043 | 18756 | S100a16 | NM_026416.2 | chr3:90541222-90543151 |
| 18660 | Rrp12 | NM_199447.2 | chr19:41862850-41896153 | 18757 | S100a2 | NM_001195760.1 | chr3:90590246-90591508 |
| 18661 | Rrp15 | NM_026041.2 | chr1:186721086-186749358 | 18758 | S100a3 | NM_011310.2 | chr3:90600214-90602702 |
| 18662 | Rrp1b | NM_001163734.1 | chr17:32048284-32062862 | 18759 | S100a4 | NM_011311.2 | chr3:90603769-90606045 |
| 18663 | Rrp36 | NM_144857.1 | chr17:46667452-46674255 | 18760 | S100a5 | NM_011312.2 | chr3:90608521-90611780 |
| 18664 | Rrp7a | NM_029101.4 | chr15:83115845-83122801 | 18761 | S100a6 | NM_011313.2 | chr3:90612893-90614414 |
| 18665 | Rrp8 | NM_025897.2 | chr7:105731729-105736584 | 18762 | S100a7a | NM_199422.1 | chr3:90654301-90658130 |
| 18666 | Rrp9 | NM_145620.4 | chr9:106477308-106485415 | 18763 | S100a8 | NM_013650.2 | chr3:90669070-90670034 |
| 18667 | Rrs1 | NM_021511.2 | chr1:9545407-9547455 | 18764 | S100a9 | NM_001281852.1 | chr3:90692629-90695721 |
| 18668 | Rs1 | NM_011302.3 | chrX:160768012-160799663 | 18765 | S100b | NM_009115.3 | chr10:76253835-76261319 |
| 18669 | Rsad1 | NM_001013381.2 | chr11:94539797-94549207 | 18766 | S100g | NM_009789.1 | chrX:162961991-162964599 |
| 18670 | Rsad2 | NM_021384.4 | chr12:26442742-26456452 | 18767 | S100pbp | NM_029036.2 | chr4:129150824-129189482 |
| 18671 | Rsbn1 | NM_172684.2 | chr3:103914119-103966620 | 18768 | S100z | NM_001081159.1 | chr13:95477300-95478655 |
| 18672 | Rsbn1l | NM_001080977.1 | chr5:20893023-20951822 | 18769 | S1pr1 | NM_007901.5 | chr3:115710432-115715055 |
| 18673 | Rsc1a1 | NM_023544.5 | chr4:141683562-141685716 | 18770 | S1pr2 | NM_010333.4 | chr9:20965951-20976793 |
| 18674 | Rsf1 | NM_001081267.2 | chr7:97579895-97692782 | 18771 | S1pr3 | NM_010101.4 | chr13:51408617-51422797 |
| 18675 | Rsg1 | NM_001081174.2 | chr4:141213955-141220114 | 18772 | S1pr4 | NM_010102.2 | chr10:81497744-81500137 |
| 18676 | Rsl1 | NM_001013769.1 | chr13:67173181-67183498 | 18773 | S1pr5 | NM_053190.2 | chr9:21242916-21248443 |
| 18677 | Rsl1d1 | NM_025546.2 | chr16:11193036-11203292 | 18774 | Saa1 | NM_009117.3 | chr7:46740498-46742980 |
| 18678 | Rsl24d1 | NM_198609.2 | chr9:73113468-73123333 | 18775 | Saa2 | NM_011314.2 | chr7:46751832-46754314 |
| 18679 | Rslcan18 | NM_001256052.1 | chr13:67096612-67114028 | 18776 | Saa3 | NM_011315.3 | chr7:46711997-46715676 |
| 18680 | Rsph1 | NM_025290.3 | chr17:31255019-31277356 | 18777 | Saa4 | NM_011316.3 | chr7:46727998-46732543 |
| 18681 | Rsph3a | NM_025789.5 | chr17:7945613-7979556 | 18778 | Saal1 | NM_030233.1 | chr7:46686107-46710651 |
| 18682 | Rsph3b | NM_001083945.1 | chr17:6904715-6948356 | 18779 | Sac3d1 | NM_133678.3 | chr19:6116003-6118586 |
| 18683 | Rsph4a | NM_001162957.1 | chr10:33905110-33916021 | 18780 | Sacm1l | NM_030692.4 | chr9:123529881-123592598 |
| 18684 | Rsph6a | NM_001159671.1 | chr7:19054686-19074447 | 18781 | Sacs | NM_172809.3 | chr14:61138456-61240693 |
| 18685 | Rsph9 | NM_029338.3 | chr17:46129276-46144198 | 18782 | Sae1 | NM_001285891.1 | chr7:16320235-16387896 |
| 18686 | Rspo1 | NM_138683.2 | chr4:124986429-125009099 | 18783 | Safb | NM_001163300.1 | chr17:56584981-56606294 |
| 18687 | Rspo2 | NM_172815.3 | chr15:43020794-43170818 | 18784 | Safb2 | NM_001029979.2 | chr17:56562941-56584583 |
| 18688 | Rspo3 | NM_028351.3 | chr10:29453106-29535867 | 18785 | Sag | NM_009118.2 | chr1:87803679-87845157 |
| 18689 | Rspo4 | NM_001040689.1 | chr2:151842926-151874668 | 18786 | Sall1 | NM_021390.3 | chr8:89027242-89044162 |
| 18690 | Rspry1 | NM_026274.4 | chr8:94601940-94660276 | 18787 | Sall2 | NM_001244916.1 | chr14:52311176-52316323 |
| 18691 | Rsrc1 | NM_025822.3 | chr3:66985671-67358403 | 18788 | Sall3 | NM_178280.3 | chr18:80966373-80986578 |
| 18692 | Rsrc2 | NM_001005525.2 | chr5:123728425-123749414 | 18789 | Sall4 | NM_175303.4 | chr2:168748331-168767201 |
| 18693 | Rsrp1 | NM_023665.3 | chr4:134923624-134927370 | 18790 | Samd1 | NM_001081415.1 | chr8:83997671-84000386 |
| 18694 | Rsu1 | NM_009105.4 | chr2:13076966-13271415 | 18791 | Samd10 | NM_172676.2 | chr2:181595217-181599147 |
| 18695 | Rtbdn | NM_144929.2 | chr8:84946990-84956603 | 18792 | Samd11 | NM_001110516.1 | chr4:156246965-156255338 |
| 18696 | Rtca | NM_025517.3 | chr3:116488963-116508175 | 18793 | Samd12 | NM_177225.4 | chr15:53461800-53902436 |
| 18697 | Rtcb | NM_145422.4 | chr10:85938636-85957793 | 18794 | Samd14 | NM_146025.2 | chr11:95009878-95026087 |
| 18698 | Rtdr1 | NM_001163533.1 | chr10:74957476-75032586 | 18795 | Samd15 | NM_001290288.1 | chr12:87200542-87213538 |
| 18699 | Rtel1 | NM_001001882.3 | chr2:181319723-181356616 | 18796 | Samd3 | NM_001013766.2 | chr10:26229706-26260804 |
| 18700 | Rtf1 | NM_030112.2 | chr2:119675067-119735407 | 18797 | Samd4 | NM_001037221.2 | chr14:46882964-47105817 |
| 18701 | Rtfdc1 | NM_025542.2 | chr2:172440577-172469899 | 18798 | Samd4b | NM_175021.3 | chr7:28399521-28436191 |
| 18702 | Rtkn | NM_001136227.1 | chr6:83135807-83152579 | 18799 | Samd5 | NM_177271.3 | chr10:9627258-9675208 |
| 18703 | Rtkn2 | NM_001081346.1 | chr10:67979597-68043864 | 18800 | Samd7 | NM_029489.3 | chr3:30746292-30767174 |
| 18704 | Rtl1 | NM_184109.1 | chr12:109590168-109595403 | 18801 | Samd8 | NM_026283.2 | chr14:21750530-21798725 |
| 18705 | Rtn1 | NM_001007596.2 | chr12:72211748-72236727 | 18802 | Samd9l | NM_010156.3 | chr6:3372257-3399571 |
| 18706 | Rtn2 | NM_001025364.3 | chr7:19291068-19296164 | 18803 | Samhd1 | NM_001139520.1 | chr2:157097528-157135222 |
| 18707 | Rtn3 | NM_001003933.2 | chr19:7425894-7483291 | 18804 | Samm50 | NM_178614.4 | chr15:84192232-84214303 |
| 18708 | Rtn4 | NM_024226.4 | chr11:29718562-29744414 | 18805 | Samsn1 | NM_023380.2 | chr16:75858793-75909266 |
| 18709 | Rtn4ip1 | NM_130892.4 | chr10:43901806-43947862 | 18806 | Samt2 | NM_001037167.1 | chrX:154575227-154579330 |
| 18710 | Rtn4r | NM_022982.2 | chr16:18127705-18152408 | 18807 | Samt3 | NM_028554.3 | chrX:86044198-86047313 |
| 18711 | Rtn4rl1 | NM_177708.5 | chr11:75193992-75267762 | 18808 | Samt4 | NM_029199.2 | chrX:154482001-154484682 |
| 18712 | Rtn4rl2 | NM_199223.1 | chr2:84871945-84886692 | 18809 | Sap130 | NM_172965.2 | chr18:31634382-31723061 |
| 18713 | Rtp1 | NM_001004151.2 | chr16:23429132-23433960 | 18810 | Sap18 | NM_009119.3 | chr14:57798188-57804980 |
| 18714 | Rtp2 | NM_001008230.3 | chr16:23925547-23930794 | 18811 | Sap25 | NM_001081962.2 | chr5:137641472-137642902 |
| 18715 | Rtp3 | NM_153100.2 | chr9:110984942-110989713 | 18812 | Sap30 | NM_021788.2 | chr8:57482701-57487860 |
| 18716 | Rtp4 | NM_023386.5 | chr16:23609918-23614222 | 18813 | Sap30bp | NM_020483 | chr11:115933658-115965534 |
| 18717 | Rttn | NM_175542.3 | chr18:88971789-89131014 | 18814 | Sap30l | NM_001081168 | chr11:57801636-57810615 |
| 18718 | Rubie | NR_046459.1 | chr14:46568330-46575851 | 18815 | Sapcd1 | NM_023893.4 | chr17:35025972-35028016 |
| 18719 | Rufy1 | NM_172557.2 | chr11:50389302-50431111 | 18816 | Sapcd2 | NM_001081085.2 | chr2:25372034-25378213 |
| 18720 | Rufy2 | NM_027425.3 | chr10:62980222-63018742 | 18817 | Sar1a | NM_009120.2 | chr10:61680320-61693297 |

Fig.21 - 98

| | | | | | | | |
|---|---|---|---|---|---|---|---|
| 18818 | Sar1b | NM_025535.2 | chr11:51763662-51791953 | 18915 | Schip1 | NM_001113420.1 | chr3:68064801-68626482 |
| 18819 | Sardh | NM_138665.2 | chr2:27188392-27247303 | 18916 | Scimp | NM_001045526.2 | chr11:70790931-70812561 |
| 18820 | Sarm1 | NM_001168521.1 | chr11:78472329-78497754 | 18917 | Scin | NM_001146196.1 | chr12:40059770-40134228 |
| 18821 | Sarnp | NM_025364.2 | chr10:128821770-128877638 | 18918 | Sclt1 | NM_001081411.1 | chr3:41626704-41742514 |
| 18822 | Sars | NM_001204979.1 | chr3:108424863-108445259 | 18919 | Scly | NM_016717.3 | chr1:91298337-91321080 |
| 18823 | Sars2 | NM_023637.3 | chr7:28741967-28753879 | 18920 | Scmh1 | NM_001159630.1 | chr4:120405280-120530199 |
| 18824 | Sart1 | NM_016882.3 | chr19:5377522-5388703 | 18921 | Scml2 | NM_001290651.1 | chrX:161162749-161258213 |
| 18825 | Sart3 | NM_016926.1 | chr5:113742445-113771649 | 18922 | Scml4 | NM_172938.3 | chr10:42860511-42960782 |
| 18826 | Sash1 | NM_175155.4 | chr10:8722218-8886070 | 18923 | Scn10a | NM_001205321.1 | chr9:119608455-119719032 |
| 18827 | Sash3 | NM_028773.4 | chrX:48146435-48161566 | 18924 | Scn11a | NM_011887.3 | chr9:119753764-119825456 |
| 18828 | Sass6 | NM_001289568.1 | chr3:116594957-116630986 | 18925 | Scn1a | NM_018733.2 | chr2:66270781-66440837 |
| 18829 | Sat1 | NM_001291865.1 | chrX:155213125-155216449 | 18926 | Scn1b | NM_011322.2 | chr7:31116523-31126945 |
| 18830 | Sat2 | NM_026991.2 | chr11:69622108-69623869 | 18927 | Scn2a1 | NM_001099298.2 | chr2:65670444-65767447 |
| 18831 | Satb1 | NM_001163630.1 | chr17:51736186-51812054 | 18928 | Scn2b | NM_001014761.2 | chr9:45117875-45130070 |
| 18832 | Satb2 | NM_139146.2 | chr1:56793980-56971334 | 18929 | Scn3a | NM_018732.3 | chr2:65457117-65567492 |
| 18833 | Satl1 | NM_028655.1 | chrX:112384304-112406779 | 18930 | Scn3b | NM_001083917.1 | chr9:40269215-40291618 |
| 18834 | Sav1 | NM_022028.2 | chr12:69965012-69987002 | 18931 | Scn4a | NM_133199.2 | chr11:106318592-106349390 |
| 18835 | Saysd1 | NM_026209.1 | chr14:20075635-20083172 | 18932 | Scn4b | NM_001013390.2 | chr9:45139041-45154061 |
| 18836 | Sbds | NM_023248.1 | chr5:130245731-130255462 | 18933 | Scn5a | NM_001253860.1 | chr9:119483407-119562678 |
| 18837 | Sbf1 | NM_001081030.2 | chr15:89288235-89315311 | 18934 | Scn7a | NM_009135.2 | chr2:66673425-66784910 |
| 18838 | Sbf2 | NM_177324.2 | chr7:110308012-110614920 | 18935 | Scn8a | NM_001077499.2 | chr15:100870644-101045938 |
| 18839 | Sbk1 | NM_145587.2 | chr7:126272618-126294999 | 18936 | Scn9a | NM_001290674.1 | chr2:66480080-66634962 |
| 18840 | Sbk2 | NM_001146329.1 | chr7:4957080-4964348 | 18937 | Scnm1 | NM_001163573.1 | chr3:95129718-95134012 |
| 18841 | Sbk3 | NM_001200041.1 | chr7:4965259-4970961 | 18938 | Scnn1a | NM_011324.2 | chr6:125321339-125344942 |
| 18842 | Sbno1 | NM_001081203.1 | chr5:124368701-124425914 | 18939 | Scnn1b | NM_001272023.1 | chr7:121865037-121918728 |
| 18843 | Sbno2 | NM_183426.1 | chr10:80057013-80102702 | 18940 | Scnn1g | NM_011326.3 | chr7:121734506-121768477 |
| 18844 | Sbp | NM_011321.3 | chr17:23941995-23945607 | 18941 | Sco1 | NM_001040026.1 | chr11:67052669-67067440 |
| 18845 | Sbpl | NM_001077421.1 | chr17:23953084-23955273 | 18942 | Sco2 | NM_001111288.1 | chr15:89371636-89373818 |
| 18846 | Sbsn | NM_001083903.1 | chr7:30751470-30756134 | 18943 | Scoc | NM_001039137.3 | chr8:83434491-83458396 |
| 18847 | Sbspon | NM_001033288.3 | chr1:15853861-15892722 | 18944 | Scp2 | NM_011327.4 | chr4:108043829-108118547 |
| 18848 | Sc5d | NM_172769.2 | chr9:42254176-42264300 | 18945 | Scp2d1 | NM_025490.2 | chr2:144823665-144824415 |
| 18849 | Scaf1 | NM_001008422.1 | chr7:45002949-45016249 | 18946 | Scpep1 | NM_029023.3 | chr11:88924019-88955442 |
| 18850 | Scaf11 | NM_028148.2 | chr15:96411697-96460843 | 18947 | Scpep1os | NR_045955.1 | chr11:88905927-88936061 |
| 18851 | Scaf4 | NM_178923.3 | chr16:90229143-90284425 | 18948 | Scrg1 | NM_009136.3 | chr8:57455922-57477585 |
| 18852 | Scaf8 | NM_134123.3 | chr17:3114971-3198859 | 18949 | Scrib | NM_134089.2 | chr15:76047162-76069784 |
| 18853 | Scai | NM_178778.3 | chr2:39066214-39190730 | 18950 | Scrn1 | NM_027268.2 | chr6:54508815-54566382 |
| 18854 | Scamp1 | NM_029153.1 | chr13:94201432-94285281 | 18951 | Scrn2 | NM_146027.2 | chr11:97029951-97033960 |
| 18855 | Scamp2 | NM_022813.3 | chr9:57560943-57588798 | 18952 | Scrn3 | NM_029022 | chr2:73312651-73337807 |
| 18856 | Scamp3 | NM_011886.3 | chr3:89177390-89182770 | 18953 | Scrt1 | NM_130893 | chr15:76516202-76522129 |
| 18857 | Scamp4 | NM_019575.4 | chr10:80602881-80615783 | 18954 | Scrt2 | NM_001160410.1 | chr2:152081528-152095802 |
| 18858 | Scamp5 | NM_020270.3 | chr9:57441326-57468060 | 18955 | Sct | NM_001287171.1 | chr7:141278328-141279133 |
| 18859 | Scand1 | NM_020255.3 | chr2:156311845-156312704 | 18956 | Sctr | NM_001012322.2 | chr1:120006979-120063532 |
| 18860 | Scap | NM_001001144.3 | chr9:110333355-110384949 | 18957 | Scube1 | NM_001271472.1 | chr15:83602582-83725039 |
| 18861 | Scaper | NM_001081341.1 | chr9:55549882-55938115 | 18958 | Scube2 | NM_020052.2 | chr7:109798690-109865679 |
| 18862 | Scara3 | NM_172604.3 | chr14:65919394-65953744 | 18959 | Scube3 | NM_001004366.1 | chr17:28142525-28171345 |
| 18863 | Scara5 | NM_001168318.1 | chr14:65666402-65744943 | 18960 | Scx | NM_198885.3 | chr15:76457437-76459468 |
| 18864 | Scarb1 | NM_001205082.1 | chr5:125277086-125341094 | 18961 | Scyl1 | NM_023912.2 | chr19:5758426-5771401 |
| 18865 | Scarb2 | NM_007644.4 | chr5:92441310-92505657 | 18962 | Scyl2 | NM_198021.2 | chr10:89640106-89686285 |
| 18866 | Scarf1 | NM_001004157.2 | chr11:75513540-75526580 | 18963 | Scyl3 | NM_001286002.1 | chr1:163929099-163955126 |
| 18867 | Scarf2 | NM_153790.2 | chr16:17797281-17808287 | 18964 | Sdad1 | NM_172713.2 | chr5:92284009-92310024 |
| 18868 | Scarletltr | NR_040743.1 | chr9:64080760-64088045 | 18965 | Sdc1 | NM_011519.2 | chr12:8771395-8793687 |
| 18869 | Scarna10 | NR_028517.2 | chr6:125186360-125186431 | 18966 | Sdc2 | NM_008304.2 | chr15:32920722-33034721 |
| 18870 | Scarna13 | NR_028576.1 | chr12:105031074-105031349 | 18967 | Sdc3 | NM_011520.3 | chr4:130792536-130826318 |
| 18871 | Scarna17 | NR_028560.1 | chr18:74778393-74778455 | 18968 | Sdc4 | NM_011521.2 | chr2:164424246-164443188 |
| 18872 | Scarna2 | NR_028538.1 | chr3:108554341-108554402 | 18969 | Sdcbp | NM_001098227.1 | chr4:6365679-6396122 |
| 18873 | Scarna3a | NR_028518.1 | chr1:159340306-159340424 | 18970 | Sdcbp2 | NM_145535.2 | chr2:151572622-151590005 |
| 18874 | Scarna3b | NR_028544.1 | chr12:80391613-80391737 | 18971 | Sdccag3 | NM_001085407.1 | chr2:26382799-26389316 |
| 18875 | Scarna6 | NR_028519.2 | chr1:87784570-87784818 | 18972 | Sdccag8 | NM_029756.3 | chr1:176814659-177020432 |
| 18876 | Scarna8 | NR_028545.1 | chr4:86586452-86586570 | 18973 | Sde2 | NM_145943.1 | chr1:180851150-180868114 |
| 18877 | Scarna9 | NR_028568.2 | chr9:15326288-15326330 | 18974 | Sdf2 | NM_009143.3 | chr11:78245745-78255496 |
| 18878 | Sccpdh | NM_178653.3 | chr1:179668230-179687184 | 18975 | Sdf2l1 | NM_022324.3 | chr16:17130137-17132383 |
| 18879 | Scd1 | NM_009127.4 | chr19:44394449-44407709 | 18976 | Sdf4 | NM_011341.5 | chr4:155992842-156013610 |
| 18880 | Scd2 | NM_009128.2 | chr19:44293675-44306862 | 18977 | Sdha | NM_023281.1 | chr13:74322254-74350240 |
| 18881 | Scd3 | NM_024450.2 | chr19:44203287-44244016 | 18978 | Sdhaf1 | NM_001033140.3 | chr7:30321408-30322375 |
| 18882 | Scd4 | NM_183216.3 | chr19:44333325-44346743 | 18979 | Sdhaf2 | NM_025333.4 | chr19:10500511-10525209 |
| 18883 | Scel | NM_022886.2 | chr14:103513340-103613346 | 18980 | Sdhb | NM_023374.3 | chr4:140961270-140979192 |
| 18884 | Scfd1 | NM_029825.3 | chr12:51377512-51450102 | 18981 | Sdhc | NM_025321.3 | chr1:171129156-171150603 |
| 18885 | Scfd2 | NM_001114660.2 | chr5:74204815-74531742 | 18982 | Sdhd | NM_025848.3 | chr9:50596339-50603849 |
| 18886 | Scg2 | NM_009129.2 | chr1:79434668-79440090 | 18983 | Sdk1 | NM_177879.5 | chr5:141241533-142213791 |
| 18887 | Scg3 | NM_001164790.1 | chr9:75643365-75684056 | 18984 | Sdk2 | NM_172800.2 | chr11:113780789-114065951 |
| 18888 | Scg5 | NM_009162.3 | chr2:113776312-113829091 | 18985 | Sdpr | NM_138741.1 | chr1:51289125-51302960 |
| 18889 | Scgb1a1 | NM_011681.2 | chr19:9083641-9087956 | 18986 | Sdr16c5 | NM_181989.1 | chr4:3995941-4019663 |
| 18890 | Scgb1b19 | NM_001281526.1 | chr7:33287306-33288491 | 18987 | Sdr16c6 | NM_001080710.2 | chr4:4056665-4077514 |
| 18891 | Scgb1b2 | NM_020563.3 | chr7:31290518-31291816 | 18988 | Sdr39u1 | NM_001082975.1 | chr14:55897284-55900232 |
| 18892 | Scgb1b20 | NM_001270543.1 | chr7:33373233-33374558 | 18989 | Sdr42e1 | NM_028725.3 | chr8:117661398-117671515 |
| 18893 | Scgb1b24 | NM_001099329.2 | chr7:33743798-33745103 | 18990 | Sdr9c7 | NM_027301.3 | chr10:127898534-127911759 |
| 18894 | Scgb1b27 | NM_009596.1 | chr7:34021566-34022881 | 18991 | Sds | NM_145565.1 | chr5:120476546-120483905 |
| 18895 | Scgb1b29 | NM_001256066.1 | chr7:32441540-32442844 | 18992 | Sdsl | NM_133902.2 | chr5:120458201-120472763 |
| 18896 | Scgb1b3 | NM_001256073.1 | chr7:31375591-31376916 | 18993 | Sebox | NM_008759.2 | chr11:78503512-78505081 |
| 18897 | Scgb1b30 | NM_001099330.2 | chr7:34095430-34100837 | 18994 | Sec1 | NM_001271578.1 | chr7:45677685-45694402 |
| 18898 | Scgb1b7 | NM_001270542.1 | chr7:31712664-31713980 | 18995 | Sec11a | NM_019951.1 | chr7:80915378-80947550 |
| 18899 | Scgb1c1 | NM_001099742.1 | chr7:140845564-140846769 | 18996 | Sec11c | NM_025468.2 | chr18:65800577-65817657 |
| 18900 | Scgb2b12 | NM_001281508.1 | chr7:32325285-32327245 | 18997 | Sec13 | NM_024206.4 | chr6:113728051-113740681 |
| 18901 | Scgb2b15 | NM_001281523.1 | chr7:32827682-33056552 | 18998 | Sec14l1 | NM_001166506.1 | chr11:117115171-117159268 |
| 18902 | Scgb2b17 | NM_001281524.1 | chr7:33054582-33056552 | 18999 | Sec14l2 | NM_144520.2 | chr11:4097039-4118729 |
| 18903 | Scgb2b19 | NM_001199336.1 | chr7:33278365-33280341 | 19000 | Sec14l3 | NM_001029937.2 | chr11:4064852-4078990 |
| 18904 | Scgb2b2 | NM_207262.2 | chr7:31302768-31304977 | 19001 | Sec14l4 | NM_146013.1 | chr11:4031781-4048009 |
| 18905 | Scgb2b20 | NM_001009952.2 | chr7:33364342-33366319 | 19002 | Sec14l5 | NM_001127725.1 | chr16:5147108-5183934 |
| 18906 | Scgb2b23-ps | NR_045685.1 | chr7:33625144-33653281 | 19003 | Sec16a | NM_153125.2 | chr2:26409430-26445216 |
| 18907 | Scgb2b24 | NM_177446.2 | chr7:33737192-33739295 | 19004 | Sec16b | NM_001159986.1 | chr1:157526169-157568424 |
| 18908 | Scgb2b26 | NM_178308.2 | chr7:33942996-33944985 | 19005 | Sec22a | NM_133704.4 | chr16:35311130-35363918 |
| 18909 | Scgb2b27 | NM_001100464.1 | chr7:34011918-34013942 | 19006 | Sec22b | NM_011342.4 | chr3:97901226-97922318 |
| 18910 | Scgb2b3 | NM_001270541 | chr7:31359037-31362072 | 19007 | Sec22c | NM_001164562.1 | chr9:121680044-121705029 |
| 18911 | Scgb2b7 | NM_001198871.1 | chr7:31703778-31705754 | 19008 | Sec23a | NM_009147.2 | chr12:58958383-59012017 |
| 18912 | Scgb3a1 | NM_054037.2 | chr11:49663594-49665118 | 19009 | Sec23b | NM_001252543.1 | chr2:144556228-144590753 |
| 18913 | Scgb3a2 | NM_001289643.1 | chr18:43764300-43767399 | 19010 | Sec23ip | NM_001029982.2 | chr7:128744869-128784835 |
| 18914 | Scgn | NM_145399.1 | chr13:23953456-23991214 | 19011 | Sec24a | NM_001290785.1 | chr11:51692262-51756834 |

Fig.21 - 99

| | | | |
|---|---|---|---|
| 19012 | Sec24b | NM_207209.2 | chr3:129983184-130060907 |
| 19013 | Sec24c | NM_001168273.1 | chr14:20674320-20694850 |
| 19014 | Sec24d | NM_027135.2 | chr3:123267495-123365636 |
| 19015 | Sec31a | NM_026969.1 | chr5:100361648-100416234 |
| 19016 | Sec31b | NM_001033343.1 | chr19:44516956-44545848 |
| 19017 | Sec61a1 | NM_016906.4 | chr6:88503606-88518800 |
| 19018 | Sec61a2 | NM_021305.3 | chr2:5870986-5895353 |
| 19019 | Sec61b | NM_024171.2 | chr4:47474660-47483233 |
| 19020 | Sec61g | NM_001109971.1 | chr11:16501637-16508484 |
| 19021 | Sec62 | NM_027016.2 | chr3:30792875-30821263 |
| 19022 | Sec63 | NM_153055.3 | chr10:42761495-42832514 |
| 19023 | Secisbp2 | NM_029279.2 | chr13:51651707-51684044 |
| 19024 | Secisbp2l | NM_177608.3 | chr2:125736985-125782870 |
| 19025 | Sectm1a | NM_145373.2 | chr11:121067402-121081139 |
| 19026 | Sectm1b | NM_026907.3 | chr11:121053422-121063569 |
| 19027 | Seh1l | NM_001039088.1 | chr18:67774875-67795487 |
| 19028 | Sel1l | NM_001039089.1 | chr12:91806042-91849157 |
| 19029 | Sel1l2 | NM_001033296.2 | chr2:140229857-140389710 |
| 19030 | Sel1l3 | NM_172710.3 | chr5:53107082-53213452 |
| 19031 | Sele | NM_011345.2 | chr1:164048233-164057677 |
| 19032 | Selenbp1 | NM_009150.3 | chr3:94933082-94944758 |
| 19033 | Selenbp2 | NM_019414.2 | chr3:94693572-94704406 |
| 19034 | Selk | NM_019979.2 | chr14:29968379-29975074 |
| 19035 | Sell | NM_001164059.1 | chr1:164062075-164080785 |
| 19036 | Selm | NM_053267.2 | chr11:3514701-3517351 |
| 19037 | Selo | NM_027905.2 | chr15:89089106-89100340 |
| 19038 | Selp | NM_011347.2 | chr1:164115263-164150026 |
| 19039 | Selplg | NM_009151.3 | chr5:113817797-113830501 |
| 19040 | Selt | NM_001040396.2 | chr3:58576657-58593546 |
| 19041 | Sema3a | NM_001243072.1 | chr5:13396783-13603485 |
| 19042 | Sema3b | NM_001042779.2 | chr9:107597673-107609241 |
| 19043 | Sema3c | NM_013657.5 | chr5:17574815-17730267 |
| 19044 | Sema3d | NM_028882.4 | chr5:12383165-12588943 |
| 19045 | Sema3e | NM_011348.2 | chr5:14025275-14256689 |
| 19046 | Sema3f | NM_011349.3 | chr9:107681501-107710475 |
| 19047 | Sema3g | NM_001025379.1 | chr14:31217872-31229511 |
| 19048 | Sema4a | NM_001163489.1 | chr3:88435961-88461182 |
| 19049 | Sema4b | NM_013659.4 | chr7:80186840-80226524 |
| 19050 | Sema4c | NM_001126047.3 | chr1:36548638-36558381 |
| 19051 | Sema4d | NM_001281880.1 | chr13:51701247-51793747 |
| 19052 | Sema4f | NM_001308374.1 | chr6:82911884-829397 |
| 19053 | Sema4g | NM_011976.1 | chr19:44989343-45003395 |
| 19054 | Sema5a | NM_009154.2 | chr15:32244812-32696341 |
| 19055 | Sema5b | NM_013661.2 | chr16:35541361-35664258 |
| 19056 | Sema6a | NM_018744.2 | chr18:47245253-47368868 |
| 19057 | Sema6b | NM_001130456.1 | chr17:56123084-56140343 |
| 19058 | Sema6c | NM_001272024.1 | chr3:95160419-95174050 |
| 19059 | Sema6d | NM_001290997.1 | chr2:124089968-124667789 |
| 19060 | Sema7a | NM_011352.2 | chr9:57940134-57962865 |
| 19061 | Senp1 | NM_144851.5 | chr15:98038743-98093569 |
| 19062 | Senp2 | NM_029457.3 | chr16:22009483-22049269 |
| 19063 | Senp3 | NM_001163571.1 | chr11:69673109-69682084 |
| 19064 | Senp5 | NM_177103.4 | chr16:31959669-32003287 |
| 19065 | Senp6 | NM_146003.2 | chr9:80066902-80144780 |
| 19066 | Senp7 | NM_001003972.2 | chr16:56072059-56190011 |
| 19067 | Senp8 | NM_001172068.1 | chr9:59734258-59750649 |
| 19068 | Sephs1 | NM_175400.6 | chr2:4881563-4910556 |
| 19069 | Sephs2 | NM_009266.3 | chr7:127271878-127274059 |
| 19070 | Sepn1 | NM_029100.2 | chr4:134537891-134552166 |
| 19071 | Sepp1 | NM_001042613.1 | chr15:3270766-3280508 |
| 19072 | Sepsecs | NM_172490.3 | chr5:52643406-52669701 |
| 19073 | Sept1 | NM_017461.2 | chr7:127214441-127218445 |
| 19074 | Sept10 | NM_001024910.3 | chr10:59141626-59221847 |
| 19075 | Sept11 | NM_001009818.2 | chr5:93093415-93175075 |
| 19076 | Sept12 | NM_027669.3 | chr16:4986857-4997852 |
| 19077 | Sept14 | NM_028826.1 | chr5:129683390-129708511 |
| 19078 | Sept15 | NM_053102.2 | chr3:144570426-144597676 |
| 19079 | Sept2 | NM_001159717.1 | chr1:93478992-93509732 |
| 19080 | Sept3 | NM_011889.2 | chr15:82274934-82294442 |
| 19081 | Sept4 | NM_001284392.1 | chr11:87581060-87590539 |
| 19082 | Sept5 | NM_213614.2 | chr16:18621810-18629938 |
| 19083 | Sept6 | NM_001177323.2 | chrX:36911271-36989695 |
| 19084 | Sept7 | NM_001205367.1 | chr9:25252438-25308571 |
| 19085 | Sept8 | NM_001252332.1 | chr11:53519735-53544096 |
| 19086 | Sept9 | NM_001113486.1 | chr11:117199660-117362325 |
| 19087 | Sepw1 | NM_009156.2 | chr7:15917207-15922371 |
| 19088 | Serac1 | NM_001111017.1 | chr17:6040570-6079739 |
| 19089 | Serbp1 | NM_001113564.1 | chr6:67266978-67289302 |
| 19090 | Serf1 | NM_011353.2 | chr13:100108018-100114233 |
| 19091 | Serf2 | NM_001290837.1 | chr2:121449197-121453420 |
| 19092 | Sergef | NM_013789.2 | chr7:46443158-46639807 |
| 19093 | Serhl | NM_023475.3 | chr15:83100204-83116671 |
| 19094 | Serinc1 | NM_019760.3 | chr10:57515774-57532529 |
| 19095 | Serinc2 | NM_001253386.1 | chr4:130253496-130275220 |
| 19096 | Serinc3 | NM_012032.4 | chr2:163623272-163645143 |
| 19097 | Serinc4 | NM_001025371.2 | chr2:121451176-121456764 |
| 19098 | Serinc5 | NM_172588.2 | chr13:92611137-92711946 |
| 19099 | Serp1 | NM_030685.3 | chr3:58521970-58525884 |
| 19100 | Serp2 | NM_001160326.1 | chr14:76532811-76556687 |
| 19101 | Serpina10 | NM_144834.4 | chr12:103616674-103631444 |
| 19102 | Serpina11 | NM_001166350.1 | chr12:103980242-103989957 |
| 19103 | Serpina12 | NM_026535.2 | chr12:104028768-104044443 |
| 19104 | Serpina1a | NM_001252569.1 | chr12:103853294-103863619 |
| 19105 | Serpina1b | NM_009244.4 | chr12:103728155-103738189 |
| 19106 | Serpina1c | NM_009245.2 | chr12:103894925-103904950 |
| 19107 | Serpina1d | NM_009246.3 | chr12:103763586-103773633 |
| 19108 | Serpina1e | NM_009247.2 | chr12:103946931-103956897 |
| 19109 | Serpina1f | NM_001164742.1 | chr12:103688043-103694689 |
| 19110 | Serpina3a | NM_001167705.1 | chr12:104112723-104121896 |
| 19111 | Serpina3b | NM_173024.3 | chr12:104127995-104139545 |
| 19112 | Serpina3c | NM_008458.2 | chr12:104146906-104153872 |
| 19113 | Serpina3f | NM_001033335.3 | chr12:104214632-104221129 |
| 19114 | Serpina3g | NM_009251.1 | chr12:104214543-104241934 |
| 19115 | Serpina3h | NR_033450.1 | chr12:104247895-104254405 |
| 19116 | Serpina3i | NM_001199940.1 | chr12:104263121-104269365 |
| 19117 | Serpina3j | NM_001101472.2 | chr12:104314569-104320586 |
| 19118 | Serpina3k | NM_011458.2 | chr12:104338485-104345739 |
| 19119 | Serpina3m | NM_009253.2 | chr12:104387163-104394257 |
| 19120 | Serpina3n | NM_009252.2 | chr12:104406707-104414329 |
| 19121 | Serpina4-ps1 | NR_002861.2 | chr12:104077958-104086920 |
| 19122 | Serpina5 | NM_172953.3 | chr12:104101112-104106137 |
| 19123 | Serpina6 | NM_007618.3 | chr12:103646630-103657218 |
| 19124 | Serpina7 | NM_177920.5 | chrX:139079249-139085236 |
| 19125 | Serpina9 | NM_027997.2 | chr12:103996619-104013652 |
| 19126 | Serpinb10 | NM_001160307.1 | chr1:107529002-107549271 |
| 19127 | Serpinb11 | NM_025867.2 | chr1:107362313-107380475 |
| 19128 | Serpinb12 | NM_001199213.1 | chr1:106938956-106957080 |
| 19129 | Serpinb13 | NM_172852.3 | chr1:106980983-107001195 |
| 19130 | Serpinb1a | NM_025429.2 | chr13:32842091-32851185 |
| 19131 | Serpinb1b | NM_173052.2 | chr13:33084102-33094380 |
| 19132 | Serpinb1c | NM_173051.2 | chr13:32881396-32898140 |
| 19133 | Serpinb2 | NM_001174170.1 | chr1:107511422-107525600 |
| 19134 | Serpinb3a | NM_009126.3 | chr1:107045586-107052303 |
| 19135 | Serpinb3b | NM_198680.2 | chr1:107153960-107161114 |
| 19136 | Serpinb3c | NM_201363.2 | chr1:107271200-107278371 |
| 19137 | Serpinb3d | NM_201376.1 | chr1:107078192-107083480 |
| 19138 | Serpinb5 | NM_009257.3 | chr1:106861179-106883348 |
| 19139 | Serpinb6a | NM_001164117.1 | chr13:33917917-33936083 |
| 19140 | Serpinb6b | NM_011454.1 | chr13:32965512-32979037 |
| 19141 | Serpinb6c | NM_148942.2 | chr13:33879815-33905708 |
| 19142 | Serpinb6d | NM_001076790.2 | chr13:33661404-33671585 |
| 19143 | Serpinb6e | NM_001045535.2 | chr13:33832344-33843408 |
| 19144 | Serpinb7 | NM_027548.3 | chr1:107422688-107452689 |
| 19145 | Serpinb8 | NM_001159748.1 | chr1:107590005-107606150 |
| 19146 | Serpinb9 | NM_009256.3 | chr13:33004540-33017955 |
| 19147 | Serpinb9b | NM_011452.2 | chr13:33027413-33040558 |
| 19148 | Serpinb9c | NM_001164524.1 | chr13:33149274-33159754 |
| 19149 | Serpinb9d | NM_011460.2 | chr13:33192958-33203980 |
| 19150 | Serpinb9e | NM_011456.2 | chr13:33249609-33260846 |
| 19151 | Serpinb9f | NM_183197.1 | chr13:33324076-33335366 |
| 19152 | Serpinb9g | NM_011455.3 | chr13:33484789-33496000 |
| 19153 | Serpinc1 | NM_080844.4 | chr1:160978605-161003014 |
| 19154 | Serpind1 | NM_008223.3 | chr16:17331370-17343572 |
| 19155 | Serpine1 | NM_008871.2 | chr5:137061505-137072272 |
| 19156 | Serpine2 | NM_009255.4 | chr1:79794320-79858665 |
| 19157 | Serpine3 | NM_001199945.1 | chr14:62663666-62692243 |
| 19158 | Serpinf1 | NM_011340.3 | chr11:75410028-75422623 |
| 19159 | Serpinf2 | NM_008878.2 | chr11:75431735-75439501 |
| 19160 | Serping1 | NM_009776.3 | chr2:84765359-84775429 |
| 19161 | Serpinh1 | NM_001111043.1 | chr7:99345374-99353239 |
| 19162 | Serpini1 | NM_009250.2 | chr3:75557532-75642523 |
| 19163 | Serpini2 | NM_026460.3 | chr3:75242356-75270078 |
| 19164 | Sertad1 | NM_018820.4 | chr7:27486952-27490314 |
| 19165 | Sertad2 | NM_001038625.1 | chr11:20631976-20653023 |
| 19166 | Sertad3 | NM_133210.2 | chr7:27473839-27477364 |
| 19167 | Sertad4 | NM_001177794.1 | chr1:192844487-192851747 |
| 19168 | Sertm1 | NM_177854.4 | chr3:54897068-54915887 |
| 19169 | Sesn1 | NM_001013370.2 | chr10:41887438-41908436 |
| 19170 | Sesn2 | NM_144907.1 | chr4:132492806-132510456 |
| 19171 | Sesn3 | NM_030261.4 | chr9:14276300-14326134 |
| 19172 | Sestd1 | NM_175465.6 | chr2:77180339-77280592 |
| 19173 | Set | NM_001204875.1 | chr2:30066473-30072577 |
| 19174 | Setbp1 | NM_053099.2 | chr18:78750377-79109391 |
| 19175 | Setd1a | NM_178029.3 | chr7:127777388-127800119 |
| 19176 | Setd1b | NM_001040398.2 | chr5:123142192-123168630 |
| 19177 | Setd2 | NM_001081340.2 | chr9:110532596-110618633 |
| 19178 | Setd3 | NM_028262.3 | chr12:108106430-108179284 |
| 19179 | Setd4 | NM_145482.3 | chr16:93583460-93603815 |
| 19180 | Setd5 | NM_028385.1 | chr6:113077638-113153424 |
| 19181 | Setd6 | NM_001035123.3 | chr8:95715912-95719004 |
| 19182 | Setd7 | NM_080793.5 | chr3:51515317-51560823 |
| 19183 | Setd8 | NM_030241.3 | chr5:124439929-124462311 |
| 19184 | Setdb1 | NM_001163641.1 | chr3:95323524-95357202 |
| 19185 | Setdb2 | NM_001081024.1 | chr14:59402010-59440877 |
| 19186 | Setmar | NM_001276356.1 | chr6:108065044-108077127 |
| 19187 | Setx | NM_198033.2 | chr2:29124991-29182471 |
| 19188 | Sez6 | NM_001291225.1 | chr11:77930838-77979052 |
| 19189 | Sez6l | NM_001253916.1 | chr5:112419150-112577198 |
| 19190 | Sez6l2 | NM_001252566.1 | chr7:126950534-126970606 |
| 19191 | Sf1 | NM_001110791.1 | chr19:6363689-6378038 |
| 19192 | Sf3a1 | NM_026175.5 | chr11:4160353-4182541 |
| 19193 | Sf3a2 | NM_013651.4 | chr10:80798734-80804922 |
| 19194 | Sf3a3 | NM_029157.3 | chr4:124714860-124732422 |
| 19195 | Sf3b1 | NM_031179.2 | chr1:54985169-55027478 |
| 19196 | Sf3b2 | NM_030109.2 | chr19:5273920-5295455 |
| 19197 | Sf3b3 | NM_133953.2 | chr8:110810491-110846803 |
| 19198 | Sf3b4 | NM_153053.4 | chr3:96172505-96177564 |
| 19199 | Sf3b5 | NM_175102.4 | chr10:13008449-13009183 |
| 19200 | Sf3b6 | NM_025323.2 | chr12:4817607-4827659 |
| 19201 | Sfi1 | NM_030207.2 | chr11:3131849-3193463 |
| 19202 | Sfmbt1 | NM_001166531.1 | chr14:30715166-30822721 |
| 19203 | Sfmbt2 | NM_001198808.1 | chr2:10370450-10595253 |
| 19204 | Sfn | NM_018754.2 | chr4:133600555-133602168 |
| 19205 | Sfpq | NM_023603.3 | chr4:127021300-127031236 |

Fig.21 - 100

| | | | | | | | |
|---|---|---|---|---|---|---|---|
| 19206 | Sfr1 | NM_026377.2 | chr19:47731755-47735588 | 19303 | Shisa3 | NM_001033415.3 | chr5:67607882-67613987 |
| 19207 | Sfrp1 | NM_013834.3 | chr8:23411501-23449632 | 19304 | Shisa4 | NM_175259.4 | chr1:135371455-135375063 |
| 19208 | Sfrp2 | NM_009144.2 | chr3:83766320-83774314 | 19305 | Shisa5 | NM_001284332.1 | chr9:109038566-109057792 |
| 19209 | Sfrp4 | NM_016687.3 | chr13:19623174-19632823 | 19306 | Shisa6 | NM_001034874.3 | chr11:66211724-66526126 |
| 19210 | Sfrp5 | NM_018780.3 | chr19:42197970-42202252 | 19307 | Shisa7 | NM_001290291.1 | chr7:4825551-4844696 |
| 19211 | Sfswap | NM_172276.3 | chr5:129501230-129571384 | 19308 | Shisa9 | NM_001174086.1 | chr16:11984112-12270904 |
| 19212 | Sft2d1 | NM_134114.2 | chr17:8311102-8327442 | 19309 | Shkbp1 | NM_138676.2 | chr7:27342132-27356008 |
| 19213 | Sft2d2 | NM_145512.4 | chr1:165174340-165194433 | 19310 | Shmt1 | NM_009171.2 | chr11:60788896-60811265 |
| 19214 | Sft2d3 | NM_026006.1 | chr18:31909093-31911903 | 19311 | Shmt2 | NM_001252316.1 | chr10:127517122-127522444 |
| 19215 | Sfta2 | NM_001163194.1 | chr17:35649707-35650569 | 19312 | Shoc2 | NM_001168505.1 | chr19:53944305-54033278 |
| 19216 | Sftpa1 | NM_023134.4 | chr14:41131787-41136373 | 19313 | Shox2 | NM_013665.1 | chr3:66973265-66981771 |
| 19217 | Sftpb | NM_001282071.1 | chr6:72304609-72314373 | 19314 | Shpk | NM_029031.3 | chr11:73199481-73224506 |
| 19218 | Sftpc | NM_011359.2 | chr14:70520941-70524081 | 19315 | Shprh | NM_001077707.1 | chr10:11149429-11215273 |
| 19219 | Sftpd | NM_009160.2 | chr14:41172211-41185198 | 19316 | Shq1 | NM_181590.5 | chr6:100571810-100671157 |
| 19220 | Sfxn1 | NM_027324.5 | chr13:54071844-54108345 | 19317 | Shroom1 | NM_001290789.1 | chr11:53457204-53467766 |
| 19221 | Sfxn2 | NM_053196.3 | chr19:46573364-46596900 | 19318 | Shroom2 | NM_001290684.1 | chrX:152609508-152769486 |
| 19222 | Sfxn3 | NM_001178012.1 | chr19:45047575-45056383 | 19319 | Shroom3 | NM_001077595.2 | chr5:92809381-92965759 |
| 19223 | Sfxn4 | NM_053198.3 | chr19:60837276-60861430 | 19320 | Shroom4 | NM_001040459.2 | chrX:6400031-6637454 |
| 19224 | Sfxn5 | NM_178639.4 | chr6:85213050-85333422 | 19321 | Siae | NM_011734.3 | chr9:37613846-37648318 |
| 19225 | Sgca | NM_009161.4 | chr11:94962776-94976327 | 19322 | Siah1a | NM_009172.2 | chr8:86723937-86746006 |
| 19226 | Sgcb | NM_011890.4 | chr5:73632748-73647731 | 19323 | Siah1b | NM_009173.2 | chrX:164070702-164076135 |
| 19227 | Sgcd | NM_011891.4 | chr11:46978782-47379302 | 19324 | Siah2 | NM_009174.3 | chr3:58674948-58692388 |
| 19228 | Sgce | NM_001130188.1 | chr6:4674349-4747204 | 19325 | Siah3 | NM_001128093.1 | chr14:75455981-75526141 |
| 19229 | Sgcg | NM_011892.3 | chr14:61219114-61258490 | 19326 | Sidt1 | NM_001159419.1 | chr16:44240179-44332838 |
| 19230 | Sgcz | NM_145841.2 | chr8:37522553-38661508 | 19327 | Sidt2 | NM_001289668.1 | chr9:45937856-45955249 |
| 19231 | Sgip1 | NM_001285852.1 | chr4:102760134-102972682 | 19328 | Sigirr | NM_023059.3 | chr7:141091174-141100546 |
| 19232 | Sgk1 | NM_001161845.2 | chr10:21882183-21999902 | 19329 | Siglec1 | NM_011426.3 | chr2:131069219-131086765 |
| 19233 | Sgk2 | NM_001291152.1 | chr2:162991414-163014139 | 19330 | Siglec15 | NM_001101038.1 | chr18:78043613-78057268 |
| 19234 | Sgk3 | NM_001037759.1 | chr1:9848280-9902568 | 19331 | Siglec5 | NM_001271019.1 | chr7:43351340-43359470 |
| 19235 | Sgms1 | NM_001168525.1 | chr19:32122726-32349316 | 19332 | Siglece | NM_031181.2 | chr7:43651069-43660161 |
| 19236 | Sgms2 | NM_028943.5 | chr3:131318985-131344923 | 19333 | Siglecg | NM_172900.3 | chr7:43408279-43418349 |
| 19237 | Sgol1 | NM_028232.2 | chr17:53674786-53689315 | 19334 | Siglech | NM_178706.5 | chr7:55768183-55778925 |
| 19238 | Sgol2 | NM_001177867.1 | chr1:57985339-58025897 | 19335 | Sigmar1 | NM_001286538.1 | chr4:41738492-41741359 |
| 19239 | Sgpl1 | NM_009163.4 | chr10:61098641-61147665 | 19336 | Sik1 | NM_010831.2 | chr17:31844249-31855792 |
| 19240 | Sgpp1 | NM_030750.3 | chr12:75714247-75735729 | 19337 | Sik2 | NM_178710.3 | chr9:50892800-51009073 |
| 19241 | Sgpp2 | NM_001004173.2 | chr1:78310345-78420286 | 19338 | Sik3 | NM_027498.3 | chr9:46012819-46224194 |
| 19242 | Sgsh | NM_018822.3 | chr11:119343488-119355510 | 19339 | Sike1 | NM_025679.3 | chr3:102995739-103003914 |
| 19243 | Sgsm1 | NM_001162965.1 | chr5:113257473-113310786 | 19340 | Sil1 | NM_030749.2 | chr18:35266395-35498925 |
| 19244 | Sgsm2 | NM_197943.2 | chr11:74849263-74897080 | 19341 | Sim1 | NM_011376.3 | chr10:50895650-50989152 |
| 19245 | Sgsm3 | NM_134091.2 | chr15:80977764-81012290 | 19342 | Sim2 | NM_011377.2 | chr16:94085259-94127032 |
| 19246 | Sgta | NM_024499.1 | chr10:81044072-81060154 | 19343 | Simc1 | NM_176987.4 | chr13:54503804-54551290 |
| 19247 | Sgtb | NM_144838.1 | chr13:104109789-104141441 | 19344 | Sin3a | NM_001110350.1 | chr9:57076375-57128368 |
| 19248 | Sh2b1 | NM_001081459.2 | chr7:126466992-126475130 | 19345 | Sin3b | NM_001113248.2 | chr8:72723269-72739914 |
| 19249 | Sh2b2 | NM_018825.4 | chr5:136218148-136244940 | 19346 | Sipa1 | NM_001164480.1 | chr19:5651184-5663707 |
| 19250 | Sh2b3 | NM_008507.4 | chr5:121815480-121836859 | 19347 | Sipa1l1 | NM_001167983.1 | chr12:82170015-82451784 |
| 19251 | Sh2d1a | NM_011364.4 | chrX:42502564-42522095 | 19348 | Sipa1l2 | NM_001081337.1 | chr8:125418062-125492710 |
| 19252 | Sh2d1b1 | NM_012009.4 | chr1:170277323-170285662 | 19349 | Sipa1l3 | NM_001081028.1 | chr7:29320377-29505460 |
| 19253 | Sh2d1b2 | NM_001033499.1 | chr1:170232870-170251587 | 19350 | Sirpa | NM_001177647.2 | chr2:129592606-129632228 |
| 19254 | Sh2d2a | NM_001025571.2 | chr3:87846754-87855722 | 19351 | Sirpb1a | NM_001002898.1 | chr3:15371826-15426427 |
| 19255 | Sh2d3c | NM_001252547.1 | chr2:32727688-32755007 | 19352 | Sirpb1b | NM_001173460.1 | chr3:15495753-15575065 |
| 19256 | Sh2d4a | NM_028182.1 | chr8:68276527-68347704 | 19353 | Sirt1 | NM_001159589.2 | chr10:63319004-63339035 |
| 19257 | Sh2d4b | NM_177816.3 | chr14:40815867-40892965 | 19354 | Sirt2 | NM_001122765.1 | chr7:28766751-28788665 |
| 19258 | Sh2d5 | NM_001099631.1 | chr4:138250410-138260968 | 19355 | Sirt3 | NM_001127351.1 | chr7:140863662-140881869 |
| 19259 | Sh2d7 | NM_173778.3 | chr9:54538983-54545020 | 19356 | Sirt4 | NM_001167691.1 | chr5:115478009-115484297 |
| 19260 | Sh3bgr | NM_015825.2 | chr16:96200469-96228933 | 19357 | Sirt5 | NM_178848.3 | chr13:43370715-43395203 |
| 19261 | Sh3bgrl | NM_019989.2 | chrX:109095406-109162467 | 19358 | Sirt6 | NM_001163430.1 | chr10:81621785-81627322 |
| 19262 | Sh3bgrl2 | NM_172507.5 | chr9:83548337-83600291 | 19359 | Sirt7 | NM_153056.2 | chr11:120618371-120625002 |
| 19263 | Sh3bgrl3 | NM_080559.1 | chr4:134127405-134128753 | 19360 | Sis | NM_001081137.1 | chr3:72888559-72966866 |
| 19264 | Sh3bp1 | NM_009164.3 | chr15:78899785-78912052 | 19361 | Sit1 | NM_019436.3 | chr4:43482082-43483709 |
| 19265 | Sh3bp2 | NM_001145858.1 | chr5:34543364-34563639 | 19362 | Siva1 | NM_001161737.1 | chr12:112644827-112649152 |
| 19266 | Sh3bp4 | NM_133816.2 | chr1:89070461-89153793 | 19363 | Six1 | NM_009189.3 | chr12:73041826-73046712 |
| 19267 | Sh3bp5 | NM_011894.2 | chr14:31373963-31436033 | 19364 | Six2 | NM_011380.2 | chr17:85684267-85688254 |
| 19268 | Sh3bp5l | NM_001161338.2 | chr11:58330706-58347728 | 19365 | Six3 | NM_011381.4 | chr17:85620833-85626191 |
| 19269 | Sh3d19 | NM_001082414.2 | chr3:86084433-86130521 | 19366 | Six3os1 | NR_015385.2 | chr17:85609089-85618396 |
| 19270 | Sh3d21 | NM_001162533.1 | chr4:126150601-126163341 | 19367 | Six4 | NM_011382.2 | chr12:73100258-73113245 |
| 19271 | Sh3gl1 | NM_001252471.1 | chr17:56016749-56036637 | 19368 | Six5 | NM_011383.1 | chr7:19094543-19098345 |
| 19272 | Sh3gl2 | NM_019535.2 | chr4:85205455-85389379 | 19369 | Six6 | NM_011384.5 | chr12:72939881-72944899 |
| 19273 | Sh3gl3 | NM_001277954.1 | chr7:82259907-82307420 | 19370 | Ska1 | NM_001164355.1 | chr18:74195298-74207818 |
| 19274 | Sh3glb1 | NM_001282037.1 | chr3:144686901-144720335 | 19371 | Ska2 | NM_025377.3 | chr11:87109260-87122974 |
| 19275 | Sh3glb2 | NM_001289709.1 | chr2:30344776-30359316 | 19372 | Ska3 | NM_198605.3 | chr14:57806560-57826163 |
| 19276 | Sh3kbp1 | NM_001135727.2 | chrX:159627271-159975920 | 19373 | Skap1 | NM_001033186.3 | chr11:96464590-96759622 |
| 19277 | Sh3pxd2a | NM_001164717.1 | chr19:47260173-47464411 | 19374 | Skap2 | NM_018773.2 | chr6:51859164-52012549 |
| 19278 | Sh3pxd2b | NM_177364.3 | chr11:32347810-32428183 | 19375 | Ski | NM_011385.2 | chr4:155154074-155222535 |
| 19279 | Sh3rf1 | NM_021506.2 | chr8:61224170-61396072 | 19376 | Skida1 | NM_028317.2 | chr2:18044086-18048449 |
| 19280 | Sh3rf2 | NM_001146299.1 | chr18:42053709-42158695 | 19377 | Skil | NM_001039090.2 | chr3:31095057-31122923 |
| 19281 | Sh3rf3 | NM_172788.3 | chr10:58813358-59138916 | 19378 | Skint1 | NM_001102662.1 | chr4:112006268-112029538 |
| 19282 | Sh3tc1 | NM_194344.2 | chr5:35697179-35729276 | 19379 | Skint10 | NM_177668.2 | chr4:112710829-112774861 |
| 19283 | Sh3tc2 | NM_172628.2 | chr18:61953074-62015719 | 19380 | Skint11 | NM_001166027.1 | chr4:114163383-114245028 |
| 19284 | Sh3yl1 | NM_001289480.1 | chr12:30911668-30960160 | 19381 | Skint2 | NM_001285963.1 | chr4:112613739-112652248 |
| 19285 | Shank1 | NM_001034115.1 | chr7:44310263-44358353 | 19382 | Skint3 | NM_001102474.1 | chr4:112232420-112298245 |
| 19286 | Shank2 | NM_001081370.2 | chr7:144284384-144422675 | 19383 | Skint4 | NM_178786.4 | chr4:112081469-112168076 |
| 19287 | Shank3 | NM_021423.3 | chr15:89499856-89560260 | 19384 | Skint5 | NM_001167876.1 | chr4:113477890-113999503 |
| 19288 | Sharpin | NM_025340.2 | chr15:76347039-76351110 | 19385 | Skint6 | NM_001103199.1 | chr4:112804615-113286973 |
| 19289 | Shb | NM_001033306.1 | chr4:45423275-45530828 | 19386 | Skint7 | NM_001142775.1 | chr4:111972926-111988221 |
| 19290 | Shbg | NM_011367.2 | chr11:69614815-69617905 | 19387 | Skint8 | NM_001100466.1 | chr4:111919554-111950356 |
| 19291 | Shc1 | NM_001113331.2 | chr3:89421620-89430029 | 19388 | Skint9 | NM_177864.2 | chr4:112385968-112433985 |
| 19292 | Shc2 | NM_001024539.1 | chr10:79617937-79637918 | 19389 | Skiv2l | NM_021337.2 | chr17:34839225-34850204 |
| 19293 | Shc3 | NM_009167.3 | chr13:51431041-51567084 | 19390 | Skiv2l2 | NM_028151.2 | chr13:112867779-112927380 |
| 19294 | Shc4 | NM_199022.2 | chr2:125627446-125724148 | 19391 | Skor1 | NM_001163755.1 | chr9:63138163-63147011 |
| 19295 | Shcbp1 | NM_011369.2 | chr8:4735979-4779534 | 19392 | Skor2 | NM_001109743.1 | chr18:76856404-76900342 |
| 19296 | Shcbp1l | NM_001033162.2 | chr1:153425208-153452574 | 19393 | Skp1a | NM_011543.4 | chr11:52231994-52246858 |
| 19297 | Shd | NM_001159523.1 | chr17:55970481-55976619 | 19394 | Skp2 | NM_001285980.1 | chr15:9111981-9155425 |
| 19298 | She | NM_172530.3 | chr3:89831369-89858846 | 19395 | Sla | NM_001029841.4 | chr15:66780818-66831829 |
| 19299 | Shf | NM_001013829.2 | chr2:122348891-122368918 | 19396 | Sla2 | NM_029983.5 | chr2:156872921-156887078 |
| 19300 | Shfm1 | NM_009169.2 | chr6:6558274-6578658 | 19397 | Slain1 | NM_198014.2 | chr14:103650242-103704799 |
| 19301 | Shh | NM_009170.3 | chr5:28456839-28467101 | 19398 | Slain1os | NR_045148.1 | chr14:103692982-103699432 |
| 19302 | Shisa2 | NM_145463.5 | chr14:59625280-59631660 | 19399 | Slain2 | NM_001113423.2 | chr5:72914361-72978841 |

Fig.21 - 101

| | | | |
|---|---|---|---|
| 19400 | Slamf1 | NM_013730.4 | chr1:171767131-171801184 |
| 19401 | Slamf6 | NM_030710.2 | chr1:171917536-171943868 |
| 19402 | Slamf7 | NM_144539.5 | chr1:171632402-171653037 |
| 19403 | Slamf8 | NM_029084.3 | chr1:172581376-172590568 |
| 19404 | Slamf9 | NM_029612.4 | chr1:172475359-172478409 |
| 19405 | Slbp | NM_001289724.1 | chr5:33640054-33652574 |
| 19406 | Slc10a1 | NM_001177561.1 | chr12:80953184-80968079 |
| 19407 | Slc10a2 | NM_011388.2 | chr8:5085622-5105232 |
| 19408 | Slc10a3 | NM_001256104.1 | chrX:74369218-74373349 |
| 19409 | Slc10a3-ubl4 | NM_001278271.1 | chrX:74367446-74373349 |
| 19410 | Slc10a4 | NM_173403.2 | chr5:73006903-73012955 |
| 19411 | Slc10a5 | NM_001010834.2 | chr3:10331733-10335656 |
| 19412 | Slc10a6 | NM_029415.2 | chr5:103605710-103629403 |
| 19413 | Slc10a7 | NM_001009981.2 | chr8:78509327-78734012 |
| 19414 | Slc11a1 | NM_013612.2 | chr1:74375202-74386051 |
| 19415 | Slc11a2 | NM_001146161.1 | chr15:100392657-100423055 |
| 19416 | Slc12a1 | NM_001079690.1 | chr2:125152599-125230001 |
| 19417 | Slc12a2 | NM_009194.3 | chr18:57878677-57946821 |
| 19418 | Slc12a3 | NM_001205311.1 | chr8:94329207-94366221 |
| 19419 | Slc12a4 | NM_001253804.1 | chr8:105943589-105966115 |
| 19420 | Slc12a5 | NM_020333.2 | chr2:164967987-164999731 |
| 19421 | Slc12a6 | NM_133648.2 | chr2:112284687-112363163 |
| 19422 | Slc12a7 | NM_011390.2 | chr13:73763696-73816742 |
| 19423 | Slc12a8 | NM_001083902.1 | chr16:33518594-33664135 |
| 19424 | Slc12a9 | NM_031406.3 | chr5:137314557-137333582 |
| 19425 | Slc13a1 | NM_019481.2 | chr6:24088282-24168092 |
| 19426 | Slc13a2 | NM_022411.3 | chr11:78397275-78422185 |
| 19427 | Slc13a2os | NR_003282.2 | chr11:78394484-78405657 |
| 19428 | Slc13a3 | NM_054055.2 | chr2:165405294-165473197 |
| 19429 | Slc13a4 | NM_172892.3 | chr6:35267952-35308126 |
| 19430 | Slc13a5 | NM_001004148.4 | chr11:72241993-72266604 |
| 19431 | Slc14a1 | NM_001171010.1 | chr18:78100090-78123426 |
| 19432 | Slc14a2 | NM_001110273.1 | chr18:78146143-78179172 |
| 19433 | Slc15a1 | NM_053079.2 | chr14:121459620-121505254 |
| 19434 | Slc15a2 | NM_001145899.1 | chr16:36771876-36784962 |
| 19435 | Slc15a3 | NM_023044.2 | chr19:10842543-10857915 |
| 19436 | Slc15a4 | NM_133895.1 | chr5:127595665-127617392 |
| 19437 | Slc15a5 | NM_177787.4 | chr6:137983589-138079916 |
| 19438 | Slc16a1 | NM_009196.4 | chr3:104638663-104658462 |
| 19439 | Slc16a10 | NM_001114332.1 | chr10:40033534-40142254 |
| 19440 | Slc16a11 | NM_153081.3 | chr11:70213909-70216414 |
| 19441 | Slc16a12 | NM_172838.3 | chr19:34668405-34747111 |
| 19442 | Slc16a13 | NM_172371.3 | chr11:70216791-70220994 |
| 19443 | Slc16a14 | NM_027921.1 | chr1:84906704-84935083 |
| 19444 | Slc16a2 | NM_009197.2 | chrX:103697413-103821988 |
| 19445 | Slc16a3 | NM_001038653.1 | chr11:120949066-120959000 |
| 19446 | Slc16a4 | NM_146136.1 | chr3:107291291-107312116 |
| 19447 | Slc16a5 | NM_001080934.1 | chr11:115462472-115474398 |
| 19448 | Slc16a6 | NM_001029842.1 | chr11:109450855-109473596 |
| 19449 | Slc16a7 | NM_011391.1 | chr10:125227484-125328535 |
| 19450 | Slc16a8 | NM_020516.2 | chr15:79251015-79254748 |
| 19451 | Slc16a9 | NM_025807.3 | chr10:70245275-70285951 |
| 19452 | Slc17a1 | NM_001170638.1 | chr13:23870272-23895730 |
| 19453 | Slc17a2 | NM_144836.2 | chr13:23807026-23823525 |
| 19454 | Slc17a3 | NM_001164743.1 | chr13:23839433-23860714 |
| 19455 | Slc17a4 | NM_177016.3 | chr13:23897888-23915007 |
| 19456 | Slc17a5 | NM_001276452.1 | chr9:78536486-78588045 |
| 19457 | Slc17a6 | NM_080853.3 | chr7:51621829-51671126 |
| 19458 | Slc17a7 | NM_182993.2 | chr7:45163920-45176139 |
| 19459 | Slc17a8 | NM_182959.3 | chr10:89574019-89621249 |
| 19460 | Slc17a9 | NM_183161.3 | chr2:180725338-180742278 |
| 19461 | Slc18a1 | NM_153054.2 | chr8:69037707-69089222 |
| 19462 | Slc18a2 | NM_172523.3 | chr19:59260877-59296012 |
| 19463 | Slc18a3 | NM_021712.2 | chr14:32462436-32464850 |
| 19464 | Slc18b1 | NM_183116.2 | chr10:23796985-23827968 |
| 19465 | Slc19a1 | NM_001199271.1 | chr10:77032738-77050432 |
| 19466 | Slc19a2 | NM_001276455.1 | chr1:164249045-164265385 |
| 19467 | Slc19a3 | NM_030556.2 | chr1:83012522-83038448 |
| 19468 | Slc1a1 | NM_009199.2 | chr19:28835165-28913960 |
| 19469 | Slc1a2 | NM_001077514.3 | chr2:102658682-102790784 |
| 19470 | Slc1a3 | NM_148938.3 | chr15:8634123-8710807 |
| 19471 | Slc1a4 | NM_018861.3 | chr11:20302179-20332713 |
| 19472 | Slc1a5 | NM_009201.2 | chr7:16781345-16798274 |
| 19473 | Slc1a6 | NM_009200.3 | chr10:78780495-78814825 |
| 19474 | Slc1a7 | NM_146255.2 | chr4:107968333-108013532 |
| 19475 | Slc20a1 | NM_001159593.1 | chr2:129198772-129205104 |
| 19476 | Slc20a2 | NM_011394.3 | chr8:22476699-22569616 |
| 19477 | Slc22a1 | NM_009202.5 | chr17:12648874-12675838 |
| 19478 | Slc22a12 | NM_009203.3 | chr19:6535855-6543070 |
| 19479 | Slc22a13 | NM_133980.3 | chr9:119192977-119209105 |
| 19480 | Slc22a13b-ps | NR_033303.1 | chr9:119220490-119231692 |
| 19481 | Slc22a14 | NM_001037749.2 | chr9:119169455-119190393 |
| 19482 | Slc22a15 | NM_001039371.2 | chr3:101855770-101924453 |
| 19483 | Slc22a16 | NM_027572.1 | chr10:40570361-40604132 |
| 19484 | Slc22a17 | NM_021551.4 | chr14:54906726-54913132 |
| 19485 | Slc22a18 | NM_001042760.1 | chr7:143475115-143499332 |
| 19486 | Slc22a19 | NM_144785.2 | chr19:7673075-7711310 |
| 19487 | Slc22a2 | NM_013667.2 | chr17:12584188-12628488 |
| 19488 | Slc22a20 | NM_198650.2 | chr19:5970233-5986143 |
| 19489 | Slc22a21 | NM_019723.2 | chr11:53950823-53980027 |
| 19490 | Slc22a22 | NM_172378.2 | chr15:57243770-57477625 |
| 19491 | Slc22a23 | NM_001033167.3 | chr13:34179157-34345182 |
| 19492 | Slc22a26 | NM_146232.1 | chr19:7781980-7802667 |
| 19493 | Slc22a27 | NM_134256.1 | chr19:7864388-7966027 |
| 19494 | Slc22a28 | NM_001013820.3 | chr19:8062208-8131982 |
| 19495 | Slc22a29 | NM_172776.2 | chr19:8160167-8218839 |
| 19496 | Slc22a3 | NM_011395.2 | chr17:12419973-12507704 |
| 19497 | Slc22a30 | NM_177002.3 | chr19:8335622-8405105 |
| 19498 | Slc22a4 | NM_019687.3 | chr11:53983125-54028090 |
| 19499 | Slc22a5 | NM_011396.3 | chr11:53864541-53891703 |
| 19500 | Slc22a6 | NM_008766.3 | chr19:8617995-8628299 |
| 19501 | Slc22a7 | NM_144856.2 | chr17:46432184-46438477 |
| 19502 | Slc22a8 | NM_001164634.1 | chr19:8591253-8611835 |
| 19503 | Slc23a1 | NM_011397.4 | chr18:35614603-35627227 |
| 19504 | Slc23a2 | NM_018824.2 | chr2:132052495-132145108 |
| 19505 | Slc23a3 | NM_194333.3 | chr1:75125541-75133890 |
| 19506 | Slc24a1 | NM_144813.1 | chr9:64922860-64951607 |
| 19507 | Slc24a2 | NM_001110240.1 | chr4:86983125-87227963 |
| 19508 | Slc24a3 | NM_053195.2 | chr2:145242610-145641939 |
| 19509 | Slc24a4 | NM_172152.2 | chr12:102129418-102266749 |
| 19510 | Slc24a5 | NM_175034.3 | chr2:125068126-125088677 |
| 19511 | Slc25a1 | NM_153150.2 | chr16:17925210-17928219 |
| 19512 | Slc25a10 | NM_013770.2 | chr11:120491836-120501161 |
| 19513 | Slc25a11 | NM_024211.3 | chr11:70644026-70647039 |
| 19514 | Slc25a12 | NM_172436.3 | chr2:71274294-71367554 |
| 19515 | Slc25a13 | NM_001177572.1 | chr6:6041217-6217173 |
| 19516 | Slc25a14 | NM_001166450.2 | chrX:48623673-48662298 |
| 19517 | Slc25a15 | NM_181325.4 | chr8:22375549-22398621 |
| 19518 | Slc25a16 | NM_175194.2 | chr10:62920632-62946494 |
| 19519 | Slc25a17 | NM_011399.3 | chr15:81318920-81360765 |
| 19520 | Slc25a18 | NM_001081048.2 | chr6:120773767-120793982 |
| 19521 | Slc25a19 | NM_001252384.1 | chr11:115614180-115628295 |
| 19522 | Slc25a2 | NM_001159275.1 | chr18:37637377-37638723 |
| 19523 | Slc25a20 | NM_020520.4 | chr9:108662097-108684641 |
| 19524 | Slc25a21 | NM_001167976.1 | chr12:56712633-57160643 |
| 19525 | Slc25a22 | NM_001177576.1 | chr7:141429748-141434594 |
| 19526 | Slc25a23 | NM_025877.4 | chr17:57043710-57059863 |
| 19527 | Slc25a24 | NM_172685.3 | chr3:109123148-109168409 |
| 19528 | Slc25a25 | NM_001164357.1 | chr2:32414486-32451470 |
| 19529 | Slc25a26 | NM_026255.5 | chr6:94500313-94604652 |
| 19530 | Slc25a27 | NM_028711.3 | chr17:43641899-43667015 |
| 19531 | Slc25a28 | NM_145156.1 | chr19:43663800-43674881 |
| 19532 | Slc25a29 | NM_181328.3 | chr12:108825877-108835876 |
| 19533 | Slc25a3 | NM_133668.3 | chr10:91116577-91123963 |
| 19534 | Slc25a30 | NM_026232.3 | chr14:75761998-75787037 |
| 19535 | Slc25a31 | NM_178386.3 | chr3:40708870-40726094 |
| 19536 | Slc25a32 | NM_172402.3 | chr15:39094190-39112716 |
| 19537 | Slc25a33 | NM_027460.2 | chr4:149744035-149774267 |
| 19538 | Slc25a34 | NM_001013780.1 | chr4:141618824-141623834 |
| 19539 | Slc25a35 | NM_028048.2 | chr11:68968130-68972515 |
| 19540 | Slc25a36 | NM_138756.4 | chr9:97077010-97111041 |
| 19541 | Slc25a37 | NM_026331.3 | chr14:69241850-69285103 |
| 19542 | Slc25a38 | NM_144793.1 | chr9:120110398-120124319 |
| 19543 | Slc25a39 | NM_026542.3 | chr11:102402975-102407517 |
| 19544 | Slc25a4 | NM_007450.4 | chr8:46207340-46211009 |
| 19545 | Slc25a40 | NM_001289595.1 | chr5:8422837-8454839 |
| 19546 | Slc25a41 | NM_175333.3 | chr17:57032771-57041654 |
| 19547 | Slc25a42 | NM_001007570.2 | chr8:70184339-70212281 |
| 19548 | Slc25a43 | NM_001085497.2 | chrX:36743631-36777307 |
| 19549 | Slc25a44 | NM_001145876.2 | chr3:88410493-88425141 |
| 19550 | Slc25a45 | NM_134154.3 | chr19:5878465-5885768 |
| 19551 | Slc25a46 | NM_026165.3 | chr18:31580167-31609902 |
| 19552 | Slc25a47 | NM_001012310.2 | chr12:108851128-108856815 |
| 19553 | Slc25a48 | NM_177809.4 | chr13:56438354-56472363 |
| 19554 | Slc25a5 | NM_007451.4 | chrX:36795596-36798808 |
| 19555 | Slc25a51 | NM_001009949.3 | chr4:45395923-45408766 |
| 19556 | Slc25a53 | NM_001082412.2 | chrX:136981115-137038302 |
| 19557 | Slc25a54 | NM_029054.1 | chr3:109080498-109116582 |
| 19558 | Slc26a1 | NM_174870.4 | chr5:108669880-108675365 |
| 19559 | Slc26a10 | NM_177615.3 | chr10:127172425-127180645 |
| 19560 | Slc26a11 | NM_178743.3 | chr11:119355556-119381076 |
| 19561 | Slc26a2 | NM_007885.2 | chr18:61196853-61211596 |
| 19562 | Slc26a3 | NM_021353.3 | chr12:31438218-31473919 |
| 19563 | Slc26a4 | NM_011867.3 | chr12:31519818-31559969 |
| 19564 | Slc26a5 | NM_001289787.1 | chr5:21809000-21862006 |
| 19565 | Slc26a6 | NM_134420.4 | chr9:108854042-108862143 |
| 19566 | Slc26a7 | NM_145947.2 | chr4:14505196-14621778 |
| 19567 | Slc26a8 | NM_001290320.1 | chr17:28637778-28689987 |
| 19568 | Slc26a9 | NM_177243.4 | chr1:131744021-131770405 |
| 19569 | Slc27a1 | NM_011977.3 | chr8:71568926-71586708 |
| 19570 | Slc27a2 | NM_011978.2 | chr2:126553023-126588243 |
| 19571 | Slc27a3 | NM_011988.3 | chr3:90385232-90389927 |
| 19572 | Slc27a4 | NM_011989.4 | chr2:29802679-29817522 |
| 19573 | Slc27a5 | NM_009512.2 | chr7:12988345-12998192 |
| 19574 | Slc27a6 | NM_001081072.1 | chr18:58556239-58612869 |
| 19575 | Slc28a1 | NM_001004184.3 | chr7:81114798-81170416 |
| 19576 | Slc28a2 | NM_172980.2 | chr2:122426476-122461130 |
| 19577 | Slc28a3 | NM_022317.3 | chr13:58553307-58610877 |
| 19578 | Slc29a1 | NM_001199113.1 | chr17:45585199-45593640 |
| 19579 | Slc29a2 | NM_007854.3 | chr19:5024005-5031972 |
| 19580 | Slc29a3 | NM_023596.3 | chr10:60712071-60752782 |
| 19581 | Slc29a4 | NM_146257.2 | chr5:142702100-142722490 |
| 19582 | Slc2a1 | NM_011400.3 | chr4:119108744-119137329 |
| 19583 | Slc2a10 | NM_130451.3 | chr2:165503896-165519917 |
| 19584 | Slc2a12 | NM_178934.4 | chr10:22645010-22703880 |
| 19585 | Slc2a13 | NM_001033633.3 | chr15:91267690-91573261 |
| 19586 | Slc2a2 | NM_031197.2 | chr3:28697902-28728360 |
| 19587 | Slc2a3 | NM_011401.4 | chr6:122727808-122742745 |
| 19588 | Slc2a4 | NM_009204.2 | chr11:69942285-69948190 |
| 19589 | Slc2a4rg-ps | NR_045164.1 | chr2:181384249-181387596 |
| 19590 | Slc2a5 | NM_019741.3 | chr4:150119343-150144168 |
| 19591 | Slc2a6 | NM_001177627.1 | chr2:27021366-27027998 |
| 19592 | Slc2a7 | NM_001085529.1 | chr4:150148971-150168482 |
| 19593 | Slc2a8 | NM_019488.4 | chr2:32972988-32982056 |

Fig.21 - 102

| | | | |
|---|---|---|---|
| 19594 | Slc2a9 | NM_001012363.2 | chr5:38349272-38483385 |
| 19595 | Slc30a1 | NM_009579.3 | chr1:191906780-191913247 |
| 19596 | Slc30a10 | NM_001033286.2 | chr1:185454847-185468761 |
| 19597 | Slc30a2 | NM_001039677.2 | chr4:134343045-134354484 |
| 19598 | Slc30a3 | NM_011773.3 | chr5:31086105-31093527 |
| 19599 | Slc30a4 | NM_001290993.1 | chr2:122681232-122702663 |
| 19600 | Slc30a5 | NM_022885.2 | chr13:100802647-100833427 |
| 19601 | Slc30a6 | NM_001252478.1 | chr17:74395607-74424229 |
| 19602 | Slc30a7 | NM_023214.7 | chr3:115938972-116007406 |
| 19603 | Slc30a8 | NM_172816.3 | chr15:52295552-52335733 |
| 19604 | Slc30a9 | NM_178651.4 | chr5:67306954-67356142 |
| 19605 | Slc31a1 | NM_175090.4 | chr4:62360700-62391769 |
| 19606 | Slc31a2 | NM_001290518.1 | chr4:62291546-62298412 |
| 19607 | Slc32a1 | NM_009508.2 | chr2:158610757-158615747 |
| 19608 | Slc33a1 | NM_001272035.1 | chr3:63942323-63964733 |
| 19609 | Slc34a1 | NM_011392.2 | chr13:55399647-55414695 |
| 19610 | Slc34a2 | NM_011402.3 | chr5:53049352-53071663 |
| 19611 | Slc34a3 | NM_080854.3 | chr2:25228896-25234234 |
| 19612 | Slc35a1 | NM_011895.3 | chr4:34663256-34687438 |
| 19613 | Slc35a2 | NM_001083937.1 | chrX:7884243-7894027 |
| 19614 | Slc35a3 | NM_144902.3 | chr3:116670797-116712280 |
| 19615 | Slc35a4 | NM_001083317.1 | chr18:36679214-36683862 |
| 19616 | Slc35a5 | NM_028756.4 | chr16:45139572-45158673 |
| 19617 | Slc35b1 | NM_016752.1 | chr11:95384921-95391652 |
| 19618 | Slc35b2 | NM_028662.2 | chr17:45564151-45567669 |
| 19619 | Slc35b3 | NM_001170430.1 | chr13:38932139-38960537 |
| 19620 | Slc35b4 | NM_021435.3 | chr6:34155878-34177054 |
| 19621 | Slc35c1 | NM_145832.3 | chr2:92452764-92460518 |
| 19622 | Slc35c2 | NM_001252573.1 | chr2:165276521-165287787 |
| 19623 | Slc35d1 | NM_177732.4 | chr4:103171717-103214884 |
| 19624 | Slc35d2 | NM_001001321.3 | chr13:64096309-64129330 |
| 19625 | Slc35d3 | NM_029529.3 | chr10:19847916-19851459 |
| 19626 | Slc35e1 | NM_177766.3 | chr8:72477994-72492614 |
| 19627 | Slc35e2 | NM_177186.4 | chr4:155601415-155623340 |
| 19628 | Slc35e3 | NM_029875.2 | chr10:117733677-117746358 |
| 19629 | Slc35e4 | NM_153142.3 | chr11:3907021-3914664 |
| 19630 | Slc35f1 | NM_178675.4 | chr10:52690500-53111622 |
| 19631 | Slc35f2 | NM_028060.3 | chr9:53771534-53818161 |
| 19632 | Slc35f3 | NM_175434.3 | chr8:126298578-126395977 |
| 19633 | Slc35f4 | NM_029238.2 | chr14:49298519-49525837 |
| 19634 | Slc35f5 | NM_028787.4 | chr1:125561015-125595684 |
| 19635 | Slc35f6 | NM_175675.3 | chr5:30647938-30659729 |
| 19636 | Slc35g1 | NM_175507.3 | chr19:38395979-38405607 |
| 19637 | Slc35g2 | NM_001101483.1 | chr9:100552187-100571085 |
| 19638 | Slc35g3 | NM_019871.2 | chr11:69759885-69761844 |
| 19639 | Slc36a1 | NM_153139.4 | chr11:55204339-55236330 |
| 19640 | Slc36a1os | NR_046034.1 | chr11:55191718-55197682 |
| 19641 | Slc36a2 | NM_153170.3 | chr11:55158467-55185077 |
| 19642 | Slc36a3 | NM_172258.3 | chr11:55124822-55151706 |
| 19643 | Slc36a4 | NM_172289.4 | chr9:15709768-15738789 |
| 19644 | Slc37a1 | NM_001242427.1 | chr17:31296223-31350698 |
| 19645 | Slc37a2 | NM_001145960.1 | chr9:37229148-37255738 |
| 19646 | Slc37a3 | NM_028123.3 | chr6:39334770-39377707 |
| 19647 | Slc37a4 | NM_008063.2 | chr9:44398175-44402965 |
| 19648 | Slc38a1 | NM_001166456.1 | chr15:96571417-96642343 |
| 19649 | Slc38a10 | NM_001164798.1 | chr11:120103950-120151351 |
| 19650 | Slc38a11 | NM_177074.2 | chr2:65316632-65364026 |
| 19651 | Slc38a2 | NM_175121.3 | chr15:96687391-96699698 |
| 19652 | Slc38a3 | NM_001199217.1 | chr9:107651154-107667399 |
| 19653 | Slc38a4 | NM_027052.3 | chr15:96994822-97055956 |
| 19654 | Slc38a5 | NM_172479.3 | chrX:8271380-8280176 |
| 19655 | Slc38a6 | NM_001037717.3 | chr12:73286847-73354045 |
| 19656 | Slc38a7 | NM_172758.4 | chr8:95835922-95853491 |
| 19657 | Slc38a8 | NM_001009950.1 | chr8:119479602-119501698 |
| 19658 | Slc38a9 | NM_178746.4 | chr13:112660765-112738752 |
| 19659 | Slc39a1 | NM_013901.2 | chr3:90248191-90253612 |
| 19660 | Slc39a10 | NM_172653.2 | chr1:46807543-46853509 |
| 19661 | Slc39a11 | NM_001166503.1 | chr11:113244854-113565815 |
| 19662 | Slc39a12 | NM_001012305.2 | chr2:14388397-14494977 |
| 19663 | Slc39a13 | NM_001290765.1 | chr2:91061780-91070315 |
| 19664 | Slc39a14 | NM_001135151.1 | chr14:70303466-70351424 |
| 19665 | Slc39a2 | NM_001039676.2 | chr14:51893609-51896745 |
| 19666 | Slc39a3 | NM_134135.1 | chr10:81028539-81033912 |
| 19667 | Slc39a4 | NM_028064.2 | chr15:76612382-76616852 |
| 19668 | Slc39a5 | NM_001136237.1 | chr10:128395930-128401224 |
| 19669 | Slc39a6 | NM_139143.3 | chr18:24579880-24603817 |
| 19670 | Slc39a7 | NM_001077709.1 | chr17:34028265-34031690 |
| 19671 | Slc39a8 | NM_001135149.1 | chr3:135825656-135888572 |
| 19672 | Slc39a9 | NM_026244.2 | chr12:80644214-80683342 |
| 19673 | Slc3a1 | NM_009205.2 | chr17:85028346-85064241 |
| 19674 | Slc3a2 | NM_001161413.1 | chr19:8706881-8723369 |
| 19675 | Slc40a1 | NM_016917.2 | chr1:45908069-45925594 |
| 19676 | Slc41a1 | NM_173865.3 | chr1:131828011-131848864 |
| 19677 | Slc41a2 | NM_177388.3 | chr10:83231138-83337817 |
| 19678 | Slc41a3 | NM_001037493.2 | chr6:90619245-90646412 |
| 19679 | Slc43a1 | NM_001081349.2 | chr2:84839407-84863586 |
| 19680 | Slc43a2 | NM_001199283.1 | chr11:75532111-75577572 |
| 19681 | Slc43a3 | NM_021398.3 | chr2:84936645-84958509 |
| 19682 | Slc44a1 | NM_001159633.1 | chr4:53440412-53550164 |
| 19683 | Slc44a2 | NM_001199186.1 | chr9:21320718-21355028 |
| 19684 | Slc44a3 | NM_145394.3 | chr3:121459527-121532344 |
| 19685 | Slc44a4 | NM_023557.3 | chr17:34914465-34930436 |
| 19686 | Slc44a5 | NM_001081263.1 | chr3:153973435-154271720 |
| 19687 | Slc45a1 | NM_173774.3 | chr4:150629395-150652174 |
| 19688 | Slc45a2 | NM_053077.3 | chr15:11000720-11029233 |
| 19689 | Slc45a3 | NM_001177628.2 | chr1:131962914-131982972 |
| 19690 | Slc45a4 | NM_001033219.3 | chr15:73580290-73624744 |
| 19691 | Slc46a1 | NM_026740.2 | chr11:78465700-78471945 |
| 19692 | Slc46a2 | NM_021053.4 | chr4:59905898-59915056 |
| 19693 | Slc46a3 | NM_027872.3 | chr5:147878440-147894802 |
| 19694 | Slc47a1 | NM_026183.5 | chr11:61343399-61378075 |
| 19695 | Slc47a2 | NM_001033542.2 | chr11:61301630-61342860 |
| 19696 | Slc48a1 | NM_026353.4 | chr15:97784364-97792692 |
| 19697 | Slc4a1 | NM_011403.2 | chr11:102348819-102365281 |
| 19698 | Slc4a10 | NM_001242378.1 | chr2:62046514-62326743 |
| 19699 | Slc4a11 | NM_001081162.1 | chr2:130684107-130697519 |
| 19700 | Slc4a1ap | NM_009206.2 | chr5:31526994-31554038 |
| 19701 | Slc4a2 | NM_001253892.1 | chr5:24427520-24440947 |
| 19702 | Slc4a3 | NM_009208.3 | chr1:75546265-75559431 |
| 19703 | Slc4a4 | NM_001136260.1 | chr5:88887259-89239656 |
| 19704 | Slc4a5 | NM_001166067.1 | chr6:83237374-83304945 |
| 19705 | Slc4a7 | NM_001033270.2 | chr14:14703024-14799943 |
| 19706 | Slc4a8 | NM_021530.2 | chr15:100761746-100823971 |
| 19707 | Slc4a9 | NM_001271544.1 | chr18:36528151-36544608 |
| 19708 | Slc50a1 | NM_009057.3 | chr3:89268245-89270570 |
| 19709 | Slc51a | NM_145932.3 | chr16:32475577-32487879 |
| 19710 | Slc51b | NM_178933.2 | chr9:65412752-65422773 |
| 19711 | Slc52a2 | NM_029643.3 | chr15:76538942-76542130 |
| 19712 | Slc52a3 | NM_001164819.1 | chr2:151999865-152009258 |
| 19713 | Slc5a1 | NM_019810.4 | chr5:33104218-33162699 |
| 19714 | Slc5a10 | NM_001033227.2 | chr11:61672781-61720799 |
| 19715 | Slc5a11 | NM_146198.2 | chr7:123214865-123273251 |
| 19716 | Slc5a12 | NM_001003915.2 | chr2:110597298-110649345 |
| 19717 | Slc5a2 | NM_133254.3 | chr7:128265696-128272433 |
| 19718 | Slc5a3 | NM_017391.3 | chr16:92058321-92087473 |
| 19719 | Slc5a4a | NM_133184.2 | chr10:76147450-76189265 |
| 19720 | Slc5a4b | NM_023219.2 | chr10:76058620-76111018 |
| 19721 | Slc5a5 | NM_053248.2 | chr8:70882888-70892757 |
| 19722 | Slc5a6 | NM_001177621.1 | chr5:31036035-31048562 |
| 19723 | Slc5a7 | NM_022025.4 | chr17:54273589-54299034 |
| 19724 | Slc5a8 | NM_145423.2 | chr10:88885991-88929515 |
| 19725 | Slc5a9 | NM_145551.4 | chr4:111875376-111902796 |
| 19726 | Slc6a1 | NM_178703.4 | chr6:114282634-114317525 |
| 19727 | Slc6a11 | NM_172890.3 | chr6:114131240-114249886 |
| 19728 | Slc6a12 | NM_133661.3 | chr6:121346696-121365773 |
| 19729 | Slc6a13 | NM_144512.2 | chr6:121300295-121337718 |
| 19730 | Slc6a14 | NM_020049.4 | chrX:21714899-21742357 |
| 19731 | Slc6a15 | NM_001252330.1 | chr10:103367807-103419379 |
| 19732 | Slc6a17 | NM_172271.2 | chr3:107467547-107518018 |
| 19733 | Slc6a18 | NM_001040692.3 | chr13:73661749-73678023 |
| 19734 | Slc6a19 | NM_028878.3 | chr13:73681156-73700695 |
| 19735 | Slc6a19os | NM_027780.1 | chr13:73699825-73709856 |
| 19736 | Slc6a2 | NM_009209.3 | chr8:92961046-93001667 |
| 19737 | Slc6a20a | NM_139142.2 | chr9:123636906-123678832 |
| 19738 | Slc6a20b | NM_011731.3 | chr9:123593819-123632565 |
| 19739 | Slc6a3 | NM_010020.3 | chr13:73536746-73578672 |
| 19740 | Slc6a4 | NM_010484.2 | chr11:76998596-77032343 |
| 19741 | Slc6a5 | NM_001146013.1 | chr7:49910298-49959493 |
| 19742 | Slc6a6 | NM_009320.4 | chr6:91684066-91759063 |
| 19743 | Slc6a7 | NM_201353.1 | chr18:60995379-61014199 |
| 19744 | Slc6a8 | NM_001142809.1 | chrX:73673132-73682500 |
| 19745 | Slc6a9 | NM_008135.4 | chr4:117835257-117869305 |
| 19746 | Slc7a1 | NM_007513.4 | chr5:148327409-148399904 |
| 19747 | Slc7a10 | NM_017394.4 | chr7:35186384-35201111 |
| 19748 | Slc7a11 | NM_011990.2 | chr3:50364935-50443613 |
| 19749 | Slc7a12 | NM_080852.2 | chr3:14480698-14505818 |
| 19750 | Slc7a13 | NM_028746.3 | chr4:19818726-19842213 |
| 19751 | Slc7a14 | NM_172861.3 | chr3:31202855-31310319 |
| 19752 | Slc7a15 | NM_001038660.2 | chr12:8528482-8539566 |
| 19753 | Slc7a2 | NM_001044740.2 | chr8:40862366-40922070 |
| 19754 | Slc7a3 | NM_007515.3 | chrX:101079209-101085405 |
| 19755 | Slc7a4 | NM_144852.3 | chr16:17572017-17576671 |
| 19756 | Slc7a5 | NM_011404.3 | chr8:121881145-121907686 |
| 19757 | Slc7a6 | NM_178798.3 | chr8:106168874-106198704 |
| 19758 | Slc7a6os | NM_001007567.2 | chr8:106200437-106210933 |
| 19759 | Slc7a7 | NM_001253679.1 | chr14:54369441-54417679 |
| 19760 | Slc7a8 | NM_016972.2 | chr14:54722214-54781886 |
| 19761 | Slc7a9 | NM_001199015.1 | chr7:35449091-35466034 |
| 19762 | Slc8a1 | NM_001112798.2 | chr17:81373104-81738387 |
| 19763 | Slc8a2 | NM_148946.2 | chr7:16130299-16160511 |
| 19764 | Slc8a3 | NM_001167920.1 | chr12:81197914-81333180 |
| 19765 | Slc8b1 | NM_001177594.1 | chr5:120511191-120534024 |
| 19766 | Slc9a1 | NM_016981.2 | chr4:133369771-133423698 |
| 19767 | Slc9a2 | NM_001033289.2 | chr1:40681711-40768885 |
| 19768 | Slc9a3 | NM_001081060.1 | chr13:74121514-74166064 |
| 19769 | Slc9a3r1 | NM_012030.2 | chr11:115163340-115181178 |
| 19770 | Slc9a3r2 | NM_023055.2 | chr17:24639281-24650305 |
| 19771 | Slc9a4 | NM_177084.3 | chr1:40580226-40630731 |
| 19772 | Slc9a5 | NM_001081332.1 | chr8:105348257-105369881 |
| 19773 | Slc9a6 | NM_172780.3 | chrX:56609834-56664230 |
| 19774 | Slc9a7 | NM_177353.3 | chrX:20105754-20291764 |
| 19775 | Slc9a8 | NM_148929.3 | chr2:167421709-167477000 |
| 19776 | Slc9a9 | NM_177909.5 | chr9:94669891-95230452 |
| 19777 | Slc9b1 | NM_028946.3 | chr3:135348036-135397827 |
| 19778 | Slc9b2 | NM_178877.6 | chr3:135307699-135342767 |
| 19779 | Slc9c1 | NM_198106.4 | chr16:45535265-45607001 |
| 19780 | Slco1a1 | NM_013797.5 | chr6:141907280-141946962 |
| 19781 | Slco1a4 | NM_030687.1 | chr6:141805439-141856171 |
| 19782 | Slco1a5 | NM_001267707.1 | chr6:142234226-142278874 |
| 19783 | Slco1a6 | NM_023718.3 | chr6:142085767-142186149 |
| 19784 | Slco1b2 | NM_020495.1 | chr6:141629517-141686635 |
| 19785 | Slco1c1 | NM_001177772.1 | chr6:141524385-141570177 |
| 19786 | Slco2a1 | NM_033314.3 | chr9:103008488-103087849 |
| 19787 | Slco2b1 | NM_001252530.1 | chr7:99657803-99706842 |

Fig.21 - 103

| | | | |
|---|---|---|---|
| 19788 | Slco3a1 | NM_001038643.1 | chr7:74275417-74554780 |
| 19789 | Slco4a1 | NM_148933.1 | chr2:180460977-180474853 |
| 19790 | Slco4c1 | NM_172658.3 | chr1:96818783-96872171 |
| 19791 | Slco5a1 | NM_172841.2 | chr1:12866549-12991135 |
| 19792 | Slco6b1 | NR_120500.1 | chr1:96906176-96997560 |
| 19793 | Slco6c1 | NM_028942.4 | chr1:97059448-97128303 |
| 19794 | Slco6d1 | NM_001164233.1 | chr1:98421123-98509380 |
| 19795 | Slfn1 | NM_011407.1 | chr11:83116844-83122659 |
| 19796 | Slfn10-ps | NR_073523.1 | chr11:83028125-83040042 |
| 19797 | Slfn14 | NM_001166028.1 | chr11:83275111-83286726 |
| 19798 | Slfn2 | NM_011408.1 | chr11:83065111-83070678 |
| 19799 | Slfn3 | NM_011409.1 | chr11:83191329-83215154 |
| 19800 | Slfn4 | NM_011410.3 | chr11:83175185-83190216 |
| 19801 | Slfn5 | NM_183201.4 | chr11:82952101-82964850 |
| 19802 | Slfn5os | NR_045932.1 | chr11:82942340-82960681 |
| 19803 | Slfn8 | NM_001167743.1 | chr11:83002157-83020722 |
| 19804 | Slfn9 | NM_172796.2 | chr11:82980302-82991830 |
| 19805 | Slfnl1 | NM_177570.3 | chr4:120532230-120536661 |
| 19806 | Slirp | NM_026958.3 | chr12:87443895-87449924 |
| 19807 | Slit1 | NM_015748.3 | chr19:41600256-41743856 |
| 19808 | Slit2 | NM_001291227.2 | chr5:47983154-48307736 |
| 19809 | Slit3 | NM_011412.3 | chr11:35121455-35708507 |
| 19810 | Slitrk1 | NM_199065.2 | chr14:108909988-108914239 |
| 19811 | Slitrk2 | NM_001161431.1 | chrX:66649317-66661402 |
| 19812 | Slitrk3 | NM_198864.2 | chr3:73048124-73056943 |
| 19813 | Slitrk4 | NM_178740.4 | chrX:64269443-64276996 |
| 19814 | Slitrk5 | NM_198865.1 | chr14:111675114-111683135 |
| 19815 | Slitrk6 | NM_175499.4 | chr14:110748577-110755149 |
| 19816 | Slk | NM_001164639.1 | chr19:47580018-47645246 |
| 19817 | Slmap | NM_032008.4 | chr14:26413167-26534624 |
| 19818 | Slmo1 | NM_144867.2 | chr18:67464848-67480581 |
| 19819 | Slmo2 | NM_025531.2 | chr2:174465090-174472941 |
| 19820 | Sln | NM_025540.2 | chr9:53850250-53853849 |
| 19821 | Slpi | NM_011414.3 | chr2:164354069-164356507 |
| 19822 | Sltm | NM_025690.3 | chr9:70542869-70592232 |
| 19823 | Slu7 | NM_148673.3 | chr11:43433730-43447981 |
| 19824 | Slurp1 | NM_020519.1 | chr15:74726643-74728026 |
| 19825 | Slx | NM_001136476.1 | chrX:26522656-26545565 |
| 19826 | Slx1b | NM_029420.2 | chr7:126688926-126695783 |
| 19827 | Slx4 | NM_177472.5 | chr16:3979105-4001678 |
| 19828 | Slx4ip | NM_001038641.1 | chr2:136899350-137069778 |
| 19829 | Slxl1 | NM_029181.1 | chrX:55226875-55243736 |
| 19830 | Sly | NM_201530.2 | chrY:55213719-75222053 |
| 19831 | Smad1 | NM_008539.3 | chr8:79338397-79399468 |
| 19832 | Smad2 | NM_001252481.1 | chr18:76261120-76311747 |
| 19833 | Smad3 | NM_016769.4 | chr9:63646766-63757994 |
| 19834 | Smad4 | NM_008540.2 | chr18:73639012-73703741 |
| 19835 | Smad5 | NM_001164041.1 | chr13:56703051-56742378 |
| 19836 | Smad6 | NM_008542.3 | chr9:63953075-64022059 |
| 19837 | Smad7 | NM_001042660.1 | chr18:75367364-75395934 |
| 19838 | Smad9 | NM_019483.5 | chr3:54755581-54801269 |
| 19839 | Smagp | NM_001033872.2 | chr15:100621339-100636940 |
| 19840 | Smap1 | NM_001290683.1 | chr1:23845624-23909736 |
| 19841 | Smap2 | NM_133716.3 | chr4:120968316-121017247 |
| 19842 | Smarca1 | NM_001290708.1 | chrX:47809369-47892552 |
| 19843 | Smarca2 | NM_011416.2 | chr19:26605159-26778321 |
| 19844 | Smarca4 | NM_001174078.1 | chr9:21616168-21704230 |
| 19845 | Smarca5 | NM_053124.2 | chr8:80699942-80739459 |
| 19846 | Smarca5-ps | NR_002888.2 | chr4:145464209-145467961 |
| 19847 | Smarcad1 | NM_001253392.1 | chr6:65043205-65116049 |
| 19848 | Smarcal1 | NM_018817.2 | chr1:72583250-72636790 |
| 19849 | Smarcb1 | NM_001161853.1 | chr10:75896768-75921614 |
| 19850 | Smarcc1 | NM_009211.2 | chr9:110132023-110240178 |
| 19851 | Smarcc2 | NM_001114096.1 | chr10:128459235-128490174 |
| 19852 | Smarcd1 | NM_031842.2 | chr15:99702286-99713995 |
| 19853 | Smarcd2 | NM_001130187.1 | chr11:106263170-106272972 |
| 19854 | Smarcd3 | NM_025891.3 | chr5:24592621-24602002 |
| 19855 | Smarce1 | NM_020618.4 | chr11:99209047-99231017 |
| 19856 | Smc1a | NM_019710.2 | chrX:152016427-152061973 |
| 19857 | Smc1b | NM_080470.1 | chr15:85064688-85131957 |
| 19858 | Smc2 | NM_008017.4 | chr4:52439220-52488365 |
| 19859 | Smc2os | NR_045175.1 | chr4:52430283-52438964 |
| 19860 | Smc3 | NM_007790.3 | chr19:53600395-53645831 |
| 19861 | Smc4 | NM_133786.3 | chr3:69004971-69034623 |
| 19862 | Smc5 | NM_001252684.1 | chr19:23206440-23273897 |
| 19863 | Smc6 | NM_025695.4 | chr12:11265885-11319785 |
| 19864 | Smchd1 | NM_028887.3 | chr17:71344492-71475343 |
| 19865 | Smco1 | NM_183283.2 | chr16:32271608-32274779 |
| 19866 | Smco2 | NM_027059.1 | chr6:146850109-146871404 |
| 19867 | Smco3 | NM_001039558.2 | chr6:136829930-136835450 |
| 19868 | Smco4 | NM_133214.2 | chr9:15505494-15545259 |
| 19869 | Smcp | NM_008574.3 | chr3:92583865-92589024 |
| 19870 | Smcr8 | NM_001085440.1 | chr11:60777524-60788287 |
| 19871 | Smdt1 | NM_026914.1 | chr15:82346045-82349062 |
| 19872 | Smek1 | NM_001160214.1 | chr12:101039408-101083702 |
| 19873 | Smek2 | NM_134034.2 | chr11:29172906-29220797 |
| 19874 | Smg1 | NM_001031814.1 | chr7:118131311-118243637 |
| 19875 | Smg5 | NM_178246.3 | chr3:88336259-88362337 |
| 19876 | Smg6 | NM_001002764.1 | chr11:74925871-75164448 |
| 19877 | Smg7 | NM_001005507.2 | chr1:152836994-152902646 |
| 19878 | Smg8 | NM_024262.1 | chr11:87077731-87086777 |
| 19879 | Smg9 | NM_028047.2 | chr7:24399627-24422777 |
| 19880 | Smgc | NM_198927.3 | chr15:91838327-91861435 |
| 19881 | Smim1 | NM_001163721.1 | chr4:154020469-154026044 |
| 19882 | Smim11 | NM_138743.2 | chr16:92301302-92313041 |
| 19883 | Smim12 | NM_030252.2 | chr4:127243783-127247809 |
| 19884 | Smim13 | NM_001135577.2 | chr13:41249843-41276577 |
| 19885 | Smim14 | NM_133697.3 | chr5:65448754-65492835 |
| 19886 | Smim15 | NM_001048250.2 | chr13:108044473-108049146 |
| 19887 | Smim18 | NM_001206849.1 | chr8:33742111-33747770 |
| 19888 | Smim19 | NM_001012667.2 | chr8:22462613-22476879 |
| 19889 | Smim20 | NM_001145433.1 | chr5:53267105-53278540 |
| 19890 | Smim22 | NM_001253796.1 | chr16:5007315-5008308 |
| 19891 | Smim23 | NM_027050.1 | chr11:32820375-32824594 |
| 19892 | Smim24 | NM_001099917.1 | chr10:81393063-81395079 |
| 19893 | Smim3 | NM_134133.2 | chr18:60474190-60501983 |
| 19894 | Smim4 | NM_001308091.1 | chr14:31124505-31128930 |
| 19895 | Smim5 | NM_183259.3 | chr11:115900138-115906269 |
| 19896 | Smim6 | NM_001162998.1 | chr11:115912016-115913917 |
| 19897 | Smim7 | NM_172396.3 | chr8:72565197-72571048 |
| 19898 | Smim8 | NM_025471.2 | chr4:34768671-34778337 |
| 19899 | Smim9 | NM_001033786.2 | chrX:75146056-75163751 |
| 19900 | Smlr1 | NM_001195596.1 | chr10:25527942-25536272 |
| 19901 | Smn1 | NM_001252629.1 | chr13:100123204-100137698 |
| 19902 | Smndc1 | NM_172429.2 | chr19:53379213-53390573 |
| 19903 | Smo | NM_176996.4 | chr6:29735496-29761366 |
| 19904 | Smoc1 | NM_001146217.1 | chr12:81026807-81186414 |
| 19905 | Smoc2 | NM_022315.2 | chr17:14279505-14404790 |
| 19906 | Smok2a | NM_013741.1 | chr17:13221187-13227658 |
| 19907 | Smok2b | NM_001167913.2 | chr17:13230262-13237189 |
| 19908 | Smok3a | NM_001126045.1 | chr5:138021428-138034665 |
| 19909 | Smok3b | NM_001039889.3 | chr5:138037223-138050633 |
| 19910 | Smok4a | NR_030763.1 | chr17:13521455-13528434 |
| 19911 | Smox | NM_001177833.1 | chr2:131491861-131525183 |
| 19912 | Smpd1 | NM_011421.2 | chr7:105554359-105558389 |
| 19913 | Smpd2 | NM_009213.2 | chr10:41487171-41490340 |
| 19914 | Smpd3 | NM_021491.3 | chr8:106252547-106337988 |
| 19915 | Smpd4 | NM_001164609.1 | chr16:17619353-17644830 |
| 19916 | Smpd5 | NM_001195537.1 | chr15:76294433-76296896 |
| 19917 | Smpdl3a | NM_020561.2 | chr10:57794543-57811830 |
| 19918 | Smpdl3b | NM_133888.2 | chr4:132732965-132757171 |
| 19919 | Smpx | NM_001252591.2 | chrX:157698972-157752591 |
| 19920 | Smr2 | NM_001252679.1 | chr5:88086555-88109053 |
| 19921 | Smr3a | NM_011422.3 | chr5:88002524-88008534 |
| 19922 | Sms | NM_009214.3 | chrX:157443953-157492046 |
| 19923 | Smtn | NM_001159284.1 | chr11:3517521-3539292 |
| 19924 | Smtnl1 | NM_024230.2 | chr2:84811175-84822652 |
| 19925 | Smtnl2 | NM_177776.3 | chr11:72390113-72411713 |
| 19926 | Smu1 | NM_021535.4 | chr4:40735648-40757885 |
| 19927 | Smug1 | NM_027885.3 | chr15:103153289-103163284 |
| 19928 | Smurf1 | NM_001038627.1 | chr5:144876494-144965830 |
| 19929 | Smurf2 | NM_025481.2 | chr11:106820063-106920715 |
| 19930 | Smyd1 | NM_001160127.1 | chr6:71213939-71262281 |
| 19931 | Smyd2 | NM_026796.1 | chr1:189880491-189922288 |
| 19932 | Smyd3 | NM_027188.3 | chr1:178955038-179518003 |
| 19933 | Smyd4 | NM_001102611.1 | chr11:75348432-75405705 |
| 19934 | Smyd5 | NM_144918.2 | chr6:85431975-85446429 |
| 19935 | Snai1 | NM_011427.2 | chr2:167538226-167542811 |
| 19936 | Snai2 | NM_011415.2 | chr16:14705858-14709382 |
| 19937 | Snai3 | NM_013914.2 | chr8:122454205-122460692 |
| 19938 | Snap23 | NM_001177792.1 | chr2:120567670-120600722 |
| 19939 | Snap25 | NM_001291056.1 | chr2:136713449-136782428 |
| 19940 | Snap29 | NM_023348.4 | chr16:17405999-17430826 |
| 19941 | Snap47 | NM_144521.2 | chr11:59407149-59449956 |
| 19942 | Snap91 | NM_001277982.1 | chr9:86765926-86880397 |
| 19943 | Snapc1 | NM_178392.4 | chr12:73964529-73984820 |
| 19944 | Snapc2 | NM_133968.1 | chr8:4253101-4256220 |
| 19945 | Snapc3 | NM_029949.3 | chr4:83417743-83453340 |
| 19946 | Snapc4 | NM_172339.4 | chr2:26362764-26380653 |
| 19947 | Snapc5 | NM_183316.2 | chr9:64179296-64182688 |
| 19948 | Snapin | NM_133854.3 | chr3:90488025-90491013 |
| 19949 | Snca | NM_001042451.2 | chr6:60731572-60829004 |
| 19950 | Sncaip | NM_001199151.1 | chr18:52767810-52915931 |
| 19951 | Sncb | NM_033610.2 | chr13:54758859-54766440 |
| 19952 | Sncg | NM_011430.3 | chr14:34370273-34374669 |
| 19953 | Snd1 | NM_019776.2 | chr6:28480347-28888832 |
| 19954 | Sned1 | NM_172463.4 | chr1:93235896-93296448 |
| 19955 | Snf8 | NM_033568.2 | chr11:96034916-96047405 |
| 19956 | Snhg1 | NR_002896.3 | chr19:8723486-8726326 |
| 19957 | Snhg10 | NR_003145.3 | chr12:105030616-105032279 |
| 19958 | Snhg11 | NM_175692.3 | chr2:158375637-158386145 |
| 19959 | Snhg12 | NR_029468.1 | chr4:132308677-132311024 |
| 19960 | Snhg18 | NR_038186.1 | chr15:32240567-32244662 |
| 19961 | Snhg3 | NR_003270.2 | chr4:132351932-132353686 |
| 19962 | Snhg4 | NR_038073.1 | chr18:35553409-35558316 |
| 19963 | Snhg5 | NR_040721.1 | chr9:88521052-88522897 |
| 19964 | Snhg6 | NR_024067.2 | chr1:9942024-9944118 |
| 19965 | Snhg7 | NR_024068.2 | chr2:26637175-26640244 |
| 19966 | Snhg8 | NR_028574.1 | chr3:123507551-123508336 |
| 19967 | Snhg9 | NR_027900.2 | chr17:24719530-24719965 |
| 19968 | Snip1 | NM_175246.4 | chr4:125066692-125074043 |
| 19969 | Snn | NM_009223.3 | chr16:11066297-11074986 |
| 19970 | Snora15 | NR_003681.1 | chr5:129794560-129794676 |
| 19971 | Snora16a | NR_029412.1 | chr4:132309464-132309600 |
| 19972 | Snora17 | NR_028571.1 | chr2:26639189-26639321 |
| 19973 | Snora19 | NR_034047.1 | chr19:24942235-60774385 |
| 19974 | Snora20 | NR_028479.1 | chr17:12922856-12922917 |
| 19975 | Snora21 | NR_028078.1 | chr11:97781638-97781766 |
| 19976 | Snora23 | NR_033336.1 | chr7:110046363-110046547 |
| 19977 | Snora24 | NR_028573.1 | chr3:123507936-123508066 |
| 19978 | Snora26 | NR_031758.1 | chr5:74093529-74093650 |
| 19979 | Snora28 | NR_033168.1 | chr12:111540945-111541066 |
| 19980 | Snora2b | NR_034052.1 | chr15:98526347-98526459 |
| 19981 | Snora3 | NR_028079.1 | chr7:109520131-109520251 |

Fig.21 - 104

| | | | | | | | |
|---|---|---|---|---|---|---|---|
| 19982 | Snora30 | NR_034045.1 | chr7:127527896-127528003 | 20079 | Snord99 | NR_028537.1 | chr4:132310702-132310758 |
| 19983 | Snora31 | NR_028481.1 | chr14:75847922-75848034 | 20080 | Snph | NM_001291076.1 | chr2:151590548-151632593 |
| 19984 | Snora33 | NR_037680.1 | chr10:23785346-23785451 | 20081 | Snrk | NM_001164572.1 | chr9:122117265-122169702 |
| 19985 | Snora34 | NR_034051.1 | chr15:98519716-98519834 | 20082 | Snrnp200 | NM_177214.4 | chr2:127208403-127240451 |
| 19986 | Snora35 | NR_028446.1 | chrX:146994389-146994508 | 20083 | Snrnp25 | NM_030093.3 | chr11:32205414-32208995 |
| 19987 | Snora36b | NR_034044.1 | chr1:185242925-185243038 | 20084 | Snrnp27 | NM_025665.2 | chr6:86675168-86684491 |
| 19988 | Snora41 | NR_028558.1 | chr1:63179022-63179135 | 20085 | Snrnp35 | NM_029532.2 | chr5:124483154-124491122 |
| 19989 | Snora43 | NR_028572.1 | chr2:26637846-26637985 | 20086 | Snrnp40 | NM_025645.2 | chr4:130360131-130390030 |
| 19990 | Snora44 | NR_034050.1 | chr4:132309952-132310069 | 20087 | Snrnp48 | NM_026382.2 | chr13:38204938-38227663 |
| 19991 | Snora47 | NR_034043.1 | chr13:95330610-95330736 | 20088 | Snrnp70 | NM_009224.3 | chr7:45376453-45395742 |
| 19992 | Snora52 | NR_034049.2 | chr7:141448802-141448936 | 20089 | Snrpa | NM_001046637.1 | chr7:27187005-27195760 |
| 19993 | Snora5c | NR_034042.1 | chr11:6620318-6620419 | 20090 | Snrpa1 | NM_021336.4 | chr7:66060335-66074587 |
| 19994 | Snora61 | NR_034046.1 | chr4:132310306-132310368 | 20091 | Snrpb | NM_009225.2 | chr2:130171635-130179364 |
| 19995 | Snora62 | NR_002902.2 | chr9:120130433-120130561 | 20092 | Snrpb2 | NM_021335.3 | chr2:143063068-143072052 |
| 19996 | Snora64 | NR_002897.1 | chr17:24720788-24720905 | 20093 | Snrpc | NM_011432.2 | chr17:27840086-27851967 |
| 19997 | Snora65 | NR_002898.2 | chr2:32963300-32963418 | 20094 | Snrpd1 | NM_009226.4 | chr18:10617795-10628230 |
| 19998 | Snora68 | NR_002901.1 | chr8:70895758-70895856 | 20095 | Snrpd2 | NM_026943.1 | chr7:19149837-19152726 |
| 19999 | Snora69 | NR_002900.1 | chrX:37082911-37083033 | 20096 | Snrpd3 | NM_026095.4 | chr10:75518041-75535440 |
| 20000 | Snora70 | NR_002899.1 | chrX:74272491-74272620 | 20097 | Snrpe | NM_009227.3 | chr1:133603870-133610280 |
| 20001 | Snora74a | NR_002905.3 | chr18:35557029-35557227 | 20098 | Snrpf | NM_027246.1 | chr10:93583028-93589658 |
| 20002 | Snora75 | NR_028478.1 | chr1:86351169-86351285 | 20099 | Snrpg | NM_026506.2 | chr6:86371539-86378902 |
| 20003 | Snora78 | NR_028515.1 | chr17:24719675-24719832 | 20100 | Snrpn | NM_001082961.1 | chr7:59982501-60097608 |
| 20004 | Snora7a | NR_028546.1 | chr6:115807974-115808103 | 20101 | Snta1 | NM_009228.2 | chr2:154376313-154408084 |
| 20005 | Snora81 | NR_034048.1 | chr16:23110769-23110933 | 20102 | Sntb1 | NM_016667.3 | chr15:55639153-55906949 |
| 20006 | Snord100 | NR_037681.1 | chr10:23785753-23785821 | 20103 | Sntb2 | NM_009229.4 | chr8:106935749-107014192 |
| 20007 | Snord104 | NR_030703.1 | chr11:106500990-106501063 | 20104 | Sntg1 | NM_001290390.1 | chr1:8359738-9299877 |
| 20008 | Snord11 | NR_028521.1 | chr1:59704807-59704870 | 20105 | Sntg2 | NM_172951.3 | chr12:30174556-30373375 |
| 20009 | Snord110 | NR_028547.1 | chr2:130275514-130275573 | 20106 | Sntn | NM_177624.3 | chr14:13670875-13683148 |
| 20010 | Snord111 | NR_028559.1 | chr8:110838534-110838598 | 20107 | Snupn | NM_178374.3 | chr9:56950923-56983199 |
| 20011 | Snord116 | NR_002895.2 | chr7:59675990-59676079 | 20108 | Snurf | NM_033174.3 | chr7:59982500-60005156 |
| 20012 | Snord116l1 | NR_033778.1 | chr7:59894622-59894711 | 20109 | Snw1 | NM_025507.2 | chr12:87449909-87472299 |
| 20013 | Snord116l2 | NR_033779.1 | chr7:59675989-59751580 | 20110 | Snx1 | NM_019727.2 | chr9:66088126-66124886 |
| 20014 | Snord118 | NR_028566.1 | chr19:7478517-7478561 | 20111 | Snx10 | NM_001127348.1 | chr6:51544522-51590670 |
| 20015 | Snord12 | NR_028540.1 | chr2:167065292-167065358 | 20112 | Snx11 | NM_001163389.1 | chr11:96767548-96777555 |
| 20016 | Snord123 | NR_028575.2 | chr15:32241844-32241932 | 20113 | Snx12 | NM_001110310.1 | chrX:101211960-101222563 |
| 20017 | Snord14a | NR_028273.1 | chr2:32777987-32778009 | 20114 | Snx13 | NM_001014973.2 | chr12:35047188-35147477 |
| 20018 | Snord14c | NR_028276.1 | chr10:46244748-46244770 | 20115 | Snx14 | NM_172926.3 | chr9:88376746-88438951 |
| 20019 | Snord14d | NR_028274.1 | chr10:46244747-46244770 | 20116 | Snx15 | NM_026912.1 | chr19:6119403-6128215 |
| 20020 | Snord15a | NR_002172.1 | chr7:99482784-99482932 | 20117 | Snx16 | NM_001127191.2 | chr3:10417816-10440130 |
| 20021 | Snord15b | NR_002173.1 | chr7:99479562-99479707 | 20118 | Snx17 | NM_153680.2 | chr5:31193303-31198900 |
| 20022 | Snord16a | NR_028548.1 | chr9:64175431-64175522 | 20119 | Snx18 | NM_130796.4 | chr13:113592178-113618564 |
| 20023 | Snord17 | NR_030762.1 | chr2:144265981-144266202 | 20120 | Snx19 | NM_028874.2 | chr9:30427328-30466726 |
| 20024 | Snord19 | NR_028523.1 | chr14:31016218-31016272 | 20121 | Snx2 | NM_026386.1 | chr18:53176364-53220860 |
| 20025 | Snord1a | NR_028570.1 | chr11:116674596-116674672 | 20122 | Snx20 | NM_027840.3 | chr8:88626827-88636128 |
| 20026 | Snord1b | NR_028567.1 | chr11:116674146-116674223 | 20123 | Snx21 | NM_133924.3 | chr2:164786020-164792770 |
| 20027 | Snord1c | NR_028569.1 | chr11:116672504-116672582 | 20124 | Snx22 | NM_001025612.2 | chr9:66065175-66069731 |
| 20028 | Snord2 | NR_030705.1 | chr16:23108952-23109020 | 20125 | Snx24 | NM_029394.3 | chr18:53245661-53390825 |
| 20029 | Snord22 | NR_004445.1 | chr19:8725865-8725991 | 20126 | Snx25 | NM_207213.2 | chr8:46033260-46124146 |
| 20030 | Snord23 | NR_028539.1 | chr7:15938761-15938860 | 20127 | Snx27 | NM_001082484.2 | chr3:94497541-94582716 |
| 20031 | Snord32a | NR_000002.8 | chr7:45127382-45127463 | 20128 | Snx29 | NM_001290148.1 | chr16:11405647-11755473 |
| 20032 | Snord33 | NR_001277.2 | chr7:45126863-45126945 | 20129 | Snx3 | NM_017472.4 | chr10:42502053-42535369 |
| 20033 | Snord34 | NR_002455.1 | chr7:45126602-45126668 | 20130 | Snx30 | NM_172468.2 | chr4:59805649-59904740 |
| 20034 | Snord35a | NR_000003.8 | chr7:45126346-45126435 | 20131 | Snx31 | NM_025712.4 | chr15:36504061-36555572 |
| 20035 | Snord35b | NR_000004.8 | chr7:45123025-45123111 | 20132 | Snx32 | NM_001024560.2 | chr19:5495277-5510489 |
| 20036 | Snord37 | NR_028549.1 | chr10:81178960-81179013 | 20133 | Snx33 | NM_175483.5 | chr9:56917199-56928371 |
| 20037 | Snord38a | NR_028524.1 | chr4:117154515-117154574 | 20134 | Snx4 | NM_080557.2 | chr16:33251455-33299562 |
| 20038 | Snord42a | NR_037682.1 | chr11:78181301-78181346 | 20135 | Snx5 | NM_001199188.1 | chr2:144250123-144270902 |
| 20039 | Snord42b | NR_037683.1 | chr11:78183058-78183113 | 20136 | Snx6 | NM_026998.3 | chr12:54746356-54795662 |
| 20040 | Snord43 | NR_028281.1 | chr15:80082858-80082906 | 20137 | Snx7 | NM_001190156.1 | chr3:117781496-117868936 |
| 20041 | Snord45b | NR_028561.1 | chr3:153910566-153910611 | 20138 | Snx8 | NM_172277.2 | chr5:140340302-140389247 |
| 20042 | Snord45c | NR_028525.1 | chr3:153912128-153912189 | 20139 | Snx9 | NM_025664.5 | chr17:5841379-5930711 |
| 20043 | Snord47 | NR_028543.1 | chr1:161038091-161038156 | 20140 | Soat1 | NM_009230.3 | chr1:156428107-156474328 |
| 20044 | Snord49a | NR_028550.1 | chr11:62603459-62603521 | 20141 | Soat2 | NM_146064.1 | chr15:102150574-102163436 |
| 20045 | Snord49b | NR_028526.1 | chr11:62603085-62603148 | 20142 | Sobp | NM_175407.3 | chr10:43002499-43174530 |
| 20046 | Snord4a | NR_030702.1 | chr11:78181686-78181756 | 20143 | Socs1 | NM_001271603.1 | chr16:10783808-10785536 |
| 20047 | Snord52 | NR_028527.1 | chr17:34950949-34951008 | 20144 | Socs2 | NM_001168655.1 | chr10:95411489-95416212 |
| 20048 | Snord53 | NR_028551.1 | chr7:71640888-110848315 | 20145 | Socs3 | NM_007707.3 | chr11:117966086-117969366 |
| 20049 | Snord55 | NR_030704.1 | chr4:117155770-117155848 | 20146 | Socs4 | NM_080843.2 | chr14:47277142-47291591 |
| 20050 | Snord57 | NR_028528.1 | chr2:130278025-130278088 | 20147 | Socs5 | NM_019654.2 | chr17:87107678-87137588 |
| 20051 | Snord58b | NR_028552.1 | chr14:52069314-75001122 | 20148 | Socs6 | NM_018821.4 | chr18:88867879-88894207 |
| 20052 | Snord61 | NR_002903.1 | chrX:57391447-57391509 | 20149 | Socs7 | NM_138657.3 | chr11:97362550-97398542 |
| 20053 | Snord64 | NR_028529.1 | chr7:59978810-59978856 | 20150 | Sod1 | NM_011434.1 | chr16:90220741-90226324 |
| 20054 | Snord65 | NR_028541.1 | chr11:62604529-62604586 | 20151 | Sod2 | NM_013671.3 | chr17:13007838-13018119 |
| 20055 | Snord66 | NR_028530.1 | chr16:20684255-20684313 | 20152 | Sod3 | NM_011435.3 | chr5:52363803-52369738 |
| 20056 | Snord67 | NR_028553.1 | chr2:91596080-91596178 | 20153 | Soga1 | NM_001164663.1 | chr2:157010441-157079265 |
| 20057 | Snord68 | NR_028128.1 | chr8:123103057-123103105 | 20154 | Soga3 | NM_026138.2 | chr10:29143995-29199628 |
| 20058 | Snord69 | NR_028531.1 | chr14:31014292-31014351 | 20155 | Sohlh1 | NM_001001714.1 | chr2:25842996-25847248 |
| 20059 | Snord7 | NR_028362.1 | chr11:83294303-83294399 | 20156 | Sohlh2 | NM_028937.3 | chr3:55182043-55209957 |
| 20060 | Snord70 | NR_028554.1 | chr1:59691957-59692010 | 20157 | Son | NM_019973.2 | chr16:91647823-91663318 |
| 20061 | Snord71 | NR_028532.1 | chr8:109839311-109839375 | 20158 | Sorbs1 | NM_001034962.1 | chr19:40292039-40451398 |
| 20062 | Snord72 | NR_028091.1 | chr15:5118420-5118480 | 20159 | Sorbs2 | NM_001205219.1 | chr8:45507787-45827906 |
| 20063 | Snord73a | NR_004417.1 | chr3:86138790-86138858 | 20160 | Sorbs2os | NR_045739.1 | chr8:45723324-45819297 |
| 20064 | Snord8 | NR_028542.1 | chr14:52209785-52209884 | 20161 | Sorbs3 | NM_001271407.1 | chr14:70180467-70206022 |
| 20065 | Snord82 | NR_002851.1 | chr1:86356259-86356327 | 20162 | Sorcs1 | NM_001252501.1 | chr19:50143300-50678646 |
| 20066 | Snord83b | NR_028282.1 | chr15:80078502-80078579 | 20163 | Sorcs2 | NM_030889.2 | chr5:36017180-36398139 |
| 20067 | Snord85 | NR_028565.1 | chr4:130749637-130749702 | 20164 | Sorcs3 | NM_025696.3 | chr19:48206024-48805505 |
| 20068 | Snord87 | NR_004410.1 | chr1:9942469-9942543 | 20165 | Sord | NM_146126.4 | chr2:122234838-122265337 |
| 20069 | Snord88a | NR_028533.1 | chr7:44250149-44250221 | 20166 | Sorl1 | NM_011436.3 | chr9:41968488-42124289 |
| 20070 | Snord88c | NR_028534.1 | chr7:44249854-44249938 | 20167 | Sort1 | NM_001271599.1 | chr3:108284063-108361519 |
| 20071 | Snord89 | NR_028555.2 | chr1:39548746-39548840 | 20168 | Sos1 | NM_009231.2 | chr17:80393751-80480453 |
| 20072 | Snord90 | NR_028535.1 | chr2:37400060-37400128 | 20169 | Sos2 | NM_001135559.1 | chr12:69583760-69681852 |
| 20073 | Snord91a | NR_028562.1 | chr11:74905445-74905505 | 20170 | Sost | NM_024449.6 | chr11:101962457-101967015 |
| 20074 | Snord92 | NR_028556.2 | chr17:71631278-71631361 | 20171 | Sostdc1 | NM_025312.3 | chr12:36314168-36318452 |
| 20075 | Snord93 | NR_028536.1 | chr5:23852232-23852277 | 20172 | Sowaha | NM_183173.2 | chr11:53476577-53480195 |
| 20076 | Snord95 | NR_028564.1 | chr11:48803138-48803206 | 20173 | Sowahb | NM_175270.4 | chr5:93041122-93045022 |
| 20077 | Snord96a | NR_028563.1 | chr11:48802032-48802109 | 20174 | Sowahc | NM_172939.3 | chr10:59221921-59226433 |
| 20078 | Snord98 | NR_028557.1 | chr10:62765578-62765606 | 20175 | Sowahd | NM_173779.3 | chrX:37048844-37050418 |

Fig.21 - 105

| | | | | | | | |
|---|---|---|---|---|---|---|---|
| 20176 | Sox1 | NM_009233.3 | chr8:12395518-12399555 | 20273 | Spdyb | NM_029048.3 | chr5:143216315-143225882 |
| 20177 | Sox10 | NM_011437.1 | chr15:79154912-79164490 | 20274 | Specc1 | NM_001029936.3 | chr11:62077023-62223013 |
| 20178 | Sox11 | NM_009234.6 | chr12:27334267-27342718 | 20275 | Specc1l | NM_001145826.1 | chr10:75212389-75312400 |
| 20179 | Sox12 | NM_011438.2 | chr2:152393611-152398046 | 20276 | Speer1-ps1 | NR_001586.3 | chr5:11340407-11346273 |
| 20180 | Sox13 | NM_011439.2 | chr1:133382299-133424212 | 20277 | Speer2 | NM_173069.3 | chr16:69856873-69863744 |
| 20181 | Sox14 | NM_011440.1 | chr9:99874105-99876170 | 20278 | Speer3 | NM_027650.3 | chr5:13791618-13796819 |
| 20182 | Sox15 | NM_009235.2 | chr11:69655036-69656727 | 20279 | Speer4a | NM_029376.2 | chr5:26034269-26039505 |
| 20183 | Sox17 | NM_001289464.1 | chr1:4490927-4497354 | 20280 | Speer4b | NM_028561.2 | chr5:27495808-27501392 |
| 20184 | Sox18 | NM_009236.2 | chr2:181669836-181671640 | 20281 | Speer4c | NM_001281511.1 | chr5:15709504-15714271 |
| 20185 | Sox2 | NM_011443.4 | chr3:34649994-34652460 | 20282 | Speer4d | NM_025759.3 | chr5:15619098-15623864 |
| 20186 | Sox21 | NM_177753.3 | chr14:118233233-118237030 | 20283 | Speer4e | NM_001122661.1 | chr5:14933630-14938475 |
| 20187 | Sox2ot | NR_015580.2 | chr3:34560380-34677993 | 20284 | Speer4f | NM_027609.2 | chr5:17476121-17480936 |
| 20188 | Sox3 | NM_009237.2 | chrX:60891365-60893430 | 20285 | Speer5-ps1 | NR_001582.2 | chr10:44170446-44219585 |
| 20189 | Sox30 | NM_173384.2 | chr11:45980309-46017992 | 20286 | Speer6-ps1 | NR_001581.2 | chr13:3149474-3189678 |
| 20190 | Sox4 | NM_009238.2 | chr13:28950729-28953682 | 20287 | Speer7-ps1 | NR_001585.3 | chr5:15680709-15714596 |
| 20191 | Sox5 | NM_001113559.2 | chr6:143828424-144209568 | 20288 | Speer8-ps1 | NR_001584.3 | chr5:14945293-14978541 |
| 20192 | Sox5os3 | NR_040519.1 | chr6:144672867-144693832 | 20289 | Speer9-ps1 | NR_001583.3 | chr7:3128145-3144992 |
| 20193 | Sox6 | NM_001025559.3 | chr7:115470871-116038744 | 20290 | Spef1 | NM_027641.2 | chr2:131170260-131174810 |
| 20194 | Sox7 | NM_011446.1 | chr14:63943705-63950732 | 20291 | Spef2 | NM_177123.4 | chr15:9661545-9748868 |
| 20195 | Sox8 | NM_011447.3 | chr17:25565892-25570686 | 20292 | Speg | NM_001085370.1 | chr1:75385609-75432304 |
| 20196 | Sox9 | NM_011448.4 | chr11:112782209-112787757 | 20293 | Spem1 | NM_028855.1 | chr11:69820870-69822165 |
| 20197 | Sp1 | NM_013672.2 | chr15:102406315-102436404 | 20294 | Spen | NM_019763.2 | chr4:141467889-141538597 |
| 20198 | Sp100 | NM_013673.4 | chr1:85649987-85709997 | 20295 | Spert | NM_001164139.1 | chr14:75582833-75593116 |
| 20199 | Sp110 | NM_030194.1 | chr1:85576898-85598810 | 20296 | Spesp1 | NM_025721.2 | chr9:62270728-62282179 |
| 20200 | Sp140 | NM_001013817.2 | chr1:85600702-85645036 | 20297 | Spg11 | NM_145531.2 | chr2:122053525-122118386 |
| 20201 | Sp2 | NM_001080964.1 | chr11:96953337-96977688 | 20298 | Spg20 | NM_001144987.1 | chr3:55112165-55137332 |
| 20202 | Sp3 | NM_001018042.3 | chr2:72936431-72980446 | 20299 | Spg21 | NM_138584.2 | chr9:65460936-65488470 |
| 20203 | Sp3os | NR_045269.2 | chr2:72979431-72989249 | 20300 | Spg7 | NM_153176.4 | chr8:123065507-123097751 |
| 20204 | Sp4 | NM_001166385.2 | chr12:118231685-118301440 | 20301 | Sphk1 | NM_001172472.1 | chr11:116532443-116536675 |
| 20205 | Sp5 | NM_022435.2 | chr2:70474922-70477726 | 20302 | Sphk2 | NM_001172561.1 | chr7:45709462-45718002 |
| 20206 | Sp6 | NM_031183.2 | chr11:97013568-97024738 | 20303 | Sphkap | NM_172430.3 | chr1:83255780-83408200 |
| 20207 | Sp7 | NM_130458.3 | chr15:102357176-102366271 | 20304 | Spi1 | NM_011355.1 | chr2:91096796-91115756 |
| 20208 | Sp8 | NM_177082.4 | chr12:118846328-118852578 | 20305 | Spib | NM_019866.1 | chr7:44525994-44532071 |
| 20209 | Sp9 | NM_001005343.2 | chr2:73271925-73275771 | 20306 | Spic | NM_011461.3 | chr10:88675269-88683023 |
| 20210 | Spa17 | NM_011449.2 | chr9:37603293-37613720 | 20307 | Spice1 | NM_144550.4 | chr16:44347400-44388492 |
| 20211 | Spaca1 | NM_001290443.1 | chr4:34024871-34050065 | 20308 | Spidr | NM_146068.4 | chr16:15889224-16146851 |
| 20212 | Spaca3 | NM_029367.1 | chr11:80858388-80867814 | 20309 | Spin1 | NM_001283028.1 | chr13:51100879-51152562 |
| 20213 | Spaca4 | NM_027055.3 | chr7:45725106-45725816 | 20310 | Spin2c | NM_001005370.1 | chrX:153832292-153834240 |
| 20214 | Spaca5 | NM_001085393.2 | chrX:21068487-21077959 | 20311 | Spin2d | NM_001243002.1 | chrX:73175301-73176989 |
| 20215 | Spaca6 | NM_001162909.1 | chr17:17830974-17839071 | 20312 | Spin2-ps1 | NM_029106.2 | chrX:3835879-3836656 |
| 20216 | Spaca7 | NM_024279.2 | chr8:12573048-12600738 | 20313 | Spin4 | NM_178753.4 | chrX:95022506-95026682 |
| 20217 | Spag1 | NM_012031.3 | chr15:36179529-36235177 | 20314 | Spink10 | NM_177829.3 | chr18:62548910-62661388 |
| 20218 | Spag11a | NM_153115.1 | chr8:19157886-19159578 | 20315 | Spink11 | NM_001048217.3 | chr18:44190044-44196177 |
| 20219 | Spag11b | NM_001034905.2 | chr8:19140758-19143010 | 20316 | Spink12 | NM_030061.3 | chr18:44104522-44108543 |
| 20220 | Spag16 | NM_001271533.1 | chr1:69826969-69926250 | 20317 | Spink13 | NM_001168423.2 | chr18:62607539-62741387 |
| 20221 | Spag17 | NM_028892.4 | chr3:99885416-100143322 | 20318 | Spink14 | NM_001039218.2 | chr18:44027868-44032208 |
| 20222 | Spag4 | NM_139151.4 | chr2:156065212-156069499 | 20319 | Spink2 | NM_001289764.1 | chr5:77205106-77211259 |
| 20223 | Spag5 | NM_017407.2 | chr11:78301590-78322454 | 20320 | Spink3 | NM_009258.5 | chr18:43728068-43737237 |
| 20224 | Spag6 | NM_015773.2 | chr16:16753015-16829363 | 20321 | Spink4 | NM_011463.2 | chr4:40920055-40931395 |
| 20225 | Spag7 | NM_001167669.1 | chr11:70663768-70669416 | 20322 | Spink5 | NM_001081180.1 | chr18:43963240-44022487 |
| 20226 | Spag8 | NM_001007463.1 | chr4:43651728-43653552 | 20323 | Spink6 | NM_001013797.1 | chr18:44071392-44083610 |
| 20227 | Spag9 | NM_001025428.1 | chr11:94044204-94126082 | 20324 | Spink7 | NM_001001803.2 | chr18:62592412-62596264 |
| 20228 | Spam1 | NM_001079875.2 | chr6:24791187-24801048 | 20325 | Spink8 | NM_183136.2 | chr9:109816626-109826627 |
| 20229 | Sparc | NM_001290817.1 | chr11:55394158-55420080 | 20326 | Spinkl | NM_183123.2 | chr18:44166357-44175073 |
| 20230 | Sparcl1 | NM_010097.4 | chr5:104079108-104114088 | 20327 | Spint1 | NM_016907.3 | chr2:119237359-119249513 |
| 20231 | Spast | NM_001162870.1 | chr17:74338986-74391113 | 20328 | Spint2 | NM_001082548.1 | chr7:29256329-29281977 |
| 20232 | Spata1 | NM_027617.3 | chr3:146457202-146499753 | 20329 | Spint3 | NM_001177401.1 | chr2:164569694-164573456 |
| 20233 | Spata13 | NM_001033272.2 | chr14:60634704-60764556 | 20330 | Spint4 | NM_030058.2 | chr2:164698500-164702448 |
| 20234 | Spata16 | NM_027583.3 | chr3:26637630-26927481 | 20331 | Spint5 | NM_001040055.1 | chr2:164715304-164718068 |
| 20235 | Spata17 | NM_028848.3 | chr1:187048406-187215446 | 20332 | Spire1 | NM_176832.2 | chr18:67488208-67549173 |
| 20236 | Spata18 | NM_178387.3 | chr5:73651379-73679484 | 20333 | Spire2 | NM_172287.2 | chr8:123332712-123369518 |
| 20237 | Spata19 | NM_029299.3 | chr9:27396806-27401711 | 20334 | Spn | NM_001037810.2 | chr7:127133463-127137823 |
| 20238 | Spata2 | NM_170756.2 | chr2:167481135-167492874 | 20335 | Spn-ps | NR_033583.1 | chr15:90891395-90898476 |
| 20239 | Spata20 | NM_144827.4 | chr11:94478903-94485310 | 20336 | Spns1 | NM_023712.3 | chr7:126370059-126377934 |
| 20240 | Spata21 | NM_177867.3 | chr4:141408344-141112759 | 20337 | Spns2 | NM_001276383.1 | chr11:72451637-72475027 |
| 20241 | Spata22 | NM_001045531.1 | chr11:73329740-73346044 | 20338 | Spns3 | NM_029932.3 | chr11:72498155-72550246 |
| 20242 | Spata24 | NM_027733.5 | chr18:35660015-35662186 | 20339 | Spo11 | NM_001083959.1 | chr2:172979841-172993576 |
| 20243 | Spata25 | NM_029370.1 | chr2:164827381-164828534 | 20340 | Spock1 | NM_001166463.1 | chr13:57426090-57908332 |
| 20244 | Spata2l | NM_030176.2 | chr8:123232257-123236209 | 20341 | Spock2 | NM_052994.2 | chr10:60106256-60133913 |
| 20245 | Spata3 | NM_001122732.1 | chr1:86021941-86029958 | 20342 | Spock3 | NM_001252620.1 | chr8:62951231-63357096 |
| 20246 | Spata31 | NM_030047.2 | chr13:64917405-64923195 | 20343 | Spon1 | NM_145584.2 | chr7:113765997-114043375 |
| 20247 | Spata31d1a | NM_028157.2 | chr13:59700082-59706197 | 20344 | Spon2 | NM_133903.3 | chr5:33213517-33218238 |
| 20248 | Spata31d1b | NM_001167593.1 | chr13:59712283-59719289 | 20345 | Spop | NM_025287.2 | chr11:95414082-95493410 |
| 20249 | Spata31d1c | NM_001083890.2 | chr13:65033057-65038004 | 20346 | Spopl | NM_001165997.1 | chr2:23510053-23572104 |
| 20250 | Spata31d1d | NM_177711.3 | chr13:59725924-59731752 | 20347 | Spp1 | NM_001204201.1 | chr5:104435110-104441053 |
| 20251 | Spata32 | NM_177801.3 | chr11:103208126-103218432 | 20348 | Spp2 | NM_029269.3 | chr1:88407009-88426438 |
| 20252 | Spata33 | NM_177279.4 | chr8:123212857-123222045 | 20349 | Sppl2a | NM_023220.2 | chr2:126890394-126933235 |
| 20253 | Spata4 | NM_133711.3 | chr8:54600780-54610098 | 20350 | Sppl2b | NM_175195.3 | chr10:80855274-80868708 |
| 20254 | Spata45 | NM_029336.1 | chr1:191036821-191042941 | 20351 | Sppl2c | NM_001082535.1 | chr11:104186326-104191166 |
| 20255 | Spata5 | NM_001163511.2 | chr3:37419949-37579096 | 20352 | Sppl3 | NM_029012.2 | chr5:115011523-115098790 |
| 20256 | Spata5l1 | NM_001033256.3 | chr2:122630624-122632704 | 20353 | Spr | NM_011467.2 | chr6:85133679-85137764 |
| 20257 | Spata6 | NM_026470.3 | chr4:111720009-111829140 | 20354 | Spred1 | NM_001277256.1 | chr2:117121070-117172853 |
| 20258 | Spata7 | NM_001289572.1 | chr12:98628141-98669819 | 20355 | Spred2 | NM_033523.4 | chr11:19924441-20022597 |
| 20259 | Spata9 | NM_029343.3 | chr13:75967738-75998968 | 20356 | Spred3 | NM_182927.3 | chr7:29158828-29168647 |
| 20260 | Spatc1 | NM_028852.1 | chr15:76268088-76292572 | 20357 | Sprn | NM_183147.2 | chr7:140150627-140154659 |
| 20261 | Spatc1l | NM_029661.1 | chr10:76562271-76570200 | 20358 | Sprr1a | NM_009264.2 | chr3:92483953-92485881 |
| 20262 | Spats1 | NM_027649.3 | chr17:45448936-45474938 | 20359 | Sprr1b | NM_009265.3 | chr3:92436808-92438779 |
| 20263 | Spats2 | NM_139140.1 | chr15:99126844-99212466 | 20360 | Sprr2a1 | NM_011468.4 | chr3:92215835-92222491 |
| 20264 | Spats2l | NM_001164566.1 | chr1:57845570-57948397 | 20361 | Sprr2a2 | NM_001164787.1 | chr3:92215834-92257304 |
| 20265 | Spc24 | NM_026282.5 | chr9:21755441-21760286 | 20362 | Sprr2b | NM_011469.3 | chr3:92316704-92318085 |
| 20266 | Spc25 | NM_001199123.2 | chr2:69193894-69206213 | 20363 | Sprr2d | NM_011470.2 | chr3:92339139-92340873 |
| 20267 | Spcs1 | NM_026911.3 | chr14:30999825-31001666 | 20364 | Sprr2e | NM_011471.2 | chr3:92352142-92353449 |
| 20268 | Spcs2 | NM_025668.3 | chr7:99837568-99858883 | 20365 | Sprr2f | NM_011472.2 | chr3:92365186-92366442 |
| 20269 | Spcs3 | NM_029701.1 | chr8:54520432-54529998 | 20366 | Sprr2g | NR_003548.1 | chr3:92373914-92375229 |
| 20270 | Spdef | NM_013891.4 | chr17:27714446-27728951 | 20367 | Sprr2h | NM_011474.3 | chr3:92385684-92387319 |
| 20271 | Spdl1 | NM_027411.2 | chr11:34809184-34833641 | 20368 | Sprr2i | NM_011475.3 | chr3:92407990-92409271 |
| 20272 | Spdya | NM_001142631.1 | chr17:71552060-71578700 | 20369 | Sprr2j-ps | NR_003185.1 | chr3:92418086-92419396 |

Fig.21 - 106

| | | | |
|---|---|---|---|
| 20370 | Sprr2k | NM_011477.3 | chr3:92432581-92433927 |
| 20371 | Sprr3 | NM_001204427.1 | chr3:92456501-92458720 |
| 20372 | Sprr4 | NM_173070.1 | chr3:92500262-92500493 |
| 20373 | Sprtn | NM_001111141.1 | chr8:124897885-124903813 |
| 20374 | Spry1 | NM_011896.3 | chr3:37639946-37644599 |
| 20375 | Spry2 | NM_011897.3 | chr14:105891946-105896819 |
| 20376 | Spry3 | NM_001030293.2 | chrX_GL456233_random:101351-106392 |
| 20377 | Spry4 | NM_011898.2 | chr18:38586264-38601268 |
| 20378 | Spryd3 | NM_001033277.3 | chr15:102116527-102136215 |
| 20379 | Spryd4 | NM_025716.2 | chr10:128209909-128211794 |
| 20380 | Spryd7 | NM_025697.4 | chr14:61531850-61556886 |
| 20381 | Spsb1 | NM_029035.2 | chr4:149896283-149955006 |
| 20382 | Spsb2 | NM_013539.2 | chr6:124808917-124810616 |
| 20383 | Spsb3 | NM_001163750.1 | chr17:24886673-24892147 |
| 20384 | Spsb4 | NM_145134.3 | chr9:96943481-97018355 |
| 20385 | Spt1 | NM_009267.2 | chr15:103895855-103899312 |
| 20386 | Spta1 | NM_011465.4 | chr1:174172775-174248449 |
| 20387 | Sptan1 | NM_001177667.1 | chr2:29965553-30031451 |
| 20388 | Sptb | NM_013675.3 | chr12:76580487-76710547 |
| 20389 | Sptbn1 | NM_009260.2 | chr11:30106842-30198257 |
| 20390 | Sptbn2 | NM_021287.1 | chr19:4711222-4752352 |
| 20391 | Sptbn4 | NM_001199234.1 | chr7:27356382-27396198 |
| 20392 | Sptlc1 | NM_009269.2 | chr13:53332747-53377361 |
| 20393 | Sptlc2 | NM_011479.4 | chr12:87305057-87388355 |
| 20394 | Sptlc3 | NM_175467.3 | chr2:139493919-139637674 |
| 20395 | Sptssa | NM_134054.2 | chr12:54645373-54656572 |
| 20396 | Sptssb | NM_001164210.2 | chr3:69819538-69859894 |
| 20397 | Spty2d1 | NM_175318.4 | chr7:46990395-47008414 |
| 20398 | Spz1 | NM_030237.3 | chr13:92574631-92576232 |
| 20399 | Sqle | NM_009270.3 | chr15:59315091-59331193 |
| 20400 | Sqrdl | NM_001162503.1 | chr2:122765358-122809551 |
| 20401 | Sqstm1 | NM_001290769.1 | chr11:50200151-50210820 |
| 20402 | Sra1 | NM_001164406.1 | chr18:36667186-36670311 |
| 20403 | Srbd1 | NM_030133.3 | chr17:85984664-86145175 |
| 20404 | Src | NM_001025395.2 | chr2:157424292-157471838 |
| 20405 | Srcin1 | NM_018873.2 | chr11:97509339-97575126 |
| 20406 | Srcrb4d | NM_001160366.1 | chr5:135960222-135974476 |
| 20407 | Srd5a1 | NM_175283.3 | chr13:69573448-69611463 |
| 20408 | Srd5a2 | NM_053188.2 | chr17:74017705-74047916 |
| 20409 | Srd5a3 | NM_020611.4 | chr5:76140272-76155503 |
| 20410 | Srebf1 | NM_011480.4 | chr11:60199083-60220627 |
| 20411 | Srebf2 | NM_033218.1 | chr15:82147268-82204960 |
| 20412 | Srek1 | NM_172592.2 | chr13:103741614-103764582 |
| 20413 | Srek1ip1 | NM_026075.2 | chr13:104817238-104838659 |
| 20414 | Srf | NM_020493.2 | chr17:46546838-46556162 |
| 20415 | Srfbp1 | NM_026040.3 | chr18:52465692-52490738 |
| 20416 | Srgap1 | NM_001081037.2 | chr10:121780990-122047315 |
| 20417 | Srgap2 | NM_001081011.2 | chr1:131285250-131527361 |
| 20418 | Srgap3 | NM_080448.4 | chr6:112717971-112947266 |
| 20419 | Srgn | NM_011157.2 | chr10:62494427-62507755 |
| 20420 | Sri | NM_001080974.2 | chr5:8056541-8069314 |
| 20421 | Srl | NM_175347.4 | chr16:4480227-4523053 |
| 20422 | Srm | NM_009272.4 | chr4:148591512-148594619 |
| 20423 | Srms | NM_011481.3 | chr2:181205562-181213171 |
| 20424 | Srp14 | NM_009273.4 | chr2:118475842-118479696 |
| 20425 | Srp19 | NM_025527.3 | chr18:34331144-34336599 |
| 20426 | Srp54a | NM_011899.4 | chr12:55080495-55115367 |
| 20427 | Srp54b | NM_001100109.1 | chr12:55155103-55263480 |
| 20428 | Srp54c | NM_001100110.1 | chr12:55230477-55263020 |
| 20429 | Srp68 | NM_146032.3 | chr11:116245165-116274217 |
| 20430 | Srp72 | NM_025691.1 | chr5:76974700-76999935 |
| 20431 | Srp9 | NM_012058.3 | chr1:182124736-182132415 |
| 20432 | Srpk1 | NM_016795.3 | chr17:28589591-28622454 |
| 20433 | Srpk2 | NM_009274.2 | chr5:23503355-23616571 |
| 20434 | Srpk3 | NM_019684.2 | chrX:73774404-73778924 |
| 20435 | Srpr | NM_026130.1 | chr9:35211202-35217003 |
| 20436 | Srprb | NM_009275.4 | chr9:103188032-103202065 |
| 20437 | Srpx | NM_016911.4 | chrX:10037976-10117661 |
| 20438 | Srpx2 | NM_001083895.3 | chrX:133908424-133932446 |
| 20439 | Srr | NM_001163311.1 | chr11:74906358-74925798 |
| 20440 | Srrd | NM_027323.2 | chr5:112337390-112343040 |
| 20441 | Srrm1 | NM_001130477.1 | chr4:135320483-135353214 |
| 20442 | Srrm2 | NM_175229.3 | chr17:23803186-23824743 |
| 20443 | Srrm3 | NM_021403.3 | chr5:135818105-135874772 |
| 20444 | Srrm4 | NM_026886.3 | chr5:116439272-116591817 |
| 20445 | Srrm4os | NR_015595.2 | chr5:116438721-116465487 |
| 20446 | Srrt | NM_001109909.1 | chr5:137295703-137307674 |
| 20447 | Srsf1 | NM_001078167.2 | chr11:88047372-88053757 |
| 20448 | Srsf10 | NM_001080387.2 | chr4:135856070-135865818 |
| 20449 | Srsf11 | NM_001093752.1 | chr3:158010492-158036639 |
| 20450 | Srsf12 | NM_177774.4 | chr4:33208990-33233340 |
| 20451 | Srsf2 | NM_011358.2 | chr11:116849896-116853094 |
| 20452 | Srsf3 | NM_013663.5 | chr17:29032659-29043372 |
| 20453 | Srsf4 | NM_020587.2 | chr4:131873638-131901725 |
| 20454 | Srsf5 | NM_001079694.1 | chr12:80945503-80950507 |
| 20455 | Srsf6 | NM_026499.4 | chr2:162931507-162937120 |
| 20456 | Srsf7 | NM_001195485.1 | chr17:80200079-80207305 |
| 20457 | Srsf9 | NM_025573.3 | chr5:115327176-115333080 |
| 20458 | Srxn1 | NM_029688.5 | chr2:152105523-152111376 |
| 20459 | Sry | NM_011564.1 | chrY:2662470-2663658 |
| 20460 | Ss18 | NM_001161369.1 | chr18:14625628-14682914 |
| 20461 | Ss18l1 | NM_178750.5 | chr2:180042482-180070201 |
| 20462 | Ssb | NM_001110145.1 | chr2:69861561-69871846 |
| 20463 | Ssbp1 | NM_001286663.1 | chr6:40471467-40481823 |
| 20464 | Ssbp2 | NM_024186.4 | chr13:91461096-91706175 |
| 20465 | Ssbp3 | NM_023672.2 | chr4:106911469-107049694 |
| 20466 | Ssbp4 | NM_133772.2 | chr8:70597489-70608314 |
| 20467 | Ssc5d | NM_173008.2 | chr7:4925843-4944797 |
| 20468 | Ssfa2 | NM_080558.4 | chr2:79635424-79672964 |
| 20469 | Ssh1 | NM_198109.4 | chr5:113942218-113993757 |
| 20470 | Ssh2 | NM_001291190.1 | chr11:77348279-77460219 |
| 20471 | Ssh3 | NM_198113.2 | chr19:4261668-4269172 |
| 20472 | Ssmem1 | NM_027073.1 | chr6:30509848-30520253 |
| 20473 | Ssna1 | NM_023464.2 | chr2:25271038-25272418 |
| 20474 | Sspn | NM_010656.2 | chr6:145934146-145965225 |
| 20475 | Sspo | NM_173428.3 | chr6:48448228-48501250 |
| 20476 | Ssr1 | NM_025965.3 | chr13:37971400-37994190 |
| 20477 | Ssr2 | NM_025448.3 | chr3:88579670-88588413 |
| 20478 | Ssr3 | NM_026155.3 | chr3:65379656-65392553 |
| 20479 | Ssr4 | NM_001166480.1 | chrX:73787027-73790828 |
| 20480 | Ssrp1 | NM_001136081.2 | chr2:85037450-85047114 |
| 20481 | Sssca1 | NM_020491.5 | chr19:5730305-5731732 |
| 20482 | Sst | NM_009215.1 | chr16:23889580-23890844 |
| 20483 | Sstr1 | NM_009216.3 | chr12:58211803-58216036 |
| 20484 | Sstr2 | NM_001042606.2 | chr11:113619480-113625366 |
| 20485 | Sstr3 | NM_009218.3 | chr15:78537014-78544345 |
| 20486 | Sstr4 | NM_009219.3 | chr2:148395376-148396764 |
| 20487 | Sstr5 | NM_001191008.1 | chr17:25489874-25497288 |
| 20488 | Ssty1 | NM_009220.2 | chrY:13376412-21097715 |
| 20489 | Ssty2 | NM_023546.3 | chrY:22512350-80465434 |
| 20490 | Ssu2 | NM_175525.3 | chr6:112359323-112388023 |
| 20491 | Ssu72 | NM_026899.3 | chr4:155704814-155733873 |
| 20492 | Ssx2ip | NM_001253768.1 | chr3:146404641-146440137 |
| 20493 | Ssx9 | NM_199063.2 | chrX:8748429-8754587 |
| 20494 | Ssxb1 | NM_026492.3 | chrX:8413304-8422157 |
| 20495 | Ssxb10 | NM_199064.1 | chrX:8327423-8336235 |
| 20496 | Ssxb2 | NM_001001450.4 | chrX:8454342-8461726 |
| 20497 | Ssxb3 | NM_198898.2 | chrX:8583543-8589246 |
| 20498 | Ssxb5 | NM_199319.3 | chrX:8803690-8809386 |
| 20499 | Ssxb6 | NM_001205108.1 | chrX:8542603-8548255 |
| 20500 | Ssxb8 | NM_001081565.3 | chrX:8685330-8690844 |
| 20501 | Ssxb9 | NM_199066.2 | chrX:8366977-8375388 |
| 20502 | St13 | NM_133726.2 | chr15:81365043-81399694 |
| 20503 | St14 | NM_011176.4 | chr9:31088589-31131799 |
| 20504 | St18 | NM_001244692.1 | chr1:6487230-6860940 |
| 20505 | St3gal1 | NM_009177.4 | chr15:67102874-67176882 |
| 20506 | St3gal2 | NM_009179.3 | chr8:110919864-110972497 |
| 20507 | St3gal3 | NM_001161774.2 | chr4:117932152-118134946 |
| 20508 | St3gal4 | NM_009178.3 | chr9:35046578-35116810 |
| 20509 | St3gal5 | NM_001035228.2 | chr6:72097607-72154570 |
| 20510 | St3gal6 | NM_018784.2 | chr16:58470540-58523312 |
| 20511 | St5 | NM_001001326.1 | chr7:109523910-109617147 |
| 20512 | St6gal1 | NM_001252505.1 | chr16:23226020-23360350 |
| 20513 | St6gal2 | NM_172829.2 | chr17:55445716-55499226 |
| 20514 | St6galnac1 | NM_011371.2 | chr11:116765024-116775507 |
| 20515 | St6galnac2 | NM_009180.3 | chr11:116676704-116694660 |
| 20516 | St6galnac3 | NM_011372.2 | chr3:153202508-153725133 |
| 20517 | St6galnac4 | NM_001276425.1 | chr2:32587077-32599696 |
| 20518 | St6galnac5 | NM_012028.4 | chr3:152820709-152982207 |
| 20519 | St6galnac6 | NM_001025310.2 | chr2:32606984-32620809 |
| 20520 | St7 | NM_001083315.2 | chr6:17749169-17943023 |
| 20521 | St7l | NM_001253702.1 | chr3:104864505-104930063 |
| 20522 | St8sia1 | NM_011374.2 | chr6:142821540-142964452 |
| 20523 | St8sia2 | NM_009181.2 | chr7:73939119-74013682 |
| 20524 | St8sia3 | NM_009182.3 | chr18:64254358-64276144 |
| 20525 | St8sia3os | NR_045366.1 | chr18:64157723-64277180 |
| 20526 | St8sia4 | NM_001159745.1 | chr1:95627242-95667594 |
| 20527 | St8sia5 | NM_013666.2 | chr18:77185846-77255450 |
| 20528 | St8sia6 | NM_145838.1 | chr2:13654935-13793520 |
| 20529 | Stab1 | NM_138672.2 | chr14:31139016-31168641 |
| 20530 | Stab2 | NM_138673.2 | chr10:86841209-87007942 |
| 20531 | Stac | NM_016853.2 | chr9:111561433-111690216 |
| 20532 | Stac2 | NM_146028.4 | chr11:98036623-98053462 |
| 20533 | Stac3 | NM_177707.3 | chr10:127501716-127508815 |
| 20534 | Stag1 | NM_009282.3 | chr9:100643622-100958544 |
| 20535 | Stag2 | NM_001077712.2 | chrX:42149411-42277186 |
| 20536 | Stag3 | NM_016964.2 | chr5:138280508-138312393 |
| 20537 | Stam | NM_011484.2 | chr2:14074111-14148330 |
| 20538 | Stam2 | NM_019667.2 | chr2:52692205-52742149 |
| 20539 | Stambp | NM_024239.2 | chr6:83543205-83572504 |
| 20540 | Stambpl1 | NM_029682.4 | chr19:34192269-34240328 |
| 20541 | Stamos | NR_038162.1 | chr2:14070332-14073934 |
| 20542 | Stap1 | NM_019992.4 | chr5:86071745-86104000 |
| 20543 | Stap2 | NM_145934.2 | chr17:55997075-56005606 |
| 20544 | Star | NM_011485.4 | chr8:25808512-25815982 |
| 20545 | Stard10 | NM_019990.4 | chr7:101321318-101346312 |
| 20546 | Stard13 | NM_001163493.1 | chr5:151037514-151190193 |
| 20547 | Stard3 | NM_021547.3 | chr11:98358367-98381112 |
| 20548 | Stard3nl | NM_024270.3 | chr13:19357675-19395813 |
| 20549 | Stard4 | NM_133774.4 | chr18:33201420-33213816 |
| 20550 | Stard5 | NM_023377.4 | chr7:83632016-83642328 |
| 20551 | Stard6 | NM_001289648.1 | chr18:70472571-70501065 |
| 20552 | Stard7 | NM_139308.2 | chr2:127270228-127298934 |
| 20553 | Stard8 | NM_199018.2 | chrX:99042580-99074728 |
| 20554 | Stat1 | NM_001205313.1 | chr1:52119621-52161865 |
| 20555 | Stat2 | NM_019963.1 | chr10:128270575-128292849 |
| 20556 | Stat3 | NM_011486.5 | chr11:100886805-100939540 |
| 20557 | Stat4 | NM_011487.5 | chr1:52008239-52107189 |
| 20558 | Stat5a | NM_001164062.1 | chr11:100860483-100885169 |
| 20559 | Stat5b | NM_001113563.1 | chr11:100780730-100850585 |
| 20560 | Stat6 | NM_009284.2 | chr10:127642985-127660987 |
| 20561 | Stau1 | NM_001109905.2 | chr2:166947548-166996299 |
| 20562 | Stau2 | NM_001111272.1 | chr1:16228809-16519302 |
| 20563 | Stbd1 | NM_175096.3 | chr5:92603050-92606579 |

Fig.21 - 107

| | | | |
|---|---|---|---|
| 20564 | Stc1 | NM_009285.3 | chr14:69029288-69041401 |
| 20565 | Stc2 | NM_011491.3 | chr11:31359440-31370061 |
| 20566 | Steap1 | NM_027399.3 | chr5:5736321-5749317 |
| 20567 | Steap2 | NM_001103156.2 | chr5:5664828-5694089 |
| 20568 | Steap3 | NM_001085409.1 | chr1:120226415-120271082 |
| 20569 | Steap4 | NM_054098.3 | chr5:7960471-7982213 |
| 20570 | Stfa1 | NM_001082543.1 | chr16:36277147-36285371 |
| 20571 | Stfa2 | NM_001082545.1 | chr16:36403945-36408363 |
| 20572 | Stfa2l1 | NM_173869.3 | chr16:36156810-36161948 |
| 20573 | Stfa3 | NM_025288.2 | chr16:36450536-36455392 |
| 20574 | Stil | NM_009185.3 | chr4:115000117-115043198 |
| 20575 | Stim1 | NM_009287.4 | chr7:102267823-102436855 |
| 20576 | Stim2 | NM_001081103.2 | chr5:53998522-54121057 |
| 20577 | Stip1 | NM_016737.2 | chr19:7020695-7040026 |
| 20578 | Stk10 | NM_009288.2 | chr11:32533265-32624595 |
| 20579 | Stk11 | NM_011492.4 | chr10:80115802-80130685 |
| 20580 | Stk11ip | NM_027886.3 | chr1:75521528-75537335 |
| 20581 | Stk16 | NM_001277992.1 | chr1:75210828-75215606 |
| 20582 | Stk17b | NM_133810.3 | chr1:53755511-53785215 |
| 20583 | Stk19 | NM_019442.3 | chr17:34823992-34836903 |
| 20584 | Stk24 | NM_145465.2 | chr14:121286340-121379230 |
| 20585 | Stk25 | NM_021537.3 | chr1:93620750-93635727 |
| 20586 | Stk3 | NM_019635.2 | chr15:34875498-35155806 |
| 20587 | Stk31 | NM_029916.2 | chr6:49395603-49469502 |
| 20588 | Stk32a | NM_178749.3 | chr18:43207696-43317481 |
| 20589 | Stk32b | NM_022416.2 | chr5:37446824-37717153 |
| 20590 | Stk32c | NM_001162540.1 | chr7:139103637-139213307 |
| 20591 | Stk33 | NM_054103.1 | chr7:109279215-109439053 |
| 20592 | Stk35 | NM_001038635.2 | chr2:129800516-129832285 |
| 20593 | Stk36 | NM_175031.3 | chr1:74601454-74636893 |
| 20594 | Stk38 | NM_134115.2 | chr17:28970884-29007937 |
| 20595 | Stk38l | NM_172734.3 | chr6:146724929-146778814 |
| 20596 | Stk39 | NM_016866.2 | chr2:68210446-68471981 |
| 20597 | Stk4 | NM_021420.3 | chr2:164074177-164155521 |
| 20598 | Stk40 | NM_001145827.1 | chr4:126103956-126141029 |
| 20599 | Stmn1 | NM_019641.4 | chr4:134468319-134473843 |
| 20600 | Stmn1-rs1 | NR_029430.1 | chr9:115281091-115282070 |
| 20601 | Stmn2 | NM_025285.2 | chr3:8509526-8561604 |
| 20602 | Stmn3 | NM_009133.3 | chr2:181306458-181314500 |
| 20603 | Stmn4 | NM_019675.3 | chr14:66344295-66361680 |
| 20604 | Stmnd1 | NM_001005422.1 | chr13:46273720-46300115 |
| 20605 | Stom | NM_013515.2 | chr2:35313989-35337009 |
| 20606 | Stoml1 | NM_026942.3 | chr9:58253163-58262524 |
| 20607 | Stoml2 | NM_023231.2 | chr4:43027689-43031384 |
| 20608 | Stoml3 | NM_153156.1 | chr3:53488792-53507652 |
| 20609 | Ston1 | NM_029858.2 | chr17:88626554-88645724 |
| 20610 | Ston2 | NM_175367.6 | chr12:91633008-91786436 |
| 20611 | Stox1 | NM_001033260.1 | chr10:62659421-62726099 |
| 20612 | Stox2 | NM_001114311.1 | chr8:47180047-47289402 |
| 20613 | Stpg1 | NM_030189.3 | chr4:135495986-135537803 |
| 20614 | Stpg2 | NM_198659.2 | chr3:139205892-139710299 |
| 20615 | Stra13 | NM_016665.2 | chr11:120710940-120713767 |
| 20616 | Stra6 | NM_001162475.1 | chr9:58129087-58153997 |
| 20617 | Stra8 | NM_009292.1 | chr6:34920959-34939342 |
| 20618 | Strada | NM_001252448.1 | chr11:106162973-106193603 |
| 20619 | Stradb | NM_172656.5 | chr1:58973570-58995121 |
| 20620 | Strap | NM_011499.3 | chr6:137735081-137751930 |
| 20621 | Strbp | NM_009261.3 | chr2:37569867-37647285 |
| 20622 | Strc | NM_080459.2 | chr2:121363726-121380940 |
| 20623 | Strip1 | NM_153563.2 | chr3:107612531-107631710 |
| 20624 | Strip2 | NM_001037740.1 | chr6:29917012-29959680 |
| 20625 | Strn | NM_011500.2 | chr17:78653963-78736560 |
| 20626 | Strn3 | NM_001172098.1 | chr12:51608540-51691914 |
| 20627 | Strn4 | NM_001039878.2 | chr7:16815888-16840931 |
| 20628 | Stt3a | NM_008408.4 | chr9:36732412-36767578 |
| 20629 | Stt3b | NM_024222.2 | chr9:115242581-115310421 |
| 20630 | Stub1 | NM_019719.3 | chr17:25830635-25833361 |
| 20631 | Stx11 | NM_001163590.1 | chr10:12939982-12964259 |
| 20632 | Stx12 | NM_133887.4 | chr4:132854063-132884458 |
| 20633 | Stx16 | NM_001102423.1 | chr2:174077050-174099771 |
| 20634 | Stx17 | NM_026343.2 | chr4:48124918-48186506 |
| 20635 | Stx18 | NM_001289535.1 | chr5:38039229-38137769 |
| 20636 | Stx19 | NM_026588.1 | chr16:62814675-62822722 |
| 20637 | Stx1a | NM_016801.3 | chr5:135023571-135051099 |
| 20638 | Stx1b | NM_024414.2 | chr7:127806843-127824531 |
| 20639 | Stx2 | NM_001286033.1 | chr5:128984557-129008572 |
| 20640 | Stx3 | NM_001025307.1 | chr19:11775117-11819403 |
| 20641 | Stx4a | NM_009294.3 | chr7:127841807-127848965 |
| 20642 | Stx5a | NM_001167799.1 | chr19:8741423-8755642 |
| 20643 | Stx6 | NM_021433.3 | chr1:155158702-155203517 |
| 20644 | Stx7 | NM_016797.4 | chr10:24149316-24188959 |
| 20645 | Stx8 | NM_018768.2 | chr11:67966482-68207148 |
| 20646 | Stxbp1 | NM_001113569.1 | chr2:32787606-32847237 |
| 20647 | Stxbp2 | NM_011503.4 | chr8:3631159-3643644 |
| 20648 | Stxbp3a | NM_011504.1 | chr3:108793179-108840502 |
| 20649 | Stxbp3b | NR_073559.1 | chr19:9557605-9559248 |
| 20650 | Stxbp4 | NM_011505.2 | chr11:90476492-90638108 |
| 20651 | Stxbp5 | NM_001081344.2 | chr10:9755546-9901040 |
| 20652 | Stxbp5l | NM_001114611.1 | chr16:37107309-37384958 |
| 20653 | Stxbp6 | NM_144552.3 | chr12:44852485-45074483 |
| 20654 | Styk1 | NM_172891.2 | chr6:131299143-131313827 |
| 20655 | Styx | NM_019637.3 | chr14:45351185-45376884 |
| 20656 | Styxl1 | NM_001289554.1 | chr5:135747219-135778301 |
| 20657 | Sub1 | NM_011294.3 | chr15:11981338-11996007 |
| 20658 | Sucla2 | NM_011506.3 | chr14:73552785-73596142 |
| 20659 | Suclg1 | NM_019879.3 | chr6:73248504-73276907 |
| 20660 | Suclg2 | NM_011507.3 | chr6:95473008-95718846 |
| 20661 | Sucnr1 | NM_032400.2 | chr3:60081868-60087566 |
| 20662 | Suco | NM_172645.2 | chr1:161816111-161876661 |
| 20663 | Suds3 | NM_001122666.2 | chr5:117091677-117115993 |
| 20664 | Sufu | NM_001025391.2 | chr19:46396895-46488804 |
| 20665 | Sugct | NM_138654.3 | chr13:16857474-17694765 |
| 20666 | Sugp1 | NM_027481.2 | chr8:70042812-70071953 |
| 20667 | Sugp2 | NM_001168290.1 | chr8:70234225-70261044 |
| 20668 | Sugt1 | NM_026474.5 | chr14:79587690-79629755 |
| 20669 | Sulf1 | NM_001198565.1 | chr1:12692429-12860372 |
| 20670 | Sulf2 | NM_001252578.1 | chr2:166073898-166155285 |
| 20671 | Sult1a1 | NM_133670.1 | chr7:126672869-126676357 |
| 20672 | Sult1b1 | NM_019878.4 | chr5:87513338-87538195 |
| 20673 | Sult1c1 | NM_018751.2 | chr17:53961614-53990631 |
| 20674 | Sult1c2 | NM_026935.4 | chr17:53829636-53845958 |
| 20675 | Sult1d1 | NM_016771.3 | chr5:87554649-87569006 |
| 20676 | Sult1e1 | NM_023135.2 | chr5:87575967-87591611 |
| 20677 | Sult2a1 | NM_001111296.2 | chr7:13796245-13837410 |
| 20678 | Sult2a2 | NM_009286.2 | chr7:13733505-13779637 |
| 20679 | Sult2a3 | NM_001101586.2 | chr7:14067554-14122993 |
| 20680 | Sult2a4 | NM_001101534.1 | chr7:13909676-13989588 |
| 20681 | Sult2a5 | NM_001184980.1 | chr7:13623966-13670807 |
| 20682 | Sult2a6 | NM_001081325.2 | chr7:14222402-14254870 |
| 20683 | Sult2a7 | NM_001184981.1 | chr7:14465158-14492926 |
| 20684 | Sult2b1 | NM_017465.2 | chr7:45729982-45759555 |
| 20685 | Sult3a1 | NM_020565.2 | chr10:33857721-33879475 |
| 20686 | Sult4a1 | NM_013873.3 | chr15:84076096-84105754 |
| 20687 | Sult5a1 | NM_020564.3 | chr8:123142846-123158280 |
| 20688 | Sult6b1 | NM_001163625.1 | chr17:78883937-78906992 |
| 20689 | Sumf1 | NM_145937.3 | chr6:108107020-108185583 |
| 20690 | Sumf2 | NM_026445.2 | chr5:129846989-129863951 |
| 20691 | Sumo1 | NM_009460.2 | chr1:59639433-59670834 |
| 20692 | Sumo2 | NM_133354.2 | chr11:115523108-115536230 |
| 20693 | Sumo3 | NM_019929.4 | chr10:77606096-77618331 |
| 20694 | Sun1 | NM_001256115.1 | chr5:139200636-139249839 |
| 20695 | Sun2 | NM_001205345.1 | chr15:79724067-79742536 |
| 20696 | Sun3 | NM_001290519.1 | chr11:9016053-9039591 |
| 20697 | Sun5 | NM_029599.2 | chr2:153856187-153871084 |
| 20698 | Suox | NM_173733.3 | chr10:128669886-128673918 |
| 20699 | Supt16 | NM_033618.3 | chr14:52160418-52197239 |
| 20700 | Supt20 | NM_019995.3 | chr3:54693104-54716837 |
| 20701 | Supt3 | NM_178652.2 | chr17:44777170-45119284 |
| 20702 | Supt4a | NM_009296.1 | chr11:87737564-87743617 |
| 20703 | Supt5 | NM_013676.1 | chr7:28314895-28338719 |
| 20704 | Supt6 | NM_009297.2 | chr11:78206748-78245703 |
| 20705 | Supt7l | NM_028150.2 | chr5:31514568-31526762 |
| 20706 | Supv3l1 | NM_181423.2 | chr10:62429377-62449693 |
| 20707 | Surf1 | NM_001271724.1 | chr2:26913377-26916530 |
| 20708 | Surf2 | NM_013678.2 | chr2:26916420-26920170 |
| 20709 | Surf4 | NM_011512.3 | chr2:26920040-26933511 |
| 20710 | Surf6 | NM_009298.3 | chr2:26890771-26902813 |
| 20711 | Susd1 | NM_001163288.2 | chr4:59314682-59438633 |
| 20712 | Susd2 | NM_001162913.1 | chr10:75636618-75644008 |
| 20713 | Susd3 | NM_025491.3 | chr13:49230832-49248163 |
| 20714 | Susd4 | NM_144796.4 | chr1:182764905-182895654 |
| 20715 | Susd5 | NM_001101510.1 | chr9:114057353-114098733 |
| 20716 | Suv39h1 | NM_001290716.1 | chrX:8061170-8074313 |
| 20717 | Suv39h2 | NM_022724.4 | chr2:3455814-3474986 |
| 20718 | Suv420h1 | NM_001167884.1 | chr19:3767420-3806324 |
| 20719 | Suv420h2 | NM_001115018.1 | chr7:4740126-4747514 |
| 20720 | Suz12 | NM_001163018.1 | chr11:79993105-80034123 |
| 20721 | Sv2a | NM_022030.3 | chr3:96181226-96195180 |
| 20722 | Sv2b | NM_001109753.1 | chr7:75114894-75308386 |
| 20723 | Sv2c | NM_029210.1 | chr13:95959442-96132577 |
| 20724 | Sva | NM_009299.2 | chr6:42038393-42042851 |
| 20725 | Sval1 | NM_027832.3 | chr6:41951627-41956098 |
| 20726 | Sval2 | NM_032542.1 | chr6:41860338-41864322 |
| 20727 | Sval3 | NM_001003952.1 | chr6:41968139-41973090 |
| 20728 | Svep1 | NM_022814.2 | chr4:58042795-58206596 |
| 20729 | Svil | NM_153153.3 | chr18:5046588-5119293 |
| 20730 | Svip | NM_001160345.1 | chr7:51997160-52006018 |
| 20731 | Svop | NM_026805.1 | chr5:114026912-114091380 |
| 20732 | Svopl | NM_177200.4 | chr6:37983738-38046996 |
| 20733 | Svs1 | NM_172888.3 | chr6:48986860-48991724 |
| 20734 | Svs2 | NM_017390.4 | chr2:164235928-164238341 |
| 20735 | Svs3a | NM_021363.2 | chr2:164289267-164291500 |
| 20736 | Svs3b | NM_173377.2 | chr2:164254362-164256643 |
| 20737 | Svs4 | NM_009300.3 | chr2:164275955-164278307 |
| 20738 | Svs5 | NM_009301.2 | chr2:164332764-164334394 |
| 20739 | Svs6 | NM_013679.2 | chr2:164316750-164318450 |
| 20740 | Swap70 | NM_009302.3 | chr7:110221702-110283506 |
| 20741 | Swi5 | NM_001290552.1 | chr2:32278804-32288069 |
| 20742 | Swsap1 | NM_025870.1 | chr9:21955752-21958270 |
| 20743 | Swt1 | NM_025819.4 | chr1:151367698-151428435 |
| 20744 | Syap1 | NM_025932.2 | chrX:162856842-162888462 |
| 20745 | Sybu | NM_001032727.1 | chr15:44671855-44788063 |
| 20746 | Syce1 | NM_001143765.1 | chr7:140777228-140787854 |
| 20747 | Syce1l | NM_001048145.1 | chr8:113643212-113655533 |
| 20748 | Syce2 | NM_001168244.1 | chr8:84872257-84887446 |
| 20749 | Syce3 | NM_001162880.1 | chr15:89390173-89410503 |
| 20750 | Sycn | NM_026716.3 | chr7:28540884-28542210 |
| 20751 | Sycp1 | NM_011516.2 | chr3:102818498-102936100 |
| 20752 | Sycp1-ps1 | NR_024208.1 | chr7:18786301-18789414 |
| 20753 | Sycp2 | NM_177191.3 | chr2:178345295-178407658 |
| 20754 | Sycp3 | NM_011517.2 | chr10:88459586-88473236 |
| 20755 | Syde1 | NM_027875.1 | chr10:78584502-78591964 |
| 20756 | Syde2 | NM_001166064.1 | chr3:145987869-146021720 |
| 20757 | Syf2 | NM_026780.3 | chr4:134930979-134937537 |

Fig.21 - 108

| | | | | | | | |
|---|---|---|---|---|---|---|---|
| 20758 | Syk | NM_001198977.1 | chr13:52583436-52648792 | 20855 | Taf13 | NM_025444.2 | chr3:108571698-108582068 |
| 20759 | Sympk | NM_026605.2 | chr7:19024376-19054622 | 20856 | Taf15 | NM_027427.2 | chr11:83473107-83506740 |
| 20760 | Syn1 | NM_001110780.1 | chrX:20860510-20920918 | 20857 | Taf1a | NM_001277957.1 | chr1:183388884-183410206 |
| 20761 | Syn2 | NM_001111015.1 | chr6:115134901-115282626 | 20858 | Taf1b | NM_020614.2 | chr12:24498580-24558571 |
| 20762 | Syn3 | NM_001164495.1 | chr10:86291592-86498896 | 20859 | Taf1c | NM_021441.2 | chr8:119597973-119605240 |
| 20763 | Syna | NM_001013751.2 | chr5:134557253-134560171 | 20860 | Taf1d | NM_027261.3 | chr9:15306213-15312105 |
| 20764 | Synb | NM_173420.3 | chr14:69290397-69512549 | 20861 | Taf2 | NM_001081288.1 | chr15:55015128-55072152 |
| 20765 | Sync | NM_023485.3 | chr4:129287620-129308559 | 20862 | Taf3 | NM_027748.3 | chr2:9914551-10048609 |
| 20766 | Syncrip | NM_001284328.1 | chr9:88449363-88482397 | 20863 | Taf4a | NM_001081092.1 | chr2:179912145-179976646 |
| 20767 | Syndig1 | NM_001085521.2 | chr2:149830782-150004392 | 20864 | Taf4b | NM_001100449.1 | chr18:14783244-14900359 |
| 20768 | Syndig1l | NM_001033334.2 | chr12:84677277-84698807 | 20865 | Taf5 | NM_177342.3 | chr19:47067747-47083479 |
| 20769 | Syne1 | NM_001079686.1 | chr10:5020195-5194707 | 20866 | Taf5l | NM_133966.2 | chr8:123996312-124021309 |
| 20770 | Syne2 | NM_001005510.2 | chr12:75818317-76110928 | 20867 | Taf6 | NM_009315.3 | chr5:138178616-138187186 |
| 20771 | Syne3 | NM_001042699.2 | chr12:104929932-104998677 | 20868 | Taf6l | NM_001177798.1 | chr19:8774353-8786417 |
| 20772 | Syne4 | NM_001290565.1 | chr7:30314815-30319045 | 20869 | Taf7 | NM_175770.4 | chr18:37640490-37644204 |
| 20773 | Syngap1 | NM_001281491.1 | chr17:26941451-26970645 | 20870 | Taf7l | NM_028958.4 | chrX:134460115-134476490 |
| 20774 | Syngr1 | NM_009303.2 | chr15:80091333-80113440 | 20871 | Taf8 | NM_022015.3 | chr17:47488049-47502287 |
| 20775 | Syngr2 | NM_009304.2 | chr11:117809666-117814286 | 20872 | Taf9 | NM_001015889.2 | chr13:100651606-100656060 |
| 20776 | Syngr3 | NM_011522.3 | chr17:24685091-24689949 | 20873 | Taf9b | NM_001001176.2 | chrX:106206873-106219842 |
| 20777 | Syngr4 | NM_001291064.1 | chr7:45886844-45896711 | 20874 | Tagap | NM_145968.2 | chr17:7925999-7934897 |
| 20778 | Synj1 | NM_001045515.1 | chr16:90936100-91011095 | 20875 | Tagap1 | NM_147155.2 | chr17:6954964-6961156 |
| 20779 | Synj2 | NM_001113351.1 | chr17:5975585-6044290 | 20876 | Tagln | NM_011526.5 | chr9:45929627-45936058 |
| 20780 | Synj2bp | NM_025292.7 | chr12:81492193-81532911 | 20877 | Tagln2 | NM_178598.2 | chr1:172500245-172507375 |
| 20781 | Synm | NM_183312.3 | chr7:67730160-67759742 | 20878 | Tagln3 | NM_019754.3 | chr16:45711229-45724531 |
| 20782 | Synpo | NM_001109975.1 | chr18:60600205-60610104 | 20879 | Tal1 | NM_001287388.1 | chr4:115057810-115071755 |
| 20783 | Synpo2 | NM_080451.2 | chr3:123076518-123236149 | 20880 | Tal2 | NM_009317.3 | chr4:53779704-53786885 |
| 20784 | Synpo2l | NM_175132.4 | chr14:20658947-20668354 | 20881 | Taldo1 | NM_011528.4 | chr7:141392159-141402976 |
| 20785 | Synpr | NM_001163032.1 | chr14:13453957-13615469 | 20882 | Tamm41 | NM_026894.1 | chr6:115004380-115037874 |
| 20786 | Synrg | NM_194341.2 | chr11:83964430-84044576 | 20883 | Tanc1 | NM_001290659.1 | chr2:59646768-59846213 |
| 20787 | Syp | NM_009305.2 | chrX:7638579-7653256 | 20884 | Tanc2 | NM_181071.3 | chr11:105589985-105929303 |
| 20788 | Sypl | NM_013635.3 | chr12:32953944-32979502 | 20885 | Tango2 | NM_138583.2 | chr16:18300824-18343932 |
| 20789 | Sypl2 | NM_008596.1 | chr3:108212265-108226599 | 20886 | Tango6 | NM_173037.1 | chr8:106683067-106851439 |
| 20790 | Sys1 | NM_025575.3 | chr2:164460970-164465510 | 20887 | Tank | NM_001164071.1 | chr2:61593096-61654169 |
| 20791 | Syt1 | NM_001252341.1 | chr10:108497649-109010975 | 20888 | Taok1 | NM_144825.2 | chr11:77529161-77607815 |
| 20792 | Syt10 | NM_018803.2 | chr15:89782392-89841860 | 20889 | Taok2 | NM_001163774.1 | chr7:126865676-126884967 |
| 20793 | Syt11 | NM_018804.3 | chr3:88744700-88772599 | 20890 | Taok3 | NM_001081308.2 | chr5:117133587-117275098 |
| 20794 | Syt12 | NM_134164.5 | chr19:4445907-4477143 | 20891 | Tap1 | NM_001161730.1 | chr17:34187555-34197225 |
| 20795 | Syt13 | NM_030725.4 | chr2:92915100-92956051 | 20892 | Tap2 | NM_011530.3 | chr17:34204478-34216321 |
| 20796 | Syt14 | NM_181546.3 | chr1:192891233-193035775 | 20893 | Tapbp | NM_001025313.1 | chr17:33919477-33929290 |
| 20797 | Syt15 | NM_176931.2 | chr14:34220045-34227740 | 20894 | Tapbpl | NM_145391.2 | chr6:125224211-125231860 |
| 20798 | Syt16 | NM_172804.2 | chr12:73997760-74267916 | 20895 | Tapt1 | NM_173764.3 | chr5:44175161-44226606 |
| 20799 | Syt17 | NM_138649.1 | chr7:118381855-118443552 | 20896 | Tarbp2 | NM_001253795.1 | chr15:102518191-102523676 |
| 20800 | Syt2 | NM_009307.3 | chr1:134646680-134749417 | 20897 | Tardbp | NM_001003898.3 | chr4:148612381-148626996 |
| 20801 | Syt3 | NM_001114116.1 | chr7:44384125-44400030 | 20898 | Tarm1 | NM_177363.3 | chr7:3489075-3502552 |
| 20802 | Syt4 | NM_009308.3 | chr18:31437807-31447415 | 20899 | Tars | NM_033074.3 | chr15:11383662-11399658 |
| 20803 | Syt5 | NM_016908.2 | chr7:4539764-4546567 | 20900 | Tars2 | NM_001163617.1 | chr3:95739973-95754977 |
| 20804 | Syt6 | NM_001276676.1 | chr3:103575281-103635179 | 20901 | Tarsl2 | NM_172310.2 | chr7:65644897-65692093 |
| 20805 | Syt7 | NM_018801.3 | chr19:10389089-10453181 | 20902 | Tas1r1 | NM_031867.2 | chr4:152027913-152038490 |
| 20806 | Syt8 | NM_001285857.1 | chr7:142434976-142440396 | 20903 | Tas1r2 | NM_031873.1 | chr4:139653537-139670279 |
| 20807 | Syt9 | NM_021889.4 | chr7:107370789-107548655 | 20904 | Tas1r3 | NM_031872.2 | chr4:155859269-155863353 |
| 20808 | Sytl1 | NM_031393.2 | chr4:133253089-133263087 | 20905 | Tas2r102 | NM_199153.2 | chr6:132762130-132763174 |
| 20809 | Sytl2 | NM_001040085.2 | chr7:90302354-90410719 | 20906 | Tas2r103 | NM_053211.1 | chr6:133036162-133037101 |
| 20810 | Sytl3 | NM_031395.2 | chr17:6673609-6738044 | 20907 | Tas2r104 | NM_207011.1 | chr6:131684835-131685744 |
| 20811 | Sytl4 | NM_001290717.1 | chrX:133936384-133981812 | 20908 | Tas2r105 | NM_020501.1 | chr6:131686560-131687463 |
| 20812 | Sytl5 | NM_001290728.1 | chrX:9885620-9998864 | 20909 | Tas2r106 | NM_207016.1 | chr6:131677959-131678886 |
| 20813 | Syvn1 | NM_001164709.1 | chr19:6047144-6053718 | 20910 | Tas2r107 | NM_199154.1 | chr6:131659157-131660084 |
| 20814 | Szrd1 | NM_001025608.2 | chr4:141112977-141139796 | 20911 | Tas2r108 | NM_020502.1 | chr6:40493591-40494485 |
| 20815 | Szt2 | NM_198170.4 | chr4:118362739-118409263 | 20912 | Tas2r109 | NM_207017.1 | chr6:132980014-132980965 |
| 20816 | T | NM_009309.2 | chr17:8434422-8442496 | 20913 | Tas2r110 | NM_199155.2 | chr6:132868007-132869009 |
| 20817 | T2 | NM_001161832.1 | chr17:8372395-8422726 | 20914 | Tas2r113 | NM_207018.1 | chr6:132893010-132893940 |
| 20818 | Taar1 | NM_053205.1 | chr10:23920405-23921404 | 20915 | Tas2r114 | NM_207019.1 | chr6:131689133-131690063 |
| 20819 | Taar2 | NM_001007266.1 | chr10:23938571-23941583 | 20916 | Tas2r115 | NM_207020.1 | chr6:132737053-132737986 |
| 20820 | Taar3 | NM_001008429.1 | chr10:23949557-23950589 | 20917 | Tas2r116 | NM_053212.1 | chr6:132855437-132856355 |
| 20821 | Taar4 | NM_001008499.1 | chr10:23960493-23961537 | 20918 | Tas2r117 | NM_207021.1 | chr6:132802900-132803893 |
| 20822 | Taar5 | NM_001009574.1 | chr10:23970705-23971719 | 20919 | Tas2r118 | NM_207022.1 | chr6:23969160-23970060 |
| 20823 | Taar6 | NM_001010828.1 | chr10:23984608-23985646 | 20920 | Tas2r119 | NM_020503.2 | chr15:32177288-32178294 |
| 20824 | Taar7a | NM_001010829.1 | chr10:23992404-23993481 | 20921 | Tas2r120 | NM_207023.1 | chr6:132656956-132657844 |
| 20825 | Taar7b | NM_001010827.1 | chr10:23999938-24001015 | 20922 | Tas2r121 | NM_207024.1 | chr6:132700089-132701007 |
| 20826 | Taar7d | NM_001010838.1 | chr10:24027221-24028298 | 20923 | Tas2r122 | NM_001039128.1 | chr6:132710998-132711928 |
| 20827 | Taar7e | NM_001010835.1 | chr10:24037613-24038690 | 20924 | Tas2r123 | NM_207025.1 | chr6:132847141-132848143 |
| 20828 | Taar7f | NM_001010839.1 | chr10:24049509-24050586 | 20925 | Tas2r124 | NM_207026.1 | chr6:132754729-132755659 |
| 20829 | Taar8a | NM_001010830.1 | chr10:24076499-24077534 | 20926 | Tas2r125 | NM_207027.1 | chr6:132909650-132910586 |
| 20830 | Taar8b | NM_001010837.1 | chr10:24091259-24092294 | 20927 | Tas2r126 | NM_207028.1 | chr6:42434534-42435461 |
| 20831 | Taar8c | NM_001010840.2 | chr10:24100842-24101951 | 20928 | Tas2r129 | NM_207029.1 | chr6:132951101-132952064 |
| 20832 | Taar9 | NM_001010831.1 | chr10:24108487-24109534 | 20929 | Tas2r130 | NM_199156.1 | chr6:131629891-131630830 |
| 20833 | Tab1 | NM_025609.2 | chr15:80133153-80161702 | 20930 | Tas2r131 | NM_207030.1 | chr6:132956911-132957844 |
| 20834 | Tab2 | NM_138667.3 | chr10:7905647-7956123 | 20931 | Tas2r134 | NM_199158.1 | chr2:51627510-51628407 |
| 20835 | Tab3 | NM_025729.4 | chrX:85574021-85634469 | 20932 | Tas2r135 | NM_199159.1 | chr6:42405528-42406494 |
| 20836 | Tac1 | NM_009311.2 | chr6:7555070-7562973 | 20933 | Tas2r136 | NM_181276.1 | chr6:132777178-132778162 |
| 20837 | Tac2 | NM_001199971.1 | chr10:127724477-127731768 | 20934 | Tas2r137 | NM_001025385.1 | chr6:40491237-40492239 |
| 20838 | Tac4 | NM_053093.1 | chr11:95261528-95269261 | 20935 | Tas2r138 | NM_001001451.1 | chr6:40612314-40613310 |
| 20839 | Tacc1 | NM_177089.5 | chr8:25154551-25201542 | 20936 | Tas2r139 | NM_181275.1 | chr6:42140935-42141895 |
| 20840 | Tacc2 | NM_001004468.2 | chr7:130577483-130764782 | 20937 | Tas2r140 | NM_021562.1 | chr6:133054854-133055793 |
| 20841 | Tacc3 | NM_001040435.3 | chr5:33658127-33672202 | 20938 | Tas2r143 | NM_001001452.1 | chr6:42400237-42401119 |
| 20842 | Taco1 | NM_027346.1 | chr11:106066106-106073612 | 20939 | Tas2r144 | NM_001001453.1 | chr6:42215327-42216287 |
| 20843 | Tacr1 | NM_009313.5 | chr6:82402474-82560104 | 20940 | Tasp1 | NM_001159640.2 | chr2:139833478-140066805 |
| 20844 | Tacr2 | NM_009314.4 | chr10:62252437-62265990 | 20941 | Tat | NM_146214.3 | chr8:109990435-109999804 |
| 20845 | Tacr3 | NM_021382.6 | chr3:134829006-134934581 | 20942 | Tatdn1 | NM_175151.4 | chr15:58890152-58933730 |
| 20846 | Tacstd2 | NM_020047.3 | chr6:67534058-67535822 | 20943 | Tatdn2 | NM_001033463.3 | chr6:113697498-113711068 |
| 20847 | Tada1 | NM_030245.3 | chr1:166379166-166393620 | 20944 | Tatdn3 | NM_001163421.1 | chr1:191045829-191062932 |
| 20848 | Tada2a | NM_172562.3 | chr11:84078919-84129568 | 20945 | Tax1bp1 | NM_025816.3 | chr6:52713728-52766779 |
| 20849 | Tada2b | NM_001170454.1 | chr5:36473669-36484285 | 20946 | Tax1bp3 | NM_029564.2 | chr11:73177082-73182046 |
| 20850 | Tada3 | NM_133932.2 | chr6:113366656-113377520 | 20947 | Taz | NM_001173547.2 | chrX:74282717-74290151 |
| 20851 | Taf1 | NM_001290729.1 | chrX:101527574-101601789 | 20948 | Tbata | NM_001017407.1 | chr10:61175261-61180680 |
| 20852 | Taf10 | NM_020024.3 | chr7:105742893-105744338 | 20949 | Tbc1d1 | NM_001289514.1 | chr5:64230356-64351486 |
| 20853 | Taf11 | NM_026836.2 | chr17:27901127-27907724 | 20950 | Tbc1d10a | NM_134023.1 | chr11:4186832-4215505 |
| 20854 | Taf12 | NM_025579.3 | chr4:132274374-132293330 | 20951 | Tbc1d10b | NM_144522.5 | chr7:127197458-127208468 |

Fig.21 - 109

| | | | |
|---|---|---|---|
| 20952 | Tbc1d10c | NM_178650.3 | chr19:4184356-4191047 |
| 20953 | Tbc1d12 | NM_145952.3 | chr19:38836578-38919923 |
| 20954 | Tbc1d13 | NM_146252.2 | chr2:30133870-30152013 |
| 20955 | Tbc1d14 | NM_001113362.1 | chr5:36490603-36586226 |
| 20956 | Tbc1d15 | NM_025706.3 | chr10:115197870-115251493 |
| 20957 | Tbc1d16 | NM_172443.3 | chr11:119143042-119228499 |
| 20958 | Tbc1d17 | NM_001042655.1 | chr7:44840775-44849079 |
| 20959 | Tbc1d19 | NM_144517.4 | chr5:53809626-53904380 |
| 20960 | Tbc1d2 | NM_198664.3 | chr4:46604389-46650199 |
| 20961 | Tbc1d20 | NM_024196.3 | chr2:152293871-152312590 |
| 20962 | Tbc1d21 | NM_028854.3 | chr9:58359803-58370369 |
| 20963 | Tbc1d22a | NM_145476.2 | chr15:86214458-86498503 |
| 20964 | Tbc1d22b | NM_198647.1 | chr17:29549801-29606808 |
| 20965 | Tbc1d22bos | NR_045447.1 | chr17:29578815-29596305 |
| 20966 | Tbc1d23 | NM_026254.2 | chr16:57168863-57231466 |
| 20967 | Tbc1d24 | NM_001163847.1 | chr17:24175430-24205562 |
| 20968 | Tbc1d25 | NM_001166437.1 | chrX:8154471-8176181 |
| 20969 | Tbc1d2b | NM_194334.2 | chr9:90202048-90270769 |
| 20970 | Tbc1d30 | NM_029057.1 | chr10:121263819-121311189 |
| 20971 | Tbc1d31 | NM_001081396.2 | chr15:57912198-57970068 |
| 20972 | Tbc1d32 | NM_001033385.3 | chr10:56014293-56228689 |
| 20973 | Tbc1d4 | NM_001081278.2 | chr14:101442359-101609191 |
| 20974 | Tbc1d5 | NM_001285991.1 | chr17:50733126-51179352 |
| 20975 | Tbc1d7 | NM_001252639.1 | chr13:43151741-43171501 |
| 20976 | Tbc1d8 | NM_018775.4 | chr1:39371495-39478747 |
| 20977 | Tbc1d8b | NM_001081499.2 | chrX:139684995-139754218 |
| 20978 | Tbc1d9 | NM_001111304.1 | chr8:83165351-83272940 |
| 20979 | Tbc1d9b | NM_001290759.1 | chr11:50131359-50172785 |
| 20980 | Tbca | NM_009321.2 | chr13:94788942-94842899 |
| 20981 | Tbcb | NM_025548.3 | chr7:30224130-30232029 |
| 20982 | Tbcc | NM_178385.3 | chr17:46890620-46892463 |
| 20983 | Tbccd1 | NM_001081368.2 | chr16:22813214-22857569 |
| 20984 | Tbcd | NM_029878.1 | chr11:121451948-121617170 |
| 20985 | Tbce | NM_178337.3 | chr13:13997950-14039638 |
| 20986 | Tbcel | NM_173038.3 | chr9:42412316-42472226 |
| 20987 | Tbck | NM_001163455.2 | chr3:132684143-132841688 |
| 20988 | Tbk1 | NM_019786.4 | chr10:121546455-121586794 |
| 20989 | Tbkbp1 | NM_198100.2 | chr11:97136170-97149712 |
| 20990 | Tbl1x | NM_020601.2 | chrX:77511226-77660265 |
| 20991 | Tbl1xr1 | NM_030732.3 | chr3:22076651-22216594 |
| 20992 | Tbl2 | NM_013763.2 | chr5:135149710-135162662 |
| 20993 | Tbl3 | NM_145396.4 | chr17:24700652-24707653 |
| 20994 | Tbp | NM_013684.3 | chr17:15499887-15517427 |
| 20995 | Tbpl1 | NM_011603.5 | chr10:22703876-22731447 |
| 20996 | Tbpl2 | NM_001289689.1 | chr2:24071365-24096595 |
| 20997 | Tbr1 | NM_009322.3 | chr2:61804452-61814113 |
| 20998 | Tbrg1 | NM_025289.3 | chr9:37649181-37657312 |
| 20999 | Tbrg3 | NR_027799.1 | chr15:82890482-82898461 |
| 21000 | Tbrg4 | NM_001130457.1 | chr11:6615597-6626067 |
| 21001 | Tbx1 | NM_001285472.1 | chr16:18581703-18590671 |
| 21002 | Tbx10 | NM_001001320.1 | chr19:3992751-3999512 |
| 21003 | Tbx15 | NM_009323.2 | chr3:99253759-99354260 |
| 21004 | Tbx18 | NM_023814.4 | chr9:87702799-87731260 |
| 21005 | Tbx19 | NM_032005.4 | chr1:165137852-165160773 |
| 21006 | Tbx2 | NM_009324.2 | chr11:85832614-85841948 |
| 21007 | Tbx20 | NM_001205085.1 | chr9:24740247-24774303 |
| 21008 | Tbx21 | NM_019507.2 | chr11:97098006-97115331 |
| 21009 | Tbx22 | NM_001290747.1 | chrX:107679014-107688980 |
| 21010 | Tbx3 | NM_011535.3 | chr5:119671232-119684724 |
| 21011 | Tbx3os2 | NR_040416.1 | chr5:119685087-119691218 |
| 21012 | Tbx4 | NM_011536.2 | chr11:85890062-85916097 |
| 21013 | Tbx5 | NM_011537.3 | chr5:119834662-119885218 |
| 21014 | Tbx6 | NM_011538.2 | chr7:126781482-126785548 |
| 21015 | Tbxa2r | NM_001277265.1 | chr10:81328702-81335174 |
| 21016 | Tbxas1 | NM_011539.3 | chr6:38918985-39084579 |
| 21017 | Tc2n | NM_001082976.2 | chr12:101645445-101718523 |
| 21018 | Tcaim | NM_001013405.2 | chr9:122805546-122836332 |
| 21019 | Tcam1 | NM_029467.3 | chr11:106276671-106288143 |
| 21020 | Tcap | NM_011540.2 | chr11:98383810-98384953 |
| 21021 | Tcea1 | NM_001159750.1 | chr1:4857693-4897909 |
| 21022 | Tcea2 | NM_009326.2 | chr2:181680309-181688051 |
| 21023 | Tcea3 | NM_011542.2 | chr4:136247956-136274899 |
| 21024 | Tceal1 | NM_146236.1 | chrX:136708064-136709866 |
| 21025 | Tceal3 | NM_001029978.2 | chrX:136666374-136668377 |
| 21026 | Tceal5 | NM_177919.2 | chrX:136200950-136203851 |
| 21027 | Tceal6 | NM_025355.4 | chrX:135208685-135210687 |
| 21028 | Tceal7 | NM_001127169.1 | chrX:136224040-136226100 |
| 21029 | Tceal8 | NM_001168578.1 | chrX:136168983-136172251 |
| 21030 | Tceanc | NM_001007577.2 | chrX:166499814-166510478 |
| 21031 | Tceanc2 | NM_025617.2 | chr4:107134161-107178366 |
| 21032 | Tceb1 | NM_026456.3 | chr1:16642764-16656865 |
| 21033 | Tceb2 | NM_026305.2 | chr17:23824739-23829109 |
| 21034 | Tceb3 | NM_013736.4 | chr4:136003369-136021649 |
| 21035 | Tcerg1 | NM_001039474.1 | chr18:42511486-42575785 |
| 21036 | Tcerg1l | NM_183289.3 | chr7:138208971-138397730 |
| 21037 | Tcf12 | NM_001253862.1 | chr9:71844251-72111201 |
| 21038 | Tcf15 | NM_009328.2 | chr2:152143608-152149096 |
| 21039 | Tcf19 | NM_001163763.1 | chr17:35512734-35516824 |
| 21040 | Tcf20 | NM_001114140.1 | chr15:82808625-82912134 |
| 21041 | Tcf21 | NM_011545.1 | chr10:22817274-22820128 |
| 21042 | Tcf23 | NM_053085.2 | chr5:30968676-30977018 |
| 21043 | Tcf24 | NM_001285425.1 | chr1:9960162-9967485 |
| 21044 | Tcf25 | NM_001037877.3 | chr8:123373710-123404174 |
| 21045 | Tcf3 | NM_001164147.1 | chr10:80409164-80433653 |
| 21046 | Tcf4 | NM_001083967.1 | chr18:69345720-69687967 |
| 21047 | Tcf7 | NM_009331.4 | chr11:52252282-52282595 |
| 21048 | Tcf7l1 | NM_001079822.2 | chr6:72626370-72789045 |
| 21049 | Tcf7l2 | NM_001142918.1 | chr19:55741809-55933655 |
| 21050 | Tcfl5 | NM_178254.3 | chr2:180621956-180642691 |
| 21051 | Tchh | NM_001163098.1 | chr3:93442329-93449077 |
| 21052 | Tchhl1 | NM_027762.3 | chr3:93468753-93471980 |
| 21053 | Tchp | NM_029992.2 | chr5:114707778-114722327 |
| 21054 | Tcirg1 | NM_001136091.2 | chr19:3896049-3907133 |
| 21055 | Tcl1 | NM_001289468.1 | chr12:105216754-105222737 |
| 21056 | Tcl1b1 | NM_013773.2 | chr12:105159703-105166625 |
| 21057 | Tcl1b2 | NM_013775.1 | chr12:105147032-105155225 |
| 21058 | Tcl1b3 | NM_013772.2 | chr12:105191044-105195399 |
| 21059 | Tcl1b4 | NM_013774.2 | chr12:105202421-105206993 |
| 21060 | Tcl1b5 | NM_013776.1 | chr12:105176357-105181145 |
| 21061 | Tcn2 | NM_001130458.1 | chr11:3917079-3932078 |
| 21062 | Tcof1 | NM_001198984.1 | chr18:60813755-60848964 |
| 21063 | Tcp1 | NM_001290712.1 | chr17:12916328-12925067 |
| 21064 | Tcp10a | NM_011553.4 | chr17:7324659-7345860 |
| 21065 | Tcp10b | NM_009341.2 | chr17:13061110-13082226 |
| 21066 | Tcp10c | NM_001167578.1 | chr17:13354571-13377223 |
| 21067 | Tcp11 | NM_001085555.1 | chr17:28066746-28080639 |
| 21068 | Tcp11l1 | NM_177190.5 | chr2:104679979-104712162 |
| 21069 | Tcp11l2 | NM_146008.2 | chr10:84576946-84614355 |
| 21070 | Tcstv1 | NM_018756.3 | chr13:119893386-119894785 |
| 21071 | Tcstv3 | NM_153523.3 | chr13:120316861-120318261 |
| 21072 | Tcta | NM_133986.2 | chr9:108302954-108306159 |
| 21073 | Tcte1 | NM_013688.2 | chr17:45523433-45542677 |
| 21074 | Tcte2 | NM_022311.2 | chr17:13716435-13761394 |
| 21075 | Tcte3 | NM_011560.3 | chr17:15027153-15041559 |
| 21076 | Tctex1d1 | NM_001163767.1 | chr4:102986378-103005594 |
| 21077 | Tctex1d2 | NM_025329.3 | chr16:32419701-32428892 |
| 21078 | Tctex1d4 | NM_175030.2 | chr4:117126812-117128730 |
| 21079 | Tctn1 | NM_001039153.3 | chr5:122239494-122264460 |
| 21080 | Tctn2 | NM_026486.3 | chr5:124598748-124627738 |
| 21081 | Tctn3 | NM_026260.2 | chr19:40596445-40612215 |
| 21082 | Tdg | NM_011561.2 | chr10:82629837-82650494 |
| 21083 | Tdgf1 | NM_011562.2 | chr9:110939607-110946158 |
| 21084 | Tdh | NM_021480.5 | chr14:63492346-63509092 |
| 21085 | Tdo2 | NM_019911.2 | chr3:81958413-81975728 |
| 21086 | Tdp1 | NM_028354.4 | chr12:99884514-99955216 |
| 21087 | Tdp2 | NM_019551.2 | chr13:24831658-24842153 |
| 21088 | Tdpoz1 | NM_148949.2 | chr3:93669332-93676283 |
| 21089 | Tdpoz2 | NM_001007222.3 | chr3:93651541-93652686 |
| 21090 | Tdpoz3 | NM_207271.2 | chr3:93826019-93827117 |
| 21091 | Tdpoz4 | NM_207272.2 | chr3:93796397-93797510 |
| 21092 | Tdpoz5 | NM_207273.2 | chr3:93960291-94072644 |
| 21093 | Tdrd1 | NM_001002238.2 | chr19:56826208-56870012 |
| 21094 | Tdrd12 | NM_028034.2 | chr7:35493609-35537744 |
| 21095 | Tdrd3 | NM_001253755.1 | chr14:87416582-87515192 |
| 21096 | Tdrd5 | NM_001134741.1 | chr1:156255295-156303348 |
| 21097 | Tdrd6 | NM_001161366.1 | chr17:43615334-43630299 |
| 21098 | Tdrd7 | NM_001290475.1 | chr4:45965334-46034765 |
| 21099 | Tdrd9 | NM_029056.1 | chr12:111971558-112068854 |
| 21100 | Tdrkh | NM_028307.1 | chr3:94413317-94431499 |
| 21101 | Tdrp | NM_173744.4 | chr8:13952007-13974777 |
| 21102 | Tead1 | NM_001166584.1 | chr7:112679319-112906805 |
| 21103 | Tead2 | NM_001285498.1 | chr7:45215752-45233619 |
| 21104 | Tead3 | NM_001098226.3 | chr17:28331672-28350600 |
| 21105 | Tead4 | NM_001080979.1 | chr6:128227142-128300813 |
| 21106 | Tec | NM_001113460.2 | chr5:72755717-72868448 |
| 21107 | Tecpr1 | NM_027410.1 | chr5:144195346-144223578 |
| 21108 | Tecpr2 | NM_001081057.2 | chr12:110889263-110972394 |
| 21109 | Tecr | NM_027179.1 | chr8:83571697-83594491 |
| 21110 | Tecrl | NM_153801.3 | chr5:83278121-83355195 |
| 21111 | Tecta | NM_009347.2 | chr9:42329621-42399929 |
| 21112 | Tectb | NM_009348.3 | chr19:55180884-55196313 |
| 21113 | Teddm1 | NM_178244.3 | chr1:153891645-153893060 |
| 21114 | Tef | NM_017376.3 | chr15:81811413-81826863 |
| 21115 | Tefm | NM_183275.2 | chr11:80136677-80142153 |
| 21116 | Tek | NM_001290549.1 | chr4:94739288-94874976 |
| 21117 | Tekt1 | NM_001282006.1 | chr11:72344716-72362442 |
| 21118 | Tekt2 | NM_011902.2 | chr4:126322120-126325199 |
| 21119 | Tekt3 | NM_027660.1 | chr11:63061658-63094960 |
| 21120 | Tekt4 | NM_027951.1 | chr17:25471589-25476594 |
| 21121 | Tekt5 | NM_001099275.1 | chr16:10361253-10395448 |
| 21122 | Telo2 | NM_001163661.1 | chr17:25099568-25115967 |
| 21123 | Ten1 | NM_027107.1 | chr11:116198854-116215318 |
| 21124 | Tenc1 | NM_153533.2 | chr15:102102987-102116401 |
| 21125 | Tenm1 | NM_011855.4 | chrX:42532392-43274784 |
| 21126 | Tenm2 | NM_001290702.1 | chr11:36006655-36944241 |
| 21127 | Tenm3 | NM_001145937.1 | chr8:48225664-48674690 |
| 21128 | Tenm4 | NM_011858.4 | chr7:96210636-96911092 |
| 21129 | Tep1 | NM_009351.2 | chr14:50824060-50870554 |
| 21130 | Tepp | NM_028532.3 | chr8:95311597-95321329 |
| 21131 | Terc | NR_001579.1 | chr3:96414436-96414833 |
| 21132 | Terf1 | NM_001286628.1 | chr1:15805645-15844052 |
| 21133 | Terf2 | NM_001083118.2 | chr8:107075516-107096545 |
| 21134 | Terf2ip | NM_020584.2 | chr8:112011358-112020528 |
| 21135 | Tert | NM_009354.1 | chr13:73627000-73649041 |
| 21136 | Tes | NM_207176.3 | chr6:17065148-17105825 |
| 21137 | Tesc | NM_021344.3 | chr5:118027823-118061870 |
| 21138 | Tescl | NM_001163810.1 | chr7:24333079-24333965 |
| 21139 | Tesk1 | NM_011571.3 | chr4:43442276-43448075 |
| 21140 | Tesk2 | NM_146151.4 | chr4:116720954-116804248 |
| 21141 | Tespa1 | NM_183264.4 | chr10:130322851-130362642 |
| 21142 | Tet1 | NM_001253857.1 | chr10:62804569-62880014 |
| 21143 | Tet2 | NM_001040400.2 | chr3:133463676-133544390 |
| 21144 | Tet3 | NM_183138.2 | chr6:83362373-83441678 |
| 21145 | Tex10 | NM_172304.3 | chr4:48430955-48473422 |

Fig.21 - 110

| | | | | | | | |
|---|---|---|---|---|---|---|---|
| 21146 | Tex101 | NM_019981.2 | chr7:24668011-24672050 | 21243 | Theg | NM_011583.3 | chr10:79576476-79587136 |
| 21147 | Tex11 | NM_001167997.1 | chrX:100877881-101059639 | 21244 | Them4 | NM_029431.1 | chr3:94310131-94332532 |
| 21148 | Tex12 | NM_025687.3 | chr9:50557147-50561268 | 21245 | Them5 | NM_025416.3 | chr3:94342098-94347352 |
| 21149 | Tex13 | NM_031381.2 | chrX:140808306-140813433 | 21246 | Them6 | NM_198607.1 | chr15:74721233-74724373 |
| 21150 | Tex13a | NM_026469.2 | chrX:138208164-138209580 | 21247 | Them7 | NM_001159638.1 | chr2:105224341-105379860 |
| 21151 | Tex14 | NM_001199293.1 | chr11:87427590-87555823 | 21248 | Themis | NM_178666.6 | chr10:28668359-28883818 |
| 21152 | Tex15 | NM_031374.2 | chr8:33570543-33585585 | 21249 | Themis2 | NM_001033308.2 | chr4:132782356-132796364 |
| 21153 | Tex16 | NM_031382.2 | chrX:112093519-112127326 | 21250 | Themis3 | NM_028998.1 | chr17:66555251-66594622 |
| 21154 | Tex19.1 | NM_028602.2 | chr11:121146142-121148313 | 21251 | Thg1l | NM_001080969.3 | chr11:45945305-45955503 |
| 21155 | Tex19.2 | NM_027622.3 | chr11:121116214-121118677 | 21252 | Thnsl1 | NM_001001297.2 | chr2:21205723-21215009 |
| 21156 | Tex2 | NM_198292.3 | chr11:106502138-106612930 | 21253 | Thnsl2 | NM_001033929.2 | chr6:71128165-71144380 |
| 21157 | Tex21 | NM_001159532.1 | chr12:76203984-76246746 | 21254 | Thoc1 | NM_153552.3 | chr18:9958179-9995484 |
| 21158 | Tex22 | NM_029381.1 | chr12:113074501-113088914 | 21255 | Thoc2 | NM_001033422.1 | chrX:41794993-41911901 |
| 21159 | Tex24 | NM_001013609.2 | chr8:27344393-27349188 | 21256 | Thoc3 | NM_028597.3 | chr13:54458836-54468840 |
| 21160 | Tex26 | NM_029464.2 | chr5:149439659-149470979 | 21257 | Thoc5 | NM_172438.3 | chr11:4895342-4928865 |
| 21161 | Tex261 | NM_009357.2 | chr6:83770413-83775812 | 21258 | Thoc6 | NM_001008425.1 | chr17:23668618-23673770 |
| 21162 | Tex264 | NM_001081654.2 | chr9:106658745-106685952 | 21259 | Thoc7 | NM_001285780.1 | chr14:13949011-13961313 |
| 21163 | Tex28 | NM_001126488.2 | chrX:74150943-74167838 | 21260 | Thop1 | NM_022653.4 | chr10:81070082-81082360 |
| 21164 | Tex29 | NM_029326.1 | chr8:11840520-11855761 | 21261 | Thpo | NM_001173505.1 | chr16:20724453-20730598 |
| 21165 | Tex30 | NM_029368.1 | chr1:44086616-44102388 | 21262 | Thra | NM_178060.4 | chr11:98741784-98765113 |
| 21166 | Tex33 | NM_001163612.2 | chr15:78378399-78395912 | 21263 | Thrap3 | NM_146153.3 | chr4:126164082-126202710 |
| 21167 | Tex35 | NM_028540.3 | chr1:157099146-157108650 | 21264 | Thrb | NM_001113417.1 | chr14:17660959-18038088 |
| 21168 | Tex36 | NM_028654.1 | chr7:133587023-133602115 | 21265 | Thrsp | NM_009381.2 | chr7:97412956-97417510 |
| 21169 | Tex37 | NM_028825.3 | chr6:70913088-70918922 | 21266 | Thsd1 | NM_001205253.1 | chr8:22227302-22261332 |
| 21170 | Tex38 | NM_029196.1 | chr4:115779833-115781034 | 21267 | Thsd4 | NM_001040426.3 | chr9:59966930-60511035 |
| 21171 | Tex40 | NM_001039494.2 | chr19:6922425-6925380 | 21268 | Thsd7a | NM_001164805.1 | chr6:12311607-12749253 |
| 21172 | Tex43 | NM_026099.3 | chr18:56588347-56594779 | 21269 | Thsd7b | NM_172485.3 | chr1:129273303-130219278 |
| 21173 | Tex9 | NM_009359.4 | chr9:72458054-72491959 | 21270 | Thtpa | NM_153083.5 | chr14:55094783-55098995 |
| 21174 | Tfam | NM_009360.4 | chr10:71225476-71238044 | 21271 | Thumpd1 | NM_145585.2 | chr7:119715093-119720798 |
| 21175 | Tfap2a | NM_001122948.2 | chr13:40715302-40730457 | 21272 | Thumpd2 | NM_028138.1 | chr17:81026326-81065085 |
| 21176 | Tfap2b | NM_001025305.2 | chr1:19212053-19238845 | 21273 | Thumpd3 | NM_008188.2 | chr6:113046326-113068273 |
| 21177 | Tfap2c | NM_001159696.1 | chr2:172550990-172558621 | 21274 | Thy1 | NM_009382.3 | chr9:44043383-44048579 |
| 21178 | Tfap2d | NM_153154.2 | chr1:19103021-19166332 | 21275 | Thyn1 | NM_144543.2 | chr9:26999676-27007334 |
| 21179 | Tfap2e | NM_198960.2 | chr4:126716002-126736269 | 21276 | Tia1 | NM_001164078.1 | chr6:86404218-86433405 |
| 21180 | Tfap4 | NM_031182.2 | chr16:4544660-4559720 | 21277 | Tial1 | NM_009383.2 | chr7:128439776-128461513 |
| 21181 | Tfb1m | NM_146074.1 | chr17:3519255-3557713 | 21278 | Tiam1 | NM_001145886.1 | chr16:89787110-89974699 |
| 21182 | Tfb2m | NM_008249.4 | chr1:179528055-179546267 | 21279 | Tiam2 | NM_001122998.1 | chr17:3326572-3519397 |
| 21183 | Tfcp2 | NM_001289603.1 | chr15:100502747-100552008 | 21280 | Ticam1 | NM_174989.4 | chr17:56269461-56276767 |
| 21184 | Tfcp2l1 | NM_023755.2 | chr1:118627944-118685168 | 21281 | Ticam2 | NM_173394.3 | chr18:46558230-46574533 |
| 21185 | Tfdp1 | NM_001291765.1 | chr8:13338750-13378448 | 21282 | Ticrr | NM_029835.1 | chr7:79660195-79698145 |
| 21186 | Tfdp2 | NM_001184706.1 | chr9:96196274-96323646 | 21283 | Tie1 | NM_011587.2 | chr4:118471190-118489849 |
| 21187 | Tfe3 | NM_001105196.1 | chrX:7762660-7775202 | 21284 | Tifa | NM_145133.3 | chr3:127789912-127798389 |
| 21188 | Tfeb | NM_001161722.1 | chr17:47737036-47792416 | 21285 | Tifab | NM_001168615.1 | chr13:56173702-56178885 |
| 21189 | Tfec | NM_031198.3 | chr6:16833380-16898441 | 21286 | Tigd2 | NM_001081145.1 | chr6:59208869-59212033 |
| 21190 | Tff1 | NM_009362.2 | chr17:31161395-31165053 | 21287 | Tigd3 | NM_198634.1 | chr19:5891137-5894107 |
| 21191 | Tff2 | NM_009363.3 | chr17:31141061-31144282 | 21288 | Tigd4 | NM_207278.2 | chr3:84593573-84597032 |
| 21192 | Tff3 | NM_011575.2 | chr17:31125305-31129611 | 21289 | Tigd5 | NM_178646.4 | chr15:75909734-75914535 |
| 21193 | Tfg | NM_001252443.1 | chr16:56690328-56717450 | 21290 | Tigit | NM_001146325.1 | chr16:43648860-43664184 |
| 21194 | Tfip11 | NM_018783.4 | chr5:112326368-112338073 | 21291 | Timd2 | NM_001161355.1 | chr11:46668959-46698830 |
| 21195 | Tfpi | NM_001177319.1 | chr2:84440722-84476759 | 21292 | Timd4 | NM_178759.4 | chr11:46810798-46844333 |
| 21196 | Tfpi2 | NM_009364.3 | chr6:3962594-3968354 | 21293 | Timeless | NM_001136082.2 | chr10:128232062-128252941 |
| 21197 | Tfpt | NM_001290381.1 | chr7:3620323-3629929 | 21294 | Timm10 | NM_013899.2 | chr2:84827020-84830213 |
| 21198 | Tfr2 | NM_001289507.1 | chr5:137570804-137588081 | 21295 | Timm10b | NM_019502.2 | chr7:105640539-105641845 |
| 21199 | Tfrc | NM_011638.4 | chr16:32608895-32632794 | 21296 | Timm13 | NM_013895.4 | chr10:80899449-80900969 |
| 21200 | Tg | NM_009375.2 | chr15:66670769-66850720 | 21297 | Timm17a | NM_011590.2 | chr1:135301534-135313737 |
| 21201 | Tgds | NM_029578.3 | chr14:118111910-118132765 | 21298 | Timm17b | NM_011591.5 | chrX:7899335-7907652 |
| 21202 | Tgfa | NM_031199.4 | chr6:86195226-86275743 | 21299 | Timm21 | NM_025969.4 | chr18:84947293-84951524 |
| 21203 | Tgfb1 | NM_011577.2 | chr7:25687001-25705077 | 21300 | Timm22 | NM_001291161.1 | chr11:76406924-76416313 |
| 21204 | Tgfb1i1 | NM_001289550.1 | chr7:128246811-128255699 | 21301 | Timm23 | NM_016897.3 | chr14:32180165-32201891 |
| 21205 | Tgfb2 | NM_009367.3 | chr1:186623185-186705992 | 21302 | Timm44 | NM_011592.2 | chr8:4259730-4275905 |
| 21206 | Tgfb3 | NM_009368.3 | chr12:86056742-86079041 | 21303 | Timm50 | NM_025616.3 | chr7:28305825-28312046 |
| 21207 | Tgfbi | NM_009369.4 | chr13:56609602-56639339 | 21304 | Timm8a1 | NM_013898.3 | chrX:11609223-134541670 |
| 21208 | Tgfbr1 | NM_009370.3 | chr4:47353221-47414924 | 21305 | Timm8a2 | NM_001037744.1 | chr14:122034673-122038422 |
| 21209 | Tgfbr2 | NM_009371.3 | chr9:116087694-116175363 | 21306 | Timm8b | NM_013897.2 | chr9:50603900-50605320 |
| 21210 | Tgfbr3 | NM_011578.3 | chr5:107106569-107289595 | 21307 | Timm9 | NM_001024853.1 | chr12:71123171-71136675 |
| 21211 | Tgfbrap1 | NM_001013025.2 | chr1:43047268-43098622 | 21308 | Timmdc1 | NM_024273.2 | chr16:38497842-38522663 |
| 21212 | Tgif1 | NM_001164074.1 | chr17:70844204-70853532 | 21309 | Timp1 | NM_001044384.1 | chrX:20870165-20874737 |
| 21213 | Tgif2 | NM_001291124.1 | chr2:156840006-156855569 | 21310 | Timp2 | NM_011594.3 | chr11:118301060-118355411 |
| 21214 | Tgif2lx1 | NM_153109.1 | chrX:118427234-118480729 | 21311 | Timp3 | NM_011595.2 | chr10:86300411-86349505 |
| 21215 | Tgif2lx2 | NM_001142750.1 | chrX:118427226-118480737 | 21312 | Timp4 | NM_080639.3 | chr6:115245615-115251849 |
| 21216 | Tgm1 | NM_001161714.1 | chr14:55700008-55713492 | 21313 | Tinag | NM_012033.3 | chr9:76951697-77045781 |
| 21217 | Tgm2 | NM_009373.3 | chr2:158116404-158146392 | 21314 | Tinagl1 | NM_001168333.1 | chr4:130165599-130174802 |
| 21218 | Tgm3 | NM_009374.3 | chr2:130012339-130050399 | 21315 | Tinf2 | NM_145705.3 | chr14:55679079-55681817 |
| 21219 | Tgm4 | NM_177911.4 | chr9:123034740-123067558 | 21316 | Tiparp | NM_178892.5 | chr3:65528446-65555518 |
| 21220 | Tgm5 | NM_028799.2 | chr2:121046110-121085759 | 21317 | Tipin | NM_025372.3 | chr9:64281606-64304792 |
| 21221 | Tgm6 | NM_001289747.1 | chr2:130123274-130154232 | 21318 | Tiprl | NM_145513.4 | chr1:165212285-165236958 |
| 21222 | Tgm7 | NM_001160424.1 | chr2:121093588-121109791 | 21319 | Tirap | NM_001177845.1 | chr9:35184390-35200291 |
| 21223 | Tgoln1 | NM_009443.3 | chr6:72608420-72617000 | 21320 | Tjap1 | NM_001252473.1 | chr17:46257850-46283026 |
| 21224 | Tgoln2 | NM_009444.1 | chr6:72610956-72616748 | 21321 | Tjp1 | NM_001163574.1 | chr7:65296164-65371244 |
| 21225 | Tgs1 | NM_054089.4 | chr4:3574878-3616623 | 21322 | Tjp2 | NM_001198985.1 | chr19:24094501-24174140 |
| 21226 | Tgtp1 | NM_011579.3 | chr11:48985328-48992246 | 21323 | Tjp3 | NM_001282095.1 | chr10:81273187-81291581 |
| 21227 | Tgtp2 | NM_001145164.1 | chr11:49057195-49064212 | 21324 | Tk1 | NM_001271729.1 | chr11:117815518-117826014 |
| 21228 | Th | NM_009377.1 | chr7:142892775-142899966 | 21325 | Tk2 | NM_021028.3 | chr8:104226690-104248558 |
| 21229 | Tha1 | NM_027919.4 | chr11:117867948-117873526 | 21326 | Tkt | NM_009388.6 | chr14:30549130-30574726 |
| 21230 | Thada | NM_183021.3 | chr17:84190055-84466208 | 21327 | Tktl1 | NM_031379.2 | chrX:74177258-74208498 |
| 21231 | Thap1 | NM_199042.2 | chr8:26158168-26164151 | 21328 | Tktl2 | NM_001271574.1 | chr8:66511739-66519199 |
| 21232 | Thap11 | NM_021513.2 | chr8:105855102-105856950 | 21329 | Tlcd1 | NM_001291235.1 | chr11:78178148-78180819 |
| 21233 | Thap2 | NM_025780.3 | chr10:115369965-115384435 | 21330 | Tlcd2 | NM_001291156.1 | chr11:75468049-75470899 |
| 21234 | Thap3 | NM_001145929.1 | chr4:151982637-151988986 | 21331 | Tldc1 | NM_028883.2 | chr8:119760586-119778416 |
| 21235 | Thap4 | NM_025920.3 | chr1:93705390-93754838 | 21332 | Tldc2 | NM_001177439.1 | chr2:157087054-157096481 |
| 21236 | Thap6 | NR_028429.1 | chr5:91962881-91972137 | 21333 | Tle1 | NM_001285529.1 | chr4:72109944-72200893 |
| 21237 | Thap7 | NM_026909.2 | chr16:17527981-17531052 | 21334 | Tle2 | NM_001252401.1 | chr10:81575286-81590473 |
| 21238 | Thbd | NM_009378.3 | chr2:148404470-148408188 | 21335 | Tle3 | NM_001083927.1 | chr9:61372365-61418497 |
| 21239 | Thbs1 | NM_011580.4 | chr2:118111875-118127133 | 21336 | Tle4 | NM_011600.3 | chr19:14448071-14598183 |
| 21240 | Thbs2 | NM_011581.3 | chr17:14665499-14694262 | 21337 | Tle6 | NM_053254.2 | chr10:81590904-81600900 |
| 21241 | Thbs3 | NM_013691.2 | chr3:89215186-89226837 | 21338 | Tlk1 | NM_172664.3 | chr2:70712407-70825480 |
| 21242 | Thbs4 | NM_011582.3 | chr13:92751585-92794818 | 21339 | Tlk2 | NM_001112705.2 | chr11:105181526-105283959 |

Fig.21 - 111

| | | | | | | | |
|---|---|---|---|---|---|---|---|
| 21340 | Tll1 | NM_009390.2 | chr8:64014769-64205993 | 21437 | Tmem129 | NM_026698.2 | chr5:33653215-33657832 |
| 21341 | Tll2 | NM_011904.3 | chr19:41083980-41206774 | 21438 | Tmem130 | NM_177735.4 | chr5:144735914-144761578 |
| 21342 | Tln1 | NM_011602.5 | chr4:43531512-43562583 | 21439 | Tmem131 | NM_018872.2 | chr1:36792188-36939527 |
| 21343 | Tln2 | NM_001081242.2 | chr9:67217084-67559703 | 21440 | Tmem132a | NM_133804.2 | chr19:10857828-10869779 |
| 21344 | Tlr1 | NM_001276445.1 | chr5:64924679-64933558 | 21441 | Tmem132b | NM_001190352.1 | chr5:125532417-125792583 |
| 21345 | Tlr11 | NM_205819.3 | chr14:50357913-50363663 | 21442 | Tmem132c | NM_175432.3 | chr5:127241825-127565790 |
| 21346 | Tlr12 | NM_205823.2 | chr4:128615445-128618619 | 21443 | Tmem132cos | NR_038127.1 | chr5:127256859-127281623 |
| 21347 | Tlr13 | NM_205820.1 | chrX:106143274-106160493 | 21444 | Tmem132d | NM_172885.2 | chr5:127783490-128433077 |
| 21348 | Tlr2 | NM_011905.3 | chr3:83836271-83841608 | 21445 | Tmem132e | NM_001304439.1 | chr11:82387446-82447618 |
| 21349 | Tlr3 | NM_126166.4 | chr8:45395664-45410539 | 21446 | Tmem134 | NM_001078649.1 | chr19:4125959-4132307 |
| 21350 | Tlr4 | NM_021297.3 | chr4:66827550-66846581 | 21447 | Tmem135 | NM_028343.4 | chr7:89139720-89338787 |
| 21351 | Tlr5 | NM_016928.3 | chr1:182954787-182976044 | 21448 | Tmem136 | NM_001034863.3 | chr9:43108652-43116570 |
| 21352 | Tlr6 | NM_011604.3 | chr5:64953094-64960034 | 21449 | Tmem138 | NM_028411.4 | chr19:10570477-10577386 |
| 21353 | Tlr7 | NM_001290755.1 | chrX:167304925-167330571 | 21450 | Tmem139 | NM_175408.4 | chr6:42261969-42264555 |
| 21354 | Tlr8 | NM_133212.3 | chrX:167241122-167264329 | 21451 | Tmem140 | NM_197986.2 | chr6:34863145-34874946 |
| 21355 | Tlr9 | NM_031178.2 | chr9:106222597-106226876 | 21452 | Tmem141 | NM_001040130.3 | chr2:25620065-25622002 |
| 21356 | Tlx1 | NM_021901.3 | chr19:45150714-45156943 | 21453 | Tmem143 | NM_144801.2 | chr7:45897068-45917413 |
| 21357 | Tlx2 | NM_009392.2 | chr6:83068324-83070225 | 21454 | Tmem144 | NM_027495.4 | chr3:79813152-79842662 |
| 21358 | Tlx3 | NM_019916.2 | chr11:33200751-33203588 | 21455 | Tmem145 | NM_183311.2 | chr7:25306107-25316195 |
| 21359 | Tm2d1 | NM_053157.2 | chr4:98355369-98383265 | 21456 | Tmem147 | NM_027215.2 | chr7:30727700-30729534 |
| 21360 | Tm2d2 | NM_027194.3 | chr8:25017210-25023260 | 21457 | Tmem14a | NM_001290679.1 | chr1:21218574-21230167 |
| 21361 | Tm2d3 | NM_026795.3 | chr7:65693416-65701913 | 21458 | Tmem14c | NM_025387.3 | chr13:41016249-41022582 |
| 21362 | Tm4sf1 | NM_008536.3 | chr3:57287063-57301919 | 21459 | Tmem150a | NM_144916.3 | chr6:72355482-72359762 |
| 21363 | Tm4sf19 | NM_001160402.1 | chr16:32400505-32408227 | 21460 | Tmem150b | NM_001142792.1 | chr7:4716001-4725082 |
| 21364 | Tm4sf20 | NM_025453.3 | chr1:82756648-82768456 | 21461 | Tmem150c | NM_182841.1 | chr5:100077872-100159808 |
| 21365 | Tm4sf4 | NM_145539.2 | chr3:57425409-57441675 | 21462 | Tmem150cos | NR_045993.1 | chr5:100077961-100095273 |
| 21366 | Tm4sf5 | NM_029360.3 | chr11:70505273-70511183 | 21463 | Tmem151a | NM_001001885.1 | chr19:5079336-5085477 |
| 21367 | Tm6sf1 | NM_001291282.1 | chr7:81862686-81884079 | 21464 | Tmem151b | NM_001013749.2 | chr17:45541939-45549677 |
| 21368 | Tm6sf2 | NM_181540.4 | chr8:70072923-70080066 | 21465 | Tmem154 | NM_177260.2 | chr3:84666191-84704575 |
| 21369 | Tm7sf2 | NM_028454.2 | chr19:6062820-6067850 | 21466 | Tmem158 | NM_001002267.2 | chr9:123259056-123260789 |
| 21370 | Tm7sf3 | NM_026281.2 | chr6:146602275-146634592 | 21467 | Tmem159 | NM_145586.1 | chr7:120102425-120120986 |
| 21371 | Tm9sf1 | NM_028780.3 | chr14:55635965-55643806 | 21468 | Tmem160 | NM_026938.1 | chr7:16452778-16455490 |
| 21372 | Tm9sf2 | NM_080556.3 | chr14:122107081-122159603 | 21469 | Tmem161a | NM_145597.4 | chr8:70172407-70183681 |
| 21373 | Tm9sf3 | NM_133352.2 | chr19:41210841-41264004 | 21470 | Tmem161b | NM_175187.5 | chr13:84222295-84295966 |
| 21374 | Tm9sf4 | NM_133847.3 | chr2:153161300-153210463 | 21471 | Tmem163 | NM_028135.2 | chr1:127490341-127678021 |
| 21375 | Tma16 | NM_025465.2 | chr8:66476345-66486507 | 21472 | Tmem164 | NM_001199357.1 | chrX:142681399-142843494 |
| 21376 | Tma7 | NM_183250.2 | chr9:109077987-109082381 | 21473 | Tmem165 | NM_011626.2 | chr5:76183879-76209244 |
| 21377 | Tmbim1 | NM_027154.5 | chr1:74288246-74304336 | 21474 | Tmem167 | NM_025335.3 | chr13:90089666-90114921 |
| 21378 | Tmbim4 | NM_026617.3 | chr10:120208825-120224897 | 21475 | Tmem167b | NM_026198.2 | chr3:108556424-108562466 |
| 21379 | Tmbim6 | NM_001171034.1 | chr15:99392946-99410049 | 21476 | Tmem168 | NM_028990.4 | chr6:13580688-13608063 |
| 21380 | Tmbim7 | NM_029141.4 | chr5:3657003-3679544 | 21477 | Tmem169 | NM_175564.4 | chr1:72284372-72302995 |
| 21381 | Tmc1 | NM_028953.2 | chr19:20783457-20954202 | 21478 | Tmem17 | NM_153596.3 | chr11:22512282-22519231 |
| 21382 | Tmc2 | NM_138655.1 | chr2:130195193-130264445 | 21479 | Tmem170 | NM_025781.2 | chr8:111864897-111876675 |
| 21383 | Tmc3 | NM_177695.4 | chr7:83584930-83623709 | 21480 | Tmem170b | NM_001163572.1 | chr13:41606215-41641357 |
| 21384 | Tmc4 | NM_181820.2 | chr7:3665753-3677553 | 21481 | Tmem171 | NM_001025606.1 | chr13:98686237-98694831 |
| 21385 | Tmc5 | NM_001105252.1 | chr7:118597296-118675085 | 21482 | Tmem173 | NM_001289591.1 | chr18:35733677-35740554 |
| 21386 | Tmc6 | NM_145439.2 | chr11:117765984-117780683 | 21483 | Tmem174 | NM_026685.2 | chr13:98634977-98637410 |
| 21387 | Tmc7 | NM_172476.4 | chr7:118535843-118584686 | 21484 | Tmem175 | NM_001163531.1 | chr5:108629809-108647770 |
| 21388 | Tmc8 | NM_001195088.1 | chr11:117782657-117793137 | 21485 | Tmem176a | NM_001098271.1 | chr6:48841655-48845364 |
| 21389 | Tmcc1 | NM_177412.1 | chr6:116018617-116193374 | 21486 | Tmem176b | NM_001164207.1 | chr6:48833811-48841374 |
| 21390 | Tmcc2 | NM_178874.3 | chr1:132356314-132391386 | 21487 | Tmem177 | NM_175106.3 | chr1:119907898-119913168 |
| 21391 | Tmcc3 | NM_001168684.1 | chr10:94575256-94590954 | 21488 | Tmem178 | NM_026516.2 | chr17:80944631-81001816 |
| 21392 | Tmco1 | NM_001039483.1 | chr1:167308669-167333978 | 21489 | Tmem178b | NM_001004182.3 | chr6:40110252-40248353 |
| 21393 | Tmco2 | NM_001081312.1 | chr4:121105650-121109226 | 21490 | Tmem179 | NM_178915.3 | chr12:112500183-112511160 |
| 21394 | Tmco3 | NM_172282.2 | chr8:13288012-13322924 | 21491 | Tmem179b | NM_026325.3 | chr19:8772521-8774467 |
| 21395 | Tmco4 | NM_029857.3 | chr4:138972904-139059171 | 21492 | Tmem18 | NM_172049.2 | chr12:30584442-30591219 |
| 21396 | Tmco5 | NM_026104.4 | chr2:116878690-116892500 | 21493 | Tmem180 | NM_029186.2 | chr19:46356879-46375254 |
| 21397 | Tmco5b | NM_029232.2 | chr2:113285731-113297190 | 21494 | Tmem181a | NM_001033178.3 | chr17:6270469-6308314 |
| 21398 | Tmco6 | NM_028036.3 | chr18:36735069-36742391 | 21495 | Tmem181b-ps | NR_033520.1 | chr17:6439001-6450994 |
| 21399 | Tmed1 | NM_010744.3 | chr9:21507379-21510186 | 21496 | Tmem181c-ps | NR_028305.2 | chr17:6610102-6620925 |
| 21400 | Tmed10 | NM_026775.4 | chr12:85340613-85374717 | 21497 | Tmem182 | NM_001081198.1 | chr1:40805600-40855267 |
| 21401 | Tmed11 | NM_026109.2 | chr5:108777234-108795363 | 21498 | Tmem183a | NM_001042485.1 | chr1:134346096-134361999 |
| 21402 | Tmed2 | NM_019770.2 | chr5:124540790-124550503 | 21499 | Tmem184a | NM_001161548.1 | chr5:139804951-139814283 |
| 21403 | Tmed3 | NM_025360.2 | chr9:89699202-89705043 | 21500 | Tmem184b | NM_001253817.1 | chr15:79360683-79402919 |
| 21404 | Tmed4 | NM_134020.1 | chr11:6270713-6274837 | 21501 | Tmem184c | NM_145599.4 | chr8:77595977-77610653 |
| 21405 | Tmed5 | NM_028876.2 | chr5:108121646-108132591 | 21502 | Tmem185b | NM_146103.2 | chr1:119526153-119528983 |
| 21406 | Tmed6 | NM_025458.2 | chr8:107061483-107065644 | 21503 | Tmem186 | NM_025708.4 | chr16:8633730-8637701 |
| 21407 | Tmed7 | NM_025698.1 | chr18:46585927-46597535 | 21504 | Tmem189 | NM_145538.2 | chr2:167643224-167661544 |
| 21408 | Tmed8 | NM_001033475.3 | chr12:87166241-87200229 | 21505 | Tmem19 | NM_133683.3 | chr10:115340739-115362262 |
| 21409 | Tmed9 | NM_026211.3 | chr13:55593134-55597694 | 21506 | Tmem190 | NM_030028.1 | chr7:4782939-4784340 |
| 21410 | Tmeff1 | NM_021436.2 | chr4:48585192-48663131 | 21507 | Tmem191c | NM_177473.3 | chr16:17276299-17278661 |
| 21411 | Tmeff2 | NM_019790.4 | chr1:50927522-51187270 | 21508 | Tmem192 | NM_001163747.1 | chr8:64947184-64969037 |
| 21412 | Tmem100 | NM_026433.2 | chr11:90030347-90036505 | 21509 | Tmem194 | NM_001113211.1 | chr10:127677064-127701047 |
| 21413 | Tmem101 | NM_029649.2 | chr11:102152546-102156407 | 21510 | Tmem194b | NM_001142647.1 | chr1:52630704-52651919 |
| 21414 | Tmem102 | NM_001033433.4 | chr11:69803594-69805624 | 21511 | Tmem196 | NM_001160385.2 | chr12:119945961-120021245 |
| 21415 | Tmem104 | NM_001033393.2 | chr11:115187486-115247025 | 21512 | Tmem198 | NM_177056.4 | chr1:75479531-75485693 |
| 21416 | Tmem106a | NM_144830.3 | chr11:101582241-101591785 | 21513 | Tmem198b | NM_178066.2 | chr10:128800035-128804370 |
| 21417 | Tmem106b | NM_027992.3 | chr6:13069758-13089269 | 21514 | Tmem199 | NM_199199.3 | chr11:78507054-78512168 |
| 21418 | Tmem106c | NM_001252153.1 | chr15:97964228-97970286 | 21515 | Tmem2 | NM_001033759.2 | chr19:21778339-21858360 |
| 21419 | Tmem107 | NM_025838.2 | chr11:69070808-69073293 | 21516 | Tmem200a | NM_029881.3 | chr10:25991185-26079052 |
| 21420 | Tmem108 | NM_178638.4 | chr9:103482935-103761837 | 21517 | Tmem200b | NM_001201367.1 | chr4:131921770-131923140 |
| 21421 | Tmem109 | NM_134142.1 | chr19:10870660-10881743 | 21518 | Tmem200c | NM_001206661.1 | chr17:68837135-68843138 |
| 21422 | Tmem11 | NM_001168507.1 | chr11:60864451-60879038 | 21519 | Tmem201 | NM_001284270.1 | chr4:149715374-149738068 |
| 21423 | Tmem110 | NM_028839.4 | chr14:30825593-30877210 | 21520 | Tmem202 | NM_178388.2 | chr9:59518684-59525501 |
| 21424 | Tmem115 | NM_019704.2 | chr9:107533944-107538656 | 21521 | Tmem203 | NM_177344.3 | chr2:25255438-25256352 |
| 21425 | Tmem116 | NM_001161626.1 | chr5:121463180-121495421 | 21522 | Tmem204 | NM_001001183.1 | chr17:25057701-25081114 |
| 21426 | Tmem117 | NM_178789.4 | chr15:94629184-95096097 | 21523 | Tmem205 | NM_001253867.1 | chr9:21921008-21927535 |
| 21427 | Tmem119 | NM_146162.2 | chr5:113793728-113800352 | 21524 | Tmem206 | NM_025864.3 | chr1:191325964-191352925 |
| 21428 | Tmem120a | NM_172541.2 | chr5:135735489-135744172 | 21525 | Tmem207 | NM_001101640.1 | chr16:26503792-26526771 |
| 21429 | Tmem120b | NM_001039723.2 | chr5:123076274-123117445 | 21526 | Tmem208 | NM_025486.2 | chr8:105326363-105329057 |
| 21430 | Tmem121 | NM_153776.2 | chr12:113185902-113189522 | 21527 | Tmem209 | NM_178625.4 | chr6:30481232-30509783 |
| 21431 | Tmem123 | NM_133739.2 | chr9:7764076-7794332 | 21528 | Tmem210 | NM_030055.1 | chr2:25288144-25289187 |
| 21432 | Tmem125 | NM_172383.3 | chr4:118540940-118543726 | 21529 | Tmem211 | NM_001033428.2 | chr5:113226908-113239263 |
| 21433 | Tmem126a | NM_025460.2 | chr7:90450711-90457208 | 21530 | Tmem212 | NM_001164437.1 | chr3:27866064-27896368 |
| 21434 | Tmem126b | NM_026734.1 | chr7:90468828-90475995 | 21531 | Tmem213 | NM_029921.1 | chr6:38109352-38115806 |
| 21435 | Tmem127 | NM_175145.3 | chr2:127247974-127260764 | 21532 | Tmem214 | NM_144525.3 | chr5:30869646-30877467 |
| 21436 | Tmem128 | NM_025480.3 | chr5:38260374-38269618 | 21533 | Tmem215 | NM_001166009.1 | chr4:40473129-40475653 |

Fig.21 - 112

| | | | |
|---|---|---|---|
| 21534 | Tmem216 | NM_001277860.1 | chr19:10550460-10555763 |
| 21535 | Tmem217 | NM_001162901.1 | chr17:29526017-29549593 |
| 21536 | Tmem218 | NM_025464.3 | chr9:37208222-37223228 |
| 21537 | Tmem219 | NM_026827.1 | chr7:126886218-126898278 |
| 21538 | Tmem220 | NM_001291042.1 | chr11:67025153-67035312 |
| 21539 | Tmem221 | NM_001100462.1 | chr8:71554302-71558871 |
| 21540 | Tmem222 | NM_025667.3 | chr4:133266044-133277790 |
| 21541 | Tmem223 | NM_025791.1 | chr19:8770995-8772475 |
| 21542 | Tmem225 | NM_029379.1 | chr9:40148121-40150878 |
| 21543 | Tmem229a | NM_177013.3 | chr6:24951140-24956125 |
| 21544 | Tmem229b | NM_001170401.1 | chr12:78961794-78983478 |
| 21545 | Tmem230 | NM_001141971.1 | chr2:132239491-132247788 |
| 21546 | Tmem231 | NM_001033321.1 | chr8:111912017-111933791 |
| 21547 | Tmem232 | NM_001008973.2 | chr17:65256004-65540782 |
| 21548 | Tmem233 | NM_001101546.1 | chr5:116040533-116083246 |
| 21549 | Tmem234 | NM_029748.2 | chr4:129600706-129607879 |
| 21550 | Tmem235 | NM_001085535.1 | chr11:117860751-117865543 |
| 21551 | Tmem236 | NM_001081310.2 | chr2:14174523-14221993 |
| 21552 | Tmem237 | NM_001033449.1 | chr1:59100592-59120096 |
| 21553 | Tmem238 | NM_029384.1 | chr7:4784784-4789560 |
| 21554 | Tmem239 | NM_025753.3 | chr2:130406521-130407794 |
| 21555 | Tmem240 | NM_001101506.1 | chr4:155734803-155740564 |
| 21556 | Tmem241 | NM_001289666.1 | chr18:11981302-12121537 |
| 21557 | Tmem242 | NM_027457.4 | chr17:5410863-5440260 |
| 21558 | Tmem243 | NM_001081029.1 | chr5:9100736-9118983 |
| 21559 | Tmem245 | NM_175518.5 | chr4:56876012-56947429 |
| 21560 | Tmem246 | NM_025944.3 | chr4:49584505-49597870 |
| 21561 | Tmem247 | NM_001277980.1 | chr17:86917347-86922367 |
| 21562 | Tmem248 | NM_001081394.1 | chr5:130219743-130243765 |
| 21563 | Tmem25 | NM_027865.2 | chr9:44793778-44799216 |
| 21564 | Tmem251 | NM_177140.3 | chr12:102743759-102745397 |
| 21565 | Tmem252 | NM_183160.3 | chr19:24674007-24679661 |
| 21566 | Tmem253 | NM_001033805.3 | chr14:52016864-52019787 |
| 21567 | Tmem254a | NM_025311.3 | chr14:25923297-26206619 |
| 21568 | Tmem254b | NM_001270495.1 | chr14:25923996-26207025 |
| 21569 | Tmem254c | NM_001270498.1 | chr14:25923997-26207041 |
| 21570 | Tmem255a | NM_001289727.1 | chrX:38196572-38252481 |
| 21571 | Tmem255b | NM_001143671.1 | chr8:13435458-13461451 |
| 21572 | Tmem256 | NM_026982.1 | chr11:69838524-69839558 |
| 21573 | Tmem258 | NM_001163431.1 | chr19:10206032-10207824 |
| 21574 | Tmem259 | NM_001003949.3 | chr10:79977119-79984330 |
| 21575 | Tmem26 | NM_177794.3 | chr10:68723745-68782654 |
| 21576 | Tmem260 | NM_172600.4 | chr14:48446351-48515130 |
| 21577 | Tmem261 | NM_025849.3 | chr4:75277353-75278286 |
| 21578 | Tmem263 | NM_001013028.2 | chr10:85102626-85117747 |
| 21579 | Tmem27 | NM_020626.2 | chrX:164090186-164118859 |
| 21580 | Tmem28 | NM_001081283.1 | chrX:99821068-99846345 |
| 21581 | Tmem29 | NM_001164683.1 | chrX:150397772-150459150 |
| 21582 | Tmem30a | NM_133718.4 | chr9:79768940-79793430 |
| 21583 | Tmem30b | NM_178715.3 | chr12:73543113-73546395 |
| 21584 | Tmem30c | NM_027651.1 | chr16:57266138-57292851 |
| 21585 | Tmem33 | NM_001285452.1 | chr5:67260564-67291461 |
| 21586 | Tmem35 | NM_026239.2 | chrX:134295224-134305969 |
| 21587 | Tmem37 | NM_019432.2 | chr1:120067376-120073780 |
| 21588 | Tmem38a | NM_144534.1 | chr8:72572102-72587284 |
| 21589 | Tmem38b | NM_028053.2 | chr4:53826044-53862018 |
| 21590 | Tmem39a | NM_001205286.1 | chr16:38558697-38592162 |
| 21591 | Tmem39b | NM_199305.1 | chr4:129676354-129696838 |
| 21592 | Tmem40 | NM_001168256.1 | chr6:115729136-115762466 |
| 21593 | Tmem41a | NM_025693.4 | chr16:21934326-21947552 |
| 21594 | Tmem41b | NM_153525.5 | chr7:109972186-109986230 |
| 21595 | Tmem42 | NM_001164823.1 | chr9:123021325-123023491 |
| 21596 | Tmem43 | NM_028766.2 | chr6:91473750-91488458 |
| 21597 | Tmem44 | NM_172614.3 | chr16:30511854-30550578 |
| 21598 | Tmem45a | NM_019631.3 | chr16:56805160-56886163 |
| 21599 | Tmem45b | NM_144936.1 | chr9:31426195-31464238 |
| 21600 | Tmem47 | NM_138751.2 | chrX:81070643-81097875 |
| 21601 | Tmem5 | NM_153059.1 | chr10:122081259-122097102 |
| 21602 | Tmem50a | NM_027935.2 | chr4:134897848-134914916 |
| 21603 | Tmem50b | NM_030018.3 | chr16:91574507-91597680 |
| 21604 | Tmem51 | NM_145402.3 | chr4:142030992-142084304 |
| 21605 | Tmem51os1 | NR_027137.1 | chr4:142084297-142088101 |
| 21606 | Tmem52 | NM_001253856.1 | chr4:155469113-155470858 |
| 21607 | Tmem52b | NM_001081186.1 | chr6:129512556-129519227 |
| 21608 | Tmem53 | NM_001285812.1 | chr4:117251950-117268588 |
| 21609 | Tmem54 | NM_001290706.1 | chr4:129105547-129111624 |
| 21610 | Tmem55a | NM_028264.4 | chr4:14864218-14915260 |
| 21611 | Tmem55b | NM_001033271.5 | chr14:50927214-50930849 |
| 21612 | Tmem56 | NM_178936.3 | chr3:121202009-121263316 |
| 21613 | Tmem57 | NM_025382.6 | chr4:134802759-134853345 |
| 21614 | Tmem59 | NM_029565.3 | chr4:107178629-107200996 |
| 21615 | Tmem59l | NM_182991.2 | chr8:70483866-70487358 |
| 21616 | Tmem60 | NM_177601.3 | chr5:20882452-20886870 |
| 21617 | Tmem62 | NM_175285.3 | chr2:120977061-121007842 |
| 21618 | Tmem63a | NM_144794.2 | chr1:180942517-180975104 |
| 21619 | Tmem63b | NM_198167.3 | chr17:45660176-45686218 |
| 21620 | Tmem63c | NM_172583.2 | chr12:87026563-87090039 |
| 21621 | Tmem64 | NM_181401.3 | chr4:15265819-15286753 |
| 21622 | Tmem65 | NM_175212.4 | chr15:58782268-58823427 |
| 21623 | Tmem66 | NM_026432.3 | chr8:34154562-34170847 |
| 21624 | Tmem67 | NM_177861.4 | chr4:12039355-12087957 |
| 21625 | Tmem68 | NM_028097.3 | chr4:3549040-3574768 |
| 21626 | Tmem69 | NM_177670.4 | chr4:116551527-116555943 |
| 21627 | Tmem70 | NM_026392.1 | chr1:16665190-16678275 |
| 21628 | Tmem71 | NM_172514.3 | chr15:66526211-66561046 |
| 21629 | Tmem72 | NM_178768.4 | chr6:116692724-116716758 |
| 21630 | Tmem74 | NM_175502.3 | chr15:43866694-43870029 |
| 21631 | Tmem74b | NM_001160363.1 | chr2:151702007-151707310 |
| 21632 | Tmem79 | NM_024246.5 | chr3:88328652-88334433 |
| 21633 | Tmem8 | NM_021793.2 | chr17:26113315-26123253 |
| 21634 | Tmem80 | NM_001141950.1 | chr7:141328129-141337155 |
| 21635 | Tmem81 | NM_029025.3 | chr1:132506229-132508639 |
| 21636 | Tmem82 | NM_145987.2 | chr4:141614232-141618633 |
| 21637 | Tmem86a | NM_026436.3 | chr7:47050639-47054776 |
| 21638 | Tmem86b | NM_023440.2 | chr7:4628039-4630482 |
| 21639 | Tmem87a | NM_001110496.1 | chr2:120355308-120404116 |
| 21640 | Tmem87b | NM_028248.2 | chr2:128818302-128854261 |
| 21641 | Tmem88 | NM_025915.4 | chr11:69396515-69398234 |
| 21642 | Tmem88b | NM_001033394.3 | chr4:155781590-155785874 |
| 21643 | Tmem89 | NM_027066.1 | chr9:108914618-108915563 |
| 21644 | Tmem8b | NM_001085508.2 | chr4:43668970-43692667 |
| 21645 | Tmem8c | NM_001159602.1 | chr2:27061635-27067868 |
| 21646 | Tmem9 | NM_001160145.1 | chr1:136008218-136035030 |
| 21647 | Tmem91 | NM_001290497.1 | chr7:25669139-25675166 |
| 21648 | Tmem92 | NM_001034896.2 | chr11:94777216-94782703 |
| 21649 | Tmem95 | NM_001195710.1 | chr11:69876683-69878018 |
| 21650 | Tmem97 | NM_133706.2 | chr11:78541816-78550735 |
| 21651 | Tmem98 | NM_029537.1 | chr11:80810414-80822033 |
| 21652 | Tmem9b | NM_020050.1 | chr7:109735835-109752263 |
| 21653 | Tmevpg1 | NR_104123.1 | chr10:118502034-118556525 |
| 21654 | Tmf1 | NM_001081111.2 | chr6:97151949-97179124 |
| 21655 | Tmie | NM_146260.2 | chr9:110866045-110880083 |
| 21656 | Tmigd1 | NM_025655.2 | chr11:76904544-76916586 |
| 21657 | Tmlhe | NM_138758.1 | chrX_GL456233_random:159646-334187 |
| 21658 | Tmod1 | NM_021883.2 | chr4:46039221-46116032 |
| 21659 | Tmod2 | NM_001038710.1 | chr9:75565621-75599133 |
| 21660 | Tmod3 | NM_016963.2 | chr9:75497783-75559657 |
| 21661 | Tmod4 | NM_016712.3 | chr3:95124513-95129208 |
| 21662 | Tmpo | NM_001080129.2 | chr10:91147570-91171619 |
| 21663 | Tmppe | NM_001200002.1 | chr9:114401094-114411201 |
| 21664 | Tmprss11a | NM_001033233.2 | chr5:86410409-86468990 |
| 21665 | Tmprss11bnl | NM_177024.4 | chr5:86659521-86676298 |
| 21666 | Tmprss11c | NM_001030297.2 | chr5:86231480-86289308 |
| 21667 | Tmprss11d | NM_145561.2 | chr5:86302853-86373387 |
| 21668 | Tmprss11e | NM_172880.2 | chr5:86705185-86745807 |
| 21669 | Tmprss11f | NM_178730.3 | chr5:86521225-86632424 |
| 21670 | Tmprss11g | NM_177162.4 | chr5:86485876-86518600 |
| 21671 | Tmprss12 | NM_183109.3 | chr15:100280836-100293053 |
| 21672 | Tmprss13 | NM_001013373.2 | chr9:45319099-45347580 |
| 21673 | Tmprss15 | NM_008941.3 | chr16:78953007-79091097 |
| 21674 | Tmprss2 | NM_015775.2 | chr16:97564681-97611195 |
| 21675 | Tmprss3 | NM_001163776.1 | chr17:31179267-31198974 |
| 21676 | Tmprss4 | NM_145403.2 | chr9:45172725-45204075 |
| 21677 | Tmprss5 | NM_030709.2 | chr9:49102778-49117587 |
| 21678 | Tmprss6 | NM_027902.2 | chr15:78439666-78468634 |
| 21679 | Tmprss7 | NM_172455.3 | chr16:45656316-45693658 |
| 21680 | Tmprss9 | NM_001081688.2 | chr10:80879815-80899494 |
| 21681 | Tmsb10 | NM_001039392.2 | chr6:72957346-72958232 |
| 21682 | Tmsb15a | NM_030106.2 | chrX:135718666-135720673 |
| 21683 | Tmsb15b1 | NM_001081983.1 | chrX:136974021-136976874 |
| 21684 | Tmsb15b2 | NM_001080967.4 | chrX:136954987-136957979 |
| 21685 | Tmsb15l | NM_207267.4 | chrX:136954987-136976874 |
| 21686 | Tmsb4x | NM_021278.2 | chrX:167207093-167209218 |
| 21687 | Tmtc1 | NM_198967.5 | chr6:148232429-148444352 |
| 21688 | Tmtc2 | NM_177368.4 | chr10:105187663-105574479 |
| 21689 | Tmtc3 | NM_001033332.2 | chr10:100448183-100487347 |
| 21690 | Tmtc4 | NM_028651.2 | chr14:122918974-122983261 |
| 21691 | Tmub1 | NM_022418.3 | chr5:24445462-24447846 |
| 21692 | Tmub2 | NM_028076.3 | chr11:102284938-102289427 |
| 21693 | Tmx1 | NM_028339.1 | chr12:70453153-70467624 |
| 21694 | Tmx2 | NM_001290751.1 | chr2:84671310-84678174 |
| 21695 | Tmx3 | NM_198295.2 | chr18:90510153-90543267 |
| 21696 | Tmx4 | NM_029148.1 | chr2:134594501-134644121 |
| 21697 | Tnc | NM_011607.3 | chr4:63959784-64047015 |
| 21698 | Tnf | NM_001278601.1 | chr17:35199366-35202007 |
| 21699 | Tnfaip1 | NM_001159392.1 | chr11:78522849-78536260 |
| 21700 | Tnfaip2 | NM_009396.2 | chr12:111442660-111455018 |
| 21701 | Tnfaip3 | NM_001166402.1 | chr10:19000909-19011963 |
| 21702 | Tnfaip6 | NM_009398.2 | chr2:52038112-52056681 |
| 21703 | Tnfaip8 | NM_001177759.1 | chr18:49979426-50093229 |
| 21704 | Tnfaip8l1 | NM_025566.3 | chr17:56162490-56173955 |
| 21705 | Tnfaip8l2 | NM_027206.2 | chr3:95139520-95142360 |
| 21706 | Tnfaip8l3 | NM_001033535.3 | chr9:54025605-54068411 |
| 21707 | Tnfrsf10b | NM_020275.4 | chr14:69767471-69784411 |
| 21708 | Tnfrsf11a | NM_009399.3 | chr1:105780722-105847981 |
| 21709 | Tnfrsf11b | NM_008764.3 | chr15:54250618-54278484 |
| 21710 | Tnfrsf12a | NM_001161746.1 | chr17:23675444-23677449 |
| 21711 | Tnfrsf13b | NM_021349.1 | chr11:61140834-61147642 |
| 21712 | Tnfrsf13c | NM_028075.2 | chr15:82221743-82224336 |
| 21713 | Tnfrsf14 | NM_178931.2 | chr4:154922209-154928077 |
| 21714 | Tnfrsf17 | NM_011608.1 | chr16:11313808-11320068 |
| 21715 | Tnfrsf18 | NM_009400.2 | chr4:156026341-156028891 |
| 21716 | Tnfrsf19 | NM_001164155.1 | chr14:60963833-61037987 |
| 21717 | Tnfrsf1a | NM_011609.4 | chr6:125349722-125362483 |
| 21718 | Tnfrsf1b | NM_011610.3 | chr4:145212367-145246870 |
| 21719 | Tnfrsf21 | NM_178589.3 | chr17:43016554-43089188 |
| 21720 | Tnfrsf22 | NM_023680.4 | chr7:143634807-143649664 |
| 21721 | Tnfrsf23 | NM_024290.4 | chr7:143665806-143685875 |
| 21722 | Tnfrsf25 | NM_001291010.1 | chr4:152115933-152120119 |
| 21723 | Tnfrsf26 | NM_175649.5 | chr7:143607684-143627845 |
| 21724 | Tnfrsf4 | NM_011659.2 | chr4:156013694-156016589 |
| 21725 | Tnfrsf8 | NM_009401.2 | chr4:145268975-145315147 |
| 21726 | Tnfrsf9 | NM_001077508.1 | chr4:150920154-150946102 |
| 21727 | Tnfsf10 | NM_009425.2 | chr3:27317076-27339665 |

Fig.21 - 113

| | | | | | | | |
|---|---|---|---|---|---|---|---|
| 21728 | Tnfsf11 | NM_011613.3 | chr14:78277445-78308043 | 21825 | Tpgs2 | NM_001004361.2 | chr18:25136038-25168877 |
| 21729 | Tnfsf12 | NM_011614.3 | chr11:69686239-69696098 | 21826 | Tph1 | NM_001136084.2 | chr7:46644640-46667377 |
| 21730 | Tnfsf12Tnfsf13 | NM_001034097.2 | chr11:69682576-69696098 | 21827 | Tph2 | NM_173391.3 | chr10:115078640-115185022 |
| 21731 | Tnfsf13 | NM_001159505.1 | chr11:69682576-69685554 | 21828 | Tpi1 | NM_009415.2 | chr6:124810591-124814296 |
| 21732 | Tnfsf13b | NM_033622.1 | chr8:10006632-10035999 | 21829 | Tpk1 | NM_013861.4 | chr6:43345000-43666278 |
| 21733 | Tnfsf14 | NM_019418.3 | chr17:57189473-57194181 | 21830 | Tpm1 | NM_001164248.1 | chr9:67027889-67049213 |
| 21734 | Tnfsf15 | NM_177371.3 | chr4:63724602-63745113 | 21831 | Tpm2 | NM_001277875.1 | chr4:43513725-43523554 |
| 21735 | Tnfsf18 | NM_183391.3 | chr1:161494656-161505290 | 21832 | Tpm3 | NM_001253738.1 | chr3:90079520-90100902 |
| 21736 | Tnfsf4 | NM_009452.2 | chr1:161395437-161418206 | 21833 | Tpm4 | NM_001001491.1 | chr8:72135291-72153129 |
| 21737 | Tnfsf8 | NM_009403.3 | chr4:63832823-63861284 | 21834 | Tpmt | NM_016785.2 | chr13:47023542-47043217 |
| 21738 | Tnfsf9 | NM_009404.3 | chr17:57105384-57107757 | 21835 | Tpo | NM_009417.3 | chr12:30054660-30132624 |
| 21739 | Tnik | NM_001163007.1 | chr3:28263213-28670585 | 21836 | Tpp1 | NM_009906.5 | chr7:105744846-105752207 |
| 21740 | Tnip1 | NM_001199275.2 | chr11:54910786-54962940 | 21837 | Tpp2 | NM_009418.3 | chr1:43933993-44003000 |
| 21741 | Tnip2 | NM_139064.2 | chr5:34496095-34513979 | 21838 | Tppp | NM_182839.2 | chr13:74009418-74035753 |
| 21742 | Tnip3 | NM_001001495.2 | chr6:65590397-65634040 | 21839 | Tppp2 | NM_001128634.1 | chr14:51918760-51920700 |
| 21743 | Tnk1 | NM_031880.3 | chr11:69850562-69858730 | 21840 | Tppp3 | NM_026481.3 | chr8:105467491-105471422 |
| 21744 | Tnk2 | NM_001110147.1 | chr16:32644642-32683493 | 21841 | Tpr | NM_133780.3 | chr1:150392837-150449935 |
| 21745 | Tnk2os | NR_033493.1 | chr16:32665222-32668197 | 21842 | Tpra1 | NM_011906.2 | chr6:88902250-88912240 |
| 21746 | Tnks | NM_175091.3 | chr8:34829178-34965690 | 21843 | Tprg | NM_175165.3 | chr16:25286816-25422344 |
| 21747 | Tnks1bp1 | NM_001081260.2 | chr2:85050459-85073048 | 21844 | Tprgl | NM_026388.2 | chr4:154157484-154160684 |
| 21748 | Tnks2 | NM_001163635.1 | chr19:36834231-36893477 | 21845 | Tprkb | NM_001170488.1 | chr6:85915718-85930284 |
| 21749 | Tnmd | NM_022322.2 | chrX:133851007-133865578 | 21846 | Tprn | NM_175286.4 | chr2:25262597-25269886 |
| 21750 | Tnn | NM_177839.3 | chr1:160085031-160153575 | 21847 | Tpsab1 | NM_031187.4 | chr17:25343244-25345562 |
| 21751 | Tnnc1 | NM_009393.2 | chr14:31208311-31211711 | 21848 | Tpsb2 | NM_010781.3 | chr17:25366332-25368092 |
| 21752 | Tnnc2 | NM_009394.2 | chr2:164777161-164779734 | 21849 | Tpsg1 | NM_012034.3 | chr17:25370552-25374442 |
| 21753 | Tnni1 | NM_001112702.1 | chr1:135799510-135810989 | 21850 | Tpst1 | NM_001130476.2 | chr5:130079369-130135733 |
| 21754 | Tnni2 | NM_009405.2 | chr7:142442467-142444405 | 21851 | Tpst2 | NM_009419.3 | chr5:112276706-112315356 |
| 21755 | Tnni3 | NM_009406.3 | chr7:4518307-4522443 | 21852 | Tpt1 | NM_009429.3 | chr14:75845255-75848303 |
| 21756 | Tnni3k | NM_177066.5 | chr3:154786290-155055407 | 21853 | Tpte | NM_199257.2 | chr8:22283440-22371418 |
| 21757 | Tnnt1 | NM_001277903.1 | chr7:4504569-4515975 | 21854 | Tpx2 | NM_001141975.1 | chr2:152847963-152895321 |
| 21758 | Tnnt2 | NM_001130174.2 | chr1:135836333-135852268 | 21855 | Tra2a | NM_198102.2 | chr6:49243920-49264052 |
| 21759 | Tnnt3 | NM_001163664.1 | chr7:142498835-142516009 | 21856 | Tra2b | NM_009186.4 | chr16:22245740-22265929 |
| 21760 | Tnp1 | NM_009407.2 | chr1:73015074-73015899 | 21857 | Trabd | NM_026485.2 | chr15:89076063-89087075 |
| 21761 | Tnp2 | NM_013694.4 | chr16:10787934-10788655 | 21858 | Trabd2b | NM_001085549.1 | chr4:114406723-114615098 |
| 21762 | Tnpo1 | NM_001048267.1 | chr13:98842080-98891042 | 21859 | Tradd | NM_001033161.2 | chr8:105258574-105264594 |
| 21763 | Tnpo2 | NM_001122843.1 | chr8:85037167-85057582 | 21860 | Traf1 | NM_009421.3 | chr2:34943257-34961772 |
| 21764 | Tnpo3 | NM_177296.4 | chr6:29540826-29609607 | 21861 | Traf2 | NM_001290413.1 | chr2:25517981-25546940 |
| 21765 | Tnr | NM_022312.3 | chr1:159523768-159924922 | 21862 | Traf3 | NM_001286122.1 | chr12:111166369-111267155 |
| 21766 | Tnrc18 | NM_001122730.2 | chr5:142724604-142817387 | 21863 | Traf3ip1 | NM_028718.2 | chr1:91494667-91529307 |
| 21767 | Tnrc6a | NM_144925.3 | chr7:123123884-123195296 | 21864 | Traf3ip2 | NM_134000.3 | chr10:39612933-39655307 |
| 21768 | Tnrc6b | NM_144812.2 | chr15:80711312-80941086 | 21865 | Traf3ip3 | NM_153137.4 | chr1:193175503-193201546 |
| 21769 | Tnrc6c | NM_198022.2 | chr11:117654288-117763441 | 21866 | Traf4 | NM_009423.4 | chr11:78158422-78165550 |
| 21770 | Tns1 | NM_001289895.1 | chr1:73910230-74124449 | 21867 | Traf5 | NM_011633.2 | chr1:191997202-192092599 |
| 21771 | Tns3 | NM_001083587.1 | chr11:8431651-8664535 | 21868 | Traf6 | NM_009424.3 | chr2:101678419-101701668 |
| 21772 | Tns4 | NM_172564.3 | chr11:99065677-99089306 | 21869 | Traf7 | NM_001172113.1 | chr17:24508849-24527938 |
| 21773 | Tnxb | NM_031176.2 | chr17:34670534-34719815 | 21870 | Trafd1 | NM_001163470.1 | chr5:121371724-121385615 |
| 21774 | Tob1 | NM_009427.2 | chr11:94211453-94215492 | 21871 | Traip | NM_011634.3 | chr9:107950962-107972268 |
| 21775 | Tob2 | NM_020507.3 | chr15:81848269-81858326 | 21872 | Trak1 | NM_175114.3 | chr9:121366957-121474918 |
| 21776 | Toe1 | NM_026654.2 | chr4:116804002-116807559 | 21873 | Trak2 | NM_172406.3 | chr1:58900449-58973482 |
| 21777 | Tollip | NM_023764.3 | chr7:141881584-141902406 | 21874 | Tram1 | NM_028173.5 | chr1:13564692-13589910 |
| 21778 | Tom1 | NM_001136259.1 | chr8:75033685-75070121 | 21875 | Tram1l1 | NM_146140.3 | chr3:124321036-124323260 |
| 21779 | Tom1l1 | NM_028011.2 | chr11:90645690-90687601 | 21876 | Tram2 | NM_177409.3 | chr1:21001396-21079225 |
| 21780 | Tom1l2 | NM_001039092.3 | chr11:60226713-60352905 | 21877 | Trank1 | NM_001164659.1 | chr9:111311738-111395775 |
| 21781 | Tomm20 | NM_024214.2 | chr8:126930663-126945921 | 21878 | Trap1 | NM_026508.2 | chr16:4039976-4077810 |
| 21782 | Tomm20l | NM_029227.1 | chr12:71111427-71123222 | 21879 | Trap1a | NM_011635.1 | chrX:139333682-139338165 |
| 21783 | Tomm22 | NM_172609.3 | chr15:79670867-79672862 | 21880 | Trappc1 | NM_001024206.2 | chr11:69323985-69325793 |
| 21784 | Tomm34 | NM_001291155.1 | chr2:164053539-164071169 | 21881 | Trappc10 | NM_001081055.1 | chr10:78186725-78244642 |
| 21785 | Tomm40 | NM_001109748.1 | chr7:19701312-19715429 | 21882 | Trappc11 | NM_177240.3 | chr8:47490127-47533470 |
| 21786 | Tomm40l | NM_001037170.2 | chr1:171217802-171222514 | 21883 | Trappc12 | NM_001161410.1 | chr12:28690627-28750394 |
| 21787 | Tomm5 | NM_001099675.1 | chr4:45105259-45108113 | 21884 | Trappc13 | NM_001093759.1 | chr13:104142152-104178466 |
| 21788 | Tomm6 | NM_001164729.1 | chr17:47686644-47688386 | 21885 | Trappc2 | NM_025432.4 | chrX:166440754-166453140 |
| 21789 | Tomm6os | NR_045945.1 | chr17:47687609-47691092 | 21886 | Trappc2l | NM_021502.2 | chr8:122611625-122615591 |
| 21790 | Tomm7 | NM_025394.3 | chr5:23838943-23844145 | 21887 | Trappc3 | NM_013718.2 | chr4:126262404-126275883 |
| 21791 | Tomm70a | NM_138599.5 | chr16:57121713-57154530 | 21888 | Trappc3l | NM_001162937.1 | chr10:34037596-34109815 |
| 21792 | Tomt | NM_001081679.1 | chr7:101899807-101906359 | 21889 | Trappc4 | NM_021789.2 | chr9:44403758-44407548 |
| 21793 | Tonsl | NM_183091.3 | chr15:76626236-76639929 | 21890 | Trappc5 | NM_025701.4 | chr8:3676476-3680921 |
| 21794 | Top1 | NM_009408.2 | chr2:160645896-160722763 | 21891 | Trappc6a | NM_025960.3 | chr7:19508728-19516145 |
| 21795 | Top1mt | NM_028404.2 | chr15:75657032-75678790 | 21892 | Trappc6b | NM_030057.3 | chr12:59043091-59061472 |
| 21796 | Top2a | NM_011623.2 | chr11:98992946-99024189 | 21893 | Trappc8 | NM_029491.2 | chr18:20846144-20896078 |
| 21797 | Top2b | NM_009409.2 | chr14:16365205-16430787 | 21894 | Trappc9 | NM_001164641.1 | chr15:72799523-73061204 |
| 21798 | Top3a | NM_009410.2 | chr11:60740058-60777365 | 21895 | Trat1 | NM_198297.3 | chr16:48734689-48771956 |
| 21799 | Top3b | NM_011624.2 | chr16:16870890-16892986 | 21896 | Trcg1 | NM_001014398.2 | chr9:57236555-57249864 |
| 21800 | Topaz1 | NM_001199736.1 | chr9:122747345-122802074 | 21897 | Trdmt1 | NM_010067.4 | chr2:13510160-13544664 |
| 21801 | Topbp1 | NM_176979.5 | chr9:103305326-103350427 | 21898 | Trdn | NM_029726.2 | chr10:33083482-33476709 |
| 21802 | Topors | NM_134097.3 | chr4:40259605-40269841 | 21899 | Treh | NM_001277847.1 | chr9:44673232-44686305 |
| 21803 | Toporsl | NM_026652.2 | chr4:52596273-52612163 | 21900 | Trem1 | NM_021406.5 | chr17:48232738-48246924 |
| 21804 | Toporsos | NR_045265.1 | chr4:40269578-40270221 | 21901 | Trem2 | NM_001272078.1 | chr17:48346400-48352276 |
| 21805 | Tor1a | NM_144884.2 | chr2:30960560-30967918 | 21902 | Trem3 | NM_021407.3 | chr17:48247728-48258847 |
| 21806 | Tor1aip1 | NM_001160018.1 | chr1:156004598-156036480 | 21903 | Treml1 | NM_001289451.1 | chr17:48359915-48367176 |
| 21807 | Tor1aip2 | NM_001160180.1 | chr1:156035726-156053736 | 21904 | Treml2 | NM_001033405.2 | chr17:48300037-48312534 |
| 21808 | Tor1b | NM_133673.3 | chr2:30953000-30959015 | 21905 | Treml4 | NM_001033922.2 | chr17:48264294-48275358 |
| 21809 | Tor2a | NM_152800.3 | chr2:32757233-32762244 | 21906 | Trerf1 | NM_001097623.1 | chr17:47140941-47359458 |
| 21810 | Tor3a | NM_023141.2 | chr1:156653616-156674339 | 21907 | Trex1 | NM_001012236.1 | chr9:109057931-109059251 |
| 21811 | Tor4a | NM_146115.4 | chr2:25192719-25196813 | 21908 | Trex2 | NM_011907.4 | chrX:73433704-73435343 |
| 21812 | Tox | NM_145711.4 | chr4:6687385-6990723 | 21909 | Trf | NM_133977.2 | chr9:103208875-103230286 |
| 21813 | Tox2 | NM_001098799.1 | chr2:163225453-163323102 | 21910 | Trh | NM_009426.3 | chr6:92242060-92244650 |
| 21814 | Tox3 | NM_172913.3 | chr8:90247111-90348252 | 21911 | Trhde | NM_146241.2 | chr10:114398820-114801370 |
| 21815 | Tox4 | NM_023434.3 | chr14:52279145-52295509 | 21912 | Trhr | NM_013696.2 | chr15:44196134-44235912 |
| 21816 | Tpbg | NM_001164792.1 | chr9:85842851-85847055 | 21913 | Trhr2 | NM_133202.2 | chr8:122356966-122360746 |
| 21817 | Tpbpa | NM_009411.3 | chr13:60938691-60941935 | 21914 | Triap1 | NM_026933.2 | chr5:115341246-115343552 |
| 21818 | Tpbpb | NM_026429.4 | chr13:60901294-60904847 | 21915 | Trib1 | NM_144549.4 | chr15:59648653-59657099 |
| 21819 | Tpcn1 | NM_145853.2 | chr5:120534156-120588613 | 21916 | Trib2 | NM_144551.5 | chr12:15791726-15816785 |
| 21820 | Tpcn2 | NM_146206.5 | chr7:145253922-145284011 | 21917 | Trib3 | NM_175093.2 | chr2:152337424-152344060 |
| 21821 | Tpd52 | NM_001025261.1 | chr3:8929435-8964054 | 21918 | Tril | NM_025817.4 | chr6:53815467-53820825 |
| 21822 | Tpd52l1 | NM_009413.1 | chr10:31332379-31445921 | 21919 | Trim10 | NM_011280.2 | chr17:36869573-36877833 |
| 21823 | Tpd52l2 | NM_001291197.1 | chr2:181497141-181517962 | 21920 | Trim11 | NM_001290988.1 | chr11:58978092-58991462 |
| 21824 | Tpgs1 | NM_148934.2 | chr10:79669409-79676126 | 21921 | Trim12a | NM_023835.2 | chr7:104299896-104315495 |

Fig.21 - 114

| | | | | | | | |
|---|---|---|---|---|---|---|---|
| 21922 | Trim12c | NM_001146007.1 | chr7:104338753-104353358 | 22019 | Trove2 | NM_013835.2 | chr1:143750790-143777051 |
| 21923 | Trim13 | NM_001164220.1 | chr14:61598225-61605945 | 22020 | Trp53 | NM_001127233.1 | chr11:69580358-69591873 |
| 21924 | Trim14 | NM_029077.4 | chr4:46505071-46536141 | 22021 | Trp53bp1 | NM_001290830.1 | chr2:121194834-121271407 |
| 21925 | Trim15 | NM_001024134.2 | chr17:36860690-36867187 | 22022 | Trp53bp2 | NM_173378.2 | chr1:182409166-182462436 |
| 21926 | Trim16 | NM_053169.2 | chr11:62820252-62842948 | 22023 | Trp53cor1 | NR_036469.2 | chr17:29057473-29079126 |
| 21927 | Trim17 | NM_031172.2 | chr11:58963780-58971729 | 22024 | Trp53i11 | NM_001025246.1 | chr2:93187583-93201757 |
| 21928 | Trim2 | NM_001271725.1 | chr3:84160438-84270783 | 22025 | Trp53i13 | NM_001024920.1 | chr11:77508098-77513273 |
| 21929 | Trim21 | NM_001082552.2 | chr7:102557919-102565482 | 22026 | Trp53inp1 | NM_001199105.1 | chr4:11156440-11174377 |
| 21930 | Trim23 | NM_030731.3 | chr13:104179097-104202048 | 22027 | Trp53inp2 | NM_178111.3 | chr2:155381855-155389847 |
| 21931 | Trim24 | NM_001272064.1 | chr6:37870810-37968445 | 22028 | Trp53rk | NM_023815.4 | chr2:166793766-166799492 |
| 21932 | Trim25 | NM_009546.2 | chr11:88999402-89020293 | 22029 | Trp53tg5 | NM_001271575.1 | chr2:164470303-164473724 |
| 21933 | Trim26 | NM_001025599.3 | chr17:36837145-36859398 | 22030 | Trp63 | NM_001127259.1 | chr16:25683764-25892088 |
| 21934 | Trim27 | NM_009054.3 | chr13:21179930-21194723 | 22031 | Trp73 | NM_001126330.1 | chr4:154056248-154097173 |
| 21935 | Trim28 | NM_011588.3 | chr7:13024151-13031032 | 22032 | Trpa1 | NM_177781.4 | chr1:14872647-14918862 |
| 21936 | Trim29 | NM_023655.2 | chr9:43310762-43336125 | 22033 | Trpc1 | NM_011643.3 | chr9:95705066-95750358 |
| 21937 | Trim3 | NM_001285870.1 | chr7:105610647-105633571 | 22034 | Trpc2 | NM_001109897.2 | chr7:102083115-102096864 |
| 21938 | Trim30a | NM_009099.2 | chr7:104409025-104465193 | 22035 | Trpc3 | NM_019510.2 | chr3:36620481-36690167 |
| 21939 | Trim30b | NM_175648.2 | chr7:104355397-104358646 | 22036 | Trpc4 | NM_001253682.1 | chr3:54156056-54318471 |
| 21940 | Trim30d | NM_001167828.1 | chr7:104470013-104507849 | 22037 | Trpc4ap | NM_001163452.1 | chr2:155634276-155692384 |
| 21941 | Trim30e-ps1 | NR_033673.1 | chr7:104532958-104535361 | 22038 | Trpc5 | NM_009428.3 | chrX:144377326-144688180 |
| 21942 | Trim31 | NM_146077.2 | chr17:36898129-36910217 | 22039 | Trpc5os | NM_001195579.1 | chrX:144456581-144477063 |
| 21943 | Trim32 | NM_001161782.1 | chr4:65604985-65616240 | 22040 | Trpc6 | NM_001282086.1 | chr9:8544141-8680741 |
| 21944 | Trim33 | NM_001079830.2 | chr3:103279292-103358770 | 22041 | Trpc7 | NM_012035.3 | chr13:56773097-56895789 |
| 21945 | Trim34a | NM_030684.3 | chr7:104244456-104262236 | 22042 | Trpd52l3 | NM_025741.2 | chr19:30003789-30006020 |
| 21946 | Trim34b | NM_001243916.1 | chr7:104329470-104336617 | 22043 | Trpm1 | NM_001039104.2 | chr7:64153834-64269759 |
| 21947 | Trim35 | NM_029979.3 | chr14:66297024-66311424 | 22044 | Trpm2 | NM_138301.2 | chr10:77907721-77969872 |
| 21948 | Trim36 | NM_001170855.1 | chr18:46165299-46198818 | 22045 | Trpm3 | NM_001035239.2 | chr19:22139116-22989882 |
| 21949 | Trim37 | NM_197987.2 | chr11:87127074-87220686 | 22046 | Trpm4 | NM_175130.4 | chr7:45303154-45333780 |
| 21950 | Trim38 | NM_001029935.2 | chr13:23782540-23791528 | 22047 | Trpm5 | NM_020277.2 | chr7:143071528-143094642 |
| 21951 | Trim39 | NM_024468.2 | chr17:36258872-36272004 | 22048 | Trpm6 | NM_153417.1 | chr19:18749982-18892511 |
| 21952 | Trim40 | NM_001033235.3 | chr17:36881597-36890125 | 22049 | Trpm7 | NM_001164325.1 | chr2:126791557-126876261 |
| 21953 | Trim41 | NM_145377.2 | chr11:48806403-48817391 | 22050 | Trpm8 | NM_134252.3 | chr1:88306711-88388851 |
| 21954 | Trim42 | NM_030219.2 | chr9:97349561-97369958 | 22051 | Trps1 | NM_032000.2 | chr15:50654758-50890041 |
| 21955 | Trim43a | NM_001034906.2 | chr9:88581035-88588819 | 22052 | Trpt1 | NM_153597.2 | chr19:6996130-6999046 |
| 21956 | Trim43b | NM_001170884.1 | chr9:89084623-89092835 | 22053 | Trpv1 | NM_001001445.2 | chr11:73234148-73261322 |
| 21957 | Trim43c | NM_001177858.1 | chr9:88839163-88848190 | 22054 | Trpv2 | NM_011706.2 | chr11:62574485-62600305 |
| 21958 | Trim44 | NM_020267.2 | chr2:102300118-102400900 | 22055 | Trpv3 | NM_145099.2 | chr11:73267619-73297200 |
| 21959 | Trim45 | NM_001165952.1 | chr3:100922492-100936925 | 22056 | Trpv4 | NM_022017.3 | chr5:114622153-114658421 |
| 21960 | Trim46 | NM_001039466.1 | chr3:89234176-89245199 | 22057 | Trpv5 | NM_001007572.2 | chr6:41652769-41680723 |
| 21961 | Trim47 | NM_001205081.1 | chr11:116105749-116110235 | 22058 | Trpv6 | NM_022413.4 | chr6:41620618-41636405 |
| 21962 | Trim50 | NM_178240.2 | chr5:135353295-135367654 | 22059 | Trrap | NM_001081362.1 | chr5:144768791-144859773 |
| 21963 | Trim52 | NM_198601.3 | chr14:106106197-106109280 | 22060 | Trub1 | NM_028115.3 | chr19:57452905-57491005 |
| 21964 | Trim54 | NM_021447.2 | chr5:31116611-31137626 | 22061 | Trub2 | NM_001290495.1 | chr2:29774683-29787671 |
| 21965 | Trim55 | NM_001081281.1 | chr3:19644459-19691599 | 22062 | Try10 | NM_001038996.2 | chr6:41354104-41357944 |
| 21966 | Trim56 | NM_201373.4 | chr5:137111285-137116207 | 22063 | Try4 | NM_011646.5 | chr6:41302271-41305533 |
| 21967 | Trim58 | NM_001039047.1 | chr11:58640464-58652404 | 22064 | Try5 | NM_001003405.4 | chr6:41311231-41314710 |
| 21968 | Trim59 | NM_025863.3 | chr3:69035293-69044742 | 22065 | Tsacc | NM_029801.2 | chr3:88282759-88296838 |
| 21969 | Trim6 | NM_001013616.2 | chr7:104218794-104235152 | 22066 | Tsc1 | NM_001289575.1 | chr2:28641232-28691172 |
| 21970 | Trim60 | NM_153097.2 | chr8:64998447-65018688 | 22067 | Tsc2 | NM_001039363.2 | chr17:24595815-24632627 |
| 21971 | Trim61 | NM_001177551.1 | chr8:65012975-65018688 | 22068 | Tsc22d1 | NM_001177751.2 | chr14:76488435-76507765 |
| 21972 | Trim62 | NM_178110.2 | chr4:128884139-128911326 | 22069 | Tsc22d2 | NM_001081229.1 | chr3:58415688-58466787 |
| 21973 | Trim63 | NM_001039048.2 | chr4:134315119-134329629 | 22070 | Tsc22d3 | NM_001077364.1 | chrX:140539528-140600522 |
| 21974 | Trim65 | NM_178802.4 | chr11:116124707-116131128 | 22071 | Tsc22d4 | NM_023910.6 | chr5:137745968-137759747 |
| 21975 | Trim66 | NM_001170912.1 | chr7:109449000-109508134 | 22072 | Tsen15 | NM_025677.3 | chr1:152370735-152386682 |
| 21976 | Trim67 | NM_198632.2 | chr8:124793018-124834704 | 22073 | Tsen2 | NM_199033.1 | chr6:115544703-115578336 |
| 21977 | Trim68 | NM_198012.3 | chr7:102677584-102687327 | 22074 | Tsen34 | NM_001164204.1 | chr7:3693609-3701035 |
| 21978 | Trim69 | NM_080510.2 | chr2:122160699-122179027 | 22075 | Tsen54 | NM_029557.1 | chr11:115814738-115823102 |
| 21979 | Trim7 | NM_053166.2 | chr11:48826137-48850195 | 22076 | Tsfm | NM_025537.3 | chr10:127022331-127030814 |
| 21980 | Trim71 | NM_001042503.2 | chr9:114511272-114564369 | 22077 | Tsg101 | NM_021884.3 | chr7:46889026-46919930 |
| 21981 | Trim72 | NM_001079932.3 | chr7:128004377-128011393 | 22078 | Tsga10 | NM_001290720.1 | chr1:37760823-37865298 |
| 21982 | Trim75 | NM_001033429.2 | chr8:64981650-64987644 | 22079 | Tsga13 | NM_054073.2 | chr6:30896980-30915573 |
| 21983 | Trim8 | NM_053100.2 | chr19:46501647-46516455 | 22080 | Tsga8 | NM_021898.2 | chrX:83486675-83487924 |
| 21984 | Trim9 | NM_001110202.1 | chr12:70244534-70347614 | 22081 | Tshb | NM_001165939.1 | chr3:102777397-102782714 |
| 21985 | Triml1 | NM_177742.4 | chr8:43129806-43141486 | 22082 | Tshr | NM_001113404.1 | chr12:91400992-91522541 |
| 21986 | Triml2 | NM_001160412.1 | chr8:43183121-43193884 | 22083 | Tshz1 | NM_001081300.1 | chr18:84011626-84086562 |
| 21987 | Trio | NM_001081302.1 | chr15:27730648-28025848 | 22084 | Tshz2 | NM_080455.2 | chr2:169633645-169888504 |
| 21988 | Triobp | NM_001024716.1 | chr15:78983055-79005864 | 22085 | Tshz3 | NM_172298.2 | chr7:36698117-36773457 |
| 21989 | Trip10 | NM_001242389.1 | chr17:57249450-57263697 | 22086 | Tsix | NR_002844.2 | chrX:103431516-103484957 |
| 21990 | Trip11 | NM_028446.1 | chr12:101837371-101913171 | 22087 | Tsks | NM_001077591.1 | chr7:44943239-44958037 |
| 21991 | Trip12 | NM_133975.4 | chr1:84721188-84839304 | 22088 | Tsku | NM_001024619.3 | chr7:98350667-98361288 |
| 21992 | Trip13 | NM_027182.2 | chr13:73912461-73937767 | 22089 | Tslp | NM_021367.2 | chr18:32815382-32819799 |
| 21993 | Trip4 | NM_001170907.1 | chr9:65828925-65885173 | 22090 | Tsn | NM_011650.3 | chr1:118298517-118311132 |
| 21994 | Trip6 | NM_011639.3 | chr5:137309898-137314241 | 22091 | Tsnax | NM_016909.2 | chr8:125012996-125034192 |
| 21995 | Triqk | NM_001171801.1 | chr4:12906836-12981485 | 22092 | Tsnaxip1 | NM_024445.4 | chr8:105827743-105844676 |
| 21996 | Trit1 | NM_025873.2 | chr4:123016596-123054934 | 22093 | Tspan1 | NM_133681.4 | chr4:116161880-116167598 |
| 21997 | Trmt1 | NM_001164559.1 | chr8:84689246-84699808 | 22094 | Tspan10 | NM_145363.2 | chr11:120442630-120447321 |
| 21998 | Trmt10a | NM_175389.4 | chr3:138143537-138159820 | 22095 | Tspan11 | NM_026743.3 | chr6:127887621-127953031 |
| 21999 | Trmt10b | NM_027266.4 | chr4:45297167-45316131 | 22096 | Tspan12 | NM_173007.3 | chr6:21771394-21852515 |
| 22000 | Trmt10c | NM_029092.3 | chr16:56033719-56037774 | 22097 | Tspan13 | NM_025359.3 | chr12:36014554-36042478 |
| 22001 | Trmt11 | NM_028604.2 | chr10:30534224-30600749 | 22098 | Tspan14 | NM_145928.2 | chr14:40906443-40966807 |
| 22002 | Trmt112 | NM_001166370.1 | chr19:6909697-6911026 | 22099 | Tspan15 | NM_197996.2 | chr10:62185395-62231218 |
| 22003 | Trmt12 | NM_026642.2 | chr15:58872648-58876781 | 22100 | Tspan17 | NM_028841.3 | chr13:54789404-54796775 |
| 22004 | Trmt13 | NM_030016.2 | chr3:116581335-116614587 | 22101 | Tspan18 | NM_183180.2 | chr2:93201759-93334487 |
| 22005 | Trmt1l | NM_026876.3 | chr1:151428647-151458183 | 22102 | Tspan2 | NM_001243132.1 | chr3:102735214-102772310 |
| 22006 | Trmt2a | NM_001080999.2 | chr16:18248882-18254772 | 22103 | Tspan2os | NR_040588.1 | chr3:102720230-102735417 |
| 22007 | Trmt2b | NM_001167994.1 | chrX:134222954-134276984 | 22104 | Tspan3 | NM_019793.3 | chr9:56135883-56161070 |
| 22008 | Trmt44 | NM_030208.3 | chr5:35556208-35575070 | 22105 | Tspan31 | NM_025982.4 | chr10:127067289-127070261 |
| 22009 | Trmt5 | NM_029580.3 | chr12:73280409-73286711 | 22106 | Tspan32 | NM_001128080.2 | chr7:143005045-143019485 |
| 22010 | Trmt6 | NM_175113.3 | chr2:132804214-132816054 | 22107 | Tspan33 | NM_146173.3 | chr6:29694213-29718558 |
| 22011 | Trmt61a | NM_001099792.1 | chr12:111678104-111683902 | 22108 | Tspan4 | NM_001252588.1 | chr7:141476379-141493427 |
| 22012 | Trmt61b | NR_015549.1 | chr17:71557026-71598761 | 22109 | Tspan5 | NM_019571.5 | chr3:138742207-138904433 |
| 22013 | Trmu | NM_028063.2 | chr15:85879326-85897393 | 22110 | Tspan6 | NM_019656.3 | chrX:133891069-133898429 |
| 22014 | Trnau1ap | NM_027925.3 | chr4:132311762-132329538 | 22111 | Tspan7 | NM_019634.2 | chrX:10485115-10596604 |
| 22015 | Trnp1 | NM_001081156.2 | chr4:133491099-133498550 | 22112 | Tspan8 | NM_001168679.1 | chr10:115817283-115849893 |
| 22016 | Trnt1 | NM_001242358.1 | chr6:106769137-106782474 | 22113 | Tspan9 | NM_175414.4 | chr6:127961399-128143578 |
| 22017 | Tro | NM_001002272.3 | chrX:150644709-150657436 | 22114 | Tspear | NM_001287074.1 | chr10:77686568-77886769 |
| 22018 | Troap | NM_001162506.1 | chr15:99074972-99083409 | 22115 | Tspo | NM_009775.4 | chr15:83563572-83574203 |

Fig.21 - 115

| | | | | | | | |
|---|---|---|---|---|---|---|---|
| 22116 | Tspo2 | NM_027292.2 | chr17:48448434-48451501 | 22213 | Tuba3a | NM_009446.2 | chr6:125278273-125286042 |
| 22117 | Tspyl1 | NM_009433.3 | chr10:34282189-34284885 | 22214 | Tuba3b | NM_009449.3 | chr6:145615962-145621477 |
| 22118 | Tspyl2 | NM_029836.3 | chrX:152336851-152342484 | 22215 | Tuba4a | NM_009447.4 | chr1:75214971-75219294 |
| 22119 | Tspyl3 | NM_198617.2 | chr2:153222369-153225441 | 22216 | Tuba8 | NM_017379.2 | chr6:121210733-121226098 |
| 22120 | Tspyl4 | NM_030203.2 | chr10:34297420-34301320 | 22217 | Tubal3 | NM_001033879.3 | chr13:3924694-3935277 |
| 22121 | Tspyl5 | NM_001085421.1 | chr15:33683874-33687883 | 22218 | Tubb1 | NM_001080971.2 | chr2:174450594-174458380 |
| 22122 | Tspy-ps | NR_027507.1 | chrY:1055763-1058868 | 22219 | Tubb2a | NM_009450.2 | chr13:34074279-34078008 |
| 22123 | Tsr1 | NM_177325.3 | chr11:74898079-74909340 | 22220 | Tubb2a-ps2 | NR_003964.2 | chr12:11882195-11882899 |
| 22124 | Tsr2 | NM_001164578.1 | chrX:151087093-151096543 | 22221 | Tubb2b | NM_023716.2 | chr13:34127007-34130354 |
| 22125 | Tsr3 | NM_001163718.1 | chr17:25240169-25242799 | 22222 | Tubb3 | NM_023279.2 | chr8:123411563-123422010 |
| 22126 | Tssc1 | NM_201357.2 | chr12:28751827-28867491 | 22223 | Tubb4a | NM_009451.3 | chr17:57080065-57087782 |
| 22127 | Tssc4 | NM_001115085.1 | chr7:143069367-143071087 | 22224 | Tubb4b | NM_146116.2 | chr2:25222157-25224702 |
| 22128 | Tssk1 | NM_009435.2 | chr16:17894202-17895653 | 22225 | Tubb5 | NM_011655.5 | chr17:35833919-35838301 |
| 22129 | Tssk2 | NM_009436.2 | chr16:17898636-17900024 | 22226 | Tubb6 | NM_026473.2 | chr18:67390730-67402749 |
| 22130 | Tssk3 | NM_080442.2 | chr4:129489007-129490770 | 22227 | Tubd1 | NM_001199045.1 | chr11:86544990-86567360 |
| 22131 | Tssk4 | NM_001253888.1 | chr14:55650183-55652539 | 22228 | Tube1 | NM_028006.2 | chr10:39134022-39151058 |
| 22132 | Tssk5 | NM_183099.2 | chr15:76371957-76374938 | 22229 | Tubg1 | NM_134024.2 | chr11:101120130-101126419 |
| 22133 | Tssk6 | NM_032004.1 | chr8:69902214-69903518 | 22230 | Tubg2 | NM_134028.2 | chr11:101155883-101161787 |
| 22134 | Tst | NM_009437.4 | chr15:78399555-78405859 | 22231 | Tubgcp2 | NM_001286007.1 | chr7:139995954-140036674 |
| 22135 | Tsta3 | NM_031201.1 | chr15:75924682-75929730 | 22232 | Tubgcp3 | NM_198031.1 | chr8:12614276-12672100 |
| 22136 | Tstd1 | NM_001164525.1 | chr1:171419032-171420352 | 22233 | Tubgcp4 | NM_001290824.1 | chr2:121171167-121198770 |
| 22137 | Tstd2 | NM_173033.3 | chr4:46114745-46138475 | 22234 | Tubgcp5 | NM_146190.2 | chr7:55794147-55831447 |
| 22138 | Tstd3 | NM_029840.1 | chr4:21757381-21767211 | 22235 | Tubgcp6 | NM_001163319.1 | chr15:89099097-89123150 |
| 22139 | Tsx | NM_009440.2 | chrX:103414466-103424583 | 22236 | Tufm | NM_001163713.1 | chr7:126487354-126490731 |
| 22140 | Ttbk1 | NM_001162864.1 | chr17:46442447-46487675 | 22237 | Tuft1 | NM_011656.3 | chr3:94612752-94658872 |
| 22141 | Ttbk2 | NM_001024856.2 | chr2:120732816-120850418 | 22238 | Tug1 | NR_002321.2 | chr11:3639784-3648814 |
| 22142 | Ttc1 | NM_133795.1 | chr11:43730005-43747973 | 22239 | Tulp1 | NM_021478.1 | chr17:28351518-28365143 |
| 22143 | Ttc12 | NM_172770.3 | chr9:49436960-49486225 | 22240 | Tulp2 | NM_001045555.2 | chr7:45513705-45522927 |
| 22144 | Ttc13 | NM_145607.3 | chr8:124671326-124721975 | 22241 | Tulp3 | NM_011657.2 | chr6:128321160-128355851 |
| 22145 | Ttc14 | NM_001290500.1 | chr3:33800182-33814860 | 22242 | Tulp4 | NM_001103181.1 | chr17:6106829-6240637 |
| 22146 | Ttc16 | NM_001290563.1 | chr2:32757025-32775633 | 22243 | Tunar | NR_045047.1 | chr12:105336593-105383932 |
| 22147 | Ttc17 | NM_183106.2 | chr2:94300765-94406689 | 22244 | Tusc1 | NM_026954.1 | chr4:93334147-93335511 |
| 22148 | Ttc18 | NM_001163638.1 | chr14:20394189-20452225 | 22245 | Tusc2 | NM_019742.4 | chr9:107563254-107566108 |
| 22149 | Ttc19 | NM_028360.2 | chr11:62281472-62316424 | 22246 | Tusc3 | NM_030254.3 | chr8:39005866-39130817 |
| 22150 | Ttc21a | NM_028735.3 | chr9:119937605-119967793 | 22247 | Tusc5 | NM_177709.3 | chr11:76679872-76698664 |
| 22151 | Ttc21b | NM_001047604.2 | chr2:66184326-66256617 | 22248 | Tut1 | NM_197993.2 | chr19:8953849-8966210 |
| 22152 | Ttc22 | NM_177667.4 | chr4:106622448-106640187 | 22249 | Tvp23a | NM_001013778.1 | chr16:10420558-10447350 |
| 22153 | Ttc23 | NM_001168475.1 | chr7:67647409-67726576 | 22250 | Tvp23b | NM_026210.4 | chr11:62879489-62895184 |
| 22154 | Ttc23l | NM_029430.1 | chr15:10503946-10558668 | 22251 | Twf1 | NM_008971.4 | chr15:94577947-94589824 |
| 22155 | Ttc24 | NM_172526.3 | chr3:88069409-88078304 | 22252 | Twf2 | NM_011876.3 | chr9:106203107-106215387 |
| 22156 | Ttc25 | NM_028918.2 | chr11:100545631-100572566 | 22253 | Twist1 | NM_011658.2 | chr12:33957670-33959831 |
| 22157 | Ttc26 | NM_153600.2 | chr6:38381523-38427647 | 22254 | Twist2 | NM_007855.3 | chr1:91801460-91848034 |
| 22158 | Ttc27 | NM_152817.3 | chr17:74717749-74863570 | 22255 | Twistnb | NM_172253.2 | chr12:33429623-33439380 |
| 22159 | Ttc28 | NM_001267622.1 | chr5:110879802-111289779 | 22256 | Twsg1 | NM_023053.3 | chr17:65923064-65951187 |
| 22160 | Ttc29 | NM_183096.3 | chr8:78213341-78394326 | 22257 | Txk | NM_001122754.2 | chr5:72695977-72752777 |
| 22161 | Ttc3 | NM_009441.2 | chr16:94370738-94469221 | 22258 | Txlna | NM_001005506.3 | chr4:129626076-129640805 |
| 22162 | Ttc30a1 | NM_030188.3 | chr2:75979105-75981967 | 22259 | Txlnb | NM_138628.3 | chr10:17796218-17845663 |
| 22163 | Ttc30a2 | NM_001081228.1 | chr2:75976171-75978179 | 22260 | Txlng | NM_001290776.1 | chrX:162778916-162829454 |
| 22164 | Ttc30b | NM_028235.1 | chr2:75935849-75938462 | 22261 | Txn1 | NM_011660.3 | chr4:57943372-57956411 |
| 22165 | Ttc32 | NM_029321.2 | chr12:9029996-9036394 | 22262 | Txn2 | NM_019913.5 | chr15:77915050-77928994 |
| 22166 | Ttc33 | NM_026213.3 | chr15:5185559-5218332 | 22263 | Txndc11 | NM_029582.2 | chr16:11074910-11134532 |
| 22167 | Ttc34 | NM_172878.3 | chr4:154856199-154867127 | 22264 | Txndc12 | NM_025334.3 | chr4:108834677-108862119 |
| 22168 | Ttc36 | NM_138951.1 | chr9:44799399-44802951 | 22265 | Txndc15 | NM_175150.3 | chr13:55714649-55726228 |
| 22169 | Ttc37 | NM_001081352.1 | chr13:76098733-76187983 | 22266 | Txndc16 | NM_172597.3 | chr14:45134447-45219425 |
| 22170 | Ttc38 | NM_001033337.4 | chr15:85832303-85858822 | 22267 | Txndc17 | NM_026559.2 | chr11:72207553-72210487 |
| 22171 | Ttc39a | NM_001145948.1 | chr4:109415646-109444745 | 22268 | Txndc2 | NM_001146002.1 | chr17:65637504-65642204 |
| 22172 | Ttc39b | NM_027238.2 | chr4:83220300-83324189 | 22269 | Txndc5 | NM_001289598.1 | chr13:38500270-38528824 |
| 22173 | Ttc39c | NM_028341.4 | chr18:12643532-12737052 | 22270 | Txndc8 | NM_026132.2 | chr4:57984028-58009124 |
| 22174 | Ttc39d | NM_026351.2 | chr17:80215913-80217936 | 22271 | Txndc9 | NM_172054.4 | chr1:37983866-37997208 |
| 22175 | Ttc4 | NM_001172073.1 | chr4:106661807-106678686 | 22272 | Txnip | NM_001009935.2 | chr3:96557956-96561857 |
| 22176 | Ttc5 | NM_001080949.2 | chr14:50765408-50785520 | 22273 | Txnl1 | NM_016792.4 | chr18:63662800-63692359 |
| 22177 | Ttc7 | NM_028639.3 | chr17:87282885-87381770 | 22274 | Txnl4a | NM_001038608.2 | chr18:80206797-80212732 |
| 22178 | Ttc7b | NM_001033213.1 | chr12:100300769-100520822 | 22275 | Txnl4b | NM_175646.3 | chr8:109565985-109574051 |
| 22179 | Ttc8 | NM_029553.3 | chr12:98920573-98983238 | 22276 | Txnrd1 | NM_001042513.1 | chr10:82859205-82897724 |
| 22180 | Ttc9 | NM_001033149.3 | chr12:81631248-81667557 | 22277 | Txnrd2 | NM_013711.3 | chr16:18426416-18479073 |
| 22181 | Ttc9b | NM_028417.1 | chr7:27653923-27656207 | 22278 | Txnrd3 | NM_001178058.1 | chr6:89643987-89675529 |
| 22182 | Ttc9c | NM_027412.3 | chr19:8809074-8819294 | 22279 | Tyk2 | NM_001205312.1 | chr9:21104067-21131275 |
| 22183 | Ttf1 | NM_009442.2 | chr2:29060262-29087650 | 22280 | Tymp | NM_138302.1 | chr15:89371930-89377037 |
| 22184 | Ttf2 | NM_001013026.2 | chr3:100938859-100969663 | 22281 | Tyms | NM_021288.4 | chr5:30058199-30073625 |
| 22185 | Tti1 | NM_029282.1 | chr2:157981802-158009360 | 22282 | Tyms-ps | NR_000040.2 | chr10:87966670-87968017 |
| 22186 | Tti2 | NM_001199988.1 | chr8:31150315-31164703 | 22283 | Tyr | NM_011661.4 | chr7:87427404-87493411 |
| 22187 | Ttk | NM_001110265.1 | chr9:83834688-83869493 | 22284 | Tyro3 | NM_001290800.1 | chr2:119797739-119818103 |
| 22188 | Ttl | NM_027192.2 | chr2:129065946-129096283 | 22285 | Tyrobp | NM_011662.2 | chr7:30413787-30417579 |
| 22189 | Ttll1 | NM_178869.4 | chr15:83483768-83510907 | 22286 | Tyrp1 | NM_001282014.1 | chr4:80834212-80851736 |
| 22190 | Ttll10 | NM_029264.2 | chr4:156034836-156050817 | 22287 | Tysnd1 | NM_001272090.1 | chr10:61695513-61702773 |
| 22191 | Ttll11 | NM_029774.2 | chr2:35751225-35979624 | 22288 | Tyw1 | NM_001015876.2 | chr5:130257036-130341567 |
| 22192 | Ttll12 | NM_183017.2 | chr15:83575093-83595157 | 22289 | Tyw3 | NM_001168358.1 | chr3:154576519-154597098 |
| 22193 | Ttll13 | NM_177765.3 | chr7:80246375-80260821 | 22290 | Tyw5 | NM_001037742.2 | chr1:57388243-57406674 |
| 22194 | Ttll2 | NM_001098267.1 | chr17:7350903-7352696 | 22291 | U2af1 | NM_001163769.1 | chr17:31647081-31658754 |
| 22195 | Ttll3 | NM_001142732.1 | chr6:113389259-113399448 | 22292 | U2af1l4 | NM_170760.3 | chr7:30563339-30565364 |
| 22196 | Ttll4 | NM_001014974.1 | chr1:74661753-74697973 | 22293 | U2af2 | NM_001205231.1 | chr7:5062142-5079945 |
| 22197 | Ttll5 | NM_001081423.2 | chr12:85824949-86053760 | 22294 | U2surp | NM_001114977.1 | chr9:95456893-95511996 |
| 22198 | Ttll6 | NM_172799.4 | chr11:96133785-96165452 | 22295 | U90926 | NR_033483.1 | chr5:92209894-92215408 |
| 22199 | Ttll7 | NM_001302957.1 | chr3:146852136-146984009 | 22296 | Uaca | NM_028283.2 | chr9:60794547-60880370 |
| 22200 | Ttll8 | NM_172818.3 | chr15:88913897-88954418 | 22297 | Uap1 | NM_133806.5 | chr1:170141222-170174964 |
| 22201 | Ttll9 | NM_001083618.1 | chr2:152962484-153008482 | 22298 | Uap1l1 | NM_001033293.2 | chr2:25361491-25365626 |
| 22202 | Ttn | NM_011652.3 | chr2:76703983-76982547 | 22299 | Uba1 | NM_001136085.2 | chrX:20662895-20683179 |
| 22203 | Ttpa | NM_015767.3 | chr4:20008427-20030785 | 22300 | Uba1y | NM_011667.2 | chrY:818712-844224 |
| 22204 | Ttpal | NM_029512.2 | chr2:163602313-163619013 | 22301 | Uba2 | NM_016682.2 | chr7:34140696-34168529 |
| 22205 | Ttr | NM_013697.5 | chr18:20665249-20674326 | 22302 | Uba3 | NM_001111106.2 | chr6:97183631-97205647 |
| 22206 | Ttyh1 | NM_001001454.4 | chr7:4119529-4135407 | 22303 | Uba5 | NM_025692.3 | chr9:104046587-104063121 |
| 22207 | Ttyh2 | NM_053273.2 | chr11:114675467-114720984 | 22304 | Uba52 | NM_019883.3 | chr8:70508265-70510367 |
| 22208 | Ttyh3 | NM_175274.5 | chr5:140620576-140649031 | 22305 | Uba6 | NM_172712.2 | chr5:86110729-86172743 |
| 22209 | Tub | NM_021885.4 | chr7:109010879-109034459 | 22306 | Uba7 | NM_023738.4 | chr9:107975566-107984056 |
| 22210 | Tuba1a | NM_011653.2 | chr15:98949846-98953501 | 22307 | Ubac1 | NM_133835.2 | chr2:25996957-26021760 |
| 22211 | Tuba1b | NM_011654.2 | chr15:98931430-98934390 | 22308 | Ubac2 | NM_026861.2 | chr14:121878605-122021035 |
| 22212 | Tuba1c | NM_009448.4 | chr15:99029890-99038105 | 22309 | Ubald1 | NM_145359.2 | chr16:4874778-4879851 |

Fig.21 - 116

| | | | | | | | |
|---|---|---|---|---|---|---|---|
| 22310 | Ubald2 | NM_176902.3 | chr11:116434093-116439077 | 22407 | Ucma | NM_001113558.2 | chr2:4976121-4985748 |
| 22311 | Ubap1 | NM_001290454.1 | chr4:41348995-41389766 | 22408 | Ucn | NM_021290.2 | chr5:31137988-31138895 |
| 22312 | Ubap1l | NM_001111145.1 | chr9:65361059-65380377 | 22409 | Ucn2 | NM_145077.1 | chr9:108986162-108987164 |
| 22313 | Ubap2 | NM_026872.1 | chr4:41194314-41275135 | 22410 | Ucn3 | NM_031250.5 | chr13:3940687-3945349 |
| 22314 | Ubap2l | NM_001165983.1 | chr3:90000287-90052475 | 22411 | Ucp1 | NM_009463.3 | chr8:83290347-83298456 |
| 22315 | Ubash3a | NM_177823.4 | chr17:31208065-31242403 | 22412 | Ucp2 | NM_011671.5 | chr7:100493357-100499629 |
| 22316 | Ubash3b | NM_176860.5 | chr9:41013640-41157494 | 22413 | Ucp3 | NM_009464.3 | chr7:100472990-100486432 |
| 22317 | Ubb | NM_011664.4 | chr11:62551170-62553213 | 22414 | Uevld | NM_001040695.1 | chr7:46923215-46958518 |
| 22318 | Ubc | NM_019639.4 | chr5:125385964-125390017 | 22415 | Ufc1 | NM_025388.2 | chr1:171288563-171294982 |
| 22319 | Ubd | NM_023137.3 | chr17:37193891-37196101 | 22416 | Ufd1l | NM_011672.4 | chr16:18812293-18835261 |
| 22320 | Ube2a | NM_019668.4 | chrX:36874294-36884220 | 22417 | Ufl1 | NM_026194.4 | chr4:25248585-25281821 |
| 22321 | Ube2b | NM_009458.4 | chr11:51985145-52000466 | 22418 | Ufm1 | NM_026435.5 | chr3:53853375-53863807 |
| 22322 | Ube2c | NM_026785.2 | chr2:164769928-164772902 | 22419 | Ufsp1 | NM_027356.2 | chr5:137294668-137295664 |
| 22323 | Ube2cbp | NM_027394.2 | chr9:86307233-86464916 | 22420 | Ufsp2 | NM_138668.2 | chr8:45975527-45996956 |
| 22324 | Ube2d1 | NM_145420.2 | chr10:71254979-71285262 | 22421 | Ugcg | NM_011673.3 | chr4:59189549-59222833 |
| 22325 | Ube2d2a | NM_019912.2 | chr18:35771558-35807172 | 22422 | Ugdh | NM_009466.2 | chr5:65413221-65435842 |
| 22326 | Ube2d2b | NM_001276397.1 | chr5:107830161-107831777 | 22423 | Uggt1 | NM_198899.2 | chr1:36140027-36244302 |
| 22327 | Ube2d3 | NM_025356.4 | chr3:135438758-135467178 | 22424 | Uggt2 | NM_001081252.2 | chr14:118984984-119099434 |
| 22328 | Ube2dnl1 | NM_001276396.1 | chrX:114905011-114905941 | 22425 | Ugp2 | NM_001290634.1 | chr11:21321125-21371267 |
| 22329 | Ube2dnl2 | NM_001081661.1 | chrX:114907581-114908510 | 22426 | Ugt1a1 | NM_201645.2 | chr1:88211958-88220002 |
| 22330 | Ube2e1 | NM_009455.3 | chr14:18282728-18331844 | 22427 | Ugt1a10 | NM_201641.2 | chr1:88055410-88220002 |
| 22331 | Ube2e2 | NM_144839.1 | chr14:18573576-18893627 | 22428 | Ugt1a2 | NM_013701.3 | chr1:88200610-88220002 |
| 22332 | Ube2e3 | NM_009454.2 | chr2:78869046-78920583 | 22429 | Ugt1a5 | NM_201643.2 | chr1:88166011-88220002 |
| 22333 | Ube2f | NM_026454.3 | chr1:91250318-91286025 | 22430 | Ugt1a6a | NM_145079.3 | chr1:88134808-88220002 |
| 22334 | Ube2g1 | NM_025985.4 | chr11:72607260-72686481 | 22431 | Ugt1a6b | NM_201410.3 | chr1:88103256-88218998 |
| 22335 | Ube2g2 | NM_019803.3 | chr10:77622320-77645990 | 22432 | Ugt1a7c | NM_201642.4 | chr1:88095000-88220002 |
| 22336 | Ube2h | NM_001169576.1 | chr6:30211289-30304539 | 22433 | Ugt1a9 | NM_201644.2 | chr1:88070778-88220002 |
| 22337 | Ube2i | NM_001177609.1 | chr17:25260510-25273914 | 22434 | Ugt2a1 | NM_053184.2 | chr5:87459489-87490871 |
| 22338 | Ube2j1 | NM_019586.3 | chr4:33031424-33052364 | 22435 | Ugt2a2 | NM_001024148.1 | chr5:87459492-87482258 |
| 22339 | Ube2j2 | NM_001039157.2 | chr4:155943812-155959604 | 22436 | Ugt2a3 | NM_028094.3 | chr5:87324971-87337195 |
| 22340 | Ube2k | NM_016786.4 | chr5:65537244-65598989 | 22437 | Ugt2b1 | NM_152811.1 | chr5:86916638-86926503 |
| 22341 | Ube2l3 | NM_009456.2 | chr16:17152014-17201492 | 22438 | Ugt2b34 | NM_153598.2 | chr5:86889769-86906937 |
| 22342 | Ube2l6 | NM_019949.2 | chr2:84798827-84810003 | 22439 | Ugt2b35 | NM_172881.3 | chr5:87000859-87013274 |
| 22343 | Ube2m | NM_001168469.2 | chr7:13035119-13038275 | 22440 | Ugt2b36 | NM_001029867.1 | chr5:87065926-87092555 |
| 22344 | Ube2n | NM_080560.3 | chr10:95515161-95545658 | 22441 | Ugt2b37 | NM_053215.3 | chr5:87240491-87254788 |
| 22345 | Ube2o | NM_173755.3 | chr11:116537752-116581447 | 22442 | Ugt2b38 | NM_133894.2 | chr5:87409939-87424203 |
| 22346 | Ube2q1 | NM_027315.4 | chr3:89773608-89783997 | 22443 | Ugt2b5 | NM_009467.3 | chr5:87124946-87140340 |
| 22347 | Ube2q2 | NM_180600.3 | chr9:55149368-55207529 | 22444 | Ugt3a1 | NM_207216.2 | chr15:9279828-9315036 |
| 22348 | Ube2ql1 | NM_001145162.1 | chr13:69702831-69739897 | 22445 | Ugt3a2 | NM_144845.3 | chr15:9335597-9370870 |
| 22349 | Ube2r2 | NM_026275.4 | chr4:41136020-41193370 | 22446 | Ugt8a | NM_011674.4 | chr3:125865342-125938550 |
| 22350 | Ube2s | NM_133777.2 | chr7:4808013-4812340 | 22447 | Uhmk1 | NM_010633.3 | chr1:170199255-170215393 |
| 22351 | Ube2t | NM_001278115.1 | chr1:134962564-134974179 | 22448 | Uhrf1 | NM_001111078.1 | chr17:56304312-56323486 |
| 22352 | Ube2u | NM_001033773.4 | chr4:100478866-100550145 | 22449 | Uhrf1bp1 | NM_001080769.1 | chr17:27856506-27900040 |
| 22353 | Ube2v1 | NM_023230.2 | chr2:167607638-167632005 | 22450 | Uhrf1bp1l | NM_029166.2 | chr10:89744990-89819869 |
| 22354 | Ube2v2 | NM_001159351.1 | chr16:15550985-15594518 | 22451 | Uhrf2 | NM_144873.2 | chr19:30030512-30093724 |
| 22355 | Ube2w | NM_001271016.1 | chr1:16540787-16619338 | 22452 | Uimc1 | NM_011307.2 | chr13:55027879-55100295 |
| 22356 | Ube2z | NM_172300.3 | chr11:96047430-96065364 | 22453 | Ulbp1 | NM_029975.2 | chr10:7444872-7473477 |
| 22357 | Ube3a | NM_001033962.1 | chr7:59228751-59306727 | 22454 | Ulk1 | NM_009469.3 | chr5:110784488-110810081 |
| 22358 | Ube3b | NM_054093.2 | chr5:114380606-114421166 | 22455 | Ulk2 | NM_013881.4 | chr11:61775597-61855092 |
| 22359 | Ube3c | NM_133907.3 | chr5:29569241-29676077 | 22456 | Ulk3 | NM_027895.1 | chr9:57589451-57596233 |
| 22360 | Ube4a | NM_145400.3 | chr9:44923126-44965600 | 22457 | Ulk4 | NM_177589.3 | chr9:120964453-121277172 |
| 22361 | Ube4b | NM_022022.3 | chr4:149328415-149426631 | 22458 | Umod | NM_001278605.1 | chr7:119462707-119479262 |
| 22362 | Ubfd1 | NM_138589.2 | chr7:122067197-122082199 | 22459 | Umodl1 | NM_177465.4 | chr17:30954682-31010710 |
| 22363 | Ubiad1 | NM_027873.2 | chr4:148434496-148444751 | 22460 | Umps | NM_009471.2 | chr16:33955011-33967003 |
| 22364 | Ubl3 | NM_011908.2 | chr5:148504630-148552788 | 22461 | Unc119 | NM_011676.3 | chr11:78343494-78349164 |
| 22365 | Ubl4 | NM_145405.2 | chrX:74365717-74368548 | 22462 | Unc119b | NM_175352.4 | chr5:115122565-115134975 |
| 22366 | Ubl4b | NM_026261.2 | chr3:107553697-107555073 | 22463 | Unc13a | NM_001029873.2 | chr8:71626711-71671757 |
| 22367 | Ubl5 | NM_025401.3 | chr9:20643317-20646789 | 22464 | Unc13b | NM_001081413.2 | chr4:43058952-43264873 |
| 22368 | Ubl7 | NM_001122873.1 | chr9:57910985-57929968 | 22465 | Unc13c | NM_001081153.1 | chr9:73479423-73933567 |
| 22369 | Ublcp1 | NM_024475.5 | chr11:44454570-44470548 | 22466 | Unc13d | NM_001009573.2 | chr11:116062095-116077961 |
| 22370 | Ubn1 | NM_026666.3 | chr16:5050067-5086285 | 22467 | Unc45a | NM_133952.2 | chr7:80325291-80340219 |
| 22371 | Ubn2 | NM_177185.4 | chr6:38433924-38512763 | 22468 | Unc45b | NM_178680.4 | chr11:82911252-82943406 |
| 22372 | Ubox5 | NM_001255993.1 | chr2:130589995-130630038 | 22469 | Unc50 | NM_026123.3 | chr1:37430171-37439124 |
| 22373 | Ubp1 | NM_001083319.1 | chr9:113930933-113977201 | 22470 | Unc5a | NM_153131.3 | chr13:54949431-55006018 |
| 22374 | Ubqln1 | NM_026842.4 | chr13:58176155-58215653 | 22471 | Unc5b | NM_029770.2 | chr10:60762594-60831581 |
| 22375 | Ubqln2 | NM_018798.2 | chrX:153498231-153501558 | 22472 | Unc5c | NM_009472.4 | chr3:141465563-141834924 |
| 22376 | Ubqln3 | NM_198623.2 | chr7:104140622-104143272 | 22473 | Unc5cl | NM_152823.4 | chr17:48454900-48468684 |
| 22377 | Ubqln4 | NM_033526.2 | chr3:88553715-88569725 | 22474 | Unc5d | NM_153135.3 | chr8:28646716-29219636 |
| 22378 | Ubqlnl | NM_198624.3 | chr7:104148258-104150556 | 22475 | Unc79 | NM_001081017.2 | chr12:102948858-103183997 |
| 22379 | Ubr1 | NM_009461.2 | chr2:120860275-120970715 | 22476 | Unc80 | NM_175510.3 | chr1:66468446-66699148 |
| 22380 | Ubr2 | NM_001177374.1 | chr17:46928290-47010532 | 22477 | Unc93a | NM_199252.2 | chr17:13108616-13131791 |
| 22381 | Ubr3 | NM_001303033.1 | chr2:69896969-70024010 | 22478 | Unc93b1 | NM_001161428.1 | chr19:3935185-3949340 |
| 22382 | Ubr4 | NM_001160319.1 | chr4:139380658-139489532 | 22479 | Uncx | NM_013702.3 | chr5:139543897-139548178 |
| 22383 | Ubr5 | NM_001081359.3 | chr15:37967327-38078854 | 22480 | Ung | NM_001040691.1 | chr5:114130434-114139321 |
| 22384 | Ubr7 | NM_025666.2 | chr12:102757974-102777701 | 22481 | Unk | NM_001286006.1 | chr11:116030315-116061214 |
| 22385 | Ubtd1 | NM_145500.3 | chr19:41981762-42034641 | 22482 | Unkl | NM_001197024.1 | chr17:25188399-25234442 |
| 22386 | Ubtd2 | NM_173784.3 | chr11:32455371-32518709 | 22483 | Uox | NM_009474.5 | chr3:146597148-146631483 |
| 22387 | Ubtf | NM_001044383.2 | chr11:102304562-102317287 | 22484 | Upb1 | NM_133995.4 | chr10:75406910-75441679 |
| 22388 | Ubtfl1 | NM_001033793.3 | chr9:18404417-18411502 | 22485 | Upf1 | NM_001122829.1 | chr8:70331521-70353273 |
| 22389 | Ubxn1 | NM_146093.1 | chr19:8871558-8875656 | 22486 | Upf2 | NM_001081132.1 | chr2:5951468-6056703 |
| 22390 | Ubxn10 | NM_001285928.1 | chr4:138718525-138737167 | 22487 | Upf3a | NM_025924.2 | chr8:13785614-13798537 |
| 22391 | Ubxn11 | NM_026257.3 | chr4:134102582-134126780 | 22488 | Upf3b | NM_026573.3 | chrX:37091677-37110322 |
| 22392 | Ubxn2a | NM_145441.3 | chr12:4879031-4907520 | 22489 | Upk1a | NM_026815.2 | chr7:30603091-30612734 |
| 22393 | Ubxn2b | NM_026534.2 | chr4:6191104-6219788 | 22490 | Upk1b | NM_178924.4 | chr16:38773183-38800203 |
| 22394 | Ubxn4 | NM_026390.2 | chr1:128244180-128279377 | 22491 | Upk2 | NM_009476.2 | chr9:44452714-44454767 |
| 22395 | Ubxn6 | NM_024432.2 | chr17:56068252-56074989 | 22492 | Upk3a | NM_023478.2 | chr15:85017140-85022560 |
| 22396 | Ubxn7 | NM_177633.4 | chr16:32332251-32393747 | 22493 | Upk3b | NM_175309.4 | chr5:136038495-136044993 |
| 22397 | Ubxn8 | NM_178648.2 | chr8:33619585-33641976 | 22494 | Upk3bl | NM_027158.1 | chr5:136057266-136064324 |
| 22398 | Uchl1 | NM_011670.2 | chr5:66676120-66687234 | 22495 | Upp1 | NM_001159401.1 | chr11:9118007-9136170 |
| 22399 | Uchl1os | NR_102714.1 | chr5:66626494-66676497 | 22496 | Upp2 | NM_001289659.1 | chr2:58755183-58791242 |
| 22400 | Uchl3 | NM_016723.2 | chr14:101653966-101696125 | 22497 | Uprt | NM_001081189.1 | chrX:104482781-104506262 |
| 22401 | Uchl4 | NM_033607.1 | chr9:64235200-64236362 | 22498 | Uqcc1 | NM_018888.4 | chr2:155846885-155930310 |
| 22402 | Uchl5 | NM_001159866.1 | chr1:143777277-143807466 | 22499 | Uqcc2 | NM_026063.2 | chr17:27122664-27133891 |
| 22403 | Uck1 | NM_011675.2 | chr2:32255001-32260105 | 22500 | Uqcr10 | NM_197979.2 | chr11:4701967-4704344 |
| 22404 | Uck2 | NM_030724.3 | chr1:167226083-167285127 | 22501 | Uqcr11 | NM_025650.2 | chr10:80402996-80406821 |
| 22405 | Uckl1 | NM_026765.3 | chr2:181569152-181581973 | 22502 | Uqcrb | NM_026219.1 | chr13:66900620-66905350 |
| 22406 | Uckl1os | NR_027289.1 | chr2:181578479-181585115 | 22503 | Uqcrc1 | NM_025407.2 | chr9:108936647-108949641 |

Fig.21 - 117

| | | | |
|---|---|---|---|
| 22504 | Uqcrc2 | NM_025899.2 | chr7:120635188-120659523 |
| 22505 | Uqcrfs1 | NM_025710.2 | chr13:30540311-30545316 |
| 22506 | Uqcrh | NM_025641.3 | chr4:116066964-116075070 |
| 22507 | Uqcrq | NM_025352.2 | chr11:53428947-53430831 |
| 22508 | Urad | NM_001039678.2 | chr5:147314983-147322440 |
| 22509 | Urah | NM_029821.2 | chr7:140835495-140837968 |
| 22510 | Urb1 | NM_029497.1 | chr16:90751526-90810413 |
| 22511 | Urb2 | NM_001029876.1 | chr8:124023472-124048504 |
| 22512 | Urgcp | NM_001077661.1 | chr11:5713416-5762376 |
| 22513 | Uri1 | NM_011274.5 | chr7:37959991-38019552 |
| 22514 | Urm1 | NM_026615.4 | chr2:29827388-29844996 |
| 22515 | Uroc1 | NM_144940.2 | chr6:90333288-90364548 |
| 22516 | Urod | NM_009478.4 | chr4:116989964-116994413 |
| 22517 | Uros | NM_009479.3 | chr7:133686354-133709295 |
| 22518 | Usb1 | NM_133954.2 | chr8:95332283-95347513 |
| 22519 | Use1 | NM_001145780.1 | chr8:71366847-71369732 |
| 22520 | Usf1 | NM_009480.3 | chr1:171411680-171418759 |
| 22521 | Usf2 | NM_011680.2 | chr7:30945247-30956803 |
| 22522 | Ush1c | NM_001163733.1 | chr7:46195350-46238490 |
| 22523 | Ush1g | NM_176847.3 | chr11:115315191-115321918 |
| 22524 | Ush2a | NM_021408.3 | chr1:188262837-188965039 |
| 22525 | Ushbp1 | NM_181418.3 | chr8:71384273-71395801 |
| 22526 | Usmg5 | NM_023211.4 | chr19:47083470-47090625 |
| 22527 | Uso1 | NM_019490.1 | chr5:92137937-92202795 |
| 22528 | Usp1 | NM_146144.4 | chr4:98923809-98935542 |
| 22529 | Usp10 | NM_009462.2 | chr8:119910359-119957559 |
| 22530 | Usp11 | NM_145628.4 | chrX:20703908-20720539 |
| 22531 | Usp12 | NM_011669.3 | chr5:146734811-146794956 |
| 22532 | Usp13 | NM_001013024.2 | chr3:32817625-32935257 |
| 22533 | Usp14 | NM_001038589.2 | chr18:9993614-10030149 |
| 22534 | Usp15 | NM_027604.4 | chr10:123105005-123197019 |
| 22535 | Usp16 | NM_024258.2 | chr16:87454984-87483513 |
| 22536 | Usp17la | NM_007887.2 | chr7:104857015-104862667 |
| 22537 | Usp17lb | NM_201409.2 | chr7:104840311-104842504 |
| 22538 | Usp17lc | NM_010089.3 | chr7:103416695-103419174 |
| 22539 | Usp17ld | NM_001001559.2 | chr7:103250085-103252505 |
| 22540 | Usp17le | NM_001256973.1 | chr7:104768048-104777470 |
| 22541 | Usp18 | NM_011909.2 | chr6:121245905-121270917 |
| 22542 | Usp19 | NM_001168371.2 | chr9:108490675-108502337 |
| 22543 | Usp2 | NM_016808.2 | chr9:44069428-44095627 |
| 22544 | Usp20 | NM_028846.5 | chr2:30982278-31022655 |
| 22545 | Usp21 | NM_013919.4 | chr1:171281952-171287961 |
| 22546 | Usp22 | NM_001004143.4 | chr11:61151780-61175059 |
| 22547 | Usp24 | NM_183225.2 | chr4:106316212-106441327 |
| 22548 | Usp25 | NM_013918.2 | chr16:77014068-77116780 |
| 22549 | Usp26 | NM_031388.2 | chrX:51753958-51801233 |
| 22550 | Usp27x | NM_019461.4 | chrX:7372590-7375830 |
| 22551 | Usp28 | NM_175482.3 | chr9:48985384-49042517 |
| 22552 | Usp29 | NM_001290994.1 | chr7:6730582-6967219 |
| 22553 | Usp3 | NM_144937.4 | chr9:66514638-66593115 |
| 22554 | Usp30 | NM_001033202.3 | chr5:114100332-114122924 |
| 22555 | Usp31 | NM_001033173.1 | chr7:121642020-121707253 |
| 22556 | Usp32 | NM_001029934.1 | chr11:84984487-85139955 |
| 22557 | Usp33 | NM_001076676.2 | chr3:152346477-152393614 |
| 22558 | Usp34 | NM_001190401.2 | chr11:23306894-23490560 |
| 22559 | Usp35 | NM_001177412.1 | chr7:97309379-97325964 |
| 22560 | Usp36 | NM_001033528.1 | chr11:118259652-118290244 |
| 22561 | Usp37 | NM_176972.4 | chr1:74435509-74544286 |
| 22562 | Usp38 | NM_027554.2 | chr8:80980732-81014906 |
| 22563 | Usp39 | NM_138592.4 | chr6:72318675-72345175 |
| 22564 | Usp4 | NM_011678.2 | chr9:108347830-108392529 |
| 22565 | Usp40 | NM_001033291.2 | chr1:87945120-88008551 |
| 22566 | Usp42 | NM_029749.2 | chr5:143710325-143732280 |
| 22567 | Usp43 | NM_001291049.1 | chr11:67854522-67922153 |
| 22568 | Usp44 | NM_001206851.1 | chr10:93831554-93858087 |
| 22569 | Usp45 | NM_001290425.1 | chr4:21776269-21837872 |
| 22570 | Usp46 | NM_177561.3 | chr5:74000037-74068411 |
| 22571 | Usp47 | NM_133758.3 | chr7:112023505-112111386 |
| 22572 | Usp48 | NM_130879.2 | chr4:137594188-137658537 |
| 22573 | Usp49 | NM_198421.1 | chr17:47630689-47684067 |
| 22574 | Usp5 | NM_013700.1 | chr6:124815018-124829447 |
| 22575 | Usp50 | NM_029163.3 | chr2:126761049-126783460 |
| 22576 | Usp51 | NM_001137547.1 | chrX:153006468-153009459 |
| 22577 | Usp53 | NM_133857.3 | chr3:122933600-122984447 |
| 22578 | Usp54 | NM_030180.2 | chr14:20548911-20618354 |
| 22579 | Usp6nl | NM_001080548.1 | chr2:6322756-6443820 |
| 22580 | Usp7 | NM_001003918.2 | chr16:8688721-8738342 |
| 22581 | Usp8 | NM_001252580.1 | chr2:126707327-126759314 |
| 22582 | Usp9x | NM_009481.2 | chrX:13071497-13173327 |
| 22583 | Usp9y | NM_148943.2 | chrY:1298960-1459782 |
| 22584 | Uspl1 | NM_001013378.2 | chr5:149184559-149215434 |
| 22585 | Ust | NM_177387.3 | chr10:8204752-8518825 |
| 22586 | Utf1 | NM_009482.2 | chr7:139943855-139945112 |
| 22587 | Utp11l | NM_026031.3 | chr4:124678763-124693554 |
| 22588 | Utp14a | NM_028276.1 | chrX:48256933-48282449 |
| 22589 | Utp14b | NM_001001981.3 | chr1:78657824-78667601 |
| 22590 | Utp15 | NM_178918.3 | chr13:98246844-98262992 |
| 22591 | Utp18 | NM_001013375.1 | chr11:93859242-93885766 |
| 22592 | Utp20 | NM_175158.3 | chr10:88746606-88826814 |
| 22593 | Utp23 | NM_030132.5 | chr15:51877440-51884622 |
| 22594 | Utp3 | NM_023054.1 | chr5:88554482-88556083 |
| 22595 | Utp6 | NM_144826.3 | chr11:79933955-79962387 |
| 22596 | Utrn | NM_011682.4 | chr10:12382187-12861735 |
| 22597 | Uts2 | NM_011910.2 | chr4:150997096-151001810 |
| 22598 | Uts2b | NM_198166.3 | chr16:27353321-27370239 |
| 22599 | Uts2r | NM_145440.1 | chr11:121160270-121161973 |
| 22600 | Uty | NM_009484.2 | chrY:1097143-1245738 |
| 22601 | Uvrag | NM_178635.3 | chr7:98886742-99141144 |
| 22602 | Uvssa | NM_001081101.2 | chr5:33378695-33419754 |
| 22603 | Uxs1 | NM_026430.3 | chr1:43750230-43827708 |
| 22604 | Uxt | NM_013840.3 | chrX:20951664-20961978 |
| 22605 | V1ra8 | NM_053223.1 | chr6:90202816-90203656 |
| 22606 | V1rd18 | NM_207618.2 | chr7:24003090-24004247 |
| 22607 | V1rd19 | NM_207619.2 | chr7:24003110-24004028 |
| 22608 | Vac14 | NM_146216.2 | chr8:110618637-110720398 |
| 22609 | Vamp1 | NM_001080557.1 | chr6:125215580-125222306 |
| 22610 | Vamp2 | NM_009497.3 | chr11:69088527-69092381 |
| 22611 | Vamp3 | NM_009498.4 | chr4:151047304-151057953 |
| 22612 | Vamp4 | NM_016796.3 | chr1:162570827-162599078 |
| 22613 | Vamp5 | NM_001080742.2 | chr6:72368048-72380468 |
| 22614 | Vamp7 | NM_011515.5 | chrX_GL456233_random:10736-39191 |
| 22615 | Vamp8 | NM_016794.3 | chr6:72385220-72390667 |
| 22616 | Vangl1 | NM_177545.5 | chr3:102155899-102205011 |
| 22617 | Vangl2 | NM_033509.4 | chr1:172000959-172027295 |
| 22618 | Vapa | NM_013933.3 | chr17:65580052-65613555 |
| 22619 | Vapb | NM_019806.5 | chr2:173737570-173784336 |
| 22620 | Vars | NM_011690.3 | chr17:35000906-35016329 |
| 22621 | Vars2 | NM_175137.4 | chr17:35655634-35667592 |
| 22622 | Vash1 | NM_177354.4 | chr12:86678699-86695681 |
| 22623 | Vash2 | NM_144879.2 | chr1:190947645-190978998 |
| 22624 | Vasn | NM_139307.3 | chr16:4639944-4651166 |
| 22625 | Vasp | NM_001282021.1 | chr7:19256929-19271854 |
| 22626 | Vat1 | NM_012037.2 | chr11:101458747-101466199 |
| 22627 | Vat1l | NM_173016.3 | chr8:114205639-114374070 |
| 22628 | Vaultrc5 | NR_027885.1 | chr18:36801762-36802107 |
| 22629 | Vav1 | NM_001163815.1 | chr17:57279099-57329236 |
| 22630 | Vav2 | NM_009500.1 | chr2:27263634-27426825 |
| 22631 | Vav3 | NM_020505.2 | chr3:109340682-109685694 |
| 22632 | Vax1 | NM_009501.1 | chr19:59166186-59170029 |
| 22633 | Vax2 | NM_011912.3 | chr6:83711263-83738304 |
| 22634 | Vax2os | NR_002871.1 | chr6:83692805-83712201 |
| 22635 | Vbp1 | NM_011692.2 | chrX:75514296-75534946 |
| 22636 | Vcam1 | NM_011693.3 | chr3:116110019-116129688 |
| 22637 | Vcan | NM_001081249.1 | chr13:89655309-89742512 |
| 22638 | Vcl | NM_009502.4 | chr14:20929432-21033673 |
| 22639 | Vcp | NM_009503.4 | chr4:42979963-43000507 |
| 22640 | Vcpip1 | NM_173443.2 | chr1:9718621-9748382 |
| 22641 | Vcpkmt | NM_001033236.2 | chr12:69577627-69583028 |
| 22642 | Vdac1 | NM_011694.5 | chr11:52360861-52389397 |
| 22643 | Vdac2 | NM_011695.2 | chr14:21831560-21845879 |
| 22644 | Vdac3 | NM_001198998.1 | chr8:22577074-22593813 |
| 22645 | Vdr | NM_009504.4 | chr15:97854426-97908296 |
| 22646 | Vegfa | NM_001025250.3 | chr17:46016992-46032377 |
| 22647 | Vegfb | NM_001185164.1 | chr19:6982471-6987651 |
| 22648 | Vegfc | NM_009506.2 | chr8:54077531-54186454 |
| 22649 | Veph1 | NM_145820.3 | chr3:66053557-66296837 |
| 22650 | Vezf1 | NM_016686.4 | chr11:88068278-88084729 |
| 22651 | Vezt | NM_172538.5 | chr10:93961521-94035799 |
| 22652 | Vgf | NM_001039385.1 | chr5:137030294-137033351 |
| 22653 | Vgll1 | NM_133251.2 | chrX:57088105-57106540 |
| 22654 | Vgll2 | NM_153786.2 | chr10:52022501-52028471 |
| 22655 | Vgll3 | NM_028572.1 | chr16:65815632-65863066 |
| 22656 | Vgll4 | NM_177683.2 | chr6:114862091-114921752 |
| 22657 | Vhl | NM_009507.3 | chr6:113624020-113631633 |
| 22658 | Vil1 | NM_009509.2 | chr1:74409383-74435560 |
| 22659 | Vill | NM_001164567.1 | chr9:119052777-119071525 |
| 22660 | Vim | NM_011701.4 | chr2:13574310-13582826 |
| 22661 | Vimp | NM_024439.3 | chr7:66079648-66089405 |
| 22662 | Vip | NM_011702.3 | chr10:5639217-5647616 |
| 22663 | Vipas39 | NM_001142580.1 | chr12:87238874-87266286 |
| 22664 | Vipr1 | NM_011703.4 | chr9:121642715-121672954 |
| 22665 | Vipr2 | NM_009511.2 | chr12:116077725-116146261 |
| 22666 | Vit | NM_001197028.1 | chr17:78508062-78627409 |
| 22667 | Vkorc1 | NM_178600.2 | chr7:127893062-127895617 |
| 22668 | Vkorc1l1 | NM_001001327.2 | chr5:129942108-129986692 |
| 22669 | Vldlr | NM_001161420.1 | chr19:27217019-27254231 |
| 22670 | Vma21 | NM_001081356.3 | chrX:71816757-71824706 |
| 22671 | Vmac | NM_001166474.1 | chr17:56713931-56717699 |
| 22672 | Vmn1r1 | NM_001166728.1 | chr1:182157177-182158098 |
| 22673 | Vmn1r10 | NM_053231.2 | chr6:57113424-57114360 |
| 22674 | Vmn1r100 | NM_001166844.1 | chr7:20417941-22414506 |
| 22675 | Vmn1r101 | NM_001166836.1 | chr7:20441669-22438229 |
| 22676 | Vmn1r103 | NM_001166737.1 | chr7:20509616-20510534 |
| 22677 | Vmn1r104 | NM_001166738.1 | chr7:20533836-22523890 |
| 22678 | Vmn1r107 | NM_001166759.1 | chr7:20668917-23218255 |
| 22679 | Vmn1r11 | NM_053233.2 | chr6:57137352-57138252 |
| 22680 | Vmn1r112 | NM_001166847.1 | chr7:20771184-20772078 |
| 22681 | Vmn1r113 | NM_001166716.1 | chr7:20787284-20788208 |
| 22682 | Vmn1r114 | NM_001166837.1 | chr7:20811220-20812186 |
| 22683 | Vmn1r115 | NM_001166745.1 | chr7:20844097-20844985 |
| 22684 | Vmn1r116 | NM_001166744.1 | chr7:20872255-20873179 |
| 22685 | Vmn1r117 | NM_001166743.1 | chr7:20883197-20884121 |
| 22686 | Vmn1r118 | NM_001166742.1 | chr7:20232907-22229484 |
| 22687 | Vmn1r119 | NM_001166708.1 | chr7:21011531-21012455 |
| 22688 | Vmn1r12 | NM_001101579.1 | chr6:57158919-57159843 |
| 22689 | Vmn1r120 | NM_001166715.1 | chr7:21052866-21053784 |
| 22690 | Vmn1r121 | NM_001166741.1 | chr7:21097568-21098513 |
| 22691 | Vmn1r122 | NM_001166714.1 | chr7:21133210-21134128 |
| 22692 | Vmn1r123 | NM_001166707.1 | chr7:21162184-21163108 |
| 22693 | Vmn1r124 | NM_001166757.1 | chr7:21259693-21260617 |
| 22694 | Vmn1r125 | NM_001166740.1 | chr7:21272178-21273102 |
| 22695 | Vmn1r126 | NM_001166838.1 | chr7:21300501-21301467 |
| 22696 | Vmn1r127 | NM_001166726.1 | chr7:21318943-21319861 |
| 22697 | Vmn1r128 | NM_001166739.1 | chr7:21349372-21350296 |

Fig.21 - 118

| | | | |
|---|---|---|---|
| 22698 | Vmn1r129 | NM_001166725.1 | chr7:21360367-21361291 |
| 22699 | Vmn1r13 | NM_053235.2 | chr6:57209857-57210760 |
| 22700 | Vmn1r130 | NM_001166848.1 | chr7:21511780-21512703 |
| 22701 | Vmn1r132 | NM_001122682.1 | chr7:21612744-22097921 |
| 22702 | Vmn1r135 | NM_001166747.1 | chr7:21832350-23056463 |
| 22703 | Vmn1r137 | NM_001166849.1 | chr7:21994587-21995510 |
| 22704 | Vmn1r138 | NM_001167169.1 | chr7:22027977-22028897 |
| 22705 | Vmn1r139 | NM_001166748.1 | chr7:21614091-22097760 |
| 22706 | Vmn1r14 | NM_053237.2 | chr6:57233438-57234350 |
| 22707 | Vmn1r142 | NM_001166749.1 | chr7:20167459-22164035 |
| 22708 | Vmn1r148 | NM_030736.2 | chr7:22412230-22414668 |
| 22709 | Vmn1r15 | NM_053236.2 | chr6:57258148-57259048 |
| 22710 | Vmn1r151 | NM_001166712.1 | chr7:22498760-22499678 |
| 22711 | Vmn1r152 | NM_001166752.1 | chr7:20533836-22523890 |
| 22712 | Vmn1r157 | NM_001166754.1 | chr7:22761696-22762590 |
| 22713 | Vmn1r158 | NM_001166841.1 | chr7:22789858-22790782 |
| 22714 | Vmn1r159 | NM_001166758.1 | chr7:22842687-22843605 |
| 22715 | Vmn1r16 | NM_134184.2 | chr6:57322723-57323635 |
| 22716 | Vmn1r160 | NM_001166724.1 | chr7:22871223-22872147 |
| 22717 | Vmn1r163 | NM_001166755.1 | chr7:23055540-23056463 |
| 22718 | Vmn1r165 | NM_001166850.1 | chr7:23217332-23218255 |
| 22719 | Vmn1r166 | NM_001167168.1 | chr7:23250762-23251682 |
| 22720 | Vmn1r167 | NM_001101562.1 | chr7:23504641-23505589 |
| 22721 | Vmn1r168 | NM_001166842.1 | chr7:23540719-23541649 |
| 22722 | Vmn1r169 | NM_001166843.1 | chr7:23577184-23578099 |
| 22723 | Vmn1r17 | NM_134171.1 | chr6:57360466-57361378 |
| 22724 | Vmn1r170 | NM_001166722.1 | chr7:23606174-23607089 |
| 22725 | Vmn1r171 | NM_030737.2 | chr7:23631987-23633568 |
| 22726 | Vmn1r172 | NM_030735.1 | chr7:23658315-23660663 |
| 22727 | Vmn1r173 | NM_001166718.1 | chr7:23702341-23703283 |
| 22728 | Vmn1r174 | NM_207548.2 | chr7:23753910-23754852 |
| 22729 | Vmn1r175 | NM_001166727.1 | chr7:23808285-23809200 |
| 22730 | Vmn1r176 | NM_001166721.1 | chr7:23834811-23835726 |
| 22731 | Vmn1r177 | NM_206872.2 | chr7:23865519-23866449 |
| 22732 | Vmn1r178 | NM_206868.1 | chr7:23893528-23894443 |
| 22733 | Vmn1r179 | NM_207545.1 | chr7:23928385-23929357 |
| 22734 | Vmn1r18 | NM_134181.1 | chr6:57389667-57390567 |
| 22735 | Vmn1r180 | NM_206869.2 | chr7:23952386-23953356 |
| 22736 | Vmn1r181 | NM_207546.2 | chr7:23983960-23985048 |
| 22737 | Vmn1r183 | NM_203489.1 | chr7:24054773-24055691 |
| 22738 | Vmn1r184 | NM_001167540.1 | chr7:26266830-26267775 |
| 22739 | Vmn1r185 | NM_134231.1 | chr7:26611118-26612078 |
| 22740 | Vmn1r186 | NM_001167567.1 | chr7:94185-5676224 |
| 22741 | Vmn1r187 | NM_001167568.1 | chr7:149260-5803631 |
| 22742 | Vmn1r188 | NM_145850.1 | chr13:22087877-22088804 |
| 22743 | Vmn1r189 | NM_145844.1 | chr13:22101726-22102665 |
| 22744 | Vmn1r19 | NM_134182.1 | chr6:57404463-57405390 |
| 22745 | Vmn1r191 | NM_145849.1 | chr13:22178685-22179582 |
| 22746 | Vmn1r192 | NM_145845.1 | chr13:22187145-22188048 |
| 22747 | Vmn1r193 | NM_134225.1 | chr13:22218860-22219820 |
| 22748 | Vmn1r194 | NM_001080972.1 | chr13:22244214-22245105 |
| 22749 | Vmn1r195 | NM_134223.2 | chr13:22278316-22279404 |
| 22750 | Vmn1r196 | NM_001167541.1 | chr13:22293192-22294098 |
| 22751 | Vmn1r197 | NM_134244.1 | chr13:22327910-22328807 |
| 22752 | Vmn1r198 | NM_134220.1 | chr13:22354345-22355248 |
| 22753 | Vmn1r199 | NM_134213.1 | chr13:22382537-22383641 |
| 22754 | Vmn1r2 | NM_001167534.1 | chr4:3172082-3173003 |
| 22755 | Vmn1r20 | NM_001101533.1 | chr6:57431690-57432602 |
| 22756 | Vmn1r200 | NM_134212.1 | chr13:22395028-22395967 |
| 22757 | Vmn1r201 | NM_134221.1 | chr13:22474617-22475520 |
| 22758 | Vmn1r202 | NM_134224.1 | chr13:22501336-22502245 |
| 22759 | Vmn1r203 | NM_134236.1 | chr13:22524050-22524986 |
| 22760 | Vmn1r204 | NM_001045544.1 | chr13:22556200-22557109 |
| 22761 | Vmn1r205 | NM_134217.1 | chr13:22591979-22592930 |
| 22762 | Vmn1r206 | NM_134216.1 | chr13:22620096-22621035 |
| 22763 | Vmn1r207-ps | NM_001166709.1 | chr13:22725683-22726622 |
| 22764 | Vmn1r208 | NM_134218.1 | chr13:22772398-22773325 |
| 22765 | Vmn1r209 | NM_001013787.1 | chr13:22805579-22806518 |
| 22766 | Vmn1r21 | NM_134183.1 | chr6:57843563-57844457 |
| 22767 | Vmn1r210 | NM_134235.1 | chr13:22827193-22828114 |
| 22768 | Vmn1r211 | NM_134243.1 | chr13:22851598-22852495 |
| 22769 | Vmn1r212 | NM_134241.1 | chr13:22883087-22884161 |
| 22770 | Vmn1r213 | NM_134215.1 | chr13:23011248-23012403 |
| 22771 | Vmn1r214 | NM_134214.1 | chr13:23034337-23035441 |
| 22772 | Vmn1r215 | NM_134219.1 | chr13:23075791-23076694 |
| 22773 | Vmn1r216 | NM_134245.1 | chr13:23099148-23100045 |
| 22774 | Vmn1r217 | NM_134239.1 | chr13:23113833-23114730 |
| 22775 | Vmn1r218 | NM_134222.1 | chr13:23136484-23137381 |
| 22776 | Vmn1r219 | NM_134238.1 | chr13:23162642-23163581 |
| 22777 | Vmn1r22 | NM_134178.1 | chr6:57900081-57900990 |
| 22778 | Vmn1r220 | NM_134237.1 | chr13:23183627-23184524 |
| 22779 | Vmn1r221 | NM_001167542.1 | chr13:23217372-23218311 |
| 22780 | Vmn1r222 | NM_134240.1 | chr13:23232114-23233041 |
| 22781 | Vmn1r223 | NM_001083311.1 | chr13:23249237-23250323 |
| 22782 | Vmn1r224 | NM_001166735.1 | chr17:20419162-20420059 |
| 22783 | Vmn1r225 | NM_134194.1 | chr17:20502298-20503195 |
| 22784 | Vmn1r226 | NM_134191.1 | chr17:20687507-20688404 |
| 22785 | Vmn1r227 | NM_134195.2 | chr17:20735099-20736126 |
| 22786 | Vmn1r228 | NM_134192.3 | chr17:20776058-20777501 |
| 22787 | Vmn1r229 | NM_134190.1 | chr17:20814494-20815415 |
| 22788 | Vmn1r23 | NM_134179.1 | chr6:57925882-57926791 |
| 22789 | Vmn1r230 | NM_134197.1 | chr17:20846550-20847501 |
| 22790 | Vmn1r231 | NM_134196.1 | chr17:20889715-20890651 |
| 22791 | Vmn1r232 | NM_134193.2 | chr17:20913204-20914363 |
| 22792 | Vmn1r233 | NM_134202.1 | chr17:20993726-20994686 |
| 22793 | Vmn1r234 | NM_134198.1 | chr17:21228825-21229815 |
| 22794 | Vmn1r235 | NM_134199.3 | chr17:21260426-21262863 |
| 22795 | Vmn1r236 | NM_134201.2 | chr17:21286532-21287653 |
| 22796 | Vmn1r237 | NM_134200.1 | chr17:21314016-21314886 |
| 22797 | Vmn1r238 | NM_001167539.1 | chr18:3122491-3123412 |
| 22798 | Vmn1r24 | NM_134173.2 | chr6:57955640-57956531 |
| 22799 | Vmn1r25 | NM_053238.2 | chr6:57978393-57979302 |
| 22800 | Vmn1r26 | NM_134172.1 | chr6:58008182-58009202 |
| 22801 | Vmn1r27 | NM_134436.2 | chr6:58215105-58216017 |
| 22802 | Vmn1r28 | NM_134180.1 | chr6:58265173-58266082 |
| 22803 | Vmn1r29 | NM_053232.1 | chr6:58307296-58308208 |
| 22804 | Vmn1r3 | NM_001167535.1 | chr4:3184384-3185305 |
| 22805 | Vmn1r30 | NM_134177.1 | chr6:58434936-58435845 |
| 22806 | Vmn1r31 | NM_001166729.1 | chr6:58471966-58472878 |
| 22807 | Vmn1r32 | NM_134170.3 | chr6:66552182-66559708 |
| 22808 | Vmn1r33 | NM_134169.1 | chr6:66611650-66612568 |
| 22809 | Vmn1r34 | NM_001166719.1 | chr6:66636822-66637752 |
| 22810 | Vmn1r35 | NM_134167.1 | chr6:66678793-66679684 |
| 22811 | Vmn1r36 | NM_134166.1 | chr6:66715971-66716889 |
| 22812 | Vmn1r37 | NM_134165.1 | chr6:66731391-66732300 |
| 22813 | Vmn1r38 | NM_134168.1 | chr6:66776221-66777130 |
| 22814 | Vmn1r39 | NM_001166720.1 | chr6:66804414-66805332 |
| 22815 | Vmn1r4 | NM_134176.1 | chr6:56956512-56957406 |
| 22816 | Vmn1r40 | NM_053228.1 | chr6:89714202-89715135 |
| 22817 | Vmn1r41 | NM_053230.2 | chr6:89740887-89747414 |
| 22818 | Vmn1r42 | NM_053221.2 | chr6:89844517-89845615 |
| 22819 | Vmn1r43 | NM_053220.2 | chr6:89869460-89870529 |
| 22820 | Vmn1r44 | NM_053227.2 | chr6:89893273-89894206 |
| 22821 | Vmn1r45 | NM_011684.2 | chr6:89931649-89940507 |
| 22822 | Vmn1r46 | NM_053229.1 | chr6:89976170-89977100 |
| 22823 | Vmn1r47 | NM_053219.2 | chr6:90021887-90022820 |
| 22824 | Vmn1r48 | NM_053218.1 | chr6:90035932-90036841 |
| 22825 | Vmn1r49 | NM_011911.1 | chr6:90072085-90073018 |
| 22826 | Vmn1r5 | NM_134174.2 | chr6:56985341-56986289 |
| 22827 | Vmn1r50 | NM_053225.1 | chr6:90107274-90108207 |
| 22828 | Vmn1r51 | NM_011683.2 | chr6:90122642-90130645 |
| 22829 | Vmn1r52 | NM_053222.1 | chr6:90178715-90179645 |
| 22830 | Vmn1r53 | NM_053226.2 | chr6:90223316-90224438 |
| 22831 | Vmn1r54 | NM_053224.1 | chr6:90269105-90270053 |
| 22832 | Vmn1r55 | NM_001166706.1 | chr7:5146489-5147422 |
| 22833 | Vmn1r56 | NM_030740.1 | chr7:5194915-5196747 |
| 22834 | Vmn1r57 | NM_001166734.1 | chr7:5220477-5221410 |
| 22835 | Vmn1r58 | NM_030739.2 | chr7:5408889-5413145 |
| 22836 | Vmn1r59 | NM_207543.1 | chr7:5453826-5454759 |
| 22837 | Vmn1r6 | NM_134175.1 | chr6:57002354-57003266 |
| 22838 | Vmn1r60 | NM_001166732.1 | chr7:5544196-5545099 |
| 22839 | Vmn1r61 | NM_001166733.1 | chr7:5610410-5611313 |
| 22840 | Vmn1r62 | NM_030741.2 | chr7:93810-5676597 |
| 22841 | Vmn1r63 | NM_030742.1 | chr7:148880-5805445 |
| 22842 | Vmn1r64 | NM_207544.1 | chr7:5883579-5884542 |
| 22843 | Vmn1r65 | NM_030738.2 | chr7:6007749-6011010 |
| 22844 | Vmn1r66 | NM_134230.3 | chr7:10273827-10275351 |
| 22845 | Vmn1r67 | NM_134229.2 | chr7:10446788-10447787 |
| 22846 | Vmn1r68 | NM_001172072.1 | chr7:10527209-10528169 |
| 22847 | Vmn1r69 | NM_145842.3 | chr7:10579755-10581487 |
| 22848 | Vmn1r7 | NM_001166710.1 | chr6:57024337-57025273 |
| 22849 | Vmn1r70 | NM_134228.1 | chr7:10633586-10634483 |
| 22850 | Vmn1r71 | NM_145848.3 | chr7:10747501-10749538 |
| 22851 | Vmn1r72 | NM_145843.1 | chr7:11669598-11670519 |
| 22852 | Vmn1r73 | NM_134203.1 | chr7:11756256-11757168 |
| 22853 | Vmn1r74 | NM_134206.1 | chr7:11846774-11847689 |
| 22854 | Vmn1r75 | NM_134207.1 | chr7:11880342-11881260 |
| 22855 | Vmn1r76 | NM_134205.2 | chr7:11930310-11931285 |
| 22856 | Vmn1r77 | NM_001166731.1 | chr7:12041298-12042219 |
| 22857 | Vmn1r78 | NM_134208.2 | chr7:12152463-12153405 |
| 22858 | Vmn1r79 | NM_001166835.1 | chr7:12176192-12177113 |
| 22859 | Vmn1r8 | NM_134187.3 | chr6:57035912-57037125 |
| 22860 | Vmn1r80 | NM_134204.2 | chr7:12192964-12193891 |
| 22861 | Vmn1r81 | NM_134210.1 | chr7:12259758-12260679 |
| 22862 | Vmn1r82 | NM_134234.1 | chr7:12304804-12305719 |
| 22863 | Vmn1r83 | NM_134209.1 | chr7:12321207-12322128 |
| 22864 | Vmn1r84 | NM_134233.1 | chr7:12361807-12362764 |
| 22865 | Vmn1r85 | NM_145847.1 | chr7:13084288-13085215 |
| 22866 | Vmn1r86 | NM_001167536.1 | chr7:13101996-13102947 |
| 22867 | Vmn1r87 | NM_134227.1 | chr7:13131470-13132358 |
| 22868 | Vmn1r88 | NM_001167537.1 | chr7:13177718-13178669 |
| 22869 | Vmn1r89 | NM_134226.1 | chr7:13219338-13220289 |
| 22870 | Vmn1r9 | NM_134185.2 | chr6:57070894-57071945 |
| 22871 | Vmn1r90 | NM_001244031.1 | chr7:14561241-14562171 |
| 22872 | Vmn1r91 | NM_001166736.1 | chr7:20101157-20102081 |
| 22873 | Vmn1r94 | NM_001166723.1 | chr7:20167459-22164035 |
| 22874 | Vmn1r95 | NM_001167538.1 | chr7:20193709-22190300 |
| 22875 | Vmn1r-ps103 | NM_134211.2 | chr13:22441271-22442243 |
| 22876 | Vmn1r-ps79 | NR_030707.1 | chr7:20356625-22354555 |
| 22877 | Vmn2r1 | NM_019918.2 | chr3:64081641-64105458 |
| 22878 | Vmn2r10 | NM_009491.3 | chr5:108995538-109006436 |
| 22879 | Vmn2r100 | NM_001104562.1 | chr17:19504810-19532060 |
| 22880 | Vmn2r101 | NM_001104563.1 | chr17:19577230-19612317 |
| 22881 | Vmn2r102 | NM_001104564.1 | chr17:19660398-19694748 |
| 22882 | Vmn2r103 | NM_001104565.1 | chr17:19773362-19812536 |
| 22883 | Vmn2r104 | NM_001104566.1 | chr17:20029424-20048205 |
| 22884 | Vmn2r105 | NM_001104567.1 | chr17:20208229-20234872 |
| 22885 | Vmn2r106 | NM_001104568.1 | chr17:20267546-20285430 |
| 22886 | Vmn2r107 | NM_001104569.1 | chr17:20345424-20375772 |
| 22887 | Vmn2r108 | NM_001104570.1 | chr17:20462372-20481236 |
| 22888 | Vmn2r109 | NM_001104571.1 | chr17:20540516-20564756 |
| 22889 | Vmn2r11 | NM_001104622.1 | chr5:109046872-109059452 |
| 22890 | Vmn2r110 | NM_001104572.1 | chr17:20573828-20596259 |
| 22891 | Vmn2r111 | NM_001104573.1 | chr17:22547940-22573273 |

Fig.21 - 119

| | | | |
|---|---|---|---|
| 22892 | Vmn2r112 | NM_001104575.1 | chr17:22601147-22619133 |
| 22893 | Vmn2r113 | NM_001104578.1 | chr17:22943183-22958814 |
| 22894 | Vmn2r114 | NM_001102584.1 | chr17:23290933-23312313 |
| 22895 | Vmn2r115 | NM_001104579.1 | chr17:23343976-23360128 |
| 22896 | Vmn2r116 | NM_001104580.1 | chr17:23384802-23401864 |
| 22897 | Vmn2r117 | NM_001104581.1 | chr17:23459674-23479597 |
| 22898 | Vmn2r118 | NM_001104582.1 | chr17:55592340-55624672 |
| 22899 | Vmn2r12 | NM_001104623.1 | chr5:109085848-109097864 |
| 22900 | Vmn2r120 | NM_001104591.1 | chr17:57508782-57545314 |
| 22901 | Vmn2r121 | NM_001100616.1 | chrX:124127338-124135910 |
| 22902 | Vmn2r122 | NM_009488.2 | chr4_JH584292_random:3535-11935 |
| 22903 | Vmn2r123 | NM_009485.1 | chr4:156331125-156339496 |
| 22904 | Vmn2r124 | NM_001271883.1 | chr17:18049483-18074220 |
| 22905 | Vmn2r13 | NM_001104624.1 | chr5:109156067-109192107 |
| 22906 | Vmn2r14 | NM_001104625.1 | chr5:109215501-109224622 |
| 22907 | Vmn2r15 | NM_001104626.1 | chr5:109286268-109297556 |
| 22908 | Vmn2r16 | NM_001104627.1 | chr5:109330380-109364481 |
| 22909 | Vmn2r17 | NM_001104628.1 | chr5:109420012-109453387 |
| 22910 | Vmn2r18 | NM_001102582.1 | chr5:151561660-151586924 |
| 22911 | Vmn2r19 | NM_001104632.1 | chr6:123308332-123336537 |
| 22912 | Vmn2r2 | NM_001104592.1 | chr3:64116431-64137480 |
| 22913 | Vmn2r20 | NM_001104634.1 | chr6:123385261-123418061 |
| 22914 | Vmn2r21 | NM_001104635.1 | chr6:123492507-123533406 |
| 22915 | Vmn2r22 | NM_001104637.1 | chr6:123609757-123650635 |
| 22916 | Vmn2r23 | NM_001104638.1 | chr6:123702820-123742239 |
| 22917 | Vmn2r24 | NM_001104639.1 | chr6:123778970-123816280 |
| 22918 | Vmn2r25 | NM_001104641.1 | chr6:123822813-123853190 |
| 22919 | Vmn2r26 | NM_019917.2 | chr6:124024757-124062035 |
| 22920 | Vmn2r27 | NM_001104642.1 | chr6:124191595-124231784 |
| 22921 | Vmn2r28 | NM_001081405.1 | chr7:5480455-5493851 |
| 22922 | Vmn2r29 | NM_001113468.1 | chr7:7231326-7247328 |
| 22923 | Vmn2r3 | NM_001104614.1 | chr3:64258960-64287417 |
| 22924 | Vmn2r30 | NM_009490.3 | chr7:7312273-7337493 |
| 22925 | Vmn2r31 | NM_001105062.1 | chr7:7383984-7399627 |
| 22926 | Vmn2r32 | NM_001105063.1 | chr7:7463968-7479973 |
| 22927 | Vmn2r33 | NM_001105065.2 | chr7:7550966-7566786 |
| 22928 | Vmn2r34 | NM_001105066.1 | chr7:7671828-7689398 |
| 22929 | Vmn2r35 | NM_001105067.1 | chr7:7786150-7819867 |
| 22930 | Vmn2r36 | NM_001105068.1 | chr7:7876652-7902462 |
| 22931 | Vmn2r37 | NM_009489.2 | chr7:9205548-9223653 |
| 22932 | Vmn2r38 | NM_001105070.1 | chr7:9074795-9097765 |
| 22933 | Vmn2r39 | NM_001105071.1 | chr7:9014749-9030682 |
| 22934 | Vmn2r4 | NM_001104615.1 | chr3:64388620-64410057 |
| 22935 | Vmn2r40 | NM_001105072.1 | chr7:8907733-8931402 |
| 22936 | Vmn2r41 | NM_001105073.1 | chr7:8137904-8161551 |
| 22937 | Vmn2r42 | NM_009493.2 | chr7:8183268-8200320 |
| 22938 | Vmn2r43 | NM_198961.2 | chr7:8244350-8260599 |
| 22939 | Vmn2r44 | NM_001105074.1 | chr7:8367459-8383238 |
| 22940 | Vmn2r45 | NM_001105075.1 | chr7:8471468-8488959 |
| 22941 | Vmn2r46 | NM_001105076.1 | chr7:9485564-9770571 |
| 22942 | Vmn2r47 | NM_001105151.1 | chr7:8010338-9841325 |
| 22943 | Vmn2r48 | NM_001105152.1 | chr7:9927623-9953585 |
| 22944 | Vmn2r49 | NM_001105156.1 | chr7:9976244-9992139 |
| 22945 | Vmn2r5 | NM_001104618.1 | chr3:64490820-64507685 |
| 22946 | Vmn2r50 | NM_001105178.1 | chr7:10037234-10053178 |
| 22947 | Vmn2r51 | NM_001105179.1 | chr7:10087197-10105659 |
| 22948 | Vmn2r52 | NM_001105191.1 | chr7:10158651-10176286 |
| 22949 | Vmn2r53 | NM_001104644.1 | chr7:12581469-12606544 |
| 22950 | Vmn2r54 | NM_001081449.2 | chr7:12615232-12636134 |
| 22951 | Vmn2r55 | NM_001104645.1 | chr7:12651705-12684991 |
| 22952 | Vmn2r56 | NM_001104648.1 | chr7:12693997-12733105 |
| 22953 | Vmn2r57 | NM_177764.4 | chr7:41399731-41448641 |
| 22954 | Vmn2r58 | NM_001105055.1 | chr7:41836880-41872670 |
| 22955 | Vmn2r59 | NM_001105056.1 | chr7:42011791-42058981 |
| 22956 | Vmn2r6 | NM_001104619.1 | chr3:64537560-64559818 |
| 22957 | Vmn2r60 | NM_001105057.1 | chr7:42116470-42195776 |
| 22958 | Vmn2r61 | NM_001105058.1 | chr7:42260052-42300755 |
| 22959 | Vmn2r62 | NM_001105059.1 | chr7:42764437-42793496 |
| 22960 | Vmn2r63 | NM_001105060.1 | chr7:42903250-42933789 |
| 22961 | Vmn2r65 | NM_001105180.1 | chr7:84940168-84964009 |
| 22962 | Vmn2r66 | NM_001033878.3 | chr7:84994644-85012020 |
| 22963 | Vmn2r67 | NM_001102579.1 | chr7:85136239-85155902 |
| 22964 | Vmn2r68 | NM_001105181.1 | chr7:85221517-85237704 |
| 22965 | Vmn2r69 | NM_001105182.1 | chr7:85406375-85415676 |
| 22966 | Vmn2r7 | NM_175674.3 | chr3:64690659-64719602 |
| 22967 | Vmn2r70 | NM_001105183.1 | chr7:85558702-85569088 |
| 22968 | Vmn2r71 | NM_001105184.1 | chr7:85615461-85624547 |
| 22969 | Vmn2r72 | NM_001105185.1 | chr7:85737783-85754981 |
| 22970 | Vmn2r73 | NM_001105186.1 | chr7:85857546-85875938 |
| 22971 | Vmn2r74 | NM_001105187.1 | chr7:85951866-85961482 |
| 22972 | Vmn2r75 | NM_001102578.1 | chr7:86148041-86171724 |
| 22973 | Vmn2r76 | NM_001102580.1 | chr7:86225205-86246201 |
| 22974 | Vmn2r77 | NM_001105188.1 | chr7:86795140-86812032 |
| 22975 | Vmn2r78 | NM_001105189.1 | chr7:86915348-86955177 |
| 22976 | Vmn2r79 | NM_001105190.1 | chr7:86996464-87037968 |
| 22977 | Vmn2r8 | NM_001104620.1 | chr5:108797192-108808754 |
| 22978 | Vmn2r80 | NM_001103368.1 | chr10:79148815-79194933 |
| 22979 | Vmn2r81 | NM_175936.1 | chr10:79247776-79294535 |
| 22980 | Vmn2r82 | NM_001101572.1 | chr10:79356590-79396766 |
| 22981 | Vmn2r83 | NM_001104537.1 | chr10:79468957-79492154 |
| 22982 | Vmn2r84 | NM_001081448.1 | chr10:130385799-130394241 |
| 22983 | Vmn2r85 | NM_001102602.1 | chr10:130418260-130429612 |
| 22984 | Vmn2r86 | NM_001103365.1 | chr10:130446198-130455894 |
| 22985 | Vmn2r87 | NM_001103366.1 | chr10:130471820-130497379 |
| 22986 | Vmn2r88 | NM_011686.1 | chr14:51411000-51418882 |
| 22987 | Vmn2r89 | NM_009486.3 | chr14:51451961-51461293 |
| 22988 | Vmn2r9 | NM_001104621.1 | chr5:108842946-108852510 |
| 22989 | Vmn2r90 | NM_001104539.1 | chr17:17703940-17734167 |
| 22990 | Vmn2r91 | NM_001104540.1 | chr17:18085056-18136643 |
| 22991 | Vmn2r92 | NM_001104541.1 | chr17:18151929-18185178 |
| 22992 | Vmn2r93 | NM_001104542.1 | chr17:18298280-18326441 |
| 22993 | Vmn2r94 | NM_001104543.1 | chr17:18243569-18277566 |
| 22994 | Vmn2r95 | NM_001102581.1 | chr17:18424103-18452324 |
| 22995 | Vmn2r96 | NM_001104547.1 | chr17:18581726-18598157 |
| 22996 | Vmn2r97 | NM_001104549.1 | chr17:18914321-18948071 |
| 22997 | Vmn2r98 | NM_001104550.1 | chr17:19053492-19081311 |
| 22998 | Vmn2r99 | NM_001104551.2 | chr17:19362134-19394590 |
| 22999 | Vmn2r-ps11 | NR_003962.1 | chr3:64632862-64844743 |
| 23000 | Vmn2r-ps129 | NR_033648.1 | chr17:23004970-23006659 |
| 23001 | Vmn2r-ps159 | NR_028141.1 | chr4:156331102-156339499 |
| 23002 | Vmn2r-ps54 | NR_004441.1 | chr7:41668013-41727193 |
| 23003 | Vmn2r-ps60 | NR_028441.1 | chr7:42430104-42430369 |
| 23004 | Vmo1 | NM_001013607.1 | chr11:70513515-70514616 |
| 23005 | Vmp1 | NM_029478.3 | chr11:86583864-86683822 |
| 23006 | Vnn1 | NM_011704.3 | chr10:23894687-23905343 |
| 23007 | Vnn3 | NM_011979.2 | chr10:23851461-23869843 |
| 23008 | Vopp1 | NM_146168.1 | chr6:57752263-57825125 |
| 23009 | Vprbp | NM_001015507.2 | chr9:106821975-106880992 |
| 23010 | Vpreb1 | NM_016982.2 | chr16:16868400-16869255 |
| 23011 | Vpreb2 | NM_016983.1 | chr16:17980564-17981080 |
| 23012 | Vpreb3 | NM_009514.4 | chr10:75948311-75949646 |
| 23013 | Vps11 | NM_027889.1 | chr9:44348104-44361670 |
| 23014 | Vps13a | NM_173028.4 | chr19:16615365-16780933 |
| 23015 | Vps13b | NM_177151.3 | chr15:35371545-35931229 |
| 23016 | Vps13c | NM_177184.3 | chr9:67840395-67995634 |
| 23017 | Vps13d | NM_001276465.1 | chr4:144972621-145195005 |
| 23018 | Vps16 | NM_030559.3 | chr2:130424319-130444269 |
| 23019 | Vps18 | NM_172269.3 | chr2:119288741-119298453 |
| 23020 | Vps25 | NM_001284411.1 | chr11:101253706-101259547 |
| 23021 | Vps26a | NM_001113355.1 | chr10:62454842-62486598 |
| 23022 | Vps26b | NM_178027.4 | chr9:27004501-27030094 |
| 23023 | Vps28 | NM_025842.4 | chr15:76622085-76626084 |
| 23024 | Vps29 | NM_019780.1 | chr5:122354412-122363287 |
| 23025 | Vps33a | NM_029929.3 | chr5:123528759-123573015 |
| 23026 | Vps33b | NM_178070.4 | chr7:80269654-80291579 |
| 23027 | Vps35 | NM_022997.4 | chr8:85260391-85299497 |
| 23028 | Vps36 | NM_027338.1 | chr8:22192859-22218597 |
| 23029 | Vps37a | NM_033560.3 | chr8:40511782-40551134 |
| 23030 | Vps37b | NM_177876.4 | chr5:124004640-124032260 |
| 23031 | Vps37c | NM_181403.2 | chr19:10688814-10714419 |
| 23032 | Vps37d | NM_001199677.1 | chr5:135072899-135078266 |
| 23033 | Vps39 | NM_147153.3 | chr2:120316460-120353133 |
| 23034 | Vps41 | NM_172120.4 | chr13:18717291-18866811 |
| 23035 | Vps45 | NM_013841.3 | chr3:95999831-96058455 |
| 23036 | Vps4a | NM_126165.1 | chr8:107031325-107045756 |
| 23037 | Vps4b | NM_009190.2 | chr1:106770787-106796725 |
| 23038 | Vps51 | NM_001081041.1 | chr19:6067841-6077187 |
| 23039 | Vps52 | NM_172620.3 | chr17:33955881-33966488 |
| 23040 | Vps53 | NM_026664.3 | chr11:76046225-76179630 |
| 23041 | Vps54 | NM_001290628.1 | chr11:21239031-21321133 |
| 23042 | Vps72 | NM_009336.2 | chr3:95111041-95123051 |
| 23043 | Vps8 | NM_001285893.1 | chr16:21423117-21644681 |
| 23044 | Vps9d1 | NM_028200.2 | chr8:123242355-123254222 |
| 23045 | Vrk1 | NM_001029843.1 | chr12:106010262-106077410 |
| 23046 | Vrk2 | NM_001252447.1 | chr11:26471401-26593920 |
| 23047 | Vrk3 | NM_133945.1 | chr7:44748628-44777514 |
| 23048 | Vrtn | NM_001033776.2 | chr12:84642895-84651455 |
| 23049 | Vsig1 | NM_026103.1 | chrX:140923188-140939472 |
| 23050 | Vsig10 | NM_001033311.3 | chr5:117319265-117355006 |
| 23051 | Vsig10l | NM_001290316.1 | chr7:43463232-43472014 |
| 23052 | Vsig2 | NM_020518.2 | chr9:37539254-37544205 |
| 23053 | Vsig4 | NM_177789.4 | chrX:96247202-96293438 |
| 23054 | Vsig8 | NM_177723.4 | chr1:172555937-172563717 |
| 23055 | Vsnl1 | NM_012038.4 | chr12:11325244-11436649 |
| 23056 | Vstm2a | NM_001290539.1 | chr11:16257723-16284551 |
| 23057 | Vstm2b | NM_021387.3 | chr7:40899277-40929968 |
| 23058 | Vstm2l | NM_198627.2 | chr2:157914652-157944719 |
| 23059 | Vstm4 | NM_178791.4 | chr14:32856755-32939489 |
| 23060 | Vstm5 | NM_026955.2 | chr9:15239044-15259413 |
| 23061 | Vsx1 | NM_054068.2 | chr2:150680701-150689137 |
| 23062 | Vsx2 | NM_007701.3 | chr12:84569827-84595457 |
| 23063 | Vta1 | NM_025418.3 | chr10:14655332-14705489 |
| 23064 | Vtcn1 | NM_178594.3 | chr3:100825458-100896922 |
| 23065 | Vti1a | NM_016862.4 | chr19:55316056-55627461 |
| 23066 | Vti1b | NM_016800.3 | chr12:79156016-79172458 |
| 23067 | Vtn | NM_011707.2 | chr11:78499119-78502325 |
| 23068 | Vwa1 | NM_147776.4 | chr4:155768494-155774561 |
| 23069 | Vwa2 | NM_172840.2 | chr19:56874415-56912078 |
| 23070 | Vwa3a | NM_177697.3 | chr7:120739556-120805540 |
| 23071 | Vwa5a | NM_001145957.1 | chr9:38718267-38743337 |
| 23072 | Vwa5b1 | NM_029401.1 | chr4:138568968-138623992 |
| 23073 | Vwa5b2 | NM_001144953.1 | chr16:20589581-20605377 |
| 23074 | Vwa7 | NM_138582.1 | chr17:35016578-35026741 |
| 23075 | Vwa8 | NM_027906.1 | chr14:78849177-79202310 |
| 23076 | Vwa9 | NM_001077631.2 | chr9:64960831-64986981 |
| 23077 | Vwc2 | NM_177033.3 | chr11:11114015-11263526 |
| 23078 | Vwc2l | NM_177164.3 | chr1:70725714-70885397 |
| 23079 | Vwce | NM_027913.1 | chr19:10634232-10665210 |
| 23080 | Vwde | NM_001013757.2 | chr6:13185610-13224965 |
| 23081 | Vwf | NM_011708.4 | chr6:125552947-125686679 |
| 23082 | Wac | NM_001146298.2 | chr18:7869196-7929028 |
| 23083 | Wap | NM_011709.5 | chr11:6635482-6638649 |
| 23084 | Wapal | NM_001004436.4 | chr14:34673927-34747983 |
| 23085 | Wars | NM_001164314.1 | chr12:108860029-108894174 |

Fig.21 - 120

| | | | |
|---|---|---|---|
| 23086 | Wars2 | NM_027462.4 | chr3:99141089-99220203 |
| 23087 | Was | NM_009515.2 | chrX:8081465-8090491 |
| 23088 | Wasf1 | NM_031877.3 | chr10:40883533-40938569 |
| 23089 | Wasf2 | NM_153423.6 | chr4:133130632-133198330 |
| 23090 | Wasf3 | NM_145155.3 | chr5:146385005-146471125 |
| 23091 | Wash | NM_001037757.1 | chr17:66111644-66120503 |
| 23092 | Wasl | NM_001167745.1 | chr6:24632685-24664995 |
| 23093 | Wbp1 | NM_001083922.1 | chr6:83119043-83121461 |
| 23094 | Wbp11 | NM_021714.4 | chr6:136813653-136828216 |
| 23095 | Wbp1l | NM_001177812.1 | chr19:46599105-46657389 |
| 23096 | Wbp2 | NM_016852.2 | chr11:116078572-116086964 |
| 23097 | Wbp2nl | NM_029066.1 | chr15:82298983-82314558 |
| 23098 | Wbp4 | NM_018765.3 | chr14:79459936-79481268 |
| 23099 | Wbp5 | NM_011712.2 | chrX:136245079-136247139 |
| 23100 | Wbscr16 | NM_033572.2 | chr5:134148057-134176767 |
| 23101 | Wbscr17 | NM_145218.3 | chr5:130874950-131307522 |
| 23102 | Wbscr22 | NM_025375.3 | chr5:135052956-135064666 |
| 23103 | Wbscr25 | NR_026907.1 | chr5:134987432-135001350 |
| 23104 | Wbscr27 | NM_024479.2 | chr5:134932372-134942637 |
| 23105 | Wbscr28 | NM_029681.3 | chr5:134901592-134906733 |
| 23106 | Wdfy1 | NM_001111279.1 | chr1:79702261-79761769 |
| 23107 | Wdfy2 | NM_175546.4 | chr14:62837689-62956886 |
| 23108 | Wdfy3 | NM_172882.3 | chr5:101832952-102069921 |
| 23109 | Wdfy4 | NM_001146022.2 | chr14:32959546-33185066 |
| 23110 | Wdhd1 | NM_172598.3 | chr14:47240943-47276857 |
| 23111 | Wdpcp | NM_145425.3 | chr11:21572280-21898686 |
| 23112 | Wdr1 | NM_011715.2 | chr5:38526812-38561595 |
| 23113 | Wdr11 | NM_172255.3 | chr7:129591862-129635738 |
| 23114 | Wdr12 | NM_001199060.1 | chr1:60076867-60098500 |
| 23115 | Wdr13 | NM_001290783.1 | chrX:8123300-8132858 |
| 23116 | Wdr16 | NM_027963.2 | chr11:67924805-67965642 |
| 23117 | Wdr17 | NM_001172152.1 | chr8:54629615-54724368 |
| 23118 | Wdr18 | NM_175450.4 | chr10:79960151-79969246 |
| 23119 | Wdr19 | NM_153391.2 | chr5:65199695-65260415 |
| 23120 | Wdr20 | NM_027149.2 | chr12:110737948-110795028 |
| 23121 | Wdr20rt | NM_027614.1 | chr12:65225516-65228454 |
| 23122 | Wdr24 | NM_173741.3 | chr17:25823626-25828730 |
| 23123 | Wdr25 | NM_177602.3 | chr12:108894271-109028452 |
| 23124 | Wdr26 | NM_145514.5 | chr1:181173225-181211978 |
| 23125 | Wdr27 | NM_175173.3 | chr17:14818671-14943124 |
| 23126 | Wdr3 | NM_175552.4 | chr3:100138179-100162403 |
| 23127 | Wdr31 | NM_001290521.1 | chr4:62452631-62470895 |
| 23128 | Wdr33 | NM_001170966.1 | chr18:31804056-31835435 |
| 23129 | Wdr34 | NM_001008498.2 | chr2:30031557-30048879 |
| 23130 | Wdr35 | NM_001159527.1 | chr12:8974000-9028847 |
| 23131 | Wdr36 | NM_001110015.1 | chr18:32837224-32866420 |
| 23132 | Wdr37 | NM_001039388.2 | chr13:8802965-8871736 |
| 23133 | Wdr38 | NM_029687.3 | chr2:38998308-39001584 |
| 23134 | Wdr4 | NM_021322.2 | chr17:31494321-31512487 |
| 23135 | Wdr41 | NM_172590.3 | chr13:94976343-95023316 |
| 23136 | Wdr43 | NM_175639.1 | chr17:71616214-71659031 |
| 23137 | Wdr44 | NM_175180.3 | chrX:23693050-23806001 |
| 23138 | Wdr45 | NM_001290792.1 | chrX:7722219-7728201 |
| 23139 | Wdr45b | NM_025793.3 | chr11:121327202-121354447 |
| 23140 | Wdr46 | NM_020603.2 | chr17:33940722-33949695 |
| 23141 | Wdr47 | NM_181400.3 | chr3:108591277-108645719 |
| 23142 | Wdr48 | NM_026236.3 | chr9:119894894-119926579 |
| 23143 | Wdr5 | NM_080848.2 | chr2:27515146-27536538 |
| 23144 | Wdr52 | NM_001033247.1 | chr16:44394798-44482428 |
| 23145 | Wdr53 | NM_001185162.1 | chr16:32247226-32257083 |
| 23146 | Wdr54 | NM_023790.2 | chr6:83152709-83156379 |
| 23147 | Wdr55 | NM_026464.2 | chr18:36760238-36763708 |
| 23148 | Wdr59 | NM_001170742.1 | chr8:111448783-111522107 |
| 23149 | Wdr5b | NM_027113.2 | chr16:36041189-36042974 |
| 23150 | Wdr6 | NM_031392.2 | chr9:108572312-108578670 |
| 23151 | Wdr60 | NM_146039.3 | chr12:116207049-116263025 |
| 23152 | Wdr61 | NM_001025375.2 | chr9:54717152-54734549 |
| 23153 | Wdr62 | NM_146186.3 | chr7:30240137-30280421 |
| 23154 | Wdr63 | NM_172864.3 | chr3:146040525-146108036 |
| 23155 | Wdr64 | NM_029453.2 | chr1:175698592-175815733 |
| 23156 | Wdr65 | NM_026789.4 | chr4:118554550-118620405 |
| 23157 | Wdr7 | NM_001014981.1 | chr18:63708694-63989759 |
| 23158 | Wdr70 | NM_001081402.1 | chr15:7873054-8099209 |
| 23159 | Wdr72 | NM_001033500.3 | chr9:74110333-74283203 |
| 23160 | Wdr73 | NM_028026.2 | chr7:80890722-80901269 |
| 23161 | Wdr74 | NM_134139.1 | chr19:8735838-8740624 |
| 23162 | Wdr75 | NM_028599.2 | chr1:45795500-45823613 |
| 23163 | Wdr76 | NM_001290986.1 | chr2:121506722-121544859 |
| 23164 | Wdr77 | NM_027432.3 | chr3:105959497-105969760 |
| 23165 | Wdr78 | NM_146254.4 | chr4:103038064-103114299 |
| 23166 | Wdr8 | NM_021499.2 | chr4:154142371-154156818 |
| 23167 | Wdr81 | NM_138950.2 | chr11:75440942-75454717 |
| 23168 | Wdr82 | NM_029896.1 | chr9:106170928-106191706 |
| 23169 | Wdr83 | NM_026399.2 | chr8:85075034-85080746 |
| 23170 | Wdr83os | NM_001001493.2 | chr8:85080962-85082339 |
| 23171 | Wdr86 | NM_001081441.1 | chr5:24712268-24730680 |
| 23172 | Wdr89 | NM_028203.1 | chr12:75630593-75669537 |
| 23173 | Wdr90 | NM_001163766.1 | chr17:25844733-25861515 |
| 23174 | Wdr91 | NM_001013366.1 | chr6:34880425-34910831 |
| 23175 | Wdr92 | NM_178909.4 | chr11:17211892-17235200 |
| 23176 | Wdr93 | NM_001037927.1 | chr7:79743162-79785950 |
| 23177 | Wdr95 | NM_029440.3 | chr5:149528678-149611894 |
| 23178 | Wdr96 | NM_027559.2 | chr19:47736856-47837361 |
| 23179 | Wdsub1 | NM_001159636.1 | chr2:59855193-59882606 |
| 23180 | Wdtc1 | NM_199306.4 | chr4:133292465-133339315 |
| 23181 | Wdyhv1 | NM_029734.1 | chr15:58141435-58158654 |
| 23182 | Wee1 | NM_009516.3 | chr7:110122058-110143299 |
| 23183 | Wee2 | NM_201370.2 | chr6:40442862-40466815 |
| 23184 | Wfdc1 | NM_023395.2 | chr8:119666364-119688020 |
| 23185 | Wfdc10 | NM_001039501.2 | chr2:164656045-164657368 |
| 23186 | Wfdc11 | NM_001161806.1 | chr2:164662893-164674087 |
| 23187 | Wfdc12 | NM_138684.2 | chr2:164189230-164190558 |
| 23188 | Wfdc13 | NM_001012704.1 | chr2:164685106-164687706 |
| 23189 | Wfdc15a | NM_183271.2 | chr2:164198871-164200117 |
| 23190 | Wfdc15b | NM_001045554.1 | chr2:164214453-164221663 |
| 23191 | Wfdc16 | NM_001012723.2 | chr2:164634707-164638802 |
| 23192 | Wfdc17 | NM_001081957.1 | chr11:83704055-83706269 |
| 23193 | Wfdc18 | NM_007969.4 | chr11:83709003-83711360 |
| 23194 | Wfdc2 | NM_026323.2 | chr2:164562715-164568506 |
| 23195 | Wfdc3 | NM_027961.1 | chr2:164731225-164743267 |
| 23196 | Wfdc5 | NM_145369.3 | chr2:164176324-164182742 |
| 23197 | Wfdc6a | NM_001033240.4 | chr2:164579518-164585447 |
| 23198 | Wfdc6b | NM_001012725.2 | chr2:164613699-164618213 |
| 23199 | Wfdc8 | NM_001080550.2 | chr2:164596457-164613626 |
| 23200 | Wfdc9 | NM_001160414.1 | chr2:164649624-164654966 |
| 23201 | Wfikkn1 | NM_001100454.1 | chr17:25877627-25880858 |
| 23202 | Wfikkn2 | NM_181819.2 | chr11:94235951-94242579 |
| 23203 | Wfs1 | NM_011716.2 | chr5:36966103-36988982 |
| 23204 | Whamm | NM_001004185.3 | chr7:81571291-81596836 |
| 23205 | Whrn | NM_001008791.2 | chr4:63414909-63495991 |
| 23206 | Whsc1 | NM_001081102.2 | chr5:33843111-33897966 |
| 23207 | Whsc1l1 | NM_001001735.2 | chr8:25602283-25677972 |
| 23208 | Wibg | NM_001170869.2 | chr10:128748454-128766568 |
| 23209 | Wif1 | NM_011915.2 | chr10:121034003-121100642 |
| 23210 | Wipf1 | NM_001289722.1 | chr2:73429609-73453889 |
| 23211 | Wipf2 | NM_197940.2 | chr11:98863597-98905578 |
| 23212 | Wipf3 | NM_001167860.1 | chr6:54452882-54503768 |
| 23213 | Wipi1 | NM_145940.2 | chr11:109573520-109611389 |
| 23214 | Wipi2 | NM_178398.4 | chr5:142629583-142669370 |
| 23215 | Wisp1 | NM_018865.2 | chr15:66891392-66923199 |
| 23216 | Wisp2 | NM_016873.2 | chr2:163820833-163833147 |
| 23217 | Wisp3 | NM_001127376.1 | chr10:39150970-39163794 |
| 23218 | Wiz | NM_011717.4 | chr17:32354049-32388950 |
| 23219 | Wls | NM_026582.4 | chr3:159839694-159935175 |
| 23220 | Wnk1 | NM_001185020.1 | chr6:119923968-120038655 |
| 23221 | Wnk2 | NM_001290311.1 | chr13:49037600-49148328 |
| 23222 | Wnk3 | NM_001271678.1 | chrX:151198077-151320192 |
| 23223 | Wnk4 | NM_175638.3 | chr11:101260566-101277409 |
| 23224 | Wnt1 | NM_021279.4 | chr15:98789856-98793830 |
| 23225 | Wnt10a | NM_009518.2 | chr1:74792018-74804175 |
| 23226 | Wnt10b | NM_011718.2 | chr15:98771751-98778150 |
| 23227 | Wnt11 | NM_001285792.1 | chr7:98835111-98854747 |
| 23228 | Wnt16 | NM_053116.4 | chr6:22288226-22298522 |
| 23229 | Wnt2 | NM_023653.5 | chr6:17988939-18030445 |
| 23230 | Wnt2b | NM_009520.3 | chr3:104944804-104961709 |
| 23231 | Wnt3 | NM_009521.2 | chr11:103774174-103818021 |
| 23232 | Wnt3a | NM_009522.2 | chr11:59248041-59290751 |
| 23233 | Wnt4 | NM_009523.2 | chr4:137277634-137299501 |
| 23234 | Wnt5a | NM_001256224.1 | chr14:28511404-28525515 |
| 23235 | Wnt5b | NM_001271757.1 | chr6:119432530-119449386 |
| 23236 | Wnt6 | NM_009526.3 | chr1:74771891-74785319 |
| 23237 | Wnt7a | NM_009527.3 | chr6:91363982-91411369 |
| 23238 | Wnt7b | NM_001163633.1 | chr15:85535436-85580729 |
| 23239 | Wnt8a | NM_009290.2 | chr18:34542327-34548061 |
| 23240 | Wnt8b | NM_011720.3 | chr19:44493471-44514273 |
| 23241 | Wnt9a | NM_139298.2 | chr11:59306929-59333552 |
| 23242 | Wnt9b | NM_011719.4 | chr11:103727362-103749821 |
| 23243 | Wrap53 | NM_144824.2 | chr11:69561753-69579324 |
| 23244 | Wrb | NM_207301.2 | chr16:96145418-96157852 |
| 23245 | Wrn | NM_001122822.1 | chr8:33234372-33385527 |
| 23246 | Wrnip1 | NM_030215.3 | chr13:32802029-32822610 |
| 23247 | Wsb1 | NM_001042565.3 | chr11:79239382-79243024 |
| 23248 | Wsb2 | NM_021539.4 | chr5:117357304-117378589 |
| 23249 | Wscd1 | NM_177618.4 | chr11:71750702-71789646 |
| 23250 | Wscd2 | NM_177292.3 | chr5:113550419-113589725 |
| 23251 | Wt1 | NM_144783.2 | chr2:105126528-105173614 |
| 23252 | Wt1os | NR_015462.1 | chr2:105076537-105126510 |
| 23253 | Wtap | NM_001113532.1 | chr17:12971019-12992259 |
| 23254 | Wtip | NM_207212.2 | chr7:34109549-34133268 |
| 23255 | Wwc1 | NM_170779.1 | chr11:35839177-35980089 |
| 23256 | Wwc2 | NM_133791.4 | chr8:47827605-47990551 |
| 23257 | Wwox | NM_019573.3 | chr8:114439651-115352712 |
| 23258 | Wwp1 | NM_001276292.1 | chr4:19608299-19709004 |
| 23259 | Wwp2 | NM_025830.3 | chr8:107436397-107558595 |
| 23260 | Wwtr1 | NM_001168281.1 | chr3:57455643-57575910 |
| 23261 | Xab2 | NM_026156.2 | chr8:3610089-3621296 |
| 23262 | Xaf1 | NM_001037713.4 | chr11:72301628-72313733 |
| 23263 | Xbp1 | NM_001271730.1 | chr11:5520640-5525993 |
| 23264 | Xcl1 | NM_008510.1 | chr1:164931647-164935510 |
| 23265 | Xcr1 | NM_011798.4 | chr9:123852314-123862029 |
| 23266 | Xdh | NM_011723.3 | chr17:73883894-73950196 |
| 23267 | Xiap | NM_009688.3 | chrX:42067835-42109664 |
| 23268 | Xirp1 | NM_011724.3 | chr9:120013754-120023598 |
| 23269 | Xirp2 | NM_001024618.2 | chr2:67446001-67526606 |
| 23270 | Xist | NR_001463.3 | chrX:103460372-103483233 |
| 23271 | Xk | NM_023500.2 | chrX:9272783-9313245 |
| 23272 | Xkr4 | NM_001011874.1 | chr1:3214481-3671498 |
| 23273 | Xkr5 | NM_001113350.2 | chr8:18932728-18950975 |
| 23274 | Xkr6 | NM_173393.2 | chr14:63606529-63820410 |
| 23275 | Xkr7 | NM_001011732.1 | chr2:153031851-153055775 |
| 23276 | Xkr8 | NM_201368.1 | chr4:132724903-132732546 |
| 23277 | Xkr9 | NM_001011873.2 | chr1:13668770-13701723 |
| 23278 | Xkrx | NM_183319.2 | chrX:134149044-134161928 |
| 23279 | Xlr | NM_001291747.1 | chrX:53783734-53797706 |

Fig.21 - 121

| | | | | | | | |
|---|---|---|---|---|---|---|---|
| 23280 | Xlr3a | NM_001110784.1 | chrX:73086292-73097095 | 23377 | Zbtb12 | NM_198886.3 | chr17:34894558-34896844 |
| 23281 | Xlr3b | NM_001081643.1 | chrX:73192178-73202930 | 23378 | Zbtb14 | NM_009547.2 | chr17:69383977-69390544 |
| 23282 | Xlr3c | NM_011727.2 | chrX:73254539-73265390 | 23379 | Zbtb16 | NM_001033324.2 | chr9:48654296-48835945 |
| 23283 | Xlr4a | NM_001081642.1 | chrX:73074344-73082507 | 23380 | Zbtb17 | NM_009541.2 | chr4:141444672-141467937 |
| 23284 | Xlr4b | NM_021365.3 | chrX:73214332-73222453 | 23381 | Zbtb18 | NM_001012330.1 | chr1:177444660-177450764 |
| 23285 | Xlr4c | NM_183094.3 | chrX:73234075-73243130 | 23382 | Zbtb2 | NM_001033466.3 | chr10:4367073-4388108 |
| 23286 | Xlr5a | NM_001045539.2 | chrX:73107634-73117702 | 23383 | Zbtb20 | NM_001285805.1 | chr16:42907644-43619123 |
| 23287 | Xlr5b | NM_001111293.1 | chrX:73148840-73158399 | 23384 | Zbtb21 | NM_001081684.1 | chr16:97947434-97962621 |
| 23288 | Xlr5c | NM_031493.1 | chrX:73285196-73290515 | 23385 | Zbtb22 | NM_020625.1 | chr17:33916175-33919325 |
| 23289 | Xndc1 | NM_001286689.1 | chr7:102065490-102083762 | 23386 | Zbtb24 | NM_001277229.1 | chr10:41450357-41465582 |
| 23290 | Xntrpc | NM_011644.3 | chr7:102071218-102096864 | 23387 | Zbtb25 | NM_001172104.1 | chr12:76348899-76369464 |
| 23291 | Xpa | NM_011728.2 | chr4:46175221-46196311 | 23388 | Zbtb26 | NM_199025.2 | chr2:37432167-37443121 |
| 23292 | Xpc | NM_009531.2 | chr6:91489306-91515888 | 23389 | Zbtb3 | NM_001098237.1 | chr19:8802529-8804854 |
| 23293 | Xpnpep1 | NM_133216.3 | chr19:52991179-53038654 | 23390 | Zbtb32 | NM_021397.2 | chr7:30589680-30592942 |
| 23294 | Xpnpep2 | NM_001289729.1 | chrX:48108724-48136981 | 23391 | Zbtb33 | NM_001079513.1 | chrX:38189792-38197046 |
| 23295 | Xpnpep3 | NM_177310.2 | chr15:81400187-81454888 | 23392 | Zbtb34 | NM_001085507.1 | chr2:33406107-33431324 |
| 23296 | Xpo1 | NM_001035226.1 | chr11:23256040-23297597 | 23393 | Zbtb37 | NM_173424.3 | chr1:161017755-161034259 |
| 23297 | Xpo4 | NM_020506.1 | chr14:57582270-57664956 | 23394 | Zbtb38 | NM_175537.3 | chr9:96685422-96731675 |
| 23298 | Xpo5 | NM_028198.2 | chr17:46202854-46242299 | 23395 | Zbtb39 | NM_198035.1 | chr10:127739537-127747339 |
| 23299 | Xpo6 | NM_028816.2 | chr7:126101718-126200408 | 23396 | Zbtb4 | NM_029348.2 | chr11:69765911-69784026 |
| 23300 | Xpo7 | NM_023045.2 | chr14:70654245-70766628 | 23397 | Zbtb40 | NM_198248.1 | chr4:136979731-137048695 |
| 23301 | Xpot | NM_001081056.1 | chr10:121587379-121626316 | 23398 | Zbtb41 | NM_172643.5 | chr1:139422382-139453007 |
| 23302 | Xpr1 | NM_011273.2 | chr1:155275656-155417444 | 23399 | Zbtb42 | NM_001100460.1 | chr12:112678839-112682747 |
| 23303 | Xrcc1 | NM_009532.4 | chr7:24547149-24573438 | 23400 | Zbtb43 | NM_001025594.1 | chr2:33450287-33468532 |
| 23304 | Xrcc2 | NM_020570.2 | chr5:25689815-25705797 | 23401 | Zbtb44 | NM_001115130.1 | chr9:31030643-31075885 |
| 23305 | Xrcc3 | NM_028875.3 | chr12:111803191-111813879 | 23402 | Zbtb45 | NM_001024699.1 | chr7:13005665-13009800 |
| 23306 | Xrcc4 | NM_028012.4 | chr13:89848913-90089608 | 23403 | Zbtb46 | NM_027656.2 | chr2:181390885-181424388 |
| 23307 | Xrcc5 | NM_009533.2 | chr1:72307420-72394953 | 23404 | Zbtb48 | NM_133879.2 | chr4:152019775-152027671 |
| 23308 | Xrcc6 | NM_010247.2 | chr15:82016368-82040084 | 23405 | Zbtb49 | NM_029162.2 | chr5:38200043-38220428 |
| 23309 | Xrcc6bp1 | NM_001159559.1 | chr10:126887247-126901371 | 23406 | Zbtb5 | NM_001163283.1 | chr4:44991242-45012412 |
| 23310 | Xrn1 | NM_011916.3 | chr9:95954759-96057806 | 23407 | Zbtb6 | NM_146253.5 | chr2:37425499-37430919 |
| 23311 | Xrn2 | NM_011917.2 | chr2:147013059-147077997 | 23408 | Zbtb7a | NM_010731.3 | chr10:81136270-81151657 |
| 23312 | Xrra1 | NM_001164258.1 | chr7:99859217-99917824 | 23409 | Zbtb7b | NM_009565.4 | chr3:89377645-89393203 |
| 23313 | Xxylt1 | NM_198626.2 | chr16:30955502-31081432 | 23410 | Zbtb7c | NM_145356.3 | chr18:75820177-76148564 |
| 23314 | Xylb | NM_001033209.3 | chr9:119357380-119393797 | 23411 | Zbtb8a | NM_028603.4 | chr4:129353631-129378028 |
| 23315 | Xylt1 | NM_175645.3 | chr7:117380978-117667630 | 23412 | Zbtb8b | NM_153541.3 | chr4:129425764-129440818 |
| 23316 | Xylt2 | NM_145828.3 | chr11:94663846-94677493 | 23413 | Zbtb8os | NM_025970.3 | chr4:129336025-129347029 |
| 23317 | Yae1d1 | NM_025904.3 | chr13:17986639-17993351 | 23414 | Zbtb9 | NM_001005916.2 | chr17:26973178-26976203 |
| 23318 | Yaf2 | NM_024189.6 | chr15:93283832-93336935 | 23415 | Zbtbd6 | NM_001034882.3 | chr14:79451834-79454816 |
| 23319 | Yap1 | NM_001171147.1 | chr9:7932000-8004596 | 23416 | Zc2hc1a | NM_173181.3 | chr3:7503425-7553848 |
| 23320 | Yars | NM_134151.4 | chr4:129189794-129219607 | 23417 | Zc2hc1b | NM_029172.1 | chr10:13149643-13178023 |
| 23321 | Yars2 | NM_198246.2 | chr16:16302964-16309640 | 23418 | Zc2hc1c | NM_172414.4 | chr12:85288590-85299358 |
| 23322 | Ybey | NM_172550.4 | chr10:76459566-76469114 | 23419 | Zc3h10 | NM_134003.1 | chr10:128543564-128547744 |
| 23323 | Ybx1 | NM_011732.2 | chr4:119277326-119294513 | 23420 | Zc3h11a | NM_001276767.1 | chr1:133619870-133661399 |
| 23324 | Ybx2 | NM_016875.2 | chr11:69935898-69941599 | 23421 | Zc3h12a | NM_153159.2 | chr4:125118413-125127881 |
| 23325 | Ybx3 | NM_011733.2 | chr6:131364857-131388450 | 23422 | Zc3h12b | NM_001034907.2 | chrX:95711677-95927970 |
| 23326 | Ydjc | NM_026940.4 | chr16:17146966-17148857 | 23423 | Zc3h12c | NM_001162921.1 | chr9:52111984-52168111 |
| 23327 | Yeats2 | NM_001033237.2 | chr16:20141062-20232573 | 23424 | Zc3h12d | NM_172785.3 | chr10:7832469-7870397 |
| 23328 | Yeats4 | NM_026570.4 | chr10:117215140-117224507 | 23425 | Zc3h13 | NM_026083.2 | chr14:75284372-75344426 |
| 23329 | Yes1 | NM_001205132.1 | chr5:32611170-32687066 | 23426 | Zc3h14 | NM_001008506.2 | chr12:98746967-98787774 |
| 23330 | Yif1a | NM_026553.4 | chr19:5088537-5092879 | 23427 | Zc3h15 | NM_026934.3 | chr2:83644577-83664616 |
| 23331 | Yif1b | NM_001110201.1 | chr7:29238322-29247532 | 23428 | Zc3h18 | NM_001029993.1 | chr8:122376615-122417360 |
| 23332 | Yipf1 | NM_001205156.1 | chr4:107314362-107359823 | 23429 | Zc3h3 | NM_172121.1 | chr15:75754446-75841908 |
| 23333 | Yipf2 | NM_001205157.1 | chr9:21588681-21592831 | 23430 | Zc3h4 | NM_198631.2 | chr7:16401195-16437696 |
| 23334 | Yipf3 | NM_145353.2 | chr17:46248079-46252537 | 23431 | Zc3h6 | NM_178404.3 | chr2:128967401-129018563 |
| 23335 | Yipf4 | NM_026417.4 | chr17:74489492-74500277 | 23432 | Zc3h7a | NM_145931.2 | chr16:11136593-11176393 |
| 23336 | Yipf5 | NM_023311.3 | chr18:40204864-40219399 | 23433 | Zc3h7b | NM_001081016.1 | chr15:81744847-81796269 |
| 23337 | Yipf6 | NM_207633.2 | chrX:98937780-98949020 | 23434 | Zc3h8 | NM_020594.2 | chr2:128926267-128944020 |
| 23338 | Yipf7 | NM_023784.5 | chr5:69516669-69542647 | 23435 | Zc3hav1 | NM_028421.1 | chr6:38310496-38354603 |
| 23339 | Ykt6 | NM_019661.4 | chr11:5955757-5967781 | 23436 | Zc3hav1l | NM_172467.3 | chr6:38287393-38299259 |
| 23340 | Ylpm1 | NM_178363.3 | chr12:84996320-85070515 | 23437 | Zc3hc1 | NM_172735.2 | chr6:30366387-30391010 |
| 23341 | Yme1l1 | NM_013771.5 | chr2:23156504-23199260 | 23438 | Zc4h2 | NM_001003916.2 | chrX:95639193-95658509 |
| 23342 | Yod1 | NM_178691.4 | chr1:130717326-130722057 | 23439 | Zcchc10 | NM_026479.4 | chr11:53324688-53333301 |
| 23343 | Ypel1 | NM_001291047.1 | chr16:17070138-17086736 | 23440 | Zcchc11 | NM_175472.3 | chr4:108459425-108559415 |
| 23344 | Ypel2 | NM_001005341.3 | chr11:86936424-86993762 | 23441 | Zcchc12 | NM_028325.3 | chrX:36195903-36199158 |
| 23345 | Ypel3 | NM_025347.2 | chr7:126776974-126780514 | 23442 | Zcchc13 | NM_029158.2 | chrX:103630585-103631664 |
| 23346 | Ypel4 | NM_001005342.2 | chr2:84734203-84737877 | 23443 | Zcchc14 | NM_080855.2 | chr8:121598702-121651933 |
| 23347 | Ypel5 | NM_027166.5 | chr17:72836703-72851195 | 23444 | Zcchc16 | NM_001033795.4 | chrX:144688906-145122410 |
| 23348 | Yrdc | NM_153566.2 | chr4:124850758-124855242 | 23445 | Zcchc17 | NM_153160.4 | chr4:130316084-130359943 |
| 23349 | Ythdc1 | NM_177680.3 | chr5:86804489-86836657 | 23446 | Zcchc18 | NM_001035509.1 | chrX:136993154-136996923 |
| 23350 | Ythdc2 | NM_001163013.1 | chr18:44828664-44889720 | 23447 | Zcchc2 | NM_001122675.1 | chr1:105990405-106034079 |
| 23351 | Ythdf1 | NM_173761.3 | chr2:180904376-180920936 | 23448 | Zcchc24 | NM_001101433.1 | chr14:25711639-25768856 |
| 23352 | Ythdf2 | NM_145393.4 | chr4:132184915-132212256 | 23449 | Zcchc3 | NM_175126.4 | chr2:152411955-152415044 |
| 23353 | Ythdf3 | NM_001145919.1 | chr3:16183182-16217037 | 23450 | Zcchc4 | NM_030185.3 | chr5:52783054-52823571 |
| 23354 | Ywhab | NM_018753.6 | chr2:163995196-164018587 | 23451 | Zcchc5 | NM_199468.1 | chrX:106837081-106840643 |
| 23355 | Ywhae | NM_009536.4 | chr11:75732886-75765841 | 23452 | Zcchc6 | NM_153538.3 | chr13:59771878-59823147 |
| 23356 | Ywhag | NM_018871.3 | chr5:135908378-135934641 | 23453 | Zcchc7 | NM_138590.4 | chr4:44756558-44932214 |
| 23357 | Ywhah | NM_011738.2 | chr5:33018815-33027966 | 23454 | Zcchc8 | NM_027494.3 | chr5:123698301-123721044 |
| 23358 | Ywhaq | NM_011739.3 | chr12:21390328-21417436 | 23455 | Zcchc9 | NM_145453.2 | chr13:91796532-91807696 |
| 23359 | Ywhaz | NM_001253805.1 | chr15:36770261-36794538 | 23456 | Zcrb1 | NM_026025.2 | chr15:93386112-93398290 |
| 23360 | Yy1 | NM_009537.3 | chr12:108793310-108816632 | 23457 | Zcwpw1 | NM_001005426.2 | chr5:137787801-137822621 |
| 23361 | Yy2 | NM_001098723.1 | chrX:157566118-157568985 | 23458 | Zdbf2 | NM_001267872.1 | chr1:63273268-63314575 |
| 23362 | Zadh2 | NM_146090.5 | chr18:84088157-84097514 | 23459 | Zdhhc1 | NM_175160.3 | chr8:105472424-105496870 |
| 23363 | Zak | NM_001164791.1 | chr2:72285700-72407501 | 23460 | Zdhhc11 | NM_027704.2 | chr13:73963861-73992840 |
| 23364 | Zan | NM_011741.2 | chr5:137378636-137477064 | 23461 | Zdhhc12 | NM_001037762.1 | chr2:30090943-30093635 |
| 23365 | Zap70 | NM_001289612.1 | chr1:36778983-36782820 | 23462 | Zdhhc13 | NM_028031.3 | chr7:48789002-48827437 |
| 23366 | Zar1 | NM_174877.3 | chr5:72577113-72581084 | 23463 | Zdhhc14 | NM_146073.3 | chr17:5492599-5753891 |
| 23367 | Zar1l | NM_001159693.1 | chr5:150507068-150518159 | 23464 | Zdhhc15 | NM_175358.4 | chrX:104536969-104671064 |
| 23368 | Zbbx | NM_172515.3 | chr3:75037894-75143772 | 23465 | Zdhhc16 | NM_023740.2 | chr19:41933471-41944103 |
| 23369 | Zbed3 | NM_028106.3 | chr13:95324824-95337842 | 23466 | Zdhhc17 | NM_172554.2 | chr10:110941779-111010066 |
| 23370 | Zbed4 | NM_181412.3 | chr15:88751710-88784516 | 23467 | Zdhhc18 | NM_001017968.2 | chr4:133606991-133633429 |
| 23371 | Zbed5 | NM_183088.2 | chr5:129895722-129903622 | 23468 | Zdhhc19 | NM_199309.2 | chr16:32496280-32507214 |
| 23372 | Zbed6 | NM_001166552.1 | chr1:133655878-133660885 | 23469 | Zdhhc2 | NM_178395.3 | chr8:40423814-40484842 |
| 23373 | Zbp1 | NM_001139519.1 | chr2:173213735-173218922 | 23470 | Zdhhc20 | NM_029492.4 | chr14:57832701-57890262 |
| 23374 | Zbtb1 | NM_178744.2 | chr12:76370265-76388747 | 23471 | Zdhhc21 | NM_026647.3 | chr4:82798737-82859661 |
| 23375 | Zbtb10 | NM_177660.3 | chr3:9250566-9285332 | 23472 | Zdhhc22 | NM_001080943.2 | chr12:86983380-86988676 |
| 23376 | Zbtb11 | NM_173026.2 | chr16:55973803-56008912 | 23473 | Zdhhc23 | NM_001007460.1 | chr16:43969145-43979050 |

Fig.21 - 122

| | | | |
|---|---|---|---|
| 23474 | Zdhhc24 | NM_001168516.1 | chr19:4878667-4885397 |
| 23475 | Zdhhc25 | NM_027306.3 | chr15:88600301-88601669 |
| 23476 | Zdhhc3 | NM_026917.5 | chr9:123072309-123113205 |
| 23477 | Zdhhc4 | NM_028379.5 | chr5:143316488-143329238 |
| 23478 | Zdhhc5 | NM_144887.4 | chr2:84687919-84715164 |
| 23479 | Zdhhc6 | NM_001033573.1 | chr19:55298295-55316032 |
| 23480 | Zdhhc7 | NM_133967.3 | chr8:120081094-120101472 |
| 23481 | Zdhhc8 | NM_172151.4 | chr16:18220752-18235136 |
| 23482 | Zdhhc9 | NM_172465.4 | chrX:48171970-48208702 |
| 23483 | Zeb1 | NM_011546.3 | chr18:5591859-5775468 |
| 23484 | Zeb2 | NM_001289521.1 | chr2:44983511-45110277 |
| 23485 | Zeb2os | NR_110571.1 | chr2:45112259-45114084 |
| 23486 | Zer1 | NM_001290503.1 | chr2:30097282-30124611 |
| 23487 | Zf12 | NR_003547.2 | chrX_GL456233_random:268798-270075 |
| 23488 | Zfand1 | NM_025512.2 | chr3:10339955-10351301 |
| 23489 | Zfand2a | NM_001159908.1 | chr5:139471215-139484491 |
| 23490 | Zfand2b | NM_001159905.1 | chr1:75168645-75171626 |
| 23491 | Zfand3 | NM_148926.2 | chr17:30005086-30210020 |
| 23492 | Zfand4 | NM_001290338.1 | chr6:116264218-116330304 |
| 23493 | Zfand5 | NM_009551.5 | chr19:21272277-21286840 |
| 23494 | Zfand6 | NM_022985.6 | chr7:84615053-84679351 |
| 23495 | Zfa-ps | NR_037920.1 | chr10:52542319-52545739 |
| 23496 | Zfat | NM_001145888.1 | chr15:68083737-68258856 |
| 23497 | Zfc3h1 | NM_001033261.2 | chr10:115384958-115432771 |
| 23498 | Zfhx2 | NM_001039198.1 | chr14:55061658-55092048 |
| 23499 | Zfhx2os | NR_004444.2 | chr14:55073090-55075874 |
| 23500 | Zfhx3 | NM_007496.2 | chr8:108714643-108961636 |
| 23501 | Zfhx4 | NM_030708.2 | chr3:5218553-5415855 |
| 23502 | Zfml | NM_001166371.1 | chr6:83914352-83986871 |
| 23503 | Zfp1 | NM_001037665.2 | chr8:111643442-111671008 |
| 23504 | Zfp101 | NM_009542.2 | chr17:33380178-33394637 |
| 23505 | Zfp105 | NM_009544.2 | chr9:122923077-122931028 |
| 23506 | Zfp106 | NM_011743.2 | chr2:120506829-120563831 |
| 23507 | Zfp108 | NM_018791.2 | chr7:24254793-24262444 |
| 23508 | Zfp109 | NM_020262.3 | chr7:24227794-24237598 |
| 23509 | Zfp11 | NM_172462.4 | chr5:129654594-129670088 |
| 23510 | Zfp110 | NM_022981.4 | chr7:12834809-12850584 |
| 23511 | Zfp111 | NM_019940.2 | chr7:24193214-24208149 |
| 23512 | Zfp112 | NM_021307.2 | chr7:24112319-24127952 |
| 23513 | Zfp113 | NM_019747.4 | chr5:138139701-138155744 |
| 23514 | Zfp114 | NM_001029933.2 | chr7:24175044-24182318 |
| 23515 | Zfp119a | NM_144546.6 | chr17:55864891-55878953 |
| 23516 | Zfp119b | NM_146249.4 | chr17:55938372-55945259 |
| 23517 | Zfp12 | NM_001289589.1 | chr5:143235162-143248834 |
| 23518 | Zfp120 | NM_023266.4 | chr2:150114406-150136678 |
| 23519 | Zfp128 | NM_153802.4 | chr7:12881177-12893422 |
| 23520 | Zfp13 | NM_011747.2 | chr17:23575851-23599487 |
| 23521 | Zfp131 | NM_028245.4 | chr13:119765185-119790806 |
| 23522 | Zfp133-ps | NR_033459.1 | chr2:144459279-144467794 |
| 23523 | Zfp14 | NM_011748.2 | chr7:30036358-30051396 |
| 23524 | Zfp142 | NM_029888.3 | chr1:74566425-74588028 |
| 23525 | Zfp143 | NM_009281.3 | chr7:110061701-110095392 |
| 23526 | Zfp146 | NM_011980.3 | chr7:30161267-30169727 |
| 23527 | Zfp148 | NM_011749.4 | chr16:33380774-33503903 |
| 23528 | Zfp157 | NM_028130.3 | chr5:138441475-138460694 |
| 23529 | Zfp160 | NM_145483.2 | chr17:21008940-21028856 |
| 23530 | Zfp169 | NM_001164575.1 | chr13:48487659-48513410 |
| 23531 | Zfp174 | NM_001081217.1 | chr16:3847222-3858880 |
| 23532 | Zfp180 | NM_001045486.2 | chr7:24081896-24107708 |
| 23533 | Zfp182 | NM_001013387.2 | chrX:21026183-21062038 |
| 23534 | Zfp184 | NM_183014.1 | chr13:21945093-21960485 |
| 23535 | Zfp185 | NM_001109043.1 | chrX:72987338-73031543 |
| 23536 | Zfp189 | NM_001289901.1 | chr4:49521175-49531558 |
| 23537 | Zfp191 | NM_021559.2 | chr18:24012266-24020771 |
| 23538 | Zfp2 | NM_001044697.2 | chr11:50898711-50916176 |
| 23539 | Zfp202 | NM_030713.2 | chr9:40192315-40213604 |
| 23540 | Zfp207 | NM_001130169.1 | chr11:80383278-80396248 |
| 23541 | Zfp212 | NM_001145881.1 | chr6:47920567-47932637 |
| 23542 | Zfp213 | NM_001033496.3 | chr17:23556766-23564226 |
| 23543 | Zfp217 | NM_001033299.3 | chr2:170108642-170131220 |
| 23544 | Zfp219 | NM_001253694.1 | chr14:52006085-52019713 |
| 23545 | Zfp229 | NM_001164676.1 | chr17:21733723-21748969 |
| 23546 | Zfp235 | NM_019941.2 | chr7:24134162-24143241 |
| 23547 | Zfp236 | NM_177832.3 | chr18:82593596-82692734 |
| 23548 | Zfp239 | NM_001001792.1 | chr6:117863076-117872766 |
| 23549 | Zfp248 | NM_028335.2 | chr6:118427318-118455506 |
| 23550 | Zfp251 | NM_001007568.2 | chr15:76852140-76871435 |
| 23551 | Zfp26 | NM_011753.3 | chr9:20428317-20460160 |
| 23552 | Zfp260 | NM_011981.4 | chr7:30095075-30107614 |
| 23553 | Zfp263 | NM_148924.3 | chr16:3744098-3750788 |
| 23554 | Zfp266 | NM_001082485.1 | chr9:20495068-20521419 |
| 23555 | Zfp27 | NM_001037707.1 | chr7:29893336-29906104 |
| 23556 | Zfp273 | NM_198322.3 | chr13:67813815-67827000 |
| 23557 | Zfp275 | NM_001160229.1 | chrX:73342620-73359079 |
| 23558 | Zfp276 | NM_020497.2 | chr8:123254194-123270551 |
| 23559 | Zfp277 | NM_172575.3 | chr12:40315045-40445790 |
| 23560 | Zfp28 | NM_175247.3 | chr7:6383317-6396637 |
| 23561 | Zfp280b | NM_177475.3 | chr10:76032611-76042969 |
| 23562 | Zfp280c | NM_001166648.1 | chrX:48541625-48594373 |
| 23563 | Zfp280d | NM_146224.5 | chr9:72274887-72363771 |
| 23564 | Zfp281 | NM_001160251.1 | chr1:136624900-136630391 |
| 23565 | Zfp282 | NM_146175.3 | chr6:47877554-47908484 |
| 23566 | Zfp286 | NM_138949.3 | chr11:62778386-62789417 |
| 23567 | Zfp287 | NM_133208.2 | chr11:62711485-62729093 |
| 23568 | Zfp292 | NM_013889.2 | chr4:34803109-34882948 |
| 23569 | Zfp296 | NM_022409.2 | chr7:19577286-19580656 |
| 23570 | Zfp3 | NM_177565.3 | chr11:70764446-70772928 |
| 23571 | Zfp30 | NM_013705.1 | chr7:29784789-29794540 |
| 23572 | Zfp300 | NM_183185.3 | chrX:21079149-21089229 |
| 23573 | Zfp316 | NM_017467.3 | chr5:143249694-143270022 |
| 23574 | Zfp317 | NM_172918.4 | chr9:19622090-19649731 |
| 23575 | Zfp318 | NM_021346.2 | chr17:46383765-46420918 |
| 23576 | Zfp319 | NM_024467.3 | chr8:95326135-95331950 |
| 23577 | Zfp322a | NM_001111107.2 | chr13:23353102-23369208 |
| 23578 | Zfp324 | NM_178732.3 | chr7:12965863-12973822 |
| 23579 | Zfp326 | NM_018759.2 | chr5:105876567-105915820 |
| 23580 | Zfp329 | NM_026046.3 | chr7:12803779-12818860 |
| 23581 | Zfp330 | NM_145600.1 | chr8:82763619-82774126 |
| 23582 | Zfp334 | NM_178411.3 | chr2:165377435-165388259 |
| 23583 | Zfp335 | NM_199027.2 | chr2:164891891-164911750 |
| 23584 | Zfp341 | NM_199304.1 | chr2:154613367-154646817 |
| 23585 | Zfp345 | NM_001034900.3 | chr2:150470990-150485063 |
| 23586 | Zfp346 | NM_012017.2 | chr13:55105308-55135071 |
| 23587 | Zfp35 | NM_011755.2 | chr18:23989633-24005371 |
| 23588 | Zfp352 | NM_153102.3 | chr4:90218819-90225687 |
| 23589 | Zfp354a | NM_009329.3 | chr11:51059256-51072799 |
| 23590 | Zfp354b | NM_013744.3 | chr11:50921785-50931635 |
| 23591 | Zfp354c | NM_013922.4 | chr11:50811084-50827731 |
| 23592 | Zfp358 | NM_080461.2 | chr8:3493137-3497208 |
| 23593 | Zfp36 | NM_011756.4 | chr7:28376783-28379228 |
| 23594 | Zfp362 | NM_001081098.1 | chr4:128773084-128806112 |
| 23595 | Zfp365 | NM_178679.2 | chr10:67886104-67912662 |
| 23596 | Zfp366 | NM_001004149.1 | chr13:99184822-99247032 |
| 23597 | Zfp367 | NM_175494.4 | chr13:64133056-64153199 |
| 23598 | Zfp369 | NM_178364.5 | chr13:65278853-65297795 |
| 23599 | Zfp36l1 | NM_007564.5 | chr12:80107759-80113013 |
| 23600 | Zfp36l2 | NM_001001806.2 | chr17:84183927-84187947 |
| 23601 | Zfp36l3 | NM_001009549.2 | chrX:53772685-53776394 |
| 23602 | Zfp37 | NM_001290350.1 | chr4:62189536-62208546 |
| 23603 | Zfp382 | NM_001081007.1 | chr7:30121947-30135032 |
| 23604 | Zfp383 | NM_001243908.1 | chr7:29908516-29916813 |
| 23605 | Zfp384 | NM_001252083.1 | chr6:125009237-125037870 |
| 23606 | Zfp385a | NM_013866.2 | chr15:103313894-103340086 |
| 23607 | Zfp385b | NM_001113399.1 | chr2:77410626-77703272 |
| 23608 | Zfp385c | NM_177790.4 | chr11:100626218-100650693 |
| 23609 | Zfp386 | NM_001004066.3 | chr12:116047723-116063207 |
| 23610 | Zfp389 | NR_026798.1 | chr13:21504315-21506524 |
| 23611 | Zfp39 | NM_011758.2 | chr11:58888152-58904225 |
| 23612 | Zfp395 | NM_199029.2 | chr14:65358675-65398930 |
| 23613 | Zfp397 | NM_027007.2 | chr18:23954687-23964670 |
| 23614 | Zfp398 | NM_027477.3 | chr6:47835660-47868257 |
| 23615 | Zfp40 | NM_009555.2 | chr17:23173868-23193228 |
| 23616 | Zfp407 | NM_001033341.2 | chr18:84207701-84589504 |
| 23617 | Zfp408 | NM_001033451.2 | chr2:91643687-91649791 |
| 23618 | Zfp41 | NM_001044718.2 | chr15:75616683-75625300 |
| 23619 | Zfp410 | NM_001252582.1 | chr12:84316858-84334119 |
| 23620 | Zfp414 | NM_026712.3 | chr17:33629091-33631714 |
| 23621 | Zfp418 | NM_146179.2 | chr7:7171352-7183560 |
| 23622 | Zfp42 | NM_009556.3 | chr8:43295066-43307009 |
| 23623 | Zfp420 | NM_172740.2 | chr7:29859978-29877302 |
| 23624 | Zfp422 | NM_026057.3 | chr6:116624015-116628999 |
| 23625 | Zfp423 | NM_033327.2 | chr8:87661809-87959595 |
| 23626 | Zfp426 | NM_001110309.1 | chr9:20468548-20492746 |
| 23627 | Zfp428 | NM_001290461.1 | chr7:24507086-24515682 |
| 23628 | Zfp429 | NM_001080941.1 | chr13:67389327-67399767 |
| 23629 | Zfp433 | NM_001243067.1 | chr10:81704824-81881086 |
| 23630 | Zfp438 | NM_178722.5 | chr18:5210030-5334439 |
| 23631 | Zfp442 | XM_006500041.2 | chr2:150406372-150451530 |
| 23632 | Zfp444 | NM_001146024.1 | chr7:6172512-6193104 |
| 23633 | Zfp445 | NM_173364.5 | chr9:122848909-122866006 |
| 23634 | Zfp446 | NM_001168561.1 | chr7:12977847-12985716 |
| 23635 | Zfp449 | NM_030139.4 | chrX:56346399-56365674 |
| 23636 | Zfp451 | NM_001290699.1 | chr1:33761540-33814595 |
| 23637 | Zfp454 | NM_172794.2 | chr11:50872722-50887443 |
| 23638 | Zfp455 | NM_001048204.1 | chr13:67194505-67209298 |
| 23639 | Zfp456 | NM_001001186.3 | chr13:67363583-67375763 |
| 23640 | Zfp457 | NM_001003666.2 | chr13:67292450-67306412 |
| 23641 | Zfp458 | NM_001001152.2 | chr13:67254917-67269068 |
| 23642 | Zfp459 | NM_177811.4 | chr13:67405712-67421418 |
| 23643 | Zfp46 | NM_009557.3 | chr4:136286068-136293942 |
| 23644 | Zfp462 | NM_172867.3 | chr4:54947944-55083563 |
| 23645 | Zfp467 | NM_001085415.1 | chr6:48436612-48445090 |
| 23646 | Zfp472 | NM_153063.3 | chr17:32965830-32979211 |
| 23647 | Zfp473 | NM_001289836.1 | chr7:44731481-44748349 |
| 23648 | Zfp474 | NM_025749.3 | chr18:52615914-52639830 |
| 23649 | Zfp488 | NM_001013777.2 | chr14:33967069-33978764 |
| 23650 | Zfp493 | NM_028402.2 | chr13:67779692-67789080 |
| 23651 | Zfp503 | NM_145459.3 | chr14:21983961-21989601 |
| 23652 | Zfp507 | NM_177739.3 | chr7:35772345-35802989 |
| 23653 | Zfp51 | NM_009558.4 | chr17:21450373-21465589 |
| 23654 | Zfp511 | NM_027201.1 | chr7:140036390-140040605 |
| 23655 | Zfp512 | NM_172993.3 | chr5:31452435-31481753 |
| 23656 | Zfp513 | NM_001177901.1 | chr5:31198980-31202042 |
| 23657 | Zfp516 | NM_001177464.1 | chr18:82914631-83005314 |
| 23658 | Zfp518a | NM_028319.1 | chr19:40894704-40917947 |
| 23659 | Zfp518b | NM_001081144.2 | chr5:38668483-38684826 |
| 23660 | Zfp52 | NM_144515.2 | chr17:21535538-21562601 |
| 23661 | Zfp521 | NM_145492.4 | chr18:13687013-13972733 |
| 23662 | Zfp523 | NM_172617.3 | chr17:28177417-28205886 |
| 23663 | Zfp524 | NM_025324.2 | chr7:5015507-5018488 |
| 23664 | Zfp526 | NM_175436.5 | chr7:25221424-25227495 |
| 23665 | Zfp53 | NM_013843.3 | chr17:21488987-21510477 |
| 23666 | Zfp532 | NM_207255.2 | chr18:65580229-65689436 |
| 23667 | Zfp534 | NM_001127188.2 | chr4:147674224-147702631 |

Fig.21 - 123

| | | | | | | | |
|---|---|---|---|---|---|---|---|
| 23668 | Zfp536 | NM_172385.2 | chr7:37479108-37769752 | 23765 | Zfp760 | NM_001008501.2 | chr17:21707740-21725636 |
| 23669 | Zfp54 | NM_011760.2 | chr17:21423226-21435384 | 23766 | Zfp763 | NM_028543.3 | chr17:33016863-33033381 |
| 23670 | Zfp541 | NM_001099277.1 | chr7:16071941-16096328 | 23767 | Zfp764 | NM_001167832.1 | chr7:127403667-127406822 |
| 23671 | Zfp551 | NM_001033820.3 | chr7:12415148-12422491 | 23768 | Zfp768 | NM_146202.1 | chr7:127342794-127345314 |
| 23672 | Zfp553 | NM_146201.1 | chr7:127233442-127237860 | 23769 | Zfp770 | NM_175466.4 | chr2:114193460-114201432 |
| 23673 | Zfp558 | NM_028935.1 | chr9:18454053-18473559 | 23770 | Zfp771 | NM_177362.3 | chr7:127244525-127254801 |
| 23674 | Zfp560 | NM_001004190.3 | chr9:20345135-20385158 | 23771 | Zfp772 | NM_145577.2 | chr7:7202121-7209998 |
| 23675 | Zfp563 | NM_001024950.2 | chr17:33089366-33110704 | 23772 | Zfp773 | NM_029584.1 | chr7:7130677-7136755 |
| 23676 | Zfp566 | NM_152814.2 | chr7:30077336-30090510 | 23773 | Zfp775 | NM_173429.2 | chr6:48613179-48623227 |
| 23677 | Zfp568 | NM_001033355.3 | chr7:29983954-30028282 | 23774 | Zfp777 | NM_001081382.1 | chr6:48024187-48048114 |
| 23678 | Zfp57 | NM_001013745.2 | chr17:37002524-37010729 | 23775 | Zfp78 | NM_001025163.1 | chr7:6363307-6382605 |
| 23679 | Zfp572 | NR_045613.1 | chr15:59306947-59312013 | 23776 | Zfp780b | NM_001081021.1 | chr7:27959799-27979157 |
| 23680 | Zfp574 | NM_001168506.1 | chr7:25077204-25083492 | 23777 | Zfp781 | NM_199062.1 | chr10:81742820-81930480 |
| 23681 | Zfp575 | NM_001033205.3 | chr7:24583837-24587641 | 23778 | Zfp783 | NR_027963.1 | chr6:47943174-47954549 |
| 23682 | Zfp579 | NM_026741.2 | chr7:4992851-4996101 | 23779 | Zfp784 | NM_001039532.2 | chr7:5034445-5038446 |
| 23683 | Zfp58 | NM_001007575.2 | chr13:67490166-67500522 | 23780 | Zfp786 | NM_177882.4 | chr6:47819265-47830505 |
| 23684 | Zfp580 | NM_026900.1 | chr7:5051531-5053723 | 23781 | Zfp787 | NM_001013012.1 | chr7:6131488-6155971 |
| 23685 | Zfp583 | NM_001033249.3 | chr7:6315664-6330434 | 23782 | Zfp788 | NM_023363.2 | chr7:41633530-41651532 |
| 23686 | Zfp59 | NM_011762.3 | chr7:27838583-27856774 | 23783 | Zfp790 | NM_001145880.1 | chr7:29816071-29832042 |
| 23687 | Zfp592 | NM_178707.4 | chr7:80993683-81045162 | 23784 | Zfp791 | NM_001037745.1 | chr8:85109166-85123095 |
| 23688 | Zfp593 | NM_024215.2 | chr4:134243305-134245591 | 23785 | Zfp799 | NM_177359.5 | chr17:32815452-32830261 |
| 23689 | Zfp595 | NM_177622.3 | chr13:67312997-67332560 | 23786 | Zfp800 | NM_001081678.1 | chr6:28239930-28261601 |
| 23690 | Zfp597 | NM_001033159.2 | chr16:3861543-3872374 | 23787 | Zfp804a | NM_175513.3 | chr2:82053657-82259878 |
| 23691 | Zfp598 | NM_183149.1 | chr17:24669751-24682016 | 23788 | Zfp804b | NM_001163223.1 | chr5:6769029-7344378 |
| 23692 | Zfp599 | NM_181419.3 | chr9:22247429-22259895 | 23789 | Zfp808 | NM_001039239.2 | chr13:62129889-62173936 |
| 23693 | Zfp60 | NM_009560.2 | chr7:27731408-27751689 | 23790 | Zfp809 | NM_001164624.1 | chr9:22225711-22239693 |
| 23694 | Zfp600 | NM_001177545.2 | chr4:146157689-146198756 | 23791 | Zfp81 | NM_207541.1 | chr17:33333727-33358878 |
| 23695 | Zfp605 | NM_001163996.1 | chr5:110110091-110129794 | 23792 | Zfp810 | NM_145612.2 | chr9:22276747-22307638 |
| 23696 | Zfp606 | NM_001039951.2 | chr7:12478304-12482714 | 23793 | Zfp811 | NM_001267583.1 | chr17:32795675-32809931 |
| 23697 | Zfp607 | NM_001024726.2 | chr7:27860584-27880825 | 23794 | Zfp819 | NM_028913.3 | chr7:43607168-43618279 |
| 23698 | Zfp608 | NM_175751.4 | chr18:54888044-54990180 | 23795 | Zfp82 | NM_001252519.1 | chr7:30056033-30072823 |
| 23699 | Zfp609 | NM_172536.3 | chr9:65691582-65827564 | 23796 | Zfp820 | NM_029281.2 | chr17:21816875-21845759 |
| 23700 | Zfp61 | NM_009561.2 | chr7:24291045-24299549 | 23797 | Zfp821 | NM_001167946.2 | chr8:109705545-109724932 |
| 23701 | Zfp612 | NM_175480.4 | chr8:110079733-110092752 | 23798 | Zfp825 | NM_146231.1 | chr13:74480056-74493950 |
| 23702 | Zfp616 | NM_001177570.1 | chr11:74070169-74087301 | 23799 | Zfp827 | NM_178267.3 | chr8:79028436-79193766 |
| 23703 | Zfp617 | NM_133358.3 | chr8:71922824-71934630 | 23800 | Zfp830 | NM_025884.4 | chr11:82764344-82767622 |
| 23704 | Zfp618 | NM_028326.1 | chr4:62965573-63134030 | 23801 | Zfp831 | NM_001099328.1 | chr2:174643533-174710830 |
| 23705 | Zfp619 | NM_001004139.2 | chr7:39517765-39540415 | 23802 | Zfp839 | NM_001199785.1 | chr12:110850278-110869998 |
| 23706 | Zfp62 | NM_001024846.1 | chr11:49203499-49218816 | 23803 | Zfp84 | NM_023750.2 | chr7:29768551-29781419 |
| 23707 | Zfp622 | NM_144523.2 | chr15:25984365-25998482 | 23804 | Zfp846 | NM_172919.1 | chr9:20581327-20595193 |
| 23708 | Zfp623 | NM_030199.3 | chr15:75940951-75949400 | 23805 | Zfp85 | NM_001001130.2 | chr13:67747799-67755134 |
| 23709 | Zfp628 | NM_170759.2 | chr7:4915216-4922003 | 23806 | Zfp850 | NM_001254951.1 | chr7:27984470-28014115 |
| 23710 | Zfp629 | NM_177226.5 | chr7:127607034-127614433 | 23807 | Zfp85os | NR_027969.1 | chr13:67729261-67756838 |
| 23711 | Zfp637 | NM_177684.2 | chr6:117841241-117845956 | 23808 | Zfp862-ps | NR_015597.1 | chr6:48504338-48534831 |
| 23712 | Zfp639 | NM_001161818.1 | chr3:32510549-32520833 | 23809 | Zfp865 | NM_001033383.2 | chr7:5020375-5033223 |
| 23713 | Zfp64 | NM_009564.2 | chr2:168925360-168955587 | 23810 | Zfp866 | NM_177899.3 | chr8:69761323-69774911 |
| 23714 | Zfp641 | NM_173769.3 | chr15:98286121-98296083 | 23811 | Zfp867 | NM_178417.3 | chr11:59461196-59472474 |
| 23715 | Zfp644 | NM_026856.2 | chr5:106616740-106696830 | 23812 | Zfp868 | NM_001045553.1 | chr8:69610653-69625175 |
| 23716 | Zfp646 | NM_172749.4 | chr7:127877700-127885996 | 23813 | Zfp869 | NM_001039965.1 | chr8:69705136-69716902 |
| 23717 | Zfp647 | NM_001168276.1 | chr15:76910369-76925448 | 23814 | Zfp87 | NM_133228.3 | chr13:67515781-67526231 |
| 23718 | Zfp648 | NM_001204908.1 | chr1:154201186-154205674 | 23815 | Zfp870 | NM_207245.2 | chr17:32879220-32891603 |
| 23719 | Zfp65 | NM_145622.2 | chr13:67705305-67729173 | 23816 | Zfp871 | NM_172458.3 | chr17:32765496-32788287 |
| 23720 | Zfp651 | NM_001166644.1 | chr9:121760032-121771742 | 23817 | Zfp872 | NM_001033813.4 | chr9:22188165-22202123 |
| 23721 | Zfp652 | NM_201609.2 | chr11:95749066-95764713 | 23818 | Zfp873 | NM_001024626.2 | chr10:82048126-82061586 |
| 23722 | Zfp652os | NR_045780.1 | chr11:95700105-95712754 | 23819 | Zfp874a | NM_177712.4 | chr13:67440430-67451624 |
| 23723 | Zfp653 | NM_177318.3 | chr9:22055410-22071376 | 23820 | Zfp874b | NM_001076791.2 | chr13:67471512-67484253 |
| 23724 | Zfp654 | NM_028059.2 | chr16:64780346-64786321 | 23821 | Zfp879 | NM_001290779.1 | chr11:50832030-50841552 |
| 23725 | Zfp655 | NM_001083958.1 | chr5:145231714-145238322 | 23822 | Zfp882 | NM_001166645.1 | chr8:71908605-71918851 |
| 23726 | Zfp658 | NM_001008549.2 | chr7:43562369-43575461 | 23823 | Zfp9 | NM_011763.2 | chr6:118461949-118479273 |
| 23727 | Zfp661 | NM_001111029.1 | chr2:127575534-127584677 | 23824 | Zfp90 | NM_011764.3 | chr8:106415338-106425889 |
| 23728 | Zfp663 | NM_001005425.1 | chr2:165351296-165362119 | 23825 | Zfp91 | NM_053009.3 | chr19:12766938-12796123 |
| 23729 | Zfp664 | NM_001081750.1 | chr5:124862704-124888630 | 23826 | Zfp91Cntf | NR_024093.1 | chr19:12763527-12796123 |
| 23730 | Zfp667 | NM_001024928.2 | chr7:6286579-6307883 | 23827 | Zfp92 | NM_009566.5 | chrX:73411095-73426998 |
| 23731 | Zfp668 | NM_146259.3 | chr7:127865358-127876823 | 23828 | Zfp93 | NM_009567.4 | chr7:24270417-24277794 |
| 23732 | Zfp672 | NM_001256516.1 | chr11:58315113-58323365 | 23829 | Zfp930 | NM_001013379.2 | chr8:69209045-69230539 |
| 23733 | Zfp677 | NM_172486.2 | chr17:21383747-21399265 | 23830 | Zfp931 | NM_001162922.1 | chr2:178066877-178078425 |
| 23734 | Zfp68 | NM_001044747.2 | chr5:138603651-138619743 | 23831 | Zfp932 | NM_145563.2 | chr5:109996526-110010411 |
| 23735 | Zfp687 | NM_030074.2 | chr3:95006701-95015238 | 23832 | Zfp933 | NM_198619.2 | chr4:147822985-147848375 |
| 23736 | Zfp688 | NM_026999.4 | chr7:127418965-127422034 | 23833 | Zfp934 | NM_001162911.1 | chr13:62516796-62558599 |
| 23737 | Zfp689 | NM_175163.3 | chr7:127442135-127449158 | 23834 | Zfp935 | NM_001136496.1 | chr13:62453015-62466812 |
| 23738 | Zfp69 | NM_001005788.3 | chr4:120930136-120951701 | 23835 | Zfp936 | NM_001034893.1 | chr7:43177587-43192109 |
| 23739 | Zfp691 | NM_001145935.1 | chr4:119169517-119173856 | 23836 | Zfp937 | NM_001142411.2 | chr2:150218098-150244874 |
| 23740 | Zfp692 | NM_001040686.1 | chr11:58307068-58314613 | 23837 | Zfp938 | NM_001105557.2 | chr10:82224855-82241275 |
| 23741 | Zfp697 | NM_172863.4 | chr3:98382480-98431949 | 23838 | Zfp939 | NM_001243021.1 | chr7:39449517-39477416 |
| 23742 | Zfp7 | NM_145916.2 | chr15:76879275-76892394 | 23839 | Zfp94 | NM_001199321.1 | chr7:24301703-24316666 |
| 23743 | Zfp703 | NM_001101502.1 | chr8:26977335-26981462 | 23840 | Zfp940 | NM_173738.2 | chr7:29843935-29853648 |
| 23744 | Zfp704 | NM_133218.2 | chr3:9427009-9610085 | 23841 | Zfp941 | NM_001001180.2 | chr7:140809676-140822178 |
| 23745 | Zfp706 | NM_026521.4 | chr15:36997026-37007402 | 23842 | Zfp942 | NM_001199048.1 | chr17:21926962-21962464 |
| 23746 | Zfp707 | NM_001081065.1 | chr15:75969184-75975865 | 23843 | Zfp943 | NM_001025373.2 | chr17:21962558-21994366 |
| 23747 | Zfp708 | NM_001012325.2 | chr13:67069397-67097976 | 23844 | Zfp944 | NM_176962.4 | chr17:22337988-22361400 |
| 23748 | Zfp709 | NM_145624.4 | chr8:71882067-71892565 | 23845 | Zfp945 | NM_001110254.1 | chr17:22846696-22867134 |
| 23749 | Zfp710 | NM_001145999.1 | chr7:80024813-80092751 | 23846 | Zfp946 | NM_198003.2 | chr17:22424267-22456689 |
| 23750 | Zfp711 | NM_177747.3 | chrX:112600525-112635062 | 23847 | Zfp947 | NM_177596.3 | chr17:22144358-22165977 |
| 23751 | Zfp712 | NM_001166218.1 | chr13:67038593-67061170 | 23848 | Zfp948 | NM_001002008.1 | chr17:21567045-21588682 |
| 23752 | Zfp715 | NM_027264.3 | chr7:43296522-43313261 | 23849 | Zfp949 | NM_001142943.1 | chr9:88548019-88571086 |
| 23753 | Zfp719 | NM_172482.1 | chr7:43579585-43593710 | 23850 | Zfp951 | NM_001039231.3 | chr5:104813167-104860068 |
| 23754 | Zfp72 | NM_001081680.1 | chr13:74371425-74376566 | 23851 | Zfp952 | NM_001045559.1 | chr17:32993138-33005457 |
| 23755 | Zfp735 | NM_001126489.2 | chr11:73688777-73713808 | 23852 | Zfp953 | NM_001038651.3 | chr13:67339308-67360572 |
| 23756 | Zfp738 | NM_001001187.3 | chr13:67667440-67683512 | 23853 | Zfp954 | NM_172738.3 | chr7:7114682-7121476 |
| 23757 | Zfp74 | NM_178384.3 | chr7:29932790-29951893 | 23854 | Zfp955a | NM_029952.3 | chr17:33239506-33255145 |
| 23758 | Zfp740 | NM_001289690.1 | chr15:102204570-102215610 | 23855 | Zfp955b | NM_001142957.1 | chr17:33289543-33304689 |
| 23759 | Zfp746 | NM_001163475.1 | chr6:48062394-48086593 | 23856 | Zfp956 | NM_178898.4 | chr6:47953389-47965299 |
| 23760 | Zfp747 | NM_175560.3 | chr7:127372536-127376050 | 23857 | Zfp957 | NM_001033215.3 | chr14:79212354-79247367 |
| 23761 | Zfp748 | NM_001035231.3 | chr13:67538640-67553152 | 23858 | Zfp958 | NM_145591.4 | chr8:4613169-4630231 |
| 23762 | Zfp750 | NM_178763.4 | chr11:121510975-121519342 | 23859 | Zfp959 | NM_145490.4 | chr17:55892092-55898930 |
| 23763 | Zfp758 | NM_145484.2 | chr17:22361452-22377281 | 23860 | Zfp960 | NM_001005358.2 | chr17:17064102-17089631 |
| 23764 | Zfp759 | NM_172392.3 | chr13:67128227-67141787 | 23861 | Zfp961 | NM_001164581.1 | chr8:71951065-71970333 |

Fig.21 - 124

| | | | |
|---|---|---|---|
| 23862 | Zfp963 | NM_001200023.1 | chr8:69741638-69749962 |
| 23863 | Zfp964 | NM_001177527.1 | chr8:69654555-69664453 |
| 23864 | Zfp97 | NM_011765.5 | chr17:17121382-17146878 |
| 23865 | Zfpl1 | NM_024231.2 | chr19:6080762-6084891 |
| 23866 | Zfpm1 | NM_009569.3 | chr8:122282140-122337247 |
| 23867 | Zfpm2 | NM_011766.5 | chr15:40655041-41104592 |
| 23868 | Zfr | NM_011767.2 | chr15:12117850-12185449 |
| 23869 | Zfr2 | NM_001034895.3 | chr10:81233162-81252123 |
| 23870 | Zfx | NM_001044386.1 | chrX:94074630-94123407 |
| 23871 | Zfy1 | NM_009570.4 | chrY:725206-797409 |
| 23872 | Zfy2 | NM_009571.2 | chrY:2106174-2170409 |
| 23873 | Zfyve1 | NM_183154.3 | chr12:83546940-83597147 |
| 23874 | Zfyve16 | NM_173392.4 | chr13:92487748-92530810 |
| 23875 | Zfyve19 | NM_001164827.1 | chr2:119208719-119217050 |
| 23876 | Zfyve20 | NM_030081.2 | chr6:92186711-92214811 |
| 23877 | Zfyve21 | NM_026752.4 | chr12:111814169-111828388 |
| 23878 | Zfyve26 | NM_001008550.1 | chr12:79232346-79296282 |
| 23879 | Zfyve27 | NM_001164531.1 | chr19:42170566-42194592 |
| 23880 | Zfyve28 | NM_001015039.1 | chr5:34194893-34288324 |
| 23881 | Zfyve9 | NM_183300.2 | chr4:108639258-108723876 |
| 23882 | Zg16 | NM_026918.2 | chr7:127050155-127051977 |
| 23883 | Zglp1 | NM_001103168.1 | chr9:21062392-21067093 |
| 23884 | Zgpat | NM_001048148.1 | chr2:181365422-181380793 |
| 23885 | Zgrf1 | NM_197997.2 | chr3:127553488-127618023 |
| 23886 | Zhx1 | NM_001042438.2 | chr15:58047002-58076508 |
| 23887 | Zhx2 | NM_199449.2 | chr15:57694666-57839832 |
| 23888 | Zhx3 | NM_177263.3 | chr2:160770446-160872990 |
| 23889 | Zic1 | NM_009573.3 | chr9:91360344-91365799 |
| 23890 | Zic2 | NM_009574.3 | chr14:122475383-122480328 |
| 23891 | Zic3 | NM_009575.2 | chrX:58030627-58036630 |
| 23892 | Zic4 | NM_009576.2 | chr9:91368971-91389348 |
| 23893 | Zic5 | NM_022987.3 | chr14:122459159-122465658 |
| 23894 | Zik1 | NM_009577.3 | chr7:10487223-10495381 |
| 23895 | Zim1 | NM_011769.4 | chr7:6675442-6696432 |
| 23896 | Zim3 | NR_036631.2 | chr7:6955685-6976662 |
| 23897 | Zkscan1 | NM_029869.1 | chr5:138085083-138107822 |
| 23898 | Zkscan14 | NM_023322.2 | chr5:145194945-145201882 |
| 23899 | Zkscan16 | NM_001099323.2 | chr4:58943627-58958355 |
| 23900 | Zkscan17 | NM_001130529.2 | chr11:59485520-59506640 |
| 23901 | Zkscan2 | NM_001081329.1 | chr7:123478638-123500449 |
| 23902 | Zkscan3 | NM_001145778.1 | chr13:21387003-21402755 |
| 23903 | Zkscan4 | NM_001039115.2 | chr13:21478848-21485505 |
| 23904 | Zkscan5 | NM_001167944.1 | chr5:145204558-145221750 |
| 23905 | Zkscan6 | NM_026107.3 | chr11:65807174-65829239 |
| 23906 | Zkscan7 | NM_001177505.1 | chr9:122888470-122896124 |
| 23907 | Zkscan8 | NM_001251833.1 | chr13:21513220-21531114 |
| 23908 | Zmat1 | NM_175446.3 | chrX:134971372-135009209 |
| 23909 | Zmat2 | NM_025594.3 | chr18:36793922-36799660 |
| 23910 | Zmat3 | NM_009517.2 | chr3:32334793-32365665 |
| 23911 | Zmat4 | NM_001277239.1 | chr8:23669660-24063116 |
| 23912 | Zmat5 | NM_026015.2 | chr11:4704677-4737666 |
| 23913 | Zmiz1 | NM_183208.4 | chr14:25459184-25666743 |
| 23914 | Zmiz2 | NM_001005867.1 | chr11:6395124-6406162 |
| 23915 | Zmpste24 | NM_172700.2 | chr4:121059237-121098243 |
| 23916 | Zmym1 | NM_026670.4 | chr4:127047093-127061132 |
| 23917 | Zmym2 | NM_029498.3 | chr14:56887794-56962579 |
| 23918 | Zmym3 | NM_001177985.1 | chrX:101404383-101419914 |
| 23919 | Zmym4 | NM_001114399.1 | chr4:126861819-126967923 |
| 23920 | Zmym5 | NM_001253752.1 | chr14:56790584-56811716 |
| 23921 | Zmym6 | NM_001285885.1 | chr4:127077382-127124374 |
| 23922 | Zmynd10 | NM_053253.3 | chr9:107547309-107551319 |
| 23923 | Zmynd11 | NM_001199141.1 | chr13:9684835-9765314 |
| 23924 | Zmynd12 | NM_001014900.2 | chr4:119422683-119453899 |
| 23925 | Zmynd15 | NM_001029929.2 | chr11:70459621-70466199 |
| 23926 | Zmynd19 | NM_026021.4 | chr2:24949776-24960870 |
| 23927 | Zmynd8 | NM_001252584.2 | chr2:165784151-165884838 |
| 23928 | Znf41-ps | NR_040355.1 | chr4:145789514-145831978 |
| 23929 | Znf512b | NM_001164597.1 | chr2:181582102-181592461 |
| 23930 | Znfx1 | NM_001033196.2 | chr2:167035796-167063015 |
| 23931 | Znhit1 | NM_027318.4 | chr5:136982193-136987959 |
| 23932 | Znhit2 | NM_013859.2 | chr19:6061206-6062468 |
| 23933 | Znhit3 | NM_001005223.2 | chr11:84910954-84916356 |
| 23934 | Znhit6 | NM_001081094.1 | chr3:145576207-145605245 |
| 23935 | Znrd1 | NM_023162.4 | chr17:36954357-36958428 |
| 23936 | Znrd1as | NM_029602.1 | chr17:36958591-36965623 |
| 23937 | Znrf1 | NM_001168621.1 | chr8:111536639-111626030 |
| 23938 | Znrf2 | NM_199143.2 | chr6:54816915-54890224 |
| 23939 | Znrf3 | NM_001080924.2 | chr11:5276328-5444847 |
| 23940 | Znrf4 | NM_011483.2 | chr17:56511247-56512483 |
| 23941 | Zp1 | NM_009580.2 | chr19:10914295-10920601 |
| 23942 | Zp2 | NM_011775.6 | chr7:120132360-120145290 |
| 23943 | Zp3 | NM_011776.1 | chr5:135980104-135988624 |
| 23944 | Zp3r | NM_009581.2 | chr1:130576705-130629606 |
| 23945 | Zp4-ps | NR_027813.1 | chr13:11522051-11525682 |
| 23946 | Zpbp | NM_001185153.1 | chr11:11280039-11462419 |
| 23947 | Zpbp2 | NM_001166494.1 | chr11:98551096-98558665 |
| 23948 | Zpld1 | NM_178720.4 | chr16:55225174-55283237 |
| 23949 | Zpr1 | NM_011752.2 | chr9:46273063-46282642 |
| 23950 | Zranb1 | NM_207302.1 | chr7:132949621-132983951 |
| 23951 | Zranb2 | NM_017381.2 | chr3:157534396-157548339 |
| 23952 | Zranb3 | NM_001285945.1 | chr1:127954178-128103047 |
| 23953 | Zrsr1 | NM_011663.3 | chr11:22972004-22976496 |
| 23954 | Zrsr2 | NM_009453.3 | chrX:163935442-163958666 |
| 23955 | Zscan10 | NM_001033425.4 | chr17:23600825-23611019 |
| 23956 | Zscan12 | NM_016684.2 | chr13:21362819-21372302 |
| 23957 | Zscan18 | NM_001017955.2 | chr7:12768091-12775658 |
| 23958 | Zscan2 | NM_009553.2 | chr7:80862107-80876513 |
| 23959 | Zscan20 | NM_177758.4 | chr4:128583538-128610098 |
| 23960 | Zscan21 | NM_001044703.2 | chr5:138116904-138134265 |
| 23961 | Zscan22 | NM_001001447.3 | chr7:12897808-12909083 |
| 23962 | Zscan25 | NM_001081431.1 | chr5:145283342-145291469 |
| 23963 | Zscan26 | NM_001013786.2 | chr13:21442174-21453727 |
| 23964 | Zscan29 | NM_001290819.1 | chr2:121158272-121171125 |
| 23965 | Zscan4a | NR_033707.1 | chr7:10794240-10799065 |
| 23966 | Zscan4b | NM_001185173.1 | chr7:10900739-10905050 |
| 23967 | Zscan4c | NM_001013765.2 | chr7:11005744-11010547 |
| 23968 | Zscan4d | NM_001100186.1 | chr7:11161642-11166148 |
| 23969 | Zscan4e | NM_001161802.1 | chr7:11306369-11310682 |
| 23970 | Zscan4f | NM_001110316.2 | chr7:11397914-11402318 |
| 23971 | Zscan5b | NM_133204.2 | chr7:6222277-6239412 |
| 23972 | Zswim1 | NM_028028.2 | chr2:164822685-164826867 |
| 23973 | Zswim2 | NM_027964.2 | chr2:83915078-83941226 |
| 23974 | Zswim3 | NM_178375.2 | chr2:164805113-164822127 |
| 23975 | Zswim4 | NM_172503.3 | chr8:84210941-84237042 |
| 23976 | Zswim5 | NM_001029912.2 | chr4:116877401-116989105 |
| 23977 | Zswim6 | NM_145456.2 | chr13:107724616-107890064 |
| 23978 | Zswim7 | NM_027198.1 | chr11:62267223-62281395 |
| 23979 | Zswim8 | NM_001252081.1 | chr14:20707551-20723619 |
| 23980 | Zufsp | NM_028287.2 | chr10:33926935-33951212 |
| 23981 | Zw10 | NM_012039.2 | chr9:49055580-49078773 |
| 23982 | Zwilch | NM_026507.4 | chr9:64137143-64172931 |
| 23983 | Zwint | NM_025635.4 | chr10:72654845-72669792 |
| 23984 | Zxda | NR_003292.1 | chrX:94791284-94798289 |
| 23985 | Zxdb | NM_001081473.1 | chrX:94724568-94730191 |
| 23986 | Zxdc | NM_030260.3 | chr6:90369493-90385394 |
| 23987 | Zyg11a | NM_001167936.1 | chr4:108181933-108217922 |
| 23988 | Zyg11b | NM_001033634.3 | chr4:108227754-108301090 |
| 23989 | Zyx | NM_001289617.1 | chr6:42349827-42360213 |
| 23990 | Zzef1 | NM_001045536.2 | chr11:72796225-72927120 |
| 23991 | Zzz3 | NM_001080755.2 | chr3:152396002-152462826 |

Fig.22 - 1

| Line No. | Groups | | | | Sub-Group | Gene Name | Human Gene ID | Updated |
|---|---|---|---|---|---|---|---|---|
| 303 | 2 | 3 | 4 | 5 | V-2 | 1700047G03Rik | | |
| 512 | 2 | 3 | 4 | 5 | V-2 | 2210409E12Rik | | |
| 563 | 2 | 3 | 4 | 5 | V-2 | 2310057J18Rik | | |
| 662 | 2 | 3 | 4 | 5 | V-2 | 2810007J24Rik | | |
| 680 | 2 | 3 | 4 | 5 | V-2 | 2810417H13Rik | | |
| 732 | 2 | 3 | 4 | 5 | V-2 | 3110082I17Rik | | |
| 1371 | 2 | 3 | 4 | 5 | V-2 | 6330410L21Rik | | |
| 1435 | 2 | 3 | 4 | 5 | V-2 | 9130204L05Rik | | |
| 1577 | 2 | 3 | 4 | 5 | V-2 | A530016L24Rik | | |
| 1953 | 2 | 3 | 4 | 5 | V-2 | Adamts12 | 81792 | 4-May-15 |
| 2009 | 2 | 3 | 4 | 5 | V-2 | Adh6-ps1 | | |
| 2163 | 2 | 3 | 4 | 5 | V-2 | Aicda | 57379 | 4-May-15 |
| 2221 | 2 | 3 | 4 | 5 | V-2 | Akr1c14 | | |
| 2222 | 2 | 3 | 4 | 5 | V-2 | Akr1c18 | | |
| 2238 | 2 | 3 | 4 | 5 | V-2 | Alas2 | 212 | 23-May-15 |
| 2249 | 2 | 3 | 4 | 5 | V-2 | Aldh1l2 | 160428 | 23-May-15 |
| 2310 | 2 | 3 | 4 | 5 | V-2 | Alx4 | 60529 | 23-May-15 |
| 2346 | 2 | 3 | 4 | 5 | V-2 | Amy2a2 | 279 | 7-Jun-15 |
| 2364 | 2 | 3 | 4 | 5 | V-2 | Ang4 | 51378 | 4-May-15 |
| 2377 | 2 | 3 | 4 | 5 | V-2 | Angptl7 | 10218 | 12-May-15 |
| 2539 | 2 | 3 | 4 | 5 | V-2 | Aplnr | 187 | 3-May-15 |
| 2543 | 2 | 3 | 4 | 5 | V-2 | Apoa1 | 335 | 31-May-15 |
| 2561 | 2 | 3 | 4 | 5 | V-2 | Apoh | 350 | 31-May-15 |
| 3027 | 2 | 3 | 4 | 5 | V-2 | AU040972 | | |
| 3063 | 2 | 3 | 4 | 5 | V-2 | AY074887 | | |
| 3260 | 2 | 3 | 4 | 5 | V-2 | BC048546 | | |
| 3340 | 2 | 3 | 4 | 5 | V-2 | Bcl6 | 604 | 17-May-15 |
| 3348 | 2 | 3 | 4 | 5 | V-2 | Bcmo1 | 53630 | 4-May-15 |
| 3463 | 2 | 3 | 4 | 5 | V-2 | Bpifa1 | 51297 | 10-May-15 |
| 3468 | 2 | 3 | 4 | 5 | V-2 | Bpifb1 | 92747 | 4-May-15 |
| 3612 | 2 | 3 | 4 | 5 | V-2 | C1qtnf6 | 114904 | 4-May-15 |
| 3663 | 2 | 3 | 4 | 5 | V-2 | C6 | 729 | 7-Jun-15 |
| 3682 | 2 | 3 | 4 | 5 | V-2 | C8b | 732 | 4-May-15 |
| 3816 | 2 | 3 | 4 | 5 | V-2 | Car1 | 759 | 2015/6/7 |
| 3825 | 2 | 3 | 4 | 5 | V-2 | Car4 | 762 | 23-May-15 |
| 3828 | 2 | 3 | 4 | 5 | V-2 | Car6 | | |
| 3886 | 2 | 3 | 4 | 5 | V-2 | Cbl | 867 | 12-May-15 |
| 4093 | 2 | 3 | 4 | 5 | V-2 | Ccl8 | 6355 | 4-May-15 |
| 4099 | 2 | 3 | 4 | 5 | V-2 | Ccnb1 | 891 | 24-May-15 |
| 4138 | 2 | 3 | 4 | 5 | V-2 | Ccr9 | 10803 | 4-May-15 |
| 4189 | 2 | 3 | 4 | 5 | V-2 | Cd27 | 939 | 4-May-15 |
| 4211 | 2 | 3 | 4 | 5 | V-2 | Cd3e | 916 | |
| 4214 | 2 | 3 | 4 | 5 | V-2 | Cd4 | 920 | 17-May-15 |
| 4227 | 2 | 3 | 4 | 5 | V-2 | Cd5l | 922 | 4-May-15 |
| 4244 | 2 | 3 | 4 | 5 | V-2 | Cd8a | 925 | 4-May-15 |
| 4245 | 2 | 3 | 4 | 5 | V-2 | Cd8b1 | 926 | 12-May-15 |
| 4347 | 2 | 3 | 4 | 5 | V-2 | Cdk6 | 1021 | 17-May-15 |
| 4506 | 2 | 3 | 4 | 5 | V-2 | Ces3a | | |
| 4507 | 2 | 3 | 4 | 5 | V-2 | Ces3b | | |
| 4531 | 2 | 3 | 4 | 5 | V-2 | Cgref1 | 10669 | |
| 4657 | 2 | 3 | 4 | 5 | V-2 | Cidea | 1149 | 4-May-15 |
| 4659 | 2 | 3 | 4 | 5 | V-2 | Cidec | 63924 | 4-May-15 |
| 4662 | 2 | 3 | 4 | 5 | V-2 | Cilp2 | 148113 | 4-May-15 |
| 4696 | 2 | 3 | 4 | 5 | V-2 | Clca3 | 9629 | 4-May-15 |
| 4738 | 2 | 3 | 4 | 5 | V-2 | Clec11a | 6320 | |
| 4750 | 2 | 3 | 4 | 5 | V-2 | Clec2h | | |
| 4830 | 2 | 3 | 4 | 5 | V-2 | Cml5 | | |
| 4935 | 2 | 3 | 4 | 5 | V-2 | Col15a1 | 1306 | |
| 4940 | 2 | 3 | 4 | 5 | V-2 | Col1a1 | 1277 | |
| 4941 | 2 | 3 | 4 | 5 | V-2 | Col1a2 | 1278 | |
| 4951 | 2 | 3 | 4 | 5 | V-2 | Col3a1 | 1281 | |
| 4959 | 2 | 3 | 4 | 5 | V-2 | Col5a1 | 1289 | |
| 4963 | 2 | 3 | 4 | 5 | V-2 | Col6a2 | 1292 | |
| 4964 | 2 | 3 | 4 | 5 | V-2 | Col6a3 | 1293 | |
| 5109 | 2 | 3 | 4 | 5 | V-2 | Cpz | 8532 | 4-May-15 |
| 5281 | 2 | 3 | 4 | 5 | V-2 | Cthrc1 | 115908 | |
| 5299 | 2 | 3 | 4 | 5 | V-2 | Ctrb1 | 1504 | 4-May-15 |
| 5334 | 2 | 3 | 4 | 5 | V-2 | Ctxn3 | 613212 | |
| 5448 | 2 | 3 | 4 | 5 | V-2 | Cyp2c54 | | |
| 5458 | 2 | 3 | 4 | 5 | V-2 | Cyp2d12 | | |
| 5465 | 2 | 3 | 4 | 5 | V-2 | Cyp2d9 | | |
| 5479 | 2 | 3 | 4 | 5 | V-2 | Cyp2u1 | 113612 | |
| 5493 | 2 | 3 | 4 | 5 | V-2 | Cyp4a12a | | |
| 5494 | 2 | 3 | 4 | 5 | V-2 | Cyp4a12b | | |
| 5516 | 2 | 3 | 4 | 5 | V-2 | Cyp7b1 | 9420 | 17-May-15 |
| 5517 | 2 | 3 | 4 | 5 | V-2 | Cyp8b1 | 1582 | 12-May-15 |
| 5582 | 2 | 3 | 4 | 5 | V-2 | D430019H16Rik | | |
| 5714 | 2 | 3 | 4 | 5 | V-2 | Dcpp1 | | |
| 5715 | 2 | 3 | 4 | 5 | V-2 | Dcpp2 | | |
| 5716 | 2 | 3 | 4 | 5 | V-2 | Dcpp3 | | |
| 5720 | 2 | 3 | 4 | 5 | V-2 | Dct | 1638 | 21-May-15 |
| 5807 | 2 | 3 | 4 | 5 | V-2 | Defa22 | | |
| 5862 | 2 | 3 | 4 | 5 | V-2 | Defb8 | | |
| 5963 | 2 | 3 | 4 | 5 | V-2 | Dio1 | 1733 | 10-Jun-15 |
| 6018 | 2 | 3 | 4 | 5 | V-2 | Dmbt1 | 1755 | 12-May-15 |
| 6129 | 2 | 3 | 4 | 5 | V-2 | Dntt | 1791 | 12-May-15 |
| 6264 | 2 | 3 | 4 | 5 | V-2 | Dupd1 | 338599 | 4-May-15 |
| 6508 | 2 | 3 | 4 | 5 | V-2 | Egfr | 1956 | 24-May-15 |
| 6617 | 2 | 3 | 4 | 5 | V-2 | Elovl3 | 83401 | 4-May-15 |
| 6653 | 2 | 3 | 4 | 5 | V-2 | Emp1 | 2012 | 4-May-15 |
| 6814 | 2 | 3 | 4 | 5 | V-2 | Esco2 | 157570 | 23-May-15 |
| 6958 | 2 | 3 | 4 | 5 | V-2 | Fabp4 | 2167 | 24-May-15 |
| 6975 | 2 | 3 | 4 | 5 | V-2 | Faim3 | 9214 | 12-May-15 |
| 7173 | 2 | 3 | 4 | 5 | V-2 | Fam64a | 54478 | 4-May-15 |
| 7409 | 2 | 3 | 4 | 5 | V-2 | Fgf15 | | |
| 7583 | 2 | 3 | 4 | 5 | V-2 | Foxn3 | 1112 | 4-May-15 |
| 7704 | 2 | 3 | 4 | 5 | V-2 | Fzd8 | 8325 | 4-May-15 |
| 7845 | 2 | 3 | 4 | 5 | V-2 | Gbp11 | | |
| 7948 | 2 | 3 | 4 | 5 | V-2 | Gh | 2688 | 7-Jun-15 |
| 7990 | 2 | 3 | 4 | 5 | V-2 | Gjb2 | 2706 | 31-May-15 |
| 8125 | 2 | 3 | 4 | 5 | V-2 | Gm10486 | | |
| 8142 | 2 | 3 | 4 | 5 | V-2 | Gm10591 | | |
| 8158 | 2 | 3 | 4 | 5 | V-2 | Gm10681 | | |
| 8258 | 2 | 3 | 4 | 5 | V-2 | Gm12238 | | |
| 8333 | 2 | 3 | 4 | 5 | V-2 | Gm13304 | | |
| 8462 | 2 | 3 | 4 | 5 | V-2 | Gm15348 | | |
| 8532 | 2 | 3 | 4 | 5 | V-2 | Gm16551 | | |
| 8682 | 2 | 3 | 4 | 5 | V-2 | Gm2083 | | |
| 8709 | 2 | 3 | 4 | 5 | V-2 | Gm21498 | | |
| 8765 | 2 | 3 | 4 | 5 | V-2 | Gm3415 | | |
| 8863 | 2 | 3 | 4 | 5 | V-2 | Gm4956 | | |
| 8920 | 2 | 3 | 4 | 5 | V-2 | Gm5424 | | |
| 8941 | 2 | 3 | 4 | 5 | V-2 | Gm5549 | | |
| 9016 | 2 | 3 | 4 | 5 | V-2 | Gm6300 | | |
| 9111 | 2 | 3 | 4 | 5 | V-2 | Gm7849 | | |
| 9113 | 2 | 3 | 4 | 5 | V-2 | Gm7861 | | |
| 9136 | 2 | 3 | 4 | 5 | V-2 | Gm853 | | |
| 9277 | 2 | 3 | 4 | 5 | V-2 | Golt1a | 127845 | 4-May-15 |
| 9318 | 2 | 3 | 4 | 5 | V-2 | Gpcpd1 | 56261 | 4-May-15 |
| 9530 | 2 | 3 | 4 | 5 | V-2 | Gsdmc | 56169 | 4-May-15 |
| 9531 | 2 | 3 | 4 | 5 | V-2 | Gsdmc2 | | |
| 9532 | 2 | 3 | 4 | 5 | V-2 | Gsdmc3 | | |
| 9533 | 2 | 3 | 4 | 5 | V-2 | Gsdmc4 | | |
| 9732 | 2 | 3 | 4 | 5 | V-2 | Hao1 | 54363 | 12-May-15 |
| 9951 | 2 | 3 | 4 | 5 | V-2 | Hist1h4c | 8364 | 4-May-15 |
| 9954 | 2 | 3 | 4 | 5 | V-2 | Hist1h4h | 8365 | 12-May-15 |
| 9955 | 2 | 3 | 4 | 5 | V-2 | Hist1h4i | 8294 | 2-Jun-15 |
| 9956 | 2 | 3 | 4 | 5 | V-2 | Hist1h4j | 8363 | 12-May-15 |
| 9958 | 2 | 3 | 4 | 5 | V-2 | Hist1h4m | | |
| 10127 | 2 | 3 | 4 | 5 | V-2 | Hrg | 3273 | 7-Jun-15 |
| 10167 | 2 | 3 | 4 | 5 | V-2 | Hsd3b5 | | |
| 10316 | 2 | 3 | 4 | 5 | V-2 | Ifitm7 | | |
| 10707 | 2 | 3 | 4 | 5 | V-2 | Itln1 | 55600 | 17-May-15 |
| 10832 | 2 | 3 | 4 | 5 | V-2 | Kcne3 | 10008 | 23-May-15 |
| 10867 | 2 | 3 | 4 | 5 | V-2 | Kcnk10 | 54207 | 4-May-15 |
| 10984 | 2 | 3 | 4 | 5 | V-2 | Kif20b | 9585 | 21-May-15 |
| 11019 | 2 | 3 | 4 | 5 | V-2 | Kiss1 | 3814 | 7-Jun-15 |
| 11104 | 2 | 3 | 4 | 5 | V-2 | Klk1b21 | | |
| 11106 | 2 | 3 | 4 | 5 | V-2 | Klk1b24 | | |
| 11108 | 2 | 3 | 4 | 5 | V-2 | Klk1b27 | | |
| 11113 | 2 | 3 | 4 | 5 | V-2 | Klk1b8 | | |
| 11301 | 2 | 3 | 4 | 5 | V-2 | Ky | 339855 | 4-May-15 |
| 11396 | 2 | 3 | 4 | 5 | V-2 | Lck | 3932 | 4-May-15 |
| 11414 | 2 | 3 | 4 | 5 | V-2 | Lct | 3938 | 12-May-15 |
| 11433 | 2 | 3 | 4 | 5 | V-2 | Lect1 | 11061 | 4-May-15 |
| 11535 | 2 | 3 | 4 | 5 | V-2 | Lipf | 8513 | 4-May-15 |
| 11701 | 2 | 3 | 4 | 5 | V-2 | Lrrc16a | 55604 | 17-May-15 |
| 11711 | 2 | 3 | 4 | 5 | V-2 | Lrrc26 | 389816 | 12-May-15 |
| 11813 | 2 | 3 | 4 | 5 | V-2 | Ltf | 4057 | 23-May-15 |
| 11876 | 2 | 3 | 4 | 5 | V-2 | Lyz1 | | |
| 12007 | 2 | 3 | 4 | 5 | V-2 | Map3k7cl | 56911 | 4-May-15 |
| 12143 | 2 | 3 | 4 | 5 | V-2 | Mcm10 | 55388 | 4-May-15 |
| 12159 | 2 | 3 | 4 | 5 | V-2 | Mcpt1 | 1375 | 4-May-15 |
| 12160 | 2 | 3 | 4 | 5 | V-2 | Mcpt2 | | |
| 12227 | 2 | 3 | 4 | 5 | V-2 | Mef2b | 4207 | 7-Jun-15 |
| 12277 | 2 | 3 | 4 | 5 | V-2 | Mettl21c | 196541 | 4-May-15 |
| 12365 | 2 | 3 | 4 | 5 | V-2 | Mid1 | 4281 | 7-Jun-15 |
| 12412 | 2 | 3 | 4 | 5 | V-2 | Mir1199 | 102466515 | 4-May-15 |
| 12532 | 2 | 3 | 4 | 5 | V-2 | Mir1940 | | |
| 12827 | 2 | 3 | 4 | 5 | V-2 | Mir433 | 574034 | 21-May-15 |
| 12876 | 2 | 3 | 4 | 5 | V-2 | Mir486 | 619554 | 21-May-15 |
| 12935 | 2 | 3 | 4 | 5 | V-2 | Mir546 | | |
| 12961 | 2 | 3 | 4 | 5 | V-2 | Mir6236 | | |
| 12966 | 2 | 3 | 4 | 5 | V-2 | Mir6244 | | |
| 12989 | 2 | 3 | 4 | 5 | V-2 | Mir6360 | | |
| 13048 | 2 | 3 | 4 | 5 | V-2 | Mir6516 | 102466864 | 4-May-15 |
| 13194 | 2 | 3 | 4 | 5 | V-2 | Mir6981 | | |
| 13281 | 2 | 3 | 4 | 5 | V-2 | Mir7060 | | |
| 13421 | 2 | 3 | 4 | 5 | V-2 | Mir8091 | | |
| 13426 | 2 | 3 | 4 | 5 | V-2 | Mir8096 | | |
| 13432 | 2 | 3 | 4 | 5 | V-2 | Mir8102 | | |
| 13444 | 2 | 3 | 4 | 5 | V-2 | Mir8114 | | |
| 13647 | 2 | 3 | 4 | 5 | V-2 | Mptx1 | 649458 | 4-May-15 |
| 13648 | 2 | 3 | 4 | 5 | V-2 | Mptx2 | | |
| 13652 | 2 | 3 | 4 | 5 | V-2 | Mpz | 4359 | 23-May-15 |
| 13657 | 2 | 3 | 4 | 5 | V-2 | Mrap | 56246 | 7-Jun-15 |
| 13779 | 2 | 3 | 4 | 5 | V-2 | Ms4a1 | 931 | 7-Jun-15 |
| 13812 | 2 | 3 | 4 | 5 | V-2 | Msln | 10232 | 4-May-15 |
| 13920 | 2 | 3 | 4 | 5 | V-2 | Mup1 | 60386 | 23-May-15 |
| 13921 | 2 | 3 | 4 | 5 | V-2 | Mup10 | | |
| 13922 | 2 | 3 | 4 | 5 | V-2 | Mup11 | | |
| 13923 | 2 | 3 | 4 | 5 | V-2 | Mup12 | | |
| 13924 | 2 | 3 | 4 | 5 | V-2 | Mup13 | | |
| 13925 | 2 | 3 | 4 | 5 | V-2 | Mup14 | | |
| 13926 | 2 | 3 | 4 | 5 | V-2 | Mup15 | | |
| 13927 | 2 | 3 | 4 | 5 | V-2 | Mup16 | | |
| 13928 | 2 | 3 | 4 | 5 | V-2 | Mup17 | | |
| 13929 | 2 | 3 | 4 | 5 | V-2 | Mup19 | | |
| 13930 | 2 | 3 | 4 | 5 | V-2 | Mup2 | | |
| 13931 | 2 | 3 | 4 | 5 | V-2 | Mup20 | | |
| 13932 | 2 | 3 | 4 | 5 | V-2 | Mup21 | | |
| 13937 | 2 | 3 | 4 | 5 | V-2 | Mup7 | | |

Fig.22 - 2

| | | | | | | | | |
|---|---|---|---|---|---|---|---|---|
| 13938 | 2 | 3 | 4 | 5 | V-2 | Mup8 | | |
| 13939 | 2 | 3 | 4 | 5 | V-2 | Mup9 | | |
| 13961 | 2 | 3 | 4 | 5 | V-2 | Myb | 4602 | 24-May-15 |
| 13963 | 2 | 3 | 4 | 5 | V-2 | Mybl1 | 4603 | 12-May-15 |
| 14051 | 2 | 3 | 4 | 5 | V-2 | Myoz3 | 91977 | 4-May-15 |
| 14143 | 2 | 3 | 4 | 5 | V-2 | Nat8 | 9027 | 4-May-15 |
| 14429 | 2 | 3 | 4 | 5 | V-2 | Nkx3-1 | 4824 | 12-May-15 |
| 14482 | 2 | 3 | 4 | 5 | V-2 | Nmrk2 | 27231 | 4-May-15 |
| 14633 | 2 | 3 | 4 | 5 | V-2 | Nrep | 9315 | 12-May-15 |
| 14747 | 2 | 3 | 4 | 5 | V-2 | Nuggc | 389643 | 4-May-15 |
| 16008 | 2 | 3 | 4 | 5 | V-2 | Onecut1 | 3175 | 12-May-15 |
| 16080 | 2 | 3 | 4 | 5 | V-2 | Ostn | 344901 | 4-May-15 |
| 16126 | 2 | 3 | 4 | 5 | V-2 | Oxtr | 5021 | 31-May-15 |
| 16226 | 2 | 3 | 4 | 5 | V-2 | Paqr9 | 344838 | 4-May-15 |
| 16267 | 2 | 3 | 4 | 5 | V-2 | Pax5 | 5079 | 17-May-15 |
| 16444 | 2 | 3 | 4 | 5 | V-2 | Pde6a | 5145 | 23-May-15 |
| 16596 | 2 | 3 | 4 | 5 | V-2 | Pglyrp1 | 8993 | 4-May-15 |
| 16653 | 2 | 3 | 4 | 5 | V-2 | Phlda1 | 22822 | 4-May-15 |
| 16709 | 2 | 3 | 4 | 5 | V-2 | Pigr | 5284 | 12-May-15 |
| 16831 | 2 | 3 | 4 | 5 | V-2 | Pla2g4c | 8605 | 4-May-15 |
| 16843 | 2 | 3 | 4 | 5 | V-2 | Plac9a | | |
| 16917 | 2 | 3 | 4 | 5 | V-2 | Plin1 | 5346 | 12-May-15 |
| 17465 | 2 | 3 | 4 | 5 | V-2 | Prr32 | 100130 613 | 4-May-15 |
| 17696 | 2 | 3 | 4 | 5 | V-2 | Ptprcap | 5790 | 4-May-15 |
| 17772 | 2 | 3 | 4 | 5 | V-2 | Pzp | 5858 | 12-May-15 |
| 17912 | 2 | 3 | 4 | 5 | V-2 | Rag1 | 5896 | 23-May-15 |
| 17962 | 2 | 3 | 4 | 5 | V-2 | Rarres1 | 5918 | 4-May-15 |
| 18137 | 2 | 3 | 4 | 5 | V-2 | Reg3b | | |
| 18139 | 2 | 3 | 4 | 5 | V-2 | Reg3g | 130120 | 4-May-15 |
| 18166 | 2 | 3 | 4 | 5 | V-2 | Retnlb | 84666 | 4-May-15 |
| 18217 | 2 | 3 | 4 | 5 | V-2 | Rgs13 | 6003 | 7-Jun-15 |
| 18358 | 2 | 3 | 4 | 5 | V-2 | Rn45s | 100861 532 | 4-May-15 |
| 18472 | 2 | 3 | 4 | 5 | V-2 | Rnu12 | 267010 | 4-May-15 |
| 18775 | 2 | 3 | 4 | 5 | V-2 | Saa2 | 6289 | 7-Jun-15 |
| 18827 | 2 | 3 | 4 | 5 | V-2 | Sash3 | 54440 | 4-May-15 |
| 18844 | 2 | 3 | 4 | 5 | V-2 | Sbp | | |
| 18845 | 2 | 3 | 4 | 5 | V-2 | Sbpl | | |
| 18863 | 2 | 3 | 4 | 5 | V-2 | Scara5 | 286133 | 4-May-15 |
| 18874 | 2 | 3 | 4 | 5 | V-2 | Scarna3b | | |
| 18876 | 2 | 3 | 4 | 5 | V-2 | Scarna8 | 677776 | 4-May-15 |
| 18880 | 2 | 3 | 4 | 5 | V-2 | Scd2 | 79966 | 4-May-15 |
| 18881 | 2 | 3 | 4 | 5 | V-2 | Scd3 | | |
| 18882 | 2 | 3 | 4 | 5 | V-2 | Scd4 | 79966 | 4-May-15 |
| 18894 | 2 | 3 | 4 | 5 | V-2 | Scgb1b27 | | |
| 18900 | 2 | 3 | 4 | 5 | V-2 | Scgb2b12 | | |
| 18901 | 2 | 3 | 4 | 5 | V-2 | Scgb2b15 | | |
| 18902 | 2 | 3 | 4 | 5 | V-2 | Scgb2b17 | | |
| 19033 | 2 | 3 | 4 | 5 | V-2 | Selenbp2 | | |
| 19108 | 2 | 3 | 4 | 5 | V-2 | Serpina1e | | |
| 19121 | 2 | 3 | 4 | 5 | V-2 | Serpina4-ps1 | | |
| 19210 | 2 | 3 | 4 | 5 | V-2 | Sfrp5 | 6425 | 10-May-15 |
| 19501 | 2 | 3 | 4 | 5 | V-2 | Slc22a7 | 10864 | 17-May-15 |
| 19780 | 2 | 3 | 4 | 5 | V-2 | Slco1a1 | | |
| 19925 | 2 | 3 | 4 | 5 | V-2 | Smtnl2 | 342527 | 4-May-15 |
| 19980 | 2 | 3 | 4 | 5 | V-2 | Snora2b | 677794 | 4-May-15 |
| 19982 | 2 | 3 | 4 | 5 | V-2 | Snora30 | 677813 | 4-May-15 |
| 19990 | 2 | 3 | 4 | 5 | V-2 | Snora44 | 677825 | 4-May-15 |
| 20003 | 2 | 3 | 4 | 5 | V-2 | Snora78 | 677844 | 4-May-15 |
| 20021 | 2 | 3 | 4 | 5 | V-2 | Snord15b | 114599 | 4-May-15 |
| 20030 | 2 | 3 | 4 | 5 | V-2 | Snord23 | 692091 | 4-May-15 |
| 20385 | 2 | 3 | 4 | 5 | V-2 | Spt1 | 10558 | 23-May-15 |
| 20714 | 2 | 3 | 4 | 5 | V-2 | Susd4 | 55061 | 4-May-15 |
| 21117 | 2 | 3 | 4 | 5 | V-2 | Tekt1 | 83659 | 21-May-15 |
| 21265 | 2 | 3 | 4 | 5 | V-2 | Thrsp | 7069 | 4-May-15 |
| 21288 | 2 | 3 | 4 | 5 | V-2 | Tigd4 | 201798 | 4-May-15 |
| 21599 | 2 | 3 | 4 | 5 | V-2 | Tmem45b | 120224 | 4-May-15 |
| 21712 | 2 | 3 | 4 | 5 | V-2 | Tnfrsf13c | 115650 | 12-May-15 |
| 22205 | 2 | 3 | 4 | 5 | V-2 | Ttr | 7276 | 31-May-15 |
| 23012 | 2 | 3 | 4 | 5 | V-2 | Vpreb3 | 29802 | 13-May-15 |
| 23193 | 2 | 3 | 4 | 5 | V-2 | Wfdc18 | | |
| 23372 | 2 | 3 | 4 | 5 | V-2 | Zbed6 | 100381 270 | 4-May-15 |
| 23598 | 2 | 3 | 4 | 5 | V-2 | Zfp369 | | |
| 18 | 2 | 3 | 4 | 5 | V-1 | 0610040F04Rik | | |
| 138 | 2 | 3 | 4 | 5 | V-1 | 1700009N14Rik | | |
| 239 | 2 | 3 | 4 | 5 | V-1 | 1700027A15Rik | | |
| 440 | 2 | 3 | 4 | 5 | V-1 | 1810009J06Rik | | |
| 450 | 2 | 3 | 4 | 5 | V-1 | 1810019D21Rik | | |
| 466 | 2 | 3 | 4 | 5 | V-1 | 1810053B23Rik | | |
| 498 | 2 | 3 | 4 | 5 | V-1 | 2210010C04Rik | | |
| 517 | 2 | 3 | 4 | 5 | V-1 | 2300002M23Rik | | |
| 526 | 2 | 3 | 4 | 5 | V-1 | 2310002L09Rik | | |
| 536 | 2 | 3 | 4 | 5 | V-1 | 2310010J17Rik | | |
| 540 | 2 | 3 | 4 | 5 | V-1 | 2310015B20Rik | | |
| 568 | 2 | 3 | 4 | 5 | V-1 | 2310061N02Rik | | |
| 639 | 2 | 3 | 4 | 5 | V-1 | 2610528A11Rik | | |
| 687 | 2 | 3 | 4 | 5 | V-1 | 2810459M11Rik | | |
| 705 | 2 | 3 | 4 | 5 | V-1 | 2900092D14Rik | | |
| 738 | 2 | 3 | 4 | 5 | V-1 | 3425401B19Rik | | |
| 826 | 2 | 3 | 4 | 5 | V-1 | 4930412O13Rik | | |
| 1088 | 2 | 3 | 4 | 5 | V-1 | 4930571K23Rik | | |
| 1215 | 2 | 3 | 4 | 5 | V-1 | 4933411K16Rik | | |
| 1289 | 2 | 3 | 4 | 5 | V-1 | 5033404E19Rik | | |
| 1338 | 2 | 3 | 4 | 5 | V-1 | 5830403L16Rik | | |
| 1408 | 2 | 3 | 4 | 5 | V-1 | 8430408G22Rik | | |
| 1440 | 2 | 3 | 4 | 5 | V-1 | 9130230L23Rik | | |

| | | | | | | | | |
|---|---|---|---|---|---|---|---|---|
| 1444 | 2 | 3 | 4 | 5 | V-1 | 9230102K24Rik | | |
| 1446 | 2 | 3 | 4 | 5 | V-1 | 9230104L09Rik | | |
| 1449 | 2 | 3 | 4 | 5 | V-1 | 9230110F15Rik | | |
| 1608 | 2 | 3 | 4 | 5 | V-1 | A730008H23Rik | | |
| 1646 | 2 | 3 | 4 | 5 | V-1 | A930016O22Rik | | |
| 1712 | 2 | 3 | 4 | 5 | V-1 | Abcb1b | | |
| 1740 | 2 | 3 | 4 | 5 | V-1 | Abcg4 | 64137 | 12-May-15 |
| 1775 | 2 | 3 | 4 | 5 | V-1 | Abra | 137735 | 12-May-15 |
| 1795 | 2 | 3 | 4 | 5 | V-1 | Acan | 176 | 12-May-15 |
| 1806 | 2 | 3 | 4 | 5 | V-1 | Acbd7 | 414149 | 4-May-15 |
| 1826 | 2 | 3 | 4 | 5 | V-1 | Acnat2 | | |
| 1835 | 2 | 3 | 4 | 5 | V-1 | Acot3 | | |
| 1866 | 2 | 3 | 4 | 5 | V-1 | Acsm3 | 6296 | 12-May-15 |
| 1873 | 2 | 3 | 4 | 5 | V-1 | Acta1 | 58 | 23-May-15 |
| 1877 | 2 | 3 | 4 | 5 | V-1 | Actc1 | 70 | 23-May-15 |
| 1879 | 2 | 3 | 4 | 5 | V-1 | Actg2 | 72 | 12-May-15 |
| 1888 | 2 | 3 | 4 | 5 | V-1 | Actn2 | 88 | 23-May-15 |
| 1889 | 2 | 3 | 4 | 5 | V-1 | Actn3 | 89 | 29-May-15 |
| 1935 | 2 | 3 | 4 | 5 | V-1 | Adam28 | 10863 | 4-May-15 |
| 1947 | 2 | 3 | 4 | 5 | V-1 | Adam7 | 8756 | 4-May-15 |
| 2002 | 2 | 3 | 4 | 5 | V-1 | Add2 | 119 | 12-May-15 |
| 2005 | 2 | 3 | 4 | 5 | V-1 | Adh1 | 124 | 4-May-15 |
| 2010 | 2 | 3 | 4 | 5 | V-1 | Adh7 | 131 | 12-May-15 |
| 2013 | 2 | 3 | 4 | 5 | V-1 | Adig | 149685 | 4-May-15 |
| 2014 | 2 | 3 | 4 | 5 | V-1 | Adipoq | 9370 | 24-May-15 |
| 2104 | 2 | 3 | 4 | 5 | V-1 | Agr2 | 10551 | 17-May-15 |
| 2127 | 2 | 3 | 4 | 5 | V-1 | Ahsg | 197 | 12-May-15 |
| 2213 | 2 | 3 | 4 | 5 | V-1 | Akp3 | | |
| 2216 | 2 | 3 | 4 | 5 | V-1 | Akr1b3 | | |
| 2217 | 2 | 3 | 4 | 5 | V-1 | Akr1b7 | | |
| 2218 | 2 | 3 | 4 | 5 | V-1 | Akr1b8 | | |
| 2239 | 2 | 3 | 4 | 5 | V-1 | Alb | 213 | 7-Jun-15 |
| 2243 | 2 | 3 | 4 | 5 | V-1 | Aldh1a1 | 216 | 23-May-15 |
| 2245 | 2 | 3 | 4 | 5 | V-1 | Aldh1a3 | 220 | 23-May-15 |
| 2251 | 2 | 3 | 4 | 5 | V-1 | Aldh3a1 | 218 | 23-May-15 |
| 2264 | 2 | 3 | 4 | 5 | V-1 | Aldob | 229 | 12-May-15 |
| 2265 | 2 | 3 | 4 | 5 | V-1 | Aldoc | 230 | 17-May-15 |
| 2301 | 2 | 3 | 4 | 5 | V-1 | Alpk3 | 57538 | 4-May-15 |
| 2302 | 2 | 3 | 4 | 5 | V-1 | Alpl | 249 | 23-May-15 |
| 2317 | 2 | 3 | 4 | 5 | V-1 | Amd1 | 262 | 4-May-15 |
| 2339 | 2 | 3 | 4 | 5 | V-1 | Ampd1 | 270 | 12-May-15 |
| 2345 | 2 | 3 | 4 | 5 | V-1 | Amy1 | 276, 277, 278 | 7-Jun-15 |
| 2347 | 2 | 3 | 4 | 5 | V-1 | Amy2a5 | | |
| 2348 | 2 | 3 | 4 | 5 | V-1 | Amy2b | 280 | 4-May-15 |
| 2379 | 2 | 3 | 4 | 5 | V-1 | Ank1 | 286 | 12-May-15 |
| 2395 | 2 | 3 | 4 | 5 | V-1 | Ankrd1 | 27063 | 23-May-15 |
| 2405 | 2 | 3 | 4 | 5 | V-1 | Ankrd2 | 26287 | 4-May-15 |
| 2438 | 2 | 3 | 4 | 5 | V-1 | Ankrd66 | 100287 718 | 21-May-15 |
| 2462 | 2 | 3 | 4 | 5 | V-1 | Anpep | 290 | 12-May-15 |
| 2491 | 2 | 3 | 4 | 5 | V-1 | Ap1m2 | 10053 | 12-May-15 |
| 2493 | 2 | 3 | 4 | 5 | V-1 | Ap1s2 | 8905 | 21-May-15 |
| 2494 | 2 | 3 | 4 | 5 | V-1 | Ap1s3 | 130340 | 4-May-15 |
| 2545 | 2 | 3 | 4 | 5 | V-1 | Apoa2 | 336 | 7-Jun-15 |
| 2546 | 2 | 3 | 4 | 5 | V-1 | Apoa4 | 337 | 4-May-15 |
| 2547 | 2 | 3 | 4 | 5 | V-1 | Apoa5 | 116519 | 12-May-15 |
| 2550 | 2 | 3 | 4 | 5 | V-1 | Apobec2 | 10930 | 4-May-15 |
| 2556 | 2 | 3 | 4 | 5 | V-1 | Apoc3 | 345 | 12-May-15 |
| 2557 | 2 | 3 | 4 | 5 | V-1 | Apoc4 | 346 | 12-May-15 |
| 2559 | 2 | 3 | 4 | 5 | V-1 | Apoe | 348 | 24-May-15 |
| 2572 | 2 | 3 | 4 | 5 | V-1 | Apol8 | | |
| 2624 | 2 | 3 | 4 | 5 | V-1 | Arg2 | 384 | 4-May-15 |
| 2746 | 2 | 3 | 4 | 5 | V-1 | Armcx6 | 54470 | 4-May-15 |
| 2748 | 2 | 3 | 4 | 5 | V-1 | Arnt2 | 9915 | 12-May-15 |
| 2774 | 2 | 3 | 4 | 5 | V-1 | Art1 | 417 | 7-Jun-15 |
| 2794 | 2 | 3 | 4 | 5 | V-1 | Asb11 | 140456 | 4-May-15 |
| 2799 | 2 | 3 | 4 | 5 | V-1 | Asb16 | 92591 | 4-May-15 |
| 2806 | 2 | 3 | 4 | 5 | V-1 | Asb5 | 140458 | 4-May-15 |
| 2817 | 2 | 3 | 4 | 5 | V-1 | Ascl4 | 121549 | 4-May-15 |
| 2843 | 2 | 3 | 4 | 5 | V-1 | Asprv1 | 151516 | 4-May-15 |
| 2864 | 2 | 3 | 4 | 5 | V-1 | Atcayos | | |
| 2907 | 2 | 3 | 4 | 5 | V-1 | Atp10b | 23120 | 4-May-15 |
| 2926 | 2 | 3 | 4 | 5 | V-1 | Atp2a1 | 487 | 12-May-15 |
| 2931 | 2 | 3 | 4 | 5 | V-1 | Atp2b3 | 492 | 4-May-15 |
| 2962 | 2 | 3 | 4 | 5 | V-1 | Atp6v0c-ps2 | | |
| 2964 | 2 | 3 | 4 | 5 | V-1 | Atp6v0d2 | 245972 | 4-May-15 |
| 3012 | 2 | 3 | 4 | 5 | V-1 | AU015791 | | |
| 3053 | 2 | 3 | 4 | 5 | V-1 | AW549542 | | |
| 3068 | 2 | 3 | 4 | 5 | V-1 | AY761185 | | |
| 3182 | 2 | 3 | 4 | 5 | V-1 | Basp1 | 10409 | 4-May-15 |
| 3266 | 2 | 3 | 4 | 5 | V-1 | BC048679 | | |
| 3299 | 2 | 3 | 4 | 5 | V-1 | BC100530 | | |
| 3328 | 2 | 3 | 4 | 5 | V-1 | Bcl2a1b | | |
| 3337 | 2 | 3 | 4 | 5 | V-1 | Bcl2l15 | 440603 | 4-May-15 |
| 3374 | 2 | 3 | 4 | 5 | V-1 | Bex1 | 55859 | 7-Jun-15 |
| 3375 | 2 | 3 | 4 | 5 | V-1 | Bex2 | 84707 | 7-Jun-15 |
| 3376 | 2 | 3 | 4 | 5 | V-1 | Bex4 | 56271 | 4-May-15 |
| 3383 | 2 | 3 | 4 | 5 | V-1 | Bglap3 | | |
| 3392 | 2 | 3 | 4 | 5 | V-1 | Bhmt | 635 | 4-May-15 |
| 3462 | 2 | 3 | 4 | 5 | V-1 | Bpi | 671 | 4-May-15 |
| 3520 | 2 | 3 | 4 | 5 | V-1 | Bsph1 | 100131 137 | 4-May-15 |
| 3522 | 2 | 3 | 4 | 5 | V-1 | Bspry | 54836 | 4-May-15 |
| 3546 | 2 | 3 | 4 | 5 | V-1 | Btg3 | 10950 | 12-May-15 |
| 3553 | 2 | 3 | 4 | 5 | V-1 | Btnl10 | 100129 094 | 4-May-15 |
| 3633 | 2 | 3 | 4 | 5 | V-1 | C2cd4b | 388125 | 4-May-15 |

Fig.22 - 3

| | | | | | | | | |
|---|---|---|---|---|---|---|---|---|
| 3637 | 2 | 3 | 4 | 5 | V-1 | C3 | 718 | 23-May-15 |
| 3656 | 2 | 3 | 4 | 5 | V-1 | C4bp | 722, 725 | 7-Jun-15 |
| 3713 | 2 | 3 | 4 | 5 | V-1 | Cacna1s | 779 | 23-May-15 |
| 3722 | 2 | 3 | 4 | 5 | V-1 | Cacng1 | 786 | 4-May-15 |
| 3727 | 2 | 3 | 4 | 5 | V-1 | Cacng6 | 59285 | 4-May-15 |
| 3755 | 2 | 3 | 4 | 5 | V-1 | Calm4 | | |
| 3757 | 2 | 3 | 4 | 5 | V-1 | Calml3 | 810 | 4-May-15 |
| 3768 | 2 | 3 | 4 | 5 | V-1 | Camk2a | 815 | 12-May-15 |
| 3769 | 2 | 3 | 4 | 5 | V-1 | Camk2b | 816 | 4-May-15 |
| 3791 | 2 | 3 | 4 | 5 | V-1 | Cap2 | 10486 | 7-Jun-15 |
| 3801 | 2 | 3 | 4 | 5 | V-1 | Capn5 | 726 | |
| 3823 | 2 | 3 | 4 | 5 | V-1 | Car2 | 760 | 2015/6/7 |
| 3824 | 2 | 3 | 4 | 5 | V-1 | Car3 | 761 | 17-May-15 |
| 3827 | 2 | 3 | 4 | 5 | V-1 | Car5b | | |
| 3864 | 2 | 3 | 4 | 5 | V-1 | Casq1 | 844 | |
| 3882 | 2 | 3 | 4 | 5 | V-1 | Cav3 | 859 | 23-May-15 |
| 3894 | 2 | 3 | 4 | 5 | V-1 | Cbr1 | 873 | 4-May-15 |
| 3896 | 2 | 3 | 4 | 5 | V-1 | Cbr3 | 874 | 4-May-15 |
| 4074 | 2 | 3 | 4 | 5 | V-1 | Ccl17 | 6361 | |
| 4076 | 2 | 3 | 4 | 5 | V-1 | Ccl2 | 6347 | |
| 4077 | 2 | 3 | 4 | 5 | V-1 | Ccl20 | 6364 | 31-May-15 |
| 4079 | 2 | 3 | 4 | 5 | V-1 | Ccl21b | | |
| 4091 | 2 | 3 | 4 | 5 | V-1 | Ccl6 | | |
| 4120 | 2 | 3 | 4 | 5 | V-1 | Ccno | 10309 | 4-May-15 |
| 4222 | 2 | 3 | 4 | 5 | V-1 | Cd52 | 1043 | 12-May-15 |
| 4293 | 2 | 3 | 4 | 5 | V-1 | Cdcp1 | 64866 | |
| 4311 | 2 | 3 | 4 | 5 | V-1 | Cdh3 | 1001 | 2015/6/7 |
| 4386 | 2 | 3 | 4 | 5 | V-1 | Ceacam10 | | |
| 4411 | 2 | 3 | 4 | 5 | V-1 | Cel | 1056 | 12-May-15 |
| 4412 | 2 | 3 | 4 | 5 | V-1 | Cela1 | 1990 | 4-May-15 |
| 4413 | 2 | 3 | 4 | 5 | V-1 | Cela2a | 63036 | 4-May-15 |
| 4414 | 2 | 3 | 4 | 5 | V-1 | Cela3b | 23436 | 4-May-15 |
| 4493 | 2 | 3 | 4 | 5 | V-1 | Ces1c | | |
| 4509 | 2 | 3 | 4 | 5 | V-1 | Ces5a | 221223 | 4-May-15 |
| 4516 | 2 | 3 | 4 | 5 | V-1 | Cfd | 1675 | 7-Jun-15 |
| 4534 | 2 | 3 | 4 | 5 | V-1 | Chac1 | 79094 | 23-May-15 |
| 4566 | 2 | 3 | 4 | 5 | V-1 | Chga | 1113 | |
| 4674 | 2 | 3 | 4 | 5 | V-1 | Cited1 | 4435 | 4-May-15 |
| 4676 | 2 | 3 | 4 | 5 | V-1 | Cited4 | 163732 | |
| 4685 | 2 | 3 | 4 | 5 | V-1 | Ckm | 1158 | 24-May-15 |
| 4687 | 2 | 3 | 4 | 5 | V-1 | Ckmt2 | 1160 | |
| 4711 | 2 | 3 | 4 | 5 | V-1 | Cldn1 | 9076 | |
| 4715 | 2 | 3 | 4 | 5 | V-1 | Cldn13 | | |
| 4716 | 2 | 3 | 4 | 5 | V-1 | Cldn14 | 23562 | 4-May-15 |
| 4722 | 2 | 3 | 4 | 5 | V-1 | Cldn2 | 9075 | 24-May-15 |
| 4724 | 2 | 3 | 4 | 5 | V-1 | Cldn22 | 53842 | 4-May-15 |
| 4726 | 2 | 3 | 4 | 5 | V-1 | Cldn24 | 100132 463 | 4-May-15 |
| 4729 | 2 | 3 | 4 | 5 | V-1 | Cldn3 | 1365 | 4-May-15 |
| 4730 | 2 | 3 | 4 | 5 | V-1 | Cldn4 | 1364 | 3-May-15 |
| 4733 | 2 | 3 | 4 | 5 | V-1 | Cldn7 | 1366 | |
| 4734 | 2 | 3 | 4 | 5 | V-1 | Cldn8 | 9073 | 4-May-15 |
| 4752 | 2 | 3 | 4 | 5 | V-1 | Clec2l | 154790 | 4-May-15 |
| 4767 | 2 | 3 | 4 | 5 | V-1 | Clec7a | 64581 | |
| 4797 | 2 | 3 | 4 | 5 | V-1 | Clps | 1208 | 4-May-15 |
| 4798 | 2 | 3 | 4 | 5 | V-1 | Clpsl2 | 389383 | 4-May-15 |
| 4812 | 2 | 3 | 4 | 5 | V-1 | Clu | 1191 | 12-May-15 |
| 4845 | 2 | 3 | 4 | 5 | V-1 | Cmya5 | 202333 | |
| 4851 | 2 | 3 | 4 | 5 | V-1 | Cnfn | 84518 | 4-May-15 |
| 4865 | 2 | 3 | 4 | 5 | V-1 | Cnn1 | 1264 | |
| 4965 | 2 | 3 | 4 | 5 | V-1 | Col6a4 | 344875, 646300 | /5/4 |
| 5042 | 2 | 3 | 4 | 5 | V-1 | Cox6a2 | 1339 | |
| 5046 | 2 | 3 | 4 | 5 | V-1 | Cox7a1 | 1346 | |
| 5056 | 2 | 3 | 4 | 5 | V-1 | Cpa1 | 1357 | 4-May-15 |
| 5057 | 2 | 3 | 4 | 5 | V-1 | Cpa2 | 1358 | 4-May-15 |
| 5062 | 2 | 3 | 4 | 5 | V-1 | Cpb1 | 1360 | 4-May-15 |
| 5119 | 2 | 3 | 4 | 5 | V-1 | Crb3 | 92359 | 4-May-15 |
| 5122 | 2 | 3 | 4 | 5 | V-1 | Crct1 | 54544 | 4-May-15 |
| 5148 | 2 | 3 | 4 | 5 | V-1 | Crisp1 | 167 | |
| 5151 | 2 | 3 | 4 | 5 | V-1 | Crisp4 | | |
| 5177 | 2 | 3 | 4 | 5 | V-1 | Cryaa | 1409 | |
| 5180 | 2 | 3 | 4 | 5 | V-1 | Cryba2 | 1412 | 3-May-15 |
| 5181 | 2 | 3 | 4 | 5 | V-1 | Cryba4 | 1413 | 4-May-15 |
| 5188 | 2 | 3 | 4 | 5 | V-1 | Crygc | 1420 | 4-May-15 |
| 5199 | 2 | 3 | 4 | 5 | V-1 | Csad | 51380 | 4-May-15 |
| 5244 | 2 | 3 | 4 | 5 | V-1 | Csrp3 | 8048 | 23-May-15 |
| 5246 | 2 | 3 | 4 | 5 | V-1 | Cst11 | 140880 | 4-May-15 |
| 5247 | 2 | 3 | 4 | 5 | V-1 | Cst12 | | |
| 5252 | 2 | 3 | 4 | 5 | V-1 | Cst8 | 10047 | 4-May-15 |
| 5302 | 2 | 3 | 4 | 5 | V-1 | Ctrl | 1506 | 4-May-15 |
| 5350 | 2 | 3 | 4 | 5 | V-1 | Cux2 | 23316 | 4-May-15 |
| 5351 | 2 | 3 | 4 | 5 | V-1 | Cuzd1 | 50624 | 4-May-15 |
| 5362 | 2 | 3 | 4 | 5 | V-1 | Cxcl1 | 2919 | |
| 5366 | 2 | 3 | 4 | 5 | V-1 | Cxcl13 | 10563 | 3-May-15 |
| 5388 | 2 | 3 | 4 | 5 | V-1 | Cyb561 | 1534 | 12-May-15 |
| 5413 | 2 | 3 | 4 | 5 | V-1 | Cym | | |
| 5419 | 2 | 3 | 4 | 5 | V-1 | Cyp1a1 | 1543 | 12-May-15 |
| 5426 | 2 | 3 | 4 | 5 | V-1 | Cyp26b1 | 56603 | |
| 5429 | 2 | 3 | 4 | 5 | V-1 | Cyp27b1 | 1594 | 12-May-15 |
| 5432 | 2 | 3 | 4 | 5 | V-1 | Cyp2a4 | | |
| 5433 | 2 | 3 | 4 | 5 | V-1 | Cyp2a5 | | |
| 5439 | 2 | 3 | 4 | 5 | V-1 | Cyp2b9 | 1555, 1556 | 7-Jun-15 |
| 5442 | 2 | 3 | 4 | 5 | V-1 | Cyp2c38 | | |
| 5466 | 2 | 3 | 4 | 5 | V-1 | Cyp2e1 | 1571 | 24-May-15 |
| 5467 | 2 | 3 | 4 | 5 | V-1 | Cyp2f2 | | |
| 5481 | 2 | 3 | 4 | 5 | V-1 | Cyp39a1 | 51302 | |

| | | | | | | | | |
|---|---|---|---|---|---|---|---|---|
| 5482 | 2 | 3 | 4 | 5 | V-1 | Cyp3a11 | | |
| 5488 | 2 | 3 | 4 | 5 | V-1 | Cyp3a44 | | |
| 5490 | 2 | 3 | 4 | 5 | V-1 | Cyp3a59 | | |
| 5495 | 2 | 3 | 4 | 5 | V-1 | Cyp4a14 | | |
| 5610 | 2 | 3 | 4 | 5 | V-1 | D730048I06Rik | | |
| 5671 | 2 | 3 | 4 | 5 | V-1 | Dbil5 | | |
| 5719 | 2 | 3 | 4 | 5 | V-1 | Dcstamp | 81501 | 12-May-15 |
| 5745 | 2 | 3 | 4 | 5 | V-1 | Ddi2 | 84301 | 4-May-15 |
| 5748 | 2 | 3 | 4 | 5 | V-1 | Ddit4l | 115265 | 4-May-15 |
| 5809 | 2 | 3 | 4 | 5 | V-1 | Defa24 | | |
| 5821 | 2 | 3 | 4 | 5 | V-1 | Defb1 | 1672 | 10-May-15 |
| 5822 | 2 | 3 | 4 | 5 | V-1 | Defb10 | 245913 | 4-May-15 |
| 5824 | 2 | 3 | 4 | 5 | V-1 | Defb12 | 245915 | 4-May-15 |
| 5826 | 2 | 3 | 4 | 5 | V-1 | Defb14 | 245928 | 4-May-15 |
| 5827 | 2 | 3 | 4 | 5 | V-1 | Defb15 | 245929 | 4-May-15 |
| 5828 | 2 | 3 | 4 | 5 | V-1 | Defb18 | 117285 | 4-May-15 |
| 5829 | 2 | 3 | 4 | 5 | V-1 | Defb19 | 245932 | 4-May-15 |
| 5830 | 2 | 3 | 4 | 5 | V-1 | Defb2 | 1673 | 10-May-15 |
| 5831 | 2 | 3 | 4 | 5 | V-1 | Defb20 | 245932 | 4-May-15 |
| 5832 | 2 | 3 | 4 | 5 | V-1 | Defb21 | 245934 | 4-May-15 |
| 5833 | 2 | 3 | 4 | 5 | V-1 | Defb22 | 245935 | 12-May-15 |
| 5834 | 2 | 3 | 4 | 5 | V-1 | Defb23 | 245936 | 4-May-15 |
| 5835 | 2 | 3 | 4 | 5 | V-1 | Defb25 | 245938 | 4-May-15 |
| 5836 | 2 | 3 | 4 | 5 | V-1 | Defb26 | 81623 | 4-May-15 |
| 5837 | 2 | 3 | 4 | 5 | V-1 | Defb28 | 245939 | 4-May-15 |
| 5838 | 2 | 3 | 4 | 5 | V-1 | Defb29 | 140881 | 4-May-15 |
| 5840 | 2 | 3 | 4 | 5 | V-1 | Defb30 | 245940 | 4-May-15 |
| 5843 | 2 | 3 | 4 | 5 | V-1 | Defb35 | | |
| 5845 | 2 | 3 | 4 | 5 | V-1 | Defb37 | | |
| 5847 | 2 | 3 | 4 | 5 | V-1 | Defb39 | | |
| 5848 | 2 | 3 | 4 | 5 | V-1 | Defb4 | 1673 | 7-Jun-15 |
| 5850 | 2 | 3 | 4 | 5 | V-1 | Defb41 | | |
| 5851 | 2 | 3 | 4 | 5 | V-1 | Defb42 | | |
| 5852 | 2 | 3 | 4 | 5 | V-1 | Defb43 | | |
| 5854 | 2 | 3 | 4 | 5 | V-1 | Defb45 | | |
| 5856 | 2 | 3 | 4 | 5 | V-1 | Defb47 | | |
| 5857 | 2 | 3 | 4 | 5 | V-1 | Defb48 | | |
| 5885 | 2 | 3 | 4 | 5 | V-1 | Depdc7 | 91614 | 4-May-15 |
| 5890 | 2 | 3 | 4 | 5 | V-1 | Derl3 | 91319 | 3-May-15 |
| 5891 | 2 | 3 | 4 | 5 | V-1 | Des | 1674 | 23-May-15 |
| 5935 | 2 | 3 | 4 | 5 | V-1 | Dhrs7c | 201140 | 4-May-15 |
| 6023 | 2 | 3 | 4 | 5 | V-1 | Dmkn | 93099 | 21-May-15 |
| 6033 | 2 | 3 | 4 | 5 | V-1 | Dmrtc1a | | |
| 6038 | 2 | 3 | 4 | 5 | V-1 | Dmtn | 2039 | 7-Jun-15 |
| 6074 | 2 | 3 | 4 | 5 | V-1 | Dnajb8 | 165721 | 4-May-15 |
| 6107 | 2 | 3 | 4 | 5 | V-1 | Dnase1 | 1773 | 12-May-15 |
| 6126 | 2 | 3 | 4 | 5 | V-1 | Dnmt3l | 29947 | 17-May-15 |
| 6158 | 2 | 3 | 4 | 5 | V-1 | Dopey2 | 9980 | 4-May-15 |
| 6263 | 2 | 3 | 4 | 5 | V-1 | Duoxa2 | 405753 | 4-May-15 |
| 6273 | 2 | 3 | 4 | 5 | V-1 | Dusp13 | 51207 | 4-May-15 |
| 6275 | 2 | 3 | 4 | 5 | V-1 | Dusp15 | 128853 | 4-May-15 |
| 6288 | 2 | 3 | 4 | 5 | V-1 | Dusp5 | 1847 | 4-May-15 |
| 6336 | 2 | 3 | 4 | 5 | V-1 | E030003E18Rik | | |
| 6404 | 2 | 3 | 4 | 5 | V-1 | Ear3 | 7025 | 4-May-15 |
| 6442 | 2 | 3 | 4 | 5 | V-1 | Eddm3b | 64184 | 4-May-15 |
| 6457 | 2 | 3 | 4 | 5 | V-1 | Eef1a2 | 1917 | 4-May-15 |
| 6513 | 2 | 3 | 4 | 5 | V-1 | Egr2 | 1959 | 23-May-15 |
| 6560 | 2 | 3 | 4 | 5 | V-1 | Eif3j1 | | |
| 6561 | 2 | 3 | 4 | 5 | V-1 | Eif3j2 | | |
| 6605 | 2 | 3 | 4 | 5 | V-1 | Ell3 | 80237 | 4-May-15 |
| 6616 | 2 | 3 | 4 | 5 | V-1 | Elovl2 | 54898 | 4-May-15 |
| 6618 | 2 | 3 | 4 | 5 | V-1 | Elovl4 | 6785 | 12-May-15 |
| 6643 | 2 | 3 | 4 | 5 | V-1 | Emid1 | 129080 | 4-May-15 |
| 6654 | 2 | 3 | 4 | 5 | V-1 | Emp2 | 2013 | 12-May-15 |
| 6659 | 2 | 3 | 4 | 5 | V-1 | Emx2 | 2018 | 17-May-15 |
| 6678 | 2 | 3 | 4 | 5 | V-1 | Eno3 | 2027 | 12-May-15 |
| 6684 | 2 | 3 | 4 | 5 | V-1 | Enpp1 | 5167 | 23-May-15 |
| 6713 | 2 | 3 | 4 | 5 | V-1 | Epb4.1l4b | | |
| 6715 | 2 | 3 | 4 | 5 | V-1 | Epb4.2 | | |
| 6718 | 2 | 3 | 4 | 5 | V-1 | Epcam | 4072 | 24-May-15 |
| 6747 | 2 | 3 | 4 | 5 | V-1 | Eppin | 57119 | 4-May-15 |
| 6799 | 2 | 3 | 4 | 5 | V-1 | Ermap | 114625 | 12-May-15 |
| 6839 | 2 | 3 | 4 | 5 | V-1 | Esrp1 | 54845 | 4-May-15 |
| 6840 | 2 | 3 | 4 | 5 | V-1 | Esrp2 | 80004 | 4-May-15 |
| 6866 | 2 | 3 | 4 | 5 | V-1 | Etv4 | 2118 | 28-May-15 |
| 6867 | 2 | 3 | 4 | 5 | V-1 | Etv5 | 2119 | 24-May-15 |
| 6953 | 2 | 3 | 4 | 5 | V-1 | Faah | 2166 | 7-Jun-15 |
| 6954 | 2 | 3 | 4 | 5 | V-1 | Fabp1 | 2168 | 12-May-15 |
| 6967 | 2 | 3 | 4 | 5 | V-1 | Fads6 | 283985 | 7-Jun-15 |
| 6974 | 2 | 3 | 4 | 5 | V-1 | Faim2 | 23017 | 4-May-15 |
| 7111 | 2 | 3 | 4 | 5 | V-1 | Fam20a | 54757 | 30-Apr-15 |
| 7142 | 2 | 3 | 4 | 5 | V-1 | Fam25c | 644054 | 12-May-15 |
| 7203 | 2 | 3 | 4 | 5 | V-1 | Fam84a | 151354 | 12-May-15 |
| 7307 | 2 | 3 | 4 | 5 | V-1 | Fbxo40 | 51725 | 4-May-15 |
| 7395 | 2 | 3 | 4 | 5 | V-1 | Fga | 2243 | 29-May-15 |
| 7396 | 2 | 3 | 4 | 5 | V-1 | Fgb | 2244 | 29-May-15 |
| 7417 | 2 | 3 | 4 | 5 | V-1 | Fgf23 | 8074 | 17-May-15 |
| 7425 | 2 | 3 | 4 | 5 | V-1 | Fgfbp1 | 9982 | 4-May-15 |
| 7434 | 2 | 3 | 4 | 5 | V-1 | Fgg | 2266 | 31-May-15 |
| 7446 | 2 | 3 | 4 | 5 | V-1 | Fhl3 | 2275 | 7-Jun-15 |
| 7447 | 2 | 3 | 4 | 5 | V-1 | Fhl4 | 8676 | 23-May-15 |
| 7466 | 2 | 3 | 4 | 5 | V-1 | Fitm1 | 161247 | 4-May-15 |
| 7494 | 2 | 3 | 4 | 5 | V-1 | Flnc | 2318 | 23-May-15 |
| 7498 | 2 | 3 | 4 | 5 | V-1 | Flrt2 | 23768 | 4-May-15 |
| 7512 | 2 | 3 | 4 | 5 | V-1 | Fmo2 | 2327 | 12-May-15 |
| 7513 | 2 | 3 | 4 | 5 | V-1 | Fmo3 | 2328 | 24-May-15 |
| 7522 | 2 | 3 | 4 | 5 | V-1 | Fn3k | 64122 | 4-May-15 |

Fig.22 - 4

| | | | | | | | | |
|---|---|---|---|---|---|---|---|---|
| 7569 | 2 | 3 | 4 | 5 | V-1 | Foxi1 | 2299 | 23-May-15 |
| 7572 | 2 | 3 | 4 | 5 | V-1 | Foxj1 | 2302 | 12-May-15 |
| 7671 | 2 | 3 | 4 | 5 | V-1 | Fut1 | 2523 | 12-May-15 |
| 7675 | 2 | 3 | 4 | 5 | V-1 | Fut4 | 2526 | 12-May-15 |
| 7688 | 2 | 3 | 4 | 5 | V-1 | Fxyd4 | 53828 | 4-May-15 |
| 7760 | 2 | 3 | 4 | 5 | V-1 | Gadd45b | 4616 | 4-May-15 |
| 7769 | 2 | 3 | 4 | 5 | V-1 | Gal3st4 | 79690 | 4-May-15 |
| 7779 | 2 | 3 | 4 | 5 | V-1 | Galnt12 | 79695 | 4-May-15 |
| 7803 | 2 | 3 | 4 | 5 | V-1 | Gap43 | 2596 | 12-May-15 |
| 7826 | 2 | 3 | 4 | 5 | V-1 | Gata3 | 2625 | 17-May-15 |
| 7857 | 2 | 3 | 4 | 5 | V-1 | Gc | 2638 | 17-May-15 |
| 7873 | 2 | 3 | 4 | 5 | V-1 | Gcm2 | 9247 | 12-May-15 |
| 7878 | 2 | 3 | 4 | 5 | V-1 | Gcnt4 | 51301 | 4-May-15 |
| 7926 | 2 | 3 | 4 | 5 | V-1 | Gfra1 | 2674 | 12-May-15 |
| 8012 | 2 | 3 | 4 | 5 | V-1 | Glb1l3 | 112937 | 4-May-15 |
| 8015 | 2 | 3 | 4 | 5 | V-1 | Gldc | 2731 | 23-May-15 |
| 8041 | 2 | 3 | 4 | 5 | V-1 | Glrp1 | | |
| 8097 | 2 | 3 | 4 | 5 | V-1 | Gm10334 | | |
| 8126 | 2 | 3 | 4 | 5 | V-1 | Gm10487 | | |
| 8148 | 2 | 3 | 4 | 5 | V-1 | Gm10639 | | |
| 8186 | 2 | 3 | 4 | 5 | V-1 | Gm1110 | | |
| 8256 | 2 | 3 | 4 | 5 | V-1 | Gm12191 | | |
| 8338 | 2 | 3 | 4 | 5 | V-1 | Gm13363 | | |
| 8407 | 2 | 3 | 4 | 5 | V-1 | Gm14476 | | |
| 8441 | 2 | 3 | 4 | 5 | V-1 | Gm15056 | | |
| 8464 | 2 | 3 | 4 | 5 | V-1 | Gm15386 | | |
| 8491 | 2 | 3 | 4 | 5 | V-1 | Gm15816 | | |
| 8543 | 2 | 3 | 4 | 5 | V-1 | Gm1673 | | |
| 8564 | 2 | 3 | 4 | 5 | V-1 | Gm17252 | | |
| 8567 | 2 | 3 | 4 | 5 | V-1 | Gm17365 | | |
| 8571 | 2 | 3 | 4 | 5 | V-1 | Gm17677 | | |
| 8573 | 2 | 3 | 4 | 5 | V-1 | Gm17727 | | |
| 8616 | 2 | 3 | 4 | 5 | V-1 | Gm1987 | | |
| 8651 | 2 | 3 | 4 | 5 | V-1 | Gm2061 | | |
| 8728 | 2 | 3 | 4 | 5 | V-1 | Gm266 | | |
| 8729 | 2 | 3 | 4 | 5 | V-1 | Gm2663 | | |
| 8767 | 2 | 3 | 4 | 5 | V-1 | Gm3428 | | |
| 8784 | 2 | 3 | 4 | 5 | V-1 | Gm3776 | | |
| 8829 | 2 | 3 | 4 | 5 | V-1 | Gm4736 | | |
| 8844 | 2 | 3 | 4 | 5 | V-1 | Gm4846 | | |
| 8936 | 2 | 3 | 4 | 5 | V-1 | Gm5531 | | |
| 8962 | 2 | 3 | 4 | 5 | V-1 | Gm5741 | | |
| 8964 | 2 | 3 | 4 | 5 | V-1 | Gm5771 | | |
| 8984 | 2 | 3 | 4 | 5 | V-1 | Gm5916 | | |
| 8995 | 2 | 3 | 4 | 5 | V-1 | Gm6040 | | |
| 9051 | 2 | 3 | 4 | 5 | V-1 | Gm6644 | | |
| 9060 | 2 | 3 | 4 | 5 | V-1 | Gm6792 | | |
| 9093 | 2 | 3 | 4 | 5 | V-1 | Gm7325 | | |
| 9094 | 2 | 3 | 4 | 5 | V-1 | Gm7334 | | |
| 9141 | 2 | 3 | 4 | 5 | V-1 | Gm867 | | |
| 9151 | 2 | 3 | 4 | 5 | V-1 | Gm8882 | | |
| 9172 | 2 | 3 | 4 | 5 | V-1 | Gm94 | | |
| 9196 | 2 | 3 | 4 | 5 | V-1 | Gm9992 | | |
| 9207 | 2 | 3 | 4 | 5 | V-1 | Gml | 2765 | 4-May-15 |
| 9291 | 2 | 3 | 4 | 5 | V-1 | Gp2 | 2813 | 4-May-15 |
| 9335 | 2 | 3 | 4 | 5 | V-1 | Gpnmb | 10457 | 12-May-15 |
| 9418 | 2 | 3 | 4 | 5 | V-1 | Gpr82 | 27197 | 12-May-15 |
| 9449 | 2 | 3 | 4 | 5 | V-1 | Gpx5 | 2880 | 4-May-15 |
| 9450 | 2 | 3 | 4 | 5 | V-1 | Gpx6 | 257202 | 12-May-15 |
| 9472 | 2 | 3 | 4 | 5 | V-1 | Grhl2 | 79977 | 24-May-15 |
| 9539 | 2 | 3 | 4 | 5 | V-1 | Gsg1 | 83445 | 7-Jun-15 |
| 9550 | 2 | 3 | 4 | 5 | V-1 | Gsta1 | 2938 | 12-May-15 |
| 9551 | 2 | 3 | 4 | 5 | V-1 | Gsta2 | 2939 | 12-May-15 |
| 9553 | 2 | 3 | 4 | 5 | V-1 | Gsta4 | 2941 | 12-May-15 |
| 9556 | 2 | 3 | 4 | 5 | V-1 | Gstm1 | 2944 | 24-May-15 |
| 9558 | 2 | 3 | 4 | 5 | V-1 | Gstm3 | 2947 | 12-May-15 |
| 9626 | 2 | 3 | 4 | 5 | V-1 | Gulo | 2989 | 12-May-15 |
| 9636 | 2 | 3 | 4 | 5 | V-1 | Gypa | 2993 | 21-May-15 |
| 9652 | 2 | 3 | 4 | 5 | V-1 | H19 | 283120 | 23-May-15 |
| 9698 | 2 | 3 | 4 | 5 | V-1 | H2-Q10 | | |
| 9704 | 2 | 3 | 4 | 5 | V-1 | H2-Q8 | | |
| 9728 | 2 | 3 | 4 | 5 | V-1 | Hamp | 57817 | 24-May-15 |
| 9729 | 2 | 3 | 4 | 5 | V-1 | Hamp2 | | |
| 9734 | 2 | 3 | 4 | 5 | V-1 | Hap1 | 9001 | 7-Jun-15 |
| 9755 | 2 | 3 | 4 | 5 | V-1 | Havcr1 | 26762 | 13-May-15 |
| 9759 | 2 | 3 | 4 | 5 | V-1 | Hba-a2 | 3039 | 7-Jun-15 |
| 9764 | 2 | 3 | 4 | 5 | V-1 | Hbb-bs | 3044 | 9-Jun-16 |
| 9820 | 2 | 3 | 4 | 5 | V-1 | Hebp2 | 23593 | 4-May-15 |
| 9834 | 2 | 3 | 4 | 5 | V-1 | Hemgn | 55363 | 4-May-15 |
| 9836 | 2 | 3 | 4 | 5 | V-1 | Hemt1 | | |
| 9865 | 2 | 3 | 4 | 5 | V-1 | Hfe2 | 148738 | 23-May-15 |
| 9869 | 2 | 3 | 4 | 5 | V-1 | Hgfac | 3083 | 4-May-15 |
| 9902 | 2 | 3 | 4 | 5 | V-1 | Hipk2 | 28996 | 31-May-15 |
| 9961 | 2 | 3 | 4 | 5 | V-1 | Hist2h2aa2 | | |
| 9967 | 2 | 3 | 4 | 5 | V-1 | Hist2h3c1 | | |
| 9968 | 2 | 3 | 4 | 5 | V-1 | Hist2h3c2 | | |
| 10079 | 2 | 3 | 4 | 5 | V-1 | Hoxb6 | 3216 | 4-May-15 |
| 10081 | 2 | 3 | 4 | 5 | V-1 | Hoxb8 | 3218 | 4-May-15 |
| 10097 | 2 | 3 | 4 | 5 | V-1 | Hoxd3 | 3232 | 12-May-15 |
| 10108 | 2 | 3 | 4 | 5 | V-1 | Hpdl | 84842 | 7-Jun-15 |
| 10120 | 2 | 3 | 4 | 5 | V-1 | Hpx | 3263 | 4-May-15 |
| 10125 | 2 | 3 | 4 | 5 | V-1 | Hrc | 3270 | 12-May-15 |
| 10140 | 2 | 3 | 4 | 5 | V-1 | Hs3st3b1 | 9953 | 4-May-15 |
| 10161 | 2 | 3 | 4 | 5 | V-1 | Hsd17b6 | 8630 | 4-May-15 |
| 10199 | 2 | 3 | 4 | 5 | V-1 | Hspb3 | 8988 | 4-May-15 |
| 10200 | 2 | 3 | 4 | 5 | V-1 | Hspb6 | 126393 | 12-May-15 |
| 10201 | 2 | 3 | 4 | 5 | V-1 | Hspb7 | 27129 | 4-May-15 |
| 10203 | 2 | 3 | 4 | 5 | V-1 | Hspb9 | 94086 | 4-May-15 |
| 10263 | 2 | 3 | 4 | 5 | V-1 | Icam4 | 3386 | 12-May-15 |
| 10283 | 2 | 3 | 4 | 5 | V-1 | Ido1 | 3620 | 24-May-15 |
| 10373 | 2 | 3 | 4 | 5 | V-1 | Igfbp1 | 3484 | 17-May-15 |
| 10386 | 2 | 3 | 4 | 5 | V-1 | Igj | 3512 | 12-May-15 |
| 10468 | 2 | 3 | 4 | 5 | V-1 | Il22ra1 | 58985 | 4-May-15 |
| 10497 | 2 | 3 | 4 | 5 | V-1 | Ildr1 | 286676 | 4-May-15 |
| 10498 | 2 | 3 | 4 | 5 | V-1 | Ildr2 | 387597 | 12-May-15 |
| 10528 | 2 | 3 | 4 | 5 | V-1 | Inha | 3623 | 12-May-15 |
| 10579 | 2 | 3 | 4 | 5 | V-1 | Ip6k3 | 117283 | 4-May-15 |
| 10665 | 2 | 3 | 4 | 5 | V-1 | Isyna1 | 51477 | 12-May-15 |
| 10687 | 2 | 3 | 4 | 5 | V-1 | Itgax | 3687 | 12-May-15 |
| 10690 | 2 | 3 | 4 | 5 | V-1 | Itgb1bp2 | 26548 | 4-May-15 |
| 10761 | 2 | 3 | 4 | 5 | V-1 | Jph2 | 57158 | 4-May-15 |
| 10767 | 2 | 3 | 4 | 5 | V-1 | Jsrp1 | 126306 | 4-May-15 |
| 10815 | 2 | 3 | 4 | 5 | V-1 | Kcna7 | 3743 | 4-May-15 |
| 10824 | 2 | 3 | 4 | 5 | V-1 | Kcnc4 | 3749 | 4-May-15 |
| 10841 | 2 | 3 | 4 | 5 | V-1 | Kcnh3 | 23416 | 4-May-15 |
| 10853 | 2 | 3 | 4 | 5 | V-1 | Kcnj11 | 3767 | 31-May-15 |
| 10854 | 2 | 3 | 4 | 5 | V-1 | Kcnj12 | 3768 | 4-May-15 |
| 10857 | 2 | 3 | 4 | 5 | V-1 | Kcnj15 | 3772 | 21-May-15 |
| 10866 | 2 | 3 | 4 | 5 | V-1 | Kcnk1 | 3775 | 4-May-15 |
| 10879 | 2 | 3 | 4 | 5 | V-1 | Kcnk9 | 51305 | 4-May-15 |
| 10906 | 2 | 3 | 4 | 5 | V-1 | Kctd1 | 284252 | 4-May-15 |
| 10956 | 2 | 3 | 4 | 5 | V-1 | Kel | 3792 | 12-May-15 |
| 11030 | 2 | 3 | 4 | 5 | V-1 | Klf1 | 10661 | 12-May-15 |
| 11077 | 2 | 3 | 4 | 5 | V-1 | Klhl30 | 377007 | 4-May-15 |
| 11078 | 2 | 3 | 4 | 5 | V-1 | Klhl31 | 401265 | 4-May-15 |
| 11081 | 2 | 3 | 4 | 5 | V-1 | Klhl34 | 257240 | 4-May-15 |
| 11086 | 2 | 3 | 4 | 5 | V-1 | Klhl40 | 131377 | 4-May-15 |
| 11087 | 2 | 3 | 4 | 5 | V-1 | Klhl41 | 10324 | 4-May-15 |
| 11095 | 2 | 3 | 4 | 5 | V-1 | Klk10 | 5655 | 21-May-15 |
| 11099 | 2 | 3 | 4 | 5 | V-1 | Klk14 | 43847 | 4-May-15 |
| 11107 | 2 | 3 | 4 | 5 | V-1 | Klk1b26 | | |
| 11144 | 2 | 3 | 4 | 5 | V-1 | Klrb1a | | |
| 11146 | 2 | 3 | 4 | 5 | V-1 | Klrb1c | | |
| 11167 | 2 | 3 | 4 | 5 | V-1 | Kng1 | 3827 | 4-May-15 |
| 11192 | 2 | 3 | 4 | 5 | V-1 | Krt13 | 3860 | 7-Jun-15 |
| 11193 | 2 | 3 | 4 | 5 | V-1 | Krt14 | 3861 | 23-May-15 |
| 11195 | 2 | 3 | 4 | 5 | V-1 | Krt16 | 3868 | 23-May-15 |
| 11197 | 2 | 3 | 4 | 5 | V-1 | Krt18 | 3875 | 24-May-15 |
| 11202 | 2 | 3 | 4 | 5 | V-1 | Krt23 | 25984 | 4-May-15 |
| 11216 | 2 | 3 | 4 | 5 | V-1 | Krt4 | 3851 | 4-May-15 |
| 11219 | 2 | 3 | 4 | 5 | V-1 | Krt5 | 3852 | 23-May-15 |
| 11220 | 2 | 3 | 4 | 5 | V-1 | Krt6a | 3853 | 28-May-15 |
| 11230 | 2 | 3 | 4 | 5 | V-1 | Krt78 | 196374 | 4-May-15 |
| 11295 | 2 | 3 | 4 | 5 | V-1 | Krtdap | 388533 | 4-May-15 |
| 11302 | 2 | 3 | 4 | 5 | V-1 | Kynu | 8942 | 4-May-15 |
| 11317 | 2 | 3 | 4 | 5 | V-1 | Lad1 | 3898 | 7-Jun-15 |
| 11370 | 2 | 3 | 4 | 5 | V-1 | Lbx1 | 10660 | 4-May-15 |
| 11389 | 2 | 3 | 4 | 5 | V-1 | Lce3a | 353142 | 4-May-15 |
| 11390 | 2 | 3 | 4 | 5 | V-1 | Lce3b | 353143 | 4-May-15 |
| 11391 | 2 | 3 | 4 | 5 | V-1 | Lce3c | 353144 | 4-May-15 |
| 11393 | 2 | 3 | 4 | 5 | V-1 | Lce3e | 353145 | 4-May-15 |
| 11394 | 2 | 3 | 4 | 5 | V-1 | Lce3f | | |
| 11400 | 2 | 3 | 4 | 5 | V-1 | Lcn10 | 414332 | 4-May-15 |
| 11402 | 2 | 3 | 4 | 5 | V-1 | Lcn12 | 286256 | 4-May-15 |
| 11403 | 2 | 3 | 4 | 5 | V-1 | Lcn2 | 3934 | 17-May-15 |
| 11406 | 2 | 3 | 4 | 5 | V-1 | Lcn5 | 353176 | |
| 11407 | 2 | 3 | 4 | 5 | V-1 | Lcn6 | 158062 | 4-May-15 |
| 11408 | 2 | 3 | 4 | 5 | V-1 | Lcn8 | 138307 | 12-May-15 |
| 11409 | 2 | 3 | 4 | 5 | V-1 | Lcn9 | 392399 | 4-May-15 |
| 11418 | 2 | 3 | 4 | 5 | V-1 | Ldb3 | 11155 | 23-May-15 |
| 11435 | 2 | 3 | 4 | 5 | V-1 | Lef1 | 51176 | 12-May-15 |
| 11439 | 2 | 3 | 4 | 5 | V-1 | Lelp1 | 149018 | 4-May-15 |
| 11467 | 2 | 3 | 4 | 5 | V-1 | Lgals7 | 3963, 653499 | 7-Jun-15 |
| 11527 | 2 | 3 | 4 | 5 | V-1 | Lingo1 | 84894 | 12-May-15 |
| 11536 | 2 | 3 | 4 | 5 | V-1 | Lipg | 9388 | 12-May-15 |
| 11538 | 2 | 3 | 4 | 5 | V-1 | Lipi | 149998 | 4-May-15 |
| 11573 | 2 | 3 | 4 | 5 | V-1 | Lmod2 | 442721 | 12-May-15 |
| 11574 | 2 | 3 | 4 | 5 | V-1 | Lmod3 | 56203 | 12-May-15 |
| 11586 | 2 | 3 | 4 | 5 | V-1 | LOC100048884 | | |
| 11629 | 2 | 3 | 4 | 5 | V-1 | Lor | 4014 | 4-May-15 |
| 11664 | 2 | 3 | 4 | 5 | V-1 | Lrcol1 | 100507 055 | 4-May-15 |
| 11716 | 2 | 3 | 4 | 5 | V-1 | Lrrc30 | 339291 | 4-May-15 |
| 11833 | 2 | 3 | 4 | 5 | V-1 | Ly6g5b | 58496 | 21-May-15 |
| 11834 | 2 | 3 | 4 | 5 | V-1 | Ly6g5c | 80741 | 4-May-15 |
| 11859 | 2 | 3 | 4 | 5 | V-1 | Lypd8 | 646627 | 4-May-15 |
| 11877 | 2 | 3 | 4 | 5 | V-1 | Lyz2 | | |
| 11908 | 2 | 3 | 4 | 5 | V-1 | Mafa | 389692 | 7-Jun-15 |
| 11945 | 2 | 3 | 4 | 5 | V-1 | Mal | 4118 | 7-Jun-15 |
| 12049 | 2 | 3 | 4 | 5 | V-1 | Marc1 | 64757 | 12-May-15 |
| 12084 | 2 | 3 | 4 | 5 | V-1 | Mat1a | 4143 | 12-May-15 |
| 12097 | 2 | 3 | 4 | 5 | V-1 | Mb | 4151 | 12-May-15 |
| 12115 | 2 | 3 | 4 | 5 | V-1 | Mbnl3 | 55796 | 4-May-15 |
| 12116 | 2 | 3 | 4 | 5 | V-1 | Mboat1 | 154141 | 4-May-15 |
| 12180 | 2 | 3 | 4 | 5 | V-1 | Mdk | 4192 | 7-Jun-15 |
| 12256 | 2 | 3 | 4 | 5 | V-1 | Mesp1 | 55897 | 28-May-15 |
| 12257 | 2 | 3 | 4 | 5 | V-1 | Mesp2 | 145873 | 23-May-15 |
| 12312 | 2 | 3 | 4 | 5 | V-1 | Mfrp | 83552 | 4-May-15 |
| 12317 | 2 | 3 | 4 | 5 | V-1 | Mfsd2a | 84879 | 4-May-15 |
| 12432 | 2 | 3 | 4 | 5 | V-1 | Mir1291 | 100302 221 | 21-May-15 |
| 12443 | 2 | 3 | 4 | 5 | V-1 | Mir133a-2 | | |
| 12458 | 2 | 3 | 4 | 5 | V-1 | Mir142b | | |
| 12479 | 2 | 3 | 4 | 5 | V-1 | Mir1668 | | |

Fig.22 - 5

| | | | | | | | | |
|---|---|---|---|---|---|---|---|---|
| 12553 | 2 | 3 | 4 | 5 | V-1 | Mir1957b | | |
| 12604 | 2 | 3 | 4 | 5 | V-1 | Mir215 | 406997 | 21-May-15 |
| 12972 | 2 | 3 | 4 | 5 | V-1 | Mir6340 | | |
| 12986 | 2 | 3 | 4 | 5 | V-1 | Mir6357 | | |
| 12992 | 2 | 3 | 4 | 5 | V-1 | Mir6363 | | |
| 13011 | 2 | 3 | 4 | 5 | V-1 | Mir6385 | | |
| 13018 | 2 | 3 | 4 | 5 | V-1 | Mir6392 | | |
| 13022 | 2 | 3 | 4 | 5 | V-1 | Mir6396 | | |
| 13029 | 2 | 3 | 4 | 5 | V-1 | Mir6403 | | |
| 13044 | 2 | 3 | 4 | 5 | V-1 | Mir6418 | | |
| 13092 | 2 | 3 | 4 | 5 | V-1 | Mir682 | | |
| 13093 | 2 | 3 | 4 | 5 | V-1 | Mir683-1 | | |
| 13094 | 2 | 3 | 4 | 5 | V-1 | Mir683-2 | | |
| 13130 | 2 | 3 | 4 | 5 | V-1 | Mir692-2b | | |
| 13180 | 2 | 3 | 4 | 5 | V-1 | Mir697 | | |
| 13205 | 2 | 3 | 4 | 5 | V-1 | Mir6992 | | |
| 13246 | 2 | 3 | 4 | 5 | V-1 | Mir703 | | |
| 13320 | 2 | 3 | 4 | 5 | V-1 | Mir7-1 | 407043 | 24-May-15 |
| 13330 | 2 | 3 | 4 | 5 | V-1 | Mir719 | | |
| 13375 | 2 | 3 | 4 | 5 | V-1 | Mir760 | 100126 348 | 21-May-15 |
| 13423 | 2 | 3 | 4 | 5 | V-1 | Mir8093 | | |
| 13424 | 2 | 3 | 4 | 5 | V-1 | Mir8094 | | |
| 13427 | 2 | 3 | 4 | 5 | V-1 | Mir8097 | | |
| 13428 | 2 | 3 | 4 | 5 | V-1 | Mir8098 | | |
| 13429 | 2 | 3 | 4 | 5 | V-1 | Mir8099-1 | | |
| 13433 | 2 | 3 | 4 | 5 | V-1 | Mir8103 | | |
| 13434 | 2 | 3 | 4 | 5 | V-1 | Mir8104 | | |
| 13438 | 2 | 3 | 4 | 5 | V-1 | Mir8108 | | |
| 13442 | 2 | 3 | 4 | 5 | V-1 | Mir8112 | | |
| 13448 | 2 | 3 | 4 | 5 | V-1 | Mir8119 | | |
| 13483 | 2 | 3 | 4 | 5 | V-1 | Mirlet7d | 406886 | 21-May-15 |
| 13514 | 2 | 3 | 4 | 5 | V-1 | Mlf1 | 4291 | 12-May-15 |
| 13544 | 2 | 3 | 4 | 5 | V-1 | Mmp12 | 4321 | 4-May-15 |
| 13562 | 2 | 3 | 4 | 5 | V-1 | Mmp7 | 4316 | 17-May-15 |
| 13823 | 2 | 3 | 4 | 5 | V-1 | Mss51 | 118490 | 4-May-15 |
| 13826 | 2 | 3 | 4 | 5 | V-1 | Mstn | 2660 | 23-May-15 |
| 13832 | 2 | 3 | 4 | 5 | V-1 | Mt1 | 4489, 4494, 4495 | 7-Jun-15 |
| 13833 | 2 | 3 | 4 | 5 | V-1 | Mt2 | 4502 | 7-Jun-15 |
| 13834 | 2 | 3 | 4 | 5 | V-1 | Mt3 | 4504 | 24-May-15 |
| 13835 | 2 | 3 | 4 | 5 | V-1 | Mt4 | 84560 | 4-May-15 |
| 13905 | 2 | 3 | 4 | 5 | V-1 | Muc15 | 143662 | 4-May-15 |
| 13911 | 2 | 3 | 4 | 5 | V-1 | Muc5b | 727897 | 23-May-15 |
| 13933 | 2 | 3 | 4 | 5 | V-1 | Mup3 | | |
| 13935 | 2 | 3 | 4 | 5 | V-1 | Mup5 | | |
| 13940 | 2 | 3 | 4 | 5 | V-1 | Murc | 347273 | 4-May-15 |
| 13960 | 2 | 3 | 4 | 5 | V-1 | Myadml2 | 255275 | 4-May-15 |
| 13965 | 2 | 3 | 4 | 5 | V-1 | Mybpc1 | 4604 | 4-May-15 |
| 13966 | 2 | 3 | 4 | 5 | V-1 | Mybpc2 | 4606 | 4-May-15 |
| 13982 | 2 | 3 | 4 | 5 | V-1 | Myf6 | 4618 | 28-May-15 |
| 13984 | 2 | 3 | 4 | 5 | V-1 | Myh1 | 4619 | 4-May-15 |
| 13990 | 2 | 3 | 4 | 5 | V-1 | Myh2 | 4620 | 4-May-15 |
| 13992 | 2 | 3 | 4 | 5 | V-1 | Myh4 | 4622 | 4-May-15 |
| 13994 | 2 | 3 | 4 | 5 | V-1 | Myh7 | 4625 | 23-May-15 |
| 13996 | 2 | 3 | 4 | 5 | V-1 | Myh8 | 4626 | 12-May-15 |
| 13998 | 2 | 3 | 4 | 5 | V-1 | Myl1 | 4632 | 4-May-15 |
| 14003 | 2 | 3 | 4 | 5 | V-1 | Myl3 | 4634 | 23-May-15 |
| 14006 | 2 | 3 | 4 | 5 | V-1 | Myl6b | 140465 | 4-May-15 |
| 14011 | 2 | 3 | 4 | 5 | V-1 | Mylk2 | 85366 | 23-May-15 |
| 14013 | 2 | 3 | 4 | 5 | V-1 | Mylk4 | 340156 | 4-May-15 |
| 14014 | 2 | 3 | 4 | 5 | V-1 | Mylpf | 29895 | 4-May-15 |
| 14020 | 2 | 3 | 4 | 5 | V-1 | Myo18b | 84700 | 4-May-15 |
| 14031 | 2 | 3 | 4 | 5 | V-1 | Myo3b | 140469 | 4-May-15 |
| 14033 | 2 | 3 | 4 | 5 | V-1 | Myo5b | 4645 | 4-May-15 |
| 14045 | 2 | 3 | 4 | 5 | V-1 | Myom1 | 8736 | 12-May-15 |
| 14046 | 2 | 3 | 4 | 5 | V-1 | Myom2 | 9172 | 4-May-15 |
| 14048 | 2 | 3 | 4 | 5 | V-1 | Myot | 9499 | 23-May-15 |
| 14049 | 2 | 3 | 4 | 5 | V-1 | Myoz1 | 58529 | 12-May-15 |
| 14050 | 2 | 3 | 4 | 5 | V-1 | Myoz2 | 51778 | 12-May-15 |
| 14052 | 2 | 3 | 4 | 5 | V-1 | Mypn | 84665 | 12-May-15 |
| 14111 | 2 | 3 | 4 | 5 | V-1 | Naip7 | | |
| 14147 | 2 | 3 | 4 | 5 | V-1 | Nav2 | 89797 | 4-May-15 |
| 14183 | 2 | 3 | 4 | 5 | V-1 | Ncmap | 400746 | 4-May-15 |
| 14196 | 2 | 3 | 4 | 5 | V-1 | Nctc1 | | |
| 14262 | 2 | 3 | 4 | 5 | V-1 | Neb | 4703 | 23-May-15 |
| 14296 | 2 | 3 | 4 | 5 | V-1 | Nell1 | 4745 | 12-May-15 |
| 14324 | 2 | 3 | 4 | 5 | V-1 | Nexn | 91624 | 23-May-15 |
| 14338 | 2 | 3 | 4 | 5 | V-1 | Nfe2l3 | 9603 | 4-May-15 |
| 14364 | 2 | 3 | 4 | 5 | V-1 | Ngfr | 4804 | 17-May-15 |
| 14379 | 2 | 3 | 4 | 5 | V-1 | Nhsl1 | 57224 | 4-May-15 |
| 14524 | 2 | 3 | 4 | 5 | V-1 | Nos2 | 4843 | 7-Jun-15 |
| 14586 | 2 | 3 | 4 | 5 | V-1 | Nptxr | 23467 | 4-May-15 |
| 14617 | 2 | 3 | 4 | 5 | V-1 | Nr4a1 | 3164 | 3-May-15 |
| 14624 | 2 | 3 | 4 | 5 | V-1 | Nrap | 4892 | 7-Jun-15 |
| 14680 | 2 | 3 | 4 | 5 | V-1 | Nt5c1a | 84618 | 4-May-15 |
| 14708 | 2 | 3 | 4 | 5 | V-1 | Ntsr2 | 23620 | 4-May-15 |
| 14776 | 2 | 3 | 4 | 5 | V-1 | Nupr1l | 389493 | 4-May-15 |
| 14787 | 2 | 3 | 4 | 5 | V-1 | Nxf3 | 56000 | 12-May-15 |
| 14792 | 2 | 3 | 4 | 5 | V-1 | Nxpe2 | 120406 | 4-May-15 |
| 14799 | 2 | 3 | 4 | 5 | V-1 | Nxph4 | 11247 | 4-May-15 |
| 14823 | 2 | 3 | 4 | 5 | V-1 | Oaz1-ps | | |
| 14825 | 2 | 3 | 4 | 5 | V-1 | Oaz3 | 51686 | 12-May-15 |
| 14841 | 2 | 3 | 4 | 5 | V-1 | Ociad2 | 132299 | 4-May-15 |
| 14848 | 2 | 3 | 4 | 5 | V-1 | Odf1 | 4956 | 4-May-15 |
| 14854 | 2 | 3 | 4 | 5 | V-1 | Odf3l2 | 284451 | 4-May-15 |
| 16005 | 2 | 3 | 4 | 5 | V-1 | Omp | 4975 | 4-May-15 |
| 16065 | 2 | 3 | 4 | 5 | V-1 | Oscar | 126014 | 17-May-15 |
| 16110 | 2 | 3 | 4 | 5 | V-1 | Ovch2 | 341277 | 4-May-15 |
| 16128 | 2 | 3 | 4 | 5 | V-1 | P2rx2 | 22953 | 21-May-15 |
| 16165 | 2 | 3 | 4 | 5 | V-1 | Padi2 | 11240 | 4-May-15 |
| 16256 | 2 | 3 | 4 | 5 | V-1 | Pate2 | 399967 | 4-May-15 |
| 16264 | 2 | 3 | 4 | 5 | V-1 | Pax2 | 5076 | 23-May-15 |
| 16271 | 2 | 3 | 4 | 5 | V-1 | Pax8 | 7849 | 24-May-15 |
| 16376 | 2 | 3 | 4 | 5 | V-1 | Pck1 | 5105 | 4-May-15 |
| 16393 | 2 | 3 | 4 | 5 | V-1 | Pcp4 | 5121 | 4-May-15 |
| 16394 | 2 | 3 | 4 | 5 | V-1 | Pcp4l1 | 654790 | 4-May-15 |
| 16404 | 2 | 3 | 4 | 5 | V-1 | Pcsk9 | 255738 | 23-May-15 |
| 16410 | 2 | 3 | 4 | 5 | V-1 | Pcyt1b | 9468 | 4-May-15 |
| 16477 | 2 | 3 | 4 | 5 | V-1 | Pdk4 | 5166 | 4-May-15 |
| 16480 | 2 | 3 | 4 | 5 | V-1 | Pdlim3 | 27295 | 4-May-15 |
| 16507 | 2 | 3 | 4 | 5 | V-1 | Pdzk1 | 5174 | 17-May-15 |
| 16508 | 2 | 3 | 4 | 5 | V-1 | Pdzk1ip1 | 10158 | 4-May-15 |
| 16582 | 2 | 3 | 4 | 5 | V-1 | Pgam2 | 5224 | 4-May-15 |
| 16589 | 2 | 3 | 4 | 5 | V-1 | Pgc | 5225 | 12-May-15 |
| 16594 | 2 | 3 | 4 | 5 | V-1 | Pgk2 | 5232 | 28-May-15 |
| 16756 | 2 | 3 | 4 | 5 | V-1 | Pip5k1b | 8395 | 12-May-15 |
| 16800 | 2 | 3 | 4 | 5 | V-1 | Pkia | 5569 | 4-May-15 |
| 16822 | 2 | 3 | 4 | 5 | V-1 | Pla2g1b | 5319 | 12-May-15 |
| 16835 | 2 | 3 | 4 | 5 | V-1 | Pla2g5 | 5322 | 4-May-15 |
| 16841 | 2 | 3 | 4 | 5 | V-1 | Plac8 | 51316 | 7-Jun-15 |
| 16881 | 2 | 3 | 4 | 5 | V-1 | Plek2 | 26499 | 4-May-15 |
| 16913 | 2 | 3 | 4 | 5 | V-1 | Plet1 | 349633 | 4-May-15 |
| 16920 | 2 | 3 | 4 | 5 | V-1 | Plin4 | 729359 | 4-May-15 |
| 16980 | 2 | 3 | 4 | 5 | V-1 | Pnlip | 5406 | 12-May-15 |
| 16981 | 2 | 3 | 4 | 5 | V-1 | Pnliprp1 | 5407 | 12-May-15 |
| 16989 | 2 | 3 | 4 | 5 | V-1 | Pnmt | 5409 | 7-Jun-15 |
| 17102 | 2 | 3 | 4 | 5 | V-1 | Pou3f3 | 5455 | 4-May-15 |
| 17103 | 2 | 3 | 4 | 5 | V-1 | Pou3f3os | | |
| 17122 | 2 | 3 | 4 | 5 | V-1 | Ppapdc3 | 84814 | 4-May-15 |
| 17158 | 2 | 3 | 4 | 5 | V-1 | Ppl | 5493 | 4-May-15 |
| 17185 | 2 | 3 | 4 | 5 | V-1 | Ppp1r14c | 81706 | 4-May-15 |
| 17199 | 2 | 3 | 4 | 5 | V-1 | Ppp1r27 | 116729 | 4-May-15 |
| 17207 | 2 | 3 | 4 | 5 | V-1 | Ppp1r3a | 5506 | 12-May-15 |
| 17279 | 2 | 3 | 4 | 5 | V-1 | Prb1 | 5542 | 7-Jun-15 |
| 17316 | 2 | 3 | 4 | 5 | V-1 | Prh1 | 5554 | 31-May-15 |
| 17347 | 2 | 3 | 4 | 5 | V-1 | Prkcq | 5588 | 3-May-15 |
| 17389 | 2 | 3 | 4 | 5 | V-1 | Prlr | 5618 | 7-Jun-15 |
| 17390 | 2 | 3 | 4 | 5 | V-1 | Prm1 | 5619 | 7-Jun-15 |
| 17391 | 2 | 3 | 4 | 5 | V-1 | Prm2 | 5620 | 12-May-15 |
| 17392 | 2 | 3 | 4 | 5 | V-1 | Prm3 | 58531 | 12-May-15 |
| 17414 | 2 | 3 | 4 | 5 | V-1 | Prol1 | 58503 | 4-May-15 |
| 17416 | 2 | 3 | 4 | 5 | V-1 | Prom2 | 150696 | 4-May-15 |
| 17426 | 2 | 3 | 4 | 5 | V-1 | Prp2 | | |
| 17440 | 2 | 3 | 4 | 5 | V-1 | Prph | 5630 | 7-Jun-15 |
| 17442 | 2 | 3 | 4 | 5 | V-1 | Prpmp5 | | |
| 17466 | 2 | 3 | 4 | 5 | V-1 | Prr33 | 102724 536 | 17-Mar-15 |
| 17486 | 2 | 3 | 4 | 5 | V-1 | Prss1 | 5644 | 7-Jun-15 |
| 17489 | 2 | 3 | 4 | 5 | V-1 | Prss2 | 5645 | 23-May-15 |
| 17496 | 2 | 3 | 4 | 5 | V-1 | Prss3 | 5646 | 4-May-15 |
| 17523 | 2 | 3 | 4 | 5 | V-1 | Prss8 | 5652 | 12-May-15 |
| 17636 | 2 | 3 | 4 | 5 | V-1 | Ptgds | 5730 | 12-May-15 |
| 17652 | 2 | 3 | 4 | 5 | V-1 | Ptgs2 | 5743 | 24-May-15 |
| 17654 | 2 | 3 | 4 | 5 | V-1 | Pth | 5741 | 7-Jun-15 |
| 17689 | 2 | 3 | 4 | 5 | V-1 | Ptpn5 | 84867 | 12-May-15 |
| 17737 | 2 | 3 | 4 | 5 | V-1 | Pvalb | 5816 | 4-May-15 |
| 17742 | 2 | 3 | 4 | 5 | V-1 | Pvrl4 | 81607 | 4-May-15 |
| 17801 | 2 | 3 | 4 | 5 | V-1 | Rab11fip1 | 80223 | 4-May-15 |
| 17804 | 2 | 3 | 4 | 5 | V-1 | Rab11fip4 | 84440 | 12-May-15 |
| 17821 | 2 | 3 | 4 | 5 | V-1 | Rab25 | 57111 | 4-May-15 |
| 17822 | 2 | 3 | 4 | 5 | V-1 | Rab26 | 25837 | 12-May-15 |
| 17829 | 2 | 3 | 4 | 5 | V-1 | Rab30 | 27314 | 4-May-15 |
| 17895 | 2 | 3 | 4 | 5 | V-1 | Rad51b | 5890 | 4-May-15 |
| 17928 | 2 | 3 | 4 | 5 | V-1 | Ramp1 | 10267 | 21-May-15 |
| 17930 | 2 | 3 | 4 | 5 | V-1 | Ramp3 | 10268 | 12-May-15 |
| 17970 | 2 | 3 | 4 | 5 | V-1 | Rasal1 | 8437 | 4-May-15 |
| 17973 | 2 | 3 | 4 | 5 | V-1 | Rasd1 | 51655 | 4-May-15 |
| 17979 | 2 | 3 | 4 | 5 | V-1 | Rasgrf1 | 5923 | 4-May-15 |
| 17986 | 2 | 3 | 4 | 5 | V-1 | Rasl10a | 10633 | 4-May-15 |
| 18019 | 2 | 3 | 4 | 5 | V-1 | Rbfox1 | 54715 | 12-May-15 |
| 18026 | 2 | 3 | 4 | 5 | V-1 | Rbm11 | 54033 | 4-May-15 |
| 18039 | 2 | 3 | 4 | 5 | V-1 | Rbm24 | 221662 | 4-May-15 |
| 18134 | 2 | 3 | 4 | 5 | V-1 | Reg1 | | |
| 18135 | 2 | 3 | 4 | 5 | V-1 | Reg2 | | |
| 18234 | 2 | 3 | 4 | 5 | V-1 | Rgs9 | 8787 | 7-Jun-15 |
| 18237 | 2 | 3 | 4 | 5 | V-1 | Rhag | 6005 | 12-May-15 |
| 18247 | 2 | 3 | 4 | 5 | V-1 | Rhcg | 51458 | 12-May-15 |
| 18248 | 2 | 3 | 4 | 5 | V-1 | Rhd | 6007 | 21-May-15 |
| 18300 | 2 | 3 | 4 | 5 | V-1 | Rhpn1 | 114822 | 4-May-15 |
| 18355 | 2 | 3 | 4 | 5 | V-1 | Rmrp | 6023 | 23-May-15 |
| 18357 | 2 | 3 | 4 | 5 | V-1 | Rn4.5s | | |
| 18359 | 2 | 3 | 4 | 5 | V-1 | Rnase1 | 6035 | 4-May-15 |
| 18360 | 2 | 3 | 4 | 5 | V-1 | Rnase10 | 338879 | 4-May-15 |
| 18361 | 2 | 3 | 4 | 5 | V-1 | Rnase11 | 122651 | 4-May-15 |
| 18362 | 2 | 3 | 4 | 5 | V-1 | Rnase12 | 493901 | 4-May-15 |
| 18363 | 2 | 3 | 4 | 5 | V-1 | Rnase13 | 440163 | 4-May-15 |
| 18364 | 2 | 3 | 4 | 5 | V-1 | Rnase2a | | |
| 18368 | 2 | 3 | 4 | 5 | V-1 | Rnase9 | 390443 | 4-May-15 |
| 18394 | 2 | 3 | 4 | 5 | V-1 | Rnf128 | 79589 | 4-May-15 |
| 18409 | 2 | 3 | 4 | 5 | V-1 | Rnf149 | 284996 | 4-May-15 |
| 18421 | 2 | 3 | 4 | 5 | V-1 | Rnf180 | 285671 | 4-May-15 |
| 18426 | 2 | 3 | 4 | 5 | V-1 | Rnf186 | 54546 | 4-May-15 |
| 18454 | 2 | 3 | 4 | 5 | V-1 | Rnf43 | 54894 | 4-May-15 |
| 18553 | 2 | 3 | 4 | 5 | V-1 | Rpl38 | 6169 | 12-May-15 |

Fig.22・6

| | | | | | | | | |
|---|---|---|---|---|---|---|---|---|
| 18556 | 2 | 3 | 4 | 5 | V-1 | Rpl3l | 6123 | 4-May-15 |
| 18579 | 2 | 3 | 4 | 5 | V-1 | Rpph1 | 85495 | 4-May-15 |
| 18586 | 2 | 3 | 4 | 5 | V-1 | Rprm | 56475 | 4-May-15 |
| 18636 | 2 | 3 | 4 | 5 | V-1 | Rptn | 126638 | 4-May-15 |
| 18706 | 2 | 3 | 4 | 5 | V-1 | Rtn2 | 6253 | 4-May-15 |
| 18728 | 2 | 3 | 4 | 5 | V-1 | Runx2 | 860 | 31-May-15 |
| 18748 | 2 | 3 | 4 | 5 | V-1 | Ryr1 | 6261 | 24-May-15 |
| 18763 | 2 | 3 | 4 | 5 | V-1 | S100a8 | 6279 | 3-May-15 |
| 18764 | 2 | 3 | 4 | 5 | V-1 | S100a9 | 6280 | 24-May-15 |
| 18766 | 2 | 3 | 4 | 5 | V-1 | S100g | 795 | 4-May-15 |
| 18774 | 2 | 3 | 4 | 5 | V-1 | Saa1 | 6288 | 17-May-15 |
| 18776 | 2 | 3 | 4 | 5 | V-1 | Saa3 | 6290 | 4-May-15 |
| 18840 | 2 | 3 | 4 | 5 | V-1 | Sbk2 | 646643 | 4-May-15 |
| 18889 | 2 | 3 | 4 | 5 | V-1 | Scgb1a1 | 7356 | 4-May-15 |
| 18909 | 2 | 3 | 4 | 5 | V-1 | Scgb2b27 | | |
| 18913 | 2 | 3 | 4 | 5 | V-1 | Scgb3a2 | 117156 | 12-May-15 |
| 18932 | 2 | 3 | 4 | 5 | V-1 | Scn4b | 6330 | 23-May-15 |
| 18960 | 2 | 3 | 4 | 5 | V-1 | Scx | 642658 | 4-May-15 |
| 19095 | 2 | 3 | 4 | 5 | V-1 | Serinc2 | 347735 | 4-May-15 |
| 19101 | 2 | 3 | 4 | 5 | V-1 | Serpina10 | 51156 | 12-May-15 |
| 19104 | 2 | 3 | 4 | 5 | V-1 | Serpina1a | | |
| 19105 | 2 | 3 | 4 | 5 | V-1 | Serpina1b | | |
| 19106 | 2 | 3 | 4 | 5 | V-1 | Serpina1c | | |
| 19107 | 2 | 3 | 4 | 5 | V-1 | Serpina1d | | |
| 19109 | 2 | 3 | 4 | 5 | V-1 | Serpina1f | | |
| 19118 | 2 | 3 | 4 | 5 | V-1 | Serpina3k | | |
| 19134 | 2 | 3 | 4 | 5 | V-1 | Serpinb3a | | |
| 19143 | 2 | 3 | 4 | 5 | V-1 | Serpinb6e | | |
| 19153 | 2 | 3 | 4 | 5 | V-1 | Serpinc1 | 462 | 17-May-15 |
| 19159 | 2 | 3 | 4 | 5 | V-1 | Serpinf2 | 5345 | 20-May-15 |
| 19225 | 2 | 3 | 4 | 5 | V-1 | Sgca | 6442 | 12-May-15 |
| 19229 | 2 | 3 | 4 | 5 | V-1 | Sgcg | 6445 | 23-May-15 |
| 19272 | 2 | 3 | 4 | 5 | V-1 | Sh3gl2 | 6456 | 12-May-15 |
| 19294 | 2 | 3 | 4 | 5 | V-1 | Shc4 | 399694 | 4-May-15 |
| 19445 | 2 | 3 | 4 | 5 | V-1 | Slc16a3 | 9123 | 4-May-15 |
| 19451 | 2 | 3 | 4 | 5 | V-1 | Slc16a9 | 220963 | 4-May-15 |
| 19460 | 2 | 3 | 4 | 5 | V-1 | Slc17a9 | 63910 | 23-May-15 |
| 19468 | 2 | 3 | 4 | 5 | V-1 | Slc1a1 | 6505 | 12-May-15 |
| 19492 | 2 | 3 | 4 | 5 | V-1 | Slc22a26 | | |
| 19493 | 2 | 3 | 4 | 5 | V-1 | Slc22a27 | | |
| 19552 | 2 | 3 | 4 | 5 | V-1 | Slc25a47 | 283600 | 4-May-15 |
| 19596 | 2 | 3 | 4 | 5 | V-1 | Slc30a10 | 55532 | 23-May-15 |
| 19610 | 2 | 3 | 4 | 5 | V-1 | Slc34a2 | 10568 | 17-May-15 |
| 19631 | 2 | 3 | 4 | 5 | V-1 | Slc35f2 | 54733 | 4-May-15 |
| 19652 | 2 | 3 | 4 | 5 | V-1 | Slc38a3 | 10991 | 4-May-15 |
| 19654 | 2 | 3 | 4 | 5 | V-1 | Slc38a5 | 92745 | 4-May-15 |
| 19697 | 2 | 3 | 4 | 5 | V-1 | Slc4a1 | 6521 | 14-May-15 |
| 19710 | 2 | 3 | 4 | 5 | V-1 | Slc51b | 123264 | 4-May-15 |
| 19749 | 2 | 3 | 4 | 5 | V-1 | Slc7a12 | | |
| 19756 | 2 | 3 | 4 | 5 | V-1 | Slc7a5 | 8140 | 4-May-15 |
| 19760 | 2 | 3 | 4 | 5 | V-1 | Slc7a8 | 23428 | 4-May-15 |
| 19781 | 2 | 3 | 4 | 5 | V-1 | Slco1a4 | | |
| 19820 | 2 | 3 | 4 | 5 | V-1 | Sln | 6588 | 12-May-15 |
| 19821 | 2 | 3 | 4 | 5 | V-1 | Slpi | 6590 | 4-May-15 |
| 19865 | 2 | 3 | 4 | 5 | V-1 | Smco1 | 255798 | 4-May-15 |
| 19869 | 2 | 3 | 4 | 5 | V-1 | Smcp | 4184 | 4-May-15 |
| 19919 | 2 | 3 | 4 | 5 | V-1 | Smpx | 23676 | 23-May-15 |
| 19921 | 2 | 3 | 4 | 5 | V-1 | Smr3a | 26952 | 4-May-15 |
| 19924 | 2 | 3 | 4 | 5 | V-1 | Smtnl1 | 219537 | 4-May-15 |
| 19930 | 2 | 3 | 4 | 5 | V-1 | Smyd1 | 150572 | 4-May-15 |
| 19970 | 2 | 3 | 4 | 5 | V-1 | Snora15 | 677803 | 4-May-15 |
| 19971 | 2 | 3 | 4 | 5 | V-1 | Snora16a | 692073 | 4-May-15 |
| 19972 | 2 | 3 | 4 | 5 | V-1 | Snora17 | 677804 | 4-May-15 |
| 19975 | 2 | 3 | 4 | 5 | V-1 | Snora21 | 619505 | 4-May-15 |
| 19979 | 2 | 3 | 4 | 5 | V-1 | Snora28 | 677811 | 4-May-15 |
| 19981 | 2 | 3 | 4 | 5 | V-1 | Snora3 | 619562 | 4-May-15 |
| 19983 | 2 | 3 | 4 | 5 | V-1 | Snora31 | 677814 | 4-May-15 |
| 19984 | 2 | 3 | 4 | 5 | V-1 | Snora33 | 594839 | 4-May-15 |
| 19985 | 2 | 3 | 4 | 5 | V-1 | Snora34 | 677815 | 4-May-15 |
| 19988 | 2 | 3 | 4 | 5 | V-1 | Snora41 | 619569 | 4-May-15 |
| 19989 | 2 | 3 | 4 | 5 | V-1 | Snora43 | 677824 | 4-May-15 |
| 19992 | 2 | 3 | 4 | 5 | V-1 | Snora52 | 619565 | 4-May-15 |
| 19996 | 2 | 3 | 4 | 5 | V-1 | Snora64 | 26784 | 4-May-15 |
| 19997 | 2 | 3 | 4 | 5 | V-1 | Snora65 | 26783 | 4-May-15 |
| 19998 | 2 | 3 | 4 | 5 | V-1 | Snora68 | 26780 | 4-May-15 |
| 19999 | 2 | 3 | 4 | 5 | V-1 | Snora69 | 26779 | 4-May-15 |
| 20000 | 2 | 3 | 4 | 5 | V-1 | Snora70 | 26778 | 4-May-15 |
| 20002 | 2 | 3 | 4 | 5 | V-1 | Snora75 | 654321 | 4-May-15 |
| 20004 | 2 | 3 | 4 | 5 | V-1 | Snora7a | 619563 | 4-May-15 |
| 20005 | 2 | 3 | 4 | 5 | V-1 | Snora81 | 677847 | 4-May-15 |
| 20020 | 2 | 3 | 4 | 5 | V-1 | Snord15a | 6079 | 4-May-15 |
| 20029 | 2 | 3 | 4 | 5 | V-1 | Snord22 | 9304 | 4-May-15 |
| 20108 | 2 | 3 | 4 | 5 | V-1 | Snurf | 8926 | 7-Jun-15 |
| 20170 | 2 | 3 | 4 | 5 | V-1 | Sost | 50964 | 24-May-15 |
| 20193 | 2 | 3 | 4 | 5 | V-1 | Sox6 | 55553 | 12-May-15 |
| 20218 | 2 | 3 | 4 | 5 | V-1 | Spag11a | 653423 | 4-May-15 |
| 20219 | 2 | 3 | 4 | 5 | V-1 | Spag11b | 10407 | 14-May-15 |
| 20245 | 2 | 3 | 4 | 5 | V-1 | Spata3 | 130560 | 4-May-15 |
| 20310 | 2 | 3 | 4 | 5 | V-1 | Spin2c | | |
| 20314 | 2 | 3 | 4 | 5 | V-1 | Spink10 | | |
| 20315 | 2 | 3 | 4 | 5 | V-1 | Spink11 | | |
| 20316 | 2 | 3 | 4 | 5 | V-1 | Spink12 | | |
| 20319 | 2 | 3 | 4 | 5 | V-1 | Spink2 | 6691 | 4-May-15 |
| 20320 | 2 | 3 | 4 | 5 | V-1 | Spink3 | 6690 | 23-May-15 |
| 20322 | 2 | 3 | 4 | 5 | V-1 | Spink5 | 11005 | 12-May-15 |
| 20325 | 2 | 3 | 4 | 5 | V-1 | Spink8 | 646424 | 4-May-15 |
| 20327 | 2 | 3 | 4 | 5 | V-1 | Spint1 | 6692 | 4-May-15 |
| 20328 | 2 | 3 | 4 | 5 | V-1 | Spint2 | 10653 | 21-May-15 |
| 20330 | 2 | 3 | 4 | 5 | V-1 | Spint4 | 391253 | 4-May-15 |
| 20331 | 2 | 3 | 4 | 5 | V-1 | Spint5 | | |
| 20347 | 2 | 3 | 4 | 5 | V-1 | Spp1 | 6696 | 7-Jun-15 |
| 20358 | 2 | 3 | 4 | 5 | V-1 | Sprr1a | 6698 | 12-May-15 |
| 20367 | 2 | 3 | 4 | 5 | V-1 | Sprr2h | | |
| 20371 | 2 | 3 | 4 | 5 | V-1 | Sprr3 | 6707 | 12-May-15 |
| 20386 | 2 | 3 | 4 | 5 | V-1 | Spta1 | 6708 | 12-May-15 |
| 20388 | 2 | 3 | 4 | 5 | V-1 | Sptb | 6710 | 21-May-15 |
| 20398 | 2 | 3 | 4 | 5 | V-1 | Spz1 | 84654 | 4-May-15 |
| 20408 | 2 | 3 | 4 | 5 | V-1 | Srd5a2 | 6716 | 4-May-15 |
| 20421 | 2 | 3 | 4 | 5 | V-1 | Srl | 6345 | 4-May-15 |
| 20503 | 2 | 3 | 4 | 5 | V-1 | St14 | 6768 | 23-May-15 |
| 20512 | 2 | 3 | 4 | 5 | V-1 | St6gal1 | 6480 | 12-May-15 |
| 20533 | 2 | 3 | 4 | 5 | V-1 | Stac3 | 246329 | 4-May-15 |
| 20565 | 2 | 3 | 4 | 5 | V-1 | Stc2 | 8614 | 4-May-15 |
| 20570 | 2 | 3 | 4 | 5 | V-1 | Stfa1 | | |
| 20572 | 2 | 3 | 4 | 5 | V-1 | Stfa2l1 | | |
| 20750 | 2 | 3 | 4 | 5 | V-1 | Sycn | 342898 | 4-May-15 |
| 20761 | 2 | 3 | 4 | 5 | V-1 | Syn2 | 6854 | 4-May-15 |
| 20781 | 2 | 3 | 4 | 5 | V-1 | Synm | 23336 | 4-May-15 |
| 20783 | 2 | 3 | 4 | 5 | V-1 | Synpo2 | 171024 | 4-May-15 |
| 20784 | 2 | 3 | 4 | 5 | V-1 | Synpo2l | 79933 | 4-May-15 |
| 20789 | 2 | 3 | 4 | 5 | V-1 | Sypl2 | 284612 | 4-May-15 |
| 20811 | 2 | 3 | 4 | 5 | V-1 | Sytl4 | 94121 | 4-May-15 |
| 20846 | 2 | 3 | 4 | 5 | V-1 | Tacstd2 | 4070 | 12-May-15 |
| 20976 | 2 | 3 | 4 | 5 | V-1 | Tbc1d8 | 11138 | 4-May-15 |
| 21001 | 2 | 3 | 4 | 5 | V-1 | Tbx1 | 6899 | 23-May-15 |
| 21003 | 2 | 3 | 4 | 5 | V-1 | Tbx15 | 6913 | 4-May-15 |
| 21020 | 2 | 3 | 4 | 5 | V-1 | Tcap | 8557 | 23-May-15 |
| 21023 | 2 | 3 | 4 | 5 | V-1 | Tcea3 | 6920 | 4-May-15 |
| 21025 | 2 | 3 | 4 | 5 | V-1 | Tceal3 | 85012 | 4-May-15 |
| 21047 | 2 | 3 | 4 | 5 | V-1 | Tcf7 | 6932 | 12-May-15 |
| 21067 | 2 | 3 | 4 | 5 | V-1 | Tcp11 | 6954 | 4-May-15 |
| 21113 | 2 | 3 | 4 | 5 | V-1 | Teddm1 | 127670 | 4-May-15 |
| 21175 | 2 | 3 | 4 | 5 | V-1 | Tfap2a | 7020 | 23-May-15 |
| 21176 | 2 | 3 | 4 | 5 | V-1 | Tfap2b | 7021 | 23-May-15 |
| 21190 | 2 | 3 | 4 | 5 | V-1 | Tff1 | 7031 | 12-May-15 |
| 21191 | 2 | 3 | 4 | 5 | V-1 | Tff2 | 7032 | 12-May-15 |
| 21215 | 2 | 3 | 4 | 5 | V-1 | Tgif2lx2 | | |
| 21218 | 2 | 3 | 4 | 5 | V-1 | Tgm3 | 7053 | 12-May-15 |
| 21224 | 2 | 3 | 4 | 5 | V-1 | Tgoln2 | 10618 | 12-May-15 |
| 21309 | 2 | 3 | 4 | 5 | V-1 | Timp1 | 7076 | 12-May-15 |
| 21414 | 2 | 3 | 4 | 5 | V-1 | Tmem102 | 284114 | 14-May-15 |
| 21450 | 2 | 3 | 4 | 5 | V-1 | Tmem139 | 135932 | 12-May-15 |
| 21461 | 2 | 3 | 4 | 5 | V-1 | Tmem150c | 441027 | 4-May-15 |
| 21497 | 2 | 3 | 4 | 5 | V-1 | Tmem182 | 130827 | 4-May-15 |
| 21530 | 2 | 3 | 4 | 5 | V-1 | Tmem212 | 389177 | 4-May-15 |
| 21548 | 2 | 3 | 4 | 5 | V-1 | Tmem233 | 387890 | 4-May-15 |
| 21565 | 2 | 3 | 4 | 5 | V-1 | Tmem252 | 169693 | 4-May-15 |
| 21569 | 2 | 3 | 4 | 5 | V-1 | Tmem254c | | |
| 21583 | 2 | 3 | 4 | 5 | V-1 | Tmem30b | 161291 | 4-May-15 |
| 21588 | 2 | 3 | 4 | 5 | V-1 | Tmem38a | 79041 | 4-May-15 |
| 21604 | 2 | 3 | 4 | 5 | V-1 | Tmem51 | 55092 | 4-May-15 |
| 21606 | 2 | 3 | 4 | 5 | V-1 | Tmem52 | 339456 | 4-May-15 |
| 21658 | 2 | 3 | 4 | 5 | V-1 | Tmod1 | 7111 | 12-May-15 |
| 21661 | 2 | 3 | 4 | 5 | V-1 | Tmod4 | 29765 | 4-May-15 |
| 21673 | 2 | 3 | 4 | 5 | V-1 | Tmprss15 | 5651 | 7-Jun-15 |
| 21752 | 2 | 3 | 4 | 5 | V-1 | Tnnc2 | 7125 | 12-May-15 |
| 21753 | 2 | 3 | 4 | 5 | V-1 | Tnni1 | 7135 | 12-May-15 |
| 21754 | 2 | 3 | 4 | 5 | V-1 | Tnni2 | 7136 | 4-May-15 |
| 21759 | 2 | 3 | 4 | 5 | V-1 | Tnnt3 | 7140 | 12-May-15 |
| 21760 | 2 | 3 | 4 | 5 | V-1 | Tnp1 | 7141 | 12-May-15 |
| 21761 | 2 | 3 | 4 | 5 | V-1 | Tnp2 | 7142 | 4-May-15 |
| 21814 | 2 | 3 | 4 | 5 | V-1 | Tox3 | 27324 | 4-May-15 |
| 21822 | 2 | 3 | 4 | 5 | V-1 | Tpd52l1 | 7164 | 4-May-15 |
| 21831 | 2 | 3 | 4 | 5 | V-1 | Tpm2 | 7169 | 2-Jun-15 |
| 21839 | 2 | 3 | 4 | 5 | V-1 | Tppp2 | 122664 | 21-May-15 |
| 21877 | 2 | 3 | 4 | 5 | V-1 | Trank1 | 9881 | 12-May-15 |
| 21898 | 2 | 3 | 4 | 5 | V-1 | Trdn | 10345 | 23-May-15 |
| 21901 | 2 | 3 | 4 | 5 | V-1 | Trem2 | 54209 | 23-May-15 |
| 21964 | 2 | 3 | 4 | 5 | V-1 | Trim54 | 57159 | 4-May-15 |
| 21973 | 2 | 3 | 4 | 5 | V-1 | Trim63 | 84676 | 4-May-15 |
| 21981 | 2 | 3 | 4 | 5 | V-1 | Trim72 | 493829 | 4-May-15 |
| 22015 | 2 | 3 | 4 | 5 | V-1 | Trnp1 | 388610 | 4-May-15 |
| 22058 | 2 | 3 | 4 | 5 | V-1 | Trpv6 | 55503 | 20-May-15 |
| 22063 | 2 | 3 | 4 | 5 | V-1 | Try4 | 5647 | |
| 22064 | 2 | 3 | 4 | 5 | V-1 | Try5 | 168330 | 4-May-15 |
| 22093 | 2 | 3 | 4 | 5 | V-1 | Tspan1 | 10103 | 4-May-15 |
| 22133 | 2 | 3 | 4 | 5 | V-1 | Tssk6 | 83983 | 4-May-15 |
| 22168 | 2 | 3 | 4 | 5 | V-1 | Ttc36 | 143941 | 4-May-15 |
| 22202 | 2 | 3 | 4 | 5 | V-1 | Ttn | 7273 | 23-May-15 |
| 22216 | 2 | 3 | 4 | 5 | V-1 | Tuba8 | 51807 | 4-May-15 |
| 22220 | 2 | 3 | 4 | 5 | V-1 | Tubb2a-ps2 | | |
| 22259 | 2 | 3 | 4 | 5 | V-1 | Txlnb | 167838 | 12-May-15 |
| 22411 | 2 | 3 | 4 | 5 | V-1 | Ucp1 | 7350 | 12-May-15 |
| 22426 | 2 | 3 | 4 | 5 | V-1 | Ugt1a1 | 54658 | 28-May-15 |
| 22427 | 2 | 3 | 4 | 5 | V-1 | Ugt1a10 | 54575 | 28-May-15 |
| 22428 | 2 | 3 | 4 | 5 | V-1 | Ugt1a2 | | |
| 22483 | 2 | 3 | 4 | 5 | V-1 | Uox | 391051 | 12-May-15 |
| 22532 | 2 | 3 | 4 | 5 | V-1 | Usp13 | 8975 | 29-May-15 |
| 22623 | 2 | 3 | 4 | 5 | V-1 | Vash2 | 79805 | 4-May-15 |
| 22654 | 2 | 3 | 4 | 5 | V-1 | Vgll2 | 245806 | 4-May-15 |
| 23103 | 2 | 3 | 4 | 5 | V-1 | Wbscr25 | | |
| 23185 | 2 | 3 | 4 | 5 | V-1 | Wfdc10 | | |
| 23186 | 2 | 3 | 4 | 5 | V-1 | Wfdc11 | 259239 | 4-May-15 |
| 23188 | 2 | 3 | 4 | 5 | V-1 | Wfdc13 | 164237 | 4-May-15 |

Fig.22 - 7

| | | | | | | | | |
|---|---|---|---|---|---|---|---|---|
| 23190 | 2 | 3 | 4 | 5 | V-1 | Wfdc15b | | |
| 23197 | 2 | 3 | 4 | 5 | V-1 | Wfdc6a | | |
| 23198 | 2 | 3 | 4 | 5 | V-1 | Wfdc6b | | |
| 23199 | 2 | 3 | 4 | 5 | V-1 | Wfdc8 | 90199 | 4-May-15 |
| 23200 | 2 | 3 | 4 | 5 | V-1 | Wfdc9 | 259240 | 4-May-15 |
| 23268 | 2 | 3 | 4 | 5 | V-1 | Xirp1 | 165904 | 17-May-15 |
| 23269 | 2 | 3 | 4 | 5 | V-1 | Xirp2 | 129446 | 3-May-15 |
| 23278 | 2 | 3 | 4 | 5 | V-1 | Xkrx | 402415 | 4-May-15 |
| 23338 | 2 | 3 | 4 | 5 | V-1 | Yipf7 | 285525 | 4-May-15 |
| 23346 | 2 | 3 | 4 | 5 | V-1 | Ypel4 | 219539 | 4-May-15 |
| 23718 | 2 | 3 | 4 | 5 | V-1 | Zfp648 | | |
| 23762 | 2 | 3 | 4 | 5 | V-1 | Zfp750 | 79755 | 28-May-15 |
| 10 | 2 | 3 | 4 | | IV-2 | 0610012G03Rik | | |
| 14 | 2 | 3 | 4 | | IV-2 | 0610037L13Rik | | |
| 16 | 2 | 3 | 4 | | IV-2 | 0610039K10Rik | | |
| 21 | 2 | 3 | 4 | | IV-2 | 1010001N08Rik | | |
| 23 | 2 | 3 | 4 | | IV-2 | 1110001J03Rik | | |
| 28 | 2 | 3 | 4 | | IV-2 | 1110007C09Rik | | |
| 36 | 2 | 3 | 4 | | IV-2 | 1110020A21Rik | | |
| 37 | 2 | 3 | 4 | | IV-2 | 1110025L11Rik | | |
| 43 | 2 | 3 | 4 | | IV-2 | 1110036E04Rik | | |
| 48 | 2 | 3 | 4 | | IV-2 | 1110051M20Rik | | |
| 56 | 2 | 3 | 4 | | IV-2 | 1190002N15Rik | | |
| 57 | 2 | 3 | 4 | | IV-2 | 1190003K10Rik | | |
| 59 | 2 | 3 | 4 | | IV-2 | 1190007I07Rik | | |
| 75 | 2 | 3 | 4 | | IV-2 | 1600002D24Rik | | |
| 100 | 2 | 3 | 4 | | IV-2 | 1700001K19Rik | | |
| 105 | 2 | 3 | 4 | | IV-2 | 1700001P01Rik | | |
| 109 | 2 | 3 | 4 | | IV-2 | 1700003E24Rik | | |
| 169 | 2 | 3 | 4 | | IV-2 | 1700016D06Rik | | |
| 188 | 2 | 3 | 4 | | IV-2 | 1700019A02Rik | | |
| 201 | 2 | 3 | 4 | | IV-2 | 1700020G17Rik | | |
| 204 | 2 | 3 | 4 | | IV-2 | 1700020M21Rik | | |
| 225 | 2 | 3 | 4 | | IV-2 | 1700024P04Rik | | |
| 245 | 2 | 3 | 4 | | IV-2 | 1700028D13Rik | | |
| 257 | 2 | 3 | 4 | | IV-2 | 1700029J03Rik | | |
| 267 | 2 | 3 | 4 | | IV-2 | 1700030K09Rik | | |
| 308 | 2 | 3 | 4 | | IV-2 | 1700048O20Rik | | |
| 324 | 2 | 3 | 4 | | IV-2 | 1700057G04Rik | | |
| 354 | 2 | 3 | 4 | | IV-2 | 1700072B07Rik | | |
| 365 | 2 | 3 | 4 | | IV-2 | 1700084F23Rik | | |
| 369 | 2 | 3 | 4 | | IV-2 | 1700086O06Rik | | |
| 377 | 2 | 3 | 4 | | IV-2 | 1700093K21Rik | | |
| 384 | 2 | 3 | 4 | | IV-2 | 1700096K18Rik | | |
| 386 | 2 | 3 | 4 | | IV-2 | 1700100L14Rik | | |
| 391 | 2 | 3 | 4 | | IV-2 | 1700102P08Rik | | |
| 392 | 2 | 3 | 4 | | IV-2 | 1700104L18Rik | | |
| 405 | 2 | 3 | 4 | | IV-2 | 1700111N16Rik | | |
| 413 | 2 | 3 | 4 | | IV-2 | 1700120E14Rik | | |
| 416 | 2 | 3 | 4 | | IV-2 | 1700121L16Rik | | |
| 427 | 2 | 3 | 4 | | IV-2 | 1700125G02Rik | | |
| 430 | 2 | 3 | 4 | | IV-2 | 1700125H20Rik | | |
| 438 | 2 | 3 | 4 | | IV-2 | 1810008I18Rik | | |
| 439 | 2 | 3 | 4 | | IV-2 | 1810009A15Rik | | |
| 447 | 2 | 3 | 4 | | IV-2 | 1810013L24Rik | | |
| 454 | 2 | 3 | 4 | | IV-2 | 1810024B03Rik | | |
| 459 | 2 | 3 | 4 | | IV-2 | 1810034E14Rik | | |
| 462 | 2 | 3 | 4 | | IV-2 | 1810043G02Rik | | |
| 470 | 2 | 3 | 4 | | IV-2 | 1810062O18Rik | | |
| 476 | 2 | 3 | 4 | | IV-2 | 2010005H15Rik | | |
| 479 | 2 | 3 | 4 | | IV-2 | 2010012O05Rik | | |
| 489 | 2 | 3 | 4 | | IV-2 | 2010111I01Rik | | |
| 497 | 2 | 3 | 4 | | IV-2 | 2200002J24Rik | | |
| 499 | 2 | 3 | 4 | | IV-2 | 2210011C24Rik | | |
| 500 | 2 | 3 | 4 | | IV-2 | 2210013O21Rik | | |
| 507 | 2 | 3 | 4 | | IV-2 | 2210404O09Rik | | |
| 510 | 2 | 3 | 4 | | IV-2 | 2210408I21Rik | | |
| 513 | 2 | 3 | 4 | | IV-2 | 2210414B05Rik | | |
| 515 | 2 | 3 | 4 | | IV-2 | 2210417A02Rik | | |
| 521 | 2 | 3 | 4 | | IV-2 | 2310001H17Rik | | |
| 523 | 2 | 3 | 4 | | IV-2 | 2310002D06Rik | | |
| 527 | 2 | 3 | 4 | | IV-2 | 2310003H01Rik | | |
| 537 | 2 | 3 | 4 | | IV-2 | 2310011J03Rik | | |
| 541 | 2 | 3 | 4 | | IV-2 | 2310015D24Rik | | |
| 552 | 2 | 3 | 4 | | IV-2 | 2310035C23Rik | | |
| 561 | 2 | 3 | 4 | | IV-2 | 2310047M10Rik | | |
| 564 | 2 | 3 | 4 | | IV-2 | 2310057M21Rik | | |
| 570 | 2 | 3 | 4 | | IV-2 | 2310067B10Rik | | |
| 573 | 2 | 3 | 4 | | IV-2 | 2310069G16Rik | | |
| 574 | 2 | 3 | 4 | | IV-2 | 2310079G19Rik | | |
| 576 | 2 | 3 | 4 | | IV-2 | 2410002F23Rik | | |
| 583 | 2 | 3 | 4 | | IV-2 | 2410007B07Rik | | |
| 592 | 2 | 3 | 4 | | IV-2 | 2410088K16Rik | | |
| 623 | 2 | 3 | 4 | | IV-2 | 2610037D02Rik | | |
| 632 | 2 | 3 | 4 | | IV-2 | 2610306M01Rik | | |
| 633 | 2 | 3 | 4 | | IV-2 | 2610307P16Rik | | |
| 641 | 2 | 3 | 4 | | IV-2 | 2700029M09Rik | | |
| 650 | 2 | 3 | 4 | | IV-2 | 2700070H01Rik | | |
| 653 | 2 | 3 | 4 | | IV-2 | 2700089E24Rik | | |
| 658 | 2 | 3 | 4 | | IV-2 | 2810001G20Rik | | |
| 669 | 2 | 3 | 4 | | IV-2 | 2810047C21Rik1 | | |
| 678 | 2 | 3 | 4 | | IV-2 | 2810408M09Rik | | |
| 681 | 2 | 3 | 4 | | IV-2 | 2810428I15Rik | | |
| 684 | 2 | 3 | 4 | | IV-2 | 2810442I21Rik | | |
| 694 | 2 | 3 | 4 | | IV-2 | 2900011O08Rik | | |
| 697 | 2 | 3 | 4 | | IV-2 | 2900052N01Rik | | |
| 711 | 2 | 3 | 4 | | IV-2 | 3100003L05Rik | | |
| 713 | 2 | 3 | 4 | | IV-2 | 3110002H16Rik | | |
| 716 | 2 | 3 | 4 | | IV-2 | 3110009F21Rik | | |
| 726 | 2 | 3 | 4 | | IV-2 | 3110052M02Rik | | |
| 731 | 2 | 3 | 4 | | IV-2 | 3110079O15Rik | | |
| 733 | 2 | 3 | 4 | | IV-2 | 3110082J24Rik | | |
| 739 | 2 | 3 | 4 | | IV-2 | 3632451O06Rik | | |
| 740 | 2 | 3 | 4 | | IV-2 | 3632454L22Rik | | |
| 746 | 2 | 3 | 4 | | IV-2 | 4430402I18Rik | | |
| 752 | 2 | 3 | 4 | | IV-2 | 4632434I11Rik | | |
| 814 | 2 | 3 | 4 | | IV-2 | 4930404I05Rik | | |
| 816 | 2 | 3 | 4 | | IV-2 | 4930405A10Rik | | |
| 843 | 2 | 3 | 4 | | IV-2 | 4930426L09Rik | | |
| 845 | 2 | 3 | 4 | | IV-2 | 4930428D18Rik | | |
| 853 | 2 | 3 | 4 | | IV-2 | 4930430A15Rik | | |
| 887 | 2 | 3 | 4 | | IV-2 | 4930448I06Rik | | |
| 905 | 2 | 3 | 4 | | IV-2 | 4930455D15Rik | | |
| 956 | 2 | 3 | 4 | | IV-2 | 4930502E09Rik | | |
| 977 | 2 | 3 | 4 | | IV-2 | 4930512B01Rik | | |
| 1019 | 2 | 3 | 4 | | IV-2 | 4930529M08Rik | | |
| 1027 | 2 | 3 | 4 | | IV-2 | 4930539M17Rik | | |
| 1074 | 2 | 3 | 4 | | IV-2 | 4930563M20Rik | | |
| 1076 | 2 | 3 | 4 | | IV-2 | 4930564C03Rik | | |
| 1078 | 2 | 3 | 4 | | IV-2 | 4930565D16Rik | | |
| 1081 | 2 | 3 | 4 | | IV-2 | 4930567H17Rik | | |
| 1096 | 2 | 3 | 4 | | IV-2 | 4930578E11Rik | | |
| 1099 | 2 | 3 | 4 | | IV-2 | 4930578N18Rik | | |
| 1124 | 2 | 3 | 4 | | IV-2 | 4931403G20Rik | | |
| 1131 | 2 | 3 | 4 | | IV-2 | 4931409K22Rik | | |
| 1168 | 2 | 3 | 4 | | IV-2 | 4933400A11Rik | | |
| 1287 | 2 | 3 | 4 | | IV-2 | 5031439G07Rik | | |
| 1296 | 2 | 3 | 4 | | IV-2 | 5330434G04Rik | | |
| 1304 | 2 | 3 | 4 | | IV-2 | 5430416O09Rik | | |
| 1309 | 2 | 3 | 4 | | IV-2 | 5430425K12Rik | | |
| 1323 | 2 | 3 | 4 | | IV-2 | 5730409E04Rik | | |
| 1328 | 2 | 3 | 4 | | IV-2 | 5730435O14Rik | | |
| 1336 | 2 | 3 | 4 | | IV-2 | 5730522E02Rik | | |
| 1345 | 2 | 3 | 4 | | IV-2 | 5830418P13Rik | | |
| 1354 | 2 | 3 | 4 | | IV-2 | 5930438M14Rik | | |
| 1372 | 2 | 3 | 4 | | IV-2 | 6330415B21Rik | | |
| 1375 | 2 | 3 | 4 | | IV-2 | 6330419J24Rik | | |
| 1376 | 2 | 3 | 4 | | IV-2 | 6330549D23Rik | | |
| 1378 | 2 | 3 | 4 | | IV-2 | 6430503K07Rik | | |
| 1389 | 2 | 3 | 4 | | IV-2 | 6530411M01Rik | | |
| 1413 | 2 | 3 | 4 | | IV-2 | 8430427H17Rik | | |
| 1414 | 2 | 3 | 4 | | IV-2 | 8430429K09Rik | | |
| 1424 | 2 | 3 | 4 | | IV-2 | 9030624G23Rik | | |
| 1428 | 2 | 3 | 4 | | IV-2 | 9130011E15Rik | | |
| 1434 | 2 | 3 | 4 | | IV-2 | 9130024F11Rik | | |
| 1436 | 2 | 3 | 4 | | IV-2 | 9130209A04Rik | | |
| 1463 | 2 | 3 | 4 | | IV-2 | 9330159M07Rik | | |
| 1499 | 2 | 3 | 4 | | IV-2 | 9530059O14Rik | | |
| 1503 | 2 | 3 | 4 | | IV-2 | 9530082P21Rik | | |
| 1520 | 2 | 3 | 4 | | IV-2 | 9930111J21Rik2 | | |
| 1522 | 2 | 3 | 4 | | IV-2 | A130010J15Rik | | |
| 1534 | 2 | 3 | 4 | | IV-2 | A230057D06Rik | | |
| 1545 | 2 | 3 | 4 | | IV-2 | A330021E22Rik | | |
| 1572 | 2 | 3 | 4 | | IV-2 | A430107P09Rik | | |
| 1575 | 2 | 3 | 4 | | IV-2 | A530006G24Rik | | |
| 1578 | 2 | 3 | 4 | | IV-2 | A530032D15Rik | | |
| 1582 | 2 | 3 | 4 | | IV-2 | A530054K11Rik | | |
| 1590 | 2 | 3 | 4 | | IV-2 | A630007B06Rik | | |
| 1591 | 2 | 3 | 4 | | IV-2 | A630010A05Rik | | |
| 1603 | 2 | 3 | 4 | | IV-2 | A630077J23Rik | | |
| 1605 | 2 | 3 | 4 | | IV-2 | A630095E13Rik | | |
| 1610 | 2 | 3 | 4 | | IV-2 | A730017L22Rik | | |
| 1621 | 2 | 3 | 4 | | IV-2 | A730090N16Rik | | |
| 1633 | 2 | 3 | 4 | | IV-2 | A930003A15Rik | | |
| 1639 | 2 | 3 | 4 | | IV-2 | A930007I19Rik | | |
| 1647 | 2 | 3 | 4 | | IV-2 | A930017M01Rik | | |
| 1660 | 2 | 3 | 4 | | IV-2 | AA536875 | | |
| 1671 | 2 | 3 | 4 | | IV-2 | Aadacl2 | 344752 | 4-May-15 |
| 1673 | 2 | 3 | 4 | | IV-2 | Aadat | 51166 | 4-May-15 |
| 1674 | 2 | 3 | 4 | | IV-2 | Aaed1 | 195827 | 4-May-15 |
| 1692 | 2 | 3 | 4 | | IV-2 | Abat | 18 | 4-May-15 |
| 1693 | 2 | 3 | 4 | | IV-2 | Abca1 | 19 | 24-May-15 |
| 1708 | 2 | 3 | 4 | | IV-2 | Abca9 | 10350 | 12-May-15 |
| 1747 | 2 | 3 | 4 | | IV-2 | Abhd12 | 26090 | 4-May-15 |
| 1759 | 2 | 3 | 4 | | IV-2 | Abhd3 | 171586 | 21-May-15 |
| 1760 | 2 | 3 | 4 | | IV-2 | Abhd4 | 63874 | 4-May-15 |
| 1768 | 2 | 3 | 4 | | IV-2 | Abl1 | 25 | 24-May-15 |
| 1776 | 2 | 3 | 4 | | IV-2 | Abracl | 58527 | 12-May-15 |
| 1797 | 2 | 3 | 4 | | IV-2 | Acap2 | 23527 | 4-May-15 |
| 1811 | 2 | 3 | 4 | | IV-2 | Ace2 | 59272 | 17-May-15 |
| 1812 | 2 | 3 | 4 | | IV-2 | Ace3 | 100129 123 | 12-May-15 |
| 1817 | 2 | 3 | 4 | | IV-2 | Acin1 | 22985 | 2-Jun-15 |
| 1819 | 2 | 3 | 4 | | IV-2 | Ackr2 | 1238 | 4-May-15 |
| 1821 | 2 | 3 | 4 | | IV-2 | Ackr4 | 51554 | 4-May-15 |
| 1823 | 2 | 3 | 4 | | IV-2 | Acmsd | 130013 | 12-May-15 |
| 1831 | 2 | 3 | 4 | | IV-2 | Acot11 | 26027 | 4-May-15 |
| 1846 | 2 | 3 | 4 | | IV-2 | Acp1 | 52 | 12-May-15 |
| 1849 | 2 | 3 | 4 | | IV-2 | Acp6 | 51205 | 4-May-15 |
| 1851 | 2 | 3 | 4 | | IV-2 | Acpt | 93650 | 4-May-15 |
| 1863 | 2 | 3 | 4 | | IV-2 | Acsl6 | 23305 | 4-May-15 |
| 1865 | 2 | 3 | 4 | | IV-2 | Acsm2 | 123876 | 19-May-15 |
| 1867 | 2 | 3 | 4 | | IV-2 | Acsm4 | 341392 | 4-May-15 |
| 1870 | 2 | 3 | 4 | | IV-2 | Acss2 | 55902 | 4-May-15 |
| 1871 | 2 | 3 | 4 | | IV-2 | Acss2os | | |

Fig.22 - 8

| | | | | | | | | |
|---|---|---|---|---|---|---|---|---|
| 1875 | 2 | 3 | 4 | | IV-2 | Actb | 60 | 7-Jun-15 |
| 1878 | 2 | 3 | 4 | | IV-2 | Actg1 | 71 | 23-May-15 |
| 1880 | 2 | 3 | 4 | | IV-2 | Actl10 | 170487 | 4-May-15 |
| 1890 | 2 | 3 | 4 | | IV-2 | Actn4 | 81 | 29-May-15 |
| 1904 | 2 | 3 | 4 | | IV-2 | Acvr1b | 91 | 4-May-15 |
| 1911 | 2 | 3 | 4 | | IV-2 | Acyp1 | 97 | 12-May-15 |
| 1919 | 2 | 3 | 4 | | IV-2 | Adam12 | 8038 | 4-May-15 |
| 1920 | 2 | 3 | 4 | | IV-2 | Adam15 | 8751 | 4-May-15 |
| 1931 | 2 | 3 | 4 | | IV-2 | Adam24 | 646479 | 12-May-15 |
| 1949 | 2 | 3 | 4 | | IV-2 | Adam9 | 8754 | 17-May-15 |
| 1954 | 2 | 3 | 4 | | IV-2 | Adamts13 | 11093 | 31-May-15 |
| 1962 | 2 | 3 | 4 | | IV-2 | Adamts20 | 80070 | 12-May-15 |
| 1965 | 2 | 3 | 4 | | IV-2 | Adamts5 | 11096 | 4-May-15 |
| 1966 | 2 | 3 | 4 | | IV-2 | Adamts6 | 11174 | 4-May-15 |
| 1969 | 2 | 3 | 4 | | IV-2 | Adamts9 | 56999 | 4-May-15 |
| 1970 | 2 | 3 | 4 | | IV-2 | Adamtsl1 | 92949 | 4-May-15 |
| 1976 | 2 | 3 | 4 | | IV-2 | Adap2 | 55803 | 4-May-15 |
| 1977 | 2 | 3 | 4 | | IV-2 | Adar | 103 | 12-May-15 |
| 1983 | 2 | 3 | 4 | | IV-2 | Adc | | |
| 1990 | 2 | 3 | 4 | | IV-2 | Adcy10 | 55811 | 4-May-15 |
| 1995 | 2 | 3 | 4 | | IV-2 | Adcy6 | 112 | 23-May-15 |
| 2003 | 2 | 3 | 4 | | IV-2 | Add3 | 120 | 12-May-15 |
| 2008 | 2 | 3 | 4 | | IV-2 | Adh6a | | |
| 2015 | 2 | 3 | 4 | | IV-2 | Adipor1 | 51094 | 31-May-15 |
| 2020 | 2 | 3 | 4 | | IV-2 | Adnp | 23394 | 4-May-15 |
| 2030 | 2 | 3 | 4 | | IV-2 | Adprhl2 | 54936 | 4-May-15 |
| 2033 | 2 | 3 | 4 | | IV-2 | Adra1b | 147 | 14-May-15 |
| 2041 | 2 | 3 | 4 | | IV-2 | Adrbk1 | 156 | 12-May-15 |
| 2052 | 2 | 3 | 4 | | IV-2 | AF067061 | | |
| 2055 | 2 | 3 | 4 | | IV-2 | AF357355 | | |
| 2072 | 2 | 3 | 4 | | IV-2 | Afmid | 125061 | 4-May-15 |
| 2076 | 2 | 3 | 4 | | IV-2 | Agap1 | 116987 | 12-May-15 |
| 2094 | 2 | 3 | 4 | | IV-2 | Ago3 | 192669 | 4-May-15 |
| 2098 | 2 | 3 | 4 | | IV-2 | Agpat3 | 56894 | 4-May-15 |
| 2100 | 2 | 3 | 4 | | IV-2 | Agpat5 | 55326 | 4-May-15 |
| 2105 | 2 | 3 | 4 | | IV-2 | Agr3 | 155465 | 4-May-15 |
| 2113 | 2 | 3 | 4 | | IV-2 | Agtrap | 57085 | 4-May-15 |
| 2115 | 2 | 3 | 4 | | IV-2 | Agxt2 | 64902 | 12-May-15 |
| 2135 | 2 | 3 | 4 | | IV-2 | AI413582 | | |
| 2136 | 2 | 3 | 4 | | IV-2 | AI414108 | | |
| 2145 | 2 | 3 | 4 | | IV-2 | AI506816 | | |
| 2147 | 2 | 3 | 4 | | IV-2 | AI593442 | | |
| 2154 | 2 | 3 | 4 | | IV-2 | AI747448 | | |
| 2155 | 2 | 3 | 4 | | IV-2 | AI837181 | | |
| 2160 | 2 | 3 | 4 | | IV-2 | AI854517 | | |
| 2164 | 2 | 3 | 4 | | IV-2 | Aida | 64853 | 7-Jun-15 |
| 2166 | 2 | 3 | 4 | | IV-2 | Aif1l | 83543 | 4-May-15 |
| 2170 | 2 | 3 | 4 | | IV-2 | Aig1 | 51390 | 12-May-15 |
| 2171 | 2 | 3 | 4 | | IV-2 | Aim1 | 202 | 7-Jun-15 |
| 2174 | 2 | 3 | 4 | | IV-2 | Aimp1 | 9255 | 17-May-15 |
| 2180 | 2 | 3 | 4 | | IV-2 | Ajap1 | 55966 | 4-May-15 |
| 2185 | 2 | 3 | 4 | | IV-2 | Ak2 | 204 | 4-May-15 |
| 2229 | 2 | 3 | 4 | | IV-2 | Akr1e1 | | |
| 2231 | 2 | 3 | 4 | | IV-2 | Akt1 | 207 | 31-May-15 |
| 2240 | 2 | 3 | 4 | | IV-2 | Alcam | 214 | 4-May-15 |
| 2247 | 2 | 3 | 4 | | IV-2 | Aldh1b1 | 219 | 23-May-15 |
| 2250 | 2 | 3 | 4 | | IV-2 | Aldh2 | 217 | 24-May-15 |
| 2268 | 2 | 3 | 4 | | IV-2 | Alg11 | 440138 | 23-May-15 |
| 2289 | 2 | 3 | 4 | | IV-2 | Alms1-ps2 | | |
| 2290 | 2 | 3 | 4 | | IV-2 | Alox12 | 239 | 17-May-15 |
| 2291 | 2 | 3 | 4 | | IV-2 | Alox12b | 242 | 23-May-15 |
| 2296 | 2 | 3 | 4 | | IV-2 | Alox8 | | |
| 2297 | 2 | 3 | 4 | | IV-2 | Aloxe3 | 59344 | 23-May-15 |
| 2308 | 2 | 3 | 4 | | IV-2 | Alx1 | 8092 | 12-May-15 |
| 2313 | 2 | 3 | 4 | | IV-2 | Amacr | 23600 | 12-May-15 |
| 2314 | 2 | 3 | 4 | | IV-2 | Ambn | 258 | 4-May-15 |
| 2316 | 2 | 3 | 4 | | IV-2 | Ambra1 | 55626 | 21-May-15 |
| 2318 | 2 | 3 | 4 | | IV-2 | Amd2 | 263 | 4-May-15 |
| 2319 | 2 | 3 | 4 | | IV-2 | Amdhd1 | 144193 | 4-May-15 |
| 2320 | 2 | 3 | 4 | | IV-2 | Amdhd2 | 51005 | 4-May-15 |
| 2333 | 2 | 3 | 4 | | IV-2 | Ammecr1l | 83607 | 4-May-15 |
| 2340 | 2 | 3 | 4 | | IV-2 | Ampd2 | 271 | 12-May-15 |
| 2344 | 2 | 3 | 4 | | IV-2 | Amtn | 401138 | 4-May-15 |
| 2349 | 2 | 3 | 4 | | IV-2 | Amz1 | 155185 | 4-May-15 |
| 2362 | 2 | 3 | 4 | | IV-2 | Ang2 | | |
| 2363 | 2 | 3 | 4 | | IV-2 | Ang3 | | |
| 2365 | 2 | 3 | 4 | | IV-2 | Ang5 | 27329 | 4-May-15 |
| 2372 | 2 | 3 | 4 | | IV-2 | Angptl1 | 9068 | 4-May-15 |
| 2373 | 2 | 3 | 4 | | IV-2 | Angptl2 | 23452 | 12-May-15 |
| 2376 | 2 | 3 | 4 | | IV-2 | Angptl6 | 83854 | 4-May-15 |
| 2378 | 2 | 3 | 4 | | IV-2 | Ank | 56172 | 7-Jun-15 |
| 2380 | 2 | 3 | 4 | | IV-2 | Ank2 | 287 | 12-May-15 |
| 2391 | 2 | 3 | 4 | | IV-2 | Ankle2 | 23141 | 4-May-15 |
| 2408 | 2 | 3 | 4 | | IV-2 | Ankrd24 | 170961 | 4-May-15 |
| 2412 | 2 | 3 | 4 | | IV-2 | Ankrd29 | 147463 | 12-May-15 |
| 2413 | 2 | 3 | 4 | | IV-2 | Ankrd32 | 84250 | 4-May-15 |
| 2422 | 2 | 3 | 4 | | IV-2 | Ankrd39 | 51239 | 4-May-15 |
| 2434 | 2 | 3 | 4 | | IV-2 | Ankrd6 | 22881 | 4-May-15 |
| 2449 | 2 | 3 | 4 | | IV-2 | Ano1 | 55107 | 29-May-15 |
| 2455 | 2 | 3 | 4 | | IV-2 | Ano6 | 196527 | 23-May-15 |
| 2470 | 2 | 3 | 4 | | IV-2 | Anxa2 | 302 | 17-May-15 |
| 2472 | 2 | 3 | 4 | | IV-2 | Anxa4 | 307 | 4-May-15 |
| 2478 | 2 | 3 | 4 | | IV-2 | Aoah | 313 | 12-May-15 |
| 2486 | 2 | 3 | 4 | | IV-2 | Ap1ar | 55435 | 4-May-15 |
| 2495 | 2 | 3 | 4 | | IV-2 | Ap2a1 | 160 | 12-May-15 |
| 2506 | 2 | 3 | 4 | | IV-2 | Ap3s2 | 10239 | 4-May-15 |
| 2527 | 2 | 3 | 4 | | IV-2 | Apeh | 327 | 21-May-15 |
| 2530 | 2 | 3 | 4 | | IV-2 | Apex2 | 27301 | 12-May-15 |
| 2537 | 2 | 3 | 4 | | IV-2 | Aplf | 200558 | 4-May-15 |
| 2540 | 2 | 3 | 4 | | IV-2 | Aplp1 | 333 | 4-May-15 |
| 2544 | 2 | 3 | 4 | | IV-2 | Apoa1bp | 128240 | 12-May-15 |
| 2548 | 2 | 3 | 4 | | IV-2 | Apob | 338 | 17-May-15 |
| 2551 | 2 | 3 | 4 | | IV-2 | Apobec3 | | |
| 2562 | 2 | 3 | 4 | | IV-2 | Apol10a | | |
| 2563 | 2 | 3 | 4 | | IV-2 | Apol10b | | |
| 2564 | 2 | 3 | 4 | | IV-2 | Apol11a | | |
| 2567 | 2 | 3 | 4 | | IV-2 | Apol7a | | |
| 2575 | 2 | 3 | 4 | | IV-2 | Apold1 | 81575 | 4-May-15 |
| 2580 | 2 | 3 | 4 | | IV-2 | Apoo-ps | | |
| 2589 | 2 | 3 | 4 | | IV-2 | Aqp11 | 282679 | 4-May-15 |
| 2591 | 2 | 3 | 4 | | IV-2 | Aqp2 | 359 | 23-May-15 |
| 2597 | 2 | 3 | 4 | | IV-2 | Aqp8 | 343 | 17-May-15 |
| 2601 | 2 | 3 | 4 | | IV-2 | Araf | 369 | 4-May-15 |
| 2608 | 2 | 3 | 4 | | IV-2 | Arel1 | 9870 | 4-May-15 |
| 2625 | 2 | 3 | 4 | | IV-2 | Arglu1 | 55082 | 12-May-15 |
| 2629 | 2 | 3 | 4 | | IV-2 | Arhgap12 | 94134 | 4-May-15 |
| 2631 | 2 | 3 | 4 | | IV-2 | Arhgap15os | | |
| 2636 | 2 | 3 | 4 | | IV-2 | Arhgap20os | | |
| 2643 | 2 | 3 | 4 | | IV-2 | Arhgap27 | 201176 | 4-May-15 |
| 2648 | 2 | 3 | 4 | | IV-2 | Arhgap31 | 57514 | 4-May-15 |
| 2656 | 2 | 3 | 4 | | IV-2 | Arhgap40 | 343578 | 4-May-15 |
| 2676 | 2 | 3 | 4 | | IV-2 | Arhgef2 | 9181 | 4-May-15 |
| 2701 | 2 | 3 | 4 | | IV-2 | Arih1 | 25820 | 31-May-15 |
| 2706 | 2 | 3 | 4 | | IV-2 | Arl13a | 392509 | 4-May-15 |
| 2721 | 2 | 3 | 4 | | IV-2 | Arl5c | 390790 | 4-May-15 |
| 2722 | 2 | 3 | 4 | | IV-2 | Arl6 | 84100 | 7-Jun-15 |
| 2723 | 2 | 3 | 4 | | IV-2 | Arl6ip1 | 23204 | 4-May-15 |
| 2743 | 2 | 3 | 4 | | IV-2 | Armcx3 | 51566 | 4-May-15 |
| 2747 | 2 | 3 | 4 | | IV-2 | Arnt | 405 | 7-Jun-15 |
| 2750 | 2 | 3 | 4 | | IV-2 | Arntl2 | 56938 | 4-May-15 |
| 2760 | 2 | 3 | 4 | | IV-2 | Arr3 | 407 | 12-May-15 |
| 2766 | 2 | 3 | 4 | | IV-2 | Arrdc4 | 91947 | 4-May-15 |
| 2771 | 2 | 3 | 4 | | IV-2 | Arsi | 340075 | 23-May-15 |
| 2772 | 2 | 3 | 4 | | IV-2 | Arsj | 79642 | 4-May-15 |
| 2778 | 2 | 3 | 4 | | IV-2 | Art4 | 420 | 7-Jun-15 |
| 2780 | 2 | 3 | 4 | | IV-2 | Artn | 9048 | 4-May-15 |
| 2785 | 2 | 3 | 4 | | IV-2 | Arxes2 | | |
| 2796 | 2 | 3 | 4 | | IV-2 | Asb13 | 79754 | 4-May-15 |
| 2800 | 2 | 3 | 4 | | IV-2 | Asb17 | 127247 | 4-May-15 |
| 2804 | 2 | 3 | 4 | | IV-2 | Asb3 | 51130 | 10-May-15 |
| 2807 | 2 | 3 | 4 | | IV-2 | Asb6 | 140459 | 4-May-15 |
| 2821 | 2 | 3 | 4 | | IV-2 | Asgr1 | 432 | 4-May-15 |
| 2834 | 2 | 3 | 4 | | IV-2 | Asnsd1 | 54529 | 4-May-15 |
| 2840 | 2 | 3 | 4 | | IV-2 | Asphd2 | 57168 | 23-May-15 |
| 2842 | 2 | 3 | 4 | | IV-2 | Aspn | 54829 | 12-May-15 |
| 2844 | 2 | 3 | 4 | | IV-2 | Aspscr1 | 79058 | 4-May-15 |
| 2847 | 2 | 3 | 4 | | IV-2 | Aste1 | 28990 | 12-May-15 |
| 2858 | 2 | 3 | 4 | | IV-2 | Atad2b | 54454 | 12-May-15 |
| 2861 | 2 | 3 | 4 | | IV-2 | Atad5 | 79915 | 4-May-15 |
| 2862 | 2 | 3 | 4 | | IV-2 | Atat1 | 79969 | 4-May-15 |
| 2865 | 2 | 3 | 4 | | IV-2 | Ate1 | 11101 | 4-May-15 |
| 2869 | 2 | 3 | 4 | | IV-2 | Atf4 | 468 | 4-May-15 |
| 2874 | 2 | 3 | 4 | | IV-2 | Atf7ip | 55729 | 12-May-15 |
| 2877 | 2 | 3 | 4 | | IV-2 | Atg101 | 60673 | 21-May-15 |
| 2894 | 2 | 3 | 4 | | IV-2 | Athl1 | 80162 | 12-May-15 |
| 2910 | 2 | 3 | 4 | | IV-2 | Atp11b | 23200 | 4-May-15 |
| 2927 | 2 | 3 | 4 | | IV-2 | Atp2a2 | 488 | 24-May-15 |
| 2933 | 2 | 3 | 4 | | IV-2 | Atp2c1 | 27032 | 4-May-15 |
| 2950 | 2 | 3 | 4 | | IV-2 | Atp5l | 10632 | 4-May-15 |
| 2963 | 2 | 3 | 4 | | IV-2 | Atp6v0d1 | 9114 | 4-May-15 |
| 2965 | 2 | 3 | 4 | | IV-2 | Atp6v0e | 8992 | 4-May-15 |
| 2969 | 2 | 3 | 4 | | IV-2 | Atp6v1b2 | 526 | 4-May-15 |
| 3021 | 2 | 3 | 4 | | IV-2 | AU022252 | | |
| 3028 | 2 | 3 | 4 | | IV-2 | AU041133 | | |
| 3030 | 2 | 3 | 4 | | IV-2 | Aup1 | 550 | 3-May-15 |
| 3032 | 2 | 3 | 4 | | IV-2 | Aurkaip1 | 54998 | 4-May-15 |
| 3034 | 2 | 3 | 4 | | IV-2 | Aurkc | 6795 | 21-May-15 |
| 3037 | 2 | 3 | 4 | | IV-2 | AV051173 | | |
| 3038 | 2 | 3 | 4 | | IV-2 | AV320801 | | |
| 3041 | 2 | 3 | 4 | | IV-2 | Avl9 | 23080 | 12-May-15 |
| 3045 | 2 | 3 | 4 | | IV-2 | Avpr1b | 553 | 17-May-15 |
| 3050 | 2 | 3 | 4 | | IV-2 | AW146154 | | |
| 3054 | 2 | 3 | 4 | | IV-2 | AW549877 | | |
| 3062 | 2 | 3 | 4 | | IV-2 | Axl | 558 | 17-May-15 |
| 3064 | 2 | 3 | 4 | | IV-2 | AY358078 | | |
| 3071 | 2 | 3 | 4 | | IV-2 | Azi2 | 64343 | 7-Jun-15 |
| 3091 | 2 | 3 | 4 | | IV-2 | B230217O12Rik | | |
| 3094 | 2 | 3 | 4 | | IV-2 | B230319C09Rik | | |
| 3101 | 2 | 3 | 4 | | IV-2 | B3galt2 | 8707 | 23-May-15 |
| 3102 | 2 | 3 | 4 | | IV-2 | B3galt4 | 8705 | 4-May-15 |
| 3104 | 2 | 3 | 4 | | IV-2 | B3galt6 | 126792 | 4-May-15 |
| 3110 | 2 | 3 | 4 | | IV-2 | B3gnt2 | 10678 | 4-May-15 |
| 3114 | 2 | 3 | 4 | | IV-2 | B3gnt6 | 192134 | 4-May-15 |
| 3116 | 2 | 3 | 4 | | IV-2 | B3gnt8 | 374907 | 4-May-15 |
| 3121 | 2 | 3 | 4 | | IV-2 | B430306N03Rik | | |
| 3137 | 2 | 3 | 4 | | IV-2 | B930003M22Rik | | |
| 3138 | 2 | 3 | 4 | | IV-2 | B930018H19Rik | | |
| 3144 | 2 | 3 | 4 | | IV-2 | B9d2 | 80776 | 4-May-15 |
| 3152 | 2 | 3 | 4 | | IV-2 | Bach2os | | |
| 3156 | 2 | 3 | 4 | | IV-2 | Bag3 | 9531 | 24-May-15 |
| 3171 | 2 | 3 | 4 | | IV-2 | Bambi-ps1 | | |
| 3175 | 2 | 3 | 4 | | IV-2 | Banp | 54971 | 12-May-15 |
| 3178 | 2 | 3 | 4 | | IV-2 | Barhl1 | 56751 | 28-May-15 |
| 3184 | 2 | 3 | 4 | | IV-2 | Batf2 | 116071 | 12-May-15 |

Fig.22 - 9

| | | | | | | | | |
|---|---|---|---|---|---|---|---|---|
| 3186 | 2 | 3 | 4 | | IV-2 | Bax | 581 | 24-May-15 |
| 3216 | 2 | 3 | 4 | | IV-2 | BC005624 | | |
| 3227 | 2 | 3 | 4 | | IV-2 | BC021767 | | |
| 3228 | 2 | 3 | 4 | | IV-2 | BC021785 | | |
| 3237 | 2 | 3 | 4 | | IV-2 | BC027072 | | |
| 3247 | 2 | 3 | 4 | | IV-2 | BC030870 | | |
| 3261 | 2 | 3 | 4 | | IV-2 | BC048562 | | |
| 3272 | 2 | 3 | 4 | | IV-2 | BC051019 | | |
| 3275 | 2 | 3 | 4 | | IV-2 | BC051537 | | |
| 3290 | 2 | 3 | 4 | | IV-2 | BC065397 | | |
| 3296 | 2 | 3 | 4 | | IV-2 | BC094916 | | |
| 3300 | 2 | 3 | 4 | | IV-2 | BC107364 | | |
| 3310 | 2 | 3 | 4 | | IV-2 | Bcas1os2 | | |
| 3325 | 2 | 3 | 4 | | IV-2 | Bcl11b | 64919 | 28-May-15 |
| 3326 | 2 | 3 | 4 | | IV-2 | Bcl2 | 596 | 31-May-15 |
| 3329 | 2 | 3 | 4 | | IV-2 | Bcl2a1c | | |
| 3331 | 2 | 3 | 4 | | IV-2 | Bcl2l1 | 598 | 24-May-15 |
| 3341 | 2 | 3 | 4 | | IV-2 | Bcl6b | 255877 | 4-May-15 |
| 3342 | 2 | 3 | 4 | | IV-2 | Bcl7a | 605 | 4-May-15 |
| 3349 | 2 | 3 | 4 | | IV-2 | Bco2 | 83875 | 4-May-15 |
| 3350 | 2 | 3 | 4 | | IV-2 | Bcor | 54880 | 23-May-15 |
| 3356 | 2 | 3 | 4 | | IV-2 | Bdkrb1 | 623 | 12-May-15 |
| 3358 | 2 | 3 | 4 | | IV-2 | Bdnf | 627 | 7-Jun-15 |
| 3360 | 2 | 3 | 4 | | IV-2 | Bean1 | 146227 | 23-May-15 |
| 3372 | 2 | 3 | 4 | | IV-2 | Bet1 | 10282 | 4-May-15 |
| 3377 | 2 | 3 | 4 | | IV-2 | Bex6 | | |
| 3378 | 2 | 3 | 4 | | IV-2 | Bfar | 51283 | 4-May-15 |
| 3385 | 2 | 3 | 4 | | IV-2 | Bhlha15 | 168620 | 14-May-15 |
| 3393 | 2 | 3 | 4 | | IV-2 | Bhmt2 | 23743 | 4-May-15 |
| 3395 | 2 | 3 | 4 | | IV-2 | Bicd1 | 636 | 3-May-15 |
| 3401 | 2 | 3 | 4 | | IV-2 | Bin3 | 55909 | 4-May-15 |
| 3405 | 2 | 3 | 4 | | IV-2 | Birc6 | 57448 | 21-May-15 |
| 3411 | 2 | 3 | 4 | | IV-2 | Blmh | 642 | 23-May-15 |
| 3421 | 2 | 3 | 4 | | IV-2 | Blzf1 | 8548 | 4-May-15 |
| 3432 | 2 | 3 | 4 | | IV-2 | Bmp6 | 654 | 17-May-15 |
| 3435 | 2 | 3 | 4 | | IV-2 | Bmp8b | 656 | 4-May-15 |
| 3437 | 2 | 3 | 4 | | IV-2 | Bmpr1a | 657 | 23-May-15 |
| 3443 | 2 | 3 | 4 | | IV-2 | Bnc1 | 646 | 7-Jun-15 |
| 3444 | 2 | 3 | 4 | | IV-2 | Bnc2 | 54796 | 4-May-15 |
| 3469 | 2 | 3 | 4 | | IV-2 | Bpifb2 | 80341 | 4-May-15 |
| 3475 | 2 | 3 | 4 | | IV-2 | Bpifb9b | | |
| 3483 | 2 | 3 | 4 | | IV-2 | Brca2 | 675 | 25-May-15 |
| 3484 | 2 | 3 | 4 | | IV-2 | Brcc3 | 79184 | 7-Jun-15 |
| 3503 | 2 | 3 | 4 | | IV-2 | Brix1 | 55299 | 4-May-15 |
| 3515 | 2 | 3 | 4 | | IV-2 | Bscl2 | 26580 | 23-May-15 |
| 3524 | 2 | 3 | 4 | | IV-2 | Bst2 | 684 | 31-May-15 |
| 3525 | 2 | 3 | 4 | | IV-2 | Bsx | 390259 | 4-May-15 |
| 3538 | 2 | 3 | 4 | | IV-2 | Btbd8 | 284697 | 4-May-15 |
| 3541 | 2 | 3 | 4 | | IV-2 | Btd | 686 | 7-Jun-15 |
| 3547 | 2 | 3 | 4 | | IV-2 | Btg4 | 54766 | 14-May-15 |
| 3550 | 2 | 3 | 4 | | IV-2 | Btn1a1 | 696 | 20-May-15 |
| 3554 | 2 | 3 | 4 | | IV-2 | Btnl2 | 56244 | 12-May-15 |
| 3559 | 2 | 3 | 4 | | IV-2 | Btrc | 8945 | 4-May-15 |
| 3561 | 2 | 3 | 4 | | IV-2 | Bub1b | 701 | 24-May-15 |
| 3562 | 2 | 3 | 4 | | IV-2 | Bub3 | 9184 | 12-May-15 |
| 3566 | 2 | 3 | 4 | | IV-2 | Bysl | 705 | 21-May-15 |
| 3615 | 2 | 3 | 4 | | IV-2 | C1ra | | |
| 3627 | 2 | 3 | 4 | | IV-2 | C230079O03Rik | | |
| 3634 | 2 | 3 | 4 | | IV-2 | C2cd4c | 126567 | 4-May-15 |
| 3636 | 2 | 3 | 4 | | IV-2 | C2cd5 | 9847 | 4-May-15 |
| 3642 | 2 | 3 | 4 | | IV-2 | C330013F16Rik | | |
| 3649 | 2 | 3 | 4 | | IV-2 | C330046G13Rik | | |
| 3664 | 2 | 3 | 4 | | IV-2 | C630028M04Rik | | |
| 3668 | 2 | 3 | 4 | | IV-2 | C730002L08Rik | | |
| 3674 | 2 | 3 | 4 | | IV-2 | C86187 | | |
| 3684 | 2 | 3 | 4 | | IV-2 | C9 | 735 | 7-Jun-15 |
| 3696 | 2 | 3 | 4 | | IV-2 | Cabp2 | 51475 | 4-May-15 |
| 3704 | 2 | 3 | 4 | | IV-2 | Cacna1a | 773 | 22-May-15 |
| 3705 | 2 | 3 | 4 | | IV-2 | Cacna1b | 774 | 12-May-15 |
| 3709 | 2 | 3 | 4 | | IV-2 | Cacna1f | 778 | 23-May-15 |
| 3711 | 2 | 3 | 4 | | IV-2 | Cacna1h | 8912 | 4-May-15 |
| 3715 | 2 | 3 | 4 | | IV-2 | Cacna2d2 | 9254 | 12-May-15 |
| 3716 | 2 | 3 | 4 | | IV-2 | Cacna2d3 | 55799 | 4-May-15 |
| 3723 | 2 | 3 | 4 | | IV-2 | Cacng2 | 10369 | 4-May-15 |
| 3730 | 2 | 3 | 4 | | IV-2 | Cactin | 58509 | 4-May-15 |
| 3737 | 2 | 3 | 4 | | IV-2 | Cadm4 | 199731 | 1-Jun-15 |
| 3745 | 2 | 3 | 4 | | IV-2 | Calcoco1 | 57658 | 29-May-15 |
| 3759 | 2 | 3 | 4 | | IV-2 | Caln1 | 83698 | 4-May-15 |
| 3771 | 2 | 3 | 4 | | IV-2 | Camk2g | 818 | 4-May-15 |
| 3775 | 2 | 3 | 4 | | IV-2 | Camkk1 | 84254 | 4-May-15 |
| 3781 | 2 | 3 | 4 | | IV-2 | Camsap1 | 157922 | 4-May-15 |
| 3788 | 2 | 3 | 4 | | IV-2 | Cant1 | 124583 | 4-May-15 |
| 3797 | 2 | 3 | 4 | | IV-2 | Capn13 | 92291 | 4-May-15 |
| 3798 | 2 | 3 | 4 | | IV-2 | Capn15 | 6650 | 12-May-15 |
| 3817 | 2 | 3 | 4 | | IV-2 | Car10 | 8038 | 4-May-15 |
| 3820 | 2 | 3 | 4 | | IV-2 | Car13 | | |
| 3822 | 2 | 3 | 4 | | IV-2 | Car15 | | |
| 3826 | 2 | 3 | 4 | | IV-2 | Car5a | | |
| 3829 | 2 | 3 | 4 | | IV-2 | Car7 | | |
| 3834 | 2 | 3 | 4 | | IV-2 | Card14 | 79092 | 4-May-15 |
| 3849 | 2 | 3 | 4 | | IV-2 | Casd1 | 64921 | 4-May-15 |
| 3859 | 2 | 3 | 4 | | IV-2 | Casp6 | 839 | 4-May-15 |
| 3860 | 2 | 3 | 4 | | IV-2 | Casp7 | 840 | 12-May-15 |
| 3863 | 2 | 3 | 4 | | IV-2 | Casp9 | 842 | 4-May-15 |
| 3881 | 2 | 3 | 4 | | IV-2 | Cav2 | 858 | 4-May-15 |
| 3883 | 2 | 3 | 4 | | IV-2 | Cbfa2t2 | 9139 | 12-May-15 |
| 3887 | 2 | 3 | 4 | | IV-2 | Cblb | 868 | 7-Jun-15 |
| 3899 | 2 | 3 | 4 | | IV-2 | Cbwd1 | 55871 | 4-May-15 |
| 3905 | 2 | 3 | 4 | | IV-2 | Cbx6 | 23466 | 12-May-15 |
| 3916 | 2 | 3 | 4 | | IV-2 | Ccbl2 | 56267 | 4-May-15 |
| 3923 | 2 | 3 | 4 | | IV-2 | Ccdc107 | 203260 | 4-May-15 |
| 3960 | 2 | 3 | 4 | | IV-2 | Ccdc150 | 284992 | 4-May-15 |
| 3964 | 2 | 3 | 4 | | IV-2 | Ccdc154 | 645811 | 4-May-15 |
| 3986 | 2 | 3 | 4 | | IV-2 | Ccdc181 | 57821 | 4-May-15 |
| 3999 | 2 | 3 | 4 | | IV-2 | Ccdc30 | 728621 | 4-May-15 |
| 4024 | 2 | 3 | 4 | | IV-2 | Ccdc62 | 84660 | 4-May-15 |
| 4033 | 2 | 3 | 4 | | IV-2 | Ccdc7 | 79741 | 4-May-15 |
| 4039 | 2 | 3 | 4 | | IV-2 | Ccdc77 | 84318 | |
| 4043 | 2 | 3 | 4 | | IV-2 | Ccdc80 | 151887 | |
| 4044 | 2 | 3 | 4 | | IV-2 | Ccdc81 | 60494 | 28-May-15 |
| 4049 | 2 | 3 | 4 | | IV-2 | Ccdc85b | 11007 | 4-May-15 |
| 4051 | 2 | 3 | 4 | | IV-2 | Ccdc86 | 79080 | 4-May-15 |
| 4061 | 2 | 3 | 4 | | IV-2 | Ccdc93 | 54520 | 4-May-15 |
| 4069 | 2 | 3 | 4 | | IV-2 | Cckar | 886 | 12-May-15 |
| 4073 | 2 | 3 | 4 | | IV-2 | Ccl12 | | |
| 4082 | 2 | 3 | 4 | | IV-2 | Ccl24 | 6369 | |
| 4083 | 2 | 3 | 4 | | IV-2 | Ccl25 | 6370 | 4-May-15 |
| 4084 | 2 | 3 | 4 | | IV-2 | Ccl26 | 10344 | 17-May-15 |
| 4088 | 2 | 3 | 4 | | IV-2 | Ccl3 | 6348 | |
| 4089 | 2 | 3 | 4 | | IV-2 | Ccl4 | 6351 | 3-May-15 |
| 4100 | 2 | 3 | 4 | | IV-2 | Ccnb1ip1 | 57820 | |
| 4102 | 2 | 3 | 4 | | IV-2 | Ccnb3 | 85417 | 4-May-15 |
| 4105 | 2 | 3 | 4 | | IV-2 | Ccnd2 | 894 | 4-May-15 |
| 4110 | 2 | 3 | 4 | | IV-2 | Ccnf | 899 | 4-May-15 |
| 4111 | 2 | 3 | 4 | | IV-2 | Ccng1 | 900 | 4-May-15 |
| 4117 | 2 | 3 | 4 | | IV-2 | Ccnk | 8812 | 4-May-15 |
| 4122 | 2 | 3 | 4 | | IV-2 | Ccnt2 | 905 | 4-May-15 |
| 4130 | 2 | 3 | 4 | | IV-2 | Ccr1l1 | | |
| 4137 | 2 | 3 | 4 | | IV-2 | Ccr8 | 1237 | 4-May-15 |
| 4166 | 2 | 3 | 4 | | IV-2 | Cd19 | 930 | 12-May-15 |
| 4168 | 2 | 3 | 4 | | IV-2 | Cd1d2 | | |
| 4169 | 2 | 3 | 4 | | IV-2 | Cd2 | 914 | 12-May-15 |
| 4170 | 2 | 3 | 4 | | IV-2 | Cd200 | 4345 | |
| 4172 | 2 | 3 | 4 | | IV-2 | Cd200r2 | 344807 | 4-May-15 |
| 4178 | 2 | 3 | 4 | | IV-2 | Cd209c | | |
| 4180 | 2 | 3 | 4 | | IV-2 | Cd209e | | |
| 4182 | 2 | 3 | 4 | | IV-2 | Cd209g | | |
| 4184 | 2 | 3 | 4 | | IV-2 | Cd226 | 10666 | 12-May-15 |
| 4187 | 2 | 3 | 4 | | IV-2 | Cd248 | 57124 | |
| 4190 | 2 | 3 | 4 | | IV-2 | Cd274 | 29126 | 24-May-15 |
| 4191 | 2 | 3 | 4 | | IV-2 | Cd276 | 80381 | |
| 4192 | 2 | 3 | 4 | | IV-2 | Cd28 | 940 | 12-May-15 |
| 4193 | 2 | 3 | 4 | | IV-2 | Cd2ap | 23607 | 4-May-15 |
| 4199 | 2 | 3 | 4 | | IV-2 | Cd300ld | 100131<br>439 | 4-May-15 |
| 4203 | 2 | 3 | 4 | | IV-2 | Cd302 | 9936 | 4-May-15 |
| 4206 | 2 | 3 | 4 | | IV-2 | Cd34 | 947 | 17-May-15 |
| 4209 | 2 | 3 | 4 | | IV-2 | Cd38 | 952 | |
| 4212 | 2 | 3 | 4 | | IV-2 | Cd3eap | 10849 | 4-May-15 |
| 4215 | 2 | 3 | 4 | | IV-2 | Cd40 | 958 | 12-May-15 |
| 4216 | 2 | 3 | 4 | | IV-2 | Cd40lg | 959 | 31-May-15 |
| 4218 | 2 | 3 | 4 | | IV-2 | Cd46 | 4179 | 23-May-15 |
| 4224 | 2 | 3 | 4 | | IV-2 | Cd55 | 1604 | |
| 4232 | 2 | 3 | 4 | | IV-2 | Cd7 | 924 | 4-May-15 |
| 4235 | 2 | 3 | 4 | | IV-2 | Cd74 | 972 | |
| 4238 | 2 | 3 | 4 | | IV-2 | Cd80 | 941 | |
| 4243 | 2 | 3 | 4 | | IV-2 | Cd86 | 942 | 31-May-15 |
| 4252 | 2 | 3 | 4 | | IV-2 | Cdadc1 | 81602 | 4-May-15 |
| 4259 | 2 | 3 | 4 | | IV-2 | Cdc20b | 166979 | 4-May-15 |
| 4264 | 2 | 3 | 4 | | IV-2 | Cdc26 | 246184 | 4-May-15 |
| 4279 | 2 | 3 | 4 | | IV-2 | Cdc42se1 | 56882 | 21-May-15 |
| 4282 | 2 | 3 | 4 | | IV-2 | Cdc5l | 988 | 24-May-15 |
| 4284 | 2 | 3 | 4 | | IV-2 | Cdc7 | 8317 | |
| 4288 | 2 | 3 | 4 | | IV-2 | Cdca4 | 55038 | |
| 4290 | 2 | 3 | 4 | | IV-2 | Cdca7 | 83879 | |
| 4298 | 2 | 3 | 4 | | IV-2 | Cdh12 | 1010 | 4-May-15 |
| 4302 | 2 | 3 | 4 | | IV-2 | Cdh17 | 1015 | 17-May-15 |
| 4315 | 2 | 3 | 4 | | IV-2 | Cdh7 | 1005 | 7-Jun-15 |
| 4325 | 2 | 3 | 4 | | IV-2 | Cdk10 | 8558 | 12-May-15 |
| 4348 | 2 | 3 | 4 | | IV-2 | Cdk7 | 1022 | 4-May-15 |
| 4373 | 2 | 3 | 4 | | IV-2 | Cdr2l | 30850 | 12-May-15 |
| 4415 | 2 | 3 | 4 | | IV-2 | Celf1 | 10658 | 2-Jun-15 |
| 4427 | 2 | 3 | 4 | | IV-2 | Cenpa | 1058 | 17-May-15 |
| 4428 | 2 | 3 | 4 | | IV-2 | Cenpb | 1059 | 4-May-15 |
| 4431 | 2 | 3 | 4 | | IV-2 | Cenpf | 1063 | 21-May-15 |
| 4433 | 2 | 3 | 4 | | IV-2 | Cenpi | 2491 | |
| 4434 | 2 | 3 | 4 | | IV-2 | Cenpj | 55835 | 31-May-15 |
| 4436 | 2 | 3 | 4 | | IV-2 | Cenpl | 91687 | |
| 4438 | 2 | 3 | 4 | | IV-2 | Cenpn | 55839 | 4-May-15 |
| 4439 | 2 | 3 | 4 | | IV-2 | Cenpo | 79172 | 4-May-15 |
| 4441 | 2 | 3 | 4 | | IV-2 | Cenpq | 55166 | |
| 4444 | 2 | 3 | 4 | | IV-2 | Cenpv | 201161 | 4-May-15 |
| 4459 | 2 | 3 | 4 | | IV-2 | Cep250 | 11190 | 17-May-15 |
| 4465 | 2 | 3 | 4 | | IV-2 | Cep57 | 9702 | 4-May-15 |
| 4477 | 2 | 3 | 4 | | IV-2 | Cep89 | 84902 | 12-May-15 |
| 4483 | 2 | 3 | 4 | | IV-2 | Cerk | 64781 | |
| 4486 | 2 | 3 | 4 | | IV-2 | Cers2 | 29956 | 4-May-15 |
| 4491 | 2 | 3 | 4 | | IV-2 | Ces1a | | |
| 4500 | 2 | 3 | 4 | | IV-2 | Ces2c | | |
| 4501 | 2 | 3 | 4 | | IV-2 | Ces2d-ps | | |
| 4503 | 2 | 3 | 4 | | IV-2 | Ces2f | | |
| 4505 | 2 | 3 | 4 | | IV-2 | Ces2h | | |
| 4517 | 2 | 3 | 4 | | IV-2 | Cfdp1 | 10428 | 4-May-15 |
| 4532 | 2 | 3 | 4 | | IV-2 | Cgrrf1 | 10668 | 21-May-15 |
| 4537 | 2 | 3 | 4 | | IV-2 | Chadl | 150356 | 12-May-15 |

Fig.22 - 10

| | | | | | | | | |
|---|---|---|---|---|---|---|---|---|
| 4548 | 2 | 3 | 4 | | IV-2 | Chchd6 | 84303 | 2-Jun-15 |
| 4563 | 2 | 3 | 4 | | IV-2 | Chek2 | 11200 | |
| 4569 | 2 | 3 | 4 | | IV-2 | Chic1 | 53344 | 4-May-15 |
| 4575 | 2 | 3 | 4 | | IV-2 | Chil6 | | |
| 4583 | 2 | 3 | 4 | | IV-2 | Chmp1a | 5119 | 4-May-15 |
| 4597 | 2 | 3 | 4 | | IV-2 | Chordc1 | 26973 | 4-May-15 |
| 4606 | 2 | 3 | 4 | | IV-2 | Chrdl2 | 25884 | 4-May-15 |
| 4617 | 2 | 3 | 4 | | IV-2 | Chrna5 | 1138 | 24-May-15 |
| 4620 | 2 | 3 | 4 | | IV-2 | Chrna9 | 55584 | 12-May-15 |
| 4631 | 2 | 3 | 4 | | IV-2 | Chst12 | 55501 | |
| 4637 | 2 | 3 | 4 | | IV-2 | Chst4 | 10164 | |
| 4640 | 2 | 3 | 4 | | IV-2 | Chst8 | 64377 | 4-May-15 |
| 4654 | 2 | 3 | 4 | | IV-2 | Cib3 | 117286 | |
| 4655 | 2 | 3 | 4 | | IV-2 | Cib4 | 130106 | 4-May-15 |
| 4658 | 2 | 3 | 4 | | IV-2 | Cideb | 27141 | 12-May-15 |
| 4660 | 2 | 3 | 4 | | IV-2 | Ciita | 4261 | 12-May-15 |
| 4663 | 2 | 3 | 4 | | IV-2 | Cinp | 51550 | 4-May-15 |
| 4677 | 2 | 3 | 4 | | IV-2 | Ciz1 | 25792 | 12-May-15 |
| 4680 | 2 | 3 | 4 | | IV-2 | Ckap2l | 150468 | 12-May-15 |
| 4681 | 2 | 3 | 4 | | IV-2 | Ckap4 | 10970 | 4-May-15 |
| 4686 | 2 | 3 | 4 | | IV-2 | Ckmt1 | 548596, 1159 | /6/7 |
| 4691 | 2 | 3 | 4 | | IV-2 | Clasp1 | 23332 | 28-May-15 |
| 4697 | 2 | 3 | 4 | | IV-2 | Clca4 | 22802 | 12-May-15 |
| 4703 | 2 | 3 | 4 | | IV-2 | Clcn2 | 1181 | |
| 4713 | 2 | 3 | 4 | | IV-2 | Cldn11 | 5010 | 12-May-15 |
| 4718 | 2 | 3 | 4 | | IV-2 | Cldn16 | 10686 | 4-May-15 |
| 4723 | 2 | 3 | 4 | | IV-2 | Cldn20 | 49861 | |
| 4732 | 2 | 3 | 4 | | IV-2 | Cldn6 | 9074 | 4-May-15 |
| 4740 | 2 | 3 | 4 | | IV-2 | Clec12b | 387837 | 4-May-15 |
| 4742 | 2 | 3 | 4 | | IV-2 | Clec16a | 23274 | 10-May-15 |
| 4751 | 2 | 3 | 4 | | IV-2 | Clec2i | | |
| 4756 | 2 | 3 | 4 | | IV-2 | Clec4a2 | | |
| 4758 | 2 | 3 | 4 | | IV-2 | Clec4a4 | | |
| 4768 | 2 | 3 | 4 | | IV-2 | Clec9a | 283420 | 1-May-15 |
| 4771 | 2 | 3 | 4 | | IV-2 | Clic1 | 1192 | |
| 4773 | 2 | 3 | 4 | | IV-2 | Clic4 | 25932 | 4-May-15 |
| 4776 | 2 | 3 | 4 | | IV-2 | Clint1 | 9685 | 12-May-15 |
| 4808 | 2 | 3 | 4 | | IV-2 | Clstn3 | 9746 | 4-May-15 |
| 4809 | 2 | 3 | 4 | | IV-2 | Clta | 1211 | 4-May-15 |
| 4813 | 2 | 3 | 4 | | IV-2 | Cluap1 | 23059 | 4-May-15 |
| 4819 | 2 | 3 | 4 | | IV-2 | Cma2 | | |
| 4820 | 2 | 3 | 4 | | IV-2 | Cmah | 8418 | 3-May-15 |
| 4821 | 2 | 3 | 4 | | IV-2 | Cmas | 55907 | 12-May-15 |
| 4828 | 2 | 3 | 4 | | IV-2 | Cml2 | 51471 | 4-May-15 |
| 4829 | 2 | 3 | 4 | | IV-2 | Cml3 | 339983 | 4-May-15 |
| 4831 | 2 | 3 | 4 | | IV-2 | Cmpk1 | 51727 | 14-May-15 |
| 4834 | 2 | 3 | 4 | | IV-2 | Cmtm1 | 113540 | 4-May-15 |
| 4846 | 2 | 3 | 4 | | IV-2 | Cnbd2 | 140894 | 12-May-15 |
| 4850 | 2 | 3 | 4 | | IV-2 | Cnep1r1 | 255919 | 4-May-15 |
| 4866 | 2 | 3 | 4 | | IV-2 | Cnn2 | 1265 | |
| 4891 | 2 | 3 | 4 | | IV-2 | Cnst | 163882 | 4-May-15 |
| 4910 | 2 | 3 | 4 | | IV-2 | Cntrob | 116840 | |
| 4931 | 2 | 3 | 4 | | IV-2 | Col11a2 | 1302 | 23-May-15 |
| 4936 | 2 | 3 | 4 | | IV-2 | Col16a1 | 1307 | |
| 4942 | 2 | 3 | 4 | | IV-2 | Col20a1 | 57642 | 4-May-15 |
| 4945 | 2 | 3 | 4 | | IV-2 | Col24a1 | 255631 | 21-May-15 |
| 4946 | 2 | 3 | 4 | | IV-2 | Col25a1 | 84570 | 4-May-15 |
| 4949 | 2 | 3 | 4 | | IV-2 | Col28a1 | 340267 | 4-May-15 |
| 4952 | 2 | 3 | 4 | | IV-2 | Col4a1 | 1282 | |
| 4953 | 2 | 3 | 4 | | IV-2 | Col4a2 | 1284 | |
| 4954 | 2 | 3 | 4 | | IV-2 | Col4a3 | 1285 | |
| 4955 | 2 | 3 | 4 | | IV-2 | Col4a3bp | 10087 | 21-May-15 |
| 4957 | 2 | 3 | 4 | | IV-2 | Col4a5 | 1287 | 23-May-15 |
| 4958 | 2 | 3 | 4 | | IV-2 | Col4a6 | 1288 | 12-May-15 |
| 4960 | 2 | 3 | 4 | | IV-2 | Col5a2 | 1290 | |
| 4961 | 2 | 3 | 4 | | IV-2 | Col5a3 | 50509 | |
| 4962 | 2 | 3 | 4 | | IV-2 | Col6a1 | 1291 | |
| 4967 | 2 | 3 | 4 | | IV-2 | Col6a6 | 131873 | 4-May-15 |
| 4968 | 2 | 3 | 4 | | IV-2 | Col7a1 | 1294 | 31-May-15 |
| 4970 | 2 | 3 | 4 | | IV-2 | Col8a2 | 1296 | 23-May-15 |
| 4976 | 2 | 3 | 4 | | IV-2 | Colec12 | 81035 | 3-May-15 |
| 4979 | 2 | 3 | 4 | | IV-2 | Commd1 | 150684 | 4-May-15 |
| 4986 | 2 | 3 | 4 | | IV-2 | Commd7 | 149951 | 4-May-15 |
| 4990 | 2 | 3 | 4 | | IV-2 | Comt | 1312 | 31-May-15 |
| 4999 | 2 | 3 | 4 | | IV-2 | Cops2 | 9318 | 4-May-15 |
| 5009 | 2 | 3 | 4 | | IV-2 | Coq10a | 93058 | 4-May-15 |
| 5019 | 2 | 3 | 4 | | IV-2 | Coro1a | 11151 | 4-May-15 |
| 5020 | 2 | 3 | 4 | | IV-2 | Coro1b | 57175 | 4-May-15 |
| 5025 | 2 | 3 | 4 | | IV-2 | Coro7 | 79585 | |
| 5037 | 2 | 3 | 4 | | IV-2 | Cox4i1 | 1327 | 4-May-15 |
| 5039 | 2 | 3 | 4 | | IV-2 | Cox5a | 9377 | 4-May-15 |
| 5043 | 2 | 3 | 4 | | IV-2 | Cox6b1 | 1340 | 4-May-15 |
| 5047 | 2 | 3 | 4 | | IV-2 | Cox7a2 | 1347 | 4-May-15 |
| 5054 | 2 | 3 | 4 | | IV-2 | Cox8c | 341947 | 4-May-15 |
| 5058 | 2 | 3 | 4 | | IV-2 | Cpa3 | 1359 | /5/12 |
| 5059 | 2 | 3 | 4 | | IV-2 | Cpa4 | 51200 | 4-May-15 |
| 5060 | 2 | 3 | 4 | | IV-2 | Cpa5 | 93979 | 4-May-15 |
| 5063 | 2 | 3 | 4 | | IV-2 | Cpb2 | 1361 | 4-May-15 |
| 5085 | 2 | 3 | 4 | | IV-2 | Cpne6 | 9362 | 4-May-15 |
| 5093 | 2 | 3 | 4 | | IV-2 | Cpsf1 | 29894 | 4-May-15 |
| 5108 | 2 | 3 | 4 | | IV-2 | Cpxm2 | 119587 | 4-May-15 |
| 5110 | 2 | 3 | 4 | | IV-2 | Cr1l | 1379 | 4-May-15 |
| 5114 | 2 | 3 | 4 | | IV-2 | Cradd | 8738 | 4-May-15 |
| 5126 | 2 | 3 | 4 | | IV-2 | Creb3l2 | 64764 | |
| 5128 | 2 | 3 | 4 | | IV-2 | Creb3l4 | 148327 | 28-May-15 |
| 5136 | 2 | 3 | 4 | | IV-2 | Creld1 | 78987 | 4-May-15 |
| 5143 | 2 | 3 | 4 | | IV-2 | Crim1 | 51232 | 12-May-15 |

| | | | | | | | | |
|---|---|---|---|---|---|---|---|---|
| 5145 | 2 | 3 | 4 | | IV-2 | Crip2 | 1397 | 4-May-15 |
| 5153 | 2 | 3 | 4 | | IV-2 | Crispld2 | 83716 | |
| 5158 | 2 | 3 | 4 | | IV-2 | Crlf3 | 51379 | 12-May-15 |
| 5162 | 2 | 3 | 4 | | IV-2 | Crnkl1 | 51340 | 28-May-15 |
| 5164 | 2 | 3 | 4 | | IV-2 | Crocc | 9696 | 4-May-15 |
| 5168 | 2 | 3 | 4 | | IV-2 | Crtam | 56253 | 12-May-15 |
| 5179 | 2 | 3 | 4 | | IV-2 | Cryba1 | 1411 | 17-May-15 |
| 5182 | 2 | 3 | 4 | | IV-2 | Crybb1 | 1414 | |
| 5184 | 2 | 3 | 4 | | IV-2 | Crybb3 | 1417 | 4-May-15 |
| 5185 | 2 | 3 | 4 | | IV-2 | Crybg3 | 131544 | 4-May-15 |
| 5193 | 2 | 3 | 4 | | IV-2 | Crygs | 1427 | 4-May-15 |
| 5196 | 2 | 3 | 4 | | IV-2 | Cryz | 1429 | 12-May-15 |
| 5212 | 2 | 3 | 4 | | IV-2 | Csgalnact2 | 55454 | 12-May-15 |
| 5251 | 2 | 3 | 4 | | IV-2 | Cst7 | 8530 | 12-May-15 |
| 5257 | 2 | 3 | 4 | | IV-2 | Cstf1 | 1477 | 12-May-15 |
| 5271 | 2 | 3 | 4 | | IV-2 | Ctdnep1 | 23399 | 4-May-15 |
| 5282 | 2 | 3 | 4 | | IV-2 | Ctif | 9811 | 4-May-15 |
| 5300 | 2 | 3 | 4 | | IV-2 | Ctrc | 11330 | 23-May-15 |
| 5303 | 2 | 3 | 4 | | IV-2 | Cts3 | | |
| 5318 | 2 | 3 | 4 | | IV-2 | Ctsl | 1514 | |
| 5326 | 2 | 3 | 4 | | IV-2 | Ctsz | 1522 | |
| 5335 | 2 | 3 | 4 | | IV-2 | Cubn | 8029 | 12-May-15 |
| 5336 | 2 | 3 | 4 | | IV-2 | Cuedc1 | 404093 | 12-May-15 |
| 5352 | 2 | 3 | 4 | | IV-2 | Cwc15 | 51503 | 4-May-15 |
| 5364 | 2 | 3 | 4 | | IV-2 | Cxcl11 | 6373 | 3-May-15 |
| 5372 | 2 | 3 | 4 | | IV-2 | Cxcl3 | 2921 | 12-May-15 |
| 5374 | 2 | 3 | 4 | | IV-2 | Cxcl9 | 4283 | |
| 5380 | 2 | 3 | 4 | | IV-2 | Cxcr6 | 10663 | 14-May-15 |
| 5384 | 2 | 3 | 4 | | IV-2 | Cxxc1 | 30827 | 4-May-15 |
| 5394 | 2 | 3 | 4 | | IV-2 | Cyb5d2 | 124936 | 4-May-15 |
| 5403 | 2 | 3 | 4 | | IV-2 | Cyc1 | 1537 | 7-Jun-15 |
| 5415 | 2 | 3 | 4 | | IV-2 | Cyp11b1 | 1584 | 12-May-15 |
| 5418 | 2 | 3 | 4 | | IV-2 | Cyp19a1 | 1588 | 21-May-15 |
| 5420 | 2 | 3 | 4 | | IV-2 | Cyp1a2 | 1544 | 24-May-15 |
| 5430 | 2 | 3 | 4 | | IV-2 | Cyp2a12 | | |
| 5434 | 2 | 3 | 4 | | IV-2 | Cyp2ab1 | | |
| 5446 | 2 | 3 | 4 | | IV-2 | Cyp2c50 | | |
| 5447 | 2 | 3 | 4 | | IV-2 | Cyp2c53-ps | | |
| 5456 | 2 | 3 | 4 | | IV-2 | Cyp2d10 | | |
| 5457 | 2 | 3 | 4 | | IV-2 | Cyp2d11 | | |
| 5459 | 2 | 3 | 4 | | IV-2 | Cyp2d13 | | |
| 5464 | 2 | 3 | 4 | | IV-2 | Cyp2d40 | | |
| 5472 | 2 | 3 | 4 | | IV-2 | Cyp2j5 | | |
| 5480 | 2 | 3 | 4 | | IV-2 | Cyp2w1 | 54905 | 4-May-15 |
| 5502 | 2 | 3 | 4 | | IV-2 | Cyp4f13 | | |
| 5504 | 2 | 3 | 4 | | IV-2 | Cyp4f15 | | |
| 5508 | 2 | 3 | 4 | | IV-2 | Cyp4f37 | | |
| 5513 | 2 | 3 | 4 | | IV-2 | Cyp4x1 | 260293 | 4-May-15 |
| 5518 | 2 | 3 | 4 | | IV-2 | Cypt1 | | |
| 5533 | 2 | 3 | 4 | | IV-2 | Cyth1 | 9267 | 12-May-15 |
| 5537 | 2 | 3 | 4 | | IV-2 | Cytip | 9595 | |
| 5540 | 2 | 3 | 4 | | IV-2 | D030018L15Rik | | |
| 5545 | 2 | 3 | 4 | | IV-2 | D030040B21Rik | | |
| 5555 | 2 | 3 | 4 | | IV-2 | D130020L05Rik | | |
| 5556 | 2 | 3 | 4 | | IV-2 | D130040H23Rik | | |
| 5558 | 2 | 3 | 4 | | IV-2 | D130058E03 | | |
| 5576 | 2 | 3 | 4 | | IV-2 | D330045A20Rik | | |
| 5583 | 2 | 3 | 4 | | IV-2 | D430020J02Rik | | |
| 5584 | 2 | 3 | 4 | | IV-2 | D430036J16Rik | | |
| 5593 | 2 | 3 | 4 | | IV-2 | D630010B17Rik | | |
| 5597 | 2 | 3 | 4 | | IV-2 | D630029K05Rik | | |
| 5608 | 2 | 3 | 4 | | IV-2 | D730005E14Rik | | |
| 5621 | 2 | 3 | 4 | | IV-2 | D830032E09Rik | | |
| 5648 | 2 | 3 | 4 | | IV-2 | Dak | 26007 | 4-May-15 |
| 5656 | 2 | 3 | 4 | | IV-2 | Dapk2 | 23604 | 21-May-15 |
| 5659 | 2 | 3 | 4 | | IV-2 | Dapp1 | 27071 | 12-May-15 |
| 5669 | 2 | 3 | 4 | | IV-2 | Dbhos | | |
| 5674 | 2 | 3 | 4 | | IV-2 | Dbndd2 | 55861 | 4-May-15 |
| 5677 | 2 | 3 | 4 | | IV-2 | Dbpht2 | | |
| 5700 | 2 | 3 | 4 | | IV-2 | Dcdc2b | 149069 | 4-May-15 |
| 5704 | 2 | 3 | 4 | | IV-2 | Dclk1 | 9201 | 21-May-15 |
| 5707 | 2 | 3 | 4 | | IV-2 | Dclre1a | 9937 | 12-May-15 |
| 5717 | 2 | 3 | 4 | | IV-2 | Dcps | 28960 | 4-May-15 |
| 5738 | 2 | 3 | 4 | | IV-2 | Ddah2 | 23564 | 31-May-15 |
| 5739 | 2 | 3 | 4 | | IV-2 | Ddb1 | 1642 | 4-May-15 |
| 5746 | 2 | 3 | 4 | | IV-2 | Ddit3 | 1649 | 17-May-15 |
| 5751 | 2 | 3 | 4 | | IV-2 | Ddost | 1650 | 23-May-15 |
| 5794 | 2 | 3 | 4 | | IV-2 | Deaf1 | 10522 | 4-May-15 |
| 5802 | 2 | 3 | 4 | | IV-2 | Def8 | 54849 | 4-May-15 |
| 5810 | 2 | 3 | 4 | | IV-2 | Defa25 | | |
| 5813 | 2 | 3 | 4 | | IV-2 | Defa4 | 1669 | 4-May-15 |
| 5839 | 2 | 3 | 4 | | IV-2 | Defb3 | | |
| 5861 | 2 | 3 | 4 | | IV-2 | Defb7 | 245910 | 4-May-15 |
| 5866 | 2 | 3 | 4 | | IV-2 | Dek | 7913 | 4-May-15 |
| 5884 | 2 | 3 | 4 | | IV-2 | Depdc5 | 9681 | 28-May-15 |
| 5892 | 2 | 3 | 4 | | IV-2 | Desi1 | 27351 | 7-Jun-15 |
| 5909 | 2 | 3 | 4 | | IV-2 | Dgkd | 8527 | 4-May-15 |
| 5920 | 2 | 3 | 4 | | IV-2 | Dhcr7 | 1717 | 23-May-15 |
| 5921 | 2 | 3 | 4 | | IV-2 | Dhdds | 79947 | 29-May-15 |
| 5925 | 2 | 3 | 4 | | IV-2 | Dhodh | 1723 | 4-May-15 |
| 5934 | 2 | 3 | 4 | | IV-2 | Dhrs7b | 25979 | 4-May-15 |
| 5936 | 2 | 3 | 4 | | IV-2 | Dhrs9 | 10170 | 4-May-15 |
| 5937 | 2 | 3 | 4 | | IV-2 | Dhrsx | 207063 | 4-May-15 |
| 5953 | 2 | 3 | 4 | | IV-2 | Dhx8 | 1659 | 12-May-15 |
| 5964 | 2 | 3 | 4 | | IV-2 | Dio2 | 1734 | 17-May-15 |
| 5965 | 2 | 3 | 4 | | IV-2 | Dio3 | 1735 | 12-May-15 |
| 5979 | 2 | 3 | 4 | | IV-2 | Dixdc1 | 85458 | 12-May-15 |

Fig.22 - 11

| | | | | | | | | |
|---|---|---|---|---|---|---|---|---|
| 5991 | 2 | 3 | 4 | | IV-2 | Dleu7 | 220107 | 12-May-15 |
| 5992 | 2 | 3 | 4 | | IV-2 | Dlg1 | 1739 | 12-May-15 |
| 6002 | 2 | 3 | 4 | | IV-2 | Dlk1 | 8788 | 31-May-15 |
| 6005 | 2 | 3 | 4 | | IV-2 | Dll3 | 10683 | 23-May-15 |
| 6010 | 2 | 3 | 4 | | IV-2 | Dlx2 | 1746 | 4-May-15 |
| 6011 | 2 | 3 | 4 | | IV-2 | Dlx3 | 1747 | 28-May-15 |
| 6012 | 2 | 3 | 4 | | IV-2 | Dlx4 | 1748 | 4-May-15 |
| 6013 | 2 | 3 | 4 | | IV-2 | Dlx5 | 1749 | 28-May-15 |
| 6019 | 2 | 3 | 4 | | IV-2 | Dmbx1 | 127343 | 4-May-15 |
| 6021 | 2 | 3 | 4 | | IV-2 | Dmd | 1756 | 24-May-15 |
| 6022 | 2 | 3 | 4 | | IV-2 | Dmgdh | 29958 | 4-May-15 |
| 6027 | 2 | 3 | 4 | | IV-2 | Dmrt1 | 1761 | 4-May-15 |
| 6039 | 2 | 3 | 4 | | IV-2 | Dmwd | 1762 | 4-May-15 |
| 6067 | 2 | 3 | 4 | | IV-2 | Dnajb14 | 79982 | 4-May-15 |
| 6090 | 2 | 3 | 4 | | IV-2 | Dnajc24 | 120526 | 4-May-15 |
| 6108 | 2 | 3 | 4 | | IV-2 | Dnase1l1 | 1774 | 12-May-15 |
| 6115 | 2 | 3 | 4 | | IV-2 | Dnlz | 728489 | 4-May-15 |
| 6130 | 2 | 3 | 4 | | IV-2 | Dnttip1 | 116092 | 4-May-15 |
| 6135 | 2 | 3 | 4 | | IV-2 | Dock1 | 1793 | 24-May-15 |
| 6137 | 2 | 3 | 4 | | IV-2 | Dock11 | 139818 | 12-May-15 |
| 6145 | 2 | 3 | 4 | | IV-2 | Dock9 | 23348 | 12-May-15 |
| 6150 | 2 | 3 | 4 | | IV-2 | Dok4 | 55715 | 3-May-15 |
| 6152 | 2 | 3 | 4 | | IV-2 | Dok6 | 220164 | 2-Jun-15 |
| 6167 | 2 | 3 | 4 | | IV-2 | Dpep3 | 64180 | 4-May-15 |
| 6171 | 2 | 3 | 4 | | IV-2 | Dph1 | 1801 | 4-May-15 |
| 6185 | 2 | 3 | 4 | | IV-2 | Dpp8 | 54878 | 21-May-15 |
| 6193 | 2 | 3 | 4 | | IV-2 | Dpy19l1 | 23333 | 4-May-15 |
| 6196 | 2 | 3 | 4 | | IV-2 | Dpy19l4 | 286148 | 4-May-15 |
| 6197 | 2 | 3 | 4 | | IV-2 | Dpy30 | 84661 | 24-May-15 |
| 6207 | 2 | 3 | 4 | | IV-2 | Dqx1 | 165545 | 4-May-15 |
| 6218 | 2 | 3 | 4 | | IV-2 | Drd5 | 1816 | 28-May-15 |
| 6230 | 2 | 3 | 4 | | IV-2 | Dscr3 | 10311 | 12-May-15 |
| 6243 | 2 | 3 | 4 | | IV-2 | Dstn | 11034 | 4-May-15 |
| 6259 | 2 | 3 | 4 | | IV-2 | Dtymk | 1841 | 4-May-15 |
| 6265 | 2 | 3 | 4 | | IV-2 | Dus1l | 64118 | 4-May-15 |
| 6271 | 2 | 3 | 4 | | IV-2 | Dusp11 | 8446 | 4-May-15 |
| 6280 | 2 | 3 | 4 | | IV-2 | Dusp21 | 63904 | 4-May-15 |
| 6293 | 2 | 3 | 4 | | IV-2 | Dut | 1854 | 12-May-15 |
| 6297 | 2 | 3 | 4 | | IV-2 | Duxbl3 | | |
| 6322 | 2 | 3 | 4 | | IV-2 | Dynlt3 | 6990 | 4-May-15 |
| 6340 | 2 | 3 | 4 | | IV-2 | E030019B06Rik | | |
| 6345 | 2 | 3 | 4 | | IV-2 | E030044B06Rik | | |
| 6352 | 2 | 3 | 4 | | IV-2 | E130114P18Rik | | |
| 6361 | 2 | 3 | 4 | | IV-2 | E130309F12Rik | | |
| 6363 | 2 | 3 | 4 | | IV-2 | E130311K13Rik | | |
| 6372 | 2 | 3 | 4 | | IV-2 | E2f2 | 1870 | 4-May-15 |
| 6373 | 2 | 3 | 4 | | IV-2 | E2f3 | 1871 | 17-May-15 |
| 6379 | 2 | 3 | 4 | | IV-2 | E330009J07Rik | | |
| 6380 | 2 | 3 | 4 | | IV-2 | E330011O21Rik | | |
| 6402 | 2 | 3 | 4 | | IV-2 | Ear14 | | |
| 6405 | 2 | 3 | 4 | | IV-2 | Ear4 | | |
| 6415 | 2 | 3 | 4 | | IV-2 | Ebna1bp2 | 10969 | 4-May-15 |
| 6424 | 2 | 3 | 4 | | IV-2 | Echdc2 | 55268 | 4-May-15 |
| 6433 | 2 | 3 | 4 | | IV-2 | Ecsit | 51295 | 4-May-15 |
| 6435 | 2 | 3 | 4 | | IV-2 | Ect2l | 345930 | 4-May-15 |
| 6439 | 2 | 3 | 4 | | IV-2 | Edaradd | 128178 | 23-May-15 |
| 6440 | 2 | 3 | 4 | | IV-2 | Edc3 | 80153 | 4-May-15 |
| 6443 | 2 | 3 | 4 | | IV-2 | Edem1 | 9695 | 23-May-15 |
| 6451 | 2 | 3 | 4 | | IV-2 | Ednra | 1909 | 31-May-15 |
| 6458 | 2 | 3 | 4 | | IV-2 | Eef1b2 | 1933 | 4-May-15 |
| 6469 | 2 | 3 | 4 | | IV-2 | Efcab12 | 90288 | 4-May-15 |
| 6475 | 2 | 3 | 4 | | IV-2 | Efcab5 | 374786 | 4-May-15 |
| 6478 | 2 | 3 | 4 | | IV-2 | Efcab8 | 388795 | 4-May-15 |
| 6483 | 2 | 3 | 4 | | IV-2 | Efhb | 151651 | 4-May-15 |
| 6487 | 2 | 3 | 4 | | IV-2 | Efhd2 | 79180 | 4-May-15 |
| 6491 | 2 | 3 | 4 | | IV-2 | Efna4 | 1945 | 31-May-15 |
| 6492 | 2 | 3 | 4 | | IV-2 | Efna5 | 1946 | 24-May-15 |
| 6498 | 2 | 3 | 4 | | IV-2 | Efs | 10278 | 4-May-15 |
| 6503 | 2 | 3 | 4 | | IV-2 | Egfem1 | | |
| 6505 | 2 | 3 | 4 | | IV-2 | Egfl7 | 51162 | 4-May-15 |
| 6509 | 2 | 3 | 4 | | IV-2 | Egln1 | 54583 | 12-May-15 |
| 6529 | 2 | 3 | 4 | | IV-2 | Eid2b | 126272 | 4-May-15 |
| 6562 | 2 | 3 | 4 | | IV-2 | Eif3k | 27335 | 12-May-15 |
| 6576 | 2 | 3 | 4 | | IV-2 | Eif4enif1 | 56478 | 4-May-15 |
| 6589 | 2 | 3 | 4 | | IV-2 | Elavl1 | 1994 | 2-Jun-15 |
| 6598 | 2 | 3 | 4 | | IV-2 | Elfn1 | 392617 | 4-May-15 |
| 6600 | 2 | 3 | 4 | | IV-2 | Elk1 | 2002 | 7-Jun-15 |
| 6603 | 2 | 3 | 4 | | IV-2 | Ell | 8178 | 4-May-15 |
| 6606 | 2 | 3 | 4 | | IV-2 | Elmo1 | 9844 | 24-May-15 |
| 6614 | 2 | 3 | 4 | | IV-2 | Elof1 | 84337 | 4-May-15 |
| 6621 | 2 | 3 | 4 | | IV-2 | Elovl7 | 79993 | 12-May-15 |
| 6641 | 2 | 3 | 4 | | IV-2 | Eme2 | 197342 | 4-May-15 |
| 6644 | 2 | 3 | 4 | | IV-2 | Emilin1 | 11117 | 4-May-15 |
| 6657 | 2 | 3 | 4 | | IV-2 | Emr4 | 326342 | 4-May-15 |
| 6666 | 2 | 3 | 4 | | IV-2 | Endod1 | 23052 | 4-May-15 |
| 6669 | 2 | 3 | 4 | | IV-2 | Endov | 284131 | 14-May-15 |
| 6676 | 2 | 3 | 4 | | IV-2 | Eno1b | | |
| 6679 | 2 | 3 | 4 | | IV-2 | Eno4 | 387712 | 4-May-15 |
| 6690 | 2 | 3 | 4 | | IV-2 | Enpp7 | 339221 | 4-May-15 |
| 6702 | 2 | 3 | 4 | | IV-2 | Eny2 | 56943 | 12-May-15 |
| 6716 | 2 | 3 | 4 | | IV-2 | Epc1 | 80314 | 7-Jun-15 |
| 6726 | 2 | 3 | 4 | | IV-2 | Epha4 | 2043 | 12-May-15 |
| 6733 | 2 | 3 | 4 | | IV-2 | Ephb3 | 2049 | 4-May-15 |
| 6738 | 2 | 3 | 4 | | IV-2 | Ephx3 | 79852 | 4-May-15 |
| 6739 | 2 | 3 | 4 | | IV-2 | Ephx4 | 253152 | 4-May-15 |
| 6741 | 2 | 3 | 4 | | IV-2 | Epm2aip1 | 9852 | 4-May-15 |
| 6754 | 2 | 3 | 4 | | IV-2 | Eps8l2 | 64787 | 4-May-15 |

| | | | | | | | | |
|---|---|---|---|---|---|---|---|---|
| 6760 | 2 | 3 | 4 | | IV-2 | Eqtn | 54586 | 4-May-15 |
| 6767 | 2 | 3 | 4 | | IV-2 | Erbb4 | 2066 | 23-May-15 |
| 6770 | 2 | 3 | 4 | | IV-2 | Ercc1 | 2067 | 23-May-15 |
| 6777 | 2 | 3 | 4 | | IV-2 | Ercc6l2 | 375748 | 4-May-15 |
| 6780 | 2 | 3 | 4 | | IV-2 | Ereg | 2069 | 4-May-15 |
| 6789 | 2 | 3 | 4 | | IV-2 | Eri3 | 79033 | 2-Jun-15 |
| 6800 | 2 | 3 | 4 | | IV-2 | Ermard | 55780 | 12-May-15 |
| 6804 | 2 | 3 | 4 | | IV-2 | Ern2 | 10595 | 12-May-15 |
| 6808 | 2 | 3 | 4 | | IV-2 | Erp29 | 10961 | 17-May-15 |
| 6813 | 2 | 3 | 4 | | IV-2 | Esco1 | 114799 | 4-May-15 |
| 6818 | 2 | 3 | 4 | | IV-2 | Esp1 | | |
| 6858 | 2 | 3 | 4 | | IV-2 | Etnppl | 64850 | 3-May-15 |
| 6876 | 2 | 3 | 4 | | IV-2 | Evi2a-evi2b | | |
| 6878 | 2 | 3 | 4 | | IV-2 | Evi5 | 7813 | 21-May-15 |
| 6881 | 2 | 3 | 4 | | IV-2 | Evpl | 2125 | 12-May-15 |
| 6888 | 2 | 3 | 4 | | IV-2 | Exo5 | 64789 | 4-May-15 |
| 6893 | 2 | 3 | 4 | | IV-2 | Exoc3l4 | 91828 | 4-May-15 |
| 6909 | 2 | 3 | 4 | | IV-2 | Exosc8 | 11340 | 4-May-15 |
| 6915 | 2 | 3 | 4 | | IV-2 | Extl2 | 2135 | 4-May-15 |
| 6926 | 2 | 3 | 4 | | IV-2 | F11r | 50848 | 4-May-15 |
| 6931 | 2 | 3 | 4 | | IV-2 | F2r | 2149 | 17-May-15 |
| 6948 | 2 | 3 | 4 | | IV-2 | F830045P16Rik | | |
| 6955 | 2 | 3 | 4 | | IV-2 | Fabp12 | 646486 | 4-May-15 |
| 6968 | 2 | 3 | 4 | | IV-2 | Faf1 | 11124 | 4-May-15 |
| 6977 | 2 | 3 | 4 | | IV-2 | Fam101b | 359845 | 4-May-15 |
| 6978 | 2 | 3 | 4 | | IV-2 | Fam102a | 399665 | 4-May-15 |
| 6987 | 2 | 3 | 4 | | IV-2 | Fam110a | 83541 | 4-May-15 |
| 6988 | 2 | 3 | 4 | | IV-2 | Fam110b | 90362 | 4-May-15 |
| 6992 | 2 | 3 | 4 | | IV-2 | Fam114a2 | 10827 | 4-May-15 |
| 7014 | 2 | 3 | 4 | | IV-2 | Fam131a | 131408 | 12-May-15 |
| 7019 | 2 | 3 | 4 | | IV-2 | Fam133b | 257415 | 4-May-15 |
| 7033 | 2 | 3 | 4 | | IV-2 | Fam151a | 338094 | 4-May-15 |
| 7034 | 2 | 3 | 4 | | IV-2 | Fam151b | 167555 | 4-May-15 |
| 7035 | 2 | 3 | 4 | | IV-2 | Fam154a | 158297 | 4-May-15 |
| 7049 | 2 | 3 | 4 | | IV-2 | Fam163b | 642968 | 4-May-15 |
| 7050 | 2 | 3 | 4 | | IV-2 | Fam166a | 401565 | 4-May-15 |
| 7063 | 2 | 3 | 4 | | IV-2 | Fam172a | 83989 | 12-May-15 |
| 7068 | 2 | 3 | 4 | | IV-2 | Fam175a | 84142 | 4-May-15 |
| 7072 | 2 | 3 | 4 | | IV-2 | Fam179a | 165186 | 4-May-15 |
| 7078 | 2 | 3 | 4 | | IV-2 | Fam184a | 79632 | 4-May-15 |
| 7095 | 2 | 3 | 4 | | IV-2 | Fam196b | 100131897 | 4-May-15 |
| 7114 | 2 | 3 | 4 | | IV-2 | Fam21 | | |
| 7118 | 2 | 3 | 4 | | IV-2 | Fam212b | 55924 | 4-May-15 |
| 7121 | 2 | 3 | 4 | | IV-2 | Fam214a | 56204 | 4-May-15 |
| 7122 | 2 | 3 | 4 | | IV-2 | Fam214b | 80256 | 4-May-15 |
| 7126 | 2 | 3 | 4 | | IV-2 | Fam217b | 63939 | 4-May-15 |
| 7127 | 2 | 3 | 4 | | IV-2 | Fam219a | 203259 | 4-May-15 |
| 7132 | 2 | 3 | 4 | | IV-2 | Fam221b | 392307 | 4-May-15 |
| 7143 | 2 | 3 | 4 | | IV-2 | Fam26d | 221301 | 12-May-15 |
| 7144 | 2 | 3 | 4 | | IV-2 | Fam26e | 254228 | 4-May-15 |
| 7146 | 2 | 3 | 4 | | IV-2 | Fam32a | 26017 | 4-May-15 |
| 7151 | 2 | 3 | 4 | | IV-2 | Fam43a | 131583 | 4-May-15 |
| 7157 | 2 | 3 | 4 | | IV-2 | Fam46d | 169966 | 4-May-15 |
| 7168 | 2 | 3 | 4 | | IV-2 | Fam57b | 83723 | 4-May-15 |
| 7169 | 2 | 3 | 4 | | IV-2 | Fam58b | 339521 | 4-May-15 |
| 7174 | 2 | 3 | 4 | | IV-2 | Fam65a | 79567 | 4-May-15 |
| 7176 | 2 | 3 | 4 | | IV-2 | Fam65c | 140876 | 12-May-15 |
| 7177 | 2 | 3 | 4 | | IV-2 | Fam69a | 388650 | 4-May-15 |
| 7179 | 2 | 3 | 4 | | IV-2 | Fam69c | 125704 | 4-May-15 |
| 7193 | 2 | 3 | 4 | | IV-2 | Fam78b | 149297 | 4-May-15 |
| 7201 | 2 | 3 | 4 | | IV-2 | Fam83g | 644815 | 4-May-15 |
| 7207 | 2 | 3 | 4 | | IV-2 | Fam89b | 23625 | 4-May-15 |
| 7212 | 2 | 3 | 4 | | IV-2 | Fam98a | 25940 | 4-May-15 |
| 7220 | 2 | 3 | 4 | | IV-2 | Fancd2os | 115795 | 4-May-15 |
| 7221 | 2 | 3 | 4 | | IV-2 | Fance | 2178 | 23-May-15 |
| 7226 | 2 | 3 | 4 | | IV-2 | Fancm | 57697 | 23-May-15 |
| 7239 | 2 | 3 | 4 | | IV-2 | Fastk | 10922 | 4-May-15 |
| 7258 | 2 | 3 | 4 | | IV-2 | Fbln7 | 129804 | 4-May-15 |
| 7259 | 2 | 3 | 4 | | IV-2 | Fbn1 | 2200 | 23-May-15 |
| 7260 | 2 | 3 | 4 | | IV-2 | Fbn2 | 2201 | 23-May-15 |
| 7263 | 2 | 3 | 4 | | IV-2 | Fbrs | 64319 | 12-May-15 |
| 7270 | 2 | 3 | 4 | | IV-2 | Fbxl16 | 146330 | 4-May-15 |
| 7273 | 2 | 3 | 4 | | IV-2 | Fbxl19 | 54620 | 4-May-15 |
| 7275 | 2 | 3 | 4 | | IV-2 | Fbxl20 | 84961 | 1-Jun-15 |
| 7308 | 2 | 3 | 4 | | IV-2 | Fbxo41 | 150726 | 4-May-15 |
| 7316 | 2 | 3 | 4 | | IV-2 | Fbxo5 | 26271 | 23-May-15 |
| 7317 | 2 | 3 | 4 | | IV-2 | Fbxo6 | 26270 | 4-May-15 |
| 7343 | 2 | 3 | 4 | | IV-2 | Fcer1a | 2205 | 12-May-15 |
| 7349 | 2 | 3 | 4 | | IV-2 | Fcgr2b | 2213 | 17-May-15 |
| 7352 | 2 | 3 | 4 | | IV-2 | Fcgrt | 2217 | 12-May-15 |
| 7358 | 2 | 3 | 4 | | IV-2 | Fcnb | | |
| 7360 | 2 | 3 | 4 | | IV-2 | Fcrl5 | 83416 | 4-May-15 |
| 7363 | 2 | 3 | 4 | | IV-2 | Fcrlb | 127943 | 4-May-15 |
| 7367 | 2 | 3 | 4 | | IV-2 | Fdx1 | 2230 | 12-May-15 |
| 7368 | 2 | 3 | 4 | | IV-2 | Fdx1l | 112812 | 4-May-15 |
| 7383 | 2 | 3 | 4 | | IV-2 | Fert2 | | |
| 7393 | 2 | 3 | 4 | | IV-2 | Ffar3 | 2865 | 4-May-15 |
| 7403 | 2 | 3 | 4 | | IV-2 | Fgf1 | 2246 | 12-May-15 |
| 7410 | 2 | 3 | 4 | | IV-2 | Fgf16 | 8823 | 4-May-15 |
| 7414 | 2 | 3 | 4 | | IV-2 | Fgf20 | 26281 | 4-May-15 |
| 7421 | 2 | 3 | 4 | | IV-2 | Fgf6 | 2251 | 4-May-15 |
| 7435 | 2 | 3 | 4 | | IV-2 | Fggy | 55277 | 4-May-15 |
| 7439 | 2 | 3 | 4 | | IV-2 | Fh1 | 2317 | 23-May-15 |
| 7445 | 2 | 3 | 4 | | IV-2 | Fhl2 | 2274 | 31-May-15 |
| 7449 | 2 | 3 | 4 | | IV-2 | Fhod1 | 29109 | 4-May-15 |
| 7460 | 2 | 3 | 4 | | IV-2 | Fignl2 | 401720 | 4-May-15 |
| 7462 | 2 | 3 | 4 | | IV-2 | Filip1l | 11259 | 4-May-15 |

Fig.22 - 12

| | | | | | | | | |
|---|---|---|---|---|---|---|---|---|
| 7467 | 2 | 3 | 4 | | IV-2 | Fitm2 | 128486 | 4-May-15 |
| 7472 | 2 | 3 | 4 | | IV-2 | Fkbp14 | 55033 | 4-May-15 |
| 7473 | 2 | 3 | 4 | | IV-2 | Fkbp15 | 23307 | 4-May-15 |
| 7495 | 2 | 3 | 4 | | IV-2 | Flot1 | 10211 | 31-May-15 |
| 7500 | 2 | 3 | 4 | | IV-2 | Flt1 | 2321 | 17-May-15 |
| 7506 | 2 | 3 | 4 | | IV-2 | Fmn1 | 342184 | 12-May-15 |
| 7509 | 2 | 3 | 4 | | IV-2 | Fmnl2 | 114793 | 4-May-15 |
| 7514 | 2 | 3 | 4 | | IV-2 | Fmo4 | 2329 | 4-May-15 |
| 7519 | 2 | 3 | 4 | | IV-2 | Fmr1 | 2332 | 7-Jun-15 |
| 7520 | 2 | 3 | 4 | | IV-2 | Fmr1nb | 158521 | 4-May-15 |
| 7529 | 2 | 3 | 4 | | IV-2 | Fndc3a | 22862 | 4-May-15 |
| 7537 | 2 | 3 | 4 | | IV-2 | Fnip1 | 96459 | 4-May-15 |
| 7541 | 2 | 3 | 4 | | IV-2 | Focad | 54914 | 4-May-15 |
| 7545 | 2 | 3 | 4 | | IV-2 | Folr4 | 390243 | 4-May-15 |
| 7549 | 2 | 3 | 4 | | IV-2 | Fosl1 | 8061 | 17-May-15 |
| 7564 | 2 | 3 | 4 | | IV-2 | Foxe3 | 2301 | 12-May-15 |
| 7565 | 2 | 3 | 4 | | IV-2 | Foxf1 | 2294 | 28-May-15 |
| 7581 | 2 | 3 | 4 | | IV-2 | Foxn1 | 8456 | 4-May-15 |
| 7584 | 2 | 3 | 4 | | IV-2 | Foxn4 | 121643 | 4-May-15 |
| 7587 | 2 | 3 | 4 | | IV-2 | Foxo4 | 4303 | 12-May-15 |
| 7589 | 2 | 3 | 4 | | IV-2 | Foxp1 | 27086 | 2-Jun-15 |
| 7594 | 2 | 3 | 4 | | IV-2 | Foxr1 | 283150 | 28-May-15 |
| 7611 | 2 | 3 | 4 | | IV-2 | Frem1 | 158326 | 23-May-15 |
| 7612 | 2 | 3 | 4 | | IV-2 | Frem2 | 341640 | 4-May-15 |
| 7635 | 2 | 3 | 4 | | IV-2 | Fsbp | 100861 412 | 4-May-15 |
| 7638 | 2 | 3 | 4 | | IV-2 | Fscn2 | 25794 | 23-May-15 |
| 7643 | 2 | 3 | 4 | | IV-2 | Fshb | 2488 | 12-May-15 |
| 7647 | 2 | 3 | 4 | | IV-2 | Fstl1 | 11167 | 4-May-15 |
| 7648 | 2 | 3 | 4 | | IV-2 | Fstl3 | 10272 | 4-May-15 |
| 7652 | 2 | 3 | 4 | | IV-2 | Fth1 | 2495 | 24-May-15 |
| 7672 | 2 | 3 | 4 | | IV-2 | Fut10 | 84750 | 4-May-15 |
| 7692 | 2 | 3 | 4 | | IV-2 | Fyb | 2533 | 12-May-15 |
| 7693 | 2 | 3 | 4 | | IV-2 | Fyco1 | 79443 | 21-May-15 |
| 7701 | 2 | 3 | 4 | | IV-2 | Fzd5 | 7855 | 12-May-15 |
| 7705 | 2 | 3 | 4 | | IV-2 | Fzd9 | 8326 | 4-May-15 |
| 7708 | 2 | 3 | 4 | | IV-2 | G2e3 | 55632 | 23-May-15 |
| 7709 | 2 | 3 | 4 | | IV-2 | G3bp1 | 10146 | 31-May-15 |
| 7716 | 2 | 3 | 4 | | IV-2 | G630093K05Rik | | |
| 7718 | 2 | 3 | 4 | | IV-2 | G6bos | | |
| 7720 | 2 | 3 | 4 | | IV-2 | G6pc2 | 57818 | 4-May-15 |
| 7746 | 2 | 3 | 4 | | IV-2 | Gabrd | 2563 | 4-May-15 |
| 7752 | 2 | 3 | 4 | | IV-2 | Gabrq | 55879 | 4-May-15 |
| 7764 | 2 | 3 | 4 | | IV-2 | Gak | 2580 | 31-May-15 |
| 7772 | 2 | 3 | 4 | | IV-2 | Galk1 | 2584 | 12-May-15 |
| 7784 | 2 | 3 | 4 | | IV-2 | Galnt18 | 374378 | 14-May-15 |
| 7801 | 2 | 3 | 4 | | IV-2 | Ganab | 23193 | 4-May-15 |
| 7804 | 2 | 3 | 4 | | IV-2 | Gapdh | 2597 | 7-Jun-15 |
| 7807 | 2 | 3 | 4 | | IV-2 | Gapvd1 | 26130 | 12-May-15 |
| 7816 | 2 | 3 | 4 | | IV-2 | Gas2l1 | 10634 | 4-May-15 |
| 7819 | 2 | 3 | 4 | | IV-2 | Gas5 | 60674 | 17-May-15 |
| 7825 | 2 | 3 | 4 | | IV-2 | Gata2 | 2624 | 28-May-15 |
| 7834 | 2 | 3 | 4 | | IV-2 | Gatc | 283459 | 23-May-15 |
| 7836 | 2 | 3 | 4 | | IV-2 | Gatsl2 | 729438 | 14-May-15 |
| 7838 | 2 | 3 | 4 | | IV-2 | Gba | 2629 | 31-May-15 |
| 7844 | 2 | 3 | 4 | | IV-2 | Gbp10 | | |
| 7846 | 2 | 3 | 4 | | IV-2 | Gbp2 | 2634 | 4-May-15 |
| 7849 | 2 | 3 | 4 | | IV-2 | Gbp4 | 115361 | 4-May-15 |
| 7850 | 2 | 3 | 4 | | IV-2 | Gbp5 | 115362 | 4-May-15 |
| 7851 | 2 | 3 | 4 | | IV-2 | Gbp6 | 163351 | 4-May-15 |
| 7852 | 2 | 3 | 4 | | IV-2 | Gbp7 | 388646 | 4-May-15 |
| 7854 | 2 | 3 | 4 | | IV-2 | Gbp9 | | |
| 7855 | 2 | 3 | 4 | | IV-2 | Gbx1 | 2636 | 4-May-15 |
| 7858 | 2 | 3 | 4 | | IV-2 | Gca | 25801 | 4-May-15 |
| 7868 | 2 | 3 | 4 | | IV-2 | Gck | 2645 | 7-Jun-15 |
| 7869 | 2 | 3 | 4 | | IV-2 | Gckr | 2646 | 7-Jun-15 |
| 7881 | 2 | 3 | 4 | | IV-2 | Gcsh | 2653 | 23-May-15 |
| 7885 | 2 | 3 | 4 | | IV-2 | Gdap1l1 | 78997 | 4-May-15 |
| 7890 | 2 | 3 | 4 | | IV-2 | Gdf11 | 10220 | 21-May-15 |
| 7892 | 2 | 3 | 4 | | IV-2 | Gdf2 | 2658 | 4-May-15 |
| 7896 | 2 | 3 | 4 | | IV-2 | Gdf7 | 151449 | 4-May-15 |
| 7898 | 2 | 3 | 4 | | IV-2 | Gdi1 | 2664 | 12-May-15 |
| 7915 | 2 | 3 | 4 | | IV-2 | Get4 | 51608 | 29-May-15 |
| 7920 | 2 | 3 | 4 | | IV-2 | Gfm1 | 85476 | 12-May-15 |
| 7928 | 2 | 3 | 4 | | IV-2 | Gfra3 | 2676 | 4-May-15 |
| 7936 | 2 | 3 | 4 | | IV-2 | Ggct | 79017 | 4-May-15 |
| 7937 | 2 | 3 | 4 | | IV-2 | Ggcx | 2677 | 31-May-15 |
| 7944 | 2 | 3 | 4 | | IV-2 | Ggt5 | 2687 | 4-May-15 |
| 7949 | 2 | 3 | 4 | | IV-2 | Ghdc | 84514 | 4-May-15 |
| 7955 | 2 | 3 | 4 | | IV-2 | Ghsr | 2693 | 12-May-15 |
| 7964 | 2 | 3 | 4 | | IV-2 | Gimap5 | 55340 | 4-May-15 |
| 7967 | 2 | 3 | 4 | | IV-2 | Gimap8 | 155038 | 4-May-15 |
| 7973 | 2 | 3 | 4 | | IV-2 | Gins3 | 64785 | 4-May-15 |
| 7985 | 2 | 3 | 4 | | IV-2 | Gja4 | 2701 | 12-May-15 |
| 7997 | 2 | 3 | 4 | | IV-2 | Gjc3 | 349149 | 20-May-15 |
| 7999 | 2 | 3 | 4 | | IV-2 | Gjd3 | 125111 | 4-May-15 |
| 8007 | 2 | 3 | 4 | | IV-2 | Gkn3 | | |
| 8008 | 2 | 3 | 4 | | IV-2 | Gla | 2717 | 7-Jun-15 |
| 8017 | 2 | 3 | 4 | | IV-2 | Gldnos | | |
| 8020 | 2 | 3 | 4 | | IV-2 | Gli1 | 2735 | 17-May-15 |
| 8021 | 2 | 3 | 4 | | IV-2 | Gli2 | 2736 | 23-May-15 |
| 8022 | 2 | 3 | 4 | | IV-2 | Gli3 | 2737 | 31-May-15 |
| 8024 | 2 | 3 | 4 | | IV-2 | Glipr1l1 | 256710 | 12-May-15 |
| 8027 | 2 | 3 | 4 | | IV-2 | Glis1 | 148979 | 4-May-15 |
| 8028 | 2 | 3 | 4 | | IV-2 | Glis2 | 84662 | 28-May-15 |
| 8030 | 2 | 3 | 4 | | IV-2 | Glmn | 11146 | 12-May-15 |
| 8054 | 2 | 3 | 4 | | IV-2 | Gltp | 51228 | 12-May-15 |
| 8066 | 2 | 3 | 4 | | IV-2 | Glyr1 | 84656 | 20-May-15 |

| | | | | | | | | |
|---|---|---|---|---|---|---|---|---|
| 8070 | 2 | 3 | 4 | | IV-2 | Gm10033 | | |
| 8072 | 2 | 3 | 4 | | IV-2 | Gm10052 | | |
| 8076 | 2 | 3 | 4 | | IV-2 | Gm10081 | | |
| 8081 | 2 | 3 | 4 | | IV-2 | Gm10104 | | |
| 8084 | 2 | 3 | 4 | | IV-2 | Gm10147 | | |
| 8089 | 2 | 3 | 4 | | IV-2 | Gm10230 | | |
| 8093 | 2 | 3 | 4 | | IV-2 | Gm10280 | | |
| 8095 | 2 | 3 | 4 | | IV-2 | Gm10319 | | |
| 8096 | 2 | 3 | 4 | | IV-2 | Gm10324 | | |
| 8098 | 2 | 3 | 4 | | IV-2 | Gm10336 | | |
| 8113 | 2 | 3 | 4 | | IV-2 | Gm10421 | | |
| 8127 | 2 | 3 | 4 | | IV-2 | Gm10488 | | |
| 8128 | 2 | 3 | 4 | | IV-2 | Gm10494 | | |
| 8133 | 2 | 3 | 4 | | IV-2 | Gm10532 | | |
| 8143 | 2 | 3 | 4 | | IV-2 | Gm10619 | | |
| 8159 | 2 | 3 | 4 | | IV-2 | Gm10684 | | |
| 8171 | 2 | 3 | 4 | | IV-2 | Gm10789 | | |
| 8179 | 2 | 3 | 4 | | IV-2 | Gm10857 | | |
| 8183 | 2 | 3 | 4 | | IV-2 | Gm10921 | | |
| 8189 | 2 | 3 | 4 | | IV-2 | Gm11128 | | |
| 8190 | 2 | 3 | 4 | | IV-2 | Gm11149 | | |
| 8241 | 2 | 3 | 4 | | IV-2 | Gm11944 | | |
| 8259 | 2 | 3 | 4 | | IV-2 | Gm12250 | | |
| 8268 | 2 | 3 | 4 | | IV-2 | Gm12505 | | |
| 8277 | 2 | 3 | 4 | | IV-2 | Gm12718 | | |
| 8278 | 2 | 3 | 4 | | IV-2 | Gm12789 | | |
| 8286 | 2 | 3 | 4 | | IV-2 | Gm12992 | | |
| 8289 | 2 | 3 | 4 | | IV-2 | Gm13023 | | |
| 8309 | 2 | 3 | 4 | | IV-2 | Gm13154 | | |
| 8314 | 2 | 3 | 4 | | IV-2 | Gm1322 | | |
| 8334 | 2 | 3 | 4 | | IV-2 | Gm13305 | | |
| 8336 | 2 | 3 | 4 | | IV-2 | Gm13308 | | |
| 8337 | 2 | 3 | 4 | | IV-2 | Gm13315 | | |
| 8339 | 2 | 3 | 4 | | IV-2 | Gm13375 | | |
| 8343 | 2 | 3 | 4 | | IV-2 | Gm13497 | | |
| 8354 | 2 | 3 | 4 | | IV-2 | Gm13749 | | |
| 8364 | 2 | 3 | 4 | | IV-2 | Gm14015 | | |
| 8368 | 2 | 3 | 4 | | IV-2 | Gm14092 | | |
| 8380 | 2 | 3 | 4 | | IV-2 | Gm14306 | | |
| 8400 | 2 | 3 | 4 | | IV-2 | Gm14440 | | |
| 8402 | 2 | 3 | 4 | | IV-2 | Gm14458 | | |
| 8420 | 2 | 3 | 4 | | IV-2 | Gm14625 | | |
| 8438 | 2 | 3 | 4 | | IV-2 | Gm15008 | | |
| 8442 | 2 | 3 | 4 | | IV-2 | Gm15091 | | |
| 8455 | 2 | 3 | 4 | | IV-2 | Gm15292 | | |
| 8456 | 2 | 3 | 4 | | IV-2 | Gm15293 | | |
| 8459 | 2 | 3 | 4 | | IV-2 | Gm15315 | | |
| 8463 | 2 | 3 | 4 | | IV-2 | Gm15350 | | |
| 8467 | 2 | 3 | 4 | | IV-2 | Gm15412 | | |
| 8472 | 2 | 3 | 4 | | IV-2 | Gm15446 | | |
| 8481 | 2 | 3 | 4 | | IV-2 | Gm15663 | | |
| 8498 | 2 | 3 | 4 | | IV-2 | Gm15941 | | |
| 8500 | 2 | 3 | 4 | | IV-2 | Gm15997 | | |
| 8527 | 2 | 3 | 4 | | IV-2 | Gm16515 | | |
| 8533 | 2 | 3 | 4 | | IV-2 | Gm16576 | | |
| 8544 | 2 | 3 | 4 | | IV-2 | Gm16740 | | |
| 8578 | 2 | 3 | 4 | | IV-2 | Gm17762 | | |
| 8593 | 2 | 3 | 4 | | IV-2 | Gm19424 | | |
| 8607 | 2 | 3 | 4 | | IV-2 | Gm1968 | | |
| 8617 | 2 | 3 | 4 | | IV-2 | Gm19897 | | |
| 8618 | 2 | 3 | 4 | | IV-2 | Gm1993 | | |
| 8621 | 2 | 3 | 4 | | IV-2 | Gm2002 | | |
| 8624 | 2 | 3 | 4 | | IV-2 | Gm2011 | | |
| 8625 | 2 | 3 | 4 | | IV-2 | Gm20110 | | |
| 8635 | 2 | 3 | 4 | | IV-2 | Gm20268 | | |
| 8640 | 2 | 3 | 4 | | IV-2 | Gm20324 | | |
| 8642 | 2 | 3 | 4 | | IV-2 | Gm20356 | | |
| 8650 | 2 | 3 | 4 | | IV-2 | Gm20605 | | |
| 8683 | 2 | 3 | 4 | | IV-2 | Gm20831 | | |
| 8696 | 2 | 3 | 4 | | IV-2 | Gm21057 | | |
| 8700 | 2 | 3 | 4 | | IV-2 | Gm21221 | | |
| 8710 | 2 | 3 | 4 | | IV-2 | Gm21541 | | |
| 8712 | 2 | 3 | 4 | | IV-2 | Gm21637 | | |
| 8731 | 2 | 3 | 4 | | IV-2 | Gm2696 | | |
| 8732 | 2 | 3 | 4 | | IV-2 | Gm2721 | | |
| 8747 | 2 | 3 | 4 | | IV-2 | Gm3139 | | |
| 8752 | 2 | 3 | 4 | | IV-2 | Gm3258 | | |
| 8755 | 2 | 3 | 4 | | IV-2 | Gm3279 | | |
| 8757 | 2 | 3 | 4 | | IV-2 | Gm3286 | | |
| 8766 | 2 | 3 | 4 | | IV-2 | Gm3417 | | |
| 8775 | 2 | 3 | 4 | | IV-2 | Gm362 | | |
| 8782 | 2 | 3 | 4 | | IV-2 | Gm3750 | | |
| 8789 | 2 | 3 | 4 | | IV-2 | Gm4027 | | |
| 8791 | 2 | 3 | 4 | | IV-2 | Gm41 | | |
| 8804 | 2 | 3 | 4 | | IV-2 | Gm4297 | | |
| 8812 | 2 | 3 | 4 | | IV-2 | Gm436 | | |
| 8813 | 2 | 3 | 4 | | IV-2 | Gm4371 | | |
| 8819 | 2 | 3 | 4 | | IV-2 | Gm4489 | | |
| 8853 | 2 | 3 | 4 | | IV-2 | Gm4894 | | |
| 8862 | 2 | 3 | 4 | | IV-2 | Gm4952 | | |
| 8864 | 2 | 3 | 4 | | IV-2 | Gm4961 | | |
| 8868 | 2 | 3 | 4 | | IV-2 | Gm4981 | | |
| 8874 | 2 | 3 | 4 | | IV-2 | Gm5071 | | |
| 8887 | 2 | 3 | 4 | | IV-2 | Gm5111 | | |
| 8897 | 2 | 3 | 4 | | IV-2 | Gm5136 | | |
| 8906 | 2 | 3 | 4 | | IV-2 | Gm5177 | | |
| 8908 | 2 | 3 | 4 | | IV-2 | Gm527 | | |

Fig.22 - 13

| | | | | | | | | |
|---|---|---|---|---|---|---|---|---|
| 8911 | 2 | 3 | 4 | | IV-2 | Gm5334 | | |
| 8921 | 2 | 3 | 4 | | IV-2 | Gm5431 | | |
| 8942 | 2 | 3 | 4 | | IV-2 | Gm5577 | | |
| 8952 | 2 | 3 | 4 | | IV-2 | Gm5634 | | |
| 8965 | 2 | 3 | 4 | | IV-2 | Gm5779 | | |
| 8966 | 2 | 3 | 4 | | IV-2 | Gm5795 | | |
| 8975 | 2 | 3 | 4 | | IV-2 | Gm5862 | | |
| 8983 | 2 | 3 | 4 | | IV-2 | Gm5901 | | |
| 8999 | 2 | 3 | 4 | | IV-2 | Gm6086 | | |
| 9018 | 2 | 3 | 4 | | IV-2 | Gm6313 | | |
| 9035 | 2 | 3 | 4 | | IV-2 | Gm6537 | | |
| 9041 | 2 | 3 | 4 | | IV-2 | Gm6583 | | |
| 9052 | 2 | 3 | 4 | | IV-2 | Gm6654 | | |
| 9090 | 2 | 3 | 4 | | IV-2 | Gm7257 | | |
| 9095 | 2 | 3 | 4 | | IV-2 | Gm7337 | | |
| 9104 | 2 | 3 | 4 | | IV-2 | Gm7616 | | |
| 9112 | 2 | 3 | 4 | | IV-2 | Gm7854 | | |
| 9114 | 2 | 3 | 4 | | IV-2 | Gm7903 | | |
| 9127 | 2 | 3 | 4 | | IV-2 | Gm8267 | | |
| 9134 | 2 | 3 | 4 | | IV-2 | Gm839 | | |
| 9137 | 2 | 3 | 4 | | IV-2 | Gm8579 | | |
| 9142 | 2 | 3 | 4 | | IV-2 | Gm8677 | | |
| 9146 | 2 | 3 | 4 | | IV-2 | Gm8773 | | |
| 9156 | 2 | 3 | 4 | | IV-2 | Gm8979 | | |
| 9158 | 2 | 3 | 4 | | IV-2 | Gm8994 | | |
| 9164 | 2 | 3 | 4 | | IV-2 | Gm9079 | | |
| 9169 | 2 | 3 | 4 | | IV-2 | Gm9268 | | |
| 9171 | 2 | 3 | 4 | | IV-2 | Gm9376 | | |
| 9184 | 2 | 3 | 4 | | IV-2 | Gm9839 | | |
| 9186 | 2 | 3 | 4 | | IV-2 | Gm9866 | | |
| 9189 | 2 | 3 | 4 | | IV-2 | Gm9899 | | |
| 9210 | 2 | 3 | 4 | | IV-2 | Gmppa | 29926 | 4-May-15 |
| 9212 | 2 | 3 | 4 | | IV-2 | Gmpr | 2766 | 12-May-15 |
| 9219 | 2 | 3 | 4 | | IV-2 | Gna15 | 2769 | 4-May-15 |
| 9225 | 2 | 3 | 4 | | IV-2 | Gnaq | 2776 | 4-May-15 |
| 9237 | 2 | 3 | 4 | | IV-2 | Gnb5 | 10681 | 4-May-15 |
| 9241 | 2 | 3 | 4 | | IV-2 | Gng12 | 55970 | 4-May-15 |
| 9251 | 2 | 3 | 4 | | IV-2 | Gnl1 | 2794 | 12-May-15 |
| 9256 | 2 | 3 | 4 | | IV-2 | Gnpat | 8443 | 12-May-15 |
| 9263 | 2 | 3 | 4 | | IV-2 | Gnrhr | 2798 | 23-May-15 |
| 9272 | 2 | 3 | 4 | | IV-2 | Golgb1 | 2804 | 4-May-15 |
| 9278 | 2 | 3 | 4 | | IV-2 | Golt1b | 51026 | 4-May-15 |
| 9287 | 2 | 3 | 4 | | IV-2 | Got1l1 | 137362 | 4-May-15 |
| 9290 | 2 | 3 | 4 | | IV-2 | Gp1bb | 2812 | 12-May-15 |
| 9294 | 2 | 3 | 4 | | IV-2 | Gp6 | 51206 | 4-May-15 |
| 9295 | 2 | 3 | 4 | | IV-2 | Gp9 | 2815 | 12-May-15 |
| 9296 | 2 | 3 | 4 | | IV-2 | Gpa33 | 10223 | 12-May-15 |
| 9300 | 2 | 3 | 4 | | IV-2 | Gpank1 | 7918 | 4-May-15 |
| 9324 | 2 | 3 | 4 | | IV-2 | Gphb5 | 122876 | 4-May-15 |
| 9328 | 2 | 3 | 4 | | IV-2 | Gpkow | 27238 | 4-May-15 |
| 9338 | 2 | 3 | 4 | | IV-2 | Gpr107 | 57720 | 12-May-15 |
| 9341 | 2 | 3 | 4 | | IV-2 | Gpr111 | 222611 | 4-May-15 |
| 9372 | 2 | 3 | 4 | | IV-2 | Gpr156 | 165829 | 12-May-15 |
| 9380 | 2 | 3 | 4 | | IV-2 | Gpr171 | 29909 | 4-May-15 |
| 9381 | 2 | 3 | 4 | | IV-2 | Gpr173 | 54328 | 12-May-15 |
| 9383 | 2 | 3 | 4 | | IV-2 | Gpr176 | 11245 | 4-May-15 |
| 9386 | 2 | 3 | 4 | | IV-2 | Gpr180 | 160897 | 31-May-15 |
| 9389 | 2 | 3 | 4 | | IV-2 | Gpr19 | 2842 | 4-May-15 |
| 9396 | 2 | 3 | 4 | | IV-2 | Gpr3 | 2827 | 4-May-15 |
| 9409 | 2 | 3 | 4 | | IV-2 | Gpr56 | 9289 | 23-May-15 |
| 9433 | 2 | 3 | 4 | | IV-2 | Gprc6a | 222545 | 4-May-15 |
| 9437 | 2 | 3 | 4 | | IV-2 | Gps1 | 2873 | 4-May-15 |
| 9446 | 2 | 3 | 4 | | IV-2 | Gpx2-ps1 | | |
| 9451 | 2 | 3 | 4 | | IV-2 | Gpx7 | 2882 | 4-May-15 |
| 9452 | 2 | 3 | 4 | | IV-2 | Gpx8 | 493869 | 12-May-15 |
| 9453 | 2 | 3 | 4 | | IV-2 | Gramd1a | 57655 | 12-May-15 |
| 9460 | 2 | 3 | 4 | | IV-2 | Grap2 | 9402 | 12-May-15 |
| 9461 | 2 | 3 | 4 | | IV-2 | Grasp | 160622 | 4-May-15 |
| 9464 | 2 | 3 | 4 | | IV-2 | Grb2 | 2885 | 31-May-15 |
| 9470 | 2 | 3 | 4 | | IV-2 | Grem2 | 64388 | 4-May-15 |
| 9474 | 2 | 3 | 4 | | IV-2 | Grhpr | 9380 | 31-May-15 |
| 9475 | 2 | 3 | 4 | | IV-2 | Gria1 | 2890 | 3-May-15 |
| 9478 | 2 | 3 | 4 | | IV-2 | Gria4 | 2893 | 12-May-15 |
| 9492 | 2 | 3 | 4 | | IV-2 | Grin2c | 2905 | 4-May-15 |
| 9494 | 2 | 3 | 4 | | IV-2 | Grin3a | 116443 | 4-May-15 |
| 9495 | 2 | 3 | 4 | | IV-2 | Grin3b | 116444 | 4-May-15 |
| 9521 | 2 | 3 | 4 | | IV-2 | Grwd1 | 83743 | 12-May-15 |
| 9534 | 2 | 3 | 4 | | IV-2 | Gsdmcl1 | | |
| 9536 | 2 | 3 | 4 | | IV-2 | Gsdmcl-ps | | |
| 9577 | 2 | 3 | 4 | | IV-2 | Gtf2a1l | 11036 | 4-May-15 |
| 9608 | 2 | 3 | 4 | | IV-2 | Gtsf1 | 121355 | 4-May-15 |
| 9611 | 2 | 3 | 4 | | IV-2 | Guca1b | 2979 | 31-May-15 |
| 9612 | 2 | 3 | 4 | | IV-2 | Guca2a | 2980 | 4-May-15 |
| 9613 | 2 | 3 | 4 | | IV-2 | Guca2b | 2981 | 4-May-15 |
| 9630 | 2 | 3 | 4 | | IV-2 | Gxylt1 | 283464 | 4-May-15 |
| 9637 | 2 | 3 | 4 | | IV-2 | Gypc | 2995 | 12-May-15 |
| 9640 | 2 | 3 | 4 | | IV-2 | Gzf1 | 64412 | 4-May-15 |
| 9653 | 2 | 3 | 4 | | IV-2 | H1f0 | 3005 | 4-May-15 |
| 9657 | 2 | 3 | 4 | | IV-2 | H2-Aa | | |
| 9658 | 2 | 3 | 4 | | IV-2 | H2-Ab1 | | |
| 9659 | 2 | 3 | 4 | | IV-2 | H2afb1 | 474382 | 4-May-15 |
| 9660 | 2 | 3 | 4 | | IV-2 | H2afb2 | 474381 | 4-May-15 |
| 9664 | 2 | 3 | 4 | | IV-2 | H2afx | 3014 | 4-May-15 |
| 9669 | 2 | 3 | 4 | | IV-2 | H2bfm | 286436 | 4-May-15 |
| 9671 | 2 | 3 | 4 | | IV-2 | H2-D1 | | |
| 9675 | 2 | 3 | 4 | | IV-2 | H2-Ea-ps | | |
| 9678 | 2 | 3 | 4 | | IV-2 | H2-K1 | | |
| 9692 | 2 | 3 | 4 | | IV-2 | H2-M3 | | |
| 9693 | 2 | 3 | 4 | | IV-2 | H2-M5 | | |
| 9696 | 2 | 3 | 4 | | IV-2 | H2-Ob | | |
| 9697 | 2 | 3 | 4 | | IV-2 | H2-Q1 | | |
| 9699 | 2 | 3 | 4 | | IV-2 | H2-Q2 | | |
| 9700 | 2 | 3 | 4 | | IV-2 | H2-Q4 | | |
| 9702 | 2 | 3 | 4 | | IV-2 | H2-Q6 | | |
| 9703 | 2 | 3 | 4 | | IV-2 | H2-Q7 | | |
| 9706 | 2 | 3 | 4 | | IV-2 | H2-T10 | | |
| 9708 | 2 | 3 | 4 | | IV-2 | H2-T23 | | |
| 9733 | 2 | 3 | 4 | | IV-2 | Hao2 | 51179 | 12-May-15 |
| 9739 | 2 | 3 | 4 | | IV-2 | Harbi1 | 283254 | 4-May-15 |
| 9744 | 2 | 3 | 4 | | IV-2 | Has2os | | |
| 9746 | 2 | 3 | 4 | | IV-2 | Hat1 | 8520 | 17-May-15 |
| 9754 | 2 | 3 | 4 | | IV-2 | Haus8 | 93323 | 4-May-15 |
| 9760 | 2 | 3 | 4 | | IV-2 | Hba-x | | |
| 9763 | 2 | 3 | 4 | | IV-2 | Hbb-bh2 | | |
| 9771 | 2 | 3 | 4 | | IV-2 | Hbs1l | 10767 | 4-May-15 |
| 9774 | 2 | 3 | 4 | | IV-2 | Hcar2 | 338442 | 4-May-15 |
| 9775 | 2 | 3 | 4 | | IV-2 | Hccs | 3052 | 23-May-15 |
| 9780 | 2 | 3 | 4 | | IV-2 | Hcls1 | 3059 | 12-May-15 |
| 9781 | 2 | 3 | 4 | | IV-2 | Hcn1 | 348980 | 4-May-15 |
| 9783 | 2 | 3 | 4 | | IV-2 | Hcn3 | 57657 | 4-May-15 |
| 9784 | 2 | 3 | 4 | | IV-2 | Hcn4 | 10021 | 23-May-15 |
| 9789 | 2 | 3 | 4 | | IV-2 | Hdac1 | 3065 | 28-May-15 |
| 9792 | 2 | 3 | 4 | | IV-2 | Hdac2 | 3066 | 28-May-15 |
| 9795 | 2 | 3 | 4 | | IV-2 | Hdac5 | 10014 | 31-May-15 |
| 9799 | 2 | 3 | 4 | | IV-2 | Hdac9 | 9734 | 4-May-15 |
| 9803 | 2 | 3 | 4 | | IV-2 | Hdgf | 3068 | 17-May-15 |
| 9810 | 2 | 3 | 4 | | IV-2 | Hdlbp | 3069 | 12-May-15 |
| 9821 | 2 | 3 | 4 | | IV-2 | Heca | 51696 | 4-May-15 |
| 9828 | 2 | 3 | 4 | | IV-2 | Helb | 92797 | 4-May-15 |
| 9830 | 2 | 3 | 4 | | IV-2 | Helq | 113510 | 4-May-15 |
| 9835 | 2 | 3 | 4 | | IV-2 | Hemk1 | 51409 | 4-May-15 |
| 9851 | 2 | 3 | 4 | | IV-2 | Hes3 | 390992 | 4-May-15 |
| 9866 | 2 | 3 | 4 | | IV-2 | Hfm1 | 164045 | 4-May-15 |
| 9874 | 2 | 3 | 4 | | IV-2 | Hhex | 3087 | 12-May-15 |
| 9875 | 2 | 3 | 4 | | IV-2 | Hhip | 64399 | 12-May-15 |
| 9876 | 2 | 3 | 4 | | IV-2 | Hhipl1 | 84439 | 14-May-15 |
| 9877 | 2 | 3 | 4 | | IV-2 | Hhipl2 | 79802 | 4-May-15 |
| 9887 | 2 | 3 | 4 | | IV-2 | Hif1an | 55662 | 4-May-15 |
| 9891 | 2 | 3 | 4 | | IV-2 | Higd1c | 613227 | 4-May-15 |
| 9903 | 2 | 3 | 4 | | IV-2 | Hipk3 | 10114 | 4-May-15 |
| 9908 | 2 | 3 | 4 | | IV-2 | Hist1h1b | 3009 | 4-May-15 |
| 9909 | 2 | 3 | 4 | | IV-2 | Hist1h1c | 3006 | 12-May-15 |
| 9910 | 2 | 3 | 4 | | IV-2 | Hist1h1d | 3007 | 4-May-15 |
| 9911 | 2 | 3 | 4 | | IV-2 | Hist1h1e | 3008 | 12-May-15 |
| 9912 | 2 | 3 | 4 | | IV-2 | Hist1h1t | 3010 | 4-May-15 |
| 9916 | 2 | 3 | 4 | | IV-2 | Hist1h2ad | 3013 | 4-May-15 |
| 9917 | 2 | 3 | 4 | | IV-2 | Hist1h2ae | 3012 | 14-May-15 |
| 9922 | 2 | 3 | 4 | | IV-2 | Hist1h2ak | 8330 | 4-May-15 |
| 9928 | 2 | 3 | 4 | | IV-2 | Hist1h2bc | 8347 | 12-May-15 |
| 9931 | 2 | 3 | 4 | | IV-2 | Hist1h2bg | 8339 | 4-May-15 |
| 9933 | 2 | 3 | 4 | | IV-2 | Hist1h2bj | 8970 | 4-May-15 |
| 9935 | 2 | 3 | 4 | | IV-2 | Hist1h2bl | 8340 | 4-May-15 |
| 9938 | 2 | 3 | 4 | | IV-2 | Hist1h2bp | | |
| 9939 | 2 | 3 | 4 | | IV-2 | Hist1h2bq | | |
| 9940 | 2 | 3 | 4 | | IV-2 | Hist1h3a | 8350 | 4-May-15 |
| 9941 | 2 | 3 | 4 | | IV-2 | Hist1h3b | 8358 | 4-May-15 |
| 9942 | 2 | 3 | 4 | | IV-2 | Hist1h3c | 8352 | 4-May-15 |
| 9944 | 2 | 3 | 4 | | IV-2 | Hist1h3e | 8353 | 4-May-15 |
| 9945 | 2 | 3 | 4 | | IV-2 | Hist1h3f | 8968 | 4-May-15 |
| 9948 | 2 | 3 | 4 | | IV-2 | Hist1h3i | 8354 | 4-May-15 |
| 9949 | 2 | 3 | 4 | | IV-2 | Hist1h4a | 8359 | 2-Jun-15 |
| 9950 | 2 | 3 | 4 | | IV-2 | Hist1h4b | 8366 | 4-May-15 |
| 9953 | 2 | 3 | 4 | | IV-2 | Hist1h4f | 8361 | 4-May-15 |
| 9957 | 2 | 3 | 4 | | IV-2 | Hist1h4k | 8362 | 4-May-15 |
| 9962 | 2 | 3 | 4 | | IV-2 | Hist2h2ab | 317772 | 4-May-15 |
| 9964 | 2 | 3 | 4 | | IV-2 | Hist2h2bb | 338391 | 4-May-15 |
| 9970 | 2 | 3 | 4 | | IV-2 | Hist3h2a | 92815 | 4-May-15 |
| 9972 | 2 | 3 | 4 | | IV-2 | Hist3h2bb-ps | | |
| 9974 | 2 | 3 | 4 | | IV-2 | Hivep1 | 3096 | 12-May-15 |
| 9976 | 2 | 3 | 4 | | IV-2 | Hivep3 | 59269 | 4-May-15 |
| 9982 | 2 | 3 | 4 | | IV-2 | Hkdc1 | 80201 | 4-May-15 |
| 9991 | 2 | 3 | 4 | | IV-2 | Hmg20a | 10363 | 12-May-15 |
| 9997 | 2 | 3 | 4 | | IV-2 | Hmgb1 | 3146 | 7-Jun-15 |
| 9998 | 2 | 3 | 4 | | IV-2 | Hmgb1-rs17 | | |
| 10007 | 2 | 3 | 4 | | IV-2 | Hmgn1 | 3150 | 4-May-15 |
| 10009 | 2 | 3 | 4 | | IV-2 | Hmgn3 | 9324 | 4-May-15 |
| 10010 | 2 | 3 | 4 | | IV-2 | Hmgn5 | 79366 | 4-May-15 |
| 10014 | 2 | 3 | 4 | | IV-2 | Hmmr | 3161 | 23-May-15 |
| 10030 | 2 | 3 | 4 | | IV-2 | Hnrnpa2b1 | 3181 | 31-May-15 |
| 10050 | 2 | 3 | 4 | | IV-2 | Homer2 | 9455 | 14-May-15 |
| 10071 | 2 | 3 | 4 | | IV-2 | Hoxa7 | 3204 | 28-May-15 |
| 10103 | 2 | 3 | 4 | | IV-2 | Hp1bp3 | 50809 | 12-May-15 |
| 10105 | 2 | 3 | 4 | | IV-2 | Hpcal1 | 3241 | 21-May-15 |
| 10107 | 2 | 3 | 4 | | IV-2 | Hpd | 3242 | 4-May-15 |
| 10110 | 2 | 3 | 4 | | IV-2 | Hpgds | 27306 | 4-May-15 |
| 10121 | 2 | 3 | 4 | | IV-2 | Hr | 55806 | 12-May-15 |
| 10122 | 2 | 3 | 4 | | IV-2 | Hras | 3265 | 2-Jun-15 |
| 10128 | 2 | 3 | 4 | | IV-2 | Hrh1 | 3269 | 4-May-15 |
| 10144 | 2 | 3 | 4 | | IV-2 | Hs6st1 | 9394 | 14-May-15 |
| 10146 | 2 | 3 | 4 | | IV-2 | Hs6st3 | 266722 | 4-May-15 |
| 10147 | 2 | 3 | 4 | | IV-2 | Hsbp1 | 3281 | 17-May-15 |
| 10149 | 2 | 3 | 4 | | IV-2 | Hscb | 150274 | 4-May-15 |
| 10151 | 2 | 3 | 4 | | IV-2 | Hsd11b2 | 3291 | 12-May-15 |
| 10152 | 2 | 3 | 4 | | IV-2 | Hsd17b1 | 3292 | 4-May-15 |

Fig.22 - 14

| | | | | | | | | |
|---|---|---|---|---|---|---|---|---|
| 10155 | 2 | 3 | 4 | | IV-2 | Hsd17b12 | 51144 | 4-May-15 |
| 10159 | 2 | 3 | 4 | | IV-2 | Hsd17b3 | 3293 | 4-May-15 |
| 10165 | 2 | 3 | 4 | | IV-2 | Hsd3b3 | | |
| 10166 | 2 | 3 | 4 | | IV-2 | Hsd3b4 | | |
| 10168 | 2 | 3 | 4 | | IV-2 | Hsd3b6 | | |
| 10169 | 2 | 3 | 4 | | IV-2 | Hsd3b7 | 80270 | 4-May-15 |
| 10170 | 2 | 3 | 4 | | IV-2 | Hsdl1 | 83693 | 4-May-15 |
| 10175 | 2 | 3 | 4 | | IV-2 | Hsf3 | | |
| 10180 | 2 | 3 | 4 | | IV-2 | Hsp90aa1 | 3320 | 17-May-15 |
| 10185 | 2 | 3 | 4 | | IV-2 | Hspa13 | 6782 | 4-May-15 |
| 10191 | 2 | 3 | 4 | | IV-2 | Hspa4 | 3308 | 7-Jun-15 |
| 10202 | 2 | 3 | 4 | | IV-2 | Hspb8 | 26353 | 23-May-15 |
| 10232 | 2 | 3 | 4 | | IV-2 | Hus1 | 3364 | 4-May-15 |
| 10237 | 2 | 3 | 4 | | IV-2 | Hyal2 | 8692 | 31-May-15 |
| 10244 | 2 | 3 | 4 | | IV-2 | Hykk | 123688 | 4-May-15 |
| 10251 | 2 | 3 | 4 | | IV-2 | I830077J02Rik | | |
| 10264 | 2 | 3 | 4 | | IV-2 | Icam5 | 7087 | 4-May-15 |
| 10281 | 2 | 3 | 4 | | IV-2 | Idi2 | 91734 | 12-May-15 |
| 10301 | 2 | 3 | 4 | | IV-2 | Ifi30 | 10437 | 4-May-15 |
| 10304 | 2 | 3 | 4 | | IV-2 | Ifi44l | 10964 | 4-May-15 |
| 10306 | 2 | 3 | 4 | | IV-2 | Ifih1 | 64135 | 17-May-15 |
| 10308 | 2 | 3 | 4 | | IV-2 | Ifit2 | 3433 | 12-May-15 |
| 10317 | 2 | 3 | 4 | | IV-2 | Ifltd1 | 160492 | 12-May-15 |
| 10364 | 2 | 3 | 4 | | IV-2 | Igf1 | 3479 | 24-May-15 |
| 10387 | 2 | 3 | 4 | | IV-2 | Igll1 | 3543 | 12-May-15 |
| 10390 | 2 | 3 | 4 | | IV-2 | Igsf10 | 285313 | 4-May-15 |
| 10391 | 2 | 3 | 4 | | IV-2 | Igsf11 | 152404 | 12-May-15 |
| 10399 | 2 | 3 | 4 | | IV-2 | Igsf9b | 22997 | 4-May-15 |
| 10402 | 2 | 3 | 4 | | IV-2 | Iigp1 | | |
| 10403 | 2 | 3 | 4 | | IV-2 | Ik | 3550 | 7-Jun-15 |
| 10410 | 2 | 3 | 4 | | IV-2 | Ikzf2 | 22807 | 10-May-15 |
| 10411 | 2 | 3 | 4 | | IV-2 | Ikzf3 | 22806 | 4-May-15 |
| 10412 | 2 | 3 | 4 | | IV-2 | Ikzf4 | 64375 | 4-May-15 |
| 10416 | 2 | 3 | 4 | | IV-2 | Il10rb | 3588 | 12-May-15 |
| 10430 | 2 | 3 | 4 | | IV-2 | Il17a | 3605 | 7-Jun-15 |
| 10441 | 2 | 3 | 4 | | IV-2 | Il18bp | 10068 | 3-May-15 |
| 10447 | 2 | 3 | 4 | | IV-2 | Il1bos | | |
| 10467 | 2 | 3 | 4 | | IV-2 | Il22 | 50616 | 7-Jun-15 |
| 10470 | 2 | 3 | 4 | | IV-2 | Il23a | 51561 | 17-May-15 |
| 10479 | 2 | 3 | 4 | | IV-2 | Il3 | 3562 | 12-May-15 |
| 10483 | 2 | 3 | 4 | | IV-2 | Il34 | 146433 | 4-May-15 |
| 10490 | 2 | 3 | 4 | | IV-2 | Il6 | 3569 | 24-May-15 |
| 10512 | 2 | 3 | 4 | | IV-2 | Impact | 55364 | 12-May-15 |
| 10522 | 2 | 3 | 4 | | IV-2 | Inf2 | 64423 | 4-May-15 |
| 10531 | 2 | 3 | 4 | | IV-2 | Inhbc | 3626 | 4-May-15 |
| 10533 | 2 | 3 | 4 | | IV-2 | Inip | 58493 | 4-May-15 |
| 10535 | 2 | 3 | 4 | | IV-2 | Ino80 | 54617 | 4-May-15 |
| 10547 | 2 | 3 | 4 | | IV-2 | Inpp5e | 56623 | 23-May-15 |
| 10550 | 2 | 3 | 4 | | IV-2 | Inpp5k | 51763 | 4-May-15 |
| 10553 | 2 | 3 | 4 | | IV-2 | Ins2 | | |
| 10556 | 2 | 3 | 4 | | IV-2 | Insig2 | 51141 | 4-May-15 |
| 10560 | 2 | 3 | 4 | | IV-2 | Insm1 | 3642 | 4-May-15 |
| 10562 | 2 | 3 | 4 | | IV-2 | Insr | 3643 | 17-May-15 |
| 10603 | 2 | 3 | 4 | | IV-2 | Iqch | 64799 | 7-Jun-15 |
| 10609 | 2 | 3 | 4 | | IV-2 | Iqsec1 | 9922 | 12-May-15 |
| 10615 | 2 | 3 | 4 | | IV-2 | Irak2 | 3656 | 4-May-15 |
| 10626 | 2 | 3 | 4 | | IV-2 | Irf5 | 3663 | 24-May-15 |
| 10629 | 2 | 3 | 4 | | IV-2 | Irf8 | 3394 | 24-May-15 |
| 10638 | 2 | 3 | 4 | | IV-2 | Irs3 | | |
| 10655 | 2 | 3 | 4 | | IV-2 | Islr2 | 57611 | 4-May-15 |
| 10661 | 2 | 3 | 4 | | IV-2 | Ispd | 729920 | 7-Jun-15 |
| 10680 | 2 | 3 | 4 | | IV-2 | Itga8 | 8516 | 4-May-15 |
| 10688 | 2 | 3 | 4 | | IV-2 | Itgb1 | 3688 | 31-May-15 |
| 10692 | 2 | 3 | 4 | | IV-2 | Itgb2l | | |
| 10698 | 2 | 3 | 4 | | IV-2 | Itgb7 | 3695 | 12-May-15 |
| 10701 | 2 | 3 | 4 | | IV-2 | Itih1 | 3697 | 4-May-15 |
| 10703 | 2 | 3 | 4 | | IV-2 | Itih3 | 3699 | 4-May-15 |
| 10706 | 2 | 3 | 4 | | IV-2 | Itk | 3702 | 24-May-15 |
| 10708 | 2 | 3 | 4 | | IV-2 | Itm2a | 9452 | 4-May-15 |
| 10709 | 2 | 3 | 4 | | IV-2 | Itm2b | 9445 | 3-May-15 |
| 10743 | 2 | 3 | 4 | | IV-2 | Jakmip2 | 9832 | 4-May-15 |
| 10762 | 2 | 3 | 4 | | IV-2 | Jph3 | 57338 | 23-May-15 |
| 10764 | 2 | 3 | 4 | | IV-2 | Jpx | 554203 | 22-May-15 |
| 10768 | 2 | 3 | 4 | | IV-2 | Jtb | 10899 | 4-May-15 |
| 10784 | 2 | 3 | 4 | | IV-2 | Kars | 3735 | 4-May-15 |
| 10796 | 2 | 3 | 4 | | IV-2 | Katnbl1 | 79768 | 4-May-15 |
| 10798 | 2 | 3 | 4 | | IV-2 | Kazn | 23254 | 2-Jun-15 |
| 10812 | 2 | 3 | 4 | | IV-2 | Kcna4 | 3739 | 12-May-15 |
| 10816 | 2 | 3 | 4 | | IV-2 | Kcnab1 | 7881 | 12-May-15 |
| 10820 | 2 | 3 | 4 | | IV-2 | Kcnb2 | 9312 | 12-May-15 |
| 10822 | 2 | 3 | 4 | | IV-2 | Kcnc2 | 3747 | 31-May-15 |
| 10828 | 2 | 3 | 4 | | IV-2 | Kcnd3os | | |
| 10830 | 2 | 3 | 4 | | IV-2 | Kcne1l | 23630 | 4-May-15 |
| 10831 | 2 | 3 | 4 | | IV-2 | Kcne2 | 9992 | 23-May-15 |
| 10833 | 2 | 3 | 4 | | IV-2 | Kcne4 | 23704 | 4-May-15 |
| 10836 | 2 | 3 | 4 | | IV-2 | Kcng2 | 26251 | 12-May-15 |
| 10840 | 2 | 3 | 4 | | IV-2 | Kcnh2 | 3757 | 23-May-15 |
| 10846 | 2 | 3 | 4 | | IV-2 | Kcnh8 | 131096 | 4-May-15 |
| 10856 | 2 | 3 | 4 | | IV-2 | Kcnj14 | 3770 | 4-May-15 |
| 10860 | 2 | 3 | 4 | | IV-2 | Kcnj3 | 3760 | 4-May-15 |
| 10861 | 2 | 3 | 4 | | IV-2 | Kcnj4 | 3761 | 4-May-15 |
| 10863 | 2 | 3 | 4 | | IV-2 | Kcnj6 | 3763 | 12-May-15 |
| 10865 | 2 | 3 | 4 | | IV-2 | Kcnj9 | 3765 | 12-May-15 |
| 10868 | 2 | 3 | 4 | | IV-2 | Kcnk12 | 56660 | 4-May-15 |
| 10872 | 2 | 3 | 4 | | IV-2 | Kcnk18 | 338567 | 4-May-15 |
| 10875 | 2 | 3 | 4 | | IV-2 | Kcnk4 | 50801 | 12-May-15 |
| 10880 | 2 | 3 | 4 | | IV-2 | Kcnma1 | 3778 | 14-May-15 |

| | | | | | | | | |
|---|---|---|---|---|---|---|---|---|
| 10885 | 2 | 3 | 4 | | IV-2 | Kcnmb4os1 | | |
| 10891 | 2 | 3 | 4 | | IV-2 | Kcnq1ot1 | 10984 | 22-May-15 |
| 10893 | 2 | 3 | 4 | | IV-2 | Kcnq3 | 3786 | 23-May-15 |
| 10898 | 2 | 3 | 4 | | IV-2 | Kcns2 | 3788 | 4-May-15 |
| 10916 | 2 | 3 | 4 | | IV-2 | Kctd18 | 130535 | 4-May-15 |
| 10934 | 2 | 3 | 4 | | IV-2 | Kdm1a | 23028 | 10-May-15 |
| 10953 | 2 | 3 | 4 | | IV-2 | Kdsr | 2531 | 4-May-15 |
| 10957 | 2 | 3 | 4 | | IV-2 | Kera | 11081 | 4-May-15 |
| 10970 | 2 | 3 | 4 | | IV-2 | Kif12 | 113220 | 4-May-15 |
| 10973 | 2 | 3 | 4 | | IV-2 | Kif14 | 9928 | 17-May-15 |
| 10974 | 2 | 3 | 4 | | IV-2 | Kif15 | 56992 | 17-May-15 |
| 10975 | 2 | 3 | 4 | | IV-2 | Kif16b | 55614 | 4-May-15 |
| 10978 | 2 | 3 | 4 | | IV-2 | Kif18b | 146909 | 4-May-15 |
| 10979 | 2 | 3 | 4 | | IV-2 | Kif19a | 124602 | 4-May-15 |
| 10985 | 2 | 3 | 4 | | IV-2 | Kif21a | 55605 | 23-May-15 |
| 10987 | 2 | 3 | 4 | | IV-2 | Kif22 | 3835 | 4-May-15 |
| 10988 | 2 | 3 | 4 | | IV-2 | Kif23 | 9493 | 4-May-15 |
| 10989 | 2 | 3 | 4 | | IV-2 | Kif24 | 347240 | 4-May-15 |
| 10996 | 2 | 3 | 4 | | IV-2 | Kif3a | 11127 | 4-May-15 |
| 11000 | 2 | 3 | 4 | | IV-2 | Kif4-ps | | |
| 11002 | 2 | 3 | 4 | | IV-2 | Kif5b | 3799 | 4-May-15 |
| 11009 | 2 | 3 | 4 | | IV-2 | Kifc2 | 90990 | 4-May-15 |
| 11020 | 2 | 3 | 4 | | IV-2 | Kiss1r | 84634 | 7-Jun-15 |
| 11022 | 2 | 3 | 4 | | IV-2 | Kitl | 4254 | 24-May-15 |
| 11031 | 2 | 3 | 4 | | IV-2 | Klf10 | 7071 | 3-May-15 |
| 11033 | 2 | 3 | 4 | | IV-2 | Klf12 | 11278 | 2-Jun-15 |
| 11034 | 2 | 3 | 4 | | IV-2 | Klf13 | 51621 | 4-May-15 |
| 11043 | 2 | 3 | 4 | | IV-2 | Klf6 | 1316 | 4-May-15 |
| 11045 | 2 | 3 | 4 | | IV-2 | Klf8 | 11279 | 4-May-15 |
| 11053 | 2 | 3 | 4 | | IV-2 | Klhdc7b | 113730 | 4-May-15 |
| 11055 | 2 | 3 | 4 | | IV-2 | Klhdc8b | 200942 | 4-May-15 |
| 11056 | 2 | 3 | 4 | | IV-2 | Klhdc9 | 126823 | 21-May-15 |
| 11060 | 2 | 3 | 4 | | IV-2 | Klhl12 | 59349 | 4-May-15 |
| 11064 | 2 | 3 | 4 | | IV-2 | Klhl17 | 339451 | 4-May-15 |
| 11079 | 2 | 3 | 4 | | IV-2 | Klhl32 | 114792 | 4-May-15 |
| 11082 | 2 | 3 | 4 | | IV-2 | Klhl35 | 283212 | 7-Jun-15 |
| 11085 | 2 | 3 | 4 | | IV-2 | Klhl4 | 56062 | 4-May-15 |
| 11088 | 2 | 3 | 4 | | IV-2 | Klhl42 | 57542 | 4-May-15 |
| 11091 | 2 | 3 | 4 | | IV-2 | Klhl7 | 55975 | 23-May-15 |
| 11102 | 2 | 3 | 4 | | IV-2 | Klk1b11 | | |
| 11103 | 2 | 3 | 4 | | IV-2 | Klk1b16 | | |
| 11105 | 2 | 3 | 4 | | IV-2 | Klk1b22 | | |
| 11112 | 2 | 3 | 4 | | IV-2 | Klk1b7-ps | | |
| 11115 | 2 | 3 | 4 | | IV-2 | Klk4 | 9622 | 7-Jun-15 |
| 11124 | 2 | 3 | 4 | | IV-2 | Klra12 | | |
| 11126 | 2 | 3 | 4 | | IV-2 | Klra14-ps | | |
| 11130 | 2 | 3 | 4 | | IV-2 | Klra19 | | |
| 11132 | 2 | 3 | 4 | | IV-2 | Klra21 | | |
| 11135 | 2 | 3 | 4 | | IV-2 | Klra3 | | |
| 11136 | 2 | 3 | 4 | | IV-2 | Klra33 | | |
| 11143 | 2 | 3 | 4 | | IV-2 | Klrb1 | 3820 | 4-May-15 |
| 11153 | 2 | 3 | 4 | | IV-2 | Klre1 | | |
| 11154 | 2 | 3 | 4 | | IV-2 | Klrg1 | 10219 | 4-May-15 |
| 11156 | 2 | 3 | 4 | | IV-2 | Klri1 | | |
| 11160 | 2 | 3 | 4 | | IV-2 | Kmt2a | 4297 | 31-May-15 |
| 11171 | 2 | 3 | 4 | | IV-2 | Kntc1 | 9735 | 14-May-15 |
| 11172 | 2 | 3 | 4 | | IV-2 | Kpna1 | 3836 | 12-May-15 |
| 11207 | 2 | 3 | 4 | | IV-2 | Krt28 | 162605 | 4-May-15 |
| 11208 | 2 | 3 | 4 | | IV-2 | Krt31 | 3881 | 4-May-15 |
| 11209 | 2 | 3 | 4 | | IV-2 | Krt32 | 3882 | 4-May-15 |
| 11211 | 2 | 3 | 4 | | IV-2 | Krt33b | 3884 | 12-May-15 |
| 11213 | 2 | 3 | 4 | | IV-2 | Krt35 | 3886 | 4-May-15 |
| 11214 | 2 | 3 | 4 | | IV-2 | Krt36 | 8689 | 4-May-15 |
| 11225 | 2 | 3 | 4 | | IV-2 | Krt73 | 319101 | 28-May-15 |
| 11235 | 2 | 3 | 4 | | IV-2 | Krt82 | 3888 | 4-May-15 |
| 11236 | 2 | 3 | 4 | | IV-2 | Krt83 | 3889 | 4-May-15 |
| 11237 | 2 | 3 | 4 | | IV-2 | Krt84 | 3890 | 4-May-15 |
| 11240 | 2 | 3 | 4 | | IV-2 | Krt9 | 3857 | 4-May-15 |
| 11241 | 2 | 3 | 4 | | IV-2 | Krtap10-10 | 353333 | 4-May-15 |
| 11242 | 2 | 3 | 4 | | IV-2 | Krtap10-4 | 386672 | 4-May-15 |
| 11244 | 2 | 3 | 4 | | IV-2 | Krtap12-1 | 353332 | 4-May-15 |
| 11245 | 2 | 3 | 4 | | IV-2 | Krtap13 | 81850 | 4-May-15 |
| 11253 | 2 | 3 | 4 | | IV-2 | Krtap16-3 | | |
| 11254 | 2 | 3 | 4 | | IV-2 | Krtap17-1 | 83902 | 4-May-15 |
| 11255 | 2 | 3 | 4 | | IV-2 | Krtap19-1 | 337882 | 4-May-15 |
| 11257 | 2 | 3 | 4 | | IV-2 | Krtap19-4 | 337971 | 4-May-15 |
| 11258 | 2 | 3 | 4 | | IV-2 | Krtap19-5 | 337972 | 12-May-15 |
| 11260 | 2 | 3 | 4 | | IV-2 | Krtap20-2 | 337976 | 4-May-15 |
| 11261 | 2 | 3 | 4 | | IV-2 | Krtap21-1 | 337977 | 4-May-15 |
| 11264 | 2 | 3 | 4 | | IV-2 | Krtap24-1 | 643803 | 4-May-15 |
| 11268 | 2 | 3 | 4 | | IV-2 | Krtap31-1 | | |
| 11281 | 2 | 3 | 4 | | IV-2 | Krtap5-2 | 440021 | 4-May-15 |
| 11282 | 2 | 3 | 4 | | IV-2 | Krtap5-3 | 387266 | 4-May-15 |
| 11284 | 2 | 3 | 4 | | IV-2 | Krtap5-5 | 439915 | 4-May-15 |
| 11285 | 2 | 3 | 4 | | IV-2 | Krtap6-1 | 337966 | 4-May-15 |
| 11292 | 2 | 3 | 4 | | IV-2 | Krtap9-5 | 81870 | 4-May-15 |
| 11293 | 2 | 3 | 4 | | IV-2 | Krtcap2 | 200185 | 4-May-15 |
| 11307 | 2 | 3 | 4 | | IV-2 | L3mbtl1 | 26013 | 4-May-15 |
| 11311 | 2 | 3 | 4 | | IV-2 | l7Rn6 | 51501 | 4-May-15 |
| 11319 | 2 | 3 | 4 | | IV-2 | Lage3 | 8270 | 4-May-15 |
| 11324 | 2 | 3 | 4 | | IV-2 | Lama3 | 3909 | 7-Jun-15 |
| 11333 | 2 | 3 | 4 | | IV-2 | Lamp1 | 3916 | 29-May-15 |
| 11350 | 2 | 3 | 4 | | IV-2 | Large | 9215 | 23-May-15 |
| 11359 | 2 | 3 | 4 | | IV-2 | Las1l | 81887 | 4-May-15 |
| 11363 | 2 | 3 | 4 | | IV-2 | Lats1 | 9113 | 24-May-15 |
| 11366 | 2 | 3 | 4 | | IV-2 | Layn | 143903 | 4-May-15 |
| 11397 | 2 | 3 | 4 | | IV-2 | Lclat1 | 253558 | 12-May-15 |

Fig.22 - 15

| | | | | | | | | |
|---|---|---|---|---|---|---|---|---|
| 11404 | 2 | 3 | 4 | | IV-2 | Lcn3 | | |
| 11412 | 2 | 3 | 4 | | IV-2 | Lcp1 | 3936 | 7-Jun-15 |
| 11413 | 2 | 3 | 4 | | IV-2 | Lcp2 | 3937 | 4-May-15 |
| 11415 | 2 | 3 | 4 | | IV-2 | Lctl | 197021 | 4-May-15 |
| 11416 | 2 | 3 | 4 | | IV-2 | Ldb1 | 8861 | 7-Jun-15 |
| 11419 | 2 | 3 | 4 | | IV-2 | Ldha | 3939 | 17-May-15 |
| 11429 | 2 | 3 | 4 | | IV-2 | Ldlrap1 | 26119 | 12-May-15 |
| 11434 | 2 | 3 | 4 | | IV-2 | Lect2 | 3950 | 17-May-15 |
| 11460 | 2 | 3 | 4 | | IV-2 | Lgals1 | 3956 | 24-May-15 |
| 11464 | 2 | 3 | 4 | | IV-2 | Lgals3bp | 3959 | 12-May-15 |
| 11485 | 2 | 3 | 4 | | IV-2 | Lhfpl3 | 375612 | 4-May-15 |
| 11488 | 2 | 3 | 4 | | IV-2 | Lhpp | 64077 | 4-May-15 |
| 11500 | 2 | 3 | 4 | | IV-2 | Lifr | 3977 | 12-May-15 |
| 11501 | 2 | 3 | 4 | | IV-2 | Lig1 | 3978 | 7-Jun-15 |
| 11505 | 2 | 3 | 4 | | IV-2 | Lilra6 | 79168 | 20-May-15 |
| 11512 | 2 | 3 | 4 | | IV-2 | Lime1 | 54923 | 4-May-15 |
| 11524 | 2 | 3 | 4 | | IV-2 | Lin7c | 55327 | 4-May-15 |
| 11543 | 2 | 3 | 4 | | IV-2 | Lipt1 | 51601 | 13-May-15 |
| 11554 | 2 | 3 | 4 | | IV-2 | Lman2 | 10960 | 4-May-15 |
| 11564 | 2 | 3 | 4 | | IV-2 | Lmna | 4000 | 24-May-15 |
| 11566 | 2 | 3 | 4 | | IV-2 | Lmnb2 | 84823 | 4-May-15 |
| 11575 | 2 | 3 | 4 | | IV-2 | Lmtk2 | 22853 | 12-May-15 |
| 11584 | 2 | 3 | 4 | | IV-2 | LOC100040786 | | |
| 11587 | 2 | 3 | 4 | | IV-2 | LOC100502896 | | |
| 11594 | 2 | 3 | 4 | | IV-2 | LOC100505025 | | |
| 11597 | 2 | 3 | 4 | | IV-2 | LOC100862015 | | |
| 11609 | 2 | 3 | 4 | | IV-2 | LOC102632423 | | |
| 11627 | 2 | 3 | 4 | | IV-2 | Lonrf2 | 164832 | 12-May-15 |
| 11631 | 2 | 3 | 4 | | IV-2 | Loxhd1 | 125336 | 23-May-15 |
| 11634 | 2 | 3 | 4 | | IV-2 | Loxl3 | 84695 | 23-May-15 |
| 11636 | 2 | 3 | 4 | | IV-2 | Lpar1 | 1902 | 4-May-15 |
| 11639 | 2 | 3 | 4 | | IV-2 | Lpar4 | 2846 | 4-May-15 |
| 11648 | 2 | 3 | 4 | | IV-2 | Lphn1 | 22859 | 4-May-15 |
| 11652 | 2 | 3 | 4 | | IV-2 | Lpin2 | 9663 | 23-May-15 |
| 11656 | 2 | 3 | 4 | | IV-2 | Lpp | 4026 | 12-May-15 |
| 11658 | 2 | 3 | 4 | | IV-2 | Lrat | 9227 | 23-May-15 |
| 11679 | 2 | 3 | 4 | | IV-2 | Lrmp | 4033 | 4-May-15 |
| 11695 | 2 | 3 | 4 | | IV-2 | Lrrc1 | 55227 | 4-May-15 |
| 11697 | 2 | 3 | 4 | | IV-2 | Lrrc10b | 390205 | 4-May-15 |
| 11700 | 2 | 3 | 4 | | IV-2 | Lrrc15 | 131578 | 4-May-15 |
| 11707 | 2 | 3 | 4 | | IV-2 | Lrrc20 | 55222 | 4-May-15 |
| 11708 | 2 | 3 | 4 | | IV-2 | Lrrc23 | 10233 | 4-May-15 |
| 11712 | 2 | 3 | 4 | | IV-2 | Lrrc27 | 80313 | 5-Jun-15 |
| 11713 | 2 | 3 | 4 | | IV-2 | Lrrc28 | 123355 | 4-May-15 |
| 11717 | 2 | 3 | 4 | | IV-2 | Lrrc32 | 2615 | 20-May-15 |
| 11718 | 2 | 3 | 4 | | IV-2 | Lrrc34 | 151827 | 4-May-15 |
| 11723 | 2 | 3 | 4 | | IV-2 | Lrrc4 | 64101 | 12-May-15 |
| 11724 | 2 | 3 | 4 | | IV-2 | Lrrc40 | 55631 | 4-May-15 |
| 11737 | 2 | 3 | 4 | | IV-2 | Lrrc55 | 219527 | 4-May-15 |
| 11754 | 2 | 3 | 4 | | IV-2 | Lrrc8a | 56262 | 1-Jun-15 |
| 11756 | 2 | 3 | 4 | | IV-2 | Lrrc8c | 84230 | 12-May-15 |
| 11770 | 2 | 3 | 4 | | IV-2 | Lrrn2 | 10446 | 4-May-15 |
| 11771 | 2 | 3 | 4 | | IV-2 | Lrrn3 | 54674 | 4-May-15 |
| 11780 | 2 | 3 | 4 | | IV-2 | Lrtm2 | 654429 | 4-May-15 |
| 11792 | 2 | 3 | 4 | | IV-2 | Lsm4 | 25804 | 28-May-15 |
| 11794 | 2 | 3 | 4 | | IV-2 | Lsm6 | 11157 | 4-May-15 |
| 11798 | 2 | 3 | 4 | | IV-2 | Lsp1 | 4046 | 4-May-15 |
| 11802 | 2 | 3 | 4 | | IV-2 | Lta | 4049 | 12-May-15 |
| 11803 | 2 | 3 | 4 | | IV-2 | Lta4h | 4048 | 12-May-15 |
| 11806 | 2 | 3 | 4 | | IV-2 | Ltb4r2 | 56413 | 4-May-15 |
| 11807 | 2 | 3 | 4 | | IV-2 | Ltbp1 | 4052 | 12-May-15 |
| 11814 | 2 | 3 | 4 | | IV-2 | Ltk | 4058 | 4-May-15 |
| 11831 | 2 | 3 | 4 | | IV-2 | Ly6e | 4061 | 4-May-15 |
| 11837 | 2 | 3 | 4 | | IV-2 | Ly6g6e | 79136 | 4-May-15 |
| 11839 | 2 | 3 | 4 | | IV-2 | Ly6h | 4062 | 4-May-15 |
| 11840 | 2 | 3 | 4 | | IV-2 | Ly6i | | |
| 11842 | 2 | 3 | 4 | | IV-2 | Ly75 | 4065 | 31-May-15 |
| 11846 | 2 | 3 | 4 | | IV-2 | Lyar | 55646 | 4-May-15 |
| 11849 | 2 | 3 | 4 | | IV-2 | Lyl1 | 4066 | 12-May-15 |
| 11850 | 2 | 3 | 4 | | IV-2 | Lyn | 4067 | 12-May-15 |
| 11864 | 2 | 3 | 4 | | IV-2 | Lyrm2 | 57226 | 4-May-15 |
| 11868 | 2 | 3 | 4 | | IV-2 | Lyrm7os | | |
| 11878 | 2 | 3 | 4 | | IV-2 | Lyzl1 | 84569 | 4-May-15 |
| 11886 | 2 | 3 | 4 | | IV-2 | Lzts2 | 84445 | 4-May-15 |
| 11893 | 2 | 3 | 4 | | IV-2 | Mab21l3 | 126868 | 4-May-15 |
| 11897 | 2 | 3 | 4 | | IV-2 | Macrod2 | 140733 | 3-May-15 |
| 11900 | 2 | 3 | 4 | | IV-2 | Mad2l1bp | 9587 | 4-May-15 |
| 11940 | 2 | 3 | 4 | | IV-2 | Magoh | 4116 | 7-Jun-15 |
| 11942 | 2 | 3 | 4 | | IV-2 | Magt1 | 84061 | 23-May-15 |
| 11948 | 2 | 3 | 4 | | IV-2 | Mall | 7851 | 4-May-15 |
| 11949 | 2 | 3 | 4 | | IV-2 | Malsu1 | 115416 | 12-May-15 |
| 11957 | 2 | 3 | 4 | | IV-2 | Mamstr | 284358 | 4-May-15 |
| 11980 | 2 | 3 | 4 | | IV-2 | Map1b | 4131 | 4-May-15 |
| 11981 | 2 | 3 | 4 | | IV-2 | Map1lc3a | 84557 | 21-May-15 |
| 11992 | 2 | 3 | 4 | | IV-2 | Map2k7 | 5609 | 7-Jun-15 |
| 12008 | 2 | 3 | 4 | | IV-2 | Map3k8 | 1326 | 4-May-15 |
| 12037 | 2 | 3 | 4 | | IV-2 | Mapk8ip2 | 23542 | 4-May-15 |
| 12052 | 2 | 3 | 4 | | IV-2 | March10 | 162333 | 4-May-15 |
| 12063 | 2 | 3 | 4 | | IV-2 | Marcksl1 | 65108 | 4-May-15 |
| 12078 | 2 | 3 | 4 | | IV-2 | Masp2 | 10747 | 12-May-15 |
| 12085 | 2 | 3 | 4 | | IV-2 | Mat2a | 4144 | 12-May-15 |
| 12088 | 2 | 3 | 4 | | IV-2 | Matn1 | 4146 | 12-May-15 |
| 12092 | 2 | 3 | 4 | | IV-2 | Matr3 | 9782 | 10-May-15 |
| 12098 | 2 | 3 | 4 | | IV-2 | Mb21d1 | 115004 | 4-May-15 |
| 12144 | 2 | 3 | 4 | | IV-2 | Mcm2 | 4171 | 7-Jun-15 |
| 12145 | 2 | 3 | 4 | | IV-2 | Mcm3 | 4172 | 4-May-15 |
| 12149 | 2 | 3 | 4 | | IV-2 | Mcm6 | 4175 | 4-May-15 |

| | | | | | | | | |
|---|---|---|---|---|---|---|---|---|
| 12162 | 2 | 3 | 4 | | IV-2 | Mcpt8 | | |
| 12176 | 2 | 3 | 4 | | IV-2 | Mdga2 | 161357 | 4-May-15 |
| 12181 | 2 | 3 | 4 | | IV-2 | Mdm1 | 56890 | 12-May-15 |
| 12184 | 2 | 3 | 4 | | IV-2 | Mdn1 | 23195 | 21-May-15 |
| 12188 | 2 | 3 | 4 | | IV-2 | Me3 | 10873 | 12-May-15 |
| 12209 | 2 | 3 | 4 | | IV-2 | Med22 | 6837 | 28-May-15 |
| 12215 | 2 | 3 | 4 | | IV-2 | Med28 | 80306 | 4-May-15 |
| 12225 | 2 | 3 | 4 | | IV-2 | Medag | 84935 | 4-May-15 |
| 12232 | 2 | 3 | 4 | | IV-2 | Megf10 | 84466 | 7-Jun-15 |
| 12237 | 2 | 3 | 4 | | IV-2 | Mei1 | 150365 | 12-May-15 |
| 12240 | 2 | 3 | 4 | | IV-2 | Meiob | 254528 | 4-May-15 |
| 12245 | 2 | 3 | 4 | | IV-2 | Memo1 | 51072 | 7-Jun-15 |
| 12248 | 2 | 3 | 4 | | IV-2 | Meox2 | 4223 | 10-May-15 |
| 12249 | 2 | 3 | 4 | | IV-2 | Mep1a | 4224 | 12-May-15 |
| 12259 | 2 | 3 | 4 | | IV-2 | Met | 4233 | 7-Jun-15 |
| 12268 | 2 | 3 | 4 | | IV-2 | Mettl13 | 51603 | 23-May-15 |
| 12278 | 2 | 3 | 4 | | IV-2 | Mettl21e | | |
| 12282 | 2 | 3 | 4 | | IV-2 | Mettl25 | 84190 | 4-May-15 |
| 12292 | 2 | 3 | 4 | | IV-2 | Mettl8 | 79828 | 4-May-15 |
| 12295 | 2 | 3 | 4 | | IV-2 | Mex3b | 84206 | 4-May-15 |
| 12301 | 2 | 3 | 4 | | IV-2 | Mfap3 | 4238 | 4-May-15 |
| 12305 | 2 | 3 | 4 | | IV-2 | Mff | 56947 | 4-May-15 |
| 12307 | 2 | 3 | 4 | | IV-2 | Mfhas1 | 9258 | 4-May-15 |
| 12319 | 2 | 3 | 4 | | IV-2 | Mfsd3 | 113655 | 4-May-15 |
| 12321 | 2 | 3 | 4 | | IV-2 | Mfsd5 | 84975 | 4-May-15 |
| 12335 | 2 | 3 | 4 | | IV-2 | Mgat4a | 11320 | 4-May-15 |
| 12351 | 2 | 3 | 4 | | IV-2 | Mia2 | 117153 | 7-Jun-15 |
| 12355 | 2 | 3 | 4 | | IV-2 | Mib2 | 142678 | 4-May-15 |
| 12362 | 2 | 3 | 4 | | IV-2 | Micu1 | 10367 | 4-May-15 |
| 12413 | 2 | 3 | 4 | | IV-2 | Mir1224 | 100187716 | 21-May-15 |
| 12435 | 2 | 3 | 4 | | IV-2 | Mir1298 | 100302153 | 4-May-15 |
| 12459 | 2 | 3 | 4 | | IV-2 | Mir143 | 406935 | 21-May-15 |
| 12480 | 2 | 3 | 4 | | IV-2 | Mir17 | 406952 | 24-May-15 |
| 12533 | 2 | 3 | 4 | | IV-2 | Mir1941 | 406969 | 21-May-15 |
| 12554 | 2 | 3 | 4 | | IV-2 | Mir1958 | | |
| 12605 | 2 | 3 | 4 | | IV-2 | Mir216a | 406998 | 21-May-15 |
| 12828 | 2 | 3 | 4 | | IV-2 | Mir434 | | |
| 12877 | 2 | 3 | 4 | | IV-2 | Mir487b | 664616 | 21-May-15 |
| 12936 | 2 | 3 | 4 | | IV-2 | Mir547 | | |
| 12962 | 2 | 3 | 4 | | IV-2 | Mir6237 | | |
| 12967 | 2 | 3 | 4 | | IV-2 | Mir6335 | | |
| 12971 | 2 | 3 | 4 | | IV-2 | Mir6339 | | |
| 12973 | 2 | 3 | 4 | | IV-2 | Mir6341 | | |
| 12987 | 2 | 3 | 4 | | IV-2 | Mir6358 | | |
| 12990 | 2 | 3 | 4 | | IV-2 | Mir6361 | | |
| 12993 | 2 | 3 | 4 | | IV-2 | Mir6364 | | |
| 13019 | 2 | 3 | 4 | | IV-2 | Mir6393 | | |
| 13030 | 2 | 3 | 4 | | IV-2 | Mir6404 | | |
| 13049 | 2 | 3 | 4 | | IV-2 | Mir653 | 724023 | 21-May-15 |
| 13126 | 2 | 3 | 4 | | IV-2 | Mir6920 | | |
| 13195 | 2 | 3 | 4 | | IV-2 | Mir6982 | | |
| 13247 | 2 | 3 | 4 | | IV-2 | Mir7030 | | |
| 13282 | 2 | 3 | 4 | | IV-2 | Mir7061 | | |
| 13331 | 2 | 3 | 4 | | IV-2 | Mir7-2 | 407044 | 21-May-15 |
| 13332 | 2 | 3 | 4 | | IV-2 | Mir721 | | |
| 13376 | 2 | 3 | 4 | | IV-2 | Mir761 | 100313892 | 5-May-15 |
| 13422 | 2 | 3 | 4 | | IV-2 | Mir8092 | | |
| 13425 | 2 | 3 | 4 | | IV-2 | Mir8095 | | |
| 13430 | 2 | 3 | 4 | | IV-2 | Mir8100 | | |
| 13435 | 2 | 3 | 4 | | IV-2 | Mir8105 | | |
| 13443 | 2 | 3 | 4 | | IV-2 | Mir8113 | | |
| 13445 | 2 | 3 | 4 | | IV-2 | Mir8115 | | |
| 13476 | 2 | 3 | 4 | | IV-2 | Mirg | | |
| 13477 | 2 | 3 | 4 | | IV-2 | Mirlet7a-1 | | |
| 13484 | 2 | 3 | 4 | | IV-2 | Mirlet7e | 406887 | 21-May-15 |
| 13494 | 2 | 3 | 4 | | IV-2 | Misp | 126353 | 4-May-15 |
| 13499 | 2 | 3 | 4 | | IV-2 | Mkks | 8195 | 23-May-15 |
| 13501 | 2 | 3 | 4 | | IV-2 | Mkl2 | 57496 | 21-May-15 |
| 13512 | 2 | 3 | 4 | | IV-2 | Mlc1 | 23209 | 31-May-15 |
| 13515 | 2 | 3 | 4 | | IV-2 | Mlf2 | 8079 | 4-May-15 |
| 13531 | 2 | 3 | 4 | | IV-2 | Mlycd | 23417 | 4-May-15 |
| 13554 | 2 | 3 | 4 | | IV-2 | Mmp20 | 9313 | 4-May-15 |
| 13557 | 2 | 3 | 4 | | IV-2 | Mmp24 | 10893 | 4-May-15 |
| 13558 | 2 | 3 | 4 | | IV-2 | Mmp25 | 64386 | 7-Jun-15 |
| 13576 | 2 | 3 | 4 | | IV-2 | Mnt | 4335 | 12-May-15 |
| 13579 | 2 | 3 | 4 | | IV-2 | Mob1a | 55233 | 4-May-15 |
| 13581 | 2 | 3 | 4 | | IV-2 | Mob2 | 81532 | 4-May-15 |
| 13591 | 2 | 3 | 4 | | IV-2 | Mog | 4340 | 21-May-15 |
| 13594 | 2 | 3 | 4 | | IV-2 | Mogs | 7841 | 23-May-15 |
| 13608 | 2 | 3 | 4 | | IV-2 | Morn3 | 283385 | 4-May-15 |
| 13619 | 2 | 3 | 4 | | IV-2 | Moxd2 | 100289017 | 4-May-15 |
| 13632 | 2 | 3 | 4 | | IV-2 | Mplkip | 136647 | 7-Jun-15 |
| 13635 | 2 | 3 | 4 | | IV-2 | Mpp1 | 4354 | 7-Jun-15 |
| 13639 | 2 | 3 | 4 | | IV-2 | Mpp5 | 64398 | 12-May-15 |
| 13643 | 2 | 3 | 4 | | IV-2 | Mpped1 | 758 | 4-May-15 |
| 13644 | 2 | 3 | 4 | | IV-2 | Mpped2 | 744 | 4-May-15 |
| 13649 | 2 | 3 | 4 | | IV-2 | Mpv17 | 4358 | 23-May-15 |
| 13651 | 2 | 3 | 4 | | IV-2 | Mpv17l2 | 84769 | 4-May-15 |
| 13656 | 2 | 3 | 4 | | IV-2 | Mr1 | 3140 | 7-Jun-15 |
| 13662 | 2 | 3 | 4 | | IV-2 | Mre11a | 4361 | 12-May-15 |
| 13664 | 2 | 3 | 4 | | IV-2 | Mrfap1 | 93621 | 4-May-15 |
| 13712 | 2 | 3 | 4 | | IV-2 | Mrpl22 | 29093 | 7-Jun-15 |
| 13756 | 2 | 3 | 4 | | IV-2 | Mrps2 | 51116 | 28-May-15 |
| 13780 | 2 | 3 | 4 | | IV-2 | Ms4a10 | 341116 | 4-May-15 |
| 13787 | 2 | 3 | 4 | | IV-2 | Ms4a4c | | |

Fig.22 - 16

| | | | | | | | | |
|---|---|---|---|---|---|---|---|---|
| 13788 | 2 | 3 | 4 | | IV-2 | Ms4a4d | | |
| 13800 | 2 | 3 | 4 | | IV-2 | Msgn1 | 343930 | 28-May-15 |
| 13807 | 2 | 3 | 4 | | IV-2 | Msi2 | 124540 | 17-May-15 |
| 13813 | 2 | 3 | 4 | | IV-2 | Mslnl | 401827 | 4-May-15 |
| 13822 | 2 | 3 | 4 | | IV-2 | Msrb3 | 253827 | 4-May-15 |
| 13824 | 2 | 3 | 4 | | IV-2 | Mst1 | 4485 | 16-Jun-15 |
| 13827 | 2 | 3 | 4 | | IV-2 | Msto1 | 55154 | 4-May-15 |
| 13829 | 2 | 3 | 4 | | IV-2 | Msx1os | | |
| 13831 | 2 | 3 | 4 | | IV-2 | Msx3 | | |
| 13836 | 2 | 3 | 4 | | IV-2 | Mta1 | 9112 | 17-May-15 |
| 13853 | 2 | 3 | 4 | | IV-2 | Mtf1 | 4520 | 7-Jun-15 |
| 13860 | 2 | 3 | 4 | | IV-2 | Mtg1 | 92170 | 4-May-15 |
| 13883 | 2 | 3 | 4 | | IV-2 | Mtmr9 | 66036 | 4-May-15 |
| 13907 | 2 | 3 | 4 | | IV-2 | Muc2 | 4583 | 4-May-15 |
| 13910 | 2 | 3 | 4 | | IV-2 | Muc5ac | 4586 | 7-Jun-15 |
| 13915 | 2 | 3 | 4 | | IV-2 | Mug2 | | |
| 13941 | 2 | 3 | 4 | | IV-2 | Mus81 | 80198 | 17-May-15 |
| 13944 | 2 | 3 | 4 | | IV-2 | Mut | 4594 | 23-May-15 |
| 13946 | 2 | 3 | 4 | | IV-2 | Mvb12a | 93343 | 4-May-15 |
| 13952 | 2 | 3 | 4 | | IV-2 | Mx2 | 4600 | 12-May-15 |
| 13955 | 2 | 3 | 4 | | IV-2 | Mxd4 | 10608 | 4-May-15 |
| 13962 | 2 | 3 | 4 | | IV-2 | Mybbp1a | 10514 | 4-May-15 |
| 13967 | 2 | 3 | 4 | | IV-2 | Mybpc3 | 4607 | 23-May-15 |
| 13971 | 2 | 3 | 4 | | IV-2 | Mycbp | 26292 | 4-May-15 |
| 13975 | 2 | 3 | 4 | | IV-2 | Mycn | 4613 | 23-May-15 |
| 13976 | 2 | 3 | 4 | | IV-2 | Mycs | | |
| 13978 | 2 | 3 | 4 | | IV-2 | Myd88 | 4615 | 4-May-15 |
| 13983 | 2 | 3 | 4 | | IV-2 | Myg1 | 60314 | 4-May-15 |
| 13988 | 2 | 3 | 4 | | IV-2 | Myh14 | 79784 | 7-Jun-15 |
| 13991 | 2 | 3 | 4 | | IV-2 | Myh3 | 4621 | 12-May-15 |
| 13993 | 2 | 3 | 4 | | IV-2 | Myh6 | 4624 | 23-May-15 |
| 13995 | 2 | 3 | 4 | | IV-2 | Myh7b | 57644 | 4-May-15 |
| 13997 | 2 | 3 | 4 | | IV-2 | Myh9 | 4627 | 23-May-15 |
| 13999 | 2 | 3 | 4 | | IV-2 | Myl10 | 93408 | 4-May-15 |
| 14004 | 2 | 3 | 4 | | IV-2 | Myl4 | 4635 | 12-May-15 |
| 14005 | 2 | 3 | 4 | | IV-2 | Myl6 | 4637 | 4-May-15 |
| 14007 | 2 | 3 | 4 | | IV-2 | Myl7 | 58498 | 4-May-15 |
| 14012 | 2 | 3 | 4 | | IV-2 | Mylk3 | 91807 | 12-May-15 |
| 14015 | 2 | 3 | 4 | | IV-2 | Mynn | 55892 | 12-May-15 |
| 14021 | 2 | 3 | 4 | | IV-2 | Myo19 | 80179 | 12-May-15 |
| 14028 | 2 | 3 | 4 | | IV-2 | Myo1g | 64005 | 12-May-15 |
| 14029 | 2 | 3 | 4 | | IV-2 | Myo1h | 283446 | 4-May-15 |
| 14034 | 2 | 3 | 4 | | IV-2 | Myo5c | 55930 | 4-May-15 |
| 14053 | 2 | 3 | 4 | | IV-2 | Mypop | 339344 | 4-May-15 |
| 14062 | 2 | 3 | 4 | | IV-2 | Mzf1 | 7593 | 7-Jun-15 |
| 14068 | 2 | 3 | 4 | | IV-2 | N4bp2l1 | 90634 | 4-May-15 |
| 14087 | 2 | 3 | 4 | | IV-2 | Naaladl1 | 10004 | 4-May-15 |
| 14110 | 2 | 3 | 4 | | IV-2 | Naip6 | | |
| 14112 | 2 | 3 | 4 | | IV-2 | Nalcn | 259232 | 17-May-15 |
| 14125 | 2 | 3 | 4 | | IV-2 | Napa | 8775 | 7-Jun-15 |
| 14131 | 2 | 3 | 4 | | IV-2 | Narf | 26502 | n-2015 |
| 14145 | 2 | 3 | 4 | | IV-2 | Nat9 | 26151 | 20-May-15 |
| 14161 | 2 | 3 | 4 | | IV-2 | Ncapd3 | 23310 | 4-May-15 |
| 14163 | 2 | 3 | 4 | | IV-2 | Ncapg2 | 54892 | 4-May-15 |
| 14164 | 2 | 3 | 4 | | IV-2 | Ncaph | 23397 | 12-May-15 |
| 14165 | 2 | 3 | 4 | | IV-2 | Ncaph2 | 29781 | 4-May-15 |
| 14169 | 2 | 3 | 4 | | IV-2 | Ncdn | 23154 | 12-May-15 |
| 14174 | 2 | 3 | 4 | | IV-2 | Nck1 | 4690 | 1-Jun-15 |
| 14178 | 2 | 3 | 4 | | IV-2 | Nckap5 | 344148 | 4-May-15 |
| 14194 | 2 | 3 | 4 | | IV-2 | Ncs1 | 23413 | 12-May-15 |
| 14197 | 2 | 3 | 4 | | IV-2 | Ndc1 | 55706 | 4-May-15 |
| 14199 | 2 | 3 | 4 | | IV-2 | Nde1 | 54820 | 4-May-15 |
| 14208 | 2 | 3 | 4 | | IV-2 | Ndrg1 | 10397 | 23-May-15 |
| 14212 | 2 | 3 | 4 | | IV-2 | Ndst1 | 3340 | 4-May-15 |
| 14223 | 2 | 3 | 4 | | IV-2 | Ndufa4 | 4697 | 7-Jun-15 |
| 14225 | 2 | 3 | 4 | | IV-2 | Ndufa5 | 4698 | 12-May-15 |
| 14234 | 2 | 3 | 4 | | IV-2 | Ndufaf4 | 29078 | 4-May-15 |
| 14263 | 2 | 3 | 4 | | IV-2 | Nebl | 10529 | 4-May-15 |
| 14264 | 2 | 3 | 4 | | IV-2 | Necab1 | 64168 | 4-May-15 |
| 14265 | 2 | 3 | 4 | | IV-2 | Necab2 | 54550 | 12-May-15 |
| 14281 | 2 | 3 | 4 | | IV-2 | Nek1 | 4750 | 4-May-15 |
| 14282 | 2 | 3 | 4 | | IV-2 | Nek10 | 152110 | 12-May-15 |
| 14285 | 2 | 3 | 4 | | IV-2 | Nek3 | 4752 | 4-May-15 |
| 14286 | 2 | 3 | 4 | | IV-2 | Nek4 | 6787 | 4-May-15 |
| 14287 | 2 | 3 | 4 | | IV-2 | Nek5 | 341676 | 4-May-15 |
| 14299 | 2 | 3 | 4 | | IV-2 | Nemf | 9147 | 12-May-15 |
| 14303 | 2 | 3 | 4 | | IV-2 | Nes | 10763 | 17-May-15 |
| 14304 | 2 | 3 | 4 | | IV-2 | Nespas | 149775 | 12-May-15 |
| 14308 | 2 | 3 | 4 | | IV-2 | Neu1 | 4758 | 7-Jun-15 |
| 14313 | 2 | 3 | 4 | | IV-2 | Neurl1b | 54492 | 21-May-15 |
| 14315 | 2 | 3 | 4 | | IV-2 | Neurl3 | 93082 | 7-Jun-15 |
| 14323 | 2 | 3 | 4 | | IV-2 | Neurog3 | 50674 | 28-May-15 |
| 14325 | 2 | 3 | 4 | | IV-2 | Nf1 | 4763 | 23-May-15 |
| 14328 | 2 | 3 | 4 | | IV-2 | Nfasc | 23114 | 4-May-15 |
| 14329 | 2 | 3 | 4 | | IV-2 | Nfat5 | 10725 | 12-May-15 |
| 14343 | 2 | 3 | 4 | | IV-2 | Nfix | 4784 | 17-May-15 |
| 14350 | 2 | 3 | 4 | | IV-2 | Nfkbil1 | 4795 | 12-May-15 |
| 14368 | 2 | 3 | 4 | | IV-2 | Ngrn | 51335 | 4-May-15 |
| 14370 | 2 | 3 | 4 | | IV-2 | Nhlh1 | 4807 | 28-May-15 |
| 14373 | 2 | 3 | 4 | | IV-2 | Nhlrc2 | 374354 | 4-May-15 |
| 14376 | 2 | 3 | 4 | | IV-2 | Nhp2 | 55651 | 31-May-15 |
| 14377 | 2 | 3 | 4 | | IV-2 | Nhp2l1 | 4809 | 2-Jun-15 |
| 14384 | 2 | 3 | 4 | | IV-2 | Nif3l1 | 60491 | 3-May-15 |
| 14390 | 2 | 3 | 4 | | IV-2 | Ninl | 22981 | 4-May-15 |
| 14402 | 2 | 3 | 4 | | IV-2 | Nisch | 11188 | 17-May-15 |
| 14430 | 2 | 3 | 4 | | IV-2 | Nkx3-2 | 579 | 4-May-15 |
| 14433 | 2 | 3 | 4 | | IV-2 | Nkx6-3 | 157848 | 4-May-15 |

| | | | | | | | | |
|---|---|---|---|---|---|---|---|---|
| 14445 | 2 | 3 | 4 | | IV-2 | Nlrp14 | 338323 | 4-May-15 |
| 14460 | 2 | 3 | 4 | | IV-2 | Nlrp9a | | |
| 14465 | 2 | 3 | 4 | | IV-2 | Nmbr | 4829 | 4-May-15 |
| 14470 | 2 | 3 | 4 | | IV-2 | Nme4 | 4833 | 4-May-15 |
| 14472 | 2 | 3 | 4 | | IV-2 | Nme6 | 10201 | 21-May-15 |
| 14474 | 2 | 3 | 4 | | IV-2 | Nme8 | 51314 | 28-May-15 |
| 14478 | 2 | 3 | 4 | | IV-2 | Nmnat2 | 23057 | 4-May-15 |
| 14483 | 2 | 3 | 4 | | IV-2 | Nms | 129521 | 4-May-15 |
| 14491 | 2 | 3 | 4 | | IV-2 | Nnt | 23530 | 13-May-15 |
| 14502 | 2 | 3 | 4 | | IV-2 | Nol10 | 79954 | 4-May-15 |
| 14525 | 2 | 3 | 4 | | IV-2 | Nos3 | 4846 | 7-Jun-15 |
| 14535 | 2 | 3 | 4 | | IV-2 | Nova1 | 4857 | 4-May-15 |
| 14545 | 2 | 3 | 4 | | IV-2 | Npas3 | 64067 | 28-May-15 |
| 14547 | 2 | 3 | 4 | | IV-2 | Npat | 4863 | 4-May-15 |
| 14554 | 2 | 3 | 4 | | IV-2 | Npdc1 | 56654 | 4-May-15 |
| 14571 | 2 | 3 | 4 | | IV-2 | Npm3-ps1 | | |
| 14578 | 2 | 3 | 4 | | IV-2 | Npr3 | 4883 | un-2015 |
| 14579 | 2 | 3 | 4 | | IV-2 | Nprl2 | 10641 | 29-May-15 |
| 14587 | 2 | 3 | 4 | | IV-2 | Npvf | 64111 | 4-May-15 |
| 14590 | 2 | 3 | 4 | | IV-2 | Npy1r | 4886 | 4-May-15 |
| 14600 | 2 | 3 | 4 | | IV-2 | Nr1d2 | 9975 | 4-May-15 |
| 14609 | 2 | 3 | 4 | | IV-2 | Nr2c2ap | 126382 | 4-May-15 |
| 14625 | 2 | 3 | 4 | | IV-2 | Nrarp | 441478 | 12-May-15 |
| 14626 | 2 | 3 | 4 | | IV-2 | Nras | 4893 | 24-May-15 |
| 14634 | 2 | 3 | 4 | | IV-2 | Nrf1 | 4899 | 7-Jun-15 |
| 14642 | 2 | 3 | 4 | | IV-2 | Nrip2 | 83714 | 4-May-15 |
| 14643 | 2 | 3 | 4 | | IV-2 | Nrip3 | 56675 | 4-May-15 |
| 14649 | 2 | 3 | 4 | | IV-2 | Nron | 641373 | 4-May-15 |
| 14654 | 2 | 3 | 4 | | IV-2 | Nrsn1 | 140767 | 12-May-15 |
| 14663 | 2 | 3 | 4 | | IV-2 | Nsf | 4905 | 28-May-15 |
| 14667 | 2 | 3 | 4 | | IV-2 | Nsl1 | 25936 | 7-Jun-15 |
| 14668 | 2 | 3 | 4 | | IV-2 | Nsmaf | 8439 | 4-May-15 |
| 14681 | 2 | 3 | 4 | | IV-2 | Nt5c1b | 93034 | 4-May-15 |
| 14689 | 2 | 3 | 4 | | IV-2 | Nt5m | 56953 | 4-May-15 |
| 14697 | 2 | 3 | 4 | | IV-2 | Ntn3 | 4917 | 12-May-15 |
| 14702 | 2 | 3 | 4 | | IV-2 | Ntpcr | 84284 | 12-May-15 |
| 14705 | 2 | 3 | 4 | | IV-2 | Ntrk3 | 4916 | 12-May-15 |
| 14718 | 2 | 3 | 4 | | IV-2 | Nudc | 10726 | 4-May-15 |
| 14732 | 2 | 3 | 4 | | IV-2 | Nudt18 | 79873 | 4-May-15 |
| 14734 | 2 | 3 | 4 | | IV-2 | Nudt2 | 318 | 12-May-15 |
| 14735 | 2 | 3 | 4 | | IV-2 | Nudt21 | 11051 | 4-May-15 |
| 14742 | 2 | 3 | 4 | | IV-2 | Nudt8 | 254552 | 4-May-15 |
| 14743 | 2 | 3 | 4 | | IV-2 | Nudt9 | 53343 | 4-May-15 |
| 14745 | 2 | 3 | 4 | | IV-2 | Nufip1 | 26747 | 4-May-15 |
| 14748 | 2 | 3 | 4 | | IV-2 | Numa1 | 4926 | 21-May-15 |
| 14763 | 2 | 3 | 4 | | IV-2 | Nup43 | 348995 | 4-May-15 |
| 14777 | 2 | 3 | 4 | | IV-2 | Nus1 | 116150 | 28-May-15 |
| 14779 | 2 | 3 | 4 | | IV-2 | Nutf2 | 10204 | 12-May-15 |
| 14781 | 2 | 3 | 4 | | IV-2 | Nutm1 | 256646 | 4-May-15 |
| 14794 | 2 | 3 | 4 | | IV-2 | Nxpe4 | 54827 | 4-May-15 |
| 14796 | 2 | 3 | 4 | | IV-2 | Nxph1 | 30010 | 4-May-15 |
| 14800 | 2 | 3 | 4 | | IV-2 | Nxt1 | 29107 | 4-May-15 |
| 14805 | 2 | 3 | 4 | | IV-2 | Nyx | 60506 | 23-May-15 |
| 14806 | 2 | 3 | 4 | | IV-2 | Oacyl | | |
| 14808 | 2 | 3 | 4 | | IV-2 | Oard1 | 221443 | 4-May-15 |
| 14816 | 2 | 3 | 4 | | IV-2 | Oas1h | | |
| 14818 | 2 | 3 | 4 | | IV-2 | Oas3 | 4940 | 10-May-15 |
| 14819 | 2 | 3 | 4 | | IV-2 | Oasl1 | | |
| 14820 | 2 | 3 | 4 | | IV-2 | Oasl2 | | |
| 14821 | 2 | 3 | 4 | | IV-2 | Oat | 4942 | 12-May-15 |
| 14822 | 2 | 3 | 4 | | IV-2 | Oaz1 | 4946 | 12-May-15 |
| 14824 | 2 | 3 | 4 | | IV-2 | Oaz2 | 4947 | 4-May-15 |
| 14826 | 2 | 3 | 4 | | IV-2 | Obfc1 | 79991 | 12-May-15 |
| 14837 | 2 | 3 | 4 | | IV-2 | Oc90 | 729330 | 4-May-15 |
| 14853 | 2 | 3 | 4 | | IV-2 | Odf3l1 | 161753 | 4-May-15 |
| 14869 | 2 | 3 | 4 | | IV-2 | Oit1 | 131177 | 4-May-15 |
| 14875 | 2 | 3 | 4 | | IV-2 | Olfm3 | 118427 | 4-May-15 |
| 14876 | 2 | 3 | 4 | | IV-2 | Olfm4 | 10562 | 4-May-15 |
| 14879 | 2 | 3 | 4 | | IV-2 | Olfml2b | 25903 | 4-May-15 |
| 14880 | 2 | 3 | 4 | | IV-2 | Olfml3 | 56944 | 4-May-15 |
| 14881 | 2 | 3 | 4 | | IV-2 | Olfr1 | 4991 | 4-May-15 |
| 15225 | 2 | 3 | 4 | | IV-2 | Olfr140 | | |
| 15330 | 2 | 3 | 4 | | IV-2 | Olfr166 | | |
| 15380 | 2 | 3 | 4 | | IV-2 | Olfr225 | | |
| 15631 | 2 | 3 | 4 | | IV-2 | Olfr555 | | |
| 15635 | 2 | 3 | 4 | | IV-2 | Olfr559 | | |
| 15982 | 2 | 3 | 4 | | IV-2 | Olfr980 | | |
| 15999 | 2 | 3 | 4 | | IV-2 | Olig2 | 10215 | 17-May-15 |
| 16001 | 2 | 3 | 4 | | IV-2 | Olr1 | 4973 | 12-May-15 |
| 16004 | 2 | 3 | 4 | | IV-2 | Omg | 4974 | 4-May-15 |
| 16006 | 2 | 3 | 4 | | IV-2 | Omt2a | | |
| 16009 | 2 | 3 | 4 | | IV-2 | Onecut2 | 9480 | 28-May-15 |
| 16038 | 2 | 3 | 4 | | IV-2 | Orai3 | 93129 | 4-May-15 |
| 16041 | 2 | 3 | 4 | | IV-2 | Orc2 | 4999 | 7-Jun-15 |
| 16047 | 2 | 3 | 4 | | IV-2 | Orm2 | 5005 | 3-May-15 |
| 16049 | 2 | 3 | 4 | | IV-2 | Ormdl1 | 94101 | 4-May-15 |
| 16071 | 2 | 3 | 4 | | IV-2 | Osgin2 | 734 | 4-May-15 |
| 16076 | 2 | 3 | 4 | | IV-2 | Ost4 | 100128731 | 4-May-15 |
| 16081 | 2 | 3 | 4 | | IV-2 | Otc | 5009 | 23-May-15 |
| 16088 | 2 | 3 | 4 | | IV-2 | Otop2 | 92736 | 4-May-15 |
| 16114 | 2 | 3 | 4 | | IV-2 | Ovol3 | 728361 | 28-May-15 |
| 16133 | 2 | 3 | 4 | | IV-2 | P2rx7 | 5027 | 17-May-15 |
| 16135 | 2 | 3 | 4 | | IV-2 | P2ry10 | 27334 | 4-May-15 |
| 16136 | 2 | 3 | 4 | | IV-2 | P2ry12 | 64805 | 17-May-15 |
| 16137 | 2 | 3 | 4 | | IV-2 | P2ry13 | 53829 | 21-May-15 |
| 16138 | 2 | 3 | 4 | | IV-2 | P2ry14 | 9934 | 4-May-15 |
| 16142 | 2 | 3 | 4 | | IV-2 | P4ha1 | 5033 | 12-May-15 |

Fig.22 - 17

| | | | | | | | | |
|---|---|---|---|---|---|---|---|---|
| 16173 | 2 | 3 | 4 | | IV-2 | Pafah2 | 5051 | 4-May-15 |
| 16177 | 2 | 3 | 4 | | IV-2 | Paics | 10606 | 4-May-15 |
| 16182 | 2 | 3 | 4 | | IV-2 | Pak1ip1 | 55003 | 4-May-15 |
| 16185 | 2 | 3 | 4 | | IV-2 | Pak4 | 10298 | 4-May-15 |
| 16191 | 2 | 3 | 4 | | IV-2 | Palm | 5064 | 12-May-15 |
| 16195 | 2 | 3 | 4 | | IV-2 | Pam | 5066 | 13-Jun-15 |
| 16213 | 2 | 3 | 4 | | IV-2 | Papola | 10914 | 3-Jun-15 |
| 16220 | 2 | 3 | 4 | | IV-2 | Paqr3 | 152559 | 4-May-15 |
| 16221 | 2 | 3 | 4 | | IV-2 | Paqr4 | 124222 | 4-May-15 |
| 16225 | 2 | 3 | 4 | | IV-2 | Paqr8 | 85315 | 4-May-15 |
| 16237 | 2 | 3 | 4 | | IV-2 | Parn | 5073 | 4-Jun-15 |
| 16251 | 2 | 3 | 4 | | IV-2 | Pars2 | 25973 | 4-May-15 |
| 16254 | 2 | 3 | 4 | | IV-2 | Parvg | 64098 | 12-May-15 |
| 16255 | 2 | 3 | 4 | | IV-2 | Pask | 23178 | 7-Jun-15 |
| 16268 | 2 | 3 | 4 | | IV-2 | Pax6 | 5080 | 23-May-15 |
| 16288 | 2 | 3 | 4 | | IV-2 | Pcbd2 | 84105 | 4-May-15 |
| 16296 | 2 | 3 | 4 | | IV-2 | Pcdh10 | 57575 | 4-May-15 |
| 16299 | 2 | 3 | 4 | | IV-2 | Pcdh15 | 65217 | 23-May-15 |
| 16302 | 2 | 3 | 4 | | IV-2 | Pcdh19 | 57526 | 7-Jun-15 |
| 16348 | 2 | 3 | 4 | | IV-2 | Pcdhga2 | 56113 | 4-May-15 |
| 16396 | 2 | 3 | 4 | | IV-2 | Pcsk1n | 27344 | 4-May-15 |
| 16401 | 2 | 3 | 4 | | IV-2 | Pcsk5 | 5125 | 12-May-15 |
| 16434 | 2 | 3 | 4 | | IV-2 | Pde1c | 5137 | 4-May-15 |
| 16435 | 2 | 3 | 4 | | IV-2 | Pde2a | 5138 | 21-May-15 |
| 16443 | 2 | 3 | 4 | | IV-2 | Pde5a | 8654 | 4-May-15 |
| 16445 | 2 | 3 | 4 | | IV-2 | Pde6b | 5158 | 23-May-15 |
| 16463 | 2 | 3 | 4 | | IV-2 | Pdha1 | 5160 | 21-May-15 |
| 16468 | 2 | 3 | 4 | | IV-2 | Pdia3 | 2923 | 4-May-15 |
| 16481 | 2 | 3 | 4 | | IV-2 | Pdlim4 | 8572 | 4-May-15 |
| 16482 | 2 | 3 | 4 | | IV-2 | Pdlim5 | 10611 | 12-May-15 |
| 16489 | 2 | 3 | 4 | | IV-2 | Pdrg1 | 81572 | 12-May-15 |
| 16493 | 2 | 3 | 4 | | IV-2 | Pdss2 | 57107 | 31-May-15 |
| 16509 | 2 | 3 | 4 | | IV-2 | Pdzrn3 | 23024 | 12-May-15 |
| 16525 | 2 | 3 | 4 | | IV-2 | Peli1 | 57162 | 12-May-15 |
| 16532 | 2 | 3 | 4 | | IV-2 | Peo1 | 56652 | 23-May-15 |
| 16563 | 2 | 3 | 4 | | IV-2 | Pfas | 5198 | 21-May-15 |
| 16571 | 2 | 3 | 4 | | IV-2 | Pfkfb4 | 5210 | 12-May-15 |
| 16574 | 2 | 3 | 4 | | IV-2 | Pfkp | 5214 | 4-May-15 |
| 16578 | 2 | 3 | 4 | | IV-2 | Pfn4 | 375189 | 4-May-15 |
| 16581 | 2 | 3 | 4 | | IV-2 | Pgam1 | 5223 | 7-Jun-15 |
| 16583 | 2 | 3 | 4 | | IV-2 | Pgam5 | 192111 | 24-May-15 |
| 16590 | 2 | 3 | 4 | | IV-2 | Pgd | 5226 | 7-Jun-15 |
| 16593 | 2 | 3 | 4 | | IV-2 | Pgk1 | 5230 | 12-May-15 |
| 16595 | 2 | 3 | 4 | | IV-2 | Pgls | 25796 | 4-May-15 |
| 16597 | 2 | 3 | 4 | | IV-2 | Pglyrp2 | 114770 | 4-May-15 |
| 16605 | 2 | 3 | 4 | | IV-2 | Pgp | 283871 | 7-Jun-15 |
| 16628 | 2 | 3 | 4 | | IV-2 | Phf11c | | |
| 16634 | 2 | 3 | 4 | | IV-2 | Phf2 | 5253 | 7-Jun-15 |
| 16649 | 2 | 3 | 4 | | IV-2 | Phka2 | 5256 | 23-May-15 |
| 16652 | 2 | 3 | 4 | | IV-2 | Phkg2 | 5261 | 4-May-15 |
| 16654 | 2 | 3 | 4 | | IV-2 | Phlda2 | 7262 | 4-May-15 |
| 16655 | 2 | 3 | 4 | | IV-2 | Phlda3 | 23612 | 14-May-15 |
| 16674 | 2 | 3 | 4 | | IV-2 | Phyhipl | 84457 | 4-May-15 |
| 16677 | 2 | 3 | 4 | | IV-2 | Pi16 | 221476 | 1-May-15 |
| 16683 | 2 | 3 | 4 | | IV-2 | Pias1 | 8554 | 4-May-15 |
| 16691 | 2 | 3 | 4 | | IV-2 | Pidd1 | 55367 | 12-May-15 |
| 16708 | 2 | 3 | 4 | | IV-2 | Pigq | 9091 | 4-May-15 |
| 16710 | 2 | 3 | 4 | | IV-2 | Pigs | 94005 | 4-May-15 |
| 16722 | 2 | 3 | 4 | | IV-2 | Pik3c2a | 5286 | 24-May-15 |
| 16729 | 2 | 3 | 4 | | IV-2 | Pik3cg | 5294 | 12-May-15 |
| 16740 | 2 | 3 | 4 | | IV-2 | Pilrb2 | | |
| 16747 | 2 | 3 | 4 | | IV-2 | Pinc | | |
| 16752 | 2 | 3 | 4 | | IV-2 | Pip4k2a | 5305 | 4-May-15 |
| 16763 | 2 | 3 | 4 | | IV-2 | Pira2 | | |
| 16766 | 2 | 3 | 4 | | IV-2 | Pira7 | | |
| 16768 | 2 | 3 | 4 | | IV-2 | Pirt | 644139 | 12-May-15 |
| 16784 | 2 | 3 | 4 | | IV-2 | Pitx3 | 5309 | 23-May-15 |
| 16804 | 2 | 3 | 4 | | IV-2 | Pkm | 5315 | 17-May-15 |
| 16816 | 2 | 3 | 4 | | IV-2 | Pla2g10 | 8399 | 12-May-15 |
| 16823 | 2 | 3 | 4 | | IV-2 | Pla2g2a | 5320 | 4-May-15 |
| 16826 | 2 | 3 | 4 | | IV-2 | Pla2g2e | 30814 | 4-May-15 |
| 16827 | 2 | 3 | 4 | | IV-2 | Pla2g2f | 64600 | 4-May-15 |
| 16832 | 2 | 3 | 4 | | IV-2 | Pla2g4d | 283748 | 4-May-15 |
| 16842 | 2 | 3 | 4 | | IV-2 | Plac8l1 | 153770 | 4-May-15 |
| 16844 | 2 | 3 | 4 | | IV-2 | Plac9b | | |
| 16852 | 2 | 3 | 4 | | IV-2 | Plbd1 | 79887 | 4-May-15 |
| 16858 | 2 | 3 | 4 | | IV-2 | Plcd1 | 5333 | 17-May-15 |
| 16868 | 2 | 3 | 4 | | IV-2 | Plcxd1 | 55344 | 4-May-15 |
| 16870 | 2 | 3 | 4 | | IV-2 | Plcxd3 | 345557 | 4-May-15 |
| 16889 | 2 | 3 | 4 | | IV-2 | Plekha8 | 84725 | 4-May-15 |
| 16919 | 2 | 3 | 4 | | IV-2 | Plin3 | 10226 | 4-May-15 |
| 16926 | 2 | 3 | 4 | | IV-2 | Plk5 | 126520 | 4-May-15 |
| 16929 | 2 | 3 | 4 | | IV-2 | Plod1 | 5351 | 23-May-15 |
| 16943 | 2 | 3 | 4 | | IV-2 | Plvap | 83483 | 4-May-15 |
| 16955 | 2 | 3 | 4 | | IV-2 | Plxnd1 | 23129 | 4-May-15 |
| 16956 | 2 | 3 | 4 | | IV-2 | Pm20d1 | 148811 | 4-May-15 |
| 16961 | 2 | 3 | 4 | | IV-2 | Pmepa1 | 56937 | 4-May-15 |
| 16962 | 2 | 3 | 4 | | IV-2 | Pmf1 | 11243 | 7-Jun-15 |
| 16975 | 2 | 3 | 4 | | IV-2 | Pnck | 139728 | 4-May-15 |
| 16982 | 2 | 3 | 4 | | IV-2 | Pnliprp2 | 5408 | 4-May-15 |
| 16983 | 2 | 3 | 4 | | IV-2 | Pnma1 | 9240 | 17-May-15 |
| 16999 | 2 | 3 | 4 | | IV-2 | Pnpla6 | 10908 | 12-May-15 |
| 17029 | 2 | 3 | 4 | | IV-2 | Pole2 | 5427 | 12-May-15 |
| 17030 | 2 | 3 | 4 | | IV-2 | Pole3 | 54107 | 4-May-15 |
| 17041 | 2 | 3 | 4 | | IV-2 | Polr1a | 25885 | 4-Jun-15 |
| 17057 | 2 | 3 | 4 | | IV-2 | Polr2l | 5441 | 12-May-15 |
| 17074 | 2 | 3 | 4 | | IV-2 | Pomgnt1 | 55624 | 23-May-15 |

| | | | | | | | | |
|---|---|---|---|---|---|---|---|---|
| 17089 | 2 | 3 | 4 | | IV-2 | Por | 5447 | 7-Jun-15 |
| 17092 | 2 | 3 | 4 | | IV-2 | Pot1a | | |
| 17097 | 2 | 3 | 4 | | IV-2 | Pou2f1 | 5451 | 28-May-15 |
| 17124 | 2 | 3 | 4 | | IV-2 | Ppard | 5467 | 17-May-15 |
| 17130 | 2 | 3 | 4 | | IV-2 | Ppcdc | 60490 | 12-May-15 |
| 17132 | 2 | 3 | 4 | | IV-2 | Ppdpf | 79144 | 12-May-15 |
| 17145 | 2 | 3 | 4 | | IV-2 | Ppid | 5481 | 17-May-15 |
| 17152 | 2 | 3 | 4 | | IV-2 | Ppil2 | 23759 | 12-May-15 |
| 17159 | 2 | 3 | 4 | | IV-2 | Ppm1a | 5494 | 4-May-15 |
| 17163 | 2 | 3 | 4 | | IV-2 | Ppm1f | 9647 | 4-May-15 |
| 17177 | 2 | 3 | 4 | | IV-2 | Ppp1r11 | 6992 | 12-May-15 |
| 17180 | 2 | 3 | 4 | | IV-2 | Ppp1r12c | 54776 | 4-May-15 |
| 17184 | 2 | 3 | 4 | | IV-2 | Ppp1r14b | 26472 | 12-May-15 |
| 17186 | 2 | 3 | 4 | | IV-2 | Ppp1r14d | 54866 | 4-May-15 |
| 17195 | 2 | 3 | 4 | | IV-2 | Ppp1r1c | 151242 | 12-May-15 |
| 17203 | 2 | 3 | 4 | | IV-2 | Ppp1r32 | 220004 | 4-May-15 |
| 17212 | 2 | 3 | 4 | | IV-2 | Ppp1r3f | 89801 | 4-May-15 |
| 17218 | 2 | 3 | 4 | | IV-2 | Ppp1r9a | 55607 | 12-May-15 |
| 17263 | 2 | 3 | 4 | | IV-2 | Pradc1 | 84279 | 4-May-15 |
| 17268 | 2 | 3 | 4 | | IV-2 | Pramef17 | 391004 | 4-May-15 |
| 17280 | 2 | 3 | 4 | | IV-2 | Prc1 | 9055 | 4-May-15 |
| 17281 | 2 | 3 | 4 | | IV-2 | Prcc | 5546 | 4-May-15 |
| 17294 | 2 | 3 | 4 | | IV-2 | Prdm6 | 93166 | 12-May-15 |
| 17308 | 2 | 3 | 4 | | IV-2 | Prep | 5550 | 7-Jun-15 |
| 17313 | 2 | 3 | 4 | | IV-2 | Prg2 | 5553 | 7-Jun-15 |
| 17324 | 2 | 3 | 4 | | IV-2 | Primpol | 201973 | 23-May-15 |
| 17327 | 2 | 3 | 4 | | IV-2 | Prkab1 | 5564 | 2-Jun-15 |
| 17329 | 2 | 3 | 4 | | IV-2 | Prkaca | 5566 | 7-Jun-15 |
| 17335 | 2 | 3 | 4 | | IV-2 | Prkar1a | 5573 | 23-May-15 |
| 17339 | 2 | 3 | 4 | | IV-2 | Prkca | 5578 | 31-May-15 |
| 17341 | 2 | 3 | 4 | | IV-2 | Prkcd | 5580 | 17-May-15 |
| 17361 | 2 | 3 | 4 | | IV-2 | Prl2a1 | | |
| 17403 | 2 | 3 | 4 | | IV-2 | Prnp | 5621 | 23-May-15 |
| 17405 | 2 | 3 | 4 | | IV-2 | Proc | 5624 | 31-May-15 |
| 17411 | 2 | 3 | 4 | | IV-2 | Prok2 | 60675 | 23-May-15 |
| 17413 | 2 | 3 | 4 | | IV-2 | Prokr2 | 128674 | 23-May-15 |
| 17424 | 2 | 3 | 4 | | IV-2 | Prox2 | 283571 | 4-May-15 |
| 17443 | 2 | 3 | 4 | | IV-2 | Prps1 | 5631 | 7-Jun-15 |
| 17447 | 2 | 3 | 4 | | IV-2 | Prpsap1 | 5635 | 21-May-15 |
| 17450 | 2 | 3 | 4 | | IV-2 | Prr12 | 57479 | 4-May-15 |
| 17467 | 2 | 3 | 4 | | IV-2 | Prr5 | 55615 | 4-May-15 |
| 17469 | 2 | 3 | 4 | | IV-2 | Prr7 | 80758 | 4-May-15 |
| 17470 | 2 | 3 | 4 | | IV-2 | Prr9 | 574414 | 4-May-15 |
| 17485 | 2 | 3 | 4 | | IV-2 | Prrxl1 | 644168 | 4-May-15 |
| 17490 | 2 | 3 | 4 | | IV-2 | Prss21 | 10942 | 10-May-15 |
| 17494 | 2 | 3 | 4 | | IV-2 | Prss28 | | |
| 17497 | 2 | 3 | 4 | | IV-2 | Prss30 | | |
| 17499 | 2 | 3 | 4 | | IV-2 | Prss33 | 260429 | 4-May-15 |
| 17502 | 2 | 3 | 4 | | IV-2 | Prss36 | 146547 | 4-May-15 |
| 17518 | 2 | 3 | 4 | | IV-2 | Prss54 | 221191 | 20-May-15 |
| 17526 | 2 | 3 | 4 | | IV-2 | Prune | 58497 | 12-May-15 |
| 17528 | 2 | 3 | 4 | | IV-2 | Prx | 57716 | 7-Jun-15 |
| 17533 | 2 | 3 | 4 | | IV-2 | Psd | 5662 | 7-Jun-15 |
| 17534 | 2 | 3 | 4 | | IV-2 | Psd2 | 84249 | 4-May-15 |
| 17575 | 2 | 3 | 4 | | IV-2 | Psmc1 | 5700 | 21-May-15 |
| 17607 | 2 | 3 | 4 | | IV-2 | Pspc1 | 55269 | 4-May-15 |
| 17616 | 2 | 3 | 4 | | IV-2 | Ptbp1 | 5725 | 4-May-15 |
| 17623 | 2 | 3 | 4 | | IV-2 | Ptch2 | 8643 | 23-May-15 |
| 17624 | 2 | 3 | 4 | | IV-2 | Ptchd1 | 139411 | 23-May-15 |
| 17635 | 2 | 3 | 4 | | IV-2 | Ptgdr2 | 11251 | 4-May-15 |
| 17637 | 2 | 3 | 4 | | IV-2 | Ptger1 | 5731 | 4-May-15 |
| 17640 | 2 | 3 | 4 | | IV-2 | Ptger4 | 5734 | 4-May-15 |
| 17649 | 2 | 3 | 4 | | IV-2 | Ptgr1 | 22949 | 4-May-15 |
| 17650 | 2 | 3 | 4 | | IV-2 | Ptgr2 | 145482 | 4-May-15 |
| 17656 | 2 | 3 | 4 | | IV-2 | Pth2 | 113091 | 4-May-15 |
| 17666 | 2 | 3 | 4 | | IV-2 | Ptov1 | 53635 | 17-May-15 |
| 17674 | 2 | 3 | 4 | | IV-2 | Ptplb | 201562 | 4-May-15 |
| 17692 | 2 | 3 | 4 | | IV-2 | Ptpn9 | 5780 | 4-May-15 |
| 17697 | 2 | 3 | 4 | | IV-2 | Ptprd | 5789 | 4-May-15 |
| 17703 | 2 | 3 | 4 | | IV-2 | Ptprk | 5796 | 12-May-15 |
| 17718 | 2 | 3 | 4 | | IV-2 | Ptrh2 | 51651 | 21-May-15 |
| 17725 | 2 | 3 | 4 | | IV-2 | Puf60 | 22827 | 2-Jun-15 |
| 17731 | 2 | 3 | 4 | | IV-2 | Pus1 | 80324 | 4-May-15 |
| 17738 | 2 | 3 | 4 | | IV-2 | Pvr | 5817 | 17-May-15 |
| 17750 | 2 | 3 | 4 | | IV-2 | Pxk | 54899 | 17-May-15 |
| 17752 | 2 | 3 | 4 | | IV-2 | Pxmp4 | 11264 | 29-May-15 |
| 17758 | 2 | 3 | 4 | | IV-2 | Pycr2 | 29920 | 4-May-15 |
| 17760 | 2 | 3 | 4 | | IV-2 | Pydc3 | | |
| 17768 | 2 | 3 | 4 | | IV-2 | Pyroxd1 | 79912 | 4-May-15 |
| 17773 | 2 | 3 | 4 | | IV-2 | Qars | 5859 | 7-Jun-15 |
| 17777 | 2 | 3 | 4 | | IV-2 | Qpctl | 54814 | 4-May-15 |
| 17802 | 2 | 3 | 4 | | IV-2 | Rab11fip2 | 22841 | 4-May-15 |
| 17824 | 2 | 3 | 4 | | IV-2 | Rab27a | 5873 | 14-May-15 |
| 17830 | 2 | 3 | 4 | | IV-2 | Rab31 | 11031 | 4-May-15 |
| 17839 | 2 | 3 | 4 | | IV-2 | Rab39 | 54734 | 7-Jun-15 |
| 17852 | 2 | 3 | 4 | | IV-2 | Rab43 | 339122 | 4-May-15 |
| 17861 | 2 | 3 | 4 | | IV-2 | Rab7 | 7879 | 7-Jun-15 |
| 17867 | 2 | 3 | 4 | | IV-2 | Rabac1 | 10567 | 4-May-15 |
| 17878 | 2 | 3 | 4 | | IV-2 | Rabl3 | 285282 | 4-May-15 |
| 17882 | 2 | 3 | 4 | | IV-2 | Rac3 | 5881 | 7-Jun-15 |
| 17884 | 2 | 3 | 4 | | IV-2 | Rad1 | 5810 | 7-Jun-15 |
| 17894 | 2 | 3 | 4 | | IV-2 | Rad51ap2 | 729475 | 4-May-15 |
| 17899 | 2 | 3 | 4 | | IV-2 | Rad54b | 25788 | 4-May-15 |
| 17900 | 2 | 3 | 4 | | IV-2 | Rad54l | 8438 | 7-Jun-15 |
| 17901 | 2 | 3 | 4 | | IV-2 | Rad54l2 | 23132 | 4-May-15 |
| 17910 | 2 | 3 | 4 | | IV-2 | Raet1e | 135250 | 12-May-15 |
| 17911 | 2 | 3 | 4 | | IV-2 | Raf1 | 5894 | 23-May-15 |

Fig.22 - 18

| | | | | | | | | |
|---|---|---|---|---|---|---|---|---|
| 17913 | 2 | 3 | 4 | | IV-2 | Rag2 | 5897 | 12-May-15 |
| 17917 | 2 | 3 | 4 | | IV-2 | Rala | 5898 | 4-May-15 |
| 17957 | 2 | 3 | 4 | | IV-2 | Raph1 | 65059 | 4-May-15 |
| 17964 | 2 | 3 | 4 | | IV-2 | Rars | 5917 | 4-May-15 |
| 17966 | 2 | 3 | 4 | | IV-2 | Rasa1 | 5921 | 7-Jun-15 |
| 17978 | 2 | 3 | 4 | | IV-2 | Rasgef1c | 255426 | 4-May-15 |
| 17980 | 2 | 3 | 4 | | IV-2 | Rasgrf2 | 5924 | 4-May-15 |
| 17982 | 2 | 3 | 4 | | IV-2 | Rasgrp2 | 10235 | 4-May-15 |
| 17984 | 2 | 3 | 4 | | IV-2 | Rasgrp4 | 115727 | 12-May-15 |
| 17987 | 2 | 3 | 4 | | IV-2 | Rasl10b | 91608 | 4-May-15 |
| 17991 | 2 | 3 | 4 | | IV-2 | Rasl2-9 | | |
| 18020 | 2 | 3 | 4 | | IV-2 | Rbfox2 | 23543 | 2-Jun-15 |
| 18032 | 2 | 3 | 4 | | IV-2 | Rbm15 | 64783 | 4-May-15 |
| 18040 | 2 | 3 | 4 | | IV-2 | Rbm25 | 58517 | 12-May-15 |
| 18056 | 2 | 3 | 4 | | IV-2 | Rbm45 | 129831 | 4-May-15 |
| 18079 | 2 | 3 | 4 | | IV-2 | Rbpj | 3516 | 12-May-15 |
| 18081 | 2 | 3 | 4 | | IV-2 | Rbpms | 11030 | 4-May-15 |
| 18118 | 2 | 3 | 4 | | IV-2 | Rdh8 | 50700 | 21-May-15 |
| 18121 | 2 | 3 | 4 | | IV-2 | Rdx | 5962 | 3-Jun-15 |
| 18140 | 2 | 3 | 4 | | IV-2 | Reg4 | 83998 | 4-May-15 |
| 18141 | 2 | 3 | 4 | | IV-2 | Rel | 5966 | 28-May-15 |
| 18149 | 2 | 3 | 4 | | IV-2 | Rem2 | 161253 | 4-May-15 |
| 18151 | 2 | 3 | 4 | | IV-2 | Ren2 | | |
| 18153 | 2 | 3 | 4 | | IV-2 | Rep15 | 387849 | 28-May-15 |
| 18163 | 2 | 3 | 4 | | IV-2 | Ret | 5979 | 22-May-15 |
| 18189 | 2 | 3 | 4 | | IV-2 | Rftn2 | 130132 | 12-May-15 |
| 18211 | 2 | 3 | 4 | | IV-2 | Rgp1 | 9827 | un-2015 |
| 18215 | 2 | 3 | 4 | | IV-2 | Rgs11 | 8786 | 21-May-15 |
| 18218 | 2 | 3 | 4 | | IV-2 | Rgs14 | 10636 | 4-May-15 |
| 18219 | 2 | 3 | 4 | | IV-2 | Rgs16 | 6004 | 4-May-15 |
| 18220 | 2 | 3 | 4 | | IV-2 | Rgs17 | 26575 | 4-May-15 |
| 18230 | 2 | 3 | 4 | | IV-2 | Rgs6 | 9628 | 17-May-15 |
| 18233 | 2 | 3 | 4 | | IV-2 | Rgs8 | 85397 | 4-May-15 |
| 18238 | 2 | 3 | 4 | | IV-2 | Rhbdd1 | 84236 | 29-May-15 |
| 18244 | 2 | 3 | 4 | | IV-2 | Rhbdl2 | 54933 | 4-May-15 |
| 18249 | 2 | 3 | 4 | | IV-2 | Rheb | 6009 | 7-Jun-15 |
| 18251 | 2 | 3 | 4 | | IV-2 | Rhno1 | 83695 | 4-May-15 |
| 18253 | 2 | 3 | 4 | | IV-2 | Rhoa | 387 | 31-May-15 |
| 18263 | 2 | 3 | 4 | | IV-2 | Rhoj | 57381 | 4-May-15 |
| 18269 | 2 | 3 | 4 | | IV-2 | Rhox1 | | |
| 18296 | 2 | 3 | 4 | | IV-2 | Rhox6 | | |
| 18303 | 2 | 3 | 4 | | IV-2 | Ribc1 | 158787 | 4-May-15 |
| 18310 | 2 | 3 | 4 | | IV-2 | Riiad1 | 284485 | 4-May-15 |
| 18322 | 2 | 3 | 4 | | IV-2 | Rin1 | 9610 | 4-May-15 |
| 18333 | 2 | 3 | 4 | | IV-2 | Ripk3 | 11035 | 17-May-15 |
| 18346 | 2 | 3 | 4 | | IV-2 | Rltpr | 146206 | 4-May-15 |
| 18347 | 2 | 3 | 4 | | IV-2 | Rmdn1 | 51115 | 12-May-15 |
| 18353 | 2 | 3 | 4 | | IV-2 | Rmnd5a | 64795 | 4-May-15 |
| 18356 | 2 | 3 | 4 | | IV-2 | Rmst | 196475 | 4-May-15 |
| 18377 | 2 | 3 | 4 | | IV-2 | Rnd1 | 27289 | 4-May-15 |
| 18379 | 2 | 3 | 4 | | IV-2 | Rnd3 | 390 | 12-May-15 |
| 18391 | 2 | 3 | 4 | | IV-2 | Rnf123 | 63891 | 4-May-15 |
| 18393 | 2 | 3 | 4 | | IV-2 | Rnf126 | 55658 | 4-May-15 |
| 18404 | 2 | 3 | 4 | | IV-2 | Rnf144a | 9781 | 18-May-15 |
| 18424 | 2 | 3 | 4 | | IV-2 | Rnf183 | 138065 | 4-May-15 |
| 18425 | 2 | 3 | 4 | | IV-2 | Rnf185 | 91445 | 28-May-15 |
| 18444 | 2 | 3 | 4 | | IV-2 | Rnf25 | 64320 | 4-May-15 |
| 18473 | 2 | 3 | 4 | | IV-2 | Rnu6 | | |
| 18485 | 2 | 3 | 4 | | IV-2 | Ropn1 | 54763 | 31-May-15 |
| 18500 | 2 | 3 | 4 | | IV-2 | Rpain | 84268 | 4-May-15 |
| 18509 | 2 | 3 | 4 | | IV-2 | Rpgrip1 | 57096 | 12-May-15 |
| 18511 | 2 | 3 | 4 | | IV-2 | Rph3a | 22895 | 4-May-15 |
| 18538 | 2 | 3 | 4 | | IV-2 | Rpl29 | 6159 | 4-May-15 |
| 18541 | 2 | 3 | 4 | | IV-2 | Rpl31 | 6160 | 4-May-15 |
| 18546 | 2 | 3 | 4 | | IV-2 | Rpl35 | 11224 | 4-May-15 |
| 18554 | 2 | 3 | 4 | | IV-2 | Rpl39 | 6170 | 12-May-15 |
| 18557 | 2 | 3 | 4 | | IV-2 | Rpl4 | 6124 | 4-May-15 |
| 18574 | 2 | 3 | 4 | | IV-2 | Rpp25 | 54913 | 4-May-15 |
| 18592 | 2 | 3 | 4 | | IV-2 | Rps14 | 6208 | 2-Jun-15 |
| 18616 | 2 | 3 | 4 | | IV-2 | Rps3 | 6188 | 4-May-15 |
| 18632 | 2 | 3 | 4 | | IV-2 | Rps7 | 6201 | 7-Jun-15 |
| 18645 | 2 | 3 | 4 | | IV-2 | Rraga | 10670 | 29-May-15 |
| 18656 | 2 | 3 | 4 | | IV-2 | Rrm2b | 50484 | 17-May-15 |
| 18671 | 2 | 3 | 4 | | IV-2 | Rsbn1 | 54665 | 4-May-15 |
| 18703 | 2 | 3 | 4 | | IV-2 | Rtkn2 | 219790 | 4-May-15 |
| 18704 | 2 | 3 | 4 | | IV-2 | Rtl1 | 388015 | 4-May-15 |
| 18707 | 2 | 3 | 4 | | IV-2 | Rtn3 | 10313 | 12-May-15 |
| 18713 | 2 | 3 | 4 | | IV-2 | Rtp1 | 132112 | 9-May-15 |
| 18716 | 2 | 3 | 4 | | IV-2 | Rtp4 | 64108 | 9-May-15 |
| 18727 | 2 | 3 | 4 | | IV-2 | Runx1t1 | 862 | 21-May-15 |
| 18730 | 2 | 3 | 4 | | IV-2 | Rusc1 | 23623 | 4-May-15 |
| 18737 | 2 | 3 | 4 | | IV-2 | Rwdd3 | 25950 | 4-May-15 |
| 18749 | 2 | 3 | 4 | | IV-2 | Ryr2 | 6262 | 23-May-15 |
| 18760 | 2 | 3 | 4 | | IV-2 | S100a5 | 6276 | 4-May-15 |
| 18765 | 2 | 3 | 4 | | IV-2 | S100b | 6285 | 24-May-15 |
| 18771 | 2 | 3 | 4 | | IV-2 | S1pr3 | 1903 | 4-May-15 |
| 18772 | 2 | 3 | 4 | | IV-2 | S1pr4 | 8698 | 4-May-15 |
| 18777 | 2 | 3 | 4 | | IV-2 | Saa4 | 6291 | 7-Jun-15 |
| 18778 | 2 | 3 | 4 | | IV-2 | Saal1 | 113174 | 4-May-15 |
| 18793 | 2 | 3 | 4 | | IV-2 | Samd12 | 401474 | 4-May-15 |
| 18806 | 2 | 3 | 4 | | IV-2 | Samt2 | | |
| 18812 | 2 | 3 | 4 | | IV-2 | Sap30 | 8819 | 4-May-15 |
| 18813 | 2 | 3 | 4 | | IV-2 | Sap30bp | 29115 | 12-May-15 |
| 18817 | 2 | 3 | 4 | | IV-2 | Sar1a | 56681 | 3-May-15 |
| 18820 | 2 | 3 | 4 | | IV-2 | Sarm1 | 23098 | 12-May-15 |
| 18821 | 2 | 3 | 4 | | IV-2 | Sarnp | 84324 | 4-May-15 |
| 18828 | 2 | 3 | 4 | | IV-2 | Sass6 | 163786 | 4-May-15 |
| 18832 | 2 | 3 | 4 | | IV-2 | Satb2 | 23314 | 4-May-15 |
| 18847 | 2 | 3 | 4 | | IV-2 | Sbspon | 157869 | 4-May-15 |
| 18864 | 2 | 3 | 4 | | IV-2 | Scarb1 | 949 | 24-May-15 |
| 18871 | 2 | 3 | 4 | | IV-2 | Scarna17 | 677769 | 12-May-15 |
| 18877 | 2 | 3 | 4 | | IV-2 | Scarna9 | 619383 | 12-May-15 |
| 18890 | 2 | 3 | 4 | | IV-2 | Scgb1b19 | | |
| 18895 | 2 | 3 | 4 | | IV-2 | Scgb1b29 | | |
| 18897 | 2 | 3 | 4 | | IV-2 | Scgb1b30 | | |
| 18903 | 2 | 3 | 4 | | IV-2 | Scgb2b19 | | |
| 18906 | 2 | 3 | 4 | | IV-2 | Scgb2b23-ps | | |
| 18910 | 2 | 3 | 4 | | IV-2 | Scgb2b3 | | |
| 18912 | 2 | 3 | 4 | | IV-2 | Scgb3a1 | 92304 | 4-May-15 |
| 18914 | 2 | 3 | 4 | | IV-2 | Scgn | 10590 | 4-May-15 |
| 18923 | 2 | 3 | 4 | | IV-2 | Scn10a | 6336 | 4-May-15 |
| 18927 | 2 | 3 | 4 | | IV-2 | Scn2a1 | 6326 | 17-May-15 |
| 18933 | 2 | 3 | 4 | | IV-2 | Scn5a | 6331 | 31-May-15 |
| 18948 | 2 | 3 | 4 | | IV-2 | Scrg1 | 11341 | 4-May-15 |
| 18951 | 2 | 3 | 4 | | IV-2 | Scrn2 | 90507 | 4-May-15 |
| 18961 | 2 | 3 | 4 | | IV-2 | Scyl1 | 57410 | 7-Jun-15 |
| 18976 | 2 | 3 | 4 | | IV-2 | Sdf4 | 51150 | 4-May-15 |
| 18991 | 2 | 3 | 4 | | IV-2 | Sds | 10993 | 21-May-15 |
| 18993 | 2 | 3 | 4 | | IV-2 | Sebox | 645832 | 12-May-15 |
| 19001 | 2 | 3 | 4 | | IV-2 | Sec14l4 | 284904 | 4-May-15 |
| 19002 | 2 | 3 | 4 | | IV-2 | Sec14l5 | 9717 | 4-May-15 |
| 19021 | 2 | 3 | 4 | | IV-2 | Sec62 | 7095 | 12-May-15 |
| 19034 | 2 | 3 | 4 | | IV-2 | Selk | 58515 | 4-May-15 |
| 19036 | 2 | 3 | 4 | | IV-2 | Selm | 140606 | 4-May-15 |
| 19040 | 2 | 3 | 4 | | IV-2 | Selt | 51714 | 4-May-15 |
| 19044 | 2 | 3 | 4 | | IV-2 | Sema3d | 223117 | 21-May-15 |
| 19059 | 2 | 3 | 4 | | IV-2 | Sema6d | 80031 | 4-May-15 |
| 19060 | 2 | 3 | 4 | | IV-2 | Sema7a | 8482 | 4-May-15 |
| 19061 | 2 | 3 | 4 | | IV-2 | Senp1 | 29843 | 4-May-15 |
| 19074 | 2 | 3 | 4 | | IV-2 | Sept10 | 151011 | 4-May-15 |
| 19088 | 2 | 3 | 4 | | IV-2 | Serac1 | 84947 | 23-May-15 |
| 19091 | 2 | 3 | 4 | | IV-2 | Serf2 | 10169 | 4-May-15 |
| 19102 | 2 | 3 | 4 | | IV-2 | Serpina11 | 256394 | 4-May-15 |
| 19115 | 2 | 3 | 4 | | IV-2 | Serpina3h | | |
| 19122 | 2 | 3 | 4 | | IV-2 | Serpina5 | 5104 | 12-May-15 |
| 19131 | 2 | 3 | 4 | | IV-2 | Serpinb1b | | |
| 19135 | 2 | 3 | 4 | | IV-2 | Serpinb3b | | |
| 19140 | 2 | 3 | 4 | | IV-2 | Serpinb6b | | |
| 19160 | 2 | 3 | 4 | | IV-2 | Serping1 | 710 | 12-May-15 |
| 19162 | 2 | 3 | 4 | | IV-2 | Serpini1 | 5274 | 12-May-15 |
| 19164 | 2 | 3 | 4 | | IV-2 | Sertad1 | 29950 | 4-May-15 |
| 19168 | 2 | 3 | 4 | | IV-2 | Sertm1 | 400120 | 4-May-15 |
| 19172 | 2 | 3 | 4 | | IV-2 | Sestd1 | 91404 | 4-May-15 |
| 19211 | 2 | 3 | 4 | | IV-2 | Sfswap | 6433 | 12-May-15 |
| 19219 | 2 | 3 | 4 | | IV-2 | Sftpd | 6441 | 12-May-15 |
| 19223 | 2 | 3 | 4 | | IV-2 | Sfxn4 | 119559 | 4-May-15 |
| 19230 | 2 | 3 | 4 | | IV-2 | Sgcz | 137868 | 17-May-15 |
| 19238 | 2 | 3 | 4 | | IV-2 | Sgol2 | 151246 | 4-May-15 |
| 19239 | 2 | 3 | 4 | | IV-2 | Sgpl1 | 8879 | 4-May-15 |
| 19248 | 2 | 3 | 4 | | IV-2 | Sh2b1 | 25970 | 21-May-15 |
| 19250 | 2 | 3 | 4 | | IV-2 | Sh2b3 | 10019 | 21-May-15 |
| 19252 | 2 | 3 | 4 | | IV-2 | Sh2d1b1 | | |
| 19261 | 2 | 3 | 4 | | IV-2 | Sh3bgrl | 6451 | 4-May-15 |
| 19266 | 2 | 3 | 4 | | IV-2 | Sh3bp4 | 23677 | 4-May-15 |
| 19277 | 2 | 3 | 4 | | IV-2 | Sh3pxd2a | 9644 | 4-May-15 |
| 19278 | 2 | 3 | 4 | | IV-2 | Sh3pxd2b | 285590 | 12-May-15 |
| 19296 | 2 | 3 | 4 | | IV-2 | Shcbp1l | 81626 | 12-May-15 |
| 19303 | 2 | 3 | 4 | | IV-2 | Shisa3 | 152573 | 4-May-15 |
| 19321 | 2 | 3 | 4 | | IV-2 | Siae | 54414 | 4-May-15 |
| 19334 | 2 | 3 | 4 | | IV-2 | Siglech | | |
| 19335 | 2 | 3 | 4 | | IV-2 | Sigmar1 | 10280 | 23-May-15 |
| 19338 | 2 | 3 | 4 | | IV-2 | Sik3 | 23387 | 24-May-15 |
| 19357 | 2 | 3 | 4 | | IV-2 | Sirt5 | 23408 | 31-May-15 |
| 19362 | 2 | 3 | 4 | | IV-2 | Siva1 | 10572 | 31-May-15 |
| 19403 | 2 | 3 | 4 | | IV-2 | Slamf8 | 56833 | 4-May-15 |
| 19404 | 2 | 3 | 4 | | IV-2 | Slamf9 | 89886 | 4-May-15 |
| 19405 | 2 | 3 | 4 | | IV-2 | Slbp | 7884 | 12-May-15 |
| 19408 | 2 | 3 | 4 | | IV-2 | Slc10a3 | 8273 | 4-May-15 |
| 19431 | 2 | 3 | 4 | | IV-2 | Slc14a1 | 6563 | 21-May-15 |
| 19436 | 2 | 3 | 4 | | IV-2 | Slc15a4 | 121260 | 12-May-15 |
| 19438 | 2 | 3 | 4 | | IV-2 | Slc16a1 | 6566 | 21-May-15 |
| 19443 | 2 | 3 | 4 | | IV-2 | Slc16a14 | 151473 | 4-May-15 |
| 19446 | 2 | 3 | 4 | | IV-2 | Slc16a4 | 9122 | 4-May-15 |
| 19456 | 2 | 3 | 4 | | IV-2 | Slc17a5 | 26503 | 23-May-15 |
| 19476 | 2 | 3 | 4 | | IV-2 | Slc20a2 | 6575 | 23-May-15 |
| 19479 | 2 | 3 | 4 | | IV-2 | Slc22a13 | 9390 | 4-May-15 |
| 19487 | 2 | 3 | 4 | | IV-2 | Slc22a2 | 6582 | 4-May-15 |
| 19495 | 2 | 3 | 4 | | IV-2 | Slc22a29 | | |
| 19508 | 2 | 3 | 4 | | IV-2 | Slc24a3 | 57419 | 4-May-15 |
| 19511 | 2 | 3 | 4 | | IV-2 | Slc25a1 | 6576 | 17-May-15 |
| 19512 | 2 | 3 | 4 | | IV-2 | Slc25a10 | 1468 | 4-May-15 |
| 19535 | 2 | 3 | 4 | | IV-2 | Slc25a31 | 83447 | 4-May-15 |
| 19554 | 2 | 3 | 4 | | IV-2 | Slc25a5 | 292 | 6-May-15 |
| 19562 | 2 | 3 | 4 | | IV-2 | Slc26a3 | 1811 | 4-May-15 |
| 19567 | 2 | 3 | 4 | | IV-2 | Slc26a8 | 116369 | 4-May-15 |
| 19571 | 2 | 3 | 4 | | IV-2 | Slc27a3 | 11000 | 4-May-15 |
| 19572 | 2 | 3 | 4 | | IV-2 | Slc27a4 | 10999 | 17-May-15 |
| 19585 | 2 | 3 | 4 | | IV-2 | Slc2a13 | 114134 | 4-May-15 |
| 19586 | 2 | 3 | 4 | | IV-2 | Slc2a2 | 6514 | 12-May-15 |
| 19589 | 2 | 3 | 4 | | IV-2 | Slc2a4rg-ps | | |
| 19591 | 2 | 3 | 4 | | IV-2 | Slc2a6 | 11182 | 4-May-15 |
| 19599 | 2 | 3 | 4 | | IV-2 | Slc30a4 | 7782 | 4-May-15 |
| 19602 | 2 | 3 | 4 | | IV-2 | Slc30a7 | 148867 | 4-May-15 |
| 19611 | 2 | 3 | 4 | | IV-2 | Slc34a3 | 142680 | 12-May-15 |

Fig.22 - 19

| | | | | | | | | |
|---|---|---|---|---|---|---|---|---|
| 19612 | 2 | 3 | 4 | | IV-2 | Slc35a1 | 10559 | 23-May-15 |
| 19642 | 2 | 3 | 4 | | IV-2 | Slc36a3 | 285641 | 4-May-15 |
| 19650 | 2 | 3 | 4 | | IV-2 | Slc38a11 | 151258 | 4-May-15 |
| 19655 | 2 | 3 | 4 | | IV-2 | Slc38a6 | 145389 | 4-May-15 |
| 19661 | 2 | 3 | 4 | | IV-2 | Slc39a11 | 201266 | 4-May-15 |
| 19676 | 2 | 3 | 4 | | IV-2 | Slc41a1 | 254428 | 4-May-15 |
| 19680 | 2 | 3 | 4 | | IV-2 | Slc43a2 | 124935 | 21-May-15 |
| 19698 | 2 | 3 | 4 | | IV-2 | Slc4a10 | 57282 | 4-May-15 |
| 19730 | 2 | 3 | 4 | | IV-2 | Slc6a14 | 11254 | 4-May-15 |
| 19735 | 2 | 3 | 4 | | IV-2 | Slc6a19os | | |
| 19741 | 2 | 3 | 4 | | IV-2 | Slc6a5 | 9152 | 7-Jun-15 |
| 19751 | 2 | 3 | 4 | | IV-2 | Slc7a14 | 57709 | 4-May-15 |
| 19769 | 2 | 3 | 4 | | IV-2 | Slc9a3r1 | 9368 | 31-May-15 |
| 19777 | 2 | 3 | 4 | | IV-2 | Slc9b1 | 150159 | 31-May-15 |
| 19790 | 2 | 3 | 4 | | IV-2 | Slco4c1 | 353189 | 4-May-15 |
| 19792 | 2 | 3 | 4 | | IV-2 | Slco6b1 | | |
| 19796 | 2 | 3 | 4 | | IV-2 | Slfn10-ps | | |
| 19799 | 2 | 3 | 4 | | IV-2 | Slfn3 | 55106 | 4-May-15 |
| 19800 | 2 | 3 | 4 | | IV-2 | Slfn4 | | |
| 19801 | 2 | 3 | 4 | | IV-2 | Slfn5 | 162394 | 4-May-15 |
| 19802 | 2 | 3 | 4 | | IV-2 | Slfn5os | | |
| 19805 | 2 | 3 | 4 | | IV-2 | Slfnl1 | 200172 | 4-May-15 |
| 19807 | 2 | 3 | 4 | | IV-2 | Slit1 | 6585 | 7-Jun-15 |
| 19816 | 2 | 3 | 4 | | IV-2 | Slk | 9748 | 7-Jun-15 |
| 19822 | 2 | 3 | 4 | | IV-2 | Sltm | 79811 | 4-May-15 |
| 19844 | 2 | 3 | 4 | | IV-2 | Smarca4 | 6597 | 4-May-15 |
| 19859 | 2 | 3 | 4 | | IV-2 | Smc2os | | |
| 19862 | 2 | 3 | 4 | | IV-2 | Smc5 | 23137 | 4-May-15 |
| 19866 | 2 | 3 | 4 | | IV-2 | Smco2 | 341346 | 4-May-15 |
| 19870 | 2 | 3 | 4 | | IV-2 | Smcr8 | 140775 | 4-May-15 |
| 19882 | 2 | 3 | 4 | | IV-2 | Smim11 | 54065 | 4-May-15 |
| 19888 | 2 | 3 | 4 | | IV-2 | Smim19 | 114926 | 4-May-15 |
| 19894 | 2 | 3 | 4 | | IV-2 | Smim4 | 440957 | 4-May-15 |
| 19912 | 2 | 3 | 4 | | IV-2 | Smpd1 | 6609 | 23-May-15 |
| 19915 | 2 | 3 | 4 | | IV-2 | Smpd4 | 55627 | 12-May-15 |
| 19920 | 2 | 3 | 4 | | IV-2 | Smr2 | | |
| 19922 | 2 | 3 | 4 | | IV-2 | Sms | 6611 | 13-Jun-15 |
| 19926 | 2 | 3 | 4 | | IV-2 | Smu1 | 55234 | 4-May-15 |
| 19931 | 2 | 3 | 4 | | IV-2 | Smyd2 | 56950 | 21-May-15 |
| 19943 | 2 | 3 | 4 | | IV-2 | Snapc1 | 6617 | 4-May-15 |
| 19946 | 2 | 3 | 4 | | IV-2 | Snapc4 | 6621 | 4-May-15 |
| 19953 | 2 | 3 | 4 | | IV-2 | Snd1 | 27044 | 31-May-15 |
| 19955 | 2 | 3 | 4 | | IV-2 | Snf8 | 11267 | 21-May-15 |
| 19965 | 2 | 3 | 4 | | IV-2 | Snhg7 | 84973 | 12-May-15 |
| 19966 | 2 | 3 | 4 | | IV-2 | Snhg8 | 100093630 | 12-May-15 |
| 19973 | 2 | 3 | 4 | | IV-2 | Snora19 | 641451 | 4-May-15 |
| 19977 | 2 | 3 | 4 | | IV-2 | Snora24 | 677809 | 21-May-15 |
| 19986 | 2 | 3 | 4 | | IV-2 | Snora35 | 677816 | 4-May-15 |
| 19991 | 2 | 3 | 4 | | IV-2 | Snora47 | 677828 | 4-May-15 |
| 19993 | 2 | 3 | 4 | | IV-2 | Snora5c | 677796 | 4-May-15 |
| 20006 | 2 | 3 | 4 | | IV-2 | Snord100 | 594838 | 4-May-15 |
| 20022 | 2 | 3 | 4 | | IV-2 | Snord16a | | |
| 20024 | 2 | 3 | 4 | | IV-2 | Snord19 | 692089 | 4-May-15 |
| 20031 | 2 | 3 | 4 | | IV-2 | Snord32a | 26819 | 4-May-15 |
| 20094 | 2 | 3 | 4 | | IV-2 | Snrpd1 | 6632 | 7-Jun-15 |
| 20100 | 2 | 3 | 4 | | IV-2 | Snrpn | 6638 | 23-May-15 |
| 20104 | 2 | 3 | 4 | | IV-2 | Sntg1 | 54212 | 28-May-15 |
| 20106 | 2 | 3 | 4 | | IV-2 | Sntn | 132203 | 4-May-15 |
| 20132 | 2 | 3 | 4 | | IV-2 | Snx32 | 254122 | 12-May-15 |
| 20147 | 2 | 3 | 4 | | IV-2 | Socs5 | 9655 | 12-May-15 |
| 20153 | 2 | 3 | 4 | | IV-2 | Soga1 | 140710 | 12-May-15 |
| 20162 | 2 | 3 | 4 | | IV-2 | Sorcs1 | 114815 | 4-May-15 |
| 20167 | 2 | 3 | 4 | | IV-2 | Sort1 | 6272 | 31-May-15 |
| 20178 | 2 | 3 | 4 | | IV-2 | Sox11 | 6664 | 28-May-15 |
| 20186 | 2 | 3 | 4 | | IV-2 | Sox21 | 11166 | 28-May-15 |
| 20194 | 2 | 3 | 4 | | IV-2 | Sox7 | 83595 | 4-May-15 |
| 20195 | 2 | 3 | 4 | | IV-2 | Sox8 | 30812 | 4-May-15 |
| 20201 | 2 | 3 | 4 | | IV-2 | Sp2 | 6668 | 7-Jun-15 |
| 20206 | 2 | 3 | 4 | | IV-2 | Sp6 | 80320 | 4-May-15 |
| 20212 | 2 | 3 | 4 | | IV-2 | Spaca3 | 124912 | 4-May-15 |
| 20224 | 2 | 3 | 4 | | IV-2 | Spag6 | 9576 | 12-May-15 |
| 20230 | 2 | 3 | 4 | | IV-2 | Sparcl1 | 8404 | 12-May-15 |
| 20253 | 2 | 3 | 4 | | IV-2 | Spata4 | 132851 | 4-May-15 |
| 20257 | 2 | 3 | 4 | | IV-2 | Spata6 | 54558 | 4-May-15 |
| 20264 | 2 | 3 | 4 | | IV-2 | Spats2l | 26010 | 4-May-15 |
| 20267 | 2 | 3 | 4 | | IV-2 | Spcs1 | 28972 | 4-May-15 |
| 20284 | 2 | 3 | 4 | | IV-2 | Speer4f | | |
| 20321 | 2 | 3 | 4 | | IV-2 | Spink4 | 27290 | 4-May-15 |
| 20335 | 2 | 3 | 4 | | IV-2 | Spn-ps | | |
| 20338 | 2 | 3 | 4 | | IV-2 | Spns3 | 201305 | 4-May-15 |
| 20344 | 2 | 3 | 4 | | IV-2 | Spon2 | 10417 | 4-May-15 |
| 20345 | 2 | 3 | 4 | | IV-2 | Spop | 8405 | 17-May-15 |
| 20348 | 2 | 3 | 4 | | IV-2 | Spp2 | 6694 | 4-May-15 |
| 20368 | 2 | 3 | 4 | | IV-2 | Sprr2i | | |
| 20378 | 2 | 3 | 4 | | IV-2 | Spryd3 | 84926 | 12-May-15 |
| 20387 | 2 | 3 | 4 | | IV-2 | Sptan1 | 6709 | 4-May-15 |
| 20389 | 2 | 3 | 4 | | IV-2 | Sptbn1 | 6711 | 4-May-15 |
| 20397 | 2 | 3 | 4 | | IV-2 | Spty2d1 | 144108 | 4-May-15 |
| 20400 | 2 | 3 | 4 | | IV-2 | Sqrdl | 58472 | 4-May-15 |
| 20409 | 2 | 3 | 4 | | IV-2 | Srd5a3 | 79644 | 23-May-15 |
| 20411 | 2 | 3 | 4 | | IV-2 | Srebf2 | 6721 | 17-May-15 |
| 20420 | 2 | 3 | 4 | | IV-2 | Sri | 6717 | 31-May-15 |
| 20422 | 2 | 3 | 4 | | IV-2 | Srm | 6723 | 7-Jun-15 |
| 20429 | 2 | 3 | 4 | | IV-2 | Srp68 | 6730 | 4-May-15 |
| 20439 | 2 | 3 | 4 | | IV-2 | Srr | 63826 | 4-May-15 |
| 20468 | 2 | 3 | 4 | | IV-2 | Ssfa2 | 6744 | 4-May-15 |
| 20470 | 2 | 3 | 4 | | IV-2 | Ssh2 | 85464 | 12-May-15 |

| | | | | | | | | |
|---|---|---|---|---|---|---|---|---|
| 20483 | 2 | 3 | 4 | | IV-2 | Sstr1 | 6751 | 4-May-15 |
| 20485 | 2 | 3 | 4 | | IV-2 | Sstr3 | 6753 | 24-May-15 |
| 20487 | 2 | 3 | 4 | | IV-2 | Sstr5 | 6755 | 12-May-15 |
| 20507 | 2 | 3 | 4 | | IV-2 | St3gal3 | 6487 | 4-May-15 |
| 20516 | 2 | 3 | 4 | | IV-2 | St6galnac3 | 256435 | 4-May-15 |
| 20524 | 2 | 3 | 4 | | IV-2 | St8sia3 | 51046 | 23-May-15 |
| 20534 | 2 | 3 | 4 | | IV-2 | Stag1 | 10274 | 16-Jun-15 |
| 20550 | 2 | 3 | 4 | | IV-2 | Stard5 | 80765 | 4-May-15 |
| 20574 | 2 | 3 | 4 | | IV-2 | Stil | 6491 | 23-May-15 |
| 20575 | 2 | 3 | 4 | | IV-2 | Stim1 | 6786 | 31-May-15 |
| 20584 | 2 | 3 | 4 | | IV-2 | Stk24 | 8428 | 12-May-15 |
| 20600 | 2 | 3 | 4 | | IV-2 | Stmn1-rs1 | | |
| 20603 | 2 | 3 | 4 | | IV-2 | Stmn4 | 81551 | 4-May-15 |
| 20610 | 2 | 3 | 4 | | IV-2 | Ston2 | 85439 | 12-May-15 |
| 20617 | 2 | 3 | 4 | | IV-2 | Stra8 | 346673 | 4-May-15 |
| 20625 | 2 | 3 | 4 | | IV-2 | Strn | 6801 | 4-May-15 |
| 20626 | 2 | 3 | 4 | | IV-2 | Strn3 | 29966 | 4-May-15 |
| 20632 | 2 | 3 | 4 | | IV-2 | Stx12 | 23673 | 4-May-15 |
| 20647 | 2 | 3 | 4 | | IV-2 | Stxbp2 | 6813 | 22-May-15 |
| 20662 | 2 | 3 | 4 | | IV-2 | Suco | 51430 | 4-May-15 |
| 20663 | 2 | 3 | 4 | | IV-2 | Suds3 | 64426 | 4-May-15 |
| 20673 | 2 | 3 | 4 | | IV-2 | Sult1c1 | 6819 | 7-Jun-15 |
| 20675 | 2 | 3 | 4 | | IV-2 | Sult1d1 | 133150 | 4-May-15 |
| 20676 | 2 | 3 | 4 | | IV-2 | Sult1e1 | 6783 | 12-May-15 |
| 20677 | 2 | 3 | 4 | | IV-2 | Sult2a1 | 6822 | 7-Jun-15 |
| 20688 | 2 | 3 | 4 | | IV-2 | Sult6b1 | 391365 | 4-May-15 |
| 20713 | 2 | 3 | 4 | | IV-2 | Susd3 | 203328 | 12-May-15 |
| 20715 | 2 | 3 | 4 | | IV-2 | Susd5 | 26032 | 4-May-15 |
| 20746 | 2 | 3 | 4 | | IV-2 | Syce1 | 93426 | 4-May-15 |
| 20749 | 2 | 3 | 4 | | IV-2 | Syce3 | 644186 | 4-May-15 |
| 20751 | 2 | 3 | 4 | | IV-2 | Sycp1 | 6847 | 4-May-15 |
| 20763 | 2 | 3 | 4 | | IV-2 | Syna | | |
| 20766 | 2 | 3 | 4 | | IV-2 | Syncrip | 10492 | 1-Jun-15 |
| 20775 | 2 | 3 | 4 | | IV-2 | Syngr2 | 9144 | 4-May-15 |
| 20782 | 2 | 3 | 4 | | IV-2 | Synpo | 11346 | 4-May-15 |
| 20785 | 2 | 3 | 4 | | IV-2 | Synpr | 132204 | 4-May-15 |
| 20788 | 2 | 3 | 4 | | IV-2 | Sypl | 6856 | 4-May-15 |
| 20790 | 2 | 3 | 4 | | IV-2 | Sys1 | 90196 | 4-May-15 |
| 20792 | 2 | 3 | 4 | | IV-2 | Syt10 | 341359 | 4-May-15 |
| 20795 | 2 | 3 | 4 | | IV-2 | Syt13 | 57586 | 4-May-15 |
| 20796 | 2 | 3 | 4 | | IV-2 | Syt14 | 255928 | 12-May-15 |
| 20799 | 2 | 3 | 4 | | IV-2 | Syt17 | 51760 | 12-May-15 |
| 20803 | 2 | 3 | 4 | | IV-2 | Syt5 | 6861 | 4-May-15 |
| 20806 | 2 | 3 | 4 | | IV-2 | Syt8 | 90019 | 4-May-15 |
| 20855 | 2 | 3 | 4 | | IV-2 | Taf13 | 6884 | 4-May-15 |
| 20880 | 2 | 3 | 4 | | IV-2 | Tal2 | 6887 | 28-May-15 |
| 20885 | 2 | 3 | 4 | | IV-2 | Tango2 | 128989 | 4-May-15 |
| 20892 | 2 | 3 | 4 | | IV-2 | Tap2 | 6891 | 7-Jun-15 |
| 20942 | 2 | 3 | 4 | | IV-2 | Tatdn1 | 83940 | 4-May-15 |
| 20949 | 2 | 3 | 4 | | IV-2 | Tbc1d1 | 23216 | 4-May-15 |
| 20953 | 2 | 3 | 4 | | IV-2 | Tbc1d12 | 23232 | 4-May-15 |
| 20991 | 2 | 3 | 4 | | IV-2 | Tbl1xr1 | 79718 | 12-May-15 |
| 21002 | 2 | 3 | 4 | | IV-2 | Tbx10 | 347853 | 4-May-15 |
| 21009 | 2 | 3 | 4 | | IV-2 | Tbx22 | 50945 | 17-May-15 |
| 21016 | 2 | 3 | 4 | | IV-2 | Tbxas1 | 6916 | 12-May-15 |
| 21018 | 2 | 3 | 4 | | IV-2 | Tcaim | 285343 | 4-May-15 |
| 21021 | 2 | 3 | 4 | | IV-2 | Tcea1 | 6917 | 4-May-15 |
| 21024 | 2 | 3 | 4 | | IV-2 | Tceal1 | 9338 | 4-May-15 |
| 21026 | 2 | 3 | 4 | | IV-2 | Tceal5 | 340543 | 4-May-15 |
| 21027 | 2 | 3 | 4 | | IV-2 | Tceal6 | 158931 | 4-May-15 |
| 21028 | 2 | 3 | 4 | | IV-2 | Tceal7 | 56849 | 4-May-15 |
| 21029 | 2 | 3 | 4 | | IV-2 | Tceal8 | 90843 | 4-May-15 |
| 21039 | 2 | 3 | 4 | | IV-2 | Tcf19 | 6941 | 21-May-15 |
| 21041 | 2 | 3 | 4 | | IV-2 | Tcf21 | 6943 | 28-May-15 |
| 21048 | 2 | 3 | 4 | | IV-2 | Tcf7l1 | 83439 | 4-May-15 |
| 21052 | 2 | 3 | 4 | | IV-2 | Tchhl1 | 126637 | 12-May-15 |
| 21085 | 2 | 3 | 4 | | IV-2 | Tdo2 | 6999 | 4-May-15 |
| 21097 | 2 | 3 | 4 | | IV-2 | Tdrd6 | 221400 | 4-May-15 |
| 21106 | 2 | 3 | 4 | | IV-2 | Tec | 7006 | 7-Jun-15 |
| 21118 | 2 | 3 | 4 | | IV-2 | Tekt2 | 27285 | 12-May-15 |
| 21129 | 2 | 3 | 4 | | IV-2 | Tep1 | 7011 | 7-Jun-15 |
| 21136 | 2 | 3 | 4 | | IV-2 | Tes | 26136 | 4-May-15 |
| 21138 | 2 | 3 | 4 | | IV-2 | Tescl | | |
| 21166 | 2 | 3 | 4 | | IV-2 | Tex33 | 339669 | 4-May-15 |
| 21171 | 2 | 3 | 4 | | IV-2 | Tex40 | 25858 | 4-May-15 |
| 21174 | 2 | 3 | 4 | | IV-2 | Tfam | 7019 | 31-May-15 |
| 21193 | 2 | 3 | 4 | | IV-2 | Tfg | 10342 | 17-May-15 |
| 21197 | 2 | 3 | 4 | | IV-2 | Tfpt | 29844 | 4-May-15 |
| 21201 | 2 | 3 | 4 | | IV-2 | Tgds | 23483 | 4-May-15 |
| 21206 | 2 | 3 | 4 | | IV-2 | Tgfb3 | 7043 | 23-May-15 |
| 21219 | 2 | 3 | 4 | | IV-2 | Tgm4 | 7047 | 21-May-15 |
| 21221 | 2 | 3 | 4 | | IV-2 | Tgm6 | 343641 | 23-May-15 |
| 21225 | 2 | 3 | 4 | | IV-2 | Tgs1 | 96764 | 7-Jun-15 |
| 21227 | 2 | 3 | 4 | | IV-2 | Tgtp2 | | |
| 21228 | 2 | 3 | 4 | | IV-2 | Th | 7054 | 23-May-15 |
| 21243 | 2 | 3 | 4 | | IV-2 | Theg | 51298 | 4-May-15 |
| 21248 | 2 | 3 | 4 | | IV-2 | Themis | 387357 | 12-May-15 |
| 21249 | 2 | 3 | 4 | | IV-2 | Themis2 | 9473 | 4-May-15 |
| 21250 | 2 | 3 | 4 | | IV-2 | Themis3 | | |
| 21279 | 2 | 3 | 4 | | IV-2 | Tiam2 | 26230 | 4-May-15 |
| 21282 | 2 | 3 | 4 | | IV-2 | Ticrr | 90381 | 12-May-15 |
| 21289 | 2 | 3 | 4 | | IV-2 | Tigd5 | 84948 | 4-May-15 |
| 21291 | 2 | 3 | 4 | | IV-2 | Timd2 | | |
| 21310 | 2 | 3 | 4 | | IV-2 | Timp2 | 7077 | 24-May-15 |
| 21325 | 2 | 3 | 4 | | IV-2 | Tk2 | 7084 | 23-May-15 |
| 21327 | 2 | 3 | 4 | | IV-2 | Tktl1 | 8277 | 4-May-15 |
| 21330 | 2 | 3 | 4 | | IV-2 | Tlcd2 | 727910 | 4-May-15 |

Fig.22 - 20

| | | | | | | | | |
|---|---|---|---|---|---|---|---|---|
| 21331 | 2 | 3 | 4 | | IV-2 | Tldc1 | 57707 | 4-May-15 |
| 21333 | 2 | 3 | 4 | | IV-2 | Tle1 | 7088 | 4-May-15 |
| 21341 | 2 | 3 | 4 | | IV-2 | Tll2 | 7093 | 4-May-15 |
| 21345 | 2 | 3 | 4 | | IV-2 | Tlr11 | | |
| 21355 | 2 | 3 | 4 | | IV-2 | Tlr9 | 54106 | 17-May-15 |
| 21356 | 2 | 3 | 4 | | IV-2 | Tlx1 | 3195 | 4-May-15 |
| 21366 | 2 | 3 | 4 | | IV-2 | Tm4sf5 | 9032 | 4-May-15 |
| 21389 | 2 | 3 | 4 | | IV-2 | Tmcc1 | 23023 | 4-May-15 |
| 21391 | 2 | 3 | 4 | | IV-2 | Tmcc3 | 57458 | 4-May-15 |
| 21395 | 2 | 3 | 4 | | IV-2 | Tmco4 | 255104 | 4-May-15 |
| 21427 | 2 | 3 | 4 | | IV-2 | Tmem119 | 338773 | 4-May-15 |
| 21428 | 2 | 3 | 4 | | IV-2 | Tmem120a | 83862 | 4-May-15 |
| 21430 | 2 | 3 | 4 | | IV-2 | Tmem121 | 80757 | 4-May-15 |
| 21462 | 2 | 3 | 4 | | IV-2 | Tmem150cos | | |
| 21472 | 2 | 3 | 4 | | IV-2 | Tmem164 | 84187 | 12-May-15 |
| 21491 | 2 | 3 | 4 | | IV-2 | Tmem179b | 374395 | 4-May-15 |
| 21498 | 2 | 3 | 4 | | IV-2 | Tmem183a | 92703 | 4-May-15 |
| 21501 | 2 | 3 | 4 | | IV-2 | Tmem184c | 55751 | 4-May-15 |
| 21512 | 2 | 3 | 4 | | IV-2 | Tmem198 | 130612 | 12-May-15 |
| 21539 | 2 | 3 | 4 | | IV-2 | Tmem221 | 100130519 | 4-May-15 |
| 21540 | 2 | 3 | 4 | | IV-2 | Tmem222 | 84065 | 4-May-15 |
| 21545 | 2 | 3 | 4 | | IV-2 | Tmem230 | 29058 | 4-May-15 |
| 21549 | 2 | 3 | 4 | | IV-2 | Tmem234 | 56063 | 12-May-15 |
| 21553 | 2 | 3 | 4 | | IV-2 | Tmem238 | 388564 | 4-May-15 |
| 21564 | 2 | 3 | 4 | | IV-2 | Tmem251 | 26175 | 4-May-15 |
| 21570 | 2 | 3 | 4 | | IV-2 | Tmem255a | 55026 | 4-May-15 |
| 21574 | 2 | 3 | 4 | | IV-2 | Tmem259 | 91304 | 4-May-15 |
| 21581 | 2 | 3 | 4 | | IV-2 | Tmem29 | 29057 | 4-May-15 |
| 21589 | 2 | 3 | 4 | | IV-2 | Tmem38b | 55151 | 4-May-15 |
| 21598 | 2 | 3 | 4 | | IV-2 | Tmem45a | 55076 | 12-May-15 |
| 21600 | 2 | 3 | 4 | | IV-2 | Tmem47 | 83604 | 4-May-15 |
| 21607 | 2 | 3 | 4 | | IV-2 | Tmem52b | 120939 | 4-May-15 |
| 21610 | 2 | 3 | 4 | | IV-2 | Tmem55a | 55529 | 4-May-15 |
| 21623 | 2 | 3 | 4 | | IV-2 | Tmem66 | 51669 | 12-May-15 |
| 21633 | 2 | 3 | 4 | | IV-2 | Tmem8 | 58986 | 4-May-15 |
| 21638 | 2 | 3 | 4 | | IV-2 | Tmem86b | 255043 | 4-May-15 |
| 21643 | 2 | 3 | 4 | | IV-2 | Tmem89 | 440955 | 4-May-15 |
| 21659 | 2 | 3 | 4 | | IV-2 | Tmod2 | 29767 | 12-May-15 |
| 21662 | 2 | 3 | 4 | | IV-2 | Tmpo | 7112 | 23-May-15 |
| 21676 | 2 | 3 | 4 | | IV-2 | Tmprss4 | 56649 | 12-May-15 |
| 21682 | 2 | 3 | 4 | | IV-2 | Tmsb15a | 11013 | 4-May-15 |
| 21686 | 2 | 3 | 4 | | IV-2 | Tmsb4x | 7114 | 4-May-15 |
| 21706 | 2 | 3 | 4 | | IV-2 | Tnfaip8l3 | 388121 | 3-May-15 |
| 21713 | 2 | 3 | 4 | | IV-2 | Tnfrsf14 | 8764 | 10-May-15 |
| 21714 | 2 | 3 | 4 | | IV-2 | Tnfrsf17 | 608 | 4-May-15 |
| 21723 | 2 | 3 | 4 | | IV-2 | Tnfrsf26 | | |
| 21733 | 2 | 3 | 4 | | IV-2 | Tnfsf14 | 8740 | 24-May-15 |
| 21750 | 2 | 3 | 4 | | IV-2 | Tnn | 63923 | 4-May-15 |
| 21762 | 2 | 3 | 4 | | IV-2 | Tnpo1 | 3842 | 4-May-15 |
| 21763 | 2 | 3 | 4 | | IV-2 | Tnpo2 | 30000 | 12-May-15 |
| 21767 | 2 | 3 | 4 | | IV-2 | Tnrc6a | 27327 | 4-May-15 |
| 21774 | 2 | 3 | 4 | | IV-2 | Tob1 | 10140 | 4-May-15 |
| 21793 | 2 | 3 | 4 | | IV-2 | Tonsl | 4796 | 4-May-15 |
| 21797 | 2 | 3 | 4 | | IV-2 | Top2b | 7155 | 17-May-15 |
| 21805 | 2 | 3 | 4 | | IV-2 | Tor1a | 1861 | 23-May-15 |
| 21815 | 2 | 3 | 4 | | IV-2 | Tox4 | 9878 | 4-May-15 |
| 21817 | 2 | 3 | 4 | | IV-2 | Tpbpa | | |
| 21832 | 2 | 3 | 4 | | IV-2 | Tpm3 | 7170 | 23-May-15 |
| 21835 | 2 | 3 | 4 | | IV-2 | Tpo | 7173 | 12-May-15 |
| 21841 | 2 | 3 | 4 | | IV-2 | Tpr | 7175 | 17-May-15 |
| 21855 | 2 | 3 | 4 | | IV-2 | Tra2a | 29896 | 2-Jun-15 |
| 21859 | 2 | 3 | 4 | | IV-2 | Tradd | 8717 | 4-May-15 |
| 21878 | 2 | 3 | 4 | | IV-2 | Trap1 | 10131 | 7-Jun-15 |
| 21892 | 2 | 3 | 4 | | IV-2 | Trappc6b | 122553 | 4-May-15 |
| 21899 | 2 | 3 | 4 | | IV-2 | Treh | 11181 | 12-May-15 |
| 21904 | 2 | 3 | 4 | | IV-2 | Treml2 | 79865 | 4-May-15 |
| 21905 | 2 | 3 | 4 | | IV-2 | Treml4 | 285852 | 4-May-15 |
| 21920 | 2 | 3 | 4 | | IV-2 | Trim11 | 81559 | 4-May-15 |
| 21924 | 2 | 3 | 4 | | IV-2 | Trim14 | 9830 | 12-May-15 |
| 21925 | 2 | 3 | 4 | | IV-2 | Trim15 | 89870 | 7-Jun-15 |
| 21947 | 2 | 3 | 4 | | IV-2 | Trim35 | 23087 | 12-May-15 |
| 21951 | 2 | 3 | 4 | | IV-2 | Trim39 | 56658 | 2-Jun-15 |
| 21953 | 2 | 3 | 4 | | IV-2 | Trim41 | 90933 | 4-May-15 |
| 21968 | 2 | 3 | 4 | | IV-2 | Trim59 | 286827 | 4-May-15 |
| 21969 | 2 | 3 | 4 | | IV-2 | Trim6 | 117854 | 4-May-15 |
| 21974 | 2 | 3 | 4 | | IV-2 | Trim65 | 201292 | 12-May-15 |
| 21977 | 2 | 3 | 4 | | IV-2 | Trim68 | 55128 | 4-May-15 |
| 21980 | 2 | 3 | 4 | | IV-2 | Trim71 | 131405 | 7-Jun-15 |
| 21982 | 2 | 3 | 4 | | IV-2 | Trim75 | 391714 | 4-May-15 |
| 21991 | 2 | 3 | 4 | | IV-2 | Trip12 | 9320 | 23-May-15 |
| 21993 | 2 | 3 | 4 | | IV-2 | Trip4 | 9325 | 4-May-15 |
| 22013 | 2 | 3 | 4 | | IV-2 | Trmu | 55687 | 23-May-15 |
| 22019 | 2 | 3 | 4 | | IV-2 | Trove2 | 6738 | 4-May-15 |
| 22025 | 2 | 3 | 4 | | IV-2 | Trp53i13 | | |
| 22028 | 2 | 3 | 4 | | IV-2 | Trp53rk | | |
| 22041 | 2 | 3 | 4 | | IV-2 | Trpc7 | 57113 | 7-Jun-15 |
| 22044 | 2 | 3 | 4 | | IV-2 | Trpm2 | 7226 | 7-Jun-15 |
| 22053 | 2 | 3 | 4 | | IV-2 | Trpv1 | 7442 | 17-May-15 |
| 22055 | 2 | 3 | 4 | | IV-2 | Trpv3 | 162514 | 7-Jun-15 |
| 22066 | 2 | 3 | 4 | | IV-2 | Tsc1 | 7248 | 13-Jun-15 |
| 22069 | 2 | 3 | 4 | | IV-2 | Tsc22d2 | 9819 | 4-May-15 |
| 22083 | 2 | 3 | 4 | | IV-2 | Tshz1 | 10194 | 12-May-15 |
| 22085 | 2 | 3 | 4 | | IV-2 | Tshz3 | 57616 | 4-May-15 |
| 22090 | 2 | 3 | 4 | | IV-2 | Tsn | 7247 | 3-May-15 |
| 22101 | 2 | 3 | 4 | | IV-2 | Tspan18 | 90139 | 4-May-15 |
| 22111 | 2 | 3 | 4 | | IV-2 | Tspan7 | 7102 | 23-May-15 |
| 22122 | 2 | 3 | 4 | | IV-2 | Tspy-ps | | |
| 22139 | 2 | 3 | 4 | | IV-2 | Tsx | | |
| 22157 | 2 | 3 | 4 | | IV-2 | Ttc26 | 79989 | 12-May-15 |
| 22174 | 2 | 3 | 4 | | IV-2 | Ttc39d | | |
| 22188 | 2 | 3 | 4 | | IV-2 | Ttl | 150465 | 7-Jun-15 |
| 22197 | 2 | 3 | 4 | | IV-2 | Ttll5 | 23093 | 7-Jun-15 |
| 22200 | 2 | 3 | 4 | | IV-2 | Ttll8 | 164714 | 4-May-15 |
| 22211 | 2 | 3 | 4 | | IV-2 | Tuba1b | 10376 | 4-May-15 |
| 22213 | 2 | 3 | 4 | | IV-2 | Tuba3a | | |
| 22217 | 2 | 3 | 4 | | IV-2 | Tubal3 | 79861 | 7-Jun-15 |
| 22238 | 2 | 3 | 4 | | IV-2 | Tug1 | 55000 | 12-May-15 |
| 22247 | 2 | 3 | 4 | | IV-2 | Tusc5 | 286753 | 4-May-15 |
| 22248 | 2 | 3 | 4 | | IV-2 | Tut1 | 64852 | 4-May-15 |
| 22255 | 2 | 3 | 4 | | IV-2 | Twistnb | 221830 | 12-May-15 |
| 22260 | 2 | 3 | 4 | | IV-2 | Txlng | 55787 | 4-May-15 |
| 22281 | 2 | 3 | 4 | | IV-2 | Tyms | 7298 | 17-May-15 |
| 22282 | 2 | 3 | 4 | | IV-2 | Tyms-ps | | |
| 22283 | 2 | 3 | 4 | | IV-2 | Tyr | 7299 | 7-Jun-15 |
| 22284 | 2 | 3 | 4 | | IV-2 | Tyro3 | 7301 | 4-May-15 |
| 22286 | 2 | 3 | 4 | | IV-2 | Tyrp1 | 7306 | 7-Jun-15 |
| 22287 | 2 | 3 | 4 | | IV-2 | Tysnd1 | 219743 | 21-May-15 |
| 22290 | 2 | 3 | 4 | | IV-2 | Tyw5 | 129450 | 4-May-15 |
| 22311 | 2 | 3 | 4 | | IV-2 | Ubap1 | 51271 | 12-May-15 |
| 22320 | 2 | 3 | 4 | | IV-2 | Ube2a | 7319 | 2-Jun-15 |
| 22323 | 2 | 3 | 4 | | IV-2 | Ube2cbp | 90025 | 4-May-15 |
| 22358 | 2 | 3 | 4 | | IV-2 | Ube3b | 89910 | 4-May-15 |
| 22364 | 2 | 3 | 4 | | IV-2 | Ubl3 | 5412 | 4-May-15 |
| 22407 | 2 | 3 | 4 | | IV-2 | Ucma | 221044 | 4-May-15 |
| 22412 | 2 | 3 | 4 | | IV-2 | Ucp2 | 7351 | 12-May-15 |
| 22414 | 2 | 3 | 4 | | IV-2 | Uevld | 55293 | 4-May-15 |
| 22437 | 2 | 3 | 4 | | IV-2 | Ugt2b1 | | |
| 22444 | 2 | 3 | 4 | | IV-2 | Ugt3a1 | 133688 | 28-May-15 |
| 22445 | 2 | 3 | 4 | | IV-2 | Ugt3a2 | 167127 | 28-May-15 |
| 22449 | 2 | 3 | 4 | | IV-2 | Uhrf1bp1 | 54887 | 4-May-15 |
| 22454 | 2 | 3 | 4 | | IV-2 | Ulk1 | 8408 | 24-May-15 |
| 22462 | 2 | 3 | 4 | | IV-2 | Unc119b | 84747 | 4-May-15 |
| 22469 | 2 | 3 | 4 | | IV-2 | Unc50 | 25972 | 4-May-15 |
| 22481 | 2 | 3 | 4 | | IV-2 | Unk | 85451 | 21-May-15 |
| 22484 | 2 | 3 | 4 | | IV-2 | Upb1 | 51733 | 17-May-15 |
| 22494 | 2 | 3 | 4 | | IV-2 | Upk3bl | 100134938 | 4-May-15 |
| 22496 | 2 | 3 | 4 | | IV-2 | Upp2 | 151531 | 21-May-15 |
| 22498 | 2 | 3 | 4 | | IV-2 | Uqcc1 | 55245 | 4-May-15 |
| 22527 | 2 | 3 | 4 | | IV-2 | Uso1 | 8615 | 12-May-15 |
| 22533 | 2 | 3 | 4 | | IV-2 | Usp14 | 9097 | 4-May-15 |
| 22590 | 2 | 3 | 4 | | IV-2 | Utp15 | 84135 | 4-May-15 |
| 22618 | 2 | 3 | 4 | | IV-2 | Vapa | 9218 | 4-May-15 |
| 22626 | 2 | 3 | 4 | | IV-2 | Vat1 | 10493 | 13-Jun-15 |
| 22630 | 2 | 3 | 4 | | IV-2 | Vav2 | 7410 | 4-May-15 |
| 22637 | 2 | 3 | 4 | | IV-2 | Vcan | 1462 | 23-May-15 |
| 22638 | 2 | 3 | 4 | | IV-2 | Vcl | 7414 | 28-May-15 |
| 22649 | 2 | 3 | 4 | | IV-2 | Veph1 | 79674 | 4-May-15 |
| 22655 | 2 | 3 | 4 | | IV-2 | Vgll3 | 389136 | 4-May-15 |
| 22656 | 2 | 3 | 4 | | IV-2 | Vgll4 | 9686 | 12-May-15 |
| 22657 | 2 | 3 | 4 | | IV-2 | Vhl | 7428 | 31-May-15 |
| 22660 | 2 | 3 | 4 | | IV-2 | Vim | 7431 | 17-May-15 |
| 23013 | 2 | 3 | 4 | | IV-2 | Vps11 | 55823 | 12-May-15 |
| 23044 | 2 | 3 | 4 | | IV-2 | Vps9d1 | 9605 | 4-May-15 |
| 23055 | 2 | 3 | 4 | | IV-2 | Vsnl1 | 7447 | 4-May-15 |
| 23068 | 2 | 3 | 4 | | IV-2 | Vwa1 | 64856 | 4-May-15 |
| 23075 | 2 | 3 | 4 | | IV-2 | Vwa8 | 23078 | 4-May-15 |
| 23088 | 2 | 3 | 4 | | IV-2 | Wasf1 | 8936 | 4-May-15 |
| 23091 | 2 | 3 | 4 | | IV-2 | Wash | 100287171 | 7-Jun-15 |
| 23108 | 2 | 3 | 4 | | IV-2 | Wdfy3 | 23001 | 21-May-15 |
| 23159 | 2 | 3 | 4 | | IV-2 | Wdr72 | 256764 | 4-May-15 |
| 23210 | 2 | 3 | 4 | | IV-2 | Wipf1 | 7456 | 4-May-15 |
| 23217 | 2 | 3 | 4 | | IV-2 | Wisp3 | 8838 | 4-May-15 |
| 23227 | 2 | 3 | 4 | | IV-2 | Wnt11 | 7481 | 12-May-15 |
| 23231 | 2 | 3 | 4 | | IV-2 | Wnt3 | 7473 | 23-May-15 |
| 23265 | 2 | 3 | 4 | | IV-2 | Xcr1 | 2829 | 4-May-15 |
| 23270 | 2 | 3 | 4 | | IV-2 | Xist | 7503 | 7-Jun-15 |
| 23274 | 2 | 3 | 4 | | IV-2 | Xkr6 | 286046 | 4-May-15 |
| 23279 | 2 | 3 | 4 | | IV-2 | Xlr | | |
| 23282 | 2 | 3 | 4 | | IV-2 | Xlr3c | | |
| 23298 | 2 | 3 | 4 | | IV-2 | Xpo5 | 57510 | 4-May-15 |
| 23311 | 2 | 3 | 4 | | IV-2 | Xrn2 | 22803 | 4-May-15 |
| 23316 | 2 | 3 | 4 | | IV-2 | Xylt2 | 64132 | 31-May-15 |
| 23339 | 2 | 3 | 4 | | IV-2 | Ykt6 | 10652 | 23-May-15 |
| 23343 | 2 | 3 | 4 | | IV-2 | Ypel1 | 29799 | 2-Jun-15 |
| 23347 | 2 | 3 | 4 | | IV-2 | Ypel5 | 51646 | 12-May-15 |
| 23380 | 2 | 3 | 4 | | IV-2 | Zbtb17 | 7709 | 3-May-15 |
| 23384 | 2 | 3 | 4 | | IV-2 | Zbtb21 | 49854 | 28-May-15 |
| 23391 | 2 | 3 | 4 | | IV-2 | Zbtb33 | 10009 | 12-May-15 |
| 23395 | 2 | 3 | 4 | | IV-2 | Zbtb39 | 9880 | 4-May-15 |
| 23404 | 2 | 3 | 4 | | IV-2 | Zbtb48 | 3104 | 4-May-15 |
| 23414 | 2 | 3 | 4 | | IV-2 | Zbtb9 | 221504 | 4-May-15 |
| 23416 | 2 | 3 | 4 | | IV-2 | Zc2hc1a | 51101 | 4-May-15 |
| 23425 | 2 | 3 | 4 | | IV-2 | Zc3h13 | 23091 | 12-May-15 |
| 23459 | 2 | 3 | 4 | | IV-2 | Zdhhc1 | 29800 | 4-May-15 |
| 23470 | 2 | 3 | 4 | | IV-2 | Zdhhc20 | 253832 | 12-May-15 |
| 23474 | 2 | 3 | 4 | | IV-2 | Zdhhc24 | 254359 | 4-May-15 |
| 23485 | 2 | 3 | 4 | | IV-2 | Zeb2os | | |
| 23488 | 2 | 3 | 4 | | IV-2 | Zfand1 | 79752 | 4-May-15 |
| 23523 | 2 | 3 | 4 | | IV-2 | Zfp14 | 57677 | 4-May-15 |
| 23531 | 2 | 3 | 4 | | IV-2 | Zfp174 | | |
| 23597 | 2 | 3 | 4 | | IV-2 | Zfp367 | | |
| 23599 | 2 | 3 | 4 | | IV-2 | Zfp36l1 | 677 | 4-May-15 |
| 23604 | 2 | 3 | 4 | | IV-2 | Zfp383 | | |

Fig.22 - 21

| | | | | | | | | |
|---|---|---|---|---|---|---|---|---|
| 23608 | 2 | 3 | 4 | | IV-2 | Zfp385c | | |
| 23615 | 2 | 3 | 4 | | IV-2 | Zfp40 | | |
| 23620 | 2 | 3 | 4 | | IV-2 | Zfp414 | 84330 | 4-May-15 |
| 23632 | 2 | 3 | 4 | | IV-2 | Zfp444 | | |
| 23659 | 2 | 3 | 4 | | IV-2 | Zfp518b | | |
| 23660 | 2 | 3 | 4 | | IV-2 | Zfp52 | | |
| 23665 | 2 | 3 | 4 | | IV-2 | Zfp53 | | |
| 23669 | 2 | 3 | 4 | | IV-2 | Zfp54 | | |
| 23672 | 2 | 3 | 4 | | IV-2 | Zfp553 | | |
| 23686 | 2 | 3 | 4 | | IV-2 | Zfp59 | | |
| 23698 | 2 | 3 | 4 | | IV-2 | Zfp608 | | |
| 23715 | 2 | 3 | 4 | | IV-2 | Zfp644 | | |
| 23722 | 2 | 3 | 4 | | IV-2 | Zfp652os | | |
| 23737 | 2 | 3 | 4 | | IV-2 | Zfp689 | | |
| 23792 | 2 | 3 | 4 | | IV-2 | Zfp810 | | |
| 23797 | 2 | 3 | 4 | | IV-2 | Zfp821 | | |
| 23816 | 2 | 3 | 4 | | IV-2 | Zfp871 | | |
| 23818 | 2 | 3 | 4 | | IV-2 | Zfp873 | | |
| 23819 | 2 | 3 | 4 | | IV-2 | Zfp874a | | |
| 23830 | 2 | 3 | 4 | | IV-2 | Zfp931 | | |
| 23831 | 2 | 3 | 4 | | IV-2 | Zfp932 | | |
| 23837 | 2 | 3 | 4 | | IV-2 | Zfp938 | | |
| 23883 | 2 | 3 | 4 | | IV-2 | Zglp1 | 100125288 | 4-May-15 |
| 23886 | 2 | 3 | 4 | | IV-2 | Zhx1 | 11244 | 4-May-15 |
| 23907 | 2 | 3 | 4 | | IV-2 | Zkscan8 | 7745 | 14-May-15 |
| 23936 | 2 | 3 | 4 | | IV-2 | Znrd1as | 80862 | 12-May-15 |
| 23938 | 2 | 3 | 4 | | IV-2 | Znrf2 | 223082 | 23-May-15 |
| 23983 | 2 | 3 | 4 | | IV-2 | Zwint | 11130 | 4-May-15 |
| 1 | 2 | 3 | 4 | | IV-1 | 0610005C13Rik | | |
| 9 | 2 | 3 | 4 | | IV-1 | 0610011F06Rik | | |
| 17 | 2 | 3 | 4 | | IV-1 | 0610040B10Rik | | |
| 20 | 2 | 3 | 4 | | IV-1 | 0610043K17Rik | | |
| 22 | 2 | 3 | 4 | | IV-1 | 1100001G20Rik | | |
| 29 | 2 | 3 | 4 | | IV-1 | 1110008F13Rik | | |
| 45 | 2 | 3 | 4 | | IV-1 | 1110038B12Rik | | |
| 47 | 2 | 3 | 4 | | IV-1 | 1110046J04Rik | | |
| 55 | 2 | 3 | 4 | | IV-1 | 1190002F15Rik | | |
| 58 | 2 | 3 | 4 | | IV-1 | 1190005I06Rik | | |
| 62 | 2 | 3 | 4 | | IV-1 | 1300002K09Rik | | |
| 63 | 2 | 3 | 4 | | IV-1 | 1300017J02Rik | | |
| 66 | 2 | 3 | 4 | | IV-1 | 1500009L16Rik | | |
| 67 | 2 | 3 | 4 | | IV-1 | 1500011B03Rik | | |
| 74 | 2 | 3 | 4 | | IV-1 | 1500017E21Rik | | |
| 78 | 2 | 3 | 4 | | IV-1 | 1600010M07Rik | | |
| 81 | 2 | 3 | 4 | | IV-1 | 1600014C23Rik | | |
| 84 | 2 | 3 | 4 | | IV-1 | 1600016N20Rik | | |
| 86 | 2 | 3 | 4 | | IV-1 | 1600020E01Rik | | |
| 92 | 2 | 3 | 4 | | IV-1 | 1700001C02Rik | | |
| 97 | 2 | 3 | 4 | | IV-1 | 1700001G17Rik | | |
| 98 | 2 | 3 | 4 | | IV-1 | 1700001J03Rik | | |
| 104 | 2 | 3 | 4 | | IV-1 | 1700001O22Rik | | |
| 107 | 2 | 3 | 4 | | IV-1 | 1700003D09Rik | | |
| 108 | 2 | 3 | 4 | | IV-1 | 1700003E16Rik | | |
| 110 | 2 | 3 | 4 | | IV-1 | 1700003F12Rik | | |
| 127 | 2 | 3 | 4 | | IV-1 | 1700007K13Rik | | |
| 137 | 2 | 3 | 4 | | IV-1 | 1700009J07Rik | | |
| 139 | 2 | 3 | 4 | | IV-1 | 1700009P17Rik | | |
| 142 | 2 | 3 | 4 | | IV-1 | 1700010I02Rik | | |
| 149 | 2 | 3 | 4 | | IV-1 | 1700011H14Rik | | |
| 153 | 2 | 3 | 4 | | IV-1 | 1700012A03Rik | | |
| 155 | 2 | 3 | 4 | | IV-1 | 1700012B09Rik | | |
| 156 | 2 | 3 | 4 | | IV-1 | 1700012D01Rik | | |
| 157 | 2 | 3 | 4 | | IV-1 | 1700012D14Rik | | |
| 172 | 2 | 3 | 4 | | IV-1 | 1700016K19Rik | | |
| 174 | 2 | 3 | 4 | | IV-1 | 1700016L21Rik | | |
| 191 | 2 | 3 | 4 | | IV-1 | 1700019D03Rik | | |
| 203 | 2 | 3 | 4 | | IV-1 | 1700020L24Rik | | |
| 221 | 2 | 3 | 4 | | IV-1 | 1700023L04Rik | | |
| 226 | 2 | 3 | 4 | | IV-1 | 1700024P16Rik | | |
| 235 | 2 | 3 | 4 | | IV-1 | 1700026D08Rik | | |
| 238 | 2 | 3 | 4 | | IV-1 | 1700026L06Rik | | |
| 244 | 2 | 3 | 4 | | IV-1 | 1700028B04Rik | | |
| 258 | 2 | 3 | 4 | | IV-1 | 1700029J07Rik | | |
| 262 | 2 | 3 | 4 | | IV-1 | 1700030A11Rik | | |
| 263 | 2 | 3 | 4 | | IV-1 | 1700030C10Rik | | |
| 286 | 2 | 3 | 4 | | IV-1 | 1700037C18Rik | | |
| 291 | 2 | 3 | 4 | | IV-1 | 1700041C23Rik | | |
| 304 | 2 | 3 | 4 | | IV-1 | 1700047I17Rik2 | | |
| 348 | 2 | 3 | 4 | | IV-1 | 1700067K01Rik | | |
| 353 | 2 | 3 | 4 | | IV-1 | 1700071M16Rik | | |
| 363 | 2 | 3 | 4 | | IV-1 | 1700084C01Rik | | |
| 364 | 2 | 3 | 4 | | IV-1 | 1700084E18Rik | | |
| 372 | 2 | 3 | 4 | | IV-1 | 1700092C02Rik | | |
| 376 | 2 | 3 | 4 | | IV-1 | 1700092M07Rik | | |
| 388 | 2 | 3 | 4 | | IV-1 | 1700101I11Rik | | |
| 404 | 2 | 3 | 4 | | IV-1 | 1700110K17Rik | | |
| 406 | 2 | 3 | 4 | | IV-1 | 1700112E06Rik | | |
| 411 | 2 | 3 | 4 | | IV-1 | 1700119H24Rik | | |
| 412 | 2 | 3 | 4 | | IV-1 | 1700120C14Rik | | |
| 415 | 2 | 3 | 4 | | IV-1 | 1700120K04Rik | | |
| 426 | 2 | 3 | 4 | | IV-1 | 1700124L16Rik | | |
| 441 | 2 | 3 | 4 | | IV-1 | 1810010D01Rik | | |
| 442 | 2 | 3 | 4 | | IV-1 | 1810010H24Rik | | |
| 443 | 2 | 3 | 4 | | IV-1 | 1810011H11Rik | | |
| 444 | 2 | 3 | 4 | | IV-1 | 1810011O10Rik | | |
| 445 | 2 | 3 | 4 | | IV-1 | 1810012K16Rik | | |
| 452 | 2 | 3 | 4 | | IV-1 | 1810021B22Rik | | |

| | | | | | | | | |
|---|---|---|---|---|---|---|---|---|
| 453 | 2 | 3 | 4 | | IV-1 | 1810022K09Rik | | |
| 458 | 2 | 3 | 4 | | IV-1 | 1810032O08Rik | | |
| 464 | 2 | 3 | 4 | | IV-1 | 1810044D09Rik | | |
| 465 | 2 | 3 | 4 | | IV-1 | 1810046K07Rik | | |
| 469 | 2 | 3 | 4 | | IV-1 | 1810062G17Rik | | |
| 471 | 2 | 3 | 4 | | IV-1 | 1810064F22Rik | | |
| 475 | 2 | 3 | 4 | | IV-1 | 2010003K11Rik | | |
| 478 | 2 | 3 | 4 | | IV-1 | 2010010A06Rik | | |
| 481 | 2 | 3 | 4 | | IV-1 | 2010016I18Rik | | |
| 485 | 2 | 3 | 4 | | IV-1 | 2010107G12Rik | | |
| 486 | 2 | 3 | 4 | | IV-1 | 2010107G23Rik | | |
| 488 | 2 | 3 | 4 | | IV-1 | 2010109I03Rik | | |
| 490 | 2 | 3 | 4 | | IV-1 | 2010204K13Rik | | |
| 491 | 2 | 3 | 4 | | IV-1 | 2010300C02Rik | | |
| 496 | 2 | 3 | 4 | | IV-1 | 2200002D01Rik | | |
| 506 | 2 | 3 | 4 | | IV-1 | 2210039B01Rik | | |
| 508 | 2 | 3 | 4 | | IV-1 | 2210407C18Rik | | |
| 509 | 2 | 3 | 4 | | IV-1 | 2210408F21Rik | | |
| 511 | 2 | 3 | 4 | | IV-1 | 2210409D07Rik | | |
| 519 | 2 | 3 | 4 | | IV-1 | 2300005B03Rik | | |
| 522 | 2 | 3 | 4 | | IV-1 | 2310001K24Rik | | |
| 525 | 2 | 3 | 4 | | IV-1 | 2310002J15Rik | | |
| 535 | 2 | 3 | 4 | | IV-1 | 2310009B15Rik | | |
| 543 | 2 | 3 | 4 | | IV-1 | 2310020H05Rik | | |
| 547 | 2 | 3 | 4 | | IV-1 | 2310030G06Rik | | |
| 550 | 2 | 3 | 4 | | IV-1 | 2310034G01Rik | | |
| 551 | 2 | 3 | 4 | | IV-1 | 2310034O05Rik | | |
| 556 | 2 | 3 | 4 | | IV-1 | 2310040G24Rik | | |
| 557 | 2 | 3 | 4 | | IV-1 | 2310042E22Rik | | |
| 569 | 2 | 3 | 4 | | IV-1 | 2310065F04Rik | | |
| 575 | 2 | 3 | 4 | | IV-1 | 2310081J21Rik | | |
| 580 | 2 | 3 | 4 | | IV-1 | 2410004N09Rik | | |
| 581 | 2 | 3 | 4 | | IV-1 | 2410004P03Rik | | |
| 582 | 2 | 3 | 4 | | IV-1 | 2410006H16Rik | | |
| 612 | 2 | 3 | 4 | | IV-1 | 2610016A17Rik | | |
| 626 | 2 | 3 | 4 | | IV-1 | 2610203C20Rik | | |
| 638 | 2 | 3 | 4 | | IV-1 | 2610524H06Rik | | |
| 640 | 2 | 3 | 4 | | IV-1 | 2610528J11Rik | | |
| 642 | 2 | 3 | 4 | | IV-1 | 2700038G22Rik | | |
| 656 | 2 | 3 | 4 | | IV-1 | 2700097O09Rik | | |
| 663 | 2 | 3 | 4 | | IV-1 | 2810008D09Rik | | |
| 668 | 2 | 3 | 4 | | IV-1 | 2810032G03Rik | | |
| 676 | 2 | 3 | 4 | | IV-1 | 2810408A11Rik | | |
| 688 | 2 | 3 | 4 | | IV-1 | 2810468N07Rik | | |
| 691 | 2 | 3 | 4 | | IV-1 | 2900005J15Rik | | |
| 696 | 2 | 3 | 4 | | IV-1 | 2900041M22Rik | | |
| 702 | 2 | 3 | 4 | | IV-1 | 2900076A07Rik | | |
| 707 | 2 | 3 | 4 | | IV-1 | 3000002C10Rik | | |
| 715 | 2 | 3 | 4 | | IV-1 | 3110009E18Rik | | |
| 719 | 2 | 3 | 4 | | IV-1 | 3110021N24Rik | | |
| 737 | 2 | 3 | 4 | | IV-1 | 3300005D01Rik | | |
| 760 | 2 | 3 | 4 | | IV-1 | 4833411C07Rik | | |
| 763 | 2 | 3 | 4 | | IV-1 | 4833418N02Rik | | |
| 764 | 2 | 3 | 4 | | IV-1 | 4833419F23Rik | | |
| 767 | 2 | 3 | 4 | | IV-1 | 4833423E24Rik | | |
| 785 | 2 | 3 | 4 | | IV-1 | 4921511I17Rik | | |
| 797 | 2 | 3 | 4 | | IV-1 | 4921533I20Rik | | |
| 815 | 2 | 3 | 4 | | IV-1 | 4930404N11Rik | | |
| 842 | 2 | 3 | 4 | | IV-1 | 4930426D05Rik | | |
| 852 | 2 | 3 | 4 | | IV-1 | 4930429F24Rik | | |
| 873 | 2 | 3 | 4 | | IV-1 | 4930442L01Rik | | |
| 881 | 2 | 3 | 4 | | IV-1 | 4930447J18Rik | | |
| 896 | 2 | 3 | 4 | | IV-1 | 4930452A19Rik | | |
| 904 | 2 | 3 | 4 | | IV-1 | 4930455C13Rik | | |
| 912 | 2 | 3 | 4 | | IV-1 | 4930461G14Rik | | |
| 930 | 2 | 3 | 4 | | IV-1 | 4930474M22Rik | | |
| 940 | 2 | 3 | 4 | | IV-1 | 4930481A15Rik | | |
| 948 | 2 | 3 | 4 | | IV-1 | 4930487D11Rik | | |
| 949 | 2 | 3 | 4 | | IV-1 | 4930487H11Rik | | |
| 958 | 2 | 3 | 4 | | IV-1 | 4930503B20Rik | | |
| 967 | 2 | 3 | 4 | | IV-1 | 4930506C21Rik | | |
| 968 | 2 | 3 | 4 | | IV-1 | 4930506M07Rik | | |
| 983 | 2 | 3 | 4 | | IV-1 | 4930515G16Rik | | |
| 994 | 2 | 3 | 4 | | IV-1 | 4930520O04Rik | | |
| 1018 | 2 | 3 | 4 | | IV-1 | 4930529L06Rik | | |
| 1026 | 2 | 3 | 4 | | IV-1 | 4930539J05Rik | | |
| 1068 | 2 | 3 | 4 | | IV-1 | 4930562C15Rik | | |
| 1070 | 2 | 3 | 4 | | IV-1 | 4930563D23Rik | | |
| 1104 | 2 | 3 | 4 | | IV-1 | 4930581F22Rik | | |
| 1145 | 2 | 3 | 4 | | IV-1 | 4931431C16Rik | | |
| 1164 | 2 | 3 | 4 | | IV-1 | 4932438H23Rik | | |
| 1184 | 2 | 3 | 4 | | IV-1 | 4933402P03Rik | | |
| 1188 | 2 | 3 | 4 | | IV-1 | 4933404O12Rik | | |
| 1193 | 2 | 3 | 4 | | IV-1 | 4933406C10Rik | | |
| 1223 | 2 | 3 | 4 | | IV-1 | 4933415F23Rik | | |
| 1230 | 2 | 3 | 4 | | IV-1 | 4933417D19Rik | | |
| 1248 | 2 | 3 | 4 | | IV-1 | 4933427I22Rik | | |
| 1288 | 2 | 3 | 4 | | IV-1 | 5033403H07Rik | | |
| 1297 | 2 | 3 | 4 | | IV-1 | 5330439B14Rik | | |
| 1302 | 2 | 3 | 4 | | IV-1 | 5430405H02Rik | | |
| 1303 | 2 | 3 | 4 | | IV-1 | 5430416N02Rik | | |
| 1308 | 2 | 3 | 4 | | IV-1 | 5430421N21Rik | | |
| 1310 | 2 | 3 | 4 | | IV-1 | 5430427M07Rik | | |
| 1314 | 2 | 3 | 4 | | IV-1 | 5430435G22Rik | | |
| 1322 | 2 | 3 | 4 | | IV-1 | 5730408K05Rik | | |
| 1325 | 2 | 3 | 4 | | IV-1 | 5730416F02Rik | | |
| 1334 | 2 | 3 | 4 | | IV-1 | 5730507C01Rik | | |

Fig.22 - 22

| | | | | | | | | |
|---|---|---|---|---|---|---|---|---|
| 1337 | 2 | 3 | 4 | | IV-1 | 5730559C18Rik | | |
| 1346 | 2 | 3 | 4 | | IV-1 | 5830428M24Rik | | |
| 1351 | 2 | 3 | 4 | | IV-1 | 5930403L14Rik | | |
| 1357 | 2 | 3 | 4 | | IV-1 | 6030419C18Rik | | |
| 1362 | 2 | 3 | 4 | | IV-1 | 6030468B19Rik | | |
| 1374 | 2 | 3 | 4 | | IV-1 | 6330418K02Rik | | |
| 1383 | 2 | 3 | 4 | | IV-1 | 6430571L13Rik | | |
| 1390 | 2 | 3 | 4 | | IV-1 | 6720416L17Rik | | |
| 1409 | 2 | 3 | 4 | | IV-1 | 8430419L09Rik | | |
| 1445 | 2 | 3 | 4 | | IV-1 | 9230102O04Rik | | |
| 1447 | 2 | 3 | 4 | | IV-1 | 9230105E05Rik | | |
| 1448 | 2 | 3 | 4 | | IV-1 | 9230110C19Rik | | |
| 1453 | 2 | 3 | 4 | | IV-1 | 9230116L04Rik | | |
| 1459 | 2 | 3 | 4 | | IV-1 | 9330133O14Rik | | |
| 1466 | 2 | 3 | 4 | | IV-1 | 9330175E14Rik | | |
| 1490 | 2 | 3 | 4 | | IV-1 | 9530002B09Rik | | |
| 1493 | 2 | 3 | 4 | | IV-1 | 9530026P05Rik | | |
| 1497 | 2 | 3 | 4 | | IV-1 | 9530052E02Rik | | |
| 1502 | 2 | 3 | 4 | | IV-1 | 9530080O11Rik | | |
| 1514 | 2 | 3 | 4 | | IV-1 | 9930012K11Rik | | |
| 1541 | 2 | 3 | 4 | | IV-1 | A230103J11Rik | | |
| 1558 | 2 | 3 | 4 | | IV-1 | A330076C08Rik | | |
| 1573 | 2 | 3 | 4 | | IV-1 | A4galt | 53947 | 12-May-15 |
| 1576 | 2 | 3 | 4 | | IV-1 | A530013C23Rik | | |
| 1595 | 2 | 3 | 4 | | IV-1 | A630023A22Rik | | |
| 1598 | 2 | 3 | 4 | | IV-1 | A630066F11Rik | | |
| 1600 | 2 | 3 | 4 | | IV-1 | A630073D07Rik | | |
| 1609 | 2 | 3 | 4 | | IV-1 | A730017C20Rik | | |
| 1613 | 2 | 3 | 4 | | IV-1 | A730020M07Rik | | |
| 1614 | 2 | 3 | 4 | | IV-1 | A730036I17Rik | | |
| 1645 | 2 | 3 | 4 | | IV-1 | A930015D03Rik | | |
| 1654 | 2 | 3 | 4 | | IV-1 | AA413626 | | |
| 1655 | 2 | 3 | 4 | | IV-1 | AA414768 | | |
| 1657 | 2 | 3 | 4 | | IV-1 | AA465934 | | |
| 1658 | 2 | 3 | 4 | | IV-1 | AA467197 | | |
| 1659 | 2 | 3 | 4 | | IV-1 | AA474331 | | |
| 1666 | 2 | 3 | 4 | | IV-1 | AA986860 | | |
| 1670 | 2 | 3 | 4 | | IV-1 | Aadac | 13 | 4-May-15 |
| 1687 | 2 | 3 | 4 | | IV-1 | Aass | 10157 | 4-May-15 |
| 1691 | 2 | 3 | 4 | | IV-1 | AB124611 | | |
| 1700 | 2 | 3 | 4 | | IV-1 | Abca2 | 20 | 12-May-15 |
| 1704 | 2 | 3 | 4 | | IV-1 | Abca6 | 23460 | 12-May-15 |
| 1706 | 2 | 3 | 4 | | IV-1 | Abca8a | | |
| 1709 | 2 | 3 | 4 | | IV-1 | Abcb10 | 23456 | 12-May-15 |
| 1711 | 2 | 3 | 4 | | IV-1 | Abcb1a | | |
| 1713 | 2 | 3 | 4 | | IV-1 | Abcb4 | 5244 | 12-May-15 |
| 1714 | 2 | 3 | 4 | | IV-1 | Abcb5 | 340273 | 4-May-15 |
| 1722 | 2 | 3 | 4 | | IV-1 | Abcc2 | 1244 | 17-May-15 |
| 1723 | 2 | 3 | 4 | | IV-1 | Abcc3 | 8714 | 17-May-15 |
| 1724 | 2 | 3 | 4 | | IV-1 | Abcc4 | 10257 | 12-May-15 |
| 1728 | 2 | 3 | 4 | | IV-1 | Abcc9 | 10060 | 23-May-15 |
| 1730 | 2 | 3 | 4 | | IV-1 | Abcd2 | 225 | 7-Jun-15 |
| 1741 | 2 | 3 | 4 | | IV-1 | Abcg5 | 64240 | 23-May-15 |
| 1746 | 2 | 3 | 4 | | IV-1 | Abhd11os | | |
| 1748 | 2 | 3 | 4 | | IV-1 | Abhd12b | 145447 | 4-May-15 |
| 1751 | 2 | 3 | 4 | | IV-1 | Abhd14b | 84836 | 4-May-15 |
| 1758 | 2 | 3 | 4 | | IV-1 | Abhd2 | 11057 | 23-May-15 |
| 1763 | 2 | 3 | 4 | | IV-1 | Abhd8 | 79575 | 4-May-15 |
| 1765 | 2 | 3 | 4 | | IV-1 | Abi2 | 10152 | 12-May-15 |
| 1771 | 2 | 3 | 4 | | IV-1 | Ablim2 | 84448 | 12-May-15 |
| 1772 | 2 | 3 | 4 | | IV-1 | Ablim3 | 22885 | 12-May-15 |
| 1773 | 2 | 3 | 4 | | IV-1 | Abo | 28 | 12-May-15 |
| 1781 | 2 | 3 | 4 | | IV-1 | Acaa1b | | |
| 1783 | 2 | 3 | 4 | | IV-1 | Acaca | 31 | 4-May-15 |
| 1784 | 2 | 3 | 4 | | IV-1 | Acacb | 32 | 12-May-15 |
| 1796 | 2 | 3 | 4 | | IV-1 | Acap1 | 9744 | 4-May-15 |
| 1810 | 2 | 3 | 4 | | IV-1 | Ace | 1636 | 24-May-15 |
| 1816 | 2 | 3 | 4 | | IV-1 | Ache | 43 | 28-May-15 |
| 1818 | 2 | 3 | 4 | | IV-1 | Ackr1 | 2532 | 21-May-15 |
| 1820 | 2 | 3 | 4 | | IV-1 | Ackr3 | 57007 | 31-May-15 |
| 1822 | 2 | 3 | 4 | | IV-1 | Acly | 47 | 4-May-15 |
| 1824 | 2 | 3 | 4 | | IV-1 | Acn9 | 57001 | 4-May-15 |
| 1825 | 2 | 3 | 4 | | IV-1 | Acnat1 | | |
| 1829 | 2 | 3 | 4 | | IV-1 | Acot1 | 641371 | 4-May-15 |
| 1832 | 2 | 3 | 4 | | IV-1 | Acot12 | 134526 | 4-May-15 |
| 1834 | 2 | 3 | 4 | | IV-1 | Acot2 | 10965 | 4-May-15 |
| 1836 | 2 | 3 | 4 | | IV-1 | Acot4 | 122970 | 4-May-15 |
| 1843 | 2 | 3 | 4 | | IV-1 | Acox2 | 8309 | 4-May-15 |
| 1844 | 2 | 3 | 4 | | IV-1 | Acox3 | 8310 | 4-May-15 |
| 1848 | 2 | 3 | 4 | | IV-1 | Acp5 | 54 | 3-May-15 |
| 1850 | 2 | 3 | 4 | | IV-1 | Acpp | 55 | 12-May-15 |
| 1852 | 2 | 3 | 4 | | IV-1 | Acr | 49 | 12-May-15 |
| 1853 | 2 | 3 | 4 | | IV-1 | Acrbp | 84519 | 4-May-15 |
| 1855 | 2 | 3 | 4 | | IV-1 | Acsbg1 | 23205 | 4-May-15 |
| 1857 | 2 | 3 | 4 | | IV-1 | Acsf2 | 80221 | 4-May-15 |
| 1859 | 2 | 3 | 4 | | IV-1 | Acsl1 | 2180 | 4-May-15 |
| 1860 | 2 | 3 | 4 | | IV-1 | Acsl3 | 2181 | 4-May-15 |
| 1861 | 2 | 3 | 4 | | IV-1 | Acsl4 | 2182 | 23-May-15 |
| 1868 | 2 | 3 | 4 | | IV-1 | Acsm5 | 54988 | 4-May-15 |
| 1869 | 2 | 3 | 4 | | IV-1 | Acss1 | 84532 | 4-May-15 |
| 1872 | 2 | 3 | 4 | | IV-1 | Acss3 | 79611 | 4-May-15 |
| 1874 | 2 | 3 | 4 | | IV-1 | Acta2 | 59 | 23-May-15 |
| 1912 | 2 | 3 | 4 | | IV-1 | Acyp2 | 98 | 3-May-15 |
| 1913 | 2 | 3 | 4 | | IV-1 | Ada | 100 | 22-May-15 |
| 1948 | 2 | 3 | 4 | | IV-1 | Adam8 | 101 | 4-May-15 |
| 1950 | 2 | 3 | 4 | | IV-1 | Adamdec1 | 27299 | 24-May-15 |
| 1951 | 2 | 3 | 4 | | IV-1 | Adamts1 | 9510 | 4-May-15 |

| | | | | | | | | |
|---|---|---|---|---|---|---|---|---|
| 1956 | 2 | 3 | 4 | | IV-1 | Adamts15 | 170689 | 17-May-15 |
| 1967 | 2 | 3 | 4 | | IV-1 | Adamts7 | 11173 | 4-May-15 |
| 1971 | 2 | 3 | 4 | | IV-1 | Adamtsl2 | 9719 | 23-May-15 |
| 1973 | 2 | 3 | 4 | | IV-1 | Adamtsl4 | 54507 | 23-May-15 |
| 1978 | 2 | 3 | 4 | | IV-1 | Adarb1 | 104 | 12-May-15 |
| 1986 | 2 | 3 | 4 | | IV-1 | Adck3 | 56997 | 4-May-15 |
| 1991 | 2 | 3 | 4 | | IV-1 | Adcy2 | 108 | 4-May-15 |
| 1997 | 2 | 3 | 4 | | IV-1 | Adcy8 | 114 | 12-May-15 |
| 2000 | 2 | 3 | 4 | | IV-1 | Adcyap1r1 | 117 | 17-May-15 |
| 2011 | 2 | 3 | 4 | | IV-1 | Adhfe1 | 137872 | 4-May-15 |
| 2018 | 2 | 3 | 4 | | IV-1 | Adm | 133 | 17-May-15 |
| 2023 | 2 | 3 | 4 | | IV-1 | Adora1 | 134 | 21-May-15 |
| 2026 | 2 | 3 | 4 | | IV-1 | Adora3 | 140 | 12-May-15 |
| 2029 | 2 | 3 | 4 | | IV-1 | Adprhl1 | 113622 | 4-May-15 |
| 2037 | 2 | 3 | 4 | | IV-1 | Adra2c | 152 | 4-May-15 |
| 2039 | 2 | 3 | 4 | | IV-1 | Adrb2 | 154 | 28-May-15 |
| 2040 | 2 | 3 | 4 | | IV-1 | Adrb3 | 155 | 4-May-15 |
| 2046 | 2 | 3 | 4 | | IV-1 | Adssl1 | 122622 | 12-May-15 |
| 2047 | 2 | 3 | 4 | | IV-1 | Adtrp | 84830 | 4-May-15 |
| 2054 | 2 | 3 | 4 | | IV-1 | AF251705 | | |
| 2061 | 2 | 3 | 4 | | IV-1 | AF529169 | | |
| 2089 | 2 | 3 | 4 | | IV-1 | Agl | 178 | 23-May-15 |
| 2091 | 2 | 3 | 4 | | IV-1 | Agmo | 392636 | 4-May-15 |
| 2093 | 2 | 3 | 4 | | IV-1 | Ago2 | 27161 | 28-May-15 |
| 2097 | 2 | 3 | 4 | | IV-1 | Agpat2 | 10555 | 23-May-15 |
| 2102 | 2 | 3 | 4 | | IV-1 | Agpat9 | 84803 | 4-May-15 |
| 2108 | 2 | 3 | 4 | | IV-1 | Agt | 183 | 7-Jun-15 |
| 2121 | 2 | 3 | 4 | | IV-1 | Ahi1 | 54806 | 23-May-15 |
| 2141 | 2 | 3 | 4 | | IV-1 | AI463170 | | |
| 2142 | 2 | 3 | 4 | | IV-1 | AI464131 | | |
| 2146 | 2 | 3 | 4 | | IV-1 | AI507597 | | |
| 2151 | 2 | 3 | 4 | | IV-1 | AI646519 | | |
| 2152 | 2 | 3 | 4 | | IV-1 | AI661453 | | |
| 2153 | 2 | 3 | 4 | | IV-1 | AI662270 | | |
| 2165 | 2 | 3 | 4 | | IV-1 | Aif1 | 199 | 4-May-15 |
| 2168 | 2 | 3 | 4 | | IV-1 | Aifm2 | 84883 | 4-May-15 |
| 2183 | 2 | 3 | 4 | | IV-1 | Ak1 | 203 | 12-May-15 |
| 2187 | 2 | 3 | 4 | | IV-1 | Ak4 | 205 | 12-May-15 |
| 2201 | 2 | 3 | 4 | | IV-1 | Akap4 | 8852 | 4-May-15 |
| 2203 | 2 | 3 | 4 | | IV-1 | Akap6 | 9472 | 4-May-15 |
| 2219 | 2 | 3 | 4 | | IV-1 | Akr1c12 | | |
| 2220 | 2 | 3 | 4 | | IV-1 | Akr1c13 | | |
| 2223 | 2 | 3 | 4 | | IV-1 | Akr1c19 | | |
| 2230 | 2 | 3 | 4 | | IV-1 | Akr7a5 | | |
| 2236 | 2 | 3 | 4 | | IV-1 | Alad | 210 | 4-May-15 |
| 2244 | 2 | 3 | 4 | | IV-1 | Aldh1a2 | 8854 | 23-May-15 |
| 2246 | 2 | 3 | 4 | | IV-1 | Aldh1a7 | | |
| 2253 | 2 | 3 | 4 | | IV-1 | Aldh3b1 | 221 | 23-May-15 |
| 2254 | 2 | 3 | 4 | | IV-1 | Aldh3b2 | 222 | 23-May-15 |
| 2255 | 2 | 3 | 4 | | IV-1 | Aldh4a1 | 8659 | 23-May-15 |
| 2256 | 2 | 3 | 4 | | IV-1 | Aldh5a1 | 7915 | 23-May-15 |
| 2257 | 2 | 3 | 4 | | IV-1 | Aldh6a1 | 4329 | 23-May-15 |
| 2261 | 2 | 3 | 4 | | IV-1 | Aldoa | 226 | 4-May-15 |
| 2262 | 2 | 3 | 4 | | IV-1 | Aldoart1 | | |
| 2263 | 2 | 3 | 4 | | IV-1 | Aldoart2 | | |
| 2267 | 2 | 3 | 4 | | IV-1 | Alg10b | 144245 | 12-May-15 |
| 2280 | 2 | 3 | 4 | | IV-1 | Alkbh2 | 121642 | 12-May-15 |
| 2285 | 2 | 3 | 4 | | IV-1 | Alkbh7 | 84266 | 4-May-15 |
| 2293 | 2 | 3 | 4 | | IV-1 | Alox15 | 246 | 4-May-15 |
| 2294 | 2 | 3 | 4 | | IV-1 | Alox5 | 240 | 10-May-15 |
| 2295 | 2 | 3 | 4 | | IV-1 | Alox5ap | 241 | 4-May-15 |
| 2300 | 2 | 3 | 4 | | IV-1 | Alpk2 | 115701 | 4-May-15 |
| 2307 | 2 | 3 | 4 | | IV-1 | Als2cr12 | 130540 | 12-May-15 |
| 2311 | 2 | 3 | 4 | | IV-1 | Alyref | 10189 | 4-May-15 |
| 2315 | 2 | 3 | 4 | | IV-1 | Ambp | 259 | 12-May-15 |
| 2328 | 2 | 3 | 4 | | IV-1 | Amica1 | 120425 | 4-May-15 |
| 2329 | 2 | 3 | 4 | | IV-1 | Amigo1 | 57463 | 2-Jun-15 |
| 2330 | 2 | 3 | 4 | | IV-1 | Amigo2 | 347902 | 4-May-15 |
| 2331 | 2 | 3 | 4 | | IV-1 | Amigo3 | 386724 | 4-May-15 |
| 2335 | 2 | 3 | 4 | | IV-1 | Amn1 | 196394 | 4-May-15 |
| 2336 | 2 | 3 | 4 | | IV-1 | Amot | 154796 | 4-May-15 |
| 2343 | 2 | 3 | 4 | | IV-1 | Amt | 275 | 23-May-15 |
| 2361 | 2 | 3 | 4 | | IV-1 | Ang | 283 | 23-May-15 |
| 2374 | 2 | 3 | 4 | | IV-1 | Angptl3 | 27329 | 4-May-15 |
| 2375 | 2 | 3 | 4 | | IV-1 | Angptl4 | 51129 | 4-May-15 |
| 2381 | 2 | 3 | 4 | | IV-1 | Ank3 | 288 | 17-May-15 |
| 2390 | 2 | 3 | 4 | | IV-1 | Ankle1 | 126549 | 4-May-15 |
| 2400 | 2 | 3 | 4 | | IV-1 | Ankrd13b | 124930 | 4-May-15 |
| 2407 | 2 | 3 | 4 | | IV-1 | Ankrd23 | 200539 | 4-May-15 |
| 2419 | 2 | 3 | 4 | | IV-1 | Ankrd35 | 148741 | 4-May-15 |
| 2421 | 2 | 3 | 4 | | IV-1 | Ankrd37 | 353322 | 21-May-15 |
| 2437 | 2 | 3 | 4 | | IV-1 | Ankrd63 | 100131 244 | 4-May-15 |
| 2440 | 2 | 3 | 4 | | IV-1 | Ankrd9 | 122416 | 4-May-15 |
| 2448 | 2 | 3 | 4 | | IV-1 | Anln | 54443 | 4-May-15 |
| 2454 | 2 | 3 | 4 | | IV-1 | Ano5 | 203859 | 23-May-15 |
| 2463 | 2 | 3 | 4 | | IV-1 | Antxr1 | 84168 | 4-May-15 |
| 2469 | 2 | 3 | 4 | | IV-1 | Anxa13 | 312 | 4-May-15 |
| 2471 | 2 | 3 | 4 | | IV-1 | Anxa3 | 306 | 12-May-15 |
| 2476 | 2 | 3 | 4 | | IV-1 | Anxa8 | 653145 | 7-Jun-15 |
| 2477 | 2 | 3 | 4 | | IV-1 | Anxa9 | 8416 | 4-May-15 |
| 2479 | 2 | 3 | 4 | | IV-1 | Aoc1 | 26 | 4-May-15 |
| 2481 | 2 | 3 | 4 | | IV-1 | Aoc3 | 8639 | 4-May-15 |
| 2482 | 2 | 3 | 4 | | IV-1 | Aox1 | 316 | 12-May-15 |
| 2501 | 2 | 3 | 4 | | IV-1 | Ap3b2 | 8120 | 4-May-15 |
| 2520 | 2 | 3 | 4 | | IV-1 | Apbb1ip | 54518 | 12-May-15 |
| 2525 | 2 | 3 | 4 | | IV-1 | Apcdd1 | 147495 | 4-May-15 |
| 2529 | 2 | 3 | 4 | | IV-1 | Apex1 | 328 | 12-May-15 |

Fig.22 - 23

| | | | | | | | | |
|---|---|---|---|---|---|---|---|---|
| 2549 | 2 | 3 | 4 | | IV-1 | Apobec1 | 339 | 23-May-15 |
| 2553 | 2 | 3 | 4 | | IV-1 | Apobr | 55911 | 4-May-15 |
| 2554 | 2 | 3 | 4 | | IV-1 | Apoc1 | 341 | 21-May-15 |
| 2555 | 2 | 3 | 4 | | IV-1 | Apoc2 | 344 | 4-May-15 |
| 2558 | 2 | 3 | 4 | | IV-1 | Apod | 347 | 12-May-15 |
| 2560 | 2 | 3 | 4 | | IV-1 | Apof | 319 | 4-May-15 |
| 2565 | 2 | 3 | 4 | | IV-1 | Apol11b | | |
| 2566 | 2 | 3 | 4 | | IV-1 | Apol6 | 80830 | 4-May-15 |
| 2571 | 2 | 3 | 4 | | IV-1 | Apol7e | | |
| 2573 | 2 | 3 | 4 | | IV-1 | Apol9a | | |
| 2574 | 2 | 3 | 4 | | IV-1 | Apol9b | | |
| 2578 | 2 | 3 | 4 | | IV-1 | Apoo | 79135 | 4-May-15 |
| 2582 | 2 | 3 | 4 | | IV-1 | App | 351 | 31-May-15 |
| 2588 | 2 | 3 | 4 | | IV-1 | Aqp1 | 358 | 17-May-15 |
| 2592 | 2 | 3 | 4 | | IV-1 | Aqp3 | 360 | 12-May-15 |
| 2593 | 2 | 3 | 4 | | IV-1 | Aqp4 | 361 | 24-May-15 |
| 2594 | 2 | 3 | 4 | | IV-1 | Aqp5 | 362 | 4-May-15 |
| 2595 | 2 | 3 | 4 | | IV-1 | Aqp6 | 363 | 4-May-15 |
| 2596 | 2 | 3 | 4 | | IV-1 | Aqp7 | 364 | 4-May-15 |
| 2598 | 2 | 3 | 4 | | IV-1 | Aqp9 | 366 | 7-Jun-15 |
| 2600 | 2 | 3 | 4 | | IV-1 | Ar | 367 | 7-Jun-15 |
| 2607 | 2 | 3 | 4 | | IV-1 | Areg | 374 | 4-May-15 |
| 2628 | 2 | 3 | 4 | | IV-1 | Arhgap11a | 9824 | 4-May-15 |
| 2640 | 2 | 3 | 4 | | IV-1 | Arhgap24 | 83478 | 4-May-15 |
| 2641 | 2 | 3 | 4 | | IV-1 | Arhgap25 | 9938 | 4-May-15 |
| 2642 | 2 | 3 | 4 | | IV-1 | Arhgap26 | 23092 | 12-May-15 |
| 2659 | 2 | 3 | 4 | | IV-1 | Arhgap5 | 394 | 12-May-15 |
| 2661 | 2 | 3 | 4 | | IV-1 | Arhgap8 | 23779 | 4-May-15 |
| 2662 | 2 | 3 | 4 | | IV-1 | Arhgap9 | 64333 | 4-May-15 |
| 2665 | 2 | 3 | 4 | | IV-1 | Arhgdig | 398 | 4-May-15 |
| 2672 | 2 | 3 | 4 | | IV-1 | Arhgef16 | 27237 | 12-May-15 |
| 2673 | 2 | 3 | 4 | | IV-1 | Arhgef17 | 9828 | 4-May-15 |
| 2682 | 2 | 3 | 4 | | IV-1 | Arhgef37 | 389337 | 4-May-15 |
| 2685 | 2 | 3 | 4 | | IV-1 | Arhgef4 | 50649 | 17-May-15 |
| 2699 | 2 | 3 | 4 | | IV-1 | Arid5a | 10865 | 4-May-15 |
| 2700 | 2 | 3 | 4 | | IV-1 | Arid5b | 84159 | 4-May-15 |
| 2705 | 2 | 3 | 4 | | IV-1 | Arl11 | 115761 | 4-May-15 |
| 2717 | 2 | 3 | 4 | | IV-1 | Arl4c | 10123 | 4-May-15 |
| 2718 | 2 | 3 | 4 | | IV-1 | Arl4d | 379 | 4-May-15 |
| 2720 | 2 | 3 | 4 | | IV-1 | Arl5b | 221079 | 4-May-15 |
| 2732 | 2 | 3 | 4 | | IV-1 | Armc12 | 221481 | 4-May-15 |
| 2742 | 2 | 3 | 4 | | IV-1 | Armcx2 | 9823 | 4-May-15 |
| 2764 | 2 | 3 | 4 | | IV-1 | Arrdc2 | 27106 | 4-May-15 |
| 2765 | 2 | 3 | 4 | | IV-1 | Arrdc3 | 57561 | 4-May-15 |
| 2767 | 2 | 3 | 4 | | IV-1 | Arrdc5 | 645432 | 21-May-15 |
| 2775 | 2 | 3 | 4 | | IV-1 | Art2a-ps | | |
| 2777 | 2 | 3 | 4 | | IV-1 | Art3 | 419 | 4-May-15 |
| 2779 | 2 | 3 | 4 | | IV-1 | Art5 | 116969 | 4-May-15 |
| 2781 | 2 | 3 | 4 | | IV-1 | Arv1 | 64801 | 4-May-15 |
| 2784 | 2 | 3 | 4 | | IV-1 | Arxes1 | | |
| 2791 | 2 | 3 | 4 | | IV-1 | Asap3 | 55616 | 4-May-15 |
| 2793 | 2 | 3 | 4 | | IV-1 | Asb10 | 136371 | 4-May-15 |
| 2795 | 2 | 3 | 4 | | IV-1 | Asb12 | 142689 | 4-May-15 |
| 2797 | 2 | 3 | 4 | | IV-1 | Asb14 | 142686 | 4-May-15 |
| 2798 | 2 | 3 | 4 | | IV-1 | Asb15 | 142685 | 4-May-15 |
| 2801 | 2 | 3 | 4 | | IV-1 | Asb17os | | |
| 2802 | 2 | 3 | 4 | | IV-1 | Asb18 | 401036 | 4-May-15 |
| 2803 | 2 | 3 | 4 | | IV-1 | Asb2 | 51676 | 4-May-15 |
| 2820 | 2 | 3 | 4 | | IV-1 | Asf1b | 55723 | 4-May-15 |
| 2830 | 2 | 3 | 4 | | IV-1 | Asl | 435 | 7-Jun-15 |
| 2833 | 2 | 3 | 4 | | IV-1 | Asns | 440 | 12-May-15 |
| 2835 | 2 | 3 | 4 | | IV-1 | Aspa | 443 | 23-May-15 |
| 2837 | 2 | 3 | 4 | | IV-1 | Aspg | 374569 | 4-May-15 |
| 2838 | 2 | 3 | 4 | | IV-1 | Asph | 444 | 12-May-15 |
| 2846 | 2 | 3 | 4 | | IV-1 | Ass1 | 445 | 23-May-15 |
| 2860 | 2 | 3 | 4 | | IV-1 | Atad3aos | | |
| 2863 | 2 | 3 | 4 | | IV-1 | Atcay | 85300 | 4-May-15 |
| 2868 | 2 | 3 | 4 | | IV-1 | Atf3 | 467 | 12-May-15 |
| 2886 | 2 | 3 | 4 | | IV-1 | Atg4a | 115201 | 24-May-15 |
| 2904 | 2 | 3 | 4 | | IV-1 | Atoh8 | 84913 | 3-May-15 |
| 2912 | 2 | 3 | 4 | | IV-1 | Atp12a | 479 | 4-May-15 |
| 2916 | 2 | 3 | 4 | | IV-1 | Atp13a4 | 84239 | 4-May-15 |
| 2919 | 2 | 3 | 4 | | IV-1 | Atp1a2 | 477 | 23-May-15 |
| 2923 | 2 | 3 | 4 | | IV-1 | Atp1b2 | 482 | 4-May-15 |
| 2929 | 2 | 3 | 4 | | IV-1 | Atp2b1 | 490 | 24-May-15 |
| 2930 | 2 | 3 | 4 | | IV-1 | Atp2b2 | 491 | 7-Jun-15 |
| 2935 | 2 | 3 | 4 | | IV-1 | Atp4a | 495 | 4-May-15 |
| 2946 | 2 | 3 | 4 | | IV-1 | Atp5h | 10476 | 4-May-15 |
| 2949 | 2 | 3 | 4 | | IV-1 | Atp5k | 521 | 4-May-15 |
| 2951 | 2 | 3 | 4 | | IV-1 | Atp5o | 539 | 23-May-15 |
| 2956 | 2 | 3 | 4 | | IV-1 | Atp6ap2 | 10159 | 3-May-15 |
| 2966 | 2 | 3 | 4 | | IV-1 | Atp6v0e2 | 155066 | 4-May-15 |
| 2977 | 2 | 3 | 4 | | IV-1 | Atp6v1g2 | 534 | 4-May-15 |
| 2978 | 2 | 3 | 4 | | IV-1 | Atp6v1g3 | 127124 | 4-May-15 |
| 2989 | 2 | 3 | 4 | | IV-1 | Atp9a | 10079 | 4-May-15 |
| 3033 | 2 | 3 | 4 | | IV-1 | Aurkb | 9212 | 17-May-15 |
| 3035 | 2 | 3 | 4 | | IV-1 | Auts2 | 26053 | 7-Jun-15 |
| 3036 | 2 | 3 | 4 | | IV-1 | AV039307 | | |
| 3040 | 2 | 3 | 4 | | IV-1 | Avil | 10677 | 4-May-15 |
| 3042 | 2 | 3 | 4 | | IV-1 | Avp | 551 | 7-Jun-15 |
| 3044 | 2 | 3 | 4 | | IV-1 | Avpr1a | 552 | 17-May-15 |
| 3047 | 2 | 3 | 4 | | IV-1 | AW011738 | | |
| 3058 | 2 | 3 | 4 | | IV-1 | Awat1 | 158833 | 4-May-15 |
| 3059 | 2 | 3 | 4 | | IV-1 | Awat2 | 158835 | 4-May-15 |
| 3070 | 2 | 3 | 4 | | IV-1 | Azgp1 | 563 | 24-May-15 |
| 3085 | 2 | 3 | 4 | | IV-1 | B230208H11Rik | | |
| 3103 | 2 | 3 | 4 | | IV-1 | B3galt5 | 10317 | 23-May-15 |

| | | | | | | | | |
|---|---|---|---|---|---|---|---|---|
| 3113 | 2 | 3 | 4 | | IV-1 | B3gnt5 | 84002 | 4-May-15 |
| 3115 | 2 | 3 | 4 | | IV-1 | B3gnt7 | 93010 | 4-May-15 |
| 3123 | 2 | 3 | 4 | | IV-1 | B4galnt1 | 2583 | 4-May-15 |
| 3124 | 2 | 3 | 4 | | IV-1 | B4galnt2 | 124872 | 4-May-15 |
| 3125 | 2 | 3 | 4 | | IV-1 | B4galnt3 | 283358 | 4-May-15 |
| 3130 | 2 | 3 | 4 | | IV-1 | B4galt4 | 8702 | 4-May-15 |
| 3132 | 2 | 3 | 4 | | IV-1 | B4galt6 | 9331 | 4-May-15 |
| 3150 | 2 | 3 | 4 | | IV-1 | Bach1 | 571 | 13-Jun-15 |
| 3155 | 2 | 3 | 4 | | IV-1 | Bag2 | 9532 | 4-May-15 |
| 3165 | 2 | 3 | 4 | | IV-1 | Baiap2 | 10458 | 12-May-15 |
| 3166 | 2 | 3 | 4 | | IV-1 | Baiap2l1 | 55971 | 4-May-15 |
| 3181 | 2 | 3 | 4 | | IV-1 | Barx2 | 8538 | 28-May-15 |
| 3183 | 2 | 3 | 4 | | IV-1 | Batf | 10538 | 7-Jun-15 |
| 3185 | 2 | 3 | 4 | | IV-1 | Batf3 | 55509 | 4-May-15 |
| 3187 | 2 | 3 | 4 | | IV-1 | Baz1a | 11177 | 4-May-15 |
| 3191 | 2 | 3 | 4 | | IV-1 | BB014433 | | |
| 3194 | 2 | 3 | 4 | | IV-1 | BB123696 | | |
| 3203 | 2 | 3 | 4 | | IV-1 | Bbs12 | 166379 | 23-May-15 |
| 3215 | 2 | 3 | 4 | | IV-1 | BC005561 | | |
| 3222 | 2 | 3 | 4 | | IV-1 | BC018242 | | |
| 3229 | 2 | 3 | 4 | | IV-1 | BC021891 | | |
| 3230 | 2 | 3 | 4 | | IV-1 | BC022687 | | |
| 3236 | 2 | 3 | 4 | | IV-1 | BC026585 | | |
| 3239 | 2 | 3 | 4 | | IV-1 | BC028528 | | |
| 3240 | 2 | 3 | 4 | | IV-1 | BC029214 | | |
| 3250 | 2 | 3 | 4 | | IV-1 | BC033916 | | |
| 3289 | 2 | 3 | 4 | | IV-1 | BC064078 | | |
| 3295 | 2 | 3 | 4 | | IV-1 | BC089597 | | |
| 3301 | 2 | 3 | 4 | | IV-1 | BC117090 | | |
| 3307 | 2 | 3 | 4 | | IV-1 | Bcar1 | 9564 | 17-May-15 |
| 3308 | 2 | 3 | 4 | | IV-1 | Bcar3 | 8412 | 12-May-15 |
| 3316 | 2 | 3 | 4 | | IV-1 | Bcat2 | 587 | 4-May-15 |
| 3319 | 2 | 3 | 4 | | IV-1 | Bche | 590 | 17-May-15 |
| 3327 | 2 | 3 | 4 | | IV-1 | Bcl2a1a | | |
| 3330 | 2 | 3 | 4 | | IV-1 | Bcl2a1d | | |
| 3333 | 2 | 3 | 4 | | IV-1 | Bcl2l11 | 10018 | 17-May-15 |
| 3336 | 2 | 3 | 4 | | IV-1 | Bcl2l14 | 79370 | 4-May-15 |
| 3339 | 2 | 3 | 4 | | IV-1 | Bcl3 | 602 | 4-May-15 |
| 3354 | 2 | 3 | 4 | | IV-1 | Bdh1 | 622 | 4-May-15 |
| 3355 | 2 | 3 | 4 | | IV-1 | Bdh2 | 56898 | 4-May-15 |
| 3357 | 2 | 3 | 4 | | IV-1 | Bdkrb2 | 624 | 7-Jun-15 |
| 3366 | 2 | 3 | 4 | | IV-1 | Bend5 | 79656 | 4-May-15 |
| 3370 | 2 | 3 | 4 | | IV-1 | Best2 | 54831 | 4-May-15 |
| 3381 | 2 | 3 | 4 | | IV-1 | Bglap | 632 | 17-May-15 |
| 3387 | 2 | 3 | 4 | | IV-1 | Bhlhb9 | 80823 | 4-May-15 |
| 3391 | 2 | 3 | 4 | | IV-1 | Bhlhe41 | 79365 | 4-May-15 |
| 3399 | 2 | 3 | 4 | | IV-1 | Bin1 | 274 | 12-May-15 |
| 3403 | 2 | 3 | 4 | | IV-1 | Birc3 | 330 | 24-May-15 |
| 3404 | 2 | 3 | 4 | | IV-1 | Birc5 | 332 | 24-May-15 |
| 3409 | 2 | 3 | 4 | | IV-1 | Blk | 640 | 17-May-15 |
| 3410 | 2 | 3 | 4 | | IV-1 | Blm | 641 | 23-May-15 |
| 3422 | 2 | 3 | 4 | | IV-1 | Bmf | 90427 | 4-May-15 |
| 3425 | 2 | 3 | 4 | | IV-1 | Bmp10 | 27302 | 4-May-15 |
| 3430 | 2 | 3 | 4 | | IV-1 | Bmp4 | 652 | 31-May-15 |
| 3433 | 2 | 3 | 4 | | IV-1 | Bmp7 | 655 | 17-May-15 |
| 3434 | 2 | 3 | 4 | | IV-1 | Bmp8a | 353500 | 4-May-15 |
| 3438 | 2 | 3 | 4 | | IV-1 | Bmpr1b | 658 | 3-May-15 |
| 3442 | 2 | 3 | 4 | | IV-1 | Bmyc | | |
| 3447 | 2 | 3 | 4 | | IV-1 | Bnip3 | 664 | 12-May-15 |
| 3450 | 2 | 3 | 4 | | IV-1 | Boc | 91653 | 12-May-15 |
| 3456 | 2 | 3 | 4 | | IV-1 | Bola3 | 388962 | 4-May-15 |
| 3460 | 2 | 3 | 4 | | IV-1 | Bpgm | 669 | 12-May-15 |
| 3461 | 2 | 3 | 4 | | IV-1 | Bphl | 670 | 12-May-15 |
| 3464 | 2 | 3 | 4 | | IV-1 | Bpifa2 | 140683 | 4-May-15 |
| 3497 | 2 | 3 | 4 | | IV-1 | Bri3bp | 140707 | 4-May-15 |
| 3521 | 2 | 3 | 4 | | IV-1 | Bsph2 | | |
| 3537 | 2 | 3 | 4 | | IV-1 | Btbd7 | 55727 | 4-May-15 |
| 3549 | 2 | 3 | 4 | | IV-1 | Btla | 151888 | 4-May-15 |
| 3565 | 2 | 3 | 4 | | IV-1 | Bves | 11149 | 4-May-15 |
| 3567 | 2 | 3 | 4 | | IV-1 | Bzrap1 | 9256 | 4-May-15 |
| 3572 | 2 | 3 | 4 | | IV-1 | C030013G03Rik | | |
| 3578 | 2 | 3 | 4 | | IV-1 | C030034L19Rik | | |
| 3588 | 2 | 3 | 4 | | IV-1 | C130050O18Rik | | |
| 3597 | 2 | 3 | 4 | | IV-1 | C1galt1 | 56913 | 14-May-15 |
| 3599 | 2 | 3 | 4 | | IV-1 | C1qa | 712 | 17-May-15 |
| 3600 | 2 | 3 | 4 | | IV-1 | C1qb | 713 | 4-May-15 |
| 3602 | 2 | 3 | 4 | | IV-1 | C1qc | 714 | 4-May-15 |
| 3609 | 2 | 3 | 4 | | IV-1 | C1qtnf3 | 114899 | 4-May-15 |
| 3610 | 2 | 3 | 4 | | IV-1 | C1qtnf4 | 114900 | 4-May-15 |
| 3613 | 2 | 3 | 4 | | IV-1 | C1qtnf7 | 114905 | 4-May-15 |
| 3624 | 2 | 3 | 4 | | IV-1 | C230035I16Rik | | |
| 3650 | 2 | 3 | 4 | | IV-1 | C3ar1 | 719 | 4-May-15 |
| 3654 | 2 | 3 | 4 | | IV-1 | C4a | 720 | 4-May-15 |
| 3655 | 2 | 3 | 4 | | IV-1 | C4b | 721 | 17-May-15 |
| 3657 | 2 | 3 | 4 | | IV-1 | C4bp-ps1 | | |
| 3659 | 2 | 3 | 4 | | IV-1 | C530008M17Rik | | |
| 3667 | 2 | 3 | 4 | | IV-1 | C7 | 730 | 7-Jun-15 |
| 3671 | 2 | 3 | 4 | | IV-1 | C77080 | | |
| 3673 | 2 | 3 | 4 | | IV-1 | C78339 | | |
| 3683 | 2 | 3 | 4 | | IV-1 | C8g | 733 | 4-May-15 |
| 3693 | 2 | 3 | 4 | | IV-1 | Cables1 | 91768 | 4-May-15 |
| 3697 | 2 | 3 | 4 | | IV-1 | Cabp4 | 57010 | 14-May-15 |
| 3698 | 2 | 3 | 4 | | IV-1 | Cabp5 | 56344 | 12-May-15 |
| 3700 | 2 | 3 | 4 | | IV-1 | Cabs1 | 85438 | 4-May-15 |
| 3707 | 2 | 3 | 4 | | IV-1 | Cacna1d | 776 | 10-May-15 |
| 3714 | 2 | 3 | 4 | | IV-1 | Cacna2d1 | 781 | 12-May-15 |
| 3718 | 2 | 3 | 4 | | IV-1 | Cacnb1 | 782 | 12-May-15 |

Fig.22 - 24

| | | | | | | | | |
|---|---|---|---|---|---|---|---|---|
| 3728 | 2 | 3 | 4 | | IV-1 | Cacng7 | 59284 | 4-May-15 |
| 3733 | 2 | 3 | 4 | | IV-1 | Cad | 790 | 7-Jun-15 |
| 3734 | 2 | 3 | 4 | | IV-1 | Cadm1 | 23705 | 12-May-15 |
| 3739 | 2 | 3 | 4 | | IV-1 | Cadps2 | 93664 | 12-May-15 |
| 3741 | 2 | 3 | 4 | | IV-1 | Calb1 | 793 | 4-May-15 |
| 3743 | 2 | 3 | 4 | | IV-1 | Calca | 796 | 17-May-15 |
| 3744 | 2 | 3 | 4 | | IV-1 | Calcb | 797 | 28-May-15 |
| 3747 | 2 | 3 | 4 | | IV-1 | Calcr | 799 | 31-May-15 |
| 3758 | 2 | 3 | 4 | | IV-1 | Calml4 | 91860 | 4-May-15 |
| 3773 | 2 | 3 | 4 | | IV-1 | Camk2n2 | 94032 | 4-May-15 |
| 3776 | 2 | 3 | 4 | | IV-1 | Camkk2 | 10645 | 12-May-15 |
| 3780 | 2 | 3 | 4 | | IV-1 | Camp | 820 | 7-Jun-15 |
| 3783 | 2 | 3 | 4 | | IV-1 | Camsap3 | 57662 | 4-May-15 |
| 3792 | 2 | 3 | 4 | | IV-1 | Capg | 822 | 2015/6/7 |
| 3794 | 2 | 3 | 4 | | IV-1 | Capn10 | 11132 | 23-May-15 |
| 3800 | 2 | 3 | 4 | | IV-1 | Capn3 | 825 | 23-May-15 |
| 3804 | 2 | 3 | 4 | | IV-1 | Capn8 | 388743 | 4-May-15 |
| 3807 | 2 | 3 | 4 | | IV-1 | Capns2 | 84290 | 4-May-15 |
| 3819 | 2 | 3 | 4 | | IV-1 | Car12 | | |
| 3821 | 2 | 3 | 4 | | IV-1 | Car14 | | |
| 3830 | 2 | 3 | 4 | | IV-1 | Car8 | | |
| 3844 | 2 | 3 | 4 | | IV-1 | Cartpt | 9607 | |
| 3854 | 2 | 3 | 4 | | IV-1 | Casp12 | 100506 742 | 4-May-15 |
| 3865 | 2 | 3 | 4 | | IV-1 | Casq2 | 845 | |
| 3866 | 2 | 3 | 4 | | IV-1 | Casr | 846 | 24-May-15 |
| 3875 | 2 | 3 | 4 | | IV-1 | Catsper4 | 378807 | 4-May-15 |
| 3888 | 2 | 3 | 4 | | IV-1 | Cblc | 23624 | 7-Jun-15 |
| 3890 | 2 | 3 | 4 | | IV-1 | Cbln1 | 869 | 4-May-15 |
| 3892 | 2 | 3 | 4 | | IV-1 | Cbln3 | 643866 | 4-May-15 |
| 3895 | 2 | 3 | 4 | | IV-1 | Cbr2 | | |
| 3898 | 2 | 3 | 4 | | IV-1 | Cbs | 875 | 23-May-15 |
| 3914 | 2 | 3 | 4 | | IV-1 | Ccbe1 | 147372 | |
| 3915 | 2 | 3 | 4 | | IV-1 | Ccbl1 | 883 | 4-May-15 |
| 3926 | 2 | 3 | 4 | | IV-1 | Ccdc11 | 220136 | 4-May-15 |
| 3935 | 2 | 3 | 4 | | IV-1 | Ccdc120 | 90060 | 28-May-15 |
| 3937 | 2 | 3 | 4 | | IV-1 | Ccdc122 | 160857 | 4-May-15 |
| 3948 | 2 | 3 | 4 | | IV-1 | Ccdc136 | 64753 | |
| 3963 | 2 | 3 | 4 | | IV-1 | Ccdc153 | 283152 | 4-May-15 |
| 3990 | 2 | 3 | 4 | | IV-1 | Ccdc19 | 25790 | 4-May-15 |
| 3992 | 2 | 3 | 4 | | IV-1 | Ccdc23 | 374969 | 4-May-15 |
| 3997 | 2 | 3 | 4 | | IV-1 | Ccdc28b | 79140 | 23-May-15 |
| 4018 | 2 | 3 | 4 | | IV-1 | Ccdc57 | 284001 | |
| 4019 | 2 | 3 | 4 | | IV-1 | Ccdc58 | 131076 | 21-May-15 |
| 4026 | 2 | 3 | 4 | | IV-1 | Ccdc64 | 92558 | |
| 4038 | 2 | 3 | 4 | | IV-1 | Ccdc74a | 90557 | |
| 4042 | 2 | 3 | 4 | | IV-1 | Ccdc8 | 83987 | 23-May-15 |
| 4050 | 2 | 3 | 4 | | IV-1 | Ccdc85c | 317762 | 4-May-15 |
| 4055 | 2 | 3 | 4 | | IV-1 | Ccdc88c | 440193 | 12-May-15 |
| 4060 | 2 | 3 | 4 | | IV-1 | Ccdc92 | 80212 | 21-May-15 |
| 4068 | 2 | 3 | 4 | | IV-1 | Cck | 885 | 2015/6/7 |
| 4070 | 2 | 3 | 4 | | IV-1 | Cckbr | 887 | 12-May-15 |
| 4072 | 2 | 3 | 4 | | IV-1 | Ccl11 | 6356 | 10-May-15 |
| 4075 | 2 | 3 | 4 | | IV-1 | Ccl19 | 6363 | |
| 4078 | 2 | 3 | 4 | | IV-1 | Ccl21a | | |
| 4080 | 2 | 3 | 4 | | IV-1 | Ccl21c | | |
| 4081 | 2 | 3 | 4 | | IV-1 | Ccl22 | 6367 | 4-May-15 |
| 4086 | 2 | 3 | 4 | | IV-1 | Ccl27b | | |
| 4087 | 2 | 3 | 4 | | IV-1 | Ccl28 | 56477 | 7-Jun-15 |
| 4090 | 2 | 3 | 4 | | IV-1 | Ccl5 | 6352 | 17-May-15 |
| 4094 | 2 | 3 | 4 | | IV-1 | Ccl9 | | |
| 4104 | 2 | 3 | 4 | | IV-1 | Ccnd1 | 595 | 24-May-15 |
| 4108 | 2 | 3 | 4 | | IV-1 | Ccne1 | 898 | |
| 4109 | 2 | 3 | 4 | | IV-1 | Ccne2 | 9134 | |
| 4128 | 2 | 3 | 4 | | IV-1 | Ccr1 | 1230 | |
| 4131 | 2 | 3 | 4 | | IV-1 | Ccr2 | 729230 | |
| 4132 | 2 | 3 | 4 | | IV-1 | Ccr3 | 1232 | 12-May-15 |
| 4135 | 2 | 3 | 4 | | IV-1 | Ccr6 | 1235 | |
| 4136 | 2 | 3 | 4 | | IV-1 | Ccr7 | 1236 | 17-May-15 |
| 4139 | 2 | 3 | 4 | | IV-1 | Ccrl2 | 9034 | 4-May-15 |
| 4140 | 2 | 3 | 4 | | IV-1 | Ccrn4l | 25819 | |
| 4155 | 2 | 3 | 4 | | IV-1 | Cd101 | 9398 | |
| 4157 | 2 | 3 | 4 | | IV-1 | Cd14 | 929 | 2015/6/7 |
| 4160 | 2 | 3 | 4 | | IV-1 | Cd163 | 9332 | |
| 4163 | 2 | 3 | 4 | | IV-1 | Cd164l2 | 388611 | 4-May-15 |
| 4164 | 2 | 3 | 4 | | IV-1 | Cd177 | 57126 | 4-May-15 |
| 4167 | 2 | 3 | 4 | | IV-1 | Cd1d1 | | |
| 4175 | 2 | 3 | 4 | | IV-1 | Cd207 | 50489 | |
| 4177 | 2 | 3 | 4 | | IV-1 | Cd209b | | |
| 4179 | 2 | 3 | 4 | | IV-1 | Cd209d | | |
| 4183 | 2 | 3 | 4 | | IV-1 | Cd22 | 933 | |
| 4188 | 2 | 3 | 4 | | IV-1 | Cd24a | 100133 941 | 21-May-15 |
| 4195 | 2 | 3 | 4 | | IV-1 | Cd300a | 11314 | |
| 4198 | 2 | 3 | 4 | | IV-1 | Cd300lb | 124599 | |
| 4207 | 2 | 3 | 4 | | IV-1 | Cd36 | 948 | |
| 4210 | 2 | 3 | 4 | | IV-1 | Cd3d | 915 | 12-May-15 |
| 4217 | 2 | 3 | 4 | | IV-1 | Cd44 | 960 | |
| 4220 | 2 | 3 | 4 | | IV-1 | Cd48 | 962 | |
| 4221 | 2 | 3 | 4 | | IV-1 | Cd5 | 921 | 12-May-15 |
| 4223 | 2 | 3 | 4 | | IV-1 | Cd53 | 963 | |
| 4225 | 2 | 3 | 4 | | IV-1 | Cd59a | | |
| 4228 | 2 | 3 | 4 | | IV-1 | Cd6 | 923 | 12-May-15 |
| 4229 | 2 | 3 | 4 | | IV-1 | Cd63 | 967 | 17-May-15 |
| 4230 | 2 | 3 | 4 | | IV-1 | Cd68 | 968 | |
| 4231 | 2 | 3 | 4 | | IV-1 | Cd69 | 969 | 4-May-15 |
| 4233 | 2 | 3 | 4 | | IV-1 | Cd70 | 970 | 4-May-15 |
| 4234 | 2 | 3 | 4 | | IV-1 | Cd72 | 971 | 4-May-15 |

| | | | | | | | | |
|---|---|---|---|---|---|---|---|---|
| 4236 | 2 | 3 | 4 | | IV-1 | Cd79a | 973 | 12-May-15 |
| 4237 | 2 | 3 | 4 | | IV-1 | Cd79b | 974 | 4-May-15 |
| 4242 | 2 | 3 | 4 | | IV-1 | Cd84 | 8832 | |
| 4246 | 2 | 3 | 4 | | IV-1 | Cd9 | 928 | |
| 4251 | 2 | 3 | 4 | | IV-1 | Cda | 978 | 4-May-15 |
| 4261 | 2 | 3 | 4 | | IV-1 | Cdc25a | 993 | 31-May-15 |
| 4278 | 2 | 3 | 4 | | IV-1 | Cdc42ep5 | 148170 | 4-May-15 |
| 4283 | 2 | 3 | 4 | | IV-1 | Cdc6 | 990 | |
| 4287 | 2 | 3 | 4 | | IV-1 | Cdca3 | 83461 | 12-May-15 |
| 4291 | 2 | 3 | 4 | | IV-1 | Cdca7l | 55536 | |
| 4295 | 2 | 3 | 4 | | IV-1 | Cdh1 | 999 | 2015/6/7 |
| 4301 | 2 | 3 | 4 | | IV-1 | Cdh16 | 1014 | 7-Jun-15 |
| 4304 | 2 | 3 | 4 | | IV-1 | Cdh19 | 28513 | y-2015 |
| 4307 | 2 | 3 | 4 | | IV-1 | Cdh22 | 64405 | 4-May-15 |
| 4321 | 2 | 3 | 4 | | IV-1 | Cdhr5 | 53841 | 4-May-15 |
| 4324 | 2 | 3 | 4 | | IV-1 | Cdk1 | 983 | 24-May-15 |
| 4333 | 2 | 3 | 4 | | IV-1 | Cdk18 | 5129 | 4-May-15 |
| 4357 | 2 | 3 | 4 | | IV-1 | Cdkn1a | 1026 | 24-May-15 |
| 4359 | 2 | 3 | 4 | | IV-1 | Cdkn1c | 1028 | |
| 4363 | 2 | 3 | 4 | | IV-1 | Cdkn2b | 1030 | |
| 4364 | 2 | 3 | 4 | | IV-1 | Cdkn2c | 1031 | |
| 4367 | 2 | 3 | 4 | | IV-1 | Cdnf | 441549 | 4-May-15 |
| 4368 | 2 | 3 | 4 | | IV-1 | Cdo1 | 1036 | |
| 4370 | 2 | 3 | 4 | | IV-1 | Cdpf1 | 150383 | 4-May-15 |
| 4372 | 2 | 3 | 4 | | IV-1 | Cdr2 | 1039 | |
| 4375 | 2 | 3 | 4 | | IV-1 | Cds1 | 1040 | /6/7 |
| 4377 | 2 | 3 | 4 | | IV-1 | Cdsn | 1041 | 30-May-15 |
| 4378 | 2 | 3 | 4 | | IV-1 | Cdt1 | 81620 | |
| 4388 | 2 | 3 | 4 | | IV-1 | Ceacam12 | | |
| 4401 | 2 | 3 | 4 | | IV-1 | Cebpa | 1050 | |
| 4402 | 2 | 3 | 4 | | IV-1 | Cebpb | 1051 | |
| 4403 | 2 | 3 | 4 | | IV-1 | Cebpd | 1052 | 4-May-15 |
| 4420 | 2 | 3 | 4 | | IV-1 | Celf6 | 60677 | 4-May-15 |
| 4426 | 2 | 3 | 4 | | IV-1 | Cend1 | 51286 | 4-May-15 |
| 4432 | 2 | 3 | 4 | | IV-1 | Cenph | 64946 | 4-May-15 |
| 4435 | 2 | 3 | 4 | | IV-1 | Cenpk | 64105 | |
| 4440 | 2 | 3 | 4 | | IV-1 | Cenpp | 401541 | |
| 4442 | 2 | 3 | 4 | | IV-1 | Cenpt | 80152 | |
| 4456 | 2 | 3 | 4 | | IV-1 | Cep170b | 283638 | 4-May-15 |
| 4471 | 2 | 3 | 4 | | IV-1 | Cep76 | 79959 | |
| 4472 | 2 | 3 | 4 | | IV-1 | Cep78 | 84131 | 4-May-15 |
| 4475 | 2 | 3 | 4 | | IV-1 | Cep85 | 64793 | 4-May-15 |
| 4476 | 2 | 3 | 4 | | IV-1 | Cep85l | 387119 | |
| 4480 | 2 | 3 | 4 | | IV-1 | Cept1 | 10390 | 12-May-15 |
| 4485 | 2 | 3 | 4 | | IV-1 | Cers1 | 10715 | 21-May-15 |
| 4489 | 2 | 3 | 4 | | IV-1 | Cers5 | 91012 | /5/21 |
| 4490 | 2 | 3 | 4 | | IV-1 | Cers6 | 253782 | |
| 4494 | 2 | 3 | 4 | | IV-1 | Ces1d | | |
| 4495 | 2 | 3 | 4 | | IV-1 | Ces1e | | |
| 4496 | 2 | 3 | 4 | | IV-1 | Ces1f | | |
| 4497 | 2 | 3 | 4 | | IV-1 | Ces1g | | |
| 4498 | 2 | 3 | 4 | | IV-1 | Ces2a | | |
| 4499 | 2 | 3 | 4 | | IV-1 | Ces2b | | |
| 4502 | 2 | 3 | 4 | | IV-1 | Ces2e | | |
| 4504 | 2 | 3 | 4 | | IV-1 | Ces2g | | |
| 4508 | 2 | 3 | 4 | | IV-1 | Ces4a | 283848 | |
| 4518 | 2 | 3 | 4 | | IV-1 | Cfh | 3075 | |
| 4526 | 2 | 3 | 4 | | IV-1 | Cftr | 1080 | 24-May-15 |
| 4529 | 2 | 3 | 4 | | IV-1 | Cgn | 57530 | |
| 4530 | 2 | 3 | 4 | | IV-1 | Cgnl1 | 84952 | 12-May-15 |
| 4535 | 2 | 3 | 4 | | IV-1 | Chac2 | 494143 | |
| 4543 | 2 | 3 | 4 | | IV-1 | Chchd10 | 400916 | |
| 4549 | 2 | 3 | 4 | | IV-1 | Chchd7 | 79145 | 4-May-15 |
| 4568 | 2 | 3 | 4 | | IV-1 | Chia1 | | |
| 4572 | 2 | 3 | 4 | | IV-1 | Chil1 | | |
| 4573 | 2 | 3 | 4 | | IV-1 | Chil3 | | |
| 4602 | 2 | 3 | 4 | | IV-1 | Chpt1 | 56994 | 4-May-15 |
| 4604 | 2 | 3 | 4 | | IV-1 | Chrd | 8646 | 12-May-15 |
| 4605 | 2 | 3 | 4 | | IV-1 | Chrdl1 | 91851 | |
| 4608 | 2 | 3 | 4 | | IV-1 | Chrm2 | 1129 | 4-May-15 |
| 4610 | 2 | 3 | 4 | | IV-1 | Chrm4 | 1132 | 12-May-15 |
| 4613 | 2 | 3 | 4 | | IV-1 | Chrna10 | 57053 | 4-May-15 |
| 4614 | 2 | 3 | 4 | | IV-1 | Chrna2 | 1135 | 23-May-15 |
| 4615 | 2 | 3 | 4 | | IV-1 | Chrna3 | 1136 | 12-May-15 |
| 4616 | 2 | 3 | 4 | | IV-1 | Chrna4 | 1137 | |
| 4628 | 2 | 3 | 4 | | IV-1 | Chst1 | 8534 | |
| 4629 | 2 | 3 | 4 | | IV-1 | Chst10 | 9486 | |
| 4639 | 2 | 3 | 4 | | IV-1 | Chst7 | 56548 | 12-May-15 |
| 4651 | 2 | 3 | 4 | | IV-1 | Ciart | 148523 | 12-May-15 |
| 4653 | 2 | 3 | 4 | | IV-1 | Cib2 | 10518 | 4-May-15 |
| 4661 | 2 | 3 | 4 | | IV-1 | Cilp | 8483 | 12-May-15 |
| 4675 | 2 | 3 | 4 | | IV-1 | Cited2 | 10370 | 10-May-15 |
| 4683 | 2 | 3 | 4 | | IV-1 | Ckb | 1152 | 7-Jun-15 |
| 4684 | 2 | 3 | 4 | | IV-1 | Cklf | 51192 | /5/4 |
| 4688 | 2 | 3 | 4 | | IV-1 | Cks1b | 1163 | |
| 4694 | 2 | 3 | 4 | | IV-1 | Clca1 | 1179 | |
| 4695 | 2 | 3 | 4 | | IV-1 | Clca2 | 9635 | |
| 4698 | 2 | 3 | 4 | | IV-1 | Clca5 | | |
| 4699 | 2 | 3 | 4 | | IV-1 | Clca6 | | |
| 4701 | 2 | 3 | 4 | | IV-1 | Clcf1 | 23529 | |
| 4702 | 2 | 3 | 4 | | IV-1 | Clcn1 | 1180 | 23-May-15 |
| 4706 | 2 | 3 | 4 | | IV-1 | Clcn5 | 1184 | |
| 4712 | 2 | 3 | 4 | | IV-1 | Cldn10 | 9071 | 4-May-15 |
| 4719 | 2 | 3 | 4 | | IV-1 | Cldn17 | 26285 | 4-May-15 |
| 4725 | 2 | 3 | 4 | | IV-1 | Cldn23 | 137075 | |
| 4731 | 2 | 3 | 4 | | IV-1 | Cldn5 | 7122 | |
| 4739 | 2 | 3 | 4 | | IV-1 | Clec12a | 160364 | |

Fig.22 - 25

| | | | | | | | | |
|---|---|---|---|---|---|---|---|---|
| 4745 | 2 | 3 | 4 | | IV-1 | Clec1b | 51266 | 4-May-15 |
| 4747 | 2 | 3 | 4 | | IV-1 | Clec2e | | |
| 4755 | 2 | 3 | 4 | | IV-1 | Clec4a1 | | |
| 4757 | 2 | 3 | 4 | | IV-1 | Clec4a3 | | |
| 4759 | 2 | 3 | 4 | | IV-1 | Clec4b1 | | |
| 4761 | 2 | 3 | 4 | | IV-1 | Clec4d | 338339 | |
| 4765 | 2 | 3 | 4 | | IV-1 | Clec4n | 93978 | |
| 4772 | 2 | 3 | 4 | | IV-1 | Clic3 | 9022 | |
| 4774 | 2 | 3 | 4 | | IV-1 | Clic5 | 53405 | 4-May-15 |
| 4775 | 2 | 3 | 4 | | IV-1 | Clic6 | 54102 | |
| 4780 | 2 | 3 | 4 | | IV-1 | Clip4 | 79745 | /6/7 |
| 4786 | 2 | 3 | 4 | | IV-1 | Clmp | 79827 | |
| 4789 | 2 | 3 | 4 | | IV-1 | Cln6 | 54982 | 23-May-15 |
| 4806 | 2 | 3 | 4 | | IV-1 | Clstn1 | 22883 | 4-May-15 |
| 4818 | 2 | 3 | 4 | | IV-1 | Cma1 | 1215 | |
| 4822 | 2 | 3 | 4 | | IV-1 | Cmbl | 134147 | 4-May-15 |
| 4827 | 2 | 3 | 4 | | IV-1 | Cml1 | 9027 | 4-May-15 |
| 4857 | 2 | 3 | 4 | | IV-1 | Cngb3 | 54714 | 23-May-15 |
| 4862 | 2 | 3 | 4 | | IV-1 | Cnksr1 | 10256 | |
| 4863 | 2 | 3 | 4 | | IV-1 | Cnksr2 | 22866 | 13-May-15 |
| 4890 | 2 | 3 | 4 | | IV-1 | Cnrip1 | 25927 | 21-May-15 |
| 4893 | 2 | 3 | 4 | | IV-1 | Cntf | 1270 | 4-May-15 |
| 4894 | 2 | 3 | 4 | | IV-1 | Cntfr | 1271 | |
| 4919 | 2 | 3 | 4 | | IV-1 | Coch | 1690 | |
| 4929 | 2 | 3 | 4 | | IV-1 | Col10a1 | 1300 | |
| 4932 | 2 | 3 | 4 | | IV-1 | Col12a1 | 1303 | 12-May-15 |
| 4944 | 2 | 3 | 4 | | IV-1 | Col23a1 | 91522 | 12-May-15 |
| 4969 | 2 | 3 | 4 | | IV-1 | Col8a1 | 1295 | 4-May-15 |
| 4973 | 2 | 3 | 4 | | IV-1 | Col9a3 | 1299 | 23-May-15 |
| 4998 | 2 | 3 | 4 | | IV-1 | Coprs | 55352 | 4-May-15 |
| 5000 | 2 | 3 | 4 | | IV-1 | Cops3 | 8533 | 4-May-15 |
| 5022 | 2 | 3 | 4 | | IV-1 | Coro2a | 7464 | |
| 5024 | 2 | 3 | 4 | | IV-1 | Coro6 | 84940 | |
| 5027 | 2 | 3 | 4 | | IV-1 | Cotl1 | 23406 | |
| 5038 | 2 | 3 | 4 | | IV-1 | Cox4i2 | 84701 | 4-May-15 |
| 5044 | 2 | 3 | 4 | | IV-1 | Cox6b2 | 125965 | 4-May-15 |
| 5053 | 2 | 3 | 4 | | IV-1 | Cox8b | 404544 | |
| 5055 | 2 | 3 | 4 | | IV-1 | Cp | 1356 | |
| 5066 | 2 | 3 | 4 | | IV-1 | Cpeb1 | 64506 | 4-May-15 |
| 5067 | 2 | 3 | 4 | | IV-1 | Cpeb2 | 132864 | 4-May-15 |
| 5071 | 2 | 3 | 4 | | IV-1 | Cphx1 | | |
| 5078 | 2 | 3 | 4 | | IV-1 | Cpn1 | 1369 | 7-Jun-15 |
| 5079 | 2 | 3 | 4 | | IV-1 | Cpn2 | 1370 | 7-Jun-15 |
| 5084 | 2 | 3 | 4 | | IV-1 | Cpne5 | 57699 | 4-May-15 |
| 5086 | 2 | 3 | 4 | | IV-1 | Cpne7 | 27132 | 12-May-15 |
| 5087 | 2 | 3 | 4 | | IV-1 | Cpne8 | 144402 | 4-May-15 |
| 5089 | 2 | 3 | 4 | | IV-1 | Cpox | 1371 | |
| 5092 | 2 | 3 | 4 | | IV-1 | Cps1 | 1373 | 7-Jun-15 |
| 5102 | 2 | 3 | 4 | | IV-1 | Cpt1b | 1375 | /6/7 |
| 5111 | 2 | 3 | 4 | | IV-1 | Cr2 | 1380 | 7-Jun-15 |
| 5112 | 2 | 3 | 4 | | IV-1 | Crabp1 | 1381 | 4-May-15 |
| 5113 | 2 | 3 | 4 | | IV-1 | Crabp2 | 1382 | |
| 5116 | 2 | 3 | 4 | | IV-1 | Crat | 1384 | 12-May-15 |
| 5124 | 2 | 3 | 4 | | IV-1 | Creb3 | 10488 | 7-Jun-15 |
| 5127 | 2 | 3 | 4 | | IV-1 | Creb3l3 | 84699 | 28-May-15 |
| 5129 | 2 | 3 | 4 | | IV-1 | Creb5 | 9586 | 21-May-15 |
| 5137 | 2 | 3 | 4 | | IV-1 | Creld2 | 79174 | |
| 5138 | 2 | 3 | 4 | | IV-1 | Crem | 1390 | 12-May-15 |
| 5142 | 2 | 3 | 4 | | IV-1 | Crhr2 | 1395 | 4-May-15 |
| 5144 | 2 | 3 | 4 | | IV-1 | Crip1 | 1396 | /6/7 |
| 5146 | 2 | 3 | 4 | | IV-1 | Crip3 | 401262 | 4-May-15 |
| 5149 | 2 | 3 | 4 | | IV-1 | Crisp2 | 7180 | 21-May-15 |
| 5156 | 2 | 3 | 4 | | IV-1 | Crlf1 | 9244 | 23-May-15 |
| 5175 | 2 | 3 | 4 | | IV-1 | Cry1 | 1407 | 4-May-15 |
| 5178 | 2 | 3 | 4 | | IV-1 | Cryab | 1410 | 23-May-15 |
| 5183 | 2 | 3 | 4 | | IV-1 | Crybb2 | 1415 | 21-May-15 |
| 5187 | 2 | 3 | 4 | | IV-1 | Crygb | 1419 | 4-May-15 |
| 5192 | 2 | 3 | 4 | | IV-1 | Crygn | 155051 | 21-May-15 |
| 5207 | 2 | 3 | 4 | | IV-1 | Csf2rb | 1439 | |
| 5210 | 2 | 3 | 4 | | IV-1 | Csf3r | 1441 | |
| 5238 | 2 | 3 | 4 | | IV-1 | Csrnp1 | 64651 | |
| 5245 | 2 | 3 | 4 | | IV-1 | Cst10 | | |
| 5250 | 2 | 3 | 4 | | IV-1 | Cst6 | 1474 | 7-Jun-15 |
| 5279 | 2 | 3 | 4 | | IV-1 | Ctgf | 1490 | 12-May-15 |
| 5280 | 2 | 3 | 4 | | IV-1 | Cth | 1491 | 7-Jun-15 |
| 5283 | 2 | 3 | 4 | | IV-1 | Ctla2a | | |
| 5284 | 2 | 3 | 4 | | IV-1 | Ctla2b | | |
| 5285 | 2 | 3 | 4 | | IV-1 | Ctla4 | 1493 | 17-May-15 |
| 5301 | 2 | 3 | 4 | | IV-1 | Ctrcos | | |
| 5310 | 2 | 3 | 4 | | IV-1 | Ctsc | 1075 | |
| 5312 | 2 | 3 | 4 | | IV-1 | Ctse | 1510 | 12-May-15 |
| 5313 | 2 | 3 | 4 | | IV-1 | Ctsf | 8722 | 4-May-15 |
| 5317 | 2 | 3 | 4 | | IV-1 | Ctsk | 1513 | |
| 5324 | 2 | 3 | 4 | | IV-1 | Ctss | 1520 | 24-May-15 |
| 5325 | 2 | 3 | 4 | | IV-1 | Ctsw | 1521 | 4-May-15 |
| 5328 | 2 | 3 | 4 | | IV-1 | Cttnbp2 | 83992 | 17-May-15 |
| 5332 | 2 | 3 | 4 | | IV-1 | Ctxn1 | 404217 | 4-May-15 |
| 5358 | 2 | 3 | 4 | | IV-1 | Cwh43 | 80157 | 4-May-15 |
| 5359 | 2 | 3 | 4 | | IV-1 | Cx3cl1 | 6376 | |
| 5360 | 2 | 3 | 4 | | IV-1 | Cx3cr1 | 1524 | |
| 5361 | 2 | 3 | 4 | | IV-1 | Cxadr | 1525 | 17-May-15 |
| 5363 | 2 | 3 | 4 | | IV-1 | Cxcl10 | 3627 | |
| 5367 | 2 | 3 | 4 | | IV-1 | Cxcl14 | 9547 | |
| 5375 | 2 | 3 | 4 | | IV-1 | Cxcr1 | 3577 | |
| 5376 | 2 | 3 | 4 | | IV-1 | Cxcr2 | 3579 | 17-May-15 |
| 5378 | 2 | 3 | 4 | | IV-1 | Cxcr4 | 7852 | 21-May-15 |
| 5379 | 2 | 3 | 4 | | IV-1 | Cxcr5 | 643 | 17-May-15 |

| | | | | | | | | |
|---|---|---|---|---|---|---|---|---|
| 5387 | 2 | 3 | 4 | | IV-1 | Cyb5 | 1528 | 12-May-15 |
| 5400 | 2 | 3 | 4 | | IV-1 | Cyba | 1535 | |
| 5401 | 2 | 3 | 4 | | IV-1 | Cybb | 1536 | |
| 5407 | 2 | 3 | 4 | | IV-1 | Cyfip2 | 26999 | |
| 5408 | 2 | 3 | 4 | | IV-1 | Cygb | 114757 | 12-May-15 |
| 5417 | 2 | 3 | 4 | | IV-1 | Cyp17a1 | 1586 | 24-May-15 |
| 5424 | 2 | 3 | 4 | | IV-1 | Cyp24a1 | 1591 | 4-May-15 |
| 5428 | 2 | 3 | 4 | | IV-1 | Cyp27a1 | 1593 | 24-May-15 |
| 5431 | 2 | 3 | 4 | | IV-1 | Cyp2a22 | | |
| 5435 | 2 | 3 | 4 | | IV-1 | Cyp2b10 | | |
| 5436 | 2 | 3 | 4 | | IV-1 | Cyp2b13 | | |
| 5441 | 2 | 3 | 4 | | IV-1 | Cyp2c37 | | |
| 5443 | 2 | 3 | 4 | | IV-1 | Cyp2c39 | | |
| 5444 | 2 | 3 | 4 | | IV-1 | Cyp2c40 | | |
| 5445 | 2 | 3 | 4 | | IV-1 | Cyp2c44 | | |
| 5449 | 2 | 3 | 4 | | IV-1 | Cyp2c55 | | |
| 5453 | 2 | 3 | 4 | | IV-1 | Cyp2c68 | | |
| 5454 | 2 | 3 | 4 | | IV-1 | Cyp2c69 | | |
| 5460 | 2 | 3 | 4 | | IV-1 | Cyp2d22 | | |
| 5461 | 2 | 3 | 4 | | IV-1 | Cyp2d26 | | |
| 5468 | 2 | 3 | 4 | | IV-1 | Cyp2g1 | 22952 | 12-May-15 |
| 5471 | 2 | 3 | 4 | | IV-1 | Cyp2j13 | | |
| 5475 | 2 | 3 | 4 | | IV-1 | Cyp2j9 | | |
| 5483 | 2 | 3 | 4 | | IV-1 | Cyp3a13 | | |
| 5485 | 2 | 3 | 4 | | IV-1 | Cyp3a25 | | |
| 5486 | 2 | 3 | 4 | | IV-1 | Cyp3a41a | | |
| 5487 | 2 | 3 | 4 | | IV-1 | Cyp3a41b | | |
| 5492 | 2 | 3 | 4 | | IV-1 | Cyp4a10 | | |
| 5498 | 2 | 3 | 4 | | IV-1 | Cyp4a31 | | |
| 5500 | 2 | 3 | 4 | | IV-1 | Cyp4b1 | 1580 | |
| 5501 | 2 | 3 | 4 | | IV-1 | Cyp4b1-ps2 | | |
| 5506 | 2 | 3 | 4 | | IV-1 | Cyp4f17 | | |
| 5514 | 2 | 3 | 4 | | IV-1 | Cyp51 | 1595 | |
| 5528 | 2 | 3 | 4 | | IV-1 | Cyr61 | 3491 | |
| 5532 | 2 | 3 | 4 | | IV-1 | Cystm1 | 84418 | 12-May-15 |
| 5538 | 2 | 3 | 4 | | IV-1 | Cytl1 | 54360 | |
| 5539 | 2 | 3 | 4 | | IV-1 | Cyyr1 | 116159 | |
| 5549 | 2 | 3 | 4 | | IV-1 | D10Bwg1379e | | |
| 5554 | 2 | 3 | 4 | | IV-1 | D130017N08Rik | | |
| 5569 | 2 | 3 | 4 | | IV-1 | D1Pas1 | | |
| 5574 | 2 | 3 | 4 | | IV-1 | D330023K18Rik | | |
| 5575 | 2 | 3 | 4 | | IV-1 | D330041H03Rik | | |
| 5592 | 2 | 3 | 4 | | IV-1 | D630003M21Rik | | |
| 5596 | 2 | 3 | 4 | | IV-1 | D630024D03Rik | | |
| 5599 | 2 | 3 | 4 | | IV-1 | D630033O11Rik | | |
| 5600 | 2 | 3 | 4 | | IV-1 | D630039A03Rik | | |
| 5602 | 2 | 3 | 4 | | IV-1 | D630045J12Rik | | |
| 5603 | 2 | 3 | 4 | | IV-1 | D630045M09Rik | | |
| 5620 | 2 | 3 | 4 | | IV-1 | D830031N03Rik | | |
| 5627 | 2 | 3 | 4 | | IV-1 | D930015M05Rik | | |
| 5632 | 2 | 3 | 4 | | IV-1 | D930048N14Rik | | |
| 5641 | 2 | 3 | 4 | | IV-1 | Dact2 | 168002 | 17-May-15 |
| 5642 | 2 | 3 | 4 | | IV-1 | Dact3 | 147906 | 4-May-15 |
| 5652 | 2 | 3 | 4 | | IV-1 | Dao | 1610 | 7-Jun-15 |
| 5658 | 2 | 3 | 4 | | IV-1 | Dapl1 | 92196 | 12-May-15 |
| 5667 | 2 | 3 | 4 | | IV-1 | Dbf4 | 10926 | 4-May-15 |
| 5672 | 2 | 3 | 4 | | IV-1 | Dbn1 | 1627 | 3-May-15 |
| 5673 | 2 | 3 | 4 | | IV-1 | Dbndd1 | 79007 | 4-May-15 |
| 5676 | 2 | 3 | 4 | | IV-1 | Dbp | 1628 | 7-Jun-15 |
| 5699 | 2 | 3 | 4 | | IV-1 | Dcdc2a | 51473 | 24-May-15 |
| 5701 | 2 | 3 | 4 | | IV-1 | Dcdc2c | 728597 | 21-May-15 |
| 5710 | 2 | 3 | 4 | | IV-1 | Dcn | 1634 | 23-May-15 |
| 5721 | 2 | 3 | 4 | | IV-1 | Dctd | 1635 | 4-May-15 |
| 5735 | 2 | 3 | 4 | | IV-1 | Dcxr | 51181 | 4-May-15 |
| 5740 | 2 | 3 | 4 | | IV-1 | Ddb2 | 1643 | 23-May-15 |
| 5747 | 2 | 3 | 4 | | IV-1 | Ddit4 | 54541 | 4-May-15 |
| 5749 | 2 | 3 | 4 | | IV-1 | Ddn | 23109 | 4-May-15 |
| 5750 | 2 | 3 | 4 | | IV-1 | Ddo | 8528 | 4-May-15 |
| 5768 | 2 | 3 | 4 | | IV-1 | Ddx26b | 203522 | 4-May-15 |
| 5797 | 2 | 3 | 4 | | IV-1 | Decr1 | 1666 | 12-May-15 |
| 5803 | 2 | 3 | 4 | | IV-1 | Defa17 | | |
| 5804 | 2 | 3 | 4 | | IV-1 | Defa2 | 1667 | 12-May-15 |
| 5805 | 2 | 3 | 4 | | IV-1 | Defa20 | | |
| 5806 | 2 | 3 | 4 | | IV-1 | Defa21 | | |
| 5808 | 2 | 3 | 4 | | IV-1 | Defa23 | | |
| 5812 | 2 | 3 | 4 | | IV-1 | Defa3 | 1668 | 4-May-15 |
| 5816 | 2 | 3 | 4 | | IV-1 | Defa-ps1 | | |
| 5823 | 2 | 3 | 4 | | IV-1 | Defb11 | 245913 | 4-May-15 |
| 5844 | 2 | 3 | 4 | | IV-1 | Defb36 | | |
| 5846 | 2 | 3 | 4 | | IV-1 | Defb38 | | |
| 5849 | 2 | 3 | 4 | | IV-1 | Defb40 | | |
| 5860 | 2 | 3 | 4 | | IV-1 | Defb6 | | |
| 5863 | 2 | 3 | 4 | | IV-1 | Defb9 | 245912 | 4-May-15 |
| 5865 | 2 | 3 | 4 | | IV-1 | Degs2 | 123099 | 4-May-15 |
| 5870 | 2 | 3 | 4 | | IV-1 | Dennd2a | 27147 | 4-May-15 |
| 5871 | 2 | 3 | 4 | | IV-1 | Dennd2c | 163259 | 4-May-15 |
| 5883 | 2 | 3 | 4 | | IV-1 | Depdc1b | 55789 | 4-May-15 |
| 5898 | 2 | 3 | 4 | | IV-1 | Dfna5 | 1687 | 23-May-15 |
| 5901 | 2 | 3 | 4 | | IV-1 | Dgat2 | 84649 | 12-May-15 |
| 5902 | 2 | 3 | 4 | | IV-1 | Dgat2l6 | 347516 | 4-May-15 |
| 5912 | 2 | 3 | 4 | | IV-1 | Dgkg | 1608 | 12-May-15 |
| 5919 | 2 | 3 | 4 | | IV-1 | Dhcr24 | 1718 | 12-May-15 |
| 5928 | 2 | 3 | 4 | | IV-1 | Dhrs11 | 79154 | 4-May-15 |
| 5966 | 2 | 3 | 4 | | IV-1 | Dio3os | 64150 | 12-May-15 |
| 5983 | 2 | 3 | 4 | | IV-1 | Dkk3 | 27122 | 4-May-15 |
| 5985 | 2 | 3 | 4 | | IV-1 | Dkkl1 | 27120 | 4-May-15 |
| 5987 | 2 | 3 | 4 | | IV-1 | Dlc1 | 10395 | 7-Jun-15 |

Fig.22 - 26

| | | | | | | | | |
|---|---|---|---|---|---|---|---|---|
| 5996 | 2 | 3 | 4 | | IV-1 | Dlg5 | 9231 | 12-May-15 |
| 5997 | 2 | 3 | 4 | | IV-1 | Dlgap1 | 9229 | 17-May-15 |
| 6003 | 2 | 3 | 4 | | IV-1 | Dlk2 | 65989 | 4-May-15 |
| 6004 | 2 | 3 | 4 | | IV-1 | Dll1 | 28514 | 12-May-15 |
| 6024 | 2 | 3 | 4 | | IV-1 | Dmp1 | 1758 | 17-May-15 |
| 6028 | 2 | 3 | 4 | | IV-1 | Dmrt2 | 10655 | 4-May-15 |
| 6045 | 2 | 3 | 4 | | IV-1 | Dnaaf3 | 352909 | 23-May-15 |
| 6062 | 2 | 3 | 4 | | IV-1 | Dnaja4 | 55466 | 4-May-15 |
| 6069 | 2 | 3 | 4 | | IV-1 | Dnajb3 | 414061 | 4-May-15 |
| 6071 | 2 | 3 | 4 | | IV-1 | Dnajb5 | 25822 | 4-May-15 |
| 6077 | 2 | 3 | 4 | | IV-1 | Dnajc10 | 54431 | 4-May-15 |
| 6079 | 2 | 3 | 4 | | IV-1 | Dnajc12 | 56521 | 4-May-15 |
| 6082 | 2 | 3 | 4 | | IV-1 | Dnajc15 | 29103 | 21-May-15 |
| 6106 | 2 | 3 | 4 | | IV-1 | Dnali1 | 7802 | 4-May-15 |
| 6109 | 2 | 3 | 4 | | IV-1 | Dnase1l2 | 1775 | 12-May-15 |
| 6113 | 2 | 3 | 4 | | IV-1 | Dnd1 | 373863 | 4-May-15 |
| 6119 | 2 | 3 | 4 | | IV-1 | Dnm3 | 26052 | 12-May-15 |
| 6120 | 2 | 3 | 4 | | IV-1 | Dnm3os | 100628315 | 4-May-15 |
| 6133 | 2 | 3 | 4 | | IV-1 | Doc2b | 8447 | 4-May-15 |
| 6134 | 2 | 3 | 4 | | IV-1 | Doc2g | | |
| 6142 | 2 | 3 | 4 | | IV-1 | Dock6 | 57572 | 21-May-15 |
| 6149 | 2 | 3 | 4 | | IV-1 | Dok3 | 79930 | 21-May-15 |
| 6159 | 2 | 3 | 4 | | IV-1 | Dos | 255057 | 4-May-15 |
| 6165 | 2 | 3 | 4 | | IV-1 | Dpep1 | 1800 | 12-May-15 |
| 6191 | 2 | 3 | 4 | | IV-1 | Dppa5a | | |
| 6198 | 2 | 3 | 4 | | IV-1 | Dpyd | 1806 | 31-May-15 |
| 6199 | 2 | 3 | 4 | | IV-1 | Dpys | 1807 | 4-May-15 |
| 6201 | 2 | 3 | 4 | | IV-1 | Dpysl3 | 1809 | 4-May-15 |
| 6219 | 2 | 3 | 4 | | IV-1 | Dreh | | |
| 6236 | 2 | 3 | 4 | | IV-1 | Dsg2 | 1829 | 23-May-15 |
| 6240 | 2 | 3 | 4 | | IV-1 | Dsp | 1832 | 7-Jun-15 |
| 6249 | 2 | 3 | 4 | | IV-1 | Dtna | 1837 | 12-May-15 |
| 6250 | 2 | 3 | 4 | | IV-1 | Dtnb | 1838 | 4-May-15 |
| 6254 | 2 | 3 | 4 | | IV-1 | Dtx1 | 1840 | 4-May-15 |
| 6261 | 2 | 3 | 4 | | IV-1 | Duox2 | 50506 | 24-May-15 |
| 6269 | 2 | 3 | 4 | | IV-1 | Dusp1 | 1843 | 7-Jun-15 |
| 6270 | 2 | 3 | 4 | | IV-1 | Dusp10 | 11221 | 4-May-15 |
| 6274 | 2 | 3 | 4 | | IV-1 | Dusp14 | 11072 | 4-May-15 |
| 6277 | 2 | 3 | 4 | | IV-1 | Dusp18 | 150290 | 12-May-15 |
| 6279 | 2 | 3 | 4 | | IV-1 | Dusp2 | 1844 | 4-May-15 |
| 6283 | 2 | 3 | 4 | | IV-1 | Dusp26 | 78986 | 7-Jun-15 |
| 6284 | 2 | 3 | 4 | | IV-1 | Dusp27 | 92235 | 4-May-15 |
| 6285 | 2 | 3 | 4 | | IV-1 | Dusp28 | 285193 | 4-May-15 |
| 6287 | 2 | 3 | 4 | | IV-1 | Dusp4 | 1846 | 4-May-15 |
| 6290 | 2 | 3 | 4 | | IV-1 | Dusp7 | 1849 | 4-May-15 |
| 6291 | 2 | 3 | 4 | | IV-1 | Dusp8 | 1850 | 4-May-15 |
| 6295 | 2 | 3 | 4 | | IV-1 | Duxbl1 | | |
| 6304 | 2 | 3 | 4 | | IV-1 | Dydc2 | 84332 | 4-May-15 |
| 6314 | 2 | 3 | 4 | | IV-1 | Dynll1 | 8655 | 24-May-15 |
| 6317 | 2 | 3 | 4 | | IV-1 | Dynlrb2 | 83657 | 12-May-15 |
| 6321 | 2 | 3 | 4 | | IV-1 | Dynlt1f | | |
| 6326 | 2 | 3 | 4 | | IV-1 | Dyrk3 | 8444 | 4-May-15 |
| 6332 | 2 | 3 | 4 | | IV-1 | Dzip1 | 22873 | 4-May-15 |
| 6333 | 2 | 3 | 4 | | IV-1 | Dzip1l | 199221 | 4-May-15 |
| 6337 | 2 | 3 | 4 | | IV-1 | E030011O05Rik | | |
| 6348 | 2 | 3 | 4 | | IV-1 | E130012A19Rik | | |
| 6351 | 2 | 3 | 4 | | IV-1 | E130112N10Rik | | |
| 6360 | 2 | 3 | 4 | | IV-1 | E130309D14Rik | | |
| 6362 | 2 | 3 | 4 | | IV-1 | E130310I04Rik | | |
| 6370 | 2 | 3 | 4 | | IV-1 | E230029C05Rik | | |
| 6371 | 2 | 3 | 4 | | IV-1 | E2f1 | 1869 | 7-Jun-15 |
| 6378 | 2 | 3 | 4 | | IV-1 | E2f8 | 79733 | 12-May-15 |
| 6382 | 2 | 3 | 4 | | IV-1 | E330013P04Rik | | |
| 6396 | 2 | 3 | 4 | | IV-1 | E530011L22Rik | | |
| 6403 | 2 | 3 | 4 | | IV-1 | Ear2 | 2063 | 4-May-15 |
| 6423 | 2 | 3 | 4 | | IV-1 | Echdc1 | 55862 | 4-May-15 |
| 6425 | 2 | 3 | 4 | | IV-1 | Echdc3 | 79746 | 4-May-15 |
| 6429 | 2 | 3 | 4 | | IV-1 | Eci3 | | |
| 6431 | 2 | 3 | 4 | | IV-1 | Ecm2 | 1842 | 4-May-15 |
| 6432 | 2 | 3 | 4 | | IV-1 | Ecscr | 641700 | 4-May-15 |
| 6436 | 2 | 3 | 4 | | IV-1 | Eda | 1896 | 24-May-15 |
| 6448 | 2 | 3 | 4 | | IV-1 | Edn1 | 1906 | 24-May-15 |
| 6450 | 2 | 3 | 4 | | IV-1 | Edn3 | 1908 | 23-May-15 |
| 6455 | 2 | 3 | 4 | | IV-1 | Eed | 8726 | 2-Jun-15 |
| 6463 | 2 | 3 | 4 | | IV-1 | Eef2k | 29904 | 4-May-15 |
| 6465 | 2 | 3 | 4 | | IV-1 | Eepd1 | 80820 | 4-May-15 |
| 6467 | 2 | 3 | 4 | | IV-1 | Efcab10 | 100130771 | 12-May-15 |
| 6473 | 2 | 3 | 4 | | IV-1 | Efcab4a | 283229 | 4-May-15 |
| 6474 | 2 | 3 | 4 | | IV-1 | Efcab4b | 84766 | 4-May-15 |
| 6481 | 2 | 3 | 4 | | IV-1 | Efemp1 | 2202 | 29-May-15 |
| 6482 | 2 | 3 | 4 | | IV-1 | Efemp2 | 30008 | 23-May-15 |
| 6488 | 2 | 3 | 4 | | IV-1 | Efna1 | 1942 | 14-May-15 |
| 6501 | 2 | 3 | 4 | | IV-1 | Egf | 1950 | 24-May-15 |
| 6502 | 2 | 3 | 4 | | IV-1 | Egfbp2 | | |
| 6504 | 2 | 3 | 4 | | IV-1 | Egfl6 | 25975 | 4-May-15 |
| 6511 | 2 | 3 | 4 | | IV-1 | Egln3 | 112399 | 12-May-15 |
| 6512 | 2 | 3 | 4 | | IV-1 | Egr1 | 1958 | 24-May-15 |
| 6514 | 2 | 3 | 4 | | IV-1 | Egr3 | 1960 | 12-May-15 |
| 6522 | 2 | 3 | 4 | | IV-1 | Ehf | 26298 | 4-May-15 |
| 6523 | 2 | 3 | 4 | | IV-1 | Ehhadh | 1962 | 12-May-15 |
| 6530 | 2 | 3 | 4 | | IV-1 | Eid3 | 493861 | 7-Jun-15 |
| 6573 | 2 | 3 | 4 | | IV-1 | Eif4ebp1 | 1978 | 29-May-15 |
| 6584 | 2 | 3 | 4 | | IV-1 | Eif5b | 9669 | 12-May-15 |
| 6588 | 2 | 3 | 4 | | IV-1 | Elane | 1991 | 23-May-15 |
| 6595 | 2 | 3 | 4 | | IV-1 | Elf3 | 1999 | 12-May-15 |
| 6602 | 2 | 3 | 4 | | IV-1 | Elk4 | 2005 | 28-May-15 |
| 6609 | 2 | 3 | 4 | | IV-1 | Elmod1 | 55531 | 12-May-15 |
| 6613 | 2 | 3 | 4 | | IV-1 | Eln | 2006 | 23-May-15 |
| 6620 | 2 | 3 | 4 | | IV-1 | Elovl6 | 79071 | 4-May-15 |
| 6627 | 2 | 3 | 4 | | IV-1 | Eltd1 | 64123 | 12-May-15 |
| 6628 | 2 | 3 | 4 | | IV-1 | Emb | 5463 | 4-May-15 |
| 6637 | 2 | 3 | 4 | | IV-1 | Emc9 | 51016 | 4-May-15 |
| 6645 | 2 | 3 | 4 | | IV-1 | Emilin2 | 84034 | 4-May-15 |
| 6655 | 2 | 3 | 4 | | IV-1 | Emp3 | 2014 | 4-May-15 |
| 6656 | 2 | 3 | 4 | | IV-1 | Emr1 | 2015 | 4-May-15 |
| 6665 | 2 | 3 | 4 | | IV-1 | Enc1 | 8507 | 4-May-15 |
| 6672 | 2 | 3 | 4 | | IV-1 | Enho | 375704 | 4-May-15 |
| 6674 | 2 | 3 | 4 | | IV-1 | Enkur | 219670 | 4-May-15 |
| 6675 | 2 | 3 | 4 | | IV-1 | Eno1 | 2023 | 24-May-15 |
| 6685 | 2 | 3 | 4 | | IV-1 | Enpp2 | 5168 | 12-May-15 |
| 6688 | 2 | 3 | 4 | | IV-1 | Enpp5 | 59084 | 4-May-15 |
| 6696 | 2 | 3 | 4 | | IV-1 | Entpd3 | 956 | 4-May-15 |
| 6700 | 2 | 3 | 4 | | IV-1 | Entpd7 | 57089 | 4-May-15 |
| 6709 | 2 | 3 | 4 | | IV-1 | Epb4.1l1 | | |
| 6711 | 2 | 3 | 4 | | IV-1 | Epb4.1l3 | | |
| 6719 | 2 | 3 | 4 | | IV-1 | Epdr1 | 54749 | 4-May-15 |
| 6721 | 2 | 3 | 4 | | IV-1 | Epgn | 255324 | 4-May-15 |
| 6722 | 2 | 3 | 4 | | IV-1 | Epha1 | 2041 | 12-May-15 |
| 6735 | 2 | 3 | 4 | | IV-1 | Ephb6 | 2051 | 17-May-15 |
| 6736 | 2 | 3 | 4 | | IV-1 | Ephx1 | 2052 | 17-May-15 |
| 6737 | 2 | 3 | 4 | | IV-1 | Ephx2 | 2053 | 12-May-15 |
| 6740 | 2 | 3 | 4 | | IV-1 | Epm2a | 7957 | 13-Jun-15 |
| 6744 | 2 | 3 | 4 | | IV-1 | Epn3 | 55040 | 4-May-15 |
| 6746 | 2 | 3 | 4 | | IV-1 | Epor | 2057 | 12-May-15 |
| 6748 | 2 | 3 | 4 | | IV-1 | Eppk1 | 83481 | 4-May-15 |
| 6756 | 2 | 3 | 4 | | IV-1 | Epsti1 | 94240 | 4-May-15 |
| 6766 | 2 | 3 | 4 | | IV-1 | Erbb3 | 2065 | 17-May-15 |
| 6803 | 2 | 3 | 4 | | IV-1 | Ern1 | 2081 | 24-May-15 |
| 6810 | 2 | 3 | 4 | | IV-1 | Errfi1 | 54206 | 4-May-15 |
| 6812 | 2 | 3 | 4 | | IV-1 | Esam | 90952 | 4-May-15 |
| 6815 | 2 | 3 | 4 | | IV-1 | Esd | 2098 | 4-May-15 |
| 6835 | 2 | 3 | 4 | | IV-1 | Espn | 83715 | 23-May-15 |
| 6843 | 2 | 3 | 4 | | IV-1 | Esrrg | 2104 | 4-May-15 |
| 6847 | 2 | 3 | 4 | | IV-1 | Esyt3 | 83850 | 4-May-15 |
| 6863 | 2 | 3 | 4 | | IV-1 | Etv1 | 2115 | 24-May-15 |
| 6872 | 2 | 3 | 4 | | IV-1 | Eva1c | 59271 | 4-May-15 |
| 6873 | 2 | 3 | 4 | | IV-1 | Evc | 2121 | 12-May-15 |
| 6874 | 2 | 3 | 4 | | IV-1 | Evc2 | 132884 | 21-May-15 |
| 6875 | 2 | 3 | 4 | | IV-1 | Evi2a | 2123 | 12-May-15 |
| 6907 | 2 | 3 | 4 | | IV-1 | Exosc6 | 118460 | 23-May-15 |
| 6914 | 2 | 3 | 4 | | IV-1 | Extl1 | 2134 | 12-May-15 |
| 6917 | 2 | 3 | 4 | | IV-1 | Eya1 | 2138 | 23-May-15 |
| 6923 | 2 | 3 | 4 | | IV-1 | Ezr | 7430 | 24-May-15 |
| 6930 | 2 | 3 | 4 | | IV-1 | F2 | 2147 | 31-May-15 |
| 6932 | 2 | 3 | 4 | | IV-1 | F2rl1 | 2150 | 4-May-15 |
| 6935 | 2 | 3 | 4 | | IV-1 | F3 | 2152 | 7-Jun-15 |
| 6942 | 2 | 3 | 4 | | IV-1 | F7 | 2155 | 12-May-15 |
| 6946 | 2 | 3 | 4 | | IV-1 | F830002L21Rik | | |
| 6947 | 2 | 3 | 4 | | IV-1 | F830016B08Rik | | |
| 6952 | 2 | 3 | 4 | | IV-1 | Fa2h | 79152 | 31-May-15 |
| 6957 | 2 | 3 | 4 | | IV-1 | Fabp3 | 2170 | 24-May-15 |
| 6959 | 2 | 3 | 4 | | IV-1 | Fabp5 | 2171 | 7-Jun-15 |
| 6960 | 2 | 3 | 4 | | IV-1 | Fabp6 | 2172 | 4-May-15 |
| 6961 | 2 | 3 | 4 | | IV-1 | Fabp7 | 2173 | 12-May-15 |
| 6962 | 2 | 3 | 4 | | IV-1 | Fabp9 | 646480 | 4-May-15 |
| 6965 | 2 | 3 | 4 | | IV-1 | Fads2 | 9415 | 4-May-15 |
| 6966 | 2 | 3 | 4 | | IV-1 | Fads3 | 3995 | 4-May-15 |
| 6970 | 2 | 3 | 4 | | IV-1 | Fah | 2184 | 7-Jun-15 |
| 6976 | 2 | 3 | 4 | | IV-1 | Fam101a | 144347 | 4-May-15 |
| 6983 | 2 | 3 | 4 | | IV-1 | Fam107a | 11170 | 12-May-15 |
| 6985 | 2 | 3 | 4 | | IV-1 | Fam109a | 144717 | 4-May-15 |
| 6986 | 2 | 3 | 4 | | IV-1 | Fam109b | 150368 | 4-May-15 |
| 6989 | 2 | 3 | 4 | | IV-1 | Fam110c | 642273 | 4-May-15 |
| 6996 | 2 | 3 | 4 | | IV-1 | Fam117a | 81558 | 4-May-15 |
| 7009 | 2 | 3 | 4 | | IV-1 | Fam126a | 84668 | 23-May-15 |
| 7017 | 2 | 3 | 4 | | IV-1 | Fam132a | 388581 | 4-May-15 |
| 7018 | 2 | 3 | 4 | | IV-1 | Fam132b | 151176 | 12-May-15 |
| 7021 | 2 | 3 | 4 | | IV-1 | Fam134b | 54463 | 23-May-15 |
| 7026 | 2 | 3 | 4 | | IV-1 | Fam13a | 10144 | 24-May-15 |
| 7052 | 2 | 3 | 4 | | IV-1 | Fam167a | 83648 | 4-May-15 |
| 7056 | 2 | 3 | 4 | | IV-1 | Fam169a | 26049 | 12-May-15 |
| 7061 | 2 | 3 | 4 | | IV-1 | Fam171a2 | 284069 | 12-May-15 |
| 7077 | 2 | 3 | 4 | | IV-1 | Fam183b | 340286 | 21-May-15 |
| 7079 | 2 | 3 | 4 | | IV-1 | Fam184b | 27146 | 4-May-15 |
| 7083 | 2 | 3 | 4 | | IV-1 | Fam187b | 148109 | 4-May-15 |
| 7092 | 2 | 3 | 4 | | IV-1 | Fam195a | 84331 | 4-May-15 |
| 7097 | 2 | 3 | 4 | | IV-1 | Fam198b | 51313 | 4-May-15 |
| 7103 | 2 | 3 | 4 | | IV-1 | Fam19a5 | 25817 | 4-May-15 |
| 7113 | 2 | 3 | 4 | | IV-1 | Fam20c | 56975 | 17-May-15 |
| 7119 | 2 | 3 | 4 | | IV-1 | Fam213a | 84293 | 4-May-15 |
| 7120 | 2 | 3 | 4 | | IV-1 | Fam213b | 127281 | 4-May-15 |
| 7133 | 2 | 3 | 4 | | IV-1 | Fam222a | 84915 | 4-May-15 |
| 7150 | 2 | 3 | 4 | | IV-1 | Fam3c | 10447 | 4-May-15 |
| 7154 | 2 | 3 | 4 | | IV-1 | Fam46a | 55603 | 12-May-15 |
| 7155 | 2 | 3 | 4 | | IV-1 | Fam46b | 115572 | 4-May-15 |
| 7156 | 2 | 3 | 4 | | IV-1 | Fam46c | 54855 | 4-May-15 |
| 7167 | 2 | 3 | 4 | | IV-1 | Fam57a | 79850 | 4-May-15 |
| 7178 | 2 | 3 | 4 | | IV-1 | Fam69b | 138311 | 4-May-15 |
| 7181 | 2 | 3 | 4 | | IV-1 | Fam71b | 153745 | 4-May-15 |
| 7183 | 2 | 3 | 4 | | IV-1 | Fam71e1 | 112703 | 12-May-15 |
| 7186 | 2 | 3 | 4 | | IV-1 | Fam71f2 | 346653 | 4-May-15 |
| 7188 | 2 | 3 | 4 | | IV-1 | Fam73a | 374986 | 4-May-15 |
| 7192 | 2 | 3 | 4 | | IV-1 | Fam78a | 286336 | 4-May-15 |

Fig.22 - 27

| | | | | | | | | |
|---|---|---|---|---|---|---|---|---|
| 7196 | 2 | 3 | 4 | | IV-1 | Fam83b | 222584 | 4-May-15 |
| 7202 | 2 | 3 | 4 | | IV-1 | Fam83h | 286077 | 21-May-15 |
| 7204 | 2 | 3 | 4 | | IV-1 | Fam84b | 157638 | 4-May-15 |
| 7206 | 2 | 3 | 4 | | IV-1 | Fam89a | 375061 | 4-May-15 |
| 7214 | 2 | 3 | 4 | | IV-1 | Fam98c | 147965 | 4-May-15 |
| 7230 | 2 | 3 | 4 | | IV-1 | Far2 | 55711 | 14-May-15 |
| 7233 | 2 | 3 | 4 | | IV-1 | Fars2 | 10667 | 21-May-15 |
| 7237 | 2 | 3 | 4 | | IV-1 | Fasl | 356 | 24-May-15 |
| 7238 | 2 | 3 | 4 | | IV-1 | Fasn | 2194 | 4-May-15 |
| 7253 | 2 | 3 | 4 | | IV-1 | Fblim1 | 54751 | 4-May-15 |
| 7257 | 2 | 3 | 4 | | IV-1 | Fbln5 | 10516 | 23-May-15 |
| 7261 | 2 | 3 | 4 | | IV-1 | Fbp1 | 2203 | 12-May-15 |
| 7262 | 2 | 3 | 4 | | IV-1 | Fbp2 | 8789 | 7-Jun-15 |
| 7272 | 2 | 3 | 4 | | IV-1 | Fbxl18 | 80028 | 4-May-15 |
| 7284 | 2 | 3 | 4 | | IV-1 | Fbxo10 | 26267 | 4-May-15 |
| 7290 | 2 | 3 | 4 | | IV-1 | Fbxo2 | 26232 | 12-May-15 |
| 7298 | 2 | 3 | 4 | | IV-1 | Fbxo30 | 84085 | 7-Jun-15 |
| 7299 | 2 | 3 | 4 | | IV-1 | Fbxo31 | 79791 | 7-Jun-15 |
| 7300 | 2 | 3 | 4 | | IV-1 | Fbxo32 | 114907 | 12-May-15 |
| 7303 | 2 | 3 | 4 | | IV-1 | Fbxo36 | 130888 | 4-May-15 |
| 7339 | 2 | 3 | 4 | | IV-1 | Fbxw7 | 55294 | 12-May-15 |
| 7344 | 2 | 3 | 4 | | IV-1 | Fcer1g | 2207 | 12-May-15 |
| 7345 | 2 | 3 | 4 | | IV-1 | Fcer2a | | |
| 7346 | 2 | 3 | 4 | | IV-1 | Fcf1 | 51077 | 12-May-15 |
| 7348 | 2 | 3 | 4 | | IV-1 | Fcgr1 | | |
| 7350 | 2 | 3 | 4 | | IV-1 | Fcgr3 | 2214, 2215 | 7-Jun-15 |
| 7351 | 2 | 3 | 4 | | IV-1 | Fcgr4 | | |
| 7357 | 2 | 3 | 4 | | IV-1 | Fcna | | |
| 7359 | 2 | 3 | 4 | | IV-1 | Fcrl1 | 115350 | 4-May-15 |
| 7365 | 2 | 3 | 4 | | IV-1 | Fdft1 | 2222 | 12-May-15 |
| 7366 | 2 | 3 | 4 | | IV-1 | Fdps | 2224 | 12-May-15 |
| 7371 | 2 | 3 | 4 | | IV-1 | Fech | 2235 | 23-May-15 |
| 7372 | 2 | 3 | 4 | | IV-1 | Fem1a | 55527 | 7-Jun-15 |
| 7384 | 2 | 3 | 4 | | IV-1 | Fes | 2242 | 12-May-15 |
| 7385 | 2 | 3 | 4 | | IV-1 | Fetub | 26998 | 12-May-15 |
| 7391 | 2 | 3 | 4 | | IV-1 | Ffar1 | 2864 | 17-May-15 |
| 7394 | 2 | 3 | 4 | | IV-1 | Ffar4 | 338557 | 24-May-15 |
| 7397 | 2 | 3 | 4 | | IV-1 | Fgd1 | 2245 | 31-May-15 |
| 7400 | 2 | 3 | 4 | | IV-1 | Fgd4 | 121512 | 23-May-15 |
| 7406 | 2 | 3 | 4 | | IV-1 | Fgf12 | 2257 | 4-May-15 |
| 7407 | 2 | 3 | 4 | | IV-1 | Fgf13 | 2258 | 7-Jun-15 |
| 7412 | 2 | 3 | 4 | | IV-1 | Fgf18 | 8817 | 4-May-15 |
| 7413 | 2 | 3 | 4 | | IV-1 | Fgf2 | 2247 | 7-Jun-15 |
| 7415 | 2 | 3 | 4 | | IV-1 | Fgf21 | 26291 | 17-May-15 |
| 7426 | 2 | 3 | 4 | | IV-1 | Fgfbp3 | 143282 | 4-May-15 |
| 7427 | 2 | 3 | 4 | | IV-1 | Fgfr1 | 2260 | 23-May-15 |
| 7430 | 2 | 3 | 4 | | IV-1 | Fgfr2 | 2263 | 24-May-15 |
| 7436 | 2 | 3 | 4 | | IV-1 | Fgl1 | 2267 | 4-May-15 |
| 7442 | 2 | 3 | 4 | | IV-1 | Fhdc1 | 85462 | 21-May-15 |
| 7443 | 2 | 3 | 4 | | IV-1 | Fhit | 2272 | 31-May-15 |
| 7444 | 2 | 3 | 4 | | IV-1 | Fhl1 | 2273 | 7-Jun-15 |
| 7452 | 2 | 3 | 4 | | IV-1 | Fibin | 387758 | 4-May-15 |
| 7456 | 2 | 3 | 4 | | IV-1 | Figf | 2277 | 17-May-15 |
| 7459 | 2 | 3 | 4 | | IV-1 | Fignl1 | 63979 | 4-May-15 |
| 7461 | 2 | 3 | 4 | | IV-1 | Filip1 | 27145 | 4-May-15 |
| 7469 | 2 | 3 | 4 | | IV-1 | Fjx1 | 24147 | 4-May-15 |
| 7471 | 2 | 3 | 4 | | IV-1 | Fkbp11 | 51303 | 21-May-15 |
| 7475 | 2 | 3 | 4 | | IV-1 | Fkbp1b | 2281 | 4-May-15 |
| 7479 | 2 | 3 | 4 | | IV-1 | Fkbp5 | 2289 | 17-May-15 |
| 7505 | 2 | 3 | 4 | | IV-1 | Flywch2 | 114984 | 4-May-15 |
| 7511 | 2 | 3 | 4 | | IV-1 | Fmo1 | 2326 | 12-May-15 |
| 7518 | 2 | 3 | 4 | | IV-1 | Fmod | 2331 | 12-May-15 |
| 7521 | 2 | 3 | 4 | | IV-1 | Fn1 | 2335 | 24-May-15 |
| 7523 | 2 | 3 | 4 | | IV-1 | Fn3krp | 79672 | 4-May-15 |
| 7533 | 2 | 3 | 4 | | IV-1 | Fndc5 | 252995 | 17-May-15 |
| 7534 | 2 | 3 | 4 | | IV-1 | Fndc7 | 163479 | 4-May-15 |
| 7542 | 2 | 3 | 4 | | IV-1 | Folh1 | 2346 | 14-May-15 |
| 7543 | 2 | 3 | 4 | | IV-1 | Folr1 | 2348 | 24-May-15 |
| 7544 | 2 | 3 | 4 | | IV-1 | Folr2 | 2350 | 12-May-15 |
| 7547 | 2 | 3 | 4 | | IV-1 | Fos | 2353 | 4-May-15 |
| 7548 | 2 | 3 | 4 | | IV-1 | Fosb | 2354 | 12-May-15 |
| 7550 | 2 | 3 | 4 | | IV-1 | Fosl2 | 2355 | 12-May-15 |
| 7556 | 2 | 3 | 4 | | IV-1 | Foxc1 | 2296 | 4-May-15 |
| 7561 | 2 | 3 | 4 | | IV-1 | Foxd3 | 27022 | 17-May-15 |
| 7567 | 2 | 3 | 4 | | IV-1 | Foxg1 | 2290 | 27-May-15 |
| 7578 | 2 | 3 | 4 | | IV-1 | Foxl2 | 668 | 28-May-15 |
| 7579 | 2 | 3 | 4 | | IV-1 | Foxl2os | | |
| 7586 | 2 | 3 | 4 | | IV-1 | Foxo3 | 2309 | 31-May-15 |
| 7593 | 2 | 3 | 4 | | IV-1 | Foxq1 | 94234 | 28-May-15 |
| 7597 | 2 | 3 | 4 | | IV-1 | Foxred2 | 80020 | 4-May-15 |
| 7599 | 2 | 3 | 4 | | IV-1 | Fpgs | 2356 | 12-May-15 |
| 7610 | 2 | 3 | 4 | | IV-1 | Frat2 | 23401 | 4-May-15 |
| 7613 | 2 | 3 | 4 | | IV-1 | Frem3 | 166752 | 12-May-15 |
| 7615 | 2 | 3 | 4 | | IV-1 | Frk | 2444 | 4-May-15 |
| 7619 | 2 | 3 | 4 | | IV-1 | Frmd5 | 84978 | 4-May-15 |
| 7623 | 2 | 3 | 4 | | IV-1 | Frmpd1 | 22844 | 4-May-15 |
| 7624 | 2 | 3 | 4 | | IV-1 | Frmpd1os | | |
| 7642 | 2 | 3 | 4 | | IV-1 | Fsd2 | 123722 | 4-May-15 |
| 7646 | 2 | 3 | 4 | | IV-1 | Fst | 10468 | 12-May-15 |
| 7667 | 2 | 3 | 4 | | IV-1 | Fundc2 | 65991 | 12-May-15 |
| 7674 | 2 | 3 | 4 | | IV-1 | Fut2 | 2524 | 31-May-15 |
| 7682 | 2 | 3 | 4 | | IV-1 | Fxn | 2395 | 28-May-15 |
| 7685 | 2 | 3 | 4 | | IV-1 | Fxyd1 | 5348 | 12-May-15 |
| 7686 | 2 | 3 | 4 | | IV-1 | Fxyd2 | 486 | 4-May-15 |
| 7687 | 2 | 3 | 4 | | IV-1 | Fxyd3 | 5349 | 4-May-15 |
| 7697 | 2 | 3 | 4 | | IV-1 | Fzd10 | 11211 | 4-May-15 |
| 7700 | 2 | 3 | 4 | | IV-1 | Fzd4 | 8322 | 23-May-15 |

| | | | | | | | | |
|---|---|---|---|---|---|---|---|---|
| 7707 | 2 | 3 | 4 | | IV-1 | G0s2 | 50486 | 4-May-15 |
| 7712 | 2 | 3 | 4 | | IV-1 | G630025P09Rik | | |
| 7717 | 2 | 3 | 4 | | IV-1 | G6b | 80739 | 4-May-15 |
| 7719 | 2 | 3 | 4 | | IV-1 | G6pc | 2538 | 23-May-15 |
| 7726 | 2 | 3 | 4 | | IV-1 | Gab1 | 2549 | 7-Jun-15 |
| 7759 | 2 | 3 | 4 | | IV-1 | Gadd45a | 1647 | 24-May-15 |
| 7761 | 2 | 3 | 4 | | IV-1 | Gadd45g | 10912 | 4-May-15 |
| 7766 | 2 | 3 | 4 | | IV-1 | Gal3st1 | 9514 | 4-May-15 |
| 7781 | 2 | 3 | 4 | | IV-1 | Galnt14 | 79623 | 14-May-15 |
| 7782 | 2 | 3 | 4 | | IV-1 | Galnt15 | 117248 | 7-Jun-15 |
| 7786 | 2 | 3 | 4 | | IV-1 | Galnt3 | 2591 | 4-May-15 |
| 7789 | 2 | 3 | 4 | | IV-1 | Galnt6 | 11226 | 4-May-15 |
| 7800 | 2 | 3 | 4 | | IV-1 | Gan | 8139 | 23-May-15 |
| 7805 | 2 | 3 | 4 | | IV-1 | Gapdhs | 26330 | 12-May-15 |
| 7809 | 2 | 3 | 4 | | IV-1 | Garem | 64762 | 4-May-15 |
| 7814 | 2 | 3 | 4 | | IV-1 | Gas1 | 2619 | 4-May-15 |
| 7820 | 2 | 3 | 4 | | IV-1 | Gas6 | 2621 | 18-May-15 |
| 7824 | 2 | 3 | 4 | | IV-1 | Gata1 | 2623 | 23-May-15 |
| 7833 | 2 | 3 | 4 | | IV-1 | Gatad2b | 57459 | 4-May-15 |
| 7837 | 2 | 3 | 4 | | IV-1 | Gatsl3 | 652968 | 4-May-15 |
| 7859 | 2 | 3 | 4 | | IV-1 | Gcat | 23464 | 4-May-15 |
| 7867 | 2 | 3 | 4 | | IV-1 | Gchfr | 2644 | 4-May-15 |
| 7871 | 2 | 3 | 4 | | IV-1 | Gclm | 2730 | 21-May-15 |
| 7875 | 2 | 3 | 4 | | IV-1 | Gcnt1 | 2650 | 4-May-15 |
| 7884 | 2 | 3 | 4 | | IV-1 | Gdap10 | | |
| 7889 | 2 | 3 | 4 | | IV-1 | Gdf10 | 2662 | 4-May-15 |
| 7891 | 2 | 3 | 4 | | IV-1 | Gdf15 | 9518 | 4-May-15 |
| 7894 | 2 | 3 | 4 | | IV-1 | Gdf5 | 8200 | 28-May-15 |
| 7902 | 2 | 3 | 4 | | IV-1 | Gdpd2 | 54857 | 4-May-15 |
| 7906 | 2 | 3 | 4 | | IV-1 | Gdpgp1 | 390637 | 12-May-15 |
| 7907 | 2 | 3 | 4 | | IV-1 | Gem | 2669 | 7-Jun-15 |
| 7916 | 2 | 3 | 4 | | IV-1 | Gfap | 2670 | 23-May-15 |
| 7919 | 2 | 3 | 4 | | IV-1 | Gfi1b | 8328 | 23-May-15 |
| 7929 | 2 | 3 | 4 | | IV-1 | Gfra4 | 64096 | 4-May-15 |
| 7938 | 2 | 3 | 4 | | IV-1 | Ggh | 8836 | 12-May-15 |
| 7940 | 2 | 3 | 4 | | IV-1 | Ggnbp1 | 449520 | 4-May-15 |
| 7942 | 2 | 3 | 4 | | IV-1 | Ggps1 | 9453 | 4-May-15 |
| 7943 | 2 | 3 | 4 | | IV-1 | Ggt1 | 2678 | 17-May-15 |
| 7946 | 2 | 3 | 4 | | IV-1 | Ggt7 | 2686 | 4-May-15 |
| 7951 | 2 | 3 | 4 | | IV-1 | Ghr | 2690 | 24-May-15 |
| 7962 | 2 | 3 | 4 | | IV-1 | Gimap3 | 474345 | 4-May-15 |
| 7963 | 2 | 3 | 4 | | IV-1 | Gimap4 | 55303 | 4-May-15 |
| 7966 | 2 | 3 | 4 | | IV-1 | Gimap7 | 168537 | 4-May-15 |
| 7971 | 2 | 3 | 4 | | IV-1 | Gins1 | 9837 | 12-May-15 |
| 7972 | 2 | 3 | 4 | | IV-1 | Gins2 | 51659 | 4-May-15 |
| 7977 | 2 | 3 | 4 | | IV-1 | Gipc2 | 54810 | 4-May-15 |
| 7978 | 2 | 3 | 4 | | IV-1 | Gipc3 | 126326 | 4-May-15 |
| 7989 | 2 | 3 | 4 | | IV-1 | Gjb1 | 2705 | 23-May-15 |
| 7991 | 2 | 3 | 4 | | IV-1 | Gjb3 | 2707 | 23-May-15 |
| 7992 | 2 | 3 | 4 | | IV-1 | Gjb4 | 127534 | 4-May-15 |
| 7993 | 2 | 3 | 4 | | IV-1 | Gjb5 | 2709 | 4-May-15 |
| 8002 | 2 | 3 | 4 | | IV-1 | Gk2 | 2712 | 12-May-15 |
| 8003 | 2 | 3 | 4 | | IV-1 | Gk5 | 256356 | 4-May-15 |
| 8004 | 2 | 3 | 4 | | IV-1 | Gkap1 | 80318 | 12-May-15 |
| 8006 | 2 | 3 | 4 | | IV-1 | Gkn2 | 200504 | 4-May-15 |
| 8009 | 2 | 3 | 4 | | IV-1 | Glb1 | 2720 | 12-May-15 |
| 8011 | 2 | 3 | 4 | | IV-1 | Glb1l2 | 89944 | 4-May-15 |
| 8023 | 2 | 3 | 4 | | IV-1 | Glipr1 | 11010 | 4-May-15 |
| 8026 | 2 | 3 | 4 | | IV-1 | Glipr2 | 152007 | 4-May-15 |
| 8040 | 2 | 3 | 4 | | IV-1 | Glrb | 2743 | 4-May-15 |
| 8045 | 2 | 3 | 4 | | IV-1 | Glrx5 | 51218 | 4-May-15 |
| 8060 | 2 | 3 | 4 | | IV-1 | Glud1 | 2746 | 7-Jun-15 |
| 8061 | 2 | 3 | 4 | | IV-1 | Glul | 2752 | 4-May-15 |
| 8064 | 2 | 3 | 4 | | IV-1 | Glycam1 | 644076 | 4-May-15 |
| 8077 | 2 | 3 | 4 | | IV-1 | Gm10094 | | |
| 8083 | 2 | 3 | 4 | | IV-1 | Gm10142 | | |
| 8087 | 2 | 3 | 4 | | IV-1 | Gm10228 | | |
| 8088 | 2 | 3 | 4 | | IV-1 | Gm10229 | | |
| 8117 | 2 | 3 | 4 | | IV-1 | Gm10439 | | |
| 8121 | 2 | 3 | 4 | | IV-1 | Gm10451 | | |
| 8139 | 2 | 3 | 4 | | IV-1 | Gm10560 | | |
| 8147 | 2 | 3 | 4 | | IV-1 | Gm10638 | | |
| 8151 | 2 | 3 | 4 | | IV-1 | Gm10653 | | |
| 8172 | 2 | 3 | 4 | | IV-1 | Gm10790 | | |
| 8176 | 2 | 3 | 4 | | IV-1 | Gm10823 | | |
| 8181 | 2 | 3 | 4 | | IV-1 | Gm10865 | | |
| 8182 | 2 | 3 | 4 | | IV-1 | Gm10872 | | |
| 8188 | 2 | 3 | 4 | | IV-1 | Gm11127 | | |
| 8208 | 2 | 3 | 4 | | IV-1 | Gm11517 | | |
| 8216 | 2 | 3 | 4 | | IV-1 | Gm11554 | | |
| 8217 | 2 | 3 | 4 | | IV-1 | Gm11559 | | |
| 8218 | 2 | 3 | 4 | | IV-1 | Gm11562 | | |
| 8219 | 2 | 3 | 4 | | IV-1 | Gm11563 | | |
| 8220 | 2 | 3 | 4 | | IV-1 | Gm11564 | | |
| 8222 | 2 | 3 | 4 | | IV-1 | Gm11567 | | |
| 8223 | 2 | 3 | 4 | | IV-1 | Gm11568 | | |
| 8226 | 2 | 3 | 4 | | IV-1 | Gm11595 | | |
| 8227 | 2 | 3 | 4 | | IV-1 | Gm11596 | | |
| 8231 | 2 | 3 | 4 | | IV-1 | Gm11710 | | |
| 8232 | 2 | 3 | 4 | | IV-1 | Gm11744 | | |
| 8238 | 2 | 3 | 4 | | IV-1 | Gm11837 | | |
| 8239 | 2 | 3 | 4 | | IV-1 | Gm11937 | | |
| 8240 | 2 | 3 | 4 | | IV-1 | Gm11938 | | |
| 8243 | 2 | 3 | 4 | | IV-1 | Gm11974 | | |
| 8249 | 2 | 3 | 4 | | IV-1 | Gm12060 | | |
| 8257 | 2 | 3 | 4 | | IV-1 | Gm12216 | | |
| 8264 | 2 | 3 | 4 | | IV-1 | Gm12359 | | |

Fig.22 - 28

| | | | | | | | | |
|---|---|---|---|---|---|---|---|---|
| 8288 | 2 | 3 | 4 | | IV-1 | Gm13011 | | |
| 8304 | 2 | 3 | 4 | | IV-1 | Gm13124 | | |
| 8307 | 2 | 3 | 4 | | IV-1 | Gm13139 | | |
| 8308 | 2 | 3 | 4 | | IV-1 | Gm13152 | | |
| 8311 | 2 | 3 | 4 | | IV-1 | Gm13177 | | |
| 8312 | 2 | 3 | 4 | | IV-1 | Gm13178 | | |
| 8335 | 2 | 3 | 4 | | IV-1 | Gm13306 | | |
| 8352 | 2 | 3 | 4 | | IV-1 | Gm13629 | | |
| 8353 | 2 | 3 | 4 | | IV-1 | Gm13710 | | |
| 8357 | 2 | 3 | 4 | | IV-1 | Gm13807 | | |
| 8363 | 2 | 3 | 4 | | IV-1 | Gm14005 | | |
| 8367 | 2 | 3 | 4 | | IV-1 | Gm14085 | | |
| 8370 | 2 | 3 | 4 | | IV-1 | Gm14137 | | |
| 8377 | 2 | 3 | 4 | | IV-1 | Gm14288 | | |
| 8378 | 2 | 3 | 4 | | IV-1 | Gm14295 | | |
| 8399 | 2 | 3 | 4 | | IV-1 | Gm14436 | | |
| 8401 | 2 | 3 | 4 | | IV-1 | Gm14446 | | |
| 8406 | 2 | 3 | 4 | | IV-1 | Gm14475 | | |
| 8419 | 2 | 3 | 4 | | IV-1 | Gm14548 | | |
| 8434 | 2 | 3 | 4 | | IV-1 | Gm14850 | | |
| 8435 | 2 | 3 | 4 | | IV-1 | Gm14851 | | |
| 8443 | 2 | 3 | 4 | | IV-1 | Gm15093 | | |
| 8454 | 2 | 3 | 4 | | IV-1 | Gm15284 | | |
| 8458 | 2 | 3 | 4 | | IV-1 | Gm15308 | | |
| 8476 | 2 | 3 | 4 | | IV-1 | Gm15545 | | |
| 8478 | 2 | 3 | 4 | | IV-1 | Gm15612 | | |
| 8484 | 2 | 3 | 4 | | IV-1 | Gm15706 | | |
| 8487 | 2 | 3 | 4 | | IV-1 | Gm15760 | | |
| 8492 | 2 | 3 | 4 | | IV-1 | Gm15850 | | |
| 8497 | 2 | 3 | 4 | | IV-1 | Gm15915 | | |
| 8526 | 2 | 3 | 4 | | IV-1 | Gm16501 | | |
| 8537 | 2 | 3 | 4 | | IV-1 | Gm16617 | | |
| 8545 | 2 | 3 | 4 | | IV-1 | Gm16793 | | |
| 8548 | 2 | 3 | 4 | | IV-1 | Gm16845 | | |
| 8550 | 2 | 3 | 4 | | IV-1 | Gm16861 | | |
| 8559 | 2 | 3 | 4 | | IV-1 | Gm16998 | | |
| 8588 | 2 | 3 | 4 | | IV-1 | Gm19299 | | |
| 8606 | 2 | 3 | 4 | | IV-1 | Gm19668 | | |
| 8609 | 2 | 3 | 4 | | IV-1 | Gm19705 | | |
| 8639 | 2 | 3 | 4 | | IV-1 | Gm20319 | | |
| 8662 | 2 | 3 | 4 | | IV-1 | Gm20748 | | |
| 8667 | 2 | 3 | 4 | | IV-1 | Gm20754 | | |
| 8694 | 2 | 3 | 4 | | IV-1 | Gm20939 | | |
| 8695 | 2 | 3 | 4 | | IV-1 | Gm21002 | | |
| 8711 | 2 | 3 | 4 | | IV-1 | Gm21586 | | |
| 8722 | 2 | 3 | 4 | | IV-1 | Gm2373 | | |
| 8746 | 2 | 3 | 4 | | IV-1 | Gm3086 | | |
| 8749 | 2 | 3 | 4 | | IV-1 | Gm3219 | | |
| 8758 | 2 | 3 | 4 | | IV-1 | Gm3317 | | |
| 8768 | 2 | 3 | 4 | | IV-1 | Gm3434 | | |
| 8788 | 2 | 3 | 4 | | IV-1 | Gm4013 | | |
| 8803 | 2 | 3 | 4 | | IV-1 | Gm4285 | | |
| 8814 | 2 | 3 | 4 | | IV-1 | Gm438 | | |
| 8820 | 2 | 3 | 4 | | IV-1 | Gm4532 | | |
| 8822 | 2 | 3 | 4 | | IV-1 | Gm4559 | | |
| 8842 | 2 | 3 | 4 | | IV-1 | Gm4836 | | |
| 8848 | 2 | 3 | 4 | | IV-1 | Gm4861 | | |
| 8852 | 2 | 3 | 4 | | IV-1 | Gm4890 | | |
| 8858 | 2 | 3 | 4 | | IV-1 | Gm4926 | | |
| 8867 | 2 | 3 | 4 | | IV-1 | Gm4980 | | |
| 8882 | 2 | 3 | 4 | | IV-1 | Gm5089 | | |
| 8886 | 2 | 3 | 4 | | IV-1 | Gm5108 | | |
| 8892 | 2 | 3 | 4 | | IV-1 | Gm5126 | | |
| 8905 | 2 | 3 | 4 | | IV-1 | Gm5176 | | |
| 8909 | 2 | 3 | 4 | | IV-1 | Gm5294 | | |
| 8910 | 2 | 3 | 4 | | IV-1 | Gm53 | | |
| 8922 | 2 | 3 | 4 | | IV-1 | Gm5434 | | |
| 8932 | 2 | 3 | 4 | | IV-1 | Gm5483 | | |
| 8933 | 2 | 3 | 4 | | IV-1 | Gm5485 | | |
| 8938 | 2 | 3 | 4 | | IV-1 | Gm5538 | | |
| 8955 | 2 | 3 | 4 | | IV-1 | Gm5643 | | |
| 8958 | 2 | 3 | 4 | | IV-1 | Gm572 | | |
| 8982 | 2 | 3 | 4 | | IV-1 | Gm590 | | |
| 8998 | 2 | 3 | 4 | | IV-1 | Gm6083 | | |
| 9000 | 2 | 3 | 4 | | IV-1 | Gm609 | | |
| 9012 | 2 | 3 | 4 | | IV-1 | Gm6268 | | |
| 9014 | 2 | 3 | 4 | | IV-1 | Gm6289 | | |
| 9017 | 2 | 3 | 4 | | IV-1 | Gm6307 | | |
| 9022 | 2 | 3 | 4 | | IV-1 | Gm6377 | | |
| 9031 | 2 | 3 | 4 | | IV-1 | Gm6484 | | |
| 9039 | 2 | 3 | 4 | | IV-1 | Gm6568 | | |
| 9046 | 2 | 3 | 4 | | IV-1 | Gm6614 | | |
| 9050 | 2 | 3 | 4 | | IV-1 | Gm6642 | | |
| 9054 | 2 | 3 | 4 | | IV-1 | Gm6696 | | |
| 9073 | 2 | 3 | 4 | | IV-1 | Gm694 | | |
| 9085 | 2 | 3 | 4 | | IV-1 | Gm715 | | |
| 9089 | 2 | 3 | 4 | | IV-1 | Gm7244 | | |
| 9106 | 2 | 3 | 4 | | IV-1 | Gm766 | | |
| 9107 | 2 | 3 | 4 | | IV-1 | Gm7694 | | |
| 9133 | 2 | 3 | 4 | | IV-1 | Gm8369 | | |
| 9148 | 2 | 3 | 4 | | IV-1 | Gm8801 | | |
| 9155 | 2 | 3 | 4 | | IV-1 | Gm8909 | | |
| 9170 | 2 | 3 | 4 | | IV-1 | Gm933 | | |
| 9183 | 2 | 3 | 4 | | IV-1 | Gm9833 | | |
| 9188 | 2 | 3 | 4 | | IV-1 | Gm9895 | | |
| 9194 | 2 | 3 | 4 | | IV-1 | Gm9961 | | |
| 9197 | 2 | 3 | 4 | | IV-1 | Gm9994 | | |

| | | | | | | | | |
|---|---|---|---|---|---|---|---|---|
| 9220 | 2 | 3 | 4 | | IV-1 | Gnai1 | 2770 | 12-May-15 |
| 9228 | 2 | 3 | 4 | | IV-1 | Gnat2 | 2780 | 23-May-15 |
| 9229 | 2 | 3 | 4 | | IV-1 | Gnat3 | 346562 | 4-May-15 |
| 9235 | 2 | 3 | 4 | | IV-1 | Gnb3 | 2784 | 12-May-15 |
| 9242 | 2 | 3 | 4 | | IV-1 | Gng13 | 51764 | 4-May-15 |
| 9243 | 2 | 3 | 4 | | IV-1 | Gng2 | 54331 | 12-May-15 |
| 9245 | 2 | 3 | 4 | | IV-1 | Gng4 | 2786 | 4-May-15 |
| 9248 | 2 | 3 | 4 | | IV-1 | Gng8 | 94235 | #NAME? |
| 9250 | 2 | 3 | 4 | | IV-1 | Gngt2 | 2793 | 4-May-15 |
| 9253 | 2 | 3 | 4 | | IV-1 | Gnl3 | 26354 | 4-May-15 |
| 9255 | 2 | 3 | 4 | | IV-1 | Gnmt | 27232 | 12-May-15 |
| 9286 | 2 | 3 | 4 | | IV-1 | Got1 | 2805 | 7-Jun-15 |
| 9288 | 2 | 3 | 4 | | IV-1 | Got2 | 2806 | 4-May-15 |
| 9292 | 2 | 3 | 4 | | IV-1 | Gp49a | | |
| 9312 | 2 | 3 | 4 | | IV-1 | Gpc1 | 2817 | 3-May-15 |
| 9313 | 2 | 3 | 4 | | IV-1 | Gpc2 | 221914 | 4-May-15 |
| 9314 | 2 | 3 | 4 | | IV-1 | Gpc3 | 2719 | 23-May-15 |
| 9319 | 2 | 3 | 4 | | IV-1 | Gpd1 | 2819 | 4-May-15 |
| 9321 | 2 | 3 | 4 | | IV-1 | Gpd2 | 2820 | 12-May-15 |
| 9322 | 2 | 3 | 4 | | IV-1 | Gper1 | 2852 | 31-May-15 |
| 9323 | 2 | 3 | 4 | | IV-1 | Gpha2 | 170589 | 4-May-15 |
| 9327 | 2 | 3 | 4 | | IV-1 | Gpihbp1 | 338328 | 4-May-15 |
| 9336 | 2 | 3 | 4 | | IV-1 | Gpr1 | 2825 | 4-May-15 |
| 9343 | 2 | 3 | 4 | | IV-1 | Gpr114 | 221188 | 12-May-15 |
| 9345 | 2 | 3 | 4 | | IV-1 | Gpr116 | 221395 | 4-May-15 |
| 9354 | 2 | 3 | 4 | | IV-1 | Gpr133 | 283383 | 4-May-15 |
| 9357 | 2 | 3 | 4 | | IV-1 | Gpr137b | 7107 | 4-May-15 |
| 9387 | 2 | 3 | 4 | | IV-1 | Gpr182 | 11318 | 4-May-15 |
| 9395 | 2 | 3 | 4 | | IV-1 | Gpr27 | 2850 | 4-May-15 |
| 9398 | 2 | 3 | 4 | | IV-1 | Gpr33 | 2856 | 2-Jun-15 |
| 9403 | 2 | 3 | 4 | | IV-1 | Gpr39 | 2863 | 12-May-15 |
| 9406 | 2 | 3 | 4 | | IV-1 | Gpr50 | 9248 | 21-May-15 |
| 9414 | 2 | 3 | 4 | | IV-1 | Gpr64 | 10149 | 4-May-15 |
| 9415 | 2 | 3 | 4 | | IV-1 | Gpr65 | 8477 | 4-May-15 |
| 9416 | 2 | 3 | 4 | | IV-1 | Gpr68 | 8111 | 4-May-15 |
| 9422 | 2 | 3 | 4 | | IV-1 | Gpr87 | 53836 | 4-May-15 |
| 9428 | 2 | 3 | 4 | | IV-1 | Gprasp2 | 114928 | 4-May-15 |
| 9429 | 2 | 3 | 4 | | IV-1 | Gprc5a | 9052 | 4-May-15 |
| 9431 | 2 | 3 | 4 | | IV-1 | Gprc5c | 55890 | 12-May-15 |
| 9432 | 2 | 3 | 4 | | IV-1 | Gprc5d | 55507 | 4-May-15 |
| 9434 | 2 | 3 | 4 | | IV-1 | Gprin1 | 114787 | 4-May-15 |
| 9436 | 2 | 3 | 4 | | IV-1 | Gprin3 | 285513 | 14-May-15 |
| 9440 | 2 | 3 | 4 | | IV-1 | Gpsm2 | 29899 | 4-May-15 |
| 9442 | 2 | 3 | 4 | | IV-1 | Gpt | 2875 | 7-Jun-15 |
| 9443 | 2 | 3 | 4 | | IV-1 | Gpt2 | 84706 | 4-May-15 |
| 9445 | 2 | 3 | 4 | | IV-1 | Gpx2 | 2877 | 4-May-15 |
| 9447 | 2 | 3 | 4 | | IV-1 | Gpx3 | 2878 | 12-May-15 |
| 9454 | 2 | 3 | 4 | | IV-1 | Gramd1b | 57476 | 21-May-15 |
| 9458 | 2 | 3 | 4 | | IV-1 | Gramd4 | 23151 | 14-May-15 |
| 9465 | 2 | 3 | 4 | | IV-1 | Grb7 | 2886 | 12-May-15 |
| 9469 | 2 | 3 | 4 | | IV-1 | Grem1 | 26585 | 12-May-15 |
| 9471 | 2 | 3 | 4 | | IV-1 | Grhl1 | 29841 | 28-May-15 |
| 9477 | 2 | 3 | 4 | | IV-1 | Gria3 | 2892 | 12-May-15 |
| 9489 | 2 | 3 | 4 | | IV-1 | Grin1os | | |
| 9516 | 2 | 3 | 4 | | IV-1 | Grpel2 | 134266 | 4-May-15 |
| 9518 | 2 | 3 | 4 | | IV-1 | Grrp1 | 79927 | 4-May-15 |
| 9528 | 2 | 3 | 4 | | IV-1 | Gsdma2 | | |
| 9541 | 2 | 3 | 4 | | IV-1 | Gsg2 | 83903 | 12-May-15 |
| 9547 | 2 | 3 | 4 | | IV-1 | Gspt2 | 23708 | 21-May-15 |
| 9548 | 2 | 3 | 4 | | IV-1 | Gsr | 2936 | 4-May-15 |
| 9549 | 2 | 3 | 4 | | IV-1 | Gss | 2937 | 16-Jun-15 |
| 9552 | 2 | 3 | 4 | | IV-1 | Gsta3 | 2940 | 12-May-15 |
| 9555 | 2 | 3 | 4 | | IV-1 | Gstk1 | 373156 | 4-May-15 |
| 9557 | 2 | 3 | 4 | | IV-1 | Gstm2 | 2946 | 17-May-15 |
| 9559 | 2 | 3 | 4 | | IV-1 | Gstm4 | 2948 | 12-May-15 |
| 9560 | 2 | 3 | 4 | | IV-1 | Gstm5 | 2949 | 4-May-15 |
| 9561 | 2 | 3 | 4 | | IV-1 | Gstm6 | | |
| 9562 | 2 | 3 | 4 | | IV-1 | Gstm7 | | |
| 9563 | 2 | 3 | 4 | | IV-1 | Gsto1 | 9446 | 4-May-15 |
| 9566 | 2 | 3 | 4 | | IV-1 | Gstp2 | | |
| 9567 | 2 | 3 | 4 | | IV-1 | Gstt1 | 2952 | 24-May-15 |
| 9568 | 2 | 3 | 4 | | IV-1 | Gstt2 | 2953 | 12-May-15 |
| 9604 | 2 | 3 | 4 | | IV-1 | Gtpbp4 | 23560 | 4-May-15 |
| 9610 | 2 | 3 | 4 | | IV-1 | Guca1a | 2978 | 31-May-15 |
| 9616 | 2 | 3 | 4 | | IV-1 | Gucy1a3 | 2982 | 12-May-15 |
| 9625 | 2 | 3 | 4 | | IV-1 | Guk1 | 2987 | 4-May-15 |
| 9629 | 2 | 3 | 4 | | IV-1 | Gvin1 | 387751 | 4-May-15 |
| 9632 | 2 | 3 | 4 | | IV-1 | Gyg | 2992 | 12-May-15 |
| 9633 | 2 | 3 | 4 | | IV-1 | Gyk | | |
| 9635 | 2 | 3 | 4 | | IV-1 | Gyltl1b | 120071 | 23-May-15 |
| 9641 | 2 | 3 | 4 | | IV-1 | Gzma | 3001 | 21-May-15 |
| 9642 | 2 | 3 | 4 | | IV-1 | Gzmb | 3002 | 31-May-15 |
| 9654 | 2 | 3 | 4 | | IV-1 | H1fnt | 341567 | 4-May-15 |
| 9662 | 2 | 3 | 4 | | IV-1 | H2afj | 55766 | 7-Jun-15 |
| 9667 | 2 | 3 | 4 | | IV-1 | H2afy3 | | |
| 9670 | 2 | 3 | 4 | | IV-1 | H2-Bl | | |
| 9674 | 2 | 3 | 4 | | IV-1 | H2-DMb2 | | |
| 9677 | 2 | 3 | 4 | | IV-1 | H2-Eb2 | | |
| 9691 | 2 | 3 | 4 | | IV-1 | H2-M2 | | |
| 9694 | 2 | 3 | 4 | | IV-1 | H2-M9 | | |
| 9701 | 2 | 3 | 4 | | IV-1 | H2-Q5 | | |
| 9705 | 2 | 3 | 4 | | IV-1 | H2-Q9 | | |
| 9707 | 2 | 3 | 4 | | IV-1 | H2-T22 | | |
| 9711 | 2 | 3 | 4 | | IV-1 | H2-T9 | | |
| 9721 | 2 | 3 | 4 | | IV-1 | Hacl1 | 26061 | 7-Jun-15 |
| 9742 | 2 | 3 | 4 | | IV-1 | Has1 | 3036 | 4-May-15 |
| 9750 | 2 | 3 | 4 | | IV-1 | Haus4 | 54930 | 4-May-15 |

Fig.22 - 29

| | | | | | | | | |
|---|---|---|---|---|---|---|---|---|
| 9758 | 2 | 3 | 4 | | IV-1 | Hba-a1 | | |
| 9761 | 2 | 3 | 4 | | IV-1 | Hbb-b1 | 3043 | 9-Jun-15 |
| 9765 | 2 | 3 | 4 | | IV-1 | Hbb-bt | 3045 | 9-Jun-17 |
| 9769 | 2 | 3 | 4 | | IV-1 | Hbq1a | | |
| 9770 | 2 | 3 | 4 | | IV-1 | Hbq1b | | |
| 9786 | 2 | 3 | 4 | | IV-1 | Hcrtr1 | 3061 | 17-May-15 |
| 9791 | 2 | 3 | 4 | | IV-1 | Hdac11 | 79885 | 4-May-15 |
| 9796 | 2 | 3 | 4 | | IV-1 | Hdac6 | 10013 | 10-May-15 |
| 9800 | 2 | 3 | 4 | | IV-1 | Hdc | 3067 | 7-Jun-15 |
| 9801 | 2 | 3 | 4 | | IV-1 | Hddc2 | 51020 | 4-May-15 |
| 9802 | 2 | 3 | 4 | | IV-1 | Hddc3 | 374659 | 4-May-15 |
| 9823 | 2 | 3 | 4 | | IV-1 | Hectd2 | 143279 | 12-May-15 |
| 9827 | 2 | 3 | 4 | | IV-1 | Heg1 | 57493 | 12-May-15 |
| 9854 | 2 | 3 | 4 | | IV-1 | Hes7 | 84667 | 4-May-15 |
| 9857 | 2 | 3 | 4 | | IV-1 | Hexb | 3074 | 12-May-15 |
| 9863 | 2 | 3 | 4 | | IV-1 | Heyl | 26508 | 4-May-15 |
| 9873 | 2 | 3 | 4 | | IV-1 | Hhatl | 57467 | 4-May-15 |
| 9888 | 2 | 3 | 4 | | IV-1 | Hif3a | 64344 | 4-May-15 |
| 9890 | 2 | 3 | 4 | | IV-1 | Higd1b | 51751 | 4-May-15 |
| 9894 | 2 | 3 | 4 | | IV-1 | Hils1 | 373861 | 12-May-15 |
| 9897 | 2 | 3 | 4 | | IV-1 | Hint2 | 84681 | 12-May-15 |
| 9900 | 2 | 3 | 4 | | IV-1 | Hip1r | 9026 | 4-May-15 |
| 9907 | 2 | 3 | 4 | | IV-1 | Hist1h1a | 3024 | 4-May-15 |
| 9914 | 2 | 3 | 4 | | IV-1 | Hist1h2ab | 8335 | 4-May-15 |
| 9915 | 2 | 3 | 4 | | IV-1 | Hist1h2ac | 8334 | 4-May-15 |
| 9918 | 2 | 3 | 4 | | IV-1 | Hist1h2af | | |
| 9919 | 2 | 3 | 4 | | IV-1 | Hist1h2ag | 8969 | 4-May-15 |
| 9920 | 2 | 3 | 4 | | IV-1 | Hist1h2ah | 85235 | 4-May-15 |
| 9921 | 2 | 3 | 4 | | IV-1 | Hist1h2ai | 8329 | 4-May-15 |
| 9924 | 2 | 3 | 4 | | IV-1 | Hist1h2ao | | |
| 9925 | 2 | 3 | 4 | | IV-1 | Hist1h2ap | | |
| 9926 | 2 | 3 | 4 | | IV-1 | Hist1h2ba | 255626 | 4-May-15 |
| 9927 | 2 | 3 | 4 | | IV-1 | Hist1h2bb | 3018 | 4-May-15 |
| 9930 | 2 | 3 | 4 | | IV-1 | Hist1h2bf | 8343 | 4-May-15 |
| 9932 | 2 | 3 | 4 | | IV-1 | Hist1h2bh | 8345 | 4-May-15 |
| 9934 | 2 | 3 | 4 | | IV-1 | Hist1h2bk | 85236 | 4-May-15 |
| 9936 | 2 | 3 | 4 | | IV-1 | Hist1h2bm | 8342 | 4-May-15 |
| 9937 | 2 | 3 | 4 | | IV-1 | Hist1h2bn | 8341 | 4-May-15 |
| 9943 | 2 | 3 | 4 | | IV-1 | Hist1h3d | 8351 | 4-May-15 |
| 9946 | 2 | 3 | 4 | | IV-1 | Hist1h3g | 8355 | 4-May-15 |
| 9947 | 2 | 3 | 4 | | IV-1 | Hist1h3h | 8357 | 4-May-15 |
| 9952 | 2 | 3 | 4 | | IV-1 | Hist1h4d | 8360 | 1-Jun-15 |
| 9959 | 2 | 3 | 4 | | IV-1 | Hist1h4n | | |
| 9960 | 2 | 3 | 4 | | IV-1 | Hist2h2aa1 | | |
| 9963 | 2 | 3 | 4 | | IV-1 | Hist2h2ac | 8338 | 4-May-15 |
| 9965 | 2 | 3 | 4 | | IV-1 | Hist2h2be | 8349 | 4-May-15 |
| 9966 | 2 | 3 | 4 | | IV-1 | Hist2h3b | | |
| 9969 | 2 | 3 | 4 | | IV-1 | Hist2h4 | 8370 | 4-May-15 |
| 9971 | 2 | 3 | 4 | | IV-1 | Hist3h2ba | 337872 | 4-May-15 |
| 9977 | 2 | 3 | 4 | | IV-1 | Hjurp | 55355 | 17-May-15 |
| 9980 | 2 | 3 | 4 | | IV-1 | Hk2 | 3099 | 7-Jun-15 |
| 9984 | 2 | 3 | 4 | | IV-1 | Hlf | 3131 | 7-Jun-15 |
| 9986 | 2 | 3 | 4 | | IV-1 | Hlx | 3142 | 4-May-15 |
| 9987 | 2 | 3 | 4 | | IV-1 | Hmbox1 | 79618 | 4-May-15 |
| 9988 | 2 | 3 | 4 | | IV-1 | Hmbs | 3145 | 23-May-15 |
| 9993 | 2 | 3 | 4 | | IV-1 | Hmga1 | 3159 | 12-May-15 |
| 9994 | 2 | 3 | 4 | | IV-1 | Hmga1-rs1 | | |
| 10000 | 2 | 3 | 4 | | IV-1 | Hmgb3 | 3149 | 12-May-15 |
| 10001 | 2 | 3 | 4 | | IV-1 | Hmgb4 | 127540 | 4-May-15 |
| 10004 | 2 | 3 | 4 | | IV-1 | Hmgcr | 3156 | 12-May-15 |
| 10005 | 2 | 3 | 4 | | IV-1 | Hmgcs1 | 3157 | 4-May-15 |
| 10006 | 2 | 3 | 4 | | IV-1 | Hmgcs2 | 3158 | 12-May-15 |
| 10015 | 2 | 3 | 4 | | IV-1 | Hmox1 | 3162 | 17-May-15 |
| 10023 | 2 | 3 | 4 | | IV-1 | Hnf1b | 6928 | 31-May-15 |
| 10024 | 2 | 3 | 4 | | IV-1 | Hnf4a | 3172 | 23-May-15 |
| 10029 | 2 | 3 | 4 | | IV-1 | Hnrnpa1 | 3178 | 23-May-15 |
| 10048 | 2 | 3 | 4 | | IV-1 | Hoga1 | 112817 | 12-May-15 |
| 10053 | 2 | 3 | 4 | | IV-1 | Hook1 | 51361 | 4-May-15 |
| 10054 | 2 | 3 | 4 | | IV-1 | Hook2 | 29911 | 4-May-15 |
| 10056 | 2 | 3 | 4 | | IV-1 | Hopx | 84525 | 4-May-15 |
| 10066 | 2 | 3 | 4 | | IV-1 | Hoxa2 | 3199 | 12-May-15 |
| 10070 | 2 | 3 | 4 | | IV-1 | Hoxa6 | 3203 | 4-May-15 |
| 10075 | 2 | 3 | 4 | | IV-1 | Hoxb2 | 3212 | 4-May-15 |
| 10076 | 2 | 3 | 4 | | IV-1 | Hoxb3 | 3213 | 12-May-15 |
| 10077 | 2 | 3 | 4 | | IV-1 | Hoxb4 | 3214 | 4-May-15 |
| 10080 | 2 | 3 | 4 | | IV-1 | Hoxb7 | 3217 | 4-May-15 |
| 10082 | 2 | 3 | 4 | | IV-1 | Hoxb9 | 3219 | 24-May-15 |
| 10084 | 2 | 3 | 4 | | IV-1 | Hoxc11 | 3227 | 28-May-15 |
| 10099 | 2 | 3 | 4 | | IV-1 | Hoxd4 | 3233 | 12-May-15 |
| 10100 | 2 | 3 | 4 | | IV-1 | Hoxd8 | 3234 | 12-May-15 |
| 10101 | 2 | 3 | 4 | | IV-1 | Hoxd9 | 3235 | 4-May-15 |
| 10102 | 2 | 3 | 4 | | IV-1 | Hp | 3240 | 7-Jun-15 |
| 10106 | 2 | 3 | 4 | | IV-1 | Hpcal4 | 51440 | 4-May-15 |
| 10109 | 2 | 3 | 4 | | IV-1 | Hpgd | 3248 | 17-May-15 |
| 10111 | 2 | 3 | 4 | | IV-1 | Hpn | 3249 | 12-May-15 |
| 10123 | 2 | 3 | 4 | | IV-1 | Hrasls | 57110 | 4-May-15 |
| 10124 | 2 | 3 | 4 | | IV-1 | Hrasls5 | 117245 | 4-May-15 |
| 10126 | 2 | 3 | 4 | | IV-1 | Hrct1 | 646962 | 4-May-15 |
| 10131 | 2 | 3 | 4 | | IV-1 | Hrh4 | 59340 | 4-May-15 |
| 10133 | 2 | 3 | 4 | | IV-1 | Hrnr | 388697 | 17-May-15 |
| 10137 | 2 | 3 | 4 | | IV-1 | Hs3st1 | 9957 | 4-May-15 |
| 10139 | 2 | 3 | 4 | | IV-1 | Hs3st3a1 | 9955 | 4-May-15 |
| 10143 | 2 | 3 | 4 | | IV-1 | Hs3st6 | 64711 | 4-May-15 |
| 10148 | 2 | 3 | 4 | | IV-1 | Hsbp1l1 | 440498 | 4-May-15 |
| 10150 | 2 | 3 | 4 | | IV-1 | Hsd11b1 | 3290 | 12-May-15 |
| 10153 | 2 | 3 | 4 | | IV-1 | Hsd17b10 | 3028 | 12-May-15 |
| 10156 | 2 | 3 | 4 | | IV-1 | Hsd17b13 | 345275 | 4-May-15 |
| 10157 | 2 | 3 | 4 | | IV-1 | Hsd17b14 | 51171 | 4-May-15 |
| 10162 | 2 | 3 | 4 | | IV-1 | Hsd17b7 | 51478 | 7-Jun-15 |
| 10183 | 2 | 3 | 4 | | IV-1 | Hspa12a | 259217 | 12-May-15 |
| 10187 | 2 | 3 | 4 | | IV-1 | Hspa1a | 3303 | 20-May-15 |
| 10188 | 2 | 3 | 4 | | IV-1 | Hspa1b | 3304 | 20-May-15 |
| 10189 | 2 | 3 | 4 | | IV-1 | Hspa1l | 3305 | 12-May-15 |
| 10190 | 2 | 3 | 4 | | IV-1 | Hspa2 | 3306 | 4-May-15 |
| 10196 | 2 | 3 | 4 | | IV-1 | Hspb1 | 3315 | 23-May-15 |
| 10197 | 2 | 3 | 4 | | IV-1 | Hspb11 | 51668 | 4-May-15 |
| 10198 | 2 | 3 | 4 | | IV-1 | Hspb2 | 3316 | 4-May-15 |
| 10207 | 2 | 3 | 4 | | IV-1 | Hspe1 | 3336 | 4-May-15 |
| 10210 | 2 | 3 | 4 | | IV-1 | Htatip2 | 10553 | 31-May-15 |
| 10218 | 2 | 3 | 4 | | IV-1 | Htr2c | 3358 | 31-May-15 |
| 10223 | 2 | 3 | 4 | | IV-1 | Htr5b | 645694 | 4-May-15 |
| 10226 | 2 | 3 | 4 | | IV-1 | Htra1 | 5654 | 23-May-15 |
| 10228 | 2 | 3 | 4 | | IV-1 | Htra3 | 94031 | 4-May-15 |
| 10229 | 2 | 3 | 4 | | IV-1 | Htra4 | 203100 | 4-May-15 |
| 10235 | 2 | 3 | 4 | | IV-1 | Hvcn1 | 84329 | 3-May-15 |
| 10236 | 2 | 3 | 4 | | IV-1 | Hyal1 | 3373 | 7-Jun-15 |
| 10243 | 2 | 3 | 4 | | IV-1 | Hyi | 81888 | 4-May-15 |
| 10245 | 2 | 3 | 4 | | IV-1 | Hyls1 | 219844 | 4-May-15 |
| 10246 | 2 | 3 | 4 | | IV-1 | Hyou1 | 10525 | 31-May-15 |
| 10248 | 2 | 3 | 4 | | IV-1 | I730028E13Rik | | |
| 10253 | 2 | 3 | 4 | | IV-1 | Iapp | 3375 | 17-May-15 |
| 10257 | 2 | 3 | 4 | | IV-1 | Ibsp | 3381 | 12-May-15 |
| 10270 | 2 | 3 | 4 | | IV-1 | Id1 | 3397 | 31-May-15 |
| 10272 | 2 | 3 | 4 | | IV-1 | Id3 | 3399 | 21-May-15 |
| 10276 | 2 | 3 | 4 | | IV-1 | Idh2 | 3418 | 21-May-15 |
| 10280 | 2 | 3 | 4 | | IV-1 | Idi1 | 3422 | 21-May-15 |
| 10284 | 2 | 3 | 4 | | IV-1 | Ido2 | 169355 | 4-May-15 |
| 10288 | 2 | 3 | 4 | | IV-1 | Ier3 | 8870 | 21-May-15 |
| 10299 | 2 | 3 | 4 | | IV-1 | Ifi27l2a | | |
| 10300 | 2 | 3 | 4 | | IV-1 | Ifi27l2b | | |
| 10310 | 2 | 3 | 4 | | IV-1 | Ifitm1 | 8519 | 4-May-15 |
| 10314 | 2 | 3 | 4 | | IV-1 | Ifitm5 | 387733 | 4-May-15 |
| 10315 | 2 | 3 | 4 | | IV-1 | Ifitm6 | | |
| 10342 | 2 | 3 | 4 | | IV-1 | Ifnlr1 | 163702 | 4-May-15 |
| 10345 | 2 | 3 | 4 | | IV-1 | Ifrd2 | 7866 | 12-May-15 |
| 10350 | 2 | 3 | 4 | | IV-1 | Ift22 | 64792 | 12-May-15 |
| 10356 | 2 | 3 | 4 | | IV-1 | Ift74 | 80173 | 4-May-15 |
| 10365 | 2 | 3 | 4 | | IV-1 | Igf1r | 3480 | 7-Jun-15 |
| 10374 | 2 | 3 | 4 | | IV-1 | Igfbp2 | 3485 | 12-May-15 |
| 10375 | 2 | 3 | 4 | | IV-1 | Igfbp3 | 3486 | 17-May-15 |
| 10377 | 2 | 3 | 4 | | IV-1 | Igfbp5 | 3488 | 12-May-15 |
| 10378 | 2 | 3 | 4 | | IV-1 | Igfbp6 | 3489 | 4-May-15 |
| 10381 | 2 | 3 | 4 | | IV-1 | Igfl3 | 388555 | 4-May-15 |
| 10383 | 2 | 3 | 4 | | IV-1 | Igfn1 | 91156 | 4-May-15 |
| 10393 | 2 | 3 | 4 | | IV-1 | Igsf23 | 147710 | 4-May-15 |
| 10395 | 2 | 3 | 4 | | IV-1 | Igsf5 | 150084 | 21-May-15 |
| 10396 | 2 | 3 | 4 | | IV-1 | Igsf6 | 10261 | 4-May-15 |
| 10401 | 2 | 3 | 4 | | IV-1 | Ihh | 3549 | 12-May-15 |
| 10421 | 2 | 3 | 4 | | IV-1 | Il12b | 3593 | 23-May-15 |
| 10437 | 2 | 3 | 4 | | IV-1 | Il17rc | 84818 | 4-May-15 |
| 10440 | 2 | 3 | 4 | | IV-1 | Il18 | 3606 | 17-May-15 |
| 10443 | 2 | 3 | 4 | | IV-1 | Il18rap | 8807 | 4-May-15 |
| 10446 | 2 | 3 | 4 | | IV-1 | Il1b | 3553 | 24-May-15 |
| 10451 | 2 | 3 | 4 | | IV-1 | Il1f8 | 27177 | 4-May-15 |
| 10452 | 2 | 3 | 4 | | IV-1 | Il1f9 | 56300 | 4-May-15 |
| 10454 | 2 | 3 | 4 | | IV-1 | Il1r2 | 7850 | 4-May-15 |
| 10458 | 2 | 3 | 4 | | IV-1 | Il1rl1 | 9173 | 12-May-15 |
| 10460 | 2 | 3 | 4 | | IV-1 | Il1rn | 3557 | 23-May-15 |
| 10464 | 2 | 3 | 4 | | IV-1 | Il20rb | 53833 | 4-May-15 |
| 10469 | 2 | 3 | 4 | | IV-1 | Il22ra2 | 116379 | 4-May-15 |
| 10477 | 2 | 3 | 4 | | IV-1 | Il2rb | 3560 | 12-May-15 |
| 10482 | 2 | 3 | 4 | | IV-1 | Il33 | 90865 | 17-May-15 |
| 10487 | 2 | 3 | 4 | | IV-1 | Il4ra | 3566 | 17-May-15 |
| 10491 | 2 | 3 | 4 | | IV-1 | Il6ra | 3570 | 17-May-15 |
| 10494 | 2 | 3 | 4 | | IV-1 | Il7r | 3575 | 12-May-15 |
| 10505 | 2 | 3 | 4 | | IV-1 | Immp1l | 196294 | 23-May-15 |
| 10506 | 2 | 3 | 4 | | IV-1 | Immp2l | 83943 | 23-May-15 |
| 10511 | 2 | 3 | 4 | | IV-1 | Impa2 | 3613 | 12-May-15 |
| 10514 | 2 | 3 | 4 | | IV-1 | Impdh1 | 3614 | 7-Jun-15 |
| 10519 | 2 | 3 | 4 | | IV-1 | Inadl | 10207 | 4-May-15 |
| 10520 | 2 | 3 | 4 | | IV-1 | Inca1 | 388324 | 4-May-15 |
| 10521 | 2 | 3 | 4 | | IV-1 | Incenp | 3619 | 4-May-15 |
| 10529 | 2 | 3 | 4 | | IV-1 | Inhba | 3624 | 4-May-15 |
| 10530 | 2 | 3 | 4 | | IV-1 | Inhbb | 3625 | 4-May-15 |
| 10534 | 2 | 3 | 4 | | IV-1 | Inmt | 11185 | 4-May-15 |
| 10546 | 2 | 3 | 4 | | IV-1 | Inpp5d | 3635 | 12-May-15 |
| 10551 | 2 | 3 | 4 | | IV-1 | Inppl1 | 3636 | 17-May-15 |
| 10555 | 2 | 3 | 4 | | IV-1 | Insig1 | 3638 | 4-May-15 |
| 10557 | 2 | 3 | 4 | | IV-1 | Insl3 | 3640 | 4-May-15 |
| 10559 | 2 | 3 | 4 | | IV-1 | Insl6 | 11172 | 4-May-15 |
| 10571 | 2 | 3 | 4 | | IV-1 | Ints6 | 26512 | 12-May-15 |
| 10578 | 2 | 3 | 4 | | IV-1 | Ip6k2 | 51447 | 4-May-15 |
| 10581 | 2 | 3 | 4 | | IV-1 | Ipmk | 253430 | 4-May-15 |
| 10602 | 2 | 3 | 4 | | IV-1 | Iqcg | 84223 | 4-May-15 |
| 10605 | 2 | 3 | 4 | | IV-1 | Iqck | 124152 | 4-May-15 |
| 10625 | 2 | 3 | 4 | | IV-1 | Irf4 | 3662 | 24-May-15 |
| 10627 | 2 | 3 | 4 | | IV-1 | Irf6 | 3664 | 23-May-15 |
| 10632 | 2 | 3 | 4 | | IV-1 | Irgc1 | 56269 | 4-May-15 |
| 10637 | 2 | 3 | 4 | | IV-1 | Irs2 | 8660 | 12-May-15 |
| 10640 | 2 | 3 | 4 | | IV-1 | Irx1 | 79192 | 7-Jun-15 |
| 10641 | 2 | 3 | 4 | | IV-1 | Irx2 | 153572 | 4-May-15 |
| 10648 | 2 | 3 | 4 | | IV-1 | Iscu | 23479 | 23-May-15 |
| 10649 | 2 | 3 | 4 | | IV-1 | Isg15 | 9636 | 12-May-15 |
| 10650 | 2 | 3 | 4 | | IV-1 | Isg20 | 3669 | 12-May-15 |

Fig.22 - 30

| | | | | | | | | |
|---|---|---|---|---|---|---|---|---|
| 10652 | 2 | 3 | 4 | | IV-1 | Isl1 | 3670 | 4-May-15 |
| 10653 | 2 | 3 | 4 | | IV-1 | Isl2 | 64843 | 4-May-15 |
| 10654 | 2 | 3 | 4 | | IV-1 | Islr | 3671 | 12-May-15 |
| 10656 | 2 | 3 | 4 | | IV-1 | Ism1 | 140862 | 4-May-15 |
| 10657 | 2 | 3 | 4 | | IV-1 | Ism2 | 145501 | 4-May-15 |
| 10659 | 2 | 3 | 4 | | IV-1 | Isoc2a | | |
| 10664 | 2 | 3 | 4 | | IV-1 | Isy1 | 57461 | 12-May-15 |
| 10669 | 2 | 3 | 4 | | IV-1 | Itfg3 | 83986 | 4-May-15 |
| 10673 | 2 | 3 | 4 | | IV-1 | Itga2 | 3673 | 12-May-15 |
| 10675 | 2 | 3 | 4 | | IV-1 | Itga3 | 3675 | 30-May-15 |
| 10682 | 2 | 3 | 4 | | IV-1 | Itgad | 3681 | 12-May-15 |
| 10685 | 2 | 3 | 4 | | IV-1 | Itgam | 3684 | 12-May-15 |
| 10691 | 2 | 3 | 4 | | IV-1 | Itgb2 | 3689 | 12-May-15 |
| 10702 | 2 | 3 | 4 | | IV-1 | Itih2 | 3698 | 4-May-15 |
| 10713 | 2 | 3 | 4 | | IV-1 | Itpka | 3706 | 4-May-15 |
| 10726 | 2 | 3 | 4 | | IV-1 | Ivns1abp | 10625 | 4-May-15 |
| 10728 | 2 | 3 | 4 | | IV-1 | Iyd | 389434 | 4-May-15 |
| 10735 | 2 | 3 | 4 | | IV-1 | Jade3 | 9767 | 4-May-15 |
| 10737 | 2 | 3 | 4 | | IV-1 | Jag2 | 3714 | 4-May-15 |
| 10742 | 2 | 3 | 4 | | IV-1 | Jakmip1 | 152789 | 12-May-15 |
| 10760 | 2 | 3 | 4 | | IV-1 | Jph1 | 56704 | 17-May-15 |
| 10780 | 2 | 3 | 4 | | IV-1 | Kansl1l | 151050 | 12-May-15 |
| 10783 | 2 | 3 | 4 | | IV-1 | Kap | | |
| 10797 | 2 | 3 | 4 | | IV-1 | Kazald1 | 81621 | 4-May-15 |
| 10801 | 2 | 3 | 4 | | IV-1 | Kbtbd13 | 390594 | 23-May-15 |
| 10813 | 2 | 3 | 4 | | IV-1 | Kcna5 | 3741 | 17-May-15 |
| 10821 | 2 | 3 | 4 | | IV-1 | Kcnc1 | 3746 | 4-May-15 |
| 10823 | 2 | 3 | 4 | | IV-1 | Kcnc3 | 3748 | 23-May-15 |
| 10825 | 2 | 3 | 4 | | IV-1 | Kcnd1 | 3750 | 12-May-15 |
| 10829 | 2 | 3 | 4 | | IV-1 | Kcne1 | 3753 | 23-May-15 |
| 10834 | 2 | 3 | 4 | | IV-1 | Kcnf1 | 3754 | 4-May-15 |
| 10838 | 2 | 3 | 4 | | IV-1 | Kcng4 | 93107 | 4-May-15 |
| 10839 | 2 | 3 | 4 | | IV-1 | Kcnh1 | 3756 | 17-May-15 |
| 10848 | 2 | 3 | 4 | | IV-1 | Kcnip2 | 30819 | 12-May-15 |
| 10849 | 2 | 3 | 4 | | IV-1 | Kcnip3 | 30818 | 12-May-15 |
| 10855 | 2 | 3 | 4 | | IV-1 | Kcnj13 | 3769 | 24-May-15 |
| 10858 | 2 | 3 | 4 | | IV-1 | Kcnj16 | 3773 | 12-May-15 |
| 10859 | 2 | 3 | 4 | | IV-1 | Kcnj2 | 3759 | 23-May-15 |
| 10864 | 2 | 3 | 4 | | IV-1 | Kcnj8 | 3764 | 4-May-15 |
| 10874 | 2 | 3 | 4 | | IV-1 | Kcnk3 | 3777 | 4-May-15 |
| 10876 | 2 | 3 | 4 | | IV-1 | Kcnk5 | 8645 | 4-May-15 |
| 10877 | 2 | 3 | 4 | | IV-1 | Kcnk6 | 9424 | 4-May-15 |
| 10884 | 2 | 3 | 4 | | IV-1 | Kcnmb4 | 27345 | 4-May-15 |
| 10888 | 2 | 3 | 4 | | IV-1 | Kcnn3 | 3782 | 12-May-15 |
| 10890 | 2 | 3 | 4 | | IV-1 | Kcnq1 | 3784 | 22-May-15 |
| 10899 | 2 | 3 | 4 | | IV-1 | Kcns3 | 3790 | 12-May-15 |
| 10900 | 2 | 3 | 4 | | IV-1 | Kcnt1 | 57582 | 7-Jun-15 |
| 10912 | 2 | 3 | 4 | | IV-1 | Kctd14 | 65987 | 4-May-15 |
| 10913 | 2 | 3 | 4 | | IV-1 | Kctd15 | 79047 | 4-May-15 |
| 10933 | 2 | 3 | 4 | | IV-1 | Kdf1 | 126695 | 4-May-15 |
| 10958 | 2 | 3 | 4 | | IV-1 | Khdc1a | | |
| 10960 | 2 | 3 | 4 | | IV-1 | Khdc1c | | |
| 10964 | 2 | 3 | 4 | | IV-1 | Khdrbs3 | 10656 | 12-May-15 |
| 10965 | 2 | 3 | 4 | | IV-1 | Khk | 3795 | 3-May-15 |
| 10986 | 2 | 3 | 4 | | IV-1 | Kif21b | 23046 | 4-May-15 |
| 10990 | 2 | 3 | 4 | | IV-1 | Kif26a | 26153 | 4-May-15 |
| 11003 | 2 | 3 | 4 | | IV-1 | Kif5c | 3800 | 4-May-15 |
| 11017 | 2 | 3 | 4 | | IV-1 | Kirrel3 | 84623 | 4-May-15 |
| 11024 | 2 | 3 | 4 | | IV-1 | Kl | 9365 | 24-May-15 |
| 11025 | 2 | 3 | 4 | | IV-1 | Klb | 152831 | 4-May-15 |
| 11028 | 2 | 3 | 4 | | IV-1 | Klc3 | 147700 | 4-May-15 |
| 11032 | 2 | 3 | 4 | | IV-1 | Klf11 | 8462 | 4-May-15 |
| 11036 | 2 | 3 | 4 | | IV-1 | Klf15 | 28999 | 4-May-15 |
| 11039 | 2 | 3 | 4 | | IV-1 | Klf2 | 10365 | 12-May-15 |
| 11042 | 2 | 3 | 4 | | IV-1 | Klf5 | 688 | 17-May-15 |
| 11044 | 2 | 3 | 4 | | IV-1 | Klf7 | 8609 | 4-May-15 |
| 11046 | 2 | 3 | 4 | | IV-1 | Klf9 | 687 | 31-May-15 |
| 11052 | 2 | 3 | 4 | | IV-1 | Klhdc7a | 127707 | 12-May-15 |
| 11058 | 2 | 3 | 4 | | IV-1 | Klhl10 | 317719 | 7-Jun-15 |
| 11059 | 2 | 3 | 4 | | IV-1 | Klhl11 | 55175 | 2-Jun-15 |
| 11062 | 2 | 3 | 4 | | IV-1 | Klhl14 | 57565 | 4-May-15 |
| 11063 | 2 | 3 | 4 | | IV-1 | Klhl15 | 80311 | 12-May-15 |
| 11070 | 2 | 3 | 4 | | IV-1 | Klhl23 | 151230 | 4-May-15 |
| 11075 | 2 | 3 | 4 | | IV-1 | Klhl29 | 114818 | 4-May-15 |
| 11080 | 2 | 3 | 4 | | IV-1 | Klhl33 | 123103 | 4-May-15 |
| 11084 | 2 | 3 | 4 | | IV-1 | Klhl38 | 340359 | 4-May-15 |
| 11094 | 2 | 3 | 4 | | IV-1 | Klk1 | 3816 | 12-May-15 |
| 11109 | 2 | 3 | 4 | | IV-1 | Klk1b3 | | |
| 11110 | 2 | 3 | 4 | | IV-1 | Klk1b4 | | |
| 11111 | 2 | 3 | 4 | | IV-1 | Klk1b5 | | |
| 11114 | 2 | 3 | 4 | | IV-1 | Klk1b9 | | |
| 11117 | 2 | 3 | 4 | | IV-1 | Klk6 | 5653 | 7-Jun-15 |
| 11120 | 2 | 3 | 4 | | IV-1 | Klk9 | 284366 | 4-May-15 |
| 11134 | 2 | 3 | 4 | | IV-1 | Klra23 | | |
| 11140 | 2 | 3 | 4 | | IV-1 | Klra7 | | |
| 11142 | 2 | 3 | 4 | | IV-1 | Klra9 | | |
| 11145 | 2 | 3 | 4 | | IV-1 | Klrb1b | | |
| 11149 | 2 | 3 | 4 | | IV-1 | Klrc1 | 3821 | 12-May-15 |
| 11155 | 2 | 3 | 4 | | IV-1 | Klrg2 | 346689 | 4-May-15 |
| 11168 | 2 | 3 | 4 | | IV-1 | Kng2 | | |
| 11179 | 2 | 3 | 4 | | IV-1 | Kprp | 448834 | 4-May-15 |
| 11186 | 2 | 3 | 4 | | IV-1 | Kri1 | 65095 | 4-May-15 |
| 11189 | 2 | 3 | 4 | | IV-1 | Krt1 | 3848 | 24-May-15 |
| 11190 | 2 | 3 | 4 | | IV-1 | Krt10 | 3858 | 4-May-15 |
| 11194 | 2 | 3 | 4 | | IV-1 | Krt15 | 3866 | 4-May-15 |
| 11198 | 2 | 3 | 4 | | IV-1 | Krt19 | 3880 | 12-May-15 |
| 11200 | 2 | 3 | 4 | | IV-1 | Krt20 | 54474 | 4-May-15 |

| | | | | | | | | |
|---|---|---|---|---|---|---|---|---|
| 11212 | 2 | 3 | 4 | | IV-1 | Krt34 | 3885 | 12-May-15 |
| 11215 | 2 | 3 | 4 | | IV-1 | Krt39 | 390792 | 4-May-15 |
| 11218 | 2 | 3 | 4 | | IV-1 | Krt42 | | |
| 11221 | 2 | 3 | 4 | | IV-1 | Krt6b | 3854 | 4-May-15 |
| 11222 | 2 | 3 | 4 | | IV-1 | Krt7 | 3855 | 12-May-15 |
| 11224 | 2 | 3 | 4 | | IV-1 | Krt72 | 140807 | 4-May-15 |
| 11229 | 2 | 3 | 4 | | IV-1 | Krt77 | 374454 | 4-May-15 |
| 11231 | 2 | 3 | 4 | | IV-1 | Krt79 | 338785 | 4-May-15 |
| 11232 | 2 | 3 | 4 | | IV-1 | Krt8 | 3856 | 4-May-15 |
| 11239 | 2 | 3 | 4 | | IV-1 | Krt86 | 3892 | 12-May-15 |
| 11243 | 2 | 3 | 4 | | IV-1 | Krtap11-1 | 337880 | 4-May-15 |
| 11246 | 2 | 3 | 4 | | IV-1 | Krtap1-3 | 81850 | 4-May-15 |
| 11247 | 2 | 3 | 4 | | IV-1 | Krtap13-1 | 140258 | 4-May-15 |
| 11248 | 2 | 3 | 4 | | IV-1 | Krtap14 | 728255 | 4-May-15 |
| 11249 | 2 | 3 | 4 | | IV-1 | Krtap1-4 | 728255 | 4-May-15 |
| 11250 | 2 | 3 | 4 | | IV-1 | Krtap15 | 83895 | 4-May-15 |
| 11252 | 2 | 3 | 4 | | IV-1 | Krtap16-1 | 100505753 | 4-May-15 |
| 11256 | 2 | 3 | 4 | | IV-1 | Krtap19-3 | 337970 | 4-May-15 |
| 11259 | 2 | 3 | 4 | | IV-1 | Krtap19-9b | | |
| 11262 | 2 | 3 | 4 | | IV-1 | Krtap22-2 | 100288287 | 4-May-15 |
| 11263 | 2 | 3 | 4 | | IV-1 | Krtap2-4 | 85294 | 4-May-15 |
| 11265 | 2 | 3 | 4 | | IV-1 | Krtap26-1 | 388818 | 4-May-15 |
| 11266 | 2 | 3 | 4 | | IV-1 | Krtap27-1 | 643812 | 4-May-15 |
| 11267 | 2 | 3 | 4 | | IV-1 | Krtap3-1 | 83896 | 4-May-15 |
| 11270 | 2 | 3 | 4 | | IV-1 | Krtap3-2 | 83897 | 4-May-15 |
| 11271 | 2 | 3 | 4 | | IV-1 | Krtap3-3 | 85293 | 4-May-15 |
| 11272 | 2 | 3 | 4 | | IV-1 | Krtap4-1 | 85285 | 4-May-15 |
| 11273 | 2 | 3 | 4 | | IV-1 | Krtap4-13 | 84616 | 4-May-15 |
| 11274 | 2 | 3 | 4 | | IV-1 | Krtap4-16 | | |
| 11275 | 2 | 3 | 4 | | IV-1 | Krtap4-2 | 85291 | 4-May-15 |
| 11276 | 2 | 3 | 4 | | IV-1 | Krtap4-6 | 81871 | 4-May-15 |
| 11277 | 2 | 3 | 4 | | IV-1 | Krtap4-7 | 100132476 | 4-May-15 |
| 11278 | 2 | 3 | 4 | | IV-1 | Krtap4-8 | 728224 | 4-May-15 |
| 11279 | 2 | 3 | 4 | | IV-1 | Krtap4-9 | 100132386 | 4-May-15 |
| 11280 | 2 | 3 | 4 | | IV-1 | Krtap5-1 | 387264 | 4-May-15 |
| 11283 | 2 | 3 | 4 | | IV-1 | Krtap5-4 | 387267 | 4-Apr-15 |
| 11288 | 2 | 3 | 4 | | IV-1 | Krtap7-1 | 337878 | 4-May-15 |
| 11289 | 2 | 3 | 4 | | IV-1 | Krtap8-1 | 337879 | 4-May-15 |
| 11290 | 2 | 3 | 4 | | IV-1 | Krtap9-1 | 728318 | 4-May-15 |
| 11291 | 2 | 3 | 4 | | IV-1 | Krtap9-3 | 83900 | 12-May-15 |
| 11296 | 2 | 3 | 4 | | IV-1 | Ksr1 | 8844 | 4-May-15 |
| 11306 | 2 | 3 | 4 | | IV-1 | L3hypdh | 112849 | 12-May-15 |
| 11318 | 2 | 3 | 4 | | IV-1 | Lag3 | 3902 | 4-May-15 |
| 11325 | 2 | 3 | 4 | | IV-1 | Lama4 | 3910 | 4-May-15 |
| 11327 | 2 | 3 | 4 | | IV-1 | Lamb1 | 3912 | 12-May-15 |
| 11329 | 2 | 3 | 4 | | IV-1 | Lamb3 | 3914 | 23-May-15 |
| 11331 | 2 | 3 | 4 | | IV-1 | Lamc2 | 3918 | 23-May-15 |
| 11346 | 2 | 3 | 4 | | IV-1 | Lap3 | 51056 | 7-Jun-15 |
| 11349 | 2 | 3 | 4 | | IV-1 | Laptm5 | 7805 | 4-May-15 |
| 11355 | 2 | 3 | 4 | | IV-1 | Larp6 | 55323 | 4-May-15 |
| 11358 | 2 | 3 | 4 | | IV-1 | Lars2 | 23395 | 4-May-15 |
| 11362 | 2 | 3 | 4 | | IV-1 | Lat2 | 7462 | 7-Jun-15 |
| 11365 | 2 | 3 | 4 | | IV-1 | Lax1 | 54900 | 4-May-15 |
| 11368 | 2 | 3 | 4 | | IV-1 | Lbp | 3929 | 7-Jun-15 |
| 11369 | 2 | 3 | 4 | | IV-1 | Lbr | 3930 | 12-May-15 |
| 11374 | 2 | 3 | 4 | | IV-1 | Lcat | 3931 | 4-May-15 |
| 11375 | 2 | 3 | 4 | | IV-1 | Lce1a1 | | |
| 11376 | 2 | 3 | 4 | | IV-1 | Lce1a2 | | |
| 11377 | 2 | 3 | 4 | | IV-1 | Lce1b | 353132 | 4-May-15 |
| 11378 | 2 | 3 | 4 | | IV-1 | Lce1c | 353133 | 4-May-15 |
| 11379 | 2 | 3 | 4 | | IV-1 | Lce1d | 353134 | 4-May-15 |
| 11380 | 2 | 3 | 4 | | IV-1 | Lce1e | 353135 | 4-May-15 |
| 11381 | 2 | 3 | 4 | | IV-1 | Lce1f | 353137 | 4-May-15 |
| 11382 | 2 | 3 | 4 | | IV-1 | Lce1g | | |
| 11383 | 2 | 3 | 4 | | IV-1 | Lce1h | | |
| 11384 | 2 | 3 | 4 | | IV-1 | Lce1i | | |
| 11385 | 2 | 3 | 4 | | IV-1 | Lce1j | | |
| 11386 | 2 | 3 | 4 | | IV-1 | Lce1k | | |
| 11387 | 2 | 3 | 4 | | IV-1 | Lce1l | | |
| 11392 | 2 | 3 | 4 | | IV-1 | Lce3d | 84648 | 4-May-15 |
| 11395 | 2 | 3 | 4 | | IV-1 | Lce6a | 448835 | 4-May-15 |
| 11410 | 2 | 3 | 4 | | IV-1 | Lcor | 84458 | 4-May-15 |
| 11411 | 2 | 3 | 4 | | IV-1 | Lcorl | 254251 | 4-May-15 |
| 11421 | 2 | 3 | 4 | | IV-1 | Ldhb | 3945 | 12-May-15 |
| 11423 | 2 | 3 | 4 | | IV-1 | Ldhd | 197257 | 4-May-15 |
| 11424 | 2 | 3 | 4 | | IV-1 | Ldlr | 3949 | 17-May-15 |
| 11436 | 2 | 3 | 4 | | IV-1 | Lefty1 | 10637 | 4-May-15 |
| 11448 | 2 | 3 | 4 | | IV-1 | Lep | 3952 | 24-May-15 |
| 11449 | 2 | 3 | 4 | | IV-1 | Lepr | 3953 | 7-Jun-15 |
| 11459 | 2 | 3 | 4 | | IV-1 | Lfng | 3955 | 23-May-15 |
| 11461 | 2 | 3 | 4 | | IV-1 | Lgals12 | 85329 | 4-May-15 |
| 11462 | 2 | 3 | 4 | | IV-1 | Lgals2 | 3957 | 7-Jun-15 |
| 11463 | 2 | 3 | 4 | | IV-1 | Lgals3 | 3958 | 24-May-15 |
| 11465 | 2 | 3 | 4 | | IV-1 | Lgals4 | 3960 | 4-May-15 |
| 11466 | 2 | 3 | 4 | | IV-1 | Lgals6 | | |
| 11473 | 2 | 3 | 4 | | IV-1 | Lgi3 | 203190 | 4-May-15 |
| 11475 | 2 | 3 | 4 | | IV-1 | Lgmn | 5641 | 4-May-15 |
| 11484 | 2 | 3 | 4 | | IV-1 | Lhfpl2 | 10184 | 4-May-15 |
| 11490 | 2 | 3 | 4 | | IV-1 | Lhx1os | | |
| 11495 | 2 | 3 | 4 | | IV-1 | Lhx6 | 26468 | 4-May-15 |
| 11499 | 2 | 3 | 4 | | IV-1 | Lif | 3976 | 28-May-15 |
| 11506 | 2 | 3 | 4 | | IV-1 | Lilrb4 | 11006 | 12-May-15 |
| 11516 | 2 | 3 | 4 | | IV-1 | Lims2 | 55679 | 17-May-15 |
| 11523 | 2 | 3 | 4 | | IV-1 | Lin7b | 64130 | 12-May-15 |
| 11534 | 2 | 3 | 4 | | IV-1 | Lipe | 3991 | 12-May-15 |

Fig.22 - 31

| | | | | | | | | |
|---|---|---|---|---|---|---|---|---|
| 11550 | 2 | 3 | 4 | | IV-1 | Llgl2 | 3993 | 31-May-15 |
| 11559 | 2 | 3 | 4 | | IV-1 | Lmbrd2 | 92255 | 4-May-15 |
| 11560 | 2 | 3 | 4 | | IV-1 | Lmcd1 | 29995 | 4-May-15 |
| 11572 | 2 | 3 | 4 | | IV-1 | Lmod1 | 25802 | 12-May-15 |
| 11580 | 2 | 3 | 4 | | IV-1 | Lnpep | 4012 | 12-May-15 |
| 11581 | 2 | 3 | 4 | | IV-1 | Lnx1 | 84708 | 26-May-15 |
| 11589 | 2 | 3 | 4 | | IV-1 | LOC100503496 | | |
| 11590 | 2 | 3 | 4 | | IV-1 | LOC100503676 | | |
| 11591 | 2 | 3 | 4 | | IV-1 | LOC100504039 | | |
| 11593 | 2 | 3 | 4 | | IV-1 | LOC100504703 | | |
| 11605 | 2 | 3 | 4 | | IV-1 | LOC101243624 | | |
| 11619 | 2 | 3 | 4 | | IV-1 | LOC106740 | | |
| 11628 | 2 | 3 | 4 | | IV-1 | Lonrf3 | 79836 | 4-May-15 |
| 11630 | 2 | 3 | 4 | | IV-1 | Lox | 4015 | 12-May-15 |
| 11633 | 2 | 3 | 4 | | IV-1 | Loxl2 | 4017 | 12-May-15 |
| 11638 | 2 | 3 | 4 | | IV-1 | Lpar3 | 23566 | 4-May-15 |
| 11646 | 2 | 3 | 4 | | IV-1 | Lpcat4 | 254531 | 7-Jun-15 |
| 11649 | 2 | 3 | 4 | | IV-1 | Lphn2 | 23266 | 12-May-15 |
| 11651 | 2 | 3 | 4 | | IV-1 | Lpin1 | 23175 | 4-May-15 |
| 11653 | 2 | 3 | 4 | | IV-1 | Lpin3 | 64900 | 4-May-15 |
| 11654 | 2 | 3 | 4 | | IV-1 | Lpl | 4023 | 7-Jun-15 |
| 11655 | 2 | 3 | 4 | | IV-1 | Lpo | 4025 | 4-May-15 |
| 11663 | 2 | 3 | 4 | | IV-1 | Lrch4 | 4034 | 4-May-15 |
| 11670 | 2 | 3 | 4 | | IV-1 | Lrg1 | 116844 | 4-May-15 |
| 11675 | 2 | 3 | 4 | | IV-1 | Lrig3 | 121227 | 4-May-15 |
| 11677 | 2 | 3 | 4 | | IV-1 | Lrit2 | 340745 | 4-May-15 |
| 11680 | 2 | 3 | 4 | | IV-1 | Lrp1 | 4035 | 7-Jun-15 |
| 11688 | 2 | 3 | 4 | | IV-1 | Lrp4 | 4038 | 7-Jun-15 |
| 11699 | 2 | 3 | 4 | | IV-1 | Lrrc14b | 389257 | 4-May-15 |
| 11702 | 2 | 3 | 4 | | IV-1 | Lrrc16b | 90668 | 12-May-15 |
| 11704 | 2 | 3 | 4 | | IV-1 | Lrrc18 | 474354 | 12-May-15 |
| 11706 | 2 | 3 | 4 | | IV-1 | Lrrc2 | 79442 | 4-May-15 |
| 11720 | 2 | 3 | 4 | | IV-1 | Lrrc38 | 126755 | 4-May-15 |
| 11721 | 2 | 3 | 4 | | IV-1 | Lrrc39 | 127495 | 4-May-15 |
| 11728 | 2 | 3 | 4 | | IV-1 | Lrrc45 | 201255 | 4-May-15 |
| 11729 | 2 | 3 | 4 | | IV-1 | Lrrc46 | 90506 | 4-May-15 |
| 11735 | 2 | 3 | 4 | | IV-1 | Lrrc51 | 220074 | 23-May-15 |
| 11736 | 2 | 3 | 4 | | IV-1 | Lrrc52 | 440699 | 4-May-15 |
| 11753 | 2 | 3 | 4 | | IV-1 | Lrrc75b | 388886 | 4-May-15 |
| 11755 | 2 | 3 | 4 | | IV-1 | Lrrc8b | 23507 | 4-May-15 |
| 11769 | 2 | 3 | 4 | | IV-1 | Lrrn1 | 57633 | 4-May-15 |
| 11781 | 2 | 3 | 4 | | IV-1 | Lrwd1 | 222229 | 4-May-15 |
| 11793 | 2 | 3 | 4 | | IV-1 | Lsm5 | 23658 | 4-May-15 |
| 11795 | 2 | 3 | 4 | | IV-1 | Lsm7 | 51690 | 4-May-15 |
| 11797 | 2 | 3 | 4 | | IV-1 | Lsmem1 | 286006 | 4-May-15 |
| 11799 | 2 | 3 | 4 | | IV-1 | Lsr | 51599 | 4-May-15 |
| 11800 | 2 | 3 | 4 | | IV-1 | Lss | 4047 | 7-Jun-15 |
| 11801 | 2 | 3 | 4 | | IV-1 | Lst1 | | |
| 11804 | 2 | 3 | 4 | | IV-1 | Ltb | 4050 | 12-May-15 |
| 11805 | 2 | 3 | 4 | | IV-1 | Ltb4r1 | 1241 | 17-May-15 |
| 11809 | 2 | 3 | 4 | | IV-1 | Ltbp3 | 4054 | 7-Jun-15 |
| 11810 | 2 | 3 | 4 | | IV-1 | Ltbp4 | 8425 | 12-May-15 |
| 11812 | 2 | 3 | 4 | | IV-1 | Ltc4s | 4056 | 4-May-15 |
| 11821 | 2 | 3 | 4 | | IV-1 | Lurap1 | 541468 | 4-May-15 |
| 11826 | 2 | 3 | 4 | | IV-1 | Lxn | 56925 | 4-May-15 |
| 11830 | 2 | 3 | 4 | | IV-1 | Ly6d | 8581 | 7-Jun-15 |
| 11832 | 2 | 3 | 4 | | IV-1 | Ly6f | | |
| 11835 | 2 | 3 | 4 | | IV-1 | Ly6g6c | 80740 | 4-May-15 |
| 11836 | 2 | 3 | 4 | | IV-1 | Ly6g6d | 58530 | 4-May-15 |
| 11841 | 2 | 3 | 4 | | IV-1 | Ly6k | 54742 | 4-May-15 |
| 11843 | 2 | 3 | 4 | | IV-1 | Ly86 | 9450 | 4-May-15 |
| 11844 | 2 | 3 | 4 | | IV-1 | Ly9 | 4063 | 4-May-15 |
| 11845 | 2 | 3 | 4 | | IV-1 | Ly96 | 23643 | 31-May-15 |
| 11847 | 2 | 3 | 4 | | IV-1 | Lyg1 | 129530 | 4-May-15 |
| 11852 | 2 | 3 | 4 | | IV-1 | Lypd1 | 116372 | 4-May-15 |
| 11854 | 2 | 3 | 4 | | IV-1 | Lypd3 | 27076 | 4-May-15 |
| 11857 | 2 | 3 | 4 | | IV-1 | Lypd6 | 130574 | 4-May-15 |
| 11874 | 2 | 3 | 4 | | IV-1 | Lyst | 1130 | 23-May-15 |
| 11875 | 2 | 3 | 4 | | IV-1 | Lyve1 | 10894 | 4-May-15 |
| 11896 | 2 | 3 | 4 | | IV-1 | Macrod1 | 28992 | 12-May-15 |
| 11901 | 2 | 3 | 4 | | IV-1 | Mad2l2 | 10459 | 4-May-15 |
| 11902 | 2 | 3 | 4 | | IV-1 | Madcam1 | 8174 | 4-May-15 |
| 11906 | 2 | 3 | 4 | | IV-1 | Maf | 4094 | 12-May-15 |
| 11909 | 2 | 3 | 4 | | IV-1 | Mafb | 9935 | 2-Jun-15 |
| 11910 | 2 | 3 | 4 | | IV-1 | Maff | 23764 | 4-May-15 |
| 11926 | 2 | 3 | 4 | | IV-1 | Mageb2 | 4113 | 4-May-15 |
| 11939 | 2 | 3 | 4 | | IV-1 | Magix | 79917 | 4-May-15 |
| 11946 | 2 | 3 | 4 | | IV-1 | Mal2 | 114569 | 4-May-15 |
| 11955 | 2 | 3 | 4 | | IV-1 | Maml3 | 55534 | 4-May-15 |
| 11958 | 2 | 3 | 4 | | IV-1 | Man1a | | |
| 11967 | 2 | 3 | 4 | | IV-1 | Man2c1os | | |
| 11972 | 2 | 3 | 4 | | IV-1 | Manf | 7873 | 21-May-15 |
| 11976 | 2 | 3 | 4 | | IV-1 | Maoa | 4128 | 31-May-15 |
| 11977 | 2 | 3 | 4 | | IV-1 | Maob | 4129 | 17-May-15 |
| 11997 | 2 | 3 | 4 | | IV-1 | Map3k13 | 9175 | 12-May-15 |
| 11998 | 2 | 3 | 4 | | IV-1 | Map3k14 | 9020 | 14-May-15 |
| 12005 | 2 | 3 | 4 | | IV-1 | Map3k6 | 9064 | 12-May-15 |
| 12017 | 2 | 3 | 4 | | IV-1 | Map6d1 | 79929 | 4-May-15 |
| 12018 | 2 | 3 | 4 | | IV-1 | Map7 | 9053 | 4-May-15 |
| 12020 | 2 | 3 | 4 | | IV-1 | Map7d2 | 256714 | 4-May-15 |
| 12025 | 2 | 3 | 4 | | IV-1 | Mapk12 | 6300 | 12-May-15 |
| 12026 | 2 | 3 | 4 | | IV-1 | Mapk13 | 5603 | 4-May-15 |
| 12032 | 2 | 3 | 4 | | IV-1 | Mapk4 | 5596 | 12-May-15 |
| 12044 | 2 | 3 | 4 | | IV-1 | Mapkbp1 | 23005 | 12-May-15 |
| 12048 | 2 | 3 | 4 | | IV-1 | Mapt | 4137 | 23-May-15 |
| 12055 | 2 | 3 | 4 | | IV-1 | March3 | 115123 | 4-May-15 |
| 12061 | 2 | 3 | 4 | | IV-1 | March9 | 92979 | 4-May-15 |
| 12064 | 2 | 3 | 4 | | IV-1 | Marcksl1-ps4 | | |
| 12065 | 2 | 3 | 4 | | IV-1 | Marco | 8685 | 17-May-15 |
| 12067 | 2 | 3 | 4 | | IV-1 | Mark1 | 4139 | 4-May-15 |
| 12074 | 2 | 3 | 4 | | IV-1 | Marveld2 | 153562 | 23-May-15 |
| 12075 | 2 | 3 | 4 | | IV-1 | Marveld3 | 91862 | 4-May-15 |
| 12077 | 2 | 3 | 4 | | IV-1 | Masp1 | 5648 | 17-May-15 |
| 12087 | 2 | 3 | 4 | | IV-1 | Matk | 4145 | 12-May-15 |
| 12110 | 2 | 3 | 4 | | IV-1 | Mbl2 | 4153 | 7-Jun-15 |
| 12117 | 2 | 3 | 4 | | IV-1 | Mboat2 | 129642 | 4-May-15 |
| 12125 | 2 | 3 | 4 | | IV-1 | Mc2r | 4158 | 12-May-15 |
| 12128 | 2 | 3 | 4 | | IV-1 | Mc5r | 4161 | 4-May-15 |
| 12156 | 2 | 3 | 4 | | IV-1 | Mcoln2 | 255231 | 12-May-15 |
| 12157 | 2 | 3 | 4 | | IV-1 | Mcoln3 | 55283 | 12-May-15 |
| 12161 | 2 | 3 | 4 | | IV-1 | Mcpt4 | | |
| 12169 | 2 | 3 | 4 | | IV-1 | Mcts2 | 100101490 | 4-May-15 |
| 12175 | 2 | 3 | 4 | | IV-1 | Mdga1 | 266727 | 4-May-15 |
| 12183 | 2 | 3 | 4 | | IV-1 | Mdm4 | 4194 | 4-May-15 |
| 12186 | 2 | 3 | 4 | | IV-1 | Me1 | 4199 | 7-Jun-15 |
| 12224 | 2 | 3 | 4 | | IV-1 | Med9os | | |
| 12228 | 2 | 3 | 4 | | IV-1 | Mef2c | 4208 | 12-May-15 |
| 12234 | 2 | 3 | 4 | | IV-1 | Megf6 | 1953 | 12-May-15 |
| 12235 | 2 | 3 | 4 | | IV-1 | Megf8 | 1954 | 4-May-15 |
| 12236 | 2 | 3 | 4 | | IV-1 | Megf9 | 1955 | 4-May-15 |
| 12242 | 2 | 3 | 4 | | IV-1 | Meis2 | 4212 | 4-May-15 |
| 12263 | 2 | 3 | 4 | | IV-1 | Metrn | 79006 | 4-May-15 |
| 12264 | 2 | 3 | 4 | | IV-1 | Metrnl | 284207 | 4-May-15 |
| 12275 | 2 | 3 | 4 | | IV-1 | Mettl20 | 254013 | 23-May-15 |
| 12279 | 2 | 3 | 4 | | IV-1 | Mettl22 | 79091 | 4-May-15 |
| 12300 | 2 | 3 | 4 | | IV-1 | Mfap2 | 4237 | 4-May-15 |
| 12302 | 2 | 3 | 4 | | IV-1 | Mfap3l | 9848 | 12-May-15 |
| 12303 | 2 | 3 | 4 | | IV-1 | Mfap4 | 4239 | 4-May-15 |
| 12304 | 2 | 3 | 4 | | IV-1 | Mfap5 | 8076 | 21-May-15 |
| 12306 | 2 | 3 | 4 | | IV-1 | Mfge8 | 4240 | 4-May-15 |
| 12323 | 2 | 3 | 4 | | IV-1 | Mfsd6l | 162387 | 4-May-15 |
| 12326 | 2 | 3 | 4 | | IV-1 | Mfsd7c | 55640 | 4-May-15 |
| 12331 | 2 | 3 | 4 | | IV-1 | Mgarp | 84709 | 4-May-15 |
| 12338 | 2 | 3 | 4 | | IV-1 | Mgat5 | 4249 | 4-May-15 |
| 12339 | 2 | 3 | 4 | | IV-1 | Mgat5b | 146664 | 12-May-15 |
| 12341 | 2 | 3 | 4 | | IV-1 | Mgl2 | | |
| 12345 | 2 | 3 | 4 | | IV-1 | Mgp | 4256 | 12-May-15 |
| 12347 | 2 | 3 | 4 | | IV-1 | Mgst1 | 4257 | 21-May-15 |
| 12349 | 2 | 3 | 4 | | IV-1 | Mgst3 | 4259 | 4-May-15 |
| 12350 | 2 | 3 | 4 | | IV-1 | Mia | 8190 | 4-May-15 |
| 12354 | 2 | 3 | 4 | | IV-1 | Mib1 | 57534 | 7-Jun-15 |
| 12366 | 2 | 3 | 4 | | IV-1 | Mid1ip1 | 58526 | 4-May-15 |
| 12379 | 2 | 3 | 4 | | IV-1 | Mill2 | | |
| 12380 | 2 | 3 | 4 | | IV-1 | Milr1 | 284021 | 12-May-15 |
| 12460 | 2 | 3 | 4 | | IV-1 | Mir143hg | 728264 | 12-May-15 |
| 12621 | 2 | 3 | 4 | | IV-1 | Mir22hg | 84981 | 12-May-15 |
| 13480 | 2 | 3 | 4 | | IV-1 | Mirlet7bhg | 400931 | 12-May-15 |
| 13518 | 2 | 3 | 4 | | IV-1 | Mlip | 90523 | 4-May-15 |
| 13526 | 2 | 3 | 4 | | IV-1 | Mlph | 79083 | 7-Jun-15 |
| 13530 | 2 | 3 | 4 | | IV-1 | Mlxipl | 51085 | 23-May-15 |
| 13537 | 2 | 3 | 4 | | IV-1 | Mmd2 | 221938 | 4-May-15 |
| 13538 | 2 | 3 | 4 | | IV-1 | Mme | 4311 | 7-Jun-15 |
| 13541 | 2 | 3 | 4 | | IV-1 | Mmgt2 | | |
| 13543 | 2 | 3 | 4 | | IV-1 | Mmp11 | 4320 | 4-May-15 |
| 13545 | 2 | 3 | 4 | | IV-1 | Mmp13 | 4322 | 24-May-15 |
| 13546 | 2 | 3 | 4 | | IV-1 | Mmp14 | 4323 | 10-May-15 |
| 13560 | 2 | 3 | 4 | | IV-1 | Mmp28 | 79148 | 12-May-15 |
| 13561 | 2 | 3 | 4 | | IV-1 | Mmp3 | 4314 | 17-May-15 |
| 13563 | 2 | 3 | 4 | | IV-1 | Mmp8 | 4317 | 24-May-15 |
| 13564 | 2 | 3 | 4 | | IV-1 | Mmp9 | 4318 | 24-May-15 |
| 13569 | 2 | 3 | 4 | | IV-1 | Mn1 | 4330 | 12-May-15 |
| 13575 | 2 | 3 | 4 | | IV-1 | Mns1 | 55329 | 4-May-15 |
| 13578 | 2 | 3 | 4 | | IV-1 | Moap1 | 64112 | 4-May-15 |
| 13585 | 2 | 3 | 4 | | IV-1 | Mob4 | 25843 | 4-May-15 |
| 13593 | 2 | 3 | 4 | | IV-1 | Mogat2 | 80168 | 4-May-15 |
| 13603 | 2 | 3 | 4 | | IV-1 | Morc4 | 79710 | 12-May-15 |
| 13624 | 2 | 3 | 4 | | IV-1 | Mpeg1 | 219972 | 12-May-15 |
| 13636 | 2 | 3 | 4 | | IV-1 | Mpp2 | 4355 | 7-Jun-15 |
| 13637 | 2 | 3 | 4 | | IV-1 | Mpp3 | 4356 | 4-May-15 |
| 13640 | 2 | 3 | 4 | | IV-1 | Mpp6 | 51678 | 7-Jun-15 |
| 13653 | 2 | 3 | 4 | | IV-1 | Mpzl1 | 9019 | 4-May-15 |
| 13654 | 2 | 3 | 4 | | IV-1 | Mpzl2 | 10205 | 4-May-15 |
| 13655 | 2 | 3 | 4 | | IV-1 | Mpzl3 | 196264 | 4-May-15 |
| 13658 | 2 | 3 | 4 | | IV-1 | Mrap2 | 112609 | 24-May-15 |
| 13660 | 2 | 3 | 4 | | IV-1 | Mrc1 | 4360 | 12-May-15 |
| 13663 | 2 | 3 | 4 | | IV-1 | Mreg | 55686 | 4-May-15 |
| 13682 | 2 | 3 | 4 | | IV-1 | Mrgprg | 386746 | 4-May-15 |
| 13724 | 2 | 3 | 4 | | IV-1 | Mrpl37 | 51253 | 4-May-15 |
| 13755 | 2 | 3 | 4 | | IV-1 | Mrps18c | 51023 | 12-May-15 |
| 13790 | 2 | 3 | 4 | | IV-1 | Ms4a6b | | |
| 13791 | 2 | 3 | 4 | | IV-1 | Ms4a6c | | |
| 13792 | 2 | 3 | 4 | | IV-1 | Ms4a6d | | |
| 13793 | 2 | 3 | 4 | | IV-1 | Ms4a7 | 58475 | 7-Jun-15 |
| 13794 | 2 | 3 | 4 | | IV-1 | Ms4a8a | | |
| 13815 | 2 | 3 | 4 | | IV-1 | Msmo1 | 6307 | 4-May-15 |
| 13818 | 2 | 3 | 4 | | IV-1 | Msr1 | 4481 | 17-May-15 |
| 13825 | 2 | 3 | 4 | | IV-1 | Mst1r | 4486 | 12-May-15 |
| 13863 | 2 | 3 | 4 | | IV-1 | Mthfd1l | 25902 | 4-May-15 |
| 13864 | 2 | 3 | 4 | | IV-1 | Mthfd2 | 10797 | 4-May-15 |
| 13867 | 2 | 3 | 4 | | IV-1 | Mthfs | 10588 | 12-May-15 |
| 13875 | 2 | 3 | 4 | | IV-1 | Mtmr11 | 10903 | 17-May-15 |
| 13877 | 2 | 3 | 4 | | IV-1 | Mtmr14 | 64419 | 4-May-15 |
| 13898 | 2 | 3 | 4 | | IV-1 | Mtus1 | 57509 | 12-May-15 |
| 13903 | 2 | 3 | 4 | | IV-1 | Muc1 | 4582 | 22-May-15 |

Fig.22 - 32

| | | | | | | | | |
|---|---|---|---|---|---|---|---|---|
| 13906 | 2 | 3 | 4 | | IV-1 | Muc19 | 283463 | 12-May-15 |
| 13908 | 2 | 3 | 4 | | IV-1 | Muc20 | 200958 | 12-May-15 |
| 13909 | 2 | 3 | 4 | | IV-1 | Muc4 | 4585 | 12-May-15 |
| 13912 | 2 | 3 | 4 | | IV-1 | Muc6 | 4588 | 12-May-15 |
| 13919 | 2 | 3 | 4 | | IV-1 | Mum1l1 | 139221 | 4-May-15 |
| 13934 | 2 | 3 | 4 | | IV-1 | Mup4 | | |
| 13943 | 2 | 3 | 4 | | IV-1 | Mustn1 | 389125 | 12-May-15 |
| 13948 | 2 | 3 | 4 | | IV-1 | Mvd | 4597 | 4-May-15 |
| 13949 | 2 | 3 | 4 | | IV-1 | Mvk | 4598 | 17-May-15 |
| 13950 | 2 | 3 | 4 | | IV-1 | Mvp | 9961 | 7-Jun-15 |
| 13954 | 2 | 3 | 4 | | IV-1 | Mxd3 | 83463 | 4-May-15 |
| 13958 | 2 | 3 | 4 | | IV-1 | Mxra8 | 54587 | 4-May-15 |
| 13964 | 2 | 3 | 4 | | IV-1 | Mybl2 | 4605 | 4-May-15 |
| 13968 | 2 | 3 | 4 | | IV-1 | Mybph | 4608 | 4-May-15 |
| 13970 | 2 | 3 | 4 | | IV-1 | Myc | 4609 | 24-May-15 |
| 13973 | 2 | 3 | 4 | | IV-1 | Mycbpap | 84073 | 4-May-15 |
| 13974 | 2 | 3 | 4 | | IV-1 | Mycl | 4610 | 21-May-15 |
| 13985 | 2 | 3 | 4 | | IV-1 | Myh10 | 4628 | 21-May-15 |
| 13986 | 2 | 3 | 4 | | IV-1 | Myh11 | 4629 | 28-May-15 |
| 13987 | 2 | 3 | 4 | | IV-1 | Myh13 | 8735 | 4-May-15 |
| 14002 | 2 | 3 | 4 | | IV-1 | Myl2 | 4633 | 23-May-15 |
| 14008 | 2 | 3 | 4 | | IV-1 | Myl9 | 10398 | 4-May-15 |
| 14010 | 2 | 3 | 4 | | IV-1 | Mylk | 4638 | 28-May-15 |
| 14027 | 2 | 3 | 4 | | IV-1 | Myo1f | 4542 | 4-May-15 |
| 14040 | 2 | 3 | 4 | | IV-1 | Myoc | 4653 | 16-May-15 |
| 14041 | 2 | 3 | 4 | | IV-1 | Myocd | 93649 | 31-May-15 |
| 14042 | 2 | 3 | 4 | | IV-1 | Myod1 | 4654 | 28-May-15 |
| 14043 | 2 | 3 | 4 | | IV-1 | Myof | 26509 | 4-May-15 |
| 14044 | 2 | 3 | 4 | | IV-1 | Myog | 4656 | 4-May-15 |
| 14047 | 2 | 3 | 4 | | IV-1 | Myom3 | 127294 | 4-May-15 |
| 14061 | 2 | 3 | 4 | | IV-1 | Mzb1 | 51237 | 4-May-15 |
| 14092 | 2 | 3 | 4 | | IV-1 | Naca | 4666 | 3-May-15 |
| 14109 | 2 | 3 | 4 | | IV-1 | Naip5 | | |
| 14119 | 2 | 3 | 4 | | IV-1 | Nans | 54187 | 4-May-15 |
| 14124 | 2 | 3 | 4 | | IV-1 | Nap1l5 | 266812 | 4-May-15 |
| 14129 | 2 | 3 | 4 | | IV-1 | Naprt1 | 93100 | 4-May-15 |
| 14144 | 2 | 3 | 4 | | IV-1 | Nat8l | 339983 | 4-May-15 |
| 14152 | 2 | 3 | 4 | | IV-1 | Nbeal2 | 23218 | 4-May-15 |
| 14157 | 2 | 3 | 4 | | IV-1 | Ncam1 | 4684 | 12-May-15 |
| 14168 | 2 | 3 | 4 | | IV-1 | Nccrp1 | 342897 | 4-May-15 |
| 14171 | 2 | 3 | 4 | | IV-1 | Ncf1 | 653361 | 12-May-15 |
| 14172 | 2 | 3 | 4 | | IV-1 | Ncf2 | 4688 | 12-May-15 |
| 14173 | 2 | 3 | 4 | | IV-1 | Ncf4 | 4689 | 4-May-15 |
| 14209 | 2 | 3 | 4 | | IV-1 | Ndrg2 | 57447 | 4-May-15 |
| 14211 | 2 | 3 | 4 | | IV-1 | Ndrg4 | 65009 | 4-May-15 |
| 14224 | 2 | 3 | 4 | | IV-1 | Ndufa4l2 | 56901 | 4-May-15 |
| 14240 | 2 | 3 | 4 | | IV-1 | Ndufb2 | 4708 | 12-May-15 |
| 14261 | 2 | 3 | 4 | | IV-1 | Neat1 | 283131 | 12-May-15 |
| 14273 | 2 | 3 | 4 | | IV-1 | Nedd9 | 4739 | 4-May-15 |
| 14275 | 2 | 3 | 4 | | IV-1 | Nefl | 4747 | 23-May-15 |
| 14276 | 2 | 3 | 4 | | IV-1 | Nefm | 4741 | 28-May-15 |
| 14279 | 2 | 3 | 4 | | IV-1 | Neil2 | 252969 | 4-May-15 |
| 14280 | 2 | 3 | 4 | | IV-1 | Neil3 | 55247 | 4-May-15 |
| 14284 | 2 | 3 | 4 | | IV-1 | Nek2 | 4751 | 4-May-15 |
| 14290 | 2 | 3 | 4 | | IV-1 | Nek8 | 284086 | 7-Jun-15 |
| 14305 | 2 | 3 | 4 | | IV-1 | Net1 | 10276 | n-2015 |
| 14307 | 2 | 3 | 4 | | IV-1 | Neto2 | 81831 | 4-May-15 |
| 14309 | 2 | 3 | 4 | | IV-1 | Neu2 | 4759 | 4-May-15 |
| 14310 | 2 | 3 | 4 | | IV-1 | Neu3 | 10825 | 3-May-15 |
| 14312 | 2 | 3 | 4 | | IV-1 | Neurl1a | | |
| 14314 | 2 | 3 | 4 | | IV-1 | Neurl2 | 140825 | 4-May-15 |
| 14317 | 2 | 3 | 4 | | IV-1 | Neurod1 | 4760 | 28-May-15 |
| 14319 | 2 | 3 | 4 | | IV-1 | Neurod4 | 58158 | 7-Jun-15 |
| 14331 | 2 | 3 | 4 | | IV-1 | Nfatc2 | 4773 | 28-May-15 |
| 14335 | 2 | 3 | 4 | | IV-1 | Nfe2 | 4778 | 12-May-15 |
| 14342 | 2 | 3 | 4 | | IV-1 | Nfil3 | 4783 | 4-May-15 |
| 14345 | 2 | 3 | 4 | | IV-1 | Nfkb2 | 4791 | 28-May-15 |
| 14346 | 2 | 3 | 4 | | IV-1 | Nfkbia | 4792 | 31-May-15 |
| 14351 | 2 | 3 | 4 | | IV-1 | Nfkbiz | 64332 | 4-May-15 |
| 14365 | 2 | 3 | 4 | | IV-1 | Ngfrap1 | 27018 | 12-May-15 |
| 14367 | 2 | 3 | 4 | | IV-1 | Ngp | | |
| 14369 | 2 | 3 | 4 | | IV-1 | Nhej1 | 79840 | 4-May-15 |
| 14375 | 2 | 3 | 4 | | IV-1 | Nhlrc4 | 283948 | 21-May-15 |
| 14383 | 2 | 3 | 4 | | IV-1 | Nid2 | 22795 | 12-May-15 |
| 14392 | 2 | 3 | 4 | | IV-1 | Nipa1 | 123606 | 12-May-15 |
| 14394 | 2 | 3 | 4 | | IV-1 | Nipal1 | 152519 | 4-May-15 |
| 14395 | 2 | 3 | 4 | | IV-1 | Nipal2 | 79815 | 4-May-15 |
| 14435 | 2 | 3 | 4 | | IV-1 | Nlgn1 | 22871 | 28-May-15 |
| 14447 | 2 | 3 | 4 | | IV-1 | Nlrp1b | | |
| 14464 | 2 | 3 | 4 | | IV-1 | Nmb | 4828 | 7-Jun-15 |
| 14471 | 2 | 3 | 4 | | IV-1 | Nme5 | 8382 | 4-May-15 |
| 14487 | 2 | 3 | 4 | | IV-1 | Nmur1 | 10316 | 12-May-15 |
| 14489 | 2 | 3 | 4 | | IV-1 | Nnat | 4826 | 4-May-15 |
| 14490 | 2 | 3 | 4 | | IV-1 | Nnmt | 4837 | 17-May-15 |
| 14533 | 2 | 3 | 4 | | IV-1 | Notum | 147111 | 9-May-15 |
| 14534 | 2 | 3 | 4 | | IV-1 | Nov | 4856 | 7-Jun-15 |
| 14537 | 2 | 3 | 4 | | IV-1 | Nox1 | 27035 | 10-May-15 |
| 14540 | 2 | 3 | 4 | | IV-1 | Noxa1 | 10811 | 26-May-15 |
| 14548 | 2 | 3 | 4 | | IV-1 | Npb | 256933 | 4-May-15 |
| 14553 | 2 | 3 | 4 | | IV-1 | Npcd | | |
| 14557 | 2 | 3 | 4 | | IV-1 | Npff | 8620 | 4-May-15 |
| 14566 | 2 | 3 | 4 | | IV-1 | Npl | 80896 | 4-May-15 |
| 14570 | 2 | 3 | 4 | | IV-1 | Npm3 | 10360 | 4-May-15 |
| 14572 | 2 | 3 | 4 | | IV-1 | Npnt | 255743 | 4-May-15 |
| 14573 | 2 | 3 | 4 | | IV-1 | Nppa | 4878 | 12-May-15 |
| 14576 | 2 | 3 | 4 | | IV-1 | Npr1 | 4881 | 24-May-15 |
| 14584 | 2 | 3 | 4 | | IV-1 | Nptx1 | 4884 | 3-May-15 |

| | | | | | | | | |
|---|---|---|---|---|---|---|---|---|
| 14585 | 2 | 3 | 4 | | IV-1 | Nptx2 | 4885 | 12-May-15 |
| 14588 | 2 | 3 | 4 | | IV-1 | Npw | 283869 | 12-May-15 |
| 14589 | 2 | 3 | 4 | | IV-1 | Npy | 4852 | 17-May-15 |
| 14595 | 2 | 3 | 4 | | IV-1 | Nqo1 | 1728 | 17-May-15 |
| 14599 | 2 | 3 | 4 | | IV-1 | Nr1d1 | 9572 | 12-May-15 |
| 14603 | 2 | 3 | 4 | | IV-1 | Nr1h4 | 9971 | 31-May-15 |
| 14605 | 2 | 3 | 4 | | IV-1 | Nr1i2 | 8856 | 24-May-15 |
| 14606 | 2 | 3 | 4 | | IV-1 | Nr1i3 | 9970 | 4-May-15 |
| 14608 | 2 | 3 | 4 | | IV-1 | Nr2c2 | 7182 | 31-May-15 |
| 14612 | 2 | 3 | 4 | | IV-1 | Nr2f1 | 7025 | 7-Jun-15 |
| 14613 | 2 | 3 | 4 | | IV-1 | Nr2f2 | 7026 | 7-Jun-15 |
| 14619 | 2 | 3 | 4 | | IV-1 | Nr4a3 | 8013 | 4-May-15 |
| 14629 | 2 | 3 | 4 | | IV-1 | Nrbp2 | 340371 | 4-May-15 |
| 14640 | 2 | 3 | 4 | | IV-1 | Nrgn | 4900 | 4-May-15 |
| 14641 | 2 | 3 | 4 | | IV-1 | Nrip1 | 8204 | 3-May-15 |
| 14647 | 2 | 3 | 4 | | IV-1 | Nrn1 | 51299 | 4-May-15 |
| 14656 | 2 | 3 | 4 | | IV-1 | Nrtn | 4902 | 23-May-15 |
| 14672 | 2 | 3 | 4 | | IV-1 | Nsmf | 26012 | 4-May-15 |
| 14678 | 2 | 3 | 4 | | IV-1 | Nsun7 | 79730 | 4-May-15 |
| 14688 | 2 | 3 | 4 | | IV-1 | Nt5e | 4907 | 4-May-15 |
| 14692 | 2 | 3 | 4 | | IV-1 | Ntf5 | 4909 | 4-May-15 |
| 14701 | 2 | 3 | 4 | | IV-1 | Ntng2 | 84628 | 4-May-15 |
| 14707 | 2 | 3 | 4 | | IV-1 | Ntsr1 | 4923 | 4-May-15 |
| 14716 | 2 | 3 | 4 | | IV-1 | Nucb2 | 4925 | 4-May-15 |
| 14723 | 2 | 3 | 4 | | IV-1 | Nudt10 | 170685 | 7-Jun-15 |
| 14724 | 2 | 3 | 4 | | IV-1 | Nudt11 | 55190 | 4-May-15 |
| 14731 | 2 | 3 | 4 | | IV-1 | Nudt17 | 200035 | 23-May-15 |
| 14736 | 2 | 3 | 4 | | IV-1 | Nudt22 | 84304 | 4-May-15 |
| 14738 | 2 | 3 | 4 | | IV-1 | Nudt4 | 11163 | 12-May-15 |
| 14758 | 2 | 3 | 4 | | IV-1 | Nup210 | 23225 | 4-May-15 |
| 14775 | 2 | 3 | 4 | | IV-1 | Nupr1 | 26471 | 4-May-15 |
| 14780 | 2 | 3 | 4 | | IV-1 | Nutf2-ps1 | | |
| 14790 | 2 | 3 | 4 | | IV-1 | Nxnl1 | 115861 | 4-May-15 |
| 14791 | 2 | 3 | 4 | | IV-1 | Nxnl2 | 158046 | 4-May-15 |
| 14793 | 2 | 3 | 4 | | IV-1 | Nxpe3 | 91775 | 4-May-15 |
| 14795 | 2 | 3 | 4 | | IV-1 | Nxpe5 | | |
| 14810 | 2 | 3 | 4 | | IV-1 | Oas1b | | |
| 14813 | 2 | 3 | 4 | | IV-1 | Oas1e | | |
| 14833 | 2 | 3 | 4 | | IV-1 | Obp2a | 29991 | 12-May-15 |
| 14835 | 2 | 3 | 4 | | IV-1 | Obscn | 84033 | 28-May-15 |
| 14836 | 2 | 3 | 4 | | IV-1 | Obsl1 | 23363 | 23-May-15 |
| 14842 | 2 | 3 | 4 | | IV-1 | Ocln | 100506658 | 12-May-15 |
| 14845 | 2 | 3 | 4 | | IV-1 | Ocstamp | 128506 | 4-May-15 |
| 14852 | 2 | 3 | 4 | | IV-1 | Odf3b | 440836 | 4-May-15 |
| 14870 | 2 | 3 | 4 | | IV-1 | Oit3 | 170392 | 4-May-15 |
| 14874 | 2 | 3 | 4 | | IV-1 | Olfm2 | 93145 | 12-May-15 |
| 14878 | 2 | 3 | 4 | | IV-1 | Olfml2a | 169611 | 4-May-15 |
| 14910 | 2 | 3 | 4 | | IV-1 | Olfr1033 | | |
| 14911 | 2 | 3 | 4 | | IV-1 | Olfr1034 | | |
| 15202 | 2 | 3 | 4 | | IV-1 | Olfr1372-ps1 | | |
| 15210 | 2 | 3 | 4 | | IV-1 | Olfr1383 | | |
| 15453 | 2 | 3 | 4 | | IV-1 | Olfr330 | | |
| 15936 | 2 | 3 | 4 | | IV-1 | Olfr920 | | |
| 15998 | 2 | 3 | 4 | | IV-1 | Olig1 | 116448 | 4-May-15 |
| 16003 | 2 | 3 | 4 | | IV-1 | Omd | 4958 | 4-May-15 |
| 16034 | 2 | 3 | 4 | | IV-1 | Optc | 26254 | 4-May-15 |
| 16040 | 2 | 3 | 4 | | IV-1 | Orc1 | 4998 | 7-Jun-15 |
| 16054 | 2 | 3 | 4 | | IV-1 | Osbp2 | 23762 | 12-May-15 |
| 16055 | 2 | 3 | 4 | | IV-1 | Osbpl10 | 114884 | 4-May-15 |
| 16057 | 2 | 3 | 4 | | IV-1 | Osbpl1a | 114876 | 4-May-15 |
| 16062 | 2 | 3 | 4 | | IV-1 | Osbpl7 | 114881 | 21-May-15 |
| 16066 | 2 | 3 | 4 | | IV-1 | Oscp1 | 127700 | 4-May-15 |
| 16070 | 2 | 3 | 4 | | IV-1 | Osgin1 | 29948 | 4-May-15 |
| 16073 | 2 | 3 | 4 | | IV-1 | Osmr | 9180 | 23-May-15 |
| 16074 | 2 | 3 | 4 | | IV-1 | Osr1 | 130497 | 7-Jun-15 |
| 16075 | 2 | 3 | 4 | | IV-1 | Osr2 | 116039 | 4-May-15 |
| 16083 | 2 | 3 | 4 | | IV-1 | Otof | 9381 | 23-May-15 |
| 16113 | 2 | 3 | 4 | | IV-1 | Ovol2 | 58495 | 4-May-15 |
| 16127 | 2 | 3 | 4 | | IV-1 | P2rx1 | 5023 | 17-May-15 |
| 16132 | 2 | 3 | 4 | | IV-1 | P2rx6 | 9127 | 4-May-15 |
| 16139 | 2 | 3 | 4 | | IV-1 | P2ry2 | 5029 | 17-May-15 |
| 16140 | 2 | 3 | 4 | | IV-1 | P2ry4 | 5030 | 21-May-15 |
| 16143 | 2 | 3 | 4 | | IV-1 | P4ha2 | 8974 | 4-May-15 |
| 16161 | 2 | 3 | 4 | | IV-1 | Pacsin1 | 29993 | 4-May-15 |
| 16163 | 2 | 3 | 4 | | IV-1 | Pacsin3 | 29763 | 4-May-15 |
| 16164 | 2 | 3 | 4 | | IV-1 | Padi1 | 29943 | 4-May-15 |
| 16172 | 2 | 3 | 4 | | IV-1 | Pafah1b3 | 5050 | 4-May-15 |
| 16174 | 2 | 3 | 4 | | IV-1 | Pag1 | 55824 | 4-May-15 |
| 16176 | 2 | 3 | 4 | | IV-1 | Pah | 5053 | 23-May-15 |
| 16186 | 2 | 3 | 4 | | IV-1 | Pak6 | 56924 | 4-May-15 |
| 16194 | 2 | 3 | 4 | | IV-1 | Palmd | 54873 | 4-May-15 |
| 16196 | 2 | 3 | 4 | | IV-1 | Pam16 | 51025 | 28-May-15 |
| 16197 | 2 | 3 | 4 | | IV-1 | Pamr1 | 25891 | 4-May-15 |
| 16200 | 2 | 3 | 4 | | IV-1 | Pank1 | 53354 | 4-May-15 |
| 16207 | 2 | 3 | 4 | | IV-1 | Paox | 196743 | 4-May-15 |
| 16216 | 2 | 3 | 4 | | IV-1 | Pappa | 5069 | 17-May-15 |
| 16217 | 2 | 3 | 4 | | IV-1 | Pappa2 | 60676 | 17-May-15 |
| 16227 | 2 | 3 | 4 | | IV-1 | Pard3 | 56288 | 4-May-15 |
| 16230 | 2 | 3 | 4 | | IV-1 | Pard6b | 84612 | 4-May-15 |
| 16231 | 2 | 3 | 4 | | IV-1 | Pard6g | 84552 | 12-May-15 |
| 16236 | 2 | 3 | 4 | | IV-1 | Parm1 | 25849 | 4-May-15 |
| 16248 | 2 | 3 | 4 | | IV-1 | Parp8 | 79668 | 12-May-15 |
| 16252 | 2 | 3 | 4 | | IV-1 | Parva | 55742 | 4-May-15 |
| 16272 | 2 | 3 | 4 | | IV-1 | Pax9 | 5083 | 28-May-15 |
| 16277 | 2 | 3 | 4 | | IV-1 | Pbld1 | | |
| 16291 | 2 | 3 | 4 | | IV-1 | Pcbp3 | 54039 | 4-May-15 |
| 16295 | 2 | 3 | 4 | | IV-1 | Pcdh1 | 5097 | 4-May-15 |

Fig.22 - 33

| | | | | | | | | |
|---|---|---|---|---|---|---|---|---|
| 16360 | 2 | 3 | 4 | | IV-1 | Pcdhgb6 | 56100 | 4-May-15 |
| 16370 | 2 | 3 | 4 | | IV-1 | Pcgf2 | 7703 | 4-May-15 |
| 16377 | 2 | 3 | 4 | | IV-1 | Pck2 | 5106 | 12-May-15 |
| 16391 | 2 | 3 | 4 | | IV-1 | Pcolce2 | 26577 | 12-May-15 |
| 16392 | 2 | 3 | 4 | | IV-1 | Pcp2 | 126006 | 7-Jun-15 |
| 16400 | 2 | 3 | 4 | | IV-1 | Pcsk4 | 54760 | 4-May-15 |
| 16402 | 2 | 3 | 4 | | IV-1 | Pcsk6 | 5046 | 4-May-15 |
| 16405 | 2 | 3 | 4 | | IV-1 | Pctp | 58488 | 4-May-15 |
| 16406 | 2 | 3 | 4 | | IV-1 | Pcx | | |
| 16408 | 2 | 3 | 4 | | IV-1 | Pcyox1l | 78991 | 4-May-15 |
| 16421 | 2 | 3 | 4 | | IV-1 | Pdcd5 | 9141 | 4-May-15 |
| 16436 | 2 | 3 | 4 | | IV-1 | Pde3a | 5139 | 31-May-15 |
| 16437 | 2 | 3 | 4 | | IV-1 | Pde3b | 5140 | 4-May-15 |
| 16441 | 2 | 3 | 4 | | IV-1 | Pde4d | 5144 | 2-Jun-15 |
| 16442 | 2 | 3 | 4 | | IV-1 | Pde4dip | 9659 | 12-May-15 |
| 16458 | 2 | 3 | 4 | | IV-1 | Pdgfc | 56034 | 17-May-15 |
| 16460 | 2 | 3 | 4 | | IV-1 | Pdgfra | 5156 | 17-May-15 |
| 16467 | 2 | 3 | 4 | | IV-1 | Pdia2 | 64714 | 12-May-15 |
| 16469 | 2 | 3 | 4 | | IV-1 | Pdia4 | 9601 | 4-May-15 |
| 16470 | 2 | 3 | 4 | | IV-1 | Pdia5 | 10954 | 4-May-15 |
| 16474 | 2 | 3 | 4 | | IV-1 | Pdk1 | 5163 | 7-Jun-15 |
| 16475 | 2 | 3 | 4 | | IV-1 | Pdk2 | 5164 | 4-May-15 |
| 16479 | 2 | 3 | 4 | | IV-1 | Pdlim2 | 64236 | 4-May-15 |
| 16483 | 2 | 3 | 4 | | IV-1 | Pdlim7 | 9260 | 24-May-15 |
| 16488 | 2 | 3 | 4 | | IV-1 | Pdpr | 55066 | 4-May-15 |
| 16490 | 2 | 3 | 4 | | IV-1 | Pds5a | 23244 | 4-May-15 |
| 16494 | 2 | 3 | 4 | | IV-1 | Pdx1 | 3651 | 7-Jun-15 |
| 16496 | 2 | 3 | 4 | | IV-1 | Pdxk | 8566 | 4-May-15 |
| 16514 | 2 | 3 | 4 | | IV-1 | Pear1 | 375033 | 4-May-15 |
| 16515 | 2 | 3 | 4 | | IV-1 | Pebp1 | 5037 | 4-May-15 |
| 16520 | 2 | 3 | 4 | | IV-1 | Peg10 | 23089 | 4-May-15 |
| 16522 | 2 | 3 | 4 | | IV-1 | Peg13 | | |
| 16523 | 2 | 3 | 4 | | IV-1 | Peg3 | 5178 | 12-May-15 |
| 16524 | 2 | 3 | 4 | | IV-1 | Peg3os | | |
| 16530 | 2 | 3 | 4 | | IV-1 | Pemt | 10400 | 7-Jun-15 |
| 16531 | 2 | 3 | 4 | | IV-1 | Penk | 5179 | 12-May-15 |
| 16534 | 2 | 3 | 4 | | IV-1 | Per1 | 5187 | 12-May-15 |
| 16535 | 2 | 3 | 4 | | IV-1 | Per2 | 8864 | 17-May-15 |
| 16536 | 2 | 3 | 4 | | IV-1 | Per3 | 8863 | 17-May-15 |
| 16538 | 2 | 3 | 4 | | IV-1 | Perm1 | 84808 | 12-May-15 |
| 16539 | 2 | 3 | 4 | | IV-1 | Perp | 64065 | 4-May-15 |
| 16543 | 2 | 3 | 4 | | IV-1 | Pet117 | 100303755 | 4-May-15 |
| 16547 | 2 | 3 | 4 | | IV-1 | Pex11a | 8800 | 4-May-15 |
| 16553 | 2 | 3 | 4 | | IV-1 | Pex16 | 9409 | 23-May-15 |
| 16565 | 2 | 3 | 4 | | IV-1 | Pfdn2 | 5202 | 4-May-15 |
| 16568 | 2 | 3 | 4 | | IV-1 | Pfkfb1 | 5207 | 4-May-15 |
| 16570 | 2 | 3 | 4 | | IV-1 | Pfkfb3 | 5209 | 12-May-15 |
| 16573 | 2 | 3 | 4 | | IV-1 | Pfkm | 5213 | 12-May-15 |
| 16576 | 2 | 3 | 4 | | IV-1 | Pfn2 | 5217 | 4-May-15 |
| 16584 | 2 | 3 | 4 | | IV-1 | Pgap1 | 80055 | 4-May-15 |
| 16585 | 2 | 3 | 4 | | IV-1 | Pgap2 | 27315 | 12-May-15 |
| 16586 | 2 | 3 | 4 | | IV-1 | Pgap3 | 93210 | 12-May-15 |
| 16591 | 2 | 3 | 4 | | IV-1 | Pgf | 5228 | 21-May-15 |
| 16598 | 2 | 3 | 4 | | IV-1 | Pglyrp3 | 114771 | 4-May-15 |
| 16602 | 2 | 3 | 4 | | IV-1 | Pgm2l1 | 283209 | 4-May-15 |
| 16604 | 2 | 3 | 4 | | IV-1 | Pgm5 | 5239 | 17-May-15 |
| 16608 | 2 | 3 | 4 | | IV-1 | Pgr | 5241 | 24-May-15 |
| 16610 | 2 | 3 | 4 | | IV-1 | Pgrmc1 | 10857 | 3-May-15 |
| 16633 | 2 | 3 | 4 | | IV-1 | Phf19 | 26147 | 4-May-15 |
| 16642 | 2 | 3 | 4 | | IV-1 | Phf6 | 84295 | 24-May-15 |
| 16643 | 2 | 3 | 4 | | IV-1 | Phf7 | 51533 | 4-May-15 |
| 16645 | 2 | 3 | 4 | | IV-1 | Phgdh | 26227 | 13-May-15 |
| 16646 | 2 | 3 | 4 | | IV-1 | Phgr1 | 644844 | 4-May-15 |
| 16648 | 2 | 3 | 4 | | IV-1 | Phka1 | 5255 | 4-May-15 |
| 16651 | 2 | 3 | 4 | | IV-1 | Phkg1 | 5260 | 12-May-15 |
| 16656 | 2 | 3 | 4 | | IV-1 | Phldb1 | 23187 | 29-May-15 |
| 16661 | 2 | 3 | 4 | | IV-1 | Phospho1 | 162466 | 4-May-15 |
| 16668 | 2 | 3 | 4 | | IV-1 | Phtf1os | | |
| 16669 | 2 | 3 | 4 | | IV-1 | Phtf2 | 57157 | 4-May-15 |
| 16673 | 2 | 3 | 4 | | IV-1 | Phyhip | 9796 | 4-May-15 |
| 16696 | 2 | 3 | 4 | | IV-1 | Piga | 5277 | 12-May-15 |
| 16697 | 2 | 3 | 4 | | IV-1 | Pigb | 9488 | 23-May-15 |
| 16731 | 2 | 3 | 4 | | IV-1 | Pik3r1 | 5295 | 28-May-15 |
| 16742 | 2 | 3 | 4 | | IV-1 | Pim2 | 11040 | 4-May-15 |
| 16746 | 2 | 3 | 4 | | IV-1 | Pin4 | 5303 | 12-May-15 |
| 16748 | 2 | 3 | 4 | | IV-1 | Pink1 | 65018 | 31-May-15 |
| 16751 | 2 | 3 | 4 | | IV-1 | Pip | 5304 | 7-Jun-15 |
| 16759 | 2 | 3 | 4 | | IV-1 | Pipox | 51268 | 4-May-15 |
| 16760 | 2 | 3 | 4 | | IV-1 | Pir | 8544 | 3-May-15 |
| 16761 | 2 | 3 | 4 | | IV-1 | Pira1 | | |
| 16762 | 2 | 3 | 4 | | IV-1 | Pira11 | | |
| 16767 | 2 | 3 | 4 | | IV-1 | Pirb | 11025 | 4-May-15 |
| 16777 | 2 | 3 | 4 | | IV-1 | Pitpnm1 | 9600 | 4-May-15 |
| 16781 | 2 | 3 | 4 | | IV-1 | Pitrm1 | 10531 | 23-May-15 |
| 16783 | 2 | 3 | 4 | | IV-1 | Pitx2 | 5308 | 23-May-15 |
| 16788 | 2 | 3 | 4 | | IV-1 | Pja1 | 64219 | 4-May-15 |
| 16795 | 2 | 3 | 4 | | IV-1 | Pkd2l2 | 27039 | 12-May-15 |
| 16801 | 2 | 3 | 4 | | IV-1 | Pkib | 5570 | 4-May-15 |
| 16803 | 2 | 3 | 4 | | IV-1 | Pklr | 5313 | 17-May-15 |
| 16805 | 2 | 3 | 4 | | IV-1 | Pkmyt1 | 9088 | 4-May-15 |
| 16810 | 2 | 3 | 4 | | IV-1 | Pknox2 | 63876 | 12-May-15 |
| 16811 | 2 | 3 | 4 | | IV-1 | Pkp1 | 5317 | 4-May-15 |
| 16813 | 2 | 3 | 4 | | IV-1 | Pkp3 | 11187 | 4-May-15 |
| 16815 | 2 | 3 | 4 | | IV-1 | Pla1a | 51365 | 12-May-15 |
| 16817 | 2 | 3 | 4 | | IV-1 | Pla2g10os | | |
| 16818 | 2 | 3 | 4 | | IV-1 | Pla2g12a | 81579 | 4-May-15 |
| 16825 | 2 | 3 | 4 | | IV-1 | Pla2g2d | 26279 | 12-May-15 |
| 16837 | 2 | 3 | 4 | | IV-1 | Pla2g7 | 7941 | 12-May-15 |
| 16851 | 2 | 3 | 4 | | IV-1 | Plb1 | 151056 | 4-May-15 |
| 16867 | 2 | 3 | 4 | | IV-1 | Plcl2 | 23228 | 14-May-15 |
| 16875 | 2 | 3 | 4 | | IV-1 | Pld4 | 122618 | 12-May-15 |
| 16877 | 2 | 3 | 4 | | IV-1 | Pld6 | 201164 | 29-May-15 |
| 16880 | 2 | 3 | 4 | | IV-1 | Plek | 5341 | 4-May-15 |
| 16884 | 2 | 3 | 4 | | IV-1 | Plekha3 | 65977 | 12-May-15 |
| 16887 | 2 | 3 | 4 | | IV-1 | Plekha6 | 22874 | 4-May-15 |
| 16890 | 2 | 3 | 4 | | IV-1 | Plekhb1 | 58473 | 4-May-15 |
| 16893 | 2 | 3 | 4 | | IV-1 | Plekhd1os | | |
| 16901 | 2 | 3 | 4 | | IV-1 | Plekhg6 | 55200 | 4-May-15 |
| 16902 | 2 | 3 | 4 | | IV-1 | Plekhh1 | 57475 | 12-May-15 |
| 16904 | 2 | 3 | 4 | | IV-1 | Plekhh3 | 79990 | 12-May-15 |
| 16912 | 2 | 3 | 4 | | IV-1 | Plekhs1 | 79949 | 30-Apr-15 |
| 16914 | 2 | 3 | 4 | | IV-1 | Plet1os | | |
| 16916 | 2 | 3 | 4 | | IV-1 | Plgrkt | 55848 | 4-May-15 |
| 16918 | 2 | 3 | 4 | | IV-1 | Plin2 | 123 | 4-May-15 |
| 16921 | 2 | 3 | 4 | | IV-1 | Plin5 | 440503 | 4-May-15 |
| 16923 | 2 | 3 | 4 | | IV-1 | Plk2 | 10769 | 17-May-15 |
| 16924 | 2 | 3 | 4 | | IV-1 | Plk3 | 1263 | 4-May-15 |
| 16927 | 2 | 3 | 4 | | IV-1 | Pllp | 51090 | 4-May-15 |
| 16936 | 2 | 3 | 4 | | IV-1 | Pls3 | 5358 | 12-May-15 |
| 16947 | 2 | 3 | 4 | | IV-1 | Plxna2 | 5362 | 4-May-15 |
| 16952 | 2 | 3 | 4 | | IV-1 | Plxnb2 | 23654 | 4-May-15 |
| 16957 | 2 | 3 | 4 | | IV-1 | Pm20d2 | 135293 | 4-May-15 |
| 16960 | 2 | 3 | 4 | | IV-1 | Pmel | 6490 | 12-May-15 |
| 16974 | 2 | 3 | 4 | | IV-1 | Pmvk | 10654 | 4-May-15 |
| 16994 | 2 | 3 | 4 | | IV-1 | Pnp2 | | |
| 16996 | 2 | 3 | 4 | | IV-1 | Pnpla2 | 57104 | 4-May-15 |
| 16998 | 2 | 3 | 4 | | IV-1 | Pnpla5 | 150379 | 4-May-15 |
| 17009 | 2 | 3 | 4 | | IV-1 | Podn | 127435 | 4-May-15 |
| 17010 | 2 | 3 | 4 | | IV-1 | Podnl1 | 79883 | 4-May-15 |
| 17023 | 2 | 3 | 4 | | IV-1 | Pold2 | 5425 | 12-May-15 |
| 17056 | 2 | 3 | 4 | | IV-1 | Polr2k | 5440 | 4-May-15 |
| 17073 | 2 | 3 | 4 | | IV-1 | Pomc | 5443 | 23-May-15 |
| 17080 | 2 | 3 | 4 | | IV-1 | Pon1 | 5444 | 17-May-15 |
| 17084 | 2 | 3 | 4 | | IV-1 | Pop4 | 10775 | 4-May-15 |
| 17087 | 2 | 3 | 4 | | IV-1 | Popdc2 | 64091 | 12-May-15 |
| 17088 | 2 | 3 | 4 | | IV-1 | Popdc3 | 64208 | 4-May-15 |
| 17090 | 2 | 3 | 4 | | IV-1 | Porcn | 64840 | 23-May-15 |
| 17091 | 2 | 3 | 4 | | IV-1 | Postn | 10631 | 17-May-15 |
| 17096 | 2 | 3 | 4 | | IV-1 | Pou2af1 | 5450 | 4-May-15 |
| 17099 | 2 | 3 | 4 | | IV-1 | Pou2f3 | 25833 | 28-May-15 |
| 17100 | 2 | 3 | 4 | | IV-1 | Pou3f1 | 5453 | 4-May-15 |
| 17116 | 2 | 3 | 4 | | IV-1 | Ppap2a | 8611 | 4-May-15 |
| 17118 | 2 | 3 | 4 | | IV-1 | Ppap2c | 8612 | 4-May-15 |
| 17123 | 2 | 3 | 4 | | IV-1 | Ppara | 5465 | 17-May-15 |
| 17125 | 2 | 3 | 4 | | IV-1 | Pparg | 5468 | 17-May-15 |
| 17127 | 2 | 3 | 4 | | IV-1 | Ppargc1b | 133522 | 4-May-15 |
| 17129 | 2 | 3 | 4 | | IV-1 | Ppbp | 5473 | 12-May-15 |
| 17166 | 2 | 3 | 4 | | IV-1 | Ppm1j | 333926 | 12-May-15 |
| 17179 | 2 | 3 | 4 | | IV-1 | Ppp1r12b | 4660 | 14-May-15 |
| 17187 | 2 | 3 | 4 | | IV-1 | Ppp1r15a | 23645 | 12-May-15 |
| 17191 | 2 | 3 | 4 | | IV-1 | Ppp1r17 | 10842 | 4-May-15 |
| 17193 | 2 | 3 | 4 | | IV-1 | Ppp1r1a | 5502 | 4-May-15 |
| 17194 | 2 | 3 | 4 | | IV-1 | Ppp1r1b | 84152 | 4-May-15 |
| 17198 | 2 | 3 | 4 | | IV-1 | Ppp1r26 | 9858 | 4-May-15 |
| 17208 | 2 | 3 | 4 | | IV-1 | Ppp1r3b | 79660 | 4-May-15 |
| 17209 | 2 | 3 | 4 | | IV-1 | Ppp1r3c | 5507 | 4-May-15 |
| 17211 | 2 | 3 | 4 | | IV-1 | Ppp1r3e | 90673 | 4-May-15 |
| 17214 | 2 | 3 | 4 | | IV-1 | Ppp1r3g | 648791 | 4-May-15 |
| 17225 | 2 | 3 | 4 | | IV-1 | Ppp2r2b | 5521 | 23-May-15 |
| 17229 | 2 | 3 | 4 | | IV-1 | Ppp2r3a | 5523 | 4-May-15 |
| 17258 | 2 | 3 | 4 | | IV-1 | Ppy | 5539 | 4-May-15 |
| 17267 | 2 | 3 | 4 | | IV-1 | Pramef12 | 390999 | 4-May-15 |
| 17278 | 2 | 3 | 4 | | IV-1 | Prap1 | 118471 | 12-May-15 |
| 17298 | 2 | 3 | 4 | | IV-1 | Prdx2 | 7001 | 4-May-15 |
| 17307 | 2 | 3 | 4 | | IV-1 | Prelp | 5549 | 12-May-15 |
| 17315 | 2 | 3 | 4 | | IV-1 | Prg4 | 10216 | 7-Jun-15 |
| 17326 | 2 | 3 | 4 | | IV-1 | Prkaa2 | 5563 | 4-May-15 |
| 17334 | 2 | 3 | 4 | | IV-1 | Prkag3 | 53632 | 12-May-15 |
| 17338 | 2 | 3 | 4 | | IV-1 | Prkar2b | 5577 | 12-May-15 |
| 17360 | 2 | 3 | 4 | | IV-1 | Prl | 5617 | 17-May-15 |
| 17402 | 2 | 3 | 4 | | IV-1 | Prnd | 23627 | 12-May-15 |
| 17404 | 2 | 3 | 4 | | IV-1 | Prob1 | 389333 | 4-May-15 |
| 17406 | 2 | 3 | 4 | | IV-1 | Proca1 | 147011 | 4-May-15 |
| 17407 | 2 | 3 | 4 | | IV-1 | Procr | 10544 | 4-May-15 |
| 17408 | 2 | 3 | 4 | | IV-1 | Prodh | 5625 | 4-May-15 |
| 17415 | 2 | 3 | 4 | | IV-1 | Prom1 | 8842 | 23-May-15 |
| 17422 | 2 | 3 | 4 | | IV-1 | Proser2 | 254427 | 4-May-15 |
| 17454 | 2 | 3 | 4 | | IV-1 | Prr15 | 222171 | 4-May-15 |
| 17455 | 2 | 3 | 4 | | IV-1 | Prr15l | 79170 | 4-May-15 |
| 17456 | 2 | 3 | 4 | | IV-1 | Prr16 | 51334 | 12-May-15 |
| 17462 | 2 | 3 | 4 | | IV-1 | Prr27 | 401137 | 4-May-15 |
| 17464 | 2 | 3 | 4 | | IV-1 | Prr30 | 339779 | 4-May-15 |
| 17475 | 2 | 3 | 4 | | IV-1 | Prrg1 | 5638 | 4-May-15 |
| 17479 | 2 | 3 | 4 | | IV-1 | Prrt1 | 80863 | 4-May-15 |
| 17487 | 2 | 3 | 4 | | IV-1 | Prss12 | 8492 | 4-May-15 |
| 17493 | 2 | 3 | 4 | | IV-1 | Prss27 | 83886 | 4-May-15 |
| 17512 | 2 | 3 | 4 | | IV-1 | Prss46 | 100287362 | 4-May-15 |
| 17517 | 2 | 3 | 4 | | IV-1 | Prss53 | 339105 | 4-May-15 |
| 17530 | 2 | 3 | 4 | | IV-1 | Psapl1 | 768239 | 4-May-15 |
| 17531 | 2 | 3 | 4 | | IV-1 | Psat1 | 29968 | 12-May-15 |
| 17532 | 2 | 3 | 4 | | IV-1 | Psca | 8000 | 4-May-15 |
| 17548 | 2 | 3 | 4 | | IV-1 | Psg25 | | |
| 17596 | 2 | 3 | 4 | | IV-1 | Psme1 | 5720 | 4-May-15 |
| 17605 | 2 | 3 | 4 | | IV-1 | Psmg4 | 389362 | 4-May-15 |

Fig.22 - 34

| | | | | | | | | |
|---|---|---|---|---|---|---|---|---|
| 17612 | 2 | 3 | 4 | | IV-1 | Pstpip1 | 9051 | 12-May-15 |
| 17620 | 2 | 3 | 4 | | IV-1 | Ptcd2 | 79810 | 4-May-15 |
| 17622 | 2 | 3 | 4 | | IV-1 | Ptch1 | 5727 | 23-May-15 |
| 17630 | 2 | 3 | 4 | | IV-1 | Ptdss2 | 81490 | 12-May-15 |
| 17632 | 2 | 3 | 4 | | IV-1 | Pter | 9317 | 4-May-15 |
| 17639 | 2 | 3 | 4 | | IV-1 | Ptger3 | 5733 | 12-May-15 |
| 17641 | 2 | 3 | 4 | | IV-1 | Ptges | 9536 | 12-May-15 |
| 17644 | 2 | 3 | 4 | | IV-1 | Ptges3l | 100885848 | 4-May-15 |
| 17648 | 2 | 3 | 4 | | IV-1 | Ptgis | 5740 | 12-May-15 |
| 17655 | 2 | 3 | 4 | | IV-1 | Pth1r | 5745 | 12-May-15 |
| 17658 | 2 | 3 | 4 | | IV-1 | Pthlh | 5744 | 12-May-15 |
| 17661 | 2 | 3 | 4 | | IV-1 | Ptk6 | 5753 | 12-May-15 |
| 17669 | 2 | 3 | 4 | | IV-1 | Ptp4a3 | 11156 | 24-May-15 |
| 17671 | 2 | 3 | 4 | | IV-1 | Ptpla | 9200 | 12-May-15 |
| 17679 | 2 | 3 | 4 | | IV-1 | Ptpn13 | 5783 | 12-May-15 |
| 17685 | 2 | 3 | 4 | | IV-1 | Ptpn22 | 26191 | 17-May-15 |
| 17687 | 2 | 3 | 4 | | IV-1 | Ptpn3 | 5774 | 4-May-15 |
| 17699 | 2 | 3 | 4 | | IV-1 | Ptprf | 5792 | 4-May-15 |
| 17717 | 2 | 3 | 4 | | IV-1 | Ptrh1 | 138428 | 4-May-15 |
| 17740 | 2 | 3 | 4 | | IV-1 | Pvrl2 | 5819 | 12-May-15 |
| 17749 | 2 | 3 | 4 | | IV-1 | Pxdn | 7837 | 4-May-15 |
| 17751 | 2 | 3 | 4 | | IV-1 | Pxmp2 | 5827 | 12-May-15 |
| 17763 | 2 | 3 | 4 | | IV-1 | Pygl | 5836 | 23-May-15 |
| 17764 | 2 | 3 | 4 | | IV-1 | Pygm | 5837 | 23-May-15 |
| 17765 | 2 | 3 | 4 | | IV-1 | Pygo1 | 26108 | 4-May-15 |
| 17776 | 2 | 3 | 4 | | IV-1 | Qpct | 25797 | 4-May-15 |
| 17779 | 2 | 3 | 4 | | IV-1 | Qrfp | 347148 | 4-May-15 |
| 17786 | 2 | 3 | 4 | | IV-1 | Qsox2 | 169714 | 4-May-15 |
| 17812 | 2 | 3 | 4 | | IV-1 | Rab17 | 64284 | 4-May-15 |
| 17816 | 2 | 3 | 4 | | IV-1 | Rab20 | 55647 | 4-May-15 |
| 17823 | 2 | 3 | 4 | | IV-1 | Rab26os | | |
| 17834 | 2 | 3 | 4 | | IV-1 | Rab34 | 83871 | 4-May-15 |
| 17838 | 2 | 3 | 4 | | IV-1 | Rab38 | 23682 | 4-May-15 |
| 17840 | 2 | 3 | 4 | | IV-1 | Rab39b | 116442 | 4-May-15 |
| 17848 | 2 | 3 | 4 | | IV-1 | Rab3ip | 117177 | 4-May-15 |
| 17851 | 2 | 3 | 4 | | IV-1 | Rab42 | 115273 | 4-May-15 |
| 17854 | 2 | 3 | 4 | | IV-1 | Rab4a | 5867 | 21-May-15 |
| 17903 | 2 | 3 | 4 | | IV-1 | Rad9b | 144715 | 4-May-15 |
| 17916 | 2 | 3 | 4 | | IV-1 | Rai2 | 10742 | 4-May-15 |
| 17953 | 2 | 3 | 4 | | IV-1 | Rapgef4 | 11069 | 4-May-15 |
| 17954 | 2 | 3 | 4 | | IV-1 | Rapgef5 | 9771 | 4-May-15 |
| 17958 | 2 | 3 | 4 | | IV-1 | Rapsn | 5913 | 12-May-15 |
| 17963 | 2 | 3 | 4 | | IV-1 | Rarres2 | 5919 | 4-May-15 |
| 17974 | 2 | 3 | 4 | | IV-1 | Rasd2 | 23551 | 4-May-15 |
| 17975 | 2 | 3 | 4 | | IV-1 | Rasef | 158158 | 14-May-15 |
| 17977 | 2 | 3 | 4 | | IV-1 | Rasgef1b | 153020 | 4-May-15 |
| 17988 | 2 | 3 | 4 | | IV-1 | Rasl11a | 387496 | 4-May-15 |
| 17989 | 2 | 3 | 4 | | IV-1 | Rasl11b | 65997 | 4-May-15 |
| 17993 | 2 | 3 | 4 | | IV-1 | Rassf10 | 644943 | 4-May-15 |
| 17998 | 2 | 3 | 4 | | IV-1 | Rassf6 | 166824 | 4-May-15 |
| 17999 | 2 | 3 | 4 | | IV-1 | Rassf7 | 8045 | 4-May-15 |
| 18000 | 2 | 3 | 4 | | IV-1 | Rassf8 | 11228 | 4-May-15 |
| 18001 | 2 | 3 | 4 | | IV-1 | Rassf9 | 9182 | 4-May-15 |
| 18021 | 2 | 3 | 4 | | IV-1 | Rbfox3 | 146713 | 4-May-15 |
| 18031 | 2 | 3 | 4 | | IV-1 | Rbm14-rbm4 | 100526737 | 4-May-15 |
| 18037 | 2 | 3 | 4 | | IV-1 | Rbm20 | 282996 | 23-May-15 |
| 18048 | 2 | 3 | 4 | | IV-1 | Rbm38 | 55544 | 4-May-15 |
| 18050 | 2 | 3 | 4 | | IV-1 | Rbm3os | | |
| 18074 | 2 | 3 | 4 | | IV-1 | Rbp1 | 5947 | 7-Jun-15 |
| 18075 | 2 | 3 | 4 | | IV-1 | Rbp2 | 5948 | 7-Jun-15 |
| 18077 | 2 | 3 | 4 | | IV-1 | Rbp4 | 5950 | 7-Jun-15 |
| 18078 | 2 | 3 | 4 | | IV-1 | Rbp7 | 116362 | 30-Apr-15 |
| 18087 | 2 | 3 | 4 | | IV-1 | Rcan2 | 10231 | 4-May-15 |
| 18097 | 2 | 3 | 4 | | IV-1 | Rcn1 | 5954 | 13-Jun-15 |
| 18100 | 2 | 3 | 4 | | IV-1 | Rcor1 | 23186 | 7-Jun-15 |
| 18103 | 2 | 3 | 4 | | IV-1 | Rcsd1 | 92241 | 23-May-15 |
| 18107 | 2 | 3 | 4 | | IV-1 | Rdh1 | 5959 | 4-May-15 |
| 18109 | 2 | 3 | 4 | | IV-1 | Rdh11 | 51109 | 4-May-15 |
| 18110 | 2 | 3 | 4 | | IV-1 | Rdh12 | 145226 | 23-May-15 |
| 18113 | 2 | 3 | 4 | | IV-1 | Rdh16 | 8608 | 12-May-15 |
| 18114 | 2 | 3 | 4 | | IV-1 | Rdh18-ps | | |
| 18119 | 2 | 3 | 4 | | IV-1 | Rdh9 | | |
| 18122 | 2 | 3 | 4 | | IV-1 | Rec8 | 9985 | 4-May-15 |
| 18127 | 2 | 3 | 4 | | IV-1 | Redrum | | |
| 18128 | 2 | 3 | 4 | | IV-1 | Reep1 | 65055 | 4-May-15 |
| 18133 | 2 | 3 | 4 | | IV-1 | Reep6 | 92840 | 4-May-15 |
| 18136 | 2 | 3 | 4 | | IV-1 | Reg3a | 5068 | 12-May-15 |
| 18138 | 2 | 3 | 4 | | IV-1 | Reg3d | | |
| 18148 | 2 | 3 | 4 | | IV-1 | Rem1 | 28954 | 4-May-15 |
| 18152 | 2 | 3 | 4 | | IV-1 | Renbp | 5973 | 4-May-15 |
| 18159 | 2 | 3 | 4 | | IV-1 | Rerg | 85004 | 4-May-15 |
| 18161 | 2 | 3 | 4 | | IV-1 | Resp18 | 389075 | 4-May-15 |
| 18164 | 2 | 3 | 4 | | IV-1 | Retn | 56729 | 17-May-15 |
| 18165 | 2 | 3 | 4 | | IV-1 | Retnla | | |
| 18167 | 2 | 3 | 4 | | IV-1 | Retnlg | | |
| 18168 | 2 | 3 | 4 | | IV-1 | Retsat | 54884 | 12-May-15 |
| 18193 | 2 | 3 | 4 | | IV-1 | Rfx2 | 5990 | 4-May-15 |
| 18203 | 2 | 3 | 4 | | IV-1 | Rgag4 | 340526 | 4-May-15 |
| 18208 | 2 | 3 | 4 | | IV-1 | Rgma | 56963 | 12-May-15 |
| 18210 | 2 | 3 | 4 | | IV-1 | Rgn | 9104 | 4-May-15 |
| 18213 | 2 | 3 | 4 | | IV-1 | Rgs1 | 5996 | 4-May-15 |
| 18214 | 2 | 3 | 4 | | IV-1 | Rgs10 | 6001 | 4-May-15 |
| 18223 | 2 | 3 | 4 | | IV-1 | Rgs2 | 5997 | 7-Jun-15 |
| 18229 | 2 | 3 | 4 | | IV-1 | Rgs5 | 8490 | 12-May-15 |
| 18241 | 2 | 3 | 4 | | IV-1 | Rhbdf1 | 64285 | 4-May-15 |
| 18246 | 2 | 3 | 4 | | IV-1 | Rhbg | 57127 | 12-May-15 |
| 18250 | 2 | 3 | 4 | | IV-1 | Rhebl1 | 121268 | 12-May-15 |
| 18252 | 2 | 3 | 4 | | IV-1 | Rho | 6010 | 7-Jun-15 |
| 18259 | 2 | 3 | 4 | | IV-1 | Rhod | 29984 | 4-May-15 |
| 18267 | 2 | 3 | 4 | | IV-1 | Rhou | 58480 | 4-May-15 |
| 18268 | 2 | 3 | 4 | | IV-1 | Rhov | 171177 | 4-May-15 |
| 18301 | 2 | 3 | 4 | | IV-1 | Rhpn2 | 85415 | 4-May-15 |
| 18311 | 2 | 3 | 4 | | IV-1 | Rilp | 83547 | 7-Jun-15 |
| 18312 | 2 | 3 | 4 | | IV-1 | Rilpl1 | 353116 | 4-May-15 |
| 18321 | 2 | 3 | 4 | | IV-1 | Rims4 | 140730 | 4-May-15 |
| 18323 | 2 | 3 | 4 | | IV-1 | Rin2 | 54453 | 14-May-15 |
| 18334 | 2 | 3 | 4 | | IV-1 | Ripk4 | 54101 | 10-May-15 |
| 18352 | 2 | 3 | 4 | | IV-1 | Rmnd1 | 55005 | 4-May-15 |
| 18365 | 2 | 3 | 4 | | IV-1 | Rnase2b | | |
| 18366 | 2 | 3 | 4 | | IV-1 | Rnase4 | 6038 | 7-Jun-15 |
| 18378 | 2 | 3 | 4 | | IV-1 | Rnd2 | 8153 | 4-May-15 |
| 18392 | 2 | 3 | 4 | | IV-1 | Rnf125 | 54941 | 23-May-15 |
| 18406 | 2 | 3 | 4 | | IV-1 | Rnf145 | 153830 | 4-May-15 |
| 18412 | 2 | 3 | 4 | | IV-1 | Rnf152 | 220441 | 4-May-15 |
| 18413 | 2 | 3 | 4 | | IV-1 | Rnf157 | 114804 | 4-May-15 |
| 18422 | 2 | 3 | 4 | | IV-1 | Rnf181 | 51255 | 23-May-15 |
| 18423 | 2 | 3 | 4 | | IV-1 | Rnf182 | 221687 | 4-May-15 |
| 18433 | 2 | 3 | 4 | | IV-1 | Rnf208 | 727800 | 4-May-15 |
| 18437 | 2 | 3 | 4 | | IV-1 | Rnf217 | 154214 | 4-May-15 |
| 18443 | 2 | 3 | 4 | | IV-1 | Rnf24 | 11237 | 2-Jun-15 |
| 18486 | 2 | 3 | 4 | | IV-1 | Ropn1l | 83853 | 4-May-15 |
| 18491 | 2 | 3 | 4 | | IV-1 | Rorc | 6097 | 4-May-15 |
| 18492 | 2 | 3 | 4 | | IV-1 | Ros1 | 6098 | 12-May-15 |
| 18507 | 2 | 3 | 4 | | IV-1 | Rpf2 | 84154 | 4-May-15 |
| 18518 | 2 | 3 | 4 | | IV-1 | Rpl12 | 6136 | 12-May-15 |
| 18524 | 2 | 3 | 4 | | IV-1 | Rpl17 | 6139 | 7-Jun-15 |
| 18537 | 2 | 3 | 4 | | IV-1 | Rpl28 | 6158 | 3-May-15 |
| 18540 | 2 | 3 | 4 | | IV-1 | Rpl30 | 6156 | 4-May-15 |
| 18544 | 2 | 3 | 4 | | IV-1 | Rpl34 | 6164 | 4-May-15 |
| 18545 | 2 | 3 | 4 | | IV-1 | Rpl34-ps1 | | |
| 18547 | 2 | 3 | 4 | | IV-1 | Rpl35a | 6165 | 2-Jun-15 |
| 18548 | 2 | 3 | 4 | | IV-1 | Rpl36 | 25873 | 12-May-15 |
| 18549 | 2 | 3 | 4 | | IV-1 | Rpl36a | 6173 | 7-Jun-15 |
| 18551 | 2 | 3 | 4 | | IV-1 | Rpl37 | 6167 | 4-May-15 |
| 18552 | 2 | 3 | 4 | | IV-1 | Rpl37a | 6168 | 4-May-15 |
| 18568 | 2 | 3 | 4 | | IV-1 | Rplp2 | 6181 | 12-May-15 |
| 18573 | 2 | 3 | 4 | | IV-1 | Rpp21 | 79897 | 4-May-15 |
| 18576 | 2 | 3 | 4 | | IV-1 | Rpp30 | 10556 | 4-May-15 |
| 18580 | 2 | 3 | 4 | | IV-1 | Rprd1a | 55197 | 12-May-15 |
| 18587 | 2 | 3 | 4 | | IV-1 | Rprml | 388394 | 4-May-15 |
| 18588 | 2 | 3 | 4 | | IV-1 | Rps10 | 6204 | 22-May-15 |
| 18589 | 2 | 3 | 4 | | IV-1 | Rps11 | 6205 | 4-May-15 |
| 18590 | 2 | 3 | 4 | | IV-1 | Rps12 | 6206 | 7-Jun-15 |
| 18598 | 2 | 3 | 4 | | IV-1 | Rps17 | 6218 | 23-May-15 |
| 18599 | 2 | 3 | 4 | | IV-1 | Rps18 | 6222 | 7-Jun-15 |
| 18608 | 2 | 3 | 4 | | IV-1 | Rps25 | 6230 | 4-May-15 |
| 18609 | 2 | 3 | 4 | | IV-1 | Rps26 | 6231 | 7-Jun-15 |
| 18610 | 2 | 3 | 4 | | IV-1 | Rps27 | 6232 | 17-May-15 |
| 18614 | 2 | 3 | 4 | | IV-1 | Rps28 | 6234 | 28-May-15 |
| 18615 | 2 | 3 | 4 | | IV-1 | Rps29 | 6235 | 4-May-15 |
| 18633 | 2 | 3 | 4 | | IV-1 | Rps8 | 6202 | 4-May-15 |
| 18644 | 2 | 3 | 4 | | IV-1 | Rrad | 6236 | 4-May-15 |
| 18646 | 2 | 3 | 4 | | IV-1 | Rragb | 10325 | 4-May-15 |
| 18648 | 2 | 3 | 4 | | IV-1 | Rragd | 58528 | 4-May-15 |
| 18655 | 2 | 3 | 4 | | IV-1 | Rrm2 | 6241 | 4-May-15 |
| 18670 | 2 | 3 | 4 | | IV-1 | Rsad2 | 91543 | 4-May-15 |
| 18685 | 2 | 3 | 4 | | IV-1 | Rsph9 | 221421 | 23-May-15 |
| 18711 | 2 | 3 | 4 | | IV-1 | Rtn4rl1 | 146760 | 4-May-15 |
| 18712 | 2 | 3 | 4 | | IV-1 | Rtn4rl2 | 349667 | 4-May-15 |
| 18722 | 2 | 3 | 4 | | IV-1 | Rufy4 | 285180 | 4-May-15 |
| 18723 | 2 | 3 | 4 | | IV-1 | Rundc1 | 146923 | 12-May-15 |
| 18726 | 2 | 3 | 4 | | IV-1 | Runx1 | 861 | 21-May-15 |
| 18729 | 2 | 3 | 4 | | IV-1 | Runx3 | 864 | 21-May-15 |
| 18731 | 2 | 3 | 4 | | IV-1 | Rusc2 | 9853 | 4-May-15 |
| 18745 | 2 | 3 | 4 | | IV-1 | Rxrg | 6258 | 4-May-15 |
| 18752 | 2 | 3 | 4 | | IV-1 | S100a10 | 6281 | 12-May-15 |
| 18755 | 2 | 3 | 4 | | IV-1 | S100a14 | 57402 | 7-Jun-15 |
| 18759 | 2 | 3 | 4 | | IV-1 | S100a4 | 6275 | 17-May-15 |
| 18767 | 2 | 3 | 4 | | IV-1 | S100pbp | 64766 | 4-May-15 |
| 18785 | 2 | 3 | 4 | | IV-1 | Sag | 6295 | 7-Jun-15 |
| 18786 | 2 | 3 | 4 | | IV-1 | Sall1 | 6299 | 23-May-15 |
| 18791 | 2 | 3 | 4 | | IV-1 | Samd10 | 140700 | 4-May-15 |
| 18792 | 2 | 3 | 4 | | IV-1 | Samd11 | | |
| 18794 | 2 | 3 | 4 | | IV-1 | Samd14 | 201191 | 4-May-15 |
| 18797 | 2 | 3 | 4 | | IV-1 | Samd4 | 23034 | 4-May-15 |
| 18799 | 2 | 3 | 4 | | IV-1 | Samd5 | 389432 | 12-May-15 |
| 18811 | 2 | 3 | 4 | | IV-1 | Sap25 | 100316904 | 4-May-15 |
| 18815 | 2 | 3 | 4 | | IV-1 | Sapcd1 | 401251 | 4-May-15 |
| 18818 | 2 | 3 | 4 | | IV-1 | Sar1b | 51128 | 4-May-15 |
| 18831 | 2 | 3 | 4 | | IV-1 | Satb1 | 6304 | 26-May-15 |
| 18841 | 2 | 3 | 4 | | IV-1 | Sbk3 | 100130827 | 4-May-15 |
| 18846 | 2 | 3 | 4 | | IV-1 | Sbsn | 374897 | 4-May-15 |
| 18867 | 2 | 3 | 4 | | IV-1 | Scarf2 | 91179 | 21-May-15 |
| 18870 | 2 | 3 | 4 | | IV-1 | Scarna13 | 677768 | 4-May-15 |
| 18875 | 2 | 3 | 4 | | IV-1 | Scarna6 | 677772 | 4-May-15 |
| 18879 | 2 | 3 | 4 | | IV-1 | Scd1 | 6319 | 4-May-15 |
| 18883 | 2 | 3 | 4 | | IV-1 | Scel | 8796 | 4-May-15 |
| 18888 | 2 | 3 | 4 | | IV-1 | Scg5 | 6447 | 12-May-15 |
| 18899 | 2 | 3 | 4 | | IV-1 | Scgb1c1 | 147199 | 4-May-15 |
| 18907 | 2 | 3 | 4 | | IV-1 | Scgb2b24 | | |
| 18908 | 2 | 3 | 4 | | IV-1 | Scgb2b26 | | |
| 18915 | 2 | 3 | 4 | | IV-1 | Schip1 | 29970 | 4-May-15 |

Fig.22 - 35

| | | | | | | | | |
|---|---|---|---|---|---|---|---|---|
| 18916 | 2 | 3 | 4 | | IV-1 | Scimp | 388325 | 4-May-15 |
| 18917 | 2 | 3 | 4 | | IV-1 | Scin | 85477 | 4-May-15 |
| 18922 | 2 | 3 | 4 | | IV-1 | Scml4 | 256380 | 12-May-15 |
| 18925 | 2 | 3 | 4 | | IV-1 | Scn1a | 6323 | 22-May-15 |
| 18926 | 2 | 3 | 4 | | IV-1 | Scn1b | 6324 | 23-May-15 |
| 18928 | 2 | 3 | 4 | | IV-1 | Scn2b | 6327 | 12-May-15 |
| 18931 | 2 | 3 | 4 | | IV-1 | Scn4a | 6329 | 22-May-15 |
| 18938 | 2 | 3 | 4 | | IV-1 | Scnn1a | 6337 | 12-May-15 |
| 18939 | 2 | 3 | 4 | | IV-1 | Scnn1b | 6338 | 12-May-15 |
| 18940 | 2 | 3 | 4 | | IV-1 | Scnn1g | 6340 | 17-May-15 |
| 18956 | 2 | 3 | 4 | | IV-1 | Sctr | 6344 | 4-May-15 |
| 18957 | 2 | 3 | 4 | | IV-1 | Scube1 | 80274 | 4-May-15 |
| 18965 | 2 | 3 | 4 | | IV-1 | Sdc1 | 6382 | 17-May-15 |
| 18968 | 2 | 3 | 4 | | IV-1 | Sdc4 | 6385 | 3-May-15 |
| 18970 | 2 | 3 | 4 | | IV-1 | Sdcbp2 | 27111 | 4-May-15 |
| 18975 | 2 | 3 | 4 | | IV-1 | Sdf2l1 | 23753 | 4-May-15 |
| 18987 | 2 | 3 | 4 | | IV-1 | Sdr16c6 | 442388 | 4-May-15 |
| 18992 | 2 | 3 | 4 | | IV-1 | Sdsl | 113675 | 12-May-15 |
| 18996 | 2 | 3 | 4 | | IV-1 | Sec11c | 90701 | 14-May-15 |
| 18999 | 2 | 3 | 4 | | IV-1 | Sec14l2 | 23541 | 12-May-15 |
| 19000 | 2 | 3 | 4 | | IV-1 | Sec14l3 | 266629 | 4-May-15 |
| 19014 | 2 | 3 | 4 | | IV-1 | Sec24d | 9871 | 17-May-15 |
| 19030 | 2 | 3 | 4 | | IV-1 | Sel1l3 | 23231 | 12-May-15 |
| 19031 | 2 | 3 | 4 | | IV-1 | Sele | 6401 | 17-May-15 |
| 19032 | 2 | 3 | 4 | | IV-1 | Selenbp1 | 8991 | 4-May-15 |
| 19035 | 2 | 3 | 4 | | IV-1 | Sell | 6402 | 17-May-15 |
| 19043 | 2 | 3 | 4 | | IV-1 | Sema3c | 10512 | 31-May-15 |
| 19046 | 2 | 3 | 4 | | IV-1 | Sema3f | 6405 | 4-May-15 |
| 19053 | 2 | 3 | 4 | | IV-1 | Sema4g | 57715 | 4-May-15 |
| 19057 | 2 | 3 | 4 | | IV-1 | Sema6b | 10501 | 4-May-15 |
| 19071 | 2 | 3 | 4 | | IV-1 | Sepp1 | 6414 | 3-May-15 |
| 19072 | 2 | 3 | 4 | | IV-1 | Sepsecs | 51091 | 4-May-15 |
| 19111 | 2 | 3 | 4 | | IV-1 | Serpina3b | | |
| 19112 | 2 | 3 | 4 | | IV-1 | Serpina3c | | |
| 19113 | 2 | 3 | 4 | | IV-1 | Serpina3f | | |
| 19114 | 2 | 3 | 4 | | IV-1 | Serpina3g | | |
| 19117 | 2 | 3 | 4 | | IV-1 | Serpina3j | | |
| 19119 | 2 | 3 | 4 | | IV-1 | Serpina3m | | |
| 19120 | 2 | 3 | 4 | | IV-1 | Serpina3n | | |
| 19124 | 2 | 3 | 4 | | IV-1 | Serpina7 | 6906 | 4-May-15 |
| 19127 | 2 | 3 | 4 | | IV-1 | Serpinb11 | 89778 | 12-May-15 |
| 19128 | 2 | 3 | 4 | | IV-1 | Serpinb12 | 89777 | 20-May-15 |
| 19130 | 2 | 3 | 4 | | IV-1 | Serpinb1a | | |
| 19133 | 2 | 3 | 4 | | IV-1 | Serpinb2 | 5055 | 17-May-15 |
| 19136 | 2 | 3 | 4 | | IV-1 | Serpinb3c | | |
| 19139 | 2 | 3 | 4 | | IV-1 | Serpinb6a | | |
| 19154 | 2 | 3 | 4 | | IV-1 | Serpind1 | 3053 | 4-May-15 |
| 19155 | 2 | 3 | 4 | | IV-1 | Serpine1 | 5054 | 24-May-15 |
| 19156 | 2 | 3 | 4 | | IV-1 | Serpine2 | 5270 | 12-May-15 |
| 19157 | 2 | 3 | 4 | | IV-1 | Serpine3 | 647174 | 4-May-15 |
| 19163 | 2 | 3 | 4 | | IV-1 | Serpini2 | 5276 | 4-May-15 |
| 19169 | 2 | 3 | 4 | | IV-1 | Sesn1 | 27244 | 4-May-15 |
| 19171 | 2 | 3 | 4 | | IV-1 | Sesn3 | 143686 | 4-May-15 |
| 19174 | 2 | 3 | 4 | | IV-1 | Setbp1 | 26040 | 17-May-15 |
| 19179 | 2 | 3 | 4 | | IV-1 | Setd4 | 54093 | 4-May-15 |
| 19204 | 2 | 3 | 4 | | IV-1 | Sfn | 2810 | 7-Jun-15 |
| 19209 | 2 | 3 | 4 | | IV-1 | Sfrp4 | 6424 | 17-May-15 |
| 19218 | 2 | 3 | 4 | | IV-1 | Sftpc | 6440 | 23-May-15 |
| 19226 | 2 | 3 | 4 | | IV-1 | Sgcb | 6443 | 23-May-15 |
| 19228 | 2 | 3 | 4 | | IV-1 | Sgce | 8910 | 23-May-15 |
| 19232 | 2 | 3 | 4 | | IV-1 | Sgk1 | 6446 | 21-May-15 |
| 19233 | 2 | 3 | 4 | | IV-1 | Sgk2 | 10110 | 7-Jun-15 |
| 19236 | 2 | 3 | 4 | | IV-1 | Sgms2 | 166929 | 4-May-15 |
| 19240 | 2 | 3 | 4 | | IV-1 | Sgpp1 | 81537 | 4-May-15 |
| 19241 | 2 | 3 | 4 | | IV-1 | Sgpp2 | 130367 | 4-May-15 |
| 19249 | 2 | 3 | 4 | | IV-1 | Sh2b2 | 10603 | 21-May-15 |
| 19256 | 2 | 3 | 4 | | IV-1 | Sh2d4a | 63898 | 4-May-15 |
| 19258 | 2 | 3 | 4 | | IV-1 | Sh2d5 | 400745 | 4-May-15 |
| 19259 | 2 | 3 | 4 | | IV-1 | Sh2d7 | 646892 | 4-May-15 |
| 19260 | 2 | 3 | 4 | | IV-1 | Sh3bgr | 6450 | 7-Jun-15 |
| 19273 | 2 | 3 | 4 | | IV-1 | Sh3gl3 | 6457 | 4-May-15 |
| 19280 | 2 | 3 | 4 | | IV-1 | Sh3rf2 | 153769 | 21-May-15 |
| 19282 | 2 | 3 | 4 | | IV-1 | Sh3tc1 | 54436 | 4-May-15 |
| 19283 | 2 | 3 | 4 | | IV-1 | Sh3tc2 | 79628 | 23-May-15 |
| 19284 | 2 | 3 | 4 | | IV-1 | Sh3yl1 | 26751 | 12-May-15 |
| 19286 | 2 | 3 | 4 | | IV-1 | Shank2 | 22941 | 23-May-15 |
| 19289 | 2 | 3 | 4 | | IV-1 | Shb | 6461 | 4-May-15 |
| 19299 | 2 | 3 | 4 | | IV-1 | Shf | 90525 | 21-May-15 |
| 19301 | 2 | 3 | 4 | | IV-1 | Shh | 6469 | 24-May-15 |
| 19302 | 2 | 3 | 4 | | IV-1 | Shisa2 | 387914 | 4-May-15 |
| 19304 | 2 | 3 | 4 | | IV-1 | Shisa4 | 149345 | 4-May-15 |
| 19316 | 2 | 3 | 4 | | IV-1 | Shq1 | 55164 | 3-May-15 |
| 19317 | 2 | 3 | 4 | | IV-1 | Shroom1 | 134549 | 12-May-15 |
| 19319 | 2 | 3 | 4 | | IV-1 | Shroom3 | 57619 | 21-May-15 |
| 19328 | 2 | 3 | 4 | | IV-1 | Sigirr | 59307 | 24-May-15 |
| 19329 | 2 | 3 | 4 | | IV-1 | Siglec1 | 6614 | 4-May-15 |
| 19330 | 2 | 3 | 4 | | IV-1 | Siglec15 | 284266 | 4-May-15 |
| 19332 | 2 | 3 | 4 | | IV-1 | Siglece | | |
| 19336 | 2 | 3 | 4 | | IV-1 | Sik1 | 150094 | 23-May-15 |
| 19337 | 2 | 3 | 4 | | IV-1 | Sik2 | 23235 | 4-May-15 |
| 19350 | 2 | 3 | 4 | | IV-1 | Sirpa | 140885 | 17-May-15 |
| 19361 | 2 | 3 | 4 | | IV-1 | Sit1 | 27240 | 7-Jun-15 |
| 19363 | 2 | 3 | 4 | | IV-1 | Six1 | 6495 | 31-May-15 |
| 19368 | 2 | 3 | 4 | | IV-1 | Six5 | 147912 | 23-May-15 |
| 19373 | 2 | 3 | 4 | | IV-1 | Skap1 | 8631 | 4-May-15 |
| 19387 | 2 | 3 | 4 | | IV-1 | Skint8 | | |
| 19395 | 2 | 3 | 4 | | IV-1 | Sla | 6503 | 7-Jun-15 |

| | | | | | | | | |
|---|---|---|---|---|---|---|---|---|
| 19402 | 2 | 3 | 4 | | IV-1 | Slamf7 | 57823 | 4-May-15 |
| 19406 | 2 | 3 | 4 | | IV-1 | Slc10a1 | 6554 | 17-May-15 |
| 19407 | 2 | 3 | 4 | | IV-1 | Slc10a2 | 6555 | 12-May-15 |
| 19411 | 2 | 3 | 4 | | IV-1 | Slc10a5 | 347051 | 4-May-15 |
| 19412 | 2 | 3 | 4 | | IV-1 | Slc10a6 | 345274 | 4-May-15 |
| 19414 | 2 | 3 | 4 | | IV-1 | Slc11a1 | 6556 | 12-May-15 |
| 19425 | 2 | 3 | 4 | | IV-1 | Slc13a1 | 6561 | 4-May-15 |
| 19427 | 2 | 3 | 4 | | IV-1 | Slc13a2os | | |
| 19432 | 2 | 3 | 4 | | IV-1 | Slc14a2 | 8170 | 3-May-15 |
| 19433 | 2 | 3 | 4 | | IV-1 | Slc15a1 | 6564 | 12-May-15 |
| 19434 | 2 | 3 | 4 | | IV-1 | Slc15a2 | 6565 | 4-May-15 |
| 19435 | 2 | 3 | 4 | | IV-1 | Slc15a3 | 51296 | 4-May-15 |
| 19439 | 2 | 3 | 4 | | IV-1 | Slc16a10 | 117247 | 4-May-15 |
| 19440 | 2 | 3 | 4 | | IV-1 | Slc16a11 | 162515 | 4-May-15 |
| 19441 | 2 | 3 | 4 | | IV-1 | Slc16a12 | 387700 | 4-May-15 |
| 19442 | 2 | 3 | 4 | | IV-1 | Slc16a13 | 201232 | 4-May-15 |
| 19444 | 2 | 3 | 4 | | IV-1 | Slc16a2 | 6567 | 23-May-15 |
| 19447 | 2 | 3 | 4 | | IV-1 | Slc16a5 | 9121 | 4-May-15 |
| 19448 | 2 | 3 | 4 | | IV-1 | Slc16a6 | 9120 | 20-May-15 |
| 19449 | 2 | 3 | 4 | | IV-1 | Slc16a7 | 9194 | 4-May-15 |
| 19452 | 2 | 3 | 4 | | IV-1 | Slc17a1 | 6568 | 12-May-15 |
| 19455 | 2 | 3 | 4 | | IV-1 | Slc17a4 | 10050 | 4-May-15 |
| 19465 | 2 | 3 | 4 | | IV-1 | Slc19a1 | 6573 | 12-May-15 |
| 19467 | 2 | 3 | 4 | | IV-1 | Slc19a3 | 80704 | 23-May-15 |
| 19470 | 2 | 3 | 4 | | IV-1 | Slc1a3 | 6507 | 23-May-15 |
| 19471 | 2 | 3 | 4 | | IV-1 | Slc1a4 | 6509 | 28-May-15 |
| 19472 | 2 | 3 | 4 | | IV-1 | Slc1a5 | 6510 | 4-May-15 |
| 19475 | 2 | 3 | 4 | | IV-1 | Slc20a1 | 6574 | 4-May-15 |
| 19484 | 2 | 3 | 4 | | IV-1 | Slc22a17 | 51310 | 1-Jun-15 |
| 19498 | 2 | 3 | 4 | | IV-1 | Slc22a4 | 6583 | 12-May-15 |
| 19502 | 2 | 3 | 4 | | IV-1 | Slc22a8 | 9376 | 12-May-15 |
| 19505 | 2 | 3 | 4 | | IV-1 | Slc23a3 | 151295 | 4-May-15 |
| 19509 | 2 | 3 | 4 | | IV-1 | Slc24a4 | 123041 | 4-May-15 |
| 19510 | 2 | 3 | 4 | | IV-1 | Slc24a5 | 283652 | 12-May-15 |
| 19514 | 2 | 3 | 4 | | IV-1 | Slc25a12 | 8604 | 4-May-15 |
| 19515 | 2 | 3 | 4 | | IV-1 | Slc25a13 | 10165 | 23-May-15 |
| 19524 | 2 | 3 | 4 | | IV-1 | Slc25a21 | 89874 | 4-May-15 |
| 19527 | 2 | 3 | 4 | | IV-1 | Slc25a24 | 29957 | 4-May-15 |
| 19528 | 2 | 3 | 4 | | IV-1 | Slc25a25 | 114789 | 4-May-15 |
| 19532 | 2 | 3 | 4 | | IV-1 | Slc25a29 | 123096 | 4-May-15 |
| 19534 | 2 | 3 | 4 | | IV-1 | Slc25a30 | 253512 | 4-May-15 |
| 19537 | 2 | 3 | 4 | | IV-1 | Slc25a33 | 84275 | 4-May-15 |
| 19541 | 2 | 3 | 4 | | IV-1 | Slc25a37 | 51312 | 4-May-15 |
| 19544 | 2 | 3 | 4 | | IV-1 | Slc25a4 | 291 | 4-May-15 |
| 19553 | 2 | 3 | 4 | | IV-1 | Slc25a48 | 153328 | 4-May-15 |
| 19560 | 2 | 3 | 4 | | IV-1 | Slc26a11 | 284129 | 4-May-15 |
| 19561 | 2 | 3 | 4 | | IV-1 | Slc26a2 | 1836 | 23-May-15 |
| 19568 | 2 | 3 | 4 | | IV-1 | Slc26a9 | 115019 | 4-May-15 |
| 19569 | 2 | 3 | 4 | | IV-1 | Slc27a1 | 376497 | 4-May-15 |
| 19570 | 2 | 3 | 4 | | IV-1 | Slc27a2 | 11001 | 4-May-15 |
| 19576 | 2 | 3 | 4 | | IV-1 | Slc28a2 | 9153 | 4-May-15 |
| 19577 | 2 | 3 | 4 | | IV-1 | Slc28a3 | 64078 | 4-May-15 |
| 19584 | 2 | 3 | 4 | | IV-1 | Slc2a12 | 154091 | 17-May-15 |
| 19588 | 2 | 3 | 4 | | IV-1 | Slc2a4 | 6517 | 17-May-15 |
| 19590 | 2 | 3 | 4 | | IV-1 | Slc2a5 | 6518 | 12-May-15 |
| 19592 | 2 | 3 | 4 | | IV-1 | Slc2a7 | 155184 | 4-May-15 |
| 19597 | 2 | 3 | 4 | | IV-1 | Slc30a2 | 7780 | 4-May-15 |
| 19598 | 2 | 3 | 4 | | IV-1 | Slc30a3 | 7781 | 4-May-15 |
| 19628 | 2 | 3 | 4 | | IV-1 | Slc35e3 | 55508 | 4-May-15 |
| 19629 | 2 | 3 | 4 | | IV-1 | Slc35e4 | 339665 | 4-May-15 |
| 19636 | 2 | 3 | 4 | | IV-1 | Slc35g1 | 159371 | 4-May-15 |
| 19641 | 2 | 3 | 4 | | IV-1 | Slc36a2 | 153201 | 4-May-15 |
| 19643 | 2 | 3 | 4 | | IV-1 | Slc36a4 | 120103 | 28-May-15 |
| 19644 | 2 | 3 | 4 | | IV-1 | Slc37a1 | 54020 | 4-May-15 |
| 19645 | 2 | 3 | 4 | | IV-1 | Slc37a2 | 219855 | 12-May-15 |
| 19653 | 2 | 3 | 4 | | IV-1 | Slc38a4 | 55089 | 4-May-15 |
| 19669 | 2 | 3 | 4 | | IV-1 | Slc39a6 | 25800 | 12-May-15 |
| 19671 | 2 | 3 | 4 | | IV-1 | Slc39a8 | 64116 | 4-May-15 |
| 19677 | 2 | 3 | 4 | | IV-1 | Slc41a2 | 84102 | 4-May-15 |
| 19678 | 2 | 3 | 4 | | IV-1 | Slc41a3 | 54946 | 4-May-15 |
| 19679 | 2 | 3 | 4 | | IV-1 | Slc43a1 | 8501 | 4-May-15 |
| 19685 | 2 | 3 | 4 | | IV-1 | Slc44a4 | 80736 | 4-May-15 |
| 19689 | 2 | 3 | 4 | | IV-1 | Slc45a3 | 85414 | 4-May-15 |
| 19691 | 2 | 3 | 4 | | IV-1 | Slc46a1 | 113235 | 23-May-15 |
| 19692 | 2 | 3 | 4 | | IV-1 | Slc46a2 | 57864 | 4-May-15 |
| 19694 | 2 | 3 | 4 | | IV-1 | Slc47a1 | 55244 | 24-May-15 |
| 19699 | 2 | 3 | 4 | | IV-1 | Slc4a11 | 83959 | 23-May-15 |
| 19703 | 2 | 3 | 4 | | IV-1 | Slc4a4 | 8671 | 17-May-15 |
| 19709 | 2 | 3 | 4 | | IV-1 | Slc51a | 200931 | 4-May-15 |
| 19712 | 2 | 3 | 4 | | IV-1 | Slc52a3 | 113278 | 4-May-15 |
| 19713 | 2 | 3 | 4 | | IV-1 | Slc5a1 | 6523 | 12-May-15 |
| 19716 | 2 | 3 | 4 | | IV-1 | Slc5a12 | 159963 | 4-May-15 |
| 19718 | 2 | 3 | 4 | | IV-1 | Slc5a3 | 6526 | 12-May-15 |
| 19719 | 2 | 3 | 4 | | IV-1 | Slc5a4a | | |
| 19722 | 2 | 3 | 4 | | IV-1 | Slc5a6 | 8884 | 12-May-15 |
| 19724 | 2 | 3 | 4 | | IV-1 | Slc5a8 | 160728 | 4-May-15 |
| 19731 | 2 | 3 | 4 | | IV-1 | Slc6a15 | 55117 | 14-May-15 |
| 19732 | 2 | 3 | 4 | | IV-1 | Slc6a17 | 388662 | 17-May-15 |
| 19733 | 2 | 3 | 4 | | IV-1 | Slc6a18 | 348932 | 4-May-15 |
| 19734 | 2 | 3 | 4 | | IV-1 | Slc6a19 | 340024 | 4-May-15 |
| 19736 | 2 | 3 | 4 | | IV-1 | Slc6a2 | 6530 | 12-May-15 |
| 19740 | 2 | 3 | 4 | | IV-1 | Slc6a4 | 6532 | 17-May-15 |
| 19742 | 2 | 3 | 4 | | IV-1 | Slc6a6 | 6533 | 12-May-15 |
| 19744 | 2 | 3 | 4 | | IV-1 | Slc6a8 | 6535 | 23-May-15 |
| 19745 | 2 | 3 | 4 | | IV-1 | Slc6a9 | 6536 | 4-May-15 |
| 19746 | 2 | 3 | 4 | | IV-1 | Slc7a1 | 6541 | 4-May-15 |
| 19747 | 2 | 3 | 4 | | IV-1 | Slc7a10 | 56301 | 12-May-15 |

Fig.22 - 36

| | | | | | | | | |
|---|---|---|---|---|---|---|---|---|
| 19748 | 2 | 3 | 4 | | IV-1 | Slc7a11 | 23657 | 12-May-15 |
| 19753 | 2 | 3 | 4 | | IV-1 | Slc7a2 | 6542 | 4-May-15 |
| 19755 | 2 | 3 | 4 | | IV-1 | Slc7a4 | 6545 | 4-May-15 |
| 19764 | 2 | 3 | 4 | | IV-1 | Slc8a3 | 6547 | 12-May-15 |
| 19767 | 2 | 3 | 4 | | IV-1 | Slc9a2 | 6549 | 12-May-15 |
| 19768 | 2 | 3 | 4 | | IV-1 | Slc9a3 | 6550 | 17-May-15 |
| 19771 | 2 | 3 | 4 | | IV-1 | Slc9a4 | 389015 | 4-May-15 |
| 19776 | 2 | 3 | 4 | | IV-1 | Slc9a9 | 285195 | 4-May-15 |
| 19778 | 2 | 3 | 4 | | IV-1 | Slc9b2 | 133308 | 4-May-15 |
| 19782 | 2 | 3 | 4 | | IV-1 | Slco1a5 | | |
| 19788 | 2 | 3 | 4 | | IV-1 | Slco3a1 | 28232 | 12-May-15 |
| 19789 | 2 | 3 | 4 | | IV-1 | Slco4a1 | 28231 | 4-May-15 |
| 19791 | 2 | 3 | 4 | | IV-1 | Slco5a1 | 81796 | 4-May-15 |
| 19797 | 2 | 3 | 4 | | IV-1 | Slfn14 | 342618 | 4-May-15 |
| 19798 | 2 | 3 | 4 | | IV-1 | Slfn2 | | |
| 19818 | 2 | 3 | 4 | | IV-1 | Slmo1 | 10650 | 12-May-15 |
| 19836 | 2 | 3 | 4 | | IV-1 | Smad6 | 4091 | 12-May-15 |
| 19854 | 2 | 3 | 4 | | IV-1 | Smarcd3 | 6604 | 4-May-15 |
| 19881 | 2 | 3 | 4 | | IV-1 | Smim1 | 388588 | 12-May-15 |
| 19890 | 2 | 3 | 4 | | IV-1 | Smim22 | 440335 | 12-May-15 |
| 19892 | 2 | 3 | 4 | | IV-1 | Smim24 | 284422 | 4-May-15 |
| 19893 | 2 | 3 | 4 | | IV-1 | Smim3 | 85027 | 4-May-15 |
| 19895 | 2 | 3 | 4 | | IV-1 | Smim5 | 643008 | 4-May-15 |
| 19914 | 2 | 3 | 4 | | IV-1 | Smpd3 | 55512 | 12-May-15 |
| 19918 | 2 | 3 | 4 | | IV-1 | Smpdl3b | 27293 | 4-May-15 |
| 19936 | 2 | 3 | 4 | | IV-1 | Snai2 | 6591 | 1-Jun-15 |
| 19937 | 2 | 3 | 4 | | IV-1 | Snai3 | 333929 | 4-May-15 |
| 19942 | 2 | 3 | 4 | | IV-1 | Snap91 | 9892 | 4-May-15 |
| 19945 | 2 | 3 | 4 | | IV-1 | Snapc3 | 6619 | 4-May-15 |
| 19949 | 2 | 3 | 4 | | IV-1 | Snca | 6622 | 24-May-15 |
| 19950 | 2 | 3 | 4 | | IV-1 | Sncaip | 9627 | 31-May-15 |
| 19952 | 2 | 3 | 4 | | IV-1 | Sncg | 6623 | 4-May-15 |
| 19957 | 2 | 3 | 4 | | IV-1 | Snhg10 | 283596 | 12-May-15 |
| 19958 | 2 | 3 | 4 | | IV-1 | Snhg11 | 128439 | 4-May-15 |
| 19959 | 2 | 3 | 4 | | IV-1 | Snhg12 | 85028 | 12-May-15 |
| 19961 | 2 | 3 | 4 | | IV-1 | Snhg3 | 8420 | 12-May-15 |
| 19963 | 2 | 3 | 4 | | IV-1 | Snhg5 | 387066 | 12-May-15 |
| 19964 | 2 | 3 | 4 | | IV-1 | Snhg6 | 641638 | 12-May-15 |
| 19967 | 2 | 3 | 4 | | IV-1 | Snhg9 | 735301 | 12-May-15 |
| 19969 | 2 | 3 | 4 | | IV-1 | Snn | 8303 | 4-May-15 |
| 19976 | 2 | 3 | 4 | | IV-1 | Snora23 | 677808 | 4-May-15 |
| 20001 | 2 | 3 | 4 | | IV-1 | Snora74a | 26821 | 4-May-15 |
| 20023 | 2 | 3 | 4 | | IV-1 | Snord17 | 692086 | 4-May-15 |
| 20080 | 2 | 3 | 4 | | IV-1 | Snph | 9751 | 4-May-15 |
| 20085 | 2 | 3 | 4 | | IV-1 | Snrnp35 | 11066 | 4-May-15 |
| 20090 | 2 | 3 | 4 | | IV-1 | Snrpa1 | 6627 | 2-Jun-15 |
| 20098 | 2 | 3 | 4 | | IV-1 | Snrpf | 6636 | 28-May-15 |
| 20099 | 2 | 3 | 4 | | IV-1 | Snrpg | 6637 | 4-May-15 |
| 20101 | 2 | 3 | 4 | | IV-1 | Snta1 | 6640 | 23-May-15 |
| 20102 | 2 | 3 | 4 | | IV-1 | Sntb1 | 6641 | 4-May-15 |
| 20103 | 2 | 3 | 4 | | IV-1 | Sntb2 | 6645 | 4-May-15 |
| 20105 | 2 | 3 | 4 | | IV-1 | Sntg2 | 54221 | 4-May-15 |
| 20111 | 2 | 3 | 4 | | IV-1 | Snx10 | 29887 | 4-May-15 |
| 20124 | 2 | 3 | 4 | | IV-1 | Snx22 | 79856 | 4-May-15 |
| 20141 | 2 | 3 | 4 | | IV-1 | Soat2 | 8435 | 12-May-15 |
| 20142 | 2 | 3 | 4 | | IV-1 | Sobp | 55084 | 4-May-15 |
| 20143 | 2 | 3 | 4 | | IV-1 | Socs1 | 8651 | 4-May-15 |
| 20144 | 2 | 3 | 4 | | IV-1 | Socs2 | 8835 | 14-May-15 |
| 20145 | 2 | 3 | 4 | | IV-1 | Socs3 | 9021 | 23-May-15 |
| 20146 | 2 | 3 | 4 | | IV-1 | Socs4 | 122809 | 7-Jun-15 |
| 20155 | 2 | 3 | 4 | | IV-1 | Sohlh1 | 402381 | 4-May-15 |
| 20163 | 2 | 3 | 4 | | IV-1 | Sorcs2 | 57537 | 4-May-15 |
| 20172 | 2 | 3 | 4 | | IV-1 | Sowaha | 134548 | 4-May-15 |
| 20173 | 2 | 3 | 4 | | IV-1 | Sowahb | 345079 | 4-May-15 |
| 20179 | 2 | 3 | 4 | | IV-1 | Sox12 | 6666 | 4-May-15 |
| 20182 | 2 | 3 | 4 | | IV-1 | Sox15 | 6665 | 12-May-15 |
| 20183 | 2 | 3 | 4 | | IV-1 | Sox17 | 64321 | 31-May-15 |
| 20196 | 2 | 3 | 4 | | IV-1 | Sox9 | 6662 | 23-May-15 |
| 20203 | 2 | 3 | 4 | | IV-1 | Sp3os | | |
| 20210 | 2 | 3 | 4 | | IV-1 | Spa17 | 53340 | 4-May-15 |
| 20236 | 2 | 3 | 4 | | IV-1 | Spata18 | 132671 | 4-May-15 |
| 20242 | 2 | 3 | 4 | | IV-1 | Spata24 | 202051 | 4-May-15 |
| 20252 | 2 | 3 | 4 | | IV-1 | Spata33 | 124045 | 4-May-15 |
| 20265 | 2 | 3 | 4 | | IV-1 | Spc24 | 147841 | 4-May-15 |
| 20266 | 2 | 3 | 4 | | IV-1 | Spc25 | 57405 | 4-May-15 |
| 20274 | 2 | 3 | 4 | | IV-1 | Specc1 | 92521 | 4-May-15 |
| 20290 | 2 | 3 | 4 | | IV-1 | Spef1 | 25876 | 4-May-15 |
| 20292 | 2 | 3 | 4 | | IV-1 | Speg | 10290 | 12-May-15 |
| 20293 | 2 | 3 | 4 | | IV-1 | Spem1 | 374768 | 4-May-15 |
| 20295 | 2 | 3 | 4 | | IV-1 | Spert | 220082 | 4-May-15 |
| 20301 | 2 | 3 | 4 | | IV-1 | Sphk1 | 8877 | 12-May-15 |
| 20305 | 2 | 3 | 4 | | IV-1 | Spib | 6689 | 12-May-15 |
| 20306 | 2 | 3 | 4 | | IV-1 | Spic | 121599 | 28-May-15 |
| 20317 | 2 | 3 | 4 | | IV-1 | Spink13 | 153218 | 4-May-15 |
| 20326 | 2 | 3 | 4 | | IV-1 | Spinkl | | |
| 20329 | 2 | 3 | 4 | | IV-1 | Spint3 | 10816 | 7-Jun-15 |
| 20332 | 2 | 3 | 4 | | IV-1 | Spire1 | 56907 | 4-May-15 |
| 20340 | 2 | 3 | 4 | | IV-1 | Spock1 | 6695 | 4-May-15 |
| 20341 | 2 | 3 | 4 | | IV-1 | Spock2 | 9806 | 12-May-15 |
| 20356 | 2 | 3 | 4 | | IV-1 | Spred3 | 399473 | 4-May-15 |
| 20359 | 2 | 3 | 4 | | IV-1 | Sprr1b | 6699 | 12-May-15 |
| 20360 | 2 | 3 | 4 | | IV-1 | Sprr2a1 | | |
| 20361 | 2 | 3 | 4 | | IV-1 | Sprr2a2 | | |
| 20362 | 2 | 3 | 4 | | IV-1 | Sprr2b | 6701 | 4-May-15 |
| 20363 | 2 | 3 | 4 | | IV-1 | Sprr2d | 6703 | 4-May-15 |
| 20365 | 2 | 3 | 4 | | IV-1 | Sprr2f | 6705 | 4-May-15 |
| 20366 | 2 | 3 | 4 | | IV-1 | Sprr2g | 6706 | 4-May-15 |

| | | | | | | | | |
|---|---|---|---|---|---|---|---|---|
| 20374 | 2 | 3 | 4 | | IV-1 | Spry1 | 10252 | 4-May-15 |
| 20375 | 2 | 3 | 4 | | IV-1 | Spry2 | 10253 | 17-May-15 |
| 20396 | 2 | 3 | 4 | | IV-1 | Sptssb | 165679 | 4-May-15 |
| 20399 | 2 | 3 | 4 | | IV-1 | Sqle | 6713 | 4-May-15 |
| 20407 | 2 | 3 | 4 | | IV-1 | Srd5a1 | 6715 | 12-May-15 |
| 20417 | 2 | 3 | 4 | | IV-1 | Srgap2 | 23380 | 7-Jun-15 |
| 20428 | 2 | 3 | 4 | | IV-1 | Srp54c | | |
| 20434 | 2 | 3 | 4 | | IV-1 | Srpk3 | 26576 | 4-May-15 |
| 20461 | 2 | 3 | 4 | | IV-1 | Ss18l1 | 26039 | 4-May-15 |
| 20482 | 2 | 3 | 4 | | IV-1 | Sst | 6750 | 17-May-15 |
| 20492 | 2 | 3 | 4 | | IV-1 | Ssx2ip | 117178 | 4-May-15 |
| 20496 | 2 | 3 | 4 | | IV-1 | Ssxb2 | | |
| 20505 | 2 | 3 | 4 | | IV-1 | St3gal1 | 6482 | 12-May-15 |
| 20510 | 2 | 3 | 4 | | IV-1 | St3gal6 | 10402 | 4-May-15 |
| 20515 | 2 | 3 | 4 | | IV-1 | St6galnac2 | 10610 | 4-May-15 |
| 20518 | 2 | 3 | 4 | | IV-1 | St6galnac5 | 81849 | 4-May-15 |
| 20527 | 2 | 3 | 4 | | IV-1 | St8sia5 | 29906 | 12-May-15 |
| 20528 | 2 | 3 | 4 | | IV-1 | St8sia6 | 338596 | 4-May-15 |
| 20530 | 2 | 3 | 4 | | IV-1 | Stab2 | 55576 | 12-May-15 |
| 20541 | 2 | 3 | 4 | | IV-1 | Stamos | | |
| 20542 | 2 | 3 | 4 | | IV-1 | Stap1 | 26228 | 7-Jun-15 |
| 20545 | 2 | 3 | 4 | | IV-1 | Stard10 | 10809 | 12-May-15 |
| 20564 | 2 | 3 | 4 | | IV-1 | Stc1 | 6781 | 12-May-15 |
| 20567 | 2 | 3 | 4 | | IV-1 | Steap2 | 261729 | 4-May-15 |
| 20568 | 2 | 3 | 4 | | IV-1 | Steap3 | 55240 | 4-May-15 |
| 20569 | 2 | 3 | 4 | | IV-1 | Steap4 | 79689 | 4-May-15 |
| 20571 | 2 | 3 | 4 | | IV-1 | Stfa2 | | |
| 20573 | 2 | 3 | 4 | | IV-1 | Stfa3 | | |
| 20588 | 2 | 3 | 4 | | IV-1 | Stk32a | 202374 | 28-May-15 |
| 20601 | 2 | 3 | 4 | | IV-1 | Stmn2 | 11075 | 4-May-15 |
| 20609 | 2 | 3 | 4 | | IV-1 | Ston1 | 11037 | 4-May-15 |
| 20624 | 2 | 3 | 4 | | IV-1 | Strip2 | 57464 | 4-May-15 |
| 20671 | 2 | 3 | 4 | | IV-1 | Sult1a1 | 6817 | 31-May-15 |
| 20674 | 2 | 3 | 4 | | IV-1 | Sult1c2 | 6819 | 7-Jun-15 |
| 20684 | 2 | 3 | 4 | | IV-1 | Sult2b1 | 6820 | 4-May-15 |
| 20698 | 2 | 3 | 4 | | IV-1 | Suox | 6821 | 12-May-15 |
| 20711 | 2 | 3 | 4 | | IV-1 | Susd1 | 64420 | 4-May-15 |
| 20712 | 2 | 3 | 4 | | IV-1 | Susd2 | 56241 | 4-May-15 |
| 20730 | 2 | 3 | 4 | | IV-1 | Svip | 258010 | 3-May-15 |
| 20745 | 2 | 3 | 4 | | IV-1 | Sybu | 55638 | 4-May-15 |
| 20748 | 2 | 3 | 4 | | IV-1 | Syce2 | 256126 | 4-May-15 |
| 20765 | 2 | 3 | 4 | | IV-1 | Sync | 81493 | 4-May-15 |
| 20771 | 2 | 3 | 4 | | IV-1 | Syne3 | 161176 | 4-May-15 |
| 20772 | 2 | 3 | 4 | | IV-1 | Syne4 | 163183 | 12-May-15 |
| 20774 | 2 | 3 | 4 | | IV-1 | Syngr1 | 9145 | 4-May-15 |
| 20794 | 2 | 3 | 4 | | IV-1 | Syt12 | 91683 | 7-Jun-15 |
| 20801 | 2 | 3 | 4 | | IV-1 | Syt3 | 84258 | 4-May-15 |
| 20808 | 2 | 3 | 4 | | IV-1 | Sytl1 | 84958 | 12-May-15 |
| 20809 | 2 | 3 | 4 | | IV-1 | Sytl2 | 54843 | 12-May-15 |
| 20810 | 2 | 3 | 4 | | IV-1 | Sytl3 | 94120 | 4-May-15 |
| 20836 | 2 | 3 | 4 | | IV-1 | Tac1 | 6863 | 4-May-15 |
| 20838 | 2 | 3 | 4 | | IV-1 | Tac4 | 255061 | 7-Jun-15 |
| 20876 | 2 | 3 | 4 | | IV-1 | Tagln | 6876 | 12-May-15 |
| 20879 | 2 | 3 | 4 | | IV-1 | Tal1 | 6886 | 17-May-15 |
| 20898 | 2 | 3 | 4 | | IV-1 | Tarm1 | 441864 | 4-May-15 |
| 20941 | 2 | 3 | 4 | | IV-1 | Tat | 6898 | 7-Jun-15 |
| 20967 | 2 | 3 | 4 | | IV-1 | Tbc1d24 | 57465 | 4-May-15 |
| 20970 | 2 | 3 | 4 | | IV-1 | Tbc1d30 | 23329 | 4-May-15 |
| 20973 | 2 | 3 | 4 | | IV-1 | Tbc1d4 | 9882 | 4-May-15 |
| 20978 | 2 | 3 | 4 | | IV-1 | Tbc1d9 | 23158 | 4-May-15 |
| 21006 | 2 | 3 | 4 | | IV-1 | Tbx2 | 6909 | 12-May-15 |
| 21008 | 2 | 3 | 4 | | IV-1 | Tbx21 | 30009 | 4-May-15 |
| 21010 | 2 | 3 | 4 | | IV-1 | Tbx3 | 6926 | 4-May-15 |
| 21011 | 2 | 3 | 4 | | IV-1 | Tbx3os2 | | |
| 21022 | 2 | 3 | 4 | | IV-1 | Tcea2 | 6919 | 12-May-15 |
| 21032 | 2 | 3 | 4 | | IV-1 | Tceb1 | 6921 | 4-May-15 |
| 21038 | 2 | 3 | 4 | | IV-1 | Tcf15 | 6939 | 10-May-15 |
| 21042 | 2 | 3 | 4 | | IV-1 | Tcf23 | 150921 | 4-May-15 |
| 21043 | 2 | 3 | 4 | | IV-1 | Tcf24 | 100129654 | 4-May-15 |
| 21051 | 2 | 3 | 4 | | IV-1 | Tchh | 7062 | 12-May-15 |
| 21061 | 2 | 3 | 4 | | IV-1 | Tcn2 | 6948 | 4-May-15 |
| 21075 | 2 | 3 | 4 | | IV-1 | Tcte3 | 6991 | 12-May-15 |
| 21077 | 2 | 3 | 4 | | IV-1 | Tctex1d2 | 255758 | 4-May-15 |
| 21078 | 2 | 3 | 4 | | IV-1 | Tctex1d4 | 343521 | 4-May-15 |
| 21082 | 2 | 3 | 4 | | IV-1 | Tdg | 6996 | 12-May-15 |
| 21102 | 2 | 3 | 4 | | IV-1 | Tead1 | 7003 | 4-May-15 |
| 21111 | 2 | 3 | 4 | | IV-1 | Tecta | 7007 | 23-May-15 |
| 21114 | 2 | 3 | 4 | | IV-1 | Tef | 7008 | 4-May-15 |
| 21124 | 2 | 3 | 4 | | IV-1 | Tenc1 | 23371 | 17-May-15 |
| 21131 | 2 | 3 | 4 | | IV-1 | Terc | 7012 | 24-May-15 |
| 21170 | 2 | 3 | 4 | | IV-1 | Tex38 | 374973 | 28-May-15 |
| 21180 | 2 | 3 | 4 | | IV-1 | Tfap4 | 7023 | 28-May-15 |
| 21184 | 2 | 3 | 4 | | IV-1 | Tfcp2l1 | 29842 | 4-May-15 |
| 21185 | 2 | 3 | 4 | | IV-1 | Tfdp1 | 7027 | 2-Jun-15 |
| 21186 | 2 | 3 | 4 | | IV-1 | Tfdp2 | 7029 | 5-May-15 |
| 21192 | 2 | 3 | 4 | | IV-1 | Tff3 | 7033 | 24-May-15 |
| 21198 | 2 | 3 | 4 | | IV-1 | Tfr2 | 7036 | 23-May-15 |
| 21199 | 2 | 3 | 4 | | IV-1 | Tfrc | 7037 | 24-May-15 |
| 21200 | 2 | 3 | 4 | | IV-1 | Tg | 7038 | 31-May-15 |
| 21212 | 2 | 3 | 4 | | IV-1 | Tgif1 | 7050 | 23-May-15 |
| 21216 | 2 | 3 | 4 | | IV-1 | Tgm1 | 7051 | 23-May-15 |
| 21239 | 2 | 3 | 4 | | IV-1 | Thbs1 | 7057 | 12-May-15 |
| 21240 | 2 | 3 | 4 | | IV-1 | Thbs2 | 7058 | 17-May-15 |
| 21242 | 2 | 3 | 4 | | IV-1 | Thbs4 | 7060 | 12-May-15 |
| 21245 | 2 | 3 | 4 | | IV-1 | Them5 | 284486 | 4-May-15 |
| 21246 | 2 | 3 | 4 | | IV-1 | Them6 | 51337 | 4-May-15 |
| 21247 | 2 | 3 | 4 | | IV-1 | Them7 | | |

Fig.22 - 37

| | | | | | | | | |
|---|---|---|---|---|---|---|---|---|
| 21266 | 2 | 3 | 4 | | IV-1 | Thsd1 | 55901 | 4-May-15 |
| 21267 | 2 | 3 | 4 | | IV-1 | Thsd4 | 79875 | 4-May-15 |
| 21275 | 2 | 3 | 4 | | IV-1 | Thyn1 | 29087 | 4-May-15 |
| 21283 | 2 | 3 | 4 | | IV-1 | Tie1 | 7075 | 4-May-15 |
| 21284 | 2 | 3 | 4 | | IV-1 | Tifa | 92610 | 7-Jun-15 |
| 21292 | 2 | 3 | 4 | | IV-1 | Timd4 | 91937 | 4-May-15 |
| 21312 | 2 | 3 | 4 | | IV-1 | Timp4 | 7079 | 12-May-15 |
| 21322 | 2 | 3 | 4 | | IV-1 | Tjp2 | 9414 | 12-May-15 |
| 21323 | 2 | 3 | 4 | | IV-1 | Tjp3 | 27134 | 4-May-15 |
| 21326 | 2 | 3 | 4 | | IV-1 | Tkt | 7086 | 7-Jun-15 |
| 21329 | 2 | 3 | 4 | | IV-1 | Tlcd1 | 116238 | 4-May-15 |
| 21334 | 2 | 3 | 4 | | IV-1 | Tle2 | 7089 | 4-May-15 |
| 21347 | 2 | 3 | 4 | | IV-1 | Tlr13 | | |
| 21348 | 2 | 3 | 4 | | IV-1 | Tlr2 | 7097 | 24-May-15 |
| 21351 | 2 | 3 | 4 | | IV-1 | Tlr5 | 7100 | 17-May-15 |
| 21353 | 2 | 3 | 4 | | IV-1 | Tlr7 | 51284 | 17-May-15 |
| 21363 | 2 | 3 | 4 | | IV-1 | Tm4sf19 | 116211 | 4-May-15 |
| 21365 | 2 | 3 | 4 | | IV-1 | Tm4sf4 | 7104 | 4-May-15 |
| 21368 | 2 | 3 | 4 | | IV-1 | Tm6sf2 | 53345 | 4-May-15 |
| 21369 | 2 | 3 | 4 | | IV-1 | Tm7sf2 | 7108 | 4-May-15 |
| 21384 | 2 | 3 | 4 | | IV-1 | Tmc4 | 147798 | 21-May-15 |
| 21385 | 2 | 3 | 4 | | IV-1 | Tmc5 | 79838 | 12-May-15 |
| 21387 | 2 | 3 | 4 | | IV-1 | Tmc7 | 79905 | 4-May-15 |
| 21390 | 2 | 3 | 4 | | IV-1 | Tmcc2 | 9911 | 12-May-15 |
| 21406 | 2 | 3 | 4 | | IV-1 | Tmed6 | 146456 | 4-May-15 |
| 21412 | 2 | 3 | 4 | | IV-1 | Tmem100 | 55273 | 3-May-15 |
| 21429 | 2 | 3 | 4 | | IV-1 | Tmem120b | 144404 | 4-May-15 |
| 21432 | 2 | 3 | 4 | | IV-1 | Tmem125 | 128218 | 4-May-15 |
| 21452 | 2 | 3 | 4 | | IV-1 | Tmem141 | 85014 | 4-May-15 |
| 21454 | 2 | 3 | 4 | | IV-1 | Tmem144 | 55314 | 4-May-15 |
| 21465 | 2 | 3 | 4 | | IV-1 | Tmem154 | 201799 | 4-May-15 |
| 21482 | 2 | 3 | 4 | | IV-1 | Tmem173 | 340061 | 4-May-15 |
| 21488 | 2 | 3 | 4 | | IV-1 | Tmem178 | 130733 | 2-Jun-15 |
| 21490 | 2 | 3 | 4 | | IV-1 | Tmem179 | 388021 | 4-May-15 |
| 21493 | 2 | 3 | 4 | | IV-1 | Tmem180 | 79847 | 21-May-15 |
| 21499 | 2 | 3 | 4 | | IV-1 | Tmem184a | 202915 | 4-May-15 |
| 21507 | 2 | 3 | 4 | | IV-1 | Tmem191c | 645426 | 20-May-15 |
| 21517 | 2 | 3 | 4 | | IV-1 | Tmem200b | 399474 | 4-May-15 |
| 21531 | 2 | 3 | 4 | | IV-1 | Tmem213 | 155006 | 4-May-15 |
| 21538 | 2 | 3 | 4 | | IV-1 | Tmem220 | 388335 | 4-May-15 |
| 21559 | 2 | 3 | 4 | | IV-1 | Tmem245 | 23731 | 4-May-15 |
| 21568 | 2 | 3 | 4 | | IV-1 | Tmem254b | | |
| 21572 | 2 | 3 | 4 | | IV-1 | Tmem256 | 254863 | 4-May-15 |
| 21573 | 2 | 3 | 4 | | IV-1 | Tmem258 | 746 | 4-May-15 |
| 21587 | 2 | 3 | 4 | | IV-1 | Tmem37 | 140738 | 12-May-15 |
| 21592 | 2 | 3 | 4 | | IV-1 | Tmem40 | 55287 | 4-May-15 |
| 21608 | 2 | 3 | 4 | | IV-1 | Tmem53 | 79639 | 4-May-15 |
| 21612 | 2 | 3 | 4 | | IV-1 | Tmem56 | 148534 | 4-May-15 |
| 21621 | 2 | 3 | 4 | | IV-1 | Tmem64 | 169200 | 4-May-15 |
| 21647 | 2 | 3 | 4 | | IV-1 | Tmem91 | 641649 | 4-May-15 |
| 21651 | 2 | 3 | 4 | | IV-1 | Tmem98 | 26022 | 12-May-15 |
| 21656 | 2 | 3 | 4 | | IV-1 | Tmigd1 | 388364 | 4-May-15 |
| 21664 | 2 | 3 | 4 | | IV-1 | Tmprss11a | 339967 | 12-May-15 |
| 21670 | 2 | 3 | 4 | | IV-1 | Tmprss11g | | |
| 21674 | 2 | 3 | 4 | | IV-1 | Tmprss2 | 7113 | 21-May-15 |
| 21683 | 2 | 3 | 4 | | IV-1 | Tmsb15b1 | | |
| 21684 | 2 | 3 | 4 | | IV-1 | Tmsb15b2 | | |
| 21685 | 2 | 3 | 4 | | IV-1 | Tmsb15l | | |
| 21698 | 2 | 3 | 4 | | IV-1 | Tnf | 7124 | 31-May-15 |
| 21701 | 2 | 3 | 4 | | IV-1 | Tnfaip3 | 7128 | 12-May-15 |
| 21702 | 2 | 3 | 4 | | IV-1 | Tnfaip6 | 7130 | 23-May-15 |
| 21705 | 2 | 3 | 4 | | IV-1 | Tnfaip8l2 | 79626 | 4-May-15 |
| 21710 | 2 | 3 | 4 | | IV-1 | Tnfrsf12a | 51330 | 4-May-15 |
| 21711 | 2 | 3 | 4 | | IV-1 | Tnfrsf13b | 23495 | 4-May-15 |
| 21715 | 2 | 3 | 4 | | IV-1 | Tnfrsf18 | 8784 | 3-May-15 |
| 21716 | 2 | 3 | 4 | | IV-1 | Tnfrsf19 | 55504 | 4-May-15 |
| 21718 | 2 | 3 | 4 | | IV-1 | Tnfrsf1b | 7133 | 12-May-15 |
| 21719 | 2 | 3 | 4 | | IV-1 | Tnfrsf21 | 27242 | 4-May-15 |
| 21722 | 2 | 3 | 4 | | IV-1 | Tnfrsf25 | 8718 | 24-May-15 |
| 21724 | 2 | 3 | 4 | | IV-1 | Tnfrsf4 | 7293 | 4-May-15 |
| 21726 | 2 | 3 | 4 | | IV-1 | Tnfrsf9 | 3604 | 24-May-15 |
| 21731 | 2 | 3 | 4 | | IV-1 | Tnfsf13 | 8741 | 17-May-15 |
| 21732 | 2 | 3 | 4 | | IV-1 | Tnfsf13b | 10673 | 17-May-15 |
| 21743 | 2 | 3 | 4 | | IV-1 | Tnk1 | 8711 | 12-May-15 |
| 21746 | 2 | 3 | 4 | | IV-1 | Tnks | 8658 | 4-May-15 |
| 21747 | 2 | 3 | 4 | | IV-1 | Tnks1bp1 | 85456 | 4-May-15 |
| 21749 | 2 | 3 | 4 | | IV-1 | Tnmd | 64102 | 4-May-15 |
| 21751 | 2 | 3 | 4 | | IV-1 | Tnnc1 | 7134 | 7-Jun-15 |
| 21755 | 2 | 3 | 4 | | IV-1 | Tnni3 | 7137 | 23-May-15 |
| 21757 | 2 | 3 | 4 | | IV-1 | Tnnt1 | 7138 | 23-May-15 |
| 21758 | 2 | 3 | 4 | | IV-1 | Tnnt2 | 7139 | 23-May-15 |
| 21771 | 2 | 3 | 4 | | IV-1 | Tns3 | 64759 | 12-May-15 |
| 21772 | 2 | 3 | 4 | | IV-1 | Tns4 | 84951 | 12-May-15 |
| 21773 | 2 | 3 | 4 | | IV-1 | Tnxb | 7148 | 8-May-15 |
| 21779 | 2 | 3 | 4 | | IV-1 | Tom1l1 | 10040 | 4-May-15 |
| 21804 | 2 | 3 | 4 | | IV-1 | Toporsos | | |
| 21816 | 2 | 3 | 4 | | IV-1 | Tpbg | 7162 | 4-May-15 |
| 21821 | 2 | 3 | 4 | | IV-1 | Tpd52 | 7163 | 4-May-15 |
| 21830 | 2 | 3 | 4 | | IV-1 | Tpm1 | 7168 | 31-May-15 |
| 21834 | 2 | 3 | 4 | | IV-1 | Tpmt | 7172 | 12-May-15 |
| 21848 | 2 | 3 | 4 | | IV-1 | Tpsb2 | 64499 | 4-May-15 |
| 21851 | 2 | 3 | 4 | | IV-1 | Tpst2 | 8459 | 21-May-15 |
| 21876 | 2 | 3 | 4 | | IV-1 | Tram2 | 9697 | 21-May-15 |
| 21902 | 2 | 3 | 4 | | IV-1 | Trem3 | | |
| 21908 | 2 | 3 | 4 | | IV-1 | Trex2 | 11219 | 4-May-15 |
| 21909 | 2 | 3 | 4 | | IV-1 | Trf | 7018 | 7-Jun-15 |
| 21917 | 2 | 3 | 4 | | IV-1 | Trib3 | 57761 | 12-May-15 |
| 21919 | 2 | 3 | 4 | | IV-1 | Trim10 | 10107 | 4-May-15 |
| 21931 | 2 | 3 | 4 | | IV-1 | Trim24 | 8805 | 4-May-15 |
| 21936 | 2 | 3 | 4 | | IV-1 | Trim29 | 23650 | 12-May-15 |
| 21940 | 2 | 3 | 4 | | IV-1 | Trim30d | | |
| 21948 | 2 | 3 | 4 | | IV-1 | Trim36 | 55521 | 12-May-15 |
| 21965 | 2 | 3 | 4 | | IV-1 | Trim55 | 84675 | 12-May-15 |
| 21966 | 2 | 3 | 4 | | IV-1 | Trim56 | 81844 | 4-May-15 |
| 21967 | 2 | 3 | 4 | | IV-1 | Trim58 | 25893 | 4-May-15 |
| 21990 | 2 | 3 | 4 | | IV-1 | Trip11 | 9321 | 6-May-15 |
| 22030 | 2 | 3 | 4 | | IV-1 | Trp63 | | |
| 22039 | 2 | 3 | 4 | | IV-1 | Trpc5os | 100329135 | 4-May-15 |
| 22045 | 2 | 3 | 4 | | IV-1 | Trpm3 | 80036 | 4-May-15 |
| 22046 | 2 | 3 | 4 | | IV-1 | Trpm4 | 54795 | 12-May-15 |
| 22047 | 2 | 3 | 4 | | IV-1 | Trpm5 | 29850 | 4-May-15 |
| 22056 | 2 | 3 | 4 | | IV-1 | Trpv4 | 59341 | 23-May-15 |
| 22057 | 2 | 3 | 4 | | IV-1 | Trpv5 | 56302 | 12-May-15 |
| 22062 | 2 | 3 | 4 | | IV-1 | Try10 | | |
| 22065 | 2 | 3 | 4 | | IV-1 | Tsacc | 128229 | 21-May-15 |
| 22068 | 2 | 3 | 4 | | IV-1 | Tsc22d1 | 8848 | 12-May-15 |
| 22070 | 2 | 3 | 4 | | IV-1 | Tsc22d3 | 1831 | 4-May-15 |
| 22079 | 2 | 3 | 4 | | IV-1 | Tsga13 | 114960 | 12-May-15 |
| 22088 | 2 | 3 | 4 | | IV-1 | Tsku | 25987 | 4-May-15 |
| 22089 | 2 | 3 | 4 | | IV-1 | Tslp | 85480 | 24-May-15 |
| 22096 | 2 | 3 | 4 | | IV-1 | Tspan12 | 23554 | 23-May-15 |
| 22102 | 2 | 3 | 4 | | IV-1 | Tspan2 | 10100 | 21-May-15 |
| 22107 | 2 | 3 | 4 | | IV-1 | Tspan33 | 340348 | 4-May-15 |
| 22108 | 2 | 3 | 4 | | IV-1 | Tspan4 | 7106 | 4-May-15 |
| 22110 | 2 | 3 | 4 | | IV-1 | Tspan6 | 7105 | 4-May-15 |
| 22112 | 2 | 3 | 4 | | IV-1 | Tspan8 | 7103 | 17-May-15 |
| 22116 | 2 | 3 | 4 | | IV-1 | Tspo2 | 222642 | 4-May-15 |
| 22129 | 2 | 3 | 4 | | IV-1 | Tssk2 | 23617 | 4-May-15 |
| 22131 | 2 | 3 | 4 | | IV-1 | Tssk4 | 283629 | 7-Jun-15 |
| 22156 | 2 | 3 | 4 | | IV-1 | Ttc25 | 83538 | 4-May-15 |
| 22180 | 2 | 3 | 4 | | IV-1 | Ttc9 | 23508 | 4-May-15 |
| 22190 | 2 | 3 | 4 | | IV-1 | Ttll10 | 254173 | 4-May-15 |
| 22194 | 2 | 3 | 4 | | IV-1 | Ttll2 | 83887 | 4-May-15 |
| 22196 | 2 | 3 | 4 | | IV-1 | Ttll4 | 9654 | 21-May-15 |
| 22203 | 2 | 3 | 4 | | IV-1 | Ttpa | 7274 | 23-May-15 |
| 22206 | 2 | 3 | 4 | | IV-1 | Ttyh1 | 57348 | 4-May-15 |
| 22215 | 2 | 3 | 4 | | IV-1 | Tuba4a | 7277 | 4-May-15 |
| 22219 | 2 | 3 | 4 | | IV-1 | Tubb2a | 7280 | 12-May-15 |
| 22221 | 2 | 3 | 4 | | IV-1 | Tubb2b | 347733 | 4-May-15 |
| 22222 | 2 | 3 | 4 | | IV-1 | Tubb3 | 10381 | 23-May-15 |
| 22239 | 2 | 3 | 4 | | IV-1 | Tulp1 | 7287 | 31-May-15 |
| 22244 | 2 | 3 | 4 | | IV-1 | Tusc1 | 286319 | 4-May-15 |
| 22246 | 2 | 3 | 4 | | IV-1 | Tusc3 | 7991 | 23-May-15 |
| 22268 | 2 | 3 | 4 | | IV-1 | Txndc2 | 84203 | 12-May-15 |
| 22285 | 2 | 3 | 4 | | IV-1 | Tyrobp | 7305 | 23-May-15 |
| 22298 | 2 | 3 | 4 | | IV-1 | Uap1l1 | 91373 | 23-May-15 |
| 22307 | 2 | 3 | 4 | | IV-1 | Ubac1 | 10422 | 4-May-15 |
| 22310 | 2 | 3 | 4 | | IV-1 | Ubald2 | 283991 | 4-May-15 |
| 22319 | 2 | 3 | 4 | | IV-1 | Ubd | 10537 | 3-May-15 |
| 22342 | 2 | 3 | 4 | | IV-1 | Ube2l6 | 9246 | 12-May-15 |
| 22348 | 2 | 3 | 4 | | IV-1 | Ube2ql1 | 134111 | 4-May-15 |
| 22360 | 2 | 3 | 4 | | IV-1 | Ube4a | 9354 | 12-May-15 |
| 22366 | 2 | 3 | 4 | | IV-1 | Ubl4b | 164153 | 4-May-15 |
| 22390 | 2 | 3 | 4 | | IV-1 | Ubxn10 | 127733 | 4-May-15 |
| 22406 | 2 | 3 | 4 | | IV-1 | Uckl1os | 100113386 | 4-May-15 |
| 22408 | 2 | 3 | 4 | | IV-1 | Ucn | 7349 | 4-May-15 |
| 22409 | 2 | 3 | 4 | | IV-1 | Ucn2 | 90226 | 7-Jun-15 |
| 22413 | 2 | 3 | 4 | | IV-1 | Ucp3 | 7352 | 17-May-15 |
| 22422 | 2 | 3 | 4 | | IV-1 | Ugdh | 7358 | 12-May-15 |
| 22429 | 2 | 3 | 4 | | IV-1 | Ugt1a5 | 54579 | 12-May-15 |
| 22430 | 2 | 3 | 4 | | IV-1 | Ugt1a6a | | |
| 22431 | 2 | 3 | 4 | | IV-1 | Ugt1a6b | | |
| 22432 | 2 | 3 | 4 | | IV-1 | Ugt1a7c | | |
| 22433 | 2 | 3 | 4 | | IV-1 | Ugt1a9 | 54600 | 28-May-15 |
| 22438 | 2 | 3 | 4 | | IV-1 | Ugt2b34 | | |
| 22440 | 2 | 3 | 4 | | IV-1 | Ugt2b36 | | |
| 22443 | 2 | 3 | 4 | | IV-1 | Ugt2b5 | | |
| 22446 | 2 | 3 | 4 | | IV-1 | Ugt8a | | |
| 22447 | 2 | 3 | 4 | | IV-1 | Uhmk1 | 127933 | 4-May-15 |
| 22448 | 2 | 3 | 4 | | IV-1 | Uhrf1 | 29128 | 12-May-15 |
| 22458 | 2 | 3 | 4 | | IV-1 | Umod | 7369 | 23-May-15 |
| 22464 | 2 | 3 | 4 | | IV-1 | Unc13b | 10497 | 4-May-15 |
| 22468 | 2 | 3 | 4 | | IV-1 | Unc45b | 146862 | 12-May-15 |
| 22470 | 2 | 3 | 4 | | IV-1 | Unc5a | 90249 | 4-May-15 |
| 22477 | 2 | 3 | 4 | | IV-1 | Unc93a | 54346 | 4-May-15 |
| 22480 | 2 | 3 | 4 | | IV-1 | Ung | 7374 | 4-May-15 |
| 22489 | 2 | 3 | 4 | | IV-1 | Upk1a | 11045 | 4-May-15 |
| 22490 | 2 | 3 | 4 | | IV-1 | Upk1b | 7348 | 4-May-15 |
| 22492 | 2 | 3 | 4 | | IV-1 | Upk3a | 7380 | 4-May-15 |
| 22493 | 2 | 3 | 4 | | IV-1 | Upk3b | 80761 | 19-Mar-15 |
| 22497 | 2 | 3 | 4 | | IV-1 | Uprt | 139596 | 12-May-15 |
| 22499 | 2 | 3 | 4 | | IV-1 | Uqcc2 | 84300 | 14-May-15 |
| 22516 | 2 | 3 | 4 | | IV-1 | Urod | 7389 | 23-May-15 |
| 22526 | 2 | 3 | 4 | | IV-1 | Usmg5 | 84833 | 4-May-15 |
| 22543 | 2 | 3 | 4 | | IV-1 | Usp2 | 9099 | 12-May-15 |
| 22544 | 2 | 3 | 4 | | IV-1 | Usp20 | 10868 | 4-May-15 |
| 22558 | 2 | 3 | 4 | | IV-1 | Usp34 | 9736 | 4-May-15 |
| 22559 | 2 | 3 | 4 | | IV-1 | Usp35 | 57558 | 4-May-15 |
| 22570 | 2 | 3 | 4 | | IV-1 | Usp46 | 64854 | 4-May-15 |
| 22589 | 2 | 3 | 4 | | IV-1 | Utp14b | 9724 | 4-May-15 |
| 22599 | 2 | 3 | 4 | | IV-1 | Uts2r | 2837 | 4-May-15 |
| 22627 | 2 | 3 | 4 | | IV-1 | Vat1l | 57687 | 4-May-15 |
| 22636 | 2 | 3 | 4 | | IV-1 | Vcam1 | 7412 | 17-May-15 |

Fig.22 · 38

| | | | | | | | | |
|---|---|---|---|---|---|---|---|---|
| 22641 | 2 | 3 | 4 | | IV-1 | Vcpkmt | 79609 | 4-May-15 |
| 22642 | 2 | 3 | 4 | | IV-1 | Vdac1 | 7416 | 12-May-15 |
| 22645 | 2 | 3 | 4 | | IV-1 | Vdr | 7421 | 7-Jun-15 |
| 22652 | 2 | 3 | 4 | | IV-1 | Vgf | 7425 | 4-May-15 |
| 22662 | 2 | 3 | 4 | | IV-1 | Vip | 7432 | 7-Jun-15 |
| 22669 | 2 | 3 | 4 | | IV-1 | Vldlr | 7436 | 23-May-15 |
| 22996 | 2 | 3 | 4 | | IV-1 | Vmn2r97 | | |
| 23006 | 2 | 3 | 4 | | IV-1 | Vnn1 | 8876 | 4-May-15 |
| 23007 | 2 | 3 | 4 | | IV-1 | Vnn3 | 55350 | 23-May-15 |
| 23016 | 2 | 3 | 4 | | IV-1 | Vps13c | 54832 | 4-May-15 |
| 23023 | 2 | 3 | 4 | | IV-1 | Vps28 | 51160 | 4-May-15 |
| 23030 | 2 | 3 | 4 | | IV-1 | Vps37b | 79720 | 4-May-15 |
| 23032 | 2 | 3 | 4 | | IV-1 | Vps37d | 155382 | 4-May-15 |
| 23050 | 2 | 3 | 4 | | IV-1 | Vsig10 | 54621 | 4-May-15 |
| 23052 | 2 | 3 | 4 | | IV-1 | Vsig2 | 23584 | 4-May-15 |
| 23054 | 2 | 3 | 4 | | IV-1 | Vsig8 | 391123 | 4-May-15 |
| 23058 | 2 | 3 | 4 | | IV-1 | Vstm2l | 128434 | 4-May-15 |
| 23060 | 2 | 3 | 4 | | IV-1 | Vstm5 | 387804 | 4-May-15 |
| 23067 | 2 | 3 | 4 | | IV-1 | Vtn | 7448 | 24-May-15 |
| 23069 | 2 | 3 | 4 | | IV-1 | Vwa2 | 340706 | 4-May-15 |
| 23085 | 2 | 3 | 4 | | IV-1 | Wars | 7453 | 12-May-15 |
| 23107 | 2 | 3 | 4 | | IV-1 | Wdfy2 | 115825 | 12-May-15 |
| 23123 | 2 | 3 | 4 | | IV-1 | Wdr25 | 79446 | 4-May-15 |
| 23187 | 2 | 3 | 4 | | IV-1 | Wfdc12 | 128488 | 4-May-15 |
| 23191 | 2 | 3 | 4 | | IV-1 | Wfdc16 | | |
| 23192 | 2 | 3 | 4 | | IV-1 | Wfdc17 | | |
| 23194 | 2 | 3 | 4 | | IV-1 | Wfdc2 | 10406 | 4-May-15 |
| 23202 | 2 | 3 | 4 | | IV-1 | Wfikkn2 | 124857 | 12-May-15 |
| 23205 | 2 | 3 | 4 | | IV-1 | Whrn | 25861 | 23-May-15 |
| 23212 | 2 | 3 | 4 | | IV-1 | Wipf3 | 644150 | 4-May-15 |
| 23213 | 2 | 3 | 4 | | IV-1 | Wipi1 | 55062 | 21-May-15 |
| 23216 | 2 | 3 | 4 | | IV-1 | Wisp2 | 8839 | 21-May-15 |
| 23221 | 2 | 3 | 4 | | IV-1 | Wnk2 | 65268 | 12-May-15 |
| 23223 | 2 | 3 | 4 | | IV-1 | Wnk4 | 65266 | 23-May-15 |
| 23225 | 2 | 3 | 4 | | IV-1 | Wnt10a | 80326 | 4-May-15 |
| 23229 | 2 | 3 | 4 | | IV-1 | Wnt2 | 7472 | 12-May-15 |
| 23230 | 2 | 3 | 4 | | IV-1 | Wnt2b | 7482 | 17-May-15 |
| 23235 | 2 | 3 | 4 | | IV-1 | Wnt5b | 81029 | 12-May-15 |
| 23236 | 2 | 3 | 4 | | IV-1 | Wnt6 | 7475 | 4-May-15 |
| 23238 | 2 | 3 | 4 | | IV-1 | Wnt7b | 7477 | 4-May-15 |
| 23247 | 2 | 3 | 4 | | IV-1 | Wsb1 | 26118 | 14-May-15 |
| 23254 | 2 | 3 | 4 | | IV-1 | Wtip | 126374 | 4-May-15 |
| 23255 | 2 | 3 | 4 | | IV-1 | Wwc1 | 23286 | 24-May-15 |
| 23264 | 2 | 3 | 4 | | IV-1 | Xcl1 | 6375 | 4-May-15 |
| 23266 | 2 | 3 | 4 | | IV-1 | Xdh | 7498 | 24-May-15 |
| 23271 | 2 | 3 | 4 | | IV-1 | Xk | 7504 | 26-May-15 |
| 23280 | 2 | 3 | 4 | | IV-1 | Xlr3a | | |
| 23281 | 2 | 3 | 4 | | IV-1 | Xlr3b | | |
| 23283 | 2 | 3 | 4 | | IV-1 | Xlr4a | | |
| 23295 | 2 | 3 | 4 | | IV-1 | Xpnpep3 | 63929 | 4-May-15 |
| 23307 | 2 | 3 | 4 | | IV-1 | Xrcc5 | 7520 | 23-May-15 |
| 23309 | 2 | 3 | 4 | | IV-1 | Xrcc6bp1 | 91419 | 4-May-15 |
| 23310 | 2 | 3 | 4 | | IV-1 | Xrn1 | 54464 | 4-May-15 |
| 23314 | 2 | 3 | 4 | | IV-1 | Xylb | 9942 | 4-May-15 |
| 23325 | 2 | 3 | 4 | | IV-1 | Ybx3 | 8531 | 12-May-15 |
| 23342 | 2 | 3 | 4 | | IV-1 | Yod1 | 55432 | 29-May-15 |
| 23344 | 2 | 3 | 4 | | IV-1 | Ypel2 | 388403 | 4-May-15 |
| 23365 | 2 | 3 | 4 | | IV-1 | Zap70 | 7535 | 23-May-15 |
| 23366 | 2 | 3 | 4 | | IV-1 | Zar1 | 326340 | 4-May-15 |
| 23373 | 2 | 3 | 4 | | IV-1 | Zbp1 | 81030 | 7-Jun-15 |
| 23379 | 2 | 3 | 4 | | IV-1 | Zbtb16 | 7704 | 4-May-15 |
| 23390 | 2 | 3 | 4 | | IV-1 | Zbtb32 | 27033 | 4-May-15 |
| 23399 | 2 | 3 | 4 | | IV-1 | Zbtb42 | 100128927 | 4-May-15 |
| 23412 | 2 | 3 | 4 | | IV-1 | Zbtb8b | 728116 | 4-May-15 |
| 23413 | 2 | 3 | 4 | | IV-1 | Zbtb8os | 339487 | 21-May-15 |
| 23418 | 2 | 3 | 4 | | IV-1 | Zc2hc1c | 79696 | 4-May-15 |
| 23421 | 2 | 3 | 4 | | IV-1 | Zc3h12a | 80149 | 4-May-15 |
| 23423 | 2 | 3 | 4 | | IV-1 | Zc3h12c | 85463 | 4-May-15 |
| 23434 | 2 | 3 | 4 | | IV-1 | Zc3h8 | 84524 | 4-May-15 |
| 23443 | 2 | 3 | 4 | | IV-1 | Zcchc14 | 23174 | 4-May-15 |
| 23446 | 2 | 3 | 4 | | IV-1 | Zcchc18 | 644353 | 4-May-15 |
| 23473 | 2 | 3 | 4 | | IV-1 | Zdhhc23 | 254887 | 4-May-15 |
| 23487 | 2 | 3 | 4 | | IV-1 | Zf12 | | |
| 23507 | 2 | 3 | 4 | | IV-1 | Zfp108 | | |
| 23516 | 2 | 3 | 4 | | IV-1 | Zfp119b | | |
| 23520 | 2 | 3 | 4 | | IV-1 | Zfp13 | 7755 | 4-May-15 |
| 23530 | 2 | 3 | 4 | | IV-1 | Zfp169 | | |
| 23535 | 2 | 3 | 4 | | IV-1 | Zfp185 | | |
| 23573 | 2 | 3 | 4 | | IV-1 | Zfp316 | | |
| 23593 | 2 | 3 | 4 | | IV-1 | Zfp36 | 7538 | 21-May-15 |
| 23596 | 2 | 3 | 4 | | IV-1 | Zfp366 | | |
| 23600 | 2 | 3 | 4 | | IV-1 | Zfp36l2 | 678 | 4-May-15 |
| 23606 | 2 | 3 | 4 | | IV-1 | Zfp385a | | |
| 23607 | 2 | 3 | 4 | | IV-1 | Zfp385b | | |
| 23628 | 2 | 3 | 4 | | IV-1 | Zfp429 | | |
| 23651 | 2 | 3 | 4 | | IV-1 | Zfp503 | | |
| 23676 | 2 | 3 | 4 | | IV-1 | Zfp566 | | |
| 23678 | 2 | 3 | 4 | | IV-1 | Zfp57 | 346171 | 22-May-15 |
| 23701 | 2 | 3 | 4 | | IV-1 | Zfp612 | 7571 | 12-May-15 |
| 23720 | 2 | 3 | 4 | | IV-1 | Zfp651 | | |
| 23721 | 2 | 3 | 4 | | IV-1 | Zfp652 | | |
| 23740 | 2 | 3 | 4 | | IV-1 | Zfp692 | 55657 | 4-May-15 |
| 23791 | 2 | 3 | 4 | | IV-1 | Zfp81 | | |
| 23869 | 2 | 3 | 4 | | IV-1 | Zfr2 | 23217 | 4-May-15 |
| 23882 | 2 | 3 | 4 | | IV-1 | Zg16 | 653808 | 4-May-15 |
| 23888 | 2 | 3 | 4 | | IV-1 | Zhx3 | 23051 | 4-May-15 |
| 23908 | 2 | 3 | 4 | | IV-1 | Zmat1 | 84460 | 4-May-15 |

| | | | | | | | | |
|---|---|---|---|---|---|---|---|---|
| 23911 | 2 | 3 | 4 | | IV-1 | Zmat4 | 79698 | 2-Jun-15 |
| 23940 | 2 | 3 | 4 | | IV-1 | Znrf4 | 148066 | 4-May-15 |
| 23957 | 2 | 3 | 4 | | IV-1 | Zscan18 | 65982 | 28-May-15 |
| 3 | 2 | 3 | | | III-2 | 0610009B22Rik | | |
| 6 | 2 | 3 | | | III-2 | 0610010B08Rik | | |
| 8 | 2 | 3 | | | III-2 | 0610010K14Rik | | |
| 11 | 2 | 3 | | | III-2 | 0610030E20Rik | | |
| 12 | 2 | 3 | | | III-2 | 0610031J06Rik | | |
| 15 | 2 | 3 | | | III-2 | 0610038B21Rik | | |
| 26 | 2 | 3 | | | III-2 | 1110004F10Rik | | |
| 33 | 2 | 3 | | | III-2 | 1110015O18Rik | | |
| 34 | 2 | 3 | | | III-2 | 1110017D15Rik | | |
| 39 | 2 | 3 | | | III-2 | 1110028F18Rik | | |
| 42 | 2 | 3 | | | III-2 | 1110034G24Rik | | |
| 44 | 2 | 3 | | | III-2 | 1110037F02Rik | | |
| 61 | 2 | 3 | | | III-2 | 1300002E11Rik | | |
| 71 | 2 | 3 | | | III-2 | 1500015A07Rik | | |
| 73 | 2 | 3 | | | III-2 | 1500015O10Rik | | |
| 76 | 2 | 3 | | | III-2 | 1600002H07Rik | | |
| 80 | 2 | 3 | | | III-2 | 1600014C10Rik | | |
| 94 | 2 | 3 | | | III-2 | 1700001D01Rik | | |
| 95 | 2 | 3 | | | III-2 | 1700001F09Rik | | |
| 106 | 2 | 3 | | | III-2 | 1700003C15Rik | | |
| 126 | 2 | 3 | | | III-2 | 1700007K09Rik | | |
| 130 | 2 | 3 | | | III-2 | 1700008F21Rik | | |
| 134 | 2 | 3 | | | III-2 | 1700008O03Rik | | |
| 141 | 2 | 3 | | | III-2 | 1700010D01Rik | | |
| 145 | 2 | 3 | | | III-2 | 1700010K23Rik | | |
| 151 | 2 | 3 | | | III-2 | 1700011L22Rik | | |
| 159 | 2 | 3 | | | III-2 | 1700012L04Rik | | |
| 170 | 2 | 3 | | | III-2 | 1700016G22Rik | | |
| 189 | 2 | 3 | | | III-2 | 1700019B03Rik | | |
| 193 | 2 | 3 | | | III-2 | 1700019G17Rik | | |
| 197 | 2 | 3 | | | III-2 | 1700019N19Rik | | |
| 202 | 2 | 3 | | | III-2 | 1700020I14Rik | | |
| 205 | 2 | 3 | | | III-2 | 1700020N01Rik | | |
| 209 | 2 | 3 | | | III-2 | 1700021F07Rik | | |
| 231 | 2 | 3 | | | III-2 | 1700025G04Rik | | |
| 246 | 2 | 3 | | | III-2 | 1700028E10Rik | | |
| 265 | 2 | 3 | | | III-2 | 1700030F18Rik | | |
| 268 | 2 | 3 | | | III-2 | 1700030L20Rik | | |
| 269 | 2 | 3 | | | III-2 | 1700030M09Rik | | |
| 275 | 2 | 3 | | | III-2 | 1700031P21Rik | | |
| 289 | 2 | 3 | | | III-2 | 1700039E22Rik | | |
| 309 | 2 | 3 | | | III-2 | 1700049E15Rik | | |
| 310 | 2 | 3 | | | III-2 | 1700049E22Rik | | |
| 313 | 2 | 3 | | | III-2 | 1700051A21Rik | | |
| 317 | 2 | 3 | | | III-2 | 1700054A03Rik | | |
| 318 | 2 | 3 | | | III-2 | 1700054K19Rik | | |
| 320 | 2 | 3 | | | III-2 | 1700054O13Rik | | |
| 325 | 2 | 3 | | | III-2 | 1700057H15Rik | | |
| 329 | 2 | 3 | | | III-2 | 1700061G19Rik | | |
| 338 | 2 | 3 | | | III-2 | 1700065J11Rik | | |
| 346 | 2 | 3 | | | III-2 | 1700066O22Rik | | |
| 357 | 2 | 3 | | | III-2 | 1700074H08Rik | | |
| 366 | 2 | 3 | | | III-2 | 1700084J12Rik | | |
| 378 | 2 | 3 | | | III-2 | 1700094D03Rik | | |
| 387 | 2 | 3 | | | III-2 | 1700101E01Rik | | |
| 390 | 2 | 3 | | | III-2 | 1700102H20Rik | | |
| 393 | 2 | 3 | | | III-2 | 1700105P06Rik | | |
| 408 | 2 | 3 | | | III-2 | 1700112J05Rik | | |
| 414 | 2 | 3 | | | III-2 | 1700120G07Rik | | |
| 417 | 2 | 3 | | | III-2 | 1700121N20Rik | | |
| 428 | 2 | 3 | | | III-2 | 1700125G22Rik | | |
| 431 | 2 | 3 | | | III-2 | 1700126H18Rik | | |
| 448 | 2 | 3 | | | III-2 | 1810014B01Rik | | |
| 455 | 2 | 3 | | | III-2 | 1810026B05Rik | | |
| 457 | 2 | 3 | | | III-2 | 1810030O07Rik | | |
| 460 | 2 | 3 | | | III-2 | 1810037I17Rik | | |
| 461 | 2 | 3 | | | III-2 | 1810041L15Rik | | |
| 463 | 2 | 3 | | | III-2 | 1810043H04Rik | | |
| 477 | 2 | 3 | | | III-2 | 2010009K17Rik | | |
| 480 | 2 | 3 | | | III-2 | 2010015L04Rik | | |
| 483 | 2 | 3 | | | III-2 | 2010106E10Rik | | |
| 501 | 2 | 3 | | | III-2 | 2210015D19Rik | | |
| 503 | 2 | 3 | | | III-2 | 2210016L21Rik | | |
| 514 | 2 | 3 | | | III-2 | 2210416O15Rik | | |
| 528 | 2 | 3 | | | III-2 | 2310005A03Rik | | |
| 529 | 2 | 3 | | | III-2 | 2310005E17Rik | | |
| 538 | 2 | 3 | | | III-2 | 2310014L17Rik | | |
| 542 | 2 | 3 | | | III-2 | 2310016D03Rik | | |
| 553 | 2 | 3 | | | III-2 | 2310036O22Rik | | |
| 562 | 2 | 3 | | | III-2 | 2310050C09Rik | | |
| 565 | 2 | 3 | | | III-2 | 2310057N15Rik | | |
| 571 | 2 | 3 | | | III-2 | 2310068J16Rik | | |
| 584 | 2 | 3 | | | III-2 | 2410012E07Rik | | |
| 589 | 2 | 3 | | | III-2 | 2410018L13Rik | | |
| 593 | 2 | 3 | | | III-2 | 2410089E03Rik | | |
| 597 | 2 | 3 | | | III-2 | 2410131K14Rik | | |
| 601 | 2 | 3 | | | III-2 | 2510002D24Rik | | |
| 602 | 2 | 3 | | | III-2 | 2510003E04Rik | | |
| 603 | 2 | 3 | | | III-2 | 2510009E07Rik | | |
| 611 | 2 | 3 | | | III-2 | 2610015P09Rik | | |
| 615 | 2 | 3 | | | III-2 | 2610020H08Rik | | |
| 616 | 2 | 3 | | | III-2 | 2610027K06Rik | | |
| 618 | 2 | 3 | | | III-2 | 2610028H24Rik | | |
| 621 | 2 | 3 | | | III-2 | 2610035D17Rik | | |

Fig.22 - 39

| | | | | | | | | |
|---|---|---|---|---|---|---|---|---|
| 624 | 2 | 3 | | | III-2 | 2610044O15Rik8 | | |
| 634 | 2 | 3 | | | III-2 | 2610316D01Rik | | |
| 637 | 2 | 3 | | | III-2 | 2610507I01Rik | | |
| 644 | 2 | 3 | | | III-2 | 2700046G09Rik | | |
| 646 | 2 | 3 | | | III-2 | 2700054A10Rik | | |
| 651 | 2 | 3 | | | III-2 | 2700081O15Rik | | |
| 654 | 2 | 3 | | | III-2 | 2700089I24Rik | | |
| 655 | 2 | 3 | | | III-2 | 2700094K13Rik | | |
| 661 | 2 | 3 | | | III-2 | 2810006K23Rik | | |
| 665 | 2 | 3 | | | III-2 | 2810021J22Rik | | |
| 670 | 2 | 3 | | | III-2 | 2810049E08Rik | | |
| 675 | 2 | 3 | | | III-2 | 2810405F15Rik | | |
| 679 | 2 | 3 | | | III-2 | 2810410L24Rik | | |
| 682 | 2 | 3 | | | III-2 | 2810429I04Rik | | |
| 685 | 2 | 3 | | | III-2 | 2810442N19Rik | | |
| 686 | 2 | 3 | | | III-2 | 2810454H06Rik | | |
| 690 | 2 | 3 | | | III-2 | 2810474O19Rik | | |
| 695 | 2 | 3 | | | III-2 | 2900026A02Rik | | |
| 704 | 2 | 3 | | | III-2 | 2900092C05Rik | | |
| 709 | 2 | 3 | | | III-2 | 3010026O09Rik | | |
| 712 | 2 | 3 | | | III-2 | 3110001I22Rik | | |
| 717 | 2 | 3 | | | III-2 | 3110015C05Rik | | |
| 721 | 2 | 3 | | | III-2 | 3110039I08Rik | | |
| 727 | 2 | 3 | | | III-2 | 3110056K07Rik | | |
| 730 | 2 | 3 | | | III-2 | 3110070M22Rik | | |
| 734 | 2 | 3 | | | III-2 | 3110099E03Rik | | |
| 741 | 2 | 3 | | | III-2 | 3830403N18Rik | | |
| 749 | 2 | 3 | | | III-2 | 4632427E13Rik | | |
| 751 | 2 | 3 | | | III-2 | 4632428N05Rik | | |
| 753 | 2 | 3 | | | III-2 | 4732416N19Rik | | |
| 755 | 2 | 3 | | | III-2 | 4732471J01Rik | | |
| 757 | 2 | 3 | | | III-2 | 4732491K20Rik | | |
| 766 | 2 | 3 | | | III-2 | 4833422C13Rik | | |
| 792 | 2 | 3 | | | III-2 | 4921525O09Rik | | |
| 799 | 2 | 3 | | | III-2 | 4921536K21Rik | | |
| 817 | 2 | 3 | | | III-2 | 4930405A21Rik | | |
| 818 | 2 | 3 | | | III-2 | 4930405D11Rik | | |
| 819 | 2 | 3 | | | III-2 | 4930405J17Rik | | |
| 828 | 2 | 3 | | | III-2 | 4930413F20Rik | | |
| 831 | 2 | 3 | | | III-2 | 4930414L22Rik | | |
| 833 | 2 | 3 | | | III-2 | 4930415F15Rik | | |
| 837 | 2 | 3 | | | III-2 | 4930417O22Rik | | |
| 846 | 2 | 3 | | | III-2 | 4930428E07Rik | | |
| 848 | 2 | 3 | | | III-2 | 4930428O21Rik | | |
| 850 | 2 | 3 | | | III-2 | 4930429D17Rik | | |
| 854 | 2 | 3 | | | III-2 | 4930430D24Rik | | |
| 857 | 2 | 3 | | | III-2 | 4930430J02Rik | | |
| 861 | 2 | 3 | | | III-2 | 4930432K21Rik | | |
| 875 | 2 | 3 | | | III-2 | 4930444F02Rik | | |
| 885 | 2 | 3 | | | III-2 | 4930448F12Rik | | |
| 898 | 2 | 3 | | | III-2 | 4930452G13Rik | | |
| 911 | 2 | 3 | | | III-2 | 4930459L07Rik | | |
| 932 | 2 | 3 | | | III-2 | 4930474N09Rik | | |
| 942 | 2 | 3 | | | III-2 | 4930483J18Rik | | |
| 957 | 2 | 3 | | | III-2 | 4930502E18Rik | | |
| 970 | 2 | 3 | | | III-2 | 4930507D10Rik | | |
| 978 | 2 | 3 | | | III-2 | 4930513D17Rik | | |
| 981 | 2 | 3 | | | III-2 | 4930515B02Rik | | |
| 996 | 2 | 3 | | | III-2 | 4930521E06Rik | | |
| 1006 | 2 | 3 | | | III-2 | 4930525D18Rik | | |
| 1011 | 2 | 3 | | | III-2 | 4930527F14Rik | | |
| 1020 | 2 | 3 | | | III-2 | 4930532M18Rik | | |
| 1028 | 2 | 3 | | | III-2 | 4930539N22Rik | | |
| 1032 | 2 | 3 | | | III-2 | 4930543E12Rik | | |
| 1062 | 2 | 3 | | | III-2 | 4930557A04Rik | | |
| 1075 | 2 | 3 | | | III-2 | 4930564B18Rik | | |
| 1077 | 2 | 3 | | | III-2 | 4930564D02Rik | | |
| 1079 | 2 | 3 | | | III-2 | 4930565N06Rik | | |
| 1082 | 2 | 3 | | | III-2 | 4930567J20Rik | | |
| 1097 | 2 | 3 | | | III-2 | 4930578I06Rik | | |
| 1106 | 2 | 3 | | | III-2 | 4930583P06Rik | | |
| 1113 | 2 | 3 | | | III-2 | 4930593A02Rik | | |
| 1117 | 2 | 3 | | | III-2 | 4930596D02Rik | | |
| 1123 | 2 | 3 | | | III-2 | 4931403E22Rik | | |
| 1125 | 2 | 3 | | | III-2 | 4931406B18Rik | | |
| 1132 | 2 | 3 | | | III-2 | 4931412M21 | | |
| 1135 | 2 | 3 | | | III-2 | 4931419H13Rik | | |
| 1137 | 2 | 3 | | | III-2 | 4931423N10Rik | | |
| 1140 | 2 | 3 | | | III-2 | 4931429I11Rik | | |
| 1147 | 2 | 3 | | | III-2 | 4931440F15Rik | | |
| 1152 | 2 | 3 | | | III-2 | 4932411N23Rik | | |
| 1166 | 2 | 3 | | | III-2 | 4932443I19Rik | | |
| 1169 | 2 | 3 | | | III-2 | 4933400B14Rik | | |
| 1190 | 2 | 3 | | | III-2 | 4933405E24Rik | | |
| 1209 | 2 | 3 | | | III-2 | 4933408N05Rik | | |
| 1213 | 2 | 3 | | | III-2 | 4933411G06Rik | | |
| 1217 | 2 | 3 | | | III-2 | 4933412E12Rik | | |
| 1219 | 2 | 3 | | | III-2 | 4933412O06Rik | | |
| 1252 | 2 | 3 | | | III-2 | 4933429O19Rik | | |
| 1259 | 2 | 3 | | | III-2 | 4933432G23Rik | | |
| 1279 | 2 | 3 | | | III-2 | 4933440M02Rik | | |
| 1292 | 2 | 3 | | | III-2 | 5330411J11Rik | | |
| 1294 | 2 | 3 | | | III-2 | 5330417C22Rik | | |
| 1305 | 2 | 3 | | | III-2 | 5430417L22Rik | | |
| 1321 | 2 | 3 | | | III-2 | 5730405O15Rik | | |
| 1327 | 2 | 3 | | | III-2 | 5730422E09Rik | | |
| 1329 | 2 | 3 | | | III-2 | 5730455P16Rik | | |

| | | | | | | | | |
|---|---|---|---|---|---|---|---|---|
| 1342 | 2 | 3 | | | III-2 | 5830416P10Rik | | |
| 1343 | 2 | 3 | | | III-2 | 5830417I10Rik | | |
| 1348 | 2 | 3 | | | III-2 | 5830444B04Rik | | |
| 1355 | 2 | 3 | | | III-2 | 6030407O03Rik | | |
| 1359 | 2 | 3 | | | III-2 | 6030443J06Rik | | |
| 1369 | 2 | 3 | | | III-2 | 6330408A02Rik | | |
| 1373 | 2 | 3 | | | III-2 | 6330416G13Rik | | |
| 1377 | 2 | 3 | | | III-2 | 6430411K18Rik | | |
| 1379 | 2 | 3 | | | III-2 | 6430531B16Rik | | |
| 1386 | 2 | 3 | | | III-2 | 6430706D22Rik | | |
| 1393 | 2 | 3 | | | III-2 | 6720489N17Rik | | |
| 1395 | 2 | 3 | | | III-2 | 6820431F20Rik | | |
| 1397 | 2 | 3 | | | III-2 | 7420461P10Rik | | |
| 1416 | 2 | 3 | | | III-2 | 8430436N08Rik | | |
| 1422 | 2 | 3 | | | III-2 | 9030617O03Rik | | |
| 1425 | 2 | 3 | | | III-2 | 9030624J02Rik | | |
| 1429 | 2 | 3 | | | III-2 | 9130015A21Rik | | |
| 1430 | 2 | 3 | | | III-2 | 9130015L21Rik | | |
| 1442 | 2 | 3 | | | III-2 | 9130409I23Rik | | |
| 1457 | 2 | 3 | | | III-2 | 9330111N05Rik | | |
| 1458 | 2 | 3 | | | III-2 | 9330117O12Rik | | |
| 1472 | 2 | 3 | | | III-2 | 9330188P03Rik | | |
| 1482 | 2 | 3 | | | III-2 | 9430037G07Rik | | |
| 1485 | 2 | 3 | | | III-2 | 9430060I03Rik | | |
| 1486 | 2 | 3 | | | III-2 | 9430069I07Rik | | |
| 1496 | 2 | 3 | | | III-2 | 9530051G07Rik | | |
| 1500 | 2 | 3 | | | III-2 | 9530068E07Rik | | |
| 1504 | 2 | 3 | | | III-2 | 9530091C08Rik | | |
| 1512 | 2 | 3 | | | III-2 | 9830147E19Rik | | |
| 1518 | 2 | 3 | | | III-2 | 9930111H07Rik | | |
| 1523 | 2 | 3 | | | III-2 | A130077B15Rik | | |
| 1527 | 2 | 3 | | | III-2 | A230009B12Rik | | |
| 1533 | 2 | 3 | | | III-2 | A230056P14Rik | | |
| 1538 | 2 | 3 | | | III-2 | A230072E10Rik | | |
| 1539 | 2 | 3 | | | III-2 | A230073K19Rik | | |
| 1543 | 2 | 3 | | | III-2 | A2m | 2 | 23-May-15 |
| 1552 | 2 | 3 | | | III-2 | A330048O09Rik | | |
| 1554 | 2 | 3 | | | III-2 | A330050F15Rik | | |
| 1563 | 2 | 3 | | | III-2 | A430005L14Rik | | |
| 1567 | 2 | 3 | | | III-2 | A430088P11Rik | | |
| 1579 | 2 | 3 | | | III-2 | A530046M15Rik | | |
| 1586 | 2 | 3 | | | III-2 | A530072M11Rik | | |
| 1588 | 2 | 3 | | | III-2 | A530099J19Rik | | |
| 1592 | 2 | 3 | | | III-2 | A630012P03Rik | | |
| 1606 | 2 | 3 | | | III-2 | A630095N17Rik | | |
| 1607 | 2 | 3 | | | III-2 | A730006G06Rik | | |
| 1612 | 2 | 3 | | | III-2 | A730020E08Rik | | |
| 1618 | 2 | 3 | | | III-2 | A730082K24Rik | | |
| 1626 | 2 | 3 | | | III-2 | A830019L24Rik | | |
| 1627 | 2 | 3 | | | III-2 | A830052D11Rik | | |
| 1629 | 2 | 3 | | | III-2 | A830082K12Rik | | |
| 1630 | 2 | 3 | | | III-2 | A830082N09Rik | | |
| 1635 | 2 | 3 | | | III-2 | A930004D18Rik | | |
| 1638 | 2 | 3 | | | III-2 | A930006K02Rik | | |
| 1640 | 2 | 3 | | | III-2 | A930009A15Rik | | |
| 1648 | 2 | 3 | | | III-2 | A930018P22Rik | | |
| 1650 | 2 | 3 | | | III-2 | A930024E05Rik | | |
| 1661 | 2 | 3 | | | III-2 | AA543186 | | |
| 1663 | 2 | 3 | | | III-2 | AA545190 | | |
| 1672 | 2 | 3 | | | III-2 | Aadacl3 | 126767 | 12-May-15 |
| 1679 | 2 | 3 | | | III-2 | Aanat | 15 | 12-May-15 |
| 1686 | 2 | 3 | | | III-2 | Aasdhppt | 60496 | 4-May-15 |
| 1690 | 2 | 3 | | | III-2 | AB041803 | | |
| 1695 | 2 | 3 | | | III-2 | Abca13 | 154664 | 12-May-15 |
| 1703 | 2 | 3 | | | III-2 | Abca5 | 23461 | 4-May-15 |
| 1718 | 2 | 3 | | | III-2 | Abcb9 | 23457 | 4-May-15 |
| 1720 | 2 | 3 | | | III-2 | Abcc10 | 89845 | 4-May-15 |
| 1727 | 2 | 3 | | | III-2 | Abcc8 | 6833 | 24-May-15 |
| 1734 | 2 | 3 | | | III-2 | Abcf1 | 23 | 21-May-15 |
| 1754 | 2 | 3 | | | III-2 | Abhd16b | 140701 | 4-May-15 |
| 1769 | 2 | 3 | | | III-2 | Abl2 | 27 | 12-May-15 |
| 1778 | 2 | 3 | | | III-2 | Abtb1 | 80325 | 4-May-15 |
| 1780 | 2 | 3 | | | III-2 | Acaa1a | | |
| 1789 | 2 | 3 | | | III-2 | Acad9 | 28976 | 4-May-15 |
| 1798 | 2 | 3 | | | III-2 | Acap3 | 116983 | 4-May-15 |
| 1805 | 2 | 3 | | | III-2 | Acbd6 | 84320 | 14-May-15 |
| 1813 | 2 | 3 | | | III-2 | Acer1 | 125981 | 21-May-15 |
| 1828 | 2 | 3 | | | III-2 | Aco2 | 50 | 12-May-15 |
| 1838 | 2 | 3 | | | III-2 | Acot6 | 641372 | 4-May-15 |
| 1876 | 2 | 3 | | | III-2 | Actbl2 | 345651 | 4-May-15 |
| 1881 | 2 | 3 | | | III-2 | Actl11 | | |
| 1882 | 2 | 3 | | | III-2 | Actl6a | 86 | 3-May-15 |
| 1885 | 2 | 3 | | | III-2 | Actl7b | 10880 | 12-May-15 |
| 1891 | 2 | 3 | | | III-2 | Actr10 | 55860 | 4-May-15 |
| 1907 | 2 | 3 | | | III-2 | Acvr2b | 93 | 12-May-15 |
| 1917 | 2 | 3 | | | III-2 | Adam10 | 102 | 24-May-15 |
| 1918 | 2 | 3 | | | III-2 | Adam11 | 4185 | 4-May-15 |
| 1923 | 2 | 3 | | | III-2 | Adam19 | 8728 | 4-May-15 |
| 1926 | 2 | 3 | | | III-2 | Adam2 | 2515 | 4-May-15 |
| 1927 | 2 | 3 | | | III-2 | Adam20 | 8748 | 4-May-15 |
| 1932 | 2 | 3 | | | III-2 | Adam25 | | |
| 1955 | 2 | 3 | | | III-2 | Adamts14 | 140766 | 10-May-15 |
| 1958 | 2 | 3 | | | III-2 | Adamts17 | 170691 | 17-May-15 |
| 1960 | 2 | 3 | | | III-2 | Adamts19 | 171019 | 4-May-15 |
| 1963 | 2 | 3 | | | III-2 | Adamts3 | 9508 | May-15 |
| 1985 | 2 | 3 | | | III-2 | Adck2 | 90956 | 4-May-15 |
| 2004 | 2 | 3 | | | III-2 | Adgb | 79747 | 4-May-15 |

Fig.22 - 40

| | | | | | | | | |
|---|---|---|---|---|---|---|---|---|
| 2016 | 2 | 3 | | | III-2 | Adipor2 | 79602 | 31-May-15 |
| 2021 | 2 | 3 | | | III-2 | Adnp2 | 22850 | 4-May-15 |
| 2025 | 2 | 3 | | | III-2 | Adora2b | 136 | 24-May-15 |
| 2028 | 2 | 3 | | | III-2 | Adprh | 141 | 12-May-15 |
| 2031 | 2 | 3 | | | III-2 | Adprm | 56985 | 4-May-15 |
| 2042 | 2 | 3 | | | III-2 | Adrbk2 | 157 | 14-May-15 |
| 2045 | 2 | 3 | | | III-2 | Adss | 159 | 4-May-15 |
| 2053 | 2 | 3 | | | III-2 | AF067063 | | |
| 2056 | 2 | 3 | | | III-2 | AF357359 | | |
| 2066 | 2 | 3 | | | III-2 | Aff2 | 2334 | 12-May-15 |
| 2069 | 2 | 3 | | | III-2 | Afg3l1 | 172 | 12-May-15 |
| 2074 | 2 | 3 | | | III-2 | Aftph | 54812 | 4-May-15 |
| 2075 | 2 | 3 | | | III-2 | Aga | 175 | 12-May-15 |
| 2079 | 2 | 3 | | | III-2 | Agbl1 | 123624 | 23-May-15 |
| 2083 | 2 | 3 | | | III-2 | Agbl5 | 60509 | 23-May-15 |
| 2085 | 2 | 3 | | | III-2 | Agfg1 | 3267 | 4-May-15 |
| 2095 | 2 | 3 | | | III-2 | Ago4 | 192670 | 4-May-15 |
| 2099 | 2 | 3 | | | III-2 | Agpat4 | 56895 | 4-May-15 |
| 2101 | 2 | 3 | | | III-2 | Agpat6 | 137964 | 4-May-15 |
| 2112 | 2 | 3 | | | III-2 | Agtr2 | 186 | 4-May-15 |
| 2114 | 2 | 3 | | | III-2 | Agxt | 189 | 23-May-15 |
| 2116 | 2 | 3 | | | III-2 | Ahctf1 | 25909 | 4-May-15 |
| 2119 | 2 | 3 | | | III-2 | Ahcyl2 | 23382 | 3-May-15 |
| 2124 | 2 | 3 | | | III-2 | Ahrr | 57491 | 10-Jun-15 |
| 2125 | 2 | 3 | | | III-2 | Ahsa1 | 10598 | 4-May-15 |
| 2126 | 2 | 3 | | | III-2 | Ahsa2 | 130872 | 4-May-15 |
| 2132 | 2 | 3 | | | III-2 | AI314180 | | |
| 2137 | 2 | 3 | | | III-2 | AI427809 | | |
| 2144 | 2 | 3 | | | III-2 | AI504432 | | |
| 2148 | 2 | 3 | | | III-2 | AI597479 | | |
| 2156 | 2 | 3 | | | III-2 | AI839979 | | |
| 2158 | 2 | 3 | | | III-2 | AI847159 | | |
| 2161 | 2 | 3 | | | III-2 | AI854703 | | |
| 2172 | 2 | 3 | | | III-2 | Aim1l | 55057 | 4-May-15 |
| 2175 | 2 | 3 | | | III-2 | Aimp2 | 7965 | 4-May-15 |
| 2181 | 2 | 3 | | | III-2 | Ajuba | 84962 | 4-May-15 |
| 2186 | 2 | 3 | | | III-2 | Ak3 | 50808 | 7-Jun-15 |
| 2198 | 2 | 3 | | | III-2 | Akap17b | | |
| 2206 | 2 | 3 | | | III-2 | Akap8l | 26993 | 12-May-15 |
| 2210 | 2 | 3 | | | III-2 | Akirin2 | 55122 | 4-May-15 |
| 2227 | 2 | 3 | | | III-2 | Akr1cl | | |
| 2228 | 2 | 3 | | | III-2 | Akr1d1 | 6718 | 4-May-15 |
| 2232 | 2 | 3 | | | III-2 | Akt1s1 | 84335 | 4-May-15 |
| 2242 | 2 | 3 | | | III-2 | Aldh18a1 | 5832 | 23-May-15 |
| 2260 | 2 | 3 | | | III-2 | Aldh9a1 | 223 | 23-May-15 |
| 2269 | 2 | 3 | | | III-2 | Alg12 | 79087 | 23-May-15 |
| 2271 | 2 | 3 | | | III-2 | Alg14 | 199857 | 5-May-15 |
| 2272 | 2 | 3 | | | III-2 | Alg2 | 85365 | 13-Jun-15 |
| 2273 | 2 | 3 | | | III-2 | Alg3 | 10195 | 22-May-15 |
| 2275 | 2 | 3 | | | III-2 | Alg6 | 29929 | 23-May-15 |
| 2278 | 2 | 3 | | | III-2 | Alk | 238 | 26-May-15 |
| 2282 | 2 | 3 | | | III-2 | Alkbh4 | 54784 | 4-May-15 |
| 2284 | 2 | 3 | | | III-2 | Alkbh6 | 84964 | 4-May-15 |
| 2287 | 2 | 3 | | | III-2 | Allc | 55821 | 20-May-15 |
| 2288 | 2 | 3 | | | III-2 | Alms1 | 7840 | 22-May-15 |
| 2292 | 2 | 3 | | | III-2 | Alox12e | 245 | 4-May-15 |
| 2309 | 2 | 3 | | | III-2 | Alx3 | 257 | 12-May-15 |
| 2321 | 2 | 3 | | | III-2 | Amelx | 265 | 12-May-15 |
| 2322 | 2 | 3 | | | III-2 | Amer1 | 139285 | 4-May-15 |
| 2324 | 2 | 3 | | | III-2 | Amer3 | 205147 | 4-May-15 |
| 2325 | 2 | 3 | | | III-2 | Amfr | 267 | 29-May-15 |
| 2334 | 2 | 3 | | | III-2 | Amn | 81693 | 7-Jun-15 |
| 2351 | 2 | 3 | | | III-2 | Anapc1 | 64682 | 12-May-15 |
| 2352 | 2 | 3 | | | III-2 | Anapc10 | 10393 | 4-May-15 |
| 2354 | 2 | 3 | | | III-2 | Anapc13 | 25847 | 4-May-15 |
| 2357 | 2 | 3 | | | III-2 | Anapc2 | 29882 | 4-May-15 |
| 2366 | 2 | 3 | | | III-2 | Ang6 | | |
| 2383 | 2 | 3 | | | III-2 | Ankdd1b | 728780 | 4-May-15 |
| 2385 | 2 | 3 | | | III-2 | Ankfn1 | 162282 | 4-May-15 |
| 2386 | 2 | 3 | | | III-2 | Ankfy1 | 51479 | 4-May-15 |
| 2392 | 2 | 3 | | | III-2 | Ankmy1 | 51281 | 21-May-15 |
| 2404 | 2 | 3 | | | III-2 | Ankrd17 | 26057 | 4-May-15 |
| 2410 | 2 | 3 | | | III-2 | Ankrd27 | 84079 | 4-May-15 |
| 2414 | 2 | 3 | | | III-2 | Ankrd33 | 341405 | 4-May-15 |
| 2415 | 2 | 3 | | | III-2 | Ankrd33b | 651746 | 4-May-15 |
| 2417 | 2 | 3 | | | III-2 | Ankrd34b | 340120 | 4-May-15 |
| 2418 | 2 | 3 | | | III-2 | Ankrd34c | 390616 | 4-May-15 |
| 2423 | 2 | 3 | | | III-2 | Ankrd40 | 91369 | 21-May-15 |
| 2424 | 2 | 3 | | | III-2 | Ankrd42 | 338699 | 12-May-15 |
| 2432 | 2 | 3 | | | III-2 | Ankrd54 | 129138 | 4-May-15 |
| 2435 | 2 | 3 | | | III-2 | Ankrd60 | 140731 | 12-May-15 |
| 2442 | 2 | 3 | | | III-2 | Anks1b | 56899 | 4-May-15 |
| 2443 | 2 | 3 | | | III-2 | Anks3 | 124401 | 12-May-15 |
| 2451 | 2 | 3 | | | III-2 | Ano2 | 57101 | 4-May-15 |
| 2452 | 2 | 3 | | | III-2 | Ano3 | 63982 | 21-May-15 |
| 2456 | 2 | 3 | | | III-2 | Ano7 | 50636 | 4-May-15 |
| 2466 | 2 | 3 | | | III-2 | Anxa1 | 301 | 24-May-15 |
| 2468 | 2 | 3 | | | III-2 | Anxa11 | 311 | 10-May-15 |
| 2475 | 2 | 3 | | | III-2 | Anxa7 | 310 | 4-May-15 |
| 2487 | 2 | 3 | | | III-2 | Ap1b1 | 162 | 12-May-15 |
| 2496 | 2 | 3 | | | III-2 | Ap2a2 | 161 | 4-May-15 |
| 2507 | 2 | 3 | | | III-2 | Ap4b1 | 10717 | 12-May-15 |
| 2509 | 2 | 3 | | | III-2 | Ap4m1 | 9179 | 4-May-15 |
| 2511 | 2 | 3 | | | III-2 | Ap5b1 | 91056 | 4-May-15 |
| 2513 | 2 | 3 | | | III-2 | Ap5s1 | 55317 | 4-May-15 |
| 2514 | 2 | 3 | | | III-2 | Ap5z1 | 9907 | 4-May-15 |
| 2515 | 2 | 3 | | | III-2 | Apaf1 | 317 | 12-May-15 |

| | | | | | | | | |
|---|---|---|---|---|---|---|---|---|
| 2517 | 2 | 3 | | | III-2 | Apba2 | 321 | 12-May-15 |
| 2522 | 2 | 3 | | | III-2 | Apbb3 | 10307 | 4-May-15 |
| 2528 | 2 | 3 | | | III-2 | Apela | 100506013 | 12-May-15 |
| 2531 | 2 | 3 | | | III-2 | Aph1a | 51107 | 4-May-15 |
| 2532 | 2 | 3 | | | III-2 | Aph1b | 83464 | 4-May-15 |
| 2534 | 2 | 3 | | | III-2 | Api5 | 8539 | 4-May-15 |
| 2538 | 2 | 3 | | | III-2 | Apln | 8862 | 17-May-15 |
| 2542 | 2 | 3 | | | III-2 | Apmap | 57136 | 4-May-15 |
| 2569 | 2 | 3 | | | III-2 | Apol7c | | |
| 2570 | 2 | 3 | | | III-2 | Apol7d | | |
| 2577 | 2 | 3 | | | III-2 | Apon | | |
| 2579 | 2 | 3 | | | III-2 | Apool | 139322 | 4-May-15 |
| 2585 | 2 | 3 | | | III-2 | Appl2 | 55198 | 7-Jun-15 |
| 2602 | 2 | 3 | | | III-2 | Arap1 | 116985 | 4-May-15 |
| 2609 | 2 | 3 | | | III-2 | Arf1 | 375 | 7-Jun-15 |
| 2626 | 2 | 3 | | | III-2 | Arhgap1 | 392 | 4-May-15 |
| 2630 | 2 | 3 | | | III-2 | Arhgap15 | 55843 | 4-May-15 |
| 2632 | 2 | 3 | | | III-2 | Arhgap17 | 55114 | 4-May-15 |
| 2638 | 2 | 3 | | | III-2 | Arhgap22 | 58504 | 14-May-15 |
| 2644 | 2 | 3 | | | III-2 | Arhgap27os3 | | |
| 2645 | 2 | 3 | | | III-2 | Arhgap28 | 79822 | 31-May-15 |
| 2647 | 2 | 3 | | | III-2 | Arhgap30 | 257106 | 12-May-15 |
| 2652 | 2 | 3 | | | III-2 | Arhgap35 | 2909 | 17-May-15 |
| 2654 | 2 | 3 | | | III-2 | Arhgap39 | 80728 | 21-May-15 |
| 2664 | 2 | 3 | | | III-2 | Arhgdib | 397 | 17-May-15 |
| 2667 | 2 | 3 | | | III-2 | Arhgef10 | 9639 | 12-May-15 |
| 2670 | 2 | 3 | | | III-2 | Arhgef12 | 23365 | 12-May-15 |
| 2675 | 2 | 3 | | | III-2 | Arhgef19 | 128272 | 4-May-15 |
| 2677 | 2 | 3 | | | III-2 | Arhgef25 | 115557 | 4-May-15 |
| 2678 | 2 | 3 | | | III-2 | Arhgef26 | 26084 | 4-May-15 |
| 2681 | 2 | 3 | | | III-2 | Arhgef33 | 100271715 | 12-May-15 |
| 2684 | 2 | 3 | | | III-2 | Arhgef39 | 84904 | 4-May-15 |
| 2690 | 2 | 3 | | | III-2 | Arhgef9 | 23229 | 23-May-15 |
| 2695 | 2 | 3 | | | III-2 | Arid3b | 10620 | 28-May-15 |
| 2702 | 2 | 3 | | | III-2 | Arih2 | 10425 | 4-May-15 |
| 2707 | 2 | 3 | | | III-2 | Arl13b | 200894 | 23-May-15 |
| 2713 | 2 | 3 | | | III-2 | Arl2 | 402 | 4-May-15 |
| 2725 | 2 | 3 | | | III-2 | Arl6ip5 | 10550 | 12-May-15 |
| 2735 | 2 | 3 | | | III-2 | Armc4 | 55130 | 28-May-15 |
| 2736 | 2 | 3 | | | III-2 | Armc5 | 79798 | 4-May-15 |
| 2739 | 2 | 3 | | | III-2 | Armc8 | 25852 | 4-May-15 |
| 2740 | 2 | 3 | | | III-2 | Armc9 | 80210 | 12-May-15 |
| 2744 | 2 | 3 | | | III-2 | Armcx4 | 100131755 | 12-May-15 |
| 2761 | 2 | 3 | | | III-2 | Arrb1 | 408 | 17-May-15 |
| 2773 | 2 | 3 | | | III-2 | Arsk | 153642 | 23-May-15 |
| 2788 | 2 | 3 | | | III-2 | Asah2 | 56624 | 4-May-15 |
| 2805 | 2 | 3 | | | III-2 | Asb4 | 51666 | 4-May-15 |
| 2808 | 2 | 3 | | | III-2 | Asb7 | 140460 | 4-May-15 |
| 2814 | 2 | 3 | | | III-2 | Ascl1 | 429 | 23-May-15 |
| 2816 | 2 | 3 | | | III-2 | Ascl3 | 56676 | 4-May-15 |
| 2819 | 2 | 3 | | | III-2 | Asf1a | 25842 | 4-May-15 |
| 2823 | 2 | 3 | | | III-2 | Ash1l | 55870 | 4-May-15 |
| 2841 | 2 | 3 | | | III-2 | Aspm | 259266 | 23-May-15 |
| 2845 | 2 | 3 | | | III-2 | Asrgl1 | 80150 | 12-May-15 |
| 2848 | 2 | 3 | | | III-2 | Astl | 431705 | 4-May-15 |
| 2851 | 2 | 3 | | | III-2 | Asun | 55726 | 4-May-15 |
| 2855 | 2 | 3 | | | III-2 | Asz1 | 136991 | 4-May-15 |
| 2859 | 2 | 3 | | | III-2 | Atad3a | 55210 | 4-May-15 |
| 2866 | 2 | 3 | | | III-2 | Atf1 | 466 | 7-Jun-15 |
| 2872 | 2 | 3 | | | III-2 | Atf6b | 1388 | 4-May-15 |
| 2876 | 2 | 3 | | | III-2 | Atg10 | 83734 | 4-May-15 |
| 2878 | 2 | 3 | | | III-2 | Atg12 | 9140 | 21-May-15 |
| 2879 | 2 | 3 | | | III-2 | Atg13 | 9776 | 28-May-15 |
| 2880 | 2 | 3 | | | III-2 | Atg14 | 22863 | 28-May-15 |
| 2884 | 2 | 3 | | | III-2 | Atg2b | 55102 | 21-May-15 |
| 2888 | 2 | 3 | | | III-2 | Atg4c | 84938 | 21-May-15 |
| 2895 | 2 | 3 | | | III-2 | Atic | 471 | 4-May-15 |
| 2896 | 2 | 3 | | | III-2 | Atl1 | 51062 | 7-Jun-15 |
| 2900 | 2 | 3 | | | III-2 | Atmin | 23300 | 4-May-15 |
| 2901 | 2 | 3 | | | III-2 | Atn1 | 1822 | 23-May-15 |
| 2903 | 2 | 3 | | | III-2 | Atoh7 | 220202 | 23-May-15 |
| 2909 | 2 | 3 | | | III-2 | Atp11a | 23250 | 4-May-15 |
| 2911 | 2 | 3 | | | III-2 | Atp11c | 286410 | 4-May-15 |
| 2914 | 2 | 3 | | | III-2 | Atp13a2 | 23400 | 23-May-15 |
| 2922 | 2 | 3 | | | III-2 | Atp1b1 | 481 | 12-May-15 |
| 2934 | 2 | 3 | | | III-2 | Atp2c2 | 9914 | 4-May-15 |
| 2937 | 2 | 3 | | | III-2 | Atp5a1 | 498 | 12-May-15 |
| 2943 | 2 | 3 | | | III-2 | Atp5g1 | 516 | 4-May-15 |
| 2953 | 2 | 3 | | | III-2 | Atp5sl | 55101 | 4-May-15 |
| 2975 | 2 | 3 | | | III-2 | Atp6v1f | 9296 | 4-May-15 |
| 2983 | 2 | 3 | | | III-2 | Atp8a2 | 51761 | 4-May-15 |
| 2984 | 2 | 3 | | | III-2 | Atp8b1 | 5205 | 23-May-15 |
| 2986 | 2 | 3 | | | III-2 | Atp8b3 | 148229 | 4-May-15 |
| 2991 | 2 | 3 | | | III-2 | Atpaf1 | 64756 | 4-May-15 |
| 2996 | 2 | 3 | | | III-2 | Atrip | 84126 | 4-May-15 |
| 2999 | 2 | 3 | | | III-2 | Atrx | 546 | 23-May-15 |
| 3000 | 2 | 3 | | | III-2 | Atxn1 | 6310 | 23-May-15 |
| 3004 | 2 | 3 | | | III-2 | Atxn2l | 11273 | 21-May-15 |
| 3009 | 2 | 3 | | | III-2 | Atxn7l3 | 56970 | 12-May-15 |
| 3010 | 2 | 3 | | | III-2 | Atxn7l3b | 552889 | 12-May-15 |
| 3031 | 2 | 3 | | | III-2 | Aurka | 6790 | 24-May-15 |
| 3051 | 2 | 3 | | | III-2 | AW209491 | | |
| 3055 | 2 | 3 | | | III-2 | AW551984 | | |
| 3056 | 2 | 3 | | | III-2 | AW554918 | | |
| 3066 | 2 | 3 | | | III-2 | AY512931 | | |

Fig.22 - 41

| | | | | | | | | |
|---|---|---|---|---|---|---|---|---|
| 3072 | 2 | 3 | | | III-2 | Azin1 | 51582 | 4-May-15 |
| 3074 | 2 | 3 | | | III-2 | B020004J07Rik | | |
| 3088 | 2 | 3 | | | III-2 | B230216G23Rik | | |
| 3090 | 2 | 3 | | | III-2 | B230217C12Rik | | |
| 3092 | 2 | 3 | | | III-2 | B230219D22Rik | | |
| 3093 | 2 | 3 | | | III-2 | B230312C02Rik | | |
| 3095 | 2 | 3 | | | III-2 | B230323A14Rik | | |
| 3107 | 2 | 3 | | | III-2 | B3gat3 | 26229 | 23-May-15 |
| 3108 | 2 | 3 | | | III-2 | B3glct | 145173 | 23-May-15 |
| 3111 | 2 | 3 | | | III-2 | B3gnt3 | 10331 | 4-May-15 |
| 3118 | 2 | 3 | | | III-2 | B3gntl1 | 146712 | 4-May-15 |
| 3127 | 2 | 3 | | | III-2 | B4galt1 | 2683 | 23-May-15 |
| 3134 | 2 | 3 | | | III-2 | B630005N14Rik | | |
| 3139 | 2 | 3 | | | III-2 | B930025P03Rik | | |
| 3143 | 2 | 3 | | | III-2 | B9d1 | 27077 | 4-May-15 |
| 3146 | 2 | 3 | | | III-2 | Baat | 570 | 4-May-15 |
| 3153 | 2 | 3 | | | III-2 | Bad | 572 | 4-May-15 |
| 3157 | 2 | 3 | | | III-2 | Bag4 | 9530 | 3-May-15 |
| 3162 | 2 | 3 | | | III-2 | Bai1 | 575 | 4-May-15 |
| 3173 | 2 | 3 | | | III-2 | Banf2 | 140836 | 4-May-15 |
| 3174 | 2 | 3 | | | III-2 | Bank1 | 55024 | 4-May-15 |
| 3176 | 2 | 3 | | | III-2 | Bap1 | 8314 | 7-Jun-15 |
| 3179 | 2 | 3 | | | III-2 | Barhl2 | 343472 | 28-May-15 |
| 3189 | 2 | 3 | | | III-2 | Baz2a | 11176 | 4-May-15 |
| 3200 | 2 | 3 | | | III-2 | Bbox1 | 8424 | 12-May-15 |
| 3202 | 2 | 3 | | | III-2 | Bbs10 | 79738 | 23-May-15 |
| 3210 | 2 | 3 | | | III-2 | BC002163 | | |
| 3212 | 2 | 3 | | | III-2 | BC003965 | | |
| 3217 | 2 | 3 | | | III-2 | BC005764 | | |
| 3238 | 2 | 3 | | | III-2 | BC027231 | | |
| 3242 | 2 | 3 | | | III-2 | BC030307 | | |
| 3253 | 2 | 3 | | | III-2 | BC037034 | | |
| 3256 | 2 | 3 | | | III-2 | BC039966 | | |
| 3262 | 2 | 3 | | | III-2 | BC048602 | | |
| 3271 | 2 | 3 | | | III-2 | BC049762 | | |
| 3273 | 2 | 3 | | | III-2 | BC051142 | | |
| 3276 | 2 | 3 | | | III-2 | BC051628 | | |
| 3280 | 2 | 3 | | | III-2 | BC053393 | | |
| 3283 | 2 | 3 | | | III-2 | BC055324 | | |
| 3285 | 2 | 3 | | | III-2 | BC061194 | | |
| 3286 | 2 | 3 | | | III-2 | BC061195 | | |
| 3291 | 2 | 3 | | | III-2 | BC068157 | | |
| 3294 | 2 | 3 | | | III-2 | BC089491 | | |
| 3298 | 2 | 3 | | | III-2 | BC100451 | | |
| 3303 | 2 | 3 | | | III-2 | Bcam | 4059 | 7-Jun-15 |
| 3311 | 2 | 3 | | | III-2 | Bcas2 | 10286 | 4-May-15 |
| 3323 | 2 | 3 | | | III-2 | Bcl10 | 8915 | 12-May-15 |
| 3332 | 2 | 3 | | | III-2 | Bcl2l10 | 10017 | 12-May-15 |
| 3344 | 2 | 3 | | | III-2 | Bcl7c | 9274 | 4-May-15 |
| 3346 | 2 | 3 | | | III-2 | Bcl9l | 283149 | 4-May-15 |
| 3351 | 2 | 3 | | | III-2 | Bcorl1 | 63035 | 21-May-15 |
| 3353 | 2 | 3 | | | III-2 | Bcs1l | 617 | 23-May-15 |
| 3359 | 2 | 3 | | | III-2 | Bdp1 | 55814 | 7-Jun-15 |
| 3361 | 2 | 3 | | | III-2 | Becn1 | 8678 | 31-May-15 |
| 3362 | 2 | 3 | | | III-2 | Becn2 | 441925 | 4-May-15 |
| 3365 | 2 | 3 | | | III-2 | Bend4 | 389206 | 4-May-15 |
| 3368 | 2 | 3 | | | III-2 | Bend7 | 222389 | 4-May-15 |
| 3373 | 2 | 3 | | | III-2 | Bet1l | 51272 | 4-May-15 |
| 3379 | 2 | 3 | | | III-2 | Bfsp1 | 631 | 4-May-15 |
| 3386 | 2 | 3 | | | III-2 | Bhlha9 | 727857 | 4-May-15 |
| 3389 | 2 | 3 | | | III-2 | Bhlhe23 | 128408 | 4-May-15 |
| 3390 | 2 | 3 | | | III-2 | Bhlhe40 | 8553 | 4-May-15 |
| 3394 | 2 | 3 | | | III-2 | Bicc1 | 80114 | 4-May-15 |
| 3396 | 2 | 3 | | | III-2 | Bicd2 | 23299 | 4-May-15 |
| 3397 | 2 | 3 | | | III-2 | Bid | 637 | 17-May-15 |
| 3402 | 2 | 3 | | | III-2 | Birc2 | 329 | 24-May-15 |
| 3406 | 2 | 3 | | | III-2 | Birc7 | 79444 | 23-May-15 |
| 3407 | 2 | 3 | | | III-2 | Bivm | 54841 | 4-May-15 |
| 3412 | 2 | 3 | | | III-2 | Blnk | 29760 | 17-May-15 |
| 3414 | 2 | 3 | | | III-2 | Bloc1s2 | 282991 | 4-May-15 |
| 3424 | 2 | 3 | | | III-2 | Bmp1 | 649 | 12-May-15 |
| 3429 | 2 | 3 | | | III-2 | Bmp3 | 651 | 12-May-15 |
| 3445 | 2 | 3 | | | III-2 | Bnip1 | 662 | 4-May-15 |
| 3458 | 2 | 3 | | | III-2 | Bop1 | 23246 | 4-May-15 |
| 3470 | 2 | 3 | | | III-2 | Bpifb3 | 359710 | 4-May-15 |
| 3474 | 2 | 3 | | | III-2 | Bpifb9a | | |
| 3476 | 2 | 3 | | | III-2 | Bpifc | 254240 | 4-May-15 |
| 3480 | 2 | 3 | | | III-2 | Brap | 8315 | 7-Jun-15 |
| 3485 | 2 | 3 | | | III-2 | Brd1 | 23774 | 12-May-15 |
| 3486 | 2 | 3 | | | III-2 | Brd2 | 6046 | 12-May-15 |
| 3492 | 2 | 3 | | | III-2 | Brdt | 676 | 4-May-15 |
| 3495 | 2 | 3 | | | III-2 | Brf2 | 55290 | 7-Jun-15 |
| 3501 | 2 | 3 | | | III-2 | Brinp3 | 339479 | 4-May-15 |
| 3504 | 2 | 3 | | | III-2 | Brk1 | 55845 | 4-May-15 |
| 3516 | 2 | 3 | | | III-2 | Bsdc1 | 55108 | 4-May-15 |
| 3518 | 2 | 3 | | | III-2 | Bsn | 8927 | 4-May-15 |
| 3527 | 2 | 3 | | | III-2 | Btbd1 | 53339 | 4-May-15 |
| 3531 | 2 | 3 | | | III-2 | Btbd17 | 388419 | 4-May-15 |
| 3535 | 2 | 3 | | | III-2 | Btbd3 | 22903 | 4-May-15 |
| 3539 | 2 | 3 | | | III-2 | Btbd9 | 114781 | 4-May-15 |
| 3542 | 2 | 3 | | | III-2 | Btf3 | 689 | 7-Jun-15 |
| 3545 | 2 | 3 | | | III-2 | Btg2 | 7832 | 26-May-15 |
| 3548 | 2 | 3 | | | III-2 | Btk | 695 | 22-May-15 |
| 3551 | 2 | 3 | | | III-2 | Btn2a2 | 10385 | 4-May-15 |
| 3555 | 2 | 3 | | | III-2 | Btnl4 | | |
| 3557 | 2 | 3 | | | III-2 | Btnl6 | | |
| 3560 | 2 | 3 | | | III-2 | Bub1 | 699 | 12-May-15 |
| 3563 | 2 | 3 | | | III-2 | Bud13 | 84811 | 12-May-15 |
| 3569 | 2 | 3 | | | III-2 | Bzw2 | 28969 | 4-May-15 |
| 3574 | 2 | 3 | | | III-2 | C030018K13Rik | | |
| 3576 | 2 | 3 | | | III-2 | C030029H02Rik | | |
| 3581 | 2 | 3 | | | III-2 | C030046E11Rik | | |
| 3583 | 2 | 3 | | | III-2 | C130026I21Rik | | |
| 3584 | 2 | 3 | | | III-2 | C130026L21Rik | | |
| 3590 | 2 | 3 | | | III-2 | C130060K24Rik | | |
| 3604 | 2 | 3 | | | III-2 | C1ql2 | 165257 | 4-May-15 |
| 3606 | 2 | 3 | | | III-2 | C1ql4 | 338761 | 4-May-15 |
| 3618 | 2 | 3 | | | III-2 | C1s1 | | |
| 3621 | 2 | 3 | | | III-2 | C230004F18Rik | | |
| 3622 | 2 | 3 | | | III-2 | C230024C17Rik | | |
| 3628 | 2 | 3 | | | III-2 | C230091D08Rik | | |
| 3631 | 2 | 3 | | | III-2 | C2cd3 | 26005 | 12-May-15 |
| 3639 | 2 | 3 | | | III-2 | C330007P06Rik | | |
| 3643 | 2 | 3 | | | III-2 | C330018D20Rik | | |
| 3644 | 2 | 3 | | | III-2 | C330021F23Rik | | |
| 3652 | 2 | 3 | | | III-2 | C430002N11Rik | | |
| 3661 | 2 | 3 | | | III-2 | C5ar1 | 728 | 12-May-15 |
| 3665 | 2 | 3 | | | III-2 | C630031E19Rik | | |
| 3669 | 2 | 3 | | | III-2 | C730027H18Rik | | |
| 3675 | 2 | 3 | | | III-2 | C86695 | | |
| 3685 | 2 | 3 | | | III-2 | C920006O11Rik | | |
| 3689 | 2 | 3 | | | III-2 | Caap1 | 79886 | 12-May-15 |
| 3691 | 2 | 3 | | | III-2 | Cab39l | 81617 | 4-May-15 |
| 3706 | 2 | 3 | | | III-2 | Cacna1c | 775 | 23-May-15 |
| 3710 | 2 | 3 | | | III-2 | Cacna1g | 8913 | 4-May-15 |
| 3717 | 2 | 3 | | | III-2 | Cacna2d4 | 93589 | 4-May-15 |
| 3720 | 2 | 3 | | | III-2 | Cacnb3 | 784 | 4-May-15 |
| 3724 | 2 | 3 | | | III-2 | Cacng3 | 10368 | 4-May-15 |
| 3726 | 2 | 3 | | | III-2 | Cacng5 | 27091 | 4-May-15 |
| 3731 | 2 | 3 | | | III-2 | Cacul1 | 143384 | 4-May-15 |
| 3736 | 2 | 3 | | | III-2 | Cadm3 | 57863 | 4-May-15 |
| 3751 | 2 | 3 | | | III-2 | Calhm2 | 51063 | 12-May-15 |
| 3753 | 2 | 3 | | | III-2 | Calm2 | 805 | 12-May-15 |
| 3765 | 2 | 3 | | | III-2 | Camk1 | 8536 | 7-Jun-15 |
| 3772 | 2 | 3 | | | III-2 | Camk2n1 | 55450 | 4-May-15 |
| 3779 | 2 | 3 | | | III-2 | Caml | 819 | 4-May-15 |
| 3782 | 2 | 3 | | | III-2 | Camsap2 | 23271 | 4-May-15 |
| 3786 | 2 | 3 | | | III-2 | Cand1 | 55832 | 4-May-15 |
| 3789 | 2 | 3 | | | III-2 | Canx | 821 | 17-May-15 |
| 3806 | 2 | 3 | | | III-2 | Capns1 | 826 | 12-May-15 |
| 3813 | 2 | 3 | | | III-2 | Capza2 | 830 | 4-May-15 |
| 3814 | 2 | 3 | | | III-2 | Capza3 | 93661 | 4-May-15 |
| 3818 | 2 | 3 | | | III-2 | Car11 | | |
| 3840 | 2 | 3 | | | III-2 | Carm1 | 10498 | 3-May-15 |
| 3850 | 2 | 3 | | | III-2 | Cask | 8573 | 4-May-15 |
| 3857 | 2 | 3 | | | III-2 | Casp3 | 836 | |
| 3858 | 2 | 3 | | | III-2 | Casp4 | 837 | |
| 3861 | 2 | 3 | | | III-2 | Casp8 | 841 | 23-May-15 |
| 3862 | 2 | 3 | | | III-2 | Casp8ap2 | 9994 | 12-May-15 |
| 3872 | 2 | 3 | | | III-2 | Catsper1 | 117144 | 23-May-15 |
| 3873 | 2 | 3 | | | III-2 | Catsper2 | 117155 | 23-May-15 |
| 3877 | 2 | 3 | | | III-2 | Catsperd | 257062 | 4-May-15 |
| 3884 | 2 | 3 | | | III-2 | Cbfa2t3 | 863 | 4-May-15 |
| 3901 | 2 | 3 | | | III-2 | Cbx2 | 84733 | 4-May-15 |
| 3904 | 2 | 3 | | | III-2 | Cbx5 | 23468 | 4-May-15 |
| 3906 | 2 | 3 | | | III-2 | Cbx7 | 23492 | 21-May-15 |
| 3909 | 2 | 3 | | | III-2 | Cc2d1a | 54862 | 31-May-15 |
| 3910 | 2 | 3 | | | III-2 | Cc2d1b | 200014 | 28-May-15 |
| 3913 | 2 | 3 | | | III-2 | Ccar2 | 57805 | 29-May-15 |
| 3930 | 2 | 3 | | | III-2 | Ccdc114 | 93233 | 4-May-15 |
| 3939 | 2 | 3 | | | III-2 | Ccdc125 | 202243 | 4-May-15 |
| 3940 | 2 | 3 | | | III-2 | Ccdc126 | 90693 | 4-May-15 |
| 3951 | 2 | 3 | | | III-2 | Ccdc14 | 64770 | 12-May-15 |
| 3952 | 2 | 3 | | | III-2 | Ccdc141 | 285025 | 4-May-15 |
| 3957 | 2 | 3 | | | III-2 | Ccdc148 | 130940 | 12-May-15 |
| 3961 | 2 | 3 | | | III-2 | Ccdc151 | 115948 | 28-May-15 |
| 3965 | 2 | 3 | | | III-2 | Ccdc155 | 147872 | 14-May-15 |
| 3971 | 2 | 3 | | | III-2 | Ccdc163 | | |
| 3972 | 2 | 3 | | | III-2 | Ccdc166 | 100130274 | 4-May-15 |
| 3973 | 2 | 3 | | | III-2 | Ccdc167 | 154467 | 4-May-15 |
| 3975 | 2 | 3 | | | III-2 | Ccdc17 | 149483 | |
| 3984 | 2 | 3 | | | III-2 | Ccdc178 | 374864 | 12-May-15 |
| 3987 | 2 | 3 | | | III-2 | Ccdc183 | 84960 | |
| 4002 | 2 | 3 | | | III-2 | Ccdc34 | 91057 | |
| 4013 | 2 | 3 | | | III-2 | Ccdc50 | 152137 | 23-May-15 |
| 4017 | 2 | 3 | | | III-2 | Ccdc55 | 84081 | 4-May-15 |
| 4023 | 2 | 3 | | | III-2 | Ccdc61 | 729440 | 4-May-15 |
| 4028 | 2 | 3 | | | III-2 | Ccdc65 | 85478 | 4-May-15 |
| 4034 | 2 | 3 | | | III-2 | Ccdc70 | 83446 | 4-May-15 |
| 4040 | 2 | 3 | | | III-2 | Ccdc78 | 124093 | 4-May-15 |
| 4045 | 2 | 3 | | | III-2 | Ccdc82 | 79780 | 4-May-15 |
| 4052 | 2 | 3 | | | III-2 | Ccdc87 | 55231 | 4-May-15 |
| 4057 | 2 | 3 | | | III-2 | Ccdc9 | 26093 | 4-May-15 |
| 4058 | 2 | 3 | | | III-2 | Ccdc90b | 60492 | 4-May-15 |
| 4062 | 2 | 3 | | | III-2 | Ccdc94 | 55702 | 4-May-15 |
| 4064 | 2 | 3 | | | III-2 | Ccdc97 | 90324 | 4-May-15 |
| 4085 | 2 | 3 | | | III-2 | Ccl27a | | |
| 4096 | 2 | 3 | | | III-2 | Ccm2l | 140706 | 4-May-15 |
| 4098 | 2 | 3 | | | III-2 | Ccna2 | 890 | 24-May-15 |
| 4101 | 2 | 3 | | | III-2 | Ccnb2 | 9133 | 12-May-15 |
| 4103 | 2 | 3 | | | III-2 | Ccnc | 892 | 17-May-15 |
| 4107 | 2 | 3 | | | III-2 | Ccndbp1 | 23582 | 17-May-15 |
| 4115 | 2 | 3 | | | III-2 | Ccnj | 54619 | 4-May-15 |
| 4123 | 2 | 3 | | | III-2 | Ccny | 219771 | 4-May-15 |

Fig.22 - 42

| | | | | | | | | |
|---|---|---|---|---|---|---|---|---|
| 4127 | 2 | 3 | | | III-2 | Ccpg1os | | |
| 4144 | 2 | 3 | | | III-2 | Ccser2 | 54462 | 4-May-15 |
| 4146 | 2 | 3 | | | III-2 | Cct3 | 7203 | 4-May-15 |
| 4171 | 2 | 3 | | | III-2 | Cd200r1 | 131450 | |
| 4173 | 2 | 3 | | | III-2 | Cd200r3 | | |
| 4181 | 2 | 3 | | | III-2 | Cd209f | | |
| 4185 | 2 | 3 | | | III-2 | Cd244 | 51744 | |
| 4186 | 2 | 3 | | | III-2 | Cd247 | 919 | 21-May-15 |
| 4219 | 2 | 3 | | | III-2 | Cd47 | 961 | 31-May-15 |
| 4239 | 2 | 3 | | | III-2 | Cd81 | 975 | 31-May-15 |
| 4249 | 2 | 3 | | | III-2 | Cd97 | 976 | |
| 4253 | 2 | 3 | | | III-2 | Cdan1 | 146059 | 23-May-15 |
| 4258 | 2 | 3 | | | III-2 | Cdc20 | 991 | |
| 4260 | 2 | 3 | | | III-2 | Cdc23 | 8697 | 4-May-15 |
| 4277 | 2 | 3 | | | III-2 | Cdc42ep4 | 23580 | 4-May-15 |
| 4280 | 2 | 3 | | | III-2 | Cdc42se2 | 56990 | 4-May-15 |
| 4285 | 2 | 3 | | | III-2 | Cdc73 | 79577 | 23-May-15 |
| 4289 | 2 | 3 | | | III-2 | Cdca5 | 113130 | 17-May-15 |
| 4299 | 2 | 3 | | | III-2 | Cdh13 | 1012 | 17-May-15 |
| 4309 | 2 | 3 | | | III-2 | Cdh24 | 64403 | 7-Jun-15 |
| 4314 | 2 | 3 | | | III-2 | Cdh6 | 1004 | 12-May-15 |
| 4316 | 2 | 3 | | | III-2 | Cdh8 | 1006 | 12-May-15 |
| 4317 | 2 | 3 | | | III-2 | Cdh9 | 1007 | 4-May-15 |
| 4323 | 2 | 3 | | | III-2 | Cdipt | 10423 | 4-May-15 |
| 4326 | 2 | 3 | | | III-2 | Cdk11b | 984 | 12-May-15 |
| 4327 | 2 | 3 | | | III-2 | Cdk12 | 51755 | 21-May-15 |
| 4329 | 2 | 3 | | | III-2 | Cdk14 | 5218 | |
| 4331 | 2 | 3 | | | III-2 | Cdk16 | 5127 | 31-May-15 |
| 4336 | 2 | 3 | | | III-2 | Cdk20 | 23552 | 4-May-15 |
| 4337 | 2 | 3 | | | III-2 | Cdk2ap1 | 8099 | 4-May-15 |
| 4341 | 2 | 3 | | | III-2 | Cdk5 | 1020 | 27-May-15 |
| 4343 | 2 | 3 | | | III-2 | Cdk5r2 | 8941 | 4-May-15 |
| 4344 | 2 | 3 | | | III-2 | Cdk5rap1 | 51654 | 4-May-15 |
| 4346 | 2 | 3 | | | III-2 | Cdk5rap3 | 80279 | 12-May-15 |
| 4349 | 2 | 3 | | | III-2 | Cdk8 | 1024 | 17-May-15 |
| 4354 | 2 | 3 | | | III-2 | Cdkl3 | 51265 | 4-May-15 |
| 4380 | 2 | 3 | | | III-2 | Cdx1 | 1044 | 28-May-15 |
| 4397 | 2 | 3 | | | III-2 | Ceacam3 | 1084 | 4-May-15 |
| 4406 | 2 | 3 | | | III-2 | Cebpz | 10153 | 7-Jun-15 |
| 4416 | 2 | 3 | | | III-2 | Celf2 | 10659 | |
| 4418 | 2 | 3 | | | III-2 | Celf4 | 56853 | |
| 4425 | 2 | 3 | | | III-2 | Cemip | 57214 | 12-May-15 |
| 4429 | 2 | 3 | | | III-2 | Cenpc1 | 1060 | 4-May-15 |
| 4446 | 2 | 3 | | | III-2 | Cep104 | 9731 | 4-May-15 |
| 4449 | 2 | 3 | | | III-2 | Cep128 | 145508 | 12-May-15 |
| 4452 | 2 | 3 | | | III-2 | Cep152 | 22995 | |
| 4453 | 2 | 3 | | | III-2 | Cep162 | 22832 | 4-May-15 |
| 4454 | 2 | 3 | | | III-2 | Cep164 | 22897 | 31-May-15 |
| 4460 | 2 | 3 | | | III-2 | Cep290 | 80184 | 29-May-15 |
| 4461 | 2 | 3 | | | III-2 | Cep350 | 9857 | 12-May-15 |
| 4463 | 2 | 3 | | | III-2 | Cep44 | 80817 | 4-May-15 |
| 4478 | 2 | 3 | | | III-2 | Cep95 | 90799 | 12-May-15 |
| 4479 | 2 | 3 | | | III-2 | Cep97 | 79598 | 12-May-15 |
| 4484 | 2 | 3 | | | III-2 | Cerkl | 375298 | 23-May-15 |
| 4487 | 2 | 3 | | | III-2 | Cers3 | 204219 | 4-May-15 |
| 4492 | 2 | 3 | | | III-2 | Ces1b | | |
| 4523 | 2 | 3 | | | III-2 | Cfl2 | 1073 | 23-May-15 |
| 4525 | 2 | 3 | | | III-2 | Cfp | 5199 | 17-May-15 |
| 4533 | 2 | 3 | | | III-2 | Ch25h | 9023 | 4-May-15 |
| 4538 | 2 | 3 | | | III-2 | Chaf1a | 10036 | |
| 4539 | 2 | 3 | | | III-2 | Chaf1b | 8208 | |
| 4541 | 2 | 3 | | | III-2 | Chat | 1103 | 23-May-15 |
| 4547 | 2 | 3 | | | III-2 | Chchd5 | 84269 | 4-May-15 |
| 4551 | 2 | 3 | | | III-2 | Chd1l | 9557 | 12-May-15 |
| 4552 | 2 | 3 | | | III-2 | Chd2 | 1106 | 7-Jun-15 |
| 4555 | 2 | 3 | | | III-2 | Chd4 | 1108 | 12-May-15 |
| 4556 | 2 | 3 | | | III-2 | Chd5 | 26038 | 7-Jun-15 |
| 4562 | 2 | 3 | | | III-2 | Chek1 | 1111 | |
| 4564 | 2 | 3 | | | III-2 | Cherp | 10523 | 4-May-15 |
| 4565 | 2 | 3 | | | III-2 | Chfr | 55743 | 23-May-15 |
| 4570 | 2 | 3 | | | III-2 | Chic2 | 26511 | 4-May-15 |
| 4576 | 2 | 3 | | | III-2 | Chit1 | 1118 | 4-May-15 |
| 4579 | 2 | 3 | | | III-2 | ChkbCpt1b | 386593 | 4-May-15 |
| 4584 | 2 | 3 | | | III-2 | Chmp1b | 57132 | 4-May-15 |
| 4591 | 2 | 3 | | | III-2 | Chmp6 | 79643 | 3-May-15 |
| 4595 | 2 | 3 | | | III-2 | Chn2 | 1124 | 12-May-15 |
| 4598 | 2 | 3 | | | III-2 | Chp1 | 11261 | 7-Jun-15 |
| 4607 | 2 | 3 | | | III-2 | Chrm1 | 1128 | 4-May-15 |
| 4618 | 2 | 3 | | | III-2 | Chrna6 | 8973 | 12-May-15 |
| 4621 | 2 | 3 | | | III-2 | Chrnb1 | 1140 | 7-May-15 |
| 4634 | 2 | 3 | | | III-2 | Chst15 | 51363 | 12-May-15 |
| 4644 | 2 | 3 | | | III-2 | Chtf18 | 63922 | |
| 4646 | 2 | 3 | | | III-2 | Chtop | 26097 | 4-May-15 |
| 4664 | 2 | 3 | | | III-2 | Cipc | 85457 | 4-May-15 |
| 4673 | 2 | 3 | | | III-2 | Cit | 11113 | /6/7 |
| 4679 | 2 | 3 | | | III-2 | Ckap2 | 26586 | 4-May-15 |
| 4682 | 2 | 3 | | | III-2 | Ckap5 | 9793 | 4-May-15 |
| 4690 | 2 | 3 | | | III-2 | Cks2 | 1164 | 12-May-15 |
| 4692 | 2 | 3 | | | III-2 | Clasp2 | 23122 | 28-May-15 |
| 4710 | 2 | 3 | | | III-2 | Clcnkb | 1188 | |
| 4721 | 2 | 3 | | | III-2 | Cldn19 | 149461 | 1-Jun-15 |
| 4728 | 2 | 3 | | | III-2 | Cldn26 | | |
| 4743 | 2 | 3 | | | III-2 | Clec18a | 348174 | 4-May-15 |
| 4749 | 2 | 3 | | | III-2 | Clec2g | | |
| 4764 | 2 | 3 | | | III-2 | Clec4g | 339390 | |
| 4770 | 2 | 3 | | | III-2 | Clhc1 | 130162 | |
| 4782 | 2 | 3 | | | III-2 | Clk2 | 1196 | 7-Jun-15 |
| 4795 | 2 | 3 | | | III-2 | Clpb | 81570 | 31-May-15 |
| 4810 | 2 | 3 | | | III-2 | Cltb | 1212 | 4-May-15 |
| 4817 | 2 | 3 | | | III-2 | Clybl | 171425 | |
| 4826 | 2 | 3 | | | III-2 | Cmklr1 | 1240 | |
| 4832 | 2 | 3 | | | III-2 | Cmpk2 | 129607 | |
| 4835 | 2 | 3 | | | III-2 | Cmtm2a | | |
| 4836 | 2 | 3 | | | III-2 | Cmtm2b | | |
| 4841 | 2 | 3 | | | III-2 | Cmtm7 | 112616 | 4-May-15 |
| 4842 | 2 | 3 | | | III-2 | Cmtm8 | 152189 | 4-May-15 |
| 4844 | 2 | 3 | | | III-2 | Cmtr2 | 55783 | 12-May-15 |
| 4847 | 2 | 3 | | | III-2 | Cnbp | 7555 | 23-May-15 |
| 4856 | 2 | 3 | | | III-2 | Cngb1 | 1258 | 23-May-15 |
| 4861 | 2 | 3 | | | III-2 | Cnih4 | 29097 | 21-May-15 |
| 4867 | 2 | 3 | | | III-2 | Cnn3 | 1266 | 4-May-15 |
| 4871 | 2 | 3 | | | III-2 | Cnnm4 | 26504 | 12-May-15 |
| 4874 | 2 | 3 | | | III-2 | Cnot11 | 55571 | 4-May-15 |
| 4877 | 2 | 3 | | | III-2 | Cnot4 | 4850 | 3-Jun-15 |
| 4879 | 2 | 3 | | | III-2 | Cnot6l | 246175 | 3-Jun-15 |
| 4880 | 2 | 3 | | | III-2 | Cnot7 | 29883 | 16-May-15 |
| 4881 | 2 | 3 | | | III-2 | Cnot8 | 9337 | 2-Jun-15 |
| 4883 | 2 | 3 | | | III-2 | Cnppd1 | 27013 | 12-May-15 |
| 4884 | 2 | 3 | | | III-2 | Cnpy1 | 285888 | 4-May-15 |
| 4887 | 2 | 3 | | | III-2 | Cnpy4 | 245812 | 4-May-15 |
| 4888 | 2 | 3 | | | III-2 | Cnr1 | 1268 | 7-Jun-15 |
| 4889 | 2 | 3 | | | III-2 | Cnr2 | 1269 | 17-May-15 |
| 4896 | 2 | 3 | | | III-2 | Cntn1 | 1272 | 4-May-15 |
| 4904 | 2 | 3 | | | III-2 | Cntnap3 | 79937 | 4-May-15 |
| 4908 | 2 | 3 | | | III-2 | Cntnap5c | | |
| 4909 | 2 | 3 | | | III-2 | Cntrl | 11064 | 4-May-15 |
| 4911 | 2 | 3 | | | III-2 | Coa3 | 28958 | 4-May-15 |
| 4912 | 2 | 3 | | | III-2 | Coa4 | 51287 | 4-May-15 |
| 4914 | 2 | 3 | | | III-2 | Coa6 | 388753 | 21-May-15 |
| 4916 | 2 | 3 | | | III-2 | Coasy | 80347 | 4-May-15 |
| 4921 | 2 | 3 | | | III-2 | Cog2 | 22796 | 4-May-15 |
| 4922 | 2 | 3 | | | III-2 | Cog3 | 83548 | 4-May-15 |
| 4934 | 2 | 3 | | | III-2 | Col14a1 | 7373 | |
| 4937 | 2 | 3 | | | III-2 | Col17a1 | 1308 | 24-May-15 |
| 4938 | 2 | 3 | | | III-2 | Col18a1 | 80781 | |
| 4943 | 2 | 3 | | | III-2 | Col22a1 | 169044 | 12-May-15 |
| 4948 | 2 | 3 | | | III-2 | Col27a1 | 85301 | |
| 4956 | 2 | 3 | | | III-2 | Col4a4 | 1286 | 23-May-15 |
| 4972 | 2 | 3 | | | III-2 | Col9a2 | 1298 | 23-May-15 |
| 4978 | 2 | 3 | | | III-2 | Colq | 8292 | |
| 4980 | 2 | 3 | | | III-2 | Commd10 | 51397 | 12-May-15 |
| 4985 | 2 | 3 | | | III-2 | Commd6 | 170622 | 4-May-15 |
| 4987 | 2 | 3 | | | III-2 | Commd8 | 54951 | 4-May-15 |
| 4991 | 2 | 3 | | | III-2 | Comtd1 | 118881 | 4-May-15 |
| 4993 | 2 | 3 | | | III-2 | Copb1 | 1315 | 4-May-15 |
| 5002 | 2 | 3 | | | III-2 | Cops5 | 10987 | 4-May-15 |
| 5015 | 2 | 3 | | | III-2 | Coq6 | 51004 | 23-May-15 |
| 5016 | 2 | 3 | | | III-2 | Coq7 | 10229 | 28-May-15 |
| 5021 | 2 | 3 | | | III-2 | Coro1c | 23603 | 4-May-15 |
| 5026 | 2 | 3 | | | III-2 | Cort | 1325 | |
| 5029 | 2 | 3 | | | III-2 | Cox11 | 1353 | 7-Jun-15 |
| 5036 | 2 | 3 | | | III-2 | Cox20 | 116228 | 4-May-15 |
| 5049 | 2 | 3 | | | III-2 | Cox7b | 1349 | 15-May-15 |
| 5064 | 2 | 3 | | | III-2 | Cpd | 1362 | 7-Jun-15 |
| 5065 | 2 | 3 | | | III-2 | Cpe | 1363 | 12-May-15 |
| 5073 | 2 | 3 | | | III-2 | Cplx1 | 10815 | 4-May-15 |
| 5081 | 2 | 3 | | | III-2 | Cpne2 | 221184 | |
| 5083 | 2 | 3 | | | III-2 | Cpne4 | 131034 | 4-May-15 |
| 5091 | 2 | 3 | | | III-2 | Cpq | 10404 | 4-May-15 |
| 5094 | 2 | 3 | | | III-2 | Cpsf2 | 53981 | 4-May-15 |
| 5101 | 2 | 3 | | | III-2 | Cpt1a | 1374 | 23-May-15 |
| 5105 | 2 | 3 | | | III-2 | Cpvl | 54504 | 23-May-15 |
| 5106 | 2 | 3 | | | III-2 | Cpxcr1 | 53336 | 4-May-15 |
| 5118 | 2 | 3 | | | III-2 | Crb2 | 286204 | 12-May-15 |
| 5121 | 2 | 3 | | | III-2 | Crcp | 27297 | 4-May-15 |
| 5132 | 2 | 3 | | | III-2 | Crebrf | 153222 | 4-May-15 |
| 5155 | 2 | 3 | | | III-2 | Crkl | 1399 | 29-May-15 |
| 5159 | 2 | 3 | | | III-2 | Crls1 | 54675 | 4-May-15 |
| 5163 | 2 | 3 | | | III-2 | Crnn | 49860 | 4-May-15 |
| 5166 | 2 | 3 | | | III-2 | Crp | 1401 | #NAME? |
| 5169 | 2 | 3 | | | III-2 | Crtap | 10491 | 4-May-15 |
| 5172 | 2 | 3 | | | III-2 | Crtc3 | 64784 | 12-May-15 |
| 5186 | 2 | 3 | | | III-2 | Cryga | 1418 | 4-May-15 |
| 5194 | 2 | 3 | | | III-2 | Cryl1 | 51084 | 4-May-15 |
| 5197 | 2 | 3 | | | III-2 | Cryzl1 | 9946 | 4-May-15 |
| 5202 | 2 | 3 | | | III-2 | Cse1l | 1434 | 12-May-15 |
| 5206 | 2 | 3 | | | III-2 | Csf2ra | 1438 | 4-May-15 |
| 5213 | 2 | 3 | | | III-2 | Csk | 1445 | 3-May-15 |
| 5225 | 2 | 3 | | | III-2 | Csnk1d | 1453 | 29-May-15 |
| 5236 | 2 | 3 | | | III-2 | Cspp1 | 79848 | 12-May-15 |
| 5237 | 2 | 3 | | | III-2 | Csprs | | |
| 5258 | 2 | 3 | | | III-2 | Cstf2 | 1478 | 12-May-15 |
| 5260 | 2 | 3 | | | III-2 | Cstf3 | 1479 | 4-May-15 |
| 5269 | 2 | 3 | | | III-2 | Ctcfl | 140690 | 4-May-15 |
| 5270 | 2 | 3 | | | III-2 | Ctcflos | | |
| 5272 | 2 | 3 | | | III-2 | Ctdp1 | 9150 | 23-May-15 |
| 5277 | 2 | 3 | | | III-2 | Ctf1 | 1489 | 12-May-15 |
| 5291 | 2 | 3 | | | III-2 | Ctnnbip1 | 56998 | 14-May-15 |
| 5298 | 2 | 3 | | | III-2 | Ctr9 | 9646 | 12-May-15 |
| 5304 | 2 | 3 | | | III-2 | Cts6 | | |
| 5339 | 2 | 3 | | | III-2 | Cul2 | 8453 | 12-May-15 |
| 5344 | 2 | 3 | | | III-2 | Cul7 | 9820 | 23-May-15 |
| 5347 | 2 | 3 | | | III-2 | Cutal | | |
| 5369 | 2 | 3 | | | III-2 | Cxcl16 | 58191 | |

Fig.22 - 43

| | | | | | | | | |
|---|---|---|---|---|---|---|---|---|
| 5371 | 2 | 3 | | | III-2 | Cxcl2 | 2920 | 12-May-15 |
| 5373 | 2 | 3 | | | III-2 | Cxcl5 | 6374 | |
| 5382 | 2 | 3 | | | III-2 | Cxx1b | 26071 | 4-May-15 |
| 5383 | 2 | 3 | | | III-2 | Cxx1c | 441518 | 4-May-15 |
| 5385 | 2 | 3 | | | III-2 | Cxxc4 | 80319 | 4-May-15 |
| 5399 | 2 | 3 | | | III-2 | Cyb5rl | 606495 | 4-May-15 |
| 5406 | 2 | 3 | | | III-2 | Cyfip1 | 23191 | 17-May-15 |
| 5463 | 2 | 3 | | | III-2 | Cyp2d37-ps | | |
| 5473 | 2 | 3 | | | III-2 | Cyp2j6 | | |
| 5511 | 2 | 3 | | | III-2 | Cyp4f41-ps | | |
| 5519 | 2 | 3 | | | III-2 | Cypt12 | | |
| 5534 | 2 | 3 | | | III-2 | Cyth2 | 9266 | 31-May-15 |
| 5541 | 2 | 3 | | | III-2 | D030024E09Rik | | |
| 5546 | 2 | 3 | | | III-2 | D030045P18Rik | | |
| 5548 | 2 | 3 | | | III-2 | D030056L22Rik | | |
| 5557 | 2 | 3 | | | III-2 | D130043K22Rik | | |
| 5559 | 2 | 3 | | | III-2 | D14Ertd670e | | |
| 5563 | 2 | 3 | | | III-2 | D17Ertd648e | | |
| 5581 | 2 | 3 | | | III-2 | D3Ertd751e | | |
| 5585 | 2 | 3 | | | III-2 | D430041D05Rik | | |
| 5587 | 2 | 3 | | | III-2 | D4Ertd617e | | |
| 5594 | 2 | 3 | | | III-2 | D630013N20Rik | | |
| 5598 | 2 | 3 | | | III-2 | D630032N06Rik | | |
| 5607 | 2 | 3 | | | III-2 | D730001G18Rik | | |
| 5614 | 2 | 3 | | | III-2 | D7Ertd715e | | |
| 5617 | 2 | 3 | | | III-2 | D830015G02Rik | | |
| 5619 | 2 | 3 | | | III-2 | D830030K20Rik | | |
| 5622 | 2 | 3 | | | III-2 | D830046C22Rik | | |
| 5629 | 2 | 3 | | | III-2 | D930020B18Rik | | |
| 5635 | 2 | 3 | | | III-2 | Dab1 | 1600 | 7-Jun-15 |
| 5639 | 2 | 3 | | | III-2 | Dach2 | 117154 | 4-May-15 |
| 5640 | 2 | 3 | | | III-2 | Dact1 | 51339 | 3-May-15 |
| 5647 | 2 | 3 | | | III-2 | Daglb | 221955 | 21-May-15 |
| 5649 | 2 | 3 | | | III-2 | Dalrd3 | 55152 | 4-May-15 |
| 5654 | 2 | 3 | | | III-2 | Dap3 | 7818 | 7-Jun-15 |
| 5655 | 2 | 3 | | | III-2 | Dapk1 | 1612 | 4-May-15 |
| 5661 | 2 | 3 | | | III-2 | Dars2 | 55157 | 23-May-15 |
| 5662 | 2 | 3 | | | III-2 | Daw1 | 164781 | 4-May-15 |
| 5675 | 2 | 3 | | | III-2 | Dbnl | 28988 | 4-May-15 |
| 5679 | 2 | 3 | | | III-2 | Dbt | 1629 | 23-May-15 |
| 5687 | 2 | 3 | | | III-2 | Dcaf13 | 25879 | 4-May-15 |
| 5691 | 2 | 3 | | | III-2 | Dcaf5 | 8816 | 4-May-15 |
| 5706 | 2 | 3 | | | III-2 | Dclk3 | 85443 | 4-May-15 |
| 5708 | 2 | 3 | | | III-2 | Dclre1b | 64858 | 4-May-15 |
| 5712 | 2 | 3 | | | III-2 | Dcp1b | 196513 | 23-May-15 |
| 5718 | 2 | 3 | | | III-2 | Dcst1 | 149095 | 4-May-15 |
| 5726 | 2 | 3 | | | III-2 | Dctn5 | 84516 | 4-May-15 |
| 5731 | 2 | 3 | | | III-2 | Dcun1d3 | 123879 | 3-May-15 |
| 5737 | 2 | 3 | | | III-2 | Ddah1 | 23576 | 4-May-15 |
| 5752 | 2 | 3 | | | III-2 | Ddr1 | 780 | 4-May-15 |
| 5755 | 2 | 3 | | | III-2 | Ddt | 1652 | 4-May-15 |
| 5760 | 2 | 3 | | | III-2 | Ddx18 | 8886 | 4-May-15 |
| 5772 | 2 | 3 | | | III-2 | Ddx39 | 10212 | 4-May-15 |
| 5793 | 2 | 3 | | | III-2 | Ddx60 | 55601 | 4-May-15 |
| 5795 | 2 | 3 | | | III-2 | Dear1 | 55223 | 4-May-15 |
| 5799 | 2 | 3 | | | III-2 | Dedd | 9191 | 4-May-15 |
| 5800 | 2 | 3 | | | III-2 | Dedd2 | 162989 | 4-May-15 |
| 5801 | 2 | 3 | | | III-2 | Def6 | 50619 | /6/7 |
| 5811 | 2 | 3 | | | III-2 | Defa26 | | |
| 5814 | 2 | 3 | | | III-2 | Defa5 | 1670 | 24-May-15 |
| 5867 | 2 | 3 | | | III-2 | Dennd1a | 57706 | 4-May-15 |
| 5868 | 2 | 3 | | | III-2 | Dennd1b | 163486 | 12-May-15 |
| 5869 | 2 | 3 | | | III-2 | Dennd1c | 79958 | 12-May-15 |
| 5878 | 2 | 3 | | | III-2 | Dennd5b | 160518 | 12-May-15 |
| 5882 | 2 | 3 | | | III-2 | Depdc1a | 55635 | 4-May-15 |
| 5888 | 2 | 3 | | | III-2 | Derl1 | 79139 | 29-May-15 |
| 5889 | 2 | 3 | | | III-2 | Derl2 | 51009 | 12-May-15 |
| 5896 | 2 | 3 | | | III-2 | Dffa | 1676 | 4-May-15 |
| 5910 | 2 | 3 | | | III-2 | Dgke | 8526 | 24-May-15 |
| 5911 | 2 | 3 | | | III-2 | Dgkeos | | |
| 5914 | 2 | 3 | | | III-2 | Dgki | 9162 | 4-May-15 |
| 5917 | 2 | 3 | | | III-2 | Dgkz | 8525 | 4-May-15 |
| 5922 | 2 | 3 | | | III-2 | Dhdh | 27294 | 4-May-15 |
| 5924 | 2 | 3 | | | III-2 | Dhh | 50846 | 23-May-15 |
| 5926 | 2 | 3 | | | III-2 | Dhps | 1725 | 12-May-15 |
| 5931 | 2 | 3 | | | III-2 | Dhrs3 | 9249 | 21-May-15 |
| 5933 | 2 | 3 | | | III-2 | Dhrs7 | 51635 | 4-May-15 |
| 5938 | 2 | 3 | | | III-2 | Dhtkd1 | 55526 | 4-May-15 |
| 5940 | 2 | 3 | | | III-2 | Dhx16 | 8449 | 4-May-15 |
| 5941 | 2 | 3 | | | III-2 | Dhx29 | 54505 | 4-May-15 |
| 5943 | 2 | 3 | | | III-2 | Dhx32 | 55760 | 4-May-15 |
| 5945 | 2 | 3 | | | III-2 | Dhx34 | 9704 | 4-May-15 |
| 5947 | 2 | 3 | | | III-2 | Dhx36 | 170506 | 21-May-15 |
| 5948 | 2 | 3 | | | III-2 | Dhx37 | 57647 | 4-May-15 |
| 5954 | 2 | 3 | | | III-2 | Dhx9 | 1660 | 4-May-15 |
| 5960 | 2 | 3 | | | III-2 | Dido1 | 11083 | 4-May-15 |
| 5968 | 2 | 3 | | | III-2 | Dip2b | 57609 | 4-May-15 |
| 5969 | 2 | 3 | | | III-2 | Dip2c | 22982 | 4-May-15 |
| 5975 | 2 | 3 | | | III-2 | Dis3l2 | 129563 | 4-May-15 |
| 5977 | 2 | 3 | | | III-2 | Disp1 | 84976 | 12-May-15 |
| 5982 | 2 | 3 | | | III-2 | Dkk2 | 27123 | 4-May-15 |
| 5993 | 2 | 3 | | | III-2 | Dlg2 | 1740 | 7-Jun-15 |
| 5995 | 2 | 3 | | | III-2 | Dlg4 | 1742 | 7-Jun-15 |
| 6006 | 2 | 3 | | | III-2 | Dll4 | 54567 | 4-May-15 |
| 6008 | 2 | 3 | | | III-2 | Dlx1 | 1745 | 4-May-15 |
| 6015 | 2 | 3 | | | III-2 | Dlx6as2 | 100873931 | 1-Feb-15 |
| 6020 | 2 | 3 | | | III-2 | Dmc1 | 11144 | 7-Jun-15 |

| | | | | | | | | |
|---|---|---|---|---|---|---|---|---|
| 6040 | 2 | 3 | | | III-2 | Dmxl1 | 1657 | 4-May-15 |
| 6042 | 2 | 3 | | | III-2 | Dna2 | 1763 | 3-May-15 |
| 6044 | 2 | 3 | | | III-2 | Dnaaf2 | 55172 | 28-May-15 |
| 6057 | 2 | 3 | | | III-2 | Dnaic1 | | |
| 6061 | 2 | 3 | | | III-2 | Dnaja3 | 9093 | 4-May-15 |
| 6064 | 2 | 3 | | | III-2 | Dnajb11 | 51726 | 4-May-15 |
| 6065 | 2 | 3 | | | III-2 | Dnajb12 | 54788 | 12-May-15 |
| 6073 | 2 | 3 | | | III-2 | Dnajb7 | 150353 | 4-May-15 |
| 6081 | 2 | 3 | | | III-2 | Dnajc14 | 85406 | 4-May-15 |
| 6084 | 2 | 3 | | | III-2 | Dnajc17 | 55192 | 4-May-15 |
| 6092 | 2 | 3 | | | III-2 | Dnajc27 | 51277 | 4-May-15 |
| 6095 | 2 | 3 | | | III-2 | Dnajc30 | 84277 | 4-May-15 |
| 6098 | 2 | 3 | | | III-2 | Dnajc5b | 85479 | 4-May-15 |
| 6104 | 2 | 3 | | | III-2 | Dnal1 | 83544 | 23-May-15 |
| 6116 | 2 | 3 | | | III-2 | Dnm1 | 1759 | 21-May-15 |
| 6123 | 2 | 3 | | | III-2 | Dnmt3a | 1788 | 21-May-15 |
| 6124 | 2 | 3 | | | III-2 | Dnmt3aos | | |
| 6125 | 2 | 3 | | | III-2 | Dnmt3b | 1789 | 24-May-15 |
| 6138 | 2 | 3 | | | III-2 | Dock2 | 1794 | 4-May-15 |
| 6140 | 2 | 3 | | | III-2 | Dock4 | 9732 | 4-May-15 |
| 6146 | 2 | 3 | | | III-2 | Dohh | 83475 | 4-May-15 |
| 6153 | 2 | 3 | | | III-2 | Dok7 | 285489 | 23-May-15 |
| 6155 | 2 | 3 | | | III-2 | Dolpp1 | 57171 | 21-May-15 |
| 6156 | 2 | 3 | | | III-2 | Donson | 29980 | 4-May-15 |
| 6162 | 2 | 3 | | | III-2 | Dpagt1 | 1798 | 23-May-15 |
| 6168 | 2 | 3 | | | III-2 | Dpf1 | 8193 | 2-Jun-15 |
| 6173 | 2 | 3 | | | III-2 | Dph3 | 285381 | 4-May-15 |
| 6176 | 2 | 3 | | | III-2 | Dph7 | 92715 | 12-May-15 |
| 6186 | 2 | 3 | | | III-2 | Dpp9 | 91039 | 4-May-15 |
| 6194 | 2 | 3 | | | III-2 | Dpy19l2 | 283417 | 4-May-15 |
| 6195 | 2 | 3 | | | III-2 | Dpy19l3 | 147991 | 4-May-15 |
| 6205 | 2 | 3 | | | III-2 | DQ267101 | | |
| 6209 | 2 | 3 | | | III-2 | Dram1 | 55332 | 4-May-15 |
| 6214 | 2 | 3 | | | III-2 | Drd1a | 1812 | 28-May-15 |
| 6224 | 2 | 3 | | | III-2 | Dsc1 | 1823 | 7-Jun-15 |
| 6225 | 2 | 3 | | | III-2 | Dsc2 | 1824 | 7-Jun-15 |
| 6231 | 2 | 3 | | | III-2 | Dse | 29940 | 12-May-15 |
| 6234 | 2 | 3 | | | III-2 | Dsg1b | | |
| 6244 | 2 | 3 | | | III-2 | Dstyk | 25778 | 4-May-15 |
| 6252 | 2 | 3 | | | III-2 | Dtwd1 | 56986 | 14-May-15 |
| 6256 | 2 | 3 | | | III-2 | Dtx3 | 196403 | 2-Jun-15 |
| 6257 | 2 | 3 | | | III-2 | Dtx3l | 151636 | 12-May-15 |
| 6260 | 2 | 3 | | | III-2 | Duox1 | 53905 | 24-May-15 |
| 6268 | 2 | 3 | | | III-2 | Dus4l | 11062 | 4-May-15 |
| 6272 | 2 | 3 | | | III-2 | Dusp12 | 11266 | 4-May-15 |
| 6281 | 2 | 3 | | | III-2 | Dusp22 | 56940 | 4-May-15 |
| 6294 | 2 | 3 | | | III-2 | Dux | | |
| 6298 | 2 | 3 | | | III-2 | Dvl1 | 1855 | 7-Jun-15 |
| 6303 | 2 | 3 | | | III-2 | Dydc1 | 143241 | 4-May-15 |
| 6316 | 2 | 3 | | | III-2 | Dynlrb1 | 83658 | 4-May-15 |
| 6319 | 2 | 3 | | | III-2 | Dynlt1b | | |
| 6323 | 2 | 3 | | | III-2 | Dyrk1a | 1859 | 12-May-15 |
| 6328 | 2 | 3 | | | III-2 | Dysf | 8291 | 31-May-15 |
| 6339 | 2 | 3 | | | III-2 | E030018B13Rik | | |
| 6341 | 2 | 3 | | | III-2 | E030019B13Rik | | |
| 6344 | 2 | 3 | | | III-2 | E030030I06Rik | | |
| 6346 | 2 | 3 | | | III-2 | E130006D01Rik | | |
| 6353 | 2 | 3 | | | III-2 | E130201H02Rik | | |
| 6364 | 2 | 3 | | | III-2 | E130317F20Rik | | |
| 6366 | 2 | 3 | | | III-2 | E230016K23Rik | | |
| 6369 | 2 | 3 | | | III-2 | E230025N22Rik | | |
| 6387 | 2 | 3 | | | III-2 | E330021D16Rik | | |
| 6393 | 2 | 3 | | | III-2 | E430025E21Rik | | |
| 6394 | 2 | 3 | | | III-2 | E4f1 | 1877 | 23-May-15 |
| 6395 | 2 | 3 | | | III-2 | E530001F21Rik | | |
| 6397 | 2 | 3 | | | III-2 | Eaf1 | 85403 | 7-Jun-15 |
| 6406 | 2 | 3 | | | III-2 | Ear6 | | |
| 6412 | 2 | 3 | | | III-2 | Ebf3 | 253738 | 7-Jun-15 |
| 6416 | 2 | 3 | | | III-2 | Ebp | 10682 | 7-Jun-15 |
| 6419 | 2 | 3 | | | III-2 | Ece1 | 1889 | 12-May-15 |
| 6441 | 2 | 3 | | | III-2 | Edc4 | 23644 | 4-May-15 |
| 6444 | 2 | 3 | | | III-2 | Edem2 | 55741 | 4-May-15 |
| 6445 | 2 | 3 | | | III-2 | Edem3 | 80267 | 4-May-15 |
| 6446 | 2 | 3 | | | III-2 | Edf1 | 8721 | 4-May-15 |
| 6454 | 2 | 3 | | | III-2 | Eea1 | 8411 | 4-May-15 |
| 6462 | 2 | 3 | | | III-2 | Eef2 | 1938 | 23-May-15 |
| 6470 | 2 | 3 | | | III-2 | Efcab14 | 9813 | 4-May-15 |
| 6471 | 2 | 3 | | | III-2 | Efcab2 | 84288 | 4-May-15 |
| 6476 | 2 | 3 | | | III-2 | Efcab6 | 64800 | 4-May-15 |
| 6479 | 2 | 3 | | | III-2 | Efcab9 | 285588 | 4-May-15 |
| 6484 | 2 | 3 | | | III-2 | Efhc1 | 114327 | 10-May-15 |
| 6486 | 2 | 3 | | | III-2 | Efhd1 | 80303 | 7-May-15 |
| 6495 | 2 | 3 | | | III-2 | Efnb3 | 1949 | 4-May-15 |
| 6496 | 2 | 3 | | | III-2 | Efr3a | 23167 | 4-May-15 |
| 6500 | 2 | 3 | | | III-2 | Eftud2 | 9343 | 23-May-15 |
| 6510 | 2 | 3 | | | III-2 | Egln2 | 112398 | 4-May-15 |
| 6516 | 2 | 3 | | | III-2 | Ehbp1 | 23301 | 4-May-15 |
| 6519 | 2 | 3 | | | III-2 | Ehd2 | 30846 | 12-May-15 |
| 6525 | 2 | 3 | | | III-2 | Ehmt2 | 10919 | 4-May-15 |
| 6538 | 2 | 3 | | | III-2 | Eif2ak2 | 5610 | 31-May-15 |
| 6540 | 2 | 3 | | | III-2 | Eif2ak4 | 440275 | 4-May-15 |
| 6542 | 2 | 3 | | | III-2 | Eif2b2 | 8892 | 23-May-15 |
| 6545 | 2 | 3 | | | III-2 | Eif2b5 | 8893 | 23-May-15 |
| 6546 | 2 | 3 | | | III-2 | Eif2d | 1939 | 21-May-15 |
| 6577 | 2 | 3 | | | III-2 | Eif4g1 | 1981 | 29-May-15 |
| 6581 | 2 | 3 | | | III-2 | Eif5 | 1983 | 4-May-15 |
| 6586 | 2 | 3 | | | III-2 | Elac1 | 55520 | 4-May-15 |

Fig.22 - 44

| | | | | | | | | |
|---|---|---|---|---|---|---|---|---|
| 6590 | 2 | 3 | | | III-2 | Elavl2 | 1993 | 4-May-15 |
| 6597 | 2 | 3 | | | III-2 | Elf5 | 2001 | 4-May-15 |
| 6599 | 2 | 3 | | | III-2 | Elfn2 | 114794 | 4-May-15 |
| 6601 | 2 | 3 | | | III-2 | Elk3 | 2004 | 7-Jun-15 |
| 6604 | 2 | 3 | | | III-2 | Ell2 | 22936 | 4-May-15 |
| 6607 | 2 | 3 | | | III-2 | Elmo2 | 63916 | 4-May-15 |
| 6615 | 2 | 3 | | | III-2 | Elovl1 | 64834 | 4-May-15 |
| 6623 | 2 | 3 | | | III-2 | Elp3 | 55140 | 4-May-15 |
| 6626 | 2 | 3 | | | III-2 | Elp6 | 54859 | 4-May-15 |
| 6630 | 2 | 3 | | | III-2 | Emc10 | 284361 | 4-May-15 |
| 6633 | 2 | 3 | | | III-2 | Emc4 | 51234 | 4-May-15 |
| 6636 | 2 | 3 | | | III-2 | Emc8 | 10328 | 4-May-15 |
| 6640 | 2 | 3 | | | III-2 | Eme1 | 146956 | 4-May-15 |
| 6647 | 2 | 3 | | | III-2 | Eml1 | 2009 | 12-May-15 |
| 6649 | 2 | 3 | | | III-2 | Eml3 | 256364 | 12-May-15 |
| 6650 | 2 | 3 | | | III-2 | Eml4 | 27436 | 17-May-15 |
| 6658 | 2 | 3 | | | III-2 | Emx1 | 2016 | 4-May-15 |
| 6670 | 2 | 3 | | | III-2 | Eng | 2022 | 23-May-15 |
| 6680 | 2 | 3 | | | III-2 | Enoph1 | 58478 | 4-May-15 |
| 6683 | 2 | 3 | | | III-2 | Enpep | 2028 | 12-May-15 |
| 6703 | 2 | 3 | | | III-2 | Eogt | 285203 | 4-May-15 |
| 6707 | 2 | 3 | | | III-2 | Epas1 | 2034 | 17-May-15 |
| 6724 | 2 | 3 | | | III-2 | Epha2 | 1969 | 4-May-15 |
| 6727 | 2 | 3 | | | III-2 | Epha5 | 2044 | 29-May-15 |
| 6730 | 2 | 3 | | | III-2 | Epha8 | 2046 | 3-May-15 |
| 6743 | 2 | 3 | | | III-2 | Epn2 | 22905 | 3-May-15 |
| 6750 | 2 | 3 | | | III-2 | Eps15 | 2060 | 12-May-15 |
| 6752 | 2 | 3 | | | III-2 | Eps8 | 2059 | 4-May-15 |
| 6758 | 2 | 3 | | | III-2 | Epx | 8288 | 12-May-15 |
| 6759 | 2 | 3 | | | III-2 | Epyc | 1833 | 4-May-15 |
| 6761 | 2 | 3 | | | III-2 | Eral1 | 26284 | 4-May-15 |
| 6763 | 2 | 3 | | | III-2 | Eras | 3266 | 10-May-15 |
| 6768 | 2 | 3 | | | III-2 | Erc1 | 23085 | 12-May-15 |
| 6771 | 2 | 3 | | | III-2 | Ercc2 | 2068 | 4-Jun-15 |
| 6772 | 2 | 3 | | | III-2 | Ercc3 | 2071 | 4-Jun-15 |
| 6773 | 2 | 3 | | | III-2 | Ercc4 | 2072 | 23-May-15 |
| 6778 | 2 | 3 | | | III-2 | Ercc8 | 1161 | 23-May-15 |
| 6784 | 2 | 3 | | | III-2 | Ergic2 | 51290 | 4-May-15 |
| 6788 | 2 | 3 | | | III-2 | Eri2 | 112479 | 4-May-15 |
| 6791 | 2 | 3 | | | III-2 | Erich2 | 285141 | 12-May-15 |
| 6792 | 2 | 3 | | | III-2 | Erich3 | 127254 | 12-May-15 |
| 6795 | 2 | 3 | | | III-2 | Erich6 | 131831 | 12-May-15 |
| 6796 | 2 | 3 | | | III-2 | Erlec1 | 27248 | 3-May-15 |
| 6802 | 2 | 3 | | | III-2 | Ermp1 | 79956 | 4-May-15 |
| 6809 | 2 | 3 | | | III-2 | Erp44 | 23071 | 4-May-15 |
| 6817 | 2 | 3 | | | III-2 | Esm1 | 11082 | 12-May-15 |
| 6819 | 2 | 3 | | | III-2 | Esp15 | | |
| 6823 | 2 | 3 | | | III-2 | Esp24 | | |
| 6837 | 2 | 3 | | | III-2 | Esr1 | 2099 | 31-May-15 |
| 6842 | 2 | 3 | | | III-2 | Esrrb | 2103 | 23-May-15 |
| 6845 | 2 | 3 | | | III-2 | Esyt1 | 23344 | 4-May-15 |
| 6853 | 2 | 3 | | | III-2 | Etfdh | 2110 | 12-May-15 |
| 6870 | 2 | 3 | | | III-2 | Eva1a | 84141 | 4-May-15 |
| 6877 | 2 | 3 | | | III-2 | Evi2b | 2124 | 4-May-15 |
| 6879 | 2 | 3 | | | III-2 | Evi5l | 115704 | 12-May-15 |
| 6887 | 2 | 3 | | | III-2 | Exo1 | 9156 | 12-May-15 |
| 6889 | 2 | 3 | | | III-2 | Exoc1 | 55763 | 7-Jun-15 |
| 6890 | 2 | 3 | | | III-2 | Exoc2 | 55770 | 3-May-15 |
| 6894 | 2 | 3 | | | III-2 | Exoc4 | 60412 | 4-May-15 |
| 6896 | 2 | 3 | | | III-2 | Exoc6 | 54536 | 4-May-15 |
| 6897 | 2 | 3 | | | III-2 | Exoc6b | 23233 | 4-May-15 |
| 6898 | 2 | 3 | | | III-2 | Exoc7 | 23265 | 3-May-15 |
| 6900 | 2 | 3 | | | III-2 | Exog | 9941 | 3-May-15 |
| 6904 | 2 | 3 | | | III-2 | Exosc3 | 51010 | 23-May-15 |
| 6913 | 2 | 3 | | | III-2 | Ext2 | 2132 | 7-Jun-15 |
| 6916 | 2 | 3 | | | III-2 | Extl3 | 2137 | 4-May-15 |
| 6920 | 2 | 3 | | | III-2 | Eya4 | 2070 | 23-May-15 |
| 6927 | 2 | 3 | | | III-2 | F12 | 2161 | 31-May-15 |
| 6938 | 2 | 3 | | | III-2 | F630028O10Rik | | |
| 6940 | 2 | 3 | | | III-2 | F630111L10Rik | | |
| 6949 | 2 | 3 | | | III-2 | F8a | 8263 | 4-May-15 |
| 6964 | 2 | 3 | | | III-2 | Fads1 | 3992 | 12-May-15 |
| 6969 | 2 | 3 | | | III-2 | Faf2 | 23197 | 12-May-15 |
| 6980 | 2 | 3 | | | III-2 | Fam103a1 | 83640 | 4-May-15 |
| 6981 | 2 | 3 | | | III-2 | Fam104a | 84923 | 4-May-15 |
| 6993 | 2 | 3 | | | III-2 | Fam115a | 9747 | 4-May-15 |
| 7001 | 2 | 3 | | | III-2 | Fam120aos | 158293 | 4-May-15 |
| 7003 | 2 | 3 | | | III-2 | Fam120c | 54954 | 4-May-15 |
| 7005 | 2 | 3 | | | III-2 | Fam122b | 159090 | 12-May-15 |
| 7007 | 2 | 3 | | | III-2 | Fam124a | 220108 | 4-May-15 |
| 7029 | 2 | 3 | | | III-2 | Fam149a | 25854 | 4-May-15 |
| 7031 | 2 | 3 | | | III-2 | Fam150a | 389658 | 4-May-15 |
| 7032 | 2 | 3 | | | III-2 | Fam150b | 285016 | 4-May-15 |
| 7036 | 2 | 3 | | | III-2 | Fam154b | 283726 | 4-May-15 |
| 7039 | 2 | 3 | | | III-2 | Fam159b | 100132916 | 4-May-15 |
| 7040 | 2 | 3 | | | III-2 | Fam160a1 | 729830 | 4-May-15 |
| 7042 | 2 | 3 | | | III-2 | Fam160b1 | 57700 | 4-May-15 |
| 7051 | 2 | 3 | | | III-2 | Fam166b | 730112 | 4-May-15 |
| 7055 | 2 | 3 | | | III-2 | Fam168b | 130074 | 21-May-15 |
| 7059 | 2 | 3 | | | III-2 | Fam170b | 170370 | 4-May-15 |
| 7064 | 2 | 3 | | | III-2 | Fam173a | 65990 | 21-May-15 |
| 7069 | 2 | 3 | | | III-2 | Fam175b | 23172 | 4-May-15 |
| 7070 | 2 | 3 | | | III-2 | Fam178a | 55719 | 12-May-15 |
| 7076 | 2 | 3 | | | III-2 | Fam181b | 220382 | 4-May-15 |
| 7085 | 2 | 3 | | | III-2 | Fam188b | 84182 | 4-May-15 |
| 7087 | 2 | 3 | | | III-2 | Fam189a2 | 9413 | 4-May-15 |
| 7089 | 2 | 3 | | | III-2 | Fam192a | 80011 | 4-May-15 |

| | | | | | | | | |
|---|---|---|---|---|---|---|---|---|
| 7094 | 2 | 3 | | | III-2 | Fam196a | 642938 | 4-May-15 |
| 7096 | 2 | 3 | | | III-2 | Fam198a | 729085 | 4-May-15 |
| 7108 | 2 | 3 | | | III-2 | Fam208a | 23272 | 4-May-15 |
| 7110 | 2 | 3 | | | III-2 | Fam209 | | |
| 7115 | 2 | 3 | | | III-2 | Fam210a | 125228 | 12-May-15 |
| 7125 | 2 | 3 | | | III-2 | Fam217a | 222826 | 4-May-15 |
| 7128 | 2 | 3 | | | III-2 | Fam219aos | | |
| 7135 | 2 | 3 | | | III-2 | Fam227a | 646851 | 4-May-15 |
| 7139 | 2 | 3 | | | III-2 | Fam229a | 100128071 | 21-May-15 |
| 7147 | 2 | 3 | | | III-2 | Fam35a | 54537 | 4-May-15 |
| 7149 | 2 | 3 | | | III-2 | Fam3b | 54097 | 12-May-15 |
| 7153 | 2 | 3 | | | III-2 | Fam45a | 404636 | 4-May-15 |
| 7158 | 2 | 3 | | | III-2 | Fam47c | 442444 | 4-May-15 |
| 7163 | 2 | 3 | | | III-2 | Fam50b | 26240 | 4-May-15 |
| 7170 | 2 | 3 | | | III-2 | Fam60a | 58516 | 12-May-15 |
| 7175 | 2 | 3 | | | III-2 | Fam65b | 9750 | 4-May-15 |
| 7180 | 2 | 3 | | | III-2 | Fam71a | 149647 | 12-May-15 |
| 7185 | 2 | 3 | | | III-2 | Fam71f1 | 84691 | 4-May-15 |
| 7191 | 2 | 3 | | | III-2 | Fam76b | 143684 | 4-May-15 |
| 7194 | 2 | 3 | | | III-2 | Fam81a | 145773 | 4-May-15 |
| 7195 | 2 | 3 | | | III-2 | Fam83a | 84985 | 4-May-15 |
| 7198 | 2 | 3 | | | III-2 | Fam83d | 81610 | 12-May-15 |
| 7200 | 2 | 3 | | | III-2 | Fam83f | 113828 | 12-May-15 |
| 7208 | 2 | 3 | | | III-2 | Fam92a | | |
| 7210 | 2 | 3 | | | III-2 | Fam96a | 84191 | 4-May-15 |
| 7213 | 2 | 3 | | | III-2 | Fam98b | 283742 | 4-May-15 |
| 7217 | 2 | 3 | | | III-2 | Fancb | 2187 | 23-May-15 |
| 7219 | 2 | 3 | | | III-2 | Fancd2 | 2177 | 23-May-15 |
| 7222 | 2 | 3 | | | III-2 | Fancf | 2188 | 28-May-15 |
| 7224 | 2 | 3 | | | III-2 | Fanci | 55215 | 23-May-15 |
| 7227 | 2 | 3 | | | III-2 | Fank1 | 92565 | 12-May-15 |
| 7228 | 2 | 3 | | | III-2 | Fap | 2191 | 7-Jun-15 |
| 7240 | 2 | 3 | | | III-2 | Fastkd1 | 79675 | 4-May-15 |
| 7249 | 2 | 3 | | | III-2 | Fau | 2197 | 12-May-15 |
| 7252 | 2 | 3 | | | III-2 | Fbl | 2091 | 4-May-15 |
| 7264 | 2 | 3 | | | III-2 | Fbrsl1 | 57666 | 4-May-15 |
| 7271 | 2 | 3 | | | III-2 | Fbxl17 | 64839 | 4-May-15 |
| 7276 | 2 | 3 | | | III-2 | Fbxl21 | 26223 | 4-May-15 |
| 7279 | 2 | 3 | | | III-2 | Fbxl4 | 26235 | 4-May-15 |
| 7295 | 2 | 3 | | | III-2 | Fbxo27 | 126433 | 4-May-15 |
| 7296 | 2 | 3 | | | III-2 | Fbxo28 | 23219 | 4-May-15 |
| 7297 | 2 | 3 | | | III-2 | Fbxo3 | 26273 | 31-May-15 |
| 7302 | 2 | 3 | | | III-2 | Fbxo34 | 55030 | 4-May-15 |
| 7305 | 2 | 3 | | | III-2 | Fbxo39 | 162517 | 4-May-15 |
| 7309 | 2 | 3 | | | III-2 | Fbxo42 | 54455 | 4-May-15 |
| 7313 | 2 | 3 | | | III-2 | Fbxo46 | 23403 | 4-May-15 |
| 7315 | 2 | 3 | | | III-2 | Fbxo48 | 554251 | 4-May-15 |
| 7329 | 2 | 3 | | | III-2 | Fbxw19 | | |
| 7330 | 2 | 3 | | | III-2 | Fbxw2 | 26190 | 4-May-15 |
| 7341 | 2 | 3 | | | III-2 | Fbxw9 | 84261 | 4-May-15 |
| 7354 | 2 | 3 | | | III-2 | Fcho2 | 115548 | 4-May-15 |
| 7355 | 2 | 3 | | | III-2 | Fchsd1 | 89848 | 4-May-15 |
| 7361 | 2 | 3 | | | III-2 | Fcrl6 | 343413 | 4-May-15 |
| 7364 | 2 | 3 | | | III-2 | Fcrls | | |
| 7370 | 2 | 3 | | | III-2 | Fdxr | 2232 | 4-May-15 |
| 7374 | 2 | 3 | | | III-2 | Fem1c | 56929 | 4-May-15 |
| 7377 | 2 | 3 | | | III-2 | Fer1l4 | 80307 | 4-May-15 |
| 7378 | 2 | 3 | | | III-2 | Fer1l5 | 90342 | 12-May-15 |
| 7390 | 2 | 3 | | | III-2 | Fezf2 | 55079 | 4-May-15 |
| 7404 | 2 | 3 | | | III-2 | Fgf10 | 2255 | 12-May-15 |
| 7411 | 2 | 3 | | | III-2 | Fgf17 | 8822 | 12-May-15 |
| 7429 | 2 | 3 | | | III-2 | Fgfr1op2 | 26127 | 4-May-15 |
| 7433 | 2 | 3 | | | III-2 | Fgfrl1 | 53834 | 4-May-15 |
| 7440 | 2 | 3 | | | III-2 | Fhad1 | 114827 | 4-May-15 |
| 7450 | 2 | 3 | | | III-2 | Fhod3 | 80206 | 7-Jun-15 |
| 7455 | 2 | 3 | | | III-2 | Fig4 | 9896 | 23-May-15 |
| 7464 | 2 | 3 | | | III-2 | Firre | 286467 | 22-May-15 |
| 7474 | 2 | 3 | | | III-2 | Fkbp1a | 2280 | 12-May-15 |
| 7477 | 2 | 3 | | | III-2 | Fkbp3 | 2287 | 4-May-15 |
| 7483 | 2 | 3 | | | III-2 | Fkbp9 | 11328 | 4-May-15 |
| 7485 | 2 | 3 | | | III-2 | Fkrp | 79147 | 23-May-15 |
| 7486 | 2 | 3 | | | III-2 | Fktn | 2218 | 23-May-15 |
| 7488 | 2 | 3 | | | III-2 | Flcn | 201163 | 24-May-15 |
| 7492 | 2 | 3 | | | III-2 | Flna | 2316 | 28-May-15 |
| 7497 | 2 | 3 | | | III-2 | Flrt1 | 23769 | 12-May-15 |
| 7507 | 2 | 3 | | | III-2 | Fmn2 | 56776 | 24-May-15 |
| 7510 | 2 | 3 | | | III-2 | Fmnl3 | 91010 | 4-May-15 |
| 7517 | 2 | 3 | | | III-2 | Fmo9 | | |
| 7525 | 2 | 3 | | | III-2 | Fnbp1l | 54874 | 4-May-15 |
| 7530 | 2 | 3 | | | III-2 | Fndc3b | 64778 | 4-May-15 |
| 7538 | 2 | 3 | | | III-2 | Fnip2 | 57600 | 4-May-15 |
| 7540 | 2 | 3 | | | III-2 | Fntb | 2342 | 4-May-15 |
| 7552 | 2 | 3 | | | III-2 | Foxa2 | 3170 | 31-May-15 |
| 7553 | 2 | 3 | | | III-2 | Foxa3 | 3171 | 28-May-15 |
| 7554 | 2 | 3 | | | III-2 | Foxb1 | 27023 | 28-May-15 |
| 7559 | 2 | 3 | | | III-2 | Foxd2 | 2306 | 28-May-15 |
| 7560 | 2 | 3 | | | III-2 | Foxd2os | | |
| 7563 | 2 | 3 | | | III-2 | Foxe1 | 2304 | 28-May-15 |
| 7566 | 2 | 3 | | | III-2 | Foxf2 | 2295 | 12-May-15 |
| 7577 | 2 | 3 | | | III-2 | Foxl1 | 2300 | 28-May-15 |
| 7582 | 2 | 3 | | | III-2 | Foxn2 | 3344 | 4-May-15 |
| 7585 | 2 | 3 | | | III-2 | Foxo1 | 2308 | 17-May-15 |
| 7590 | 2 | 3 | | | III-2 | Foxp2 | 93986 | 3-May-15 |
| 7595 | 2 | 3 | | | III-2 | Foxr2 | 139628 | 28-May-15 |
| 7605 | 2 | 3 | | | III-2 | Fpr-rs4 | | |
| 7609 | 2 | 3 | | | III-2 | Frat1 | 10023 | 4-May-15 |
| 7617 | 2 | 3 | | | III-2 | Frmd4a | 55691 | 14-May-15 |

Fig.22 - 45

| | | | | | | | | |
|---|---|---|---|---|---|---|---|---|
| 7621 | 2 | 3 | | | III-2 | Frmd7 | 90167 | 23-May-15 |
| 7622 | 2 | 3 | | | III-2 | Frmd8 | 83786 | 12-May-15 |
| 7632 | 2 | 3 | | | III-2 | Fry | 10129 | 12-May-15 |
| 7633 | 2 | 3 | | | III-2 | Fryl | 285527 | 4-May-15 |
| 7634 | 2 | 3 | | | III-2 | Frzb | 2487 | 4-May-15 |
| 7636 | 2 | 3 | | | III-2 | Fscb | 84075 | 4-May-15 |
| 7637 | 2 | 3 | | | III-2 | Fscn1 | 6624 | 24-May-15 |
| 7639 | 2 | 3 | | | III-2 | Fscn3 | 29999 | 12-May-15 |
| 7640 | 2 | 3 | | | III-2 | Fsd1 | 79187 | 4-May-15 |
| 7644 | 2 | 3 | | | III-2 | Fshr | 2492 | 24-May-15 |
| 7645 | 2 | 3 | | | III-2 | Fsip1 | 161835 | 4-May-15 |
| 7650 | 2 | 3 | | | III-2 | Fstl5 | 56884 | 4-May-15 |
| 7651 | 2 | 3 | | | III-2 | Ftcd | 10841 | 4-May-15 |
| 7653 | 2 | 3 | | | III-2 | Fthl17 | 53940 | 4-May-15 |
| 7656 | 2 | 3 | | | III-2 | Fto | 79068 | 31-May-15 |
| 7660 | 2 | 3 | | | III-2 | Ftx | 100302692 | 22-May-15 |
| 7662 | 2 | 3 | | | III-2 | Fubp3 | 8939 | 28-May-15 |
| 7666 | 2 | 3 | | | III-2 | Fundc1 | 139341 | 24-May-15 |
| 7670 | 2 | 3 | | | III-2 | Fus | 2521 | 31-May-15 |
| 7677 | 2 | 3 | | | III-2 | Fut7 | 2529 | 4-May-15 |
| 7679 | 2 | 3 | | | III-2 | Fut9 | 10690 | 12-May-15 |
| 7695 | 2 | 3 | | | III-2 | Fyttd1 | 84248 | 4-May-15 |
| 7702 | 2 | 3 | | | III-2 | Fzd6 | 8323 | 12-May-15 |
| 7706 | 2 | 3 | | | III-2 | Fzr1 | 51343 | 4-May-15 |
| 7710 | 2 | 3 | | | III-2 | G3bp2 | 9908 | 31-May-15 |
| 7714 | 2 | 3 | | | III-2 | G630071F17Rik | | |
| 7721 | 2 | 3 | | | III-2 | G6pc3 | 92579 | 4-May-15 |
| 7725 | 2 | 3 | | | III-2 | Gaa | 2548 | 23-May-15 |
| 7729 | 2 | 3 | | | III-2 | Gabarap | 11337 | 24-May-15 |
| 7735 | 2 | 3 | | | III-2 | Gabpb1 | 2553 | 7-Jun-15 |
| 7738 | 2 | 3 | | | III-2 | Gabra2 | 2555 | 4-May-15 |
| 7740 | 2 | 3 | | | III-2 | Gabra4 | 2557 | 4-May-15 |
| 7741 | 2 | 3 | | | III-2 | Gabra5 | 2558 | 28-May-15 |
| 7747 | 2 | 3 | | | III-2 | Gabre | 2564 | 12-May-15 |
| 7758 | 2 | 3 | | | III-2 | Gad2 | 2572 | 24-May-15 |
| 7765 | 2 | 3 | | | III-2 | Gal | 51083 | 17-May-15 |
| 7774 | 2 | 3 | | | III-2 | Galm | 130589 | 12-May-15 |
| 7775 | 2 | 3 | | | III-2 | Galns | 2588 | 23-May-15 |
| 7777 | 2 | 3 | | | III-2 | Galnt10 | 55568 | 4-May-15 |
| 7778 | 2 | 3 | | | III-2 | Galnt11 | 63917 | 12-May-15 |
| 7798 | 2 | 3 | | | III-2 | Galt | 2592 | 7-Jun-15 |
| 7799 | 2 | 3 | | | III-2 | Gamt | 2593 | 23-May-15 |
| 7802 | 2 | 3 | | | III-2 | Ganc | 2595 | 12-May-15 |
| 7808 | 2 | 3 | | | III-2 | Gar1 | 54433 | 7-Jun-15 |
| 7811 | 2 | 3 | | | III-2 | Garnl3 | 84253 | 14-May-15 |
| 7818 | 2 | 3 | | | III-2 | Gas2l3 | 283431 | 12-May-15 |
| 7835 | 2 | 3 | | | III-2 | Gatm | 2628 | 23-May-15 |
| 7839 | 2 | 3 | | | III-2 | Gba2 | 57704 | 4-May-15 |
| 7843 | 2 | 3 | | | III-2 | Gbgt1 | 26301 | 12-May-15 |
| 7848 | 2 | 3 | | | III-2 | Gbp3 | 2635 | 7-Jun-15 |
| 7856 | 2 | 3 | | | III-2 | Gbx2 | 2637 | 12-May-15 |
| 7863 | 2 | 3 | | | III-2 | Gcfc2 | 6936 | 4-May-15 |
| 7864 | 2 | 3 | | | III-2 | Gcg | 2641 | 17-May-15 |
| 7882 | 2 | 3 | | | III-2 | Gda | 9615 | 7-Jun-15 |
| 7893 | 2 | 3 | | | III-2 | Gdf3 | 9573 | 4-May-15 |
| 7899 | 2 | 3 | | | III-2 | Gdi2 | 2665 | 4-May-15 |
| 7905 | 2 | 3 | | | III-2 | Gdpd5 | 81544 | 4-May-15 |
| 7910 | 2 | 3 | | | III-2 | Gemin5 | 25929 | 12-May-15 |
| 7921 | 2 | 3 | | | III-2 | Gfm2 | 84340 | 4-May-15 |
| 7924 | 2 | 3 | | | III-2 | Gfpt1 | 2673 | 23-May-15 |
| 7931 | 2 | 3 | | | III-2 | Gfy | 100507003 | 4-May-15 |
| 7950 | 2 | 3 | | | III-2 | Ghitm | 27069 | 4-May-15 |
| 7953 | 2 | 3 | | | III-2 | Ghrhr | 2692 | 12-May-15 |
| 7956 | 2 | 3 | | | III-2 | Gid4 | 79018 | 12-May-15 |
| 7958 | 2 | 3 | | | III-2 | Gif | 2694 | 7-Jun-15 |
| 7975 | 2 | 3 | | | III-2 | Gip | 2695 | 7-Jun-15 |
| 7976 | 2 | 3 | | | III-2 | Gipc1 | 10755 | 4-May-15 |
| 7980 | 2 | 3 | | | III-2 | Git1 | 28964 | 12-May-15 |
| 7981 | 2 | 3 | | | III-2 | Git2 | 9815 | 31-May-15 |
| 7984 | 2 | 3 | | | III-2 | Gja3 | 2700 | 4-May-15 |
| 7988 | 2 | 3 | | | III-2 | Gja8 | 2703 | 12-May-15 |
| 7998 | 2 | 3 | | | III-2 | Gjd2 | 57369 | 12-May-15 |
| 8000 | 2 | 3 | | | III-2 | Gjd4 | 219770 | 4-May-15 |
| 8001 | 2 | 3 | | | III-2 | Gje1 | 100126572 | 4-May-15 |
| 8018 | 2 | 3 | | | III-2 | Gle1 | 2733 | 4-May-15 |
| 8025 | 2 | 3 | | | III-2 | Glipr1l2 | 144321 | 4-May-15 |
| 8029 | 2 | 3 | | | III-2 | Glis3 | 169792 | 14-May-15 |
| 8032 | 2 | 3 | | | III-2 | Glod4 | 51031 | 4-May-15 |
| 8036 | 2 | 3 | | | III-2 | Glra1 | 2741 | 23-May-15 |
| 8038 | 2 | 3 | | | III-2 | Glra3 | 8001 | 12-May-15 |
| 8044 | 2 | 3 | | | III-2 | Glrx3 | 10539 | 4-May-15 |
| 8048 | 2 | 3 | | | III-2 | Glt1d1 | 144423 | 4-May-15 |
| 8052 | 2 | 3 | | | III-2 | Glt8d1 | 55830 | 23-May-15 |
| 8055 | 2 | 3 | | | III-2 | Gltpd1 | 80772 | 4-May-15 |
| 8056 | 2 | 3 | | | III-2 | Gltpd2 | 388323 | 4-May-15 |
| 8067 | 2 | 3 | | | III-2 | Gm10007 | | |
| 8073 | 2 | 3 | | | III-2 | Gm10057 | | |
| 8082 | 2 | 3 | | | III-2 | Gm10125 | | |
| 8085 | 2 | 3 | | | III-2 | Gm10190 | | |
| 8086 | 2 | 3 | | | III-2 | Gm10220 | | |
| 8090 | 2 | 3 | | | III-2 | Gm10248 | | |
| 8094 | 2 | 3 | | | III-2 | Gm10318 | | |
| 8099 | 2 | 3 | | | III-2 | Gm10354 | | |
| 8101 | 2 | 3 | | | III-2 | Gm10373 | | |
| 8114 | 2 | 3 | | | III-2 | Gm10432 | | |

| | | | | | | | | |
|---|---|---|---|---|---|---|---|---|
| 8115 | 2 | 3 | | | III-2 | Gm10433 | | |
| 8123 | 2 | 3 | | | III-2 | Gm10471 | | |
| 8129 | 2 | 3 | | | III-2 | Gm10509 | | |
| 8131 | 2 | 3 | | | III-2 | Gm10512 | | |
| 8134 | 2 | 3 | | | III-2 | Gm10536 | | |
| 8137 | 2 | 3 | | | III-2 | Gm10549 | | |
| 8138 | 2 | 3 | | | III-2 | Gm10556 | | |
| 8141 | 2 | 3 | | | III-2 | Gm10584 | | |
| 8144 | 2 | 3 | | | III-2 | Gm10635 | | |
| 8146 | 2 | 3 | | | III-2 | Gm10637 | | |
| 8154 | 2 | 3 | | | III-2 | Gm10665 | | |
| 8160 | 2 | 3 | | | III-2 | Gm10696 | | |
| 8169 | 2 | 3 | | | III-2 | Gm10785 | | |
| 8180 | 2 | 3 | | | III-2 | Gm10863 | | |
| 8184 | 2 | 3 | | | III-2 | Gm10922 | | |
| 8191 | 2 | 3 | | | III-2 | Gm11166 | | |
| 8207 | 2 | 3 | | | III-2 | Gm11487 | | |
| 8210 | 2 | 3 | | | III-2 | Gm11538 | | |
| 8234 | 2 | 3 | | | III-2 | Gm11757 | | |
| 8242 | 2 | 3 | | | III-2 | Gm11961 | | |
| 8245 | 2 | 3 | | | III-2 | Gm11981 | | |
| 8252 | 2 | 3 | | | III-2 | Gm12159 | | |
| 8261 | 2 | 3 | | | III-2 | Gm12295 | | |
| 8267 | 2 | 3 | | | III-2 | Gm12504 | | |
| 8269 | 2 | 3 | | | III-2 | Gm12522 | | |
| 8279 | 2 | 3 | | | III-2 | Gm12794 | | |
| 8282 | 2 | 3 | | | III-2 | Gm12886 | | |
| 8290 | 2 | 3 | | | III-2 | Gm13031 | | |
| 8292 | 2 | 3 | | | III-2 | Gm13034 | | |
| 8294 | 2 | 3 | | | III-2 | Gm13043 | | |
| 8310 | 2 | 3 | | | III-2 | Gm13157 | | |
| 8315 | 2 | 3 | | | III-2 | Gm13238 | | |
| 8317 | 2 | 3 | | | III-2 | Gm13247 | | |
| 8320 | 2 | 3 | | | III-2 | Gm13272 | | |
| 8341 | 2 | 3 | | | III-2 | Gm13483 | | |
| 8344 | 2 | 3 | | | III-2 | Gm13498 | | |
| 8349 | 2 | 3 | | | III-2 | Gm13580 | | |
| 8351 | 2 | 3 | | | III-2 | Gm136 | | |
| 8355 | 2 | 3 | | | III-2 | Gm13752 | | |
| 8362 | 2 | 3 | | | III-2 | Gm13944 | | |
| 8365 | 2 | 3 | | | III-2 | Gm14023 | | |
| 8369 | 2 | 3 | | | III-2 | Gm14124 | | |
| 8372 | 2 | 3 | | | III-2 | Gm14151 | | |
| 8381 | 2 | 3 | | | III-2 | Gm14308 | | |
| 8393 | 2 | 3 | | | III-2 | Gm14391 | | |
| 8397 | 2 | 3 | | | III-2 | Gm14420 | | |
| 8403 | 2 | 3 | | | III-2 | Gm14459 | | |
| 8421 | 2 | 3 | | | III-2 | Gm14632 | | |
| 8437 | 2 | 3 | | | III-2 | Gm14920 | | |
| 8439 | 2 | 3 | | | III-2 | Gm15023 | | |
| 8457 | 2 | 3 | | | III-2 | Gm15299 | | |
| 8460 | 2 | 3 | | | III-2 | Gm15319 | | |
| 8461 | 2 | 3 | | | III-2 | Gm15328 | | |
| 8468 | 2 | 3 | | | III-2 | Gm15413 | | |
| 8482 | 2 | 3 | | | III-2 | Gm15679 | | |
| 8499 | 2 | 3 | | | III-2 | Gm15987 | | |
| 8501 | 2 | 3 | | | III-2 | Gm16023 | | |
| 8503 | 2 | 3 | | | III-2 | Gm1604b | | |
| 8506 | 2 | 3 | | | III-2 | Gm16130 | | |
| 8523 | 2 | 3 | | | III-2 | Gm16451 | | |
| 8528 | 2 | 3 | | | III-2 | Gm16523 | | |
| 8529 | 2 | 3 | | | III-2 | Gm1653 | | |
| 8531 | 2 | 3 | | | III-2 | Gm16548 | | |
| 8536 | 2 | 3 | | | III-2 | Gm1661 | | |
| 8540 | 2 | 3 | | | III-2 | Gm16701 | | |
| 8541 | 2 | 3 | | | III-2 | Gm16702 | | |
| 8542 | 2 | 3 | | | III-2 | Gm16712 | | |
| 8552 | 2 | 3 | | | III-2 | Gm16880 | | |
| 8557 | 2 | 3 | | | III-2 | Gm16973 | | |
| 8563 | 2 | 3 | | | III-2 | Gm1720 | | |
| 8570 | 2 | 3 | | | III-2 | Gm17660 | | |
| 8576 | 2 | 3 | | | III-2 | Gm17751 | | |
| 8579 | 2 | 3 | | | III-2 | Gm17769 | | |
| 8582 | 2 | 3 | | | III-2 | Gm17830 | | |
| 8585 | 2 | 3 | | | III-2 | Gm18853 | | |
| 8594 | 2 | 3 | | | III-2 | Gm1943 | | |
| 8596 | 2 | 3 | | | III-2 | Gm19461 | | |
| 8598 | 2 | 3 | | | III-2 | Gm19510 | | |
| 8602 | 2 | 3 | | | III-2 | Gm19589 | | |
| 8608 | 2 | 3 | | | III-2 | Gm19689 | | |
| 8614 | 2 | 3 | | | III-2 | Gm19784 | | |
| 8619 | 2 | 3 | | | III-2 | Gm1995 | | |
| 8622 | 2 | 3 | | | III-2 | Gm20063 | | |
| 8626 | 2 | 3 | | | III-2 | Gm2012 | | |
| 8636 | 2 | 3 | | | III-2 | Gm2027 | | |
| 8638 | 2 | 3 | | | III-2 | Gm20300 | | |
| 8643 | 2 | 3 | | | III-2 | Gm20362 | | |
| 8649 | 2 | 3 | | | III-2 | Gm20604 | | |
| 8660 | 2 | 3 | | | III-2 | Gm20745 | | |
| 8684 | 2 | 3 | | | III-2 | Gm20854 | | |
| 8701 | 2 | 3 | | | III-2 | Gm21269 | | |
| 8713 | 2 | 3 | | | III-2 | Gm21671 | | |
| 8733 | 2 | 3 | | | III-2 | Gm2762 | | |
| 8739 | 2 | 3 | | | III-2 | Gm2897 | | |
| 8748 | 2 | 3 | | | III-2 | Gm3143 | | |
| 8753 | 2 | 3 | | | III-2 | Gm3259 | | |
| 8754 | 2 | 3 | | | III-2 | Gm3264 | | |

Fig.22 - 46

| | | | | | | | | |
|---|---|---|---|---|---|---|---|---|
| 8756 | 2 | 3 | | | III-2 | Gm3285 | | |
| 8776 | 2 | 3 | | | III-2 | Gm364 | | |
| 8783 | 2 | 3 | | | III-2 | Gm3763 | | |
| 8786 | 2 | 3 | | | III-2 | Gm3893 | | |
| 8792 | 2 | 3 | | | III-2 | Gm4133 | | |
| 8793 | 2 | 3 | | | III-2 | Gm4175 | | |
| 8805 | 2 | 3 | | | III-2 | Gm4301 | | |
| 8824 | 2 | 3 | | | III-2 | Gm4567 | | |
| 8827 | 2 | 3 | | | III-2 | Gm4719 | | |
| 8841 | 2 | 3 | | | III-2 | Gm4832 | | |
| 8854 | 2 | 3 | | | III-2 | Gm4906 | | |
| 8857 | 2 | 3 | | | III-2 | Gm4925 | | |
| 8865 | 2 | 3 | | | III-2 | Gm4971 | | |
| 8866 | 2 | 3 | | | III-2 | Gm4975 | | |
| 8869 | 2 | 3 | | | III-2 | Gm4984 | | |
| 8875 | 2 | 3 | | | III-2 | Gm5072 | | |
| 8880 | 2 | 3 | | | III-2 | Gm5087 | | |
| 8890 | 2 | 3 | | | III-2 | Gm5122 | | |
| 8898 | 2 | 3 | | | III-2 | Gm5141 | | |
| 8900 | 2 | 3 | | | III-2 | Gm5148 | | |
| 8907 | 2 | 3 | | | III-2 | Gm525 | | |
| 8912 | 2 | 3 | | | III-2 | Gm5346 | | |
| 8924 | 2 | 3 | | | III-2 | Gm5458 | | |
| 8943 | 2 | 3 | | | III-2 | Gm5591 | | |
| 8944 | 2 | 3 | | | III-2 | Gm5592 | | |
| 8947 | 2 | 3 | | | III-2 | Gm561 | | |
| 8951 | 2 | 3 | | | III-2 | Gm5627 | | |
| 8957 | 2 | 3 | | | III-2 | Gm5712 | | |
| 8967 | 2 | 3 | | | III-2 | Gm5796 | | |
| 8973 | 2 | 3 | | | III-2 | Gm5833 | | |
| 8977 | 2 | 3 | | | III-2 | Gm5878 | | |
| 9002 | 2 | 3 | | | III-2 | Gm6121 | | |
| 9008 | 2 | 3 | | | III-2 | Gm6225 | | |
| 9019 | 2 | 3 | | | III-2 | Gm6329 | | |
| 9025 | 2 | 3 | | | III-2 | Gm6408 | | |
| 9033 | 2 | 3 | | | III-2 | Gm6524 | | |
| 9036 | 2 | 3 | | | III-2 | Gm6548 | | |
| 9042 | 2 | 3 | | | III-2 | Gm6588 | | |
| 9066 | 2 | 3 | | | III-2 | Gm6880 | | |
| 9075 | 2 | 3 | | | III-2 | Gm6994 | | |
| 9076 | 2 | 3 | | | III-2 | Gm7008 | | |
| 9079 | 2 | 3 | | | III-2 | Gm7073 | | |
| 9082 | 2 | 3 | | | III-2 | Gm711 | | |
| 9087 | 2 | 3 | | | III-2 | Gm7168 | | |
| 9091 | 2 | 3 | | | III-2 | Gm7271 | | |
| 9096 | 2 | 3 | | | III-2 | Gm7361 | | |
| 9099 | 2 | 3 | | | III-2 | Gm7457 | | |
| 9105 | 2 | 3 | | | III-2 | Gm765 | | |
| 9109 | 2 | 3 | | | III-2 | Gm773 | | |
| 9115 | 2 | 3 | | | III-2 | Gm7904 | | |
| 9135 | 2 | 3 | | | III-2 | Gm8439 | | |
| 9138 | 2 | 3 | | | III-2 | Gm8580 | | |
| 9143 | 2 | 3 | | | III-2 | Gm8693 | | |
| 9147 | 2 | 3 | | | III-2 | Gm8787 | | |
| 9150 | 2 | 3 | | | III-2 | Gm884 | | |
| 9157 | 2 | 3 | | | III-2 | Gm8989 | | |
| 9162 | 2 | 3 | | | III-2 | Gm9054 | | |
| 9165 | 2 | 3 | | | III-2 | Gm9112 | | |
| 9178 | 2 | 3 | | | III-2 | Gm9731 | | |
| 9179 | 2 | 3 | | | III-2 | Gm9733 | | |
| 9185 | 2 | 3 | | | III-2 | Gm9855 | | |
| 9187 | 2 | 3 | | | III-2 | Gm9871 | | |
| 9190 | 2 | 3 | | | III-2 | Gm9920 | | |
| 9191 | 2 | 3 | | | III-2 | Gm9926 | | |
| 9203 | 2 | 3 | | | III-2 | Gmeb2 | 26205 | 4-May-15 |
| 9211 | 2 | 3 | | | III-2 | Gmppb | 29925 | 4-May-15 |
| 9214 | 2 | 3 | | | III-2 | Gmps | 8833 | 4-May-15 |
| 9216 | 2 | 3 | | | III-2 | Gna12 | 2768 | 4-May-15 |
| 9218 | 2 | 3 | | | III-2 | Gna14 | 9630 | 4-May-15 |
| 9222 | 2 | 3 | | | III-2 | Gnai3 | 2773 | 12-May-15 |
| 9226 | 2 | 3 | | | III-2 | Gnas | 2778 | 23-May-15 |
| 9231 | 2 | 3 | | | III-2 | Gnb1 | 2782 | 3-May-15 |
| 9232 | 2 | 3 | | | III-2 | Gnb1l | 54584 | 4-May-15 |
| 9240 | 2 | 3 | | | III-2 | Gng11 | 2791 | 4-May-15 |
| 9252 | 2 | 3 | | | III-2 | Gnl2 | 29889 | 4-May-15 |
| 9257 | 2 | 3 | | | III-2 | Gnpda1 | 10007 | 12-May-15 |
| 9258 | 2 | 3 | | | III-2 | Gnpda2 | 132789 | 12-May-15 |
| 9259 | 2 | 3 | | | III-2 | Gnpnat1 | 64841 | 4-May-15 |
| 9264 | 2 | 3 | | | III-2 | Gns | 2799 | 4-May-15 |
| 9270 | 2 | 3 | | | III-2 | Golga7 | 51125 | 4-May-15 |
| 9276 | 2 | 3 | | | III-2 | Golph3l | 55204 | 4-May-15 |
| 9279 | 2 | 3 | | | III-2 | Gon4l | 54856 | 12-May-15 |
| 9301 | 2 | 3 | | | III-2 | Gpat2 | 150763 | 4-May-15 |
| 9302 | 2 | 3 | | | III-2 | Gpatch1 | 55094 | 4-May-15 |
| 9310 | 2 | 3 | | | III-2 | Gpbp1 | 65056 | 4-May-15 |
| 9316 | 2 | 3 | | | III-2 | Gpc5 | 2262 | 24-May-15 |
| 9325 | 2 | 3 | | | III-2 | Gphn | 10243 | 23-May-15 |
| 9329 | 2 | 3 | | | III-2 | Gpld1 | 2822 | 4-May-15 |
| 9339 | 2 | 3 | | | III-2 | Gpr108 | 56927 | 4-May-15 |
| 9342 | 2 | 3 | | | III-2 | Gpr113 | 165082 | 4-May-15 |
| 9350 | 2 | 3 | | | III-2 | Gpr125 | 166647 | 14-May-15 |
| 9351 | 2 | 3 | | | III-2 | Gpr126 | 57211 | 4-May-15 |
| 9361 | 2 | 3 | | | III-2 | Gpr141 | 353345 | 14-May-15 |
| 9368 | 2 | 3 | | | III-2 | Gpr151 | 134391 | 4-May-15 |
| 9373 | 2 | 3 | | | III-2 | Gpr157 | 80045 | 12-May-15 |
| 9378 | 2 | 3 | | | III-2 | Gpr165 | | |
| 9379 | 2 | 3 | | | III-2 | Gpr17 | 2840 | 10-May-15 |
| 9382 | 2 | 3 | | | III-2 | Gpr174 | 84636 | 4-May-15 |
| 9384 | 2 | 3 | | | III-2 | Gpr179 | 440435 | 4-May-15 |
| 9385 | 2 | 3 | | | III-2 | Gpr18 | 2841 | 4-May-15 |
| 9391 | 2 | 3 | | | III-2 | Gpr21 | 2844 | 4-May-15 |
| 9392 | 2 | 3 | | | III-2 | Gpr22 | 2845 | 4-May-15 |
| 9394 | 2 | 3 | | | III-2 | Gpr26 | 2849 | 7-Jun-15 |
| 9397 | 2 | 3 | | | III-2 | Gpr31b | | |
| 9401 | 2 | 3 | | | III-2 | Gpr37 | 2861 | 28-May-15 |
| 9402 | 2 | 3 | | | III-2 | Gpr37l1 | 9283 | 4-May-15 |
| 9410 | 2 | 3 | | | III-2 | Gpr6 | 2830 | 4-May-15 |
| 9421 | 2 | 3 | | | III-2 | Gpr85 | 54329 | 4-May-15 |
| 9438 | 2 | 3 | | | III-2 | Gps2 | 2874 | 4-May-15 |
| 9467 | 2 | 3 | | | III-2 | Greb1 | 9687 | 18-May-15 |
| 9479 | 2 | 3 | | | III-2 | Grid1 | 2894 | 4-May-15 |
| 9480 | 2 | 3 | | | III-2 | Grid2 | 2895 | 4-May-15 |
| 9481 | 2 | 3 | | | III-2 | Grid2ip | 392862 | 4-May-15 |
| 9496 | 2 | 3 | | | III-2 | Grina | 2907 | 4-May-15 |
| 9501 | 2 | 3 | | | III-2 | Grk1 | 6011 | 12-May-15 |
| 9504 | 2 | 3 | | | III-2 | Grk6 | 2870 | 12-May-15 |
| 9505 | 2 | 3 | | | III-2 | Grm1 | 2911 | 4-May-15 |
| 9512 | 2 | 3 | | | III-2 | Grm8 | 2918 | 12-May-15 |
| 9520 | 2 | 3 | | | III-2 | Grtp1 | 79774 | 4-May-15 |
| 9526 | 2 | 3 | | | III-2 | Gsc2 | 2928 | 4-May-15 |
| 9527 | 2 | 3 | | | III-2 | Gsdma | 284110 | 4-May-15 |
| 9535 | 2 | 3 | | | III-2 | Gsdmcl2 | | |
| 9537 | 2 | 3 | | | III-2 | Gsdmd | 79792 | 4-May-15 |
| 9543 | 2 | 3 | | | III-2 | Gsk3b | 2932 | 31-May-15 |
| 9565 | 2 | 3 | | | III-2 | Gstp1 | 2950 | 23-May-15 |
| 9573 | 2 | 3 | | | III-2 | Gsx2 | 170825 | 12-May-15 |
| 9578 | 2 | 3 | | | III-2 | Gtf2a2 | 2958 | 2-Jun-15 |
| 9581 | 2 | 3 | | | III-2 | Gtf2e2 | 2961 | 31-May-15 |
| 9583 | 2 | 3 | | | III-2 | Gtf2f2 | 2963 | 4-May-15 |
| 9586 | 2 | 3 | | | III-2 | Gtf2h3 | 2967 | 4-May-15 |
| 9587 | 2 | 3 | | | III-2 | Gtf2h4 | 2968 | 4-May-15 |
| 9590 | 2 | 3 | | | III-2 | Gtf2ird1 | 9569 | 28-May-15 |
| 9594 | 2 | 3 | | | III-2 | Gtf3c2 | 2976 | 4-May-15 |
| 9597 | 2 | 3 | | | III-2 | Gtf3c5 | 9328 | 4-May-15 |
| 9599 | 2 | 3 | | | III-2 | Gtl3 | 29105 | 4-May-15 |
| 9600 | 2 | 3 | | | III-2 | Gtpbp1 | 9567 | 4-May-15 |
| 9603 | 2 | 3 | | | III-2 | Gtpbp3 | 84705 | 4-May-15 |
| 9609 | 2 | 3 | | | III-2 | Gtsf1l | 149699 | 4-May-15 |
| 9618 | 2 | 3 | | | III-2 | Gucy1b3 | 2983 | 12-May-15 |
| 9620 | 2 | 3 | | | III-2 | Gucy2d | 3000 | 31-May-15 |
| 9631 | 2 | 3 | | | III-2 | Gxylt2 | 727936 | 4-May-15 |
| 9651 | 2 | 3 | | | III-2 | H13 | 81502 | 23-May-15 |
| 9661 | 2 | 3 | | | III-2 | H2afb3 | 83740 | 4-May-15 |
| 9666 | 2 | 3 | | | III-2 | H2afy2 | 55506 | 4-May-15 |
| 9676 | 2 | 3 | | | III-2 | H2-Eb1 | | |
| 9681 | 2 | 3 | | | III-2 | H2-Ke6 | 7923 | 4-May-15 |
| 9683 | 2 | 3 | | | III-2 | H2-M1 | | |
| 9710 | 2 | 3 | | | III-2 | H2-T3 | | |
| 9714 | 2 | 3 | | | III-2 | H60b | | |
| 9715 | 2 | 3 | | | III-2 | H60c | | |
| 9720 | 2 | 3 | | | III-2 | Hace1 | 57531 | 12-May-15 |
| 9723 | 2 | 3 | | | III-2 | Hadha | 3030 | 31-May-15 |
| 9731 | 2 | 3 | | | III-2 | Hand2 | 9464 | 4-May-15 |
| 9737 | 2 | 3 | | | III-2 | Hapln3 | 145864 | 12-May-15 |
| 9740 | 2 | 3 | | | III-2 | Hars | 3035 | 12-May-15 |
| 9745 | 2 | 3 | | | III-2 | Has3 | 3038 | 4-May-15 |
| 9749 | 2 | 3 | | | III-2 | Haus3 | 79441 | 12-May-15 |
| 9753 | 2 | 3 | | | III-2 | Haus7 | 55559 | 4-May-15 |
| 9772 | 2 | 3 | | | III-2 | Hc | 727 | 7-Jun-15 |
| 9776 | 2 | 3 | | | III-2 | Hcfc1 | 3054 | 23-May-15 |
| 9777 | 2 | 3 | | | III-2 | Hcfc1r1 | 54985 | 12-May-15 |
| 9782 | 2 | 3 | | | III-2 | Hcn2 | 610 | 12-May-15 |
| 9790 | 2 | 3 | | | III-2 | Hdac10 | 83933 | 28-May-15 |
| 9793 | 2 | 3 | | | III-2 | Hdac3 | 8841 | 31-May-15 |
| 9804 | 2 | 3 | | | III-2 | Hdgfl1 | 154150 | 12-May-15 |
| 9811 | 2 | 3 | | | III-2 | Hdx | 139324 | 4-May-15 |
| 9814 | 2 | 3 | | | III-2 | Heatr3 | 55027 | 4-May-15 |
| 9818 | 2 | 3 | | | III-2 | Heatr9 | 256957 | 4-May-15 |
| 9819 | 2 | 3 | | | III-2 | Hebp1 | 50865 | 4-May-15 |
| 9825 | 2 | 3 | | | III-2 | Hecw1 | 23072 | 4-May-15 |
| 9829 | 2 | 3 | | | III-2 | Hells | 3070 | 4-May-15 |
| 9832 | 2 | 3 | | | III-2 | Helz | 9931 | 23-May-15 |
| 9842 | 2 | 3 | | | III-2 | Herc1 | 8925 | 12-May-15 |
| 9847 | 2 | 3 | | | III-2 | Herpud1 | 9709 | 12-May-15 |
| 9852 | 2 | 3 | | | III-2 | Hes5 | 388585 | 4-May-15 |
| 9859 | 2 | 3 | | | III-2 | Hexim1 | 10614 | 4-May-15 |
| 9867 | 2 | 3 | | | III-2 | Hgd | 3081 | 23-May-15 |
| 9878 | 2 | 3 | | | III-2 | Hhla1 | 10086 | 4-May-15 |
| 9879 | 2 | 3 | | | III-2 | Hiat1 | 64645 | 12-May-15 |
| 9885 | 2 | 3 | | | III-2 | Hid1 | 283987 | 12-May-15 |
| 9886 | 2 | 3 | | | III-2 | Hif1a | 3091 | 31-May-15 |
| 9892 | 2 | 3 | | | III-2 | Higd2a | 192286 | 4-May-15 |
| 9913 | 2 | 3 | | | III-2 | Hist1h2aa | 221613 | 4-May-15 |
| 9923 | 2 | 3 | | | III-2 | Hist1h2an | | |
| 9929 | 2 | 3 | | | III-2 | Hist1h2be | 8344 | 4-May-15 |
| 9979 | 2 | 3 | | | III-2 | Hk1os | | |
| 9990 | 2 | 3 | | | III-2 | Hmcn1 | 83872 | 4-May-15 |
| 9992 | 2 | 3 | | | III-2 | Hmg20b | 10362 | 4-May-15 |
| 9999 | 2 | 3 | | | III-2 | Hmgb2 | 3148 | 4-May-15 |
| 10011 | 2 | 3 | | | III-2 | Hmgxb3 | 22993 | 4-May-15 |
| 10017 | 2 | 3 | | | III-2 | Hmx1 | 3166 | 12-May-15 |
| 10019 | 2 | 3 | | | III-2 | Hmx3 | 340784 | 4-May-15 |
| 10021 | 2 | 3 | | | III-2 | Hn1l | 90861 | 7-Jun-15 |
| 10027 | 2 | 3 | | | III-2 | Hnmt | 3176 | 12-May-15 |

Fig.22 - 47

| | | | | | | | | |
|---|---|---|---|---|---|---|---|---|
| 10031 | 2 | 3 | | | III-2 | Hnrnpa3 | 220988 | 4-May-15 |
| 10041 | 2 | 3 | | | III-2 | Hnrnpl | 3191 | 1-Jun-15 |
| 10052 | 2 | 3 | | | III-2 | Homez | 57594 | 12-May-15 |
| 10058 | 2 | 3 | | | III-2 | Hormad2 | 150280 | 4-May-15 |
| 10086 | 2 | 3 | | | III-2 | Hoxc13 | 3229 | 7-Jun-15 |
| 10088 | 2 | 3 | | | III-2 | Hoxc5 | 3222 | 4-May-15 |
| 10090 | 2 | 3 | | | III-2 | Hoxc8 | 3224 | 4-May-15 |
| 10091 | 2 | 3 | | | III-2 | Hoxc9 | 3225 | 4-May-15 |
| 10104 | 2 | 3 | | | III-2 | Hpca | 3208 | 7-Jun-15 |
| 10113 | 2 | 3 | | | III-2 | Hps1 | 3257 | 23-May-15 |
| 10114 | 2 | 3 | | | III-2 | Hps3 | 84343 | 23-May-15 |
| 10117 | 2 | 3 | | | III-2 | Hps6 | 79803 | 23-May-15 |
| 10129 | 2 | 3 | | | III-2 | Hrh2 | 3274 | 4-May-15 |
| 10142 | 2 | 3 | | | III-2 | Hs3st5 | 222537 | 7-Jun-15 |
| 10145 | 2 | 3 | | | III-2 | Hs6st2 | 90161 | 4-May-15 |
| 10160 | 2 | 3 | | | III-2 | Hsd17b4 | 3295 | 12-May-15 |
| 10171 | 2 | 3 | | | III-2 | Hsdl2 | 84263 | 4-May-15 |
| 10173 | 2 | 3 | | | III-2 | Hsf2 | 3298 | 12-May-15 |
| 10174 | 2 | 3 | | | III-2 | Hsf2bp | 11077 | 28-May-15 |
| 10176 | 2 | 3 | | | III-2 | Hsf4 | 3299 | 17-May-15 |
| 10178 | 2 | 3 | | | III-2 | Hsfy2 | 159119 | 4-May-15 |
| 10182 | 2 | 3 | | | III-2 | Hsp90b1 | 7184 | 10-May-15 |
| 10186 | 2 | 3 | | | III-2 | Hspa14 | 51182 | 4-May-15 |
| 10192 | 2 | 3 | | | III-2 | Hspa4l | 22824 | 4-May-15 |
| 10205 | 2 | 3 | | | III-2 | Hspbp1 | 23640 | 4-May-15 |
| 10212 | 2 | 3 | | | III-2 | Htr1a | 3350 | 17-May-15 |
| 10215 | 2 | 3 | | | III-2 | Htr1f | 3355 | 4-May-15 |
| 10231 | 2 | 3 | | | III-2 | Hunk | 30811 | 4-May-15 |
| 10233 | 2 | 3 | | | III-2 | Hus1b | 135458 | 4-May-15 |
| 10238 | 2 | 3 | | | III-2 | Hyal3 | 8372 | 7-Jun-15 |
| 10240 | 2 | 3 | | | III-2 | Hyal5 | 6677 | 12-May-15 |
| 10255 | 2 | 3 | | | III-2 | Iars2 | 55699 | 12-May-15 |
| 10265 | 2 | 3 | | | III-2 | Ick | 22858 | 4-May-15 |
| 10266 | 2 | 3 | | | III-2 | Icmt | 23463 | 4-May-15 |
| 10267 | 2 | 3 | | | III-2 | Icos | 29851 | 17-May-15 |
| 10269 | 2 | 3 | | | III-2 | Ict1 | 3396 | 4-May-15 |
| 10282 | 2 | 3 | | | III-2 | Idnk | 414328 | 4-May-15 |
| 10286 | 2 | 3 | | | III-2 | Idua | 3425 | 23-May-15 |
| 10290 | 2 | 3 | | | III-2 | Ier5 | 51278 | 12-May-15 |
| 10293 | 2 | 3 | | | III-2 | Iffo2 | 126917 | 4-May-15 |
| 10305 | 2 | 3 | | | III-2 | Ifi47 | | |
| 10307 | 2 | 3 | | | III-2 | Ifit1 | 3434 | 4-May-15 |
| 10318 | 2 | 3 | | | III-2 | Ifna1 | 3439 | 31-May-15 |
| 10335 | 2 | 3 | | | III-2 | Ifne | 338376 | 4-May-15 |
| 10338 | 2 | 3 | | | III-2 | Ifngr2 | 3460 | 24-May-15 |
| 10340 | 2 | 3 | | | III-2 | Ifnl2 | 282616 | 4-May-15 |
| 10347 | 2 | 3 | | | III-2 | Ift140 | 9742 | 4-May-15 |
| 10354 | 2 | 3 | | | III-2 | Ift52 | 51098 | 12-May-15 |
| 10355 | 2 | 3 | | | III-2 | Ift57 | 55081 | 4-May-15 |
| 10361 | 2 | 3 | | | III-2 | Igbp1b | | |
| 10368 | 2 | 3 | | | III-2 | Igf2bp2 | 10644 | 17-May-15 |
| 10371 | 2 | 3 | | | III-2 | Igf2r | 3482 | 21-May-15 |
| 10385 | 2 | 3 | | | III-2 | Igip | 492311 | 4-May-15 |
| 10388 | 2 | 3 | | | III-2 | Iglon5 | 402665 | 4-May-15 |
| 10400 | 2 | 3 | | | III-2 | Igtp | | |
| 10406 | 2 | 3 | | | III-2 | Ikbkb | 3551 | 31-May-15 |
| 10415 | 2 | 3 | | | III-2 | Il10ra | 3587 | 4-May-15 |
| 10420 | 2 | 3 | | | III-2 | Il12a | 3592 | 17-May-15 |
| 10423 | 2 | 3 | | | III-2 | Il12rb2 | 3595 | 4-May-15 |
| 10427 | 2 | 3 | | | III-2 | Il15 | 3600 | 17-May-15 |
| 10431 | 2 | 3 | | | III-2 | Il17b | 27190 | 4-May-15 |
| 10435 | 2 | 3 | | | III-2 | Il17ra | 23765 | 24-May-15 |
| 10448 | 2 | 3 | | | III-2 | Il1f10 | 84639 | 4-May-15 |
| 10456 | 2 | 3 | | | III-2 | Il1rapl1 | 11141 | 23-May-15 |
| 10462 | 2 | 3 | | | III-2 | Il20 | 50604 | 4-May-15 |
| 10466 | 2 | 3 | | | III-2 | Il21r | 50615 | 4-May-15 |
| 10471 | 2 | 3 | | | III-2 | Il23r | 149233 | 31-May-15 |
| 10480 | 2 | 3 | | | III-2 | Il31 | 386653 | 7-Jun-15 |
| 10486 | 2 | 3 | | | III-2 | Il4i1 | 259307 | 4-May-15 |
| 10493 | 2 | 3 | | | III-2 | Il7 | 3574 | 17-May-15 |
| 10496 | 2 | 3 | | | III-2 | Il9r | 3581 | 10-May-15 |
| 10508 | 2 | 3 | | | III-2 | Imp3 | 55272 | 7-Jun-15 |
| 10526 | 2 | 3 | | | III-2 | Ing4 | 51147 | 2-Jun-15 |
| 10532 | 2 | 3 | | | III-2 | Inhbe | 83729 | 12-May-15 |
| 10536 | 2 | 3 | | | III-2 | Ino80b | 83444 | 4-May-15 |
| 10538 | 2 | 3 | | | III-2 | Ino80d | 54891 | 12-May-15 |
| 10543 | 2 | 3 | | | III-2 | Inpp4b | 8821 | 4-May-15 |
| 10545 | 2 | 3 | | | III-2 | Inpp5b | 3633 | 16-May-15 |
| 10548 | 2 | 3 | | | III-2 | Inpp5f | 22876 | 16-Jun-15 |
| 10563 | 2 | 3 | | | III-2 | Insrr | 3645 | 4-May-15 |
| 10565 | 2 | 3 | | | III-2 | Ints10 | 55174 | 4-May-15 |
| 10566 | 2 | 3 | | | III-2 | Ints12 | 57117 | 4-May-15 |
| 10569 | 2 | 3 | | | III-2 | Ints4 | 92105 | 4-May-15 |
| 10574 | 2 | 3 | | | III-2 | Ints9 | 55756 | 4-May-15 |
| 10577 | 2 | 3 | | | III-2 | Ip6k1 | 9807 | 12-May-15 |
| 10585 | 2 | 3 | | | III-2 | Ipo5 | 3843 | 12-May-15 |
| 10595 | 2 | 3 | | | III-2 | Iqcd | 115811 | 4-May-15 |
| 10604 | 2 | 3 | | | III-2 | Iqcj | 654502 | 4-May-15 |
| 10612 | 2 | 3 | | | III-2 | Iqub | 154865 | 4-May-15 |
| 10613 | 2 | 3 | | | III-2 | Irak1 | 3654 | 12-May-15 |
| 10620 | 2 | 3 | | | III-2 | Irf2 | 3660 | 31-May-15 |
| 10621 | 2 | 3 | | | III-2 | Irf2bp1 | 26145 | 4-May-15 |
| 10622 | 2 | 3 | | | III-2 | Irf2bp2 | 359948 | 12-May-15 |
| 10631 | 2 | 3 | | | III-2 | Irg1 | 730249 | 4-May-15 |
| 10635 | 2 | 3 | | | III-2 | Irgq | 126298 | 4-May-15 |
| 10636 | 2 | 3 | | | III-2 | Irs1 | 3667 | 17-May-15 |
| 10645 | 2 | 3 | | | III-2 | Irx6 | 79190 | 4-May-15 |
| 10646 | 2 | 3 | | | III-2 | Isca1 | 81689 | 4-May-15 |
| 10662 | 2 | 3 | | | III-2 | Ist1 | 9798 | 7-Jun-15 |
| 10663 | 2 | 3 | | | III-2 | Isx | 91464 | 4-May-15 |
| 10672 | 2 | 3 | | | III-2 | Itga11 | 22801 | 21-May-15 |
| 10689 | 2 | 3 | | | III-2 | Itgb1bp1 | 9270 | 8-May-15 |
| 10693 | 2 | 3 | | | III-2 | Itgb3 | 3690 | 24-May-15 |
| 10695 | 2 | 3 | | | III-2 | Itgb4 | 3691 | 23-May-15 |
| 10704 | 2 | 3 | | | III-2 | Itih4 | 3700 | 17-May-15 |
| 10716 | 2 | 3 | | | III-2 | Itpr1 | 3708 | 4-May-15 |
| 10721 | 2 | 3 | | | III-2 | Itpripl2 | 162073 | 4-May-15 |
| 10723 | 2 | 3 | | | III-2 | Itsn2 | 50618 | 4-May-15 |
| 10730 | 2 | 3 | | | III-2 | Izumo2 | 126123 | 4-May-15 |
| 10734 | 2 | 3 | | | III-2 | Jade2 | 23338 | 12-May-15 |
| 10739 | 2 | 3 | | | III-2 | Jak1 | 3716 | 31-May-15 |
| 10744 | 2 | 3 | | | III-2 | Jakmip3 | 282973 | 4-May-15 |
| 10748 | 2 | 3 | | | III-2 | Jazf1 | 221895 | 4-May-15 |
| 10763 | 2 | 3 | | | III-2 | Jph4 | 84502 | 4-May-15 |
| 10765 | 2 | 3 | | | III-2 | Jrk | 8629 | 4-May-15 |
| 10766 | 2 | 3 | | | III-2 | Jrkl | 8690 | 4-May-15 |
| 10769 | 2 | 3 | | | III-2 | Jun | 3725 | 31-May-15 |
| 10772 | 2 | 3 | | | III-2 | Jup | 3728 | 22-May-15 |
| 10779 | 2 | 3 | | | III-2 | Kansl1 | 284058 | 23-May-15 |
| 10782 | 2 | 3 | | | III-2 | Kansl3 | 55683 | 4-May-15 |
| 10785 | 2 | 3 | | | III-2 | Kat2a | 2648 | 17-May-15 |
| 10791 | 2 | 3 | | | III-2 | Kat8 | 84148 | 4-May-15 |
| 10794 | 2 | 3 | | | III-2 | Katnal2 | 83473 | 4-May-15 |
| 10803 | 2 | 3 | | | III-2 | Kbtbd3 | 143879 | 4-May-15 |
| 10805 | 2 | 3 | | | III-2 | Kbtbd7 | 84078 | 4-May-15 |
| 10810 | 2 | 3 | | | III-2 | Kcna2 | 3737 | 22-May-15 |
| 10819 | 2 | 3 | | | III-2 | Kcnb1 | 3745 | 4-May-15 |
| 10827 | 2 | 3 | | | III-2 | Kcnd3 | 3752 | 22-May-15 |
| 10837 | 2 | 3 | | | III-2 | Kcng3 | 170850 | 4-May-15 |
| 10847 | 2 | 3 | | | III-2 | Kcnip1 | 30820 | 4-May-15 |
| 10852 | 2 | 3 | | | III-2 | Kcnj10 | 3766 | 23-May-15 |
| 10862 | 2 | 3 | | | III-2 | Kcnj5 | 3762 | 23-May-15 |
| 10869 | 2 | 3 | | | III-2 | Kcnk13 | 56659 | 4-May-15 |
| 10873 | 2 | 3 | | | III-2 | Kcnk2 | 3776 | 4-May-15 |
| 10882 | 2 | 3 | | | III-2 | Kcnmb2 | 10242 | 7-Jun-15 |
| 10883 | 2 | 3 | | | III-2 | Kcnmb3 | 27094 | 4-May-15 |
| 10892 | 2 | 3 | | | III-2 | Kcnq2 | 3785 | 23-May-15 |
| 10894 | 2 | 3 | | | III-2 | Kcnq4 | 9132 | 23-May-15 |
| 10897 | 2 | 3 | | | III-2 | Kcns1 | 3787 | 4-May-15 |
| 10908 | 2 | 3 | | | III-2 | Kctd11 | 147040 | 4-May-15 |
| 10911 | 2 | 3 | | | III-2 | Kctd13 | 253980 | 4-May-15 |
| 10915 | 2 | 3 | | | III-2 | Kctd17 | 79734 | 27-May-15 |
| 10917 | 2 | 3 | | | III-2 | Kctd19 | 146212 | 4-May-15 |
| 10922 | 2 | 3 | | | III-2 | Kctd4 | 386618 | 4-May-15 |
| 10929 | 2 | 3 | | | III-2 | Kdelc2 | 143888 | 4-May-15 |
| 10935 | 2 | 3 | | | III-2 | Kdm1b | 221656 | 31-May-15 |
| 10946 | 2 | 3 | | | III-2 | Kdm5c | 8242 | 23-May-15 |
| 10947 | 2 | 3 | | | III-2 | Kdm5d | 8284 | 23-May-15 |
| 10967 | 2 | 3 | | | III-2 | Khsrp | 8570 | 31-May-15 |
| 10968 | 2 | 3 | | | III-2 | Kidins220 | 57498 | 4-May-15 |
| 10971 | 2 | 3 | | | III-2 | Kif13a | 63971 | 4-May-15 |
| 10972 | 2 | 3 | | | III-2 | Kif13b | 23303 | 4-May-15 |
| 10980 | 2 | 3 | | | III-2 | Kif1a | 547 | 23-May-15 |
| 10993 | 2 | 3 | | | III-2 | Kif2a | 3796 | 3-May-15 |
| 10995 | 2 | 3 | | | III-2 | Kif2c | 11004 | 3-May-15 |
| 10997 | 2 | 3 | | | III-2 | Kif3b | 9371 | 4-May-15 |
| 10999 | 2 | 3 | | | III-2 | Kif4 | 24137 | 4-May-15 |
| 11001 | 2 | 3 | | | III-2 | Kif5a | 3798 | 10-May-15 |
| 11005 | 2 | 3 | | | III-2 | Kif7 | 374654 | 23-May-15 |
| 11007 | 2 | 3 | | | III-2 | Kifap3 | 22920 | 4-May-15 |
| 11008 | 2 | 3 | | | III-2 | Kifc1 | 3833 | 4-May-15 |
| 11014 | 2 | 3 | | | III-2 | Kir3dl2 | 3812 | 12-May-15 |
| 11021 | 2 | 3 | | | III-2 | Kit | 3815 | 24-May-15 |
| 11049 | 2 | 3 | | | III-2 | Klhdc2 | 23588 | 4-May-15 |
| 11054 | 2 | 3 | | | III-2 | Klhdc8a | 55220 | 4-May-15 |
| 11061 | 2 | 3 | | | III-2 | Klhl13 | 90293 | 4-May-15 |
| 11065 | 2 | 3 | | | III-2 | Klhl18 | 23276 | 4-May-15 |
| 11068 | 2 | 3 | | | III-2 | Klhl21 | 9903 | 4-May-15 |
| 11083 | 2 | 3 | | | III-2 | Klhl36 | 79786 | 12-May-15 |
| 11089 | 2 | 3 | | | III-2 | Klhl5 | 51088 | 12-May-15 |
| 11090 | 2 | 3 | | | III-2 | Klhl6 | 89857 | 7-Jun-15 |
| 11092 | 2 | 3 | | | III-2 | Klhl8 | 57563 | 12-May-15 |
| 11116 | 2 | 3 | | | III-2 | Klk5 | 25818 | 4-May-15 |
| 11125 | 2 | 3 | | | III-2 | Klra13-ps | | |
| 11127 | 2 | 3 | | | III-2 | Klra15 | | |
| 11128 | 2 | 3 | | | III-2 | Klra17 | | |
| 11131 | 2 | 3 | | | III-2 | Klra2 | | |
| 11133 | 2 | 3 | | | III-2 | Klra22 | | |
| 11137 | 2 | 3 | | | III-2 | Klra4 | | |
| 11148 | 2 | 3 | | | III-2 | Klrb1-ps1 | | |
| 11157 | 2 | 3 | | | III-2 | Klri2 | | |
| 11166 | 2 | 3 | | | III-2 | Kndc1 | 85442 | 4-May-15 |
| 11173 | 2 | 3 | | | III-2 | Kpna2 | 3838 | 17-May-15 |
| 11175 | 2 | 3 | | | III-2 | Kpna4 | 3840 | 4-May-15 |
| 11182 | 2 | 3 | | | III-2 | Krba1 | 84626 | 12-May-15 |
| 11183 | 2 | 3 | | | III-2 | Krcc1 | 51315 | 21-May-15 |
| 11184 | 2 | 3 | | | III-2 | Kremen1 | 83999 | 21-May-15 |
| 11204 | 2 | 3 | | | III-2 | Krt25 | 147183 | 4-May-15 |
| 11210 | 2 | 3 | | | III-2 | Krt33a | 3883 | 4-May-15 |
| 11226 | 2 | 3 | | | III-2 | Krt74 | 121391 | 7-Jun-15 |
| 11227 | 2 | 3 | | | III-2 | Krt75 | 9119 | 4-May-15 |
| 11228 | 2 | 3 | | | III-2 | Krt76 | 51350 | 4-May-15 |
| 11269 | 2 | 3 | | | III-2 | Krtap31-2 | | |
| 11298 | 2 | 3 | | | III-2 | Kti12 | 112970 | 4-May-15 |

Fig.22 - 48

| | | | | | | | | |
|---|---|---|---|---|---|---|---|---|
| 11299 | 2 | 3 | | | III-2 | Ktn1 | 3895 | 4-May-15 |
| 11308 | 2 | 3 | | | III-2 | L3mbtl2 | 83746 | 21-May-15 |
| 11314 | 2 | 3 | | | III-2 | Lactb | 114294 | 4-May-15 |
| 11316 | 2 | 3 | | | III-2 | Lactbl1 | 646262 | 4-May-15 |
| 11322 | 2 | 3 | | | III-2 | Lama1 | 284217 | 17-May-15 |
| 11334 | 2 | 3 | | | III-2 | Lamp2 | 3920 | 12-May-15 |
| 11337 | 2 | 3 | | | III-2 | Lamtor1 | 55004 | 4-May-15 |
| 11344 | 2 | 3 | | | III-2 | Lancl3 | 347404 | 4-May-15 |
| 11348 | 2 | 3 | | | III-2 | Laptm4b | 55353 | 17-May-15 |
| 11352 | 2 | 3 | | | III-2 | Larp1b | 55132 | 28-May-15 |
| 11354 | 2 | 3 | | | III-2 | Larp4b | 23185 | 24-May-15 |
| 11357 | 2 | 3 | | | III-2 | Lars | 51520 | 4-May-15 |
| 11364 | 2 | 3 | | | III-2 | Lats2 | 26524 | 4-May-15 |
| 11398 | 2 | 3 | | | III-2 | Lcmt1 | 51451 | 4-May-15 |
| 11405 | 2 | 3 | | | III-2 | Lcn4 | | |
| 11417 | 2 | 3 | | | III-2 | Ldb2 | 9079 | 12-May-15 |
| 11426 | 2 | 3 | | | III-2 | Ldlrad2 | 401944 | 4-May-15 |
| 11430 | 2 | 3 | | | III-2 | Ldoc1 | 23641 | 21-May-15 |
| 11432 | 2 | 3 | | | III-2 | Leap2 | 116842 | 4-May-15 |
| 11438 | 2 | 3 | | | III-2 | Lekr1 | 389170 | 4-May-15 |
| 11445 | 2 | 3 | | | III-2 | Leng8 | 114823 | 4-May-15 |
| 11452 | 2 | 3 | | | III-2 | Leprel2 | 10536 | 4-May-15 |
| 11453 | 2 | 3 | | | III-2 | Leprel4 | 10609 | 12-May-15 |
| 11469 | 2 | 3 | | | III-2 | Lgals9 | 3965 | 17-May-15 |
| 11470 | 2 | 3 | | | III-2 | Lgalsl | 29094 | 12-May-15 |
| 11479 | 2 | 3 | | | III-2 | Lgsn | 51557 | 4-May-15 |
| 11482 | 2 | 3 | | | III-2 | Lhfp | 10186 | 12-May-15 |
| 11483 | 2 | 3 | | | III-2 | Lhfpl1 | 340596 | 4-May-15 |
| 11486 | 2 | 3 | | | III-2 | Lhfpl4 | 375323 | 4-May-15 |
| 11502 | 2 | 3 | | | III-2 | Lig3 | 3980 | 16-Jun-15 |
| 11504 | 2 | 3 | | | III-2 | Lilra5 | 353514 | 4-May-15 |
| 11508 | 2 | 3 | | | III-2 | Lima1 | 51474 | 12-May-15 |
| 11511 | 2 | 3 | | | III-2 | Limd2 | 80774 | 4-May-15 |
| 11513 | 2 | 3 | | | III-2 | Limk1 | 3984 | 4-May-15 |
| 11515 | 2 | 3 | | | III-2 | Lims1 | 3987 | 24-May-15 |
| 11518 | 2 | 3 | | | III-2 | Lin28b | 389421 | 4-May-15 |
| 11521 | 2 | 3 | | | III-2 | Lin54 | 132660 | 1-Jun-15 |
| 11525 | 2 | 3 | | | III-2 | Lin9 | 286826 | 4-May-15 |
| 11529 | 2 | 3 | | | III-2 | Lingo3 | 645191 | 4-May-15 |
| 11531 | 2 | 3 | | | III-2 | Lins | 55180 | 4-May-15 |
| 11549 | 2 | 3 | | | III-2 | Llgl1 | 3996 | 4-May-15 |
| 11562 | 2 | 3 | | | III-2 | Lmf2 | 91289 | 4-May-15 |
| 11567 | 2 | 3 | | | III-2 | Lmo1 | 4004 | 17-May-15 |
| 11570 | 2 | 3 | | | III-2 | Lmo4 | 8543 | 7-Jun-15 |
| 11578 | 2 | 3 | | | III-2 | Lmx1b | 4010 | 23-May-15 |
| 11583 | 2 | 3 | | | III-2 | LOC100038947 | | |
| 11585 | 2 | 3 | | | III-2 | LOC100043315 | | |
| 11588 | 2 | 3 | | | III-2 | LOC100503280 | | |
| 11595 | 2 | 3 | | | III-2 | LOC100861615 | | |
| 11598 | 2 | 3 | | | III-2 | LOC100862268 | | |
| 11601 | 2 | 3 | | | III-2 | LOC101056043 | | |
| 11624 | 2 | 3 | | | III-2 | Lonp1 | 9361 | 23-May-15 |
| 11632 | 2 | 3 | | | III-2 | Loxl1 | 4016 | 12-May-15 |
| 11635 | 2 | 3 | | | III-2 | Loxl4 | 84171 | 4-May-15 |
| 11640 | 2 | 3 | | | III-2 | Lpar5 | 57121 | 4-May-15 |
| 11642 | 2 | 3 | | | III-2 | Lpcat1 | 79888 | 4-May-15 |
| 11643 | 2 | 3 | | | III-2 | Lpcat2 | 54947 | 12-May-15 |
| 11659 | 2 | 3 | | | III-2 | Lrba | 987 | 7-Jun-15 |
| 11667 | 2 | 3 | | | III-2 | Lrfn3 | 79414 | 4-May-15 |
| 11672 | 2 | 3 | | | III-2 | Lrif1 | 55791 | 4-May-15 |
| 11674 | 2 | 3 | | | III-2 | Lrig2 | 9860 | 7-Jun-15 |
| 11684 | 2 | 3 | | | III-2 | Lrp1b | 53353 | 12-May-15 |
| 11685 | 2 | 3 | | | III-2 | Lrp2 | 4036 | 23-May-15 |
| 11691 | 2 | 3 | | | III-2 | Lrp8 | 7804 | 12-May-15 |
| 11693 | 2 | 3 | | | III-2 | Lrpprc | 10128 | 7-Jun-15 |
| 11696 | 2 | 3 | | | III-2 | Lrrc10 | 376132 | 4-May-15 |
| 11698 | 2 | 3 | | | III-2 | Lrrc14 | 9684 | 4-May-15 |
| 11714 | 2 | 3 | | | III-2 | Lrrc29 | 26231 | 4-May-15 |
| 11719 | 2 | 3 | | | III-2 | Lrrc36 | 55282 | 4-May-15 |
| 11725 | 2 | 3 | | | III-2 | Lrrc41 | 10489 | 12-May-15 |
| 11726 | 2 | 3 | | | III-2 | Lrrc42 | 115353 | 4-May-15 |
| 11738 | 2 | 3 | | | III-2 | Lrrc56 | 115399 | 4-May-15 |
| 11740 | 2 | 3 | | | III-2 | Lrrc58 | 116064 | 4-May-15 |
| 11747 | 2 | 3 | | | III-2 | Lrrc7 | 57554 | 14-May-15 |
| 11758 | 2 | 3 | | | III-2 | Lrrc8e | 80131 | 4-May-15 |
| 11762 | 2 | 3 | | | III-2 | Lrrfip1 | 9208 | 4-May-15 |
| 11772 | 2 | 3 | | | III-2 | Lrrn4 | 164312 | 13-Jun-15 |
| 11773 | 2 | 3 | | | III-2 | Lrrn4cl | 221091 | 4-May-15 |
| 11775 | 2 | 3 | | | III-2 | Lrrtm2 | 26045 | 2-Jun-15 |
| 11779 | 2 | 3 | | | III-2 | Lrtm1 | 57408 | 12-May-15 |
| 11787 | 2 | 3 | | | III-2 | Lsm12 | 124801 | 21-May-15 |
| 11815 | 2 | 3 | | | III-2 | Ltn1 | 26046 | 23-May-15 |
| 11816 | 2 | 3 | | | III-2 | Ltv1 | 84946 | 12-May-15 |
| 11818 | 2 | 3 | | | III-2 | Luc7l2 | 51631 | 4-May-15 |
| 11825 | 2 | 3 | | | III-2 | Luzp4 | 51213 | 4-May-15 |
| 11828 | 2 | 3 | | | III-2 | Ly6c1 | | |
| 11829 | 2 | 3 | | | III-2 | Ly6c2 | | |
| 11838 | 2 | 3 | | | III-2 | Ly6g6f | 259215 | 4-May-15 |
| 11861 | 2 | 3 | | | III-2 | Lypla2 | 11313 | 4-May-15 |
| 11862 | 2 | 3 | | | III-2 | Lyplal1 | 127018 | 4-May-15 |
| 11865 | 2 | 3 | | | III-2 | Lyrm4 | 57128 | 12-May-15 |
| 11867 | 2 | 3 | | | III-2 | Lyrm7 | 90624 | 12-May-15 |
| 11869 | 2 | 3 | | | III-2 | Lyrm9 | 201229 | 4-May-15 |
| 11871 | 2 | 3 | | | III-2 | Lysmd2 | 256586 | 4-May-15 |
| 11879 | 2 | 3 | | | III-2 | Lyzl4 | 131375 | 12-May-15 |
| 11890 | 2 | 3 | | | III-2 | Maats1 | 89876 | 4-May-15 |
| 11894 | 2 | 3 | | | III-2 | Macc1 | 346389 | 17-May-15 |
| 11898 | 2 | 3 | | | III-2 | Mad1l1 | 8379 | 24-May-15 |
| 11905 | 2 | 3 | | | III-2 | Mael | 84944 | 4-May-15 |
| 11932 | 2 | 3 | | | III-2 | Magee1 | 57692 | 7-Jun-15 |
| 11933 | 2 | 3 | | | III-2 | Magee2 | 139599 | 4-May-15 |
| 11934 | 2 | 3 | | | III-2 | Mageh1 | 28986 | 4-May-15 |
| 11936 | 2 | 3 | | | III-2 | Magi1 | 9223 | 7-Jun-15 |
| 11941 | 2 | 3 | | | III-2 | Magohb | 55110 | 12-May-15 |
| 11943 | 2 | 3 | | | III-2 | Mak | 4117 | 7-Jun-15 |
| 11951 | 2 | 3 | | | III-2 | Mamdc2 | 256691 | 4-May-15 |
| 11953 | 2 | 3 | | | III-2 | Maml1 | 9794 | 4-May-15 |
| 11960 | 2 | 3 | | | III-2 | Man1b1 | 11253 | 17-May-15 |
| 11969 | 2 | 3 | | | III-2 | Manbal | 63905 | 4-May-15 |
| 11974 | 2 | 3 | | | III-2 | Mansc1 | 54682 | 4-May-15 |
| 11979 | 2 | 3 | | | III-2 | Map1a | 4130 | 7-Jun-15 |
| 11983 | 2 | 3 | | | III-2 | Map1s | 55201 | 21-May-15 |
| 11986 | 2 | 3 | | | III-2 | Map2k2 | 5605 | 7-Jun-15 |
| 11990 | 2 | 3 | | | III-2 | Map2k5 | 5607 | 3-May-15 |
| 11993 | 2 | 3 | | | III-2 | Map3k1 | 4214 | 12-May-15 |
| 11995 | 2 | 3 | | | III-2 | Map3k11 | 4296 | 4-May-15 |
| 12000 | 2 | 3 | | | III-2 | Map3k19 | 80122 | 21-May-15 |
| 12011 | 2 | 3 | | | III-2 | Map4k1 | 11184 | 4-May-15 |
| 12012 | 2 | 3 | | | III-2 | Map4k2 | 5871 | 3-May-15 |
| 12016 | 2 | 3 | | | III-2 | Map6 | 4135 | 4-May-15 |
| 12028 | 2 | 3 | | | III-2 | Mapk15 | 225689 | 3-May-15 |
| 12038 | 2 | 3 | | | III-2 | Mapk8ip3 | 23162 | 4-May-15 |
| 12039 | 2 | 3 | | | III-2 | Mapk9 | 5601 | 17-May-15 |
| 12051 | 2 | 3 | | | III-2 | March1 | 55016 | 12-May-15 |
| 12053 | 2 | 3 | | | III-2 | March11 | 441061 | 4-May-15 |
| 12057 | 2 | 3 | | | III-2 | March5 | 54708 | 31-May-15 |
| 12072 | 2 | 3 | | | III-2 | Mars2 | 92935 | 4-May-15 |
| 12079 | 2 | 3 | | | III-2 | Mast1 | 22983 | 7-Jun-15 |
| 12089 | 2 | 3 | | | III-2 | Matn2 | 4147 | 14-May-15 |
| 12091 | 2 | 3 | | | III-2 | Matn4 | 8785 | 4-May-15 |
| 12093 | 2 | 3 | | | III-2 | Mau2 | 23383 | 4-May-15 |
| 12099 | 2 | 3 | | | III-2 | Mb21d2 | 151963 | 4-May-15 |
| 12101 | 2 | 3 | | | III-2 | Mbd2 | 8932 | 7-Jun-15 |
| 12123 | 2 | 3 | | | III-2 | Mbtps2 | 51360 | 4-May-15 |
| 12131 | 2 | 3 | | | III-2 | Mcc | 4163 | 28-May-15 |
| 12134 | 2 | 3 | | | III-2 | Mccc2 | 64087 | 23-May-15 |
| 12136 | 2 | 3 | | | III-2 | Mcemp1 | 199675 | 4-May-15 |
| 12137 | 2 | 3 | | | III-2 | Mcf2 | 4168 | 4-May-15 |
| 12147 | 2 | 3 | | | III-2 | Mcm4 | 4173 | 4-May-15 |
| 12153 | 2 | 3 | | | III-2 | Mcmbp | 79892 | 4-May-15 |
| 12154 | 2 | 3 | | | III-2 | Mcmdc2 | 157777 | 4-May-15 |
| 12163 | 2 | 3 | | | III-2 | Mcpt9 | | |
| 12177 | 2 | 3 | | | III-2 | Mdh1 | 4190 | 28-May-15 |
| 12185 | 2 | 3 | | | III-2 | Mdp1 | 145553 | 7-Jun-15 |
| 12200 | 2 | 3 | | | III-2 | Med13l | 23389 | 28-May-15 |
| 12203 | 2 | 3 | | | III-2 | Med16 | 10025 | 12-May-15 |
| 12205 | 2 | 3 | | | III-2 | Med18 | 54797 | 28-May-15 |
| 12210 | 2 | 3 | | | III-2 | Med23 | 9439 | 21-May-15 |
| 12216 | 2 | 3 | | | III-2 | Med29 | 55588 | 4-May-15 |
| 12220 | 2 | 3 | | | III-2 | Med6 | 10001 | 4-May-15 |
| 12223 | 2 | 3 | | | III-2 | Med9 | 55090 | 28-May-15 |
| 12226 | 2 | 3 | | | III-2 | Mef2a | 4205 | 17-May-15 |
| 12238 | 2 | 3 | | | III-2 | Mei4 | 101928601 | 4-May-15 |
| 12244 | 2 | 3 | | | III-2 | Melk | 9833 | 4-May-15 |
| 12247 | 2 | 3 | | | III-2 | Meox1 | 4222 | 12-May-15 |
| 12250 | 2 | 3 | | | III-2 | Mep1b | 4225 | 12-May-15 |
| 12251 | 2 | 3 | | | III-2 | Mepce | 56257 | 4-May-15 |
| 12252 | 2 | 3 | | | III-2 | Mepe | 56955 | 12-May-15 |
| 12261 | 2 | 3 | | | III-2 | Metap1d | 254042 | 4-May-15 |
| 12269 | 2 | 3 | | | III-2 | Mettl14 | 57721 | 4-May-15 |
| 12272 | 2 | 3 | | | III-2 | Mettl17 | 64745 | 4-May-15 |
| 12284 | 2 | 3 | | | III-2 | Mettl4 | 64863 | 12-May-15 |
| 12290 | 2 | 3 | | | III-2 | Mettl7a3 | | |
| 12293 | 2 | 3 | | | III-2 | Mettl9 | 51108 | 21-May-15 |
| 12296 | 2 | 3 | | | III-2 | Mex3c | 51320 | 28-May-15 |
| 12297 | 2 | 3 | | | III-2 | Mex3d | 399664 | 4-May-15 |
| 12299 | 2 | 3 | | | III-2 | Mfap1b | | |
| 12308 | 2 | 3 | | | III-2 | Mfi2 | 4241 | 4-May-15 |
| 12314 | 2 | 3 | | | III-2 | Mfsd10 | 10227 | 4-May-15 |
| 12316 | 2 | 3 | | | III-2 | Mfsd12 | 126321 | 12-May-15 |
| 12322 | 2 | 3 | | | III-2 | Mfsd6 | 54842 | 4-May-15 |
| 12330 | 2 | 3 | | | III-2 | Mgam | 8972 | 12-May-15 |
| 12344 | 2 | 3 | | | III-2 | Mgmt | 4255 | 17-May-15 |
| 12356 | 2 | 3 | | | III-2 | Mical1 | 64780 | 12-May-15 |
| 12357 | 2 | 3 | | | III-2 | Mical2 | 9645 | 12-May-15 |
| 12359 | 2 | 3 | | | III-2 | Micalcl | 84953 | 4-May-15 |
| 12363 | 2 | 3 | | | III-2 | Micu2 | 221154 | 4-May-15 |
| 12374 | 2 | 3 | | | III-2 | Mier3 | 166968 | 12-May-15 |
| 12383 | 2 | 3 | | | III-2 | Minos1 | 440574 | 2-Jun-15 |
| 12387 | 2 | 3 | | | III-2 | Mip | 4284 | 7-Jun-15 |
| 12388 | 2 | 3 | | | III-2 | Mipep | 4285 | 12-May-15 |
| 12391 | 2 | 3 | | | III-2 | Mir101a | | |
| 12414 | 2 | 3 | | | III-2 | Mir122a | 406906 | 7-Jun-15 |
| 12436 | 2 | 3 | | | III-2 | Mir129b | | |
| 12462 | 2 | 3 | | | III-2 | Mir145 | 406937 | 24-May-15 |
| 12481 | 2 | 3 | | | III-2 | Mir17hg | 407975 | 17-May-15 |
| 12483 | 2 | 3 | | | III-2 | Mir181a-1 | | |
| 12535 | 2 | 3 | | | III-2 | Mir1942 | 406970 | 21-May-15 |
| 12555 | 2 | 3 | | | III-2 | Mir195b | | |
| 12606 | 2 | 3 | | | III-2 | Mir216b | 100126319 | 21-May-15 |
| 12829 | 2 | 3 | | | III-2 | Mir448 | 554212 | 4-May-15 |
| 12878 | 2 | 3 | | | III-2 | Mir488 | 574441 | 21-May-15 |
| 12937 | 2 | 3 | | | III-2 | Mir551b | 693136 | 21-May-15 |

Fig.22 - 49

| | | | | | | | | |
|---|---|---|---|---|---|---|---|---|
| 12963 | 2 | 3 | | | III-2 | Mir6238 | | |
| 12968 | 2 | 3 | | | III-2 | Mir6336 | | |
| 12974 | 2 | 3 | | | III-2 | Mir6342 | | |
| 12988 | 2 | 3 | | | III-2 | Mir6359 | | |
| 12991 | 2 | 3 | | | III-2 | Mir6362 | | |
| 12994 | 2 | 3 | | | III-2 | Mir6365 | | |
| 13020 | 2 | 3 | | | III-2 | Mir6394 | | |
| 13031 | 2 | 3 | | | III-2 | Mir6405 | | |
| 13050 | 2 | 3 | | | III-2 | Mir6537 | | |
| 13127 | 2 | 3 | | | III-2 | Mir6921 | | |
| 13196 | 2 | 3 | | | III-2 | Mir6983 | | |
| 13213 | 2 | 3 | | | III-2 | Mir700 | | |
| 13248 | 2 | 3 | | | III-2 | Mir7031 | | |
| 13283 | 2 | 3 | | | III-2 | Mir7062 | | |
| 13333 | 2 | 3 | | | III-2 | Mir7210 | | |
| 13377 | 2 | 3 | | | III-2 | Mir762 | 100313837 | 4-May-15 |
| 13431 | 2 | 3 | | | III-2 | Mir8101 | | |
| 13436 | 2 | 3 | | | III-2 | Mir8106 | | |
| 13446 | 2 | 3 | | | III-2 | Mir8116 | | |
| 13478 | 2 | 3 | | | III-2 | Mirlet7a-2 | | |
| 13482 | 2 | 3 | | | III-2 | Mirlet7c-2 | | |
| 13485 | 2 | 3 | | | III-2 | Mirlet7f-1 | | |
| 13495 | 2 | 3 | | | III-2 | Mitd1 | 129531 | 12-May-15 |
| 13500 | 2 | 3 | | | III-2 | Mkl1 | 57591 | 17-May-15 |
| 13502 | 2 | 3 | | | III-2 | Mkln1 | 4289 | 4-May-15 |
| 13503 | 2 | 3 | | | III-2 | Mkln1os | | |
| 13505 | 2 | 3 | | | III-2 | Mknk2 | 2872 | 4-May-15 |
| 13508 | 2 | 3 | | | III-2 | Mkrn3 | 7681 | 23-May-15 |
| 13513 | 2 | 3 | | | III-2 | Mlec | 9761 | 12-May-15 |
| 13516 | 2 | 3 | | | III-2 | Mlh1 | 4292 | 7-Jun-15 |
| 13521 | 2 | 3 | | | III-2 | Mllt10 | 8028 | 4-May-15 |
| 13535 | 2 | 3 | | | III-2 | Mmadhc | 27249 | 23-May-15 |
| 13540 | 2 | 3 | | | III-2 | Mmgt1 | 93380 | 4-May-15 |
| 13548 | 2 | 3 | | | III-2 | Mmp16 | 4325 | 17-May-15 |
| 13549 | 2 | 3 | | | III-2 | Mmp17 | 4326 | 4-May-15 |
| 13552 | 2 | 3 | | | III-2 | Mmp1b | | |
| 13555 | 2 | 3 | | | III-2 | Mmp21 | 118856 | 17-May-15 |
| 13559 | 2 | 3 | | | III-2 | Mmp27 | 64066 | 4-May-15 |
| 13568 | 2 | 3 | | | III-2 | Mms22l | 253714 | 4-May-15 |
| 13573 | 2 | 3 | | | III-2 | Mnda | 4332 | 4-May-15 |
| 13577 | 2 | 3 | | | III-2 | Mnx1 | 3110 | 12-May-15 |
| 13582 | 2 | 3 | | | III-2 | Mob3a | 126308 | 4-May-15 |
| 13584 | 2 | 3 | | | III-2 | Mob3c | 148932 | 4-May-15 |
| 13592 | 2 | 3 | | | III-2 | Mogat1 | 116255 | 4-May-15 |
| 13597 | 2 | 3 | | | III-2 | Mon1b | 22879 | 4-May-15 |
| 13605 | 2 | 3 | | | III-2 | Morf4l2 | 9643 | 4-May-15 |
| 13611 | 2 | 3 | | | III-2 | Mos | 4342 | 7-Jun-15 |
| 13615 | 2 | 3 | | | III-2 | Mospd4 | | |
| 13618 | 2 | 3 | | | III-2 | Moxd1 | 26002 | 4-May-15 |
| 13620 | 2 | 3 | | | III-2 | Mpc1 | 51660 | 7-Jun-15 |
| 13622 | 2 | 3 | | | III-2 | Mpdu1 | 9526 | 4-May-15 |
| 13623 | 2 | 3 | | | III-2 | Mpdz | 8777 | 7-Jun-15 |
| 13630 | 2 | 3 | | | III-2 | Mpi | 4351 | 7-Jun-15 |
| 13633 | 2 | 3 | | | III-2 | Mpnd | 84954 | 4-May-15 |
| 13645 | 2 | 3 | | | III-2 | Mprip | 23164 | 12-May-15 |
| 13646 | 2 | 3 | | | III-2 | Mpst | 4357 | 7-Jun-15 |
| 13650 | 2 | 3 | | | III-2 | Mpv17l | 255027 | 4-May-15 |
| 13665 | 2 | 3 | | | III-2 | Mrgbp | 55257 | 4-May-15 |
| 13685 | 2 | 3 | | | III-2 | Mrgprx2 | 117194 | 12-May-15 |
| 13689 | 2 | 3 | | | III-2 | Mroh1 | 727957 | 4-May-15 |
| 13696 | 2 | 3 | | | III-2 | Mroh8 | 140699 | 12-May-15 |
| 13700 | 2 | 3 | | | III-2 | Mrpl11 | 65003 | 12-May-15 |
| 13705 | 2 | 3 | | | III-2 | Mrpl16 | 54948 | 4-May-15 |
| 13709 | 2 | 3 | | | III-2 | Mrpl2 | 51069 | 13-Jun-15 |
| 13715 | 2 | 3 | | | III-2 | Mrpl27 | 51264 | 7-Jun-15 |
| 13722 | 2 | 3 | | | III-2 | Mrpl35 | 51318 | 4-May-15 |
| 13723 | 2 | 3 | | | III-2 | Mrpl36 | 64979 | 4-May-15 |
| 13730 | 2 | 3 | | | III-2 | Mrpl42 | 28977 | 4-May-15 |
| 13735 | 2 | 3 | | | III-2 | Mrpl47 | 57129 | 4-May-15 |
| 13738 | 2 | 3 | | | III-2 | Mrpl50 | 54534 | 14-May-15 |
| 13739 | 2 | 3 | | | III-2 | Mrpl51 | 51258 | 28-May-15 |
| 13742 | 2 | 3 | | | III-2 | Mrpl54 | 116541 | 21-May-15 |
| 13744 | 2 | 3 | | | III-2 | Mrpl57 | 78988 | 28-May-15 |
| 13745 | 2 | 3 | | | III-2 | Mrpl9 | 65005 | 4-May-15 |
| 13746 | 2 | 3 | | | III-2 | Mrps10 | 55173 | 28-May-15 |
| 13749 | 2 | 3 | | | III-2 | Mrps14 | 63931 | 12-May-15 |
| 13753 | 2 | 3 | | | III-2 | Mrps18a | 55168 | 28-May-15 |
| 13757 | 2 | 3 | | | III-2 | Mrps21 | 54460 | 28-May-15 |
| 13760 | 2 | 3 | | | III-2 | Mrps24 | 64951 | 28-May-15 |
| 13765 | 2 | 3 | | | III-2 | Mrps30 | 10884 | 12-May-15 |
| 13772 | 2 | 3 | | | III-2 | Mrps6 | 64968 | 28-May-15 |
| 13774 | 2 | 3 | | | III-2 | Mrps9 | 64965 | 4-May-15 |
| 13778 | 2 | 3 | | | III-2 | Mrvi1 | 10335 | 12-May-15 |
| 13781 | 2 | 3 | | | III-2 | Ms4a13 | 503497 | 12-May-15 |
| 13797 | 2 | 3 | | | III-2 | Msantd3 | 91283 | 4-May-15 |
| 13801 | 2 | 3 | | | III-2 | Msh2 | 4436 | 23-May-15 |
| 13814 | 2 | 3 | | | III-2 | Msmb | 4477 | 12-May-15 |
| 13817 | 2 | 3 | | | III-2 | Msn | 4478 | 17-May-15 |
| 13820 | 2 | 3 | | | III-2 | Msrb1 | 51734 | 12-May-15 |
| 13830 | 2 | 3 | | | III-2 | Msx2 | 4488 | 23-May-15 |
| 13837 | 2 | 3 | | | III-2 | Mta2 | 9219 | 4-May-15 |
| 13841 | 2 | 3 | | | III-2 | Mtap7d3 | | |
| 13843 | 2 | 3 | | | III-2 | Mtch1 | 23787 | 14-May-15 |
| 13851 | 2 | 3 | | | III-2 | Mterfd2 | 130916 | 4-May-15 |
| 13854 | 2 | 3 | | | III-2 | Mtf2 | 22823 | 4-May-15 |
| 13862 | 2 | 3 | | | III-2 | Mthfd1 | 4522 | 21-May-15 |
| 13869 | 2 | 3 | | | III-2 | Mtif2 | 4528 | 12-May-15 |
| 13879 | 2 | 3 | | | III-2 | Mtmr3 | 8897 | 24-May-15 |
| 13889 | 2 | 3 | | | III-2 | Mtpn | 136319 | 4-May-15 |
| 13890 | 2 | 3 | | | III-2 | Mtr | 4548 | 23-May-15 |
| 13893 | 2 | 3 | | | III-2 | Mtrr | 4552 | 23-May-15 |
| 13894 | 2 | 3 | | | III-2 | Mtss1 | 9788 | 3-May-15 |
| 13914 | 2 | 3 | | | III-2 | Mug1 | | |
| 13917 | 2 | 3 | | | III-2 | Mul1 | 79594 | 23-May-15 |
| 13945 | 2 | 3 | | | III-2 | Mutyh | 4595 | 23-May-15 |
| 13956 | 2 | 3 | | | III-2 | Mxi1 | 4601 | 4-May-15 |
| 13957 | 2 | 3 | | | III-2 | Mxra7 | 439921 | 12-May-15 |
| 13977 | 2 | 3 | | | III-2 | Myct1 | 80177 | 4-May-15 |
| 13989 | 2 | 3 | | | III-2 | Myh15 | 22989 | 12-May-15 |
| 14000 | 2 | 3 | | | III-2 | Myl12a | 10627 | 12-May-15 |
| 14016 | 2 | 3 | | | III-2 | Myo10 | 4651 | 31-May-15 |
| 14018 | 2 | 3 | | | III-2 | Myo16 | 23026 | 4-May-15 |
| 14019 | 2 | 3 | | | III-2 | Myo18a | 399687 | 4-May-15 |
| 14030 | 2 | 3 | | | III-2 | Myo3a | 53904 | 23-May-15 |
| 14064 | 2 | 3 | | | III-2 | Mzt2 | | |
| 14065 | 2 | 3 | | | III-2 | N28178 | | |
| 14067 | 2 | 3 | | | III-2 | N4bp2 | 55728 | 12-May-15 |
| 14071 | 2 | 3 | | | III-2 | N6amt1 | 29104 | 4-May-15 |
| 14075 | 2 | 3 | | | III-2 | Naa15 | 80155 | 4-May-15 |
| 14078 | 2 | 3 | | | III-2 | Naa25 | 80018 | 4-May-15 |
| 14080 | 2 | 3 | | | III-2 | Naa35 | 60560 | 4-May-15 |
| 14083 | 2 | 3 | | | III-2 | Naa50 | 80218 | 2-Jun-15 |
| 14085 | 2 | 3 | | | III-2 | Naaa | 27163 | 4-May-15 |
| 14095 | 2 | 3 | | | III-2 | Nacc2 | 138151 | 28-May-15 |
| 14097 | 2 | 3 | | | III-2 | Nadk2 | 133686 | 4-May-15 |
| 14104 | 2 | 3 | | | III-2 | Nagpa | 51172 | 4-May-15 |
| 14105 | 2 | 3 | | | III-2 | Nags | 162417 | 3-May-15 |
| 14106 | 2 | 3 | | | III-2 | Naif1 | 203245 | 4-May-15 |
| 14108 | 2 | 3 | | | III-2 | Naip2 | | |
| 14113 | 2 | 3 | | | III-2 | Nampt | 10135 | 17-May-15 |
| 14123 | 2 | 3 | | | III-2 | Nap1l4 | 4676 | 12-May-15 |
| 14126 | 2 | 3 | | | III-2 | Napb | 63908 | 7-Jun-15 |
| 14132 | 2 | 3 | | | III-2 | Narfl | 64428 | 12-May-15 |
| 14133 | 2 | 3 | | | III-2 | Narg2 | 79664 | 4-May-15 |
| 14135 | 2 | 3 | | | III-2 | Nars2 | 79731 | 3-May-15 |
| 14138 | 2 | 3 | | | III-2 | Nat10 | 55226 | 4-May-15 |
| 14139 | 2 | 3 | | | III-2 | Nat14 | 57106 | 23-May-15 |
| 14141 | 2 | 3 | | | III-2 | Nat3 | | |
| 14149 | 2 | 3 | | | III-2 | Nbas | 51594 | 29-May-15 |
| 14159 | 2 | 3 | | | III-2 | Ncan | 1463 | 4-May-15 |
| 14166 | 2 | 3 | | | III-2 | Ncbp1 | 4686 | 4-May-15 |
| 14170 | 2 | 3 | | | III-2 | Nceh1 | 57552 | 12-May-15 |
| 14175 | 2 | 3 | | | III-2 | Nck2 | 8440 | 4-May-15 |
| 14180 | 2 | 3 | | | III-2 | Nckipsd | 51517 | 4-May-15 |
| 14186 | 2 | 3 | | | III-2 | Ncoa3 | 8202 | 17-May-15 |
| 14201 | 2 | 3 | | | III-2 | Ndfip1 | 80762 | 31-May-15 |
| 14205 | 2 | 3 | | | III-2 | Ndnl2 | 56160 | 4-May-15 |
| 14206 | 2 | 3 | | | III-2 | Ndor1 | 27158 | 4-May-15 |
| 14214 | 2 | 3 | | | III-2 | Ndst3 | 9348 | 4-May-15 |
| 14218 | 2 | 3 | | | III-2 | Ndufa11 | 126328 | 4-May-15 |
| 14232 | 2 | 3 | | | III-2 | Ndufaf2 | 91942 | 23-May-15 |
| 14235 | 2 | 3 | | | III-2 | Ndufaf5 | 79133 | 23-May-15 |
| 14238 | 2 | 3 | | | III-2 | Ndufb10 | 4716 | 12-May-15 |
| 14254 | 2 | 3 | | | III-2 | Ndufs5 | 4725 | 4-May-15 |
| 14255 | 2 | 3 | | | III-2 | Ndufs6 | 4726 | 4-May-15 |
| 14268 | 2 | 3 | | | III-2 | Necap2 | 55707 | 4-May-15 |
| 14271 | 2 | 3 | | | III-2 | Nedd4l | 23327 | 17-May-15 |
| 14283 | 2 | 3 | | | III-2 | Nek11 | 79858 | 28-May-15 |
| 14288 | 2 | 3 | | | III-2 | Nek6 | 10783 | 4-May-15 |
| 14292 | 2 | 3 | | | III-2 | Nelfa | 7469 | 4-May-15 |
| 14321 | 2 | 3 | | | III-2 | Neurog1 | 4762 | 12-May-15 |
| 14326 | 2 | 3 | | | III-2 | Nf2 | 4771 | 1-Jun-15 |
| 14333 | 2 | 3 | | | III-2 | Nfatc3 | 4775 | 24-May-15 |
| 14337 | 2 | 3 | | | III-2 | Nfe2l2 | 4780 | 31-May-15 |
| 14359 | 2 | 3 | | | III-2 | Nfyc | 4802 | 21-May-15 |
| 14360 | 2 | 3 | | | III-2 | Ngb | 58157 | 7-Jun-15 |
| 14371 | 2 | 3 | | | III-2 | Nhlh2 | 4808 | 28-May-15 |
| 14378 | 2 | 3 | | | III-2 | Nhs | 4810 | 17-May-15 |
| 14381 | 2 | 3 | | | III-2 | Nicn1 | 84276 | 4-May-15 |
| 14385 | 2 | 3 | | | III-2 | Nifk | 84365 | 4-May-15 |
| 14387 | 2 | 3 | | | III-2 | Nin | 51199 | 12-May-15 |
| 14388 | 2 | 3 | | | III-2 | Ninj1 | 4814 | 18-May-15 |
| 14398 | 2 | 3 | | | III-2 | Nipbl | 25836 | 23-May-15 |
| 14401 | 2 | 3 | | | III-2 | Nipsnap3b | 55335 | 12-May-15 |
| 14403 | 2 | 3 | | | III-2 | Nit1 | 4817 | 4-May-15 |
| 14407 | 2 | 3 | | | III-2 | Nkain3 | 286183 | 4-May-15 |
| 14410 | 2 | 3 | | | III-2 | Nkapl | 222698 | 4-May-15 |
| 14415 | 2 | 3 | | | III-2 | Nkiras2 | 28511 | 4-May-15 |
| 14424 | 2 | 3 | | | III-2 | Nkx2-3 | 159296 | 4-May-15 |
| 14431 | 2 | 3 | | | III-2 | Nkx6-1 | 4825 | 4-May-15 |
| 14434 | 2 | 3 | | | III-2 | Nle1 | 54475 | 4-May-15 |
| 14439 | 2 | 3 | | | III-2 | Nln | 57486 | 4-May-15 |
| 14440 | 2 | 3 | | | III-2 | Nlrc3 | 197358 | 4-May-15 |
| 14443 | 2 | 3 | | | III-2 | Nlrp10 | 338322 | 4-May-15 |
| 14444 | 2 | 3 | | | III-2 | Nlrp12 | 91662 | 4-May-15 |
| 14446 | 2 | 3 | | | III-2 | Nlrp1a | | |
| 14455 | 2 | 3 | | | III-2 | Nlrp4f | | |
| 14461 | 2 | 3 | | | III-2 | Nlrp9b | | |
| 14466 | 2 | 3 | | | III-2 | Nmd3 | 51068 | 4-May-15 |
| 14468 | 2 | 3 | | | III-2 | Nme2 | 4831 | 31-May-15 |
| 14469 | 2 | 3 | | | III-2 | Nme3 | 4832 | 12-May-15 |
| 14475 | 2 | 3 | | | III-2 | Nme9 | 347736 | 4-May-15 |
| 14479 | 2 | 3 | | | III-2 | Nmnat3 | 349565 | 4-May-15 |
| 14484 | 2 | 3 | | | III-2 | Nmt1 | 4836 | 4-May-15 |

Fig.22 - 50

| | | | | | | | | |
|---|---|---|---|---|---|---|---|---|
| 14503 | 2 | 3 | | | III-2 | Nol11 | 25926 | 4-May-15 |
| 14504 | 2 | 3 | | | III-2 | Nol12 | 79159 | 12-May-15 |
| 14507 | 2 | 3 | | | III-2 | Nol6 | 65083 | 4-May-15 |
| 14511 | 2 | 3 | | | III-2 | Nolc1 | 9221 | 12-May-15 |
| 14522 | 2 | 3 | | | III-2 | Nos1 | 4842 | 7-Jun-15 |
| 14532 | 2 | 3 | | | III-2 | Noto | 344022 | 4-May-15 |
| 14536 | 2 | 3 | | | III-2 | Nova2 | 4858 | 4-May-15 |
| 14539 | 2 | 3 | | | III-2 | Nox4 | 50507 | 10-May-15 |
| 14543 | 2 | 3 | | | III-2 | Npas1 | 4861 | 28-May-15 |
| 14546 | 2 | 3 | | | III-2 | Npas4 | 266743 | 28-May-15 |
| 14550 | 2 | 3 | | | III-2 | Npc1 | 4864 | 7-Jun-15 |
| 14556 | 2 | 3 | | | III-2 | Npepps | 9520 | 4-May-15 |
| 14561 | 2 | 3 | | | III-2 | Nphp3 | 27031 | 12-May-15 |
| 14568 | 2 | 3 | | | III-2 | Npm1 | 4869 | 17-May-15 |
| 14581 | 2 | 3 | | | III-2 | Nps | 594857 | 4-May-15 |
| 14592 | 2 | 3 | | | III-2 | Npy4r | 5540 | 4-May-15 |
| 14601 | 2 | 3 | | | III-2 | Nr1h2 | 7376 | 29-May-15 |
| 14611 | 2 | 3 | | | III-2 | Nr2e3 | 10002 | 23-May-15 |
| 14616 | 2 | 3 | | | III-2 | Nr3c2 | 4306 | 24-May-15 |
| 14627 | 2 | 3 | | | III-2 | Nrbf2 | 29982 | 4-May-15 |
| 14635 | 2 | 3 | | | III-2 | Nrg1 | 3084 | 12-May-15 |
| 14637 | 2 | 3 | | | III-2 | Nrg3 | 10718 | 4-May-15 |
| 14638 | 2 | 3 | | | III-2 | Nrg3os | | |
| 14650 | 2 | 3 | | | III-2 | Nrp | | |
| 14651 | 2 | 3 | | | III-2 | Nrp1 | 8829 | 7-Jun-15 |
| 14653 | 2 | 3 | | | III-2 | Nrros | 375387 | 4-May-15 |
| 14655 | 2 | 3 | | | III-2 | Nrsn2 | 80023 | 4-May-15 |
| 14658 | 2 | 3 | | | III-2 | Nrxn2 | 9379 | 4-May-15 |
| 14660 | 2 | 3 | | | III-2 | Nsa2 | 10412 | 4-May-15 |
| 14664 | 2 | 3 | | | III-2 | Nsfl1c | 55968 | 21-May-15 |
| 14669 | 2 | 3 | | | III-2 | Nsmce1 | 197370 | 23-May-15 |
| 14676 | 2 | 3 | | | III-2 | Nsun5 | 55695 | 4-May-15 |
| 14686 | 2 | 3 | | | III-2 | Nt5dc2 | 64943 | 4-May-15 |
| 14690 | 2 | 3 | | | III-2 | Ntan1 | 123803 | 4-May-15 |
| 14695 | 2 | 3 | | | III-2 | Ntmt1 | 28989 | 4-May-15 |
| 14698 | 2 | 3 | | | III-2 | Ntn4 | 59277 | 7-Jun-15 |
| 14700 | 2 | 3 | | | III-2 | Ntng1 | 22854 | 7-Jun-15 |
| 14703 | 2 | 3 | | | III-2 | Ntrk1 | 4914 | 23-May-15 |
| 14712 | 2 | 3 | | | III-2 | Nubp1 | 4682 | 10-Jun-15 |
| 14719 | 2 | 3 | | | III-2 | Nudcd1 | 84955 | 12-May-15 |
| 14722 | 2 | 3 | | | III-2 | Nudt1 | 4521 | 31-May-15 |
| 14727 | 2 | 3 | | | III-2 | Nudt14 | 256281 | 4-May-15 |
| 14749 | 2 | 3 | | | III-2 | Numb | 8650 | 4-May-15 |
| 14750 | 2 | 3 | | | III-2 | Numbl | 9253 | 12-May-15 |
| 14756 | 2 | 3 | | | III-2 | Nup188 | 23511 | 4-May-15 |
| 14757 | 2 | 3 | | | III-2 | Nup205 | 23165 | 12-May-15 |
| 14760 | 2 | 3 | | | III-2 | Nup214 | 8021 | 12-May-15 |
| 14778 | 2 | 3 | | | III-2 | Nusap1 | 51203 | 4-May-15 |
| 14782 | 2 | 3 | | | III-2 | Nvl | 4931 | 4-May-15 |
| 14797 | 2 | 3 | | | III-2 | Nxph2 | 11249 | 4-May-15 |
| 14801 | 2 | 3 | | | III-2 | Nxt2 | 55916 | 4-May-15 |
| 14804 | 2 | 3 | | | III-2 | Nynrin | 57523 | 4-May-15 |
| 14817 | 2 | 3 | | | III-2 | Oas2 | 4939 | 4-May-15 |
| 14828 | 2 | 3 | | | III-2 | Obox2 | | |
| 14838 | 2 | 3 | | | III-2 | Oca2 | 4948 | 23-May-15 |
| 14840 | 2 | 3 | | | III-2 | Ociad1 | 54940 | 4-May-15 |
| 14856 | 2 | 3 | | | III-2 | Ofcc1 | 266553 | 7-Jun-15 |
| 14861 | 2 | 3 | | | III-2 | Ogfod2 | 79676 | 23-May-15 |
| 14864 | 2 | 3 | | | III-2 | Ogfrl1 | 79627 | 12-May-15 |
| 14868 | 2 | 3 | | | III-2 | Oip5 | 11339 | 4-May-15 |
| 14882 | 2 | 3 | | | III-2 | Olfr10 | | |
| 14913 | 2 | 3 | | | III-2 | Olfr1037 | | |
| 15204 | 2 | 3 | | | III-2 | Olfr1377 | | |
| 15223 | 2 | 3 | | | III-2 | Olfr1395 | | |
| 15226 | 2 | 3 | | | III-2 | Olfr1402 | | |
| 15244 | 2 | 3 | | | III-2 | Olfr1424 | | |
| 15331 | 2 | 3 | | | III-2 | Olfr167 | | |
| 15381 | 2 | 3 | | | III-2 | Olfr228 | | |
| 15632 | 2 | 3 | | | III-2 | Olfr556 | | |
| 15636 | 2 | 3 | | | III-2 | Olfr56 | | |
| 15756 | 2 | 3 | | | III-2 | Olfr699 | | |
| 15823 | 2 | 3 | | | III-2 | Olfr781 | | |
| 15979 | 2 | 3 | | | III-2 | Olfr978 | | |
| 15983 | 2 | 3 | | | III-2 | Olfr981 | | |
| 16000 | 2 | 3 | | | III-2 | Olig3 | 167826 | 4-May-15 |
| 16002 | 2 | 3 | | | III-2 | Oma1 | 115209 | 3-May-15 |
| 16007 | 2 | 3 | | | III-2 | Omt2b | | |
| 16010 | 2 | 3 | | | III-2 | Onecut3 | 390874 | 28-May-15 |
| 16012 | 2 | 3 | | | III-2 | Oog1 | | |
| 16018 | 2 | 3 | | | III-2 | Oosp3 | | |
| 16022 | 2 | 3 | | | III-2 | Opcml | 4978 | 4-May-15 |
| 16026 | 2 | 3 | | | III-2 | Opn1sw | 611 | 12-May-15 |
| 16029 | 2 | 3 | | | III-2 | Opn5 | 221391 | 4-May-15 |
| 16036 | 2 | 3 | | | III-2 | Orai1 | 84876 | 21-May-15 |
| 16042 | 2 | 3 | | | III-2 | Orc3 | 23595 | 4-May-15 |
| 16048 | 2 | 3 | | | III-2 | Orm3 | | |
| 16050 | 2 | 3 | | | III-2 | Ormdl2 | 29095 | 4-May-15 |
| 16068 | 2 | 3 | | | III-2 | Osgep | 55644 | 4-May-15 |
| 16072 | 2 | 3 | | | III-2 | Osm | 5008 | 7-Jun-15 |
| 16082 | 2 | 3 | | | III-2 | Otoa | 146183 | 23-May-15 |
| 16089 | 2 | 3 | | | III-2 | Otop3 | 347741 | 4-May-15 |
| 16100 | 2 | 3 | | | III-2 | Otud5 | 55593 | 4-May-15 |
| 16101 | 2 | 3 | | | III-2 | Otud6a | 139562 | 4-May-15 |
| 16115 | 2 | 3 | | | III-2 | Oxa1l | 5018 | 4-May-15 |
| 16125 | 2 | 3 | | | III-2 | Oxt | 5020 | 4-May-15 |
| 16153 | 2 | 3 | | | III-2 | Pabpc5 | 140886 | 4-May-15 |
| 16160 | 2 | 3 | | | III-2 | Pacs2 | 23241 | 21-May-15 |

| | | | | | | | | |
|---|---|---|---|---|---|---|---|---|
| 16169 | 2 | 3 | | | III-2 | Paf1 | 54623 | 7-Jun-15 |
| 16178 | 2 | 3 | | | III-2 | Paip1 | 10605 | 12-May-15 |
| 16183 | 2 | 3 | | | III-2 | Pak2 | 5062 | 7-Jun-15 |
| 16192 | 2 | 3 | | | III-2 | Palm2 | 114299 | 4-May-15 |
| 16199 | 2 | 3 | | | III-2 | Pan3 | 255967 | 7-Jun-15 |
| 16202 | 2 | 3 | | | III-2 | Pank3 | 79646 | 4-May-15 |
| 16204 | 2 | 3 | | | III-2 | Panx1 | 24145 | 12-May-15 |
| 16206 | 2 | 3 | | | III-2 | Panx3 | 116337 | 4-May-15 |
| 16214 | 2 | 3 | | | III-2 | Papolb | 56903 | 12-May-15 |
| 16219 | 2 | 3 | | | III-2 | Papss2 | 9060 | 4-May-15 |
| 16222 | 2 | 3 | | | III-2 | Paqr5 | 54852 | 4-May-15 |
| 16223 | 2 | 3 | | | III-2 | Paqr6 | 79957 | 4-May-15 |
| 16235 | 2 | 3 | | | III-2 | Parl | 55486 | 24-May-15 |
| 16241 | 2 | 3 | | | III-2 | Parp12 | 64761 | 4-May-15 |
| 16242 | 2 | 3 | | | III-2 | Parp14 | 54625 | 4-May-15 |
| 16244 | 2 | 3 | | | III-2 | Parp2 | 10038 | 17-May-15 |
| 16250 | 2 | 3 | | | III-2 | Parpbp | 55010 | 4-May-15 |
| 16269 | 2 | 3 | | | III-2 | Pax6os1 | | |
| 16274 | 2 | 3 | | | III-2 | Paxip1 | 22976 | 31-May-15 |
| 16275 | 2 | 3 | | | III-2 | Pbdc1 | 51260 | 12-May-15 |
| 16280 | 2 | 3 | | | III-2 | Pbrm1 | 55193 | 4-May-15 |
| 16281 | 2 | 3 | | | III-2 | Pbsn | | |
| 16282 | 2 | 3 | | | III-2 | Pbx1 | 5087 | 28-May-15 |
| 16290 | 2 | 3 | | | III-2 | Pcbp2 | 5094 | 4-May-15 |
| 16297 | 2 | 3 | | | III-2 | Pcdh11x | 27328 | 12-May-15 |
| 16298 | 2 | 3 | | | III-2 | Pcdh12 | 51294 | 4-May-15 |
| 16300 | 2 | 3 | | | III-2 | Pcdh17 | 27253 | 4-May-15 |
| 16303 | 2 | 3 | | | III-2 | Pcdh20 | 64881 | 4-May-15 |
| 16304 | 2 | 3 | | | III-2 | Pcdh7 | 5099 | 4-May-15 |
| 16306 | 2 | 3 | | | III-2 | Pcdh9 | 5101 | 4-May-15 |
| 16308 | 2 | 3 | | | III-2 | Pcdha10 | 56139 | 4-May-15 |
| 16310 | 2 | 3 | | | III-2 | Pcdha12 | 56137 | 4-May-15 |
| 16321 | 2 | 3 | | | III-2 | Pcdhac2 | 56134 | 4-May-15 |
| 16327 | 2 | 3 | | | III-2 | Pcdhb14 | 56122 | 12-May-15 |
| 16329 | 2 | 3 | | | III-2 | Pcdhb16 | 57717 | 12-May-15 |
| 16332 | 2 | 3 | | | III-2 | Pcdhb19 | 84054 | 4-May-15 |
| 16336 | 2 | 3 | | | III-2 | Pcdhb22 | | |
| 16338 | 2 | 3 | | | III-2 | Pcdhb4 | 56131 | 12-May-15 |
| 16341 | 2 | 3 | | | III-2 | Pcdhb7 | 56129 | 4-May-15 |
| 16345 | 2 | 3 | | | III-2 | Pcdhga10 | 56106 | 4-May-15 |
| 16347 | 2 | 3 | | | III-2 | Pcdhga12 | 26025 | 4-May-15 |
| 16351 | 2 | 3 | | | III-2 | Pcdhga5 | 56110 | 4-May-15 |
| 16356 | 2 | 3 | | | III-2 | Pcdhgb1 | 56104 | 4-May-15 |
| 16363 | 2 | 3 | | | III-2 | Pcdhgc3 | 5098 | 4-May-15 |
| 16367 | 2 | 3 | | | III-2 | Pced1b | 91523 | 4-May-15 |
| 16369 | 2 | 3 | | | III-2 | Pcgf1 | 84759 | 4-May-15 |
| 16373 | 2 | 3 | | | III-2 | Pcgf6 | 84108 | 4-May-15 |
| 16375 | 2 | 3 | | | III-2 | Pcif1 | 63935 | 4-May-15 |
| 16380 | 2 | 3 | | | III-2 | Pcmt1 | 5110 | 4-May-15 |
| 16381 | 2 | 3 | | | III-2 | Pcmtd1 | 115294 | 4-May-15 |
| 16385 | 2 | 3 | | | III-2 | Pcnt | 5116 | 4-May-15 |
| 16388 | 2 | 3 | | | III-2 | Pcnxl3 | 399909 | 4-May-15 |
| 16399 | 2 | 3 | | | III-2 | Pcsk2os2 | | |
| 16413 | 2 | 3 | | | III-2 | Pdc | 5132 | 7-Jun-15 |
| 16416 | 2 | 3 | | | III-2 | Pdcd11 | 22984 | 4-May-15 |
| 16427 | 2 | 3 | | | III-2 | Pdcl3 | 79031 | 4-May-15 |
| 16439 | 2 | 3 | | | III-2 | Pde4b | 5142 | 4-May-15 |
| 16446 | 2 | 3 | | | III-2 | Pde6c | 5146 | 23-May-15 |
| 16450 | 2 | 3 | | | III-2 | Pde7a | 5150 | 4-May-15 |
| 16453 | 2 | 3 | | | III-2 | Pde8b | 8622 | 4-May-15 |
| 16456 | 2 | 3 | | | III-2 | Pdgfa | 5154 | 31-May-15 |
| 16487 | 2 | 3 | | | III-2 | Pdpn | 10630 | 17-May-15 |
| 16492 | 2 | 3 | | | III-2 | Pdss1 | 23590 | 4-May-15 |
| 16498 | 2 | 3 | | | III-2 | Pdxp | 57026 | 4-May-15 |
| 16500 | 2 | 3 | | | III-2 | Pdzd11 | 51248 | 4-May-15 |
| 16503 | 2 | 3 | | | III-2 | Pdzd4 | 57595 | 12-May-15 |
| 16504 | 2 | 3 | | | III-2 | Pdzd7 | 79955 | 23-May-15 |
| 16506 | 2 | 3 | | | III-2 | Pdzd9 | 255762 | 4-May-15 |
| 16511 | 2 | 3 | | | III-2 | Pea15a | | |
| 16513 | 2 | 3 | | | III-2 | Peak1 | 79834 | 4-May-15 |
| 16526 | 2 | 3 | | | III-2 | Peli2 | 57161 | 4-May-15 |
| 16533 | 2 | 3 | | | III-2 | Pepd | 5184 | 31-May-15 |
| 16545 | 2 | 3 | | | III-2 | Pex1 | 5189 | 13-Jun-15 |
| 16549 | 2 | 3 | | | III-2 | Pex11g | 92960 | 12-May-15 |
| 16551 | 2 | 3 | | | III-2 | Pex13 | 5194 | 28-May-15 |
| 16555 | 2 | 3 | | | III-2 | Pex2 | 5828 | 17-May-15 |
| 16561 | 2 | 3 | | | III-2 | Pex7 | 5191 | 23-May-15 |
| 16562 | 2 | 3 | | | III-2 | Pf4 | 5196 | 24-May-15 |
| 16575 | 2 | 3 | | | III-2 | Pfn1 | 5216 | 7-Jun-15 |
| 16579 | 2 | 3 | | | III-2 | Pfpl | | |
| 16580 | 2 | 3 | | | III-2 | Pga5 | 5222 | 4-May-15 |
| 16615 | 2 | 3 | | | III-2 | Phactr3 | 116154 | 4-May-15 |
| 16624 | 2 | 3 | | | III-2 | Phf1 | 5252 | 4-May-15 |
| 16630 | 2 | 3 | | | III-2 | Phf12 | 57649 | 12-May-15 |
| 16631 | 2 | 3 | | | III-2 | Phf13 | 148479 | 4-May-15 |
| 16635 | 2 | 3 | | | III-2 | Phf20 | 51230 | 12-May-15 |
| 16636 | 2 | 3 | | | III-2 | Phf20l1 | 51105 | 4-May-15 |
| 16638 | 2 | 3 | | | III-2 | Phf21b | 112885 | 12-May-15 |
| 16650 | 2 | 3 | | | III-2 | Phkb | 5257 | 23-May-15 |
| 16659 | 2 | 3 | | | III-2 | Phlpp1 | 23239 | 4-May-15 |
| 16660 | 2 | 3 | | | III-2 | Phlpp2 | 23035 | 4-May-15 |
| 16663 | 2 | 3 | | | III-2 | Phox2a | 401 | 23-May-15 |
| 16665 | 2 | 3 | | | III-2 | Phpt1 | 29085 | 4-May-15 |
| 16675 | 2 | 3 | | | III-2 | Phykpl | 85007 | 4-May-15 |
| 16679 | 2 | 3 | | | III-2 | Pi4k2b | 55300 | 4-May-15 |
| 16682 | 2 | 3 | | | III-2 | Pianp | 196500 | 4-May-15 |
| 16684 | 2 | 3 | | | III-2 | Pias2 | 9063 | 4-May-15 |

Fig.22 - 51

| | | | | | | | | |
|---|---|---|---|---|---|---|---|---|
| 16688 | 2 | 3 | | | III-2 | Picalm | 8301 | 12-May-15 |
| 16689 | 2 | 3 | | | III-2 | Pick1 | 9463 | 4-May-15 |
| 16694 | 2 | 3 | | | III-2 | Pif1 | 80119 | 24-May-15 |
| 16699 | 2 | 3 | | | III-2 | Pigf | 5281 | 12-May-15 |
| 16701 | 2 | 3 | | | III-2 | Pigh | 5283 | 12-May-15 |
| 16703 | 2 | 3 | | | III-2 | Pigl | 9487 | 4-May-15 |
| 16706 | 2 | 3 | | | III-2 | Pigo | 84720 | 4-May-15 |
| 16711 | 2 | 3 | | | III-2 | Pigt | 51604 | 12-May-15 |
| 16712 | 2 | 3 | | | III-2 | Pigu | 128869 | 21-May-15 |
| 16713 | 2 | 3 | | | III-2 | Pigv | 55650 | 12-May-15 |
| 16724 | 2 | 3 | | | III-2 | Pik3c2g | 5288 | 3-May-15 |
| 16725 | 2 | 3 | | | III-2 | Pik3c3 | 5289 | 21-May-15 |
| 16727 | 2 | 3 | | | III-2 | Pik3cb | 5291 | 31-May-15 |
| 16735 | 2 | 3 | | | III-2 | Pik3r5 | 23533 | 4-May-15 |
| 16737 | 2 | 3 | | | III-2 | Pikfyve | 200576 | 12-May-15 |
| 16738 | 2 | 3 | | | III-2 | Pilra | 29992 | 4-May-15 |
| 16745 | 2 | 3 | | | III-2 | Pin1rt1 | | |
| 16753 | 2 | 3 | | | III-2 | Pip4k2b | 8396 | 4-May-15 |
| 16758 | 2 | 3 | | | III-2 | Pip5kl1 | 138429 | 4-May-15 |
| 16764 | 2 | 3 | | | III-2 | Pira4 | | |
| 16769 | 2 | 3 | | | III-2 | Pisd | 23761 | 7-Jun-15 |
| 16770 | 2 | 3 | | | III-2 | Pisd-ps1 | | |
| 16773 | 2 | 3 | | | III-2 | Pithd1 | 57095 | 4-May-15 |
| 16780 | 2 | 3 | | | III-2 | Pitpnm3 | 83394 | 4-May-15 |
| 16792 | 2 | 3 | | | III-2 | Pkd1l3 | 342372 | 4-May-15 |
| 16798 | 2 | 3 | | | III-2 | Pkhd1 | 5314 | 23-May-15 |
| 16808 | 2 | 3 | | | III-2 | Pkn3 | 29941 | 21-May-15 |
| 16824 | 2 | 3 | | | III-2 | Pla2g2c | 391013 | 4-May-15 |
| 16828 | 2 | 3 | | | III-2 | Pla2g3 | 50487 | 4-May-15 |
| 16833 | 2 | 3 | | | III-2 | Pla2g4e | 123745 | 4-May-15 |
| 16839 | 2 | 3 | | | III-2 | Plaa | 9373 | 4-May-15 |
| 16840 | 2 | 3 | | | III-2 | Plac1 | 10761 | 24-May-15 |
| 16846 | 2 | 3 | | | III-2 | Plagl1 | 5325 | 28-May-15 |
| 16853 | 2 | 3 | | | III-2 | Plbd2 | 196463 | 12-May-15 |
| 16854 | 2 | 3 | | | III-2 | Plcb1 | 23236 | 4-May-15 |
| 16856 | 2 | 3 | | | III-2 | Plcb3 | 5331 | 4-May-15 |
| 16857 | 2 | 3 | | | III-2 | Plcb4 | 5332 | 12-May-15 |
| 16865 | 2 | 3 | | | III-2 | Plch2 | 9651 | 4-May-15 |
| 16871 | 2 | 3 | | | III-2 | Plcz1 | 89869 | 4-May-15 |
| 16872 | 2 | 3 | | | III-2 | Pld1 | 5337 | 13-Jun-15 |
| 16896 | 2 | 3 | | | III-2 | Plekhg1 | 57480 | 4-May-15 |
| 16900 | 2 | 3 | | | III-2 | Plekhg5 | 57449 | 4-May-15 |
| 16930 | 2 | 3 | | | III-2 | Plod2 | 5352 | 28-May-15 |
| 16932 | 2 | 3 | | | III-2 | Plp1 | 5354 | 23-May-15 |
| 16942 | 2 | 3 | | | III-2 | Pltp | 5360 | 4-May-15 |
| 16946 | 2 | 3 | | | III-2 | Plxna1 | 5361 | 4-May-15 |
| 16949 | 2 | 3 | | | III-2 | Plxna4 | 91584 | 4-May-15 |
| 16964 | 2 | 3 | | | III-2 | Pmis2 | | |
| 16971 | 2 | 3 | | | III-2 | Pmpcb | 9512 | 4-May-15 |
| 16973 | 2 | 3 | | | III-2 | Pms2 | 5395 | 22-May-15 |
| 16976 | 2 | 3 | | | III-2 | Pnisr | 25957 | 12-May-15 |
| 16977 | 2 | 3 | | | III-2 | Pnkd | 25953 | 23-May-15 |
| 16985 | 2 | 3 | | | III-2 | Pnma3 | 29944 | 4-May-15 |
| 17000 | 2 | 3 | | | III-2 | Pnpla7 | 375775 | 4-May-15 |
| 17002 | 2 | 3 | | | III-2 | Pnpo | 55163 | 2-Jun-15 |
| 17008 | 2 | 3 | | | III-2 | Poc5 | 134359 | 17-May-15 |
| 17014 | 2 | 3 | | | III-2 | Pofut1 | 23509 | 17-May-15 |
| 17021 | 2 | 3 | | | III-2 | Polb | 5423 | 7-Jun-15 |
| 17026 | 2 | 3 | | | III-2 | Poldip2 | 26073 | 12-May-15 |
| 17032 | 2 | 3 | | | III-2 | Polg | 5428 | 23-May-15 |
| 17033 | 2 | 3 | | | III-2 | Polg2 | 11232 | 12-May-15 |
| 17036 | 2 | 3 | | | III-2 | Polk | 51426 | 7-Jun-15 |
| 17038 | 2 | 3 | | | III-2 | Polm | 27434 | 4-May-15 |
| 17040 | 2 | 3 | | | III-2 | Polq | 10721 | 7-Jun-15 |
| 17042 | 2 | 3 | | | III-2 | Polr1b | 84172 | 4-May-15 |
| 17047 | 2 | 3 | | | III-2 | Polr2b | 5431 | 12-May-15 |
| 17051 | 2 | 3 | | | III-2 | Polr2f | 5435 | 12-May-15 |
| 17058 | 2 | 3 | | | III-2 | Polr2m | 81488 | 4-May-15 |
| 17059 | 2 | 3 | | | III-2 | Polr3a | 11128 | 23-May-15 |
| 17067 | 2 | 3 | | | III-2 | Polr3h | 171568 | 4-May-15 |
| 17068 | 2 | 3 | | | III-2 | Polr3k | 51728 | 28-May-15 |
| 17075 | 2 | 3 | | | III-2 | Pomgnt2 | 84892 | 4-May-15 |
| 17082 | 2 | 3 | | | III-2 | Pon3 | 5446 | 4-May-15 |
| 17093 | 2 | 3 | | | III-2 | Pot1b | | |
| 17094 | 2 | 3 | | | III-2 | Poteg | 404785 | 4-May-15 |
| 17112 | 2 | 3 | | | III-2 | Pp2d1 | 151649 | 4-May-15 |
| 17131 | 2 | 3 | | | III-2 | Ppcs | 79717 | 4-May-15 |
| 17134 | 2 | 3 | | | III-2 | Ppef2 | 5470 | 4-May-15 |
| 17146 | 2 | 3 | | | III-2 | Ppie | 10450 | 3-Jun-15 |
| 17150 | 2 | 3 | | | III-2 | Ppih | 10465 | 12-May-15 |
| 17151 | 2 | 3 | | | III-2 | Ppil1 | 51645 | 4-May-15 |
| 17153 | 2 | 3 | | | III-2 | Ppil3 | 53938 | 4-May-15 |
| 17160 | 2 | 3 | | | III-2 | Ppm1b | 5495 | 31-May-15 |
| 17164 | 2 | 3 | | | III-2 | Ppm1g | 5496 | 4-May-15 |
| 17170 | 2 | 3 | | | III-2 | Ppm1n | 147699 | 4-May-15 |
| 17172 | 2 | 3 | | | III-2 | Ppox | 5498 | 13-Jun-15 |
| 17173 | 2 | 3 | | | III-2 | Ppp1ca | 5499 | 12-May-15 |
| 17176 | 2 | 3 | | | III-2 | Ppp1r10 | 5514 | 4-May-15 |
| 17178 | 2 | 3 | | | III-2 | Ppp1r12a | 4659 | 12-May-15 |
| 17196 | 2 | 3 | | | III-2 | Ppp1r2 | 5504 | 12-May-15 |
| 17202 | 2 | 3 | | | III-2 | Ppp1r2-ps9 | | |
| 17204 | 2 | 3 | | | III-2 | Ppp1r35 | 221908 | 4-May-15 |
| 17220 | 2 | 3 | | | III-2 | Ppp2ca | 5515 | 17-May-15 |
| 17235 | 2 | 3 | | | III-2 | Ppp2r5c | 5527 | 4-May-15 |
| 17236 | 2 | 3 | | | III-2 | Ppp2r5d | 5528 | 23-May-15 |
| 17241 | 2 | 3 | | | III-2 | Ppp3r1 | 5534 | 4-May-15 |
| 17253 | 2 | 3 | | | III-2 | Pprc1 | 23082 | 4-May-15 |
| 17255 | 2 | 3 | | | III-2 | Ppt2 | 9374 | 4-May-15 |
| 17257 | 2 | 3 | | | III-2 | Ppwd1 | 23398 | 4-May-15 |
| 17269 | 2 | 3 | | | III-2 | Pramef25 | 441873 | 4-May-15 |
| 17282 | 2 | 3 | | | III-2 | Prcp | 5547 | 4-May-15 |
| 17286 | 2 | 3 | | | III-2 | Prdm12 | 59335 | 28-May-15 |
| 17292 | 2 | 3 | | | III-2 | Prdm4 | 11108 | 4-May-15 |
| 17295 | 2 | 3 | | | III-2 | Prdm8 | 56978 | 4-May-15 |
| 17296 | 2 | 3 | | | III-2 | Prdm9 | 56979 | 12-May-15 |
| 17302 | 2 | 3 | | | III-2 | Prdx6 | 9588 | 7-Jun-15 |
| 17319 | 2 | 3 | | | III-2 | Prickle3 | 4007 | 12-May-15 |
| 17320 | 2 | 3 | | | III-2 | Prickle4 | 29964 | 4-May-15 |
| 17330 | 2 | 3 | | | III-2 | Prkacb | 5567 | 12-May-15 |
| 17336 | 2 | 3 | | | III-2 | Prkar1b | 5575 | 4-May-15 |
| 17344 | 2 | 3 | | | III-2 | Prkcg | 5582 | 23-May-15 |
| 17349 | 2 | 3 | | | III-2 | Prkcz | 5590 | 4-May-15 |
| 17351 | 2 | 3 | | | III-2 | Prkd2 | 25865 | 4-May-15 |
| 17352 | 2 | 3 | | | III-2 | Prkd3 | 23683 | 17-May-15 |
| 17353 | 2 | 3 | | | III-2 | Prkdc | 5591 | 12-May-15 |
| 17354 | 2 | 3 | | | III-2 | Prkg1 | 5592 | 28-May-15 |
| 17356 | 2 | 3 | | | III-2 | Prkra | 8575 | 12-May-15 |
| 17357 | 2 | 3 | | | III-2 | Prkrip1 | 79706 | 4-May-15 |
| 17358 | 2 | 3 | | | III-2 | Prkrir | 5612 | 4-May-15 |
| 17362 | 2 | 3 | | | III-2 | Prl2b1 | | |
| 17397 | 2 | 3 | | | III-2 | Prmt5 | 10419 | 4-May-15 |
| 17398 | 2 | 3 | | | III-2 | Prmt6 | 55170 | 4-May-15 |
| 17412 | 2 | 3 | | | III-2 | Prokr1 | 10887 | 4-May-15 |
| 17421 | 2 | 3 | | | III-2 | Proser1 | 80209 | 4-May-15 |
| 17439 | 2 | 3 | | | III-2 | Prpf8 | 10594 | 23-May-15 |
| 17445 | 2 | 3 | | | III-2 | Prps1l3 | | |
| 17448 | 2 | 3 | | | III-2 | Prpsap2 | 5636 | 4-May-15 |
| 17451 | 2 | 3 | | | III-2 | Prr13 | 54458 | 4-May-15 |
| 17453 | 2 | 3 | | | III-2 | Prr14l | 253143 | 4-May-15 |
| 17458 | 2 | 3 | | | III-2 | Prr19 | 284338 | 20-May-15 |
| 17461 | 2 | 3 | | | III-2 | Prr24 | 255783 | 4-May-15 |
| 17472 | 2 | 3 | | | III-2 | Prrc2a | 7916 | 12-May-15 |
| 17474 | 2 | 3 | | | III-2 | Prrc2c | 23215 | 4-May-15 |
| 17482 | 2 | 3 | | | III-2 | Prrt4 | 401399 | 4-May-15 |
| 17484 | 2 | 3 | | | III-2 | Prrx2 | 51450 | 4-May-15 |
| 17491 | 2 | 3 | | | III-2 | Prss22 | 64063 | 12-May-15 |
| 17495 | 2 | 3 | | | III-2 | Prss29 | | |
| 17498 | 2 | 3 | | | III-2 | Prss32 | | |
| 17500 | 2 | 3 | | | III-2 | Prss34 | | |
| 17504 | 2 | 3 | | | III-2 | Prss38 | 339501 | 4-May-15 |
| 17519 | 2 | 3 | | | III-2 | Prss55 | 203074 | 4-May-15 |
| 17535 | 2 | 3 | | | III-2 | Psd3 | 23362 | 4-May-15 |
| 17537 | 2 | 3 | | | III-2 | Psen1 | 5663 | 31-May-15 |
| 17538 | 2 | 3 | | | III-2 | Psen2 | 5664 | 31-May-15 |
| 17556 | 2 | 3 | | | III-2 | Psma1 | 5682 | 4-May-15 |
| 17567 | 2 | 3 | | | III-2 | Psmb2 | 5690 | 4-May-15 |
| 17574 | 2 | 3 | | | III-2 | Psmb9 | 5698 | 12-May-15 |
| 17576 | 2 | 3 | | | III-2 | Psmc2 | 5701 | 4-May-15 |
| 17580 | 2 | 3 | | | III-2 | Psmc5 | 5705 | 4-May-15 |
| 17581 | 2 | 3 | | | III-2 | Psmc6 | 5706 | 4-May-15 |
| 17589 | 2 | 3 | | | III-2 | Psmd3 | 5709 | 3-May-15 |
| 17599 | 2 | 3 | | | III-2 | Psme3 | 10197 | 24-May-15 |
| 17611 | 2 | 3 | | | III-2 | Pstk | 118672 | 12-May-15 |
| 17617 | 2 | 3 | | | III-2 | Ptbp2 | 58155 | 2-Jun-15 |
| 17625 | 2 | 3 | | | III-2 | Ptchd2 | 57540 | 12-May-15 |
| 17634 | 2 | 3 | | | III-2 | Ptgdr | 5729 | 3-May-15 |
| 17638 | 2 | 3 | | | III-2 | Ptger2 | 5732 | 12-May-15 |
| 17643 | 2 | 3 | | | III-2 | Ptges3 | 10728 | 4-May-15 |
| 17651 | 2 | 3 | | | III-2 | Ptgs1 | 5742 | 12-May-15 |
| 17657 | 2 | 3 | | | III-2 | Pth2r | 5746 | 4-May-15 |
| 17660 | 2 | 3 | | | III-2 | Ptk2b | 2185 | 4-May-15 |
| 17665 | 2 | 3 | | | III-2 | Ptn | 5764 | 17-May-15 |
| 17667 | 2 | 3 | | | III-2 | Ptp4a1 | 7803 | 17-May-15 |
| 17673 | 2 | 3 | | | III-2 | Ptplad2 | 401494 | 1-Jun-15 |
| 17694 | 2 | 3 | | | III-2 | Ptprb | 5787 | 12-May-15 |
| 17701 | 2 | 3 | | | III-2 | Ptprh | 5794 | 12-May-15 |
| 17708 | 2 | 3 | | | III-2 | Ptprq | 374462 | 4-May-15 |
| 17709 | 2 | 3 | | | III-2 | Ptprr | 5801 | 17-May-15 |
| 17711 | 2 | 3 | | | III-2 | Ptprt | 11122 | 4-May-15 |
| 17712 | 2 | 3 | | | III-2 | Ptprtos | | |
| 17719 | 2 | 3 | | | III-2 | Ptrhd1 | 391356 | 4-May-15 |
| 17723 | 2 | 3 | | | III-2 | Ptx3 | 5806 | 24-May-15 |
| 17726 | 2 | 3 | | | III-2 | Pum1 | 9698 | 29-May-15 |
| 17730 | 2 | 3 | | | III-2 | Purg | 29942 | 4-May-15 |
| 17734 | 2 | 3 | | | III-2 | Pus7 | 54517 | 12-May-15 |
| 17745 | 2 | 3 | | | III-2 | Pwp2 | 5822 | 4-May-15 |
| 17759 | 2 | 3 | | | III-2 | Pycrl | 65263 | 4-May-15 |
| 17761 | 2 | 3 | | | III-2 | Pydc4 | | |
| 17762 | 2 | 3 | | | III-2 | Pygb | 5834 | 4-May-15 |
| 17767 | 2 | 3 | | | III-2 | Pyhin1 | 149628 | 4-May-15 |
| 17771 | 2 | 3 | | | III-2 | Pyy | 5697 | 23-May-15 |
| 17774 | 2 | 3 | | | III-2 | Qdpr | 5860 | 12-May-15 |
| 17778 | 2 | 3 | | | III-2 | Qprt | 23475 | 21-May-15 |
| 17782 | 2 | 3 | | | III-2 | Qrich2 | 84074 | 4-May-15 |
| 17789 | 2 | 3 | | | III-2 | R3hcc1 | 203069 | 12-May-15 |
| 17797 | 2 | 3 | | | III-2 | Rab10 | 10890 | 4-May-15 |
| 17803 | 2 | 3 | | | III-2 | Rab11fip3 | 9727 | 21-May-15 |
| 17806 | 2 | 3 | | | III-2 | Rab11fip4os2 | | |
| 17809 | 2 | 3 | | | III-2 | Rab13 | 5872 | 3-May-15 |
| 17811 | 2 | 3 | | | III-2 | Rab15 | 376267 | 12-May-15 |
| 17819 | 2 | 3 | | | III-2 | Rab23 | 51715 | 21-May-15 |
| 17827 | 2 | 3 | | | III-2 | Rab2a | 5862 | 21-May-15 |
| 17831 | 2 | 3 | | | III-2 | Rab32 | 10981 | 4-May-15 |
| 17832 | 2 | 3 | | | III-2 | Rab33a | 9363 | 21-May-15 |

Fig.22 - 52

| | | | | | | | | |
|---|---|---|---|---|---|---|---|---|
| 17833 | 2 | 3 | | | III-2 | Rab33b | 83452 | 21-May-15 |
| 17853 | 2 | 3 | | | III-2 | Rab44 | 401258 | 4-May-15 |
| 17864 | 2 | 3 | | | III-2 | Rab8b | 51762 | 4-May-15 |
| 17879 | 2 | 3 | | | III-2 | Rabl6 | 55684 | 4-May-15 |
| 17885 | 2 | 3 | | | III-2 | Rad17 | 5884 | 12-May-15 |
| 17888 | 2 | 3 | | | III-2 | Rad21l | 642636 | 4-May-15 |
| 17889 | 2 | 3 | | | III-2 | Rad23a | 5886 | 4-May-15 |
| 17891 | 2 | 3 | | | III-2 | Rad50 | 10111 | 4-May-15 |
| 17898 | 2 | 3 | | | III-2 | Rad52 | 5893 | 12-May-15 |
| 17906 | 2 | 3 | | | III-2 | Raet1a | | |
| 17914 | 2 | 3 | | | III-2 | Rai1 | 10743 | 16-Jun-15 |
| 17918 | 2 | 3 | | | III-2 | Ralb | 5899 | 21-May-15 |
| 17919 | 2 | 3 | | | III-2 | Ralbp1 | 10928 | 7-Jun-15 |
| 17927 | 2 | 3 | | | III-2 | Ralyl | 138046 | 2-Jun-15 |
| 17932 | 2 | 3 | | | III-2 | Ranbp1 | 5902 | 1-Jun-15 |
| 17935 | 2 | 3 | | | III-2 | Ranbp2 | 5903 | 23-May-15 |
| 17937 | 2 | 3 | | | III-2 | Ranbp3l | 202151 | 28-May-15 |
| 17939 | 2 | 3 | | | III-2 | Ranbp9 | 10048 | 4-May-15 |
| 17943 | 2 | 3 | | | III-2 | Rap1b | 5908 | 21-May-15 |
| 17947 | 2 | 3 | | | III-2 | Rap2a | 5911 | 12-May-15 |
| 17948 | 2 | 3 | | | III-2 | Rap2b | 5912 | 4-May-15 |
| 17956 | 2 | 3 | | | III-2 | Rapgefl1 | 51195 | 12-May-15 |
| 17960 | 2 | 3 | | | III-2 | Rarb | 5915 | 12-May-15 |
| 17967 | 2 | 3 | | | III-2 | Rasa2 | 5922 | 4-May-15 |
| 17983 | 2 | 3 | | | III-2 | Rasgrp3 | 25780 | 12-May-15 |
| 17985 | 2 | 3 | | | III-2 | Rasip1 | 54922 | 4-May-15 |
| 18003 | 2 | 3 | | | III-2 | Raver1-fdx1l | | |
| 18008 | 2 | 3 | | | III-2 | Rbak | 57786 | 12-May-15 |
| 18009 | 2 | 3 | | | III-2 | Rbakdn | 389458 | 4-May-15 |
| 18012 | 2 | 3 | | | III-2 | Rbbp6 | 5930 | 12-May-15 |
| 18016 | 2 | 3 | | | III-2 | Rbbp9 | 10741 | 4-May-15 |
| 18017 | 2 | 3 | | | III-2 | Rbck1 | 10616 | 23-May-15 |
| 18024 | 2 | 3 | | | III-2 | Rbl2 | 5934 | 4-May-15 |
| 18030 | 2 | 3 | | | III-2 | Rbm14 | 10432 | 1-Jun-15 |
| 18035 | 2 | 3 | | | III-2 | Rbm18 | 92400 | 4-May-15 |
| 18036 | 2 | 3 | | | III-2 | Rbm19 | 9904 | 4-May-15 |
| 18041 | 2 | 3 | | | III-2 | Rbm26 | 64062 | 4-May-15 |
| 18046 | 2 | 3 | | | III-2 | Rbm33 | 155435 | 4-May-15 |
| 18054 | 2 | 3 | | | III-2 | Rbm43 | 375287 | 4-May-15 |
| 18061 | 2 | 3 | | | III-2 | Rbm4b | 83759 | 4-May-15 |
| 18070 | 2 | 3 | | | III-2 | Rbmx2 | 51634 | 4-May-15 |
| 18080 | 2 | 3 | | | III-2 | Rbpjl | 11317 | 4-May-15 |
| 18084 | 2 | 3 | | | III-2 | Rc3h1 | 149041 | 4-May-15 |
| 18089 | 2 | 3 | | | III-2 | Rcbtb1 | 55213 | 4-May-15 |
| 18093 | 2 | 3 | | | III-2 | Rccd1 | 91433 | 4-May-15 |
| 18099 | 2 | 3 | | | III-2 | Rcn3 | 57333 | 7-Jun-15 |
| 18102 | 2 | 3 | | | III-2 | Rcor3 | 55758 | 14-May-15 |
| 18124 | 2 | 3 | | | III-2 | Recql | 5965 | 4-May-15 |
| 18125 | 2 | 3 | | | III-2 | Recql4 | 9401 | 23-May-15 |
| 18131 | 2 | 3 | | | III-2 | Reep4 | 80346 | 4-May-15 |
| 18143 | 2 | 3 | | | III-2 | Relb | 5971 | 28-May-15 |
| 18150 | 2 | 3 | | | III-2 | Ren1 | | |
| 18155 | 2 | 3 | | | III-2 | Reps1 | 85021 | 4-May-15 |
| 18158 | 2 | 3 | | | III-2 | Rere | 473 | 12-May-15 |
| 18172 | 2 | 3 | | | III-2 | Rexo1 | 57455 | 4-May-15 |
| 18180 | 2 | 3 | | | III-2 | Rfesd | 317671 | 4-May-15 |
| 18181 | 2 | 3 | | | III-2 | Rffl | 117584 | 4-May-15 |
| 18182 | 2 | 3 | | | III-2 | Rfk | 55312 | 4-May-15 |
| 18195 | 2 | 3 | | | III-2 | Rfx4 | 5992 | 28-May-15 |
| 18196 | 2 | 3 | | | III-2 | Rfx5 | 5993 | 4-May-15 |
| 18200 | 2 | 3 | | | III-2 | Rfxank | 8625 | 4-May-15 |
| 18206 | 2 | 3 | | | III-2 | Rgl2 | 5863 | 4-May-15 |
| 18207 | 2 | 3 | | | III-2 | Rgl3 | 57139 | 4-May-15 |
| 18212 | 2 | 3 | | | III-2 | Rgr | 5995 | 7-Jun-15 |
| 18221 | 2 | 3 | | | III-2 | Rgs18 | 64407 | 4-May-15 |
| 18231 | 2 | 3 | | | III-2 | Rgs7 | 6000 | 4-May-15 |
| 18254 | 2 | 3 | | | III-2 | Rhob | 388 | 12-May-15 |
| 18262 | 2 | 3 | | | III-2 | Rhoh | 399 | 12-May-15 |
| 18266 | 2 | 3 | | | III-2 | Rhot2 | 89941 | 4-May-15 |
| 18270 | 2 | 3 | | | III-2 | Rhox10 | | |
| 18287 | 2 | 3 | | | III-2 | Rhox3h | | |
| 18297 | 2 | 3 | | | III-2 | Rhox7 | | |
| 18305 | 2 | 3 | | | III-2 | Ric3 | 79608 | 14-May-15 |
| 18307 | 2 | 3 | | | III-2 | Ric8b | 55188 | 4-May-15 |
| 18316 | 2 | 3 | | | III-2 | Rimkla | 284716 | 4-May-15 |
| 18319 | 2 | 3 | | | III-2 | Rims2 | 9699 | 12-May-15 |
| 18325 | 2 | 3 | | | III-2 | Ring1 | 6015 | 4-May-15 |
| 18326 | 2 | 3 | | | III-2 | Rinl | 126432 | 4-May-15 |
| 18328 | 2 | 3 | | | III-2 | Riok1 | 83732 | 4-May-15 |
| 18339 | 2 | 3 | | | III-2 | Rit2 | 6014 | 4-May-15 |
| 18344 | 2 | 3 | | | III-2 | Rln1 | 6013 | 4-May-15 |
| 18345 | 2 | 3 | | | III-2 | Rln3 | 117579 | 4-May-15 |
| 18348 | 2 | 3 | | | III-2 | Rmdn2 | 151393 | 4-May-15 |
| 18350 | 2 | 3 | | | III-2 | Rmi1 | 80010 | 4-May-15 |
| 18351 | 2 | 3 | | | III-2 | Rmi2 | 116028 | 4-May-15 |
| 18354 | 2 | 3 | | | III-2 | Rmnd5b | 64777 | 4-May-15 |
| 18381 | 2 | 3 | | | III-2 | Rnf103 | 7844 | 4-May-15 |
| 18384 | 2 | 3 | | | III-2 | Rnf112 | 7732 | 4-May-15 |
| 18386 | 2 | 3 | | | III-2 | Rnf113a2 | | |
| 18390 | 2 | 3 | | | III-2 | Rnf122 | 79845 | 4-May-15 |
| 18396 | 2 | 3 | | | III-2 | Rnf130 | 55819 | 2-Jun-15 |
| 18402 | 2 | 3 | | | III-2 | Rnf14 | 9604 | 4-May-15 |
| 18418 | 2 | 3 | | | III-2 | Rnf169 | 254225 | 4-May-15 |
| 18419 | 2 | 3 | | | III-2 | Rnf17 | 56163 | 14-May-15 |
| 18429 | 2 | 3 | | | III-2 | Rnf19b | 127544 | 23-May-15 |
| 18436 | 2 | 3 | | | III-2 | Rnf216 | 54476 | 4-May-15 |
| 18439 | 2 | 3 | | | III-2 | Rnf220 | 55182 | 4-May-15 |
| 18442 | 2 | 3 | | | III-2 | Rnf224 | 643596 | 4-May-15 |
| 18447 | 2 | 3 | | | III-2 | Rnf32 | 140545 | 21-May-15 |
| 18448 | 2 | 3 | | | III-2 | Rnf34 | 80196 | 12-May-15 |
| 18452 | 2 | 3 | | | III-2 | Rnf40 | 9810 | 4-May-15 |
| 18466 | 2 | 3 | | | III-2 | Rnmtl1 | 55178 | 4-May-15 |
| 18471 | 2 | 3 | | | III-2 | Rnu11 | 26824 | 7-Jun-15 |
| 18474 | 2 | 3 | | | III-2 | Rnu7 | | |
| 18478 | 2 | 3 | | | III-2 | Robo3 | 64221 | 4-May-15 |
| 18481 | 2 | 3 | | | III-2 | Rock2 | 9475 | 31-May-15 |
| 18482 | 2 | 3 | | | III-2 | Rogdi | 79641 | 12-May-15 |
| 18483 | 2 | 3 | | | III-2 | Rom1 | 6094 | 23-May-15 |
| 18501 | 2 | 3 | | | III-2 | Rpap1 | 26015 | 4-May-15 |
| 18502 | 2 | 3 | | | III-2 | Rpap2 | 79871 | 4-May-15 |
| 18504 | 2 | 3 | | | III-2 | Rpe | 6120 | 4-May-15 |
| 18505 | 2 | 3 | | | III-2 | Rpe65 | 6121 | 23-May-15 |
| 18506 | 2 | 3 | | | III-2 | Rpf1 | 80135 | 7-Jun-15 |
| 18510 | 2 | 3 | | | III-2 | Rpgrip1l | 23322 | 23-May-15 |
| 18539 | 2 | 3 | | | III-2 | Rpl3 | 6122 | 31-May-15 |
| 18542 | 2 | 3 | | | III-2 | Rpl31-ps12 | | |
| 18578 | 2 | 3 | | | III-2 | Rpp40 | 10799 | 12-May-15 |
| 18604 | 2 | 3 | | | III-2 | Rps20 | 6224 | 4-May-15 |
| 18617 | 2 | 3 | | | III-2 | Rps3a1 | | |
| 18625 | 2 | 3 | | | III-2 | Rps6ka4 | 8986 | 4-May-15 |
| 18626 | 2 | 3 | | | III-2 | Rps6ka5 | 9252 | 4-May-15 |
| 18631 | 2 | 3 | | | III-2 | Rps6kl1 | 83694 | 12-May-15 |
| 18643 | 2 | 3 | | | III-2 | Rqcd1 | 9125 | 12-May-15 |
| 18651 | 2 | 3 | | | III-2 | Rrbp1 | 6238 | 4-May-15 |
| 18657 | 2 | 3 | | | III-2 | Rrn3 | 54700 | 4-May-15 |
| 18662 | 2 | 3 | | | III-2 | Rrp1b | 23076 | 4-May-15 |
| 18663 | 2 | 3 | | | III-2 | Rrp36 | 88745 | 4-May-15 |
| 18665 | 2 | 3 | | | III-2 | Rrp8 | 23378 | 28-May-15 |
| 18673 | 2 | 3 | | | III-2 | Rsc1a1 | 6248 | 4-May-15 |
| 18675 | 2 | 3 | | | III-2 | Rsg1 | 79363 | 4-May-15 |
| 18678 | 2 | 3 | | | III-2 | Rsl24d1 | 51187 | 4-May-15 |
| 18682 | 2 | 3 | | | III-2 | Rsph3b | | |
| 18683 | 2 | 3 | | | III-2 | Rsph4a | 345895 | 23-May-15 |
| 18684 | 2 | 3 | | | III-2 | Rsph6a | 81492 | 4-May-15 |
| 18691 | 2 | 3 | | | III-2 | Rsrc1 | 51319 | 4-May-15 |
| 18705 | 2 | 3 | | | III-2 | Rtn1 | 6252 | 12-May-15 |
| 18708 | 2 | 3 | | | III-2 | Rtn4 | 57142 | 10-May-15 |
| 18714 | 2 | 3 | | | III-2 | Rtp2 | 344892 | 9-May-15 |
| 18715 | 2 | 3 | | | III-2 | Rtp3 | 83597 | 9-May-15 |
| 18718 | 2 | 3 | | | III-2 | Rubie | | |
| 18719 | 2 | 3 | | | III-2 | Rufy1 | 80230 | 4-May-15 |
| 18739 | 2 | 3 | | | III-2 | Rxfp1 | 59350 | 4-May-15 |
| 18747 | 2 | 3 | | | III-2 | Ryk | 6259 | 21-May-15 |
| 18750 | 2 | 3 | | | III-2 | Ryr3 | 6263 | 12-May-15 |
| 18757 | 2 | 3 | | | III-2 | S100a2 | 6273 | 12-May-15 |
| 18761 | 2 | 3 | | | III-2 | S100a6 | 6277 | 4-May-15 |
| 18773 | 2 | 3 | | | III-2 | S1pr5 | 53637 | 4-May-15 |
| 18779 | 2 | 3 | | | III-2 | Sac3d1 | 29901 | 4-May-15 |
| 18781 | 2 | 3 | | | III-2 | Sacs | 26278 | 23-May-15 |
| 18788 | 2 | 3 | | | III-2 | Sall3 | 27164 | 4-May-15 |
| 18790 | 2 | 3 | | | III-2 | Samd1 | 90378 | 4-May-15 |
| 18796 | 2 | 3 | | | III-2 | Samd3 | 154075 | 7-Jun-15 |
| 18801 | 2 | 3 | | | III-2 | Samd8 | 142891 | 4-May-15 |
| 18804 | 2 | 3 | | | III-2 | Samm50 | 25813 | 4-May-15 |
| 18807 | 2 | 3 | | | III-2 | Samt3 | | |
| 18814 | 2 | 3 | | | III-2 | Sap30l | 79685 | 4-May-15 |
| 18825 | 2 | 3 | | | III-2 | Sart3 | 9733 | 12-May-15 |
| 18833 | 2 | 3 | | | III-2 | Satl1 | 340562 | 4-May-15 |
| 18834 | 2 | 3 | | | III-2 | Sav1 | 60485 | 4-May-15 |
| 18848 | 2 | 3 | | | III-2 | Sc5d | 6309 | 12-May-15 |
| 18849 | 2 | 3 | | | III-2 | Scaf1 | 58506 | 4-May-15 |
| 18850 | 2 | 3 | | | III-2 | Scaf11 | 9169 | 12-May-15 |
| 18852 | 2 | 3 | | | III-2 | Scaf8 | 22828 | 4-May-15 |
| 18860 | 2 | 3 | | | III-2 | Scap | 22937 | 7-Jun-15 |
| 18861 | 2 | 3 | | | III-2 | Scaper | 49855 | 4-May-15 |
| 18862 | 2 | 3 | | | III-2 | Scara3 | 51435 | 3-May-15 |
| 18869 | 2 | 3 | | | III-2 | Scarna10 | 692148 | 4-May-15 |
| 18872 | 2 | 3 | | | III-2 | Scarna2 | 677766 | 12-May-15 |
| 18878 | 2 | 3 | | | III-2 | Sccpdh | 51097 | 4-May-15 |
| 18886 | 2 | 3 | | | III-2 | Scg2 | 7857 | 7-Jun-15 |
| 18891 | 2 | 3 | | | III-2 | Scgb1b2 | | |
| 18896 | 2 | 3 | | | III-2 | Scgb1b3 | | |
| 18898 | 2 | 3 | | | III-2 | Scgb1b7 | | |
| 18904 | 2 | 3 | | | III-2 | Scgb2b2 | 284402 | 4-May-15 |
| 18905 | 2 | 3 | | | III-2 | Scgb2b20 | | |
| 18911 | 2 | 3 | | | III-2 | Scgb2b7 | | |
| 18924 | 2 | 3 | | | III-2 | Scn11a | 11280 | 12-May-15 |
| 18930 | 2 | 3 | | | III-2 | Scn3b | 55800 | 23-May-15 |
| 18942 | 2 | 3 | | | III-2 | Sco2 | 9997 | 4-May-15 |
| 18944 | 2 | 3 | | | III-2 | Scp2 | 6342 | 7-Jun-15 |
| 18946 | 2 | 3 | | | III-2 | Scpep1 | 59342 | 4-May-15 |
| 18959 | 2 | 3 | | | III-2 | Scube3 | 222663 | 4-May-15 |
| 18962 | 2 | 3 | | | III-2 | Scyl2 | 55681 | 7-Jun-15 |
| 18974 | 2 | 3 | | | III-2 | Sdf2 | 6388 | 4-May-15 |
| 18977 | 2 | 3 | | | III-2 | Sdha | 6389 | 23-May-15 |
| 18982 | 2 | 3 | | | III-2 | Sdhd | 6392 | 23-May-15 |
| 18994 | 2 | 3 | | | III-2 | Sec1 | 653677 | 4-May-15 |
| 19009 | 2 | 3 | | | III-2 | Sec23b | 10483 | 4-May-15 |
| 19011 | 2 | 3 | | | III-2 | Sec24a | 10802 | 4-May-15 |
| 19016 | 2 | 3 | | | III-2 | Sec31b | 25956 | 4-May-15 |
| 19022 | 2 | 3 | | | III-2 | Sec63 | 11231 | 4-May-15 |
| 19028 | 2 | 3 | | | III-2 | Sel1l | 6400 | 12-May-15 |
| 19038 | 2 | 3 | | | III-2 | Selp | 6403 | 7-Jun-15 |
| 19041 | 2 | 3 | | | III-2 | Sema3a | 10371 | 17-May-15 |

Fig.22 - 53

| | | | | | | | | |
|---|---|---|---|---|---|---|---|---|
| 19045 | 2 | 3 | | | III-2 | Sema3e | 9723 | 23-May-15 |
| 19055 | 2 | 3 | | | III-2 | Sema5b | 54437 | 4-May-15 |
| 19056 | 2 | 3 | | | III-2 | Sema6a | 57556 | 12-May-15 |
| 19062 | 2 | 3 | | | III-2 | Senp2 | 59343 | 7-Jun-15 |
| 19068 | 2 | 3 | | | III-2 | Sephs1 | 22929 | 2-Jun-15 |
| 19076 | 2 | 3 | | | III-2 | Sept12 | 124404 | 12-May-15 |
| 19082 | 2 | 3 | | | III-2 | Sept5 | 5413 | 4-May-15 |
| 19085 | 2 | 3 | | | III-2 | Sept8 | 23176 | 4-May-15 |
| 19087 | 2 | 3 | | | III-2 | Sepw1 | 6415 | 12-May-15 |
| 19092 | 2 | 3 | | | III-2 | Sergef | 26297 | 4-May-15 |
| 19103 | 2 | 3 | | | III-2 | Serpina12 | 145264 | 12-May-15 |
| 19123 | 2 | 3 | | | III-2 | Serpina6 | 866 | 31-May-15 |
| 19132 | 2 | 3 | | | III-2 | Serpinb1c | | |
| 19142 | 2 | 3 | | | III-2 | Serpinb6d | | |
| 19148 | 2 | 3 | | | III-2 | Serpinb9c | | |
| 19165 | 2 | 3 | | | III-2 | Sertad2 | 9792 | 21-May-15 |
| 19173 | 2 | 3 | | | III-2 | Set | 6418 | 3-May-15 |
| 19181 | 2 | 3 | | | III-2 | Setd6 | 79918 | 4-May-15 |
| 19185 | 2 | 3 | | | III-2 | Setdb2 | 83852 | 4-May-15 |
| 19188 | 2 | 3 | | | III-2 | Sez6 | 124925 | 4-May-15 |
| 19192 | 2 | 3 | | | III-2 | Sf3a1 | 10291 | 4-May-15 |
| 19212 | 2 | 3 | | | III-2 | Sft2d1 | 113402 | 4-May-15 |
| 19217 | 2 | 3 | | | III-2 | Sftpb | 6439 | 12-May-15 |
| 19220 | 2 | 3 | | | III-2 | Sfxn1 | 94081 | 4-May-15 |
| 19224 | 2 | 3 | | | III-2 | Sfxn5 | 94097 | 4-May-15 |
| 19231 | 2 | 3 | | | III-2 | Sgip1 | 84251 | 12-May-15 |
| 19267 | 2 | 3 | | | III-2 | Sh3bp5 | 9467 | 4-May-15 |
| 19269 | 2 | 3 | | | III-2 | Sh3d19 | 152503 | 4-May-15 |
| 19297 | 2 | 3 | | | III-2 | Shd | 56961 | 4-May-15 |
| 19306 | 2 | 3 | | | III-2 | Shisa6 | 388336 | 14-May-15 |
| 19309 | 2 | 3 | | | III-2 | Shkbp1 | 92799 | 4-May-15 |
| 19310 | 2 | 3 | | | III-2 | Shmt1 | 6470 | 12-May-15 |
| 19322 | 2 | 3 | | | III-2 | Siah1a | 6477 | 24-May-15 |
| 19339 | 2 | 3 | | | III-2 | Sike1 | 80143 | 4-May-15 |
| 19348 | 2 | 3 | | | III-2 | Sipa1l2 | 57568 | 4-May-15 |
| 19349 | 2 | 3 | | | III-2 | Sipa1l3 | 23094 | 4-May-15 |
| 19352 | 2 | 3 | | | III-2 | Sirpb1b | | |
| 19354 | 2 | 3 | | | III-2 | Sirt2 | 22933 | 17-May-15 |
| 19355 | 2 | 3 | | | III-2 | Sirt3 | 23410 | 31-May-15 |
| 19359 | 2 | 3 | | | III-2 | Sirt7 | 51547 | 23-May-15 |
| 19360 | 2 | 3 | | | III-2 | Sis | 5155 | 7-Jun-15 |
| 19366 | 2 | 3 | | | III-2 | Six3os1 | | |
| 19370 | 2 | 3 | | | III-2 | Ska1 | 220134 | 4-May-15 |
| 19372 | 2 | 3 | | | III-2 | Ska3 | 221150 | 4-May-15 |
| 19375 | 2 | 3 | | | III-2 | Ski | 6497 | 23-May-15 |
| 19376 | 2 | 3 | | | III-2 | Skida1 | 387640 | 4-May-15 |
| 19378 | 2 | 3 | | | III-2 | Skint1 | 391037 | 4-May-15 |
| 19384 | 2 | 3 | | | III-2 | Skint5 | | |
| 19392 | 2 | 3 | | | III-2 | Skor2 | 652991 | 21-May-15 |
| 19397 | 2 | 3 | | | III-2 | Slain1 | 122060 | 4-May-15 |
| 19398 | 2 | 3 | | | III-2 | Slain1os | | |
| 19399 | 2 | 3 | | | III-2 | Slain2 | 57606 | 4-May-15 |
| 19401 | 2 | 3 | | | III-2 | Slamf6 | 114836 | 4-May-15 |
| 19409 | 2 | 3 | | | III-2 | Slc10a3-ubl4 | | |
| 19421 | 2 | 3 | | | III-2 | Slc12a6 | 9990 | 23-May-15 |
| 19422 | 2 | 3 | | | III-2 | Slc12a7 | 10723 | 4-May-15 |
| 19423 | 2 | 3 | | | III-2 | Slc12a8 | 84561 | 1-Jun-15 |
| 19429 | 2 | 3 | | | III-2 | Slc13a4 | 26266 | 4-May-15 |
| 19437 | 2 | 3 | | | III-2 | Slc15a5 | 729025 | 4-May-15 |
| 19463 | 2 | 3 | | | III-2 | Slc18a3 | 6572 | 4-May-15 |
| 19464 | 2 | 3 | | | III-2 | Slc18b1 | 116843 | 4-May-15 |
| 19477 | 2 | 3 | | | III-2 | Slc22a1 | 6580 | 17-May-15 |
| 19480 | 2 | 3 | | | III-2 | Slc22a13b-ps | | |
| 19488 | 2 | 3 | | | III-2 | Slc22a20 | 440044 | 4-May-15 |
| 19489 | 2 | 3 | | | III-2 | Slc22a21 | | |
| 19497 | 2 | 3 | | | III-2 | Slc22a30 | | |
| 19513 | 2 | 3 | | | III-2 | Slc25a11 | 8402 | 4-May-15 |
| 19518 | 2 | 3 | | | III-2 | Slc25a16 | 8034 | 4-May-15 |
| 19526 | 2 | 3 | | | III-2 | Slc25a23 | 79085 | 4-May-15 |
| 19536 | 2 | 3 | | | III-2 | Slc25a32 | 81034 | 4-May-15 |
| 19540 | 2 | 3 | | | III-2 | Slc25a36 | 55186 | 4-May-15 |
| 19546 | 2 | 3 | | | III-2 | Slc25a41 | 284427 | 4-May-15 |
| 19547 | 2 | 3 | | | III-2 | Slc25a42 | 284439 | 4-May-15 |
| 19550 | 2 | 3 | | | III-2 | Slc25a45 | 283130 | 12-May-15 |
| 19565 | 2 | 3 | | | III-2 | Slc26a6 | 65010 | 4-May-15 |
| 19573 | 2 | 3 | | | III-2 | Slc27a5 | 10998 | 4-May-15 |
| 19587 | 2 | 3 | | | III-2 | Slc2a3 | 6515 | 12-May-15 |
| 19600 | 2 | 3 | | | III-2 | Slc30a5 | 64924 | 4-May-15 |
| 19603 | 2 | 3 | | | III-2 | Slc30a8 | 169026 | 31-May-15 |
| 19613 | 2 | 3 | | | III-2 | Slc35a2 | 7355 | 4-May-15 |
| 19616 | 2 | 3 | | | III-2 | Slc35a5 | 55032 | 4-May-15 |
| 19627 | 2 | 3 | | | III-2 | Slc35e2 | 9906 | 7-Jun-15 |
| 19633 | 2 | 3 | | | III-2 | Slc35f4 | 341880 | 12-May-15 |
| 19635 | 2 | 3 | | | III-2 | Slc35f6 | 54978 | 4-May-15 |
| 19639 | 2 | 3 | | | III-2 | Slc36a1 | 206358 | 12-May-15 |
| 19647 | 2 | 3 | | | III-2 | Slc37a4 | 2542 | 28-May-15 |
| 19651 | 2 | 3 | | | III-2 | Slc38a2 | 54407 | 12-May-15 |
| 19660 | 2 | 3 | | | III-2 | Slc39a10 | 57181 | 4-May-15 |
| 19662 | 2 | 3 | | | III-2 | Slc39a12 | 221074 | 4-May-15 |
| 19663 | 2 | 3 | | | III-2 | Slc39a13 | 91252 | 17-May-15 |
| 19673 | 2 | 3 | | | III-2 | Slc3a1 | 6519 | 12-May-15 |
| 19684 | 2 | 3 | | | III-2 | Slc44a3 | 126969 | 4-May-15 |
| 19687 | 2 | 3 | | | III-2 | Slc45a1 | 50651 | 21-May-15 |
| 19696 | 2 | 3 | | | III-2 | Slc48a1 | 55652 | 4-May-15 |
| 19705 | 2 | 3 | | | III-2 | Slc4a7 | 9497 | 4-May-15 |
| 19707 | 2 | 3 | | | III-2 | Slc4a9 | 83697 | 4-May-15 |
| 19708 | 2 | 3 | | | III-2 | Slc50a1 | 55974 | 4-May-15 |

| | | | | | | | | |
|---|---|---|---|---|---|---|---|---|
| 19727 | 2 | 3 | | | III-2 | Slc6a11 | 6538 | 4-May-15 |
| 19729 | 2 | 3 | | | III-2 | Slc6a13 | 6540 | 4-May-15 |
| 19752 | 2 | 3 | | | III-2 | Slc7a15 | | |
| 19762 | 2 | 3 | | | III-2 | Slc8a1 | 6546 | 23-May-15 |
| 19793 | 2 | 3 | | | III-2 | Slco6c1 | | |
| 19803 | 2 | 3 | | | III-2 | Slfn8 | | |
| 19806 | 2 | 3 | | | III-2 | Slirp | 81892 | 4-May-15 |
| 19808 | 2 | 3 | | | III-2 | Slit2 | 9353 | 7-Jun-15 |
| 19811 | 2 | 3 | | | III-2 | Slitrk2 | 84631 | 4-May-15 |
| 19817 | 2 | 3 | | | III-2 | Slmap | 7871 | 12-May-15 |
| 19823 | 2 | 3 | | | III-2 | Slu7 | 10569 | 4-May-15 |
| 19830 | 2 | 3 | | | III-2 | Sly | 54440 | 4-May-15 |
| 19841 | 2 | 3 | | | III-2 | Smap2 | 64744 | 7-Jun-15 |
| 19845 | 2 | 3 | | | III-2 | Smarca5 | 8467 | 4-May-15 |
| 19847 | 2 | 3 | | | III-2 | Smarcad1 | 56916 | 4-May-15 |
| 19848 | 2 | 3 | | | III-2 | Smarcal1 | 50485 | 23-May-15 |
| 19849 | 2 | 3 | | | III-2 | Smarcb1 | 6598 | 12-May-15 |
| 19856 | 2 | 3 | | | III-2 | Smc1a | 8243 | 23-May-15 |
| 19857 | 2 | 3 | | | III-2 | Smc1b | 27127 | 4-May-15 |
| 19858 | 2 | 3 | | | III-2 | Smc2 | 10592 | 7-Jun-15 |
| 19860 | 2 | 3 | | | III-2 | Smc3 | 9126 | 23-May-15 |
| 19863 | 2 | 3 | | | III-2 | Smc6 | 79677 | 4-May-15 |
| 19867 | 2 | 3 | | | III-2 | Smco3 | 440087 | 4-May-15 |
| 19876 | 2 | 3 | | | III-2 | Smg6 | 23293 | 4-May-15 |
| 19884 | 2 | 3 | | | III-2 | Smim13 | 221710 | 12-May-15 |
| 19898 | 2 | 3 | | | III-2 | Smim8 | 57150 | 4-May-15 |
| 19900 | 2 | 3 | | | III-2 | Smlr1 | 100507 203 | 4-May-15 |
| 19903 | 2 | 3 | | | III-2 | Smo | 6608 | 7-Jun-15 |
| 19904 | 2 | 3 | | | III-2 | Smoc1 | 64093 | 4-May-15 |
| 19907 | 2 | 3 | | | III-2 | Smok2b | | |
| 19913 | 2 | 3 | | | III-2 | Smpd2 | 6610 | 4-May-15 |
| 19916 | 2 | 3 | | | III-2 | Smpd5 | 392275 | 4-May-15 |
| 19927 | 2 | 3 | | | III-2 | Smug1 | 23583 | 4-May-15 |
| 19929 | 2 | 3 | | | III-2 | Smurf2 | 64750 | 23-May-15 |
| 19935 | 2 | 3 | | | III-2 | Snai1 | 6615 | 17-May-15 |
| 19939 | 2 | 3 | | | III-2 | Snap25 | 6616 | 7-May-15 |
| 19944 | 2 | 3 | | | III-2 | Snapc2 | 6618 | 4-May-15 |
| 19974 | 2 | 3 | | | III-2 | Snora20 | 677806 | 4-May-15 |
| 19978 | 2 | 3 | | | III-2 | Snora26 | 677810 | 4-May-15 |
| 19987 | 2 | 3 | | | III-2 | Snora36b | 677818 | 4-May-15 |
| 19994 | 2 | 3 | | | III-2 | Snora61 | 677838 | 4-May-15 |
| 20007 | 2 | 3 | | | III-2 | Snord104 | 692227 | 12-May-15 |
| 20025 | 2 | 3 | | | III-2 | Snord1a | 677848 | 4-May-15 |
| 20032 | 2 | 3 | | | III-2 | Snord33 | 26818 | 4-May-15 |
| 20095 | 2 | 3 | | | III-2 | Snrpd2 | 6633 | 4-May-15 |
| 20107 | 2 | 3 | | | III-2 | Snupn | 10073 | 4-May-15 |
| 20110 | 2 | 3 | | | III-2 | Snx1 | 6642 | 21-May-15 |
| 20113 | 2 | 3 | | | III-2 | Snx12 | 29934 | 2-Jun-15 |
| 20126 | 2 | 3 | | | III-2 | Snx25 | 83891 | 4-May-15 |
| 20130 | 2 | 3 | | | III-2 | Snx30 | 401548 | 7-Jun-15 |
| 20135 | 2 | 3 | | | III-2 | Snx5 | 27131 | 12-May-15 |
| 20154 | 2 | 3 | | | III-2 | Soga3 | 387104 | 4-May-15 |
| 20165 | 2 | 3 | | | III-2 | Sord | 6652 | 12-May-15 |
| 20168 | 2 | 3 | | | III-2 | Sos1 | 6654 | 23-May-15 |
| 20187 | 2 | 3 | | | III-2 | Sox2ot | 347689 | 12-May-15 |
| 20189 | 2 | 3 | | | III-2 | Sox30 | 11063 | 28-May-15 |
| 20190 | 2 | 3 | | | III-2 | Sox4 | 6659 | 4-May-15 |
| 20192 | 2 | 3 | | | III-2 | Sox5os3 | | |
| 20198 | 2 | 3 | | | III-2 | Sp100 | 6672 | 4-May-15 |
| 20199 | 2 | 3 | | | III-2 | Sp110 | 3431 | 7-Jun-15 |
| 20200 | 2 | 3 | | | III-2 | Sp140 | 11262 | 4-May-15 |
| 20205 | 2 | 3 | | | III-2 | Sp5 | 389058 | 4-May-15 |
| 20208 | 2 | 3 | | | III-2 | Sp8 | 221833 | 4-May-15 |
| 20209 | 2 | 3 | | | III-2 | Sp9 | 100131 390 | 4-May-15 |
| 20213 | 2 | 3 | | | III-2 | Spaca4 | 171169 | 4-May-15 |
| 20215 | 2 | 3 | | | III-2 | Spaca6 | 147650 | 3-May-15 |
| 20217 | 2 | 3 | | | III-2 | Spag1 | 6674 | 26-May-15 |
| 20225 | 2 | 3 | | | III-2 | Spag7 | 9552 | 4-May-15 |
| 20232 | 2 | 3 | | | III-2 | Spata1 | 100505 741 | 4-May-15 |
| 20235 | 2 | 3 | | | III-2 | Spata17 | 128153 | 4-May-15 |
| 20244 | 2 | 3 | | | III-2 | Spata2l | 124044 | 4-May-15 |
| 20254 | 2 | 3 | | | III-2 | Spata45 | 149643 | 4-May-15 |
| 20258 | 2 | 3 | | | III-2 | Spata7 | 55812 | 23-May-15 |
| 20260 | 2 | 3 | | | III-2 | Spatc1 | 375686 | 4-May-15 |
| 20261 | 2 | 3 | | | III-2 | Spatc1l | 84221 | 4-May-15 |
| 20268 | 2 | 3 | | | III-2 | Spcs2 | 9789 | 4-May-15 |
| 20285 | 2 | 3 | | | III-2 | Speer5-ps1 | | |
| 20288 | 2 | 3 | | | III-2 | Speer8-ps1 | | |
| 20299 | 2 | 3 | | | III-2 | Spg21 | 51324 | 4-May-15 |
| 20308 | 2 | 3 | | | III-2 | Spidr | 23514 | 12-May-15 |
| 20336 | 2 | 3 | | | III-2 | Spns1 | 83985 | 4-May-15 |
| 20339 | 2 | 3 | | | III-2 | Spo11 | 23626 | 31-May-15 |
| 20346 | 2 | 3 | | | III-2 | Spopl | 339745 | 4-May-15 |
| 20349 | 2 | 3 | | | III-2 | Sppl2a | 84888 | 29-May-15 |
| 20350 | 2 | 3 | | | III-2 | Sppl2b | 56928 | 4-May-15 |
| 20352 | 2 | 3 | | | III-2 | Sppl3 | 121665 | 29-May-15 |
| 20384 | 2 | 3 | | | III-2 | Spsb4 | 92369 | 4-May-15 |
| 20390 | 2 | 3 | | | III-2 | Sptbn2 | 6712 | 23-May-15 |
| 20393 | 2 | 3 | | | III-2 | Sptlc2 | 9517 | 4-May-15 |
| 20410 | 2 | 3 | | | III-2 | Srebf1 | 6720 | 24-May-15 |
| 20424 | 2 | 3 | | | III-2 | Srp14 | 6727 | 3-Jun-15 |
| 20430 | 2 | 3 | | | III-2 | Srp72 | 6731 | 12-May-15 |
| 20436 | 2 | 3 | | | III-2 | Srprb | 58477 | 21-May-15 |
| 20440 | 2 | 3 | | | III-2 | Srrd | 402055 | 4-May-15 |
| 20442 | 2 | 3 | | | III-2 | Srrm2 | 23524 | 3-Jun-15 |

Fig.22 - 54

| | | | | | | | | |
|---|---|---|---|---|---|---|---|---|
| 20444 | 2 | 3 | | | III-2 | Srrm4 | 84530 | 4-May-15 |
| 20449 | 2 | 3 | | | III-2 | Srsf11 | 9295 | 4-May-15 |
| 20450 | 2 | 3 | | | III-2 | Srsf12 | 135295 | 4-May-15 |
| 20452 | 2 | 3 | | | III-2 | Srsf3 | 6428 | 21-May-15 |
| 20454 | 2 | 3 | | | III-2 | Srsf5 | 6430 | 1-Jun-15 |
| 20460 | 2 | 3 | | | III-2 | Ss18 | 6760 | 12-May-15 |
| 20463 | 2 | 3 | | | III-2 | Ssbp1 | 6742 | 14-May-15 |
| 20464 | 2 | 3 | | | III-2 | Ssbp2 | 23635 | 12-May-15 |
| 20466 | 2 | 3 | | | III-2 | Ssbp4 | 170463 | 12-May-15 |
| 20473 | 2 | 3 | | | III-2 | Ssna1 | 8636 | 4-May-15 |
| 20486 | 2 | 3 | | | III-2 | Sstr4 | 6754 | 12-May-15 |
| 20488 | 2 | 3 | | | III-2 | Ssty1 | | |
| 20489 | 2 | 3 | | | III-2 | Ssty2 | | |
| 20494 | 2 | 3 | | | III-2 | Ssxb1 | | |
| 20514 | 2 | 3 | | | III-2 | St6galnac1 | 55808 | 4-May-15 |
| 20517 | 2 | 3 | | | III-2 | St6galnac4 | 27090 | 4-May-15 |
| 20525 | 2 | 3 | | | III-2 | St8sia3os | | |
| 20535 | 2 | 3 | | | III-2 | Stag2 | 10735 | 21-May-15 |
| 20536 | 2 | 3 | | | III-2 | Stag3 | 10734 | 4-May-15 |
| 20537 | 2 | 3 | | | III-2 | Stam | 8027 | 4-May-15 |
| 20576 | 2 | 3 | | | III-2 | Stim2 | 57620 | 10-May-15 |
| 20580 | 2 | 3 | | | III-2 | Stk11ip | 114790 | 1-Jun-15 |
| 20582 | 2 | 3 | | | III-2 | Stk17b | 9262 | 4-May-15 |
| 20585 | 2 | 3 | | | III-2 | Stk25 | 10494 | 4-May-15 |
| 20594 | 2 | 3 | | | III-2 | Stk38 | 11329 | 20-May-15 |
| 20604 | 2 | 3 | | | III-2 | Stmnd1 | 401236 | 4-May-15 |
| 20605 | 2 | 3 | | | III-2 | Stom | 2040 | 12-May-15 |
| 20614 | 2 | 3 | | | III-2 | Stpg2 | 285555 | 12-May-15 |
| 20615 | 2 | 3 | | | III-2 | Stra13 | 201254 | 7-Jun-15 |
| 20618 | 2 | 3 | | | III-2 | Strada | 92335 | 4-May-15 |
| 20623 | 2 | 3 | | | III-2 | Strip1 | 85369 | 4-May-15 |
| 20627 | 2 | 3 | | | III-2 | Strn4 | 29888 | 12-May-15 |
| 20641 | 2 | 3 | | | III-2 | Stx4a | 6810 | 4-May-15 |
| 20648 | 2 | 3 | | | III-2 | Stxbp3a | | |
| 20652 | 2 | 3 | | | III-2 | Stxbp5l | 9515 | 12-May-15 |
| 20664 | 2 | 3 | | | III-2 | Sufu | 51684 | 23-May-15 |
| 20678 | 2 | 3 | | | III-2 | Sult2a2 | | |
| 20686 | 2 | 3 | | | III-2 | Sult4a1 | 25830 | 4-May-15 |
| 20689 | 2 | 3 | | | III-2 | Sumf1 | 285362 | 4-May-15 |
| 20692 | 2 | 3 | | | III-2 | Sumo2 | 6613 | 24-May-15 |
| 20694 | 2 | 3 | | | III-2 | Sun1 | 23353 | 4-May-15 |
| 20717 | 2 | 3 | | | III-2 | Suv39h2 | 79723 | 3-May-15 |
| 20719 | 2 | 3 | | | III-2 | Suv420h2 | 84787 | 4-May-15 |
| 20721 | 2 | 3 | | | III-2 | Sv2a | 9900 | 12-May-15 |
| 20725 | 2 | 3 | | | III-2 | Sval1 | | |
| 20727 | 2 | 3 | | | III-2 | Sval3 | | |
| 20747 | 2 | 3 | | | III-2 | Syce1l | 100130958 | 4-May-15 |
| 20752 | 2 | 3 | | | III-2 | Sycp1-ps1 | | |
| 20756 | 2 | 3 | | | III-2 | Syde2 | 84144 | 4-May-15 |
| 20764 | 2 | 3 | | | III-2 | Synb | | |
| 20767 | 2 | 3 | | | III-2 | Syndig1 | 79953 | 4-May-15 |
| 20776 | 2 | 3 | | | III-2 | Syngr3 | 9143 | 4-May-15 |
| 20778 | 2 | 3 | | | III-2 | Synj1 | 8867 | 12-May-15 |
| 20786 | 2 | 3 | | | III-2 | Synrg | 11276 | 4-May-15 |
| 20791 | 2 | 3 | | | III-2 | Syt1 | 6857 | 12-May-15 |
| 20793 | 2 | 3 | | | III-2 | Syt11 | 23208 | 16-Jun-15 |
| 20797 | 2 | 3 | | | III-2 | Syt15 | 83849 | 4-May-15 |
| 20800 | 2 | 3 | | | III-2 | Syt2 | 127833 | 5-May-15 |
| 20804 | 2 | 3 | | | III-2 | Syt6 | 148281 | 4-May-15 |
| 20807 | 2 | 3 | | | III-2 | Syt9 | 143425 | 4-May-15 |
| 20813 | 2 | 3 | | | III-2 | Syvn1 | 84447 | 29-May-15 |
| 20835 | 2 | 3 | | | III-2 | Tab3 | 257397 | 4-May-15 |
| 20852 | 2 | 3 | | | III-2 | Taf10 | 6881 | 4-May-15 |
| 20854 | 2 | 3 | | | III-2 | Taf12 | 6883 | 4-May-15 |
| 20860 | 2 | 3 | | | III-2 | Taf1d | 79101 | 12-May-15 |
| 20862 | 2 | 3 | | | III-2 | Taf3 | 83860 | 4-May-15 |
| 20867 | 2 | 3 | | | III-2 | Taf6 | 6878 | 12-May-15 |
| 20870 | 2 | 3 | | | III-2 | Taf7l | 54457 | 12-May-15 |
| 20871 | 2 | 3 | | | III-2 | Taf8 | 129685 | 4-May-15 |
| 20893 | 2 | 3 | | | III-2 | Tapbp | 6892 | 17-May-15 |
| 20896 | 2 | 3 | | | III-2 | Tarbp2 | 6895 | 12-May-15 |
| 20906 | 2 | 3 | | | III-2 | Tas2r103 | | |
| 20943 | 2 | 3 | | | III-2 | Tatdn2 | 9797 | 12-May-15 |
| 20948 | 2 | 3 | | | III-2 | Tbata | 219793 | 12-May-15 |
| 20954 | 2 | 3 | | | III-2 | Tbc1d13 | 54662 | 4-May-15 |
| 20959 | 2 | 3 | | | III-2 | Tbc1d19 | 55296 | 4-May-15 |
| 20972 | 2 | 3 | | | III-2 | Tbc1d32 | 221322 | 4-May-15 |
| 20984 | 2 | 3 | | | III-2 | Tbcd | 6904 | 4-May-15 |
| 20989 | 2 | 3 | | | III-2 | Tbkbp1 | 9755 | 4-May-15 |
| 20992 | 2 | 3 | | | III-2 | Tbl2 | 26608 | 4-May-15 |
| 20994 | 2 | 3 | | | III-2 | Tbp | 6908 | 31-May-15 |
| 20996 | 2 | 3 | | | III-2 | Tbpl2 | 387332 | 7-Jun-15 |
| 21005 | 2 | 3 | | | III-2 | Tbx19 | 9095 | 4-May-15 |
| 21019 | 2 | 3 | | | III-2 | Tcam1 | 146771 | 4-May-15 |
| 21035 | 2 | 3 | | | III-2 | Tcerg1 | 10915 | 4-May-15 |
| 21050 | 2 | 3 | | | III-2 | Tcfl5 | 10732 | 24-May-15 |
| 21063 | 2 | 3 | | | III-2 | Tcp1 | 6950 | 17-May-15 |
| 21071 | 2 | 3 | | | III-2 | Tcstv3 | | |
| 21074 | 2 | 3 | | | III-2 | Tcte2 | | |
| 21081 | 2 | 3 | | | III-2 | Tctn3 | 26123 | 23-May-15 |
| 21084 | 2 | 3 | | | III-2 | Tdh | 157739 | 7-Jun-15 |
| 21086 | 2 | 3 | | | III-2 | Tdp1 | 55775 | 24-May-15 |
| 21087 | 2 | 3 | | | III-2 | Tdp2 | 51567 | 3-May-15 |
| 21098 | 2 | 3 | | | III-2 | Tdrd7 | 23424 | 4-May-15 |
| 21100 | 2 | 3 | | | III-2 | Tdrkh | 11022 | 4-May-15 |
| 21104 | 2 | 3 | | | III-2 | Tead3 | 7005 | 4-May-15 |
| 21107 | 2 | 3 | | | III-2 | Tecpr1 | 25851 | 21-May-15 |
| 21108 | 2 | 3 | | | III-2 | Tecpr2 | 9895 | 4-May-15 |
| 21130 | 2 | 3 | | | III-2 | Tepp | 374739 | 4-May-15 |
| 21135 | 2 | 3 | | | III-2 | Tert | 7015 | 4-Jun-15 |
| 21139 | 2 | 3 | | | III-2 | Tesk1 | 7016 | 4-May-15 |
| 21143 | 2 | 3 | | | III-2 | Tet2 | 54790 | 17-May-15 |
| 21146 | 2 | 3 | | | III-2 | Tex101 | 83639 | 4-May-15 |
| 21147 | 2 | 3 | | | III-2 | Tex11 | 56159 | 4-May-15 |
| 21150 | 2 | 3 | | | III-2 | Tex13a | 56157 | 21-May-15 |
| 21154 | 2 | 3 | | | III-2 | Tex19.1 | | |
| 21158 | 2 | 3 | | | III-2 | Tex22 | 647310 | 4-May-15 |
| 21167 | 2 | 3 | | | III-2 | Tex35 | 84066 | 4-May-15 |
| 21173 | 2 | 3 | | | III-2 | Tex9 | 374618 | 4-May-15 |
| 21178 | 2 | 3 | | | III-2 | Tfap2d | 83741 | 4-May-15 |
| 21182 | 2 | 3 | | | III-2 | Tfb2m | 64216 | 4-May-15 |
| 21226 | 2 | 3 | | | III-2 | Tgtp1 | | |
| 21229 | 2 | 3 | | | III-2 | Tha1 | | |
| 21238 | 2 | 3 | | | III-2 | Thbd | 7056 | 23-May-15 |
| 21244 | 2 | 3 | | | III-2 | Them4 | 117145 | 4-May-15 |
| 21251 | 2 | 3 | | | III-2 | Thg1l | 54974 | 4-May-15 |
| 21253 | 2 | 3 | | | III-2 | Thnsl2 | 55258 | 4-May-15 |
| 21256 | 2 | 3 | | | III-2 | Thoc3 | 84321 | 4-May-15 |
| 21257 | 2 | 3 | | | III-2 | Thoc5 | 8563 | 4-May-15 |
| 21260 | 2 | 3 | | | III-2 | Thop1 | 7064 | 12-May-15 |
| 21263 | 2 | 3 | | | III-2 | Thrap3 | 9967 | 12-May-15 |
| 21264 | 2 | 3 | | | III-2 | Thrb | 7068 | 14-May-15 |
| 21269 | 2 | 3 | | | III-2 | Thsd7b | 80731 | 4-May-15 |
| 21270 | 2 | 3 | | | III-2 | Thtpa | 79178 | 4-May-15 |
| 21274 | 2 | 3 | | | III-2 | Thy1 | 7070 | 3-May-15 |
| 21277 | 2 | 3 | | | III-2 | Tial1 | 7073 | 2-Jun-15 |
| 21281 | 2 | 3 | | | III-2 | Ticam2 | 353376 | 4-May-15 |
| 21287 | 2 | 3 | | | III-2 | Tigd3 | 220359 | 21-May-15 |
| 21290 | 2 | 3 | | | III-2 | Tigit | 201633 | 3-May-15 |
| 21294 | 2 | 3 | | | III-2 | Timm10 | 26519 | 21-May-15 |
| 21296 | 2 | 3 | | | III-2 | Timm13 | 26517 | 4-May-15 |
| 21301 | 2 | 3 | | | III-2 | Timm23 | 100287932 | 7-Jun-15 |
| 21306 | 2 | 3 | | | III-2 | Timm8b | 26521 | 4-May-15 |
| 21308 | 2 | 3 | | | III-2 | Timmdc1 | 51300 | 4-May-15 |
| 21311 | 2 | 3 | | | III-2 | Timp3 | 7078 | 12-May-15 |
| 21314 | 2 | 3 | | | III-2 | Tinagl1 | 64129 | 12-May-15 |
| 21319 | 2 | 3 | | | III-2 | Tirap | 114609 | 12-May-15 |
| 21328 | 2 | 3 | | | III-2 | Tktl2 | 84076 | 4-May-15 |
| 21332 | 2 | 3 | | | III-2 | Tldc2 | 140711 | 4-May-15 |
| 21336 | 2 | 3 | | | III-2 | Tle4 | 7091 | 31-May-15 |
| 21339 | 2 | 3 | | | III-2 | Tlk2 | 11011 | 4-May-15 |
| 21342 | 2 | 3 | | | III-2 | Tln1 | 7094 | 12-May-15 |
| 21357 | 2 | 3 | | | III-2 | Tlx2 | 3196 | 7-Jun-15 |
| 21359 | 2 | 3 | | | III-2 | Tm2d1 | 83941 | 4-May-15 |
| 21361 | 2 | 3 | | | III-2 | Tm2d3 | 80213 | 12-May-15 |
| 21371 | 2 | 3 | | | III-2 | Tm9sf1 | 10548 | 21-May-15 |
| 21372 | 2 | 3 | | | III-2 | Tm9sf2 | 9375 | 4-May-15 |
| 21378 | 2 | 3 | | | III-2 | Tmbim4 | 51643 | 4-May-15 |
| 21393 | 2 | 3 | | | III-2 | Tmco2 | 127391 | 4-May-15 |
| 21396 | 2 | 3 | | | III-2 | Tmco5 | 145942 | 12-May-15 |
| 21401 | 2 | 3 | | | III-2 | Tmed11 | | |
| 21403 | 2 | 3 | | | III-2 | Tmed3 | 23423 | 4-May-15 |
| 21405 | 2 | 3 | | | III-2 | Tmed5 | 50999 | 4-May-15 |
| 21410 | 2 | 3 | | | III-2 | Tmeff1 | 8577 | 4-May-15 |
| 21422 | 2 | 3 | | | III-2 | Tmem11 | 8834 | 4-May-15 |
| 21435 | 2 | 3 | | | III-2 | Tmem127 | 55654 | 23-May-15 |
| 21436 | 2 | 3 | | | III-2 | Tmem128 | 85013 | 12-May-15 |
| 21440 | 2 | 3 | | | III-2 | Tmem132a | 54972 | 4-May-15 |
| 21442 | 2 | 3 | | | III-2 | Tmem132c | 92293 | 4-May-15 |
| 21443 | 2 | 3 | | | III-2 | Tmem132cos | | |
| 21444 | 2 | 3 | | | III-2 | Tmem132d | 121256 | 4-May-15 |
| 21448 | 2 | 3 | | | III-2 | Tmem136 | 219902 | 4-May-15 |
| 21459 | 2 | 3 | | | III-2 | Tmem150a | 129303 | 4-May-15 |
| 21463 | 2 | 3 | | | III-2 | Tmem151a | 256472 | 4-May-15 |
| 21467 | 2 | 3 | | | III-2 | Tmem159 | 57146 | 12-May-15 |
| 21469 | 2 | 3 | | | III-2 | Tmem161a | 54929 | 4-May-15 |
| 21470 | 2 | 3 | | | III-2 | Tmem161b | 153396 | 4-May-15 |
| 21476 | 2 | 3 | | | III-2 | Tmem168 | 64418 | 4-May-15 |
| 21484 | 2 | 3 | | | III-2 | Tmem175 | 84286 | 12-May-15 |
| 21495 | 2 | 3 | | | III-2 | Tmem181b-ps | | |
| 21502 | 2 | 3 | | | III-2 | Tmem185b | 79134 | 12-May-15 |
| 21503 | 2 | 3 | | | III-2 | Tmem186 | 25880 | 4-May-15 |
| 21504 | 2 | 3 | | | III-2 | Tmem189 | 387521 | 4-May-15 |
| 21510 | 2 | 3 | | | III-2 | Tmem194b | 100131211 | 28-May-15 |
| 21511 | 2 | 3 | | | III-2 | Tmem196 | 256130 | 4-May-15 |
| 21514 | 2 | 3 | | | III-2 | Tmem199 | 147007 | 4-May-15 |
| 21515 | 2 | 3 | | | III-2 | Tmem2 | 23670 | 4-May-15 |
| 21526 | 2 | 3 | | | III-2 | Tmem208 | 29100 | 4-May-15 |
| 21528 | 2 | 3 | | | III-2 | Tmem210 | 100505993 | 4-May-15 |
| 21529 | 2 | 3 | | | III-2 | Tmem211 | 255349 | 4-May-15 |
| 21533 | 2 | 3 | | | III-2 | Tmem215 | 401498 | 4-May-15 |
| 21536 | 2 | 3 | | | III-2 | Tmem218 | 219854 | 4-May-15 |
| 21541 | 2 | 3 | | | III-2 | Tmem223 | 79064 | 4-May-15 |
| 21543 | 2 | 3 | | | III-2 | Tmem229a | 730130 | 12-May-15 |
| 21546 | 2 | 3 | | | III-2 | Tmem231 | 79583 | 23-May-15 |
| 21550 | 2 | 3 | | | III-2 | Tmem235 | 283999 | 4-May-15 |
| 21551 | 2 | 3 | | | III-2 | Tmem236 | 653567 | 4-May-15 |
| 21575 | 2 | 3 | | | III-2 | Tmem26 | 219623 | 4-May-15 |
| 21577 | 2 | 3 | | | III-2 | Tmem261 | 90871 | 21-May-15 |
| 21578 | 2 | 3 | | | III-2 | Tmem263 | 90488 | 4-May-15 |
| 21580 | 2 | 3 | | | III-2 | Tmem28 | 27112 | 4-May-15 |
| 21582 | 2 | 3 | | | III-2 | Tmem30a | 55754 | 3-May-15 |

Fig.22 - 55

| | | | | | | | | |
|---|---|---|---|---|---|---|---|---|
| 21585 | 2 | 3 | | | III-2 | Tmem33 | 55161 | 1-Jun-15 |
| 21595 | 2 | 3 | | | III-2 | Tmem42 | 131616 | 4-May-15 |
| 21611 | 2 | 3 | | | III-2 | Tmem55b | 90809 | 4-May-15 |
| 21617 | 2 | 3 | | | III-2 | Tmem62 | 80021 | 4-May-15 |
| 21619 | 2 | 3 | | | III-2 | Tmem63b | 55362 | 4-May-15 |
| 21624 | 2 | 3 | | | III-2 | Tmem67 | 91147 | 23-May-15 |
| 21628 | 2 | 3 | | | III-2 | Tmem71 | 137835 | 4-May-15 |
| 21630 | 2 | 3 | | | III-2 | Tmem74 | 157753 | 21-May-15 |
| 21635 | 2 | 3 | | | III-2 | Tmem81 | 388730 | 4-May-15 |
| 21640 | 2 | 3 | | | III-2 | Tmem87b | 84910 | 4-May-15 |
| 21641 | 2 | 3 | | | III-2 | Tmem88 | 92162 | 4-May-15 |
| 21653 | 2 | 3 | | | III-2 | Tmevpg1 | 100885789 | 1-May-15 |
| 21663 | 2 | 3 | | | III-2 | Tmppe | 643853 | 4-May-15 |
| 21677 | 2 | 3 | | | III-2 | Tmprss5 | 80975 | 12-May-15 |
| 21679 | 2 | 3 | | | III-2 | Tmprss7 | 344805 | 12-May-15 |
| 21681 | 2 | 3 | | | III-2 | Tmsb10 | 9168 | 21-May-15 |
| 21690 | 2 | 3 | | | III-2 | Tmtc4 | 84899 | 4-May-15 |
| 21692 | 2 | 3 | | | III-2 | Tmub2 | 79089 | 4-May-15 |
| 21707 | 2 | 3 | | | III-2 | Tnfrsf10b | 8795 | 17-May-15 |
| 21708 | 2 | 3 | | | III-2 | Tnfrsf11a | 8792 | 31-May-15 |
| 21721 | 2 | 3 | | | III-2 | Tnfrsf23 | | |
| 21735 | 2 | 3 | | | III-2 | Tnfsf18 | 8995 | 30-Apr-15 |
| 21737 | 2 | 3 | | | III-2 | Tnfsf8 | 944 | 17-May-15 |
| 21764 | 2 | 3 | | | III-2 | Tnpo3 | 23534 | 4-May-15 |
| 21770 | 2 | 3 | | | III-2 | Tns1 | 7145 | 12-May-15 |
| 21782 | 2 | 3 | | | III-2 | Tomm20l | 387990 | 4-May-15 |
| 21792 | 2 | 3 | | | III-2 | Tomt | | |
| 21794 | 2 | 3 | | | III-2 | Top1 | 7150 | 12-May-15 |
| 21795 | 2 | 3 | | | III-2 | Top1mt | 116447 | 14-May-15 |
| 21798 | 2 | 3 | | | III-2 | Top3a | 7156 | 4-May-15 |
| 21803 | 2 | 3 | | | III-2 | Toporsl | | |
| 21806 | 2 | 3 | | | III-2 | Tor1aip1 | 26092 | 4-May-15 |
| 21818 | 2 | 3 | | | III-2 | Tpbpb | | |
| 21824 | 2 | 3 | | | III-2 | Tpgs1 | 91978 | 12-May-15 |
| 21826 | 2 | 3 | | | III-2 | Tph1 | 7166 | 3-May-15 |
| 21828 | 2 | 3 | | | III-2 | Tpi1 | 7167 | 4-May-15 |
| 21833 | 2 | 3 | | | III-2 | Tpm4 | 7171 | 14-May-15 |
| 21843 | 2 | 3 | | | III-2 | Tprg | | |
| 21847 | 2 | 3 | | | III-2 | Tpsab1 | 7177 | 12-May-15 |
| 21850 | 2 | 3 | | | III-2 | Tpst1 | 8460 | 21-May-15 |
| 21856 | 2 | 3 | | | III-2 | Tra2b | 6434 | 17-May-15 |
| 21857 | 2 | 3 | | | III-2 | Trabd | 80305 | 4-May-15 |
| 21860 | 2 | 3 | | | III-2 | Traf1 | 7185 | 4-May-15 |
| 21862 | 2 | 3 | | | III-2 | Traf3 | 7187 | 29-May-15 |
| 21864 | 2 | 3 | | | III-2 | Traf3ip2 | 10758 | 13-May-15 |
| 21866 | 2 | 3 | | | III-2 | Traf4 | 9618 | 21-May-15 |
| 21869 | 2 | 3 | | | III-2 | Traf7 | 84231 | 4-May-15 |
| 21870 | 2 | 3 | | | III-2 | Trafd1 | 10906 | 12-May-15 |
| 21873 | 2 | 3 | | | III-2 | Trak2 | 66008 | 4-May-15 |
| 21879 | 2 | 3 | | | III-2 | Trap1a | | |
| 21888 | 2 | 3 | | | III-2 | Trappc3l | 100128327 | 7-Jun-15 |
| 21891 | 2 | 3 | | | III-2 | Trappc6a | 79090 | 4-May-15 |
| 21897 | 2 | 3 | | | III-2 | Trdmt1 | 1787 | 12-May-15 |
| 21906 | 2 | 3 | | | III-2 | Trerf1 | 55809 | 28-May-15 |
| 21907 | 2 | 3 | | | III-2 | Trex1 | 11277 | 23-May-15 |
| 21911 | 2 | 3 | | | III-2 | Trhde | 29953 | 4-May-15 |
| 21915 | 2 | 3 | | | III-2 | Trib1 | 10221 | 4-May-15 |
| 21921 | 2 | 3 | | | III-2 | Trim12a | | |
| 21928 | 2 | 3 | | | III-2 | Trim2 | 23321 | 4-May-15 |
| 21933 | 2 | 3 | | | III-2 | Trim26 | 7726 | 4-May-15 |
| 21942 | 2 | 3 | | | III-2 | Trim31 | 11074 | 7-Jun-15 |
| 21950 | 2 | 3 | | | III-2 | Trim38 | 10475 | 12-May-15 |
| 21952 | 2 | 3 | | | III-2 | Trim40 | 135644 | 7-Jun-15 |
| 21954 | 2 | 3 | | | III-2 | Trim42 | 287015 | 4-May-15 |
| 21963 | 2 | 3 | | | III-2 | Trim52 | 84851 | 12-May-15 |
| 21970 | 2 | 3 | | | III-2 | Trim60 | 166655 | 4-May-15 |
| 21971 | 2 | 3 | | | III-2 | Trim61 | 391712 | 4-May-15 |
| 21976 | 2 | 3 | | | III-2 | Trim67 | 440730 | 12-May-15 |
| 21983 | 2 | 3 | | | III-2 | Trim8 | 81603 | 4-May-15 |
| 21985 | 2 | 3 | | | III-2 | Triml1 | 339976 | 4-May-15 |
| 21989 | 2 | 3 | | | III-2 | Trip10 | 9322 | 4-May-15 |
| 21992 | 2 | 3 | | | III-2 | Trip13 | 9319 | 4-May-15 |
| 21994 | 2 | 3 | | | III-2 | Trip6 | 7205 | 12-May-15 |
| 22004 | 2 | 3 | | | III-2 | Trmt13 | 54482 | 4-May-15 |
| 22006 | 2 | 3 | | | III-2 | Trmt2a | 27037 | 4-May-15 |
| 22037 | 2 | 3 | | | III-2 | Trpc4ap | 26133 | 12-May-15 |
| 22038 | 2 | 3 | | | III-2 | Trpc5 | 7224 | 4-May-15 |
| 22042 | 2 | 3 | | | III-2 | Trpd52l3 | | |
| 22049 | 2 | 3 | | | III-2 | Trpm7 | 54822 | 4-May-15 |
| 22051 | 2 | 3 | | | III-2 | Trps1 | 7227 | 4-May-15 |
| 22054 | 2 | 3 | | | III-2 | Trpv2 | 51393 | 4-May-15 |
| 22060 | 2 | 3 | | | III-2 | Trub1 | 142940 | 12-May-15 |
| 22075 | 2 | 3 | | | III-2 | Tsen54 | 283989 | 23-May-15 |
| 22086 | 2 | 3 | | | III-2 | Tsix | 9383 | 12-May-15 |
| 22087 | 2 | 3 | | | III-2 | Tsks | 60385 | 4-May-15 |
| 22091 | 2 | 3 | | | III-2 | Tsnax | 7257 | 12-May-15 |
| 22095 | 2 | 3 | | | III-2 | Tspan11 | 441631 | 12-May-15 |
| 22099 | 2 | 3 | | | III-2 | Tspan15 | 23555 | 4-May-15 |
| 22104 | 2 | 3 | | | III-2 | Tspan3 | 10099 | 4-May-15 |
| 22106 | 2 | 3 | | | III-2 | Tspan32 | 10077 | 4-May-15 |
| 22115 | 2 | 3 | | | III-2 | Tspo | 706 | 12-May-15 |
| 22117 | 2 | 3 | | | III-2 | Tspyl1 | 7259 | 4-May-15 |
| 22123 | 2 | 3 | | | III-2 | Tsr1 | 55720 | 12-May-15 |
| 22125 | 2 | 3 | | | III-2 | Tsr3 | 115939 | 4-May-15 |
| 22127 | 2 | 3 | | | III-2 | Tssc4 | 10078 | 12-May-15 |
| 22128 | 2 | 3 | | | III-2 | Tssk1 | 83942 | 7-Jun-15 |
| 22138 | 2 | 3 | | | III-2 | Tstd3 | 100130890 | 12-May-15 |
| 22142 | 2 | 3 | | | III-2 | Ttc1 | 7265 | 4-May-15 |
| 22147 | 2 | 3 | | | III-2 | Ttc17 | 55761 | 12-May-15 |
| 22158 | 2 | 3 | | | III-2 | Ttc27 | 55622 | 12-May-15 |
| 22161 | 2 | 3 | | | III-2 | Ttc3 | 7267 | 12-May-15 |
| 22167 | 2 | 3 | | | III-2 | Ttc34 | 100287898 | 4-May-15 |
| 22171 | 2 | 3 | | | III-2 | Ttc39a | 22996 | 12-May-15 |
| 22173 | 2 | 3 | | | III-2 | Ttc39c | 125488 | 12-May-15 |
| 22175 | 2 | 3 | | | III-2 | Ttc4 | 7268 | 4-May-15 |
| 22184 | 2 | 3 | | | III-2 | Ttf2 | 8458 | 7-Jun-15 |
| 22186 | 2 | 3 | | | III-2 | Tti2 | 80185 | 4-May-15 |
| 22201 | 2 | 3 | | | III-2 | Ttll9 | 164395 | 4-May-15 |
| 22210 | 2 | 3 | | | III-2 | Tuba1a | 7846 | 4-May-15 |
| 22218 | 2 | 3 | | | III-2 | Tubb1 | 81027 | 7-Jun-15 |
| 22225 | 2 | 3 | | | III-2 | Tubb5 | 203068 | 7-Jun-15 |
| 22226 | 2 | 3 | | | III-2 | Tubb6 | 84617 | 12-May-15 |
| 22228 | 2 | 3 | | | III-2 | Tube1 | 51175 | 4-May-15 |
| 22230 | 2 | 3 | | | III-2 | Tubg2 | 27175 | 14-May-15 |
| 22233 | 2 | 3 | | | III-2 | Tubgcp4 | 27229 | 30-May-15 |
| 22235 | 2 | 3 | | | III-2 | Tubgcp6 | 85378 | 4-May-15 |
| 22243 | 2 | 3 | | | III-2 | Tunar | 100507043 | 16-May-15 |
| 22249 | 2 | 3 | | | III-2 | Tvp23a | 780776 | 4-May-15 |
| 22261 | 2 | 3 | | | III-2 | Txn1 | | |
| 22280 | 2 | 3 | | | III-2 | Tymp | 1890 | 23-May-15 |
| 22288 | 2 | 3 | | | III-2 | Tyw1 | 55253 | 4-May-15 |
| 22289 | 2 | 3 | | | III-2 | Tyw3 | 127253 | 4-May-15 |
| 22293 | 2 | 3 | | | III-2 | U2af2 | 11338 | 21-May-15 |
| 22294 | 2 | 3 | | | III-2 | U2surp | 23350 | 4-May-15 |
| 22296 | 2 | 3 | | | III-2 | Uaca | 55075 | 4-May-15 |
| 22297 | 2 | 3 | | | III-2 | Uap1 | 6675 | 4-May-15 |
| 22300 | 2 | 3 | | | III-2 | Uba1y | | |
| 22312 | 2 | 3 | | | III-2 | Ubap1l | 390595 | 4-May-15 |
| 22314 | 2 | 3 | | | III-2 | Ubap2l | 9898 | 4-May-15 |
| 22318 | 2 | 3 | | | III-2 | Ubc | 7316 | 7-Jun-15 |
| 22321 | 2 | 3 | | | III-2 | Ube2b | 7320 | 12-May-15 |
| 22326 | 2 | 3 | | | III-2 | Ube2d2b | | |
| 22333 | 2 | 3 | | | III-2 | Ube2f | 140739 | 2-Jun-15 |
| 22341 | 2 | 3 | | | III-2 | Ube2l3 | 7332 | 3-May-15 |
| 22353 | 2 | 3 | | | III-2 | Ube2v1 | 7335 | 2-Jun-15 |
| 22363 | 2 | 3 | | | III-2 | Ubiad1 | 29914 | 23-May-15 |
| 22365 | 2 | 3 | | | III-2 | Ubl4 | 8266 | 29-May-15 |
| 22370 | 2 | 3 | | | III-2 | Ubn1 | 29855 | 12-May-15 |
| 22373 | 2 | 3 | | | III-2 | Ubp1 | 7342 | 4-May-15 |
| 22385 | 2 | 3 | | | III-2 | Ubtd1 | 80019 | 28-May-15 |
| 22387 | 2 | 3 | | | III-2 | Ubtf | 7343 | 12-May-15 |
| 22388 | 2 | 3 | | | III-2 | Ubtfl1 | 642623 | 4-May-15 |
| 22395 | 2 | 3 | | | III-2 | Ubxn6 | 80700 | 4-May-15 |
| 22398 | 2 | 3 | | | III-2 | Uchl1 | 7345 | 23-May-15 |
| 22399 | 2 | 3 | | | III-2 | Uchl1os | | |
| 22400 | 2 | 3 | | | III-2 | Uchl3 | 7347 | 12-May-15 |
| 22404 | 2 | 3 | | | III-2 | Uck2 | 7371 | 4-May-15 |
| 22405 | 2 | 3 | | | III-2 | Uckl1 | 54963 | 4-May-15 |
| 22415 | 2 | 3 | | | III-2 | Ufc1 | 51506 | 12-May-15 |
| 22421 | 2 | 3 | | | III-2 | Ugcg | 7357 | 4-May-15 |
| 22424 | 2 | 3 | | | III-2 | Uggt2 | 55757 | 4-May-15 |
| 22442 | 2 | 3 | | | III-2 | Ugt2b38 | | |
| 22450 | 2 | 3 | | | III-2 | Uhrf1bp1l | 23074 | 4-May-15 |
| 22455 | 2 | 3 | | | III-2 | Ulk2 | 9706 | 21-May-15 |
| 22457 | 2 | 3 | | | III-2 | Ulk4 | 54986 | 4-May-15 |
| 22473 | 2 | 3 | | | III-2 | Unc5cl | 222643 | 4-May-15 |
| 22482 | 2 | 3 | | | III-2 | Unkl | 64718 | 4-May-15 |
| 22495 | 2 | 3 | | | III-2 | Upp1 | 7378 | 4-May-15 |
| 22513 | 2 | 3 | | | III-2 | Uri1 | 8725 | 24-May-15 |
| 22519 | 2 | 3 | | | III-2 | Use1 | 55850 | 7-Jun-15 |
| 22524 | 2 | 3 | | | III-2 | Ush2a | 7399 | 23-May-15 |
| 22525 | 2 | 3 | | | III-2 | Ushbp1 | 83878 | 12-May-15 |
| 22528 | 2 | 3 | | | III-2 | Usp1 | 7398 | 4-May-15 |
| 22530 | 2 | 3 | | | III-2 | Usp11 | 8237 | 21-May-15 |
| 22534 | 2 | 3 | | | III-2 | Usp15 | 9958 | 2-Jun-15 |
| 22546 | 2 | 3 | | | III-2 | Usp22 | 23326 | 21-May-15 |
| 22557 | 2 | 3 | | | III-2 | Usp33 | 23032 | 21-May-15 |
| 22561 | 2 | 3 | | | III-2 | Usp37 | 57695 | 4-May-15 |
| 22575 | 2 | 3 | | | III-2 | Usp50 | 373509 | 12-May-15 |
| 22581 | 2 | 3 | | | III-2 | Usp8 | 9101 | 4-May-15 |
| 22591 | 2 | 3 | | | III-2 | Utp18 | 51096 | 4-May-15 |
| 22605 | 2 | 3 | | | III-2 | V1ra8 | | |
| 22615 | 2 | 3 | | | III-2 | Vamp8 | 8673 | 28-May-15 |
| 22616 | 2 | 3 | | | III-2 | Vangl1 | 81839 | 4-May-15 |
| 22619 | 2 | 3 | | | III-2 | Vapb | 9217 | 23-May-15 |
| 22639 | 2 | 3 | | | III-2 | Vcp | 7415 | 29-May-15 |
| 22650 | 2 | 3 | | | III-2 | Vezf1 | 7716 | 4-May-15 |
| 22651 | 2 | 3 | | | III-2 | Vezt | 55591 | 4-May-15 |
| 22658 | 2 | 3 | | | III-2 | Vil1 | 7429 | 12-May-15 |
| 22659 | 2 | 3 | | | III-2 | Vill | 50853 | 12-May-15 |
| 22661 | 2 | 3 | | | III-2 | Vimp | 55829 | 29-May-15 |
| 22664 | 2 | 3 | | | III-2 | Vipr1 | 7433 | 12-May-15 |
| 22671 | 2 | 3 | | | III-2 | Vmac | 400673 | 4-May-15 |
| 22924 | 2 | 3 | | | III-2 | Vmn2r30 | | |
| 23014 | 2 | 3 | | | III-2 | Vps13a | 23230 | 23-May-15 |
| 23018 | 2 | 3 | | | III-2 | Vps16 | 64601 | 21-May-15 |
| 23025 | 2 | 3 | | | III-2 | Vps33a | 65082 | 4-May-15 |
| 23027 | 2 | 3 | | | III-2 | Vps35 | 55737 | 23-May-15 |
| 23034 | 2 | 3 | | | III-2 | Vps41 | 27072 | 7-Jun-15 |
| 23035 | 2 | 3 | | | III-2 | Vps45 | 11311 | 4-May-15 |
| 23037 | 2 | 3 | | | III-2 | Vps4b | 9525 | 31-May-15 |

Fig.22 - 56

| | | | | | | | | |
|---|---|---|---|---|---|---|---|---|
| 23045 | 2 | 3 | | | III-2 | Vrk1 | 7443 | 4-May-15 |
| 23048 | 2 | 3 | | | III-2 | Vrtn | 55237 | 4-May-15 |
| 23049 | 2 | 3 | | | III-2 | Vsig1 | 340547 | 4-May-15 |
| 23056 | 2 | 3 | | | III-2 | Vstm2a | 222008 | 4-May-15 |
| 23073 | 2 | 3 | | | III-2 | Vwa5b2 | 90113 | 4-May-15 |
| 23076 | 2 | 3 | | | III-2 | Vwa9 | 81556 | 4-May-15 |
| 23077 | 2 | 3 | | | III-2 | Vwc2 | 375567 | 4-May-15 |
| 23083 | 2 | 3 | | | III-2 | Wap | | |
| 23089 | 2 | 3 | | | III-2 | Wasf2 | 10163 | 4-May-15 |
| 23093 | 2 | 3 | | | III-2 | Wbp1 | 23559 | 7-Jun-15 |
| 23095 | 2 | 3 | | | III-2 | Wbp1l | 54838 | 4-May-15 |
| 23116 | 2 | 3 | | | III-2 | Wdr16 | 146845 | 4-May-15 |
| 23143 | 2 | 3 | | | III-2 | Wdr5 | 11091 | 4-May-15 |
| 23151 | 2 | 3 | | | III-2 | Wdr60 | 55112 | 4-May-15 |
| 23156 | 2 | 3 | | | III-2 | Wdr65 | 149465 | 12-May-15 |
| 23163 | 2 | 3 | | | III-2 | Wdr76 | 79968 | 4-May-15 |
| 23165 | 2 | 3 | | | III-2 | Wdr78 | 79819 | 4-May-15 |
| 23167 | 2 | 3 | | | III-2 | Wdr81 | 124997 | 4-May-15 |
| 23168 | 2 | 3 | | | III-2 | Wdr82 | 80335 | 10-Jun-15 |
| 23169 | 2 | 3 | | | III-2 | Wdr83 | 84292 | 4-May-15 |
| 23176 | 2 | 3 | | | III-2 | Wdr93 | 56964 | 4-May-15 |
| 23178 | 2 | 3 | | | III-2 | Wdr96 | 80217 | 4-May-15 |
| 23207 | 2 | 3 | | | III-2 | Whsc1l1 | 54904 | 7-May-15 |
| 23208 | 2 | 3 | | | III-2 | Wibg | 84305 | 4-May-15 |
| 23211 | 2 | 3 | | | III-2 | Wipf2 | 147179 | 10-May-15 |
| 23215 | 2 | 3 | | | III-2 | Wisp1 | 8840 | 17-May-15 |
| 23218 | 2 | 3 | | | III-2 | Wiz | 58525 | 4-May-15 |
| 23232 | 2 | 3 | | | III-2 | Wnt3a | 89780 | 17-May-15 |
| 23240 | 2 | 3 | | | III-2 | Wnt8b | 7479 | 4-May-15 |
| 23243 | 2 | 3 | | | III-2 | Wrap53 | 55135 | 23-May-15 |
| 23245 | 2 | 3 | | | III-2 | Wrn | 7486 | 23-May-15 |
| 23249 | 2 | 3 | | | III-2 | Wscd1 | 23302 | 4-May-15 |
| 23251 | 2 | 3 | | | III-2 | Wt1 | 7490 | 24-May-15 |
| 23252 | 2 | 3 | | | III-2 | Wt1os | | |
| 23253 | 2 | 3 | | | III-2 | Wtap | 9589 | 4-May-15 |
| 23259 | 2 | 3 | | | III-2 | Wwp2 | 11060 | 23-May-15 |
| 23262 | 2 | 3 | | | III-2 | Xaf1 | 54739 | 4-May-15 |
| 23273 | 2 | 3 | | | III-2 | Xkr5 | 389610 | 4-May-15 |
| 23275 | 2 | 3 | | | III-2 | Xkr7 | 343702 | 4-May-15 |
| 23276 | 2 | 3 | | | III-2 | Xkr8 | 55113 | 4-May-15 |
| 23277 | 2 | 3 | | | III-2 | Xkr9 | 389668 | 4-May-15 |
| 23285 | 2 | 3 | | | III-2 | Xlr4c | | |
| 23286 | 2 | 3 | | | III-2 | Xlr5a | | |
| 23297 | 2 | 3 | | | III-2 | Xpo4 | 64328 | 4-May-15 |
| 23312 | 2 | 3 | | | III-2 | Xrra1 | 143570 | 4-May-15 |
| 23317 | 2 | 3 | | | III-2 | Yae1d1 | 57002 | 12-May-15 |
| 23319 | 2 | 3 | | | III-2 | Yap1 | 10413 | 31-May-15 |
| 23324 | 2 | 3 | | | III-2 | Ybx2 | 51087 | 4-May-15 |
| 23328 | 2 | 3 | | | III-2 | Yeats4 | 8089 | 4-May-15 |
| 23331 | 2 | 3 | | | III-2 | Yif1b | 90522 | 4-May-15 |
| 23332 | 2 | 3 | | | III-2 | Yipf1 | 54432 | 28-May-15 |
| 23340 | 2 | 3 | | | III-2 | Ylpm1 | 56252 | 12-May-15 |
| 23348 | 2 | 3 | | | III-2 | Yrdc | 79693 | 4-May-15 |
| 23352 | 2 | 3 | | | III-2 | Ythdf2 | 51441 | 4-May-15 |
| 23363 | 2 | 3 | | | III-2 | Zak | 51776 | 4-May-15 |
| 23371 | 2 | 3 | | | III-2 | Zbed5 | 58486 | 4-May-15 |
| 23375 | 2 | 3 | | | III-2 | Zbtb10 | 65986 | 4-May-15 |
| 23376 | 2 | 3 | | | III-2 | Zbtb11 | 27107 | 12-May-15 |
| 23381 | 2 | 3 | | | III-2 | Zbtb18 | 10472 | 4-May-15 |
| 23385 | 2 | 3 | | | III-2 | Zbtb22 | 9278 | 4-May-15 |
| 23389 | 2 | 3 | | | III-2 | Zbtb3 | 79842 | 4-May-15 |
| 23392 | 2 | 3 | | | III-2 | Zbtb34 | 403341 | 1-Jun-15 |
| 23397 | 2 | 3 | | | III-2 | Zbtb40 | 9923 | 4-May-15 |
| 23403 | 2 | 3 | | | III-2 | Zbtb46 | 140685 | 28-May-15 |
| 23405 | 2 | 3 | | | III-2 | Zbtb49 | 166793 | 28-May-15 |
| 23406 | 2 | 3 | | | III-2 | Zbtb5 | 9925 | 4-May-15 |
| 23407 | 2 | 3 | | | III-2 | Zbtb6 | 10773 | 4-May-15 |
| 23408 | 2 | 3 | | | III-2 | Zbtb7a | 51341 | 4-May-15 |
| 23415 | 2 | 3 | | | III-2 | Zbtbd6 | | |
| 23417 | 2 | 3 | | | III-2 | Zc2hc1b | 153918 | 4-May-15 |
| 23420 | 2 | 3 | | | III-2 | Zc3h11a | 9877 | 4-May-15 |
| 23426 | 2 | 3 | | | III-2 | Zc3h14 | 79882 | 4-May-15 |
| 23433 | 2 | 3 | | | III-2 | Zc3h7b | 23264 | 4-May-15 |
| 23436 | 2 | 3 | | | III-2 | Zc3hav1l | 92092 | 21-May-15 |
| 23437 | 2 | 3 | | | III-2 | Zc3hc1 | 51530 | 4-May-15 |
| 23438 | 2 | 3 | | | III-2 | Zc4h2 | 55906 | 4-May-15 |
| 23440 | 2 | 3 | | | III-2 | Zcchc11 | 23318 | 4-May-15 |
| 23448 | 2 | 3 | | | III-2 | Zcchc24 | 219654 | 4-May-15 |
| 23450 | 2 | 3 | | | III-2 | Zcchc4 | 29063 | 4-May-15 |
| 23451 | 2 | 3 | | | III-2 | Zcchc5 | 203430 | 4-May-15 |
| 23452 | 2 | 3 | | | III-2 | Zcchc6 | 79670 | 12-May-15 |
| 23454 | 2 | 3 | | | III-2 | Zcchc8 | 55596 | 27-May-15 |
| 23464 | 2 | 3 | | | III-2 | Zdhhc15 | 158866 | 23-May-15 |
| 23465 | 2 | 3 | | | III-2 | Zdhhc16 | 84287 | 12-May-15 |
| 23469 | 2 | 3 | | | III-2 | Zdhhc2 | 51201 | 4-May-15 |
| 23475 | 2 | 3 | | | III-2 | Zdhhc25 | | |
| 23476 | 2 | 3 | | | III-2 | Zdhhc3 | 51304 | 4-May-15 |
| 23479 | 2 | 3 | | | III-2 | Zdhhc6 | 64429 | 12-May-15 |
| 23490 | 2 | 3 | | | III-2 | Zfand2b | 130617 | 4-May-15 |
| 23491 | 2 | 3 | | | III-2 | Zfand3 | 60685 | 4-May-15 |
| 23492 | 2 | 3 | | | III-2 | Zfand4 | 93550 | 4-May-15 |
| 23494 | 2 | 3 | | | III-2 | Zfand6 | 54469 | 4-May-15 |
| 23498 | 2 | 3 | | | III-2 | Zfhx2 | 85446 | 4-May-15 |
| 23502 | 2 | 3 | | | III-2 | Zfml | 27332 | 4-May-15 |
| 23503 | 2 | 3 | | | III-2 | Zfp1 | 162239 | 12-May-15 |
| 23509 | 2 | 3 | | | III-2 | Zfp11 | | |
| 23510 | 2 | 3 | | | III-2 | Zfp110 | | |
| 23511 | 2 | 3 | | | III-2 | Zfp111 | | |
| 23515 | 2 | 3 | | | III-2 | Zfp119a | | |
| 23519 | 2 | 3 | | | III-2 | Zfp128 | 7554 | 4-May-15 |
| 23524 | 2 | 3 | | | III-2 | Zfp142 | | |
| 23526 | 2 | 3 | | | III-2 | Zfp146 | | |
| 23532 | 2 | 3 | | | III-2 | Zfp180 | | |
| 23533 | 2 | 3 | | | III-2 | Zfp182 | 7569 | 4-May-15 |
| 23538 | 2 | 3 | | | III-2 | Zfp2 | 80108 | 4-May-15 |
| 23539 | 2 | 3 | | | III-2 | Zfp202 | | |
| 23543 | 2 | 3 | | | III-2 | Zfp217 | | |
| 23544 | 2 | 3 | | | III-2 | Zfp219 | 51222 | 4-May-15 |
| 23545 | 2 | 3 | | | III-2 | Zfp229 | | |
| 23547 | 2 | 3 | | | III-2 | Zfp236 | | |
| 23548 | 2 | 3 | | | III-2 | Zfp239 | | |
| 23552 | 2 | 3 | | | III-2 | Zfp260 | 339324 | 4-May-15 |
| 23553 | 2 | 3 | | | III-2 | Zfp263 | | |
| 23556 | 2 | 3 | | | III-2 | Zfp273 | | |
| 23560 | 2 | 3 | | | III-2 | Zfp28 | 140612 | 12-May-15 |
| 23564 | 2 | 3 | | | III-2 | Zfp281 | | |
| 23565 | 2 | 3 | | | III-2 | Zfp282 | | |
| 23566 | 2 | 3 | | | III-2 | Zfp286 | | |
| 23568 | 2 | 3 | | | III-2 | Zfp292 | 23036 | 28-May-15 |
| 23570 | 2 | 3 | | | III-2 | Zfp3 | 124961 | 4-May-15 |
| 23575 | 2 | 3 | | | III-2 | Zfp318 | 24149 | 4-May-15 |
| 23577 | 2 | 3 | | | III-2 | Zfp322a | | |
| 23578 | 2 | 3 | | | III-2 | Zfp324 | | |
| 23580 | 2 | 3 | | | III-2 | Zfp329 | | |
| 23582 | 2 | 3 | | | III-2 | Zfp334 | | |
| 23602 | 2 | 3 | | | III-2 | Zfp37 | 7539 | 4-May-15 |
| 23609 | 2 | 3 | | | III-2 | Zfp386 | | |
| 23614 | 2 | 3 | | | III-2 | Zfp398 | | |
| 23616 | 2 | 3 | | | III-2 | Zfp407 | | |
| 23621 | 2 | 3 | | | III-2 | Zfp418 | | |
| 23622 | 2 | 3 | | | III-2 | Zfp42 | 132625 | 12-May-15 |
| 23623 | 2 | 3 | | | III-2 | Zfp420 | | |
| 23625 | 2 | 3 | | | III-2 | Zfp423 | 23090 | 20-May-15 |
| 23626 | 2 | 3 | | | III-2 | Zfp426 | | |
| 23627 | 2 | 3 | | | III-2 | Zfp428 | 126299 | 4-May-15 |
| 23630 | 2 | 3 | | | III-2 | Zfp438 | | |
| 23631 | 2 | 3 | | | III-2 | Zfp442 | | |
| 23633 | 2 | 3 | | | III-2 | Zfp445 | | |
| 23635 | 2 | 3 | | | III-2 | Zfp449 | | |
| 23639 | 2 | 3 | | | III-2 | Zfp456 | | |
| 23641 | 2 | 3 | | | III-2 | Zfp458 | | |
| 23644 | 2 | 3 | | | III-2 | Zfp462 | 58499 | 4-May-15 |
| 23647 | 2 | 3 | | | III-2 | Zfp473 | | |
| 23650 | 2 | 3 | | | III-2 | Zfp493 | | |
| 23653 | 2 | 3 | | | III-2 | Zfp51 | | |
| 23668 | 2 | 3 | | | III-2 | Zfp536 | | |
| 23671 | 2 | 3 | | | III-2 | Zfp551 | | |
| 23680 | 2 | 3 | | | III-2 | Zfp574 | | |
| 23687 | 2 | 3 | | | III-2 | Zfp592 | | |
| 23688 | 2 | 3 | | | III-2 | Zfp593 | | |
| 23694 | 2 | 3 | | | III-2 | Zfp600 | | |
| 23697 | 2 | 3 | | | III-2 | Zfp607 | | |
| 23703 | 2 | 3 | | | III-2 | Zfp617 | | |
| 23706 | 2 | 3 | | | III-2 | Zfp62 | 643836 | 4-May-15 |
| 23708 | 2 | 3 | | | III-2 | Zfp623 | | |
| 23716 | 2 | 3 | | | III-2 | Zfp646 | | |
| 23717 | 2 | 3 | | | III-2 | Zfp647 | 58500 | 4-May-15 |
| 23726 | 2 | 3 | | | III-2 | Zfp658 | | |
| 23727 | 2 | 3 | | | III-2 | Zfp661 | 7549 | 12-May-15 |
| 23728 | 2 | 3 | | | III-2 | Zfp663 | | |
| 23733 | 2 | 3 | | | III-2 | Zfp677 | | |
| 23735 | 2 | 3 | | | III-2 | Zfp687 | | |
| 23746 | 2 | 3 | | | III-2 | Zfp707 | | |
| 23749 | 2 | 3 | | | III-2 | Zfp710 | | |
| 23750 | 2 | 3 | | | III-2 | Zfp711 | 7552 | 4-May-15 |
| 23751 | 2 | 3 | | | III-2 | Zfp712 | | |
| 23753 | 2 | 3 | | | III-2 | Zfp719 | | |
| 23758 | 2 | 3 | | | III-2 | Zfp740 | 283337 | 4-May-15 |
| 23759 | 2 | 3 | | | III-2 | Zfp746 | | |
| 23764 | 2 | 3 | | | III-2 | Zfp759 | | |
| 23765 | 2 | 3 | | | III-2 | Zfp760 | | |
| 23766 | 2 | 3 | | | III-2 | Zfp763 | | |
| 23767 | 2 | 3 | | | III-2 | Zfp764 | | |
| 23768 | 2 | 3 | | | III-2 | Zfp768 | | |
| 23775 | 2 | 3 | | | III-2 | Zfp78 | | |
| 23778 | 2 | 3 | | | III-2 | Zfp783 | | |
| 23781 | 2 | 3 | | | III-2 | Zfp787 | | |
| 23786 | 2 | 3 | | | III-2 | Zfp800 | | |
| 23787 | 2 | 3 | | | III-2 | Zfp804a | | |
| 23788 | 2 | 3 | | | III-2 | Zfp804b | | |
| 23798 | 2 | 3 | | | III-2 | Zfp825 | | |
| 23802 | 2 | 3 | | | III-2 | Zfp839 | | |
| 23807 | 2 | 3 | | | III-2 | Zfp85os | | |
| 23808 | 2 | 3 | | | III-2 | Zfp862-ps | | |
| 23810 | 2 | 3 | | | III-2 | Zfp866 | | |
| 23811 | 2 | 3 | | | III-2 | Zfp867 | | |
| 23817 | 2 | 3 | | | III-2 | Zfp872 | | |
| 23821 | 2 | 3 | | | III-2 | Zfp879 | | |
| 23822 | 2 | 3 | | | III-2 | Zfp882 | | |
| 23832 | 2 | 3 | | | III-2 | Zfp933 | | |
| 23833 | 2 | 3 | | | III-2 | Zfp934 | | |
| 23835 | 2 | 3 | | | III-2 | Zfp936 | | |
| 23836 | 2 | 3 | | | III-2 | Zfp937 | | |
| 23838 | 2 | 3 | | | III-2 | Zfp939 | | |

Fig.22 - 57

| | | | | | | | | |
|---|---|---|---|---|---|---|---|---|
| 23840 | 2 | 3 | | | III-2 | Zfp940 | | |
| 23844 | 2 | 3 | | | III-2 | Zfp944 | | |
| 23846 | 2 | 3 | | | III-2 | Zfp946 | | |
| 23852 | 2 | 3 | | | III-2 | Zfp953 | | |
| 23855 | 2 | 3 | | | III-2 | Zfp955b | | |
| 23856 | 2 | 3 | | | III-2 | Zfp956 | | |
| 23858 | 2 | 3 | | | III-2 | Zfp958 | | |
| 23868 | 2 | 3 | | | III-2 | Zfr | 51663 | 12-May-15 |
| 23871 | 2 | 3 | | | III-2 | Zfy1 | | |
| 23872 | 2 | 3 | | | III-2 | Zfy2 | | |
| 23876 | 2 | 3 | | | III-2 | Zfyve20 | 64145 | 12-May-15 |
| 23877 | 2 | 3 | | | III-2 | Zfyve21 | 79038 | 21-May-15 |
| 23884 | 2 | 3 | | | III-2 | Zgpat | 84619 | 12-May-15 |
| 23887 | 2 | 3 | | | III-2 | Zhx2 | 22882 | 31-May-15 |
| 23890 | 2 | 3 | | | III-2 | Zic2 | 7546 | 2-Jun-15 |
| 23891 | 2 | 3 | | | III-2 | Zic3 | 7547 | 28-May-15 |
| 23893 | 2 | 3 | | | III-2 | Zic5 | 85416 | 4-May-15 |
| 23897 | 2 | 3 | | | III-2 | Zkscan1 | 7586 | 4-May-15 |
| 23903 | 2 | 3 | | | III-2 | Zkscan4 | 387032 | 20-May-15 |
| 23904 | 2 | 3 | | | III-2 | Zkscan5 | 23660 | 12-May-15 |
| 23906 | 2 | 3 | | | III-2 | Zkscan7 | 55888 | 4-May-15 |
| 23915 | 2 | 3 | | | III-2 | Zmpste24 | 10269 | 4-May-15 |
| 23918 | 2 | 3 | | | III-2 | Zmym3 | 9203 | 12-May-15 |
| 23924 | 2 | 3 | | | III-2 | Zmynd12 | 84217 | 4-May-15 |
| 23927 | 2 | 3 | | | III-2 | Zmynd8 | 23613 | 12-May-15 |
| 23928 | 2 | 3 | | | III-2 | Znf41-ps | | |
| 23929 | 2 | 3 | | | III-2 | Znf512b | 57473 | 4-May-15 |
| 23939 | 2 | 3 | | | III-2 | Znrf3 | 84133 | 23-May-15 |
| 23942 | 2 | 3 | | | III-2 | Zp2 | 7783 | 4-May-15 |
| 23952 | 2 | 3 | | | III-2 | Zranb3 | 84083 | 4-May-15 |
| 23953 | 2 | 3 | | | III-2 | Zrsr1 | 7310 | 4-May-15 |
| 23961 | 2 | 3 | | | III-2 | Zscan22 | 342945 | 4-May-15 |
| 23976 | 2 | 3 | | | III-2 | Zswim5 | 57643 | 4-May-15 |
| 23980 | 2 | 3 | | | III-2 | Zufsp | 221302 | 4-May-15 |
| 23988 | 2 | 3 | | | III-2 | Zyg11b | 79699 | 2-Jun-15 |
| 23990 | 2 | 3 | | | III-2 | Zzef1 | 23140 | 12-May-15 |
| 2 | 2 | 3 | | | III-1 | 0610007P14Rik | | |
| 5 | 2 | 3 | | | III-1 | 0610009O20Rik | | |
| 7 | 2 | 3 | | | III-1 | 0610010F05Rik | | |
| 19 | 2 | 3 | | | III-1 | 0610040J01Rik | | |
| 24 | 2 | 3 | | | III-1 | 1110002L01Rik | | |
| 25 | 2 | 3 | | | III-1 | 1110004E09Rik | | |
| 30 | 2 | 3 | | | III-1 | 1110008L16Rik | | |
| 31 | 2 | 3 | | | III-1 | 1110008P14Rik | | |
| 32 | 2 | 3 | | | III-1 | 1110012L19Rik | | |
| 35 | 2 | 3 | | | III-1 | 1110019D14Rik | | |
| 38 | 2 | 3 | | | III-1 | 1110028F11Rik | | |
| 41 | 2 | 3 | | | III-1 | 1110032F04Rik | | |
| 46 | 2 | 3 | | | III-1 | 1110038F14Rik | | |
| 49 | 2 | 3 | | | III-1 | 1110054M08Rik | | |
| 50 | 2 | 3 | | | III-1 | 1110057K04Rik | | |
| 51 | 2 | 3 | | | III-1 | 1110058L19Rik | | |
| 54 | 2 | 3 | | | III-1 | 1110065P20Rik | | |
| 60 | 2 | 3 | | | III-1 | 1200014J11Rik | | |
| 64 | 2 | 3 | | | III-1 | 1500004A13Rik | | |
| 65 | 2 | 3 | | | III-1 | 1500009C09Rik | | |
| 68 | 2 | 3 | | | III-1 | 1500011K16Rik | | |
| 69 | 2 | 3 | | | III-1 | 1500012F01Rik | | |
| 70 | 2 | 3 | | | III-1 | 1500012K07Rik | | |
| 72 | 2 | 3 | | | III-1 | 1500015L24Rik | | |
| 77 | 2 | 3 | | | III-1 | 1600002K03Rik | | |
| 79 | 2 | 3 | | | III-1 | 1600012H06Rik | | |
| 82 | 2 | 3 | | | III-1 | 1600014K23Rik | | |
| 85 | 2 | 3 | | | III-1 | 1600019K03Rik | | |
| 87 | 2 | 3 | | | III-1 | 1600023N17Rik | | |
| 88 | 2 | 3 | | | III-1 | 1600025M17Rik | | |
| 89 | 2 | 3 | | | III-1 | 1600027J07Rik | | |
| 93 | 2 | 3 | | | III-1 | 1700001C19Rik | | |
| 96 | 2 | 3 | | | III-1 | 1700001G11Rik | | |
| 99 | 2 | 3 | | | III-1 | 1700001J11Rik | | |
| 101 | 2 | 3 | | | III-1 | 1700001K23Rik | | |
| 103 | 2 | 3 | | | III-1 | 1700001L19Rik | | |
| 111 | 2 | 3 | | | III-1 | 1700003G13Rik | | |
| 128 | 2 | 3 | | | III-1 | 1700007L15Rik | | |
| 129 | 2 | 3 | | | III-1 | 1700007P06Rik | | |
| 133 | 2 | 3 | | | III-1 | 1700008K24Rik | | |
| 140 | 2 | 3 | | | III-1 | 1700010B08Rik | | |
| 143 | 2 | 3 | | | III-1 | 1700010I14Rik | | |
| 150 | 2 | 3 | | | III-1 | 1700011I03Rik | | |
| 154 | 2 | 3 | | | III-1 | 1700012B07Rik | | |
| 158 | 2 | 3 | | | III-1 | 1700012I11Rik | | |
| 164 | 2 | 3 | | | III-1 | 1700013H16Rik | | |
| 173 | 2 | 3 | | | III-1 | 1700016L04Rik | | |
| 175 | 2 | 3 | | | III-1 | 1700016P04Rik | | |
| 177 | 2 | 3 | | | III-1 | 1700017D01Rik | | |
| 187 | 2 | 3 | | | III-1 | 1700018L02Rik | | |
| 192 | 2 | 3 | | | III-1 | 1700019E08Rik | | |
| 194 | 2 | 3 | | | III-1 | 1700019G24Rik | | |
| 206 | 2 | 3 | | | III-1 | 1700020N15Rik | | |
| 215 | 2 | 3 | | | III-1 | 1700022H16Rik | | |
| 222 | 2 | 3 | | | III-1 | 1700024B18Rik | | |
| 227 | 2 | 3 | | | III-1 | 1700025B11Rik | | |
| 232 | 2 | 3 | | | III-1 | 1700025K24Rik | | |
| 236 | 2 | 3 | | | III-1 | 1700026D11Rik | | |
| 240 | 2 | 3 | | | III-1 | 1700027F09Rik | | |
| 247 | 2 | 3 | | | III-1 | 1700028I16Rik | | |
| 249 | 2 | 3 | | | III-1 | 1700028K03Rik | | |

| | | | | | | | | |
|---|---|---|---|---|---|---|---|---|
| 250 | 2 | 3 | | | III-1 | 1700028M03Rik | | |
| 251 | 2 | 3 | | | III-1 | 1700028P14Rik | | |
| 255 | 2 | 3 | | | III-1 | 1700029H14Rik | | |
| 259 | 2 | 3 | | | III-1 | 1700029M20Rik | | |
| 264 | 2 | 3 | | | III-1 | 1700030F04Rik | | |
| 271 | 2 | 3 | | | III-1 | 1700030O20Rik | | |
| 284 | 2 | 3 | | | III-1 | 1700034P13Rik | | |
| 287 | 2 | 3 | | | III-1 | 1700037H04Rik | | |
| 288 | 2 | 3 | | | III-1 | 1700039E15Rik | | |
| 292 | 2 | 3 | | | III-1 | 1700041M19Rik | | |
| 297 | 2 | 3 | | | III-1 | 1700044C05Rik | | |
| 300 | 2 | 3 | | | III-1 | 1700046C09Rik | | |
| 305 | 2 | 3 | | | III-1 | 1700047L14Rik | | |
| 328 | 2 | 3 | | | III-1 | 1700061F12Rik | | |
| 339 | 2 | 3 | | | III-1 | 1700065J18Rik | | |
| 344 | 2 | 3 | | | III-1 | 1700066M21Rik | | |
| 349 | 2 | 3 | | | III-1 | 1700067P10Rik | | |
| 355 | 2 | 3 | | | III-1 | 1700072O05Rik | | |
| 358 | 2 | 3 | | | III-1 | 1700074P13Rik | | |
| 367 | 2 | 3 | | | III-1 | 1700085C21Rik | | |
| 370 | 2 | 3 | | | III-1 | 1700088E04Rik | | |
| 371 | 2 | 3 | | | III-1 | 1700091H14Rik | | |
| 373 | 2 | 3 | | | III-1 | 1700092C10Rik | | |
| 379 | 2 | 3 | | | III-1 | 1700094J05Rik | | |
| 383 | 2 | 3 | | | III-1 | 1700096J18Rik | | |
| 385 | 2 | 3 | | | III-1 | 1700097N02Rik | | |
| 389 | 2 | 3 | | | III-1 | 1700101O22Rik | | |
| 394 | 2 | 3 | | | III-1 | 1700106J16Rik | | |
| 402 | 2 | 3 | | | III-1 | 1700110C19Rik | | |
| 407 | 2 | 3 | | | III-1 | 1700112H15Rik | | |
| 410 | 2 | 3 | | | III-1 | 1700113H08Rik | | |
| 424 | 2 | 3 | | | III-1 | 1700123O20Rik | | |
| 436 | 2 | 3 | | | III-1 | 1810007C17Rik | | |
| 446 | 2 | 3 | | | III-1 | 1810013A23Rik | | |
| 449 | 2 | 3 | | | III-1 | 1810018F18Rik | | |
| 451 | 2 | 3 | | | III-1 | 1810020O05Rik | | |
| 467 | 2 | 3 | | | III-1 | 1810055G02Rik | | |
| 468 | 2 | 3 | | | III-1 | 1810058I24Rik | | |
| 472 | 2 | 3 | | | III-1 | 1810065E05Rik | | |
| 473 | 2 | 3 | | | III-1 | 2010001E11Rik | | |
| 482 | 2 | 3 | | | III-1 | 2010106C02Rik | | |
| 484 | 2 | 3 | | | III-1 | 2010107E04Rik | | |
| 487 | 2 | 3 | | | III-1 | 2010109A12Rik | | |
| 492 | 2 | 3 | | | III-1 | 2010308F09Rik | | |
| 493 | 2 | 3 | | | III-1 | 2010310C07Rik | | |
| 502 | 2 | 3 | | | III-1 | 2210016F16Rik | | |
| 516 | 2 | 3 | | | III-1 | 2210420H20Rik | | |
| 518 | 2 | 3 | | | III-1 | 2300003K06Rik | | |
| 520 | 2 | 3 | | | III-1 | 2300009A05Rik | | |
| 524 | 2 | 3 | | | III-1 | 2310002F09Rik | | |
| 532 | 2 | 3 | | | III-1 | 2310007L24Rik | | |
| 533 | 2 | 3 | | | III-1 | 2310008N11Rik | | |
| 534 | 2 | 3 | | | III-1 | 2310009A05Rik | | |
| 544 | 2 | 3 | | | III-1 | 2310022A10Rik | | |
| 548 | 2 | 3 | | | III-1 | 2310033P09Rik | | |
| 549 | 2 | 3 | | | III-1 | 2310034C09Rik | | |
| 555 | 2 | 3 | | | III-1 | 2310039L15Rik | | |
| 558 | 2 | 3 | | | III-1 | 2310043L19Rik | | |
| 567 | 2 | 3 | | | III-1 | 2310061J03Rik | | |
| 572 | 2 | 3 | | | III-1 | 2310069B03Rik | | |
| 577 | 2 | 3 | | | III-1 | 2410003L11Rik | | |
| 587 | 2 | 3 | | | III-1 | 2410016O06Rik | | |
| 588 | 2 | 3 | | | III-1 | 2410017I17Rik | | |
| 599 | 2 | 3 | | | III-1 | 2410141K09Rik | | |
| 606 | 2 | 3 | | | III-1 | 2610001J05Rik | | |
| 610 | 2 | 3 | | | III-1 | 2610008E11Rik | | |
| 613 | 2 | 3 | | | III-1 | 2610018G03Rik | | |
| 614 | 2 | 3 | | | III-1 | 2610020C07Rik | | |
| 620 | 2 | 3 | | | III-1 | 2610034M16Rik | | |
| 622 | 2 | 3 | | | III-1 | 2610035F20Rik | | |
| 625 | 2 | 3 | | | III-1 | 2610100L16Rik | | |
| 627 | 2 | 3 | | | III-1 | 2610203C22Rik | | |
| 629 | 2 | 3 | | | III-1 | 2610207O16Rik | | |
| 631 | 2 | 3 | | | III-1 | 2610305D13Rik | | |
| 636 | 2 | 3 | | | III-1 | 2610507B11Rik | | |
| 643 | 2 | 3 | | | III-1 | 2700046A07Rik | | |
| 645 | 2 | 3 | | | III-1 | 2700049A03Rik | | |
| 648 | 2 | 3 | | | III-1 | 2700062C07Rik | | |
| 657 | 2 | 3 | | | III-1 | 2700099C18Rik | | |
| 659 | 2 | 3 | | | III-1 | 2810002D19Rik | | |
| 664 | 2 | 3 | | | III-1 | 2810013P06Rik | | |
| 666 | 2 | 3 | | | III-1 | 2810025M15Rik | | |
| 667 | 2 | 3 | | | III-1 | 2810029C07Rik | | |
| 671 | 2 | 3 | | | III-1 | 2810055G20Rik | | |
| 674 | 2 | 3 | | | III-1 | 2810404M03Rik | | |
| 677 | 2 | 3 | | | III-1 | 2810408I11Rik | | |
| 689 | 2 | 3 | | | III-1 | 2810471M01Rik | | |
| 692 | 2 | 3 | | | III-1 | 2900008C10Rik | | |
| 693 | 2 | 3 | | | III-1 | 2900009J06Rik | | |
| 698 | 2 | 3 | | | III-1 | 2900055J20Rik | | |
| 700 | 2 | 3 | | | III-1 | 2900057B20Rik | | |
| 701 | 2 | 3 | | | III-1 | 2900060B14Rik | | |
| 703 | 2 | 3 | | | III-1 | 2900079G21Rik | | |
| 706 | 2 | 3 | | | III-1 | 2900097C17Rik | | |
| 708 | 2 | 3 | | | III-1 | 3010001F23Rik | | |
| 710 | 2 | 3 | | | III-1 | 3010033K07Rik | | |
| 714 | 2 | 3 | | | III-1 | 3110007F17Rik | | |

Fig.22 - 58

| | | | | | | | | |
|---|---|---|---|---|---|---|---|---|
| 718 | 2 | 3 | | | III-1 | 3110021A11Rik | | |
| 720 | 2 | 3 | | | III-1 | 3110035E14Rik | | |
| 724 | 2 | 3 | | | III-1 | 3110043O21Rik | | |
| 725 | 2 | 3 | | | III-1 | 3110045C21Rik | | |
| 728 | 2 | 3 | | | III-1 | 3110057O12Rik | | |
| 729 | 2 | 3 | | | III-1 | 3110062M04Rik | | |
| 742 | 2 | 3 | | | III-1 | 3830406C13Rik | | |
| 744 | 2 | 3 | | | III-1 | 3830417A13Rik | | |
| 747 | 2 | 3 | | | III-1 | 4631405J19Rik | | |
| 750 | 2 | 3 | | | III-1 | 4632428C04Rik | | |
| 754 | 2 | 3 | | | III-1 | 4732456N10Rik | | |
| 756 | 2 | 3 | | | III-1 | 4732490B19Rik | | |
| 758 | 2 | 3 | | | III-1 | 4831440E17Rik | | |
| 759 | 2 | 3 | | | III-1 | 4833403I15Rik | | |
| 761 | 2 | 3 | | | III-1 | 4833412C05Rik | | |
| 765 | 2 | 3 | | | III-1 | 4833420G17Rik | | |
| 768 | 2 | 3 | | | III-1 | 4833424O15Rik | | |
| 770 | 2 | 3 | | | III-1 | 4833427G06Rik | | |
| 779 | 2 | 3 | | | III-1 | 4921508D12Rik | | |
| 786 | 2 | 3 | | | III-1 | 4921511M17Rik | | |
| 796 | 2 | 3 | | | III-1 | 4921531P14Rik | | |
| 798 | 2 | 3 | | | III-1 | 4921534H16Rik | | |
| 800 | 2 | 3 | | | III-1 | 4921539E11Rik | | |
| 802 | 2 | 3 | | | III-1 | 4922502H24Rik | | |
| 803 | 2 | 3 | | | III-1 | 4922502N22Rik | | |
| 810 | 2 | 3 | | | III-1 | 4930402K13Rik | | |
| 821 | 2 | 3 | | | III-1 | 4930406D18Rik | | |
| 825 | 2 | 3 | | | III-1 | 4930412D23Rik | | |
| 827 | 2 | 3 | | | III-1 | 4930413E15Rik | | |
| 829 | 2 | 3 | | | III-1 | 4930413G21Rik | | |
| 830 | 2 | 3 | | | III-1 | 4930413M19Rik | | |
| 838 | 2 | 3 | | | III-1 | 4930419G24Rik | | |
| 841 | 2 | 3 | | | III-1 | 4930425O10Rik | | |
| 844 | 2 | 3 | | | III-1 | 4930427A07Rik | | |
| 856 | 2 | 3 | | | III-1 | 4930430F21Rik | | |
| 870 | 2 | 3 | | | III-1 | 4930440I19Rik | | |
| 872 | 2 | 3 | | | III-1 | 4930442J19Rik | | |
| 874 | 2 | 3 | | | III-1 | 4930443O20Rik | | |
| 882 | 2 | 3 | | | III-1 | 4930447K03Rik | | |
| 888 | 2 | 3 | | | III-1 | 4930448I18Rik | | |
| 897 | 2 | 3 | | | III-1 | 4930452B06Rik | | |
| 903 | 2 | 3 | | | III-1 | 4930455B14Rik | | |
| 906 | 2 | 3 | | | III-1 | 4930455F16Rik | | |
| 910 | 2 | 3 | | | III-1 | 4930459C07Rik | | |
| 913 | 2 | 3 | | | III-1 | 4930463O16Rik | | |
| 915 | 2 | 3 | | | III-1 | 4930465M20Rik | | |
| 931 | 2 | 3 | | | III-1 | 4930474N05Rik | | |
| 936 | 2 | 3 | | | III-1 | 4930480E11Rik | | |
| 938 | 2 | 3 | | | III-1 | 4930480K15Rik | | |
| 941 | 2 | 3 | | | III-1 | 4930482G09Rik | | |
| 950 | 2 | 3 | | | III-1 | 4930488B22Rik | | |
| 959 | 2 | 3 | | | III-1 | 4930503E14Rik | | |
| 964 | 2 | 3 | | | III-1 | 4930504O13Rik | | |
| 969 | 2 | 3 | | | III-1 | 4930507D05Rik | | |
| 972 | 2 | 3 | | | III-1 | 4930509J09Rik | | |
| 984 | 2 | 3 | | | III-1 | 4930515L03Rik | | |
| 990 | 2 | 3 | | | III-1 | 4930519F16Rik | | |
| 991 | 2 | 3 | | | III-1 | 4930519F24Rik | | |
| 995 | 2 | 3 | | | III-1 | 4930520P13Rik | | |
| 1000 | 2 | 3 | | | III-1 | 4930523O13Rik | | |
| 1010 | 2 | 3 | | | III-1 | 4930526L06Rik | | |
| 1021 | 2 | 3 | | | III-1 | 4930533B01Rik | | |
| 1024 | 2 | 3 | | | III-1 | 4930539C22Rik | | |
| 1034 | 2 | 3 | | | III-1 | 4930544G11Rik | | |
| 1037 | 2 | 3 | | | III-1 | 4930545H06Rik | | |
| 1038 | 2 | 3 | | | III-1 | 4930545L23Rik | | |
| 1044 | 2 | 3 | | | III-1 | 4930548H24Rik | | |
| 1047 | 2 | 3 | | | III-1 | 4930549C01Rik | | |
| 1050 | 2 | 3 | | | III-1 | 4930550L24Rik | | |
| 1054 | 2 | 3 | | | III-1 | 4930554C24Rik | | |
| 1057 | 2 | 3 | | | III-1 | 4930556C24Rik | | |
| 1061 | 2 | 3 | | | III-1 | 4930556N09Rik | | |
| 1069 | 2 | 3 | | | III-1 | 4930562F07Rik | | |
| 1071 | 2 | 3 | | | III-1 | 4930563E18Rik | | |
| 1089 | 2 | 3 | | | III-1 | 4930571O06Rik | | |
| 1090 | 2 | 3 | | | III-1 | 4930572K03Rik | | |
| 1095 | 2 | 3 | | | III-1 | 4930578C19Rik | 79742 | 4-May-15 |
| 1100 | 2 | 3 | | | III-1 | 4930579F01Rik | | |
| 1103 | 2 | 3 | | | III-1 | 4930579K19Rik | | |
| 1105 | 2 | 3 | | | III-1 | 4930583K01Rik | | |
| 1112 | 2 | 3 | | | III-1 | 4930592I03Rik | | |
| 1122 | 2 | 3 | | | III-1 | 4931402G19Rik | | |
| 1127 | 2 | 3 | | | III-1 | 4931406H21Rik | | |
| 1139 | 2 | 3 | | | III-1 | 4931428L18Rik | | |
| 1146 | 2 | 3 | | | III-1 | 4931431F19Rik | | |
| 1148 | 2 | 3 | | | III-1 | 4931440J10Rik | | |
| 1154 | 2 | 3 | | | III-1 | 4932413F04Rik | | |
| 1161 | 2 | 3 | | | III-1 | 4932429P05Rik | | |
| 1165 | 2 | 3 | | | III-1 | 4932441J04Rik | | |
| 1185 | 2 | 3 | | | III-1 | 4933403O08Rik | | |
| 1189 | 2 | 3 | | | III-1 | 4933405D12Rik | | |
| 1194 | 2 | 3 | | | III-1 | 4933406D12Rik | | |
| 1198 | 2 | 3 | | | III-1 | 4933406J08Rik | | |
| 1199 | 2 | 3 | | | III-1 | 4933406J10Rik | | |
| 1207 | 2 | 3 | | | III-1 | 4933408B17Rik | | |
| 1208 | 2 | 3 | | | III-1 | 4933408J17Rik | | |
| 1212 | 2 | 3 | | | III-1 | 4933411E08Rik | | |

| | | | | | | | | |
|---|---|---|---|---|---|---|---|---|
| 1216 | 2 | 3 | | | III-1 | 4933411K20Rik | | |
| 1218 | 2 | 3 | | | III-1 | 4933412E24Rik | | |
| 1221 | 2 | 3 | | | III-1 | 4933413J09Rik | | |
| 1224 | 2 | 3 | | | III-1 | 4933416C03Rik | | |
| 1227 | 2 | 3 | | | III-1 | 4933416M06Rik | | |
| 1231 | 2 | 3 | | | III-1 | 4933417E11Rik | | |
| 1232 | 2 | 3 | | | III-1 | 4933417G07Rik | | |
| 1233 | 2 | 3 | | | III-1 | 4933417O13Rik | | |
| 1236 | 2 | 3 | | | III-1 | 4933422A05Rik | | |
| 1238 | 2 | 3 | | | III-1 | 4933424G05Rik | | |
| 1249 | 2 | 3 | | | III-1 | 4933428C19Rik | | |
| 1253 | 2 | 3 | | | III-1 | 4933430H16Rik | | |
| 1258 | 2 | 3 | | | III-1 | 4933431G14Rik | | |
| 1277 | 2 | 3 | | | III-1 | 4933439K11Rik | | |
| 1278 | 2 | 3 | | | III-1 | 4933440J02Rik | | |
| 1283 | 2 | 3 | | | III-1 | 5031425F14Rik | | |
| 1290 | 2 | 3 | | | III-1 | 5033406O09Rik | | |
| 1291 | 2 | 3 | | | III-1 | 5133400J02Rik | | |
| 1298 | 2 | 3 | | | III-1 | 5430401F13Rik | | |
| 1301 | 2 | 3 | | | III-1 | 5430403N17Rik | | |
| 1311 | 2 | 3 | | | III-1 | 5430427O19Rik | | |
| 1315 | 2 | 3 | | | III-1 | 5430437J10Rik | | |
| 1316 | 2 | 3 | | | III-1 | 5430440P10Rik | | |
| 1324 | 2 | 3 | | | III-1 | 5730412P04Rik | | |
| 1326 | 2 | 3 | | | III-1 | 5730420D15Rik | | |
| 1333 | 2 | 3 | | | III-1 | 5730488B01Rik | | |
| 1335 | 2 | 3 | | | III-1 | 5730508B09Rik | | |
| 1339 | 2 | 3 | | | III-1 | 5830411N06Rik | | |
| 1340 | 2 | 3 | | | III-1 | 5830415F09Rik | | |
| 1341 | 2 | 3 | | | III-1 | 5830416I19Rik | | |
| 1347 | 2 | 3 | | | III-1 | 5830432E09Rik | | |
| 1349 | 2 | 3 | | | III-1 | 5830454E08Rik | | |
| 1350 | 2 | 3 | | | III-1 | 5830473C10Rik | | |
| 1352 | 2 | 3 | | | III-1 | 5930412G12Rik | | |
| 1353 | 2 | 3 | | | III-1 | 5930430L01Rik | | |
| 1358 | 2 | 3 | | | III-1 | 6030440G07Rik | | |
| 1363 | 2 | 3 | | | III-1 | 6030469F06Rik | | |
| 1366 | 2 | 3 | | | III-1 | 6330403A02Rik | | |
| 1367 | 2 | 3 | | | III-1 | 6330403K07Rik | | |
| 1368 | 2 | 3 | | | III-1 | 6330407A03Rik | | |
| 1384 | 2 | 3 | | | III-1 | 6430573F11Rik | | |
| 1385 | 2 | 3 | | | III-1 | 6430584L05Rik | | |
| 1391 | 2 | 3 | | | III-1 | 6720468P15Rik | | |
| 1396 | 2 | 3 | | | III-1 | 7420426K07Rik | | |
| 1403 | 2 | 3 | | | III-1 | 8030423F21Rik | | |
| 1410 | 2 | 3 | | | III-1 | 8430422H06Rik | | |
| 1415 | 2 | 3 | | | III-1 | 8430431K14Rik | | |
| 1423 | 2 | 3 | | | III-1 | 9030619P08Rik | | |
| 1427 | 2 | 3 | | | III-1 | 9130008F23Rik | | |
| 1437 | 2 | 3 | | | III-1 | 9130221F21Rik | | |
| 1441 | 2 | 3 | | | III-1 | 9130401M01Rik | | |
| 1443 | 2 | 3 | | | III-1 | 9230009I02Rik | | |
| 1450 | 2 | 3 | | | III-1 | 9230112D13Rik | | |
| 1454 | 2 | 3 | | | III-1 | 9230116N13Rik | | |
| 1455 | 2 | 3 | | | III-1 | 9330020H09Rik | | |
| 1460 | 2 | 3 | | | III-1 | 9330151L19Rik | | |
| 1461 | 2 | 3 | | | III-1 | 9330158H04Rik | | |
| 1464 | 2 | 3 | | | III-1 | 9330162012Rik | | |
| 1465 | 2 | 3 | | | III-1 | 9330162B11Rik | | |
| 1467 | 2 | 3 | | | III-1 | 9330175M20Rik | | |
| 1470 | 2 | 3 | | | III-1 | 9330182L06Rik | | |
| 1471 | 2 | 3 | | | III-1 | 9330182O14Rik | | |
| 1475 | 2 | 3 | | | III-1 | 9430014N10Rik | | |
| 1477 | 2 | 3 | | | III-1 | 9430016H08Rik | | |
| 1481 | 2 | 3 | | | III-1 | 9430021M05Rik | | |
| 1484 | 2 | 3 | | | III-1 | 9430041J12Rik | | |
| 1489 | 2 | 3 | | | III-1 | 9430091E24Rik | | |
| 1491 | 2 | 3 | | | III-1 | 9530003J23Rik | | |
| 1494 | 2 | 3 | | | III-1 | 9530027J09Rik | | |
| 1495 | 2 | 3 | | | III-1 | 9530036O11Rik | | |
| 1498 | 2 | 3 | | | III-1 | 9530053A07Rik | | |
| 1508 | 2 | 3 | | | III-1 | 9630028H03Rik | | |
| 1510 | 2 | 3 | | | III-1 | 9830107B12Rik | | |
| 1515 | 2 | 3 | | | III-1 | 9930014A18Rik | | |
| 1516 | 2 | 3 | | | III-1 | 9930021J03Rik | | |
| 1517 | 2 | 3 | | | III-1 | 9930104L06Rik | | |
| 1526 | 2 | 3 | | | III-1 | A230001M10Rik | | |
| 1528 | 2 | 3 | | | III-1 | A230020J21Rik | | |
| 1532 | 2 | 3 | | | III-1 | A230056J06Rik | | |
| 1535 | 2 | 3 | | | III-1 | A230065H16Rik | | |
| 1536 | 2 | 3 | | | III-1 | A230070E04Rik | | |
| 1537 | 2 | 3 | | | III-1 | A230072C01Rik | | |
| 1542 | 2 | 3 | | | III-1 | A230108P19Rik | | |
| 1546 | 2 | 3 | | | III-1 | A330023F24Rik | | |
| 1547 | 2 | 3 | | | III-1 | A330032B11Rik | | |
| 1550 | 2 | 3 | | | III-1 | A330040F15Rik | | |
| 1551 | 2 | 3 | | | III-1 | A330041J22Rik | | |
| 1553 | 2 | 3 | | | III-1 | A330049N07Rik | | |
| 1556 | 2 | 3 | | | III-1 | A330070K13Rik | | |
| 1557 | 2 | 3 | | | III-1 | A330074K22Rik | | |
| 1559 | 2 | 3 | | | III-1 | A330076H08Rik | | |
| 1560 | 2 | 3 | | | III-1 | A330093E20Rik | | |
| 1561 | 2 | 3 | | | III-1 | A330102I10Rik | | |
| 1565 | 2 | 3 | | | III-1 | A430035B10Rik | | |
| 1566 | 2 | 3 | | | III-1 | A430078G23Rik | | |
| 1574 | 2 | 3 | | | III-1 | A4gnt | 51146 | 4-May-15 |
| 1581 | 2 | 3 | | | III-1 | A530053G22Rik | | |

Fig.22 - 59

| | | | | | | | | |
|---|---|---|---|---|---|---|---|---|
| 1583 | 2 | 3 | | | III-1 | A530058N18Rik | | |
| 1584 | 2 | 3 | | | III-1 | A530064D06Rik | | |
| 1589 | 2 | 3 | | | III-1 | A630001G21Rik | | |
| 1596 | 2 | 3 | | | III-1 | A630023P12Rik | | |
| 1597 | 2 | 3 | | | III-1 | A630033H20Rik | | |
| 1599 | 2 | 3 | | | III-1 | A630072M18Rik | | |
| 1601 | 2 | 3 | | | III-1 | A630075F10Rik | | |
| 1604 | 2 | 3 | | | III-1 | A630089N07Rik | | |
| 1611 | 2 | 3 | | | III-1 | A730018C14Rik | | |
| 1615 | 2 | 3 | | | III-1 | A730043L09Rik | | |
| 1622 | 2 | 3 | | | III-1 | A730098P11Rik | | |
| 1625 | 2 | 3 | | | III-1 | A830018L16Rik | | |
| 1634 | 2 | 3 | | | III-1 | A930003O13Rik | | |
| 1637 | 2 | 3 | | | III-1 | A930006I01Rik | | |
| 1644 | 2 | 3 | | | III-1 | A930013F10Rik | | |
| 1649 | 2 | 3 | | | III-1 | A930019D19Rik | | |
| 1651 | 2 | 3 | | | III-1 | A930041C12Rik | | |
| 1652 | 2 | 3 | | | III-1 | AA387883 | | |
| 1656 | 2 | 3 | | | III-1 | AA415398 | | |
| 1667 | 2 | 3 | | | III-1 | AA987161 | | |
| 1668 | 2 | 3 | | | III-1 | Aaas | 8086 | 12-May-15 |
| 1669 | 2 | 3 | | | III-1 | Aacs | 65985 | 12-May-15 |
| 1675 | 2 | 3 | | | III-1 | Aagab | 79719 | 17-May-15 |
| 1682 | 2 | 3 | | | III-1 | Aars | 16 | 31-May-15 |
| 1683 | 2 | 3 | | | III-1 | Aars2 | 57505 | 4-May-15 |
| 1688 | 2 | 3 | | | III-1 | Aatf | 26574 | 12-May-15 |
| 1689 | 2 | 3 | | | III-1 | Aatk | 9625 | 4-May-15 |
| 1694 | 2 | 3 | | | III-1 | Abca12 | 26154 | 23-May-15 |
| 1701 | 2 | 3 | | | III-1 | Abca3 | 21 | 12-May-15 |
| 1705 | 2 | 3 | | | III-1 | Abca7 | 10347 | 12-May-15 |
| 1707 | 2 | 3 | | | III-1 | Abca8b | | |
| 1710 | 2 | 3 | | | III-1 | Abcb11 | 8647 | 23-May-15 |
| 1715 | 2 | 3 | | | III-1 | Abcb6 | 10058 | 12-May-15 |
| 1716 | 2 | 3 | | | III-1 | Abcb7 | 22 | 23-May-15 |
| 1719 | 2 | 3 | | | III-1 | Abcc1 | 4363 | 12-May-15 |
| 1721 | 2 | 3 | | | III-1 | Abcc12 | 94160 | 4-May-15 |
| 1725 | 2 | 3 | | | III-1 | Abcc5 | 10057 | 21-May-15 |
| 1729 | 2 | 3 | | | III-1 | Abcd1 | 215 | 7-Jun-15 |
| 1731 | 2 | 3 | | | III-1 | Abcd3 | 5825 | 7-Jun-15 |
| 1732 | 2 | 3 | | | III-1 | Abcd4 | 5826 | 23-May-15 |
| 1733 | 2 | 3 | | | III-1 | Abce1 | 6059 | 21-May-15 |
| 1738 | 2 | 3 | | | III-1 | Abcg2 | 9429 | 24-May-15 |
| 1739 | 2 | 3 | | | III-1 | Abcg3 | | |
| 1742 | 2 | 3 | | | III-1 | Abcg8 | 64241 | 23-May-15 |
| 1744 | 2 | 3 | | | III-1 | Abhd10 | 55347 | 4-May-15 |
| 1749 | 2 | 3 | | | III-1 | Abhd13 | 84945 | 4-May-15 |
| 1752 | 2 | 3 | | | III-1 | Abhd15 | 116236 | 4-May-15 |
| 1753 | 2 | 3 | | | III-1 | Abhd16a | 7920 | 4-May-15 |
| 1761 | 2 | 3 | | | III-1 | Abhd5 | 51099 | 12-May-15 |
| 1762 | 2 | 3 | | | III-1 | Abhd6 | 57406 | 17-May-15 |
| 1764 | 2 | 3 | | | III-1 | Abi1 | 10006 | 12-May-15 |
| 1766 | 2 | 3 | | | III-1 | Abi3 | 51225 | 4-May-15 |
| 1767 | 2 | 3 | | | III-1 | Abi3bp | 25890 | 4-May-15 |
| 1770 | 2 | 3 | | | III-1 | Ablim1 | 3983 | 12-May-15 |
| 1774 | 2 | 3 | | | III-1 | Abr | 29 | 4-May-15 |
| 1777 | 2 | 3 | | | III-1 | Abt1 | 29777 | 4-May-15 |
| 1779 | 2 | 3 | | | III-1 | Abtb2 | 25841 | 4-May-15 |
| 1782 | 2 | 3 | | | III-1 | Acaa2 | 10449 | 4-May-15 |
| 1785 | 2 | 3 | | | III-1 | Acad10 | 80724 | 4-May-15 |
| 1786 | 2 | 3 | | | III-1 | Acad11 | 84129 | 4-May-15 |
| 1787 | 2 | 3 | | | III-1 | Acad12 | | |
| 1788 | 2 | 3 | | | III-1 | Acad8 | 27034 | 12-May-15 |
| 1791 | 2 | 3 | | | III-1 | Acadm | 34 | 23-May-15 |
| 1792 | 2 | 3 | | | III-1 | Acads | 35 | 23-May-15 |
| 1793 | 2 | 3 | | | III-1 | Acadsb | 36 | 12-May-15 |
| 1794 | 2 | 3 | | | III-1 | Acadvl | 37 | 23-May-15 |
| 1801 | 2 | 3 | | | III-1 | Acat3 | | |
| 1802 | 2 | 3 | | | III-1 | Acbd3 | 64746 | 4-May-15 |
| 1807 | 2 | 3 | | | III-1 | Accs | 84680 | 20-May-15 |
| 1808 | 2 | 3 | | | III-1 | Accsl | 390110 | 12-May-15 |
| 1815 | 2 | 3 | | | III-1 | Acer3 | 55331 | 12-May-15 |
| 1827 | 2 | 3 | | | III-1 | Aco1 | 48 | 31-May-15 |
| 1830 | 2 | 3 | | | III-1 | Acot10 | | |
| 1833 | 2 | 3 | | | III-1 | Acot13 | 55856 | 24-May-15 |
| 1837 | 2 | 3 | | | III-1 | Acot5 | | |
| 1839 | 2 | 3 | | | III-1 | Acot7 | 11332 | 4-May-15 |
| 1840 | 2 | 3 | | | III-1 | Acot8 | 10005 | 12-May-15 |
| 1841 | 2 | 3 | | | III-1 | Acot9 | 23597 | 4-May-15 |
| 1842 | 2 | 3 | | | III-1 | Acox1 | 51 | 12-May-15 |
| 1845 | 2 | 3 | | | III-1 | Acoxl | 55289 | 4-May-15 |
| 1847 | 2 | 3 | | | III-1 | Acp2 | 53 | 12-May-15 |
| 1854 | 2 | 3 | | | III-1 | Acrv1 | 56 | 4-May-15 |
| 1856 | 2 | 3 | | | III-1 | Acsbg2 | 81616 | 4-May-15 |
| 1858 | 2 | 3 | | | III-1 | Acsf3 | 197322 | 4-May-15 |
| 1862 | 2 | 3 | | | III-1 | Acsl5 | 51703 | 4-May-15 |
| 1864 | 2 | 3 | | | III-1 | Acsm1 | 116285 | 4-May-15 |
| 1887 | 2 | 3 | | | III-1 | Actn1 | 87 | 14-May-15 |
| 1898 | 2 | 3 | | | III-1 | Actr6 | 64431 | 4-May-15 |
| 1899 | 2 | 3 | | | III-1 | Actr8 | 93973 | 4-May-15 |
| 1900 | 2 | 3 | | | III-1 | Actrt1 | 139741 | 28-May-15 |
| 1905 | 2 | 3 | | | III-1 | Acvr1c | 130399 | 4-May-15 |
| 1906 | 2 | 3 | | | III-1 | Acvr2a | 92 | 4-May-15 |
| 1909 | 2 | 3 | | | III-1 | Acy1 | 95 | 4-May-15 |
| 1910 | 2 | 3 | | | III-1 | Acy3 | 91703 | 12-May-15 |
| 1914 | 2 | 3 | | | III-1 | Adad1 | 132612 | 4-May-15 |
| 1921 | 2 | 3 | | | III-1 | Adam17 | 6868 | 24-May-15 |
| 1925 | 2 | 3 | | | III-1 | Adam1b | 100420505 | 4-May-15 |

| | | | | | | | | |
|---|---|---|---|---|---|---|---|---|
| 1936 | 2 | 3 | | | III-1 | Adam29 | 11086 | 4-May-15 |
| 1944 | 2 | 3 | | | III-1 | Adam5 | 255926 | 4-May-15 |
| 1952 | 2 | 3 | | | III-1 | Adamts10 | 81794 | 23-May-15 |
| 1957 | 2 | 3 | | | III-1 | Adamts16 | 170690 | 12-May-15 |
| 1968 | 2 | 3 | | | III-1 | Adamts8 | 11095 | 4-May-15 |
| 1972 | 2 | 3 | | | III-1 | Adamtsl3 | 57188 | 4-May-15 |
| 1974 | 2 | 3 | | | III-1 | Adamtsl5 | 339366 | 4-May-15 |
| 1975 | 2 | 3 | | | III-1 | Adap1 | 11033 | 4-May-15 |
| 1979 | 2 | 3 | | | III-1 | Adarb2 | 105 | 4-May-15 |
| 1982 | 2 | 3 | | | III-1 | Adat3 | 113179 | 4-May-15 |
| 1984 | 2 | 3 | | | III-1 | Adck1 | 57143 | 4-May-15 |
| 1987 | 2 | 3 | | | III-1 | Adck4 | 79934 | 23-May-15 |
| 1989 | 2 | 3 | | | III-1 | Adcy1 | 107 | 4-May-15 |
| 1992 | 2 | 3 | | | III-1 | Adcy3 | 109 | 7-Jun-15 |
| 1993 | 2 | 3 | | | III-1 | Adcy4 | 196883 | 4-May-15 |
| 1994 | 2 | 3 | | | III-1 | Adcy5 | 111 | 23-May-15 |
| 1996 | 2 | 3 | | | III-1 | Adcy7 | 113 | 4-May-15 |
| 1998 | 2 | 3 | | | III-1 | Adcy9 | 115 | 24-May-15 |
| 2001 | 2 | 3 | | | III-1 | Add1 | 118 | 17-May-15 |
| 2006 | 2 | 3 | | | III-1 | Adh4 | 127 | 7-Jun-15 |
| 2012 | 2 | 3 | | | III-1 | Adi1 | 55256 | 12-May-15 |
| 2019 | 2 | 3 | | | III-1 | Adm2 | 79924 | 17-May-15 |
| 2022 | 2 | 3 | | | III-1 | Ado | 84890 | 4-May-15 |
| 2024 | 2 | 3 | | | III-1 | Adora2a | 135 | 12-May-15 |
| 2027 | 2 | 3 | | | III-1 | Adpgk | 83440 | 14-May-15 |
| 2034 | 2 | 3 | | | III-1 | Adra1d | 146 | 4-May-15 |
| 2035 | 2 | 3 | | | III-1 | Adra2a | 150 | 4-May-15 |
| 2036 | 2 | 3 | | | III-1 | Adra2b | 151 | 4-May-15 |
| 2038 | 2 | 3 | | | III-1 | Adrb1 | 153 | 24-May-15 |
| 2043 | 2 | 3 | | | III-1 | Adrm1 | 11047 | 4-May-15 |
| 2044 | 2 | 3 | | | III-1 | Adsl | 158 | 12-May-15 |
| 2048 | 2 | 3 | | | III-1 | Aebp1 | 165 | 4-May-15 |
| 2049 | 2 | 3 | | | III-1 | Aebp2 | 121536 | 17-May-15 |
| 2051 | 2 | 3 | | | III-1 | Aes | 166 | 12-May-15 |
| 2064 | 2 | 3 | | | III-1 | Afap1l2 | 84632 | 4-May-15 |
| 2065 | 2 | 3 | | | III-1 | Aff1 | 4299 | 12-May-15 |
| 2068 | 2 | 3 | | | III-1 | Aff4 | 27125 | 22-May-15 |
| 2073 | 2 | 3 | | | III-1 | Afp | 174 | 7-Jun-15 |
| 2077 | 2 | 3 | | | III-1 | Agap2 | 116986 | 4-May-15 |
| 2078 | 2 | 3 | | | III-1 | Agap3 | 116988 | 12-May-15 |
| 2081 | 2 | 3 | | | III-1 | Agbl3 | 340351 | 4-May-15 |
| 2082 | 2 | 3 | | | III-1 | Agbl4 | 84871 | 4-May-15 |
| 2084 | 2 | 3 | | | III-1 | Ager | 177 | 17-May-15 |
| 2087 | 2 | 3 | | | III-1 | Aggf1 | 55109 | 4-May-15 |
| 2090 | 2 | 3 | | | III-1 | Agmat | 79814 | 4-May-15 |
| 2092 | 2 | 3 | | | III-1 | Ago1 | 26523 | 4-May-15 |
| 2096 | 2 | 3 | | | III-1 | Agpat1 | 10554 | 4-May-15 |
| 2103 | 2 | 3 | | | III-1 | Agps | 8540 | 4-May-15 |
| 2106 | 2 | 3 | | | III-1 | Agrn | 375790 | 12-May-15 |
| 2107 | 2 | 3 | | | III-1 | Agrp | 181 | 4-May-15 |
| 2109 | 2 | 3 | | | III-1 | Agtpbp1 | 23287 | 23-May-15 |
| 2110 | 2 | 3 | | | III-1 | Agtr1a | | |
| 2111 | 2 | 3 | | | III-1 | Agtr1b | 185 | 24-May-15 |
| 2117 | 2 | 3 | | | III-1 | Ahcy | 191 | 12-May-15 |
| 2118 | 2 | 3 | | | III-1 | Ahcyl1 | 10768 | 3-May-15 |
| 2120 | 2 | 3 | | | III-1 | Ahdc1 | 27245 | 4-May-15 |
| 2122 | 2 | 3 | | | III-1 | Ahnak | 79026 | 4-May-15 |
| 2123 | 2 | 3 | | | III-1 | Ahr | 196 | 17-May-15 |
| 2128 | 2 | 3 | | | III-1 | AI115009 | | |
| 2130 | 2 | 3 | | | III-1 | AI182371 | | |
| 2131 | 2 | 3 | | | III-1 | AI197445 | | |
| 2138 | 2 | 3 | | | III-1 | AI429214 | | |
| 2139 | 2 | 3 | | | III-1 | AI450353 | | |
| 2140 | 2 | 3 | | | III-1 | AI462493 | | |
| 2143 | 2 | 3 | | | III-1 | AI467606 | | |
| 2157 | 2 | 3 | | | III-1 | AI846148 | | |
| 2159 | 2 | 3 | | | III-1 | AI848285 | | |
| 2167 | 2 | 3 | | | III-1 | Aifm1 | 9131 | 19-May-15 |
| 2169 | 2 | 3 | | | III-1 | Aifm3 | 150209 | 4-May-15 |
| 2173 | 2 | 3 | | | III-1 | Aim2 | 9447 | 17-May-15 |
| 2179 | 2 | 3 | | | III-1 | Airn | 100271873 | 12-May-15 |
| 2182 | 2 | 3 | | | III-1 | AK010878 | | |
| 2188 | 2 | 3 | | | III-1 | Ak5 | 26289 | 4-May-15 |
| 2189 | 2 | 3 | | | III-1 | Ak6 | 102157402 | 4-May-15 |
| 2190 | 2 | 3 | | | III-1 | Ak7 | 122481 | 4-May-15 |
| 2191 | 2 | 3 | | | III-1 | Ak8 | 158067 | 4-May-15 |
| 2195 | 2 | 3 | | | III-1 | Akap12 | 9590 | 4-May-15 |
| 2196 | 2 | 3 | | | III-1 | Akap13 | 11214 | 26-May-15 |
| 2197 | 2 | 3 | | | III-1 | Akap14 | 158798 | 4-May-15 |
| 2202 | 2 | 3 | | | III-1 | Akap5 | 9495 | 21-May-15 |
| 2204 | 2 | 3 | | | III-1 | Akap7 | 9465 | 4-May-15 |
| 2205 | 2 | 3 | | | III-1 | Akap8 | 10270 | 4-May-15 |
| 2207 | 2 | 3 | | | III-1 | Akap9 | 10142 | 29-May-15 |
| 2208 | 2 | 3 | | | III-1 | Akip1 | 56672 | 4-May-15 |
| 2209 | 2 | 3 | | | III-1 | Akirin1 | 79647 | 4-May-15 |
| 2214 | 2 | 3 | | | III-1 | Akr1a1 | 10327 | 12-May-15 |
| 2224 | 2 | 3 | | | III-1 | Akr1c20 | | |
| 2237 | 2 | 3 | | | III-1 | Alas1 | 211 | 12-May-15 |
| 2241 | 2 | 3 | | | III-1 | Aldh16a1 | 126133 | 23-May-15 |
| 2248 | 2 | 3 | | | III-1 | Aldh1l1 | 10840 | 23-May-15 |
| 2252 | 2 | 3 | | | III-1 | Aldh3a2 | 224 | 23-May-15 |
| 2258 | 2 | 3 | | | III-1 | Aldh7a1 | 501 | 23-May-15 |
| 2266 | 2 | 3 | | | III-1 | Alg1 | 56052 | 23-May-15 |
| 2276 | 2 | 3 | | | III-1 | Alg8 | 79053 | 23-May-15 |
| 2277 | 2 | 3 | | | III-1 | Alg9 | 79796 | 23-May-15 |
| 2281 | 2 | 3 | | | III-1 | Alkbh3 | 221120 | 4-May-15 |

Fig.22 - 60

| | | | | | | | | |
|---|---|---|---|---|---|---|---|---|
| 2286 | 2 | 3 | | | III-1 | Alkbh8 | 91801 | 4-May-15 |
| 2298 | 2 | 3 | | | III-1 | Alpi | 248 | 4-May-15 |
| 2299 | 2 | 3 | | | III-1 | Alpk1 | 80216 | 28-May-15 |
| 2303 | 2 | 3 | | | III-1 | Alppl2 | 251 | 4-May-15 |
| 2304 | 2 | 3 | | | III-1 | Als2 | 57679 | 23-May-15 |
| 2306 | 2 | 3 | | | III-1 | Als2cr11 | 151254 | 4-May-15 |
| 2312 | 2 | 3 | | | III-1 | Alyref2 | | |
| 2323 | 2 | 3 | | | III-1 | Amer2 | 219287 | 4-May-15 |
| 2327 | 2 | 3 | | | III-1 | Amhr2 | 269 | 12-May-15 |
| 2332 | 2 | 3 | | | III-1 | Ammecr1 | 9949 | 12-May-15 |
| 2337 | 2 | 3 | | | III-1 | Amotl1 | 154810 | 4-May-15 |
| 2338 | 2 | 3 | | | III-1 | Amotl2 | 51421 | 4-May-15 |
| 2341 | 2 | 3 | | | III-1 | Ampd3 | 272 | 12-May-15 |
| 2342 | 2 | 3 | | | III-1 | Amph | 273 | 12-May-15 |
| 2350 | 2 | 3 | | | III-1 | Amz2 | 51321 | 23-May-15 |
| 2355 | 2 | 3 | | | III-1 | Anapc15 | 25906 | 4-May-15 |
| 2356 | 2 | 3 | | | III-1 | Anapc16 | 119504 | 31-May-15 |
| 2370 | 2 | 3 | | | III-1 | Angpt2 | 285 | 24-May-15 |
| 2371 | 2 | 3 | | | III-1 | Angpt4 | 51378 | 4-May-15 |
| 2382 | 2 | 3 | | | III-1 | Ankar | 150709 | 4-May-15 |
| 2388 | 2 | 3 | | | III-1 | Ankib1 | 54467 | 4-May-15 |
| 2394 | 2 | 3 | | | III-1 | Ankra2 | 57763 | 4-May-15 |
| 2396 | 2 | 3 | | | III-1 | Ankrd10 | 55608 | 12-May-15 |
| 2398 | 2 | 3 | | | III-1 | Ankrd12 | 23253 | 21-May-15 |
| 2399 | 2 | 3 | | | III-1 | Ankrd13a | 88455 | 12-May-15 |
| 2401 | 2 | 3 | | | III-1 | Ankrd13c | 81573 | 4-May-15 |
| 2403 | 2 | 3 | | | III-1 | Ankrd16 | 54522 | 4-May-15 |
| 2406 | 2 | 3 | | | III-1 | Ankrd22 | 118932 | 4-May-15 |
| 2409 | 2 | 3 | | | III-1 | Ankrd26 | 22852 | 4-May-15 |
| 2411 | 2 | 3 | | | III-1 | Ankrd28 | 23243 | 4-May-15 |
| 2416 | 2 | 3 | | | III-1 | Ankrd34a | 284615 | 4-May-15 |
| 2420 | 2 | 3 | | | III-1 | Ankrd36 | 375248 | 16-May-15 |
| 2425 | 2 | 3 | | | III-1 | Ankrd44 | 91526 | 4-May-15 |
| 2427 | 2 | 3 | | | III-1 | Ankrd46 | 157567 | 4-May-15 |
| 2431 | 2 | 3 | | | III-1 | Ankrd53 | 79998 | 4-May-15 |
| 2439 | 2 | 3 | | | III-1 | Ankrd7 | 56311 | 4-May-15 |
| 2441 | 2 | 3 | | | III-1 | Anks1 | 23294 | 21-May-15 |
| 2445 | 2 | 3 | | | III-1 | Anks6 | 203286 | 4-May-15 |
| 2450 | 2 | 3 | | | III-1 | Ano10 | 55129 | 4-May-15 |
| 2459 | 2 | 3 | | | III-1 | Anp32a | 8125 | 2-Jun-15 |
| 2464 | 2 | 3 | | | III-1 | Antxr2 | 118429 | 23-May-15 |
| 2467 | 2 | 3 | | | III-1 | Anxa10 | 11199 | 4-May-15 |
| 2473 | 2 | 3 | | | III-1 | Anxa5 | 308 | 28-May-15 |
| 2474 | 2 | 3 | | | III-1 | Anxa6 | 309 | 4-May-15 |
| 2480 | 2 | 3 | | | III-1 | Aoc2 | 314 | 21-May-15 |
| 2483 | 2 | 3 | | | III-1 | Aox2 | 344454 | 12-May-15 |
| 2490 | 2 | 3 | | | III-1 | Ap1m1 | 8907 | 4-May-15 |
| 2492 | 2 | 3 | | | III-1 | Ap1s1 | 1174 | 4-May-15 |
| 2502 | 2 | 3 | | | III-1 | Ap3d1 | 8943 | 4-May-15 |
| 2505 | 2 | 3 | | | III-1 | Ap3s1 | 1176 | 4-May-15 |
| 2512 | 2 | 3 | | | III-1 | Ap5m1 | 55745 | 4-May-15 |
| 2516 | 2 | 3 | | | III-1 | Apba1 | 320 | 4-May-15 |
| 2518 | 2 | 3 | | | III-1 | Apba3 | 9546 | 4-May-15 |
| 2521 | 2 | 3 | | | III-1 | Apbb2 | 323 | 23-May-15 |
| 2523 | 2 | 3 | | | III-1 | Apc | 324 | 7-Jun-15 |
| 2524 | 2 | 3 | | | III-1 | Apc2 | 10297 | 7-Jun-15 |
| 2526 | 2 | 3 | | | III-1 | Apcs | 325 | 4-May-15 |
| 2533 | 2 | 3 | | | III-1 | Aph1c | | |
| 2536 | 2 | 3 | | | III-1 | Apitd1 | 378708 | 4-May-15 |
| 2541 | 2 | 3 | | | III-1 | Aplp2 | 334 | 12-May-15 |
| 2552 | 2 | 3 | | | III-1 | Apobec4 | 403314 | 4-May-15 |
| 2568 | 2 | 3 | | | III-1 | Apol7b | | |
| 2576 | 2 | 3 | | | III-1 | Apom | 55937 | 17-May-15 |
| 2581 | 2 | 3 | | | III-1 | Apopt1 | 84334 | 4-May-15 |
| 2583 | 2 | 3 | | | III-1 | Appbp2 | 10513 | 4-May-15 |
| 2586 | 2 | 3 | | | III-1 | Aprt | 353 | 23-May-15 |
| 2587 | 2 | 3 | | | III-1 | Aptx | 54840 | 23-May-15 |
| 2590 | 2 | 3 | | | III-1 | Aqp12 | 375318 | 4-May-15 |
| 2599 | 2 | 3 | | | III-1 | Aqr | 9716 | 4-May-15 |
| 2604 | 2 | 3 | | | III-1 | Arap3 | 64411 | 4-May-15 |
| 2605 | 2 | 3 | | | III-1 | Arc | 23237 | 7-Jun-15 |
| 2606 | 2 | 3 | | | III-1 | Arcn1 | 372 | 4-May-15 |
| 2620 | 2 | 3 | | | III-1 | Arfip1 | 27236 | 4-May-15 |
| 2634 | 2 | 3 | | | III-1 | Arhgap19 | 84986 | 4-May-15 |
| 2635 | 2 | 3 | | | III-1 | Arhgap20 | 57569 | 4-May-15 |
| 2637 | 2 | 3 | | | III-1 | Arhgap21 | 57584 | 4-May-15 |
| 2639 | 2 | 3 | | | III-1 | Arhgap23 | 57636 | 4-May-15 |
| 2646 | 2 | 3 | | | III-1 | Arhgap29 | 9411 | 24-May-15 |
| 2649 | 2 | 3 | | | III-1 | Arhgap32 | 9743 | 4-May-15 |
| 2650 | 2 | 3 | | | III-1 | Arhgap33 | 115703 | 4-May-15 |
| 2651 | 2 | 3 | | | III-1 | Arhgap33os | | |
| 2653 | 2 | 3 | | | III-1 | Arhgap36 | 158763 | 12-May-15 |
| 2655 | 2 | 3 | | | III-1 | Arhgap4 | 393 | 4-May-15 |
| 2657 | 2 | 3 | | | III-1 | Arhgap42 | 143872 | 4-May-15 |
| 2658 | 2 | 3 | | | III-1 | Arhgap44 | 9912 | 4-May-15 |
| 2660 | 2 | 3 | | | III-1 | Arhgap6 | 395 | 4-May-15 |
| 2663 | 2 | 3 | | | III-1 | Arhgdia | 396 | 12-May-15 |
| 2666 | 2 | 3 | | | III-1 | Arhgef1 | 9138 | 31-May-15 |
| 2668 | 2 | 3 | | | III-1 | Arhgef10l | 55160 | 4-May-15 |
| 2669 | 2 | 3 | | | III-1 | Arhgef11 | 9826 | 4-May-15 |
| 2674 | 2 | 3 | | | III-1 | Arhgef18 | 23370 | 4-May-15 |
| 2679 | 2 | 3 | | | III-1 | Arhgef28 | 64283 | 4-May-15 |
| 2683 | 2 | 3 | | | III-1 | Arhgef38 | 54848 | 4-May-15 |
| 2686 | 2 | 3 | | | III-1 | Arhgef40 | 55701 | 4-May-15 |
| 2688 | 2 | 3 | | | III-1 | Arhgef6 | 9459 | 23-May-15 |
| 2694 | 2 | 3 | | | III-1 | Arid3a | 1820 | 28-May-15 |
| 2696 | 2 | 3 | | | III-1 | Arid3c | 138715 | 4-May-15 |
| 2698 | 2 | 3 | | | III-1 | Arid4b | 51742 | 4-May-15 |
| 2709 | 2 | 3 | | | III-1 | Arl14ep | 120534 | 4-May-15 |
| 2710 | 2 | 3 | | | III-1 | Arl14epl | 644100 | 4-May-15 |
| 2714 | 2 | 3 | | | III-1 | Arl2bp | 23568 | 4-May-15 |
| 2716 | 2 | 3 | | | III-1 | Arl4a | 10124 | 4-May-15 |
| 2719 | 2 | 3 | | | III-1 | Arl5a | 26225 | 4-May-15 |
| 2724 | 2 | 3 | | | III-1 | Arl6ip4 | 51329 | 12-May-15 |
| 2727 | 2 | 3 | | | III-1 | Arl8a | 127829 | 4-May-15 |
| 2733 | 2 | 3 | | | III-1 | Armc2 | 84071 | 4-May-15 |
| 2741 | 2 | 3 | | | III-1 | Armcx1 | 51309 | 12-May-15 |
| 2749 | 2 | 3 | | | III-1 | Arntl | 406 | 12-May-15 |
| 2751 | 2 | 3 | | | III-1 | Arpc1a | 10552 | 4-May-15 |
| 2753 | 2 | 3 | | | III-1 | Arpc2 | 10109 | 31-May-15 |
| 2758 | 2 | 3 | | | III-1 | Arpp19 | 10776 | 12-May-15 |
| 2759 | 2 | 3 | | | III-1 | Arpp21 | 10777 | 4-May-15 |
| 2763 | 2 | 3 | | | III-1 | Arrdc1 | 92714 | 4-May-15 |
| 2768 | 2 | 3 | | | III-1 | Arsa | 410 | 23-May-15 |
| 2769 | 2 | 3 | | | III-1 | Arsb | 411 | 13-Jun-15 |
| 2770 | 2 | 3 | | | III-1 | Arsg | 22901 | 20-May-15 |
| 2776 | 2 | 3 | | | III-1 | Art2b | | |
| 2782 | 2 | 3 | | | III-1 | Arvcf | 421 | 12-May-15 |
| 2783 | 2 | 3 | | | III-1 | Arx | 170302 | 23-May-15 |
| 2786 | 2 | 3 | | | III-1 | As3mt | 57412 | 4-May-15 |
| 2787 | 2 | 3 | | | III-1 | Asah1 | 427 | 12-May-15 |
| 2790 | 2 | 3 | | | III-1 | Asap2 | 8853 | 12-May-15 |
| 2792 | 2 | 3 | | | III-1 | Asb1 | 51665 | 4-May-15 |
| 2811 | 2 | 3 | | | III-1 | Ascc1 | 51008 | 12-May-15 |
| 2812 | 2 | 3 | | | III-1 | Ascc2 | 84164 | 4-May-15 |
| 2813 | 2 | 3 | | | III-1 | Ascc3 | 10973 | 4-May-15 |
| 2815 | 2 | 3 | | | III-1 | Ascl2 | 430 | 4-May-15 |
| 2818 | 2 | 3 | | | III-1 | Ascl5 | 647219 | 4-May-15 |
| 2822 | 2 | 3 | | | III-1 | Asgr2 | 433 | 12-May-15 |
| 2824 | 2 | 3 | | | III-1 | Ash2l | 9070 | 4-May-15 |
| 2826 | 2 | 3 | | | III-1 | Asic2 | 40 | 12-May-15 |
| 2827 | 2 | 3 | | | III-1 | Asic3 | 9311 | 4-May-15 |
| 2828 | 2 | 3 | | | III-1 | Asic4 | 55515 | 12-May-15 |
| 2831 | 2 | 3 | | | III-1 | Asmt | | |
| 2836 | 2 | 3 | | | III-1 | Aspdh | 554235 | 4-May-15 |
| 2839 | 2 | 3 | | | III-1 | Asphd1 | 253982 | 4-May-15 |
| 2854 | 2 | 3 | | | III-1 | Asxl3 | 80816 | 4-May-15 |
| 2856 | 2 | 3 | | | III-1 | Atad1 | 84896 | 4-May-15 |
| 2870 | 2 | 3 | | | III-1 | Atf5 | 22809 | 21-May-15 |
| 2871 | 2 | 3 | | | III-1 | Atf6 | 22926 | 17-May-15 |
| 2873 | 2 | 3 | | | III-1 | Atf7 | 11016 | 4-May-15 |
| 2875 | 2 | 3 | | | III-1 | Atf7ip2 | 80063 | 4-May-15 |
| 2883 | 2 | 3 | | | III-1 | Atg2a | 23130 | 21-May-15 |
| 2887 | 2 | 3 | | | III-1 | Atg4b | 23192 | 23-May-15 |
| 2889 | 2 | 3 | | | III-1 | Atg4d | 84971 | 21-May-15 |
| 2893 | 2 | 3 | | | III-1 | Atg9b | 285973 | 21-May-15 |
| 2897 | 2 | 3 | | | III-1 | Atl2 | 64225 | 23-May-15 |
| 2898 | 2 | 3 | | | III-1 | Atl3 | 25923 | 4-May-15 |
| 2899 | 2 | 3 | | | III-1 | Atm | 472 | 31-May-15 |
| 2902 | 2 | 3 | | | III-1 | Atoh1 | 474 | 4-May-15 |
| 2905 | 2 | 3 | | | III-1 | Atox1 | 475 | 17-May-15 |
| 2906 | 2 | 3 | | | III-1 | Atp10a | 57194 | 21-May-15 |
| 2908 | 2 | 3 | | | III-1 | Atp10d | 57205 | 4-May-15 |
| 2913 | 2 | 3 | | | III-1 | Atp13a1 | 57130 | 4-May-15 |
| 2917 | 2 | 3 | | | III-1 | Atp13a5 | 344905 | 4-May-15 |
| 2918 | 2 | 3 | | | III-1 | Atp1a1 | 476 | 7-Jun-15 |
| 2920 | 2 | 3 | | | III-1 | Atp1a3 | 478 | 23-May-15 |
| 2921 | 2 | 3 | | | III-1 | Atp1a4 | 480 | 4-May-15 |
| 2924 | 2 | 3 | | | III-1 | Atp1b3 | 483 | 12-May-15 |
| 2925 | 2 | 3 | | | III-1 | Atp1b4 | 23439 | 4-May-15 |
| 2928 | 2 | 3 | | | III-1 | Atp2a3 | 489 | 12-May-15 |
| 2932 | 2 | 3 | | | III-1 | Atp2b4 | 493 | 17-May-15 |
| 2936 | 2 | 3 | | | III-1 | Atp4b | 496 | 4-May-15 |
| 2938 | 2 | 3 | | | III-1 | Atp5b | 506 | 12-May-15 |
| 2939 | 2 | 3 | | | III-1 | Atp5c1 | 509 | 4-May-15 |
| 2940 | 2 | 3 | | | III-1 | Atp5d | 513 | 4-May-15 |
| 2941 | 2 | 3 | | | III-1 | Atp5e | 514 | 4-May-15 |
| 2942 | 2 | 3 | | | III-1 | Atp5f1 | 515 | 12-May-15 |
| 2944 | 2 | 3 | | | III-1 | Atp5g2 | 517 | 4-May-15 |
| 2945 | 2 | 3 | | | III-1 | Atp5g3 | 518 | 4-May-15 |
| 2947 | 2 | 3 | | | III-1 | Atp5j | 522 | 12-May-15 |
| 2952 | 2 | 3 | | | III-1 | Atp5s | 27109 | 4-May-15 |
| 2955 | 2 | 3 | | | III-1 | Atp6ap1l | 92270 | 4-May-15 |
| 2957 | 2 | 3 | | | III-1 | Atp6v0a1 | 535 | 21-May-15 |
| 2960 | 2 | 3 | | | III-1 | Atp6v0b | 533 | 3-Jun-15 |
| 2967 | 2 | 3 | | | III-1 | Atp6v1a | 523 | 12-May-15 |
| 2968 | 2 | 3 | | | III-1 | Atp6v1b1 | 525 | 24-May-15 |
| 2970 | 2 | 3 | | | III-1 | Atp6v1c1 | 528 | 2-Jun-15 |
| 2971 | 2 | 3 | | | III-1 | Atp6v1c2 | 245973 | 4-May-15 |
| 2972 | 2 | 3 | | | III-1 | Atp6v1d | 51382 | 4-May-15 |
| 2973 | 2 | 3 | | | III-1 | Atp6v1e1 | 529 | 4-May-15 |
| 2974 | 2 | 3 | | | III-1 | Atp6v1e2 | 90423 | 4-May-15 |
| 2979 | 2 | 3 | | | III-1 | Atp6v1h | 51606 | 4-May-15 |
| 2980 | 2 | 3 | | | III-1 | Atp7a | 538 | 23-May-15 |
| 2981 | 2 | 3 | | | III-1 | Atp7b | 540 | 23-May-15 |
| 2982 | 2 | 3 | | | III-1 | Atp8a1 | 10396 | 12-May-15 |
| 2987 | 2 | 3 | | | III-1 | Atp8b4 | 79895 | 4-May-15 |
| 2990 | 2 | 3 | | | III-1 | Atp9b | 374868 | 12-May-15 |
| 2994 | 2 | 3 | | | III-1 | Atr | 545 | 13-Jun-15 |
| 2995 | 2 | 3 | | | III-1 | Atraid | 51374 | 4-May-15 |
| 3001 | 2 | 3 | | | III-1 | Atxn10 | 25814 | 23-May-15 |
| 3005 | 2 | 3 | | | III-1 | Atxn3 | 4287 | 7-Jun-15 |
| 3011 | 2 | 3 | | | III-1 | AU015228 | | |
| 3013 | 2 | 3 | | | III-1 | AU015836 | | |

Fig.22 - 61

| | | | | | | | | |
|---|---|---|---|---|---|---|---|---|
| 3016 | 2 | 3 | | | III-1 | AU018829 | | |
| 3022 | 2 | 3 | | | III-1 | AU022751 | | |
| 3039 | 2 | 3 | | | III-1 | Aven | 57099 | 4-May-15 |
| 3043 | 2 | 3 | | | III-1 | Avpi1 | 60370 | 21-May-15 |
| 3046 | 2 | 3 | | | III-1 | Avpr2 | 554 | 23-May-15 |
| 3048 | 2 | 3 | | | III-1 | AW046200 | | |
| 3049 | 2 | 3 | | | III-1 | AW112010 | | |
| 3060 | 2 | 3 | | | III-1 | Axin1 | 8312 | 4-May-15 |
| 3061 | 2 | 3 | | | III-1 | Axin2 | 8313 | 4-May-15 |
| 3065 | 2 | 3 | | | III-1 | AY512915 | | |
| 3069 | 2 | 3 | | | III-1 | Aym1 | | |
| 3080 | 2 | 3 | | | III-1 | B130034C11Rik | | |
| 3081 | 2 | 3 | | | III-1 | B230112J18Rik | | |
| 3084 | 2 | 3 | | | III-1 | B230206H07Rik | | |
| 3086 | 2 | 3 | | | III-1 | B230209E15Rik | | |
| 3089 | 2 | 3 | | | III-1 | B230216N24Rik | | |
| 3097 | 2 | 3 | | | III-1 | B330016D10Rik | | |
| 3098 | 2 | 3 | | | III-1 | B3galnt1 | 8706 | 14-May-15 |
| 3099 | 2 | 3 | | | III-1 | B3galnt2 | 148789 | 4-May-15 |
| 3105 | 2 | 3 | | | III-1 | B3gat1 | 27087 | 4-May-15 |
| 3106 | 2 | 3 | | | III-1 | B3gat2 | 135152 | 12-May-15 |
| 3112 | 2 | 3 | | | III-1 | B3gnt4 | 79369 | 4-May-15 |
| 3117 | 2 | 3 | | | III-1 | B3gnt9 | 84752 | 4-May-15 |
| 3119 | 2 | 3 | | | III-1 | B430010I23Rik | | |
| 3120 | 2 | 3 | | | III-1 | B430212C06Rik | | |
| 3122 | 2 | 3 | | | III-1 | B430319G15Rik | | |
| 3126 | 2 | 3 | | | III-1 | B4galnt4 | 338707 | 4-May-15 |
| 3128 | 2 | 3 | | | III-1 | B4galt2 | 8704 | 4-May-15 |
| 3129 | 2 | 3 | | | III-1 | B4galt3 | 8703 | 7-Jun-15 |
| 3131 | 2 | 3 | | | III-1 | B4galt5 | 9334 | 4-May-15 |
| 3133 | 2 | 3 | | | III-1 | B4galt7 | 11285 | 4-May-15 |
| 3136 | 2 | 3 | | | III-1 | B830017H08Rik | | |
| 3141 | 2 | 3 | | | III-1 | B930059L03Rik | | |
| 3142 | 2 | 3 | | | III-1 | B930092H01Rik | | |
| 3147 | 2 | 3 | | | III-1 | Babam1 | 29086 | 4-May-15 |
| 3149 | 2 | 3 | | | III-1 | Bace2 | 25825 | 12-May-15 |
| 3151 | 2 | 3 | | | III-1 | Bach2 | 60468 | 12-May-15 |
| 3154 | 2 | 3 | | | III-1 | Bag1 | 573 | 17-May-15 |
| 3160 | 2 | 3 | | | III-1 | Bahcc1 | 57597 | 4-May-15 |
| 3161 | 2 | 3 | | | III-1 | Bahd1 | 22893 | 4-May-15 |
| 3163 | 2 | 3 | | | III-1 | Bai2 | 576 | 4-May-15 |
| 3167 | 2 | 3 | | | III-1 | Baiap2l2 | 80115 | 4-May-15 |
| 3168 | 2 | 3 | | | III-1 | Baiap3 | 8938 | 4-May-15 |
| 3170 | 2 | 3 | | | III-1 | Bambi | 25805 | 12-May-15 |
| 3172 | 2 | 3 | | | III-1 | Banf1 | 8815 | 4-May-15 |
| 3188 | 2 | 3 | | | III-1 | Baz1b | 9031 | 4-May-15 |
| 3192 | 2 | 3 | | | III-1 | BB019430 | | |
| 3195 | 2 | 3 | | | III-1 | BB283400 | | |
| 3197 | 2 | 3 | | | III-1 | BB557941 | | |
| 3199 | 2 | 3 | | | III-1 | Bbip1 | 92482 | 4-May-15 |
| 3201 | 2 | 3 | | | III-1 | Bbs1 | 582 | 29-May-15 |
| 3204 | 2 | 3 | | | III-1 | Bbs2 | 583 | 23-May-15 |
| 3205 | 2 | 3 | | | III-1 | Bbs4 | 585 | 23-May-15 |
| 3207 | 2 | 3 | | | III-1 | Bbs7 | 55212 | 23-May-15 |
| 3208 | 2 | 3 | | | III-1 | Bbs9 | 27241 | 31-May-15 |
| 3218 | 2 | 3 | | | III-1 | BC006965 | | |
| 3219 | 2 | 3 | | | III-1 | BC016579 | | |
| 3223 | 2 | 3 | | | III-1 | BC018473 | | |
| 3225 | 2 | 3 | | | III-1 | BC020402 | | |
| 3226 | 2 | 3 | | | III-1 | BC021614 | | |
| 3231 | 2 | 3 | | | III-1 | BC023829 | | |
| 3232 | 2 | 3 | | | III-1 | BC024139 | | |
| 3233 | 2 | 3 | | | III-1 | BC024386 | | |
| 3235 | 2 | 3 | | | III-1 | BC025920 | | |
| 3241 | 2 | 3 | | | III-1 | BC029722 | | |
| 3243 | 2 | 3 | | | III-1 | BC030336 | | |
| 3248 | 2 | 3 | | | III-1 | BC031181 | | |
| 3251 | 2 | 3 | | | III-1 | BC035044 | | |
| 3254 | 2 | 3 | | | III-1 | BC037704 | | |
| 3255 | 2 | 3 | | | III-1 | BC039771 | | |
| 3257 | 2 | 3 | | | III-1 | BC048403 | | |
| 3263 | 2 | 3 | | | III-1 | BC048609 | | |
| 3267 | 2 | 3 | | | III-1 | BC049352 | | |
| 3270 | 2 | 3 | | | III-1 | BC049730 | | |
| 3274 | 2 | 3 | | | III-1 | BC051226 | | |
| 3279 | 2 | 3 | | | III-1 | BC052688 | | |
| 3282 | 2 | 3 | | | III-1 | BC055111 | | |
| 3293 | 2 | 3 | | | III-1 | BC080695 | | |
| 3297 | 2 | 3 | | | III-1 | Bc1 | | |
| 3302 | 2 | 3 | | | III-1 | BC147527 | | |
| 3304 | 2 | 3 | | | III-1 | Bcan | 63827 | 12-May-15 |
| 3306 | 2 | 3 | | | III-1 | Bcap31 | 10134 | 24-May-15 |
| 3309 | 2 | 3 | | | III-1 | Bcas1 | 8537 | 4-May-15 |
| 3315 | 2 | 3 | | | III-1 | Bcat1 | 586 | 4-May-15 |
| 3317 | 2 | 3 | | | III-1 | Bccip | 56647 | 4-May-15 |
| 3320 | 2 | 3 | | | III-1 | Bckdha | 593 | 23-May-15 |
| 3321 | 2 | 3 | | | III-1 | Bckdhb | 594 | 23-May-15 |
| 3322 | 2 | 3 | | | III-1 | Bckdk | 10295 | 4-May-15 |
| 3334 | 2 | 3 | | | III-1 | Bcl2l12 | 83596 | 4-May-15 |
| 3335 | 2 | 3 | | | III-1 | Bcl2l13 | 23786 | 4-May-15 |
| 3338 | 2 | 3 | | | III-1 | Bcl2l2 | 599 | 4-May-15 |
| 3343 | 2 | 3 | | | III-1 | Bcl7b | 9275 | 4-May-15 |
| 3345 | 2 | 3 | | | III-1 | Bcl9 | 607 | 4-May-15 |
| 3347 | 2 | 3 | | | III-1 | Bclaf1 | 9774 | 4-May-15 |
| 3352 | 2 | 3 | | | III-1 | Bcr | 613 | 24-May-15 |
| 3364 | 2 | 3 | | | III-1 | Bend3 | 57673 | 20-May-15 |
| 3367 | 2 | 3 | | | III-1 | Bend6 | 221336 | 4-May-15 |

| | | | | | | | | |
|---|---|---|---|---|---|---|---|---|
| 3371 | 2 | 3 | | | III-1 | Best3 | 144453 | 4-May-15 |
| 3380 | 2 | 3 | | | III-1 | Bfsp2 | 8419 | 17-May-15 |
| 3382 | 2 | 3 | | | III-1 | Bglap2 | | |
| 3384 | 2 | 3 | | | III-1 | Bgn | 633 | 17-May-15 |
| 3388 | 2 | 3 | | | III-1 | Bhlhe22 | 27319 | 4-May-15 |
| 3398 | 2 | 3 | | | III-1 | Bik | 638 | 12-May-15 |
| 3400 | 2 | 3 | | | III-1 | Bin2 | 51411 | 12-May-15 |
| 3408 | 2 | 3 | | | III-1 | Blcap | 10904 | 4-May-15 |
| 3413 | 2 | 3 | | | III-1 | Bloc1s1 | 2647 | 28-May-15 |
| 3415 | 2 | 3 | | | III-1 | Bloc1s3 | 388552 | 23-May-15 |
| 3416 | 2 | 3 | | | III-1 | Bloc1s4 | 55330 | 4-May-15 |
| 3420 | 2 | 3 | | | III-1 | Blvrb | 645 | 4-May-15 |
| 3423 | 2 | 3 | | | III-1 | Bmi1 | 648 | 2-Jun-15 |
| 3426 | 2 | 3 | | | III-1 | Bmp15 | 9210 | 3-May-15 |
| 3428 | 2 | 3 | | | III-1 | Bmp2k | 55589 | 12-May-15 |
| 3431 | 2 | 3 | | | III-1 | Bmp5 | 653 | 17-May-15 |
| 3436 | 2 | 3 | | | III-1 | Bmper | 168667 | 4-May-15 |
| 3439 | 2 | 3 | | | III-1 | Bmpr2 | 659 | 31-May-15 |
| 3446 | 2 | 3 | | | III-1 | Bnip2 | 663 | 4-May-15 |
| 3448 | 2 | 3 | | | III-1 | Bnip3l | 665 | 4-May-15 |
| 3449 | 2 | 3 | | | III-1 | Bnipl | 149428 | 4-May-15 |
| 3451 | 2 | 3 | | | III-1 | Bod1 | 91272 | 4-May-15 |
| 3453 | 2 | 3 | | | III-1 | Bok | 666 | 4-May-15 |
| 3454 | 2 | 3 | | | III-1 | Bola1 | 51027 | 4-May-15 |
| 3455 | 2 | 3 | | | III-1 | Bola2 | 552900 | 7-Jun-15 |
| 3457 | 2 | 3 | | | III-1 | Boll | 66037 | 4-May-15 |
| 3459 | 2 | 3 | | | III-1 | Bora | 79866 | 4-May-15 |
| 3465 | 2 | 3 | | | III-1 | Bpifa3 | 128861 | 4-May-15 |
| 3477 | 2 | 3 | | | III-1 | Bpnt1 | 10380 | 4-May-15 |
| 3494 | 2 | 3 | | | III-1 | Brf1 | 2972 | 7-Jun-15 |
| 3498 | 2 | 3 | | | III-1 | Bricd5 | 283870 | 4-May-15 |
| 3506 | 2 | 3 | | | III-1 | Brms1l | 84312 | 4-May-15 |
| 3507 | 2 | 3 | | | III-1 | Brox | 148362 | 4-May-15 |
| 3510 | 2 | 3 | | | III-1 | Brs3 | 680 | 4-May-15 |
| 3512 | 2 | 3 | | | III-1 | Brsk2 | 9024 | 28-May-15 |
| 3513 | 2 | 3 | | | III-1 | Brwd1 | 54014 | 12-May-15 |
| 3519 | 2 | 3 | | | III-1 | Bsnd | 7809 | 12-May-15 |
| 3523 | 2 | 3 | | | III-1 | Bst1 | 683 | 7-Jun-15 |
| 3526 | 2 | 3 | | | III-1 | Btaf1 | 9044 | 4-May-15 |
| 3530 | 2 | 3 | | | III-1 | Btbd16 | 118663 | 4-May-15 |
| 3532 | 2 | 3 | | | III-1 | Btbd18 | 643376 | 4-May-15 |
| 3534 | 2 | 3 | | | III-1 | Btbd2 | 55643 | 4-May-15 |
| 3536 | 2 | 3 | | | III-1 | Btbd6 | 90135 | 4-May-15 |
| 3543 | 2 | 3 | | | III-1 | Btf3l4 | 91408 | 4-May-15 |
| 3556 | 2 | 3 | | | III-1 | Btnl5-ps | | |
| 3568 | 2 | 3 | | | III-1 | Bzw1 | 9689 | 4-May-15 |
| 3570 | 2 | 3 | | | III-1 | C030006K11Rik | | |
| 3571 | 2 | 3 | | | III-1 | C030007H22Rik | | |
| 3573 | 2 | 3 | | | III-1 | C030016D13Rik | | |
| 3579 | 2 | 3 | | | III-1 | C030037D09Rik | | |
| 3580 | 2 | 3 | | | III-1 | C030039L03Rik | | |
| 3586 | 2 | 3 | | | III-1 | C130036L24Rik | | |
| 3587 | 2 | 3 | | | III-1 | C130046K22Rik | | |
| 3589 | 2 | 3 | | | III-1 | C130060C02Rik | | |
| 3594 | 2 | 3 | | | III-1 | C130080G10Rik | | |
| 3598 | 2 | 3 | | | III-1 | C1galt1c1 | 29071 | 4-May-15 |
| 3601 | 2 | 3 | | | III-1 | C1qbp | 708 | 4-May-15 |
| 3603 | 2 | 3 | | | III-1 | C1ql1 | 10882 | 4-May-15 |
| 3605 | 2 | 3 | | | III-1 | C1ql3 | 389941 | 4-May-15 |
| 3611 | 2 | 3 | | | III-1 | C1qtnf5 | 114902 | 4-May-15 |
| 3614 | 2 | 3 | | | III-1 | C1qtnf9 | 338872 | 12-May-15 |
| 3616 | 2 | 3 | | | III-1 | C1rb | | |
| 3617 | 2 | 3 | | | III-1 | C1rl | 51279 | 12-May-15 |
| 3619 | 2 | 3 | | | III-1 | C1s2 | | |
| 3620 | 2 | 3 | | | III-1 | C2 | 717 | 7-Jun-15 |
| 3625 | 2 | 3 | | | III-1 | C230037L18Rik | | |
| 3626 | 2 | 3 | | | III-1 | C230052I12Rik | | |
| 3630 | 2 | 3 | | | III-1 | C2cd2l | 9854 | 4-May-15 |
| 3635 | 2 | 3 | | | III-1 | C2cd4d | 100191 040 | 4-May-15 |
| 3638 | 2 | 3 | | | III-1 | C330006A16Rik | | |
| 3645 | 2 | 3 | | | III-1 | C330022C24Rik | | |
| 3646 | 2 | 3 | | | III-1 | C330024C12Rik | | |
| 3651 | 2 | 3 | | | III-1 | C430002E04Rik | | |
| 3658 | 2 | 3 | | | III-1 | C530005A16Rik | | |
| 3660 | 2 | 3 | | | III-1 | C530044C16Rik | | |
| 3662 | 2 | 3 | | | III-1 | C5ar2 | 27202 | 4-May-15 |
| 3672 | 2 | 3 | | | III-1 | C77370 | | |
| 3686 | 2 | 3 | | | III-1 | C920009B18Rik | | |
| 3688 | 2 | 3 | | | III-1 | C920025E04Rik | | |
| 3690 | 2 | 3 | | | III-1 | Cab39 | 51719 | 4-May-15 |
| 3694 | 2 | 3 | | | III-1 | Cables2 | 81928 | 4-May-15 |
| 3695 | 2 | 3 | | | III-1 | Cabp1 | 9478 | 7-Jun-15 |
| 3699 | 2 | 3 | | | III-1 | Cabp7 | 164633 | 4-May-15 |
| 3701 | 2 | 3 | | | III-1 | Cabyr | 26256 | 12-May-15 |
| 3702 | 2 | 3 | | | III-1 | Cacfd1 | 11094 | 4-May-15 |
| 3708 | 2 | 3 | | | III-1 | Cacna1e | 777 | 12-May-15 |
| 3712 | 2 | 3 | | | III-1 | Cacna1i | 8911 | 7-Jun-15 |
| 3719 | 2 | 3 | | | III-1 | Cacnb2 | 783 | 23-May-15 |
| 3729 | 2 | 3 | | | III-1 | Cacng8 | 59283 | 4-May-15 |
| 3735 | 2 | 3 | | | III-1 | Cadm2 | 253559 | 2-Jun-15 |
| 3738 | 2 | 3 | | | III-1 | Cadps | 8618 | 4-May-15 |
| 3740 | 2 | 3 | | | III-1 | Cage1 | 285782 | 4-May-15 |
| 3742 | 2 | 3 | | | III-1 | Calb2 | 794 | 17-May-15 |
| 3746 | 2 | 3 | | | III-1 | Calcoco2 | 10241 | 12-May-15 |
| 3748 | 2 | 3 | | | III-1 | Calcrl | 10203 | 12-May-15 |
| 3750 | 2 | 3 | | | III-1 | Calhm1 | 255022 | 4-May-15 |
| 3756 | 2 | 3 | | | III-1 | Calm5 | | |

Fig.22 - 62

| | | | | | | | | |
|---|---|---|---|---|---|---|---|---|
| 3760 | 2 | 3 | | | III-1 | Calr | 811 | 17-May-15 |
| 3761 | 2 | 3 | | | III-1 | Calr3 | 125972 | 4-May-15 |
| 3762 | 2 | 3 | | | III-1 | Calr4 | | |
| 3766 | 2 | 3 | | | III-1 | Camk1d | 57118 | 4-May-15 |
| 3767 | 2 | 3 | | | III-1 | Camk1g | 57172 | 4-May-15 |
| 3770 | 2 | 3 | | | III-1 | Camk2d | 817 | 4-May-15 |
| 3774 | 2 | 3 | | | III-1 | Camk4 | 814 | 4-May-15 |
| 3777 | 2 | 3 | | | III-1 | Camkmt | 79823 | 12-May-15 |
| 3778 | 2 | 3 | | | III-1 | Camkv | 79012 | 4-May-15 |
| 3784 | 2 | 3 | | | III-1 | Camta1 | 23261 | 4-May-15 |
| 3785 | 2 | 3 | | | III-1 | Camta2 | 23125 | 4-May-15 |
| 3793 | 2 | 3 | | | III-1 | Capn1 | 823 | 17-May-15 |
| 3795 | 2 | 3 | | | III-1 | Capn11 | 11131 | 4-May-15 |
| 3799 | 2 | 3 | | | III-1 | Capn2 | 824 | 12-May-15 |
| 3802 | 2 | 3 | | | III-1 | Capn6 | 827 | |
| 3805 | 2 | 3 | | | III-1 | Capn9 | 10753 | 4-May-15 |
| 3808 | 2 | 3 | | | III-1 | Caprin1 | 4076 | 4-May-15 |
| 3810 | 2 | 3 | | | III-1 | Caps2 | 84698 | 4-May-15 |
| 3831 | 2 | 3 | | | III-1 | Car9 | | |
| 3832 | 2 | 3 | | | III-1 | Card10 | 29775 | 4-May-15 |
| 3833 | 2 | 3 | | | III-1 | Card11 | 84433 | 4-May-15 |
| 3835 | 2 | 3 | | | III-1 | Card6 | 84674 | 12-May-15 |
| 3836 | 2 | 3 | | | III-1 | Card9 | 64170 | 24-May-15 |
| 3839 | 2 | 3 | | | III-1 | Carkd | 55739 | 4-May-15 |
| 3845 | 2 | 3 | | | III-1 | Casc1 | 55259 | 4-May-15 |
| 3848 | 2 | 3 | | | III-1 | Casc5 | 57082 | |
| 3853 | 2 | 3 | | | III-1 | Casp1 | 834 | |
| 3855 | 2 | 3 | | | III-1 | Casp14 | 23581 | 4-May-15 |
| 3856 | 2 | 3 | | | III-1 | Casp2 | 835 | |
| 3867 | 2 | 3 | | | III-1 | Cass4 | 57091 | |
| 3869 | 2 | 3 | | | III-1 | Casz1 | 54897 | 4-May-15 |
| 3870 | 2 | 3 | | | III-1 | Cat | 847 | 7-Jun-15 |
| 3871 | 2 | 3 | | | III-1 | Catip | 375307 | 21-May-15 |
| 3874 | 2 | 3 | | | III-1 | Catsper3 | 347732 | 4-May-15 |
| 3876 | 2 | 3 | | | III-1 | Catsperb | 79820 | 4-May-15 |
| 3878 | 2 | 3 | | | III-1 | Catsperg1 | | |
| 3889 | 2 | 3 | | | III-1 | Cbll1 | 79872 | 12-May-15 |
| 3891 | 2 | 3 | | | III-1 | Cbln2 | 147381 | 4-May-15 |
| 3893 | 2 | 3 | | | III-1 | Cbln4 | 140689 | 4-May-15 |
| 3897 | 2 | 3 | | | III-1 | Cbr4 | 84869 | 4-May-15 |
| 3900 | 2 | 3 | | | III-1 | Cbx1 | 10951 | 4-May-15 |
| 3903 | 2 | 3 | | | III-1 | Cbx4 | 8535 | 17-May-15 |
| 3907 | 2 | 3 | | | III-1 | Cbx8 | 57332 | 4-May-15 |
| 3917 | 2 | 3 | | | III-1 | Ccdc101 | 112869 | 4-May-15 |
| 3918 | 2 | 3 | | | III-1 | Ccdc102a | 92922 | 12-May-15 |
| 3919 | 2 | 3 | | | III-1 | Ccdc103 | 388389 | 23-May-15 |
| 3921 | 2 | 3 | | | III-1 | Ccdc105 | 126402 | 4-May-15 |
| 3924 | 2 | 3 | | | III-1 | Ccdc108 | 255101 | 4-May-15 |
| 3927 | 2 | 3 | | | III-1 | Ccdc110 | 256309 | 4-May-15 |
| 3929 | 2 | 3 | | | III-1 | Ccdc113 | 29070 | 12-May-15 |
| 3932 | 2 | 3 | | | III-1 | Ccdc116 | 164592 | 28-May-15 |
| 3933 | 2 | 3 | | | III-1 | Ccdc117 | 150275 | 4-May-15 |
| 3936 | 2 | 3 | | | III-1 | Ccdc121 | 79635 | 4-May-15 |
| 3938 | 2 | 3 | | | III-1 | Ccdc124 | 115098 | 4-May-15 |
| 3941 | 2 | 3 | | | III-1 | Ccdc127 | 133957 | 4-May-15 |
| 3947 | 2 | 3 | | | III-1 | Ccdc135 | 84229 | 4-May-15 |
| 3949 | 2 | 3 | | | III-1 | Ccdc137 | 339230 | 4-May-15 |
| 3950 | 2 | 3 | | | III-1 | Ccdc138 | 165055 | 12-May-15 |
| 3953 | 2 | 3 | | | III-1 | Ccdc142 | 84865 | 4-May-15 |
| 3954 | 2 | 3 | | | III-1 | Ccdc144b | 284047 | 4-May-15 |
| 3958 | 2 | 3 | | | III-1 | Ccdc149 | 91050 | 4-May-15 |
| 3969 | 2 | 3 | | | III-1 | Ccdc160 | 347475 | 12-May-15 |
| 3970 | 2 | 3 | | | III-1 | Ccdc162 | 221262 | 4-May-15 |
| 3974 | 2 | 3 | | | III-1 | Ccdc169 | 728591 | 12-May-15 |
| 3976 | 2 | 3 | | | III-1 | Ccdc170 | 80129 | 4-May-15 |
| 3977 | 2 | 3 | | | III-1 | Ccdc171 | 203238 | 4-May-15 |
| 3980 | 2 | 3 | | | III-1 | Ccdc174 | 51244 | 4-May-15 |
| 3981 | 2 | 3 | | | III-1 | Ccdc175 | 729665 | 4-May-15 |
| 3983 | 2 | 3 | | | III-1 | Ccdc177 | 56936 | 4-May-15 |
| 3989 | 2 | 3 | | | III-1 | Ccdc185 | 164127 | 4-May-15 |
| 3991 | 2 | 3 | | | III-1 | Ccdc22 | 28952 | 4-May-15 |
| 3993 | 2 | 3 | | | III-1 | Ccdc24 | 149473 | 12-May-15 |
| 3994 | 2 | 3 | | | III-1 | Ccdc25 | 55246 | 12-May-15 |
| 3998 | 2 | 3 | | | III-1 | Ccdc3 | 83643 | 12-May-15 |
| 4000 | 2 | 3 | | | III-1 | Ccdc32 | 90416 | 4-May-15 |
| 4003 | 2 | 3 | | | III-1 | Ccdc34os | | |
| 4011 | 2 | 3 | | | III-1 | Ccdc43 | 124808 | 4-May-15 |
| 4012 | 2 | 3 | | | III-1 | Ccdc47 | 57003 | 4-May-15 |
| 4014 | 2 | 3 | | | III-1 | Ccdc51 | 79714 | 4-May-15 |
| 4016 | 2 | 3 | | | III-1 | Ccdc54 | 84692 | 4-May-15 |
| 4020 | 2 | 3 | | | III-1 | Ccdc59 | 29080 | 4-May-15 |
| 4021 | 2 | 3 | | | III-1 | Ccdc6 | 8030 | |
| 4025 | 2 | 3 | | | III-1 | Ccdc63 | 160762 | 12-May-15 |
| 4027 | 2 | 3 | | | III-1 | Ccdc64b | 146439 | 4-May-15 |
| 4030 | 2 | 3 | | | III-1 | Ccdc67 | 159989 | 4-May-15 |
| 4031 | 2 | 3 | | | III-1 | Ccdc68 | 80323 | |
| 4032 | 2 | 3 | | | III-1 | Ccdc69 | 26112 | |
| 4037 | 2 | 3 | | | III-1 | Ccdc73 | 493860 | 4-May-15 |
| 4046 | 2 | 3 | | | III-1 | Ccdc83 | 220047 | 4-May-15 |
| 4048 | 2 | 3 | | | III-1 | Ccdc85a | 114800 | 4-May-15 |
| 4056 | 2 | 3 | | | III-1 | Ccdc89 | 220388 | 4-May-15 |
| 4063 | 2 | 3 | | | III-1 | Ccdc96 | 257236 | 28-May-15 |
| 4071 | 2 | 3 | | | III-1 | Ccl1 | 6346 | 4-May-15 |
| 4092 | 2 | 3 | | | III-1 | Ccl7 | 6354 | |
| 4095 | 2 | 3 | | | III-1 | Ccm2 | 83605 | 23-May-15 |
| 4097 | 2 | 3 | | | III-1 | Ccna1 | 8900 | 12-May-15 |
| 4106 | 2 | 3 | | | III-1 | Ccnd3 | 896 | 12-May-15 |

| | | | | | | | | |
|---|---|---|---|---|---|---|---|---|
| 4112 | 2 | 3 | | | III-1 | Ccng2 | 901 | |
| 4113 | 2 | 3 | | | III-1 | Ccnh | 902 | 4-May-15 |
| 4118 | 2 | 3 | | | III-1 | Ccnl1 | 57018 | 7-Jun-15 |
| 4121 | 2 | 3 | | | III-1 | Ccnt1 | 904 | 4-May-15 |
| 4129 | 2 | 3 | | | III-1 | Ccr10 | 2826 | 7-Jun-15 |
| 4133 | 2 | 3 | | | III-1 | Ccr4 | 1233 | 7-Jun-15 |
| 4141 | 2 | 3 | | | III-1 | Ccs | 9973 | 12-May-15 |
| 4142 | 2 | 3 | | | III-1 | Ccsap | 126731 | |
| 4143 | 2 | 3 | | | III-1 | Ccser1 | 401145 | 4-May-15 |
| 4156 | 2 | 3 | | | III-1 | Cd109 | 135228 | |
| 4158 | 2 | 3 | | | III-1 | Cd151 | 977 | 4-May-15 |
| 4159 | 2 | 3 | | | III-1 | Cd160 | 11126 | 31-May-15 |
| 4161 | 2 | 3 | | | III-1 | Cd163l1 | 283316 | 28-May-15 |
| 4162 | 2 | 3 | | | III-1 | Cd164 | 8763 | 12-May-15 |
| 4165 | 2 | 3 | | | III-1 | Cd180 | 4064 | 4-May-15 |
| 4176 | 2 | 3 | | | III-1 | Cd209a | | |
| 4194 | 2 | 3 | | | III-1 | Cd2bp2 | 10421 | 4-May-15 |
| 4196 | 2 | 3 | | | III-1 | Cd300c | 10871 | |
| 4197 | 2 | 3 | | | III-1 | Cd300e | 342510 | 4-May-15 |
| 4200 | 2 | 3 | | | III-1 | Cd300lf | 146722 | 4-May-15 |
| 4201 | 2 | 3 | | | III-1 | Cd300lg | 146894 | |
| 4202 | 2 | 3 | | | III-1 | Cd300lh | | |
| 4204 | 2 | 3 | | | III-1 | Cd320 | 51293 | 4-May-15 |
| 4205 | 2 | 3 | | | III-1 | Cd33 | 945 | |
| 4208 | 2 | 3 | | | III-1 | Cd37 | 951 | 4-May-15 |
| 4213 | 2 | 3 | | | III-1 | Cd3g | 917 | 2015/6/7 |
| 4226 | 2 | 3 | | | III-1 | Cd59b | | |
| 4241 | 2 | 3 | | | III-1 | Cd83 | 9308 | |
| 4247 | 2 | 3 | | | III-1 | Cd93 | 22918 | |
| 4248 | 2 | 3 | | | III-1 | Cd96 | 10225 | 12-May-15 |
| 4250 | 2 | 3 | | | III-1 | Cd99l2 | 83692 | 4-May-15 |
| 4256 | 2 | 3 | | | III-1 | Cdc14b | 8555 | 13-Jun-15 |
| 4257 | 2 | 3 | | | III-1 | Cdc16 | 8881 | 12-May-15 |
| 4262 | 2 | 3 | | | III-1 | Cdc25b | 994 | |
| 4263 | 2 | 3 | | | III-1 | Cdc25c | 995 | |
| 4265 | 2 | 3 | | | III-1 | Cdc27 | 996 | 31-May-15 |
| 4267 | 2 | 3 | | | III-1 | Cdc37 | 11140 | 17-May-15 |
| 4272 | 2 | 3 | | | III-1 | Cdc42bpb | 9578 | 4-May-15 |
| 4273 | 2 | 3 | | | III-1 | Cdc42bpg | 55561 | 4-May-15 |
| 4274 | 2 | 3 | | | III-1 | Cdc42ep1 | 11135 | 4-May-15 |
| 4275 | 2 | 3 | | | III-1 | Cdc42ep2 | 10435 | 4-May-15 |
| 4276 | 2 | 3 | | | III-1 | Cdc42ep3 | 10602 | 4-May-15 |
| 4286 | 2 | 3 | | | III-1 | Cdca2 | 157313 | |
| 4292 | 2 | 3 | | | III-1 | Cdca8 | 55143 | 4-May-15 |
| 4294 | 2 | 3 | | | III-1 | Cdcp2 | 200008 | 4-May-15 |
| 4296 | 2 | 3 | | | III-1 | Cdh10 | 1008 | 12-May-15 |
| 4297 | 2 | 3 | | | III-1 | Cdh11 | 1009 | |
| 4300 | 2 | 3 | | | III-1 | Cdh15 | 1013 | 12-May-15 |
| 4303 | 2 | 3 | | | III-1 | Cdh18 | 1016 | 12-May-15 |
| 4305 | 2 | 3 | | | III-1 | Cdh2 | 1000 | 24-May-15 |
| 4306 | 2 | 3 | | | III-1 | Cdh20 | 28316 | 12-May-15 |
| 4308 | 2 | 3 | | | III-1 | Cdh23 | 64072 | 23-May-15 |
| 4312 | 2 | 3 | | | III-1 | Cdh4 | 1002 | 4-May-15 |
| 4319 | 2 | 3 | | | III-1 | Cdhr2 | 54825 | 4-May-15 |
| 4322 | 2 | 3 | | | III-1 | Cdip1 | 29965 | 4-May-15 |
| 4330 | 2 | 3 | | | III-1 | Cdk15 | 65061 | 4-May-15 |
| 4334 | 2 | 3 | | | III-1 | Cdk19 | 23097 | 12-May-15 |
| 4340 | 2 | 3 | | | III-1 | Cdk4 | 1019 | 31-May-15 |
| 4350 | 2 | 3 | | | III-1 | Cdk9 | 1025 | 4-May-15 |
| 4352 | 2 | 3 | | | III-1 | Cdkl1 | 8814 | 21-May-15 |
| 4353 | 2 | 3 | | | III-1 | Cdkl2 | 8999 | 4-May-15 |
| 4355 | 2 | 3 | | | III-1 | Cdkl4 | 344387 | |
| 4358 | 2 | 3 | | | III-1 | Cdkn1b | 1027 | 31-May-15 |
| 4360 | 2 | 3 | | | III-1 | Cdkn2a | 1029 | 24-May-15 |
| 4361 | 2 | 3 | | | III-1 | Cdkn2aip | 55602 | 4-May-15 |
| 4365 | 2 | 3 | | | III-1 | Cdkn2d | 1032 | |
| 4366 | 2 | 3 | | | III-1 | Cdkn3 | 1033 | |
| 4369 | 2 | 3 | | | III-1 | Cdon | 50937 | |
| 4371 | 2 | 3 | | | III-1 | Cdr1 | 1038 | 4-May-15 |
| 4374 | 2 | 3 | | | III-1 | Cdrt4 | 284040 | 4-May-15 |
| 4376 | 2 | 3 | | | III-1 | Cds2 | 8760 | 4-May-15 |
| 4379 | 2 | 3 | | | III-1 | Cdv3 | 55573 | 4-May-15 |
| 4384 | 2 | 3 | | | III-1 | Cdyl2 | 124359 | 4-May-15 |
| 4387 | 2 | 3 | | | III-1 | Ceacam11 | | |
| 4389 | 2 | 3 | | | III-1 | Ceacam13 | | |
| 4396 | 2 | 3 | | | III-1 | Ceacam20 | 125931 | 4-May-15 |
| 4400 | 2 | 3 | | | III-1 | Ceacam-ps1 | | |
| 4404 | 2 | 3 | | | III-1 | Cebpe | 1053 | 4-May-15 |
| 4405 | 2 | 3 | | | III-1 | Cebpg | 1054 | 28-May-15 |
| 4408 | 2 | 3 | | | III-1 | Cecr2 | 27443 | |
| 4409 | 2 | 3 | | | III-1 | Cecr5 | 27440 | 4-May-15 |
| 4410 | 2 | 3 | | | III-1 | Cecr6 | 27439 | 4-May-15 |
| 4417 | 2 | 3 | | | III-1 | Celf3 | 11189 | 4-May-15 |
| 4419 | 2 | 3 | | | III-1 | Celf5 | 60680 | 3-Jun-15 |
| 4421 | 2 | 3 | | | III-1 | Celrr | | |
| 4423 | 2 | 3 | | | III-1 | Celsr2 | 1952 | 31-May-15 |
| 4424 | 2 | 3 | | | III-1 | Celsr3 | 1951 | 12-May-15 |
| 4437 | 2 | 3 | | | III-1 | Cenpm | 79019 | |
| 4443 | 2 | 3 | | | III-1 | Cenpu | 79682 | |
| 4445 | 2 | 3 | | | III-1 | Cenpw | 387103 | |
| 4447 | 2 | 3 | | | III-1 | Cep112 | 201134 | 12-May-15 |
| 4448 | 2 | 3 | | | III-1 | Cep120 | 153241 | 4-May-15 |
| 4451 | 2 | 3 | | | III-1 | Cep135 | 9662 | 23-May-15 |
| 4455 | 2 | 3 | | | III-1 | Cep170 | 9859 | 12-May-15 |
| 4457 | 2 | 3 | | | III-1 | Cep19 | 84984 | 4-May-15 |
| 4462 | 2 | 3 | | | III-1 | Cep41 | 95681 | 23-May-15 |
| 4464 | 2 | 3 | | | III-1 | Cep55 | 55165 | 4-May-15 |

Fig.22 - 63

| | | | | | | | | |
|---|---|---|---|---|---|---|---|---|
| 4466 | 2 | 3 | | | III-1 | Cep57l1 | 285753 | 12-May-15 |
| 4467 | 2 | 3 | | | III-1 | Cep63 | 80254 | 4-May-15 |
| 4468 | 2 | 3 | | | III-1 | Cep68 | 23177 | 7-Jun-15 |
| 4470 | 2 | 3 | | | III-1 | Cep72 | 55722 | |
| 4473 | 2 | 3 | | | III-1 | Cep83 | 51134 | 12-May-15 |
| 4474 | 2 | 3 | | | III-1 | Cep83os | | |
| 4481 | 2 | 3 | | | III-1 | Cer1 | 9350 | 4-May-15 |
| 4488 | 2 | 3 | | | III-1 | Cers4 | 79603 | 4-May-15 |
| 4510 | 2 | 3 | | | III-1 | Cetn1 | 1068 | 4-May-15 |
| 4512 | 2 | 3 | | | III-1 | Cetn3 | 1070 | 12-May-15 |
| 4513 | 2 | 3 | | | III-1 | Cetn4 | | |
| 4514 | 2 | 3 | | | III-1 | Cfb | 629 | |
| 4515 | 2 | 3 | | | III-1 | Cfc1 | 55997 | 4-May-15 |
| 4519 | 2 | 3 | | | III-1 | Cfhr1 | 3078 | 23-May-15 |
| 4521 | 2 | 3 | | | III-1 | Cfi | 3426 | |
| 4522 | 2 | 3 | | | III-1 | Cfl1 | 1072 | 7-Jun-15 |
| 4524 | 2 | 3 | | | III-1 | Cflar | 8837 | 31-May-15 |
| 4527 | 2 | 3 | | | III-1 | Cga | 1081 | /6/7 |
| 4536 | 2 | 3 | | | III-1 | Chad | 1101 | 4-May-15 |
| 4540 | 2 | 3 | | | III-1 | Champ1 | 283489 | 4-May-15 |
| 4544 | 2 | 3 | | | III-1 | Chchd2 | 51142 | 21-May-15 |
| 4546 | 2 | 3 | | | III-1 | Chchd4 | 131474 | 4-May-15 |
| 4550 | 2 | 3 | | | III-1 | Chd1 | 1105 | 12-May-15 |
| 4553 | 2 | 3 | | | III-1 | Chd3 | 1107 | 4-May-15 |
| 4554 | 2 | 3 | | | III-1 | Chd3os | | |
| 4558 | 2 | 3 | | | III-1 | Chd7 | 55636 | 23-May-15 |
| 4559 | 2 | 3 | | | III-1 | Chd8 | 57680 | 12-May-15 |
| 4561 | 2 | 3 | | | III-1 | Chdh | 55349 | 21-May-15 |
| 4567 | 2 | 3 | | | III-1 | Chgb | 1114 | 21-May-15 |
| 4574 | 2 | 3 | | | III-1 | Chil4 | | |
| 4577 | 2 | 3 | | | III-1 | Chka | 1119 | |
| 4578 | 2 | 3 | | | III-1 | Chkb | 1120 | 22-May-15 |
| 4581 | 2 | 3 | | | III-1 | Chm | 1121 | 7-Jun-15 |
| 4590 | 2 | 3 | | | III-1 | Chmp5 | 51510 | 31-May-15 |
| 4599 | 2 | 3 | | | III-1 | Chp2 | 63928 | 4-May-15 |
| 4600 | 2 | 3 | | | III-1 | Chpf | 79586 | |
| 4601 | 2 | 3 | | | III-1 | Chpf2 | 54480 | 4-May-15 |
| 4603 | 2 | 3 | | | III-1 | Chrac1 | 54108 | 12-May-15 |
| 4609 | 2 | 3 | | | III-1 | Chrm3 | 1131 | 4-May-15 |
| 4611 | 2 | 3 | | | III-1 | Chrm5 | 1133 | 4-May-15 |
| 4619 | 2 | 3 | | | III-1 | Chrna7 | 1139 | 7-Jun-15 |
| 4622 | 2 | 3 | | | III-1 | Chrnb2 | 1141 | 23-May-15 |
| 4623 | 2 | 3 | | | III-1 | Chrnb3 | 1142 | 12-May-15 |
| 4625 | 2 | 3 | | | III-1 | Chrnd | 1144 | 7-May-15 |
| 4630 | 2 | 3 | | | III-1 | Chst11 | 50515 | 4-May-15 |
| 4632 | 2 | 3 | | | III-1 | Chst13 | 166012 | 4-May-15 |
| 4635 | 2 | 3 | | | III-1 | Chst2 | 9435 | |
| 4636 | 2 | 3 | | | III-1 | Chst3 | 9469 | |
| 4638 | 2 | 3 | | | III-1 | Chst5 | 23563 | 4-May-15 |
| 4641 | 2 | 3 | | | III-1 | Chst9 | 83539 | 4-May-15 |
| 4642 | 2 | 3 | | | III-1 | Chsy1 | 22856 | 4-May-15 |
| 4649 | 2 | 3 | | | III-1 | Ciao1 | 9391 | 4-May-15 |
| 4652 | 2 | 3 | | | III-1 | Cib1 | 10519 | 4-May-15 |
| 4656 | 2 | 3 | | | III-1 | Cic | 23152 | 17-May-15 |
| 4667 | 2 | 3 | | | III-1 | Cirh1a | 84916 | 12-May-15 |
| 4668 | 2 | 3 | | | III-1 | Cisd1 | 55847 | 4-May-15 |
| 4669 | 2 | 3 | | | III-1 | Cisd2 | 493856 | 2-Jun-15 |
| 4671 | 2 | 3 | | | III-1 | Cish | 1154 | 4-May-15 |
| 4672 | 2 | 3 | | | III-1 | Cistr-act | 102216268 | 12-May-15 |
| 4678 | 2 | 3 | | | III-1 | CK137956 | | |
| 4689 | 2 | 3 | | | III-1 | Cks1brt | | |
| 4700 | 2 | 3 | | | III-1 | Clcc1 | 23155 | 4-May-15 |
| 4704 | 2 | 3 | | | III-1 | Clcn3 | 1182 | 4-May-15 |
| 4705 | 2 | 3 | | | III-1 | Clcn4-2 | | |
| 4707 | 2 | 3 | | | III-1 | Clcn6 | 1185 | |
| 4708 | 2 | 3 | | | III-1 | Clcn7 | 1186 | 23-May-15 |
| 4709 | 2 | 3 | | | III-1 | Clcnka | 1187 | 4-May-15 |
| 4714 | 2 | 3 | | | III-1 | Cldn12 | 9069 | 4-May-15 |
| 4717 | 2 | 3 | | | III-1 | Cldn15 | 24146 | |
| 4720 | 2 | 3 | | | III-1 | Cldn18 | 51208 | 12-May-15 |
| 4727 | 2 | 3 | | | III-1 | Cldn25 | 644672 | 4-May-15 |
| 4735 | 2 | 3 | | | III-1 | Cldn9 | 9080 | 4-May-15 |
| 4736 | 2 | 3 | | | III-1 | Cldnd2 | 125875 | 4-May-15 |
| 4741 | 2 | 3 | | | III-1 | Clec14a | 161198 | 4-May-15 |
| 4744 | 2 | 3 | | | III-1 | Clec1a | 51267 | |
| 4746 | 2 | 3 | | | III-1 | Clec2d | 29121 | 4-May-15 |
| 4748 | 2 | 3 | | | III-1 | Clec2f | | |
| 4753 | 2 | 3 | | | III-1 | Clec3a | 10143 | 14-May-15 |
| 4760 | 2 | 3 | | | III-1 | Clec4b2 | | |
| 4762 | 2 | 3 | | | III-1 | Clec4e | 26253 | 4-May-15 |
| 4766 | 2 | 3 | | | III-1 | Clec5a | 23601 | |
| 4769 | 2 | 3 | | | III-1 | Clgn | 1047 | 13-Jun-15 |
| 4777 | 2 | 3 | | | III-1 | Clip1 | 6249 | 4-May-15 |
| 4778 | 2 | 3 | | | III-1 | Clip2 | 7461 | 7-Jun-15 |
| 4781 | 2 | 3 | | | III-1 | Clk1 | 1195 | 7-Jun-15 |
| 4787 | 2 | 3 | | | III-1 | Cln3 | 1201 | 23-May-15 |
| 4788 | 2 | 3 | | | III-1 | Cln5 | 1203 | 23-May-15 |
| 4790 | 2 | 3 | | | III-1 | Cln8 | 2055 | |
| 4799 | 2 | 3 | | | III-1 | Clptm1 | 1209 | 4-May-15 |
| 4807 | 2 | 3 | | | III-1 | Clstn2 | 64084 | 24-May-15 |
| 4814 | 2 | 3 | | | III-1 | Cluh | 23277 | 12-May-15 |
| 4823 | 2 | 3 | | | III-1 | Cmc1 | 152100 | 4-May-15 |
| 4824 | 2 | 3 | | | III-1 | Cmc2 | 56942 | |
| 4825 | 2 | 3 | | | III-1 | Cmip | 80790 | 12-May-15 |
| 4833 | 2 | 3 | | | III-1 | Cmss1 | 84319 | 4-May-15 |
| 4839 | 2 | 3 | | | III-1 | Cmtm5 | 116173 | 4-May-15 |
| 4843 | 2 | 3 | | | III-1 | Cmtr1 | 23070 | 12-May-15 |
| 4848 | 2 | 3 | | | III-1 | Cndp1 | 84735 | 4-May-15 |
| 4849 | 2 | 3 | | | III-1 | Cndp2 | 55748 | 12-May-15 |
| 4852 | 2 | 3 | | | III-1 | Cnga1 | 1259 | 23-May-15 |
| 4854 | 2 | 3 | | | III-1 | Cnga3 | 1261 | 31-May-15 |
| 4855 | 2 | 3 | | | III-1 | Cnga4 | 1262 | 4-May-15 |
| 4858 | 2 | 3 | | | III-1 | Cnih1 | 10175 | 4-May-15 |
| 4859 | 2 | 3 | | | III-1 | Cnih2 | 254263 | 4-May-15 |
| 4860 | 2 | 3 | | | III-1 | Cnih3 | 149111 | 4-May-15 |
| 4864 | 2 | 3 | | | III-1 | Cnksr3 | 154043 | 4-May-15 |
| 4870 | 2 | 3 | | | III-1 | Cnnm3 | 26505 | 4-May-15 |
| 4873 | 2 | 3 | | | III-1 | Cnot10 | 25904 | 21-May-15 |
| 4875 | 2 | 3 | | | III-1 | Cnot2 | 4848 | 28-May-15 |
| 4892 | 2 | 3 | | | III-1 | Cntd1 | 124817 | 4-May-15 |
| 4895 | 2 | 3 | | | III-1 | Cntln | 54875 | 1-Jun-15 |
| 4897 | 2 | 3 | | | III-1 | Cntn2 | 6900 | 4-May-15 |
| 4903 | 2 | 3 | | | III-1 | Cntnap2 | 26047 | 12-May-15 |
| 4913 | 2 | 3 | | | III-1 | Coa5 | 493753 | 4-May-15 |
| 4918 | 2 | 3 | | | III-1 | Cobll1 | 22837 | 4-May-15 |
| 4920 | 2 | 3 | | | III-1 | Cog1 | 9382 | 23-May-15 |
| 4926 | 2 | 3 | | | III-1 | Cog7 | 91949 | 23-May-15 |
| 4930 | 2 | 3 | | | III-1 | Col11a1 | 1301 | 23-May-15 |
| 4933 | 2 | 3 | | | III-1 | Col13a1 | 1305 | 12-May-15 |
| 4939 | 2 | 3 | | | III-1 | Col19a1 | 1310 | 12-May-15 |
| 4947 | 2 | 3 | | | III-1 | Col26a1 | 136227 | 4-May-15 |
| 4950 | 2 | 3 | | | III-1 | Col2a1 | 1280 | |
| 4966 | 2 | 3 | | | III-1 | Col6a5 | 256076 | 4-May-15 |
| 4971 | 2 | 3 | | | III-1 | Col9a1 | 1297 | 23-May-15 |
| 4974 | 2 | 3 | | | III-1 | Colec10 | 10584 | 4-May-15 |
| 4977 | 2 | 3 | | | III-1 | Colgalt2 | 23127 | |
| 4981 | 2 | 3 | | | III-1 | Commd2 | 51122 | 4-May-15 |
| 4984 | 2 | 3 | | | III-1 | Commd5 | 28991 | 14-May-15 |
| 4988 | 2 | 3 | | | III-1 | Commd9 | 29099 | 12-May-15 |
| 4992 | 2 | 3 | | | III-1 | Copa | 1314 | 4-May-15 |
| 5001 | 2 | 3 | | | III-1 | Cops4 | 51138 | 4-May-15 |
| 5010 | 2 | 3 | | | III-1 | Coq10b | 80219 | 4-May-15 |
| 5011 | 2 | 3 | | | III-1 | Coq2 | 27235 | 12-May-15 |
| 5012 | 2 | 3 | | | III-1 | Coq3 | 51805 | 9-May-15 |
| 5013 | 2 | 3 | | | III-1 | Coq4 | 51117 | 3-Jun-15 |
| 5017 | 2 | 3 | | | III-1 | Coq9 | 57017 | 3-May-15 |
| 5018 | 2 | 3 | | | III-1 | Corin | 10699 | 10-May-15 |
| 5023 | 2 | 3 | | | III-1 | Coro2b | 10391 | 4-May-15 |
| 5028 | 2 | 3 | | | III-1 | Cox10 | 1352 | 22-May-15 |
| 5031 | 2 | 3 | | | III-1 | Cox15 | 1355 | 23-May-15 |
| 5032 | 2 | 3 | | | III-1 | Cox16 | 51241 | 4-May-15 |
| 5033 | 2 | 3 | | | III-1 | Cox17 | 10063 | 4-May-15 |
| 5034 | 2 | 3 | | | III-1 | Cox18 | 285521 | 4-May-15 |
| 5035 | 2 | 3 | | | III-1 | Cox19 | 90639 | 4-May-15 |
| 5040 | 2 | 3 | | | III-1 | Cox5b | 1329 | 21-May-15 |
| 5041 | 2 | 3 | | | III-1 | Cox6a1 | 1337 | 12-May-15 |
| 5045 | 2 | 3 | | | III-1 | Cox6c | 1345 | 4-May-15 |
| 5048 | 2 | 3 | | | III-1 | Cox7a2l | 9167 | 4-May-15 |
| 5050 | 2 | 3 | | | III-1 | Cox7b2 | 170712 | 4-May-15 |
| 5052 | 2 | 3 | | | III-1 | Cox8a | 1351 | 28-May-15 |
| 5061 | 2 | 3 | | | III-1 | Cpa6 | 57094 | 16-Jun-15 |
| 5068 | 2 | 3 | | | III-1 | Cpeb3 | 22849 | |
| 5069 | 2 | 3 | | | III-1 | Cpeb4 | 80315 | |
| 5070 | 2 | 3 | | | III-1 | Cped1 | 79974 | |
| 5072 | 2 | 3 | | | III-1 | Cphx2 | | |
| 5074 | 2 | 3 | | | III-1 | Cplx2 | 10814 | 12-May-15 |
| 5075 | 2 | 3 | | | III-1 | Cplx3 | 594855 | 4-May-15 |
| 5076 | 2 | 3 | | | III-1 | Cplx4 | 339302 | 4-May-15 |
| 5077 | 2 | 3 | | | III-1 | Cpm | 1368 | 12-May-15 |
| 5080 | 2 | 3 | | | III-1 | Cpne1 | 8904 | 3-May-15 |
| 5082 | 2 | 3 | | | III-1 | Cpne3 | 8895 | 3-May-15 |
| 5088 | 2 | 3 | | | III-1 | Cpne9 | 151835 | 12-May-15 |
| 5090 | 2 | 3 | | | III-1 | Cpped1 | 55313 | 4-May-15 |
| 5096 | 2 | 3 | | | III-1 | Cpsf3l | 54973 | 4-May-15 |
| 5099 | 2 | 3 | | | III-1 | Cpsf6 | 11052 | 1-Jun-15 |
| 5103 | 2 | 3 | | | III-1 | Cpt1c | 126129 | 28-May-15 |
| 5104 | 2 | 3 | | | III-1 | Cpt2 | 1376 | 23-May-15 |
| 5115 | 2 | 3 | | | III-1 | Cramp1l | 57585 | 4-May-15 |
| 5117 | 2 | 3 | | | III-1 | Crb1 | 23418 | 23-May-15 |
| 5120 | 2 | 3 | | | III-1 | Crbn | 51185 | 4-May-15 |
| 5123 | 2 | 3 | | | III-1 | Creb1 | 1385 | 2-Jun-15 |
| 5125 | 2 | 3 | | | III-1 | Creb3l1 | 90993 | |
| 5130 | 2 | 3 | | | III-1 | Crebbp | 1387 | 23-May-15 |
| 5131 | 2 | 3 | | | III-1 | Crebl2 | 1389 | 5-May-15 |
| 5133 | 2 | 3 | | | III-1 | Crebzf | 58487 | 4-May-15 |
| 5135 | 2 | 3 | | | III-1 | Creg2 | 200407 | 4-May-15 |
| 5139 | 2 | 3 | | | III-1 | Crh | 1392 | 4-May-15 |
| 5147 | 2 | 3 | | | III-1 | Cript | 9419 | 4-May-15 |
| 5150 | 2 | 3 | | | III-1 | Crisp3 | 10321 | |
| 5152 | 2 | 3 | | | III-1 | Crispld1 | 83690 | 4-May-15 |
| 5154 | 2 | 3 | | | III-1 | Crk | 1398 | 17-May-15 |
| 5157 | 2 | 3 | | | III-1 | Crlf2 | 64109 | 12-May-15 |
| 5160 | 2 | 3 | | | III-1 | Crmp1 | 1400 | 4-May-15 |
| 5161 | 2 | 3 | | | III-1 | Crnde | 643911 | 20-May-15 |
| 5165 | 2 | 3 | | | III-1 | Crot | 54677 | |
| 5167 | 2 | 3 | | | III-1 | Crtac1 | 55118 | 4-May-15 |
| 5170 | 2 | 3 | | | III-1 | Crtc1 | 23373 | 4-May-15 |
| 5171 | 2 | 3 | | | III-1 | Crtc2 | 200186 | 4-May-15 |
| 5176 | 2 | 3 | | | III-1 | Cry2 | 1408 | 20-May-15 |
| 5189 | 2 | 3 | | | III-1 | Crygd | 1421 | 4-May-15 |
| 5190 | 2 | 3 | | | III-1 | Cryge | | |
| 5195 | 2 | 3 | | | III-1 | Crym | 1428 | 23-May-15 |
| 5200 | 2 | 3 | | | III-1 | Csdc2 | 27254 | 4-May-15 |
| 5201 | 2 | 3 | | | III-1 | Csde1 | 7812 | 1-Jun-15 |

Fig.22 - 64

| | | | | | | | | |
|---|---|---|---|---|---|---|---|---|
| 5204 | 2 | 3 | | | III-1 | Csf1r | 1436 | 24-May-15 |
| 5205 | 2 | 3 | | | III-1 | Csf2 | 1437 | 4-May-15 |
| 5208 | 2 | 3 | | | III-1 | Csf2rb2 | 23772 | |
| 5209 | 2 | 3 | | | III-1 | Csf3 | 1440 | 12-May-15 |
| 5211 | 2 | 3 | | | III-1 | Csgalnact1 | 55790 | |
| 5215 | 2 | 3 | | | III-1 | Csmd1 | 64478 | 12-May-15 |
| 5232 | 2 | 3 | | | III-1 | Csnk2b | 1460 | 21-May-15 |
| 5239 | 2 | 3 | | | III-1 | Csrnp2 | 81566 | 28-May-15 |
| 5240 | 2 | 3 | | | III-1 | Csrnp3 | 80034 | 28-May-15 |
| 5243 | 2 | 3 | | | III-1 | Csrp2bp | 57325 | 7-Jun-15 |
| 5248 | 2 | 3 | | | III-1 | Cst13 | | |
| 5253 | 2 | 3 | | | III-1 | Cst9 | 128822 | 4-May-15 |
| 5256 | 2 | 3 | | | III-1 | Cstb | 1476 | 23-May-15 |
| 5261 | 2 | 3 | | | III-1 | Cstl1 | 128817 | 12-May-15 |
| 5266 | 2 | 3 | | | III-1 | Ctbs | 1486 | 12-May-15 |
| 5267 | 2 | 3 | | | III-1 | Ctc1 | 80169 | 22-May-15 |
| 5278 | 2 | 3 | | | III-1 | Ctf2 | | |
| 5286 | 2 | 3 | | | III-1 | Ctnna1 | 1495 | 12-May-15 |
| 5290 | 2 | 3 | | | III-1 | Ctnnb1 | 1499 | 31-May-15 |
| 5292 | 2 | 3 | | | III-1 | Ctnnbl1 | 56259 | 12-May-15 |
| 5294 | 2 | 3 | | | III-1 | Ctnnd2 | 1501 | 29-May-15 |
| 5295 | 2 | 3 | | | III-1 | Ctns | 1497 | 23-May-15 |
| 5297 | 2 | 3 | | | III-1 | Ctps2 | 56474 | 4-May-15 |
| 5311 | 2 | 3 | | | III-1 | Ctsd | 1509 | 23-May-15 |
| 5314 | 2 | 3 | | | III-1 | Ctsg | 1511 | 12-May-15 |
| 5315 | 2 | 3 | | | III-1 | Ctsh | 1512 | 12-May-15 |
| 5316 | 2 | 3 | | | III-1 | Ctsj | | |
| 5319 | 2 | 3 | | | III-1 | Ctsll3 | 644021 | 4-May-15 |
| 5327 | 2 | 3 | | | III-1 | Cttn | 2017 | 17-May-15 |
| 5329 | 2 | 3 | | | III-1 | Cttnbp2nl | 55917 | 4-May-15 |
| 5330 | 2 | 3 | | | III-1 | Ctu1 | 90353 | 23-May-15 |
| 5331 | 2 | 3 | | | III-1 | Ctu2 | 348180 | 4-May-15 |
| 5333 | 2 | 3 | | | III-1 | Ctxn2 | 399697 | 4-May-15 |
| 5337 | 2 | 3 | | | III-1 | Cuedc2 | 79004 | 4-May-15 |
| 5346 | 2 | 3 | | | III-1 | Cuta | 51596 | 12-May-15 |
| 5348 | 2 | 3 | | | III-1 | Cutc | 51076 | 4-May-15 |
| 5349 | 2 | 3 | | | III-1 | Cux1 | 1523 | 28-May-15 |
| 5353 | 2 | 3 | | | III-1 | Cwc22 | 57703 | 4-May-15 |
| 5357 | 2 | 3 | | | III-1 | Cwf19l2 | 143884 | 4-May-15 |
| 5365 | 2 | 3 | | | III-1 | Cxcl12 | 6387 | 17-May-15 |
| 5368 | 2 | 3 | | | III-1 | Cxcl15 | | |
| 5370 | 2 | 3 | | | III-1 | Cxcl17 | 284340 | 4-May-15 |
| 5377 | 2 | 3 | | | III-1 | Cxcr3 | 2833 | 12-May-15 |
| 5381 | 2 | 3 | | | III-1 | Cxx1a | | |
| 5389 | 2 | 3 | | | III-1 | Cyb561a3 | 220002 | 4-May-15 |
| 5392 | 2 | 3 | | | III-1 | Cyb5b | 80777 | 4-May-15 |
| 5395 | 2 | 3 | | | III-1 | Cyb5r1 | 51706 | 4-May-15 |
| 5396 | 2 | 3 | | | III-1 | Cyb5r2 | 51700 | 12-May-15 |
| 5397 | 2 | 3 | | | III-1 | Cyb5r3 | 1727 | 21-May-15 |
| 5398 | 2 | 3 | | | III-1 | Cyb5r4 | 51167 | 4-May-15 |
| 5402 | 2 | 3 | | | III-1 | Cybrd1 | 79901 | 12-May-15 |
| 5404 | 2 | 3 | | | III-1 | Cycs | 54205 | 12-May-15 |
| 5405 | 2 | 3 | | | III-1 | Cyct | | |
| 5409 | 2 | 3 | | | III-1 | Cyhr1 | 50626 | 4-May-15 |
| 5414 | 2 | 3 | | | III-1 | Cyp11a1 | 1583 | 12-May-15 |
| 5416 | 2 | 3 | | | III-1 | Cyp11b2 | 1585 | 24-May-15 |
| 5421 | 2 | 3 | | | III-1 | Cyp1b1 | 1545 | |
| 5422 | 2 | 3 | | | III-1 | Cyp20a1 | 57404 | 4-May-15 |
| 5423 | 2 | 3 | | | III-1 | Cyp21a1 | | |
| 5425 | 2 | 3 | | | III-1 | Cyp26a1 | 1592 | 17-May-15 |
| 5427 | 2 | 3 | | | III-1 | Cyp26c1 | 340665 | 4-May-15 |
| 5437 | 2 | 3 | | | III-1 | Cyp2b19 | | |
| 5438 | 2 | 3 | | | III-1 | Cyp2b23 | | |
| 5440 | 2 | 3 | | | III-1 | Cyp2c29 | | |
| 5450 | 2 | 3 | | | III-1 | Cyp2c65 | | |
| 5451 | 2 | 3 | | | III-1 | Cyp2c66 | | |
| 5452 | 2 | 3 | | | III-1 | Cyp2c67 | | |
| 5455 | 2 | 3 | | | III-1 | Cyp2c70 | | |
| 5462 | 2 | 3 | | | III-1 | Cyp2d34 | | |
| 5469 | 2 | 3 | | | III-1 | Cyp2j11 | | |
| 5474 | 2 | 3 | | | III-1 | Cyp2j8 | | |
| 5476 | 2 | 3 | | | III-1 | Cyp2r1 | 120227 | 31-May-15 |
| 5477 | 2 | 3 | | | III-1 | Cyp2s1 | 29785 | |
| 5478 | 2 | 3 | | | III-1 | Cyp2t4 | | |
| 5484 | 2 | 3 | | | III-1 | Cyp3a16 | | |
| 5489 | 2 | 3 | | | III-1 | Cyp3a57 | | |
| 5491 | 2 | 3 | | | III-1 | Cyp46a1 | 10858 | 4-May-15 |
| 5496 | 2 | 3 | | | III-1 | Cyp4a29 | | |
| 5499 | 2 | 3 | | | III-1 | Cyp4a32 | | |
| 5503 | 2 | 3 | | | III-1 | Cyp4f14 | | |
| 5505 | 2 | 3 | | | III-1 | Cyp4f16 | | |
| 5507 | 2 | 3 | | | III-1 | Cyp4f18 | | |
| 5509 | 2 | 3 | | | III-1 | Cyp4f39 | | |
| 5515 | 2 | 3 | | | III-1 | Cyp7a1 | 1581 | 12-May-15 |
| 5520 | 2 | 3 | | | III-1 | Cypt14 | | |
| 5526 | 2 | 3 | | | III-1 | Cypt8 | | |
| 5527 | 2 | 3 | | | III-1 | Cypt9 | | |
| 5529 | 2 | 3 | | | III-1 | Cys1 | 192668 | 4-May-15 |
| 5530 | 2 | 3 | | | III-1 | Cysltr1 | 10800 | 4-May-15 |
| 5531 | 2 | 3 | | | III-1 | Cysltr2 | 57105 | 9-May-15 |
| 5536 | 2 | 3 | | | III-1 | Cyth4 | 27128 | |
| 5550 | 2 | 3 | | | III-1 | D10Jhu81e | | |
| 5551 | 2 | 3 | | | III-1 | D10Wsu102e | | |
| 5570 | 2 | 3 | | | III-1 | D230025D16Rik | | |
| 5571 | 2 | 3 | | | III-1 | D230030E09Rik | | |
| 5572 | 2 | 3 | | | III-1 | D2hgdh | 728294 | 4-May-15 |
| 5577 | 2 | 3 | | | III-1 | D330050G23Rik | | |
| 5579 | 2 | 3 | | | III-1 | D3Bwg0562e | | |
| 5580 | 2 | 3 | | | III-1 | D3Ertd254e | | |
| 5586 | 2 | 3 | | | III-1 | D430042O09Rik | | |
| 5601 | 2 | 3 | | | III-1 | D630041G03Rik | | |
| 5604 | 2 | 3 | | | III-1 | D6Ertd474e | | |
| 5606 | 2 | 3 | | | III-1 | D6Wsu163e | | |
| 5609 | 2 | 3 | | | III-1 | D730045A05Rik | | |
| 5611 | 2 | 3 | | | III-1 | D730050B12Rik | | |
| 5615 | 2 | 3 | | | III-1 | D830005E20Rik | | |
| 5625 | 2 | 3 | | | III-1 | D930007P13Rik | | |
| 5628 | 2 | 3 | | | III-1 | D930016D06Rik | | |
| 5630 | 2 | 3 | | | III-1 | D930028M14Rik | | |
| 5631 | 2 | 3 | | | III-1 | D930032P07Rik | | |
| 5634 | 2 | 3 | | | III-1 | Daam2 | 23500 | 14-May-15 |
| 5636 | 2 | 3 | | | III-1 | Dab2 | 1601 | |
| 5637 | 2 | 3 | | | III-1 | Dab2ip | 153090 | 4-May-15 |
| 5638 | 2 | 3 | | | III-1 | Dach1 | 1602 | 23-May-15 |
| 5643 | 2 | 3 | | | III-1 | Dad1 | 1603 | 12-May-15 |
| 5644 | 2 | 3 | | | III-1 | Daf2 | | |
| 5645 | 2 | 3 | | | III-1 | Dag1 | 1605 | 28-May-15 |
| 5650 | 2 | 3 | | | III-1 | Dancr | 57291 | |
| 5651 | 2 | 3 | | | III-1 | Dand5 | 199699 | 4-May-15 |
| 5653 | 2 | 3 | | | III-1 | Dap | 1611 | /6/7 |
| 5657 | 2 | 3 | | | III-1 | Dapk3 | 1613 | 4-May-15 |
| 5660 | 2 | 3 | | | III-1 | Dars | 1615 | 4-May-15 |
| 5668 | 2 | 3 | | | III-1 | Dbh | 1621 | 23-May-15 |
| 5670 | 2 | 3 | | | III-1 | Dbi | 1622 | 7-Jun-15 |
| 5678 | 2 | 3 | | | III-1 | Dbr1 | 51163 | 4-May-15 |
| 5680 | 2 | 3 | | | III-1 | Dbx1 | 120237 | 4-May-15 |
| 5686 | 2 | 3 | | | III-1 | Dcaf12l2 | 340578 | 4-May-15 |
| 5688 | 2 | 3 | | | III-1 | Dcaf15 | 90379 | 4-May-15 |
| 5696 | 2 | 3 | | | III-1 | Dcbld1 | 285761 | 4-May-15 |
| 5697 | 2 | 3 | | | III-1 | Dcbld2 | 131566 | 4-May-15 |
| 5698 | 2 | 3 | | | III-1 | Dcc | 1630 | 17-May-15 |
| 5702 | 2 | 3 | | | III-1 | Dchs1 | 8642 | 4-May-15 |
| 5705 | 2 | 3 | | | III-1 | Dclk2 | 166614 | 4-May-15 |
| 5709 | 2 | 3 | | | III-1 | Dclre1c | 64421 | 23-May-15 |
| 5711 | 2 | 3 | | | III-1 | Dcp1a | 55802 | 4-May-15 |
| 5722 | 2 | 3 | | | III-1 | Dctn1 | 1639 | 23-May-15 |
| 5725 | 2 | 3 | | | III-1 | Dctn4 | 51164 | 4-May-15 |
| 5729 | 2 | 3 | | | III-1 | Dcun1d1 | 54165 | 2-Jun-15 |
| 5732 | 2 | 3 | | | III-1 | Dcun1d4 | 23142 | 4-May-15 |
| 5736 | 2 | 3 | | | III-1 | Dda1 | 79016 | 4-May-15 |
| 5741 | 2 | 3 | | | III-1 | Ddc | 1644 | 12-May-15 |
| 5742 | 2 | 3 | | | III-1 | Ddhd1 | 80821 | 28-May-15 |
| 5743 | 2 | 3 | | | III-1 | Ddhd2 | 23259 | 4-May-15 |
| 5744 | 2 | 3 | | | III-1 | Ddi1 | 414301 | 4-May-15 |
| 5753 | 2 | 3 | | | III-1 | Ddr2 | 4921 | 17-May-15 |
| 5756 | 2 | 3 | | | III-1 | Ddx1 | 1653 | 4-May-15 |
| 5761 | 2 | 3 | | | III-1 | Ddx19a | 55308 | 4-May-15 |
| 5769 | 2 | 3 | | | III-1 | Ddx27 | 55661 | 4-May-15 |
| 5770 | 2 | 3 | | | III-1 | Ddx28 | 55794 | 4-May-15 |
| 5776 | 2 | 3 | | | III-1 | Ddx4 | 54514 | 4-May-15 |
| 5783 | 2 | 3 | | | III-1 | Ddx5 | 1655 | 4-May-15 |
| 5786 | 2 | 3 | | | III-1 | Ddx52 | 11056 | 4-May-15 |
| 5790 | 2 | 3 | | | III-1 | Ddx58 | 23586 | 24-May-15 |
| 5791 | 2 | 3 | | | III-1 | Ddx59 | 83479 | 4-May-15 |
| 5798 | 2 | 3 | | | III-1 | Decr2 | 26063 | 4-May-15 |
| 5815 | 2 | 3 | | | III-1 | Defa6 | 1671 | 4-May-15 |
| 5817 | 2 | 3 | | | III-1 | Defa-ps12 | | |
| 5825 | 2 | 3 | | | III-1 | Defb13 | 245927 | 4-May-15 |
| 5841 | 2 | 3 | | | III-1 | Defb33 | | |
| 5853 | 2 | 3 | | | III-1 | Defb44-ps | | |
| 5855 | 2 | 3 | | | III-1 | Defb46 | | |
| 5858 | 2 | 3 | | | III-1 | Defb5 | | |
| 5864 | 2 | 3 | | | III-1 | Degs1 | 8560 | 12-May-15 |
| 5872 | 2 | 3 | | | III-1 | Dennd2d | 79961 | 4-May-15 |
| 5873 | 2 | 3 | | | III-1 | Dennd3 | 22898 | 12-May-15 |
| 5874 | 2 | 3 | | | III-1 | Dennd4a | 10260 | 12-May-15 |
| 5876 | 2 | 3 | | | III-1 | Dennd4c | 55667 | 4-May-15 |
| 5877 | 2 | 3 | | | III-1 | Dennd5a | 23258 | 4-May-15 |
| 5879 | 2 | 3 | | | III-1 | Dennd6a | 201627 | 4-May-15 |
| 5881 | 2 | 3 | | | III-1 | Denr | 8562 | 4-May-15 |
| 5886 | 2 | 3 | | | III-1 | Deptor | 64798 | 31-May-15 |
| 5887 | 2 | 3 | | | III-1 | Dera | 51071 | 4-May-15 |
| 5893 | 2 | 3 | | | III-1 | Desi2 | 51029 | 7-Jun-15 |
| 5897 | 2 | 3 | | | III-1 | Dffb | 1677 | 12-May-15 |
| 5899 | 2 | 3 | | | III-1 | Dfnb59 | 494513 | 23-May-15 |
| 5903 | 2 | 3 | | | III-1 | Dgcr14 | 8220 | 23-May-15 |
| 5906 | 2 | 3 | | | III-1 | Dgcr8 | 54487 | 23-May-15 |
| 5907 | 2 | 3 | | | III-1 | Dgka | 1606 | 12-May-15 |
| 5913 | 2 | 3 | | | III-1 | Dgkh | 160851 | 12-May-15 |
| 5918 | 2 | 3 | | | III-1 | Dguok | 1716 | 23-May-15 |
| 5923 | 2 | 3 | | | III-1 | Dhfr | 1719 | 12-May-15 |
| 5929 | 2 | 3 | | | III-1 | Dhrs13 | 147015 | 4-May-15 |
| 5930 | 2 | 3 | | | III-1 | Dhrs2 | 10202 | 4-May-15 |
| 5932 | 2 | 3 | | | III-1 | Dhrs4 | 10901 | 12-May-15 |
| 5939 | 2 | 3 | | | III-1 | Dhx15 | 1665 | 3-May-15 |
| 5942 | 2 | 3 | | | III-1 | Dhx30 | 22907 | 4-May-15 |
| 5955 | 2 | 3 | | | III-1 | Diablo | 56616 | 17-May-15 |
| 5957 | 2 | 3 | | | III-1 | Diap2 | | |
| 5959 | 2 | 3 | | | III-1 | Dicer1 | 23405 | 24-May-15 |
| 5961 | 2 | 3 | | | III-1 | Diexf | 27042 | 4-May-15 |
| 5967 | 2 | 3 | | | III-1 | Dip2a | 23181 | 4-May-15 |
| 5972 | 2 | 3 | | | III-1 | Dirc2 | 84925 | 4-May-15 |
| 5973 | 2 | 3 | | | III-1 | Dis3 | 22894 | 17-May-15 |
| 5974 | 2 | 3 | | | III-1 | Dis3l | 115752 | 12-May-15 |

Fig.22 - 65

| | | | | | | | | |
|---|---|---|---|---|---|---|---|---|
| 5978 | 2 | 3 | | | III-1 | Disp2 | 85455 | 4-May-15 |
| 5980 | 2 | 3 | | | III-1 | Dkc1 | 1736 | 31-May-15 |
| 5981 | 2 | 3 | | | III-1 | Dkk1 | 22943 | 31-May-15 |
| 5984 | 2 | 3 | | | III-1 | Dkk4 | 27121 | 4-May-15 |
| 5986 | 2 | 3 | | | III-1 | Dlat | 1737 | 29-May-15 |
| 5988 | 2 | 3 | | | III-1 | Dld | 1738 | 7-Jun-15 |
| 5994 | 2 | 3 | | | III-1 | Dlg3 | 1741 | 7-Jun-15 |
| 5998 | 2 | 3 | | | III-1 | Dlgap2 | 9228 | 4-May-15 |
| 6007 | 2 | 3 | | | III-1 | Dlst | 1743 | 4-May-15 |
| 6014 | 2 | 3 | | | III-1 | Dlx6 | 1750 | 4-May-15 |
| 6025 | 2 | 3 | | | III-1 | Dmpk | 1760 | 31-May-15 |
| 6026 | 2 | 3 | | | III-1 | Dmr | 91833 | 3-May-15 |
| 6029 | 2 | 3 | | | III-1 | Dmrt3 | 58524 | 4-May-15 |
| 6034 | 2 | 3 | | | III-1 | Dmrtc1b | 728656 | 4-May-15 |
| 6041 | 2 | 3 | | | III-1 | Dmxl2 | 23312 | 31-May-15 |
| 6043 | 2 | 3 | | | III-1 | Dnaaf1 | 123872 | 23-May-15 |
| 6046 | 2 | 3 | | | III-1 | Dnah1 | 25981 | 28-May-15 |
| 6063 | 2 | 3 | | | III-1 | Dnajb1 | 3337 | 12-May-15 |
| 6068 | 2 | 3 | | | III-1 | Dnajb2 | 3300 | 23-May-15 |
| 6070 | 2 | 3 | | | III-1 | Dnajb4 | 11080 | 4-May-15 |
| 6072 | 2 | 3 | | | III-1 | Dnajb6 | 10049 | 23-May-15 |
| 6075 | 2 | 3 | | | III-1 | Dnajb9 | 4189 | 4-May-15 |
| 6076 | 2 | 3 | | | III-1 | Dnajc1 | 64215 | 4-May-15 |
| 6078 | 2 | 3 | | | III-1 | Dnajc11 | 55735 | 4-May-15 |
| 6080 | 2 | 3 | | | III-1 | Dnajc13 | 23317 | 23-May-15 |
| 6083 | 2 | 3 | | | III-1 | Dnajc16 | 23341 | 4-May-15 |
| 6086 | 2 | 3 | | | III-1 | Dnajc19 | 131118 | 4-May-15 |
| 6088 | 2 | 3 | | | III-1 | Dnajc21 | 134218 | 4-May-15 |
| 6091 | 2 | 3 | | | III-1 | Dnajc25 | 548645 | 4-May-15 |
| 6093 | 2 | 3 | | | III-1 | Dnajc28 | 54943 | 4-May-15 |
| 6094 | 2 | 3 | | | III-1 | Dnajc3 | 5611 | 4-May-15 |
| 6097 | 2 | 3 | | | III-1 | Dnajc5 | 80331 | 12-May-15 |
| 6102 | 2 | 3 | | | III-1 | Dnajc8 | 22826 | 4-May-15 |
| 6110 | 2 | 3 | | | III-1 | Dnase1l3 | 1776 | 4-May-15 |
| 6111 | 2 | 3 | | | III-1 | Dnase2a | 1777 | 12-May-15 |
| 6112 | 2 | 3 | | | III-1 | Dnase2b | 58511 | 4-May-15 |
| 6114 | 2 | 3 | | | III-1 | Dner | 92737 | 4-May-15 |
| 6117 | 2 | 3 | | | III-1 | Dnm1l | 10059 | 31-May-15 |
| 6121 | 2 | 3 | | | III-1 | Dnmbp | 23268 | 4-May-15 |
| 6122 | 2 | 3 | | | III-1 | Dnmt1 | 1786 | 22-May-15 |
| 6127 | 2 | 3 | | | III-1 | Dnpep | 23549 | 4-May-15 |
| 6131 | 2 | 3 | | | III-1 | Dnttip2 | 30836 | 4-May-15 |
| 6136 | 2 | 3 | | | III-1 | Dock10 | 55619 | 4-May-15 |
| 6139 | 2 | 3 | | | III-1 | Dock3 | 1795 | 12-May-15 |
| 6141 | 2 | 3 | | | III-1 | Dock5 | 80005 | 12-May-15 |
| 6143 | 2 | 3 | | | III-1 | Dock7 | 85440 | 4-May-15 |
| 6144 | 2 | 3 | | | III-1 | Dock8 | 81704 | 4-May-15 |
| 6148 | 2 | 3 | | | III-1 | Dok2 | 9046 | 4-May-15 |
| 6151 | 2 | 3 | | | III-1 | Dok5 | 55816 | 4-May-15 |
| 6154 | 2 | 3 | | | III-1 | Dolk | 22845 | 23-May-15 |
| 6157 | 2 | 3 | | | III-1 | Dopey1 | 23033 | 4-May-15 |
| 6160 | 2 | 3 | | | III-1 | Dot1l | 84444 | 4-May-15 |
| 6161 | 2 | 3 | | | III-1 | Doxl2 | | |
| 6164 | 2 | 3 | | | III-1 | Dpcr1 | 135656 | 4-May-15 |
| 6166 | 2 | 3 | | | III-1 | Dpep2 | 64174 | 4-May-15 |
| 6172 | 2 | 3 | | | III-1 | Dph2 | 1802 | 4-May-15 |
| 6178 | 2 | 3 | | | III-1 | Dpm2 | 8818 | 23-May-15 |
| 6183 | 2 | 3 | | | III-1 | Dpp6 | 1804 | 12-May-15 |
| 6184 | 2 | 3 | | | III-1 | Dpp7 | 29952 | 4-May-15 |
| 6190 | 2 | 3 | | | III-1 | Dppa4 | 55211 | 4-May-15 |
| 6192 | 2 | 3 | | | III-1 | Dpt | 1805 | 4-May-15 |
| 6200 | 2 | 3 | | | III-1 | Dpysl2 | 1808 | 4-May-15 |
| 6202 | 2 | 3 | | | III-1 | Dpysl4 | 10570 | 4-May-15 |
| 6203 | 2 | 3 | | | III-1 | Dpysl5 | 56896 | 4-May-15 |
| 6204 | 2 | 3 | | | III-1 | DQ267100 | | |
| 6208 | 2 | 3 | | | III-1 | Dr1 | 1810 | 24-May-15 |
| 6211 | 2 | 3 | | | III-1 | Drap1 | 10589 | 4-May-15 |
| 6212 | 2 | 3 | | | III-1 | Draxin | 374946 | 4-May-15 |
| 6220 | 2 | 3 | | | III-1 | Drg1 | 4733 | 7-Jun-15 |
| 6223 | 2 | 3 | | | III-1 | Drp2 | 1821 | 7-Jun-15 |
| 6226 | 2 | 3 | | | III-1 | Dsc3 | 1825 | 13-Jun-15 |
| 6227 | 2 | 3 | | | III-1 | Dscam | 1826 | 4-May-15 |
| 6233 | 2 | 3 | | | III-1 | Dsg1a | | |
| 6237 | 2 | 3 | | | III-1 | Dsg3 | 1830 | 12-May-15 |
| 6241 | 2 | 3 | | | III-1 | Dspp | 1834 | 12-May-15 |
| 6246 | 2 | 3 | | | III-1 | Dtd2 | 112487 | 4-May-15 |
| 6251 | 2 | 3 | | | III-1 | Dtnbp1 | 84062 | 31-May-15 |
| 6253 | 2 | 3 | | | III-1 | Dtwd2 | 285605 | 12-May-15 |
| 6255 | 2 | 3 | | | III-1 | Dtx2 | 113878 | 4-May-15 |
| 6262 | 2 | 3 | | | III-1 | Duoxa1 | 90527 | 20-May-15 |
| 6266 | 2 | 3 | | | III-1 | Dus2 | 54920 | 4-May-15 |
| 6276 | 2 | 3 | | | III-1 | Dusp16 | 80824 | 4-May-15 |
| 6278 | 2 | 3 | | | III-1 | Dusp19 | 142679 | 4-May-15 |
| 6282 | 2 | 3 | | | III-1 | Dusp23 | 54935 | 7-Jun-15 |
| 6286 | 2 | 3 | | | III-1 | Dusp3 | 1845 | 4-May-15 |
| 6289 | 2 | 3 | | | III-1 | Dusp6 | 1848 | 4-May-15 |
| 6292 | 2 | 3 | | | III-1 | Dusp9 | 1852 | 4-May-15 |
| 6296 | 2 | 3 | | | III-1 | Duxbl2 | | |
| 6300 | 2 | 3 | | | III-1 | Dvl3 | 1857 | 12-May-15 |
| 6302 | 2 | 3 | | | III-1 | Dxo | 1797 | 12-May-15 |
| 6305 | 2 | 3 | | | III-1 | Dym | 54808 | 4-May-15 |
| 6308 | 2 | 3 | | | III-1 | Dync1i1 | 1780 | 12-May-15 |
| 6315 | 2 | 3 | | | III-1 | Dynll2 | 140735 | 4-May-15 |
| 6318 | 2 | 3 | | | III-1 | Dynlt1a | | |
| 6320 | 2 | 3 | | | III-1 | Dynlt1c | | |
| 6327 | 2 | 3 | | | III-1 | Dyrk4 | 8798 | 4-May-15 |
| 6331 | 2 | 3 | | | III-1 | Dzank1 | 55184 | 28-May-15 |

| | | | | | | | | |
|---|---|---|---|---|---|---|---|---|
| 6334 | 2 | 3 | | | III-1 | Dzip3 | 9666 | 21-May-15 |
| 6338 | 2 | 3 | | | III-1 | E030013I19Rik | | |
| 6343 | 2 | 3 | | | III-1 | E030025P04Rik | | |
| 6349 | 2 | 3 | | | III-1 | E130018N17Rik | | |
| 6350 | 2 | 3 | | | III-1 | E130102H24Rik | | |
| 6354 | 2 | 3 | | | III-1 | E130215H24Rik | | |
| 6355 | 2 | 3 | | | III-1 | E130218I03Rik | | |
| 6358 | 2 | 3 | | | III-1 | E130308A19Rik | | |
| 6359 | 2 | 3 | | | III-1 | E130309D02Rik | | |
| 6365 | 2 | 3 | | | III-1 | E230008N13Rik | | |
| 6367 | 2 | 3 | | | III-1 | E230016M11Rik | | |
| 6368 | 2 | 3 | | | III-1 | E230019M04Rik | | |
| 6374 | 2 | 3 | | | III-1 | E2f4 | 1874 | 4-May-15 |
| 6377 | 2 | 3 | | | III-1 | E2f7 | 144455 | 4-May-15 |
| 6381 | 2 | 3 | | | III-1 | E330012B07Rik | | |
| 6383 | 2 | 3 | | | III-1 | E330014E10Rik | | |
| 6386 | 2 | 3 | | | III-1 | E330020D12Rik | | |
| 6399 | 2 | 3 | | | III-1 | Eapp | 55837 | 12-May-15 |
| 6400 | 2 | 3 | | | III-1 | Ear1 | 9572 | 12-May-15 |
| 6401 | 2 | 3 | | | III-1 | Ear10 | | |
| 6407 | 2 | 3 | | | III-1 | Ear7 | 7067 | 12-May-15 |
| 6410 | 2 | 3 | | | III-1 | Ebf1 | 1879 | 12-May-15 |
| 6411 | 2 | 3 | | | III-1 | Ebf2 | 64641 | 4-May-15 |
| 6413 | 2 | 3 | | | III-1 | Ebf4 | 57593 | 4-May-15 |
| 6414 | 2 | 3 | | | III-1 | Ebi3 | 10148 | 12-May-15 |
| 6418 | 2 | 3 | | | III-1 | Ecd | 11319 | 7-Jun-15 |
| 6421 | 2 | 3 | | | III-1 | Ecel1 | 9427 | 12-May-15 |
| 6426 | 2 | 3 | | | III-1 | Echs1 | 1892 | 31-May-15 |
| 6427 | 2 | 3 | | | III-1 | Eci1 | 1632 | 4-May-15 |
| 6428 | 2 | 3 | | | III-1 | Eci2 | 10455 | 4-May-15 |
| 6430 | 2 | 3 | | | III-1 | Ecm1 | 1893 | 12-May-15 |
| 6434 | 2 | 3 | | | III-1 | Ect2 | 1894 | 4-May-15 |
| 6437 | 2 | 3 | | | III-1 | Eda2r | 60401 | 4-May-15 |
| 6438 | 2 | 3 | | | III-1 | Edar | 10913 | 23-May-15 |
| 6449 | 2 | 3 | | | III-1 | Edn2 | 1907 | 4-May-15 |
| 6452 | 2 | 3 | | | III-1 | Ednrb | 1910 | 24-May-15 |
| 6453 | 2 | 3 | | | III-1 | Edrf1 | 26098 | 4-May-15 |
| 6456 | 2 | 3 | | | III-1 | Eef1a1 | 1915 | 24-May-15 |
| 6459 | 2 | 3 | | | III-1 | Eef1d | 1936 | 4-May-15 |
| 6460 | 2 | 3 | | | III-1 | Eef1e1 | 9521 | 4-May-15 |
| 6461 | 2 | 3 | | | III-1 | Eef1g | 1937 | 4-May-15 |
| 6464 | 2 | 3 | | | III-1 | Eefsec | 60678 | 4-May-15 |
| 6466 | 2 | 3 | | | III-1 | Efcab1 | 79645 | 4-May-15 |
| 6468 | 2 | 3 | | | III-1 | Efcab11 | 90141 | 4-May-15 |
| 6472 | 2 | 3 | | | III-1 | Efcab3 | 146779 | 4-May-15 |
| 6489 | 2 | 3 | | | III-1 | Efna2 | 1943 | 12-May-15 |
| 6490 | 2 | 3 | | | III-1 | Efna3 | 1944 | 4-May-15 |
| 6493 | 2 | 3 | | | III-1 | Efnb1 | 1947 | 12-May-15 |
| 6494 | 2 | 3 | | | III-1 | Efnb2 | 1948 | 4-May-15 |
| 6499 | 2 | 3 | | | III-1 | Eftud1 | 79631 | 4-May-15 |
| 6506 | 2 | 3 | | | III-1 | Egfl8 | 80864 | 12-May-15 |
| 6507 | 2 | 3 | | | III-1 | Egflam | 133584 | 4-May-15 |
| 6515 | 2 | 3 | | | III-1 | Egr4 | 1961 | 4-May-15 |
| 6517 | 2 | 3 | | | III-1 | Ehbp1l1 | 254102 | 12-May-15 |
| 6518 | 2 | 3 | | | III-1 | Ehd1 | 10938 | 10-May-15 |
| 6520 | 2 | 3 | | | III-1 | Ehd3 | 30845 | 4-May-15 |
| 6521 | 2 | 3 | | | III-1 | Ehd4 | 30844 | 4-May-15 |
| 6524 | 2 | 3 | | | III-1 | Ehmt1 | 79813 | 23-May-15 |
| 6531 | 2 | 3 | | | III-1 | Eif1 | 10209 | 4-May-15 |
| 6532 | 2 | 3 | | | III-1 | Eif1a | 1964 | 7-Jun-15 |
| 6537 | 2 | 3 | | | III-1 | Eif2ak1 | 27102 | 7-Jun-15 |
| 6539 | 2 | 3 | | | III-1 | Eif2ak3 | 9451 | 31-May-15 |
| 6541 | 2 | 3 | | | III-1 | Eif2b1 | 1967 | 23-May-15 |
| 6544 | 2 | 3 | | | III-1 | Eif2b4 | 8890 | 23-May-15 |
| 6556 | 2 | 3 | | | III-1 | Eif3f | 8665 | 4-May-15 |
| 6557 | 2 | 3 | | | III-1 | Eif3g | 8666 | 12-May-15 |
| 6563 | 2 | 3 | | | III-1 | Eif3l | 51386 | 4-May-15 |
| 6565 | 2 | 3 | | | III-1 | Eif4a1 | 1973 | 17-May-15 |
| 6566 | 2 | 3 | | | III-1 | Eif4a2 | 1974 | 4-May-15 |
| 6574 | 2 | 3 | | | III-1 | Eif4ebp2 | 1979 | 4-May-15 |
| 6575 | 2 | 3 | | | III-1 | Eif4ebp3 | 8637 | 4-May-15 |
| 6585 | 2 | 3 | | | III-1 | Eif6 | 3692 | 12-May-15 |
| 6591 | 2 | 3 | | | III-1 | Elavl3 | 1995 | 2-Jun-15 |
| 6594 | 2 | 3 | | | III-1 | Elf2 | 1998 | 28-May-15 |
| 6596 | 2 | 3 | | | III-1 | Elf4 | 2000 | 28-May-15 |
| 6610 | 2 | 3 | | | III-1 | Elmod2 | 255520 | 4-May-15 |
| 6612 | 2 | 3 | | | III-1 | Elmsan1 | 91748 | 9-May-15 |
| 6619 | 2 | 3 | | | III-1 | Elovl5 | 60481 | 21-May-15 |
| 6622 | 2 | 3 | | | III-1 | Elp2 | 55250 | 4-May-15 |
| 6625 | 2 | 3 | | | III-1 | Elp5 | 23587 | 4-May-15 |
| 6629 | 2 | 3 | | | III-1 | Emc1 | 23065 | 4-May-15 |
| 6632 | 2 | 3 | | | III-1 | Emc3 | 55831 | 21-May-15 |
| 6638 | 2 | 3 | | | III-1 | Emcn | 51705 | 4-May-15 |
| 6639 | 2 | 3 | | | III-1 | Emd | 2010 | 23-May-15 |
| 6642 | 2 | 3 | | | III-1 | Emg1 | 10436 | 14-May-15 |
| 6646 | 2 | 3 | | | III-1 | Emilin3 | 90187 | 7-Jun-15 |
| 6651 | 2 | 3 | | | III-1 | Eml5 | 161436 | 12-May-15 |
| 6660 | 2 | 3 | | | III-1 | Emx2os | 196047 | 12-May-15 |
| 6663 | 2 | 3 | | | III-1 | Enah | 55740 | 31-May-15 |
| 6664 | 2 | 3 | | | III-1 | Enam | 10117 | 12-May-15 |
| 6667 | 2 | 3 | | | III-1 | Endog | 2021 | 4-May-15 |
| 6668 | 2 | 3 | | | III-1 | Endou | 8909 | 23-May-15 |
| 6671 | 2 | 3 | | | III-1 | Engase | 64772 | 4-May-15 |
| 6673 | 2 | 3 | | | III-1 | Enkd1 | 84080 | 4-May-15 |
| 6677 | 2 | 3 | | | III-1 | Eno2 | 2026 | 4-May-15 |
| 6681 | 2 | 3 | | | III-1 | Enox1 | 55068 | 4-May-15 |
| 6686 | 2 | 3 | | | III-1 | Enpp3 | 5169 | 4-May-15 |

Fig.22 - 66

| | | | | | | | | |
|---|---|---|---|---|---|---|---|---|
| 6687 | 2 | 3 | | | III-1 | Enpp4 | 22875 | 4-May-15 |
| 6689 | 2 | 3 | | | III-1 | Enpp6 | 133121 | 12-May-15 |
| 6691 | 2 | 3 | | | III-1 | Ensa | 2029 | 4-May-15 |
| 6695 | 2 | 3 | | | III-1 | Entpd2 | 954 | 4-May-15 |
| 6697 | 2 | 3 | | | III-1 | Entpd4 | 9583 | 21-May-15 |
| 6698 | 2 | 3 | | | III-1 | Entpd5 | 957 | 12-May-15 |
| 6699 | 2 | 3 | | | III-1 | Entpd6 | 955 | 4-May-15 |
| 6701 | 2 | 3 | | | III-1 | Entpd8 | 377841 | 4-May-15 |
| 6704 | 2 | 3 | | | III-1 | Eomes | 8320 | 12-May-15 |
| 6705 | 2 | 3 | | | III-1 | Ep300 | 2033 | 29-May-15 |
| 6706 | 2 | 3 | | | III-1 | Ep400 | 57634 | 4-May-15 |
| 6708 | 2 | 3 | | | III-1 | Epb4.1 | | |
| 6710 | 2 | 3 | | | III-1 | Epb4.1l2 | | |
| 6712 | 2 | 3 | | | III-1 | Epb4.1l4a | | |
| 6714 | 2 | 3 | | | III-1 | Epb4.1l5 | | |
| 6717 | 2 | 3 | | | III-1 | Epc2 | 26122 | 4-May-15 |
| 6720 | 2 | 3 | | | III-1 | Epg5 | 57724 | 21-May-15 |
| 6723 | 2 | 3 | | | III-1 | Epha10 | 284656 | 4-May-15 |
| 6725 | 2 | 3 | | | III-1 | Epha3 | 2042 | 4-May-15 |
| 6728 | 2 | 3 | | | III-1 | Epha6 | 285220 | 4-May-15 |
| 6734 | 2 | 3 | | | III-1 | Ephb4 | 2050 | 31-May-15 |
| 6742 | 2 | 3 | | | III-1 | Epn1 | 29924 | 3-May-15 |
| 6745 | 2 | 3 | | | III-1 | Epo | 2056 | 7-Jun-15 |
| 6749 | 2 | 3 | | | III-1 | Eprs | 2058 | 4-May-15 |
| 6751 | 2 | 3 | | | III-1 | Eps15l1 | 58513 | 3-May-15 |
| 6753 | 2 | 3 | | | III-1 | Eps8l1 | 54869 | 4-May-15 |
| 6755 | 2 | 3 | | | III-1 | Eps8l3 | 79574 | 12-May-15 |
| 6757 | 2 | 3 | | | III-1 | Ept1 | 85465 | 28-May-15 |
| 6765 | 2 | 3 | | | III-1 | Erbb2ip | 55914 | 28-May-15 |
| 6779 | 2 | 3 | | | III-1 | Erdr1 | | |
| 6781 | 2 | 3 | | | III-1 | Erf | 2077 | 7-Jun-15 |
| 6782 | 2 | 3 | | | III-1 | Erg | 2078 | 31-May-15 |
| 6783 | 2 | 3 | | | III-1 | Ergic1 | 57222 | 28-May-15 |
| 6787 | 2 | 3 | | | III-1 | Eri1 | 90459 | 4-May-15 |
| 6790 | 2 | 3 | | | III-1 | Erich1 | 157697 | 14-May-15 |
| 6793 | 2 | 3 | | | III-1 | Erich4 | 100170765 | 4-May-15 |
| 6794 | 2 | 3 | | | III-1 | Erich5 | 203111 | 4-May-15 |
| 6797 | 2 | 3 | | | III-1 | Erlin1 | 10613 | 28-May-15 |
| 6801 | 2 | 3 | | | III-1 | Ermn | 57471 | 14-May-15 |
| 6805 | 2 | 3 | | | III-1 | Ero1l | 30001 | 23-May-15 |
| 6806 | 2 | 3 | | | III-1 | Ero1lb | 56605 | 4-May-15 |
| 6807 | 2 | 3 | | | III-1 | Erp27 | 121506 | 4-May-15 |
| 6811 | 2 | 3 | | | III-1 | Erv3 | 2086 | 21-May-15 |
| 6816 | 2 | 3 | | | III-1 | Esf1 | 51575 | 4-May-15 |
| 6834 | 2 | 3 | | | III-1 | Espl1 | 9700 | 4-May-15 |
| 6836 | 2 | 3 | | | III-1 | Espnl | 339768 | 12-May-15 |
| 6838 | 2 | 3 | | | III-1 | Esr2 | 2100 | 31-May-15 |
| 6841 | 2 | 3 | | | III-1 | Esrra | 2101 | 10-May-15 |
| 6844 | 2 | 3 | | | III-1 | Esx1 | 80712 | 12-May-15 |
| 6848 | 2 | 3 | | | III-1 | Etaa1 | 54465 | 4-May-15 |
| 6852 | 2 | 3 | | | III-1 | Etfb | 2109 | 12-May-15 |
| 6855 | 2 | 3 | | | III-1 | Etl4 | 100144434 | 7-Dec-14 |
| 6856 | 2 | 3 | | | III-1 | Etnk1 | 55500 | 4-May-15 |
| 6857 | 2 | 3 | | | III-1 | Etnk2 | 55224 | 12-May-15 |
| 6859 | 2 | 3 | | | III-1 | Etohd2 | | |
| 6860 | 2 | 3 | | | III-1 | Etohi1 | | |
| 6861 | 2 | 3 | | | III-1 | Ets1 | 2113 | 21-May-15 |
| 6862 | 2 | 3 | | | III-1 | Ets2 | 2114 | 12-May-15 |
| 6864 | 2 | 3 | | | III-1 | Etv2 | 2116 | 28-May-15 |
| 6868 | 2 | 3 | | | III-1 | Etv6 | 2120 | 31-May-15 |
| 6869 | 2 | 3 | | | III-1 | EU599041 | | |
| 6871 | 2 | 3 | | | III-1 | Eva1b | 55194 | 28-May-15 |
| 6880 | 2 | 3 | | | III-1 | Evl | 51466 | 12-May-15 |
| 6882 | 2 | 3 | | | III-1 | Evx1 | 2128 | 4-May-15 |
| 6883 | 2 | 3 | | | III-1 | Evx2 | 344191 | 4-May-15 |
| 6895 | 2 | 3 | | | III-1 | Exoc5 | 10640 | 4-May-15 |
| 6902 | 2 | 3 | | | III-1 | Exosc10 | 5394 | 12-May-15 |
| 6908 | 2 | 3 | | | III-1 | Exosc7 | 23016 | 4-May-15 |
| 6910 | 2 | 3 | | | III-1 | Exosc9 | 5393 | 4-May-15 |
| 6911 | 2 | 3 | | | III-1 | Exph5 | 23086 | 12-May-15 |
| 6912 | 2 | 3 | | | III-1 | Ext1 | 2131 | 23-May-15 |
| 6918 | 2 | 3 | | | III-1 | Eya2 | 2139 | 4-May-15 |
| 6919 | 2 | 3 | | | III-1 | Eya3 | 2140 | 4-May-15 |
| 6921 | 2 | 3 | | | III-1 | Ezh1 | 2145 | 13-Jun-15 |
| 6922 | 2 | 3 | | | III-1 | Ezh2 | 2146 | 23-May-15 |
| 6924 | 2 | 3 | | | III-1 | F10 | 2159 | 7-Jun-15 |
| 6925 | 2 | 3 | | | III-1 | F11 | 2160 | 7-Jun-15 |
| 6929 | 2 | 3 | | | III-1 | F13b | 2165 | 12-May-15 |
| 6933 | 2 | 3 | | | III-1 | F2rl2 | 2151 | 4-May-15 |
| 6934 | 2 | 3 | | | III-1 | F2rl3 | 9002 | 17-May-15 |
| 6936 | 2 | 3 | | | III-1 | F420014N23Rik | | |
| 6937 | 2 | 3 | | | III-1 | F5 | 2153 | 23-May-15 |
| 6939 | 2 | 3 | | | III-1 | F630042J09Rik | | |
| 6943 | 2 | 3 | | | III-1 | F730035M05Rik | | |
| 6945 | 2 | 3 | | | III-1 | F8 | 2157 | 23-May-15 |
| 6956 | 2 | 3 | | | III-1 | Fabp2 | 2169 | 17-May-15 |
| 6963 | 2 | 3 | | | III-1 | Fadd | 8772 | 31-May-15 |
| 6971 | 2 | 3 | | | III-1 | Fahd1 | 81889 | 10-May-15 |
| 6972 | 2 | 3 | | | III-1 | Fahd2a | 51011 | 4-May-15 |
| 6973 | 2 | 3 | | | III-1 | Faim | 55179 | 4-May-15 |
| 6979 | 2 | 3 | | | III-1 | Fam102b | 284611 | 4-May-15 |
| 6984 | 2 | 3 | | | III-1 | Fam107b | 83641 | 4-May-15 |
| 6990 | 2 | 3 | | | III-1 | Fam111a | 63901 | 4-May-15 |
| 6991 | 2 | 3 | | | III-1 | Fam114a1 | 92689 | 4-May-15 |
| 6994 | 2 | 3 | | | III-1 | Fam115c | 285966 | 4-May-15 |
| 6995 | 2 | 3 | | | III-1 | Fam115e | | |
| 6997 | 2 | 3 | | | III-1 | Fam117b | 150864 | 4-May-15 |
| 6998 | 2 | 3 | | | III-1 | Fam118a | 55007 | 4-May-15 |
| 6999 | 2 | 3 | | | III-1 | Fam118b | 79607 | 4-May-15 |
| 7008 | 2 | 3 | | | III-1 | Fam124b | 79843 | 4-May-15 |
| 7010 | 2 | 3 | | | III-1 | Fam126b | 285172 | 4-May-15 |
| 7011 | 2 | 3 | | | III-1 | Fam129a | 116496 | 12-May-15 |
| 7012 | 2 | 3 | | | III-1 | Fam129b | 64855 | 4-May-15 |
| 7013 | 2 | 3 | | | III-1 | Fam129c | 199786 | 4-May-15 |
| 7015 | 2 | 3 | | | III-1 | Fam131b | 9715 | 28-May-15 |
| 7016 | 2 | 3 | | | III-1 | Fam131c | 348487 | 4-May-15 |
| 7020 | 2 | 3 | | | III-1 | Fam134a | 79137 | 4-May-15 |
| 7022 | 2 | 3 | | | III-1 | Fam134c | 162427 | 12-May-15 |
| 7027 | 2 | 3 | | | III-1 | Fam13b | 51306 | 28-May-15 |
| 7028 | 2 | 3 | | | III-1 | Fam13c | 220965 | 4-May-15 |
| 7030 | 2 | 3 | | | III-1 | Fam149b | | |
| 7041 | 2 | 3 | | | III-1 | Fam160a2 | 84067 | 4-May-15 |
| 7045 | 2 | 3 | | | III-1 | Fam161b | 145483 | 12-May-15 |
| 7046 | 2 | 3 | | | III-1 | Fam162a | 26355 | 4-May-15 |
| 7047 | 2 | 3 | | | III-1 | Fam162b | 221303 | 4-May-15 |
| 7053 | 2 | 3 | | | III-1 | Fam167b | 84734 | 4-May-15 |
| 7054 | 2 | 3 | | | III-1 | Fam168a | 23201 | 1-Jun-15 |
| 7057 | 2 | 3 | | | III-1 | Fam169b | 283777 | 4-May-15 |
| 7058 | 2 | 3 | | | III-1 | Fam170a | 340069 | 4-May-15 |
| 7062 | 2 | 3 | | | III-1 | Fam171b | 165215 | 4-May-15 |
| 7066 | 2 | 3 | | | III-1 | Fam174a | 345757 | 4-May-15 |
| 7071 | 2 | 3 | | | III-1 | Fam178b | 51252 | 4-May-15 |
| 7073 | 2 | 3 | | | III-1 | Fam179b | 23116 | 12-May-15 |
| 7074 | 2 | 3 | | | III-1 | Fam180a | 389558 | 4-May-15 |
| 7075 | 2 | 3 | | | III-1 | Fam181a | 90050 | 4-May-15 |
| 7080 | 2 | 3 | | | III-1 | Fam185a | 222234 | 4-May-15 |
| 7084 | 2 | 3 | | | III-1 | Fam188a | 80013 | 4-May-15 |
| 7086 | 2 | 3 | | | III-1 | Fam189a1 | 23359 | 4-May-15 |
| 7088 | 2 | 3 | | | III-1 | Fam189b | 10712 | 4-May-15 |
| 7093 | 2 | 3 | | | III-1 | Fam195b | 348262 | 4-May-15 |
| 7098 | 2 | 3 | | | III-1 | Fam199x | 139231 | 12-May-15 |
| 7099 | 2 | 3 | | | III-1 | Fam19a1 | 407738 | 4-May-15 |
| 7104 | 2 | 3 | | | III-1 | Fam203a | 51236 | 4-May-15 |
| 7105 | 2 | 3 | | | III-1 | Fam204a | 63877 | 4-May-15 |
| 7112 | 2 | 3 | | | III-1 | Fam20b | 9917 | 23-May-15 |
| 7116 | 2 | 3 | | | III-1 | Fam210b | 116151 | 4-May-15 |
| 7117 | 2 | 3 | | | III-1 | Fam212a | 389119 | 4-May-15 |
| 7123 | 2 | 3 | | | III-1 | Fam216a | 29902 | 4-May-15 |
| 7129 | 2 | 3 | | | III-1 | Fam219b | 57184 | 4-May-15 |
| 7131 | 2 | 3 | | | III-1 | Fam221a | 340277 | 4-May-15 |
| 7134 | 2 | 3 | | | III-1 | Fam222b | 55731 | 4-May-15 |
| 7140 | 2 | 3 | | | III-1 | Fam229b | 619208 | 4-May-15 |
| 7141 | 2 | 3 | | | III-1 | Fam24a | 118670 | 4-May-15 |
| 7145 | 2 | 3 | | | III-1 | Fam26f | 441168 | 4-May-15 |
| 7148 | 2 | 3 | | | III-1 | Fam3a | 60343 | 4-May-15 |
| 7152 | 2 | 3 | | | III-1 | Fam43b | 163933 | 4-May-15 |
| 7161 | 2 | 3 | | | III-1 | Fam49b | 51571 | 4-May-15 |
| 7162 | 2 | 3 | | | III-1 | Fam50a | 9130 | 21-May-15 |
| 7171 | 2 | 3 | | | III-1 | Fam63a | 55793 | 4-May-15 |
| 7172 | 2 | 3 | | | III-1 | Fam63b | 54629 | 4-May-15 |
| 7182 | 2 | 3 | | | III-1 | Fam71d | 161142 | 4-May-15 |
| 7184 | 2 | 3 | | | III-1 | Fam71e2 | 284418 | 4-May-15 |
| 7187 | 2 | 3 | | | III-1 | Fam72a | 729533 | 4-May-15 |
| 7189 | 2 | 3 | | | III-1 | Fam73b | 84895 | 4-May-15 |
| 7197 | 2 | 3 | | | III-1 | Fam83c | 128876 | 4-May-15 |
| 7199 | 2 | 3 | | | III-1 | Fam83e | 54854 | 4-May-15 |
| 7205 | 2 | 3 | | | III-1 | Fam86 | | |
| 7209 | 2 | 3 | | | III-1 | Fam92b | 339145 | 4-May-15 |
| 7215 | 2 | 3 | | | III-1 | Fan1 | 22909 | 7-Jun-15 |
| 7216 | 2 | 3 | | | III-1 | Fanca | 2175 | 23-May-15 |
| 7223 | 2 | 3 | | | III-1 | Fancg | 2189 | 23-May-15 |
| 7229 | 2 | 3 | | | III-1 | Far1 | 84188 | 4-May-15 |
| 7231 | 2 | 3 | | | III-1 | Farp1 | 10160 | 4-May-15 |
| 7232 | 2 | 3 | | | III-1 | Farp2 | 9855 | 12-May-15 |
| 7234 | 2 | 3 | | | III-1 | Farsa | 2193 | 4-May-15 |
| 7235 | 2 | 3 | | | III-1 | Farsb | 10056 | 4-May-15 |
| 7244 | 2 | 3 | | | III-1 | Fat1 | 2195 | 12-May-15 |
| 7245 | 2 | 3 | | | III-1 | Fat2 | 2196 | 12-May-15 |
| 7246 | 2 | 3 | | | III-1 | Fat3 | 120114 | 4-May-15 |
| 7248 | 2 | 3 | | | III-1 | Fate1 | 89885 | 4-May-15 |
| 7250 | 2 | 3 | | | III-1 | Faxc | 84553 | 4-May-15 |
| 7254 | 2 | 3 | | | III-1 | Fbll1 | 345630 | 3-May-15 |
| 7256 | 2 | 3 | | | III-1 | Fbln2 | 2199 | 12-May-15 |
| 7265 | 2 | 3 | | | III-1 | Fbxl12 | 54850 | 20-May-15 |
| 7267 | 2 | 3 | | | III-1 | Fbxl13 | 222235 | 4-May-15 |
| 7274 | 2 | 3 | | | III-1 | Fbxl2 | 25827 | 4-May-15 |
| 7278 | 2 | 3 | | | III-1 | Fbxl3 | 26224 | 4-May-15 |
| 7282 | 2 | 3 | | | III-1 | Fbxl7 | 23194 | 4-May-15 |
| 7285 | 2 | 3 | | | III-1 | Fbxo11 | 80204 | 2-Jun-15 |
| 7288 | 2 | 3 | | | III-1 | Fbxo17 | 115290 | 4-May-15 |
| 7289 | 2 | 3 | | | III-1 | Fbxo18 | 84893 | 4-May-15 |
| 7291 | 2 | 3 | | | III-1 | Fbxo21 | 23014 | 4-May-15 |
| 7292 | 2 | 3 | | | III-1 | Fbxo22 | 26263 | 4-May-15 |
| 7301 | 2 | 3 | | | III-1 | Fbxo33 | 254170 | 4-May-15 |
| 7304 | 2 | 3 | | | III-1 | Fbxo38 | 81545 | 4-May-15 |
| 7312 | 2 | 3 | | | III-1 | Fbxo45 | 200933 | 4-May-15 |
| 7318 | 2 | 3 | | | III-1 | Fbxo7 | 25793 | 23-May-15 |
| 7340 | 2 | 3 | | | III-1 | Fbxw8 | 26259 | 4-May-15 |
| 7342 | 2 | 3 | | | III-1 | Fcamr | 83953 | 4-May-15 |
| 7347 | 2 | 3 | | | III-1 | Fcgbp | 8857 | 4-May-15 |
| 7353 | 2 | 3 | | | III-1 | Fcho1 | 23149 | 4-May-15 |
| 7356 | 2 | 3 | | | III-1 | Fchsd2 | 9873 | 4-May-15 |
| 7369 | 2 | 3 | | | III-1 | Fdxacb1 | 91893 | 4-May-15 |

Fig.22 - 67

| | | | | | | | | |
|---|---|---|---|---|---|---|---|---|
| 7373 | 2 | 3 | | | III-1 | Fem1b | 10116 | 4-May-15 |
| 7375 | 2 | 3 | | | III-1 | Fen1 | 2237 | 3-May-15 |
| 7376 | 2 | 3 | | | III-1 | Fendrr | 400550 | 16-May-15 |
| 7381 | 2 | 3 | | | III-1 | Fermt2 | 10979 | 4-May-15 |
| 7382 | 2 | 3 | | | III-1 | Fermt3 | 83706 | 24-May-15 |
| 7386 | 2 | 3 | | | III-1 | Fev | 54738 | 12-May-15 |
| 7387 | 2 | 3 | | | III-1 | Fez1 | 9638 | 7-Jun-15 |
| 7388 | 2 | 3 | | | III-1 | Fez2 | 9637 | 21-May-15 |
| 7389 | 2 | 3 | | | III-1 | Fezf1 | 389549 | 4-May-15 |
| 7392 | 2 | 3 | | | III-1 | Ffar2 | 2867 | 7-Jun-15 |
| 7398 | 2 | 3 | | | III-1 | Fgd2 | 221472 | 7-Jun-15 |
| 7399 | 2 | 3 | | | III-1 | Fgd3 | 89846 | 7-Jun-15 |
| 7401 | 2 | 3 | | | III-1 | Fgd5 | 152273 | 12-May-15 |
| 7402 | 2 | 3 | | | III-1 | Fgd6 | 55785 | 4-May-15 |
| 7405 | 2 | 3 | | | III-1 | Fgf11 | 2256 | 4-May-15 |
| 7408 | 2 | 3 | | | III-1 | Fgf14 | 2259 | 23-May-15 |
| 7416 | 2 | 3 | | | III-1 | Fgf22 | 27006 | 4-May-15 |
| 7418 | 2 | 3 | | | III-1 | Fgf3 | 2248 | 23-May-15 |
| 7422 | 2 | 3 | | | III-1 | Fgf7 | 2252 | 12-May-15 |
| 7428 | 2 | 3 | | | III-1 | Fgfr1op | 11116 | 4-May-15 |
| 7431 | 2 | 3 | | | III-1 | Fgfr3 | 2261 | 23-May-15 |
| 7437 | 2 | 3 | | | III-1 | Fgl2 | 10875 | 12-May-15 |
| 7438 | 2 | 3 | | | III-1 | Fgr | 2268 | 12-May-15 |
| 7448 | 2 | 3 | | | III-1 | Fhl5 | 9457 | 7-Jun-15 |
| 7451 | 2 | 3 | | | III-1 | Fibcd1 | 84929 | 4-May-15 |
| 7453 | 2 | 3 | | | III-1 | Fibp | 9158 | 2-Jun-15 |
| 7454 | 2 | 3 | | | III-1 | Ficd | 11153 | 4-May-15 |
| 7457 | 2 | 3 | | | III-1 | Figla | 344018 | 4-May-15 |
| 7463 | 2 | 3 | | | III-1 | Fip1l1 | 81608 | 3-May-15 |
| 7465 | 2 | 3 | | | III-1 | Fis1 | 51024 | 4-May-15 |
| 7468 | 2 | 3 | | | III-1 | Fiz1 | 84922 | 4-May-15 |
| 7470 | 2 | 3 | | | III-1 | Fkbp10 | 60681 | 4-May-15 |
| 7476 | 2 | 3 | | | III-1 | Fkbp2 | 2286 | 4-May-15 |
| 7478 | 2 | 3 | | | III-1 | Fkbp4 | 2288 | 7-Jun-15 |
| 7480 | 2 | 3 | | | III-1 | Fkbp6 | 8468 | 4-May-15 |
| 7484 | 2 | 3 | | | III-1 | Fkbpl | 63943 | 25-May-15 |
| 7487 | 2 | 3 | | | III-1 | Flad1 | 80308 | 4-May-15 |
| 7490 | 2 | 3 | | | III-1 | Fli1 | 2313 | 7-Jun-15 |
| 7491 | 2 | 3 | | | III-1 | Flii | 2314 | 3-Jun-15 |
| 7496 | 2 | 3 | | | III-1 | Flot2 | 2319 | 3-May-15 |
| 7499 | 2 | 3 | | | III-1 | Flrt3 | 23767 | 4-May-15 |
| 7501 | 2 | 3 | | | III-1 | Flt3 | 2322 | 31-May-15 |
| 7502 | 2 | 3 | | | III-1 | Flt3l | 2323 | 12-May-15 |
| 7503 | 2 | 3 | | | III-1 | Flt4 | 2324 | 23-May-15 |
| 7504 | 2 | 3 | | | III-1 | Flywch1 | 84256 | 12-May-15 |
| 7515 | 2 | 3 | | | III-1 | Fmo5 | 2330 | 4-May-15 |
| 7516 | 2 | 3 | | | III-1 | Fmo6 | 388714 | 4-May-15 |
| 7524 | 2 | 3 | | | III-1 | Fnbp1 | 23048 | 4-May-15 |
| 7532 | 2 | 3 | | | III-1 | Fndc4 | 64838 | 4-May-15 |
| 7535 | 2 | 3 | | | III-1 | Fndc8 | 54752 | 4-May-15 |
| 7546 | 2 | 3 | | | III-1 | Fopnl | 123811 | 12-May-15 |
| 7551 | 2 | 3 | | | III-1 | Foxa1 | 3169 | 17-May-15 |
| 7557 | 2 | 3 | | | III-1 | Foxc2 | 2303 | 23-May-15 |
| 7558 | 2 | 3 | | | III-1 | Foxd1 | 2297 | 28-May-15 |
| 7562 | 2 | 3 | | | III-1 | Foxd4 | 2298 | 28-May-15 |
| 7568 | 2 | 3 | | | III-1 | Foxh1 | 8928 | 23-May-15 |
| 7570 | 2 | 3 | | | III-1 | Foxi2 | 399823 | 28-May-15 |
| 7573 | 2 | 3 | | | III-1 | Foxj2 | 55810 | 28-May-15 |
| 7580 | 2 | 3 | | | III-1 | Foxm1 | 2305 | 17-May-15 |
| 7588 | 2 | 3 | | | III-1 | Foxo6 | 100132074 | 28-May-15 |
| 7592 | 2 | 3 | | | III-1 | Foxp4 | 116113 | 4-May-15 |
| 7598 | 2 | 3 | | | III-1 | Foxs1 | 2307 | 4-May-15 |
| 7600 | 2 | 3 | | | III-1 | Fpgt | 8790 | 4-May-15 |
| 7603 | 2 | 3 | | | III-1 | Fpr3 | 2359 | 4-May-15 |
| 7608 | 2 | 3 | | | III-1 | Fras1 | 80144 | 12-May-15 |
| 7614 | 2 | 3 | | | III-1 | Frg1 | 2483 | 23-May-15 |
| 7616 | 2 | 3 | | | III-1 | Frmd3 | 257019 | 4-May-15 |
| 7618 | 2 | 3 | | | III-1 | Frmd4b | 23150 | 12-May-15 |
| 7620 | 2 | 3 | | | III-1 | Frmd6 | 122786 | 4-May-15 |
| 7625 | 2 | 3 | | | III-1 | Frmpd3 | 84443 | 4-May-15 |
| 7628 | 2 | 3 | | | III-1 | Frrs1l | 23732 | 4-May-15 |
| 7630 | 2 | 3 | | | III-1 | Frs3 | 10817 | 3-May-15 |
| 7631 | 2 | 3 | | | III-1 | Frs3os | | |
| 7649 | 2 | 3 | | | III-1 | Fstl4 | 23105 | 4-May-15 |
| 7655 | 2 | 3 | | | III-1 | Ftmt | 94033 | 28-May-15 |
| 7658 | 2 | 3 | | | III-1 | Ftsj2 | 29960 | 30-May-15 |
| 7663 | 2 | 3 | | | III-1 | Fuca1 | 2517 | 21-May-15 |
| 7665 | 2 | 3 | | | III-1 | Fuk | 197258 | 4-May-15 |
| 7668 | 2 | 3 | | | III-1 | Fuom | 282969 | 12-May-15 |
| 7669 | 2 | 3 | | | III-1 | Furin | 5045 | 31-May-15 |
| 7673 | 2 | 3 | | | III-1 | Fut11 | 170384 | 23-May-15 |
| 7676 | 2 | 3 | | | III-1 | Fut4-ps1 | | |
| 7681 | 2 | 3 | | | III-1 | Fv1 | | |
| 7683 | 2 | 3 | | | III-1 | Fxr1 | 8087 | 1-Jun-15 |
| 7684 | 2 | 3 | | | III-1 | Fxr2 | 9513 | 12-May-15 |
| 7689 | 2 | 3 | | | III-1 | Fxyd5 | 53827 | 4-May-15 |
| 7690 | 2 | 3 | | | III-1 | Fxyd6 | 53826 | 4-May-15 |
| 7691 | 2 | 3 | | | III-1 | Fxyd7 | 53822 | 12-May-15 |
| 7694 | 2 | 3 | | | III-1 | Fyn | 2534 | 12-May-15 |
| 7698 | 2 | 3 | | | III-1 | Fzd2 | 2535 | 4-May-15 |
| 7699 | 2 | 3 | | | III-1 | Fzd3 | 7976 | 7-Jun-15 |
| 7703 | 2 | 3 | | | III-1 | Fzd7 | 8324 | 4-May-15 |
| 7713 | 2 | 3 | | | III-1 | G630055G22Rik | | |
| 7723 | 2 | 3 | | | III-1 | G6pdx | | |
| 7724 | 2 | 3 | | | III-1 | G730013B05Rik | | |
| 7727 | 2 | 3 | | | III-1 | Gab2 | 9846 | 17-May-15 |
| 7728 | 2 | 3 | | | III-1 | Gab3 | 139716 | 12-May-15 |

| | | | | | | | | |
|---|---|---|---|---|---|---|---|---|
| 7734 | 2 | 3 | | | III-1 | Gabpa | 2551 | 12-May-15 |
| 7736 | 2 | 3 | | | III-1 | Gabpb2 | 126626 | 7-Jun-15 |
| 7737 | 2 | 3 | | | III-1 | Gabra1 | 2554 | 3-May-15 |
| 7748 | 2 | 3 | | | III-1 | Gabrg1 | 2565 | 4-May-15 |
| 7753 | 2 | 3 | | | III-1 | Gabrr1 | 2569 | 4-May-15 |
| 7757 | 2 | 3 | | | III-1 | Gad1os | | |
| 7762 | 2 | 3 | | | III-1 | Gadd45gip1 | 90480 | 4-May-15 |
| 7763 | 2 | 3 | | | III-1 | Gadl1 | 339896 | 4-May-15 |
| 7767 | 2 | 3 | | | III-1 | Gal3st2 | 64090 | 7-Jun-15 |
| 7768 | 2 | 3 | | | III-1 | Gal3st3 | 89792 | 4-May-15 |
| 7770 | 2 | 3 | | | III-1 | Galc | 2581 | 23-May-15 |
| 7771 | 2 | 3 | | | III-1 | Gale | 2582 | 23-May-15 |
| 7773 | 2 | 3 | | | III-1 | Galk2 | 2585 | 4-May-15 |
| 7776 | 2 | 3 | | | III-1 | Galnt1 | 2589 | 4-May-15 |
| 7780 | 2 | 3 | | | III-1 | Galnt13 | 114805 | 7-Jun-15 |
| 7783 | 2 | 3 | | | III-1 | Galnt16 | 57452 | 4-May-15 |
| 7785 | 2 | 3 | | | III-1 | Galnt2 | 2590 | 12-May-15 |
| 7787 | 2 | 3 | | | III-1 | Galnt4 | 8693 | 4-May-15 |
| 7788 | 2 | 3 | | | III-1 | Galnt5 | 11227 | 4-May-15 |
| 7790 | 2 | 3 | | | III-1 | Galnt7 | 51809 | 7-Jun-15 |
| 7791 | 2 | 3 | | | III-1 | Galnt9 | 50614 | 4-May-15 |
| 7792 | 2 | 3 | | | III-1 | Galntl5 | 168391 | 23-May-15 |
| 7797 | 2 | 3 | | | III-1 | Galr3 | 8484 | 12-May-15 |
| 7806 | 2 | 3 | | | III-1 | Gapt | 202309 | 12-May-15 |
| 7810 | 2 | 3 | | | III-1 | Gareml | 150946 | 4-May-15 |
| 7815 | 2 | 3 | | | III-1 | Gas2 | 2620 | 4-May-15 |
| 7817 | 2 | 3 | | | III-1 | Gas2l2 | 246176 | 4-May-15 |
| 7821 | 2 | 3 | | | III-1 | Gas7 | 8522 | 4-May-15 |
| 7822 | 2 | 3 | | | III-1 | Gas8 | 2622 | 12-May-15 |
| 7823 | 2 | 3 | | | III-1 | Gast | 2520 | 12-May-15 |
| 7827 | 2 | 3 | | | III-1 | Gata4 | 2626 | 31-May-15 |
| 7828 | 2 | 3 | | | III-1 | Gata5 | 140628 | 4-May-15 |
| 7829 | 2 | 3 | | | III-1 | Gata5os | | |
| 7831 | 2 | 3 | | | III-1 | Gatad1 | 57798 | 12-May-15 |
| 7841 | 2 | 3 | | | III-1 | Gbe1 | 2632 | 23-May-15 |
| 7842 | 2 | 3 | | | III-1 | Gbf1 | 8729 | 16-Jun-15 |
| 7847 | 2 | 3 | | | III-1 | Gbp2b | | |
| 7853 | 2 | 3 | | | III-1 | Gbp8 | | |
| 7860 | 2 | 3 | | | III-1 | Gcc1 | 79571 | 4-May-15 |
| 7861 | 2 | 3 | | | III-1 | Gcc2 | 9648 | 4-May-15 |
| 7865 | 2 | 3 | | | III-1 | Gcgr | 2642 | 4-May-15 |
| 7866 | 2 | 3 | | | III-1 | Gch1 | 2643 | 23-May-15 |
| 7870 | 2 | 3 | | | III-1 | Gclc | 2729 | 12-May-15 |
| 7872 | 2 | 3 | | | III-1 | Gcm1 | 8521 | 4-May-15 |
| 7874 | 2 | 3 | | | III-1 | Gcn1l1 | 10985 | 12-May-15 |
| 7876 | 2 | 3 | | | III-1 | Gcnt2 | 2651 | 23-May-15 |
| 7877 | 2 | 3 | | | III-1 | Gcnt3 | 9245 | 24-May-15 |
| 7879 | 2 | 3 | | | III-1 | Gcnt7 | 140687 | 4-May-15 |
| 7883 | 2 | 3 | | | III-1 | Gdap1 | 54332 | 23-May-15 |
| 7886 | 2 | 3 | | | III-1 | Gdap2 | 54834 | 12-May-15 |
| 7888 | 2 | 3 | | | III-1 | Gdf1 | 2657 | 4-May-15 |
| 7895 | 2 | 3 | | | III-1 | Gdf6 | 392255 | 12-May-15 |
| 7897 | 2 | 3 | | | III-1 | Gdf9 | 2661 | 4-May-15 |
| 7903 | 2 | 3 | | | III-1 | Gdpd3 | 79153 | 21-May-15 |
| 7908 | 2 | 3 | | | III-1 | Gemin2 | 8487 | 23-May-15 |
| 7909 | 2 | 3 | | | III-1 | Gemin4 | 50628 | 4-May-15 |
| 7912 | 2 | 3 | | | III-1 | Gemin7 | 79760 | 4-May-15 |
| 7914 | 2 | 3 | | | III-1 | Gen1 | 348654 | 4-May-15 |
| 7917 | 2 | 3 | | | III-1 | Gfer | 2671 | 12-May-15 |
| 7923 | 2 | 3 | | | III-1 | Gfod2 | 81577 | 4-May-15 |
| 7927 | 2 | 3 | | | III-1 | Gfra2 | 2675 | 12-May-15 |
| 7930 | 2 | 3 | | | III-1 | Gfral | 389400 | 4-May-15 |
| 7934 | 2 | 3 | | | III-1 | Gga3 | 23163 | 12-May-15 |
| 7939 | 2 | 3 | | | III-1 | Ggn | 199720 | 4-May-15 |
| 7941 | 2 | 3 | | | III-1 | Ggnbp2 | 79893 | 4-May-15 |
| 7945 | 2 | 3 | | | III-1 | Ggt6 | 124975 | 4-May-15 |
| 7947 | 2 | 3 | | | III-1 | Ggta1 | 2681 | 4-May-15 |
| 7952 | 2 | 3 | | | III-1 | Ghrh | 2691 | 12-May-15 |
| 7960 | 2 | 3 | | | III-1 | Gigyf2 | 26058 | 23-May-15 |
| 7965 | 2 | 3 | | | III-1 | Gimap6 | 474344 | 4-May-15 |
| 7968 | 2 | 3 | | | III-1 | Gimap9 | | |
| 7969 | 2 | 3 | | | III-1 | Gin1 | 54826 | 4-May-15 |
| 7970 | 2 | 3 | | | III-1 | Ginm1 | 116254 | 4-May-15 |
| 7974 | 2 | 3 | | | III-1 | Gins4 | 84296 | 4-May-15 |
| 7979 | 2 | 3 | | | III-1 | Gipr | 2696 | 9-May-15 |
| 7983 | 2 | 3 | | | III-1 | Gja10 | 84694 | 4-May-15 |
| 7986 | 2 | 3 | | | III-1 | Gja5 | 2702 | 31-May-15 |
| 7987 | 2 | 3 | | | III-1 | Gja6 | | |
| 7994 | 2 | 3 | | | III-1 | Gjb6 | 10804 | 23-May-15 |
| 7995 | 2 | 3 | | | III-1 | Gjc1 | 10052 | 7-Jun-15 |
| 7996 | 2 | 3 | | | III-1 | Gjc2 | 57165 | 12-May-15 |
| 8005 | 2 | 3 | | | III-1 | Gkn1 | 56287 | 4-May-15 |
| 8010 | 2 | 3 | | | III-1 | Glb1l | 79411 | 23-May-15 |
| 8013 | 2 | 3 | | | III-1 | Glcci1 | 113263 | 12-May-15 |
| 8016 | 2 | 3 | | | III-1 | Gldn | 342035 | 4-May-15 |
| 8031 | 2 | 3 | | | III-1 | Glo1 | 2739 | 17-May-15 |
| 8035 | 2 | 3 | | | III-1 | Glp2r | 9340 | 4-May-15 |
| 8042 | 2 | 3 | | | III-1 | Glrx | 2745 | 12-May-15 |
| 8043 | 2 | 3 | | | III-1 | Glrx2 | 51022 | 4-May-15 |
| 8046 | 2 | 3 | | | III-1 | Gls | 2744 | 13-Jun-15 |
| 8049 | 2 | 3 | | | III-1 | Glt25d1 | 79709 | 4-May-15 |
| 8051 | 2 | 3 | | | III-1 | Glt6d1 | 360203 | 4-May-15 |
| 8053 | 2 | 3 | | | III-1 | Glt8d2 | 83468 | 23-May-15 |
| 8057 | 2 | 3 | | | III-1 | Gltscr1 | 29998 | 21-May-15 |
| 8058 | 2 | 3 | | | III-1 | Gltscr1l | 23506 | 12-May-15 |
| 8059 | 2 | 3 | | | III-1 | Gltscr2 | 29997 | 4-May-15 |
| 8062 | 2 | 3 | | | III-1 | Glyat | 10249 | 4-May-15 |

Fig.22 - 68

| | | | | | | | | |
|---|---|---|---|---|---|---|---|---|
| 8065 | 2 | 3 | | | III-1 | Glyctk | 132158 | 4-May-15 |
| 8069 | 2 | 3 | | | III-1 | Gm10024 | | |
| 8071 | 2 | 3 | | | III-1 | Gm10046 | | |
| 8075 | 2 | 3 | | | III-1 | Gm10069 | | |
| 8078 | 2 | 3 | | | III-1 | Gm10096 | | |
| 8079 | 2 | 3 | | | III-1 | Gm101 | | |
| 8110 | 2 | 3 | | | III-1 | Gm10413 | | |
| 8118 | 2 | 3 | | | III-1 | Gm10440 | | |
| 8122 | 2 | 3 | | | III-1 | Gm10466 | | |
| 8130 | 2 | 3 | | | III-1 | Gm10510 | | |
| 8140 | 2 | 3 | | | III-1 | Gm10578 | | |
| 8149 | 2 | 3 | | | III-1 | Gm10640 | | |
| 8152 | 2 | 3 | | | III-1 | Gm10658 | | |
| 8153 | 2 | 3 | | | III-1 | Gm10662 | | |
| 8166 | 2 | 3 | | | III-1 | Gm10768 | | |
| 8167 | 2 | 3 | | | III-1 | Gm10778 | | |
| 8173 | 2 | 3 | | | III-1 | Gm10791 | | |
| 8177 | 2 | 3 | | | III-1 | Gm10825 | | |
| 8187 | 2 | 3 | | | III-1 | Gm11110 | | |
| 8192 | 2 | 3 | | | III-1 | Gm11186 | | |
| 8199 | 2 | 3 | | | III-1 | Gm11346 | | |
| 8200 | 2 | 3 | | | III-1 | Gm11351 | | |
| 8201 | 2 | 3 | | | III-1 | Gm1140 | | |
| 8206 | 2 | 3 | | | III-1 | Gm11468 | | |
| 8209 | 2 | 3 | | | III-1 | Gm11529 | | |
| 8221 | 2 | 3 | | | III-1 | Gm11565 | | |
| 8224 | 2 | 3 | | | III-1 | Gm11569 | | |
| 8225 | 2 | 3 | | | III-1 | Gm11570 | | |
| 8228 | 2 | 3 | | | III-1 | Gm11627 | | |
| 8229 | 2 | 3 | | | III-1 | Gm11651 | | |
| 8230 | 2 | 3 | | | III-1 | Gm11696 | | |
| 8233 | 2 | 3 | | | III-1 | Gm11747 | | |
| 8237 | 2 | 3 | | | III-1 | Gm11780 | | |
| 8244 | 2 | 3 | | | III-1 | Gm11978 | | |
| 8250 | 2 | 3 | | | III-1 | Gm12070 | | |
| 8251 | 2 | 3 | | | III-1 | Gm12130 | | |
| 8260 | 2 | 3 | | | III-1 | Gm12253 | | |
| 8265 | 2 | 3 | | | III-1 | Gm12409 | | |
| 8266 | 2 | 3 | | | III-1 | Gm12429 | | |
| 8287 | 2 | 3 | | | III-1 | Gm13003 | | |
| 8293 | 2 | 3 | | | III-1 | Gm13040 | | |
| 8305 | 2 | 3 | | | III-1 | Gm13125 | | |
| 8313 | 2 | 3 | | | III-1 | Gm13212 | | |
| 8319 | 2 | 3 | | | III-1 | Gm13271 | | |
| 8340 | 2 | 3 | | | III-1 | Gm13446 | | |
| 8345 | 2 | 3 | | | III-1 | Gm13539 | | |
| 8348 | 2 | 3 | | | III-1 | Gm13547 | | |
| 8358 | 2 | 3 | | | III-1 | Gm13826 | | |
| 8359 | 2 | 3 | | | III-1 | Gm13871 | | |
| 8371 | 2 | 3 | | | III-1 | Gm14139 | | |
| 8379 | 2 | 3 | | | III-1 | Gm14305 | | |
| 8384 | 2 | 3 | | | III-1 | Gm14326 | | |
| 8385 | 2 | 3 | | | III-1 | Gm14327 | | |
| 8386 | 2 | 3 | | | III-1 | Gm14345 | | |
| 8392 | 2 | 3 | | | III-1 | Gm14379 | | |
| 8394 | 2 | 3 | | | III-1 | Gm14393 | | |
| 8395 | 2 | 3 | | | III-1 | Gm14403 | | |
| 8396 | 2 | 3 | | | III-1 | Gm14405 | | |
| 8398 | 2 | 3 | | | III-1 | Gm14431 | | |
| 8408 | 2 | 3 | | | III-1 | Gm14477 | | |
| 8416 | 2 | 3 | | | III-1 | Gm14501 | | |
| 8427 | 2 | 3 | | | III-1 | Gm14725 | | |
| 8436 | 2 | 3 | | | III-1 | Gm14858 | | |
| 8444 | 2 | 3 | | | III-1 | Gm15097 | | |
| 8450 | 2 | 3 | | | III-1 | Gm15140 | | |
| 8452 | 2 | 3 | | | III-1 | Gm15217 | | |
| 8465 | 2 | 3 | | | III-1 | Gm15401 | | |
| 8466 | 2 | 3 | | | III-1 | Gm15408 | | |
| 8470 | 2 | 3 | | | III-1 | Gm15421 | | |
| 8473 | 2 | 3 | | | III-1 | Gm15455 | | |
| 8474 | 2 | 3 | | | III-1 | Gm15471 | | |
| 8477 | 2 | 3 | | | III-1 | Gm156 | | |
| 8479 | 2 | 3 | | | III-1 | Gm1564 | | |
| 8485 | 2 | 3 | | | III-1 | Gm15708 | | |
| 8486 | 2 | 3 | | | III-1 | Gm15713 | | |
| 8488 | 2 | 3 | | | III-1 | Gm15772 | | |
| 8489 | 2 | 3 | | | III-1 | Gm15787 | | |
| 8490 | 2 | 3 | | | III-1 | Gm15800 | | |
| 8493 | 2 | 3 | | | III-1 | Gm1587 | | |
| 8502 | 2 | 3 | | | III-1 | Gm16039 | | |
| 8504 | 2 | 3 | | | III-1 | Gm16062 | | |
| 8505 | 2 | 3 | | | III-1 | Gm16063 | | |
| 8516 | 2 | 3 | | | III-1 | Gm16386 | | |
| 8521 | 2 | 3 | | | III-1 | Gm16432 | | |
| 8534 | 2 | 3 | | | III-1 | Gm16596 | | |
| 8535 | 2 | 3 | | | III-1 | Gm166 | | |
| 8538 | 2 | 3 | | | III-1 | Gm16675 | | |
| 8539 | 2 | 3 | | | III-1 | Gm16677 | | |
| 8546 | 2 | 3 | | | III-1 | Gm16796 | | |
| 8549 | 2 | 3 | | | III-1 | Gm16853 | | |
| 8551 | 2 | 3 | | | III-1 | Gm16863 | | |
| 8556 | 2 | 3 | | | III-1 | Gm16938 | | |
| 8558 | 2 | 3 | | | III-1 | Gm16982 | | |
| 8560 | 2 | 3 | | | III-1 | Gm17019 | | |
| 8562 | 2 | 3 | | | III-1 | Gm1715 | | |
| 8565 | 2 | 3 | | | III-1 | Gm17296 | | |
| 8568 | 2 | 3 | | | III-1 | Gm17455 | | |
| 8572 | 2 | 3 | | | III-1 | Gm17689 | | |
| 8574 | 2 | 3 | | | III-1 | Gm17745 | | |
| 8589 | 2 | 3 | | | III-1 | Gm19303 | | |
| 8600 | 2 | 3 | | | III-1 | Gm19557 | | |
| 8601 | 2 | 3 | | | III-1 | Gm19583 | | |
| 8604 | 2 | 3 | | | III-1 | Gm1965 | | |
| 8605 | 2 | 3 | | | III-1 | Gm1966 | | |
| 8610 | 2 | 3 | | | III-1 | Gm19710 | | |
| 8611 | 2 | 3 | | | III-1 | Gm19757 | | |
| 8613 | 2 | 3 | | | III-1 | Gm19782 | | |
| 8620 | 2 | 3 | | | III-1 | Gm19990 | | |
| 8637 | 2 | 3 | | | III-1 | Gm2030 | | |
| 8641 | 2 | 3 | | | III-1 | Gm20337 | | |
| 8646 | 2 | 3 | | | III-1 | Gm20556 | | |
| 8652 | 2 | 3 | | | III-1 | Gm20611 | | |
| 8659 | 2 | 3 | | | III-1 | Gm20744 | | |
| 8663 | 2 | 3 | | | III-1 | Gm20750 | | |
| 8668 | 2 | 3 | | | III-1 | Gm20755 | | |
| 8678 | 2 | 3 | | | III-1 | Gm20816 | | |
| 8686 | 2 | 3 | | | III-1 | Gm20858 | | |
| 8691 | 2 | 3 | | | III-1 | Gm20877 | | |
| 8693 | 2 | 3 | | | III-1 | Gm20917 | | |
| 8697 | 2 | 3 | | | III-1 | Gm2109 | | |
| 8715 | 2 | 3 | | | III-1 | Gm21708 | | |
| 8716 | 2 | 3 | | | III-1 | Gm2176 | | |
| 8723 | 2 | 3 | | | III-1 | Gm2381 | | |
| 8725 | 2 | 3 | | | III-1 | Gm2447 | | |
| 8730 | 2 | 3 | | | III-1 | Gm2694 | | |
| 8750 | 2 | 3 | | | III-1 | Gm3230 | | |
| 8751 | 2 | 3 | | | III-1 | Gm3238 | | |
| 8759 | 2 | 3 | | | III-1 | Gm3336 | | |
| 8760 | 2 | 3 | | | III-1 | Gm3383 | | |
| 8761 | 2 | 3 | | | III-1 | Gm3402 | | |
| 8769 | 2 | 3 | | | III-1 | Gm3435 | | |
| 8770 | 2 | 3 | | | III-1 | Gm3458 | | |
| 8772 | 2 | 3 | | | III-1 | Gm3500 | | |
| 8779 | 2 | 3 | | | III-1 | Gm3701 | | |
| 8785 | 2 | 3 | | | III-1 | Gm382 | | |
| 8790 | 2 | 3 | | | III-1 | Gm4070 | | |
| 8800 | 2 | 3 | | | III-1 | Gm4265 | | |
| 8815 | 2 | 3 | | | III-1 | Gm44 | | |
| 8821 | 2 | 3 | | | III-1 | Gm4541 | | |
| 8823 | 2 | 3 | | | III-1 | Gm4566 | | |
| 8830 | 2 | 3 | | | III-1 | Gm4745 | | |
| 8836 | 2 | 3 | | | III-1 | Gm4791 | | |
| 8840 | 2 | 3 | | | III-1 | Gm4827 | | |
| 8843 | 2 | 3 | | | III-1 | Gm4841 | | |
| 8845 | 2 | 3 | | | III-1 | Gm4847 | | |
| 8849 | 2 | 3 | | | III-1 | Gm4871 | | |
| 8859 | 2 | 3 | | | III-1 | Gm4937 | | |
| 8883 | 2 | 3 | | | III-1 | Gm5091 | | |
| 8888 | 2 | 3 | | | III-1 | Gm5113 | | |
| 8889 | 2 | 3 | | | III-1 | Gm5114 | | |
| 8893 | 2 | 3 | | | III-1 | Gm5127 | | |
| 8894 | 2 | 3 | | | III-1 | Gm5129 | | |
| 8896 | 2 | 3 | | | III-1 | Gm5134 | | |
| 8899 | 2 | 3 | | | III-1 | Gm5142 | | |
| 8902 | 2 | 3 | | | III-1 | Gm5166 | | |
| 8916 | 2 | 3 | | | III-1 | Gm5414 | | |
| 8917 | 2 | 3 | | | III-1 | Gm5415 | | |
| 8923 | 2 | 3 | | | III-1 | Gm5441 | | |
| 8934 | 2 | 3 | | | III-1 | Gm5512 | | |
| 8937 | 2 | 3 | | | III-1 | Gm5535 | | |
| 8939 | 2 | 3 | | | III-1 | Gm5544 | | |
| 8948 | 2 | 3 | | | III-1 | Gm5615 | | |
| 8950 | 2 | 3 | | | III-1 | Gm5622 | | |
| 8953 | 2 | 3 | | | III-1 | Gm5635 | | |
| 8956 | 2 | 3 | | | III-1 | Gm5662 | | |
| 8959 | 2 | 3 | | | III-1 | Gm5725 | | |
| 8963 | 2 | 3 | | | III-1 | Gm5766 | | |
| 8968 | 2 | 3 | | | III-1 | Gm5797 | | |
| 8972 | 2 | 3 | | | III-1 | Gm5820 | | |
| 8976 | 2 | 3 | | | III-1 | Gm5868 | | |
| 8985 | 2 | 3 | | | III-1 | Gm5925 | | |
| 8989 | 2 | 3 | | | III-1 | Gm5938 | | |
| 8996 | 2 | 3 | | | III-1 | Gm6042 | | |
| 9001 | 2 | 3 | | | III-1 | Gm6116 | | |
| 9004 | 2 | 3 | | | III-1 | Gm6150 | | |
| 9011 | 2 | 3 | | | III-1 | Gm6260 | | |
| 9013 | 2 | 3 | | | III-1 | Gm6277 | | |
| 9015 | 2 | 3 | | | III-1 | Gm6297 | | |
| 9020 | 2 | 3 | | | III-1 | Gm6367 | | |
| 9023 | 2 | 3 | | | III-1 | Gm6402 | | |
| 9032 | 2 | 3 | | | III-1 | Gm6498 | | |
| 9040 | 2 | 3 | | | III-1 | Gm6578 | | |
| 9047 | 2 | 3 | | | III-1 | Gm6623 | | |
| 9053 | 2 | 3 | | | III-1 | Gm6682 | | |
| 9055 | 2 | 3 | | | III-1 | Gm6710 | | |
| 9056 | 2 | 3 | | | III-1 | Gm6756 | | |
| 9061 | 2 | 3 | | | III-1 | Gm6793 | | |
| 9065 | 2 | 3 | | | III-1 | Gm6878 | | |
| 9071 | 2 | 3 | | | III-1 | Gm6936 | | |
| 9074 | 2 | 3 | | | III-1 | Gm6981 | | |
| 9078 | 2 | 3 | | | III-1 | Gm7056 | | |
| 9084 | 2 | 3 | | | III-1 | Gm7134 | | |
| 9086 | 2 | 3 | | | III-1 | Gm7157 | | |
| 9098 | 2 | 3 | | | III-1 | Gm7444 | | |

Fig.22 - 69

| | | | | | | | | |
|---|---|---|---|---|---|---|---|---|
| 9108 | 2 | 3 | | | III-1 | Gm7714 | | |
| 9110 | 2 | 3 | | | III-1 | Gm7788 | | |
| 9128 | 2 | 3 | | | III-1 | Gm829 | | |
| 9149 | 2 | 3 | | | III-1 | Gm8817 | | |
| 9152 | 2 | 3 | | | III-1 | Gm8883 | | |
| 9159 | 2 | 3 | | | III-1 | Gm9 | | |
| 9161 | 2 | 3 | | | III-1 | Gm9047 | | |
| 9173 | 2 | 3 | | | III-1 | Gm9513 | | |
| 9193 | 2 | 3 | | | III-1 | Gm996 | | |
| 9195 | 2 | 3 | | | III-1 | Gm9962 | | |
| 9198 | 2 | 3 | | | III-1 | Gm9999 | | |
| 9202 | 2 | 3 | | | III-1 | Gmeb1 | 10691 | 4-May-15 |
| 9208 | 2 | 3 | | | III-1 | Gmnc | 647309 | 4-May-15 |
| 9209 | 2 | 3 | | | III-1 | Gmnn | 51053 | 24-May-15 |
| 9213 | 2 | 3 | | | III-1 | Gmpr2 | 51292 | 4-May-15 |
| 9217 | 2 | 3 | | | III-1 | Gna13 | 10672 | 17-May-15 |
| 9221 | 2 | 3 | | | III-1 | Gnai2 | 2771 | 12-May-15 |
| 9224 | 2 | 3 | | | III-1 | Gnao1 | 2775 | 12-May-15 |
| 9227 | 2 | 3 | | | III-1 | Gnat1 | 2779 | 21-May-15 |
| 9230 | 2 | 3 | | | III-1 | Gnaz | 2781 | 4-May-15 |
| 9236 | 2 | 3 | | | III-1 | Gnb4 | 59345 | 4-May-15 |
| 9238 | 2 | 3 | | | III-1 | Gne | 10020 | 23-May-15 |
| 9239 | 2 | 3 | | | III-1 | Gng10 | 2790 | 4-May-15 |
| 9244 | 2 | 3 | | | III-1 | Gng3 | 2785 | 4-May-15 |
| 9246 | 2 | 3 | | | III-1 | Gng5 | 2787 | 12-May-15 |
| 9247 | 2 | 3 | | | III-1 | Gng7 | 2788 | 4-May-15 |
| 9249 | 2 | 3 | | | III-1 | Gngt1 | 2792 | 3-May-15 |
| 9254 | 2 | 3 | | | III-1 | Gnl3l | 54552 | 4-May-15 |
| 9260 | 2 | 3 | | | III-1 | Gnptab | 79158 | 23-May-15 |
| 9261 | 2 | 3 | | | III-1 | Gnptg | 84572 | 23-May-15 |
| 9262 | 2 | 3 | | | III-1 | Gnrh1 | 2796 | 31-May-15 |
| 9268 | 2 | 3 | | | III-1 | Golga4 | 2803 | 4-May-15 |
| 9269 | 2 | 3 | | | III-1 | Golga5 | 9950 | 7-Jun-15 |
| 9273 | 2 | 3 | | | III-1 | Golim4 | 27333 | 12-May-15 |
| 9274 | 2 | 3 | | | III-1 | Golm1 | 51280 | 17-May-15 |
| 9275 | 2 | 3 | | | III-1 | Golph3 | 64083 | 12-May-15 |
| 9281 | 2 | 3 | | | III-1 | Gorab | 92344 | 4-May-15 |
| 9282 | 2 | 3 | | | III-1 | Gorasp1 | 64689 | 4-May-15 |
| 9289 | 2 | 3 | | | III-1 | Gp1ba | 2811 | 12-May-15 |
| 9293 | 2 | 3 | | | III-1 | Gp5 | 2814 | 4-May-15 |
| 9297 | 2 | 3 | | | III-1 | Gpaa1 | 8733 | 4-May-15 |
| 9308 | 2 | 3 | | | III-1 | Gpatch8 | 23131 | 4-May-15 |
| 9315 | 2 | 3 | | | III-1 | Gpc4 | 2239 | 23-May-15 |
| 9317 | 2 | 3 | | | III-1 | Gpc6 | 10082 | 4-May-15 |
| 9320 | 2 | 3 | | | III-1 | Gpd1l | 23171 | 23-May-15 |
| 9331 | 2 | 3 | | | III-1 | Gpm6b | 2824 | 12-May-15 |
| 9332 | 2 | 3 | | | III-1 | Gpn1 | 11321 | 4-May-15 |
| 9334 | 2 | 3 | | | III-1 | Gpn3 | 51184 | 4-May-15 |
| 9337 | 2 | 3 | | | III-1 | Gpr101 | 83550 | 4-May-15 |
| 9344 | 2 | 3 | | | III-1 | Gpr115 | 221393 | 12-May-15 |
| 9346 | 2 | 3 | | | III-1 | Gpr119 | 139760 | 4-May-15 |
| 9348 | 2 | 3 | | | III-1 | Gpr123 | 84435 | 12-May-15 |
| 9352 | 2 | 3 | | | III-1 | Gpr128 | 84873 | 21-May-15 |
| 9355 | 2 | 3 | | | III-1 | Gpr135 | 64582 | 21-May-15 |
| 9356 | 2 | 3 | | | III-1 | Gpr137 | 56834 | 12-May-15 |
| 9358 | 2 | 3 | | | III-1 | Gpr137b-ps | | |
| 9359 | 2 | 3 | | | III-1 | Gpr137c | 283554 | 4-May-15 |
| 9360 | 2 | 3 | | | III-1 | Gpr139 | 124274 | 12-May-15 |
| 9365 | 2 | 3 | | | III-1 | Gpr149 | 344758 | 4-May-15 |
| 9371 | 2 | 3 | | | III-1 | Gpr155 | 151556 | 4-May-15 |
| 9374 | 2 | 3 | | | III-1 | Gpr158 | 57512 | 31-May-15 |
| 9376 | 2 | 3 | | | III-1 | Gpr161 | 23432 | 12-May-15 |
| 9388 | 2 | 3 | | | III-1 | Gpr183 | 1880 | 4-May-15 |
| 9390 | 2 | 3 | | | III-1 | Gpr20 | 2843 | 21-May-15 |
| 9393 | 2 | 3 | | | III-1 | Gpr25 | 2848 | 4-May-15 |
| 9399 | 2 | 3 | | | III-1 | Gpr34 | 2857 | 4-May-15 |
| 9400 | 2 | 3 | | | III-1 | Gpr35 | 2859 | 4-May-15 |
| 9404 | 2 | 3 | | | III-1 | Gpr4 | 2828 | 4-May-15 |
| 9407 | 2 | 3 | | | III-1 | Gpr52 | 9293 | 4-May-15 |
| 9411 | 2 | 3 | | | III-1 | Gpr61 | 83873 | 4-May-15 |
| 9417 | 2 | 3 | | | III-1 | Gpr75 | 10936 | 4-May-15 |
| 9419 | 2 | 3 | | | III-1 | Gpr83 | 10888 | 12-May-15 |
| 9420 | 2 | 3 | | | III-1 | Gpr84 | 53831 | 4-May-15 |
| 9423 | 2 | 3 | | | III-1 | Gpr88 | 54112 | 4-May-15 |
| 9424 | 2 | 3 | | | III-1 | Gpr89 | 653519<br>51463 | 4-May-15<br>14-May-15 |
| 9426 | 2 | 3 | | | III-1 | Gpr98 | 84059 | 23-May-15 |
| 9430 | 2 | 3 | | | III-1 | Gprc5b | 51704 | 12-May-15 |
| 9435 | 2 | 3 | | | III-1 | Gprin2 | 9721 | 4-May-15 |
| 9439 | 2 | 3 | | | III-1 | Gpsm1 | 26086 | 4-May-15 |
| 9441 | 2 | 3 | | | III-1 | Gpsm3 | 63940 | 4-May-15 |
| 9444 | 2 | 3 | | | III-1 | Gpx1 | 2876 | 12-May-15 |
| 9448 | 2 | 3 | | | III-1 | Gpx4 | 2879 | 10-May-15 |
| 9455 | 2 | 3 | | | III-1 | Gramd1c | 54762 | 4-May-15 |
| 9456 | 2 | 3 | | | III-1 | Gramd2 | 196996 | 4-May-15 |
| 9457 | 2 | 3 | | | III-1 | Gramd3 | 65983 | 12-May-15 |
| 9459 | 2 | 3 | | | III-1 | Grap | 10750 | 4-May-15 |
| 9462 | 2 | 3 | | | III-1 | Grb10 | 2887 | 4-May-15 |
| 9463 | 2 | 3 | | | III-1 | Grb14 | 2888 | 17-May-15 |
| 9466 | 2 | 3 | | | III-1 | Grcc10 | 113246 | 12-May-15 |
| 9468 | 2 | 3 | | | III-1 | Greb1l | 80000 | 4-May-15 |
| 9473 | 2 | 3 | | | III-1 | Grhl3 | 57822 | 4-May-15 |
| 9476 | 2 | 3 | | | III-1 | Gria2 | 2891 | 4-May-15 |
| 9484 | 2 | 3 | | | III-1 | Grik2 | 2898 | 7-Jun-15 |
| 9488 | 2 | 3 | | | III-1 | Grin1 | 2902 | 7-Jun-15 |
| 9490 | 2 | 3 | | | III-1 | Grin2a | 2903 | 31-May-15 |
| 9493 | 2 | 3 | | | III-1 | Grin2d | 2906 | 4-May-15 |
| 9497 | 2 | 3 | | | III-1 | Grip1 | 23426 | 7-Jun-15 |
| 9499 | 2 | 3 | | | III-1 | Grip2 | 80852 | 4-May-15 |
| 9500 | 2 | 3 | | | III-1 | Gripap1 | 56850 | 4-May-15 |
| 9507 | 2 | 3 | | | III-1 | Grm3 | 2913 | 4-May-15 |
| 9514 | 2 | 3 | | | III-1 | Grp | 2922 | 17-May-15 |
| 9517 | 2 | 3 | | | III-1 | Grpr | 2925 | 17-May-15 |
| 9519 | 2 | 3 | | | III-1 | Grsf1 | 2926 | 31-May-15 |
| 9522 | 2 | 3 | | | III-1 | Grxcr1 | 389207 | 4-May-15 |
| 9525 | 2 | 3 | | | III-1 | Gsc | 145258 | 4-May-15 |
| 9529 | 2 | 3 | | | III-1 | Gsdma3 | | |
| 9540 | 2 | 3 | | | III-1 | Gsg1l | 146395 | 4-May-15 |
| 9542 | 2 | 3 | | | III-1 | Gsk3a | 2931 | 28-May-15 |
| 9545 | 2 | 3 | | | III-1 | Gsn | 2934 | 12-May-15 |
| 9546 | 2 | 3 | | | III-1 | Gspt1 | 2935 | 4-May-15 |
| 9554 | 2 | 3 | | | III-1 | Gstcd | 79807 | 4-May-15 |
| 9564 | 2 | 3 | | | III-1 | Gsto2 | 119391 | 12-May-15 |
| 9569 | 2 | 3 | | | III-1 | Gstt3 | | |
| 9570 | 2 | 3 | | | III-1 | Gstt4 | 25774 | 4-May-15 |
| 9572 | 2 | 3 | | | III-1 | Gsx1 | 219409 | 28-May-15 |
| 9575 | 2 | 3 | | | III-1 | Gtdc1 | 79712 | 21-May-15 |
| 9579 | 2 | 3 | | | III-1 | Gtf2b | 2959 | 4-May-15 |
| 9584 | 2 | 3 | | | III-1 | Gtf2h1 | 2965 | 4-May-15 |
| 9588 | 2 | 3 | | | III-1 | Gtf2h5 | 404672 | 4-Jun-15 |
| 9589 | 2 | 3 | | | III-1 | Gtf2i | 2969 | 7-Jun-15 |
| 9591 | 2 | 3 | | | III-1 | Gtf2ird2 | 84163 | 4-May-15 |
| 9592 | 2 | 3 | | | III-1 | Gtf3a | 2971 | 4-May-15 |
| 9602 | 2 | 3 | | | III-1 | Gtpbp2 | 54676 | 2-Jun-15 |
| 9605 | 2 | 3 | | | III-1 | Gtpbp6 | 8225 | 21-May-15 |
| 9607 | 2 | 3 | | | III-1 | Gtse1 | 51512 | 12-May-15 |
| 9614 | 2 | 3 | | | III-1 | Gucd1 | 83606 | 4-May-15 |
| 9615 | 2 | 3 | | | III-1 | Gucy1a2 | 2977 | 4-May-15 |
| 9617 | 2 | 3 | | | III-1 | Gucy1b2 | 2974 | 4-May-15 |
| 9627 | 2 | 3 | | | III-1 | Gulp1 | 51454 | 12-May-15 |
| 9628 | 2 | 3 | | | III-1 | Gusb | 2990 | 4-May-15 |
| 9634 | 2 | 3 | | | III-1 | Gykl1 | | |
| 9638 | 2 | 3 | | | III-1 | Gys1 | 2997 | 12-May-15 |
| 9639 | 2 | 3 | | | III-1 | Gys2 | 2998 | 4-May-15 |
| 9643 | 2 | 3 | | | III-1 | Gzmc | | |
| 9644 | 2 | 3 | | | III-1 | Gzmd | | |
| 9650 | 2 | 3 | | | III-1 | Gzmn | | |
| 9655 | 2 | 3 | | | III-1 | H1foo | 132243 | 12-May-15 |
| 9663 | 2 | 3 | | | III-1 | H2afv | 94239 | 2-Jun-15 |
| 9665 | 2 | 3 | | | III-1 | H2afy | 9555 | 12-May-15 |
| 9668 | 2 | 3 | | | III-1 | H2afz | 3015 | 12-May-15 |
| 9672 | 2 | 3 | | | III-1 | H2-DMa | | |
| 9673 | 2 | 3 | | | III-1 | H2-DMb1 | | |
| 9679 | 2 | 3 | | | III-1 | H2-K2 | | |
| 9680 | 2 | 3 | | | III-1 | H2-Ke2 | 10471 | 4-May-15 |
| 9682 | 2 | 3 | | | III-1 | H2-L | | |
| 9695 | 2 | 3 | | | III-1 | H2-Oa | | |
| 9709 | 2 | 3 | | | III-1 | H2-T24 | | |
| 9712 | 2 | 3 | | | III-1 | H3f3a | 3020 | 12-May-15 |
| 9716 | 2 | 3 | | | III-1 | H6pd | 9563 | 13-May-15 |
| 9717 | 2 | 3 | | | III-1 | Haao | 23498 | 4-May-15 |
| 9718 | 2 | 3 | | | III-1 | Habp2 | 3026 | 23-May-15 |
| 9719 | 2 | 3 | | | III-1 | Habp4 | 22927 | 12-May-15 |
| 9722 | 2 | 3 | | | III-1 | Hadh | 3033 | 7-Jun-15 |
| 9725 | 2 | 3 | | | III-1 | Hagh | 3029 | 12-May-15 |
| 9726 | 2 | 3 | | | III-1 | Haghl | 84264 | 21-May-15 |
| 9727 | 2 | 3 | | | III-1 | Hal | 3034 | 12-May-15 |
| 9730 | 2 | 3 | | | III-1 | Hand1 | 9421 | 17-May-15 |
| 9735 | 2 | 3 | | | III-1 | Hapln1 | 1404 | 28-May-15 |
| 9743 | 2 | 3 | | | III-1 | Has2 | 3037 | 14-May-15 |
| 9747 | 2 | 3 | | | III-1 | Haus1 | 115106 | 4-May-15 |
| 9748 | 2 | 3 | | | III-1 | Haus2 | 55142 | 4-May-15 |
| 9751 | 2 | 3 | | | III-1 | Haus5 | 23354 | 4-May-15 |
| 9752 | 2 | 3 | | | III-1 | Haus6 | 54801 | 4-May-15 |
| 9756 | 2 | 3 | | | III-1 | Havcr2 | 84868 | 24-May-15 |
| 9757 | 2 | 3 | | | III-1 | Hax1 | 10456 | 4-May-15 |
| 9762 | 2 | 3 | | | III-1 | Hbb-bh1 | | |
| 9766 | 2 | 3 | | | III-1 | Hbb-y | | |
| 9768 | 2 | 3 | | | III-1 | Hbp1 | 26959 | 4-May-15 |
| 9779 | 2 | 3 | | | III-1 | Hck | 3055 | 12-May-15 |
| 9785 | 2 | 3 | | | III-1 | Hcrt | 3060 | 17-May-15 |
| 9787 | 2 | 3 | | | III-1 | Hcrtr2 | 3062 | 16-Jun-15 |
| 9797 | 2 | 3 | | | III-1 | Hdac7 | 51564 | 7-Jun-15 |
| 9807 | 2 | 3 | | | III-1 | Hdhd1a | 8226 | 28-May-15 |
| 9815 | 2 | 3 | | | III-1 | Heatr5a | 25938 | 4-May-15 |
| 9817 | 2 | 3 | | | III-1 | Heatr6 | 63897 | 4-May-15 |
| 9822 | 2 | 3 | | | III-1 | Hectd1 | 25831 | 12-May-15 |
| 9824 | 2 | 3 | | | III-1 | Hectd3 | 79654 | 4-May-15 |
| 9831 | 2 | 3 | | | III-1 | Helt | 391723 | 4-May-15 |
| 9833 | 2 | 3 | | | III-1 | Helz2 | 85441 | 12-May-15 |
| 9837 | 2 | 3 | | | III-1 | Henmt1 | 113802 | 4-May-15 |
| 9839 | 2 | 3 | | | III-1 | Hepacam2 | 253012 | 4-May-15 |
| 9840 | 2 | 3 | | | III-1 | Heph | 9843 | 4-May-15 |
| 9841 | 2 | 3 | | | III-1 | Hephl1 | 341208 | 4-May-15 |
| 9843 | 2 | 3 | | | III-1 | Herc2 | 8924 | 23-May-15 |
| 9844 | 2 | 3 | | | III-1 | Herc3 | 8916 | 4-May-15 |
| 9845 | 2 | 3 | | | III-1 | Herc4 | 26091 | 4-May-15 |
| 9848 | 2 | 3 | | | III-1 | Herpud2 | 64224 | 4-May-15 |
| 9850 | 2 | 3 | | | III-1 | Hes2 | 54626 | 4-May-15 |
| 9855 | 2 | 3 | | | III-1 | Hesx1 | 8820 | 4-May-15 |
| 9856 | 2 | 3 | | | III-1 | Hexa | 3073 | 23-May-15 |
| 9858 | 2 | 3 | | | III-1 | Hexdc | 284004 | 4-May-15 |
| 9862 | 2 | 3 | | | III-1 | Hey2 | 23493 | 4-May-15 |
| 9864 | 2 | 3 | | | III-1 | Hfe | 3077 | 23-May-15 |
| 9870 | 2 | 3 | | | III-1 | Hgs | 9146 | 4-May-15 |

Fig.22 - 70

| | | | | | | | | |
|---|---|---|---|---|---|---|---|---|
| 9872 | 2 | 3 | | | III-1 | Hhat | 55733 | 4-May-15 |
| 9882 | 2 | 3 | | | III-1 | Hibch | 26275 | 12-May-15 |
| 9883 | 2 | 3 | | | III-1 | Hic1 | 3090 | 4-May-15 |
| 9884 | 2 | 3 | | | III-1 | Hic2 | 23119 | 4-May-15 |
| 9889 | 2 | 3 | | | III-1 | Higd1a | 25994 | 4-May-15 |
| 9895 | 2 | 3 | | | III-1 | Hinfp | 25988 | 4-May-15 |
| 9898 | 2 | 3 | | | III-1 | Hint3 | 135114 | 4-May-15 |
| 9899 | 2 | 3 | | | III-1 | Hip1 | 3092 | 7-Jun-15 |
| 9901 | 2 | 3 | | | III-1 | Hipk1 | 204851 | 4-May-15 |
| 9904 | 2 | 3 | | | III-1 | Hipk4 | 147746 | 4-May-15 |
| 9905 | 2 | 3 | | | III-1 | Hira | 7290 | 4-May-15 |
| 9973 | 2 | 3 | | | III-1 | Hist4h4 | 121504 | 12-May-15 |
| 9975 | 2 | 3 | | | III-1 | Hivep2 | 3097 | 12-May-15 |
| 9978 | 2 | 3 | | | III-1 | Hk1 | 3098 | 7-Jun-15 |
| 9981 | 2 | 3 | | | III-1 | Hk3 | 3101 | 7-Jun-15 |
| 9983 | 2 | 3 | | | III-1 | Hlcs | 3141 | 4-May-15 |
| 9985 | 2 | 3 | | | III-1 | Hltf | 6596 | 4-May-15 |
| 9989 | 2 | 3 | | | III-1 | Hmces | 56941 | 4-May-15 |
| 9995 | 2 | 3 | | | III-1 | Hmga2 | 8091 | 31-May-15 |
| 9996 | 2 | 3 | | | III-1 | Hmga2-ps1 | | |
| 10002 | 2 | 3 | | | III-1 | Hmgcl | 3155 | 23-May-15 |
| 10008 | 2 | 3 | | | III-1 | Hmgn2 | 3151 | 4-May-15 |
| 10012 | 2 | 3 | | | III-1 | Hmgxb4 | 10042 | 4-May-15 |
| 10016 | 2 | 3 | | | III-1 | Hmox2 | 3163 | 4-May-15 |
| 10018 | 2 | 3 | | | III-1 | Hmx2 | 3167 | 4-May-15 |
| 10020 | 2 | 3 | | | III-1 | Hn1 | 51155 | 7-Jun-15 |
| 10022 | 2 | 3 | | | III-1 | Hnf1a | 6927 | 21-May-15 |
| 10025 | 2 | 3 | | | III-1 | Hnf4aos | | |
| 10036 | 2 | 3 | | | III-1 | Hnrnpf | 3185 | 4-May-15 |
| 10049 | 2 | 3 | | | III-1 | Homer1 | 9456 | 4-May-15 |
| 10051 | 2 | 3 | | | III-1 | Homer3 | 9454 | 4-May-15 |
| 10055 | 2 | 3 | | | III-1 | Hook3 | 84376 | 4-May-15 |
| 10057 | 2 | 3 | | | III-1 | Hormad1 | 84072 | 4-May-15 |
| 10061 | 2 | 3 | | | III-1 | Hoxa1 | 3198 | 7-Jun-15 |
| 10063 | 2 | 3 | | | III-1 | Hoxa11 | 3207 | 4-May-15 |
| 10065 | 2 | 3 | | | III-1 | Hoxa13 | 3209 | 23-May-15 |
| 10067 | 2 | 3 | | | III-1 | Hoxa3 | 3200 | 21-May-15 |
| 10068 | 2 | 3 | | | III-1 | Hoxa4 | 3201 | 12-May-15 |
| 10069 | 2 | 3 | | | III-1 | Hoxa5 | 3202 | 28-May-15 |
| 10072 | 2 | 3 | | | III-1 | Hoxa9 | 3205 | 31-May-15 |
| 10073 | 2 | 3 | | | III-1 | Hoxb1 | 3211 | 7-Jun-15 |
| 10078 | 2 | 3 | | | III-1 | Hoxb5 | 3215 | 28-May-15 |
| 10083 | 2 | 3 | | | III-1 | Hoxc10 | 3226 | 4-May-15 |
| 10085 | 2 | 3 | | | III-1 | Hoxc12 | 3228 | 4-May-15 |
| 10098 | 2 | 3 | | | III-1 | Hoxd3os1 | | |
| 10112 | 2 | 3 | | | III-1 | Hprt | 3251 | 23-May-15 |
| 10115 | 2 | 3 | | | III-1 | Hps4 | 89781 | 23-May-15 |
| 10130 | 2 | 3 | | | III-1 | Hrh3 | 11255 | 4-May-15 |
| 10132 | 2 | 3 | | | III-1 | Hrk | 8739 | 4-May-15 |
| 10134 | 2 | 3 | | | III-1 | Hrsp12 | 10247 | 4-May-15 |
| 10135 | 2 | 3 | | | III-1 | Hs1bp3 | 64342 | 4-May-15 |
| 10138 | 2 | 3 | | | III-1 | Hs3st2 | 9956 | 4-May-15 |
| 10141 | 2 | 3 | | | III-1 | Hs3st4 | 9951 | 4-May-15 |
| 10154 | 2 | 3 | | | III-1 | Hsd17b11 | 51170 | 4-May-15 |
| 10158 | 2 | 3 | | | III-1 | Hsd17b2 | 3294 | 12-May-15 |
| 10163 | 2 | 3 | | | III-1 | Hsd3b1 | 3283 | 12-May-15 |
| 10164 | 2 | 3 | | | III-1 | Hsd3b2 | 3284 | 12-May-15 |
| 10172 | 2 | 3 | | | III-1 | Hsf1 | 3297 | 24-May-15 |
| 10177 | 2 | 3 | | | III-1 | Hsf5 | 124535 | 4-May-15 |
| 10181 | 2 | 3 | | | III-1 | Hsp90ab1 | 3326 | 21-May-15 |
| 10184 | 2 | 3 | | | III-1 | Hspa12b | 116835 | 4-May-15 |
| 10194 | 2 | 3 | | | III-1 | Hspa8 | 3312 | 4-May-15 |
| 10195 | 2 | 3 | | | III-1 | Hspa9 | 3313 | 31-May-15 |
| 10204 | 2 | 3 | | | III-1 | Hspbap1 | 79663 | 7-Jun-15 |
| 10208 | 2 | 3 | | | III-1 | Hspg2 | 3339 | 12-May-15 |
| 10209 | 2 | 3 | | | III-1 | Hsph1 | 10808 | 12-May-15 |
| 10211 | 2 | 3 | | | III-1 | Htatsf1 | 27336 | 4-May-15 |
| 10214 | 2 | 3 | | | III-1 | Htr1d | 3352 | 24-May-15 |
| 10219 | 2 | 3 | | | III-1 | Htr3a | 3359 | 12-May-15 |
| 10220 | 2 | 3 | | | III-1 | Htr3b | 9177 | 12-May-15 |
| 10221 | 2 | 3 | | | III-1 | Htr4 | 3360 | 21-May-15 |
| 10224 | 2 | 3 | | | III-1 | Htr6 | 3362 | 4-May-15 |
| 10227 | 2 | 3 | | | III-1 | Htra2 | 27429 | 23-May-15 |
| 10230 | 2 | 3 | | | III-1 | Htt | 3064 | 7-Jun-15 |
| 10247 | 2 | 3 | | | III-1 | Hypk | 25764 | 4-May-15 |
| 10249 | 2 | 3 | | | III-1 | I730030J21Rik | | |
| 10252 | 2 | 3 | | | III-1 | Iah1 | 285148 | 14-May-15 |
| 10254 | 2 | 3 | | | III-1 | Iars | 3376 | 4-May-15 |
| 10258 | 2 | 3 | | | III-1 | Ibtk | 25998 | 4-May-15 |
| 10262 | 2 | 3 | | | III-1 | Icam2 | 3384 | 12-May-15 |
| 10271 | 2 | 3 | | | III-1 | Id2 | 3398 | 4-May-15 |
| 10273 | 2 | 3 | | | III-1 | Id4 | 3400 | 24-May-15 |
| 10274 | 2 | 3 | | | III-1 | Ide | 3416 | 24-May-15 |
| 10275 | 2 | 3 | | | III-1 | Idh1 | 3417 | 7-Jun-15 |
| 10277 | 2 | 3 | | | III-1 | Idh3a | 3419 | 12-May-15 |
| 10278 | 2 | 3 | | | III-1 | Idh3b | 3420 | 23-May-15 |
| 10285 | 2 | 3 | | | III-1 | Ids | 3423 | 23-May-15 |
| 10289 | 2 | 3 | | | III-1 | Ier3ip1 | 51124 | 4-May-15 |
| 10291 | 2 | 3 | | | III-1 | Ier5l | 389792 | 4-May-15 |
| 10292 | 2 | 3 | | | III-1 | Iffo1 | 25900 | 4-May-15 |
| 10294 | 2 | 3 | | | III-1 | Ifi202b | | |
| 10295 | 2 | 3 | | | III-1 | Ifi203 | | |
| 10297 | 2 | 3 | | | III-1 | Ifi205 | | |
| 10298 | 2 | 3 | | | III-1 | Ifi27 | 3429 | 17-May-15 |
| 10302 | 2 | 3 | | | III-1 | Ifi35 | 3430 | 12-May-15 |
| 10309 | 2 | 3 | | | III-1 | Ifit3 | 3437 | 21-May-15 |
| 10311 | 2 | 3 | | | III-1 | Ifitm10 | 402778 | 4-May-15 |
| 10312 | 2 | 3 | | | III-1 | Ifitm2 | 10581 | 4-May-15 |
| 10313 | 2 | 3 | | | III-1 | Ifitm3 | 10410 | 31-May-15 |
| 10343 | 2 | 3 | | | III-1 | Ifnz | | |
| 10346 | 2 | 3 | | | III-1 | Ift122 | 55764 | 12-May-15 |
| 10348 | 2 | 3 | | | III-1 | Ift172 | 26160 | 4-Jun-15 |
| 10351 | 2 | 3 | | | III-1 | Ift27 | 11020 | 4-May-15 |
| 10352 | 2 | 3 | | | III-1 | Ift43 | 112752 | 4-May-15 |
| 10353 | 2 | 3 | | | III-1 | Ift46 | 56912 | 12-May-15 |
| 10357 | 2 | 3 | | | III-1 | Ift80 | 57560 | 4-May-15 |
| 10359 | 2 | 3 | | | III-1 | Ift88 | 8100 | 21-May-15 |
| 10363 | 2 | 3 | | | III-1 | Igdcc4 | 57722 | 4-May-15 |
| 10366 | 2 | 3 | | | III-1 | Igf2 | 3481 | 24-May-15 |
| 10367 | 2 | 3 | | | III-1 | Igf2bp1 | 10642 | 1-Jun-15 |
| 10376 | 2 | 3 | | | III-1 | Igfbp4 | 3487 | 4-May-15 |
| 10379 | 2 | 3 | | | III-1 | Igfbp7 | 3490 | 12-May-15 |
| 10380 | 2 | 3 | | | III-1 | Igfbpl1 | 347252 | 4-May-15 |
| 10382 | 2 | 3 | | | III-1 | Igflr1 | 79713 | 4-May-15 |
| 10384 | 2 | 3 | | | III-1 | Ighmbp2 | 3508 | 23-May-15 |
| 10389 | 2 | 3 | | | III-1 | Igsf1 | 3547 | 4-May-15 |
| 10392 | 2 | 3 | | | III-1 | Igsf21 | 84966 | 4-May-15 |
| 10394 | 2 | 3 | | | III-1 | Igsf3 | 3321 | 4-May-15 |
| 10397 | 2 | 3 | | | III-1 | Igsf8 | 93185 | 4-May-15 |
| 10398 | 2 | 3 | | | III-1 | Igsf9 | 57549 | 4-May-15 |
| 10404 | 2 | 3 | | | III-1 | Ikbip | 121457 | 4-May-15 |
| 10405 | 2 | 3 | | | III-1 | Ikbkap | 8518 | 23-May-15 |
| 10409 | 2 | 3 | | | III-1 | Ikzf1 | 10320 | 12-May-15 |
| 10413 | 2 | 3 | | | III-1 | Ikzf5 | 64376 | 2-Jun-15 |
| 10417 | 2 | 3 | | | III-1 | Il11 | 3589 | 4-May-15 |
| 10419 | 2 | 3 | | | III-1 | Il11ra2 | | |
| 10422 | 2 | 3 | | | III-1 | Il12rb1 | 3594 | 4-May-15 |
| 10424 | 2 | 3 | | | III-1 | Il13 | 3596 | 4-May-15 |
| 10425 | 2 | 3 | | | III-1 | Il13ra1 | 3597 | 8-May-15 |
| 10426 | 2 | 3 | | | III-1 | Il13ra2 | 3598 | 12-May-15 |
| 10428 | 2 | 3 | | | III-1 | Il15ra | 3601 | 4-May-15 |
| 10429 | 2 | 3 | | | III-1 | Il16 | 3603 | 4-May-15 |
| 10433 | 2 | 3 | | | III-1 | Il17d | 53342 | 4-May-15 |
| 10434 | 2 | 3 | | | III-1 | Il17f | 112744 | 4-May-15 |
| 10438 | 2 | 3 | | | III-1 | Il17rd | 54756 | 4-May-15 |
| 10442 | 2 | 3 | | | III-1 | Il18r1 | 8809 | 24-May-15 |
| 10444 | 2 | 3 | | | III-1 | Il19 | 29949 | 4-May-15 |
| 10449 | 2 | 3 | | | III-1 | Il1f5 | 26525 | 7-Jun-15 |
| 10450 | 2 | 3 | | | III-1 | Il1f6 | 27179 | 4-May-15 |
| 10453 | 2 | 3 | | | III-1 | Il1r1 | 3554 | 12-May-15 |
| 10455 | 2 | 3 | | | III-1 | Il1rap | 3556 | 12-May-15 |
| 10459 | 2 | 3 | | | III-1 | Il1rl2 | 8808 | 4-May-15 |
| 10461 | 2 | 3 | | | III-1 | Il2 | 3558 | 12-May-15 |
| 10465 | 2 | 3 | | | III-1 | Il21 | 59067 | 7-Jun-15 |
| 10472 | 2 | 3 | | | III-1 | Il24 | 11009 | 4-May-15 |
| 10476 | 2 | 3 | | | III-1 | Il2ra | 3559 | 17-May-15 |
| 10478 | 2 | 3 | | | III-1 | Il2rg | 3561 | 23-May-15 |
| 10484 | 2 | 3 | | | III-1 | Il3ra | 3563 | 4-May-15 |
| 10485 | 2 | 3 | | | III-1 | Il4 | 3565 | 31-May-15 |
| 10488 | 2 | 3 | | | III-1 | Il5 | 3567 | 28-May-15 |
| 10489 | 2 | 3 | | | III-1 | Il5ra | 3568 | 12-May-15 |
| 10492 | 2 | 3 | | | III-1 | Il6st | 3572 | 12-May-15 |
| 10495 | 2 | 3 | | | III-1 | Il9 | 3578 | 7-Jun-15 |
| 10499 | 2 | 3 | | | III-1 | Ilf2 | 3608 | 4-May-15 |
| 10500 | 2 | 3 | | | III-1 | Ilf3 | 3609 | 12-May-15 |
| 10502 | 2 | 3 | | | III-1 | Ilkap | 80895 | 7-Jun-15 |
| 10503 | 2 | 3 | | | III-1 | Iltifb | | |
| 10507 | 2 | 3 | | | III-1 | Immt | 10989 | 4-May-15 |
| 10513 | 2 | 3 | | | III-1 | Impad1 | 54928 | 4-May-15 |
| 10515 | 2 | 3 | | | III-1 | Impdh2 | 3615 | 31-May-15 |
| 10516 | 2 | 3 | | | III-1 | Impg1 | 3617 | 12-May-15 |
| 10518 | 2 | 3 | | | III-1 | Ina | 9118 | 17-May-15 |
| 10523 | 2 | 3 | | | III-1 | Ing1 | 3621 | 17-May-15 |
| 10525 | 2 | 3 | | | III-1 | Ing3 | 54556 | 4-May-15 |
| 10539 | 2 | 3 | | | III-1 | Ino80dos | | |
| 10540 | 2 | 3 | | | III-1 | Ino80e | 283899 | 12-May-15 |
| 10542 | 2 | 3 | | | III-1 | Inpp4a | 3631 | 12-May-15 |
| 10552 | 2 | 3 | | | III-1 | Ins1 | 2305 | 31-May-15 |
| 10554 | 2 | 3 | | | III-1 | Insc | 387755 | 4-May-15 |
| 10558 | 2 | 3 | | | III-1 | Insl5 | 10022 | 4-May-15 |
| 10561 | 2 | 3 | | | III-1 | Insm2 | 84684 | 21-May-15 |
| 10564 | 2 | 3 | | | III-1 | Ints1 | 26173 | 4-May-15 |
| 10572 | 2 | 3 | | | III-1 | Ints7 | 25896 | 4-May-15 |
| 10573 | 2 | 3 | | | III-1 | Ints8 | 55656 | 4-May-15 |
| 10580 | 2 | 3 | | | III-1 | Ipcef1 | 26034 | 4-May-15 |
| 10582 | 2 | 3 | | | III-1 | Ipo11 | 51194 | 4-May-15 |
| 10584 | 2 | 3 | | | III-1 | Ipo4 | 79711 | 4-May-15 |
| 10587 | 2 | 3 | | | III-1 | Ipo8 | 10526 | 4-May-15 |
| 10591 | 2 | 3 | | | III-1 | Ipw | 3653 | 23-May-15 |
| 10594 | 2 | 3 | | | III-1 | Iqcc | 55721 | 4-May-15 |
| 10596 | 2 | 3 | | | III-1 | Iqce | 23288 | 4-May-15 |
| 10599 | 2 | 3 | | | III-1 | Iqcf4 | 100506840 | 4-May-15 |
| 10606 | 2 | 3 | | | III-1 | Iqgap1 | 8826 | 17-May-15 |
| 10608 | 2 | 3 | | | III-1 | Iqgap3 | 128239 | 17-May-15 |
| 10610 | 2 | 3 | | | III-1 | Iqsec2 | 23096 | 2-Jun-15 |
| 10611 | 2 | 3 | | | III-1 | Iqsec3 | 440073 | 4-May-15 |
| 10616 | 2 | 3 | | | III-1 | Irak3 | 11213 | 17-May-15 |
| 10617 | 2 | 3 | | | III-1 | Irak4 | 51135 | 3-May-15 |
| 10623 | 2 | 3 | | | III-1 | Irf2bpl | 64207 | 12-May-15 |
| 10624 | 2 | 3 | | | III-1 | Irf3 | 3661 | 7-Jun-15 |
| 10628 | 2 | 3 | | | III-1 | Irf7 | 3665 | 21-May-15 |
| 10630 | 2 | 3 | | | III-1 | Irf9 | 10379 | 4-May-15 |
| 10633 | 2 | 3 | | | III-1 | Irgm1 | 345611 | 21-May-15 |
| 10639 | 2 | 3 | | | III-1 | Irs4 | 8471 | 14-May-15 |

Fig.22 - 71

| | | | | | | | | |
|---|---|---|---|---|---|---|---|---|
| 10642 | 2 | 3 | | | III-1 | Irx3 | 79191 | 24-May-15 |
| 10643 | 2 | 3 | | | III-1 | Irx4 | 50805 | 4-May-15 |
| 10644 | 2 | 3 | | | III-1 | Irx5 | 10265 | 4-May-15 |
| 10647 | 2 | 3 | | | III-1 | Isca2 | 122961 | 31-May-15 |
| 10651 | 2 | 3 | | | III-1 | Isg20l2 | 81875 | 4-May-15 |
| 10658 | 2 | 3 | | | III-1 | Isoc1 | 51015 | 4-May-15 |
| 10660 | 2 | 3 | | | III-1 | Isoc2b | | |
| 10666 | 2 | 3 | | | III-1 | Itch | 83737 | 4-May-15 |
| 10670 | 2 | 3 | | | III-1 | Itga1 | 3672 | 12-May-15 |
| 10671 | 2 | 3 | | | III-1 | Itga10 | 8515 | 12-May-15 |
| 10674 | 2 | 3 | | | III-1 | Itga2b | 3674 | 12-May-15 |
| 10676 | 2 | 3 | | | III-1 | Itga4 | 3676 | 24-May-15 |
| 10677 | 2 | 3 | | | III-1 | Itga5 | 3678 | 4-May-15 |
| 10678 | 2 | 3 | | | III-1 | Itga6 | 3655 | 23-May-15 |
| 10679 | 2 | 3 | | | III-1 | Itga7 | 3679 | 23-May-15 |
| 10681 | 2 | 3 | | | III-1 | Itga9 | 3680 | 12-May-15 |
| 10683 | 2 | 3 | | | III-1 | Itgae | 3682 | 24-May-15 |
| 10684 | 2 | 3 | | | III-1 | Itgal | 3683 | 12-May-15 |
| 10686 | 2 | 3 | | | III-1 | Itgav | 3685 | 31-May-15 |
| 10694 | 2 | 3 | | | III-1 | Itgb3bp | 23421 | 4-May-15 |
| 10696 | 2 | 3 | | | III-1 | Itgb5 | 3693 | 17-May-15 |
| 10697 | 2 | 3 | | | III-1 | Itgb6 | 3694 | 4-May-15 |
| 10699 | 2 | 3 | | | III-1 | Itgb8 | 3696 | 31-May-15 |
| 10700 | 2 | 3 | | | III-1 | Itgbl1 | 9358 | 4-May-15 |
| 10705 | 2 | 3 | | | III-1 | Itih5 | 80760 | 17-May-15 |
| 10710 | 2 | 3 | | | III-1 | Itm2c | 81618 | 4-May-15 |
| 10711 | 2 | 3 | | | III-1 | Itpa | 3704 | 31-May-15 |
| 10712 | 2 | 3 | | | III-1 | Itpk1 | 3705 | 4-May-15 |
| 10714 | 2 | 3 | | | III-1 | Itpkb | 3707 | 4-May-15 |
| 10717 | 2 | 3 | | | III-1 | Itpr2 | 3709 | 21-May-15 |
| 10718 | 2 | 3 | | | III-1 | Itpr3 | 3710 | 12-May-15 |
| 10720 | 2 | 3 | | | III-1 | Itpripl1 | 150771 | 4-May-15 |
| 10722 | 2 | 3 | | | III-1 | Itsn1 | 6453 | 14-May-15 |
| 10724 | 2 | 3 | | | III-1 | Ivd | 3712 | 23-May-15 |
| 10725 | 2 | 3 | | | III-1 | Ivl | 3713 | 12-May-15 |
| 10727 | 2 | 3 | | | III-1 | Iws1 | 55677 | 14-May-15 |
| 10729 | 2 | 3 | | | III-1 | Izumo1 | 284359 | 4-May-15 |
| 10733 | 2 | 3 | | | III-1 | Jade1 | 79960 | 4-May-15 |
| 10736 | 2 | 3 | | | III-1 | Jag1 | 182 | 31-May-15 |
| 10738 | 2 | 3 | | | III-1 | Jagn1 | 84522 | 4-May-15 |
| 10746 | 2 | 3 | | | III-1 | Jam3 | 83700 | 4-May-15 |
| 10749 | 2 | 3 | | | III-1 | Jdp2 | 122953 | 4-May-15 |
| 10750 | 2 | 3 | | | III-1 | Jkamp | 51528 | 4-May-15 |
| 10751 | 2 | 3 | | | III-1 | Jmjd1c | 221037 | 4-May-15 |
| 10753 | 2 | 3 | | | III-1 | Jmjd6 | 23210 | 4-May-15 |
| 10754 | 2 | 3 | | | III-1 | Jmjd7 | 100137047 | 4-May-15 |
| 10755 | 2 | 3 | | | III-1 | Jmjd7-pla2g4b | 8681 | 4-May-15 |
| 10756 | 2 | 3 | | | III-1 | Jmjd8 | 339123 | 21-May-15 |
| 10770 | 2 | 3 | | | III-1 | Junb | 3726 | 7-Jun-15 |
| 10771 | 2 | 3 | | | III-1 | Jund | 3727 | 4-May-15 |
| 10774 | 2 | 3 | | | III-1 | Kank1 | 23189 | 12-May-15 |
| 10775 | 2 | 3 | | | III-1 | Kank2 | 25959 | 4-May-15 |
| 10776 | 2 | 3 | | | III-1 | Kank3 | 256949 | 12-May-15 |
| 10777 | 2 | 3 | | | III-1 | Kank4 | 163782 | 4-May-15 |
| 10781 | 2 | 3 | | | III-1 | Kansl2 | 54934 | 12-May-15 |
| 10789 | 2 | 3 | | | III-1 | Kat6b | 23522 | 4-May-15 |
| 10790 | 2 | 3 | | | III-1 | Kat7 | 11143 | 24-May-15 |
| 10792 | 2 | 3 | | | III-1 | Katna1 | 11104 | 4-May-15 |
| 10793 | 2 | 3 | | | III-1 | Katnal1 | 84056 | 4-May-15 |
| 10799 | 2 | 3 | | | III-1 | Kbtbd11 | 9920 | 4-May-15 |
| 10800 | 2 | 3 | | | III-1 | Kbtbd12 | 166348 | 4-May-15 |
| 10802 | 2 | 3 | | | III-1 | Kbtbd2 | 25948 | 4-May-15 |
| 10804 | 2 | 3 | | | III-1 | Kbtbd4 | 55709 | 4-May-15 |
| 10807 | 2 | 3 | | | III-1 | Kcmf1 | 56888 | 4-May-15 |
| 10808 | 2 | 3 | | | III-1 | Kcna1 | 3736 | 23-May-15 |
| 10809 | 2 | 3 | | | III-1 | Kcna10 | 3744 | 4-May-15 |
| 10814 | 2 | 3 | | | III-1 | Kcna6 | 3742 | 4-May-15 |
| 10817 | 2 | 3 | | | III-1 | Kcnab2 | 8514 | 4-May-15 |
| 10826 | 2 | 3 | | | III-1 | Kcnd2 | 3751 | 12-May-15 |
| 10835 | 2 | 3 | | | III-1 | Kcng1 | 3755 | 3-May-15 |
| 10842 | 2 | 3 | | | III-1 | Kcnh4 | 23415 | 4-May-15 |
| 10845 | 2 | 3 | | | III-1 | Kcnh7 | 90134 | 4-May-15 |
| 10850 | 2 | 3 | | | III-1 | Kcnip4 | 80333 | 4-May-15 |
| 10851 | 2 | 3 | | | III-1 | Kcnj1 | 3758 | 4-May-15 |
| 10878 | 2 | 3 | | | III-1 | Kcnk7 | 10089 | 4-May-15 |
| 10881 | 2 | 3 | | | III-1 | Kcnmb1 | 3779 | 12-May-15 |
| 10886 | 2 | 3 | | | III-1 | Kcnn1 | 3780 | 12-May-15 |
| 10887 | 2 | 3 | | | III-1 | Kcnn2 | 3781 | 4-May-15 |
| 10889 | 2 | 3 | | | III-1 | Kcnn4 | 3783 | 12-May-15 |
| 10895 | 2 | 3 | | | III-1 | Kcnq5 | 56479 | 12-May-15 |
| 10896 | 2 | 3 | | | III-1 | Kcnrg | 283518 | 4-May-15 |
| 10901 | 2 | 3 | | | III-1 | Kcnt2 | 343450 | 4-May-15 |
| 10905 | 2 | 3 | | | III-1 | Kcp | 375616 | 4-May-15 |
| 10907 | 2 | 3 | | | III-1 | Kctd10 | 83892 | 4-May-15 |
| 10909 | 2 | 3 | | | III-1 | Kctd12 | 115207 | 4-May-15 |
| 10910 | 2 | 3 | | | III-1 | Kctd12b | | |
| 10914 | 2 | 3 | | | III-1 | Kctd16 | 57528 | 4-May-15 |
| 10919 | 2 | 3 | | | III-1 | Kctd20 | 222658 | 4-May-15 |
| 10923 | 2 | 3 | | | III-1 | Kctd5 | 54442 | 4-May-15 |
| 10927 | 2 | 3 | | | III-1 | Kctd9 | 54793 | 4-May-15 |
| 10928 | 2 | 3 | | | III-1 | Kdelc1 | 79070 | 4-May-15 |
| 10939 | 2 | 3 | | | III-1 | Kdm3b | 51780 | 4-May-15 |
| 10949 | 2 | 3 | | | III-1 | Kdm6b | 23135 | 4-May-15 |
| 10950 | 2 | 3 | | | III-1 | Kdm7a | 80853 | 23-May-15 |
| 10951 | 2 | 3 | | | III-1 | Kdm8 | 79831 | 4-May-15 |
| 10952 | 2 | 3 | | | III-1 | Kdr | 3791 | 17-May-15 |
| 10954 | 2 | 3 | | | III-1 | Keap1 | 9817 | 31-May-15 |

| | | | | | | | | |
|---|---|---|---|---|---|---|---|---|
| 10959 | 2 | 3 | | | III-1 | Khdc1b | | |
| 10961 | 2 | 3 | | | III-1 | Khdc3 | | |
| 10962 | 2 | 3 | | | III-1 | Khdrbs1 | 10657 | 31-May-15 |
| 10966 | 2 | 3 | | | III-1 | Khnyn | 23351 | 12-May-15 |
| 10976 | 2 | 3 | | | III-1 | Kif17 | 57576 | 4-May-15 |
| 10977 | 2 | 3 | | | III-1 | Kif18a | 81930 | 4-May-15 |
| 10982 | 2 | 3 | | | III-1 | Kif1c | 10749 | 12-May-15 |
| 10983 | 2 | 3 | | | III-1 | Kif20a | 10112 | 4-May-15 |
| 10991 | 2 | 3 | | | III-1 | Kif26b | 55083 | 4-May-15 |
| 10992 | 2 | 3 | | | III-1 | Kif27 | 55582 | 4-May-15 |
| 11004 | 2 | 3 | | | III-1 | Kif6 | 221458 | 4-May-15 |
| 11006 | 2 | 3 | | | III-1 | Kif9 | 64147 | 4-May-15 |
| 11010 | 2 | 3 | | | III-1 | Kifc3 | 3801 | 4-May-15 |
| 11013 | 2 | 3 | | | III-1 | Kir3dl1 | 3811 | 12-May-15 |
| 11016 | 2 | 3 | | | III-1 | Kirrel2 | 84063 | 4-May-15 |
| 11018 | 2 | 3 | | | III-1 | Kis2 | | |
| 11023 | 2 | 3 | | | III-1 | Kiz | 55857 | 21-May-15 |
| 11026 | 2 | 3 | | | III-1 | Klc1 | 3831 | 12-May-15 |
| 11029 | 2 | 3 | | | III-1 | Klc4 | 89953 | 4-May-15 |
| 11035 | 2 | 3 | | | III-1 | Klf14 | 136259 | 4-May-15 |
| 11037 | 2 | 3 | | | III-1 | Klf16 | 83855 | 4-May-15 |
| 11038 | 2 | 3 | | | III-1 | Klf17 | 128209 | 24-May-15 |
| 11040 | 2 | 3 | | | III-1 | Klf3 | 51274 | 2-Jun-15 |
| 11041 | 2 | 3 | | | III-1 | Klf4 | 9314 | 31-May-15 |
| 11047 | 2 | 3 | | | III-1 | Klhdc1 | 122773 | 4-May-15 |
| 11048 | 2 | 3 | | | III-1 | Klhdc10 | 23008 | 12-May-15 |
| 11050 | 2 | 3 | | | III-1 | Klhdc3 | 116138 | 12-May-15 |
| 11051 | 2 | 3 | | | III-1 | Klhdc4 | 54758 | 4-May-15 |
| 11057 | 2 | 3 | | | III-1 | Klhl1 | 57626 | 4-May-15 |
| 11067 | 2 | 3 | | | III-1 | Klhl20 | 27252 | 4-May-15 |
| 11069 | 2 | 3 | | | III-1 | Klhl22 | 84861 | 4-May-15 |
| 11071 | 2 | 3 | | | III-1 | Klhl24 | 54800 | 4-May-15 |
| 11072 | 2 | 3 | | | III-1 | Klhl25 | 64410 | 4-May-15 |
| 11073 | 2 | 3 | | | III-1 | Klhl26 | 55295 | 4-May-15 |
| 11076 | 2 | 3 | | | III-1 | Klhl3 | 26249 | 4-May-15 |
| 11096 | 2 | 3 | | | III-1 | Klk11 | 11012 | 4-May-15 |
| 11097 | 2 | 3 | | | III-1 | Klk12 | 43849 | 4-May-15 |
| 11098 | 2 | 3 | | | III-1 | Klk13 | 26085 | 4-May-15 |
| 11100 | 2 | 3 | | | III-1 | Klk15 | 55554 | 12-May-15 |
| 11118 | 2 | 3 | | | III-1 | Klk7 | 5650 | 7-Jun-15 |
| 11119 | 2 | 3 | | | III-1 | Klk8 | 11202 | 4-May-15 |
| 11121 | 2 | 3 | | | III-1 | Klkb1 | 3818 | 4-May-15 |
| 11129 | 2 | 3 | | | III-1 | Klra18 | | |
| 11141 | 2 | 3 | | | III-1 | Klra8 | | |
| 11147 | 2 | 3 | | | III-1 | Klrb1f | | |
| 11150 | 2 | 3 | | | III-1 | Klrc2 | 3822 | 12-May-15 |
| 11159 | 2 | 3 | | | III-1 | Kmo | 8564 | 4-May-15 |
| 11161 | 2 | 3 | | | III-1 | Kmt2b | 9757 | 4-May-15 |
| 11162 | 2 | 3 | | | III-1 | Kmt2c | 58508 | 4-May-15 |
| 11163 | 2 | 3 | | | III-1 | Kmt2d | 8085 | 23-May-15 |
| 11164 | 2 | 3 | | | III-1 | Kmt2e | 55904 | 4-May-15 |
| 11165 | 2 | 3 | | | III-1 | Kncn | 148930 | 4-May-15 |
| 11169 | 2 | 3 | | | III-1 | Knop1 | 400506 | 4-May-15 |
| 11174 | 2 | 3 | | | III-1 | Kpna3 | 3839 | 4-May-15 |
| 11180 | 2 | 3 | | | III-1 | Kptn | 11133 | 4-May-15 |
| 11187 | 2 | 3 | | | III-1 | Krit1 | 889 | 31-May-15 |
| 11191 | 2 | 3 | | | III-1 | Krt12 | 3859 | 4-May-15 |
| 11196 | 2 | 3 | | | III-1 | Krt17 | 3872 | 22-May-15 |
| 11199 | 2 | 3 | | | III-1 | Krt2 | 3849 | 4-May-15 |
| 11201 | 2 | 3 | | | III-1 | Krt222 | 125113 | 4-May-15 |
| 11203 | 2 | 3 | | | III-1 | Krt24 | 192666 | 4-May-15 |
| 11206 | 2 | 3 | | | III-1 | Krt27 | 342574 | 4-May-15 |
| 11217 | 2 | 3 | | | III-1 | Krt40 | 125115 | 4-May-15 |
| 11223 | 2 | 3 | | | III-1 | Krt71 | 112802 | 4-May-15 |
| 11233 | 2 | 3 | | | III-1 | Krt80 | 144501 | 4-May-15 |
| 11234 | 2 | 3 | | | III-1 | Krt81 | 3887 | 12-May-15 |
| 11238 | 2 | 3 | | | III-1 | Krt85 | 3891 | 4-May-15 |
| 11251 | 2 | 3 | | | III-1 | Krtap1-5 | 83895 | 4-May-15 |
| 11286 | 2 | 3 | | | III-1 | Krtap6-2 | 337967 | 12-May-15 |
| 11287 | 2 | 3 | | | III-1 | Krtap6-5 | | |
| 11294 | 2 | 3 | | | III-1 | Krtcap3 | 200634 | 4-May-15 |
| 11297 | 2 | 3 | | | III-1 | Ksr2 | 283455 | 7-Jun-15 |
| 11303 | 2 | 3 | | | III-1 | L1cam | 3897 | 23-May-15 |
| 11304 | 2 | 3 | | | III-1 | L1td1 | 54596 | 4-May-15 |
| 11313 | 2 | 3 | | | III-1 | Lace1 | 246269 | 4-May-15 |
| 11315 | 2 | 3 | | | III-1 | Lactb2 | 51110 | 4-May-15 |
| 11320 | 2 | 3 | | | III-1 | Lair1 | 3903 | 3-May-15 |
| 11321 | 2 | 3 | | | III-1 | Lalba | 3906 | 4-May-15 |
| 11326 | 2 | 3 | | | III-1 | Lama5 | 3911 | 12-May-15 |
| 11328 | 2 | 3 | | | III-1 | Lamb2 | 3913 | 7-Jun-15 |
| 11330 | 2 | 3 | | | III-1 | Lamc1 | 3915 | 12-May-15 |
| 11332 | 2 | 3 | | | III-1 | Lamc3 | 10319 | 4-May-15 |
| 11339 | 2 | 3 | | | III-1 | Lamtor3 | 8649 | 4-May-15 |
| 11340 | 2 | 3 | | | III-1 | Lamtor4 | 389541 | 4-May-15 |
| 11341 | 2 | 3 | | | III-1 | Lamtor5 | 10542 | 17-May-15 |
| 11343 | 2 | 3 | | | III-1 | Lancl2 | 55915 | 12-May-15 |
| 11345 | 2 | 3 | | | III-1 | Lao1 | | |
| 11347 | 2 | 3 | | | III-1 | Laptm4a | 9741 | 4-May-15 |
| 11351 | 2 | 3 | | | III-1 | Larp1 | 23367 | 4-May-15 |
| 11353 | 2 | 3 | | | III-1 | Larp4 | 113251 | 3-May-15 |
| 11356 | 2 | 3 | | | III-1 | Larp7 | 51574 | 17-May-15 |
| 11360 | 2 | 3 | | | III-1 | Lasp1 | 3927 | 7-Jun-15 |
| 11367 | 2 | 3 | | | III-1 | Lbh | 81606 | 7-Jun-15 |
| 11371 | 2 | 3 | | | III-1 | Lbx2 | 85474 | 12-May-15 |
| 11372 | 2 | 3 | | | III-1 | Lca5 | 167691 | 23-May-15 |
| 11388 | 2 | 3 | | | III-1 | Lce1m | | |
| 11399 | 2 | 3 | | | III-1 | Lcmt2 | 9836 | 4-May-15 |

Fig.22 - 72

| | | | | | | | | |
|---|---|---|---|---|---|---|---|---|
| 11401 | 2 | 3 | | | III-1 | Lcn11 | | |
| 11420 | 2 | 3 | | | III-1 | Ldhal6b | 92483 | 12-May-15 |
| 11422 | 2 | 3 | | | III-1 | Ldhc | 3948 | 21-May-15 |
| 11425 | 2 | 3 | | | III-1 | Ldlrad1 | 388633 | 4-May-15 |
| 11428 | 2 | 3 | | | III-1 | Ldlrad4 | 753 | 4-May-15 |
| 11437 | 2 | 3 | | | III-1 | Lefty2 | 7044 | 4-May-15 |
| 11440 | 2 | 3 | | | III-1 | Lemd1 | 93273 | 4-May-15 |
| 11444 | 2 | 3 | | | III-1 | Leng1 | 79165 | 4-May-15 |
| 11447 | 2 | 3 | | | III-1 | Leo1 | 123169 | 4-May-15 |
| 11450 | 2 | 3 | | | III-1 | Lepre1 | 64175 | 4-May-15 |
| 11451 | 2 | 3 | | | III-1 | Leprel1 | 55214 | 4-May-15 |
| 11454 | 2 | 3 | | | III-1 | Leprot | 54741 | 4-May-15 |
| 11458 | 2 | 3 | | | III-1 | Letmd1 | 25875 | 12-May-15 |
| 11468 | 2 | 3 | | | III-1 | Lgals8 | 3964 | 17-May-15 |
| 11471 | 2 | 3 | | | III-1 | Lgi1 | 9211 | 1-Jun-15 |
| 11474 | 2 | 3 | | | III-1 | Lgi4 | 163175 | 4-May-15 |
| 11476 | 2 | 3 | | | III-1 | Lgr4 | 55366 | 12-May-15 |
| 11477 | 2 | 3 | | | III-1 | Lgr5 | 8549 | 17-May-15 |
| 11481 | 2 | 3 | | | III-1 | Lhcgr | 3973 | 12-May-15 |
| 11489 | 2 | 3 | | | III-1 | Lhx1 | 3975 | 4-May-15 |
| 11491 | 2 | 3 | | | III-1 | Lhx2 | 9355 | 4-May-15 |
| 11492 | 2 | 3 | | | III-1 | Lhx3 | 8022 | 12-May-15 |
| 11493 | 2 | 3 | | | III-1 | Lhx4 | 89884 | 12-May-15 |
| 11496 | 2 | 3 | | | III-1 | Lhx8 | 431707 | 4-May-15 |
| 11503 | 2 | 3 | | | III-1 | Lig4 | 3981 | 4-May-15 |
| 11507 | 2 | 3 | | | III-1 | Lim2 | 3982 | 4-May-15 |
| 11509 | 2 | 3 | | | III-1 | Limch1 | 22998 | 12-May-15 |
| 11510 | 2 | 3 | | | III-1 | Limd1 | 8994 | 24-May-15 |
| 11517 | 2 | 3 | | | III-1 | Lin28a | 79727 | 4-May-15 |
| 11520 | 2 | 3 | | | III-1 | Lin52 | 91750 | 4-May-15 |
| 11526 | 2 | 3 | | | III-1 | Lincrna-cox2 | | |
| 11528 | 2 | 3 | | | III-1 | Lingo2 | 158038 | 4-May-15 |
| 11530 | 2 | 3 | | | III-1 | Lingo4 | 339398 | 4-May-15 |
| 11532 | 2 | 3 | | | III-1 | Lipa | 3988 | 7-Jun-15 |
| 11533 | 2 | 3 | | | III-1 | Lipc | 3990 | 24-May-15 |
| 11537 | 2 | 3 | | | III-1 | Liph | 200879 | 13-Jun-15 |
| 11539 | 2 | 3 | | | III-1 | Lipk | 643414 | 4-May-15 |
| 11542 | 2 | 3 | | | III-1 | Lipo1 | | |
| 11544 | 2 | 3 | | | III-1 | Lipt2 | 387787 | 4-May-15 |
| 11545 | 2 | 3 | | | III-1 | Litaf | 9516 | 23-May-15 |
| 11546 | 2 | 3 | | | III-1 | Lix1 | 167410 | 21-May-15 |
| 11547 | 2 | 3 | | | III-1 | Lix1l | 128077 | 12-May-15 |
| 11548 | 2 | 3 | | | III-1 | Lkaaear1 | 198437 | 4-May-15 |
| 11551 | 2 | 3 | | | III-1 | Llph | 84298 | 4-May-15 |
| 11555 | 2 | 3 | | | III-1 | Lman2l | 81562 | 17-May-15 |
| 11557 | 2 | 3 | | | III-1 | Lmbr1l | 55716 | 4-May-15 |
| 11561 | 2 | 3 | | | III-1 | Lmf1 | 64788 | 14-May-15 |
| 11565 | 2 | 3 | | | III-1 | Lmnb1 | 4001 | 4-May-15 |
| 11568 | 2 | 3 | | | III-1 | Lmo2 | 4005 | 21-May-15 |
| 11569 | 2 | 3 | | | III-1 | Lmo3 | 55885 | 12-May-15 |
| 11571 | 2 | 3 | | | III-1 | Lmo7 | 4008 | 12-May-15 |
| 11576 | 2 | 3 | | | III-1 | Lmtk3 | 114783 | 4-May-15 |
| 11577 | 2 | 3 | | | III-1 | Lmx1a | 4009 | 3-May-15 |
| 11582 | 2 | 3 | | | III-1 | Lnx2 | 222484 | 4-May-15 |
| 11592 | 2 | 3 | | | III-1 | LOC100504608 | | |
| 11596 | 2 | 3 | | | III-1 | LOC100861978 | | |
| 11600 | 2 | 3 | | | III-1 | LOC101055863 | | |
| 11602 | 2 | 3 | | | III-1 | LOC101056136 | | |
| 11606 | 2 | 3 | | | III-1 | LOC101669761 | | |
| 11607 | 2 | 3 | | | III-1 | LOC102308570 | | |
| 11610 | 2 | 3 | | | III-1 | LOC102632430 | | |
| 11613 | 2 | 3 | | | III-1 | LOC102634101 | | |
| 11614 | 2 | 3 | | | III-1 | LOC102634401 | | |
| 11620 | 2 | 3 | | | III-1 | LOC171588 | | |
| 11623 | 2 | 3 | | | III-1 | Loh12cr1 | 118426 | 4-May-15 |
| 11625 | 2 | 3 | | | III-1 | Lonp2 | 83752 | 23-May-15 |
| 11637 | 2 | 3 | | | III-1 | Lpar2 | 9170 | 12-May-15 |
| 11641 | 2 | 3 | | | III-1 | Lpar6 | 10161 | 17-May-15 |
| 11644 | 2 | 3 | | | III-1 | Lpcat2b | | |
| 11647 | 2 | 3 | | | III-1 | Lpgat1 | 9926 | 4-May-15 |
| 11650 | 2 | 3 | | | III-1 | Lphn3 | 23284 | 4-May-15 |
| 11657 | 2 | 3 | | | III-1 | Lpxn | 9404 | 12-May-15 |
| 11660 | 2 | 3 | | | III-1 | Lrch1 | 23143 | 4-May-15 |
| 11665 | 2 | 3 | | | III-1 | Lrfn1 | 57622 | 12-May-15 |
| 11666 | 2 | 3 | | | III-1 | Lrfn2 | 57497 | 4-May-15 |
| 11669 | 2 | 3 | | | III-1 | Lrfn5 | 145581 | 4-May-15 |
| 11671 | 2 | 3 | | | III-1 | Lrguk | 136332 | 4-May-15 |
| 11676 | 2 | 3 | | | III-1 | Lrit1 | 26103 | 12-May-15 |
| 11678 | 2 | 3 | | | III-1 | Lrit3 | 345193 | 7-Jun-15 |
| 11681 | 2 | 3 | | | III-1 | Lrp10 | 26020 | 4-May-15 |
| 11687 | 2 | 3 | | | III-1 | Lrp3 | 4037 | 4-May-15 |
| 11689 | 2 | 3 | | | III-1 | Lrp5 | 4041 | 23-May-15 |
| 11690 | 2 | 3 | | | III-1 | Lrp6 | 4040 | 4-May-15 |
| 11694 | 2 | 3 | | | III-1 | Lrr1 | 122769 | 4-May-15 |
| 11703 | 2 | 3 | | | III-1 | Lrrc17 | 10234 | 4-May-15 |
| 11705 | 2 | 3 | | | III-1 | Lrrc19 | 64922 | 4-May-15 |
| 11709 | 2 | 3 | | | III-1 | Lrrc24 | 441381 | 4-May-15 |
| 11710 | 2 | 3 | | | III-1 | Lrrc25 | 126364 | 4-May-15 |
| 11715 | 2 | 3 | | | III-1 | Lrrc3 | 81543 | 4-May-15 |
| 11722 | 2 | 3 | | | III-1 | Lrrc3b | 116135 | 14-May-15 |
| 11730 | 2 | 3 | | | III-1 | Lrrc47 | 57470 | 4-May-15 |
| 11732 | 2 | 3 | | | III-1 | Lrrc49 | 54839 | 4-May-15 |
| 11733 | 2 | 3 | | | III-1 | Lrrc4b | 94030 | 4-May-15 |
| 11734 | 2 | 3 | | | III-1 | Lrrc4c | 57689 | 2-Jun-15 |
| 11739 | 2 | 3 | | | III-1 | Lrrc57 | 255252 | 4-May-15 |
| 11741 | 2 | 3 | | | III-1 | Lrrc59 | 55379 | 4-May-15 |
| 11743 | 2 | 3 | | | III-1 | Lrrc61 | 65999 | 4-May-15 |
| 11749 | 2 | 3 | | | III-1 | Lrrc72 | 100506049 | 4-May-15 |
| 11751 | 2 | 3 | | | III-1 | Lrrc74 | 145497 | 4-May-15 |
| 11757 | 2 | 3 | | | III-1 | Lrrc8d | 55144 | 4-May-15 |
| 11759 | 2 | 3 | | | III-1 | Lrrc9 | 341883 | 4-May-15 |
| 11764 | 2 | 3 | | | III-1 | Lrriq1 | 84125 | 4-May-15 |
| 11768 | 2 | 3 | | | III-1 | Lrrk2 | 120892 | 23-May-15 |
| 11774 | 2 | 3 | | | III-1 | Lrrtm1 | 347730 | 17-May-15 |
| 11777 | 2 | 3 | | | III-1 | Lrrtm4 | 80059 | 4-May-15 |
| 11782 | 2 | 3 | | | III-1 | Lsamp | 4045 | 21-May-15 |
| 11784 | 2 | 3 | | | III-1 | Lsm1 | 27257 | 4-May-15 |
| 11786 | 2 | 3 | | | III-1 | Lsm11 | 134353 | 4-May-15 |
| 11788 | 2 | 3 | | | III-1 | Lsm14a | 26065 | 4-May-15 |
| 11791 | 2 | 3 | | | III-1 | Lsm3 | 27258 | 4-May-15 |
| 11796 | 2 | 3 | | | III-1 | Lsm8 | 51691 | 4-May-15 |
| 11808 | 2 | 3 | | | III-1 | Ltbp2 | 4053 | 7-Jun-15 |
| 11811 | 2 | 3 | | | III-1 | Ltbr | 4055 | 12-May-15 |
| 11817 | 2 | 3 | | | III-1 | Luc7l | 55692 | 21-May-15 |
| 11822 | 2 | 3 | | | III-1 | Lurap1l | 286343 | 4-May-15 |
| 11823 | 2 | 3 | | | III-1 | Luzp1 | 7798 | 4-May-15 |
| 11824 | 2 | 3 | | | III-1 | Luzp2 | 338645 | 4-May-15 |
| 11827 | 2 | 3 | | | III-1 | Ly6a | | |
| 11848 | 2 | 3 | | | III-1 | Lyg2 | 254773 | 4-May-15 |
| 11851 | 2 | 3 | | | III-1 | Lynx1 | 66004 | 4-May-15 |
| 11853 | 2 | 3 | | | III-1 | Lypd2 | 137797 | 4-May-15 |
| 11855 | 2 | 3 | | | III-1 | Lypd4 | 147719 | 12-May-15 |
| 11858 | 2 | 3 | | | III-1 | Lypd6b | 130576 | 4-May-15 |
| 11860 | 2 | 3 | | | III-1 | Lypla1 | 10434 | 4-May-15 |
| 11863 | 2 | 3 | | | III-1 | Lyrm1 | 57149 | 12-May-15 |
| 11866 | 2 | 3 | | | III-1 | Lyrm5 | 144363 | 4-May-15 |
| 11870 | 2 | 3 | | | III-1 | Lysmd1 | 388695 | 4-May-15 |
| 11872 | 2 | 3 | | | III-1 | Lysmd3 | 116068 | 4-May-15 |
| 11873 | 2 | 3 | | | III-1 | Lysmd4 | 145748 | 4-May-15 |
| 11887 | 2 | 3 | | | III-1 | Lzts3 | 9762 | 4-May-15 |
| 11888 | 2 | 3 | | | III-1 | M1ap | 130951 | 4-May-15 |
| 11899 | 2 | 3 | | | III-1 | Mad2l1 | 4085 | 12-May-15 |
| 11903 | 2 | 3 | | | III-1 | Madd | 8567 | 7-Jun-15 |
| 11904 | 2 | 3 | | | III-1 | Maea | 10296 | 12-May-15 |
| 11907 | 2 | 3 | | | III-1 | Maf1 | 84232 | 4-May-15 |
| 11911 | 2 | 3 | | | III-1 | Mafg | 4097 | 2-Jun-15 |
| 11912 | 2 | 3 | | | III-1 | Mafk | 7975 | 4-May-15 |
| 11913 | 2 | 3 | | | III-1 | Mag | 4099 | 4-May-15 |
| 11927 | 2 | 3 | | | III-1 | Mageb3 | 4114 | 4-May-15 |
| 11931 | 2 | 3 | | | III-1 | Maged2 | 10916 | 4-May-15 |
| 11935 | 2 | 3 | | | III-1 | Magel2 | 54551 | 23-May-15 |
| 11937 | 2 | 3 | | | III-1 | Magi2 | 9863 | 10-May-15 |
| 11947 | 2 | 3 | | | III-1 | Malat1 | 378938 | 21-May-15 |
| 11950 | 2 | 3 | | | III-1 | Malt1 | 10892 | 4-May-15 |
| 11952 | 2 | 3 | | | III-1 | Mamdc4 | 158056 | 4-May-15 |
| 11956 | 2 | 3 | | | III-1 | Mamld1 | 10046 | 23-May-15 |
| 11959 | 2 | 3 | | | III-1 | Man1a2 | 10905 | 12-May-15 |
| 11962 | 2 | 3 | | | III-1 | Man2a1 | 4124 | 4-May-15 |
| 11963 | 2 | 3 | | | III-1 | Man2a2 | 4122 | 12-May-15 |
| 11966 | 2 | 3 | | | III-1 | Man2c1 | 4123 | 4-May-15 |
| 11968 | 2 | 3 | | | III-1 | Manba | 4126 | 4-May-15 |
| 11973 | 2 | 3 | | | III-1 | Manr | | |
| 11975 | 2 | 3 | | | III-1 | Mansc4 | 100287284 | 4-May-15 |
| 11978 | 2 | 3 | | | III-1 | Map10 | 54627 | 4-May-15 |
| 11982 | 2 | 3 | | | III-1 | Map1lc3b | 81631 | 1-Jun-15 |
| 11985 | 2 | 3 | | | III-1 | Map2k1 | 5604 | 28-May-15 |
| 11988 | 2 | 3 | | | III-1 | Map2k3os | | |
| 11989 | 2 | 3 | | | III-1 | Map2k4 | 6416 | 12-May-15 |
| 11999 | 2 | 3 | | | III-1 | Map3k15 | 389840 | 21-May-15 |
| 12006 | 2 | 3 | | | III-1 | Map3k7 | 6885 | 12-May-15 |
| 12009 | 2 | 3 | | | III-1 | Map3k9 | 4293 | 4-May-15 |
| 12010 | 2 | 3 | | | III-1 | Map4 | 4134 | 4-May-15 |
| 12015 | 2 | 3 | | | III-1 | Map4k5 | 11183 | 4-May-15 |
| 12019 | 2 | 3 | | | III-1 | Map7d1 | 55700 | 4-May-15 |
| 12021 | 2 | 3 | | | III-1 | Map9 | 79884 | 4-May-15 |
| 12027 | 2 | 3 | | | III-1 | Mapk14 | 1432 | 31-May-15 |
| 12029 | 2 | 3 | | | III-1 | Mapk1ip1 | | |
| 12030 | 2 | 3 | | | III-1 | Mapk1ip1l | 93487 | 4-May-15 |
| 12033 | 2 | 3 | | | III-1 | Mapk6 | 5597 | 4-May-15 |
| 12034 | 2 | 3 | | | III-1 | Mapk7 | 5598 | 4-May-15 |
| 12043 | 2 | 3 | | | III-1 | Mapkapk5 | 8550 | 4-May-15 |
| 12045 | 2 | 3 | | | III-1 | Mapre1 | 22919 | 31-May-15 |
| 12047 | 2 | 3 | | | III-1 | Mapre3 | 22924 | 12-May-15 |
| 12050 | 2 | 3 | | | III-1 | Marc2 | 54996 | 4-May-15 |
| 12056 | 2 | 3 | | | III-1 | March4 | 57574 | 4-May-15 |
| 12060 | 2 | 3 | | | III-1 | March8 | 220972 | 4-May-15 |
| 12062 | 2 | 3 | | | III-1 | Marcks | 4082 | 10-May-15 |
| 12066 | 2 | 3 | | | III-1 | Marf1 | 9665 | 4-May-15 |
| 12068 | 2 | 3 | | | III-1 | Mark2 | 2011 | 21-May-15 |
| 12071 | 2 | 3 | | | III-1 | Mars | 4141 | 7-Jun-15 |
| 12076 | 2 | 3 | | | III-1 | Mas1 | 4142 | 4-May-15 |
| 12080 | 2 | 3 | | | III-1 | Mast2 | 23139 | 4-May-15 |
| 12082 | 2 | 3 | | | III-1 | Mast4 | 375449 | 12-May-15 |
| 12083 | 2 | 3 | | | III-1 | Mastl | 84930 | 4-May-15 |
| 12086 | 2 | 3 | | | III-1 | Mat2b | 27430 | 12-May-15 |
| 12100 | 2 | 3 | | | III-1 | Mbd1 | 4152 | 7-Jun-15 |
| 12107 | 2 | 3 | | | III-1 | Mbd6 | 114785 | 31-May-15 |
| 12109 | 2 | 3 | | | III-1 | Mbl1 | | |
| 12111 | 2 | 3 | | | III-1 | Mblac1 | 255374 | 4-May-15 |
| 12112 | 2 | 3 | | | III-1 | Mblac2 | 153364 | 4-May-15 |
| 12113 | 2 | 3 | | | III-1 | Mbnl1 | 4154 | 4-May-15 |
| 12118 | 2 | 3 | | | III-1 | Mboat4 | 619373 | 12-May-15 |
| 12119 | 2 | 3 | | | III-1 | Mboat7 | 79143 | 4-May-15 |

Fig.22 - 73

| | | | | | | | | |
|---|---|---|---|---|---|---|---|---|
| 12121 | 2 | 3 | | | III-1 | Mbtd1 | 54799 | 12-May-15 |
| 12124 | 2 | 3 | | | III-1 | Mc1r | 4157 | 7-Jun-15 |
| 12126 | 2 | 3 | | | III-1 | Mc3r | 4159 | 24-May-15 |
| 12129 | 2 | 3 | | | III-1 | Mcam | 4162 | 4-May-15 |
| 12132 | 2 | 3 | | | III-1 | Mccc1 | 56922 | 23-May-15 |
| 12133 | 2 | 3 | | | III-1 | Mccc1os | | |
| 12139 | 2 | 3 | | | III-1 | Mcfd2 | 90411 | 1-Jun-15 |
| 12140 | 2 | 3 | | | III-1 | Mchr1 | 2847 | 12-May-15 |
| 12141 | 2 | 3 | | | III-1 | Mcidas | 345643 | 4-May-15 |
| 12146 | 2 | 3 | | | III-1 | Mcm3ap | 8888 | 12-May-15 |
| 12148 | 2 | 3 | | | III-1 | Mcm5 | 4174 | 4-May-15 |
| 12150 | 2 | 3 | | | III-1 | Mcm7 | 4176 | 10-May-15 |
| 12151 | 2 | 3 | | | III-1 | Mcm8 | 84515 | 4-May-15 |
| 12152 | 2 | 3 | | | III-1 | Mcm9 | 254394 | 4-May-15 |
| 12158 | 2 | 3 | | | III-1 | Mcph1 | 79648 | 23-May-15 |
| 12164 | 2 | 3 | | | III-1 | Mcpt-ps1 | | |
| 12166 | 2 | 3 | | | III-1 | Mctp1 | 79772 | 12-May-15 |
| 12167 | 2 | 3 | | | III-1 | Mctp2 | 55784 | 4-May-15 |
| 12168 | 2 | 3 | | | III-1 | Mcts1 | 28985 | 4-May-15 |
| 12170 | 2 | 3 | | | III-1 | Mcu | 90550 | 10-May-15 |
| 12174 | 2 | 3 | | | III-1 | Mdfic | 29969 | 4-May-15 |
| 12178 | 2 | 3 | | | III-1 | Mdh1b | 130752 | 4-May-15 |
| 12179 | 2 | 3 | | | III-1 | Mdh2 | 4191 | 3-May-15 |
| 12182 | 2 | 3 | | | III-1 | Mdm2 | 4193 | 31-May-15 |
| 12187 | 2 | 3 | | | III-1 | Me2 | 4200 | 7-Jun-15 |
| 12189 | 2 | 3 | | | III-1 | Mea1 | 4201 | 4-May-15 |
| 12192 | 2 | 3 | | | III-1 | Mecp2 | 4204 | 23-May-15 |
| 12193 | 2 | 3 | | | III-1 | Mecr | 51102 | 4-May-15 |
| 12194 | 2 | 3 | | | III-1 | Med1 | 5469 | 7-Jun-15 |
| 12196 | 2 | 3 | | | III-1 | Med11 | 400569 | 28-May-15 |
| 12198 | 2 | 3 | | | III-1 | Med12l | 116931 | 28-May-15 |
| 12201 | 2 | 3 | | | III-1 | Med14 | 9282 | 21-May-15 |
| 12206 | 2 | 3 | | | III-1 | Med19 | 219541 | 28-May-15 |
| 12214 | 2 | 3 | | | III-1 | Med27 | 9442 | 4-May-15 |
| 12218 | 2 | 3 | | | III-1 | Med31 | 51003 | 2-Jun-15 |
| 12219 | 2 | 3 | | | III-1 | Med4 | 29079 | 31-May-15 |
| 12221 | 2 | 3 | | | III-1 | Med7 | 9443 | 4-May-15 |
| 12222 | 2 | 3 | | | III-1 | Med8 | 112950 | 12-May-15 |
| 12229 | 2 | 3 | | | III-1 | Mef2d | 4209 | 28-May-15 |
| 12230 | 2 | 3 | | | III-1 | Mefv | 4210 | 23-May-15 |
| 12231 | 2 | 3 | | | III-1 | Meg3 | 55384 | 7-Jun-15 |
| 12233 | 2 | 3 | | | III-1 | Megf11 | 84465 | 4-May-15 |
| 12241 | 2 | 3 | | | III-1 | Meis1 | 4211 | 4-May-15 |
| 12243 | 2 | 3 | | | III-1 | Meis3 | 56917 | 7-Jun-15 |
| 12246 | 2 | 3 | | | III-1 | Men1 | 4221 | 23-May-15 |
| 12254 | 2 | 3 | | | III-1 | Mesdc1 | 59274 | 4-May-15 |
| 12258 | 2 | 3 | | | III-1 | Mest | 4232 | 12-May-15 |
| 12260 | 2 | 3 | | | III-1 | Metap1 | 23173 | 4-May-15 |
| 12265 | 2 | 3 | | | III-1 | Mettl1 | 4234 | 4-May-15 |
| 12266 | 2 | 3 | | | III-1 | Mettl10 | 399818 | 4-May-15 |
| 12267 | 2 | 3 | | | III-1 | Mettl11b | 149281 | 4-May-15 |
| 12270 | 2 | 3 | | | III-1 | Mettl15 | 196074 | 4-May-15 |
| 12271 | 2 | 3 | | | III-1 | Mettl16 | 79066 | 12-May-15 |
| 12273 | 2 | 3 | | | III-1 | Mettl18 | 92342 | 4-May-15 |
| 12274 | 2 | 3 | | | III-1 | Mettl2 | 339175 | 7-Jun-15 |
| 12276 | 2 | 3 | | | III-1 | Mettl21a | 151194 | 23-May-15 |
| 12280 | 2 | 3 | | | III-1 | Mettl23 | 124512 | 12-May-15 |
| 12283 | 2 | 3 | | | III-1 | Mettl3 | 56339 | 29-May-15 |
| 12285 | 2 | 3 | | | III-1 | Mettl5 | 29081 | 4-May-15 |
| 12286 | 2 | 3 | | | III-1 | Mettl6 | 131965 | 12-May-15 |
| 12288 | 2 | 3 | | | III-1 | Mettl7a2 | | |
| 12289 | 2 | 3 | | | III-1 | Mettl7a2Higd1c | | |
| 12291 | 2 | 3 | | | III-1 | Mettl7b | 196410 | 4-May-15 |
| 12298 | 2 | 3 | | | III-1 | Mfap1a | | |
| 12309 | 2 | 3 | | | III-1 | Mfn1 | 55669 | 24-May-15 |
| 12310 | 2 | 3 | | | III-1 | Mfn2 | 9927 | 31-May-15 |
| 12311 | 2 | 3 | | | III-1 | Mfng | 4242 | 4-May-15 |
| 12313 | 2 | 3 | | | III-1 | Mfsd1 | 64747 | 4-May-15 |
| 12315 | 2 | 3 | | | III-1 | Mfsd11 | 79157 | 4-May-15 |
| 12318 | 2 | 3 | | | III-1 | Mfsd2b | 388931 | 4-May-15 |
| 12320 | 2 | 3 | | | III-1 | Mfsd4 | 148808 | 4-May-15 |
| 12324 | 2 | 3 | | | III-1 | Mfsd7a | | |
| 12325 | 2 | 3 | | | III-1 | Mfsd7b | 28982 | 4-May-15 |
| 12327 | 2 | 3 | | | III-1 | Mfsd8 | 256471 | 23-May-15 |
| 12329 | 2 | 3 | | | III-1 | Mga | 23269 | 7-Jun-15 |
| 12332 | 2 | 3 | | | III-1 | Mgat1 | 4245 | 7-Jun-15 |
| 12334 | 2 | 3 | | | III-1 | Mgat3 | 4248 | 7-Jun-15 |
| 12336 | 2 | 3 | | | III-1 | Mgat4b | 11282 | 4-May-15 |
| 12337 | 2 | 3 | | | III-1 | Mgat4c | 25834 | 12-May-15 |
| 12340 | 2 | 3 | | | III-1 | Mgea5 | 10724 | 7-Jun-15 |
| 12342 | 2 | 3 | | | III-1 | Mgll | 11343 | 17-May-15 |
| 12343 | 2 | 3 | | | III-1 | Mgme1 | 92667 | 4-May-15 |
| 12346 | 2 | 3 | | | III-1 | Mgrn1 | 23295 | 4-May-15 |
| 12348 | 2 | 3 | | | III-1 | Mgst2 | 4258 | 4-May-15 |
| 12352 | 2 | 3 | | | III-1 | Mia3 | 375056 | 4-May-15 |
| 12353 | 2 | 3 | | | III-1 | Miat | 440823 | 12-May-15 |
| 12358 | 2 | 3 | | | III-1 | Mical3 | 57553 | 23-May-15 |
| 12360 | 2 | 3 | | | III-1 | Micall1 | 85377 | 4-May-15 |
| 12361 | 2 | 3 | | | III-1 | Micall2 | 79778 | 4-May-15 |
| 12367 | 2 | 3 | | | III-1 | Mid2 | 11043 | 4-May-15 |
| 12368 | 2 | 3 | | | III-1 | Midn | 90007 | 4-May-15 |
| 12371 | 2 | 3 | | | III-1 | Mien1 | 84299 | 4-May-15 |
| 12372 | 2 | 3 | | | III-1 | Mier1 | 57708 | 12-May-15 |
| 12376 | 2 | 3 | | | III-1 | Mif4gd | 57409 | 3-May-15 |
| 12378 | 2 | 3 | | | III-1 | Mill1 | | |
| 12381 | 2 | 3 | | | III-1 | Mina | 84864 | 12-May-15 |
| 12382 | 2 | 3 | | | III-1 | Mink1 | 50488 | 4-May-15 |
| 12385 | 2 | 3 | | | III-1 | Mios | 54468 | 29-May-15 |
| 12389 | 2 | 3 | | | III-1 | Mipol1 | 145282 | 12-May-15 |
| 12390 | 2 | 3 | | | III-1 | Mir100 | 406892 | 21-May-15 |
| 12444 | 2 | 3 | | | III-1 | Mir133b | 442890 | 21-May-15 |
| 12461 | 2 | 3 | | | III-1 | Mir144 | 406936 | 21-May-15 |
| 12482 | 2 | 3 | | | III-1 | Mir18 | 406953 | 21-May-15 |
| 12622 | 2 | 3 | | | III-1 | Mir23a | 407010 | 24-May-15 |
| 13012 | 2 | 3 | | | III-1 | Mir6386 | | |
| 13023 | 2 | 3 | | | III-1 | Mir6397 | | |
| 13045 | 2 | 3 | | | III-1 | Mir6419 | | |
| 13095 | 2 | 3 | | | III-1 | Mir684-1 | | |
| 13181 | 2 | 3 | | | III-1 | Mir6970 | | |
| 13206 | 2 | 3 | | | III-1 | Mir6993 | | |
| 13439 | 2 | 3 | | | III-1 | Mir8109 | | |
| 13449 | 2 | 3 | | | III-1 | Mir8120 | | |
| 13481 | 2 | 3 | | | III-1 | Mirlet7c-1 | | |
| 13497 | 2 | 3 | | | III-1 | Mixl1 | 83881 | 28-May-15 |
| 13504 | 2 | 3 | | | III-1 | Mknk1 | 8569 | 4-May-15 |
| 13506 | 2 | 3 | | | III-1 | Mkrn1 | 23608 | 12-May-15 |
| 13507 | 2 | 3 | | | III-1 | Mkrn2 | 23609 | 23-May-15 |
| 13510 | 2 | 3 | | | III-1 | Mkx | 283078 | 4-May-15 |
| 13511 | 2 | 3 | | | III-1 | Mlana | 2315 | 4-May-15 |
| 13519 | 2 | 3 | | | III-1 | Mlkl | 197259 | 4-May-15 |
| 13520 | 2 | 3 | | | III-1 | Mllt1 | 4298 | 21-May-15 |
| 13524 | 2 | 3 | | | III-1 | Mllt4 | 4301 | 21-May-15 |
| 13525 | 2 | 3 | | | III-1 | Mllt6 | 4302 | 4-May-15 |
| 13527 | 2 | 3 | | | III-1 | Mlst8 | 64223 | 4-May-15 |
| 13532 | 2 | 3 | | | III-1 | Mmaa | 166785 | 7-Jun-15 |
| 13534 | 2 | 3 | | | III-1 | Mmachc | 25974 | 23-May-15 |
| 13539 | 2 | 3 | | | III-1 | Mmel1 | 79258 | 4-May-15 |
| 13542 | 2 | 3 | | | III-1 | Mmp10 | 4319 | 12-May-15 |
| 13547 | 2 | 3 | | | III-1 | Mmp15 | 4324 | 31-May-15 |
| 13551 | 2 | 3 | | | III-1 | Mmp1a | | |
| 13565 | 2 | 3 | | | III-1 | Mmrn1 | 22915 | 4-May-15 |
| 13566 | 2 | 3 | | | III-1 | Mmrn2 | 79812 | 4-May-15 |
| 13567 | 2 | 3 | | | III-1 | Mms19 | 64210 | 4-May-15 |
| 13570 | 2 | 3 | | | III-1 | Mnat1 | 4331 | 1-Jun-15 |
| 13571 | 2 | 3 | | | III-1 | Mnd1 | 84057 | 4-May-15 |
| 13572 | 2 | 3 | | | III-1 | Mnd1-ps | | |
| 13574 | 2 | 3 | | | III-1 | Mndal | | |
| 13580 | 2 | 3 | | | III-1 | Mob1b | 92597 | 4-May-15 |
| 13586 | 2 | 3 | | | III-1 | Mobp | 4336 | 4-May-15 |
| 13588 | 2 | 3 | | | III-1 | Mocs1 | 4337 | 12-May-15 |
| 13589 | 2 | 3 | | | III-1 | Mocs2 | 4338 | 4-May-15 |
| 13590 | 2 | 3 | | | III-1 | Mocs3 | 27304 | 24-May-15 |
| 13595 | 2 | 3 | | | III-1 | Mok | 5891 | 4-May-15 |
| 13601 | 2 | 3 | | | III-1 | Morc2b | | |
| 13604 | 2 | 3 | | | III-1 | Morf4l1 | 10933 | 4-May-15 |
| 13606 | 2 | 3 | | | III-1 | Morn1 | 79906 | 12-May-15 |
| 13607 | 2 | 3 | | | III-1 | Morn2 | 729967 | 4-May-15 |
| 13609 | 2 | 3 | | | III-1 | Morn4 | 118812 | 4-May-15 |
| 13610 | 2 | 3 | | | III-1 | Morn5 | 254956 | 4-May-15 |
| 13613 | 2 | 3 | | | III-1 | Mospd2 | 158747 | 4-May-15 |
| 13614 | 2 | 3 | | | III-1 | Mospd3 | 64598 | 4-May-15 |
| 13617 | 2 | 3 | | | III-1 | Mov10l1 | 54456 | 4-May-15 |
| 13621 | 2 | 3 | | | III-1 | Mpc2 | 25874 | 12-May-15 |
| 13625 | 2 | 3 | | | III-1 | Mpg | 4350 | 12-May-15 |
| 13626 | 2 | 3 | | | III-1 | Mphosph10 | 10199 | 4-May-15 |
| 13627 | 2 | 3 | | | III-1 | Mphosph6 | 10200 | 4-May-15 |
| 13628 | 2 | 3 | | | III-1 | Mphosph8 | 54737 | 17-May-15 |
| 13629 | 2 | 3 | | | III-1 | Mphosph9 | 10198 | 4-May-15 |
| 13631 | 2 | 3 | | | III-1 | Mpl | 4352 | 3-May-15 |
| 13634 | 2 | 3 | | | III-1 | Mpo | 4353 | 23-May-15 |
| 13638 | 2 | 3 | | | III-1 | Mpp4 | 58538 | 7-Jun-15 |
| 13641 | 2 | 3 | | | III-1 | Mpp7 | 143098 | 7-Jun-15 |
| 13659 | 2 | 3 | | | III-1 | Mras | 22808 | 4-May-15 |
| 13661 | 2 | 3 | | | III-1 | Mrc2 | 9902 | 4-May-15 |
| 13666 | 2 | 3 | | | III-1 | Mrgpra1 | | |
| 13674 | 2 | 3 | | | III-1 | Mrgprb2 | | |
| 13676 | 2 | 3 | | | III-1 | Mrgprb4 | | |
| 13681 | 2 | 3 | | | III-1 | Mrgprf | 116535 | 4-May-15 |
| 13683 | 2 | 3 | | | III-1 | Mrgprh | | |
| 13688 | 2 | 3 | | | III-1 | Mro | 83876 | 4-May-15 |
| 13690 | 2 | 3 | | | III-1 | Mroh2a | 339766 | 3-May-15 |
| 13701 | 2 | 3 | | | III-1 | Mrpl12 | 6182 | 4-May-15 |
| 13703 | 2 | 3 | | | III-1 | Mrpl14 | 64928 | 7-Jun-15 |
| 13704 | 2 | 3 | | | III-1 | Mrpl15 | 29088 | 7-Jun-15 |
| 13710 | 2 | 3 | | | III-1 | Mrpl20 | 55052 | 4-May-15 |
| 13711 | 2 | 3 | | | III-1 | Mrpl21 | 219927 | 4-May-15 |
| 13713 | 2 | 3 | | | III-1 | Mrpl23 | 6150 | 4-May-15 |
| 13714 | 2 | 3 | | | III-1 | Mrpl24 | 79590 | 4-May-15 |
| 13716 | 2 | 3 | | | III-1 | Mrpl28 | 10573 | 7-Jun-15 |
| 13719 | 2 | 3 | | | III-1 | Mrpl32 | 64983 | 7-Jun-15 |
| 13720 | 2 | 3 | | | III-1 | Mrpl33 | 9553 | 4-May-15 |
| 13721 | 2 | 3 | | | III-1 | Mrpl34 | 64981 | 4-May-15 |
| 13725 | 2 | 3 | | | III-1 | Mrpl38 | 64978 | 4-May-15 |
| 13726 | 2 | 3 | | | III-1 | Mrpl39 | 54148 | 12-May-15 |
| 13731 | 2 | 3 | | | III-1 | Mrpl43 | 84545 | 12-May-15 |
| 13734 | 2 | 3 | | | III-1 | Mrpl46 | 26589 | 4-May-15 |
| 13736 | 2 | 3 | | | III-1 | Mrpl48 | 51642 | 4-May-15 |
| 13737 | 2 | 3 | | | III-1 | Mrpl49 | 740 | 4-May-15 |
| 13741 | 2 | 3 | | | III-1 | Mrpl53 | 116540 | 4-May-15 |
| 13747 | 2 | 3 | | | III-1 | Mrps11 | 64963 | 28-May-15 |
| 13752 | 2 | 3 | | | III-1 | Mrps17 | 51373 | 28-May-15 |
| 13754 | 2 | 3 | | | III-1 | Mrps18b | 28973 | 28-May-15 |
| 13758 | 2 | 3 | | | III-1 | Mrps22 | 56945 | 4-May-15 |
| 13759 | 2 | 3 | | | III-1 | Mrps23 | 51649 | 4-May-15 |

Fig.22 - 74

| | | | | | | | | |
|---|---|---|---|---|---|---|---|---|
| 13761 | 2 | 3 | | | III-1 | Mrps25 | 64432 | 4-May-15 |
| 13762 | 2 | 3 | | | III-1 | Mrps26 | 64949 | 4-May-15 |
| 13763 | 2 | 3 | | | III-1 | Mrps27 | 23107 | 4-May-15 |
| 13768 | 2 | 3 | | | III-1 | Mrps34 | 65993 | 21-May-15 |
| 13769 | 2 | 3 | | | III-1 | Mrps35 | 60488 | 7-Jun-15 |
| 13770 | 2 | 3 | | | III-1 | Mrps36 | 92259 | 4-May-15 |
| 13775 | 2 | 3 | | | III-1 | Mrrf | 92399 | 4-May-15 |
| 13777 | 2 | 3 | | | III-1 | Mrto4 | 51154 | 4-May-15 |
| 13785 | 2 | 3 | | | III-1 | Ms4a3 | 932 | 4-May-15 |
| 13789 | 2 | 3 | | | III-1 | Ms4a5 | 64232 | 4-May-15 |
| 13795 | 2 | 3 | | | III-1 | Msantd1 | 345222 | 4-May-15 |
| 13796 | 2 | 3 | | | III-1 | Msantd2 | 79684 | 12-May-15 |
| 13805 | 2 | 3 | | | III-1 | Msh6 | 2956 | 23-May-15 |
| 13806 | 2 | 3 | | | III-1 | Msi1 | 4440 | 1-Jun-15 |
| 13808 | 2 | 3 | | | III-1 | Msl1 | 339287 | 7-Jun-15 |
| 13816 | 2 | 3 | | | III-1 | Msmp | 692094 | 4-May-15 |
| 13819 | 2 | 3 | | | III-1 | Msra | 4482 | 12-May-15 |
| 13821 | 2 | 3 | | | III-1 | Msrb2 | 22921 | 23-May-15 |
| 13828 | 2 | 3 | | | III-1 | Msx1 | 4487 | 17-May-15 |
| 13845 | 2 | 3 | | | III-1 | Mtcl1 | 23255 | 12-May-15 |
| 13847 | 2 | 3 | | | III-1 | Mtdh | 92140 | 17-May-15 |
| 13848 | 2 | 3 | | | III-1 | Mterf1a | | |
| 13850 | 2 | 3 | | | III-1 | Mterfd1 | 51001 | 4-May-15 |
| 13857 | 2 | 3 | | | III-1 | Mtfr1 | 9650 | 20-May-15 |
| 13858 | 2 | 3 | | | III-1 | Mtfr1l | 56181 | 4-May-15 |
| 13861 | 2 | 3 | | | III-1 | Mtg2 | 26164 | 4-May-15 |
| 13865 | 2 | 3 | | | III-1 | Mthfd2l | 441024 | 4-May-15 |
| 13866 | 2 | 3 | | | III-1 | Mthfr | 4524 | 24-May-15 |
| 13868 | 2 | 3 | | | III-1 | Mthfsd | 64779 | 4-May-15 |
| 13876 | 2 | 3 | | | III-1 | Mtmr12 | 54545 | 12-May-15 |
| 13878 | 2 | 3 | | | III-1 | Mtmr2 | 8898 | 23-May-15 |
| 13884 | 2 | 3 | | | III-1 | Mtnr1a | 4543 | 4-May-15 |
| 13885 | 2 | 3 | | | III-1 | Mtnr1b | 4544 | 21-May-15 |
| 13891 | 2 | 3 | | | III-1 | Mtrf1 | 9617 | 4-May-15 |
| 13892 | 2 | 3 | | | III-1 | Mtrf1l | 54516 | 4-May-15 |
| 13896 | 2 | 3 | | | III-1 | Mttp | 4547 | 7-Jun-15 |
| 13897 | 2 | 3 | | | III-1 | Mturn | 222166 | 4-May-15 |
| 13899 | 2 | 3 | | | III-1 | Mtus2 | 23281 | 4-May-15 |
| 13900 | 2 | 3 | | | III-1 | Mtx1 | 4580 | 4-May-15 |
| 13901 | 2 | 3 | | | III-1 | Mtx2 | 10651 | 4-May-15 |
| 13902 | 2 | 3 | | | III-1 | Mtx3 | 345778 | 4-May-15 |
| 13904 | 2 | 3 | | | III-1 | Muc13 | 56667 | 4-May-15 |
| 13913 | 2 | 3 | | | III-1 | Mucl1 | 118430 | 4-May-15 |
| 13916 | 2 | 3 | | | III-1 | Mug-ps1 | | |
| 13936 | 2 | 3 | | | III-1 | Mup6 | | |
| 13942 | 2 | 3 | | | III-1 | Musk | 4593 | 7-May-15 |
| 13947 | 2 | 3 | | | III-1 | Mvb12b | 89853 | 12-May-15 |
| 13951 | 2 | 3 | | | III-1 | Mx1 | 4599 | 12-May-15 |
| 13953 | 2 | 3 | | | III-1 | Mxd1 | 4084 | 24-May-15 |
| 13959 | 2 | 3 | | | III-1 | Myadm | 91663 | 21-May-15 |
| 13969 | 2 | 3 | | | III-1 | Mybphl | 343263 | 4-May-15 |
| 13972 | 2 | 3 | | | III-1 | Mycbp2 | 23077 | 4-May-15 |
| 13979 | 2 | 3 | | | III-1 | Myef2 | 50804 | 4-May-15 |
| 13980 | 2 | 3 | | | III-1 | Myeov2 | 150678 | 4-May-15 |
| 13981 | 2 | 3 | | | III-1 | Myf5 | 4617 | 28-May-15 |
| 14009 | 2 | 3 | | | III-1 | Mylip | 29116 | 4-May-15 |
| 14017 | 2 | 3 | | | III-1 | Myo15 | 51168 | 23-May-15 |
| 14022 | 2 | 3 | | | III-1 | Myo1a | 4640 | 23-May-15 |
| 14024 | 2 | 3 | | | III-1 | Myo1c | 4641 | 7-Jun-15 |
| 14025 | 2 | 3 | | | III-1 | Myo1d | 4642 | 12-May-15 |
| 14026 | 2 | 3 | | | III-1 | Myo1e | 4643 | 12-May-15 |
| 14032 | 2 | 3 | | | III-1 | Myo5a | 4644 | 4-May-15 |
| 14035 | 2 | 3 | | | III-1 | Myo6 | 4646 | 23-May-15 |
| 14036 | 2 | 3 | | | III-1 | Myo7a | 4647 | 23-May-15 |
| 14037 | 2 | 3 | | | III-1 | Myo7b | 4648 | 4-May-15 |
| 14054 | 2 | 3 | | | III-1 | Myrf | 745 | 12-May-15 |
| 14057 | 2 | 3 | | | III-1 | Mysm1 | 114803 | 4-May-15 |
| 14063 | 2 | 3 | | | III-1 | Mzt1 | 440145 | 4-May-15 |
| 14066 | 2 | 3 | | | III-1 | N4bp1 | 9683 | 4-May-15 |
| 14069 | 2 | 3 | | | III-1 | N4bp2l2 | 10443 | 4-May-15 |
| 14072 | 2 | 3 | | | III-1 | N6amt2 | 221143 | 4-May-15 |
| 14074 | 2 | 3 | | | III-1 | Naa11 | 84779 | 4-May-15 |
| 14077 | 2 | 3 | | | III-1 | Naa20 | 51126 | 4-May-15 |
| 14082 | 2 | 3 | | | III-1 | Naa40 | 79829 | 4-May-15 |
| 14088 | 2 | 3 | | | III-1 | Nab1 | 4664 | 4-May-15 |
| 14090 | 2 | 3 | | | III-1 | Nabp1 | 64859 | 4-May-15 |
| 14091 | 2 | 3 | | | III-1 | Nabp2 | 79035 | 4-May-15 |
| 14093 | 2 | 3 | | | III-1 | Nacad | 23148 | 4-May-15 |
| 14094 | 2 | 3 | | | III-1 | Nacc1 | 112939 | 4-May-15 |
| 14096 | 2 | 3 | | | III-1 | Nadk | 65220 | 4-May-15 |
| 14098 | 2 | 3 | | | III-1 | Nadsyn1 | 55191 | 12-May-15 |
| 14100 | 2 | 3 | | | III-1 | Naf1 | 92345 | 16-Jun-15 |
| 14107 | 2 | 3 | | | III-1 | Naip1 | | |
| 14114 | 2 | 3 | | | III-1 | Nanog | 79923 | 7-Jun-15 |
| 14117 | 2 | 3 | | | III-1 | Nanos3 | 342977 | 4-May-15 |
| 14120 | 2 | 3 | | | III-1 | Nap1l1 | 4673 | 12-May-15 |
| 14121 | 2 | 3 | | | III-1 | Nap1l2 | 4674 | 12-May-15 |
| 14122 | 2 | 3 | | | III-1 | Nap1l3 | 4675 | 12-May-15 |
| 14127 | 2 | 3 | | | III-1 | Napepld | 222236 | 12-May-15 |
| 14128 | 2 | 3 | | | III-1 | Napg | 8774 | 4-May-15 |
| 14130 | 2 | 3 | | | III-1 | Napsa | 9476 | 4-May-15 |
| 14136 | 2 | 3 | | | III-1 | Nasp | 4678 | 21-May-15 |
| 14140 | 2 | 3 | | | III-1 | Nat2 | 10 | 16-Jun-15 |
| 14146 | 2 | 3 | | | III-1 | Nav1 | 89796 | 4-May-15 |
| 14148 | 2 | 3 | | | III-1 | Nav3 | 89795 | 12-May-15 |
| 14150 | 2 | 3 | | | III-1 | Nbea | 26960 | 4-May-15 |
| 14151 | 2 | 3 | | | III-1 | Nbeal1 | 65065 | 4-May-15 |
| 14153 | 2 | 3 | | | III-1 | Nbl1 | 4681 | 10-May-15 |
| 14154 | 2 | 3 | | | III-1 | Nbn | 4683 | 7-Jun-15 |
| 14158 | 2 | 3 | | | III-1 | Ncam2 | 4685 | 7-Jun-15 |
| 14162 | 2 | 3 | | | III-1 | Ncapg | 64151 | 4-May-15 |
| 14167 | 2 | 3 | | | III-1 | Ncbp2 | 22916 | 2-Jun-15 |
| 14176 | 2 | 3 | | | III-1 | Nckap1 | 10787 | 4-May-15 |
| 14179 | 2 | 3 | | | III-1 | Nckap5l | 57701 | 4-May-15 |
| 14181 | 2 | 3 | | | III-1 | Ncl | 4691 | 13-Jun-15 |
| 14184 | 2 | 3 | | | III-1 | Ncoa1 | 8648 | 3-May-15 |
| 14190 | 2 | 3 | | | III-1 | Ncoa7 | 135112 | 4-May-15 |
| 14191 | 2 | 3 | | | III-1 | Ncor1 | 9611 | 4-May-15 |
| 14192 | 2 | 3 | | | III-1 | Ncor2 | 9612 | 4-May-15 |
| 14195 | 2 | 3 | | | III-1 | Ncstn | 23385 | 12-May-15 |
| 14198 | 2 | 3 | | | III-1 | Ndc80 | 10403 | 4-May-15 |
| 14200 | 2 | 3 | | | III-1 | Ndel1 | 81565 | 2-Jun-15 |
| 14202 | 2 | 3 | | | III-1 | Ndfip2 | 54602 | 4-May-15 |
| 14207 | 2 | 3 | | | III-1 | Ndp | 4693 | 23-May-15 |
| 14210 | 2 | 3 | | | III-1 | Ndrg3 | 57446 | 4-May-15 |
| 14213 | 2 | 3 | | | III-1 | Ndst2 | 8509 | 4-May-15 |
| 14217 | 2 | 3 | | | III-1 | Ndufa10 | 4705 | 23-May-15 |
| 14219 | 2 | 3 | | | III-1 | Ndufa12 | 55967 | 23-May-15 |
| 14220 | 2 | 3 | | | III-1 | Ndufa13 | 51079 | 4-May-15 |
| 14222 | 2 | 3 | | | III-1 | Ndufa3 | 4696 | 23-May-15 |
| 14226 | 2 | 3 | | | III-1 | Ndufa6 | 4700 | 4-May-15 |
| 14227 | 2 | 3 | | | III-1 | Ndufa7 | 4701 | 29-May-15 |
| 14228 | 2 | 3 | | | III-1 | Ndufa8 | 4702 | 4-May-15 |
| 14229 | 2 | 3 | | | III-1 | Ndufa9 | 4704 | 23-May-15 |
| 14231 | 2 | 3 | | | III-1 | Ndufaf1 | 51103 | 4-May-15 |
| 14233 | 2 | 3 | | | III-1 | Ndufaf3 | 25915 | 4-May-15 |
| 14236 | 2 | 3 | | | III-1 | Ndufaf6 | 137682 | 23-May-15 |
| 14237 | 2 | 3 | | | III-1 | Ndufaf7 | 55471 | 31-May-15 |
| 14241 | 2 | 3 | | | III-1 | Ndufb3 | 4709 | 23-May-15 |
| 14242 | 2 | 3 | | | III-1 | Ndufb4 | 4710 | 4-May-15 |
| 14243 | 2 | 3 | | | III-1 | Ndufb5 | 4711 | 4-May-15 |
| 14244 | 2 | 3 | | | III-1 | Ndufb6 | 4712 | 4-May-15 |
| 14247 | 2 | 3 | | | III-1 | Ndufb9 | 4715 | 28-May-15 |
| 14248 | 2 | 3 | | | III-1 | Ndufc1 | 4717 | 4-May-15 |
| 14249 | 2 | 3 | | | III-1 | Ndufc2 | 4718 | 4-May-15 |
| 14250 | 2 | 3 | | | III-1 | Ndufs1 | 4719 | 4-May-15 |
| 14251 | 2 | 3 | | | III-1 | Ndufs2 | 4720 | 21-May-15 |
| 14252 | 2 | 3 | | | III-1 | Ndufs3 | 4722 | 23-May-15 |
| 14253 | 2 | 3 | | | III-1 | Ndufs4 | 4724 | 23-May-15 |
| 14256 | 2 | 3 | | | III-1 | Ndufs7 | 374291 | 23-May-15 |
| 14257 | 2 | 3 | | | III-1 | Ndufs8 | 4728 | 23-May-15 |
| 14258 | 2 | 3 | | | III-1 | Ndufv1 | 4723 | 12-May-15 |
| 14259 | 2 | 3 | | | III-1 | Ndufv2 | 4729 | 4-May-15 |
| 14260 | 2 | 3 | | | III-1 | Ndufv3 | 4731 | 4-May-15 |
| 14266 | 2 | 3 | | | III-1 | Necab3 | 63941 | 4-May-15 |
| 14267 | 2 | 3 | | | III-1 | Necap1 | 25977 | 4-May-15 |
| 14272 | 2 | 3 | | | III-1 | Nedd8 | 4738 | 4-May-15 |
| 14274 | 2 | 3 | | | III-1 | Nefh | 4744 | 23-May-15 |
| 14277 | 2 | 3 | | | III-1 | Negr1 | 257194 | 4-May-15 |
| 14278 | 2 | 3 | | | III-1 | Neil1 | 79661 | 4-May-15 |
| 14289 | 2 | 3 | | | III-1 | Nek7 | 140609 | 4-May-15 |
| 14291 | 2 | 3 | | | III-1 | Nek9 | 91754 | 4-May-15 |
| 14293 | 2 | 3 | | | III-1 | Nelfb | 25920 | 4-May-15 |
| 14297 | 2 | 3 | | | III-1 | Nell1os | | |
| 14298 | 2 | 3 | | | III-1 | Nell2 | 4753 | 12-May-15 |
| 14300 | 2 | 3 | | | III-1 | Nenf | 29937 | 4-May-15 |
| 14302 | 2 | 3 | | | III-1 | Nepn | 442253 | 4-May-15 |
| 14306 | 2 | 3 | | | III-1 | Neto1 | 81832 | 4-May-15 |
| 14311 | 2 | 3 | | | III-1 | Neu4 | 129807 | 4-May-15 |
| 14316 | 2 | 3 | | | III-1 | Neurl4 | 84461 | 4-May-15 |
| 14318 | 2 | 3 | | | III-1 | Neurod2 | 4761 | 28-May-15 |
| 14320 | 2 | 3 | | | III-1 | Neurod6 | 63974 | 28-May-15 |
| 14330 | 2 | 3 | | | III-1 | Nfatc1 | 4772 | 31-May-15 |
| 14332 | 2 | 3 | | | III-1 | Nfatc2ip | 84901 | 4-May-15 |
| 14336 | 2 | 3 | | | III-1 | Nfe2l1 | 4779 | 4-May-15 |
| 14339 | 2 | 3 | | | III-1 | Nfia | 4774 | 2-Jun-15 |
| 14340 | 2 | 3 | | | III-1 | Nfib | 4781 | 28-May-15 |
| 14341 | 2 | 3 | | | III-1 | Nfic | 4782 | 17-May-15 |
| 14344 | 2 | 3 | | | III-1 | Nfkb1 | 4790 | 28-May-15 |
| 14347 | 2 | 3 | | | III-1 | Nfkbib | 4793 | 31-May-15 |
| 14348 | 2 | 3 | | | III-1 | Nfkbid | 84807 | 4-May-15 |
| 14349 | 2 | 3 | | | III-1 | Nfkbie | 4794 | 12-May-15 |
| 14352 | 2 | 3 | | | III-1 | Nfrkb | 4798 | 4-May-15 |
| 14355 | 2 | 3 | | | III-1 | Nfx1 | 4799 | 4-May-15 |
| 14361 | 2 | 3 | | | III-1 | Ngdn | 25983 | 4-May-15 |
| 14362 | 2 | 3 | | | III-1 | Ngef | 25791 | 4-May-15 |
| 14363 | 2 | 3 | | | III-1 | Ngf | 4803 | 24-May-15 |
| 14366 | 2 | 3 | | | III-1 | Ngly1 | 55768 | 12-May-15 |
| 14374 | 2 | 3 | | | III-1 | Nhlrc3 | 387921 | 4-May-15 |
| 14380 | 2 | 3 | | | III-1 | Nhsl2 | 340527 | 12-May-15 |
| 14382 | 2 | 3 | | | III-1 | Nid1 | 4811 | 12-May-15 |
| 14389 | 2 | 3 | | | III-1 | Ninj2 | 4815 | 12-May-15 |
| 14391 | 2 | 3 | | | III-1 | Nip7 | 51388 | 4-May-15 |
| 14393 | 2 | 3 | | | III-1 | Nipa2 | 81614 | 7-Jun-15 |
| 14396 | 2 | 3 | | | III-1 | Nipal3 | 57185 | 4-May-15 |
| 14399 | 2 | 3 | | | III-1 | Nipsnap1 | 8508 | 17-May-15 |
| 14400 | 2 | 3 | | | III-1 | Nipsnap3a | 25934 | 4-May-15 |
| 14405 | 2 | 3 | | | III-1 | Nkain1 | 79570 | 4-May-15 |
| 14406 | 2 | 3 | | | III-1 | Nkain2 | 154215 | 4-May-15 |
| 14409 | 2 | 3 | | | III-1 | Nkap | 79576 | 4-May-15 |
| 14411 | 2 | 3 | | | III-1 | Nkd1 | 85407 | 12-May-15 |
| 14412 | 2 | 3 | | | III-1 | Nkd2 | 85409 | 4-May-15 |
| 14413 | 2 | 3 | | | III-1 | Nkg7 | 4818 | 4-May-15 |
| 14414 | 2 | 3 | | | III-1 | Nkiras1 | 28512 | 4-May-15 |

Fig.22 - 75

| | | | | | | | | |
|---|---|---|---|---|---|---|---|---|
| 14417 | 2 | 3 | | | III-1 | Nkrf | 55922 | 4-May-15 |
| 14418 | 2 | 3 | | | III-1 | Nktr | 4820 | 4-May-15 |
| 14419 | 2 | 3 | | | III-1 | Nkx1-1 | 54729 | 4-May-15 |
| 14423 | 2 | 3 | | | III-1 | Nkx2-2os | | |
| 14425 | 2 | 3 | | | III-1 | Nkx2-4 | 644524 | 4-May-15 |
| 14426 | 2 | 3 | | | III-1 | Nkx2-5 | 1482 | 31-May-15 |
| 14436 | 2 | 3 | | | III-1 | Nlgn2 | 57555 | 28-May-15 |
| 14437 | 2 | 3 | | | III-1 | Nlgn3 | 54413 | 28-May-15 |
| 14438 | 2 | 3 | | | III-1 | Nlk | 51701 | 7-Jun-15 |
| 14448 | 2 | 3 | | | III-1 | Nlrp1c-ps | | |
| 14451 | 2 | 3 | | | III-1 | Nlrp4a | | |
| 14473 | 2 | 3 | | | III-1 | Nme7 | 29922 | 12-May-15 |
| 14477 | 2 | 3 | | | III-1 | Nmnat1 | 64802 | 23-May-15 |
| 14480 | 2 | 3 | | | III-1 | Nmral1 | 57407 | 20-May-15 |
| 14486 | 2 | 3 | | | III-1 | Nmu | 10874 | 12-May-15 |
| 14488 | 2 | 3 | | | III-1 | Nmur2 | 56923 | 4-May-15 |
| 14492 | 2 | 3 | | | III-1 | Noa1 | 84273 | 4-May-15 |
| 14496 | 2 | 3 | | | III-1 | Noc3l | 64318 | 4-May-15 |
| 14499 | 2 | 3 | | | III-1 | Nod2 | 64127 | 31-May-15 |
| 14506 | 2 | 3 | | | III-1 | Nol4 | 8715 | 4-May-15 |
| 14510 | 2 | 3 | | | III-1 | Nol9 | 79707 | 4-May-15 |
| 14512 | 2 | 3 | | | III-1 | Nom1 | 64434 | 4-May-15 |
| 14517 | 2 | 3 | | | III-1 | Nop16 | 51491 | 12-May-15 |
| 14518 | 2 | 3 | | | III-1 | Nop2 | 4839 | 12-May-15 |
| 14519 | 2 | 3 | | | III-1 | Nop56 | 10528 | 22-May-15 |
| 14520 | 2 | 3 | | | III-1 | Nop58 | 51602 | 21-May-15 |
| 14521 | 2 | 3 | | | III-1 | Nop9 | 161424 | 7-Jun-15 |
| 14523 | 2 | 3 | | | III-1 | Nos1ap | 9722 | 1-Jun-15 |
| 14526 | 2 | 3 | | | III-1 | Nosip | 51070 | 4-May-15 |
| 14528 | 2 | 3 | | | III-1 | Notch1 | 4851 | 31-May-15 |
| 14529 | 2 | 3 | | | III-1 | Notch2 | 4853 | 23-May-15 |
| 14530 | 2 | 3 | | | III-1 | Notch3 | 4854 | 23-May-15 |
| 14531 | 2 | 3 | | | III-1 | Notch4 | 4855 | 4-May-15 |
| 14538 | 2 | 3 | | | III-1 | Nox3 | 50508 | 3-May-15 |
| 14541 | 2 | 3 | | | III-1 | Noxo1 | 124056 | 4-May-15 |
| 14542 | 2 | 3 | | | III-1 | Noxred1 | 122945 | 4-May-15 |
| 14549 | 2 | 3 | | | III-1 | Npbwr1 | 2831 | 4-May-15 |
| 14551 | 2 | 3 | | | III-1 | Npc1l1 | 29881 | 17-May-15 |
| 14555 | 2 | 3 | | | III-1 | Npepl1 | 79716 | 4-May-15 |
| 14558 | 2 | 3 | | | III-1 | Npffr1 | 64106 | 4-May-15 |
| 14562 | 2 | 3 | | | III-1 | Nphp4 | 261734 | 17-May-15 |
| 14563 | 2 | 3 | | | III-1 | Nphs1 | 4868 | 4-May-15 |
| 14567 | 2 | 3 | | | III-1 | Nploc4 | 55666 | 28-May-15 |
| 14569 | 2 | 3 | | | III-1 | Npm2 | 10361 | 4-May-15 |
| 14574 | 2 | 3 | | | III-1 | Nppb | 4879 | 17-May-15 |
| 14575 | 2 | 3 | | | III-1 | Nppc | 4880 | 21-May-15 |
| 14577 | 2 | 3 | | | III-1 | Npr2 | 4882 | 7-Jun-15 |
| 14580 | 2 | 3 | | | III-1 | Nprl3 | 8131 | 29-May-15 |
| 14591 | 2 | 3 | | | III-1 | Npy2r | 4887 | 12-May-15 |
| 14596 | 2 | 3 | | | III-1 | Nqo2 | 4835 | 28-May-15 |
| 14598 | 2 | 3 | | | III-1 | Nr0b2 | 8431 | 24-May-15 |
| 14604 | 2 | 3 | | | III-1 | Nr1h5 | 643609 | 4-May-15 |
| 14607 | 2 | 3 | | | III-1 | Nr2c1 | 7181 | 4-May-15 |
| 14610 | 2 | 3 | | | III-1 | Nr2e1 | 7101 | 4-May-15 |
| 14614 | 2 | 3 | | | III-1 | Nr2f6 | 2063 | 4-May-15 |
| 14615 | 2 | 3 | | | III-1 | Nr3c1 | 2908 | 31-May-15 |
| 14618 | 2 | 3 | | | III-1 | Nr4a2 | 4929 | 31-May-15 |
| 14620 | 2 | 3 | | | III-1 | Nr5a1 | 2516 | 12-May-15 |
| 14621 | 2 | 3 | | | III-1 | Nr5a2 | 2494 | 12-May-15 |
| 14623 | 2 | 3 | | | III-1 | Nradd | | |
| 14630 | 2 | 3 | | | III-1 | Nrcam | 4897 | 4-May-15 |
| 14632 | 2 | 3 | | | III-1 | Nrde2 | 55051 | 4-May-15 |
| 14636 | 2 | 3 | | | III-1 | Nrg2 | 9542 | 12-May-15 |
| 14639 | 2 | 3 | | | III-1 | Nrg4 | 145957 | 4-May-15 |
| 14644 | 2 | 3 | | | III-1 | Nrk | 203447 | 12-May-15 |
| 14648 | 2 | 3 | | | III-1 | Nrn1l | 123904 | 4-May-15 |
| 14657 | 2 | 3 | | | III-1 | Nrxn1 | 9378 | 4-May-15 |
| 14659 | 2 | 3 | | | III-1 | Nrxn3 | 9369 | 4-May-15 |
| 14661 | 2 | 3 | | | III-1 | Nsd1 | 64324 | 23-May-15 |
| 14662 | 2 | 3 | | | III-1 | Nsdhl | 50814 | 23-May-15 |
| 14666 | 2 | 3 | | | III-1 | Nsg2 | | |
| 14671 | 2 | 3 | | | III-1 | Nsmce4a | 54780 | 4-May-15 |
| 14673 | 2 | 3 | | | III-1 | Nsun2 | 54888 | 4-May-15 |
| 14675 | 2 | 3 | | | III-1 | Nsun4 | 387338 | 4-May-15 |
| 14677 | 2 | 3 | | | III-1 | Nsun6 | 221078 | 12-May-15 |
| 14679 | 2 | 3 | | | III-1 | Nt5c | 30833 | 4-May-15 |
| 14682 | 2 | 3 | | | III-1 | Nt5c2 | 22978 | 2-Jun-15 |
| 14683 | 2 | 3 | | | III-1 | Nt5c3 | 51251 | 4-May-15 |
| 14684 | 2 | 3 | | | III-1 | Nt5c3b | 115024 | 10-May-15 |
| 14687 | 2 | 3 | | | III-1 | Nt5dc3 | 51559 | 23-May-15 |
| 14691 | 2 | 3 | | | III-1 | Ntf3 | 4908 | 3-May-15 |
| 14693 | 2 | 3 | | | III-1 | Nthl1 | 4913 | 28-May-15 |
| 14694 | 2 | 3 | | | III-1 | Ntm | 50863 | 2-Jun-15 |
| 14696 | 2 | 3 | | | III-1 | Ntn1 | 9423 | 17-May-15 |
| 14699 | 2 | 3 | | | III-1 | Ntn5 | 126147 | 4-May-15 |
| 14706 | 2 | 3 | | | III-1 | Nts | 4922 | 4-May-15 |
| 14709 | 2 | 3 | | | III-1 | Nuak1 | 9891 | 4-May-15 |
| 14710 | 2 | 3 | | | III-1 | Nuak2 | 81788 | 4-May-15 |
| 14711 | 2 | 3 | | | III-1 | Nub1 | 51667 | 4-May-15 |
| 14715 | 2 | 3 | | | III-1 | Nucb1 | 4924 | 4-May-15 |
| 14717 | 2 | 3 | | | III-1 | Nucks1 | 64710 | 4-May-15 |
| 14725 | 2 | 3 | | | III-1 | Nudt12 | 83594 | 4-May-15 |
| 14726 | 2 | 3 | | | III-1 | Nudt13 | 25961 | 4-May-15 |
| 14728 | 2 | 3 | | | III-1 | Nudt15 | 55270 | 4-May-15 |
| 14730 | 2 | 3 | | | III-1 | Nudt16l1 | 84309 | 4-May-15 |
| 14733 | 2 | 3 | | | III-1 | Nudt19 | 390916 | 21-May-15 |
| 14737 | 2 | 3 | | | III-1 | Nudt3 | 11165 | 4-May-15 |
| 14739 | 2 | 3 | | | III-1 | Nudt5 | 11164 | 21-May-15 |
| 14741 | 2 | 3 | | | III-1 | Nudt7 | 283927 | 4-May-15 |
| 14744 | 2 | 3 | | | III-1 | Nuf2 | 83540 | 4-May-15 |
| 14746 | 2 | 3 | | | III-1 | Nufip2 | 57532 | 4-May-15 |
| 14751 | 2 | 3 | | | III-1 | Nup107 | 57122 | 4-May-15 |
| 14759 | 2 | 3 | | | III-1 | Nup210l | 91181 | 21-May-15 |
| 14762 | 2 | 3 | | | III-1 | Nup37 | 79023 | 2-Jun-15 |
| 14764 | 2 | 3 | | | III-1 | Nup50 | 10762 | 4-May-15 |
| 14769 | 2 | 3 | | | III-1 | Nup85 | 79902 | 4-May-15 |
| 14773 | 2 | 3 | | | III-1 | Nupl1 | 9818 | 4-May-15 |
| 14774 | 2 | 3 | | | III-1 | Nupl2 | 11097 | 12-May-15 |
| 14784 | 2 | 3 | | | III-1 | Nwd2 | 57495 | 4-May-15 |
| 14788 | 2 | 3 | | | III-1 | Nxf7 | | |
| 14807 | 2 | 3 | | | III-1 | Oaf | 220323 | 4-May-15 |
| 14809 | 2 | 3 | | | III-1 | Oas1a | | |
| 14811 | 2 | 3 | | | III-1 | Oas1c | | |
| 14812 | 2 | 3 | | | III-1 | Oas1d | | |
| 14814 | 2 | 3 | | | III-1 | Oas1f | | |
| 14827 | 2 | 3 | | | III-1 | Obox1 | | |
| 14834 | 2 | 3 | | | III-1 | Obp2b | 29989 | 4-May-15 |
| 14843 | 2 | 3 | | | III-1 | Ocm | 654231 | 4-May-15 |
| 14844 | 2 | 3 | | | III-1 | Ocrl | 4952 | 23-May-15 |
| 14846 | 2 | 3 | | | III-1 | Odam | 54959 | 4-May-15 |
| 14847 | 2 | 3 | | | III-1 | Odc1 | 4953 | 7-Jun-15 |
| 14849 | 2 | 3 | | | III-1 | Odf2 | 4957 | 7-Jun-15 |
| 14850 | 2 | 3 | | | III-1 | Odf2l | 57489 | 4-May-15 |
| 14851 | 2 | 3 | | | III-1 | Odf3 | 113746 | 4-May-15 |
| 14855 | 2 | 3 | | | III-1 | Odf4 | 146852 | 4-May-15 |
| 14858 | 2 | 3 | | | III-1 | Ogdh | 4967 | 12-May-15 |
| 14860 | 2 | 3 | | | III-1 | Ogfod1 | 55239 | 12-May-15 |
| 14862 | 2 | 3 | | | III-1 | Ogfod3 | 79701 | 4-May-15 |
| 14863 | 2 | 3 | | | III-1 | Ogfr | 11054 | 4-May-15 |
| 14865 | 2 | 3 | | | III-1 | Ogg1 | 4968 | 4-May-15 |
| 14867 | 2 | 3 | | | III-1 | Ogt | 8473 | 17-May-15 |
| 14871 | 2 | 3 | | | III-1 | Ola1 | 29789 | 23-May-15 |
| 14877 | 2 | 3 | | | III-1 | Olfml1 | 283298 | 4-May-15 |
| 14912 | 2 | 3 | | | III-1 | Olfr1036 | | |
| 15107 | 2 | 3 | | | III-1 | Olfr1270 | | |
| 15177 | 2 | 3 | | | III-1 | Olfr1346 | | |
| 15203 | 2 | 3 | | | III-1 | Olfr1373 | | |
| 15211 | 2 | 3 | | | III-1 | Olfr1384 | | |
| 15243 | 2 | 3 | | | III-1 | Olfr1423 | | |
| 15308 | 2 | 3 | | | III-1 | Olfr1508 | | |
| 15454 | 2 | 3 | | | III-1 | Olfr331 | | |
| 15619 | 2 | 3 | | | III-1 | Olfr541 | | |
| 15937 | 2 | 3 | | | III-1 | Olfr921 | | |
| 15992 | 2 | 3 | | | III-1 | Olfr992 | | |
| 16021 | 2 | 3 | | | III-1 | Opalin | 93377 | 4-May-15 |
| 16024 | 2 | 3 | | | III-1 | Oplah | 26873 | 4-May-15 |
| 16025 | 2 | 3 | | | III-1 | Opn1mw | 2652 | 23-May-15 |
| 16028 | 2 | 3 | | | III-1 | Opn4 | 94233 | 21-May-15 |
| 16035 | 2 | 3 | | | III-1 | Optn | 10133 | 23-May-15 |
| 16037 | 2 | 3 | | | III-1 | Orai2 | 80228 | 4-May-15 |
| 16039 | 2 | 3 | | | III-1 | Oraov1 | 220064 | 4-May-15 |
| 16045 | 2 | 3 | | | III-1 | Orc6 | 23594 | 4-May-15 |
| 16046 | 2 | 3 | | | III-1 | Orm1 | 5004 | 12-May-15 |
| 16051 | 2 | 3 | | | III-1 | Ormdl3 | 94103 | 4-May-15 |
| 16052 | 2 | 3 | | | III-1 | Os9 | 10956 | 3-May-15 |
| 16056 | 2 | 3 | | | III-1 | Osbpl11 | 114885 | 4-May-15 |
| 16058 | 2 | 3 | | | III-1 | Osbpl2 | 9885 | 22-May-15 |
| 16059 | 2 | 3 | | | III-1 | Osbpl3 | 26031 | 4-May-15 |
| 16060 | 2 | 3 | | | III-1 | Osbpl5 | 114879 | 4-May-15 |
| 16061 | 2 | 3 | | | III-1 | Osbpl6 | 114880 | 12-May-15 |
| 16063 | 2 | 3 | | | III-1 | Osbpl8 | 114882 | 12-May-15 |
| 16067 | 2 | 3 | | | III-1 | Oser1 | 51526 | 4-May-15 |
| 16069 | 2 | 3 | | | III-1 | Osgepl1 | 64172 | 4-May-15 |
| 16077 | 2 | 3 | | | III-1 | Ostc | 58505 | 4-May-15 |
| 16078 | 2 | 3 | | | III-1 | Ostf1 | 26578 | 4-May-15 |
| 16079 | 2 | 3 | | | III-1 | Ostm1 | 28962 | 4-May-15 |
| 16084 | 2 | 3 | | | III-1 | Otog | 340990 | 7-Jun-15 |
| 16090 | 2 | 3 | | | III-1 | Otor | 56914 | 12-May-15 |
| 16091 | 2 | 3 | | | III-1 | Otos | 150677 | 4-May-15 |
| 16094 | 2 | 3 | | | III-1 | OTTMUSG00000016609 | | |
| 16095 | 2 | 3 | | | III-1 | Otub1 | 55611 | 4-May-15 |
| 16097 | 2 | 3 | | | III-1 | Otud1 | 220213 | 4-May-15 |
| 16098 | 2 | 3 | | | III-1 | Otud3 | 23252 | 4-May-15 |
| 16099 | 2 | 3 | | | III-1 | Otud4 | 54726 | 4-May-15 |
| 16104 | 2 | 3 | | | III-1 | Otud7b | 56957 | 17-May-15 |
| 16105 | 2 | 3 | | | III-1 | Otulin | 90268 | 4-May-15 |
| 16106 | 2 | 3 | | | III-1 | Otx1 | 5013 | 30-May-15 |
| 16108 | 2 | 3 | | | III-1 | Otx2os1 | 100309464 | 21-May-15 |
| 16111 | 2 | 3 | | | III-1 | Ovgp1 | 5016 | 12-May-15 |
| 16112 | 2 | 3 | | | III-1 | Ovol1 | 5017 | 4-May-15 |
| 16117 | 2 | 3 | | | III-1 | Oxct2a | | |
| 16119 | 2 | 3 | | | III-1 | Oxgr1 | 27199 | 4-May-15 |
| 16121 | 2 | 3 | | | III-1 | Oxnad1 | 92106 | 4-May-15 |
| 16122 | 2 | 3 | | | III-1 | Oxr1 | 55074 | 12-May-15 |
| 16129 | 2 | 3 | | | III-1 | P2rx3 | 5024 | 4-May-15 |
| 16131 | 2 | 3 | | | III-1 | P2rx5 | 5026 | 24-May-15 |
| 16134 | 2 | 3 | | | III-1 | P2ry1 | 5028 | 12-May-15 |
| 16141 | 2 | 3 | | | III-1 | P2ry6 | 5031 | 4-May-15 |
| 16144 | 2 | 3 | | | III-1 | P4ha3 | 283208 | 4-May-15 |
| 16145 | 2 | 3 | | | III-1 | P4hb | 5034 | 17-May-15 |
| 16147 | 2 | 3 | | | III-1 | Pa2g4 | 5036 | 12-May-15 |
| 16150 | 2 | 3 | | | III-1 | Pabpc2 | 26986 | 12-May-15 |
| 16158 | 2 | 3 | | | III-1 | Pacrgl | 133015 | 4-May-15 |

Fig.22 - 76

| | | | | | | | | |
|---|---|---|---|---|---|---|---|---|
| 16159 | 2 | 3 | | | III-1 | Pacs1 | 55690 | 4-May-15 |
| 16162 | 2 | 3 | | | III-1 | Pacsin2 | 11252 | 4-May-15 |
| 16166 | 2 | 3 | | | III-1 | Padi3 | 51702 | 4-May-15 |
| 16168 | 2 | 3 | | | III-1 | Padi6 | 353238 | 4-May-15 |
| 16175 | 2 | 3 | | | III-1 | Pagr1a | | |
| 16187 | 2 | 3 | | | III-1 | Pak7 | 57144 | 17-May-15 |
| 16190 | 2 | 3 | | | III-1 | Palld | 23022 | 31-May-15 |
| 16193 | 2 | 3 | | | III-1 | Palm3 | 342979 | 4-May-15 |
| 16198 | 2 | 3 | | | III-1 | Pan2 | 9924 | 7-Jun-15 |
| 16201 | 2 | 3 | | | III-1 | Pank2 | 80025 | 28-May-15 |
| 16203 | 2 | 3 | | | III-1 | Pank4 | 55229 | 4-May-15 |
| 16205 | 2 | 3 | | | III-1 | Panx2 | 56666 | 4-May-15 |
| 16208 | 2 | 3 | | | III-1 | Papd4 | 167153 | 4-May-15 |
| 16209 | 2 | 3 | | | III-1 | Papd5 | 64282 | 4-May-15 |
| 16212 | 2 | 3 | | | III-1 | Papln | 89932 | 4-May-15 |
| 16215 | 2 | 3 | | | III-1 | Papolg | 64895 | 4-May-15 |
| 16218 | 2 | 3 | | | III-1 | Papss1 | 9061 | 4-May-15 |
| 16224 | 2 | 3 | | | III-1 | Paqr7 | 164091 | 4-May-15 |
| 16228 | 2 | 3 | | | III-1 | Pard3b | 117583 | 12-May-15 |
| 16229 | 2 | 3 | | | III-1 | Pard6a | 50855 | 4-May-15 |
| 16232 | 2 | 3 | | | III-1 | Parg | 8505 | 17-May-15 |
| 16234 | 2 | 3 | | | III-1 | Park7 | 11315 | 31-May-15 |
| 16238 | 2 | 3 | | | III-1 | Parp1 | 142 | 31-May-15 |
| 16245 | 2 | 3 | | | III-1 | Parp3 | 10039 | 4-May-15 |
| 16249 | 2 | 3 | | | III-1 | Parp9 | 83666 | 4-May-15 |
| 16253 | 2 | 3 | | | III-1 | Parvb | 29780 | 4-May-15 |
| 16257 | 2 | 3 | | | III-1 | Pate4 | 399968 | 12-May-15 |
| 16261 | 2 | 3 | | | III-1 | Paupar | 103157000 | 4-May-15 |
| 16263 | 2 | 3 | | | III-1 | Pax1 | 5075 | 4-May-15 |
| 16265 | 2 | 3 | | | III-1 | Pax3 | 5077 | 23-May-15 |
| 16273 | 2 | 3 | | | III-1 | Paxbp1 | 94104 | 12-May-15 |
| 16278 | 2 | 3 | | | III-1 | Pbld2 | | |
| 16279 | 2 | 3 | | | III-1 | Pbp2 | | |
| 16283 | 2 | 3 | | | III-1 | Pbx2 | 5089 | 7-Jun-15 |
| 16285 | 2 | 3 | | | III-1 | Pbx4 | 80714 | 4-May-15 |
| 16286 | 2 | 3 | | | III-1 | Pbxip1 | 57326 | 4-May-15 |
| 16289 | 2 | 3 | | | III-1 | Pcbp1 | 5093 | 4-May-15 |
| 16292 | 2 | 3 | | | III-1 | Pcbp4 | 57060 | 4-May-15 |
| 16293 | 2 | 3 | | | III-1 | Pcca | 5095 | 23-May-15 |
| 16305 | 2 | 3 | | | III-1 | Pcdh8 | 5100 | 18-May-15 |
| 16307 | 2 | 3 | | | III-1 | Pcdha1 | 56147 | 4-May-15 |
| 16323 | 2 | 3 | | | III-1 | Pcdhb10 | 56126 | 12-May-15 |
| 16328 | 2 | 3 | | | III-1 | Pcdhb15 | 56121 | 4-May-15 |
| 16331 | 2 | 3 | | | III-1 | Pcdhb18 | 54660 | 4-May-15 |
| 16346 | 2 | 3 | | | III-1 | Pcdhga11 | 56105 | 4-May-15 |
| 16349 | 2 | 3 | | | III-1 | Pcdhga3 | 56112 | 12-May-15 |
| 16350 | 2 | 3 | | | III-1 | Pcdhga4 | 56111 | 4-May-15 |
| 16353 | 2 | 3 | | | III-1 | Pcdhga7 | 56108 | 4-May-15 |
| 16354 | 2 | 3 | | | III-1 | Pcdhga8 | 9708 | 4-May-15 |
| 16357 | 2 | 3 | | | III-1 | Pcdhgb2 | 56103 | 4-May-15 |
| 16358 | 2 | 3 | | | III-1 | Pcdhgb4 | 8641 | 4-May-15 |
| 16359 | 2 | 3 | | | III-1 | Pcdhgb5 | 56101 | 4-May-15 |
| 16361 | 2 | 3 | | | III-1 | Pcdhgb7 | 56099 | 4-May-15 |
| 16362 | 2 | 3 | | | III-1 | Pcdhgb8 | | |
| 16364 | 2 | 3 | | | III-1 | Pcdhgc4 | 56098 | 12-May-15 |
| 16365 | 2 | 3 | | | III-1 | Pcdhgc5 | 56097 | 4-May-15 |
| 16366 | 2 | 3 | | | III-1 | Pced1a | 64773 | 4-May-15 |
| 16368 | 2 | 3 | | | III-1 | Pcf11 | 51585 | 4-May-15 |
| 16371 | 2 | 3 | | | III-1 | Pcgf3 | 10336 | 2-Jun-15 |
| 16372 | 2 | 3 | | | III-1 | Pcgf5 | 84333 | 2-Jun-15 |
| 16374 | 2 | 3 | | | III-1 | Pcid2 | 55795 | 4-May-15 |
| 16378 | 2 | 3 | | | III-1 | Pclo | 27445 | 4-May-15 |
| 16379 | 2 | 3 | | | III-1 | Pcm1 | 5108 | 7-Jun-15 |
| 16382 | 2 | 3 | | | III-1 | Pcmtd2 | 55251 | 4-May-15 |
| 16386 | 2 | 3 | | | III-1 | Pcnx | 22990 | 21-May-15 |
| 16387 | 2 | 3 | | | III-1 | Pcnxl2 | 80003 | 4-May-15 |
| 16390 | 2 | 3 | | | III-1 | Pcolce | 5118 | 4-May-15 |
| 16395 | 2 | 3 | | | III-1 | Pcsk1 | 5122 | 4-May-15 |
| 16397 | 2 | 3 | | | III-1 | Pcsk2 | 5126 | 12-May-15 |
| 16398 | 2 | 3 | | | III-1 | Pcsk2os1 | | |
| 16403 | 2 | 3 | | | III-1 | Pcsk7 | 9159 | 4-May-15 |
| 16407 | 2 | 3 | | | III-1 | Pcyox1 | 51449 | 4-May-15 |
| 16409 | 2 | 3 | | | III-1 | Pcyt1a | 5130 | 4-May-15 |
| 16411 | 2 | 3 | | | III-1 | Pcyt2 | 5833 | 21-May-15 |
| 16412 | 2 | 3 | | | III-1 | Pdap1 | 11333 | 4-May-15 |
| 16417 | 2 | 3 | | | III-1 | Pdcd1lg2 | 80380 | 4-May-15 |
| 16419 | 2 | 3 | | | III-1 | Pdcd2l | 84306 | 4-May-15 |
| 16422 | 2 | 3 | | | III-1 | Pdcd6 | 10016 | 4-May-15 |
| 16423 | 2 | 3 | | | III-1 | Pdcd6ip | 10015 | 3-May-15 |
| 16428 | 2 | 3 | | | III-1 | Pddc1 | 347862 | 12-May-15 |
| 16433 | 2 | 3 | | | III-1 | Pde1b | 5153 | 21-May-15 |
| 16438 | 2 | 3 | | | III-1 | Pde4a | 5141 | 12-May-15 |
| 16440 | 2 | 3 | | | III-1 | Pde4c | 5143 | 12-May-15 |
| 16449 | 2 | 3 | | | III-1 | Pde6h | 5149 | 23-May-15 |
| 16451 | 2 | 3 | | | III-1 | Pde7b | 27115 | 4-May-15 |
| 16454 | 2 | 3 | | | III-1 | Pde9a | 5152 | 4-May-15 |
| 16455 | 2 | 3 | | | III-1 | Pdf | 64146 | 24-May-15 |
| 16457 | 2 | 3 | | | III-1 | Pdgfb | 5155 | 31-May-15 |
| 16459 | 2 | 3 | | | III-1 | Pdgfd | 80310 | 4-May-15 |
| 16461 | 2 | 3 | | | III-1 | Pdgfrb | 5159 | 23-May-15 |
| 16462 | 2 | 3 | | | III-1 | Pdgfrl | 5157 | 4-May-15 |
| 16464 | 2 | 3 | | | III-1 | Pdha2 | 5161 | 4-May-15 |
| 16466 | 2 | 3 | | | III-1 | Pdhx | 8050 | 29-May-15 |
| 16471 | 2 | 3 | | | III-1 | Pdia6 | 10130 | 4-May-15 |
| 16472 | 2 | 3 | | | III-1 | Pdik1l | 149420 | 4-May-15 |
| 16473 | 2 | 3 | | | III-1 | Pdilt | 204474 | 4-May-15 |
| 16476 | 2 | 3 | | | III-1 | Pdk3 | 5165 | 4-May-15 |
| 16478 | 2 | 3 | | | III-1 | Pdlim1 | 9124 | 21-May-15 |
| 16484 | 2 | 3 | | | III-1 | Pdp1 | 54704 | 7-Jun-15 |
| 16485 | 2 | 3 | | | III-1 | Pdp2 | 57546 | 4-May-15 |
| 16486 | 2 | 3 | | | III-1 | Pdpk1 | 5170 | 4-May-15 |
| 16491 | 2 | 3 | | | III-1 | Pds5b | 23047 | 4-May-15 |
| 16495 | 2 | 3 | | | III-1 | Pdxdc1 | 23042 | 23-May-15 |
| 16497 | 2 | 3 | | | III-1 | Pdxk-ps | | |
| 16499 | 2 | 3 | | | III-1 | Pdyn | 5173 | 23-May-15 |
| 16501 | 2 | 3 | | | III-1 | Pdzd2 | 23037 | 12-May-15 |
| 16502 | 2 | 3 | | | III-1 | Pdzd3 | 79849 | 4-May-15 |
| 16510 | 2 | 3 | | | III-1 | Pdzrn4 | 29951 | 4-May-15 |
| 16512 | 2 | 3 | | | III-1 | Pea15b | | |
| 16516 | 2 | 3 | | | III-1 | Pebp4 | 157310 | 4-May-15 |
| 16519 | 2 | 3 | | | III-1 | Pef1 | 553115 | 4-May-15 |
| 16521 | 2 | 3 | | | III-1 | Peg12 | | |
| 16527 | 2 | 3 | | | III-1 | Peli3 | 246330 | 3-May-15 |
| 16529 | 2 | 3 | | | III-1 | Pelp1 | 27043 | 12-May-15 |
| 16537 | 2 | 3 | | | III-1 | Peril | | |
| 16540 | 2 | 3 | | | III-1 | Pes1 | 23481 | 7-Jun-15 |
| 16541 | 2 | 3 | | | III-1 | Pet100 | 100131801 | 4-May-15 |
| 16542 | 2 | 3 | | | III-1 | Pet112 | 5188 | 23-May-15 |
| 16544 | 2 | 3 | | | III-1 | Pet2 | | |
| 16548 | 2 | 3 | | | III-1 | Pex11b | 8799 | 4-May-15 |
| 16550 | 2 | 3 | | | III-1 | Pex12 | 5193 | 23-May-15 |
| 16554 | 2 | 3 | | | III-1 | Pex19 | 5824 | 4-May-15 |
| 16558 | 2 | 3 | | | III-1 | Pex5 | 5830 | 23-May-15 |
| 16564 | 2 | 3 | | | III-1 | Pfdn1 | 5201 | 4-May-15 |
| 16566 | 2 | 3 | | | III-1 | Pfdn4 | 5203 | 4-May-15 |
| 16567 | 2 | 3 | | | III-1 | Pfdn5 | 5204 | 4-May-15 |
| 16569 | 2 | 3 | | | III-1 | Pfkfb2 | 5208 | 4-May-15 |
| 16572 | 2 | 3 | | | III-1 | Pfkl | 5211 | 4-May-15 |
| 16577 | 2 | 3 | | | III-1 | Pfn3 | 345456 | 4-May-15 |
| 16587 | 2 | 3 | | | III-1 | Pgbd1 | 84547 | 4-May-15 |
| 16592 | 2 | 3 | | | III-1 | Pggt1b | 5229 | 4-May-15 |
| 16599 | 2 | 3 | | | III-1 | Pglyrp4 | 57115 | 4-May-15 |
| 16600 | 2 | 3 | | | III-1 | Pgm1 | 5236 | 23-May-15 |
| 16601 | 2 | 3 | | | III-1 | Pgm2 | 55276 | 4-May-15 |
| 16603 | 2 | 3 | | | III-1 | Pgm3 | 5238 | 4-May-15 |
| 16606 | 2 | 3 | | | III-1 | Pgpep1 | 54858 | 12-May-15 |
| 16607 | 2 | 3 | | | III-1 | Pgpep1l | 145814 | 4-May-15 |
| 16609 | 2 | 3 | | | III-1 | Pgr15l | | |
| 16611 | 2 | 3 | | | III-1 | Pgrmc2 | 10424 | 4-May-15 |
| 16613 | 2 | 3 | | | III-1 | Phactr1 | 221692 | 4-May-15 |
| 16614 | 2 | 3 | | | III-1 | Phactr2 | 9749 | 4-May-15 |
| 16616 | 2 | 3 | | | III-1 | Phactr4 | 65979 | 4-May-15 |
| 16621 | 2 | 3 | | | III-1 | Phc2 | 1912 | 14-May-15 |
| 16623 | 2 | 3 | | | III-1 | Phex | 5251 | 23-May-15 |
| 16625 | 2 | 3 | | | III-1 | Phf10 | 55274 | 4-May-15 |
| 16626 | 2 | 3 | | | III-1 | Phf11a | | |
| 16627 | 2 | 3 | | | III-1 | Phf11b | | |
| 16629 | 2 | 3 | | | III-1 | Phf11d | | |
| 16639 | 2 | 3 | | | III-1 | Phf23 | 79142 | 4-May-15 |
| 16640 | 2 | 3 | | | III-1 | Phf3 | 23469 | 12-May-15 |
| 16641 | 2 | 3 | | | III-1 | Phf5a | 84844 | 4-May-15 |
| 16644 | 2 | 3 | | | III-1 | Phf8 | 23133 | 4-May-15 |
| 16647 | 2 | 3 | | | III-1 | Phip | 55023 | 4-May-15 |
| 16657 | 2 | 3 | | | III-1 | Phldb2 | 90102 | 4-May-15 |
| 16658 | 2 | 3 | | | III-1 | Phldb3 | 653583 | 4-May-15 |
| 16662 | 2 | 3 | | | III-1 | Phospho2 | 493911 | 4-May-15 |
| 16666 | 2 | 3 | | | III-1 | Phrf1 | 57661 | 4-May-15 |
| 16670 | 2 | 3 | | | III-1 | Phxr4 | | |
| 16672 | 2 | 3 | | | III-1 | Phyhd1 | 254295 | 4-May-15 |
| 16676 | 2 | 3 | | | III-1 | Pi15 | 51050 | 4-May-15 |
| 16678 | 2 | 3 | | | III-1 | Pi4k2a | 55361 | 28-May-15 |
| 16680 | 2 | 3 | | | III-1 | Pi4ka | 5297 | 4-May-15 |
| 16681 | 2 | 3 | | | III-1 | Pi4kb | 5298 | 4-May-15 |
| 16686 | 2 | 3 | | | III-1 | Pias4 | 51588 | 4-May-15 |
| 16692 | 2 | 3 | | | III-1 | Piezo1 | 9780 | 10-May-15 |
| 16693 | 2 | 3 | | | III-1 | Piezo2 | 63895 | 21-May-15 |
| 16698 | 2 | 3 | | | III-1 | Pigc | 5279 | 4-May-15 |
| 16700 | 2 | 3 | | | III-1 | Pigg | 54872 | 4-May-15 |
| 16702 | 2 | 3 | | | III-1 | Pigk | 10026 | 4-May-15 |
| 16707 | 2 | 3 | | | III-1 | Pigp | 51227 | 4-May-15 |
| 16716 | 2 | 3 | | | III-1 | Pigyl | | |
| 16719 | 2 | 3 | | | III-1 | Pih1d2 | 120379 | 4-May-15 |
| 16720 | 2 | 3 | | | III-1 | Pih1d3 | 139212 | 4-May-15 |
| 16723 | 2 | 3 | | | III-1 | Pik3c2b | 5287 | 4-May-15 |
| 16726 | 2 | 3 | | | III-1 | Pik3ca | 5290 | 31-May-15 |
| 16730 | 2 | 3 | | | III-1 | Pik3ip1 | 113791 | 4-May-15 |
| 16732 | 2 | 3 | | | III-1 | Pik3r2 | 5296 | 28-May-15 |
| 16734 | 2 | 3 | | | III-1 | Pik3r4 | 30849 | 3-May-15 |
| 16741 | 2 | 3 | | | III-1 | Pim1 | 5292 | 7-Jun-15 |
| 16743 | 2 | 3 | | | III-1 | Pim3 | 415116 | 4-May-15 |
| 16744 | 2 | 3 | | | III-1 | Pin1 | 5300 | 7-Jun-15 |
| 16749 | 2 | 3 | | | III-1 | Pinlyp | 390940 | 4-May-15 |
| 16750 | 2 | 3 | | | III-1 | Pinx1 | 54984 | 31-May-15 |
| 16754 | 2 | 3 | | | III-1 | Pip4k2c | 79837 | 4-May-15 |
| 16755 | 2 | 3 | | | III-1 | Pip5k1a | 8394 | 4-May-15 |
| 16757 | 2 | 3 | | | III-1 | Pip5k1c | 23396 | 4-May-15 |
| 16771 | 2 | 3 | | | III-1 | Pisd-ps2 | | |
| 16778 | 2 | 3 | | | III-1 | Pitpnm2 | 57605 | 12-May-15 |
| 16782 | 2 | 3 | | | III-1 | Pitx1 | 5307 | 28-May-15 |
| 16785 | 2 | 3 | | | III-1 | Piwil1 | 9271 | 4-May-15 |
| 16789 | 2 | 3 | | | III-1 | Pja2 | 9867 | 4-May-15 |
| 16791 | 2 | 3 | | | III-1 | Pkd1l2 | 114780 | 12-May-15 |
| 16794 | 2 | 3 | | | III-1 | Pkd2l1 | 9033 | 12-May-15 |
| 16796 | 2 | 3 | | | III-1 | Pkdcc | 91461 | 4-May-15 |

Fig.22 - 77

| | | | | | | | | |
|---|---|---|---|---|---|---|---|---|
| 16797 | 2 | 3 | | | III-1 | Pkdrej | 10343 | 4-May-15 |
| 16802 | 2 | 3 | | | III-1 | Pkig | 11142 | 4-May-15 |
| 16806 | 2 | 3 | | | III-1 | Pkn1 | 5585 | 4-May-15 |
| 16807 | 2 | 3 | | | III-1 | Pkn2 | 5586 | 4-May-15 |
| 16812 | 2 | 3 | | | III-1 | Pkp2 | 5318 | 23-May-15 |
| 16814 | 2 | 3 | | | III-1 | Pkp4 | 8502 | 4-May-15 |
| 16819 | 2 | 3 | | | III-1 | Pla2g12b | 84647 | 4-May-15 |
| 16829 | 2 | 3 | | | III-1 | Pla2g4a | 5321 | 12-May-15 |
| 16830 | 2 | 3 | | | III-1 | Pla2g4b | 100137049 | 4-May-15 |
| 16834 | 2 | 3 | | | III-1 | Pla2g4f | 255189 | 4-May-15 |
| 16836 | 2 | 3 | | | III-1 | Pla2g6 | 8398 | 23-May-15 |
| 16838 | 2 | 3 | | | III-1 | Pla2r1 | 22925 | 24-May-15 |
| 16845 | 2 | 3 | | | III-1 | Plag1 | 5324 | 28-May-15 |
| 16847 | 2 | 3 | | | III-1 | Plagl2 | 5326 | 31-May-15 |
| 16848 | 2 | 3 | | | III-1 | Plat | 5327 | 29-May-15 |
| 16849 | 2 | 3 | | | III-1 | Plau | 5328 | 24-May-15 |
| 16850 | 2 | 3 | | | III-1 | Plaur | 5329 | 17-May-15 |
| 16859 | 2 | 3 | | | III-1 | Plcd3 | 113026 | 4-May-15 |
| 16860 | 2 | 3 | | | III-1 | Plcd4 | 84812 | 4-May-15 |
| 16862 | 2 | 3 | | | III-1 | Plcg1 | 5335 | 31-May-15 |
| 16863 | 2 | 3 | | | III-1 | Plcg2 | 5336 | 12-May-15 |
| 16864 | 2 | 3 | | | III-1 | Plch1 | 23007 | 4-May-15 |
| 16866 | 2 | 3 | | | III-1 | Plcl1 | 5334 | 4-May-15 |
| 16869 | 2 | 3 | | | III-1 | Plcxd2 | 257068 | 4-May-15 |
| 16873 | 2 | 3 | | | III-1 | Pld2 | 5338 | 31-May-15 |
| 16876 | 2 | 3 | | | III-1 | Pld5 | 200150 | 4-May-15 |
| 16878 | 2 | 3 | | | III-1 | Pldi | | |
| 16882 | 2 | 3 | | | III-1 | Plekha1 | 59338 | 17-May-15 |
| 16883 | 2 | 3 | | | III-1 | Plekha2 | 59339 | 12-May-15 |
| 16885 | 2 | 3 | | | III-1 | Plekha4 | 57664 | 4-May-15 |
| 16886 | 2 | 3 | | | III-1 | Plekha5 | 54477 | 12-May-15 |
| 16888 | 2 | 3 | | | III-1 | Plekha7 | 144100 | 4-May-15 |
| 16891 | 2 | 3 | | | III-1 | Plekhb2 | 55041 | 12-May-15 |
| 16894 | 2 | 3 | | | III-1 | Plekhf1 | 79156 | 21-May-15 |
| 16895 | 2 | 3 | | | III-1 | Plekhf2 | 79666 | 4-May-15 |
| 16898 | 2 | 3 | | | III-1 | Plekhg3 | 26030 | 21-May-15 |
| 16903 | 2 | 3 | | | III-1 | Plekhh2 | 130271 | 4-May-15 |
| 16905 | 2 | 3 | | | III-1 | Plekhj1 | 55111 | 4-May-15 |
| 16906 | 2 | 3 | | | III-1 | Plekhm1 | 9842 | 12-May-15 |
| 16910 | 2 | 3 | | | III-1 | Plekho1 | 51177 | 12-May-15 |
| 16915 | 2 | 3 | | | III-1 | Plg | 5340 | 12-May-15 |
| 16922 | 2 | 3 | | | III-1 | Plk1 | 5347 | 24-May-15 |
| 16925 | 2 | 3 | | | III-1 | Plk4 | 10733 | 24-May-15 |
| 16928 | 2 | 3 | | | III-1 | Pln | 5350 | 23-May-15 |
| 16931 | 2 | 3 | | | III-1 | Plod3 | 8985 | 4-May-15 |
| 16933 | 2 | 3 | | | III-1 | Plp2 | 5355 | 4-May-15 |
| 16934 | 2 | 3 | | | III-1 | Plrg1 | 5356 | 4-May-15 |
| 16935 | 2 | 3 | | | III-1 | Pls1 | 5357 | 4-May-15 |
| 16937 | 2 | 3 | | | III-1 | Plscr1 | 5359 | 28-May-15 |
| 16938 | 2 | 3 | | | III-1 | Plscr2 | 57047 | 4-May-15 |
| 16939 | 2 | 3 | | | III-1 | Plscr3 | 57048 | 4-May-15 |
| 16941 | 2 | 3 | | | III-1 | Plscr5 | 389158 | 12-May-15 |
| 16944 | 2 | 3 | | | III-1 | Plxdc1 | 57125 | 4-May-15 |
| 16948 | 2 | 3 | | | III-1 | Plxna3 | 55558 | 12-May-15 |
| 16950 | 2 | 3 | | | III-1 | Plxna4os1 | | |
| 16953 | 2 | 3 | | | III-1 | Plxnb3 | 5365 | 4-May-15 |
| 16958 | 2 | 3 | | | III-1 | Pmaip1 | 5366 | 4-May-15 |
| 16959 | 2 | 3 | | | III-1 | Pmch | 5367 | 4-May-15 |
| 16963 | 2 | 3 | | | III-1 | Pmfbp1 | 83449 | 4-May-15 |
| 16967 | 2 | 3 | | | III-1 | Pmm2 | 5373 | 23-May-15 |
| 16969 | 2 | 3 | | | III-1 | Pmp22 | 5376 | 7-Jun-15 |
| 16970 | 2 | 3 | | | III-1 | Pmpca | 23203 | 4-May-15 |
| 16984 | 2 | 3 | | | III-1 | Pnma2 | 10687 | 4-May-15 |
| 16986 | 2 | 3 | | | III-1 | Pnma5 | 114824 | 12-May-15 |
| 16990 | 2 | 3 | | | III-1 | Pnn | 5411 | 4-May-15 |
| 16992 | 2 | 3 | | | III-1 | Pnoc | 5368 | 12-May-15 |
| 16995 | 2 | 3 | | | III-1 | Pnpla1 | 285848 | 7-Jun-15 |
| 16997 | 2 | 3 | | | III-1 | Pnpla3 | 80339 | 24-May-15 |
| 17001 | 2 | 3 | | | III-1 | Pnpla8 | 50640 | 31-May-15 |
| 17003 | 2 | 3 | | | III-1 | Pnpt1 | 87178 | 4-May-15 |
| 17004 | 2 | 3 | | | III-1 | Pnrc1 | 10957 | 12-May-15 |
| 17005 | 2 | 3 | | | III-1 | Pnrc2 | 55629 | 12-May-15 |
| 17007 | 2 | 3 | | | III-1 | Poc1b | 282809 | 12-May-15 |
| 17011 | 2 | 3 | | | III-1 | Podxl | 5420 | 12-May-15 |
| 17012 | 2 | 3 | | | III-1 | Podxl2 | 50512 | 4-May-15 |
| 17013 | 2 | 3 | | | III-1 | Pof1b | 79983 | 12-May-15 |
| 17017 | 2 | 3 | | | III-1 | Poglut1 | 56983 | 4-May-15 |
| 17022 | 2 | 3 | | | III-1 | Pold1 | 5424 | 21-May-15 |
| 17024 | 2 | 3 | | | III-1 | Pold3 | 10714 | 4-May-15 |
| 17031 | 2 | 3 | | | III-1 | Pole4 | 56655 | 4-May-15 |
| 17034 | 2 | 3 | | | III-1 | Polh | 5429 | 7-Jun-15 |
| 17037 | 2 | 3 | | | III-1 | Poll | 27343 | 21-May-15 |
| 17044 | 2 | 3 | | | III-1 | Polr1d | 51082 | 24-May-15 |
| 17046 | 2 | 3 | | | III-1 | Polr2a | 5430 | 17-May-15 |
| 17052 | 2 | 3 | | | III-1 | Polr2g | 5436 | 4-May-15 |
| 17053 | 2 | 3 | | | III-1 | Polr2h | 5437 | 2-Jun-15 |
| 17054 | 2 | 3 | | | III-1 | Polr2i | 5438 | 28-May-15 |
| 17055 | 2 | 3 | | | III-1 | Polr2j | 5439 | 4-May-15 |
| 17062 | 2 | 3 | | | III-1 | Polr3d | 661 | 21-May-15 |
| 17063 | 2 | 3 | | | III-1 | Polr3e | 55718 | 4-May-15 |
| 17064 | 2 | 3 | | | III-1 | Polr3f | 10621 | 4-May-15 |
| 17066 | 2 | 3 | | | III-1 | Polr3gl | 84265 | 4-May-15 |
| 17070 | 2 | 3 | | | III-1 | Pom121 | 9883 | 7-Jun-15 |
| 17071 | 2 | 3 | | | III-1 | Pom121l12 | 285877 | 4-May-15 |
| 17076 | 2 | 3 | | | III-1 | Pomk | 84197 | 4-May-15 |
| 17078 | 2 | 3 | | | III-1 | Pomt1 | 10585 | 23-May-15 |
| 17081 | 2 | 3 | | | III-1 | Pon2 | 5445 | 31-May-15 |

| | | | | | | | | |
|---|---|---|---|---|---|---|---|---|
| 17083 | 2 | 3 | | | III-1 | Pop1 | 10940 | 7-Jun-15 |
| 17085 | 2 | 3 | | | III-1 | Pop5 | 51367 | 4-May-15 |
| 17086 | 2 | 3 | | | III-1 | Pop7 | 10248 | 4-May-15 |
| 17098 | 2 | 3 | | | III-1 | Pou2f2 | 5452 | 4-May-15 |
| 17101 | 2 | 3 | | | III-1 | Pou3f2 | 5454 | 4-May-15 |
| 17104 | 2 | 3 | | | III-1 | Pou3f4 | 5456 | 23-May-15 |
| 17114 | 2 | 3 | | | III-1 | Ppa2 | 27068 | 12-May-15 |
| 17115 | 2 | 3 | | | III-1 | Ppan | 56342 | 4-May-15 |
| 17117 | 2 | 3 | | | III-1 | Ppap2b | 8613 | 21-May-15 |
| 17119 | 2 | 3 | | | III-1 | Ppapdc1a | 196051 | 4-May-15 |
| 17120 | 2 | 3 | | | III-1 | Ppapdc1b | 84513 | 4-May-15 |
| 17121 | 2 | 3 | | | III-1 | Ppapdc2 | 403313 | 23-May-15 |
| 17126 | 2 | 3 | | | III-1 | Ppargc1a | 10891 | 24-May-15 |
| 17128 | 2 | 3 | | | III-1 | Ppat | 5471 | 7-Jun-15 |
| 17133 | 2 | 3 | | | III-1 | Ppef1 | 5475 | 4-May-15 |
| 17138 | 2 | 3 | | | III-1 | Ppfia4 | 8497 | 4-May-15 |
| 17140 | 2 | 3 | | | III-1 | Ppfibp2 | 8495 | 4-May-15 |
| 17141 | 2 | 3 | | | III-1 | Pphln1 | 51535 | 4-May-15 |
| 17144 | 2 | 3 | | | III-1 | Ppic | 5480 | 4-May-15 |
| 17149 | 2 | 3 | | | III-1 | Ppig | 9360 | 4-May-15 |
| 17157 | 2 | 3 | | | III-1 | Ppip5k2 | 23262 | 4-May-15 |
| 17167 | 2 | 3 | | | III-1 | Ppm1k | 152926 | 4-May-15 |
| 17168 | 2 | 3 | | | III-1 | Ppm1l | 151742 | 4-May-15 |
| 17169 | 2 | 3 | | | III-1 | Ppm1m | 132160 | 4-May-15 |
| 17171 | 2 | 3 | | | III-1 | Ppme1 | 51400 | 4-May-15 |
| 17181 | 2 | 3 | | | III-1 | Ppp1r13b | 23368 | 17-May-15 |
| 17183 | 2 | 3 | | | III-1 | Ppp1r14a | 94274 | 4-May-15 |
| 17188 | 2 | 3 | | | III-1 | Ppp1r15b | 84919 | 4-May-15 |
| 17192 | 2 | 3 | | | III-1 | Ppp1r18 | 170954 | 4-May-15 |
| 17200 | 2 | 3 | | | III-1 | Ppp1r2-ps3 | | |
| 17201 | 2 | 3 | | | III-1 | Ppp1r2-ps7 | | |
| 17205 | 2 | 3 | | | III-1 | Ppp1r36 | 145376 | 4-May-15 |
| 17206 | 2 | 3 | | | III-1 | Ppp1r37 | 284352 | 4-May-15 |
| 17210 | 2 | 3 | | | III-1 | Ppp1r3d | 5509 | 4-May-15 |
| 17213 | 2 | 3 | | | III-1 | Ppp1r3fos | | |
| 17215 | 2 | 3 | | | III-1 | Ppp1r42 | 286187 | 4-May-15 |
| 17219 | 2 | 3 | | | III-1 | Ppp1r9b | 84687 | 4-May-15 |
| 17226 | 2 | 3 | | | III-1 | Ppp2r2c | 5522 | 12-May-15 |
| 17227 | 2 | 3 | | | III-1 | Ppp2r2cos | | |
| 17230 | 2 | 3 | | | III-1 | Ppp2r3c | 55012 | 20-May-15 |
| 17231 | 2 | 3 | | | III-1 | Ppp2r3d | | |
| 17239 | 2 | 3 | | | III-1 | Ppp3cb | 5532 | 4-May-15 |
| 17246 | 2 | 3 | | | III-1 | Ppp4r2 | 151987 | 4-May-15 |
| 17250 | 2 | 3 | | | III-1 | Ppp6r1 | 22870 | 4-May-15 |
| 17252 | 2 | 3 | | | III-1 | Ppp6r3 | 55291 | 4-May-15 |
| 17254 | 2 | 3 | | | III-1 | Ppt1 | 5538 | 23-May-15 |
| 17259 | 2 | 3 | | | III-1 | Pqbp1 | 10084 | 4-May-15 |
| 17260 | 2 | 3 | | | III-1 | Pqlc1 | 80148 | 4-May-15 |
| 17261 | 2 | 3 | | | III-1 | Pqlc2 | 54896 | 4-May-15 |
| 17262 | 2 | 3 | | | III-1 | Pqlc3 | 130814 | 4-May-15 |
| 17264 | 2 | 3 | | | III-1 | Praf2 | 11230 | 12-May-15 |
| 17265 | 2 | 3 | | | III-1 | Pram1 | 84106 | 24-May-15 |
| 17266 | 2 | 3 | | | III-1 | Prame | 23532 | 12-May-15 |
| 17283 | 2 | 3 | | | III-1 | Prdm1 | 639 | 24-May-15 |
| 17284 | 2 | 3 | | | III-1 | Prdm10 | 56980 | 4-May-15 |
| 17285 | 2 | 3 | | | III-1 | Prdm11 | 56981 | 13-May-15 |
| 17297 | 2 | 3 | | | III-1 | Prdx1 | 5052 | 4-May-15 |
| 17299 | 2 | 3 | | | III-1 | Prdx3 | 10935 | 4-May-15 |
| 17300 | 2 | 3 | | | III-1 | Prdx4 | 10549 | 4-May-15 |
| 17301 | 2 | 3 | | | III-1 | Prdx5 | 25824 | 4-May-15 |
| 17303 | 2 | 3 | | | III-1 | Prdx6b | | |
| 17304 | 2 | 3 | | | III-1 | Preb | 10113 | 4-May-15 |
| 17309 | 2 | 3 | | | III-1 | Prepl | 9581 | 4-May-15 |
| 17310 | 2 | 3 | | | III-1 | Prex1 | 57580 | 4-May-15 |
| 17312 | 2 | 3 | | | III-1 | Prf1 | 5551 | 7-Jun-15 |
| 17314 | 2 | 3 | | | III-1 | Prg3 | 10394 | 4-May-15 |
| 17317 | 2 | 3 | | | III-1 | Prickle1 | 144165 | 23-May-15 |
| 17321 | 2 | 3 | | | III-1 | Prim1 | 5557 | 17-May-15 |
| 17322 | 2 | 3 | | | III-1 | Prim2 | 5558 | 17-May-15 |
| 17323 | 2 | 3 | | | III-1 | Prima1 | 145270 | 4-May-15 |
| 17325 | 2 | 3 | | | III-1 | Prkaa1 | 5562 | 31-May-15 |
| 17328 | 2 | 3 | | | III-1 | Prkab2 | 5565 | 12-May-15 |
| 17333 | 2 | 3 | | | III-1 | Prkag2os1 | | |
| 17337 | 2 | 3 | | | III-1 | Prkar2a | 5576 | 4-May-15 |
| 17340 | 2 | 3 | | | III-1 | Prkcb | 5579 | 24-May-15 |
| 17342 | 2 | 3 | | | III-1 | Prkcdbp | 112464 | 4-May-15 |
| 17343 | 2 | 3 | | | III-1 | Prkce | 5581 | 10-May-15 |
| 17345 | 2 | 3 | | | III-1 | Prkch | 5583 | 4-May-15 |
| 17346 | 2 | 3 | | | III-1 | Prkci | 5584 | 17-May-15 |
| 17348 | 2 | 3 | | | III-1 | Prkcsh | 5589 | 12-May-15 |
| 17350 | 2 | 3 | | | III-1 | Prkd1 | 5587 | 4-May-15 |
| 17393 | 2 | 3 | | | III-1 | Prmt1 | 3276 | 12-May-15 |
| 17396 | 2 | 3 | | | III-1 | Prmt3 | 10196 | 4-May-15 |
| 17401 | 2 | 3 | | | III-1 | Prn | 64428 | 12-May-15 |
| 17409 | 2 | 3 | | | III-1 | Prodh2 | 58510 | 7-Jun-15 |
| 17417 | 2 | 3 | | | III-1 | Prop1 | 5626 | 23-May-15 |
| 17419 | 2 | 3 | | | III-1 | Pros1 | 5627 | 12-May-15 |
| 17420 | 2 | 3 | | | III-1 | Prosc | 11212 | 4-May-15 |
| 17423 | 2 | 3 | | | III-1 | Prox1 | 5629 | 4-May-15 |
| 17425 | 2 | 3 | | | III-1 | Proz | 8858 | 4-May-15 |
| 17427 | 2 | 3 | | | III-1 | Prpf18 | 8559 | 12-May-15 |
| 17432 | 2 | 3 | | | III-1 | Prpf38b | 55119 | 4-May-15 |
| 17436 | 2 | 3 | | | III-1 | Prpf40b | 25766 | 4-May-15 |
| 17437 | 2 | 3 | | | III-1 | Prpf4b | 8899 | 4-May-15 |
| 17438 | 2 | 3 | | | III-1 | Prpf6 | 24148 | 23-May-15 |
| 17441 | 2 | 3 | | | III-1 | Prph2 | 5961 | 23-May-15 |
| 17444 | 2 | 3 | | | III-1 | Prps1l1 | 221823 | 4-May-15 |

Fig.22 - 78

| | | | | | | | | |
|---|---|---|---|---|---|---|---|---|
| 17452 | 2 | 3 | | | III-1 | Prr14 | 78994 | 4-May-15 |
| 17457 | 2 | 3 | | | III-1 | Prr18 | 285800 | 4-May-15 |
| 17463 | 2 | 3 | | | III-1 | Prr3 | 80742 | 7-Jun-15 |
| 17468 | 2 | 3 | | | III-1 | Prr5l | 79899 | 4-May-15 |
| 17471 | 2 | 3 | | | III-1 | Prrc1 | 133619 | 4-May-15 |
| 17476 | 2 | 3 | | | III-1 | Prrg2 | 5639 | 4-May-15 |
| 17477 | 2 | 3 | | | III-1 | Prrg3 | 79057 | 14-May-15 |
| 17478 | 2 | 3 | | | III-1 | Prrg4 | 79056 | 4-May-15 |
| 17480 | 2 | 3 | | | III-1 | Prrt2 | 112476 | 31-May-15 |
| 17481 | 2 | 3 | | | III-1 | Prrt3 | 285368 | 12-May-15 |
| 17483 | 2 | 3 | | | III-1 | Prrx1 | 5396 | 23-May-15 |
| 17488 | 2 | 3 | | | III-1 | Prss16 | 10279 | 4-May-15 |
| 17492 | 2 | 3 | | | III-1 | Prss23 | 11098 | 12-May-15 |
| 17503 | 2 | 3 | | | III-1 | Prss37 | 136242 | 4-May-15 |
| 17513 | 2 | 3 | | | III-1 | Prss48 | 345062 | 4-May-15 |
| 17515 | 2 | 3 | | | III-1 | Prss51 | 346702 | 17-Mar-15 |
| 17524 | 2 | 3 | | | III-1 | Prtg | 283659 | 4-May-15 |
| 17527 | 2 | 3 | | | III-1 | Prune2 | 158471 | 12-May-15 |
| 17529 | 2 | 3 | | | III-1 | Psap | 5660 | 7-Jun-15 |
| 17536 | 2 | 3 | | | III-1 | Psd4 | 23550 | 4-May-15 |
| 17539 | 2 | 3 | | | III-1 | Psenen | 55851 | 31-May-15 |
| 17541 | 2 | 3 | | | III-1 | Psg17 | | |
| 17549 | 2 | 3 | | | III-1 | Psg26 | | |
| 17553 | 2 | 3 | | | III-1 | Psg-ps1 | | |
| 17557 | 2 | 3 | | | III-1 | Psma2 | 5683 | 4-May-15 |
| 17559 | 2 | 3 | | | III-1 | Psma4 | 5685 | 4-May-15 |
| 17560 | 2 | 3 | | | III-1 | Psma5 | 5686 | 12-May-15 |
| 17561 | 2 | 3 | | | III-1 | Psma6 | 5687 | 4-May-15 |
| 17562 | 2 | 3 | | | III-1 | Psma7 | 5688 | 4-May-15 |
| 17566 | 2 | 3 | | | III-1 | Psmb11 | 122706 | 4-May-15 |
| 17568 | 2 | 3 | | | III-1 | Psmb3 | 5691 | 4-May-15 |
| 17569 | 2 | 3 | | | III-1 | Psmb4 | 5692 | 3-May-15 |
| 17570 | 2 | 3 | | | III-1 | Psmb5 | 5693 | 4-May-15 |
| 17571 | 2 | 3 | | | III-1 | Psmb6 | 5694 | 4-May-15 |
| 17572 | 2 | 3 | | | III-1 | Psmb7 | 5695 | 21-May-15 |
| 17573 | 2 | 3 | | | III-1 | Psmb8 | 5696 | 12-May-15 |
| 17577 | 2 | 3 | | | III-1 | Psmc3 | 5702 | 4-May-15 |
| 17579 | 2 | 3 | | | III-1 | Psmc4 | 5704 | 4-May-15 |
| 17582 | 2 | 3 | | | III-1 | Psmd1 | 5707 | 4-May-15 |
| 17585 | 2 | 3 | | | III-1 | Psmd12 | 5718 | 2-Jun-15 |
| 17587 | 2 | 3 | | | III-1 | Psmd14 | 10213 | 12-May-15 |
| 17588 | 2 | 3 | | | III-1 | Psmd2 | 5708 | 4-May-15 |
| 17597 | 2 | 3 | | | III-1 | Psme2 | 5721 | 4-May-15 |
| 17598 | 2 | 3 | | | III-1 | Psme2b | | |
| 17604 | 2 | 3 | | | III-1 | Psmg3 | 84262 | 4-May-15 |
| 17606 | 2 | 3 | | | III-1 | Psors1c2 | 170680 | 12-May-15 |
| 17608 | 2 | 3 | | | III-1 | Psph | 5723 | 4-May-15 |
| 17609 | 2 | 3 | | | III-1 | Pspn | 5623 | 12-May-15 |
| 17610 | 2 | 3 | | | III-1 | Psrc1 | 84722 | 4-May-15 |
| 17613 | 2 | 3 | | | III-1 | Pstpip2 | 9050 | 4-May-15 |
| 17614 | 2 | 3 | | | III-1 | Ptafr | 5724 | 4-May-15 |
| 17615 | 2 | 3 | | | III-1 | Ptar1 | 375743 | 4-May-15 |
| 17618 | 2 | 3 | | | III-1 | Ptbp3 | 9991 | 1-Jun-15 |
| 17621 | 2 | 3 | | | III-1 | Ptcd3 | 55037 | 4-May-15 |
| 17626 | 2 | 3 | | | III-1 | Ptchd3 | 374308 | 12-May-15 |
| 17631 | 2 | 3 | | | III-1 | Pten | 5728 | 2-Jun-15 |
| 17633 | 2 | 3 | | | III-1 | Ptf1a | 256297 | 28-May-15 |
| 17642 | 2 | 3 | | | III-1 | Ptges2 | 80142 | 4-May-15 |
| 17645 | 2 | 3 | | | III-1 | Ptgfr | 5737 | 4-May-15 |
| 17646 | 2 | 3 | | | III-1 | Ptgfrn | 5738 | 4-May-15 |
| 17653 | 2 | 3 | | | III-1 | Ptgs2os | | |
| 17659 | 2 | 3 | | | III-1 | Ptk2 | 5747 | 31-May-15 |
| 17662 | 2 | 3 | | | III-1 | Ptk7 | 5754 | 4-May-15 |
| 17663 | 2 | 3 | | | III-1 | Ptma | 5757 | 7-Jun-15 |
| 17668 | 2 | 3 | | | III-1 | Ptp4a2 | 8073 | 12-May-15 |
| 17670 | 2 | 3 | | | III-1 | Ptpdc1 | 138639 | 12-May-15 |
| 17672 | 2 | 3 | | | III-1 | Ptplad1 | 51495 | 4-May-15 |
| 17675 | 2 | 3 | | | III-1 | Ptpmt1 | 114971 | 4-May-15 |
| 17676 | 2 | 3 | | | III-1 | Ptpn1 | 5770 | 17-May-15 |
| 17680 | 2 | 3 | | | III-1 | Ptpn14 | 5784 | 14-May-15 |
| 17681 | 2 | 3 | | | III-1 | Ptpn18 | 26469 | 4-May-15 |
| 17682 | 2 | 3 | | | III-1 | Ptpn2 | 5771 | 24-May-15 |
| 17683 | 2 | 3 | | | III-1 | Ptpn20 | 26095 | 14-May-15 |
| 17686 | 2 | 3 | | | III-1 | Ptpn23 | 25930 | 4-May-15 |
| 17688 | 2 | 3 | | | III-1 | Ptpn4 | 5775 | 4-May-15 |
| 17690 | 2 | 3 | | | III-1 | Ptpn6 | 5777 | 24-May-15 |
| 17693 | 2 | 3 | | | III-1 | Ptpra | 5786 | 17-May-15 |
| 17695 | 2 | 3 | | | III-1 | Ptprc | 5788 | 12-May-15 |
| 17698 | 2 | 3 | | | III-1 | Ptpre | 5791 | 4-May-15 |
| 17700 | 2 | 3 | | | III-1 | Ptprg | 5793 | 12-May-15 |
| 17704 | 2 | 3 | | | III-1 | Ptprm | 5797 | 4-May-15 |
| 17705 | 2 | 3 | | | III-1 | Ptprn | 5798 | 4-May-15 |
| 17706 | 2 | 3 | | | III-1 | Ptprn2 | 5799 | 12-May-15 |
| 17710 | 2 | 3 | | | III-1 | Ptprs | 5802 | 12-May-15 |
| 17716 | 2 | 3 | | | III-1 | Ptrf | 284119 | 3-May-15 |
| 17721 | 2 | 3 | | | III-1 | Pttg1 | 9232 | 17-May-15 |
| 17722 | 2 | 3 | | | III-1 | Pttg1ip | 754 | 12-May-15 |
| 17724 | 2 | 3 | | | III-1 | Ptx4 | 390667 | 4-May-15 |
| 17729 | 2 | 3 | | | III-1 | Purb | 5814 | 4-May-15 |
| 17732 | 2 | 3 | | | III-1 | Pus10 | 150962 | 12-May-15 |
| 17736 | 2 | 3 | | | III-1 | Pusl1 | 126789 | 23-May-15 |
| 17739 | 2 | 3 | | | III-1 | Pvrl1 | 5818 | 12-May-15 |
| 17741 | 2 | 3 | | | III-1 | Pvrl3 | 25945 | 4-May-15 |
| 17743 | 2 | 3 | | | III-1 | Pvt1 | 5820 | 17-May-15 |
| 17744 | 2 | 3 | | | III-1 | Pwp1 | 11137 | 4-May-15 |
| 17748 | 2 | 3 | | | III-1 | Pxdc1 | 221749 | 4-May-15 |
| 17753 | 2 | 3 | | | III-1 | Pxn | 5829 | 7-Jun-15 |
| 17756 | 2 | 3 | | | III-1 | Pycard | 29108 | 4-May-15 |
| 17757 | 2 | 3 | | | III-1 | Pycr1 | 5831 | 12-May-15 |
| 17766 | 2 | 3 | | | III-1 | Pygo2 | 90780 | 4-May-15 |
| 17769 | 2 | 3 | | | III-1 | Pyroxd2 | 84795 | 4-May-15 |
| 17770 | 2 | 3 | | | III-1 | Pyurf | 100996939 | 4-May-15 |
| 17775 | 2 | 3 | | | III-1 | Qk | 9444 | 28-May-15 |
| 17780 | 2 | 3 | | | III-1 | Qrfpr | 84109 | 4-May-15 |
| 17785 | 2 | 3 | | | III-1 | Qsox1 | 5768 | 4-May-15 |
| 17787 | 2 | 3 | | | III-1 | Qtrt1 | 81890 | 4-May-15 |
| 17788 | 2 | 3 | | | III-1 | Qtrtd1 | 79691 | 4-May-15 |
| 17799 | 2 | 3 | | | III-1 | Rab11a | 8766 | 29-May-15 |
| 17805 | 2 | 3 | | | III-1 | Rab11fip4os1 | | |
| 17808 | 2 | 3 | | | III-1 | Rab12 | 201475 | 21-May-15 |
| 17810 | 2 | 3 | | | III-1 | Rab14 | 51552 | 4-May-15 |
| 17813 | 2 | 3 | | | III-1 | Rab18 | 22931 | 24-May-15 |
| 17814 | 2 | 3 | | | III-1 | Rab19 | 401409 | 4-May-15 |
| 17815 | 2 | 3 | | | III-1 | Rab1b | 81876 | 21-May-15 |
| 17817 | 2 | 3 | | | III-1 | Rab21 | 23011 | 4-May-15 |
| 17825 | 2 | 3 | | | III-1 | Rab27b | 5874 | 4-May-15 |
| 17826 | 2 | 3 | | | III-1 | Rab28 | 9364 | 23-May-15 |
| 17828 | 2 | 3 | | | III-1 | Rab2b | 84932 | 4-May-15 |
| 17835 | 2 | 3 | | | III-1 | Rab35 | 11021 | 23-May-15 |
| 17841 | 2 | 3 | | | III-1 | Rab3a | 5864 | 12-May-15 |
| 17842 | 2 | 3 | | | III-1 | Rab3b | 5865 | 21-May-15 |
| 17844 | 2 | 3 | | | III-1 | Rab3d | 9545 | 4-May-15 |
| 17845 | 2 | 3 | | | III-1 | Rab3gap1 | 22930 | 4-May-15 |
| 17849 | 2 | 3 | | | III-1 | Rab40b | 10966 | 4-May-15 |
| 17850 | 2 | 3 | | | III-1 | Rab40c | 57799 | 4-May-15 |
| 17855 | 2 | 3 | | | III-1 | Rab4b | 53916 | 23-May-15 |
| 17862 | 2 | 3 | | | III-1 | Rab7l1 | 8934 | 4-May-15 |
| 17863 | 2 | 3 | | | III-1 | Rab8a | 4218 | 4-May-15 |
| 17866 | 2 | 3 | | | III-1 | Rab9b | 51209 | 4-May-15 |
| 17868 | 2 | 3 | | | III-1 | Rabep1 | 9135 | 4-May-15 |
| 17870 | 2 | 3 | | | III-1 | Rabepk | 10244 | 4-May-15 |
| 17873 | 2 | 3 | | | III-1 | Rabgef1 | 27342 | 31-May-15 |
| 17883 | 2 | 3 | | | III-1 | Racgap1 | 29127 | 3-May-15 |
| 17887 | 2 | 3 | | | III-1 | Rad21 | 5885 | 31-May-15 |
| 17893 | 2 | 3 | | | III-1 | Rad51ap1 | 10635 | 4-May-15 |
| 17896 | 2 | 3 | | | III-1 | Rad51c | 5889 | 23-May-15 |
| 17902 | 2 | 3 | | | III-1 | Rad9a | 5883 | 17-May-15 |
| 17904 | 2 | 3 | | | III-1 | Radil | 55698 | 4-May-15 |
| 17909 | 2 | 3 | | | III-1 | Raet1d | | |
| 17915 | 2 | 3 | | | III-1 | Rai14 | 26064 | 4-May-15 |
| 17922 | 2 | 3 | | | III-1 | Ralgapb | 57148 | 4-May-15 |
| 17924 | 2 | 3 | | | III-1 | Ralgps1 | 9649 | 4-May-15 |
| 17925 | 2 | 3 | | | III-1 | Ralgps2 | 55103 | 4-May-15 |
| 17926 | 2 | 3 | | | III-1 | Raly | 22913 | 12-May-15 |
| 17929 | 2 | 3 | | | III-1 | Ramp2 | 10266 | 4-May-15 |
| 17931 | 2 | 3 | | | III-1 | Ran | 5901 | 23-May-15 |
| 17934 | 2 | 3 | | | III-1 | Ranbp17 | 64901 | 12-May-15 |
| 17936 | 2 | 3 | | | III-1 | Ranbp3 | 8498 | 4-May-15 |
| 17942 | 2 | 3 | | | III-1 | Rap1a | 5906 | 10-May-15 |
| 17945 | 2 | 3 | | | III-1 | Rap1gap2 | 23108 | 4-May-15 |
| 17946 | 2 | 3 | | | III-1 | Rap1gds1 | 5910 | 4-May-15 |
| 17949 | 2 | 3 | | | III-1 | Rap2c | 57826 | 4-May-15 |
| 17952 | 2 | 3 | | | III-1 | Rapgef3 | 10411 | 31-May-15 |
| 17955 | 2 | 3 | | | III-1 | Rapgef6 | 51735 | 12-May-15 |
| 17959 | 2 | 3 | | | III-1 | Rara | 5914 | 17-May-15 |
| 17961 | 2 | 3 | | | III-1 | Rarg | 5916 | 4-May-15 |
| 17965 | 2 | 3 | | | III-1 | Rars2 | 57038 | 4-May-15 |
| 17969 | 2 | 3 | | | III-1 | Rasa4 | 10156 | 17-May-15 |
| 17971 | 2 | 3 | | | III-1 | Rasal2 | 9462 | 4-May-15 |
| 17972 | 2 | 3 | | | III-1 | Rasal3 | 64926 | 4-May-15 |
| 17976 | 2 | 3 | | | III-1 | Rasgef1a | 221002 | 4-May-15 |
| 17981 | 2 | 3 | | | III-1 | Rasgrp1 | 10125 | 12-May-15 |
| 17990 | 2 | 3 | | | III-1 | Rasl12 | 51285 | 12-May-15 |
| 17992 | 2 | 3 | | | III-1 | Rassf1 | 11186 | 17-May-15 |
| 17994 | 2 | 3 | | | III-1 | Rassf2 | 9770 | 28-May-15 |
| 17995 | 2 | 3 | | | III-1 | Rassf3 | 283349 | 7-Jun-15 |
| 17996 | 2 | 3 | | | III-1 | Rassf4 | 83937 | 13-Jun-15 |
| 17997 | 2 | 3 | | | III-1 | Rassf5 | 83593 | 13-Jun-15 |
| 18002 | 2 | 3 | | | III-1 | Raver1 | 125950 | 4-May-15 |
| 18005 | 2 | 3 | | | III-1 | Rax | 30062 | 7-Jun-15 |
| 18006 | 2 | 3 | | | III-1 | Rb1 | 5925 | 7-Jun-15 |
| 18013 | 2 | 3 | | | III-1 | Rbbp7 | 5931 | 4-May-15 |
| 18015 | 2 | 3 | | | III-1 | Rbbp8nl | 140893 | 4-May-15 |
| 18022 | 2 | 3 | | | III-1 | Rbks | 64080 | 4-May-15 |
| 18023 | 2 | 3 | | | III-1 | Rbl1 | 5933 | 4-May-15 |
| 18025 | 2 | 3 | | | III-1 | Rbm10 | 8241 | 12-May-15 |
| 18027 | 2 | 3 | | | III-1 | Rbm12 | 10137 | 4-May-15 |
| 18029 | 2 | 3 | | | III-1 | Rbm12b2 | | |
| 18033 | 2 | 3 | | | III-1 | Rbm15b | 29890 | 4-May-15 |
| 18034 | 2 | 3 | | | III-1 | Rbm17 | 84991 | 21-May-15 |
| 18038 | 2 | 3 | | | III-1 | Rbm22 | 55696 | 2-Jun-15 |
| 18042 | 2 | 3 | | | III-1 | Rbm27 | 54439 | 4-May-15 |
| 18044 | 2 | 3 | | | III-1 | Rbm3 | 5935 | 4-May-15 |
| 18045 | 2 | 3 | | | III-1 | Rbm31y | | |
| 18047 | 2 | 3 | | | III-1 | Rbm34 | 23029 | 4-May-15 |
| 18049 | 2 | 3 | | | III-1 | Rbm39 | 9584 | 4-May-15 |
| 18051 | 2 | 3 | | | III-1 | Rbm4 | 5936 | 17-May-15 |
| 18052 | 2 | 3 | | | III-1 | Rbm41 | 55285 | 4-May-15 |
| 18053 | 2 | 3 | | | III-1 | Rbm42 | 79171 | 4-May-15 |
| 18057 | 2 | 3 | | | III-1 | Rbm46 | 166863 | 4-May-15 |
| 18060 | 2 | 3 | | | III-1 | Rbm48 | 84060 | 4-May-15 |
| 18064 | 2 | 3 | | | III-1 | Rbm7 | 10179 | 4-May-15 |
| 18067 | 2 | 3 | | | III-1 | Rbms2 | 5939 | 4-May-15 |
| 18068 | 2 | 3 | | | III-1 | Rbms3 | 27303 | 4-May-15 |

Fig.22 - 79

| | | | | | | | | |
|---|---|---|---|---|---|---|---|---|
| 18069 | 2 | 3 | | | III-1 | Rbmx | 27316 | 12-May-15 |
| 18071 | 2 | 3 | | | III-1 | Rbmxl1 | 494115 | 4-May-15 |
| 18076 | 2 | 3 | | | III-1 | Rbp3 | 5949 | 7-Jun-15 |
| 18082 | 2 | 3 | | | III-1 | Rbpms2 | 348093 | 4-May-15 |
| 18083 | 2 | 3 | | | III-1 | Rbx1 | 9978 | 2-Jun-15 |
| 18088 | 2 | 3 | | | III-1 | Rcan3 | 11123 | 12-May-15 |
| 18090 | 2 | 3 | | | III-1 | Rcbtb2 | 1102 | 4-May-15 |
| 18095 | 2 | 3 | | | III-1 | Rchy1 | 25898 | 2-Jun-15 |
| 18098 | 2 | 3 | | | III-1 | Rcn2 | 5955 | 7-Jun-15 |
| 18101 | 2 | 3 | | | III-1 | Rcor2 | 283248 | 4-May-15 |
| 18104 | 2 | 3 | | | III-1 | Rcvrn | 5957 | 12-May-15 |
| 18108 | 2 | 3 | | | III-1 | Rdh10 | 157506 | 31-May-15 |
| 18111 | 2 | 3 | | | III-1 | Rdh13 | 112724 | 4-May-15 |
| 18115 | 2 | 3 | | | III-1 | Rdh19 | | |
| 18116 | 2 | 3 | | | III-1 | Rdh5 | 5959 | 4-May-15 |
| 18117 | 2 | 3 | | | III-1 | Rdh7 | | |
| 18120 | 2 | 3 | | | III-1 | Rdm1 | 201299 | 4-May-15 |
| 18123 | 2 | 3 | | | III-1 | Reck | 8434 | 31-May-15 |
| 18126 | 2 | 3 | | | III-1 | Recql5 | 9400 | 21-May-15 |
| 18129 | 2 | 3 | | | III-1 | Reep2 | 51308 | 4-May-15 |
| 18130 | 2 | 3 | | | III-1 | Reep3 | 221035 | 4-May-15 |
| 18132 | 2 | 3 | | | III-1 | Reep5 | 7905 | 12-May-15 |
| 18142 | 2 | 3 | | | III-1 | Rela | 5970 | 29-May-15 |
| 18144 | 2 | 3 | | | III-1 | Rell1 | 768211 | 4-May-15 |
| 18145 | 2 | 3 | | | III-1 | Rell2 | 285613 | 4-May-15 |
| 18147 | 2 | 3 | | | III-1 | Relt | 84957 | 4-May-15 |
| 18154 | 2 | 3 | | | III-1 | Repin1 | 29803 | 17-May-15 |
| 18156 | 2 | 3 | | | III-1 | Reps2 | 9185 | 4-May-15 |
| 18157 | 2 | 3 | | | III-1 | Rer1 | 11079 | 20-May-15 |
| 18160 | 2 | 3 | | | III-1 | Rergl | 79785 | 4-May-15 |
| 18162 | 2 | 3 | | | III-1 | Rest | 5978 | 28-May-15 |
| 18169 | 2 | 3 | | | III-1 | Rev1 | 51455 | 23-May-15 |
| 18170 | 2 | 3 | | | III-1 | Rev3l | 5980 | 12-May-15 |
| 18171 | 2 | 3 | | | III-1 | Rex2 | 25996 | 4-May-15 |
| 18175 | 2 | 3 | | | III-1 | Rfc1 | 5981 | 7-Jun-15 |
| 18177 | 2 | 3 | | | III-1 | Rfc3 | 5983 | 4-May-15 |
| 18178 | 2 | 3 | | | III-1 | Rfc4 | 5984 | 4-May-15 |
| 18179 | 2 | 3 | | | III-1 | Rfc5 | 5985 | 4-May-15 |
| 18188 | 2 | 3 | | | III-1 | Rftn1 | 23180 | 4-May-15 |
| 18190 | 2 | 3 | | | III-1 | Rfwd2 | 64326 | 4-May-15 |
| 18194 | 2 | 3 | | | III-1 | Rfx3 | 5991 | 28-May-15 |
| 18204 | 2 | 3 | | | III-1 | Rgcc | 28984 | 4-May-15 |
| 18205 | 2 | 3 | | | III-1 | Rgl1 | 23179 | 7-Jun-15 |
| 18209 | 2 | 3 | | | III-1 | Rgmb | 285704 | 4-May-15 |
| 18216 | 2 | 3 | | | III-1 | Rgs12 | 6002 | 21-May-15 |
| 18222 | 2 | 3 | | | III-1 | Rgs19 | 10287 | 4-May-15 |
| 18224 | 2 | 3 | | | III-1 | Rgs20 | 8601 | 4-May-15 |
| 18225 | 2 | 3 | | | III-1 | Rgs21 | 431704 | 4-May-15 |
| 18227 | 2 | 3 | | | III-1 | Rgs3 | 5998 | 12-May-15 |
| 18228 | 2 | 3 | | | III-1 | Rgs4 | 5999 | 4-May-15 |
| 18232 | 2 | 3 | | | III-1 | Rgs7bp | 401190 | 4-May-15 |
| 18235 | 2 | 3 | | | III-1 | Rgs9bp | 388531 | 4-May-15 |
| 18239 | 2 | 3 | | | III-1 | Rhbdd2 | 57414 | 4-May-15 |
| 18240 | 2 | 3 | | | III-1 | Rhbdd3 | 25807 | 4-May-15 |
| 18242 | 2 | 3 | | | III-1 | Rhbdf2 | 79651 | 4-May-15 |
| 18243 | 2 | 3 | | | III-1 | Rhbdl1 | 9028 | 4-May-15 |
| 18245 | 2 | 3 | | | III-1 | Rhbdl3 | 162494 | 4-May-15 |
| 18255 | 2 | 3 | | | III-1 | Rhobtb1 | 9886 | 4-May-15 |
| 18256 | 2 | 3 | | | III-1 | Rhobtb2 | 23221 | 21-May-15 |
| 18258 | 2 | 3 | | | III-1 | Rhoc | 389 | 3-May-15 |
| 18260 | 2 | 3 | | | III-1 | Rhof | 54509 | 23-May-15 |
| 18261 | 2 | 3 | | | III-1 | Rhog | 391 | 4-May-15 |
| 18264 | 2 | 3 | | | III-1 | Rhoq | 23433 | 4-May-15 |
| 18265 | 2 | 3 | | | III-1 | Rhot1 | 55288 | 23-May-15 |
| 18289 | 2 | 3 | | | III-1 | Rhox4b | | |
| 18290 | 2 | 3 | | | III-1 | Rhox4c | | |
| 18302 | 2 | 3 | | | III-1 | Rian | 79104 | 12-May-15 |
| 18304 | 2 | 3 | | | III-1 | Ribc2 | 26150 | 4-May-15 |
| 18309 | 2 | 3 | | | III-1 | Rif1 | 55183 | 7-Jun-15 |
| 18313 | 2 | 3 | | | III-1 | Rilpl2 | 196383 | 4-May-15 |
| 18314 | 2 | 3 | | | III-1 | Rimbp2 | 23504 | 4-May-15 |
| 18317 | 2 | 3 | | | III-1 | Rimklb | 57494 | 12-May-15 |
| 18318 | 2 | 3 | | | III-1 | Rims1 | 22999 | 12-May-15 |
| 18320 | 2 | 3 | | | III-1 | Rims3 | 9783 | 28-May-15 |
| 18324 | 2 | 3 | | | III-1 | Rin3 | 79890 | 4-May-15 |
| 18335 | 2 | 3 | | | III-1 | Ripply1 | 92129 | 4-May-15 |
| 18338 | 2 | 3 | | | III-1 | Rit1 | 6016 | 7-Jun-15 |
| 18341 | 2 | 3 | | | III-1 | Rlbp1 | 6017 | 4-May-15 |
| 18349 | 2 | 3 | | | III-1 | Rmdn3 | 55177 | 12-May-15 |
| 18367 | 2 | 3 | | | III-1 | Rnase6 | 6039 | 4-May-15 |
| 18369 | 2 | 3 | | | III-1 | Rnaseh1 | 246243 | 7-Jun-15 |
| 18371 | 2 | 3 | | | III-1 | Rnaseh2b | 79621 | 23-May-15 |
| 18372 | 2 | 3 | | | III-1 | Rnaseh2c | 84153 | 23-May-15 |
| 18373 | 2 | 3 | | | III-1 | Rnasek | 440400 | 21-May-15 |
| 18374 | 2 | 3 | | | III-1 | Rnasel | 6041 | 4-May-15 |
| 18375 | 2 | 3 | | | III-1 | Rnaset2a | | |
| 18376 | 2 | 3 | | | III-1 | Rnaset2b | | |
| 18380 | 2 | 3 | | | III-1 | Rnf10 | 9921 | 4-May-15 |
| 18385 | 2 | 3 | | | III-1 | Rnf113a1 | | |
| 18387 | 2 | 3 | | | III-1 | Rnf114 | 55905 | 4-May-15 |
| 18389 | 2 | 3 | | | III-1 | Rnf121 | 55298 | 4-May-15 |
| 18395 | 2 | 3 | | | III-1 | Rnf13 | 11342 | 1-Jun-15 |
| 18405 | 2 | 3 | | | III-1 | Rnf144b | 255488 | 4-May-15 |
| 18407 | 2 | 3 | | | III-1 | Rnf146 | 81847 | 4-Jun-15 |
| 18410 | 2 | 3 | | | III-1 | Rnf150 | 57484 | 4-May-15 |
| 18411 | 2 | 3 | | | III-1 | Rnf151 | 146310 | 4-May-15 |
| 18414 | 2 | 3 | | | III-1 | Rnf165 | 494470 | 1-Jun-15 |
| 18416 | 2 | 3 | | | III-1 | Rnf167 | 26001 | 4-May-15 |
| 18427 | 2 | 3 | | | III-1 | Rnf187 | 149603 | 12-May-15 |
| 18431 | 2 | 3 | | | III-1 | Rnf20 | 56254 | 2-Jun-15 |
| 18434 | 2 | 3 | | | III-1 | Rnf214 | 257160 | 4-May-15 |
| 18438 | 2 | 3 | | | III-1 | Rnf219 | 79596 | 4-May-15 |
| 18440 | 2 | 3 | | | III-1 | Rnf222 | 643904 | 12-May-15 |
| 18445 | 2 | 3 | | | III-1 | Rnf26 | 79102 | 4-May-15 |
| 18451 | 2 | 3 | | | III-1 | Rnf4 | 6047 | 4-May-15 |
| 18455 | 2 | 3 | | | III-1 | Rnf44 | 22838 | 4-May-15 |
| 18457 | 2 | 3 | | | III-1 | Rnf6 | 6049 | 4-May-15 |
| 18462 | 2 | 3 | | | III-1 | Rngtt | 8732 | 12-May-15 |
| 18465 | 2 | 3 | | | III-1 | Rnmt | 8731 | 21-May-15 |
| 18468 | 2 | 3 | | | III-1 | Rnpep | 6051 | 4-May-15 |
| 18477 | 2 | 3 | | | III-1 | Robo2 | 6092 | 12-May-15 |
| 18480 | 2 | 3 | | | III-1 | Rock1 | 6093 | 31-May-15 |
| 18484 | 2 | 3 | | | III-1 | Romo1 | 140823 | 24-May-15 |
| 18487 | 2 | 3 | | | III-1 | Ror1 | 4919 | 7-Jun-15 |
| 18488 | 2 | 3 | | | III-1 | Ror2 | 4920 | 7-Jun-15 |
| 18489 | 2 | 3 | | | III-1 | Rora | 6095 | 12-May-15 |
| 18490 | 2 | 3 | | | III-1 | Rorb | 6096 | 4-May-15 |
| 18493 | 2 | 3 | | | III-1 | Rp1 | 6101 | 23-May-15 |
| 18497 | 2 | 3 | | | III-1 | Rpa1 | 6117 | 7-Jun-15 |
| 18499 | 2 | 3 | | | III-1 | Rpa3 | 6119 | 4-May-15 |
| 18508 | 2 | 3 | | | III-1 | Rpgr | 6103 | 23-May-15 |
| 18512 | 2 | 3 | | | III-1 | Rph3al | 9501 | 4-May-15 |
| 18513 | 2 | 3 | | | III-1 | Rpia | 22934 | 4-May-15 |
| 18515 | 2 | 3 | | | III-1 | Rpl10a | 4736 | 25-May-15 |
| 18516 | 2 | 3 | | | III-1 | Rpl10l | 140801 | 4-May-15 |
| 18519 | 2 | 3 | | | III-1 | Rpl13 | 6137 | 7-Jun-15 |
| 18520 | 2 | 3 | | | III-1 | Rpl13a | 23521 | 4-May-15 |
| 18521 | 2 | 3 | | | III-1 | Rpl14 | 9045 | 12-May-15 |
| 18522 | 2 | 3 | | | III-1 | Rpl14-ps1 | | |
| 18523 | 2 | 3 | | | III-1 | Rpl15 | 6138 | 12-May-15 |
| 18525 | 2 | 3 | | | III-1 | Rpl18 | 6141 | 21-May-15 |
| 18527 | 2 | 3 | | | III-1 | Rpl19 | 6143 | 21-May-15 |
| 18528 | 2 | 3 | | | III-1 | Rpl21 | 6144 | 7-Jun-15 |
| 18529 | 2 | 3 | | | III-1 | Rpl22 | 6146 | 4-May-15 |
| 18531 | 2 | 3 | | | III-1 | Rpl23 | 9349 | 7-Jun-15 |
| 18532 | 2 | 3 | | | III-1 | Rpl23a | 6147 | 12-May-15 |
| 18533 | 2 | 3 | | | III-1 | Rpl24 | 6152 | 7-Jun-15 |
| 18534 | 2 | 3 | | | III-1 | Rpl26 | 6154 | 4-May-15 |
| 18536 | 2 | 3 | | | III-1 | Rpl27a | 6157 | 4-May-15 |
| 18543 | 2 | 3 | | | III-1 | Rpl32 | 6161 | 4-May-15 |
| 18550 | 2 | 3 | | | III-1 | Rpl36al | 6166 | 12-May-15 |
| 18555 | 2 | 3 | | | III-1 | Rpl39l | 116832 | 4-May-15 |
| 18558 | 2 | 3 | | | III-1 | Rpl41 | 6171 | 12-May-15 |
| 18559 | 2 | 3 | | | III-1 | Rpl5 | 6125 | 23-May-15 |
| 18560 | 2 | 3 | | | III-1 | Rpl6 | 6128 | 12-May-15 |
| 18561 | 2 | 3 | | | III-1 | Rpl7 | 6129 | 5-May-15 |
| 18562 | 2 | 3 | | | III-1 | Rpl7a | 6130 | 7-Jun-15 |
| 18563 | 2 | 3 | | | III-1 | Rpl7l1 | 285855 | 12-May-15 |
| 18566 | 2 | 3 | | | III-1 | Rplp0 | 6175 | 4-May-15 |
| 18567 | 2 | 3 | | | III-1 | Rplp1 | 6176 | 12-May-15 |
| 18569 | 2 | 3 | | | III-1 | Rplp2-ps1 | | |
| 18570 | 2 | 3 | | | III-1 | Rpn1 | 6184 | 7-Jun-15 |
| 18575 | 2 | 3 | | | III-1 | Rpp25l | 138716 | 4-May-15 |
| 18577 | 2 | 3 | | | III-1 | Rpp38 | 10557 | 4-May-15 |
| 18581 | 2 | 3 | | | III-1 | Rprd1b | 58490 | 1-Jun-15 |
| 18591 | 2 | 3 | | | III-1 | Rps13 | 6207 | 12-May-15 |
| 18593 | 2 | 3 | | | III-1 | Rps15 | 6209 | 12-May-15 |
| 18595 | 2 | 3 | | | III-1 | Rps15a-ps4 | | |
| 18596 | 2 | 3 | | | III-1 | Rps15a-ps6 | | |
| 18597 | 2 | 3 | | | III-1 | Rps16 | 6217 | 4-May-15 |
| 18600 | 2 | 3 | | | III-1 | Rps19 | 6223 | 21-May-15 |
| 18601 | 2 | 3 | | | III-1 | Rps19bp1 | 91582 | 4-May-15 |
| 18602 | 2 | 3 | | | III-1 | Rps19-ps3 | | |
| 18603 | 2 | 3 | | | III-1 | Rps2 | 6187 | 4-May-15 |
| 18605 | 2 | 3 | | | III-1 | Rps21 | 6227 | 21-May-15 |
| 18607 | 2 | 3 | | | III-1 | Rps24 | 6229 | 23-May-15 |
| 18611 | 2 | 3 | | | III-1 | Rps27a | 6233 | 17-May-15 |
| 18612 | 2 | 3 | | | III-1 | Rps27l | 51065 | 4-May-15 |
| 18613 | 2 | 3 | | | III-1 | Rps27rt | | |
| 18618 | 2 | 3 | | | III-1 | Rps4l | 128580 | 4-May-15 |
| 18619 | 2 | 3 | | | III-1 | Rps4x | 6191 | 12-May-15 |
| 18620 | 2 | 3 | | | III-1 | Rps5 | 6193 | 12-May-15 |
| 18621 | 2 | 3 | | | III-1 | Rps6 | 6194 | 4-May-15 |
| 18622 | 2 | 3 | | | III-1 | Rps6ka1 | 6195 | 17-May-15 |
| 18624 | 2 | 3 | | | III-1 | Rps6ka3 | 6197 | 23-May-15 |
| 18628 | 2 | 3 | | | III-1 | Rps6kb1 | 6198 | 3-May-15 |
| 18634 | 2 | 3 | | | III-1 | Rps9 | 6203 | 4-May-15 |
| 18635 | 2 | 3 | | | III-1 | Rpsa | 3921 | 21-May-15 |
| 18637 | 2 | 3 | | | III-1 | Rptor | 57521 | 17-May-15 |
| 18640 | 2 | 3 | | | III-1 | Rpusd2 | 27079 | 4-May-15 |
| 18642 | 2 | 3 | | | III-1 | Rpusd4 | 84881 | 4-May-15 |
| 18647 | 2 | 3 | | | III-1 | Rragc | 64121 | 31-May-15 |
| 18649 | 2 | 3 | | | III-1 | Rras | 6237 | 12-May-15 |
| 18650 | 2 | 3 | | | III-1 | Rras2 | 22800 | 4-May-15 |
| 18652 | 2 | 3 | | | III-1 | Rreb1 | 6239 | 4-May-15 |
| 18660 | 2 | 3 | | | III-1 | Rrp12 | 23223 | 4-May-15 |
| 18661 | 2 | 3 | | | III-1 | Rrp15 | 51018 | 4-May-15 |
| 18667 | 2 | 3 | | | III-1 | Rrs1 | 23212 | 4-May-15 |
| 18672 | 2 | 3 | | | III-1 | Rsbn1l | 222194 | 4-May-15 |
| 18674 | 2 | 3 | | | III-1 | Rsf1 | 51773 | 31-May-15 |
| 18677 | 2 | 3 | | | III-1 | Rsl1d1 | 26156 | 4-May-15 |
| 18681 | 2 | 3 | | | III-1 | Rsph3a | | |
| 18686 | 2 | 3 | | | III-1 | Rspo1 | 284654 | 4-May-15 |
| 18687 | 2 | 3 | | | III-1 | Rspo2 | 340419 | 4-May-15 |

Fig.22 - 80

| | | | | | | | | |
|---|---|---|---|---|---|---|---|---|
| 18689 | 2 | 3 | | | III-1 | Rspo4 | 343637 | 4-May-15 |
| 18690 | 2 | 3 | | | III-1 | Rspry1 | 89970 | 20-May-15 |
| 18694 | 2 | 3 | | | III-1 | Rsu1 | 6251 | 1-Jun-15 |
| 18696 | 2 | 3 | | | III-1 | Rtca | 8634 | 12-May-15 |
| 18698 | 2 | 3 | | | III-1 | Rtdr1 | 27156 | 9-May-15 |
| 18709 | 2 | 3 | | | III-1 | Rtn4ip1 | 84816 | 4-May-15 |
| 18717 | 2 | 3 | | | III-1 | Rttn | 25914 | 4-May-15 |
| 18724 | 2 | 3 | | | III-1 | Rundc3a | 10900 | 6-May-15 |
| 18725 | 2 | 3 | | | III-1 | Rundc3b | 154661 | 4-May-15 |
| 18732 | 2 | 3 | | | III-1 | Ruvbl1 | 8607 | 17-May-15 |
| 18733 | 2 | 3 | | | III-1 | Ruvbl2 | 10856 | 17-May-15 |
| 18735 | 2 | 3 | | | III-1 | Rwdd2a | 112611 | 4-May-15 |
| 18736 | 2 | 3 | | | III-1 | Rwdd2b | 10069 | 4-May-15 |
| 18738 | 2 | 3 | | | III-1 | Rwdd4a | 201965 | 12-May-15 |
| 18744 | 2 | 3 | | | III-1 | Rxrb | 6257 | 4-May-15 |
| 18746 | 2 | 3 | | | III-1 | Rybp | 23429 | 2-Jun-15 |
| 18753 | 2 | 3 | | | III-1 | S100a11 | 6282 | 4-May-15 |
| 18754 | 2 | 3 | | | III-1 | S100a13 | 6284 | 12-May-15 |
| 18756 | 2 | 3 | | | III-1 | S100a16 | 140576 | 7-Jun-15 |
| 18758 | 2 | 3 | | | III-1 | S100a3 | 6274 | 4-May-15 |
| 18762 | 2 | 3 | | | III-1 | S100a7a | 338324 | 4-May-15 |
| 18768 | 2 | 3 | | | III-1 | S100z | 170591 | 4-May-15 |
| 18769 | 2 | 3 | | | III-1 | S1pr1 | 1901 | 12-May-15 |
| 18770 | 2 | 3 | | | III-1 | S1pr2 | 9294 | 21-May-15 |
| 18787 | 2 | 3 | | | III-1 | Sall2 | 6297 | 21-May-15 |
| 18795 | 2 | 3 | | | III-1 | Samd15 | 161394 | 4-May-15 |
| 18798 | 2 | 3 | | | III-1 | Samd4b | 55095 | 12-May-15 |
| 18800 | 2 | 3 | | | III-1 | Samd7 | 344658 | 12-May-15 |
| 18802 | 2 | 3 | | | III-1 | Samd9l | 219285 | 12-May-15 |
| 18805 | 2 | 3 | | | III-1 | Samsn1 | 64092 | 4-May-15 |
| 18816 | 2 | 3 | | | III-1 | Sapcd2 | 89958 | 4-May-15 |
| 18819 | 2 | 3 | | | III-1 | Sardh | 1757 | 12-May-15 |
| 18822 | 2 | 3 | | | III-1 | Sars | 6301 | 7-Jun-15 |
| 18829 | 2 | 3 | | | III-1 | Sat1 | 6303 | 13-Jun-15 |
| 18830 | 2 | 3 | | | III-1 | Sat2 | 112483 | 13-Jun-15 |
| 18835 | 2 | 3 | | | III-1 | Saysd1 | 55776 | 12-May-15 |
| 18842 | 2 | 3 | | | III-1 | Sbno1 | 55206 | 4-May-15 |
| 18855 | 2 | 3 | | | III-1 | Scamp2 | 10066 | 4-May-15 |
| 18865 | 2 | 3 | | | III-1 | Scarb2 | 950 | 17-May-15 |
| 18866 | 2 | 3 | | | III-1 | Scarf1 | 8578 | 4-May-15 |
| 18868 | 2 | 3 | | | III-1 | Scarletltr | | |
| 18884 | 2 | 3 | | | III-1 | Scfd1 | 23256 | 21-May-15 |
| 18885 | 2 | 3 | | | III-1 | Scfd2 | 152579 | 4-May-15 |
| 18918 | 2 | 3 | | | III-1 | Sclt1 | 132320 | 12-May-15 |
| 18919 | 2 | 3 | | | III-1 | Scly | 51540 | 4-May-15 |
| 18920 | 2 | 3 | | | III-1 | Scmh1 | 22955 | 4-May-15 |
| 18929 | 2 | 3 | | | III-1 | Scn3a | 6328 | 12-May-15 |
| 18934 | 2 | 3 | | | III-1 | Scn7a | 6332 | 31-May-15 |
| 18935 | 2 | 3 | | | III-1 | Scn8a | 6334 | 21-May-15 |
| 18941 | 2 | 3 | | | III-1 | Sco1 | 6341 | 12-May-15 |
| 18943 | 2 | 3 | | | III-1 | Scoc | 60592 | 4-May-15 |
| 18947 | 2 | 3 | | | III-1 | Scpep1os | | |
| 18949 | 2 | 3 | | | III-1 | Scrib | 23513 | 4-May-15 |
| 18950 | 2 | 3 | | | III-1 | Scrn1 | 9805 | 4-May-15 |
| 18952 | 2 | 3 | | | III-1 | Scrn3 | 79634 | 12-May-15 |
| 18953 | 2 | 3 | | | III-1 | Scrt1 | 83482 | 4-May-15 |
| 18955 | 2 | 3 | | | III-1 | Sct | 6343 | 7-Jun-15 |
| 18958 | 2 | 3 | | | III-1 | Scube2 | 57758 | 4-May-15 |
| 18966 | 2 | 3 | | | III-1 | Sdc2 | 6383 | 31-May-15 |
| 18967 | 2 | 3 | | | III-1 | Sdc3 | 9672 | 4-May-15 |
| 18969 | 2 | 3 | | | III-1 | Sdcbp | 6386 | 10-May-15 |
| 18971 | 2 | 3 | | | III-1 | Sdccag3 | 10807 | 4-May-15 |
| 18978 | 2 | 3 | | | III-1 | Sdhaf1 | 644096 | 4-May-15 |
| 18979 | 2 | 3 | | | III-1 | Sdhaf2 | 54949 | 23-May-15 |
| 18980 | 2 | 3 | | | III-1 | Sdhb | 6390 | 23-May-15 |
| 18981 | 2 | 3 | | | III-1 | Sdhc | 6391 | 23-May-15 |
| 18986 | 2 | 3 | | | III-1 | Sdr16c5 | 195814 | 4-May-15 |
| 18988 | 2 | 3 | | | III-1 | Sdr39u1 | 56948 | 21-May-15 |
| 18990 | 2 | 3 | | | III-1 | Sdr9c7 | 121214 | 4-May-15 |
| 18995 | 2 | 3 | | | III-1 | Sec11a | 23478 | 4-May-15 |
| 18997 | 2 | 3 | | | III-1 | Sec13 | 6396 | 29-May-15 |
| 19003 | 2 | 3 | | | III-1 | Sec16a | 9919 | 12-May-15 |
| 19004 | 2 | 3 | | | III-1 | Sec16b | 89866 | 3-May-15 |
| 19005 | 2 | 3 | | | III-1 | Sec22a | 26984 | 4-May-15 |
| 19006 | 2 | 3 | | | III-1 | Sec22b | 9554 | 4-May-15 |
| 19007 | 2 | 3 | | | III-1 | Sec22c | 9117 | 4-May-15 |
| 19010 | 2 | 3 | | | III-1 | Sec23ip | 11196 | 4-May-15 |
| 19012 | 2 | 3 | | | III-1 | Sec24b | 10427 | 4-May-15 |
| 19015 | 2 | 3 | | | III-1 | Sec31a | 22872 | 10-May-15 |
| 19017 | 2 | 3 | | | III-1 | Sec61a1 | 29927 | 12-May-15 |
| 19018 | 2 | 3 | | | III-1 | Sec61a2 | 55176 | 4-May-15 |
| 19019 | 2 | 3 | | | III-1 | Sec61b | 10952 | 4-May-15 |
| 19020 | 2 | 3 | | | III-1 | Sec61g | 23480 | 4-May-15 |
| 19024 | 2 | 3 | | | III-1 | Secisbp2l | 9728 | 4-May-15 |
| 19025 | 2 | 3 | | | III-1 | Sectm1a | | |
| 19026 | 2 | 3 | | | III-1 | Sectm1b | | |
| 19027 | 2 | 3 | | | III-1 | Seh1l | 81929 | 29-May-15 |
| 19037 | 2 | 3 | | | III-1 | Selo | 83642 | 4-May-15 |
| 19039 | 2 | 3 | | | III-1 | Selplg | 6404 | 12-May-15 |
| 19047 | 2 | 3 | | | III-1 | Sema3g | 56920 | 4-May-15 |
| 19048 | 2 | 3 | | | III-1 | Sema4a | 64218 | 23-May-15 |
| 19049 | 2 | 3 | | | III-1 | Sema4b | 10509 | 4-May-15 |
| 19051 | 2 | 3 | | | III-1 | Sema4d | 10507 | 4-May-15 |
| 19054 | 2 | 3 | | | III-1 | Sema5a | 9037 | 4-May-15 |
| 19058 | 2 | 3 | | | III-1 | Sema6c | 10500 | 4-May-15 |
| 19070 | 2 | 3 | | | III-1 | Sepn1 | 57190 | 23-May-15 |
| 19073 | 2 | 3 | | | III-1 | Sept1 | 1731 | 4-May-15 |

| | | | | | | | | |
|---|---|---|---|---|---|---|---|---|
| 19075 | 2 | 3 | | | III-1 | Sept11 | 55752 | 12-May-15 |
| 19081 | 2 | 3 | | | III-1 | Sept4 | 5414 | 12-May-15 |
| 19083 | 2 | 3 | | | III-1 | Sept6 | 23157 | 4-May-15 |
| 19084 | 2 | 3 | | | III-1 | Sept7 | 989 | 4-May-15 |
| 19086 | 2 | 3 | | | III-1 | Sept9 | 10801 | 23-May-15 |
| 19089 | 2 | 3 | | | III-1 | Serbp1 | 26135 | 4-May-15 |
| 19094 | 2 | 3 | | | III-1 | Serinc1 | 57515 | 28-May-15 |
| 19096 | 2 | 3 | | | III-1 | Serinc3 | 10955 | 12-May-15 |
| 19097 | 2 | 3 | | | III-1 | Serinc4 | 619189 | 14-May-15 |
| 19099 | 2 | 3 | | | III-1 | Serp1 | 27230 | 14-May-15 |
| 19110 | 2 | 3 | | | III-1 | Serpina3a | | |
| 19116 | 2 | 3 | | | III-1 | Serpina3i | | |
| 19125 | 2 | 3 | | | III-1 | Serpina9 | 327657 | 4-May-15 |
| 19126 | 2 | 3 | | | III-1 | Serpinb10 | 5273 | 28-May-15 |
| 19129 | 2 | 3 | | | III-1 | Serpinb13 | 5275 | 12-May-15 |
| 19137 | 2 | 3 | | | III-1 | Serpinb3d | | |
| 19141 | 2 | 3 | | | III-1 | Serpinb6c | | |
| 19144 | 2 | 3 | | | III-1 | Serpinb7 | 8710 | 4-May-15 |
| 19145 | 2 | 3 | | | III-1 | Serpinb8 | 5271 | 4-May-15 |
| 19146 | 2 | 3 | | | III-1 | Serpinb9 | 5272 | 4-May-15 |
| 19147 | 2 | 3 | | | III-1 | Serpinb9b | | |
| 19158 | 2 | 3 | | | III-1 | Serpinf1 | 5176 | 24-May-15 |
| 19161 | 2 | 3 | | | III-1 | Serpinh1 | 871 | 3-May-15 |
| 19170 | 2 | 3 | | | III-1 | Sesn2 | 83667 | 4-May-15 |
| 19175 | 2 | 3 | | | III-1 | Setd1a | 9739 | 24-May-15 |
| 19176 | 2 | 3 | | | III-1 | Setd1b | 23067 | 4-May-15 |
| 19177 | 2 | 3 | | | III-1 | Setd2 | 29072 | 31-May-15 |
| 19178 | 2 | 3 | | | III-1 | Setd3 | 84193 | 4-May-15 |
| 19180 | 2 | 3 | | | III-1 | Setd5 | 55209 | 4-May-15 |
| 19182 | 2 | 3 | | | III-1 | Setd7 | 80854 | 12-May-15 |
| 19184 | 2 | 3 | | | III-1 | Setdb1 | 9869 | 4-May-15 |
| 19186 | 2 | 3 | | | III-1 | Setmar | 6419 | 21-May-15 |
| 19187 | 2 | 3 | | | III-1 | Setx | 23064 | 23-May-15 |
| 19191 | 2 | 3 | | | III-1 | Sf1 | 7536 | 7-Jun-15 |
| 19195 | 2 | 3 | | | III-1 | Sf3b1 | 23451 | 16-Jun-15 |
| 19202 | 2 | 3 | | | III-1 | Sfmbt1 | 51460 | 4-May-15 |
| 19205 | 2 | 3 | | | III-1 | Sfpq | 6421 | 7-Jun-15 |
| 19206 | 2 | 3 | | | III-1 | Sfr1 | 119392 | 4-May-15 |
| 19208 | 2 | 3 | | | III-1 | Sfrp2 | 6423 | 4-May-15 |
| 19213 | 2 | 3 | | | III-1 | Sft2d2 | 375035 | 4-May-15 |
| 19216 | 2 | 3 | | | III-1 | Sftpa1 | 653509 | 23-May-15 |
| 19221 | 2 | 3 | | | III-1 | Sfxn2 | 118980 | 4-May-15 |
| 19222 | 2 | 3 | | | III-1 | Sfxn3 | 81855 | 4-May-15 |
| 19227 | 2 | 3 | | | III-1 | Sgcd | 6444 | 23-May-15 |
| 19234 | 2 | 3 | | | III-1 | Sgk3 | 23678 | 4-May-15 |
| 19235 | 2 | 3 | | | III-1 | Sgms1 | 259230 | 12-May-15 |
| 19237 | 2 | 3 | | | III-1 | Sgol1 | 151648 | 4-May-15 |
| 19242 | 2 | 3 | | | III-1 | Sgsh | 6448 | 17-May-15 |
| 19244 | 2 | 3 | | | III-1 | Sgsm2 | 9905 | 4-May-15 |
| 19246 | 2 | 3 | | | III-1 | Sgta | 6449 | 28-May-15 |
| 19251 | 2 | 3 | | | III-1 | Sh2d1a | 4068 | 23-May-15 |
| 19253 | 2 | 3 | | | III-1 | Sh2d1b2 | | |
| 19255 | 2 | 3 | | | III-1 | Sh2d3c | 10044 | 12-May-15 |
| 19257 | 2 | 3 | | | III-1 | Sh2d4b | 387694 | 4-May-15 |
| 19262 | 2 | 3 | | | III-1 | Sh3bgrl2 | 83699 | 4-May-15 |
| 19263 | 2 | 3 | | | III-1 | Sh3bgrl3 | 83442 | 4-May-15 |
| 19264 | 2 | 3 | | | III-1 | Sh3bp1 | 23616 | 7-Jun-15 |
| 19265 | 2 | 3 | | | III-1 | Sh3bp2 | 6452 | 23-May-15 |
| 19270 | 2 | 3 | | | III-1 | Sh3d21 | 79729 | 4-May-15 |
| 19271 | 2 | 3 | | | III-1 | Sh3gl1 | 6455 | 14-May-15 |
| 19274 | 2 | 3 | | | III-1 | Sh3glb1 | 51100 | 21-May-15 |
| 19276 | 2 | 3 | | | III-1 | Sh3kbp1 | 30011 | 4-May-15 |
| 19279 | 2 | 3 | | | III-1 | Sh3rf1 | 57630 | 23-May-15 |
| 19281 | 2 | 3 | | | III-1 | Sh3rf3 | 344558 | 4-May-15 |
| 19285 | 2 | 3 | | | III-1 | Shank1 | 50944 | 21-May-15 |
| 19287 | 2 | 3 | | | III-1 | Shank3 | 85358 | 23-May-15 |
| 19288 | 2 | 3 | | | III-1 | Sharpin | 81858 | 21-May-15 |
| 19290 | 2 | 3 | | | III-1 | Shbg | 6462 | 4-May-15 |
| 19295 | 2 | 3 | | | III-1 | Shcbp1 | 79801 | 4-May-15 |
| 19298 | 2 | 3 | | | III-1 | She | 126669 | 4-May-15 |
| 19300 | 2 | 3 | | | III-1 | Shfm1 | 7979 | 21-May-15 |
| 19305 | 2 | 3 | | | III-1 | Shisa5 | 51246 | 12-May-15 |
| 19311 | 2 | 3 | | | III-1 | Shmt2 | 6472 | 4-May-15 |
| 19312 | 2 | 3 | | | III-1 | Shoc2 | 8036 | 3-Jun-15 |
| 19315 | 2 | 3 | | | III-1 | Shprh | 257218 | 4-May-15 |
| 19318 | 2 | 3 | | | III-1 | Shroom2 | 357 | 4-May-15 |
| 19320 | 2 | 3 | | | III-1 | Shroom4 | 57477 | 4-May-15 |
| 19323 | 2 | 3 | | | III-1 | Siah1b | | |
| 19324 | 2 | 3 | | | III-1 | Siah2 | 6478 | 24-May-15 |
| 19325 | 2 | 3 | | | III-1 | Siah3 | 283514 | 28-May-15 |
| 19326 | 2 | 3 | | | III-1 | Sidt1 | 54847 | 12-May-15 |
| 19327 | 2 | 3 | | | III-1 | Sidt2 | 51092 | 4-May-15 |
| 19331 | 2 | 3 | | | III-1 | Siglec5 | 8778 | 4-May-15 |
| 19333 | 2 | 3 | | | III-1 | Siglecg | | |
| 19343 | 2 | 3 | | | III-1 | Simc1 | 375484 | 21-May-15 |
| 19344 | 2 | 3 | | | III-1 | Sin3a | 25942 | 4-May-15 |
| 19346 | 2 | 3 | | | III-1 | Sipa1 | 6494 | 12-May-15 |
| 19347 | 2 | 3 | | | III-1 | Sipa1l1 | 26037 | 4-May-15 |
| 19351 | 2 | 3 | | | III-1 | Sirpb1a | | |
| 19358 | 2 | 3 | | | III-1 | Sirt6 | 51548 | 31-May-15 |
| 19364 | 2 | 3 | | | III-1 | Six2 | 10736 | 4-May-15 |
| 19365 | 2 | 3 | | | III-1 | Six3 | 6496 | 22-May-15 |
| 19367 | 2 | 3 | | | III-1 | Six4 | 51804 | 4-May-15 |
| 19369 | 2 | 3 | | | III-1 | Six6 | 4990 | 28-May-15 |
| 19371 | 2 | 3 | | | III-1 | Ska2 | 348235 | 24-May-15 |
| 19374 | 2 | 3 | | | III-1 | Skap2 | 8935 | 4-May-15 |
| 19377 | 2 | 3 | | | III-1 | Skil | 6498 | 12-May-15 |

Fig.22 - 81

| | | | | | | | | |
|---|---|---|---|---|---|---|---|---|
| 19379 | 2 | 3 | | | III-1 | Skint10 | | |
| 19380 | 2 | 3 | | | III-1 | Skint11 | | |
| 19382 | 2 | 3 | | | III-1 | Skint3 | | |
| 19383 | 2 | 3 | | | III-1 | Skint4 | | |
| 19385 | 2 | 3 | | | III-1 | Skint6 | | |
| 19386 | 2 | 3 | | | III-1 | Skint7 | | |
| 19388 | 2 | 3 | | | III-1 | Skint9 | | |
| 19389 | 2 | 3 | | | III-1 | Skiv2l | 6499 | 12-May-15 |
| 19394 | 2 | 3 | | | III-1 | Skp2 | 6502 | 17-May-15 |
| 19396 | 2 | 3 | | | III-1 | Sla2 | 84174 | 4-May-15 |
| 19413 | 2 | 3 | | | III-1 | Slc10a7 | 84068 | 4-May-15 |
| 19415 | 2 | 3 | | | III-1 | Slc11a2 | 4891 | 12-May-15 |
| 19416 | 2 | 3 | | | III-1 | Slc12a1 | 6557 | 12-May-15 |
| 19418 | 2 | 3 | | | III-1 | Slc12a3 | 6559 | 12-May-15 |
| 19419 | 2 | 3 | | | III-1 | Slc12a4 | 6560 | 4-May-15 |
| 19426 | 2 | 3 | | | III-1 | Slc13a2 | 9058 | 4-May-15 |
| 19428 | 2 | 3 | | | III-1 | Slc13a3 | 64849 | 4-May-15 |
| 19430 | 2 | 3 | | | III-1 | Slc13a5 | 284111 | 31-May-15 |
| 19450 | 2 | 3 | | | III-1 | Slc16a8 | 23539 | 4-May-15 |
| 19453 | 2 | 3 | | | III-1 | Slc17a2 | 10246 | 4-May-15 |
| 19454 | 2 | 3 | | | III-1 | Slc17a3 | 10786 | 4-May-15 |
| 19457 | 2 | 3 | | | III-1 | Slc17a6 | 57084 | 12-May-15 |
| 19458 | 2 | 3 | | | III-1 | Slc17a7 | 57030 | 4-May-15 |
| 19461 | 2 | 3 | | | III-1 | Slc18a1 | 6570 | 3-May-15 |
| 19462 | 2 | 3 | | | III-1 | Slc18a2 | 6571 | 4-May-15 |
| 19466 | 2 | 3 | | | III-1 | Slc19a2 | 10560 | 23-May-15 |
| 19469 | 2 | 3 | | | III-1 | Slc1a2 | 6506 | 21-May-15 |
| 19473 | 2 | 3 | | | III-1 | Slc1a6 | 6511 | 4-May-15 |
| 19478 | 2 | 3 | | | III-1 | Slc22a12 | 116085 | 12-May-15 |
| 19485 | 2 | 3 | | | III-1 | Slc22a18 | 5002 | 23-May-15 |
| 19486 | 2 | 3 | | | III-1 | Slc22a19 | | |
| 19490 | 2 | 3 | | | III-1 | Slc22a22 | | |
| 19494 | 2 | 3 | | | III-1 | Slc22a28 | | |
| 19496 | 2 | 3 | | | III-1 | Slc22a3 | 6581 | 17-May-15 |
| 19499 | 2 | 3 | | | III-1 | Slc22a5 | 6584 | 23-May-15 |
| 19500 | 2 | 3 | | | III-1 | Slc22a6 | 9356 | 4-May-15 |
| 19503 | 2 | 3 | | | III-1 | Slc23a1 | 9963 | 7-Jun-15 |
| 19504 | 2 | 3 | | | III-1 | Slc23a2 | 9962 | 7-Jun-15 |
| 19506 | 2 | 3 | | | III-1 | Slc24a1 | 9187 | 4-May-15 |
| 19516 | 2 | 3 | | | III-1 | Slc25a14 | 9016 | 4-May-15 |
| 19517 | 2 | 3 | | | III-1 | Slc25a15 | 10166 | 23-May-15 |
| 19520 | 2 | 3 | | | III-1 | Slc25a18 | 83733 | 12-May-15 |
| 19525 | 2 | 3 | | | III-1 | Slc25a22 | 79751 | 4-May-15 |
| 19529 | 2 | 3 | | | III-1 | Slc25a26 | 115286 | 4-May-15 |
| 19530 | 2 | 3 | | | III-1 | Slc25a27 | 9481 | 4-May-15 |
| 19531 | 2 | 3 | | | III-1 | Slc25a28 | 81894 | 12-May-15 |
| 19533 | 2 | 3 | | | III-1 | Slc25a3 | 5250 | 21-May-15 |
| 19538 | 2 | 3 | | | III-1 | Slc25a34 | 284723 | 4-May-15 |
| 19539 | 2 | 3 | | | III-1 | Slc25a35 | 399512 | 21-May-15 |
| 19542 | 2 | 3 | | | III-1 | Slc25a38 | 54977 | 24-May-15 |
| 19543 | 2 | 3 | | | III-1 | Slc25a39 | 51629 | 4-May-15 |
| 19545 | 2 | 3 | | | III-1 | Slc25a40 | 55972 | 4-May-15 |
| 19548 | 2 | 3 | | | III-1 | Slc25a43 | 203427 | 4-May-15 |
| 19551 | 2 | 3 | | | III-1 | Slc25a46 | 91137 | 3-Jun-15 |
| 19555 | 2 | 3 | | | III-1 | Slc25a51 | 92014 | 4-May-15 |
| 19557 | 2 | 3 | | | III-1 | Slc25a54 | | |
| 19559 | 2 | 3 | | | III-1 | Slc26a10 | 65012 | 4-May-15 |
| 19563 | 2 | 3 | | | III-1 | Slc26a4 | 5172 | 22-May-15 |
| 19574 | 2 | 3 | | | III-1 | Slc27a6 | 28965 | 4-May-15 |
| 19578 | 2 | 3 | | | III-1 | Slc29a1 | 2030 | 17-May-15 |
| 19579 | 2 | 3 | | | III-1 | Slc29a2 | 3177 | 4-May-15 |
| 19580 | 2 | 3 | | | III-1 | Slc29a3 | 55315 | 4-May-15 |
| 19581 | 2 | 3 | | | III-1 | Slc29a4 | 222962 | 4-May-15 |
| 19582 | 2 | 3 | | | III-1 | Slc2a1 | 6513 | 24-May-15 |
| 19583 | 2 | 3 | | | III-1 | Slc2a10 | 81031 | 23-May-15 |
| 19593 | 2 | 3 | | | III-1 | Slc2a8 | 29988 | 4-May-15 |
| 19594 | 2 | 3 | | | III-1 | Slc2a9 | 56606 | 31-May-15 |
| 19595 | 2 | 3 | | | III-1 | Slc30a1 | 7779 | 17-May-15 |
| 19606 | 2 | 3 | | | III-1 | Slc31a2 | 1318 | 4-May-15 |
| 19614 | 2 | 3 | | | III-1 | Slc35a3 | 23443 | 4-May-15 |
| 19615 | 2 | 3 | | | III-1 | Slc35a4 | 113829 | 4-May-15 |
| 19620 | 2 | 3 | | | III-1 | Slc35b4 | 84912 | 4-May-15 |
| 19622 | 2 | 3 | | | III-1 | Slc35c2 | 51006 | 4-May-15 |
| 19624 | 2 | 3 | | | III-1 | Slc35d2 | 11046 | 4-May-15 |
| 19625 | 2 | 3 | | | III-1 | Slc35d3 | 340146 | 4-May-15 |
| 19630 | 2 | 3 | | | III-1 | Slc35f1 | 222553 | 4-May-15 |
| 19632 | 2 | 3 | | | III-1 | Slc35f3 | 148641 | 4-May-15 |
| 19637 | 2 | 3 | | | III-1 | Slc35g2 | 80723 | 4-May-15 |
| 19638 | 2 | 3 | | | III-1 | Slc35g3 | 146861 | 4-May-15 |
| 19640 | 2 | 3 | | | III-1 | Slc36a1os | | |
| 19646 | 2 | 3 | | | III-1 | Slc37a3 | 84255 | 4-May-15 |
| 19649 | 2 | 3 | | | III-1 | Slc38a10 | 124565 | 12-May-15 |
| 19656 | 2 | 3 | | | III-1 | Slc38a7 | 55238 | 12-May-15 |
| 19659 | 2 | 3 | | | III-1 | Slc39a1 | 27173 | 17-May-15 |
| 19665 | 2 | 3 | | | III-1 | Slc39a2 | 29986 | 4-May-15 |
| 19666 | 2 | 3 | | | III-1 | Slc39a3 | 29985 | 4-May-15 |
| 19668 | 2 | 3 | | | III-1 | Slc39a5 | 283375 | 4-May-15 |
| 19670 | 2 | 3 | | | III-1 | Slc39a7 | 7922 | 4-May-15 |
| 19672 | 2 | 3 | | | III-1 | Slc39a9 | 55334 | 12-May-15 |
| 19674 | 2 | 3 | | | III-1 | Slc3a2 | 6520 | 12-May-15 |
| 19675 | 2 | 3 | | | III-1 | Slc40a1 | 30061 | 28-May-15 |
| 19681 | 2 | 3 | | | III-1 | Slc43a3 | 29015 | 4-May-15 |
| 19682 | 2 | 3 | | | III-1 | Slc44a1 | 23446 | 4-May-15 |
| 19686 | 2 | 3 | | | III-1 | Slc44a5 | 204962 | 14-May-15 |
| 19690 | 2 | 3 | | | III-1 | Slc45a4 | 57210 | 4-May-15 |
| 19693 | 2 | 3 | | | III-1 | Slc46a3 | 283537 | 12-May-15 |
| 19695 | 2 | 3 | | | III-1 | Slc47a2 | 146802 | 4-May-15 |
| 19700 | 2 | 3 | | | III-1 | Slc4a1ap | 22950 | 4-May-15 |
| 19704 | 2 | 3 | | | III-1 | Slc4a5 | 57835 | 4-May-15 |
| 19706 | 2 | 3 | | | III-1 | Slc4a8 | 9498 | 4-May-15 |
| 19711 | 2 | 3 | | | III-1 | Slc52a2 | 79581 | 4-May-15 |
| 19714 | 2 | 3 | | | III-1 | Slc5a10 | 125206 | 4-May-15 |
| 19717 | 2 | 3 | | | III-1 | Slc5a2 | 6524 | 20-May-15 |
| 19720 | 2 | 3 | | | III-1 | Slc5a4b | | |
| 19723 | 2 | 3 | | | III-1 | Slc5a7 | 60482 | 12-May-15 |
| 19725 | 2 | 3 | | | III-1 | Slc5a9 | 200010 | 4-May-15 |
| 19726 | 2 | 3 | | | III-1 | Slc6a1 | 6529 | 28-May-15 |
| 19728 | 2 | 3 | | | III-1 | Slc6a12 | 6539 | 4-May-15 |
| 19737 | 2 | 3 | | | III-1 | Slc6a20a | | |
| 19738 | 2 | 3 | | | III-1 | Slc6a20b | | |
| 19739 | 2 | 3 | | | III-1 | Slc6a3 | 6531 | 24-May-15 |
| 19743 | 2 | 3 | | | III-1 | Slc6a7 | 6534 | 4-May-15 |
| 19750 | 2 | 3 | | | III-1 | Slc7a13 | 157724 | 4-May-15 |
| 19754 | 2 | 3 | | | III-1 | Slc7a3 | 84889 | 4-May-15 |
| 19757 | 2 | 3 | | | III-1 | Slc7a6 | 9057 | 4-May-15 |
| 19758 | 2 | 3 | | | III-1 | Slc7a6os | 84138 | 4-May-15 |
| 19761 | 2 | 3 | | | III-1 | Slc7a9 | 11136 | 12-May-15 |
| 19763 | 2 | 3 | | | III-1 | Slc8a2 | 6543 | 4-May-15 |
| 19765 | 2 | 3 | | | III-1 | Slc8b1 | 80024 | 4-May-15 |
| 19766 | 2 | 3 | | | III-1 | Slc9a1 | 6548 | 4-May-15 |
| 19770 | 2 | 3 | | | III-1 | Slc9a3r2 | 9351 | 4-May-15 |
| 19772 | 2 | 3 | | | III-1 | Slc9a5 | 6553 | 4-May-15 |
| 19775 | 2 | 3 | | | III-1 | Slc9a8 | 23315 | 4-May-15 |
| 19779 | 2 | 3 | | | III-1 | Slc9c1 | 285335 | 4-May-15 |
| 19783 | 2 | 3 | | | III-1 | Slco1a6 | | |
| 19786 | 2 | 3 | | | III-1 | Slco2a1 | 6578 | 17-May-15 |
| 19787 | 2 | 3 | | | III-1 | Slco2b1 | 11309 | 12-May-15 |
| 19804 | 2 | 3 | | | III-1 | Slfn9 | | |
| 19809 | 2 | 3 | | | III-1 | Slit3 | 6586 | 7-Jun-15 |
| 19810 | 2 | 3 | | | III-1 | Slitrk1 | 114798 | 23-May-15 |
| 19819 | 2 | 3 | | | III-1 | Slmo2 | 51012 | 4-May-15 |
| 19825 | 2 | 3 | | | III-1 | Slx | | |
| 19827 | 2 | 3 | | | III-1 | Slx4 | 84464 | 23-May-15 |
| 19828 | 2 | 3 | | | III-1 | Slx4ip | 128710 | 4-May-15 |
| 19832 | 2 | 3 | | | III-1 | Smad2 | 4087 | 24-May-15 |
| 19837 | 2 | 3 | | | III-1 | Smad7 | 4092 | 12-May-15 |
| 19838 | 2 | 3 | | | III-1 | Smad9 | 4093 | 4-May-15 |
| 19839 | 2 | 3 | | | III-1 | Smagp | 57228 | 4-May-15 |
| 19840 | 2 | 3 | | | III-1 | Smap1 | 60682 | 7-Jun-15 |
| 19843 | 2 | 3 | | | III-1 | Smarca2 | 6595 | 4-May-15 |
| 19855 | 2 | 3 | | | III-1 | Smarce1 | 6605 | 12-May-15 |
| 19864 | 2 | 3 | | | III-1 | Smchd1 | 23347 | 23-May-15 |
| 19871 | 2 | 3 | | | III-1 | Smdt1 | 91689 | 4-May-15 |
| 19875 | 2 | 3 | | | III-1 | Smg5 | 23381 | 12-May-15 |
| 19880 | 2 | 3 | | | III-1 | Smgc | | |
| 19883 | 2 | 3 | | | III-1 | Smim12 | 113444 | 4-May-15 |
| 19889 | 2 | 3 | | | III-1 | Smim20 | 389203 | 4-May-15 |
| 19891 | 2 | 3 | | | III-1 | Smim23 | 644994 | 21-May-15 |
| 19896 | 2 | 3 | | | III-1 | Smim6 | 100130933 | 4-May-15 |
| 19897 | 2 | 3 | | | III-1 | Smim7 | 79086 | 4-May-15 |
| 19899 | 2 | 3 | | | III-1 | Smim9 | 100132963 | 4-May-15 |
| 19905 | 2 | 3 | | | III-1 | Smoc2 | 64094 | 4-May-15 |
| 19906 | 2 | 3 | | | III-1 | Smok2a | | |
| 19917 | 2 | 3 | | | III-1 | Smpdl3a | 10924 | 23-May-15 |
| 19923 | 2 | 3 | | | III-1 | Smtn | 6525 | 4-May-15 |
| 19932 | 2 | 3 | | | III-1 | Smyd3 | 64754 | 21-May-15 |
| 19938 | 2 | 3 | | | III-1 | Snap23 | 8773 | 4-May-15 |
| 19940 | 2 | 3 | | | III-1 | Snap29 | 9342 | 28-May-15 |
| 19947 | 2 | 3 | | | III-1 | Snapc5 | 10302 | 21-May-15 |
| 19951 | 2 | 3 | | | III-1 | Sncb | 6620 | 4-May-15 |
| 19954 | 2 | 3 | | | III-1 | Sned1 | 25992 | 12-May-15 |
| 19956 | 2 | 3 | | | III-1 | Snhg1 | 23642 | 4-May-15 |
| 19960 | 2 | 3 | | | III-1 | Snhg18 | 100505806 | 4-May-15 |
| 19962 | 2 | 3 | | | III-1 | Snhg4 | 724102 | 4-May-15 |
| 19968 | 2 | 3 | | | III-1 | Snip1 | 79753 | 4-May-15 |
| 20081 | 2 | 3 | | | III-1 | Snrk | 54861 | 4-May-15 |
| 20082 | 2 | 3 | | | III-1 | Snrnp200 | 23020 | 23-May-15 |
| 20083 | 2 | 3 | | | III-1 | Snrnp25 | 79622 | 4-May-15 |
| 20084 | 2 | 3 | | | III-1 | Snrnp27 | 11017 | 4-May-15 |
| 20086 | 2 | 3 | | | III-1 | Snrnp40 | 9410 | 4-May-15 |
| 20088 | 2 | 3 | | | III-1 | Snrnp70 | 6625 | 12-May-15 |
| 20089 | 2 | 3 | | | III-1 | Snrpa | 6626 | 12-May-15 |
| 20091 | 2 | 3 | | | III-1 | Snrpb | 6628 | 28-May-15 |
| 20092 | 2 | 3 | | | III-1 | Snrpb2 | 6629 | 4-May-15 |
| 20096 | 2 | 3 | | | III-1 | Snrpd3 | 6634 | 4-May-15 |
| 20109 | 2 | 3 | | | III-1 | Snw1 | 22938 | 4-May-15 |
| 20112 | 2 | 3 | | | III-1 | Snx11 | 29916 | 21-May-15 |
| 20114 | 2 | 3 | | | III-1 | Snx13 | 23161 | 12-May-15 |
| 20125 | 2 | 3 | | | III-1 | Snx24 | 28966 | 4-May-15 |
| 20129 | 2 | 3 | | | III-1 | Snx3 | 8724 | 2-Jun-15 |
| 20131 | 2 | 3 | | | III-1 | Snx31 | 169166 | 4-May-15 |
| 20133 | 2 | 3 | | | III-1 | Snx33 | 257364 | 12-May-15 |
| 20136 | 2 | 3 | | | III-1 | Snx6 | 58533 | 4-May-15 |
| 20138 | 2 | 3 | | | III-1 | Snx8 | 29886 | 4-May-15 |
| 20148 | 2 | 3 | | | III-1 | Socs6 | 9306 | 4-May-15 |
| 20151 | 2 | 3 | | | III-1 | Sod2 | 6648 | 17-May-15 |
| 20152 | 2 | 3 | | | III-1 | Sod3 | 6649 | 4-May-15 |
| 20156 | 2 | 3 | | | III-1 | Sohlh2 | 54937 | 4-May-15 |
| 20158 | 2 | 3 | | | III-1 | Sorbs1 | 10580 | 12-May-15 |
| 20159 | 2 | 3 | | | III-1 | Sorbs2 | 8470 | 20-May-15 |
| 20160 | 2 | 3 | | | III-1 | Sorbs2os | | |
| 20164 | 2 | 3 | | | III-1 | Sorcs3 | 22986 | 4-May-15 |

Fig.22 - 82

| | | | | | | | | |
|---|---|---|---|---|---|---|---|---|
| 20166 | 2 | 3 | | | III-1 | Sorl1 | 6653 | 23-May-15 |
| 20171 | 2 | 3 | | | III-1 | Sostdc1 | 25928 | 4-May-15 |
| 20174 | 2 | 3 | | | III-1 | Sowahc | 65124 | 4-May-15 |
| 20175 | 2 | 3 | | | III-1 | Sowahd | 347454 | 4-May-15 |
| 20176 | 2 | 3 | | | III-1 | Sox1 | 6656 | 4-May-15 |
| 20180 | 2 | 3 | | | III-1 | Sox13 | 9580 | 4-May-15 |
| 20181 | 2 | 3 | | | III-1 | Sox14 | 8403 | 4-May-15 |
| 20184 | 2 | 3 | | | III-1 | Sox18 | 54345 | 4-May-15 |
| 20185 | 2 | 3 | | | III-1 | Sox2 | 6657 | 24-May-15 |
| 20197 | 2 | 3 | | | III-1 | Sp1 | 6667 | 13-Jun-15 |
| 20202 | 2 | 3 | | | III-1 | Sp3 | 6670 | 16-Jun-15 |
| 20204 | 2 | 3 | | | III-1 | Sp4 | 6671 | 28-May-15 |
| 20207 | 2 | 3 | | | III-1 | Sp7 | 121340 | 17-May-15 |
| 20211 | 2 | 3 | | | III-1 | Spaca1 | 81833 | 4-May-15 |
| 20216 | 2 | 3 | | | III-1 | Spaca7 | 122258 | 4-May-15 |
| 20220 | 2 | 3 | | | III-1 | Spag16 | 79582 | 12-May-15 |
| 20231 | 2 | 3 | | | III-1 | Spast | 6683 | 31-May-15 |
| 20233 | 2 | 3 | | | III-1 | Spata13 | 221178 | 4-May-15 |
| 20234 | 2 | 3 | | | III-1 | Spata16 | 83893 | 12-May-15 |
| 20237 | 2 | 3 | | | III-1 | Spata19 | 219938 | 4-May-15 |
| 20243 | 2 | 3 | | | III-1 | Spata25 | 128497 | 4-May-15 |
| 20246 | 2 | 3 | | | III-1 | Spata31 | | |
| 20256 | 2 | 3 | | | III-1 | Spata5l1 | 79029 | 4-May-15 |
| 20259 | 2 | 3 | | | III-1 | Spata9 | 83890 | 4-May-15 |
| 20270 | 2 | 3 | | | III-1 | Spdef | 25803 | 20-May-15 |
| 20272 | 2 | 3 | | | III-1 | Spdya | 245711 | 4-May-15 |
| 20273 | 2 | 3 | | | III-1 | Spdyb | | |
| 20275 | 2 | 3 | | | III-1 | Specc1l | 23384 | 28-May-15 |
| 20276 | 2 | 3 | | | III-1 | Speer1-ps1 | | |
| 20291 | 2 | 3 | | | III-1 | Spef2 | 79925 | 4-May-15 |
| 20294 | 2 | 3 | | | III-1 | Spen | 23013 | 13-Jun-15 |
| 20296 | 2 | 3 | | | III-1 | Spesp1 | 246777 | 4-May-15 |
| 20298 | 2 | 3 | | | III-1 | Spg20 | 23111 | 23-May-15 |
| 20302 | 2 | 3 | | | III-1 | Sphk2 | 56848 | 4-May-15 |
| 20303 | 2 | 3 | | | III-1 | Sphkap | 80309 | 12-May-15 |
| 20304 | 2 | 3 | | | III-1 | Spi1 | 6688 | 12-May-15 |
| 20307 | 2 | 3 | | | III-1 | Spice1 | 152185 | 4-May-15 |
| 20311 | 2 | 3 | | | III-1 | Spin2d | | |
| 20318 | 2 | 3 | | | III-1 | Spink14 | 408187 | 21-May-15 |
| 20323 | 2 | 3 | | | III-1 | Spink6 | 404203 | 4-May-15 |
| 20333 | 2 | 3 | | | III-1 | Spire2 | 84501 | 24-May-15 |
| 20334 | 2 | 3 | | | III-1 | Spn | 6693 | 7-Jun-15 |
| 20342 | 2 | 3 | | | III-1 | Spock3 | 50859 | 12-May-15 |
| 20343 | 2 | 3 | | | III-1 | Spon1 | 10418 | 4-May-15 |
| 20357 | 2 | 3 | | | III-1 | Sprn | 503542 | 4-May-15 |
| 20364 | 2 | 3 | | | III-1 | Sprr2e | 6704 | 4-May-15 |
| 20369 | 2 | 3 | | | III-1 | Sprr2j-ps | | |
| 20372 | 2 | 3 | | | III-1 | Sprr4 | 163778 | 16-May-15 |
| 20373 | 2 | 3 | | | III-1 | Sprtn | 83932 | 12-May-15 |
| 20376 | 2 | 3 | | | III-1 | Spry3 | 10251 | 12-May-15 |
| 20379 | 2 | 3 | | | III-1 | Spryd4 | 283377 | 4-May-15 |
| 20380 | 2 | 3 | | | III-1 | Spryd7 | 57213 | 4-May-15 |
| 20381 | 2 | 3 | | | III-1 | Spsb1 | 80176 | 4-May-15 |
| 20382 | 2 | 3 | | | III-1 | Spsb2 | 84727 | 4-May-15 |
| 20383 | 2 | 3 | | | III-1 | Spsb3 | 90864 | 4-May-15 |
| 20391 | 2 | 3 | | | III-1 | Sptbn4 | 57731 | 4-May-15 |
| 20395 | 2 | 3 | | | III-1 | Sptssa | 171546 | 4-May-15 |
| 20401 | 2 | 3 | | | III-1 | Sqstm1 | 8878 | 24-May-15 |
| 20402 | 2 | 3 | | | III-1 | Sra1 | 10011 | 7-Jun-15 |
| 20403 | 2 | 3 | | | III-1 | Srbd1 | 55133 | 4-May-15 |
| 20405 | 2 | 3 | | | III-1 | Srcin1 | 80725 | 4-May-15 |
| 20412 | 2 | 3 | | | III-1 | Srek1 | 140890 | 3-Jun-15 |
| 20416 | 2 | 3 | | | III-1 | Srgap1 | 57522 | 4-May-15 |
| 20418 | 2 | 3 | | | III-1 | Srgap3 | 9901 | 7-Jun-15 |
| 20419 | 2 | 3 | | | III-1 | Srgn | 5552 | 12-May-15 |
| 20423 | 2 | 3 | | | III-1 | Srms | 6725 | 4-May-15 |
| 20425 | 2 | 3 | | | III-1 | Srp19 | 6728 | 4-May-15 |
| 20426 | 2 | 3 | | | III-1 | Srp54a | | |
| 20433 | 2 | 3 | | | III-1 | Srpk2 | 6733 | 4-May-15 |
| 20435 | 2 | 3 | | | III-1 | Srpr | 6734 | 4-May-15 |
| 20438 | 2 | 3 | | | III-1 | Srpx2 | 27286 | 23-May-15 |
| 20441 | 2 | 3 | | | III-1 | Srrm1 | 10250 | 4-May-15 |
| 20443 | 2 | 3 | | | III-1 | Srrm3 | 222183 | 4-May-15 |
| 20446 | 2 | 3 | | | III-1 | Srrt | 51593 | 4-May-15 |
| 20447 | 2 | 3 | | | III-1 | Srsf1 | 6426 | 4-May-15 |
| 20455 | 2 | 3 | | | III-1 | Srsf6 | 6431 | 21-May-15 |
| 20459 | 2 | 3 | | | III-1 | Sry | 6736 | 28-May-15 |
| 20462 | 2 | 3 | | | III-1 | Ssb | 6741 | 21-May-15 |
| 20467 | 2 | 3 | | | III-1 | Ssc5d | 284297 | 4-May-15 |
| 20469 | 2 | 3 | | | III-1 | Ssh1 | 54434 | 24-May-15 |
| 20471 | 2 | 3 | | | III-1 | Ssh3 | 54961 | 12-May-15 |
| 20472 | 2 | 3 | | | III-1 | Ssmem1 | 136263 | 4-May-15 |
| 20475 | 2 | 3 | | | III-1 | Sspo | 23145 | 4-May-15 |
| 20476 | 2 | 3 | | | III-1 | Ssr1 | 6745 | 12-May-15 |
| 20478 | 2 | 3 | | | III-1 | Ssr3 | 6747 | 21-May-15 |
| 20480 | 2 | 3 | | | III-1 | Ssrp1 | 6749 | 24-May-15 |
| 20484 | 2 | 3 | | | III-1 | Sstr2 | 6752 | 24-May-15 |
| 20493 | 2 | 3 | | | III-1 | Ssx9 | 280660 | 4-May-15 |
| 20497 | 2 | 3 | | | III-1 | Ssxb3 | | |
| 20504 | 2 | 3 | | | III-1 | St18 | 9705 | 12-May-15 |
| 20506 | 2 | 3 | | | III-1 | St3gal2 | 6483 | 12-May-15 |
| 20508 | 2 | 3 | | | III-1 | St3gal4 | 6484 | 21-May-15 |
| 20509 | 2 | 3 | | | III-1 | St3gal5 | 8869 | 12-May-15 |
| 20511 | 2 | 3 | | | III-1 | St5 | 6764 | 12-May-15 |
| 20513 | 2 | 3 | | | III-1 | St6gal2 | 84620 | 24-May-15 |
| 20519 | 2 | 3 | | | III-1 | St6galnac6 | 30815 | 4-May-15 |
| 20520 | 2 | 3 | | | III-1 | St7 | 7982 | 7-Jun-15 |
| 20521 | 2 | 3 | | | III-1 | St7l | 54879 | 21-May-15 |
| 20526 | 2 | 3 | | | III-1 | St8sia4 | 7903 | 4-May-15 |
| 20529 | 2 | 3 | | | III-1 | Stab1 | 23166 | 12-May-15 |
| 20531 | 2 | 3 | | | III-1 | Stac | 6769 | 12-May-15 |
| 20532 | 2 | 3 | | | III-1 | Stac2 | 342667 | 12-May-15 |
| 20538 | 2 | 3 | | | III-1 | Stam2 | 10254 | 4-May-15 |
| 20540 | 2 | 3 | | | III-1 | Stambpl1 | 57559 | 4-May-15 |
| 20543 | 2 | 3 | | | III-1 | Stap2 | 55620 | 4-May-15 |
| 20544 | 2 | 3 | | | III-1 | Star | 6770 | 7-Jun-15 |
| 20546 | 2 | 3 | | | III-1 | Stard13 | 90627 | 17-May-15 |
| 20547 | 2 | 3 | | | III-1 | Stard3 | 10948 | 4-May-15 |
| 20551 | 2 | 3 | | | III-1 | Stard6 | 147323 | 4-May-15 |
| 20554 | 2 | 3 | | | III-1 | Stat1 | 6772 | 31-May-15 |
| 20556 | 2 | 3 | | | III-1 | Stat3 | 6774 | 31-May-15 |
| 20557 | 2 | 3 | | | III-1 | Stat4 | 6775 | 12-May-15 |
| 20560 | 2 | 3 | | | III-1 | Stat6 | 6778 | 24-May-15 |
| 20563 | 2 | 3 | | | III-1 | Stbd1 | 8987 | 4-May-15 |
| 20566 | 2 | 3 | | | III-1 | Steap1 | 26872 | 4-May-15 |
| 20579 | 2 | 3 | | | III-1 | Stk11 | 6794 | 24-May-15 |
| 20583 | 2 | 3 | | | III-1 | Stk19 | 8859 | 12-May-15 |
| 20589 | 2 | 3 | | | III-1 | Stk32b | 55351 | 14-May-15 |
| 20590 | 2 | 3 | | | III-1 | Stk32c | 282974 | 4-May-15 |
| 20591 | 2 | 3 | | | III-1 | Stk33 | 65975 | 12-May-15 |
| 20592 | 2 | 3 | | | III-1 | Stk35 | 140901 | 4-May-15 |
| 20596 | 2 | 3 | | | III-1 | Stk39 | 27347 | 4-May-15 |
| 20597 | 2 | 3 | | | III-1 | Stk4 | 6789 | 4-May-15 |
| 20602 | 2 | 3 | | | III-1 | Stmn3 | 50861 | 12-May-15 |
| 20606 | 2 | 3 | | | III-1 | Stoml1 | 9399 | 12-May-15 |
| 20608 | 2 | 3 | | | III-1 | Stoml3 | 161003 | 4-May-15 |
| 20611 | 2 | 3 | | | III-1 | Stox1 | 219736 | 3-May-15 |
| 20612 | 2 | 3 | | | III-1 | Stox2 | 56977 | 4-May-15 |
| 20613 | 2 | 3 | | | III-1 | Stpg1 | 90529 | 4-May-15 |
| 20616 | 2 | 3 | | | III-1 | Stra6 | 64220 | 4-May-15 |
| 20620 | 2 | 3 | | | III-1 | Strap | 11171 | 7-Jun-15 |
| 20622 | 2 | 3 | | | III-1 | Strc | 161497 | 23-May-15 |
| 20631 | 2 | 3 | | | III-1 | Stx11 | 8676 | 23-May-15 |
| 20633 | 2 | 3 | | | III-1 | Stx16 | 8675 | 4-May-15 |
| 20636 | 2 | 3 | | | III-1 | Stx19 | 415117 | 4-May-15 |
| 20637 | 2 | 3 | | | III-1 | Stx1a | 6804 | 12-May-15 |
| 20638 | 2 | 3 | | | III-1 | Stx1b | 112755 | 12-May-15 |
| 20639 | 2 | 3 | | | III-1 | Stx2 | 2054 | 4-May-15 |
| 20640 | 2 | 3 | | | III-1 | Stx3 | 6809 | 3-May-15 |
| 20646 | 2 | 3 | | | III-1 | Stxbp1 | 6812 | 26-May-15 |
| 20655 | 2 | 3 | | | III-1 | Styx | 6815 | 4-May-15 |
| 20658 | 2 | 3 | | | III-1 | Sucla2 | 8803 | 23-May-15 |
| 20659 | 2 | 3 | | | III-1 | Suclg1 | 8802 | 4-May-15 |
| 20660 | 2 | 3 | | | III-1 | Suclg2 | 8801 | 4-May-15 |
| 20661 | 2 | 3 | | | III-1 | Sucnr1 | 56670 | 4-May-15 |
| 20666 | 2 | 3 | | | III-1 | Sugp1 | 57794 | 4-May-15 |
| 20667 | 2 | 3 | | | III-1 | Sugp2 | 10147 | 4-May-15 |
| 20669 | 2 | 3 | | | III-1 | Sulf1 | 23213 | 17-May-15 |
| 20670 | 2 | 3 | | | III-1 | Sulf2 | 55959 | 4-May-15 |
| 20672 | 2 | 3 | | | III-1 | Sult1b1 | 27284 | 4-May-15 |
| 20685 | 2 | 3 | | | III-1 | Sult3a1 | | |
| 20687 | 2 | 3 | | | III-1 | Sult5a1 | | |
| 20693 | 2 | 3 | | | III-1 | Sumo3 | 6612 | 7-Jun-15 |
| 20696 | 2 | 3 | | | III-1 | Sun3 | 256979 | 4-May-15 |
| 20699 | 2 | 3 | | | III-1 | Supt16 | | |
| 20701 | 2 | 3 | | | III-1 | Supt3 | | |
| 20703 | 2 | 3 | | | III-1 | Supt5 | | |
| 20716 | 2 | 3 | | | III-1 | Suv39h1 | 6839 | 12-May-15 |
| 20722 | 2 | 3 | | | III-1 | Sv2b | 9899 | 4-May-15 |
| 20731 | 2 | 3 | | | III-1 | Svop | 55530 | 4-May-15 |
| 20733 | 2 | 3 | | | III-1 | Svs1 | | |
| 20744 | 2 | 3 | | | III-1 | Syap1 | 94056 | 4-May-15 |
| 20755 | 2 | 3 | | | III-1 | Syde1 | 85360 | 4-May-15 |
| 20757 | 2 | 3 | | | III-1 | Syf2 | 25949 | 17-May-15 |
| 20759 | 2 | 3 | | | III-1 | Sympk | 8189 | 4-May-15 |
| 20760 | 2 | 3 | | | III-1 | Syn1 | 6853 | 7-Jun-15 |
| 20762 | 2 | 3 | | | III-1 | Syn3 | 8224 | 4-May-15 |
| 20770 | 2 | 3 | | | III-1 | Syne2 | 23224 | 4-May-15 |
| 20773 | 2 | 3 | | | III-1 | Syngap1 | 8831 | 3-Jun-15 |
| 20777 | 2 | 3 | | | III-1 | Syngr4 | 23546 | 4-May-15 |
| 20780 | 2 | 3 | | | III-1 | Synj2bp | 55333 | 4-May-15 |
| 20798 | 2 | 3 | | | III-1 | Syt16 | 83851 | 21-May-15 |
| 20802 | 2 | 3 | | | III-1 | Syt4 | 6860 | 12-May-15 |
| 20805 | 2 | 3 | | | III-1 | Syt7 | 9066 | 4-May-15 |
| 20812 | 2 | 3 | | | III-1 | Sytl5 | 94122 | 21-May-15 |
| 20814 | 2 | 3 | | | III-1 | Szrd1 | 26099 | 4-May-15 |
| 20837 | 2 | 3 | | | III-1 | Tac2 | 6863 | 4-May-15 |
| 20839 | 2 | 3 | | | III-1 | Tacc1 | 6867 | 4-May-15 |
| 20841 | 2 | 3 | | | III-1 | Tacc3 | 10460 | 23-May-15 |
| 20842 | 2 | 3 | | | III-1 | Taco1 | 51204 | 4-May-15 |
| 20843 | 2 | 3 | | | III-1 | Tacr1 | 6869 | 17-May-15 |
| 20845 | 2 | 3 | | | III-1 | Tacr3 | 6870 | 12-May-15 |
| 20847 | 2 | 3 | | | III-1 | Tada1 | 117143 | 7-Jun-15 |
| 20849 | 2 | 3 | | | III-1 | Tada2b | 93624 | 4-May-15 |
| 20856 | 2 | 3 | | | III-1 | Taf15 | 8148 | 4-May-15 |
| 20857 | 2 | 3 | | | III-1 | Taf1a | 9015 | 4-May-15 |
| 20858 | 2 | 3 | | | III-1 | Taf1b | 9014 | 4-May-15 |
| 20859 | 2 | 3 | | | III-1 | Taf1c | 9013 | 4-May-15 |
| 20861 | 2 | 3 | | | III-1 | Taf2 | 6873 | 12-May-15 |
| 20865 | 2 | 3 | | | III-1 | Taf5 | 6877 | 12-May-15 |
| 20869 | 2 | 3 | | | III-1 | Taf7 | 6879 | 4-May-15 |
| 20872 | 2 | 3 | | | III-1 | Taf9 | 6880 | 4-May-15 |
| 20875 | 2 | 3 | | | III-1 | Tagap1 | 117289 | 4-May-15 |
| 20877 | 2 | 3 | | | III-1 | Tagln2 | 8407 | 4-May-15 |

Fig.22 - 83

| | | | | | | | | |
|---|---|---|---|---|---|---|---|---|
| 20878 | 2 | 3 | | | III-1 | Tagln3 | 29114 | 4-May-15 |
| 20881 | 2 | 3 | | | III-1 | Taldo1 | 6888 | 12-May-15 |
| 20882 | 2 | 3 | | | III-1 | Tamm41 | 132001 | 4-May-15 |
| 20883 | 2 | 3 | | | III-1 | Tanc1 | 85461 | 4-May-15 |
| 20884 | 2 | 3 | | | III-1 | Tanc2 | 26115 | 4-May-15 |
| 20886 | 2 | 3 | | | III-1 | Tango6 | 79613 | 12-May-15 |
| 20888 | 2 | 3 | | | III-1 | Taok1 | 57551 | 4-May-15 |
| 20897 | 2 | 3 | | | III-1 | Tardbp | 23435 | 31-May-15 |
| 20899 | 2 | 3 | | | III-1 | Tars | 6897 | 4-May-15 |
| 20902 | 2 | 3 | | | III-1 | Tas1r1 | 80835 | 4-May-15 |
| 20905 | 2 | 3 | | | III-1 | Tas2r102 | | |
| 20944 | 2 | 3 | | | III-1 | Tatdn3 | 128387 | 4-May-15 |
| 20947 | 2 | 3 | | | III-1 | Taz | 6901 | 7-Jun-15 |
| 20950 | 2 | 3 | | | III-1 | Tbc1d10a | 83874 | 4-May-15 |
| 20958 | 2 | 3 | | | III-1 | Tbc1d17 | 79735 | 21-May-15 |
| 20961 | 2 | 3 | | | III-1 | Tbc1d20 | 128637 | 4-May-15 |
| 20964 | 2 | 3 | | | III-1 | Tbc1d22b | 55633 | 4-May-15 |
| 20968 | 2 | 3 | | | III-1 | Tbc1d25 | 4943 | 21-May-15 |
| 20969 | 2 | 3 | | | III-1 | Tbc1d2b | 23102 | 4-May-15 |
| 20971 | 2 | 3 | | | III-1 | Tbc1d31 | 93594 | 4-May-15 |
| 20974 | 2 | 3 | | | III-1 | Tbc1d5 | 9779 | 28-May-15 |
| 20977 | 2 | 3 | | | III-1 | Tbc1d8b | 54885 | 12-May-15 |
| 20979 | 2 | 3 | | | III-1 | Tbc1d9b | 23061 | 12-May-15 |
| 20981 | 2 | 3 | | | III-1 | Tbcb | 1155 | 10-May-15 |
| 20982 | 2 | 3 | | | III-1 | Tbcc | 6903 | 4-May-15 |
| 20987 | 2 | 3 | | | III-1 | Tbck | 93627 | 4-May-15 |
| 20990 | 2 | 3 | | | III-1 | Tbl1x | 6907 | 4-May-15 |
| 21004 | 2 | 3 | | | III-1 | Tbx18 | 9096 | 4-May-15 |
| 21007 | 2 | 3 | | | III-1 | Tbx20 | 57057 | 12-May-15 |
| 21012 | 2 | 3 | | | III-1 | Tbx4 | 9496 | 4-May-15 |
| 21013 | 2 | 3 | | | III-1 | Tbx5 | 6910 | 31-May-15 |
| 21015 | 2 | 3 | | | III-1 | Tbxa2r | 6915 | 31-May-15 |
| 21017 | 2 | 3 | | | III-1 | Tc2n | 123036 | 4-May-15 |
| 21030 | 2 | 3 | | | III-1 | Tceanc | 170082 | 4-May-15 |
| 21031 | 2 | 3 | | | III-1 | Tceanc2 | 127428 | 4-May-15 |
| 21033 | 2 | 3 | | | III-1 | Tceb2 | 6923 | 2-Jun-15 |
| 21040 | 2 | 3 | | | III-1 | Tcf20 | 6942 | 4-May-15 |
| 21044 | 2 | 3 | | | III-1 | Tcf25 | 22980 | 31-May-15 |
| 21049 | 2 | 3 | | | III-1 | Tcf7l2 | 6934 | 31-May-15 |
| 21053 | 2 | 3 | | | III-1 | Tchp | 84260 | 12-May-15 |
| 21054 | 2 | 3 | | | III-1 | Tcirg1 | 10312 | 12-May-15 |
| 21055 | 2 | 3 | | | III-1 | Tcl1 | 8115 | 7-Jun-15 |
| 21062 | 2 | 3 | | | III-1 | Tcof1 | 6949 | 24-May-15 |
| 21068 | 2 | 3 | | | III-1 | Tcp11l1 | 55346 | 4-May-15 |
| 21069 | 2 | 3 | | | III-1 | Tcp11l2 | 255394 | 12-May-15 |
| 21070 | 2 | 3 | | | III-1 | Tcstv1 | | |
| 21076 | 2 | 3 | | | III-1 | Tctex1d1 | 200132 | 12-May-15 |
| 21079 | 2 | 3 | | | III-1 | Tctn1 | 79600 | 23-May-15 |
| 21083 | 2 | 3 | | | III-1 | Tdgf1 | 6997 | 13-Jun-15 |
| 21088 | 2 | 3 | | | III-1 | Tdpoz1 | | |
| 21096 | 2 | 3 | | | III-1 | Tdrd5 | 163589 | 4-May-15 |
| 21101 | 2 | 3 | | | III-1 | Tdrp | 157695 | 4-May-15 |
| 21103 | 2 | 3 | | | III-1 | Tead2 | 8463 | 4-May-15 |
| 21105 | 2 | 3 | | | III-1 | Tead4 | 7004 | 4-May-15 |
| 21109 | 2 | 3 | | | III-1 | Tecr | 9524 | 4-May-15 |
| 21110 | 2 | 3 | | | III-1 | Tecrl | 253017 | 4-May-15 |
| 21112 | 2 | 3 | | | III-1 | Tectb | 6975 | 4-May-15 |
| 21115 | 2 | 3 | | | III-1 | Tefm | 79736 | 4-May-15 |
| 21116 | 2 | 3 | | | III-1 | Tek | 7010 | 23-May-15 |
| 21119 | 2 | 3 | | | III-1 | Tekt3 | 64518 | 4-May-15 |
| 21125 | 2 | 3 | | | III-1 | Tenm1 | 10178 | 12-May-15 |
| 21128 | 2 | 3 | | | III-1 | Tenm4 | 26011 | 12-May-15 |
| 21132 | 2 | 3 | | | III-1 | Terf1 | 7013 | 4-May-15 |
| 21133 | 2 | 3 | | | III-1 | Terf2 | 7014 | 4-May-15 |
| 21134 | 2 | 3 | | | III-1 | Terf2ip | 54386 | 4-May-15 |
| 21137 | 2 | 3 | | | III-1 | Tesc | 54997 | 17-May-15 |
| 21142 | 2 | 3 | | | III-1 | Tet1 | 80312 | 31-May-15 |
| 21144 | 2 | 3 | | | III-1 | Tet3 | 200424 | 10-May-15 |
| 21145 | 2 | 3 | | | III-1 | Tex10 | 54881 | 4-May-15 |
| 21149 | 2 | 3 | | | III-1 | Tex13 | | |
| 21161 | 2 | 3 | | | III-1 | Tex261 | 113419 | 4-May-15 |
| 21164 | 2 | 3 | | | III-1 | Tex29 | 121793 | 4-May-15 |
| 21172 | 2 | 3 | | | III-1 | Tex43 | 389320 | 4-May-15 |
| 21177 | 2 | 3 | | | III-1 | Tfap2c | 7022 | 4-May-15 |
| 21181 | 2 | 3 | | | III-1 | Tfb1m | 51106 | 4-May-15 |
| 21187 | 2 | 3 | | | III-1 | Tfe3 | 7030 | 28-May-15 |
| 21189 | 2 | 3 | | | III-1 | Tfec | 22797 | 4-May-15 |
| 21194 | 2 | 3 | | | III-1 | Tfip11 | 24144 | 4-May-15 |
| 21202 | 2 | 3 | | | III-1 | Tgfa | 7039 | 12-May-15 |
| 21203 | 2 | 3 | | | III-1 | Tgfb1 | 7040 | 24-May-15 |
| 21204 | 2 | 3 | | | III-1 | Tgfb1i1 | 7041 | 4-May-15 |
| 21205 | 2 | 3 | | | III-1 | Tgfb2 | 7042 | 23-May-15 |
| 21207 | 2 | 3 | | | III-1 | Tgfbi | 7045 | 12-May-15 |
| 21208 | 2 | 3 | | | III-1 | Tgfbr1 | 7046 | 24-May-15 |
| 21209 | 2 | 3 | | | III-1 | Tgfbr2 | 7048 | 23-May-15 |
| 21211 | 2 | 3 | | | III-1 | Tgfbrap1 | 9392 | 4-May-15 |
| 21213 | 2 | 3 | | | III-1 | Tgif2 | 60436 | 4-May-15 |
| 21214 | 2 | 3 | | | III-1 | Tgif2lx1 | | |
| 21217 | 2 | 3 | | | III-1 | Tgm2 | 7052 | 12-May-15 |
| 21220 | 2 | 3 | | | III-1 | Tgm5 | 9333 | 4-May-15 |
| 21222 | 2 | 3 | | | III-1 | Tgm7 | 116179 | 12-May-15 |
| 21223 | 2 | 3 | | | III-1 | Tgoln1 | | |
| 21230 | 2 | 3 | | | III-1 | Thada | 63892 | 4-May-15 |
| 21234 | 2 | 3 | | | III-1 | Thap3 | 90326 | 4-May-15 |
| 21235 | 2 | 3 | | | III-1 | Thap4 | 51078 | 4-May-15 |
| 21241 | 2 | 3 | | | III-1 | Thbs3 | 7059 | 12-May-15 |
| 21252 | 2 | 3 | | | III-1 | Thnsl1 | 79896 | 4-May-15 |
| 21254 | 2 | 3 | | | III-1 | Thoc1 | 9984 | 4-May-15 |
| 21255 | 2 | 3 | | | III-1 | Thoc2 | 57187 | 4-May-15 |
| 21259 | 2 | 3 | | | III-1 | Thoc7 | 80145 | 4-May-15 |
| 21261 | 2 | 3 | | | III-1 | Thpo | 7066 | 12-May-15 |
| 21262 | 2 | 3 | | | III-1 | Thra | 7067 | 12-May-15 |
| 21268 | 2 | 3 | | | III-1 | Thsd7a | 221981 | 4-May-15 |
| 21276 | 2 | 3 | | | III-1 | Tia1 | 7072 | 4-May-15 |
| 21280 | 2 | 3 | | | III-1 | Ticam1 | 148022 | 17-May-15 |
| 21285 | 2 | 3 | | | III-1 | Tifab | 497189 | 4-May-15 |
| 21286 | 2 | 3 | | | III-1 | Tigd2 | 166815 | 4-May-15 |
| 21293 | 2 | 3 | | | III-1 | Timeless | 8914 | 3-May-15 |
| 21295 | 2 | 3 | | | III-1 | Timm10b | 26515 | 4-May-15 |
| 21299 | 2 | 3 | | | III-1 | Timm21 | 29090 | 4-May-15 |
| 21300 | 2 | 3 | | | III-1 | Timm22 | 29928 | 4-May-15 |
| 21302 | 2 | 3 | | | III-1 | Timm44 | 10469 | 21-May-15 |
| 21304 | 2 | 3 | | | III-1 | Timm8a1 | | |
| 21305 | 2 | 3 | | | III-1 | Timm8a2 | | |
| 21313 | 2 | 3 | | | III-1 | Tinag | 27283 | 4-May-15 |
| 21315 | 2 | 3 | | | III-1 | Tinf2 | 26277 | 23-May-15 |
| 21318 | 2 | 3 | | | III-1 | Tiprl | 261726 | 31-May-15 |
| 21320 | 2 | 3 | | | III-1 | Tjap1 | 93643 | 4-May-15 |
| 21324 | 2 | 3 | | | III-1 | Tk1 | 7083 | 4-May-15 |
| 21335 | 2 | 3 | | | III-1 | Tle3 | 7090 | 21-May-15 |
| 21337 | 2 | 3 | | | III-1 | Tle6 | 79816 | 4-May-15 |
| 21338 | 2 | 3 | | | III-1 | Tlk1 | 9874 | 4-May-15 |
| 21346 | 2 | 3 | | | III-1 | Tlr12 | | |
| 21349 | 2 | 3 | | | III-1 | Tlr3 | 7098 | 31-May-15 |
| 21350 | 2 | 3 | | | III-1 | Tlr4 | 7099 | 31-May-15 |
| 21352 | 2 | 3 | | | III-1 | Tlr6 | 10333 | 17-May-15 |
| 21354 | 2 | 3 | | | III-1 | Tlr8 | 51311 | 17-May-15 |
| 21360 | 2 | 3 | | | III-1 | Tm2d2 | 83877 | 4-May-15 |
| 21362 | 2 | 3 | | | III-1 | Tm4sf1 | 4071 | 4-May-15 |
| 21364 | 2 | 3 | | | III-1 | Tm4sf20 | 79853 | 4-May-15 |
| 21367 | 2 | 3 | | | III-1 | Tm6sf1 | 53346 | 17-May-15 |
| 21370 | 2 | 3 | | | III-1 | Tm7sf3 | 51768 | 12-May-15 |
| 21373 | 2 | 3 | | | III-1 | Tm9sf3 | 56889 | 4-May-15 |
| 21376 | 2 | 3 | | | III-1 | Tma7 | 51372 | 4-May-15 |
| 21377 | 2 | 3 | | | III-1 | Tmbim1 | 64114 | 4-May-15 |
| 21379 | 2 | 3 | | | III-1 | Tmbim6 | 7009 | 31-May-15 |
| 21386 | 2 | 3 | | | III-1 | Tmc6 | 11322 | 4-May-15 |
| 21388 | 2 | 3 | | | III-1 | Tmc8 | 147138 | 4-May-15 |
| 21392 | 2 | 3 | | | III-1 | Tmco1 | 54499 | 4-May-15 |
| 21399 | 2 | 3 | | | III-1 | Tmed1 | 11018 | 4-May-15 |
| 21404 | 2 | 3 | | | III-1 | Tmed4 | 222068 | 12-May-15 |
| 21407 | 2 | 3 | | | III-1 | Tmed7 | 51014 | 4-May-15 |
| 21411 | 2 | 3 | | | III-1 | Tmeff2 | 23671 | 12-May-15 |
| 21413 | 2 | 3 | | | III-1 | Tmem101 | 84336 | 4-May-15 |
| 21415 | 2 | 3 | | | III-1 | Tmem104 | 54868 | 4-May-15 |
| 21417 | 2 | 3 | | | III-1 | Tmem106b | 54664 | 17-May-15 |
| 21418 | 2 | 3 | | | III-1 | Tmem106c | 79022 | 4-May-15 |
| 21420 | 2 | 3 | | | III-1 | Tmem108 | 66000 | 4-May-15 |
| 21431 | 2 | 3 | | | III-1 | Tmem123 | 114908 | 7-Jun-15 |
| 21433 | 2 | 3 | | | III-1 | Tmem126a | 84233 | 4-May-15 |
| 21434 | 2 | 3 | | | III-1 | Tmem126b | 55863 | 4-May-15 |
| 21441 | 2 | 3 | | | III-1 | Tmem132b | 114795 | 4-May-15 |
| 21447 | 2 | 3 | | | III-1 | Tmem135 | 65084 | 15-May-15 |
| 21451 | 2 | 3 | | | III-1 | Tmem140 | 55281 | 4-May-15 |
| 21453 | 2 | 3 | | | III-1 | Tmem143 | 55260 | 12-May-15 |
| 21455 | 2 | 3 | | | III-1 | Tmem145 | 284339 | 4-May-15 |
| 21460 | 2 | 3 | | | III-1 | Tmem150b | 284417 | 4-May-15 |
| 21464 | 2 | 3 | | | III-1 | Tmem151b | 441151 | 4-May-15 |
| 21466 | 2 | 3 | | | III-1 | Tmem158 | 25907 | 4-May-15 |
| 21468 | 2 | 3 | | | III-1 | Tmem160 | 54958 | 4-May-15 |
| 21473 | 2 | 3 | | | III-1 | Tmem165 | 55858 | 23-May-15 |
| 21479 | 2 | 3 | | | III-1 | Tmem170 | 124491 | 12-May-15 |
| 21480 | 2 | 3 | | | III-1 | Tmem170b | 100113407 | 4-May-15 |
| 21481 | 2 | 3 | | | III-1 | Tmem171 | 134285 | 4-May-15 |
| 21483 | 2 | 3 | | | III-1 | Tmem174 | 134288 | 4-May-15 |
| 21486 | 2 | 3 | | | III-1 | Tmem176b | 28959 | 4-May-15 |
| 21487 | 2 | 3 | | | III-1 | Tmem177 | 80775 | 12-May-15 |
| 21489 | 2 | 3 | | | III-1 | Tmem178b | 100507421 | 4-May-15 |
| 21492 | 2 | 3 | | | III-1 | Tmem18 | 129787 | 4-May-15 |
| 21494 | 2 | 3 | | | III-1 | Tmem181a | | |
| 21500 | 2 | 3 | | | III-1 | Tmem184b | 25829 | 21-May-15 |
| 21505 | 2 | 3 | | | III-1 | Tmem19 | 55266 | 4-May-15 |
| 21508 | 2 | 3 | | | III-1 | Tmem192 | 201931 | 4-May-15 |
| 21509 | 2 | 3 | | | III-1 | Tmem194 | 23306 | 28-May-15 |
| 21513 | 2 | 3 | | | III-1 | Tmem198b | 440104 | 4-May-15 |
| 21516 | 2 | 3 | | | III-1 | Tmem200a | 114801 | 4-May-15 |
| 21518 | 2 | 3 | | | III-1 | Tmem200c | 645369 | 4-May-15 |
| 21519 | 2 | 3 | | | III-1 | Tmem201 | 199953 | 4-May-15 |
| 21521 | 2 | 3 | | | III-1 | Tmem203 | 94107 | 28-May-15 |
| 21523 | 2 | 3 | | | III-1 | Tmem205 | 374882 | 4-May-15 |
| 21524 | 2 | 3 | | | III-1 | Tmem206 | 55248 | 4-May-15 |
| 21527 | 2 | 3 | | | III-1 | Tmem209 | 84928 | 21-May-15 |
| 21532 | 2 | 3 | | | III-1 | Tmem214 | 54867 | 4-May-15 |
| 21537 | 2 | 3 | | | III-1 | Tmem219 | 124446 | 4-May-15 |
| 21547 | 2 | 3 | | | III-1 | Tmem232 | 642987 | 4-May-15 |
| 21554 | 2 | 3 | | | III-1 | Tmem239 | 100288797 | 4-May-15 |
| 21556 | 2 | 3 | | | III-1 | Tmem241 | 85019 | 4-May-15 |
| 21557 | 2 | 3 | | | III-1 | Tmem242 | 729515 | 4-May-15 |
| 21560 | 2 | 3 | | | III-1 | Tmem246 | 84302 | 12-May-15 |
| 21561 | 2 | 3 | | | III-1 | Tmem247 | 388946 | 4-May-15 |
| 21566 | 2 | 3 | | | III-1 | Tmem253 | 643382 | 4-May-15 |
| 21567 | 2 | 3 | | | III-1 | Tmem254a | | |

Fig.22 - 84

| | | | | | | | | |
|---|---|---|---|---|---|---|---|---|
| 21571 | 2 | 3 | | | III-1 | Tmem255b | 348013 | 4-May-15 |
| 21579 | 2 | 3 | | | III-1 | Tmem27 | 57393 | 17-May-15 |
| 21584 | 2 | 3 | | | III-1 | Tmem30c | 644444 | 4-May-15 |
| 21590 | 2 | 3 | | | III-1 | Tmem39a | 55254 | 28-May-15 |
| 21593 | 2 | 3 | | | III-1 | Tmem41a | 90407 | 4-May-15 |
| 21594 | 2 | 3 | | | III-1 | Tmem41b | 440026 | 4-May-15 |
| 21601 | 2 | 3 | | | III-1 | Tmem5 | 10329 | 21-May-15 |
| 21602 | 2 | 3 | | | III-1 | Tmem50a | 23585 | 4-May-15 |
| 21603 | 2 | 3 | | | III-1 | Tmem50b | 757 | 4-May-15 |
| 21605 | 2 | 3 | | | III-1 | Tmem51os1 | | |
| 21609 | 2 | 3 | | | III-1 | Tmem54 | 113452 | 4-May-15 |
| 21613 | 2 | 3 | | | III-1 | Tmem57 | 55219 | 4-May-15 |
| 21622 | 2 | 3 | | | III-1 | Tmem65 | 157378 | 12-May-15 |
| 21626 | 2 | 3 | | | III-1 | Tmem69 | 51249 | 4-May-15 |
| 21629 | 2 | 3 | | | III-1 | Tmem72 | 643236 | 4-May-15 |
| 21632 | 2 | 3 | | | III-1 | Tmem79 | 84283 | 4-May-15 |
| 21634 | 2 | 3 | | | III-1 | Tmem80 | 283232 | 4-May-15 |
| 21637 | 2 | 3 | | | III-1 | Tmem86a | 144110 | 4-May-15 |
| 21639 | 2 | 3 | | | III-1 | Tmem87a | 25963 | 4-May-15 |
| 21644 | 2 | 3 | | | III-1 | Tmem8b | 51754 | 4-May-15 |
| 21645 | 2 | 3 | | | III-1 | Tmem8c | 389827 | 4-May-15 |
| 21648 | 2 | 3 | | | III-1 | Tmem92 | 162461 | 7-Jun-15 |
| 21652 | 2 | 3 | | | III-1 | Tmem9b | 56674 | 12-May-15 |
| 21657 | 2 | 3 | | | III-1 | Tmlhe | 55217 | 4-May-15 |
| 21660 | 2 | 3 | | | III-1 | Tmod3 | 29766 | 4-May-15 |
| 21665 | 2 | 3 | | | III-1 | Tmprss11bnl | 401136 | 4-May-15 |
| 21666 | 2 | 3 | | | III-1 | Tmprss11c | | |
| 21668 | 2 | 3 | | | III-1 | Tmprss11e | 28983 | 4-May-15 |
| 21671 | 2 | 3 | | | III-1 | Tmprss12 | 283471 | 4-May-15 |
| 21675 | 2 | 3 | | | III-1 | Tmprss3 | 64699 | 7-Jun-15 |
| 21678 | 2 | 3 | | | III-1 | Tmprss6 | 164656 | 4-May-15 |
| 21687 | 2 | 3 | | | III-1 | Tmtc1 | 83857 | 4-May-15 |
| 21688 | 2 | 3 | | | III-1 | Tmtc2 | 160335 | 4-May-15 |
| 21689 | 2 | 3 | | | III-1 | Tmtc3 | 160418 | 4-May-15 |
| 21691 | 2 | 3 | | | III-1 | Tmub1 | 83590 | 29-May-15 |
| 21694 | 2 | 3 | | | III-1 | Tmx2 | 51075 | 4-May-15 |
| 21696 | 2 | 3 | | | III-1 | Tmx4 | 56255 | 4-May-15 |
| 21699 | 2 | 3 | | | III-1 | Tnfaip1 | 7126 | 4-May-15 |
| 21703 | 2 | 3 | | | III-1 | Tnfaip8 | 25816 | 4-May-15 |
| 21709 | 2 | 3 | | | III-1 | Tnfrsf11b | 4982 | 7-Jun-15 |
| 21717 | 2 | 3 | | | III-1 | Tnfrsf1a | 7132 | 14-May-15 |
| 21720 | 2 | 3 | | | III-1 | Tnfrsf22 | | |
| 21725 | 2 | 3 | | | III-1 | Tnfrsf8 | 943 | 4-May-15 |
| 21727 | 2 | 3 | | | III-1 | Tnfsf10 | 8743 | 24-May-15 |
| 21728 | 2 | 3 | | | III-1 | Tnfsf11 | 8600 | 17-May-15 |
| 21729 | 2 | 3 | | | III-1 | Tnfsf12 | 8742 | 4-May-15 |
| 21730 | 2 | 3 | | | III-1 | Tnfsf12Tnfsf13 | 407977 | 4-May-15 |
| 21734 | 2 | 3 | | | III-1 | Tnfsf15 | 9966 | 12-May-15 |
| 21739 | 2 | 3 | | | III-1 | Tnik | 23043 | 4-May-15 |
| 21740 | 2 | 3 | | | III-1 | Tnip1 | 10318 | 21-May-15 |
| 21741 | 2 | 3 | | | III-1 | Tnip2 | 79155 | 4-May-15 |
| 21742 | 2 | 3 | | | III-1 | Tnip3 | 79931 | 12-May-15 |
| 21744 | 2 | 3 | | | III-1 | Tnk2 | 10188 | 21-May-15 |
| 21745 | 2 | 3 | | | III-1 | Tnk2os | | |
| 21748 | 2 | 3 | | | III-1 | Tnks2 | 80351 | 12-May-15 |
| 21756 | 2 | 3 | | | III-1 | Tnni3k | 51086 | 4-May-15 |
| 21768 | 2 | 3 | | | III-1 | Tnrc6b | 23112 | 4-May-15 |
| 21769 | 2 | 3 | | | III-1 | Tnrc6c | 57690 | 12-May-15 |
| 21775 | 2 | 3 | | | III-1 | Tob2 | 10766 | 12-May-15 |
| 21776 | 2 | 3 | | | III-1 | Toe1 | 114034 | 4-May-15 |
| 21777 | 2 | 3 | | | III-1 | Tollip | 54472 | 4-May-15 |
| 21780 | 2 | 3 | | | III-1 | Tom1l2 | 146691 | 4-May-15 |
| 21784 | 2 | 3 | | | III-1 | Tomm34 | 10953 | 4-May-15 |
| 21787 | 2 | 3 | | | III-1 | Tomm5 | 401505 | 12-May-15 |
| 21788 | 2 | 3 | | | III-1 | Tomm6 | 100188 893 | 4-May-15 |
| 21789 | 2 | 3 | | | III-1 | Tomm6os | | |
| 21790 | 2 | 3 | | | III-1 | Tomm7 | 54543 | 4-May-15 |
| 21791 | 2 | 3 | | | III-1 | Tomm70a | 9868 | 24-May-15 |
| 21796 | 2 | 3 | | | III-1 | Top2a | 7153 | 12-May-15 |
| 21799 | 2 | 3 | | | III-1 | Top3b | 8940 | 4-May-15 |
| 21802 | 2 | 3 | | | III-1 | Topors | 10210 | 23-May-15 |
| 21807 | 2 | 3 | | | III-1 | Tor1aip2 | 163590 | 12-May-15 |
| 21812 | 2 | 3 | | | III-1 | Tox | 9760 | 31-May-15 |
| 21813 | 2 | 3 | | | III-1 | Tox2 | 84969 | 4-May-15 |
| 21820 | 2 | 3 | | | III-1 | Tpcn2 | 219931 | 29-May-15 |
| 21823 | 2 | 3 | | | III-1 | Tpd52l2 | 7165 | 4-May-15 |
| 21825 | 2 | 3 | | | III-1 | Tpgs2 | 25941 | 4-May-15 |
| 21827 | 2 | 3 | | | III-1 | Tph2 | 121278 | 7-Jun-15 |
| 21829 | 2 | 3 | | | III-1 | Tpk1 | 27010 | 4-May-15 |
| 21836 | 2 | 3 | | | III-1 | Tpp1 | 1200 | 7-Jun-15 |
| 21840 | 2 | 3 | | | III-1 | Tppp3 | 51673 | 4-May-15 |
| 21842 | 2 | 3 | | | III-1 | Tpra1 | 131601 | 4-May-15 |
| 21844 | 2 | 3 | | | III-1 | Tprgl | | |
| 21849 | 2 | 3 | | | III-1 | Tpsg1 | 25823 | 12-May-15 |
| 21852 | 2 | 3 | | | III-1 | Tpt1 | 7178 | 4-May-15 |
| 21853 | 2 | 3 | | | III-1 | Tpte | 7179 | 12-May-15 |
| 21858 | 2 | 3 | | | III-1 | Trabd2b | 388630 | 4-May-15 |
| 21861 | 2 | 3 | | | III-1 | Traf2 | 7186 | 7-Jun-15 |
| 21865 | 2 | 3 | | | III-1 | Traf3ip3 | 80342 | 4-May-15 |
| 21867 | 2 | 3 | | | III-1 | Traf5 | 7188 | 4-May-15 |
| 21868 | 2 | 3 | | | III-1 | Traf6 | 7189 | 23-May-15 |
| 21872 | 2 | 3 | | | III-1 | Trak1 | 22906 | 4-May-15 |
| 21874 | 2 | 3 | | | III-1 | Tram1 | 23471 | 7-Jun-15 |
| 21887 | 2 | 3 | | | III-1 | Trappc3 | 27095 | 2-Jun-15 |
| 21890 | 2 | 3 | | | III-1 | Trappc5 | 126003 | 4-May-15 |
| 21893 | 2 | 3 | | | III-1 | Trappc8 | 22878 | 21-May-15 |
| 21896 | 2 | 3 | | | III-1 | Trcg1 | | |
| 21900 | 2 | 3 | | | III-1 | Trem1 | 54210 | 28-May-15 |
| 21903 | 2 | 3 | | | III-1 | Treml1 | 340205 | 4-May-15 |
| 21910 | 2 | 3 | | | III-1 | Trh | 7200 | 4-May-15 |
| 21916 | 2 | 3 | | | III-1 | Trib2 | 28951 | 4-May-15 |
| 21918 | 2 | 3 | | | III-1 | Tril | 9865 | 12-May-15 |
| 21922 | 2 | 3 | | | III-1 | Trim12c | | |
| 21926 | 2 | 3 | | | III-1 | Trim16 | 10626 | 4-May-15 |
| 21927 | 2 | 3 | | | III-1 | Trim17 | 51127 | 4-May-15 |
| 21929 | 2 | 3 | | | III-1 | Trim21 | 6737 | 24-May-15 |
| 21932 | 2 | 3 | | | III-1 | Trim25 | 7706 | 4-May-15 |
| 21937 | 2 | 3 | | | III-1 | Trim3 | 10612 | 17-May-15 |
| 21938 | 2 | 3 | | | III-1 | Trim30a | | |
| 21939 | 2 | 3 | | | III-1 | Trim30b | | |
| 21941 | 2 | 3 | | | III-1 | Trim30e-ps1 | | |
| 21949 | 2 | 3 | | | III-1 | Trim37 | 4591 | 4-May-15 |
| 21961 | 2 | 3 | | | III-1 | Trim47 | 91107 | 4-May-15 |
| 21962 | 2 | 3 | | | III-1 | Trim50 | 135892 | 4-May-15 |
| 21975 | 2 | 3 | | | III-1 | Trim66 | 9866 | 4-May-15 |
| 21978 | 2 | 3 | | | III-1 | Trim69 | 140691 | 12-May-15 |
| 21984 | 2 | 3 | | | III-1 | Trim9 | 114088 | 4-May-15 |
| 21995 | 2 | 3 | | | III-1 | Triqk | 286144 | 4-May-15 |
| 21996 | 2 | 3 | | | III-1 | Trit1 | 54802 | 4-May-15 |
| 21997 | 2 | 3 | | | III-1 | Trmt1 | 55621 | 7-Jun-15 |
| 21998 | 2 | 3 | | | III-1 | Trmt10a | 93587 | 4-May-15 |
| 21999 | 2 | 3 | | | III-1 | Trmt10b | 158234 | 23-May-15 |
| 22000 | 2 | 3 | | | III-1 | Trmt10c | 54931 | 4-May-15 |
| 22001 | 2 | 3 | | | III-1 | Trmt11 | 60487 | 4-May-15 |
| 22002 | 2 | 3 | | | III-1 | Trmt112 | 51504 | 4-May-15 |
| 22003 | 2 | 3 | | | III-1 | Trmt12 | 55039 | 4-May-15 |
| 22005 | 2 | 3 | | | III-1 | Trmt1l | 81627 | 4-May-15 |
| 22007 | 2 | 3 | | | III-1 | Trmt2b | 79979 | 4-May-15 |
| 22011 | 2 | 3 | | | III-1 | Trmt61a | 115708 | 4-May-15 |
| 22014 | 2 | 3 | | | III-1 | Trnau1ap | 54952 | 4-May-15 |
| 22016 | 2 | 3 | | | III-1 | Trnt1 | 51095 | 7-Jun-15 |
| 22017 | 2 | 3 | | | III-1 | Tro | 7216 | 4-May-15 |
| 22020 | 2 | 3 | | | III-1 | Trp53 | 7157 | 31-May-15 |
| 22023 | 2 | 3 | | | III-1 | Trp53cor1 | 102800 311 | 23-May-15 |
| 22026 | 2 | 3 | | | III-1 | Trp53inp1 | | |
| 22027 | 2 | 3 | | | III-1 | Trp53inp2 | | |
| 22029 | 2 | 3 | | | III-1 | Trp53tg5 | | |
| 22031 | 2 | 3 | | | III-1 | Trp73 | | |
| 22034 | 2 | 3 | | | III-1 | Trpc2 | 7221 | 12-May-15 |
| 22035 | 2 | 3 | | | III-1 | Trpc3 | 7222 | 10-May-15 |
| 22036 | 2 | 3 | | | III-1 | Trpc4 | 7223 | 12-May-15 |
| 22040 | 2 | 3 | | | III-1 | Trpc6 | 7225 | 24-May-15 |
| 22048 | 2 | 3 | | | III-1 | Trpm6 | 140803 | 4-May-15 |
| 22059 | 2 | 3 | | | III-1 | Trrap | 8295 | 4-May-15 |
| 22067 | 2 | 3 | | | III-1 | Tsc2 | 7249 | 12-May-15 |
| 22071 | 2 | 3 | | | III-1 | Tsc22d4 | 81628 | 12-May-15 |
| 22073 | 2 | 3 | | | III-1 | Tsen2 | 80746 | 23-May-15 |
| 22074 | 2 | 3 | | | III-1 | Tsen34 | 79042 | 23-May-15 |
| 22078 | 2 | 3 | | | III-1 | Tsga10 | 80705 | 21-May-15 |
| 22080 | 2 | 3 | | | III-1 | Tsga8 | | |
| 22084 | 2 | 3 | | | III-1 | Tshz2 | 128553 | 4-May-15 |
| 22094 | 2 | 3 | | | III-1 | Tspan10 | 83882 | 14-May-15 |
| 22097 | 2 | 3 | | | III-1 | Tspan13 | 27075 | 12-May-15 |
| 22098 | 2 | 3 | | | III-1 | Tspan14 | 81619 | 4-May-15 |
| 22100 | 2 | 3 | | | III-1 | Tspan17 | 26262 | 4-May-15 |
| 22103 | 2 | 3 | | | III-1 | Tspan2os | | |
| 22105 | 2 | 3 | | | III-1 | Tspan31 | 6302 | 20-May-15 |
| 22109 | 2 | 3 | | | III-1 | Tspan5 | 10098 | 4-May-15 |
| 22113 | 2 | 3 | | | III-1 | Tspan9 | 10867 | 4-May-15 |
| 22114 | 2 | 3 | | | III-1 | Tspear | 54084 | 7-Jun-15 |
| 22119 | 2 | 3 | | | III-1 | Tspyl3 | 128854 | 4-May-15 |
| 22121 | 2 | 3 | | | III-1 | Tspyl5 | 85453 | 4-May-15 |
| 22124 | 2 | 3 | | | III-1 | Tsr2 | 90121 | 4-May-15 |
| 22130 | 2 | 3 | | | III-1 | Tssk3 | 81629 | 4-May-15 |
| 22132 | 2 | 3 | | | III-1 | Tssk5 | 283629 | 12-May-15 |
| 22134 | 2 | 3 | | | III-1 | Tst | 7263 | 4-May-15 |
| 22135 | 2 | 3 | | | III-1 | Tsta3 | 7264 | 4-May-15 |
| 22136 | 2 | 3 | | | III-1 | Tstd1 | 100131 187 | 4-May-15 |
| 22137 | 2 | 3 | | | III-1 | Tstd2 | 158427 | 4-May-15 |
| 22140 | 2 | 3 | | | III-1 | Ttbk1 | 84630 | 12-May-15 |
| 22143 | 2 | 3 | | | III-1 | Ttc12 | 54970 | 12-May-15 |
| 22144 | 2 | 3 | | | III-1 | Ttc13 | 79573 | 4-May-15 |
| 22149 | 2 | 3 | | | III-1 | Ttc19 | 54902 | 4-May-15 |
| 22151 | 2 | 3 | | | III-1 | Ttc21b | 79809 | 22-May-15 |
| 22153 | 2 | 3 | | | III-1 | Ttc23 | 64927 | 4-May-15 |
| 22159 | 2 | 3 | | | III-1 | Ttc28 | 23331 | 12-May-15 |
| 22160 | 2 | 3 | | | III-1 | Ttc29 | 83894 | 4-May-15 |
| 22162 | 2 | 3 | | | III-1 | Ttc30a1 | | |
| 22164 | 2 | 3 | | | III-1 | Ttc30b | 150737 | 4-May-15 |
| 22165 | 2 | 3 | | | III-1 | Ttc32 | 130502 | 12-May-15 |
| 22166 | 2 | 3 | | | III-1 | Ttc33 | 23548 | 4-May-15 |
| 22169 | 2 | 3 | | | III-1 | Ttc37 | 9652 | 4-May-15 |
| 22170 | 2 | 3 | | | III-1 | Ttc38 | 55020 | 4-May-15 |
| 22172 | 2 | 3 | | | III-1 | Ttc39b | 158219 | 4-May-15 |
| 22181 | 2 | 3 | | | III-1 | Ttc9b | 148014 | 4-May-15 |
| 22189 | 2 | 3 | | | III-1 | Ttll1 | 25809 | 4-May-15 |
| 22191 | 2 | 3 | | | III-1 | Ttll11 | 158135 | 4-May-15 |
| 22192 | 2 | 3 | | | III-1 | Ttll12 | 23170 | 4-May-15 |
| 22193 | 2 | 3 | | | III-1 | Ttll13 | 440307 | 4-May-15 |
| 22195 | 2 | 3 | | | III-1 | Ttll3 | 26140 | 23-May-15 |
| 22198 | 2 | 3 | | | III-1 | Ttll6 | 284076 | 12-May-15 |
| 22204 | 2 | 3 | | | III-1 | Ttpal | 79183 | 4-May-15 |
| 22207 | 2 | 3 | | | III-1 | Ttyh2 | 94015 | 12-May-15 |

Fig.22 - 85

| | | | | | | | | |
|---|---|---|---|---|---|---|---|---|
| 22208 | 2 | 3 | | | III-1 | Ttyh3 | 80727 | 4-May-15 |
| 22209 | 2 | 3 | | | III-1 | Tub | 7275 | 28-May-15 |
| 22212 | 2 | 3 | | | III-1 | Tuba1c | 84790 | 12-May-15 |
| 22214 | 2 | 3 | | | III-1 | Tuba3b | | |
| 22223 | 2 | 3 | | | III-1 | Tubb4a | 10382 | 12-May-15 |
| 22224 | 2 | 3 | | | III-1 | Tubb4b | 10383 | 21-May-15 |
| 22227 | 2 | 3 | | | III-1 | Tubd1 | 51174 | 4-May-15 |
| 22229 | 2 | 3 | | | III-1 | Tubg1 | 7283 | 31-May-15 |
| 22231 | 2 | 3 | | | III-1 | Tubgcp2 | 10844 | 4-May-15 |
| 22236 | 2 | 3 | | | III-1 | Tufm | 7284 | 4-May-15 |
| 22240 | 2 | 3 | | | III-1 | Tulp2 | 7288 | 28-May-15 |
| 22245 | 2 | 3 | | | III-1 | Tusc2 | 11334 | 4-May-15 |
| 22251 | 2 | 3 | | | III-1 | Twf1 | 5756 | 3-May-15 |
| 22253 | 2 | 3 | | | III-1 | Twist1 | 7291 | 28-May-15 |
| 22254 | 2 | 3 | | | III-1 | Twist2 | 117581 | 4-May-15 |
| 22256 | 2 | 3 | | | III-1 | Twsg1 | 57045 | 4-May-15 |
| 22258 | 2 | 3 | | | III-1 | Txlna | 200081 | 28-May-15 |
| 22262 | 2 | 3 | | | III-1 | Txn2 | 25828 | 12-May-15 |
| 22263 | 2 | 3 | | | III-1 | Txndc11 | 51061 | 12-May-15 |
| 22264 | 2 | 3 | | | III-1 | Txndc12 | 51060 | 4-May-15 |
| 22265 | 2 | 3 | | | III-1 | Txndc15 | 79770 | 4-May-15 |
| 22267 | 2 | 3 | | | III-1 | Txndc17 | 84817 | 4-May-15 |
| 22269 | 2 | 3 | | | III-1 | Txndc5 | 81567 | 31-May-15 |
| 22273 | 2 | 3 | | | III-1 | Txnl1 | 9352 | 12-May-15 |
| 22274 | 2 | 3 | | | III-1 | Txnl4a | 10907 | 2-Jun-15 |
| 22278 | 2 | 3 | | | III-1 | Txnrd3 | 114112 | 12-May-15 |
| 22279 | 2 | 3 | | | III-1 | Tyk2 | 7297 | 4-May-15 |
| 22291 | 2 | 3 | | | III-1 | U2af1 | 7307 | 2-Jun-15 |
| 22292 | 2 | 3 | | | III-1 | U2af1l4 | 199746 | 7-Jun-15 |
| 22299 | 2 | 3 | | | III-1 | Uba1 | 7317 | 31-May-15 |
| 22303 | 2 | 3 | | | III-1 | Uba5 | 79876 | 4-May-15 |
| 22305 | 2 | 3 | | | III-1 | Uba6 | 55236 | 4-May-15 |
| 22306 | 2 | 3 | | | III-1 | Uba7 | 7318 | 4-May-15 |
| 22308 | 2 | 3 | | | III-1 | Ubac2 | 337867 | 3-May-15 |
| 22315 | 2 | 3 | | | III-1 | Ubash3a | 53347 | 24-May-15 |
| 22322 | 2 | 3 | | | III-1 | Ube2c | 11065 | 4-May-15 |
| 22324 | 2 | 3 | | | III-1 | Ube2d1 | 7321 | 31-May-15 |
| 22325 | 2 | 3 | | | III-1 | Ube2d2a | | |
| 22339 | 2 | 3 | | | III-1 | Ube2j2 | 118424 | 29-May-15 |
| 22340 | 2 | 3 | | | III-1 | Ube2k | 3093 | 2-Jun-15 |
| 22343 | 2 | 3 | | | III-1 | Ube2m | 9040 | 4-May-15 |
| 22346 | 2 | 3 | | | III-1 | Ube2q1 | 55585 | 2-Jun-15 |
| 22347 | 2 | 3 | | | III-1 | Ube2q2 | 92912 | 12-May-15 |
| 22349 | 2 | 3 | | | III-1 | Ube2r2 | 54926 | 12-May-15 |
| 22350 | 2 | 3 | | | III-1 | Ube2s | 27338 | 4-May-15 |
| 22352 | 2 | 3 | | | III-1 | Ube2u | 148581 | 4-May-15 |
| 22357 | 2 | 3 | | | III-1 | Ube3a | 7337 | 23-May-15 |
| 22359 | 2 | 3 | | | III-1 | Ube3c | 9690 | 4-May-15 |
| 22361 | 2 | 3 | | | III-1 | Ube4b | 10277 | 4-May-15 |
| 22367 | 2 | 3 | | | III-1 | Ubl5 | 59286 | 7-Jun-15 |
| 22368 | 2 | 3 | | | III-1 | Ubl7 | 84993 | 12-May-15 |
| 22377 | 2 | 3 | | | III-1 | Ubqln4 | 56893 | 21-May-15 |
| 22378 | 2 | 3 | | | III-1 | Ubqlnl | 143630 | 4-May-15 |
| 22386 | 2 | 3 | | | III-1 | Ubtd2 | 92181 | 4-May-15 |
| 22391 | 2 | 3 | | | III-1 | Ubxn11 | 91544 | 4-May-15 |
| 22392 | 2 | 3 | | | III-1 | Ubxn2a | 165324 | 21-May-15 |
| 22393 | 2 | 3 | | | III-1 | Ubxn2b | 137886 | 21-May-15 |
| 22394 | 2 | 3 | | | III-1 | Ubxn4 | 23190 | 4-May-15 |
| 22396 | 2 | 3 | | | III-1 | Ubxn7 | 26043 | 4-May-15 |
| 22401 | 2 | 3 | | | III-1 | Uchl4 | | |
| 22402 | 2 | 3 | | | III-1 | Uchl5 | 51377 | 4-May-15 |
| 22403 | 2 | 3 | | | III-1 | Uck1 | 83549 | 13-Jun-15 |
| 22410 | 2 | 3 | | | III-1 | Ucn3 | 114131 | 7-Jun-15 |
| 22416 | 2 | 3 | | | III-1 | Ufd1l | 7353 | 28-May-15 |
| 22417 | 2 | 3 | | | III-1 | Ufl1 | 23376 | 23-May-15 |
| 22420 | 2 | 3 | | | III-1 | Ufsp2 | 55325 | 4-May-15 |
| 22423 | 2 | 3 | | | III-1 | Uggt1 | 56886 | 4-May-15 |
| 22434 | 2 | 3 | | | III-1 | Ugt2a1 | 10941 | 12-May-15 |
| 22439 | 2 | 3 | | | III-1 | Ugt2b35 | | |
| 22441 | 2 | 3 | | | III-1 | Ugt2b37 | | |
| 22451 | 2 | 3 | | | III-1 | Uhrf2 | 115426 | 4-May-15 |
| 22459 | 2 | 3 | | | III-1 | Umodl1 | 89766 | 4-May-15 |
| 22461 | 2 | 3 | | | III-1 | Unc119 | 9094 | 4-May-15 |
| 22463 | 2 | 3 | | | III-1 | Unc13a | 23025 | 17-May-15 |
| 22465 | 2 | 3 | | | III-1 | Unc13c | 440279 | 12-May-15 |
| 22471 | 2 | 3 | | | III-1 | Unc5b | 219699 | 4-May-15 |
| 22472 | 2 | 3 | | | III-1 | Unc5c | 8633 | 4-May-15 |
| 22474 | 2 | 3 | | | III-1 | Unc5d | 137970 | 4-May-15 |
| 22478 | 2 | 3 | | | III-1 | Unc93b1 | 81622 | 4-May-15 |
| 22479 | 2 | 3 | | | III-1 | Uncx | 340260 | 21-May-15 |
| 22485 | 2 | 3 | | | III-1 | Upf1 | 5976 | 2-Jun-15 |
| 22491 | 2 | 3 | | | III-1 | Upk2 | 7379 | 4-May-15 |
| 22500 | 2 | 3 | | | III-1 | Uqcr10 | 29796 | 4-May-15 |
| 22501 | 2 | 3 | | | III-1 | Uqcr11 | 10975 | 4-May-15 |
| 22502 | 2 | 3 | | | III-1 | Uqcrb | 7381 | 12-May-15 |
| 22503 | 2 | 3 | | | III-1 | Uqcrc1 | 7384 | 4-May-15 |
| 22504 | 2 | 3 | | | III-1 | Uqcrc2 | 7385 | 4-May-15 |
| 22505 | 2 | 3 | | | III-1 | Uqcrfs1 | 7386 | 4-May-15 |
| 22506 | 2 | 3 | | | III-1 | Uqcrh | 7388 | 4-May-15 |
| 22507 | 2 | 3 | | | III-1 | Uqcrq | 27089 | 4-May-15 |
| 22508 | 2 | 3 | | | III-1 | Urad | 646625 | 4-May-15 |
| 22509 | 2 | 3 | | | III-1 | Urah | 100130015 | 4-May-15 |
| 22510 | 2 | 3 | | | III-1 | Urb1 | 9875 | 12-May-15 |
| 22511 | 2 | 3 | | | III-1 | Urb2 | 9816 | 4-May-15 |
| 22512 | 2 | 3 | | | III-1 | Urgcp | 55665 | 12-May-15 |
| 22514 | 2 | 3 | | | III-1 | Urm1 | 81605 | 24-May-15 |
| 22517 | 2 | 3 | | | III-1 | Uros | 7390 | 4-May-15 |

| | | | | | | | | |
|---|---|---|---|---|---|---|---|---|
| 22518 | 2 | 3 | | | III-1 | Usb1 | 79650 | 23-May-15 |
| 22522 | 2 | 3 | | | III-1 | Ush1c | 10083 | 23-May-15 |
| 22531 | 2 | 3 | | | III-1 | Usp12 | 219333 | 7-Jun-15 |
| 22542 | 2 | 3 | | | III-1 | Usp19 | 10869 | 28-May-15 |
| 22545 | 2 | 3 | | | III-1 | Usp21 | 27005 | 7-Jun-15 |
| 22552 | 2 | 3 | | | III-1 | Usp29 | 57663 | 3-May-15 |
| 22556 | 2 | 3 | | | III-1 | Usp32 | 84669 | 4-May-15 |
| 22560 | 2 | 3 | | | III-1 | Usp36 | 57602 | 12-May-15 |
| 22562 | 2 | 3 | | | III-1 | Usp38 | 84640 | 12-May-15 |
| 22571 | 2 | 3 | | | III-1 | Usp47 | 55031 | 4-May-15 |
| 22574 | 2 | 3 | | | III-1 | Usp5 | 8078 | 29-May-15 |
| 22578 | 2 | 3 | | | III-1 | Usp54 | 159195 | 4-May-15 |
| 22579 | 2 | 3 | | | III-1 | Usp6nl | 9712 | 12-May-15 |
| 22580 | 2 | 3 | | | III-1 | Usp7 | 7874 | 31-May-15 |
| 22583 | 2 | 3 | | | III-1 | Usp9y | 8287 | 23-May-15 |
| 22586 | 2 | 3 | | | III-1 | Utf1 | 8433 | 4-May-15 |
| 22587 | 2 | 3 | | | III-1 | Utp11l | 51118 | 4-May-15 |
| 22588 | 2 | 3 | | | III-1 | Utp14a | 10813 | 4-May-15 |
| 22600 | 2 | 3 | | | III-1 | Uty | 7404 | 12-May-15 |
| 22602 | 2 | 3 | | | III-1 | Uvssa | 57654 | 21-May-15 |
| 22603 | 2 | 3 | | | III-1 | Uxs1 | 80146 | 12-May-15 |
| 22604 | 2 | 3 | | | III-1 | Uxt | 8409 | 4-May-15 |
| 22610 | 2 | 3 | | | III-1 | Vamp2 | 6844 | 4-May-15 |
| 22613 | 2 | 3 | | | III-1 | Vamp5 | 10791 | 4-May-15 |
| 22614 | 2 | 3 | | | III-1 | Vamp7 | 6845 | 21-May-15 |
| 22617 | 2 | 3 | | | III-1 | Vangl2 | 57216 | 4-May-15 |
| 22621 | 2 | 3 | | | III-1 | Vars2 | 57176 | 7-Jun-15 |
| 22624 | 2 | 3 | | | III-1 | Vasn | 114990 | 4-May-15 |
| 22625 | 2 | 3 | | | III-1 | Vasp | 7408 | 17-May-15 |
| 22628 | 2 | 3 | | | III-1 | Vaultrc5 | | |
| 22629 | 2 | 3 | | | III-1 | Vav1 | 7409 | 12-May-15 |
| 22631 | 2 | 3 | | | III-1 | Vav3 | 10451 | 4-May-15 |
| 22632 | 2 | 3 | | | III-1 | Vax1 | 11023 | 23-May-15 |
| 22643 | 2 | 3 | | | III-1 | Vdac2 | 7417 | 12-May-15 |
| 22646 | 2 | 3 | | | III-1 | Vegfa | 7422 | 31-May-15 |
| 22647 | 2 | 3 | | | III-1 | Vegfb | 7423 | 12-May-15 |
| 22648 | 2 | 3 | | | III-1 | Vegfc | 7424 | 4-May-15 |
| 22653 | 2 | 3 | | | III-1 | Vgll1 | 51442 | 7-Jun-15 |
| 22663 | 2 | 3 | | | III-1 | Vipas39 | 63894 | 4-May-15 |
| 22665 | 2 | 3 | | | III-1 | Vipr2 | 7434 | 12-May-15 |
| 22666 | 2 | 3 | | | III-1 | Vit | 5212 | 4-May-15 |
| 22667 | 2 | 3 | | | III-1 | Vkorc1 | 79001 | 17-May-15 |
| 22668 | 2 | 3 | | | III-1 | Vkorc1l1 | 154807 | 4-May-15 |
| 22670 | 2 | 3 | | | III-1 | Vma21 | 203547 | 7-Jun-15 |
| 22737 | 2 | 3 | | | III-1 | Vmn1r183 | | |
| 22923 | 2 | 3 | | | III-1 | Vmn2r3 | | |
| 22997 | 2 | 3 | | | III-1 | Vmn2r98 | | |
| 23000 | 2 | 3 | | | III-1 | Vmn2r-ps129 | | |
| 23005 | 2 | 3 | | | III-1 | Vmp1 | 81671 | 21-May-15 |
| 23008 | 2 | 3 | | | III-1 | Vopp1 | 81552 | 12-May-15 |
| 23009 | 2 | 3 | | | III-1 | Vprbp | 9730 | 4-May-15 |
| 23010 | 2 | 3 | | | III-1 | Vpreb1 | 7441 | 21-May-15 |
| 23011 | 2 | 3 | | | III-1 | Vpreb2 | 3543 | 12-May-15 |
| 23015 | 2 | 3 | | | III-1 | Vps13b | 157680 | 23-May-15 |
| 23017 | 2 | 3 | | | III-1 | Vps13d | 55187 | 12-May-15 |
| 23019 | 2 | 3 | | | III-1 | Vps18 | 57617 | 4-May-15 |
| 23024 | 2 | 3 | | | III-1 | Vps29 | 51699 | 4-May-15 |
| 23029 | 2 | 3 | | | III-1 | Vps37a | 137492 | 4-May-15 |
| 23031 | 2 | 3 | | | III-1 | Vps37c | 55048 | 4-May-15 |
| 23033 | 2 | 3 | | | III-1 | Vps39 | 23339 | 21-May-15 |
| 23047 | 2 | 3 | | | III-1 | Vrk3 | 51231 | 21-May-15 |
| 23051 | 2 | 3 | | | III-1 | Vsig10l | 147645 | 4-May-15 |
| 23053 | 2 | 3 | | | III-1 | Vsig4 | 11326 | 4-May-15 |
| 23057 | 2 | 3 | | | III-1 | Vstm2b | 342865 | 4-May-15 |
| 23059 | 2 | 3 | | | III-1 | Vstm4 | 196740 | 4-May-15 |
| 23061 | 2 | 3 | | | III-1 | Vsx1 | 30813 | 12-May-15 |
| 23070 | 2 | 3 | | | III-1 | Vwa3a | 146177 | 4-May-15 |
| 23071 | 2 | 3 | | | III-1 | Vwa5a | 4013 | 4-May-15 |
| 23082 | 2 | 3 | | | III-1 | Wac | 51322 | 12-May-15 |
| 23086 | 2 | 3 | | | III-1 | Wars2 | 10352 | 12-May-15 |
| 23087 | 2 | 3 | | | III-1 | Was | 7454 | 23-May-15 |
| 23090 | 2 | 3 | | | III-1 | Wasf3 | 10810 | 12-May-15 |
| 23092 | 2 | 3 | | | III-1 | Wasl | 8976 | 4-May-15 |
| 23096 | 2 | 3 | | | III-1 | Wbp2 | 23558 | 12-May-15 |
| 23100 | 2 | 3 | | | III-1 | Wbscr16 | 81554 | 4-May-15 |
| 23101 | 2 | 3 | | | III-1 | Wbscr17 | 64409 | 4-May-15 |
| 23102 | 2 | 3 | | | III-1 | Wbscr22 | 114049 | 4-May-15 |
| 23104 | 2 | 3 | | | III-1 | Wbscr27 | 155368 | 3-May-15 |
| 23105 | 2 | 3 | | | III-1 | Wbscr28 | 135886 | 4-May-15 |
| 23109 | 2 | 3 | | | III-1 | Wdfy4 | 57705 | 4-May-15 |
| 23110 | 2 | 3 | | | III-1 | Wdhd1 | 11169 | 4-May-15 |
| 23111 | 2 | 3 | | | III-1 | Wdpcp | 51057 | 23-May-15 |
| 23112 | 2 | 3 | | | III-1 | Wdr1 | 9948 | 14-May-15 |
| 23115 | 2 | 3 | | | III-1 | Wdr13 | 64743 | 20-May-15 |
| 23118 | 2 | 3 | | | III-1 | Wdr18 | 57418 | 4-May-15 |
| 23122 | 2 | 3 | | | III-1 | Wdr24 | 84219 | 29-May-15 |
| 23124 | 2 | 3 | | | III-1 | Wdr26 | 80232 | 21-May-15 |
| 23127 | 2 | 3 | | | III-1 | Wdr31 | 114987 | 4-May-15 |
| 23130 | 2 | 3 | | | III-1 | Wdr35 | 57539 | 28-May-15 |
| 23131 | 2 | 3 | | | III-1 | Wdr36 | 134430 | 4-May-15 |
| 23137 | 2 | 3 | | | III-1 | Wdr44 | 54521 | 4-May-15 |
| 23138 | 2 | 3 | | | III-1 | Wdr45 | 11152 | 23-May-15 |
| 23139 | 2 | 3 | | | III-1 | Wdr45b | 56270 | 21-May-15 |
| 23141 | 2 | 3 | | | III-1 | Wdr47 | 22911 | 4-May-15 |
| 23145 | 2 | 3 | | | III-1 | Wdr53 | 348793 | 4-May-15 |
| 23146 | 2 | 3 | | | III-1 | Wdr54 | 84058 | 4-May-15 |
| 23147 | 2 | 3 | | | III-1 | Wdr55 | 54853 | 4-May-15 |

Fig.22 - 86

| | | | | | | | | |
|---|---|---|---|---|---|---|---|---|
| 23152 | 2 | 3 | | | III-1 | Wdr61 | 80349 | 12-May-15 |
| 23160 | 2 | 3 | | | III-1 | Wdr73 | 84942 | 20-May-15 |
| 23162 | 2 | 3 | | | III-1 | Wdr75 | 84128 | 3-May-15 |
| 23170 | 2 | 3 | | | III-1 | Wdr83os | 51398 | 4-May-15 |
| 23171 | 2 | 3 | | | III-1 | Wdr86 | 349136 | 21-May-15 |
| 23172 | 2 | 3 | | | III-1 | Wdr89 | 112840 | 4-May-15 |
| 23173 | 2 | 3 | | | III-1 | Wdr90 | 197335 | 4-May-15 |
| 23174 | 2 | 3 | | | III-1 | Wdr91 | 29062 | 12-May-15 |
| 23175 | 2 | 3 | | | III-1 | Wdr92 | 116143 | 4-May-15 |
| 23183 | 2 | 3 | | | III-1 | Wee2 | 494551 | 7-Jun-15 |
| 23189 | 2 | 3 | | | III-1 | Wfdc15a | | |
| 23195 | 2 | 3 | | | III-1 | Wfdc3 | 140686 | 4-May-15 |
| 23196 | 2 | 3 | | | III-1 | Wfdc5 | 149708 | 4-May-15 |
| 23201 | 2 | 3 | | | III-1 | Wfikkn1 | 117166 | 4-May-15 |
| 23203 | 2 | 3 | | | III-1 | Wfs1 | 7466 | 31-May-15 |
| 23204 | 2 | 3 | | | III-1 | Whamm | 123720 | 4-May-15 |
| 23206 | 2 | 3 | | | III-1 | Whsc1 | 7468 | 23-May-15 |
| 23209 | 2 | 3 | | | III-1 | Wif1 | 11197 | 4-May-15 |
| 23214 | 2 | 3 | | | III-1 | Wipi2 | 26100 | 21-May-15 |
| 23222 | 2 | 3 | | | III-1 | Wnk3 | 65267 | 4-May-15 |
| 23224 | 2 | 3 | | | III-1 | Wnt1 | 7471 | 17-May-15 |
| 23226 | 2 | 3 | | | III-1 | Wnt10b | 7480 | 4-May-15 |
| 23228 | 2 | 3 | | | III-1 | Wnt16 | 51384 | 7-Jun-15 |
| 23234 | 2 | 3 | | | III-1 | Wnt5a | 7474 | 31-May-15 |
| 23237 | 2 | 3 | | | III-1 | Wnt7a | 7476 | 4-May-15 |
| 23239 | 2 | 3 | | | III-1 | Wnt8a | 7478 | 7-Jun-15 |
| 23242 | 2 | 3 | | | III-1 | Wnt9b | 7484 | 4-May-15 |
| 23248 | 2 | 3 | | | III-1 | Wsb2 | 55884 | 2-Jun-15 |
| 23250 | 2 | 3 | | | III-1 | Wscd2 | 9671 | 12-May-15 |
| 23256 | 2 | 3 | | | III-1 | Wwc2 | 80014 | 4-May-15 |
| 23257 | 2 | 3 | | | III-1 | Wwox | 51741 | 12-May-15 |
| 23258 | 2 | 3 | | | III-1 | Wwp1 | 11059 | 4-May-15 |
| 23261 | 2 | 3 | | | III-1 | Xab2 | 56949 | 4-May-15 |
| 23267 | 2 | 3 | | | III-1 | Xiap | 331 | 23-May-15 |
| 23272 | 2 | 3 | | | III-1 | Xkr4 | 114786 | 4-May-15 |
| 23284 | 2 | 3 | | | III-1 | Xlr4b | | |
| 23292 | 2 | 3 | | | III-1 | Xpc | 7508 | 23-May-15 |
| 23294 | 2 | 3 | | | III-1 | Xpnpep2 | 7512 | 4-May-15 |
| 23296 | 2 | 3 | | | III-1 | Xpo1 | 7514 | 24-May-15 |
| 23299 | 2 | 3 | | | III-1 | Xpo6 | 23214 | 4-May-15 |
| 23301 | 2 | 3 | | | III-1 | Xpot | 11260 | 4-May-15 |
| 23303 | 2 | 3 | | | III-1 | Xrcc1 | 7515 | 31-May-15 |
| 23306 | 2 | 3 | | | III-1 | Xrcc4 | 7518 | 17-May-15 |
| 23308 | 2 | 3 | | | III-1 | Xrcc6 | 2547 | 24-May-15 |
| 23315 | 2 | 3 | | | III-1 | Xylt1 | 64131 | 23-May-15 |
| 23320 | 2 | 3 | | | III-1 | Yars | 8565 | 17-May-15 |
| 23326 | 2 | 3 | | | III-1 | Ydjc | 150223 | 4-May-15 |
| 23327 | 2 | 3 | | | III-1 | Yeats2 | 55689 | 4-May-15 |
| 23330 | 2 | 3 | | | III-1 | Yif1a | 10897 | 12-May-15 |
| 23333 | 2 | 3 | | | III-1 | Yipf2 | 78992 | 4-May-15 |
| 23334 | 2 | 3 | | | III-1 | Yipf3 | 25844 | 4-May-15 |
| 23341 | 2 | 3 | | | III-1 | Yme1l1 | 10730 | 12-May-15 |
| 23345 | 2 | 3 | | | III-1 | Ypel3 | 83719 | 4-May-15 |
| 23349 | 2 | 3 | | | III-1 | Ythdc1 | 91746 | 12-May-15 |
| 23364 | 2 | 3 | | | III-1 | Zan | 7455 | 12-May-15 |
| 23367 | 2 | 3 | | | III-1 | Zar1l | 646799 | 4-May-15 |
| 23370 | 2 | 3 | | | III-1 | Zbed4 | 9889 | 4-May-15 |
| 23374 | 2 | 3 | | | III-1 | Zbtb1 | 22890 | 4-May-15 |
| 23378 | 2 | 3 | | | III-1 | Zbtb14 | 7541 | 1-Jun-15 |
| 23382 | 2 | 3 | | | III-1 | Zbtb2 | 57621 | 3-May-15 |
| 23393 | 2 | 3 | | | III-1 | Zbtb37 | 84614 | 4-May-15 |
| 23396 | 2 | 3 | | | III-1 | Zbtb4 | 57659 | 4-May-15 |
| 23398 | 2 | 3 | | | III-1 | Zbtb41 | 360023 | 4-May-15 |
| 23400 | 2 | 3 | | | III-1 | Zbtb43 | 23099 | 4-May-15 |
| 23401 | 2 | 3 | | | III-1 | Zbtb44 | 29068 | 4-May-15 |
| 23410 | 2 | 3 | | | III-1 | Zbtb7c | 201501 | 12-May-15 |
| 23411 | 2 | 3 | | | III-1 | Zbtb8a | 653121 | 12-May-15 |
| 23419 | 2 | 3 | | | III-1 | Zc3h10 | 84872 | 12-May-15 |
| 23422 | 2 | 3 | | | III-1 | Zc3h12b | 340554 | 4-May-15 |
| 23424 | 2 | 3 | | | III-1 | Zc3h12d | 340152 | 4-May-15 |
| 23427 | 2 | 3 | | | III-1 | Zc3h15 | 55854 | 12-May-15 |
| 23430 | 2 | 3 | | | III-1 | Zc3h4 | 23211 | 4-May-15 |
| 23432 | 2 | 3 | | | III-1 | Zc3h7a | 29066 | 4-May-15 |
| 23435 | 2 | 3 | | | III-1 | Zc3hav1 | 56829 | 4-May-15 |
| 23441 | 2 | 3 | | | III-1 | Zcchc12 | 170261 | 4-May-15 |
| 23444 | 2 | 3 | | | III-1 | Zcchc16 | 340595 | 4-May-15 |
| 23447 | 2 | 3 | | | III-1 | Zcchc2 | 54877 | 4-May-15 |
| 23449 | 2 | 3 | | | III-1 | Zcchc3 | 85364 | 4-May-15 |
| 23457 | 2 | 3 | | | III-1 | Zcwpw1 | 55063 | 4-May-15 |
| 23460 | 2 | 3 | | | III-1 | Zdhhc11 | 79844 | 14-May-15 |
| 23462 | 2 | 3 | | | III-1 | Zdhhc13 | 54503 | 12-May-15 |
| 23463 | 2 | 3 | | | III-1 | Zdhhc14 | 79683 | 4-May-15 |
| 23466 | 2 | 3 | | | III-1 | Zdhhc17 | 23390 | 1-Jun-15 |
| 23471 | 2 | 3 | | | III-1 | Zdhhc21 | 340481 | 4-May-15 |
| 23486 | 2 | 3 | | | III-1 | Zer1 | 10444 | 4-May-15 |
| 23489 | 2 | 3 | | | III-1 | Zfand2a | 90637 | 4-May-15 |
| 23495 | 2 | 3 | | | III-1 | Zfa-ps | | |
| 23499 | 2 | 3 | | | III-1 | Zfhx2os | | |
| 23500 | 2 | 3 | | | III-1 | Zfhx3 | 463 | 12-May-15 |
| 23504 | 2 | 3 | | | III-1 | Zfp101 | | |
| 23506 | 2 | 3 | | | III-1 | Zfp106 | 64397 | 12-May-15 |
| 23508 | 2 | 3 | | | III-1 | Zfp109 | | |
| 23513 | 2 | 3 | | | III-1 | Zfp113 | 7551 | 12-May-15 |
| 23517 | 2 | 3 | | | III-1 | Zfp12 | | |
| 23521 | 2 | 3 | | | III-1 | Zfp131 | | |
| 23522 | 2 | 3 | | | III-1 | Zfp133-ps | | |
| 23525 | 2 | 3 | | | III-1 | Zfp143 | | |
| 23529 | 2 | 3 | | | III-1 | Zfp160 | | |
| 23536 | 2 | 3 | | | III-1 | Zfp189 | | |
| 23537 | 2 | 3 | | | III-1 | Zfp191 | 7572 | 12-May-15 |
| 23540 | 2 | 3 | | | III-1 | Zfp207 | | |
| 23550 | 2 | 3 | | | III-1 | Zfp251 | | |
| 23558 | 2 | 3 | | | III-1 | Zfp276 | 92822 | 4-May-15 |
| 23561 | 2 | 3 | | | III-1 | Zfp280b | | |
| 23562 | 2 | 3 | | | III-1 | Zfp280c | | |
| 23563 | 2 | 3 | | | III-1 | Zfp280d | | |
| 23569 | 2 | 3 | | | III-1 | Zfp296 | 162979 | 4-May-15 |
| 23571 | 2 | 3 | | | III-1 | Zfp30 | 22835 | 4-May-15 |
| 23572 | 2 | 3 | | | III-1 | Zfp300 | | |
| 23574 | 2 | 3 | | | III-1 | Zfp317 | | |
| 23576 | 2 | 3 | | | III-1 | Zfp319 | 57567 | 4-May-15 |
| 23579 | 2 | 3 | | | III-1 | Zfp326 | 284695 | 4-May-15 |
| 23583 | 2 | 3 | | | III-1 | Zfp335 | | |
| 23590 | 2 | 3 | | | III-1 | Zfp354b | | |
| 23591 | 2 | 3 | | | III-1 | Zfp354c | | |
| 23594 | 2 | 3 | | | III-1 | Zfp362 | | |
| 23601 | 2 | 3 | | | III-1 | Zfp36l3 | | |
| 23605 | 2 | 3 | | | III-1 | Zfp384 | | |
| 23611 | 2 | 3 | | | III-1 | Zfp39 | | |
| 23613 | 2 | 3 | | | III-1 | Zfp397 | | |
| 23617 | 2 | 3 | | | III-1 | Zfp408 | | |
| 23618 | 2 | 3 | | | III-1 | Zfp41 | 286128 | 12-May-15 |
| 23624 | 2 | 3 | | | III-1 | Zfp422 | 7570 | 4-May-15 |
| 23629 | 2 | 3 | | | III-1 | Zfp433 | | |
| 23634 | 2 | 3 | | | III-1 | Zfp446 | | |
| 23637 | 2 | 3 | | | III-1 | Zfp454 | | |
| 23645 | 2 | 3 | | | III-1 | Zfp467 | 168544 | 4-May-15 |
| 23646 | 2 | 3 | | | III-1 | Zfp472 | | |
| 23649 | 2 | 3 | | | III-1 | Zfp488 | | |
| 23652 | 2 | 3 | | | III-1 | Zfp507 | | |
| 23658 | 2 | 3 | | | III-1 | Zfp518a | | |
| 23661 | 2 | 3 | | | III-1 | Zfp521 | | |
| 23662 | 2 | 3 | | | III-1 | Zfp523 | 7629 | 4-May-15 |
| 23663 | 2 | 3 | | | III-1 | Zfp524 | | |
| 23664 | 2 | 3 | | | III-1 | Zfp526 | | |
| 23666 | 2 | 3 | | | III-1 | Zfp532 | | |
| 23667 | 2 | 3 | | | III-1 | Zfp534 | | |
| 23670 | 2 | 3 | | | III-1 | Zfp541 | | |
| 23673 | 2 | 3 | | | III-1 | Zfp558 | | |
| 23677 | 2 | 3 | | | III-1 | Zfp568 | | |
| 23679 | 2 | 3 | | | III-1 | Zfp572 | | |
| 23685 | 2 | 3 | | | III-1 | Zfp583 | | |
| 23689 | 2 | 3 | | | III-1 | Zfp595 | | |
| 23699 | 2 | 3 | | | III-1 | Zfp609 | | |
| 23700 | 2 | 3 | | | III-1 | Zfp61 | | |
| 23702 | 2 | 3 | | | III-1 | Zfp616 | | |
| 23705 | 2 | 3 | | | III-1 | Zfp619 | | |
| 23713 | 2 | 3 | | | III-1 | Zfp64 | 55734 | 4-May-15 |
| 23719 | 2 | 3 | | | III-1 | Zfp65 | | |
| 23723 | 2 | 3 | | | III-1 | Zfp653 | | |
| 23724 | 2 | 3 | | | III-1 | Zfp654 | | |
| 23732 | 2 | 3 | | | III-1 | Zfp672 | | |
| 23738 | 2 | 3 | | | III-1 | Zfp69 | 339559 | 4-May-15 |
| 23739 | 2 | 3 | | | III-1 | Zfp691 | 51058 | 4-May-15 |
| 23741 | 2 | 3 | | | III-1 | Zfp697 | | |
| 23742 | 2 | 3 | | | III-1 | Zfp7 | | |
| 23743 | 2 | 3 | | | III-1 | Zfp703 | | |
| 23745 | 2 | 3 | | | III-1 | Zfp706 | | |
| 23755 | 2 | 3 | | | III-1 | Zfp735 | | |
| 23761 | 2 | 3 | | | III-1 | Zfp748 | | |
| 23763 | 2 | 3 | | | III-1 | Zfp758 | | |
| 23769 | 2 | 3 | | | III-1 | Zfp770 | | |
| 23771 | 2 | 3 | | | III-1 | Zfp772 | | |
| 23772 | 2 | 3 | | | III-1 | Zfp773 | | |
| 23774 | 2 | 3 | | | III-1 | Zfp777 | | |
| 23779 | 2 | 3 | | | III-1 | Zfp784 | | |
| 23780 | 2 | 3 | | | III-1 | Zfp786 | | |
| 23783 | 2 | 3 | | | III-1 | Zfp790 | | |
| 23785 | 2 | 3 | | | III-1 | Zfp799 | | |
| 23790 | 2 | 3 | | | III-1 | Zfp809 | | |
| 23793 | 2 | 3 | | | III-1 | Zfp811 | | |
| 23794 | 2 | 3 | | | III-1 | Zfp819 | | |
| 23796 | 2 | 3 | | | III-1 | Zfp820 | | |
| 23800 | 2 | 3 | | | III-1 | Zfp830 | | |
| 23806 | 2 | 3 | | | III-1 | Zfp850 | | |
| 23809 | 2 | 3 | | | III-1 | Zfp865 | | |
| 23812 | 2 | 3 | | | III-1 | Zfp868 | | |
| 23820 | 2 | 3 | | | III-1 | Zfp874b | | |
| 23824 | 2 | 3 | | | III-1 | Zfp90 | 146198 | 20-May-15 |
| 23825 | 2 | 3 | | | III-1 | Zfp91 | 80829 | 4-May-15 |
| 23839 | 2 | 3 | | | III-1 | Zfp94 | | |
| 23841 | 2 | 3 | | | III-1 | Zfp941 | | |
| 23847 | 2 | 3 | | | III-1 | Zfp947 | | |
| 23848 | 2 | 3 | | | III-1 | Zfp948 | | |
| 23849 | 2 | 3 | | | III-1 | Zfp949 | | |
| 23854 | 2 | 3 | | | III-1 | Zfp955a | | |
| 23857 | 2 | 3 | | | III-1 | Zfp957 | | |
| 23859 | 2 | 3 | | | III-1 | Zfp959 | | |
| 23861 | 2 | 3 | | | III-1 | Zfp961 | | |
| 23864 | 2 | 3 | | | III-1 | Zfp97 | | |
| 23866 | 2 | 3 | | | III-1 | Zfpm1 | 161882 | 17-May-15 |
| 23867 | 2 | 3 | | | III-1 | Zfpm2 | 23414 | 4-May-15 |
| 23870 | 2 | 3 | | | III-1 | Zfx | 7543 | 31-May-15 |
| 23874 | 2 | 3 | | | III-1 | Zfyve16 | 9765 | 4-May-15 |

Fig.22 - 87

| | | | | | | | | |
|---|---|---|---|---|---|---|---|---|
| 23875 | 2 | 3 | | | III-1 | Zfyve19 | 84936 | 21-May-15 |
| 23878 | 2 | 3 | | | III-1 | Zfyve26 | 23503 | 4-May-15 |
| 23881 | 2 | 3 | | | III-1 | Zfyve9 | 9372 | 24-May-15 |
| 23889 | 2 | 3 | | | III-1 | Zic1 | 7545 | 2-Jun-15 |
| 23896 | 2 | 3 | | | III-1 | Zim3 | 114026 | 12-May-15 |
| 23909 | 2 | 3 | | | III-1 | Zmat2 | 153527 | 4-May-15 |
| 23912 | 2 | 3 | | | III-1 | Zmat5 | 55954 | 2-Jun-15 |
| 23913 | 2 | 3 | | | III-1 | Zmiz1 | 57178 | 17-May-15 |
| 23914 | 2 | 3 | | | III-1 | Zmiz2 | 83637 | 4-May-15 |
| 23923 | 2 | 3 | | | III-1 | Zmynd11 | 10771 | 4-May-15 |
| 23926 | 2 | 3 | | | III-1 | Zmynd19 | 116225 | 2-Jun-15 |
| 23934 | 2 | 3 | | | III-1 | Znhit6 | 54680 | 4-May-15 |
| 23937 | 2 | 3 | | | III-1 | Znrf1 | 84937 | 23-May-15 |
| 23941 | 2 | 3 | | | III-1 | Zp1 | 22917 | 12-May-15 |
| 23951 | 2 | 3 | | | III-1 | Zranb2 | 9406 | 2-Jun-15 |
| 23954 | 2 | 3 | | | III-1 | Zrsr2 | 8233 | 4-May-15 |
| 23958 | 2 | 3 | | | III-1 | Zscan2 | 54993 | 12-May-15 |
| 23959 | 2 | 3 | | | III-1 | Zscan20 | 7579 | 4-May-15 |
| 23962 | 2 | 3 | | | III-1 | Zscan25 | 221785 | 4-May-15 |
| 23963 | 2 | 3 | | | III-1 | Zscan26 | 7741 | 12-May-15 |
| 23979 | 2 | 3 | | | III-1 | Zswim8 | 23053 | 12-May-15 |
| 23984 | 2 | 3 | | | III-1 | Zxda | 7789 | 4-May-15 |
| 23989 | 2 | 3 | | | III-1 | Zyx | 7791 | 4-May-15 |
| 117 | 2 | | | | II-2 | 1700003P14Rik | | |
| 124 | 2 | | | | II-2 | 1700007G11Rik | | |
| 161 | 2 | | | | II-2 | 1700013D24Rik | | |
| 176 | 2 | | | | II-2 | 1700017B05Rik | | |
| 182 | 2 | | | | II-2 | 1700018B08Rik | | |
| 195 | 2 | | | | II-2 | 1700019L03Rik | | |
| 218 | 2 | | | | II-2 | 1700023E05Rik | | |
| 295 | 2 | | | | II-2 | 1700042G15Rik | | |
| 334 | 2 | | | | II-2 | 1700064J06Rik | | |
| 336 | 2 | | | | II-2 | 1700065D16Rik | | |
| 337 | 2 | | | | II-2 | 1700065I16Rik | | |
| 343 | 2 | | | | II-2 | 1700066B19Rik | | |
| 345 | 2 | | | | II-2 | 1700066N21Rik | | |
| 374 | 2 | | | | II-2 | 1700092E19Rik | | |
| 395 | 2 | | | | II-2 | 1700108F19Rik | | |
| 403 | 2 | | | | II-2 | 1700110I01Rik | | |
| 419 | 2 | | | | II-2 | 1700123I01Rik | | |
| 420 | 2 | | | | II-2 | 1700123K08Rik | | |
| 422 | 2 | | | | II-2 | 1700123M08Rik | | |
| 423 | 2 | | | | II-2 | 1700123O12Rik | | |
| 434 | 2 | | | | II-2 | 1700129C05Rik | | |
| 531 | 2 | | | | II-2 | 2310007B03Rik | | |
| 545 | 2 | | | | II-2 | 2310022B05Rik | | |
| 586 | 2 | | | | II-2 | 2410015M20Rik | | |
| 595 | 2 | | | | II-2 | 2410124H12Rik | | |
| 782 | 2 | | | | II-2 | 4921511C10Rik | | |
| 784 | 2 | | | | II-2 | 4921511H03Rik | | |
| 791 | 2 | | | | II-2 | 4921524L21Rik | | |
| 806 | 2 | | | | II-2 | 4930401O12Rik | | |
| 807 | 2 | | | | II-2 | 4930402F06Rik | | |
| 859 | 2 | | | | II-2 | 4930431P03Rik | | |
| 865 | 2 | | | | II-2 | 4930433N12Rik | | |
| 867 | 2 | | | | II-2 | 4930435E12Rik | | |
| 878 | 2 | | | | II-2 | 4930444P10Rik | | |
| 880 | 2 | | | | II-2 | 4930447C04Rik | | |
| 894 | 2 | | | | II-2 | 4930451G09Rik | | |
| 944 | 2 | | | | II-2 | 4930483O08Rik | | |
| 947 | 2 | | | | II-2 | 4930486L24Rik | | |
| 953 | 2 | | | | II-2 | 4930500J02Rik | | |
| 975 | 2 | | | | II-2 | 4930511E03Rik | | |
| 980 | 2 | | | | II-2 | 4930513O06Rik | | |
| 987 | 2 | | | | II-2 | 4930518P08Rik | | |
| 988 | 2 | | | | II-2 | 4930519D14Rik | | |
| 989 | 2 | | | | II-2 | 4930519F09Rik | | |
| 999 | 2 | | | | II-2 | 4930523C07Rik | | |
| 1025 | 2 | | | | II-2 | 4930539E08Rik | | |
| 1030 | 2 | | | | II-2 | 4930542C21Rik | | |
| 1031 | 2 | | | | II-2 | 4930542D17Rik | | |
| 1033 | 2 | | | | II-2 | 4930544D05Rik | | |
| 1042 | 2 | | | | II-2 | 4930547E14Rik | | |
| 1080 | 2 | | | | II-2 | 4930567H12Rik | | |
| 1085 | 2 | | | | II-2 | 4930568E12Rik | | |
| 1172 | 2 | | | | II-2 | 4933400L20Rik | | |
| 1175 | 2 | | | | II-2 | 4933401H06Rik | | |
| 1228 | 2 | | | | II-2 | 4933416M07Rik | | |
| 1241 | 2 | | | | II-2 | 4933425L06Rik | | |
| 1244 | 2 | | | | II-2 | 4933427D14Rik | | |
| 1246 | 2 | | | | II-2 | 4933427E13Rik | | |
| 1256 | 2 | | | | II-2 | 4933430N04Rik | | |
| 1262 | 2 | | | | II-2 | 4933432K03Rik | | |
| 1263 | 2 | | | | II-2 | 4933433C11Rik | | |
| 1265 | 2 | | | | II-2 | 4933433G08Rik | | |
| 1267 | 2 | | | | II-2 | 4933433G19Rik | | |
| 1274 | 2 | | | | II-2 | 4933438B17Rik | | |
| 1331 | 2 | | | | II-2 | 5730460C07Rik | | |
| 1361 | 2 | | | | II-2 | 6030466F02Rik | | |
| 1381 | 2 | | | | II-2 | 6430550D23Rik | | |
| 1392 | 2 | | | | II-2 | 6720483E21Rik | | |
| 1399 | 2 | | | | II-2 | 7420701I03Rik | | |
| 1402 | 2 | | | | II-2 | 8030411F24Rik | | |
| 1404 | 2 | | | | II-2 | 8030423J24Rik | | |
| 1406 | 2 | | | | II-2 | 8030443G20Rik | | |
| 1418 | 2 | | | | II-2 | 9030025P20Rik | | |
| 1419 | 2 | | | | II-2 | 9030204H09Rik | | |

| | | | | | | | | |
|---|---|---|---|---|---|---|---|---|
| 1420 | 2 | | | | II-2 | 9030404E10Rik | | |
| 1468 | 2 | | | | II-2 | 9330178D15Rik | | |
| 1476 | 2 | | | | II-2 | 9430015G10Rik | | |
| 1478 | 2 | | | | II-2 | 9430018G01Rik | | |
| 1511 | 2 | | | | II-2 | 9830132P13Rik | | |
| 1513 | 2 | | | | II-2 | 9830166K06Rik | | |
| 1529 | 2 | | | | II-2 | A230028O05Rik | | |
| 1571 | 2 | | | | II-2 | A430105I19Rik | | |
| 1587 | 2 | | | | II-2 | A530088E08Rik | | |
| 1642 | 2 | | | | II-2 | A930011O12Rik | | |
| 1643 | 2 | | | | II-2 | A930012L18Rik | | |
| 1665 | 2 | | | | II-2 | AA792892 | | |
| 1676 | 2 | | | | II-2 | Aak1 | 22848 | 31-May-15 |
| 1930 | 2 | | | | II-2 | Adam23 | 8745 | 4-May-15 |
| 2059 | 2 | | | | II-2 | AF357426 | | |
| 2060 | 2 | | | | II-2 | AF366264 | | |
| 2134 | 2 | | | | II-2 | AI317395 | | |
| 2368 | 2 | | | | II-2 | Angel2 | 90806 | 12-May-15 |
| 2369 | 2 | | | | II-2 | Angpt1 | 284 | 17-May-15 |
| 2832 | 2 | | | | II-2 | Asna1 | 439 | 4-May-15 |
| 2857 | 2 | | | | II-2 | Atad2 | 29028 | 4-May-15 |
| 3017 | 2 | | | | II-2 | AU019823 | | |
| 3019 | 2 | | | | II-2 | AU021063 | | |
| 3026 | 2 | | | | II-2 | AU040320 | | |
| 3076 | 2 | | | | II-2 | B020018J22Rik | | |
| 3077 | 2 | | | | II-2 | B020031M17Rik | | |
| 3078 | 2 | | | | II-2 | B130006D01Rik | | |
| 3096 | 2 | | | | II-2 | B2m | 567 | 17-May-15 |
| 3258 | 2 | | | | II-2 | BC048502 | | |
| 3305 | 2 | | | | II-2 | Bcap29 | 55973 | 4-May-15 |
| 3472 | 2 | | | | II-2 | Bpifb5 | | |
| 3473 | 2 | | | | II-2 | Bpifb6 | 128859 | 4-May-15 |
| 3592 | 2 | | | | II-2 | C130074G19Rik | | |
| 3593 | 2 | | | | II-2 | C130079G13Rik | | |
| 3677 | 2 | | | | II-2 | C87414 | | |
| 3678 | 2 | | | | II-2 | C87436 | | |
| 3679 | 2 | | | | II-2 | C87499 | | |
| 3680 | 2 | | | | II-2 | C87977 | | |
| 3749 | 2 | | | | II-2 | Cald1 | 800 | 4-May-15 |
| 3967 | 2 | | | | II-2 | Ccdc158 | 339965 | 4-May-15 |
| 3968 | 2 | | | | II-2 | Ccdc159 | 126075 | 4-May-15 |
| 4006 | 2 | | | | II-2 | Ccdc38 | 120935 | 28-May-15 |
| 4390 | 2 | | | | II-2 | Ceacam14 | | |
| 4392 | 2 | | | | II-2 | Ceacam16 | 388551 | |
| 4393 | 2 | | | | II-2 | Ceacam18 | 729767 | 4-May-15 |
| 4582 | 2 | | | | II-2 | Chml | 1122 | 4-May-15 |
| 4665 | 2 | | | | II-2 | Cir1 | 9541 | 7-Jun-15 |
| 4794 | 2 | | | | II-2 | Clp1 | 10978 | 16-Jun-15 |
| 4837 | 2 | | | | II-2 | Cmtm3 | 123920 | 4-May-15 |
| 4901 | 2 | | | | II-2 | Cntn6 | 27255 | 21-May-15 |
| 5220 | 2 | | | | II-2 | Csn1s2a | 286828 | 4-May-15 |
| 5222 | 2 | | | | II-2 | Csn2 | 1447 | 7-Jun-15 |
| 5223 | 2 | | | | II-2 | Csn3 | 1448 | 7-Jun-15 |
| 5224 | 2 | | | | II-2 | Csnk1a1 | 1452 | 4-May-15 |
| 5306 | 2 | | | | II-2 | Cts8 | | |
| 5307 | 2 | | | | II-2 | Cts8-ps | | |
| 5308 | 2 | | | | II-2 | Ctsa | 5476 | 12-May-15 |
| 5309 | 2 | | | | II-2 | Ctsb | 1508 | 31-May-15 |
| 5322 | 2 | | | | II-2 | Ctsq | | |
| 5323 | 2 | | | | II-2 | Ctsr | | |
| 5544 | 2 | | | | II-2 | D030028A08Rik | | |
| 5561 | 2 | | | | II-2 | D16Ertd472e | | |
| 5562 | 2 | | | | II-2 | D16Ertd519e | | |
| 5565 | 2 | | | | II-2 | D17Wsu104e | | |
| 5566 | 2 | | | | II-2 | D17Wsu92e | | |
| 5591 | 2 | | | | II-2 | D5Ertd605e | | |
| 5618 | 2 | | | | II-2 | D830026I12Rik | | |
| 5633 | 2 | | | | II-2 | Daam1 | 23002 | 14-May-15 |
| 5689 | 2 | | | | II-2 | Dcaf17 | 80067 | 23-May-15 |
| 5820 | 2 | | | | II-2 | Defa-rs7 | | |
| 6001 | 2 | | | | II-2 | Dlgap5 | 9787 | 4-May-15 |
| 6030 | 2 | | | | II-2 | Dmrta1 | 63951 | 4-May-15 |
| 6051 | 2 | | | | II-2 | Dnah5 | 1767 | 23-May-15 |
| 6056 | 2 | | | | II-2 | Dnah9 | 1770 | 12-May-15 |
| 6215 | 2 | | | | II-2 | Drd2 | 1813 | 31-May-15 |
| 6301 | 2 | | | | II-2 | DXBay18 | | |
| 6309 | 2 | | | | II-2 | Dync1i2 | 1781 | 1-Jun-15 |
| 6342 | 2 | | | | II-2 | E030024N20Rik | | |
| 6384 | 2 | | | | II-2 | E330017A01Rik | | |
| 6391 | 2 | | | | II-2 | E430016F16Rik | | |
| 6408 | 2 | | | | II-2 | Ears2 | 124454 | 12-May-15 |
| 6731 | 2 | | | | II-2 | Ephb1 | 2047 | 17-May-15 |
| 6821 | 2 | | | | II-2 | Esp18 | | |
| 6822 | 2 | | | | II-2 | Esp23 | | |
| 6825 | 2 | | | | II-2 | Esp31 | | |
| 6826 | 2 | | | | II-2 | Esp34 | | |
| 6827 | 2 | | | | II-2 | Esp36 | | |
| 6828 | 2 | | | | II-2 | Esp38 | | |
| 6829 | 2 | | | | II-2 | Esp4 | | |
| 6830 | 2 | | | | II-2 | Esp5 | | |
| 6831 | 2 | | | | II-2 | Esp6 | | |
| 6833 | 2 | | | | II-2 | Esp8 | | |
| 6886 | 2 | | | | II-2 | Exd2 | 55218 | 4-May-15 |
| 7251 | 2 | | | | II-2 | Fbf1 | 85302 | 4-May-15 |
| 7326 | 2 | | | | II-2 | Fbxw16 | | |
| 7327 | 2 | | | | II-2 | Fbxw17 | | |
| 7328 | 2 | | | | II-2 | Fbxw18 | | |

Fig.22 - 88

| | | | | | | | | |
|---|---|---|---|---|---|---|---|---|
| 7332 | 2 | | | | II-2 | Fbxw21 | | |
| 7334 | 2 | | | | II-2 | Fbxw24 | | |
| 7335 | 2 | | | | II-2 | Fbxw26 | | |
| 7336 | 2 | | | | II-2 | Fbxw28 | | |
| 7337 | 2 | | | | II-2 | Fbxw4 | 6468 | 4-May-15 |
| 7338 | 2 | | | | II-2 | Fbxw5 | 54461 | 4-May-15 |
| 7380 | 2 | | | | II-2 | Fermt1 | 55612 | 12-May-15 |
| 7571 | 2 | | | | II-2 | Foxi3 | 344167 | 12-May-15 |
| 7601 | 2 | | | | II-2 | Fpr1 | 2357 | 12-May-15 |
| 7654 | 2 | | | | II-2 | Ftl1 | | |
| 7813 | 2 | | | | II-2 | Gart | 2618 | 4-May-15 |
| 7961 | 2 | | | | II-2 | Gimap1 | 170575 | 4-May-15 |
| 8039 | 2 | | | | II-2 | Glra4 | 441509 | 4-May-15 |
| 8092 | 2 | | | | II-2 | Gm10272 | | |
| 8103 | 2 | | | | II-2 | Gm10377 | | |
| 8107 | 2 | | | | II-2 | Gm10406 | | |
| 8108 | 2 | | | | II-2 | Gm10408 | | |
| 8109 | 2 | | | | II-2 | Gm10409 | | |
| 8111 | 2 | | | | II-2 | Gm10415 | | |
| 8119 | 2 | | | | II-2 | Gm10445 | | |
| 8156 | 2 | | | | II-2 | Gm10670 | | |
| 8157 | 2 | | | | II-2 | Gm10677 | | |
| 8162 | 2 | | | | II-2 | Gm10731 | | |
| 8163 | 2 | | | | II-2 | Gm10745 | | |
| 8164 | 2 | | | | II-2 | Gm10754 | | |
| 8174 | 2 | | | | II-2 | Gm10804 | | |
| 8195 | 2 | | | | II-2 | Gm11213 | | |
| 8204 | 2 | | | | II-2 | Gm11426 | | |
| 8214 | 2 | | | | II-2 | Gm11548 | | |
| 8215 | 2 | | | | II-2 | Gm11549 | | |
| 8236 | 2 | | | | II-2 | Gm11762 | | |
| 8263 | 2 | | | | II-2 | Gm12338 | | |
| 8274 | 2 | | | | II-2 | Gm12669 | | |
| 8280 | 2 | | | | II-2 | Gm128 | | |
| 8281 | 2 | | | | II-2 | Gm12830 | | |
| 8285 | 2 | | | | II-2 | Gm12942 | | |
| 8296 | 2 | | | | II-2 | Gm13057 | | |
| 8297 | 2 | | | | II-2 | Gm13078 | | |
| 8299 | 2 | | | | II-2 | Gm13084 | | |
| 8300 | 2 | | | | II-2 | Gm13088 | | |
| 8301 | 2 | | | | II-2 | Gm13102 | | |
| 8302 | 2 | | | | II-2 | Gm13103 | | |
| 8303 | 2 | | | | II-2 | Gm13119 | | |
| 8318 | 2 | | | | II-2 | Gm13251 | | |
| 8324 | 2 | | | | II-2 | Gm13278 | | |
| 8329 | 2 | | | | II-2 | Gm13288 | | |
| 8332 | 2 | | | | II-2 | Gm13298 | | |
| 8346 | 2 | | | | II-2 | Gm13544 | | |
| 8374 | 2 | | | | II-2 | Gm14169 | | |
| 8383 | 2 | | | | II-2 | Gm14325 | | |
| 8389 | 2 | | | | II-2 | Gm14351 | | |
| 8424 | 2 | | | | II-2 | Gm14685 | | |
| 8426 | 2 | | | | II-2 | Gm14718 | | |
| 8428 | 2 | | | | II-2 | Gm14743 | | |
| 8431 | 2 | | | | II-2 | Gm14812 | | |
| 8510 | 2 | | | | II-2 | Gm16294 | | |
| 8511 | 2 | | | | II-2 | Gm1631 | | |
| 8512 | 2 | | | | II-2 | Gm16325 | | |
| 8513 | 2 | | | | II-2 | Gm16336 | | |
| 8517 | 2 | | | | II-2 | Gm16390 | | |
| 8575 | 2 | | | | II-2 | Gm17746 | | |
| 8583 | 2 | | | | II-2 | Gm1821 | | |
| 8584 | 2 | | | | II-2 | Gm18409 | | |
| 8587 | 2 | | | | II-2 | Gm19277 | | |
| 8590 | 2 | | | | II-2 | Gm19345 | | |
| 8592 | 2 | | | | II-2 | Gm19402 | | |
| 8628 | 2 | | | | II-2 | Gm20139 | | |
| 8632 | 2 | | | | II-2 | Gm20199 | | |
| 8633 | 2 | | | | II-2 | Gm2022 | | |
| 8645 | 2 | | | | II-2 | Gm20554 | | |
| 8647 | 2 | | | | II-2 | Gm20594 | | |
| 8655 | 2 | | | | II-2 | Gm20738 | | |
| 8656 | 2 | | | | II-2 | Gm20740 | | |
| 8666 | 2 | | | | II-2 | Gm20753 | | |
| 8672 | 2 | | | | II-2 | Gm20759 | | |
| 8674 | 2 | | | | II-2 | Gm20767 | | |
| 8675 | 2 | | | | II-2 | Gm20806 | | |
| 8676 | 2 | | | | II-2 | Gm20809 | | |
| 8677 | 2 | | | | II-2 | Gm20815 | | |
| 8681 | 2 | | | | II-2 | Gm20826 | | |
| 8708 | 2 | | | | II-2 | Gm21319 | | |
| 8721 | 2 | | | | II-2 | Gm21951 | | |
| 8726 | 2 | | | | II-2 | Gm2516 | | |
| 8735 | 2 | | | | II-2 | Gm2825 | | |
| 8796 | 2 | | | | II-2 | Gm4216 | | |
| 8798 | 2 | | | | II-2 | Gm4251 | | |
| 8807 | 2 | | | | II-2 | Gm4303 | | |
| 8808 | 2 | | | | II-2 | Gm4307 | | |
| 8809 | 2 | | | | II-2 | Gm4312 | | |
| 8810 | 2 | | | | II-2 | Gm4340 | | |
| 8811 | 2 | | | | II-2 | Gm4349 | | |
| 8816 | 2 | | | | II-2 | Gm4432 | | |
| 8818 | 2 | | | | II-2 | Gm4477 | | |
| 8826 | 2 | | | | II-2 | Gm4710 | | |
| 8833 | 2 | | | | II-2 | Gm4776 | | |
| 8835 | 2 | | | | II-2 | Gm4788 | | |
| 8877 | 2 | | | | II-2 | Gm5083 | | |
| 8879 | 2 | | | | II-2 | Gm5086 | | |
| 8881 | 2 | | | | II-2 | Gm5088 | | |
| 8990 | 2 | | | | II-2 | Gm5941 | | |
| 9005 | 2 | | | | II-2 | Gm6164 | | |
| 9007 | 2 | | | | II-2 | Gm6213 | | |
| 9021 | 2 | | | | II-2 | Gm6370 | | |
| 9027 | 2 | | | | II-2 | Gm6432 | | |
| 9029 | 2 | | | | II-2 | Gm6460 | | |
| 9070 | 2 | | | | II-2 | Gm6927 | | |
| 9081 | 2 | | | | II-2 | Gm7104 | | |
| 9103 | 2 | | | | II-2 | Gm7609 | | |
| 9119 | 2 | | | | II-2 | Gm806 | | |
| 9122 | 2 | | | | II-2 | Gm815 | | |
| 9124 | 2 | | | | II-2 | Gm8221 | | |
| 9125 | 2 | | | | II-2 | Gm8234 | | |
| 9126 | 2 | | | | II-2 | Gm826 | | |
| 9131 | 2 | | | | II-2 | Gm833 | | |
| 9132 | 2 | | | | II-2 | Gm8363 | | |
| 9139 | 2 | | | | II-2 | Gm8615 | | |
| 9266 | 2 | | | | II-2 | Golga2 | 2801 | 4-May-15 |
| 9349 | 2 | | | | II-2 | Gpr124 | 25960 | 4-May-15 |
| 9366 | 2 | | | | II-2 | Gpr15 | 2838 | 4-May-15 |
| 9408 | 2 | | | | II-2 | Gpr55 | 9290 | 4-May-15 |
| 9510 | 2 | | | | II-2 | Grm6 | 2916 | 4-May-15 |
| 9513 | 2 | | | | II-2 | Grn | 2896 | 23-May-15 |
| 9538 | 2 | | | | II-2 | Gse1 | 23199 | 21-May-15 |
| 9580 | 2 | | | | II-2 | Gtf2e1 | 2960 | 4-May-15 |
| 9623 | 2 | | | | II-2 | Gucy2g | 390003 | 4-May-15 |
| 9647 | 2 | | | | II-2 | Gzmg | | |
| 9649 | 2 | | | | II-2 | Gzmm | 3004 | 4-May-15 |
| 9685 | 2 | | | | II-2 | H2-M10.2 | | |
| 9686 | 2 | | | | II-2 | H2-M10.3 | | |
| 9687 | 2 | | | | II-2 | H2-M10.4 | | |
| 9688 | 2 | | | | II-2 | H2-M10.5 | | |
| 9689 | 2 | | | | II-2 | H2-M10.6 | | |
| 9690 | 2 | | | | II-2 | H2-M11 | | |
| 9881 | 2 | | | | II-2 | Hibadh | 11112 | 12-May-15 |
| 10062 | 2 | | | | II-2 | Hoxa10 | 3206 | 28-May-15 |
| 10089 | 2 | | | | II-2 | Hoxc6 | 3223 | 4-May-15 |
| 10095 | 2 | | | | II-2 | Hoxd12 | 3238 | 4-May-15 |
| 10321 | 2 | | | | II-2 | Ifna13 | 3447 | 4-May-15 |
| 10322 | 2 | | | | II-2 | Ifna14 | 3448 | 12-May-15 |
| 10323 | 2 | | | | II-2 | Ifna15 | | |
| 10324 | 2 | | | | II-2 | Ifna16 | 3449 | 12-May-15 |
| 10325 | 2 | | | | II-2 | Ifna2 | 3440 | 3-May-15 |
| 10326 | 2 | | | | II-2 | Ifna4 | 3441 | 4-May-15 |
| 10327 | 2 | | | | II-2 | Ifna5 | 3442 | 4-May-15 |
| 10328 | 2 | | | | II-2 | Ifna6 | 3443 | 4-May-15 |
| 10329 | 2 | | | | II-2 | Ifna7 | 3444 | 4-May-15 |
| 10330 | 2 | | | | II-2 | Ifna9 | | |
| 10331 | 2 | | | | II-2 | Ifnab | | |
| 10332 | 2 | | | | II-2 | Ifnar1 | 3454 | 12-May-15 |
| 10333 | 2 | | | | II-2 | Ifnar2 | 3455 | 12-May-15 |
| 10337 | 2 | | | | II-2 | Ifngr1 | 3459 | 12-May-15 |
| 10344 | 2 | | | | II-2 | Ifrd1 | 3475 | 26-May-15 |
| 10370 | 2 | | | | II-2 | Igf2os | | |
| 10473 | 2 | | | | II-2 | Il25 | 64806 | 7-Jun-15 |
| 10475 | 2 | | | | II-2 | Il27ra | 9466 | 4-May-15 |
| 10607 | 2 | | | | II-2 | Iqgap2 | 10788 | 17-May-15 |
| 10634 | 2 | | | | II-2 | Irgm2 | | |
| 10904 | 2 | | | | II-2 | Kcnv2 | 169522 | 4-May-15 |
| 11139 | 2 | | | | II-2 | Klra6 | | |
| 11310 | 2 | | | | II-2 | L3mbtl4 | 91133 | 14-May-15 |
| 11312 | 2 | | | | II-2 | Lacc1 | 144811 | 4-May-15 |
| 11603 | 2 | | | | II-2 | LOC101056149 | | |
| 11618 | 2 | | | | II-2 | LOC102636514 | | |
| 11895 | 2 | | | | II-2 | Macf1 | 23499 | 12-May-15 |
| 11917 | 2 | | | | II-2 | Magea3 | 4102 | 31-May-15 |
| 11919 | 2 | | | | II-2 | Magea5 | 4104 | 4-May-15 |
| 11923 | 2 | | | | II-2 | Mageb16 | 139604 | 4-May-15 |
| 11925 | 2 | | | | II-2 | Mageb18 | 286514 | 4-May-15 |
| 11938 | 2 | | | | II-2 | Magi3 | 260425 | 4-May-15 |
| 12081 | 2 | | | | II-2 | Mast3 | 23031 | 21-May-15 |
| 12393 | 2 | | | | II-2 | Mir101c | | |
| 12394 | 2 | | | | II-2 | Mir103-1 | 406895 | 21-May-15 |
| 12395 | 2 | | | | II-2 | Mir103-2 | 406896 | 21-May-15 |
| 12396 | 2 | | | | II-2 | Mir105 | | |
| 12397 | 2 | | | | II-2 | Mir106a | 406899 | 21-May-15 |
| 12398 | 2 | | | | II-2 | Mir106b | 406900 | 21-May-15 |
| 12399 | 2 | | | | II-2 | Mir107 | 406901 | 21-May-15 |
| 12400 | 2 | | | | II-2 | Mir10a | 406902 | 21-May-15 |
| 12401 | 2 | | | | II-2 | Mir10b | 406903 | 21-May-15 |
| 12402 | 2 | | | | II-2 | Mir1187 | | |
| 12403 | 2 | | | | II-2 | Mir1188 | | |
| 12404 | 2 | | | | II-2 | Mir1190 | | |
| 12405 | 2 | | | | II-2 | Mir1191 | | |
| 12406 | 2 | | | | II-2 | Mir1191b | | |
| 12407 | 2 | | | | II-2 | Mir1192 | | |
| 12408 | 2 | | | | II-2 | Mir1193 | 100422837 | 4-May-15 |
| 12409 | 2 | | | | II-2 | Mir1195 | | |
| 12410 | 2 | | | | II-2 | Mir1197 | 100302250 | 21-May-15 |
| 12411 | 2 | | | | II-2 | Mir1198 | | |
| 12415 | 2 | | | | II-2 | Mir1231 | 100302158 | 4-May-15 |
| 12416 | 2 | | | | II-2 | Mir1247 | 100302145 | 21-May-15 |
| 12417 | 2 | | | | II-2 | Mir1249 | 100302 | 4-May-15 |

Fig.22 - 89

| | | | | | | | | |
|---|---|---|---|---|---|---|---|---|
| | | | | | | | 149 | |
| 12418 | 2 | | | | II-2 | Mir124a-1 | | |
| 12419 | 2 | | | | II-2 | Mir124a-2 | | |
| 12420 | 2 | | | | II-2 | Mir124a-3 | | |
| 12421 | 2 | | | | II-2 | Mir1251 | 100302 289 | 4-May-15 |
| 12422 | 2 | | | | II-2 | Mir1258 | 100302 172 | 21-May-15 |
| 12423 | 2 | | | | II-2 | Mir125a | 406910 | 24-May-15 |
| 12424 | 2 | | | | II-2 | Mir125b-1 | | |
| 12425 | 2 | | | | II-2 | Mir125b-2 | | |
| 12426 | 2 | | | | II-2 | Mir126 | 406913 | 21-May-15 |
| 12427 | 2 | | | | II-2 | Mir1264 | 100302 251 | 4-May-15 |
| 12428 | 2 | | | | II-2 | Mir126b | | |
| 12429 | 2 | | | | II-2 | Mir127 | 406914 | 21-May-15 |
| 12430 | 2 | | | | II-2 | Mir128-1 | 406915 | 21-May-15 |
| 12431 | 2 | | | | II-2 | Mir128-2 | 406916 | 21-May-15 |
| 12433 | 2 | | | | II-2 | Mir129-1 | 406917 | 21-May-15 |
| 12434 | 2 | | | | II-2 | Mir129-2 | 406918 | 21-May-15 |
| 12438 | 2 | | | | II-2 | Mir130a | 406919 | 24-May-15 |
| 12439 | 2 | | | | II-2 | Mir130b | 406920 | 21-May-15 |
| 12440 | 2 | | | | II-2 | Mir130c | | |
| 12441 | 2 | | | | II-2 | Mir132 | 406921 | 21-May-15 |
| 12442 | 2 | | | | II-2 | Mir133a-1 | | |
| 12445 | 2 | | | | II-2 | Mir133c | | |
| 12447 | 2 | | | | II-2 | Mir135a-1 | | |
| 12448 | 2 | | | | II-2 | Mir135a-2 | | |
| 12449 | 2 | | | | II-2 | Mir135b | 442891 | 21-May-15 |
| 12450 | 2 | | | | II-2 | Mir136 | 406927 | 24-May-15 |
| 12451 | 2 | | | | II-2 | Mir137 | 406928 | 21-May-15 |
| 12452 | 2 | | | | II-2 | Mir138-1 | 406929 | 21-May-15 |
| 12453 | 2 | | | | II-2 | Mir138-2 | 406930 | 21-May-15 |
| 12454 | 2 | | | | II-2 | Mir139 | 406931 | 21-May-15 |
| 12455 | 2 | | | | II-2 | Mir140 | 406932 | 21-May-15 |
| 12456 | 2 | | | | II-2 | Mir141 | 406933 | 21-May-15 |
| 12457 | 2 | | | | II-2 | Mir142 | 406934 | 21-May-15 |
| 12464 | 2 | | | | II-2 | Mir146 | | |
| 12465 | 2 | | | | II-2 | Mir146b | 574447 | 21-May-15 |
| 12466 | 2 | | | | II-2 | Mir147 | 406939 | 4-May-15 |
| 12467 | 2 | | | | II-2 | Mir148a | 406940 | 21-May-15 |
| 12468 | 2 | | | | II-2 | Mir148b | 442892 | 21-May-15 |
| 12469 | 2 | | | | II-2 | Mir149 | 406941 | 21-May-15 |
| 12470 | 2 | | | | II-2 | Mir150 | 406942 | 21-May-15 |
| 12471 | 2 | | | | II-2 | Mir152 | 406943 | 21-May-15 |
| 12472 | 2 | | | | II-2 | Mir153 | | |
| 12473 | 2 | | | | II-2 | Mir154 | 406946 | 21-May-15 |
| 12474 | 2 | | | | II-2 | Mir155 | 406947 | 21-May-15 |
| 12475 | 2 | | | | II-2 | Mir15a | 406948 | 21-May-15 |
| 12476 | 2 | | | | II-2 | Mir15b | 406949 | 21-May-15 |
| 12477 | 2 | | | | II-2 | Mir16-1 | 406950 | 21-May-15 |
| 12478 | 2 | | | | II-2 | Mir16-2 | 406951 | 21-May-15 |
| 12485 | 2 | | | | II-2 | Mir181b-1 | | |
| 12486 | 2 | | | | II-2 | Mir181b-2 | | |
| 12487 | 2 | | | | II-2 | Mir181c | 406957 | 21-May-15 |
| 12488 | 2 | | | | II-2 | Mir181d | 574457 | 21-May-15 |
| 12489 | 2 | | | | II-2 | Mir182 | 406958 | 7-Jun-15 |
| 12490 | 2 | | | | II-2 | Mir183 | 406959 | 7-Jun-15 |
| 12491 | 2 | | | | II-2 | Mir1839 | | |
| 12492 | 2 | | | | II-2 | Mir184 | 406960 | 21-May-15 |
| 12493 | 2 | | | | II-2 | Mir1843 | | |
| 12494 | 2 | | | | II-2 | Mir1843b | | |
| 12495 | 2 | | | | II-2 | Mir185 | 406961 | 24-May-15 |
| 12496 | 2 | | | | II-2 | Mir186 | 406962 | 21-May-15 |
| 12497 | 2 | | | | II-2 | Mir187 | 406963 | 21-May-15 |
| 12498 | 2 | | | | II-2 | Mir188 | 406964 | 21-May-15 |
| 12499 | 2 | | | | II-2 | Mir1892 | | |
| 12500 | 2 | | | | II-2 | Mir1893 | | |
| 12501 | 2 | | | | II-2 | Mir1894 | | |
| 12502 | 2 | | | | II-2 | Mir1895 | | |
| 12503 | 2 | | | | II-2 | Mir1896 | | |
| 12504 | 2 | | | | II-2 | Mir1897 | | |
| 12505 | 2 | | | | II-2 | Mir1898 | | |
| 12528 | 2 | | | | II-2 | Mir1934 | | |
| 12550 | 2 | | | | II-2 | Mir1955 | | |
| 12574 | 2 | | | | II-2 | Mir199a-2 | | |
| 12576 | 2 | | | | II-2 | Mir19a | 406979 | 24-May-15 |
| 12595 | 2 | | | | II-2 | Mir20b | 574032 | 7-Jun-15 |
| 12597 | 2 | | | | II-2 | Mir210 | 406992 | 24-May-15 |
| 12650 | 2 | | | | II-2 | Mir297b | | |
| 12669 | 2 | | | | II-2 | Mir3060 | | |
| 12720 | 2 | | | | II-2 | Mir3103 | | |
| 12737 | 2 | | | | II-2 | Mir33 | 407039 | 21-May-15 |
| 12943 | 2 | | | | II-2 | Mir5619 | | |
| 13051 | 2 | | | | II-2 | Mir6538 | | |
| 13077 | 2 | | | | II-2 | Mir670 | 100313 777 | 4-May-15 |
| 13082 | 2 | | | | II-2 | Mir674 | | |
| 13086 | 2 | | | | II-2 | Mir677 | | |
| 13088 | 2 | | | | II-2 | Mir679 | | |
| 13102 | 2 | | | | II-2 | Mir6898 | | |
| 13105 | 2 | | | | II-2 | Mir6900 | | |
| 13108 | 2 | | | | II-2 | Mir6903 | | |
| 13134 | 2 | | | | II-2 | Mir6926 | | |
| 13145 | 2 | | | | II-2 | Mir6936 | | |
| 13164 | 2 | | | | II-2 | Mir6953 | | |
| 13241 | 2 | | | | II-2 | Mir7025 | | |
| 13308 | 2 | | | | II-2 | Mir7085 | | |
| 13328 | 2 | | | | II-2 | Mir717 | | |
| 13364 | 2 | | | | II-2 | Mir7241 | | |
| 13399 | 2 | | | | II-2 | Mir767 | 768215 | 21-May-15 |
| 13447 | 2 | | | | II-2 | Mir8118 | | |
| 13491 | 2 | | | | II-2 | Mis12 | 79003 | 4-May-15 |
| 13669 | 2 | | | | II-2 | Mrgpra3 | | |
| 13672 | 2 | | | | II-2 | Mrgpra9 | | |
| 13673 | 2 | | | | II-2 | Mrgprb1 | | |
| 13675 | 2 | | | | II-2 | Mrgprb3 | | |
| 13679 | 2 | | | | II-2 | Mrgprd | 116512 | 4-May-15 |
| 13680 | 2 | | | | II-2 | Mrgpre | 116534 | 12-May-15 |
| 13687 | 2 | | | | II-2 | Mrm1 | 79922 | 4-May-15 |
| 13694 | 2 | | | | II-2 | Mroh6 | 642475 | 4-May-15 |
| 13699 | 2 | | | | II-2 | Mrpl10 | 124995 | 4-May-15 |
| 13803 | 2 | | | | II-2 | Msh4 | 4438 | 4-May-15 |
| 13888 | 2 | | | | II-2 | Mtpap | 55149 | 4-May-15 |
| 13918 | 2 | | | | II-2 | Mum1 | 84939 | 7-Jun-15 |
| 14422 | 2 | | | | II-2 | Nkx2-2 | 4821 | 17-May-15 |
| 14449 | 2 | | | | II-2 | Nlrp2 | 55655 | 28-May-15 |
| 14452 | 2 | | | | II-2 | Nlrp4b | | |
| 14454 | 2 | | | | II-2 | Nlrp4e | | |
| 14457 | 2 | | | | II-2 | Nlrp5 | 126206 | 4-May-15 |
| 14459 | 2 | | | | II-2 | Nlrp6 | 171389 | 4-May-15 |
| 14463 | 2 | | | | II-2 | Nlrx1 | 79671 | 4-May-15 |
| 14497 | 2 | | | | II-2 | Noc4l | 79050 | 12-May-15 |
| 14552 | 2 | | | | II-2 | Npc2 | 10577 | 23-May-15 |
| 14652 | 2 | | | | II-2 | Nrp2 | 8828 | 7-Jun-15 |
| 14815 | 2 | | | | II-2 | Oas1g | | |
| 14830 | 2 | | | | II-2 | Obox5 | | |
| 14831 | 2 | | | | II-2 | Obox6 | | |
| 14832 | 2 | | | | II-2 | Obp1a | | |
| 14859 | 2 | | | | II-2 | Ogdhl | 55753 | 4-May-15 |
| 14884 | 2 | | | | II-2 | Olfr1000 | | |
| 14885 | 2 | | | | II-2 | Olfr1002 | | |
| 14886 | 2 | | | | II-2 | Olfr1006 | | |
| 14887 | 2 | | | | II-2 | Olfr1008 | | |
| 14888 | 2 | | | | II-2 | Olfr1009 | | |
| 14889 | 2 | | | | II-2 | Olfr101 | | |
| 14890 | 2 | | | | II-2 | Olfr1010 | | |
| 14891 | 2 | | | | II-2 | Olfr1012 | | |
| 14892 | 2 | | | | II-2 | Olfr1013 | | |
| 14893 | 2 | | | | II-2 | Olfr1014 | | |
| 14894 | 2 | | | | II-2 | Olfr1015 | | |
| 14895 | 2 | | | | II-2 | Olfr1016 | | |
| 14896 | 2 | | | | II-2 | Olfr1018 | | |
| 14897 | 2 | | | | II-2 | Olfr1019 | | |
| 14898 | 2 | | | | II-2 | Olfr102 | | |
| 14899 | 2 | | | | II-2 | Olfr1020 | | |
| 14900 | 2 | | | | II-2 | Olfr1022 | | |
| 14901 | 2 | | | | II-2 | Olfr1023 | | |
| 14902 | 2 | | | | II-2 | Olfr1024 | | |
| 14904 | 2 | | | | II-2 | Olfr1028 | | |
| 14905 | 2 | | | | II-2 | Olfr1029 | | |
| 14906 | 2 | | | | II-2 | Olfr103 | | |
| 14907 | 2 | | | | II-2 | Olfr1030 | | |
| 14908 | 2 | | | | II-2 | Olfr1031 | | |
| 14909 | 2 | | | | II-2 | Olfr1032 | | |
| 14915 | 2 | | | | II-2 | Olfr1039 | | |
| 14916 | 2 | | | | II-2 | Olfr1040 | | |
| 14917 | 2 | | | | II-2 | Olfr1042 | | |
| 14918 | 2 | | | | II-2 | Olfr1043 | | |
| 14919 | 2 | | | | II-2 | Olfr1044 | | |
| 14920 | 2 | | | | II-2 | Olfr1045 | | |
| 14921 | 2 | | | | II-2 | Olfr1046 | | |
| 14922 | 2 | | | | II-2 | Olfr1047 | | |
| 14923 | 2 | | | | II-2 | Olfr1048 | | |
| 14924 | 2 | | | | II-2 | Olfr1049 | | |
| 14925 | 2 | | | | II-2 | Olfr1051 | | |
| 14926 | 2 | | | | II-2 | Olfr1052 | | |
| 14927 | 2 | | | | II-2 | Olfr1053 | | |
| 14931 | 2 | | | | II-2 | Olfr1057 | | |
| 14933 | 2 | | | | II-2 | Olfr1061 | | |
| 14934 | 2 | | | | II-2 | Olfr1062 | | |
| 14935 | 2 | | | | II-2 | Olfr1065 | | |
| 14936 | 2 | | | | II-2 | Olfr1066 | | |
| 14937 | 2 | | | | II-2 | Olfr107 | | |
| 14938 | 2 | | | | II-2 | Olfr1076 | | |
| 14939 | 2 | | | | II-2 | Olfr1077-ps1 | | |
| 14940 | 2 | | | | II-2 | Olfr1079 | | |
| 14941 | 2 | | | | II-2 | Olfr108 | | |
| 14942 | 2 | | | | II-2 | Olfr1080 | | |
| 14951 | 2 | | | | II-2 | Olfr1093 | | |
| 14952 | 2 | | | | II-2 | Olfr1094 | | |
| 14954 | 2 | | | | II-2 | Olfr1097 | | |
| 14955 | 2 | | | | II-2 | Olfr1098 | | |
| 14956 | 2 | | | | II-2 | Olfr1099 | | |
| 14957 | 2 | | | | II-2 | Olfr11 | | |
| 14958 | 2 | | | | II-2 | Olfr110 | | |
| 14959 | 2 | | | | II-2 | Olfr1100 | | |
| 14960 | 2 | | | | II-2 | Olfr1101 | | |
| 14961 | 2 | | | | II-2 | Olfr1102 | | |
| 14963 | 2 | | | | II-2 | Olfr1105 | | |
| 14964 | 2 | | | | II-2 | Olfr1106 | | |
| 14965 | 2 | | | | II-2 | Olfr1107 | | |
| 14966 | 2 | | | | II-2 | Olfr1109 | | |
| 14967 | 2 | | | | II-2 | Olfr111 | | |
| 14968 | 2 | | | | II-2 | Olfr1110 | | |
| 14969 | 2 | | | | II-2 | Olfr1111 | | |

Fig.22 - 90

| | | | | | | | | |
|---|---|---|---|---|---|---|---|---|
| 14970 | 2 | | | | II-2 | Olfr1112 | | |
| 14973 | 2 | | | | II-2 | Olfr1116-ps | | |
| 14974 | 2 | | | | II-2 | Olfr1118 | | |
| 14975 | 2 | | | | II-2 | Olfr112 | | |
| 14976 | 2 | | | | II-2 | Olfr1120 | | |
| 14977 | 2 | | | | II-2 | Olfr1121 | | |
| 14979 | 2 | | | | II-2 | Olfr1123 | | |
| 14980 | 2 | | | | II-2 | Olfr1124 | | |
| 14981 | 2 | | | | II-2 | Olfr1126 | | |
| 14982 | 2 | | | | II-2 | Olfr1128 | | |
| 14983 | 2 | | | | II-2 | Olfr1129 | | |
| 14984 | 2 | | | | II-2 | Olfr113 | | |
| 14985 | 2 | | | | II-2 | Olfr1130 | | |
| 14986 | 2 | | | | II-2 | Olfr1131 | | |
| 14987 | 2 | | | | II-2 | Olfr1132 | | |
| 14989 | 2 | | | | II-2 | Olfr1134 | | |
| 14990 | 2 | | | | II-2 | Olfr1135 | | |
| 14991 | 2 | | | | II-2 | Olfr1136 | | |
| 14992 | 2 | | | | II-2 | Olfr1137 | | |
| 14993 | 2 | | | | II-2 | Olfr1138 | | |
| 14994 | 2 | | | | II-2 | Olfr114 | | |
| 14995 | 2 | | | | II-2 | Olfr1140 | | |
| 14996 | 2 | | | | II-2 | Olfr1141 | | |
| 15026 | 2 | | | | II-2 | Olfr1181 | | |
| 15027 | 2 | | | | II-2 | Olfr1182 | | |
| 15214 | 2 | | | | II-2 | Olfr1387 | | |
| 15222 | 2 | | | | II-2 | Olfr1394 | | |
| 15233 | 2 | | | | II-2 | Olfr1412 | | |
| 15292 | 2 | | | | II-2 | Olfr1490 | | |
| 15318 | 2 | | | | II-2 | Olfr1535 | | |
| 15326 | 2 | | | | II-2 | Olfr160 | | |
| 15336 | 2 | | | | II-2 | Olfr171 | | |
| 15337 | 2 | | | | II-2 | Olfr172 | | |
| 15340 | 2 | | | | II-2 | Olfr176 | | |
| 15350 | 2 | | | | II-2 | Olfr190 | | |
| 15354 | 2 | | | | II-2 | Olfr194 | | |
| 15358 | 2 | | | | II-2 | Olfr198 | | |
| 15375 | 2 | | | | II-2 | Olfr220 | | |
| 15410 | 2 | | | | II-2 | Olfr285 | | |
| 15424 | 2 | | | | II-2 | Olfr3 | | |
| 15445 | 2 | | | | II-2 | Olfr320 | | |
| 15465 | 2 | | | | II-2 | Olfr348 | | |
| 15475 | 2 | | | | II-2 | Olfr360 | | |
| 15477 | 2 | | | | II-2 | Olfr362 | | |
| 15486 | 2 | | | | II-2 | Olfr374 | | |
| 15495 | 2 | | | | II-2 | Olfr389 | | |
| 15521 | 2 | | | | II-2 | Olfr427 | | |
| 15528 | 2 | | | | II-2 | Olfr435 | | |
| 15549 | 2 | | | | II-2 | Olfr462 | | |
| 15555 | 2 | | | | II-2 | Olfr47 | | |
| 15556 | 2 | | | | II-2 | Olfr470 | | |
| 15557 | 2 | | | | II-2 | Olfr472 | | |
| 15642 | 2 | | | | II-2 | Olfr569 | | |
| 15736 | 2 | | | | II-2 | Olfr677 | | |
| 15746 | 2 | | | | II-2 | Olfr689 | | |
| 15750 | 2 | | | | II-2 | Olfr692 | | |
| 15870 | 2 | | | | II-2 | Olfr836 | | |
| 15875 | 2 | | | | II-2 | Olfr847 | | |
| 15901 | 2 | | | | II-2 | Olfr883 | | |
| 15920 | 2 | | | | II-2 | Olfr905 | | |
| 15922 | 2 | | | | II-2 | Olfr907 | | |
| 16155 | 2 | | | | II-2 | Pabpn1 | 8106 | 23-May-15 |
| 16156 | 2 | | | | II-2 | Pabpn1l | 390748 | 4-May-15 |
| 16262 | 2 | | | | II-2 | Pawr | 5074 | 24-May-15 |
| 16284 | 2 | | | | II-2 | Pbx3 | 5090 | 17-May-15 |
| 16317 | 2 | | | | II-2 | Pcdha7 | 56141 | 4-May-15 |
| 16322 | 2 | | | | II-2 | Pcdhb1 | 29930 | 4-May-15 |
| 16612 | 2 | | | | II-2 | Pgs1 | 9489 | 4-May-15 |
| 16774 | 2 | | | | II-2 | Pitpna | 5306 | 21-May-15 |
| 16790 | 2 | | | | II-2 | Pkd1 | 5310 | 31-May-15 |
| 17216 | 2 | | | | II-2 | Ppp1r7 | 5510 | 12-May-15 |
| 17271 | 2 | | | | II-2 | Pramef8 | 391002 | 4-May-15 |
| 17272 | 2 | | | | II-2 | Pramel1 | | |
| 17273 | 2 | | | | II-2 | Pramel3 | | |
| 17275 | 2 | | | | II-2 | Pramel5 | | |
| 17277 | 2 | | | | II-2 | Pramel7 | | |
| 17289 | 2 | | | | II-2 | Prdm15 | 63977 | 21-May-15 |
| 17290 | 2 | | | | II-2 | Prdm16 | 63976 | 12-May-15 |
| 17291 | 2 | | | | II-2 | Prdm2 | 7799 | 4-May-15 |
| 17364 | 2 | | | | II-2 | Prl2c2 | | |
| 17365 | 2 | | | | II-2 | Prl2c3 | | |
| 17366 | 2 | | | | II-2 | Prl2c4 | | |
| 17367 | 2 | | | | II-2 | Prl2c5 | | |
| 17368 | 2 | | | | II-2 | Prl3a1 | | |
| 17369 | 2 | | | | II-2 | Prl3b1 | | |
| 17370 | 2 | | | | II-2 | Prl3c1 | | |
| 17371 | 2 | | | | II-2 | Prl3d1 | | |
| 17372 | 2 | | | | II-2 | Prl3d2 | | |
| 17374 | 2 | | | | II-2 | Prl4a1 | | |
| 17375 | 2 | | | | II-2 | Prl5a1 | | |
| 17376 | 2 | | | | II-2 | Prl6a1 | | |
| 17377 | 2 | | | | II-2 | Prl7a1 | | |
| 17378 | 2 | | | | II-2 | Prl7a2 | | |
| 17379 | 2 | | | | II-2 | Prl7b1 | | |
| 17380 | 2 | | | | II-2 | Prl7c1 | | |
| 17381 | 2 | | | | II-2 | Prl7d1 | | |
| 17382 | 2 | | | | II-2 | Prl8a1 | | |

| | | | | | | | | |
|---|---|---|---|---|---|---|---|---|
| 17383 | 2 | | | | II-2 | Prl8a2 | | |
| 17387 | 2 | | | | II-2 | Prlh | 51052 | 4-May-15 |
| 17388 | 2 | | | | II-2 | Prlhr | 2834 | 4-May-15 |
| 17511 | 2 | | | | II-2 | Prss45 | 377047 | 4-May-15 |
| 17542 | 2 | | | | II-2 | Psg18 | | |
| 17544 | 2 | | | | II-2 | Psg20 | | |
| 17545 | 2 | | | | II-2 | Psg21 | | |
| 17547 | 2 | | | | II-2 | Psg23 | | |
| 17550 | 2 | | | | II-2 | Psg27 | | |
| 17552 | 2 | | | | II-2 | Psg29 | | |
| 17554 | 2 | | | | II-2 | Psip1 | 11168 | 21-May-15 |
| 17555 | 2 | | | | II-2 | Pskh1 | 5681 | 12-May-15 |
| 17715 | 2 | | | | II-2 | Ptprz1 | 5803 | 17-May-15 |
| 17940 | 2 | | | | II-2 | Rangap1 | 5905 | 4-May-15 |
| 18272 | 2 | | | | II-2 | Rhox12 | | |
| 18273 | 2 | | | | II-2 | Rhox13 | | |
| 18275 | 2 | | | | II-2 | Rhox2b | | |
| 18277 | 2 | | | | II-2 | Rhox2d | | |
| 18278 | 2 | | | | II-2 | Rhox2e | | |
| 18279 | 2 | | | | II-2 | Rhox2f | | |
| 18280 | 2 | | | | II-2 | Rhox2g | | |
| 18281 | 2 | | | | II-2 | Rhox2h | | |
| 18282 | 2 | | | | II-2 | Rhox3a | | |
| 18283 | 2 | | | | II-2 | Rhox3c | | |
| 18284 | 2 | | | | II-2 | Rhox3e | | |
| 18285 | 2 | | | | II-2 | Rhox3f | | |
| 18286 | 2 | | | | II-2 | Rhox3g | | |
| 18288 | 2 | | | | II-2 | Rhox4a | | |
| 18294 | 2 | | | | II-2 | Rhox4g | | |
| 18295 | 2 | | | | II-2 | Rhox5 | | |
| 18299 | 2 | | | | II-2 | Rhox9 | | |
| 18475 | 2 | | | | II-2 | Rnu73b | 114655 | 4-May-15 |
| 18476 | 2 | | | | II-2 | Robo1 | 6091 | 17-May-15 |
| 18742 | 2 | | | | II-2 | Rxfp4 | 339403 | 4-May-15 |
| 18780 | 2 | | | | II-2 | Sacm1l | 22908 | 23-May-15 |
| 18784 | 2 | | | | II-2 | Safb2 | 9667 | 4-May-15 |
| 18893 | 2 | | | | II-2 | Scgb1b24 | | |
| 19150 | 2 | | | | II-2 | Serpinb9e | | |
| 19151 | 2 | | | | II-2 | Serpinb9f | | |
| 19152 | 2 | | | | II-2 | Serpinb9g | | |
| 19491 | 2 | | | | II-2 | Slc22a23 | 63027 | 10-May-15 |
| 19549 | 2 | | | | II-2 | Slc25a44 | 9673 | 14-May-15 |
| 19795 | 2 | | | | II-2 | Slfn1 | | |
| 19902 | 2 | | | | II-2 | Smndc1 | 10285 | 23-May-15 |
| 20009 | 2 | | | | II-2 | Snord110 | 692213 | 4-May-15 |
| 20010 | 2 | | | | II-2 | Snord111 | 692214 | 4-May-15 |
| 20011 | 2 | | | | II-2 | Snord116 | | |
| 20012 | 2 | | | | II-2 | Snord116l1 | | |
| 20013 | 2 | | | | II-2 | Snord116l2 | | |
| 20014 | 2 | | | | II-2 | Snord118 | | |
| 20015 | 2 | | | | II-2 | Snord12 | 692057 | 4-May-15 |
| 20016 | 2 | | | | II-2 | Snord123 | 100113384 | 4-May-15 |
| 20017 | 2 | | | | II-2 | Snord14a | | |
| 20018 | 2 | | | | II-2 | Snord14c | | |
| 20019 | 2 | | | | II-2 | Snord14d | | |
| 20027 | 2 | | | | II-2 | Snord1c | 677850 | 4-May-15 |
| 20028 | 2 | | | | II-2 | Snord2 | 619567 | 4-May-15 |
| 20033 | 2 | | | | II-2 | Snord34 | 26817 | 4-May-15 |
| 20034 | 2 | | | | II-2 | Snord35a | 26816 | 4-May-15 |
| 20035 | 2 | | | | II-2 | Snord35b | 84546 | 4-May-15 |
| 20037 | 2 | | | | II-2 | Snord38a | 94162 | 12-May-15 |
| 20038 | 2 | | | | II-2 | Snord42a | 26809 | 4-May-15 |
| 20039 | 2 | | | | II-2 | Snord42b | 26808 | 4-May-15 |
| 20040 | 2 | | | | II-2 | Snord43 | 26807 | 4-May-15 |
| 20041 | 2 | | | | II-2 | Snord45b | 26804 | 4-May-15 |
| 20042 | 2 | | | | II-2 | Snord45c | 692085 | 4-May-15 |
| 20043 | 2 | | | | II-2 | Snord47 | 26802 | 4-May-15 |
| 20044 | 2 | | | | II-2 | Snord49a | 26800 | 4-May-15 |
| 20045 | 2 | | | | II-2 | Snord49b | 692087 | 4-May-15 |
| 20046 | 2 | | | | II-2 | Snord4a | 26773 | 4-May-15 |
| 20047 | 2 | | | | II-2 | Snord52 | 26797 | 4-May-15 |
| 20048 | 2 | | | | II-2 | Snord53 | 26796 | 4-May-15 |
| 20049 | 2 | | | | II-2 | Snord55 | 26811 | 4-May-15 |
| 20050 | 2 | | | | II-2 | Snord57 | 26792 | 4-May-15 |
| 20051 | 2 | | | | II-2 | Snord58b | 26790 | 4-May-15 |
| 20052 | 2 | | | | II-2 | Snord61 | 26787 | 4-May-15 |
| 20053 | 2 | | | | II-2 | Snord64 | 347686 | 4-May-15 |
| 20054 | 2 | | | | II-2 | Snord65 | 692106 | 4-May-15 |
| 20055 | 2 | | | | II-2 | Snord66 | 692107 | 4-May-15 |
| 20056 | 2 | | | | II-2 | Snord67 | 692108 | 4-May-15 |
| 20057 | 2 | | | | II-2 | Snord68 | 606500 | 4-May-15 |
| 20058 | 2 | | | | II-2 | Snord69 | 692109 | 4-May-15 |
| 20059 | 2 | | | | II-2 | Snord7 | 692076 | 4-May-15 |
| 20060 | 2 | | | | II-2 | Snord70 | 692110 | 4-May-15 |
| 20061 | 2 | | | | II-2 | Snord71 | 692111 | 4-May-15 |
| 20062 | 2 | | | | II-2 | Snord72 | 619564 | 4-May-15 |
| 20063 | 2 | | | | II-2 | Snord73a | 8944 | 4-May-15 |
| 20064 | 2 | | | | II-2 | Snord8 | 319103 | 4-May-15 |
| 20065 | 2 | | | | II-2 | Snord82 | 25826 | 12-May-15 |
| 20066 | 2 | | | | II-2 | Snord83b | 116938 | 4-May-15 |
| 20067 | 2 | | | | II-2 | Snord85 | 692200 | 4-May-15 |
| 20068 | 2 | | | | II-2 | Snord87 | 641648 | 4-May-15 |
| 20069 | 2 | | | | II-2 | Snord88a | 692202 | 4-May-15 |
| 20070 | 2 | | | | II-2 | Snord88c | 692204 | 4-May-15 |
| 20071 | 2 | | | | II-2 | Snord89 | 692205 | 4-May-15 |
| 20073 | 2 | | | | II-2 | Snord91a | 692207 | 12-May-15 |
| 20074 | 2 | | | | II-2 | Snord92 | 692209 | 4-May-15 |

Fig.22 - 91

| | | | | | | | | |
|---|---|---|---|---|---|---|---|---|
| 20075 | 2 | | | | II-2 | Snord93 | 692210 | 4-May-15 |
| 20076 | 2 | | | | II-2 | Snord95 | 619570 | 4-May-15 |
| 20077 | 2 | | | | II-2 | Snord96a | 619571 | 4-May-15 |
| 20078 | 2 | | | | II-2 | Snord98 | | |
| 20079 | 2 | | | | II-2 | Snord99 | 692212 | 4-May-15 |
| 20249 | 2 | | | | II-2 | Spata31d1c | | |
| 20289 | 2 | | | | II-2 | Speer9-ps1 | | |
| 20490 | 2 | | | | II-2 | Ssu2 | 51066 | 21-May-15 |
| 20500 | 2 | | | | II-2 | Ssxb8 | | |
| 20501 | 2 | | | | II-2 | Ssxb9 | | |
| 20502 | 2 | | | | II-2 | St13 | 6767 | 4-May-15 |
| 20680 | 2 | | | | II-2 | Sult2a4 | | |
| 20681 | 2 | | | | II-2 | Sult2a5 | | |
| 20682 | 2 | | | | II-2 | Sult2a6 | | |
| 20683 | 2 | | | | II-2 | Sult2a7 | | |
| 20691 | 2 | | | | II-2 | Sumo1 | 7341 | 17-May-15 |
| 20729 | 2 | | | | II-2 | Svil | 6840 | 12-May-15 |
| 20736 | 2 | | | | II-2 | Svs3b | | |
| 20737 | 2 | | | | II-2 | Svs4 | | |
| 20739 | 2 | | | | II-2 | Svs6 | | |
| 20740 | 2 | | | | II-2 | Swap70 | 23075 | 4-May-15 |
| 20742 | 2 | | | | II-2 | Swsap1 | 126074 | 4-May-15 |
| 20819 | 2 | | | | II-2 | Taar2 | 9287 | 7-Jun-15 |
| 20821 | 2 | | | | II-2 | Taar4 | | |
| 20822 | 2 | | | | II-2 | Taar5 | 9038 | 4-May-15 |
| 20823 | 2 | | | | II-2 | Taar6 | 319100 | 4-May-15 |
| 20824 | 2 | | | | II-2 | Taar7a | | |
| 20825 | 2 | | | | II-2 | Taar7b | | |
| 20826 | 2 | | | | II-2 | Taar7d | | |
| 20827 | 2 | | | | II-2 | Taar7e | | |
| 20828 | 2 | | | | II-2 | Taar7f | | |
| 20829 | 2 | | | | II-2 | Taar8a | | |
| 20830 | 2 | | | | II-2 | Taar8b | | |
| 20831 | 2 | | | | II-2 | Taar8c | | |
| 20832 | 2 | | | | II-2 | Taar9 | 134860 | 4-May-15 |
| 20833 | 2 | | | | II-2 | Tab1 | 10454 | 4-May-15 |
| 20834 | 2 | | | | II-2 | Tab2 | 23118 | 12-May-15 |
| 20908 | 2 | | | | II-2 | Tas2r105 | | |
| 20909 | 2 | | | | II-2 | Tas2r106 | | |
| 20910 | 2 | | | | II-2 | Tas2r107 | | |
| 20911 | 2 | | | | II-2 | Tas2r108 | | |
| 20912 | 2 | | | | II-2 | Tas2r109 | | |
| 20913 | 2 | | | | II-2 | Tas2r110 | | |
| 20914 | 2 | | | | II-2 | Tas2r113 | | |
| 20915 | 2 | | | | II-2 | Tas2r114 | | |
| 20916 | 2 | | | | II-2 | Tas2r115 | | |
| 20917 | 2 | | | | II-2 | Tas2r116 | | |
| 20918 | 2 | | | | II-2 | Tas2r117 | | |
| 20919 | 2 | | | | II-2 | Tas2r118 | | |
| 20920 | 2 | | | | II-2 | Tas2r119 | | |
| 20922 | 2 | | | | II-2 | Tas2r121 | | |
| 20923 | 2 | | | | II-2 | Tas2r122 | | |
| 20924 | 2 | | | | II-2 | Tas2r123 | | |
| 20925 | 2 | | | | II-2 | Tas2r124 | | |
| 20926 | 2 | | | | II-2 | Tas2r125 | | |
| 20927 | 2 | | | | II-2 | Tas2r126 | | |
| 20928 | 2 | | | | II-2 | Tas2r129 | | |
| 20929 | 2 | | | | II-2 | Tas2r130 | | |
| 20930 | 2 | | | | II-2 | Tas2r131 | | |
| 20931 | 2 | | | | II-2 | Tas2r134 | | |
| 20999 | 2 | | | | II-2 | Tbrg3 | | |
| 21058 | 2 | | | | II-2 | Tcl1b3 | | |
| 21059 | 2 | | | | II-2 | Tcl1b4 | | |
| 21060 | 2 | | | | II-2 | Tcl1b5 | | |
| 21091 | 2 | | | | II-2 | Tdpoz4 | | |
| 21092 | 2 | | | | II-2 | Tdpoz5 | | |
| 21093 | 2 | | | | II-2 | Tdrd1 | 56165 | 4-May-15 |
| 21094 | 2 | | | | II-2 | Tdrd12 | 91646 | 4-May-15 |
| 21095 | 2 | | | | II-2 | Tdrd3 | 81550 | 4-May-15 |
| 21344 | 2 | | | | II-2 | Tlr1 | 7096 | 17-May-15 |
| 21669 | 2 | | | | II-2 | Tmprss11f | 389208 | 4-May-15 |
| 21785 | 2 | | | | II-2 | Tomm40 | 10452 | 12-May-15 |
| 21914 | 2 | | | | II-2 | Triap1 | 51499 | 4-May-15 |
| 21944 | 2 | | | | II-2 | Trim33 | 51592 | 23-May-15 |
| 21958 | 2 | | | | II-2 | Trim44 | 54765 | 4-May-15 |
| 21959 | 2 | | | | II-2 | Trim45 | 80263 | 4-May-15 |
| 22539 | 2 | | | | II-2 | Usp17ld | | |
| 22540 | 2 | | | | II-2 | Usp17le | | |
| 22541 | 2 | | | | II-2 | Usp18 | 11274 | 23-May-15 |
| 22601 | 2 | | | | II-2 | Uvrag | 7405 | 12-May-15 |
| 22609 | 2 | | | | II-2 | Vamp1 | 6843 | 23-May-15 |
| 22673 | 2 | | | | II-2 | Vmn1r10 | | |
| 22674 | 2 | | | | II-2 | Vmn1r100 | | |
| 22675 | 2 | | | | II-2 | Vmn1r101 | | |
| 22676 | 2 | | | | II-2 | Vmn1r103 | | |
| 22677 | 2 | | | | II-2 | Vmn1r104 | | |
| 22678 | 2 | | | | II-2 | Vmn1r107 | | |
| 22679 | 2 | | | | II-2 | Vmn1r11 | | |
| 22680 | 2 | | | | II-2 | Vmn1r112 | | |
| 22681 | 2 | | | | II-2 | Vmn1r113 | | |
| 22682 | 2 | | | | II-2 | Vmn1r114 | | |
| 22683 | 2 | | | | II-2 | Vmn1r115 | | |
| 22684 | 2 | | | | II-2 | Vmn1r116 | | |
| 22685 | 2 | | | | II-2 | Vmn1r117 | | |
| 22686 | 2 | | | | II-2 | Vmn1r118 | | |
| 22687 | 2 | | | | II-2 | Vmn1r119 | | |
| 22688 | 2 | | | | II-2 | Vmn1r12 | | |
| 22689 | 2 | | | | II-2 | Vmn1r120 | | |
| 22690 | 2 | | | | II-2 | Vmn1r121 | | |
| 22691 | 2 | | | | II-2 | Vmn1r122 | | |
| 22692 | 2 | | | | II-2 | Vmn1r123 | | |
| 22693 | 2 | | | | II-2 | Vmn1r124 | | |
| 22694 | 2 | | | | II-2 | Vmn1r125 | | |
| 22695 | 2 | | | | II-2 | Vmn1r126 | | |
| 22696 | 2 | | | | II-2 | Vmn1r127 | | |
| 22698 | 2 | | | | II-2 | Vmn1r129 | | |
| 22699 | 2 | | | | II-2 | Vmn1r13 | | |
| 22700 | 2 | | | | II-2 | Vmn1r130 | | |
| 22701 | 2 | | | | II-2 | Vmn1r132 | | |
| 22702 | 2 | | | | II-2 | Vmn1r135 | | |
| 22703 | 2 | | | | II-2 | Vmn1r137 | | |
| 22704 | 2 | | | | II-2 | Vmn1r138 | | |
| 22705 | 2 | | | | II-2 | Vmn1r139 | | |
| 22706 | 2 | | | | II-2 | Vmn1r14 | | |
| 22707 | 2 | | | | II-2 | Vmn1r142 | | |
| 22708 | 2 | | | | II-2 | Vmn1r148 | | |
| 22709 | 2 | | | | II-2 | Vmn1r15 | | |
| 22710 | 2 | | | | II-2 | Vmn1r151 | | |
| 22711 | 2 | | | | II-2 | Vmn1r152 | | |
| 22713 | 2 | | | | II-2 | Vmn1r158 | | |
| 22714 | 2 | | | | II-2 | Vmn1r159 | | |
| 22715 | 2 | | | | II-2 | Vmn1r16 | | |
| 22716 | 2 | | | | II-2 | Vmn1r160 | | |
| 22717 | 2 | | | | II-2 | Vmn1r163 | | |
| 22718 | 2 | | | | II-2 | Vmn1r165 | | |
| 22722 | 2 | | | | II-2 | Vmn1r169 | | |
| 22723 | 2 | | | | II-2 | Vmn1r17 | | |
| 22724 | 2 | | | | II-2 | Vmn1r170 | | |
| 22725 | 2 | | | | II-2 | Vmn1r171 | | |
| 22726 | 2 | | | | II-2 | Vmn1r172 | | |
| 22727 | 2 | | | | II-2 | Vmn1r173 | | |
| 22728 | 2 | | | | II-2 | Vmn1r174 | | |
| 22729 | 2 | | | | II-2 | Vmn1r175 | | |
| 22730 | 2 | | | | II-2 | Vmn1r176 | | |
| 22731 | 2 | | | | II-2 | Vmn1r177 | | |
| 22732 | 2 | | | | II-2 | Vmn1r178 | | |
| 22733 | 2 | | | | II-2 | Vmn1r179 | | |
| 22734 | 2 | | | | II-2 | Vmn1r18 | | |
| 22735 | 2 | | | | II-2 | Vmn1r180 | | |
| 22736 | 2 | | | | II-2 | Vmn1r181 | | |
| 22738 | 2 | | | | II-2 | Vmn1r184 | | |
| 22740 | 2 | | | | II-2 | Vmn1r186 | | |
| 22741 | 2 | | | | II-2 | Vmn1r187 | | |
| 22742 | 2 | | | | II-2 | Vmn1r188 | | |
| 22743 | 2 | | | | II-2 | Vmn1r189 | | |
| 22744 | 2 | | | | II-2 | Vmn1r19 | | |
| 22745 | 2 | | | | II-2 | Vmn1r191 | | |
| 22746 | 2 | | | | II-2 | Vmn1r192 | | |
| 22747 | 2 | | | | II-2 | Vmn1r193 | | |
| 22748 | 2 | | | | II-2 | Vmn1r194 | | |
| 22749 | 2 | | | | II-2 | Vmn1r195 | | |
| 22750 | 2 | | | | II-2 | Vmn1r196 | | |
| 22751 | 2 | | | | II-2 | Vmn1r197 | | |
| 22752 | 2 | | | | II-2 | Vmn1r198 | | |
| 22753 | 2 | | | | II-2 | Vmn1r199 | | |
| 22754 | 2 | | | | II-2 | Vmn1r2 | | |
| 22755 | 2 | | | | II-2 | Vmn1r20 | | |
| 22756 | 2 | | | | II-2 | Vmn1r200 | | |
| 22757 | 2 | | | | II-2 | Vmn1r201 | | |
| 22758 | 2 | | | | II-2 | Vmn1r202 | | |
| 22759 | 2 | | | | II-2 | Vmn1r203 | | |
| 22760 | 2 | | | | II-2 | Vmn1r204 | | |
| 22761 | 2 | | | | II-2 | Vmn1r205 | | |
| 22762 | 2 | | | | II-2 | Vmn1r206 | | |
| 22763 | 2 | | | | II-2 | Vmn1r207-ps | | |
| 22764 | 2 | | | | II-2 | Vmn1r208 | | |
| 22765 | 2 | | | | II-2 | Vmn1r209 | | |
| 22766 | 2 | | | | II-2 | Vmn1r21 | | |
| 22767 | 2 | | | | II-2 | Vmn1r210 | | |
| 22768 | 2 | | | | II-2 | Vmn1r211 | | |
| 22770 | 2 | | | | II-2 | Vmn1r213 | | |
| 22771 | 2 | | | | II-2 | Vmn1r214 | | |
| 22772 | 2 | | | | II-2 | Vmn1r215 | | |
| 22773 | 2 | | | | II-2 | Vmn1r216 | | |
| 22774 | 2 | | | | II-2 | Vmn1r217 | | |
| 22775 | 2 | | | | II-2 | Vmn1r218 | | |
| 22776 | 2 | | | | II-2 | Vmn1r219 | | |
| 22777 | 2 | | | | II-2 | Vmn1r22 | | |
| 22778 | 2 | | | | II-2 | Vmn1r220 | | |
| 22779 | 2 | | | | II-2 | Vmn1r221 | | |
| 22780 | 2 | | | | II-2 | Vmn1r222 | | |
| 22781 | 2 | | | | II-2 | Vmn1r223 | | |
| 22782 | 2 | | | | II-2 | Vmn1r224 | | |
| 22783 | 2 | | | | II-2 | Vmn1r225 | | |
| 22784 | 2 | | | | II-2 | Vmn1r226 | | |
| 22785 | 2 | | | | II-2 | Vmn1r227 | | |
| 22860 | 2 | | | | II-2 | Vmn1r80 | | |
| 22929 | 2 | | | | II-2 | Vmn2r35 | | |
| 22991 | 2 | | | | II-2 | Vmn2r92 | | |
| 23128 | 2 | | | | II-2 | Wdr33 | 55339 | 4-May-15 |
| 23289 | 2 | | | | II-2 | Xndc1 | | |
| 23290 | 2 | | | | II-2 | Xntrpc | | |
| 23291 | 2 | | | | II-2 | Xpa | 7507 | 23-May-15 |
| 23588 | 2 | | | | II-2 | Zfp352 | | |

Fig.22 - 92

| | | | | | | | | |
|---|---|---|---|---|---|---|---|---|
| 23643 | 2 | | | | II-2 | Zfp46 | 80818 | 4-May-15 |
| 23731 | 2 | | | | II-2 | Zfp668 | | |
| 23829 | 2 | | | | II-2 | Zfp930 | | |
| 23944 | 2 | | | | II-2 | Zp3r | | |
| 23945 | 2 | | | | II-2 | Zp4-ps | | |
| 23946 | 2 | | | | II-2 | Zpbp | 11055 | 4-May-15 |
| 23948 | 2 | | | | II-2 | Zpld1 | 131368 | 4-May-15 |
| 23968 | 2 | | | | II-2 | Zscan4d | | |
| 23969 | 2 | | | | II-2 | Zscan4e | | |
| 23970 | 2 | | | | II-2 | Zscan4f | | |
| 23971 | 2 | | | | II-2 | Zscan5b | 342933 | 28-May-15 |
| 23972 | 2 | | | | II-2 | Zswim1 | 90204 | 4-May-15 |
| 23973 | 2 | | | | II-2 | Zswim2 | 151112 | 4-May-15 |
| 4 | 2 | | | | II-1 | 0610009L18Rik | | |
| 13 | 2 | | | | II-1 | 0610031O16Rik | | |
| 27 | 2 | | | | II-1 | 1110006O24Rik | | |
| 40 | 2 | | | | II-1 | 1110032A03Rik | | |
| 52 | 2 | | | | II-1 | 1110059E24Rik | | |
| 53 | 2 | | | | II-1 | 1110059G10Rik | | |
| 83 | 2 | | | | II-1 | 1600015I10Rik | | |
| 90 | 2 | | | | II-1 | 1600029I14Rik | | |
| 91 | 2 | | | | II-1 | 1600029O15Rik | | |
| 102 | 2 | | | | II-1 | 1700001L05Rik | | |
| 112 | 2 | | | | II-1 | 1700003G18Rik | | |
| 113 | 2 | | | | II-1 | 1700003H04Rik | | |
| 114 | 2 | | | | II-1 | 1700003L19Rik | | |
| 115 | 2 | | | | II-1 | 1700003M02Rik | | |
| 116 | 2 | | | | II-1 | 1700003M07Rik | | |
| 118 | 2 | | | | II-1 | 1700006A11Rik | | |
| 119 | 2 | | | | II-1 | 1700006E09Rik | | |
| 120 | 2 | | | | II-1 | 1700006F04Rik | | |
| 121 | 2 | | | | II-1 | 1700006H21Rik | | |
| 122 | 2 | | | | II-1 | 1700007B14Rik | | |
| 123 | 2 | | | | II-1 | 1700007F19Rik | | |
| 125 | 2 | | | | II-1 | 1700007J10Rik | | |
| 131 | 2 | | | | II-1 | 1700008I05Rik | | |
| 132 | 2 | | | | II-1 | 1700008J07Rik | | |
| 135 | 2 | | | | II-1 | 1700008P02Rik | | |
| 136 | 2 | | | | II-1 | 1700009C05Rik | | |
| 144 | 2 | | | | II-1 | 1700010J16Rik | | |
| 146 | 2 | | | | II-1 | 1700011A15Rik | | |
| 147 | 2 | | | | II-1 | 1700011B04Rik | | |
| 148 | 2 | | | | II-1 | 1700011E24Rik | | |
| 152 | 2 | | | | II-1 | 1700011M02Rik | | |
| 160 | 2 | | | | II-1 | 1700012P22Rik | | |
| 162 | 2 | | | | II-1 | 1700013F07Rik | | |
| 163 | 2 | | | | II-1 | 1700013G24Rik | | |
| 165 | 2 | | | | II-1 | 1700015E13Rik | | |
| 166 | 2 | | | | II-1 | 1700015F17Rik | | |
| 167 | 2 | | | | II-1 | 1700015G11Rik | | |
| 168 | 2 | | | | II-1 | 1700016C15Rik | | |
| 171 | 2 | | | | II-1 | 1700016H13Rik | | |
| 178 | 2 | | | | II-1 | 1700017G19Rik | | |
| 179 | 2 | | | | II-1 | 1700017J07Rik | | |
| 180 | 2 | | | | II-1 | 1700017N19Rik | | |
| 181 | 2 | | | | II-1 | 1700018A04Rik | | |
| 183 | 2 | | | | II-1 | 1700018B24Rik | | |
| 184 | 2 | | | | II-1 | 1700018C11Rik | | |
| 185 | 2 | | | | II-1 | 1700018F24Rik | | |
| 186 | 2 | | | | II-1 | 1700018G05Rik | | |
| 190 | 2 | | | | II-1 | 1700019B21Rik | | |
| 196 | 2 | | | | II-1 | 1700019M22Rik | | |
| 198 | 2 | | | | II-1 | 1700019O17Rik | | |
| 199 | 2 | | | | II-1 | 1700020A23Rik | | |
| 200 | 2 | | | | II-1 | 1700020D05Rik | | |
| 208 | 2 | | | | II-1 | 1700021F05Rik | | |
| 210 | 2 | | | | II-1 | 1700021K19Rik | | |
| 211 | 2 | | | | II-1 | 1700021N21Rik | | |
| 212 | 2 | | | | II-1 | 1700022A21Rik | | |
| 213 | 2 | | | | II-1 | 1700022A22Rik | | |
| 214 | 2 | | | | II-1 | 1700022E09Rik | | |
| 216 | 2 | | | | II-1 | 1700022I11Rik | | |
| 217 | 2 | | | | II-1 | 1700023C21Rik | | |
| 219 | 2 | | | | II-1 | 1700023F02Rik | | |
| 220 | 2 | | | | II-1 | 1700023F06Rik | | |
| 223 | 2 | | | | II-1 | 1700024F13Rik | | |
| 224 | 2 | | | | II-1 | 1700024G13Rik | | |
| 228 | 2 | | | | II-1 | 1700025C18Rik | | |
| 229 | 2 | | | | II-1 | 1700025F22Rik | | |
| 230 | 2 | | | | II-1 | 1700025F24Rik | | |
| 233 | 2 | | | | II-1 | 1700025M24Rik | | |
| 234 | 2 | | | | II-1 | 1700025N23Rik | | |
| 237 | 2 | | | | II-1 | 1700026F02Rik | | |
| 241 | 2 | | | | II-1 | 1700027H10Rik | | |
| 242 | 2 | | | | II-1 | 1700027I24Rik | | |
| 243 | 2 | | | | II-1 | 1700027J07Rik | | |
| 248 | 2 | | | | II-1 | 1700028J19Rik | | |
| 252 | 2 | | | | II-1 | 1700028P15Rik | | |
| 253 | 2 | | | | II-1 | 1700029B22Rik | | |
| 254 | 2 | | | | II-1 | 1700029F12Rik | | |
| 256 | 2 | | | | II-1 | 1700029I15Rik | | |
| 260 | 2 | | | | II-1 | 1700029N11Rik | | |
| 261 | 2 | | | | II-1 | 1700029P11Rik | | |
| 266 | 2 | | | | II-1 | 1700030J22Rik | | |
| 270 | 2 | | | | II-1 | 1700030N03Rik | | |
| 272 | 2 | | | | II-1 | 1700031A10Rik | | |
| 273 | 2 | | | | II-1 | 1700031F05Rik | | |

| | | | | | | | | |
|---|---|---|---|---|---|---|---|---|
| 274 | 2 | | | | II-1 | 1700031M16Rik | | |
| 276 | 2 | | | | II-1 | 1700034E13Rik | | |
| 277 | 2 | | | | II-1 | 1700034F02Rik | | |
| 278 | 2 | | | | II-1 | 1700034G24Rik | | |
| 279 | 2 | | | | II-1 | 1700034H15Rik | | |
| 280 | 2 | | | | II-1 | 1700034I23Rik | | |
| 281 | 2 | | | | II-1 | 1700034J05Rik | | |
| 282 | 2 | | | | II-1 | 1700034K08Rik | | |
| 283 | 2 | | | | II-1 | 1700034O15Rik | | |
| 285 | 2 | | | | II-1 | 1700036G14Rik | | |
| 290 | 2 | | | | II-1 | 1700040L02Rik | | |
| 293 | 2 | | | | II-1 | 1700042B14Rik | | |
| 294 | 2 | | | | II-1 | 1700042G07Rik | | |
| 296 | 2 | | | | II-1 | 1700042O10Rik | | |
| 298 | 2 | | | | II-1 | 1700044K03Rik | | |
| 299 | 2 | | | | II-1 | 1700045H11Rik | | |
| 301 | 2 | | | | II-1 | 1700047A11Rik | | |
| 302 | 2 | | | | II-1 | 1700047E10Rik | | |
| 306 | 2 | | | | II-1 | 1700047M11Rik | | |
| 307 | 2 | | | | II-1 | 1700048M11Rik | | |
| 311 | 2 | | | | II-1 | 1700049G17Rik | | |
| 314 | 2 | | | | II-1 | 1700052I22Rik | | |
| 315 | 2 | | | | II-1 | 1700052K11Rik | | |
| 316 | 2 | | | | II-1 | 1700052N19Rik | | |
| 319 | 2 | | | | II-1 | 1700054M17Rik | | |
| 321 | 2 | | | | II-1 | 1700055C04Rik | | |
| 322 | 2 | | | | II-1 | 1700055N04Rik | | |
| 323 | 2 | | | | II-1 | 1700056E22Rik | | |
| 326 | 2 | | | | II-1 | 1700060C16Rik | | |
| 327 | 2 | | | | II-1 | 1700060C20Rik | | |
| 330 | 2 | | | | II-1 | 1700061I17Rik | | |
| 331 | 2 | | | | II-1 | 1700063A18Rik | | |
| 332 | 2 | | | | II-1 | 1700063D05Rik | | |
| 333 | 2 | | | | II-1 | 1700063O14Rik | | |
| 335 | 2 | | | | II-1 | 1700064M15Rik | | |
| 340 | 2 | | | | II-1 | 1700065L07Rik | | |
| 341 | 2 | | | | II-1 | 1700065O20Rik | | |
| 342 | 2 | | | | II-1 | 1700066B17Rik | | |
| 347 | 2 | | | | II-1 | 1700067G17Rik | | |
| 350 | 2 | | | | II-1 | 1700069L16Rik | | |
| 351 | 2 | | | | II-1 | 1700069P05Rik | | |
| 352 | 2 | | | | II-1 | 1700071K01Rik | | |
| 356 | 2 | | | | II-1 | 1700073E17Rik | | |
| 359 | 2 | | | | II-1 | 1700080E11Rik | | |
| 360 | 2 | | | | II-1 | 1700080N15Rik | | |
| 361 | 2 | | | | II-1 | 1700080O16Rik | | |
| 362 | 2 | | | | II-1 | 1700081H04Rik | | |
| 368 | 2 | | | | II-1 | 1700086L19Rik | | |
| 375 | 2 | | | | II-1 | 1700092K14Rik | | |
| 380 | 2 | | | | II-1 | 1700094M24Rik | | |
| 381 | 2 | | | | II-1 | 1700095A21Rik | | |
| 382 | 2 | | | | II-1 | 1700095B10Rik | | |
| 396 | 2 | | | | II-1 | 1700108J01Rik | | |
| 397 | 2 | | | | II-1 | 1700109G14Rik | | |
| 398 | 2 | | | | II-1 | 1700109G15Rik | | |
| 399 | 2 | | | | II-1 | 1700109H08Rik | | |
| 400 | 2 | | | | II-1 | 1700109I08Rik | | |
| 401 | 2 | | | | II-1 | 1700109K24Rik | | |
| 409 | 2 | | | | II-1 | 1700113A16Rik | | |
| 418 | 2 | | | | II-1 | 1700122O11Rik | | |
| 421 | 2 | | | | II-1 | 1700123L14Rik | | |
| 425 | 2 | | | | II-1 | 1700123O21Rik | | |
| 429 | 2 | | | | II-1 | 1700125H03Rik | | |
| 432 | 2 | | | | II-1 | 1700128A07Rik | | |
| 433 | 2 | | | | II-1 | 1700128F08Rik | | |
| 435 | 2 | | | | II-1 | 1810006J02Rik | | |
| 437 | 2 | | | | II-1 | 1810007D17Rik | | |
| 456 | 2 | | | | II-1 | 1810026J23Rik | | |
| 474 | 2 | | | | II-1 | 2010002M12Rik | | |
| 494 | 2 | | | | II-1 | 2010315B03Rik | | |
| 495 | 2 | | | | II-1 | 2010320M18Rik | | |
| 504 | 2 | | | | II-1 | 2210018M11Rik | | |
| 505 | 2 | | | | II-1 | 2210019I11Rik | | |
| 530 | 2 | | | | II-1 | 2310005G13Rik | | |
| 539 | 2 | | | | II-1 | 2310015A10Rik | | |
| 546 | 2 | | | | II-1 | 2310030A07Rik | | |
| 554 | 2 | | | | II-1 | 2310039H08Rik | | |
| 559 | 2 | | | | II-1 | 2310043O21Rik | | |
| 560 | 2 | | | | II-1 | 2310045N01Rik | | |
| 566 | 2 | | | | II-1 | 2310061I04Rik | | |
| 578 | 2 | | | | II-1 | 2410004B18Rik | | |
| 579 | 2 | | | | II-1 | 2410004I01Rik | | |
| 585 | 2 | | | | II-1 | 2410012M07Rik | | |
| 590 | 2 | | | | II-1 | 2410021H03Rik | | |
| 591 | 2 | | | | II-1 | 2410076I21Rik | | |
| 594 | 2 | | | | II-1 | 2410114N07Rik | | |
| 596 | 2 | | | | II-1 | 2410127L17Rik | | |
| 598 | 2 | | | | II-1 | 2410137M14Rik | | |
| 600 | 2 | | | | II-1 | 2500004C02Rik | | |
| 604 | 2 | | | | II-1 | 2510039O18Rik | | |
| 605 | 2 | | | | II-1 | 2510049J12Rik | | |
| 607 | 2 | | | | II-1 | 2610002J02Rik | | |
| 608 | 2 | | | | II-1 | 2610002M06Rik | | |
| 609 | 2 | | | | II-1 | 2610005L07Rik | | |
| 617 | 2 | | | | II-1 | 2610028E06Rik | | |
| 619 | 2 | | | | II-1 | 2610034B18Rik | | |
| 628 | 2 | | | | II-1 | 2610206C17Rik | | |

Fig.22 - 93

| | | | | | | | | |
|---|---|---|---|---|---|---|---|---|
| 630 | 2 | | | | II-1 | 2610301B20Rik | | |
| 635 | 2 | | | | II-1 | 2610318N02Rik | | |
| 647 | 2 | | | | II-1 | 2700060E02Rik | | |
| 649 | 2 | | | | II-1 | 2700069I18Rik | | |
| 652 | 2 | | | | II-1 | 2700086A05Rik | | |
| 660 | 2 | | | | II-1 | 2810004N23Rik | | |
| 672 | 2 | | | | II-1 | 2810403A07Rik | | |
| 673 | 2 | | | | II-1 | 2810403D21Rik | | |
| 683 | 2 | | | | II-1 | 2810433D01Rik | | |
| 699 | 2 | | | | II-1 | 2900056M20Rik | | |
| 722 | 2 | | | | II-1 | 3110039M20Rik | | |
| 723 | 2 | | | | II-1 | 3110040N11Rik | | |
| 735 | 2 | | | | II-1 | 3200001D21Rik | | |
| 736 | 2 | | | | II-1 | 3300002I08Rik | | |
| 743 | 2 | | | | II-1 | 3830408C21Rik | | |
| 745 | 2 | | | | II-1 | 3930402G23Rik | | |
| 748 | 2 | | | | II-1 | 4632415L05Rik | | |
| 762 | 2 | | | | II-1 | 4833417C18Rik | | |
| 769 | 2 | | | | II-1 | 4833427F10Rik | | |
| 771 | 2 | | | | II-1 | 4833428L15Rik | | |
| 772 | 2 | | | | II-1 | 4833439L19Rik | | |
| 773 | 2 | | | | II-1 | 4921501E09Rik | | |
| 774 | 2 | | | | II-1 | 4921504A21Rik | | |
| 775 | 2 | | | | II-1 | 4921504E06Rik | | |
| 776 | 2 | | | | II-1 | 4921506M07Rik | | |
| 777 | 2 | | | | II-1 | 4921507L20Rik | | |
| 778 | 2 | | | | II-1 | 4921507P07Rik | | |
| 780 | 2 | | | | II-1 | 4921509C19Rik | | |
| 781 | 2 | | | | II-1 | 4921509O07Rik | | |
| 783 | 2 | | | | II-1 | 4921511C20Rik | | |
| 787 | 2 | | | | II-1 | 4921513I03Rik | | |
| 788 | 2 | | | | II-1 | 4921515E04Rik | | |
| 789 | 2 | | | | II-1 | 4921517D22Rik | | |
| 790 | 2 | | | | II-1 | 4921524J17Rik | | |
| 793 | 2 | | | | II-1 | 4921529L05Rik | | |
| 794 | 2 | | | | II-1 | 4921530L21Rik | | |
| 795 | 2 | | | | II-1 | 4921531C22Rik | | |
| 801 | 2 | | | | II-1 | 4922502D21Rik | | |
| 804 | 2 | | | | II-1 | 4930401C15Rik | | |
| 805 | 2 | | | | II-1 | 4930401O10Rik | | |
| 808 | 2 | | | | II-1 | 4930402F11Rik | | |
| 809 | 2 | | | | II-1 | 4930402H24Rik | | |
| 811 | 2 | | | | II-1 | 4930404A05Rik | | |
| 812 | 2 | | | | II-1 | 4930404A10Rik | | |
| 813 | 2 | | | | II-1 | 4930404H11Rik | | |
| 820 | 2 | | | | II-1 | 4930405L22Rik | | |
| 822 | 2 | | | | II-1 | 4930407I10Rik | | |
| 823 | 2 | | | | II-1 | 4930412B13Rik | | |
| 824 | 2 | | | | II-1 | 4930412C18Rik | | |
| 832 | 2 | | | | II-1 | 4930414N06Rik | | |
| 834 | 2 | | | | II-1 | 4930415L06Rik | | |
| 835 | 2 | | | | II-1 | 4930415O20Rik | | |
| 836 | 2 | | | | II-1 | 4930417O13Rik | | |
| 839 | 2 | | | | II-1 | 4930423M02Rik | | |
| 840 | 2 | | | | II-1 | 4930425K10Rik | | |
| 847 | 2 | | | | II-1 | 4930428G15Rik | | |
| 851 | 2 | | | | II-1 | 4930429F11Rik | | |
| 855 | 2 | | | | II-1 | 4930430F08Rik | | |
| 858 | 2 | | | | II-1 | 4930431F12Rik | | |
| 860 | 2 | | | | II-1 | 4930432J09Rik | | |
| 862 | 2 | | | | II-1 | 4930432M17Rik | | |
| 863 | 2 | | | | II-1 | 4930433B08Rik | | |
| 864 | 2 | | | | II-1 | 4930433I11Rik | | |
| 866 | 2 | | | | II-1 | 4930434J06Rik | | |
| 868 | 2 | | | | II-1 | 4930438E09Rik | | |
| 869 | 2 | | | | II-1 | 4930440C22Rik | | |
| 871 | 2 | | | | II-1 | 4930441J16Rik | | |
| 876 | 2 | | | | II-1 | 4930444G20Rik | | |
| 877 | 2 | | | | II-1 | 4930444M15Rik | | |
| 879 | 2 | | | | II-1 | 4930447A16Rik | | |
| 883 | 2 | | | | II-1 | 4930447N08Rik | | |
| 884 | 2 | | | | II-1 | 4930448C13Rik | | |
| 886 | 2 | | | | II-1 | 4930448H16Rik | | |
| 889 | 2 | | | | II-1 | 4930448K20Rik | | |
| 890 | 2 | | | | II-1 | 4930449E01Rik | | |
| 892 | 2 | | | | II-1 | 4930449I24Rik | | |
| 893 | 2 | | | | II-1 | 4930451C15Rik | | |
| 899 | 2 | | | | II-1 | 4930452N14Rik | | |
| 900 | 2 | | | | II-1 | 4930453H23Rik | | |
| 901 | 2 | | | | II-1 | 4930453L07Rik | | |
| 907 | 2 | | | | II-1 | 4930455H04Rik | | |
| 908 | 2 | | | | II-1 | 4930455J16Rik | | |
| 909 | 2 | | | | II-1 | 4930456L15Rik | | |
| 916 | 2 | | | | II-1 | 4930467D21Rik | | |
| 917 | 2 | | | | II-1 | 4930467E23Rik | | |
| 918 | 2 | | | | II-1 | 4930467K11Rik | | |
| 920 | 2 | | | | II-1 | 4930469G21Rik | | |
| 922 | 2 | | | | II-1 | 4930470P17Rik | | |
| 923 | 2 | | | | II-1 | 4930471C04Rik | | |
| 924 | 2 | | | | II-1 | 4930471G03Rik | | |
| 925 | 2 | | | | II-1 | 4930471M09Rik | | |
| 926 | 2 | | | | II-1 | 4930473A02Rik | | |
| 927 | 2 | | | | II-1 | 4930473O22Rik | | |
| 928 | 2 | | | | II-1 | 4930474G06Rik | | |
| 929 | 2 | | | | II-1 | 4930474H20Rik | | |
| 933 | 2 | | | | II-1 | 4930478L05Rik | | |
| 934 | 2 | | | | II-1 | 4930478P22Rik | | |
| 935 | 2 | | | | II-1 | 4930479D17Rik | | |
| 937 | 2 | | | | II-1 | 4930480G23Rik | | |
| 939 | 2 | | | | II-1 | 4930480M12Rik | | |
| 943 | 2 | | | | II-1 | 4930483K19Rik | | |
| 945 | 2 | | | | II-1 | 4930486F22Rik | | |
| 946 | 2 | | | | II-1 | 4930486I03Rik | | |
| 951 | 2 | | | | II-1 | 4930488L21Rik | | |
| 952 | 2 | | | | II-1 | 4930500F04Rik | | |
| 954 | 2 | | | | II-1 | 4930500L23Rik | | |
| 955 | 2 | | | | II-1 | 4930502A04Rik | | |
| 960 | 2 | | | | II-1 | 4930503E24Rik | | |
| 961 | 2 | | | | II-1 | 4930503H13Rik | | |
| 962 | 2 | | | | II-1 | 4930503L19Rik | | |
| 963 | 2 | | | | II-1 | 4930503O07Rik | | |
| 965 | 2 | | | | II-1 | 4930505A04Rik | | |
| 966 | 2 | | | | II-1 | 4930505G20Rik | | |
| 971 | 2 | | | | II-1 | 4930509E16Rik | | |
| 973 | 2 | | | | II-1 | 4930509K18Rik | | |
| 974 | 2 | | | | II-1 | 4930511A02Rik | | |
| 979 | 2 | | | | II-1 | 4930513N10Rik | | |
| 982 | 2 | | | | II-1 | 4930515G01Rik | | |
| 985 | 2 | | | | II-1 | 4930515L19Rik | | |
| 986 | 2 | | | | II-1 | 4930517E11Rik | | |
| 992 | 2 | | | | II-1 | 4930519G04Rik | | |
| 993 | 2 | | | | II-1 | 4930519H02Rik | | |
| 997 | 2 | | | | II-1 | 4930522H14Rik | | |
| 998 | 2 | | | | II-1 | 4930522O17Rik | | |
| 1001 | 2 | | | | II-1 | 4930524B15Rik | | |
| 1002 | 2 | | | | II-1 | 4930524C18Rik | | |
| 1004 | 2 | | | | II-1 | 4930524O05Rik | | |
| 1005 | 2 | | | | II-1 | 4930524O08Rik | | |
| 1008 | 2 | | | | II-1 | 4930525M21Rik | | |
| 1009 | 2 | | | | II-1 | 4930526I15Rik | | |
| 1012 | 2 | | | | II-1 | 4930527G23Rik | | |
| 1013 | 2 | | | | II-1 | 4930528A17Rik | | |
| 1014 | 2 | | | | II-1 | 4930528D03Rik | | |
| 1015 | 2 | | | | II-1 | 4930528P14Rik | | |
| 1016 | 2 | | | | II-1 | 4930529C04Rik | | |
| 1023 | 2 | | | | II-1 | 4930538K18Rik | | |
| 1029 | 2 | | | | II-1 | 4930540M03Rik | | |
| 1035 | 2 | | | | II-1 | 4930544M13Rik | | |
| 1036 | 2 | | | | II-1 | 4930545E07Rik | | |
| 1039 | 2 | | | | II-1 | 4930546C10Rik | | |
| 1040 | 2 | | | | II-1 | 4930546K05Rik | | |
| 1041 | 2 | | | | II-1 | 4930547E08Rik | | |
| 1043 | 2 | | | | II-1 | 4930548G14Rik | | |
| 1045 | 2 | | | | II-1 | 4930548J01Rik | | |
| 1046 | 2 | | | | II-1 | 4930548K13Rik | | |
| 1049 | 2 | | | | II-1 | 4930550C14Rik | | |
| 1051 | 2 | | | | II-1 | 4930552N02Rik | | |
| 1052 | 2 | | | | II-1 | 4930552P12Rik | | |
| 1053 | 2 | | | | II-1 | 4930553E22Rik | | |
| 1055 | 2 | | | | II-1 | 4930555B11Rik | | |
| 1056 | 2 | | | | II-1 | 4930555G01Rik | | |
| 1058 | 2 | | | | II-1 | 4930556G01Rik | | |
| 1059 | 2 | | | | II-1 | 4930556J02Rik | | |
| 1060 | 2 | | | | II-1 | 4930556M19Rik | | |
| 1063 | 2 | | | | II-1 | 4930557J02Rik | | |
| 1065 | 2 | | | | II-1 | 4930558G05Rik | | |
| 1067 | 2 | | | | II-1 | 4930558K02Rik | | |
| 1072 | 2 | | | | II-1 | 4930563E22Rik | | |
| 1073 | 2 | | | | II-1 | 4930563F08Rik | | |
| 1083 | 2 | | | | II-1 | 4930567K20Rik | | |
| 1084 | 2 | | | | II-1 | 4930568D16Rik | | |
| 1086 | 2 | | | | II-1 | 4930568G15Rik | | |
| 1087 | 2 | | | | II-1 | 4930570G19Rik | | |
| 1091 | 2 | | | | II-1 | 4930572O03Rik | | |
| 1092 | 2 | | | | II-1 | 4930572O13Rik | | |
| 1093 | 2 | | | | II-1 | 4930573O16Rik | | |
| 1094 | 2 | | | | II-1 | 4930577N17Rik | | |
| 1098 | 2 | | | | II-1 | 4930578M01Rik | | |
| 1101 | 2 | | | | II-1 | 4930579G18Rik | | |
| 1102 | 2 | | | | II-1 | 4930579G24Rik | | |
| 1107 | 2 | | | | II-1 | 4930584F24Rik | | |
| 1108 | 2 | | | | II-1 | 4930590J08Rik | | |
| 1109 | 2 | | | | II-1 | 4930590L20Rik | | |
| 1110 | 2 | | | | II-1 | 4930591A17Rik | | |
| 1114 | 2 | | | | II-1 | 4930593C16Rik | | |
| 1115 | 2 | | | | II-1 | 4930594C11Rik | | |
| 1116 | 2 | | | | II-1 | 4930595M18Rik | | |
| 1118 | 2 | | | | II-1 | 4930596I21Rik | | |
| 1119 | 2 | | | | II-1 | 4930597G03Rik | | |
| 1120 | 2 | | | | II-1 | 4930598F16Rik | | |
| 1121 | 2 | | | | II-1 | 4930599N23Rik | | |
| 1126 | 2 | | | | II-1 | 4931406C07Rik | | |
| 1128 | 2 | | | | II-1 | 4931406P16Rik | | |
| 1129 | 2 | | | | II-1 | 4931408C20Rik | | |
| 1130 | 2 | | | | II-1 | 4931408D14Rik | | |
| 1133 | 2 | | | | II-1 | 4931414P19Rik | | |
| 1134 | 2 | | | | II-1 | 4931417E11Rik | | |
| 1136 | 2 | | | | II-1 | 4931420L22Rik | | |
| 1141 | 2 | | | | II-1 | 4931429L15Rik | | |
| 1142 | 2 | | | | II-1 | 4931429P17Rik | | |
| 1143 | 2 | | | | II-1 | 4931430N09Rik | | |
| 1144 | 2 | | | | II-1 | 4931431B13Rik | | |
| 1149 | 2 | | | | II-1 | 4931440L10Rik | | |
| 1150 | 2 | | | | II-1 | 4931440P22Rik | | |

Fig.22 - 94

| | | | | | | | | |
|---|---|---|---|---|---|---|---|---|
| 1151 | 2 | | | | II-1 | 4932411E22Rik | | |
| 1153 | 2 | | | | II-1 | 4932412D23Rik | | |
| 1155 | 2 | | | | II-1 | 4932414J04Rik | | |
| 1156 | 2 | | | | II-1 | 4932414N04Rik | | |
| 1157 | 2 | | | | II-1 | 4932415M13Rik | | |
| 1159 | 2 | | | | II-1 | 4932416K20Rik | | |
| 1163 | 2 | | | | II-1 | 4932438A13Rik | | |
| 1167 | 2 | | | | II-1 | 4932702P03Rik | | |
| 1170 | 2 | | | | II-1 | 4933400C23Rik | | |
| 1171 | 2 | | | | II-1 | 4933400F21Rik | | |
| 1176 | 2 | | | | II-1 | 4933402C06Rik | | |
| 1177 | 2 | | | | II-1 | 4933402D24Rik | | |
| 1178 | 2 | | | | II-1 | 4933402E13Rik | | |
| 1179 | 2 | | | | II-1 | 4933402J07Rik | | |
| 1180 | 2 | | | | II-1 | 4933402J10Rik | | |
| 1181 | 2 | | | | II-1 | 4933402J15Rik | | |
| 1182 | 2 | | | | II-1 | 4933402N03Rik | | |
| 1183 | 2 | | | | II-1 | 4933402N22Rik | | |
| 1186 | 2 | | | | II-1 | 4933404G15Rik | | |
| 1187 | 2 | | | | II-1 | 4933404K08Rik | | |
| 1191 | 2 | | | | II-1 | 4933405L10Rik | | |
| 1195 | 2 | | | | II-1 | 4933406F09Rik | | |
| 1196 | 2 | | | | II-1 | 4933406G16Rik | | |
| 1197 | 2 | | | | II-1 | 4933406I18Rik | | |
| 1200 | 2 | | | | II-1 | 4933406K04Rik | | |
| 1201 | 2 | | | | II-1 | 4933406M09Rik | | |
| 1202 | 2 | | | | II-1 | 4933407E24Rik | | |
| 1203 | 2 | | | | II-1 | 4933407G14Rik | | |
| 1204 | 2 | | | | II-1 | 4933407I05Rik | | |
| 1210 | 2 | | | | II-1 | 4933409G03Rik | | |
| 1211 | 2 | | | | II-1 | 4933409K07Rik | | |
| 1214 | 2 | | | | II-1 | 4933411G11Rik | | |
| 1220 | 2 | | | | II-1 | 4933413G19Rik | | |
| 1222 | 2 | | | | II-1 | 4933413L06Rik | | |
| 1225 | 2 | | | | II-1 | 4933416E03Rik | | |
| 1226 | 2 | | | | II-1 | 4933416I08Rik | | |
| 1229 | 2 | | | | II-1 | 4933417A18Rik | | |
| 1234 | 2 | | | | II-1 | 4933421I07Rik | | |
| 1235 | 2 | | | | II-1 | 4933421O10Rik | | |
| 1237 | 2 | | | | II-1 | 4933422H20Rik | | |
| 1239 | 2 | | | | II-1 | 4933424G06Rik | | |
| 1240 | 2 | | | | II-1 | 4933425B07Rik | | |
| 1242 | 2 | | | | II-1 | 4933426M11Rik | | |
| 1243 | 2 | | | | II-1 | 4933427D06Rik | | |
| 1245 | 2 | | | | II-1 | 4933427E11Rik | | |
| 1247 | 2 | | | | II-1 | 4933427G17Rik | | |
| 1250 | 2 | | | | II-1 | 4933428G20Rik | | |
| 1251 | 2 | | | | II-1 | 4933429K18Rik | | |
| 1254 | 2 | | | | II-1 | 4933430I17Rik | | |
| 1255 | 2 | | | | II-1 | 4933430M04Rik | | |
| 1257 | 2 | | | | II-1 | 4933431E20Rik | | |
| 1260 | 2 | | | | II-1 | 4933432I03Rik | | |
| 1261 | 2 | | | | II-1 | 4933432I09Rik | | |
| 1264 | 2 | | | | II-1 | 4933433F19Rik | | |
| 1266 | 2 | | | | II-1 | 4933433G15Rik | | |
| 1268 | 2 | | | | II-1 | 4933433H22Rik | | |
| 1269 | 2 | | | | II-1 | 4933434E20Rik | | |
| 1270 | 2 | | | | II-1 | 4933434I20Rik | | |
| 1271 | 2 | | | | II-1 | 4933436E23Rik | | |
| 1272 | 2 | | | | II-1 | 4933436H12Rik | | |
| 1273 | 2 | | | | II-1 | 4933436I01Rik | | |
| 1275 | 2 | | | | II-1 | 4933438K21Rik | | |
| 1276 | 2 | | | | II-1 | 4933439C10Rik | | |
| 1280 | 2 | | | | II-1 | 5031410I06Rik | | |
| 1282 | 2 | | | | II-1 | 5031425E22Rik | | |
| 1284 | 2 | | | | II-1 | 5031426D15Rik | | |
| 1285 | 2 | | | | II-1 | 5031434C07Rik | | |
| 1286 | 2 | | | | II-1 | 5031434O11Rik | | |
| 1293 | 2 | | | | II-1 | 5330413P13Rik | | |
| 1295 | 2 | | | | II-1 | 5330426P16Rik | | |
| 1299 | 2 | | | | II-1 | 5430402E10Rik | | |
| 1300 | 2 | | | | II-1 | 5430402O13Rik | | |
| 1306 | 2 | | | | II-1 | 5430419D17Rik | | |
| 1307 | 2 | | | | II-1 | 5430421F17Rik | | |
| 1312 | 2 | | | | II-1 | 5430428K19Rik | | |
| 1313 | 2 | | | | II-1 | 5430434I15Rik | | |
| 1317 | 2 | | | | II-1 | 5530400C23Rik | | |
| 1318 | 2 | | | | II-1 | 5530401A14Rik | | |
| 1332 | 2 | | | | II-1 | 5730480H06Rik | | |
| 1344 | 2 | | | | II-1 | 5830418K08Rik | | |
| 1356 | 2 | | | | II-1 | 6030408B16Rik | | |
| 1360 | 2 | | | | II-1 | 6030458C11Rik | | |
| 1364 | 2 | | | | II-1 | 6030498E09Rik | | |
| 1365 | 2 | | | | II-1 | 6230400D17Rik | | |
| 1370 | 2 | | | | II-1 | 6330409D20Rik | | |
| 1380 | 2 | | | | II-1 | 6430548M08Rik | | |
| 1382 | 2 | | | | II-1 | 6430562O15Rik | | |
| 1387 | 2 | | | | II-1 | 6430710C18Rik | | |
| 1388 | 2 | | | | II-1 | 6530402F18Rik | | |
| 1394 | 2 | | | | II-1 | 6820408C15Rik | | |
| 1398 | 2 | | | | II-1 | 7420700N18Rik | | |
| 1400 | 2 | | | | II-1 | 7530416G11Rik | | |
| 1405 | 2 | | | | II-1 | 8030442B05Rik | | |
| 1411 | 2 | | | | II-1 | 8430423G03Rik | | |
| 1412 | 2 | | | | II-1 | 8430426J06Rik | | |
| 1417 | 2 | | | | II-1 | 8430437L04Rik | | |
| 1421 | 2 | | | | II-1 | 9030612E09Rik | | |
| 1426 | 2 | | | | II-1 | 9030625G05Rik | | |
| 1431 | 2 | | | | II-1 | 9130019O22Rik | | |
| 1438 | 2 | | | | II-1 | 9130221H12Rik | | |
| 1439 | 2 | | | | II-1 | 9130227L01Rik | | |
| 1451 | 2 | | | | II-1 | 9230112J17Rik | | |
| 1452 | 2 | | | | II-1 | 9230114K14Rik | | |
| 1456 | 2 | | | | II-1 | 9330102E08Rik | | |
| 1462 | 2 | | | | II-1 | 9330159F19Rik | | |
| 1469 | 2 | | | | II-1 | 9330179D12Rik | | |
| 1473 | 2 | | | | II-1 | 9430007A20Rik | | |
| 1479 | 2 | | | | II-1 | 9430019J16Rik | | |
| 1480 | 2 | | | | II-1 | 9430020K01Rik | | |
| 1483 | 2 | | | | II-1 | 9430038I01Rik | | |
| 1487 | 2 | | | | II-1 | 9430076C15Rik | | |
| 1488 | 2 | | | | II-1 | 9430083A17Rik | | |
| 1492 | 2 | | | | II-1 | 9530026F06Rik | | |
| 1501 | 2 | | | | II-1 | 9530077C05Rik | | |
| 1505 | 2 | | | | II-1 | 9630001P10Rik | | |
| 1506 | 2 | | | | II-1 | 9630013A20Rik | | |
| 1507 | 2 | | | | II-1 | 9630028B13Rik | | |
| 1509 | 2 | | | | II-1 | 9630033F20Rik | | |
| 1519 | 2 | | | | II-1 | 9930111J21Rik1 | | |
| 1521 | 2 | | | | II-1 | a | | |
| 1524 | 2 | | | | II-1 | A1bg | 1 | 4-May-15 |
| 1525 | 2 | | | | II-1 | A1cf | 29974 | 4-May-15 |
| 1530 | 2 | | | | II-1 | A230046K03Rik | | |
| 1531 | 2 | | | | II-1 | A230050P20Rik | | |
| 1540 | 2 | | | | II-1 | A230077H06Rik | | |
| 1544 | 2 | | | | II-1 | A330009N23Rik | | |
| 1548 | 2 | | | | II-1 | A330033J07Rik | | |
| 1549 | 2 | | | | II-1 | A330035P11Rik | | |
| 1555 | 2 | | | | II-1 | A330069E16Rik | | |
| 1562 | 2 | | | | II-1 | A3galt2 | 127550 | 4-May-15 |
| 1564 | 2 | | | | II-1 | A430033K04Rik | | |
| 1568 | 2 | | | | II-1 | A430089I19Rik | | |
| 1569 | 2 | | | | II-1 | A430090L17Rik | | |
| 1570 | 2 | | | | II-1 | A430093F15Rik | | |
| 1580 | 2 | | | | II-1 | A530050N04Rik | | |
| 1585 | 2 | | | | II-1 | A530065N20Rik | | |
| 1593 | 2 | | | | II-1 | A630019I02Rik | | |
| 1594 | 2 | | | | II-1 | A630020A06 | | |
| 1602 | 2 | | | | II-1 | A630076J17Rik | | |
| 1616 | 2 | | | | II-1 | A730046J19Rik | | |
| 1617 | 2 | | | | II-1 | A730056A06Rik | | |
| 1619 | 2 | | | | II-1 | A730085K08Rik | | |
| 1620 | 2 | | | | II-1 | A730090H04Rik | | |
| 1623 | 2 | | | | II-1 | A830009L08Rik | | |
| 1624 | 2 | | | | II-1 | A830010M20Rik | | |
| 1628 | 2 | | | | II-1 | A830080D01Rik | | |
| 1631 | 2 | | | | II-1 | A930001A20Rik | | |
| 1632 | 2 | | | | II-1 | A930001C03Rik | | |
| 1636 | 2 | | | | II-1 | A930005H10Rik | | |
| 1641 | 2 | | | | II-1 | A930011G23Rik | | |
| 1653 | 2 | | | | II-1 | AA388235 | | |
| 1662 | 2 | | | | II-1 | AA543401 | | |
| 1664 | 2 | | | | II-1 | AA619741 | | |
| 1677 | 2 | | | | II-1 | Aamdc | 28971 | 4-May-15 |
| 1678 | 2 | | | | II-1 | Aamp | 14 | 12-May-15 |
| 1680 | 2 | | | | II-1 | Aar2 | 25980 | 4-May-15 |
| 1681 | 2 | | | | II-1 | Aard | 441376 | 4-May-15 |
| 1684 | 2 | | | | II-1 | Aarsd1 | 80755 | 4-May-15 |
| 1685 | 2 | | | | II-1 | Aasdh | 132949 | 4-May-15 |
| 1696 | 2 | | | | II-1 | Abca14 | | |
| 1697 | 2 | | | | II-1 | Abca15 | | |
| 1698 | 2 | | | | II-1 | Abca16 | | |
| 1699 | 2 | | | | II-1 | Abca17 | 650655 | 12-May-15 |
| 1702 | 2 | | | | II-1 | Abca4 | 24 | 23-May-15 |
| 1717 | 2 | | | | II-1 | Abcb8 | 11194 | 12-May-15 |
| 1726 | 2 | | | | II-1 | Abcc6 | 368 | 23-May-15 |
| 1735 | 2 | | | | II-1 | Abcf2 | 10061 | 12-May-15 |
| 1736 | 2 | | | | II-1 | Abcf3 | 55324 | 4-May-15 |
| 1737 | 2 | | | | II-1 | Abcg1 | 9619 | 24-May-15 |
| 1743 | 2 | | | | II-1 | Abhd1 | 84696 | 4-May-15 |
| 1745 | 2 | | | | II-1 | Abhd11 | 83451 | 4-May-15 |
| 1750 | 2 | | | | II-1 | Abhd14a | 25864 | 4-May-15 |
| 1755 | 2 | | | | II-1 | Abhd17a | 81926 | 4-May-15 |
| 1756 | 2 | | | | II-1 | Abhd17b | 51104 | 4-May-15 |
| 1757 | 2 | | | | II-1 | Abhd17c | 58489 | 4-May-15 |
| 1790 | 2 | | | | II-1 | Acadl | 33 | 4-May-15 |
| 1799 | 2 | | | | II-1 | Acat1 | 38 | 7-Jun-15 |
| 1800 | 2 | | | | II-1 | Acat2 | 39 | 7-Jun-15 |
| 1803 | 2 | | | | II-1 | Acbd4 | 79777 | 4-May-15 |
| 1804 | 2 | | | | II-1 | Acbd5 | 91452 | 12-May-15 |
| 1809 | 2 | | | | II-1 | Acd | 65057 | 4-May-15 |
| 1814 | 2 | | | | II-1 | Acer2 | 340485 | 4-May-15 |
| 1883 | 2 | | | | II-1 | Actl6b | 51412 | 1-Jun-15 |
| 1884 | 2 | | | | II-1 | Actl7a | 10881 | 4-May-15 |
| 1886 | 2 | | | | II-1 | Actl9 | 284382 | 4-May-15 |
| 1892 | 2 | | | | II-1 | Actr1a | 10121 | 4-May-15 |
| 1893 | 2 | | | | II-1 | Actr1b | 10120 | 4-May-15 |
| 1894 | 2 | | | | II-1 | Actr2 | 10097 | 4-May-15 |
| 1895 | 2 | | | | II-1 | Actr3 | 10096 | 4-May-15 |
| 1896 | 2 | | | | II-1 | Actr3b | 57180 | 4-May-15 |
| 1897 | 2 | | | | II-1 | Actr5 | 79913 | 4-May-15 |
| 1901 | 2 | | | | II-1 | Actrt2 | 140625 | 4-May-15 |
| 1902 | 2 | | | | II-1 | Actrt3 | 84517 | 4-May-15 |
| 1903 | 2 | | | | II-1 | Acvr1 | 90 | 12-May-15 |

Fig.22 - 95

| | | | | | | | | |
|---|---|---|---|---|---|---|---|---|
| 1908 | 2 | | | | II-1 | Acvrl1 | 94 | 23-May-15 |
| 1915 | 2 | | | | II-1 | Adad2 | 161931 | 4-May-15 |
| 1916 | 2 | | | | II-1 | Adal | 161823 | 4-May-15 |
| 1922 | 2 | | | | II-1 | Adam18 | 8749 | May-15 |
| 1924 | 2 | | | | II-1 | Adam1a | 8759 | 4-May-15 |
| 1928 | 2 | | | | II-1 | Adam21 | 8747 | 4-May-15 |
| 1929 | 2 | | | | II-1 | Adam22 | 53616 | 4-May-15 |
| 1933 | 2 | | | | II-1 | Adam26a | | |
| 1934 | 2 | | | | II-1 | Adam26b | | |
| 1937 | 2 | | | | II-1 | Adam3 | 1587 | 4-May-15 |
| 1938 | 2 | | | | II-1 | Adam30 | 11085 | 4-May-15 |
| 1939 | 2 | | | | II-1 | Adam32 | 203102 | 4-May-15 |
| 1940 | 2 | | | | II-1 | Adam33 | 80332 | 4-May-15 |
| 1941 | 2 | | | | II-1 | Adam34 | | |
| 1942 | 2 | | | | II-1 | Adam39 | | |
| 1943 | 2 | | | | II-1 | Adam4 | | |
| 1945 | 2 | | | | II-1 | Adam6a | | |
| 1946 | 2 | | | | II-1 | Adam6b | | |
| 1959 | 2 | | | | II-1 | Adamts18 | 170692 | 12-May-15 |
| 1961 | 2 | | | | II-1 | Adamts2 | 9509 | 7-Jun-15 |
| 1964 | 2 | | | | II-1 | Adamts4 | 9507 | 7-Jun-15 |
| 1980 | 2 | | | | II-1 | Adat1 | 23536 | 4-May-15 |
| 1981 | 2 | | | | II-1 | Adat2 | 134637 | 23-May-15 |
| 1988 | 2 | | | | II-1 | Adck5 | 203054 | 4-May-15 |
| 1999 | 2 | | | | II-1 | Adcyap1 | 116 | 17-May-15 |
| 2007 | 2 | | | | II-1 | Adh5 | 128 | 7-Jun-15 |
| 2017 | 2 | | | | II-1 | Adk | 132 | 4-May-15 |
| 2032 | 2 | | | | II-1 | Adra1a | 148 | 7-Jun-15 |
| 2050 | 2 | | | | II-1 | Aen | 64782 | 23-May-15 |
| 2057 | 2 | | | | II-1 | AF357399 | | |
| 2062 | 2 | | | | II-1 | Afap1 | 60312 | 4-May-15 |
| 2063 | 2 | | | | II-1 | Afap1l1 | 134265 | 4-May-15 |
| 2067 | 2 | | | | II-1 | Aff3 | 3899 | 4-May-15 |
| 2070 | 2 | | | | II-1 | Afg3l2 | 10939 | 23-May-15 |
| 2071 | 2 | | | | II-1 | Afm | 173 | 4-May-15 |
| 2080 | 2 | | | | II-1 | Agbl2 | 79841 | 23-May-15 |
| 2086 | 2 | | | | II-1 | Agfg2 | 3268 | 4-May-15 |
| 2088 | 2 | | | | II-1 | Agk | 55750 | 4-May-15 |
| 2129 | 2 | | | | II-1 | AI118078 | | |
| 2133 | 2 | | | | II-1 | AI314278 | | |
| 2149 | 2 | | | | II-1 | AI606473 | | |
| 2150 | 2 | | | | II-1 | AI607873 | | |
| 2162 | 2 | | | | II-1 | AI987944 | | |
| 2176 | 2 | | | | II-1 | Aip | 9049 | 7-Jun-15 |
| 2177 | 2 | | | | II-1 | Aipl1 | 23746 | 23-May-15 |
| 2178 | 2 | | | | II-1 | Aire | 326 | 28-May-15 |
| 2184 | 2 | | | | II-1 | AK129341 | | |
| 2192 | 2 | | | | II-1 | Akap1 | 8165 | 4-May-15 |
| 2193 | 2 | | | | II-1 | Akap10 | 11216 | 12-May-15 |
| 2194 | 2 | | | | II-1 | Akap11 | 11215 | 4-May-15 |
| 2199 | 2 | | | | II-1 | Akap2 | 11217 | 7-Jun-15 |
| 2200 | 2 | | | | II-1 | Akap3 | 10566 | 4-May-15 |
| 2211 | 2 | | | | II-1 | Akna | 80709 | 4-May-15 |
| 2212 | 2 | | | | II-1 | Aknad1 | 254268 | 4-May-15 |
| 2215 | 2 | | | | II-1 | Akr1b10 | 57016 | 4-May-15 |
| 2225 | 2 | | | | II-1 | Akr1c21 | | |
| 2226 | 2 | | | | II-1 | Akr1c6 | | |
| 2233 | 2 | | | | II-1 | Akt2 | 208 | 17-May-15 |
| 2234 | 2 | | | | II-1 | Akt3 | 10000 | 17-May-15 |
| 2235 | 2 | | | | II-1 | Aktip | 64400 | 12-May-15 |
| 2259 | 2 | | | | II-1 | Aldh8a1 | 64577 | 23-May-15 |
| 2270 | 2 | | | | II-1 | Alg13 | 79868 | 23-May-15 |
| 2274 | 2 | | | | II-1 | Alg5 | 29880 | 4-May-15 |
| 2279 | 2 | | | | II-1 | Alkbh1 | 8846 | 4-May-15 |
| 2283 | 2 | | | | II-1 | Alkbh5 | 54890 | 4-May-15 |
| 2305 | 2 | | | | II-1 | Als2cl | 259173 | 4-May-15 |
| 2326 | 2 | | | | II-1 | Amh | 268 | 17-May-15 |
| 2353 | 2 | | | | II-1 | Anapc11 | 51529 | 2-Jun-15 |
| 2358 | 2 | | | | II-1 | Anapc4 | 29945 | 4-May-15 |
| 2359 | 2 | | | | II-1 | Anapc5 | 51433 | 4-May-15 |
| 2360 | 2 | | | | II-1 | Anapc7 | 51434 | 4-May-15 |
| 2367 | 2 | | | | II-1 | Angel1 | 23357 | 29-May-15 |
| 2384 | 2 | | | | II-1 | Ankef1 | 63926 | 4-May-15 |
| 2387 | 2 | | | | II-1 | Ankhd1 | 54882 | 3-May-15 |
| 2389 | 2 | | | | II-1 | Ankk1 | 255239 | 24-May-15 |
| 2393 | 2 | | | | II-1 | Ankmy2 | 57037 | 4-May-15 |
| 2397 | 2 | | | | II-1 | Ankrd11 | 29123 | 12-May-15 |
| 2402 | 2 | | | | II-1 | Ankrd13d | 338692 | 4-May-15 |
| 2426 | 2 | | | | II-1 | Ankrd45 | 339416 | 12-May-15 |
| 2428 | 2 | | | | II-1 | Ankrd49 | 54851 | 4-May-15 |
| 2429 | 2 | | | | II-1 | Ankrd50 | 57182 | 4-May-15 |
| 2430 | 2 | | | | II-1 | Ankrd52 | 283373 | 4-May-15 |
| 2433 | 2 | | | | II-1 | Ankrd55 | 79722 | 4-May-15 |
| 2436 | 2 | | | | II-1 | Ankrd61 | 100310846 | 4-May-15 |
| 2444 | 2 | | | | II-1 | Anks4b | 257629 | 4-May-15 |
| 2446 | 2 | | | | II-1 | Ankub1 | 389161 | 4-May-15 |
| 2447 | 2 | | | | II-1 | Ankzf1 | 55139 | 4-May-15 |
| 2453 | 2 | | | | II-1 | Ano4 | 121601 | 4-May-15 |
| 2457 | 2 | | | | II-1 | Ano8 | 57719 | 4-May-15 |
| 2458 | 2 | | | | II-1 | Ano9 | 338440 | 4-May-15 |
| 2460 | 2 | | | | II-1 | Anp32b | 10541 | 4-May-15 |
| 2461 | 2 | | | | II-1 | Anp32e | 81611 | 31-May-15 |
| 2465 | 2 | | | | II-1 | Antxrl | 195977 | 4-May-15 |
| 2484 | 2 | | | | II-1 | Aox3 | | |
| 2485 | 2 | | | | II-1 | Aox4 | | |
| 2488 | 2 | | | | II-1 | Ap1g1 | 164 | 4-May-15 |
| 2489 | 2 | | | | II-1 | Ap1g2 | 8906 | 12-May-15 |

| | | | | | | | | |
|---|---|---|---|---|---|---|---|---|
| 2497 | 2 | | | | II-1 | Ap2b1 | 163 | 4-May-15 |
| 2498 | 2 | | | | II-1 | Ap2m1 | 1173 | 4-May-15 |
| 2499 | 2 | | | | II-1 | Ap2s1 | 1175 | 12-May-15 |
| 2500 | 2 | | | | II-1 | Ap3b1 | 8546 | 23-May-15 |
| 2503 | 2 | | | | II-1 | Ap3m1 | 26985 | 4-May-15 |
| 2504 | 2 | | | | II-1 | Ap3m2 | 10947 | 4-May-15 |
| 2508 | 2 | | | | II-1 | Ap4e1 | 23431 | 12-May-15 |
| 2510 | 2 | | | | II-1 | Ap4s1 | 11154 | 4-May-15 |
| 2519 | 2 | | | | II-1 | Apbb1 | 322 | 12-May-15 |
| 2535 | 2 | | | | II-1 | Apip | 51074 | 4-May-15 |
| 2584 | 2 | | | | II-1 | Appl1 | 26060 | 24-May-15 |
| 2603 | 2 | | | | II-1 | Arap2 | 116984 | 4-May-15 |
| 2610 | 2 | | | | II-1 | Arf2 | 378 | 31-May-15 |
| 2611 | 2 | | | | II-1 | Arf3 | 377 | 4-May-15 |
| 2612 | 2 | | | | II-1 | Arf4 | 378 | 31-May-15 |
| 2613 | 2 | | | | II-1 | Arf5 | 381 | 23-May-15 |
| 2614 | 2 | | | | II-1 | Arf6 | 382 | 23-May-15 |
| 2615 | 2 | | | | II-1 | Arfgap1 | 55738 | 7-Jun-15 |
| 2616 | 2 | | | | II-1 | Arfgap2 | 84364 | 4-May-15 |
| 2617 | 2 | | | | II-1 | Arfgap3 | 26286 | 4-May-15 |
| 2618 | 2 | | | | II-1 | Arfgef1 | 10565 | 4-May-15 |
| 2619 | 2 | | | | II-1 | Arfgef2 | 10564 | 4-May-15 |
| 2621 | 2 | | | | II-1 | Arfip2 | 23647 | 4-May-15 |
| 2622 | 2 | | | | II-1 | Arfrp1 | 10139 | 23-May-15 |
| 2623 | 2 | | | | II-1 | Arg1 | 383 | 7-Jun-15 |
| 2627 | 2 | | | | II-1 | Arhgap10 | 79658 | 7-Jun-15 |
| 2633 | 2 | | | | II-1 | Arhgap18 | 93663 | 4-May-15 |
| 2671 | 2 | | | | II-1 | Arhgef15 | 22899 | 4-May-15 |
| 2680 | 2 | | | | II-1 | Arhgef3 | 50650 | 4-May-15 |
| 2687 | 2 | | | | II-1 | Arhgef5 | 7984 | 4-May-15 |
| 2689 | 2 | | | | II-1 | Arhgef7 | 8874 | 4-May-15 |
| 2691 | 2 | | | | II-1 | Arid1a | 8289 | 24-May-15 |
| 2692 | 2 | | | | II-1 | Arid1b | 57492 | 12-May-15 |
| 2693 | 2 | | | | II-1 | Arid2 | 196528 | 4-May-15 |
| 2697 | 2 | | | | II-1 | Arid4a | 5926 | 4-May-15 |
| 2703 | 2 | | | | II-1 | Arl1 | 400 | 7-Jun-15 |
| 2704 | 2 | | | | II-1 | Arl10 | 285598 | 4-May-15 |
| 2708 | 2 | | | | II-1 | Arl14 | 80117 | 4-May-15 |
| 2711 | 2 | | | | II-1 | Arl15 | 54622 | 4-May-15 |
| 2712 | 2 | | | | II-1 | Arl16 | 339231 | 4-May-15 |
| 2715 | 2 | | | | II-1 | Arl3 | 403 | 4-May-15 |
| 2726 | 2 | | | | II-1 | Arl6ip6 | 151188 | 4-May-15 |
| 2728 | 2 | | | | II-1 | Arl8b | 55207 | 4-May-15 |
| 2729 | 2 | | | | II-1 | Arl9 | 132946 | 4-May-15 |
| 2730 | 2 | | | | II-1 | Armc1 | 55156 | 4-May-15 |
| 2731 | 2 | | | | II-1 | Armc10 | 83787 | 4-May-15 |
| 2734 | 2 | | | | II-1 | Armc3 | 219681 | 4-May-15 |
| 2737 | 2 | | | | II-1 | Armc6 | 93436 | 4-May-15 |
| 2738 | 2 | | | | II-1 | Armc7 | 79637 | 12-May-15 |
| 2745 | 2 | | | | II-1 | Armcx5 | 64860 | 4-May-15 |
| 2752 | 2 | | | | II-1 | Arpc1b | 10095 | 4-May-15 |
| 2754 | 2 | | | | II-1 | Arpc3 | 10094 | 12-May-15 |
| 2755 | 2 | | | | II-1 | Arpc4 | 10093 | 4-May-15 |
| 2756 | 2 | | | | II-1 | Arpc5 | 10092 | 4-May-15 |
| 2757 | 2 | | | | II-1 | Arpc5l | 81873 | 12-May-15 |
| 2762 | 2 | | | | II-1 | Arrb2 | 409 | 17-May-15 |
| 2789 | 2 | | | | II-1 | Asap1 | 50807 | 12-May-15 |
| 2809 | 2 | | | | II-1 | Asb8 | 140461 | 4-May-15 |
| 2810 | 2 | | | | II-1 | Asb9 | 140462 | 3-May-15 |
| 2825 | 2 | | | | II-1 | Asic1 | 41 | 4-May-15 |
| 2829 | 2 | | | | II-1 | Asic5 | 51802 | 4-May-15 |
| 2849 | 2 | | | | II-1 | Astn1 | 460 | 4-May-15 |
| 2850 | 2 | | | | II-1 | Astn2 | 23245 | 4-May-15 |
| 2852 | 2 | | | | II-1 | Asxl1 | 171023 | 21-May-15 |
| 2853 | 2 | | | | II-1 | Asxl2 | 55252 | 4-May-15 |
| 2867 | 2 | | | | II-1 | Atf2 | 1386 | 7-Jun-15 |
| 2881 | 2 | | | | II-1 | Atg16l1 | 55054 | 21-May-15 |
| 2882 | 2 | | | | II-1 | Atg16l2 | 89849 | 21-May-15 |
| 2885 | 2 | | | | II-1 | Atg3 | 64422 | 23-May-15 |
| 2890 | 2 | | | | II-1 | Atg5 | 9474 | 21-May-15 |
| 2891 | 2 | | | | II-1 | Atg7 | 10533 | 4-May-15 |
| 2892 | 2 | | | | II-1 | Atg9a | 79065 | 21-May-15 |
| 2915 | 2 | | | | II-1 | Atp13a3 | 79572 | 4-May-15 |
| 2948 | 2 | | | | II-1 | Atp5j2 | 9551 | 4-May-15 |
| 2954 | 2 | | | | II-1 | Atp6ap1 | 537 | 4-May-15 |
| 2958 | 2 | | | | II-1 | Atp6v0a2 | 23545 | 23-May-15 |
| 2959 | 2 | | | | II-1 | Atp6v0a4 | 50617 | 24-May-15 |
| 2961 | 2 | | | | II-1 | Atp6v0c | 527 | 4-May-15 |
| 2976 | 2 | | | | II-1 | Atp6v1g1 | 9550 | 4-May-15 |
| 2985 | 2 | | | | II-1 | Atp8b2 | 57198 | 21-May-15 |
| 2988 | 2 | | | | II-1 | Atp8b5 | | |
| 2992 | 2 | | | | II-1 | Atpaf2 | 91647 | 4-May-15 |
| 2993 | 2 | | | | II-1 | Atpif1 | 93974 | 24-May-15 |
| 2997 | 2 | | | | II-1 | Atrn | 8455 | 12-May-15 |
| 2998 | 2 | | | | II-1 | Atrnl1 | 26033 | 4-May-15 |
| 3002 | 2 | | | | II-1 | Atxn1l | 342371 | 4-May-15 |
| 3003 | 2 | | | | II-1 | Atxn2 | 6311 | 23-May-15 |
| 3006 | 2 | | | | II-1 | Atxn7 | 6314 | 23-May-15 |
| 3007 | 2 | | | | II-1 | Atxn7l1 | 222255 | 12-May-15 |
| 3008 | 2 | | | | II-1 | Atxn7l2 | 127002 | 4-May-15 |
| 3014 | 2 | | | | II-1 | AU016765 | | |
| 3015 | 2 | | | | II-1 | AU018091 | | |
| 3018 | 2 | | | | II-1 | AU019990 | | |
| 3020 | 2 | | | | II-1 | AU021092 | | |
| 3023 | 2 | | | | II-1 | AU022754 | | |
| 3024 | 2 | | | | II-1 | AU022793 | | |
| 3025 | 2 | | | | II-1 | AU023762 | | |

Fig.22 - 96

| | | | | | | | | |
|---|---|---|---|---|---|---|---|---|
| 3029 | 2 | | | | II-1 | Auh | 549 | 21-May-15 |
| 3052 | 2 | | | | II-1 | AW495222 | | |
| 3057 | 2 | | | | II-1 | AW822252 | | |
| 3067 | 2 | | | | II-1 | AY761184 | | |
| 3073 | 2 | | | | II-1 | B020004C17Rik | | |
| 3075 | 2 | | | | II-1 | B020014A21Rik | | |
| 3082 | 2 | | | | II-1 | B230118H07Rik | | |
| 3083 | 2 | | | | II-1 | B230119M05Rik | | |
| 3087 | 2 | | | | II-1 | B230214G05Rik | | |
| 3100 | 2 | | | | II-1 | B3galt1 | 8708 | 21-May-15 |
| 3109 | 2 | | | | II-1 | B3gnt1 | 10678 | 7-Jun-15 |
| 3135 | 2 | | | | II-1 | B630019K06Rik | | |
| 3140 | 2 | | | | II-1 | B930041F14Rik | | |
| 3145 | 2 | | | | II-1 | Baalc | 79870 | 12-May-15 |
| 3148 | 2 | | | | II-1 | Bace1 | 23621 | 17-May-15 |
| 3158 | 2 | | | | II-1 | Bag5 | 9529 | 28-May-15 |
| 3159 | 2 | | | | II-1 | Bag6 | 7917 | 29-May-15 |
| 3164 | 2 | | | | II-1 | Bai3 | 577 | 4-May-15 |
| 3169 | 2 | | | | II-1 | Bak1 | 578 | 17-May-15 |
| 3177 | 2 | | | | II-1 | Bard1 | 580 | 24-May-15 |
| 3180 | 2 | | | | II-1 | Barx1 | 56033 | 4-May-15 |
| 3190 | 2 | | | | II-1 | Baz2b | 29994 | 12-May-15 |
| 3193 | 2 | | | | II-1 | BB031773 | | |
| 3196 | 2 | | | | II-1 | BB287469 | | |
| 3198 | 2 | | | | II-1 | Bbc3 | 27113 | 24-May-15 |
| 3206 | 2 | | | | II-1 | Bbs5 | 129880 | 23-May-15 |
| 3209 | 2 | | | | II-1 | Bbx | 56987 | 4-May-15 |
| 3211 | 2 | | | | II-1 | BC003331 | | |
| 3213 | 2 | | | | II-1 | BC004004 | | |
| 3214 | 2 | | | | II-1 | BC005537 | | |
| 3220 | 2 | | | | II-1 | BC017158 | | |
| 3221 | 2 | | | | II-1 | BC017643 | | |
| 3224 | 2 | | | | II-1 | BC018507 | | |
| 3234 | 2 | | | | II-1 | BC024978 | | |
| 3244 | 2 | | | | II-1 | BC030499 | | |
| 3245 | 2 | | | | II-1 | BC030500 | | |
| 3246 | 2 | | | | II-1 | BC030867 | | |
| 3249 | 2 | | | | II-1 | BC031361 | | |
| 3252 | 2 | | | | II-1 | BC037032 | | |
| 3259 | 2 | | | | II-1 | BC048507 | | |
| 3264 | 2 | | | | II-1 | BC048644 | | |
| 3265 | 2 | | | | II-1 | BC048671 | | |
| 3268 | 2 | | | | II-1 | BC049635 | | |
| 3269 | 2 | | | | II-1 | BC049715 | | |
| 3277 | 2 | | | | II-1 | BC051665 | | |
| 3278 | 2 | | | | II-1 | BC052040 | | |
| 3281 | 2 | | | | II-1 | BC053749 | | |
| 3284 | 2 | | | | II-1 | BC055402 | | |
| 3287 | 2 | | | | II-1 | BC061212 | | |
| 3288 | 2 | | | | II-1 | BC061237 | | |
| 3292 | 2 | | | | II-1 | BC068281 | | |
| 3312 | 2 | | | | II-1 | Bcas3 | 54828 | 12-May-15 |
| 3313 | 2 | | | | II-1 | Bcas3os1 | | |
| 3314 | 2 | | | | II-1 | Bcas3os2 | | |
| 3318 | 2 | | | | II-1 | Bcdin3d | 144233 | 4-May-15 |
| 3324 | 2 | | | | II-1 | Bcl11a | 53335 | 4-May-15 |
| 3363 | 2 | | | | II-1 | Begain | 57596 | 4-May-15 |
| 3369 | 2 | | | | II-1 | Best1 | 7439 | 23-May-15 |
| 3417 | 2 | | | | II-1 | Bloc1s5 | 63915 | 4-May-15 |
| 3418 | 2 | | | | II-1 | Bloc1s6 | 26258 | 23-May-15 |
| 3419 | 2 | | | | II-1 | Blvra | 644 | 4-May-15 |
| 3427 | 2 | | | | II-1 | Bmp2 | 650 | 24-May-15 |
| 3440 | 2 | | | | II-1 | Bms1 | 9790 | 4-May-15 |
| 3441 | 2 | | | | II-1 | Bmx | 660 | 4-May-15 |
| 3452 | 2 | | | | II-1 | Bod1l | 259282 | 4-May-15 |
| 3466 | 2 | | | | II-1 | Bpifa5 | | |
| 3467 | 2 | | | | II-1 | Bpifa6 | | |
| 3471 | 2 | | | | II-1 | Bpifb4 | 149954 | 4-May-15 |
| 3479 | 2 | | | | II-1 | Braf | 673 | 7-Jun-15 |
| 3481 | 2 | | | | II-1 | Brat1 | 221927 | 31-May-15 |
| 3482 | 2 | | | | II-1 | Brca1 | 672 | 25-May-15 |
| 3487 | 2 | | | | II-1 | Brd3 | 8019 | 4-May-15 |
| 3488 | 2 | | | | II-1 | Brd4 | 23476 | 24-May-15 |
| 3489 | 2 | | | | II-1 | Brd7 | 29117 | 4-May-15 |
| 3490 | 2 | | | | II-1 | Brd8 | 10902 | 12-May-15 |
| 3491 | 2 | | | | II-1 | Brd9 | 65980 | 4-May-15 |
| 3493 | 2 | | | | II-1 | Bre | 9577 | 12-May-15 |
| 3496 | 2 | | | | II-1 | Bri3 | 25798 | 7-Jun-15 |
| 3499 | 2 | | | | II-1 | Brinp1 | 1620 | 4-May-15 |
| 3500 | 2 | | | | II-1 | Brinp2 | 57795 | 4-May-15 |
| 3502 | 2 | | | | II-1 | Brip1 | 83990 | 7-Jun-15 |
| 3505 | 2 | | | | II-1 | Brms1 | 25855 | 7-Jun-15 |
| 3508 | 2 | | | | II-1 | Brpf1 | 7862 | 7-Jun-15 |
| 3509 | 2 | | | | II-1 | Brpf3 | 27154 | 4-May-15 |
| 3511 | 2 | | | | II-1 | Brsk1 | 84446 | 4-May-15 |
| 3514 | 2 | | | | II-1 | Brwd3 | 254065 | 4-May-15 |
| 3517 | 2 | | | | II-1 | Bsg | 682 | 17-May-15 |
| 3528 | 2 | | | | II-1 | Btbd10 | 84280 | 12-May-15 |
| 3529 | 2 | | | | II-1 | Btbd11 | 121551 | 4-May-15 |
| 3533 | 2 | | | | II-1 | Btbd19 | 149478 | 4-May-15 |
| 3540 | 2 | | | | II-1 | Btc | 685 | 4-May-15 |
| 3544 | 2 | | | | II-1 | Btg1 | 694 | 7-Jun-15 |
| 3552 | 2 | | | | II-1 | Btnl1 | | |
| 3558 | 2 | | | | II-1 | Btnl9 | 153579 | 12-May-15 |
| 3564 | 2 | | | | II-1 | Bud31 | 8896 | 4-May-15 |
| 3575 | 2 | | | | II-1 | C030023E24Rik | | |
| 3577 | 2 | | | | II-1 | C030034I22Rik | | |
| 3582 | 2 | | | | II-1 | C130021I20Rik | | |
| 3585 | 2 | | | | II-1 | C130030K03Rik | | |
| 3591 | 2 | | | | II-1 | C130071C03Rik | | |
| 3595 | 2 | | | | II-1 | C130083M11Rik | | |
| 3596 | 2 | | | | II-1 | C1d | 10438 | 4-May-15 |
| 3607 | 2 | | | | II-1 | C1qtnf1 | 114897 | 4-May-15 |
| 3608 | 2 | | | | II-1 | C1qtnf2 | 114898 | 12-May-15 |
| 3623 | 2 | | | | II-1 | C230029M16 | | |
| 3629 | 2 | | | | II-1 | C2cd2 | 25966 | 4-May-15 |
| 3632 | 2 | | | | II-1 | C2cd4a | 145741 | 4-May-15 |
| 3640 | 2 | | | | II-1 | C330011F03Rik | | |
| 3641 | 2 | | | | II-1 | C330013E15Rik | | |
| 3647 | 2 | | | | II-1 | C330024D21Rik | | |
| 3648 | 2 | | | | II-1 | C330027C09Rik | | |
| 3653 | 2 | | | | II-1 | C430049B03Rik | | |
| 3666 | 2 | | | | II-1 | C630043F03Rik | | |
| 3670 | 2 | | | | II-1 | C730036E19Rik | | |
| 3676 | 2 | | | | II-1 | C87198 | | |
| 3681 | 2 | | | | II-1 | C8a | 731 | 12-May-15 |
| 3692 | 2 | | | | II-1 | Cabin1 | 23523 | 21-May-15 |
| 3703 | 2 | | | | II-1 | Cachd1 | 57685 | 12-May-15 |
| 3721 | 2 | | | | II-1 | Cacnb4 | 785 | 23-May-15 |
| 3725 | 2 | | | | II-1 | Cacng4 | 27092 | 4-May-15 |
| 3732 | 2 | | | | II-1 | Cacybp | 27101 | 26-May-15 |
| 3752 | 2 | | | | II-1 | Calm1 | 801 | 17-May-15 |
| 3754 | 2 | | | | II-1 | Calm3 | 808 | 12-May-15 |
| 3763 | 2 | | | | II-1 | Calu | 813 | 2-Jun-15 |
| 3764 | 2 | | | | II-1 | Caly | 50632 | 20-May-15 |
| 3787 | 2 | | | | II-1 | Cand2 | 23066 | 4-May-15 |
| 3790 | 2 | | | | II-1 | Cap1 | 10487 | 7-Jun-15 |
| 3796 | 2 | | | | II-1 | Capn12 | 147968 | 4-May-15 |
| 3803 | 2 | | | | II-1 | Capn7 | 23473 | 4-May-15 |
| 3809 | 2 | | | | II-1 | Caprin2 | 65981 | 12-May-15 |
| 3811 | 2 | | | | II-1 | Capsl | 133690 | 4-May-15 |
| 3812 | 2 | | | | II-1 | Capza1 | 829 | 4-May-15 |
| 3815 | 2 | | | | II-1 | Capzb | 832 | 4-May-15 |
| 3837 | 2 | | | | II-1 | Carf | 79800 | 7-Jun-15 |
| 3838 | 2 | | | | II-1 | Carhsp1 | 23589 | |
| 3841 | 2 | | | | II-1 | Carns1 | 57571 | 4-May-15 |
| 3842 | 2 | | | | II-1 | Cars | 833 | 4-May-15 |
| 3843 | 2 | | | | II-1 | Cars2 | 79587 | 4-May-15 |
| 3846 | 2 | | | | II-1 | Casc3 | 22794 | 4-May-15 |
| 3847 | 2 | | | | II-1 | Casc4 | 113201 | 4-May-15 |
| 3851 | 2 | | | | II-1 | Caskin1 | 57524 | 4-May-15 |
| 3852 | 2 | | | | II-1 | Caskin2 | 57513 | 4-May-15 |
| 3868 | 2 | | | | II-1 | Cast | 831 | 7-Jun-15 |
| 3879 | 2 | | | | II-1 | Catsperg2 | | |
| 3880 | 2 | | | | II-1 | Cav1 | 857 | 31-May-15 |
| 3885 | 2 | | | | II-1 | Cbfb | 865 | 7-Jun-15 |
| 3902 | 2 | | | | II-1 | Cbx3 | 11335 | 12-May-15 |
| 3908 | 2 | | | | II-1 | Cby1 | 25776 | 4-May-15 |
| 3911 | 2 | | | | II-1 | Cc2d2a | 57545 | 12-May-15 |
| 3912 | 2 | | | | II-1 | Ccar1 | 55749 | 29-May-15 |
| 3920 | 2 | | | | II-1 | Ccdc104 | 112942 | 4-May-15 |
| 3922 | 2 | | | | II-1 | Ccdc106 | 29903 | 4-May-15 |
| 3925 | 2 | | | | II-1 | Ccdc109b | 55013 | 4-May-15 |
| 3928 | 2 | | | | II-1 | Ccdc112 | 153733 | 12-May-15 |
| 3931 | 2 | | | | II-1 | Ccdc115 | 84317 | 4-May-15 |
| 3934 | 2 | | | | II-1 | Ccdc12 | 151903 | 4-May-15 |
| 3942 | 2 | | | | II-1 | Ccdc129 | 223075 | 4-May-15 |
| 3943 | 2 | | | | II-1 | Ccdc13 | 152206 | 29-May-15 |
| 3944 | 2 | | | | II-1 | Ccdc130 | 81576 | 14-May-15 |
| 3945 | 2 | | | | II-1 | Ccdc132 | 55610 | 29-May-15 |
| 3946 | 2 | | | | II-1 | Ccdc134 | 79879 | 12-May-15 |
| 3955 | 2 | | | | II-1 | Ccdc146 | 57639 | 4-May-15 |
| 3956 | 2 | | | | II-1 | Ccdc147 | 159686 | 4-May-15 |
| 3959 | 2 | | | | II-1 | Ccdc15 | 80071 | 4-May-15 |
| 3962 | 2 | | | | II-1 | Ccdc152 | 100129792 | 4-May-15 |
| 3966 | 2 | | | | II-1 | Ccdc157 | 550631 | 4-May-15 |
| 3978 | 2 | | | | II-1 | Ccdc172 | 374355 | 4-May-15 |
| 3979 | 2 | | | | II-1 | Ccdc173 | 129881 | 4-May-15 |
| 3982 | 2 | | | | II-1 | Ccdc176 | 80127 | 4-May-15 |
| 3985 | 2 | | | | II-1 | Ccdc18 | 343099 | |
| 3988 | 2 | | | | II-1 | Ccdc184 | 387856 | 4-May-15 |
| 3995 | 2 | | | | II-1 | Ccdc27 | 148870 | 4-May-15 |
| 3996 | 2 | | | | II-1 | Ccdc28a | 25901 | 4-May-15 |
| 4001 | 2 | | | | II-1 | Ccdc33 | 80125 | 12-May-15 |
| 4004 | 2 | | | | II-1 | Ccdc36 | 339834 | 4-May-15 |
| 4005 | 2 | | | | II-1 | Ccdc37 | 348807 | 4-May-15 |
| 4007 | 2 | | | | II-1 | Ccdc39 | 339829 | 23-May-15 |
| 4008 | 2 | | | | II-1 | Ccdc40 | 55036 | 23-May-15 |
| 4009 | 2 | | | | II-1 | Ccdc42 | 146849 | 4-May-15 |
| 4010 | 2 | | | | II-1 | Ccdc42b | 387885 | 4-May-15 |
| 4015 | 2 | | | | II-1 | Ccdc53 | 51019 | 4-May-15 |
| 4022 | 2 | | | | II-1 | Ccdc60 | 160777 | 4-May-15 |
| 4029 | 2 | | | | II-1 | Ccdc66 | 285331 | |
| 4035 | 2 | | | | II-1 | Ccdc71 | 64925 | 12-May-15 |
| 4036 | 2 | | | | II-1 | Ccdc71l | 168455 | 4-May-15 |
| 4041 | 2 | | | | II-1 | Ccdc79 | 283847 | 4-May-15 |
| 4047 | 2 | | | | II-1 | Ccdc84 | 338657 | 4-May-15 |
| 4053 | 2 | | | | II-1 | Ccdc88a | 55704 | 31-May-15 |
| 4054 | 2 | | | | II-1 | Ccdc88b | 283234 | |
| 4059 | 2 | | | | II-1 | Ccdc91 | 55297 | 4-May-15 |
| 4065 | 2 | | | | II-1 | Ccer1 | 196477 | 4-May-15 |
| 4066 | 2 | | | | II-1 | Cchcr1 | 54535 | 12-May-15 |
| 4067 | 2 | | | | II-1 | Ccin | 881 | 4-May-15 |
| 4114 | 2 | | | | II-1 | Ccni | 10983 | 4-May-15 |

Fig.22 - 97

| | | | | | | | | |
|---|---|---|---|---|---|---|---|---|
| 4116 | 2 | | | | II-1 | Ccnjl | 79616 | |
| 4119 | 2 | | | | II-1 | Ccnl2 | 81669 | 4-May-15 |
| 4124 | 2 | | | | II-1 | Ccnyl1 | 151195 | 4-May-15 |
| 4125 | 2 | | | | II-1 | Ccp110 | 9738 | 31-May-15 |
| 4126 | 2 | | | | II-1 | Ccpg1 | 9236 | 4-May-15 |
| 4134 | 2 | | | | II-1 | Ccr5 | 1234 | |
| 4145 | 2 | | | | II-1 | Cct2 | 10576 | 17-May-15 |
| 4147 | 2 | | | | II-1 | Cct4 | 10575 | 4-May-15 |
| 4148 | 2 | | | | II-1 | Cct5 | 22948 | 2-Jun-15 |
| 4149 | 2 | | | | II-1 | Cct6a | 908 | 4-May-15 |
| 4150 | 2 | | | | II-1 | Cct6b | 10693 | 4-May-15 |
| 4151 | 2 | | | | II-1 | Cct7 | 10574 | 4-May-15 |
| 4152 | 2 | | | | II-1 | Cct8 | 10694 | 12-May-15 |
| 4153 | 2 | | | | II-1 | Cct8l1 | 155100 | 4-May-15 |
| 4154 | 2 | | | | II-1 | Ccz1 | 51622 | 4-May-15 |
| 4174 | 2 | | | | II-1 | Cd200r4 | | |
| 4240 | 2 | | | | II-1 | Cd82 | 3732 | |
| 4254 | 2 | | | | II-1 | Cdc123 | 8872 | 4-May-15 |
| 4255 | 2 | | | | II-1 | Cdc14a | 8556 | 4-May-15 |
| 4266 | 2 | | | | II-1 | Cdc34 | 997 | 4-May-15 |
| 4268 | 2 | | | | II-1 | Cdc37l1 | 55664 | 4-May-15 |
| 4269 | 2 | | | | II-1 | Cdc40 | 51362 | 4-May-15 |
| 4270 | 2 | | | | II-1 | Cdc42 | 998 | 23-May-15 |
| 4271 | 2 | | | | II-1 | Cdc42bpa | 8476 | 4-May-15 |
| 4281 | 2 | | | | II-1 | Cdc45 | 8318 | 4-May-15 |
| 4310 | 2 | | | | II-1 | Cdh26 | 60437 | 4-May-15 |
| 4313 | 2 | | | | II-1 | Cdh5 | 1003 | 31-May-15 |
| 4318 | 2 | | | | II-1 | Cdhr1 | 92211 | 4-May-15 |
| 4320 | 2 | | | | II-1 | Cdhr3 | 222256 | |
| 4328 | 2 | | | | II-1 | Cdk13 | 8621 | 4-May-15 |
| 4332 | 2 | | | | II-1 | Cdk17 | 5128 | 4-May-15 |
| 4335 | 2 | | | | II-1 | Cdk2 | 1017 | 12-May-15 |
| 4338 | 2 | | | | II-1 | Cdk2ap2 | 10263 | 4-May-15 |
| 4339 | 2 | | | | II-1 | Cdk3-ps | | |
| 4342 | 2 | | | | II-1 | Cdk5r1 | 8851 | 4-May-15 |
| 4345 | 2 | | | | II-1 | Cdk5rap2 | 55755 | 23-May-15 |
| 4351 | 2 | | | | II-1 | Cdkal1 | 54901 | 31-May-15 |
| 4356 | 2 | | | | II-1 | Cdkl5 | 6792 | 23-May-15 |
| 4362 | 2 | | | | II-1 | Cdkn2aipnl | 91368 | 4-May-15 |
| 4381 | 2 | | | | II-1 | Cdx2 | 1045 | 31-May-15 |
| 4382 | 2 | | | | II-1 | Cdx4 | 1046 | 28-May-15 |
| 4383 | 2 | | | | II-1 | Cdyl | 9425 | 4-May-15 |
| 4385 | 2 | | | | II-1 | Ceacam1 | 634 | 12-May-15 |
| 4391 | 2 | | | | II-1 | Ceacam15 | | |
| 4394 | 2 | | | | II-1 | Ceacam19 | 56971 | 4-May-15 |
| 4395 | 2 | | | | II-1 | Ceacam2 | | |
| 4398 | 2 | | | | II-1 | Ceacam5 | 1048 | 21-May-15 |
| 4399 | 2 | | | | II-1 | Ceacam9 | | |
| 4407 | 2 | | | | II-1 | Cebpzos | 100505876 | 12-May-15 |
| 4422 | 2 | | | | II-1 | Celsr1 | 9620 | 12-May-15 |
| 4430 | 2 | | | | II-1 | Cenpe | 1062 | 17-May-15 |
| 4450 | 2 | | | | II-1 | Cep131 | 22994 | 4-May-15 |
| 4458 | 2 | | | | II-1 | Cep192 | 55125 | 4-May-15 |
| 4469 | 2 | | | | II-1 | Cep70 | 80321 | 4-May-15 |
| 4482 | 2 | | | | II-1 | Cercam | 51148 | |
| 4511 | 2 | | | | II-1 | Cetn2 | 1069 | 31-May-15 |
| 4520 | 2 | | | | II-1 | Cfhr2 | 3080 | 4-May-15 |
| 4528 | 2 | | | | II-1 | Cggbp1 | 8545 | 4-May-15 |
| 4542 | 2 | | | | II-1 | Chchd1 | 118487 | 4-May-15 |
| 4545 | 2 | | | | II-1 | Chchd3 | 54927 | 4-May-15 |
| 4557 | 2 | | | | II-1 | Chd6 | 84181 | 4-May-15 |
| 4560 | 2 | | | | II-1 | Chd9 | 80205 | 12-May-15 |
| 4571 | 2 | | | | II-1 | Chid1 | 66005 | 4-May-15 |
| 4580 | 2 | | | | II-1 | Chl1 | 10752 | /6/7 |
| 4585 | 2 | | | | II-1 | Chmp2a | 27243 | 4-May-15 |
| 4586 | 2 | | | | II-1 | Chmp2b | 25978 | 23-May-15 |
| 4587 | 2 | | | | II-1 | Chmp3 | 51652 | 4-May-15 |
| 4588 | 2 | | | | II-1 | Chmp4b | 128866 | 7-Jun-15 |
| 4589 | 2 | | | | II-1 | Chmp4c | 92421 | 4-May-15 |
| 4592 | 2 | | | | II-1 | Chmp7 | 91782 | 4-May-15 |
| 4593 | 2 | | | | II-1 | Chn1 | 1123 | 23-May-15 |
| 4594 | 2 | | | | II-1 | Chn1os3 | | |
| 4596 | 2 | | | | II-1 | Chodl | 140578 | 12-May-15 |
| 4612 | 2 | | | | II-1 | Chrna1 | 1134 | 23-May-15 |
| 4624 | 2 | | | | II-1 | Chrnb4 | 1143 | 12-May-15 |
| 4626 | 2 | | | | II-1 | Chrne | 1145 | 23-May-15 |
| 4627 | 2 | | | | II-1 | Chrng | 1146 | 4-May-15 |
| 4633 | 2 | | | | II-1 | Chst14 | 113189 | 4-May-15 |
| 4643 | 2 | | | | II-1 | Chsy3 | 337876 | #NAME? |
| 4645 | 2 | | | | II-1 | Chtf8 | 54921 | 12-May-15 |
| 4647 | 2 | | | | II-1 | Chuk | 1147 | 4-May-15 |
| 4648 | 2 | | | | II-1 | Churc1 | 91612 | 4-May-15 |
| 4650 | 2 | | | | II-1 | Ciapin1 | 57019 | 12-May-15 |
| 4666 | 2 | | | | II-1 | Cirbp | 1153 | 17-May-15 |
| 4670 | 2 | | | | II-1 | Cisd3 | 284106 | 4-May-15 |
| 4693 | 2 | | | | II-1 | Clasrp | 11129 | 12-May-15 |
| 4737 | 2 | | | | II-1 | Clec10a | 10462 | 4-May-15 |
| 4754 | 2 | | | | II-1 | Clec3b | 7123 | |
| 4763 | 2 | | | | II-1 | Clec4f | 165530 | 4-May-15 |
| 4779 | 2 | | | | II-1 | Clip3 | 25999 | |
| 4783 | 2 | | | | II-1 | Clk3 | 1198 | 4-May-15 |
| 4784 | 2 | | | | II-1 | Clk4 | 57396 | 12-May-15 |
| 4785 | 2 | | | | II-1 | Clmn | 79789 | 4-May-15 |
| 4791 | 2 | | | | II-1 | Clnk | 116449 | 4-May-15 |
| 4792 | 2 | | | | II-1 | Clns1a | 1207 | 12-May-15 |
| 4793 | 2 | | | | II-1 | Clock | 9575 | 12-May-15 |
| 4796 | 2 | | | | II-1 | Clpp | 8192 | 4-May-15 |
| 4800 | 2 | | | | II-1 | Clptm1l | 81037 | 17-May-15 |
| 4801 | 2 | | | | II-1 | Clpx | 10845 | |
| 4802 | 2 | | | | II-1 | Clrn1 | 7401 | 23-May-15 |
| 4803 | 2 | | | | II-1 | Clrn2 | 645104 | 4-May-15 |
| 4804 | 2 | | | | II-1 | Clrn3 | 119467 | 4-May-15 |
| 4805 | 2 | | | | II-1 | Clspn | 63967 | |
| 4811 | 2 | | | | II-1 | Cltc | 1213 | 31-May-15 |
| 4815 | 2 | | | | II-1 | Clvs1 | 157807 | 28-May-15 |
| 4816 | 2 | | | | II-1 | Clvs2 | 134829 | 4-May-15 |
| 4838 | 2 | | | | II-1 | Cmtm4 | 146223 | 4-May-15 |
| 4840 | 2 | | | | II-1 | Cmtm6 | 54918 | 4-May-15 |
| 4853 | 2 | | | | II-1 | Cnga2 | 1260 | 6-May-15 |
| 4868 | 2 | | | | II-1 | Cnnm1 | 26507 | 4-May-15 |
| 4869 | 2 | | | | II-1 | Cnnm2 | 54805 | |
| 4872 | 2 | | | | II-1 | Cnot1 | 23019 | 21-May-15 |
| 4876 | 2 | | | | II-1 | Cnot3 | 4849 | 4-May-15 |
| 4878 | 2 | | | | II-1 | Cnot6 | 57472 | 4-May-15 |
| 4882 | 2 | | | | II-1 | Cnp | 1267 | 7-Jun-15 |
| 4885 | 2 | | | | II-1 | Cnpy2 | 10330 | 4-May-15 |
| 4886 | 2 | | | | II-1 | Cnpy3 | 10695 | 4-May-15 |
| 4898 | 2 | | | | II-1 | Cntn3 | 5067 | 4-May-15 |
| 4899 | 2 | | | | II-1 | Cntn4 | 152330 | 4-May-15 |
| 4900 | 2 | | | | II-1 | Cntn5 | 53942 | |
| 4902 | 2 | | | | II-1 | Cntnap1 | 8506 | 12-May-15 |
| 4905 | 2 | | | | II-1 | Cntnap4 | 85445 | 4-May-15 |
| 4906 | 2 | | | | II-1 | Cntnap5a | | |
| 4915 | 2 | | | | II-1 | Coa7 | 65260 | 4-May-15 |
| 4917 | 2 | | | | II-1 | Cobl | 23242 | 4-May-15 |
| 4923 | 2 | | | | II-1 | Cog4 | 25839 | 23-May-15 |
| 4924 | 2 | | | | II-1 | Cog5 | 10466 | 23-May-15 |
| 4925 | 2 | | | | II-1 | Cog6 | 57511 | 4-May-15 |
| 4927 | 2 | | | | II-1 | Cog8 | 84342 | 23-May-15 |
| 4928 | 2 | | | | II-1 | Coil | 8161 | 4-May-15 |
| 4975 | 2 | | | | II-1 | Colec11 | 78989 | 17-May-15 |
| 4982 | 2 | | | | II-1 | Commd3 | 23412 | 4-May-15 |
| 4983 | 2 | | | | II-1 | Commd4 | 54939 | 4-May-15 |
| 4989 | 2 | | | | II-1 | Comp | 1311 | 23-May-15 |
| 4994 | 2 | | | | II-1 | Copb2 | 9276 | 12-May-15 |
| 4995 | 2 | | | | II-1 | Cope | 11316 | 4-May-15 |
| 4996 | 2 | | | | II-1 | Copg1 | 22820 | 4-May-15 |
| 4997 | 2 | | | | II-1 | Copg2 | 26958 | 12-May-15 |
| 5003 | 2 | | | | II-1 | Cops6 | 10980 | 4-May-15 |
| 5004 | 2 | | | | II-1 | Cops7a | 50813 | 4-May-15 |
| 5005 | 2 | | | | II-1 | Cops7b | 64708 | 21-May-15 |
| 5006 | 2 | | | | II-1 | Cops8 | 10920 | 4-May-15 |
| 5007 | 2 | | | | II-1 | Copz1 | 22818 | 12-May-15 |
| 5008 | 2 | | | | II-1 | Copz2 | 51226 | 4-May-15 |
| 5014 | 2 | | | | II-1 | Coq5 | 84274 | 21-May-15 |
| 5030 | 2 | | | | II-1 | Cox14 | 84987 | 12-May-15 |
| 5051 | 2 | | | | II-1 | Cox7c | 1350 | 12-May-15 |
| 5095 | 2 | | | | II-1 | Cpsf3 | 51692 | 4-May-15 |
| 5097 | 2 | | | | II-1 | Cpsf4 | 10898 | 4-May-15 |
| 5098 | 2 | | | | II-1 | Cpsf4l | 642843 | 4-May-15 |
| 5100 | 2 | | | | II-1 | Cpsf7 | 79869 | 4-May-15 |
| 5107 | 2 | | | | II-1 | Cpxm1 | 56265 | |
| 5134 | 2 | | | | II-1 | Creg1 | 8804 | 4-May-15 |
| 5140 | 2 | | | | II-1 | Crhbp | 1393 | 4-May-15 |
| 5141 | 2 | | | | II-1 | Crhr1 | 1394 | 12-May-15 |
| 5173 | 2 | | | | II-1 | Crx | 1406 | 28-May-15 |
| 5174 | 2 | | | | II-1 | Crxos | | |
| 5191 | 2 | | | | II-1 | Crygf | | |
| 5198 | 2 | | | | II-1 | Cs | 1431 | 4-May-15 |
| 5203 | 2 | | | | II-1 | Csf1 | 1435 | 12-May-15 |
| 5214 | 2 | | | | II-1 | Csl | | |
| 5216 | 2 | | | | II-1 | Csmd2 | 114784 | 4-May-15 |
| 5217 | 2 | | | | II-1 | Csmd2os | | |
| 5218 | 2 | | | | II-1 | Csmd3 | 114788 | 4-May-15 |
| 5219 | 2 | | | | II-1 | Csn1s1 | 1446 | 12-May-15 |
| 5226 | 2 | | | | II-1 | Csnk1e | 1454 | 4-May-15 |
| 5227 | 2 | | | | II-1 | Csnk1g1 | 53944 | 21-May-15 |
| 5228 | 2 | | | | II-1 | Csnk1g2 | 1455 | 4-May-15 |
| 5229 | 2 | | | | II-1 | Csnk1g3 | 1456 | 4-May-15 |
| 5230 | 2 | | | | II-1 | Csnk2a1 | 1457 | 7-Jun-15 |
| 5231 | 2 | | | | II-1 | Csnk2a2 | 1459 | 2-Jun-15 |
| 5233 | 2 | | | | II-1 | Csnka2ip | | |
| 5234 | 2 | | | | II-1 | Cspg4 | 1464 | 4-May-15 |
| 5235 | 2 | | | | II-1 | Cspg5 | 10675 | 21-May-15 |
| 5241 | 2 | | | | II-1 | Csrp1 | 1465 | 12-May-15 |
| 5242 | 2 | | | | II-1 | Csrp2 | 1466 | |
| 5249 | 2 | | | | II-1 | Cst3 | 1471 | 24-May-15 |
| 5254 | 2 | | | | II-1 | Csta1 | | |
| 5255 | 2 | | | | II-1 | Cstad | | |
| 5259 | 2 | | | | II-1 | Cstf2t | 23283 | 4-May-15 |
| 5262 | 2 | | | | II-1 | Ctag2 | 30848 | 4-May-15 |
| 5263 | 2 | | | | II-1 | Ctage5 | 4253 | 4-May-15 |
| 5264 | 2 | | | | II-1 | Ctbp1 | 1487 | 4-May-15 |
| 5265 | 2 | | | | II-1 | Ctbp2 | 1488 | 12-May-15 |
| 5268 | 2 | | | | II-1 | Ctcf | 10664 | 12-May-15 |
| 5273 | 2 | | | | II-1 | Ctdsp1 | 58190 | 4-May-15 |
| 5274 | 2 | | | | II-1 | Ctdsp2 | 10106 | 12-May-15 |
| 5275 | 2 | | | | II-1 | Ctdspl | 10217 | 4-May-15 |
| 5276 | 2 | | | | II-1 | Ctdspl2 | 51496 | 12-May-15 |
| 5287 | 2 | | | | II-1 | Ctnna2 | 1496 | 12-May-15 |
| 5288 | 2 | | | | II-1 | Ctnna3 | 29119 | 12-May-15 |
| 5289 | 2 | | | | II-1 | Ctnnal1 | 8727 | 12-May-15 |
| 5293 | 2 | | | | II-1 | Ctnnd1 | 1500 | 3-May-15 |
| 5296 | 2 | | | | II-1 | Ctps | 1503 | |

Fig.22 - 98

| | | | | | | | | |
|---|---|---|---|---|---|---|---|---|
| 5305 | 2 | | | | II-1 | Cts7 | | |
| 5320 | 2 | | | | II-1 | Ctsm | | |
| 5321 | 2 | | | | II-1 | Ctso | 1519 | 7-Jun-15 |
| 5338 | 2 | | | | II-1 | Cul1 | 8454 | 24-May-15 |
| 5340 | 2 | | | | II-1 | Cul3 | 8452 | 4-May-15 |
| 5341 | 2 | | | | II-1 | Cul4a | 8451 | 21-May-15 |
| 5342 | 2 | | | | II-1 | Cul4b | 8450 | 23-May-15 |
| 5343 | 2 | | | | II-1 | Cul5 | 8065 | 4-May-15 |
| 5345 | 2 | | | | II-1 | Cul9 | 23113 | 21-May-15 |
| 5354 | 2 | | | | II-1 | Cwc25 | 54883 | 4-May-15 |
| 5355 | 2 | | | | II-1 | Cwc27 | 10283 | 21-May-15 |
| 5356 | 2 | | | | II-1 | Cwf19l1 | 55280 | 4-May-15 |
| 5386 | 2 | | | | II-1 | Cxxc5 | 51523 | |
| 5390 | 2 | | | | II-1 | Cyb561d1 | 284613 | 4-May-15 |
| 5391 | 2 | | | | II-1 | Cyb561d2 | 11068 | 4-May-15 |
| 5393 | 2 | | | | II-1 | Cyb5d1 | 124637 | 4-May-15 |
| 5410 | 2 | | | | II-1 | Cylc1 | 1538 | 4-May-15 |
| 5411 | 2 | | | | II-1 | Cylc2 | 1539 | 4-May-15 |
| 5412 | 2 | | | | II-1 | Cyld | 1540 | 12-May-15 |
| 5470 | 2 | | | | II-1 | Cyp2j12 | | |
| 5497 | 2 | | | | II-1 | Cyp4a30b | | |
| 5510 | 2 | | | | II-1 | Cyp4f40 | | |
| 5512 | 2 | | | | II-1 | Cyp4v3 | | |
| 5521 | 2 | | | | II-1 | Cypt15 | | |
| 5522 | 2 | | | | II-1 | Cypt2 | | |
| 5523 | 2 | | | | II-1 | Cypt3 | | |
| 5524 | 2 | | | | II-1 | Cypt4 | | |
| 5525 | 2 | | | | II-1 | Cypt7 | | |
| 5535 | 2 | | | | II-1 | Cyth3 | 9265 | 4-May-15 |
| 5542 | 2 | | | | II-1 | D030025E07Rik | | |
| 5543 | 2 | | | | II-1 | D030025P21Rik | | |
| 5547 | 2 | | | | II-1 | D030047H15Rik | | |
| 5552 | 2 | | | | II-1 | D11Wsu47e | | |
| 5553 | 2 | | | | II-1 | D130009I18Rik | | |
| 5560 | 2 | | | | II-1 | D15Ertd621e | | |
| 5564 | 2 | | | | II-1 | D17H6S53E | | |
| 5567 | 2 | | | | II-1 | D19Bwg1357e | | |
| 5568 | 2 | | | | II-1 | D1Ertd622e | | |
| 5573 | 2 | | | | II-1 | D2Wsu81e | | |
| 5578 | 2 | | | | II-1 | D330050I16Rik | | |
| 5588 | 2 | | | | II-1 | D530049I02Rik | | |
| 5589 | 2 | | | | II-1 | D5Ertd577e | | |
| 5595 | 2 | | | | II-1 | D630023F18Rik | | |
| 5605 | 2 | | | | II-1 | D6Ertd527e | | |
| 5613 | 2 | | | | II-1 | D7Ertd443e | | |
| 5616 | 2 | | | | II-1 | D830013O20Rik | | |
| 5623 | 2 | | | | II-1 | D8Ertd738e | | |
| 5624 | 2 | | | | II-1 | D8Ertd82e | | |
| 5626 | 2 | | | | II-1 | D930015E06Rik | | |
| 5646 | 2 | | | | II-1 | Dagla | 747 | 4-May-15 |
| 5663 | 2 | | | | II-1 | Daxx | 1616 | 17-May-15 |
| 5664 | 2 | | | | II-1 | Dazap1 | 26528 | 2-Jun-15 |
| 5665 | 2 | | | | II-1 | Dazap2 | 9802 | 12-May-15 |
| 5666 | 2 | | | | II-1 | Dazl | 1618 | 12-May-15 |
| 5681 | 2 | | | | II-1 | Dbx2 | 440097 | 4-May-15 |
| 5682 | 2 | | | | II-1 | Dcaf10 | 79269 | 4-May-15 |
| 5684 | 2 | | | | II-1 | Dcaf12 | 25853 | 4-May-15 |
| 5685 | 2 | | | | II-1 | Dcaf12l1 | 139170 | 30-Apr-15 |
| 5690 | 2 | | | | II-1 | Dcaf4 | 26094 | 21-May-15 |
| 5692 | 2 | | | | II-1 | Dcaf6 | 55827 | 4-May-15 |
| 5693 | 2 | | | | II-1 | Dcaf7 | 10238 | 4-May-15 |
| 5694 | 2 | | | | II-1 | Dcaf8 | 50717 | 4-May-15 |
| 5695 | 2 | | | | II-1 | Dcakd | 79877 | 4-May-15 |
| 5703 | 2 | | | | II-1 | Dck | 1633 | 12-May-15 |
| 5713 | 2 | | | | II-1 | Dcp2 | 167227 | 4-May-15 |
| 5723 | 2 | | | | II-1 | Dctn2 | 10540 | 29-May-15 |
| 5724 | 2 | | | | II-1 | Dctn3 | 11258 | 4-May-15 |
| 5727 | 2 | | | | II-1 | Dctn6 | 10671 | 4-May-15 |
| 5728 | 2 | | | | II-1 | Dctpp1 | 79077 | 4-May-15 |
| 5730 | 2 | | | | II-1 | Dcun1d2 | 55208 | 4-May-15 |
| 5733 | 2 | | | | II-1 | Dcun1d5 | 84259 | 4-May-15 |
| 5734 | 2 | | | | II-1 | Dcx | 1641 | 31-May-15 |
| 5754 | 2 | | | | II-1 | Ddrgk1 | 65992 | 4-May-15 |
| 5757 | 2 | | | | II-1 | Ddx10 | 1662 | 4-May-15 |
| 5758 | 2 | | | | II-1 | Ddx11 | 1663 | 21-May-15 |
| 5759 | 2 | | | | II-1 | Ddx17 | 10521 | 4-May-15 |
| 5762 | 2 | | | | II-1 | Ddx19b | 11269 | 4-May-15 |
| 5763 | 2 | | | | II-1 | Ddx20 | 11218 | 28-May-15 |
| 5764 | 2 | | | | II-1 | Ddx21 | 9188 | 7-Jun-15 |
| 5765 | 2 | | | | II-1 | Ddx23 | 9416 | 4-May-15 |
| 5766 | 2 | | | | II-1 | Ddx24 | 57062 | 4-May-15 |
| 5767 | 2 | | | | II-1 | Ddx25 | 29118 | 12-May-15 |
| 5771 | 2 | | | | II-1 | Ddx31 | 64794 | 7-Jun-15 |
| 5773 | 2 | | | | II-1 | Ddx39b | 7919 | 4-May-15 |
| 5774 | 2 | | | | II-1 | Ddx3x | 1654 | 12-May-15 |
| 5775 | 2 | | | | II-1 | Ddx3y | 8653 | 23-May-15 |
| 5777 | 2 | | | | II-1 | Ddx41 | 51428 | 4-May-15 |
| 5778 | 2 | | | | II-1 | Ddx42 | 11325 | 4-May-15 |
| 5779 | 2 | | | | II-1 | Ddx43 | 55510 | 17-May-15 |
| 5780 | 2 | | | | II-1 | Ddx46 | 9879 | 3-May-15 |
| 5781 | 2 | | | | II-1 | Ddx47 | 51202 | 4-May-15 |
| 5782 | 2 | | | | II-1 | Ddx49 | 54555 | 4-May-15 |
| 5784 | 2 | | | | II-1 | Ddx50 | 79009 | 4-May-15 |
| 5785 | 2 | | | | II-1 | Ddx51 | 317781 | 4-May-15 |
| 5787 | 2 | | | | II-1 | Ddx54 | 79039 | 4-May-15 |
| 5788 | 2 | | | | II-1 | Ddx55 | 57696 | 4-May-15 |
| 5789 | 2 | | | | II-1 | Ddx56 | 54606 | 4-May-15 |
| 5792 | 2 | | | | II-1 | Ddx6 | 1656 | 4-May-15 |
| 5796 | 2 | | | | II-1 | Deb1 | | |
| 5818 | 2 | | | | II-1 | Defa-ps13 | | |
| 5819 | 2 | | | | II-1 | Defa-rs1 | | |
| 5842 | 2 | | | | II-1 | Defb34 | | |
| 5859 | 2 | | | | II-1 | Defb50 | | |
| 5875 | 2 | | | | II-1 | Dennd4b | 9909 | 4-May-15 |
| 5880 | 2 | | | | II-1 | Dennd6b | 414918 | 4-May-15 |
| 5894 | 2 | | | | II-1 | Det1 | 55070 | 4-May-15 |
| 5895 | 2 | | | | II-1 | Dexi | 28955 | 4-May-15 |
| 5900 | 2 | | | | II-1 | Dgat1 | 8694 | 4-May-15 |
| 5904 | 2 | | | | II-1 | Dgcr2 | 9993 | 7-Jun-15 |
| 5905 | 2 | | | | II-1 | Dgcr6 | 8214 | 7-Jun-15 |
| 5908 | 2 | | | | II-1 | Dgkb | 1607 | 4-May-15 |
| 5915 | 2 | | | | II-1 | Dgkk | 139189 | 12-May-15 |
| 5916 | 2 | | | | II-1 | Dgkq | 1609 | 21-May-15 |
| 5927 | 2 | | | | II-1 | Dhrs1 | 115817 | 12-May-15 |
| 5944 | 2 | | | | II-1 | Dhx33 | 56919 | 4-May-15 |
| 5946 | 2 | | | | II-1 | Dhx35 | 60625 | 4-May-15 |
| 5949 | 2 | | | | II-1 | Dhx38 | 9785 | 4-May-15 |
| 5950 | 2 | | | | II-1 | Dhx40 | 79665 | 12-May-15 |
| 5951 | 2 | | | | II-1 | Dhx57 | 90957 | 12-May-15 |
| 5952 | 2 | | | | II-1 | Dhx58 | 79132 | 4-May-15 |
| 5956 | 2 | | | | II-1 | Diap1 | | |
| 5958 | 2 | | | | II-1 | Diap3 | 81624 | 12-May-15 |
| 5962 | 2 | | | | II-1 | Dimt1 | 27292 | 4-May-15 |
| 5970 | 2 | | | | II-1 | Diras1 | 148252 | 4-May-15 |
| 5971 | 2 | | | | II-1 | Diras2 | 54769 | 4-May-15 |
| 5976 | 2 | | | | II-1 | Disc1 | 27185 | 31-May-15 |
| 5989 | 2 | | | | II-1 | Dlec1 | 9940 | 4-May-15 |
| 5990 | 2 | | | | II-1 | Dleu2 | 8847 | 12-May-15 |
| 5999 | 2 | | | | II-1 | Dlgap3 | 58512 | 4-May-15 |
| 6000 | 2 | | | | II-1 | Dlgap4 | 22839 | 4-May-15 |
| 6009 | 2 | | | | II-1 | Dlx1as | | |
| 6016 | 2 | | | | II-1 | Dlx6os1 | | |
| 6017 | 2 | | | | II-1 | Dmap1 | 55929 | 4-May-15 |
| 6031 | 2 | | | | II-1 | Dmrta2 | 63950 | 4-May-15 |
| 6032 | 2 | | | | II-1 | Dmrtb1 | 63948 | 4-May-15 |
| 6035 | 2 | | | | II-1 | Dmrtc1c2 | | |
| 6036 | 2 | | | | II-1 | Dmrtc2 | 63946 | 4-May-15 |
| 6037 | 2 | | | | II-1 | Dmtf1 | 9988 | 4-May-15 |
| 6047 | 2 | | | | II-1 | Dnah10 | 196385 | 4-May-15 |
| 6048 | 2 | | | | II-1 | Dnah11 | 8701 | 23-May-15 |
| 6049 | 2 | | | | II-1 | Dnah17 | 8632 | 4-May-15 |
| 6050 | 2 | | | | II-1 | Dnah2 | 146754 | 12-May-15 |
| 6052 | 2 | | | | II-1 | Dnah6 | 1768 | 4-May-15 |
| 6053 | 2 | | | | II-1 | Dnah7a | | |
| 6054 | 2 | | | | II-1 | Dnah7b | | |
| 6055 | 2 | | | | II-1 | Dnah8 | 1769 | 4-May-15 |
| 6058 | 2 | | | | II-1 | Dnaic2 | | |
| 6059 | 2 | | | | II-1 | Dnaja1 | 3301 | 4-May-15 |
| 6060 | 2 | | | | II-1 | Dnaja2 | 10294 | 4-May-15 |
| 6066 | 2 | | | | II-1 | Dnajb13 | 374407 | 12-May-15 |
| 6085 | 2 | | | | II-1 | Dnajc18 | 202052 | 4-May-15 |
| 6087 | 2 | | | | II-1 | Dnajc2 | 27000 | 4-May-15 |
| 6089 | 2 | | | | II-1 | Dnajc22 | 79962 | 12-May-15 |
| 6096 | 2 | | | | II-1 | Dnajc4 | 3338 | 21-May-15 |
| 6099 | 2 | | | | II-1 | Dnajc5g | 285126 | 14-May-15 |
| 6100 | 2 | | | | II-1 | Dnajc6 | 9829 | 12-May-15 |
| 6101 | 2 | | | | II-1 | Dnajc7 | 7266 | 4-May-15 |
| 6103 | 2 | | | | II-1 | Dnajc9 | 23234 | 4-May-15 |
| 6105 | 2 | | | | II-1 | Dnal4 | 10126 | 4-May-15 |
| 6118 | 2 | | | | II-1 | Dnm2 | 1785 | 23-May-15 |
| 6128 | 2 | | | | II-1 | Dnph1 | 10591 | 23-May-15 |
| 6132 | 2 | | | | II-1 | Doc2a | 8448 | 4-May-15 |
| 6147 | 2 | | | | II-1 | Dok1 | 1796 | 7-Jun-15 |
| 6163 | 2 | | | | II-1 | Dpcd | 25911 | 4-May-15 |
| 6169 | 2 | | | | II-1 | Dpf2 | 5977 | 2-Jun-15 |
| 6170 | 2 | | | | II-1 | Dpf3 | 8110 | 17-May-15 |
| 6174 | 2 | | | | II-1 | Dph5 | 51611 | 4-May-15 |
| 6175 | 2 | | | | II-1 | Dph6 | 89978 | 4-May-15 |
| 6177 | 2 | | | | II-1 | Dpm1 | 8813 | 4-May-15 |
| 6179 | 2 | | | | II-1 | Dpm3 | 54344 | 23-May-15 |
| 6180 | 2 | | | | II-1 | Dpp10 | 57628 | 4-May-15 |
| 6181 | 2 | | | | II-1 | Dpp3 | 10072 | 12-May-15 |
| 6182 | 2 | | | | II-1 | Dpp4 | 1803 | 17-May-15 |
| 6187 | 2 | | | | II-1 | Dppa1 | | |
| 6188 | 2 | | | | II-1 | Dppa2 | 151871 | 4-May-15 |
| 6189 | 2 | | | | II-1 | Dppa3 | 359787 | 4-May-15 |
| 6206 | 2 | | | | II-1 | DQ267102 | | |
| 6210 | 2 | | | | II-1 | Dram2 | 128338 | 4-May-15 |
| 6213 | 2 | | | | II-1 | Drc1 | 92749 | 7-Jun-15 |
| 6216 | 2 | | | | II-1 | Drd3 | 1814 | 17-May-15 |
| 6217 | 2 | | | | II-1 | Drd4 | 1815 | 29-May-15 |
| 6221 | 2 | | | | II-1 | Drg2 | 1819 | 4-May-15 |
| 6222 | 2 | | | | II-1 | Drosha | 29102 | 4-May-15 |
| 6228 | 2 | | | | II-1 | Dscaml1 | 57453 | 4-May-15 |
| 6229 | 2 | | | | II-1 | Dscc1 | 79075 | 4-May-15 |
| 6232 | 2 | | | | II-1 | Dsel | 92126 | 4-May-15 |
| 6235 | 2 | | | | II-1 | Dsg1c | | |
| 6238 | 2 | | | | II-1 | Dsg4 | 147409 | 4-May-15 |
| 6239 | 2 | | | | II-1 | Dsn1 | 79980 | 4-May-15 |
| 6242 | 2 | | | | II-1 | Dst | 667 | 12-May-15 |
| 6245 | 2 | | | | II-1 | Dtd1 | 92675 | 4-May-15 |
| 6247 | 2 | | | | II-1 | Dthd1 | 401124 | 4-May-15 |

Fig.22 - 99

| | | | | | | | | |
|---|---|---|---|---|---|---|---|---|
| 6248 | 2 | | | | II-1 | Dtl | 51514 | 7-Jun-15 |
| 6258 | 2 | | | | II-1 | Dtx4 | 23220 | 12-May-15 |
| 6267 | 2 | | | | II-1 | Dus3l | 56931 | 23-May-15 |
| 6299 | 2 | | | | II-1 | Dvl2 | 1856 | 3-May-15 |
| 6306 | 2 | | | | II-1 | Dynap | 284254 | 12-May-15 |
| 6307 | 2 | | | | II-1 | Dync1h1 | 1778 | 23-May-15 |
| 6310 | 2 | | | | II-1 | Dync1li1 | 51143 | 4-May-15 |
| 6311 | 2 | | | | II-1 | Dync1li2 | 1783 | 4-May-15 |
| 6312 | 2 | | | | II-1 | Dync2h1 | 79659 | 23-May-15 |
| 6313 | 2 | | | | II-1 | Dync2li1 | 51626 | 4-May-15 |
| 6324 | 2 | | | | II-1 | Dyrk1b | 9149 | 4-May-15 |
| 6325 | 2 | | | | II-1 | Dyrk2 | 8445 | 12-May-15 |
| 6329 | 2 | | | | II-1 | Dytn | 391475 | 4-May-15 |
| 6330 | 2 | | | | II-1 | Dyx1c1 | 161582 | 4-May-15 |
| 6335 | 2 | | | | II-1 | E030002O03Rik | | |
| 6347 | 2 | | | | II-1 | E130008D07Rik | | |
| 6356 | 2 | | | | II-1 | E130304I02Rik | | |
| 6357 | 2 | | | | II-1 | E130307A14Rik | | |
| 6375 | 2 | | | | II-1 | E2f5 | 1875 | 4-May-15 |
| 6376 | 2 | | | | II-1 | E2f6 | 1876 | 4-May-15 |
| 6385 | 2 | | | | II-1 | E330017L17Rik | | |
| 6388 | 2 | | | | II-1 | E330023G01Rik | | |
| 6389 | 2 | | | | II-1 | E330033B04Rik | | |
| 6390 | 2 | | | | II-1 | E330034G19Rik | | |
| 6392 | 2 | | | | II-1 | E430018J23Rik | | |
| 6398 | 2 | | | | II-1 | Eaf2 | 55840 | 7-Jun-15 |
| 6409 | 2 | | | | II-1 | Ebag9 | 9166 | 17-May-15 |
| 6417 | 2 | | | | II-1 | Ebpl | 84650 | 4-May-15 |
| 6420 | 2 | | | | II-1 | Ece2 | 9718 | 4-May-15 |
| 6422 | 2 | | | | II-1 | Ech1 | 1891 | 23-May-15 |
| 6447 | 2 | | | | II-1 | Edil3 | 10085 | 4-May-15 |
| 6477 | 2 | | | | II-1 | Efcab7 | 84455 | 4-May-15 |
| 6480 | 2 | | | | II-1 | Efcc1 | 79825 | 4-May-15 |
| 6485 | 2 | | | | II-1 | Efhc2 | 80258 | 4-May-15 |
| 6497 | 2 | | | | II-1 | Efr3b | 22979 | 4-May-15 |
| 6526 | 2 | | | | II-1 | Ei24 | 9538 | 21-May-15 |
| 6527 | 2 | | | | II-1 | Eid1 | 23741 | 4-May-15 |
| 6528 | 2 | | | | II-1 | Eid2 | 163126 | 4-May-15 |
| 6533 | 2 | | | | II-1 | Eif1ad | 84285 | 4-May-15 |
| 6534 | 2 | | | | II-1 | Eif1ax | 1964 | 4-May-15 |
| 6535 | 2 | | | | II-1 | Eif1b | 10289 | 4-May-15 |
| 6536 | 2 | | | | II-1 | Eif2a | 83939 | 7-Jun-15 |
| 6543 | 2 | | | | II-1 | Eif2b3 | 8891 | 23-May-15 |
| 6547 | 2 | | | | II-1 | Eif2s1 | 1965 | 28-May-15 |
| 6548 | 2 | | | | II-1 | Eif2s2 | 8894 | 4-May-15 |
| 6549 | 2 | | | | II-1 | Eif2s3x | | |
| 6550 | 2 | | | | II-1 | Eif2s3y | | |
| 6551 | 2 | | | | II-1 | Eif3a | 8661 | 13-Jun-15 |
| 6552 | 2 | | | | II-1 | Eif3b | 8662 | 12-May-15 |
| 6553 | 2 | | | | II-1 | Eif3c | 8663 | 4-May-15 |
| 6554 | 2 | | | | II-1 | Eif3d | 8664 | 4-May-15 |
| 6555 | 2 | | | | II-1 | Eif3e | 3646 | 12-May-15 |
| 6558 | 2 | | | | II-1 | Eif3h | 8667 | 12-May-15 |
| 6559 | 2 | | | | II-1 | Eif3i | 8668 | 4-May-15 |
| 6564 | 2 | | | | II-1 | Eif3m | 10480 | 12-May-15 |
| 6567 | 2 | | | | II-1 | Eif4a3 | 9775 | 4-May-15 |
| 6568 | 2 | | | | II-1 | Eif4b | 1975 | 17-May-15 |
| 6569 | 2 | | | | II-1 | Eif4e | 1977 | 31-May-15 |
| 6570 | 2 | | | | II-1 | Eif4e1b | 253314 | 4-May-15 |
| 6571 | 2 | | | | II-1 | Eif4e2 | 9470 | 31-May-15 |
| 6572 | 2 | | | | II-1 | Eif4e3 | 317649 | 4-May-15 |
| 6578 | 2 | | | | II-1 | Eif4g2 | 1982 | 4-May-15 |
| 6579 | 2 | | | | II-1 | Eif4g3 | 8672 | 4-May-15 |
| 6580 | 2 | | | | II-1 | Eif4h | 7458 | 12-May-15 |
| 6582 | 2 | | | | II-1 | Eif5a | 1984 | 7-Jun-15 |
| 6583 | 2 | | | | II-1 | Eif5a2 | 56648 | 4-May-15 |
| 6587 | 2 | | | | II-1 | Elac2 | 60528 | 4-May-15 |
| 6592 | 2 | | | | II-1 | Elavl4 | 1996 | 12-May-15 |
| 6593 | 2 | | | | II-1 | Elf1 | 1997 | 7-Jun-15 |
| 6608 | 2 | | | | II-1 | Elmo3 | 79767 | 4-May-15 |
| 6611 | 2 | | | | II-1 | Elmod3 | 84173 | 4-May-15 |
| 6624 | 2 | | | | II-1 | Elp4 | 26610 | 23-May-15 |
| 6631 | 2 | | | | II-1 | Emc2 | 9694 | 12-May-15 |
| 6634 | 2 | | | | II-1 | Emc6 | 83460 | 21-May-15 |
| 6635 | 2 | | | | II-1 | Emc7 | 56851 | 4-May-15 |
| 6648 | 2 | | | | II-1 | Eml2 | 24139 | 12-May-15 |
| 6652 | 2 | | | | II-1 | Eml6 | 400954 | 12-May-15 |
| 6661 | 2 | | | | II-1 | En1 | 2019 | 4-May-15 |
| 6662 | 2 | | | | II-1 | En2 | 2020 | 4-May-15 |
| 6682 | 2 | | | | II-1 | Enox2 | 10495 | 17-May-15 |
| 6692 | 2 | | | | II-1 | Enthd1 | 150350 | 4-May-15 |
| 6693 | 2 | | | | II-1 | Enthd2 | 146705 | 4-May-15 |
| 6694 | 2 | | | | II-1 | Entpd1 | 953 | 12-May-15 |
| 6729 | 2 | | | | II-1 | Epha7 | 2045 | 17-May-15 |
| 6732 | 2 | | | | II-1 | Ephb2 | 2048 | 23-May-15 |
| 6762 | 2 | | | | II-1 | Erap1 | 51752 | 21-May-15 |
| 6764 | 2 | | | | II-1 | Erbb2 | 2064 | 31-May-15 |
| 6769 | 2 | | | | II-1 | Erc2 | 26059 | 12-May-15 |
| 6774 | 2 | | | | II-1 | Ercc5 | 2073 | 23-May-15 |
| 6775 | 2 | | | | II-1 | Ercc6 | 2074 | 23-May-15 |
| 6776 | 2 | | | | II-1 | Ercc6l | 54821 | 10-May-15 |
| 6785 | 2 | | | | II-1 | Ergic3 | 51614 | 2-Jun-15 |
| 6786 | 2 | | | | II-1 | Erh | 2079 | 4-May-15 |
| 6798 | 2 | | | | II-1 | Erlin2 | 11160 | 29-May-15 |
| 6820 | 2 | | | | II-1 | Esp16 | | |
| 6824 | 2 | | | | II-1 | Esp3 | | |
| 6832 | 2 | | | | II-1 | Esp6-esp5 | | |

| | | | | | | | | |
|---|---|---|---|---|---|---|---|---|
| 6846 | 2 | | | | II-1 | Esyt2 | 57488 | 4-May-15 |
| 6849 | 2 | | | | II-1 | Etd | | |
| 6850 | 2 | | | | II-1 | Etf1 | 2107 | 2-Jun-15 |
| 6851 | 2 | | | | II-1 | Etfa | 2108 | 21-May-15 |
| 6854 | 2 | | | | II-1 | Ethe1 | 23474 | 4-May-15 |
| 6865 | 2 | | | | II-1 | Etv3 | 2117 | 28-May-15 |
| 6884 | 2 | | | | II-1 | Ewsr1 | 2130 | 21-May-15 |
| 6885 | 2 | | | | II-1 | Exd1 | 161829 | 4-May-15 |
| 6891 | 2 | | | | II-1 | Exoc3 | 11336 | 4-May-15 |
| 6892 | 2 | | | | II-1 | Exoc3l | 283849 | 4-May-15 |
| 6899 | 2 | | | | II-1 | Exoc8 | 149371 | 4-May-15 |
| 6901 | 2 | | | | II-1 | Exosc1 | 51013 | 2-Jun-15 |
| 6903 | 2 | | | | II-1 | Exosc2 | 23404 | 20-May-15 |
| 6905 | 2 | | | | II-1 | Exosc4 | 54512 | 4-May-15 |
| 6906 | 2 | | | | II-1 | Exosc5 | 56915 | 4-May-15 |
| 6928 | 2 | | | | II-1 | F13a1 | 2162 | 23-May-15 |
| 6941 | 2 | | | | II-1 | F630206G17Rik | | |
| 6950 | 2 | | | | II-1 | F9 | 2158 | 23-May-15 |
| 6951 | 2 | | | | II-1 | F930015N05Rik | | |
| 6982 | 2 | | | | II-1 | Fam105a | 54491 | 4-May-15 |
| 7000 | 2 | | | | II-1 | Fam120a | 23196 | 4-May-15 |
| 7002 | 2 | | | | II-1 | Fam120b | 84498 | 12-May-15 |
| 7004 | 2 | | | | II-1 | Fam122a | 116224 | 4-May-15 |
| 7006 | 2 | | | | II-1 | Fam122c | 159091 | 12-May-15 |
| 7023 | 2 | | | | II-1 | Fam135a | 57579 | 4-May-15 |
| 7024 | 2 | | | | II-1 | Fam135b | 51059 | 14-May-15 |
| 7025 | 2 | | | | II-1 | Fam136a | 84908 | 4-May-15 |
| 7037 | 2 | | | | II-1 | Fam155a | 728215 | 4-May-15 |
| 7038 | 2 | | | | II-1 | Fam159a | 348378 | 4-May-15 |
| 7043 | 2 | | | | II-1 | Fam160b2 | 64760 | 4-May-15 |
| 7044 | 2 | | | | II-1 | Fam161a | 84140 | 23-May-15 |
| 7048 | 2 | | | | II-1 | Fam163a | 148753 | 14-May-15 |
| 7060 | 2 | | | | II-1 | Fam171a1 | 221061 | 12-May-15 |
| 7065 | 2 | | | | II-1 | Fam173b | 134145 | 4-May-15 |
| 7067 | 2 | | | | II-1 | Fam174b | 400451 | 4-May-15 |
| 7081 | 2 | | | | II-1 | Fam186b | 84070 | 4-May-15 |
| 7082 | 2 | | | | II-1 | Fam187a | 100528020 | 7-Dec-14 |
| 7090 | 2 | | | | II-1 | Fam193a | 8603 | 4-May-15 |
| 7091 | 2 | | | | II-1 | Fam193b | 54540 | 4-May-15 |
| 7100 | 2 | | | | II-1 | Fam19a2 | 338811 | 21-May-15 |
| 7101 | 2 | | | | II-1 | Fam19a3 | 284467 | 4-May-15 |
| 7102 | 2 | | | | II-1 | Fam19a4 | 151647 | 4-May-15 |
| 7106 | 2 | | | | II-1 | Fam206a | 54942 | 3-May-15 |
| 7107 | 2 | | | | II-1 | Fam207a | 85395 | 4-May-15 |
| 7109 | 2 | | | | II-1 | Fam208b | 54906 | 4-May-15 |
| 7124 | 2 | | | | II-1 | Fam216b | 144809 | 4-May-15 |
| 7130 | 2 | | | | II-1 | Fam220a | 84792 | 4-May-15 |
| 7136 | 2 | | | | II-1 | Fam227b | 196951 | 4-May-15 |
| 7137 | 2 | | | | II-1 | Fam228a | 653140 | 4-May-15 |
| 7138 | 2 | | | | II-1 | Fam228b | 375190 | 12-May-15 |
| 7159 | 2 | | | | II-1 | Fam47e | 100129583 | 4-May-15 |
| 7160 | 2 | | | | II-1 | Fam49a | 81553 | 4-May-15 |
| 7164 | 2 | | | | II-1 | Fam53a | 152877 | 21-May-15 |
| 7165 | 2 | | | | II-1 | Fam53b | 9679 | 4-May-15 |
| 7166 | 2 | | | | II-1 | Fam53c | 51307 | 12-May-15 |
| 7190 | 2 | | | | II-1 | Fam76a | 199870 | 4-May-15 |
| 7211 | 2 | | | | II-1 | Fam96b | 51647 | 4-May-15 |
| 7218 | 2 | | | | II-1 | Fancc | 2176 | 28-May-15 |
| 7225 | 2 | | | | II-1 | Fancl | 55120 | 23-May-15 |
| 7236 | 2 | | | | II-1 | Fas | 355 | 13-Jun-15 |
| 7241 | 2 | | | | II-1 | Fastkd2 | 22868 | 4-May-15 |
| 7242 | 2 | | | | II-1 | Fastkd3 | 79072 | 4-May-15 |
| 7243 | 2 | | | | II-1 | Fastkd5 | 60493 | 4-May-15 |
| 7247 | 2 | | | | II-1 | Fat4 | 79633 | 4-May-15 |
| 7255 | 2 | | | | II-1 | Fbln1 | 2192 | 12-May-15 |
| 7266 | 2 | | | | II-1 | Fbxl12os | | |
| 7268 | 2 | | | | II-1 | Fbxl14 | 144699 | 2-Jun-15 |
| 7269 | 2 | | | | II-1 | Fbxl15 | 79176 | 4-May-15 |
| 7277 | 2 | | | | II-1 | Fbxl22 | 283807 | 4-May-15 |
| 7280 | 2 | | | | II-1 | Fbxl5 | 26234 | 4-May-15 |
| 7281 | 2 | | | | II-1 | Fbxl6 | 26233 | 4-May-15 |
| 7283 | 2 | | | | II-1 | Fbxl8 | 55336 | 4-May-15 |
| 7286 | 2 | | | | II-1 | Fbxo15 | 201456 | 4-May-15 |
| 7287 | 2 | | | | II-1 | Fbxo16 | 157574 | 4-May-15 |
| 7293 | 2 | | | | II-1 | Fbxo24 | 26261 | 4-May-15 |
| 7294 | 2 | | | | II-1 | Fbxo25 | 26260 | 4-May-15 |
| 7306 | 2 | | | | II-1 | Fbxo4 | 26272 | 4-May-15 |
| 7310 | 2 | | | | II-1 | Fbxo43 | 286151 | 4-May-15 |
| 7311 | 2 | | | | II-1 | Fbxo44 | 93611 | 12-May-15 |
| 7314 | 2 | | | | II-1 | Fbxo47 | 494188 | 4-May-15 |
| 7319 | 2 | | | | II-1 | Fbxo8 | 26269 | 4-May-15 |
| 7320 | 2 | | | | II-1 | Fbxo9 | 26268 | 4-May-15 |
| 7321 | 2 | | | | II-1 | Fbxw10 | 10517 | 4-May-15 |
| 7322 | 2 | | | | II-1 | Fbxw11 | 23291 | 4-May-15 |
| 7323 | 2 | | | | II-1 | Fbxw13 | | |
| 7324 | 2 | | | | II-1 | Fbxw14 | | |
| 7325 | 2 | | | | II-1 | Fbxw15 | | |
| 7331 | 2 | | | | II-1 | Fbxw20 | | |
| 7333 | 2 | | | | II-1 | Fbxw22 | | |
| 7362 | 2 | | | | II-1 | Fcrla | 84824 | 4-May-15 |
| 7379 | 2 | | | | II-1 | Ferd3l | 222894 | 4-May-15 |
| 7419 | 2 | | | | II-1 | Fgf4 | 2249 | 4-May-15 |
| 7420 | 2 | | | | II-1 | Fgf5 | 2250 | 12-May-15 |
| 7423 | 2 | | | | II-1 | Fgf8 | 2253 | 23-May-15 |
| 7424 | 2 | | | | II-1 | Fgf9 | 2254 | 4-May-15 |
| 7432 | 2 | | | | II-1 | Fgfr4 | 2264 | 18-May-15 |

Fig.22 - 100

| | | | | | | | | |
|---|---|---|---|---|---|---|---|---|
| 7441 | 2 | | | | II-1 | Fhad1os1 | | |
| 7458 | 2 | | | | II-1 | Fign | 55137 | 4-May-15 |
| 7481 | 2 | | | | II-1 | Fkbp7 | 51661 | 4-May-15 |
| 7482 | 2 | | | | II-1 | Fkbp8 | 23770 | 12-May-15 |
| 7489 | 2 | | | | II-1 | Flg2 | 388698 | 12-May-15 |
| 7493 | 2 | | | | II-1 | Flnb | 2317 | 23-May-15 |
| 7508 | 2 | | | | II-1 | Fmnl1 | 752 | 14-May-15 |
| 7526 | 2 | | | | II-1 | Fnbp4 | 23360 | 21-May-15 |
| 7527 | 2 | | | | II-1 | Fnd3c2 | | |
| 7528 | 2 | | | | II-1 | Fndc1 | 84624 | 4-May-15 |
| 7531 | 2 | | | | II-1 | Fndc3c1 | | |
| 7536 | 2 | | | | II-1 | Fndc9 | 408263 | 4-May-15 |
| 7539 | 2 | | | | II-1 | Fnta | 2339 | 4-May-15 |
| 7555 | 2 | | | | II-1 | Foxb2 | 442425 | 28-May-15 |
| 7574 | 2 | | | | II-1 | Foxj3 | 22887 | 28-May-15 |
| 7575 | 2 | | | | II-1 | Foxk1 | 221937 | 4-May-15 |
| 7576 | 2 | | | | II-1 | Foxk2 | 3607 | 28-May-15 |
| 7591 | 2 | | | | II-1 | Foxp3 | 50943 | 31-May-15 |
| 7596 | 2 | | | | II-1 | Foxred1 | 55572 | 23-May-15 |
| 7604 | 2 | | | | II-1 | Fpr-rs3 | | |
| 7606 | 2 | | | | II-1 | Fpr-rs6 | | |
| 7607 | 2 | | | | II-1 | Fra10ac1 | 118924 | 12-May-15 |
| 7626 | 2 | | | | II-1 | Frmpd4 | 9758 | 4-May-15 |
| 7627 | 2 | | | | II-1 | Frrs1 | 391059 | 4-May-15 |
| 7629 | 2 | | | | II-1 | Frs2 | 10818 | 4-May-15 |
| 7641 | 2 | | | | II-1 | Fsd1l | 83856 | 12-May-15 |
| 7657 | 2 | | | | II-1 | Ftsj1 | 24140 | 23-May-15 |
| 7659 | 2 | | | | II-1 | Ftsj3 | 117246 | 4-May-15 |
| 7661 | 2 | | | | II-1 | Fubp1 | 8880 | 12-May-15 |
| 7664 | 2 | | | | II-1 | Fuca2 | 2519 | 4-May-15 |
| 7678 | 2 | | | | II-1 | Fut8 | 2530 | 17-May-15 |
| 7680 | 2 | | | | II-1 | Fuz | 80199 | 4-May-15 |
| 7696 | 2 | | | | II-1 | Fzd1 | 8321 | 4-May-15 |
| 7711 | 2 | | | | II-1 | G530011O06Rik | | |
| 7715 | 2 | | | | II-1 | G630090E17Rik | | |
| 7722 | 2 | | | | II-1 | G6pd2 | | |
| 7730 | 2 | | | | II-1 | Gabarapl1 | 23710 | 21-May-15 |
| 7731 | 2 | | | | II-1 | Gabarapl2 | 11345 | 21-May-15 |
| 7732 | 2 | | | | II-1 | Gabbr1 | 2550 | 7-Jun-15 |
| 7733 | 2 | | | | II-1 | Gabbr2 | 9568 | 4-May-15 |
| 7739 | 2 | | | | II-1 | Gabra3 | 2556 | 12-May-15 |
| 7742 | 2 | | | | II-1 | Gabra6 | 2559 | 4-May-15 |
| 7743 | 2 | | | | II-1 | Gabrb1 | 2560 | 12-May-15 |
| 7744 | 2 | | | | II-1 | Gabrb2 | 2561 | 12-May-15 |
| 7745 | 2 | | | | II-1 | Gabrb3 | 2562 | 4-May-15 |
| 7749 | 2 | | | | II-1 | Gabrg2 | 2566 | 2-Jun-15 |
| 7750 | 2 | | | | II-1 | Gabrg3 | 2567 | 12-May-15 |
| 7751 | 2 | | | | II-1 | Gabrp | 2568 | 4-May-15 |
| 7754 | 2 | | | | II-1 | Gabrr2 | 2570 | 4-May-15 |
| 7755 | 2 | | | | II-1 | Gabrr3 | 200959 | 13-May-15 |
| 7756 | 2 | | | | II-1 | Gad1 | 2571 | 24-May-15 |
| 7793 | 2 | | | | II-1 | Galntl6 | 442117 | 12-May-15 |
| 7794 | 2 | | | | II-1 | Galp | 85569 | 4-May-15 |
| 7795 | 2 | | | | II-1 | Galr1 | 2587 | 12-May-15 |
| 7796 | 2 | | | | II-1 | Galr2 | 8811 | 4-May-15 |
| 7812 | 2 | | | | II-1 | Gars | 2617 | 7-Jun-15 |
| 7830 | 2 | | | | II-1 | Gata6 | 2627 | 24-May-15 |
| 7832 | 2 | | | | II-1 | Gatad2a | 54815 | 12-May-15 |
| 7840 | 2 | | | | II-1 | Gbas | 2631 | 4-May-15 |
| 7862 | 2 | | | | II-1 | Gcdh | 2639 | 31-May-15 |
| 7880 | 2 | | | | II-1 | Gcsam | 257144 | 4-May-15 |
| 7887 | 2 | | | | II-1 | Gde1 | 51573 | 4-May-15 |
| 7900 | 2 | | | | II-1 | Gdnf | 2668 | 23-May-15 |
| 7901 | 2 | | | | II-1 | Gdpd1 | 284161 | 4-May-15 |
| 7904 | 2 | | | | II-1 | Gdpd4 | 220032 | 4-May-15 |
| 7911 | 2 | | | | II-1 | Gemin6 | 79833 | 4-May-15 |
| 7913 | 2 | | | | II-1 | Gemin8 | 54960 | 4-May-15 |
| 7918 | 2 | | | | II-1 | Gfi1 | 2672 | 4-May-15 |
| 7922 | 2 | | | | II-1 | Gfod1 | 54438 | 14-May-15 |
| 7925 | 2 | | | | II-1 | Gfpt2 | 9945 | 4-May-15 |
| 7932 | 2 | | | | II-1 | Gga1 | 26088 | 21-May-15 |
| 7935 | 2 | | | | II-1 | Ggact | 87769 | 4-May-15 |
| 7954 | 2 | | | | II-1 | Ghrl | 51738 | 21-May-15 |
| 7957 | 2 | | | | II-1 | Gid8 | 54994 | 4-May-15 |
| 7959 | 2 | | | | II-1 | Gigyf1 | 64599 | 4-May-15 |
| 7982 | 2 | | | | II-1 | Gja1 | 2697 | 24-May-15 |
| 8014 | 2 | | | | II-1 | Glce | 26035 | 4-May-15 |
| 8019 | 2 | | | | II-1 | Glg1 | 2734 | 4-May-15 |
| 8033 | 2 | | | | II-1 | Glod5 | 392465 | 4-May-15 |
| 8034 | 2 | | | | II-1 | Glp1r | 2740 | 17-May-15 |
| 8037 | 2 | | | | II-1 | Glra2 | 2742 | 12-May-15 |
| 8047 | 2 | | | | II-1 | Gls2 | 27165 | 24-May-15 |
| 8050 | 2 | | | | II-1 | Glt28d2 | | |
| 8063 | 2 | | | | II-1 | Glyatl3 | 389396 | 4-May-15 |
| 8068 | 2 | | | | II-1 | Gm10012 | | |
| 8074 | 2 | | | | II-1 | Gm10058 | | |
| 8080 | 2 | | | | II-1 | Gm10100 | | |
| 8091 | 2 | | | | II-1 | Gm10267 | | |
| 8100 | 2 | | | | II-1 | Gm10364 | | |
| 8102 | 2 | | | | II-1 | Gm10375 | | |
| 8105 | 2 | | | | II-1 | Gm10390 | | |
| 8106 | 2 | | | | II-1 | Gm10400 | | |
| 8112 | 2 | | | | II-1 | Gm10416 | | |
| 8120 | 2 | | | | II-1 | Gm1045 | | |
| 8124 | 2 | | | | II-1 | Gm10474 | | |
| 8132 | 2 | | | | II-1 | Gm10516 | | |
| 8135 | 2 | | | | II-1 | Gm10538 | | |

| | | | | | | | | |
|---|---|---|---|---|---|---|---|---|
| 8136 | 2 | | | | II-1 | Gm10548 | | |
| 8145 | 2 | | | | II-1 | Gm10636 | | |
| 8150 | 2 | | | | II-1 | Gm10649 | | |
| 8155 | 2 | | | | II-1 | Gm10666 | | |
| 8161 | 2 | | | | II-1 | Gm10714 | | |
| 8165 | 2 | | | | II-1 | Gm10767 | | |
| 8168 | 2 | | | | II-1 | Gm10782 | | |
| 8170 | 2 | | | | II-1 | Gm10787 | | |
| 8175 | 2 | | | | II-1 | Gm10814 | | |
| 8178 | 2 | | | | II-1 | Gm10845 | | |
| 8185 | 2 | | | | II-1 | Gm10941 | | |
| 8193 | 2 | | | | II-1 | Gm11190 | | |
| 8194 | 2 | | | | II-1 | Gm11201 | | |
| 8196 | 2 | | | | II-1 | Gm1123 | | |
| 8197 | 2 | | | | II-1 | Gm11237 | | |
| 8198 | 2 | | | | II-1 | Gm11240 | | |
| 8202 | 2 | | | | II-1 | Gm1141 | | |
| 8203 | 2 | | | | II-1 | Gm11413 | | |
| 8205 | 2 | | | | II-1 | Gm11437 | | |
| 8211 | 2 | | | | II-1 | Gm11541 | | |
| 8213 | 2 | | | | II-1 | Gm11545 | | |
| 8235 | 2 | | | | II-1 | Gm11758 | | |
| 8246 | 2 | | | | II-1 | Gm11985 | | |
| 8247 | 2 | | | | II-1 | Gm11992 | | |
| 8248 | 2 | | | | II-1 | Gm12 | | |
| 8253 | 2 | | | | II-1 | Gm12169 | | |
| 8255 | 2 | | | | II-1 | Gm12185 | | |
| 8262 | 2 | | | | II-1 | Gm12298 | | |
| 8270 | 2 | | | | II-1 | Gm12530 | | |
| 8271 | 2 | | | | II-1 | Gm12603 | | |
| 8272 | 2 | | | | II-1 | Gm12633 | | |
| 8276 | 2 | | | | II-1 | Gm12709 | | |
| 8283 | 2 | | | | II-1 | Gm12887 | | |
| 8291 | 2 | | | | II-1 | Gm13032 | | |
| 8295 | 2 | | | | II-1 | Gm13051 | | |
| 8298 | 2 | | | | II-1 | Gm13083 | | |
| 8316 | 2 | | | | II-1 | Gm13242 | | |
| 8321 | 2 | | | | II-1 | Gm13275 | | |
| 8342 | 2 | | | | II-1 | Gm13490 | | |
| 8347 | 2 | | | | II-1 | Gm13546 | | |
| 8350 | 2 | | | | II-1 | Gm13582 | | |
| 8356 | 2 | | | | II-1 | Gm13769 | | |
| 8360 | 2 | | | | II-1 | Gm13889 | | |
| 8361 | 2 | | | | II-1 | Gm13939 | | |
| 8366 | 2 | | | | II-1 | Gm14057 | | |
| 8373 | 2 | | | | II-1 | Gm14164 | | |
| 8375 | 2 | | | | II-1 | Gm14204 | | |
| 8382 | 2 | | | | II-1 | Gm14322 | | |
| 8387 | 2 | | | | II-1 | Gm14346 | | |
| 8388 | 2 | | | | II-1 | Gm14347 | | |
| 8391 | 2 | | | | II-1 | Gm14378 | | |
| 8404 | 2 | | | | II-1 | Gm14461 | | |
| 8405 | 2 | | | | II-1 | Gm14474 | | |
| 8409 | 2 | | | | II-1 | Gm14478 | | |
| 8410 | 2 | | | | II-1 | Gm14479 | | |
| 8411 | 2 | | | | II-1 | Gm14482 | | |
| 8413 | 2 | | | | II-1 | Gm14484 | | |
| 8414 | 2 | | | | II-1 | Gm14496 | | |
| 8418 | 2 | | | | II-1 | Gm14525 | | |
| 8422 | 2 | | | | II-1 | Gm14634 | | |
| 8423 | 2 | | | | II-1 | Gm14635 | | |
| 8425 | 2 | | | | II-1 | Gm14692 | | |
| 8429 | 2 | | | | II-1 | Gm14744 | | |
| 8430 | 2 | | | | II-1 | Gm14781 | | |
| 8433 | 2 | | | | II-1 | Gm14827 | | |
| 8440 | 2 | | | | II-1 | Gm15055 | | |
| 8446 | 2 | | | | II-1 | Gm15107 | | |
| 8447 | 2 | | | | II-1 | Gm15114 | | |
| 8448 | 2 | | | | II-1 | Gm15127 | | |
| 8449 | 2 | | | | II-1 | Gm15133 | | |
| 8451 | 2 | | | | II-1 | Gm15179 | | |
| 8453 | 2 | | | | II-1 | Gm1527 | | |
| 8469 | 2 | | | | II-1 | Gm15417 | | |
| 8475 | 2 | | | | II-1 | Gm1553 | | |
| 8483 | 2 | | | | II-1 | Gm15698 | | |
| 8494 | 2 | | | | II-1 | Gm15880 | | |
| 8495 | 2 | | | | II-1 | Gm15881 | | |
| 8507 | 2 | | | | II-1 | Gm16157 | | |
| 8508 | 2 | | | | II-1 | Gm16287 | | |
| 8509 | 2 | | | | II-1 | Gm16291 | | |
| 8514 | 2 | | | | II-1 | Gm16367 | | |
| 8515 | 2 | | | | II-1 | Gm16381 | | |
| 8518 | 2 | | | | II-1 | Gm16404 | | |
| 8519 | 2 | | | | II-1 | Gm16405 | | |
| 8522 | 2 | | | | II-1 | Gm16445 | | |
| 8524 | 2 | | | | II-1 | Gm1647 | | |
| 8525 | 2 | | | | II-1 | Gm16497 | | |
| 8530 | 2 | | | | II-1 | Gm16532 | | |
| 8547 | 2 | | | | II-1 | Gm16833 | | |
| 8553 | 2 | | | | II-1 | Gm16894 | | |
| 8555 | 2 | | | | II-1 | Gm16907 | | |
| 8561 | 2 | | | | II-1 | Gm17066 | | |
| 8566 | 2 | | | | II-1 | Gm17359 | | |
| 8569 | 2 | | | | II-1 | Gm17644 | | |
| 8577 | 2 | | | | II-1 | Gm17757 | | |
| 8580 | 2 | | | | II-1 | Gm17801 | | |
| 8581 | 2 | | | | II-1 | Gm17821 | | |

Fig.22 - 101

| | | | | | | | | |
|---|---|---|---|---|---|---|---|---|
| 8586 | 2 | | | | II-1 | Gm19276 | | |
| 8591 | 2 | | | | II-1 | Gm19395 | | |
| 8595 | 2 | | | | II-1 | Gm19434 | | |
| 8597 | 2 | | | | II-1 | Gm19466 | | |
| 8599 | 2 | | | | II-1 | Gm19522 | | |
| 8603 | 2 | | | | II-1 | Gm19619 | | |
| 8612 | 2 | | | | II-1 | Gm1976 | | |
| 8615 | 2 | | | | II-1 | Gm1979 | | |
| 8623 | 2 | | | | II-1 | Gm20098 | | |
| 8627 | 2 | | | | II-1 | Gm20125 | | |
| 8629 | 2 | | | | II-1 | Gm2016 | | |
| 8631 | 2 | | | | II-1 | Gm20187 | | |
| 8644 | 2 | | | | II-1 | Gm2042 | | |
| 8648 | 2 | | | | II-1 | Gm20597 | | |
| 8653 | 2 | | | | II-1 | Gm20735 | | |
| 8654 | 2 | | | | II-1 | Gm20736 | | |
| 8657 | 2 | | | | II-1 | Gm20741 | | |
| 8658 | 2 | | | | II-1 | Gm20743 | | |
| 8661 | 2 | | | | II-1 | Gm20747 | | |
| 8664 | 2 | | | | II-1 | Gm20751 | | |
| 8665 | 2 | | | | II-1 | Gm20752 | | |
| 8669 | 2 | | | | II-1 | Gm20756 | | |
| 8670 | 2 | | | | II-1 | Gm20757 | | |
| 8671 | 2 | | | | II-1 | Gm20758 | | |
| 8673 | 2 | | | | II-1 | Gm20765 | | |
| 8679 | 2 | | | | II-1 | Gm20822 | | |
| 8680 | 2 | | | | II-1 | Gm20823 | | |
| 8685 | 2 | | | | II-1 | Gm20857 | | |
| 8687 | 2 | | | | II-1 | Gm20865 | | |
| 8688 | 2 | | | | II-1 | Gm20867 | | |
| 8689 | 2 | | | | II-1 | Gm2087 | | |
| 8690 | 2 | | | | II-1 | Gm20871 | | |
| 8698 | 2 | | | | II-1 | Gm21119 | | |
| 8699 | 2 | | | | II-1 | Gm2115 | | |
| 8702 | 2 | | | | II-1 | Gm21276 | | |
| 8703 | 2 | | | | II-1 | Gm21283 | | |
| 8704 | 2 | | | | II-1 | Gm21284 | | |
| 8705 | 2 | | | | II-1 | Gm21293 | | |
| 8706 | 2 | | | | II-1 | Gm21304 | | |
| 8707 | 2 | | | | II-1 | Gm21312 | | |
| 8714 | 2 | | | | II-1 | Gm21693 | | |
| 8717 | 2 | | | | II-1 | Gm21943 | | |
| 8718 | 2 | | | | II-1 | Gm21944 | | |
| 8719 | 2 | | | | II-1 | Gm21949 | | |
| 8720 | 2 | | | | II-1 | Gm21950 | | |
| 8724 | 2 | | | | II-1 | Gm2382 | | |
| 8727 | 2 | | | | II-1 | Gm2518 | | |
| 8734 | 2 | | | | II-1 | Gm2799 | | |
| 8736 | 2 | | | | II-1 | Gm2837 | | |
| 8737 | 2 | | | | II-1 | Gm2848 | | |
| 8738 | 2 | | | | II-1 | Gm2863 | | |
| 8740 | 2 | | | | II-1 | Gm2913 | | |
| 8741 | 2 | | | | II-1 | Gm2927 | | |
| 8742 | 2 | | | | II-1 | Gm2933 | | |
| 8744 | 2 | | | | II-1 | Gm3002 | | |
| 8745 | 2 | | | | II-1 | Gm3020 | | |
| 8762 | 2 | | | | II-1 | Gm3404 | | |
| 8763 | 2 | | | | II-1 | Gm3409 | | |
| 8764 | 2 | | | | II-1 | Gm3414 | | |
| 8771 | 2 | | | | II-1 | Gm3488 | | |
| 8773 | 2 | | | | II-1 | Gm3558 | | |
| 8774 | 2 | | | | II-1 | Gm3604 | | |
| 8777 | 2 | | | | II-1 | Gm3646 | | |
| 8778 | 2 | | | | II-1 | Gm3696 | | |
| 8780 | 2 | | | | II-1 | Gm3706 | | |
| 8781 | 2 | | | | II-1 | Gm3716 | | |
| 8787 | 2 | | | | II-1 | Gm3985 | | |
| 8794 | 2 | | | | II-1 | Gm4201 | | |
| 8801 | 2 | | | | II-1 | Gm4278 | | |
| 8802 | 2 | | | | II-1 | Gm428 | | |
| 8806 | 2 | | | | II-1 | Gm4302 | | |
| 8817 | 2 | | | | II-1 | Gm4461 | | |
| 8825 | 2 | | | | II-1 | Gm4598 | | |
| 8828 | 2 | | | | II-1 | Gm4724 | | |
| 8831 | 2 | | | | II-1 | Gm4759 | | |
| 8832 | 2 | | | | II-1 | Gm4763 | | |
| 8837 | 2 | | | | II-1 | Gm4792 | | |
| 8838 | 2 | | | | II-1 | Gm4794 | | |
| 8846 | 2 | | | | II-1 | Gm4850 | | |
| 8847 | 2 | | | | II-1 | Gm4858 | | |
| 8851 | 2 | | | | II-1 | Gm4884 | | |
| 8855 | 2 | | | | II-1 | Gm4907 | | |
| 8860 | 2 | | | | II-1 | Gm4944 | | |
| 8861 | 2 | | | | II-1 | Gm4951 | | |
| 8870 | 2 | | | | II-1 | Gm5 | | |
| 8872 | 2 | | | | II-1 | Gm5065 | | |
| 8876 | 2 | | | | II-1 | Gm5082 | | |
| 8878 | 2 | | | | II-1 | Gm5084 | | |
| 8884 | 2 | | | | II-1 | Gm5095 | | |
| 8885 | 2 | | | | II-1 | Gm5105 | | |
| 8891 | 2 | | | | II-1 | Gm5124 | | |
| 8895 | 2 | | | | II-1 | Gm5132 | | |
| 8901 | 2 | | | | II-1 | Gm5150 | | |
| 8903 | 2 | | | | II-1 | Gm5168 | | |
| 8904 | 2 | | | | II-1 | Gm5169 | | |
| 8913 | 2 | | | | II-1 | Gm5347 | | |
| 8914 | 2 | | | | II-1 | Gm5382 | | |

| | | | | | | | | |
|---|---|---|---|---|---|---|---|---|
| 8915 | 2 | | | | II-1 | Gm5409 | | |
| 8918 | 2 | | | | II-1 | Gm5416 | | |
| 8919 | 2 | | | | II-1 | Gm5420 | | |
| 8925 | 2 | | | | II-1 | Gm5460 | | |
| 8927 | 2 | | | | II-1 | Gm5468 | | |
| 8928 | 2 | | | | II-1 | Gm5475 | | |
| 8929 | 2 | | | | II-1 | Gm5476 | | |
| 8930 | 2 | | | | II-1 | Gm5477 | | |
| 8935 | 2 | | | | II-1 | Gm5523 | | |
| 8945 | 2 | | | | II-1 | Gm5595 | | |
| 8949 | 2 | | | | II-1 | Gm5617 | | |
| 8954 | 2 | | | | II-1 | Gm5640 | | |
| 8960 | 2 | | | | II-1 | Gm5726 | | |
| 8961 | 2 | | | | II-1 | Gm5728 | | |
| 8969 | 2 | | | | II-1 | Gm5800 | | |
| 8970 | 2 | | | | II-1 | Gm5801 | | |
| 8971 | 2 | | | | II-1 | Gm5803 | | |
| 8974 | 2 | | | | II-1 | Gm5860 | | |
| 8978 | 2 | | | | II-1 | Gm5885 | | |
| 8979 | 2 | | | | II-1 | Gm5886 | | |
| 8980 | 2 | | | | II-1 | Gm5891 | | |
| 8986 | 2 | | | | II-1 | Gm5934 | | |
| 8987 | 2 | | | | II-1 | Gm5935 | | |
| 8988 | 2 | | | | II-1 | Gm5936 | | |
| 8991 | 2 | | | | II-1 | Gm595 | | |
| 8993 | 2 | | | | II-1 | Gm6026 | | |
| 8994 | 2 | | | | II-1 | Gm6034 | | |
| 8997 | 2 | | | | II-1 | Gm608 | | |
| 9003 | 2 | | | | II-1 | Gm614 | | |
| 9006 | 2 | | | | II-1 | Gm6194 | | |
| 9009 | 2 | | | | II-1 | Gm6249 | | |
| 9010 | 2 | | | | II-1 | Gm6251 | | |
| 9024 | 2 | | | | II-1 | Gm6406 | | |
| 9026 | 2 | | | | II-1 | Gm6416 | | |
| 9030 | 2 | | | | II-1 | Gm648 | | |
| 9034 | 2 | | | | II-1 | Gm6525 | | |
| 9037 | 2 | | | | II-1 | Gm6559 | | |
| 9038 | 2 | | | | II-1 | Gm6567 | | |
| 9043 | 2 | | | | II-1 | Gm6592 | | |
| 9044 | 2 | | | | II-1 | Gm6602 | | |
| 9045 | 2 | | | | II-1 | Gm6607 | | |
| 9048 | 2 | | | | II-1 | Gm6634 | | |
| 9049 | 2 | | | | II-1 | Gm6639 | | |
| 9057 | 2 | | | | II-1 | Gm6760 | | |
| 9058 | 2 | | | | II-1 | Gm6763 | | |
| 9059 | 2 | | | | II-1 | Gm6787 | | |
| 9062 | 2 | | | | II-1 | Gm6812 | | |
| 9063 | 2 | | | | II-1 | Gm6815 | | |
| 9067 | 2 | | | | II-1 | Gm6890 | | |
| 9069 | 2 | | | | II-1 | Gm6904 | | |
| 9072 | 2 | | | | II-1 | Gm6938 | | |
| 9077 | 2 | | | | II-1 | Gm7030 | | |
| 9080 | 2 | | | | II-1 | Gm7102 | | |
| 9083 | 2 | | | | II-1 | Gm7120 | | |
| 9088 | 2 | | | | II-1 | Gm7173 | | |
| 9092 | 2 | | | | II-1 | Gm732 | | |
| 9097 | 2 | | | | II-1 | Gm7367 | | |
| 9100 | 2 | | | | II-1 | Gm7534 | | |
| 9101 | 2 | | | | II-1 | Gm7538 | | |
| 9102 | 2 | | | | II-1 | Gm7550 | | |
| 9116 | 2 | | | | II-1 | Gm7977 | | |
| 9117 | 2 | | | | II-1 | Gm7978 | | |
| 9118 | 2 | | | | II-1 | Gm805 | | |
| 9123 | 2 | | | | II-1 | Gm8179 | | |
| 9129 | 2 | | | | II-1 | Gm8298 | | |
| 9130 | 2 | | | | II-1 | Gm8300 | | |
| 9144 | 2 | | | | II-1 | Gm8709 | | |
| 9154 | 2 | | | | II-1 | Gm8898 | | |
| 9160 | 2 | | | | II-1 | Gm904 | | |
| 9163 | 2 | | | | II-1 | Gm906 | | |
| 9166 | 2 | | | | II-1 | Gm9125 | | |
| 9167 | 2 | | | | II-1 | Gm9159 | | |
| 9168 | 2 | | | | II-1 | Gm9199 | | |
| 9175 | 2 | | | | II-1 | Gm960 | | |
| 9177 | 2 | | | | II-1 | Gm973 | | |
| 9180 | 2 | | | | II-1 | Gm9758 | | |
| 9182 | 2 | | | | II-1 | Gm9776 | | |
| 9192 | 2 | | | | II-1 | Gm9958 | | |
| 9199 | 2 | | | | II-1 | Gmcl1 | 64395 | 4-May-15 |
| 9200 | 2 | | | | II-1 | Gmcl1l | 64396 | 4-May-15 |
| 9201 | 2 | | | | II-1 | Gmds | 2762 | 4-May-15 |
| 9204 | 2 | | | | II-1 | Gmfb | 2764 | 4-May-15 |
| 9205 | 2 | | | | II-1 | Gmfg | 9535 | 12-May-15 |
| 9206 | 2 | | | | II-1 | Gmip | 51291 | 28-May-15 |
| 9215 | 2 | | | | II-1 | Gna11 | 2767 | 4-May-15 |
| 9223 | 2 | | | | II-1 | Gnal | 2774 | 12-May-15 |
| 9233 | 2 | | | | II-1 | Gnb2 | 2783 | 4-May-15 |
| 9234 | 2 | | | | II-1 | Gnb2l1 | 10399 | 31-May-15 |
| 9265 | 2 | | | | II-1 | Golga1 | 2800 | 4-May-15 |
| 9267 | 2 | | | | II-1 | Golga3 | 2802 | 4-May-15 |
| 9271 | 2 | | | | II-1 | Golga7b | 401647 | 4-May-15 |
| 9280 | 2 | | | | II-1 | Gopc | 57120 | 24-May-15 |
| 9283 | 2 | | | | II-1 | Gorasp2 | 26003 | 4-May-15 |
| 9284 | 2 | | | | II-1 | Gosr1 | 9527 | 4-May-15 |
| 9285 | 2 | | | | II-1 | Gosr2 | 9570 | 4-May-15 |
| 9298 | 2 | | | | II-1 | Gpalpp1 | 55425 | 12-May-15 |
| 9299 | 2 | | | | II-1 | Gpam | 57678 | 12-May-15 |

Fig.22 - 102

| | | | | | | | | |
|---|---|---|---|---|---|---|---|---|
| 9303 | 2 | | | | II-1 | Gpatch11 | 253635 | 4-May-15 |
| 9304 | 2 | | | | II-1 | Gpatch2 | 55105 | 4-May-15 |
| 9305 | 2 | | | | II-1 | Gpatch2l | 55668 | 12-May-15 |
| 9306 | 2 | | | | II-1 | Gpatch3 | 63906 | 4-May-15 |
| 9307 | 2 | | | | II-1 | Gpatch4 | 54865 | 4-May-15 |
| 9309 | 2 | | | | II-1 | Gpbar1 | 151306 | 4-May-15 |
| 9311 | 2 | | | | II-1 | Gpbp1l1 | 60313 | 4-May-15 |
| 9326 | 2 | | | | II-1 | Gpi1 | 9091 | 4-May-15 |
| 9330 | 2 | | | | II-1 | Gpm6a | 2823 | 4-May-15 |
| 9333 | 2 | | | | II-1 | Gpn2 | 54707 | 4-May-15 |
| 9340 | 2 | | | | II-1 | Gpr110 | 266977 | 4-May-15 |
| 9347 | 2 | | | | II-1 | Gpr12 | 2835 | 4-May-15 |
| 9353 | 2 | | | | II-1 | Gpr132 | 29933 | 4-May-15 |
| 9362 | 2 | | | | II-1 | Gpr142 | 350383 | 4-May-15 |
| 9363 | 2 | | | | II-1 | Gpr143 | 4935 | 23-May-15 |
| 9364 | 2 | | | | II-1 | Gpr146 | 115330 | 12-May-15 |
| 9367 | 2 | | | | II-1 | Gpr150 | 285601 | 4-May-15 |
| 9369 | 2 | | | | II-1 | Gpr152 | 390212 | 4-May-15 |
| 9370 | 2 | | | | II-1 | Gpr153 | 387509 | 4-May-15 |
| 9375 | 2 | | | | II-1 | Gpr160 | 26996 | 4-May-15 |
| 9377 | 2 | | | | II-1 | Gpr162 | 27239 | 4-May-15 |
| 9405 | 2 | | | | II-1 | Gpr45 | 11250 | 4-May-15 |
| 9412 | 2 | | | | II-1 | Gpr62 | 118442 | 12-May-15 |
| 9413 | 2 | | | | II-1 | Gpr63 | 81491 | 4-May-15 |
| 9425 | 2 | | | | II-1 | Gpr97 | 222487 | 12-May-15 |
| 9427 | 2 | | | | II-1 | Gprasp1 | 9737 | 4-May-15 |
| 9482 | 2 | | | | II-1 | Grifin | 402635 | 3-May-15 |
| 9485 | 2 | | | | II-1 | Grik3 | 2899 | 4-May-15 |
| 9486 | 2 | | | | II-1 | Grik4 | 2900 | 4-May-15 |
| 9487 | 2 | | | | II-1 | Grik5 | 2901 | 12-May-15 |
| 9491 | 2 | | | | II-1 | Grin2b | 2904 | 17-May-15 |
| 9498 | 2 | | | | II-1 | Grip1os2 | | |
| 9502 | 2 | | | | II-1 | Grk4 | 2868 | 12-May-15 |
| 9503 | 2 | | | | II-1 | Grk5 | 2869 | 4-May-15 |
| 9506 | 2 | | | | II-1 | Grm2 | 2912 | 12-May-15 |
| 9508 | 2 | | | | II-1 | Grm4 | 2914 | 7-Jun-15 |
| 9509 | 2 | | | | II-1 | Grm5 | 2915 | 7-Jun-15 |
| 9511 | 2 | | | | II-1 | Grm7 | 2917 | 12-May-15 |
| 9515 | 2 | | | | II-1 | Grpel1 | 80273 | 4-May-15 |
| 9523 | 2 | | | | II-1 | Grxcr2 | 643226 | 4-May-15 |
| 9524 | 2 | | | | II-1 | Gsap | 54103 | 4-May-15 |
| 9544 | 2 | | | | II-1 | Gskip | 51527 | 4-May-15 |
| 9571 | 2 | | | | II-1 | Gstz1 | 2954 | 12-May-15 |
| 9574 | 2 | | | | II-1 | Gt(ROSA)26Sor | | |
| 9576 | 2 | | | | II-1 | Gtf2a1 | 2957 | 4-May-15 |
| 9582 | 2 | | | | II-1 | Gtf2f1 | 2962 | 4-May-15 |
| 9585 | 2 | | | | II-1 | Gtf2h2 | 2966 | 4-May-15 |
| 9593 | 2 | | | | II-1 | Gtf3c1 | 2975 | 4-May-15 |
| 9595 | 2 | | | | II-1 | Gtf3c3 | 9330 | 4-May-15 |
| 9596 | 2 | | | | II-1 | Gtf3c4 | 9329 | 4-May-15 |
| 9598 | 2 | | | | II-1 | Gtf3c6 | 112495 | 4-May-15 |
| 9601 | 2 | | | | II-1 | Gtpbp10 | 85865 | 4-May-15 |
| 9606 | 2 | | | | II-1 | Gtpbp8 | 29083 | 4-May-15 |
| 9619 | 2 | | | | II-1 | Gucy2c | 2984 | 12-May-15 |
| 9621 | 2 | | | | II-1 | Gucy2e | 390226 | 4-May-15 |
| 9622 | 2 | | | | II-1 | Gucy2f | 2986 | 4-May-15 |
| 9624 | 2 | | | | II-1 | Guf1 | 60558 | 4-May-15 |
| 9645 | 2 | | | | II-1 | Gzme | | |
| 9646 | 2 | | | | II-1 | Gzmf | | |
| 9648 | 2 | | | | II-1 | Gzmk | 3003 | 4-May-15 |
| 9656 | 2 | | | | II-1 | H1fx | 8971 | 4-May-15 |
| 9684 | 2 | | | | II-1 | H2-M10.1 | | |
| 9713 | 2 | | | | II-1 | H3f3b | 3021 | 4-May-15 |
| 9724 | 2 | | | | II-1 | Hadhb | 3032 | 4-May-15 |
| 9736 | 2 | | | | II-1 | Hapln2 | 60484 | 4-May-15 |
| 9741 | 2 | | | | II-1 | Hars2 | 23438 | 7-Jun-15 |
| 9767 | 2 | | | | II-1 | Hbegf | 1839 | 12-May-15 |
| 9773 | 2 | | | | II-1 | Hcar1 | 27198 | 21-May-15 |
| 9778 | 2 | | | | II-1 | Hcfc2 | 29915 | 4-May-15 |
| 9788 | 2 | | | | II-1 | Hcst | 10870 | 7-Jun-15 |
| 9794 | 2 | | | | II-1 | Hdac4 | 9759 | 28-May-15 |
| 9798 | 2 | | | | II-1 | Hdac8 | 55869 | 28-May-15 |
| 9805 | 2 | | | | II-1 | Hdgfrp2 | 84717 | 30-May-15 |
| 9806 | 2 | | | | II-1 | Hdgfrp3 | 50810 | 4-May-15 |
| 9808 | 2 | | | | II-1 | Hdhd2 | 84064 | 12-May-15 |
| 9809 | 2 | | | | II-1 | Hdhd3 | 81932 | 12-May-15 |
| 9812 | 2 | | | | II-1 | Heatr1 | 55127 | 21-May-15 |
| 9813 | 2 | | | | II-1 | Heatr2 | 54919 | 7-Jun-15 |
| 9816 | 2 | | | | II-1 | Heatr5b | 54497 | 12-May-15 |
| 9826 | 2 | | | | II-1 | Hecw2 | 57520 | 12-May-15 |
| 9838 | 2 | | | | II-1 | Hepacam | 220296 | 23-May-15 |
| 9846 | 2 | | | | II-1 | Herc6 | 55008 | 4-May-15 |
| 9849 | 2 | | | | II-1 | Hes1 | 3280 | 7-Jun-15 |
| 9853 | 2 | | | | II-1 | Hes6 | 55502 | 24-May-15 |
| 9860 | 2 | | | | II-1 | Hexim2 | 124790 | 12-May-15 |
| 9861 | 2 | | | | II-1 | Hey1 | 23462 | 28-May-15 |
| 9868 | 2 | | | | II-1 | Hgf | 3082 | 7-Jun-15 |
| 9871 | 2 | | | | II-1 | Hgsnat | 138050 | 4-May-15 |
| 9880 | 2 | | | | II-1 | Hiatl1 | 84641 | 4-May-15 |
| 9893 | 2 | | | | II-1 | Hilpda | 29923 | 4-May-15 |
| 9896 | 2 | | | | II-1 | Hint1 | 3094 | 7-Jun-15 |
| 9906 | 2 | | | | II-1 | Hirip3 | 8479 | 4-May-15 |
| 10003 | 2 | | | | II-1 | Hmgcll1 | 54511 | 4-May-15 |
| 10013 | 2 | | | | II-1 | Hmha1 | 23526 | 4-May-15 |
| 10026 | 2 | | | | II-1 | Hnf4g | 3174 | 12-May-15 |
| 10028 | 2 | | | | II-1 | Hnrnpa0 | 10949 | 4-May-15 |
| 10032 | 2 | | | | II-1 | Hnrnpab | 3182 | 4-May-15 |
| 10033 | 2 | | | | II-1 | Hnrnpc | 3183 | 4-May-15 |
| 10034 | 2 | | | | II-1 | Hnrnpd | 3184 | 2-Jun-15 |
| 10035 | 2 | | | | II-1 | Hnrnpdl | 9987 | 4-May-15 |
| 10037 | 2 | | | | II-1 | Hnrnph1 | 3187 | 4-May-15 |
| 10038 | 2 | | | | II-1 | Hnrnph2 | 3188 | 4-May-15 |
| 10039 | 2 | | | | II-1 | Hnrnph3 | 3189 | 4-May-15 |
| 10040 | 2 | | | | II-1 | Hnrnpk | 3190 | 28-May-15 |
| 10042 | 2 | | | | II-1 | Hnrnpll | 92906 | 12-May-15 |
| 10043 | 2 | | | | II-1 | Hnrnpm | 4670 | 3-Jun-15 |
| 10044 | 2 | | | | II-1 | Hnrnpr | 10236 | 2-Jun-15 |
| 10045 | 2 | | | | II-1 | Hnrnpu | 3192 | 4-May-15 |
| 10046 | 2 | | | | II-1 | Hnrnpul1 | 11100 | 12-May-15 |
| 10047 | 2 | | | | II-1 | Hnrnpul2 | 221092 | 12-May-15 |
| 10059 | 2 | | | | II-1 | Hotair | 100124700 | 31-May-15 |
| 10060 | 2 | | | | II-1 | Hottip | 100316868 | 12-May-15 |
| 10064 | 2 | | | | II-1 | Hoxa11os | | |
| 10074 | 2 | | | | II-1 | Hoxb13 | 10481 | 31-May-15 |
| 10087 | 2 | | | | II-1 | Hoxc4 | 3221 | 4-May-15 |
| 10092 | 2 | | | | II-1 | Hoxd1 | 3231 | 12-May-15 |
| 10093 | 2 | | | | II-1 | Hoxd10 | 3236 | 12-May-15 |
| 10094 | 2 | | | | II-1 | Hoxd11 | 3237 | 12-May-15 |
| 10096 | 2 | | | | II-1 | Hoxd13 | 3239 | 4-May-15 |
| 10116 | 2 | | | | II-1 | Hps5 | 11234 | 23-May-15 |
| 10118 | 2 | | | | II-1 | Hpse | 10855 | 24-May-15 |
| 10119 | 2 | | | | II-1 | Hpse2 | 60495 | 12-May-15 |
| 10136 | 2 | | | | II-1 | Hs2st1 | 9653 | 4-May-15 |
| 10179 | 2 | | | | II-1 | Hsh2d | 84941 | 4-May-15 |
| 10193 | 2 | | | | II-1 | Hspa5 | 3309 | 31-May-15 |
| 10206 | 2 | | | | II-1 | Hspd1 | 3329 | 17-May-15 |
| 10213 | 2 | | | | II-1 | Htr1b | 3351 | 24-May-15 |
| 10216 | 2 | | | | II-1 | Htr2a | 3356 | 7-Jun-15 |
| 10217 | 2 | | | | II-1 | Htr2b | 3357 | 12-May-15 |
| 10222 | 2 | | | | II-1 | Htr5a | 3361 | 12-May-15 |
| 10225 | 2 | | | | II-1 | Htr7 | 3363 | 12-May-15 |
| 10234 | 2 | | | | II-1 | Huwe1 | 10075 | 12-May-15 |
| 10239 | 2 | | | | II-1 | Hyal4 | 23553 | 4-May-15 |
| 10241 | 2 | | | | II-1 | Hyal6 | 26062 | 12-May-15 |
| 10242 | 2 | | | | II-1 | Hydin | 54768 | 23-May-15 |
| 10250 | 2 | | | | II-1 | I830012O16Rik | | |
| 10256 | 2 | | | | II-1 | Iba57 | 200205 | 4-May-15 |
| 10259 | 2 | | | | II-1 | Ica1 | 3382 | 12-May-15 |
| 10260 | 2 | | | | II-1 | Ica1l | 130026 | 4-May-15 |
| 10261 | 2 | | | | II-1 | Icam1 | 3383 | 17-May-15 |
| 10268 | 2 | | | | II-1 | Icosl | 23308 | 17-May-15 |
| 10279 | 2 | | | | II-1 | Idh3g | 3421 | 4-May-15 |
| 10287 | 2 | | | | II-1 | Ier2 | 9592 | 21-May-15 |
| 10296 | 2 | | | | II-1 | Ifi204 | | |
| 10303 | 2 | | | | II-1 | Ifi44 | 10561 | 4-May-15 |
| 10319 | 2 | | | | II-1 | Ifna11 | | |
| 10320 | 2 | | | | II-1 | Ifna12 | | |
| 10334 | 2 | | | | II-1 | Ifnb1 | 3456 | 31-May-15 |
| 10336 | 2 | | | | II-1 | Ifng | 3458 | 31-May-15 |
| 10341 | 2 | | | | II-1 | Ifnl3 | 282617 | 7-Jun-15 |
| 10349 | 2 | | | | II-1 | Ift20 | 90410 | 29-May-15 |
| 10358 | 2 | | | | II-1 | Ift81 | 28981 | 4-May-15 |
| 10360 | 2 | | | | II-1 | Igbp1 | 3476 | 4-May-15 |
| 10362 | 2 | | | | II-1 | Igdcc3 | 9543 | 4-May-15 |
| 10369 | 2 | | | | II-1 | Igf2bp3 | 10643 | 4-May-15 |
| 10372 | 2 | | | | II-1 | Igfals | 3483 | 12-May-15 |
| 10407 | 2 | | | | II-1 | Ikbke | 9641 | 12-May-15 |
| 10408 | 2 | | | | II-1 | Ikbkg | 8517 | 23-May-15 |
| 10414 | 2 | | | | II-1 | Il10 | 3586 | 31-May-15 |
| 10418 | 2 | | | | II-1 | Il11ra1 | | |
| 10432 | 2 | | | | II-1 | Il17c | 27189 | 12-May-15 |
| 10436 | 2 | | | | II-1 | Il17rb | 55540 | 4-May-15 |
| 10439 | 2 | | | | II-1 | Il17re | 132014 | 12-May-15 |
| 10445 | 2 | | | | II-1 | Il1a | 3552 | 31-May-15 |
| 10457 | 2 | | | | II-1 | Il1rapl2 | 26280 | 12-May-15 |
| 10463 | 2 | | | | II-1 | Il20ra | 53832 | 12-May-15 |
| 10474 | 2 | | | | II-1 | Il27 | 246778 | 4-May-15 |
| 10481 | 2 | | | | II-1 | Il31ra | 133396 | 7-Jun-15 |
| 10501 | 2 | | | | II-1 | Ilk | 3611 | 4-May-15 |
| 10504 | 2 | | | | II-1 | Ilvbl | 10994 | 4-May-15 |
| 10509 | 2 | | | | II-1 | Imp4 | 92856 | 7-Jun-15 |
| 10510 | 2 | | | | II-1 | Impa1 | 3612 | 12-May-15 |
| 10517 | 2 | | | | II-1 | Impg2 | 50939 | 23-May-15 |
| 10524 | 2 | | | | II-1 | Ing2 | 3622 | 7-Jun-15 |
| 10527 | 2 | | | | II-1 | Ing5 | 84289 | 4-May-15 |
| 10537 | 2 | | | | II-1 | Ino80c | 125476 | 12-May-15 |
| 10541 | 2 | | | | II-1 | Inpp1 | 3628 | 12-May-15 |
| 10544 | 2 | | | | II-1 | Inpp5a | 3632 | 4-May-15 |
| 10549 | 2 | | | | II-1 | Inpp5j | 27124 | 4-May-15 |
| 10567 | 2 | | | | II-1 | Ints2 | 57508 | 4-May-15 |
| 10568 | 2 | | | | II-1 | Ints3 | 65123 | 12-May-15 |
| 10570 | 2 | | | | II-1 | Ints5 | 80789 | 4-May-15 |
| 10575 | 2 | | | | II-1 | Intu | 27152 | 12-May-15 |
| 10576 | 2 | | | | II-1 | Invs | 27130 | 14-May-15 |
| 10583 | 2 | | | | II-1 | Ipo13 | 9670 | 4-May-15 |
| 10586 | 2 | | | | II-1 | Ipo7 | 10527 | 4-May-15 |
| 10588 | 2 | | | | II-1 | Ipo9 | 55705 | 4-May-15 |
| 10589 | 2 | | | | II-1 | Ipp | 3652 | 4-May-15 |
| 10590 | 2 | | | | II-1 | Ippk | 64768 | 28-May-15 |
| 10592 | 2 | | | | II-1 | Iqca | 79781 | 4-May-15 |
| 10593 | 2 | | | | II-1 | Iqcb1 | 9657 | 4-May-15 |
| 10597 | 2 | | | | II-1 | Iqcf1 | 132141 | 4-May-15 |
| 10598 | 2 | | | | II-1 | Iqcf3 | 401067 | 4-May-15 |

Fig.22 - 103

| | | | | | | | | |
|---|---|---|---|---|---|---|---|---|
| 10600 | 2 | | | | II-1 | Iqcf5 | 389124 | 4-May-15 |
| 10601 | 2 | | | | II-1 | Iqcf6 | 440956 | 4-May-15 |
| 10614 | 2 | | | | II-1 | Irak1bp1 | 134728 | 4-May-15 |
| 10618 | 2 | | | | II-1 | Ireb2 | 3658 | 12-May-15 |
| 10619 | 2 | | | | II-1 | Irf1 | 3659 | 12-May-15 |
| 10667 | 2 | | | | II-1 | Itfg1 | 81533 | 12-May-15 |
| 10668 | 2 | | | | II-1 | Itfg2 | 55846 | 4-May-15 |
| 10715 | 2 | | | | II-1 | Itpkc | 80271 | 31-May-15 |
| 10719 | 2 | | | | II-1 | Itprip | 85450 | 12-May-15 |
| 10731 | 2 | | | | II-1 | Izumo3 | 100129669 | 4-May-15 |
| 10732 | 2 | | | | II-1 | Izumo4 | 113177 | 4-May-15 |
| 10740 | 2 | | | | II-1 | Jak2 | 3717 | 7-Jun-15 |
| 10741 | 2 | | | | II-1 | Jak3 | 3718 | 10-May-15 |
| 10745 | 2 | | | | II-1 | Jam2 | 58494 | 7-Jun-15 |
| 10747 | 2 | | | | II-1 | Jarid2 | 3720 | 31-May-15 |
| 10752 | 2 | | | | II-1 | Jmjd4 | 65094 | 12-May-15 |
| 10757 | 2 | | | | II-1 | Jmy | 133746 | 4-May-15 |
| 10758 | 2 | | | | II-1 | Josd1 | 9929 | 12-May-15 |
| 10759 | 2 | | | | II-1 | Josd2 | 126119 | 4-May-15 |
| 10773 | 2 | | | | II-1 | Kalrn | 8997 | 4-May-15 |
| 10778 | 2 | | | | II-1 | Kank4os | | |
| 10786 | 2 | | | | II-1 | Kat2b | 8850 | 4-May-15 |
| 10787 | 2 | | | | II-1 | Kat5 | 10524 | 31-May-15 |
| 10788 | 2 | | | | II-1 | Kat6a | 7994 | 3-Jun-15 |
| 10795 | 2 | | | | II-1 | Katnb1 | 10300 | 12-May-15 |
| 10806 | 2 | | | | II-1 | Kbtbd8 | 84541 | 4-May-15 |
| 10811 | 2 | | | | II-1 | Kcna3 | 3738 | 4-May-15 |
| 10818 | 2 | | | | II-1 | Kcnab3 | 9196 | 4-May-15 |
| 10843 | 2 | | | | II-1 | Kcnh5 | 27133 | 2-Jun-15 |
| 10844 | 2 | | | | II-1 | Kcnh6 | 81033 | 4-May-15 |
| 10870 | 2 | | | | II-1 | Kcnk15 | 60598 | 4-May-15 |
| 10871 | 2 | | | | II-1 | Kcnk16 | 83795 | 4-May-15 |
| 10902 | 2 | | | | II-1 | Kcnu1 | 157855 | 4-May-15 |
| 10903 | 2 | | | | II-1 | Kcnv1 | 27012 | 1-Jun-15 |
| 10918 | 2 | | | | II-1 | Kctd2 | 23510 | 4-May-15 |
| 10920 | 2 | | | | II-1 | Kctd21 | 283219 | 4-May-15 |
| 10921 | 2 | | | | II-1 | Kctd3 | 51133 | 12-May-15 |
| 10924 | 2 | | | | II-1 | Kctd6 | 200845 | 2-Jun-15 |
| 10925 | 2 | | | | II-1 | Kctd7 | 154881 | 4-May-15 |
| 10926 | 2 | | | | II-1 | Kctd8 | 386617 | 4-May-15 |
| 10930 | 2 | | | | II-1 | Kdelr1 | 10945 | 4-May-15 |
| 10931 | 2 | | | | II-1 | Kdelr2 | 11014 | 4-May-15 |
| 10932 | 2 | | | | II-1 | Kdelr3 | 11015 | 4-May-15 |
| 10936 | 2 | | | | II-1 | Kdm2a | 22992 | 17-May-15 |
| 10937 | 2 | | | | II-1 | Kdm2b | 84678 | 17-May-15 |
| 10938 | 2 | | | | II-1 | Kdm3a | 55818 | 3-May-15 |
| 10940 | 2 | | | | II-1 | Kdm4a | 9682 | 12-May-15 |
| 10941 | 2 | | | | II-1 | Kdm4b | 23030 | 24-May-15 |
| 10942 | 2 | | | | II-1 | Kdm4c | 23081 | 17-May-15 |
| 10943 | 2 | | | | II-1 | Kdm4d | 55693 | 4-May-15 |
| 10944 | 2 | | | | II-1 | Kdm5a | 5927 | 4-May-15 |
| 10945 | 2 | | | | II-1 | Kdm5b | 10765 | 12-May-15 |
| 10948 | 2 | | | | II-1 | Kdm6a | 7403 | 23-May-15 |
| 10955 | 2 | | | | II-1 | Keg1 | | |
| 10963 | 2 | | | | II-1 | Khdrbs2 | 202559 | 12-May-15 |
| 10969 | 2 | | | | II-1 | Kif11 | 3832 | 4-May-15 |
| 10981 | 2 | | | | II-1 | Kif1b | 23095 | 23-May-15 |
| 10994 | 2 | | | | II-1 | Kif2b | 84643 | 12-May-15 |
| 10998 | 2 | | | | II-1 | Kif3c | 3797 | 4-May-15 |
| 11011 | 2 | | | | II-1 | Kifc5b | | |
| 11012 | 2 | | | | II-1 | Kin | 22944 | 4-May-15 |
| 11015 | 2 | | | | II-1 | Kirrel | 55243 | 4-May-15 |
| 11027 | 2 | | | | II-1 | Klc2 | 64837 | 7-Jun-15 |
| 11066 | 2 | | | | II-1 | Klhl2 | 11275 | 4-May-15 |
| 11074 | 2 | | | | II-1 | Klhl28 | 54813 | 12-May-15 |
| 11093 | 2 | | | | II-1 | Klhl9 | 55958 | 4-May-15 |
| 11101 | 2 | | | | II-1 | Klk1b1 | | |
| 11122 | 2 | | | | II-1 | Klra1 | 10748 | 12-May-15 |
| 11123 | 2 | | | | II-1 | Klra10 | | |
| 11138 | 2 | | | | II-1 | Klra5 | | |
| 11151 | 2 | | | | II-1 | Klrc3 | 3823 | 12-May-15 |
| 11152 | 2 | | | | II-1 | Klrd1 | 3824 | 12-May-15 |
| 11158 | 2 | | | | II-1 | Klrk1 | 22914 | 24-May-15 |
| 11170 | 2 | | | | II-1 | Knstrn | 90417 | 4-May-15 |
| 11176 | 2 | | | | II-1 | Kpna6 | 23633 | 4-May-15 |
| 11177 | 2 | | | | II-1 | Kpna7 | 402569 | 7-Jun-15 |
| 11178 | 2 | | | | II-1 | Kpnb1 | 3837 | 4-May-15 |
| 11181 | 2 | | | | II-1 | Kras | 3845 | 7-Jun-15 |
| 11185 | 2 | | | | II-1 | Kremen2 | 79412 | 4-May-15 |
| 11188 | 2 | | | | II-1 | Krr1 | 11103 | 4-May-15 |
| 11205 | 2 | | | | II-1 | Krt26 | 353288 | 4-May-15 |
| 11300 | 2 | | | | II-1 | Kxd1 | 79036 | 4-May-15 |
| 11305 | 2 | | | | II-1 | L2hgdh | 79944 | 4-May-15 |
| 11309 | 2 | | | | II-1 | L3mbtl3 | 84456 | 4-May-15 |
| 11323 | 2 | | | | II-1 | Lama2 | 3908 | 23-May-15 |
| 11335 | 2 | | | | II-1 | Lamp3 | 27074 | 7-Jun-15 |
| 11336 | 2 | | | | II-1 | Lamp5 | 24141 | 4-May-15 |
| 11338 | 2 | | | | II-1 | Lamtor2 | 28956 | 4-May-15 |
| 11342 | 2 | | | | II-1 | Lancl1 | 10314 | 12-May-15 |
| 11361 | 2 | | | | II-1 | Lat | 27040 | 7-Jun-15 |
| 11373 | 2 | | | | II-1 | Lca5l | 150082 | 12-May-15 |
| 11427 | 2 | | | | II-1 | Ldlrad3 | 143458 | 12-May-15 |
| 11431 | 2 | | | | II-1 | Ldoc1l | 84247 | 4-May-15 |
| 11441 | 2 | | | | II-1 | Lemd2 | 221496 | 29-May-15 |
| 11442 | 2 | | | | II-1 | Lemd3 | 23592 | 12-May-15 |
| 11443 | 2 | | | | II-1 | Lenep | 55891 | 4-May-15 |
| 11446 | 2 | | | | II-1 | Leng9 | 94059 | 28-May-15 |
| 11455 | 2 | | | | II-1 | Leprotl1 | 23484 | 4-May-15 |
| 11456 | 2 | | | | II-1 | Letm1 | 3954 | 17-May-15 |
| 11457 | 2 | | | | II-1 | Letm2 | 137994 | 4-May-15 |
| 11472 | 2 | | | | II-1 | Lgi2 | 55203 | 12-May-15 |
| 11478 | 2 | | | | II-1 | Lgr6 | 59352 | 4-May-15 |
| 11480 | 2 | | | | II-1 | Lhb | 3972 | 12-May-15 |
| 11487 | 2 | | | | II-1 | Lhfpl5 | 222662 | 23-May-15 |
| 11494 | 2 | | | | II-1 | Lhx5 | 64211 | 4-May-15 |
| 11497 | 2 | | | | II-1 | Lhx9 | 56956 | 12-May-15 |
| 11498 | 2 | | | | II-1 | Lias | 11019 | 4-May-15 |
| 11514 | 2 | | | | II-1 | Limk2 | 3985 | 12-May-15 |
| 11519 | 2 | | | | II-1 | Lin37 | 55957 | 4-May-15 |
| 11522 | 2 | | | | II-1 | Lin7a | 8825 | 4-May-15 |
| 11540 | 2 | | | | II-1 | Lipm | 340654 | 4-May-15 |
| 11541 | 2 | | | | II-1 | Lipn | 643418 | 4-May-15 |
| 11552 | 2 | | | | II-1 | Lman1 | 3998 | 12-May-15 |
| 11553 | 2 | | | | II-1 | Lman1l | 79748 | 4-May-15 |
| 11556 | 2 | | | | II-1 | Lmbr1 | 64327 | 4-May-15 |
| 11558 | 2 | | | | II-1 | Lmbrd1 | 55788 | 23-May-15 |
| 11563 | 2 | | | | II-1 | Lmln | 89782 | 4-May-15 |
| 11579 | 2 | | | | II-1 | Lnp | 80856 | 13-Jun-15 |
| 11599 | 2 | | | | II-1 | LOC101055769 | | |
| 11604 | 2 | | | | II-1 | LOC101056236 | | |
| 11608 | 2 | | | | II-1 | LOC102631757 | | |
| 11611 | 2 | | | | II-1 | LOC102633035 | | |
| 11612 | 2 | | | | II-1 | LOC102633315 | | |
| 11615 | 2 | | | | II-1 | LOC102634431 | | |
| 11616 | 2 | | | | II-1 | LOC102634753 | | |
| 11617 | 2 | | | | II-1 | LOC102635087 | | |
| 11621 | 2 | | | | II-1 | LOC381967 | | |
| 11622 | 2 | | | | II-1 | LOC666331 | | |
| 11626 | 2 | | | | II-1 | Lonrf1 | 91694 | 4-May-15 |
| 11645 | 2 | | | | II-1 | Lpcat3 | 10162 | 12-May-15 |
| 11661 | 2 | | | | II-1 | Lrch2 | 57631 | 4-May-15 |
| 11662 | 2 | | | | II-1 | Lrch3 | 84859 | 4-May-15 |
| 11668 | 2 | | | | II-1 | Lrfn4 | 78999 | 4-May-15 |
| 11673 | 2 | | | | II-1 | Lrig1 | 26018 | 4-May-15 |
| 11682 | 2 | | | | II-1 | Lrp11 | 84918 | 29-May-15 |
| 11683 | 2 | | | | II-1 | Lrp12 | 29967 | 12-May-15 |
| 11686 | 2 | | | | II-1 | Lrp2bp | 55805 | 7-Jun-15 |
| 11692 | 2 | | | | II-1 | Lrpap1 | 4043 | 12-May-15 |
| 11727 | 2 | | | | II-1 | Lrrc43 | 254050 | 4-May-15 |
| 11731 | 2 | | | | II-1 | Lrrc48 | 83450 | 4-May-15 |
| 11742 | 2 | | | | II-1 | Lrrc6 | 23639 | 7-Jun-15 |
| 11744 | 2 | | | | II-1 | Lrrc63 | 220416 | 4-May-15 |
| 11745 | 2 | | | | II-1 | Lrrc66 | 339977 | 4-May-15 |
| 11746 | 2 | | | | II-1 | Lrrc69 | 100130742 | 12-May-15 |
| 11748 | 2 | | | | II-1 | Lrrc71 | 149499 | 4-May-15 |
| 11750 | 2 | | | | II-1 | Lrrc73 | 221424 | 2-Jun-15 |
| 11752 | 2 | | | | II-1 | Lrrc75a | 388341 | 4-May-15 |
| 11760 | 2 | | | | II-1 | Lrrcc1 | 85444 | 4-May-15 |
| 11761 | 2 | | | | II-1 | Lrrd1 | 401387 | 4-May-15 |
| 11763 | 2 | | | | II-1 | Lrrfip2 | 9209 | 4-May-15 |
| 11765 | 2 | | | | II-1 | Lrriq3 | 127255 | 4-May-15 |
| 11766 | 2 | | | | II-1 | Lrriq4 | 344657 | 4-May-15 |
| 11767 | 2 | | | | II-1 | Lrrk1 | 79705 | 4-May-15 |
| 11776 | 2 | | | | II-1 | Lrrtm3 | 347731 | 1-Jun-15 |
| 11778 | 2 | | | | II-1 | Lrsam1 | 90678 | 7-Jun-15 |
| 11783 | 2 | | | | II-1 | Lsg1 | 55341 | 23-May-15 |
| 11785 | 2 | | | | II-1 | Lsm10 | 84967 | 4-May-15 |
| 11789 | 2 | | | | II-1 | Lsm14b | 149986 | 4-May-15 |
| 11790 | 2 | | | | II-1 | Lsm2 | 57819 | 4-May-15 |
| 11819 | 2 | | | | II-1 | Luc7l3 | 51747 | 4-May-15 |
| 11820 | 2 | | | | II-1 | Lum | 4060 | 12-May-15 |
| 11856 | 2 | | | | II-1 | Lypd5 | 284348 | 14-May-15 |
| 11880 | 2 | | | | II-1 | Lyzl4os | | |
| 11881 | 2 | | | | II-1 | Lyzl6 | 57151 | 4-May-15 |
| 11882 | 2 | | | | II-1 | Lzic | 84328 | 12-May-15 |
| 11883 | 2 | | | | II-1 | Lztfl1 | 54585 | 4-May-15 |
| 11884 | 2 | | | | II-1 | Lztr1 | 8216 | 12-May-15 |
| 11885 | 2 | | | | II-1 | Lzts1 | 11178 | 4-May-15 |
| 11889 | 2 | | | | II-1 | M6pr | 4074 | 7-Jun-15 |
| 11891 | 2 | | | | II-1 | Mab21l1 | 4081 | 4-May-15 |
| 11892 | 2 | | | | II-1 | Mab21l2 | 10586 | 4-May-15 |
| 11914 | 2 | | | | II-1 | Magea1 | 4100 | 4-May-15 |
| 11915 | 2 | | | | II-1 | Magea10 | 4109 | 4-May-15 |
| 11916 | 2 | | | | II-1 | Magea2 | 4101 | 7-Jun-15 |
| 11918 | 2 | | | | II-1 | Magea4 | 4103 | 7-Jun-15 |
| 11920 | 2 | | | | II-1 | Magea6 | 4105 | 7-Jun-15 |
| 11921 | 2 | | | | II-1 | Magea8 | 4107 | 12-May-15 |
| 11922 | 2 | | | | II-1 | Mageb1 | 4112 | 12-May-15 |
| 11924 | 2 | | | | II-1 | Mageb16-ps1 | | |
| 11928 | 2 | | | | II-1 | Mageb4 | 4115 | 4-May-15 |
| 11929 | 2 | | | | II-1 | Mageb5 | 347541 | 4-May-15 |
| 11930 | 2 | | | | II-1 | Maged1 | 9500 | 4-May-15 |
| 11944 | 2 | | | | II-1 | Mak16 | 84549 | 4-May-15 |
| 11954 | 2 | | | | II-1 | Maml2 | 84441 | 4-May-15 |
| 11961 | 2 | | | | II-1 | Man1c1 | 57134 | 4-May-15 |
| 11964 | 2 | | | | II-1 | Man2b1 | 4125 | 23-May-15 |
| 11965 | 2 | | | | II-1 | Man2b2 | 23324 | 12-May-15 |
| 11970 | 2 | | | | II-1 | Manea | 79694 | 4-May-15 |
| 11971 | 2 | | | | II-1 | Maneal | 149175 | 4-May-15 |
| 11984 | 2 | | | | II-1 | Map2 | 4133 | 7-Jun-15 |
| 11987 | 2 | | | | II-1 | Map2k3 | 5606 | 4-May-15 |
| 11991 | 2 | | | | II-1 | Map2k6 | 5608 | 4-May-15 |
| 11994 | 2 | | | | II-1 | Map3k10 | 4294 | 4-May-15 |
| 11996 | 2 | | | | II-1 | Map3k12 | 7786 | 28-May-15 |

Fig.22 - 104

| | | | | | | | | |
|---|---|---|---|---|---|---|---|---|
| 12001 | 2 | | | | II-1 | Map3k2 | 10746 | 4-May-15 |
| 12002 | 2 | | | | II-1 | Map3k3 | 4215 | 24-May-15 |
| 12003 | 2 | | | | II-1 | Map3k4 | 4216 | 4-May-15 |
| 12004 | 2 | | | | II-1 | Map3k5 | 4217 | 7-Jun-15 |
| 12013 | 2 | | | | II-1 | Map4k3 | 8491 | 12-May-15 |
| 12014 | 2 | | | | II-1 | Map4k4 | 9448 | 4-May-15 |
| 12022 | 2 | | | | II-1 | Mapk1 | 5594 | 7-Jun-15 |
| 12023 | 2 | | | | II-1 | Mapk10 | 5602 | 3-May-15 |
| 12024 | 2 | | | | II-1 | Mapk11 | 5600 | 4-May-15 |
| 12031 | 2 | | | | II-1 | Mapk3 | 5595 | 7-Jun-15 |
| 12035 | 2 | | | | II-1 | Mapk8 | 5599 | 7-Jun-15 |
| 12036 | 2 | | | | II-1 | Mapk8ip1 | 9479 | 4-May-15 |
| 12040 | 2 | | | | II-1 | Mapkap1 | 79109 | 2-Jun-15 |
| 12041 | 2 | | | | II-1 | Mapkapk2 | 9261 | 3-May-15 |
| 12042 | 2 | | | | II-1 | Mapkapk3 | 7867 | 4-May-15 |
| 12046 | 2 | | | | II-1 | Mapre2 | 10982 | 12-May-15 |
| 12054 | 2 | | | | II-1 | March2 | 51257 | 4-May-15 |
| 12058 | 2 | | | | II-1 | March6 | 10299 | 4-May-15 |
| 12059 | 2 | | | | II-1 | March7 | 64844 | 23-May-15 |
| 12069 | 2 | | | | II-1 | Mark3 | 4140 | 21-May-15 |
| 12070 | 2 | | | | II-1 | Mark4 | 57787 | 4-May-15 |
| 12073 | 2 | | | | II-1 | Marveld1 | 83742 | 4-May-15 |
| 12090 | 2 | | | | II-1 | Matn3 | 4148 | 23-May-15 |
| 12094 | 2 | | | | II-1 | Mavs | 57506 | 24-May-15 |
| 12095 | 2 | | | | II-1 | Max | 4149 | 22-May-15 |
| 12096 | 2 | | | | II-1 | Maz | 4150 | 4-May-15 |
| 12102 | 2 | | | | II-1 | Mbd3 | 53615 | 7-Jun-15 |
| 12103 | 2 | | | | II-1 | Mbd3l1 | 85509 | 4-May-15 |
| 12104 | 2 | | | | II-1 | Mbd3l2 | 125997 | 21-May-15 |
| 12105 | 2 | | | | II-1 | Mbd4 | 8930 | 12-May-15 |
| 12106 | 2 | | | | II-1 | Mbd5 | 55777 | 31-May-15 |
| 12108 | 2 | | | | II-1 | Mbip | 51562 | 12-May-15 |
| 12114 | 2 | | | | II-1 | Mbnl2 | 10150 | 12-May-15 |
| 12120 | 2 | | | | II-1 | Mbp | 4155 | 7-Jun-15 |
| 12122 | 2 | | | | II-1 | Mbtps1 | 8720 | 17-May-15 |
| 12127 | 2 | | | | II-1 | Mc4r | 4160 | 31-May-15 |
| 12130 | 2 | | | | II-1 | Mcat | 27349 | 4-May-15 |
| 12135 | 2 | | | | II-1 | Mcee | 84693 | 23-May-15 |
| 12138 | 2 | | | | II-1 | Mcf2l | 23263 | 4-May-15 |
| 12142 | 2 | | | | II-1 | Mcl1 | 4170 | 7-Jun-15 |
| 12155 | 2 | | | | II-1 | Mcoln1 | 57192 | 7-Jun-15 |
| 12165 | 2 | | | | II-1 | Mcrs1 | 10445 | 12-May-15 |
| 12171 | 2 | | | | II-1 | Mcur1 | 63933 | 4-May-15 |
| 12172 | 2 | | | | II-1 | Mdc1 | 9656 | 31-May-15 |
| 12173 | 2 | | | | II-1 | Mdfi | 4188 | 12-May-15 |
| 12190 | 2 | | | | II-1 | Meaf6 | 64769 | 2-Jun-15 |
| 12191 | 2 | | | | II-1 | Mecom | 2122 | 4-May-15 |
| 12195 | 2 | | | | II-1 | Med10 | 84246 | 4-May-15 |
| 12197 | 2 | | | | II-1 | Med12 | 9968 | 24-May-15 |
| 12199 | 2 | | | | II-1 | Med13 | 9969 | 4-May-15 |
| 12202 | 2 | | | | II-1 | Med15 | 51586 | 12-May-15 |
| 12204 | 2 | | | | II-1 | Med17 | 9440 | 4-May-15 |
| 12207 | 2 | | | | II-1 | Med20 | 9477 | 2-Jun-15 |
| 12208 | 2 | | | | II-1 | Med21 | 9412 | 4-May-15 |
| 12211 | 2 | | | | II-1 | Med24 | 9862 | 7-Jun-15 |
| 12212 | 2 | | | | II-1 | Med25 | 81857 | 7-Jun-15 |
| 12213 | 2 | | | | II-1 | Med26 | 9441 | 4-May-15 |
| 12217 | 2 | | | | II-1 | Med30 | 90390 | 4-May-15 |
| 12239 | 2 | | | | II-1 | Meig1 | 644890 | 4-May-15 |
| 12253 | 2 | | | | II-1 | Mertk | 10461 | 23-May-15 |
| 12255 | 2 | | | | II-1 | Mesdc2 | 23184 | 4-May-15 |
| 12262 | 2 | | | | II-1 | Metap2 | 10988 | 12-May-15 |
| 12281 | 2 | | | | II-1 | Mettl24 | 728464 | 4-May-15 |
| 12287 | 2 | | | | II-1 | Mettl7a1 | | |
| 12294 | 2 | | | | II-1 | Mex3a | 92312 | 4-May-15 |
| 12328 | 2 | | | | II-1 | Mfsd9 | 84804 | 4-May-15 |
| 12333 | 2 | | | | II-1 | Mgat2 | 4247 | 23-May-15 |
| 12364 | 2 | | | | II-1 | Micu3 | 286097 | 4-May-15 |
| 12369 | 2 | | | | II-1 | Mief1 | 54471 | 12-May-15 |
| 12370 | 2 | | | | II-1 | Mief2 | 125170 | 12-May-15 |
| 12373 | 2 | | | | II-1 | Mier2 | 54531 | 4-May-15 |
| 12375 | 2 | | | | II-1 | Mif | 4282 | 7-Jun-15 |
| 12377 | 2 | | | | II-1 | Miip | 60672 | 4-May-15 |
| 12384 | 2 | | | | II-1 | Minpp1 | 9562 | 4-May-15 |
| 12386 | 2 | | | | II-1 | Miox | 55586 | 4-May-15 |
| 12392 | 2 | | | | II-1 | Mir101b | | |
| 12437 | 2 | | | | II-1 | Mir1306 | 100302197 | 21-May-15 |
| 12446 | 2 | | | | II-1 | Mir134 | 406924 | 21-May-15 |
| 12463 | 2 | | | | II-1 | Mir145b | | |
| 12484 | 2 | | | | II-1 | Mir181a-2 | | |
| 12556 | 2 | | | | II-1 | Mir1960 | | |
| 12607 | 2 | | | | II-1 | Mir216c | | |
| 12624 | 2 | | | | II-1 | Mir24-1 | 407012 | 21-May-15 |
| 12964 | 2 | | | | II-1 | Mir6239 | | |
| 12975 | 2 | | | | II-1 | Mir6343 | | |
| 12995 | 2 | | | | II-1 | Mir6366 | | |
| 13014 | 2 | | | | II-1 | Mir6388 | | |
| 13021 | 2 | | | | II-1 | Mir6395 | | |
| 13025 | 2 | | | | II-1 | Mir6399 | | |
| 13032 | 2 | | | | II-1 | Mir6406 | | |
| 13047 | 2 | | | | II-1 | Mir6481 | | |
| 13096 | 2 | | | | II-1 | Mir684-2 | | |
| 13097 | 2 | | | | II-1 | Mir686 | | |
| 13129 | 2 | | | | II-1 | Mir6922 | | |
| 13183 | 2 | | | | II-1 | Mir6972 | | |
| 13208 | 2 | | | | II-1 | Mir6995 | | |
| 13214 | 2 | | | | II-1 | Mir7000 | | |
| 13249 | 2 | | | | II-1 | Mir7032 | | |
| 13334 | 2 | | | | II-1 | Mir7211 | | |
| 13378 | 2 | | | | II-1 | Mir764 | 100313838 | 4-May-15 |
| 13437 | 2 | | | | II-1 | Mir8107 | | |
| 13441 | 2 | | | | II-1 | Mir8111 | | |
| 13451 | 2 | | | | II-1 | Mir872 | | |
| 13479 | 2 | | | | II-1 | Mirlet7b | 406884 | 21-May-15 |
| 13486 | 2 | | | | II-1 | Mirlet7f-2 | | |
| 13496 | 2 | | | | II-1 | Mitf | 4286 | 31-May-15 |
| 13498 | 2 | | | | II-1 | Mki67 | 4288 | 24-May-15 |
| 13509 | 2 | | | | II-1 | Mks1 | 54903 | 23-May-15 |
| 13517 | 2 | | | | II-1 | Mlh3 | 27030 | 4-May-15 |
| 13522 | 2 | | | | II-1 | Mllt11 | 10962 | 4-May-15 |
| 13523 | 2 | | | | II-1 | Mllt3 | 4300 | 12-May-15 |
| 13528 | 2 | | | | II-1 | Mlx | 6945 | 4-May-15 |
| 13529 | 2 | | | | II-1 | Mlxip | 22877 | 4-May-15 |
| 13533 | 2 | | | | II-1 | Mmab | 326625 | 23-May-15 |
| 13536 | 2 | | | | II-1 | Mmd | 23531 | 4-May-15 |
| 13550 | 2 | | | | II-1 | Mmp19 | 4327 | 12-May-15 |
| 13553 | 2 | | | | II-1 | Mmp2 | 4313 | 24-May-15 |
| 13556 | 2 | | | | II-1 | Mmp23 | | |
| 13583 | 2 | | | | II-1 | Mob3b | 79817 | 4-May-15 |
| 13587 | 2 | | | | II-1 | Mocos | 55034 | 4-May-15 |
| 13596 | 2 | | | | II-1 | Mon1a | 84315 | 4-May-15 |
| 13598 | 2 | | | | II-1 | Mon2 | 23041 | 4-May-15 |
| 13599 | 2 | | | | II-1 | Morc1 | 27136 | 4-May-15 |
| 13600 | 2 | | | | II-1 | Morc2a | | |
| 13612 | 2 | | | | II-1 | Mospd1 | 56180 | 12-May-15 |
| 13616 | 2 | | | | II-1 | Mov10 | 4343 | 4-May-15 |
| 13642 | 2 | | | | II-1 | Mppe1 | 65258 | 12-May-15 |
| 13667 | 2 | | | | II-1 | Mrgpra2a | | |
| 13668 | 2 | | | | II-1 | Mrgpra2b | | |
| 13670 | 2 | | | | II-1 | Mrgpra4 | | |
| 13671 | 2 | | | | II-1 | Mrgpra6 | | |
| 13677 | 2 | | | | II-1 | Mrgprb5 | | |
| 13678 | 2 | | | | II-1 | Mrgprb8 | | |
| 13684 | 2 | | | | II-1 | Mrgprx1 | 259249 | 4-May-15 |
| 13686 | 2 | | | | II-1 | Mri1 | 84245 | 4-May-15 |
| 13691 | 2 | | | | II-1 | Mroh2b | 133558 | 4-May-15 |
| 13692 | 2 | | | | II-1 | Mroh4 | | |
| 13693 | 2 | | | | II-1 | Mroh5 | 389690 | 4-May-15 |
| 13695 | 2 | | | | II-1 | Mroh7 | 374977 | 4-May-15 |
| 13697 | 2 | | | | II-1 | Mroh9 | 80133 | 4-May-15 |
| 13698 | 2 | | | | II-1 | Mrpl1 | 65008 | 4-May-15 |
| 13702 | 2 | | | | II-1 | Mrpl13 | 28998 | 21-May-15 |
| 13706 | 2 | | | | II-1 | Mrpl17 | 63875 | 4-May-15 |
| 13707 | 2 | | | | II-1 | Mrpl18 | 29074 | 7-Jun-15 |
| 13708 | 2 | | | | II-1 | Mrpl19 | 9801 | 4-May-15 |
| 13717 | 2 | | | | II-1 | Mrpl3 | 11222 | 7-Jun-15 |
| 13718 | 2 | | | | II-1 | Mrpl30 | 51263 | 4-May-15 |
| 13727 | 2 | | | | II-1 | Mrpl4 | 51073 | 4-May-15 |
| 13728 | 2 | | | | II-1 | Mrpl40 | 64976 | 4-May-15 |
| 13729 | 2 | | | | II-1 | Mrpl41 | 64975 | 4-May-15 |
| 13732 | 2 | | | | II-1 | Mrpl44 | 65080 | 21-May-15 |
| 13733 | 2 | | | | II-1 | Mrpl45 | 84311 | 4-May-15 |
| 13740 | 2 | | | | II-1 | Mrpl52 | 122704 | 4-May-15 |
| 13743 | 2 | | | | II-1 | Mrpl55 | 128308 | 4-May-15 |
| 13748 | 2 | | | | II-1 | Mrps12 | 6183 | 7-Jun-15 |
| 13750 | 2 | | | | II-1 | Mrps15 | 64960 | 28-May-15 |
| 13751 | 2 | | | | II-1 | Mrps16 | 51021 | 28-May-15 |
| 13764 | 2 | | | | II-1 | Mrps28 | 28957 | 7-Jun-15 |
| 13766 | 2 | | | | II-1 | Mrps31 | 10240 | 4-May-15 |
| 13767 | 2 | | | | II-1 | Mrps33 | 51650 | 4-May-15 |
| 13771 | 2 | | | | II-1 | Mrps5 | 64969 | 12-May-15 |
| 13773 | 2 | | | | II-1 | Mrps7 | 51081 | 28-May-15 |
| 13776 | 2 | | | | II-1 | Mrs2 | 57380 | 4-May-15 |
| 13782 | 2 | | | | II-1 | Ms4a15 | 219995 | 4-May-15 |
| 13783 | 2 | | | | II-1 | Ms4a18 | 728588 | 4-May-15 |
| 13784 | 2 | | | | II-1 | Ms4a2 | 2206 | 7-Jun-15 |
| 13786 | 2 | | | | II-1 | Ms4a4b | | |
| 13798 | 2 | | | | II-1 | Msantd4 | 84437 | 4-May-15 |
| 13799 | 2 | | | | II-1 | Msc | 9242 | 7-Jun-15 |
| 13802 | 2 | | | | II-1 | Msh3 | 4437 | 4-May-15 |
| 13804 | 2 | | | | II-1 | Msh5 | 4439 | 12-May-15 |
| 13809 | 2 | | | | II-1 | Msl2 | 55167 | 4-May-15 |
| 13810 | 2 | | | | II-1 | Msl3 | 10943 | 4-May-15 |
| 13811 | 2 | | | | II-1 | Msl3l2 | 151507 | 4-May-15 |
| 13838 | 2 | | | | II-1 | Mta3 | 57504 | 4-May-15 |
| 13839 | 2 | | | | II-1 | Mtag2 | 84677 | 4-May-15 |
| 13840 | 2 | | | | II-1 | Mtap | 4507 | 12-May-15 |
| 13842 | 2 | | | | II-1 | Mtbp | 27085 | 4-May-15 |
| 13844 | 2 | | | | II-1 | Mtch2 | 23788 | 4-May-15 |
| 13846 | 2 | | | | II-1 | Mtcp1 | 4515 | 7-Jun-15 |
| 13849 | 2 | | | | II-1 | Mterf1b | | |
| 13852 | 2 | | | | II-1 | Mterfd3 | 80298 | 4-May-15 |
| 13855 | 2 | | | | II-1 | Mtfmt | 123263 | 4-May-15 |
| 13856 | 2 | | | | II-1 | Mtfp1 | 51537 | 12-May-15 |
| 13859 | 2 | | | | II-1 | Mtfr2 | 113115 | 4-May-15 |
| 13870 | 2 | | | | II-1 | Mtif3 | 219402 | 4-May-15 |
| 13871 | 2 | | | | II-1 | Mtl5 | 9633 | 12-May-15 |
| 13872 | 2 | | | | II-1 | Mtm1 | 4534 | 23-May-15 |
| 13873 | 2 | | | | II-1 | Mtmr1 | 8776 | 12-May-15 |
| 13874 | 2 | | | | II-1 | Mtmr10 | 54893 | 4-May-15 |
| 13880 | 2 | | | | II-1 | Mtmr4 | 9110 | 24-May-15 |
| 13881 | 2 | | | | II-1 | Mtmr6 | 9107 | 4-May-15 |
| 13882 | 2 | | | | II-1 | Mtmr7 | 9108 | 12-May-15 |
| 13886 | 2 | | | | II-1 | Mto1 | 25821 | 12-May-15 |

Fig.22 - 105

| | | | | | | | | |
|---|---|---|---|---|---|---|---|---|
| 13887 | 2 | | | | II-1 | Mtor | 2475 | 31-May-15 |
| 13895 | 2 | | | | II-1 | Mtss1l | 92154 | 4-May-15 |
| 14001 | 2 | | | | II-1 | Myl12b | 103910 | 4-May-15 |
| 14023 | 2 | | | | II-1 | Myo1b | 4430 | 4-May-15 |
| 14038 | 2 | | | | II-1 | Myo9a | 4649 | 4-May-15 |
| 14039 | 2 | | | | II-1 | Myo9b | 4650 | 4-May-15 |
| 14055 | 2 | | | | II-1 | Myrfl | 196446 | 12-May-15 |
| 14056 | 2 | | | | II-1 | Myrip | 25924 | 4-May-15 |
| 14058 | 2 | | | | II-1 | Myt1 | 4661 | 7-Jun-15 |
| 14059 | 2 | | | | II-1 | Myt1l | 23040 | 12-May-15 |
| 14060 | 2 | | | | II-1 | Myzap | 100820829 | 4-May-15 |
| 14070 | 2 | | | | II-1 | N4bp3 | 23138 | 4-May-15 |
| 14073 | 2 | | | | II-1 | Naa10 | 8260 | 23-May-15 |
| 14076 | 2 | | | | II-1 | Naa16 | 79612 | 12-May-15 |
| 14079 | 2 | | | | II-1 | Naa30 | 122830 | 4-May-15 |
| 14081 | 2 | | | | II-1 | Naa38 | 84316 | 7-Jun-15 |
| 14084 | 2 | | | | II-1 | Naa60 | 79903 | 4-May-15 |
| 14086 | 2 | | | | II-1 | Naalad2 | 10003 | 4-May-15 |
| 14089 | 2 | | | | II-1 | Nab2 | 4665 | 24-May-15 |
| 14099 | 2 | | | | II-1 | Nae1 | 8883 | 12-May-15 |
| 14101 | 2 | | | | II-1 | Naga | 4668 | 4-May-15 |
| 14102 | 2 | | | | II-1 | Nagk | 55577 | 4-May-15 |
| 14103 | 2 | | | | II-1 | Naglu | 4669 | 4-May-15 |
| 14115 | 2 | | | | II-1 | Nanos1 | 340719 | 4-May-15 |
| 14116 | 2 | | | | II-1 | Nanos2 | 339345 | 4-May-15 |
| 14118 | 2 | | | | II-1 | Nanp | 140838 | 4-May-15 |
| 14134 | 2 | | | | II-1 | Nars | 4677 | 4-May-15 |
| 14137 | 2 | | | | II-1 | Nat1 | 9 | 7-Jun-15 |
| 14142 | 2 | | | | II-1 | Nat6 | 24142 | 7-Jun-15 |
| 14155 | 2 | | | | II-1 | Nbr1 | 4077 | 24-May-15 |
| 14156 | 2 | | | | II-1 | Ncald | 83988 | 4-May-15 |
| 14160 | 2 | | | | II-1 | Ncapd2 | 9918 | 21-May-15 |
| 14177 | 2 | | | | II-1 | Nckap1l | 3071 | 4-May-15 |
| 14182 | 2 | | | | II-1 | Ncln | 56926 | 3-May-15 |
| 14185 | 2 | | | | II-1 | Ncoa2 | 10499 | 17-May-15 |
| 14187 | 2 | | | | II-1 | Ncoa4 | 8031 | 4-May-15 |
| 14188 | 2 | | | | II-1 | Ncoa5 | 57727 | 4-May-15 |
| 14189 | 2 | | | | II-1 | Ncoa6 | 23054 | 4-May-15 |
| 14193 | 2 | | | | II-1 | Ncr1 | 9437 | 4-May-15 |
| 14203 | 2 | | | | II-1 | Ndn | 4692 | 23-May-15 |
| 14204 | 2 | | | | II-1 | Ndnf | 79625 | 4-May-15 |
| 14215 | 2 | | | | II-1 | Ndst4 | 64579 | 4-May-15 |
| 14216 | 2 | | | | II-1 | Ndufa1 | 4694 | 3-May-15 |
| 14221 | 2 | | | | II-1 | Ndufa2 | 4695 | 23-May-15 |
| 14230 | 2 | | | | II-1 | Ndufab1 | 4706 | 4-May-15 |
| 14239 | 2 | | | | II-1 | Ndufb11 | 54539 | 31-May-15 |
| 14245 | 2 | | | | II-1 | Ndufb7 | 4713 | 4-May-15 |
| 14246 | 2 | | | | II-1 | Ndufb8 | 4714 | 23-May-15 |
| 14269 | 2 | | | | II-1 | Nedd1 | 121441 | 4-May-15 |
| 14270 | 2 | | | | II-1 | Nedd4 | 4734 | 4-May-15 |
| 14294 | 2 | | | | II-1 | Nelfcd | 51497 | 4-May-15 |
| 14295 | 2 | | | | II-1 | Nelfe | 7936 | 12-May-15 |
| 14301 | 2 | | | | II-1 | Neo1 | 4756 | 4-May-15 |
| 14322 | 2 | | | | II-1 | Neurog2 | 63973 | 4-May-15 |
| 14327 | 2 | | | | II-1 | Nfam1 | 150372 | 4-May-15 |
| 14334 | 2 | | | | II-1 | Nfatc4 | 4776 | 24-May-15 |
| 14353 | 2 | | | | II-1 | Nfs1 | 9054 | 4-May-15 |
| 14354 | 2 | | | | II-1 | Nfu1 | 27247 | 2-Jun-15 |
| 14356 | 2 | | | | II-1 | Nfxl1 | 152518 | 12-May-15 |
| 14357 | 2 | | | | II-1 | Nfya | 4800 | 4-May-15 |
| 14358 | 2 | | | | II-1 | Nfyb | 4801 | 4-May-15 |
| 14372 | 2 | | | | II-1 | Nhlrc1 | 378884 | 23-May-15 |
| 14386 | 2 | | | | II-1 | Nim1k | 167359 | 4-May-15 |
| 14397 | 2 | | | | II-1 | Nipal4 | 348938 | 23-May-15 |
| 14404 | 2 | | | | II-1 | Nit2 | 56954 | 12-May-15 |
| 14408 | 2 | | | | II-1 | Nkain4 | 128414 | 4-May-15 |
| 14416 | 2 | | | | II-1 | Nkpd1 | 284353 | 4-May-15 |
| 14420 | 2 | | | | II-1 | Nkx1-2 | 390010 | 4-May-15 |
| 14421 | 2 | | | | II-1 | Nkx2-1 | 7080 | 23-May-15 |
| 14427 | 2 | | | | II-1 | Nkx2-6 | 137814 | 4-May-15 |
| 14428 | 2 | | | | II-1 | Nkx2-9 | 26257 | 12-May-15 |
| 14432 | 2 | | | | II-1 | Nkx6-2 | 84504 | 4-May-15 |
| 14441 | 2 | | | | II-1 | Nlrc4 | 58484 | 12-May-15 |
| 14442 | 2 | | | | II-1 | Nlrc5 | 84166 | 10-May-15 |
| 14450 | 2 | | | | II-1 | Nlrp3 | 114548 | 24-May-15 |
| 14453 | 2 | | | | II-1 | Nlrp4c | | |
| 14456 | 2 | | | | II-1 | Nlrp4g | | |
| 14458 | 2 | | | | II-1 | Nlrp5-ps | | |
| 14462 | 2 | | | | II-1 | Nlrp9c | | |
| 14467 | 2 | | | | II-1 | Nme1 | 4830 | 7-Jun-15 |
| 14476 | 2 | | | | II-1 | Nmi | 9111 | 7-Jun-15 |
| 14481 | 2 | | | | II-1 | Nmrk1 | 54981 | 4-May-15 |
| 14485 | 2 | | | | II-1 | Nmt2 | 9397 | 12-May-15 |
| 14493 | 2 | | | | II-1 | Nob1 | 28987 | 17-May-15 |
| 14494 | 2 | | | | II-1 | Nobox | 135935 | 28-May-15 |
| 14495 | 2 | | | | II-1 | Noc2l | | |
| 14498 | 2 | | | | II-1 | Nod1 | 10392 | 29-May-15 |
| 14500 | 2 | | | | II-1 | Nodal | 4838 | 23-May-15 |
| 14501 | 2 | | | | II-1 | Nog | 9241 | 13-May-15 |
| 14505 | 2 | | | | II-1 | Nol3 | 8996 | 24-May-15 |
| 14508 | 2 | | | | II-1 | Nol7 | 51406 | 4-May-15 |
| 14509 | 2 | | | | II-1 | Nol8 | 55035 | 4-May-15 |
| 14513 | 2 | | | | II-1 | Nomo1 | 23420 | 4-May-15 |
| 14514 | 2 | | | | II-1 | Nono | 4841 | 1-Jun-15 |
| 14515 | 2 | | | | II-1 | Nop10 | 55505 | 31-May-15 |
| 14516 | 2 | | | | II-1 | Nop14 | 8602 | 12-May-15 |
| 14527 | 2 | | | | II-1 | Nostrin | 115677 | 4-May-15 |
| 14544 | 2 | | | | II-1 | Npas2 | 4862 | 4-May-15 |
| 14559 | 2 | | | | II-1 | Npffr2 | 10886 | 4-May-15 |
| 14560 | 2 | | | | II-1 | Nphp1 | 4867 | 23-May-15 |
| 14564 | 2 | | | | II-1 | Nphs1os | | |
| 14565 | 2 | | | | II-1 | Nphs2 | 7827 | 12-May-15 |
| 14582 | 2 | | | | II-1 | Npsr1 | 387129 | 31-May-15 |
| 14583 | 2 | | | | II-1 | Nptn | 27020 | 4-May-15 |
| 14593 | 2 | | | | II-1 | Npy5r | 4889 | 4-May-15 |
| 14594 | 2 | | | | II-1 | Npy6r | 4888 | 4-May-15 |
| 14597 | 2 | | | | II-1 | Nr0b1 | 190 | 23-May-15 |
| 14602 | 2 | | | | II-1 | Nr1h3 | 10062 | 31-May-15 |
| 14622 | 2 | | | | II-1 | Nr6a1 | 2649 | 4-May-15 |
| 14628 | 2 | | | | II-1 | Nrbp1 | 29959 | 4-May-15 |
| 14631 | 2 | | | | II-1 | Nrd1 | 4898 | 12-May-15 |
| 14645 | 2 | | | | II-1 | Nrl | 4901 | 23-May-15 |
| 14646 | 2 | | | | II-1 | Nrm | 11270 | 4-May-15 |
| 14665 | 2 | | | | II-1 | Nsg1 | 27065 | 4-May-15 |
| 14670 | 2 | | | | II-1 | Nsmce2 | 286053 | 17-May-15 |
| 14674 | 2 | | | | II-1 | Nsun3 | 63899 | 4-May-15 |
| 14685 | 2 | | | | II-1 | Nt5dc1 | 221294 | 4-May-15 |
| 14704 | 2 | | | | II-1 | Ntrk2 | 4915 | 4-May-15 |
| 14713 | 2 | | | | II-1 | Nubp2 | 10101 | 4-May-15 |
| 14714 | 2 | | | | II-1 | Nubpl | 80224 | 4-May-15 |
| 14720 | 2 | | | | II-1 | Nudcd2 | 134492 | 4-May-15 |
| 14721 | 2 | | | | II-1 | Nudcd3 | 23386 | 4-May-15 |
| 14729 | 2 | | | | II-1 | Nudt16 | 131870 | 4-May-15 |
| 14740 | 2 | | | | II-1 | Nudt6 | 11162 | 12-May-15 |
| 14752 | 2 | | | | II-1 | Nup133 | 55746 | 4-May-15 |
| 14753 | 2 | | | | II-1 | Nup153 | 9972 | 4-May-15 |
| 14754 | 2 | | | | II-1 | Nup155 | 9631 | 4-May-15 |
| 14755 | 2 | | | | II-1 | Nup160 | 23279 | 4-May-15 |
| 14761 | 2 | | | | II-1 | Nup35 | 129401 | 4-May-15 |
| 14765 | 2 | | | | II-1 | Nup54 | 53371 | 4-May-15 |
| 14766 | 2 | | | | II-1 | Nup62 | 23636 | 4-May-15 |
| 14767 | 2 | | | | II-1 | Nup62cl | 54830 | 4-May-15 |
| 14768 | 2 | | | | II-1 | Nup62-il4i1 | | |
| 14770 | 2 | | | | II-1 | Nup88 | 4927 | 4-May-15 |
| 14771 | 2 | | | | II-1 | Nup93 | 9688 | 28-May-15 |
| 14772 | 2 | | | | II-1 | Nup98 | 4928 | 4-May-15 |
| 14783 | 2 | | | | II-1 | Nwd1 | 284434 | 9-May-15 |
| 14785 | 2 | | | | II-1 | Nxf1 | 10482 | 1-Jun-15 |
| 14786 | 2 | | | | II-1 | Nxf2 | 56001 | 14-May-15 |
| 14789 | 2 | | | | II-1 | Nxn | 64359 | 4-May-15 |
| 14798 | 2 | | | | II-1 | Nxph3 | 11248 | 12-May-15 |
| 14802 | 2 | | | | II-1 | Nyap1 | 222950 | 4-May-15 |
| 14803 | 2 | | | | II-1 | Nyap2 | 57624 | 4-May-15 |
| 14829 | 2 | | | | II-1 | Obox3 | | |
| 14839 | 2 | | | | II-1 | Ocel1 | 79629 | 4-May-15 |
| 14857 | 2 | | | | II-1 | Ofd1 | 8481 | 31-May-15 |
| 14866 | 2 | | | | II-1 | Ogn | 4969 | 12-May-15 |
| 14872 | 2 | | | | II-1 | Olah | 55301 | 4-May-15 |
| 14873 | 2 | | | | II-1 | Olfm1 | 10439 | 4-May-15 |
| 14883 | 2 | | | | II-1 | Olfr100 | | |
| 14914 | 2 | | | | II-1 | Olfr1038-ps | | |
| 14978 | 2 | | | | II-1 | Olfr1122 | | |
| 15109 | 2 | | | | II-1 | Olfr1272 | | |
| 15119 | 2 | | | | II-1 | Olfr1281 | | |
| 15120 | 2 | | | | II-1 | Olfr1282 | | |
| 15137 | 2 | | | | II-1 | Olfr1301 | | |
| 15173 | 2 | | | | II-1 | Olfr1340 | | |
| 15179 | 2 | | | | II-1 | Olfr1348 | | |
| 15205 | 2 | | | | II-1 | Olfr1378 | | |
| 15213 | 2 | | | | II-1 | Olfr1386 | | |
| 15224 | 2 | | | | II-1 | Olfr1396 | | |
| 15227 | 2 | | | | II-1 | Olfr1404 | | |
| 15241 | 2 | | | | II-1 | Olfr142 | | |
| 15245 | 2 | | | | II-1 | Olfr1425 | | |
| 15310 | 2 | | | | II-1 | Olfr151 | | |
| 15332 | 2 | | | | II-1 | Olfr168 | | |
| 15349 | 2 | | | | II-1 | Olfr19 | | |
| 15352 | 2 | | | | II-1 | Olfr192 | | |
| 15362 | 2 | | | | II-1 | Olfr201 | | |
| 15373 | 2 | | | | II-1 | Olfr215 | | |
| 15376 | 2 | | | | II-1 | Olfr221 | | |
| 15386 | 2 | | | | II-1 | Olfr237-ps1 | | |
| 15414 | 2 | | | | II-1 | Olfr290 | | |
| 15430 | 2 | | | | II-1 | Olfr307 | | |
| 15436 | 2 | | | | II-1 | Olfr312 | | |
| 15443 | 2 | | | | II-1 | Olfr319 | | |
| 15444 | 2 | | | | II-1 | Olfr32 | | |
| 15456 | 2 | | | | II-1 | Olfr338 | | |
| 15520 | 2 | | | | II-1 | Olfr426 | | |
| 15529 | 2 | | | | II-1 | Olfr437 | | |
| 15621 | 2 | | | | II-1 | Olfr544 | | |
| 15623 | 2 | | | | II-1 | Olfr547 | | |
| 15625 | 2 | | | | II-1 | Olfr55 | | |
| 15629 | 2 | | | | II-1 | Olfr553 | | |
| 15633 | 2 | | | | II-1 | Olfr557 | | |
| 15637 | 2 | | | | II-1 | Olfr560 | | |
| 15762 | 2 | | | | II-1 | Olfr704 | | |
| 15787 | 2 | | | | II-1 | Olfr734 | | |
| 15789 | 2 | | | | II-1 | Olfr736 | | |
| 15824 | 2 | | | | II-1 | Olfr782 | | |
| 15939 | 2 | | | | II-1 | Olfr923 | | |
| 15980 | 2 | | | | II-1 | Olfr979 | | |
| 15994 | 2 | | | | II-1 | Olfr994 | | |
| 16011 | 2 | | | | II-1 | Ooep | 441161 | 4-May-15 |

Fig.22 - 106

| | | | | | | | | |
|---|---|---|---|---|---|---|---|---|
| 16013 | 2 | | | | II-1 | Oog2 | | |
| 16014 | 2 | | | | II-1 | Oog3 | | |
| 16023 | 2 | | | | II-1 | Ophn1 | 4983 | 12-May-15 |
| 16027 | 2 | | | | II-1 | Opn3 | 23596 | 12-May-15 |
| 16030 | 2 | | | | II-1 | Oprd1 | 4985 | 4-May-15 |
| 16031 | 2 | | | | II-1 | Oprk1 | 4986 | 12-May-15 |
| 16033 | 2 | | | | II-1 | Oprm1 | 4988 | 31-May-15 |
| 16043 | 2 | | | | II-1 | Orc4 | 5000 | 4-May-15 |
| 16044 | 2 | | | | II-1 | Orc5 | 5001 | 4-May-15 |
| 16053 | 2 | | | | II-1 | Osbp | 5007 | 28-May-15 |
| 16064 | 2 | | | | II-1 | Osbpl9 | 114883 | 12-May-15 |
| 16086 | 2 | | | | II-1 | Otol1 | 131149 | 4-May-15 |
| 16092 | 2 | | | | II-1 | Otp | 23440 | 4-May-15 |
| 16093 | 2 | | | | II-1 | Ott | 64783 | 4-May-15 |
| 16102 | 2 | | | | II-1 | Otud6b | 51633 | 4-May-15 |
| 16103 | 2 | | | | II-1 | Otud7a | 161725 | 4-May-15 |
| 16107 | 2 | | | | II-1 | Otx2 | 5015 | 28-May-15 |
| 16109 | 2 | | | | II-1 | Ovca2 | 124641 | 4-May-15 |
| 16116 | 2 | | | | II-1 | Oxct1 | 5019 | 12-May-15 |
| 16118 | 2 | | | | II-1 | Oxct2b | | |
| 16120 | 2 | | | | II-1 | Oxld1 | 339229 | 12-May-15 |
| 16123 | 2 | | | | II-1 | Oxsm | 54995 | 4-May-15 |
| 16124 | 2 | | | | II-1 | Oxsr1 | 9943 | 12-May-15 |
| 16130 | 2 | | | | II-1 | P2rx4 | 5025 | 12-May-15 |
| 16146 | 2 | | | | II-1 | P4htm | 54681 | 12-May-15 |
| 16148 | 2 | | | | II-1 | Pabpc1 | 26986 | 12-May-15 |
| 16149 | 2 | | | | II-1 | Pabpc1l | 80336 | 4-May-15 |
| 16151 | 2 | | | | II-1 | Pabpc4 | 8761 | 4-May-15 |
| 16152 | 2 | | | | II-1 | Pabpc4l | 132430 | 12-May-15 |
| 16154 | 2 | | | | II-1 | Pabpc6 | | |
| 16157 | 2 | | | | II-1 | Pacrg | 135138 | 14-May-15 |
| 16167 | 2 | | | | II-1 | Padi4 | 23569 | 21-May-15 |
| 16170 | 2 | | | | II-1 | Pafah1b1 | 5048 | 23-May-15 |
| 16171 | 2 | | | | II-1 | Pafah1b2 | 5049 | 2-Jun-15 |
| 16179 | 2 | | | | II-1 | Paip2 | 51247 | 4-May-15 |
| 16180 | 2 | | | | II-1 | Paip2b | 400961 | 4-May-15 |
| 16181 | 2 | | | | II-1 | Pak1 | 5058 | 7-Jun-15 |
| 16184 | 2 | | | | II-1 | Pak3 | 5063 | 7-Jun-15 |
| 16188 | 2 | | | | II-1 | Palb2 | 79728 | 24-May-15 |
| 16189 | 2 | | | | II-1 | Pald1 | 27143 | 4-May-15 |
| 16210 | 2 | | | | II-1 | Papd7 | 11044 | 4-May-15 |
| 16211 | 2 | | | | II-1 | Papl | 390928 | 4-May-15 |
| 16233 | 2 | | | | II-1 | Park2 | 5071 | 31-May-15 |
| 16239 | 2 | | | | II-1 | Parp10 | 84875 | 4-May-15 |
| 16240 | 2 | | | | II-1 | Parp11 | 57097 | 2-Jun-15 |
| 16243 | 2 | | | | II-1 | Parp16 | 54956 | 4-May-15 |
| 16246 | 2 | | | | II-1 | Parp4 | 143 | 4-May-15 |
| 16247 | 2 | | | | II-1 | Parp6 | 56965 | 12-May-15 |
| 16258 | 2 | | | | II-1 | Patl1 | 219988 | 4-May-15 |
| 16259 | 2 | | | | II-1 | Patl2 | 197135 | 12-May-15 |
| 16260 | 2 | | | | II-1 | Patz1 | 23598 | 4-May-15 |
| 16266 | 2 | | | | II-1 | Pax4 | 5078 | 4-May-15 |
| 16270 | 2 | | | | II-1 | Pax7 | 5081 | 12-May-15 |
| 16276 | 2 | | | | II-1 | Pbk | 55872 | 24-May-15 |
| 16287 | 2 | | | | II-1 | Pcbd1 | 5092 | 4-May-15 |
| 16294 | 2 | | | | II-1 | Pccb | 5096 | 23-May-15 |
| 16301 | 2 | | | | II-1 | Pcdh18 | 54510 | 12-May-15 |
| 16309 | 2 | | | | II-1 | Pcdha11 | 56138 | 4-May-15 |
| 16311 | 2 | | | | II-1 | Pcdha2 | 56146 | 4-May-15 |
| 16312 | 2 | | | | II-1 | Pcdha3 | 56145 | 4-May-15 |
| 16313 | 2 | | | | II-1 | Pcdha4 | 56144 | 4-May-15 |
| 16314 | 2 | | | | II-1 | Pcdha4-g | | |
| 16315 | 2 | | | | II-1 | Pcdha5 | 56143 | 4-May-15 |
| 16316 | 2 | | | | II-1 | Pcdha6 | 56142 | 4-May-15 |
| 16318 | 2 | | | | II-1 | Pcdha8 | 56140 | 4-May-15 |
| 16319 | 2 | | | | II-1 | Pcdha9 | 9752 | 4-May-15 |
| 16320 | 2 | | | | II-1 | Pcdhac1 | 56135 | 4-May-15 |
| 16324 | 2 | | | | II-1 | Pcdhb11 | 56125 | 4-May-15 |
| 16325 | 2 | | | | II-1 | Pcdhb12 | 56124 | 4-May-15 |
| 16326 | 2 | | | | II-1 | Pcdhb13 | 56123 | 4-May-15 |
| 16330 | 2 | | | | II-1 | Pcdhb17 | 54661 | 12-May-15 |
| 16333 | 2 | | | | II-1 | Pcdhb2 | 56133 | 12-May-15 |
| 16334 | 2 | | | | II-1 | Pcdhb20 | | |
| 16335 | 2 | | | | II-1 | Pcdhb21 | | |
| 16337 | 2 | | | | II-1 | Pcdhb3 | 56132 | 4-May-15 |
| 16339 | 2 | | | | II-1 | Pcdhb5 | 26167 | 4-May-15 |
| 16340 | 2 | | | | II-1 | Pcdhb6 | 56130 | 21-May-15 |
| 16342 | 2 | | | | II-1 | Pcdhb8 | 56128 | 4-May-15 |
| 16343 | 2 | | | | II-1 | Pcdhb9 | 56127 | 4-May-15 |
| 16344 | 2 | | | | II-1 | Pcdhga1 | 56114 | 4-May-15 |
| 16352 | 2 | | | | II-1 | Pcdhga6 | 56109 | 4-May-15 |
| 16355 | 2 | | | | II-1 | Pcdhga9 | 56107 | 4-May-15 |
| 16383 | 2 | | | | II-1 | Pcna | 5111 | 17-May-15 |
| 16384 | 2 | | | | II-1 | Pcnp | 57092 | 4-May-15 |
| 16389 | 2 | | | | II-1 | Pcnxl4 | 64430 | 4-May-15 |
| 16414 | 2 | | | | II-1 | Pdcd1 | 5133 | 24-May-15 |
| 16415 | 2 | | | | II-1 | Pdcd10 | 11235 | 23-May-15 |
| 16418 | 2 | | | | II-1 | Pdcd2 | 5134 | 4-May-15 |
| 16420 | 2 | | | | II-1 | Pdcd4 | 27250 | 31-May-15 |
| 16424 | 2 | | | | II-1 | Pdcd7 | 10081 | 4-May-15 |
| 16425 | 2 | | | | II-1 | Pdcl | 5082 | 3-May-15 |
| 16426 | 2 | | | | II-1 | Pdcl2 | 132954 | 4-May-15 |
| 16429 | 2 | | | | II-1 | Pde10a | 10846 | 4-May-15 |
| 16430 | 2 | | | | II-1 | Pde11a | 50940 | 12-May-15 |
| 16431 | 2 | | | | II-1 | Pde12 | 201626 | 12-May-15 |
| 16432 | 2 | | | | II-1 | Pde1a | 5136 | 14-May-15 |
| 16447 | 2 | | | | II-1 | Pde6d | 5147 | 12-May-15 |

| | | | | | | | | |
|---|---|---|---|---|---|---|---|---|
| 16448 | 2 | | | | II-1 | Pde6g | 5148 | 23-May-15 |
| 16452 | 2 | | | | II-1 | Pde8a | 5151 | 4-May-15 |
| 16465 | 2 | | | | II-1 | Pdhb | 5162 | 12-May-15 |
| 16505 | 2 | | | | II-1 | Pdzd8 | 118987 | 4-May-15 |
| 16517 | 2 | | | | II-1 | Pecam1 | 5175 | 17-May-15 |
| 16518 | 2 | | | | II-1 | Pecr | 55825 | 4-May-15 |
| 16528 | 2 | | | | II-1 | Pelo | 53918 | 4-May-15 |
| 16546 | 2 | | | | II-1 | Pex10 | 5192 | 4-May-15 |
| 16552 | 2 | | | | II-1 | Pex14 | 5195 | 4-May-15 |
| 16556 | 2 | | | | II-1 | Pex26 | 55670 | 4-May-15 |
| 16557 | 2 | | | | II-1 | Pex3 | 8504 | 28-May-15 |
| 16559 | 2 | | | | II-1 | Pex5l | 51555 | 3-Jun-15 |
| 16560 | 2 | | | | II-1 | Pex6 | 5190 | 23-May-15 |
| 16588 | 2 | | | | II-1 | Pgbd5 | 79605 | 4-May-15 |
| 16617 | 2 | | | | II-1 | Phax | 51808 | 4-May-15 |
| 16618 | 2 | | | | II-1 | Phb | 5245 | 23-May-15 |
| 16619 | 2 | | | | II-1 | Phb2 | 11331 | 1-Jun-15 |
| 16620 | 2 | | | | II-1 | Phc1 | 1911 | 4-May-15 |
| 16622 | 2 | | | | II-1 | Phc3 | 80012 | 12-May-15 |
| 16632 | 2 | | | | II-1 | Phf14 | 9678 | 12-May-15 |
| 16637 | 2 | | | | II-1 | Phf21a | 51317 | 4-May-15 |
| 16664 | 2 | | | | II-1 | Phox2b | 8929 | 28-May-15 |
| 16667 | 2 | | | | II-1 | Phtf1 | 10745 | 4-May-15 |
| 16671 | 2 | | | | II-1 | Phyh | 5264 | 23-May-15 |
| 16685 | 2 | | | | II-1 | Pias3 | 10401 | 4-May-15 |
| 16687 | 2 | | | | II-1 | Pibf1 | 10464 | 4-May-15 |
| 16690 | 2 | | | | II-1 | Pid1 | 55022 | 3-May-15 |
| 16695 | 2 | | | | II-1 | Pifo | 128344 | 4-May-15 |
| 16704 | 2 | | | | II-1 | Pigm | 93183 | 4-May-15 |
| 16705 | 2 | | | | II-1 | Pign | 23556 | 31-May-15 |
| 16714 | 2 | | | | II-1 | Pigw | 284098 | 4-May-15 |
| 16715 | 2 | | | | II-1 | Pigx | 54965 | 4-May-15 |
| 16717 | 2 | | | | II-1 | Pigz | 80235 | 4-May-15 |
| 16718 | 2 | | | | II-1 | Pih1d1 | 55011 | 4-May-15 |
| 16721 | 2 | | | | II-1 | Pik3ap1 | 118788 | 4-May-15 |
| 16728 | 2 | | | | II-1 | Pik3cd | 5293 | 7-Jun-15 |
| 16733 | 2 | | | | II-1 | Pik3r3 | 8503 | 28-May-15 |
| 16736 | 2 | | | | II-1 | Pik3r6 | 146850 | 4-May-15 |
| 16739 | 2 | | | | II-1 | Pilrb1 | | |
| 16765 | 2 | | | | II-1 | Pira6 | | |
| 16772 | 2 | | | | II-1 | Pisd-ps3 | | |
| 16775 | 2 | | | | II-1 | Pitpnb | 23760 | 4-May-15 |
| 16776 | 2 | | | | II-1 | Pitpnc1 | 26207 | 4-May-15 |
| 16779 | 2 | | | | II-1 | Pitpnm2os1 | | |
| 16786 | 2 | | | | II-1 | Piwil2 | 55124 | 4-May-15 |
| 16787 | 2 | | | | II-1 | Piwil4 | 143689 | 4-May-15 |
| 16793 | 2 | | | | II-1 | Pkd2 | 5311 | 7-Jun-15 |
| 16799 | 2 | | | | II-1 | Pkhd1l1 | 93035 | 12-May-15 |
| 16809 | 2 | | | | II-1 | Pknox1 | 5316 | 4-May-15 |
| 16820 | 2 | | | | II-1 | Pla2g15 | 23659 | 4-May-15 |
| 16821 | 2 | | | | II-1 | Pla2g16 | 11145 | 4-May-15 |
| 16855 | 2 | | | | II-1 | Plcb2 | 5330 | 4-May-15 |
| 16861 | 2 | | | | II-1 | Plce1 | 51196 | 31-May-15 |
| 16874 | 2 | | | | II-1 | Pld3 | 23646 | 12-May-15 |
| 16879 | 2 | | | | II-1 | Plec | 5339 | 23-May-15 |
| 16892 | 2 | | | | II-1 | Plekhd1 | 400224 | 4-May-15 |
| 16897 | 2 | | | | II-1 | Plekhg2 | 64857 | 4-May-15 |
| 16899 | 2 | | | | II-1 | Plekhg4 | 25894 | 17-May-15 |
| 16907 | 2 | | | | II-1 | Plekhm2 | 23207 | 29-May-15 |
| 16908 | 2 | | | | II-1 | Plekhm3 | 389072 | 21-May-15 |
| 16909 | 2 | | | | II-1 | Plekhn1 | 84069 | 4-May-15 |
| 16911 | 2 | | | | II-1 | Plekho2 | 80301 | 12-May-15 |
| 16940 | 2 | | | | II-1 | Plscr4 | 57088 | 4-May-15 |
| 16945 | 2 | | | | II-1 | Plxdc2 | 84898 | 4-May-15 |
| 16951 | 2 | | | | II-1 | Plxnb1 | 5364 | 4-May-15 |
| 16954 | 2 | | | | II-1 | Plxnc1 | 10154 | 4-May-15 |
| 16965 | 2 | | | | II-1 | Pml | 5371 | 21-May-15 |
| 16966 | 2 | | | | II-1 | Pmm1 | 5372 | 4-May-15 |
| 16968 | 2 | | | | II-1 | Pmp2 | 5375 | 4-May-15 |
| 16972 | 2 | | | | II-1 | Pms1 | 5378 | 23-May-15 |
| 16978 | 2 | | | | II-1 | Pnkp | 11284 | 17-May-15 |
| 16979 | 2 | | | | II-1 | Pnldc1 | 154197 | 4-May-15 |
| 16987 | 2 | | | | II-1 | Pnmal1 | 55228 | 4-May-15 |
| 16988 | 2 | | | | II-1 | Pnmal2 | 57469 | 4-May-15 |
| 16991 | 2 | | | | II-1 | Pno1 | 56902 | 12-May-15 |
| 16993 | 2 | | | | II-1 | Pnp | 4860 | 7-Jun-15 |
| 17006 | 2 | | | | II-1 | Poc1a | 25886 | 4-May-15 |
| 17015 | 2 | | | | II-1 | Pofut2 | 23275 | 12-May-15 |
| 17016 | 2 | | | | II-1 | Pogk | 57645 | 4-May-15 |
| 17018 | 2 | | | | II-1 | Pogz | 23126 | 23-May-15 |
| 17019 | 2 | | | | II-1 | Pola1 | 5422 | 12-May-15 |
| 17020 | 2 | | | | II-1 | Pola2 | 23649 | 4-May-15 |
| 17025 | 2 | | | | II-1 | Pold4 | 57804 | 7-Jun-15 |
| 17027 | 2 | | | | II-1 | Poldip3 | 84271 | 4-May-15 |
| 17028 | 2 | | | | II-1 | Pole | 5426 | 17-May-15 |
| 17035 | 2 | | | | II-1 | Poli | 11201 | 4-May-15 |
| 17039 | 2 | | | | II-1 | Poln | 353497 | 4-May-15 |
| 17043 | 2 | | | | II-1 | Polr1c | 9533 | 7-Jun-15 |
| 17045 | 2 | | | | II-1 | Polr1e | 64425 | 4-May-15 |
| 17048 | 2 | | | | II-1 | Polr2c | 5432 | 12-May-15 |
| 17049 | 2 | | | | II-1 | Polr2d | 5433 | 4-May-15 |
| 17050 | 2 | | | | II-1 | Polr2e | 5434 | 4-May-15 |
| 17060 | 2 | | | | II-1 | Polr3b | 55703 | 2-Jun-15 |
| 17061 | 2 | | | | II-1 | Polr3c | 10623 | 12-May-15 |
| 17065 | 2 | | | | II-1 | Polr3g | 10622 | 12-May-15 |
| 17069 | 2 | | | | II-1 | Polrmt | 5442 | 4-May-15 |
| 17072 | 2 | | | | II-1 | Pom121l2 | 94026 | 4-May-15 |

Fig.22 - 107

| | | | | | | | | |
|---|---|---|---|---|---|---|---|---|
| 17077 | 2 | | | | II-1 | Pomp | 51371 | 7-Jun-15 |
| 17079 | 2 | | | | II-1 | Pomt2 | 29954 | 23-May-15 |
| 17095 | 2 | | | | II-1 | Pou1f1 | 5449 | 4-May-15 |
| 17105 | 2 | | | | II-1 | Pou4f1 | 5457 | 28-May-15 |
| 17106 | 2 | | | | II-1 | Pou4f2 | 5458 | 28-May-15 |
| 17107 | 2 | | | | II-1 | Pou4f3 | 5459 | 28-May-15 |
| 17108 | 2 | | | | II-1 | Pou5f1 | 5460 | 24-May-15 |
| 17109 | 2 | | | | II-1 | Pou5f2 | 134187 | 4-May-15 |
| 17110 | 2 | | | | II-1 | Pou6f1 | 5463 | 4-May-15 |
| 17111 | 2 | | | | II-1 | Pou6f2 | 11281 | 4-May-15 |
| 17113 | 2 | | | | II-1 | Ppa1 | 5464 | 4-May-15 |
| 17135 | 2 | | | | II-1 | Ppfia1 | 8500 | 4-May-15 |
| 17136 | 2 | | | | II-1 | Ppfia2 | 8499 | 4-May-15 |
| 17137 | 2 | | | | II-1 | Ppfia3 | 8541 | 4-May-15 |
| 17139 | 2 | | | | II-1 | Ppfibp1 | 8496 | 4-May-15 |
| 17142 | 2 | | | | II-1 | Ppia | 5478 | 12-May-15 |
| 17143 | 2 | | | | II-1 | Ppib | 5479 | 12-May-15 |
| 17147 | 2 | | | | II-1 | Ppif | 10105 | 4-May-15 |
| 17148 | 2 | | | | II-1 | Ppifos | | |
| 17154 | 2 | | | | II-1 | Ppil4 | 85313 | 4-May-15 |
| 17155 | 2 | | | | II-1 | Ppil6 | 285755 | 4-May-15 |
| 17156 | 2 | | | | II-1 | Ppip5k1 | 9677 | 4-May-15 |
| 17161 | 2 | | | | II-1 | Ppm1d | 8493 | 31-May-15 |
| 17162 | 2 | | | | II-1 | Ppm1e | 22843 | 4-May-15 |
| 17165 | 2 | | | | II-1 | Ppm1h | 57460 | 4-May-15 |
| 17174 | 2 | | | | II-1 | Ppp1cb | 5500 | 12-May-15 |
| 17175 | 2 | | | | II-1 | Ppp1cc | 5501 | 21-May-15 |
| 17182 | 2 | | | | II-1 | Ppp1r13l | 10848 | 10-May-15 |
| 17189 | 2 | | | | II-1 | Ppp1r16a | 84988 | 4-May-15 |
| 17190 | 2 | | | | II-1 | Ppp1r16b | 26051 | 12-May-15 |
| 17197 | 2 | | | | II-1 | Ppp1r21 | 129285 | 4-May-15 |
| 17217 | 2 | | | | II-1 | Ppp1r8 | 5511 | 4-May-15 |
| 17221 | 2 | | | | II-1 | Ppp2cb | 5516 | 4-May-15 |
| 17222 | 2 | | | | II-1 | Ppp2r1a | 5518 | 23-May-15 |
| 17223 | 2 | | | | II-1 | Ppp2r1b | 5519 | 12-May-15 |
| 17224 | 2 | | | | II-1 | Ppp2r2a | 5520 | 4-May-15 |
| 17228 | 2 | | | | II-1 | Ppp2r2d | 55844 | 4-May-15 |
| 17232 | 2 | | | | II-1 | Ppp2r4 | 5524 | 12-May-15 |
| 17233 | 2 | | | | II-1 | Ppp2r5a | 5525 | 4-May-15 |
| 17234 | 2 | | | | II-1 | Ppp2r5b | 5526 | 4-May-15 |
| 17237 | 2 | | | | II-1 | Ppp2r5e | 5529 | 4-May-15 |
| 17238 | 2 | | | | II-1 | Ppp3ca | 5530 | 17-May-15 |
| 17240 | 2 | | | | II-1 | Ppp3cc | 5533 | 12-May-15 |
| 17242 | 2 | | | | II-1 | Ppp3r2 | 5535 | 4-May-15 |
| 17243 | 2 | | | | II-1 | Ppp4c | 5531 | 1-Jun-15 |
| 17244 | 2 | | | | II-1 | Ppp4r1 | 9989 | 4-May-15 |
| 17245 | 2 | | | | II-1 | Ppp4r1l-ps | | |
| 17247 | 2 | | | | II-1 | Ppp4r4 | 57718 | 4-May-15 |
| 17248 | 2 | | | | II-1 | Ppp5c | 5536 | 4-May-15 |
| 17249 | 2 | | | | II-1 | Ppp6c | 5537 | 4-May-15 |
| 17251 | 2 | | | | II-1 | Ppp6r2 | 9701 | 4-May-15 |
| 17256 | 2 | | | | II-1 | Pptc7 | 160760 | 4-May-15 |
| 17270 | 2 | | | | II-1 | Pramef6 | 440561 | 4-May-15 |
| 17274 | 2 | | | | II-1 | Pramel4 | | |
| 17276 | 2 | | | | II-1 | Pramel6 | | |
| 17287 | 2 | | | | II-1 | Prdm13 | 59336 | 28-May-15 |
| 17288 | 2 | | | | II-1 | Prdm14 | 63978 | 4-May-15 |
| 17293 | 2 | | | | II-1 | Prdm5 | 11107 | 12-May-15 |
| 17305 | 2 | | | | II-1 | Prelid1 | 27166 | 4-May-15 |
| 17306 | 2 | | | | II-1 | Prelid2 | 153768 | 4-May-15 |
| 17311 | 2 | | | | II-1 | Prex2 | 80243 | 4-May-15 |
| 17318 | 2 | | | | II-1 | Prickle2 | 166336 | 4-May-15 |
| 17331 | 2 | | | | II-1 | Prkag1 | 5571 | 31-May-15 |
| 17332 | 2 | | | | II-1 | Prkag2 | 51422 | 31-May-15 |
| 17355 | 2 | | | | II-1 | Prkg2 | 5593 | 17-May-15 |
| 17359 | 2 | | | | II-1 | Prkx | 5613 | 4-May-15 |
| 17363 | 2 | | | | II-1 | Prl2c1 | | |
| 17373 | 2 | | | | II-1 | Prl3d3 | | |
| 17394 | 2 | | | | II-1 | Prmt10 | 90826 | 4-May-15 |
| 17395 | 2 | | | | II-1 | Prmt2 | 3275 | 4-May-15 |
| 17399 | 2 | | | | II-1 | Prmt7 | 54496 | 4-May-15 |
| 17400 | 2 | | | | II-1 | Prmt8 | 56341 | 4-May-15 |
| 17410 | 2 | | | | II-1 | Prok1 | 84432 | 4-May-15 |
| 17418 | 2 | | | | II-1 | Prorsd1 | | |
| 17428 | 2 | | | | II-1 | Prpf19 | 27339 | 4-May-15 |
| 17429 | 2 | | | | II-1 | Prpf3 | 9129 | 23-May-15 |
| 17430 | 2 | | | | II-1 | Prpf31 | 26121 | 23-May-15 |
| 17431 | 2 | | | | II-1 | Prpf38a | 84950 | 4-May-15 |
| 17433 | 2 | | | | II-1 | Prpf39 | 55015 | 4-May-15 |
| 17434 | 2 | | | | II-1 | Prpf4 | 9128 | 4-May-15 |
| 17435 | 2 | | | | II-1 | Prpf40a | 55660 | 4-May-15 |
| 17446 | 2 | | | | II-1 | Prps2 | 5634 | 4-May-15 |
| 17449 | 2 | | | | II-1 | Prr11 | 55771 | 17-May-15 |
| 17459 | 2 | | | | II-1 | Prr22 | 163154 | 4-May-15 |
| 17460 | 2 | | | | II-1 | Prr23a | 729627 | 4-May-15 |
| 17473 | 2 | | | | II-1 | Prrc2b | 84726 | 4-May-15 |
| 17501 | 2 | | | | II-1 | Prss35 | 167681 | 4-May-15 |
| 17505 | 2 | | | | II-1 | Prss39 | | |
| 17506 | 2 | | | | II-1 | Prss40 | | |
| 17507 | 2 | | | | II-1 | Prss41 | 360226 | 4-May-15 |
| 17508 | 2 | | | | II-1 | Prss42 | 339906 | 4-May-15 |
| 17509 | 2 | | | | II-1 | Prss43 | 100288 960 | 4-May-15 |
| 17510 | 2 | | | | II-1 | Prss44 | 729756 | 17-Mar-15 |
| 17514 | 2 | | | | II-1 | Prss50 | 29122 | 4-May-15 |
| 17516 | 2 | | | | II-1 | Prss52 | | |
| 17520 | 2 | | | | II-1 | Prss56 | 646960 | 4-May-15 |
| 17521 | 2 | | | | II-1 | Prss57 | 400668 | 23-May-15 |

| | | | | | | | | |
|---|---|---|---|---|---|---|---|---|
| 17522 | 2 | | | | II-1 | Prss58 | 136541 | 4-May-15 |
| 17525 | 2 | | | | II-1 | Prtn3 | 5657 | 17-May-15 |
| 17540 | 2 | | | | II-1 | Psg16 | | |
| 17543 | 2 | | | | II-1 | Psg19 | | |
| 17546 | 2 | | | | II-1 | Psg22 | | |
| 17551 | 2 | | | | II-1 | Psg28 | | |
| 17558 | 2 | | | | II-1 | Psma3 | 5684 | 4-May-15 |
| 17563 | 2 | | | | II-1 | Psma8 | 143471 | 12-May-15 |
| 17564 | 2 | | | | II-1 | Psmb1 | 5689 | 4-May-15 |
| 17565 | 2 | | | | II-1 | Psmb10 | 5699 | 4-May-15 |
| 17578 | 2 | | | | II-1 | Psmc3ip | 29893 | 4-May-15 |
| 17583 | 2 | | | | II-1 | Psmd10 | 5716 | 4-May-15 |
| 17584 | 2 | | | | II-1 | Psmd11 | 5717 | 4-May-15 |
| 17586 | 2 | | | | II-1 | Psmd13 | 5719 | 4-May-15 |
| 17590 | 2 | | | | II-1 | Psmd4 | 5710 | 4-May-15 |
| 17591 | 2 | | | | II-1 | Psmd5 | 5711 | 4-May-15 |
| 17592 | 2 | | | | II-1 | Psmd6 | 9861 | 4-May-15 |
| 17593 | 2 | | | | II-1 | Psmd7 | 5713 | 4-May-15 |
| 17594 | 2 | | | | II-1 | Psmd8 | 5714 | 4-May-15 |
| 17595 | 2 | | | | II-1 | Psmd9 | 5715 | 4-May-15 |
| 17600 | 2 | | | | II-1 | Psme4 | 23198 | 4-May-15 |
| 17601 | 2 | | | | II-1 | Psmf1 | 9491 | 4-May-15 |
| 17602 | 2 | | | | II-1 | Psmg1 | 8624 | 4-May-15 |
| 17603 | 2 | | | | II-1 | Psmg2 | 56984 | 4-May-15 |
| 17619 | 2 | | | | II-1 | Ptcd1 | 26024 | 17-May-15 |
| 17627 | 2 | | | | II-1 | Ptchd4 | 442213 | 4-May-15 |
| 17628 | 2 | | | | II-1 | Ptcra | 171558 | 12-May-15 |
| 17629 | 2 | | | | II-1 | Ptdss1 | 9791 | 4-May-15 |
| 17647 | 2 | | | | II-1 | Ptgir | 5739 | 4-May-15 |
| 17664 | 2 | | | | II-1 | Ptms | 5763 | 4-May-15 |
| 17677 | 2 | | | | II-1 | Ptpn11 | 5781 | 31-May-15 |
| 17678 | 2 | | | | II-1 | Ptpn12 | 5782 | 4-May-15 |
| 17684 | 2 | | | | II-1 | Ptpn21 | 11099 | 4-May-15 |
| 17691 | 2 | | | | II-1 | Ptpn7 | 5778 | 4-May-15 |
| 17702 | 2 | | | | II-1 | Ptprj | 5795 | 12-May-15 |
| 17707 | 2 | | | | II-1 | Ptpro | 5800 | 7-Jun-15 |
| 17713 | 2 | | | | II-1 | Ptpru | 10076 | 12-May-15 |
| 17714 | 2 | | | | II-1 | Ptprv | 148713 | 4-May-15 |
| 17720 | 2 | | | | II-1 | Pts | 5805 | 12-May-15 |
| 17727 | 2 | | | | II-1 | Pum2 | 23369 | 29-May-15 |
| 17728 | 2 | | | | II-1 | Pura | 5813 | 28-May-15 |
| 17733 | 2 | | | | II-1 | Pus3 | 83480 | 4-May-15 |
| 17735 | 2 | | | | II-1 | Pus7l | 83448 | 4-May-15 |
| 17746 | 2 | | | | II-1 | Pwwp2a | 114825 | 4-May-15 |
| 17747 | 2 | | | | II-1 | Pwwp2b | 170394 | 12-May-15 |
| 17754 | 2 | | | | II-1 | Pxt1 | 222659 | 4-May-15 |
| 17755 | 2 | | | | II-1 | Pxylp1 | 92370 | 4-May-15 |
| 17781 | 2 | | | | II-1 | Qrich1 | 54870 | 12-May-15 |
| 17783 | 2 | | | | II-1 | Qrsl1 | 55278 | 4-May-15 |
| 17784 | 2 | | | | II-1 | Qser1 | 79832 | 4-May-15 |
| 17790 | 2 | | | | II-1 | R3hcc1l | 27291 | 4-May-15 |
| 17791 | 2 | | | | II-1 | R3hdm1 | 23518 | 4-May-15 |
| 17792 | 2 | | | | II-1 | R3hdm2 | 22864 | 21-May-15 |
| 17793 | 2 | | | | II-1 | R3hdm4 | 91300 | 4-May-15 |
| 17794 | 2 | | | | II-1 | R3hdml | 140902 | 4-May-15 |
| 17795 | 2 | | | | II-1 | R74862 | | |
| 17796 | 2 | | | | II-1 | Rab1 | 5861 | 7-Jun-15 |
| 17798 | 2 | | | | II-1 | Rab10os | | |
| 17800 | 2 | | | | II-1 | Rab11b | 9230 | 7-Jun-15 |
| 17807 | 2 | | | | II-1 | Rab11fip5 | 26056 | 4-May-15 |
| 17818 | 2 | | | | II-1 | Rab22a | 57403 | 4-May-15 |
| 17820 | 2 | | | | II-1 | Rab24 | 53917 | 21-May-15 |
| 17836 | 2 | | | | II-1 | Rab36 | 9609 | 23-May-15 |
| 17837 | 2 | | | | II-1 | Rab37 | 326624 | 12-May-15 |
| 17843 | 2 | | | | II-1 | Rab3c | 115827 | 12-May-15 |
| 17846 | 2 | | | | II-1 | Rab3gap2 | 25782 | 4-May-15 |
| 17847 | 2 | | | | II-1 | Rab3il1 | 5866 | 4-May-15 |
| 17856 | 2 | | | | II-1 | Rab5a | 5868 | 31-May-15 |
| 17857 | 2 | | | | II-1 | Rab5b | 5869 | 2-Jun-15 |
| 17858 | 2 | | | | II-1 | Rab5c | 5878 | 4-May-15 |
| 17859 | 2 | | | | II-1 | Rab6a | 5870 | 29-May-15 |
| 17860 | 2 | | | | II-1 | Rab6b | 51560 | 23-May-15 |
| 17865 | 2 | | | | II-1 | Rab9 | 9367 | 29-May-15 |
| 17869 | 2 | | | | II-1 | Rabep2 | 79874 | 4-May-15 |
| 17871 | 2 | | | | II-1 | Rabgap1 | 23637 | 4-May-15 |
| 17872 | 2 | | | | II-1 | Rabgap1l | 9910 | 3-May-15 |
| 17874 | 2 | | | | II-1 | Rabggta | 5875 | 12-May-15 |
| 17875 | 2 | | | | II-1 | Rabggtb | 5876 | 4-May-15 |
| 17876 | 2 | | | | II-1 | Rabif | 5877 | 4-May-15 |
| 17877 | 2 | | | | II-1 | Rabl2 | | |
| 17880 | 2 | | | | II-1 | Rac1 | 5879 | 7-Jun-15 |
| 17881 | 2 | | | | II-1 | Rac2 | 5880 | 4-May-15 |
| 17886 | 2 | | | | II-1 | Rad18 | 56852 | 12-May-15 |
| 17890 | 2 | | | | II-1 | Rad23b | 5887 | 4-May-15 |
| 17892 | 2 | | | | II-1 | Rad51 | 5888 | 24-May-15 |
| 17897 | 2 | | | | II-1 | Rad51d | 5892 | 4-May-15 |
| 17905 | 2 | | | | II-1 | Rae1 | 8480 | 12-May-15 |
| 17907 | 2 | | | | II-1 | Raet1b | | |
| 17908 | 2 | | | | II-1 | Raet1c | | |
| 17920 | 2 | | | | II-1 | Ralgapa1 | 253959 | 12-May-15 |
| 17921 | 2 | | | | II-1 | Ralgapa2 | 57186 | 4-May-15 |
| 17923 | 2 | | | | II-1 | Ralgds | 5900 | 12-May-15 |
| 17933 | 2 | | | | II-1 | Ranbp10 | 57610 | 4-May-15 |
| 17938 | 2 | | | | II-1 | Ranbp6 | 26953 | 12-May-15 |
| 17941 | 2 | | | | II-1 | Rangrf | 29098 | 4-May-15 |
| 17944 | 2 | | | | II-1 | Rap1gap | 5909 | 4-May-15 |
| 17950 | 2 | | | | II-1 | Rapgef1 | 2889 | 12-May-15 |

Fig.22 - 108

| | | | | | | | | |
|---|---|---|---|---|---|---|---|---|
| 17951 | 2 | | | | II-1 | Rapgef2 | 9693 | 4-May-15 |
| 17968 | 2 | | | | II-1 | Rasa3 | 22821 | 12-May-15 |
| 18004 | 2 | | | | II-1 | Raver2 | 55225 | 4-May-15 |
| 18007 | 2 | | | | II-1 | Rb1cc1 | 9821 | 23-May-15 |
| 18010 | 2 | | | | II-1 | Rbbp4 | 5928 | 24-May-15 |
| 18011 | 2 | | | | II-1 | Rbbp5 | 5929 | 4-May-15 |
| 18014 | 2 | | | | II-1 | Rbbp8 | 5932 | 17-May-15 |
| 18018 | 2 | | | | II-1 | Rbfa | 79863 | 4-May-15 |
| 18028 | 2 | | | | II-1 | Rbm12b1 | | |
| 18043 | 2 | | | | II-1 | Rbm28 | 55131 | 12-May-15 |
| 18055 | 2 | | | | II-1 | Rbm44 | 375316 | 4-May-15 |
| 18058 | 2 | | | | II-1 | Rbm46os | | |
| 18059 | 2 | | | | II-1 | Rbm47 | 54502 | 21-May-15 |
| 18062 | 2 | | | | II-1 | Rbm5 | 10181 | 4-May-15 |
| 18063 | 2 | | | | II-1 | Rbm6 | 10180 | 4-May-15 |
| 18065 | 2 | | | | II-1 | Rbm8a | 9939 | 23-May-15 |
| 18066 | 2 | | | | II-1 | Rbms1 | 5937 | 4-May-15 |
| 18072 | 2 | | | | II-1 | Rbmxl2 | 27288 | 4-May-15 |
| 18073 | 2 | | | | II-1 | Rbmy | 5940 | 23-May-15 |
| 18085 | 2 | | | | II-1 | Rc3h2 | 54542 | 4-May-15 |
| 18086 | 2 | | | | II-1 | Rcan1 | 1827 | 17-May-15 |
| 18091 | 2 | | | | II-1 | Rcc1 | 1104 | 4-May-15 |
| 18092 | 2 | | | | II-1 | Rcc2 | 55920 | 12-May-15 |
| 18094 | 2 | | | | II-1 | Rce1 | 9986 | 4-May-15 |
| 18096 | 2 | | | | II-1 | Rcl1 | 10171 | 1-Jun-15 |
| 18105 | 2 | | | | II-1 | Rd3 | 343035 | 23-May-15 |
| 18106 | 2 | | | | II-1 | Rd3l | 647286 | 4-May-15 |
| 18112 | 2 | | | | II-1 | Rdh14 | 57665 | 4-May-15 |
| 18146 | 2 | | | | II-1 | Reln | 5649 | 17-May-15 |
| 18173 | 2 | | | | II-1 | Rexo2 | 25996 | 4-May-15 |
| 18174 | 2 | | | | II-1 | Rexo4 | 57109 | 4-May-15 |
| 18176 | 2 | | | | II-1 | Rfc2 | 5982 | 4-May-15 |
| 18183 | 2 | | | | II-1 | Rfng | 5986 | 4-May-15 |
| 18184 | 2 | | | | II-1 | Rfpl3s | 10737 | 4-May-15 |
| 18185 | 2 | | | | II-1 | Rfpl4 | 342931 | 24-May-15 |
| 18186 | 2 | | | | II-1 | Rfpl4b | 442247 | 4-May-15 |
| 18187 | 2 | | | | II-1 | Rft1 | 91869 | 13-Jun-15 |
| 18191 | 2 | | | | II-1 | Rfwd3 | 55159 | 28-May-15 |
| 18192 | 2 | | | | II-1 | Rfx1 | 5989 | 28-May-15 |
| 18197 | 2 | | | | II-1 | Rfx6 | 222546 | 28-May-15 |
| 18198 | 2 | | | | II-1 | Rfx7 | 64864 | 4-May-15 |
| 18199 | 2 | | | | II-1 | Rfx8 | 731220 | 4-May-15 |
| 18201 | 2 | | | | II-1 | Rfxap | 5994 | 4-May-15 |
| 18202 | 2 | | | | II-1 | Rgag1 | 57529 | 4-May-15 |
| 18226 | 2 | | | | II-1 | Rgs22 | 26166 | 4-May-15 |
| 18236 | 2 | | | | II-1 | Rgsl1 | 353299 | 4-May-15 |
| 18257 | 2 | | | | II-1 | Rhobtb3 | 22836 | 4-May-15 |
| 18271 | 2 | | | | II-1 | Rhox11 | | |
| 18274 | 2 | | | | II-1 | Rhox2a | | |
| 18276 | 2 | | | | II-1 | Rhox2c | | |
| 18291 | 2 | | | | II-1 | Rhox4d | | |
| 18292 | 2 | | | | II-1 | Rhox4e | | |
| 18293 | 2 | | | | II-1 | Rhox4f | | |
| 18298 | 2 | | | | II-1 | Rhox8 | | |
| 18306 | 2 | | | | II-1 | Ric8 | 60626 | 7-Jun-15 |
| 18308 | 2 | | | | II-1 | Rictor | 253260 | 4-May-15 |
| 18315 | 2 | | | | II-1 | Rimbp3 | 85376 | 4-May-15 |
| 18327 | 2 | | | | II-1 | Rint1 | 60561 | 29-May-15 |
| 18329 | 2 | | | | II-1 | Riok2 | 55781 | 4-May-15 |
| 18330 | 2 | | | | II-1 | Riok3 | 8780 | 4-May-15 |
| 18331 | 2 | | | | II-1 | Ripk1 | 8737 | 4-May-15 |
| 18332 | 2 | | | | II-1 | Ripk2 | 8767 | 29-May-15 |
| 18336 | 2 | | | | II-1 | Ripply2 | 134701 | 12-May-15 |
| 18337 | 2 | | | | II-1 | Ripply3 | 53820 | 4-May-15 |
| 18340 | 2 | | | | II-1 | Rita1 | 84934 | 4-May-15 |
| 18342 | 2 | | | | II-1 | Rlf | 6018 | 7-Jun-15 |
| 18343 | 2 | | | | II-1 | Rlim | 51132 | 4-May-15 |
| 18370 | 2 | | | | II-1 | Rnaseh2a | 10535 | 23-May-15 |
| 18382 | 2 | | | | II-1 | Rnf11 | 26994 | 2-Jun-15 |
| 18383 | 2 | | | | II-1 | Rnf111 | 54778 | 4-May-15 |
| 18388 | 2 | | | | II-1 | Rnf115 | 27246 | 4-May-15 |
| 18397 | 2 | | | | II-1 | Rnf133 | 168433 | 4-May-15 |
| 18398 | 2 | | | | II-1 | Rnf135 | 84282 | 4-May-15 |
| 18399 | 2 | | | | II-1 | Rnf138 | 51444 | 4-May-15 |
| 18400 | 2 | | | | II-1 | Rnf138rt1 | | |
| 18401 | 2 | | | | II-1 | Rnf139 | 11236 | 4-May-15 |
| 18403 | 2 | | | | II-1 | Rnf141 | 50862 | 1-Jun-15 |
| 18408 | 2 | | | | II-1 | Rnf148 | 378925 | 4-May-15 |
| 18415 | 2 | | | | II-1 | Rnf166 | 115992 | 4-May-15 |
| 18417 | 2 | | | | II-1 | Rnf168 | 165918 | 23-May-15 |
| 18420 | 2 | | | | II-1 | Rnf170 | 81790 | 23-May-15 |
| 18428 | 2 | | | | II-1 | Rnf19a | 25897 | 12-May-15 |
| 18430 | 2 | | | | II-1 | Rnf2 | 6045 | 2-Jun-15 |
| 18432 | 2 | | | | II-1 | Rnf207 | 388591 | 4-May-15 |
| 18435 | 2 | | | | II-1 | Rnf215 | 200312 | 4-May-15 |
| 18441 | 2 | | | | II-1 | Rnf223 | 401934 | 4-May-15 |
| 18446 | 2 | | | | II-1 | Rnf31 | 55072 | 4-May-15 |
| 18449 | 2 | | | | II-1 | Rnf38 | 152006 | 2-Jun-15 |
| 18450 | 2 | | | | II-1 | Rnf39 | 80352 | 4-May-15 |
| 18453 | 2 | | | | II-1 | Rnf41 | 10193 | 2-Jun-15 |
| 18456 | 2 | | | | II-1 | Rnf5 | 6048 | 2-Jun-15 |
| 18458 | 2 | | | | II-1 | Rnf7 | 9616 | 2-Jun-15 |
| 18459 | 2 | | | | II-1 | Rnf8 | 9025 | 4-May-15 |
| 18460 | 2 | | | | II-1 | Rnft1 | 51136 | 4-May-15 |
| 18461 | 2 | | | | II-1 | Rnft2 | 84900 | 12-May-15 |
| 18463 | 2 | | | | II-1 | Rnh1 | 6050 | 13-Jun-15 |
| 18464 | 2 | | | | II-1 | Rnls | 55328 | 12-May-15 |
| 18467 | 2 | | | | II-1 | Rnpc3 | 55599 | 4-May-15 |
| 18469 | 2 | | | | II-1 | Rnpepl1 | 57140 | 4-May-15 |
| 18470 | 2 | | | | II-1 | Rnps1 | 10921 | 12-May-15 |
| 18479 | 2 | | | | II-1 | Robo4 | 54538 | 4-May-15 |
| 18494 | 2 | | | | II-1 | Rp1l1 | 94137 | 12-May-15 |
| 18495 | 2 | | | | II-1 | Rp2h | | |
| 18496 | 2 | | | | II-1 | Rp9 | 6100 | 23-May-15 |
| 18498 | 2 | | | | II-1 | Rpa2 | 6118 | 7-Jun-15 |
| 18503 | 2 | | | | II-1 | Rpap3 | 79657 | 4-May-15 |
| 18514 | 2 | | | | II-1 | Rpl10 | 6134 | 7-Jun-15 |
| 18517 | 2 | | | | II-1 | Rpl11 | 6135 | 23-May-15 |
| 18526 | 2 | | | | II-1 | Rpl18a | 6142 | 12-May-15 |
| 18530 | 2 | | | | II-1 | Rpl22l1 | 200916 | 4-May-15 |
| 18535 | 2 | | | | II-1 | Rpl27 | 6155 | 21-May-15 |
| 18564 | 2 | | | | II-1 | Rpl8 | 6132 | 4-May-15 |
| 18565 | 2 | | | | II-1 | Rpl9 | 6133 | 4-May-15 |
| 18571 | 2 | | | | II-1 | Rpn2 | 6185 | 7-Jun-15 |
| 18572 | 2 | | | | II-1 | Rpp14 | 11102 | 4-May-15 |
| 18582 | 2 | | | | II-1 | Rprd2 | 23248 | 4-May-15 |
| 18583 | 2 | | | | II-1 | Rprl1 | | |
| 18584 | 2 | | | | II-1 | Rprl2 | | |
| 18585 | 2 | | | | II-1 | Rprl3 | | |
| 18594 | 2 | | | | II-1 | Rps15a | 6210 | 4-May-15 |
| 18606 | 2 | | | | II-1 | Rps23 | 6228 | 12-May-15 |
| 18623 | 2 | | | | II-1 | Rps6ka2 | 6196 | 21-May-15 |
| 18627 | 2 | | | | II-1 | Rps6ka6 | 27330 | 4-May-15 |
| 18629 | 2 | | | | II-1 | Rps6kb2 | 6199 | 4-May-15 |
| 18630 | 2 | | | | II-1 | Rps6kc1 | 26750 | 21-May-15 |
| 18638 | 2 | | | | II-1 | Rptoros | | |
| 18639 | 2 | | | | II-1 | Rpusd1 | 113000 | 4-May-15 |
| 18641 | 2 | | | | II-1 | Rpusd3 | 285367 | 4-May-15 |
| 18653 | 2 | | | | II-1 | Rrh | 10692 | 4-May-15 |
| 18654 | 2 | | | | II-1 | Rrm1 | 6240 | 4-May-15 |
| 18658 | 2 | | | | II-1 | Rrnad1 | 51093 | 4-May-15 |
| 18659 | 2 | | | | II-1 | Rrp1 | 8568 | 7-Jun-15 |
| 18664 | 2 | | | | II-1 | Rrp7a | 27341 | 4-May-15 |
| 18666 | 2 | | | | II-1 | Rrp9 | 9136 | 4-May-15 |
| 18668 | 2 | | | | II-1 | Rs1 | 6247 | 24-May-15 |
| 18669 | 2 | | | | II-1 | Rsad1 | 55316 | 4-May-15 |
| 18676 | 2 | | | | II-1 | Rsl1 | | |
| 18679 | 2 | | | | II-1 | Rslcan18 | | |
| 18680 | 2 | | | | II-1 | Rsph1 | 89765 | 4-May-15 |
| 18688 | 2 | | | | II-1 | Rspo3 | 84870 | 4-May-15 |
| 18692 | 2 | | | | II-1 | Rsrc2 | 65117 | 4-May-15 |
| 18693 | 2 | | | | II-1 | Rsrp1 | 57035 | 4-May-15 |
| 18695 | 2 | | | | II-1 | Rtbdn | 83546 | 10-May-15 |
| 18697 | 2 | | | | II-1 | Rtcb | 51493 | 12-May-15 |
| 18699 | 2 | | | | II-1 | Rtel1 | 51750 | 4-Jun-15 |
| 18700 | 2 | | | | II-1 | Rtf1 | 23168 | 4-May-15 |
| 18701 | 2 | | | | II-1 | Rtfdc1 | 51507 | 4-May-15 |
| 18702 | 2 | | | | II-1 | Rtkn | 6242 | 12-May-15 |
| 18710 | 2 | | | | II-1 | Rtn4r | 65078 | 4-May-15 |
| 18720 | 2 | | | | II-1 | Rufy2 | 55680 | 1-Jun-15 |
| 18721 | 2 | | | | II-1 | Rufy3 | 22902 | 7-Jun-15 |
| 18734 | 2 | | | | II-1 | Rwdd1 | 51389 | 4-May-15 |
| 18740 | 2 | | | | II-1 | Rxfp2 | 122042 | 4-May-15 |
| 18741 | 2 | | | | II-1 | Rxfp3 | 51289 | 4-May-15 |
| 18743 | 2 | | | | II-1 | Rxra | 6256 | 4-May-15 |
| 18751 | 2 | | | | II-1 | S100a1 | 6271 | 4-May-15 |
| 18782 | 2 | | | | II-1 | Sae1 | 10055 | 4-May-15 |
| 18783 | 2 | | | | II-1 | Safb | 6294 | 4-May-15 |
| 18789 | 2 | | | | II-1 | Sall4 | 57167 | 23-May-15 |
| 18803 | 2 | | | | II-1 | Samhd1 | 25939 | 23-May-15 |
| 18808 | 2 | | | | II-1 | Samt4 | | |
| 18809 | 2 | | | | II-1 | Sap130 | 79595 | 7-Jun-15 |
| 18810 | 2 | | | | II-1 | Sap18 | 10284 | 4-May-15 |
| 18823 | 2 | | | | II-1 | Sars2 | 54938 | 4-May-15 |
| 18824 | 2 | | | | II-1 | Sart1 | 9092 | 12-May-15 |
| 18826 | 2 | | | | II-1 | Sash1 | 23328 | 12-May-15 |
| 18836 | 2 | | | | II-1 | Sbds | 51119 | 23-May-15 |
| 18837 | 2 | | | | II-1 | Sbf1 | 6305 | 23-May-15 |
| 18838 | 2 | | | | II-1 | Sbf2 | 81846 | 23-May-15 |
| 18839 | 2 | | | | II-1 | Sbk1 | 388228 | 4-May-15 |
| 18843 | 2 | | | | II-1 | Sbno2 | 22904 | 4-May-15 |
| 18851 | 2 | | | | II-1 | Scaf4 | 57466 | 12-May-15 |
| 18853 | 2 | | | | II-1 | Scai | 286205 | 4-May-15 |
| 18854 | 2 | | | | II-1 | Scamp1 | 9522 | 12-May-15 |
| 18856 | 2 | | | | II-1 | Scamp3 | 10067 | 4-May-15 |
| 18857 | 2 | | | | II-1 | Scamp4 | 113178 | 4-May-15 |
| 18858 | 2 | | | | II-1 | Scamp5 | 192683 | 4-May-15 |
| 18859 | 2 | | | | II-1 | Scand1 | 51282 | 28-May-15 |
| 18873 | 2 | | | | II-1 | Scarna3a | | |
| 18887 | 2 | | | | II-1 | Scg3 | 29106 | 7-Jun-15 |
| 18892 | 2 | | | | II-1 | Scgb1b20 | | |
| 18921 | 2 | | | | II-1 | Scml2 | 10389 | 31-May-15 |
| 18936 | 2 | | | | II-1 | Scn9a | 6335 | 24-May-15 |
| 18937 | 2 | | | | II-1 | Scnm1 | 79005 | 4-May-15 |
| 18945 | 2 | | | | II-1 | Scp2d1 | 140856 | 4-May-15 |
| 18954 | 2 | | | | II-1 | Scrt2 | 85508 | 4-May-15 |
| 18963 | 2 | | | | II-1 | Scyl3 | 57147 | 7-Jun-15 |
| 18964 | 2 | | | | II-1 | Sdad1 | 55153 | 4-May-15 |
| 18972 | 2 | | | | II-1 | Sdccag8 | 10806 | 23-May-15 |
| 18973 | 2 | | | | II-1 | Sde2 | 163859 | 4-May-15 |
| 18983 | 2 | | | | II-1 | Sdk1 | 221935 | 4-May-15 |
| 18984 | 2 | | | | II-1 | Sdk2 | 54549 | 4-May-15 |
| 18985 | 2 | | | | II-1 | Sdpr | 8436 | 12-May-15 |
| 18989 | 2 | | | | II-1 | Sdr42e1 | 93517 | 4-May-15 |

Fig.22 - 109

| | | | | | | | | |
|---|---|---|---|---|---|---|---|---|
| 18998 | 2 | | | | II-1 | Sec14l1 | 6397 | 31-May-15 |
| 19008 | 2 | | | | II-1 | Sec23a | 10484 | 12-May-15 |
| 19013 | 2 | | | | II-1 | Sec24c | 9632 | 12-May-15 |
| 19023 | 2 | | | | II-1 | Secisbp2 | 79048 | 4-May-15 |
| 19029 | 2 | | | | II-1 | Sel1l2 | 80343 | 4-May-15 |
| 19042 | 2 | | | | II-1 | Sema3b | 7869 | 12-May-15 |
| 19050 | 2 | | | | II-1 | Sema4c | 54910 | 4-May-15 |
| 19052 | 2 | | | | II-1 | Sema4f | 10505 | 4-May-15 |
| 19063 | 2 | | | | II-1 | Senp3 | 26168 | 4-May-15 |
| 19064 | 2 | | | | II-1 | Senp5 | 205564 | 12-May-15 |
| 19065 | 2 | | | | II-1 | Senp6 | 26054 | 12-May-15 |
| 19066 | 2 | | | | II-1 | Senp7 | 57337 | 4-May-15 |
| 19067 | 2 | | | | II-1 | Senp8 | 123228 | 4-May-15 |
| 19069 | 2 | | | | II-1 | Sephs2 | 22928 | 4-May-15 |
| 19077 | 2 | | | | II-1 | Sept14 | 346288 | 4-May-15 |
| 19078 | 2 | | | | II-1 | Sept15 | | |
| 19079 | 2 | | | | II-1 | Sept2 | 4735 | 7-Jun-15 |
| 19080 | 2 | | | | II-1 | Sept3 | 55964 | 4-May-15 |
| 19090 | 2 | | | | II-1 | Serf1 | 8293 | 4-May-15 |
| 19093 | 2 | | | | II-1 | Serhl | 94009 | 4-May-15 |
| 19098 | 2 | | | | II-1 | Serinc5 | 256987 | 28-May-15 |
| 19100 | 2 | | | | II-1 | Serp2 | 387923 | 21-May-15 |
| 19138 | 2 | | | | II-1 | Serpinb5 | 5268 | 4-May-15 |
| 19149 | 2 | | | | II-1 | Serpinb9d | | |
| 19166 | 2 | | | | II-1 | Sertad3 | 29946 | 4-May-15 |
| 19167 | 2 | | | | II-1 | Sertad4 | 56256 | 4-May-15 |
| 19183 | 2 | | | | II-1 | Setd8 | 387893 | 4-May-15 |
| 19189 | 2 | | | | II-1 | Sez6l | 23544 | 12-May-15 |
| 19190 | 2 | | | | II-1 | Sez6l2 | 26470 | 4-May-15 |
| 19193 | 2 | | | | II-1 | Sf3a2 | 8175 | 4-May-15 |
| 19194 | 2 | | | | II-1 | Sf3a3 | 10946 | 4-May-15 |
| 19196 | 2 | | | | II-1 | Sf3b2 | 10992 | 12-May-15 |
| 19197 | 2 | | | | II-1 | Sf3b3 | 23450 | 4-May-15 |
| 19198 | 2 | | | | II-1 | Sf3b4 | 10262 | 1-Jun-15 |
| 19199 | 2 | | | | II-1 | Sf3b5 | 83443 | 4-May-15 |
| 19200 | 2 | | | | II-1 | Sf3b6 | 51639 | 4-May-15 |
| 19201 | 2 | | | | II-1 | Sfi1 | 9814 | 4-May-15 |
| 19203 | 2 | | | | II-1 | Sfmbt2 | 57713 | 4-May-15 |
| 19207 | 2 | | | | II-1 | Sfrp1 | 6422 | 12-May-15 |
| 19214 | 2 | | | | II-1 | Sft2d3 | 84826 | 4-May-15 |
| 19215 | 2 | | | | II-1 | Sfta2 | 389376 | 4-May-15 |
| 19243 | 2 | | | | II-1 | Sgsm1 | 129049 | 4-May-15 |
| 19245 | 2 | | | | II-1 | Sgsm3 | 27352 | 4-May-15 |
| 19247 | 2 | | | | II-1 | Sgtb | 54557 | 14-May-15 |
| 19254 | 2 | | | | II-1 | Sh2d2a | 9047 | 4-May-15 |
| 19268 | 2 | | | | II-1 | Sh3bp5l | 80851 | 4-May-15 |
| 19275 | 2 | | | | II-1 | Sh3glb2 | 56904 | 4-May-15 |
| 19291 | 2 | | | | II-1 | Shc1 | 6464 | 31-May-15 |
| 19292 | 2 | | | | II-1 | Shc2 | 25759 | 4-May-15 |
| 19293 | 2 | | | | II-1 | Shc3 | 53358 | 12-May-15 |
| 19307 | 2 | | | | II-1 | Shisa7 | 729956 | 4-May-15 |
| 19308 | 2 | | | | II-1 | Shisa9 | 729993 | 12-May-15 |
| 19313 | 2 | | | | II-1 | Shox2 | 6474 | 17-May-15 |
| 19314 | 2 | | | | II-1 | Shpk | 23729 | 4-May-15 |
| 19340 | 2 | | | | II-1 | Sil1 | 64374 | 23-May-15 |
| 19341 | 2 | | | | II-1 | Sim1 | 6492 | 28-May-15 |
| 19342 | 2 | | | | II-1 | Sim2 | 6493 | 28-May-15 |
| 19345 | 2 | | | | II-1 | Sin3b | 23309 | 12-May-15 |
| 19353 | 2 | | | | II-1 | Sirt1 | 23411 | 31-May-15 |
| 19356 | 2 | | | | II-1 | Sirt4 | 23409 | 31-May-15 |
| 19381 | 2 | | | | II-1 | Skint2 | | |
| 19390 | 2 | | | | II-1 | Skiv2l2 | 23517 | 21-May-15 |
| 19391 | 2 | | | | II-1 | Skor1 | 390598 | 4-May-15 |
| 19393 | 2 | | | | II-1 | Skp1a | 6500 | 4-May-15 |
| 19400 | 2 | | | | II-1 | Slamf1 | 6504 | 12-May-15 |
| 19410 | 2 | | | | II-1 | Slc10a4 | 201780 | 4-May-15 |
| 19417 | 2 | | | | II-1 | Slc12a2 | 6558 | 4-May-15 |
| 19420 | 2 | | | | II-1 | Slc12a5 | 57468 | 12-May-15 |
| 19424 | 2 | | | | II-1 | Slc12a9 | 56996 | 4-May-15 |
| 19459 | 2 | | | | II-1 | Slc17a8 | 246213 | 23-May-15 |
| 19474 | 2 | | | | II-1 | Slc1a7 | 6512 | 4-May-15 |
| 19481 | 2 | | | | II-1 | Slc22a14 | 9389 | 4-May-15 |
| 19482 | 2 | | | | II-1 | Slc22a15 | 55356 | 4-May-15 |
| 19483 | 2 | | | | II-1 | Slc22a16 | 85413 | 4-May-15 |
| 19507 | 2 | | | | II-1 | Slc24a2 | 25769 | 4-May-15 |
| 19519 | 2 | | | | II-1 | Slc25a17 | 10478 | 4-May-15 |
| 19521 | 2 | | | | II-1 | Slc25a19 | 60386 | 23-May-15 |
| 19522 | 2 | | | | II-1 | Slc25a2 | 83884 | 4-May-15 |
| 19523 | 2 | | | | II-1 | Slc25a20 | 788 | 12-May-15 |
| 19556 | 2 | | | | II-1 | Slc25a53 | 401612 | 4-May-15 |
| 19558 | 2 | | | | II-1 | Slc26a1 | 10861 | 4-May-15 |
| 19564 | 2 | | | | II-1 | Slc26a5 | 375611 | 4-May-15 |
| 19566 | 2 | | | | II-1 | Slc26a7 | 115111 | 4-May-15 |
| 19575 | 2 | | | | II-1 | Slc28a1 | 9154 | 12-May-15 |
| 19601 | 2 | | | | II-1 | Slc30a6 | 55676 | 4-May-15 |
| 19604 | 2 | | | | II-1 | Slc30a9 | 10463 | 4-May-15 |
| 19605 | 2 | | | | II-1 | Slc31a1 | 1317 | 17-May-15 |
| 19607 | 2 | | | | II-1 | Slc32a1 | 140679 | 4-May-15 |
| 19608 | 2 | | | | II-1 | Slc33a1 | 9197 | 4-May-15 |
| 19609 | 2 | | | | II-1 | Slc34a1 | 6569 | 24-May-15 |
| 19617 | 2 | | | | II-1 | Slc35b1 | 10237 | 21-May-15 |
| 19618 | 2 | | | | II-1 | Slc35b2 | 347734 | 4-May-15 |
| 19619 | 2 | | | | II-1 | Slc35b3 | 51000 | 28-May-15 |
| 19621 | 2 | | | | II-1 | Slc35c1 | 55343 | 28-May-15 |
| 19623 | 2 | | | | II-1 | Slc35d1 | 23169 | 12-May-15 |
| 19626 | 2 | | | | II-1 | Slc35e1 | 79939 | 4-May-15 |
| 19634 | 2 | | | | II-1 | Slc35f5 | 80255 | 4-May-15 |

| | | | | | | | | |
|---|---|---|---|---|---|---|---|---|
| 19648 | 2 | | | | II-1 | Slc38a1 | 81539 | 4-May-15 |
| 19657 | 2 | | | | II-1 | Slc38a8 | 146167 | 4-May-15 |
| 19658 | 2 | | | | II-1 | Slc38a9 | 153129 | 14-May-15 |
| 19664 | 2 | | | | II-1 | Slc39a14 | 23516 | 4-May-15 |
| 19667 | 2 | | | | II-1 | Slc39a4 | 55630 | 10-May-15 |
| 19683 | 2 | | | | II-1 | Slc44a2 | 57153 | 12-May-15 |
| 19688 | 2 | | | | II-1 | Slc45a2 | 51151 | 23-May-15 |
| 19701 | 2 | | | | II-1 | Slc4a2 | 6522 | 21-May-15 |
| 19702 | 2 | | | | II-1 | Slc4a3 | 6508 | 12-May-15 |
| 19715 | 2 | | | | II-1 | Slc5a11 | 115584 | 4-May-15 |
| 19721 | 2 | | | | II-1 | Slc5a5 | 6528 | 12-May-15 |
| 19759 | 2 | | | | II-1 | Slc7a7 | 9056 | 23-May-15 |
| 19773 | 2 | | | | II-1 | Slc9a6 | 10479 | 7-Jun-15 |
| 19774 | 2 | | | | II-1 | Slc9a7 | 84679 | 4-May-15 |
| 19784 | 2 | | | | II-1 | Slco1b2 | | |
| 19785 | 2 | | | | II-1 | Slco1c1 | 53919 | 4-May-15 |
| 19794 | 2 | | | | II-1 | Slco6d1 | | |
| 19812 | 2 | | | | II-1 | Slitrk3 | 22865 | 4-May-15 |
| 19813 | 2 | | | | II-1 | Slitrk4 | 139065 | 4-May-15 |
| 19814 | 2 | | | | II-1 | Slitrk5 | 26050 | 4-May-15 |
| 19815 | 2 | | | | II-1 | Slitrk6 | 84189 | 23-May-15 |
| 19824 | 2 | | | | II-1 | Slurp1 | 57152 | 4-May-15 |
| 19826 | 2 | | | | II-1 | Slx1b | 79008 | 4-May-15 |
| 19829 | 2 | | | | II-1 | Slxl1 | | |
| 19831 | 2 | | | | II-1 | Smad1 | 4086 | 7-Jun-15 |
| 19833 | 2 | | | | II-1 | Smad3 | 4088 | 24-May-15 |
| 19834 | 2 | | | | II-1 | Smad4 | 4089 | 23-May-15 |
| 19835 | 2 | | | | II-1 | Smad5 | 4090 | 12-May-15 |
| 19842 | 2 | | | | II-1 | Smarca1 | 6594 | 2-Jun-15 |
| 19846 | 2 | | | | II-1 | Smarca5-ps | | |
| 19850 | 2 | | | | II-1 | Smarcc1 | 6599 | 4-May-15 |
| 19851 | 2 | | | | II-1 | Smarcc2 | 6601 | 4-May-15 |
| 19852 | 2 | | | | II-1 | Smarcd1 | 6602 | 4-May-15 |
| 19853 | 2 | | | | II-1 | Smarcd2 | 6603 | 4-May-15 |
| 19861 | 2 | | | | II-1 | Smc4 | 10051 | 4-May-15 |
| 19868 | 2 | | | | II-1 | Smco4 | 56935 | 4-May-15 |
| 19872 | 2 | | | | II-1 | Smek1 | 55671 | 1-Jun-15 |
| 19873 | 2 | | | | II-1 | Smek2 | 57223 | 4-May-15 |
| 19874 | 2 | | | | II-1 | Smg1 | 23049 | 4-May-15 |
| 19877 | 2 | | | | II-1 | Smg7 | 9887 | 4-May-15 |
| 19878 | 2 | | | | II-1 | Smg8 | 55181 | 4-May-15 |
| 19879 | 2 | | | | II-1 | Smg9 | 56006 | 4-May-15 |
| 19885 | 2 | | | | II-1 | Smim14 | 201895 | 4-May-15 |
| 19886 | 2 | | | | II-1 | Smim15 | 643155 | 4-May-15 |
| 19887 | 2 | | | | II-1 | Smim18 | 100507341 | 4-May-15 |
| 19901 | 2 | | | | II-1 | Smn1 | 6606 | 23-May-15 |
| 19908 | 2 | | | | II-1 | Smok3a | | |
| 19909 | 2 | | | | II-1 | Smok3b | | |
| 19910 | 2 | | | | II-1 | Smok4a | | |
| 19911 | 2 | | | | II-1 | Smox | 54498 | 4-May-15 |
| 19928 | 2 | | | | II-1 | Smurf1 | 57154 | 31-May-15 |
| 19933 | 2 | | | | II-1 | Smyd4 | 114826 | 4-May-15 |
| 19934 | 2 | | | | II-1 | Smyd5 | 10322 | 4-May-15 |
| 19941 | 2 | | | | II-1 | Snap47 | 116841 | 21-May-15 |
| 19948 | 2 | | | | II-1 | Snapin | 23557 | 21-May-15 |
| 19995 | 2 | | | | II-1 | Snora62 | 6044 | 4-May-15 |
| 20008 | 2 | | | | II-1 | Snord11 | 692058 | 4-May-15 |
| 20026 | 2 | | | | II-1 | Snord1b | 677849 | 4-May-15 |
| 20087 | 2 | | | | II-1 | Snrnp48 | 154007 | 4-May-15 |
| 20093 | 2 | | | | II-1 | Snrpc | 6631 | 7-Jun-15 |
| 20097 | 2 | | | | II-1 | Snrpe | 6635 | 12-May-15 |
| 20115 | 2 | | | | II-1 | Snx14 | 57231 | 29-May-15 |
| 20116 | 2 | | | | II-1 | Snx15 | 29907 | 4-May-15 |
| 20117 | 2 | | | | II-1 | Snx16 | 64089 | 4-May-15 |
| 20118 | 2 | | | | II-1 | Snx17 | 9784 | 12-May-15 |
| 20119 | 2 | | | | II-1 | Snx18 | 112574 | 4-May-15 |
| 20120 | 2 | | | | II-1 | Snx19 | 399979 | 12-May-15 |
| 20121 | 2 | | | | II-1 | Snx2 | 6643 | 4-May-15 |
| 20122 | 2 | | | | II-1 | Snx20 | 124460 | 4-May-15 |
| 20123 | 2 | | | | II-1 | Snx21 | 90203 | 4-May-15 |
| 20127 | 2 | | | | II-1 | Snx27 | 81609 | 4-May-15 |
| 20128 | 2 | | | | II-1 | Snx29 | 92017 | 4-May-15 |
| 20134 | 2 | | | | II-1 | Snx4 | 8723 | 4-May-15 |
| 20137 | 2 | | | | II-1 | Snx7 | 51375 | 4-May-15 |
| 20139 | 2 | | | | II-1 | Snx9 | 51429 | 4-May-15 |
| 20140 | 2 | | | | II-1 | Soat1 | 6646 | 4-May-15 |
| 20149 | 2 | | | | II-1 | Socs7 | 30837 | 7-Jun-15 |
| 20150 | 2 | | | | II-1 | Sod1 | 6647 | 31-May-15 |
| 20161 | 2 | | | | II-1 | Sorbs3 | 10174 | 12-May-15 |
| 20169 | 2 | | | | II-1 | Sos2 | 6655 | 4-May-15 |
| 20177 | 2 | | | | II-1 | Sox10 | 6663 | 31-May-15 |
| 20188 | 2 | | | | II-1 | Sox3 | 6658 | 23-May-15 |
| 20191 | 2 | | | | II-1 | Sox5 | 6660 | 12-May-15 |
| 20214 | 2 | | | | II-1 | Spaca5 | 389852 | 4-May-15 |
| 20221 | 2 | | | | II-1 | Spag17 | 200162 | 12-May-15 |
| 20222 | 2 | | | | II-1 | Spag4 | 6676 | 4-May-15 |
| 20223 | 2 | | | | II-1 | Spag5 | 10615 | 4-May-15 |
| 20226 | 2 | | | | II-1 | Spag8 | 26206 | 4-May-15 |
| 20227 | 2 | | | | II-1 | Spag9 | 9043 | 4-May-15 |
| 20228 | 2 | | | | II-1 | Spam1 | 6677 | 12-May-15 |
| 20229 | 2 | | | | II-1 | Sparc | 6678 | 17-May-15 |
| 20238 | 2 | | | | II-1 | Spata2 | 9825 | 12-May-15 |
| 20239 | 2 | | | | II-1 | Spata20 | 64847 | 4-May-15 |
| 20240 | 2 | | | | II-1 | Spata21 | 374955 | 4-May-15 |
| 20241 | 2 | | | | II-1 | Spata22 | 84690 | 4-May-15 |
| 20247 | 2 | | | | II-1 | Spata31d1a | | |
| 20248 | 2 | | | | II-1 | Spata31d1b | | |

Fig.22 - 110

| | | | | | | | | |
|---|---|---|---|---|---|---|---|---|
| 20250 | 2 | | | | II-1 | Spata31d1d | | |
| 20251 | 2 | | | | II-1 | Spata32 | 124783 | 21-May-15 |
| 20255 | 2 | | | | II-1 | Spata5 | 166378 | 4-May-15 |
| 20262 | 2 | | | | II-1 | Spats1 | 221409 | 4-May-15 |
| 20263 | 2 | | | | II-1 | Spats2 | 65244 | 12-May-15 |
| 20269 | 2 | | | | II-1 | Spcs3 | 60559 | 4-May-15 |
| 20271 | 2 | | | | II-1 | Spdl1 | 54908 | 4-May-15 |
| 20277 | 2 | | | | II-1 | Speer2 | | |
| 20278 | 2 | | | | II-1 | Speer3 | | |
| 20279 | 2 | | | | II-1 | Speer4a | | |
| 20280 | 2 | | | | II-1 | Speer4b | | |
| 20281 | 2 | | | | II-1 | Speer4c | | |
| 20282 | 2 | | | | II-1 | Speer4d | | |
| 20283 | 2 | | | | II-1 | Speer4e | | |
| 20286 | 2 | | | | II-1 | Speer6-ps1 | | |
| 20287 | 2 | | | | II-1 | Speer7-ps1 | | |
| 20297 | 2 | | | | II-1 | Spg11 | 80208 | 23-May-15 |
| 20300 | 2 | | | | II-1 | Spg7 | 6687 | 23-May-15 |
| 20309 | 2 | | | | II-1 | Spin1 | 10927 | 7-Jun-15 |
| 20312 | 2 | | | | II-1 | Spin2-ps1 | | |
| 20313 | 2 | | | | II-1 | Spin4 | 139886 | 4-May-15 |
| 20324 | 2 | | | | II-1 | Spink7 | 84651 | 12-May-15 |
| 20337 | 2 | | | | II-1 | Spns2 | 124976 | 4-May-15 |
| 20351 | 2 | | | | II-1 | Sppl2c | 162540 | 12-May-15 |
| 20353 | 2 | | | | II-1 | Spr | 6697 | 7-Jun-15 |
| 20354 | 2 | | | | II-1 | Spred1 | 161742 | 23-May-15 |
| 20355 | 2 | | | | II-1 | Spred2 | 200734 | 2-Jun-15 |
| 20370 | 2 | | | | II-1 | Sprr2k | | |
| 20377 | 2 | | | | II-1 | Spry4 | 81848 | 4-May-15 |
| 20392 | 2 | | | | II-1 | Sptlc1 | 10558 | 23-May-15 |
| 20394 | 2 | | | | II-1 | Sptlc3 | 55304 | 4-May-15 |
| 20404 | 2 | | | | II-1 | Src | 6714 | 17-May-15 |
| 20406 | 2 | | | | II-1 | Srcrb4d | 136853 | 12-May-15 |
| 20413 | 2 | | | | II-1 | Srek1ip1 | 285672 | 12-May-15 |
| 20414 | 2 | | | | II-1 | Srf | 6722 | 4-May-15 |
| 20415 | 2 | | | | II-1 | Srfbp1 | 153443 | 4-May-15 |
| 20427 | 2 | | | | II-1 | Srp54b | | |
| 20431 | 2 | | | | II-1 | Srp9 | 6726 | 12-May-15 |
| 20432 | 2 | | | | II-1 | Srpk1 | 6732 | 17-May-15 |
| 20437 | 2 | | | | II-1 | Srpx | 8406 | 21-May-15 |
| 20445 | 2 | | | | II-1 | Srrm4os | | |
| 20448 | 2 | | | | II-1 | Srsf10 | 10772 | 1-Jun-15 |
| 20451 | 2 | | | | II-1 | Srsf2 | 6427 | 31-May-15 |
| 20453 | 2 | | | | II-1 | Srsf4 | 6429 | 4-May-15 |
| 20456 | 2 | | | | II-1 | Srsf7 | 6432 | 1-Jun-15 |
| 20457 | 2 | | | | II-1 | Srsf9 | 8683 | 31-May-15 |
| 20458 | 2 | | | | II-1 | Srxn1 | 140809 | 4-May-15 |
| 20465 | 2 | | | | II-1 | Ssbp3 | 23648 | 12-May-15 |
| 20474 | 2 | | | | II-1 | Sspn | 8082 | 14-May-15 |
| 20477 | 2 | | | | II-1 | Ssr2 | 6746 | 4-May-15 |
| 20479 | 2 | | | | II-1 | Ssr4 | 6748 | 23-May-15 |
| 20481 | 2 | | | | II-1 | Sssca1 | 10534 | 12-May-15 |
| 20491 | 2 | | | | II-1 | Ssu72 | 29101 | 4-May-15 |
| 20495 | 2 | | | | II-1 | Ssxb10 | | |
| 20498 | 2 | | | | II-1 | Ssxb5 | | |
| 20499 | 2 | | | | II-1 | Ssxb6 | | |
| 20522 | 2 | | | | II-1 | St8sia1 | 6489 | 4-May-15 |
| 20523 | 2 | | | | II-1 | St8sia2 | 8128 | 4-May-15 |
| 20539 | 2 | | | | II-1 | Stambp | 10617 | 23-May-15 |
| 20548 | 2 | | | | II-1 | Stard3nl | 83930 | 4-May-15 |
| 20549 | 2 | | | | II-1 | Stard4 | 134429 | 12-May-15 |
| 20552 | 2 | | | | II-1 | Stard7 | 56910 | 4-May-15 |
| 20553 | 2 | | | | II-1 | Stard8 | 9754 | 4-May-15 |
| 20555 | 2 | | | | II-1 | Stat2 | 6773 | 28-May-15 |
| 20558 | 2 | | | | II-1 | Stat5a | 6776 | 31-May-15 |
| 20559 | 2 | | | | II-1 | Stat5b | 6777 | 24-May-15 |
| 20561 | 2 | | | | II-1 | Stau1 | 6780 | 4-May-15 |
| 20562 | 2 | | | | II-1 | Stau2 | 27067 | 4-May-15 |
| 20577 | 2 | | | | II-1 | Stip1 | 10963 | 7-Jun-15 |
| 20578 | 2 | | | | II-1 | Stk10 | 6793 | 4-May-15 |
| 20581 | 2 | | | | II-1 | Stk16 | 8576 | 28-May-15 |
| 20586 | 2 | | | | II-1 | Stk3 | 6788 | 7-Jun-15 |
| 20587 | 2 | | | | II-1 | Stk31 | 56164 | 4-May-15 |
| 20593 | 2 | | | | II-1 | Stk36 | 27148 | 4-May-15 |
| 20595 | 2 | | | | II-1 | Stk38l | 23012 | 4-May-15 |
| 20598 | 2 | | | | II-1 | Stk40 | 83931 | 4-May-15 |
| 20599 | 2 | | | | II-1 | Stmn1 | 3925 | 24-May-15 |
| 20607 | 2 | | | | II-1 | Stoml2 | 30968 | 4-May-15 |
| 20619 | 2 | | | | II-1 | Stradb | 55437 | 4-May-15 |
| 20621 | 2 | | | | II-1 | Strbp | 55342 | 4-May-15 |
| 20628 | 2 | | | | II-1 | Stt3a | 3703 | 4-May-15 |
| 20629 | 2 | | | | II-1 | Stt3b | 201595 | 4-May-15 |
| 20630 | 2 | | | | II-1 | Stub1 | 10273 | 12-May-15 |
| 20634 | 2 | | | | II-1 | Stx17 | 55014 | 21-May-15 |
| 20635 | 2 | | | | II-1 | Stx18 | 53407 | 4-May-15 |
| 20642 | 2 | | | | II-1 | Stx5a | 6811 | 4-May-15 |
| 20643 | 2 | | | | II-1 | Stx6 | 10228 | 12-May-15 |
| 20644 | 2 | | | | II-1 | Stx7 | 8417 | 4-May-15 |
| 20645 | 2 | | | | II-1 | Stx8 | 9482 | 10-May-15 |
| 20649 | 2 | | | | II-1 | Stxbp3b | | |
| 20650 | 2 | | | | II-1 | Stxbp4 | 252983 | 4-May-15 |
| 20651 | 2 | | | | II-1 | Stxbp5 | 134957 | 4-May-15 |
| 20653 | 2 | | | | II-1 | Stxbp6 | 29091 | 12-May-15 |
| 20654 | 2 | | | | II-1 | Styk1 | 55359 | 31-May-15 |
| 20656 | 2 | | | | II-1 | Styxl1 | 51657 | 12-May-15 |
| 20657 | 2 | | | | II-1 | Sub1 | 10923 | 28-May-15 |
| 20665 | 2 | | | | II-1 | Sugct | 79783 | 12-May-15 |

| | | | | | | | | |
|---|---|---|---|---|---|---|---|---|
| 20668 | 2 | | | | II-1 | Sugt1 | 10910 | 28-May-15 |
| 20679 | 2 | | | | II-1 | Sult2a3 | | |
| 20690 | 2 | | | | II-1 | Sumf2 | 25870 | 4-May-15 |
| 20695 | 2 | | | | II-1 | Sun2 | 25777 | 17-May-15 |
| 20697 | 2 | | | | II-1 | Sun5 | 140732 | 4-May-15 |
| 20700 | 2 | | | | II-1 | Supt20 | | |
| 20702 | 2 | | | | II-1 | Supt4a | | |
| 20704 | 2 | | | | II-1 | Supt6 | | |
| 20705 | 2 | | | | II-1 | Supt7l | 9913 | 4-May-15 |
| 20706 | 2 | | | | II-1 | Supv3l1 | 6832 | 4-May-15 |
| 20707 | 2 | | | | II-1 | Surf1 | 6834 | 23-May-15 |
| 20708 | 2 | | | | II-1 | Surf2 | 6835 | 4-May-15 |
| 20709 | 2 | | | | II-1 | Surf4 | 6836 | 4-May-15 |
| 20710 | 2 | | | | II-1 | Surf6 | 6838 | 31-May-15 |
| 20718 | 2 | | | | II-1 | Suv420h1 | 51111 | 12-May-15 |
| 20720 | 2 | | | | II-1 | Suz12 | 23512 | 4-May-15 |
| 20723 | 2 | | | | II-1 | Sv2c | 22987 | 4-May-15 |
| 20724 | 2 | | | | II-1 | Sva | | |
| 20726 | 2 | | | | II-1 | Sval2 | | |
| 20728 | 2 | | | | II-1 | Svep1 | 79987 | 12-May-15 |
| 20732 | 2 | | | | II-1 | Svopl | 136306 | 4-May-15 |
| 20734 | 2 | | | | II-1 | Svs2 | | |
| 20735 | 2 | | | | II-1 | Svs3a | | |
| 20741 | 2 | | | | II-1 | Swi5 | 375757 | 4-May-15 |
| 20743 | 2 | | | | II-1 | Swt1 | 54823 | 4-May-15 |
| 20753 | 2 | | | | II-1 | Sycp2 | 10388 | 4-May-15 |
| 20754 | 2 | | | | II-1 | Sycp3 | 50511 | 4-May-15 |
| 20758 | 2 | | | | II-1 | Syk | 6850 | 10-May-15 |
| 20768 | 2 | | | | II-1 | Syndig1l | 646658 | 4-May-15 |
| 20769 | 2 | | | | II-1 | Syne1 | 23345 | 23-May-15 |
| 20779 | 2 | | | | II-1 | Synj2 | 8871 | 4-May-15 |
| 20787 | 2 | | | | II-1 | Syp | 6855 | 4-May-15 |
| 20815 | 2 | | | | II-1 | Szt2 | 23334 | 12-May-15 |
| 20816 | 2 | | | | II-1 | T | 6862 | 4-May-15 |
| 20817 | 2 | | | | II-1 | T2 | | |
| 20818 | 2 | | | | II-1 | Taar1 | 134864 | 7-Jun-15 |
| 20820 | 2 | | | | II-1 | Taar3 | 9288 | 4-May-15 |
| 20840 | 2 | | | | II-1 | Tacc2 | 10579 | 12-May-15 |
| 20844 | 2 | | | | II-1 | Tacr2 | 6865 | 12-May-15 |
| 20848 | 2 | | | | II-1 | Tada2a | 6871 | 1-Jun-15 |
| 20850 | 2 | | | | II-1 | Tada3 | 10474 | 4-May-15 |
| 20851 | 2 | | | | II-1 | Taf1 | 6872 | 23-May-15 |
| 20853 | 2 | | | | II-1 | Taf11 | 6882 | 4-May-15 |
| 20863 | 2 | | | | II-1 | Taf4a | 6874 | 12-May-15 |
| 20864 | 2 | | | | II-1 | Taf4b | 6875 | 16-Jun-15 |
| 20866 | 2 | | | | II-1 | Taf5l | 27097 | 4-May-15 |
| 20868 | 2 | | | | II-1 | Taf6l | 10629 | 4-May-15 |
| 20873 | 2 | | | | II-1 | Taf9b | 51616 | 4-May-15 |
| 20874 | 2 | | | | II-1 | Tagap | 117289 | 4-May-15 |
| 20887 | 2 | | | | II-1 | Tank | 10010 | 4-May-15 |
| 20889 | 2 | | | | II-1 | Taok2 | 9344 | 4-May-15 |
| 20890 | 2 | | | | II-1 | Taok3 | 51347 | 12-May-15 |
| 20891 | 2 | | | | II-1 | Tap1 | 6890 | 13-Jun-15 |
| 20894 | 2 | | | | II-1 | Tapbpl | 55080 | 4-May-15 |
| 20895 | 2 | | | | II-1 | Tapt1 | 202018 | 4-May-15 |
| 20900 | 2 | | | | II-1 | Tars2 | 80222 | 4-May-15 |
| 20901 | 2 | | | | II-1 | Tarsl2 | 123283 | 4-May-15 |
| 20903 | 2 | | | | II-1 | Tas1r2 | 80834 | 7-Jun-15 |
| 20904 | 2 | | | | II-1 | Tas1r3 | 83756 | 4-May-15 |
| 20907 | 2 | | | | II-1 | Tas2r104 | | |
| 20921 | 2 | | | | II-1 | Tas2r120 | | |
| 20945 | 2 | | | | II-1 | Tax1bp1 | 8887 | 12-May-15 |
| 20946 | 2 | | | | II-1 | Tax1bp3 | 30851 | 1-Jun-15 |
| 20951 | 2 | | | | II-1 | Tbc1d10b | 26000 | 4-May-15 |
| 20952 | 2 | | | | II-1 | Tbc1d10c | 374403 | 12-May-15 |
| 20955 | 2 | | | | II-1 | Tbc1d14 | 57533 | 21-May-15 |
| 20956 | 2 | | | | II-1 | Tbc1d15 | 64786 | 4-May-15 |
| 20957 | 2 | | | | II-1 | Tbc1d16 | 125058 | 4-May-15 |
| 20960 | 2 | | | | II-1 | Tbc1d2 | 55357 | 4-May-15 |
| 20962 | 2 | | | | II-1 | Tbc1d21 | 161514 | 12-May-15 |
| 20963 | 2 | | | | II-1 | Tbc1d22a | 25771 | 12-May-15 |
| 20965 | 2 | | | | II-1 | Tbc1d22bos | | |
| 20966 | 2 | | | | II-1 | Tbc1d23 | 55773 | 4-May-15 |
| 20975 | 2 | | | | II-1 | Tbc1d7 | 51256 | 4-May-15 |
| 20980 | 2 | | | | II-1 | Tbca | 6902 | 12-May-15 |
| 20983 | 2 | | | | II-1 | Tbccd1 | 55171 | 4-May-15 |
| 20985 | 2 | | | | II-1 | Tbce | 6905 | 4-May-15 |
| 20986 | 2 | | | | II-1 | Tbcel | 219899 | 4-May-15 |
| 20988 | 2 | | | | II-1 | Tbk1 | 29110 | 4-May-15 |
| 20993 | 2 | | | | II-1 | Tbl3 | 10607 | 4-May-15 |
| 20995 | 2 | | | | II-1 | Tbpl1 | 9519 | 4-May-15 |
| 20997 | 2 | | | | II-1 | Tbr1 | 10716 | 12-May-15 |
| 20998 | 2 | | | | II-1 | Tbrg1 | 84897 | 4-May-15 |
| 21000 | 2 | | | | II-1 | Tbrg4 | 9238 | 4-May-15 |
| 21014 | 2 | | | | II-1 | Tbx6 | 6911 | 12-May-15 |
| 21034 | 2 | | | | II-1 | Tceb3 | 6924 | 4-May-15 |
| 21036 | 2 | | | | II-1 | Tcerg1l | 256536 | 4-May-15 |
| 21037 | 2 | | | | II-1 | Tcf12 | 6938 | 28-May-15 |
| 21045 | 2 | | | | II-1 | Tcf3 | 6929 | 7-Jun-15 |
| 21046 | 2 | | | | II-1 | Tcf4 | 6925 | 7-Jun-15 |
| 21056 | 2 | | | | II-1 | Tcl1b1 | | |
| 21057 | 2 | | | | II-1 | Tcl1b2 | | |
| 21064 | 2 | | | | II-1 | Tcp10a | 6953 | 4-May-15 |
| 21065 | 2 | | | | II-1 | Tcp10b | | |
| 21066 | 2 | | | | II-1 | Tcp10c | | |
| 21072 | 2 | | | | II-1 | Tcta | 6988 | 4-May-15 |
| 21073 | 2 | | | | II-1 | Tcte1 | 202500 | 4-May-15 |

Fig.22 - 111

| | | | | | | | | |
|---|---|---|---|---|---|---|---|---|
| 21080 | 2 | | | | II-1 | Tctn2 | 79867 | 23-May-15 |
| 21089 | 2 | | | | II-1 | Tdpoz2 | | |
| 21090 | 2 | | | | II-1 | Tdpoz3 | | |
| 21099 | 2 | | | | II-1 | Tdrd9 | 122402 | 4-May-15 |
| 21120 | 2 | | | | II-1 | Tekt4 | 150483 | 4-May-15 |
| 21121 | 2 | | | | II-1 | Tekt5 | 146279 | 4-May-15 |
| 21122 | 2 | | | | II-1 | Telo2 | 9894 | 4-May-15 |
| 21123 | 2 | | | | II-1 | Ten1 | 100134934 | 4-May-15 |
| 21126 | 2 | | | | II-1 | Tenm2 | 57451 | 12-May-15 |
| 21127 | 2 | | | | II-1 | Tenm3 | 55714 | 4-May-15 |
| 21140 | 2 | | | | II-1 | Tesk2 | 10420 | 4-May-15 |
| 21141 | 2 | | | | II-1 | Tespa1 | 9840 | 4-May-15 |
| 21148 | 2 | | | | II-1 | Tex12 | 56158 | 4-May-15 |
| 21151 | 2 | | | | II-1 | Tex14 | 56155 | 4-May-15 |
| 21152 | 2 | | | | II-1 | Tex15 | 56154 | 4-May-15 |
| 21153 | 2 | | | | II-1 | Tex16 | | |
| 21155 | 2 | | | | II-1 | Tex19.2 | | |
| 21156 | 2 | | | | II-1 | Tex2 | 55852 | 4-May-15 |
| 21157 | 2 | | | | II-1 | Tex21 | | |
| 21159 | 2 | | | | II-1 | Tex24 | | |
| 21160 | 2 | | | | II-1 | Tex26 | 122046 | 4-May-15 |
| 21162 | 2 | | | | II-1 | Tex264 | 51368 | 4-May-15 |
| 21163 | 2 | | | | II-1 | Tex28 | 1527 | 4-May-15 |
| 21165 | 2 | | | | II-1 | Tex30 | 93081 | 4-May-15 |
| 21168 | 2 | | | | II-1 | Tex36 | 387718 | 4-May-15 |
| 21169 | 2 | | | | II-1 | Tex37 | 200523 | 20-May-15 |
| 21179 | 2 | | | | II-1 | Tfap2e | 339488 | 28-May-15 |
| 21183 | 2 | | | | II-1 | Tfcp2 | 7024 | 4-May-15 |
| 21188 | 2 | | | | II-1 | Tfeb | 7942 | 28-May-15 |
| 21195 | 2 | | | | II-1 | Tfpi | 7035 | 12-May-15 |
| 21196 | 2 | | | | II-1 | Tfpi2 | 7980 | 12-May-15 |
| 21210 | 2 | | | | II-1 | Tgfbr3 | 7049 | 12-May-15 |
| 21231 | 2 | | | | II-1 | Thap1 | 55145 | 17-May-15 |
| 21232 | 2 | | | | II-1 | Thap11 | 57215 | 12-May-15 |
| 21233 | 2 | | | | II-1 | Thap2 | 83591 | 4-May-15 |
| 21236 | 2 | | | | II-1 | Thap6 | 152815 | 4-May-15 |
| 21237 | 2 | | | | II-1 | Thap7 | 80764 | 4-May-15 |
| 21258 | 2 | | | | II-1 | Thoc6 | 79228 | 4-May-15 |
| 21271 | 2 | | | | II-1 | Thumpd1 | 55623 | 12-May-15 |
| 21272 | 2 | | | | II-1 | Thumpd2 | 80745 | 12-May-15 |
| 21273 | 2 | | | | II-1 | Thumpd3 | 25917 | 12-May-15 |
| 21278 | 2 | | | | II-1 | Tiam1 | 7074 | 17-May-15 |
| 21297 | 2 | | | | II-1 | Timm17a | 10440 | 4-May-15 |
| 21298 | 2 | | | | II-1 | Timm17b | 10245 | 4-May-15 |
| 21303 | 2 | | | | II-1 | Timm50 | 92609 | 4-May-15 |
| 21307 | 2 | | | | II-1 | Timm9 | 26520 | 12-May-15 |
| 21316 | 2 | | | | II-1 | Tiparp | 25976 | 4-May-15 |
| 21317 | 2 | | | | II-1 | Tipin | 54962 | 4-May-15 |
| 21321 | 2 | | | | II-1 | Tjp1 | 7082 | 31-May-15 |
| 21340 | 2 | | | | II-1 | Tll1 | 7092 | 4-May-15 |
| 21343 | 2 | | | | II-1 | Tln2 | 83660 | 4-May-15 |
| 21358 | 2 | | | | II-1 | Tlx3 | 30012 | 12-May-15 |
| 21374 | 2 | | | | II-1 | Tm9sf4 | 9777 | 4-May-15 |
| 21375 | 2 | | | | II-1 | Tma16 | 55319 | 4-May-15 |
| 21380 | 2 | | | | II-1 | Tmbim7 | | |
| 21381 | 2 | | | | II-1 | Tmc1 | 117531 | 23-May-15 |
| 21382 | 2 | | | | II-1 | Tmc2 | 117532 | 4-May-15 |
| 21383 | 2 | | | | II-1 | Tmc3 | 342125 | 4-May-15 |
| 21394 | 2 | | | | II-1 | Tmco3 | 55002 | 12-May-15 |
| 21397 | 2 | | | | II-1 | Tmco5b | 100652857 | 4-May-15 |
| 21398 | 2 | | | | II-1 | Tmco6 | 55374 | 4-May-15 |
| 21400 | 2 | | | | II-1 | Tmed10 | 10972 | 12-May-15 |
| 21402 | 2 | | | | II-1 | Tmed2 | 10959 | 4-May-15 |
| 21408 | 2 | | | | II-1 | Tmed8 | 283578 | 12-May-15 |
| 21409 | 2 | | | | II-1 | Tmed9 | 54732 | 4-May-15 |
| 21416 | 2 | | | | II-1 | Tmem106a | 113277 | 12-May-15 |
| 21419 | 2 | | | | II-1 | Tmem107 | 84314 | 12-May-15 |
| 21421 | 2 | | | | II-1 | Tmem109 | 79073 | 4-May-15 |
| 21423 | 2 | | | | II-1 | Tmem110 | 375346 | 4-May-15 |
| 21424 | 2 | | | | II-1 | Tmem115 | 11070 | 4-May-15 |
| 21425 | 2 | | | | II-1 | Tmem116 | 89894 | 4-May-15 |
| 21426 | 2 | | | | II-1 | Tmem117 | 84216 | 4-May-15 |
| 21437 | 2 | | | | II-1 | Tmem129 | 92305 | 23-May-15 |
| 21438 | 2 | | | | II-1 | Tmem130 | 222865 | 4-May-15 |
| 21439 | 2 | | | | II-1 | Tmem131 | 23505 | 4-May-15 |
| 21445 | 2 | | | | II-1 | Tmem132e | 124842 | 4-May-15 |
| 21446 | 2 | | | | II-1 | Tmem134 | 80194 | 4-May-15 |
| 21449 | 2 | | | | II-1 | Tmem138 | 51524 | 23-May-15 |
| 21456 | 2 | | | | II-1 | Tmem147 | 10430 | 4-May-15 |
| 21457 | 2 | | | | II-1 | Tmem14a | 28978 | 4-May-15 |
| 21458 | 2 | | | | II-1 | Tmem14c | 51522 | 4-May-15 |
| 21471 | 2 | | | | II-1 | Tmem163 | 81615 | 4-May-15 |
| 21474 | 2 | | | | II-1 | Tmem167 | 153339 | 4-May-15 |
| 21475 | 2 | | | | II-1 | Tmem167b | 56900 | 4-May-15 |
| 21477 | 2 | | | | II-1 | Tmem169 | 92691 | 4-May-15 |
| 21478 | 2 | | | | II-1 | Tmem17 | 200728 | 4-May-15 |
| 21485 | 2 | | | | II-1 | Tmem176a | 55365 | 4-May-15 |
| 21496 | 2 | | | | II-1 | Tmem181c-ps | | |
| 21506 | 2 | | | | II-1 | Tmem190 | 147744 | 4-May-15 |
| 21520 | 2 | | | | II-1 | Tmem202 | 338949 | 4-May-15 |
| 21522 | 2 | | | | II-1 | Tmem204 | 79652 | 4-May-15 |
| 21525 | 2 | | | | II-1 | Tmem207 | 131920 | 4-May-15 |
| 21534 | 2 | | | | II-1 | Tmem216 | 51259 | 23-May-15 |
| 21535 | 2 | | | | II-1 | Tmem217 | 221468 | 4-May-15 |
| 21542 | 2 | | | | II-1 | Tmem225 | 338661 | 4-May-15 |
| 21544 | 2 | | | | II-1 | Tmem229b | 161145 | 4-May-15 |
| 21552 | 2 | | | | II-1 | Tmem237 | 65062 | 23-May-15 |
| 21555 | 2 | | | | II-1 | Tmem240 | 339453 | 23-May-15 |
| 21558 | 2 | | | | II-1 | Tmem243 | 79161 | 4-May-15 |
| 21562 | 2 | | | | II-1 | Tmem248 | 55069 | 4-May-15 |
| 21563 | 2 | | | | II-1 | Tmem25 | 84866 | 4-May-15 |
| 21576 | 2 | | | | II-1 | Tmem260 | 54916 | 4-May-15 |
| 21586 | 2 | | | | II-1 | Tmem35 | 59353 | 4-May-15 |
| 21591 | 2 | | | | II-1 | Tmem39b | 55116 | 28-May-15 |
| 21596 | 2 | | | | II-1 | Tmem43 | 79188 | 23-May-15 |
| 21597 | 2 | | | | II-1 | Tmem44 | 93109 | 4-May-15 |
| 21614 | 2 | | | | II-1 | Tmem59 | 9528 | 4-May-15 |
| 21615 | 2 | | | | II-1 | Tmem59l | 25789 | 4-May-15 |
| 21616 | 2 | | | | II-1 | Tmem60 | 85025 | 21-May-15 |
| 21618 | 2 | | | | II-1 | Tmem63a | 9725 | 12-May-15 |
| 21620 | 2 | | | | II-1 | Tmem63c | 57156 | 4-May-15 |
| 21625 | 2 | | | | II-1 | Tmem68 | 137695 | 4-May-15 |
| 21627 | 2 | | | | II-1 | Tmem70 | 54968 | 12-May-15 |
| 21631 | 2 | | | | II-1 | Tmem74b | 55321 | 12-May-15 |
| 21636 | 2 | | | | II-1 | Tmem82 | 388595 | 4-May-15 |
| 21642 | 2 | | | | II-1 | Tmem88b | 643965 | 4-May-15 |
| 21646 | 2 | | | | II-1 | Tmem9 | 252839 | 4-May-15 |
| 21649 | 2 | | | | II-1 | Tmem95 | 339168 | 4-May-15 |
| 21650 | 2 | | | | II-1 | Tmem97 | 27346 | 31-May-15 |
| 21654 | 2 | | | | II-1 | Tmf1 | 7110 | 4-May-15 |
| 21655 | 2 | | | | II-1 | Tmie | 259236 | 23-May-15 |
| 21667 | 2 | | | | II-1 | Tmprss11d | 9407 | 4-May-15 |
| 21672 | 2 | | | | II-1 | Tmprss13 | 84000 | 4-May-15 |
| 21680 | 2 | | | | II-1 | Tmprss9 | 360200 | 4-May-15 |
| 21693 | 2 | | | | II-1 | Tmx1 | 81542 | 4-May-15 |
| 21695 | 2 | | | | II-1 | Tmx3 | 54495 | 4-May-15 |
| 21697 | 2 | | | | II-1 | Tnc | 3371 | 7-Jun-15 |
| 21700 | 2 | | | | II-1 | Tnfaip2 | 7127 | 4-May-15 |
| 21704 | 2 | | | | II-1 | Tnfaip8l1 | 126282 | 12-May-15 |
| 21736 | 2 | | | | II-1 | Tnfsf4 | 7292 | 4-May-15 |
| 21738 | 2 | | | | II-1 | Tnfsf9 | 8744 | 4-May-15 |
| 21765 | 2 | | | | II-1 | Tnr | 7143 | 4-May-15 |
| 21766 | 2 | | | | II-1 | Tnrc18 | 84629 | 7-Jun-15 |
| 21778 | 2 | | | | II-1 | Tom1 | 10043 | 4-May-15 |
| 21781 | 2 | | | | II-1 | Tomm20 | 9804 | 12-May-15 |
| 21783 | 2 | | | | II-1 | Tomm22 | 56993 | 4-May-15 |
| 21786 | 2 | | | | II-1 | Tomm40l | 84134 | 4-May-15 |
| 21800 | 2 | | | | II-1 | Topaz1 | 375337 | 4-May-15 |
| 21801 | 2 | | | | II-1 | Topbp1 | 11073 | 4-May-15 |
| 21808 | 2 | | | | II-1 | Tor1b | 27348 | 4-May-15 |
| 21809 | 2 | | | | II-1 | Tor2a | 27433 | 31-May-15 |
| 21810 | 2 | | | | II-1 | Tor3a | 64222 | 12-May-15 |
| 21811 | 2 | | | | II-1 | Tor4a | 54863 | 4-May-15 |
| 21819 | 2 | | | | II-1 | Tpcn1 | 53373 | 12-May-15 |
| 21837 | 2 | | | | II-1 | Tpp2 | 7174 | 4-May-15 |
| 21838 | 2 | | | | II-1 | Tppp | 11076 | 4-May-15 |
| 21845 | 2 | | | | II-1 | Tprkb | 51002 | 4-May-15 |
| 21846 | 2 | | | | II-1 | Tprn | 286262 | 4-May-15 |
| 21854 | 2 | | | | II-1 | Tpx2 | 22974 | 4-May-15 |
| 21863 | 2 | | | | II-1 | Traf3ip1 | 26146 | 4-May-15 |
| 21871 | 2 | | | | II-1 | Traip | 10293 | 4-May-15 |
| 21875 | 2 | | | | II-1 | Tram1l1 | 133022 | 4-May-15 |
| 21880 | 2 | | | | II-1 | Trappc1 | 58485 | 4-May-15 |
| 21881 | 2 | | | | II-1 | Trappc10 | 7109 | 4-May-15 |
| 21882 | 2 | | | | II-1 | Trappc11 | 60684 | 12-May-15 |
| 21883 | 2 | | | | II-1 | Trappc12 | 51112 | 14-May-15 |
| 21884 | 2 | | | | II-1 | Trappc13 | 80006 | 4-May-15 |
| 21885 | 2 | | | | II-1 | Trappc2 | 6399 | 23-May-15 |
| 21886 | 2 | | | | II-1 | Trappc2l | 51693 | 4-May-15 |
| 21889 | 2 | | | | II-1 | Trappc4 | 51399 | 4-May-15 |
| 21894 | 2 | | | | II-1 | Trappc9 | 83696 | 4-May-15 |
| 21895 | 2 | | | | II-1 | Trat1 | 50852 | 4-May-15 |
| 21912 | 2 | | | | II-1 | Trhr | 7201 | 12-May-15 |
| 21913 | 2 | | | | II-1 | Trhr2 | | |
| 21923 | 2 | | | | II-1 | Trim13 | 10206 | 12-May-15 |
| 21930 | 2 | | | | II-1 | Trim23 | 373 | 2-Jun-15 |
| 21934 | 2 | | | | II-1 | Trim27 | 5987 | 2-Jun-15 |
| 21935 | 2 | | | | II-1 | Trim28 | 10155 | 4-May-15 |
| 21943 | 2 | | | | II-1 | Trim32 | 22954 | 23-May-15 |
| 21945 | 2 | | | | II-1 | Trim34a | | |
| 21946 | 2 | | | | II-1 | Trim34b | | |
| 21955 | 2 | | | | II-1 | Trim43a | 129868 | 4-May-15 |
| 21956 | 2 | | | | II-1 | Trim43b | 653192 | 4-May-15 |
| 21957 | 2 | | | | II-1 | Trim43c | | |
| 21960 | 2 | | | | II-1 | Trim46 | 80128 | 4-May-15 |
| 21972 | 2 | | | | II-1 | Trim62 | 55223 | 4-May-15 |
| 21979 | 2 | | | | II-1 | Trim7 | 81786 | 4-May-15 |
| 21986 | 2 | | | | II-1 | Triml2 | 205860 | 7-Jun-15 |
| 21987 | 2 | | | | II-1 | Trio | 7204 | 12-May-15 |
| 21988 | 2 | | | | II-1 | Triobp | 11078 | 23-May-15 |
| 22008 | 2 | | | | II-1 | Trmt44 | 152992 | 12-May-15 |
| 22009 | 2 | | | | II-1 | Trmt5 | 57570 | 4-May-15 |
| 22010 | 2 | | | | II-1 | Trmt6 | 51605 | 4-May-15 |
| 22012 | 2 | | | | II-1 | Trmt61b | 55006 | 4-May-15 |
| 22018 | 2 | | | | II-1 | Troap | 10024 | 4-May-15 |
| 22021 | 2 | | | | II-1 | Trp53bp1 | | |
| 22022 | 2 | | | | II-1 | Trp53bp2 | | |
| 22024 | 2 | | | | II-1 | Trp53i11 | | |
| 22032 | 2 | | | | II-1 | Trpa1 | 8989 | 7-Jun-15 |
| 22033 | 2 | | | | II-1 | Trpc1 | 7220 | 10-May-15 |
| 22043 | 2 | | | | II-1 | Trpm1 | 4308 | 12-May-15 |
| 22050 | 2 | | | | II-1 | Trpm8 | 79054 | 24-May-15 |
| 22052 | 2 | | | | II-1 | Trpt1 | 83707 | 4-May-15 |

Fig.22 - 112

| | | | | | | | | |
|---|---|---|---|---|---|---|---|---|
| 22061 | 2 | | | | II-1 | Trub2 | 26995 | 12-May-15 |
| 22072 | 2 | | | | II-1 | Tsen15 | 116461 | 4-May-15 |
| 22076 | 2 | | | | II-1 | Tsfm | 10102 | 4-May-15 |
| 22077 | 2 | | | | II-1 | Tsg101 | 7251 | 1-Jun-15 |
| 22081 | 2 | | | | II-1 | Tshb | 7252 | 31-May-15 |
| 22082 | 2 | | | | II-1 | Tshr | 7253 | 12-May-15 |
| 22092 | 2 | | | | II-1 | Tsnaxip1 | 55815 | 4-May-15 |
| 22118 | 2 | | | | II-1 | Tspyl2 | 64061 | 4-May-15 |
| 22120 | 2 | | | | II-1 | Tspyl4 | 23270 | 12-May-15 |
| 22126 | 2 | | | | II-1 | Tssc1 | 7260 | 4-May-15 |
| 22141 | 2 | | | | II-1 | Ttbk2 | 146057 | 31-May-15 |
| 22145 | 2 | | | | II-1 | Ttc14 | 151613 | 4-May-15 |
| 22146 | 2 | | | | II-1 | Ttc16 | 158248 | 4-May-15 |
| 22148 | 2 | | | | II-1 | Ttc18 | 118491 | 4-May-15 |
| 22150 | 2 | | | | II-1 | Ttc21a | 199223 | 4-May-15 |
| 22152 | 2 | | | | II-1 | Ttc22 | 55001 | 4-May-15 |
| 22154 | 2 | | | | II-1 | Ttc23l | 153657 | 22-May-15 |
| 22155 | 2 | | | | II-1 | Ttc24 | 164118 | 4-May-15 |
| 22163 | 2 | | | | II-1 | Ttc30a2 | | |
| 22176 | 2 | | | | II-1 | Ttc5 | 91875 | 4-May-15 |
| 22177 | 2 | | | | II-1 | Ttc7 | 57217 | 21-May-15 |
| 22178 | 2 | | | | II-1 | Ttc7b | 145567 | 4-May-15 |
| 22179 | 2 | | | | II-1 | Ttc8 | 123016 | 23-May-15 |
| 22182 | 2 | | | | II-1 | Ttc9c | 283237 | 4-May-15 |
| 22183 | 2 | | | | II-1 | Ttf1 | 7270 | 7-Jun-15 |
| 22185 | 2 | | | | II-1 | Tti1 | 9675 | 12-May-15 |
| 22187 | 2 | | | | II-1 | Ttk | 7272 | 4-May-15 |
| 22199 | 2 | | | | II-1 | Ttll7 | 79739 | 4-May-15 |
| 22232 | 2 | | | | II-1 | Tubgcp3 | 10426 | 4-May-15 |
| 22234 | 2 | | | | II-1 | Tubgcp5 | 114791 | 4-May-15 |
| 22237 | 2 | | | | II-1 | Tuft1 | 7286 | 12-May-15 |
| 22241 | 2 | | | | II-1 | Tulp3 | 7289 | 28-May-15 |
| 22242 | 2 | | | | II-1 | Tulp4 | 56995 | 28-May-15 |
| 22250 | 2 | | | | II-1 | Tvp23b | 51030 | 4-May-15 |
| 22252 | 2 | | | | II-1 | Twf2 | 11344 | 3-May-15 |
| 22257 | 2 | | | | II-1 | Txk | 7294 | 28-May-15 |
| 22266 | 2 | | | | II-1 | Txndc16 | 57544 | 4-May-15 |
| 22270 | 2 | | | | II-1 | Txndc8 | 255220 | 4-May-15 |
| 22271 | 2 | | | | II-1 | Txndc9 | 10190 | 4-May-15 |
| 22272 | 2 | | | | II-1 | Txnip | 10628 | 12-May-15 |
| 22275 | 2 | | | | II-1 | Txnl4b | 54957 | 4-May-15 |
| 22276 | 2 | | | | II-1 | Txnrd1 | 7296 | 14-May-15 |
| 22277 | 2 | | | | II-1 | Txnrd2 | 10587 | 4-May-15 |
| 22295 | 2 | | | | II-1 | U90926 | | |
| 22301 | 2 | | | | II-1 | Uba2 | 10054 | 23-May-15 |
| 22302 | 2 | | | | II-1 | Uba3 | 9039 | 23-May-15 |
| 22304 | 2 | | | | II-1 | Uba52 | 7311 | 1-Jun-15 |
| 22309 | 2 | | | | II-1 | Ubald1 | 124402 | 4-May-15 |
| 22313 | 2 | | | | II-1 | Ubap2 | 55833 | 4-May-15 |
| 22316 | 2 | | | | II-1 | Ubash3b | 84959 | 4-May-15 |
| 22317 | 2 | | | | II-1 | Ubb | 7314 | 29-May-15 |
| 22327 | 2 | | | | II-1 | Ube2d3 | 7323 | 4-May-15 |
| 22328 | 2 | | | | II-1 | Ube2dnl1 | | |
| 22329 | 2 | | | | II-1 | Ube2dnl2 | | |
| 22330 | 2 | | | | II-1 | Ube2e1 | 7324 | 4-May-15 |
| 22331 | 2 | | | | II-1 | Ube2e2 | 7325 | 12-May-15 |
| 22332 | 2 | | | | II-1 | Ube2e3 | 10477 | 4-May-15 |
| 22334 | 2 | | | | II-1 | Ube2g1 | 7326 | 29-May-15 |
| 22335 | 2 | | | | II-1 | Ube2g2 | 7327 | 12-May-15 |
| 22336 | 2 | | | | II-1 | Ube2h | 7328 | 12-May-15 |
| 22337 | 2 | | | | II-1 | Ube2i | 7329 | 17-May-15 |
| 22338 | 2 | | | | II-1 | Ube2j1 | 51465 | 3-May-15 |
| 22344 | 2 | | | | II-1 | Ube2n | 7334 | 4-May-15 |
| 22345 | 2 | | | | II-1 | Ube2o | 63893 | 4-May-15 |
| 22351 | 2 | | | | II-1 | Ube2t | 29089 | 4-May-15 |
| 22354 | 2 | | | | II-1 | Ube2v2 | 7336 | 4-May-15 |
| 22355 | 2 | | | | II-1 | Ube2w | 55284 | 4-May-15 |
| 22356 | 2 | | | | II-1 | Ube2z | 65264 | 4-May-15 |
| 22362 | 2 | | | | II-1 | Ubfd1 | 56061 | 12-May-15 |
| 22369 | 2 | | | | II-1 | Ublcp1 | 134510 | 28-May-15 |
| 22371 | 2 | | | | II-1 | Ubn2 | 254048 | 4-May-15 |
| 22372 | 2 | | | | II-1 | Ubox5 | 22888 | 4-May-15 |
| 22374 | 2 | | | | II-1 | Ubqln1 | 29979 | 21-May-15 |
| 22375 | 2 | | | | II-1 | Ubqln2 | 29978 | 23-May-15 |
| 22376 | 2 | | | | II-1 | Ubqln3 | 50613 | 4-May-15 |
| 22379 | 2 | | | | II-1 | Ubr1 | 197131 | 12-May-15 |
| 22380 | 2 | | | | II-1 | Ubr2 | 23304 | 4-May-15 |
| 22381 | 2 | | | | II-1 | Ubr3 | 130507 | 4-May-15 |
| 22382 | 2 | | | | II-1 | Ubr4 | 23352 | 12-May-15 |
| 22383 | 2 | | | | II-1 | Ubr5 | 51366 | 12-May-15 |
| 22384 | 2 | | | | II-1 | Ubr7 | 55148 | 4-May-15 |
| 22389 | 2 | | | | II-1 | Ubxn1 | 51035 | 31-May-15 |
| 22397 | 2 | | | | II-1 | Ubxn8 | 7993 | 4-May-15 |
| 22418 | 2 | | | | II-1 | Ufm1 | 51569 | 4-May-15 |
| 22419 | 2 | | | | II-1 | Ufsp1 | 402682 | 4-May-15 |
| 22425 | 2 | | | | II-1 | Ugp2 | 7360 | 4-May-15 |
| 22435 | 2 | | | | II-1 | Ugt2a2 | 574537 | 12-May-15 |
| 22436 | 2 | | | | II-1 | Ugt2a3 | 79799 | 28-May-15 |
| 22452 | 2 | | | | II-1 | Uimc1 | 51720 | 4-May-15 |
| 22453 | 2 | | | | II-1 | Ulbp1 | 80329 | 4-May-15 |
| 22456 | 2 | | | | II-1 | Ulk3 | 25989 | 21-May-15 |
| 22460 | 2 | | | | II-1 | Umps | 7372 | 12-May-15 |
| 22466 | 2 | | | | II-1 | Unc13d | 201294 | 23-May-15 |
| 22467 | 2 | | | | II-1 | Unc45a | 55898 | 4-May-15 |
| 22475 | 2 | | | | II-1 | Unc79 | 57578 | 4-May-15 |
| 22476 | 2 | | | | II-1 | Unc80 | 285175 | 4-May-15 |
| 22486 | 2 | | | | II-1 | Upf2 | 26019 | 12-May-15 |
| 22487 | 2 | | | | II-1 | Upf3a | 65110 | 4-May-15 |
| 22488 | 2 | | | | II-1 | Upf3b | 65109 | 23-May-15 |
| 22515 | 2 | | | | II-1 | Uroc1 | 131669 | 14-May-15 |
| 22520 | 2 | | | | II-1 | Usf1 | 7391 | 4-May-15 |
| 22521 | 2 | | | | II-1 | Usf2 | 7392 | 17-May-15 |
| 22523 | 2 | | | | II-1 | Ush1g | 124590 | 23-May-15 |
| 22529 | 2 | | | | II-1 | Usp10 | 9100 | 7-Jun-15 |
| 22535 | 2 | | | | II-1 | Usp16 | 10600 | 7-Jun-15 |
| 22536 | 2 | | | | II-1 | Usp17la | | |
| 22537 | 2 | | | | II-1 | Usp17lb | | |
| 22538 | 2 | | | | II-1 | Usp17lc | | |
| 22547 | 2 | | | | II-1 | Usp24 | 23358 | 4-May-15 |
| 22548 | 2 | | | | II-1 | Usp25 | 29761 | 12-May-15 |
| 22549 | 2 | | | | II-1 | Usp26 | 83844 | 4-May-15 |
| 22550 | 2 | | | | II-1 | Usp27x | 389856 | 4-May-15 |
| 22551 | 2 | | | | II-1 | Usp28 | 57646 | 12-May-15 |
| 22553 | 2 | | | | II-1 | Usp3 | 9960 | 4-May-15 |
| 22554 | 2 | | | | II-1 | Usp30 | 84749 | 12-May-15 |
| 22555 | 2 | | | | II-1 | Usp31 | 57478 | 7-Jun-15 |
| 22563 | 2 | | | | II-1 | Usp39 | 10713 | 4-May-15 |
| 22564 | 2 | | | | II-1 | Usp4 | 7375 | 12-May-15 |
| 22565 | 2 | | | | II-1 | Usp40 | 55230 | 12-May-15 |
| 22566 | 2 | | | | II-1 | Usp42 | 84132 | 12-May-15 |
| 22567 | 2 | | | | II-1 | Usp43 | 124739 | 4-May-15 |
| 22568 | 2 | | | | II-1 | Usp44 | 84101 | 4-May-15 |
| 22569 | 2 | | | | II-1 | Usp45 | 85015 | 4-May-15 |
| 22572 | 2 | | | | II-1 | Usp48 | 84196 | 4-May-15 |
| 22573 | 2 | | | | II-1 | Usp49 | 25862 | 4-May-15 |
| 22576 | 2 | | | | II-1 | Usp51 | 158880 | 4-May-15 |
| 22577 | 2 | | | | II-1 | Usp53 | 54532 | 12-May-15 |
| 22582 | 2 | | | | II-1 | Usp9x | 8239 | 4-May-15 |
| 22584 | 2 | | | | II-1 | Uspl1 | 10208 | 23-May-15 |
| 22585 | 2 | | | | II-1 | Ust | 10090 | 4-May-15 |
| 22592 | 2 | | | | II-1 | Utp20 | 27340 | 4-May-15 |
| 22593 | 2 | | | | II-1 | Utp23 | 84294 | 4-May-15 |
| 22594 | 2 | | | | II-1 | Utp3 | 57050 | 4-May-15 |
| 22595 | 2 | | | | II-1 | Utp6 | 55813 | 4-May-15 |
| 22596 | 2 | | | | II-1 | Utrn | 7402 | 12-May-15 |
| 22597 | 2 | | | | II-1 | Uts2 | 10911 | 17-May-15 |
| 22598 | 2 | | | | II-1 | Uts2b | 257313 | 4-May-15 |
| 22606 | 2 | | | | II-1 | V1rd18 | | |
| 22607 | 2 | | | | II-1 | V1rd19 | | |
| 22608 | 2 | | | | II-1 | Vac14 | 55697 | 4-May-15 |
| 22611 | 2 | | | | II-1 | Vamp3 | 9341 | 4-May-15 |
| 22612 | 2 | | | | II-1 | Vamp4 | 8674 | 4-May-15 |
| 22620 | 2 | | | | II-1 | Vars | 7407 | 21-May-15 |
| 22622 | 2 | | | | II-1 | Vash1 | 22846 | 21-May-15 |
| 22633 | 2 | | | | II-1 | Vax2 | 25806 | 4-May-15 |
| 22634 | 2 | | | | II-1 | Vax2os | | |
| 22635 | 2 | | | | II-1 | Vbp1 | 7411 | 1-Jun-15 |
| 22640 | 2 | | | | II-1 | Vcpip1 | 80124 | 4-May-15 |
| 22644 | 2 | | | | II-1 | Vdac3 | 7419 | 7-Jun-15 |
| 22672 | 2 | | | | II-1 | Vmn1r1 | | |
| 22739 | 2 | | | | II-1 | Vmn1r185 | | |
| 22769 | 2 | | | | II-1 | Vmn1r212 | | |
| 22875 | 2 | | | | II-1 | Vmn1r-ps103 | | |
| 22876 | 2 | | | | II-1 | Vmn1r-ps79 | | |
| 22878 | 2 | | | | II-1 | Vmn2r10 | | |
| 22925 | 2 | | | | II-1 | Vmn2r31 | | |
| 22937 | 2 | | | | II-1 | Vmn2r42 | | |
| 22969 | 2 | | | | II-1 | Vmn2r72 | | |
| 22985 | 2 | | | | II-1 | Vmn2r87 | | |
| 22989 | 2 | | | | II-1 | Vmn2r90 | | |
| 22999 | 2 | | | | II-1 | Vmn2r-ps11 | | |
| 23002 | 2 | | | | II-1 | Vmn2r-ps54 | | |
| 23004 | 2 | | | | II-1 | Vmo1 | 284013 | 4-May-15 |
| 23020 | 2 | | | | II-1 | Vps25 | 84313 | 4-May-15 |
| 23021 | 2 | | | | II-1 | Vps26a | 9559 | 4-May-15 |
| 23022 | 2 | | | | II-1 | Vps26b | 112936 | 4-May-15 |
| 23026 | 2 | | | | II-1 | Vps33b | 26276 | 4-May-15 |
| 23028 | 2 | | | | II-1 | Vps36 | 51028 | 4-May-15 |
| 23036 | 2 | | | | II-1 | Vps4a | 27183 | 4-May-15 |
| 23038 | 2 | | | | II-1 | Vps51 | 738 | 29-May-15 |
| 23039 | 2 | | | | II-1 | Vps52 | 6293 | 29-May-15 |
| 23040 | 2 | | | | II-1 | Vps53 | 55275 | 29-May-15 |
| 23041 | 2 | | | | II-1 | Vps54 | 51542 | 29-May-15 |
| 23042 | 2 | | | | II-1 | Vps72 | 6944 | 4-May-15 |
| 23043 | 2 | | | | II-1 | Vps8 | 23355 | 21-May-15 |
| 23046 | 2 | | | | II-1 | Vrk2 | 7444 | 4-May-15 |
| 23062 | 2 | | | | II-1 | Vsx2 | 338917 | 7-Jun-15 |
| 23063 | 2 | | | | II-1 | Vta1 | 51534 | 31-May-15 |
| 23064 | 2 | | | | II-1 | Vtcn1 | 79679 | 24-May-15 |
| 23065 | 2 | | | | II-1 | Vti1a | 143187 | 21-May-15 |
| 23066 | 2 | | | | II-1 | Vti1b | 10490 | 21-May-15 |
| 23072 | 2 | | | | II-1 | Vwa5b1 | 127731 | 4-May-15 |
| 23074 | 2 | | | | II-1 | Vwa7 | 80737 | 4-May-15 |
| 23078 | 2 | | | | II-1 | Vwc2l | 402117 | 2-Jun-15 |
| 23079 | 2 | | | | II-1 | Vwce | 220001 | 4-May-15 |
| 23080 | 2 | | | | II-1 | Vwde | 221806 | 7-Jun-15 |
| 23081 | 2 | | | | II-1 | Vwf | 7450 | 31-May-15 |
| 23084 | 2 | | | | II-1 | Wapal | 23063 | 4-May-15 |
| 23094 | 2 | | | | II-1 | Wbp11 | 51729 | 3-May-15 |
| 23097 | 2 | | | | II-1 | Wbp2nl | 164684 | 12-May-15 |
| 23098 | 2 | | | | II-1 | Wbp4 | 11193 | 4-May-15 |
| 23099 | 2 | | | | II-1 | Wbp5 | 51186 | 4-May-15 |
| 23106 | 2 | | | | II-1 | Wdfy1 | 57590 | 12-May-15 |
| 23113 | 2 | | | | II-1 | Wdr11 | 55717 | 7-Jun-15 |

Fig.22 · 113

| | | | | | | | | |
|---|---|---|---|---|---|---|---|---|
| 23114 | 2 | | | | II-1 | Wdr12 | 55759 | 4-May-15 |
| 23117 | 2 | | | | II-1 | Wdr17 | 116966 | 4-May-15 |
| 23119 | 2 | | | | II-1 | Wdr19 | 57728 | 4-May-15 |
| 23120 | 2 | | | | II-1 | Wdr20 | 91833 | 3-May-15 |
| 23121 | 2 | | | | II-1 | Wdr20rt | | |
| 23125 | 2 | | | | II-1 | Wdr27 | 253769 | 4-May-15 |
| 23126 | 2 | | | | II-1 | Wdr3 | 10885 | 12-May-15 |
| 23129 | 2 | | | | II-1 | Wdr34 | 89891 | 4-May-15 |
| 23132 | 2 | | | | II-1 | Wdr37 | 22884 | 4-May-15 |
| 23133 | 2 | | | | II-1 | Wdr38 | 401551 | 4-May-15 |
| 23134 | 2 | | | | II-1 | Wdr4 | 10785 | 4-May-15 |
| 23135 | 2 | | | | II-1 | Wdr41 | 55255 | 4-May-15 |
| 23136 | 2 | | | | II-1 | Wdr43 | 23160 | 4-May-15 |
| 23140 | 2 | | | | II-1 | Wdr46 | 9277 | 4-May-15 |
| 23142 | 2 | | | | II-1 | Wdr48 | 57599 | 12-May-15 |
| 23144 | 2 | | | | II-1 | Wdr52 | 55779 | 4-May-15 |
| 23148 | 2 | | | | II-1 | Wdr59 | 79726 | 29-May-15 |
| 23149 | 2 | | | | II-1 | Wdr5b | 54554 | 4-May-15 |
| 23150 | 2 | | | | II-1 | Wdr6 | 11180 | 4-May-15 |
| 23153 | 2 | | | | II-1 | Wdr62 | 284403 | 23-May-15 |
| 23154 | 2 | | | | II-1 | Wdr63 | 126820 | 12-May-15 |
| 23155 | 2 | | | | II-1 | Wdr64 | 128025 | 4-May-15 |
| 23157 | 2 | | | | II-1 | Wdr7 | 23335 | 4-May-15 |
| 23158 | 2 | | | | II-1 | Wdr70 | 55100 | 4-May-15 |
| 23161 | 2 | | | | II-1 | Wdr74 | 54663 | 12-May-15 |
| 23164 | 2 | | | | II-1 | Wdr77 | 79084 | 4-May-15 |
| 23166 | 2 | | | | II-1 | Wdr8 | 49856 | 12-May-15 |
| 23177 | 2 | | | | II-1 | Wdr95 | | |
| 23179 | 2 | | | | II-1 | Wdsub1 | 151525 | 12-May-15 |
| 23180 | 2 | | | | II-1 | Wdtc1 | 23038 | 21-May-15 |
| 23181 | 2 | | | | II-1 | Wdyhv1 | 55093 | 23-May-15 |
| 23182 | 2 | | | | II-1 | Wee1 | 7465 | 4-May-15 |
| 23184 | 2 | | | | II-1 | Wfdc1 | 58189 | 4-May-15 |
| 23219 | 2 | | | | II-1 | Wls | 79971 | 17-May-15 |
| 23220 | 2 | | | | II-1 | Wnk1 | 65125 | 31-May-15 |
| 23233 | 2 | | | | II-1 | Wnt4 | 54361 | 4-May-15 |
| 23241 | 2 | | | | II-1 | Wnt9a | 7483 | 4-May-15 |
| 23244 | 2 | | | | II-1 | Wrb | 7485 | 4-May-15 |
| 23246 | 2 | | | | II-1 | Wrnip1 | 56897 | 4-May-15 |
| 23260 | 2 | | | | II-1 | Wwtr1 | 25937 | 4-May-15 |
| 23263 | 2 | | | | II-1 | Xbp1 | 7494 | 7-Jun-15 |
| 23287 | 2 | | | | II-1 | Xlr5b | | |
| 23288 | 2 | | | | II-1 | Xlr5c | | |
| 23300 | 2 | | | | II-1 | Xpo7 | 23039 | 4-May-15 |
| 23302 | 2 | | | | II-1 | Xpr1 | 9213 | 4-May-15 |
| 23304 | 2 | | | | II-1 | Xrcc2 | 7516 | 17-May-15 |
| 23305 | 2 | | | | II-1 | Xrcc3 | 7517 | 22-May-15 |
| 23313 | 2 | | | | II-1 | Xxylt1 | 152002 | 12-May-15 |
| 23318 | 2 | | | | II-1 | Yaf2 | 10138 | 4-May-15 |
| 23321 | 2 | | | | II-1 | Yars2 | 51067 | 4-May-15 |
| 23322 | 2 | | | | II-1 | Ybey | 54059 | 4-May-15 |
| 23323 | 2 | | | | II-1 | Ybx1 | 4904 | 2-Jun-15 |
| 23329 | 2 | | | | II-1 | Yes1 | 7525 | 12-May-15 |
| 23335 | 2 | | | | II-1 | Yipf4 | 84272 | 4-May-15 |
| 23336 | 2 | | | | II-1 | Yipf5 | 81555 | 4-May-15 |
| 23337 | 2 | | | | II-1 | Yipf6 | 286451 | 4-May-15 |
| 23350 | 2 | | | | II-1 | Ythdc2 | 64848 | 4-May-15 |
| 23351 | 2 | | | | II-1 | Ythdf1 | 54915 | 12-May-15 |
| 23353 | 2 | | | | II-1 | Ythdf3 | 253943 | 4-May-15 |
| 23354 | 2 | | | | II-1 | Ywhab | 7529 | 4-May-15 |
| 23355 | 2 | | | | II-1 | Ywhae | 7531 | 4-May-15 |
| 23356 | 2 | | | | II-1 | Ywhag | 7532 | 4-May-15 |
| 23357 | 2 | | | | II-1 | Ywhah | 7533 | 12-May-15 |
| 23358 | 2 | | | | II-1 | Ywhaq | 10971 | 31-May-15 |
| 23359 | 2 | | | | II-1 | Ywhaz | 7534 | 31-May-15 |
| 23360 | 2 | | | | II-1 | Yy1 | 7528 | 17-May-15 |
| 23361 | 2 | | | | II-1 | Yy2 | 404281 | 4-May-15 |
| 23362 | 2 | | | | II-1 | Zadh2 | 284273 | 12-May-15 |
| 23368 | 2 | | | | II-1 | Zbbx | 79740 | 4-May-15 |
| 23369 | 2 | | | | II-1 | Zbed3 | 84327 | 4-May-15 |
| 23377 | 2 | | | | II-1 | Zbtb12 | 221527 | 4-May-15 |
| 23383 | 2 | | | | II-1 | Zbtb20 | 26137 | 4-May-15 |
| 23386 | 2 | | | | II-1 | Zbtb24 | 9841 | 4-May-15 |
| 23387 | 2 | | | | II-1 | Zbtb25 | 7597 | 28-May-15 |
| 23388 | 2 | | | | II-1 | Zbtb26 | 57684 | 2-Jun-15 |
| 23394 | 2 | | | | II-1 | Zbtb38 | 253461 | 12-May-15 |
| 23402 | 2 | | | | II-1 | Zbtb45 | 84878 | 12-May-15 |
| 23409 | 2 | | | | II-1 | Zbtb7b | 51043 | 4-May-15 |
| 23428 | 2 | | | | II-1 | Zc3h18 | 124245 | 7-Jun-15 |
| 23429 | 2 | | | | II-1 | Zc3h3 | 23144 | 21-May-15 |
| 23431 | 2 | | | | II-1 | Zc3h6 | 376940 | 4-May-15 |
| 23439 | 2 | | | | II-1 | Zcchc10 | 54819 | 12-May-15 |
| 23442 | 2 | | | | II-1 | Zcchc13 | 389874 | 4-May-15 |
| 23445 | 2 | | | | II-1 | Zcchc17 | 51538 | 28-May-15 |
| 23453 | 2 | | | | II-1 | Zcchc7 | 84186 | 4-May-15 |
| 23455 | 2 | | | | II-1 | Zcchc9 | 84240 | 4-May-15 |
| 23456 | 2 | | | | II-1 | Zcrb1 | 85437 | 4-May-15 |
| 23458 | 2 | | | | II-1 | Zdbf2 | 57683 | 4-May-15 |
| 23461 | 2 | | | | II-1 | Zdhhc12 | 84885 | 4-May-15 |
| 23467 | 2 | | | | II-1 | Zdhhc18 | 84243 | 4-May-15 |
| 23468 | 2 | | | | II-1 | Zdhhc19 | 131540 | 4-May-15 |
| 23472 | 2 | | | | II-1 | Zdhhc22 | 283576 | 4-May-15 |
| 23477 | 2 | | | | II-1 | Zdhhc4 | 55146 | 4-May-15 |
| 23478 | 2 | | | | II-1 | Zdhhc5 | 25921 | 4-May-15 |
| 23480 | 2 | | | | II-1 | Zdhhc7 | 55625 | 4-May-15 |
| 23481 | 2 | | | | II-1 | Zdhhc8 | 29801 | 3-May-15 |
| 23482 | 2 | | | | II-1 | Zdhhc9 | 51114 | 4-May-15 |

| | | | | | | | | |
|---|---|---|---|---|---|---|---|---|
| 23483 | 2 | | | | II-1 | Zeb1 | 6935 | 31-May-15 |
| 23484 | 2 | | | | II-1 | Zeb2 | 9839 | 31-May-15 |
| 23493 | 2 | | | | II-1 | Zfand5 | 7763 | 4-May-15 |
| 23496 | 2 | | | | II-1 | Zfat | 57623 | 28-May-15 |
| 23497 | 2 | | | | II-1 | Zfc3h1 | 196441 | 4-May-15 |
| 23501 | 2 | | | | II-1 | Zfhx4 | 79776 | 12-May-15 |
| 23505 | 2 | | | | II-1 | Zfp105 | 7584 | 12-May-15 |
| 23512 | 2 | | | | II-1 | Zfp112 | 7771 | 4-May-15 |
| 23514 | 2 | | | | II-1 | Zfp114 | | |
| 23518 | 2 | | | | II-1 | Zfp120 | | |
| 23527 | 2 | | | | II-1 | Zfp148 | 7707 | 2-Jun-15 |
| 23528 | 2 | | | | II-1 | Zfp157 | | |
| 23534 | 2 | | | | II-1 | Zfp184 | | |
| 23541 | 2 | | | | II-1 | Zfp212 | | |
| 23542 | 2 | | | | II-1 | Zfp213 | | |
| 23546 | 2 | | | | II-1 | Zfp235 | | |
| 23549 | 2 | | | | II-1 | Zfp248 | | |
| 23551 | 2 | | | | II-1 | Zfp26 | 50862 | 1-Jun-15 |
| 23554 | 2 | | | | II-1 | Zfp266 | | |
| 23555 | 2 | | | | II-1 | Zfp27 | | |
| 23557 | 2 | | | | II-1 | Zfp275 | | |
| 23559 | 2 | | | | II-1 | Zfp277 | | |
| 23567 | 2 | | | | II-1 | Zfp287 | | |
| 23581 | 2 | | | | II-1 | Zfp330 | | |
| 23584 | 2 | | | | II-1 | Zfp341 | | |
| 23585 | 2 | | | | II-1 | Zfp345 | | |
| 23586 | 2 | | | | II-1 | Zfp346 | 23567 | 21-May-15 |
| 23587 | 2 | | | | II-1 | Zfp35 | | |
| 23589 | 2 | | | | II-1 | Zfp354a | | |
| 23592 | 2 | | | | II-1 | Zfp358 | | |
| 23595 | 2 | | | | II-1 | Zfp365 | | |
| 23603 | 2 | | | | II-1 | Zfp382 | | |
| 23610 | 2 | | | | II-1 | Zfp389 | | |
| 23612 | 2 | | | | II-1 | Zfp395 | | |
| 23619 | 2 | | | | II-1 | Zfp410 | | |
| 23636 | 2 | | | | II-1 | Zfp451 | | |
| 23638 | 2 | | | | II-1 | Zfp455 | | |
| 23640 | 2 | | | | II-1 | Zfp457 | | |
| 23642 | 2 | | | | II-1 | Zfp459 | | |
| 23648 | 2 | | | | II-1 | Zfp474 | | |
| 23654 | 2 | | | | II-1 | Zfp511 | | |
| 23655 | 2 | | | | II-1 | Zfp512 | | |
| 23656 | 2 | | | | II-1 | Zfp513 | | |
| 23657 | 2 | | | | II-1 | Zfp516 | | |
| 23674 | 2 | | | | II-1 | Zfp560 | | |
| 23675 | 2 | | | | II-1 | Zfp563 | | |
| 23681 | 2 | | | | II-1 | Zfp575 | | |
| 23682 | 2 | | | | II-1 | Zfp579 | | |
| 23683 | 2 | | | | II-1 | Zfp58 | | |
| 23684 | 2 | | | | II-1 | Zfp580 | | |
| 23690 | 2 | | | | II-1 | Zfp597 | | |
| 23691 | 2 | | | | II-1 | Zfp598 | | |
| 23692 | 2 | | | | II-1 | Zfp599 | | |
| 23693 | 2 | | | | II-1 | Zfp60 | | |
| 23695 | 2 | | | | II-1 | Zfp605 | | |
| 23696 | 2 | | | | II-1 | Zfp606 | | |
| 23704 | 2 | | | | II-1 | Zfp618 | | |
| 23707 | 2 | | | | II-1 | Zfp622 | | |
| 23709 | 2 | | | | II-1 | Zfp628 | 89887 | 4-May-15 |
| 23710 | 2 | | | | II-1 | Zfp629 | | |
| 23711 | 2 | | | | II-1 | Zfp637 | | |
| 23712 | 2 | | | | II-1 | Zfp639 | | |
| 23714 | 2 | | | | II-1 | Zfp641 | | |
| 23725 | 2 | | | | II-1 | Zfp655 | | |
| 23729 | 2 | | | | II-1 | Zfp664 | | |
| 23730 | 2 | | | | II-1 | Zfp667 | | |
| 23734 | 2 | | | | II-1 | Zfp68 | | |
| 23736 | 2 | | | | II-1 | Zfp688 | | |
| 23744 | 2 | | | | II-1 | Zfp704 | | |
| 23747 | 2 | | | | II-1 | Zfp708 | | |
| 23748 | 2 | | | | II-1 | Zfp709 | | |
| 23752 | 2 | | | | II-1 | Zfp715 | | |
| 23754 | 2 | | | | II-1 | Zfp72 | | |
| 23756 | 2 | | | | II-1 | Zfp738 | | |
| 23757 | 2 | | | | II-1 | Zfp74 | | |
| 23760 | 2 | | | | II-1 | Zfp747 | | |
| 23770 | 2 | | | | II-1 | Zfp771 | | |
| 23773 | 2 | | | | II-1 | Zfp775 | | |
| 23776 | 2 | | | | II-1 | Zfp780b | | |
| 23777 | 2 | | | | II-1 | Zfp781 | | |
| 23782 | 2 | | | | II-1 | Zfp788 | | |
| 23784 | 2 | | | | II-1 | Zfp791 | | |
| 23789 | 2 | | | | II-1 | Zfp808 | | |
| 23795 | 2 | | | | II-1 | Zfp82 | 284406 | 4-May-15 |
| 23799 | 2 | | | | II-1 | Zfp827 | | |
| 23801 | 2 | | | | II-1 | Zfp831 | | |
| 23803 | 2 | | | | II-1 | Zfp84 | | |
| 23804 | 2 | | | | II-1 | Zfp846 | | |
| 23805 | 2 | | | | II-1 | Zfp85 | | |
| 23813 | 2 | | | | II-1 | Zfp869 | | |
| 23814 | 2 | | | | II-1 | Zfp87 | | |
| 23815 | 2 | | | | II-1 | Zfp870 | | |
| 23823 | 2 | | | | II-1 | Zfp9 | 219749 | 4-May-15 |
| 23826 | 2 | | | | II-1 | Zfp91Cntf | 386607 | 7-Jun-15 |
| 23827 | 2 | | | | II-1 | Zfp92 | 139735 | 4-May-15 |
| 23828 | 2 | | | | II-1 | Zfp93 | 9310 | 12-May-15 |
| 23834 | 2 | | | | II-1 | Zfp935 | | |

Fig.22 - 114

| | | | | | | | | |
|---|---|---|---|---|---|---|---|---|
| 23842 | 2 | | | | II-1 | Zfp942 | | |
| 23843 | 2 | | | | II-1 | Zfp943 | | |
| 23845 | 2 | | | | II-1 | Zfp945 | | |
| 23850 | 2 | | | | II-1 | Zfp951 | | |
| 23851 | 2 | | | | II-1 | Zfp952 | | |
| 23853 | 2 | | | | II-1 | Zfp954 | | |
| 23860 | 2 | | | | II-1 | Zfp960 | | |
| 23862 | 2 | | | | II-1 | Zfp963 | | |
| 23863 | 2 | | | | II-1 | Zfp964 | | |
| 23865 | 2 | | | | II-1 | Zfpl1 | 7542 | 4-May-15 |
| 23873 | 2 | | | | II-1 | Zfyve1 | 53349 | 21-May-15 |
| 23879 | 2 | | | | II-1 | Zfyve27 | 118813 | 12-May-15 |
| 23880 | 2 | | | | II-1 | Zfyve28 | 57732 | 4-May-15 |
| 23885 | 2 | | | | II-1 | Zgrf1 | 55345 | 4-May-15 |
| 23892 | 2 | | | | II-1 | Zic4 | 84107 | 4-May-15 |
| 23894 | 2 | | | | II-1 | Zik1 | 284307 | 4-May-15 |
| 23895 | 2 | | | | II-1 | Zim1 | | |
| 23898 | 2 | | | | II-1 | Zkscan14 | 84124 | 21-May-15 |
| 23899 | 2 | | | | II-1 | Zkscan16 | 158399 | 4-May-15 |
| 23900 | 2 | | | | II-1 | Zkscan17 | 84838 | 28-May-15 |
| 23901 | 2 | | | | II-1 | Zkscan2 | 342357 | 28-May-15 |
| 23902 | 2 | | | | II-1 | Zkscan3 | 80317 | 4-May-15 |
| 23905 | 2 | | | | II-1 | Zkscan6 | 7566 | 28-May-15 |
| 23910 | 2 | | | | II-1 | Zmat3 | 64393 | 4-May-15 |
| 23916 | 2 | | | | II-1 | Zmym1 | 79830 | 20-May-15 |
| 23917 | 2 | | | | II-1 | Zmym2 | 7750 | 12-May-15 |
| 23919 | 2 | | | | II-1 | Zmym4 | 9202 | 4-May-15 |
| 23920 | 2 | | | | II-1 | Zmym5 | 9205 | 4-May-15 |
| 23921 | 2 | | | | II-1 | Zmym6 | 9204 | 12-May-15 |
| 23922 | 2 | | | | II-1 | Zmynd10 | 51364 | 21-May-15 |
| 23925 | 2 | | | | II-1 | Zmynd15 | 84225 | 4-May-15 |
| 23930 | 2 | | | | II-1 | Znfx1 | 57169 | 4-May-15 |
| 23931 | 2 | | | | II-1 | Znhit1 | 10467 | 4-May-15 |
| 23932 | 2 | | | | II-1 | Znhit2 | 741 | 4-May-15 |
| 23933 | 2 | | | | II-1 | Znhit3 | 9326 | 4-May-15 |
| 23935 | 2 | | | | II-1 | Znrd1 | 30834 | 12-May-15 |
| 23943 | 2 | | | | II-1 | Zp3 | 7784 | 7-Jun-15 |
| 23947 | 2 | | | | II-1 | Zpbp2 | 124626 | 4-May-15 |
| 23949 | 2 | | | | II-1 | Zpr1 | 8882 | 12-May-15 |
| 23950 | 2 | | | | II-1 | Zranb1 | 54764 | 2-Jun-15 |
| 23955 | 2 | | | | II-1 | Zscan10 | 84891 | 4-May-15 |
| 23956 | 2 | | | | II-1 | Zscan12 | 9753 | 4-May-15 |
| 23960 | 2 | | | | II-1 | Zscan21 | 7589 | 28-May-15 |
| 23964 | 2 | | | | II-1 | Zscan29 | 146050 | 28-May-15 |
| 23965 | 2 | | | | II-1 | Zscan4a | | |
| 23966 | 2 | | | | II-1 | Zscan4b | | |
| 23967 | 2 | | | | II-1 | Zscan4c | | |
| 23974 | 2 | | | | II-1 | Zswim3 | 140831 | 4-May-15 |
| 23975 | 2 | | | | II-1 | Zswim4 | 65249 | 4-May-15 |
| 23977 | 2 | | | | II-1 | Zswim6 | 57688 | 4-May-15 |
| 23978 | 2 | | | | II-1 | Zswim7 | 125150 | 4-May-15 |
| 23981 | 2 | | | | II-1 | Zw10 | 9183 | 29-May-15 |
| 23982 | 2 | | | | II-1 | Zwilch | 55055 | 29-May-15 |
| 23985 | 2 | | | | II-1 | Zxdb | 158586 | 4-May-15 |
| 23986 | 2 | | | | II-1 | Zxdc | 79364 | 4-May-15 |
| 23987 | 2 | | | | II-1 | Zyg11a | 440590 | 12-May-15 |
| 23991 | 2 | | | | II-1 | Zzz3 | 26009 | 21-May-15 |
| 207 | | | | | | 1700020N18Rik | | |
| 312 | | | | | | 1700049L16Rik | | |
| 849 | | | | | | 4930429B21Rik | | |
| 891 | | | | | | 4930449E18Rik | | |
| 895 | | | | | | 4930451I11Rik | | |
| 902 | | | | | | 4930453N24Rik | | |
| 914 | | | | | | 4930465K10Rik | | |
| 919 | | | | | | 4930468A15Rik | | |
| 921 | | | | | | 4930470H14Rik | | |
| 976 | | | | | | 4930511M06Rik | | |
| 1003 | | | | | | 4930524N10Rik | | |
| 1007 | | | | | | 4930525G20Rik | | |
| 1017 | | | | | | 4930529K09Rik | | |
| 1022 | | | | | | 4930533P14Rik | | |
| 1048 | | | | | | 4930549G23Rik | | |
| 1064 | | | | | | 4930558C23Rik | | |
| 1066 | | | | | | 4930558J18Rik | | |
| 1111 | | | | | | 4930592A05Rik | | |
| 1138 | | | | | | 4931428F04Rik | | |
| 1158 | | | | | | 4932416H05Rik | | |
| 1160 | | | | | | 4932418E24Rik | | |
| 1162 | | | | | | 4932435O22Rik | | |
| 1173 | | | | | | 4933401B06Rik | | |
| 1174 | | | | | | 4933401D09Rik | | |
| 1192 | | | | | | 4933405O20Rik | | |
| 1205 | | | | | | 4933407K13Rik | | |
| 1206 | | | | | | 4933407L21Rik | | |
| 1281 | | | | | | 5031414D18Rik | | |
| 1319 | | | | | | 5530601H04Rik | | |
| 1320 | | | | | | 5730403I07Rik | | |
| 1330 | | | | | | 5730457N03Rik | | |
| 1401 | | | | | | 7630403G23Rik | | |
| 1407 | | | | | | 8030462N17Rik | | |
| 1432 | | | | | | 9130019P16Rik | | |
| 1433 | | | | | | 9130023H24Rik | | |
| 1474 | | | | | | 9430008C03Rik | | |
| 2058 | | | | | | AF357425 | | |
| 3079 | | | | | | B130024G19Rik | | |
| 3478 | | | | | | Bptf | 2186 | 4-May-15 |
| 3687 | | | | | | C920021L13Rik | | |

| | | | | | | | | |
|---|---|---|---|---|---|---|---|---|
| 4907 | | | | | | Cntnap5b | | |
| 5221 | | | | | | Csn1s2b | 100337616 | 4-May-15 |
| 5590 | | | | | | D5Ertd579e | | |
| 5612 | | | | | | D7Ertd143e | | |
| 5683 | | | | | | Dcaf11 | 80344 | 12-May-15 |
| 6944 | | | | | | F730043M19Rik | | |
| 7602 | | | | | | Fpr2 | 2358 | 4-May-15 |
| 7933 | | | | | | Gga2 | 23062 | 4-May-15 |
| 8104 | | | | | | Gm10389 | | |
| 8116 | | | | | | Gm10436 | | |
| 8212 | | | | | | Gm11544 | | |
| 8254 | | | | | | Gm12171 | | |
| 8273 | | | | | | Gm12657 | | |
| 8275 | | | | | | Gm12695 | | |
| 8284 | | | | | | Gm12888 | | |
| 8306 | | | | | | Gm13128 | | |
| 8322 | | | | | | Gm13276 | | |
| 8323 | | | | | | Gm13277 | | |
| 8325 | | | | | | Gm13279 | | |
| 8326 | | | | | | Gm13283 | | |
| 8327 | | | | | | Gm13285 | | |
| 8328 | | | | | | Gm13286 | | |
| 8330 | | | | | | Gm13290 | | |
| 8331 | | | | | | Gm13293 | | |
| 8376 | | | | | | Gm14207 | | |
| 8390 | | | | | | Gm14374 | | |
| 8412 | | | | | | Gm14483 | | |
| 8415 | | | | | | Gm14499 | | |
| 8417 | | | | | | Gm14511 | | |
| 8432 | | | | | | Gm14819 | | |
| 8445 | | | | | | Gm15104 | | |
| 8471 | | | | | | Gm15441 | | |
| 8480 | | | | | | Gm15645 | | |
| 8496 | | | | | | Gm15910 | | |
| 8520 | | | | | | Gm16430 | | |
| 8554 | | | | | | Gm16897 | | |
| 8630 | | | | | | Gm20172 | | |
| 8634 | | | | | | Gm20257 | | |
| 8692 | | | | | | Gm20878 | | |
| 8743 | | | | | | Gm2a | 2760 | 21-May-15 |
| 8795 | | | | | | Gm4214 | | |
| 8797 | | | | | | Gm4224 | | |
| 8799 | | | | | | Gm4262 | | |
| 8834 | | | | | | Gm4787 | | |
| 8839 | | | | | | Gm4814 | | |
| 8850 | | | | | | Gm4872 | | |
| 8856 | | | | | | Gm4922 | | |
| 8871 | | | | | | Gm5039 | | |
| 8873 | | | | | | Gm5069 | | |
| 8926 | | | | | | Gm5464 | | |
| 8931 | | | | | | Gm5478 | | |
| 8940 | | | | | | Gm5547 | | |
| 8946 | | | | | | Gm5607 | | |
| 8981 | | | | | | Gm5893 | | |
| 8992 | | | | | | Gm597 | | |
| 9028 | | | | | | Gm6455 | | |
| 9064 | | | | | | Gm684 | | |
| 9068 | | | | | | Gm6902 | | |
| 9120 | | | | | | Gm8096 | | |
| 9121 | | | | | | Gm813 | | |
| 9140 | | | | | | Gm8633 | | |
| 9145 | | | | | | Gm8765 | | |
| 9153 | | | | | | Gm8884 | | |
| 9174 | | | | | | Gm9573 | | |
| 9176 | | | | | | Gm9696 | | |
| 9181 | | | | | | Gm9767 | | |
| 9483 | | | | | | Grik1 | 2897 | 31-May-15 |
| 9738 | | | | | | Hapln4 | 404037 | 4-May-15 |
| 10339 | | | | | | Ifnk | 56832 | 4-May-15 |
| 12506 | | | | | | Mir1899 | | |
| 12507 | | | | | | Mir18b | 574033 | 21-May-15 |
| 12508 | | | | | | Mir190 | 406965 | 21-May-15 |
| 12509 | | | | | | Mir1900 | | |
| 12510 | | | | | | Mir1901 | | |
| 12511 | | | | | | Mir1902 | | |
| 12512 | | | | | | Mir1903 | | |
| 12513 | | | | | | Mir1904 | | |
| 12514 | | | | | | Mir1905 | | |
| 12515 | | | | | | Mir1906-1 | | |
| 12516 | | | | | | Mir1907 | | |
| 12517 | | | | | | Mir190b | 100126346 | 21-May-15 |
| 12518 | | | | | | Mir191 | 406966 | 21-May-15 |
| 12519 | | | | | | Mir1912 | 100302144 | 21-May-15 |
| 12520 | | | | | | Mir192 | 406967 | 21-May-15 |
| 12521 | | | | | | Mir1928 | | |
| 12522 | | | | | | Mir1929 | | |
| 12523 | | | | | | Mir193 | | |
| 12524 | | | | | | Mir1930 | | |
| 12525 | | | | | | Mir1931 | | |
| 12526 | | | | | | Mir1932 | | |
| 12527 | | | | | | Mir1933 | | |
| 12529 | | | | | | Mir1936 | | |
| 12530 | | | | | | Mir1938 | | |
| 12531 | | | | | | Mir193b | 574455 | 21-May-15 |
| 12534 | | | | | | Mir194-1 | 406969 | 21-May-15 |

Fig.22 - 115

| | | | | | | | | |
|---|---|---|---|---|---|---|---|---|
| 12536 | | | | | | Mir194-2 | 406970 | 21-May-15 |
| 12537 | | | | | | Mir1943 | | |
| 12538 | | | | | | Mir1945 | | |
| 12539 | | | | | | Mir1946a | | |
| 12540 | | | | | | Mir1946b | | |
| 12541 | | | | | | Mir1947 | | |
| 12542 | | | | | | Mir1948 | | |
| 12543 | | | | | | Mir1949 | | |
| 12544 | | | | | | Mir195 | 406971 | 21-May-15 |
| 12545 | | | | | | Mir1950 | | |
| 12546 | | | | | | Mir1951 | | |
| 12547 | | | | | | Mir1952 | | |
| 12548 | | | | | | Mir1953 | | |
| 12549 | | | | | | Mir1954 | | |
| 12551 | | | | | | Mir1956 | | |
| 12552 | | | | | | Mir1957 | | |
| 12557 | | | | | | Mir1961 | | |
| 12558 | | | | | | Mir1962 | | |
| 12559 | | | | | | Mir1963 | | |
| 12560 | | | | | | Mir1964 | | |
| 12561 | | | | | | Mir1966 | | |
| 12562 | | | | | | Mir1967 | | |
| 12563 | | | | | | Mir1968 | | |
| 12564 | | | | | | Mir1969 | | |
| 12565 | | | | | | Mir196a-1 | | |
| 12566 | | | | | | Mir196a-2 | | |
| 12567 | | | | | | Mir196b | 442920 | 21-May-15 |
| 12568 | | | | | | Mir1970 | | |
| 12569 | | | | | | Mir1971 | | |
| 12570 | | | | | | Mir1981 | | |
| 12571 | | | | | | Mir1982 | | |
| 12572 | | | | | | Mir1983 | | |
| 12573 | | | | | | Mir199a-1 | | |
| 12575 | | | | | | Mir199b | 406978 | 21-May-15 |
| 12577 | | | | | | Mir19b-1 | | |
| 12578 | | | | | | Mir19b-2 | | |
| 12579 | | | | | | Mir1a-1 | | |
| 12580 | | | | | | Mir1a-2 | | |
| 12581 | | | | | | Mir1b | | |
| 12582 | | | | | | Mir200a | 406983 | 21-May-15 |
| 12583 | | | | | | Mir200b | 406984 | 21-May-15 |
| 12584 | | | | | | Mir200c | 406985 | 21-May-15 |
| 12585 | | | | | | Mir201 | | |
| 12586 | | | | | | Mir202 | 574448 | 21-May-15 |
| 12587 | | | | | | Mir203 | 406986 | 21-May-15 |
| 12588 | | | | | | Mir204 | 406987 | 31-May-15 |
| 12589 | | | | | | Mir205 | 406988 | 21-May-15 |
| 12590 | | | | | | Mir206 | 406989 | 21-May-15 |
| 12591 | | | | | | Mir207 | | |
| 12592 | | | | | | Mir208a | 406990 | 4-May-15 |
| 12593 | | | | | | Mir208b | 100126336 | 21-May-15 |
| 12594 | | | | | | Mir20a | 406982 | 21-May-15 |
| 12596 | | | | | | Mir21 | 406991 | 7-Jun-15 |
| 12598 | | | | | | Mir211 | 406993 | 21-May-15 |
| 12599 | | | | | | Mir212 | 406994 | 21-May-15 |
| 12600 | | | | | | Mir2136 | | |
| 12601 | | | | | | Mir2137 | | |
| 12602 | | | | | | Mir2139 | | |
| 12603 | | | | | | Mir214 | 406996 | 21-May-15 |
| 12608 | | | | | | Mir217 | 406999 | 21-May-15 |
| 12609 | | | | | | Mir218-1 | 407000 | 21-May-15 |
| 12610 | | | | | | Mir218-2 | 407001 | 21-May-15 |
| 12611 | | | | | | Mir219-1 | 407002 | 21-May-15 |
| 12612 | | | | | | Mir219-2 | 407003 | 21-May-15 |
| 12613 | | | | | | Mir219b | 100616335 | 21-May-15 |
| 12614 | | | | | | Mir219c | | |
| 12615 | | | | | | Mir21b | | |
| 12616 | | | | | | Mir21c | | |
| 12617 | | | | | | Mir22 | 407004 | 24-May-15 |
| 12618 | | | | | | Mir221 | 407006 | 21-May-15 |
| 12619 | | | | | | Mir222 | 407007 | 21-May-15 |
| 12620 | | | | | | Mir223 | 407008 | 21-May-15 |
| 12623 | | | | | | Mir23b | 407011 | 21-May-15 |
| 12625 | | | | | | Mir24-2 | 407013 | 21-May-15 |
| 12626 | | | | | | Mir25 | 407014 | 21-May-15 |
| 12627 | | | | | | Mir26a-1 | | |
| 12628 | | | | | | Mir26a-2 | | |
| 12629 | | | | | | Mir26b | 407017 | 21-May-15 |
| 12630 | | | | | | Mir27a | 407018 | 21-May-15 |
| 12631 | | | | | | Mir27b | 407019 | 21-May-15 |
| 12632 | | | | | | Mir28 | | |
| 12633 | | | | | | Mir2861 | 100422910 | 9-May-15 |
| 12634 | | | | | | Mir28b | | |
| 12635 | | | | | | Mir28c | | |
| 12636 | | | | | | Mir290 | | |
| 12637 | | | | | | Mir290b | | |
| 12638 | | | | | | Mir291a | | |
| 12639 | | | | | | Mir291b | | |
| 12640 | | | | | | Mir292 | | |
| 12641 | | | | | | Mir292b | | |
| 12642 | | | | | | Mir293 | | |
| 12643 | | | | | | Mir294 | | |
| 12644 | | | | | | Mir295 | | |
| 12645 | | | | | | Mir296 | 407022 | 21-May-15 |
| 12646 | | | | | | Mir297-1 | | |

| | | | | | | | | |
|---|---|---|---|---|---|---|---|---|
| 12647 | | | | | | Mir297-2 | | |
| 12648 | | | | | | Mir297a-3 | | |
| 12649 | | | | | | Mir297a-4 | | |
| 12651 | | | | | | Mir297c | | |
| 12652 | | | | | | Mir298 | 100126296 | 4-May-15 |
| 12653 | | | | | | Mir299 | 407023 | 21-May-15 |
| 12654 | | | | | | Mir299b | | |
| 12655 | | | | | | Mir29a | 407021 | 21-May-15 |
| 12656 | | | | | | Mir29b-1 | | |
| 12657 | | | | | | Mir29b-2 | | |
| 12658 | | | | | | Mir29c | 407026 | 21-May-15 |
| 12659 | | | | | | Mir300 | 100126297 | 4-May-15 |
| 12660 | | | | | | Mir301 | 407027 | 21-May-15 |
| 12661 | | | | | | Mir301b | 100126318 | 21-May-15 |
| 12662 | | | | | | Mir302a | 407028 | 21-May-15 |
| 12663 | | | | | | Mir302b | 442894 | 21-May-15 |
| 12664 | | | | | | Mir302c | 442895 | 21-May-15 |
| 12665 | | | | | | Mir302d | 442896 | 21-May-15 |
| 12666 | | | | | | Mir3057 | | |
| 12667 | | | | | | Mir3058 | | |
| 12668 | | | | | | Mir3059 | | |
| 12670 | | | | | | Mir3061 | | |
| 12671 | | | | | | Mir3062 | | |
| 12672 | | | | | | Mir3063 | | |
| 12673 | | | | | | Mir3064 | 100616387 | 4-May-15 |
| 12674 | | | | | | Mir3065 | 100422915 | 21-May-15 |
| 12675 | | | | | | Mir3066 | | |
| 12676 | | | | | | Mir3067 | | |
| 12677 | | | | | | Mir3068 | | |
| 12678 | | | | | | Mir3069 | | |
| 12679 | | | | | | Mir3070a | | |
| 12680 | | | | | | Mir3070b | | |
| 12681 | | | | | | Mir3071 | | |
| 12682 | | | | | | Mir3072 | | |
| 12683 | | | | | | Mir3073 | | |
| 12684 | | | | | | Mir3073b | | |
| 12685 | | | | | | Mir3074-1 | | |
| 12686 | | | | | | Mir3074-2 | | |
| 12687 | | | | | | Mir3075 | | |
| 12688 | | | | | | Mir3076 | | |
| 12689 | | | | | | Mir3077 | | |
| 12690 | | | | | | Mir3078 | | |
| 12691 | | | | | | Mir3079 | | |
| 12692 | | | | | | Mir3081 | | |
| 12693 | | | | | | Mir3082 | | |
| 12694 | | | | | | Mir3083 | | |
| 12695 | | | | | | Mir3084 | | |
| 12696 | | | | | | Mir3084-2 | | |
| 12697 | | | | | | Mir3085 | | |
| 12698 | | | | | | Mir3086 | | |
| 12699 | | | | | | Mir3087 | | |
| 12700 | | | | | | Mir3088 | | |
| 12701 | | | | | | Mir3089 | | |
| 12702 | | | | | | Mir3091 | | |
| 12703 | | | | | | Mir3092 | | |
| 12704 | | | | | | Mir3093 | | |
| 12705 | | | | | | Mir3094 | | |
| 12706 | | | | | | Mir3095 | | |
| 12707 | | | | | | Mir3097 | | |
| 12708 | | | | | | Mir3098 | | |
| 12709 | | | | | | Mir3099 | | |
| 12710 | | | | | | Mir30a | 407029 | 7-Jun-15 |
| 12711 | | | | | | Mir30b | 407030 | 21-May-15 |
| 12712 | | | | | | Mir30c-1 | | |
| 12713 | | | | | | Mir30c-2 | | |
| 12714 | | | | | | Mir30d | 407033 | 21-May-15 |
| 12715 | | | | | | Mir30f | | |
| 12716 | | | | | | Mir31 | 407035 | 21-May-15 |
| 12717 | | | | | | Mir3100 | | |
| 12718 | | | | | | Mir3101 | | |
| 12719 | | | | | | Mir3102 | | |
| 12721 | | | | | | Mir3104 | | |
| 12722 | | | | | | Mir3106 | | |
| 12723 | | | | | | Mir3107 | | |
| 12724 | | | | | | Mir3108 | | |
| 12725 | | | | | | Mir3109 | | |
| 12726 | | | | | | Mir3110 | | |
| 12727 | | | | | | Mir3112 | | |
| 12728 | | | | | | Mir32 | 407036 | 21-May-15 |
| 12729 | | | | | | Mir320 | | |
| 12730 | | | | | | Mir322 | 494336 | 21-May-15 |
| 12731 | | | | | | Mir323 | 442897 | 21-May-15 |
| 12732 | | | | | | Mir324 | 442898 | 21-May-15 |
| 12733 | | | | | | Mir325 | | |
| 12734 | | | | | | Mir326 | 442900 | 21-May-15 |
| 12735 | | | | | | Mir328 | 442901 | 21-May-15 |
| 12736 | | | | | | Mir329 | | |
| 12738 | | | | | | Mir330 | 442902 | 21-May-15 |
| 12739 | | | | | | Mir331 | 442903 | 21-May-15 |
| 12740 | | | | | | Mir335 | 442904 | 21-May-15 |
| 12741 | | | | | | Mir337 | 442905 | 21-May-15 |
| 12742 | | | | | | Mir338 | 442906 | 21-May-15 |
| 12743 | | | | | | Mir339 | 442907 | 21-May-15 |
| 12744 | | | | | | Mir340 | 442908 | 21-May-15 |

Fig.22 - 116

| | | | | | | | | |
|---|---|---|---|---|---|---|---|---|
| 12745 | | | | | | Mir341 | | |
| 12746 | | | | | | Mir343 | | |
| 12747 | | | | | | Mir344 | | |
| 12748 | | | | | | Mir344-2 | | |
| 12749 | | | | | | Mir344b | | |
| 12750 | | | | | | Mir344c | | |
| 12751 | | | | | | Mir344d-1 | | |
| 12752 | | | | | | Mir344d-2 | | |
| 12753 | | | | | | Mir344d-3 | | |
| 12754 | | | | | | Mir344e | | |
| 12755 | | | | | | Mir344f | | |
| 12756 | | | | | | Mir344g | | |
| 12757 | | | | | | Mir344h-1 | | |
| 12758 | | | | | | Mir344i | | |
| 12759 | | | | | | Mir345 | 442910 | 21-May-15 |
| 12760 | | | | | | Mir346 | 442911 | 21-May-15 |
| 12761 | | | | | | Mir3470a | | |
| 12762 | | | | | | Mir3470b | | |
| 12763 | | | | | | Mir3471-1 | | |
| 12764 | | | | | | Mir3473 | | |
| 12765 | | | | | | Mir3473c | | |
| 12766 | | | | | | Mir3473d | | |
| 12767 | | | | | | Mir3473e | | |
| 12768 | | | | | | Mir3473f | | |
| 12769 | | | | | | Mir3473g | | |
| 12770 | | | | | | Mir3474 | | |
| 12771 | | | | | | Mir3475 | | |
| 12772 | | | | | | Mir34a | 407040 | 21-May-15 |
| 12773 | | | | | | Mir34b | 407041 | 21-May-15 |
| 12774 | | | | | | Mir34c | 407042 | 21-May-15 |
| 12775 | | | | | | Mir350 | | |
| 12776 | | | | | | Mir351 | | |
| 12777 | | | | | | Mir3535 | | |
| 12778 | | | | | | Mir3544 | | |
| 12779 | | | | | | Mir3547 | | |
| 12780 | | | | | | Mir3569 | | |
| 12781 | | | | | | Mir3572 | | |
| 12782 | | | | | | Mir362 | 574030 | 21-May-15 |
| 12783 | | | | | | Mir3620 | 100500810 | 21-May-15 |
| 12784 | | | | | | Mir363 | 574031 | 21-May-15 |
| 12785 | | | | | | Mir365-1 | 100126355 | 21-May-15 |
| 12786 | | | | | | Mir365-2 | 100126356 | 21-May-15 |
| 12787 | | | | | | Mir367 | 442912 | 17-May-15 |
| 12788 | | | | | | Mir369 | 442914 | 21-May-15 |
| 12789 | | | | | | Mir370 | 442915 | 21-May-15 |
| 12790 | | | | | | Mir374 | | |
| 12791 | | | | | | Mir374c | 100500807 | 21-May-15 |
| 12792 | | | | | | Mir375 | 494324 | 31-May-15 |
| 12793 | | | | | | Mir376a | | |
| 12794 | | | | | | Mir376b | 574435 | 21-May-15 |
| 12795 | | | | | | Mir376c | 442913 | 21-May-15 |
| 12796 | | | | | | Mir377 | 494326 | 21-May-15 |
| 12797 | | | | | | Mir378 | | |
| 12798 | | | | | | Mir378b | 100422933 | 4-May-15 |
| 12799 | | | | | | Mir378c | | |
| 12800 | | | | | | Mir379 | 494328 | 21-May-15 |
| 12801 | | | | | | Mir380 | 494329 | 21-May-15 |
| 12802 | | | | | | Mir381 | 494330 | 21-May-15 |
| 12803 | | | | | | Mir382 | 494331 | 21-May-15 |
| 12804 | | | | | | Mir383 | 494332 | 21-May-15 |
| 12805 | | | | | | Mir384 | 494333 | 4-May-15 |
| 12806 | | | | | | Mir3960 | 100616250 | 5-May-15 |
| 12807 | | | | | | Mir3962 | | |
| 12808 | | | | | | Mir3963 | | |
| 12809 | | | | | | Mir3964 | | |
| 12810 | | | | | | Mir3965 | | |
| 12811 | | | | | | Mir3966 | | |
| 12812 | | | | | | Mir3967 | | |
| 12813 | | | | | | Mir3968 | | |
| 12814 | | | | | | Mir3969 | | |
| 12815 | | | | | | Mir3970 | | |
| 12816 | | | | | | Mir3971 | | |
| 12817 | | | | | | Mir409 | 574413 | 21-May-15 |
| 12818 | | | | | | Mir410 | 574434 | 21-May-15 |
| 12819 | | | | | | Mir411 | 693121 | 21-May-15 |
| 12820 | | | | | | Mir412 | 574433 | 21-May-15 |
| 12821 | | | | | | Mir421 | 693122 | 4-May-15 |
| 12822 | | | | | | Mir423 | 494335 | 21-May-15 |
| 12823 | | | | | | Mir425 | 494337 | 21-May-15 |
| 12824 | | | | | | Mir429 | 554210 | 21-May-15 |
| 12825 | | | | | | Mir431 | 574038 | 21-May-15 |
| 12826 | | | | | | Mir432 | 574451 | 21-May-15 |
| 12830 | | | | | | Mir449a | 554213 | 21-May-15 |
| 12831 | | | | | | Mir449b | 693123 | 21-May-15 |
| 12832 | | | | | | Mir449c | 100313923 | 21-May-15 |
| 12833 | | | | | | Mir450-1 | | |
| 12834 | | | | | | Mir450-2 | | |
| 12835 | | | | | | Mir450b | 100126302 | 21-May-15 |
| 12836 | | | | | | Mir451 | 574411 | 21-May-15 |
| 12837 | | | | | | Mir452 | 574412 | 21-May-15 |
| 12838 | | | | | | Mir453 | 574410 | 21-May-15 |
| 12839 | | | | | | Mir455 | 619556 | 21-May-15 |
| 12840 | | | | | | Mir463 | | |
| 12841 | | | | | | Mir465 | | |
| 12842 | | | | | | Mir465b-1 | | |
| 12843 | | | | | | Mir465c-1 | | |
| 12844 | | | | | | Mir465d | | |
| 12845 | | | | | | Mir466 | 100423038 | 4-May-15 |
| 12846 | | | | | | Mir4660 | 100616350 | 4-May-15 |
| 12847 | | | | | | Mir466b-2 | | |
| 12848 | | | | | | Mir466b-3 | | |
| 12849 | | | | | | Mir466d | | |
| 12850 | | | | | | Mir466f-1 | | |
| 12851 | | | | | | Mir466f-2 | | |
| 12852 | | | | | | Mir466f-3 | | |
| 12853 | | | | | | Mir466g | | |
| 12854 | | | | | | Mir466h | | |
| 12855 | | | | | | Mir466i | | |
| 12856 | | | | | | Mir466n | | |
| 12857 | | | | | | Mir466p | | |
| 12858 | | | | | | Mir467a-1 | | |
| 12859 | | | | | | Mir467a-10 | | |
| 12860 | | | | | | Mir467a-2 | | |
| 12861 | | | | | | Mir467a-3 | | |
| 12862 | | | | | | Mir467a-5 | | |
| 12863 | | | | | | Mir467a-7 | | |
| 12864 | | | | | | Mir467a-9 | | |
| 12865 | | | | | | Mir467b | | |
| 12866 | | | | | | Mir467c | | |
| 12867 | | | | | | Mir467d | | |
| 12868 | | | | | | Mir467e | | |
| 12869 | | | | | | Mir467f | | |
| 12870 | | | | | | Mir468 | | |
| 12871 | | | | | | Mir470 | | |
| 12872 | | | | | | Mir471 | | |
| 12873 | | | | | | Mir483 | 619552 | 24-May-15 |
| 12874 | | | | | | Mir484 | 619553 | 21-May-15 |
| 12875 | | | | | | Mir485 | 574436 | 21-May-15 |
| 12879 | | | | | | Mir489 | 574442 | 21-May-15 |
| 12880 | | | | | | Mir490 | 574443 | 24-May-15 |
| 12881 | | | | | | Mir491 | 574444 | 24-May-15 |
| 12882 | | | | | | Mir493 | 574450 | 21-May-15 |
| 12883 | | | | | | Mir494 | 574452 | 7-Jun-15 |
| 12884 | | | | | | Mir495 | 574453 | 21-May-15 |
| 12885 | | | | | | Mir496 | 574454 | 21-May-15 |
| 12886 | | | | | | Mir496b | | |
| 12887 | | | | | | Mir497 | 574456 | 7-Jun-15 |
| 12888 | | | | | | Mir497b | | |
| 12889 | | | | | | Mir499 | 574501 | 21-May-15 |
| 12890 | | | | | | Mir500 | 574502 | 21-May-15 |
| 12891 | | | | | | Mir501 | 574503 | 21-May-15 |
| 12892 | | | | | | Mir503 | 574506 | 21-May-15 |
| 12893 | | | | | | Mir504 | 574507 | 21-May-15 |
| 12894 | | | | | | Mir5046 | | |
| 12895 | | | | | | Mir505 | 574508 | 21-May-15 |
| 12896 | | | | | | Mir509 | | |
| 12897 | | | | | | Mir5098 | | |
| 12898 | | | | | | Mir5100 | 100847014 | 4-May-15 |
| 12899 | | | | | | Mir5101 | | |
| 12900 | | | | | | Mir5103 | | |
| 12901 | | | | | | Mir5104 | | |
| 12902 | | | | | | Mir5106 | | |
| 12903 | | | | | | Mir5107 | | |
| 12904 | | | | | | Mir5108 | | |
| 12905 | | | | | | Mir511 | 574445 | 21-May-15 |
| 12906 | | | | | | Mir5112 | 574445 | 21-May-15 |
| 12907 | | | | | | Mir5113 | | |
| 12908 | | | | | | Mir5114 | | |
| 12909 | | | | | | Mir5116 | | |
| 12910 | | | | | | Mir5119 | | |
| 12911 | | | | | | Mir5120 | | |
| 12912 | | | | | | Mir5121 | 574458 | 21-May-15 |
| 12913 | | | | | | Mir5122 | 574459 | 21-May-15 |
| 12914 | | | | | | Mir5123 | | |
| 12915 | | | | | | Mir5124 | | |
| 12916 | | | | | | Mir5125 | | |
| 12917 | | | | | | Mir5126 | | |
| 12918 | | | | | | Mir5127 | | |
| 12919 | | | | | | Mir5128 | | |
| 12920 | | | | | | Mir5129 | | |
| 12921 | | | | | | Mir5130 | | |
| 12922 | | | | | | Mir5131 | | |
| 12923 | | | | | | Mir5132 | | |
| 12924 | | | | | | Mir5133 | | |
| 12925 | | | | | | Mir5134 | | |
| 12926 | | | | | | Mir5135 | | |
| 12927 | | | | | | Mir5136 | | |
| 12928 | | | | | | Mir532 | 693124 | 21-May-15 |
| 12929 | | | | | | Mir539 | 664612 | 21-May-15 |
| 12930 | | | | | | Mir540 | | |
| 12931 | | | | | | Mir541 | 100126308 | 21-May-15 |
| 12932 | | | | | | Mir542 | 664617 | 21-May-15 |
| 12933 | | | | | | Mir543 | 100126335 | 21-May-15 |
| 12934 | | | | | | Mir544 | | |
| 12938 | | | | | | Mir5615-1 | | |
| 12939 | | | | | | Mir5615-2 | | |

Fig.22 - 117

| | | | | | | | | |
|---|---|---|---|---|---|---|---|---|
| 12940 | | | | | | Mir5616 | | |
| 12941 | | | | | | Mir5617 | | |
| 12942 | | | | | | Mir5618 | | |
| 12944 | | | | | | Mir5620 | | |
| 12945 | | | | | | Mir5621 | | |
| 12946 | | | | | | Mir5622 | | |
| 12947 | | | | | | Mir5623 | | |
| 12948 | | | | | | Mir5624 | | |
| 12949 | | | | | | Mir5625 | | |
| 12950 | | | | | | Mir5626 | | |
| 12951 | | | | | | Mir5627 | | |
| 12952 | | | | | | Mir568 | 693153 | 4-May-15 |
| 12953 | | | | | | Mir5709 | | |
| 12954 | | | | | | Mir5710 | | |
| 12955 | | | | | | Mir574 | 693159 | 21-May-15 |
| 12956 | | | | | | Mir582 | 693167 | 5-May-15 |
| 12957 | | | | | | Mir592 | 693177 | 21-May-15 |
| 12958 | | | | | | Mir598 | 693183 | 21-May-15 |
| 12959 | | | | | | Mir599 | 693184 | 4-May-15 |
| 12960 | | | | | | Mir615 | 693200 | 21-May-15 |
| 12965 | | | | | | Mir6241 | | |
| 12969 | | | | | | Mir6337 | | |
| 12970 | | | | | | Mir6338 | | |
| 12976 | | | | | | Mir6344 | | |
| 12977 | | | | | | Mir6345 | | |
| 12978 | | | | | | Mir6348 | | |
| 12979 | | | | | | Mir6349 | | |
| 12980 | | | | | | Mir6350 | | |
| 12981 | | | | | | Mir6352 | | |
| 12982 | | | | | | Mir6353 | | |
| 12983 | | | | | | Mir6354 | | |
| 12984 | | | | | | Mir6355 | | |
| 12985 | | | | | | Mir6356 | | |
| 12996 | | | | | | Mir6367 | | |
| 12997 | | | | | | Mir6368 | | |
| 12998 | | | | | | Mir6369 | | |
| 12999 | | | | | | Mir6370 | | |
| 13000 | | | | | | Mir6372 | | |
| 13001 | | | | | | Mir6373 | | |
| 13002 | | | | | | Mir6374 | | |
| 13003 | | | | | | Mir6375 | | |
| 13004 | | | | | | Mir6376 | | |
| 13005 | | | | | | Mir6378 | | |
| 13006 | | | | | | Mir6380 | | |
| 13007 | | | | | | Mir6381 | | |
| 13008 | | | | | | Mir6382 | | |
| 13009 | | | | | | Mir6383 | | |
| 13010 | | | | | | Mir6384 | | |
| 13013 | | | | | | Mir6387 | | |
| 13015 | | | | | | Mir6389 | | |
| 13016 | | | | | | Mir6390 | | |
| 13017 | | | | | | Mir6391 | | |
| 13024 | | | | | | Mir6398 | | |
| 13026 | | | | | | Mir6400 | | |
| 13027 | | | | | | Mir6401 | | |
| 13028 | | | | | | Mir6402 | | |
| 13033 | | | | | | Mir6407 | | |
| 13034 | | | | | | Mir6408 | | |
| 13035 | | | | | | Mir6409 | | |
| 13036 | | | | | | Mir6410 | | |
| 13037 | | | | | | Mir6411 | | |
| 13038 | | | | | | Mir6412 | | |
| 13039 | | | | | | Mir6413 | | |
| 13040 | | | | | | Mir6414 | | |
| 13041 | | | | | | Mir6415 | | |
| 13042 | | | | | | Mir6416 | | |
| 13043 | | | | | | Mir6417 | | |
| 13046 | | | | | | Mir6420 | | |
| 13052 | | | | | | Mir6539 | | |
| 13053 | | | | | | Mir654 | 724024 | 21-May-15 |
| 13054 | | | | | | Mir6540 | | |
| 13055 | | | | | | Mir6541 | | |
| 13056 | | | | | | Mir6546 | | |
| 13057 | | | | | | Mir664 | 100302234 | 21-May-15 |
| 13058 | | | | | | Mir665 | 100126315 | 21-May-15 |
| 13059 | | | | | | Mir666 | | |
| 13060 | | | | | | Mir667 | | |
| 13061 | | | | | | Mir668 | 768214 | 4-May-15 |
| 13062 | | | | | | Mir669a-1 | | |
| 13063 | | | | | | Mir669a-2 | | |
| 13064 | | | | | | Mir669a-3 | | |
| 13065 | | | | | | Mir669a-4 | | |
| 13066 | | | | | | Mir669b | | |
| 13067 | | | | | | Mir669c | | |
| 13068 | | | | | | Mir669e | | |
| 13069 | | | | | | Mir669g | | |
| 13070 | | | | | | Mir669h | | |
| 13071 | | | | | | Mir669i | | |
| 13072 | | | | | | Mir669j | | |
| 13073 | | | | | | Mir669k | | |
| 13074 | | | | | | Mir669m-1 | | |
| 13075 | | | | | | Mir669m-2 | | |
| 13076 | | | | | | Mir669p-1 | | |
| 13078 | | | | | | Mir671 | 768213 | 21-May-15 |
| 13079 | | | | | | Mir6715 | | |
| 13080 | | | | | | Mir672 | | |

| | | | | | | | | |
|---|---|---|---|---|---|---|---|---|
| 13081 | | | | | | Mir673 | | |
| 13083 | | | | | | Mir675 | 100033819 | 4-May-15 |
| 13084 | | | | | | Mir676 | 100500887 | 21-May-15 |
| 13085 | | | | | | Mir6769b | 102466202 | 4-May-15 |
| 13087 | | | | | | Mir678 | | |
| 13089 | | | | | | Mir680-2 | | |
| 13090 | | | | | | Mir680-3 | | |
| 13091 | | | | | | Mir681 | | |
| 13098 | | | | | | Mir687 | | |
| 13099 | | | | | | Mir688 | | |
| 13100 | | | | | | Mir6896 | | |
| 13101 | | | | | | Mir6897 | | |
| 13103 | | | | | | Mir6899 | | |
| 13104 | | | | | | Mir690 | | |
| 13106 | | | | | | Mir6901 | | |
| 13107 | | | | | | Mir6902 | | |
| 13109 | | | | | | Mir6904 | | |
| 13110 | | | | | | Mir6905 | | |
| 13111 | | | | | | Mir6906 | | |
| 13112 | | | | | | Mir6907 | | |
| 13113 | | | | | | Mir6908 | | |
| 13114 | | | | | | Mir6909 | | |
| 13115 | | | | | | Mir691 | | |
| 13116 | | | | | | Mir6910 | | |
| 13117 | | | | | | Mir6911 | | |
| 13118 | | | | | | Mir6912 | | |
| 13119 | | | | | | Mir6913 | | |
| 13120 | | | | | | Mir6914 | | |
| 13121 | | | | | | Mir6915 | | |
| 13122 | | | | | | Mir6916 | | |
| 13123 | | | | | | Mir6917 | | |
| 13124 | | | | | | Mir6918 | | |
| 13125 | | | | | | Mir6919 | | |
| 13128 | | | | | | Mir692-1 | | |
| 13131 | | | | | | Mir6923 | | |
| 13132 | | | | | | Mir6924 | | |
| 13133 | | | | | | Mir6925 | | |
| 13135 | | | | | | Mir6927 | | |
| 13136 | | | | | | Mir6928 | | |
| 13137 | | | | | | Mir6929 | | |
| 13138 | | | | | | Mir693 | | |
| 13139 | | | | | | Mir6930 | | |
| 13140 | | | | | | Mir6931 | | |
| 13141 | | | | | | Mir6932 | | |
| 13142 | | | | | | Mir6933 | | |
| 13143 | | | | | | Mir6934 | | |
| 13144 | | | | | | Mir6935 | | |
| 13146 | | | | | | Mir6937 | | |
| 13147 | | | | | | Mir6938 | | |
| 13148 | | | | | | Mir6939 | | |
| 13149 | | | | | | Mir694 | | |
| 13150 | | | | | | Mir6940 | | |
| 13151 | | | | | | Mir6941 | | |
| 13152 | | | | | | Mir6942 | | |
| 13153 | | | | | | Mir6943 | | |
| 13154 | | | | | | Mir6944 | | |
| 13155 | | | | | | Mir6945 | | |
| 13156 | | | | | | Mir6946 | | |
| 13157 | | | | | | Mir6947 | | |
| 13158 | | | | | | Mir6948 | | |
| 13159 | | | | | | Mir6949 | | |
| 13160 | | | | | | Mir695 | | |
| 13161 | | | | | | Mir6950 | | |
| 13162 | | | | | | Mir6951 | | |
| 13163 | | | | | | Mir6952 | | |
| 13165 | | | | | | Mir6954 | | |
| 13166 | | | | | | Mir6955 | | |
| 13167 | | | | | | Mir6956 | | |
| 13168 | | | | | | Mir6957 | | |
| 13169 | | | | | | Mir6958 | | |
| 13170 | | | | | | Mir6959 | | |
| 13171 | | | | | | Mir6960 | | |
| 13172 | | | | | | Mir6961 | | |
| 13173 | | | | | | Mir6962 | | |
| 13174 | | | | | | Mir6963 | | |
| 13175 | | | | | | Mir6964 | | |
| 13176 | | | | | | Mir6965 | | |
| 13177 | | | | | | Mir6966 | | |
| 13178 | | | | | | Mir6968 | | |
| 13179 | | | | | | Mir6969 | | |
| 13182 | | | | | | Mir6971 | | |
| 13184 | | | | | | Mir6973a | | |
| 13185 | | | | | | Mir6973b | | |
| 13186 | | | | | | Mir6974 | | |
| 13187 | | | | | | Mir6975 | | |
| 13188 | | | | | | Mir6976 | | |
| 13189 | | | | | | Mir6977 | | |
| 13190 | | | | | | Mir6978 | | |
| 13191 | | | | | | Mir6979 | | |
| 13192 | | | | | | Mir698 | | |
| 13193 | | | | | | Mir6980 | | |
| 13197 | | | | | | Mir6984 | | |
| 13198 | | | | | | Mir6985 | | |
| 13199 | | | | | | Mir6986 | | |
| 13200 | | | | | | Mir6987 | | |

Fig.22 - 118

| | | | | | | | | |
|---|---|---|---|---|---|---|---|---|
| 13201 | | | | | | Mir6988 | | |
| 13202 | | | | | | Mir6989 | | |
| 13203 | | | | | | Mir6990 | | |
| 13204 | | | | | | Mir6991 | | |
| 13207 | | | | | | Mir6994 | | |
| 13209 | | | | | | Mir6996 | | |
| 13210 | | | | | | Mir6997 | | |
| 13211 | | | | | | Mir6998 | | |
| 13212 | | | | | | Mir6999 | | |
| 13215 | | | | | | Mir7001 | | |
| 13216 | | | | | | Mir7002 | | |
| 13217 | | | | | | Mir7003 | | |
| 13218 | | | | | | Mir7004 | | |
| 13219 | | | | | | Mir7005 | | |
| 13220 | | | | | | Mir7006 | | |
| 13221 | | | | | | Mir7007 | | |
| 13222 | | | | | | Mir7008 | | |
| 13223 | | | | | | Mir7009 | | |
| 13224 | | | | | | Mir701 | | |
| 13225 | | | | | | Mir7010 | | |
| 13226 | | | | | | Mir7011 | | |
| 13227 | | | | | | Mir7012 | | |
| 13228 | | | | | | Mir7013 | | |
| 13229 | | | | | | Mir7014 | | |
| 13230 | | | | | | Mir7015 | | |
| 13231 | | | | | | Mir7016 | | |
| 13232 | | | | | | Mir7017 | | |
| 13233 | | | | | | Mir7018 | | |
| 13234 | | | | | | Mir7019 | | |
| 13235 | | | | | | Mir702 | | |
| 13236 | | | | | | Mir7020 | | |
| 13237 | | | | | | Mir7021 | | |
| 13238 | | | | | | Mir7022 | | |
| 13239 | | | | | | Mir7023 | | |
| 13240 | | | | | | Mir7024 | | |
| 13242 | | | | | | Mir7026 | | |
| 13243 | | | | | | Mir7027 | | |
| 13244 | | | | | | Mir7028 | | |
| 13245 | | | | | | Mir7029 | | |
| 13250 | | | | | | Mir7033 | | |
| 13251 | | | | | | Mir7034 | | |
| 13252 | | | | | | Mir7035 | | |
| 13253 | | | | | | Mir7036 | | |
| 13254 | | | | | | Mir7036b | | |
| 13255 | | | | | | Mir7037 | | |
| 13256 | | | | | | Mir7038 | | |
| 13257 | | | | | | Mir7039 | | |
| 13258 | | | | | | Mir704 | | |
| 13259 | | | | | | Mir7040 | | |
| 13260 | | | | | | Mir7041 | | |
| 13261 | | | | | | Mir7042 | | |
| 13262 | | | | | | Mir7043 | | |
| 13263 | | | | | | Mir7044 | | |
| 13264 | | | | | | Mir7045 | | |
| 13265 | | | | | | Mir7046 | | |
| 13266 | | | | | | Mir7047 | | |
| 13267 | | | | | | Mir7048 | | |
| 13268 | | | | | | Mir7049 | | |
| 13269 | | | | | | Mir705 | | |
| 13270 | | | | | | Mir7050 | | |
| 13271 | | | | | | Mir7051 | | |
| 13272 | | | | | | Mir7052 | | |
| 13273 | | | | | | Mir7053 | | |
| 13274 | | | | | | Mir7054 | | |
| 13275 | | | | | | Mir7055 | | |
| 13276 | | | | | | Mir7056 | | |
| 13277 | | | | | | Mir7057 | | |
| 13278 | | | | | | Mir7058 | | |
| 13279 | | | | | | Mir7059 | | |
| 13280 | | | | | | Mir706 | | |
| 13284 | | | | | | Mir7063 | | |
| 13285 | | | | | | Mir7064 | | |
| 13286 | | | | | | Mir7065 | | |
| 13287 | | | | | | Mir7066 | | |
| 13288 | | | | | | Mir7067 | | |
| 13289 | | | | | | Mir7068 | | |
| 13290 | | | | | | Mir7069 | | |
| 13291 | | | | | | Mir707 | | |
| 13292 | | | | | | Mir7070 | | |
| 13293 | | | | | | Mir7071 | | |
| 13294 | | | | | | Mir7072 | | |
| 13295 | | | | | | Mir7073 | | |
| 13296 | | | | | | Mir7074 | | |
| 13297 | | | | | | Mir7075 | | |
| 13298 | | | | | | Mir7076 | | |
| 13299 | | | | | | Mir7077 | | |
| 13300 | | | | | | Mir7078 | | |
| 13301 | | | | | | Mir7079 | | |
| 13302 | | | | | | Mir708 | 100126333 | 4-May-15 |
| 13303 | | | | | | Mir7080 | | |
| 13304 | | | | | | Mir7081 | | |
| 13305 | | | | | | Mir7082 | | |
| 13306 | | | | | | Mir7083 | | |
| 13307 | | | | | | Mir7084 | | |
| 13309 | | | | | | Mir7086 | | |
| 13310 | | | | | | Mir7087 | | |
| 13311 | | | | | | Mir7088 | | |
| 13312 | | | | | | Mir7089 | | |
| 13313 | | | | | | Mir709 | | |
| 13314 | | | | | | Mir7090 | | |
| 13315 | | | | | | Mir7091 | | |
| 13316 | | | | | | Mir7092 | | |
| 13317 | | | | | | Mir7093 | | |
| 13318 | | | | | | Mir7094-1 | | |
| 13319 | | | | | | Mir7094-2 | | |
| 13321 | | | | | | Mir710 | | |
| 13322 | | | | | | Mir711 | 100313843 | 4-May-15 |
| 13323 | | | | | | Mir7115 | | |
| 13324 | | | | | | Mir7117 | | |
| 13325 | | | | | | Mir7118 | | |
| 13326 | | | | | | Mir7119 | | |
| 13327 | | | | | | Mir713 | | |
| 13329 | | | | | | Mir718 | 100313781 | 4-May-15 |
| 13335 | | | | | | Mir7212 | | |
| 13336 | | | | | | Mir7213 | | |
| 13337 | | | | | | Mir7214 | | |
| 13338 | | | | | | Mir7215 | | |
| 13339 | | | | | | Mir7216 | | |
| 13340 | | | | | | Mir7217 | | |
| 13341 | | | | | | Mir7218 | | |
| 13342 | | | | | | Mir7219 | | |
| 13343 | | | | | | Mir7220 | | |
| 13344 | | | | | | Mir7221 | | |
| 13345 | | | | | | Mir7222 | | |
| 13346 | | | | | | Mir7223 | | |
| 13347 | | | | | | Mir7224 | | |
| 13348 | | | | | | Mir7225 | | |
| 13349 | | | | | | Mir7226 | | |
| 13350 | | | | | | Mir7227 | | |
| 13351 | | | | | | Mir7228 | | |
| 13352 | | | | | | Mir7229 | | |
| 13353 | | | | | | Mir7230 | | |
| 13354 | | | | | | Mir7231 | | |
| 13355 | | | | | | Mir7232 | | |
| 13356 | | | | | | Mir7233 | | |
| 13357 | | | | | | Mir7234 | | |
| 13358 | | | | | | Mir7235 | | |
| 13359 | | | | | | Mir7236 | | |
| 13360 | | | | | | Mir7237 | | |
| 13361 | | | | | | Mir7238 | | |
| 13362 | | | | | | Mir7239 | | |
| 13363 | | | | | | Mir7240 | | |
| 13365 | | | | | | Mir7242 | | |
| 13366 | | | | | | Mir7243 | | |
| 13367 | | | | | | Mir741 | | |
| 13368 | | | | | | Mir742 | | |
| 13369 | | | | | | Mir743 | | |
| 13370 | | | | | | Mir743b | | |
| 13371 | | | | | | Mir744 | 100126313 | 21-May-15 |
| 13372 | | | | | | Mir7578 | | |
| 13373 | | | | | | Mir758 | 768212 | 21-May-15 |
| 13374 | | | | | | Mir759 | 100313778 | 4-May-15 |
| 13379 | | | | | | Mir7646 | | |
| 13380 | | | | | | Mir7647 | | |
| 13381 | | | | | | Mir7648 | | |
| 13382 | | | | | | Mir7649 | | |
| 13383 | | | | | | Mir7650 | | |
| 13384 | | | | | | Mir7652 | | |
| 13385 | | | | | | Mir7653 | | |
| 13386 | | | | | | Mir7654 | | |
| 13387 | | | | | | Mir7655 | | |
| 13388 | | | | | | Mir7656 | | |
| 13389 | | | | | | Mir7657 | | |
| 13390 | | | | | | Mir7658 | | |
| 13391 | | | | | | Mir7661 | | |
| 13392 | | | | | | Mir7662 | | |
| 13393 | | | | | | Mir7663 | | |
| 13394 | | | | | | Mir7665 | | |
| 13395 | | | | | | Mir7666 | | |
| 13396 | | | | | | Mir7667 | | |
| 13397 | | | | | | Mir7668 | | |
| 13398 | | | | | | Mir7669 | | |
| 13400 | | | | | | Mir7670 | | |
| 13401 | | | | | | Mir7671 | | |
| 13402 | | | | | | Mir7672 | | |
| 13403 | | | | | | Mir7673 | | |
| 13404 | | | | | | Mir7674 | | |
| 13405 | | | | | | Mir7675 | | |
| 13406 | | | | | | Mir7676-2 | | |
| 13407 | | | | | | Mir7677 | | |
| 13408 | | | | | | Mir7678 | | |
| 13409 | | | | | | Mir7679 | | |
| 13410 | | | | | | Mir7680 | | |
| 13411 | | | | | | Mir7681 | | |
| 13412 | | | | | | Mir7682 | | |
| 13413 | | | | | | Mir7684 | | |
| 13414 | | | | | | Mir7685 | | |
| 13415 | | | | | | Mir7686 | | |
| 13416 | | | | | | Mir7687 | | |
| 13417 | | | | | | Mir770 | 768222 | 4-May-15 |
| 13418 | | | | | | Mir7b | | |
| 13419 | | | | | | Mir802 | 768219 | 17-May-15 |

Fig.22 - 119

| | | | | | | | | |
|---|---|---|---|---|---|---|---|---|
| 13420 | | | | | | Mir804 | | |
| 13440 | | | | | | Mir8110 | | |
| 13450 | | | | | | Mir871 | | |
| 13452 | | | | | | Mir873b | 100126 316 | 21-May-15 |
| 13453 | | | | | | Mir874 | 100126 343 | 21-May-15 |
| 13454 | | | | | | Mir875 | 100126 309 | 4-May-15 |
| 13455 | | | | | | Mir876 | 100126 310 | 21-May-15 |
| 13456 | | | | | | Mir877 | 100126 314 | 21-May-15 |
| 13457 | | | | | | Mir878 | | |
| 13458 | | | | | | Mir879 | | |
| 13459 | | | | | | Mir880 | | |
| 13460 | | | | | | Mir881 | | |
| 13461 | | | | | | Mir882 | | |
| 13462 | | | | | | Mir883a | | |
| 13463 | | | | | | Mir883b | | |
| 13464 | | | | | | Mir9-1 | 407046 | 21-May-15 |
| 13465 | | | | | | Mir9-2 | 407047 | 21-May-15 |
| 13466 | | | | | | Mir92-1 | 407048 | 21-May-15 |
| 13467 | | | | | | Mir92-2 | | |
| 13468 | | | | | | Mir92b | 693235 | 21-May-15 |
| 13469 | | | | | | Mir93 | 407050 | 21-May-15 |
| 13470 | | | | | | Mir9-3 | 407051 | 21-May-15 |
| 13471 | | | | | | Mir96 | 407053 | 7-Jun-15 |
| 13472 | | | | | | Mir98 | 407054 | 21-May-15 |
| 13473 | | | | | | Mir99a | 407055 | 21-May-15 |
| 13474 | | | | | | Mir99b | 407056 | 21-May-15 |
| 13475 | | | | | | Mira | | |
| 13487 | | | | | | Mirlet7g | 406890 | 21-May-15 |
| 13488 | | | | | | Mirlet7i | 406891 | 21-May-15 |
| 13489 | | | | | | Mirlet7j | | |
| 13490 | | | | | | Mirlet7k | | |
| 13492 | | | | | | Mis18a | 54069 | 12-May-15 |
| 13493 | | | | | | Mis18bp1 | 55320 | 4-May-15 |
| 13602 | | | | | | Morc3 | 23515 | 4-May-15 |
| 14903 | | | | | | Olfr1026 | | |
| 14928 | | | | | | Olfr1054 | | |
| 14929 | | | | | | Olfr1055 | | |
| 14930 | | | | | | Olfr1056 | | |
| 14932 | | | | | | Olfr1058 | | |
| 14943 | | | | | | Olfr1082 | | |
| 14944 | | | | | | Olfr1084 | | |
| 14945 | | | | | | Olfr1085 | | |
| 14946 | | | | | | Olfr1086 | | |
| 14947 | | | | | | Olfr1087 | | |
| 14948 | | | | | | Olfr1089 | | |
| 14949 | | | | | | Olfr109 | | |
| 14950 | | | | | | Olfr1090 | | |
| 14953 | | | | | | Olfr1095 | | |
| 14962 | | | | | | Olfr1104 | | |
| 14971 | | | | | | Olfr1113 | | |
| 14972 | | | | | | Olfr1115 | | |
| 14988 | | | | | | Olfr1133 | | |
| 14997 | | | | | | Olfr1143 | | |
| 14998 | | | | | | Olfr1145 | | |
| 14999 | | | | | | Olfr1148 | | |
| 15000 | | | | | | Olfr115 | | |
| 15001 | | | | | | Olfr1151 | | |
| 15002 | | | | | | Olfr1152 | | |
| 15003 | | | | | | Olfr1153 | | |
| 15004 | | | | | | Olfr1154 | | |
| 15005 | | | | | | Olfr1155 | | |
| 15006 | | | | | | Olfr1156 | | |
| 15007 | | | | | | Olfr1157 | | |
| 15008 | | | | | | Olfr1158 | | |
| 15009 | | | | | | Olfr116 | | |
| 15010 | | | | | | Olfr1160 | | |
| 15011 | | | | | | Olfr1161 | | |
| 15012 | | | | | | Olfr1162 | | |
| 15013 | | | | | | Olfr1163 | | |
| 15014 | | | | | | Olfr1164 | | |
| 15015 | | | | | | Olfr1166 | | |
| 15016 | | | | | | Olfr1167 | | |
| 15017 | | | | | | Olfr1168 | | |
| 15018 | | | | | | Olfr117 | | |
| 15019 | | | | | | Olfr1170 | | |
| 15020 | | | | | | Olfr1173 | | |
| 15021 | | | | | | Olfr1176 | | |
| 15022 | | | | | | Olfr1178 | | |
| 15023 | | | | | | Olfr1179 | | |
| 15024 | | | | | | Olfr118 | | |
| 15025 | | | | | | Olfr1180 | | |
| 15028 | | | | | | Olfr1183 | | |
| 15029 | | | | | | Olfr1184 | | |
| 15030 | | | | | | Olfr1186 | | |
| 15031 | | | | | | Olfr1188 | | |
| 15032 | | | | | | Olfr1189 | | |
| 15033 | | | | | | Olfr119 | | |
| 15034 | | | | | | Olfr1193 | | |
| 15035 | | | | | | Olfr1195 | | |
| 15036 | | | | | | Olfr1196 | | |
| 15037 | | | | | | Olfr1197 | | |
| 15038 | | | | | | Olfr1198 | | |
| 15039 | | | | | | Olfr1199 | | |
| 15040 | | | | | | Olfr12 | | |
| 15041 | | | | | | Olfr120 | | |
| 15042 | | | | | | Olfr1200 | | |
| 15043 | | | | | | Olfr1201 | | |
| 15044 | | | | | | Olfr1202 | | |
| 15045 | | | | | | Olfr1204 | | |
| 15046 | | | | | | Olfr1205 | | |
| 15047 | | | | | | Olfr1206 | | |
| 15048 | | | | | | Olfr1208 | | |
| 15049 | | | | | | Olfr1209 | | |
| 15050 | | | | | | Olfr121 | | |
| 15051 | | | | | | Olfr1211 | | |
| 15052 | | | | | | Olfr1212 | | |
| 15053 | | | | | | Olfr1213 | | |
| 15054 | | | | | | Olfr1214 | | |
| 15055 | | | | | | Olfr1215 | | |
| 15056 | | | | | | Olfr1216 | | |
| 15057 | | | | | | Olfr1217 | | |
| 15058 | | | | | | Olfr1218 | | |
| 15059 | | | | | | Olfr1219 | | |
| 15060 | | | | | | Olfr122 | | |
| 15061 | | | | | | Olfr1220 | | |
| 15062 | | | | | | Olfr1221 | | |
| 15063 | | | | | | Olfr1222 | | |
| 15064 | | | | | | Olfr1223 | | |
| 15065 | | | | | | Olfr1225 | | |
| 15066 | | | | | | Olfr1226 | | |
| 15067 | | | | | | Olfr1228 | | |
| 15068 | | | | | | Olfr1229 | | |
| 15069 | | | | | | Olfr123 | | |
| 15070 | | | | | | Olfr1230 | | |
| 15071 | | | | | | Olfr1231 | | |
| 15072 | | | | | | Olfr1232 | | |
| 15073 | | | | | | Olfr1233 | | |
| 15074 | | | | | | Olfr1234 | | |
| 15075 | | | | | | Olfr1238 | | |
| 15076 | | | | | | Olfr1239 | | |
| 15077 | | | | | | Olfr124 | | |
| 15078 | | | | | | Olfr1240 | | |
| 15079 | | | | | | Olfr1241 | | |
| 15080 | | | | | | Olfr1242 | | |
| 15081 | | | | | | Olfr1243 | | |
| 15082 | | | | | | Olfr1245 | | |
| 15083 | | | | | | Olfr1246 | | |
| 15084 | | | | | | Olfr1247 | | |
| 15085 | | | | | | Olfr1248 | | |
| 15086 | | | | | | Olfr1249 | | |
| 15087 | | | | | | Olfr125 | | |
| 15088 | | | | | | Olfr1250 | | |
| 15089 | | | | | | Olfr1251 | | |
| 15090 | | | | | | Olfr1252 | | |
| 15091 | | | | | | Olfr1253 | | |
| 15092 | | | | | | Olfr1254 | | |
| 15093 | | | | | | Olfr1255 | | |
| 15094 | | | | | | Olfr1256 | | |
| 15095 | | | | | | Olfr1257 | | |
| 15096 | | | | | | Olfr1258 | | |
| 15097 | | | | | | Olfr1259 | | |
| 15098 | | | | | | Olfr126 | | |
| 15099 | | | | | | Olfr1260 | | |
| 15100 | | | | | | Olfr1261 | | |
| 15101 | | | | | | Olfr1262 | | |
| 15102 | | | | | | Olfr1263 | | |
| 15103 | | | | | | Olfr1264 | | |
| 15104 | | | | | | Olfr1265 | | |
| 15105 | | | | | | Olfr1269 | | |
| 15106 | | | | | | Olfr127 | | |
| 15108 | | | | | | Olfr1271 | | |
| 15110 | | | | | | Olfr1273-ps | | |
| 15111 | | | | | | Olfr1274-ps | | |
| 15112 | | | | | | Olfr1275 | | |
| 15113 | | | | | | Olfr1276 | | |
| 15114 | | | | | | Olfr1277 | | |
| 15115 | | | | | | Olfr1278 | | |
| 15116 | | | | | | Olfr1279 | | |
| 15117 | | | | | | Olfr128 | | |
| 15118 | | | | | | Olfr1280 | | |
| 15121 | | | | | | Olfr1283 | | |
| 15122 | | | | | | Olfr1284 | | |
| 15123 | | | | | | Olfr1286 | | |
| 15124 | | | | | | Olfr1287 | | |
| 15125 | | | | | | Olfr1288 | | |
| 15126 | | | | | | Olfr1289 | | |
| 15127 | | | | | | Olfr129 | | |
| 15128 | | | | | | Olfr1290 | | |
| 15129 | | | | | | Olfr1294 | | |
| 15130 | | | | | | Olfr1295 | | |
| 15131 | | | | | | Olfr1297 | | |
| 15132 | | | | | | Olfr1298 | | |
| 15133 | | | | | | Olfr1299 | | |
| 15134 | | | | | | Olfr13 | | |
| 15135 | | | | | | Olfr130 | | |
| 15136 | | | | | | Olfr1300-ps1 | | |
| 15138 | | | | | | Olfr1302 | | |
| 15139 | | | | | | Olfr1303 | | |
| 15140 | | | | | | Olfr1305 | | |
| 15141 | | | | | | Olfr1306 | | |
| 15142 | | | | | | Olfr1307 | | |

Fig.22 - 120

| | | | | | | | | |
|---|---|---|---|---|---|---|---|---|
| 15143 | | | | | | Olfr1308 | | |
| 15144 | | | | | | Olfr1309 | | |
| 15145 | | | | | | Olfr131 | | |
| 15146 | | | | | | Olfr1310 | | |
| 15147 | | | | | | Olfr1311 | | |
| 15148 | | | | | | Olfr1312 | | |
| 15149 | | | | | | Olfr1313 | | |
| 15150 | | | | | | Olfr1314 | | |
| 15151 | | | | | | Olfr1316 | | |
| 15152 | | | | | | Olfr1317 | | |
| 15153 | | | | | | Olfr1318 | | |
| 15154 | | | | | | Olfr132 | | |
| 15155 | | | | | | Olfr1320 | | |
| 15156 | | | | | | Olfr1321 | | |
| 15157 | | | | | | Olfr1322 | | |
| 15158 | | | | | | Olfr1323 | | |
| 15159 | | | | | | Olfr1324 | | |
| 15160 | | | | | | Olfr1325 | | |
| 15161 | | | | | | Olfr1328 | | |
| 15162 | | | | | | Olfr1329 | | |
| 15163 | | | | | | Olfr133 | | |
| 15164 | | | | | | Olfr1330 | | |
| 15165 | | | | | | Olfr1331 | | |
| 15166 | | | | | | Olfr1333 | | |
| 15167 | | | | | | Olfr1335 | | |
| 15168 | | | | | | Olfr1336 | | |
| 15169 | | | | | | Olfr1337 | | |
| 15170 | | | | | | Olfr1338 | | |
| 15171 | | | | | | Olfr1339 | | |
| 15172 | | | | | | Olfr134 | | |
| 15174 | | | | | | Olfr1341 | | |
| 15175 | | | | | | Olfr1342 | | |
| 15176 | | | | | | Olfr1344 | | |
| 15178 | | | | | | Olfr1347 | | |
| 15180 | | | | | | Olfr1349 | | |
| 15181 | | | | | | Olfr135 | | |
| 15182 | | | | | | Olfr1350 | | |
| 15183 | | | | | | Olfr1351 | | |
| 15184 | | | | | | Olfr1352 | | |
| 15185 | | | | | | Olfr1353 | | |
| 15186 | | | | | | Olfr1354 | | |
| 15187 | | | | | | Olfr1355 | | |
| 15188 | | | | | | Olfr1356 | | |
| 15189 | | | | | | Olfr1357 | | |
| 15190 | | | | | | Olfr1359 | | |
| 15191 | | | | | | Olfr136 | | |
| 15192 | | | | | | Olfr1360 | | |
| 15193 | | | | | | Olfr1361 | | |
| 15194 | | | | | | Olfr1362 | | |
| 15195 | | | | | | Olfr1364 | | |
| 15196 | | | | | | Olfr1366 | | |
| 15197 | | | | | | Olfr1367 | | |
| 15198 | | | | | | Olfr1368 | | |
| 15199 | | | | | | Olfr137 | | |
| 15200 | | | | | | Olfr1370 | | |
| 15201 | | | | | | Olfr1371 | | |
| 15206 | | | | | | Olfr138 | | |
| 15207 | | | | | | Olfr1380 | | |
| 15208 | | | | | | Olfr1381 | | |
| 15209 | | | | | | Olfr1382 | | |
| 15212 | | | | | | Olfr1385 | | |
| 15215 | | | | | | Olfr1388 | | |
| 15216 | | | | | | Olfr1389 | | |
| 15217 | | | | | | Olfr139 | | |
| 15218 | | | | | | Olfr1390 | | |
| 15219 | | | | | | Olfr1391 | | |
| 15220 | | | | | | Olfr1392 | | |
| 15221 | | | | | | Olfr1393 | | |
| 15228 | | | | | | Olfr1406 | | |
| 15229 | | | | | | Olfr1408 | | |
| 15230 | | | | | | Olfr141 | | |
| 15231 | | | | | | Olfr1410 | | |
| 15232 | | | | | | Olfr1411 | | |
| 15234 | | | | | | Olfr1413 | | |
| 15235 | | | | | | Olfr1414 | | |
| 15236 | | | | | | Olfr1415 | | |
| 15237 | | | | | | Olfr1416 | | |
| 15238 | | | | | | Olfr1417 | | |
| 15239 | | | | | | Olfr1418 | | |
| 15240 | | | | | | Olfr1419 | | |
| 15242 | | | | | | Olfr1420 | | |
| 15246 | | | | | | Olfr1426 | | |
| 15247 | | | | | | Olfr1427 | | |
| 15248 | | | | | | Olfr1428 | | |
| 15249 | | | | | | Olfr143 | | |
| 15250 | | | | | | Olfr1431 | | |
| 15251 | | | | | | Olfr1433 | | |
| 15252 | | | | | | Olfr1434 | | |
| 15253 | | | | | | Olfr1436 | | |
| 15254 | | | | | | Olfr1437 | | |
| 15255 | | | | | | Olfr1440 | | |
| 15256 | | | | | | Olfr1441 | | |
| 15257 | | | | | | Olfr1442 | | |
| 15258 | | | | | | Olfr1443 | | |
| 15259 | | | | | | Olfr1444 | | |
| 15260 | | | | | | Olfr1445 | | |
| 15261 | | | | | | Olfr1446 | | |
| 15262 | | | | | | Olfr1447 | | |
| 15263 | | | | | | Olfr1448 | | |
| 15264 | | | | | | Olfr1449 | | |
| 15265 | | | | | | Olfr145 | | |
| 15266 | | | | | | Olfr1450 | | |
| 15267 | | | | | | Olfr1451 | | |
| 15268 | | | | | | Olfr1453 | | |
| 15269 | | | | | | Olfr1454 | | |
| 15270 | | | | | | Olfr1457 | | |
| 15271 | | | | | | Olfr1459 | | |
| 15272 | | | | | | Olfr146 | | |
| 15273 | | | | | | Olfr1461 | | |
| 15274 | | | | | | Olfr1462 | | |
| 15275 | | | | | | Olfr1463 | | |
| 15276 | | | | | | Olfr1465 | | |
| 15277 | | | | | | Olfr1466 | | |
| 15278 | | | | | | Olfr1467 | | |
| 15279 | | | | | | Olfr1469 | | |
| 15280 | | | | | | Olfr147 | | |
| 15281 | | | | | | Olfr1471 | | |
| 15282 | | | | | | Olfr1472 | | |
| 15283 | | | | | | Olfr1474 | | |
| 15284 | | | | | | Olfr1475 | | |
| 15285 | | | | | | Olfr1477 | | |
| 15286 | | | | | | Olfr148 | | |
| 15287 | | | | | | Olfr1480 | | |
| 15288 | | | | | | Olfr1484 | | |
| 15289 | | | | | | Olfr1487 | | |
| 15290 | | | | | | Olfr1489 | | |
| 15291 | | | | | | Olfr149 | | |
| 15293 | | | | | | Olfr1491 | | |
| 15294 | | | | | | Olfr1494 | | |
| 15295 | | | | | | Olfr1495 | | |
| 15296 | | | | | | Olfr1496 | | |
| 15297 | | | | | | Olfr1497 | | |
| 15298 | | | | | | Olfr1499 | | |
| 15299 | | | | | | Olfr15 | | |
| 15300 | | | | | | Olfr150 | | |
| 15301 | | | | | | Olfr1500 | | |
| 15302 | | | | | | Olfr1501 | | |
| 15303 | | | | | | Olfr1502 | | |
| 15304 | | | | | | Olfr1504 | | |
| 15305 | | | | | | Olfr1505 | | |
| 15306 | | | | | | Olfr1506 | | |
| 15307 | | | | | | Olfr1507 | | |
| 15309 | | | | | | Olfr1509 | | |
| 15311 | | | | | | Olfr1510 | | |
| 15312 | | | | | | Olfr1511 | | |
| 15313 | | | | | | Olfr1512 | | |
| 15314 | | | | | | Olfr1513 | | |
| 15315 | | | | | | Olfr152 | | |
| 15316 | | | | | | Olfr153 | | |
| 15317 | | | | | | Olfr1532-ps1 | | |
| 15319 | | | | | | Olfr1537 | | |
| 15320 | | | | | | Olfr154 | | |
| 15321 | | | | | | Olfr155 | | |
| 15322 | | | | | | Olfr156 | | |
| 15323 | | | | | | Olfr157 | | |
| 15324 | | | | | | Olfr159 | | |
| 15325 | | | | | | Olfr16 | | |
| 15327 | | | | | | Olfr161 | | |
| 15328 | | | | | | Olfr164 | | |
| 15329 | | | | | | Olfr165 | | |
| 15333 | | | | | | Olfr169 | | |
| 15334 | | | | | | Olfr17 | | |
| 15335 | | | | | | Olfr170 | | |
| 15338 | | | | | | Olfr173 | | |
| 15339 | | | | | | Olfr175-ps1 | | |
| 15341 | | | | | | Olfr177 | | |
| 15342 | | | | | | Olfr178 | | |
| 15343 | | | | | | Olfr18 | | |
| 15344 | | | | | | Olfr180 | | |
| 15345 | | | | | | Olfr181 | | |
| 15346 | | | | | | Olfr183 | | |
| 15347 | | | | | | Olfr186 | | |
| 15348 | | | | | | Olfr187 | | |
| 15351 | | | | | | Olfr191 | | |
| 15353 | | | | | | Olfr193 | | |
| 15355 | | | | | | Olfr195 | | |
| 15356 | | | | | | Olfr196 | | |
| 15357 | | | | | | Olfr197 | | |
| 15359 | | | | | | Olfr199 | | |
| 15360 | | | | | | Olfr2 | 7932 | 4-May-15 |
| 15361 | | | | | | Olfr20 | | |
| 15363 | | | | | | Olfr202 | | |
| 15364 | | | | | | Olfr203 | | |
| 15365 | | | | | | Olfr204 | | |
| 15366 | | | | | | Olfr205 | | |
| 15367 | | | | | | Olfr206 | | |
| 15368 | | | | | | Olfr209 | | |
| 15369 | | | | | | Olfr211 | | |
| 15370 | | | | | | Olfr212 | | |
| 15371 | | | | | | Olfr213 | | |
| 15372 | | | | | | Olfr214 | | |
| 15374 | | | | | | Olfr218 | | |
| 15377 | | | | | | Olfr222 | | |
| 15378 | | | | | | Olfr223 | | |

Fig.22 - 121

| | | | | | | | | |
|---|---|---|---|---|---|---|---|---|
| 15379 | | | | | | Olfr224 | | |
| 15382 | | | | | | Olfr229 | | |
| 15383 | | | | | | Olfr23 | | |
| 15384 | | | | | | Olfr231 | | |
| 15385 | | | | | | Olfr235 | | |
| 15387 | | | | | | Olfr239 | | |
| 15388 | | | | | | Olfr24 | | |
| 15389 | | | | | | Olfr242 | | |
| 15390 | | | | | | Olfr243 | | |
| 15391 | | | | | | Olfr247 | | |
| 15392 | | | | | | Olfr248 | | |
| 15393 | | | | | | Olfr25 | | |
| 15394 | | | | | | Olfr259 | | |
| 15395 | | | | | | Olfr26 | | |
| 15396 | | | | | | Olfr262 | | |
| 15397 | | | | | | Olfr263 | | |
| 15398 | | | | | | Olfr266 | | |
| 15399 | | | | | | Olfr267 | | |
| 15400 | | | | | | Olfr27 | | |
| 15401 | | | | | | Olfr270 | | |
| 15402 | | | | | | Olfr272 | | |
| 15403 | | | | | | Olfr273 | | |
| 15404 | | | | | | Olfr275 | | |
| 15405 | | | | | | Olfr279 | | |
| 15406 | | | | | | Olfr281 | | |
| 15407 | | | | | | Olfr282 | | |
| 15408 | | | | | | Olfr283 | | |
| 15409 | | | | | | Olfr284 | | |
| 15411 | | | | | | Olfr286 | | |
| 15412 | | | | | | Olfr287 | | |
| 15413 | | | | | | Olfr288 | | |
| 15415 | | | | | | Olfr291 | | |
| 15416 | | | | | | Olfr292 | | |
| 15417 | | | | | | Olfr293 | | |
| 15418 | | | | | | Olfr294 | | |
| 15419 | | | | | | Olfr295 | | |
| 15420 | | | | | | Olfr297 | | |
| 15421 | | | | | | Olfr298 | | |
| 15422 | | | | | | Olfr299 | | |
| 15423 | | | | | | Olfr29-ps1 | | |
| 15425 | | | | | | Olfr30 | | |
| 15426 | | | | | | Olfr301 | | |
| 15427 | | | | | | Olfr303 | | |
| 15428 | | | | | | Olfr304 | | |
| 15429 | | | | | | Olfr305 | | |
| 15431 | | | | | | Olfr308 | | |
| 15432 | | | | | | Olfr309 | | |
| 15433 | | | | | | Olfr31 | | |
| 15434 | | | | | | Olfr310 | | |
| 15435 | | | | | | Olfr311 | | |
| 15437 | | | | | | Olfr313 | | |
| 15438 | | | | | | Olfr314 | | |
| 15439 | | | | | | Olfr315 | | |
| 15440 | | | | | | Olfr316 | | |
| 15441 | | | | | | Olfr317 | | |
| 15442 | | | | | | Olfr318 | | |
| 15446 | | | | | | Olfr322 | | |
| 15447 | | | | | | Olfr323 | | |
| 15448 | | | | | | Olfr324 | | |
| 15449 | | | | | | Olfr325 | | |
| 15450 | | | | | | Olfr328 | | |
| 15451 | | | | | | Olfr329-ps | | |
| 15452 | | | | | | Olfr33 | | |
| 15455 | | | | | | Olfr332 | | |
| 15457 | | | | | | Olfr339 | | |
| 15458 | | | | | | Olfr340 | | |
| 15459 | | | | | | Olfr341 | | |
| 15460 | | | | | | Olfr342 | | |
| 15461 | | | | | | Olfr344 | | |
| 15462 | | | | | | Olfr345 | | |
| 15463 | | | | | | Olfr346 | | |
| 15464 | | | | | | Olfr347 | | |
| 15466 | | | | | | Olfr350 | | |
| 15467 | | | | | | Olfr351 | | |
| 15468 | | | | | | Olfr352 | | |
| 15469 | | | | | | Olfr353 | | |
| 15470 | | | | | | Olfr354 | | |
| 15471 | | | | | | Olfr355 | | |
| 15472 | | | | | | Olfr356 | | |
| 15473 | | | | | | Olfr357 | | |
| 15474 | | | | | | Olfr358 | | |
| 15476 | | | | | | Olfr361 | | |
| 15478 | | | | | | Olfr365 | | |
| 15479 | | | | | | Olfr366 | | |
| 15480 | | | | | | Olfr367-ps | | |
| 15481 | | | | | | Olfr368 | | |
| 15482 | | | | | | Olfr370 | | |
| 15483 | | | | | | Olfr371 | | |
| 15484 | | | | | | Olfr372 | | |
| 15485 | | | | | | Olfr373 | | |
| 15487 | | | | | | Olfr376 | | |
| 15488 | | | | | | Olfr378 | | |
| 15489 | | | | | | Olfr38 | | |
| 15490 | | | | | | Olfr380 | | |
| 15491 | | | | | | Olfr381 | | |
| 15492 | | | | | | Olfr382 | | |
| 15493 | | | | | | Olfr384 | | |
| 15494 | | | | | | Olfr385 | | |
| 15496 | | | | | | Olfr39 | | |
| 15497 | | | | | | Olfr390 | | |
| 15498 | | | | | | Olfr391-ps | | |
| 15499 | | | | | | Olfr392 | | |
| 15500 | | | | | | Olfr393 | | |
| 15501 | | | | | | Olfr394 | | |
| 15502 | | | | | | Olfr395 | | |
| 15503 | | | | | | Olfr397 | | |
| 15504 | | | | | | Olfr398 | | |
| 15505 | | | | | | Olfr399 | | |
| 15506 | | | | | | Olfr401 | | |
| 15507 | | | | | | Olfr402 | | |
| 15508 | | | | | | Olfr403 | | |
| 15509 | | | | | | Olfr406 | | |
| 15510 | | | | | | Olfr410 | | |
| 15511 | | | | | | Olfr411 | | |
| 15512 | | | | | | Olfr412 | | |
| 15513 | | | | | | Olfr414 | | |
| 15514 | | | | | | Olfr417 | | |
| 15515 | | | | | | Olfr418-ps1 | | |
| 15516 | | | | | | Olfr419 | | |
| 15517 | | | | | | Olfr420 | | |
| 15518 | | | | | | Olfr421-ps1 | | |
| 15519 | | | | | | Olfr424 | | |
| 15522 | | | | | | Olfr429 | | |
| 15523 | | | | | | Olfr43 | | |
| 15524 | | | | | | Olfr430 | | |
| 15525 | | | | | | Olfr432 | | |
| 15526 | | | | | | Olfr433 | | |
| 15527 | | | | | | Olfr434 | | |
| 15530 | | | | | | Olfr44 | | |
| 15531 | | | | | | Olfr441 | | |
| 15532 | | | | | | Olfr444 | | |
| 15533 | | | | | | Olfr446 | | |
| 15534 | | | | | | Olfr447 | | |
| 15535 | | | | | | Olfr448 | | |
| 15536 | | | | | | Olfr449 | | |
| 15537 | | | | | | Olfr45 | | |
| 15538 | | | | | | Olfr450 | | |
| 15539 | | | | | | Olfr452 | | |
| 15540 | | | | | | Olfr453 | | |
| 15541 | | | | | | Olfr455 | | |
| 15542 | | | | | | Olfr456 | | |
| 15543 | | | | | | Olfr457 | | |
| 15544 | | | | | | Olfr458 | | |
| 15545 | | | | | | Olfr459 | | |
| 15546 | | | | | | Olfr46 | | |
| 15547 | | | | | | Olfr460 | | |
| 15548 | | | | | | Olfr461 | | |
| 15550 | | | | | | Olfr463 | | |
| 15551 | | | | | | Olfr464 | | |
| 15552 | | | | | | Olfr466 | | |
| 15553 | | | | | | Olfr467 | | |
| 15554 | | | | | | Olfr469 | | |
| 15558 | | | | | | Olfr473 | | |
| 15559 | | | | | | Olfr474 | | |
| 15560 | | | | | | Olfr476 | | |
| 15561 | | | | | | Olfr477 | | |
| 15562 | | | | | | Olfr478 | | |
| 15563 | | | | | | Olfr479 | | |
| 15564 | | | | | | Olfr48 | | |
| 15565 | | | | | | Olfr480 | | |
| 15566 | | | | | | Olfr481 | | |
| 15567 | | | | | | Olfr482 | | |
| 15568 | | | | | | Olfr483 | | |
| 15569 | | | | | | Olfr484 | | |
| 15570 | | | | | | Olfr485 | | |
| 15571 | | | | | | Olfr486 | | |
| 15572 | | | | | | Olfr487 | | |
| 15573 | | | | | | Olfr488 | | |
| 15574 | | | | | | Olfr49 | | |
| 15575 | | | | | | Olfr490 | | |
| 15576 | | | | | | Olfr491 | | |
| 15577 | | | | | | Olfr492 | | |
| 15578 | | | | | | Olfr493 | | |
| 15579 | | | | | | Olfr494 | | |
| 15580 | | | | | | Olfr495 | | |
| 15581 | | | | | | Olfr497 | | |
| 15582 | | | | | | Olfr498 | | |
| 15583 | | | | | | Olfr5 | | |
| 15584 | | | | | | Olfr50 | | |
| 15585 | | | | | | Olfr502 | | |
| 15586 | | | | | | Olfr503 | | |
| 15587 | | | | | | Olfr504 | | |
| 15588 | | | | | | Olfr506 | | |
| 15589 | | | | | | Olfr507 | | |
| 15590 | | | | | | Olfr508 | | |
| 15591 | | | | | | Olfr509 | | |
| 15592 | | | | | | Olfr51 | | |
| 15593 | | | | | | Olfr510 | | |
| 15594 | | | | | | Olfr512 | | |
| 15595 | | | | | | Olfr513 | | |
| 15596 | | | | | | Olfr514 | | |
| 15597 | | | | | | Olfr516 | | |
| 15598 | | | | | | Olfr517 | | |
| 15599 | | | | | | Olfr518 | | |

Fig.22 - 122

| | | | | | | | | |
|---|---|---|---|---|---|---|---|---|
| 15600 | | | | | | Olfr519 | | |
| 15601 | | | | | | Olfr52 | | |
| 15602 | | | | | | Olfr520 | | |
| 15603 | | | | | | Olfr521 | | |
| 15604 | | | | | | Olfr522 | | |
| 15605 | | | | | | Olfr523 | | |
| 15606 | | | | | | Olfr524 | | |
| 15607 | | | | | | Olfr525 | | |
| 15608 | | | | | | Olfr527 | | |
| 15609 | | | | | | Olfr53 | | |
| 15610 | | | | | | Olfr530 | | |
| 15611 | | | | | | Olfr531 | | |
| 15612 | | | | | | Olfr532 | | |
| 15613 | | | | | | Olfr533 | | |
| 15614 | | | | | | Olfr535 | | |
| 15615 | | | | | | Olfr536 | | |
| 15616 | | | | | | Olfr538 | | |
| 15617 | | | | | | Olfr539 | | |
| 15618 | | | | | | Olfr54 | | |
| 15620 | | | | | | Olfr543 | | |
| 15622 | | | | | | Olfr545 | | |
| 15624 | | | | | | Olfr549 | | |
| 15626 | | | | | | Olfr550 | | |
| 15627 | | | | | | Olfr551 | | |
| 15628 | | | | | | Olfr552 | | |
| 15630 | | | | | | Olfr554 | | |
| 15634 | | | | | | Olfr558 | | |
| 15638 | | | | | | Olfr561 | | |
| 15639 | | | | | | Olfr564 | | |
| 15640 | | | | | | Olfr566 | | |
| 15641 | | | | | | Olfr568 | | |
| 15643 | | | | | | Olfr57 | | |
| 15644 | | | | | | Olfr570 | | |
| 15645 | | | | | | Olfr571 | | |
| 15646 | | | | | | Olfr572 | | |
| 15647 | | | | | | Olfr574 | | |
| 15648 | | | | | | Olfr575 | | |
| 15649 | | | | | | Olfr576 | | |
| 15650 | | | | | | Olfr577 | | |
| 15651 | | | | | | Olfr578 | | |
| 15652 | | | | | | Olfr58 | | |
| 15653 | | | | | | Olfr582 | | |
| 15654 | | | | | | Olfr583 | | |
| 15655 | | | | | | Olfr584 | | |
| 15656 | | | | | | Olfr585 | | |
| 15657 | | | | | | Olfr586 | | |
| 15658 | | | | | | Olfr589 | | |
| 15659 | | | | | | Olfr59 | | |
| 15660 | | | | | | Olfr591 | | |
| 15661 | | | | | | Olfr592 | | |
| 15662 | | | | | | Olfr593 | | |
| 15663 | | | | | | Olfr594 | | |
| 15664 | | | | | | Olfr596 | | |
| 15665 | | | | | | Olfr597 | | |
| 15666 | | | | | | Olfr598 | | |
| 15667 | | | | | | Olfr599 | | |
| 15668 | | | | | | Olfr6 | | |
| 15669 | | | | | | Olfr60 | | |
| 15670 | | | | | | Olfr600 | | |
| 15671 | | | | | | Olfr601 | | |
| 15672 | | | | | | Olfr603 | | |
| 15673 | | | | | | Olfr605 | | |
| 15674 | | | | | | Olfr606 | | |
| 15675 | | | | | | Olfr608 | | |
| 15676 | | | | | | Olfr609 | | |
| 15677 | | | | | | Olfr61 | | |
| 15678 | | | | | | Olfr610 | | |
| 15679 | | | | | | Olfr611 | | |
| 15680 | | | | | | Olfr612 | | |
| 15681 | | | | | | Olfr613 | | |
| 15682 | | | | | | Olfr615 | | |
| 15683 | | | | | | Olfr616 | | |
| 15684 | | | | | | Olfr617 | | |
| 15685 | | | | | | Olfr618 | | |
| 15686 | | | | | | Olfr619 | | |
| 15687 | | | | | | Olfr62 | | |
| 15688 | | | | | | Olfr620 | | |
| 15689 | | | | | | Olfr622 | | |
| 15690 | | | | | | Olfr623 | | |
| 15691 | | | | | | Olfr624 | | |
| 15692 | | | | | | Olfr628 | | |
| 15693 | | | | | | Olfr629 | | |
| 15694 | | | | | | Olfr63 | | |
| 15695 | | | | | | Olfr630 | | |
| 15696 | | | | | | Olfr631 | | |
| 15697 | | | | | | Olfr632 | | |
| 15698 | | | | | | Olfr633 | | |
| 15699 | | | | | | Olfr635 | | |
| 15700 | | | | | | Olfr638 | | |
| 15701 | | | | | | Olfr639 | | |
| 15702 | | | | | | Olfr64 | | |
| 15703 | | | | | | Olfr640 | | |
| 15704 | | | | | | Olfr641 | | |
| 15705 | | | | | | Olfr642 | | |
| 15706 | | | | | | Olfr643 | | |
| 15707 | | | | | | Olfr644 | | |
| 15708 | | | | | | Olfr645 | | |
| 15709 | | | | | | Olfr646 | | |
| 15710 | | | | | | Olfr648 | | |
| 15711 | | | | | | Olfr649 | | |
| 15712 | | | | | | Olfr65 | | |
| 15713 | | | | | | Olfr651 | | |
| 15714 | | | | | | Olfr652 | | |
| 15715 | | | | | | Olfr653 | | |
| 15716 | | | | | | Olfr654 | | |
| 15717 | | | | | | Olfr655 | | |
| 15718 | | | | | | Olfr656 | | |
| 15719 | | | | | | Olfr657 | | |
| 15720 | | | | | | Olfr658 | | |
| 15721 | | | | | | Olfr659 | | |
| 15722 | | | | | | Olfr66 | | |
| 15723 | | | | | | Olfr661 | | |
| 15724 | | | | | | Olfr663 | | |
| 15725 | | | | | | Olfr665 | | |
| 15726 | | | | | | Olfr666 | | |
| 15727 | | | | | | Olfr667 | | |
| 15728 | | | | | | Olfr668 | | |
| 15729 | | | | | | Olfr669 | | |
| 15730 | | | | | | Olfr67 | | |
| 15731 | | | | | | Olfr670 | | |
| 15732 | | | | | | Olfr671 | | |
| 15733 | | | | | | Olfr672 | | |
| 15734 | | | | | | Olfr675 | | |
| 15735 | | | | | | Olfr676 | | |
| 15737 | | | | | | Olfr678 | | |
| 15738 | | | | | | Olfr679 | | |
| 15739 | | | | | | Olfr68 | | |
| 15740 | | | | | | Olfr681 | | |
| 15741 | | | | | | Olfr683 | | |
| 15742 | | | | | | Olfr684 | | |
| 15743 | | | | | | Olfr685 | | |
| 15744 | | | | | | Olfr686 | | |
| 15745 | | | | | | Olfr688 | | |
| 15747 | | | | | | Olfr69 | | |
| 15748 | | | | | | Olfr690 | | |
| 15749 | | | | | | Olfr691 | | |
| 15751 | | | | | | Olfr693 | | |
| 15752 | | | | | | Olfr694 | | |
| 15753 | | | | | | Olfr695 | | |
| 15754 | | | | | | Olfr697 | | |
| 15755 | | | | | | Olfr698 | | |
| 15757 | | | | | | Olfr70 | | |
| 15758 | | | | | | Olfr700 | | |
| 15759 | | | | | | Olfr701 | | |
| 15760 | | | | | | Olfr702 | | |
| 15761 | | | | | | Olfr703 | | |
| 15763 | | | | | | Olfr705 | | |
| 15764 | | | | | | Olfr706 | | |
| 15765 | | | | | | Olfr707 | | |
| 15766 | | | | | | Olfr71 | | |
| 15767 | | | | | | Olfr710 | | |
| 15768 | | | | | | Olfr711 | | |
| 15769 | | | | | | Olfr713 | | |
| 15770 | | | | | | Olfr714 | | |
| 15771 | | | | | | Olfr715 | | |
| 15772 | | | | | | Olfr716 | | |
| 15773 | | | | | | Olfr720 | | |
| 15774 | | | | | | Olfr722 | | |
| 15775 | | | | | | Olfr723 | | |
| 15776 | | | | | | Olfr724 | | |
| 15777 | | | | | | Olfr725 | | |
| 15778 | | | | | | Olfr726 | | |
| 15779 | | | | | | Olfr727 | | |
| 15780 | | | | | | Olfr728 | | |
| 15781 | | | | | | Olfr729 | | |
| 15782 | | | | | | Olfr73 | | |
| 15783 | | | | | | Olfr730 | | |
| 15784 | | | | | | Olfr731 | | |
| 15785 | | | | | | Olfr732 | | |
| 15786 | | | | | | Olfr733 | | |
| 15788 | | | | | | Olfr735 | | |
| 15790 | | | | | | Olfr738 | | |
| 15791 | | | | | | Olfr739 | | |
| 15792 | | | | | | Olfr74 | | |
| 15793 | | | | | | Olfr740 | | |
| 15794 | | | | | | Olfr741 | | |
| 15795 | | | | | | Olfr742 | | |
| 15796 | | | | | | Olfr743 | | |
| 15797 | | | | | | Olfr744 | | |
| 15798 | | | | | | Olfr745 | | |
| 15799 | | | | | | Olfr746 | | |
| 15800 | | | | | | Olfr747 | | |
| 15801 | | | | | | Olfr748 | | |
| 15802 | | | | | | Olfr749 | | |
| 15803 | | | | | | Olfr750 | | |
| 15804 | | | | | | Olfr75-ps1 | | |
| 15805 | | | | | | Olfr76 | | |
| 15806 | | | | | | Olfr761 | | |
| 15807 | | | | | | Olfr763 | | |
| 15808 | | | | | | Olfr765 | | |
| 15809 | | | | | | Olfr767 | | |
| 15810 | | | | | | Olfr768 | | |
| 15811 | | | | | | Olfr769 | | |
| 15812 | | | | | | Olfr77 | | |

Fig.22 - 123

| | | | | | | | | |
|---|---|---|---|---|---|---|---|---|
| 15813 | | | | | | Olfr770 | | |
| 15814 | | | | | | Olfr771 | | |
| 15815 | | | | | | Olfr772 | | |
| 15816 | | | | | | Olfr773 | | |
| 15817 | | | | | | Olfr774 | | |
| 15818 | | | | | | Olfr775 | | |
| 15819 | | | | | | Olfr776 | | |
| 15820 | | | | | | Olfr777 | | |
| 15821 | | | | | | Olfr78 | | |
| 15822 | | | | | | Olfr780 | | |
| 15825 | | | | | | Olfr784 | | |
| 15826 | | | | | | Olfr786 | | |
| 15827 | | | | | | Olfr787 | | |
| 15828 | | | | | | Olfr788 | | |
| 15829 | | | | | | Olfr790 | | |
| 15830 | | | | | | Olfr791 | | |
| 15831 | | | | | | Olfr792 | | |
| 15832 | | | | | | Olfr794 | | |
| 15833 | | | | | | Olfr796 | | |
| 15834 | | | | | | Olfr798 | | |
| 15835 | | | | | | Olfr799 | | |
| 15836 | | | | | | Olfr8 | | |
| 15837 | | | | | | Olfr800 | | |
| 15838 | | | | | | Olfr801 | | |
| 15839 | | | | | | Olfr802 | | |
| 15840 | | | | | | Olfr803 | | |
| 15841 | | | | | | Olfr804 | | |
| 15842 | | | | | | Olfr805 | | |
| 15843 | | | | | | Olfr806 | | |
| 15844 | | | | | | Olfr807 | | |
| 15845 | | | | | | Olfr808 | | |
| 15846 | | | | | | Olfr809 | | |
| 15847 | | | | | | Olfr810 | | |
| 15848 | | | | | | Olfr811 | | |
| 15849 | | | | | | Olfr812 | | |
| 15850 | | | | | | Olfr813 | | |
| 15851 | | | | | | Olfr814 | | |
| 15852 | | | | | | Olfr815 | | |
| 15853 | | | | | | Olfr816 | | |
| 15854 | | | | | | Olfr818 | | |
| 15855 | | | | | | Olfr819 | | |
| 15856 | | | | | | Olfr820 | | |
| 15857 | | | | | | Olfr821 | | |
| 15858 | | | | | | Olfr822 | | |
| 15859 | | | | | | Olfr823 | | |
| 15860 | | | | | | Olfr824 | | |
| 15861 | | | | | | Olfr825 | | |
| 15862 | | | | | | Olfr826 | | |
| 15863 | | | | | | Olfr827 | | |
| 15864 | | | | | | Olfr828 | | |
| 15865 | | | | | | Olfr829 | | |
| 15866 | | | | | | Olfr830 | | |
| 15867 | | | | | | Olfr832 | | |
| 15868 | | | | | | Olfr834 | | |
| 15869 | | | | | | Olfr835 | | |
| 15871 | | | | | | Olfr837 | | |
| 15872 | | | | | | Olfr843 | | |
| 15873 | | | | | | Olfr845 | | |
| 15874 | | | | | | Olfr846 | | |
| 15876 | | | | | | Olfr849 | | |
| 15877 | | | | | | Olfr850 | | |
| 15878 | | | | | | Olfr851 | | |
| 15879 | | | | | | Olfr853 | | |
| 15880 | | | | | | Olfr854 | | |
| 15881 | | | | | | Olfr855 | | |
| 15882 | | | | | | Olfr856-ps1 | | |
| 15883 | | | | | | Olfr857 | | |
| 15884 | | | | | | Olfr859 | | |
| 15885 | | | | | | Olfr860 | | |
| 15886 | | | | | | Olfr862 | | |
| 15887 | | | | | | Olfr866 | | |
| 15888 | | | | | | Olfr867 | | |
| 15889 | | | | | | Olfr868 | | |
| 15890 | | | | | | Olfr869 | | |
| 15891 | | | | | | Olfr870 | | |
| 15892 | | | | | | Olfr871 | | |
| 15893 | | | | | | Olfr872 | | |
| 15894 | | | | | | Olfr873 | | |
| 15895 | | | | | | Olfr874 | | |
| 15896 | | | | | | Olfr875 | | |
| 15897 | | | | | | Olfr876 | | |
| 15898 | | | | | | Olfr877 | | |
| 15899 | | | | | | Olfr878 | | |
| 15900 | | | | | | Olfr881 | | |
| 15902 | | | | | | Olfr884 | | |
| 15903 | | | | | | Olfr885 | | |
| 15904 | | | | | | Olfr887 | | |
| 15905 | | | | | | Olfr888 | | |
| 15906 | | | | | | Olfr889 | | |
| 15907 | | | | | | Olfr890 | | |
| 15908 | | | | | | Olfr891 | | |
| 15909 | | | | | | Olfr893 | | |
| 15910 | | | | | | Olfr894 | | |
| 15911 | | | | | | Olfr895 | | |
| 15912 | | | | | | Olfr898 | | |
| 15913 | | | | | | Olfr899 | | |
| 15914 | | | | | | Olfr9 | | |
| 15915 | | | | | | Olfr90 | | |
| 15916 | | | | | | Olfr900 | | |
| 15917 | | | | | | Olfr901 | | |
| 15918 | | | | | | Olfr902 | | |
| 15919 | | | | | | Olfr904 | | |
| 15921 | | | | | | Olfr906 | | |
| 15923 | | | | | | Olfr908 | | |
| 15924 | | | | | | Olfr91 | | |
| 15925 | | | | | | Olfr910 | | |
| 15926 | | | | | | Olfr911-ps1 | | |
| 15927 | | | | | | Olfr912 | | |
| 15928 | | | | | | Olfr913 | | |
| 15929 | | | | | | Olfr914 | | |
| 15930 | | | | | | Olfr915 | | |
| 15931 | | | | | | Olfr916 | | |
| 15932 | | | | | | Olfr917 | | |
| 15933 | | | | | | Olfr918 | | |
| 15934 | | | | | | Olfr919 | | |
| 15935 | | | | | | Olfr92 | | |
| 15938 | | | | | | Olfr922 | | |
| 15940 | | | | | | Olfr924 | | |
| 15941 | | | | | | Olfr926 | | |
| 15942 | | | | | | Olfr93 | | |
| 15943 | | | | | | Olfr930 | | |
| 15944 | | | | | | Olfr933 | | |
| 15945 | | | | | | Olfr934 | | |
| 15946 | | | | | | Olfr935 | | |
| 15947 | | | | | | Olfr936 | | |
| 15948 | | | | | | Olfr937 | | |
| 15949 | | | | | | Olfr938 | | |
| 15950 | | | | | | Olfr94 | | |
| 15951 | | | | | | Olfr943 | | |
| 15952 | | | | | | Olfr944 | | |
| 15953 | | | | | | Olfr945 | | |
| 15954 | | | | | | Olfr947-ps1 | | |
| 15955 | | | | | | Olfr948 | | |
| 15956 | | | | | | Olfr95 | | |
| 15957 | | | | | | Olfr951 | | |
| 15958 | | | | | | Olfr952 | | |
| 15959 | | | | | | Olfr954 | | |
| 15960 | | | | | | Olfr955 | | |
| 15961 | | | | | | Olfr957 | | |
| 15962 | | | | | | Olfr958 | | |
| 15963 | | | | | | Olfr959 | | |
| 15964 | | | | | | Olfr96 | | |
| 15965 | | | | | | Olfr960 | | |
| 15966 | | | | | | Olfr961 | | |
| 15967 | | | | | | Olfr963 | | |
| 15968 | | | | | | Olfr965 | | |
| 15969 | | | | | | Olfr967 | | |
| 15970 | | | | | | Olfr968 | | |
| 15971 | | | | | | Olfr969 | | |
| 15972 | | | | | | Olfr97 | | |
| 15973 | | | | | | Olfr970 | | |
| 15974 | | | | | | Olfr971 | | |
| 15975 | | | | | | Olfr972 | | |
| 15976 | | | | | | Olfr974 | | |
| 15977 | | | | | | Olfr975 | | |
| 15978 | | | | | | Olfr976 | | |
| 15981 | | | | | | Olfr98 | | |
| 15984 | | | | | | Olfr982 | | |
| 15985 | | | | | | Olfr983 | | |
| 15986 | | | | | | Olfr984 | | |
| 15987 | | | | | | Olfr985 | | |
| 15988 | | | | | | Olfr986 | | |
| 15989 | | | | | | Olfr987 | | |
| 15990 | | | | | | Olfr988 | | |
| 15991 | | | | | | Olfr99 | | |
| 15993 | | | | | | Olfr993 | | |
| 15995 | | | | | | Olfr995 | | |
| 15996 | | | | | | Olfr996 | | |
| 15997 | | | | | | Olfr998 | | |
| 16015 | | | | | | Oog4 | | |
| 16016 | | | | | | Oosp1 | 255649 | 4-May-15 |
| 16017 | | | | | | Oosp2 | 219990 | 4-May-15 |
| 16019 | | | | | | Opa1 | 4976 | 7-Jun-15 |
| 16020 | | | | | | Opa3 | 80207 | 23-May-15 |
| 16032 | | | | | | Oprl1 | 4987 | 4-May-15 |
| 16085 | | | | | | Otogl | 283310 | 7-Jun-15 |
| 16087 | | | | | | Otop1 | 133060 | 4-May-15 |
| 16096 | | | | | | Otub2 | 78990 | 4-May-15 |
| 17384 | | | | | | Prl8a6 | | |
| 17385 | | | | | | Prl8a8 | | |
| 17386 | | | | | | Prl8a9 | | |
| 20036 | | | | | | Snord37 | 26812 | 4-May-15 |
| 20072 | | | | | | Snord90 | 692206 | 4-May-15 |
| 20157 | | | | | | Son | 6651 | 12-May-15 |
| 20738 | | | | | | Svs5 | | |
| 20932 | | | | | | Tas2r135 | | |
| 20933 | | | | | | Tas2r136 | | |
| 20934 | | | | | | Tas2r137 | | |
| 20935 | | | | | | Tas2r138 | | |
| 20936 | | | | | | Tas2r139 | | |
| 20937 | | | | | | Tas2r140 | | |
| 20938 | | | | | | Tas2r143 | | |
| 20939 | | | | | | Tas2r144 | | |
| 20940 | | | | | | Tasp1 | 55617 | 4-May-15 |

Fig.22 - 124

| | | | | | | | | |
|---|---|---|---|---|---|---|---|---|
| 22697 | | | | | | Vmn1r128 | | |
| 22712 | | | | | | Vmn1r157 | | |
| 22719 | | | | | | Vmn1r166 | | |
| 22720 | | | | | | Vmn1r167 | | |
| 22721 | | | | | | Vmn1r168 | | |
| 22786 | | | | | | Vmn1r228 | | |
| 22787 | | | | | | Vmn1r229 | | |
| 22788 | | | | | | Vmn1r23 | | |
| 22789 | | | | | | Vmn1r230 | | |
| 22790 | | | | | | Vmn1r231 | | |
| 22791 | | | | | | Vmn1r232 | | |
| 22792 | | | | | | Vmn1r233 | | |
| 22793 | | | | | | Vmn1r234 | | |
| 22794 | | | | | | Vmn1r235 | | |
| 22795 | | | | | | Vmn1r236 | | |
| 22796 | | | | | | Vmn1r237 | | |
| 22797 | | | | | | Vmn1r238 | | |
| 22798 | | | | | | Vmn1r24 | | |
| 22799 | | | | | | Vmn1r25 | | |
| 22800 | | | | | | Vmn1r26 | | |
| 22801 | | | | | | Vmn1r27 | | |
| 22802 | | | | | | Vmn1r28 | | |
| 22803 | | | | | | Vmn1r29 | | |
| 22804 | | | | | | Vmn1r3 | | |
| 22805 | | | | | | Vmn1r30 | | |
| 22806 | | | | | | Vmn1r31 | | |
| 22807 | | | | | | Vmn1r32 | | |
| 22808 | | | | | | Vmn1r33 | | |
| 22809 | | | | | | Vmn1r34 | | |
| 22810 | | | | | | Vmn1r35 | | |
| 22811 | | | | | | Vmn1r36 | | |
| 22812 | | | | | | Vmn1r37 | | |
| 22813 | | | | | | Vmn1r38 | | |
| 22814 | | | | | | Vmn1r39 | | |
| 22815 | | | | | | Vmn1r4 | | |
| 22816 | | | | | | Vmn1r40 | | |
| 22817 | | | | | | Vmn1r41 | | |
| 22818 | | | | | | Vmn1r42 | | |
| 22819 | | | | | | Vmn1r43 | | |
| 22820 | | | | | | Vmn1r44 | | |
| 22821 | | | | | | Vmn1r45 | | |
| 22822 | | | | | | Vmn1r46 | | |
| 22823 | | | | | | Vmn1r47 | | |
| 22824 | | | | | | Vmn1r48 | | |
| 22825 | | | | | | Vmn1r49 | | |
| 22826 | | | | | | Vmn1r5 | | |
| 22827 | | | | | | Vmn1r50 | | |
| 22828 | | | | | | Vmn1r51 | | |
| 22829 | | | | | | Vmn1r52 | | |
| 22830 | | | | | | Vmn1r53 | | |
| 22831 | | | | | | Vmn1r54 | | |
| 22832 | | | | | | Vmn1r55 | | |
| 22833 | | | | | | Vmn1r56 | | |
| 22834 | | | | | | Vmn1r57 | | |
| 22835 | | | | | | Vmn1r58 | | |
| 22836 | | | | | | Vmn1r59 | | |
| 22837 | | | | | | Vmn1r6 | | |
| 22838 | | | | | | Vmn1r60 | | |
| 22839 | | | | | | Vmn1r61 | | |
| 22840 | | | | | | Vmn1r62 | | |
| 22841 | | | | | | Vmn1r63 | | |
| 22842 | | | | | | Vmn1r64 | | |
| 22843 | | | | | | Vmn1r65 | | |
| 22844 | | | | | | Vmn1r66 | | |
| 22845 | | | | | | Vmn1r67 | | |
| 22846 | | | | | | Vmn1r68 | | |
| 22847 | | | | | | Vmn1r69 | | |
| 22848 | | | | | | Vmn1r7 | | |
| 22849 | | | | | | Vmn1r70 | | |
| 22850 | | | | | | Vmn1r71 | | |
| 22851 | | | | | | Vmn1r72 | | |
| 22852 | | | | | | Vmn1r73 | | |
| 22853 | | | | | | Vmn1r74 | | |
| 22854 | | | | | | Vmn1r75 | | |
| 22855 | | | | | | Vmn1r76 | | |
| 22856 | | | | | | Vmn1r77 | | |
| 22857 | | | | | | Vmn1r78 | | |
| 22858 | | | | | | Vmn1r79 | | |
| 22859 | | | | | | Vmn1r8 | | |
| 22861 | | | | | | Vmn1r81 | | |
| 22862 | | | | | | Vmn1r82 | | |
| 22863 | | | | | | Vmn1r83 | | |
| 22864 | | | | | | Vmn1r84 | | |
| 22865 | | | | | | Vmn1r85 | | |
| 22866 | | | | | | Vmn1r86 | | |
| 22867 | | | | | | Vmn1r87 | | |
| 22868 | | | | | | Vmn1r88 | | |
| 22869 | | | | | | Vmn1r89 | | |
| 22870 | | | | | | Vmn1r9 | | |
| 22871 | | | | | | Vmn1r90 | | |
| 22872 | | | | | | Vmn1r91 | | |
| 22873 | | | | | | Vmn1r94 | | |
| 22874 | | | | | | Vmn1r95 | | |
| 22877 | | | | | | Vmn2r1 | | |
| 22879 | | | | | | Vmn2r100 | | |
| 22880 | | | | | | Vmn2r101 | | |
| 22881 | | | | | | Vmn2r102 | | |
| 22882 | | | | | | Vmn2r103 | | |
| 22883 | | | | | | Vmn2r104 | | |
| 22884 | | | | | | Vmn2r105 | | |
| 22885 | | | | | | Vmn2r106 | | |
| 22886 | | | | | | Vmn2r107 | | |
| 22887 | | | | | | Vmn2r108 | | |
| 22888 | | | | | | Vmn2r109 | | |
| 22889 | | | | | | Vmn2r11 | | |
| 22890 | | | | | | Vmn2r110 | | |
| 22891 | | | | | | Vmn2r111 | | |
| 22892 | | | | | | Vmn2r112 | | |
| 22893 | | | | | | Vmn2r113 | | |
| 22894 | | | | | | Vmn2r114 | | |
| 22895 | | | | | | Vmn2r115 | | |
| 22896 | | | | | | Vmn2r116 | | |
| 22897 | | | | | | Vmn2r117 | | |
| 22898 | | | | | | Vmn2r118 | | |
| 22899 | | | | | | Vmn2r12 | | |
| 22900 | | | | | | Vmn2r120 | | |
| 22901 | | | | | | Vmn2r121 | | |
| 22902 | | | | | | Vmn2r122 | | |
| 22903 | | | | | | Vmn2r123 | | |
| 22904 | | | | | | Vmn2r124 | | |
| 22905 | | | | | | Vmn2r13 | | |
| 22906 | | | | | | Vmn2r14 | | |
| 22907 | | | | | | Vmn2r15 | | |
| 22908 | | | | | | Vmn2r16 | | |
| 22909 | | | | | | Vmn2r17 | | |
| 22910 | | | | | | Vmn2r18 | | |
| 22911 | | | | | | Vmn2r19 | | |
| 22912 | | | | | | Vmn2r2 | | |
| 22913 | | | | | | Vmn2r20 | | |
| 22914 | | | | | | Vmn2r21 | | |
| 22915 | | | | | | Vmn2r22 | | |
| 22916 | | | | | | Vmn2r23 | | |
| 22917 | | | | | | Vmn2r24 | | |
| 22918 | | | | | | Vmn2r25 | | |
| 22919 | | | | | | Vmn2r26 | | |
| 22920 | | | | | | Vmn2r27 | | |
| 22921 | | | | | | Vmn2r28 | | |
| 22922 | | | | | | Vmn2r29 | | |
| 22926 | | | | | | Vmn2r32 | | |
| 22927 | | | | | | Vmn2r33 | | |
| 22928 | | | | | | Vmn2r34 | | |
| 22930 | | | | | | Vmn2r36 | | |
| 22931 | | | | | | Vmn2r37 | | |
| 22932 | | | | | | Vmn2r38 | | |
| 22933 | | | | | | Vmn2r39 | | |
| 22934 | | | | | | Vmn2r4 | | |
| 22935 | | | | | | Vmn2r40 | | |
| 22936 | | | | | | Vmn2r41 | | |
| 22938 | | | | | | Vmn2r43 | | |
| 22939 | | | | | | Vmn2r44 | | |
| 22940 | | | | | | Vmn2r45 | | |
| 22941 | | | | | | Vmn2r46 | | |
| 22942 | | | | | | Vmn2r47 | | |
| 22943 | | | | | | Vmn2r48 | | |
| 22944 | | | | | | Vmn2r49 | | |
| 22945 | | | | | | Vmn2r5 | | |
| 22946 | | | | | | Vmn2r50 | | |
| 22947 | | | | | | Vmn2r51 | | |
| 22948 | | | | | | Vmn2r52 | | |
| 22949 | | | | | | Vmn2r53 | | |
| 22950 | | | | | | Vmn2r54 | | |
| 22951 | | | | | | Vmn2r55 | | |
| 22952 | | | | | | Vmn2r56 | | |
| 22953 | | | | | | Vmn2r57 | | |
| 22954 | | | | | | Vmn2r58 | | |
| 22955 | | | | | | Vmn2r59 | | |
| 22956 | | | | | | Vmn2r6 | | |
| 22957 | | | | | | Vmn2r60 | | |
| 22958 | | | | | | Vmn2r61 | | |
| 22959 | | | | | | Vmn2r62 | | |
| 22960 | | | | | | Vmn2r63 | | |
| 22961 | | | | | | Vmn2r65 | | |
| 22962 | | | | | | Vmn2r66 | | |
| 22963 | | | | | | Vmn2r67 | | |
| 22964 | | | | | | Vmn2r68 | | |
| 22965 | | | | | | Vmn2r69 | | |
| 22966 | | | | | | Vmn2r7 | | |
| 22967 | | | | | | Vmn2r70 | | |
| 22968 | | | | | | Vmn2r71 | | |
| 22970 | | | | | | Vmn2r73 | | |
| 22971 | | | | | | Vmn2r74 | | |
| 22972 | | | | | | Vmn2r75 | | |
| 22973 | | | | | | Vmn2r76 | | |
| 22974 | | | | | | Vmn2r77 | | |
| 22975 | | | | | | Vmn2r78 | | |
| 22976 | | | | | | Vmn2r79 | | |
| 22977 | | | | | | Vmn2r8 | | |
| 22978 | | | | | | Vmn2r80 | | |
| 22979 | | | | | | Vmn2r81 | | |
| 22980 | | | | | | Vmn2r82 | | |
| 22981 | | | | | | Vmn2r83 | | |
| 22982 | | | | | | Vmn2r84 | | |
| 22983 | | | | | | Vmn2r85 | | |
| 22984 | | | | | | Vmn2r86 | | |

Fig.22 - 125

| | | | | | | | | |
|---|---|---|---|---|---|---|---|---|
| 22986 | | | | | | Vmn2r88 | | |
| 22987 | | | | | | Vmn2r89 | | |
| 22988 | | | | | | Vmn2r9 | | |
| 22990 | | | | | | Vmn2r91 | | |
| 22992 | | | | | | Vmn2r93 | | |
| 22993 | | | | | | Vmn2r94 | | |
| 22994 | | | | | | Vmn2r95 | | |
| 22995 | | | | | | Vmn2r96 | | |
| 22998 | | | | | | Vmn2r99 | | |
| 23001 | | | | | | Vmn2r-ps159 | | |
| 23003 | | | | | | Vmn2r-ps60 | | |
| 23293 | | | | | | Xpnpep1 | 7511 | 12-May-15 |

[Fig. 23-1]

| | AG | | Aorta | | Brain | | Lung | | Pancreas | | PO/ | | Skin | | Skull | | SM | | Spleen | | Testis | | TG | |
|---|---|---|---|---|---|---|---|---|---|---|---|---|---|---|---|---|---|---|---|---|---|---|---|---|
| | E | M | E | M | E | M | E | M | E | M | E | M | E | M | E | M | E | M | E | M | E | M | E | M |
| Abcb1b | 0,9 | 1,3 | 1,1 | 1,4 | 1,0 | 1,0 | 0,9 | 0,8 | 1,0 | 1,0 | 1,0 | 1,2 | 1,2 | 1,2 | 1,3 | 0,8 | 2,0 | 2,7 | 1,0 | 1,2 | 1,0 | 1,0 | 1,0 | 1,0 |
| Abcg4 | 1,0 | 1,3 | 1,0 | 0,9 | 1,0 | 0,9 | 1,0 | 1,0 | 1,0 | 1,0 | 1,2 | 1,1 | 1,3 | 1,1 | 1,5 | 1,2 | 1,0 | 1,0 | 10,2 | 0,4 | 1,0 | 1,0 | 1,0 | 1,0 |
| Abra | 1,0 | 1,0 | 1,4 | 0,9 | 1,0 | 1,0 | 1,2 | 0,9 | 1,0 | 1,0 | 1,0 | 1,0 | 2,3 | 0,8 | 2,6 | 0,2 | 0,9 | 1,6 | 1,0 | 1,0 | 1,0 | 1,0 | 1,2 | 6,0 |
| Acbd7 | 1,0 | 1,0 | 1,0 | 1,0 | 1,0 | 1,0 | 1,0 | 1,0 | 1,0 | 1,0 | 2,0 | 0,7 | 1,0 | 1,0 | 1,0 | 1,0 | 1,0 | 1,2 | 1,0 | 1,0 | 1,0 | 1,0 | 1,0 | 1,0 |
| Acnat2 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Acot3 | 1,0 | 1,0 | 1,0 | 1,0 | 0,7 | 1,1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Acsm3 | 4,4 | 2,9 | 2,0 | 2,1 | 1,0 | 1,0 | 1,0 | 1,0 | 1,0 | 1,0 | 1,0 | 1,0 | 1,3 | 0,9 | 1,0 | 1,0 | 1,7 | 1,4 | 1,0 | 1,0 | 1,0 | 1,0 | 2,8 | 2,3 |
| Acta1 | 6,2 | 1,0 | 0,6 | 0,3 | 1,1 | 1,4 | 0,7 | 1,4 | 1,0 | 1,0 | 1,0 | 1,0 | 1,7 | 0,8 | 4,2 | 0,3 | 1,0 | 1,1 | 0,8 | 1,0 | 0,9 | 1,2 | 1,1 | 21,8 |
| Actc1 | 2,1 | 1,0 | 0,9 | 0,8 | 1,0 | 1,0 | 0,7 | 0,8 | 1,0 | 1,0 | 1,0 | 1,0 | 0,9 | 0,2 | 0,9 | 0,3 | 0,5 | 0,6 | 1,0 | 1,0 | 0,8 | 1,0 | 85,4 | 0,6 |
| Actg2 | 1,0 | 1,0 | 1,2 | 1,2 | 1,0 | 1,0 | 0,8 | 1,0 | 1,4 | 1,5 | 1,0 | 1,0 | 1,4 | 1,1 | 0,9 | 0,9 | 1,0 | 1,0 | 1,0 | 1,4 | 1,1 | 1,0 | 1,0 | 1,2 |
| Actn2 | 1,0 | 1,0 | 1,2 | 0,9 | 0,8 | 1,0 | 0,7 | 0,8 | 1,0 | 1,0 | 0,9 | 0,8 | 1,9 | 0,8 | 8,3 | 0,3 | 0,8 | 1,3 | 1,0 | 1,0 | 1,0 | 1,0 | 1,7 | 7,9 |
| Actn3 | 1,0 | 1,0 | 1,0 | 1,0 | 1,0 | 1,0 | 1,0 | 1,0 | 1,0 | 1,0 | 1,0 | 1,0 | 4,6 | 1,2 | 7,9 | 0,3 | 1,7 | 1,6 | 1,0 | 1,0 | 1,2 | 0,8 | 1,2 | 34,5 |
| Adam28 | 1,0 | 1,0 | 1,0 | 1,0 | 1,0 | 1,0 | 0,9 | 1,0 | 1,0 | 1,0 | 1,2 | 1,0 | 1,0 | 1,0 | 1,0 | 1,0 | 1,0 | 1,0 | 1,0 | 1,0 | 1,0 | 1,0 | 0,8 | 1,0 |
| Adam7 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,3 | 1,0 | 1,0 |
| Add2 | 1,0 | 1,8 | 1,0 | 1,0 | 1,0 | 0,9 | 1,0 | 1,0 | 1,0 | 1,0 | 1,0 | 1,0 | 1,0 | 0,8 | 1,7 | 1,4 | 1,0 | 1,0 | 9,6 | 0,3 | 1,1 | 0,9 | 1,0 | 1,0 |
| Adh1 | 4,4 | 9,5 | 1,3 | 1,3 | 1,1 | 1,0 | 1,0 | 1,0 | 1,2 | 1,0 | 1,7 | 1,3 | 1,5 | 1,9 | 1,6 | 1,3 | 1,1 | 1,1 | 1,1 | 1,8 | 1,3 | 0,9 | 1,2 | 1,5 |
| Adh6-ps1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Adh7 | 3,0 | 5,3 | 0,7 | 1,3 | 1,0 | 1,0 | 0,9 | 1,1 | 1,0 | 1,0 | 1,0 | 1,0 | 1,1 | 1,4 | 0,6 | 1,3 | 1,0 | 1,0 | 1,0 | 1,0 | 1,0 | 1,1 | 1,0 | 1,0 |
| Adig | 1,7 | 1,5 | 0,8 | 1,2 | 1,0 | 1,0 | 1,5 | 0,4 | 1,0 | 1,0 | 1,0 | 1,0 | 1,1 | 2,3 | 1,0 | 1,0 | 1,2 | 0,7 | 1,0 | 1,0 | 1,0 | 0,9 | 1,4 | 1,4 |
| Adipoq | 1,9 | 1,8 | 1,2 | 1,6 | 1,0 | 1,0 | 0,9 | 0,2 | 0,3 | 0,3 | 1,0 | 1,0 | 1,0 | 1,6 | 1,4 | 2,9 | 0,9 | 0,7 | 3,4 | 1,4 | 1,0 | 0,9 | 1,6 | 1,4 |
| Agps | 1,0 | 0,9 | 1,0 | 1,3 | 1,0 | 0,9 | 1,0 | 1,1 | 0,8 | 1,0 | 0,9 | 1,1 | 0,9 | 0,9 | 0,9 | 0,9 | 1,0 | 1,0 | 1,0 | 1,0 | 0,9 | 1,1 | 1,0 | 1,0 |
| Agr2 | 1,0 | 1,0 | 1,0 | 1,0 | 1,0 | 1,0 | 1,0 | 1,4 | 1,6 | 2,0 | 1,0 | 1,0 | 1,0 | 1,0 | 1,0 | 1,0 | 1,0 | 1,0 | 1,0 | 1,0 | 1,0 | 1,0 | 0,5 | 0,4 |
| Ahsg | 1,4 | 1,0 | 1,6 | 0,2 | 1,9 | 0,9 | 1,6 | 1,0 | 1,7 | 1,1 | 0,9 | 0,8 | 1,0 | 1,0 | 2,5 | 1,1 | 1,0 | 1,0 | 1,0 | 1,0 | 1,0 | 1,0 | 1,0 | 1,2 |
| Aicda | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,8 | 1,0 | 1,0 | 0,5 | 0,2 | 1,0 | 1,0 | 1,0 | 1,0 |
| Airn | 1,1 | 0,7 | 1,4 | 0,9 | 0,6 | 0,8 | 1,1 | 0,9 | 1,0 | 1,0 | 0,9 | 1,0 | 0,9 | 0,7 | 1,0 | 0,9 | 1,0 | 0,5 | 1,4 | 0,9 | 1,0 | 1,0 | 0,8 | 1,1 |
| Akp3 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Akr1b3 | 0,5 | 1,1 | 1,6 | 0,6 | 1,0 | 0,7 | 1,2 | 0,5 | 1,0 | 1,0 | 1,0 | 0,9 | 0,5 | 0,2 | 0,3 | 1,2 | 2,2 | 1,1 | 1,3 | 2,1 | 0,3 | 0,4 | 2,4 | 2,0 |
| Akr1b7 | 1,4 | 1,6 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,8 | 0,9 | 1,0 | 1,0 | 1,0 | 1,0 |
| Akr1b8 | 1,7 | 1,6 | 0,8 | 0,9 | 1,0 | 1,0 | 1,1 | 1,1 | 1,0 | 1,0 | 0,8 | 0,9 | 0,8 | 1,4 | 0,9 | 2,0 | 1,0 | 0,7 | 1,6 | 1,0 | 1,1 | 1,2 | 1,0 | 1,1 |
| Akr1c14 | 1,0 | 0,7 | 0,7 | 0,6 | 1,0 | 1,0 | 2,0 | 1,8 | 1,6 | 1,0 | 0,2 | 0,1 | 0,7 | 0,9 | 1,2 | 1,0 | 0,8 | 0,6 | 0,8 | 1,2 | 1,0 | 1,0 | 0,9 | 0,7 |
| Akr1c18 | 1,0 | 1,0 | 1,0 | 1,0 | 1,0 | 1,0 | 1,0 | 1,0 | 1,0 | 1,0 | 1,0 | 1,0 | 0,9 | 0,9 | 1,0 | 1,0 | 1,0 | 1,0 | 1,0 | 1,0 | 1,0 | 1,0 | 1,0 | 1,0 |
| Alas2 | 2,3 | 2,0 | 2,5 | 1,6 | 1,3 | 1,3 | 2,5 | 1,6 | 1,0 | 1,4 | 1,2 | 0,7 | 1,3 | 1,2 | 1,6 | 2,3 | 1,7 | 2,4 | 1,9 | 0,7 | 0,9 | 1,1 | 3,8 | 2,5 |
| Alb | 1,9 | 0,6 | 1,0 | 0,2 | 1,0 | 0,7 | 1,6 | 1,0 | 1,1 | 1,2 | 0,8 | 0,9 | 1,0 | 1,5 | 5,6 | 1,0 | 1,0 | 1,0 | 0,8 | 1,6 | 1,0 | 1,0 | 1,2 | 1,0 |
| Aldh1a1 | 0,5 | 0,1 | 1,3 | 1,1 | 1,0 | 1,0 | 1,1 | 1,0 | 1,2 | 0,9 | 0,9 | 1,5 | 1,4 | 1,0 | 2,0 | 1,5 | 1,5 | 1,4 | 2,3 | 0,7 | 0,7 | 0,9 | 0,6 | 4,3 |
| Aldh1a3 | 1,0 | 0,9 | 1,2 | 1,3 | 1,0 | 1,0 | 1,0 | 1,0 | 1,0 | 1,0 | 1,0 | 1,0 | 0,6 | 0,9 | 0,4 | 0,6 | 1,0 | 1,2 | 0,9 | 0,7 | 1,0 | 1,1 | 33,7 | 3,8 |
| Aldh3a1 | 1,0 | 1,0 | 1,3 | 1,0 | 1,0 | 1,0 | 1,1 | 1,2 | 1,0 | 1,0 | 0,7 | 0,7 | 1,7 | 1,5 | 1,2 | 1,0 | 1,0 | 1,0 | 1,0 | 1,0 | 0,8 | 0,5 | 16,3 | 1,0 |
| Aldob | 2,1 | 0,8 | 1,0 | 0,2 | 1,3 | 1,1 | 1,3 | 1,0 | 1,9 | 1,9 | 0,7 | 1,0 | 1,0 | 1,2 | 1,6 | 1,6 | 1,0 | 1,0 | 1,0 | 1,7 | 1,0 | 2,2 | 1,1 | 1,0 |
| Aldoc | 1,1 | 1,3 | 1,7 | 1,8 | 1,1 | 1,1 | 1,4 | 1,1 | 1,0 | 1,0 | 1,3 | 1,1 | 1,0 | 0,8 | 1,6 | 1,1 | 1,0 | 1,0 | 0,8 | 1,0 | 0,6 | 0,7 | 0,8 | 0,9 |
| Alpk3 | 1,0 | 1,0 | 1,0 | 0,9 | 1,0 | 1,0 | 0,9 | 1,0 | 1,0 | 1,0 | 1,0 | 1,0 | 2,3 | 0,9 | 3,1 | 0,3 | 1,0 | 1,2 | 1,0 | 1,0 | 1,0 | 1,0 | 1,6 | 7,3 |
| Alpl | 0,5 | 0,5 | 0,5 | 0,6 | 0,9 | 0,9 | 0,9 | 1,0 | 1,0 | 1,0 | 0,7 | 0,9 | 0,9 | 1,2 | 0,9 | 1,1 | 0,6 | 0,8 | 1,0 | 1,3 | 1,0 | 0,6 | 1,0 | 0,7 |
| Ambp | 1,0 | 0,9 | 1,0 | 0,4 | 1,0 | 1,0 | 1,0 | 1,0 | 1,0 | 1,6 | 1,0 | 1,0 | 1,0 | 1,0 | 1,0 | 1,0 | 1,0 | 1,0 | 1,0 | 1,0 | 1,0 | 1,0 | 1,0 | 1,0 |
| Amd1 | 1,0 | 0,1 | 1,0 | 1,0 | 1,0 | 1,0 | 1,0 | 6,2 | 1,0 | 1,0 | 1,0 | 1,0 | 1,0 | 0,1 | 1,0 | 5,5 | 0,0 | 0,3 | 1,0 | 1,0 | 1,0 | 1,0 | 1,0 | 1,0 |
| Ampd1 | 1,0 | 1,0 | 1,0 | 1,0 | 1,0 | 1,0 | 1,0 | 1,0 | 1,0 | 1,0 | 1,0 | 1,0 | 2,1 | 0,7 | 2,9 | 0,3 | 0,8 | 0,9 | 1,0 | 1,0 | 1,0 | 1,0 | 0,5 | 12,9 |
| Amy1 | 1,3 | 1,4 | 1,2 | 1,5 | 1,2 | 1,0 | 1,2 | 0,8 | 0,9 | 1,0 | 2,3 | 2,1 | 1,1 | 1,6 | 1,8 | 1,2 | 1,5 | 1,4 | 1,0 | 1,3 | 1,0 | 1,1 | 1,5 | 2,1 |
| Amy2a2 | 1,0 | 1,0 | 1,0 | 1,0 | 1,0 | 1,0 | 1,0 | 1,0 | 0,7 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Prol1 | 0,1 | 0,9 | 1,0 | 1,0 | 0,9 | 0,9 | 0,9 | 1,0 | 0,9 | 1,0 | 0,6 | 0,7 | 10,1 | 1,0 | 14,5 | 1,0 | 1,4 | 1,0 | 0,7 | 0,9 | 1,1 | 0,6 | 1,1 | 1,0 |
| Amy2b | 1,0 | 0,8 | 1,0 | 1,0 | 0,9 | 1,3 | 1,0 | 1,0 | 0,9 | 1,0 | 1,0 | 0,8 | 2,3 | 1,0 | 1,3 | 1,1 | 1,0 | 1,0 | 1,0 | 0,8 | 1,0 | 1,0 | 1,0 | 1,0 |
| Ang4 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,4 | 1,0 | 1,0 |
| Angptl7 | 1,0 | 1,0 | 1,4 | 1,3 | 1,0 | 1,0 | 0,6 | 0,7 | 1,0 | 1,0 | 1,0 | 1,0 | 1,5 | 0,9 | 0,7 | 0,4 | 0,6 | 1,3 | 0,9 | 1,0 | 1,5 | 1,0 | 1,9 | 2,4 |
| Ank1 | 0,8 | 0,7 | 1,0 | 0,9 | 1,0 | 0,9 | 1,0 | 1,0 | 1,0 | 1,0 | 0,9 | 1,0 | 1,8 | 0,8 | 1,8 | 1,3 | 0,8 | 1,0 | 9,0 | 0,3 | 1,0 | 1,0 | 1,0 | 4,6 |
| Ankrd1 | 1,0 | 1,0 | 1,1 | 1,1 | 1,0 | 1,0 | 1,0 | 0,7 | 1,0 | 1,0 | 1,0 | 1,0 | 2,3 | 0,7 | 2,0 | 0,8 | 0,8 | 2,7 | 1,0 | 1,0 | 1,0 | 1,0 | 6,4 | 1,2 |
| Ankrd2 | 1,0 | 1,0 | 1,0 | 1,0 | 1,0 | 1,0 | 1,0 | 1,0 | 1,0 | 1,0 | 1,0 | 1,0 | 1,6 | 0,7 | 4,5 | 1,6 | 0,6 | 1,3 | 1,6 | 2,0 | 1,0 | 1,0 | 1,4 | 5,1 |
| Ankrd23 | 1,1 | 1,0 | 1,1 | 0,7 | 0,7 | 1,2 | 1,1 | 0,9 | 0,8 | 1,1 | 1,1 | 1,4 | 2,8 | 0,9 | 2,9 | 0,2 | 1,0 | 1,1 | 1,0 | 1,3 | 1,4 | 0,9 | 1,2 | 3,6 |
| Ankrd66 | 1,0 | 1,0 | 1,0 | 1,0 | 1,0 | 1,0 | 1,3 | 0,7 | 1,0 | 1,0 | 1,0 | 1,0 | 1,0 | 1,0 | 0,8 | 1,3 | 1,0 | 1,0 | 1,0 | 1,2 | 1,0 | 1,0 | 6,7 | 14,3 |
| Anpep | 0,9 | 0,6 | 1,0 | 0,8 | 1,1 | 1,1 | 1,0 | 1,1 | 1,2 | 1,1 | 1,0 | 1,2 | 1,2 | 1,1 | 1,2 | 1,0 | 1,0 | 1,0 | 1,1 | 1,1 | 1,0 | 1,0 | 28,3 | 4,0 |
| Ap1m2 | 1,0 | 1,0 | 1,0 | 1,0 | 1,0 | 1,0 | 0,9 | 1,0 | 1,1 | 1,1 | 0,9 | 1,0 | 1,1 | 0,8 | 1,0 | 1,0 | 1,0 | 1,0 | 0,7 | 0,6 | 1,0 | 1,0 | 1,0 | 0,9 |
| Ap1s2 | 1,1 | 0,9 | 1,0 | 0,9 | 1,0 | 1,1 | 1,1 | 0,9 | 1,0 | 1,0 | 1,0 | 1,1 | 0,9 | 1,1 | 0,9 | 0,9 | 0,9 | 0,9 | 0,9 | 0,9 | 0,9 | 1,3 | 1,0 | 1,0 |
| Ap1s3 | 1,0 | 1,0 | 1,0 | 1,0 | 1,4 | 1,1 | 0,8 | 1,0 | 0,5 | 0,9 | 1,0 | 1,0 | 0,9 | 0,9 | 0,7 | 1,1 | 1,0 | 1,0 | 0,9 | 0,9 | 1,0 | 1,0 | 1,3 | 1,0 |
| Apln | 0,3 | 0,2 | 0,5 | 0,6 | 0,8 | 0,8 | 0,6 | 0,7 | 1,0 | 1,0 | 1,1 | 1,1 | 0,5 | 0,9 | 0,8 | 0,6 | 0,6 | 0,6 | 1,0 | 1,0 | 0,6 | 1,0 | 1,3 | 0,6 |
| Aplnr | 0,4 | 0,7 | 0,3 | 0,5 | 1,0 | 1,0 | 0,4 | 0,4 | 1,0 | 1,0 | 0,0 | 0,1 | 0,6 | 0,5 | 0,7 | 0,5 | 0,0 | 0,2 | 0,5 | 0,5 | 1,0 | 1,0 | 0,4 | 0,3 |
| Apoa1 | 2,8 | 1,1 | 1,0 | 0,1 | 1,3 | 0,7 | 1,6 | 1,0 | 1,2 | 1,0 | 0,7 | 1,2 | 1,9 | 2,0 | 2,0 | 0,8 | 1,0 | 2,0 | 0,7 | 2,2 | 1,0 | 3,2 | 1,0 | 1,0 |
| Apoa2 | 5,3 | 1,5 | 0,8 | 0,1 | 0,6 | 0,9 | 2,7 | 1,0 | 0,8 | 1,6 | 1,3 | 0,6 | 1,0 | 1,0 | 1,1 | 1,0 | 1,0 | 1,0 | 0,5 | 1,2 | 1,0 | 1,7 | 1,2 | 1,1 |
| Apoa4 | 1,0 | 1,0 | 1,0 | 1,0 | 1,0 | 1,0 | 1,0 | 1,0 | 1,0 | 1,0 | 1,1 | 1,0 | 1,0 | 3,2 | 1,0 | 1,0 | 1,0 | 1,0 | 1,0 | 1,5 | 1,0 | 7,7 | 1,0 | 1,0 |
| Apoa5 | 1,0 | 0,9 | 1,0 | 0,3 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Apobec2 | 1,0 | 0,6 | 1,1 | 0,6 | 1,0 | 1,0 | 0,6 | 0,8 | 1,0 | 1,0 | 1,1 | 1,2 | 1,6 | 0,8 | 2,7 | 0,4 | 1,1 | 0,9 | 0,9 | 0,8 | 1,4 | 1,0 | 1,9 | 17,9 |
| Apoc3 | 4,4 | 1,6 | 1,0 | 0,2 | 0,6 | 0,7 | 0,8 | 1,0 | 1,0 | 1,0 | 1,7 | 2,5 | 1,0 | 1,0 | 1,0 | 1,0 | 1,0 | 1,0 | 1,0 | 0,8 | 1,0 | 1,0 | 1,0 | 0,9 |
| Apoc4 | 1,3 | 1,5 | 1,0 | 0,3 | 1,0 | 1,0 | 1,0 | 1,0 | 1,0 | 1,0 | 1,0 | 1,0 | 3,7 | 0,8 | 1,0 | 1,0 | 1,0 | 1,0 | 1,0 | 1,0 | 1,0 | 1,0 | 1,0 | 1,0 |
| Apoe | 0,8 | 0,7 | 1,8 | 1,6 | 1,1 | 1,2 | 1,0 | 0,8 | 0,8 | 1,1 | 1,2 | 1,1 | 1,4 | 1,1 | 1,1 | 1,5 | 1,1 | 0,7 | 1,1 | 1,3 | 1,2 | 0,8 | 1,2 | 1,2 |
| Apoh | 1,1 | 1,3 | 1,0 | 0,2 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Apol10a | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Apol11b | 1,0 | 1,0 | 1,0 | 1,0 | 1,0 | 1,0 | 1,0 | 1,0 | 1,0 | 1,0 | 1,0 | 1,0 | 1,0 | 1,0 | 1,1 | 1,2 | 1,0 | 1,0 | 2,2 | 0,3 | 1,0 | 1,0 | 1,0 | 1,0 |
| Apol8 | 1,0 | 1,0 | 1,0 | 1,0 | 1,2 | 1,2 | 1,0 | 1,0 | 1,0 | 1,0 | 1,0 | 1,0 | 1,0 | 1,0 | 2,0 | 1,7 | 1,0 | 1,0 | 8,6 | 0,3 | 1,0 | 1,0 | 1,0 | 1,0 |
| Arg2 | 1,0 | 1,0 | 1,0 | 1,0 | 1,5 | 1,7 | 2,0 | 2,0 | 2,7 | 4,1 | 1,0 | 1,0 | 0,4 | 0,7 | 0,8 | 0,8 | 1,0 | 1,0 | 1,5 | 1,0 | 1,0 | 1,0 | 0,9 | 1,0 |
| Armcx6 | 1,1 | 0,9 | 1,0 | 1,0 | 0,9 | 0,9 | 0,8 | 0,9 | 1,0 | 1,0 | 1,2 | 1,1 | 0,4 | 0,7 | 0,7 | 1,2 | 0,7 | 0,6 | 0,6 | 1,1 | 1,0 | 0,9 | 0,7 | 0,7 |
| Arnt2 | 0,7 | 0,6 | 1,0 | 1,0 | 1,0 | 0,9 | 1,0 | 1,0 | 1,0 | 1,0 | 1,0 | 1,0 | 1,4 | 1,1 | 0,8 | 0,9 | 0,7 | 0,9 | 1,0 | 1,1 | 1,0 | 0,9 | 1,0 | 1,0 |
| Art1 | 1,0 | 1,0 | 0,9 | 0,9 | 1,0 | 1,0 | 0,5 | 0,5 | 1,0 | 1,0 | 1,0 | 1,0 | 2,2 | 0,9 | 4,7 | 0,3 | 1,1 | 1,2 | 1,0 | 1,0 | 0,9 | 1,1 | 1,3 | 9,6 |
| Asb11 | 1,0 | 1,0 | 1,0 | 1,7 | 0,9 | 1,7 | 1,0 | 0,8 | 2,8 | 2,9 | 1,0 | 1,0 | 4,5 | 1,4 | 7,8 | 0,4 | 1,8 | 1,7 | 1,0 | 1,0 | 1,0 | 1,0 | 1,8 | 13,2 |
| Asb16 | 1,0 | 1,0 | 1,0 | 1,0 | 1,1 | 0,7 | 1,0 | 1,0 | 1,0 | 1,3 | 1,0 | 1,0 | 2,0 | 1,2 | 3,5 | 0,3 | 1,2 | 1,6 | 1,0 | 1,0 | 1,0 | 1,0 | 1,6 | 5,7 |
| Asb2 | 1,0 | 1,0 | 1,0 | 0,7 | 1,0 | 1,2 | 0,8 | 0,8 | 1,0 | 1,0 | 1,0 | 1,0 | 3,5 | 1,0 | 3,2 | 0,3 | 1,3 | 0,9 | 0,9 | 1,1 | 1,1 | 0,8 | 1,4 | 3,0 |
| Asb5 | 1,0 | 1,0 | 1,0 | 1,0 | 1,0 | 1,0 | 1,0 | 1,0 | 1,0 | 1,0 | 1,0 | 1,0 | 2,3 | 1,0 | 3,6 | 0,4 | 1,0 | 1,0 | 1,0 | 1,0 | 1,0 | 1,0 | 1,1 | 11,4 |
| Ascl4 | 1,0 | 1,0 | 1,0 | 1,0 | 1,0 | 1,0 | 1,0 | 1,0 | 1,0 | 1,0 | 1,2 | 0,6 | 0,7 | 0,6 | 1,0 | 1,0 | 1,0 | 1,0 | 1,0 | 1,0 | 1,0 | 1,0 | 1,1 | 1,1 |
| Asprv1 | 1,0 | 1,0 | 1,0 | 1,0 | 1,4 | 1,4 | 3,8 | 2,6 | 1,0 | 1,0 | 1,0 | 1,1 | 1,3 | 1,5 | 1,8 | 2,0 | 1,0 | 1,0 | 1,2 | 1,3 | 0,5 | 1,3 | 39,1 | 1,0 |

[Fig. 23-2]

| | | | | | | | | | | | | | | | | | | | | | | | | |
|---|---|---|---|---|---|---|---|---|---|---|---|---|---|---|---|---|---|---|---|---|---|---|---|---|
| Atcayos | 1,0 | 1,0 | 1,0 | 1,0 | 1,0 | 0,6 | 1,0 | 1,0 | 1,0 | 1,0 | 1,7 | 0,6 | 1,1 | 1,5 | 6,7 | 0,2 | 1,0 | 1,7 | 1,0 | 1,0 | 1,2 | 0,6 | 1,1 | 5,8 |
| Atp10b | 1,0 | 1,0 | 1,0 | 1,0 | 1,0 | 1,0 | 1,0 | 1,0 | 1,0 | 1,0 | 1,0 | 1,0 | 1,0 | 1,4 | 1,0 | 1,0 | 1,0 | 1,0 | 1,0 | 1,0 | 1,0 | 1,0 | 1,7 | 0,9 |
| Atp2a1 | 1,0 | 1,0 | 0,9 | 0,3 | 1,0 | 1,0 | 1,0 | 0,8 | 1,0 | 1,0 | 1,7 | 1,4 | 3,2 | 1,2 | 4,8 | 0,3 | 1,3 | 1,6 | 1,0 | 1,0 | 1,0 | 1,2 | 1,5 | 11,1 |
| Atp2b3 | 0,7 | 0,6 | 1,0 | 1,0 | 0,9 | 0,9 | 1,0 | 1,0 | 1,0 | 1,0 | 1,0 | 1,0 | 1,0 | 1,0 | 2,2 | 0,8 | 0,8 | 1,0 | 1,0 | 1,0 | 1,0 | 1,0 | 1,8 | 1,6 |
| Atp6v0c-ps2 | 10,9 | 1,5 | 1,0 | 0,4 | 1,0 | 4,7 | 1,0 | 3,0 | 1,0 | 1,0 | 1,0 | 1,6 | 1,0 | 1,0 | 1,0 | 3,9 | 1,0 | 0,8 | 1,0 | 3,5 | 1,0 | 1,0 | 1,0 | 1,0 |
| Atp6v0d2 | 0,8 | 0,3 | 1,0 | 1,0 | 1,0 | 1,0 | 1,4 | 1,3 | 1,0 | 1,0 | 1,0 | 1,0 | 0,9 | 0,7 | 2,8 | 3,0 | 1,0 | 1,0 | 1,0 | 1,0 | 1,0 | 1,0 | 0,5 | 0,4 |
| BC100530 | 1,0 | 1,0 | 1,0 | 1,0 | 1,0 | 1,0 | 1,0 | 1,0 | 1,0 | 1,0 | 1,0 | 1,0 | 0,1 | 4,8 | 0,6 | 1,2 | 1,0 | 1,0 | 1,0 | 1,0 | 1,0 | 1,0 | 1,0 | 1,0 |
| Basp1 | 1,0 | 1,0 | 0,8 | 1,1 | 1,0 | 1,0 | 0,9 | 0,8 | 1,0 | 1,0 | 1,1 | 1,0 | 0,5 | 0,8 | 0,6 | 0,9 | 0,9 | 0,5 | 0,8 | 0,6 | 1,0 | 1,1 | 0,5 | 0,6 |
| Bcl2a1b | 1,2 | 0,8 | 1,3 | 1,6 | 0,8 | 1,4 | 1,0 | 0,7 | 1,0 | 1,0 | 0,5 | 0,9 | 1,1 | 1,2 | 1,6 | 2,4 | 1,0 | 1,0 | 0,8 | 0,9 | 1,0 | 1,0 | 1,1 | 1,2 |
| Bcl2l15 | 1,0 | 1,0 | 1,0 | 1,0 | 0,9 | 1,1 | 1,0 | 1,0 | 1,0 | 1,0 | 1,0 | 1,0 | 1,4 | 1,0 | 1,0 | 1,1 | 1,0 | 1,0 | 1,6 | 1,8 | 0,8 | 1,0 | 1,0 | 1,0 |
| Bcl6 | 0,9 | 0,9 | 0,9 | 1,0 | 1,2 | 1,1 | 1,2 | 0,9 | 1,0 | 1,0 | 1,5 | 0,8 | 1,5 | 1,2 | 1,2 | 0,8 | 0,5 | 0,7 | 0,9 | 0,7 | 1,1 | 1,2 | 0,9 | 1,0 |
| Bcmo1 | 1,0 | 1,0 | 1,0 | 1,0 | 1,0 | 1,0 | 1,0 | 1,0 | 1,0 | 1,0 | 1,0 | 1,0 | 1,3 | 0,9 | 1,0 | 1,0 | 1,0 | 1,0 | 1,0 | 1,0 | 1,1 | 1,1 | 1,0 | 1,0 |
| Bex1 | 1,0 | 0,7 | 1,0 | 1,0 | 1,1 | 1,3 | 1,2 | 0,7 | 1,0 | 1,0 | 1,1 | 1,0 | 2,0 | 0,3 | 2,2 | 0,4 | 1,0 | 0,8 | 1,0 | 1,0 | 1,1 | 2,2 | 1,5 | 1,3 |
| Bex2 | 1,1 | 0,8 | 0,9 | 1,3 | 1,0 | 1,2 | 0,8 | 0,6 | 1,6 | 1,6 | 0,9 | 1,0 | 1,0 | 1,0 | 1,9 | 0,9 | 1,0 | 1,0 | 1,0 | 1,0 | 1,1 | 0,8 | 0,8 | 0,9 |
| Bex4 | 1,1 | 0,8 | 0,6 | 0,8 | 0,9 | 1,2 | 1,1 | 0,9 | 1,0 | 1,1 | 1,0 | 1,0 | 1,0 | 1,0 | 1,9 | 2,4 | 1,0 | 0,7 | 8,0 | 0,6 | 1,1 | 1,1 | 1,1 | 1,1 |
| | AG | | Aorta | | Brain | | Lung | | Pancreas | | PG | | Skin | | Skull | | SM | | Spleen | | Testis | | TG | |
| | E | M | E | . M | E | M | E | M | E | M | E | M | E | M | E | M | E | M | E | M | E | M | E | M |
| Bglap3 | 1,0 | 1,0 | 1,0 | 1,0 | 1,0 | 1,0 | 1,0 | 1,0 | 0,9 | 0,8 | 1,0 | 1,0 | 0,8 | 1,8 | 0,7 | 1,3 | 1,0 | 1,0 | 1,0 | 1,0 | 1,0 | 1,0 | 1,3 | 1,0 |
| Bhmt | 1,4 | 0,6 | 1,0 | 0,3 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,5 | 0,7 | 1,0 | 1,0 |
| Bmyc | 1,1 | 1,2 | 0,8 | 0,7 | 1,1 | 1,1 | 0,7 | 0,9 | 0,6 | 1,0 | 0,8 | 0,9 | 1,0 | 1,0 | 1,0 | 1,1 | 1,1 | 0,9 | 0,9 | 1,1 | 0,8 | 0,7 | 0,9 | 0,8 |
| Bpi | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,1 | 0,8 | 1,0 | 1,0 |
| Bpifb1 | 1,0 | 1,0 | 1,0 | 1,0 | 1,0 | 1,0 | 0,7 | 1,1 | 1,0 | 1,0 | 1,0 | 1,0 | 1,0 | 1,0 | 1,0 | 1,0 | 1,0 | 1,0 | 1,0 | 1,0 | 1,0 | 1,0 | 2,4 | 0,0 |
| Bsph1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Bspry | 1,1 | 1,4 | 1,0 | 1,0 | 1,0 | 1,0 | 1,3 | 1,2 | 1,1 | 1,2 | 1,2 | 1,1 | 1,3 | 1,0 | 1,0 | 1,0 | 1,0 | 1,0 | 0,8 | 2,0 | 1,1 | 0,9 | 1,0 | 1,0 |
| Btg3 | 17,1 | 0,3 | 1,1 | 2,0 | 1,0 | 1,0 | 2,5 | 1,0 | 1,0 | 1,9 | 1,0 | 3,4 | 2,4 | 1,0 | 0,2 | 1,0 | 1,0 | 1,4 | 1,0 | 1,0 | 2,3 | 1,0 | 1,2 | 2,4 |
| Btnl10 | 1,0 | 1,0 | 1,0 | 1,0 | 1,0 | 1,0 | 1,0 | 1,0 | 1,0 | 1,0 | 1,0 | 1,0 | 1,0 | 1,0 | 1,5 | 1,2 | 1,0 | 1,0 | 7,9 | 0,3 | 0,9 | 1,0 | 1,0 | 1,0 |
| C1qtnf3 | 0,9 | 1,0 | 1,2 | 1,0 | 1,0 | 1,0 | 0,6 | 0,8 | 1,0 | 1,0 | 1,0 | 1,0 | 0,9 | 1,3 | 0,4 | 0,3 | 0,5 | 1,0 | 1,0 | 1,0 | 1,0 | 1,1 | 1,0 | 1,0 |
| C1qtnf6 | 0,9 | 1,2 | 0,8 | 0,8 | 0,7 | 0,7 | 1,0 | 0,9 | 1,0 | 1,0 | 1,5 | 1,1 | 0,1 | 0,4 | 0,7 | 0,9 | 0,1 | 0,3 | 1,0 | 1,0 | 0,5 | 0,8 | 0,9 | 0,8 |
| C2cd4b | 0,9 | 0,9 | 1,0 | 1,0 | 0,8 | 0,5 | 1,1 | 1,3 | 4,4 | 13,6 | 0,7 | 1,0 | 1,0 | 1,0 | 1,0 | 1,0 | 1,0 | 1,0 | 1,0 | 1,0 | 0,9 | 0,6 | 1,1 | 0,2 |
| C3 | 1,5 | 1,6 | 1,1 | 1,0 | 1,0 | 1,0 | 1,0 | 1,1 | 1,0 | 0,6 | 1,0 | 1,0 | 1,2 | 1,3 | 0,7 | 0,7 | 1,1 | 0,9 | 1,1 | 1,2 | 1,2 | 1,0 | 1,6 | 1,5 |
| C4bp | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| C6 | 1,3 | 1,0 | 1,0 | 1,0 | 1,0 | 1,0 | 0,7 | 1,1 | 1,0 | 1,0 | 1,0 | 1,0 | 0,7 | 0,6 | 0,7 | 1,0 | 1,0 | 1,0 | 1,3 | 1,4 | 1,0 | 1,0 | 1,0 | 1,0 |
| C8b | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,1 | 1,0 | 1,0 | 1,0 |
| Cacna1s | 1,0 | 1,0 | 1,0 | 1,0 | 1,0 | 1,0 | 1,0 | 1,0 | 1,0 | 1,0 | 1,0 | 1,0 | 2,3 | 1,1 | 4,0 | 0,3 | 1,0 | 1,4 | 0,9 | 0,7 | 0,9 | 1,1 | 1,2 | 14,0 |
| Cacng1 | 1,0 | 1,0 | 1,0 | 1,0 | 1,0 | 1,0 | 1,0 | 1,0 | 1,0 | 1,0 | 1,0 | 1,0 | 2,9 | 1,0 | 4,1 | 0,4 | 1,3 | 1,5 | 1,0 | 1,0 | 1,0 | 1,0 | 1,4 | 7,0 |
| Cacng6 | 1,0 | 1,0 | 1,0 | 1,0 | 1,0 | 1,0 | 1,0 | 1,0 | 1,0 | 1,0 | 1,5 | 1,9 | 1,8 | 0,6 | 4,6 | 0,2 | 1,0 | 1,4 | 1,0 | 1,0 | 1,0 | 1,0 | 1,0 | 10,3 |
| Calm4 | 1,1 | 1,0 | 1,0 | 1,0 | 1,0 | 1,0 | 1,0 | 1,0 | 1,0 | 1,0 | 1,0 | 1,0 | 1,1 | 2,0 | 1,0 | 1,0 | 1,0 | 0,9 | 1,0 | 1,0 | 1,0 | 1,2 | 13,5 | 1,0 |
| Calml3 | 1,0 | 1,0 | 1,0 | 1,0 | 1,0 | 1,0 | 0,9 | 1,0 | 1,0 | 1,0 | 1,0 | 1,0 | 0,8 | 1,0 | 1,0 | 1,0 | 1,0 | 1,0 | 1,0 | 1,0 | 0,8 | 0,7 | 0,9 | 0,7 |
| Camk1g | 1,0 | 1,0 | 1,0 | 1,0 | 1,0 | 1,2 | 1,0 | 1,0 | 1,0 | 1,0 | 0,9 | 1,1 | 1,0 | 1,0 | 1,0 | 1,0 | 1,0 | 1,0 | 1,0 | 1,0 | 1,0 | 0,9 | 0,5 | 0,2 |
| Camk2a | 0,7 | 0,7 | 1,0 | -1,0 | 1,1 | 1,0 | 0,9 | 1,0 | 1,0 | 1,0 | 1,2 | 1,4 | 1,4 | 0,7 | 1,3 | 0,4 | 0,8 | 1,0 | 0,8 | 1,0 | 1,0 | 1,0 | 0,8 | 6,9 |
| Camk2b | 0,7 | 0,6 | 0,5 | 0,5 | 1,0 | 1,1 | 1,4 | 1,2 | 0,8 | 1,0 | 1,1 | 1,0 | 2,2 | 1,0 | 1,7 | 0,7 | 1,0 | 1,0 | 0,9 | 1,2 | 1,0 | 0,8 | 1,1 | 3,1 |
| Cap2 | 1,2 | 0,7 | 0,7 | 0,8 | 1,0 | 1,0 | 0,6 | 0,7 | 1,0 | 1,0 | 1,2 | 1,1 | 2,2 | 1,0 | 2,7 | 0,4 | 1,0 | 1,0 | 1,0 | 1,0 | 1,1 | 1,2 | 1,2 | 7,1 |
| Capn5 | 0,8 | 1,1 | 0,9 | 1,0 | 1,1 | 0,9 | 1,0 | 1,1 | 0,9 | 0,8 | 1,3 | 1,0 | 1,1 | 0,9 | 1,1 | 1,1 | 0,8 | 0,8 | 1,4 | 0,8 | 1,0 | 1,0 | 1,0 | 0,9 |
| Car1 | 1,0 | 1,0 | 1,4 | 1,2 | 1,5 | 1,0 | 1,0 | 1,0 | 1,0 | 1,0 | 1,0 | 1,0 | 1,0 | 1,0 | 1,7 | 1,8 | 2,1 | 1,0 | 4,0 | 0,3 | 1,0 | 0,8 | 1,0 | 1,0 |
| Car2 | 1,3 | 1,1 | 1,2 | 2,3 | 0,9 | 0,9 | 1,0 | 0,8 | 1,0 | 1,0 | 1,9 | 1,4 | 1,5 | 1,2 | 1,7 | 1,6 | 1,0 | 1,2 | 7,9 | 0,3 | 1,1 | 0,8 | 0,9 | 0,8 |
| Car3 | 2,1 | 1,7 | 1,1 | 1,5 | 1,0 | 1,0 | 0,7 | 0,3 | 0,4 | 0,1 | 1,0 | 1,0 | 1,3 | 1,3 | 0,9 | 0,7 | 1,2 | 1,2 | 3,4 | 2,1 | 2,1 | 1,9 | 1,2 | 1,9 |
| Car4 | 2,6 | 1,5 | 0,8 | 1,5 | 1,0 | 0,9 | 1,0 | 1,1 | 0,7 | 1,0 | 1,0 | 1,0 | 2,3 | 1,6 | 1,5 | 1,0 | 1,6 | 1,4 | 1,0 | 1,0 | 1,0 | 1,1 | 0,5 | 0,6 |
| Car5b | 1,6 | 1,6 | 1,3 | 1,4 | 1,0 | 1,0 | 1,0 | 0,8 | 0,7 | 1,2 | 1,0 | 1,0 | 1,5 | 2,2 | 1,4 | 1,2 | 1,1 | 0,7 | 0,9 | 1,0 | 0,9 | 1,5 | 1,2 | 1,2 |
| Car6 | 0,6 | 1,0 | 1,0 | 1,3 | 0,7 | 1,0 | 1,5 | 0,7 | 0,8 | 1,0 | 0,6 | 1,0 | 1,9 | 0,7 | 1,3 | 1,0 | 1,0 | 0,2 | 1,0 | 1,0 | 1,0 | 1,0 | 1,2 | 0,8 |
| Casc5 | 1,0 | 1,0 | 1,0 | 1,0 | 1,0 | 1,0 | 1,0 | 1,0 | 1,0 | 1,0 | 1,0 | 1,0 | 0,8 | 0,9 | 1,3 | 0,7 | 1,0 | 1,0 | 1,4 | 0,5 | 0,8 | 1,1 | 1,0 | 1,0 |
| Casq1 | 1,0 | 1,0 | 1,1 | 0,8 | 1,0 | 1,0 | 1,0 | 1,0 | 1,0 | 1,0 | 1,0 | 1,0 | 1,4 | 0,6 | 2,9 | 0,2 | 0,6 | 0,8 | 1,0 | 1,0 | 1,1 | 0,7 | 0,6 | 9,3 |
| Cav3 | 1,0 | 1,0 | 1,1 | 1,2 | 1,0 | 1,0 | 0,7 | 0,6 | 1,0 | 1,0 | 1,0 | 1,0 | 1,3 | 0,9 | 2,6 | 0,3 | 0,8 | 0,8 | 1,0 | 1,0 | 1,0 | 1,0 | 1,5 | 7,0 |
| Cbl | 0,6 | 0,5 | 1,3 | 1,0 | 0,7 | 0,6 | 0,7 | 1,9 | 1,0 | 1,0 | 1,3 | 1,1 | 0,6 | 1,0 | 1,2 | 0,5 | 1,0 | 1,0 | 0,9 | 1,0 | 1,0 | 1,0 | 0,9 | 0,5 |
| Cbr1 | 1,0 | 1,1 | 1,8 | 2,0 | 1,1 | 1,1 | 1,0 | 0,8 | 0,9 | 1,0 | 1,0 | 1,0 | 1,3 | 1,5 | 1,1 | 1,3 | 1,2 | 1,1 | 1,1 | 1,1 | 1,0 | 1,0 | 1,1 | 1,1 |
| Cbr3 | 1,1 | 1,3 | 2,9 | 2,2 | 1,5 | 1,2 | 1,2 | 0,9 | 0,9 | 1,0 | 1,3 | 0,9 | 0,9 | 1,4 | 0,9 | 1,2 | 1,0 | 0,6 | 1,0 | 1,3 | 0,9 | 0,5 | 2,0 | 1,4 |
| Ccl17 | 1,3 | 1,2 | 1,0 | 1,0 | 1,0 | 2,1 | 1,4 | 0,5 | 1,0 | 1,0 | 0,7 | 1,3 | 2,0 | 6,1 | 0,6 | 1,0 | 1,0 | 1,0 | 1,0 | 1,0 | 0,9 | 1,8 | 1,0 | 1,0 |
| Ccl2 | 0,7 | 1,1 | 1,8 | 0,7 | 1,0 | 1,0 | 0,9 | 0,5 | 1,0 | 1,0 | 1,0 | 1,0 | 0,5 | 0,8 | 0,7 | 0,9 | 1,0 | 0,5 | 0,7 | 0,6 | 1,0 | 1,0 | 1,0 | 1,0 |
| Ccl20 | 1,0 | 1,0 | 1,0 | 1,0 | 1,0 | 1,0 | 1,0 | 1,0 | 1,0 | 1,0 | 1,0 | 1,0 | 3,4 | 0,9 | 1,0 | 1,0 | 1,0 | 1,0 | 1,0 | 1,0 | 1,0 | 1,0 | 1,0 | 1,0 |
| Ccl21b | 1,3 | 1,2 | 0,2 | 1,3 | 1,1 | 1,8 | 0,2 | 2,1 | 0,5 | 0,5 | 1,0 | 1,7 | 1,0 | 0,4 | 0,5 | 1,6 | 2,8 | 4,0 | 1,0 | 1,0 | 0,7 | 1,4 | 1,9 | 5,4 |
| Ccl6 | 1,5 | 1,2 | 1,6 | 2,2 | 1,1 | 1,0 | 1,3 | 1,3 | 0,9 | 1,5 | 1,2 | 1,0 | 0,9 | 1,3 | 1,0 | 1,7 | 1,3 | 1,1 | 1,2 | 1,5 | 1,0 | 1,0 | 2,0 | 2,2 |
| Ccl7 | 1,0 | 1,0 | 1,4 | 1,3 | 1,0 | 1,0 | 1,1 | 0,4 | 1,0 | 1,0 | 1,0 | 1,0 | 0,5 | 0,8 | 0,5 | 0,6 | 1,0 | 1,0 | 1,0 | 1,0 | 1,0 | 1,0 | 1,0 | 1,0 |
| Ccl8 | 2,7 | 1,4 | 0,8 | 0,6 | 1,0 | 1,0 | 0,9 | 0,5 | 1,0 | 1,0 | 1,0 | 0,9 | 0,5 | 1,0 | 1,4 | 1,0 | 0,8 | 0,2 | 0,4 | 0,5 | 0,9 | 0,9 | 2,5 | 0,6 |
| Ccnb1 | 1,0 | 1,2 | 1,0 | ·1,0 | 1,0 | 1,0 | 1,0 | 1,0 | 1,0 | 1,0 | 1,0 | 1,0 | 0,2 | 0,5 | 1,2 | 1,1 | 1,0 | 1,0 | 1,9 | 0,4 | 0,9 | 0,9 | 1,0 | 1,0 |
| Ccne2 | 1,0 | 1,0 | 1,0 | 1,0 | 0,8 | 1,2 | 1,0 | 1,0 | 1,0 | 1,0 | 1,0 | 1,0 | 0,5 | 0,9 | 1,7 | 1,1 | 0,8 | 0,8 | 2,8 | 0,4 | 1,0 | 1,5 | 1,0 | 1,0 |
| Ccno | 1,0 | 1,0 | 1,6 | 4,8 | 1,1 | 1,2 | 0,8 | 0,9 | 1,0 | 1,0 | 1,0 | 1,0 | 1,0 | 1,0 | 0,9 | 0,8 | 1,0 | 1,0 | 1,0 | 1,0 | 1,0 | 0,9 | 1,0 | 1,0 |
| Cd19 | 1,0 | 1,0 | 1,0 | 1,0 | 1,0 | 1,0 | 0,4 | 0,5 | 1,0 | 1,0 | 1,0 | 1,0 | 1,0 | 1,0 | 0,7 | 1,2 | 1,0 | 1,0 | 0,9 | 0,9 | 1,0 | 1,0 | 1,0 | 1,0 |
| Cd22 | 1,0 | 1,0 | 1,0 | 1,0 | 1,0 | 1,0 | 0,5 | 0,7 | 1,0 | 1,0 | 1,0 | 1,0 | 1,0 | 1,0 | 1,1 | 1,6 | 1,0 | 1,0 | 0,9 | 1,0 | 1,0 | 1,0 | 1,0 | 1,0 |
| Cd27 | 1,0 | 1,0 | 1,0 | 1,0 | 1,0 | 1,0 | 0,6 | 0,9 | 1,0 | 1,0 | 1,1 | 1,7 | 1,1 | 1,5 | 1,4 | 1,6 | 0,8 | 0,5 | 1,0 | 1,1 | 1,0 | 1,0 | 0,1 | 1,0 |
| Cd300lh | 0,9 | 1,0 | 1,7 | 0,8 | 1,0 | 1,0 | 1,3 | 1,3 | 1,0 | 1,0 | 1,0 | 1,0 | 0,4 | 1,0 | 0,8 | 0,7 | 1,2 | 0,5 | 0,6 | 0,5 | 1,0 | 1,0 | 0,3 | 0,5 |
| Cd3e | 1,0 | 1,0 | 1,0 | 1,0 | 1,0 | 1,0 | 0,8 | 0,8 | 1,0 | 1,0 | 1,0 | 1,0 | 1,2 | 1,5 | 2,1 | 2,1 | 1,0 | 1,0 | 1,1 | 0,9 | 1,0 | 1,0 | 0,1 | 1,0 |
| Cd4 | 1,0 | 1,0 | 1,0 | 1,0 | 1,2 | 1,1 | 0,8 | 0,8 | 1,0 | 1,0 | 0,8 | 1,0 | 0,7 | 1,4 | 1,0 | 0,9 | 1,0 | 1,0 | 1,0 | 0,9 | 1,0 | 1,0 | 0,0 | 1,0 |
| Cd52 | 1,1 | 0,8 | 1,4 | 1,1 | 1,5 | 1,5 | 0,9 | 0,7 | 1,0 | 2,3 | 1,4 | 1,1 | 1,0 | 1,6 | 0,8 | 1,4 | 1,2 | 0,6 | 0,9 | 0,9 | 1,9 | 7,3 | 0,8 | 1,3 |
| Cd5l | 0,4 | 0,1 | 3,0 | 0,8 | 1,0 | 1,0 | 1,0 | 0,6 | 1,0 | 1,0 | 1,0 | 1,0 | 1,0 | 1,0 | 1,2 | 2,3 | 1,0 | 1,0 | 1,1 | 1,2 | 1,0 | 1,1 | 1,0 | 1,0 |
| Cd79a | 2,0 | 1,0 | 0,8 | 1,0 | 1,0 | 1,0 | 0,4 | 0,6 | 1,0 | 1,0 | 1,7 | 1,6 | 1,0 | 1,0 | 0,8 | 1,2 | 1,0 | 1,0 | 0,8 | 1,0 | 1,0 | 1,0 | 1,0 | 1,0 |
| Cd79b | 1,9 | 1,2 | 1,7 | 0,8 | 1,0 | 1,0 | 0,4 | 0,5 | 1,0 | 1,0 | 0,9 | 1,1 | 0,7 | 1,0 | 0,8 | 1,3 | 1,0 | 1,0 | 0,8 | 0,9 | 1,0 | 1,0 | 0,7 | 0,8 |
| Cd8a | 1,0 | 1,0 | 1,0 | 1,0 | 1,0 | 1,0 | 0,7 | 0,8 | 1,0 | 1,0 | 1,0 | 1,0 | 1,0 | 1,0 | 2,0 | 2,7 | 1,0 | 1,0 | 1,0 | 1,2 | 1,0 | 1,0 | 0,0 | 1,0 |
| Cd8b1 | 1,0 | 1,0 | 1,0 | 1,0 | 1,0 | 1,0 | 0,5 | 0,9 | 1,0 | 1,0 | 1,0 | 1,0 | 1,0 | 1,0 | 2,6 | 3,6 | 1,0 | 1,0 | 1,1 | 1,2 | 1,2 | 0,8 | 0,1 | 1,0 |
| Cdcp1 | 1,0 | 1,0 | 1,0 | 1,0 | 1,0 | 1,0 | 1,1 | 1,7 | 1,4 | 1,2 | 1,1 | 1,1 | 1,1 | 1,1 | 1,0 | 1,0 | 1,0 | 1,0 | 1,4 | 1,0 | 1,0 | 1,0 | 1,0 | 0,8 |
| Cdh3 | 1,0 | 1,0 | 1,0 | 1,0 | 1,0 | 1,0 | 1,0 | 1,0 | 1,0 | 1,0 | 1,0 | 1,0 | 0,7 | 0,8 | 1,0 | 1,0 | 1,0 | 1,0 | 1,0 | 1,0 | 1,0 | 1,0 | 1,0 | 1,6 |
| Cdk6 | 1,0 | 1,0 | 1,6 | 1,1 | 1,0 | 1,0 | 0,8 | 1,2 | 1,0 | 1,0 | 1,0 | 1,0 | 0,5 | 0,9 | 0,9 | 0,6 | 1,0 | 1,0 | 0,9 | 1,1 | 0,9 | 1,2 | 1,0 | 0,7 |
| Ceacam10 | 1,0 | 1,0 | 1,0 | 1,0 | 1,0 | 1,0 | 1,0 | 1,0 | 1,0 | 1,0 | 1,1 | 0,9 | 1,0 | 1,0 | 0,6 | 0,9 | 1,0 | 1,0 | 1,0 | 1,0 | 1,0 | 1,2 | 0,8 | 0,9 |
| Cel | 1,0 | 1,0 | 1,0 | 1,0 | 1,0 | 1,0 | 1,0 | 1,0 | 1,1 | 1,2 | 0,5 | 0,6 | 1,7 | 1,0 | 1,4 | 0,7 | 1,0 | 1,0 | 1,0 | 1,0 | 1,0 | 1,0 | 0,8 | 1,0 |
| Cela1 | 1,0 | 1,2 | 0,6 | 0,4 | 1,2 | 1,3 | 0,8 | 0,9 | 0,8 | 0,9 | 0,7 | 0,7 | 2,5 | 1,1 | 1,5 | 1,5 | 1,0 | 0,9 | 1,7 | 1,1 | 1,0 | 1,0 | 1,1 | 1,0 |
| Cela2a | 0,7 | 0,8 | 1,0 | 1,0 | 0,8 | 0,6 | 0,7 | 1,0 | 0,9 | 1,0 | 0,5 | 1,0 | 1,8 | 1,0 | 1,7 | 0,7 | 1,0 | 1,0 | 1,0 | 0,9 | 1,0 | 1,0 | 0,8 | 1,0 |

[Fig. 23-3]

| | | | | | | | | | | | | | | | | | | | | | | | | | | |
|---|---|---|---|---|---|---|---|---|---|---|---|---|---|---|---|---|---|---|---|---|---|---|---|---|---|---|
| Cela3b | 1,0 | 0,7 | 1,0 | 1,0 | 0,9 | 1,8 | 1,4 | 1,0 | 1,5 | 1,5 | 0,9 | 1,0 | 1,0 | 1,0 | 1,0 | 1,1 | 1,0 | 1,0 | 1,0 | 1,1 | 1,0 | 1,0 | 0,9 | 1,0 |
| Ces1c | 1,0 | 1,0 | 1,0 | 0,7 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Ces1g | 1,0 | 1,0 | 1,0 | 1,0 | 1,0 | 1,0 | 1,3 | 0,9 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Ces2g | 1,0 | 1,0 | 0,8 | 0,9 | 1,0 | 1,0 | 0,6 | 0,9 | 1,2 | 1,2 | 1,6 | 1,5 | 0,5 | 0,8 | 1,4 | 1,3 | 1,0 | 1,0 | 4,3 | 0,2 | 1,0 | 1,0 | 0,7 | 0,6 |
| Ces3a | 1,0 | 1,0 | 1,0 | 0,3 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Ces3b | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Ces5a | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,1 | 2,7 | 1,0 | 1,0 |
| Cfd | 2,7 | 2,3 | 1,8 | 1,9 | 1,0 | 1,0 | 1,0 | 0,2 | 0,7 | 0,1 | 1,0 | 1,0 | 1,7 | 1,9 | 1,7 | 2,9 | 1,4 | 0,9 | 7,5 | 4,0 | 1,8 | 3,5 | 2,2 | 1,2 |
| Cgref1 | 0,9 | 1,1 | 0,9 | 1,0 | 1,0 | 1,1 | 1,0 | 0,5 | 1,9 | 2,1 | 0,6 | 0,7 | 0,1 | 0,3 | 0,5 | 1,1 | 0,7 | 0,9 | 1,0 | 1,0 | 1,0 | 1,2 | 0,1 | 0,7 |
| Chac1 | 1,0 | 1,8 | 1,0 | 1,0 | 0,8 | 1,1 | 0,9 | 1,1 | 1,2 | 2,2 | 0,7 | 1,0 | 2,6 | 0,5 | 1,1 | 0,3 | 1,3 | 1,5 | 1,0 | 0,7 | 0,9 | 0,7 | 0,6 | 12,4 |
| Chga | 0,9 | 0,7 | 1,2 | 0,9 | 1,0 | 1,0 | 1,0 | 0,7 | 1,5 | 0,7 | 1,4 | 1,6 | 1,0 | 1,9 | 1,3 | 0,8 | 1,0 | 1,0 | 1,0 | 1,0 | 1,0 | 0,9 | 13,1 | 5,8 |
| Chrna4 | 0,9 | 1,2 | 1,0 | 1,0 | 0,9 | 0,9 | 1,0 | 1,0 | 1,0 | 1,0 | 1,1 | 0,9 | 1,0 | 0,4 | 1,0 | 1,0 | 1,0 | 1,0 | 1,0 | 1,0 | 1,0 | 1,0 | 1,0 | 1,0 |
| Chst11 | 0,9 | 1,1 | 1,1 | 1,1 | 0,9 | 0,9 | 1,0 | 1,1 | 1,0 | 1,0 | 1,0 | 0,7 | 0,7 | 1,0 | 1,0 | 1,1 | 1,0 | 0,8 | 1,1 | 1,0 | 1,0 | 1,0 | 0,9 | 0,7 |
| Cib3 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,9 |
| Cidea | 1,1 | 1,0 | 0,7 | 1,0 | 0,9 | 1,4 | 1,0 | 0,3 | 1,0 | 1,0 | 0,5 | 1,1 | 1,1 | 2,5 | 1,0 | 1,0 | 0,6 | 0,1 | 1,0 | 1,0 | 1,0 | 0,8 | 1,4 | 1,7 |
| Cilp | 0,7 | 0,7 | 1,1 | 1,2 | 1,0 | 1,0 | 0,8 | 0,6 | 1,0 | 1,0 | 0,8 | 1,0 | 1,5 | 1,9 | 1,8 | 0,3 | 0,6 | 1,0 | 1,0 | 1,0 | 1,0 | 1,0 | 0,6 | 1,2 |
| Cilp2 | 1,0 | 1,0 | 1,0 | 0,7 | 1,0 | 1,0 | 1,0 | 1,0 | 1,0 | 1,0 | 1,0 | 1,0 | 1,0 | 0,9 | 0,6 | 0,6 | 0,1 | 0,5 | 1,0 | 1,0 | 1,0 | 1,0 | 1,0 | 1,0 |
| Cited1 | 0,4 | 0,1 | 1,0 | 1,0 | 1,1 | 1,3 | 1,0 | 1,0 | 1,0 | 1,0 | 2,0 | 2,0 | 1,0 | 1,0 | 0,7 | 1,0 | 1,3 | 0,5 | 1,0 | 1,0 | 1,2 | 1,0 | 0,6 | 1,2 |
| Cited4 | 0,8 | 1,3 | 1,0 | 1,0 | 1,1 | 1,0 | 1,0 | 0,9 | 0,5 | 0,7 | 1,4 | 1,0 | 1,4 | 0,6 | 1,0 | 0,7 | 0,0 | 0,4 | 1,6 | 0,6 | 1,1 | 0,8 | 0,9 | 1,6 |
| Ckm | 4,0 | 1,0 | 0,7 | 0,5 | 1,0 | 1,0 | 0,6 | 0,6 | 1,0 | 1,0 | 1,0 | 1,0 | 2,7 | 0,9 | 4,9 | 0,3 | 1,3 | 1,2 | 0,9 | 1,0 | 0,8 | 1,5 | 1,3 | 14,5 |
| Ckmt2 | 1,0 | 1,0 | 0,9 | 0,8 | 1,0 | 1,0 | 0,8 | 0,9 | 1,0 | 1,0 | 1,0 | 1,0 | 1,5 | 0,7 | 7,2 | 0,2 | 0,6 | 0,7 | 1,0 | 1,0 | 1,3 | 0,8 | 1,5 | 3,3 |
| Clca3 | 1,0 | 1,0 | 1,0 | 1,0 | 1,0 | 1,0 | 0,0 | 0,6 | 1,0 | 1,0 | 1,0 | 1,0 | 1,0 | 0,9 | 1,0 | 1,0 | 1,5 | 1,0 | 1,0 | 1,0 | 1,0 | 0,9 | 1,0 | 1,0 |
| Cldn1 | 0,8 | 0,9 | 1,1 | 2,6 | 1,5 | 1,4 | 0,9 | 0,8 | 1,0 | 1,0 | 1,0 | 1,0 | 1,0 | 1,0 | 1,1 | 1,3 | 0,6 | 1,1 | 1,0 | 1,0 | 1,0 | 1,0 | 1,0 | 1,2 |
| Cldn13 | 1,0 | 1,0 | 1,0 | 1,0 | 1,0 | 1,0 | 1,0 | 1,0 | 1,0 | 1,0 | 1,0 | 1,0 | 1,0 | 1,0 | 1,7 | 1,9 | 1,0 | 1,0 | 7,4 | 0,2 | 1,7 | 1,3 | 1,0 | 1,0 |
| Cldn14 | 1,0 | 1,0 | 1,0 | 1,0 | 1,0 | 1,1 | 1,0 | 1,0 | 1,0 | 1,0 | 1,3 | 1,0 | 1,2 | 0,5 | 1,0 | 1,0 | 1,0 | 1,0 | 1,0 | 1,0 | 1,0 | 1,0 | 1,0 | 1,0 |
| Cldn2 | 1,0 | 1,0 | 1,0 | 1,0 | 0,6 | 0,9 | 1,0 | 1,0 | 1,0 | 1,0 | 1,2 | 1,1 | 1,0 | 1,0 | 2,2 | 1,3 | 1,0 | 1,0 | 1,0 | 1,0 | 1,0 | 1,0 | 0,2 | 1,0 |
| Cldn22 | 1,7 | 1,6 | 0,9 | 0,8 | 1,2 | 0,9 | 0,9 | 0,8 | 1,0 | 1,0 | 1,0 | 1,0 | 1,8 | 2,2 | 1,0 | 1,6 | 0,8 | 0,8 | 1,0 | 1,0 | 1,0 | 1,0 | 2,3 | 1,1 |
| Cldn24 | 2,0 | 0,9 | 0,8 | 0,7 | 1,0 | 1,0 | 1,0 | 1,0 | 1,0 | 1,0 | 1,0 | 1,0 | 1,0 | 1,1 | 1,0 | 1,0 | 1,0 | 1,0 | 1,0 | 1,0 | 1,0 | 1,0 | 1,0 | 1,0 |
| Cldn3 | 0,8 | 1,1 | 1,0 | 1,1 | 1,2 | 1,0 | 1,2 | 1,0 | 1,0 | 1,0 | 1,3 | 1,1 | 1,4 | 0,7 | 1,0 | 1,0 | 1,0 | 1,0 | 1,0 | 1,0 | 1,1 | 0,7 | 1,1 | 1,2 |
| Cldn4 | 1,0 | 1,0 | 1,0 | 1,0 | 1,0 | 1,0 | 1,0 | 1,1 | 1,0 | 1,9 | 1,1 | 1,1 | 1,3 | 0,9 | 1,0 | 1,0 | 1,0 | 1,0 | 1,0 | 1,0 | 1,0 | 1,0 | 1,0 | 1,2 |
| | AG | | Aorta | | Brain | | Lung | | Pancreas | | PG | | Skin | | Skull | | SM | | Spleen | | Testis | | TG | |
| | E | M | E | M | E | M | E | M | E | M | E | M | E | M | E | M | E | M | E | M | E | M | E | M |
| Cldn7 | 1,0 | 1,0 | 1,0 | 1,0 | 1,0 | 1,0 | 1,1 | 0,9 | 1,5 | 1,2 | 1,2 | 1,1 | 1,0 | 0,8 | 1,0 | 1,0 | 1,0 | 1,0 | 1,0 | 1,0 | 1,0 | 1,0 | 0,9 | 1,0 |
| Cldn8 | 1,0 | 1,0 | 1,0 | 1,0 | 1,0 | 1,0 | 1,0 | 0,9 | 0,9 | 1,6 | 1,0 | 1,1 | 0,9 | 0,8 | 1,0 | 1,0 | 1,0 | 1,0 | 1,0 | 1,0 | 1,0 | 1,0 | 1,3 | 1,1 |
| Clec11a | 0,8 | 0,6 | 0,8 | 0,8 | 0,9 | 0,9 | 0,5 | 0,5 | 1,0 | 1,0 | 0,8 | 1,2 | 0,1 | 0,4 | 0,7 | 0,8 | 0,5 | 0,4 | 0,7 | 0,8 | 1,3 | 0,6 | 0,7 | 0,7 |
| Clec2h | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Clec2l | 1,5 | 2,5 | 1,0 | 1,0 | 1,0 | 1,1 | 1,0 | 1,0 | 1,0 | 1,0 | 1,3 | 1,2 | 1,0 | 1,0 | 1,0 | 1,0 | 1,0 | 1,0 | 1,0 | 1,0 | 1,0 | 1,0 | 1,0 | 1,0 |
| Clec7a | 0,7 | 0,4 | 1,1 | 1,0 | 1,0 | 1,0 | 1,0 | 0,9 | 1,0 | 1,0 | 0,9 | 0,9 | 0,9 | 2,2 | 0,7 | 0,9 | 1,2 | 1,0 | 0,8 | 0,9 | 1,0 | 1,0 | 0,7 | 1,1 |
| Clhc1 | 0,6 | 0,7 | 1,0 | 1,0 | 1,0 | 1,0 | 1,0 | 1,0 | 1,0 | 1,0 | 1,0 | 1,0 | 0,3 | 0,5 | 1,3 | 1,3 | 1,0 | 1,0 | 1,5 | 0,3 | 0,9 | 1,2 | 1,0 | 1,0 |
| Clps | 0,9 | 1,1 | 1,0 | 1,2 | 1,0 | 1,0 | 0,7 | 0,8 | 0,9 | 1,0 | 1,3 | 0,8 | 2,3 | 1,0 | 1,4 | 1,0 | 1,0 | 0,1 | 0,3 | 0,7 | 1,0 | 0,7 | 0,6 | 0,7 |
| Clpsl2 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Clu | 1,0 | 0,9 | 1,2 | 1,0 | 1,0 | 1,0 | 1,1 | 1,1 | 1,0 | 1,3 | 0,9 | 0,9 | 1,4 | 0,7 | 1,0 | 1,0 | 0,6 | 0,9 | 0,9 | 1,1 | 1,0 | 1,0 | 1,4 | 1,6 |
| Cmah | 1,0 | 1,0 | 1,0 | 1,3 | 1,0 | 1,0 | 0,6 | 0,9 | 1,0 | 1,0 | 1,0 | 1,0 | 0,9 | 0,9 | 1,0 | 1,0 | 1,0 | 1,0 | 0,9 | 1,1 | 1,0 | 1,0 | 1,0 | 1,0 |
| Cml5 | 1,2 | 0,6 | 1,0 | 1,0 | 0,7 | 0,9 | 1,0 | 0,8 | 1,0 | 1,0 | 1,0 | 1,0 | 1,0 | 1,0 | 1,0 | 1,0 | 1,0 | 1,0 | 1,0 | 1,0 | 0,6 | 0,5 | 1,0 | 1,0 |
| Cmya5 | 1,0 | 1,0 | 1,0 | 0,9 | 1,0 | 0,9 | 0,8 | 0,9 | 1,0 | 1,0 | 1,0 | 1,0 | 2,4 | 1,0 | 4,1 | 0,3 | 1,3 | 1,4 | 1,0 | 1,0 | 1,0 | 1,0 | 1,3 | 12,5 |
| Cnfn | 1,0 | 1,0 | 1,0 | 1,0 | 1,0 | 1,0 | 1,0 | 1,0 | 1,0 | 1,0 | 1,0 | 1,0 | 1,6 | 0,7 | 1,0 | 1,0 | 1,0 | 1,0 | 1,0 | 1,0 | 1,0 | 1,0 | 6,6 | 1,0 |
| Cnn1 | 1,0 | 1,0 | 1,3 | 1,1 | 1,0 | 1,0 | 0,8 | 1,1 | 1,7 | 1,0 | 1,0 | 1,0 | 1,0 | 1,3 | 1,1 | 1,3 | 1,0 | 1,0 | 1,0 | 1,6 | 0,8 | 1,4 | 0,4 | 1,4 |
| Col15a1 | 0,6 | 0,7 | 0,6 | 0,5 | 1,0 | 1,0 | 0,6 | 0,4 | 0,7 | 0,6 | 0,6 | 0,6 | 0,1 | 0,5 | 0,6 | 0,6 | 0,1 | 0,3 | 1,0 | 1,0 | 1,2 | 0,9 | 0,5 | 0,5 |
| Col1a1 | 0,5 | 0,6 | 0,7 | 0,6 | 0,8 | 1,0 | 0,6 | 0,7 | 0,9 | 1,8 | 0,6 | 0,7 | 0,1 | 0,3 | 0,4 | 0,6 | 0,1 | 0,2 | 0,6 | 0,7 | 1,0 | 0,6 | 0,4 | 0,6 |
| Col1a2 | 0,8 | 0,7 | 0,8 | 0,8 | 0,8 | 1,0 | 0,7 | 0,7 | 0,7 | 1,1 | 0,8 | 0,8 | 0,2 | 0,3 | 0,5 | 0,7 | 0,1 | 0,3 | 0,8 | 1,0 | 0,8 | 1,0 | 0,6 | 0,7 |
| Col3a1 | 0,5 | 0,6 | 0,7 | 0,6 | 1,0 | 1,0 | 0,5 | 0,4 | 0,5 | 1,0 | 0,7 | 0,7 | 0,2 | 0,4 | 0,6 | 0,4 | 0,1 | 0,1 | 0,6 | 0,7 | 0,8 | 0,8 | 0,0 | 0,4 |
| Col5a1 | 0,9 | 0,9 | 0,9 | 0,8 | 1,3 | 1,0 | 0,8 | 1,0 | 1,0 | 1,0 | 1,0 | 0,9 | 0,2 | 0,5 | 0,7 | 0,7 | 0,2 | 0,4 | 1,2 | 1,0 | 1,0 | 0,8 | 0,6 | 0,8 |
| Col5a2 | 0,8 | 0,9 | 1,0 | 0,8 | 1,0 | 1,0 | 0,8 | 0,7 | 1,0 | 1,0 | 1,0 | 1,0 | 0,3 | 0,8 | 0,7 | 0,7 | 0,2 | 0,4 | 0,8 | 1,0 | 1,0 | 1,0 | 0,6 | 0,7 |
| Col6a2 | 0,6 | 0,6 | 0,8 | 0,6 | 1,2 | 1,1 | 0,9 | 1,0 | 0,7 | 0,8 | 0,7 | 0,8 | 0,2 | 0,7 | 0,8 | 0,8 | 0,2 | 0,3 | 0,6 | 1,2 | 1,1 | 0,7 | 0,6 | 0,6 |
| Col6a3 | 0,5 | 0,7 | 0,9 | 0,5 | 1,0 | 1,0 | 0,7 | 0,9 | 1,0 | 1,0 | 0,9 | 1,0 | 0,2 | 0,8 | 1,0 | 0,7 | 0,4 | 0,4 | 0,7 | 0,9 | 1,0 | 0,9 | 0,6 | 0,6 |
| Col6a4 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,8 | 1,0 | 1,1 | 0,8 | 1,0 | 1,0 |
| Col6a6 | 1,0 | 1,0 | 1,0 | 1,0 | 1,0 | 1,0 | 1,0 | 1,0 | 1,0 | 1,0 | 1,0 | 1,0 | 0,8 | 0,7 | 1,0 | 1,0 | 0,9 | 1,0 | 1,0 | 1,0 | 1,0 | 1,0 | 1,0 | 1,0 |
| Cox6a2 | 2,3 | 1,0 | 0,8 | 0,9 | 1,0 | 1,3 | 1,1 | 1,1 | 1,0 | 1,0 | 1,0 | 1,0 | 2,8 | 1,0 | 3,5 | 0,5 | 1,3 | 1,3 | 1,6 | 1,1 | 1,0 | 1,0 | 1,4 | 8,3 |
| Cox7a1 | 1,4 | 0,8 | 0,7 | 0,9 | 1,0 | 1,0 | 0,7 | 1,0 | 1,0 | 1,0 | 1,0 | 1,0 | 1,1 | 0,4 | 5,5 | 0,3 | 0,6 | 0,6 | 1,0 | 1,0 | 1,0 | 1,0 | 1,3 | 2,6 |
| Cpa1 | 1,0 | 0,8 | 1,0 | 1,0 | 1,1 | 0,6 | 1,0 | 1,0 | 1,0 | 1,3 | 0,8 | 0,9 | 1,2 | 1,0 | 1,0 | 0,8 | 1,0 | 1,0 | 1,0 | 0,6 | 1,0 | 1,0 | 1,0 | 1,0 |
| Cpa2 | 1,0 | 1,0 | 1,0 | 1,0 | 1,0 | 1,1 | 1,0 | 1,0 | 1,0 | 1,1 | 0,9 | 1,1 | 1,0 | 1,0 | 1,0 | 1,0 | 1,0 | 1,0 | 1,0 | 1,0 | 1,0 | 1,0 | 1,1 | 1,0 |
| Cpb1 | 1,1 | 1,0 | 1,0 | 1,0 | 0,9 | 0,8 | 1,0 | 1,0 | 1,0 | 1,1 | 0,7 | 0,6 | 1,7 | 1,0 | 1,5 | 0,7 | 1,0 | 1,0 | 1,0 | 1,1 | 1,0 | 1,0 | 1,6 | 1,0 |
| Cpz | 1,0 | 1,2 | 0,9 | 0,9 | 1,0 | 1,0 | 0,8 | 0,7 | 1,0 | 1,0 | 0,9 | 0,9 | 0,2 | 0,4 | 0,4 | 0,6 | 1,0 | 1,0 | 1,0 | 1,0 | 1,0 | 1,0 | 1,0 | 1,0 |
| Crb3 | 1,0 | 1,0 | 1,0 | 1,0 | 1,0 | 1,0 | 1,1 | 1,0 | 0,9 | 1,1 | 0,9 | 1,0 | 1,3 | 1,0 | 1,0 | 1,2 | 1,0 | 1,0 | 0,6 | 1,6 | 1,0 | 1,0 | 1,1 | 1,0 |
| Crct1 | 1,0 | 1,0 | 1,0 | 1,0 | 1,0 | 1,0 | 1,0 | 1,0 | 1,0 | 1,0 | 1,0 | 1,0 | 1,4 | 1,1 | 1,0 | 1,0 | 1,0 | 1,0 | 1,0 | 1,0 | 1,0 | 1,0 | 25,7 | 1,0 |
| Crisp1 | 1,0 | 1,0 | 1,0 | 1,0 | 1,0 | 1,0 | 1,0 | 1,0 | 1,0 | 1,0 | 0,9 | 1,0 | 3,8 | 0,4 | 1,0 | 1,0 | 1,0 | 1,0 | 1,0 | 1,0 | 3,9 | 21,6 | 1,0 | 1,0 |
| Crisp4 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Cryaa | 1,0 | 1,0 | 0,8 | 1,1 | 1,0 | 1,0 | 1,2 | 1,0 | 1,0 | 1,0 | 1,0 | 1,0 | 1,0 | 1,0 | 1,0 | 1,0 | 0,8 | 0,2 | 1,3 | 1,0 | 0,9 | 1,0 | 1,0 | 0,6 |
| Cryba2 | 1,1 | 1,0 | 1,0 | 1,0 | 1,0 | 1,0 | 1,0 | 1,0 | 1,0 | 1,0 | 0,8 | 1,5 | 1,0 | 1,0 | 1,0 | 1,0 | 1,0 | 1,0 | 1,0 | 1,0 | 1,0 | 1,0 | 10,4 | 5,3 |
| Cryba4 | 2,2 | 2,3 | 1,0 | 1,0 | 1,0 | 1,0 | 1,0 | 1,0 | 1,0 | 1,0 | 0,5 | 1,0 | 1,3 | 0,5 | 1,0 | 0,7 | 1,0 | 1,0 | 1,1 | 1,0 | 1,0 | 1,0 | 1,0 | 1,0 |
| Crybb3 | 1,0 | 1,0 | 1,0 | 1,0 | 1,0 | 1,0 | 1,0 | 0,5 | 1,0 | 1,0 | 0,9 | 1,1 | 1,0 | 1,0 | 0,9 | 1,0 | 1,0 | 1,0 | 1,0 | 1,0 | 1,0 | 1,0 | 1,0 | 1,3 |
| Crygc | 1,0 | 1,0 | 1,0 | 0,9 | 1,0 | 1,0 | 1,0 | 1,0 | 1,0 | 1,0 | 1,0 | 1,0 | 1,0 | 1,0 | 1,0 | 1,0 | 1,0 | 0,9 | 1,0 | 1,0 | 1,0 | 1,0 | 1,0 | 1,0 |
| Csad | 1,4 | 0,9 | 0,8 | 0,9 | 1,1 | 0,9 | 0,9 | 1,0 | 1,2 | 0,8 | 1,0 | 0,9 | 0,9 | 1,2 | 0,8 | 0,9 | 1,0 | 1,0 | 1,0 | 1,1 | 1,1 | 0,9 | 1,0 | 0,9 |
| Csrp3 | 1,0 | 1,0 | 0,7 | 0,8 | 1,0 | 1,0 | 0,7 | 0,7 | 1,0 | 1,0 | 1,0 | 1,0 | 1,4 | 1,0 | 2,5 | 0,6 | 0,7 | 0,9 | 1,0 | 1,0 | 1,0 | 1,0 | 1,1 | 32,8 |
| Cst11 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Cst12 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,1 | 1,1 | 1,0 | 1,0 |
| Cst6 | 1,0 | 1,0 | 1,0 | 1,0 | 1,0 | 1,2 | 1,0 | 1,0 | 1,0 | 1,0 | 1,0 | 1,0 | 1,4 | 2,0 | 1,0 | 1,0 | 1,1 | 1,0 | 1,0 | 1,0 | 1,0 | 1,0 | 0,6 | 0,6 |
| Cst8 | 4,4 | 5,0 | 1,0 | 1,0 | 1,0 | 1,0 | 2,0 | 0,9 | 1,0 | 1,0 | 1,0 | 1,0 | 1,0 | 1,0 | 1,0 | 1,0 | 1,0 | 1,0 | 1,0 | 1,0 | 1,0 | 1,4 | 1,0 | 1,0 |
| Cthrc1 | 2,1 | 1,6 | 1,5 | 0,9 | 0,8 | 1,1 | 0,9 | 0,8 | 1,0 | 1,0 | 0,9 | 1,4 | 0,2 | 0,8 | 0,5 | 0,6 | 1,0 | 1,0 | 1,0 | 1,0 | 1,0 | 1,0 | 0,7 | 1,1 |
| Scgb1a1 | 0,1 | 0,8 | 1,0 | 1,0 | 1,0 | 1,0 | 1,1 | 1,0 | 1,4 | 1,6 | 0,6 | 0,9 | 5,2 | 1,0 | 5,8 | 1,2 | 1,3 | 1,0 | 1,0 | 0,8 | 1,0 | 1,0 | 1,2 | 1,0 |
| Ctrl | 1,0 | 0,9 | 1,0 | 1,0 | 0,8 | 0,8 | 1,1 | 1,0 | 0,7 | 0,8 | 0,5 | 0,8 | 1,0 | 1,0 | 1,7 | 1,0 | 1,0 | 1,0 | 1,0 | 0,6 | 1,0 | 1,0 | 1,0 | 1,0 |
| Ctxn3 | 0,9 | 0,8 | 1,0 | 1,0 | 1,0 | 1,1 | 1,0 | 1,0 | 1,0 | 1,0 | 0,9 | 0,8 | 3,0 | 1,8 | 2,7 | 0,6 | 2,5 | 1,9 | 1,0 | 1,0 | 1,0 | 1,0 | 2,2 | 4,2 |
| Cux2 | 0,7 | 0,9 | 1,0 | 1,0 | 1,0 | 0,9 | 1,1 | 1,1 | 1,0 | 1,0 | 1,2 | 1,1 | 1,0 | 1,0 | 1,0 | 1,0 | 1,0 | 1,0 | 1,0 | 1,0 | 1,1 | 0,8 | 1,0 | 0,8 |
| Cuzd1 | 1,0 | 1,0 | 1,0 | 1,0 | 1,0 | 1,0 | 1,0 | 1,0 | 1,1 | 1,4 | 1,0 | 1,0 | 1,0 | 1,0 | 1,0 | 1,0 | 1,0 | 1,7 | 1,0 | 1,0 | 1,3 | 4,7 | 1,0 | 1,0 |

[Fig. 23-4]

| | | | | | | | | | | | | | | | | | | | | | | | | |
|---|---|---|---|---|---|---|---|---|---|---|---|---|---|---|---|---|---|---|---|---|---|---|---|---|
| Cxcl1 | 1,0 | 1,0 | 1,0 | 1,0 | 1,0 | 1,0 | 0,9 | 0,7 | 1,0 | 1,0 | 0,8 | 1,0 | 1,0 | 1,0 | 1,0 | 1,0 | 1,0 | 1,0 | 1,0 | 1,0 | 1,0 | 1,0 | 1,0 | 1,0 |
| Cxcl13 | 8,8 | 4,5 | 3,6 | 3,8 | 1,0 | 1,0 | 1,6 | 0,4 | 1,1 | 0,8 | 1,2 | 1,5 | 4,6 | 2,4 | 2,0 | 1,5 | 5,1 | 1,8 | 1,0 | 1,2 | 1,0 | 1,0 | 0,4 | 0,5 |
| Cxcr5 | 1,0 | 1,0 | 1,2 | 1,0 | 1,0 | 1,0 | 0,4 | 0,5 | 1,0 | 1,0 | 1,0 | 1,0 | 1,0 | 1,0 | 1,8 | 2,1 | 1,0 | 1,0 | 0,9 | 0,9 | 1,2 | 0,8 | 1,0 | 1,0 |
| Cyb561 | 0,8 | 0,7 | 1,2 | 1,3 | 1,1 | 1,1 | 1,1 | 1,1 | 1,1 | 1,4 | 0,9 | 0,9 | 0,8 | 1,0 | 1,3 | 1,4 | 0,7 | 1,0 | 1,4 | 1,4 | 1,0 | 1,2 | 0,9 | 0,9 |
| Cym | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Cyp1a1 | 1,0 | 1,0 | 2,1 | 3,9 | 1,0 | 1,0 | 3,1 | 6,0 | 1,0 | 1,0 | 1,0 | 1,0 | 1,0 | 1,8 | 1,0 | 1,0 | 1,0 | 1,2 | 1,0 | 1,0 | 1,0 | 1,0 | 1,4 | 2,1 |
| Cyp26b1 | 2,1 | 1,8 | 1,7 | 1,1 | 1,0 | 1,0 | 3,5 | 2,0 | 1,0 | 1,0 | 2,7 | 2,9 | 1,3 | 2,2 | 1,6 | 1,7 | 1,2 | 1,5 | 1,9 | 3,0 | 1,1 | 0,9 | 2,7 | 1,0 |
| Cyp27b1 | 1,0 | 1,0 | 1,0 | 1,0 | 1,0 | 1,0 | 1,0 | 1,0 | 1,0 | 1,0 | 1,0 | 1,0 | 0,4 | 0,5 | 1,0 | 1,0 | 1,0 | 1,0 | 1,0 | 1,0 | 1,0 | 1,0 | 1,6 | 0,7 |
| Cyp2a4 | 1,0 | 1,0 | 1,0 | 1,0 | 1,0 | 1,0 | 1,0 | 2,1 | 1,0 | 1,0 | 1,0 | 1,0 | 1,0 | 1,0 | 1,0 | 1,0 | 1,0 | 1,0 | 1,0 | 1,0 | 1,0 | 1,0 | 0,3 | 1,0 |
| Cyp2a5 | 1,0 | 1,0 | 1,0 | 1,0 | 1,0 | 1,0 | 0,8 | 1,6 | 1,0 | 1,0 | 1,5 | 2,4 | 1,0 | 1,0 | 1,0 | 1,0 | 1,0 | 1,0 | 1,0 | 1,0 | 1,0 | 1,0 | 0,0 | 6,9 |
| Cyp2b9 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Cyp2c38 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Cyp2c54 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Cyp2d12 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Cyp2d40 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Cyp2d9 | 1,0 | 1,0 | 1,0 | 0,4 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Cyp2e1 | 2,8 | 2,5 | 1,8 | 2,0 | 1,0 | 1,0 | 0,9 | 0,3 | 0,6 | 0,6 | 0,9 | 1,0 | 1,4 | 3,1 | 1,2 | 3,2 | 2,0 | 1,4 | 5,0 | 1,5 | 1,2 | 1,0 | 1,6 | 1,9 |
| Cyp2f2 | 5,3 | 2,5 | 2,7 | 1,7 | 1,0 | 1,0 | 1,1 | 1,0 | 1,0 | 1,0 | 1,3 | 1,4 | 1,4 | 1,5 | 0,8 | 1,2 | 1,3 | 1,9 | 1,0 | 1,0 | 1,0 | 1,0 | 0,4 | 1,9 |
| Cyp2u1 | 1,0 | 0,7 | 0,8 | 0,9 | 0,8 | 0,8 | 1,0 | 1,0 | 1,0 | 1,0 | 1,2 | 0,8 | 1,0 | 1,0 | 1,0 | 0,9 | 0,9 | 0,9 | 1,1 | 1,1 | 1,2 | 1,1 | 0,9 | 0,8 |
| Cyp39a1 | 0,9 | 0,9 | 0,8 | 0,9 | 1,2 | 0,8 | 1,0 | 0,9 | 1,2 | 1,5 | 1,1 | 1,0 | 1,0 | 1,0 | 0,7 | 0,9 | 0,8 | 1,1 | 0,6 | 0,8 | 1,2 | 1,3 | 0,9 | 1,2 |
| Cyp3a11 | 1,0 | 1,0 | 1,0 | 0,2 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Cyp3a44 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Cyp3a59 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Cyp4a12a | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Cyp4a12b | 1,0 | 1,0 | 1,0 | 1,0 | 1,0 | 1,0 | 1,0 | 0,7 | 1,0 | 1,0 | 1,0 | 1,0 | 1,0 | 1,0 | 1,0 | 1,0 | 1,0 | 1,0 | 1,0 | 1,0 | 1,0 | 1,0 | 0,4 | 1,0 |
| Cyp4a14 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Cyp4b1-ps2 | 1,0 | 1,0 | 1,0 | 1,0 | 1,0 | 1,0 | 1,0 | 0,7 | 1,0 | 1,0 | 1,0 | 1,0 | 1,0 | 1,0 | 1,2 | 2,2 | 1,0 | 1,0 | 1,5 | 0,4 | 1,1 | 1,0 | 1,0 | 1,0 |
| Cyp7b1 | 1,1 | 0,7 | 1,0 | 1,0 | 1,0 | 1,0 | 1,1 | 0,8 | 1,0 | 1,0 | 1,1 | 1,0 | 0,5 | 1,1 | 0,6 | 1,1 | 1,0 | 1,0 | 1,1 | 1,7 | 1,0 | 1,0 | 0,7 | 0,7 |
| Cyp8b1 | 1,0 | 1,0 | 1,0 | 1,0 | 1,0 | 1,0 | 1,1 | 1,2 | 1,0 | 1,0 | 1,0 | 1,0 | 0,8 | 1,0 | 1,0 | 1,0 | 1,0 | 1,0 | 0,9 | 1,3 | 1,0 | 1,0 | 1,0 | 1,0 |
| Cystm1 | 0,9 | 1,7 | 0,4 | 0,3 | 1,2 | 1,1 | 1,3 | 1,4 | 1,5 | 1,3 | 1,0 | 0,8 | 2,2 | 1,0 | 1,8 | 0,5 | 1,1 | 1,2 | 1,3 | 2,0 | 1,0 | 0,9 | 0,8 | 1,1 |
| Dbil5 | 1,0 | 1,0 | 1,0 | 1,2 | 1,4 | 0,7 | 1,0 | 1,0 | 1,0 | 1,0 | 1,0 | 0,8 | 1,0 | 0,7 | 1,0 | 1,3 | 1,0 | 1,0 | 1,0 | 1,0 | 1,1 | 1,2 | 1,8 | 1,0 |
| Dcpp2 | 1,0 | 1,0 | 1,0 | 0,7 | 1,0 | 1,0 | 1,0 | 0,7 | 0,5 | 1,0 | 1,2 | 1,0 | 1,0 | 1,0 | 1,0 | 1,0 | 1,0 | 0,5 | 1,0 | 1,0 | 1,0 | 1,0 | 0,2 | 0,2 |
| Dcpp3 | 1,0 | 1,0 | 1,0 | 1,1 | 1,0 | 1,0 | 1,0 | 0,6 | 0,6 | 1,0 | 0,7 | 1,0 | 1,0 | 1,0 | 1,0 | 1,0 | 1,0 | 0,5 | 1,0 | 1,0 | 1,0 | 1,0 | 0,1 | 0,1 |
| Dcstamp | 1,0 | 1,0 | 1,0 | -1,0 | 1,0 | 1,0 | 1,0 | 1,0 | 1,0 | 1,0 | 1,0 | 1,0 | 1,0 | 1,0 | 3,4 | 6,3 | 1,0 | 1,0 | 1,0 | 1,0 | 1,0 | 1,0 | 1,0 | 1,0 |
| Dct | 1,0 | 1,0 | 1,0 | 1,0 | 1,0 | 1,0 | 1,0 | 1,0 | 1,0 | 1,0 | 1,0 | 1,0 | 0,2 | 0,4 | 1,0 | 1,0 | 1,0 | 1,0 | 1,0 | 1,0 | 1,1 | 0,9 | 1,0 | 1,0 |
| Scgb1b27 | 0,1 | 0,8 | 1,7 | 0,6 | 0,8 | 0,9 | 1,0 | 1,7 | 1,2 | 1,0 | 1,1 | 1,0 | 0,8 | 1,1 | 1,5 | 0,7 | 1,3 | 1,9 | 0,9 | 0,9 | 0,8 | 1,0 | 0,9 | 1,0 |
| Ddit4l | 1,0 | 1,0 | 1,0 | 1,0 | 1,1 | 1,0 | 0,8 | 1,0 | 1,0 | 1,0 | 1,5 | 1,0 | 1,7 | 0,8 | 1,9 | 0,5 | 1,1 | 1,0 | 1,0 | 1,0 | 1,0 | 1,0 | 0,8 | 3,8 |
| Defa24 | 1,0 | 1,0 | 1,0 | 1,0 | 1,0 | 1,0 | 1,0 | 1,0 | 1,0 | 1,0 | 1,0 | 1,0 | 1,2 | 0,8 | 0,2 | 1,0 | 1,0 | 5,3 | 1,0 | 1,0 | 1,0 | 4,5 | 1,0 | 1,0 |
| Defb1 | 1,0 | 1,0 | 1,0 | 1,0 | 1,0 | 1,0 | 1,0 | 1,0 | 1,0 | 1,0 | 1,0 | 1,0 | 1,4 | 1,4 | 1,0 | 1,0 | 1,0 | 1,0 | 1,0 | 1,0 | 1,0 | 1,0 | 1,0 | 1,0 |
| Defb10 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Defb12 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Defb14 | 1,0 | 1,0 | 1,0 | 1,0 | 1,0 | 1,0 | 1,0 | 1,0 | 1,0 | 1,0 | 1,0 | 1,0 | 2,9 | 5,3 | 1,0 | 1,0 | 1,0 | 1,0 | 1,0 | 1,0 | 1,6 | 2,8 | 1,0 | 1,0 |
| Defb15 | 1,0 | 1,0 | 1,0 | 1,0 | 1,0 | 1,0 | 1,0 | 1,0 | 1,0 | 1,0 | 1,0 | 1,0 | 1,0 | 0,6 | 1,0 | 1,0 | 1,0 | 1,0 | 1,0 | 1,0 | 1,8 | 1,9 | 1,0 | 1,0 |
| Defb18 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Defb19 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,9 | 1,0 | 1,0 |
| | AG | | Aorta | | Brain | | Lung | | Pancreas | | PG | | Skin | | Skull | | SM | | Spleen | | Testis | | TG | |
| | E | M | E | M | E | M | E | M | E | M | E | M | E | M | E | M | E | M | E | M | E | M | E | M |
| Defb2 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Defb20 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Defb21 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,4 | 1,2 | 1,0 | 1,0 |
| Defb22 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,1 | 12,0 | 1,0 | 1,0 |
| Defb23 | 1,0 | 1,0 | 1,0 | 1,0 | 1,0 | 1,0 | 0,6 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Defb25 | 1,0 | 0,6 | 1,0 | 1,0 | 1,0 | 1,0 | 1,0 | 1,0 | 1,0 | 1,0 | 1,0 | 0,8 | 1,0 | 1,0 | 1,0 | 1,0 | 1,0 | 1,0 | 1,0 | 1,0 | 1,0 | 1,0 | 1,0 | 1,0 |
| Defb26 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,9 | 1,0 | 1,0 |
| Defb28 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 2,0 | 7,3 | 1,0 | 1,0 |
| Defb29 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Defb30 | 1,0 | 1,0 | 1,0 | -1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,3 | 1,0 | 1,0 |
| Defb35 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Defb37 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,3 | 5,2 | 1,0 | 1,0 |
| Defb39 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,4 | 1,0 | 1,0 |
| Defb4 | 1,0 | 1,0 | 1,0 | 1,0 | 1,0 | 1,0 | 1,0 | 1,0 | 1,0 | 1,0 | 1,0 | 1,0 | 1,0 | 3,8 | 1,0 | 1,0 | 1,0 | 1,0 | 1,0 | 1,0 | 1,0 | 1,0 | 13,4 | 1,0 |
| Defb41 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,3 | 1,0 | 1,0 |
| Defb42 | 1,0 | 1,0 | 0,3 | 0,6 | 1,0 | 1,0 | 1,0 | 1,0 | 1,0 | 1,0 | 1,0 | 1,0 | 1,0 | 1,0 | 1,0 | 1,0 | 1,0 | 1,0 | 0,8 | 0,8 | 1,0 | 1,2 | 0,8 | 0,7 |
| Defb43 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,2 | 3,2 | 1,0 | 1,0 |
| Defb45 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,6 | 1,1 | 1,0 | 1,0 |
| Defb47 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Defb48 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Defb8 | 1,0 | 1,0 | 1,0 | 1,0 | 1,0 | 1,0 | 1,0 | 1,0 | 1,0 | 1,0 | 1,0 | 1,0 | 0,2 | 0,0 | 1,0 | 1,0 | 1,0 | 1,0 | 1,0 | 1,0 | 1,0 | 1,0 | 1,0 | 1,0 |
| Depdc7 | 1,0 | 1,0 | 1,0 | 1,0 | 1,0 | 0,9 | 1,0 | 1,0 | 1,2 | 1,0 | 0,9 | 1,7 | 1,6 | 1,0 | 0,8 | 0,8 | 1,0 | 1,0 | 1,2 | 1,7 | 1,0 | 1,0 | 1,0 | 1,0 |
| Derl3 | 1,3 | 1,0 | 1,0 | 1,0 | 0,9 | 1,0 | 0,8 | 0,8 | 1,2 | 1,4 | 0,9 | 0,7 | 1,0 | 0,6 | 0,9 | 0,9 | 1,0 | 1,0 | 1,1 | 0,7 | 1,1 | 1,0 | 0,8 | 0,7 |
| Des | 1,0 | 0,9 | 0,9 | 1,0 | 1,0 | 0,9 | 0,9 | 0,9 | 1,5 | 2,3 | 0,9 | 0,8 | 2,8 | 0,9 | 4,6 | 0,4 | 1,4 | 1,2 | 0,9 | 1,0 | 1,0 | 0,8 | 1,5 | 15,1 |
| Dhrs7c | 1,0 | 1,0 | 1,0 | 1,1 | 1,0 | 1,0 | 0,9 | 0,4 | 1,0 | 1,0 | 1,0 | 1,0 | 2,4 | 1,3 | 6,8 | 0,5 | 1,3 | 1,4 | 1,0 | 1,0 | 1,0 | 1,0 | 1,4 | 10,8 |
| Dio1 | 1,0 | 1,0 | 1,0 | 1,0 | 1,0 | 1,0 | 1,0 | 1,0 | 0,4 | 0,6 | 1,0 | 1,3 | 1,0 | 1,0 | 1,0 | 1,0 | 1,0 | 1,0 | 1,0 | 1,0 | 1,6 | 1,3 | 0,7 | 0,6 |
| Dmbt1 | 1,0 | 1,0 | 1,0 | 1,0 | 1,0 | 1,0 | 1,0 | 1,0 | 2,0 | 1,3 | 1,0 | 1,0 | 1,0 | 1,0 | 1,0 | 1,0 | 1,0 | 1,0 | 1,0 | 1,0 | 1,0 | 1,0 | 0,0 | 0,0 |
| Dmkn | 1,2 | 0,7 | 1,0 | 1,1 | 1,2 | 0,8 | 1,2 | 0,8 | 1,0 | 1,0 | 1,0 | 1,0 | 1,3 | 1,5 | 0,7 | 1,1 | 1,0 | 1,0 | 1,0 | 1,0 | 1,2 | 0,8 | 10,0 | 1,0 |
| Dmrtc1a | 1,0 | 1,0 | 1,0 | 1,0 | 0,7 | 1,3 | 1,0 | 1,0 | 1,0 | 1,0 | 1,0 | 0,9 | 1,0 | 1,0 | 1,0 | 1,0 | 1,0 | 1,0 | 1,0 | 1,0 | 1,0 | 1,3 | 3,3 | 4,6 |
| Dmtn | 1,0 | 0,9 | 0,9 | 0,7 | 1,0 | 1,0 | 0,9 | 0,9 | 1,0 | 1,0 | 1,0 | 1,0 | 0,7 | 1,0 | 1,4 | 1,4 | 1,0 | 0,8 | 7,0 | 0,4 | 1,0 | 1,0 | 0,8 | 0,6 |
| Dnajb8 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,1 | 1,0 | 1,0 | 1,0 |
| Dnase1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Dnmt3l | 2,3 | 5,7 | 1,0 | -1,0 | 1,0 | 1,0 | 1,0 | 1,0 | 1,0 | 1,0 | 1,0 | 1,0 | 0,8 | 1,1 | 1,0 | 1,0 | 1,0 | 1,0 | 1,0 | 1,0 | 1,0 | 0,9 | 1,0 | 1,0 |
| Dntt | 1,0 | 1,0 | 1,0 | 1,0 | 1,0 | 1,0 | 1,0 | 1,0 | 1,0 | 1,0 | 1,0 | 1,0 | 1,0 | 1,0 | 0,6 | 1,1 | 1,0 | 1,0 | 0,7 | 1,4 | 1,0 | 1,0 | 0,0 | 1,0 |
| Dopey2 | 0,7 | 0,7 | 1,2 | 0,9 | 0,9 | 0,8 | 1,0 | 1,2 | 1,0 | 1,0 | 1,0 | 0,9 | 1,2 | 1,0 | 1,0 | 0,8 | 0,9 | 1,3 | 1,2 | 1,0 | 1,1 | 0,8 | 9,7 | 4,4 |
| Drd4 | 0,5 | 0,3 | 1,0 | 1,0 | 1,0 | 1,0 | 1,0 | 1,0 | 1,0 | 1,0 | 0,7 | 0,5 | 1,0 | 1,0 | 1,0 | 1,0 | 1,0 | 1,0 | 1,0 | 1,0 | 1,0 | 0,8 | 1,0 | 1,0 |
| Duoxa2 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,8 | 0,6 |

[Fig. 23-5]

| | | | | | | | | | | | | | | | | | | | | | | | | |
|---|---|---|---|---|---|---|---|---|---|---|---|---|---|---|---|---|---|---|---|---|---|---|---|---|
| Dupd1 | 0,9 | 1,0 | 1,0 | 1,0 | 1,0 | 1,0 | 1,0 | 1,0 | 1,0 | 1,0 | 1,0 | 1,0 | 0,6 | 0,2 | 3,5 | 0,1 | 0,2 | 0,3 | 1,0 | 1,0 | 1,0 | 1,0 | 0,3 | 2,5 |
| Dusp10 | 1,0 | 0,9 | 1,5 | 0,8 | 1,0 | 1,1 | 1,0 | 0,8 | 1,0 | 1,0 | 1,0 | 0,8 | 1,2 | 0,7 | 1,3 | 0,5 | 0,7 | 0,5 | 1,0 | 0,8 | 1,0 | 1,0 | 0,8 | 2,1 |
| Dusp13 | 1,0 | 1,0 | 1,0 | 1,0 | 1,0 | 1,0 | 1,0 | 1,0 | 1,0 | 1,0 | 1,0 | 1,0 | 2,1 | 1,0 | 6,5 | 0,2 | 1,3 | 1,2 | 1,0 | 1,0 | 1,1 | 0,9 | 0,7 | 8,6 |
| Dusp15 | 1,0 | 1,0 | 1,0 | 1,0 | 1,0 | 0,9 | 1,0 | 1,0 | 1,0 | 1,0 | 1,0 | 1,0 | 1,8 | 1,0 | 1,0 | 1,0 | 1,0 | 1,0 | 1,0 | 1,0 | 1,0 | 0,8 | 1,0 | 1,0 |
| Dusp2 | 1,0 | 0,8 | 1,0 | 1,3 | 1,0 | 1,0 | 0,7 | 0,8 | 1,0 | 1,0 | 1,5 | 2,4 | 1,1 | 0,7 | 0,7 | 1,0 | 1,1 | 1,0 | 1,0 | 1,2 | 1,2 | 1,0 | 1,1 | 0,6 |
| Dusp5 | 0,9 | 1,7 | 1,6 | 1,0 | 0,9 | 0,9 | 1,0 | 1,1 | 1,0 | 1,0 | 0,8 | 1,1 | 1,7 | 0,7 | 1,0 | 0,9 | 1,0 | 1,0 | 0,8 | 0,9 | 1,1 | 0,8 | 1,4 | 1,0 |
| Ear3 | 1,0 | 1,0 | 1,0 | 1,5 | 1,0 | 1,0 | 1,0 | 1,0 | 1,0 | 1,0 | 1,0 | 1,0 | 1,0 | 1,0 | 10,5 | 1,0 | 1,1 | 0,9 | 1,0 | 1,0 | 1,0 | 1,0 | 1,0 | 1,0 |
| Eddm3b | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,8 | 0,5 |
| Eef1a2 | 0,7 | 0,7 | 0,7 | 0,7 | 1,0 | 1,0 | 0,8 | 0,7 | 1,0 | 1,0 | 0,9 | 0,9 | 2,2 | 0,9 | 4,1 | 0,3 | 1,0 | 1,2 | 1,0 | 1,0 | 1,0 | 1,0 | 1,1 | 6,3 |
| Egfr | 0,7 | 0,8 | 1,1 | 0,9 | 0,9 | 0,8 | 1,1 | 1,2 | 1,3 | 0,9 | 1,3 | 1,2 | 0,8 | 0,9 | 0,7 | 0,7 | 0,8 | 0,9 | 1,3 | 1,0 | 1,0 | 1,0 | 0,7 | 0,6 |
| Egr2 | 1,0 | 1,2 | 1,0 | 1,6 | 1,2 | 1,0 | 1,1 | 1,2 | 1,0 | 1,0 | 0,9 | 0,6 | 1,1 | 1,2 | 1,1 | 1,0 | 1,0 | 1,0 | 0,8 | 0,6 | 1,0 | 1,0 | 1,0 | 1,0 |
| Eif3j1 | 1,0 | 15,2 | 1,0 | 0,1 | 1,0 | 1,0 | 15,3 | 1,0 | 1,0 | 1,0 | 14,5 | 1,0 | 1,0 | 0,1 | 1,0 | 1,0 | 0,2 | 1,0 | 1,0 | 1,0 | 0,0 | 0,1 | 1,0 | 1,0 |
| Eif3j2 | 1,4 | 0,5 | 1,0 | 3,1 | 1,1 | 1,1 | 0,3 | 0,9 | 1,1 | 1,0 | 0,1 | 1,0 | 1,3 | 1,7 | 1,3 | 1,0 | 2,1 | 1,3 | 1,2 | 0,8 | 7,4 | 17,3 | 1,1 | 1,4 |
| Elk4 | 0,6 | 0,8 | 1,3 | 1,2 | 1,0 | 0,9 | 0,9 | 1,5 | 1,0 | 1,0 | 1,0 | 1,2 | 0,7 | 1,2 | 1,3 | 0,6 | 1,2 | 1,2 | 0,9 | 1,1 | 0,7 | 0,9 | 1,0 | 0,7 |
| Ell3 | 1,1 | 1,1 | 1,0 | 1,0 | 0,7 | 1,1 | 1,1 | 1,0 | 1,0 | 1,0 | 0,7 | 0,8 | 1,1 | 1,5 | 1,2 | 1,3 | 1,0 | 1,0 | 1,1 | 1,1 | 0,9 | 1,2 | 0,8 | 0,8 |
| Elovl2 | 1,0 | 1,0 | 1,0 | 1,0 | 0,9 | 1,0 | 1,0 | 1,0 | 1,0 | 1,0 | 1,1 | 1,4 | 1,0 | 1,0 | 1,0 | 1,0 | 1,0 | 1,0 | 1,0 | 1,0 | 0,9 | 1,0 | 0,7 | 0,5 |
| Elovl3 | 0,4 | 0,5 | 0,0 | 0,3 | 1,0 | 1,0 | 1,0 | 1,0 | 1,0 | 1,0 | 1,0 | 1,0 | 0,7 | 1,8 | 1,0 | 1,0 | 1,0 | 0,6 | 1,0 | 1,0 | 1,0 | 1,0 | 1,1 | 2,5 |
| Elovl4 | 1,0 | 1,0 | 1,0 | 1,0 | 1,0 | 1,1 | 1,0 | 1,0 | 1,0 | 1,0 | 1,2 | 1,6 | 0,9 | 2,1 | 1,2 | 0,7 | 1,0 | 1,0 | 1,0 | 1,0 | 1,0 | 1,0 | 1,3 | 1,0 |
| Emid1 | 1,9 | 1,1 | 0,9 | 0,8 | 0,9 | 1,1 | 0,6 | 0,6 | 1,0 | 1,0 | 1,0 | 1,0 | 0,4 | 1,0 | 0,5 | 0,8 | 0,9 | 0,9 | 0,9 | 1,0 | 1,0 | 1,0 | 0,5 | 0,6 |
| Emp1 | 0,7 | 0,8 | 1,1 | 1,2 | 0,9 | 1,1 | 0,9 | 0,9 | 1,0 | 1,2 | 0,8 | 0,9 | 1,1 | 0,8 | 1,0 | 1,1 | 0,6 | 0,6 | 0,9 | 1,4 | 1,1 | 1,2 | 0,8 | 0,8 |
| Emp2 | 1,0 | 0,9 | 1,1 | 0,9 | 0,7 | 0,7 | 1,1 | 1,2 | 0,9 | 0,9 | 1,0 | 1,1 | 1,0 | 1,0 | 0,8 | 0,9 | 0,9 | 1,1 | 0,8 | 1,0 | 1,2 | 1,2 | 0,9 | 0,8 |
| Emx2 | 1,0 | 1,0 | 1,0 | 1,0 | 0,9 | 1,0 | 1,0 | 1,0 | 1,0 | 1,0 | 1,0 | 1,0 | 1,1 | 1,1 | 1,0 | 1,0 | 1,0 | 1,0 | 1,0 | 1,0 | 0,9 | 1,3 | 1,0 | 1,0 |
| Eno3 | 1,4 | 1,3 | 0,9 | 1,0 | 1,4 | 1,1 | 0,8 | 0,8 | 1,0 | 1,2 | 1,3 | 1,3 | 1,9 | 0,9 | 3,2 | 0,3 | 0,9 | 0,9 | 1,1 | 1,1 | 1,2 | 1,1 | 1,1 | 5,8 |
| Enpp1 | 1,0 | 1,0 | 1,1 | 1,1 | 1,0 | 1,0 | 1,0 | 0,9 | 1,0 | 1,0 | 1,0 | 1,0 | 1,1 | 1,1 | 1,2 | 1,0 | 0,9 | 1,0 | 1,0 | 1,2 | 1,0 | 1,0 | 0,7 | 1,1 |
| Epb4.1l4b | 1,5 | 1,5 | 1,5 | 1,1 | 1,0 | 1,0 | 1,2 | 1,3 | 1,0 | 1,3 | 1,0 | 1,2 | 1,1 | 1,0 | 0,9 | 1,3 | 1,2 | 1,1 | 1,0 | 1,0 | 0,8 | 1,5 | 0,9 | 0,8 |
| Epb4.2 | 1,0 | 1,0 | 1,0 | 1,0 | 1,0 | 1,0 | 1,0 | 1,0 | 1,0 | 1,0 | 1,0 | 1,0 | 1,0 | 1,0 | 1,6 | 1,3 | 1,0 | 1,0 | 7,0 | 0,4 | 1,0 | 1,0 | 1,0 | 0,9 |
| Epcam | 1,0 | 1,0 | 1,0 | 1,0 | 1,0 | 1,0 | 1,1 | 0,9 | 0,9 | 1,5 | 1,0 | 1,1 | 1,1 | 1,2 | 1,1 | 1,7 | 1,0 | 1,0 | 1,0 | 0,8 | 0,9 | 0,9 | 1,0 | 1,1 |
| Eppin | 1,0 | 1,0 | 1,0 | 1,0 | 1,0 | 1,0 | 2,1 | 1,4 | 1,0 | 1,0 | 1,0 | 1,0 | 1,0 | 1,0 | 1,0 | 1,0 | 1,0 | 1,0 | 1,0 | 1,0 | 0,9 | 1,0 | 1,0 | 1,0 |
| Ermap | 1,0 | 1,0 | 1,0 | 1,0 | 1,0 | 1,0 | 1,0 | 1,0 | 1,0 | 1,0 | 1,0 | 1,0 | 1,0 | 1,0 | 1,5 | 1,3 | 1,0 | 1,0 | 6,7 | 0,3 | 1,0 | 1,0 | 1,0 | 1,0 |
| Esco2 | 1,0 | 1,0 | 1,0 | -1,0 | 1,0 | 1,0 | 1,0 | 1,0 | 1,0 | 1,0 | 1,0 | 1,0 | 0,2 | 0,6 | 1,2 | 1,2 | 1,0 | 1,0 | 1,5 | 0,3 | 0,8 | 1,4 | 1,0 | 1,0 |
| Esrp1 | 1,0 | 1,0 | 1,0 | 1,0 | 1,0 | 1,0 | 1,2 | 1,1 | 1,1 | 1,1 | 0,9 | 1,0 | 1,0 | 1,0 | 1,0 | 1,0 | 1,0 | 1,0 | 1,0 | 1,0 | 0,8 | 1,1 | 1,0 | 1,0 |
| Esrp2 | 0,9 | 1,1 | 1,5 | 1,2 | 1,0 | 1,0 | 1,3 | 1,3 | 1,0 | 0,9 | 1,5 | 0,7 | 0,9 | 0,9 | 1,0 | 1,0 | 1,0 | 1,0 | 1,0 | 1,0 | 1,0 | 1,0 | 0,8 | 0,5 |
| Etv4 | 1,0 | 1,0 | 1,0 | 1,0 | 0,9 | 1,0 | 1,0 | 1,0 | 1,0 | 1,0 | 1,0 | 1,0 | 0,7 | 1,0 | 1,1 | 1,0 | 0,8 | 0,7 | 1,0 | 1,0 | 1,0 | 1,0 | 3,7 | 4,8 |
| Etv5 | 0,9 | 1,1 | 1,1 | 1,1 | 1,0 | 1,0 | 0,9 | 1,1 | 2,8 | 2,3 | 1,0 | 1,3 | 1,1 | 1,1 | 1,0 | 1,0 | 0,9 | 1,3 | 1,0 | 1,2 | 1,0 | 1,0 | 1,1 | 1,7 |
| F11 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,1 | 1,1 | 1,0 | 1,0 |
| Faah | 0,8 | 0,7 | 1,0 | 1,0 | 1,1 | 1,1 | 1,0 | 1,0 | 1,1 | 1,1 | 1,1 | 1,0 | 1,1 | 1,4 | 1,2 | 1,5 | 1,0 | 1,0 | 1,1 | 1,1 | 0,9 | 0,8 | 1,0 | 0,7 |
| Fabp1 | 1,0 | 0,9 | 1,0 | 0,2 | 1,0 | 1,0 | 0,6 | 0,9 | 0,9 | 1,6 | 1,0 | 1,0 | 1,0 | 1,6 | 1,0 | 1,0 | 1,0 | 1,0 | 0,5 | 2,2 | 1,1 | 1,8 | 1,0 | 1,0 |
| Fabp4 | 1,3 | 1,4 | 0,7 | 1,2 | 1,0 | 1,0 | 0,8 | 0,2 | 0,8 | 0,8 | 0,6 | 0,2 | 1,0 | 1,3 | 1,2 | 1,9 | 0,8 | 0,5 | 1,2 | 1,3 | 1,2 | 3,3 | 1,4 | 1,6 |
| Fabp5 | 1,1 | 0,8 | 0,6 | 0,4 | 0,9 | 0,7 | 1,2 | 0,9 | 2,9 | 2,7 | 1,0 | 0,9 | 0,9 | 1,0 | 1,0 | 1,0 | 0,5 | 0,5 | 1,2 | 0,8 | 0,8 | 0,7 | 1,5 | 1,0 |
| Fads2 | 0,8 | 1,6 | 1,3 | 0,6 | 0,9 | 1,0 | 0,8 | 1,0 | 0,4 | 0,8 | 1,1 | 1,0 | 0,5 | 0,8 | 0,9 | 0,7 | 0,8 | 1,0 | 1,1 | 1,1 | 0,6 | 0,5 | 1,2 | 0,5 |
| Faim2 | 0,6 | 0,6 | 1,3 | 0,9 | 1,0 | 1,0 | 0,9 | 1,0 | 1,0 | 1,0 | 1,0 | 1,0 | 1,0 | 1,0 | 0,8 | 0,5 | 1,0 | 1,0 | 1,0 | 1,0 | 1,0 | 1,0 | 0,9 | 1,2 |
| Faim3 | 1,0 | 1,0 | 1,0 | 1,0 | 1,0 | 1,0 | 0,4 | 0,6 | 1,0 | 1,0 | 1,0 | 1,0 | 1,0 | 1,0 | 1,5 | 2,1 | 1,0 | 1,0 | 0,8 | 1,0 | 0,9 | 0,6 | 1,0 | 1,0 |
| Fam150b | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Fam198a | 1,0 | 1,0 | 0,8 | 1,0 | 0,7 | 0,8 | 0,9 | 1,0 | 1,0 | 1,0 | 1,0 | 1,0 | 1,1 | 0,9 | 0,8 | 0,7 | 1,0 | 1,0 | 1,0 | 1,0 | 1,0 | 1,0 | 1,0 | 1,0 |
| Fam20a | 0,9 | 0,9 | 1,2 | 1,0 | 1,0 | 0,9 | 0,8 | 0,9 | 1,0 | 1,0 | 1,6 | 1,8 | 1,1 | 0,9 | 0,8 | 1,1 | 1,0 | 0,9 | 0,8 | 1,2 | 1,2 | 1,0 | 9,5 | 5,9 |
| Fam20c | 0,8 | 0,8 | 1,4 | 0,8 | 1,0 | 1,0 | 1,0 | 1,0 | 0,9 | 0,7 | 0,9 | 0,8 | 0,9 | 0,9 | 1,2 | 1,4 | 0,7 | 1,0 | 0,8 | 1,1 | 1,0 | 1,0 | 3,9 | 3,3 |
| Fam25c | 1,0 | 1,0 | 1,0 | 1,0 | 1,0 | 1,0 | 1,0 | 1,0 | 1,0 | 1,0 | 1,0 | 1,0 | 1,1 | 0,9 | 1,0 | 1,0 | 1,0 | 1,0 | 1,0 | 1,0 | 1,0 | 1,0 | 5,7 | 1,0 |
| Fam57b | 0,8 | 0,7 | 0,3 | 0,2 | 1,0 | 1,0 | 1,0 | 0,9 | 1,0 | 1,0 | 1,1 | 1,0 | 1,3 | 1,4 | 1,5 | 1,3 | 0,9 | 0,8 | 1,0 | 1,0 | 0,9 | 0,9 | 0,9 | 1,1 |
| Fam64a | 0,8 | 1,7 | 1,0 | 1,0 | 1,0 | 1,0 | 1,0 | 1,0 | 1,0 | 1,0 | 1,0 | 1,0 | 0,2 | 0,8 | 0,7 | 0,8 | 1,0 | 1,0 | 0,5 | 0,6 | 1,0 | 1,0 | 1,0 | 1,0 |
| Fam84a | 0,9 | 0,9 | 0,6 | 0,9 | 1,0 | 0,9 | 1,0 | 1,1 | 1,0 | 1,0 | 0,9 | 0,8 | 0,8 | 0,9 | 0,7 | 1,0 | 1,0 | 1,0 | 0,8 | 1,1 | 1,0 | 0,9 | 0,7 | 0,6 |
| Fbxo40 | 1,0 | 1,0 | 1,6 | 0,8 | 1,0 | 1,0 | 1,0 | 1,0 | 1,0 | 1,0 | 1,0 | 1,0 | 1,9 | 0,9 | 3,3 | 0,2 | 1,0 | 1,3 | 1,0 | 1,0 | 1,0 | 1,0 | 1,4 | 8,4 |
| Fcamr | 1,0 | 1,0 | 1,0 | 1,0 | 1,0 | 1,0 | 1,6 | 1,1 | 1,0 | 1,0 | 1,0 | 1,0 | 0,8 | 1,2 | 1,0 | 1,0 | 1,0 | 1,0 | 0,8 | 0,7 | 1,0 | 1,0 | 1,0 | 1,0 |
| Fcer2a | 1,0 | 1,0 | 1,0 | -1,0 | 1,0 | 1,0 | 0,4 | 0,6 | 1,0 | 1,0 | 1,0 | 1,0 | 0,8 | 1,0 | 1,5 | 2,3 | 1,0 | 1,0 | 0,8 | 0,9 | 1,0 | 1,0 | 1,0 | 1,0 |
| Fcgbp | 1,0 | 1,0 | 1,0 | 1,0 | 1,0 | 1,0 | 0,8 | 1,0 | 1,0 | 1,0 | 1,0 | 1,0 | 1,3 | 1,3 | 1,0 | 1,0 | 1,0 | 1,0 | 1,0 | 1,0 | 1,0 | 1,1 | 0,8 | 0,9 |
| Fcna | 2,1 | 2,3 | 1,5 | 2,0 | 1,0 | 1,0 | 1,5 | 0,2 | 1,4 | 1,0 | 1,0 | 1,0 | 1,0 | 1,3 | 1,4 | 2,6 | 1,3 | 0,7 | 1,3 | 1,3 | 1,0 | 0,9 | 1,6 | 2,8 |
| Fcrla | 1,0 | 1,0 | 1,0 | 1,0 | 1,0 | 1,0 | 0,4 | 0,3 | 1,0 | 1,0 | 1,0 | 1,0 | 1,0 | 1,0 | 0,6 | 0,9 | 1,0 | 1,0 | 0,7 | 0,7 | 0,8 | 1,1 | 1,0 | 1,0 |
| Fga | 1,0 | 0,6 | 1,0 | 0,2 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Fgb | 1,0 | 0,5 | 1,0 | 0,2 | 1,0 | 1,0 | 1,0 | 1,0 | 1,1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Fgf15 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Fgf23 | 1,0 | 1,0 | 1,0 | 1,0 | 1,0 | 1,0 | 1,0 | 1,0 | 1,0 | 1,0 | 1,0 | 1,0 | 1,0 | 1,0 | 6,2 | 20,9 | 1,0 | 1,0 | 1,0 | 1,0 | 1,0 | 1,0 | 1,0 | 1,0 |
| | AG | | Aorta | | Brain | | Lung | | Pancreas | | PG | | Skin | | Skull | | SM | | Spleen | | Testis | | TG | |
| | E | M | E | M | E | M | E | M | E | M | E | M | E | M | E | M | E | M | E | M | E | M | E | M |
| Fgfbp1 | 1,0 | 1,0 | 1,0 | 1,0 | 1,4 | 1,8 | 1,3 | 1,3 | 1,0 | 1,0 | 1,3 | 1,0 | 1,4 | 1,7 | 1,0 | 1,0 | 0,5 | 1,3 | 1,0 | 1,0 | 1,0 | 1,0 | 2,7 | 7,2 |
| Fgg | 1,0 | 1,0 | 1,0 | 0,2 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Fhl3 | 1,2 | 1,6 | 1,2 | 1,8 | 1,0 | 1,0 | 1,8 | 0,6 | 1,0 | 1,0 | 1,0 | 1,0 | 1,4 | 0,8 | 1,7 | 0,6 | 0,7 | 0,7 | 1,1 | 1,3 | 1,0 | 1,1 | 1,0 | 13,9 |
| Fhl4 | 1,0 | 1,0 | 1,0 | 1,0 | 1,0 | 1,0 | 1,0 | 1,0 | 1,0 | 1,0 | 0,9 | 1,4 | 1,0 | 1,0 | 1,0 | 1,0 | 1,0 | 1,0 | 1,0 | 1,0 | 1,0 | 1,1 | 1,0 | 1,0 |
| Fitm1 | 1,0 | 0,9 | 0,7 | 0,4 | 1,0 | 1,0 | 1,2 | 0,9 | 1,0 | 1,0 | 0,6 | 0,6 | 2,4 | 0,8 | 3,6 | 0,3 | 0,9 | 1,0 | 1,0 | 1,4 | 1,0 | 0,6 | 1,2 | 9,8 |
| Flnc | 0,9 | 0,9 | 1,4 | 0,9 | 1,0 | 1,0 | 1,0 | 1,0 | 1,0 | 1,0 | 1,0 | 1,0 | 2,1 | 0,7 | 2,6 | 0,3 | 0,8 | 1,1 | 1,0 | 1,0 | 1,0 | 0,9 | 1,8 | 9,8 |
| Flrt2 | 0,7 | 0,7 | 0,9 | 0,9 | 0,9 | 1,0 | 0,8 | 0,9 | 0,7 | 0,9 | 0,9 | 0,9 | 0,5 | 0,8 | 1,0 | 1,0 | 0,8 | 0,8 | 0,9 | 1,0 | 1,1 | 0,9 | 8,9 | 2,8 |
| Fmo2 | 0,9 | 0,9 | 1,7 | 1,3 | 1,4 | 1,8 | 1,3 | 1,6 | 1,4 | 1,0 | 1,4 | 1,4 | 1,5 | 1,6 | 1,5 | 1,1 | 1,5 | 1,5 | 1,0 | 1,0 | 1,1 | 1,4 | 1,1 | 2,0 |
| Fmo3 | 1,0 | 1,0 | 1,7 | 2,3 | 1,0 | 1,0 | 1,3 | 1,3 | 1,0 | 1,0 | 1,0 | 1,0 | 1,0 | 1,0 | 1,0 | 1,0 | 1,0 | 1,0 | 1,0 | 1,0 | 1,0 | 1,0 | 0,0 | 1,2 |
| Fn3k | 1,1 | 0,9 | 0,8 | 1,0 | 1,1 | 1,1 | 0,9 | 0,7 | 0,5 | 0,7 | 1,1 | 0,9 | 1,5 | 0,9 | 1,3 | 1,7 | 1,1 | 1,0 | 6,7 | 0,4 | 1,0 | 1,0 | 0,9 | 1,2 |
| Foxi1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Foxj1 | 1,0 | 1,0 | 1,0 | 1,0 | 1,0 | 0,8 | 1,2 | 1,0 | 1,0 | 1,0 | 0,8 | 1,1 | 1,0 | 1,0 | 1,1 | 1,4 | 1,0 | 1,0 | 1,0 | 1,0 | 1,2 | 0,8 | 0,8 | 1,2 |
| Snora78 | 0,2 | 0,5 | 0,9 | 0,8 | 0,8 | 0,6 | 0,9 | 1,7 | 1,1 | 0,7 | 1,3 | 1,2 | 1,0 | 1,2 | 1,2 | 0,6 | 0,9 | 1,5 | 1,0 | 1,2 | 0,7 | 1,1 | 1,4 | 1,4 |
| Foxo3 | 0,3 | 0,7 | 1,3 | 0,8 | 1,0 | 0,7 | 1,1 | 2,5 | 1,0 | 1,0 | 1,5 | 1,1 | 1,3 | 0,8 | 1,1 | 0,7 | 1,3 | 2,8 | 1,4 | 1,0 | 0,8 | 0,8 | 1,1 | 0,8 |
| Foxo6 | 0,7 | 1,2 | 1,1 | 0,9 | 0,8 | 0,9 | 1,2 | 0,9 | 0,9 | 1,0 | 0,8 | 1,0 | 0,6 | 0,9 | 1,0 | 0,7 | 0,2 | 0,6 | 1,0 | 1,0 | 0,9 | 0,8 | 1,3 | 1,0 |
| Foxq1 | 1,0 | 1,0 | 1,0 | 1,0 | 0,8 | 0,8 | 1,2 | 1,1 | 1,0 | 1,0 | 0,7 | 0,6 | 1,3 | 0,7 | 1,0 | 1,0 | 1,0 | 1,0 | 1,0 | 1,0 | 0,9 | 0,9 | 0,9 | 0,9 |
| Fsd2 | 1,0 | 1,0 | 0,8 | 0,5 | 1,0 | 1,0 | 1,1 | 0,9 | 1,0 | 1,0 | 1,0 | 1,0 | 1,5 | 0,7 | 3,1 | 0,3 | 0,8 | 0,9 | 0,9 | 1,1 | 1,0 | 1,0 | 0,8 | 4,5 |
| Fst | 1,2 | 1,1 | 1,3 | 1,0 | 1,1 | 0,9 | 2,8 | 2,2 | 1,0 | 1,0 | 1,0 | 1,0 | 1,0 | 1,0 | 1,3 | 2,0 | 0,6 | 0,6 | 1,0 | 1,0 | 1,0 | 1,0 | 0,9 | 0,7 |
| Fut1 | 1,0 | 1,0 | 1,0 | 1,0 | 1,0 | 1,0 | 1,0 | 1,0 | 1,0 | 1,3 | 0,6 | 0,8 | 0,9 | 0,9 | 1,0 | 1,0 | 1,0 | 1,0 | 0,7 | 1,0 | 1,1 | 0,7 | 1,0 | 1,0 |
| Fut4 | 0,7 | 0,6 | 1,0 | 1,0 | 1,0 | 1,0 | 1,4 | 1,5 | 1,0 | 1,0 | 1,1 | 1,3 | 0,7 | 0,6 | 0,9 | 1,0 | 1,0 | 1,0 | 0,9 | 1,2 | 1,0 | 1,0 | 0,9 | 1,0 |
| Fxyd3 | 1,0 | 1,0 | 1,0 | 1,0 | 1,0 | 1,0 | 1,0 | 1,0 | 1,0 | 1,0 | 1,0 | 1,0 | 0,2 | 0,8 | 1,0 | 1,0 | 1,0 | 1,0 | 1,0 | 1,0 | 1,0 | 1,0 | 1,0 | 1,0 |
| Fxyd4 | 1,0 | 1,0 | 1,0 | 1,0 | 1,0 | 1,0 | 1,0 | 1,0 | 1,0 | 1,0 | 1,0 | 1,0 | 1,2 | 1,1 | 1,0 | 1,0 | 1,0 | 1,0 | 1,0 | 1,0 | 1,0 | 1,0 | 1,2 | 0,6 |

[Fig. 23-6]

275/310

| | | | | | | | | | | | | | | | | | | | | | | | | |
|---|---|---|---|---|---|---|---|---|---|---|---|---|---|---|---|---|---|---|---|---|---|---|---|---|
| Fzd8 | 1,1 | 1,4 | 1,1 | 1,0 | 0,9 | 0,8 | 1,0 | 1,1 | 1,0 | 1,1 | 1,0 | 1,0 | 1,0 | 1,4 | 0,8 | 0,7 | 0,9 | 0,7 | 1,0 | 1,1 | 1,0 | 1,0 | 1,3 | 1,0 |
| Gadd45b | 0,8 | 1,3 | 1,3 | 0,9 | 1,1 | 1,1 | 1,0 | 1,0 | 1,0 | 1,0 | 1,0 | 1,5 | 1,3 | 0,9 | 1,1 | 1,0 | 1,1 | 1,7 | 0,8 | 0,8 | 1,1 | 1,1 | 0,8 | 1,8 |
| Gal | 1,1 | 0,8 | 1,0 | 1,0 | 0,8 | 0,7 | 0,9 | 0,7 | 0,4 | 0,2 | 0,3 | 0,6 | 1,0 | 1,0 | 1,0 | 1,0 | 1,0 | 1,0 | 1,0 | 1,0 | 1,0 | 1,0 | 1,0 | 1,0 |
| Gal3st4 | 1,0 | 0,9 | 1,0 | 1,0 | 1,0 | 0,9 | 1,0 | 0,8 | 1,0 | 1,0 | 1,0 | 0,9 | 1,2 | 1,2 | 0,8 | 0,8 | 1,0 | 1,0 | 0,9 | 0,9 | 1,0 | 1,0 | 1,1 | 0,6 |
| Galnt12 | 1,0 | 1,0 | 1,0 | -1,0 | 1,0 | 1,0 | 0,9 | 0,9 | 1,0 | 1,0 | 0,8 | 1,0 | 0,8 | 0,8 | 1,5 | 1,7 | 1,0 | 1,0 | 1,1 | 1,0 | 1,0 | 1,0 | 0,6 | 0,9 |
| Gamt | 1,0 | 1,3 | 0,8 | 0,6 | 0,8 | 0,9 | 0,8 | 0,6 | 0,8 | 0,9 | 1,1 | 0,8 | 0,9 | 0,6 | 0,9 | 0,9 | 0,2 | 0,4 | 1,0 | 0,8 | 1,0 | 1,0 | 1,3 | 1,4 |
| Gap43 | 0,7 | 8,2 | 1,3 | 0,9 | 1,0 | 1,1 | 0,7 | 0,8 | 1,0 | 1,0 | 1,7 | 1,0 | 0,5 | 0,8 | 0,6 | 1,0 | 1,0 | 1,0 | 1,0 | 1,0 | 1,0 | 1,0 | 1,0 | 1,0 |
| *Gast* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* |
| Gata3 | 1,0 | 0,7 | 1,0 | 1,0 | 0,8 | 1,0 | 1,1 | 0,8 | 1,0 | 1,0 | 1,0 | 1,0 | 1,0 | 0,8 | 1,0 | 1,0 | 1,0 | 1,0 | 1,1 | 0,8 | 1,0 | 1,1 | 8,6 | 4,2 |
| Gbp11 | 0,5 | 1,0 | 0,3 | 0,7 | 1,0 | 1,0 | 1,0 | 1,0 | 1,0 | 1,0 | 1,2 | 0,8 | 0,7 | 1,0 | 1,0 | 1,0 | 1,0 | 1,0 | 0,9 | 1,0 | 1,0 | 1,0 | 1,0 | 1,0 |
| Gbp6 | 0,8 | 0,6 | 0,9 | 0,5 | 1,2 | 1,0 | 0,6 | 0,6 | 1,0 | 1,0 | 1,0 | 1,0 | 1,1 | 1,2 | 0,9 | 0,6 | 0,5 | 0,6 | 0,8 | 0,7 | 1,0 | 1,0 | 0,6 | 0,8 |
| Gc | 1,0 | 0,7 | 1,0 | 0,2 | 1,0 | 1,0 | 1,0 | 1,0 | 0,9 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Gcm2 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 8,5 | 6,3 |
| Gcnt4 | 1,0 | 1,0 | 1,0 | 1,0 | 0,9 | 0,8 | 1,0 | 1,0 | 1,0 | 1,0 | 1,0 | 1,0 | 1,2 | 1,4 | 1,0 | 1,0 | 1,0 | 1,0 | 1,0 | 1,0 | 1,0 | 1,1 | 1,0 | 1,0 |
| Gfi1b | 1,0 | 1,0 | 1,0 | 1,0 | 1,0 | 1,0 | 1,0 | 1,0 | 1,0 | 1,0 | 1,0 | 1,0 | 1,0 | 1,0 | 1,2 | 1,3 | 1,0 | 1,0 | 3,2 | 0,3 | 1,0 | 1,0 | 1,0 | 1,0 |
| Gfra1 | 0,8 | 0,7 | 2,0 | 1,8 | 1,0 | 0,9 | 1,1 | 1,1 | 1,0 | 1,5 | 1,0 | 1,8 | 0,8 | 1,1 | 0,9 | 0,9 | 1,0 | 1,1 | 1,0 | 1,0 | 1,1 | 1,0 | 1,0 | 1,0 |
| Snord15b | 0,1 | 0,8 | 1,0 | 1,0 | 0,9 | 1,0 | 1,1 | 1,0 | 1,5 | 1,1 | 0,8 | 0,7 | 1,5 | 1,0 | 3,3 | 1,1 | 1,0 | 1,0 | 0,7 | 0,8 | 1,0 | 1,0 | 0,8 | 1,0 |
| Glb1l3 | 1,2 | 0,8 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,2 | 1,3 | 1,0 | 1,0 |
| Gldc | 1,0 | 1,0 | 1,0 | 1,0 | 1,1 | 0,9 | 1,0 | 1,0 | 1,0 | 1,0 | 1,3 | 1,5 | 1,0 | 1,8 | 1,0 | 1,0 | 1,0 | 1,0 | 1,0 | 1,0 | 0,9 | 1,5 | 1,0 | 1,0 |
| Gli1 | 0,8 | 0,6 | 1,5 | 1,0 | 1,1 | 1,0 | 1,1 | 1,2 | 1,0 | 1,0 | 1,7 | 1,2 | 0,2 | 0,8 | 0,6 | 0,4 | 1,0 | 1,0 | 1,1 | 1,5 | 1,0 | 0,9 | 1,0 | 1,2 |
| Glrp1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,8 | 1,0 | 1,0 | 1,0 |
| Spt1 | 0,0 | 0,6 | 1,0 | 1,0 | 0,6 | 1,3 | 0,8 | 1,7 | 1,0 | 1,0 | 0,9 | 0,6 | 1,0 | 1,0 | 1,0 | 2,2 | 1,0 | 1,0 | 1,0 | 1,0 | 1,0 | 0,8 | 1,0 | 1,0 |
| Gml | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,9 | 1,3 | 1,0 | 1,0 |
| Golt1a | 1,0 | 1,0 | 1,0 | 1,0 | 1,0 | 1,0 | 0,8 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Gp2 | 1,0 | 1,0 | 1,0 | 1,0 | 1,0 | 1,0 | 0,7 | 0,9 | 1,5 | 1,6 | 1,0 | 1,0 | 1,0 | 1,0 | 1,0 | 1,0 | 1,0 | 1,0 | 1,0 | 1,2 | 1,0 | 1,0 | 0,1 | 0,1 |
| *Gpcpd1* | *1,1* | *1,0* | *0,9* | *1,3* | *1,0* | *1,1* | *1,0* | *1,2* | *0,7* | *1,4* | *0,9* | *1,1* | *1,3* | *1,2* | *1,2* | *1,3* | *1,3* | *1,5* | *1,0* | *1,0* | *1,0* | *1,1* | *1,0* | *2,1* |
| Gpnmb | 1,1 | 0,3 | 1,2 | 1,5 | 1,0 | 1,0 | 1,5 | 1,0 | 1,0 | 1,0 | 1,0 | 1,0 | 1,2 | 0,7 | 0,8 | 0,7 | 1,7 | 1,1 | 0,6 | 0,5 | 1,0 | 1,0 | 1,1 | 1,2 |
| Gpr18 | 0,9 | 1,0 | 1,0 | -1,0 | 1,0 | 1,0 | 0,6 | 0,6 | 1,0 | 1,0 | 1,0 | 1,0 | 1,1 | 1,5 | 1,3 | 1,3 | 1,0 | 1,0 | 1,0 | 1,0 | 1,0 | 1,0 | 1,0 | 1,0 |
| Gpr82 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,3 | 1,3 | 1,0 | 1,0 | 1,0 | 1,0 |
| Gpx5 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Gpx6 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,2 | 1,0 | 1,0 |
| Grb14 | 0,7 | 0,8 | 0,8 | 0,7 | 1,1 | 1,0 | 0,7 | 1,1 | 1,0 | 1,0 | 0,8 | 1,2 | 0,6 | 0,6 | 1,4 | 0,9 | 0,4 | 0,4 | 1,0 | 1,0 | 1,0 | 1,0 | 0,8 | 1,0 |
| Grhl2 | 1,0 | 1,0 | 1,0 | 1,0 | 1,0 | 1,0 | 1,1 | 1,2 | 1,0 | 1,1 | 1,2 | 1,0 | 0,9 | 1,0 | 1,0 | 1,0 | 1,0 | 1,0 | 1,0 | 1,0 | 1,1 | 1,2 | 1,0 | 0,9 |
| Gsdmc | 1,0 | 1,0 | 1,0 | 1,0 | 1,0 | 1,0 | 1,0 | 1,0 | 1,0 | 1,0 | 1,0 | 1,0 | 0,7 | 2,5 | 1,0 | 1,0 | 1,0 | 1,0 | 1,0 | 1,0 | 1,0 | 1,0 | 1,0 | 1,0 |
| Gsdmc2 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Gsdmc3 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Gsdmc4 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,7 | 0,9 | 1,0 | 1,0 |
| Gsg1 | 1,0 | 1,0 | 1,0 | 1,0 | 1,0 | 1,0 | 1,0 | 1,0 | 1,0 | 1,0 | 1,0 | 1,0 | 1,0 | 1,0 | 0,7 | 1,0 | 1,0 | 1,0 | 1,0 | 1,1 | 1,1 | 0,9 | 1,0 | 1,0 |
| Gsta1 | 1,0 | 1,0 | 1,0 | 1,0 | 1,0 | 1,0 | 1,0 | 0,8 | 1,0 | 1,0 | 1,0 | 1,0 | 1,2 | 1,0 | 1,0 | 1,0 | 1,0 | 1,0 | 1,0 | 1,0 | 1,0 | 1,0 | 1,0 | 1,0 |
| Gsta2 | 1,0 | 1,0 | 1,0 | 1,0 | 1,0 | 1,0 | 1,3 | 0,9 | 1,0 | 1,0 | 1,0 | 1,0 | 1,6 | 1,2 | 1,0 | 1,0 | 1,0 | 1,0 | 1,0 | 1,0 | 0,6 | 1,3 | 1,0 | 1,0 |
| Gsta4 | 1,5 | 1,4 | 0,8 | 1,2 | 1,1 | 1,1 | 1,2 | 1,0 | 1,0 | 1,0 | 1,1 | 1,4 | 1,2 | 1,2 | 0,9 | 1,4 | 0,8 | 0,7 | 1,5 | 1,0 | 1,2 | 1,2 | 1,5 | 1,7 |
| Gstm1 | 1,5 | 1,1 | 1,6 | 1,5 | 1,2 | 1,3 | 1,2 | 1,1 | 0,9 | 1,4 | 1,3 | 1,2 | 1,7 | 1,3 | 1,0 | 1,1 | 1,6 | 1,5 | 1,2 | 1,4 | 0,8 | 0,6 | 1,3 | 1,6 |
| Gstm3 | 1,0 | 1,1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| *Gulo* | *0,7* | *0,8* | *1,0* | *1,0* | *1,0* | *1,0* | *1,0* | *1,0* | *1,0* | *1,0* | *1,1* | *1,7* | *1,0* | *1,0* | *1,0* | *1,0* | *1,0* | *1,0* | *1,0* | *1,0* | *1,0* | *1,0* | *1,0* | *1,0* |
| Gypa | 1,0 | 1,0 | 1,0 | 1,0 | 1,0 | 1,0 | 1,0 | 1,0 | 1,0 | 1,0 | 1,0 | 1,0 | 1,0 | 1,0 | 1,8 | 1,9 | 1,0 | 1,0 | 6,6 | 0,4 | 1,0 | 1,0 | 1,0 | 1,0 |
| H19 | 1,0 | 1,0 | 0,6 | 0,2 | 1,0 | 1,0 | 1,0 | 1,0 | 1,0 | 1,0 | 0,6 | 1,0 | 1,6 | 0,8 | 1,0 | 1,0 | 1,3 | 1,7 | 1,0 | 1,0 | 1,0 | 1,0 | 2,2 | 5,6 |
| H1fx | 0,9 | 1,0 | 0,7 | 0,7 | 0,9 | 1,2 | 0,7 | 0,9 | 1,0 | 1,0 | 0,8 | 0,8 | 0,5 | 0,6 | 0,8 | 0,8 | 0,9 | 0,8 | 0,6 | 0,3 | 1,1 | 0,7 | 1,2 | 0,8 |
| H2-Ob | 1,0 | 1,0 | 1,0 | 1,0 | 1,0 | 1,0 | .0,3 | 0,5 | 1,0 | 1,0 | 1,0 | 1,0 | 0,8 | 1,0 | 1,1 | 1,8 | 1,0 | 1,0 | 0,8 | 0,9 | 1,0 | 1,0 | 0,9 | 0,9 |
| H2-Q10 | 0,9 | 1,2 | 0,3 | 0,5 | 1,0 | 1,0 | 2,2 | 0,8 | 1,0 | 1,0 | 0,8 | 1,3 | 0,9 | 2,5 | 1,2 | 1,9 | 0,5 | 0,4 | 1,1 | 1,5 | 1,0 | 1,0 | 1,5 | 0,9 |
| H2-Q6 | 0,5 | 1,0 | 0,8 | 0,9 | 1,0 | 1,0 | 0,7 | 0,8 | 0,7 | 0,9 | 1,5 | 0,6 | 1,3 | 1,4 | 0,9 | 1,0 | 1,9 | 0,7 | 0,8 | 1,0 | 0,7 | 1,1 | 0,8 | 1,1 |
| H2-Q8 | 1,0 | 0,3 | 1,0 | 1,0 | 1,0 | 1,0 | 0,1 | 0,6 | 1,0 | 1,0 | 0,4 | 1,0 | 0,4 | 0,4 | 1,0 | 1,6 | 0,3 | 1,0 | 1,0 | 2,7 | 1,0 | 1,0 | 1,0 | 1,0 |
| Hamp | 1,7 | 0,8 | 1,7 | 1,1 | 1,0 | 1,0 | 1,2 | 0,8 | 1,0 | 1,0 | 1,0 | 1,0 | 1,7 | 1,0 | 1,0 | 1,0 | 1,0 | 1,0 | 1,0 | 1,1 | 1,0 | 1,0 | 1,0 | 1,0 |
| Hamp2 | 1,0 | 1,0 | 2,1 | 2,2 | 1,0 | 1,0 | 1,0 | 1,0 | 0,5 | 0,8 | 1,0 | 1,0 | 46,1 | 3,9 | 1,0 | 1,0 | 1,0 | 1,0 | 1,0 | 1,0 | 1,1 | 1,0 | 1,0 | 1,7 |
| Hao1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| *Hap1* | *0,8* | *0,7* | *1,0* | *1,0* | *0,9* | *1,1* | *0,8* | *0,9* | *1,0* | *1,0* | *1,1* | *1,2* | *0,7* | *0,7* | *1,1* | *1,1* | *1,0* | *1,0* | *1,2* | *1,3* | *1,1* | *0,9* | *1,4* | *1,1* |
| Has3 | 1,0 | 0,8 | 1,1 | 1,0 | 1,1 | 0,9 | 1,1 | 1,0 | 1,0 | 1,0 | 1,1 | 0,8 | 0,2 | 0,5 | 1,0 | 1,0 | 0,8 | 1,0 | 1,1 | 1,0 | 0,9 | 0,9 | 1,0 | 1,0 |
| Havcr1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,7 | 0,6 | 1,0 | 1,0 | 1,0 | 1,0 |
| Hba-a2 | 13,2 | 8,0 | 4,5 | 2,8 | 0,0 | 2,0 | 3,4 | 1,4 | 1,0 | 2,0 | 20,2 | 0,5 | 1,0 | 1,6 | 0,1 | 2,0 | 1,0 | 2,8 | 1,2 | 0,7 | 1,0 | 1,0 | 1,8 | 1,5 |
| Hbb-bs | 66,8 | 2,7 | 34,3 | 2,0 | 0,0 | 7,2 | 2,0 | 1,3 | 1,0 | 0,4 | 20,0 | 0,2 | 1,0 | 1,0 | 0,3 | 2,8 | 1,0 | 4,7 | 1,6 | 0,8 | 1,0 | 1,0 | 19,3 | 4,5 |
| Hebp2 | 0,8 | 0,9 | 0,5 | 0,3 | 0,9 | 1,0 | 1,0 | 0,8 | 1,0 | 1,0 | 0,8 | 0,8 | 1,0 | 1,2 | 0,7 | 0,9 | 1,0 | 1,0 | 1,0 | 1,0 | 1,4 | 1,1 | 1,2 | 0,9 |
| Hemgn | 1,0 | 1,0 | 1,0 | 1,0 | 1,0 | 1,0 | 1,0 | 1,0 | 1,0 | 1,0 | 1,0 | 1,0 | 1,0 | 1,0 | 1,6 | 1,4 | 1,0 | 1,0 | 6,5 | 0,2 | 1,1 | 1,1 | 1,0 | 1,0 |
| Hemt1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,1 | 1,3 | 1,0 | 1,0 |
| Hfe2 | 1,0 | 1,0 | 0,6 | 0,7 | 1,0 | 1,0 | 0,6 | 0,6 | 1,0 | 1,0 | 1,0 | 1,0 | 2,1 | 0,9 | 4,0 | 0,3 | 0,9 | 1,0 | 1,0 | 1,0 | 1,0 | 0,9 | 1,0 | 12,8 |
| Hgfac | 1,0 | 1,0 | 1,0 | 1,0 | 1,0 | 1,0 | 1,1 | 1,1 | 1,2 | 0,9 | 1,0 | 1,0 | 1,0 | 1,0 | 1,0 | 1,0 | 1,0 | 1,0 | 1,0 | 1,0 | 1,0 | 1,0 | 1,0 | 1,0 |
| Hhatl | 1,0 | 1,0 | 1,0 | 0,8 | 0,9 | 1,1 | 1,0 | 0,8 | 1,0 | 1,0 | 1,2 | 2,3 | 2,2 | 0,9 | 3,0 | 0,2 | 0,9 | 1,2 | 1,0 | 1,0 | 0,9 | 1,1 | 1,0 | 1,7 |
| Gh | 0,1 | 0,7 | 0,9 | 0,8 | 0,6 | 0,6 | 0,8 | 1,4 | 1,9 | 0,9 | 1,3 | 1,1 | 0,7 | 0,8 | 1,6 | 0,5 | 0,8 | 0,8 | 1,2 | 0,9 | 1,1 | 0,9 | 0,9 | 0,6 |
| Hist1h1e | 0,4 | 1,4 | 0,7 | 0,7 | 0,6 | 0,8 | 0,7 | 0,4 | 0,7 | 1,0 | 0,5 | 0,6 | 1,4 | 0,7 | 1,4 | 0,8 | 0,7 | 0,9 | 0,8 | 0,7 | 1,0 | 1,0 | 1,0 | 0,8 |
| Hist1h2ba | 0,9 | 1,1 | 0,9 | 0,6 | 1,7 | 1,4 | 0,7 | 0,5 | 0,5 | 1,6 | 0,6 | 1,1 | 1,2 | 0,9 | 1,1 | 2,8 | 0,7 | 0,9 | 0,4 | 0,4 | 0,8 | 0,6 | 1,0 | 1,1 |
| Hist1h4h | 0,6 | 0,8 | 0,4 | 0,7 | 1,0 | 1,9 | 1,2 | 0,6 | 2,6 | 1,3 | 0,4 | 0,7 | 1,6 | 0,9 | 2,7 | 1,2 | 1,2 | 0,9 | 0,4 | 0,8 | 0,9 | 0,6 | 1,4 | 2,3 |
| Hist1h4i | 0,7 | 1,2 | 1,4 | 0,3 | 1,6 | 0,5 | 0,6 | 0,6 | 2,1 | 0,7 | 1,0 | 0,9 | 1,0 | 0,6 | 1,1 | 0,9 | 0,9 | 0,7 | 1,3 | 0,8 | 0,5 | 0,8 | 1,0 | 4,0 |
| Hist1h4j | 2,1 | 1,8 | 0,1 | 0,7 | 0,5 | 1,0 | ·1,0 | 0,8 | 1,0 | 1,0 | 0,5 | 0,8 | 1,6 | 1,0 | 0,9 | 0,6 | 1,6 | 0,4 | 0,6 | 1,2 | 1,9 | 0,9 | 1,3 | 1,6 |
| Hist1h4k | 0,5 | 1,0 | 0,5 | 1,2 | 0,4 | 0,7 | 0,8 | 0,4 | 1,0 | 1,0 | 0,9 | 0,5 | 1,3 | 0,7 | 0,6 | 1,2 | 1,4 | 1,2 | 1,1 | 0,4 | 1,5 | 0,4 | 1,9 | 1,3 |
| *Hist2h2aa2* | *0,6* | *1,2* | *1,1* | *0,5* | *3,9* | *1,0* | *3,7* | *5,1* | *1,0* | *1,0* | *1,0* | *1,0* | *0,4* | *4,5* | *3,9* | *1,0* | *0,4* | *0,8* | *0,2* | *1,8* | *1,0* | *0,8* | *0,1* | *1,6* |
| Hist2h2ac | 0,7 | 1,0 | 0,9 | ˙0,6 | 1,0 | 1,0 | 1,0 | 1,0 | 1,0 | 1,0 | 1,9 | 0,6 | 2,8 | 1,2 | 1,0 | 1,0 | 0,4 | 1,1 | 1,0 | 0,3 | 0,7 | 1,2 | 1,0 | 1,0 |
| Hist2h3b | 0,3 | 1,0 | 0,6 | 0,5 | 0,4 | 0,6 | 0,8 | 0,6 | 1,2 | 0,2 | 0,7 | 0,2 | 1,4 | 0,8 | 1,3 | 1,0 | 0,8 | 1,1 | 0,8 | 0,6 | 1,6 | 0,7 | 0,6 | 1,0 |
| | AG | | Aorta | | Brain | | Lung | | Pancreas | | PG | | Skin | | Skull | | SM | | Spleen | | Testis | | TG | |
| | E | M | E | M | E | M | E | M | E | M | E | M | E | M | E | M | E | M | E | M | E | M | E | M |
| Hist2h3c1 | 0,2 | 0,6 | 0,2 | 1,0 | 1,0 | 0,4 | 0,0 | 0,9 | 0,2 | 0,3 | 4,8 | 0,2 | 1,0 | 1,0 | 9,4 | 0,9 | 0,9 | 0,1 | 1,0 | 1,0 | 1,0 | 1,0 | 1,0 | 0,3 |
| Hist2h3c2 | 0,6 | 2,3 | 1,7 | 0,4 | 1,0 | 2,5 | 7,0 | 0,9 | 1,6 | 1,3 | 0,8 | 8,1 | 0,9 | 0,4 | 0,1 | 0,6 | 1,1 | 11,8 | 0,9 | 0,6 | 0,8 | 0,6 | 0,9 | 1,2 |
| Hmgcs2 | 1,0 | 0,8 | 1,4 | 1,4 | 1,5 | 1,2 | 1,1 | 1,0 | 0,9 | 2,5 | 1,4 | 1,4 | 1,2 | 2,5 | 0,8 | 1,1 | 1,1 | 1,1 | 1,0 | 1,5 | 0,8 | 0,8 | 0,5 | 0,8 |
| Hnrnpa1 | 2,1 | 1,1 | 0,4 | 2,5 | 0,9 | 1,5 | 0,7 | 0,9 | 1,2 | 2,0 | 1,1 | 1,1 | 0,9 | 0,4 | 0,6 | 0,9 | 0,9 | 2,0 | 1,1 | 0,7 | 0,7 | 0,7 | 1,0 | 1,5 |
| Hoxb6 | 1,1 | 1,2 | 1,3 | 0,7 | 1,0 | 1,0 | 1,4 | 0,8 | 1,0 | 1,0 | 1,0 | 1,0 | 1,1 | 0,6 | 1,0 | 1,0 | 1,5 | 1,0 | 1,3 | 0,8 | 1,0 | 1,0 | 0,9 | 0,9 |
| Hoxb8 | 1,0 | 1,1 | 1,2 | 0,8 | 1,0 | 1,0 | 1,0 | 1,0 | 1,0 | 1,0 | 1,0 | 1,0 | 0,6 | 0,7 | 1,0 | 1,0 | 1,0 | 1,0 | 1,0 | 1,0 | 1,0 | 1,0 | 0,6 | 0,8 |
| *Hoxd3* | *0,8* | *0,8* | *1,0* | *1,0* | *1,0* | *1,0* | *1,0* | *1,0* | *1,0* | *1,0* | *1,0* | *1,0* | *1,1* | *1,0* | *1,0* | *1,0* | *1,0* | *1,0* | *1,0* | *1,0* | *1,0* | *1,0* | *1,0* | *1,0* |

[Fig. 23-7]

276/310

| | | | | | | | | | | | | | | | | | | | | | | | | |
|---|---|---|---|---|---|---|---|---|---|---|---|---|---|---|---|---|---|---|---|---|---|---|---|---|
| Hpdl | 0,7 | 1,0 | 0,8 | 0,9 | 0,6 | 1,3 | 1,0 | 1,0 | 1,0 | 1,0 | 1,0 | 1,0 | 0,8 | 1,0 | 1,0 | 1,0 | 0,9 | 0,8 | 1,0 | 1,0 | 1,2 | 0,7 | 1,1 | 1,0 |
| Hpx | 1,5 | 0,4 | 1,0 | -0,2 | 1,0 | 1,0 | 1,5 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Hr | 0,7 | 0,7 | 1,0 | 0,8 | 0,9 | 1,0 | 0,9 | 1,0 | 1,0 | 1,0 | 0,8 | 0,9 | 1,2 | 1,2 | 1,0 | 0,4 | 0,9 | 1,1 | 0,9 | 1,4 | 1,0 | 0,9 | 1,0 | 1,7 |
| Hrc | 1,0 | 1,0 | 0,8 | 0,8 | 1,0 | 1,0 | 0,8 | 0,7 | 1,0 | 1,0 | 1,0 | 1,0 | 2,0 | 1,0 | 3,9 | 0,4 | 0,9 | 1,0 | 1,0 | 1,0 | 1,0 | 1,0 | 1,5 | 6,2 |
| *Hrg* | 1,0 | 1,0 | 1,0 | 0,5 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Hs3st3b1 | 1,2 | 1,1 | 1,0 | 1,0 | 1,0 | 1,0 | 1,1 | 1,0 | 0,9 | 0,9 | 1,0 | 1,0 | 1,3 | 1,0 | 0,8 | 0,7 | 1,0 | 1,0 | 1,1 | 0,9 | 1,2 | 1,1 | 1,0 | 1,0 |
| Hsd17b11 | 0,9 | 0,9 | 1,0 | 1,3 | 1,1 | 1,2 | 1,1 | 1,0 | 1,0 | 1,4 | 1,1 | 1,1 | 1,1 | 1,3 | 0,9 | 1,2 | 1,0 | 1,0 | 1,0 | 1,1 | 1,0 | 1,2 | 1,1 | 1,0 |
| Hsd17b6 | 1,0 | 1,0 | 1,0 | 1,0 | 1,0 | 1,0 | 1,0 | 1,0 | 1,0 | 1,0 | 1,0 | 1,0 | 0,5 | 2,6 | 1,0 | 1,0 | 1,0 | 1,0 | 1,0 | 1,0 | 1,0 | 1,0 | 1,0 | 1,0 |
| Hsd3b2 | 0,8 | 1,3 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Hsd3b5 | 1,3 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Hspb3 | 1,0 | 1,0 | 1,0 | 0,8 | 2,1 | 1,5 | 1,0 | 1,0 | 1,0 | 1,0 | 1,0 | 1,0 | 2,1 | 0,7 | 4,5 | 0,4 | 0,7 | 0,8 | 1,0 | 1,0 | 1,0 | 1,0 | 1,2 | 6,8 |
| Hspb6 | 0,8 | 0,8 | 0,9 | 0,6 | 1,1 | 1,1 | 0,7 | 1,0 | 0,8 | 1,3 | 0,9 | 1,1 | 1,3 | 0,7 | 3,8 | 0,3 | 0,7 | 0,9 | 1,4 | 1,3 | 0,9 | 0,9 | 1,2 | 5,4 |
| Hspb7 | 1,5 | 0,8 | 1,1 | 1,1 | 1,0 | 1,0 | 0,9 | 1,0 | 1,0 | 1,0 | 1,0 | 1,0 | 1,8 | 0,7 | 3,8 | 0,4 | 0,8 | 1,1 | 1,1 | 1,9 | 0,9 | 1,0 | 1,9 | 10,9 |
| Hspb9 | 1,0 | 1,0 | 1,0 | 1,0 | 1,0 | 1,0 | 1,0 | 1,1 | 1,0 | 1,0 | 1,0 | 1,0 | 1,0 | 1,0 | 1,0 | 1,0 | 1,0 | 1,0 | 1,0 | 1,0 | 1,0 | 0,9 | 1,0 | 1,0 |
| Icam4 | 0,6 | 0,4 | 1,0 | 1,0 | 1,0 | 1,0 | 0,9 | 0,7 | 1,0 | 1,0 | 1,2 | 0,5 | 1,0 | 1,0 | 1,4 | 1,6 | 1,6 | 1,2 | 6,5 | 0,4 | 1,0 | 1,0 | 1,4 | 1,0 |
| Ido1 | 1,0 | 1,0 | 1,0 | 1,0 | 1,7 | 0,9 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Igfbp1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| *Igj* | 10,9 | 4,9 | 0,9 | 1,5 | 1,0 | 1,0 | 1,5 | 0,4 | 0,8 | 0,7 | 0,9 | 1,0 | 1,6 | 2,8 | 0,6 | 0,8 | 1,0 | 1,0 | 0,7 | 0,7 | 1,0 | 0,9 | 1,0 | 1,0 |
| Ikzf3 | 1,0 | 1,0 | 1,0 | 1,0 | 1,0 | 1,0 | 0,6 | 0,6 | 1,0 | 1,0 | 1,0 | 1,0 | 1,0 | 1,0 | 0,9 | 0,7 | 1,0 | 1,0 | 0,8 | 0,8 | 1,0 | 1,0 | 0,8 | 1,1 |
| Il21r | 0,6 | 0,7 | 1,0 | 1,0 | 1,0 | 1,0 | 0,5 | 0,7 | 1,0 | 1,0 | 1,0 | 1,0 | 1,0 | 1,0 | 0,9 | 1,3 | 1,0 | 1,0 | 0,9 | 0,9 | 1,0 | 1,0 | 0,4 | 0,9 |
| Il22ra1 | 1,0 | 1,0 | 1,0 | 1,0 | 1,0 | 1,0 | 1,0 | 1,3 | 1,8 | 1,7 | 1,6 | 1,8 | 1,3 | 1,3 | 1,0 | 1,0 | 1,0 | 1,0 | 1,0 | 1,0 | 1,0 | 1,0 | 1,0 | 1,0 |
| Ildr1 | 1,0 | 1,0 | 1,0 | 1,0 | 1,0 | 1,0 | 1,0 | 1,1 | 1,0 | 1,0 | 1,0 | 0,9 | 1,2 | 1,0 | 1,0 | 1,0 | 1,0 | 1,0 | 0,6 | 0,5 | 1,0 | 1,0 | 1,0 | 0,7 |
| Ildr2 | 0,7 | 0,6 | 1,4 | 1,2 | 0,8 | 0,8 | 0,9 | 1,0 | 1,0 | 1,0 | 1,4 | 1,4 | 1,0 | 0,9 | 0,9 | 0,8 | 0,9 | 1,0 | 0,9 | 1,0 | 1,0 | 1,0 | 1,0 | 1,0 |
| Inha | 1,3 | 1,4 | 1,2 | 1,2 | 1,0 | 1,0 | 0,8 | 1,2 | 2,2 | 2,4 | 1,0 | 0,9 | 1,1 | 1,3 | 0,8 | 0,9 | 0,9 | 0,8 | 0,7 | 1,5 | 1,0 | 0,8 | 1,2 | 1,1 |
| Insc | 1,0 | 1,0 | 1,0 | 1,0 | 0,9 | 0,6 | 1,0 | 1,0 | 1,0 | 1,0 | 1,0 | 1,0 | 1,0 | 1,0 | 0,7 | 1,0 | 1,0 | 1,0 | 1,0 | 1,0 | 1,0 | 1,0 | 1,0 | 1,0 |
| Ip6k3 | 1,0 | 1,0 | 1,0 | 1,0 | 1,0 | 1,0 | 1,4 | 1,5 | 1,0 | 1,0 | 1,0 | 1,0 | 2,0 | 1,0 | 4,1 | 0,6 | 1,3 | 1,5 | 1,0 | 0,8 | 1,0 | 1,0 | 1,2 | 8,1 |
| Isyna1 | 0,9 | 0,8 | 1,3 | 1,1 | 1,1 | 1,0 | 1,0 | 1,0 | 1,4 | 1,6 | 1,0 | 1,0 | 1,0 | 0,8 | 1,0 | 1,1 | 1,1 | 1,8 | 1,1 | 1,1 | 1,1 | 0,8 | 1,0 | 0,8 |
| Itgax | 0,8 | 0,5 | 1,0 | 1,0 | 1,0 | 1,0 | 1,1 | 0,9 | 1,0 | 1,0 | 1,0 | 1,0 | 0,9 | 2,6 | 0,9 | 1,3 | 1,0 | 1,0 | 1,0 | 1,1 | 1,0 | 1,0 | 1,0 | 1,0 |
| Itgb1bp2 | 1,0 | 1,0 | 0,8 | 0,8 | 1,0 | 1,0 | 0,8 | 0,6 | 1,0 | 1,0 | 1,0 | 1,0 | 1,5 | 0,6 | 4,0 | 0,5 | 0,7 | 0,7 | 1,0 | 1,0 | 1,0 | 1,0 | 1,2 | 7,1 |
| Itgb6 | 1,0 | 1,0 | 1,0 | 1,0 | 1,0 | 1,0 | 1,0 | 0,8 | 1,0 | 1,0 | 1,0 | 1,0 | 0,5 | 0,9 | 1,1 | 0,8 | 0,7 | 0,8 | 1,0 | 1,0 | 1,0 | 1,0 | 0,9 | 1,0 |
| *Itln1* | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Jph1 | 1,0 | 1,0 | 1,5 | 1,0 | 1,0 | 1,0 | 1,0 | 1,0 | 1,0 | 1,0 | 1,4 | 0,9 | 1,9 | 1,0 | 3,2 | 0,3 | 1,0 | 1,1 | 1,0 | 1,0 | 1,0 | 1,0 | 0,9 | 1,9 |
| Jph2 | 0,8 | 0,7 | 1,2 | 0,8 | 1,0 | 1,0 | 0,8 | 0,8 | 1,0 | 1,0 | 0,4 | 0,7 | 1,9 | 0,9 | 3,2 | 0,4 | 0,9 | 0,9 | 1,0 | 1,1 | 1,0 | 0,9 | 0,8 | 8,2 |
| Jsrp1 | 1,2 | 0,8 | 1,0 | 0,9 | 1,0 | 0,8 | 1,0 | 1,0 | 1,0 | 1,0 | 1,0 | 1,0 | 2,5 | 0,9 | 3,3 | 0,4 | 1,0 | 1,1 | 1,0 | 1,0 | 0,7 | 0,9 | 1,3 | 19,5 |
| Kcna7 | 1,0 | 1,0 | 1,0 | 1,0 | 1,0 | 1,0 | 1,0 | 1,0 | 1,0 | 1,0 | 1,0 | 1,0 | 1,6 | 0,8 | 3,2 | 0,2 | 0,8 | 0,9 | 1,0 | 1,0 | 1,0 | 1,0 | 1,1 | 8,3 |
| Kcnc1 | 1,0 | 1,0 | 1,0 | 1,0 | 0,9 | 0,8 | 1,0 | 1,0 | 1,0 | 1,0 | 1,4 | 1,2 | 1,7 | 0,6 | 2,2 | 0,2 | 0,7 | 0,9 | 1,0 | 1,0 | 1,1 | 0,8 | 1,5 | 2,6 |
| Kcnc4 | 0,8 | 0,7 | 1,6 | 1,0 | 0,9 | 1,0 | 1,0 | 1,0 | 1,0 | 1,0 | 1,1 | 1,0 | 2,7 | 1,2 | 3,3 | 0,3 | 1,5 | 1,5 | 1,0 | 1,0 | 1,0 | 1,0 | 1,4 | 5,5 |
| Kcne3 | 1,5 | 1,6 | 1,7 | 1,0 | 1,0 | 1,0 | 0,7 | 0,8 | 1,7 | 1,0 | 1,0 | 1,0 | 1,1 | 1,3 | 0,7 | 1,0 | 1,4 | 1,2 | 0,7 | 1,0 | 1,0 | 1,1 | 0,5 | 0,2 |
| Kcng4 | 1,0 | 1,0 | 1,0 | 1,0 | 1,0 | 1,0 | 1,0 | 1,0 | 1,0 | 1,0 | 1,0 | 1,0 | 1,0 | 0,9 | 1,6 | 0,2 | 0,2 | 0,4 | 1,0 | 1,0 | 1,1 | 1,0 | 2,6 | 1,5 |
| Kcnh3 | 1,0 | 1,0 | 1,0 | 1,0 | 1,1 | 1,2 | 1,4 | 0,9 | 1,0 | 1,0 | 1,9 | 1,3 | 1,0 | 1,0 | 1,0 | 1,0 | 1,0 | 1,0 | 1,2 | 1,2 | 1,0 | 1,0 | 0,9 | 0,8 |
| Kcnj11 | 0,4 | 0,5 | 0,9 | 1,0 | 1,0 | 1,0 | 0,8 | 0,8 | 1,0 | 1,0 | 1,7 | 1,2 | 1,9 | 0,8 | 4,2 | 0,2 | 1,1 | 1,1 | 1,0 | 1,0 | 1,0 | 1,0 | 1,1 | 2,8 |
| Kcnj12 | 0,9 | 0,6 | 1,0 | 1,0 | 1,0 | 1,0 | 1,0 | 1,0 | 1,0 | 1,0 | 1,0 | 1,0 | 1,7 | 0,8 | 3,7 | 0,2 | 0,9 | 1,2 | 1,0 | 1,0 | 1,0 | 1,0 | 1,2 | 6,9 |
| Kcnj15 | 0,8 | 1,0 | 0,9 | 0,6 | 1,0 | 1,0 | 1,3 | 1,3 | 1,2 | 1,0 | 1,0 | 1,0 | 1,3 | 1,5 | 5,7 | 6,1 | 1,0 | 1,0 | 1,0 | 1,0 | 1,0 | 1,0 | 1,0 | 0,8 |
| Kcnk1 | 0,7 | 0,6 | 1,0 | 1,0 | 1,0 | 1,0 | 1,3 | 1,4 | 1,1 | 1,3 | 1,0 | 0,9 | 0,7 | 1,1 | 1,0 | 1,3 | 1,0 | 1,0 | 1,0 | 1,0 | 1,1 | 1,0 | 0,9 | 0,8 |
| Kcnk16 | 1,0 | 1,0 | 1,0 | 1,0 | 1,0 | 1,0 | 1,0 | 1,0 | 1,0 | 1,0 | 1,0 | 1,0 | 1,8 | 0,3 | 1,0 | 1,0 | 1,0 | 1,0 | 1,0 | 1,0 | 1,0 | 1,0 | 1,0 | 1,0 |
| Ctrb1 | 0,2 | 0,3 | 1,0 | 1,0 | 1,0 | 0,6 | 1,0 | 1,0 | 1,0 | 1,0 | 0,7 | 0,7 | 1,0 | 1,0 | 1,0 | 1,0 | 1,0 | 1,0 | 1,0 | 1,0 | 1,0 | 1,0 | 1,0 | 1,0 |
| Kctd1 | 1,0 | 0,9 | 0,6 | 0,9 | 1,2 | 1,1 | 1,1 | 1,0 | 1,0 | 1,0 | 0,9 | 1,0 | 1,1 | 1,0 | 1,0 | 1,1 | 1,0 | 1,0 | 1,1 | 1,0 | 1,4 | 1,1 | 0,9 | 1,0 |
| Kdelr3 | 1,3 | 0,6 | 1,0 | 1,0 | 1,0 | 1,0 | 0,7 | 0,7 | 1,3 | 1,1 | 0,7 | 0,8 | 0,2 | 0,6 | 0,6 | 0,8 | 0,5 | 0,4 | 0,7 | 0,9 | 1,0 | 0,7 | 0,8 | 0,6 |
| Kel | 1,0 | 1,0 | 1,0 | 1,0 | 1,0 | 1,0 | 1,0 | 1,0 | 1,0 | 1,0 | 1,0 | 1,0 | 1,0 | 1,0 | 1,8 | 1,5 | 1,0 | 1,0 | 6,4 | 0,4 | 1,0 | 1,0 | 1,0 | 1,0 |
| Kera | 1,0 | 1,0 | 1,0 | 1,0 | 1,0 | 1,0 | 1,0 | 1,0 | 1,0 | 1,0 | 1,0 | 1,0 | 0,5 | 0,8 | 0,7 | 0,2 | 0,2 | 0,5 | 1,0 | 1,0 | 1,0 | 1,0 | 1,0 | 1,0 |
| Kif20b | 1,0 | 1,0 | 1,0 | 1,0 | 1,0 | 1,0 | 1,0 | 1,0 | 1,0 | 1,0 | 1,0 | 1,0 | 0,4 | 0,5 | 1,1 | 0,9 | 1,0 | 1,0 | 1,1 | 0,6 | 1,0 | 1,2 | 1,0 | 1,0 |
| Kiss1 | 1,0 | 1,0 | 1,0 | 1,0 | 1,0 | 1,0 | 1,0 | 0,6 | 1,0 | 1,0 | 1,0 | 1,0 | 1,0 | 1,0 | 1,0 | 1,0 | 1,0 | 1,0 | 1,0 | 1,0 | 1,0 | 0,4 | 1,0 | 1,0 |
| Klf1 | 1,0 | 1,0 | 1,0 | 1,0 | 1,0 | 1,0 | 1,0 | 1,0 | 1,0 | 1,0 | 1,3 | 0,8 | 1,0 | 1,0 | 2,0 | 1,5 | 1,0 | 1,0 | 6,2 | 0,2 | 1,0 | 1,0 | 1,0 | 1,0 |
| Klhl30 | 1,8 | 1,1 | 1,0 | 0,5 | 1,0 | 1,0 | 1,0 | 1,0 | 1,0 | 1,0 | 0,9 | 1,3 | 1,6 | 0,7 | 2,2 | 0,3 | 0,7 | 0,8 | 1,0 | 1,0 | 1,0 | 1,0 | 1,0 | 5,1 |
| Klhl31 | 1,0 | 1,0 | 1,0 | 1,0 | 1,0 | 1,0 | 1,0 | 1,0 | 1,0 | 1,0 | 1,3 | 0,9 | 1,9 | 0,7 | 4,6 | 0,2 | 0,9 | 0,8 | 1,0 | 1,0 | 1,0 | 1,0 | 1,0 | 11,1 |
| Klhl34 | 0,3 | 0,7 | 1,0 | 1,0 | 0,9 | 0,7 | 1,0 | 1,0 | 1,0 | 1,0 | 1,0 | 1,0 | 0,9 | 0,9 | 3,2 | 0,5 | 0,7 | 1,1 | 1,0 | 1,0 | 1,0 | 1,0 | 1,1 | 1,0 |
| Klhl40 | 1,0 | 1,0 | 1,0 | 1,0 | 1,7 | 1,5 | 1,0 | 1,0 | 1,0 | 1,0 | 1,0 | 1,0 | 1,9 | 0,8 | 3,4 | 0,3 | 0,8 | 1,0 | 1,0 | 1,0 | 1,0 | 1,0 | 1,3 | 9,7 |
| Klhl41 | 1,0 | 1,0 | 0,5 | 0,7 | 1,0 | 1,0 | 1,0 | 1,0 | 1,0 | 1,0 | 1,3 | 1,1 | 1,9 | 0,9 | 3,8 | 0,3 | 1,0 | 1,2 | 1,0 | 1,0 | 1,1 | 1,2 | 1,4 | 11,4 |
| Klhl6 | 1,1 | 0,9 | 1,2 | 1,1 | 1,0 | 1,0 | 1,0 | 1,0 | 1,0 | 1,0 | 0,7 | 1,1 | 1,0 | 1,1 | 0,8 | 1,0 | 0,8 | 1,0 | 1,0 | 1,0 | 1,0 | 1,0 | 0,6 | 1,0 |
| *Klk10* | 1,0 | 1,0 | 0,7 | 0,7 | 1,0 | 1,0 | 1,0 | 1,0 | 1,0 | 1,0 | 1,0 | 1,0 | 1,0 | 1,8 | 1,0 | 1,0 | 1,0 | 1,0 | 1,0 | 1,0 | 1,0 | 1,0 | 7,1 | 1,0 |
| Klk14 | 1,0 | 1,0 | 1,0 | 1,0 | 1,0 | 1,0 | 0,9 | 1,1 | 1,0 | 1,0 | 1,0 | 1,0 | 1,0 | 0,7 | 1,0 | 1,0 | 1,0 | 1,0 | 1,0 | 1,0 | 1,0 | 1,0 | 5,5 | 1,0 |
| Klk1b1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Klk1b21 | 1,0 | 1,0 | 1,0 | 1,0 | 1,0 | 1,0 | 1,0 | 1,0 | 0,8 | 0,8 | 1,8 | 1,6 | 1,0 | 1,0 | 1,0 | 1,0 | 1,0 | 1,0 | 1,0 | 1,0 | 0,1 | 0,2 | 1,0 | 1,0 |
| Klk1b24 | 1,0 | 1,0 | 1,0 | 1,0 | 1,0 | 1,0 | 1,0 | 1,0 | 0,4 | 0,5 | 1,0 | 1,0 | 1,0 | 1,0 | 1,0 | 1,0 | 1,0 | 1,0 | 1,0 | 1,0 | 0,2 | 0,2 | 1,0 | 1,0 |
| Klk1b26 | 0,3 | 1,0 | 1,0 | 0,3 | 0,9 | 1,0 | 1,3 | 0,5 | 1,2 | 1,0 | 1,2 | 1,1 | 1,0 | 1,0 | 2,8 | 1,0 | 1,0 | 0,2 | 1,0 | 1,0 | 1,0 | 1,1 | 1,9 | 4,5 |
| Klk1b27 | 1,0 | 1,0 | 1,0 | 1,0 | 1,0 | 1,0 | 1,0 | 1,0 | 0,8 | 0,5 | 1,1 | 1,6 | 1,0 | 1,0 | 1,4 | 1,8 | 1,0 | 1,0 | 1,1 | 0,6 | 0,2 | 0,3 | 1,1 | 1,0 |
| Klk1b7-ps | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,6 | 0,9 | 1,0 | 1,0 |
| Klk1b8 | 1,0 | 1,0 | 1,0 | 1,0 | 1,0 | 1,0 | 1,0 | 0,8 | 1,0 | 0,7 | 1,0 | 1,0 | 1,0 | 1,0 | 1,0 | 1,0 | 1,0 | 1,0 | 1,0 | 1,0 | 1,1 | 1,2 | 1,0 | 1,0 |
| Klk1b9 | 0,8 | 1,0 | 1,0 | 0,9 | 0,7 | 1,0 | 1,0 | 1,4 | 1,7 | 1,0 | 1,0 | 1,0 | 1,0 | 1,0 | 1,0 | 1,0 | 1,0 | 0,4 | 1,0 | 1,0 | 1,1 | 1,2 | 1,8 | 0,6 |
| Klrb1a | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,5 | 0,6 | 1,0 | 1,0 | 1,0 | 1,0 |
| Klrb1c | 1,0 | 0,9 | 1,0 | 1,0 | 1,0 | 1,0 | 0,6 | 0,8 | 1,0 | 1,0 | 1,0 | 1,0 | 1,0 | 1,0 | 0,9 | 1,8 | 1,0 | 1,0 | 0,8 | 1,0 | 1,0 | 1,0 | 1,0 | 1,0 |
| Kng1 | 1,0 | 1,0 | 1,5 | 1,0 | 1,0 | 1,0 | 1,0 | 1,0 | 1,0 | 1,0 | 1,0 | 1,0 | 1,0 | 1,0 | 1,2 | 2,1 | 1,0 | 1,0 | 1,0 | 1,0 | 1,0 | 1,0 | 1,0 | 1,0 |
| *Krt13* | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 167,0 | 1,0 |
| Krt14 | 1,0 | 1,0 | 0,9 | 1,0 | 1,0 | 1,0 | 1,0 | 1,0 | 1,0 | 1,0 | 1,0 | 1,0 | 1,0 | 1,5 | 1,0 | 1,0 | 1,0 | 1,0 | 1,0 | 1,0 | 1,0 | 1,0 | 2,4 | 1,0 |
| Krt16 | 1,0 | 1,0 | 1,0 | 1,0 | 1,0 | 1,0 | 1,0 | 1,0 | 1,0 | 1,0 | 1,0 | 1,0 | 0,9 | 2,1 | 1,0 | 1,0 | 1,0 | 1,0 | 1,0 | 1,0 | 1,0 | 1,0 | 1,0 | 1,0 |
| Krt18 | 1,0 | 1,0 | 1,1 | 1,5 | 1,0 | 1,4 | 1,1 | 1,1 | 1,1 | 1,2 | 1,0 | 1,2 | 1,0 | 0,7 | 1,3 | 1,9 | 1,0 | 1,0 | 1,0 | 1,0 | 1,0 | 1,0 | 1,2 | 1,2 |
| Krt23 | 1,0 | 1,0 | 1,0 | 1,0 | 1,0 | 1,0 | 0,7 | 0,8 | 0,9 | 1,2 | 1,0 | 1,0 | 1,5 | 0,9 | 1,0 | 1,0 | 1,0 | 1,0 | 1,0 | 1,0 | 1,0 | 1,0 | 0,9 | 1,0 |
| Krt4 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 7,2 | 1,0 |
| Krt5 | 1,0 | 1,0 | 1,0 | 1,0 | 1,0 | 1,0 | 1,0 | 0,9 | 1,0 | 1,0 | 1,0 | 1,0 | 1,0 | 1,1 | 1,0 | 1,0 | 1,0 | 1,0 | 1,0 | 1,0 | 1,0 | 1,0 | 2,0 | 1,8 |
| Krt6a | 1,0 | 1,0 | 1,0 | 1,0 | 1,0 | 1,0 | 1,0 | 1,0 | 1,0 | 1,0 | 1,0 | 1,0 | 1,3 | 1,9 | 1,0 | 1,0 | 1,0 | 1,0 | 1,0 | 1,0 | 1,4 | 1,1 | 1,0 | 1,0 |
| Krt78 | 1,0 | 1,0 | 1,0 | 1,0 | 1,0 | 1,0 | 1,0 | 1,0 | 1,0 | 1,0 | 1,0 | 1,0 | 1,1 | 1,5 | 1,0 | 1,0 | 1,0 | 1,0 | 1,0 | 1,0 | 1,0 | 1,0 | 5,8 | 1,0 |
| Krtap16-1 | 1,0 | 1,0 | 1,0 | 1,0 | 1,0 | 1,0 | 1,0 | 1,0 | 1,0 | 1,0 | 1,0 | 1,0 | 2,5 | 0,3 | 1,0 | 1,0 | 1,0 | 1,0 | 1,0 | 1,0 | 1,0 | 1,0 | 1,0 | 1,0 |
| Krtap19-1 | 1,0 | 1,0 | 1,0 | 1,0 | 1,0 | 1,0 | 1,0 | 1,0 | 1,0 | 1,0 | 1,0 | 1,0 | 2,0 | 0,2 | 1,0 | 1,0 | 1,0 | 1,0 | 1,0 | 1,0 | 1,0 | 1,0 | 1,0 | 1,0 |
| Krtap19-4 | 1,0 | 1,0 | 1,0 | 1,0 | 1,0 | 1,0 | 1,0 | 1,0 | 1,0 | 1,0 | 1,0 | 1,0 | 1,9 | 0,3 | 1,0 | 1,0 | 1,0 | 1,0 | 1,0 | 1,0 | 1,0 | 1,0 | 1,0 | 1,0 |

[Fig. 23-8]



| | | | | | | | | | | | | | | | | | | | | | | | | |
|---|---|---|---|---|---|---|---|---|---|---|---|---|---|---|---|---|---|---|---|---|---|---|---|---|
| Krtap5-2 | 1,0 | 1,0 | 1,0 | 1,0 | 1,0 | 1,0 | 1,0 | 1,0 | 1,0 | 1,0 | 1,0 | 1,0 | 0,2 | 0,4 | 1,0 | 1,0 | 1,0 | 1,0 | 1,0 | 1,0 | 1,0 | 1,0 | 1,0 | 1,0 |
| Krtdap | 1,0 | 1,0 | 1,0 | 0,9 | 1,0 | 1,0 | 1,0 | 1,0 | 1,0 | 1,0 | 1,0 | 1,0 | 1,1 | 1,4 | 1,0 | 1,0 | 1,0 | 1,4 | 1,0 | 1,0 | 1,0 | 1,0 | 8,8 | 1,0 |
| Ky | 1,0 | 1,0 | 1,0 | 1,0 | 0,9 | 0,8 | 0,8 | 1,4 | 1,0 | 1,0 | 1,1 | 1,1 | 1,3 | 0,9 | 2,5 | 0,1 | 0,6 | 0,9 | 1,0 | 1,0 | 1,0 | 1,0 | 0,9 | 1,8 |
| Kynu | 1,0 | 1,0 | 1,0 | 1,0 | 1,0 | 1,0 | 0,9 | 1,0 | 1,0 | 1,0 | 1,0 | 1,0 | 1,0 | 1,0 | 1,0 | 1,0 | 1,0 | 1,0 | 0,9 | 0,9 | 1,0 | 1,0 | 1,0 | 1,0 |
| Lad1 | 1,0 | 1,0 | 1,0 | 1,0 | 1,1 | 1,0 | 0,8 | 1,0 | 0,8 | 1,1 | 1,0 | 0,9 | 1,2 | 0,9 | 1,0 | 1,0 | 1,0 | 1,0 | 0,7 | 0,9 | 1,0 | 1,0 | 1,4 | 1,0 |
| Lars2 | 0,8 | 1,2 | 0,8 | 1,2 | 0,9 | 1,3 | 0,9 | 0,2 | 0,9 | 1,2 | 0,9 | 1,1 | 0,9 | 0,9 | 2,3 | 2,8 | 1,3 | 0,3 | 0,8 | 1,0 | 1,4 | 1,3 | 0,9 | 1,4 |
| | AG | | Aorta | | Brain | | Lung | | Pancreas | | PG | | Skin | | Skull | | SM | | Spleen | | Testis | | TG | |
| | E | M | E | M | E | M | E | M | E | M | E | M | E | M | E | M | E | M | E | M | E | M | E | M |
| Lbx1 | 1,0 | 1,0 | 1,0 | 1,0 | 1,2 | 0,7 | 1,0 | 1,0 | 1,0 | 1,0 | 1,0 | 1,0 | 2,1 | 1,6 | 1,0 | 1,0 | 0,8 | 1,2 | 1,0 | 1,0 | 1,0 | 1,0 | 0,6 | 5,2 |
| Lce3a | 1,0 | 1,0 | 1,0 | 1,0 | 1,0 | 1,0 | 1,0 | 1,0 | 1,0 | 1,0 | 1,0 | 1,0 | 1,4 | 1,0 | 1,0 | 1,0 | 1,0 | 1,0 | 1,0 | 1,0 | 1,0 | 1,0 | 30,9 | 1,0 |
| Lce3b | 1,0 | 1,0 | 1,0 | 1,0 | 1,0 | 1,0 | 1,0 | 1,0 | 1,0 | 1,0 | 1,0 | 1,0 | 1,0 | 1,2 | 1,0 | 1,0 | 1,0 | 1,0 | 1,0 | 1,0 | 1,0 | 1,0 | 19,6 | 1,0 |
| Lce3c | 1,0 | 1,0 | 1,0 | 1,0 | 1,0 | 1,0 | 1,0 | 1,0 | 1,0 | 1,0 | 1,0 | 1,0 | 2,3 | 0,6 | 1,0 | 1,0 | 1,0 | 1,0 | 1,0 | 1,0 | 1,0 | 1,0 | 14,2 | 1,0 |
| Lce3e | 1,0 | 1,0 | 1,0 | 1,0 | 1,0 | 1,0 | 1,0 | 1,0 | 1,0 | 1,0 | 1,0 | 1,0 | 0,7 | 3,1 | 1,0 | 1,0 | 1,0 | 1,0 | 1,0 | 1,0 | 1,0 | 1,0 | 11,5 | 1,0 |
| Lce3f | 1,0 | 1,0 | 1,0 | 1,0 | 1,0 | 1,0 | 1,0 | 1,0 | 1,0 | 1,0 | 1,0 | 1,0 | 0,7 | 3,7 | 1,0 | 1,0 | 1,0 | 1,0 | 1,0 | 1,0 | 1,0 | 1,0 | 21,0 | 1,0 |
| Lck | 1,0 | 0,8 | 1,0 | 1,0 | 1,0 | 1,0 | 0,6 | 0,7 | 1,0 | 1,0 | 1,2 | 1,1 | 1,1 | 0,8 | 1,1 | 1,8 | 1,0 | 1,0 | 1,0 | 0,9 | 1,0 | 1,0 | 0,0 | 0,6 |
| Lcn10 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Lcn12 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Lcn2 | 1,2 | 1,0 | 1,9 | 1,0 | 1,1 | 1,0 | 1,8 | 1,8 | 0,8 | 1,0 | 1,3 | 0,7 | 3,5 | 3,0 | 0,9 | 1,1 | 1,5 | 1,7 | 1,4 | 0,9 | 0,8 | 0,7 | 0,9 | 1,7 |
| Lcn5 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Lcn6 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Lcn8 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,8 |
| Lcn9 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Lctl | 0,5 | 0,5 | 0,7 | 0,7 | 1,0 | 1,0 | 1,0 | 0,9 | 1,0 | 1,0 | 1,0 | 1,0 | 1,0 | 1,0 | 1,2 | 1,4 | 1,0 | 0,9 | 1,0 | 1,0 | 1,0 | 1,0 | 1,0 | 1,1 |
| Ldb3 | 1,0 | 1,0 | 1,3 | 0,9 | 0,9 | 0,7 | 0,7 | 0,8 | 1,0 | 1,0 | 0,8 | 1,2 | 1,9 | 0,8 | 3,4 | 0,3 | 0,9 | 1,0 | 1,0 | 1,0 | 1,0 | 1,0 | 1,2 | 11,1 |
| Lect1 | 1,0 | 1,0 | 1,0 | 1,0 | 1,0 | 0,9 | 1,0 | 1,0 | 1,0 | 1,0 | 1,0 | 1,0 | 1,0 | 1,0 | 0,5 | 0,5 | 1,0 | 1,0 | 1,0 | 1,0 | 1,6 | 1,4 | 1,0 | 0,7 |
| Lef1 | 0,9 | 1,1 | 1,0 | 1,0 | 0,9 | 1,0 | 0,9 | 0,9 | 1,0 | 1,0 | 1,0 | 1,0 | 0,5 | 0,5 | 0,9 | 1,2 | 1,0 | 1,0 | 1,2 | 1,1 | 1,0 | 1,0 | 0,5 | 1,0 |
| Lelp1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,1 | 0,9 | 1,0 | 1,0 |
| Lgals7 | 1,0 | 1,0 | 0,9 | 1,1 | 1,0 | 1,0 | 1,0 | 1,0 | 1,0 | 1,0 | 0,5 | 0,7 | 1,0 | 1,2 | 1,0 | 1,0 | 1,0 | 1,0 | 1,0 | 1,0 | 1,0 | 1,1 | 9,6 | 1,0 |
| Lhfpl2 | 0,7 | 0,8 | 1,0 | 0,7 | 0,8 | 0,8 | 0,9 | 1,1 | 1,0 | 1,0 | 0,9 | 0,7 | 0,8 | 1,0 | 0,9 | 0,7 | 0,6 | 1,0 | 1,0 | 0,6 | 1,1 | 0,9 | 0,9 | 0,5 |
| Lingo1 | 0,8 | 1,2 | 0,7 | 0,7 | 0,9 | 1,0 | 0,6 | 1,5 | 1,0 | 1,0 | 1,3 | 1,1 | 0,7 | 0,9 | 0,7 | 0,8 | 1,0 | 1,0 | 0,8 | 1,1 | 1,2 | 0,6 | 1,0 | 1,0 |
| Lipf | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,8 | 0,9 | 0,0 | 0,0 |
| Lipg | 1,0 | 1,0 | 0,5 | 0,8 | 1,1 | 1,0 | 1,2 | 1,2 | 1,0 | 1,0 | 1,0 | 1,0 | 0,8 | 0,7 | 1,6 | 1,9 | 1,0 | 1,0 | 1,0 | 1,0 | 1,1 | 1,3 | 1,0 | 1,0 |
| Lipi | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Lmod2 | 1,0 | 1,0 | 1,0 | 0,9 | 1,0 | 1,0 | 0,9 | 0,7 | 1,0 | 1,0 | 1,0 | 1,0 | 2,3 | 0,8 | 5,7 | 0,3 | 1,0 | 1,4 | 1,0 | 1,0 | 1,0 | 1,0 | 1,3 | 4,3 |
| Lmod3 | 1,0 | 1,0 | 0,8 | 0,8 | 1,0 | 1,0 | 1,0 | 1,0 | 1,0 | 1,0 | 1,0 | 1,0 | 1,5 | 0,8 | 3,0 | 0,3 | 0,7 | 0,8 | 1,0 | 1,0 | 1,0 | 1,0 | 0,8 | 13,0 |
| Lor | 1,3 | 1,0 | 1,0 | 1,1 | 1,0 | 1,3 | 1,0 | 1,0 | 1,0 | 1,0 | 1,5 | 1,0 | 1,4 | 1,4 | 0,8 | 1,0 | 1,0 | 2,0 | 1,0 | 1,0 | 1,0 | 0,9 | 70,5 | 3,1 |
| Loxl2 | 0,9 | 1,0 | 0,9 | 0,7 | 1,0 | 1,0 | 0,8 | 1,0 | 1,0 | 1,0 | 0,8 | 0,8 | 0,3 | 0,7 | 0,6 | 0,5 | 0,2 | 0,4 | 1,1 | 1,0 | 1,0 | 1,0 | 0,5 | 0,8 |
| Lpl | 1,8 | 1,2 | 0,8 | 1,5 | 0,9 | 1,3 | 1,4 | 1,1 | 0,7 | 1,1 | 1,0 | 1,2 | 0,8 | 0,9 | 1,8 | 1,1 | 0,8 | 0,9 | 1,0 | 1,1 | 1,2 | 1,5 | 1,6 | 1,8 |
| Lrcol1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Lrrc16a | 0,8 | 0,9 | 0,9 | 1,0 | 1,1 | 0,8 | 1,1 | 1,2 | 1,0 | 1,0 | 1,3 | 1,0 | 1,0 | 0,8 | 1,0 | 1,0 | 1,0 | 1,0 | 0,9 | 1,0 | 1,0 | 1,0 | 0,9 | 0,9 |
| Lrrc26 | 1,0 | 1,0 | 1,0 | 1,0 | 1,0 | 1,0 | 1,2 | 1,4 | 1,0 | 0,7 | 0,6 | 0,6 | 1,0 | 1,0 | 1,0 | 1,0 | 1,0 | 1,0 | 1,0 | 1,0 | 0,7 | 1,1 | 0,2 | 0,7 |
| Lrrc30 | 1,0 | 1,0 | 1,0 | 1,0 | 1,0 | 1,0 | 1,0 | 1,0 | 1,0 | 1,0 | 1,0 | 1,0 | 5,4 | 1,0 | 5,6 | 0,2 | 1,7 | 1,0 | 1,0 | 1,0 | 1,0 | 1,0 | 3,0 | 4,5 |
| Lst1 | 0,9 | 0,4 | 0,9 | 1,1 | 1,1 | 2,4 | 2,0 | 0,8 | 1,0 | 1,0 | 1,0 | 1,1 | 0,6 | 0,7 | 0,5 | 1,4 | 1,0 | 0,7 | 0,8 | 1,0 | 1,0 | 1,0 | 1,0 | 0,5 |
| Ltb | 2,0 | 0,7 | 1,1 | 0,6 | 1,0 | 1,0 | 0,6 | 0,5 | 1,0 | 1,0 | 1,0 | 1,0 | 0,9 | 1,4 | 1,1 | 1,8 | 1,0 | 1,0 | 0,9 | 1,0 | 1,2 | 1,0 | 0,6 | 1,4 |
| Ltf | 1,0 | 1,0 | 1,0 | 1,0 | 1,0 | 1,0 | 1,2 | 1,1 | 1,0 | 1,0 | 1,0 | 1,0 | 1,0 | 1,0 | 0,7 | 1,0 | 1,0 | 1,0 | 0,9 | 0,4 | 1,0 | 1,0 | 0,0 | 0,2 |
| Ly6d | 0,7 | 0,4 | 2,6 | 0,7 | 1,0 | 1,0 | 0,5 | 0,4 | 1,0 | 1,0 | 1,0 | 0,8 | 1,3 | 1,5 | 0,7 | 1,1 | 1,0 | 1,0 | 0,8 | 0,9 | 0,8 | 0,6 | 1,7 | 1,0 |
| Ly6g5b | 1,0 | 1,0 | 1,0 | 1,0 | 1,0 | 1,0 | 0,9 | 1,2 | 1,0 | 1,0 | 1,0 | 1,0 | 2,0 | 1,3 | 0,7 | 1,2 | 1,0 | 1,0 | 0,8 | 1,4 | 1,0 | 0,8 | 1,0 | 1,0 |
| Ly6g5c | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Lypd8 | 1,0 | 1,0 | 1,0 | 1,0 | 3,2 | 1,0 | 1,0 | 1,0 | 0,7 | 1,0 | 1,0 | 1,0 | 1,4 | 1,0 | 1,0 | 1,0 | 3,5 | 1,9 | 0,7 | 1,0 | 1,0 | 0,6 | 1,0 | 1,0 |
| Lyz1 | 1,3 | 1,9 | 0,8 | 1,5 | 1,0 | 1,0 | 1,1 | 0,8 | 1,2 | 1,0 | 1,0 | 1,0 | 1,4 | 1,5 | 0,8 | 0,8 | 1,4 | 1,0 | 0,7 | 0,6 | 1,0 | 2,1 | 0,1 | 0,1 |
| Lyz2 | 1,0 | 0,5 | 1,2 | 1,4 | 0,7 | 1,0 | 1,2 | 1,0 | 0,9 | 1,2 | 0,7 | 0,7 | 0,9 | 1,4 | 0,8 | 0,9 | 1,0 | 0,7 | 0,8 | 0,9 | 0,9 | 0,9 | 0,1 | 0,8 |
| Mafa | 1,0 | 1,0 | 1,0 | 1,0 | 1,3 | 0,9 | 1,0 | 1,0 | 1,0 | 1,0 | 0,9 | 0,9 | 2,6 | 1,0 | 2,6 | 0,3 | 0,9 | 1,1 | 1,0 | 1,0 | 1,0 | 1,0 | 1,9 | 5,7 |
| Mafb | 0,7 | 0,8 | 1,2 | 1,9 | 1,0 | 0,7 | 0,9 | 0,8 | 1,0 | 1,0 | 1,1 | 1,1 | 0,6 | 0,9 | 1,1 | 0,8 | 0,3 | 1,8 | 1,2 | 1,4 | 1,0 | 0,8 | 4,1 | 2,7 |
| Mal | 0,9 | 1,0 | 0,9 | 1,2 | 1,1 | 0,9 | 1,0 | 1,2 | 1,0 | 1,0 | 1,2 | 1,2 | 1,3 | 0,9 | 0,8 | 0,6 | 0,6 | 0,9 | 1,0 | 1,8 | 0,6 | 1,2 | 0,9 | 0,8 |
| Malat1 | 0,7 | 1,1 | 1,4 | 1,9 | 0,9 | 1,4 | 1,0 | 0,5 | 1,0 | 0,8 | 1,2 | 1,1 | 1,1 | 0,9 | 1,2 | 1,3 | 0,9 | 0,4 | 0,9 | 1,0 | 1,0 | 1,1 | 1,2 | 1,2 |
| Map3k7cl | 1,0 | 1,0 | 1,4 | 0,8 | 1,0 | 1,0 | 1,1 | 1,0 | 1,0 | 1,0 | 1,0 | 1,0 | 1,4 | 0,6 | 0,8 | 0,1 | 0,3 | 0,4 | 0,7 | 1,4 | 0,8 | 1,0 | 1,0 | 1,1 |
| Mat1a | 1,0 | 0,7 | 1,0 | 0,5 | 1,0 | 1,0 | 1,2 | 1,0 | 2,1 | 1,9 | 1,1 | 1,2 | 1,0 | 1,0 | 1,0 | 1,0 | 1,0 | 1,0 | 1,0 | 1,0 | 1,0 | 1,0 | 1,9 | 1,0 |
| Mb | 5,2 | 1,0 | 0,8 | 0,9 | 1,0 | 1,0 | 0,6 | 0,6 | 1,0 | 1,0 | 1,0 | 1,0 | 1,2 | 0,6 | 5,0 | 0,4 | 0,7 | 0,7 | 1,3 | 1,0 | 1,3 | 1,0 | 1,2 | 6,3 |
| Mbnl3 | 1,0 | 0,8 | 1,0 | 1,0 | 1,0 | 1,0 | 1,1 | 0,9 | 1,0 | 0,9 | 1,2 | 1,0 | 0,9 | 1,2 | 1,0 | 0,9 | 1,0 | 1,0 | 1,0 | 0,8 | 1,0 | 1,0 | 1,0 | 1,0 |
| Mboat1 | 1,2 | 0,7 | 1,0 | 1,0 | 0,8 | 0,8 | 1,2 | 1,0 | 1,0 | 1,0 | 0,7 | 0,9 | 0,7 | 0,9 | 1,0 | 1,1 | 1,0 | 0,7 | 1,1 | 1,3 | 1,0 | 1,0 | 1,0 | 1,0 |
| Mcm10 | 1,0 | 0,9 | 1,0 | 1,0 | 1,0 | 1,0 | 1,0 | 1,0 | 1,0 | 1,0 | 1,0 | 1,0 | 0,4 | 0,6 | 1,0 | 1,2 | 1,0 | 1,0 | 1,5 | 0,4 | 1,3 | 1,2 | 1,0 | 1,0 |
| Mcpt1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Mcpt2 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Mdk | 1,0 | 0,8 | 0,9 | 1,0 | 1,2 | 1,0 | 0,9 | 0,9 | 1,0 | 1,0 | 1,1 | 1,1 | 2,2 | 4,5 | 0,7 | 1,0 | 1,0 | 0,6 | 0,7 | 1,1 | 1,3 | 1,3 | 0,6 | 1,8 |
| Mef2b | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,6 | 0,2 | 0,7 | 0,8 | 1,0 | 1,0 |
| Megf9 | 0,5 | 0,7 | 1,0 | 0,6 | 0,9 | 0,7 | 0,9 | 1,8 | 1,7 | 1,0 | 1,2 | 1,0 | 0,8 | 1,1 | 0,8 | 0,5 | 1,1 | 1,0 | 1,0 | 1,0 | 1,1 | 1,0 | 1,2 | 0,5 |
| Meox1 | 0,7 | 1,0 | 0,6 | 0,6 | 1,0 | 1,0 | 0,9 | 0,7 | 1,0 | 1,0 | 0,8 | 1,0 | 0,6 | 0,7 | 1,0 | 0,7 | 0,7 | 0,7 | 0,9 | 0,9 | 1,0 | 1,5 | 0,8 | 0,6 |
| Mesp1 | 1,0 | 1,0 | 1,0 | 1,0 | 1,0 | 1,0 | 1,9 | 0,7 | 1,0 | 1,0 | 1,0 | 1,0 | 1,6 | 1,5 | 1,0 | 0,8 | 1,0 | 1,0 | 1,0 | 1,0 | 1,2 | 0,8 | 4,2 | 6,8 |
| Mesp2 | 1,0 | 1,0 | 1,0 | 1,0 | 1,0 | 1,0 | 1,1 | 1,0 | 1,0 | 1,0 | 1,0 | 1,0 | 1,1 | 1,0 | 1,0 | 1,0 | 1,0 | 1,0 | 1,0 | 1,0 | 1,1 | 0,9 | 5,4 | 4,4 |
| Mest | 0,9 | 0,9 | 0,8 | 0,8 | 0,9 | 1,1 | 0,6 | 0,5 | 1,0 | 1,0 | 1,0 | 1,2 | 0,9 | 1,0 | 0,7 | 0,8 | 0,7 | 0,9 | 1,0 | 1,0 | 1,0 | 1,0 | 0,4 | 0,4 |
| Mettl21c | 1,0 | 1,0 | 1,0 | 1,0 | 1,0 | 1,0 | 1,0 | 1,0 | 1,0 | 1,0 | 1,0 | 1,0 | 0,7 | 0,7 | 1,3 | 0,2 | 0,3 | 0,4 | 1,0 | 1,0 | 1,0 | 1,0 | 0,1 | 2,3 |
| Mfrp | 1,0 | 1,0 | 1,0 | 1,0 | 1,0 | 1,0 | 1,0 | 1,0 | 1,0 | 1,0 | 1,0 | 1,0 | 1,0 | 1,0 | 0,6 | 1,0 | 1,0 | 1,0 | 1,0 | 1,0 | 1,0 | 1,0 | 1,0 | 1,0 |
| Mfsd2a | 1,0 | 1,0 | 1,1 | 1,9 | 0,9 | 1,3 | 2,1 | 2,2 | 1,0 | 1,0 | 1,2 | 1,1 | 0,6 | 0,7 | 1,0 | 1,0 | 1,0 | 1,0 | 1,0 | 1,0 | 0,8 | 1,0 | 1,9 | 2,4 |
| Mid1 | 0,3 | 0,8 | 0,0 | 0,5 | 0,5 | 0,6 | 0,4 | 0,7 | 0,3 | 0,9 | 0,3 | 0,7 | 0,3 | 0,6 | 0,1 | 0,6 | 0,5 | 1,3 | 0,0 | 0,6 | 0,4 | 0,9 | 0,1 | 0,9 |
| Amy2a5 | 0,1 | 1,4 | 0,5 | 1,0 | 0,3 | 1,6 | 0,6 | 0,3 | 1,1 | 0,6 | 0,5 | 1,3 | 0,2 | 0,9 | 2,1 | 2,9 | 4,7 | 0,3 | 0,4 | 0,7 | 1,1 | 1,2 | 2,7 | 0,8 |
| Kcnk9 | 0,2 | 1,0 | 1,0 | 6,0 | 1,0 | 1,0 | 1,0 | 1,0 | 1,0 | 1,0 | 1,0 | 1,2 | 1,0 | 1,0 | 1,0 | 1,0 | 1,0 | 1,0 | 1,0 | 1,0 | 15,0 | 0,2 | 1,0 | 1,0 |
| Mlf1 | 1,2 | 0,9 | 0,4 | 0,9 | 1,0 | 1,0 | 0,9 | 0,9 | 1,0 | 1,0 | 1,0 | 1,0 | 1,5 | 0,7 | 5,4 | 0,3 | 1,1 | 0,9 | 1,0 | 1,0 | 1,0 | 1,3 | 0,9 | 15,9 |
| Mmp12 | 0,8 | 0,4 | 1,0 | 1,0 | 1,0 | 1,0 | 1,0 | 1,1 | 1,0 | 1,0 | 1,0 | 1,0 | 1,6 | 5,1 | 1,0 | 1,0 | 1,0 | 1,0 | 1,0 | 0,9 | 1,0 | 1,0 | 1,0 | 1,0 |
| Mmp7 | 1,0 | 1,0 | 1,0 | 1,0 | 1,0 | 1,0 | 1,0 | 1,0 | 1,0 | 1,0 | 0,9 | 0,9 | 1,0 | 1,0 | 1,0 | 1,0 | 1,0 | 1,0 | 1,0 | 1,0 | 1,0 | 1,0 | 1,0 | 1,0 |
| Mptx1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 3,6 | 1,3 | 1,0 | 1,0 | 1,0 | 1,0 | 1,0 | 1,0 |
| Mptx2 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Mpz | 0,5 | 4,6 | 0,4 | 0,9 | 1,0 | 1,0 | 0,9 | 1,1 | 1,0 | 1,0 | 0,4 | 0,7 | 1,2 | 1,2 | 0,5 | 0,6 | 0,7 | 0,9 | 1,0 | 0,9 | 1,0 | 1,0 | 1,6 | 1,3 |
| Mrap | 1,8 | 1,6 | 0,7 | 0,9 | 1,0 | 1,0 | 0,5 | 0,2 | 1,0 | 1,0 | 1,0 | 1,0 | 1,0 | 1,4 | 1,6 | 2,5 | 0,8 | 0,4 | 1,9 | 2,1 | 0,7 | 0,6 | 1,1 | 1,0 |
| Ms4a1 | 1,1 | 1,0 | 1,7 | 1,0 | 1,0 | 1,0 | 0,5 | 0,5 | 1,0 | 1,0 | 1,0 | 1,0 | 1,0 | 1,0 | 1,1 | 1,5 | 1,0 | 1,0 | 0,8 | 0,8 | 1,0 | 1,0 | 1,0 | 1,0 |

[Fig. 23-9]

278/310

| | | | | | | | | | | | | | | | | | | | | | | | |
|---|---|---|---|---|---|---|---|---|---|---|---|---|---|---|---|---|---|---|---|---|---|---|---|
| Msln | 0,7 | 0,8 | 0,9 | 1,4 | 1,0 | 1,0 | 1,0 | 1,1 | 1,1 | 1,0 | 1,0 | 1,0 | 1,0 | 1,0 | 1,3 | 1,8 | 1,0 | 1,0 | 1,0 | 0,8 | 1,0 | 1,0 | 0,2 | 0,6 |
| MssS1 | 1,0 | 1,0 | 1,0 | 1,0 | 1,0 | 1,0 | 0,9 | 1,0 | 1,0 | 1,0 | 1,0 | 1,0 | 0,7 | 1,0 | 3,5 | 0,2 | 0,6 | 0,4 | 1,4 | 1,1 | 1,4 | 1,1 | 0,6 | 9,6 |
| Mstn | 1,0 | 1,0 | 1,0 | 1,0 | 1,0 | 1,0 | 1,0 | 1,0 | 1,0 | 1,0 | 1,0 | 1,0 | 1,8 | 0,7 | 2,7 | 0,4 | 1,3 | 1,0 | 1,0 | 1,0 | 1,0 | 1,0 | 1,6 | 5,3 |
| Msx1 | 0,6 | 1,1 | 0,6 | 0,9 | 1,3 | 0,9 | 0,7 | 0,7 | 1,0 | 1,0 | 1,2 | 1,0 | 0,3 | 0,5 | 0,9 | 0,9 | 0,8 | 0,5 | 1,3 | 0,9 | 1,0 | 1,0 | 0,8 | 0,7 |
| Mt1 | 1,2 | 1,1 | 1,0 | 1,4 | 1,4 | 1,4 | 1,7 | 1,5 | 0,5 | 0,7 | 1,2 | 1,2 | 1,2 | 1,0 | 1,3 | 1,9 | 1,3 | 2,1 | 1,3 | 1,1 | 1,1 | 0,7 | 0,9 | 0,9 |
| Mt2 | 1,2 | 0,8 | 0,8 | 2,1 | 1,2 | 1,4 | ·2,1 | 2,2 | 0,4 | 0,7 | 1,2 | 1,8 | 1,0 | 0,9 | 1,2 | 2,3 | 1,2 | 2,8 | 2,6 | 0,5 | 1,1 | 1,0 | 0,9 | 0,9 |
| Mt3 | 1,0 | 2,2 | 1,0 | 1,3 | 1,1 | 1,2 | 1,0 | 1,0 | 1,0 | 1,0 | 1,2 | 1,1 | 1,0 | 1,0 | 5,3 | 2,4 | 1,0 | 1,2 | 1,0 | 1,0 | 0,8 | 0,6 | 3,6 | 2,6 |
| Mt4 | 1,0 | 1,0 | 1,0 | 1,0 | 1,0 | 1,0 | 1,0 | 1,0 | 1,0 | 1,0 | 1,0 | 1,0 | 0,8 | 0,4 | 1,0 | 1,0 | 1,0 | 1,0 | 1,0 | 1,0 | 1,0 | 1,0 | 8,1 | 1,0 |
| Muc15 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Muc5b | 1,0 | 1,0 | 1,0 | 1,0 | 1,0 | 1,0 | 0,8 | 1,3 | 1,0 | 1,0 | 1,0 | 1,0 | 1,0 | 1,0 | 1,0 | 1,0 | 1,0 | 1,0 | 1,0 | 1,0 | 1,0 | 1,0 | 0,1 | 0,2 |
| Mug1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Mup1 | 1,0 | 0,3 | 1,0 | 0,2 | 1,0 | 1,0 | 1,0 | 1,0 | 1,0 | 1,0 | 1,0 | 1,0 | 0,7 | 1,1 | 1,0 | 1,0 | 1,0 | 1,0 | 1,0 | 1,0 | 1,0 | 1,0 | 1,0 | 1,0 |
| Mup10 | 1,0 | 0,6 | 0,6 | 0,1 | 1,0 | 1,0 | 1,0 | 1,0 | 1,0 | 1,0 | 1,0 | 1,0 | 0,8 | 1,0 | 1,0 | 1,0 | 1,2 | 0,6 | 1,0 | 1,0 | 1,0 | 1,0 | 1,0 | 1,0 |
| Mup11 | 1,0 | 1,2 | 1,0 | 1,0 | 1,0 | 1,0 | 1,0 | 1,0 | 1,0 | 1,0 | 1,0 | 1,0 | 2,1 | 1,0 | 1,0 | 1,0 | 1,0 | 1,0 | 1,0 | 1,0 | 1,0 | 1,0 | 1,0 | 1,0 |
| Mup12 | 1,0 | 0,3 | 0,9 | 0,1 | 1,0 | 1,0 | 1,0 | 1,0 | 1,0 | 1,0 | 1,0 | 1,0 | 0,7 | 0,9 | 1,0 | 1,0 | 0,7 | 1,0 | 1,0 | 1,0 | 1,0 | 1,0 | 1,0 | 1,0 |
| Mup13 | 1,0 | 0,4 | 0,8 | 0,1 | 1,0 | 1,0 | 1,2 | 1,0 | 1,0 | 1,0 | 1,0 | 1,0 | 1,0 | 0,8 | 1,0 | 1,0 | 0,5 | 1,0 | 1,0 | 1,0 | 1,0 | 1,0 | 1,0 | 1,0 |
| Mup14 | 1,0 | 0,1 | 0,9 | 0,1 | 1,0 | 1,0 | 2,0 | 1,0 | 1,0 | 1,0 | 1,0 | 1,0 | 0,3 | 0,5 | 1,0 | 1,0 | 1,0 | 1,0 | 1,0 | 1,0 | 1,0 | 1,0 | 1,0 | 1,0 |
| Mup15 | 1,0 | 0,3 | 0,6 | 0,2 | 1,0 | 1,0 | 1,7 | 1,0 | 1,0 | 1,0 | 1,0 | 1,0 | 0,7 | 1,4 | 1,0 | 1,0 | 0,6 | 0,9 | 1,0 | 1,0 | 1,0 | 1,0 | 1,0 | 1,0 |
| *Mup16* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* |
| Mup17 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Mup19 | 1,0 | 0,5 | 0,8 | 1,0 | 1,0 | 1,0 | 1,0 | 1,0 | 1,0 | 1,0 | 1,0 | 1,0 | 0,9 | 0,8 | 1,0 | 1,0 | 0,9 | 0,6 | 1,0 | 1,0 | 1,0 | 1,0 | 1,0 | 1,0 |
| | AG | | Aorta | | Brain | | Lung | | Pancreas | | PG | | Skin | | Skull | | SM | | Spleen | | Testis | | TG | |
| | E | M | E | M | E | M | E | M | E | M | E | M | E | M | E | M | E | M | E | M | E | M | E | M |
| Mup2 | 1,0 | 0,2 | 0,4 | 0,2 | 1,0 | 0,9 | 1,7 | 1,0 | 1,0 | 2,0 | 1,0 | 1,0 | 0,9 | 1,0 | 0,9 | 0,7 | 0,7 | 0,8 | 1,0 | 1,0 | 1,0 | 1,0 | 0,8 | 1,0 |
| Mup20 | 1,0 | 0,2 | 1,0 | 0,2 | 1,0 | 1,0 | 1,1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| *Mup21* | 1,0 | 1,0 | 1,0 | 0,2 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Mup3 | 1,0 | 0,2 | 1,0 | 0,2 | 1,0 | 1,0 | 1,0 | 1,0 | 1,0 | 1,3 | 1,0 | 1,0 | 0,6 | 1,0 | 1,0 | 1,0 | 1,0 | 1,0 | 1,0 | 1,0 | 1,0 | 1,0 | 1,0 | 1,0 |
| Mup5 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Mup7 | 1,0 | 0,1 | 1,0 | 0,1 | 1,0 | 1,1 | ·2,4 | 1,0 | 1,0 | 1,6 | 1,0 | 1,0 | 0,5 | 0,2 | 1,0 | 1,0 | 1,0 | 1,0 | 1,0 | 1,0 | 1,0 | 1,0 | 1,0 | 1,0 |
| Mup8 | 1,0 | 1,0 | 1,0 | 0,1 | 1,0 | 1,0 | 1,0 | 1,0 | 1,0 | 1,0 | 1,0 | 1,0 | 0,8 | 1,0 | 1,0 | 1,0 | 1,0 | 1,0 | 1,0 | 1,0 | 1,0 | 1,0 | 1,0 | 1,0 |
| Mup9 | 1,0 | 0,8 | 1,0 | 0,1 | 1,0 | 1,0 | 1,0 | 1,0 | 1,0 | 1,0 | 1,0 | 1,0 | 0,7 | 1,0 | 1,0 | 1,0 | 0,8 | 1,0 | 1,0 | 1,0 | 1,0 | 1,0 | 1,0 | 1,0 |
| Murc | 1,0 | 1,0 | 0,9 | 0,8 | 1,0 | 1,0 | 0,7 | 1,4 | 1,0 | 1,0 | 1,0 | 1,0 | 2,4 | 0,7 | 4,0 | 0,4 | 1,0 | 1,1 | 1,0 | 1,0 | 1,0 | 1,3 | 1,4 | 7,7 |
| Myadml2 | 1,0 | 1,0 | 1,1 | 0,8 | 0,8 | 0,9 | 0,9 | 1,0 | 1,0 | 1,0 | 0,8 | 0,8 | 1,9 | 0,7 | 2,9 | 0,3 | 0,8 | 1,0 | 0,7 | 1,0 | 1,0 | 1,0 | 1,1 | 7,0 |
| Myb | 1,0 | 1,0 | 1,0 | 1,0 | 1,0 | 1,0 | 0,8 | 0,9 | 1,0 | 1,0 | 0,9 | 1,0 | 0,7 | 0,8 | 0,8 | 0,8 | 1,0 | 1,0 | 1,0 | 0,7 | 1,0 | 1,0 | 0,1 | 1,0 |
| Mybl1 | 0,8 | 0,7 | 1,0 | 1,0 | 1,0 | 0,8 | 1,0 | 1,0 | 1,0 | 1,0 | 1,1 | 1,0 | 0,8 | 1,0 | 0,8 | 0,9 | 1,0 | 1,0 | 0,6 | 0,1 | 0,9 | 1,2 | 1,0 | 1,0 |
| Mybpc1 | 0,8 | 0,9 | 1,0 | 0,8 | 1,1 | 1,4 | 1,0 | 1,0 | 1,0 | 1,0 | 1,0 | 1,0 | 1,5 | 0,9 | 5,0 | 0,3 | 0,9 | 1,1 | 1,1 | 1,2 | 1,0 | 1,0 | 0,9 | 14,6 |
| Mybpc2 | 1,0 | 1,0 | 1,0 | 0,8 | 1,0 | 1,0 | 1,0 | 1,0 | 1,0 | 1,0 | 1,0 | 1,0 | 3,1 | 1,1 | 3,8 | 0,2 | 1,1 | 1,3 | 1,0 | 1,0 | 1,0 | 1,0 | 0,9 | 17,1 |
| Myf6 | 1,0 | 1,0 | 1,0 | 1,0 | 1,0 | 1,0 | 1,0 | 1,0 | 1,0 | 1,0 | 1,0 | 1,0 | 1,8 | 1,0 | 2,3 | 0,2 | 1,1 | 1,0 | 1,0 | 1,0 | 1,2 | 1,1 | 1,4 | 11,7 |
| Myh1 | 1,0 | 1,0 | 1,0 | 0,4 | 1,0 | 1,0 | 1,0 | 1,0 | 1,0 | 1,0 | 1,0 | 1,0 | 2,2 | 1,0 | 4,7 | 0,3 | 0,9 | 1,1 | 1,0 | 1,0 | 1,0 | 1,0 | 1,6 | 14,3 |
| Myh2 | 1,0 | 1,0 | 1,0 | 0,3 | 1,0 | 1,0 | 1,0 | 1,0 | 1,0 | 1,0 | 1,0 | 1,0 | 1,7 | 1,2 | 5,3 | 0,4 | 0,7 | 1,3 | 1,0 | 1,0 | 1,0 | 1,0 | 1,0 | 42,8 |
| Myh4 | 1,0 | 1,0 | 1,0 | 0,3 | 1,0 | 1,0 | 1,0 | 1,0 | 1,0 | 1,0 | 1,0 | 1,0 | 2,5 | 0,9 | 3,5 | 0,2 | 0,8 | 1,2 | 1,0 | 1,0 | 1,0 | 1,0 | 1,2 | 10,5 |
| Myh7 | 1,0 | 1,0 | 1,0 | 1,0 | 0,9 | 1,4 | 1,1 | 1,0 | 1,0 | 1,0 | 1,0 | 1,0 | 0,3 | 1,2 | 5,2 | 0,9 | 1,2 | 3,8 | 1,0 | 1,0 | 1,0 | 1,0 | 1,0 | 1,0 |
| Myh8 | 1,0 | 1,0 | 1,0 | .1,0 | 1,0 | 1,0 | 1,0 | 1,0 | 1,0 | 1,0 | 1,0 | 1,0 | 2,2 | 1,0 | 3,7 | 0,4 | 0,8 | 1,4 | 1,0 | 1,0 | 1,0 | 1,0 | 7,0 | 6,6 |
| *Myl1* | *1,1* | *1,3* | *0,6* | *0,7* | *1,0* | *1,0* | *0,8* | *0,5* | *1,0* | *1,0* | *1,0* | *1,0* | *1,1* | *0,4* | *2,6* | *0,2* | *0,6* | *0,6* | *1,0* | *1,0* | *1,0* | *1,0* | *0,6* | *6,4* |
| Myl3 | 1,0 | 1,0 | 1,0 | 0,6 | 1,0 | 1,0 | 0,4 | 0,6 | 1,0 | 1,0 | 1,0 | 1,0 | 0,3 | 1,0 | 4,8 | 1,0 | 0,8 | 2,3 | 1,0 | 1,0 | 1,0 | 1,0 | 16,4 | 1,0 |
| Myl6b | 0,6 | 0,8 | 0,5 | 0,5 | 0,9 | 1,0 | 1,0 | 1,0 | 1,0 | 1,0 | 1,0 | 0,8 | 0,7 | 0,5 | 5,4 | 0,6 | 0,8 | 0,8 | 1,0 | 0,7 | 1,2 | 1,2 | 1,0 | 1,1 |
| Mylk2 | 1,0 | 1,0 | 1,5 | 1,1 | 1,0 | 1,0 | 1,0 | 1,0 | 1,0 | 1,0 | 1,0 | 1,0 | 3,2 | 1,1 | 4,3 | 0,3 | 1,5 | 1,2 | 1,0 | 1,0 | 1,0 | 1,0 | 1,6 | 32,7 |
| Mylk4 | 1,0 | 1,0 | 0,9 | 0,8 | 1,0 | 1,0 | 0,6 | 1,0 | 1,0 | 1,0 | 1,0 | 1,0 | 2,0 | 0,9 | 3,6 | 0,2 | 1,0 | 1,1 | 1,0 | 1,0 | 1,0 | 1,0 | 1,0 | 17,4 |
| Mylpf | 1,0 | 1,0 | 1,0 | 0,3 | 1,0 | 1,0 | 1,0 | 0,8 | 1,0 | 1,0 | 1,0 | 1,0 | 2,1 | 0,9 | 3,3 | 0,3 | 1,0 | 1,1 | 0,6 | 0,7 | 1,0 | 1,0 | 1,1 | 13,6 |
| Myo18b | 1,0 | 1,0 | 1,0 | 0,9 | 1,0 | 1,0 | 1,0 | 1,0 | 1,0 | 1,0 | 1,0 | 1,0 | 2,5 | 1,0 | 3,8 | 0,3 | 1,2 | 1,5 | 1,0 | 1,0 | 1,0 | 0,9 | 1,4 | 7,2 |
| *Myo3b* | *1,0* | *1,0* | *1,0* | *1,0* | *1,0* | *1,0* | *1,0* | *1,0* | *1,0* | *1,0* | *0,7* | *0,8* | *1,0* | *1,0* | *1,0* | *1,0* | *1,0* | *1,0* | *1,0* | *1,0* | *1,0* | *1,0* | *0,7* | *0,8* |
| Myo5b | 0,8 | 0,8 | 0,5 | 0,7 | 1,1 | 0,9 | 1,2 | 1,3 | 1,0 | 0,8 | 1,1 | 1,1 | 1,0 | 1,2 | 1,0 | 1,0 | 1,0 | 1,0 | 1,0 | 1,0 | 1,0 | 0,9 | 1,1 | 0,7 |
| Myom1 | 1,0 | 1,0 | 1,3 | 1,1 | 1,0 | 1,0 | 0,9 | 1,0 | 1,0 | 1,0 | 1,0 | 1,0 | 2,4 | 1,1 | 3,1 | 0,4 | 1,5 | 1,4 | 0,8 | 1,2 | 1,0 | 1,0 | 1,9 | 7,7 |
| Myom2 | 1,0 | 1,0 | 1,3 | 1,5 | 1,0 | 1,0 | 1,1 | 0,9 | 1,0 | 1,0 | 1,0 | 1,0 | 2,1 | 0,9 | 3,3 | 0,3 | 1,0 | 1,0 | 1,0 | 1,0 | 1,0 | 1,0 | 1,1 | 17,0 |
| Myot | 1,2 | 1,2 | 1,0 | 0,5 | 1,0 | 1,0 | 1,0 | 1,0 | 1,0 | 1,0 | 1,0 | 1,0 | 2,6 | 1,1 | 4,7 | 0,3 | 1,2 | 1,2 | 1,0 | 1,0 | 1,0 | 1,0 | 1,5 | 17,1 |
| Myoz1 | 1,0 | 1,0 | 1,0 | 0,5 | 1,0 | 1,0 | 1,0 | 1,0 | 1,0 | 1,0 | 1,0 | 1,0 | 2,9 | 1,0 | 4,0 | 0,3 | 1,2 | 1,1 | 1,0 | 1,0 | 1,0 | 1,0 | 1,1 | 19,8 |
| Myoz2 | 1,0 | 1,0 | 1,1 | 1,2 | 1,0 | 1,0 | 0,7 | 0,7 | 1,0 | 1,0 | 1,0 | 1,0 | 1,7 | 1,1 | 5,2 | 0,5 | 1,2 | 1,5 | 1,0 | 1,0 | 1,2 | 1,0 | 1,6 | 11,2 |
| Myoz3 | 1,0 | 1,0 | 1,0 | 1,0 | 1,0 | 1,0 | 1,0 | 1,0 | 1,0 | 1,0 | 1,0 | 1,0 | 1,4 | 0,6 | 3,5 | 0,2 | 0,7 | 0,7 | 1,0 | 1,0 | 1,0 | 1,0 | 1,3 | 3,9 |
| Mypn | 1,0 | 1,0 | 1,0 | 1,0 | 1,0 | 1,0 | 1,0 | 1,0 | 1,0 | 1,0 | 1,0 | 1,0 | 2,5 | 1,1 | 3,9 | 0,3 | 1,2 | 1,4 | 1,0 | 1,0 | 1,0 | 1,0 | 1,4 | 8,3 |
| Mzb1 | 2,4 | 1,8 | 1,0 | 1,0 | 1,0 | 1,0 | 0,5 | 0,4 | 1,0 | 1,0 | 1,0 | 1,0 | 1,0 | 1,0 | 0,6 | 0,8 | 1,3 | 1,0 | 0,9 | 0,6 | 0,6 | 0,9 | 0,7 | 1,1 |
| N4bp2 | 1,0 | 1,0 | 1,1 | 1,0 | 0,7 | 1,0 | 0,8 | 1,0 | 1,0 | 1,0 | 0,9 | 0,8 | 0,6 | 0,8 | 1,0 | 0,6 | 1,0 | 1,0 | 0,9 | 0,8 | 1,0 | 1,1 | 1,1 | 0,7 |
| Naip7 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Nat8 | 0,9 | 0,9 | 1,0 | 1,0 | 0,8 | 1,0 | 1,0 | 0,6 | 0,8 | 0,4 | 1,2 | 1,0 | 0,8 | 1,5 | 1,0 | 1,0 | 1,0 | 1,0 | 1,0 | 1,0 | 0,6 | 0,5 | 1,0 | 1,0 |
| Nav2 | 0,9 | 1,0 | 1,3 | 0,8 | 1,0 | 0,9 | 0,9 | 1,2 | 1,0 | 1,0 | 1,0 | 0,9 | 0,9 | 0,6 | 1,1 | 0,7 | 0,7 | 1,1 | 1,1 | 1,0 | 0,9 | 0,8 | 0,9 | 0,7 |
| Ncmap | 1,0 | 1,0 | 1,0 | 1,0 | 1,1 | 1,2 | 1,4 | 1,2 | 1,0 | 1,0 | 0,8 | 1,5 | 1,6 | 1,8 | 0,7 | 1,0 | 0,9 | 0,8 | 1,0 | 0,9 | 1,1 | 0,9 | 1,4 | 1,0 |
| Nctc1 | 1,0 | 1,0 | 1,0 | -1,0 | 1,0 | 1,0 | 1,0 | 1,0 | 1,0 | 1,0 | 1,0 | 1,0 | 2,2 | 1,0 | 5,1 | 0,4 | 1,3 | 1,0 | 1,0 | 1,0 | 1,0 | 1,0 | 1,4 | 9,8 |
| Neat1 | 0,9 | 1,0 | 1,0 | 1,1 | 1,1 | 0,8 | 1,2 | 1,0 | 0,6 | 0,6 | 1,3 | 1,1 | 1,3 | 1,0 | 0,9 | 1,0 | 1,0 | 0,7 | 1,0 | 1,0 | 1,1 | 0,8 | 1,1 | 1,2 |
| Neb | 1,0 | 1,0 | 1,0 | 1,0 | 1,0 | 1,0 | 1,0 | 1,0 | 1,0 | 1,0 | 1,0 | 1,0 | 2,2 | 1,0 | 3,6 | 0,3 | 1,1 | 1,2 | 1,0 | 1,0 | 1,0 | 1,0 | 1,2 | 8,7 |
| *Nell1* | *1,0* | *1,0* | *1,0* | *1,0* | *0,9* | *1,1* | *1,0* | *1,0* | *1,0* | *1,0* | *1,0* | *1,4* | *1,5* | *1,1* | *1,0* | *1,0* | *1,0* | *1,0* | *1,0* | *1,0* | *1,0* | *1,0* | *5,7* | *5,3* |
| Nexn | 1,0 | 0,9 | 1,0 | 0,9 | 0,8 | 1,3 | 0,9 | 1,1 | 1,0 | 1,0 | 1,1 | 1,1 | 1,7 | 1,0 | 2,7 | 0,4 | 0,8 | 0,8 | 1,0 | 1,1 | 1,0 | 1,4 | 1,0 | 5,8 |
| Nfe2l3 | 1,0 | 1,0 | 0,8 | 1,1 | 0,9 | 1,1 | 1,1 | 0,8 | 1,0 | 1,0 | 1,0 | 1,0 | 1,2 | 0,9 | 1,0 | 1,0 | 1,0 | 1,0 | 0,7 | 0,7 | 0,8 | 0,9 | 1,5 | 0,9 |
| Ngfr | 0,8 | 4,2 | 0,8 | 0,5 | 0,8 | 0,7 | 1,0 | 1,0 | 1,0 | 1,0 | 1,1 | 1,7 | 1,1 | 1,4 | 0,8 | 0,7 | 1,0 | 1,3 | 0,9 | 1,1 | 1,1 | 1,0 | 6,9 | 26,1 |
| Nhsl1 | 0,7 | 0,8 | 1,3 | 0,7 | 0,8 | 0,9 | 0,8 | 1,1 | 1,4 | 1,1 | 1,0 | 1,0 | 0,9 | 1,0 | 0,7 | 0,8 | 0,9 | 1,4 | 0,8 | 0,9 | 0,9 | 0,9 | 0,8 | 0,8 |
| Nkx3-1 | 1,3 | 1,2 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,9 | 1,2 | 0,1 | 0,7 |
| Nmrk2 | 1,0 | 1,0 | 1,0 | 1,0 | 1,0 | 1,0 | 1,0 | 1,0 | 1,0 | 1,0 | 1,0 | 1,0 | 0,7 | 1,0 | 1,2 | 1,0 | 0,6 | 0,2 | 1,0 | 1,0 | 1,0 | 1,0 | 1,0 | 1,0 |
| *Nnat* | *1,2* | *1,1* | *2,4* | *2,6* | *1,0* | *1,2* | *0,8* | *0,8* | *0,5* | *0,5* | *0,8* | *0,8* | *0,5* | *1,5* | *0,9* | *1,7* | *0,5* | *0,7* | *1,4* | *1,2* | *1,0* | *0,8* | *0,8* | *0,9* |
| Nos2 | 1,0 | 1,0 | 0,8 | 0,7 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Scd3 | 0,2 | 0,9 | 1,5 | 0,6 | 0,9 | 0,7 | 0,9 | 0,8 | 1,0 | 1,0 | 0,8 | 0,8 | 1,2 | 1,3 | 0,8 | 0,4 | 1,0 | 1,0 | 2,6 | 1,5 | 1,0 | 1,0 | 0,9 | 0,8 |
| Nr1d1 | 0,5 | 1,4 | 0,5 | 0,9 | 1,0 | 1,0 | 0,5 | 1,2 | 0,3 | 0,8 | 0,8 | 1,2 | 0,7 | 1,4 | 0,6 | 0,7 | 0,6 | 1,1 | 0,9 | 1,5 | 0,9 | 0,8 | 0,6 | 1,3 |
| Nr4a1 | 1,4 | 1,2 | 1,8 | 1,0 | 1,2 | 0,8 | 1,2 | 0,7 | 6,0 | 1,2 | 1,3 | 1,6 | 1,7 | 0,8 | 1,0 | 0,7 | 1,0 | 1,1 | 1,0 | 1,1 | 1,0 | 0,7 | 1,0 | 1,4 |
| Nrap | 1,0 | 1,0 | 1,0 | 0,8 | 1,0 | 1,0 | 1,0 | 1,0 | 1,0 | 1,0 | 1,0 | 1,0 | 2,9 | 1,0 | 4,0 | 0,3 | 1,1 | 1,4 | 1,0 | 1,0 | 1,0 | 1,0 | 1,7 | 13,3 |
| Nrep | 0,8 | 1,0 | 0,6 | 0,6 | 0,9 | 0,9 | 0,6 | 0,7 | 0,7 | 0,8 | 0,8 | 0,7 | 0,4 | 0,7 | 0,9 | 0,5 | 0,3 | 0,3 | 0,7 | 0,8 | 1,1 | 1,3 | 0,4 | 1,2 |
| Nrip1 | 0,3 | 0,7 | 1,2 | 1,3 | 0,9 | 0,7 | 0,8 | 1,6 | 1,0 | 1,0 | 1,2 | 1,3 | 0,8 | 1,0 | 1,3 | 0,6 | 0,9 | 1,9 | 1,0 | 1,1 | 0,9 | 1,0 | 1,2 | 0,8 |
| Ntsr2 | 1,0 | 1,0 | 0,7 | 0,8 | 1,0 | 1,0 | 1,0 | 1,0 | 1,0 | 1,0 | 1,6 | 1,5 | 1,0 | 1,0 | 1,0 | 1,0 | 0,7 | 1,2 | 1,0 | 1,0 | 1,0 | 1,0 | 1,1 | 1,0 |

[Fig. 23-10]

279/310

| | | | | | | | | | | | | | | | | | | | | | | | | |
|---|---|---|---|---|---|---|---|---|---|---|---|---|---|---|---|---|---|---|---|---|---|---|---|---|
| Nudt7 | 1,1 | 1,2 | 0,6 | 0,8 | 1,1 | 1,1 | 0,9 | 0,8 | 1,1 | 0,9 | 0,7 | 1,2 | 1,0 | 1,4 | 1,0 | 0,9 | 0,6 | 0,6 | 1,0 | 1,1 | 0,7 | 1,2 | 1,1 | 1,4 |
| Nuggc | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,6 | 0,2 | 1,4 | 0,9 | 1,0 | 0,9 |
| Nupr1l | 1,1 | 1,1 | 1,4 | 1,4 | 1,0 | 1,9 | 1,0 | 0,9 | 1,0 | 1,0 | 0,9 | 1,0 | 1,3 | 1,6 | 1,0 | 1,1 | 1,0 | 1,0 | 0,5 | 1,9 | 1,1 | 0,7 | 1,2 | 1,1 |
| Nutf2-ps1 | 1,6 | 4,3 | 1,0 | 1,1 | 1,0 | 1,0 | 1,3 | 2,0 | 0,8 | 1,3 | 0,5 | 1,0 | 1,2 | 0,3 | 1,4 | 1,4 | 1,0 | 1,0 | 0,8 | 1,1 | 0,6 | 1,0 | 0,7 | 1,3 |
| Nxf3 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,9 | 1,3 | 1,0 | 1,0 |
| Nxpe2 | 1,0 | 1,0 | 1,0 | 1,0 | 1,0 | 1,0 | 0,9 | 1,0 | 1,0 | 1,0 | 1,0 | 1,0 | 1,0 | 1,0 | 1,5 | 1,2 | 1,0 | 1,0 | 2,1 | 0,6 | 1,0 | 1,0 | 1,0 | 0,8 |
| Nxph4 | 0,8 | 0,7 | 1,0 | 1,0 | 1,2 | 1,2 | 1,0 | 1,0 | 1,0 | 1,0 | 0,9 | 0,6 | 1,2 | 1,0 | 1,0 | 1,0 | 1,0 | 1,0 | 1,0 | 1,0 | 1,0 | 1,0 | 1,0 | 1,0 |
| *Oaz1-ps* | 1,0 | 1,3 | 1,2 | 1,2 | 1,0 | 1,0 | 0,0 | 1,0 | 1,0 | 1,4 | 18,5 | 3,3 | 1,0 | 0,3 | 1,0 | 10,6 | 1,0 | 1,0 | 0,0 | 8,1 | 0,2 | 1,2 | 11,7 | 1,0 |
| Oaz3 | 1,4 | 1,0 | 1,0 | 1,0 | 1,0 | 1,1 | 1,0 | 1,0 | 1,0 | 1,0 | 1,2 | 0,7 | 1,0 | 1,0 | 1,0 | 1,0 | 1,0 | 1,0 | 1,0 | 1,0 | 1,1 | 1,1 | 1,0 | 1,0 |
| Obscn | 1,0 | 1,0 | 1,0 | 0,8 | 1,0 | 1,0 | 1,0 | 1,0 | 1,0 | 1,0 | 1,0 | 1,0 | 1,9 | 0,9 | 3,6 | 0,2 | 1,1 | 1,3 | 1,0 | 1,0 | 1,0 | 1,0 | 1,4 | 4,0 |
| Ociad2 | 1,3 | 1,3 | 1,0 | 0,9 | 1,2 | 1,3 | 1,0 | 0,8 | 0,9 | 0,9 | 1,3 | 1,1 | 1,5 | 1,4 | 1,4 | 1,0 | 0,9 | 0,9 | 1,0 | 1,0 | 0,7 | 0,9 | 0,9 | 1,1 |
| Odf1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,1 | 0,9 | 1,0 | 1,0 |
| Odf3l2 | 1,0 | 1,0 | 1,0 | 1,0 | 1,0 | 1,0 | 1,0 | 1,0 | 1,0 | 1,0 | 1,0 | 1,0 | 2,4 | 1,5 | 4,2 | 0,5 | 1,8 | 1,7 | 1,0 | 1,0 | 1,0 | 1,0 | 1,7 | 26,1 |
| Olfr165 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Omp | 1,0 | 5,4 | 1,1 | 1,0 | 1,0 | 0,9 | 1,0 | 1,0 | 1,0 | 1,0 | 1,0 | 1,0 | 1,5 | 0,4 | 1,6 | 1,1 | 1,0 | 1,0 | 1,0 | 0,9 | 1,0 | 1,0 | 1,0 | 1,0 |
| Onecut1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Oscar | 1,0 | 1,0 | 1,0 | 1,0 | 1,7 | 1,1 | 1,0 | 1,0 | 1,0 | 1,0 | 1,0 | 1,0 | 1,0 | 1,0 | 4,7 | 5,4 | 1,0 | 1,0 | 1,0 | 1,0 | 1,7 | 1,1 | 1,0 | 1,0 |
| Ostn | 1,0 | 1,0 | 1,0 | 1,0 | 1,0 | 1,0 | 1,0 | 1,0 | 1,0 | 1,0 | 1,0 | 1,0 | 1,0 | 1,0 | 0,1 | 0,3 | 0,6 | 0,8 | 1,0 | 1,0 | 1,0 | 1,0 | 1,0 | 1,0 |
| Ovch2 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Oxtr | 1,8 | 4,0 | 1,0 | 1,0 | 1,0 | 1,0 | 1,0 | 1,0 | 1,0 | 1,0 | 1,0 | 1,0 | 1,0 | 0,9 | 1,0 | 1,0 | 1,0 | 1,0 | 1,0 | 1,0 | 0,6 | 0,7 | 1,0 | 1,0 |
| *P2rx2* | 1,0 | 1,0 | 1,0 | 1,0 | 1,0 | 1,0 | 1,0 | 1,0 | 1,0 | 1,0 | 1,0 | 1,2 | 1,7 | 1,3 | 0,8 | 1,3 | 1,0 | 1,0 | 1,0 | 1,0 | 0,9 | 1,0 | 1,0 | 1,0 |
| Padi2 | 0,8 | 0,7 | 0,9 | 1,0 | 0,9 | 0,8 | 0,7 | 0,9 | 1,0 | 0,8 | 1,5 | 1,2 | 2,0 | 1,4 | 1,5 | 1,0 | 1,1 | 1,4 | 1,0 | 1,2 | 1,2 | 1,1 | 0,9 | 1,3 |
| Paqr9 | 0,8 | 0,8 | 0,7 | 0,8 | 0,9 | 0,9 | 1,0 | 0,8 | 1,0 | 1,0 | 1,0 | 0,9 | 0,8 | 0,7 | 1,5 | 1,1 | 0,6 | 0,6 | 1,4 | 0,9 | 1,0 | 0,8 | 0,9 | 0,9 |
| Pate2 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Pax2 | 1,0 | 1,0 | 1,0 | 1,0 | 0,9 | 0,8 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,7 | 1,0 | 1,0 |
| Pax5 | 1,0 | 1,0 | 1,0 | 1,0 | 1,0 | 1,0 | 0,7 | 0,6 | 1,0 | 1,0 | 1,0 | 1,0 | 1,0 | 1,0 | 0,1 | 1,1 | 1,0 | 1,0 | 0,8 | 0,8 | 1,2 | 0,9 | 1,0 | 1,0 |
| Pax8 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,9 | 0,6 |
| Pck1 | 1,9 | 1,5 | 1,2 | 1,3 | 1,0 | 1,0 | 0,6 | 0,1 | 0,5 | 1,0 | 1,0 | 1,0 | 0,8 | 1,8 | 1,5 | 1,0 | 1,1 | 0,4 | 1,0 | 1,0 | 1,0 | 1,0 | 1,3 | 1,4 |
| Pcp4 | 1,2 | 0,9 | 1,0 | 1,0 | 1,1 | 1,0 | 1,0 | 0,4 | 0,7 | 1,0 | 0,6 | 0,5 | 1,3 | 1,9 | 2,6 | 0,5 | 1,0 | 1,0 | 0,5 | 1,0 | 1,0 | 1,0 | 0,9 | 0,9 |
| Pcp4l1 | 1,1 | 1,0 | 0,9 | 0,8 | 1,1 | 1,1 | 1,0 | 0,9 | 1,0 | 1,0 | 1,1 | 0,9 | 1,4 | 1,0 | 1,2 | 0,8 | 0,6 | 0,6 | 0,9 | 1,1 | 0,8 | 0,7 | 0,8 | 0,7 |
| *Pcsk9* | *1,0* | *1,0* | *1,0* | *1,0* | *0,9* | *0,9* | *1,0* | *1,0* | *1,0* | *1,0* | *1,0* | *1,0* | *0,7* | *1,0* | *1,0* | *1,0* | *1,0* | *1,0* | *1,0* | *1,0* | *1,0* | *1,0* | *1,0* | *1,0* |
| Pcyt1b | 1,0 | 1,0 | 1,0 | 1,0 | 0,9 | 0,9 | 1,1 | 1,2 | 1,0 | 1,0 | 1,0 | 1,0 | 0,9 | 1,0 | 1,3 | 1,2 | 1,0 | 1,0 | 2,5 | 0,6 | 0,8 | 1,0 | 6,8 | 6,0 |
| Pde6a | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| *Pdgfrl* | 0,7 | 0,5 | 0,9 | 0,8 | 1,0 | 1,0 | 0,7 | 0,6 | 1,0 | 1,0 | 0,9 | 1,2 | 0,3 | 0,6 | 0,5 | 0,6 | 0,4 | 0,8 | 0,5 | 1,4 | 1,0 | 1,0 | 0,8 | 1,0 |
| Pdk4 | 1,4 | 1,0 | 0,9 | 0,9 | 1,2 | 1,0 | 1,3 | 1,4 | 2,5 | 2,3 | 1,0 | 1,5 | 1,6 | 1,0 | 1,3 | 0,7 | 1,1 | 1,2 | 1,6 | 2,3 | 0,9 | 1,0 | 1,2 | 2,0 |
| Pdlim3 | 1,0 | 0,8 | 1,3 | 0,9 | 1,3 | 1,2 | 0,9 | 0,9 | 0,8 | 1,0 | 1,7 | 1,2 | 2,1 | 0,8 | 2,5 | 0,3 | 0,9 | 1,0 | 1,4 | 1,2 | 0,9 | 1,4 | 1,2 | 12,7 |
| | AG | | Aorta | | Brain | | Lung | | Pancreas | | PG | | Skin | | Skull | | SM | | Spleen | | Testis | | TG | |
| | E | M | E | M | E | M | E | M | E | M | E | M | E | M | E | M | E | M | E | M | E | M | E | M |
| Pdzk1 | 0,8 | 0,9 | 1,3 | .0,7 | 0,7 | 0,9 | 0,9 | 1,1 | 1,0 | 1,0 | 1,0 | 0,7 | 0,9 | 1,1 | 1,1 | 1,2 | 0,9 | 1,5 | 1,0 | 1,0 | 1,0 | 1,1 | 1,2 | 0,8 |
| Pdzk1ip1 | 1,0 | 1,0 | 1,0 | 1,0 | 1,0 | 1,0 | 1,1 | 1,0 | 1,2 | 1,2 | 0,9 | 0,8 | 1,5 | 1,3 | 1,1 | 1,5 | 1,0 | 1,0 | 2,3 | 0,3 | 1,0 | 0,9 | 0,8 | 0,9 |
| Pgam2 | 1,1 | 1,9 | 1,1 | 0,7 | 1,2 | 1,1 | 0,6 | 0,7 | 1,0 | 1,0 | 1,0 | 0,9 | 1,4 | 0,7 | 2,6 | 0,2 | 0,7 | 0,7 | 0,7 | 1,2 | 1,1 | 0,9 | 0,8 | 8,1 |
| Pgc | 1,0 | 1,0 | 1,0 | 0,8 | 1,0 | 1,0 | 1,0 | 0,9 | 1,1 | 1,0 | 1,0 | 1,0 | 1,0 | 1,0 | 1,0 | 1,0 | 1,0 | 0,1 | 0,3 | 1,0 | 1,0 | 1,0 | 1,0 | 0,6 |
| Pgk2 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,1 | 1,0 | 1,0 |
| *Pglyrp1* | *1,0* | *2,3* | *1,4* | *0,8* | *1,1* | *1,0* | *0,9* | *1,1* | *1,0* | *1,0* | *1,0* | *1,0* | *0,7* | *1,4* | *0,8* | *1,2* | *1,0* | *1,0* | *0,9* | *0,8* | *1,0* | *1,0* | *0,1* | *0,4* |
| Phkg1 | 1,1 | 1,2 | 1,1 | 1,0 | 0,8 | 0,8 | 0,9 | 0,6 | 1,0 | 1,0 | 1,0 | 1,0 | 2,0 | 0,8 | 3,6 | 0,2 | 0,9 | 0,9 | 1,0 | 1,0 | 1,0 | 1,0 | 1,1 | 3,5 |
| Phlda1 | 1,1 | 1,1 | 0,8 | 0,9 | 1,1 | 1,0 | 1,2 | 0,9 | 0,8 | 1,0 | 1,0 | 1,0 | 0,9 | 0,8 | 0,8 | 1,0 | 1,0 | 0,7 | 0,7 | 0,6 | 1,0 | 1,0 | 1,0 | 1,3 |
| *Pi15* | 1,0 | 1,0 | 1,1 | 0,9 | 1,0 | 1,0 | 0,7 | 0,8 | 1,0 | 1,0 | 1,0 | 1,0 | 0,3 | 0,5 | 1,0 | 1,0 | 1,0 | 1,0 | 1,0 | 1,3 | 1,0 | 1,0 | 1,0 | 1,0 |
| Pigr | 1,0 | 1,0 | 1,0 | 0,6 | 1,0 | 1,0 | 1,1 | 1,1 | 0,5 | 1,0 | 1,0 | 1,0 | 1,0 | 1,1 | 1,0 | 1,0 | 1,4 | 1,0 | 0,8 | 1,0 | 1,0 | 1,4 | 0,1 | 0,3 |
| Pip5k1b | 1,1 | 1,3 | 1,0 | 1,0 | 1,1 | 1,0 | 1,4 | 1,3 | 1,3 | 1,0 | 1,0 | 0,8 | 1,0 | 0,6 | 1,4 | 1,4 | 1,0 | 1,0 | 2,1 | 0,8 | 1,2 | 1,1 | 0,7 | 0,7 |
| Pkia | 0,8 | 1,1 | 0,9 | 0,9 | 1,0 | 1,1 | 0,8 | 0,9 | 1,0 | 1,0 | 1,1 | 1,1 | 1,8 | 0,9 | 3,0 | 0,4 | 1,1 | 1,0 | 0,9 | 1,1 | 1,0 | 0,9 | 1,1 | 6,7 |
| Pla2g1b | 1,0 | 1,0 | 1,0 | 1,2 | 1,0 | 1,0 | 1,3 | 0,8 | 1,3 | 1,5 | 1,0 | 1,0 | 1,0 | 1,0 | 1,0 | 1,0 | 1,0 | 0,2 | 1,0 | 1,0 | 1,0 | 1,0 | 1,0 | 0,4 |
| Pla2g4c | 1,0 | 1,0 | 1,0 | 1,0 | 1,0 | 1,0 | 1,0 | 1,0 | 1,0 | 1,0 | 1,0 | 1,0 | 1,0 | 1,0 | 1,5 | 1,5 | 1,0 | 1,0 | 1,5 | 1,0 | 1,0 | 1,0 | 1,0 | 1,0 |
| Pla2g5 | 0,8 | 0,7 | 1,0 | 1,0 | 1,2 | 1,0 | 1,0 | 1,0 | 1,0 | 1,0 | 1,4 | 1,4 | 0,6 | 0,7 | 0,7 | 1,2 | 1,0 | 1,3 | 1,0 | 1,0 | 1,1 | 1,0 | 0,8 | 0,8 |
| Plac8 | 1,3 | 0,9 | 0,9 | 0,8 | 1,0 | 1,0 | 0,6 | 0,5 | 1,0 | 1,0 | 1,1 | 0,9 | 0,7 | 1,2 | 0,8 | 1,1 | 0,7 | 1,1 | 0,8 | 0,9 | 0,8 | 1,7 | 1,0 | 1,8 |
| Plac9a | 0,4 | 0,6 | 0,8 | 1,9 | 2,5 | 3,6 | 0,9 | 0,4 | 2,2 | 0,9 | 0,4 | 1,2 | 1,0 | 0,4 | 2,5 | 3,4 | 0,6 | 0,4 | 1,5 | 1,6 | 0,4 | 1,4 | 3,2 | 0,3 |
| Plek2 | 1,0 | 1,0 | 1,0 | 1,0 | 1,0 | 1,0 | 1,0 | 1,0 | 0,7 | 1,5 | 1,0 | 1,0 | 0,9 | 1,1 | 3,0 | 1,9 | 1,0 | 1,0 | 2,8 | 1,0 | 1,0 | 1,0 | 1,0 | 0,9 |
| *Plet1* | *1,0* | *1,0* | *1,7* | *1,0* | *1,0* | *1,0* | *1,2* | *1,1* | *1,8* | *1,3* | *1,0* | *1,0* | *1,3* | *1,5* | *1,0* | *1,0* | *1,0* | *1,0* | *1,0* | *1,0* | *1,0* | *1,0* | *0,8* | *0,7* |
| Plin1 | 0,9 | 1,0 | 0,8 | 0,7 | 1,0 | 1,0 | 0,8 | 0,1 | 0,9 | 0,8 | 1,0 | 1,0 | 0,7 | 1,0 | 0,9 | 0,9 | 0,6 | 0,5 | 1,0 | 1,0 | 1,0 | 0,8 | 1,3 | 1,1 |
| Plin4 | 0,9 | 0,9 | 1,2 | 0,8 | 1,2 | 1,7 | 0,8 | 0,5 | 0,5 | 0,6 | 1,1 | 1,0 | 1,6 | 1,3 | 3,8 | 0,5 | 1,6 | 1,9 | 1,5 | 1,0 | 1,0 | 1,0 | 1,6 | 1,2 |
| Pnlip | 1,0 | 0,8 | 1,0 | 1,0 | 1,3 | 0,9 | 1,0 | 1,0 | 0,9 | 1,2 | 0,8 | 0,7 | 1,8 | 1,0 | 1,6 | 0,8 | 1,0 | 1,0 | 1,0 | 1,2 | 1,0 | 1,0 | 0,6 | 1,0 |
| Pnliprp1 | 1,0 | 1,0 | 1,0 | 1,0 | 1,0 | 1,0 | 1,0 | 1,0 | 0,8 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Pnmt | 1,0 | 0,8 | 1,0 | -1,8 | 1,0 | 1,0 | 1,1 | 1,5 | 1,0 | 1,0 | 1,3 | 1,9 | 1,0 | 1,0 | 1,0 | 1,0 | 5,2 | 2,3 | 1,0 | 1,0 | 1,0 | 1,0 | 1,0 | 1,2 |
| Pou2af1 | 1,4 | 1,0 | 1,2 | 1,0 | 1,0 | 1,0 | 0,6 | 0,5 | 1,0 | 1,0 | 1,0 | 1,0 | 1,0 | 1,0 | 0,6 | 0,8 | 1,0 | 1,0 | 0,8 | 0,9 | 1,0 | 1,0 | 1,0 | 1,0 |
| Pou3f3 | 1,0 | 1,0 | 1,0 | 1,0 | 1,0 | 0,9 | 1,0 | 1,0 | 1,0 | 1,0 | 1,0 | 1,0 | 1,0 | 1,0 | 1,3 | 0,7 | 1,0 | 1,0 | 1,0 | 1,0 | 1,0 | 1,0 | 1,0 | 1,0 |
| Pou3f3os | 1,0 | 1,0 | 1,0 | 1,0 | 1,2 | 1,3 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,4 | 1,0 | 1,0 |
| Ppapdc3 | 1,1 | 1,0 | 0,8 | 0,7 | 0,9 | 1,2 | 0,9 | 1,4 | 0,7 | 1,1 | 0,9 | 0,9 | 1,4 | 0,8 | 1,8 | 0,4 | 0,8 | 0,9 | 1,1 | 1,1 | 0,8 | 0,7 | 0,8 | 6,3 |
| Ppl | 0,4 | 0,3 | 1,2 | 1,4 | 1,0 | 1,0 | 1,2 | 1,3 | 1,0 | 1,0 | 0,8 | 1,1 | 1,3 | 1,0 | 0,8 | 0,7 | 1,1 | 1,0 | 1,0 | 1,3 | 1,0 | 1,0 | 1,0 | 0,8 |
| Ppp1r14c | 3,0 | 5,3 | 1,0 | 0,8 | 1,0 | 1,0 | 0,9 | 0,8 | 1,0 | 1,0 | 1,0 | 1,0 | 1,1 | 1,2 | 2,6 | 0,3 | 0,9 | 0,9 | 1,0 | 1,0 | 1,0 | 1,0 | 0,7 | 5,2 |
| Ppp1r27 | 1,0 | 1,0 | 1,0 | 1,0 | 1,0 | 1,0 | 1,0 | 1,0 | 1,0 | 1,0 | 1,0 | 1,0 | 1,5 | 0,8 | 4,2 | 0,3 | 0,6 | 0,7 | 1,0 | 1,0 | 1,1 | 1,0 | 0,5 | 7,2 |
| *Ppp1r3a* | 1,0 | 1,0 | 1,0 | 1,0 | 1,0 | 1,0 | 1,0 | 1,5 | 1,0 | 1,0 | 1,0 | 1,0 | 2,1 | 1,1 | 4,6 | 0,2 | 1,3 | 1,2 | 1,0 | 1,0 | 1,0 | 1,0 | 1,2 | 7,1 |
| Prb1 | 1,0 | 1,0 | 1,0 | 1,0 | 1,0 | 1,0 | 1,0 | 0,8 | 1,0 | 1,0 | 1,0 | 1,0 | 1,0 | 1,0 | 1,0 | 1,0 | 1,0 | 1,0 | 1,0 | 1,0 | 1,0 | 1,0 | 0,1 | 1,0 |
| Prh1 | 1,0 | 1,0 | 1,0 | 1,2 | 1,0 | 1,0 | 1,0 | 0,8 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,7 |
| Prkcq | 1,0 | 1,0 | 1,0 | 1,0 | 0,8 | 0,8 | 0,9 | 0,9 | 1,0 | 1,0 | 0,7 | 0,7 | 1,9 | 0,9 | 1,5 | 0,9 | 1,2 | 1,4 | 1,1 | 0,9 | 1,0 | 1,1 | 2,0 | 5,2 |
| Prlr | 1,2 | 1,5 | 1,3 | 1,0 | 0,7 | 0,8 | 1,0 | 1,0 | 1,0 | 1,0 | 0,7 | 0,7 | 0,9 | 1,4 | 1,0 | 0,8 | 1,0 | 1,0 | 1,0 | 1,0 | 0,8 | 0,9 | 0,7 | 0,6 |
| Prm1 | 1,0 | 1,0 | 0,7 | 1,0 | 1,0 | 1,0 | 1,0 | 1,0 | 1,0 | 1,0 | 0,7 | 1,2 | 0,9 | 0,5 | 1,0 | 1,0 | 0,9 | 1,7 | 2,5 | 1,0 | 1,1 | 1,0 | 1,0 | 1,0 |
| Prm2 | 1,0 | 1,0 | 0,5 | 1,0 | 1,0 | 1,0 | 1,0 | 1,0 | 1,0 | 1,0 | 1,0 | 1,0 | 2,6 | 1,0 | 1,0 | 1,0 | 0,6 | 2,5 | 0,7 | 1,0 | 1,1 | 0,9 | 1,0 | 1,0 |
| Prm3 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,1 | 1,0 | 1,0 | 1,0 |
| Prnd | 0,7 | 1,0 | 0,5 | 0,3 | 1,0 | 1,0 | 1,0 | 0,9 | 1,0 | 1,0 | 1,0 | 1,0 | 0,8 | 1,6 | 0,9 | 1,0 | 1,0 | 0,8 | 0,6 | 0,9 | 1,0 | 0,9 | 1,0 | 1,0 |
| Nt5c1a | 0,2 | 1,0 | 1,0 | 1,1 | 0,9 | 1,0 | 1,2 | 0,6 | 1,0 | 1,0 | 0,9 | 1,0 | 1,0 | 0,7 | 12,5 | 1,0 | 1,0 | 0,0 | 1,0 | 1,0 | 1,0 | 1,0 | 1,1 | 0,9 |
| *Prom2* | *1,0* | *1,0* | *1,0* | *1,0* | *1,0* | *1,0* | *1,0* | *1,0* | *1,0* | *1,3* | *1,2* | *1,0* | *1,1* | *0,9* | *1,0* | *1,0* | *1,0* | *1,0* | *1,0* | *1,0* | *1,0* | *1,5* | *0,8* | *0,7* |
| Prp2 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Prph | 1,3 | 7,8 | 1,0 | 0,8 | 0,7 | 0,7 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| *Prpmp5* | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,2 | 1,0 |
| Prr32 | 1,0 | 1,0 | 1,7 | 1,2 | 0,7 | 0,8 | 1,0 | 1,0 | 1,0 | 1,0 | 1,0 | 1,0 | 1,0 | 1,0 | 2,1 | 1,1 | 1,0 | 1,0 | 1,0 | 1,0 | 0,9 | 1,1 | 1,2 | 1,0 |

[Fig. 23-11]

| | | | | | | | | | | | | | | | | | | | | | | | | |
|---|---|---|---|---|---|---|---|---|---|---|---|---|---|---|---|---|---|---|---|---|---|---|---|---|
| Prr33 | 0,7 | 0,7 | 1,1 | 0,7 | 1,0 | 1,0 | 1,0 | 1,0 | 1,0 | 1,0 | 1,1 | 0,8 | 1,6 | 1,1 | 1,3 | 0,5 | 1,1 | 1,1 | 1,0 | 0,9 | 1,0 | 1,0 | 1,2 | 5,9 |
| Prss1 | 1,0 | 1,0 | 1,0 | 1,0 | 1,0 | 1,0 | 1,0 | 1,0 | 11,5 | 0,8 | 1,0 | 1,0 | 1,0 | 1,0 | 1,0 | 1,0 | 1,0 | 1,0 | 1,0 | 1,0 | 1,0 | 1,0 | 1,0 | 1,0 |
| Prss2 | 0,3 | 0,6 | 1,0 | 1,0 | 0,8 | 1,3 | 0,9 | 1,0 | 1,3 | 1,4 | 1,2 | 1,2 | 3,5 | 1,0 | 4,7 | 0,9 | 1,0 | 1,0 | 1,0 | 1,0 | 1,0 | 1,0 | 0,6 | 1,0 |
| Prss3 | 1,0 | 1,0 | 1,0 | 1,0 | 1,0 | 1,0 | 1,0 | 1,0 | 4,5 | 0,6 | 1,0 | 1,0 | 1,0 | 1,0 | 1,0 | 1,0 | 1,0 | 1,0 | 1,0 | 1,0 | 1,0 | 1,0 | 1,0 | 1,0 |
| Prss8 | 1,0 | 1,0 | 0,9 | 2,1 | 1,0 | 1,0 | 0,9 | 1,0 | 0,8 | 0,9 | 1,0 | 1,2 | 1,5 | 1,0 | 1,0 | 1,0 | 1,0 | 1,0 | 1,0 | 1,0 | 1,0 | 1,0 | 0,9 | 0,9 |
| Prtn3 | 1,0 | 1,0 | 1,8 | 1,7 | 1,0 | 0,9 | 1,1 | 0,8 | 1,0 | 1,0 | 1,0 | 1,0 | 0,9 | 1,9 | 0,8 | 0,9 | 1,0 | 1,0 | 1,1 | 0,5 | 1,0 | 1,0 | 1,0 | 1,0 |
| Ptch1 | 0,3 | 0,6 | 1,0 | 0,7 | 0,9 | 0,7 | 0,8 | 2,0 | 1,0 | 1,0 | 1,3 | 1,0 | 0,3 | 0,8 | 0,9 | 0,5 | 0,8 | 1,1 | 1,2 | 1,1 | 1,1 | 0,8 | 0,9 | 0,9 |
| Ptch2 | 0,5 | 0,9 | 1,0 | 1,0 | 1,0 | 1,0 | 1,0 | 1,0 | 1,0 | 1,0 | 1,0 | 1,0 | 0,3 | 0,6 | 1,0 | 1,0 | 1,0 | 1,0 | 1,0 | 1,0 | 1,0 | 0,8 | 1,0 | 1,0 |
| Ptgds | 0,9 | 0,9 | 1,2 | 0,3 | 1,3 | 1,2 | 1,0 | 1,0 | 1,0 | 1,0 | 1,4 | 1,3 | 0,8 | 0,5 | 1,2 | 1,8 | 1,0 | 1,0 | 1,0 | 1,0 | 1,3 | 1,0 | 1,0 | 1,6 |
| Ptger3 | 0,7 | 0,7 | 0,5 | 0,2 | 0,7 | 0,9 | 0,8 | 0,5 | 1,3 | 0,9 | 1,0 | 1,0 | 0,9 | 1,2 | 1,0 | 1,0 | 0,5 | 0,6 | 1,0 | 0,8 | 1,0 | 1,0 | 0,9 | 0,8 |
| Ptgs2 | 1,0 | 0,9 | 1,0 | 1,0 | 0,9 | 1,1 | 1,4 | 1,5 | 1,0 | 1,0 | 1,0 | 1,0 | 1,1 | 1,2 | 1,2 | 1,0 | 1,0 | 1,0 | 1,0 | 1,0 | 1,0 | 1,0 | 1,0 | 1,0 |
| Pth | 1,0 | 1,0 | 1,0 | 1,1 | 1,0 | 1,0 | 1,0 | 0,6 | 0,7 | 1,0 | 1,0 | 1,0 | 1,0 | 1,0 | 1,0 | 1,0 | 1,0 | 0,9 | 1,0 | 1,0 | 1,0 | 1,0 | 6,3 | 5,3 |
| Ptpn5 | 1,0 | 1,0 | 1,0 | 1,0 | 1,0 | 1,1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Ptprcap | 1,5 | 0,7 | 1,2 | 0,9 | 1,0 | 1,0 | 0,6 | 0,5 | 1,0 | 1,0 | 0,6 | 1,3 | 1,3 | 1,5 | 0,7 | 1,2 | 1,0 | 1,0 | 0,8 | 0,9 | 1,0 | 1,0 | 0,1 | 1,0 |
| Pvalb | 3,6 | 1,0 | 1,0 | 0,5 | 1,2 | 1,2 | 1,0 | 1,8 | 1,0 | 1,0 | 1,1 | 1,1 | 1,8 | 0,7 | 2,8 | 0,2 | 0,8 | 0,9 | 1,0 | 1,0 | 1,0 | 1,2 | 0,7 | 11,2 |
| Pvrl4 | 1,0 | 1,0 | 1,0 | 1,0 | 0,9 | 1,1 | 1,1 | 1,1 | 1,0 | 1,0 | 1,1 | 1,0 | 1,1 | 0,9 | 0,9 | 1,2 | 1,0 | 1,0 | 1,2 | 1,3 | 0,9 | 0,9 | 1,1 | 1,1 |
| Pygm | 1,5 | 2,5 | 0,8 | 0,7 | 0,9 | 1,1 | 0,7 | 1,0 | 1,0 | 1,0 | 0,8 | 0,8 | 2,5 | 1,0 | 3,1 | 0,3 | 1,0 | 1,2 | 1,0 | 1,2 | 1,0 | 1,1 | 0,9 | 3,0 |
| Pzp | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Rab11fip1 | 0,7 | 0,3 | 0,9 | 1,0 | 0,9 | 1,0 | 1,3 | 1,3 | 1,4 | 0,8 | 1,0 | 1,1 | 1,2 | 1,3 | 1,0 | 0,9 | 1,0 | 1,0 | 1,1 | 1,2 | 0,9 | 1,0 | 0,9 | 0,7 |
| Rab11fip4 | 0,7 | 0,6 | 1,0 | 1,0 | 0,9 | 0,9 | 0,9 | 1,4 | 1,2 | 1,0 | 1,0 | 1,0 | 1,1 | 1,3 | 0,7 | 0,9 | 1,0 | 1,0 | 0,8 | 1,2 | 1,1 | 1,0 | 0,8 | 0,6 |
| Rab25 | 1,0 | 1,0 | 1,0 | 1,0 | 1,0 | 1,0 | 1,1 | 1,1 | 0,8 | 1,1 | 0,8 | 0,8 | 1,1 | 0,9 | 1,0 | 1,0 | 1,0 | 1,0 | 1,0 | 1,0 | 1,0 | 1,0 | 1,0 | 0,9 |
| Rab26 | 1,0 | 1,1 | 1,0 | 1,0 | 1,0 | 1,1 | 1,0 | 1,0 | 1,0 | 1,0 | 1,0 | 1,2 | 1,0 | 1,0 | 1,0 | 1,0 | 1,0 | 1,0 | 1,0 | 1,0 | 1,0 | 1,0 | 1,0 | 1,0 |
| Rab30 | 1,4 | 1,1 | 0,7 | 0,7 | 0,9 | 0,7 | 0,7 | 1,0 | 1,0 | 1,0 | 0,8 | 0,8 | 1,1 | 0,8 | 0,9 | 1,1 | 1,0 | 0,6 | 0,8 | 1,0 | 1,0 | 1,2 | 1,2 | 1,1 |
| Rad51b | 1,0 | 1,0 | 1,0 | 1,0 | 1,0 | 1,0 | 1,0 | 1,4 | 1,0 | 1,0 | 1,0 | 1,0 | 0,9 | 1,0 | 0,7 | 0,8 | 1,0 | 1,0 | 1,3 | 1,0 | 1,2 | 1,1 | 1,0 | 1,0 |
| Rag1 | 1,0 | 1,0 | 1,0 | 1,0 | 1,0 | 1,0 | 1,0 | 1,0 | 1,0 | 1,0 | 1,0 | 1,0 | 1,0 | 1,0 | 0,6 | 0,3 | 1,0 | 1,0 | 1,0 | 1,0 | 1,0 | 1,0 | 0,1 | 1,0 |
| Ramp1 | 1,2 | 0,7 | 1,0 | 0,7 | 1,0 | 1,1 | 1,1 | 1,0 | 0,7 | 0,7 | 0,7 | 0,8 | 1,2 | 1,2 | 1,1 | 0,8 | 0,8 | 0,9 | 1,0 | 1,3 | 1,1 | 1,5 | 0,7 | 5,2 |
| Ramp3 | 1,1 | 1,1 | 0,5 | 0,8 | 0,9 | 1,2 | 1,2 | 1,3 | 1,1 | 1,0 | 0,6 | 0,6 | 1,3 | 1,5 | 1,3 | 1,0 | 1,0 | 1,0 | 1,0 | 1,6 | 1,1 | 1,0 | 0,8 | 0,6 |
| Rarres1 | 0,7 | 0,3 | 0,7 | 1,1 | 1,0 | 1,0 | 1,0 | 0,6 | 1,0 | 1,0 | 0,7 | 0,5 | 0,5 | 1,0 | 1,2 | 1,6 | 1,0 | 0,9 | 1,4 | 1,3 | 0,7 | 0,7 | 1,0 | 1,0 |
| Rasal1 | 1,5 | 2,6 | 1,0 | 1,0 | 1,1 | 1,2 | 1,0 | 1,0 | 1,0 | 1,0 | 0,8 | 0,6 | 1,4 | 1,1 | 1,0 | 1,0 | 1,0 | 1,0 | 1,1 | 1,0 | 1,0 | 0,9 | 5,7 | 4,5 |
| Rasd1 | 0,9 | 1,5 | 0,6 | 1,7 | 0,9 | 1,2 | 2,2 | 2,2 | 3,3 | 5,5 | 1,3 | 1,3 | 1,5 | 1,7 | 1,9 | 1,6 | 0,7 | 0,9 | 1,6 | 1,1 | 1,5 | 1,0 | 1,1 | 1,5 |
| Rasgrf1 | 1,1 | 1,3 | 1,0 | 1,0 | 1,1 | 0,9 | 1,0 | 1,0 | 1,0 | 1,0 | 1,3 | 0,9 | 0,9 | 1,0 | 0,6 | 0,3 | 1,0 | 1,0 | 1,0 | 1,0 | 0,8 | 0,9 | 1,0 | 1,0 |
| Rasl10a | 1,0 | 1,0 | 1,8 | 0,8 | 1,0 | 0,9 | 0,8 | 1,5 | 1,0 | 1,0 | 0,8 | 1,4 | 1,6 | 1,1 | 0,9 | 0,7 | 1,0 | 1,0 | 1,0 | 0,7 | 1,4 | 1,8 | 0,7 | 0,7 |
| Rassf6 | 1,5 | 1,2 | 1,3 | 1,8 | 1,0 | 1,2 | 1,0 | 0,7 | 1,0 | 1,0 | 1,1 | 1,2 | 0,8 | 0,9 | 1,0 | 1,0 | 1,0 | 1,0 | 0,5 | 0,3 | 1,0 | 1,0 | 1,0 | 0,9 |
| Rbfox1 | 1,0 | 1,0 | 1,0 | 1,0 | 1,1 | 1,0 | 1,0 | 1,0 | 1,0 | 1,0 | 1,0 | 0,9 | 2,6 | 1,2 | 3,9 | 0,3 | 1,2 | 1,4 | 1,0 | 1,0 | 1,0 | 1,0 | 1,2 | 5,5 |
| Rbm11 | 0,9 | 0,8 | 1,0 | 1,0 | 0,9 | 1,1 | 1,0 | 1,0 | 1,0 | 1,0 | 1,1 | 1,1 | 1,0 | 1,0 | 1,0 | 1,0 | 1,0 | 1,0 | 1,0 | 1,0 | 1,2 | 1,1 | 1,0 | 1,0 |
| Rbm24 | 0,9 | 1,0 | 1,1 | 0,9 | 1,0 | 0,9 | 1,1 | 0,9 | 1,0 | 1,0 | 1,0 | 1,0 | 2,0 | 1,1 | 2,9 | 0,3 | 1,0 | 1,1 | 1,0 | 1,0 | 1,0 | 1,0 | 1,3 | 7,2 |
| Reg1 | 1,0 | 1,2 | 1,0 | 1,0 | 1,0 | 1,0 | 1,0 | 1,0 | 1,2 | 1,3 | 0,7 | 0,9 | 1,0 | 1,0 | 1,0 | 0,7 | 1,0 | 1,0 | 1,0 | 0,8 | 1,0 | 1,0 | 1,2 | 1,0 |
| Reg2 | 1,0 | 1,0 | 1,0 | 1,0 | 1,0 | 1,0 | 1,0 | 1,0 | 1,3 | 1,4 | 1,0 | 1,0 | 1,0 | 1,0 | 1,0 | 1,0 | 1,0 | 1,0 | 1,0 | 1,0 | 1,0 | 1,0 | 1,0 | 1,0 |
| Reg3b | 1,0 | 1,0 | 1,0 | 1,0 | 1,0 | 1,0 | 1,0 | 1,0 | 1,0 | 2,7 | 1,0 | 1,0 | 1,6 | 0,8 | 0,2 | 1,0 | 1,0 | 1,0 | 1,0 | 1,0 | 1,0 | 2,3 | 1,0 | 1,0 |
| Reg3g | 1,0 | 1,0 | 1,0 | 1,0 | 1,0 | 1,0 | 0,6 | 0,9 | 1,3 | 1,9 | 0,3 | 1,0 | 1,0 | 0,8 | 0,4 | 1,0 | 1,0 | 1,0 | 1,0 | 1,0 | 1,0 | 1,0 | 0,2 | 0,9 |
| Ren2 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Retnla | 1,2 | 2,1 | 1,0 | 1,4 | 1,0 | 1,0 | 1,3 | 1,4 | 0,7 | 1,0 | 1,0 | 1,0 | 0,9 | 1,0 | 0,7 | 0,3 | 1,0 | 0,4 | 2,3 | 0,6 | 1,0 | 1,0 | 2,1 | 2,7 |
| Retnlb | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Rgs13 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,4 | 0,1 | 1,0 | 1,0 | 1,0 | 1,0 |
| Rgs7bp | 1,0 | 0,9 | 1,4 | 1,1 | 0,9 | 0,9 | 0,9 | 1,0 | 1,0 | 1,0 | 1,0 | 1,2 | 0,8 | 0,8 | 0,7 | 0,8 | 1,0 | 1,0 | 1,0 | 1,0 | 1,0 | 1,0 | 1,0 | 1,0 |
| Rgs9 | 1,1 | 0,8 | 1,0 | 1,0 | 1,1 | 1,2 | 1,1 | 1,2 | 1,0 | 1,0 | 0,9 | 1,2 | 0,8 | 0,7 | 1,6 | 1,0 | 1,0 | 1,0 | 1,0 | 0,7 | 0,8 | 1,2 | 0,7 | 0,9 |
| Rhag | 1,0 | 1,0 | 1,0 | 1,0 | 1,0 | 1,0 | 1,0 | 1,0 | 1,0 | 1,0 | 1,0 | 1,0 | 1,0 | 1,0 | 2,0 | 1,9 | 1,0 | 1,0 | 6,2 | 0,3 | 1,0 | 1,0 | 1,0 | 1,0 |
| Rhbg | 1,0 | 1,0 | 1,0 | 1,0 | 1,0 | 1,0 | 1,3 | 0,9 | 1,0 | 1,0 | 1,0 | 1,0 | 1,1 | 0,9 | 1,0 | 1,0 | 1,0 | 1,0 | 1,0 | 1,0 | 1,0 | 0,7 | 0,8 | 1,0 |
| Rhcg | 3,0 | 3,8 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,9 | 0,8 | 5,4 | 4,1 |
| Rhd | 1,0 | 1,0 | 1,0 | 1,0 | 1,0 | 1,0 | 1,0 | 1,0 | 1,0 | 1,0 | 1,0 | 1,0 | 1,0 | 1,0 | 1,5 | 1,7 | 1,0 | 1,0 | 6,1 | 0,2 | 1,0 | 1,0 | 1,0 | 1,0 |
| Rhov | 0,9 | 1,2 | 1,0 | 1,0 | 1,0 | 1,2 | 1,1 | 0,6 | 1,1 | 0,8 | 1,3 | 1,0 | 1,1 | 0,9 | 1,0 | 1,4 | 1,0 | 1,0 | 1,0 | 1,0 | 1,0 | 1,1 | 1,0 | 1,0 |
| | AG | | Aorta | | Brain | | Lung | | Pancreas | | PG | | Skin | | Skull | | SM | | Spleen | | Testis | | TG | |
| | E | M | E | M | E | M | E | M | E | M | E | M | E | M | E | M | E | M | E | M | E | M | E | M |
| Rhpn1 | 1,0 | 1,0 | 1,0 | 1,0 | 1,0 | 1,0 | 1,0 | 0,8 | 1,0 | 1,0 | 0,9 | 1,0 | 1,0 | 1,0 | 1,0 | 1,0 | 1,0 | 1,0 | 1,0 | 1,0 | 1,0 | 0,8 | 1,0 | 0,7 |
| Rian | 0,9 | 0,8 | 0,9 | 0,9 | 0,9 | 1,0 | 0,8 | 0,9 | 1,0 | 1,0 | 0,9 | 0,9 | 0,3 | 0,4 | 0,8 | 0,7 | 0,8 | 0,7 | 1,0 | 1,0 | 1,0 | 1,0 | 0,6 | 0,8 |
| Rmrp | 0,7 | 1,7 | 1,0 | 2,0 | 0,9 | 0,5 | 1,0 | 0,2 | 1,0 | 1,0 | 1,6 | 0,9 | 1,0 | 0,9 | 1,4 | 3,6 | 1,0 | 0,4 | 0,0 | 0,9 | 1,0 | 1,0 | 1,0 | 5,8 |
| Rn4.5s | 1,2 | 1,0 | 25,0 | 1,3 | 0,5 | 0,8 | 0,9 | 0,8 | 1,2 | 2,1 | 1,3 | 0,8 | 0,4 | 1,5 | 0,7 | 1,1 | 1,0 | 0,7 | 2,2 | 0,8 | 1,0 | 0,6 | 0,6 | 0,7 |
| Rn45s | 0,5 | 1,2 | 0,6 | 1,2 | 0,8 | 1,6 | 0,8 | 0,2 | 0,9 | 1,2 | 1,0 | 1,3 | 0,8 | 0,8 | 1,9 | 2,5 | 1,2 | 0,2 | 0,9 | 0,9 | 1,3 | 1,0 | 1,0 | 1,6 |
| Rnase1 | 0,4 | 1,0 | 1,0 | 1,0 | 0,9 | 1,0 | 0,6 | 1,0 | 1,0 | 1,0 | 0,4 | 0,8 | 0,7 | 1,1 | 2,8 | 0,7 | 1,0 | 1,0 | 1,0 | 0,9 | 1,0 | 1,0 | 1,6 | 1,0 |
| Rnase10 | 1,0 | 1,0 | 1,0 | 1,0 | 1,0 | 1,0 | 1,0 | 1,0 | 1,0 | 1,0 | 1,0 | 1,0 | 0,7 | 1,0 | 1,0 | 1,0 | 1,0 | 1,0 | 1,0 | 1,0 | 1,1 | 1,4 | 1,0 | 1,0 |
| Rnase11 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Rnase12 | 1,0 | 1,0 | 1,0 | 1,0 | 1,0 | 1,0 | 1,0 | 1,0 | 1,0 | 1,0 | 1,0 | 1,0 | 0,7 | 0,6 | 0,7 | 1,4 | 1,0 | 1,0 | 0,8 | 1,7 | 1,0 | 2,6 | 1,0 | 1,0 |
| Rnase13 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 2,5 | 1,0 | 1,0 | 1,0 | 1,1 | 0,9 | 1,0 | 1,0 |
| Rnase2a | 1,0 | 1,3 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Rnase9 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 2,3 | 1,0 | 1,0 |
| Rnf128 | 0,9 | 1,1 | 1,2 | 1,7 | 1,0 | 1,0 | 0,9 | 1,2 | 1,4 | 1,6 | 1,0 | 0,9 | 1,0 | 1,1 | 2,6 | 0,9 | 0,9 | 1,2 | 1,0 | 1,0 | 0,7 | 1,2 | 0,9 | 0,9 |
| Rnf149 | 1,0 | 1,0 | 0,9 | 0,9 | 1,0 | 0,9 | 1,0 | 1,0 | 1,1 | 1,1 | 0,8 | 0,8 | 1,0 | 0,7 | 1,0 | 1,3 | 0,9 | 0,8 | 1,0 | 1,2 | 1,1 | 1,0 | 1,0 | 0,7 |
| Rnf180 | 1,0 | 1,0 | 1,0 | 1,0 | 0,9 | 0,8 | 1,0 | 1,0 | 1,0 | 1,0 | 0,9 | 1,0 | 1,0 | 0,7 | 1,0 | 1,0 | 1,0 | 1,0 | 1,0 | 1,0 | 1,2 | 0,8 | 1,0 | 1,0 |
| Rnf186 | 1,0 | 1,0 | 1,0 | 1,0 | 1,0 | 1,0 | 0,9 | 0,9 | 1,0 | 1,0 | 1,0 | 1,0 | 1,0 | 1,0 | 1,0 | 1,0 | 1,0 | 1,0 | 1,5 | 0,9 | 1,0 | 1,0 | 0,8 | 1,0 |
| Rnf24 | 0,8 | 0,8 | 0,9 | 0,6 | 1,0 | 0,8 | 1,0 | 0,9 | 1,0 | 1,0 | 1,1 | 1,3 | 0,7 | 1,0 | 0,9 | 0,7 | 0,7 | 1,0 | 0,9 | 0,9 | 1,1 | 1,1 | 1,5 | 1,1 |
| Rnf43 | 0,8 | 1,2 | 1,0 | 1,0 | 1,2 | 1,0 | 0,7 | 0,9 | 0,8 | 0,9 | 1,0 | 1,2 | 0,9 | 0,8 | 0,9 | 1,1 | 1,0 | 1,0 | 0,7 | 0,7 | 1,1 | 1,0 | 1,0 | 0,9 |
| Rnu12 | 1,0 | 1,0 | 1,0 | 0,1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Rpl38 | 3,2 | 2,6 | 0,5 | 1,2 | 0,8 | 0,9 | 2,5 | 0,7 | 3,1 | 1,2 | 0,9 | 0,7 | 0,6 | 1,2 | 0,4 | 1,7 | 2,1 | 1,0 | 1,1 | 1,2 | 1,0 | 1,1 | 2,0 | 2,5 |
| Rpl3l | 1,0 | 1,0 | 1,0 | 0,8 | 1,0 | 1,0 | 1,0 | 1,0 | 1,0 | 1,0 | 1,0 | 1,0 | 2,4 | 1,1 | 3,7 | 0,3 | 1,0 | 1,0 | 1,0 | 1,0 | 1,0 | 1,0 | 1,4 | 6,6 |
| Rpph1 | 0,7 | 0,9 | 0,5 | 2,6 | 1,0 | 0,5 | 0,9 | 0,5 | 1,0 | 1,0 | 1,1 | 1,0 | 0,7 | 1,2 | 3,0 | 1,5 | 1,0 | 1,0 | 0,7 | 0,8 | 0,6 | 0,3 | 1,0 | 8,0 |
| Rprm | 1,0 | 1,0 | 1,0 | 1,0 | 1,1 | 1,1 | 1,1 | 0,9 | 0,5 | 0,8 | 1,2 | 1,0 | 1,0 | 1,0 | 1,0 | 1,0 | 1,0 | 1,0 | 1,0 | 1,0 | 1,0 | 1,0 | 0,9 | 0,9 |
| Rptn | 1,0 | 1,0 | 1,0 | 1,0 | 1,0 | 1,0 | 1,0 | 0,9 | 1,0 | 1,0 | 1,0 | 1,0 | 1,0 | 1,8 | 1,0 | 1,0 | 1,0 | 1,0 | 1,0 | 1,0 | 1,0 | 1,0 | 19,2 | 1,0 |
| Rtn2 | 0,5 | 0,9 | 0,9 | 1,0 | 1,0 | 1,0 | 0,9 | 0,9 | 1,0 | 1,0 | 1,0 | 1,1 | 2,5 | 1,0 | 2,8 | 0,3 | 1,1 | 1,2 | 1,4 | 1,5 | 1,2 | 0,8 | 1,3 | 9,6 |
| Rtp3 | 1,0 | 0,7 | 0,8 | 0,6 | 1,0 | 1,0 | 0,8 | 0,9 | 1,0 | 1,0 | 1,0 | 1,0 | 1,0 | 1,0 | 1,0 | 1,0 | 1,0 | 1,0 | 1,0 | 1,0 | 1,2 | 1,4 | 1,0 | 0,7 |
| Runx2 | 0,7 | 0,9 | 1,0 | 1,0 | 1,1 | 1,0 | 0,9 | 0,9 | 1,0 | 1,0 | 1,3 | 1,0 | 0,9 | 1,1 | 0,8 | 1,0 | 1,0 | 1,0 | 0,8 | 1,0 | 1,0 | 0,6 | 1,0 | 1,0 |
| Ryr1 | 1,0 | 1,0 | 1,0 | 1,0 | 0,9 | 0,9 | 1,0 | 1,0 | 1,0 | 1,0 | 1,0 | 1,0 | 2,2 | 0,9 | 3,4 | 0,2 | 1,1 | 1,5 | 1,0 | 1,0 | 1,0 | 1,0 | 1,1 | 12,5 |
| S100a8 | 2,4 | 1,2 | 3,5 | -2,6 | 4,2 | 1,7 | 2,3 | 2,1 | 1,0 | 1,7 | 3,1 | 1,8 | 1,1 | 1,1 | 0,8 | 1,2 | 1,0 | 1,0 | 1,1 | 1,0 | 1,0 | 1,0 | 1,7 | 1,0 |
| S100a9 | 5,6 | 1,9 | 1,5 | 3,1 | 2,4 | 2,1 | 2,7 | 1,9 | 0,9 | 0,8 | 3,5 | 2,5 | 2,8 | 4,1 | 0,9 | 1,2 | 1,0 | 1,1 | 1,4 | 1,1 | 1,0 | 1,0 | 1,6 | 2,0 |
| S100g | 1,0 | 1,0 | 1,0 | 1,0 | 1,0 | 1,0 | 0,7 | 0,7 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |

[Fig. 23-12]

281/310

| | | | | | | | | | | | | | | | | | | | | | | | | |
|---|---|---|---|---|---|---|---|---|---|---|---|---|---|---|---|---|---|---|---|---|---|---|---|---|
| Saa1 | 1,0 | 1,0 | 1,0 | 0,8 | 1,0 | 1,0 | 1,0 | 1,0 | 1,0 | 1,0 | 1,0 | 1,0 | 1,0 | 0,6 | 1,0 | 1,0 | 1,0 | 1,0 | 1,0 | 1,0 | 0,6 | 0,9 | 1,0 | 1,0 |
| Saa2 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Saa3 | 1,0 | 1,0 | 2,6 | 2,0 | 1,0 | 1,0 | 1,1 | 1,4 | 1,0 | 1,0 | 1,0 | 1,0 | 0,7 | 2,8 | 1,7 | 1,8 | 1,0 | 1,0 | 1,0 | 1,0 | 1,0 | 1,0 | 1,0 | 1,0 |
| Sash3 | 1,1 | 0,9 | 1,1 | 1,2 | 1,0 | 1,0 | 0,7 | 0,7 | 1,0 | 1,0 | 1,0 | 1,0 | 0,9 | 1,3 | 0,9 | 0,9 | 1,0 | 1,0 | 0,9 | 0,9 | 1,0 | 1,0 | 0,2 | 1,0 |
| Sbk2 | 1,0 | 1,0 | 0,7 | 1,0 | 1,0 | 1,0 | 1,0 | 0,5 | 1,0 | 1,0 | 1,1 | 1,3 | 0,7 | 0,4 | 1,5 | 0,3 | 0,6 | 0,4 | 1,3 | 1,0 | 1,0 | 1,0 | 0,8 | 15,4 |
| Sbp | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,2 | 0,1 |
| Sbpl | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,2 | 0,0 |
| Scara5 | 1,1 | 1,5 | 0,9 | 1,0 | 1,0 | 1,0 | 0,9 | 0,8 | 0,9 | 0,8 | 0,8 | 1,0 | 0,9 | 0,9 | 0,8 | 0,8 | 0,9 | 0,7 | 1,0 | 1,4 | 1,0 | 1,1 | 1,0 | 0,8 |
| Scarna3b | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Scarna8 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Scd1 | 0,4 | 0,6 | 0,2 | 0,3 | 0,9 | 0,8 | 1,1 | 0,9 | 0,3 | 0,4 | 0,9 | 0,9 | 1,0 | 2,0 | 1,6 | 1,6 | 0,5 | 0,4 | 1,0 | 1,1 | 0,8 | 0,9 | 1,1 | 1,0 |
| Scd2 | 0,9 | 1,1 | 0,4 | 0,2 | 0,9 | 0,9 | 1,1 | 1,2 | 1,0 | 1,0 | 1,0 | 0,9 | 0,7 | 0,6 | 1,0 | 0,8 | 0,5 | 0,4 | 1,1 | 1,1 | 0,9 | 0,9 | 1,0 | 0,8 |
| Slc15a2 | 0,2 | 0,6 | 0,2 | 0,3 | 1,0 | 0,9 | 1,0 | 1,0 | 1,0 | 1,0 | 1,1 | 0,9 | 1,3 | 1,9 | 1,4 | 1,0 | 0,6 | 0,5 | 0,9 | 1,0 | 1,0 | 1,1 | 1,3 | 1,0 |
| Scd4 | 0,3 | 0,6 | 0,2 | 0,2 | 1,0 | 1,0 | 0,9 | 1,5 | 1,0 | 1,0 | 1,0 | 1,0 | 0,8 | 1,7 | 1,0 | 1,0 | 1,0 | 1,0 | 1,0 | 1,0 | 1,0 | 1,0 | 1,2 | 1,1 |
| Snora64 | 0,2 | 1,0 | 1,2 | 1,2 | 0,2 | 1,0 | 1,3 | 0,9 | 1,0 | 1,0 | 0,8 | 1,0 | 1,9 | 1,6 | 3,9 | 1,0 | 1,0 | 0,1 | 1,4 | 1,0 | 1,0 | 1,0 | 0,9 | 5,8 |
| Acan | 0,2 | 1,0 | 1,0 | 1,2 | 0,6 | 1,0 | 1,4 | 0,7 | 0,8 | 1,0 | 1,3 | 1,0 | 1,0 | 1,6 | 4,8 | 1,0 | 1,0 | 0,1 | 1,0 | 1,0 | 1,0 | 1,0 | 2,0 | 1,1 |
| Scgb2b12 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,2 | 1,0 |
| Scgb2b15 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,2 | 1,0 |
| Scgb2b17 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,2 | 0,5 |
| Scgb2b20 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,2 | 1,0 |
| Scgb2b27 | 0,3 | 1,0 | 1,0 | 0,8 | 0,6 | 1,0 | 1,6 | 0,5 | 0,9 | 1,0 | 0,8 | 1,0 | 1,0 | 1,6 | 3,1 | 1,0 | 1,0 | 0,1 | 1,0 | 1,0 | 1,0 | 1,0 | 0,7 | 0,7 |
| Scgb3a1 | 1,0 | 1,0 | 1,0 | 1,0 | 1,0 | 1,0 | 1,1 | 1,3 | 1,0 | 1,0 | 1,0 | 1,0 | 1,0 | 1,0 | 1,0 | 1,0 | 1,0 | 1,0 | 1,0 | 1,0 | 1,0 | 1,0 | 0,2 | 0,4 |
| Scgb3a2 | 1,0 | 1,0 | 1,0 | 1,0 | 1,0 | 1,0 | 1,0 | 0,7 | 1,0 | 1,0 | 1,0 | 1,0 | 1,0 | 1,0 | 1,0 | 1,0 | 1,0 | 1,0 | 1,0 | 1,0 | 1,0 | 1,0 | 0,3 | 5,2 |
| Scn4b | 1,0 | 1,0 | 0,7 | 0,5 | 1,0 | 1,0 | 0,9 | 1,0 | 0,7 | 1,7 | 1,0 | 1,0 | 1,7 | 0,9 | 3,7 | 0,3 | 0,9 | 1,1 | 0,9 | 0,9 | 1,0 | 1,0 | 1,2 | 5,5 |
| Scx | 1,0 | 1,0 | 1,5 | 1,0 | 1,0 | 1,1 | 1,1 | 0,9 | 1,2 | 2,7 | 1,4 | 1,1 | 1,8 | 0,8 | 0,9 | 0,5 | 1,3 | 1,2 | 2,0 | 1,1 | 1,2 | 0,7 | 0,8 | 0,7 |
| *Sec14l3* | 1,0 | 1,0 | 1,0 | 1,0 | 1,0 | 1,0 | 1,1 | 1,1 | 1,0 | 1,0 | 0,7 | 1,0 | 1,0 | 1,0 | 1,0 | 1,0 | 1,0 | 1,0 | 1,0 | 1,0 | 1,0 | 1,0 | 0,4 | 2,3 |
| Selenbp2 | 0,8 | 1,1 | 2,0 | 1,2 | 1,0 | 1,0 | 1,3 | 1,2 | 1,0 | 1,1 | 1,1 | 1,7 | 1,7 | 0,5 | 1,2 | 1,3 | 1,5 | 2,0 | 1,2 | 1,7 | 1,0 | 0,9 | 1,3 | 1,6 |
| Sell | 1,0 | 1,0 | 1,1 | 1,0 | 1,0 | 1,0 | 0,7 | 0,9 | 1,0 | 1,0 | 1,0 | 1,0 | 1,0 | 1,0 | 1,0 | 1,2 | 1,0 | 1,0 | 0,9 | 1,0 | 0,8 | 1,2 | 0,6 | 1,0 |
| Serinc2 | 1,2 | 0,7 | 1,0 | 1,0 | 0,9 | 0,8 | 1,3 | 1,2 | 0,9 | 0,9 | 0,9 | 0,9 | 1,3 | 0,8 | 3,1 | 0,6 | 0,9 | 0,8 | 1,0 | 1,0 | 1,1 | 1,0 | 0,8 | 1,2 |
| Serpina10 | 1,0 | 1,0 | 1,0 | 1,0 | 1,0 | 1,0 | 1,0 | 1,0 | 0,9 | 0,4 | 1,0 | 1,0 | 1,0 | 1,0 | 1,0 | 1,0 | 1,0 | 1,0 | 0,7 | 1,1 | 1,0 | 1,0 | 1,0 | 1,0 |
| Serpina12 | 1,0 | 1,0 | 1,0 | 1,0 | 1,0 | 1,0 | 1,0 | 1,0 | 1,0 | 1,0 | 1,0 | 1,0 | 1,3 | 1,5 | 1,0 | 1,0 | 1,0 | 1,0 | 1,0 | 1,0 | 1,0 | 1,0 | 1,0 | 1,0 |
| Serpina1a | 0,8 | 0,4 | 1,0 | 0,1 | 1,0 | 1,0 | 1,3 | 1,0 | 1,0 | 1,0 | 0,8 | 1,0 | 1,0 | 1,0 | 1,0 | 1,0 | 1,0 | 1,0 | 0,6 | 1,2 | 1,0 | 1,0 | 1,2 | 1,0 |
| Serpina1b | 1,2 | 0,5 | 1,0 | 0,1 | 1,0 | 1,0 | 2,4 | 1,0 | 0,7 | 1,0 | 0,9 | 1,0 | 1,0 | 1,0 | 1,1 | 1,0 | 1,0 | 1,0 | 0,8 | 1,2 | 1,0 | 1,0 | 1,4 | 1,0 |
| Serpina1c | 0,6 | 0,3 | 1,0 | .0,1 | 1,0 | 1,0 | 2,1 | 1,0 | 0,7 | 1,3 | 1,0 | 1,0 | 1,0 | 1,0 | 1,3 | 1,0 | 1,0 | 1,0 | 0,9 | 0,7 | 1,0 | 1,0 | 0,8 | 1,0 |
| Serpina1d | 0,9 | 0,3 | 1,0 | 0,1 | 1,0 | 1,0 | 1,5 | 1,0 | 1,0 | 1,0 | 1,0 | 1,0 | 1,0 | 1,0 | 1,0 | 1,0 | 1,0 | 1,0 | 0,6 | 0,9 | 1,0 | 1,0 | 1,5 | 1,0 |
| Serpina1e | 1,0 | 0,1 | 1,0 | 0,2 | 0,9 | 1,0 | 1,9 | 1,0 | 1,1 | 1,0 | 1,0 | 1,0 | 1,4 | 0,7 | 1,0 | 1,0 | 1,0 | 1,0 | 1,1 | 1,2 | 1,0 | 1,0 | 1,0 | 1,0 |
| Serpina1f | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,2 | 1,0 | 1,0 |
| Serpina3c | 1,8 | 1,2 | 1,2 | 1,0 | 1,0 | 1,0 | 0,9 | 0,9 | 1,0 | 1,0 | 1,2 | 1,3 | 1,0 | 1,1 | 0,7 | 0,7 | 1,4 | 0,9 | 1,3 | 1,0 | 0,9 | 0,9 | 1,5 | 1,1 |
| Serpina3k | 1,3 | 0,3 | 1,0 | 0,1 | 0,7 | 1,0 | 2,9 | 1,0 | 1,1 | 0,7 | 1,4 | 1,0 | 0,6 | 1,0 | 1,3 | 1,0 | 1,0 | 1,0 | 1,0 | 1,2 | 1,0 | 1,0 | 0,9 | 1,0 |
| Serpina4-ps1 | 1,0 | 1,0 | 1,0 | 0,8 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Serpinb11 | 1,0 | 1,0 | 1,0 | 1,0 | 1,0 | 1,0 | 1,0 | 1,0 | 1,0 | 1,0 | 1,1 | 1,3 | 1,1 | 0,8 | 1,0 | 1,0 | 1,0 | 1,0 | 1,0 | 1,0 | 1,0 | 1,0 | 0,2 | 1,0 |
| Serpinb3a | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 5,8 | 1,0 |
| Serpinb6d | 1,0 | 1,0 | 1,0 | 1,0 | 1,0 | 1,0 | 1,0 | 1,0 | 1,0 | 1,0 | 1,0 | 1,0 | 0,3 | 0,7 | 1,0 | 1,0 | 1,0 | 1,0 | 1,0 | 1,0 | 1,0 | 1,0 | 1,0 | 1,0 |
| Serpinb6e | 1,0 | 1,0 | 1,0 | 1,0 | 1,0 | 1,0 | 1,0 | 1,0 | 1,0 | 1,0 | 1,0 | 1,0 | 4,7 | 9,3 | 1,0 | 1,0 | 1,0 | 1,0 | 1,0 | 1,0 | 1,0 | 1,0 | 1,0 | 1,0 |
| Serpinc1 | 1,0 | 1,0 | 1,0 | 0,5 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Serpinf2 | 1,0 | 1,0 | 1,0 | 0,5 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Sfrp5 | 0,8 | 1,4 | 1,1 | 1,2 | 0,8 | 1,2 | 1,0 | 1,0 | 1,0 | 1,0 | 1,3 | 1,2 | 1,9 | 1,5 | 0,8 | 0,7 | 1,0 | 1,3 | 1,0 | 1,0 | 1,0 | 1,0 | 0,7 | 0,8 |
| Sgca | 1,0 | 1,0 | 0,9 | 0,8 | 1,0 | 1,0 | 1,0 | 1,0 | 1,0 | 1,0 | 1,0 | 1,0 | 2,4 | 1,0 | 2,6 | 0,5 | 1,0 | 1,1 | 1,0 | 1,0 | 1,2 | 0,8 | 1,2 | 7,7 |
| Sgcg | 1,0 | 1,0 | 1,2 | 0,8 | 1,0 | 1,0 | 0,7 | 0,8 | 1,0 | 1,0 | 1,0 | 1,0 | 2,2 | 1,0 | 4,0 | 0,3 | 1,0 | 1,1 | 1,0 | 1,0 | 1,0 | 1,0 | 1,1 | 9,1 |
| Sh3bp2 | 0,9 | 1,1 | 1,2 | 1,0 | 1,0 | 1,0 | 1,0 | 0,9 | 1,0 | 1,0 | 1,0 | 1,0 | 0,8 | 1,1 | 1,0 | 1,1 | 0,6 | 0,5 | 0,9 | 1,0 | 0,9 | 1,0 | 0,8 | 1,2 |
| *Sh3gl2* | 1,0 | 0,9 | 1,0 | 1,0 | 1,0 | 1,0 | 0,7 | 0,8 | 1,0 | 1,0 | 1,0 | 1,0 | 1,0 | 1,4 | 1,0 | 0,7 | 1,0 | 1,0 | 1,0 | 1,0 | 1,1 | 1,3 | 1,3 | 0,8 |
| Shc4 | 1,0 | 0,8 | 1,0 | 1,0 | 0,9 | 0,9 | 1,0 | 1,0 | 1,0 | 1,0 | 1,0 | 1,0 | 0,8 | 1,1 | 1,0 | 1,0 | 1,0 | 1,0 | 1,0 | 1,0 | 0,9 | 1,1 | 1,0 | 1,0 |
| Shh | 0,5 | 0,6 | 1,0 | 1,0 | 1,0 | 1,0 | 1,2 | 1,0 | 1,0 | 1,0 | 1,0 | 1,0 | 0,3 | 0,6 | 1,0 | 1,0 | 1,0 | 1,0 | 1,0 | 1,0 | 1,0 | 1,0 | 1,0 | 1,0 |
| Foxn3 | 0,3 | 0,7 | 0,3 | 1,2 | 0,7 | 0,9 | 0,6 | 1,1 | 1,0 | 1,0 | 0,6 | 0,9 | 0,3 | 1,2 | 0,2 | 1,1 | 0,5 | 1,3 | 0,0 | 0,7 | 1,0 | 1,0 | 0,2 | 1,0 |
| Slc16a3 | 1,0 | 1,0 | 1,3 | 1,1 | 0,8 | 0,8 | 1,3 | 1,1 | 1,0 | 1,0 | 1,5 | 1,2 | 1,2 | 0,9 | 1,0 | 0,7 | 1,0 | 0,9 | 0,9 | 1,0 | 1,2 | 1,1 | 0,9 | 5,0 |
| Slc16a9 | 0,8 | 0,8 | 0,7 | 0,8 | 1,1 | 0,8 | 1,3 | 1,4 | 1,0 | 1,0 | 1,0 | 1,4 | 0,8 | 1,0 | 0,8 | 1,0 | 0,9 | 0,4 | 1,1 | 1,2 | 1,1 | 0,8 | 1,1 | 1,2 |
| Slc17a9 | 1,0 | 0,9 | 1,0 | -1,1 | 1,0 | 1,0 | 0,9 | 1,2 | 1,5 | 1,3 | 0,6 | 0,7 | 0,7 | 0,6 | 1,0 | 1,0 | 1,0 | 1,0 | 1,1 | 1,1 | 1,2 | 0,8 | 0,5 | 1,0 |
| Slc1a1 | 1,0 | 1,0 | 1,6 | 0,8 | 1,1 | 1,1 | 0,9 | 1,0 | 1,0 | 1,0 | 1,6 | 1,5 | 1,4 | 1,3 | 1,0 | 1,0 | 1,1 | 1,2 | 1,0 | 1,0 | 1,0 | 1,0 | 1,7 | 1,7 |
| Slc22a26 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Slc22a27 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Slc22a28 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Slc22a30 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Slc22a7 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Slc25a30 | 0,5 | 0,5 | 1,2 | 0,9 | 0,8 | 0,9 | 1,1 | 2,1 | 1,0 | 1,0 | 1,3 | 0,9 | 0,9 | 1,1 | 1,7 | 0,7 | 0,6 | 0,5 | 1,0 | 0,9 | 1,0 | 0,8 | 0,6 | 0,8 |
| Slc25a47 | 1,6 | 0,9 | 1,8 | 1,1 | 0,8 | 1,3 | 1,4 | 1,3 | 1,1 | 1,0 | 1,0 | 1,0 | 1,4 | 1,0 | 0,9 | 1,0 | 0,9 | 0,9 | 0,5 | 1,1 | 0,9 | 0,8 | 1,3 | 1,3 |
| Slc30a10 | 1,0 | 1,0 | 1,0 | 1,0 | 0,8 | 0,9 | 1,0 | 1,0 | 1,0 | 1,0 | 1,0 | 1,0 | 1,0 | 1,0 | 1,6 | 0,9 | 1,0 | 1,0 | 1,0 | 1,0 | 1,0 | 1,0 | 1,0 | 1,0 |
| | AG | | Aorta | | Brain | | Lung | | Pancreas | | PG | | Skin | | Skull | | SM | | Spleen | | Testis | | TG | |
| | E | M | E | M | E | M | E | M | E | M | E | M | E | M | E | M | E | M | E | M | E | M | E | M |
| Slc34a2 | 1,0 | 1,0 | 1,0 | 1,0 | 1,0 | 1,0 | 1,0 | 1,2 | 1,0 | 1,0 | 1,0 | 1,0 | 1,0 | 0,8 | 1,0 | 1,0 | 1,0 | 1,0 | 1,0 | 1,0 | 0,9 | 1,0 | 1,0 | 1,0 |
| Slc35f2 | 0,5 | 0,6 | 1,0 | 1,0 | 1,5 | 1,2 | 1,0 | 1,0 | 1,0 | 1,0 | 1,0 | 1,0 | 0,8 | 1,0 | 1,1 | 1,0 | 1,0 | 1,0 | 0,9 | 1,1 | 1,0 | 1,3 | 0,9 | 0,8 |
| Slc38a3 | 1,0 | 1,0 | 1,0 | 0,4 | 0,9 | 1,1 | 1,0 | 1,0 | 0,6 | 0,9 | 1,1 | 1,5 | 2,4 | 0,8 | 2,6 | 0,9 | 1,0 | 1,5 | 1,0 | 1,0 | 1,0 | 1,0 | 1,1 | 0,9 |
| Slc38a5 | 1,0 | 1,0 | 1,0 | 1,0 | 1,0 | 1,0 | 0,5 | 0,6 | 0,5 | 0,6 | 1,0 | 1,0 | 1,0 | 1,3 | 1,3 | 1,6 | 1,0 | 1,0 | 4,7 | 0,3 | 1,1 | 1,0 | 1,0 | 1,0 |
| Slc43a1 | 2,2 | 1,7 | 1,8 | 3,0 | 1,0 | 1,0 | 0,8 | 1,2 | 1,0 | 1,0 | 1,4 | 1,2 | 1,9 | 1,8 | 1,5 | 1,6 | 2,1 | 2,1 | 3,7 | 0,5 | 1,0 | 1,0 | 1,2 | 1,8 |
| Slc4a1 | 1,0 | 1,0 | 1,0 | 1,0 | 1,0 | 1,0 | 1,2 | 1,4 | 1,0 | 1,0 | 1,0 | 1,0 | 1,4 | 0,7 | 1,7 | 1,6 | 1,0 | 1,0 | 6,1 | 0,4 | 1,0 | 1,0 | 1,0 | 1,0 |
| Slc51b | 1,0 | 1,0 | 1,5 | 0,8 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| *Slc7a12* | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Slc7a5 | 1,0 | 0,9 | 1,2 | 1,0 | 1,0 | 1,1 | 1,2 | 1,4 | 1,6 | 2,1 | 0,9 | 0,8 | 0,8 | 0,8 | 1,4 | 1,5 | 1,2 | 1,0 | 1,4 | 0,9 | 0,9 | 0,8 | 0,6 | 1,1 |
| Slc7a8 | 1,0 | 1,0 | 5,1 | 6,9 | 1,0 | 1,0 | 1,0 | 1,3 | 1,2 | 1,2 | 1,0 | 1,1 | 1,1 | 0,7 | 1,2 | 1,3 | 0,7 | 1,3 | 1,1 | 1,3 | 0,9 | 1,0 | 2,2 | 1,4 |
| Slc9a8 | 1,0 | 1,2 | 1,0 | 0,9 | 1,0 | 1,0 | 1,0 | 1,0 | 1,2 | 1,2 | 1,1 | 1,0 | 1,1 | 1,0 | 0,9 | 1,2 | 1,0 | 1,2 | 0,9 | 1,1 | 1,0 | 1,0 | 1,1 | 1,0 |
| Slco1a1 | 1,0 | 1,0 | 1,0 | -1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Slco1a4 | 1,0 | 1,0 | 1,0 | 1,0 | 1,1 | 1,0 | 1,0 | 1,0 | 1,0 | 1,0 | 1,0 | 1,0 | 1,0 | 1,0 | 1,3 | 1,1 | 1,0 | 1,0 | 1,0 | 1,0 | 0,9 | 1,1 | 1,0 | 1,0 |
| Slfn4 | 1,0 | 1,0 | 1,0 | 1,0 | 1,0 | 1,0 | 1,8 | 1,8 | 1,0 | 1,0 | 1,0 | 1,0 | 0,3 | 0,5 | 0,7 | 0,7 | 1,0 | 1,0 | 1,2 | 1,4 | 1,0 | 1,0 | 1,0 | 1,0 |
| Slpi | 1,4 | 1,0 | 1,5 | 1,3 | 1,0 | 1,0 | 1,5 | 1,3 | 1,0 | 1,6 | 1,0 | 1,0 | 1,7 | 3,4 | 0,9 | 1,1 | 1,0 | 1,0 | 1,2 | 0,6 | 1,0 | 1,0 | 22,6 | 1,0 |
| Smco1 | 1,0 | 1,0 | 1,0 | 1,0 | 1,0 | 1,0 | 1,0 | 1,0 | 1,0 | 1,0 | 1,0 | 1,0 | 1,6 | 0,9 | 5,1 | 0,3 | 0,7 | 0,8 | 1,0 | 1,0 | 1,0 | 1,0 | 0,9 | 5,3 |

[Fig. 23-13]

282/310

| | | | | | | | | | | | | | | | | | | | | | | | | |
|---|---|---|---|---|---|---|---|---|---|---|---|---|---|---|---|---|---|---|---|---|---|---|---|---|
| Smcp | 1,0 | 1,0 | 0,8 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Smim24 | 0,9 | 1,0 | 1,3 | 1,6 | 1,0 | 0,7 | 0,9 | 0,8 | 0,2 | 0,3 | 1,0 | 0,6 | 1,0 | 1,5 | 0,8 | 1,0 | 1,0 | 1,0 | 0,7 | 1,1 | 1,1 | 0,8 | 1,0 | 1,0 |
| Smpx | 1,3 | 1,0 | 0,5 | 1,0 | 1,9 | 1,0 | 0,9 | 0,8 | 1,0 | 1,0 | 1,0 | 1,0 | 1,6 | 0,8 | 3,7 | 0,4 | 1,0 | 0,7 | 1,0 | 1,0 | 0,9 | 1,4 | 1,3 | 10,6 |
| Smr3a | 0,8 | 1,0 | 1,0 | 1,2 | 1,0 | 1,0 | 1,0 | 0,5 | 1,2 | 1,0 | 0,4 | 1,0 | 1,0 | 1,0 | 1,0 | 1,0 | 1,0 | 0,3 | 1,0 | 1,0 | 1,0 | 1,0 | 1,2 | 0,7 |
| Smtnl1 | 1,0 | 1,0 | 1,0 | 1,0 | 1,0 | 1,0 | 1,0 | 1,0 | 1,0 | 1,0 | 1,0 | 1,0 | 1,6 | 0,5 | 4,5 | 0,5 | 1,2 | 0,9 | 1,0 | 1,0 | 1,0 | 1,2 | 1,2 | 17,0 |
| Smtnl2 | 1,0 | 1,2 | 1,0 | 0,5 | 1,0 | 1,0 | 1,0 | 1,1 | 1,0 | 1,0 | 1,2 | 1,0 | 1,4 | 0,5 | 1,8 | 0,1 | 0,7 | 0,5 | 0,9 | 1,0 | 0,9 | 1,2 | 0,7 | 0,7 |
| Smyd1 | 1,0 | 1,0 | 1,0 | 0,9 | 0,7 | 0,8 | 0,6 | 0,8 | 1,0 | 1,0 | 1,0 | 1,0 | 1,8 | 0,8 | 3,7 | 0,3 | 0,9 | 1,0 | 1,0 | 1,2 | 1,0 | 1,3 | 1,2 | 7,6 |
| Sncg | 0,9 | 2,9 | 1,0 | 1,0 | 0,8 | 1,0 | 1,1 | 0,6 | 1,0 | 1,0 | 0,6 | 0,7 | 0,9 | 1,1 | 0,9 | 1,0 | 0,5 | 0,4 | 1,1 | 1,9 | 1,0 | 1,0 | 1,0 | 1,2 |
| Hipk2 | 0,3 | 1,0 | 1,0 | 1,0 | 1,0 | 1,0 | 1,0 | 1,0 | 1,0 | 1,0 | 1,0 | 1,0 | 1,0 | 23,3 | 1,0 | 15,4 | 1,0 | 1,0 | 1,0 | 23,5 | 1,0 | 1,0 | 1,0 | 0,0 |
| Nptxr | 0,3 | 1,1 | 1,0 | 21,5 | 1,2 | 0,6 | 0,1 | 1,0 | 0,0 | 1,0 | 1,0 | 1,3 | 1,0 | 0,9 | 47,6 | 30,8 | 1,0 | 1,0 | 0,5 | 70,5 | 0,2 | 24,9 | 0,3 | 0,0 |
| Socs1 | 0,9 | 0,9 | 0,9 | 1,0 | 1,0 | 0,8 | 0,7 | 0,7 | 1,0 | 1,0 | 1,3 | 0,8 | 0,7 | 1,3 | 0,8 | 0,7 | 0,3 | 1,6 | 0,9 | 1,1 | 1,4 | 0,8 | 0,5 | 0,5 |
| Socs3 | 0,9 | 1,3 | 1,1 | 1,3 | 1,1 | 1,0 | 1,2 | 1,2 | 1,3 | 2,2 | 1,2 | 1,2 | 1,1 | 1,2 | 1,1 | 1,2 | 0,3 | 1,2 | 1,2 | 1,2 | 1,0 | 1,0 | 1,4 | 1,3 |
| Sost | 1,0 | 1,0 | 1,3 | 0,9 | 1,0 | 1,0 | 1,0 | 1,0 | 1,0 | 1,0 | 1,0 | 1,0 | 0,9 | 1,0 | 0,6 | 0,8 | 1,0 | 1,0 | 1,0 | 1,0 | 1,2 | 0,9 | 1,0 | 1,0 |
| Sox6 | 0,9 | 0,8 | 1,1 | 1,1 | 0,9 | 0,9 | 1,0 | 1,0 | 0,9 | 0,9 | 1,1 | 1,1 | 1,6 | 1,4 | 1,6 | 1,0 | 1,2 | 1,5 | 6,0 | 0,5 | 1,0 | 1,0 | 1,3 | 3,4 |
| Spag11a | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Spag11b | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Sparc | 1,3 | 1,6 | 0,8 | 0,8 | 1,3 | 1,2 | 0,9 | 0,9 | 0,7 | 0,8 | 0,7 | 0,7 | 0,3 | 0,3 | 0,5 | 0,7 | 0,4 | 0,4 | 0,8 | 0,9 | 1,0 | 1,1 | 0,7 | 0,8 |
| Spata3 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,9 | 1,0 | 1,0 |
| Spc24 | 1,0 | 1,3 | 1,0 | 1,0 | 1,0 | 1,1 | 0,7 | 0,9 | 1,0 | 1,0 | 1,0 | 1,0 | 0,3 | 0,5 | 1,0 | 1,3 | 1,2 | 1,5 | 1,3 | 0,4 | 1,2 | 1,1 | 0,8 | 1,0 |
| Spin2c | 1,0 | 1,0 | 1,0 | 1,0 | 1,2 | 1,2 | 0,7 | 1,0 | 1,0 | 1,0 | 0,9 | 1,0 | 0,7 | 1,0 | 1,0 | 1,6 | 1,0 | 1,0 | 0,9 | 1,4 | 1,0 | 1,1 | 1,9 | 0,9 |
| Spink10 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Spink11 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Spink12 | 1,0 | 1,0 | 1,0 | 1,0 | 1,0 | 1,0 | 1,0 | 1,0 | 1,0 | 1,0 | 1,0 | 1,0 | 3,6 | 2,9 | 1,0 | 1,0 | 1,0 | 1,0 | 1,0 | 1,0 | 1,0 | 1,0 | 1,0 | 1,0 |
| Spink2 | 1,0 | 1,0 | 1,0 | 1,4 | 1,0 | 1,0 | 1,5 | 1,3 | 1,0 | 1,0 | 1,0 | 1,0 | 1,1 | 1,0 | 1,0 | 1,0 | 1,0 | 1,0 | 1,0 | 1,0 | 1,1 | 1,0 | 1,0 | 1,0 |
| Spink3 | 1,0 | 1,0 | 1,0 | 1,0 | 1,0 | 1,0 | 1,0 | 1,0 | 1,5 | 1,3 | 1,0 | 1,0 | 1,0 | 1,0 | 1,0 | 1,0 | 1,0 | 1,0 | 1,0 | 1,0 | 1,0 | 1,0 | 1,0 | 1,0 |
| Spink4 | 1,0 | 1,0 | 1,0 | 1,0 | 1,0 | 1,0 | 1,3 | 0,7 | 1,0 | 1,0 | 1,0 | 1,0 | 1,0 | 1,0 | 1,4 | 1,9 | 1,0 | 1,0 | 1,0 | 1,0 | 1,0 | 0,8 | 1,0 | 1,0 |
| Spink5 | 1,0 | 1,0 | 1,0 | 1,0 | 1,0 | 1,0 | 1,0 | 1,1 | 1,0 | 1,0 | 1,0 | 1,0 | 1,3 | 1,4 | 1,0 | 1,0 | 1,0 | 1,0 | 1,0 | 1,0 | 1,0 | 1,0 | 1,0 | 1,0 |
| Spink8 | 1,0 | 1,2 | 1,0 | 1,0 | 1,2 | 2,1 | 1,0 | 1,0 | 1,0 | 1,0 | 1,0 | 1,1 | 1,0 | 1,0 | 1,0 | 1,0 | 1,0 | 1,0 | 1,0 | 1,0 | 2,4 | 10,5 | 5,0 | 14,0 |
| Spint1 | 0,8 | 1,0 | 1,0 | 1,0 | 1,3 | 1,1 | 1,0 | 1,1 | 0,9 | 1,2 | 1,0 | 1,0 | 1,0 | 0,8 | 1,0 | 1,0 | 1,0 | 1,0 | 1,2 | 1,2 | 1,1 | 1,2 | 0,9 | 1,1 |
| Spint2 | 1,1 | 1,1 | 1,0 | 1,1 | 0,9 | 1,4 | 1,0 | 0,9 | 1,0 | 1,1 | 0,9 | 0,9 | 1,1 | 1,0 | 1,1 | 1,1 | 1,7 | 0,9 | 1,0 | 1,1 | 1,1 | 1,0 | 0,9 | 0,9 |
| Spint4 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Spint5 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,8 | 1,0 | 1,0 |
| Spp1 | 1,4 | 1,2 | 1,0 | 1,0 | 1,0 | 0,9 | 1,8 | 1,9 | 0,8 | 1,3 | 0,6 | 0,6 | 0,9 | 2,0 | 1,8 | 3,5 | 1,2 | 0,6 | 0,9 | 0,6 | 1,0 | 1,0 | 1,7 | 1,0 |
| Sprr1a | 1,0 | 1,0 | 1,0 | 1,0 | 1,0 | 1,0 | 1,7 | 1,3 | 1,0 | 1,0 | 1,0 | 1,0 | 1,5 | 1,0 | 1,0 | 1,0 | 1,0 | 1,0 | 1,0 | 1,0 | 1,0 | 1,0 | 20,8 | 1,0 |
| Sprr2a2 | 1,0 | 1,0 | 1,0 | 1,0 | 1,0 | 1,0 | 1,0 | 1,0 | 0,8 | 1,0 | 1,0 | 1,0 | 0,3 | 2,6 | 1,0 | 1,0 | 1,7 | 1,0 | 1,0 | 1,0 | 1,0 | 0,9 | 1,4 | 1,0 |
| Sprr2h | 1,0 | 1,0 | 1,0 | 1,0 | 1,0 | 1,0 | 1,0 | 1,0 | 1,0 | 1,0 | 1,0 | 1,0 | 3,5 | 0,7 | 1,0 | 1,0 | 1,0 | 1,0 | 1,0 | 1,0 | 1,0 | 1,0 | 1,0 | 1,0 |
| Sprr3 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 49,5 | 1,0 |
| Spry4 | 1,2 | 0,9 | 1,6 | 0,9 | 1,0 | 0,8 | 1,2 | 1,1 | 1,0 | 1,0 | 1,4 | 1,2 | 1,2 | 1,1 | 1,1 | 1,0 | 0,7 | 0,8 | 0,8 | 1,2 | 1,0 | 1,2 | 1,3 | 0,7 |
| Fads6 | 0,3 | 1,0 | 1,0 | 0,9 | 0,9 | 1,0 | 1,0 | 0,7 | 1,0 | 1,0 | 0,9 | 1,0 | 1,0 | 0,6 | 67,5 | 1,0 | 1,0 | 0,0 | 1,0 | 1,0 | 1,0 | 1,0 | 0,9 | 0,9 |
| Sptb | 0,7 | 0,7 | 1,0 | 1,0 | 1,0 | 0,9 | 0,9 | 1,0 | 1,0 | 1,0 | 1,0 | 1,0 | 2,6 | 1,2 | 1,9 | 1,1 | 1,4 | 1,6 | 5,7 | 0,3 | 1,0 | 0,8 | 1,5 | 2,6 |
| Spz1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,1 | 1,0 | 1,0 |
| Srd5a2 | 0,6 | 0,8 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Srl | 0,7 | 0,7 | 0,7 | 0,7 | 0,9 | 0,7 | 0,7 | 0,9 | 1,0 | 1,0 | 0,7 | 1,1 | 1,6 | 0,7 | 3,9 | 0,3 | 0,8 | 1,0 | 0,8 | 1,0 | 0,9 | 1,1 | 1,0 | 7,4 |
| St14 | 1,0 | 1,0 | 1,0 | 1,0 | 1,0 | 1,0 | 1,0 | 1,1 | 0,9 | 1,3 | 0,6 | 0,8 | 1,0 | 0,7 | 1,0 | 1,3 | 1,0 | 1,0 | 0,8 | 1,1 | 1,0 | 1,0 | 0,9 | 0,9 |
| St6gal1 | 0,8 | 1,1 | 1,1 | 1,0 | 1,0 | 0,9 | 0,9 | 0,9 | 1,0 | 1,0 | 1,2 | 0,9 | 0,9 | 0,9 | 1,0 | 1,2 | 0,7 | 1,0 | 0,8 | 0,8 | 1,0 | 0,9 | 0,6 | 0,6 |
| St6galnac2 | 0,9 | 0,7 | 1,1 | 1,2 | 1,2 | 0,9 | 0,9 | 0,9 | 0,7 | 1,1 | 0,7 | 0,9 | 1,1 | 1,1 | 0,8 | 1,3 | 0,7 | 0,6 | 1,1 | 1,1 | 1,1 | 1,0 | 0,8 | 0,8 |
| St8sia2 | 1,0 | 1,0 | 1,0 | 1,0 | 1,0 | 0,8 | 0,9 | 1,0 | 1,0 | 1,0 | 1,1 | 1,0 | 0,8 | 0,8 | 1,0 | 1,0 | 1,0 | 1,0 | 1,0 | 1,0 | 0,9 | 0,9 | 0,7 | 0,3 |
| Stac3 | 1,0 | 1,0 | 1,0 | 1,0 | 1,0 | 0,8 | 1,0 | 1,0 | 1,0 | 1,0 | 1,5 | 1,0 | 1,7 | 0,7 | 2,6 | 0,4 | 0,8 | 0,7 | 1,0 | 1,0 | 1,0 | 1,0 | 1,0 | 7,3 |
| Stc2 | 1,0 | 1,1 | 0,7 | 1,4 | 1,1 | 0,7 | 0,8 | 0,9 | 1,8 | 1,7 | 1,6 | 1,7 | 1,6 | 1,3 | 0,7 | 0,8 | 5,4 | 6,2 | 0,9 | 1,1 | 1,0 | 1,0 | 1,0 | 0,9 |
| Stfa1 | 1,0 | 1,0 | 1,0 | 1,0 | 1,0 | 1,0 | 1,0 | 1,0 | 1,0 | 1,0 | 1,0 | 1,0 | 0,5 | 6,8 | 1,5 | 5,2 | 1,0 | 1,0 | 1,0 | 2,0 | 1,0 | 1,0 | 1,0 | 1,0 |
| Stfa2l1 | 1,0 | 1,0 | 1,0 | 1,0 | 1,0 | 1,0 | 6,2 | 3,9 | 1,0 | 1,0 | 1,0 | 1,0 | 1,0 | 1,0 | 1,5 | 1,8 | 1,0 | 1,0 | 7,5 | 2,9 | 1,0 | 1,0 | 1,0 | 1,0 |
| Stfa3 | 1,0 | 1,0 | 1,0 | 1,0 | 1,0 | 1,0 | 1,0 | 1,0 | 1,0 | 1,0 | 1,0 | 1,0 | 0,3 | 2,6 | 1,0 | 1,0 | 1,0 | 1,0 | 1,0 | 1,0 | 1,0 | 1,0 | 1,0 | 1,0 |
| Sult5a1 | 1,3 | 0,6 | 1,7 | 1,1 | 1,0 | 1,0 | 1,1 | 1,0 | 1,0 | 1,0 | 1,3 | 1,7 | 1,5 | 1,0 | 0,7 | 0,9 | 0,5 | 0,8 | 1,0 | 1,0 | 1,0 | 1,3 | 0,6 | 1,1 |
| Susd4 | 0,9 | 1,0 | 1,0 | 1,0 | 1,0 | 0,9 | 0,9 | 1,3 | 1,0 | 1,0 | 1,0 | 0,9 | 1,0 | 0,9 | 1,3 | 0,9 | 1,0 | 1,0 | 1,0 | 1,0 | 1,0 | 1,0 | 1,8 | 1,2 |
| Sycn | 0,4 | 0,7 | 1,0 | 1,0 | 1,0 | 1,2 | 1,1 | 1,0 | 1,6 | 1,9 | 0,9 | 0,8 | 1,2 | 1,0 | 1,2 | 1,1 | 1,0 | 1,0 | 1,0 | 0,8 | 1,0 | 1,0 | 1,1 | 1,0 |
| Syn2 | 0,9 | 1,1 | 1,6 | 1,7 | 1,0 | 1,0 | 0,9 | 0,8 | 1,3 | 1,3 | 1,2 | 1,1 | 1,5 | 1,5 | 1,3 | 0,7 | 1,3 | 1,0 | 1,0 | 1,0 | 1,0 | 1,1 | 5,0 | 2,9 |
| Synm | 0,8 | 0,4 | 2,0 | 1,6 | 0,9 | 0,9 | 0,9 | 1,0 | 1,1 | 1,0 | 1,3 | 1,4 | 1,7 | 0,8 | 2,1 | 0,3 | 0,9 | 0,9 | 0,9 | 1,1 | 0,9 | 0,9 | 0,9 | 6,5 |
| Synpo2 | 0,7 | 0,8 | 1,2 | 0,8 | 1,0 | 0,9 | 0,7 | 1,0 | 1,0 | 1,0 | 1,1 | 1,4 | 1,6 | 1,1 | 3,5 | 0,2 | 1,0 | 1,5 | 0,8 | 1,0 | 1,0 | 1,0 | 1,3 | 5,3 |
| Synpo2l | 1,0 | 1,0 | 0,8 | 0,6 | 1,0 | 1,0 | 0,9 | 0,9 | 1,0 | 1,0 | 1,0 | 1,0 | 1,3 | 0,9 | 3,3 | 0,2 | 0,7 | 1,2 | 1,0 | 1,0 | 1,0 | 1,0 | 0,9 | 9,2 |
| Sypl2 | 1,0 | 1,0 | 1,0 | 1,0 | 0,8 | 0,8 | 1,0 | 1,0 | 1,0 | 1,0 | 1,0 | 1,0 | 1,2 | 0,6 | 3,4 | 0,2 | 0,6 | 0,8 | 1,0 | 1,0 | 1,0 | 1,0 | 0,8 | 9,2 |
| Syt16 | 1,0 | 1,0 | 1,0 | 1,0 | 1,1 | 1,1 | 1,0 | 1,0 | 1,0 | 1,0 | 1,2 | 1,1 | 1,0 | 1,0 | 1,0 | 1,0 | 1,0 | 1,0 | 1,0 | 1,0 | 1,0 | 1,0 | 1,0 | 1,0 |
| Sytl4 | 0,8 | 0,6 | 1,1 | 1,0 | 1,0 | 1,0 | 1,1 | 0,9 | 0,6 | 1,0 | 0,7 | 0,7 | 0,6 | 0,9 | 0,7 | 0,9 | 1,0 | 1,0 | 0,9 | 0,8 | 1,0 | 1,1 | 1,7 | 1,2 |
| Tacstd2 | 1,0 | 1,0 | 1,0 | 1,0 | 1,0 | 1,0 | 1,3 | 1,3 | 1,0 | 1,0 | 0,7 | 1,1 | 1,2 | 1,0 | 0,7 | 0,9 | 1,0 | 1,0 | 1,0 | 0,8 | 1,0 | 1,0 | 1,1 | 1,4 |
| Tbc1d8 | 0,7 | 0,5 | 1,3 | 1,1 | 1,1 | 1,0 | 1,1 | 1,2 | 0,9 | 0,9 | 0,9 | 1,2 | 1,3 | 1,1 | 0,9 | 0,8 | 1,1 | 1,1 | 1,0 | 1,2 | 1,0 | 1,0 | 1,2 | 0,8 |
| Tbx1 | 1,0 | 1,0 | 0,8 | 0,8 | 0,9 | 0,9 | 1,1 | 1,1 | 1,0 | 1,0 | 1,0 | 1,0 | 0,7 | 0,8 | 3,2 | 0,4 | 1,3 | 1,4 | 1,0 | 1,0 | 1,0 | 0,6 | 5,0 | 2,6 |
| Tbx15 | 1,0 | 1,0 | 1,0 | 1,0 | 1,0 | 1,0 | 1,0 | 1,0 | 1,0 | 1,0 | 0,9 | 1,3 | 1,4 | 1,1 | 2,0 | 0,5 | 0,9 | 1,0 | 1,0 | 1,0 | 1,0 | 1,0 | 1,4 | 8,4 |
| Tcap | 5,0 | 0,5 | 0,9 | 1,3 | 1,2 | 1,3 | 0,7 | 0,7 | 1,0 | 1,0 | 1,0 | 1,5 | 2,3 | 1,1 | 4,9 | 0,5 | 1,1 | 0,9 | 1,1 | 1,4 | 1,1 | 1,0 | 1,5 | 16,8 |
| Tcea3 | 1,0 | 1,0 | 0,5 | 1,0 | 1,0 | 1,0 | 0,9 | 0,9 | 1,3 | 1,3 | 1,0 | 1,0 | 2,4 | 1,1 | 1,8 | 0,5 | 1,2 | 1,2 | 1,0 | 1,0 | 1,0 | 1,0 | 1,1 | 6,8 |
| Tceal3 | 0,9 | 0,6 | 1,2 | 1,3 | 1,0 | 1,2 | 0,5 | 0,7 | 1,0 | 1,0 | 0,9 | 1,0 | 0,6 | 1,0 | 0,8 | 1,0 | 0,9 | 1,0 | 0,8 | 1,0 | 0,9 | 1,5 | 0,8 | 1,0 |
| Tceal7 | 1,0 | 1,0 | 1,0 | 1,0 | 1,2 | 1,1 | 1,0 | 1,0 | 1,0 | 1,0 | 1,8 | 0,9 | 1,6 | 0,5 | 1,8 | 0,2 | 0,6 | 0,9 | 1,0 | 1,0 | 1,0 | 1,0 | 1,4 | 1,0 |
| Tcf7 | 0,9 | 0,9 | 0,6 | 1,0 | 0,9 | 0,8 | 0,9 | 1,0 | 1,0 | 1,0 | 1,2 | 1,0 | 0,7 | 0,7 | 1,1 | 1,3 | 1,0 | 1,0 | 1,0 | 0,9 | 0,9 | 1,0 | 0,3 | 1,4 |
| Tcp11 | 1,0 | 1,0 | 1,0 | 1,0 | 1,0 | 1,0 | 1,3 | 1,2 | 1,0 | 1,0 | 2,1 | 1,7 | 1,0 | 1,0 | 1,0 | 1,0 | 1,0 | 1,0 | 1,0 | 1,0 | 1,1 | 0,8 | 1,0 | 1,0 |
| Teddm1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Tekt1 | 1,0 | 1,0 | 1,0 | 1,0 | 0,7 | 0,8 | 1,1 | 0,8 | 1,0 | 1,0 | 1,1 | 1,2 | 1,0 | 1,0 | 1,0 | 1,0 | 0,9 | 0,7 | 1,0 | 1,0 | 1,0 | 1,0 | 0,9 | 0,2 |
| Tfap2a | 1,0 | 1,0 | 1,0 | 1,0 | 1,0 | 1,0 | 1,0 | 1,0 | 1,0 | 1,0 | 1,0 | 1,0 | 0,9 | 0,9 | 1,0 | 1,0 | 1,0 | 1,0 | 1,0 | 1,0 | 1,0 | 1,0 | 1,0 | 1,0 |
| Tfap2b | 0,8 | 0,6 | 1,0 | 1,0 | 0,7 | 0,8 | 1,0 | 1,0 | 1,0 | 1,0 | 1,0 | 1,0 | 0,9 | 0,9 | 1,0 | 1,0 | 1,0 | 1,0 | 1,0 | 1,0 | 1,0 | 1,0 | 1,0 | 1,0 |
| Tff1 | 1,0 | 1,0 | 1,0 | 1,0 | 1,0 | 1,0 | 1,0 | 0,8 | 0,5 | 1,0 | 1,0 | 1,0 | 1,0 | 1,0 | 1,0 | 1,0 | 1,0 | 0,2 | 0,7 | 1,0 | 1,0 | 1,0 | 0,3 | 0,4 |
| Tff2 | 1,0 | 1,0 | 1,0 | 1,0 | 1,0 | 1,0 | 0,9 | 0,8 | 0,8 | 1,0 | 1,0 | 1,0 | 1,0 | 1,0 | 1,0 | 1,0 | 1,0 | 1,0 | 1,0 | 1,0 | 1,0 | 1,0 | 0,3 | 0,0 |
| Tff3 | 1,0 | 1,0 | 1,0 | 1,0 | 1,0 | 1,0 | 1,0 | 1,0 | 1,0 | 1,0 | 1,0 | 1,0 | 1,0 | 1,1 | 0,7 | 1,0 | 1,0 | 1,0 | 1,0 | 1,0 | 1,0 | 1,5 | 1,0 | 1,0 |
| Tgif2lx2 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,6 | 20,4 | 1,0 | 1,0 |
| Tgm3 | 1,0 | 1,0 | 1,0 | 1,0 | 1,0 | 1,0 | 1,0 | 1,0 | 1,0 | 1,0 | 1,0 | 1,0 | 1,9 | 0,4 | 1,0 | 1,0 | 1,0 | 1,0 | 1,0 | 1,0 | 1,0 | 1,0 | 5,4 | 1,0 |
| | AG | | Aorta | | Brain | | Lung | | Pancreas | | PG | | Skin | | Skull | | SM | | Spleen | | Testis | | TG | |
| | E | M | E | M | E | M | E | M | E | M | E | M | E | M | E | M | E | M | E | M | E | M | E | M |
| Tgoln2 | 1,0 | 1,0 | 7,3 | 1,0 | 1,0 | 1,0 | 1,0 | 1,0 | 0,5 | 1,5 | 1,0 | 1,4 | 1,0 | 3,5 | 1,0 | 0,4 | 1,4 | 1,0 | 0,3 | 1,0 | 1,3 | 1,2 | 1,0 | 0,1 |

[Fig. 23-14]

283/310

| | | | | | | | | | | | | | | | | | | | | | | | | |
|---|---|---|---|---|---|---|---|---|---|---|---|---|---|---|---|---|---|---|---|---|---|---|---|---|
| Thrsp | 1,1 | 1,2 | 0,7 | 0,8 | 1,1 | 1,1 | 1,2 | 0,2 | 0,7 | 0,4 | 1,2 | [illegible] | 1,6 | 1,8 | 2,2 | 2,3 | 0,7 | 0,5 | 2,3 | 2,3 | 0,7 | 1,1 | 1,4 | 1,5 |
| Tigd4 | 1,0 | 1,0 | 1,0 | 1,0 | 1,0 | 1,0 | 1,0 | 1,0 | 1,0 | 1,0 | 1,0 | 1,0 | 1,8 | 1,0 | 1,6 | 0,2 | 0,7 | 0,7 | 1,0 | 1,0 | 1,0 | 1,0 | 1,1 | 1,0 |
| Timp1 | 1,2 | 1,0 | 1,7 | 1,4 | 1,0 | 1,0 | 1,1 | 0,6 | 1,0 | 1,0 | 0,6 | 0,9 | 0,3 | 1,2 | 2,1 | 3,1 | 1,0 | 1,0 | 1,2 | 1,1 | 1,0 | 1,0 | 1,0 | 1,1 |
| Tmem102 | 1,0 | 1,0 | 1,0 | 1,0 | 1,0 | 1,0 | 1,5 | 0,9 | 0,9 | 1,0 | 1,0 | 0,9 | 1,1 | 1,4 | 1,0 | 1,0 | 1,0 | 1,0 | 1,0 | 1,0 | 1,0 | 1,0 | 0,9 | 1,2 |
| Tmem139 | 1,0 | 1,0 | 1,0 | 1,0 | 1,0 | 1,0 | 1,3 | 1,1 | 1,0 | 1,3 | 1,3 | 0,9 | 0,9 | 1,1 | 1,0 | 1,0 | 1,0 | 1,0 | 1,0 | 1,0 | 1,0 | 1,0 | 1,1 | 0,9 |
| Tmem150c | 0,8 | 0,9 | 1,0 | 1,0 | 1,0 | 0,9 | 1,1 | 0,9 | 1,0 | 1,0 | 0,9 | 1,1 | 0,8 | 0,8 | 1,4 | 1,0 | 1,0 | 1,0 | 0,8 | 0,9 | 1,0 | 0,8 | 0,8 | 0,6 |
| Tmem182 | 0,6 | 0,7 | 0,9 | 0,6 | 1,0 | 1,0 | 0,6 | 1,0 | 1,0 | 1,0 | 1,0 | 1,0 | 1,7 | 0,8 | 3,3 | 0,2 | 1,0 | 1,3 | 1,0 | 1,0 | 1,0 | 1,0 | 1,2 | 6,1 |
| Tmem212 | 1,0 | 1,0 | 1,0 | 1,0 | 0,5 | 1,2 | 1,2 | 0,9 | 1,0 | 1,0 | 1,0 | 1,0 | 1,0 | 1,0 | 1,0 | 1,0 | 1,0 | 1,0 | 1,0 | 1,0 | 1,0 | 1,0 | 0,8 | 1,0 |
| Tmem233 | 1,0 | 1,0 | 1,0 | 1,0 | 1,0 | 1,0 | 1,0 | 1,0 | 1,0 | 1,0 | 1,0 | 1,0 | 1,4 | 0,9 | 2,5 | 0,2 | 0,8 | 1,2 | 1,0 | 1,0 | 1,0 | 1,0 | 0,7 | 12,5 |
| Tmem252 | 1,5 | 1,4 | 1,9 | 2,7 | 1,2 | 1,1 | 0,9 | 0,7 | 1,0 | 1,0 | 1,0 | 1,0 | 2,1 | 1,1 | 1,3 | 1,3 | 1,1 | 1,6 | 1,0 | 1,0 | 1,0 | 1,0 | 1,0 | 0,9 |
| Tmem254c | 1,0 | 5,9 | 0,5 | 0,3 | 0,7 | 1,0 | 0,8 | 1,4 | 1,2 | 1,9 | 5,1 | 3,5 | 0,6 | 1,3 | 0,5 | 0,4 | 1,0 | 1,0 | 0,3 | 0,1 | 0,4 | 0,9 | 0,8 | 0,9 |
| Tmem30b | 1,0 | 1,0 | 1,0 | 1,0 | 1,0 | 1,0 | 1,1 | 1,1 | 1,1 | 1,1 | 1,2 | 1,1 | 1,0 | 1,2 | 1,0 | 0,7 | 1,0 | 1,0 | 1,0 | 1,0 | 1,0 | 1,0 | 1,1 | 0,9 |
| Tmem37 | 1,0 | 0,7 | 0,9 | 1,2 | 1,1 | 0,9 | 1,4 | 1,4 | 2,0 | 1,3 | 1,1 | 1,7 | 1,6 | 1,5 | 0,8 | 1,2 | 1,0 | 1,1 | 1,4 | 1,4 | 0,9 | 0,8 | 1,4 | 1,9 |
| Tmem38a | 1,1 | 1,1 | 1,0 | 0,9 | 1,0 | 1,1 | 1,1 | 0,8 | 1,5 | 1,6 | 0,9 | 0,9 | 2,3 | 1,0 | 4,1 | 0,4 | 1,2 | 1,1 | 1,3 | 1,1 | 0,8 | 1,1 | 1,3 | 6,3 |
| Tmem45b | 1,1 | 1,0 | 0,9 | 1,0 | 1,0 | 1,0 | 0,8 | 0,2 | 1,0 | 1,0 | 0,9 | 0,9 | 0,6 | 1,5 | 1,1 | 1,0 | 0,4 | 0,3 | 1,0 | 1,0 | 0,9 | 1,0 | 1,3 | 1,1 |
| Tmem51 | 0,9 | 1,1 | 1,0 | 0,7 | 1,1 | 1,0 | 1,1 | 1,0 | 0,8 | 1,1 | 1,2 | 0,9 | 0,8 | 1,0 | 0,9 | 1,2 | 1,2 | 0,7 | 0,9 | 1,1 | 0,9 | 1,0 | 0,9 | 1,0 |
| Tmem52 | 0,5 | 0,6 | 1,0 | 1,0 | 1,1 | 2,3 | 1,6 | 1,0 | 1,0 | 1,2 | 1,2 | 1,3 | 2,7 | 1,4 | 5,0 | 0,4 | 1,5 | 1,9 | 1,0 | 1,0 | 1,0 | 1,1 | 1,4 | 2,8 |
| Tmie | 0,6 | 0,3 | 1,6 | 0,5 | 1,0 | 1,1 | 1,2 | 0,9 | 1,0 | 1,0 | 0,8 | 1,0 | 1,0 | 1,6 | 1,3 | 1,7 | 1,0 | 1,0 | 1,6 | 1,3 | 0,9 | 1,1 | 1,0 | 0,8 |
| Tmod1 | 1,3 | 1,4 | 1,0 | 1,1 | 1,1 | 1,0 | 1,2 | 1,1 | 1,0 | 1,0 | 1,2 | 1,1 | 1,9 | 1,0 | 2,1 | 1,0 | 1,0 | 1,0 | 5,6 | 0,4 | 1,3 | 1,1 | 1,1 | 2,7 |
| Tmod4 | 1,0 | 1,0 | 1,0 | 1,0 | 1,0 | 1,0 | 1,0 | 1,0 | 1,0 | 1,0 | 1,0 | 1,0 | 2,1 | 1,2 | 2,9 | 0,3 | 1,0 | 1,1 | 1,0 | 1,0 | 1,6 | 1,0 | 1,0 | 9,4 |
| Tmprss15 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Tnfrsf13c | 1,0 | 1,0 | 1,0 | 1,0 | 1,0 | 1,0 | 0,5 | 0,6 | 1,0 | 1,0 | 1,0 | 1,0 | 1,0 | 0,6 | 0,9 | 1,3 | 1,0 | 1,0 | 1,0 | 0,9 | 1,1 | 0,8 | 1,0 | 1,0 |
| Tnmd | 1,0 | 1,0 | 1,0 | 1,0 | 1,0 | 1,0 | 1,0 | 1,0 | 1,0 | 1,0 | 1,0 | 1,0 | 0,3 | 0,6 | 0,6 | 0,7 | 0,3 | 0,4 | 1,0 | 1,0 | 1,0 | 1,0 | 4,0 | 2,7 |
| Tnnc2 | 3,5 | 1,0 | 0,9 | 0,3 | 1,0 | 1,0 | 1,0 | 1,0 | 1,0 | 1,0 | 1,0 | 1,0 | 2,1 | 0,8 | 3,1 | 0,4 | 1,0 | 0,9 | 1,0 | 1,0 | 1,2 | 1,6 | 1,0 | 15,9 |
| Tnni1 | 1,0 | 1,0 | 1,0 | 1,0 | 1,2 | 1,0 | 1,0 | 1,0 | 1,0 | 1,0 | 1,0 | 1,0 | 0,2 | 0,9 | 5,0 | 1,0 | 1,3 | 2,6 | 1,0 | 1,0 | 1,0 | 1,0 | 1,0 | 1,0 |
| Tnni2 | 2,5 | 1,0 | 0,8 | 0,3 | 1,0 | 1,0 | 0,8 | 0,9 | 1,0 | 1,0 | 1,0 | 1,0 | 2,7 | 0,9 | 4,1 | 0,4 | 1,1 | 1,1 | 1,0 | 0,9 | 1,0 | 0,9 | 1,2 | 17,1 |
| Tnnt3 | 2,5 | 1,0 | 0,9 | 0,3 | 1,0 | 1,0 | 1,0 | 1,9 | 1,0 | 1,0 | 1,0 | 1,0 | 2,5 | 0,9 | 3,6 | 0,4 | 1,1 | 1,1 | 0,9 | 1,0 | 1,0 | 1,0 | 1,1 | 17,2 |
| Tnp1 | 1,0 | 1,0 | 0,6 | 1,0 | 1,0 | 1,0 | 1,0 | 1,0 | 1,0 | 1,0 | 1,0 | 1,0 | 1,0 | 0,4 | 1,0 | 1,0 | 0,7 | 1,3 | 1,0 | 1,0 | 1,1 | 1,0 | 1,0 | 1,0 |
| Tnp2 | 1,0 | 1,0 | 0,4 | 1,0 | 1,0 | 1,0 | 1,0 | 1,0 | 1,0 | 1,0 | 1,0 | 1,0 | 1,2 | 0,5 | 1,0 | 1,0 | 0,5 | 2,5 | 1,0 | 1,0 | 1,2 | 0,9 | 1,0 | 1,0 |
| Tox3 | 0,9 | 0,5 | 1,0 | 1,0 | 1,0 | 1,0 | 1,0 | 1,0 | 1,0 | 1,0 | 1,1 | 1,0 | 1,2 | 1,0 | 1,0 | 1,0 | 1,0 | 1,0 | 1,0 | 1,0 | 1,0 | 1,1 | 0,9 | 0,7 |
| Tpd52l1 | 1,3 | 0,9 | 1,5 | 1,3 | 1,1 | 1,0 | 1,0 | 1,4 | 0,6 | 1,3 | 1,1 | 1,1 | 1,5 | 0,7 | 3,0 | 0,6 | 1,3 | 1,1 | 1,0 | 1,0 | 1,1 | 0,9 | 1,0 | 1,5 |
| Tpm2 | 0,7 | 1,0 | 1,3 | 1,1 | 1,1 | 1,0 | 1,0 | 1,0 | 1,4 | 1,0 | 0,8 | 0,9 | 1,9 | 0,8 | 4,0 | 0,4 | 0,9 | 0,9 | 1,0 | 1,2 | 1,1 | 0,9 | 1,2 | 12,2 |
| Tppp2 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,2 | 1,0 | 1,0 | 1,0 | 1,1 | 1,1 | 1,0 | 1,0 |
| Tpx2 | 1,0 | 1,2 | 1,0 | 1,0 | 1,0 | 1,0 | 0,9 | 1,0 | 1,0 | 1,0 | 1,0 | 1,0 | 0,3 | 0,6 | 1,0 | 1,2 | 1,0 | 0,8 | 1,4 | 0,4 | 1,1 | 1,1 | 1,0 | 1,0 |
| Trank1 | 0,4 | 0,4 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,8 | 0,7 |
| Trdn | 1,0 | 1,0 | 1,0 | 1,0 | 1,0 | 1,0 | 1,0 | 1,0 | 1,0 | 1,0 | 1,0 | 1,0 | 2,3 | 1,0 | 3,2 | 0,2 | 1,2 | 1,2 | 1,0 | 1,0 | 1,0 | 1,0 | 1,1 | 13,1 |
| Trem2 | 0,9 | 0,7 | 1,3 | 1,6 | 1,0 | 1,0 | 0,9 | 1,5 | 1,0 | 1,0 | 0,7 | 0,8 | 0,9 | 1,6 | 0,9 | 1,4 | 1,0 | 1,0 | 1,0 | 0,9 | 1,0 | 0,7 | 0,9 | 1,7 |
| Trim54 | 1,0 | 1,0 | 1,0 | 1,1 | 1,0 | 1,0 | 0,6 | 1,1 | 1,0 | 1,0 | 1,0 | 1,0 | 2,4 | 0,8 | 4,2 | 0,3 | 1,2 | 1,0 | 1,0 | 1,0 | 0,8 | 0,9 | 1,6 | 12,4 |
| Trim56 | 0,3 | 0,5 | 1,6 | 0,7 | 0,8 | 1,0 | 0,9 | 1,5 | 1,0 | 1,0 | 1,1 | 1,3 | 0,9 | 1,2 | 1,1 | 0,8 | 0,9 | 1,1 | 1,0 | 1,0 | 1,0 | 1,0 | 1,0 | 0,6 |
| Trim63 | 1,0 | 1,0 | 1,0 | 1,8 | 1,0 | 1,0 | 0,9 | 0,6 | 1,0 | 1,0 | 1,0 | 1,0 | 4,0 | 0,9 | 3,2 | 0,3 | 2,4 | 1,1 | 1,0 | 1,0 | 1,0 | 1,0 | 2,0 | 11,1 |
| Trim72 | 1,0 | 1,0 | 0,9 | 0,6 | 1,0 | 1,2 | 0,6 | 0,6 | 1,0 | 1,0 | 1,0 | 1,0 | 1,6 | 0,9 | 3,2 | 0,3 | 0,9 | 1,0 | 1,0 | 1,0 | 1,0 | 1,0 | 1,3 | 8,5 |
| Trnp1 | 1,0 | 0,9 | 1,3 | 1,3 | 1,0 | 1,0 | 1,4 | 1,4 | 1,0 | 1,0 | 1,2 | 1,1 | 1,5 | 1,1 | 1,3 | 1,0 | 0,9 | 1,0 | 4,4 | 5,9 | 1,0 | 0,7 | 1,1 | 1,4 |
| Trp53inp2 | 1,1 | 1,1 | 0,7 | 0,6 | 0,9 | 0,9 | 1,0 | 1,1 | 0,9 | 1,0 | 0,9 | 0,9 | 1,1 | 0,9 | 1,3 | 0,7 | 0,6 | 0,6 | 0,9 | 0,9 | 0,9 | 0,9 | 0,9 | 0,9 |
| Trpv6 | 1,0 | 1,0 | 1,0 | 1,0 | 0,9 | 0,9 | 1,5 | 2,1 | 4,5 | 2,2 | 1,0 | 1,0 | 1,3 | 0,8 | 1,0 | 1,0 | 1,0 | 1,0 | 1,0 | 1,0 | 1,0 | 1,0 | 0,9 | 1,6 |
| Try4 | 0,2 | 0,6 | 1,0 | 1,0 | 1,0 | 1,1 | 1,5 | 1,0 | 2,2 | 2,0 | 0,9 | 0,7 | 3,0 | 1,0 | 4,0 | 1,0 | 1,0 | 1,0 | 1,0 | 1,0 | 1,1 | 1,0 | 1,6 | 1,0 |
| Try5 | 0,3 | 0,8 | 1,0 | 1,0 | 0,5 | 0,8 | 0,7 | 1,0 | 2,5 | 2,1 | 0,8 | 1,0 | 1,0 | 1,0 | 3,3 | 0,9 | 1,0 | 1,0 | 1,0 | 0,8 | 1,7 | 1,1 | 1,1 | 1,0 |
| Tsc22d1 | 0,9 | 1,0 | 0,8 | 0,7 | 1,1 | 1,1 | 1,1 | 1,0 | 1,0 | 1,0 | 1,0 | 1,0 | 1,0 | 1,0 | 0,8 | 1,3 | 1,0 | 1,0 | 1,1 | 1,2 | 0,9 | 0,9 | 1,0 | 0,7 |
| Tspan1 | 1,0 | 1,0 | 1,0 | 1,0 | 1,0 | 1,0 | 1,2 | 0,9 | 2,1 | 2,0 | 0,9 | 1,2 | 1,0 | 1,0 | 1,0 | 1,0 | 1,0 | 1,0 | 1,0 | 1,0 | 1,0 | 1,0 | 0,6 | 0,7 |
| Tssk6 | 0,7 | 1,2 | 1,4 | 1,0 | 0,9 | 1,0 | 1,3 | 1,3 | 1,0 | 1,0 | 1,0 | 1,2 | 0,8 | 1,0 | 1,0 | 1,3 | 1,0 | 1,0 | 0,8 | 1,0 | 1,1 | 0,7 | 0,9 | 1,0 |
| Ttc36 | 1,7 | 1,6 | 3,5 | 1,4 | 1,0 | 1,0 | 1,5 | 1,0 | 1,0 | 1,0 | 1,0 | 0,6 | 1,0 | 1,0 | 1,0 | 1,0 | 1,0 | 1,0 | 0,7 | 1,0 | 1,3 | 0,5 | 0,9 | 1,0 |
| Ttc39c | 1,5 | 1,6 | 1,4 | 1,0 | 1,1 | 1,1 | 1,2 | 1,2 | 1,0 | 1,0 | 0,8 | 0,9 | 1,1 | 0,9 | 0,9 | 1,0 | 1,0 | 1,0 | 1,2 | 1,1 | 1,1 | 0,9 | 0,6 | 0,6 |
| Ttn | 1,0 | 1,0 | 1,0 | 0,8 | 1,0 | 1,0 | 0,9 | 1,0 | 1,0 | 1,0 | 1,0 | 1,0 | 2,0 | 1,1 | 4,1 | 0,2 | 1,2 | 1,4 | 1,0 | 1,0 | 1,0 | 1,0 | 1,1 | 11,5 |
| Ttr | 1,8 | 0,8 | 1,0 | 0,1 | 1,2 | 0,8 | 1,0 | 1,0 | 2,9 | 2,6 | 0,9 | 0,8 | 2,0 | 1,0 | 2,7 | 1,3 | 1,0 | 1,0 | 1,0 | 1,3 | 1,0 | 1,0 | 1,1 | 1,0 |
| Tuba1a | 1,2 | 1,3 | 1,0 | 0,9 | 1,0 | 1,1 | 0,9 | 0,9 | 0,8 | 0,9 | 1,0 | 0,9 | 0,9 | 0,7 | 0,9 | 1,1 | 0,7 | 0,6 | 1,0 | 0,8 | 1,0 | 1,1 | 0,8 | 0,8 |
| Tuba8 | 0,9 | 1,0 | 0,9 | 1,1 | 1,1 | 1,0 | 0,8 | 1,1 | 1,0 | 1,0 | 0,8 | 0,8 | 1,6 | 0,9 | 1,2 | 0,7 | 1,0 | 1,4 | 0,5 | 0,7 | 1,1 | 1,0 | 1,5 | 2,4 |
| Tubb2a-ps2 | 1,0 | 5,0 | 1,1 | 0,8 | 0,9 | 1,0 | 0,8 | 1,0 | 1,0 | 1,0 | 1,2 | 1,1 | 1,0 | 1,1 | 0,5 | 1,0 | 0,5 | 0,9 | 0,8 | 0,8 | 1,0 | 1,0 | 0,3 | 1,4 |
| Txlnb | 1,0 | 1,0 | 1,0 | 0,9 | 1,0 | 1,0 | 0,8 | 0,7 | 1,0 | 1,0 | 1,0 | 1,0 | 2,4 | 1,2 | 4,3 | 0,2 | 1,1 | 1,3 | 1,0 | 1,0 | 1,0 | 1,0 | 1,4 | 7,3 |
| Tyr | 1,0 | 1,0 | 1,0 | 1,0 | 1,0 | 1,0 | 1,0 | 1,0 | 1,0 | 1,0 | 1,0 | 1,0 | 0,3 | 0,4 | 1,0 | 1,0 | 1,0 | 1,0 | 1,0 | 1,0 | 1,0 | 1,0 | 1,0 | 1,0 |
| Tyrp1 | 1,0 | 1,0 | 1,0 | 1,0 | 1,0 | 1,0 | 1,0 | 1,0 | 1,0 | 1,0 | 1,0 | 1,0 | 0,3 | 0,4 | 1,0 | 1,0 | 1,0 | 1,0 | 1,0 | 1,0 | 1,0 | 1,0 | 1,0 | 1,0 |
| Ucp1 | 0,8 | 0,9 | 0,5 | 0,9 | 1,0 | 1,0 | 1,0 | 1,0 | 1,0 | 1,0 | 1,0 | 1,0 | 1,0 | 1,0 | 1,0 | 1,0 | 0,4 | 0,1 | 1,0 | 1,0 | 1,0 | 1,0 | 1,6 | 1,4 |
| Ugt1a1 | 1,0 | 1,0 | 1,0 | 1,0 | 1,0 | 1,0 | 1,0 | 1,0 | 1,0 | 1,0 | 1,0 | 1,0 | 1,0 | 2,0 | 1,0 | 1,0 | 1,0 | 1,0 | 1,0 | 1,0 | 1,0 | 1,0 | 1,0 | 1,0 |
| Ugt1a10 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Ugt1a2 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Uox | 1,0 | 0,7 | 1,0 | 0,4 | 1,0 | 1,0 | 1,0 | 1,0 | 1,0 | 1,0 | 1,0 | 1,0 | 1,0 | 0,6 | 1,0 | 1,0 | 1,0 | 1,0 | 1,0 | 1,0 | 1,0 | 1,0 | 1,0 | 1,0 |
| Upp2 | 1,4 | 0,6 | 1,0 | 1,0 | 1,0 | 1,0 | 1,0 | 1,0 | 0,3 | 0,3 | 0,9 | 1,4 | 1,0 | 1,0 | 1,0 | 1,0 | 1,0 | 1,0 | 1,0 | 1,0 | 0,9 | 1,5 | 1,0 | 1,0 |
| Usp13 | 1,0 | 1,0 | 0,9 | 0,9 | 0,8 | 0,9 | 1,0 | 1,0 | 1,0 | 1,0 | 1,1 | 0,9 | 1,7 | 1,0 | 2,4 | 0,3 | 0,9 | 1,1 | 1,0 | 1,0 | 1,0 | 1,0 | 1,1 | 7,2 |
| Vash2 | 1,0 | 1,0 | 1,0 | 1,0 | 0,9 | 1,0 | 1,0 | 1,0 | 1,0 | 1,0 | 1,0 | 1,0 | 0,8 | 1,2 | 1,2 | 1,0 | 1,0 | 1,0 | 1,0 | 1,0 | 1,0 | 0,9 | 1,0 | 0,9 |
| Vcan | 1,0 | 1,0 | 1,2 | 1,3 | 1,0 | 1,0 | 0,8 | 0,9 | 1,0 | 1,0 | 1,0 | 1,7 | 0,3 | 0,9 | 1,1 | 0,8 | 0,6 | 0,6 | 1,0 | 1,0 | 1,0 | 1,0 | 1,4 | 1,3 |
| Vgll2 | 1,0 | 1,0 | 1,0 | 1,0 | 1,0 | 1,0 | 1,0 | 1,0 | 1,0 | 1,0 | 1,0 | 1,0 | 2,8 | 1,0 | 5,0 | 0,4 | 0,8 | 1,5 | 1,0 | 1,0 | 1,0 | 1,0 | 1,7 | 6,6 |
| Vnn1 | 0,8 | 1,2 | 1,3 | 1,7 | 1,0 | 1,0 | 1,0 | 0,9 | 1,0 | 1,0 | 1,0 | 1,0 | 1,2 | 1,0 | 1,2 | 1,0 | 1,0 | 1,0 | 1,0 | 1,0 | 1,0 | 1,0 | 0,9 | 1,0 |
| Vpreb3 | 0,5 | 1,0 | 1,0 | 1,4 | 1,0 | 1,0 | 1,1 | 1,1 | 0,8 | 0,9 | 1,1 | 2,3 | 1,0 | 1,0 | 0,5 | 0,7 | 1,0 | 1,0 | 1,0 | 0,9 | 1,0 | 1,0 | 1,7 | 0,9 |
| Wbscr25 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,7 | 1,0 | 1,0 |
| Wfdc10 | 1,0 | 1,0 | 1,0 | 1,0 | 1,0 | 1,0 | 1,0 | 1,6 | 1,0 | 1,0 | 1,0 | 1,0 | 1,0 | 1,0 | 1,0 | 1,0 | 1,0 | 1,0 | 1,0 | 1,0 | 1,2 | 1,2 | 1,0 | 1,0 |
| Wfdc11 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Wfdc13 | 1,0 | 1,0 | 1,0 | 1,0 | 1,0 | 1,0 | 0,9 | 1,0 | 1,0 | 1,0 | 1,0 | 1,0 | 1,0 | 1,0 | 1,0 | 1,0 | 1,0 | 1,0 | 1,0 | 1,0 | 1,3 | 8,2 | 1,0 | 1,0 |
| Wfdc15b | 1,0 | 1,0 | 1,0 | 1,0 | 1,0 | 1,0 | 1,0 | 1,0 | 1,0 | 0,7 | 1,0 | 1,0 | 1,0 | 1,0 | 1,0 | 1,0 | 1,0 | 1,0 | 1,0 | 1,0 | 1,3 | 5,0 | 1,0 | 1,0 |
| Wfdc18 | 1,0 | 1,0 | 1,0 | 1,0 | 1,0 | 1,0 | 1,0 | 1,0 | 1,0 | 1,0 | 1,0 | 1,0 | 0,9 | 0,7 | 1,0 | 1,0 | 1,0 | 1,0 | 1,0 | 1,0 | 1,0 | 1,0 | 0,3 | 0,1 |
| Wfdc6a | 0,6 | 0,6 | 1,0 | 1,0 | 1,0 | 1,0 | 0,7 | 2,1 | 1,0 | 1,0 | 1,0 | 1,0 | 1,0 | 1,0 | 1,0 | 1,0 | 1,0 | 1,0 | 1,0 | 1,0 | 0,8 | 1,1 | 1,0 | 1,0 |
| Wfdc6b | 1,0 | 1,0 | 1,0 | 1,0 | 1,0 | 1,0 | 1,5 | 1,2 | 1,0 | 1,0 | 1,0 | 1,0 | 1,0 | 1,0 | 1,0 | 1,0 | 1,0 | 1,0 | 1,0 | 1,0 | 1,1 | 1,5 | 1,0 | 1,0 |
| Wfdc8 | 1,0 | 1,0 | 1,0 | 1,0 | 1,0 | 1,0 | 1,5 | 2,7 | 1,0 | 1,0 | 1,0 | 1,0 | 1,0 | 1,0 | 1,0 | 1,0 | 1,0 | 1,0 | 1,0 | 1,0 | 1,3 | 1,3 | 1,0 | 1,0 |
| Wfdc9 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Xirp1 | 1,0 | 1,0 | 1,7 | 1,0 | 1,0 | 1,0 | 0,7 | 1,0 | 1,0 | 1,0 | 1,0 | 1,0 | 2,5 | 0,9 | 3,5 | 0,3 | 0,6 | 1,6 | 1,0 | 1,0 | 1,1 | 0,9 | 1,5 | 8,4 |
| Xirp2 | 1,0 | 1,0 | 1,0 | 0,7 | 1,0 | 1,0 | 1,0 | 1,0 | 1,0 | 1,0 | 1,0 | 1,0 | 1,7 | 0,7 | 3,0 | 0,3 | 1,0 | 1,2 | 1,0 | 1,0 | 1,0 | 1,0 | 1,0 | 10,9 |
| Xkrx | 1,3 | 1,1 | 0,6 | 1,0 | 1,4 | 1,0 | 0,5 | 0,7 | 1,0 | 1,0 | 1,5 | 1,0 | 1,4 | 1,0 | 1,2 | 1,4 | 1,0 | 1,0 | 0,8 | 0,7 | 1,0 | 1,0 | 0,7 | 1,1 |

[Fig. 23-15]

284/310

| | | | | | | | | | | | | | | | | | | | | | | | | |
|---|---|---|---|---|---|---|---|---|---|---|---|---|---|---|---|---|---|---|---|---|---|---|---|---|
| Yipf7 | 1,0 | 1,0 | 1,0 | 1,0 | 1,0 | 1,0 | 1,0 | 1,0 | 1,0 | 1,0 | 1,0 | 1,0 | 1,7 | 1,2 | 2,8 | 0,4 | 0,7 | 0,7 | 1,0 | 1,0 | 0,7 | 0,8 | 0,9 | 5,4 |
| Ypel4 | 0,6 | 0,9 | 0,9 | 0,9 | 1,0 | 0,8 | 1,0 | 1,0 | 1,0 | 1,0 | 1,1 | 0,9 | 1,6 | 1,5 | 1,2 | 1,2 | 1,0 | 1,0 | 5,5 | 0,4 | 1,6 | 0,7 | 1,0 | 0,7 |
| Zbed6 | 1,0 | 0,7 | 1,3 | 1,0 | 0,8 | 0,6 | 0,7 | 2,7 | 1,0 | 1,0 | 1,1 | 1,1 | 0,5 | 1,1 | 1,1 | 0,6 | 1,1 | 1,5 | 1,0 | 0,8 | 0,8 | 1,0 | 1,4 | 0,7 |
| Zfp369 | 1,0 | 1,0 | 1,0 | 1,0 | 0,9 | 0,7 | 0,6 | 1,0 | 1,0 | 1,0 | 1,0 | 1,0 | 0,7 | 1,0 | 1,7 | 0,6 | 1,7 | 1,0 | 1,1 | 0,9 | 1,0 | 0,9 | 1,0 | 1,0 |
| Zfp648 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Zfp750 | 1,0 | 1,0 | 1,0 | 1,0 | 1,0 | 1,0 | 0,9 | 1,3 | 0,6 | 1,0 | 1,2 | 1,1 | 1,0 | 1,0 | 1,0 | 1,0 | 1,0 | 1,0 | 1,0 | 1,0 | 1,0 | 1,0 | 0,7 | 0,8 |
| Zg16 | 0,9 | 0,8 | 1,0 | 1,0 | 2,5 | 0,9 | 0,9 | 1,0 | 1,0 | 1,2 | 0,6 | 0,9 | 1,0 | 1,7 | 1,5 | 0,9 | 1,7 | 2,5 | 1,1 | 0,6 | 1,0 | 2,6 | 1,5 | 1,0 |

[Fig. 23-16]

| | Kidney | | Colon | | Eyeball | | Heart | | Liver | | SG | | Thymus | | Adipose | | Stomach | | Jejunum | | Ileum | |
|---|---|---|---|---|---|---|---|---|---|---|---|---|---|---|---|---|---|---|---|---|---|---|
| | E | M | E | M | E | M | E | M | E | M | E | M | E | M | E | M | E | M | E | M | E | M |
| Abcb1b | 7,8 | 5,3 | 1,2 | 2,4 | 1,0 | 1,0 | 1,2 | 1,4 | 1,0 | 1,0 | 1,0 | 1,0 | 1,2 | 1,3 | 0,7 | 1,0 | 1,0 | 1,0 | 1,0 | 1,2 | 1,2 | 1,9 |
| Abcg4 | 1,0 | 1,0 | 1,0 | 1,0 | 1,0 | 1,0 | 1,0 | 1,0 | 1,0 | 1,0 | 1,0 | 1,0 | 1,0 | 1,0 | 0,6 | 0,7 | 1,0 | 1,0 | 1,0 | 1,0 | 1,0 | 1,0 |
| Abra | 1,0 | 1,0 | 1,0 | 1,0 | 0,6 | 0,5 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Acbd7 | 1,0 | 1,0 | 1,0 | 1,0 | 1,0 | 2,7 | 1,0 | 1,6 | 1,0 | 1,0 | 1,0 | 1,0 | 1,0 | 1,0 | 5,6 | 1,1 | 1,0 | 1,0 | 1,0 | 1,0 | 1,0 | 1,0 |
| Acnat2 | 1,7 | 1,5 | 1,0 | 1,0 | 1,0 | 1,0 | 1,0 | 1,0 | 14,8 | 4,1 | 1,0 | 1,0 | 1,0 | 1,0 | 1,0 | 1,0 | 1,3 | 1,0 | 1,0 | 0,6 | 0,8 | 1,0 |
| Acot3 | 2,8 | 3,3 | 1,0 | 1,0 | 1,0 | 1,0 | 1,1 | 1,2 | 2,6 | 19,7 | 1,0 | 1,0 | 1,0 | 1,0 | 4,8 | 3,3 | 1,0 | 1,0 | 1,0 | 1,0 | 1,0 | 1,0 |
| Acsm3 | 0,4 | 0,2 | 1,1 | 0,9 | 1,0 | 1,0 | 1,0 | 1,0 | 0,8 | 0,7 | 1,1 | 1,5 | 1,3 | 1,2 | 2,9 | 6,4 | 1,5 | 1,1 | 0,9 | 0,8 | 1,1 | 1,5 |
| *Acta1* | *1,0* | *1,0* | *0,8* | *0,8* | *0,6* | *0,5* | *0,9* | *0,7* | *1,0* | *1,0* | 2,2 | *1,8* | *0,4* | *0,8* | *0,7* | 1,4 | *0,5* | 1,2 | 2,1 | *1,3* | 2,6 | *0,9* |
| Actc1 | 1,0 | 1,0 | 0,8 | 0,6 | 0,4 | 0,5 | 0,9 | 0,8 | 1,0 | 1,0 | 1,0 | 1,0 | 1,0 | 1,4 | 1,0 | 1,4 | 1,1 | 1,1 | 1,0 | 0,6 | 0,7 | 1,7 |
| Actg2 | 1,0 | 1,0 | 1,2 | 0,9 | 1,1 | 1,0 | 1,2 | 0,9 | 1,0 | 1,0 | 0,6 | 0,4 | 1,0 | 1,0 | 28,6 | 4,4 | 2,5 | 1,5 | 1,4 | 1,3 | 0,8 | 0,9 |
| Actn2 | 1,0 | 1,0 | 1,0 | 1,0 | 0,6 | 0,7 | 1,0 | 1,1 | 1,0 | 1,0 | 1,0 | 1,0 | 1,0 | 1,0 | 1,0 | 1,0 | 1,0 | 0,9 | 1,0 | 1,0 | 1,0 | 1,0 |
| Actn3 | 1,0 | 1,0 | 1,0 | 1,0 | 0,8 | 0,4 | 1,0 | 1,0 | 1,0 | 1,0 | 1,0 | 1,2 | 1,4 | 1,4 | 1,0 | 0,7 | 1,0 | 1,0 | 1,0 | 1,0 | 1,0 | 1,0 |
| Adam28 | 1,0 | 1,0 | 1,0 | 1,0 | 1,2 | 1,0 | 1,0 | 1,0 | 1,0 | 1,0 | 1,0 | 1,0 | 2,3 | 2,8 | 67,3 | 1,0 | 1,0 | 0,9 | 1,0 | 1,0 | 1,0 | 1,0 |
| Adam7 | 1,0 | 1,0 | 1,0 | 1,0 | 1,0 | 1,0 | 1,0 | 1,0 | 1,0 | 1,0 | 1,0 | 1,0 | 1,0 | 1,0 | 37,0 | 1,0 | 1,0 | 1,0 | 1,0 | 1,0 | 1,0 | 1,0 |
| Add2 | 1,0 | 1,0 | 1,0 | 1,0 | 1,1 | 0,9 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Adh1 | 0,9 | 0,7 | 1,5 | 1,4 | 0,9 | 1,1 | 0,9 | 1,1 | 1,3 | 1,3 | 2,1 | 1,5 | 4,0 | 3,6 | 0,8 | 1,6 | 1,4 | 1,1 | 1,1 | 1,1 | 1,2 | 1,0 |
| Adh6-ps1 | 1,0 | 1,0 | 1,0 | 1,0 | 1,0 | 1,0 | 1,0 | 1,0 | 0,0 | 0,1 | 1,0 | 1,0 | 1,0 | 1,0 | 1,0 | 1,0 | 1,0 | 1,0 | 1,0 | 1,0 | 1,0 | 1,0 |
| Adh7 | 1,0 | 1,0 | 1,0 | 1,0 | 1,0 | 1,1 | 1,0 | 1,0 | 1,3 | 0,9 | 1,0 | 1,0 | 1,0 | 1,0 | 1,0 | 1,0 | 0,9 | 0,9 | 1,0 | 0,8 | 1,0 | 1,0 |
| Adig | 1,0 | 1,0 | 0,7 | 1,7 | 0,7 | 0,4 | 3,8 | 0,9 | 1,0 | 1,0 | 1,0 | 1,0 | 1,6 | 8,4 | 1,1 | 1,9 | 1,0 | 1,0 | 0,9 | 1,0 | 1,2 | 2,0 |
| Adipoq | 1,0 | 1,0 | 0,6 | 1,5 | 0,3 | 0,8 | 3,8 | 1,0 | 1,0 | 1,0 | 0,7 | 4,2 | 1,6 | 5,3 | 0,7 | 1,3 | 0,5 | 1,2 | 0,9 | 0,8 | 0,5 | 1,4 |
| Agps | 0,3 | 0,3 | 1,0 | 1,1 | 1,0 | 1,2 | 0,9 | 1,0 | 1,3 | 1,2 | 1,0 | 1,1 | 0,7 | 0,9 | 0,9 | 0,9 | 1,0 | 1,0 | 1,2 | 1,1 | 1,1 | 1,2 |
| Agr2 | 1,0 | 1,0 | 0,9 | 0,8 | 1,0 | 1,0 | 1,0 | 1,0 | 1,0 | 1,0 | 1,8 | 1,4 | 1,0 | 1,0 | 8,7 | 1,0 | 1,5 | 1,1 | 1,2 | 1,4 | 1,0 | 0,9 |
| Ahsg | 0,5 | 1,0 | 0,1 | 1,0 | 1,0 | 1,0 | 1,7 | 1,7 | 0,9 | 0,9 | 1,4 | 1,0 | 1,0 | 0,9 | 0,2 | 0,5 | 15,5 | 0,2 | 1,0 | 1,0 | 1,0 | 1,0 |
| Aicda | 1,0 | 1,0 | 0,7 | 0,5 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,9 | 1,0 |
| Airn | 0,3 | 0,4 | 0,6 | 0,9 | 1,6 | 1,8 | 1,2 | 0,6 | 1,5 | 1,3 | 1,0 | 1,4 | 0,6 | 0,8 | 0,9 | 1,1 | 0,9 | 0,7 | 0,6 | 0,9 | 0,7 | 0,9 |
| Akp3 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 11,9 | 1,8 | 1,0 | 1,0 |
| Akr1b3 | 1,6 | 6,1 | 1,3 | 2,2 | 0,9 | 3,2 | 1,0 | 3,3 | 1,0 | 0,7 | 2,5 | 1,2 | 0,3 | 1,0 | 1,0 | 0,8 | 0,5 | 1,8 | 2,6 | 1,2 | 1,4 | 1,0 |
| Akr1b7 | 1,3 | 1,6 | 1,2 | 1,3 | 0,8 | 1,0 | 1,0 | 1,0 | 2,0 | 5,1 | 1,0 | 1,0 | 1,0 | 1,0 | 1,1 | 157,1 | 1,0 | 1,0 | 2,4 | 3,6 | 1,0 | 0,6 |
| Akr1b8 | 2,7 | 2,9 | 1,6 | 1,3 | 1,2 | 3,2 | 1,5 | 1,1 | 1,0 | 1,6 | 0,8 | 1,1 | 1,0 | 1,0 | 0,7 | 0,8 | 0,9 | 0,7 | 2,2 | 6,0 | 2,2 | 1,9 |
| Akr1c14 | 0,3 | 0,2 | 1,2 | 1,1 | 0,8 | 1,4 | 0,6 | 0,6 | 0,8 | 1,1 | 1,0 | 1,0 | 1,0 | 1,0 | 0,3 | 0,6 | 1,2 | 1,1 | 0,5 | 0,6 | 1,2 | 1,1 |
| Akr1c18 | 0,2 | 0,2 | 1,4 | 1,6 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Alas2 | 1,8 | 1,2 | 1,0 | 1,0 | 4,1 | 0,7 | 0,9 | 2,3 | 0,0 | 0,1 | 1,7 | 0,9 | 2,9 | 1,7 | 1,4 | 1,9 | 1,5 | 1,2 | 1,0 | 1,4 | 1,0 | 1,0 |
| Alb | 1,3 | 1,3 | 0,2 | 1,0 | 1,0 | 0,8 | 1,0 | 1,0 | 1,3 | 1,4 | 0,7 | 1,0 | 1,0 | 0,2 | 0,2 | 1,2 | 146,2 | 0,0 | 1,0 | 0,8 | 1,0 | 1,0 |
| Aldh1a1 | 6,3 | 12,8 | 1,5 | 1,7 | 1,0 | 1,2 | 0,9 | 1,1 | 0,8 | 1,0 | 0,9 | 0,8 | 2,0 | 2,0 | 1,7 | 1,2 | 1,2 | 0,9 | 1,1 | 1,4 | 1,5 | 1,0 |
| *Aldh1a3* | *1,6* | *1,3* | *1,4* | *0,9* | *1,1* | *1,3* | *1,0* | *1,0* | *1,0* | *1,0* | *1,2* | *1,1* | *1,7* | *2,5* | *0,7* | *0,7* | *1,0* | *1,4* | *1,1* | *1,1* | *1,0* | *1,1* |
| Aldh3a1 | 1,0 | 1,0 | 1,0 | 1,0 | 1,0 | 1,2 | 1,1 | 1,1 | 1,0 | 1,0 | 1,2 | 1,0 | 1,0 | 1,0 | 1,5 | 1,0 | 1,5 | 1,4 | 1,0 | 1,0 | 1,0 | 1,0 |
| Aldob | 1,1 | 1,2 | 1,0 | 1,1 | 1,0 | 1,0 | 1,3 | 1,6 | 1,4 | 0,9 | 1,0 | 1,0 | 1,0 | 0,4 | 0,5 | 2,9 | 7,3 | 0,2 | 1,1 | 0,9 | 1,0 | 1,0 |
| Aldoc | 1,2 | 1,4 | 0,9 | 1,0 | 1,1 | 1,1 | 1,0 | 1,0 | 1,1 | 1,1 | 1,6 | 1,2 | 1,3 | 1,9 | 6,4 | 1,4 | 1,3 | 0,9 | 2,6 | 0,9 | 1,8 | 1,2 |
| Alpk3 | 1,0 | 1,0 | 1,0 | 1,0 | 0,6 | 0,6 | 0,9 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Alpl | 1,5 | 1,4 | 1,4 | 0,8 | 1,0 | 1,0 | 1,0 | 1,0 | 1,6 | 2,3 | 0,6 | 0,6 | 1,0 | 1,0 | 5,8 | 0,8 | 1,4 | 1,3 | 1,1 | 0,8 | 1,0 | 0,8 |
| Ambp | 1,0 | 1,0 | 0,9 | 1,0 | 1,0 | 1,0 | 1,0 | 1,0 | 1,0 | 1,1 | 1,0 | 1,0 | 1,0 | 1,0 | 1,0 | 1,0 | 2,6 | 0,2 | 1,2 | 1,2 | 1,0 | 1,0 |
| Amd1 | 1,0 | 0,1 | 1,0 | 0,7 | 1,0 | 1,0 | 1,0 | 1,0 | 1,0 | 1,0 | 0,1 | 1,0 | 1,0 | 1,0 | 1,0 | 1,0 | 1,0 | 0,1 | 1,0 | 0,1 | 1,0 | 1,0 |
| Ampd1 | 1,0 | 1,0 | 1,0 | 1,0 | 0,9 | 0,4 | 1,0 | 1,0 | 1,0 | 1,0 | 1,0 | 1,0 | 1,2 | 1,2 | 1,0 | 1,0 | 1,0 | 1,0 | 1,0 | 1,0 | 1,0 | 1,0 |
| Amy1 | 1,2 | 0,9 | 1,3 | 1,5 | 1,2 | 1,1 | 2,0 | 1,2 | 0,9 | 1,2 | 3,3 | 12,9 | 1,6 | 2,9 | 0,7 | 1,4 | 1,8 | 1,0 | 0,6 | 1,0 | 0,9 | 1,1 |
| *Amy2a2* | 1,0 | 1,0 | 1,3 | 3,4 | 1,0 | 1,0 | 1,0 | 1,0 | 1,0 | 1,0 | 1,0 | 1,0 | 1,0 | 1,0 | 1,0 | 1,0 | 2,4 | 0,1 | 0,7 | 1,0 | 1,0 | 1,0 |
| Prol1 | 0,9 | 1,0 | 1,3 | 468,7 | 1,4 | 1,0 | 1,0 | 1,0 | 0,6 | 0,8 | 0,9 | 1,3 | 1,0 | 0,0 | 1,0 | 1,0 | 16,3 | 0,1 | 0,0 | 0,2 | 0,9 | 1,0 |
| Amy2b | 1,0 | 0,9 | 1,6 | 21,0 | 1,0 | 1,0 | 1,0 | 1,0 | 1,0 | 1,3 | 2,8 | 14,5 | 1,0 | 1,0 | 1,0 | 1,0 | 13,6 | 0,1 | 0,0 | 1,0 | 1,0 | 1,0 |
| Ang4 | 1,0 | 1,0 | 0,0 | 0,0 | 1,0 | 1,0 | 1,0 | 1,0 | 1,0 | 1,0 | 1,0 | 1,0 | 0,6 | 0,5 | 1,0 | 1,0 | 1,0 | 1,0 | 0,5 | 0,8 | 0,6 | 0,6 |
| Angptl7 | 0,2 | 0,1 | 2,1 | 1,9 | 1,2 | 1,2 | 1,3 | 1,5 | 1,0 | 1,0 | 1,0 | 1,0 | 1,4 | 1,6 | 1,5 | 0,9 | 2,0 | 1,8 | 1,0 | 1,0 | 1,0 | 1,0 |
| Ank1 | 1,0 | 1,0 | 1,3 | 0,9 | 1,0 | 0,8 | 1,1 | 1,1 | 1,0 | 1,0 | 1,0 | 1,0 | 1,0 | 1,0 | 1,0 | 1,3 | 0,9 | 1,0 | 1,0 | 1,0 | 1,0 | 1,0 |
| Ankrd1 | 1,1 | 2,6 | 1,0 | 1,0 | 1,0 | 1,2 | 1,0 | 1,1 | 1,0 | 1,0 | 1,0 | 1,0 | 1,0 | 1,0 | 1,0 | 1,0 | 1,0 | 1,0 | 1,0 | 1,0 | 1,0 | 1,0 |
| Ankrd2 | 1,0 | 1,0 | 1,0 | 1,0 | 1,0 | 1,1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Ankrd23 | 0,8 | 0,7 | 1,0 | 1,1 | 0,8 | 0,4 | 0,7 | 0,7 | 1,1 | 1,0 | 1,0 | 0,8 | 1,0 | 0,9 | 1,2 | 0,7 | 1,1 | 1,1 | 0,8 | 1,1 | 1,3 | 0,6 |
| Ankrd66 | 1,0 | 1,0 | 1,0 | 1,0 | 1,0 | 1,0 | 1,0 | 1,0 | 1,0 | 1,0 | 1,0 | 1,0 | 1,0 | 1,0 | 1,8 | 1,0 | 1,0 | 1,0 | 1,0 | 1,0 | 1,0 | 1,0 |
| Anpep | 0,8 | 0,7 | 1,8 | 1,7 | 0,7 | 0,8 | 1,0 | 1,0 | 1,1 | 0,9 | 1,3 | 1,1 | 2,0 | 1,8 | 0,8 | 0,6 | 1,1 | 1,2 | 1,7 | 1,7 | 1,2 | 1,6 |
| Ap1m2 | 1,3 | 1,2 | 1,0 | 0,9 | 1,1 | 1,4 | 1,0 | 1,0 | 1,0 | 1,0 | 0,9 | 0,9 | 1,2 | 1,1 | 7,5 | 1,4 | 1,1 | 1,0 | 1,2 | 1,1 | 1,0 | 0,9 |
| Ap1s2 | 0,9 | 1,7 | 0,9 | 0,7 | 1,0 | 1,1 | 0,8 | 0,8 | 1,1 | 1,6 | 0,7 | 0,7 | 0,7 | 0,9 | 15,3 | 1,0 | 1,0 | 1,1 | 1,0 | 1,3 | 0,8 | 0,9 |
| Ap1s3 | 1,1 | 1,0 | 1,0 | 1,3 | 1,2 | 1,5 | 1,0 | 1,0 | 1,0 | 1,0 | 0,9 | 1,3 | 0,9 | 1,0 | 6,2 | 1,2 | 1,1 | 1,1 | 0,8 | 1,0 | 0,9 | 1,2 |
| Apln | 0,4 | 0,6 | 0,8 | 0,7 | 0,7 | 0,9 | 0,5 | 0,5 | 1,0 | 1,0 | 1,0 | 1,0 | 1,0 | 1,6 | 0,3 | 0,5 | 1,6 | 1,4 | 0,8 | 1,4 | 1,0 | 0,7 |
| Aplnr | 0,5 | 0,7 | 0,8 | 0,7 | 1,0 | 0,6 | 0,3 | 0,3 | 1,0 | 1,0 | 1,0 | 1,0 | 1,0 | 0,8 | 0,2 | 0,4 | 0,4 | 0,7 | 0,6 | 0,5 | 0,5 | 0,6 |
| Apoa1 | 1,5 | 1,4 | 0,1 | 4,3 | 1,0 | 0,9 | 1,0 | 1,0 | 0,6 | 0,9 | 0,8 | 1,0 | 2,2 | 0,3 | 0,1 | 0,4 | 0,9 | 1,0 | 1,3 | 1,5 | 1,3 | 0,9 |
| *Apoa2* | *0,8* | *1,0* | *0,2* | *0,8* | *1,0* | *0,8* | *1,0* | *1,1* | *0,7* | *0,9* | *0,7* | *1,0* | *1,0* | *0,6* | 0,2 | 0,8 | 29,7 | *0,0* | 1,3 | *0,7* | *1,0* | *1,0* |
| Apoa4 | 0,7 | 0,5 | 0,4 | 3,3 | 1,0 | 1,0 | 1,0 | 1,0 | 0,7 | 0,7 | 1,0 | 1,0 | 2,2 | 0,5 | 1,0 | 1,0 | 0,8 | 0,8 | 0,7 | 1,0 | 2,5 | 1,8 |
| Apoa5 | 1,0 | 1,0 | 0,9 | 1,0 | 1,0 | 1,0 | 1,0 | 1,0 | 0,9 | 1,0 | 1,0 | 1,0 | 1,0 | 1,0 | 1,0 | 1,0 | 5,0 | 0,2 | 1,0 | 1,0 | 1,0 | 1,0 |
| Apobec2 | 0,8 | 0,4 | 0,5 | 0,6 | 0,6 | 0,6 | 1,0 | 1,0 | 1,0 | 1,0 | 1,4 | 1,2 | 0,6 | 1,1 | 1,0 | 1,0 | 0,5 | 0,8 | 1,5 | 0,6 | 1,3 | 1,0 |
| Apoc3 | 1,4 | 1,5 | 0,6 | 1,6 | 1,0 | 1,0 | 1,0 | 1,0 | 0,7 | 0,9 | 1,0 | 1,0 | 1,4 | 1,0 | 1,5 | 3,5 | 12,4 | 0,1 | 0,9 | 1,3 | 1,3 | 0,9 |
| Apoc4 | 1,7 | 2,0 | 1,0 | 1,0 | 1,0 | 1,0 | 1,0 | 1,0 | 0,8 | 0,8 | 1,0 | 1,0 | 1,0 | 1,0 | 1,7 | 0,8 | 5,0 | 0,2 | 1,0 | 1,0 | 1,0 | 1,0 |
| Apoe | 4,5 | 5,8 | 1,2 | 0,7 | 1,0 | 0,8 | 1,1 | 1,2 | 0,7 | 0,8 | 0,9 | 0,8 | 2,5 | 1,3 | 0,6 | 0,6 | 1,2 | 1,6 | 1,1 | 0,9 | 0,9 | 0,7 |
| Apoh | 1,1 | 0,9 | 1,0 | 1,0 | 1,0 | 1,0 | 1,0 | 1,0 | 0,9 | 1,2 | 1,0 | 1,0 | 1,0 | 1,0 | 1,0 | 1,0 | 3,9 | 0,3 | 1,0 | 1,0 | 1,0 | 1,0 |
| Apol10a | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,5 | 0,3 | 0,8 | 0,6 |
| Apol11b | 1,0 | 1,0 | 1,0 | 0,8 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,8 | 0,8 |
| Apol8 | 1,0 | 1,0 | 1,0 | 1,0 | 1,2 | 0,7 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Arg2 | 8,4 | 11,3 | 2,2 | 3,0 | 1,1 | 1,3 | 1,0 | 1,0 | 1,0 | 1,0 | 1,0 | 1,0 | 1,3 | 1,0 | 2,8 | 2,9 | 1,2 | 2,0 | 1,1 | 1,3 | 0,9 | 1,2 |
| Armcx6 | 0,9 | 1,4 | 0,8 | 1,2 | 1,1 | 1,1 | 1,0 | 1,0 | 1,0 | 1,0 | 1,0 | 0,9 | 1,0 | 1,1 | 8,5 | 0,7 | 0,8 | 0,9 | 1,0 | 1,0 | 1,0 | 1,0 |
| Arnt2 | 1,4 | 1,3 | 0,9 | 1,0 | 1,2 | 0,9 | 1,0 | 1,0 | 1,0 | 1,0 | 1,0 | 1,0 | 1,6 | 1,0 | 9,1 | 1,0 | 0,6 | 1,1 | 1,2 | 1,3 | 1,0 | 1,0 |
| Art1 | 1,0 | 1,0 | 1,0 | 1,0 | 0,7 | 0,6 | 1,0 | 1,0 | 1,0 | 1,0 | 1,0 | 1,0 | 0,9 | 0,8 | 1,0 | 1,0 | 1,0 | 1,0 | 1,0 | 1,0 | 1,0 | 1,0 |
| Asb11 | 0,8 | 0,9 | 1,2 | 0,9 | 0,6 | 0,8 | 1,0 | 1,2 | 1,0 | 1,0 | 1,0 | 1,0 | 1,0 | 1,0 | 7,7 | 1,0 | 1,5 | 1,6 | 1,0 | 1,0 | 1,0 | 1,0 |
| Asb16 | 1,0 | 1,0 | 1,0 | 1,0 | 0,5 | 0,5 | 1,0 | 1,0 | 1,0 | 1,0 | 0,8 | 1,4 | 1,0 | 1,0 | 1,0 | 1,0 | 1,0 | 1,0 | 1,0 | 1,0 | 1,0 | 1,0 |
| Asb2 | *0,8* | *1,0* | *0,8* | *0,8* | *0,8* | *0,5* | 1,0 | *0,9* | *1,0* | *1,0* | *0,9* | *0,6* | 1,7 | *1,0* | *1,8* | *1,1* | *1,0* | *1,0* | *1,2* | *0,8* | *0,9* | *0,7* |
| Asb5 | 1,0 | 1,0 | 1,0 | 1,0 | 0,5 | 0,6 | 0,8 | 1,1 | 1,0 | 1,0 | 1,0 | 1,0 | 1,0 | 1,0 | 1,0 | 1,0 | 1,0 | 1,0 | 1,0 | 1,0 | 1,0 | 1,0 |
| Ascl4 | 1,0 | 1,0 | 1,0 | 1,0 | 1,2 | 1,0 | 1,0 | 1,0 | 1,0 | 1,0 | 1,0 | 1,0 | 1,0 | 1,0 | 9,8 | 1,0 | 1,0 | 1,0 | 1,0 | 1,0 | 1,0 | 1,0 |
| Asprv1 | 1,0 | 1,0 | 0,8 | 0,9 | 1,0 | 1,1 | 1,0 | 1,0 | 1,0 | 1,0 | 1,0 | 0,9 | 1,8 | 0,5 | 1,0 | 1,1 | 1,0 | 0,9 | 1,1 | 1,1 | 0,8 | 0,5 |

[Fig. 23-17]

286/310

| | | | | | | | | | | | | | | | | | | | | | | |
|---|---|---|---|---|---|---|---|---|---|---|---|---|---|---|---|---|---|---|---|---|---|---|
| Atcayos | 1,0 | 1,0 | 0,8 | 1,0 | 0,8 | 0,6 | 1,2 | 0,7 | 1,0 | 1,0 | 1,2 | 1,0 | 1,0 | 1,0 | 1,0 | 1,0 | 1,0 | 0,8 | 1,0 | 1,0 | 1,4 | 0,8 |
| Atp10b | 1,3 | 0,8 | 1,0 | 1,0 | 1,0 | 1,0 | 1,0 | 1,0 | 1,0 | 1,0 | 1,1 | 1,0 | 1,0 | 1,0 | 15,1 | 1,0 | 0,8 | 0,6 | 1,4 | 1,0 | 1,6 | 1,3 |
| Atp2a1 | 1,0 | 1,0 | 1,0 | 1,2 | 0,8 | 0,6 | 0,8 | 1,3 | 1,0 | 1,0 | 3,1 | 4,1 | 0,9 | 1,0 | 1,0 | 0,6 | 0,4 | 1,5 | 1,0 | 1,0 | 1,0 | 0,9 |
| Atp2b3 | 1,0 | 1,0 | 1,0 | 1,0 | 0,9 | 1,0 | 1,0 | 1,0 | 1,0 | 1,0 | 1,0 | 1,0 | 1,0 | 1,0 | 8,5 | 1,0 | 1,0 | 1,0 | 1,0 | 1,0 | 1,0 | 1,0 |
| Atp6v0c-ps2 | 1,0 | 0,2 | 1,0 | 0,8 | 1,0 | 2,6 | 1,0 | 0,6 | 1,0 | 1,0 | 1,0 | 1,0 | 1,0 | 2,0 | 1,0 | 1,0 | 1,0 | 1,9 | 1,0 | 1,0 | 1,0 | 1,0 |
| Atp6v0d2 | 1,0 | 1,5 | 1,0 | 1,0 | 1,0 | 1,2 | 1,0 | 1,0 | 1,5 | 1,9 | 1,1 | 1,1 | 1,5 | 1,3 | 11,6 | 0,5 | 2,0 | 2,9 | 1,0 | 1,0 | 1,0 | 1,0 |
| BC100530 | 1,0 | 1,0 | 1,0 | 1,0 | 1,0 | 1,8 | 1,0 | 1,0 | 1,0 | 1,0 | 1,0 | 1,0 | 1,0 | 1,0 | 1,0 | 1,0 | 0,4 | 5,5 | 1,0 | 1,0 | 1,0 | 1,0 |
| Basp1 | 1,0 | 2,1 | 0,8 | 0,5 | 1,0 | 1,0 | 0,8 | 0,8 | 0,9 | 0,7 | 0,7 | 0,8 | 1,2 | 0,8 | 8,0 | 1,2 | 1,2 | 1,1 | 1,3 | 1,1 | 0,7 | 0,7 |
| Bcl2a1b | 2,2 | 8,2 | 0,8 | 0,6 | 0,6 | 1,8 | 1,8 | 1,6 | 0,5 | 1,4 | 0,7 | 0,9 | 1,4 | 0,9 | 0,5 | 0,7 | 1,9 | 2,7 | 1,0 | 1,2 | 0,7 | 0,7 |
| Bcl2l15 | 1,0 | 1,0 | 1,0 | 1,0 | 1,0 | 1,0 | 1,0 | 1,0 | 1,0 | 1,0 | 1,0 | 1,0 | 2,4 | 1,2 | 26,4 | 1,0 | 1,0 | 1,0 | 1,2 | 1,1 | 1,0 | 0,8 |
| Bcl6 | 0,4 | 0,5 | 0,9 | 0,7 | 1,1 | 1,0 | 0,6 | 0,8 | 0,1 | 0,3 | 1,2 | 1,1 | 0,8 | 0,9 | 0,5 | 0,4 | 0,9 | 1,2 | 1,2 | 0,8 | 0,9 | 0,8 |
| Bcmo1 | 1,1 | 1,6 | 1,0 | 0,7 | 1,0 | 1,0 | 1,0 | 1,0 | 0,8 | 1,0 | 1,0 | 1,0 | 1,0 | 1,0 | 1,0 | 1,0 | 1,0 | 1,0 | 0,1 | 0,1 | 1,4 | 1,4 |
| Bex1 | 3,5 | 5,1 | 0,5 | 1,4 | 1,0 | 1,1 | 1,7 | 1,1 | 1,0 | 1,0 | 1,0 | 1,0 | 0,7 | 0,8 | 1,0 | 1,6 | 2,4 | 0,4 | 1,0 | 1,0 | 0,5 | 0,6 |
| Bex2 | 1,0 | 0,7 | 1,0 | 0,9 | 1,0 | 1,2 | 1,0 | 1,0 | 1,0 | 1,0 | 1,1 | 1,2 | 0,7 | 0,6 | 6,3 | 2,4 | 1,2 | 0,8 | 0,8 | 1,6 | 0,7 | 1,0 |
| Bex4 | 2,7 | 3,7 | 0,7 | 0,8 | 1,0 | 1,3 | 1,0 | 1,4 | 1,0 | 1,0 | 1,0 | 1,0 | 1,0 | 1,0 | 6,8 | 3,0 | 0,8 | 0,9 | 1,0 | 1,0 | 0,5 | 0,5 |
| | Kidney | | Colon | | Eyeball | | Heart | | Liver | | SG | | Thymus | | Adipose | | Stomach | | Jejunum | | Ileum | |
| | E | M | E | M | E | M | E | M | E | M | E | M | E | M | E | M | E | M | E | M | E | M |
| Bglap3 | 1,0 | 1,0 | 1,9 | 1,4 | 1,0 | 1,0 | 1,0 | 1,0 | 1,0 | 1,0 | 0,8 | 0,5 | 1,9 | 1,0 | 26,0 | 2,0 | 1,1 | 1,0 | 3,5 | 2,0 | 2,0 | 1,2 |
| Bhmt | 3,0 | 6,0 | 0,8 | 1,0 | 1,0 | 1,0 | 1,0 | 1,0 | 0,0 | 0,1 | 1,0 | 1,0 | 1,0 | 1,0 | 1,0 | 1,0 | 1,0 | 0,2 | 1,0 | 1,0 | 0,9 | 0,9 |
| Bmyc | 1,2 | 1,1 | 0,9 | 0,8 | 0,9 | 0,8 | 1,0 | 1,0 | 0,4 | 0,2 | 0,9 | 0,8 | 1,5 | 1,0 | 4,3 | 1,3 | 1,4 | 1,5 | 0,8 | 1,2 | 0,7 | 1,0 |
| Bpi | 1,0 | 1,0 | 1,0 | 1,0 | 1,0 | 1,0 | 1,0 | 1,0 | 1,0 | 1,0 | 1,0 | 1,0 | 1,0 | 1,0 | 8,4 | 1,0 | 1,0 | 1,0 | 1,0 | 1,0 | 1,0 | 1,0 |
| Bpifb1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,5 | 0,9 | 1,0 | 1,0 | 1,0 | 1,0 |
| Bsph1 | 1,0 | 1,0 | 1,0 | 1,0 | 1,0 | 1,0 | 1,0 | 1,0 | 1,0 | 1,0 | 1,0 | 1,0 | 1,0 | 1,0 | 39,8 | 1,0 | 1,0 | 1,0 | 1,0 | 1,0 | 1,0 | 1,0 |
| Bspry | 1,1 | 1,0 | 0,9 | 1,1 | 0,9 | 1,0 | 1,0 | 1,0 | 1,0 | 1,0 | 0,8 | 0,9 | 1,4 | 1,3 | 6,2 | 2,3 | 1,0 | 1,0 | 1,3 | 1,3 | 1,2 | 1,1 |
| Btg3 | 5,2 | 1,0 | 0,3 | 1,0 | 0,6 | 1,0 | 0,2 | 1,0 | 0,5 | 1,0 | 0,5 | 0,2 | 1,3 | 1,0 | 7,2 | 1,0 | 0,4 | 2,0 | 1,0 | 1,0 | 2,1 | 1,0 |
| Btnl10 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| C1qtnf3 | 0,5 | 0,5 | 1,1 | 1,0 | 0,7 | 1,0 | 1,0 | 1,0 | 1,0 | 1,0 | 1,0 | 1,0 | 1,0 | 1,0 | 1,1 | 0,7 | 3,2 | 2,1 | 1,0 | 1,0 | 1,4 | 1,2 |
| C1qtnf6 | 1,1 | 1,8 | 1,1 | 1,0 | 1,0 | 0,9 | 0,8 | 0,9 | 0,9 | 1,0 | 0,3 | 0,7 | 1,0 | 0,8 | 0,4 | 0,5 | 1,2 | 1,4 | 1,1 | 0,9 | 0,8 | 1,1 |
| C2cd4b | 1,0 | 1,0 | 2,2 | 1,1 | 1,0 | 1,0 | 1,0 | 1,6 | 1,0 | 1,0 | 1,0 | 1,0 | 1,0 | 1,0 | 1,0 | 1,0 | 1,3 | 0,8 | 1,0 | 1,0 | 2,3 | 0,7 |
| C3 | 18,6 | 25,9 | 1,0 | 0,9 | 1,2 | 0,5 | 1,2 | 1,1 | 1,8 | 1,6 | 0,9 | 1,9 | 1,9 | 1,5 | 1,7 | 1,2 | 1,0 | 0,8 | 1,4 | 0,7 | 1,2 | 1,3 |
| C4bp | 1,0 | 1,0 | 0,8 | 0,6 | 1,0 | 1,0 | 1,0 | 1,0 | 0,9 | 0,8 | 1,0 | 1,0 | 1,0 | 1,0 | 85,3 | 1,0 | 1,2 | 0,8 | 1,0 | 1,0 | 0,9 | 1,0 |
| C6 | 1,0 | 1,0 | 1,0 | 1,0 | 1,0 | 1,0 | 1,0 | 1,0 | 0,0 | 0,1 | 1,0 | 1,0 | 1,1 | 1,0 | 0,4 | 0,6 | 1,0 | 1,0 | 0,9 | 0,8 | 0,7 | 0,7 |
| C8b | 1,0 | 1,0 | 1,0 | 1,0 | 1,0 | 1,0 | 1,0 | 1,0 | 0,0 | 0,2 | 1,0 | 1,0 | 1,0 | 1,0 | 1,0 | 1,0 | 1,0 | 1,0 | 1,0 | 1,0 | 1,0 | 1,0 |
| Cacna1s | 1,0 | 1,0 | 1,0 | 1,0 | 0,8 | 0,8 | 1,4 | 1,1 | 1,0 | 1,0 | 1,0 | 1,0 | 1,0 | 1,0 | 1,0 | 1,0 | 1,0 | 1,0 | 1,0 | 1,0 | 1,0 | 1,0 |
| Cacng1 | 1,0 | 1,0 | 1,0 | 1,0 | 1,0 | 0,6 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Cacng6 | 1,0 | 1,0 | 1,0 | 1,0 | 0,4 | 0,7 | 0,9 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Calm4 | 1,0 | 1,0 | 1,0 | 0,6 | 1,0 | 1,0 | 1,0 | 1,0 | 1,0 | 1,0 | 1,0 | 1,0 | 2,1 | 0,5 | 1,0 | 1,0 | 1,3 | 0,9 | 1,0 | 1,0 | 1,0 | 1,0 |
| Calml3 | 2,5 | 3,0 | 1,0 | 1,0 | 1,0 | 1,1 | 1,0 | 1,0 | 1,0 | 1,0 | 1,1 | 1,2 | 2,3 | 1,8 | 2,1 | 6,5 | 1,0 | 1,1 | 1,0 | 1,0 | 1,0 | 1,0 |
| Camk1g | 1,0 | 1,0 | 1,0 | 1,0 | 1,1 | 1,0 | 1,0 | 1,1 | 1,0 | 1,0 | 1,0 | 1,0 | 0,7 | 1,1 | 1,0 | 1,0 | 1,0 | 1,0 | 1,0 | 1,0 | 1,0 | 1,0 |
| Camk2a | 1,0 | 1,0 | 1,0 | 1,0 | 0,9 | 0,7 | 1,1 | 1,0 | 1,0 | 1,0 | 1,0 | 1,0 | 0,8 | 1,3 | 1,0 | 1,0 | 1,0 | 1,0 | 1,0 | 1,0 | 1,0 | 1,3 |
| Camk2b | 1,3 | 1,7 | 1,3 | 1,2 | 1,0 | 0,9 | 0,7 | 1,0 | 0,8 | 0,6 | 1,0 | 1,4 | 1,8 | 1,7 | 6,6 | 1,7 | 1,1 | 1,0 | 1,1 | 1,4 | 1,0 | 0,8 |
| Cap2 | 1,0 | 1,0 | 1,2 | 1,0 | 0,9 | 0,9 | 1,1 | 1,1 | 1,0 | 1,0 | 1,0 | 1,0 | 1,0 | 1,0 | 1,1 | 1,0 | 1,1 | 0,7 | 1,4 | 1,4 | 0,9 | 0,8 |
| Capn5 | 1,5 | 1,4 | 1,1 | 1,1 | 1,0 | 1,1 | 0,9 | 0,8 | 1,3 | 1,0 | 1,1 | 1,1 | 1,1 | 1,4 | 6,3 | 0,8 | 0,9 | 0,8 | 1,6 | 1,1 | 1,1 | 1,5 |
| Car1 | 1,0 | 1,0 | 1,1 | 1,6 | 1,0 | 1,0 | 1,0 | 1,0 | 0,0 | 0,2 | 1,0 | 1,0 | 1,0 | 1,0 | 0,8 | 1,4 | 1,0 | 1,0 | 1,0 | 1,0 | 1,0 | 1,0 |
| Car2 | 1,1 | 1,4 | 1,6 | 1,3 | 1,0 | 1,1 | 1,2 | 1,2 | 1,4 | 1,7 | 1,4 | 1,2 | 1,0 | 0,7 | 3,0 | 2,1 | 1,2 | 1,0 | 1,1 | 1,1 | 0,9 | 0,9 |
| Car3 | 0,2 | 0,1 | 0,8 | 1,9 | 0,8 | 0,9 | 3,2 | 1,0 | 0,0 | 0,1 | 1,4 | 4,1 | 1,7 | 6,1 | 0,8 | 1,1 | 0,8 | 1,1 | 1,2 | 1,0 | 0,8 | 2,2 |
| Car4 | 0,2 | 0,2 | 1,3 | 1,4 | 0,6 | 0,6 | 0,9 | 1,4 | 1,0 | 1,0 | 1,0 | 1,0 | 1,8 | 2,8 | 1,0 | 1,7 | 1,2 | 1,7 | 1,7 | 2,1 | 1,2 | 1,5 |
| Car5b | 1,2 | 1,3 | 1,3 | 1,3 | 0,9 | 1,0 | 1,6 | 1,4 | 1,4 | 1,4 | 1,0 | 1,2 | 2,5 | 5,8 | 1,3 | 1,7 | 1,1 | 1,4 | 0,9 | 1,0 | 0,9 | 1,9 |
| Car6 | 1,0 | 1,0 | 1,0 | 1,0 | 1,1 | 0,8 | 1,0 | 0,6 | 1,7 | 1,0 | 1,3 | 1,0 | 1,0 | 0,6 | 1,0 | 1,7 | 1,0 | 0,9 | 1,0 | 1,0 | 1,0 | 1,0 |
| Casc5 | 1,0 | 1,0 | 0,7 | 0,9 | 1,0 | 1,0 | 1,0 | 1,0 | 1,0 | 1,0 | 1,0 | 1,0 | 0,2 | 0,4 | 1,0 | 1,0 | 1,0 | 1,0 | 0,8 | 0,5 | 1,2 | 1,4 |
| Casq1 | 1,0 | 1,0 | 1,0 | 1,0 | 0,7 | 0,5 | 0,8 | 0,8 | 1,0 | 1,0 | 1,0 | 1,0 | 1,0 | 1,0 | 1,0 | 1,0 | 1,0 | 0,9 | 1,0 | 1,0 | 1,0 | 1,0 |
| Cav3 | 1,0 | 1,0 | 1,3 | 1,0 | 0,6 | 0,7 | 1,0 | 1,0 | 1,0 | 1,0 | 1,0 | 1,0 | 1,0 | 1,0 | 1,0 | 1,0 | 1,5 | 1,3 | 1,0 | 1,1 | 0,7 | 0,8 |
| Cbl | 1,7 | 0,8 | 0,9 | 1,2 | 1,5 | 1,0 | 0,9 | 0,7 | 1,7 | 1,0 | 0,8 | 2,3 | 0,1 | 0,4 | 0,7 | 0,4 | 0,6 | 0,8 | 0,6 | 0,3 | 1,0 | 2,7 |
| Cbr1 | 4,8 | 4,8 | 1,5 | 1,4 | 1,0 | 1,0 | 0,8 | 0,9 | 3,9 | 6,4 | 1,1 | 1,0 | 1,1 | 1,1 | 1,3 | 1,7 | 1,0 | 0,9 | 1,0 | 1,2 | 1,2 | 0,9 |
| Cbr3 | 5,9 | 5,7 | 2,2 | 1,5 | 0,8 | 0,7 | 1,1 | 0,6 | 4,7 | 11,8 | 1,0 | 0,8 | 1,8 | 1,3 | 1,5 | 1,5 | 1,0 | 0,9 | 1,5 | 2,4 | 2,1 | 1,4 |
| Ccl17 | 0,5 | 1,6 | 1,0 | 1,0 | 1,0 | 1,4 | 1,1 | 1,0 | 1,0 | 1,0 | 1,0 | 1,0 | 1,0 | 1,2 | 1,5 | 0,9 | 1,0 | 0,7 | 1,1 | 1,1 | 0,5 | 0,7 |
| Ccl2 | 1,0 | 5,9 | 0,8 | 0,6 | 1,0 | 1,7 | 1,3 | 1,3 | 1,0 | 1,7 | 1,0 | 0,7 | 0,8 | 1,3 | 0,5 | 0,7 | 2,1 | 1,8 | 1,0 | 0,8 | 1,0 | 0,9 |
| Ccl20 | 1,0 | 1,0 | 0,5 | 0,7 | 1,0 | 1,0 | 1,0 | 1,0 | 1,0 | 1,0 | 1,0 | 1,0 | 2,1 | 0,9 | 1,0 | 1,0 | 1,0 | 1,0 | 10,6 | 5,1 | 2,6 | 2,4 |
| Ccl21b | 3,6 | 0,3 | 6,3 | 7,4 | 0,5 | 1,6 | 4,2 | 2,0 | 0,8 | 0,9 | 0,4 | 2,1 | 1,0 | 1,0 | 0,5 | 3,5 | 0,4 | 0,3 | 2,8 | 1,5 | 4,9 | 0,1 |
| Ccl6 | 1,7 | 5,8 | 0,9 | 1,0 | 1,1 | 1,1 | 1,5 | 1,9 | 1,2 | 2,0 | 0,9 | 1,1 | 2,0 | 1,5 | 0,6 | 0,9 | 1,4 | 1,6 | 0,7 | 1,0 | 0,9 | 0,8 |
| Ccl7 | 1,0 | 1,0 | 0,5 | 0,3 | 1,0 | 1,0 | 1,5 | 0,5 | 1,0 | 1,0 | 1,0 | 1,0 | 1,1 | 0,9 | 0,3 | 0,2 | 1,2 | 1,1 | 1,0 | 1,0 | 0,8 | 0,7 |
| Ccl8 | 1,0 | 1,0 | 0,9 | 0,3 | 1,0 | 0,5 | 1,0 | 2,7 | 1,0 | 1,0 | 0,6 | 1,0 | 1,6 | 1,3 | 0,3 | 0,4 | 1,6 | 1,7 | 0,8 | 0,6 | 1,5 | 0,7 |
| Ccnb1 | 1,0 | 1,0 | 0,8 | 0,9 | 1,0 | 1,0 | 1,0 | 1,0 | 1,0 | 1,0 | 1,0 | 1,0 | 0,4 | 0,7 | 1,0 | 1,0 | 1,0 | 0,8 | 1,2 | 1,0 | 1,1 | 1,1 |
| Ccne2 | 1,0 | 1,0 | 0,8 | 0,7 | 1,0 | 1,4 | 1,0 | 1,0 | 1,0 | 1,0 | 1,0 | 1,0 | 0,3 | 0,5 | 1,0 | 1,0 | 0,9 | 1,0 | 1,0 | 0,7 | 0,7 | 0,4 |
| Ccno | 1,0 | 1,0 | 1,0 | 1,0 | 1,0 | 1,0 | 1,0 | 1,0 | 0,8 | 1,0 | 1,0 | 1,0 | 1,9 | 1,0 | 21,2 | 1,0 | 1,0 | 1,0 | 1,0 | 1,0 | 1,0 | 1,0 |
| Cd19 | 1,0 | 1,0 | 0,4 | 0,3 | 1,0 | 1,0 | 1,0 | 1,0 | 1,0 | 1,0 | 0,6 | 1,3 | 1,5 | 0,9 | 1,0 | 1,0 | 1,0 | 1,0 | 1,0 | 0,8 | 0,7 | 0,8 |
| Cd22 | 1,0 | 1,0 | 0,4 | 0,2 | 1,0 | 1,0 | 1,0 | 1,0 | 1,0 | 1,0 | 0,6 | 2,0 | 1,7 | 1,1 | 1,0 | 1,0 | 1,0 | 1,2 | 0,6 | 0,6 | 0,7 | 1,0 |
| Cd27 | 1,0 | 1,0 | 0,8 | 0,6 | 1,0 | 1,0 | 1,0 | 1,0 | 1,0 | 1,0 | 0,9 | 1,0 | 0,8 | 0,9 | 0,9 | 0,9 | 1,0 | 1,0 | 1,3 | 0,8 | 0,9 | 0,6 |
| Cd300lh | 0,7 | 1,0 | 0,5 | 0,5 | 1,0 | 1,0 | 1,2 | 0,3 | 1,0 | 1,0 | 0,6 | 1,6 | 0,6 | 0,5 | 0,4 | 0,4 | 0,6 | 0,8 | 1,0 | 1,0 | 1,0 | 1,0 |
| Cd3e | 1,0 | 1,0 | 0,8 | 0,7 | 1,0 | 1,0 | 1,0 | 1,0 | 1,0 | 1,0 | 1,0 | 1,4 | 0,6 | 0,8 | 1,0 | 0,9 | 1,0 | 1,0 | 1,0 | 0,9 | 0,8 | 0,5 |
| Cd4 | 1,0 | 1,0 | 1,0 | 0,4 | 1,0 | 0,8 | 1,0 | 1,0 | 0,9 | 0,8 | 0,9 | 1,4 | 0,7 | 0,8 | 0,9 | 0,7 | 0,9 | 1,0 | 0,9 | 0,8 | 0,8 | 0,8 |
| Cd52 | 1,0 | 2,4 | 0,6 | 0,3 | 1,0 | 0,9 | 1,4 | 1,2 | 0,8 | 1,4 | 0,5 | 0,8 | 1,2 | 0,9 | 3,8 | 2,0 | 1,1 | 2,1 | 1,0 | 1,1 | 0,8 | 0,5 |
| Cd5l | 1,0 | 1,0 | 1,0 | 1,0 | 1,0 | 1,7 | 1,0 | 1,0 | 1,1 | 1,2 | 1,0 | 1,0 | 2,0 | 1,1 | 1,0 | 0,6 | 1,0 | 1,0 | 1,0 | 1,0 | 1,0 | 1,0 |
| Cd79a | 1,0 | 1,0 | 0,5 | 0,3 | 1,0 | 1,0 | 1,0 | 1,0 | 1,0 | 1,0 | 0,7 | 2,1 | 2,3 | 1,0 | 0,6 | 0,8 | 1,0 | 1,0 | 1,2 | 1,0 | 0,8 | 0,8 |
| Cd79b | 0,8 | 0,8 | 0,4 | 0,3 | 1,0 | 1,0 | 1,0 | 0,8 | 0,8 | 0,6 | 0,5 | 2,5 | 1,2 | 1,0 | 0,4 | 1,2 | 1,0 | 1,4 | 0,9 | 0,8 | 0,7 | 0,6 |
| Cd8a | 1,0 | 1,0 | 0,9 | 0,8 | 1,0 | 1,0 | 1,0 | 1,0 | 1,0 | 1,0 | 1,0 | 1,1 | 0,6 | 0,7 | 1,0 | 1,0 | 1,0 | 1,0 | 1,1 | 1,0 | 0,8 | 0,6 |
| Cd8b1 | 1,0 | 1,0 | 0,4 | 1,0 | 1,0 | 1,0 | 1,0 | 1,0 | 1,0 | 1,0 | 1,0 | 1,5 | 0,6 | 0,8 | 1,0 | 1,0 | 1,0 | 1,0 | 1,0 | 0,8 | 1,2 | 0,4 |
| Cdcp1 | 1,1 | 0,9 | 1,1 | 1,1 | 1,3 | 0,8 | 1,0 | 1,0 | 1,1 | 0,7 | 0,9 | 1,3 | 1,3 | 1,5 | 5,9 | 1,0 | 0,8 | 0,9 | 1,0 | 0,6 | 1,1 | 1,4 |
| Cdh3 | 1,5 | 2,2 | 1,0 | 1,0 | 1,2 | 1,0 | 1,0 | 1,0 | 1,0 | 1,0 | 0,9 | 0,7 | 1,9 | 1,7 | 5,4 | 1,0 | 0,9 | 1,3 | 1,0 | 1,0 | 1,0 | 1,0 |
| Cdk6 | 1,0 | 0,8 | 0,9 | 1,2 | 1,0 | 1,0 | 0,5 | 0,6 | 2,6 | 0,8 | 1,0 | 1,0 | 0,2 | 0,3 | 1,0 | 1,0 | 0,9 | 1,5 | 0,5 | 0,4 | 1,1 | 1,2 |
| Ceacam10 | 1,0 | 1,0 | 1,2 | 0,7 | 0,9 | 1,0 | 1,0 | 1,0 | 1,0 | 1,0 | 1,3 | 1,4 | 1,0 | 1,0 | 11,0 | 1,9 | 0,8 | 0,7 | 2,9 | 1,9 | 1,5 | 1,1 |
| Cel | 0,9 | 1,1 | 1,6 | 73,9 | 1,0 | 1,0 | 1,0 | 1,0 | 1,0 | 0,9 | 1,0 | 1,0 | 1,0 | 0,4 | 1,0 | 1,0 | 13,2 | 0,1 | 0,0 | 1,0 | 1,0 | 1,0 |
| Cela1 | 0,5 | 0,9 | 1,0 | 2,3 | 1,1 | 1,3 | 1,0 | 1,0 | 0,0 | 0,3 | 0,7 | 1,0 | 1,1 | 0,3 | 1,0 | 0,8 | 5,5 | 0,2 | 0,1 | 1,4 | 1,0 | 1,1 |
| Cela2a | 1,3 | 0,8 | 1,3 | 98,2 | 1,0 | 1,0 | 1,0 | 1,0 | 1,0 | 0,8 | 1,3 | 1,0 | 1,0 | 0,3 | 1,0 | 1,0 | 220,7 | 0,0 | 0,0 | 1,0 | 0,7 | 1,0 |

[Fig. 23-18]

| | | | | | | | | | | | | | | | | | | | | | | |
|---|---|---|---|---|---|---|---|---|---|---|---|---|---|---|---|---|---|---|---|---|---|---|
| Cela3b | 1,0 | 0,9 | 2,2 | 89,5 | 1,0 | 1,0 | 1,0 | 1,0 | 1,0 | 0,8 | 1,1 | 1,0 | 1,0 | 0,4 | 1,0 | 1,0 | 154,8 | 0,1 | 0,0 | 1,0 | 1,0 | 1,0 |
| Ces1c | 1,0 | 1,0 | 1,0 | 1,0 | 1,0 | 1,0 | 1,0 | 1,0 | 1,4 | 1,9 | 1,0 | 1,0 | 1,0 | 1,0 | 1,0 | 1,0 | 5,9 | 0,5 | 1,0 | 1,0 | 0,9 | 1,1 |
| Ces1g | 0,3 | 0,3 | 1,3 | 1,3 | 1,0 | 1,0 | 1,0 | 1,0 | 1,4 | 2,4 | 1,0 | 1,0 | 1,0 | 1,0 | 1,0 | 1,0 | 1,3 | 0,9 | 2,2 | 2,7 | 1,6 | 0,8 |
| Ces2g | 1,3 | 1,1 | 0,8 | 1,1 | 1,0 | 1,3 | 1,0 | 1,5 | 1,0 | 0,8 | 1,0 | 1,0 | 1,5 | 1,3 | 0,7 | 0,7 | 1,0 | 0,9 | 1,0 | 1,1 | 1,0 | 1,3 |
| Ces3a | 1,0 | 1,0 | 1,0 | 1,0 | 1,0 | 1,0 | 1,0 | 1,0 | 0,0 | 0,1 | 1,0 | 1,0 | 1,0 | 1,0 | 1,0 | 1,0 | 1,0 | 0,3 | 1,0 | 1,0 | 1,0 | 1,0 |
| Ces3b | 1,0 | 1,0 | 1,0 | 1,0 | 1,0 | 1,0 | 1,0 | 1,0 | 0,0 | 0,3 | 1,0 | 1,0 | 1,0 | 1,0 | 1,0 | 1,0 | 1,0 | 1,0 | 1,0 | 1,0 | 1,0 | 1,0 |
| Ces5a | 1,0 | 1,0 | 1,0 | 1,0 | 0,9 | 1,2 | 1,0 | 1,0 | 1,0 | 1,0 | 1,0 | 1,0 | 1,0 | 1,0 | 7,7 | 1,0 | 1,0 | 1,0 | 1,0 | 1,0 | 1,0 | 1,0 |
| Cfd | 2,1 | 0,8 | 1,1 | 2,0 | 0,3 | 0,5 | 4,4 | 1,3 | 1,0 | 0,7 | 1,7 | 3,0 | 3,4 | 4,9 | 1,1 | 1,8 | 0,9 | 1,1 | 2,1 | 1,9 | 1,7 | 1,7 |
| Cgref1 | 0,9 | 1,0 | 0,9 | 0,8 | 0,8 | 1,2 | 1,0 | 0,7 | 1,4 | 1,1 | 0,8 | 1,0 | 1,0 | 1,0 | 0,4 | 1,5 | 1,4 | 1,0 | 0,8 | 1,2 | 1,1 | 0,9 |
| Chac1 | 0,7 | 0,8 | 0,5 | 0,4 | 0,8 | 0,9 | 0,5 | 1,3 | 0,4 | 0,6 | 0,3 | 1,4 | 1,0 | 1,0 | 0,7 | 1,5 | 0,9 | 1,1 | 0,5 | 0,7 | 0,7 | 0,4 |
| Chga | 1,0 | 1,0 | 0,8 | 1,0 | 1,0 | 1,0 | 1,0 | 0,7 | 1,0 | 1,0 | 1,0 | 1,0 | 5,8 | 4,8 | 1,0 | 1,0 | 1,9 | 1,3 | 0,8 | 1,1 | 0,8 | 0,9 |
| Chrna4 | 0,8 | 0,9 | 1,0 | 1,0 | 1,1 | 1,0 | 1,0 | 1,0 | 2,1 | 0,2 | 1,0 | 1,0 | 1,0 | 1,0 | 1,0 | 1,0 | 1,0 | 1,0 | 1,0 | 1,0 | 1,0 | 1,0 |
| Chst11 | 0,5 | 0,2 | 1,4 | 1,5 | 0,8 | 0,8 | 1,2 | 1,1 | 1,0 | 1,0 | 0,8 | 1,0 | 1,2 | 1,3 | 1,0 | 1,1 | 1,1 | 1,4 | 0,7 | 0,8 | 0,8 | 1,1 |
| Cib3 | 1,0 | 1,0 | 1,0 | 1,0 | 1,0 | 1,0 | 1,0 | 0,9 | 0,8 | 0,3 | 1,0 | 1,0 | 1,0 | 1,0 | 1,0 | 1,0 | 1,0 | 1,0 | 1,0 | 1,0 | 1,0 | 1,0 |
| Cidea | 1,0 | 1,0 | 1,0 | 1,0 | 0,3 | 0,7 | 1,6 | 1,0 | 1,0 | 1,0 | 1,0 | 2,8 | 0,9 | 3,0 | 0,4 | 0,9 | 0,9 | 0,9 | 1,0 | 1,0 | 1,0 | 1,0 |
| Cilp | 1,0 | 1,0 | 1,1 | 0,7 | 0,8 | 0,8 | 0,7 | 1,0 | 1,0 | 1,0 | 0,4 | 0,3 | 1,3 | 1,3 | 0,7 | 0,5 | 2,3 | 3,4 | 0,9 | 0,8 | 0,9 | 1,1 |
| Cilp2 | 1,0 | 1,0 | 1,0 | 1,0 | 0,7 | 0,5 | 0,7 | 0,7 | 1,0 | 1,0 | 1,0 | 1,0 | 1,0 | 1,0 | 1,0 | 1,0 | 1,0 | 1,0 | 1,0 | 1,0 | 1,0 | 1,0 |
| Cited1 | 1,0 | 1,0 | 1,0 | 1,0 | 0,8 | 0,9 | 1,6 | 1,1 | 1,0 | 1,0 | 1,0 | 1,0 | 1,0 | 1,0 | 13,9 | 8,5 | 1,0 | 1,0 | 1,0 | 1,0 | 1,0 | 1,0 |
| Cited4 | 1,7 | 0,9 | 1,1 | 0,9 | 0,9 | 0,9 | 1,2 | 0,6 | 1,0 | 1,0 | 1,1 | 1,0 | 1,2 | 1,4 | 5,4 | 4,2 | 1,3 | 1,1 | 1,0 | 1,0 | 0,7 | 0,7 |
| Ckm | 1,0 | 1,0 | 1,0 | 1,0 | 0,7 | 0,5 | 0,9 | 1,1 | 1,0 | 1,0 | 6,0 | 2,3 | 0,4 | 0,8 | 1,8 | 1,9 | 0,7 | 1,0 | 1,3 | 1,1 | 0,6 | 1,0 |
| Ckmt2 | 1,0 | 1,0 | 2,0 | 2,7 | 0,7 | 0,8 | 0,9 | 1,1 | 1,0 | 1,0 | 1,0 | 1,0 | 1,0 | 1,0 | 1,0 | 1,0 | 1,0 | 1,0 | 1,0 | 1,0 | 1,0 | 1,0 |
| Clca3 | 1,0 | 1,0 | 0,2 | 0,3 | 0,9 | 0,7 | 1,0 | 1,0 | 1,0 | 1,0 | 1,0 | 1,0 | 1,1 | 1,0 | 1,0 | 1,0 | 0,6 | 0,5 | 1,1 | 1,2 | 0,8 | 1,1 |
| Cldn1 | 1,2 | 1,7 | 1,3 | 1,0 | 1,4 | 1,4 | 1,0 | 1,0 | 1,1 | 0,6 | 1,0 | 0,7 | 1,7 | 1,4 | 7,1 | 1,6 | 1,0 | 1,0 | 1,0 | 1,0 | 1,0 | 1,0 |
| Cldn13 | 1,0 | 1,0 | 1,0 | 1,0 | 1,0 | 1,0 | 1,0 | 1,0 | 1,0 | 1,0 | 1,0 | 1,0 | 1,2 | 0,8 | 1,0 | 1,0 | 1,0 | 1,0 | 1,0 | 1,0 | 1,0 | 1,0 |
| Cldn14 | 1,0 | 1,0 | 1,1 | 1,2 | 1,0 | 1,0 | 1,0 | 1,0 | 0,6 | 0,6 | 1,0 | 1,0 | 1,0 | 1,0 | 17,9 | 1,0 | 1,0 | 1,0 | 1,0 | 1,0 | 1,0 | 1,0 |
| Cldn2 | 0,9 | 0,9 | 2,1 | 1,7 | 1,2 | 1,5 | 1,0 | 1,0 | 1,1 | 0,6 | 1,0 | 1,0 | 1,0 | 1,0 | 5,7 | 6,3 | 2,1 | 1,6 | 1,4 | 1,2 | 1,3 | 1,4 |
| Cldn22 | 1,0 | 0,9 | 1,0 | 1,0 | 0,8 | 1,2 | 1,0 | 1,0 | 1,0 | 1,0 | 2,6 | 8,7 | 1,0 | 1,0 | 1,7 | 2,8 | 1,3 | 0,9 | 1,0 | 1,0 | 1,0 | 1,0 |
| Cldn24 | 1,0 | 1,0 | 1,0 | 1,0 | 1,0 | 1,0 | 1,0 | 1,0 | 1,0 | 1,0 | 3,4 | 12,9 | 1,0 | 1,0 | 1,6 | 2,4 | 1,0 | 1,0 | 1,0 | 1,0 | 1,0 | 1,0 |
| Cldn3 | 1,2 | 1,2 | 1,0 | 1,1 | 1,1 | 0,9 | 1,0 | 1,0 | 0,7 | 0,7 | 1,2 | 0,8 | 3,2 | 1,4 | 11,1 | 4,1 | 2,5 | 0,8 | 1,5 | 1,3 | 1,1 | 1,1 |
| Cldn4 | 1,7 | 1,8 | 0,9 | 1,9 | 1,1 | 1,0 | 1,0 | 1,0 | 1,0 | 1,0 | 0,9 | 0,9 | 1,0 | 0,8 | 40,9 | 2,7 | 1,1 | 1,1 | 0,7 | 2,7 | 1,7 | 1,3 |
| | Kidney | | Colon | | Eyeball | | Heart | | Liver | | SG | | Thymus | | Adipose | | Stomach | | Jejunum | | Ileum | |
| | E | M | E | M | E | M | E | M | E | M | E | M | E | M | E | M | E | M | E | M | E | M |
| Cldn7 | 1,4 | 1,6 | 1,0 | 1,0 | 1,0 | 1,0 | 1,0 | 1,0 | 1,1 | 1,1 | 1,0 | 0,8 | 1,7 | 0,7 | 5,4 | 5,8 | 1,5 | 0,9 | 1,1 | 1,5 | 1,1 | 0,9 |
| Cldn8 | 1,1 | 1,1 | 1,4 | 1,0 | 1,0 | 1,0 | 1,0 | 1,0 | 1,0 | 1,0 | 1,0 | 1,2 | 1,0 | 1,0 | 24,2 | 3,3 | 1,0 | 1,1 | 1,0 | 1,0 | 0,5 | 0,6 |
| Clec11a | 0,7 | 0,9 | 0,8 | 0,9 | 0,6 | 0,7 | 1,0 | 0,6 | 1,0 | 0,8 | 0,6 | 0,9 | 1,4 | 1,9 | 2,0 | 1,2 | 1,2 | 0,9 | 1,0 | 1,0 | 1,0 | 1,0 |
| Clec2h | 0,7 | 0,6 | 1,0 | 1,0 | 1,0 | 1,0 | 1,0 | 1,0 | 0,0 | 0,2 | 1,0 | 1,0 | 1,0 | 1,0 | 1,0 | 1,0 | 1,0 | 1,0 | 1,3 | 2,1 | 1,2 | 0,9 |
| Clec2l | 1,0 | 1,0 | 1,0 | 1,0 | 1,0 | 1,2 | 0,8 | 1,0 | 1,0 | 1,0 | 1,0 | 1,0 | 1,0 | 1,0 | 5,9 | 1,0 | 1,0 | 1,0 | 1,0 | 1,0 | 1,0 | 1,0 |
| Clec7a | 1,0 | 6,5 | 1,2 | 0,9 | 1,1 | 1,3 | 1,0 | 1,9 | 0,6 | 1,1 | 0,7 | 0,7 | 1,1 | 0,9 | 0,6 | 0,7 | 1,9 | 5,9 | 0,9 | 0,9 | 0,9 | 0,9 |
| Clhc1 | 1,0 | 1,0 | 0,6 | 0,8 | 1,0 | 1,0 | 1,0 | 1,0 | 1,0 | 1,0 | 1,0 | 1,0 | 0,5 | 0,6 | 1,0 | 1,0 | 1,4 | 0,7 | 1,6 | 1,6 | 1,0 | 0,8 |
| Clps | 0,9 | 1,2 | 1,0 | 1,9 | 1,0 | 1,3 | 1,0 | 0,6 | 0,7 | 0,9 | 0,9 | 0,8 | 1,0 | 2,4 | 1,0 | 7,0 | 0,9 | 0,9 | 0,3 | 0,8 | 0,7 | 0,6 |
| Clpsl2 | 1,0 | 1,0 | 1,0 | 1,0 | 1,0 | 1,0 | 1,0 | 1,0 | 1,0 | 1,0 | 1,0 | 1,0 | 1,0 | 1,0 | 181,5 | 1,0 | 1,0 | 1,0 | 1,0 | 1,0 | 1,0 | 1,0 |
| Clu | 1,9 | 2,7 | 0,6 | 0,4 | 1,0 | 1,1 | 1,2 | 1,2 | 0,6 | 0,8 | 0,4 | 0,7 | 2,1 | 1,3 | 25,9 | 0,9 | 0,9 | 1,1 | 0,6 | 0,7 | 0,7 | 0,7 |
| Cmah | 0,7 | 0,8 | 1,6 | 1,7 | 0,8 | 1,1 | 1,0 | 1,0 | 0,5 | 0,3 | 1,0 | 1,0 | 0,5 | 0,7 | 0,6 | 0,6 | 1,0 | 1,0 | 0,9 | 0,8 | 0,7 | 1,1 |
| Cml5 | 1,0 | 0,6 | 1,7 | 1,5 | 1,0 | 1,0 | 1,0 | 1,0 | 0,0 | 0,0 | 1,0 | 1,0 | 1,0 | 1,0 | 1,0 | 1,0 | 1,0 | 1,0 | 1,3 | 1,4 | 2,4 | 1,7 |
| Cmya5 | 1,0 | 1,0 | 1,0 | 1,0 | 1,0 | 0,6 | 0,9 | 0,9 | 1,0 | 1,0 | 1,0 | 1,0 | 1,0 | 1,0 | 1,0 | 1,0 | 1,0 | 1,0 | 1,0 | 1,0 | 1,0 | 1,0 |
| Cnfn | 1,0 | 1,0 | 1,0 | 1,0 | 1,0 | 1,6 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Cnn1 | 0,8 | 1,1 | 1,0 | 0,9 | 0,7 | 0,8 | 2,0 | 1,0 | 1,0 | 1,0 | 0,7 | 0,6 | 1,0 | 1,0 | 2,2 | 8,0 | 1,4 | 1,1 | 1,1 | 1,2 | 0,7 | 0,8 |
| Col15a1 | 0,6 | 1,3 | 0,9 | 0,6 | 0,8 | 0,6 | 0,5 | 0,5 | 1,1 | 0,7 | 0,4 | 0,5 | 1,1 | 1,2 | 0,4 | 0,4 | 1,0 | 1,2 | 0,8 | 0,7 | 0,6 | 0,8 |
| Col1a1 | 0,8 | 1,9 | 1,0 | 0,6 | 0,6 | 0,6 | 0,7 | 0,5 | 0,8 | 0,5 | 0,0 | 0,3 | 0,7 | 1,2 | 0,5 | 0,5 | 1,4 | 1,4 | 1,5 | 1,0 | 0,9 | 1,0 |
| Col1a2 | 0,8 | 1,8 | 1,0 | 0,7 | 0,7 | 0,8 | 0,7 | 0,7 | 0,6 | 0,7 | 0,3 | 0,4 | 0,8 | 1,1 | 0,5 | 0,5 | 1,1 | 1,1 | 1,1 | 0,9 | 0,8 | 0,9 |
| Col3a1 | 0,8 | 2,7 | 0,9 | 0,5 | 0,4 | 0,4 | 0,5 | 0,4 | 0,5 | 0,5 | 0,1 | 0,2 | 0,5 | 0,7 | 0,3 | 0,4 | 1,2 | 1,3 | 1,0 | 0,9 | 0,8 | 0,7 |
| Col5a1 | 1,1 | 1,6 | 1,2 | 0,7 | 0,8 | 0,6 | 0,8 | 0,8 | 1,0 | 1,0 | 0,8 | 0,7 | 1,1 | 1,1 | 0,4 | 0,5 | 1,4 | 1,4 | 1,3 | 0,9 | 1,0 | 0,9 |
| Col5a2 | 1,0 | 1,8 | 1,2 | 0,6 | 0,8 | 0,8 | 0,7 | 0,6 | 1,0 | 1,0 | 0,7 | 0,9 | 1,1 | 1,1 | 0,3 | 0,5 | 1,2 | 1,4 | 0,8 | 0,9 | 0,8 | 1,2 |
| Col6a2 | 0,8 | 1,5 | 1,1 | 0,7 | 0,7 | 0,7 | 0,6 | 0,6 | 0,8 | 0,7 | 0,3 | 0,4 | 1,3 | 1,2 | 0,4 | 0,4 | 1,2 | 1,1 | 1,2 | 1,0 | 0,8 | 1,0 |
| Col6a3 | 1,0 | 1,1 | 0,9 | 0,6 | 0,8 | 0,7 | 0,7 | 0,4 | 1,0 | 0,8 | 0,4 | 0,6 | 1,3 | 1,3 | 0,4 | 0,3 | 1,0 | 1,3 | 1,0 | 0,7 | 0,8 | 1,2 |
| Col6a4 | 1,0 | 1,0 | 1,0 | 0,7 | 1,0 | 1,0 | 1,0 | 1,0 | 1,0 | 1,0 | 1,0 | 1,0 | 1,1 | 1,0 | 6,7 | 1,0 | 0,9 | 1,9 | 0,9 | 0,9 | 1,0 | 1,3 |
| Col6a6 | 1,0 | 1,0 | 1,0 | 1,0 | 1,0 | 1,0 | 0,6 | 0,7 | 1,0 | 1,0 | 1,0 | 1,0 | 1,0 | 1,2 | 0,6 | 0,3 | 1,0 | 1,0 | 1,0 | 1,0 | 1,0 | 1,0 |
| Cox6a2 | 0,5 | 1,0 | 1,0 | 1,0 | 0,7 | 0,8 | 1,2 | 1,2 | 1,0 | 1,0 | 1,0 | 1,0 | 1,2 | 0,4 | 1,0 | 1,0 | 1,0 | 1,2 | 1,0 | 1,0 | 1,0 | 1,0 |
| Cox7a1 | 0,8 | 0,8 | 0,4 | 1,3 | 0,6 | 0,6 | 0,9 | 0,8 | 1,0 | 1,0 | 1,0 | 1,4 | 1,2 | 1,0 | 1,0 | 0,7 | 1,7 | 1,2 | 1,6 | 1,8 | 0,8 | 0,7 |
| Cpa1 | 1,0 | 0,5 | 1,5 | 74,3 | 1,0 | 1,0 | 1,0 | 1,0 | 1,0 | 0,6 | 0,9 | 1,0 | 1,0 | 0,6 | 1,0 | 1,0 | 127,2 | 0,1 | 0,0 | 1,0 | 1,0 | 1,0 |
| Cpa2 | 1,0 | 1,0 | 1,5 | 12,4 | 1,2 | 1,4 | 1,0 | 1,0 | 1,0 | 1,0 | 1,0 | 1,0 | 1,0 | 1,0 | 1,0 | 1,0 | 32,3 | 0,0 | 0,1 | 1,0 | 1,0 | 1,0 |
| Cpb1 | 0,8 | 1,2 | 1,4 | 55,5 | 1,0 | 1,0 | 1,0 | 1,0 | 1,2 | 0,7 | 0,8 | 1,0 | 1,0 | 0,6 | 1,0 | 1,0 | 95,1 | 0,0 | 0,0 | 1,0 | 0,9 | 1,0 |
| Cpz | 1,0 | 1,0 | 1,0 | 1,0 | 1,0 | 0,8 | 1,0 | 1,0 | 1,0 | 1,0 | 1,0 | 1,0 | 1,0 | 1,0 | 1,1 | 1,1 | 1,0 | 1,0 | 1,0 | 1,0 | 1,0 | 1,0 |
| Crb3 | 1,1 | 1,0 | 1,1 | 1,2 | 0,7 | 1,0 | 1,0 | 1,0 | 0,9 | 1,5 | 0,8 | 0,8 | 2,0 | 1,0 | 5,4 | 2,2 | 1,0 | 0,9 | 1,1 | 1,4 | 1,1 | 1,0 |
| Crct1 | 1,0 | 1,0 | 1,0 | 1,0 | 3,2 | 1,8 | 1,0 | 1,0 | 1,0 | 1,0 | 1,0 | 1,0 | 2,3 | 0,9 | 1,0 | 1,0 | 1,0 | 0,9 | 1,0 | 1,0 | 1,0 | 1,0 |
| Crisp1 | 1,0 | 1,0 | 1,0 | 1,0 | 1,0 | 1,0 | 1,0 | 1,0 | 1,0 | 1,0 | 0,2 | 0,3 | 1,0 | 1,0 | 4,5 | 83,0 | 1,0 | 1,0 | 1,0 | 1,0 | 1,0 | 1,0 |
| Crisp4 | 1,0 | 1,0 | 1,0 | 1,0 | 1,0 | 1,0 | 1,0 | 1,0 | 1,0 | 1,0 | 1,0 | 1,0 | 1,0 | 1,0 | 19,3 | 1,0 | 1,0 | 1,0 | 1,0 | 1,0 | 1,0 | 1,0 |
| Cryaa | 0,4 | 0,7 | 3,2 | 1,0 | 1,0 | 1,1 | 1,1 | 1,4 | 1,0 | 1,0 | 1,0 | 1,0 | 0,7 | 4,1 | 0,9 | 5,0 | 1,0 | 0,4 | 1,0 | 1,0 | 1,0 | 1,0 |
| Cryba2 | 1,0 | 1,0 | 1,0 | 1,0 | 1,0 | 0,9 | 1,0 | 1,0 | 1,0 | 1,0 | 1,0 | 1,0 | 0,9 | 1,7 | 1,0 | 1,0 | 1,0 | 1,0 | 1,0 | 1,0 | 1,0 | 1,0 |
| Cryba4 | 1,0 | 1,0 | 1,4 | 1,0 | 1,0 | 1,1 | 0,6 | 1,0 | 1,0 | 1,0 | 1,0 | 1,0 | 1,0 | 1,0 | 67,6 | 1,0 | 1,0 | 1,0 | 1,0 | 0,7 | 1,0 | 1,0 |
| Crybb3 | 1,0 | 1,0 | 1,2 | 1,0 | 1,0 | 0,9 | 1,0 | 1,0 | 1,0 | 1,0 | 1,0 | 1,0 | 1,0 | 1,0 | 1,0 | 1,0 | 0,7 | 1,0 | 0,7 | 1,5 | 0,3 | 0,2 |
| Crygc | 1,0 | 1,0 | 1,6 | 1,0 | 1,0 | 1,1 | 1,0 | 0,7 | 1,0 | 1,0 | 1,0 | 1,0 | 1,0 | 1,0 | 1,0 | 5,9 | 1,0 | 0,9 | 1,0 | 1,0 | 1,0 | 1,0 |
| Csad | 0,7 | 0,6 | 1,0 | 1,4 | 1,0 | 1,1 | 1,2 | 0,8 | 11,0 | 16,0 | 1,1 | 0,8 | 1,4 | 1,2 | 0,8 | 1,2 | 1,2 | 1,0 | 0,9 | 1,0 | 1,0 | 0,9 |
| Csrp3 | 1,0 | 1,0 | 1,0 | 1,0 | 0,6 | 1,0 | 0,9 | 1,1 | 0,5 | 1,4 | 1,0 | 1,0 | 1,0 | 1,0 | 1,0 | 1,0 | 1,0 | 1,3 | 1,0 | 1,0 | 1,0 | 1,0 |
| Cst11 | 1,0 | 1,0 | 1,0 | 1,0 | 1,0 | 1,0 | 1,0 | 1,0 | 1,0 | 1,0 | 1,0 | 1,0 | 1,0 | 1,0 | 650,5 | 1,0 | 0,7 | 1,0 | 1,0 | 1,0 | 1,0 | 1,0 |
| Cst12 | 1,0 | 1,0 | 1,0 | 1,0 | 1,0 | 1,0 | 1,0 | 1,0 | 1,0 | 1,0 | 1,0 | 1,0 | 1,0 | 1,0 | 337,0 | 1,0 | 1,0 | 1,0 | 1,0 | 1,0 | 1,0 | 1,0 |
| Cst6 | 1,0 | 1,0 | 1,0 | 1,0 | 1,0 | 0,9 | 1,0 | 1,0 | 1,0 | 1,0 | 0,6 | 0,3 | 2,3 | 0,8 | 1,0 | 1,0 | 1,0 | 0,9 | 0,9 | 1,2 | 1,1 | 0,9 |
| Cst8 | 1,0 | 1,0 | 1,0 | 1,0 | 1,0 | 1,0 | 1,0 | 1,0 | 1,0 | 1,0 | 1,0 | 1,0 | 1,0 | 1,0 | 415,6 | 1,0 | 2,7 | 3,4 | 1,0 | 1,0 | 1,0 | 1,0 |
| Cthrc1 | 1,0 | 1,0 | 1,0 | 1,0 | 0,7 | 0,8 | 1,0 | 1,0 | 1,0 | 1,0 | 0,9 | 1,4 | 1,0 | 1,0 | 0,9 | 1,2 | 2,1 | 1,1 | 1,0 | 1,0 | 1,0 | 1,0 |
| Scgb1a1 | 0,9 | 1,2 | 1,9 | 533,0 | 1,0 | 1,0 | 1,0 | 1,0 | 0,9 | 0,7 | 0,5 | 1,0 | 1,0 | 0,1 | 1,0 | 1,0 | 203,6 | 0,1 | 0,0 | 0,3 | 1,1 | 1,0 |
| Ctrl | 1,0 | 0,7 | 1,0 | 34,8 | 1,0 | 1,0 | 1,0 | 1,0 | 1,0 | 0,5 | 0,9 | 1,0 | 1,0 | 0,4 | 1,0 | 1,0 | 77,6 | 0,0 | 0,0 | 1,0 | 1,1 | 1,0 |
| Ctxn3 | 0,6 | 0,2 | 1,2 | 1,0 | 0,9 | 1,2 | 1,0 | 1,0 | 1,0 | 1,0 | 1,0 | 1,0 | 1,0 | 1,0 | 0,6 | 1,0 | 1,3 | 1,1 | 1,3 | 1,1 | 0,9 | 1,1 |
| Cux2 | 1,0 | 1,0 | 1,0 | 1,0 | 1,1 | 0,8 | 0,8 | 0,9 | 13,2 | 8,3 | 1,0 | 1,0 | 1,0 | 1,0 | 1,0 | 1,0 | 1,0 | 1,0 | 1,0 | 1,0 | 1,0 | 1,0 |
| Cuzd1 | 1,0 | 1,0 | 1,6 | 1,7 | 1,0 | 1,0 | 1,0 | 1,0 | 1,0 | 1,0 | 1,0 | 1,0 | 1,0 | 1,0 | 7,3 | 6,0 | 5,1 | 0,2 | 0,5 | 1,0 | 1,0 | 1,0 |

[Fig. 23-19]

| | | | | | | | | | | | | | | | | | | | | | | |
|---|---|---|---|---|---|---|---|---|---|---|---|---|---|---|---|---|---|---|---|---|---|---|
| Cxcl1 | 4,5 | 7,1 | 1,0 | 1,0 | 1,0 | 1,0 | 2,3 | 1,0 | 0,5 | 1,1 | 1,0 | 1,0 | 1,0 | 1,0 | 0,4 | 0,3 | 1,0 | 1,0 | 1,0 | 1,0 | 1,0 | 1,0 |
| Cxcl13 | 1,0 | 1,0 | 1,2 | ,0,5 | 1,1 | 1,0 | 3,8 | 2,2 | 4,5 | 1,8 | 0,8 | 2,5 | 1,6 | 1,6 | 1,1 | 1,6 | 1,6 | 0,7 | 0,8 | 0,9 | 0,8 | 0,6 |
| Cxcr5 | 1,0 | 1,0 | 0,4 | 0,2 | 1,0 | 1,0 | 1,0 | 1,0 | 1,0 | 1,0 | 0,9 | 1,3 | 1,5 | 1,0 | 1,0 | 1,0 | 1,0 | 1,0 | 1,0 | 0,9 | 0,7 | 0,9 |
| Cyb561 | 0,9 | 1,0 | 1,2 | 1,0 | 1,0 | 1,0 | 1,3 | 1,0 | 0,7 | 1,2 | 1,1 | 0,9 | 1,5 | 1,3 | 5,8 | 1,2 | 1,1 | 1,0 | 1,1 | 1,3 | 1,1 | 1,0 |
| Cym | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 7,8 | 0,6 | 0,7 | 1,0 | 1,0 |
| Cyp1a1 | 1,0 | 1,0 | 1,2 | 1,5 | 1,0 | 1,0 | 4,3 | 3,1 | 1,6 | 1,3 | 1,0 | 1,0 | 1,0 | 1,0 | 3,0 | 3,4 | 0,8 | 0,8 | 2,4 | 1,4 | 0,3 | 1,8 |
| Cyp26b1 | 3,4 | 2,3 | 1,9 | 1,8 | 1,1 | 1,1 | 2,7 | 2,1 | 2,9 | 5,4 | 1,0 | 1,0 | 0,9 | 1,0 | 1,4 | 1,9 | 1,1 | 1,2 | 1,4 | 2,0 | 1,7 | 2,1 |
| Cyp27b1 | 10,8 | 27,2 | 1,0 | 1,0 | 1,0 | 1,0 | 1,0 | 1,0 | 1,0 | 1,0 | 1,0 | 1,0 | 1,0 | 1,0 | 1,0 | 1,0 | 0,9 | 1,1 | 1,0 | 1,0 | 1,0 | 1,0 |
| Cyp2a4 | 1,0 | 0,8 | 1,0 | 1,0 | 1,0 | 1,1 | 1,0 | 1,0 | 8,5 | 26,0 | 1,0 | 1,0 | 1,2 | 1,0 | 1,0 | 1,0 | 1,0 | 1,0 | 1,0 | 1,0 | 1,0 | 1,0 |
| Cyp2a5 | 2,2 | 2,0 | 1,0 | 1,0 | 0,8 | 1,5 | 1,0 | 1,0 | 2,5 | 4,1 | 1,9 | 1,4 | 2,5 | 1,4 | 1,0 | 1,0 | 7,5 | 0,5 | 1,0 | 1,0 | 1,0 | 1,0 |
| Cyp2b9 | 1,0 | 1,2 | 1,0 | 1,0 | 1,0 | 1,0 | 1,0 | 1,0 | 26,8 | 106,3 | 1,0 | 1,0 | 1,0 | 1,0 | 1,0 | 1,0 | 1,0 | 1,0 | 1,0 | 1,0 | 1,0 | 1,0 |
| Cyp2c38 | 1,0 | 1,0 | 1,0 | 1,0 | 1,0 | 1,0 | 1,0 | 1,0 | 4,0 | 7,2 | 1,0 | 1,0 | 1,0 | 1,0 | 1,0 | 1,0 | 1,0 | 1,0 | 1,0 | 1,0 | 1,0 | 1,0 |
| Cyp2c54 | 1,0 | 1,0 | 1,0 | 1,0 | 1,0 | 1,0 | 1,0 | 1,0 | 0,0 | 0,3 | 1,0 | 1,0 | 1,0 | 1,0 | 1,0 | 1,0 | 1,0 | 1,0 | 1,0 | 1,0 | 1,0 | 1,0 |
| Cyp2d12 | 0,3 | 0,2 | 1,9 | 2,1 | 1,0 | 1,0 | 1,0 | 1,0 | 0,0 | 0,1 | 1,0 | 1,0 | 1,0 | 1,0 | 1,0 | 1,0 | 1,0 | 1,0 | 1,0 | 1,0 | 1,0 | 1,0 |
| Cyp2d40 | 1,0 | 1,0 | 1,0 | 1,6 | 1,0 | 1,0 | 1,0 | 1,0 | 0,4 | 0,2 | 1,0 | 1,0 | 1,0 | 1,0 | 1,0 | 1,0 | 1,0 | 1,0 | 1,0 | 1,0 | 1,0 | 1,0 |
| Cyp2d9 | 1,9 | 1,1 | 1,1 | 1,4 | 1,0 | 1,0 | 1,0 | 1,0 | 0,0 | 0,1 | 1,0 | 1,0 | 1,0 | 1,0 | 0,9 | 1,0 | 1,2 | 0,2 | 1,0 | 1,0 | 1,0 | 1,0 |
| Cyp2e1 | 0,7 | 0,6 | 1,0 | 2,7 | 0,1 | 0,8 | 3,3 | 1,6 | 0,4 | 0,5 | 1,7 | 4,1 | 2,3 | 7,5 | 1,1 | 2,0 | 2,6 | 0,3 | 1,2 | 1,2 | 1,2 | 3,0 |
| Cyp2f2 | 0,8 | 0,6 | 3,4 | 2,3 | 0,8 | 0,9 | 1,0 | 1,0 | 0,3 | 0,6 | 1,6 | 1,3 | 3,1 | 1,5 | 1,4 | 3,0 | 1,0 | 0,9 | 1,0 | 1,0 | 1,0 | 1,0 |
| Cyp2u1 | 1,4 | 0,7 | 1,0 | 1,3 | 1,2 | 0,8 | 1,2 | 0,9 | 0,1 | 0,1 | 1,0 | 1,0 | 1,0 | 1,0 | 1,0 | 0,9 | 1,0 | 1,1 | 0,4 | 0,4 | 0,4 | 0,8 |
| Cyp39a1 | 0,9 | 1,3 | 1,1 | 1,0 | 1,0 | 1,2 | 1,0 | 0,9 | 2,5 | 6,5 | 1,2 | 0,9 | 1,0 | 1,5 | 1,1 | 1,3 | 0,9 | 0,9 | 1,2 | 0,7 | 1,0 | 1,3 |
| Cyp3a11 | 1,0 | 1,0 | 1,0 | 1,0 | 1,0 | 1,0 | 1,0 | 1,0 | 2,5 | 7,7 | 1,0 | 1,0 | 1,0 | 1,0 | 1,0 | 1,0 | 3,2 | 1,0 | 2,6 | 6,4 | 1,1 | 1,5 |
| Cyp3a44 | 1,0 | 1,0 | 1,0 | 1,0 | 1,0 | 1,0 | 1,0 | 1,0 | 3,6 | 5,6 | 1,0 | 1,0 | 1,0 | 1,0 | 1,0 | 1,0 | 1,0 | 1,0 | 1,3 | 2,0 | 1,0 | 1,2 |
| Cyp3a59 | 1,0 | 1,0 | 1,0 | 1,0 | 1,0 | 1,0 | 1,0 | 1,0 | 2,7 | 5,6 | 1,0 | 1,0 | 1,0 | 1,0 | 1,0 | 1,0 | 1,0 | 1,0 | 0,8 | 1,6 | 1,0 | 0,9 |
| Cyp4a12a | 0,1 | 0,1 | 1,0 | 1,0 | 1,0 | 1,0 | 1,0 | 1,0 | 0,0 | 0,0 | 1,0 | 1,0 | 1,0 | 1,0 | 1,0 | 1,0 | 1,0 | 1,0 | 1,0 | 1,0 | 1,0 | 1,0 |
| Cyp4a12b | 0,6 | 0,3 | 1,0 | -1,0 | 0,9 | 1,2 | 1,0 | 1,0 | 0,0 | 0,0 | 1,0 | 1,0 | 1,0 | 1,0 | 1,0 | 1,0 | 1,0 | 1,0 | 1,0 | 1,0 | 1,0 | 1,0 |
| Cyp4a14 | 28,9 | 42,1 | 1,0 | 1,0 | 1,0 | 1,0 | 1,0 | 1,0 | 7,8 | 51,2 | 1,0 | 1,0 | 1,0 | 1,0 | 1,0 | 1,0 | 4,1 | 1,0 | 1,0 | 1,0 | 1,0 | 1,0 |
| Cyp4b1-ps2 | 0,7 | 0,3 | 1,0 | 1,0 | 1,2 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Cyp7b1 | 1,0 | 0,9 | 1,0 | 1,0 | 1,2 | 1,4 | 1,0 | 1,0 | 0,1 | 0,3 | 1,0 | 1,0 | 1,4 | 1,6 | 1,0 | 1,0 | 1,4 | 0,7 | 0,7 | 0,9 | 1,1 | 1,5 |
| Cyp8b1 | 1,0 | 1,0 | 1,0 | 1,0 | 1,0 | 1,0 | 1,0 | 1,0 | 0,3 | 0,2 | 1,0 | 1,0 | 1,0 | 1,0 | 1,0 | 1,0 | 2,0 | 0,8 | 1,0 | 1,0 | 1,0 | 1,0 |
| Cystm1 | 1,0 | 0,8 | 1,1 | 1,0 | 0,9 | 0,6 | 1,0 | 1,0 | 2,0 | 1,9 | 1,0 | 0,8 | 2,6 | 0,9 | 2,4 | 1,4 | 1,1 | 1,0 | 1,2 | 1,3 | 1,0 | 1,0 |
| Dbil5 | 0,7 | 1,0 | 1,0 | 0,8 | 0,6 | 1,2 | 0,7 | 1,0 | 1,0 | 1,0 | 1,0 | 1,0 | 1,0 | 0,9 | 12,1 | 3,5 | 0,6 | 1,0 | 1,0 | 1,0 | 1,3 | 0,9 |
| Dcpp2 | 0,7 | 1,0 | 1,0 | 1,0 | 1,0 | 0,6 | 1,0 | 0,9 | 0,9 | 1,0 | 1,0 | 1,2 | 1,0 | 1,8 | 1,0 | 1,8 | 1,0 | 1,3 | 1,0 | 1,0 | 1,0 | 1,0 |
| Dcpp3 | 1,1 | 1,0 | 1,0 | 1,0 | 1,0 | 0,5 | 1,0 | 0,6 | 0,7 | 1,0 | 1,0 | 1,1 | 1,0 | 2,1 | 1,0 | 2,1 | 1,0 | 0,5 | 1,0 | 1,0 | 1,0 | 1,0 |
| Dcstamp | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Dct | 1,0 | 1,0 | 1,0 | 1,0 | 0,9 | 1,0 | 0,7 | 0,9 | 0,3 | 0,3 | 1,0 | 1,0 | 1,0 | 1,0 | 0,7 | 1,2 | 1,0 | 1,0 | 1,0 | 1,0 | 1,0 | 1,0 |
| Scgb1b27 | 1,3 | 0,5 | 1,1 | 0,9 | 1,1 | 0,5 | 0,8 | 0,9 | 1,8 | 0,5 | 1,0 | 2,4 | 1,0 | 0,7 | 1,3 | 0,9 | 0,7 | 0,9 | 0,7 | 0,5 | 1,0 | 2,4 |
| Ddit4l | 1,7 | 1,8 | 1,0 | 1,0 | 0,7 | 0,7 | 0,9 | 0,8 | 1,0 | 1,0 | 1,1 | 1,0 | 1,6 | 1,6 | 11,7 | 1,6 | 0,9 | 0,9 | 1,0 | 1,0 | 1,0 | 1,0 |
| Defa24 | 1,0 | 1,0 | 1,0 | 1,1 | 1,0 | 1,0 | 1,0 | 1,0 | 1,0 | 1,0 | 1,0 | 1,0 | 1,0 | 1,0 | 1,0 | 0,1 | 2,5 | 1,0 | 0,5 | 1,0 | 1,0 | 0,7 |
| Defb1 | 0,8 | 1,0 | 1,0 | 1,0 | 1,3 | 1,3 | 1,0 | 1,0 | 1,3 | 3,2 | 1,0 | 1,0 | 2,7 | 1,9 | 15,2 | 1,0 | 1,1 | 1,5 | 1,0 | 1,0 | 0,7 | 1,3 |
| Defb10 | 1,0 | 1,0 | 1,0 | 1,0 | 1,0 | 1,0 | 1,0 | 1,0 | 1,0 | 1,0 | 1,0 | 1,0 | 1,0 | 1,0 | 9,3 | 1,0 | 1,0 | 1,0 | 1,0 | 1,0 | 1,0 | 1,0 |
| Defb12 | 1,0 | 1,0 | 1,0 | 1,0 | 1,0 | 1,0 | 1,0 | 1,0 | 1,0 | 1,0 | 1,0 | 1,0 | 1,0 | 1,0 | 136,1 | 1,0 | 1,0 | 1,0 | 1,0 | 1,0 | 1,0 | 1,0 |
| Defb14 | 1,0 | 1,0 | 1,0 | 1,0 | 1,0 | 1,0 | 1,0 | 1,0 | 1,0 | 1,0 | 1,0 | 1,0 | 1,0 | 1,0 | 4,6 | 1,0 | 1,3 | 1,0 | 1,0 | 1,0 | 1,0 | 1,0 |
| Defb15 | 1,0 | 1,0 | 1,0 | 1,0 | 1,0 | 0,9 | 1,0 | 1,0 | 1,0 | 1,0 | 1,0 | 1,0 | 1,0 | 1,0 | 468,1 | 1,0 | 1,0 | 1,0 | 1,0 | 1,0 | 1,0 | 1,0 |
| Defb18 | 1,0 | 1,0 | 1,0 | 1,0 | 1,0 | 1,0 | 1,0 | 1,0 | 1,0 | 1,0 | 1,0 | 1,0 | 1,0 | 1,0 | 172,1 | 1,0 | 1,0 | 1,0 | 1,0 | 1,0 | 1,0 | 1,0 |
| Defb19 | 0,2 | 0,3 | 1,0 | 1,0 | 1,0 | 1,0 | 1,0 | 1,0 | 1,0 | 1,0 | 1,0 | 1,0 | 1,0 | 1,0 | 18,1 | 1,0 | 1,0 | 1,0 | 1,0 | 1,0 | 1,0 | 1,0 |
| | Kidney | | Colon | | Eyeball | | Heart | | Liver | | SG | | Thymus | | Adipose | | Stomach | | Jejunum | | Ileum | |
| | E | M | E | M | E | M | E | M | E | M | E | M | E | M | E | M | E | M | E | M | E | M |
| Defb2 | 1,1 | 2,0 | 1,0 | -1,0 | 1,5 | 0,4 | 1,0 | 1,0 | 1,0 | 1,0 | 1,0 | 1,0 | 1,0 | 1,0 | 7,2 | 1,4 | 1,0 | 1,0 | 1,0 | 1,0 | 1,0 | 1,0 |
| Defb20 | 1,0 | 1,0 | 1,0 | 1,0 | 1,0 | 1,0 | 1,0 | 1,0 | 1,0 | 1,0 | 1,0 | 1,0 | 1,0 | 1,0 | 1319 | 1,0 | 1,0 | 1,0 | 1,0 | 1,0 | 1,0 | 1,0 |
| Defb21 | 1,0 | 1,0 | 1,0 | 1,0 | 1,0 | 1,0 | 1,0 | 1,0 | 1,0 | 1,0 | 1,0 | 1,0 | 1,0 | 1,0 | 136,6 | 1,0 | 1,0 | 1,0 | 1,0 | 1,0 | 1,0 | 1,0 |
| Defb22 | 1,0 | 1,0 | 1,0 | 1,0 | 1,0 | 1,0 | 1,0 | 1,0 | 1,0 | 1,0 | 1,0 | 1,0 | 1,0 | 1,0 | 6,4 | 1,0 | 1,0 | 1,0 | 1,0 | 1,0 | 1,0 | 1,0 |
| Defb23 | 1,0 | 1,0 | 1,0 | 1,0 | 1,0 | 1,0 | 1,0 | 1,0 | 1,0 | 1,0 | 1,0 | 1,0 | 1,0 | 1,0 | 75,7 | 1,0 | 1,0 | 1,0 | 1,0 | 1,0 | 1,0 | 1,0 |
| Defb25 | 1,0 | 1,0 | 1,0 | 1,0 | 1,0 | 1,0 | 1,0 | 1,0 | 1,0 | 1,0 | 1,0 | 1,0 | 1,0 | 1,4 | 849,2 | 1,0 | 1,0 | 1,0 | 1,0 | 1,0 | 1,0 | 1,0 |
| Defb26 | 1,0 | 1,0 | 1,0 | 1,0 | 1,0 | 1,0 | 1,0 | 1,0 | 1,0 | 1,0 | 1,0 | 1,0 | 1,0 | 1,0 | 7,2 | 1,0 | 1,0 | 1,0 | 1,0 | 1,0 | 1,0 | 1,0 |
| Defb28 | 1,0 | 1,0 | 1,0 | 1,0 | 1,0 | 1,0 | 1,0 | 1,0 | 1,0 | 1,0 | 1,0 | 1,0 | 1,0 | 1,0 | 5,4 | 1,0 | 1,0 | 1,0 | 1,0 | 1,0 | 1,0 | 1,0 |
| Defb29 | 0,3 | 0,2 | 1,0 | 1,0 | 1,0 | 1,0 | 1,0 | 1,0 | 1,0 | 1,0 | 1,0 | 1,0 | 1,0 | 1,0 | 77,5 | 2,0 | 1,0 | 1,0 | 1,0 | 1,0 | 1,0 | 1,0 |
| Defb30 | 1,0 | 1,0 | 1,0 | 1,0 | 1,0 | 1,0 | 1,0 | 1,0 | 1,0 | 1,0 | 1,0 | 1,0 | 1,0 | 1,0 | 239,9 | 1,0 | 1,0 | 1,0 | 1,0 | 1,0 | 1,0 | 1,0 |
| Defb35 | 1,0 | 1,0 | 1,0 | 1,0 | 1,2 | 1,0 | 1,0 | 1,0 | 1,0 | 1,0 | 1,0 | 1,0 | 1,0 | 1,0 | 6,0 | 1,0 | 1,0 | 1,0 | 1,0 | 1,0 | 1,0 | 1,0 |
| Defb37 | 1,0 | 1,0 | 0,8 | 0,6 | 1,0 | 1,0 | 1,0 | 1,0 | 1,0 | 1,0 | 1,0 | 1,0 | 1,0 | 1,0 | 7,0 | 1,0 | 1,0 | 1,0 | 1,0 | 1,0 | 0,4 | 0,2 |
| Defb39 | 1,0 | 1,0 | 1,0 | 1,0 | 1,0 | 1,0 | 1,0 | 1,0 | 1,0 | 1,0 | 1,0 | 1,0 | 1,0 | 1,0 | 28,2 | 1,0 | 1,0 | 1,0 | 1,0 | 1,0 | 1,0 | 1,0 |
| Defb4 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,7 | 1,8 | 1,0 | 1,0 | 1,0 | 1,0 |
| Defb41 | 1,0 | 1,0 | 1,0 | 1,0 | 1,0 | 1,0 | 1,0 | 1,0 | 1,0 | 1,0 | 1,0 | 1,0 | 1,0 | 1,0 | 102,0 | 1,0 | 1,0 | 1,0 | 1,0 | 1,0 | 1,0 | 1,0 |
| Defb42 | 1,1 | 2,4 | 1,0 | 1,0 | 1,0 | 1,0 | 1,0 | 1,0 | 1,0 | 1,0 | 1,0 | 1,0 | 0,9 | 1,0 | 97,7 | 22,9 | 1,0 | 1,0 | 1,0 | 1,0 | 1,0 | 1,0 |
| Defb43 | 1,0 | 1,0 | 1,0 | 1,0 | 1,0 | 1,0 | 1,0 | 1,0 | 1,0 | 1,0 | 1,0 | 1,0 | 1,0 | 1,0 | 4,1 | 5,8 | 1,0 | 1,0 | 1,0 | 1,0 | 1,0 | 1,0 |
| Defb45 | 1,0 | 1,0 | 1,0 | 1,1 | 1,0 | 1,0 | 1,0 | 1,0 | 1,0 | 1,0 | 1,0 | 1,0 | 1,0 | 1,0 | 26,0 | 1,0 | 1,0 | 1,0 | 1,0 | 1,0 | 1,0 | 1,0 |
| Defb47 | 1,0 | 1,0 | 1,0 | 1,0 | 1,0 | 1,0 | 1,0 | 1,0 | 1,0 | 1,0 | 1,0 | 1,0 | 1,0 | 1,0 | 454,2 | 1,0 | 1,0 | 1,0 | 1,0 | 1,0 | 1,0 | 1,0 |
| Defb48 | 1,0 | 1,0 | 1,0 | 1,0 | 1,0 | 1,0 | 1,0 | 1,0 | 1,0 | 1,0 | 1,0 | 1,0 | 1,0 | 1,0 | 1255 | 1,0 | 1,0 | 1,0 | 1,0 | 1,0 | 1,0 | 1,0 |
| Defb8 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Depdc7 | 0,8 | 0,7 | 1,0 | 1,0 | 1,0 | 1,1 | 1,0 | 1,0 | 0,9 | 1,2 | 1,0 | 1,0 | 1,0 | 1,0 | 5,6 | 1,0 | 1,3 | 1,0 | 0,9 | 1,0 | 1,0 | 1,0 |
| Derl3 | 0,8 | 1,2 | 1,3 | 0,6 | 1,0 | 1,0 | 1,0 | 1,0 | 0,6 | 2,0 | 1,1 | 1,1 | 2,1 | 1,9 | 32,1 | 1,4 | 1,1 | 1,1 | 1,5 | 1,3 | 1,0 | 1,2 |
| Des | 0,9 | 1,1 | 1,1 | -0,9 | 0,7 | 0,8 | 1,2 | 1,3 | 1,0 | 1,4 | 1,7 | 2,2 | 1,2 | 1,2 | 1,0 | 1,6 | 1,7 | 1,2 | 1,4 | 1,1 | 0,9 | 0,9 |
| Dhrs7c | 1,0 | 1,0 | 1,0 | 1,0 | 0,5 | 0,5 | 1,0 | 1,1 | 1,0 | 1,0 | 1,0 | 1,0 | 1,0 | 1,0 | 1,0 | 1,0 | 1,0 | 1,0 | 1,0 | 1,0 | 1,0 | 1,0 |
| Dio1 | 0,3 | 0,4 | 0,7 | 0,8 | 1,0 | 1,0 | 1,0 | 1,0 | 0,0 | 0,1 | 1,1 | 1,2 | 1,3 | 0,9 | 0,7 | 1,0 | 0,5 | 0,6 | 0,6 | 0,6 | 0,7 | 0,8 |
| Dmbt1 | 1,0 | 1,0 | 1,1 | 1,0 | 1,0 | 1,0 | 1,0 | 1,0 | 1,0 | 1,0 | 1,0 | 1,0 | 1,0 | 1,0 | 1,0 | 1,0 | 2,2 | 1,1 | 1,8 | 0,8 | 1,4 | 1,2 |
| Dmkn | 1,0 | 1,0 | 1,0 | 1,0 | 1,0 | 1,1 | 1,0 | 1,0 | 1,0 | 1,0 | 1,0 | 1,0 | 1,9 | 1,0 | 1,3 | 1,6 | 1,0 | 1,0 | 1,0 | 1,0 | 1,0 | 0,8 |
| Dmrtc1a | 1,0 | 1,0 | 1,0 | 1,0 | 1,0 | 1,2 | 1,0 | 1,0 | 1,0 | 1,0 | 1,0 | 1,0 | 2,1 | 1,7 | 7,3 | 1,0 | 1,0 | 1,0 | 1,0 | 1,0 | 1,0 | 1,0 |
| Dmtn | 0,9 | 0,8 | 0,8 | 1,0 | 1,0 | 1,0 | 0,9 | 0,9 | 1,0 | 1,0 | 1,0 | 1,0 | 1,4 | 2,2 | 0,6 | 0,9 | 0,8 | 1,0 | 1,0 | 1,0 | 1,0 | 1,0 |
| Dnajb8 | 1,0 | 1,0 | 1,0 | 1,0 | 1,0 | 1,0 | 1,0 | 1,0 | 1,0 | 1,0 | 1,0 | 1,0 | 1,0 | 1,0 | 5,8 | 1,0 | 1,0 | 1,0 | 1,0 | 1,0 | 1,0 | 1,0 |
| Dnase1 | 0,1 | 0,1 | 1,0 | 1,0 | 1,0 | 1,0 | 1,0 | 1,0 | 1,0 | 1,0 | 2,4 | 5,4 | 1,0 | 1,3 | 1,0 | 1,0 | 1,0 | 1,0 | 0,7 | 0,5 | 0,6 | 0,7 |
| Dnmt3l | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Dntt | 1,0 | 1,0 | 1,0 | 1,0 | 1,0 | 1,0 | 1,0 | 1,0 | 0,9 | 0,5 | 1,0 | 1,0 | 0,6 | 0,7 | 1,0 | 1,0 | 1,0 | 1,0 | 1,0 | 1,0 | 1,0 | 1,0 |
| Dopey2 | 1,6 | 1,0 | 1,0 | 1,2 | 1,1 | 0,8 | 1,0 | 0,9 | 1,9 | 1,4 | 1,0 | 1,3 | 1,1 | 1,1 | 1,1 | 0,7 | 0,8 | 0,9 | 0,9 | 0,8 | 1,1 | 1,4 |
| Drd4 | 1,0 | 1,0 | 1,0 | 1,0 | 1,0 | 0,9 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Duoxa2 | 1,0 | 1,0 | 1,3 | 0,3 | 1,0 | 1,0 | 1,0 | 1,0 | 1,0 | 1,0 | 1,0 | 0,8 | 1,0 | 1,0 | 1,0 | 1,0 | 1,9 | 1,4 | 7,4 | 5,3 | 0,5 | 0,7 |

[Fig. 23-20]

289/310

| | | | | | | | | | | | | | | | | | | | | | | |
|---|---|---|---|---|---|---|---|---|---|---|---|---|---|---|---|---|---|---|---|---|---|---|
| Dupd1 | 1,0 | 1,0 | 1,0 | 1,0 | 0,5 | 0,4 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Dusp10 | 1,0 | 1,0 | 1,0 | 1,1 | 0,8 | 0,9 | 1,2 | 0,8 | 1,2 | 1,2 | 0,9 | 1,0 | 0,8 | 1,0 | 0,5 | 0,2 | 0,8 | 1,0 | 1,1 | 1,3 | 1,0 | 1,0 |
| Dusp13 | 1,0 | 1,0 | 1,0 | 1,0 | 0,5 | 0,7 | 1,0 | 1,0 | 1,0 | 1,0 | 1,0 | 1,0 | 1,0 | 1,0 | 1,0 | 1,0 | 1,0 | 0,9 | 1,0 | 1,0 | 1,0 | 1,0 |
| Dusp15 | 1,5 | 0,7 | 1,0 | 1,0 | 0,9 | 0,8 | 1,0 | 1,0 | 1,0 | 1,0 | 1,0 | 1,0 | 1,8 | 1,6 | 8,2 | 1,0 | 1,0 | 1,0 | 1,0 | 1,0 | 1,0 | 1,0 |
| Dusp2 | 0,8 | 1,0 | 0,6 | 0,2 | 1,0 | 1,0 | 1,0 | 1,0 | 1,0 | 1,0 | 0,9 | 1,7 | 1,3 | 1,3 | 1,0 | 1,0 | 1,0 | 1,7 | 0,9 | 1,1 | 0,8 | 0,8 |
| *Dusp5* | 2,3 | 1,9 | 1,1 | 1,1 | 1,1 | 0,7 | 2,2 | 0,7 | 1,0 | 1,0 | 2,1 | 1,4 | 1,4 | 1,0 | 5,9 | 1,2 | 1,0 | 0,8 | 1,2 | 0,8 | 1,3 | 1,9 |
| Ear3 | 1,0 | 2,2 | 1,0 | 1,0 | 1,0 | 1,0 | 0,8 | 3,5 | 2,9 | 1,0 | 1,0 | 1,0 | 1,0 | 1,6 | 1,3 | 0,2 | 1,3 | 2,3 | 1,0 | 1,0 | 1,0 | 1,0 |
| Eddm3b | 0,7 | 1,0 | 1,0 | 1,0 | 1,0 | 1,0 | 1,0 | 1,0 | 1,0 | 1,0 | 1,0 | 1,0 | 1,0 | 1,0 | 19,9 | 2,9 | 1,0 | 1,0 | 1,0 | 1,0 | 1,0 | 1,0 |
| *Eef1a2* | *1,0* | *1,0* | *0,8* | *1,1* | *0,9* | *0,7* | *1,0* | *1,1* | *1,0* | *1,0* | *1,0* | *1,0* | *1,0* | *1,0* | *1,0* | *1,0* | *0,5* | *0,9* | *1,1* | *1,0* | *1,1* | *1,1* |
| Egfr | 1,0 | 0,9 | 1,2 | 0,9 | 1,0 | 1,1 | 0,7 | 0,8 | 0,4 | 0,2 | 1,3 | 1,3 | 1,4 | 1,5 | 0,6 | 0,6 | 0,7 | 0,8 | 0,9 | 0,8 | 1,0 | 1,3 |
| Egr2 | 1,0 | 2,1 | 0,8 | 1,0 | 1,0 | 1,0 | 1,0 | 1,0 | 1,0 | 1,0 | 1,0 | 1,0 | 1,2 | 0,7 | 15,4 | 0,4 | 1,1 | 2,1 | 1,0 | 1,0 | 1,0 | 0,9 |
| Eif3j1 | 1,0 | 0,1 | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* | *0,0* |
| Eif3j2 | 0,9 | 2,3 | 1,0 | 0,9 | 1,2 | 1,1 | 1,0 | 1,0 | 1,0 | 1,2 | 0,9 | 0,9 | 0,7 | 0,9 | 0,9 | 1,0 | 1,0 | 1,0 | 0,9 | 1,1 | 1,0 | 17,1 |
| Elk4 | 1,7 | 0,9 | 0,9 | 1,1 | 1,2 | 0,7 | 0,8 | 0,7 | 3,0 | 0,5 | 0,8 | 1,6 | 0,2 | 0,7 | 0,5 | 0,4 | 0,7 | 1,0 | 0,7 | 0,4 | 1,1 | 2,4 |
| Ell3 | 0,5 | 0,5 | 1,0 | 0,7 | 0,6 | 1,2 | 1,0 | 1,0 | 0,8 | 1,5 | 1,0 | 1,1 | 1,0 | 1,0 | 5,0 | 1,0 | 1,1 | 0,9 | 0,7 | 1,0 | 1,0 | 0,7 |
| Elovl2 | 1,8 | 2,5 | 1,0 | 1,0 | 1,1 | 1,2 | 1,0 | 1,0 | 0,8 | 0,6 | 1,0 | 1,0 | 1,0 | 1,0 | 8,1 | 1,0 | 1,5 | 0,8 | 1,0 | 1,0 | 1,0 | 1,0 |
| Elovl3 | 1,0 | 1,0 | 1,0 | 1,0 | 1,0 | 1,0 | 1,0 | 1,0 | 0,1 | 0,0 | 1,0 | 1,0 | 1,0 | 1,0 | 1,0 | 1,0 | 1,0 | 1,0 | 1,0 | 1,0 | 1,0 | 1,0 |
| Elovl4 | 1,0 | 1,0 | 1,0 | 1,0 | 1,0 | 1,0 | 1,0 | 1,0 | 1,0 | 1,0 | 1,0 | 1,0 | 2,1 | 1,0 | 5,5 | 1,0 | 0,9 | 1,0 | 1,0 | 1,0 | 1,0 | 1,0 |
| Emid1 | 0,5 | 0,9 | 1,1 | 0,7 | 0,7 | 0,9 | 1,0 | 0,8 | 1,0 | 1,0 | 1,0 | 0,8 | 0,9 | 1,1 | 14,2 | 1,0 | 1,4 | 1,4 | 1,1 | 1,2 | 1,1 | 0,6 |
| Emp1 | 1,0 | 1,7 | 1,2 | 1,3 | 1,1 | 1,3 | 0,8 | 0,8 | 0,8 | 0,9 | 0,4 | 0,6 | 0,7 | 0,9 | 0,2 | 0,2 | 1,1 | 1,0 | 1,2 | 1,4 | 1,6 | 1,6 |
| Emp2 | 1,0 | 1,2 | 1,0 | 1,0 | 0,9 | 1,1 | 0,8 | 0,9 | 2,6 | 5,2 | 0,9 | 0,9 | 1,6 | 1,5 | 0,8 | 1,0 | 0,9 | 0,8 | 1,2 | 0,6 | 1,0 | 1,0 |
| Emx2 | 1,2 | 0,9 | 1,0 | 1,0 | 1,0 | 1,1 | 1,0 | 1,0 | 1,0 | 1,0 | 1,0 | 1,0 | 1,0 | 1,0 | 5,3 | 2,6 | 1,0 | 1,0 | 1,0 | 1,0 | 1,0 | 1,0 |
| Eno3 | 0,8 | 1,3 | 1,3 | 1,2 | 0,7 | 0,7 | 1,0 | 1,0 | 1,2 | 0,8 | 1,8 | 1,5 | 1,3 | 1,2 | 1,1 | 1,7 | 0,7 | 0,9 | 0,5 | 1,1 | 0,7 | 1,0 |
| Enpp1 | 0,9 | 1,0 | 1,0 | 1,0 | 1,0 | 1,0 | 1,0 | 0,8 | 1,8 | 2,1 | 1,0 | 0,9 | 1,2 | 1,0 | 13,7 | 1,0 | 1,0 | 1,1 | 1,6 | 1,4 | 0,8 | 1,3 |
| Epb4.1l4b | 1,0 | 1,0 | 1,0 | 1,0 | 1,2 | 1,3 | 1,0 | 1,0 | 0,8 | 1,1 | 1,2 | 1,1 | 1,3 | 1,3 | 5,3 | 1,7 | 1,0 | 0,8 | 1,0 | 1,2 | 1,0 | 1,0 |
| Epb4.2 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Epcam | 1,1 | 1,2 | 1,0 | 1,0 | 0,9 | 1,1 | 1,0 | 1,0 | 0,7 | 1,0 | 1,0 | 0,9 | 1,8 | 1,4 | 4,6 | 7,1 | 1,1 | 1,1 | 1,1 | 1,3 | 1,0 | 0,9 |
| Eppin | 1,0 | 1,0 | 1,0 | 1,0 | 1,0 | 1,0 | 1,0 | 1,0 | 1,0 | 1,0 | 1,0 | 1,0 | 1,0 | 1,0 | 17,3 | 1,0 | 1,0 | 1,0 | 1,0 | 1,0 | 1,0 | 1,0 |
| Ermap | 1,0 | 1,0 | 1,0 | 1,0 | 0,7 | 0,8 | 1,0 | 1,0 | 1,0 | 1,0 | 1,0 | 1,0 | 1,2 | 1,0 | 1,0 | 1,0 | 1,0 | 1,0 | 1,0 | 1,0 | 1,0 | 1,0 |
| Esco2 | 1,0 | 1,0 | 0,7 | 0,7 | 1,0 | 1,0 | 1,0 | 1,0 | 1,0 | 1,0 | 1,0 | 1,0 | 0,4 | 0,7 | 1,0 | 1,0 | 1,1 | 0,9 | 1,1 | 1,1 | 0,8 | 0,8 |
| Esrp1 | 1,2 | 1,4 | 1,1 | 1,1 | 1,1 | 1,3 | 1,0 | 1,0 | 1,0 | 1,0 | 1,1 | 1,0 | 1,5 | 1,4 | 7,6 | 1,3 | 1,0 | 1,0 | 1,1 | 1,2 | 1,2 | 1,1 |
| *Esrp2* | 1,0 | 0,7 | 1,1 | 1,0 | 0,9 | 0,9 | 1,0 | 1,0 | 1,3 | 0,8 | 1,0 | 1,2 | 1,2 | 1,4 | 9,4 | 1,0 | 0,9 | 1,0 | 1,1 | 0,7 | 0,9 | 1,4 |
| Etv4 | 3,0 | 3,2 | 1,4 | 0,9 | 0,8 | 0,9 | 1,0 | 1,0 | 1,0 | 1,0 | 1,0 | 1,0 | 1,0 | 1,0 | 8,9 | 1,0 | 1,2 | 1,1 | 1,0 | 1,7 | 1,0 | 1,0 |
| Etv5 | 1,4 | 1,5 | 1,2 | 1,2 | 1,0 | 0,9 | 1,7 | 1,3 | 0,7 | 0,6 | 0,7 | 0,9 | 1,2 | 1,5 | 8,8 | 1,4 | 1,1 | 1,1 | 1,0 | 1,4 | 1,2 | 1,5 |
| *F11* | *1,0* | *1,0* | *1,0* | *1,0* | *1,0* | *1,0* | *1,0* | *1,0* | *0,3* | *0,3* | *1,0* | *1,0* | *1,0* | *1,0* | *1,0* | *1,0* | *1,0* | *1,0* | *1,0* | *1,0* | *1,0* | *1,0* |
| Faah | 1,1 | 0,9 | 1,2 | 1,0 | 0,9 | 1,0 | 1,0 | 1,0 | 0,9 | 1,1 | 1,1 | 0,9 | 1,1 | 0,9 | 5,6 | 1,4 | 1,0 | 1,1 | 0,9 | 1,0 | 1,0 | 1,0 |
| Fabp1 | 3,4 | 6,6 | 0,1 | 2,6 | 1,0 | 1,0 | 1,0 | 1,0 | 0,5 | 0,6 | 1,0 | 1,0 | 1,0 | 0,5 | 0,3 | 1,0 | 6,2 | 0,2 | 0,6 | 0,7 | 0,5 | 0,8 |
| Fabp4 | *0,7* | *1,2* | *0,8* | *1,3* | *0,3* | *0,7* | *0,9* | *1,2* | *0,8* | *0,9* | *0,9* | *2,6* | *1,4* | *3,1* | *0,6* | *1,6* | *0,9* | *1,0* | *0,7* | *1,3* | *0,5* | *1,4* |
| Fabp5 | 0,9 | 1,4 | 1,0 | 0,6 | 1,1 | 1,2 | 0,7 | 0,6 | 0,5 | 0,3 | 1,1 | 0,7 | 0,7 | 1,0 | 0,9 | 1,3 | 1,1 | 1,2 | 1,0 | 0,7 | 0,9 | 0,8 |
| Fads2 | 1,2 | 0,9 | 1,0 | 1,0 | 1,0 | 0,8 | 1,4 | 0,7 | 0,7 | 0,3 | 1,0 | 1,5 | 0,7 | 0,7 | 3,7 | 2,1 | 1,0 | 0,6 | 1,2 | 0,6 | 1,0 | 1,1 |
| Faim2 | 1,0 | 1,0 | 0,9 | 1,2 | 1,0 | 1,0 | 1,0 | 1,0 | 1,0 | 1,0 | 0,6 | 0,4 | 1,0 | 1,0 | 5,6 | 1,0 | 1,0 | 1,0 | 1,0 | 1,0 | 1,0 | 1,0 |
| Faim3 | 1,0 | 1,0 | 0,1 | 0,3 | 1,0 | 1,0 | 1,0 | 1,0 | 1,0 | 1,0 | 0,8 | 3,1 | 2,0 | 1,7 | 1,0 | 1,0 | 1,0 | 1,0 | 0,9 | 0,6 | 0,7 | 0,8 |
| Fam150b | 1,0 | 1,0 | 1,0 | 1,0 | 1,0 | 1,0 | 1,0 | 1,0 | 1,0 | 1,0 | 1,0 | 1,0 | 1,0 | 1,0 | 0,3 | 0,7 | 1,0 | 1,0 | 1,0 | 1,0 | 1,0 | 1,0 |
| Fam198a | 1,0 | 1,0 | 1,0 | 1,0 | 1,1 | 0,7 | 0,8 | 1,0 | 0,9 | 0,3 | 1,0 | 1,0 | 1,0 | 1,0 | 1,1 | 1,0 | 1,1 | 1,0 | 1,0 | 1,0 | 1,0 | 1,0 |
| Fam20a | 2,0 | 2,9 | 1,0 | 0,8 | 0,9 | 0,7 | 1,5 | 1,0 | 0,7 | 1,0 | 0,8 | 0,7 | 1,7 | 1,5 | 0,8 | 1,2 | 0,9 | 0,9 | 1,2 | 1,2 | 1,1 | 1,1 |
| Fam20c | 1,0 | 0,7 | 0,6 | 0,7 | 0,9 | 0,8 | 0,9 | 0,9 | 0,9 | 0,5 | 0,8 | 1,4 | 2,2 | 1,1 | 0,4 | 0,2 | 0,9 | 1,2 | 1,0 | 0,9 | 0,7 | 0,8 |
| Fam25c | 4,1 | 3,9 | 1,0 | 1,0 | 1,3 | 1,0 | 1,0 | 1,0 | 1,1 | 2,1 | 2,5 | 0,3 | 1,5 | 0,9 | 1,0 | 1,0 | 1,0 | 0,9 | 1,0 | 1,0 | 1,0 | 1,0 |
| Fam57b | 1,0 | 0,8 | 0,9 | 1,3 | 0,9 | 1,0 | 1,0 | 0,7 | 1,0 | 1,0 | 1,0 | 1,0 | 1,0 | 1,0 | 1,1 | 1,2 | 1,0 | 1,2 | 1,2 | 1,0 | 0,7 | 1,2 |
| Fam64a | 1,0 | 1,0 | 0,6 | 0,6 | 1,0 | 1,0 | 1,0 | 1,0 | 1,0 | 1,0 | 1,0 | 1,0 | 0,5 | 0,7 | 0,6 | 1,0 | 1,3 | 1,0 | 1,0 | 1,0 | 0,9 | 0,7 |
| Fam84a | 1,1 | 1,0 | 0,6 | 0,7 | 1,0 | 1,1 | 1,1 | 1,0 | 1,0 | 1,0 | 1,0 | 1,0 | 2,0 | 1,2 | 11,3 | 0,5 | 0,9 | 0,8 | 1,3 | 1,5 | 1,0 | 1,4 |
| Fbxo40 | 0,5 | 0,3 | 1,0 | 1,0 | 0,7 | 0,5 | 1,0 | 0,9 | 1,0 | 1,0 | 1,0 | 1,0 | 1,0 | 1,0 | 1,0 | 1,0 | 1,0 | 1,0 | 1,0 | 1,0 | 1,0 | 1,0 |
| Fcamr | 0,4 | 0,2 | 1,0 | 0,7 | 1,0 | 1,0 | 1,0 | 1,0 | 1,0 | 1,0 | 1,0 | 0,9 | 1,0 | 1,0 | 0,7 | 1,3 | 1,0 | 1,0 | 1,3 | 0,7 | 1,2 | 1,9 |
| Fcer2a | 1,0 | 1,0 | 0,3 | 0,3 | 1,0 | 1,0 | 1,0 | 1,0 | 1,0 | 1,0 | 0,6 | 2,3 | 1,2 | 1,3 | 1,0 | 1,0 | 1,0 | 1,0 | 0,9 | 0,8 | 0,5 | 0,7 |
| Fcgbp | 0,7 | 0,6 | 0,5 | 0,5 | 1,1 | 0,8 | 1,0 | 1,0 | 1,0 | 1,0 | 1,0 | 1,0 | 2,0 | 1,2 | 1,0 | 1,0 | 0,4 | 0,2 | 1,1 | 1,0 | 1,1 | 1,3 |
| Fcna | 1,0 | 1,0 | 1,0 | 0,9 | 1,0 | 1,4 | 1,7 | 1,2 | 0,9 | 1,2 | 1,0 | 1,0 | 1,3 | 1,2 | 0,9 | 1,2 | 2,0 | 1,8 | 1,0 | 1,0 | 0,9 | 1,0 |
| Fcrla | 1,0 | 1,0 | 0,4 | 0,2 | 1,0 | 1,0 | 1,0 | 1,0 | 1,0 | 1,0 | 1,0 | 1,2 | 1,1 | 0,7 | 0,8 | 1,0 | 1,0 | 1,0 | 1,0 | 0,9 | 0,6 | 0,6 |
| Fga | 5,3 | 6,4 | 0,3 | 1,0 | 1,0 | 1,0 | 1,0 | 1,0 | 0,8 | 0,8 | 1,0 | 1,0 | 1,0 | 1,0 | 2,7 | 1,0 | 5,4 | 0,2 | 1,0 | 1,0 | 1,0 | 1,0 |
| Fgb | 8,4 | 14,2 | 0,3 | 1,0 | 1,0 | 1,0 | 1,0 | 1,0 | 0,8 | 0,8 | 1,0 | 1,0 | 1,0 | 1,0 | 1,0 | 1,0 | 7,2 | 0,1 | 1,0 | 1,0 | 1,0 | 1,0 |
| Fgf15 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,0 | 0,0 |
| Fgf23 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| | Kidney | | Colon | | Eyeball | | Heart | | Liver | | SG | | Thymus | | Adipose | | Stomach | | Jejunum | | Ileum | |
| | E | M | E | M | E | M | E | M | E | M | E | M | E | M | E | M | E | M | E | M | E | M |
| Fgfbp1 | 1,6 | 1,6 | 1,0 | 1,1 | 1,1 | 1,1 | 1,0 | 1,0 | 1,0 | 1,0 | 1,7 | 1,4 | 1,0 | 1,3 | 1,0 | 1,0 | 1,1 | 1,0 | 1,0 | 1,1 | 1,2 | 0,9 |
| Fgg | *13,0* | *12,3* | *0,5* | *1,0* | *1,0* | *1,0* | *1,0* | *1,0* | 0,7 | 0,8 | 1,0 | 1,0 | 1,0 | 1,0 | 1,0 | 1,0 | 4,4 | 0,2 | 1,0 | 1,0 | 1,0 | 1,0 |
| Fhl3 | 1,1 | 1,0 | 0,9 | 0,7 | 0,6 | 0,6 | 1,1 | 0,9 | 1,0 | 1,0 | 1,0 | 0,9 | 1,0 | 1,1 | 0,8 | 1,8 | 0,8 | 1,0 | 1,0 | 0,9 | 1,2 | 1,2 |
| Fhl4 | 1,0 | 1,0 | 1,0 | 1,0 | 1,0 | 1,0 | 1,0 | 1,0 | 1,0 | 1,0 | 1,0 | 1,0 | 1,0 | 1,0 | 6,8 | 1,8 | 1,0 | 1,0 | 1,0 | 1,0 | 1,0 | 1,0 |
| Fitm1 | *0,7* | *0,6* | *1,0* | *1,0* | *0,6* | *0,4* | *0,9* | *1,0* | *0,3* | *0,2* | *1,0* | *1,0* | *1,0* | *1,0* | *1,0* | *1,0* | 1,0 | *1,0* | *1,0* | *1,0* | *1,0* | *1,0* |
| Flnc | 1,0 | 1,0 | 1,2 | -1,0 | 0,7 | 0,6 | 0,8 | 0,9 | 1,0 | 1,0 | 1,0 | 1,0 | 1,0 | 1,0 | 1,3 | 0,9 | 1,4 | 1,4 | 1,1 | 0,7 | 0,8 | 1,5 |
| Flrt2 | 1,3 | 1,2 | 0,9 | 1,0 | 1,1 | 1,1 | 1,0 | 0,8 | 1,0 | 1,0 | 1,0 | 1,0 | 1,1 | 0,9 | 0,8 | 0,6 | 0,8 | 1,2 | 1,0 | 1,0 | 1,0 | 1,0 |
| Fmo2 | 1,2 | 1,1 | 1,7 | 1,9 | 1,1 | 1,1 | 1,5 | 1,5 | 7,8 | 8,1 | 1,2 | 1,5 | 2,3 | 3,2 | 1,1 | 1,0 | 1,2 | 1,3 | 1,3 | 1,3 | 1,4 | 2,3 |
| Fmo3 | 1,2 | 1,0 | 1,0 | 1,0 | 1,0 | 1,0 | 1,7 | 1,0 | 12,4 | 74,0 | 1,0 | 1,0 | 1,0 | 1,0 | 1,2 | 1,4 | 1,0 | 1,0 | 1,0 | 1,0 | 1,0 | 1,0 |
| Fn3k | 0,6 | 0,7 | 1,0 | 1,1 | 1,1 | 1,0 | 1,0 | 1,2 | 0,6 | 1,0 | 1,7 | 0,7 | 0,9 | 0,9 | 0,9 | 2,9 | 0,6 | 0,4 | 1,0 | 1,0 | 1,0 | 1,1 |
| Foxi1 | 1,4 | 1,0 | 1,0 | 1,0 | 1,0 | 1,0 | 1,0 | 1,0 | 1,0 | 1,0 | 1,2 | 1,2 | 1,0 | 1,0 | 13,7 | 1,0 | 1,0 | 1,0 | 1,0 | 1,0 | 1,0 | 1,0 |
| Foxj1 | 1,0 | 1,4 | 1,0 | 1,0 | 0,8 | 0,7 | 1,0 | 1,0 | 1,0 | 1,0 | 1,0 | 1,0 | 2,2 | 0,7 | 1,1 | 8,3 | 1,0 | 1,0 | 1,0 | 1,0 | 1,0 | 1,0 |
| Snora78 | 1,4 | 0,9 | 0,9 | 1,0 | 1,4 | 0,5 | 1,0 | 0,8 | 2,9 | 0,4 | 0,9 | 1,9 | 0,6 | 1,0 | 0,6 | 0,3 | 0,8 | 1,0 | 0,7 | 0,5 | 1,1 | 3,0 |
| Foxo3 | 1,7 | 1,0 | 0,8 | 1,3 | 1,1 | 0,5 | 0,9 | 1,0 | 1,2 | 0,3 | 0,9 | 1,7 | 1,0 | 0,9 | 1,1 | 0,5 | 0,8 | 1,0 | 0,9 | 0,6 | 1,2 | 1,9 |
| Foxo6 | 1,0 | 1,0 | 1,2 | 1,3 | 0,8 | 0,7 | 0,8 | 0,6 | 1,0 | 1,0 | 1,3 | 1,2 | 1,0 | 1,0 | 1,5 | 1,0 | 0,8 | 0,9 | 1,4 | 1,5 | 0,7 | 0,9 |
| Foxq1 | 1,5 | 1,4 | 1,3 | 1,1 | 1,0 | 0,8 | 1,0 | 1,0 | 0,5 | 0,3 | 1,2 | 1,1 | 3,1 | 1,2 | 1,7 | 1,0 | 0,9 | 1,0 | 0,7 | 1,2 | 1,2 | 2,4 |
| Fsd2 | 1,0 | 1,0 | 1,0 | 1,0 | 0,6 | 0,5 | 0,9 | 0,9 | 1,0 | 1,0 | 1,0 | 1,0 | 0,8 | 0,8 | 0,4 | 0,6 | 1,0 | 1,0 | 1,0 | 1,0 | 1,0 | 1,0 |
| Fst | 1,0 | 2,6 | 1,6 | 1,2 | 0,8 | 1,2 | 1,0 | 0,9 | 0,7 | 0,2 | 0,7 | 0,7 | 1,3 | 1,1 | 0,6 | 0,7 | 2,6 | 1,3 | 1,9 | 1,8 | 1,7 | 1,3 |
| Fut1 | 1,0 | 1,0 | 0,6 | 0,8 | 1,0 | 1,0 | 1,0 | 1,0 | 1,0 | 1,0 | 1,0 | 1,0 | 1,1 | 1,0 | 5,4 | 1,0 | 1,2 | 0,8 | 0,8 | 1,0 | 1,1 | 0,4 |
| Fut4 | 1,0 | 1,0 | 1,2 | 1,2 | 1,6 | 2,3 | 1,0 | 1,0 | 1,0 | 1,0 | 0,8 | 1,1 | 1,2 | 1,4 | 7,4 | 1,0 | 1,1 | 1,1 | 1,3 | 1,3 | 1,1 | 1,3 |
| Fxyd3 | 1,0 | 1,0 | 1,9 | 0,5 | 1,0 | 1,0 | 1,0 | 1,0 | 1,0 | 1,0 | 2,0 | 0,9 | 1,0 | 0,8 | 1,0 | 1,0 | 1,1 | 1,2 | 1,0 | 1,0 | 1,0 | 0,7 |
| Fxyd4 | 0,6 | 1,0 | 1,1 | 5,8 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |

[Fig. 23-21]

| | | | | | | | | | | | | | | | | | | | | | | |
|---|---|---|---|---|---|---|---|---|---|---|---|---|---|---|---|---|---|---|---|---|---|---|
| Fzd8 | 1,0 | 0,7 | 1,1 | 1,1 | 0,9 | 0,7 | 0,8 | 1,1 | 0,1 | 0,3 | 1,4 | 1,1 | 2,3 | 1,7 | 1,2 | 1,1 | 0,8 | 0,8 | 0,8 | 0,9 | 0,8 | 1,0 |
| Gadd45b | 1,6 | 1,9 | 1,0 | 0,8 | 1,0 | 0,9 | 0,8 | 1,4 | 7,4 | 10,6 | 1,1 | 0,7 | 1,6 | 0,8 | 1,2 | 1,1 | 0,8 | 1,0 | 1,5 | 1,7 | 1,7 | 1,2 |
| Gal | 1,0 | 1,0 | 0,5 | 1,7 | 1,0 | 1,0 | 1,0 | 1,0 | 1,0 | 1,0 | 1,3 | 1,6 | 1,0 | 1,0 | 1,0 | 1,0 | 1,1 | 1,5 | 1,0 | 1,2 | 0,9 | 0,9 |
| Gal3st4 | 1,0 | 1,0 | 1,0 | 0,8 | 1,1 | 0,8 | 1,0 | 1,0 | 1,0 | 1,0 | 1,0 | 1,0 | 1,0 | 0,9 | 11,7 | 1,0 | 0,7 | 0,7 | 1,0 | 1,0 | 1,0 | 1,0 |
| Galnt12 | 1,0 | 1,7 | 1,1 | 1,0 | 0,9 | 1,1 | 1,0 | 1,0 | 1,0 | 1,0 | 1,0 | 1,1 | 1,0 | 1,0 | 8,4 | 1,0 | 1,2 | 1,0 | 1,0 | 1,0 | 0,9 | 0,9 |
| Gamt | 0,6 | 0,7 | 0,6 | 1,1 | 0,9 | 0,7 | 0,5 | 0,5 | 0,6 | 0,8 | 1,0 | 0,7 | 1,9 | 1,3 | 2,0 | 0,7 | 1,1 | 0,7 | 1,6 | 0,8 | 1,0 | 0,9 |
| Gap43 | 1,0 | 1,0 | 1,0 | 1,3 | 1,0 | 1,1 | 1,0 | 1,0 | 1,0 | 1,0 | 1,0 | 1,0 | 1,0 | 1,0 | 1,0 | 1,0 | 0,7 | 0,8 | 0,9 | 0,8 | 1,0 | 0,9 |
| Gast | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,6 | 0,3 | 1,0 | 1,0 | 1,0 | 1,0 |
| Gata3 | 1,2 | 1,1 | 1,0 | 1,0 | 1,0 | 1,0 | 1,0 | 1,0 | 1,0 | 1,0 | 1,0 | 1,0 | 0,8 | 0,9 | 6,2 | 2,3 | 0,8 | 1,0 | 1,0 | 1,0 | 1,0 | 1,0 |
| Gbp11 | 1,0 | 1,0 | 1,0 | 1,0 | 1,0 | 1,0 | 1,0 | 1,0 | 0,1 | 0,2 | 1,0 | 1,0 | 0,7 | 1,0 | 0,2 | 1,0 | 1,0 | 1,0 | 1,0 | 1,0 | 1,0 | 1,0 |
| Gbp6 | 0,5 | 0,6 | 0,6 | 0,8 | 1,1 | 1,2 | 1,1 | 1,0 | 0,4 | 0,6 | 0,9 | 0,8 | 0,8 | 0,8 | 0,4 | 0,7 | 0,7 | 0,6 | 0,8 | 0,6 | 0,6 | 0,2 |
| Gc | 5,0 | 7,9 | 0,5 | 1,0 | 1,0 | 1,0 | 1,0 | 1,0 | 1,0 | 1,1 | 1,0 | 1,0 | 1,0 | 1,0 | 7,5 | 1,0 | 5,5 | 0,3 | 1,0 | 1,0 | 1,0 | 1,0 |
| Gcm2 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Gcnt4 | 2,7 | 2,6 | 0,9 | 0,9 | 1,1 | 0,8 | 1,0 | 1,0 | 1,2 | 1,2 | 1,0 | 1,0 | 1,0 | 1,0 | 17,4 | 1,0 | 1,0 | 1,0 | 0,9 | 0,8 | 1,0 | 1,2 |
| Gfi1b | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Gfra1 | 1,8 | 1,4 | 0,9 | 1,1 | 1,0 | 0,9 | 1,0 | 1,2 | 1,2 | 1,0 | 1,0 | 1,0 | 0,7 | 1,3 | 39,8 | 1,0 | 0,7 | 0,7 | 1,0 | 1,0 | 0,8 | 1,4 |
| *Snord15b* | *1,0* | *1,1* | *1,0* | *1,0* | *1,3* | *1,0* | *1,0* | *1,0* | *0,8* | *0,9* | *0,6* | *1,0* | *1,0* | *0,1* | *1,0* | *1,0* | *1,0* | *1,0* | *1,0* | *1,0* | *1,0* | *1,0* |
| Glb1l3 | 1,0 | 1,0 | 1,0 | 1,0 | 1,0 | 1,3 | 1,0 | 1,0 | 1,0 | 1,0 | 1,0 | 1,0 | 1,0 | 1,0 | 6,0 | 1,0 | 1,0 | 1,0 | 1,0 | 1,0 | 1,0 | 1,0 |
| Gldc | 3,8 | 5,0 | 1,0 | 1,0 | 1,0 | 0,9 | 1,0 | 1,0 | 1,0 | 0,7 | 1,0 | 1,0 | 1,0 | 1,0 | 5,0 | 1,2 | 1,1 | 0,9 | 1,0 | 1,0 | 1,0 | 1,0 |
| Gli1 | 1,0 | 1,0 | 1,1 | 1,0 | 1,1 | 0,9 | 1,1 | 1,1 | 1,0 | 1,0 | 1,0 | 1,0 | 1,0 | 1,0 | 1,5 | 0,9 | 1,0 | 1,0 | 0,9 | 0,8 | 1,0 | 1,0 |
| *Glrp1* | *1,0* | *1,0* | *1,0* | *1,0* | *1,0* | *1,0* | *1,0* | *1,0* | *1,0* | *1,0* | *1,0* | *1,0* | *1,0* | *1,0* | 5,5 | *1,0* | *1,0* | *1,0* | *1,0* | *1,0* | *1,0* | *1,0* |
| Spt1 | 1,0 | 1,0 | 1,0 | 1,0 | 1,0 | 0,6 | 0,9 | 1,9 | 1,0 | 1,0 | 1,0 | 1,0 | 0,9 | 0,7 | 1,0 | 1,0 | 1,0 | 1,0 | 1,0 | 1,0 | 1,0 | 1,0 |
| Gml | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,1 | 1,3 | 15,9 | 4,0 | 3,1 | 2,7 |
| Golt1a | 0,8 | 1,1 | 1,0 | 1,3 | 1,0 | 1,0 | 1,0 | 1,0 | 1,0 | 1,1 | 0,1 | 0,2 | 1,0 | 1,0 | 1,8 | 1,0 | 1,4 | 1,1 | 1,0 | 1,4 | 1,2 | 1,0 |
| Gp2 | 1,1 | 0,7 | 2,4 | 7,5 | 1,0 | 1,0 | 1,0 | 1,0 | 1,0 | 1,0 | 1,0 | 1,0 | 1,8 | 2,1 | 1,0 | 1,0 | 36,7 | 0,1 | 0,2 | 1,0 | 1,0 | 0,8 |
| Gpcpd1 | 0,8 | 1,2 | 1,0 | 1,2 | 1,0 | 1,2 | 1,0 | 1,2 | 0,1 | 0,3 | 0,9 | 1,0 | 0,8 | 1,0 | 0,7 | 1,0 | 0,9 | 1,0 | 1,5 | 1,8 | 1,2 | 1,1 |
| Gpnmb | 1,0 | 5,7 | 0,9 | 0,4 | 1,0 | 1,1 | 1,3 | 1,6 | 1,0 | 1,0 | 1,0 | 1,0 | 2,0 | 1,6 | 1,6 | 0,5 | 1,1 | 3,6 | 0,9 | 1,0 | 0,6 | 1,3 |
| Gpr18 | 1,0 | 1,0 | 0,6 | 0,2 | 1,0 | 1,0 | 1,0 | 1,0 | 1,0 | 1,0 | 1,0 | 1,4 | 1,7 | 1,1 | 1,0 | 1,0 | 1,0 | 1,0 | 1,0 | 1,2 | 0,9 | 0,9 |
| Gpr82 | 1,0 | 1,0 | 1,0 | 1,0 | 1,0 | 1,0 | 1,0 | 1,0 | 1,0 | 1,0 | 1,0 | 1,0 | 1,0 | 1,0 | 5,1 | 1,0 | 1,0 | 1,0 | 1,0 | 1,0 | 1,0 | 1,0 |
| Gpx5 | 1,0 | 1,0 | 1,0 | 1,0 | 1,0 | 1,0 | 1,0 | 1,0 | 1,0 | 1,0 | 1,0 | 1,0 | 1,0 | 1,0 | 6890 | 1,0 | 1,0 | 1,0 | 1,0 | 1,0 | 1,0 | 1,0 |
| Gpx6 | 1,3 | 1,2 | 1,0 | 1,0 | 1,0 | 1,0 | 1,0 | 1,0 | 0,3 | 0,4 | 1,0 | 1,0 | 1,0 | 1,0 | 8,9 | 1,0 | 1,0 | 1,0 | 1,0 | 1,0 | 1,0 | 1,0 |
| Grb14 | 1,3 | 1,1 | 0,8 | 0,9 | 1,0 | 0,8 | 0,9 | 0,9 | 0,7 | 0,7 | 0,7 | 0,6 | 1,0 | 1,0 | 0,2 | 0,4 | 1,0 | 1,0 | 1,0 | 1,0 | 1,0 | 0,9 |
| Grhl2 | 1,2 | 1,3 | 1,0 | 1,2 | 1,1 | 1,1 | 1,1 | 1,0 | 1,0 | 1,0 | 1,1 | 1,1 | 2,0 | 1,7 | 7,3 | 1,0 | 1,0 | 0,9 | 1,3 | 0,9 | 1,1 | 1,1 |
| Gsdmc | 1,0 | 1,0 | 0,1 | 0,2 | 1,0 | 1,0 | 1,0 | 1,0 | 1,0 | 1,0 | 1,0 | 1,0 | 1,0 | 1,0 | 1,0 | 1,0 | 0,9 | 1,2 | 1,0 | 1,0 | 0,2 | 0,4 |
| Gsdmc2 | 0,7 | 1,5 | 0,0 | 0,2 | 1,0 | 1,0 | 1,0 | 1,0 | 1,0 | 1,0 | 1,0 | 1,0 | 1,0 | 1,0 | 1,0 | 1,0 | 1,0 | 0,7 | 0,2 | 0,6 | 0,0 | 0,4 |
| Gsdmc3 | 0,9 | 1,1 | 0,0 | 0,3 | 1,0 | 1,0 | 1,0 | 1,0 | 1,0 | 1,0 | 1,0 | 1,0 | 1,0 | 1,0 | 1,0 | 1,0 | 0,8 | 0,4 | 0,9 | 1,0 | 0,1 | 0,3 |
| Gsdmc4 | 1,0 | 1,0 | 0,0 | 0,3 | 1,0 | 1,0 | 1,0 | 1,0 | 1,0 | 1,0 | 1,0 | 1,0 | 1,0 | 1,0 | 1,0 | 1,0 | 1,0 | 0,8 | 0,2 | 0,3 | 0,1 | 0,3 |
| Gsg1 | 1,0 | 1,0 | 1,0 | 1,0 | 1,0 | 1,0 | 1,0 | 1,0 | 1,0 | 1,0 | 1,0 | 1,0 | 1,0 | 1,0 | 8,5 | 2,4 | 1,0 | 1,0 | 1,0 | 1,0 | 1,0 | 1,0 |
| Gsta1 | 4,8 | 6,2 | 1,7 | 1,3 | 1,1 | 1,5 | 0,7 | 0,4 | 2,9 | 11,5 | 1,0 | 1,0 | 1,0 | 1,0 | 1,0 | 1,0 | 0,9 | 0,9 | 1,1 | 1,1 | 2,2 | 1,1 |
| Gsta2 | 3,6 | 4,1 | 1,2 | 1,0 | 1,1 | 1,3 | 1,0 | 1,0 | 3,5 | 9,9 | 1,0 | 1,0 | 1,2 | 1,0 | 1,0 | 1,0 | 0,9 | 0,7 | 1,2 | 2,6 | 1,0 | 1,0 |
| Gsta4 | 3,4 | 6,0 | 1,3 | 1,4 | 1,0 | 1,3 | 1,0 | 1,3 | 2,1 | 4,6 | 1,6 | 1,3 | 1,2 | 1,2 | 1,2 | 1,6 | 1,0 | 0,9 | 1,5 | 1,7 | 1,3 | 0,8 |
| Gstm1 | 1,3 | 1,1 | 1,7 | 1,1 | 1,0 | 1,1 | 1,2 | 1,5 | 3,3 | 4,7 | 1,8 | 1,1 | 2,3 | 2,8 | 1,1 | 1,5 | 1,2 | 1,0 | 3,0 | 5,7 | 1,5 | 1,2 |
| Gstm3 | 1,0 | 1,0 | 1,2 | 1,1 | 1,0 | 1,0 | 1,0 | 1,0 | 7,0 | 15,2 | 1,0 | 1,0 | 1,0 | 1,0 | 1,0 | 1,0 | 1,4 | 0,9 | 2,5 | 4,3 | 0,8 | 0,8 |
| Gulo | 6,8 | 7,7 | 1,0 | 1,0 | 0,7 | 0,9 | 1,0 | 1,0 | 0,5 | 0,6 | 1,0 | 1,0 | 1,0 | 1,0 | 1,0 | 1,0 | 1,0 | 1,0 | 1,0 | 1,0 | 1,0 | 1,0 |
| Gypa | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| H19 | 1,0 | 1,0 | 1,0 | 1,0 | 0,6 | 0,7 | 1,3 | 1,1 | 1,0 | 1,0 | 1,0 | 1,0 | 0,5 | 0,6 | 1,0 | 1,0 | 0,8 | 1,2 | 1,0 | 1,0 | 1,0 | 1,0 |
| H1fx | 1,5 | 0,8 | 0,8 | 0,9 | 0,7 | 0,6 | 0,9 | 0,4 | 1,0 | 1,0 | 1,0 | 1,0 | 0,7 | 0,7 | 0,8 | 0,8 | 0,7 | 0,9 | 0,9 | 1,1 | 1,1 | 0,7 |
| *H2-Ob* | 0,8 | 0,8 | *0,4* | 0,3 | 1,0 | 1,0 | 1,0 | 1,0 | 1,0 | 1,0 | 0,5 | 1,7 | 1,3 | 0,9 | 0,9 | 0,7 | 1,0 | 1,0 | 0,5 | 0,4 | 0,7 | 0,6 |
| H2-Q10 | 13,8 | 13,4 | 0,8 | 0,7 | 1,2 | 0,6 | 1,0 | 1,0 | 0,6 | 0,6 | 1,0 | 1,4 | 1,2 | 1,8 | 1,2 | 3,9 | 5,8 | 0,2 | 1,0 | 0,8 | 0,7 | 0,8 |
| H2-Q6 | 0,7 | 0,7 | 0,7 | 0,5 | 0,8 | 0,8 | 0,8 | 0,8 | 1,1 | 0,7 | 0,9 | 1,2 | 1,9 | 1,1 | 0,6 | 0,8 | 0,3 | 0,8 | 1,1 | 0,9 | 1,0 | 1,0 |
| H2-Q8 | 1,0 | 1,0 | 0,2 | 1,0 | 1,0 | 1,0 | 1,0 | 1,0 | 5,3 | 1,9 | 1,0 | 1,0 | 3,9 | 7,4 | 1,0 | 1,0 | 2,4 | 1,0 | 1,0 | 0,5 | 1,0 | 1,0 |
| *Hamp* | *1,0* | *1,0* | *0,7* | *1,0* | *1,0* | *1,0* | *1,9* | *1,8* | *0,8* | *1,8* | *1,0* | *1,0* | *1,3* | *0,7* | *1,0* | *1,0* | *11,6* | *0,2* | *1,2* | *1,0* | *1,0* | *1,0* |
| Hamp2 | 1,0 | 1,0 | 0,7 | 0,7 | 1,0 | 1,0 | 1,0 | 1,1 | 0,8 | 2,7 | 1,0 | 1,0 | 1,0 | 1,0 | 1,0 | 1,0 | 1,0 | 0,9 | 1,0 | 1,0 | 1,0 | 1,0 |
| Hao1 | 1,0 | 1,0 | 1,0 | 1,0 | 1,0 | 1,0 | 1,0 | 1,0 | 0,1 | 0,4 | 1,0 | 1,0 | 1,0 | 1,0 | 1,0 | 1,0 | 1,0 | 1,0 | 1,0 | 1,0 | 1,0 | 1,0 |
| Hap1 | 1,0 | 1,8 | 0,7 | 1,0 | 1,0 | 1,0 | 1,0 | 0,9 | 1,0 | 0,9 | 1,1 | 1,3 | 2,2 | 1,5 | 10,5 | 1,3 | 1,0 | 0,7 | 0,8 | 0,8 | 0,9 | 0,8 |
| Has3 | 1,0 | 1,0 | 1,0 | 1,0 | 1,0 | 1,0 | 0,8 | 0,7 | 1,0 | 1,0 | 1,0 | 1,0 | 1,0 | 1,2 | 0,8 | 0,6 | 0,9 | 1,3 | 1,0 | 1,0 | 1,0 | 1,0 |
| Havcr1 | 28,7 | 37,3 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Hba-a2 | 42,7 | 0,6 | 19,9 | 51,9 | 3,0 | 0,4 | 0,8 | 1,4 | 19,8 | 2,1 | 16,2 | 1,3 | 28,0 | 18,9 | 0,2 | 1,3 | 1,0 | 4,5 | 1,0 | 4,8 | 31,7 | 33,7 |
| Hbb-bs | 0,1 | 0,9 | 1,0 | 0,7 | 2,0 | 0,4 | 0,3 | 2,6 | 1,0 | 2,7 | 1,0 | 0,9 | 1,0 | 9,5 | 18,3 | 1,3 | 1,0 | 1,0 | 1,0 | 27,8 | 8,8 | 1,0 |
| Hebp2 | 1,1 | 1,8 | 1,4 | 0,8 | 1,1 | 1,1 | 1,0 | 1,0 | 1,0 | 1,0 | 0,8 | 0,5 | 1,0 | 1,0 | 6,3 | 1,0 | 0,9 | 0,9 | 1,0 | 1,0 | 0,9 | 0,9 |
| Hemgn | 1,0 | 1,0 | 1,0 | 1,0 | 1,0 | 1,0 | 1,0 | 1,0 | 1,0 | 1,0 | 1,0 | 1,0 | 0,4 | 0,5 | 1,0 | 1,0 | 1,0 | 1,0 | 0,8 | 0,7 | 1,0 | 1,0 |
| Hemt1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,7 | 1,5 | 12,1 | 2,4 | 2,5 | 1,3 |
| Hfe2 | 1,0 | 1,0 | 1,0 | 1,0 | 0,7 | 0,6 | 0,8 | 0,9 | 0,9 | 0,9 | 1,0 | 1,0 | 1,0 | 1,0 | 1,0 | 1,0 | 1,0 | 0,8 | 1,0 | 1,0 | 1,0 | 1,0 |
| Hgfac | 1,0 | 0,9 | 0,9 | 1,0 | 1,0 | 1,0 | 1,0 | 1,0 | 0,7 | 0,8 | 1,0 | 1,0 | 1,0 | 1,0 | 9,3 | 1,0 | 1,7 | 0,9 | 1,2 | 0,9 | 0,8 | 1,1 |
| Hhatl | 0,7 | 0,6 | 1,1 | 1,6 | 0,7 | 0,6 | 0,9 | 1,0 | 1,0 | 1,0 | 0,8 | 0,6 | 1,0 | 1,0 | 1,0 | 1,0 | 1,0 | 1,0 | 1,0 | 1,0 | 1,2 | 1,3 |
| Gh | 1,7 | 0,6 | 0,9 | 1,4 | 1,1 | 0,5 | 0,9 | 0,7 | 4,1 | 0,5 | 0,7 | 2,7 | 0,6 | 1,0 | 0,6 | 0,3 | 0,6 | 0,9 | 0,6 | 0,4 | 1,0 | 2,0 |
| Hist1h1e | 0,6 | 0,7 | 0,9 | 0,9 | 0,9 | 0,8 | 1,3 | 0,5 | 0,4 | 0,5 | 0,7 | 0,6 | 1,1 | 0,8 | 0,7 | 0,2 | 0,9 | 0,6 | 0,7 | 0,9 | 0,7 | 1,2 |
| Hist1h2ba | 0,8 | 1,0 | 1,0 | 1,0 | 0,6 | 0,7 | 0,4 | 1,3 | 0,5 | 0,5 | 1,7 | 0,8 | 1,8 | 1,2 | 0,3 | 0,7 | 1,2 | 0,9 | 0,8 | 1,0 | 1,0 | 1,0 |
| Hist1h4h | 0,6 | 0,6 | 0,9 | 1,2 | 0,7 | 0,5 | 0,9 | 1,3 | 0,1 | 0,2 | 0,5 | 0,4 | 1,7 | 1,0 | 0,3 | 0,7 | 1,1 | 1,2 | 1,1 | 0,8 | 1,6 | 0,9 |
| Hist1h4i | 0,7 | 0,3 | 0,8 | 0,8 | 0,9 | 0,7 | 0,6 | 0,7 | 0,5 | 0,3 | 1,5 | 1,0 | 1,0 | 0,6 | 1,0 | 0,2 | 1,0 | 0,6 | 0,7 | 1,3 | 1,1 | 0,8 |
| Hist1h4j | 0,3 | 0,8 | 0,6 | 2,3 | 0,9 | 0,4 | 0,2 | 1,1 | 1,0 | 0,7 | 1,3 | 0,6 | 0,6 | 0,6 | 0,5 | 0,6 | 1,0 | 0,4 | 1,5 | 0,6 | 0,5 | 0,3 |
| Hist1h4k | 0,5 | 1,0 | 0,8 | 0,9 | 1,1 | 0,4 | 0,4 | 1,1 | 1,0 | 0,7 | 1,0 | 1,0 | 1,4 | 0,7 | 0,5 | 0,8 | 1,2 | 0,6 | 0,7 | 0,2 | 0,6 | 1,5 |
| Hist2h2aa2 | 1,0 | 1,2 | 0,6 | 1,1 | 5,9 | 1,3 | 0,4 | 2,2 | 1,0 | 1,0 | 0,5 | 2,7 | 1,0 | 0,2 | 3,6 | 1,2 | 0,3 | 1,9 | 5,0 | 0,3 | 0,7 | 4,9 |
| Hist2h2ac | 1,0 | 1,0 | 0,8 | 0,7 | 1,0 | 1,0 | 1,0 | 1,0 | 1,0 | 1,0 | 1,0 | 1,3 | 1,2 | 0,5 | 0,7 | 1,2 | 1,0 | 1,0 | 1,4 | 1,2 | 1,0 | 2,4 |
| Hist2h3b | 1,6 | 0,3 | 1,0 | 1,4 | 0,9 | 0,5 | 0,7 | 1,5 | 0,5 | 0,6 | 1,0 | 1,0 | 2,1 | 0,7 | 1,8 | 0,4 | 1,4 | 0,5 | 0,6 | 1,6 | 2,3 | 0,8 |
| | Kidney | | Colon | | Eyeball | | Heart | | Liver | | SG | | Thymus | | Adipose | | Stomach | | Jejunum | | Ileum | |
| | E | M | E | M | E | M | E | M | E | M | E | M | E | M | E | M | E | M | E | M | E | M |
| Hist2h3c1 | 1,9 | 2,1 | 5,0 | 0,6 | 1,0 | 0,2 | 0,3 | 0,4 | 1,0 | 1,0 | 1,0 | 1,0 | 0,7 | 4,1 | 1,0 | 5,5 | 2,5 | 0,1 | 4,2 | 2,1 | 0,2 | 1,0 |
| *Hist2h3c2* | 0,2 | 0,1 | 0,8 | 2,8 | 0,9 | 0,5 | 2,9 | 1,3 | 0,3 | 0,2 | 0,8 | 0,9 | 2,8 | 0,3 | 1,4 | 0,0 | 0,2 | 1,2 | 0,5 | 0,2 | 1,3 | 1,2 |
| Hmgcs2 | 3,7 | 5,0 | 1,2 | 1,9 | 0,9 | 1,1 | 0,8 | 4,0 | 0,7 | 0,8 | 1,0 | 1,0 | 0,9 | 1,4 | 0,8 | 1,8 | 0,9 | 0,9 | 0,4 | 0,3 | 0,9 | 2,1 |
| Hnrnpa1 | 0,4 | 0,5 | 1,4 | 1,7 | 0,4 | 0,9 | 0,6 | 1,9 | 1,0 | 0,5 | 1,0 | 1,3 | 0,7 | 0,8 | 1,7 | 1,6 | 1,0 | 0,9 | 0,3 | 1,1 | 1,2 | 1,6 |
| Hoxb6 | 1,2 | 1,0 | 0,9 | 0,9 | 1,0 | 1,0 | 1,0 | 1,0 | 1,0 | 1,0 | 1,0 | 1,0 | 1,0 | 1,0 | 5,4 | 1,1 | 0,8 | 0,8 | 1,1 | 0,8 | 1,0 | 0,8 |
| *Hoxb8* | 1,3 | *1,4* | *0,9* | *1,0* | 1,0 | 1,0 | 1,0 | 1,0 | 1,0 | 1,0 | 1,0 | 1,0 | 1,0 | 1,0 | 5,8 | 2,4 | 1,2 | 0,6 | 1,0 | 1,0 | 0,9 | 1,0 |
| Hoxd3 | 1,3 | 1,0 | 0,7 | 1,2 | 1,0 | 1,0 | 1,0 | 1,0 | 1,0 | 1,0 | 1,0 | 1,0 | 1,0 | 1,0 | 5,3 | 1,8 | 1,0 | 1,0 | 1,0 | 1,0 | 1,0 | 1,0 |

[Fig. 23-22]

291/310

| | | | | | | | | | | | | | | | | | | | | | | |
|---|---|---|---|---|---|---|---|---|---|---|---|---|---|---|---|---|---|---|---|---|---|---|
| Hpdl | 1,5 | 2,0 | 1,1 | 1,2 | 1,0 | 0,8 | 0,7 | 0,7 | 1,0 | 1,0 | [illegible] | [illegible] | 1,0 | 1,0 | 9,8 | 1,0 | 1,7 | 1,3 | 1,1 | 1,4 | 1,0 | 1,4 |
| Hpx | 1,0 | 1,0 | 0,6 | 1,0 | 1,0 | 1,0 | 1,0 | 1,0 | 0,9 | 1,0 | 1,0 | 1,0 | 1,0 | 1,0 | 1,0 | 1,0 | 7,2 | 0,2 | 1,0 | 1,0 | 1,0 | 1,0 |
| Hr | 1,0 | 1,1 | 0,9 | 0,8 | 0,9 | 0,8 | 0,8 | 1,0 | 1,0 | 1,0 | 0,6 | 0,6 | 1,4 | 1,1 | 0,3 | 0,3 | 1,0 | 0,9 | 0,9 | 0,9 | 1,0 | 1,0 |
| Hrc | 1,0 | 1,0 | 0,8 | 1,2 | 0,7 | 0,7 | 1,0 | 1,1 | 1,0 | 1,0 | 1,0 | 1,0 | 1,0 | 1,0 | 1,0 | 1,0 | 0,8 | 0,9 | 0,5 | 0,6 | 0,9 | 1,4 |
| Hrg | 0,4 | 0,1 | 0,9 | 1,0 | 1,0 | 1,0 | 1,0 | 1,0 | 0,7 | 0,8 | 1,0 | 1,0 | 1,0 | 1,0 | 1,0 | 1,0 | 3,5 | 0,3 | 1,0 | 1,0 | 1,0 | 1,0 |
| Hs3st3b1 | 1,3 | 0,9 | 0,9 | 0,8 | 1,1 | 1,1 | 1,0 | 1,0 | 0,9 | 0,7 | 1,4 | 1,2 | 1,4 | 1,1 | 5,9 | 3,1 | 0,7 | 0,7 | 0,9 | 0,7 | 0,9 | 0,8 |
| Hsd17b11 | 0,3 | 0,3 | 1,1 | 1,3 | 1,1 | 1,0 | 1,0 | 1,5 | 0,8 | 1,3 | 1,0 | 0,8 | 1,1 | 1,2 | 0,9 | 1,4 | 0,9 | 0,9 | 0,9 | 1,4 | 1,2 | 1,0 |
| Hsd17b6 | 1,0 | 1,0 | 1,0 | 1,0 | 1,0 | 1,0 | 1,0 | 1,0 | 1,5 | 6,5 | 1,0 | 1,0 | 1,0 | 1,0 | 1,0 | 1,0 | 0,9 | 0,8 | 1,0 | 1,7 | 0,6 | 1,3 |
| Hsd3b2 | 0,2 | 0,2 | 1,0 | 1,2 | 1,0 | 1,0 | 1,0 | 1,0 | 0,4 | 0,4 | 1,0 | 1,0 | 1,0 | 1,0 | 1,0 | 1,0 | 1,0 | 1,0 | 0,7 | 1,2 | 1,5 | 1,8 |
| Hsd3b5 | 1,0 | 1,3 | 1,0 | 1,0 | 1,0 | 1,0 | 1,0 | 1,0 | 0,1 | 0,0 | 1,0 | 1,0 | 1,0 | 1,0 | 1,0 | 1,0 | 1,0 | 1,0 | 1,0 | 1,0 | 1,0 | 1,0 |
| Hspb3 | 1,0 | 1,0 | 1,0 | 1,0 | 0,8 | 0,5 | 0,9 | 0,7 | 1,0 | 1,0 | 1,0 | 1,0 | 1,0 | 1,0 | 1,0 | 1,0 | 1,0 | 1,0 | 1,0 | 1,0 | 1,0 | 1,0 |
| Hspb6 | 0,7 | 0,8 | 0,8 | 0,9 | 0,7 | 0,8 | 1,0 | 1,0 | 1,0 | 1,2 | 0,9 | 0,9 | 0,7 | 1,0 | 0,8 | 1,2 | 1,1 | 0,8 | 1,3 | 0,9 | 0,8 | 1,1 |
| Hspb7 | 1,1 | 1,2 | 1,0 | 1,3 | 0,8 | 1,1 | 1,2 | 1,2 | 1,0 | 1,0 | 2,2 | 1,5 | 1,0 | 1,0 | 0,7 | 0,7 | 1,3 | 1,6 | 1,2 | 1,4 | 0,6 | 0,8 |
| Hspb9 | 0,8 | 1,0 | 1,0 | 1,0 | 1,0 | 1,0 | 1,0 | 1,0 | 1,0 | 1,0 | 1,0 | 1,0 | 1,1 | 1,2 | 6,5 | 1,4 | 1,0 | 1,2 | 0,6 | 1,3 | 0,9 | 1,0 |
| Icam4 | 1,0 | 1,0 | 1,0 | 1,0 | 1,0 | 1,0 | 0,6 | 1,1 | 1,2 | 1,0 | 1,0 | 1,0 | 1,0 | 1,0 | 1,0 | 1,0 | 1,0 | 1,0 | 1,0 | 1,0 | 1,0 | 1,0 |
| Ido1 | 1,0 | 1,0 | 0,6 | 0,6 | 1,0 | 1,0 | 1,0 | 1,0 | 1,0 | 1,0 | 1,0 | 1,0 | 1,0 | 1,0 | 62,5 | 1,0 | 1,0 | 1,0 | 1,2 | 1,6 | 0,7 | 0,7 |
| Igfbp1 | 2,1 | 5,3 | 1,0 | 1,0 | 1,0 | 1,0 | 1,0 | 1,0 | 1,7 | 1,5 | 1,0 | 1,0 | 1,0 | 1,0 | 1,0 | 1,0 | 1,0 | 1,0 | 1,0 | 1,0 | 1,0 | 1,0 |
| Igj | 0,7 | 1,1 | 1,1 | 0,5 | 1,0 | 1,2 | 1,0 | 1,0 | 1,0 | 1,0 | 0,4 | 2,2 | 1,3 | 1,5 | 0,6 | 0,5 | 1,6 | 0,8 | 1,0 | 1,2 | 1,0 | 0,9 |
| Ikzf3 | 1,0 | 1,0 | 0,6 | 0,2 | 1,0 | 1,0 | 1,0 | 1,0 | 1,0 | 1,0 | 1,0 | 1,8 | 0,7 | 0,8 | 1,0 | 0,8 | 1,0 | 1,0 | 0,9 | 0,6 | 0,8 | 1,1 |
| Il21r | 1,0 | 1,2 | 0,5 | 0,3 | 1,0 | 1,0 | 1,0 | 1,0 | 1,0 | 1,0 | 0,8 | 1,4 | 0,9 | 0,8 | 1,0 | 0,8 | 1,0 | 1,0 | 1,0 | 0,8 | 0,9 | 0,6 |
| Il22ra1 | 0,5 | 0,4 | 1,1 | 1,2 | 1,0 | 1,0 | 1,0 | 1,0 | 6,0 | 4,5 | 1,0 | 1,0 | 1,0 | 1,0 | 1,0 | 1,0 | 1,4 | 0,8 | 1,3 | 1,4 | 0,8 | 1,2 |
| Ildr1 | 1,2 | 1,3 | 1,2 | 1,2 | 1,0 | 1,2 | 1,0 | 1,0 | 1,0 | 1,0 | 0,9 | 0,9 | 1,8 | 1,7 | 7,3 | 1,0 | 0,8 | 0,8 | 0,8 | 0,7 | 1,0 | 1,0 |
| Ildr2 | 1,0 | 1,1 | 1,0 | 1,0 | 1,1 | 1,1 | 1,0 | 1,0 | 4,0 | 5,6 | 1,0 | 1,0 | 1,0 | 1,0 | 1,2 | 0,8 | 1,0 | 1,0 | 1,0 | 1,0 | 1,0 | 1,0 |
| Inha | 0,9 | 0,7 | 0,8 | 1,3 | 0,9 | 0,7 | 1,2 | 1,0 | 1,0 | 1,0 | 1,2 | 1,1 | 2,7 | 2,4 | 6,6 | 1,7 | 0,8 | 0,9 | 1,0 | 1,0 | 0,6 | 0,8 |
| Insc | 1,0 | 1,0 | 1,4 | 0,9 | 1,0 | 0,8 | 1,0 | 1,0 | 0,5 | 0,3 | 1,0 | 1,0 | 1,0 | 1,0 | 1,0 | 1,0 | 1,0 | 1,0 | 0,6 | 1,1 | 1,2 | 0,7 |
| Ip6k3 | 1,0 | 1,0 | 1,0 | 1,0 | 0,9 | 0,8 | 0,9 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Isyna1 | 1,0 | 0,9 | 1,0 | 0,9 | 0,9 | 0,9 | 1,1 | 1,2 | 2,0 | 2,8 | 1,0 | 0,9 | 1,2 | 1,0 | 7,6 | 1,5 | 1,2 | 1,1 | 1,5 | 1,1 | 1,0 | 1,0 |
| Itgax | 1,0 | 2,3 | 0,8 | 0,6 | 1,0 | 1,0 | 1,0 | 1,0 | 1,0 | 1,0 | 1,0 | 1,0 | 1,2 | 1,2 | 0,5 | 0,4 | 1,9 | 5,1 | 1,0 | 0,8 | 0,7 | 0,6 |
| Itgb1bp2 | 1,0 | 1,0 | 1,0 | 1,0 | 0,7 | 0,8 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Itgb6 | 0,3 | 0,3 | 1,0 | 0,9 | 0,7 | 0,8 | 1,0 | 0,5 | 1,0 | 1,0 | 0,9 | 0,8 | 1,0 | 1,0 | 1,0 | 1,0 | 0,6 | 0,8 | 1,2 | 1,3 | 1,3 | 0,9 |
| Itln1 | 1,0 | 1,0 | 0,1 | 0,0 | 1,0 | 1,0 | 1,0 | 1,0 | 1,0 | 1,0 | 1,0 | 1,0 | 1,0 | 1,0 | 1,0 | 1,0 | 1,5 | 1,0 | 0,6 | 0,8 | 0,7 | 0,6 |
| Jph1 | 1,0 | 1,0 | 1,0 | 1,0 | 0,9 | 0,6 | 0,9 | 1,1 | 1,8 | 1,0 | 1,0 | 1,0 | 1,4 | 1,4 | 0,9 | 0,9 | 0,7 | 0,9 | 1,0 | 1,0 | 1,0 | 1,0 |
| Jph2 | 1,0 | 1,0 | 0,9 | 1,0 | 0,7 | 0,5 | 1,0 | 1,1 | 1,0 | 1,0 | 0,7 | 0,7 | 1,0 | 1,0 | 1,0 | 2,0 | 1,1 | 0,9 | 1,1 | 1,1 | 0,8 | 1,1 |
| Jsrp1 | 1,0 | 1,0 | 1,0 | 1,0 | 0,6 | 0,7 | 1,0 | 1,0 | 1,0 | 1,0 | 1,0 | 1,0 | 1,0 | 1,0 | 1,0 | 1,0 | 0,9 | 1,2 | 1,0 | 1,0 | 1,0 | 1,0 |
| Kcna7 | 1,0 | 1,0 | 1,0 | 1,0 | 0,5 | 0,4 | 0,9 | 0,9 | 1,0 | 1,0 | 1,0 | 1,0 | 1,0 | 1,0 | 1,0 | 1,0 | 1,0 | 1,0 | 1,0 | 1,0 | 1,0 | 1,0 |
| Kcnc1 | 1,0 | 1,0 | 1,0 | 1,0 | 1,0 | 0,8 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Kcnc4 | 1,0 | 1,0 | 1,0 | 1,0 | 1,0 | 0,7 | 1,0 | 1,0 | 1,0 | 1,0 | 1,0 | 1,0 | 1,0 | 1,0 | 6,0 | 1,0 | 1,0 | 1,0 | 1,0 | 1,0 | 1,0 | 1,0 |
| Kcne3 | 0,6 | 0,7 | 0,9 | 0,9 | 1,0 | 1,0 | 1,0 | 1,0 | 1,6 | 2,6 | 0,9 | 1,3 | 1,0 | 1,0 | 1,0 | 1,1 | 1,0 | 0,7 | 1,1 | 0,9 | 0,7 | 0,7 |
| Kcng4 | 1,0 | 1,0 | 1,0 | 1,0 | 1,0 | 0,8 | 1,0 | 1,0 | 1,0 | 1,0 | 1,0 | 1,0 | 1,0 | 1,0 | 1,0 | 1,0 | 2,1 | 1,2 | 1,0 | 1,0 | 1,0 | 1,0 |
| Kcnh3 | 1,0 | 1,0 | 0,7 | 1,1 | 1,0 | 1,0 | 1,0 | 1,0 | 1,0 | 1,0 | 1,0 | 1,0 | 0,9 | 0,7 | 11,0 | 1,0 | 1,0 | 1,0 | 1,0 | 1,0 | 0,9 | 1,3 |
| Kcnj11 | 1,0 | 1,0 | 1,0 | 0,9 | 0,7 | 0,7 | 1,1 | 1,1 | 1,0 | 1,0 | 1,0 | 1,0 | 1,0 | 1,0 | 5,2 | 1,0 | 0,9 | 1,0 | 1,0 | 1,0 | 1,0 | 1,0 |
| Kcnj12 | 0,7 | 0,7 | 1,0 | 1,0 | 1,0 | 0,8 | 1,4 | 1,2 | 1,0 | 1,0 | 1,0 | 1,0 | 1,0 | 1,0 | 1,7 | 1,0 | 1,0 | 0,9 | 1,0 | 1,0 | 1,0 | 1,0 |
| Kcnj15 | 1,1 | 1,0 | 1,0 | 1,0 | 1,0 | 1,0 | 1,0 | 1,0 | 1,0 | 1,0 | 1,0 | 1,3 | 1,9 | 1,8 | 1,1 | 1,0 | 0,9 | 1,0 | 1,0 | 1,0 | 1,0 | 1,0 |
| Kcnk1 | 1,7 | 1,8 | 1,0 | 0,9 | 1,0 | 0,9 | 1,0 | 1,0 | 1,0 | 1,0 | 0,8 | 0,6 | 2,2 | 1,8 | 8,4 | 4,9 | 1,1 | 1,1 | 1,4 | 1,2 | 1,1 | 1,1 |
| Kcnk16 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,7 | 1,2 | 1,0 | 1,0 | 1,0 | 1,0 |
| Ctrb1 | 1,0 | 1,0 | 1,0 | 1,0 | 1,6 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Kctd1 | 1,3 | 1,4 | 1,0 | 1,3 | 1,1 | 1,0 | 1,1 | 0,8 | 1,0 | 1,0 | 1,4 | 1,1 | 1,2 | 1,3 | 5,4 | 1,0 | 1,0 | 0,9 | 1,0 | 1,0 | 1,0 | 1,0 |
| Kdelr3 | 0,5 | 1,2 | 0,8 | 0,8 | 0,8 | 0,9 | 0,7 | 0,8 | 1,0 | 1,0 | 0,8 | 0,9 | 1,0 | 0,9 | 0,6 | 0,8 | 1,2 | 1,3 | 0,8 | 1,2 | 0,8 | 0,8 |
| Kel | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Kera | 1,0 | 1,0 | 1,0 | 1,0 | 0,9 | 0,8 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Kif20b | 0,2 | 0,2 | 0,8 | 0,9 | 1,0 | 1,0 | 1,0 | 1,0 | 1,0 | 1,0 | 1,0 | 1,0 | 0,4 | 0,6 | 1,0 | 1,0 | 0,9 | 1,1 | 1,1 | 0,7 | 1,1 | 1,3 |
| Kiss1 | 1,0 | 0,7 | 1,5 | 2,0 | 1,0 | 1,0 | 1,0 | 1,0 | 1,0 | 0,9 | 0,1 | 0,3 | 1,0 | 1,0 | 1,0 | 1,0 | 1,0 | 1,0 | 1,5 | 1,0 | 1,0 | 0,8 |
| Klf1 | 1,4 | 1,0 | 1,0 | 1,0 | 1,0 | 1,0 | 1,0 | 1,0 | 0,5 | 0,6 | 1,0 | 1,0 | 1,0 | 1,0 | 1,0 | 1,0 | 1,0 | 1,0 | 1,0 | 1,0 | 1,0 | 1,0 |
| Klhl30 | 1,0 | 1,0 | 0,9 | 0,7 | 0,8 | 0,6 | 0,8 | 0,8 | 1,0 | 1,0 | 1,0 | 1,0 | 1,0 | 1,0 | 1,0 | 1,0 | 1,0 | 1,0 | 1,0 | 1,0 | 1,0 | 1,0 |
| Klhl31 | 1,0 | 1,0 | 1,0 | 1,0 | 0,6 | 0,6 | 0,9 | 0,8 | 1,0 | 1,0 | 1,0 | 1,0 | 1,0 | 1,0 | 1,0 | 1,0 | 1,0 | 1,0 | 1,0 | 1,0 | 1,0 | 1,0 |
| Klhl34 | 1,0 | 1,0 | 1,0 | 1,0 | 1,1 | 0,6 | 0,9 | 0,7 | 1,0 | 1,0 | 1,0 | 1,0 | 1,0 | 1,0 | 5,4 | 1,0 | 1,0 | 1,0 | 1,0 | 1,0 | 1,0 | 1,0 |
| Klhl40 | 1,0 | 1,0 | 1,0 | 1,0 | 0,6 | 0,5 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Klhl41 | 1,0 | 1,0 | 1,0 | 1,0 | 0,7 | 0,7 | 0,9 | 0,9 | 1,0 | 1,0 | 1,0 | 1,0 | 1,0 | 1,0 | 1,0 | 1,0 | 0,7 | 1,3 | 1,0 | 1,0 | 1,0 | 1,0 |
| Klhl6 | 1,2 | 1,3 | 0,7 | 0,3 | 1,0 | 0,9 | 1,0 | 0,9 | 1,3 | 0,8 | 0,8 | 1,6 | 0,9 | 1,1 | 0,7 | 0,9 | 1,2 | 1,8 | 1,0 | 0,7 | 0,9 | 0,9 |
| Klk10 | 1,0 | 1,0 | 1,0 | 1,0 | 1,1 | 1,7 | 1,0 | 1,0 | 1,0 | 1,0 | 1,0 | 1,0 | 1,3 | 1,0 | 1,0 | 1,0 | 0,9 | 1,1 | 1,0 | 1,0 | 1,0 | 1,0 |
| Klk14 | 1,0 | 1,0 | 1,0 | 1,0 | 1,0 | 1,0 | 1,0 | 1,0 | 1,0 | 1,0 | 1,0 | 1,0 | 1,1 | 1,0 | 1,0 | 1,0 | 0,9 | 1,4 | 1,0 | 1,0 | 1,0 | 1,0 |
| Klk1b1 | 1,0 | 1,0 | 1,0 | 1,0 | 1,0 | 1,0 | 1,0 | 1,0 | 1,0 | 1,0 | 0,3 | 0,3 | 1,0 | 1,0 | 1,0 | 1,0 | 1,0 | 1,0 | 1,0 | 1,0 | 1,0 | 1,0 |
| Klk1b21 | 1,5 | 0,9 | 1,0 | 1,0 | 1,0 | 1,0 | 1,0 | 1,0 | 1,0 | 1,0 | 0,4 | 0,4 | 1,0 | 1,0 | 1,0 | 1,0 | 1,0 | 1,0 | 1,0 | 1,0 | 1,0 | 1,0 |
| Klk1b24 | 1,0 | 1,1 | 1,0 | 1,0 | 1,0 | 1,0 | 1,0 | 1,0 | 1,0 | 1,0 | 0,4 | 0,3 | 1,0 | 1,0 | 1,0 | 1,0 | 1,0 | 1,0 | 1,0 | 1,0 | 1,0 | 1,0 |
| Klk1b26 | 2,5 | 1,0 | 1,0 | 1,0 | 1,0 | 0,8 | 1,0 | 1,1 | 2,2 | 1,0 | 0,5 | 0,5 | 2,1 | 7,2 | 1,0 | 5,4 | 1,0 | 5,5 | 1,0 | 1,0 | 1,0 | 1,0 |
| Klk1b27 | 2,1 | 1,0 | 1,0 | 1,0 | 1,0 | 1,0 | 1,0 | 1,0 | 1,0 | 1,0 | 0,2 | 0,2 | 1,1 | 1,0 | 1,0 | 1,0 | 1,0 | 1,0 | 1,0 | 1,0 | 1,0 | 1,0 |
| Klk1b7-ps | 1,0 | 1,0 | 1,0 | 1,0 | 1,0 | 1,0 | 1,0 | 1,0 | 1,0 | 1,0 | 0,4 | 0,2 | 1,0 | 1,0 | 1,0 | 1,0 | 1,0 | 1,0 | 1,0 | 1,0 | 1,0 | 1,0 |
| Klk1b8 | 1,0 | 1,0 | 1,0 | 1,0 | 1,0 | 1,0 | 1,0 | 0,6 | 1,0 | 1,0 | 0,2 | 0,2 | 1,0 | 1,1 | 1,0 | 1,0 | 1,0 | 1,2 | 1,0 | 1,0 | 1,0 | 1,0 |
| Klk1b9 | 1,4 | 1,0 | 1,0 | 1,0 | 1,0 | 0,7 | 1,0 | 1,0 | 1,0 | 1,0 | 0,2 | 0,3 | 1,0 | 2,8 | 1,0 | 1,0 | 1,0 | 1,4 | 1,0 | 1,0 | 1,0 | 1,0 |
| Klrb1a | 1,0 | 1,0 | 1,0 | 1,0 | 1,0 | 1,0 | 1,0 | 1,0 | 1,0 | 1,0 | 1,0 | 1,0 | 1,0 | 1,0 | 7,6 | 1,0 | 1,0 | 1,0 | 1,0 | 1,0 | 1,0 | 1,0 |
| Klrb1c | 1,0 | 1,0 | 1,0 | 1,0 | 1,0 | 1,0 | 1,0 | 1,0 | 1,0 | 1,0 | 1,0 | 1,0 | 1,8 | 3,4 | 30,0 | 1,0 | 1,0 | 1,0 | 1,0 | 1,0 | 1,0 | 1,0 |
| Kng1 | 1,3 | 1,5 | 1,0 | 1,0 | 1,0 | 1,0 | 1,0 | 1,0 | 1,0 | 1,1 | 1,0 | 1,0 | 1,0 | 1,0 | 1,0 | 1,0 | 5,1 | 0,3 | 1,0 | 1,0 | 1,0 | 1,0 |
| Krt13 | 1,0 | 1,0 | 1,0 | 1,0 | 1,1 | 1,0 | 1,0 | 1,0 | 1,0 | 1,0 | 1,0 | 1,0 | 1,0 | 1,0 | 1,0 | 1,0 | 1,1 | 1,0 | 1,0 | 1,0 | 1,0 | 1,0 |
| Krt14 | 1,0 | 1,0 | 1,0 | 1,0 | 1,0 | 2,3 | 1,0 | 1,0 | 1,0 | 1,0 | 0,9 | 0,8 | 1,7 | 1,0 | 7,5 | 1,0 | 1,0 | 1,6 | 1,0 | 1,0 | 1,0 | 1,0 |
| Krt16 | 1,0 | 1,0 | 1,0 | 1,0 | 2,0 | 13,9 | 1,0 | 1,0 | 1,0 | 1,0 | 1,0 | 1,0 | 1,8 | 0,9 | 1,0 | 1,0 | 1,3 | 6,1 | 1,0 | 1,0 | 1,0 | 1,0 |
| Krt18 | 1,2 | 1,9 | 0,8 | 0,8 | 1,0 | 1,1 | 1,7 | 1,0 | 1,1 | 1,1 | 1,0 | 0,8 | 1,2 | 1,7 | 6,2 | 6,4 | 1,0 | 1,0 | 1,0 | 1,2 | 0,9 | 0,9 |
| Krt23 | 1,0 | 1,4 | 1,5 | 1,2 | 1,2 | 1,7 | 1,0 | 1,0 | 0,8 | 0,5 | 1,1 | 1,0 | 2,1 | 1,0 | 13,9 | 1,0 | 0,9 | 1,0 | 1,2 | 1,2 | 1,2 | 0,9 |
| Krt4 | 1,0 | 1,0 | 1,0 | 1,0 | 1,2 | 1,3 | 1,0 | 1,0 | 1,0 | 1,0 | 1,0 | 1,0 | 1,0 | 1,0 | 1,0 | 1,0 | 1,3 | 1,1 | 1,0 | 1,0 | 1,0 | 1,0 |
| Krt5 | 1,0 | 0,7 | 1,0 | 1,0 | 1,0 | 1,0 | 1,0 | 1,0 | 1,0 | 1,0 | 0,7 | 0,8 | 1,7 | 1,7 | 9,4 | 1,0 | 1,0 | 1,2 | 1,0 | 1,0 | 1,0 | 1,0 |
| Krt6a | 1,0 | 1,0 | 1,0 | 1,0 | 1,0 | 1,4 | 1,0 | 1,0 | 1,0 | 1,0 | 1,0 | 1,0 | 1,4 | 1,0 | 1,0 | 1,0 | 1,2 | 6,0 | 1,0 | 1,0 | 1,0 | 1,0 |
| Krt78 | 1,0 | 1,0 | 1,0 | 1,0 | 1,1 | 0,9 | 1,0 | 1,0 | 1,0 | 1,0 | 1,0 | 1,0 | 2,0 | 1,0 | 1,0 | 1,0 | 0,9 | 1,0 | 1,0 | 1,0 | 1,0 | 1,0 |
| Krtap16-1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Krtap19-1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Krtap19-4 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |

[Fig. 23-23]

292/310

| | | | | | | | | | | | | | | | | | | | | | | |
|---|---|---|---|---|---|---|---|---|---|---|---|---|---|---|---|---|---|---|---|---|---|---|
| *Krtap5-2* | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Krtdap | 1,0 | 1,0 | 1,0 | 1,0 | 1,0 | 1,0 | 1,0 | 1,0 | 1,0 | 1,0 | 1,0 | 1,0 | 2,1 | 1,0 | 1,0 | 1,0 | 0,9 | 1,1 | 1,0 | 1,0 | 1,0 | 1,0 |
| Ky | 1,0 | 1,0 | 1,0 | 1,0 | 0,5 | 0,4 | 1,0 | 0,7 | 1,0 | 1,0 | 1,0 | 1,0 | 1,0 | 1,0 | 1,0 | 1,0 | 1,0 | 1,0 | 1,1 | 1,5 | 1,2 | 2,6 |
| *Kynu* | 4,6 | 9,3 | 1,0 | 1,0 | 1,0 | 1,0 | 1,0 | 1,0 | 0,7 | 0,7 | 1,0 | 1,0 | 1,2 | 0,5 | 1,0 | 1,0 | 1,0 | 1,0 | 0,7 | 0,9 | 1,0 | 1,0 |
| Lad1 | 1,5 | 1,0 | 1,1 | 1,3 | 1,0 | 1,0 | 0,9 | 0,8 | 0,4 | 0,4 | 1,0 | 1,0 | 1,2 | 1,1 | 5,5 | 1,0 | 1,1 | 1,0 | 1,0 | 0,9 | 0,9 | 1,0 |
| Lars2 | 0,9 | 1,6 | 1,0 | 0,4 | 1,0 | 0,6 | 0,8 | 1,1 | 0,8 | 0,4 | 0,8 | 0,7 | 0,8 | 0,7 | 2,1 | 1,2 | 1,1 | 1,0 | 1,0 | 1,4 | 1,0 | 0,7 |
| | Kidney | | Colon | | Eyeball | | Heart | | Liver | | SG | | Thymus | | Adipose | | Stomach | | Jejunum | | Ileum | |
| | E | M | E | M | E | M | E | M | E | M | E | M | E | M | E | M | E | M | E | M | E | M |
| Lbx1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Lce3a | 1,0 | 1,0 | 1,0 | 1,0 | 1,8 | 1,9 | 1,0 | 1,0 | 1,0 | 1,0 | 1,0 | 1,0 | 1,0 | 1,0 | 1,0 | 1,0 | 1,1 | 1,3 | 1,0 | 1,0 | 1,0 | 1,0 |
| Lce3b | 1,0 | 1,0 | 3,3 | 1,0 | 1,0 | 1,0 | 1,0 | 1,0 | 1,0 | 1,0 | 1,0 | 1,0 | 1,0 | 1,0 | 1,0 | 1,0 | 0,9 | 1,3 | 1,0 | 1,0 | 1,0 | 1,0 |
| Lce3c | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,1 | 1,0 | 1,0 | 1,0 | 1,0 |
| Lce3e | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,1 | 1,0 | 1,0 | 1,0 | 1,0 |
| Lce3f | 1,0 | 1,0 | 1,0 | 1,0 | 1,1 | 1,5 | 1,0 | 1,0 | 1,0 | 1,0 | 1,0 | 1,0 | 1,0 | 1,0 | 1,0 | 1,0 | 1,1 | 1,1 | 1,0 | 1,0 | 1,0 | 1,0 |
| Lck | 1,0 | 1,0 | 0,8 | 0,6 | 1,0 | 1,0 | 1,0 | 1,0 | 1,0 | 1,0 | 0,7 | 1,3 | 0,8 | 0,8 | 1,1 | 1,0 | 1,0 | 1,0 | 0,9 | 0,7 | 0,7 | 0,6 |
| Lcn10 | 1,0 | 1,0 | 1,0 | 1,0 | 1,0 | 1,0 | 1,0 | 1,0 | 1,0 | 1,0 | 1,0 | 1,0 | 1,0 | 1,0 | 173,3 | 1,0 | 1,0 | 1,0 | 1,0 | 1,0 | 1,0 | 1,0 |
| Lcn12 | 1,0 | 1,0 | 1,0 | 1,0 | 1,0 | 1,0 | 1,0 | 1,0 | 1,0 | 1,0 | 1,0 | 1,0 | 1,0 | 1,0 | 23,2 | 1,0 | 1,0 | 1,0 | 1,0 | 1,0 | 1,0 | 1,0 |
| Lcn2 | 14,5 | 18,7 | 1,7 | 0,8 | 1,6 | 1,6 | 6,5 | 4,3 | 2,2 | 1,3 | 0,2 | 0,1 | 3,0 | 1,1 | 15,3 | 4,6 | 1,0 | 1,9 | 2,9 | 0,8 | 1,5 | 0,8 |
| Lcn5 | 1,0 | 1,0 | 1,0 | 1,0 | 1,0 | 1,0 | 1,0 | 1,0 | 1,0 | 1,0 | 1,0 | 1,0 | 1,0 | 1,0 | 635,9 | 1,0 | 1,0 | 1,0 | 1,0 | 1,0 | 1,0 | 1,0 |
| Lcn6 | 1,0 | 1,0 | 1,0 | 1,0 | 1,0 | 1,0 | 1,0 | 1,0 | 1,0 | 1,0 | 1,0 | 1,0 | 1,0 | 1,0 | 35,2 | 1,0 | 1,0 | 1,0 | 1,0 | 1,0 | 1,0 | 1,0 |
| Lcn8 | 1,0 | 1,0 | 1,0 | 1,0 | 1,0 | 1,0 | 1,0 | 1,0 | 1,0 | 1,0 | 1,0 | 1,0 | 1,0 | 1,0 | 599,2 | 1,0 | 1,0 | 1,0 | 1,0 | 1,0 | 1,0 | 1,0 |
| Lcn9 | 1,0 | 1,0 | 1,0 | 1,0 | 1,0 | 1,0 | 1,0 | 1,0 | 1,0 | 1,0 | 1,0 | 1,0 | 1,0 | 1,0 | 480,9 | 1,0 | 1,0 | 1,0 | 1,0 | 1,0 | 1,0 | 1,0 |
| Lctl | 1,0 | 1,0 | 1,0 | 1,0 | 1,0 | 1,1 | 1,0 | 1,0 | 1,0 | 1,0 | 1,0 | 1,0 | 1,0 | 1,0 | 0,2 | 1,2 | 1,0 | 1,0 | 1,0 | 1,0 | 1,0 | 1,0 |
| Ldb3 | 1,0 | 1,0 | 1,0 | 1,1 | 0,7 | 0,7 | 1,0 | 1,0 | 1,0 | 1,0 | 1,0 | 1,0 | 1,0 | 1,0 | 1,0 | 1,0 | 0,7 | 0,9 | 0,8 | 1,0 | 1,0 | 1,1 |
| Lect1 | 1,0 | 1,0 | 1,0 | 1,0 | 1,0 | 1,0 | 1,4 | 1,2 | 0,1 | 0,2 | 1,0 | 1,0 | 1,2 | 1,0 | 1,0 | 1,0 | 1,0 | 1,0 | 1,0 | 1,0 | 1,0 | 1,0 |
| Lef1 | 1,0 | 1,0 | 0,6 | 1,0 | 0,9 | 1,0 | 1,0 | 1,0 | 1,0 | 1,0 | 1,0 | 1,0 | 0,8 | 0,9 | 5,0 | 1,0 | 1,0 | 1,0 | 1,0 | 1,0 | 1,0 | 1,0 |
| Lelp1 | 1,0 | 1,0 | 1,0 | 1,0 | 1,0 | 1,0 | 1,0 | 1,0 | 1,0 | 1,0 | 1,0 | 1,0 | 1,0 | 1,0 | 6,5 | 1,0 | 1,0 | 1,0 | 1,0 | 1,0 | 1,0 | 1,0 |
| Lgals7 | 1,0 | 1,0 | 1,0 | 1,0 | 1,6 | 0,8 | 1,0 | 1,1 | 1,0 | 1,0 | 1,0 | 1,1 | 1,3 | 1,1 | 1,3 | 1,7 | 1,0 | 1,0 | 1,0 | 1,0 | 1,0 | 1,0 |
| Lhfpl2 | 1,6 | 2,4 | 1,5 | 1,5 | 1,0 | 0,9 | 1,3 | 0,9 | 1,1 | 1,1 | 0,9 | 0,7 | 1,3 | 1,1 | 0,4 | 0,3 | 0,9 | 1,8 | 1,1 | 1,1 | 1,3 | 1,1 |
| Lingo1 | 1,0 | 1,0 | 1,0 | 1,0 | 1,0 | 0,9 | 1,1 | 0,8 | 1,0 | 1,0 | 1,0 | 1,0 | 1,4 | 1,0 | 33,4 | 1,0 | 1,0 | 1,0 | 1,0 | 1,0 | 1,0 | 1,0 |
| Lipf | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Lipg | 1,0 | 1,0 | 1,2 | 1,0 | 1,0 | 0,7 | 1,0 | 1,0 | 1,7 | 1,2 | 1,0 | 1,0 | 1,0 | 1,9 | 14,6 | 1,4 | 0,8 | 0,6 | 1,5 | 0,7 | 1,5 | 1,5 |
| Lipi | 1,0 | 1,0 | 1,0 | 1,0 | 1,0 | 1,0 | 1,0 | 1,0 | 1,0 | 1,0 | 1,0 | 1,0 | 1,0 | 1,0 | 6,7 | 1,0 | 1,0 | 1,0 | 1,0 | 1,0 | 1,0 | 1,0 |
| Lmod2 | 1,0 | 1,0 | 1,0 | 1,0 | 0,7 | 0,7 | 0,9 | 1,1 | 1,0 | 0,9 | 1,3 | 0,9 | 1,0 | 1,0 | 1,0 | 1,0 | 1,0 | 1,0 | 1,0 | 1,0 | 1,0 | 1,0 |
| Lmod3 | 1,0 | 1,0 | 1,0 | 1,0 | 0,7 | 0,6 | 0,7 | 0,8 | 1,0 | 1,0 | 1,2 | 0,9 | 1,0 | 0,9 | 1,0 | 1,0 | 1,0 | 1,0 | 1,0 | 1,0 | 1,0 | 1,0 |
| Lor | 1,0 | 1,0 | 1,0 | 0,7 | 0,7 | 0,6 | 1,0 | 1,0 | 1,6 | 1,2 | 1,0 | 1,0 | 3,3 | 0,9 | 1,8 | 0,5 | 1,1 | 1,0 | 1,0 | 1,9 | 1,0 | 0,6 |
| Loxl2 | 1,4 | 2,4 | 1,3 | 0,8 | 0,9 | 0,9 | 0,7 | 0,6 | 0,9 | 0,9 | 0,6 | 0,8 | 0,9 | 0,9 | 0,4 | 0,5 | 1,7 | 1,8 | 1,2 | 1,0 | 1,0 | 1,5 |
| Lpl | 0,3 | 0,3 | 0,6 | 1,1 | 0,7 | 0,9 | 1,1 | 1,3 | 0,7 | 1,0 | 0,7 | 1,3 | 1,3 | 2,2 | 0,5 | 0,8 | 0,7 | 1,1 | 0,6 | 0,7 | 0,4 | 0,9 |
| Lrcol1 | 1,0 | 1,0 | 1,0 | 1,0 | 1,0 | 1,0 | 1,0 | 1,0 | 1,0 | 1,0 | 1,0 | 1,0 | 1,0 | 1,0 | 42,3 | 1,0 | 1,0 | 1,0 | 1,0 | 1,0 | 1,0 | 1,0 |
| Lrrc16a | 0,9 | 1,4 | 0,9 | 1,0 | 1,0 | 1,2 | 0,9 | 1,0 | 0,1 | 0,2 | 1,0 | 1,0 | 1,2 | 1,4 | 2,1 | 1,1 | 1,1 | 1,1 | 1,1 | 1,1 | 1,1 | 1,1 |
| Lrrc26 | 1,5 | 0,9 | 1,0 | 1,1 | 0,9 | 0,9 | 1,0 | 1,0 | 1,0 | 1,0 | 1,3 | 1,1 | 1,7 | 1,0 | 2,0 | 1,0 | 1,0 | 0,9 | 1,2 | 1,1 | 1,0 | 1,0 |
| Lrrc30 | 1,0 | 1,0 | 1,0 | 1,0 | 1,1 | 0,5 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Lst1 | 1,0 | 2,3 | 0,4 | 0,3 | 1,1 | 1,0 | 1,2 | 0,9 | 0,4 | 1,1 | 0,7 | 0,5 | 1,6 | 0,8 | 0,4 | 0,5 | 1,3 | 0,3 | 0,4 | 0,6 | 0,7 | 1,0 |
| Ltb | 1,0 | 0,6 | 0,5 | 0,3 | 1,0 | 1,0 | 1,0 | 0,8 | 0,7 | 0,5 | 0,8 | 1,6 | 1,2 | 0,8 | 0,5 | 1,3 | 1,2 | 0,8 | 1,0 | 0,9 | 0,8 | 0,5 |
| Ltf | 1,0 | 1,0 | 1,0 | 1,0 | 1,2 | 1,1 | 1,0 | 1,0 | 1,0 | 1,0 | 1,0 | 1,0 | 2,8 | 0,8 | 2,2 | 1,0 | 0,8 | 1,9 | 1,0 | 1,0 | 1,0 | 1,0 |
| Ly6d | 1,2 | 0,7 | 0,4 | 0,3 | 1,0 | 1,0 | 0,9 | 1,1 | 4,0 | 1,9 | 0,7 | 0,7 | 1,0 | 1,1 | 1,2 | 1,5 | 1,1 | 1,1 | 0,9 | 1,0 | 1,2 | 0,8 |
| Ly6g5b | 1,0 | 1,0 | 1,1 | 1,3 | 1,7 | 1,1 | 1,0 | 1,0 | 1,0 | 1,0 | 1,0 | 1,0 | 1,0 | 1,1 | 557,5 | 1,0 | 1,0 | 1,2 | 1,1 | 1,0 | 1,4 | 0,9 |
| Ly6g5c | 1,0 | 1,0 | 1,0 | 1,0 | 1,0 | 1,0 | 1,0 | 1,0 | 1,0 | 1,0 | 1,0 | 1,0 | 1,0 | 1,0 | 179,6 | 1,0 | 1,0 | 1,0 | 1,0 | 1,0 | 1,0 | 1,0 |
| Lypd8 | 1,0 | 1,0 | 1,1 | 1,0 | 1,5 | 1,0 | 0,7 | 1,0 | 1,0 | 1,0 | 1,0 | 1,0 | 1,1 | 0,6 | 284,6 | 0,8 | 0,7 | 1,1 | 1,5 | 1,1 | 1,1 | 0,9 |
| Lyz1 | 1,0 | 2,8 | 0,8 | 0,6 | 0,5 | 0,9 | 1,1 | 1,3 | 1,0 | 1,0 | 0,5 | 0,8 | 1,9 | 1,2 | 0,5 | 0,6 | 1,1 | 1,5 | 0,8 | 1,1 | 1,2 | 1,0 |
| Lyz2 | 1,3 | 6,0 | 1,1 | 0,5 | 0,9 | 0,9 | 0,9 | 1,1 | 0,9 | 1,2 | 0,7 | 0,9 | 1,3 | 1,0 | 0,5 | 0,7 | 1,1 | 3,1 | 0,9 | 0,7 | 0,9 | 0,7 |
| Mafa | 1,0 | 1,0 | 1,0 | 1,0 | 0,9 | 0,3 | 0,9 | 1,0 | 1,0 | 1,0 | 1,0 | 1,0 | 1,1 | 1,0 | 1,0 | 1,0 | 1,0 | 1,0 | 1,0 | 1,0 | 1,0 | 1,0 |
| Mafb | 1,2 | 1,5 | 1,4 | 1,0 | 0,9 | 0,8 | 1,1 | 1,2 | 0,7 | 0,8 | 0,6 | 0,7 | 1,9 | 1,3 | 0,7 | 0,7 | 1,0 | 1,8 | 1,1 | 1,0 | 1,0 | 1,4 |
| Mal | 1,1 | 1,3 | 1,9 | 2,0 | 1,0 | 1,3 | 0,8 | 0,9 | 1,0 | 1,0 | 1,0 | 1,0 | 1,0 | 1,0 | 15,9 | 3,7 | 0,8 | 0,8 | 1,2 | 1,2 | 0,5 | 0,5 |
| Malat1 | 1,6 | 1,3 | 0,8 | 0,8 | 1,2 | 0,8 | 1,0 | 1,3 | 1,7 | 0,3 | 0,8 | 1,0 | 0,6 | 0,7 | 0,9 | 0,8 | 0,8 | 1,0 | 0,7 | 0,9 | 0,9 | 1,2 |
| Map3k7cl | 0,6 | 0,6 | 1,0 | 1,0 | 0,5 | 0,5 | 1,0 | 0,7 | 1,0 | 1,0 | 1,0 | 1,0 | 0,9 | 1,1 | 0,9 | 0,5 | 1,0 | 1,1 | 1,0 | 1,0 | 1,0 | 1,0 |
| Mat1a | 1,0 | 1,0 | 0,7 | 1,0 | 1,0 | 1,0 | 1,0 | 1,0 | 1,2 | 0,9 | 1,0 | 1,0 | 1,0 | 1,0 | 1,0 | 1,0 | 8,9 | 0,2 | 1,0 | 1,0 | 1,0 | 1,0 |
| Mb | 1,0 | 1,0 | 0,4 | 1,0 | 0,8 | 0,8 | 1,0 | 1,1 | 1,0 | 1,0 | 1,0 | 1,0 | 0,4 | 2,2 | 2,0 | 3,0 | 0,4 | 1,2 | 1,0 | 1,0 | 1,0 | 1,0 |
| *Mbnl3* | 1,0 | 1,0 | 1,0 | 0,9 | 1,2 | 1,3 | 1,0 | 1,0 | 1,0 | 1,0 | 1,4 | 1,4 | 0,6 | 0,8 | 7,7 | 1,0 | 1,1 | 0,9 | 1,3 | 0,8 | 1,1 | 1,0 |
| Mboat1 | 1,0 | 1,4 | 0,9 | 0,9 | 1,1 | 1,2 | 1,0 | 1,0 | 1,0 | 1,0 | 0,7 | 0,7 | 1,1 | 1,0 | 5,5 | 1,2 | 1,0 | 1,0 | 1,5 | 1,0 | 1,0 | 1,0 |
| Mcm10 | 0,7 | 0,9 | 1,0 | 0,6 | 1,0 | 1,1 | 1,0 | 1,0 | 0,1 | 0,3 | 1,0 | 1,0 | 0,5 | 0,8 | 1,0 | 1,0 | 1,1 | 1,3 | 1,0 | 1,0 | 1,0 | 0,8 |
| *Mcpt1* | 1,0 | 1,0 | 0,0 | 0,2 | 1,0 | 1,0 | 1,0 | 1,0 | 1,0 | 1,0 | 1,0 | 1,0 | 1,0 | 1,0 | 1,0 | 1,0 | 2,8 | 1,1 | 0,9 | 1,2 | 0,5 | 0,2 |
| Mcpt2 | 1,0 | 1,0 | 0,2 | 0,4 | 1,0 | 1,0 | 1,0 | 1,0 | 1,0 | 1,0 | 1,0 | 1,0 | 1,0 | 1,0 | 1,0 | 1,0 | 2,2 | 1,1 | 1,0 | 1,0 | 0,3 | 0,4 |
| Mdk | 0,4 | 0,8 | 1,1 | 1,1 | 0,9 | 1,0 | 0,7 | 1,0 | 1,0 | 1,0 | 0,4 | 0,9 | 1,6 | 1,5 | 1,2 | 5,0 | 0,9 | 1,4 | 1,5 | 0,5 | 0,9 | 0,7 |
| *Mef2b* | 1,0 | 1,0 | 1,0 | 0,5 | 1,0 | 0,9 | 1,0 | 1,0 | 1,0 | 1,0 | 1,0 | 1,0 | 1,0 | 1,4 | 1,0 | 1,0 | 1,0 | 1,0 | 1,0 | 1,0 | 0,8 | 0,9 |
| Megf9 | 1,7 | 1,4 | 1,0 | 0,9 | 1,3 | 1,1 | 0,8 | 0,7 | 2,1 | 1,0 | 0,9 | 1,1 | 0,6 | 0,9 | 0,6 | 0,3 | 0,7 | 1,1 | 1,0 | 0,5 | 1,0 | 3,7 |
| Meox1 | 0,9 | 1,1 | 0,8 | 0,9 | 0,8 | 1,1 | 0,5 | 0,6 | 1,0 | 1,0 | 0,8 | 0,7 | 1,7 | 1,2 | 0,3 | 0,4 | 0,8 | 1,0 | 0,9 | 0,7 | 0,7 | 1,0 |
| Mesp1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Mesp2 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,9 | 0,8 | 1,0 | 1,0 | 1,0 | 1,0 |
| Mest | 1,0 | 0,9 | 0,8 | 1,8 | 0,9 | 1,0 | 0,4 | 0,4 | 1,0 | 0,9 | 1,0 | 1,0 | 1,0 | 1,0 | 0,3 | 0,5 | 0,9 | 0,8 | 1,0 | 1,0 | 1,0 | 1,0 |
| Mettl21c | 1,0 | 1,0 | 1,0 | 1,0 | 1,0 | 1,3 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Mfrp | 1,3 | 1,0 | 1,0 | 1,0 | 8,0 | 0,5 | 1,0 | 1,0 | 1,0 | 1,0 | 1,0 | 1,0 | 1,0 | 1,0 | 11,7 | 1,0 | 1,0 | 1,0 | 1,0 | 1,0 | 1,0 | 1,0 |
| Mfsd2a | 0,5 | 0,6 | 0,9 | 0,7 | 1,1 | 1,2 | 1,0 | 1,0 | 1,9 | 2,5 | 1,0 | 1,0 | 1,0 | 1,0 | 11,0 | 1,0 | 1,1 | 0,9 | 0,7 | 0,6 | 2,0 | 1,9 |
| Mid1 | 0,2 | 0,6 | 0,3 | 0,8 | 0,6 | 0,6 | 0,3 | 0,8 | 0,1 | 0,7 | 0,2 | 0,8 | 0,3 | 0,6 | 0,5 | 0,6 | 0,5 | 0,8 | 0,5 | 0,7 | 0,4 | 0,8 |
| Amy2a5 | 1,8 | 2,0 | 3,8 | 0,3 | 1,0 | 0,2 | 1,0 | 1,2 | 0,3 | 0,6 | 1,1 | 0,7 | 0,6 | 0,3 | 4,4 | 2,1 | 0,4 | 1,2 | 67,1 | 2,1 | 0,5 | 1,0 |
| Kcnk9 | 1,0 | 1,0 | 1,0 | 1,0 | 0,0 | 1,0 | 1,0 | 1,0 | 1,0 | 1,0 | 1,0 | 1,0 | 1,0 | 1,0 | 1,0 | 6,5 | 1,0 | 1,0 | 1,0 | 7,4 | 1,0 | 1,0 |
| Mlf1 | 1,0 | 1,0 | 1,0 | 1,0 | 0,7 | 0,9 | 1,0 | 1,0 | 1,0 | 1,0 | 1,0 | 1,0 | 1,0 | 1,0 | 1,3 | 1,6 | 1,0 | 1,0 | 1,0 | 1,0 | 1,0 | 1,0 |
| Mmp12 | 1,0 | 6,6 | 1,0 | 1,0 | 1,0 | 1,0 | 1,0 | 1,0 | 1,0 | 1,0 | 1,0 | 1,0 | 1,0 | 1,0 | 0,3 | 0,2 | 4,4 | 14,6 | 1,0 | 1,0 | 1,0 | 1,0 |
| Mmp7 | 1,0 | 3,0 | 1,6 | 1,2 | 1,0 | 1,0 | 1,0 | 1,0 | 1,0 | 1,0 | 1,0 | 1,0 | 1,5 | 1,0 | 1,0 | 6,0 | 1,0 | 1,0 | 0,8 | 0,8 | 1,0 | 0,8 |
| Mptx1 | 1,0 | 1,0 | 0,0 | 0,1 | 1,0 | 1,0 | 1,0 | 1,0 | 1,0 | 1,0 | 1,0 | 1,0 | 1,0 | 1,0 | 1,0 | 0,7 | 1,0 | 1,0 | 1,2 | 1,1 | 0,7 | 0,7 |
| Mptx2 | 1,0 | 1,0 | 0,1 | 0,1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,2 | 1,2 | 1,0 | 1,0 |
| Mpz | 1,0 | 1,0 | 1,0 | 1,0 | 0,1 | 0,5 | 1,0 | 1,0 | 1,0 | 1,0 | 0,5 | 0,8 | 1,0 | 1,0 | 0,8 | 1,0 | 1,0 | 1,1 | 1,0 | 1,0 | 1,0 | 1,0 |
| Mrap | 1,0 | 1,0 | 1,0 | 1,6 | 0,9 | 0,3 | 1,3 | 1,0 | 0,8 | 0,7 | 1,0 | 2,6 | 2,2 | 3,3 | 0,8 | 1,2 | 0,6 | 0,6 | 0,8 | 1,4 | 0,7 | 1,4 |
| Ms4a1 | 1,0 | 1,0 | 0,4 | 0,2 | 1,0 | 1,0 | 1,0 | 1,0 | 1,0 | 1,0 | 0,5 | 2,5 | 1,2 | 1,0 | 1,0 | 1,0 | 1,0 | 1,0 | 0,5 | 0,6 | 0,7 | 0,8 |

[Fig. 23-24]

293/310

| | | | | | | | | | | | | | | | | | | | | | | |
|---|---|---|---|---|---|---|---|---|---|---|---|---|---|---|---|---|---|---|---|---|---|---|
| Msln | 1,0 | 1,0 | 1,0 | 1,0 | 1,0 | 1,0 | 1,4 | 0,7 | 1,0 | 1,0 | 1,0 | 1,0 | 2,1 | 1,6 | 0,9 | 0,8 | 1,0 | 1,0 | 1,0 | 1,0 | 1,0 | 1,0 |
| Mss51 | 1,0 | 1,0 | 1,0 | 1,0 | 1,0 | 1,0 | 1,0 | 1,0 | 1,0 | 1,0 | 1,0 | 1,0 | 0,8 | 0,9 | 1,0 | 1,0 | 1,0 | 1,0 | 1,1 | 0,9 | 1,0 | 1,0 |
| Mstn | 1,0 | 1,0 | 1,0 | 1,0 | 1,0 | 0,5 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Msx1 | 1,0 | 1,0 | 1,0 | 1,0 | 1,1 | 0,6 | 1,3 | 0,8 | 1,0 | 1,0 | 1,0 | 0,8 | 1,9 | 1,5 | 0,9 | 0,9 | 1,0 | 1,0 | 1,0 | 1,0 | 1,3 | 1,3 |
| Mt1 | 1,9 | 2,1 | 1,4 | 3,7 | 1,0 | 1,0 | 1,2 | 1,7 | 2,9 | 5,7 | 1,5 | 1,1 | 1,9 | 1,6 | 1,2 | 2,1 | 1,0 | 1,0 | 1,5 | 2,4 | 1,0 | 1,8 |
| Mt2 | 2,3 | 3,7 | 1,7 | 7,0 | 1,0 | 1,3 | 1,7 | 2,1 | 3,0 | 9,8 | 1,8 | 2,0 | 1,4 | 1,3 | 0,9 | 2,7 | 0,8 | 1,0 | 1,7 | 7,3 | 1,4 | 3,3 |
| Mt3 | 1,0 | 1,3 | 1,9 | 0,8 | 1,0 | 1,0 | 1,3 | 1,1 | 1,0 | 1,0 | 1,0 | 1,0 | 1,0 | 1,0 | 0,6 | 4,0 | 1,0 | 0,4 | 1,0 | 1,0 | 1,0 | 1,0 |
| Mt4 | 1,0 | 1,0 | 1,0 | 1,0 | 1,0 | 1,0 | 1,0 | 1,0 | 1,0 | 1,0 | 1,0 | 1,0 | 1,6 | 1,0 | 1,0 | 1,0 | 0,8 | 1,0 | 1,0 | 1,0 | 1,0 | 1,0 |
| Muc15 | 1,0 | 1,0 | 1,0 | 1,0 | 0,9 | 1,0 | 1,0 | 1,0 | 1,0 | 1,0 | 1,0 | 1,0 | 1,0 | 1,0 | 193,0 | 1,0 | 0,8 | 0,8 | 1,0 | 1,0 | 1,0 | 1,0 |
| Muc5b | 1,0 | 1,0 | 1,0 | 1,0 | 1,0 | 1,0 | 1,0 | 1,0 | 1,0 | 1,0 | 1,0 | 1,0 | 1,0 | 1,0 | 23,3 | 1,0 | 1,0 | 1,0 | 1,0 | 1,0 | 1,0 | 1,0 |
| Mug1 | 1,0 | 1,0 | 1,0 | 1,0 | 1,0 | 1,0 | 1,0 | 1,0 | 0,3 | 0,2 | 1,0 | 1,0 | 1,0 | 1,0 | 1,0 | 1,0 | 0,5 | 0,3 | 1,0 | 1,0 | 1,0 | 1,0 |
| Mup1 | 1,0 | 1,0 | 0,5 | 1,0 | 1,0 | 1,0 | 1,0 | 1,0 | 0,1 | 0,0 | 1,0 | 1,0 | 1,0 | 1,0 | 0,3 | 1,0 | 1,0 | 0,1 | 1,0 | 1,0 | 1,0 | 1,0 |
| Mup10 | 1,0 | 1,0 | 0,3 | 1,0 | 1,0 | 1,0 | 1,0 | 1,0 | 0,1 | 0,0 | 1,0 | 1,0 | 1,0 | 1,0 | 0,4 | 1,0 | 1,1 | 0,2 | 1,0 | 1,0 | 1,0 | 1,0 |
| Mup11 | 1,0 | 1,0 | 1,0 | 1,0 | 1,0 | 1,0 | 1,0 | 1,0 | 0,1 | 0,0 | 1,0 | 1,0 | 1,0 | 1,0 | 1,0 | 1,0 | 1,0 | 0,7 | 1,0 | 1,0 | 1,0 | 1,0 |
| Mup12 | 1,0 | 1,0 | 0,3 | 1,0 | 1,0 | 1,0 | 1,0 | 1,0 | 0,2 | 0,0 | 1,0 | 1,0 | 1,0 | 1,0 | 0,3 | 1,0 | 1,0 | 0,2 | 1,0 | 1,0 | 1,0 | 1,0 |
| Mup13 | 1,0 | 1,0 | 0,4 | 1,0 | 1,0 | 1,0 | 1,0 | 1,0 | 0,2 | 0,0 | 1,0 | 1,0 | 1,0 | 1,0 | 0,2 | 1,0 | 0,7 | 0,1 | 1,0 | 1,0 | 1,0 | 1,0 |
| Mup14 | 1,0 | 1,0 | 0,1 | 1,0 | 1,0 | 1,0 | 1,0 | 1,0 | 0,2 | 0,0 | 1,0 | 1,0 | 1,0 | 1,0 | 0,2 | 0,9 | 1,4 | 0,0 | 1,0 | 1,0 | 1,0 | 1,0 |
| Mup15 | 1,0 | 1,0 | 0,2 | 1,0 | 1,0 | 1,0 | 1,0 | 1,0 | 0,2 | 0,0 | 1,0 | 1,0 | 1,0 | 1,0 | 0,2 | 1,0 | 0,8 | 0,2 | 1,0 | 1,0 | 1,0 | 1,0 |
| Mup16 | 1,0 | 1,0 | 1,0 | 1,0 | 1,0 | 1,0 | 1,0 | 1,0 | 0,2 | 0,0 | 1,0 | 1,0 | 1,0 | 1,0 | 1,0 | 1,0 | 1,0 | 1,0 | 1,0 | 1,0 | 1,0 | 1,0 |
| Mup17 | 1,0 | 1,0 | 1,0 | 1,0 | 1,0 | 1,0 | 1,0 | 1,0 | 0,2 | 0,0 | 1,0 | 1,0 | 1,0 | 1,0 | 1,0 | 1,0 | 1,0 | 1,0 | 1,0 | 1,0 | 1,0 | 1,0 |
| Mup19 | 1,0 | 1,0 | 0,4 | 1,0 | 1,0 | 1,0 | 1,0 | 1,0 | 0,2 | 0,1 | 1,0 | 1,0 | 1,0 | 1,0 | 0,2 | 1,0 | 0,9 | 2,6 | 1,0 | 1,0 | 1,0 | 1,0 |
| | Kidney | | Colon | | Eyeball | | Heart | | Liver | | SG | | Thymus | | Adipose | | Stomach | | Jejunum | | Ileum | |
| | E | M | E | M | E | M | E | M | E | M | E | M | E | M | E | M | E | M | E | M | E | M |
| Mup2 | 1,0 | 1,0 | 0,1 | 1,0 | 1,0 | 1,0 | 1,0 | 1,0 | 0,2 | 0,0 | 1,0 | 1,0 | 1,0 | 1,0 | 0,3 | 1,0 | 0,6 | 0,4 | 1,0 | 1,0 | 1,0 | 1,0 |
| Mup20 | 1,0 | 1,0 | 0,3 | 1,0 | 1,0 | 1,0 | 1,0 | 1,0 | 0,2 | 0,1 | 1,0 | 1,0 | 1,0 | 1,0 | 0,6 | 1,0 | 4,1 | 0,0 | 1,0 | 1,0 | 1,0 | 1,0 |
| Mup21 | 1,0 | 1,0 | 1,0 | 1,0 | 1,0 | 1,0 | 1,0 | 1,0 | 0,2 | 0,0 | 1,0 | 1,0 | 1,0 | 1,0 | 1,0 | 1,0 | 1,0 | 0,8 | 1,0 | 1,0 | 1,0 | 1,0 |
| Mup3 | 1,0 | 1,0 | 0,2 | 1,0 | 1,0 | 1,0 | 1,0 | 1,0 | 0,2 | 0,1 | 1,0 | 1,0 | 1,0 | 1,0 | 0,2 | 0,7 | 6,7 | 0,1 | 1,0 | 1,0 | 1,0 | 1,0 |
| Mup5 | 1,0 | 1,0 | 1,0 | 1,0 | 1,0 | 1,0 | 1,0 | 1,0 | 1,0 | 0,6 | 3,4 | 6,0 | 1,0 | 0,8 | 1,0 | 1,0 | 1,0 | 1,0 | 1,0 | 1,0 | 1,0 | 1,0 |
| Mup7 | 1,0 | 1,0 | 0,2 | 1,0 | 1,0 | 1,0 | 1,0 | 1,0 | 0,2 | 0,0 | 1,0 | 1,0 | 1,0 | 1,0 | 0,2 | 0,7 | 1,1 | 0,0 | 1,0 | 1,0 | 1,0 | 1,0 |
| Mup8 | 1,0 | 1,0 | 0,5 | 1,0 | 1,0 | 1,0 | 1,0 | 1,0 | 0,2 | 0,0 | 1,0 | 1,0 | 1,0 | 1,0 | 0,5 | 1,0 | 0,5 | 0,1 | 1,0 | 1,0 | 1,0 | 1,0 |
| Mup9 | 1,0 | 1,0 | 0,5 | 1,0 | 1,0 | 1,0 | 1,0 | 1,0 | 0,2 | 0,0 | 1,0 | 1,0 | 1,0 | 1,0 | 0,5 | 1,0 | 0,9 | 0,1 | 1,0 | 1,0 | 1,0 | 1,0 |
| Murc | 1,0 | 1,0 | 1,0 | 1,0 | 0,7 | 0,7 | 0,9 | 0,9 | 1,0 | 1,0 | 1,0 | 1,0 | 1,0 | 1,0 | 1,0 | 1,0 | 1,0 | 1,0 | 1,0 | 1,0 | 1,0 | 1,0 |
| Myadml2 | 1,0 | 1,0 | 1,0 | 1,0 | 0,7 | 0,7 | 0,9 | 0,9 | 1,0 | 1,0 | 1,0 | 1,0 | 1,0 | 1,0 | 1,0 | 1,0 | 1,0 | 1,0 | 1,0 | 1,0 | 1,0 | 1,0 |
| Myb | 1,0 | 1,0 | 1,1 | 0,9 | 0,7 | 1,0 | 1,0 | 1,0 | 1,0 | 1,0 | 1,0 | 1,0 | 0,7 | 0,9 | 0,7 | 1,0 | 1,2 | 1,1 | 1,0 | 0,9 | 1,1 | 0,9 |
| Mybl1 | 1,0 | 1,0 | 1,1 | 0,5 | 1,2 | 1,0 | 1,0 | 1,0 | 1,0 | 1,0 | 1,0 | 1,0 | 0,4 | 0,6 | 1,0 | 1,0 | 1,0 | 1,0 | 1,0 | 1,0 | 1,2 | 1,0 |
| Mybpc1 | 1,0 | 1,0 | 1,0 | 1,0 | 0,8 | 0,8 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Mybpc2 | 1,0 | 1,0 | 1,0 | 1,0 | 0,7 | 0,5 | 0,9 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Myf6 | 1,0 | 1,0 | 1,0 | 1,0 | 0,7 | 0,6 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Myh1 | 1,0 | 1,0 | 1,0 | 1,0 | 1,1 | 0,9 | 1,0 | 1,0 | 1,0 | 1,0 | 1,0 | 1,0 | 1,0 | 1,0 | 1,0 | 1,0 | 0,4 | 1,4 | 1,0 | 1,0 | 1,0 | 1,0 |
| Myh2 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Myh4 | 1,0 | 1,0 | 1,0 | 1,0 | 0,6 | 0,4 | 0,8 | 1,0 | 1,0 | 1,0 | 1,8 | 3,2 | 1,0 | 1,0 | 1,0 | 0,9 | 1,0 | 1,0 | 1,0 | 1,0 | 1,0 | 1,0 |
| Myh7 | 1,0 | 1,0 | 1,0 | 1,0 | 0,8 | 1,0 | 2,5 | 1,4 | 1,0 | 1,0 | 1,0 | 1,0 | 1,0 | 1,0 | 1,0 | 1,0 | 1,0 | 1,0 | 1,0 | 1,0 | 1,0 | 1,0 |
| Myh8 | 1,0 | 1,0 | 1,0 | 1,0 | 0,8 | 0,7 | 1,0 | 1,0 | 1,0 | 1,0 | 1,0 | 1,0 | 1,0 | 1,0 | 1,0 | 1,0 | 0,3 | 1,2 | 1,0 | 1,0 | 1,0 | 1,0 |
| Myl1 | 1,0 | 1,0 | 1,6 | 1,0 | 0,6 | 0,7 | 0,6 | 0,6 | 1,0 | 1,0 | 1,0 | 2,2 | 0,9 | 1,0 | 0,6 | 1,1 | 0,7 | 1,9 | 1,0 | 0,8 | 1,0 | 1,1 |
| Myl3 | 1,0 | 1,0 | 1,0 | 1,0 | 0,6 | 1,3 | 1,1 | 1,1 | 1,0 | 1,0 | 1,0 | 1,0 | 1,0 | 1,3 | 1,0 | 1,0 | 0,5 | 0,9 | 1,0 | 1,0 | 1,0 | 1,0 |
| Myl6b | 0,6 | 0,8 | 1,0 | -0,7 | 0,6 | 0,8 | 1,1 | 1,7 | 1,0 | 0,9 | 1,0 | 1,0 | 0,8 | 1,4 | 0,5 | 0,8 | 1,0 | 1,0 | 1,0 | 1,0 | 1,0 | 1,0 |
| Mylk2 | 1,0 | 1,0 | 1,0 | 1,0 | 0,8 | 0,5 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Mylk4 | 1,0 | 1,0 | 1,0 | 1,0 | 0,8 | 0,8 | 1,0 | 1,3 | 1,0 | 1,0 | 1,0 | 1,0 | 1,0 | 1,0 | 1,0 | 1,0 | 1,0 | 1,0 | 1,0 | 1,0 | 1,0 | 1,0 |
| Mylpf | 1,0 | 1,0 | 1,0 | 1,0 | 0,7 | 0,9 | 1,0 | 1,0 | 1,0 | 1,0 | 1,0 | 1,6 | 0,7 | 0,9 | 1,0 | 1,0 | 0,4 | 1,2 | 0,8 | 1,0 | 1,0 | 0,9 |
| Myo18b | 1,0 | 1,0 | 1,0 | 1,0 | 0,8 | 0,7 | 0,9 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Myo3b | 1,3 | 1,3 | 1,0 | 1,0 | 1,0 | 1,0 | 1,0 | 1,0 | 1,0 | 1,0 | 1,0 | 1,0 | 1,0 | 1,0 | 6,7 | 1,0 | 1,0 | 1,0 | 1,0 | 1,0 | 1,0 | 1,0 |
| Myo5b | 1,1 | 0,9 | 1,0 | 1,1 | 1,1 | 1,1 | 0,8 | 0,8 | 1,1 | 1,1 | 1,1 | 1,1 | 1,0 | 1,0 | 12,4 | 1,0 | 0,8 | 0,9 | 1,1 | 1,1 | 1,0 | 1,0 |
| Myom1 | 1,0 | 1,0 | 1,1 | 1,1 | 0,9 | 0,9 | 1,1 | 1,1 | 1,0 | 1,0 | 1,0 | 1,0 | 1,0 | 1,0 | 1,1 | 1,1 | 0,8 | 1,0 | 0,9 | 1,1 | 0,9 | 1,0 |
| Myom2 | 1,0 | 0,8 | 1,0 | 1,0 | 1,0 | 1,0 | 1,6 | 1,7 | 1,0 | 1,0 | 1,0 | 1,0 | 1,0 | 1,0 | 1,6 | 1,0 | 1,0 | 1,0 | 1,0 | 1,0 | 1,0 | 1,0 |
| Myot | 1,0 | 1,0 | 1,0 | 1,0 | 0,8 | 0,8 | 1,2 | 1,3 | 1,0 | 1,0 | 1,0 | 1,0 | 1,0 | 1,0 | 1,0 | 1,0 | 1,0 | 1,0 | 1,0 | 1,0 | 2,5 | 1,0 |
| Myoz1 | 1,0 | 1,0 | 1,0 | 1,0 | 0,8 | 0,6 | 1,0 | 1,0 | 1,0 | 1,0 | 1,0 | 1,1 | 1,0 | 1,0 | 1,0 | 1,0 | 1,0 | 0,8 | 1,0 | 1,0 | 1,0 | 1,0 |
| Myoz2 | 1,0 | 1,0 | 1,0 | 1,0 | 0,8 | 1,1 | 1,0 | 1,2 | 1,0 | 1,0 | 1,0 | 1,0 | 1,0 | 1,0 | 1,0 | 1,0 | 1,0 | 1,0 | 1,0 | 1,0 | 1,0 | 1,0 |
| Myoz3 | 1,0 | 1,0 | 1,0 | 1,0 | 0,6 | 0,5 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Mypn | 1,0 | 1,0 | 1,0 | 1,0 | 0,7 | 0,7 | 1,0 | 1,1 | 1,0 | 1,0 | 1,0 | 1,0 | 1,0 | 1,0 | 1,0 | 1,0 | 1,0 | 1,0 | 1,0 | 1,0 | 1,0 | 1,0 |
| Mzb1 | 1,1 | 1,6 | 0,7 | 0,2 | 1,0 | 1,0 | 0,7 | 1,2 | 1,0 | 1,0 | 0,7 | 1,5 | 1,4 | 1,3 | 1,0 | 1,0 | 1,0 | 1,0 | 1,2 | 1,2 | 1,2 | 0,9 |
| N4bp2 | 1,4 | 0,8 | 0,8 | 1,0 | 1,0 | 1,0 | 0,7 | 0,6 | 1,4 | 0,6 | 0,9 | 1,8 | 0,3 | 0,5 | 1,0 | 1,0 | 0,8 | 1,1 | 0,8 | 0,4 | 1,0 | 1,9 |
| Naip7 | 1,0 | 1,0 | 0,7 | 1,4 | 1,0 | 1,0 | 1,0 | 1,0 | 1,0 | 1,0 | 1,0 | 1,0 | 1,0 | 1,0 | 1,0 | 1,0 | 0,7 | 1,0 | 0,3 | 0,1 | 1,3 | 5,1 |
| Nat8 | 0,8 | 0,5 | 1,0 | 1,0 | 1,3 | 1,7 | 1,0 | 1,0 | 0,2 | 0,1 | 1,3 | 1,3 | 1,0 | 1,0 | 1,0 | 1,0 | 1,9 | 1,0 | 1,1 | 1,2 | 1,4 | 1,2 |
| Nav2 | 2,2 | 1,4 | 1,1 | 1,2 | 0,9 | 0,8 | 1,1 | 1,0 | 1,6 | 1,4 | 1,1 | 1,6 | 1,3 | 1,5 | 7,5 | 1,2 | 0,8 | 1,0 | 0,7 | 0,7 | 0,8 | 1,1 |
| Ncmap | 1,0 | 1,0 | 1,3 | 1,3 | 0,8 | 0,7 | 1,0 | 1,0 | 1,0 | 1,0 | 1,0 | 1,0 | 1,0 | 1,0 | 6,1 | 1,0 | 1,2 | 1,0 | 1,0 | 1,0 | 1,0 | 1,0 |
| Nctc1 | 1,0 | 1,0 | 1,0 | 1,0 | 1,0 | 0,8 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Neat1 | 1,0 | 1,0 | 1,0 | 1,2 | 0,9 | 0,8 | 1,3 | 1,2 | 0,2 | 0,2 | 1,0 | 1,0 | 1,1 | 1,1 | 0,9 | 0,8 | 0,7 | 0,8 | 1,4 | 2,0 | 1,4 | 1,2 |
| Neb | 1,0 | 1,0 | 1,0 | 1,0 | 0,8 | 0,7 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Nell1 | 1,0 | 1,0 | 1,0 | 1,0 | 1,2 | 0,9 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Nexn | 1,1 | 1,0 | 0,8 | 0,8 | 0,9 | 0,9 | 1,0 | 1,1 | 1,0 | 1,0 | 1,1 | 0,7 | 1,0 | 1,0 | 0,9 | 0,8 | 1,0 | 0,8 | 0,9 | 0,9 | 0,7 | 1,2 |
| Nfe2l3 | 1,4 | 1,5 | 1,4 | 1,1 | 1,2 | 1,8 | 1,0 | 1,0 | 1,0 | 1,0 | 0,9 | 0,8 | 1,7 | 1,5 | 8,5 | 1,0 | 1,2 | 1,3 | 1,0 | 1,0 | 1,0 | 1,0 |
| Ngfr | 1,0 | 1,1 | 0,8 | 1,1 | 1,0 | 0,8 | 1,0 | 1,0 | 0,5 | 0,4 | 0,7 | 0,9 | 1,3 | 1,5 | 1,0 | 1,0 | 0,8 | 1,1 | 1,0 | 0,8 | 0,9 | 1,2 |
| Nhsl1 | 1,4 | 1,1 | 0,9 | 1,1 | 1,0 | 0,9 | 1,2 | 0,9 | 1,2 | 0,6 | 1,2 | 1,5 | 0,7 | 0,9 | 5,2 | 0,9 | 0,8 | 1,1 | 0,8 | 0,9 | 0,8 | 1,3 |
| Nkx3-1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Nmrk2 | 1,0 | 1,0 | 1,0 | 1,0 | 1,0 | 0,7 | 1,6 | 0,8 | 1,0 | 1,0 | 1,0 | 1,0 | 1,0 | 1,0 | 1,0 | 1,0 | 1,0 | 1,0 | 1,0 | 1,0 | 1,0 | 1,0 |
| Nnat | 2,0 | 0,7 | 0,8 | 0,8 | 1,0 | 1,0 | 1,1 | 0,6 | 1,5 | 0,7 | 1,1 | 0,9 | 0,5 | 1,0 | 0,3 | 0,9 | 0,8 | 0,9 | 0,6 | 0,4 | 0,3 | 1,0 |
| Nos2 | 1,0 | 1,0 | 1,2 | 0,4 | 1,0 | 1,0 | 0,8 | 0,5 | 1,0 | 1,0 | 1,0 | 1,0 | 1,4 | 0,8 | 0,6 | 0,6 | 1,0 | 1,0 | 34,0 | 8,2 | 2,3 | 1,3 |
| Scd3 | 1,3 | 1,0 | 1,0 | 2,5 | 0,9 | 0,4 | 2,4 | 0,8 | 1,0 | 1,0 | 0,7 | 0,8 | 2,6 | 0,8 | 1,3 | 1,0 | 0,9 | 0,8 | 1,0 | 1,0 | 1,0 | 1,0 |
| Nr1d1 | 0,9 | 0,9 | 0,7 | 1,5 | 0,9 | 1,0 | 0,6 | 1,0 | 0,6 | 1,0 | 0,8 | 1,7 | 1,5 | 2,6 | 0,7 | 1,0 | 0,6 | 0,9 | 0,6 | 1,2 | 0,6 | 1,3 |
| Nr4a1 | 1,9 | 1,6 | 0,8 | 0,8 | 1,0 | 0,8 | 1,7 | 1,5 | 1,7 | 1,1 | 1,1 | 1,4 | 1,2 | 0,8 | 1,1 | 0,8 | 1,1 | 0,8 | 1,0 | 0,7 | 0,9 | 0,6 |
| Nrap | 1,0 | 1,0 | 0,7 | 1,0 | 0,6 | 0,5 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Nrep | 0,8 | 1,0 | 1,0 | 1,4 | 0,9 | 1,0 | 0,6 | 0,4 | 0,2 | 0,2 | 0,7 | 0,5 | 1,1 | 1,1 | 0,4 | 0,5 | 0,7 | 1,0 | 0,6 | 0,6 | 0,8 | 0,5 |
| Nrip1 | 1,7 | 1,1 | 0,9 | 1,3 | 1,5 | 0,7 | 0,8 | 0,8 | 2,9 | 0,7 | 1,0 | 2,2 | 0,5 | 1,0 | 0,4 | 0,3 | 0,8 | 1,0 | 0,7 | 0,5 | 1,0 | 2,1 |
| Ntsr2 | 1,0 | 1,0 | 1,0 | 1,0 | 0,7 | 1,0 | 1,0 | 1,4 | 1,0 | 1,0 | 1,0 | 1,0 | 1,0 | 1,0 | 6,9 | 1,3 | 1,0 | 1,0 | 1,0 | 1,0 | 1,0 | 1,0 |

[Fig. 23-25]

| | | | | | | | | | | | | | | | | | | | | | | |
|---|---|---|---|---|---|---|---|---|---|---|---|---|---|---|---|---|---|---|---|---|---|---|
| Nudt7 | 1,1 | 1,2 | 1,0 | 1,3 | 1,0 | 1,0 | 1,0 | 1,1 | 0,2 | 0,2 | 1,1 | 0,9 | 1,0 | 1,5 | 0,8 | 1,6 | 1,1 | 0,8 | 1,0 | 1,2 | 1,0 | 0,9 |
| Nuggc | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,1 | 1,0 |
| Nupr1l | 2,4 | 0,9 | 0,7 | 1,0 | 0,6 | 0,5 | 1,2 | 0,6 | 1,0 | 1,0 | 1,0 | 0,9 | 2,7 | 1,9 | 5,9 | 2,7 | 1,1 | 0,4 | 1,0 | 1,0 | 1,0 | 1,0 |
| Nutf2-ps1 | 1,0 | 0,7 | 0,9 | 1,2 | 0,9 | 1,0 | 3,4 | 0,5 | 1,5 | 0,8 | 0,9 | 0,9 | 0,7 | 0,7 | 0,8 | 1,5 | 1,0 | 1,3 | 1,1 | 1,1 | 1,3 | 0,7 |
| Nxf3 | 1,0 | 1,0 | 1,0 | 1,0 | 1,0 | 1,0 | 1,0 | 1,0 | 1,0 | 1,0 | 1,0 | 1,0 | 1,0 | 1,0 | 10,3 | 1,0 | 1,0 | 1,0 | 1,0 | 1,0 | 1,0 | 1,0 |
| Nxpe2 | 1,0 | 1,0 | 1,5 | 1,2 | 1,0 | 1,0 | 1,0 | 1,0 | 0,7 | 0,7 | 1,0 | 1,0 | 1,5 | 1,0 | 6,6 | 1,0 | 1,0 | 1,0 | 1,0 | 1,0 | 1,0 | 1,0 |
| Nxph4 | 0,6 | 0,5 | 1,0 | 1,0 | 0,9 | 0,9 | 1,0 | 1,0 | 1,0 | 1,0 | 1,0 | 1,0 | 1,0 | 1,0 | 9,0 | 1,0 | 1,0 | 1,0 | 1,0 | 1,0 | 1,0 | 1,0 |
| Oaz1-ps | 0,2 | 1,0 | 0,2 | 0,3 | 17,6 | 0,0 | 1,0 | 0,7 | 0,4 | 0,0 | 0,2 | 0,7 | 3,3 | 1,3 | 10,9 | 2,7 | 1,0 | 0,8 | 2,3 | 1,6 | 13,9 | 3,0 |
| Oaz3 | 1,0 | 1,0 | 1,0 | 0,9 | 0,8 | 1,1 | 1,0 | 1,0 | 1,0 | 1,0 | 1,0 | 1,0 | 0,5 | 0,9 | 11,5 | 2,9 | 1,0 | 1,0 | 1,0 | 1,0 | 1,0 | 1,0 |
| Obscn | 1,0 | 1,0 | 1,0 | 1,0 | 0,7 | 0,5 | 1,1 | 1,1 | 1,0 | 1,0 | 1,0 | 1,0 | 1,0 | 1,0 | 1,0 | 1,0 | 1,0 | 1,0 | 1,0 | 1,0 | 1,0 | 1,0 |
| Ociad2 | 0,7 | 0,9 | 1,2 | 1,2 | 1,0 | 1,3 | 1,2 | 0,7 | 0,6 | 0,6 | 1,4 | 0,7 | 1,1 | 1,5 | 7,6 | 1,8 | 1,3 | 1,1 | 1,2 | 1,3 | 1,1 | 1,0 |
| Odf1 | 1,0 | 1,0 | 1,0 | 1,0 | 1,0 | 1,0 | 1,0 | 1,0 | 1,0 | 1,0 | 1,0 | 1,0 | 1,0 | 1,0 | 10,9 | 3,4 | 1,0 | 1,0 | 1,0 | 1,0 | 1,0 | 1,0 |
| Odf3l2 | 1,0 | 1,0 | 1,0 | 1,0 | 1,1 | 1,2 | 1,0 | 1,0 | 1,0 | 1,0 | 1,3 | 0,8 | 1,0 | 1,0 | 1,0 | 1,0 | 1,0 | 1,0 | 1,0 | 1,0 | 1,0 | 1,0 |
| Olfr165 | 1,0 | 1,0 | 1,3 | 1,3 | 1,0 | 1,0 | 1,0 | 1,0 | 1,0 | 1,0 | 1,0 | 1,0 | 1,0 | 1,0 | 1,0 | 1,0 | 1,0 | 1,0 | 0,2 | 1,1 | 0,5 | 0,6 |
| Omp | 1,0 | 1,0 | 1,3 | 0,8 | 1,0 | 1,0 | 1,0 | 1,0 | 1,0 | 1,0 | 1,0 | 1,0 | 1,4 | 0,8 | 6,8 | 1,0 | 1,1 | 0,6 | 1,0 | 1,0 | 1,0 | 1,0 |
| Onecut1 | 1,0 | 1,0 | 1,0 | 1,0 | 1,0 | 1,0 | 1,0 | 1,0 | 0,5 | 0,2 | 1,0 | 1,0 | 1,0 | 1,0 | 1,0 | 1,0 | 1,0 | 1,0 | 1,0 | 1,0 | 1,0 | 1,0 |
| Oscar | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Ostn | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Ovch2 | 1,0 | 1,0 | 1,0 | -1,0 | 1,0 | 1,0 | 1,0 | 1,0 | 1,0 | 1,0 | 1,0 | 1,0 | 1,0 | 1,0 | 46,8 | 1,0 | 1,0 | 1,0 | 1,0 | 1,0 | 1,0 | 1,0 |
| Oxtr | 1,0 | 1,0 | 1,0 | 1,0 | 0,5 | 0,8 | 1,0 | 1,0 | 1,0 | 1,0 | 1,0 | 1,0 | 1,0 | 1,0 | 0,7 | 0,2 | 1,0 | 1,0 | 1,0 | 1,0 | 1,0 | 1,0 |
| P2rx2 | 1,0 | 1,0 | 1,1 | 1,3 | 0,7 | 0,8 | 1,0 | 1,0 | 1,0 | 1,0 | 1,0 | 1,0 | 1,0 | 1,0 | 6,9 | 1,0 | 1,0 | 1,0 | 1,0 | 1,0 | 1,0 | 0,9 |
| Padi2 | 1,0 | 1,1 | 1,2 | 1,4 | 0,9 | 0,8 | 1,0 | 1,0 | 1,0 | 1,0 | 1,3 | 1,5 | 1,0 | 1,1 | 5,2 | 1,0 | 1,5 | 1,1 | 0,9 | 1,0 | 0,8 | 0,8 |
| Paqr9 | 0,7 | 0,6 | 1,0 | 1,0 | 1,2 | 1,1 | 1,0 | 1,0 | 0,5 | 0,3 | 1,0 | 1,0 | 1,6 | 1,2 | 0,5 | 0,4 | 1,0 | 1,0 | 0,2 | 0,2 | 0,9 | 1,0 |
| Pate2 | 1,0 | 1,0 | 1,0 | 1,0 | 1,0 | 1,0 | 1,0 | 1,0 | 1,0 | 1,0 | 1,0 | 1,0 | 1,0 | 1,0 | 5,2 | 1,0 | 1,0 | 1,0 | 1,0 | 1,0 | 1,0 | 1,0 |
| Pax2 | 1,1 | 1,0 | 1,0 | 1,0 | 1,0 | 1,0 | 1,0 | 1,0 | 1,0 | 1,0 | 1,0 | 1,0 | 1,0 | 1,0 | 8,0 | 1,0 | 1,0 | 1,0 | 1,0 | 1,0 | 1,0 | 1,0 |
| Pax5 | 1,0 | 1,0 | 0,9 | 0,6 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,7 |
| Pax8 | 0,9 | 1,0 | 0,9 | 1,3 | 1,0 | 1,0 | 1,0 | 1,0 | 1,0 | 1,0 | 1,0 | 1,0 | 1,0 | 1,0 | 6,6 | 3,9 | 1,0 | 1,0 | 1,0 | 0,9 | 1,0 | 1,4 |
| Pck1 | 2,5 | 3,6 | 1,5 | 1,3 | 0,2 | 0,4 | 3,4 | 1,0 | 0,8 | 1,0 | 0,9 | 2,3 | 1,4 | 6,1 | 0,4 | 1,0 | 1,1 | 0,4 | 0,7 | 1,7 | 1,7 | 6,6 |
| Pcp4 | 0,9 | 1,1 | 1,1 | 1,2 | 1,0 | 1,3 | 1,0 | 1,1 | 1,0 | 1,0 | 0,9 | 0,6 | 1,1 | 1,1 | 1,0 | 27,8 | 1,0 | 0,8 | 1,1 | 0,9 | 0,9 | 0,9 |
| Pcp4l1 | 0,9 | 0,9 | 1,0 | 1,0 | 1,0 | 1,0 | 1,2 | 1,5 | 3,1 | 5,4 | 1,3 | 1,0 | 1,2 | 1,6 | 0,8 | 1,7 | 1,0 | 1,0 | 1,9 | 0,9 | 0,8 | 0,8 |
| Pcsk9 | 5,9 | 4,7 | 0,7 | 0,6 | 0,9 | 1,0 | 1,0 | 1,0 | 2,7 | 1,3 | 1,0 | 1,0 | 1,0 | 1,0 | 1,0 | 1,0 | 1,0 | 1,0 | 2,1 | 1,5 | 1,7 | 0,9 |
| Pcyt1b | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Pde6a | 0,3 | 0,2 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Pdgfrl | 1,0 | 1,0 | 1,0 | 1,0 | 1,0 | 0,9 | 0,8 | 0,6 | 1,0 | 1,0 | 1,0 | 1,0 | 1,1 | 1,0 | 1,4 | 1,2 | 0,7 | 0,8 | 1,0 | 1,0 | 1,0 | 1,0 |
| Pdk4 | 1,5 | 1,7 | 4,3 | 5,9 | 0,7 | 0,9 | 0,9 | 1,7 | 2,8 | 2,0 | 1,2 | 1,2 | 1,1 | 4,7 | 0,8 | 1,2 | 0,8 | 0,9 | 0,6 | 0,7 | 1,4 | 1,8 |
| Pdlim3 | 1,6 | 1,2 | 1,1 | 1,0 | 0,9 | 0,8 | 1,1 | 1,2 | 1,0 | 1,0 | 1,1 | 0,7 | 2,3 | 1,7 | 0,9 | 1,9 | 1,1 | 0,9 | 1,3 | 1,1 | 0,9 | 0,9 |
| | Kidney | | Colon | | Eyeball | | Heart | | Liver | | SG | | Thymus | | Adipose | | Stomach | | Jejunum | | Ileum | |
| | E | M | E | M | E | M | E | M | E | M | E | M | E | M | E | M | E | M | E | M | E | M |
| Pdzk1 | 0,7 | 0,7 | 0,8 | 1,3 | 1,2 | 1,1 | 1,2 | 1,0 | 1,0 | 1,0 | 0,7 | 0,9 | 0,8 | 0,6 | 7,2 | 2,0 | 1,1 | 0,8 | 1,1 | 1,5 | 1,1 | 1,1 |
| Pdzk1ip1 | 0,9 | 0,8 | 1,2 | 1,1 | 0,8 | 1,1 | 1,0 | 1,0 | 1,3 | 0,7 | 1,2 | 0,8 | 2,3 | 0,6 | 2,5 | 7,4 | 1,0 | 0,9 | 1,5 | 0,7 | 1,1 | 0,5 |
| Pgam2 | 1,0 | 1,0 | 1,4 | 1,1 | 0,6 | 0,5 | 1,0 | 1,0 | 1,0 | 1,0 | 1,6 | 1,2 | 0,9 | 1,0 | 2,0 | 1,6 | 1,2 | 0,9 | 0,8 | 0,7 | 1,4 | 1,4 |
| Pgc | 1,0 | 1,0 | 1,0 | -1,0 | 1,0 | 0,8 | 1,0 | 1,1 | 1,0 | 1,0 | 1,0 | 1,1 | 1,0 | 8,7 | 1,0 | 7,4 | 1,0 | 1,0 | 0,7 | 1,0 | 1,1 | 1,0 |
| Pgk2 | 1,0 | 1,0 | 1,0 | 1,0 | 1,0 | 1,0 | 1,0 | 1,0 | 1,0 | 1,0 | 0,5 | 0,7 | 1,0 | 1,0 | 6,1 | 1,7 | 1,0 | 1,0 | 1,0 | 1,0 | 1,0 | 1,0 |
| Pglyrp1 | 1,0 | 1,0 | 1,1 | 0,9 | 1,2 | 1,1 | 1,3 | 1,7 | 1,0 | 1,0 | 0,9 | 1,0 | 1,7 | 1,3 | 0,6 | 0,7 | 1,4 | 1,1 | 1,7 | 1,5 | 1,2 | 1,0 |
| Phkg1 | 1,0 | 1,0 | 1,0 | 1,0 | 0,5 | 0,5 | 0,7 | 0,8 | 1,0 | 1,0 | 1,0 | 0,8 | 1,0 | 1,0 | 0,9 | 1,7 | 1,0 | 1,0 | 1,0 | 1,0 | 1,0 | 1,0 |
| Phlda1 | 1,2 | 0,8 | 1,0 | 0,8 | 0,9 | 0,8 | 0,9 | 1,0 | 0,2 | 0,2 | 0,7 | 0,9 | 0,6 | 0,6 | 1,3 | 0,7 | 1,2 | 0,9 | 1,3 | 1,3 | 1,6 | 1,3 |
| Pi15 | 1,0 | 1,0 | 1,0 | 1,0 | 1,0 | 1,0 | 0,8 | 0,7 | 1,0 | 1,0 | 1,0 | 1,0 | 1,0 | 1,2 | 0,5 | 0,5 | 0,9 | 1,7 | 1,0 | 1,0 | 1,0 | 1,0 |
| Pigr | 1,4 | 1,4 | 1,1 | 0,9 | 1,0 | 1,0 | 1,0 | 1,0 | 0,8 | 1,2 | 0,7 | 1,3 | 2,5 | 1,2 | 1,0 | 1,0 | 3,0 | 1,4 | 1,2 | 1,3 | 1,1 | 1,0 |
| Pip5k1b | 1,4 | 1,4 | 1,0 | 1,0 | 1,1 | 1,1 | 1,1 | 1,1 | 1,0 | 1,0 | 0,9 | 0,9 | 0,9 | 0,9 | 7,2 | 1,0 | 0,9 | 0,8 | 1,1 | 1,1 | 1,1 | 1,1 |
| Pkia | 0,8 | 1,0 | 0,9 | 1,0 | 1,0 | 0,9 | 1,0 | 0,9 | 1,0 | 1,0 | 0,9 | 1,0 | 1,2 | 1,8 | 1,0 | 0,6 | 1,1 | 0,9 | 1,0 | 0,8 | 0,8 | 0,9 |
| Pla2g1b | 1,0 | 1,0 | 2,2 | 15,8 | 1,0 | 0,9 | 1,0 | 0,8 | 1,0 | 1,0 | 1,0 | 0,7 | 1,0 | 4,6 | 1,0 | 3,5 | 0,9 | 0,8 | 0,3 | 1,0 | 1,6 | 1,0 |
| Pla2g4c | 1,0 | 1,0 | 0,1 | 0,1 | 1,0 | 1,0 | 1,0 | 1,0 | 1,0 | 1,0 | 1,0 | 1,0 | 0,6 | 0,7 | 1,0 | 1,0 | 1,5 | 1,0 | 1,0 | 1,0 | 0,2 | 0,3 |
| Pla2g5 | 0,8 | 1,2 | 1,9 | 1,2 | 1,0 | 1,1 | 0,7 | 0,8 | 1,0 | 1,0 | 1,0 | 1,0 | 1,0 | 1,0 | 7,3 | 1,4 | 1,6 | 0,9 | 3,4 | 2,6 | 1,7 | 1,4 |
| Plac8 | 1,6 | 1,4 | 1,3 | 1,0 | 1,0 | 1,1 | 1,3 | 1,0 | 0,6 | 1,5 | 0,7 | 0,8 | 1,3 | 1,0 | 9,6 | 3,0 | 1,0 | 0,8 | 1,1 | 1,4 | 1,2 | 0,9 |
| Plac9a | 1,4 | 0,5 | 1,1 | 0,1 | 2,7 | 0,4 | 1,0 | 0,2 | 0,4 | 0,3 | 1,0 | 0,4 | 3,7 | 0,7 | 0,4 | 1,3 | 0,8 | 0,7 | 0,9 | 3,5 | 1,0 | 0,1 |
| Plek2 | 1,3 | 0,7 | 1,1 | 1,1 | 1,1 | 1,7 | 1,0 | 1,0 | 1,0 | 1,0 | 1,9 | 1,2 | 1,7 | 1,0 | 5,1 | 1,0 | 1,0 | 1,0 | 0,8 | 1,2 | 1,7 | 1,4 |
| Plet1 | 1,2 | 1,1 | 1,7 | 1,0 | 1,1 | 1,2 | 1,0 | 1,0 | 0,7 | 1,2 | 1,0 | 0,8 | 2,1 | 1,2 | 1,0 | 7,9 | 0,9 | 0,9 | 3,4 | 0,9 | 8,5 | 0,9 |
| Plin1 | 1,0 | 1,0 | 0,6 | 1,0 | 0,3 | 0,3 | 3,1 | 0,9 | 1,0 | 1,0 | 1,0 | 3,2 | 1,8 | 3,5 | 0,8 | 0,9 | 0,5 | 1,0 | 1,0 | 0,9 | 0,5 | 2,0 |
| Plin4 | 3,0 | 1,4 | 0,7 | 1,2 | 0,9 | 0,7 | 1,2 | 1,3 | 5,6 | 4,1 | 1,0 | 1,7 | 1,7 | 3,4 | 0,5 | 0,5 | 0,8 | 0,9 | 0,7 | 0,6 | 0,6 | 1,7 |
| Pnlip | 1,0 | 0,8 | 1,4 | 64,9 | 1,0 | 1,0 | 1,0 | 1,0 | 0,8 | 0,7 | 0,7 | 1,0 | 1,0 | 0,4 | 1,0 | 1,0 | 159,2 | 0,0 | 0,0 | 0,8 | 1,0 | 1,0 |
| Pnliprp1 | 1,0 | 1,0 | 1,0 | 15,5 | 1,0 | 1,0 | 1,0 | 1,0 | 1,0 | 1,0 | 1,0 | 1,0 | 1,0 | 1,0 | 1,0 | 1,0 | 2,5 | 0,2 | 0,1 | 1,0 | 0,9 | 1,0 |
| Pnmt | 1,0 | 1,0 | 1,0 | 1,0 | 1,0 | 1,0 | 1,1 | 1,6 | 1,0 | 1,0 | 1,0 | 1,0 | 1,0 | 1,0 | 2,1 | 1,0 | 1,0 | 1,0 | 1,0 | 1,0 | 1,0 | 1,0 |
| Pou2af1 | 1,0 | 1,0 | 0,7 | 0,3 | 1,0 | 1,0 | 1,0 | 1,0 | 1,0 | 1,0 | 0,7 | 2,3 | 1,2 | 1,3 | 0,7 | 0,9 | 1,0 | 1,1 | 1,1 | 1,2 | 0,8 | 1,3 |
| Pou3f3 | 1,2 | 0,9 | 1,0 | 1,4 | 1,0 | 0,9 | 1,0 | 1,0 | 1,0 | 1,0 | 1,0 | 1,0 | 1,0 | 1,0 | 7,5 | 3,7 | 1,0 | 1,0 | 1,0 | 1,0 | 1,0 | 1,0 |
| Pou3f3os | 0,7 | 1,0 | 1,0 | -1,0 | 1,4 | 2,1 | 1,0 | 1,0 | 1,0 | 1,0 | 1,0 | 1,0 | 1,0 | 1,0 | 7,4 | 2,3 | 1,0 | 1,0 | 1,0 | 1,0 | 1,0 | 1,0 |
| Ppapdc3 | 1,1 | 1,0 | 0,8 | 1,0 | 0,8 | 0,7 | 1,0 | 0,8 | 1,0 | 1,0 | 1,2 | 1,0 | 1,0 | 1,0 | 0,9 | 0,8 | 1,3 | 1,1 | 1,0 | 1,0 | 1,0 | 1,0 |
| Ppl | 1,4 | 1,0 | 0,9 | 1,1 | 1,1 | 1,1 | 1,2 | 1,2 | 5,5 | 4,7 | 1,3 | 0,9 | 1,8 | 1,9 | 1,8 | 0,8 | 1,0 | 1,0 | 1,5 | 1,5 | 0,8 | 1,3 |
| Ppp1r14c | 1,0 | 1,0 | 1,0 | 0,7 | 1,1 | 0,9 | 1,0 | 0,9 | 1,0 | 1,0 | 1,0 | 1,0 | 1,0 | 1,0 | 1,0 | 1,0 | 1,0 | 1,1 | 0,9 | 1,0 | 1,0 | 0,7 |
| Ppp1r27 | 1,0 | 1,0 | 1,0 | 1,0 | 1,0 | 1,0 | 1,0 | 1,0 | 1,0 | 1,0 | 1,0 | 1,0 | 1,2 | 3,4 | 1,0 | 1,0 | 1,0 | 1,0 | 1,0 | 1,0 | 1,0 | 1,0 |
| Ppp1r3a | 1,0 | 1,0 | 1,0 | 1,0 | 0,8 | 0,7 | 1,0 | 1,2 | 1,0 | 1,0 | 1,0 | 1,0 | 1,0 | 1,0 | 1,0 | 1,0 | 1,3 | 1,0 | 1,0 | 1,0 | 1,0 | 1,0 |
| Prb1 | 1,0 | 1,0 | 1,0 | 1,0 | 1,4 | 0,8 | 1,0 | 1,0 | 1,0 | 1,0 | 225,2 | 1782 | 1,0 | 1,0 | 1,0 | 1,0 | 1,0 | 1,0 | 1,0 | 1,0 | 1,0 | 1,0 |
| Prh1 | 1,0 | 1,0 | 1,0 | 1,0 | 1,0 | 1,0 | 1,0 | 1,5 | 1,0 | 1,0 | 517,3 | 7058 | 1,0 | 1,0 | 1,0 | 1,0 | 1,0 | 1,2 | 1,0 | 1,0 | 1,0 | 1,0 |
| Prkcq | 0,9 | 1,1 | 0,7 | 0,6 | 0,9 | 1,0 | 0,9 | 0,9 | 1,0 | 1,0 | 1,0 | 1,0 | 0,9 | 1,0 | 1,0 | 1,0 | 1,0 | 1,0 | 1,0 | 1,0 | 0,7 | 0,8 |
| Prlr | 7,2 | 21,8 | 0,8 | 1,4 | 1,0 | 1,0 | 0,6 | 0,8 | 2,0 | 3,0 | 1,2 | 1,6 | 1,0 | 1,0 | 1,4 | 1,7 | 1,0 | 0,8 | 0,8 | 0,9 | 1,1 | 1,5 |
| Prm1 | 1,0 | 1,0 | 1,4 | 0,9 | 1,0 | 1,0 | 1,0 | 1,0 | 1,0 | 1,0 | 1,0 | 1,0 | 1,2 | 0,6 | 8,6 | 9,3 | 1,0 | 1,0 | 1,0 | 1,9 | 1,0 | 1,6 |
| Prm2 | 1,0 | 1,0 | 1,0 | 0,8 | 1,2 | 1,0 | 1,2 | 1,0 | 1,0 | 1,0 | 1,0 | 1,0 | 1,0 | 1,0 | 16,5 | 12,7 | 0,8 | 1,0 | 1,0 | 1,7 | 1,0 | 1,2 |
| Prm3 | 1,0 | 1,0 | 1,0 | 1,0 | 1,0 | 1,0 | 1,0 | 1,0 | 1,0 | 1,0 | 1,0 | 1,0 | 1,0 | 1,0 | 5,9 | 1,7 | 1,0 | 1,0 | 1,0 | 1,0 | 1,0 | 1,0 |
| Prnd | 1,0 | 1,0 | 1,0 | 1,0 | 1,0 | 1,0 | 0,5 | 0,3 | 1,0 | 1,0 | 1,0 | 1,0 | 0,8 | 1,0 | 0,3 | 0,3 | 2,0 | 2,1 | 1,0 | 1,0 | 1,0 | 1,0 |
| Nt5c1a | 1,2 | 1,0 | 1,0 | 1,0 | 1,0 | 0,8 | 1,0 | 0,7 | 0,9 | 1,0 | 1,2 | 1,0 | 1,0 | 7,8 | 1,0 | 15,1 | 1,0 | 1,2 | 1,0 | 1,0 | 1,0 | 1,0 |
| Prom2 | 1,0 | 0,9 | 1,0 | 1,0 | 1,0 | 1,1 | 1,0 | 1,0 | 1,0 | 1,0 | 0,9 | 0,7 | 2,1 | 1,6 | 7,5 | 1,0 | 1,0 | 1,0 | 1,0 | 1,0 | 1,0 | 1,0 |
| Prp2 | 1,0 | 1,0 | 1,0 | 1,0 | 1,0 | 1,0 | 1,0 | 1,0 | 1,0 | 1,0 | 133,2 | 2495 | 1,0 | 1,0 | 1,0 | 1,0 | 1,0 | 1,0 | 1,0 | 1,0 | 1,0 | 1,0 |
| Prph | 1,0 | 1,0 | 0,8 | 0,9 | 1,2 | 1,4 | 1,0 | 1,0 | 1,0 | 1,0 | 1,0 | 1,0 | 1,2 | 0,9 | 1,0 | 1,0 | 0,6 | 0,6 | 0,9 | 0,9 | 0,9 | 1,1 |
| Prpmp5 | 1,0 | 1,0 | 1,0 | 1,0 | 2,5 | 1,0 | 1,0 | 1,0 | 1,0 | 1,0 | 21,6 | 198,1 | 1,0 | 1,0 | 1,0 | 1,0 | 1,0 | 1,0 | 1,0 | 1,0 | 1,0 | 1,0 |
| Prr32 | 1,0 | 1,0 | 1,0 | 1,0 | 1,0 | 1,0 | 1,0 | 1,0 | 1,0 | 1,0 | 1,0 | 1,0 | 1,1 | 1,5 | 0,2 | 0,3 | 1,0 | 1,0 | 1,0 | 1,0 | 1,0 | 1,0 |

[Fig. 23-26]

295/310

| | | | | | | | | | | | | | | | | | | | | | | |
|---|---|---|---|---|---|---|---|---|---|---|---|---|---|---|---|---|---|---|---|---|---|---|
| Prr33 | 1,0 | 1,0 | 1,1 | 1,0 | 0,7 | 0,6 | 1,2 | 1,1 | 1,0 | 1,0 | 1,0 | 1,1 | 0,9 | 0,8 | 1,2 | 0,6 | 0,9 | 1,0 | 0,7 | 0,7 | 0,9 | 1,3 |
| Prss1 | 1,0 | 1,0 | 15,9 | 1,0 | 1,0 | 1,0 | 1,0 | 1,0 | 1,0 | 1,0 | 1,0 | 1,0 | 1,0 | 1,0 | 1,0 | 1,0 | 3,7 | 0,2 | 1,0 | 1,0 | 1,0 | 1,0 |
| Prss2 | 1,2 | 1,0 | 1,9 | 251,3 | 1,0 | 1,0 | 1,0 | 1,0 | 0,6 | 0,7 | 0,7 | 1,0 | 1,7 | 0,2 | 1,0 | 1,0 | 302,9 | 0,1 | 0,0 | 0,9 | 0,9 | 1,0 |
| Prss3 | 1,0 | 1,0 | 7,6 | 9,6 | 1,0 | 1,0 | 1,0 | 1,0 | 1,0 | 1,0 | 1,0 | 1,0 | 1,0 | 1,0 | 1,0 | 1,0 | 47,2 | 0,0 | 0,2 | 1,0 | 1,0 | 1,0 |
| Prss8 | 0,7 | 0,8 | 1,0 | 1,2 | 0,9 | 1,3 | 1,0 | 1,0 | 2,0 | 1,3 | 1,0 | 0,9 | 2,0 | 1,6 | 5,8 | 2,7 | 1,1 | 1,0 | 1,4 | 1,5 | 1,2 | 1,1 |
| Prtn3 | 1,0 | 1,0 | 1,0 | 1,0 | 1,0 | 1,0 | 1,0 | 1,0 | 0,9 | 1,0 | 1,0 | 1,0 | 1,0 | 1,0 | 0,2 | 0,8 | 1,0 | 1,0 | 1,0 | 1,0 | 1,0 | 1,0 |
| Ptch1 | 1,1 | 0,9 | 0,8 | 1,2 | 1,1 | 0,7 | 1,0 | 0,9 | 0,7 | 0,5 | 1,0 | 2,3 | 0,7 | 0,7 | 0,9 | 0,4 | 0,7 | 0,9 | 0,9 | 0,6 | 1,1 | 2,0 |
| Ptch2 | 1,0 | 1,0 | 1,0 | 1,0 | 1,0 | 1,0 | 1,0 | 1,0 | 1,0 | 1,0 | 1,0 | 1,0 | 1,0 | 1,0 | 0,8 | 0,4 | 1,0 | 1,0 | 1,0 | 1,0 | 1,0 | 1,0 |
| Ptgds | 0,5 | 0,8 | 1,0 | 1,0 | 1,1 | 1,0 | 2,1 | 1,7 | 1,0 | 3,8 | 0,7 | 0,3 | 1,0 | 1,0 | 5,5 | 2,9 | 1,0 | 1,0 | 1,0 | 1,0 | 1,0 | 1,0 |
| Ptger3 | 1,4 | 1,3 | 1,1 | 1,0 | 0,6 | 0,6 | 1,0 | 1,0 | 1,0 | 1,0 | 1,0 | 1,0 | 2,0 | 1,8 | 1,1 | 0,8 | 0,9 | 0,7 | 0,9 | 1,0 | 0,7 | 1,8 |
| Ptgs2 | 1,0 | 1,0 | 0,9 | 0,7 | 1,0 | 1,0 | 1,3 | 1,0 | 1,0 | 1,0 | 1,0 | 1,0 | 1,1 | 0,7 | 1,0 | 12,0 | 1,1 | 1,1 | 0,9 | 1,0 | 1,0 | 0,9 |
| *Pth* | *1,5* | *1,0* | *1,0* | *1,0* | *1,0* | *0,7* | *1,0* | *2,2* | *1,0* | *1,0* | *1,0* | *0,7* | *5,2* | *4,9* | *1,0* | *1,6* | *1,0* | *1,4* | *1,0* | *1,0* | *1,0* | *1,0* |
| Ptpn5 | 1,0 | 1,0 | 1,0 | 1,0 | 0,7 | 1,0 | 1,0 | 1,0 | 1,0 | 1,0 | 1,0 | 1,0 | 1,0 | 1,0 | 19,9 | 1,0 | 1,0 | 1,0 | 1,0 | 1,0 | 1,0 | 1,0 |
| Ptprcap | 0,8 | 0,6 | 0,6 | 0,3 | 0,8 | 0,8 | 1,0 | 1,0 | 0,4 | 0,9 | 0,6 | 1,6 | 1,1 | 0,9 | 1,1 | 1,2 | 1,2 | 0,4 | 1,1 | 1,2 | 0,8 | 0,6 |
| Pvalb | 0,8 | 1,1 | 0,7 | 1,0 | 0,6 | 0,6 | 1,2 | 1,0 | 1,0 | 1,0 | 3,0 | 1,9 | 1,2 | 0,8 | 1,4 | 0,6 | 1,0 | 0,8 | 1,0 | 1,0 | 1,0 | 1,0 |
| Pvrl4 | 1,7 | 1,7 | 0,9 | 1,3 | 0,9 | 1,0 | 1,0 | 1,0 | 1,0 | 1,0 | 1,1 | 1,0 | 2,2 | 1,3 | 9,4 | 1,0 | 0,9 | 1,0 | 1,0 | 1,0 | 1,0 | 1,0 |
| Pygm | 1,0 | 0,8 | 1,2 | 0,9 | 0,8 | 0,6 | 1,0 | 1,1 | 1,0 | 1,0 | 1,0 | 1,7 | 0,8 | 1,2 | 0,9 | 0,7 | 0,8 | 1,0 | 1,0 | 0,8 | 0,9 | 0,8 |
| Pzp | 0,2 | 0,1 | 1,0 | 1,0 | 1,0 | 1,0 | 1,0 | 1,0 | 1,0 | 0,8 | 1,0 | 1,0 | 1,0 | 1,0 | 1,0 | 1,0 | 2,5 | 0,5 | 1,0 | 1,0 | 1,0 | 1,0 |
| *Rab11fip1* | *1,1* | *1,1* | *1,0* | *1,3* | *1,1* | *1,0* | *1,0* | *1,0* | *1,8* | *1,2* | *1,2* | *1,4* | *1,1* | *0,9* | *5,2* | *1,0* | *0,8* | *0,9* | *1,0* | *0,9* | *1,0* | *1,4* |
| Rab11fip4 | 1,4 | 0,7 | 1,1 | 1,3 | 1,2 | 0,7 | 1,0 | 1,0 | 1,2 | 1,0 | 0,9 | 1,6 | 0,8 | 1,0 | 10,0 | 1,0 | 0,8 | 1,0 | 1,1 | 0,8 | 1,3 | 1,2 |
| Rab25 | 1,0 | 1,2 | 1,0 | 1,0 | 1,1 | 1,2 | 1,0 | 1,0 | 1,0 | 1,0 | 1,0 | 0,9 | 2,1 | 0,8 | 7,7 | 1,9 | 1,0 | 1,0 | 1,2 | 1,1 | 1,2 | 1,0 |
| Rab26 | 1,0 | 1,0 | 1,0 | 1,0 | 1,0 | 0,9 | 1,0 | 1,0 | 1,0 | 1,0 | 1,2 | 1,1 | 1,0 | 1,0 | 14,8 | 1,0 | 1,0 | 1,0 | 1,0 | 1,0 | 1,0 | 1,0 |
| Rab30 | 1,6 | 1,8 | 0,5 | 0,8 | 0,8 | 1,0 | 0,9 | 0,9 | 0,6 | 1,4 | 1,0 | 1,0 | 1,0 | 1,4 | 1,4 | 0,6 | 0,9 | 1,0 | 0,6 | 1,4 | 6,0 | 1,0 |
| Rad51b | 1,0 | 1,0 | 1,1 | 1,0 | 1,0 | 1,0 | 1,0 | 1,0 | 4,2 | 9,5 | 1,0 | 1,0 | 0,8 | 0,9 | 0,8 | 1,8 | 1,0 | 1,0 | 1,1 | 1,0 | 1,1 | 1,1 |
| Rag1 | 1,0 | 1,0 | 1,0 | 1,0 | 1,0 | 1,0 | 1,0 | 1,0 | 1,0 | 1,0 | 1,0 | 1,0 | 0,5 | 0,7 | 1,0 | 1,0 | 1,0 | 1,0 | 1,0 | 1,0 | 1,0 | 1,0 |
| Ramp1 | 1,0 | 1,0 | 1,0 | 0,9 | 0,8 | 0,6 | 1,2 | 1,1 | 2,1 | 1,9 | 0,9 | 0,9 | 0,8 | 0,8 | 1,9 | 1,2 | 1,0 | 1,1 | 0,7 | 1,3 | 1,0 | 0,8 |
| Ramp3 | 0,7 | 0,7 | 0,9 | 1,1 | 0,9 | 1,0 | 1,2 | 0,8 | 1,0 | 1,0 | 0,8 | 1,2 | 1,6 | 1,5 | 6,6 | 2,8 | 1,1 | 1,1 | 1,1 | 1,4 | 1,1 | 1,2 |
| Rarres1 | 1,0 | 1,0 | 1,0 | 1,0 | 1,0 | 1,3 | 1,0 | 0,6 | 0,2 | 0,1 | 1,0 | 1,0 | 1,3 | 0,8 | 1,3 | 1,1 | 1,0 | 1,0 | 0,6 | 1,0 | 1,0 | 1,0 |
| Rasal1 | 1,0 | 1,0 | 1,0 | 1,0 | 1,0 | 1,0 | 1,0 | 1,0 | 1,0 | 1,0 | 1,1 | 1,0 | 1,3 | 1,4 | 1,0 | 1,0 | 1,0 | 1,0 | 1,0 | 1,0 | 1,0 | 1,0 |
| Rasd1 | 1,8 | 1,6 | 0,8 | 0,7 | 0,8 | 0,9 | 1,1 | 1,1 | 2,4 | 2,3 | 0,8 | 1,0 | 1,9 | 3,4 | 1,0 | 0,9 | 1,1 | 1,8 | 2,8 | 1,4 | 1,2 | 1,1 |
| Rasgrf1 | 1,0 | 1,0 | 1,0 | 1,0 | 1,1 | 1,0 | 1,0 | 1,0 | 1,0 | 1,0 | 1,0 | 1,0 | 1,0 | 1,0 | 7,2 | 1,0 | 1,0 | 1,0 | 1,0 | 1,0 | 1,0 | 1,0 |
| Rasl10a | 1,1 | 1,0 | 1,0 | 1,0 | 0,9 | 1,0 | 0,7 | 0,8 | 1,0 | 1,0 | 0,5 | 0,4 | 1,0 | 1,2 | 7,1 | 0,9 | 1,0 | 1,0 | 1,0 | 1,0 | 1,0 | 1,0 |
| Rassf6 | 1,2 | 1,5 | 1,0 | 0,9 | 1,4 | 1,2 | 1,0 | 1,0 | 1,1 | 0,9 | 1,2 | 1,2 | 1,7 | 1,7 | 2,5 | 3,6 | 1,2 | 0,9 | 0,8 | 1,1 | 1,2 | 0,9 |
| Rbfox1 | 1,0 | 0,9 | 1,0 | 1,0 | 0,9 | 1,0 | 1,0 | 1,2 | 1,0 | 1,0 | 1,0 | 1,0 | 1,8 | 1,8 | 1,0 | 1,0 | 1,0 | 1,0 | 1,0 | 1,0 | 1,0 | 1,0 |
| Rbm11 | 1,3 | 1,1 | 1,0 | 1,0 | 0,9 | 1,0 | 1,0 | 1,0 | 1,0 | 1,0 | 1,0 | 1,0 | 1,0 | 1,0 | 34,8 | 1,0 | 1,0 | 1,0 | 1,0 | 1,0 | 1,0 | 1,0 |
| Rbm24 | 1,0 | 1,0 | 1,3 | 1,1 | 0,9 | 0,8 | 1,0 | 1,1 | 1,0 | 1,0 | 1,0 | 1,2 | 1,0 | 1,0 | 2,7 | 1,0 | 1,2 | 0,7 | 0,8 | 1,0 | 0,8 | 0,8 |
| *Reg1* | *1,1* | *1,2* | *1,7* | *38,6* | *1,0* | *1,0* | *1,0* | *1,0* | *1,0* | *1,0* | *1,1* | *1,0* | *1,0* | *0,7* | *1,0* | *1,0* | *107,1* | *0,1* | *1,0* | *0,7* | *0,4* | *0,7* |
| Reg2 | 1,0 | 1,0 | 1,7 | 4,5 | 1,0 | 1,0 | 1,0 | 1,0 | 1,0 | 1,0 | 1,0 | 1,0 | 1,0 | 1,0 | 1,0 | 1,0 | 28,5 | 0,2 | 0,1 | 1,0 | 1,0 | 1,0 |
| Reg3b | 1,0 | 1,0 | 3,5 | 1,0 | 1,0 | 1,0 | 1,0 | 1,0 | 1,0 | 1,0 | 1,0 | 1,0 | 2,1 | 1,0 | 1,0 | 1,0 | 2,2 | 1,0 | 2,2 | 1,9 | 1,4 | 0,9 |
| Reg3g | 1,0 | 1,0 | 3,7 | 0,8 | 1,0 | 1,0 | 1,0 | 1,0 | 1,0 | 1,0 | 1,0 | 1,0 | 1,8 | 1,1 | 1,0 | 1,0 | 4,1 | 3,4 | 2,7 | 2,3 | 1,3 | 1,1 |
| Ren2 | 1,0 | 1,0 | 1,0 | 1,0 | 1,0 | 1,0 | 1,0 | 1,0 | 1,0 | 1,0 | 0,3 | 0,6 | 1,0 | 1,0 | 1,0 | 1,0 | 1,0 | 1,0 | 1,0 | 1,0 | 1,0 | 1,0 |
| Retnla | 1,0 | 4,5 | 0,6 | 0,7 | 0,7 | 0,4 | 1,8 | 0,8 | 1,0 | 1,0 | 0,5 | 0,4 | 1,9 | 1,3 | 0,4 | 0,6 | 0,8 | 0,8 | 2,0 | 0,6 | 1,1 | 0,6 |
| Retnlb | 1,0 | 1,0 | 0,1 | 0,1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 0,9 | 0,4 |
| *Rgs13* | *1,0* | *1,0* | *0,5* | *0,4* | *1,1* | *1,0* | *1,0* | *1,0* | *1,0* | *1,0* | *1,0* | *1,0* | *1,8* | *3,8* | *1,0* | *1,0* | *1,6* | *1,0* | *0,7* | *1,1* | *0,4* | *0,5* |
| Rgs7bp | 1,0 | 1,0 | 0,9 | 1,2 | 1,3 | 1,1 | 0,8 | 0,8 | 1,0 | 1,0 | 1,0 | 1,0 | 1,0 | 1,0 | 0,4 | 0,3 | 0,7 | 0,8 | 0,9 | 0,8 | 0,9 | 1,5 |
| Rgs9 | 1,1 | 1,1 | 1,0 | 0,9 | 1,3 | 1,2 | 1,0 | 1,0 | 1,0 | 1,0 | 1,0 | 1,0 | 1,8 | 1,9 | 5,7 | 1,0 | 0,9 | 0,9 | 0,8 | 1,0 | 1,0 | 1,1 |
| Rhag | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Rhbg | 1,1 | 0,8 | 1,2 | 1,0 | 1,1 | 1,1 | 1,0 | 1,0 | 0,2 | 0,3 | 1,0 | 1,0 | 1,0 | 1,0 | 2,1 | 1,0 | 0,8 | 1,1 | 0,9 | 1,3 | 1,2 | 1,2 |
| Rhcg | 1,1 | 0,9 | 1,0 | 1,0 | 1,0 | 1,0 | 1,0 | 1,0 | 1,0 | 1,0 | 1,0 | 1,0 | 1,0 | 1,0 | 32,2 | 1,0 | 1,0 | 1,0 | 1,0 | 1,0 | 1,0 | 1,0 |
| Rhd | 1,0 | 1,0 | 1,0 | 1,0 | 1,0 | 1,0 | 1,5 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Rhov | 0,5 | 0,3 | 1,0 | 1,2 | 1,0 | 0,9 | 1,0 | 1,0 | 1,0 | 1,0 | 1,0 | 0,9 | 2,2 | 1,5 | 1,0 | 1,0 | 1,2 | 1,3 | 2,8 | 1,3 | 1,9 | 1,6 |
| | Kidney | | Colon | | Eyeball | | Heart | | Liver | | SG | | Thymus | | Adipose | | Stomach | | Jejunum | | Ileum | |
| | E | M | E | M | E | M | E | M | E | M | E | M | E | M | E | M | E | M | E | M | E | M |
| Rhpn1 | 0,9 | 0,6 | 1,0 | 1,0 | 1,1 | 0,8 | 1,0 | 1,0 | 1,0 | 1,0 | 1,0 | 1,0 | 1,0 | 1,0 | 5,0 | 1,0 | 1,0 | 1,0 | 1,0 | 1,0 | 1,0 | 1,0 |
| Rian | 1,0 | 1,0 | 0,9 | 0,8 | 1,0 | 1,1 | 1,1 | 0,8 | 1,0 | 1,0 | 0,8 | 0,7 | 1,0 | 1,0 | 0,6 | 0,7 | 1,2 | 1,0 | 0,9 | 1,0 | 0,7 | 1,0 |
| Rmrp | 0,7 | 1,2 | 1,0 | 0,2 | 0,9 | 0,7 | 1,0 | 0,9 | 0,5 | 1,2 | 0,9 | 0,5 | 4,0 | 1,9 | 1,0 | 3,0 | 1,0 | 0,3 | 1,0 | 1,2 | 1,0 | 0,6 |
| Rn4.5s | 0,9 | 0,4 | 2,6 | 1,1 | 1,3 | 0,5 | 0,5 | 2,3 | 0,3 | 0,8 | 0,7 | 1,2 | 1,4 | 1,1 | 0,3 | 1,4 | 0,3 | 0,7 | 1,5 | 1,6 | 0,4 | 0,6 |
| Rn45s | 0,9 | 1,5 | 0,9 | 0,4 | 0,9 | 0,3 | 0,8 | 1,2 | 0,7 | 0,2 | 0,8 | 0,8 | 0,9 | 0,8 | 2,2 | 1,2 | 1,0 | 1,0 | 1,3 | 1,7 | 1,1 | 1,0 |
| Rnase1 | 1,3 | 0,9 | 1,6 | 128,7 | 1,0 | 1,0 | 1,0 | 1,0 | 0,5 | 0,7 | 2,2 | 9,3 | 2,3 | 0,3 | 1,0 | 1,0 | 5,4 | 0,2 | 0,3 | 1,0 | 0,9 | 0,8 |
| Rnase10 | 1,0 | 1,0 | 1,0 | 1,0 | 1,0 | 1,0 | 1,0 | 1,0 | 1,0 | 1,0 | 0,9 | 0,6 | 1,0 | 1,0 | 1020 | 1,0 | 1,0 | 1,0 | 1,0 | 1,0 | 1,0 | 1,0 |
| Rnase11 | 1,0 | 1,0 | 1,0 | 1,0 | 1,0 | 1,0 | 1,0 | 1,0 | 1,0 | 1,0 | 1,0 | 1,0 | 1,0 | 1,0 | 12,3 | 1,0 | 1,0 | 1,0 | 1,0 | 1,0 | 1,0 | 1,0 |
| *Rnase12* | *1,0* | *1,0* | *1,0* | *1,0* | *1,0* | *1,0* | *1,0* | *1,0* | *1,0* | *1,0* | *1,0* | *1,0* | *1,0* | *1,0* | *11,3* | *1,0* | *1,0* | *1,0* | *1,0* | *1,0* | *1,0* | *1,0* |
| Rnase13 | 1,0 | 1,0 | 1,0 | 1,0 | 1,0 | 1,0 | 1,0 | 1,0 | 1,0 | 1,0 | 1,0 | 1,0 | 1,0 | 1,0 | 130,7 | 1,0 | 1,0 | 1,0 | 1,0 | 1,0 | 1,0 | 1,0 |
| Rnase2a | 1,0 | 1,0 | 1,0 | 1,0 | 1,0 | 1,0 | 1,0 | 1,0 | 1,0 | 1,0 | 1,0 | 1,0 | 1,8 | 1,0 | 1,0 | 1,0 | 5,1 | 0,8 | 1,0 | 1,0 | 1,0 | 1,0 |
| Rnase9 | 1,0 | 1,0 | 1,0 | 1,0 | 1,0 | 1,0 | 1,0 | 1,0 | 1,0 | 1,0 | 1,0 | 1,0 | 1,0 | 1,0 | 72,9 | 1,0 | 1,0 | 1,0 | 1,0 | 1,0 | 1,0 | 1,0 |
| Rnf128 | 1,2 | 1,3 | 0,9 | 0,9 | 0,6 | 0,7 | 1,0 | 1,3 | 1,0 | 1,4 | 1,0 | 1,0 | 1,7 | 1,3 | 6,9 | 2,7 | 0,9 | 0,9 | 1,0 | 1,2 | 0,9 | 1,0 |
| Rnf149 | 1,2 | 1,3 | 1,1 | 1,1 | 1,0 | 1,0 | 1,0 | 1,3 | 0,9 | 1,1 | 1,0 | 0,9 | 1,1 | 1,0 | 5,7 | 1,0 | 0,9 | 1,0 | 1,0 | 1,0 | 1,0 | 1,0 |
| Rnf180 | 1,0 | 1,0 | 1,2 | 1,1 | 0,9 | 1,0 | 1,0 | 1,0 | 1,0 | 1,0 | 1,0 | 1,0 | 1,4 | 0,9 | 6,5 | 1,0 | 1,0 | 1,0 | 1,0 | 1,0 | 1,1 | 1,5 |
| *Rnf186* | *1,4* | *1,3* | *1,0* | *1,0* | *1,0* | *1,0* | *1,0* | *1,0* | *0,8* | *1,2* | *1,0* | *1,0* | *1,6* | *1,0* | *17,5* | *1,2* | *1,1* | *0,8* | *1,1* | *1,3* | *1,0* | *0,8* |
| Rnf24 | 0,3 | 0,3 | 1,1 | 0,9 | 1,0 | 0,9 | 1,2 | 0,8 | 2,3 | 1,5 | 1,0 | 1,5 | 1,0 | 1,3 | 1,4 | 0,8 | 1,1 | 1,3 | 1,2 | 0,8 | 1,4 | 1,7 |
| Rnf43 | 1,2 | 1,0 | 1,1 | 1,1 | 0,8 | 1,0 | 1,0 | 1,0 | 1,3 | 0,9 | 1,1 | 1,1 | 1,4 | 1,6 | 5,8 | 1,1 | 1,0 | 1,0 | 0,9 | 1,1 | 1,0 | 1,2 |
| Rnu12 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Rpl38 | 0,7 | 0,9 | 1,0 | 0,9 | 1,0 | 0,7 | 0,4 | 4,2 | 5,5 | 0,9 | 1,0 | 0,9 | 2,0 | 1,2 | 1,3 | 1,3 | 2,0 | 0,7 | 1,2 | 1,1 | 0,7 | 0,7 |
| Rpl3l | 0,7 | 1,0 | 1,0 | 1,0 | 0,7 | 0,5 | 0,9 | 1,1 | 1,0 | 1,0 | 1,0 | 1,0 | 1,0 | 1,0 | 4,6 | 1,0 | 1,0 | 1,0 | 1,0 | 1,0 | 1,0 | 1,0 |
| Rpph1 | 0,7 | 0,7 | 0,4 | 0,2 | 1,0 | 0,5 | 1,0 | 1,0 | 1,0 | 1,0 | 1,0 | 0,5 | 0,5 | 1,5 | 1,0 | 1,6 | 1,2 | 1,5 | 1,0 | 2,8 | 0,9 | 0,8 |
| Rprm | 1,7 | 1,5 | 0,6 | 1,0 | 1,0 | 1,0 | 1,0 | 1,0 | 1,0 | 1,0 | 1,0 | 1,0 | 2,0 | 1,3 | 16,3 | 1,0 | 1,0 | 1,3 | 1,0 | 1,0 | 1,0 | 1,0 |
| Rptn | 1,0 | 1,0 | 1,0 | 1,0 | 1,0 | 1,0 | 1,0 | 1,0 | 1,0 | 1,0 | 1,0 | 1,0 | 1,9 | 1,0 | 1,0 | 1,0 | 0,8 | 0,9 | 1,0 | 1,0 | 1,0 | 1,0 |
| Rtn2 | 1,0 | 1,2 | 1,4 | 1,1 | 0,8 | 0,6 | 1,0 | 1,1 | 1,0 | 1,0 | 1,0 | 1,1 | 1,0 | 1,0 | 0,4 | 1,2 | 0,6 | 0,8 | 0,9 | 0,9 | 2,2 | 0,9 |
| Rtp3 | 0,9 | 0,2 | 1,0 | 1,0 | 1,0 | 1,0 | 1,2 | 0,5 | 0,6 | 0,5 | 1,0 | 1,0 | 1,0 | 1,0 | 0,7 | 0,9 | 1,0 | 1,0 | 1,0 | 1,0 | 1,0 | 1,0 |
| Runx2 | 1,0 | 1,2 | 1,3 | 0,8 | 0,9 | 1,0 | 1,0 | 1,0 | 1,0 | 1,0 | 1,2 | 1,2 | 1,1 | 1,1 | 7,8 | 1,0 | 0,8 | 1,5 | 0,9 | 0,9 | 1,0 | 1,1 |
| Ryr1 | 1,0 | 1,0 | 1,0 | 1,0 | 0,7 | 0,4 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| S100a8 | 0,7 | 1,1 | 0,7 | 1,0 | 7,1 | 2,3 | 1,5 | 5,1 | 0,6 | 2,3 | 1,1 | 1,7 | 2,2 | 0,6 | 0,5 | 0,5 | 5,5 | 23,8 | 1,0 | 0,1 | 1,0 | 1,0 |
| S100a9 | 1,2 | 1,1 | 0,9 | 0,9 | 5,9 | 1,5 | 2,6 | 3,8 | 1,0 | 2,2 | 1,8 | 1,0 | 1,7 | 0,6 | 1,2 | 0,8 | 1,4 | 4,2 | 1,0 | 0,1 | 1,0 | 1,0 |
| S100g | 3,0 | 5,4 | 0,1 | 1,4 | 1,0 | 1,0 | 1,0 | 1,0 | 1,0 | 1,0 | 1,0 | 1,0 | 1,6 | 1,0 | 1,0 | 1,0 | 3,0 | 0,7 | 5,3 | 4,6 | 0,3 | 0,4 |

[Fig. 23-27]



| | | | | | | | | | | | | | | | | | | | | | | |
|---|---|---|---|---|---|---|---|---|---|---|---|---|---|---|---|---|---|---|---|---|---|---|
| Saa1 | 1,2 | 1,1 | 1,2 | 1,3 | 1,0 | 1,0 | 1,0 | 1,0 | 0,3 | 0,2 | 1,0 | 1,0 | 1,2 | 0,3 | 1,0 | 1,0 | 1,0 | 0,7 | 6,9 | 4,5 | 2,0 | 1,3 |
| Saa2 | 1,0 | 1,0 | 1,4 | 1,1 | 1,0 | 1,0 | 1,0 | 1,0 | 0,3 | 0,2 | 1,0 | 1,0 | 1,2 | 1,0 | 1,0 | 1,0 | 1,0 | 1,0 | 1,0 | 1,0 | 1,7 | 0,8 |
| Saa3 | 1,0 | 1,0 | 0,7 | 0,3 | 1,0 | 1,0 | 1,9 | 1,9 | 1,1 | 3,3 | 1,0 | 1,0 | 1,8 | 0,4 | 0,3 | 0,7 | 5,0 | 2,7 | 3,0 | 1,0 | 0,7 | 0,2 |
| *Sash3* | *1,0* | *1,1* | *0,6* | *0,4* | *1,0* | *1,0* | *0,7* | *1,0* | *1,0* | *1,0* | *0,8* | *1,1* | *0,7* | *0,8* | *0,6* | *0,6* | *1,0* | *1,2* | *0,8* | *0,7* | *0,7* | *0,7* |
| Sbk2 | 1,0 | 1,0 | 1,0 | 1,0 | 0,6 | 0,7 | 0,8 | 0,8 | 1,0 | 1,0 | 1,0 | 1,0 | 1,0 | 1,0 | 0,6 | 1,7 | 1,0 | 1,0 | 1,0 | 1,0 | 1,0 | 1,0 |
| Sbp | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Sbpl | 1,0 | 1,0 | 1,0 | 1,0 | 1,0 | 1,0 | 1,0 | 1,0 | 1,0 | 1,0 | 1,0 | 1,0 | 1,7 | 0,9 | 1,0 | 1,0 | 1,0 | 1,0 | 1,0 | 1,0 | 1,0 | 1,0 |
| *Scara5* | *1,3* | *0,8* | *1,4* | *1,2* | *0,8* | *0,7* | *1,1* | *1,0* | *0,4* | *0,2* | *0,7* | *0,8* | *1,5* | *2,0* | *0,7* | *0,6* | *0,8* | *0,9* | *1,0* | *1,0* | *1,2* | *1,0* |
| Scarna3b | 1,0 | 1,0 | 1,0 | 1,0 | 1,0 | 1,0 | 1,0 | 1,0 | 1,0 | 1,0 | 1,0 | 1,0 | 0,0 | 1,0 | 1,0 | 1,0 | 1,0 | 1,0 | 1,0 | 1,0 | 1,0 | 1,0 |
| Scarna8 | 1,0 | 1,0 | 1,0 | 1,0 | 1,0 | 1,0 | 1,0 | 1,0 | 1,0 | 1,0 | 1,0 | 1,0 | 1,0 | 0,0 | 1,0 | 1,0 | 1,0 | 1,0 | 1,0 | 1,0 | 1,0 | 1,0 |
| Scd1 | 3,1 | 2,9 | 0,4 | 1,1 | 0,6 | 0,7 | 2,5 | 0,6 | 1,5 | 0,5 | 0,8 | 2,3 | 0,7 | 2,8 | 0,5 | 1,1 | 0,4 | 0,6 | 0,5 | 0,8 | 0,4 | 1,6 |
| *Scd2* | *1,5* | *2,0* | *1,0* | *0,8* | *1,0* | *1,0* | *1,3* | *0,8* | *2,1* | *0,6* | *0,9* | *0,9* | *0,8* | *0,8* | *1,6* | *0,5* | *1,0* | *0,9* | *1,9* | *1,0* | *1,2* | *0,7* |
| Slc15a2 | 2,0 | 2,6 | 0,5 | 1,0 | 1,1 | 0,9 | 1,0 | 0,8 | 2,5 | 0,3 | 1,0 | 1,6 | 0,6 | 2,2 | 0,7 | 0,7 | 0,8 | 0,7 | 0,7 | 1,0 | 0,5 | 2,0 |
| Scd4 | 1,0 | 1,0 | 1,0 | 1,0 | 1,0 | 1,0 | 0,4 | 1,2 | 2,6 | 0,1 | 1,0 | 1,0 | 1,1 | 1,7 | 0,6 | 0,7 | 1,0 | 1,0 | 1,0 | 1,0 | 0,9 | 1,0 |
| Snora64 | 1,2 | 1,0 | 1,0 | 8,4 | 1,0 | 0,8 | 1,0 | 0,9 | 1,4 | 1,0 | 1,3 | 1,0 | 0,8 | 10,1 | 1,0 | 12,7 | 0,7 | 0,9 | 1,0 | 0,6 | 1,0 | 1,0 |
| *Acan* | *1,7* | *1,0* | *1,0* | *1,0* | *1,0* | *0,9* | *1,0* | *0,7* | *0,5* | *1,0* | *1,0* | *0,8* | *1,0* | *2,8* | *1,0* | *12,5* | *1,0* | *1,3* | *1,0* | *1,0* | *1,0* | *1,0* |
| Scgb2b12 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Scgb2b15 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 2,0 | 0,8 | 0,3 |
| Scgb2b17 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 2,0 | 1,0 | 0,2 |
| *Scgb2b20* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* |
| Scgb2b27 | 1,2 | 1,0 | 1,0 | 1,0 | 1,0 | 0,6 | 1,0 | 0,6 | 0,7 | 1,0 | 1,0 | 0,8 | 1,0 | 2,0 | 1,0 | 6,2 | 1,0 | 1,5 | 1,0 | 1,0 | 1,0 | 1,0 |
| Scgb3a1 | 1,0 | 1,0 | 1,0 | 1,0 | 1,0 | 1,0 | 2,0 | 1,2 | 1,0 | 1,0 | 1,0 | 1,0 | 1,0 | 1,0 | 1,0 | 1,0 | 0,9 | 1,0 | 0,6 | 0,6 | 0,5 | 0,4 |
| Scgb3a2 | 1,0 | 1,0 | 1,0 | 1,0 | 1,0 | 1,0 | 1,0 | 1,3 | 1,0 | 1,0 | 1,0 | 1,0 | 1,0 | 1,0 | 1,0 | 1,0 | 1,0 | 1,0 | 1,0 | 1,0 | 1,0 | 1,0 |
| *Scn4b* | *1,2* | *1,1* | *1,0* | *1,0* | *0,7* | *0,6* | *0,8* | *0,7* | *1,0* | *1,0* | *1,0* | *1,0* | *0,7* | *0,9* | *1,1* | *0,8* | *0,9* | *0,8* | *1,0* | *1,0* | *1,0* | *1,0* |
| Scx | 1,1 | 1,3 | 2,3 | 1,0 | 1,0 | 0,8 | 0,7 | 1,7 | 1,0 | 1,0 | 1,1 | 0,6 | 2,5 | 2,7 | 5,9 | 1,0 | 1,4 | 1,1 | 1,0 | 1,0 | 1,0 | 1,0 |
| Sec14l3 | 0,3 | 0,2 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Selenbp2 | 0,7 | 0,7 | 1,2 | 1,2 | 0,8 | 1,2 | 1,2 | 1,1 | 0,3 | 0,2 | 1,3 | 1,3 | 1,5 | 1,0 | 0,8 | 1,6 | 1,3 | 0,8 | 1,1 | 1,0 | 1,0 | 1,1 |
| Sell | 1,0 | 1,0 | 0,2 | 0,3 | 1,0 | 1,0 | 1,0 | 1,0 | 1,0 | 1,0 | 0,5 | 2,3 | 0,9 | 1,0 | 1,0 | 0,8 | 1,1 | 1,0 | 1,0 | 0,7 | 0,6 | 0,8 |
| Serinc2 | 0,8 | 0,8 | 1,1 | 1,0 | 0,7 | 0,9 | 1,0 | 1,0 | 1,2 | 1,6 | 1,0 | 0,8 | 2,0 | 0,9 | 23,4 | 1,0 | 1,1 | 1,0 | 1,2 | 1,3 | 1,1 | 1,0 |
| Serpina10 | 4,0 | 11,1 | 0,6 | 1,0 | 1,0 | 1,0 | 1,0 | 1,0 | 0,9 | 1,0 | 1,0 | 1,0 | 1,0 | 1,0 | 1,0 | 1,0 | 1,0 | 1,0 | 1,0 | 1,0 | 1,2 | 1,0 |
| Serpina12 | 1,0 | 1,0 | 1,0 | 1,0 | 1,0 | 1,0 | 1,0 | 1,0 | 0,3 | 0,2 | 1,0 | 1,0 | 1,0 | 1,0 | 1,0 | 1,0 | 0,9 | 0,9 | 1,0 | 1,0 | 1,0 | 1,0 |
| Serpina1a | 4,6 | 4,0 | 0,2 | 1,0 | 1,0 | 1,0 | 1,0 | 1,0 | 0,5 | 0,5 | 1,0 | 1,0 | 1,0 | 1,0 | 0,3 | 0,4 | 14,5 | 0,1 | 0,9 | 0,7 | 0,8 | 1,2 |
| Serpina1b | 4,4 | 3,3 | 0,2 | 1,0 | 1,0 | 1,0 | 1,0 | 1,0 | 0,5 | 0,6 | 0,8 | 1,0 | 1,0 | 1,0 | 0,3 | 0,8 | 20,2 | 0,0 | 1,0 | 1,3 | 0,8 | 1,0 |
| Serpina1c | 3,9 | 3,1 | 0,2 | 1,0 | 1,0 | 1,0 | 1,0 | 1,0 | 0,4 | 0,4 | 0,9 | 1,0 | 1,0 | 1,0 | 0,3 | 0,5 | 21,7 | 0,0 | 0,7 | 1,0 | 0,8 | 0,7 |
| Serpina1d | 2,1 | 1,5 | 0,2 | 1,0 | 1,0 | 1,0 | 1,0 | 1,0 | 0,5 | 0,5 | 1,2 | 1,0 | 1,0 | 1,0 | 0,3 | 0,6 | 13,0 | 0,1 | 1,0 | 1,0 | 1,1 | 1,0 |
| Serpina1e | 3,2 | 3,1 | 0,2 | 1,0 | 1,0 | 1,0 | 1,0 | 1,0 | 0,3 | 0,1 | 1,0 | 1,0 | 1,0 | 1,0 | 0,3 | 0,7 | 4,5 | 0,0 | 1,0 | 2,6 | 1,1 | 0,9 |
| Serpina1f | 1,2 | 1,2 | 1,0 | 1,0 | 1,0 | 1,0 | 1,0 | 1,0 | 1,0 | 1,0 | 1,0 | 1,0 | 1,0 | 1,0 | 18,2 | 11,1 | 1,0 | 1,0 | 1,0 | 1,0 | 1,0 | 1,0 |
| Serpina3c | 1,0 | 1,0 | 0,9 | 1,6 | 1,0 | 1,0 | 1,3 | 1,0 | 0,3 | 0,5 | 1,0 | 1,0 | 2,0 | 2,1 | 0,7 | 0,9 | 1,0 | 1,0 | 1,0 | 1,0 | 0,8 | 1,0 |
| Serpina3k | 2,0 | 1,0 | 0,2 | 1,0 | 1,0 | 1,0 | 1,0 | 1,0 | 0,3 | 0,3 | 0,8 | 1,0 | 1,0 | 1,0 | 0,3 | 0,9 | 13,3 | 0,0 | 1,0 | 1,0 | 1,0 | 1,0 |
| Serpina4-ps1 | 1,0 | 1,0 | 1,0 | 1,0 | 1,0 | 1,0 | 1,0 | 1,0 | 0,3 | 0,1 | 1,0 | 1,0 | 1,0 | 1,0 | 1,0 | 1,0 | 1,0 | 0,6 | 1,0 | 1,0 | 1,0 | 1,0 |
| Serpinb11 | 1,0 | 1,0 | 1,0 | 1,0 | 1,2 | 1,6 | 1,0 | 1,0 | 1,0 | 1,0 | 1,1 | 1,0 | 2,4 | 1,4 | 1,0 | 1,0 | 0,9 | 1,2 | 1,0 | 1,0 | 1,0 | 1,0 |
| Serpinb3a | 1,0 | 1,0 | 1,0 | 1,0 | 0,7 | 1,2 | 1,0 | 1,0 | 1,0 | 1,0 | 1,0 | 1,0 | 0,2 | 0,4 | 1,0 | 1,0 | 1,1 | 1,0 | 1,0 | 1,0 | 1,0 | 1,0 |
| Serpinb6d | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Serpinb6e | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Serpinc1 | 1,0 | 2,2 | 0,7 | 1,0 | 1,0 | 1,0 | 1,0 | 1,0 | 1,1 | 1,2 | 1,0 | 1,0 | 1,0 | 0,7 | 1,0 | 1,0 | 6,0 | 0,3 | 1,0 | 1,0 | 1,0 | 1,0 |
| Serpinf2 | 0,4 | 0,4 | 1,0 | 1,0 | 1,0 | 1,0 | 1,0 | 1,0 | 0,7 | 0,8 | 1,0 | 1,0 | 1,0 | 1,0 | 11,5 | 1,0 | 2,0 | 0,7 | 0,8 | 1,4 | 1,0 | 1,0 |
| Sfrp5 | 1,0 | 1,0 | 1,0 | 0,9 | 0,7 | 0,7 | 0,8 | 1,0 | 1,0 | 1,0 | 1,2 | 0,9 | 1,0 | 1,0 | 0,2 | 0,2 | 0,8 | 0,8 | 0,4 | 0,5 | 0,5 | 0,6 |
| Sgca | 1,0 | 1,0 | 1,0 | 1,0 | 0,8 | 0,8 | 1,2 | 1,1 | 1,0 | 1,0 | 1,0 | 1,0 | 1,0 | 1,0 | 1,0 | 1,0 | 1,0 | 1,0 | 1,0 | 1,0 | 1,0 | 1,0 |
| Sgcg | 1,0 | 1,0 | 1,0 | 1,0 | 0,8 | 0,8 | 1,0 | 1,1 | 1,0 | 1,0 | 1,0 | 1,0 | 1,0 | 1,0 | 1,0 | 1,0 | 1,0 | 1,0 | 1,0 | 1,0 | 1,0 | 1,0 |
| Sh3bp2 | 0,3 | 0,4 | 1,0 | 0,7 | 0,9 | 0,9 | 0,9 | 1,0 | 1,0 | 1,0 | 0,8 | 1,0 | 1,2 | 0,9 | 1,1 | 0,6 | 0,9 | 1,1 | 1,0 | 1,0 | 1,4 | 1,1 |
| Sh3gl2 | 0,8 | 0,8 | 1,1 | 1,1 | 1,0 | 1,1 | 1,0 | 1,0 | 1,0 | 1,0 | 1,0 | 1,0 | 1,0 | 1,0 | 7,3 | 1,0 | 0,9 | 0,9 | 1,1 | 1,0 | 1,1 | 1,3 |
| Shc4 | 1,0 | 1,0 | 1,0 | 1,0 | 0,9 | 0,8 | 1,0 | 1,0 | 1,0 | 1,0 | 1,0 | 1,0 | 1,0 | 1,0 | 5,2 | 1,0 | 1,0 | 1,0 | 1,0 | 1,0 | 1,0 | 1,0 |
| Shh | 1,0 | 1,0 | 1,0 | 1,0 | 1,0 | 0,8 | 1,0 | 1,0 | 1,0 | 1,0 | 1,0 | 1,0 | 1,0 | 1,0 | 3,2 | 1,0 | 0,8 | 0,9 | 1,0 | 1,0 | 1,0 | 1,0 |
| Foxn3 | 0,7 | 1,0 | 0,2 | 0,7 | 0,4 | 1,0 | 0,3 | 0,8 | 0,5 | 1,4 | 0,7 | 1,0 | 0,2 | 0,5 | 0,7 | 1,3 | 0,5 | 0,9 | 0,3 | 0,9 | 0,2 | 1,2 |
| Slc16a3 | 1,0 | 1,1 | 0,9 | 0,8 | 0,9 | 0,8 | 1,3 | 1,2 | 1,0 | 1,0 | 0,6 | 0,4 | 1,8 | 0,7 | 1,3 | 1,4 | 1,0 | 1,7 | 1,5 | 1,0 | 1,3 | 1,1 |
| Slc16a9 | 1,1 | 1,0 | 1,3 | 1,3 | 1,0 | 0,9 | 0,9 | 0,7 | 1,1 | 1,1 | 1,0 | 1,0 | 1,3 | 0,8 | 0,7 | 0,6 | 1,0 | 1,1 | 5,4 | 2,1 | 1,0 | 1,0 |
| Slc17a9 | 1,0 | 1,0 | 1,0 | 1,1 | 1,1 | 1,1 | 1,0 | 1,0 | 1,0 | 0,6 | 1,1 | 1,1 | 1,6 | 1,4 | 12,0 | 1,1 | 1,3 | 1,1 | 0,9 | 0,9 | 0,9 | 0,9 |
| Slc1a1 | 0,7 | 0,8 | 1,0 | 1,0 | 1,0 | 1,0 | 0,6 | 1,0 | 1,0 | 1,0 | 1,0 | 1,0 | 1,0 | 1,0 | 8,1 | 0,5 | 1,0 | 1,0 | 1,1 | 1,0 | 0,6 | 0,9 |
| Slc22a26 | 0,8 | 0,5 | 1,0 | 1,0 | 1,0 | 1,0 | 1,0 | 1,0 | 2,5 | 9,6 | 1,0 | 1,0 | 1,0 | 1,0 | 1,0 | 1,0 | 1,0 | 1,0 | 1,0 | 1,0 | 1,0 | 1,0 |
| Slc22a27 | 0,7 | 1,0 | 1,0 | 1,0 | 1,0 | 1,0 | 1,0 | 1,0 | 3,2 | 8,7 | 1,0 | 1,0 | 1,0 | 1,0 | 1,0 | 1,0 | 1,0 | 1,0 | 1,0 | 1,0 | 1,0 | 1,0 |
| Slc22a28 | 0,3 | 0,2 | 1,0 | 1,0 | 1,0 | 1,0 | 1,0 | 1,0 | 0,5 | 0,4 | 1,0 | 1,0 | 1,0 | 1,0 | 1,0 | 1,0 | 1,0 | 1,0 | 1,0 | 1,0 | 1,0 | 1,0 |
| Slc22a30 | 0,3 | 0,2 | 1,0 | 1,0 | 1,0 | 1,0 | 1,0 | 1,0 | 0,5 | 0,5 | 1,0 | 1,0 | 1,0 | 1,0 | 1,0 | 1,0 | 1,0 | 1,0 | 1,0 | 1,0 | 1,0 | 1,0 |
| Slc22a7 | 0,2 | 0,1 | 1,0 | 1,0 | 1,0 | 1,0 | 1,0 | 1,0 | 0,3 | 0,1 | 1,0 | 1,0 | 0,6 | 1,6 | 1,0 | 1,0 | 1,0 | 1,0 | 1,0 | 1,0 | 1,0 | 1,0 |
| Slc25a30 | 1,4 | 1,0 | 1,2 | 1,0 | 0,8 | 0,8 | 0,8 | 0,8 | 0,3 | 0,4 | 0,8 | 1,0 | 0,8 | 1,0 | 1,0 | 0,7 | 0,9 | 1,6 | 1,0 | 0,7 | 1,2 | 1,0 |
| Slc25a47 | 1,2 | 0,9 | 0,7 | 1,3 | 0,9 | 0,9 | 1,1 | 1,4 | 1,1 | 1,1 | 1,0 | 1,1 | 1,2 | 1,1 | 5,4 | 1,9 | 2,7 | 0,7 | 1,4 | 0,8 | 0,8 | 1,2 |
| Slc30a10 | 1,0 | 1,0 | 4,7 | 28,6 | 1,3 | 1,0 | 1,0 | 1,0 | 1,3 | 0,6 | 1,0 | 1,0 | 1,0 | 1,0 | 1,0 | 1,0 | 1,0 | 1,0 | 2,0 | 1,7 | 5,1 | 14,7 |
| | Kidney | | Colon | | Eyeball | | Heart | | Liver | | SG | | Thymus | | Adipose | | Stomach | | Jejunum | | Ileum | |
| | E | M | E | M | E | M | E | M | E | M | E | M | E | M | E | M | E | M | E | M | E | M |
| Slc34a2 | 1,9 | 2,1 | 1,1 | 1,4 | 1,0 | 1,3 | 1,0 | 1,0 | 1,0 | 1,0 | 1,0 | 1,0 | 1,0 | 1,0 | 7,0 | 1,0 | 0,8 | 0,9 | 1,0 | 0,4 | 0,9 | 0,8 |
| Slc35f2 | 1,0 | 0,9 | 1,8 | 1,2 | 1,2 | 1,1 | 1,0 | 1,0 | 1,0 | 1,0 | 1,0 | 1,0 | 0,9 | 0,9 | 6,8 | 1,1 | 1,3 | 1,0 | 1,5 | 1,3 | 1,3 | 1,3 |
| Slc38a3 | 4,3 | 5,2 | 0,8 | 1,0 | 1,0 | 1,1 | 1,0 | 1,3 | 0,5 | 0,6 | 1,0 | 1,0 | 1,0 | 1,0 | 0,7 | 1,4 | 3,2 | 0,9 | 1,0 | 1,0 | 1,0 | 1,0 |
| Slc38a5 | 1,0 | 1,0 | 1,0 | 1,0 | 1,0 | 1,0 | 1,0 | 1,0 | 1,0 | 1,0 | 1,0 | 1,0 | 1,0 | 1,0 | 97,0 | 1,0 | 0,7 | 0,6 | 1,0 | 1,0 | 1,0 | 1,0 |
| Slc43a1 | 1,2 | 1,1 | 1,0 | 1,0 | 1,1 | 0,9 | 1,6 | 1,5 | 0,3 | 0,2 | 1,5 | 1,2 | 0,7 | 1,1 | 1,4 | 3,0 | 1,0 | 0,8 | 1,0 | 1,0 | 1,0 | 1,3 |
| Slc4a1 | 1,2 | 1,2 | 1,0 | 1,0 | 1,4 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Slc51b | 0,9 | 1,3 | 1,0 | 1,6 | 1,0 | 1,0 | 1,0 | 1,0 | 1,0 | 5,9 | 1,0 | 1,0 | 1,0 | 1,0 | 1,0 | 1,0 | 1,0 | 1,0 | 1,7 | 3,6 | 0,9 | 0,7 |
| Slc7a12 | 1,9 | 7,2 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Slc7a5 | 1,3 | 1,3 | 1,0 | 1,1 | 1,2 | 1,1 | 1,0 | 1,3 | 1,4 | 1,1 | 1,1 | 1,6 | 0,8 | 1,0 | 24,0 | 1,2 | 1,0 | 1,2 | 1,1 | 1,0 | 1,0 | 0,9 |
| Slc7a8 | 1,3 | 1,2 | 1,0 | 1,4 | 0,9 | 1,0 | 1,2 | 1,1 | 0,8 | 1,0 | 0,9 | 1,0 | 1,3 | 1,1 | 2,0 | 0,6 | 1,3 | 1,3 | 0,9 | 1,0 | 0,7 | 1,2 |
| Slc9a8 | 0,3 | 0,2 | 1,0 | 1,0 | 0,9 | 0,9 | 1,1 | 1,0 | 1,1 | 1,1 | 0,8 | 1,0 | 0,9 | 1,2 | 1,2 | 1,1 | 1,1 | 1,2 | 1,0 | 0,9 | 0,9 | 1,0 |
| Slco1a1 | 0,1 | 0,0 | 1,0 | 1,0 | 1,0 | 1,0 | 1,0 | 1,0 | 0,3 | 0,0 | 1,0 | 1,0 | 1,0 | 1,0 | 1,0 | 1,0 | 1,0 | 1,0 | 1,0 | 1,0 | 1,0 | 1,0 |
| Slco1a4 | 1,0 | 1,0 | 1,0 | 1,0 | 1,0 | 1,1 | 1,0 | 1,0 | 5,4 | 9,6 | 1,0 | 1,0 | 1,0 | 1,0 | 1,0 | 1,0 | 1,0 | 1,0 | 1,0 | 1,0 | 1,0 | 1,0 |
| Slfn4 | 1,0 | 1,0 | 0,9 | 0,7 | 1,0 | 1,0 | 1,0 | 1,0 | 1,0 | 1,0 | 1,0 | 1,0 | 1,1 | 1,0 | 1,0 | 1,0 | 0,3 | 1,8 | 1,2 | 1,1 | 1,2 | 0,8 |
| Slpi | 1,0 | 1,0 | 0,9 | 0,4 | 1,0 | 1,0 | 1,1 | 1,0 | 1,8 | 1,9 | 0,4 | 1,0 | 3,4 | 1,4 | 1,2 | 1,5 | 1,2 | 0,9 | 1,1 | 0,7 | 0,7 | 0,8 |
| Smco1 | 1,0 | 1,0 | 1,0 | 1,0 | 0,7 | 0,7 | 1,0 | 0,9 | 1,0 | 1,0 | 1,0 | 1,0 | 1,0 | 1,0 | 1,0 | 1,0 | 1,0 | 1,0 | 1,0 | 1,0 | 1,0 | 1,0 |

[Fig. 23-28]

| | | | | | | | | | | | | | | | | | | | | | | |
|---|---|---|---|---|---|---|---|---|---|---|---|---|---|---|---|---|---|---|---|---|---|---|
| Smcp | 0,3 | 0,2 | 1,0 | 1,0 | 1,0 | 1,0 | 1,0 | 1,0 | 1,0 | 1,0 | 1,0 | 1,0 | 1,0 | 1,0 | 14,7 | 7,9 | 1,0 | 1,0 | 1,0 | 1,0 | 1,0 | 0,6 |
| Smim24 | 1,0 | 1,0 | 1,3 | 1,5 | 0,6 | 0,7 | 0,5 | 0,9 | 1,0 | 1,0 | 1,0 | 1,0 | 0,9 | 1,0 | 0,9 | 2,5 | 1,0 | 0,9 | 1,1 | 1,1 | 0,9 | 0,9 |
| Smpx | 1,0 | 1,0 | 1,6 | 1,0 | 0,6 | 1,0 | 1,0 | 1,0 | 1,0 | 1,0 | 1,0 | 1,0 | 1,0 | 1,0 | 1,0 | 1,0 | 0,5 | 1,6 | 0,9 | 1,1 | 0,8 | 0,5 |
| Smr3a | 1,1 | 1,0 | 1,0 | 1,0 | 1,0 | 1,3 | 1,0 | 0,6 | 1,0 | 1,0 | 2,1 | 5,0 | 1,0 | 2,7 | 1,0 | 1,4 | 1,0 | 2,0 | 1,0 | 1,0 | 1,0 | 1,0 |
| Smtnl1 | 1,0 | 1,0 | 1,0 | 1,0 | 0,5 | 0,6 | 1,0 | 1,0 | 1,0 | 1,0 | 1,0 | 1,0 | 1,6 | 1,0 | 1,0 | 1,0 | 1,0 | 1,0 | 1,0 | 1,0 | 1,0 | 1,0 |
| Smtnl2 | 1,3 | 1,3 | 1,4 | 1,1 | 0,6 | 0,5 | 0,9 | 0,7 | 1,0 | 1,0 | 1,0 | 1,0 | 1,6 | 1,1 | 1,6 | 0,6 | 1,3 | 1,4 | 1,7 | 1,5 | 1,1 | 1,0 |
| Smyd1 | 1,0 | 1,0 | 1,0 | 1,0 | 0,8 | 0,8 | 1,0 | 1,0 | 1,1 | 0,8 | 1,0 | 1,0 | 0,5 | 0,8 | 1,0 | 0,8 | 1,0 | 1,3 | 1,0 | 1,0 | 1,0 | 1,0 |
| Sncg | 0,4 | 0,9 | 0,9 | 0,9 | 1,0 | 1,0 | 1,0 | 1,2 | 1,0 | 1,0 | 1,7 | 1,2 | 1,8 | 1,0 | 0,3 | 0,6 | 0,6 | 0,7 | 0,7 | 1,1 | 0,8 | 0,7 |
| Hipk2 | 1,0 | 1,0 | 1,0 | 0,0 | 1,0 | 1,0 | 1,0 | 1,0 | 1,0 | 1,0 | 1,0 | 1,0 | 1,0 | 1,0 | 1,0 | 1,0 | 1,0 | 0,0 | 1,0 | 1,0 | 1,0 | 1,0 |
| Nptxr | 0,0 | 0,0 | 0,6 | 21,8 | 48,8 | 1,0 | 71,0 | 1,1 | 1,0 | 0,3 | 32,8 | 1,0 | 0,6 | 0,9 | 1,0 | 0,0 | 1,0 | 22,9 | 1,0 | 26,3 | 1,0 | 0,0 |
| *Socs1* | *1,4* | *0,6* | *0,6* | *0,6* | *1,0* | *0,7* | *1,4* | *1,2* | *0,3* | *1,0* | *1,0* | *1,0* | *0,9* | *0,7* | *0,6* | *1,3* | *1,7* | *1,0* | *2,2* | *0,9* | *0,9* | *0,8* |
| Socs3 | 2,8 | 4,0 | 1,6 | 0,8 | 0,8 | 0,9 | 1,4 | 1,1 | 1,2 | 1,4 | 0,8 | 1,0 | 1,3 | 1,2 | 0,8 | 0,8 | 1,1 | 1,0 | 1,5 | 2,4 | 1,8 | 0,9 |
| Sost | 1,0 | 1,0 | 1,0 | 1,0 | 1,0 | 1,0 | 2,5 | 1,3 | 1,0 | 1,0 | 1,0 | 1,0 | 1,0 | 1,0 | 1,0 | 7,5 | 1,0 | 1,0 | 1,0 | 1,0 | 1,0 | 1,0 |
| Sox6 | 1,0 | 1,0 | 1,0 | 1,3 | 1,1 | 0,9 | 1,0 | 0,8 | 1,1 | 0,9 | 1,0 | 1,0 | 1,0 | 1,0 | 0,8 | 0,8 | 0,8 | 0,9 | 0,9 | 0,8 | 1,0 | 1,5 |
| Spag11a | 1,0 | 1,0 | 1,0 | 1,0 | 1,0 | 1,0 | 1,0 | 1,0 | 1,0 | 1,0 | 1,0 | 1,0 | 1,0 | 1,0 | 317,6 | 1,0 | 1,0 | 1,0 | 1,0 | 1,0 | 1,0 | 1,0 |
| Spag11b | 1,0 | 1,0 | 1,0 | 1,0 | 1,0 | 1,0 | 1,0 | 1,0 | 1,0 | 1,0 | 1,0 | 1,0 | 1,0 | 1,0 | 3453 | 1,0 | 1,0 | 1,0 | 1,0 | 1,0 | 1,0 | 1,0 |
| Sparc | 0,9 | 1,5 | 1,1 | 0,8 | 0,8 | 0,9 | 0,6 | 0,6 | 0,8 | 0,9 | 0,4 | 0,4 | 0,9 | 1,2 | 0,3 | 0,6 | 1,4 | 1,4 | 1,1 | 1,1 | 0,8 | 0,8 |
| Spata3 | 1,0 | 1,0 | 1,0 | 1,0 | 1,0 | 1,0 | 1,0 | 1,0 | 1,0 | 1,0 | 1,0 | 1,0 | 1,0 | 1,0 | 8,4 | 2,4 | 1,0 | 1,0 | 1,0 | 1,0 | 1,0 | 1,0 |
| Spc24 | 0,9 | 2,6 | 0,8 | 0,7 | 1,0 | 0,8 | 0,7 | 1,1 | 1,3 | 1,0 | 1,0 | 1,0 | 0,6 | 0,7 | 1,0 | 1,0 | 0,9 | 0,7 | 1,9 | 1,1 | 1,1 | 0,9 |
| Spin2c | 1,0 | 1,0 | 1,0 | 1,0 | 0,7 | 1,4 | 1,0 | 1,0 | 1,0 | 1,0 | 1,0 | 1,0 | 1,9 | 1,1 | 13,5 | 1,0 | 0,8 | 1,0 | 1,0 | 1,0 | 1,0 | 1,0 |
| Spink10 | 1,0 | 1,0 | 1,0 | 1,0 | 1,0 | 1,0 | 1,0 | 1,0 | 1,0 | 1,0 | 1,0 | 1,0 | 1,0 | 1,0 | 7,0 | 1,0 | 1,0 | 1,0 | 1,0 | 1,0 | 1,0 | 1,0 |
| Spink11 | 1,0 | 1,0 | 1,0 | 1,0 | 1,0 | 1,0 | 1,0 | 1,0 | 1,0 | 1,0 | 1,0 | 1,0 | 1,0 | 1,0 | 467,5 | 1,0 | 1,0 | 1,0 | 1,0 | 1,0 | 1,0 | 1,0 |
| Spink12 | 1,0 | 1,0 | 1,0 | 1,0 | 1,0 | 1,0 | 1,0 | 1,0 | 1,0 | 1,0 | 1,0 | 1,0 | 1,0 | 1,0 | 75,6 | 1,0 | 1,1 | 2,6 | 1,0 | 1,0 | 1,0 | 1,0 |
| *Spink2* | *1,0* | *1,0* | *1,0* | *1,0* | *1,0* | *1,0* | *1,0* | *1,0* | *1,0* | *1,0* | *1,0* | *1,0* | *1,0* | *1,0* | *96,2* | *1,0* | *1,0* | *1,0* | *1,0* | *1,0* | *1,0* | *1,0* |
| Spink3 | 0,8 | 1,0 | 1,4 | 1,1 | 1,0 | 1,0 | 1,0 | 1,0 | 1,0 | 1,0 | 1,0 | 1,0 | 1,9 | 1,0 | 1,0 | 1,0 | 5,6 | 0,2 | 1,0 | 1,4 | 0,9 | 0,6 |
| Spink4 | 1,0 | 1,0 | 0,6 | 0,3 | 1,0 | 1,0 | 1,0 | 1,0 | 1,0 | 1,0 | 1,0 | 1,0 | 1,0 | 1,0 | 1,0 | 1,0 | 1,9 | 0,8 | 1,2 | 1,3 | 1,0 | 0,8 |
| Spink5 | 1,0 | 1,0 | 1,0 | 1,0 | 1,0 | 1,0 | 1,0 | 1,0 | 1,0 | 1,0 | 1,0 | 1,0 | 1,7 | 1,3 | 24,5 | 1,0 | 1,0 | 0,9 | 1,0 | 1,0 | 1,0 | 1,0 |
| Spink8 | 1,3 | 1,7 | 1,0 | 1,0 | 1,0 | 1,0 | 1,0 | 1,0 | 1,0 | 1,0 | 1,0 | 1,0 | 1,0 | 1,0 | 9,0 | 34,1 | 1,0 | 1,0 | 1,0 | 1,0 | 1,0 | 1,0 |
| Spint1 | 1,1 | 1,2 | 1,0 | 1,3 | 0,8 | 1,3 | 1,0 | 1,0 | 1,0 | 1,0 | 1,1 | 0,9 | 2,1 | 1,5 | 5,1 | 2,0 | 0,9 | 1,0 | 1,0 | 1,3 | 0,9 | 1,0 |
| Spint2 | 1,1 | 1,2 | 1,0 | 1,0 | 1,0 | 1,1 | 1,0 | 1,1 | 0,8 | 1,2 | 1,0 | 0,8 | 1,1 | 1,0 | 5,6 | 2,9 | 1,1 | 1,0 | 1,1 | 1,3 | 1,1 | 0,9 |
| Spint4 | 1,0 | 1,0 | 1,0 | 1,0 | 1,0 | 1,0 | 1,0 | 1,0 | 1,0 | 1,0 | 1,0 | 1,0 | 1,0 | 1,0 | 212,4 | 1,0 | 1,0 | 1,0 | 1,0 | 1,0 | 1,0 | 1,0 |
| Spint5 | 1,0 | 1,0 | 1,0 | 1,0 | 1,0 | 1,0 | 1,0 | 1,0 | 1,0 | 1,0 | 1,0 | 1,0 | 1,0 | 1,0 | 49,9 | 1,0 | 1,0 | 1,0 | 1,0 | 1,0 | 1,0 | 1,0 |
| Spp1 | 4,1 | 7,9 | 1,6 | 1,0 | 1,0 | 1,3 | 6,1 | 4,3 | 0,7 | 1,3 | 0,6 | 0,5 | 1,4 | 0,5 | 2,5 | 2,3 | 0,5 | 0,8 | 1,0 | 1,0 | 1,0 | 1,0 |
| Sprr1a | 1,2 | 1,9 | 1,0 | -0,6 | 1,3 | 4,5 | 1,0 | 1,0 | 1,0 | 1,0 | 1,0 | 1,0 | 1,9 | 0,8 | 1,0 | 1,0 | 1,1 | 1,1 | 2,4 | 2,0 | 0,9 | 0,3 |
| Sprr2a2 | 0,6 | 1,0 | 0,6 | 0,7 | 1,0 | 1,0 | 1,0 | 0,8 | 1,0 | 1,0 | 1,0 | 1,0 | 0,7 | 0,5 | 1,0 | 1,0 | 1,0 | 0,8 | 1,0 | 2,4 | 0,4 | 0,8 |
| Sprr2h | 1,0 | 1,0 | 0,3 | 1,0 | 1,0 | 1,0 | 1,0 | 1,0 | 1,0 | 1,0 | 1,0 | 1,0 | 1,0 | 1,0 | 1,0 | 1,0 | 1,0 | 12,8 | 1,0 | 1,0 | 1,0 | 1,0 |
| Sprr3 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,1 | 1,1 | 1,0 | 1,0 | 1,0 | 1,0 |
| Spry4 | 1,3 | 1,1 | 1,2 | 1,0 | 1,0 | 0,9 | 1,1 | 0,9 | 0,4 | 0,2 | 1,0 | 1,0 | 1,9 | 1,7 | 1,2 | 0,9 | 1,0 | 1,3 | 1,0 | 1,0 | 1,4 | 1,2 |
| Fads6 | 1,1 | 1,0 | 1,0 | 1,0 | 1,0 | 0,7 | 1,0 | 0,7 | 0,7 | 1,0 | 1,3 | 0,9 | 1,3 | 67,2 | 1,0 | 81,4 | 1,0 | 1,3 | 1,0 | 1,0 | 1,0 | 1,0 |
| Sptb | 1,0 | 1,0 | 1,0 | 1,0 | 1,0 | 0,8 | 1,0 | 1,0 | 1,0 | 1,0 | 1,1 | 1,6 | 2,0 | 2,4 | 1,4 | 0,9 | 1,0 | 1,0 | 1,0 | 1,0 | 1,0 | 1,0 |
| Spz1 | 1,0 | 1,0 | 1,0 | 1,0 | 1,0 | 1,0 | 1,0 | 1,0 | 1,0 | 1,0 | 1,0 | 1,0 | 1,0 | 1,0 | 9,5 | 2,2 | 1,0 | 1,0 | 1,0 | 1,0 | 1,0 | 1,0 |
| Srd5a2 | 0,2 | 0,1 | 0,8 | 0,4 | 0,8 | 0,7 | 1,0 | 1,0 | 1,0 | 1,0 | 1,0 | 1,0 | 1,0 | 1,0 | 6,1 | 1,5 | 1,0 | 1,0 | 1,0 | 1,0 | 1,0 | 1,0 |
| Srl | 1,3 | 1,1 | 1,1 | 1,3 | 0,7 | 0,7 | 0,9 | 0,8 | 1,0 | 1,0 | 1,0 | 1,0 | 0,6 | 1,0 | 1,1 | 0,8 | 0,7 | 1,2 | 1,1 | 0,9 | 1,1 | 1,5 |
| St14 | 1,1 | 1,0 | 1,1 | 1,1 | 1,1 | 0,9 | 1,0 | 1,0 | 1,0 | 1,0 | 1,0 | 0,8 | 1,8 | 1,3 | 12,1 | 1,2 | 1,1 | 1,0 | 1,2 | 1,0 | 1,2 | 1,0 |
| St6gal1 | 1,3 | 1,6 | 0,9 | 0,9 | 1,0 | 1,0 | 1,1 | 1,0 | 0,9 | 1,0 | 0,7 | 1,5 | 1,1 | 0,9 | 10,5 | 0,7 | 1,0 | 0,9 | 1,0 | 1,0 | 1,0 | 1,3 |
| St6galnac2 | 1,2 | 1,2 | 1,0 | 1,0 | 1,1 | 1,2 | 0,7 | 0,9 | 1,0 | 1,0 | 0,4 | 0,2 | 1,6 | 1,3 | 2,6 | 1,5 | 0,9 | 0,9 | 0,9 | 1,2 | 0,7 | 0,8 |
| *St8sia2* | *1,0* | *1,0* | *1,0* | *1,0* | *1,0* | *0,9* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* |
| Stac3 | 1,0 | 1,0 | 1,0 | 1,0 | 0,9 | 0,5 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Stc2 | 0,9 | 1,8 | 0,7 | 0,7 | 0,7 | 1,2 | 0,9 | 1,1 | 1,0 | 1,0 | 1,0 | 1,4 | 1,9 | 2,5 | 1,0 | 1,2 | 1,0 | 1,2 | 1,1 | 1,1 | 0,9 | 1,0 |
| Stfa1 | 1,0 | 1,0 | 1,0 | 1,0 | 0,8 | 1,3 | 1,0 | 1,0 | 1,0 | 1,0 | 1,0 | 1,0 | 1,0 | 1,0 | 1,0 | 1,0 | 1,0 | 4,4 | 1,0 | 1,0 | 1,0 | 1,0 |
| Stfa2l1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Stfa3 | 1,0 | 1,0 | 1,0 | 1,0 | 0,8 | 1,9 | 1,0 | 1,0 | 1,0 | 1,0 | 1,0 | 1,0 | 1,0 | 1,0 | 1,0 | 1,0 | 0,5 | 3,5 | 1,0 | 1,0 | 1,0 | 1,0 |
| Sult5a1 | 1,0 | 0,6 | 1,0 | 1,0 | 1,1 | 1,0 | 1,0 | 0,9 | 0,3 | 0,2 | 1,3 | 0,6 | 1,7 | 0,8 | 1,3 | 0,5 | 1,0 | 1,0 | 1,0 | 1,0 | 1,0 | 1,0 |
| Susd4 | 1,1 | 0,9 | 1,0 | 1,0 | 0,9 | 0,8 | 1,0 | 1,0 | 0,3 | 0,1 | 1,0 | 1,0 | 1,9 | 1,9 | 4,9 | 2,5 | 1,0 | 1,0 | 1,0 | 1,0 | 1,0 | 1,0 |
| Sycn | 0,6 | 0,8 | 1,6 | 1,0 | 1,0 | 1,0 | 1,0 | 1,0 | 0,5 | 0,7 | 1,0 | 1,0 | 1,0 | 0,2 | 1,0 | 1,0 | 36,2 | 0,1 | 0,0 | 0,7 | 1,8 | 0,5 |
| Syn2 | 0,7 | 0,8 | 1,0 | 1,3 | 1,0 | 1,1 | 1,2 | 0,9 | 1,0 | 1,0 | 1,2 | 0,9 | 1,0 | 1,0 | 1,5 | 3,0 | 0,8 | 1,0 | 0,9 | 0,8 | 0,9 | 1,0 |
| Synm | 1,0 | 1,0 | 1,1 | 1,0 | 0,9 | 0,8 | 0,9 | 0,9 | 1,0 | 1,0 | 0,7 | 0,8 | 1,3 | 1,1 | 0,9 | 1,4 | 1,3 | 1,1 | 1,2 | 1,0 | 1,0 | 1,3 |
| Synpo2 | 1,1 | 0,8 | 0,9 | 0,9 | 0,9 | 0,6 | 1,0 | 1,0 | 1,0 | 1,0 | 0,6 | 0,9 | 1,5 | 1,2 | 0,4 | 0,6 | 1,1 | 1,0 | 1,2 | 0,7 | 1,0 | 1,5 |
| Synpo2l | 1,1 | 0,9 | 1,0 | 1,0 | 0,7 | 0,6 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Sypl2 | 0,6 | 0,7 | 1,0 | 1,0 | 0,5 | 0,7 | 1,0 | 1,0 | 1,0 | 1,0 | 1,0 | 1,1 | 1,0 | 1,0 | 1,2 | 1,0 | 1,0 | 1,0 | 0,5 | 0,7 | 0,5 | 0,9 |
| Syt16 | 1,0 | 1,0 | 1,0 | 1,0 | 1,2 | 1,3 | 1,0 | 1,0 | 1,0 | 1,0 | 0,3 | 0,3 | 1,0 | 1,0 | 1,0 | 1,0 | 1,0 | 0,9 | 1,0 | 1,0 | 1,0 | 1,0 |
| Sytl4 | 1,0 | 1,1 | 0,9 | 0,9 | 1,0 | 1,0 | 1,0 | 1,0 | 1,0 | 1,0 | 1,1 | 1,0 | 1,0 | 1,0 | 9,8 | 1,0 | 0,9 | 0,8 | 0,7 | 0,8 | 0,8 | 0,7 |
| Tacstd2 | 1,8 | 2,1 | 1,0 | 1,0 | 1,0 | 0,9 | 1,0 | 1,0 | 1,0 | 1,0 | 1,0 | 0,8 | 1,7 | 0,9 | 7,7 | 1,0 | 0,9 | 1,0 | 1,0 | 1,0 | 1,0 | 1,0 |
| Tbc1d8 | 1,4 | 1,4 | 1,0 | 1,1 | 1,0 | 1,0 | 1,4 | 0,9 | 1,1 | 1,2 | 1,0 | 1,0 | 1,3 | 1,1 | 6,6 | 0,8 | 0,9 | 0,9 | 0,9 | 0,9 | 1,0 | 1,0 |
| Tbx1 | 1,0 | 1,0 | 1,0 | 1,0 | 0,6 | 0,3 | 1,0 | 1,0 | 1,0 | 1,0 | 1,2 | 1,2 | 1,0 | 1,0 | 1,2 | 1,2 | 1,0 | 1,0 | 1,0 | 1,0 | 1,0 | 1,0 |
| Tbx15 | 1,0 | 1,0 | 1,0 | 1,0 | 0,7 | 0,7 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Tcap | 1,0 | 1,0 | 0,9 | 1,2 | 0,8 | 0,7 | 0,9 | 1,4 | 1,0 | 1,0 | 2,2 | 1,4 | 0,7 | 1,8 | 0,7 | 1,7 | 0,2 | 1,0 | 1,0 | 1,0 | 0,8 | 0,7 |
| Tcea3 | 0,7 | 0,7 | 0,9 | 0,9 | 0,8 | 0,9 | 1,1 | 1,2 | 0,9 | 1,1 | 1,1 | 1,0 | 1,0 | 1,0 | 2,3 | 1,1 | 1,1 | 1,1 | 0,7 | 1,0 | 0,9 | 0,8 |
| Tceal3 | 0,9 | 1,8 | 0,8 | 0,8 | 1,0 | 1,1 | 1,0 | 0,7 | 1,0 | 1,0 | 0,8 | 1,1 | 1,1 | 1,1 | 5,3 | 1,9 | 0,6 | 0,8 | 0,7 | 0,6 | 0,7 | 0,4 |
| *Tceal7* | *1,0* | *1,0* | *1,0* | *1,0* | 1,3 | 0,7 | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* | *1,0* |
| Tcf7 | 1,1 | 1,9 | 0,5 | 0,5 | 0,9 | 0,9 | 1,0 | 1,2 | 1,3 | 1,5 | 0,8 | 1,6 | 0,6 | 0,8 | 8,4 | 2,0 | 0,8 | 1,1 | 1,1 | 0,7 | 0,9 | 1,0 |
| Tcp11 | 1,0 | 1,0 | 1,0 | 1,0 | 1,0 | 1,0 | 1,0 | 1,0 | 1,0 | 1,0 | 1,0 | 1,0 | 1,1 | 0,8 | 7,4 | 1,4 | 1,0 | 1,0 | 1,0 | 1,0 | 1,0 | 1,0 |
| Teddm1 | 1,0 | 1,0 | 1,0 | 1,0 | 1,0 | 1,0 | 1,0 | 1,0 | 1,0 | 1,0 | 1,0 | 1,0 | 1,0 | 1,0 | 831,0 | 1,0 | 1,0 | 1,0 | 1,0 | 1,0 | 1,0 | 1,0 |
| Tekt1 | 1,0 | 1,0 | 1,0 | 1,0 | 1,0 | 1,0 | 1,0 | 1,0 | 1,0 | 1,0 | 1,0 | 1,0 | 1,0 | 1,0 | 0,8 | 2,2 | 1,0 | 1,0 | 1,0 | 1,0 | 1,0 | 1,0 |
| Tfap2a | 1,4 | 1,3 | 1,0 | 1,0 | 1,0 | 1,4 | 1,0 | 1,0 | 1,0 | 1,0 | 1,5 | 1,0 | 1,5 | 1,3 | 5,2 | 1,0 | 1,0 | 0,9 | 1,0 | 1,0 | 1,0 | 1,0 |
| Tfap2b | 1,3 | 1,3 | 1,0 | 1,0 | 1,0 | 1,2 | 1,0 | 1,0 | 1,0 | 1,0 | 1,2 | 1,6 | 1,0 | 1,0 | 10,5 | 1,0 | 1,0 | 1,0 | 1,0 | 1,0 | 1,0 | 1,0 |
| Tff1 | 1,0 | 1,0 | 1,0 | 1,0 | 1,0 | 0,7 | 1,0 | 0,7 | 1,0 | 1,0 | 1,0 | 1,1 | 1,5 | 5,2 | 1,0 | 3,7 | 1,0 | 0,9 | 0,4 | 1,0 | 1,9 | 1,0 |
| Tff2 | 1,0 | 1,0 | 1,2 | 5,1 | 1,0 | 1,9 | 1,0 | 1,0 | 1,0 | 1,0 | 1,0 | 1,0 | 1,0 | 1,0 | 1,0 | 1,0 | 1,8 | 1,2 | 0,1 | 1,0 | 1,5 | 1,0 |
| Tff3 | 1,0 | 1,0 | 1,0 | 0,9 | 1,0 | 1,0 | 1,0 | 1,0 | 0,4 | 0,2 | 1,0 | 1,0 | 1,4 | 0,5 | 1,0 | 0,7 | 3,5 | 0,8 | 1,2 | 1,5 | 1,0 | 0,8 |
| Tgif2lx2 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Tgm3 | 1,0 | 1,0 | 2,0 | 1,5 | 1,0 | 1,0 | 1,0 | 1,0 | 1,0 | 1,0 | 1,0 | 1,0 | 1,0 | 1,0 | 1,0 | 1,0 | 0,8 | 0,9 | 1,0 | 1,0 | 1,0 | 1,0 |
| | Kidney | | Colon | | Eyeball | | Heart | | Liver | | SG | | Thymus | | Adipose | | Stomach | | Jejunum | | Ileum | |
| | E | M | E | M | E | M | E | M | E | M | E | M | E | M | E | M | E | M | E | M | E | M |
| Tgoln2 | 9,3 | 0,8 | 0,5 | 1,0 | 1,0 | 1,0 | 0,9 | 0,1 | 13,7 | 0,4 | 0,3 | 10,8 | 1,0 | 0,8 | 1,0 | 1,0 | 0,0 | 0,6 | 0,5 | 0,2 | 1,6 | 17,1 |

[Fig. 23-29]

298/310

| | | | | | | | | | | | | | | | | | | | | | | |
|---|---|---|---|---|---|---|---|---|---|---|---|---|---|---|---|---|---|---|---|---|---|---|
| Thrsp | 1,3 | 1,4 | 0,8 | 1,6 | 0,7 | 0,8 | 1,4 | 1,1 | 0,6 | 0,5 | 1,4 | [illegible] | 1,9 | 3,3 | 1,1 | 1,8 | 0,6 | 0,6 | 1,0 | 1,3 | 0,9 | 1,6 |
| Tigd4 | 1,0 | 1,0 | 1,0 | 1,0 | 0,9 | 0,9 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Timp1 | 2,8 | 6,2 | 0,9 | 0,5 | 1,0 | 1,1 | 2,1 | 1,7 | 1,0 | 1,0 | 1,0 | 1,0 | 1,2 | 0,6 | 1,3 | 1,0 | 2,3 | 3,2 | 1,0 | 1,0 | 1,0 | 1,0 |
| Tmem102 | 0,9 | 0,9 | 1,2 | 1,1 | 0,8 | 0,9 | 1,0 | 1,0 | 1,0 | 0,9 | 0,9 | 0,8 | 1,2 | 1,6 | 5,0 | 1,0 | 1,0 | 1,0 | 1,2 | 1,3 | 1,0 | 1,1 |
| Tmem139 | 0,9 | 0,8 | 1,2 | 1,4 | 1,2 | 1,2 | 1,0 | 1,0 | 1,0 | 1,0 | 0,7 | 0,7 | 1,0 | 1,0 | 5,6 | 1,0 | 0,9 | 1,0 | 1,1 | 1,5 | 1,4 | 1,1 |
| Tmem150c | 1,3 | 0,9 | 1,0 | 1,0 | 1,1 | 1,1 | 0,8 | 0,5 | 1,0 | 1,0 | 1,0 | 1,1 | 1,4 | 1,3 | 27,8 | 1,9 | 1,0 | 1,0 | 1,0 | 1,0 | 1,0 | 1,0 |
| Tmem182 | 1,0 | 1,0 | 0,8 | 0,9 | 0,6 | 0,6 | 1,0 | 1,0 | 1,0 | 1,0 | 1,0 | 1,0 | 1,0 | 1,0 | 0,7 | 0,8 | 1,0 | 1,0 | 1,0 | 1,0 | 1,0 | 1,0 |
| Tmem212 | 1,0 | 1,0 | 1,0 | 1,0 | 1,0 | 1,0 | 1,0 | 1,0 | 1,0 | 1,0 | 1,0 | 1,0 | 1,0 | 1,0 | 0,8 | 6,7 | 1,0 | 1,0 | 1,0 | 1,0 | 1,0 | 1,0 |
| Tmem233 | 1,0 | 1,0 | 1,0 | 1,0 | 0,6 | 0,3 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Tmem252 | 3,9 | 5,1 | 1,2 | 0,9 | 1,2 | 1,3 | 1,6 | 0,9 | 1,0 | 1,0 | 1,0 | 1,0 | 1,7 | 2,2 | 1,1 | 2,1 | 1,6 | 1,7 | 1,5 | 0,8 | 1,1 | 0,7 |
| Tmem254c | 0,9 | 0,6 | 2,4 | 0,7 | 0,3 | 0,7 | 1,0 | 1,0 | 0,3 | 0,0 | 0,8 | 0,8 | 3,0 | 0,2 | 1,8 | 1,1 | 1,1 | 0,7 | 0,8 | 0,5 | 0,9 | 2,7 |
| Tmem30b | 1,2 | 1,2 | 1,1 | 1,3 | 0,9 | 1,1 | 1,0 | 1,0 | 1,0 | 1,1 | 1,0 | 1,0 | 2,2 | 2,0 | 7,3 | 2,3 | 1,0 | 1,0 | 1,0 | 1,0 | 1,0 | 1,1 |
| Tmem37 | 2,3 | 2,0 | 1,3 | 1,3 | 1,0 | 1,0 | 1,4 | 1,0 | 1,2 | 1,5 | 0,5 | 0,3 | 2,0 | 1,5 | 1,1 | 1,2 | 1,8 | 1,4 | 1,4 | 1,9 | 1,3 | 1,1 |
| Tmem38a | 1,1 | 1,1 | 1,1 | 1,1 | 0,8 | 0,8 | 1,0 | 1,1 | 1,1 | 0,9 | 1,3 | 1,3 | 0,9 | 0,9 | 1,1 | 1,3 | 0,9 | 0,9 | 0,9 | 1,1 | 0,8 | 1,1 |
| Tmem45b | 0,8 | 0,8 | 1,1 | 1,3 | 1,0 | 0,6 | 2,1 | 0,5 | 1,0 | 1,0 | 1,0 | 1,0 | 1,4 | 3,0 | 0,3 | 0,6 | 0,9 | 0,9 | 1,0 | 1,1 | 1,0 | 1,0 |
| Tmem51 | 1,0 | 0,9 | 1,0 | 1,0 | 1,0 | 1,0 | 1,1 | 0,9 | 1,6 | 2,1 | 0,7 | 0,8 | 1,6 | 1,4 | 7,6 | 1,8 | 1,0 | 1,1 | 1,2 | 1,2 | 1,2 | 0,9 |
| Tmem52 | 1,9 | 2,2 | 1,0 | 1,1 | 0,8 | 0,8 | 0,8 | 1,1 | 1,0 | 1,0 | 1,1 | 0,8 | 1,0 | 1,0 | 0,8 | 2,4 | 1,0 | 1,0 | 1,0 | 1,0 | 1,0 | 1,0 |
| Tmie | 1,3 | 0,8 | 1,0 | 1,3 | 1,1 | 0,9 | 1,6 | 1,0 | 1,3 | 0,5 | 1,6 | 1,3 | 1,2 | 0,7 | 1,4 | 1,5 | 0,9 | 1,1 | 0,8 | 0,5 | 1,0 | 1,0 |
| Tmod1 | 0,9 | 1,0 | 0,8 | 1,6 | 0,8 | 0,8 | 1,0 | 1,0 | 1,0 | 1,0 | 1,0 | 1,0 | 1,0 | 1,0 | 1,7 | 2,0 | 1,0 | 0,8 | 1,0 | 1,5 | 1,6 | 1,0 |
| Tmod4 | 1,0 | 1,0 | 1,0 | 1,0 | 0,7 | 0,6 | 1,0 | 0,8 | 1,0 | 1,0 | 1,0 | 1,0 | 1,0 | 1,0 | 1,0 | 1,0 | 1,0 | 1,0 | 1,0 | 1,0 | 1,0 | 1,0 |
| Tmprss15 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 10,2 | 2,7 | 1,0 | 1,0 |
| Tnfrsf13c | 1,0 | 1,0 | 0,6 | 0,2 | 1,0 | 1,0 | 1,0 | 1,0 | 1,0 | 1,0 | 1,0 | 1,8 | 1,6 | 0,9 | 1,0 | 1,0 | 1,0 | 1,0 | 1,5 | 0,8 | 0,7 | 0,9 |
| Tnmd | 1,0 | 1,0 | 1,0 | 1,0 | 0,6 | 0,9 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Tnnc2 | 1,0 | 1,0 | 1,0 | 1,0 | 0,7 | 0,6 | 1,3 | 0,6 | 1,0 | 1,0 | 1,0 | 1,8 | 1,0 | 1,4 | 0,6 | 1,2 | 0,3 | 1,1 | 1,0 | 1,3 | 1,0 | 1,0 |
| Tnni1 | 1,0 | 1,0 | 1,8 | 1,3 | 0,7 | 1,4 | 1,2 | 1,2 | 1,0 | 1,0 | 1,0 | 1,0 | 1,0 | 1,0 | 1,0 | 1,0 | 1,0 | 1,0 | 1,0 | 1,0 | 1,0 | 1,1 |
| Tnni2 | 1,0 | 1,0 | 1,1 | 0,8 | 0,7 | 0,8 | 0,9 | 1,5 | 1,0 | 1,0 | 1,9 | 2,2 | 0,6 | 0,6 | 1,0 | 1,1 | 0,9 | 0,7 | 1,0 | 0,7 | 1,0 | 0,8 |
| Tnnt3 | 1,0 | 1,0 | 1,0 | 1,0 | 0,7 | 0,7 | 1,0 | 0,6 | 1,0 | 1,0 | 1,0 | 2,6 | 0,6 | 1,5 | 1,0 | 1,0 | 1,2 | 1,2 | 1,0 | 1,0 | 1,0 | 1,0 |
| Tnp1 | 1,0 | 1,0 | 1,0 | 1,1 | 1,0 | 1,0 | 1,1 | 1,0 | 1,0 | 1,0 | 1,0 | 1,0 | 1,0 | 1,0 | 7,7 | 13,2 | 0,9 | 1,0 | 1,0 | 1,0 | 1,0 | 1,0 |
| Tnp2 | 1,0 | 1,0 | 1,0 | 0,5 | 1,0 | 1,0 | 1,0 | 1,0 | 1,0 | 1,0 | 1,0 | 1,0 | 1,2 | 1,1 | 15,6 | 11,9 | 1,0 | 1,0 | 1,0 | 2,1 | 1,1 | 0,6 |
| Tox3 | 0,8 | 0,7 | 0,9 | 1,0 | 1,2 | 1,1 | 1,5 | 1,1 | 1,0 | 1,0 | 1,0 | 1,0 | 1,1 | 0,9 | 11,1 | 1,0 | 1,2 | 1,0 | 1,0 | 0,7 | 0,8 | 1,2 |
| Tpd52l1 | 1,0 | 1,0 | 1,0 | 1,2 | 0,7 | 0,7 | 1,1 | 1,3 | 0,9 | 0,9 | 1,0 | 0,9 | 1,4 | 1,1 | 17,1 | 2,9 | 1,2 | 1,3 | 1,0 | 1,1 | 0,8 | 0,9 |
| Tpm2 | 1,0 | 0,8 | 1,1 | 0,9 | 0,7 | 0,7 | 1,0 | 1,0 | 1,0 | 1,4 | 0,8 | 0,7 | 1,5 | 1,1 | 1,0 | 2,4 | 1,4 | 1,0 | 1,3 | 1,1 | 1,0 | 0,9 |
| Tppp2 | 1,0 | 1,0 | 1,0 | 1,0 | 1,0 | 1,0 | 1,0 | 1,0 | 1,0 | 1,0 | 1,0 | 1,0 | 1,0 | 1,0 | 6,2 | 1,0 | 1,0 | 1,0 | 1,0 | 1,0 | 1,0 | 1,0 |
| Tpx2 | 1,0 | 1,0 | 0,9 | 0,8 | 1,0 | 1,0 | 1,0 | 1,0 | 1,0 | 1,0 | 1,0 | 1,0 | 0,5 | 0,7 | 1,0 | 1,0 | 1,1 | 0,9 | 1,4 | 0,9 | 1,0 | 0,9 |
| Trank1 | 1,0 | 1,0 | 1,0 | 1,0 | 1,1 | 0,8 | 1,0 | 1,0 | 1,0 | 1,0 | 1,0 | 1,0 | 1,0 | 1,0 | 7,4 | 1,0 | 1,0 | 1,0 | 1,0 | 1,0 | 1,0 | 1,0 |
| Trdn | 1,0 | 1,0 | 1,0 | 1,0 | 0,9 | 0,7 | 0,9 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Trem2 | 1,0 | 2,4 | 2,1 | 0,5 | 0,9 | 0,9 | 1,3 | 1,2 | 1,0 | 1,0 | 1,5 | 0,7 | 1,7 | 1,9 | 0,8 | 0,4 | 2,1 | 7,4 | 1,0 | 1,0 | 1,0 | 1,0 |
| Trim54 | 1,0 | 1,0 | 1,0 | 1,0 | 0,8 | 0,6 | 1,1 | 1,5 | 1,0 | 1,0 | 1,0 | 1,0 | 1,0 | 1,0 | 1,0 | 1,0 | 1,0 | 1,0 | 1,0 | 1,0 | 1,0 | 1,0 |
| Trim56 | 1,6 | 0,7 | 0,8 | 1,0 | 1,2 | 1,0 | 1,0 | 0,7 | 3,0 | 0,6 | 0,8 | 2,3 | 0,5 | 0,8 | 0,6 | 0,2 | 0,5 | 1,1 | 0,8 | 0,4 | 1,0 | 2,5 |
| Trim63 | 0,7 | 1,4 | 1,0 | 1,0 | 0,8 | 0,8 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Trim72 | 1,0 | 1,0 | 0,9 | 1,1 | 0,6 | 0,7 | 0,9 | 0,9 | 1,0 | 1,0 | 1,4 | 1,4 | 1,0 | 1,0 | 1,0 | 1,0 | 0,9 | 1,0 | 1,3 | 0,7 | 1,3 | 1,2 |
| Trnp1 | 1,0 | 1,1 | 1,1 | 1,3 | 1,0 | 1,0 | 1,2 | 1,9 | 1,0 | 1,0 | 1,0 | 0,8 | 3,0 | 2,7 | 1,7 | 1,1 | 1,1 | 1,2 | 1,5 | 1,7 | 1,2 | 1,3 |
| Trp53inp2 | 0,9 | 1,0 | 0,9 | 1,0 | 0,9 | 0,9 | 0,9 | 1,0 | 0,9 | 0,8 | 0,9 | 1,0 | 0,9 | 0,9 | 0,3 | 0,3 | 1,2 | 1,2 | 1,0 | 0,9 | 0,9 | 1,1 |
| Trpv6 | 1,0 | 1,2 | 4,2 | 4,4 | 1,0 | 1,0 | 1,0 | 1,0 | 1,0 | 1,0 | 0,8 | 0,6 | 0,6 | 0,7 | 7,8 | 1,0 | 1,0 | 1,0 | 1,0 | 1,0 | 1,0 | 1,0 |
| Try4 | 0,9 | 1,6 | 3,4 | 279,5 | 1,0 | 1,0 | 1,0 | 1,0 | 0,8 | 0,7 | 0,8 | 1,0 | 1,0 | 0,2 | 1,0 | 1,0 | 13,6 | 0,1 | 0,0 | 0,5 | 1,7 | 1,0 |
| Try5 | 1,3 | 1,2 | 3,9 | 278,6 | 1,0 | 1,0 | 1,0 | 1,0 | 0,9 | 0,8 | 0,6 | 1,0 | 1,0 | 0,2 | 1,0 | 1,0 | 28,5 | 0,1 | 0,0 | 0,9 | 1,5 | 1,0 |
| Tsc22d1 | 2,4 | 3,0 | 1,2 | 1,1 | 1,0 | 1,0 | 1,0 | 1,1 | 0,3 | 0,2 | 0,8 | 0,8 | 1,7 | 1,5 | 0,8 | 0,9 | 0,9 | 0,9 | 0,7 | 0,8 | 0,6 | 0,7 |
| Tspan1 | 1,2 | 1,1 | 1,2 | 1,0 | 0,7 | 0,9 | 1,0 | 1,0 | 1,0 | 1,0 | 0,8 | 0,7 | 2,4 | 1,0 | 5,3 | 1,0 | 1,1 | 1,1 | 1,8 | 1,5 | 1,4 | 1,0 |
| Tssk6 | 1,8 | 0,7 | 0,9 | 1,4 | 0,9 | 0,8 | 1,0 | 1,0 | 1,0 | 1,0 | 1,0 | 1,0 | 0,8 | 1,0 | 7,3 | 2,2 | 1,0 | 1,0 | 1,1 | 1,0 | 1,5 | 1,3 |
| Ttc36 | 0,6 | 0,6 | 0,9 | 1,5 | 0,9 | 1,1 | 1,0 | 1,0 | 0,8 | 1,0 | 1,5 | 0,8 | 2,3 | 1,2 | 1,3 | 6,1 | 3,9 | 0,5 | 1,4 | 0,7 | 1,7 | 0,6 |
| Ttc39c | 0,6 | 0,5 | 1,1 | 1,1 | 1,0 | 1,1 | 0,8 | 1,0 | 0,3 | 0,2 | 0,9 | 0,7 | 1,6 | 1,5 | 0,9 | 1,2 | 1,1 | 0,9 | 1,0 | 1,0 | 1,1 | 0,8 |
| Ttn | 1,0 | 1,0 | 1,0 | 1,0 | 0,9 | 0,6 | 0,8 | 0,8 | 1,0 | 1,0 | 1,0 | 1,0 | 1,0 | 1,0 | 1,0 | 1,0 | 1,0 | 1,0 | 1,0 | 1,0 | 1,0 | 1,0 |
| Ttr | 2,3 | 1,4 | 0,7 | 1,0 | 1,0 | 1,0 | 1,0 | 1,0 | 0,7 | 0,8 | 3,0 | 2,2 | 1,0 | 1,0 | 0,4 | 0,9 | 1,3 | 0,8 | 1,7 | 1,4 | 1,2 | 0,9 |
| Tuba1a | 1,0 | 1,2 | 0,9 | 0,9 | 1,0 | 1,0 | 0,9 | 0,8 | 1,1 | 1,3 | 0,7 | 0,6 | 0,8 | 0,7 | 0,4 | 0,2 | 1,2 | 1,0 | 1,2 | 1,2 | 0,9 | 0,8 |
| Tuba8 | 1,0 | 0,9 | 1,0 | 1,0 | 0,7 | 0,9 | 1,4 | 1,0 | 5,0 | 8,1 | 1,2 | 2,3 | 1,1 | 1,7 | 2,8 | 2,0 | 1,0 | 1,0 | 1,0 | 1,0 | 1,0 | 1,0 |
| Tubb2a-ps2 | 2,3 | 1,8 | 0,7 | 1,5 | 1,0 | 1,0 | 0,7 | 1,0 | 2,4 | 0,8 | 0,4 | 0,5 | 0,6 | 0,9 | 1,5 | 0,5 | 1,0 | 1,3 | 1,2 | 0,9 | 1,6 | 2,6 |
| Txlnb | 1,0 | 1,0 | 1,0 | 1,0 | 0,7 | 0,7 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Tyr | 1,0 | 1,0 | 1,0 | 1,0 | 0,9 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Tyrp1 | 1,0 | 1,0 | 1,0 | 1,0 | 1,0 | 1,1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Ucp1 | 1,0 | 1,0 | 1,0 | 1,0 | 1,0 | 1,0 | 8,0 | 0,6 | 1,0 | 1,0 | 1,0 | 1,2 | 1,6 | 2,0 | 1,0 | 0,4 | 1,0 | 1,0 | 1,0 | 1,0 | 1,0 | 1,0 |
| Ugt1a1 | 37,4 | 31,0 | 1,2 | 2,0 | 1,0 | 1,0 | 1,0 | 1,0 | 1,2 | 1,7 | 1,0 | 1,0 | 1,0 | 1,0 | 1,0 | 1,0 | 0,9 | 0,7 | 0,7 | 0,8 | 1,0 | 1,2 |
| Ugt1a10 | 18,7 | 55,9 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Ugt1a2 | 114,4 | 119,1 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Uox | 1,0 | 1,0 | 0,5 | 0,8 | 1,0 | 1,0 | 1,0 | 1,0 | 0,9 | 0,9 | 1,8 | 1,4 | 1,0 | 1,0 | 1,0 | 1,0 | 6,4 | 0,2 | 1,0 | 1,0 | 1,0 | 1,0 |
| Upp2 | 0,7 | 1,1 | 1,0 | 1,0 | 1,6 | 0,9 | 1,0 | 1,0 | 0,3 | 0,5 | 0,5 | 0,3 | 1,0 | 1,0 | 1,0 | 1,0 | 1,0 | 0,8 | 1,0 | 1,0 | 1,0 | 1,0 |
| Usp13 | 1,0 | 1,0 | 1,0 | 1,0 | 1,0 | 0,6 | 1,0 | 0,9 | 1,0 | 1,0 | 1,0 | 1,0 | 1,0 | 1,0 | 1,0 | 1,0 | 1,0 | 1,0 | 1,0 | 1,0 | 1,0 | 1,0 |
| Vash2 | 1,0 | 1,0 | 1,0 | 1,0 | 1,2 | 0,8 | 1,0 | 1,0 | 1,0 | 1,0 | 1,0 | 1,0 | 1,5 | 1,5 | 5,2 | 1,9 | 1,0 | 1,0 | 1,0 | 1,0 | 1,0 | 1,0 |
| Vcan | 1,0 | 1,0 | 0,9 | 0,6 | 1,0 | 1,1 | 0,9 | 0,7 | 1,0 | 1,0 | 0,8 | 1,0 | 1,0 | 1,0 | 0,5 | 0,5 | 1,0 | 1,2 | 1,0 | 1,0 | 1,0 | 1,0 |
| Vgll2 | 1,0 | 1,0 | 1,0 | 1,0 | 0,5 | 0,5 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Vnn1 | 1,1 | 1,3 | 2,3 | 3,8 | 1,0 | 1,5 | 1,0 | 1,0 | 0,4 | 0,5 | 1,0 | 1,0 | 1,0 | 1,0 | 0,3 | 1,6 | 1,3 | 0,7 | 0,8 | 1,6 | 0,9 | 0,9 |
| Vpreb3 | 1,0 | 1,0 | 0,5 | 0,2 | 1,7 | 1,0 | 1,0 | 1,0 | 1,0 | 0,6 | 1,3 | 1,1 | 2,3 | 0,7 | 1,0 | 1,0 | 1,0 | 1,0 | 1,7 | 1,7 | 0,8 | 1,0 |
| Wbscr25 | 1,0 | 1,0 | 1,0 | 1,0 | 1,0 | 1,0 | 1,0 | 1,0 | 1,0 | 1,0 | 1,0 | 1,0 | 1,0 | 1,0 | 11,1 | 1,0 | 1,0 | 1,0 | 1,0 | 1,0 | 1,0 | 1,0 |
| Wfdc10 | 1,0 | 1,0 | 1,0 | 1,0 | 0,6 | 1,4 | 1,0 | 1,0 | 1,0 | 1,0 | 1,0 | 1,0 | 1,0 | 1,0 | 19,9 | 1,6 | 1,0 | 1,0 | 1,0 | 1,0 | 1,0 | 1,0 |
| Wfdc11 | 1,0 | 1,0 | 1,0 | 1,0 | 1,0 | 1,0 | 1,0 | 1,0 | 1,0 | 1,0 | 1,0 | 1,0 | 1,0 | 1,0 | 5,4 | 1,0 | 1,0 | 1,0 | 1,0 | 1,0 | 1,0 | 1,0 |
| Wfdc13 | 1,0 | 1,0 | 1,0 | 1,0 | 1,0 | 1,0 | 1,0 | 1,0 | 1,0 | 1,0 | 1,0 | 1,0 | 1,0 | 1,0 | 7,0 | 1,0 | 1,0 | 1,0 | 1,0 | 1,0 | 1,0 | 1,0 |
| Wfdc15b | 1,0 | 1,0 | 1,0 | 1,0 | 1,0 | 1,0 | 1,0 | 1,0 | 1,0 | 1,0 | 1,0 | 1,0 | 1,0 | 1,0 | 5,0 | 6,3 | 1,4 | 1,0 | 1,0 | 1,0 | 1,0 | 1,0 |
| Wfdc18 | 1,0 | 1,0 | 1,7 | 0,6 | 1,0 | 0,8 | 1,0 | 1,0 | 1,0 | 1,0 | 0,6 | 0,8 | 1,6 | 0,8 | 1,0 | 1,0 | 1,0 | 1,0 | 1,0 | 1,0 | 1,0 | 1,0 |
| Wfdc6a | 1,0 | 1,0 | 1,0 | 1,0 | 1,0 | 1,0 | 1,0 | 1,0 | 1,0 | 1,0 | 1,0 | 1,0 | 1,0 | 1,0 | 12,4 | 1,0 | 1,0 | 1,0 | 1,0 | 1,0 | 1,0 | 1,0 |
| Wfdc6b | 1,0 | 1,0 | 1,0 | 1,0 | 1,0 | 1,0 | 1,0 | 1,0 | 1,0 | 1,0 | 1,0 | 1,0 | 1,0 | 1,0 | 21,2 | 1,0 | 1,0 | 1,0 | 1,0 | 1,0 | 1,0 | 1,0 |
| Wfdc8 | 1,0 | 1,0 | 1,0 | 1,0 | 1,0 | 1,0 | 1,0 | 1,0 | 1,0 | 1,0 | 1,0 | 1,0 | 1,0 | 1,0 | 20,7 | 1,0 | 1,0 | 1,0 | 1,0 | 1,0 | 1,0 | 1,0 |
| Wfdc9 | 1,0 | 1,0 | 1,0 | 1,0 | 1,0 | 1,0 | 1,0 | 1,0 | 1,0 | 1,0 | 1,0 | 1,0 | 1,0 | 1,0 | 29,4 | 1,0 | 1,0 | 1,0 | 1,0 | 1,0 | 1,0 | 1,0 |
| Xirp1 | 1,0 | 1,0 | 1,0 | 1,0 | 0,7 | 0,7 | 1,3 | 1,1 | 1,0 | 0,9 | 1,0 | 1,0 | 1,0 | 1,0 | 1,0 | 1,0 | 1,0 | 1,0 | 1,0 | 1,0 | 1,0 | 1,0 |
| Xirp2 | 1,0 | 1,0 | 1,0 | 1,0 | 0,8 | 0,6 | 0,9 | 0,8 | 1,0 | 1,0 | 1,0 | 1,0 | 1,0 | 1,0 | 1,0 | 1,0 | 1,0 | 1,0 | 1,0 | 1,0 | 1,0 | 1,0 |
| Xkrx | 1,0 | 1,0 | 0,8 | 0,7 | 1,1 | 1,2 | 0,7 | 0,9 | 1,0 | 1,0 | 1,0 | 1,0 | 0,8 | 1,0 | 7,8 | 1,0 | 0,9 | 0,9 | 1,0 | 1,0 | 1,0 | 1,0 |

[Fig. 23-30]

299/310

| | | | | | | | | | | | | | | | | | | | | | | |
|---|---|---|---|---|---|---|---|---|---|---|---|---|---|---|---|---|---|---|---|---|---|---|
| Yipf7 | 1,0 | 1,0 | 1,0 | 1,0 | 0,6 | 0,5 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 | 1,0 |
| Ypel4 | 1,0 | 1,0 | 1,2 | 1,5 | 1,1 | 0,7 | 1,1 | 1,0 | 1,0 | 1,0 | 1,0 | 1,0 | 1,0 | 1,0 | 1,0 | 0,8 | 1,0 | 1,0 | 1,0 | 1,0 | 1,0 | 1,0 |
| Zbed6 | 1,8 | 1,2 | 0,8 | 1,2 | 1,0 | 1,0 | 0,4 | 0,4 | 4,1 | 0,4 | 0,7 | 2,7 | 0,1 | 0,2 | 1,0 | 0,8 | 0,7 | 0,8 | 0,6 | 0,4 | 1,0 | 3,0 |
| Zfp369 | 1,5 | 0,7 | 0,8 | 1,1 | 1,0 | 1,0 | 0,7 | 0,6 | 2,7 | 1,0 | 0,8 | 1,3 | 0,1 | 0,4 | 1,0 | 1,0 | 0,7 | 1,0 | 0,7 | 0,4 | 0,9 | 1,3 |
| Zfp648 | 2,4 | 2,0 | 1,0 | 1,0 | 1,0 | 1,0 | 1,0 | 1,0 | 1,0 | 1,0 | 1,0 | 1,0 | 1,0 | 1,0 | 20,8 | 1,0 | 1,0 | 1,0 | 1,0 | 1,0 | 1,0 | 1,0 |
| Zfp750 | 0,9 | 0,7 | 0,9 | 0,9 | 1,0 | 1,1 | 1,0 | 1,0 | 1,5 | 0,9 | 0,8 | 0,9 | 2,8 | 1,6 | 6,1 | 1,0 | 0,8 | 1,1 | 1,0 | 1,0 | 1,0 | 1,0 |
| Zg16 | 0,7 | 0,6 | 1,2 | 1,2 | 1,0 | 1,0 | 1,0 | 1,0 | 0,9 | 1,1 | 1,7 | 1,0 | 0,9 | 0,3 | 1,0 | 0,3 | 1,4 | 0,2 | 1,1 | 1,6 | 1,1 | 1,0 |

Fig.24

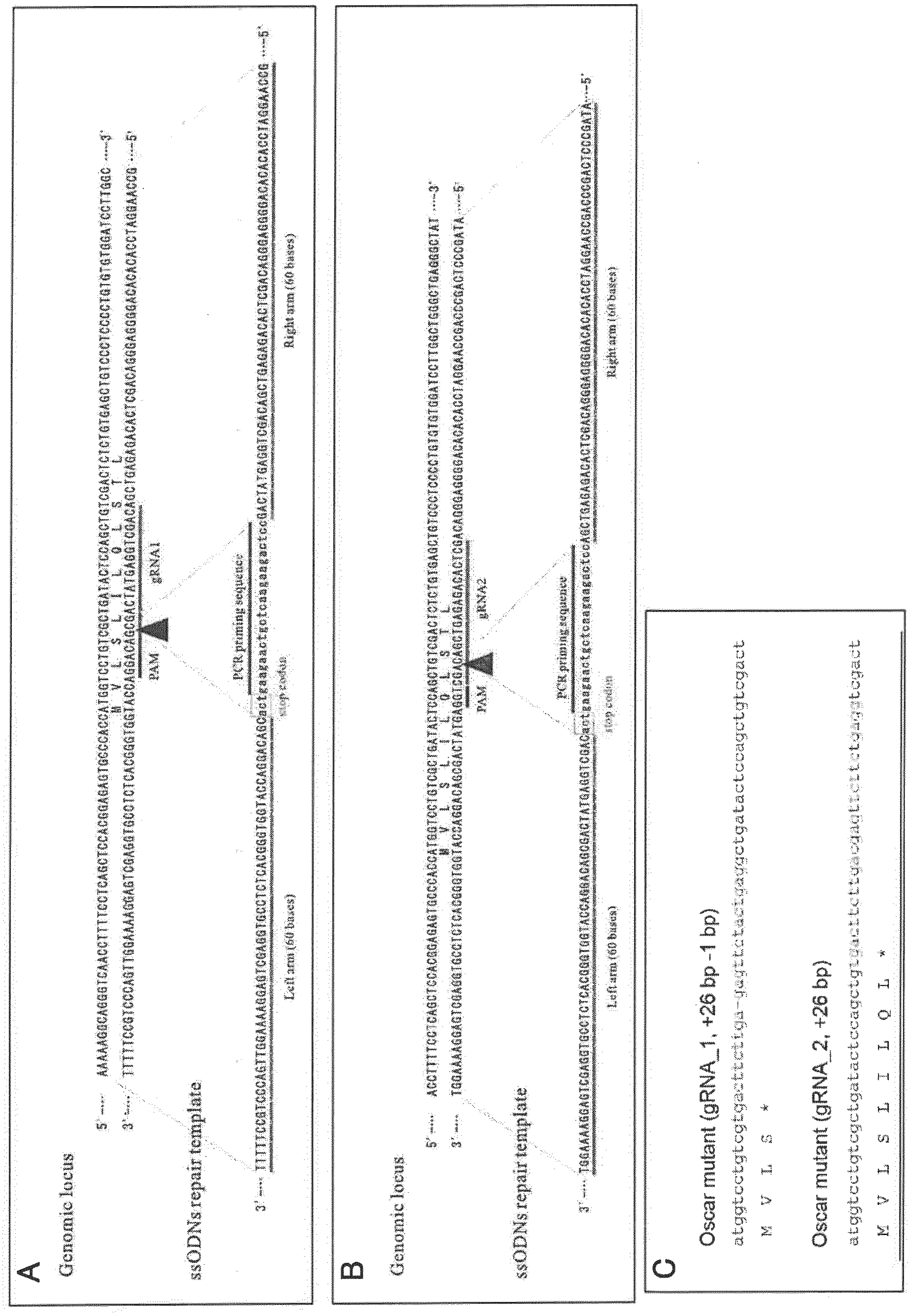
A
Genomic locus
ssODNs repair template
PAM
gRNA1
PCR priming sequence
stop codon
Left arm (60 bases)
Right arm (60 bases)
B
Genomic locus
ssODNs repair template
PAM
gRNA2
PCR priming sequence
stop codon
Left arm (60 bases)
Right arm (60 bases)
C
Oscar mutant (gRNA_1, +26 bp -1 bp)
Oscar mutant (gRNA_2, +26 bp)

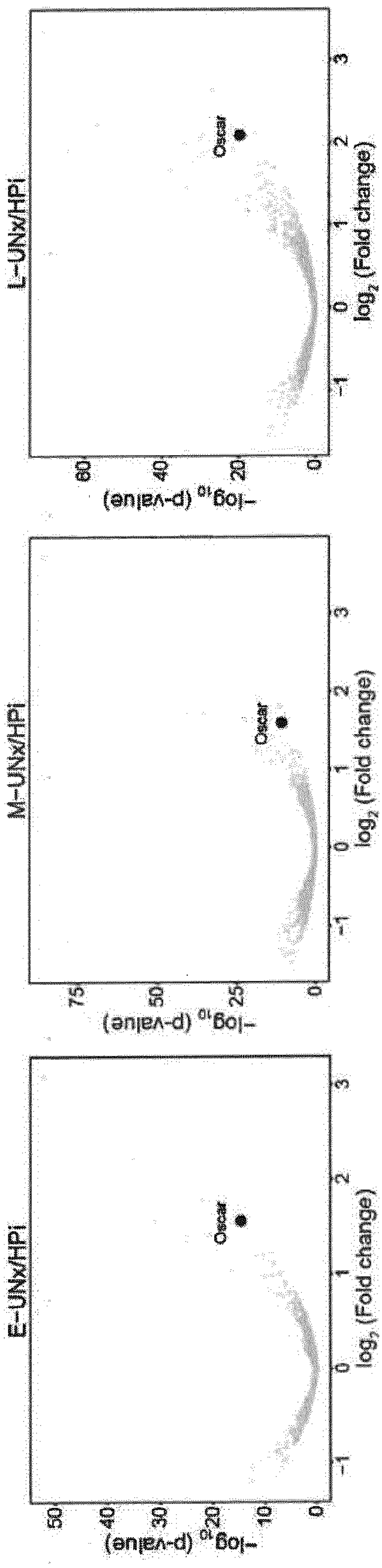
E–UNx/HPi
M–UNx/HPi
L–UNx/HPi
Oscar
Oscar
Oscar
-log10 (p-value)
log2 (Fold change)


Fig.25

Fig.26

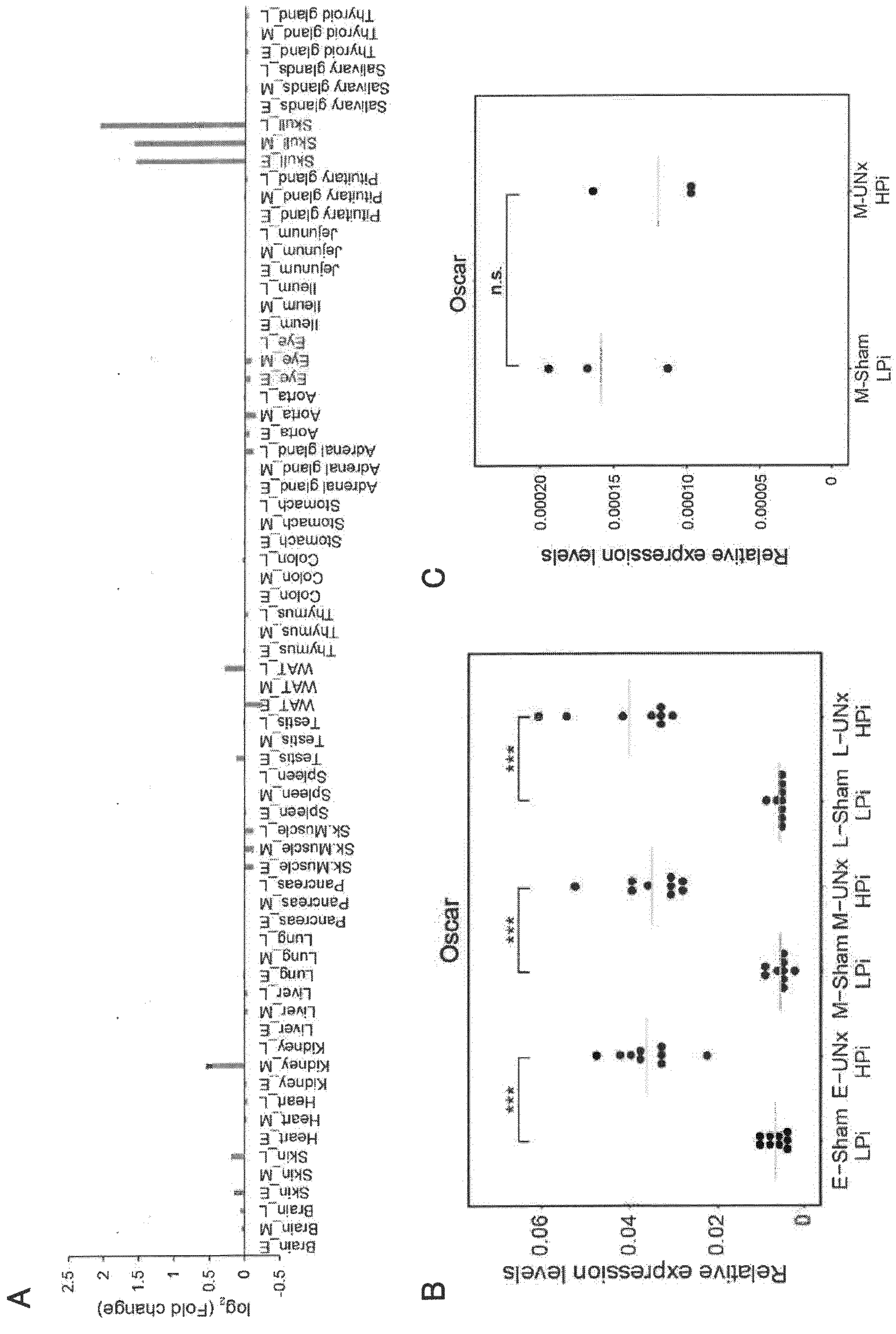
A
log2 (Fold change)
2.5
2
1.5
1
0.5
0
-0.5
Brain_E
Brain_M
Brain_L
Skin_E
Skin_M
Skin_L
Heart_E
Heart_M
Heart_L
Kidney_E
Kidney_M
Kidney_L
Liver_E
Liver_M
Liver_L
Lung_E
Lung_M
Lung_L
Pancreas_E
Pancreas_M
Pancreas_L
Sk.Muscle_E
Sk.Muscle_M
Sk.Muscle_L
Spleen_E
Spleen_M
Spleen_L
Testis_E
Testis_M
Testis_L
WAT_E
WAT_M
WAT_L
Thymus_E
Thymus_M
Thymus_L
Colon_E
Colon_M
Colon_L
Stomach_E
Stomach_M
Stomach_L
Adrenal gland_E
Adrenal gland_M
Adrenal gland_L
Aorta_E
Aorta_M
Aorta_L
Eye_E
Eye_M
Eye_L
Ileum_E
Ileum_M
Ileum_L
Jejunum_E
Jejunum_M
Jejunum_L
Pituitary gland_E
Pituitary gland_M
Pituitary gland_L
Skull_E
Skull_M
Skull_L
Salivary glands_E
Salivary glands_M
Salivary glands_L
Thyroid gland_E
Thyroid gland_M
Thyroid gland_L
B
Oscar
Relative expression levels
0.06
0.04
0.02
0
***
***
***
E−Sham LPi
E−UNx HPi
M−Sham LPi
M−UNx HPi
L−Sham LPi
L−UNx HPi
C
Oscar
Relative expression levels
0.00020
0.00015
0.00010
0.00005
0
n.s.
M-Sham LPi
M-UNx HPi

Fig.27

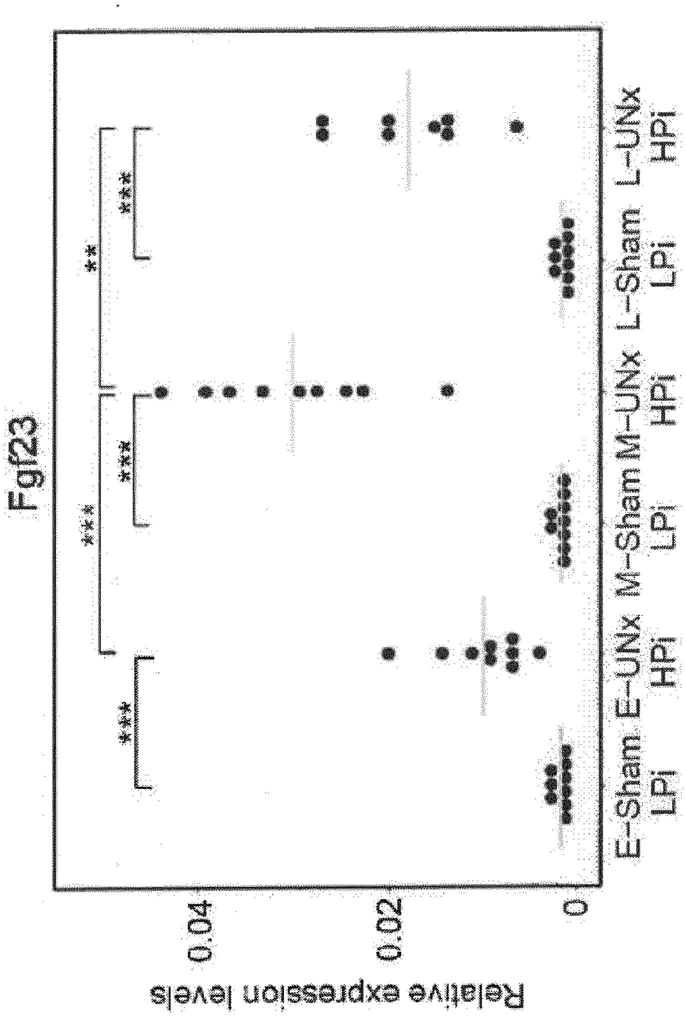
Fgf23
Relative expression levels
0.04
0.02
0
E–Sham LPi
E–UNx HPi
M–Sham LPi
M–UNx HPi
L–Sham LPi
L–UNx HPi
***
***
***
***
**
***

Fig.28

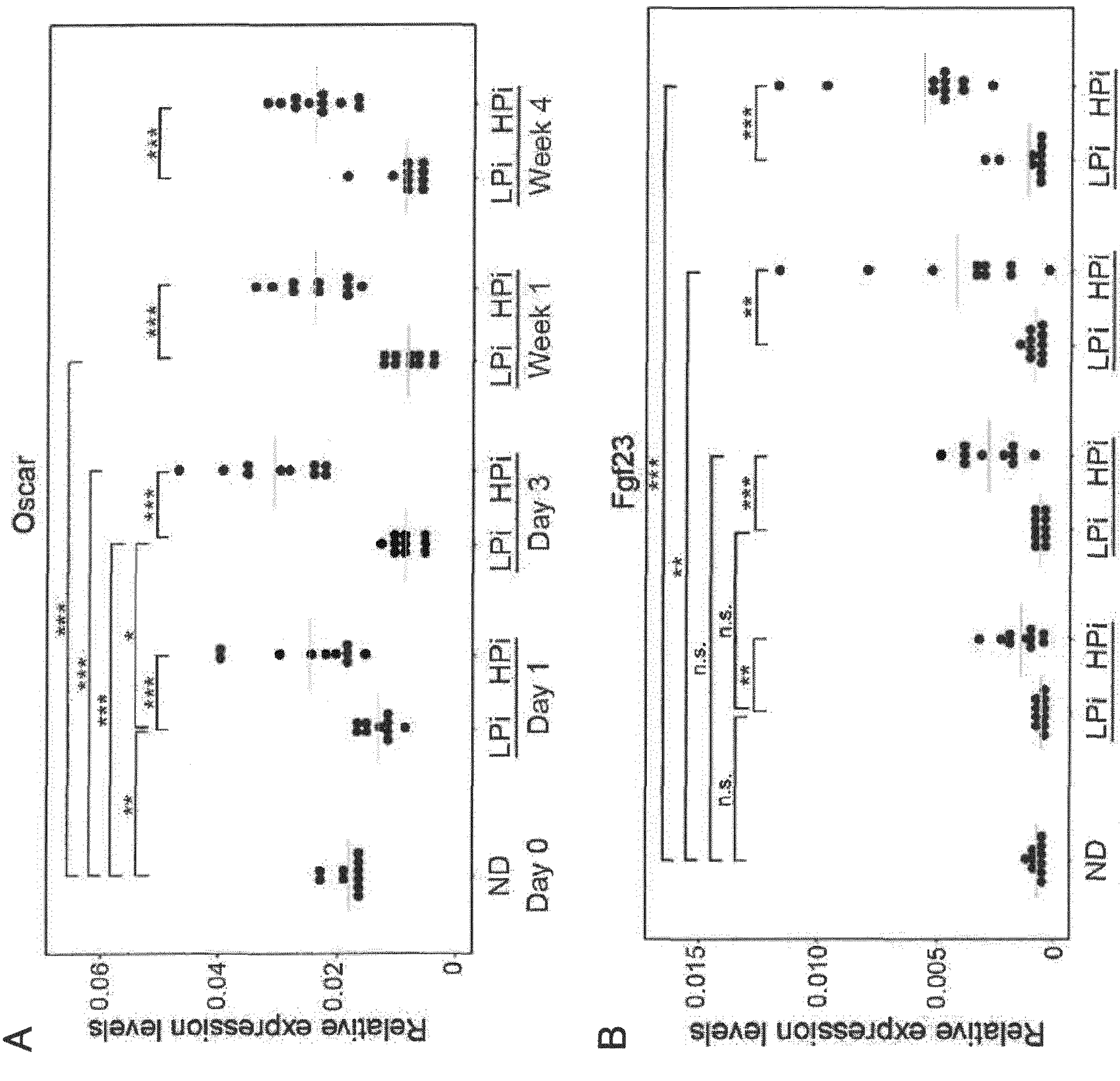
A
Oscar
Relative expression levels
0.06
0.04
0.02
0
ND Day 0
LPi HPi Day 1
LPi HPi Day 3
LPi HPi Week 1
LPi HPi Week 4
B
Fgf23
Relative expression levels
0.015
0.010
0.005
0
n.s.

Fig.29

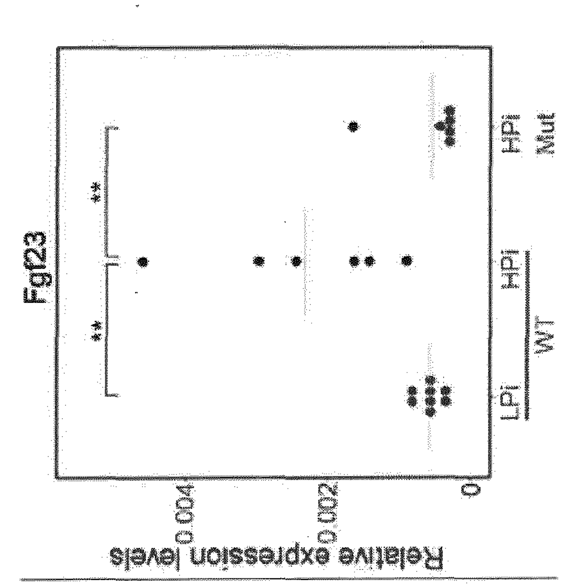
Fgf23
Relative expression levels
0.004
0.002
0
**
**
LPi
HPi
WT
HPi
Mut

Fig.30

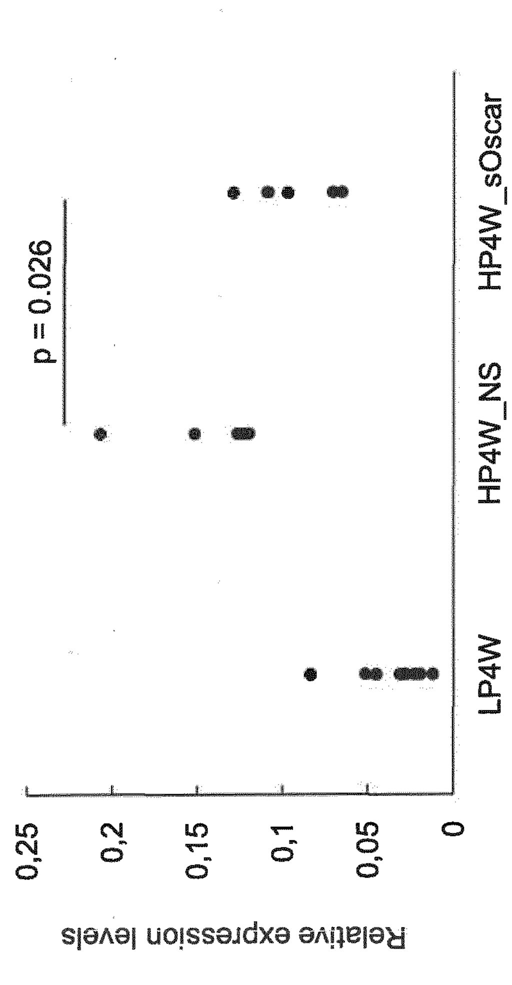
Relative expression levels
0,25
0,2
0,15
0,1
0,05
0
p = 0.026
LP4W
HP4W_NS
HP4W_sOscar

Fig.31

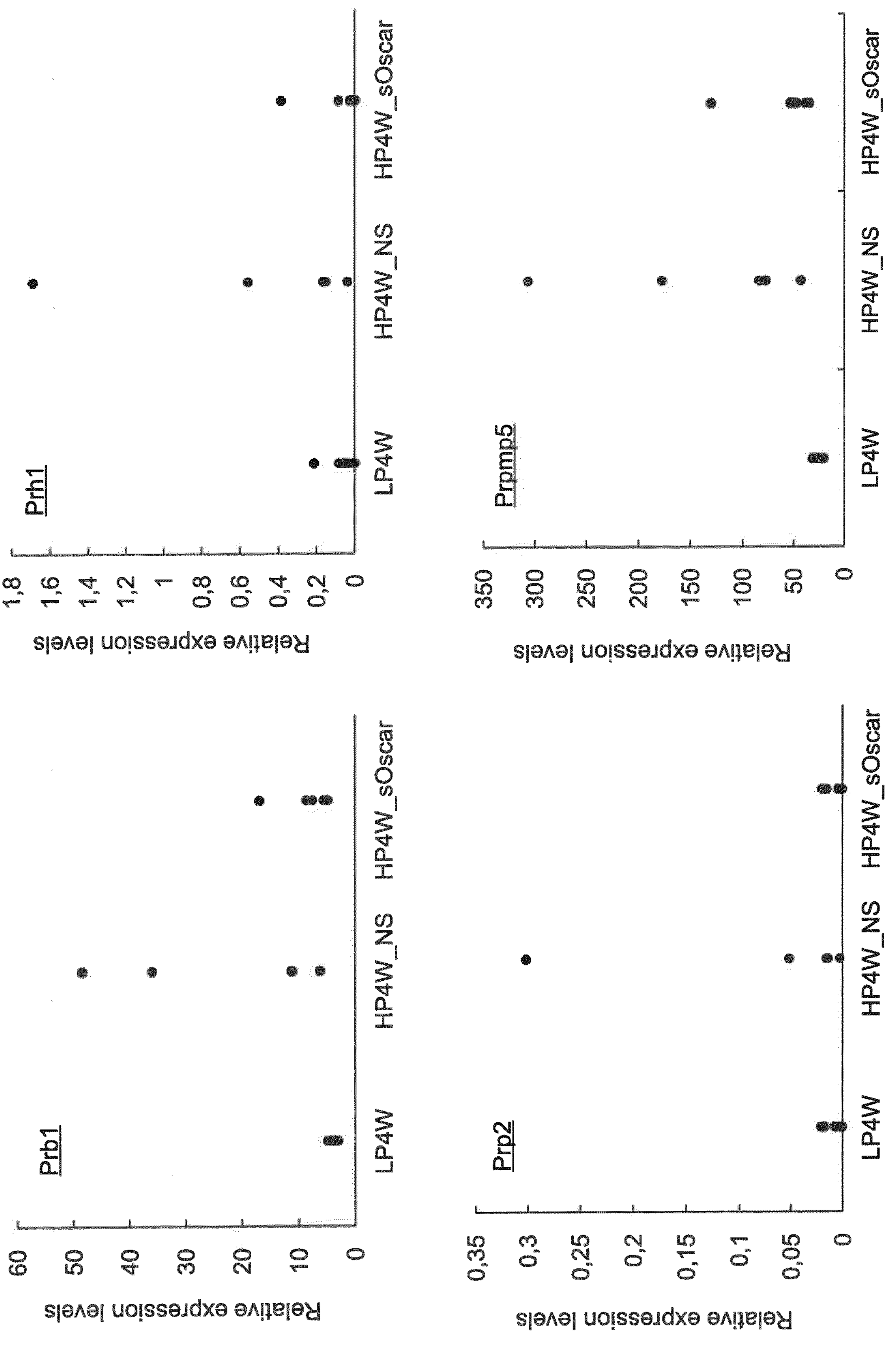
Prb1
Relative expression levels
60
50
40
30
20
10
0
LP4W
HP4W_NS
HP4W_sOscar
Prh1
Relative expression levels
1,8
1,6
1,4
1,2
1
0,8
0,6
0,4
0,2
0
LP4W
HP4W_NS
HP4W_sOscar
Prp2
Relative expression levels
0,35
0,3
0,25
0,2
0,15
0,1
0,05
0
LP4W
HP4W_NS
HP4W_sOscar
Prpmp5
Relative expression levels
350
300
250
200
150
100
50
0
LP4W
HP4W_NS
HP4W_sOscar

Fig.32

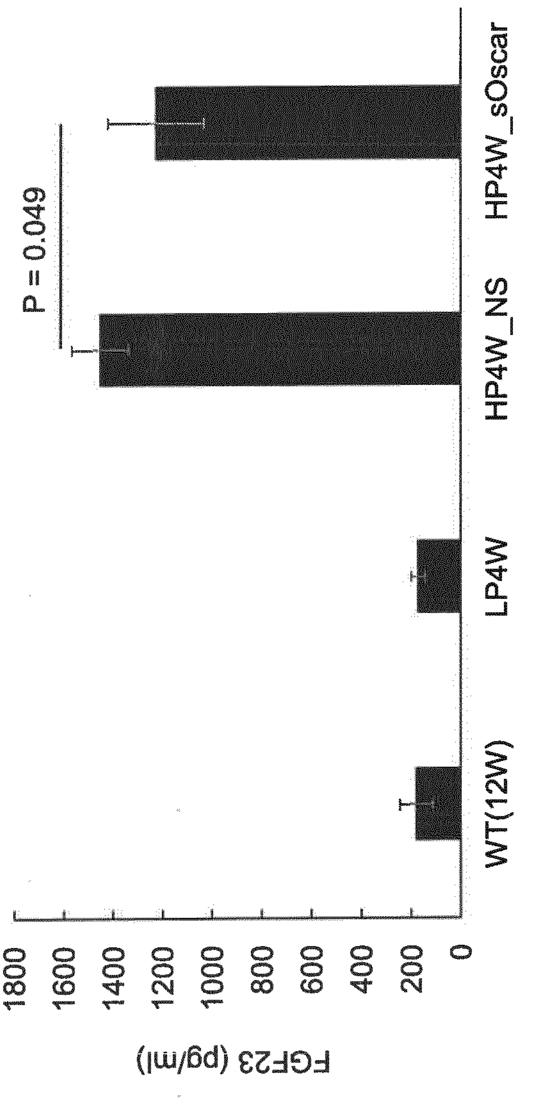
FGF23 (pg/ml)
1800
1600
1400
1200
1000
800
600
400
200
0
WT(12W)
LP4W
HP4W_NS
HP4W_sOscar
P = 0.049

Fig.33

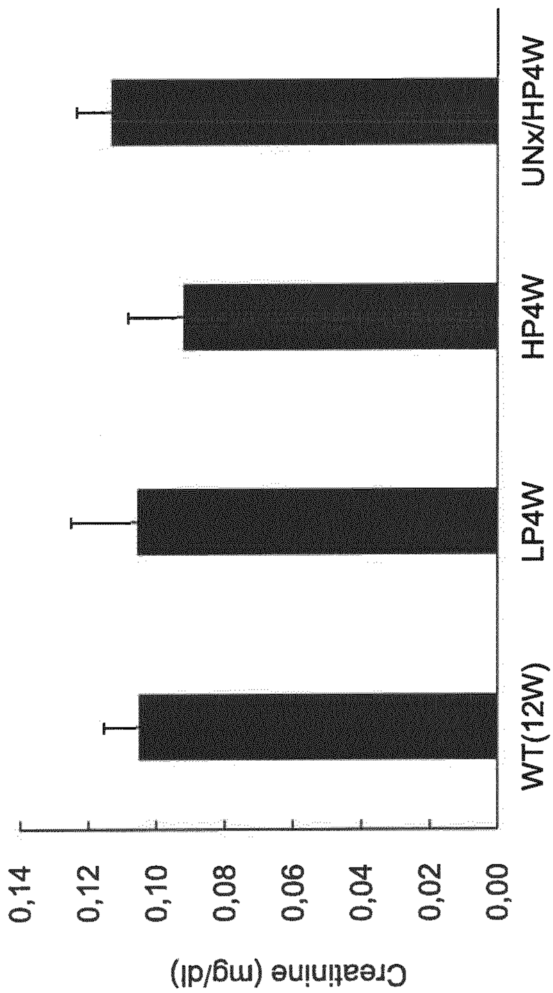
Creatinine (mg/dl)
0,14
0,12
0,10
0,08
0,06
0,04
0,02
0,00
WT(12W)
LP4W
HP4W
UNx/HP4W

Fig.34

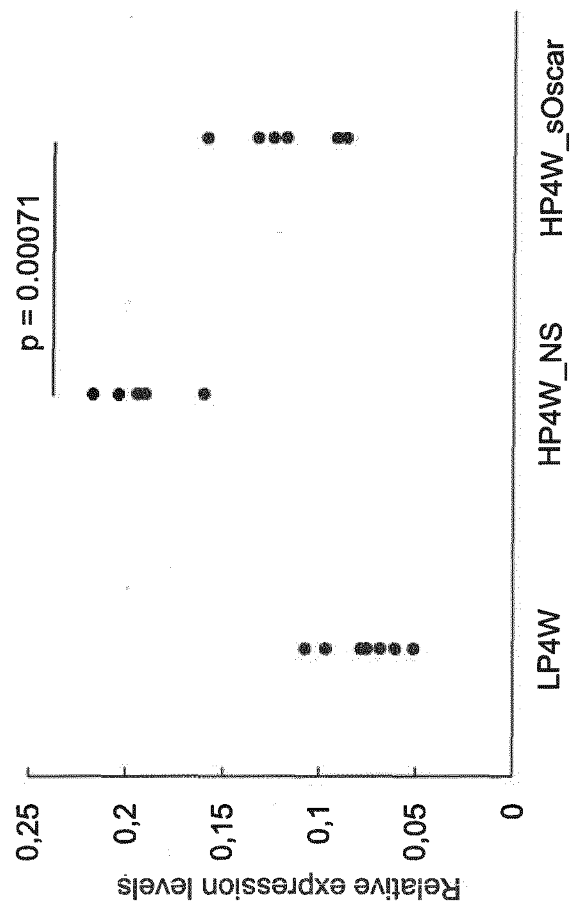
p = 0.00071
Relative expression levels
0,25
0,2
0,15
0,1
0,05
0
LP4W
HP4W_NS
HP4W_sOscar

## INTERNATIONAL SEARCH REPORT

International application No.
PCT/JP2017/012761

A. CLASSIFICATION OF SUBJECT MATTER
*C12Q1/68*(2006.01)i, *C12N15/09*(2006.01)i, *G01N33/15*(2006.01)i, *G01N33/50*(2006.01)i, *G01N33/53*(2006.01)i

According to International Patent Classification (IPC) or to both national classification and IPC

B. FIELDS SEARCHED

Minimum documentation searched (classification system followed by classification symbols)
C12Q1/68, C12N15/09, G01N33/15, G01N33/50, G01N33/53

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
JSTPlus/JMEDPlus/JST7580(JDreamIII), CAplus/MEDLINE/EMBASE/BIOSIS(STN), DWPI(Thomson Innovation)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | WO 2003/057874 A1 (Sumitomo Pharmaceuticals Co., Ltd.), 17 July 2003 (17.07.2003), claims; examples 1 to 4, 6 & AU 2002367266 A1 claims; examples 1 to 4, 6 | 1-3,7-13, 20-23<br>4-6,14-19<br>24-31 |
| X<br>Y<br>A | JP 2004-187620 A (Sumitomo Pharmaceuticals Co., Ltd.), 08 July 2004 (08.07.2004), claims; examples 1 to 4, 6 (Family: none) | 1-3,7-13, 20-23<br>4-6,14-19<br>24-31 |
| X<br>Y<br>A | JP 2001-037486 A (Tanabe Seiyaku Co., Ltd.), 13 February 2001 (13.02.2001), claims; examples 1 to 4 (Family: none) | 1-3,7-13, 20-23<br>4-6,14-19<br>24-31 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 21 June 2017 (21.06.17) | 04 July 2017 (04.07.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No.
PCT/JP2017/012761

C (Continuation). DOCUMENTS CONSIDERED TO BE RELEVANT

| Category* | Citation of document, with indication, where appropriate, of the relevant passages | Relevant to claim No. |
|---|---|---|
| X<br><br>Y<br>A | JP 2005-229834 A (Shionogi & Co., Ltd.), 02 September 2005 (02.09.2005), claims; paragraph [0021]; example 2 (Family: none) | 1-3,7-13, 20-23<br>4-6,14-19<br>24-31 |
| Y | JP 2011-050250 A (Chubu University), 17 March 2011 (17.03.2011), claims 5 to 7 (Family: none) | 14-19 |
| Y | JP 2005-323573 A (Sumitomo Pharmaceuticals Co., Ltd.), 24 November 2005 (24.11.2005), claim 24 (Family: none) | 4-6,17-19 |
| A | HUSAIN-SYED Faeq et al., "Cardio-Pulmonary-Renal Interactions", Journal of the American College of Cardiology, 2015, Vol.65, No.22, p.2433-2448, particularly, ABSTRACT, paragraph of "BIOMARKER" | 24-31 |
| A | WHITE Laura E. et al., "Inflammatory Mechanisms of Organ Crosstalk during Ischemic Acute Kidney Injury", International Journal of Nephrology, 2012, Article ID 505197, particularly, paragraph of "3.2 Kidney Crosstalk with Other Distant Organs" | 24-31 |
| A | ZOCCALI Carmine et al., "Chronic Kidney Disease (CKD) as a Systemic Disease: Whole Body Autoregulation and Inter-Organ Cross-Talk", Kidney & Blood Pressure Research, 2014, Vol.39,p.134-141, particularly, p.136, paragraph of "Inter-organ cross-talk" | 24-31 |
| A | WHITE Laura E. et al., "Surgical Sepsis and Organ Crosstalk: The Role of the Kidney", Journal of Surgical Research, 2011, Vol.167, p.306-315, particularly, p.312, left column, lines 6 to 17 | 24-31 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **LYSAGHT MJ.** *J Am Soc Nephrol.,* January 2002, vol. 13 (1), 37-40
- Clinical Practice Guidebook for Diagnosis and Treatment of Chronic Kidney Disease. 2012
- **HU M.C. et al.** *J Am Soc Nephrol,* 2011, vol. 22, 124-136
- **LOVE, M.I. ; HUBER, W. ; ANDERS, S.** *Genome biology,* 2014, vol. 15, 550

SEQUENCE LISTING

<110> ADVANCED TELECOMMUNICATIONS RESEARCH INSTITUTE INTERNATIONAL

<120> Pharmaceutical composition

<130> P16-201WO

<150>\201@JP 2016-066696
<151> 2016-03-29

<150> JP 2017-015821
<151> 2017-01-31

<160> 36

<170> PatentIn version 3.5

<210> 1
<211> 286
<212> PRT
<213> Homo sapiens

<400> 1

```
Met Ala Leu Val Leu Ile Leu Gln Leu Leu Thr Leu Trp Pro Leu Cys
1               5                   10                  15

His Thr Asp Ile Thr Pro Ser Val Ala Ile Ile Val Pro Pro Ala Ser
            20                  25                  30

Tyr His Pro Lys Pro Trp Leu Gly Ala Gln Pro Ala Thr Val Val Thr
        35                  40                  45

Pro Gly Val Asn Val Thr Leu Arg Cys Arg Ala Pro Gln Pro Ala Trp
    50                  55                  60

Arg Phe Gly Leu Phe Lys Pro Gly Glu Ile Ala Pro Leu Leu Phe Arg
65                  70                  75                  80

Asp Val Ser Ser Glu Leu Ala Glu Phe Phe Leu Glu Glu Val Thr Pro
                85                  90                  95

Ala Gln Gly Gly Ser Tyr Arg Cys Cys Tyr Arg Arg Pro Asp Trp Gly
            100                 105                 110

Pro Gly Val Trp Ser Gln Pro Ser Asp Val Leu Glu Leu Leu Val Thr
        115                 120                 125

Glu Glu Leu Pro Arg Pro Ser Leu Val Ala Leu Pro Gly Pro Val Val
    130                 135                 140

Gly Pro Gly Ala Asn Val Ser Leu Arg Cys Ala Gly Arg Leu Arg Asn
145                 150                 155                 160
```

Met Ser Phe Val Leu Tyr Arg Glu Gly Val Ala Ala Pro Leu Gln Tyr
                165                 170                 175

Arg His Ser Ala Gln Pro Trp Ala Asp Phe Thr Leu Leu Gly Ala Arg
            180                 185                 190

Ala Pro Gly Thr Tyr Ser Cys Tyr Tyr His Thr Pro Ser Ala Pro Tyr
        195                 200                 205

Val Leu Ser Gln Arg Ser Glu Val Leu Val Ile Ser Trp Glu Gly Glu
    210                 215                 220

Gly Pro Glu Ala Arg Pro Ala Ser Ser Ala Pro Gly Met Gln Ala Pro
225                 230                 235                 240

Gly Pro Pro Pro Ser Asp Pro Gly Ala Gln Ala Pro Ser Leu Ser Ser
                245                 250                 255

Phe Arg Pro Arg Gly Leu Val Leu Gln Pro Leu Leu Pro Gln Thr Gln
            260                 265                 270

Asp Ser Trp Asp Pro Ala Pro Pro Pro Ser Asp Pro Gly Val
        275                 280                 285

<210>  2
<211>  459
<212>  PRT
<213>  Artificial Sequence

<220>
<223>  Oscar-Fc fusion

<400>  2

Met Ala Leu Val Leu Ile Leu Gln Leu Leu Thr Leu Trp Pro Leu Cys
1               5                   10                  15

His Thr Asp Ile Thr Pro Ser Val Ala Ile Ile Val Pro Pro Ala Ser
            20                  25                  30

Tyr His Pro Lys Pro Trp Leu Gly Ala Gln Pro Ala Thr Val Val Thr
        35                  40                  45

Pro Gly Val Asn Val Thr Leu Arg Cys Arg Ala Pro Gln Pro Ala Trp
    50                  55                  60

Arg Phe Gly Leu Phe Lys Pro Gly Glu Ile Ala Pro Leu Leu Phe Arg
65                  70                  75                  80

```
Asp Val Ser Ser Glu Leu Ala Glu Phe Phe Leu Glu Glu Val Thr Pro
                85                  90                  95

Ala Gln Gly Gly Ser Tyr Arg Cys Cys Tyr Arg Arg Pro Asp Trp Gly
            100                 105                 110

Pro Gly Val Trp Ser Gln Pro Ser Asp Val Leu Glu Leu Leu Val Thr
        115                 120                 125

Glu Glu Leu Pro Arg Pro Ser Leu Val Ala Leu Pro Gly Pro Val Val
    130                 135                 140

Gly Pro Gly Ala Asn Val Ser Leu Arg Cys Ala Gly Arg Leu Arg Asn
145                 150                 155                 160

Met Ser Phe Val Leu Tyr Arg Glu Gly Val Ala Ala Pro Leu Gln Tyr
                165                 170                 175

Arg His Ser Ala Gln Pro Trp Ala Asp Phe Thr Leu Leu Gly Ala Arg
            180                 185                 190

Ala Pro Gly Thr Tyr Ser Cys Tyr Tyr His Thr Pro Ser Ala Pro Tyr
        195                 200                 205

Val Leu Ser Gln Arg Ser Glu Val Leu Val Ile Ser Trp Glu Gly Glu
    210                 215                 220

Gly Pro Glu Ala Arg Pro Ala Ser Asp Lys Thr His Thr Cys Pro Pro
225                 230                 235                 240

Cys Pro Ala Pro Glu Leu Leu Gly Gly Pro Ser Val Phe Leu Phe Pro
                245                 250                 255

Pro Lys Pro Lys Asp Thr Leu Met Ile Ser Arg Thr Pro Glu Val Thr
            260                 265                 270

Cys Val Val Val Asp Val Ser His Glu Asp Pro Glu Val Lys Phe Asn
        275                 280                 285

Trp Tyr Val Asp Gly Val Glu Val His Asn Ala Lys Thr Lys Pro Arg
    290                 295                 300

Glu Glu Gln Tyr Asn Ser Thr Tyr Arg Val Val Ser Val Leu Thr Val
305                 310                 315                 320

Leu His Gln Asp Trp Leu Asn Gly Lys Glu Tyr Lys Cys Lys Val Ser
                325                 330                 335
```

```
Asn Lys Ala Leu Pro Ala Pro Ile Glu Lys Thr Ile Ser Lys Ala Lys
            340                 345                 350

Gly Gln Pro Arg Glu Pro Gln Val Tyr Thr Leu Pro Pro Ser Arg Glu
        355                 360                 365

Glu Met Thr Lys Asn Gln Val Ser Leu Thr Cys Leu Val Lys Gly Phe
    370                 375                 380

Tyr Pro Ser Asp Ile Ala Val Glu Trp Glu Ser Asn Gly Gln Pro Glu
385                 390                 395                 400

Asn Asn Tyr Lys Thr Thr Pro Pro Val Leu Asp Ser Asp Gly Ser Phe
                405                 410                 415

Phe Leu Tyr Ser Lys Leu Thr Val Asp Lys Ser Arg Trp Gln Gln Gly
            420                 425                 430

Asn Val Phe Ser Cys Ser Val Met His Glu Ala Leu His Asn His Tyr
        435                 440                 445

Thr Gln Lys Ser Leu Ser Leu Ser Pro Gly Lys
    450                 455


<210>  3
<211>  271
<212>  PRT
<213>  Mus musculus

<400>  3

Met Val Leu Ser Leu Ile Leu Gln Leu Ser Thr Leu Cys Glu Leu Ser
1               5                   10                  15

Leu Pro Trp Pro Ala Cys Arg Ala Asp Phe Thr Pro Thr Ala Pro Leu
            20                  25                  30

Ala Ser Tyr Pro Gln Pro Trp Leu Gly Ala His Pro Ala Ala Val Val
        35                  40                  45

Thr Pro Gly Ile Asn Val Thr Leu Thr Cys Arg Ala Pro Gln Ser Ala
    50                  55                  60

Trp Arg Phe Ala Leu Phe Lys Ser Gly Leu Val Thr Pro Leu Leu Leu
65                  70                  75                  80

Arg Asp Val Ser Val Glu Leu Ala Glu Phe Phe Leu Glu Glu Val Thr
                85                  90                  95
```

```
Pro Ala Gln Gly Gly Ser Tyr His Cys Arg Tyr Arg Lys Thr Asp Trp
            100                 105                 110

Gly Pro Gly Val Trp Ser Gln Pro Ser Asn Val Leu Glu Leu Leu Val
        115                 120                 125

Thr Asp Gln Leu Pro Arg Pro Ser Leu Val Ala Leu Pro Gly Pro Val
    130                 135                 140

Val Ala Pro Gly Ala Asn Val Ser Leu Arg Cys Ala Gly Arg Ile Pro
145                 150                 155                 160

Gly Met Ser Phe Ala Leu Tyr Arg Val Gly Val Ala Thr Pro Leu Gln
                165                 170                 175

Tyr Ile Asp Ser Val Gln Pro Trp Ala Asp Phe Leu Leu Ile Gly Thr
            180                 185                 190

His Thr Pro Gly Thr Tyr Cys Cys Tyr Tyr His Thr Pro Ser Ala Pro
        195                 200                 205

Tyr Val Leu Ser Gln Arg Ser Gln Pro Leu Val Ile Ser Phe Glu Gly
    210                 215                 220

Ser Gly Ser Leu Asp Tyr Thr Gln Gly Asn Leu Ile Arg Leu Gly Leu
225                 230                 235                 240

Ala Gly Met Val Leu Ile Cys Leu Gly Ile Ile Val Thr Cys Asp Trp
                245                 250                 255

His Ser Arg Ser Ser Ala Phe Asp Gly Leu Leu Pro Gln Gln Asn
            260                 265                 270


<210>  4
<211>  462
<212>  PRT
<213>  Artificial Sequence

<220>
<223>  Oscar-Fc fusion

<400>  4

Met Val Leu Ser Leu Ile Leu Gln Leu Ser Thr Leu Cys Glu Leu Ser
1               5                   10                  15

Leu Pro Trp Pro Ala Cys Arg Ala Asp Phe Thr Pro Thr Ala Pro Leu
            20                  25                  30
```

```
Ala Ser Tyr Pro Gln Pro Trp Leu Gly Ala His Pro Ala Ala Val Val
        35                  40                  45

Thr Pro Gly Ile Asn Val Thr Leu Thr Cys Arg Ala Pro Gln Ser Ala
    50                  55                  60

Trp Arg Phe Ala Leu Phe Lys Ser Gly Leu Val Thr Pro Leu Leu Leu
65                  70                  75                  80

Arg Asp Val Ser Val Glu Leu Ala Glu Phe Phe Leu Glu Glu Val Thr
                85                  90                  95

Pro Ala Gln Gly Gly Ser Tyr His Cys Arg Tyr Arg Lys Thr Asp Trp
            100                 105                 110

Gly Pro Gly Val Trp Ser Gln Pro Ser Asn Val Leu Glu Leu Leu Val
        115                 120                 125

Thr Asp Gln Leu Pro Arg Pro Ser Leu Val Ala Leu Pro Gly Pro Val
    130                 135                 140

Val Ala Pro Gly Ala Asn Val Ser Leu Arg Cys Ala Gly Arg Ile Pro
145                 150                 155                 160

Gly Met Ser Phe Ala Leu Tyr Arg Val Gly Val Ala Thr Pro Leu Gln
                165                 170                 175

Tyr Ile Asp Ser Val Gln Pro Trp Ala Asp Phe Leu Leu Ile Gly Thr
            180                 185                 190

His Thr Pro Gly Thr Tyr Cys Cys Tyr Tyr His Thr Pro Ser Ala Pro
        195                 200                 205

Tyr Val Leu Ser Gln Arg Ser Gln Pro Leu Val Ile Ser Phe Glu Gly
    210                 215                 220

Ser Gly Ser Leu Asp Tyr Thr Gln Gly Asn Leu Asp Lys Thr His Thr
225                 230                 235                 240

Cys Pro Pro Cys Pro Ala Pro Glu Leu Leu Gly Gly Pro Ser Val Phe
                245                 250                 255

Leu Phe Pro Pro Lys Pro Lys Asp Thr Leu Met Ile Ser Arg Thr Pro
            260                 265                 270

Glu Val Thr Cys Val Val Val Asp Val Ser His Glu Asp Pro Glu Val
        275                 280                 285
```

Lys Phe Asn Trp Tyr Val Asp Gly Val Glu Val His Asn Ala Lys Thr
    290                 295                 300

Lys Pro Arg Glu Glu Gln Tyr Asn Ser Thr Tyr Arg Val Val Ser Val
305                 310                 315                 320

Leu Thr Val Leu His Gln Asp Trp Leu Asn Gly Lys Glu Tyr Lys Cys
                325                 330                 335

Lys Val Ser Asn Lys Ala Leu Pro Ala Pro Ile Glu Lys Thr Ile Ser
            340                 345                 350

Lys Ala Lys Gly Gln Pro Arg Glu Pro Gln Val Tyr Thr Leu Pro Pro
        355                 360                 365

Ser Arg Glu Glu Met Thr Lys Asn Gln Val Ser Leu Thr Cys Leu Val
    370                 375                 380

Lys Gly Phe Tyr Pro Ser Asp Ile Ala Val Glu Trp Glu Ser Asn Gly
385                 390                 395                 400

Gln Pro Glu Asn Asn Tyr Lys Thr Thr Pro Pro Val Leu Asp Ser Asp
                405                 410                 415

Gly Ser Phe Phe Leu Tyr Ser Lys Leu Thr Val Asp Lys Ser Arg Trp
            420                 425                 430

Gln Gln Gly Asn Val Phe Ser Cys Ser Val Met His Glu Ala Leu His
        435                 440                 445

Asn His Tyr Thr Gln Lys Ser Leu Ser Leu Ser Pro Gly Lys
    450                 455                 460

<210> 5
<211> 20
<212> DNA
<213> Mus musculus

<400> 5
acagctggag tatcagcgac 20

<210> 6
<211> 20
<212> DNA
<213> Mus musculus

<400> 6
gctcacagag agtcgacagc 20

<210> 7
<211> 146
<212> DNA
<213> Artificial Sequence

<220>
<223> ssODN

<400> 7
gccaaggatc cacacacagg ggagggacag ctcacagaga gtcgacagct ggagtatcag 60

cctcagaaga actcgtcaag aagtcacgac aggaccatgg tgggcactct ccgtggagct 120

gaggaaaagg ttgaccctgc cttttt 146

<210> 8
<211> 146
<212> DNA
<213> Artificial Sequence

<220>
<223> ssODN

<400> 8
atagccctca gcccagccaa ggatccacac acaggggagg gacagctcac agagagtcga 60

cctcagaaga actcgtcaag aagtcacagc tggagtatca gcgacaggac catggtgggc 120

actctccgtg gagctgagga aaaggt 146

<210> 9
<211> 20
<212> DNA
<213> Artificial Sequence

<220>
<223> primer

<400> 9
ctacttagcg acaacgtcct 20

<210> 10
<211> 20
<212> DNA
<213> Artificial Sequence

<220>
<223> primer

<400> 10
gccttggggt ttgaaggttt 20

<210> 11
<211> 20
<212> DNA
<213> Artificial Sequence

<220>

<223> primer

<400> 11
cagaggctat gactgttcca 20

<210> 12
<211> 20
<212> DNA
<213> Artificial Sequence

<220>
<223> primer

<400> 12
ggcagggtca accttttcct 20

<210> 13
<211> 20
<212> DNA
<213> Artificial Sequence

<220>
<223> primer

<400> 13
agggacagct cacagagagt 20

<210> 14
<211> 20
<212> DNA
<213> Artificial Sequence

<220>
<223> primer

<400> 14
gctcggtgac cctggtcttt 20

<210> 15
<211> 20
<212> DNA
<213> Artificial Sequence

<220>
<223> primer

<400> 15
aatgtgaggc gggtggaact 20

<210> 16
<211> 20
<212> DNA
<213> Artificial Sequence

<220>
<223> primer

<400> 16

cgggcatgag ttttgcactg 20

<210> 17
<211> 21
<212> DNA
<213> Artificial Sequence

<220>
<223> primer

<400> 17
tgggtatagt ccaaggagcc a 21

<210> 18
<211> 20
<212> DNA
<213> Artificial Sequence

<220>
<223> primer

<400> 18
aggagccatg actcgaaggt 20

<210> 19
<211> 21
<212> DNA
<213> Artificial Sequence

<220>
<223> primer

<400> 19
gctcaccagg tagtgatgct t 21

<210> 20
<211> 1601
<212> DNA
<213> Artificial Sequence

<220>
<223> Oscar-Fc

<400> 20
aagcttgcca ccatggccct cgtgcttatc ctccaacttc tcacgctttg gcctctgtgc 60

cacaccgaca ttactccgtc tgttgcgata attgtccctc ccgcctctta tcaccctaaa 120

ccttggctgg gcgcacagcc agctactgtg gttactcctg gggtgaacgt aacactgcgc 180

tgccgtgctc ctcagcccgc ctggagattt gggttgttta agcccggaga gatagcacca 240

ctgctgtttc gggatgtgtc ctcagagctg gctgagttct tcctggaaga ggtcactcct 300

gcccaaggag gcagctatcg gtgctgttat aggcggccgg attggggacc cggcgtttgg 360

tcccaaccat ctgatgtgct cgaactgctt gtgacagaag agctgcccag acctagcttg 420

gtagccttgc ccggtcctgt cgtcggacct ggtgccaatg tttctcttcg atgtgccgga 480

aggctgcgca atatgtcctt tgtactgtat agggagggag tagccgcacc tctgcagtat 540

aggcatagcg ctcagccctg ggcggatttt actctgcttg gtgccagagc acccgggacc 600

tattcctgct actaccacac tccttccgca ccctacgtcc tgtcacagag atcagaagtg 660

ctcgtgatct cctgggaggg agaaggccca gaagccgaca aaactcacac atgcccaccg 720

tgcccaggta agccagccca ggcctcgccc tccagctcaa ggcgggacag gtgccctaga 780

gtagcctgca tccagggaca ggccccagcc gggtgctgac acgtccacct ccatctcttc 840

ctcagcacct gaactcctgg ggggaccgtc agtcttcctc ttccccccaa aacccaagga 900

caccctcatg atctcccgga cccctgaggt cacatgcgtg gtggtggacg tgagccacga 960

agaccctgag gtcaagttca actggtacgt ggacggcgtg gaggtgcata atgccaagac 1020

aaagccgcgg gaggagcagt acaacagcac gtaccgtgtg gtcagcgtcc tcaccgtcct 1080

gcaccaggac tggctgaatg gcaaggagta caagtgcaag gtctccaaca aagccctccc 1140

agcccccatc gagaaaacca tctccaaagc caaaggtggg acccgtgggg tgcgagggcc 1200

acatggacag aggccggctc ggcccaccct ctgccctgag agtgactgct gtaccaacct 1260

ctgtccctac agggcagccc cgagaaccac aggtgtacac cctgccccca tcccgggatg 1320

agctgaccaa gaaccaggtc agcctgacct gcctggtcaa aggcttctat cccagcgaca 1380

tcgccgtgga gtgggagagc aatgggcagc cggagaacaa ctacaagacc acgcctcccg 1440

tgctggactc cgacggctcc ttcttcctct acagcaagct caccgtggac aagagcaggt 1500

ggcagcaggg gaacgtcttc tcatgctccg tgatgcatga ggctctgcac aaccactaca 1560

cgcagaagag cctctccctg tctccgggta aatgatctag a 1601

<210> 21
<211> 21
<212> DNA
<213> Artificial Sequence

<220>
<223> primer

<400> 21
aagaccttga gtagttgccc a 21

<210> 22
<211> 21
<212> DNA
<213> Artificial Sequence

<220>
<223> primer

<400> 22
tgctcgatcc tacgtttgca g 21

<210> 23
<211> 20
<212> DNA
<213> Artificial Sequence

<220>
<223> primer

<400> 23
aggagccatg actcgaaggt 20

<210> 24
<211> 21
<212> DNA
<213> Artificial Sequence

<220>
<223> primer

<400> 24
gctcaccagg tagtgatgct t 21

<210> 25
<211> 20
<212> DNA
<213> Artificial Sequence

<220>
<223> primer

<400> 25
accccagcat ggaaacaaag 20

<210> 26
<211> 25
<212> DNA
<213> Artificial Sequence

<220>
<223> primer

<400> 26
aagaatggta ttgaagtcat ctgtc 25

<210> 27
<211> 21
<212> DNA
<213> Artificial Sequence

<220>
<223> primer

<400> 27
accccgtgaa gaaaatcaga a 21

<210> 28
<211> 20

<212>  DNA
<213>  Artificial Sequence

<220>
<223>  primer

<400>  28
taacaggcgg tcttggttgg                                                    20

<210>  29
<211>  21
<212>  DNA
<213>  Artificial Sequence

<220>
<223>  primer

<400>  29
tggtggtcct gtttacagtg g                                                  21

<210>  30
<211>  21
<212>  DNA
<213>  Artificial Sequence

<220>
<223>  primer

<400>  30
ttctgaagtt cttcacgggg t                                                  21

<210>  31
<211>  24
<212>  DNA
<213>  Artificial Sequence

<220>
<223>  primer

<400>  31
cctacgaaga ctcaaattct cagc                                               24

<210>  32
<211>  19
<212>  DNA
<213>  Artificial Sequence

<220>
<223>  primer

<400>  32
gaggaccatg gtggtgtcc                                                     19

<210>  33
<211>  20
<212>  DNA
<213>  Artificial Sequence

```
<220>
<223>  primer

<400>  33
gctcggtgac cctggtcttt                                                   20


<210>  34
<211>  20
<212>  DNA
<213>  Artificial Sequence

<220>
<223>  primer

<400>  34
aatgtgaggc gggtggaact                                                   20


<210>  35
<211>  20
<212>  DNA
<213>  Artificial Sequence

<220>
<223>  primer

<400>  35
ctccatcgga gaaggacagc                                                   20


<210>  36
<211>  22
<212>  DNA
<213>  Artificial Sequence

<220>
<223>  primer

<400>  36
aggtcctgaa agtccattgt cc                                                22
```